# EUROPEAN PATENT APPLICATION

(11) **EP 1 721 977 A2**
(43) Date of publication of application: **15.11.2006**
(21) Application number: 06007721.1
(22) Date of filing: 17.09.2002
(51) Int. Cl.: C12N 15/12, C12N 15/62, C12N 15/70, C12N 15/85, C12N 5/10, C12N 1/20, C07K 14/47, C07K 16/18, C12Q 1/68, G01N 33/574

(54) **Methods of diagnosis of cancer, compositions and methods of screening for modulators of cancer**

(30) Priority: 17.09.2001 US 323469 P; 20.09.2001 US 323887 P; 13.11.2001 US 350666 P; 08.02.2002 US 355145 P; 08.02.2002 US 355257 P; 12.04.2002 US 372246 P
(62) Divisional of application: 02766297.2
(71) Applicant: PDL BioPharma, Inc., Fremont, CA 94555 (US)
(72) Inventor: Afar, Daniel, Fremont, CA 94555 (US); Aziz, Natasha, Berkeley, CA 94708 (US); Gish, Kurt C., Piedmont, CA 94611 (US); Hevezi, Peter, Encinitas, CA 92024 (US); Mack, David H., San Francisco, CA 94111 (US); Wilson, Keith E., Belmont, CA 94002 (US); Zlotnik, Albert, San Diego, CA 91230 (US)
(74) Representative: Schüssler, Andrea

(57) **Abstract**

Described herein are genes whose expression are up-regualted or down-regulated in specific cancers. Related methods and compositions that can be used for diagnosis and treatment are disclosed. Also described herein are methods that can be used to identify modulators of selected cancers.

### Annex to the application documents - subsequently filed sequences listing

SEQUENCE LISTING

<110> Afar, Daniel
      Aziz, Natasha
      Gish, Kurt C.
      Hevezi, Peter A.
      Mack, David H.
      Wilson, Keith E.
      Zlotnik, Albert
      Eos Biotechnology, Inc.

<120> Methods of Diagnosis of Cancer, Compositions and
  Methods of Screening for Modulators of Cancer

<130> P 3178EP

<140> EP06007721.1
<141> 2002-09-17

<150> US 60/323,469
<151> 2001-09-17

<160> 412

<170> FastSEQ for Windows Version 3.0

<210> 1
<211> 1410
<212> DNA
<213> Homo sapiens

<400> 1
atgcacagct ttcctccact gctgctgctg ctgttctggg gtgtggtgtc acacagcttc      60
ccagcgactc tagaaacaca agagcaagat gtggacttag tccagaaata cctggaaaaa     120
tactacaacc tgaagaatga tgggaggcaa gttgaaaagc ggagaaatag tggcccagtg     180
gttgaaaaat tgaagcaaat gcaggaattc tttgggctga aagtgactgg gaaaccagat     240
gctgaaaccc tgaaggtgat gaagcagccc agatgtggag tgcctgatgt ggctcagttt     300
gtcctcactg aggggaaccc tcgctgggag caaacacatc tgacctacag gattgaaaat     360
tacacgccag atttgccaag agcagatgtg gaccatgcca ttgagaaagc cttccaactc     420
tggagtaatg tcacacctct gacattcacc aaggtctctg agggtcaagc agacatcatg     480
atatcttttg tcaggggaga tcatcgggac aactctcctt ttgatggacc tggaggaaat     540
cttgctcatg cttttcaacc aggcccaggt attggagggg atgctcattt tgatgaagat     600
gaaaggtgga ccaacaattt cagagagtac aacttacatc gtgttgcggc tcatgaactc     660
ggccattctc ttggactctc ccattctact gatatcgggg ctttgatgta ccctagctac     720
accttcagtg gtgatgttca gctagctcag gatgacattg atggcatcca agccatatat     780
ggacgttccc aaaatcctgt ccagcccatc ggcccacaaa ccccaaaagc atgtgacagt     840
aagctaacct ttgatgctat aactacgatt cggggagaag tgatgttctt taaagacaga     900
ttctacatgc gcacaaatcc cttctacccg gaagttgagc tcaatttcat ttctgttttc     960
tggccacaac tgccaaatgg gcttgaagct gcttacgaat ttgccgacag agatgaagtc    1020
cggtttttca aagggaataa gtactgggct gttcagggac agaatgtgct acacggatac    1080
cccaaggaca tctacagctc ctttggcttc cctagaactg tgaagcatat cgatgctgct    1140
ctttctgagg aaaacactgg aaaaacctac ttctttgttg ctaacaaata ctggaggtat    1200
gatgaatata aacgatctat ggatccaggt tatcccaaaa tgatagcaca tgactttcct    1260
ggaattggcc acaaagttga tgcagttttc atgaaagatg gatttttcta tttctttcat    1320
ggaacaagac aatacaaatt tgatcctaaa acgaagagaa ttttgactct ccagaaagct    1380
aatagctggt tcaactgcag gaaaaattag                                    1410

<210> 2
<211> 1410
<212> DNA
<213> Homo sapiens
<400> 2
atgcacagct ttcctccact gctgctgctg ctgttctggg gtgtggtgtc acacagcttc      60

ccagcgactc tagaaacaca agagcaagat gtggacttag tccagaaata cctggaaaaa 120
tactacaacc tgaagaatga tgggaggcaa gttgaaaagc ggagaaatag tggcccagtg 180
gttgaaaaat tgaagcaaat gcaggaattc tttgggctga aagtgactgg gaaaccagat 240
gctgaaaccc tgaaggtgat gaagcagccc agatgtggag tgcctgatgt ggctcagttt 300
gtcctcactg aggggaaccc tcgctgggag caaacacatc tgacctacag gattgaaaat 360
tacacgccag atttgccaag agcagatgtg gaccatgcca ttgagaaagc cttccaactc 420
tggagtaatg tcacacctct gacattcacc aaggtctctg agggtcaagc agacatcatg 480
atatcttttg tcaggggaga tcatcgggac aactctcctt ttgatggacc tggaggaaat 540
cttgctcatg cttttcaacc aggcccaggt attggagggg atgctcattt tgatgaagat 600
gaaaggtgga ccaacaattt cagagagtac aacttacatc gtgttgcggc tcatgccctc 660
ggccattctc ttggactctc ccattctact gatatcgggg ctttgatgta ccctagctac 720
accttcagtg gtgatgttca gctagctcag gatgacattg atggcatcca agccatatat 780
ggacgttccc aaaatcctgt ccagcccatc ggcccacaaa ccccaaaagc atgtgacagt 840
aagctaacct ttgatgctat aactacgatt cggggagaag tgatgttctt taaagacaga 900
ttctacatgc gcacaaatcc cttctacccg gaagttgagc tcaatttcat ttctgttttc 960
tggccacaac tgccaaatgg gcttgaagct gcttacgaat ttgccgacag agatgaagtc 1020
cggtttttca aagggaataa gtactgggct gttcagggac agaatgtgct acacggatac 1080
cccaaggaca tctacagctc ctttggcttc cctagaactg tgaagcatat cgatgctgct 1140

ctttctgagg aaaacactgg aaaaacctac ttctttgttg ctaacaaata ctggaggtat 1200
gatgaatata aacgatctat ggatccaggt tatcccaaaa tgatagcaca tgactttcct 1260
ggaattggcc acaaagttga tgcagttttc atgaaagatg gatttttcta tttctttcat 1320
ggaacaagac aatacaaatt tgatcctaaa acgaagagaa ttttgactct ccagaaagct 1380
aatagctggt tcaactgcag gaaaaattag 1410

<210> 3
<211> 1821
<212> DNA
<213> Homo sapiens

<400> 3
acaaggaggc aggcaagaca gcaaggcata gagacaacat agagctaagt aaagccagtg 60
gaaatgaaga gtcttccaat cctactgttg ctgtgcgtgg cagtttgctc agcctatcca 120
ttggatggag ctgcaagggg tgaggacacc agcatgaacc ttgttcagaa atatctagaa 180
aactactacg acctcaaaaa agatgtgaaa cagtttgtta ggagaaagga cagtggtcct 240
gttgttaaaa aaatccgaga aatgcagaag ttccttggat tggaggtgac ggggaagctg 300
gactccgaca ctctggaggt gatgcgcaag cccaggtgtg gagttcctga tgttggtcac 360
ttcagaacct ttcctggcat cccgaagtgg aggaaaaccc accttacata caggattgtg 420
aattatacac cagatttgcc aaaagatgct gttgattctg ctgttgagaa agctctgaaa 480
gtctgggaag aggtgactcc actcacattc tccaggctgt atgaaggaga ggctgatata 540
atgatctctt ttgcagttag agaacatgga gacttttacc cttttgatgg acctggaaat 600
gttttggccc atgcctatgc ccctgggcca gggattaatg gagatgccca ctttgatgat 660
gatgaacaat ggacaaagga tacaacaggg accaatttat ttctcgttgc tgctcatgaa 720
attggccact ccctgggtct ctttcactca gccaacactg aagctttgat gtacccactc 780
tatcactcac tcacagacct gactcggttc cgcctgtctc aagatgatat aaatggcatt 840
cagtccctct atggacctcc ccctgactcc cctgagaccc ccctggtacc cacggaacct 900
gtccctccag aacctgggac gccagccaac tgtgatcctg ctttgtcctt tgatgctgtc 960
agcactctga ggggagaaat cctgatcttt aaagacaggc acttttggcg caaatccctc 1020
aggaagcttg aacctgaatt gcatttgatc tcttcatttt ggccatctct tccttcaggc 1080
gtggatgccg catatgaagt tactagcaag gacctcgttt tcatttttaa aggaaatcaa 1140
ttctgggcca tcagaggaaa tgaggtacga gctggatacc caagaggcat ccacacccta 1200
ggtttccctc caaccgtgag gaaaatcgat gcagccattt ctgataagga aaagaacaaa 1260
acatatttct ttgtagagga caaatactgg agatttgatg agaagagaaa ttccatggag 1320
ccaggctttc ccaagcaaat agctgaagac tttccaggga ttgactcaaa gattgatgct 1380
gtttttgaag aatttgggtt cttttatttc tttactggat cttcacagtt ggagtttgac 1440

ccaaatgcaa agaaagtgac acacactttg aagagtaaca gctggcttaa ttgttgaaag 1500
agatatgtag aaggcacaat atgggcactt taaatgaagc taataattct tcacctaagt 1560
ctctgtgaat tgaaatgttc gttttctcct gcctgtgctg tgactcgagt cacactcaag 1620
ggaacttgag cgtgaatctg tatcttgccg gtcattttta tgttattaca gggcattcaa 1680
atgggctgct gcttagcttg caccttgtca catagagtga tctttcccaa gagaagggga 1740
agcactcgtg tgcaacagac aagtgactgt atctgtgtag actatttgct tatttaataa 1800
agacgatttg tcagttgttt t 1821

<210> 4
<211> 6158
<212> DNA
<213> Homo sapiens

<220>
<221> modified_base
<222> (1)...(6158)
<223> n = g, a, c or t

<400> 4
aaccatcaaa tttagaagaa aaagcccttt gactttttcc ccctctccct ccccaatggc 60
tgtgtagcaa acatccctgg cgataccttg gaaaggacga agttggtctg cagtcgcaat 120
ttcgtgggtt gagttcacag ttgtgagtgc ggggctcgga gatggagccg tggtcctcta 180
ggtggaaaac gaaacggtgg ctctgggatt tcaccgtaac aaccctcgca ttgaccttcc 240
tcttccaagc tagagaggtc agaggagctg ctccagttga tgtactaaaa gcactagatt 300
ttcacaattc tccagaggga atatcaaaaa caacgggatt ttgcacaaac agaaagaatt 360
ctaaaggctc agatactgct tacagagttt caaagcaagc acaactcagt gccccaacaa 420
aacagttatt tccaggtgga actttcccag aagacttttc aatactattt acagtaaaac 480
caaaaaaagg aattcagtct ttccttttat ctatatataa tgagcatggt attcagcaaa 540
ttggtgttga ggttgggaga tcacctgttt ttctgtttga agaccacact ggaaaacctg 600
ccccagaaga ctatcccctc ttcagaactg ttaacatcgc tgacgggaag tggcatcggg 660
tagcaatcag cgtggagaag aaaactgtga caatgattgt tgattgtaag aagaaaacca 720
cgaaaccact tgatagaagt gagagagcaa ttgttgatac caatggaatc acggtttttg 780
gaacaaggat tttggatgaa gaagtttttg agggggacat tcagcagttt ttgatcacag 840
gtgatcccaa ggcagcatat gactactgtg agcattatag tccagactgt gactcttcag 900
cacccaaggc tgctcaagct caggaacctc agatagatga gtatgcacca gaggatataa 960
tcgaatatga ctatgagtat ggggaagcag agtataaaga ggctgaaagt gtaacagagg 1020
gacccactgt aactgaggag acaatagcac agacggaggc aaacatcgtt gatgattttc 1080
aagaatacaa ctatggaaca atggaaagtt accagacaga agctcctagg catgtttctg 1140
ggacaaatga gccaaatcca gttgaagaaa tatttactga agaatatcta acgggagagg 1200
attatgattc ccagaggaaa aattctgagg atacactata tgaaaacaaa gaaatagacg 1260
gcagggattc tgatcttctg gtagatggag atttaggcga atatgatttt tatgaatata 1320
aagaatatga agataaacca acaagccccc ctaatgaaga atttggtcca ggtgtaccag 1380
cagaaactga tattacagaa acaagcataa atggccatgg tgcatatgga gagaaaggac 1440
agaaaggaga accagcagtg gttgagcctg gtatgcttgt cgaaggacca ccaggaccag 1500
caggacctgc aggtattatg ggtcctccag gtctacaagg ccccactgga ccccctggtg 1560
accctggcga taggggcccc ccaggacgtc ctggcttacc aggggctgat ggtctacctg 1620
gtcctcctgg tactatgttg atgttaccgt tccgttatgg tggtgatggt tccaaaggac 1680
caaccatctc tgctcaggaa gctcaggctc aagctattct tcagcaggct cggattgctc 1740
tgagaggccc acctggccca atgggtctaa ctggaagacc aggtcctgtg ggggggcctg 1800
gttcatctgg ggccaaaggt gagagtggtg atccaggtcc tcagggccct cgaggcgtcc 1860
agggtccccc tggtccaacg ggaaaacctg gaaaaagggg tcgtccaggt gcagatggag 1920
gaagaggaat gccaggagaa cctggggcaa agggagatcg agggtttgat ggacttccgg 1980
gtctgccagg tgacaaaggt cacaggggtg aacgaggtcc tcaaggtcct ccaggtcctc 2040
ctggtgatga tggaatgagg ggagaagatg gagaaattgg accaagaggt cttccaggtg 2100
aagctggccc acgaggtttg ctgggtccaa ggggaactcc aggagctcca gggcagcctg 2160
gtatggcagg tgtagatggc cccccaggac caaaagggaa catgggtccc caaggggagc 2220
ctgggcctcc aggtcaacaa gggaatccag gacctcaggg tcttcctggt ccacaaggtc 2280
caattggtcc tcctggtgaa aaaggaccac aaggaaaacc aggacttgct ggacttcctg 2340
gtgctgatgg gcctcctggt catcctggga aagaaggcca gtctggagaa aagggggctc 2400
tgggtccccc tggtccacaa ggtcctattg gatnnccggg cccccgggga gtaaagggag 2460
cagatggtgt cagaggtctc aagggatcta aaggtgaaaa gggtgaagat ggttttccag 2520
gattcaaagg tgacatgggt ctaaaaggtg acagaggaga agttggtcaa attggcccaa 2580
gagggnaaga tggccctgaa ggacccaaag gtcgagcagg cccaactgga gacccaggtc 2640
cttcaggtca agcaggagaa aagggaaaac ttggagttcc aggattacca ggatatccag 2700
gaagacaagg tccaaagggt tccactggat tccctgggtt tccaggtgcc aatggagaga 2760
aaggtgcacg gggagtagct ggcaaaccag gccctcgggg tcagcgtggt ccaacgggtc 2820
ctcgaggttc aagaggtgca agaggtccca ctgggaaacc tgggccaaag ggcacttcag 2880
gtggcgatgg ccctcctggc cctccaggtg aaagaggtcc tcaaggacct cagggtccag 2940
ttggattccc tggaccaaaa ggccctcctg gaccaccagg aaggatgggc tgcccaggac 3000
accctgggca acgtggggag actggatttc aaggcaagac cggccctcct gggccagggg 3060

gagtggttgg accacaggga ccaaccggtg agactggtcc aataggggaa cgtgggtatc 3120
ctggtcctcc tggccctcct ggtgagcaag gtcttcctgg tgctgcagga aaagaaggtg 3180
caaagggtga tccaggtcct caaggtatct cagggaaaga tggaccagca ggattacgtg 3240
gtttcccagg ggaaagaggt cttcctggag ctcagggtgc acctggactg aaaggagggg 3300
aaggtcccca gggcccacca ggtccagttg gctcaccagg agaacgtggg tcagcaggta 3360
cagctggccc aattggttta cgagggcgcc cgggacctca gggtcctcct ggtccagctg 3420
gagagaaagg tgctcctgga gaaaaaggtc cccaagggcc tgcagggaga gatggagttc 3480
aaggtcctgt tggtctccca gggccagctg gtcctgccgg ctcccctggg gaagacggag 3540
acaagggtga aattggtgag ccgggacaaa aaggcagcaa gggtggcaag ggagaaaatg 3600
gccctcccgg tcccccaggt cttcaaggac cagttggtgc ccctggaatt gctggaggtg 3660
atggtgaacc aggtcctaga ggacagcagg ggatgtttgg gcaaaaaggt gatgagggtg 3720
ccagaggctt ccctggacct cctggtccaa taggtcttca gggtctgcca ggcccacctg 3780
gtgaaaaagg tgaaaatggg gatgttggtc catgggggcc acctggtcct ccaggcccaa 3840
gaggccctca aggtcccaat ggagctgatg gaccacaagg acccccaggt tctgttggtt 3900
cagttggtgg tgttggagaa aagggtgaac ctggagaagc aggaaaccca gggcctcctg 3960
gggaagcagg tgtaggcggt cccaaaggag aaagaggaga gaaaggggaa gctggtccac 4020
ctggagctgc tggacctcca ggtgccaagg ggccgccagg tgatgatggc cctaagggta 4080
acccgggtcc tgttggtttt cctggagatc ctggtcctcc tggggaactt ggccctgcag 4140
gtcaagatgg tgttggtggt gacaagggtg aagatggaga tcctggtcaa ccgggtcctc 4200
ctggcccatc tggtgaggct ggcccaccag gtcctcctgg aaaacgaggt cctcctggag 4260
ctgcaggtgc agagggaaga caaggtgaaa aaggtgctaa gggggaagca ggtgcagaag 4320
gtcctcctgg aaaaaccggc ccagtcggtc ctcagggacc tgcaggaaag cctggtccag 4380
aaggtcttcg gggcatccct ggtcctgtgg gagaacaagg tctccctgga gctgcaggcc 4440
aagatggacc acctggtcct atgggacctc ctggcttacc tggtctcaaa ggtgaccctg 4500
gctccaaggg tgaaaaggga catcctggtt taattggcct gattggtcct ccaggagaac 4560
aaggggaaaa aggtgaccga gggctccctg gaactcaagg atctccagga gcaaaagggg 4620
atgggggaat tcctggtcct gctggtccct taggtccacc tggtcctcca ggcttaccag 4680
gtcctcaagg cccaaagggt aacaaaggct ctactggacc cgctggccag aaaggtgaca 4740
gtggtcttcc agggcctcct gggcctccag gtccacctgg tgaagtcatt cagcctttac 4800
caatcttgtc ctccaaaaaa acgagaagac atactgaagg catgcaagca gatgcagatg 4860
ataatattct tgattactcg gatggaatgg aagaaatatt tggttccctc aattccctga 4920
aacaagacat cgagcatatg aaatttccaa tgggtactca gaccaatcca gcccgaactt 4980
gtaaagacct gcaactcagc catcctgact tcccagatgg tgaatattgg attgatccta 5040
accaaggttg ctcaggagat tccttcaaag tttactgtaa tttcacatct ggtggtgaga 5100
cttgcattta tccagacaaa aaatctgagg gagtaagaat ttcatcatgg ccaaaggaga 5160
aaccaggaag ttggtttagt gaatttaaga ggggaaaact gctttcatac ttagatgttg 5220
aaggaaattc catcaatatg gtgcaaatga cattcctgaa acttctgact gcctctgctc 5280
ggcaaaattt cacctaccac tgtcatcagt cagcagcctg gtatgatgtg tcatcaggaa 5340
gttatgacaa agcacttcgc ttcctgggat caaatgatga ggagatgtcc tatgacaata 5400
atccttttat caaaacactg tatgatggtt gtacgtccag aaaaggctat gaaaaaactg 5460
tcattgaaat caatacacca aaaattgatc aagtacctat tgttgatgtc atgatcagtg 5520
actttggtga tcagaatcag aagttcggat ttgaagttgg tcctgtttgt tttcttggct 5580
aagattaaga caaagaacat atcaaatcaa cagaaaatgt accttggtgc caccaaccca 5640
ttttgtgcca catgcaagtt ttgaataagg atgtatggaa aacaacgctg catatacagg 5700
taccatttag gaaataccga tgcctttgtg ggggcagaat cacagacaaa agctttgaaa 5760
atcataaaga tataagttgg tgtggctaag atggaaacag ggctgattct tgattcccaa 5820
ttctcaactc tccttttcct atttgaattt ctttggtgct gtagaaaaca aaaaaagaaa 5880
aatatatatt cataaaaaat atggtgctca ttctcatcca tccaggatgt actaaaacag 5940
tgtgtttaat aaattgtaat tattttgtgt acagttctat actgttatct gtgtccattt 6000
ccaaaacttg cacgtgtccc tgaattccgc tgactctaat ttatgaggat gccgaactct 6060
gatggcaata atatatgtat tatgaaaatg aagttatgat ttccgatgac cctaagtccc 6120
tttctttggt taatgatgaa attcctttgt gtgtgttt 6158

<210> 5
<211> 1284
<212> DNA
<213> Homo sapiens
<400> 5
gggagggaga gaggcgcgcg ggtgaaaggc gcattgatgc agcctgcggc ggcctcggag 60
cgcggcggag ccagacgctg accacgttcc tctcctcggt ctcctccgcc tccagctccg 120
cgctgcccgg cagccgggag ccatgcgacc ccagggcccc gccgcctccc cgcagcggct 180
ccgcggcctc ctgctgctcc tgctgctgca gctgcccgcg ccgtcgagcg cctctgagat 240
ccccaagggg aagcaaaagg cgcagctccg gcagagggag gtggtggacc tgtataatgg 300

aatgtgctta caagggccag caggagtgcc tggtcgagac gggagccctg gggccaatgg 360
cattccgggt acacctggga tcccaggtcg ggatggattc aaaggagaaa agggggaatg 420
tctgagggaa agctttgagg agtcctggac acccaactac aagcagtgtt catggagttc 480
attgaattat ggcatagatc ttgggaaaat tgcggagtgt acatttacaa agatgcgttc 540
aaatagtgct ctaagagttt tgttcagtgg ctcacttcgg ctaaaatgca gaaatgcatg 600
ctgtcagcgt tggtatttca cattcaatgg agctgaatgt tcaggacctc ttcccattga 660
agctataatt tatttggacc aaggaagccc tgaaatgaat tcaacaatta atattcatcg 720

cacttcttct gtggaaggac tttgtgaagg aattggtgct ggattagtgg atgttgctat 780
ctgggttggc acttgttcag attacccaaa aggagatgct tctactggat ggaattcagt 840
ttctcgcatc attattgaag aactaccaaa ataaatgctt taattttcat ttgctacctc 900
tttttttatt atgccttgga atggttcact taaatgacat tttaaataag tttatgtata 960
catctgaatg aaaagcaaag ctaaatatgt ttacagacca aagtgtgatt tcacactgtt 1020
tttaaatcta gcattattca ttttgcttca atcaaaagtg gtttcaatat tttttttagt 1080
tggttagaat actttcttca tagtcacatt ctctcaacct ataatttgga atattgttgt 1140
ggtcttttgt tttttctctt agtatagcat ttttaaaaaa atataaaagc taccaatctt 1200
tgtacaattt gtaaatgtta agaatttttt ttatatctgt taaataaaaa ttatttccaa 1260
caaccttaaa aaaaaaaaaa aaaa 1284

<210> 6
<211> 2373
<212> DNA
<213> Homo sapiens

<220>
<221> modified_base
<222> (1)...(2373)
<223> n = g, a, c or t

<400> 6
caggccggac cacttgctat atgtaggcnn nnnnnnnnnn nnnnaaatac acttactgtg 60
ttctagaggc agccctttct tatgcagaaa atacaatacg cactgcatga gaagcttgag 120
agtggattct aatccaggtc tgtcgacctt ggatatcatg catgtgggaa ggtgggtgtg 180
gtgagaaaag ttttaaggca agagtagatg gccatgttca actttacaaa atttcttgga 240
aaactggcag tattttgaac tgcatcttct ttggtaccgg aacctgcaga aacagtgtga 300
gaaattaagt cctggttcac tgcgcagtag caaagatggt caaggccatg gaaaaagcag 360
aaatttacca agaaagctga tacccatgta tagttcccac tcatctcaaa tacatctgct 420
atctttttaa gctaagtcct agacatatcg gggataacat gggggttgat tagtgaccac 480
agttatcaga agcagagaaa tgtaattcca tattttattt gaaacttatt ccatatttta 540
attggatatt gagtgattgg gttatcaaac acccacaaac tttaattttg ttaaatttat 600
atggctttga aatagaagta taagttgcta ccattttttg ataacattga aagatagtat 660
tttaccatct ttaatcatct tggaaaatac aagtcctgtg aacaaccact ctttcaccta 720
gcagcatgag gccaaaagta aaggctttaa attataacat atgggattct tagtagtatg 780
tttttttctt gaaactcagt ggctctatcn aaccttacta tctcctcact ctttctctaa 840
gactaaactc taggctctta aaaatctgcc cacaccaatc ttagaagctc tgaaaagaat 900
ttgtctttaa atatctttta atagtaacat gtattttatg gaccaaattg acattttcga 960
ctattttttc caaaaaagtc aggtgaattt cagcacactg agttgggaat ttcttatccc 1020
agaagaccaa ccaatttcat atttatttaa gattgattcc atactccgtt ttcaaggaga 1080
atccctgcag tctccttaaa ggtagaacaa atactttcta tttttttttt caccattgtg 1140
ggattggact ttaagaggtg actctaaaaa aacagagaac aaatatgtct cagttgtatt 1200
aagcacggac ccatattatc atattcactt aaaaaaatga tttcctgtgc accttttggc 1260
aacttctctt ttcaatgtag ggaaaaactt agtcaccctg aaaacccaca aaataaataa 1320
aacttgtaga tgtgggcaga aggtttgggg gtggacattg tatgtgttta aattaaaccc 1380
tgtatcactg agaagctgtt gtatgggtca gagaaaatga atgcttagaa gctgttcaca 1440
tcttcaagag cagaagcaaa ccacatgtct cagctatatt attatttatt ttttatgcat 1500
aaagtgaatc atttcttctg tattaatttc caaagggttt taccctctat ttaaatgctt 1560
tgaaaaacag tgcattgaca atgggttgat atttttcttt aaaagaaaaa tataattatg 1620
aaagccaaga taatctgaag cctgttttat tttaaaactt tttatgttct gtggttgatg 1680
ttgtttgttt gtttgtttct attttgttgg ttttttactt tgttttttgt tttgttttgt 1740
tttgttttgc atactacatg cagttcttta accaatgtct gtttggctaa tgtaattaaa 1800
gttgttaatt tatatgagtg catttcaact atgtcaatgg tttcttaata tttattgtgt 1860
agaagtactg gtaatttttt tatttacaat atgtttaaag agataacagt ttgatatgtt 1920
ttcatgtgtt tatagcagaa gttatttatt tctatggcat tccagcggat attttggtgt 1980

ttgcgaggca tgcagtcaat attttgtaca gttagtggcc agtattcagc aacgcctgat 2040
agcttctttg gccttatgtt aaataaaaag acctgtttgg gatgtaaaaa aaaaaaaaaa 2100
aaaaaaaaaa ggagagcgag gacgggcaaa aacagcagag aggagaaagg aagagcaaca 2160
gaaagagaga acgagagagg aggagcaggc cagcgaggcg gaggagaggg aagcaagcag 2220
cgggcgcgcg gcgaagacag ggcgcggcgc agcagcagga gaggggggga ggcgggcggg 2280
acgggagggg gggacccgag aggagcggga agaggagggg cgggcaggaa gggccgcgga 2340
ggaggggagg aacgaagcga agcggggagg aga 2373

<210> 7
<211> 6007
<212> DNA
<213> Homo sapiens

<220>
<221> modified_base
<222> (919)...(920)
<223> n = g, a, c or t

<400> 7
atgccaggca caaaactaac ccgaacaggc gccccagcag actacagagt gatattgaag 60
acctctcaag aggacgaatt ggatgtacct gacgacatca gcgtccgggt tatgtcatct 120
cagtctgtgc ttgtgtcctg ggtggatcct gttctggaaa aacagaagaa agttgttgca 180
tcaagacagt acaccgtgcg ctatcgagag aagggggaat tggccaggtg ggattataag 240
cagatcgcta acaggcgtgt gctgattgag aacctgattc cagacactgt gtatgaattt 300
gcagtccgta tttcacaggg tgaaagagat ggcaaatgga gtacgtcagt cttccaaaga 360
acaccagaat ctgcccctac cacagctcct gaaaacttga acgtctggcc agtcaatggc 420
aaacctacag ttgtcgctgc atcttgggat gcgctaccag agactgaggg gaaagtgaaa 480
gtctgtctgc tggacacagg actgttttca gtttcctcct tccaaccatc tgccaaatca 540
tttcagaata cattctttca tacgccccgg ctctcaaacc atttggagca aagtccctca 600
cctatcctgg agacactact tctgccctgg tggatggtct gcagcctggg gaacgctatc 660
ttttcaaaat ccgggccaca aacaggagag gcctgggacc tcactccaaa gccttcattg 720
tcgctatgcc aacaagaatg cagctgtacc cagaaggatt tcagttgtct agcttacctg 780
atcgatatcc aaaccaaaca agttaataaa gatccacaac tggaagggag tgtttttgga 840
ccatgttttc ttttctactt cctcacattt atgctggata ttggcggctt ttccttcatt 900
atgtgctatg aagacccann tgtttcttct ttgacaggca attctttaaa atctgttgca 960
gccagtaagg cggatgttca gcagaacacg gaggacaatg ggaaacccga aaaacctgag 1020
ccttcctcac cttctcccag agctccagct tcctcccaac acccctctgt gcctgcttct 1080
ccccaaggga gaaatgccaa ggaccttctt cttgacttga agaacaaaat attggctaat 1140
ggtggggcgc cccgaaaacc ccagcttcgc gccaagaagg cagaggagct ggatcttcag 1200
tcgacagaaa tcactgggga ggaggagctg ggttcccggg aggactcgcc catgtcaccc 1260
tcagacaccc aagaccagaa acggaccctg aggccgccaa gtagacacgg ccactcggtg 1320
gttgctcccg gcaggactgc agtgagggcc cggatgccag cgctgccccg aagggaaggc 1380
gtagataagc ctggcttttc cctggccacg cagccccgcc caggggcgcc cccctcggct 1440
tcggcctctc ctgcccacca cgcgtccacc cagggcacct ctcatcgtcc ttccctgcct 1500
gccagcttga atgacaacga cttggtggac tcagacgaag atgagcgcgc tgtgggctcc 1560
ctccacccca agggcgcctt cgcccagccc cggccagccc tgtcccccag ccgccagtcc 1620
ccgtccagcg ttctccgcga cagaagctct gtgcaccccg gcgcaaagcc agcctcgccg 1680
gcgcggagga ccccccattc aggggccgca gaggaagatt ccagtgcctc agccccaccc 1740
tcaagacttt ctccacccca tgggggatca tctcggctgc tgcccaccca gccacacctg 1800
agctctccac tttccaaggg cgggaaggat ggtgaggacg ccccagccac caactccaat 1860
gcgccatcac ggtccaccat gtcctcctcc gtctcttctc atctctcgtc caggacgcag 1920
gtctctgagg gagcggaggc ttctgatggt gaaagccacg gtgacggcga tagggaagac 1980
ggcggaaggc aggcggaggc cacggcccag acgctgcggg cccggcctgc ctctggacac 2040
ttccatttgc tcagacacaa accctttgct gccaacggga ggtctccaag caggttcagc 2100
attgggcggg gacctcggct gcagccctcc agctccccac agtcgactgt gccctcccga 2160
gcccacccca gggttccctc tcactctgat tcccacccta agcttagctc aggtatccat 2220
ggagacgagg aggatgagaa gccgcttcct gccaccgttg tcaatgacca cgtgccttcc 2280
tcctccaggc agcccatctc ccggggctgg gaggacttaa ggagaagccc gcagagaggg 2340
gccagcctgc atcggaagga acccatccca gagaacccca aatccacagg ggcagataca 2400
catcctcagg gcaagtactc ctccctggcc tccaaggctc aggatgttca acagagcaca 2460
gacgcggaca cggagggtca ttctcccaaa gcacagccag ggtccacaga ccgccacgcg 2520
tcccctgctc gtcctcccgc agcacggtca cagcagcatc ccagtgttcc cagaaggatg 2580
acacccggcc gggccccaga acagcagccc cctcctcccg tcgccacgtc ccagcaccac 2640

ccgggacccc agagcagaga cgcgggtcgg tcaccttccc agcccaggct ctcactgacc 2700
caggccgggc ggccccgccc cacgtcgcag ggccgctccc actcctcctc ggacccttac 2760
acggcgagct ccagagggat gctccccacg gccctccaga accaggacga ggatgcccag 2820
ggcagctacg acgacgacag cacagaagtc gaggcccagg atgtgcgggc ccccgcgcac 2880
gccgcgcgcg ccaaggaggc agctgcgtcc cttcccaagc accagcaggt ggagtctccc 2940
acaggcgcag gggcaggtgg cgaccacagg tcccagcgcg gacatgcggc ctcccccgcc 3000
aggcccagcc gacccggcgg cccccagtcc cgcgcccggg tccccagcag ggcagcgccg 3060
gggaagtcgg agcctccttc caagcggccc ctgtcctcca agtcccagca gtcggtctca 3120
gccgaggacg aggaggagga ggacgcgggg ttttttaaag gcgggaaaga agaccttctg 3180
tcttcctctg tgccaaagtg gccctcttcc tccactccca ggggcggcaa agacgccgat 3240
gggagcctcg ccaaggaaga gagggagcct gccatcgcgc ttgcccctcg cggagggagc 3300
ctggctcctg tgaagcgacc tctcccccca cctccaggca gctcccccag ggcctcccac 3360
gtcccttccc gaccgccgcc tcgcagcgct gccaccgtga gccccgtcgc gggcacccac 3420
ccctggccgc ggtacaccac gcgcgccccv cctggccact tctccaccac cccgatgctg 3480
tccttgcgcc agaggatgat gcatgccaga ttccgtaacc ctctctcccg acagcctgcc 3540
agaccctctt acagacaagg ttataatggc agaccaaatg tagaagggaa agtccttcct 3600
ggtagtaatg gaaaaccgaa tggacagaga attatcaatg gccctcaagg aacaaagtgg 3660
gttgtggacc ttgatcgtgg gttagtattg aatgcagaag gaaggtacct ccaagattca 3720
catggaaatc ctcttcggat taaactagga ggagatggtc gaaccattgt agatctggaa 3780
gggacccccg tggtgagtcc tgacggcctc ccactctttg ggcaggggcg acatggcaca 3840
cctctggcca atgcccaaga taagccaatt ttgagtcttg gaggaaagcc gctggtgggc 3900
ttggaggtca tcaaaaaaac cacccatccc cctaccacta ccatgcagcc caccactact 3960
acgacgcccc tgcctaccac tacaaccccg aggcccacca ctgccaccac catgcagccc 4020
accactacta cgacgcccct gcctaccact acaccgaggc ccaccactgc caccacccgc 4080
cgcacgacca ccaggcgtcc aacaaccaca gtccgaacca ctacgcggac aaccaccacc 4140
accaccccca aacccaccac tcccatcccc acctgtcccc ctgggacctt ggaacggcac 4200
gacgatgatg gcaacctgat aatgagctcc aatgggatcc cagagtgcta cgctgaagaa 4260
gatgagttct caggcttgga gactgacact gcagtaccta cggaagaggc ctacgttata 4320
tatgatgaag attatgaatt tgagacgtca aggccaccaa ccaccactga gccttcgacc 4380
actgctacca caccgagggt gatcccagag gaaggcgcca tcagttcctt tcctgaagaa 4440
gaatttgatc tggctggaag gaaacgattt gttgctcctt acgtgacgta cctaaataaa 4500
gacccatcag ccccgtgctc tctgactgat gcactggatc acttccaagt ggacagcctg 4560
gatgaaatca tccccaatga cctgaagaag agtgatctgc ctccccagca tgctccccgc 4620
aacatcaccg tggtggccgt ggaaggttgc cactcatttg tcattgtgga ttgggacaaa 4680
gccaccccag gagatttggt cacaggttat ttggtttaca gtgcatccta tgaagatttc 4740
atcaggaaca agttttccac tcaagcttca tcagtaactc acttgcccat tgagaaccta 4800
aagcccaaca cgaggtatta ttttaaagtg caagcacaaa atcctcatgg ctacggacct 4860
atcagccctt cggtctcatt tgtcaccgaa tcagataatc ctctgcttgt tgtgaggccc 4920
ccaggcggtg agctatctgg atcccattcg ctttcaaaca tgatcccagc tacacggact 4980
gccatggacg gcaatatgtg aagcgcacgt ggtatcgaaa gttcgtggga gttgttcttt 5040
gtaattcact gaggtataaa atctacctca gtgacaacct gaaagataca ttctacagca 5100
ttggagacag ctggggaaga ggtgaagacc attgccaatt tgtggattca caccttgatg 5160
gaagaacagg gcctcagtcc tatgtagaag ccctccctac tattcaaggc tactatcgcc 5220
agtatcgtca ggagcctgtc aggtttggga acatcggctt cggaaccccc tactactatg 5280
tgggctggta cgagtgtggg gtctccatcc ctggaaagtg gtaatcacag gaccgtcatg 5340
ctgcaagctt gccctgccca gccccaccaa ctaagtcgca ctaggggctg tgagcaaaga 5400
cagccagcat gctcagcccc gctgccctag gtgccaggaa ggtcacagat ggacactggc 5460
cattctggtc atctcagtct ggaactcagt cccacttctt ggcctggaca atgaacagga 5520
ttcagttttg ctgttaactt tgcttctcta cttttttttg tttgtttgta atagcacatc 5580
ccagagacat cagaaaccag caactgattc agtgtgattt cccagacttt ttaggcatga 5640
aattcggaca cttcagtatt tccaggaata gcatatgcac gctgttcttg cttcatggaa 5700
tgctacatgc tttctgtttt tctcattttg gatttctcca aaactaactg aatttaagct 5760
tcaggtccct ttgtatgcag tagaaaggaa ttattaaaaa caccaccaaa gaaaataaat 5820
atatcctact tgaaatttac tctatggact tacccactgc tagaataaat gtatcaaatc 5880
ttatttgtaa attctcaatt ttgatatata tatgtatata tgcatataca tatccacact 5940
tgtctgcaag aatattgatt aaaattgcta aatttgtact tgttcaccaa aaaaaaaaaa 6000
aaaaaaa 6007

<210> 8
<211> 3636
<212> DNA
<213> Homo sapiens

<400> 8
atggatccgc cggcgggagc cgctcgccgc ctgctctgcc ccgcgctgct gctgctgctg 60
ctgctgctgc cgccgccgct cctgccgccg ccgccgccgc ccgcgaacgc caggctcgcc 120
gccgccgccg accccccagg cgggcccctg gggcacggag cggagcgcat cctggcggtg 180
cccgtgcgca ctgacgccca gggccgcttg gtgtcccacg tggtgtcggc agctacgtcc 240
agagcagggg tacgagcccg cagggccgcc ccggtccgga ccccgagctt ccccggaggc 300
aacgaggagg agcctggcag tcacctcttc tacaatgtca cggtctttgg ccgagacctg 360
cacctgcggc tgcggcccaa cgcccgcctc gtggcgcccg gggccactat ggagtggcag 420
ggcgagaagg gcaccacccg cgtggagccc ctgctcggga gctgtctcta cgtcggagac 480
gtggccggcc tagccgaagc ctcctctgtg gcgctcagca actgcgatgg gctggctggt 540
ctgatccgga tggaggagga ggagttcttc atcgaaccct tggagaaggg gctggcggcg 600
caggaggctg agcaaggccg tgtgcatgtg gtgtatcgcc ggccacccac gtcccctcct 660
ctcggggggc cacaggccct ggacacaggg gcctccctgg acagcctgga cagcctcagc 720
cgcgccctgg gcgtcctaga ggagcacgcc aacagctcga ggcggagggc acgcaggcat 780
gctgcagacg atgactacaa catcgaggtc ctgctgggcg tggatgactc tgtggtgcag 840
ttccacggga aggagcacgt acagaagtac ctgctgacac tcatgaacat tgtcaatgaa 900
atctaccatg acgagtcctt gggtgcccac atcaacgtgg tcctggtgcg gatcatcctc 960
ctgagctatg gaaagtccat gagcctcatc gagatcggga acccctctca gagcctggag 1020
aatgtctgcc gctgggccta cctccagcag aagccagaca cgggccacga tgaataccac 1080
gatcacgcca tcttcctcac acggcaggac tttgggcctt ccggcatgca aggctatgct 1140
cctgtcaccg gcatgtgcca tccggtccgc agctgcaccc tgaaccatga ggacggcttc 1200
tcctcagcgt ttgtggtggc ccatgagact ggccacgtgc tgggcatgga gcacgacggg 1260
cagggcaacc gctgtggcga cgaggtgcgg ctgggcagca tcatggcgcc cctggtgcag 1320
gccgccttcc accgcttcca ctggtcccgc tgcagccagc aggagctgag ccgctacctg 1380
cactcctatg actgcctgct ggatgacccc ttcgcccacg actggccggc gctgccccag 1440
ctcccgggac tgcactactc catgaacgag caatgccgct ttgacttcgg cctgggctac 1500
atgatgtgca cggcgttccg gacctttgac ccctgcaagc agctgtggtg cagccatcct 1560
gacaacccct acttttgcaa gaccaagaag gggcccccct tggacgggac tatgtgtgca 1620
cctggcaagc attgttttaa aggacactgc atctggctga cacctgacat cctcaaacgg 1680
gacggcagct ggggcgcttg gagtccgttt ggctcctgct cacgtacctg tggcacgggc 1740
gtgaagttca ggacccgcca gtgtgacaac ccacacccgg ccaacggggg ccgcacctgc 1800
tcgggccttg cctacgactt ccagctctgc agccgccagg actgccccga ctccctggct 1860
gacttccgcg aggagcagtg ccgccagtgg gacctgtact tcgagcacgg cgacgcccag 1920
caccactggc tgccccacga gcaccgggat gccaaggaga gatgccacct gtactgcgag 1980
tccagggaga ccggggaggt ggtgtccatg aagcgcatgg tgcatgatgg gacgcgctgc 2040
tcctacaagg acgccttcag cctctgtgtg cgcggggact gcaggaaggt gggctgtgac 2100
ggtgtgatcg gctccagcaa gcaggaagac aagtgtggcg tgtgcggagg ggacaacagc 2160
cactgcaaag tggtcaaggg cacgttcaca cggtcaccca agaagcatgg ttacatcaag 2220
atgtttgaga tccctgcagg agccagacac ctgctcattc aggaggtaga cgccaccagc 2280
caccatctgg ccgtcaagaa cctggagaca ggcaagttca tcttaaatga agagaatgac 2340
gtggatgcca gttccaaaac cttcattgcc atgggcgtgg agtgggagta cagagacgag 2400
gacggccggg agacgctgca gaccatgggc cccctccacg gcaccatcac cgttctggtc 2460
atcccggtgg gagacacccg ggtctcactg acgtacaaat acatgatcca tgaggactca 2520
ctgaatgtcg atgacaacaa cgtcctggaa gaggactctg tggtctacga gtgggccctg 2580
aagaagtggt ctccgtgctc caagccctgt ggcggagggt cccagttcac caagtatggc 2640
tgccgccgga ggctggacca caagatggta caccgtggct tctgtgccgc cctctcgaag 2700
cccaaagcca tccgcagagc gtgcaaccca caggaatgct cccagccagt gtgggtcaca 2760
ggcgaatggg agccatgtag ccagacctgt gggcggacag gcatgcaggt gcgctccgtg 2820
cgctgcattc agccgctaca cgacaacacc acccgctccg tgcacgccaa gcactgcaat 2880
gacgcccggc ccgagagccg ccgggcctgc agccgcgagc tctgccctgg tcgttggcga 2940
gccgggccct ggtcccagtg ctcagtaacc tgtggcaacg gcacccagga gcggccagtg 3000
ccctgccgca ccgcggacga cagcttcggc atctgccagg aggagcgtcc tgagacagcg 3060
aggacctgca ggcttggccc ctgtccccga aacatctcag atccctccaa gaagagctac 3120
gtagttcagt ggctgtcccg cccggacccc gactcgccca tccggaagat ctcgtcaaag 3180
ggccactgcc aaggcgacaa gtcaatattc tgtaggatgg aagtcttgtc ccgctattgc 3240
tccatcccag gctacaacaa gctgtcctgc aagtcctgta acctgtacaa caacctcacc 3300
aacgtggagg gcaggataga gccaccgcct gggaagcaca acgacattga cgtgttcatg 3360
cctaccctcc cagtgcccac tgtagccatg gaggtgcggc catcaccaag cacccccctg 3420
gaggtccctc tcaatgcctc cagcaccaat gccacagagg atcacccaga aaccaatgcc 3480
gtagatgaac cctacaaaat ccatggcctg gaagatgaag tccagccacc caacctaatc 3540
cctcgacgac cgagccccta tgaaaagacc agaaaccaaa gaatccaaga gctcattgat 3600
gagatgcgga agaaagagat gctcggaaag ttctaa 3636

<210> 9
<211> 2465
<212> DNA
<213> Homo sapiens

<400> 9
accgggcacc ggacggctcg ggtactttcg ttcttaatta ggtcatgccc gtgtgagcca 60
ggaaagggct gtgtttatgg gaagccagta acactgtggc ctactatctc ttccgtggtg 120
ccatctacat ttttgggact cgggaattat gaggtagagg tggaggcgga gccggatgtc 180
agaggtcctg aaatagtcac catgggggaa aatgatccgc ctgctgttga agcccccttc 240
tcattccgat cgctttttgg ccttgatgat ttgaaaataa gtcctgttgc accagatgca 300
gatgctgttg ctgcacagat cctgtcactg ctgccattga agttttttcc aatcatcgtc 360
attgggatca ttgcattgat attagcactg gccattggtc tgggcatcca cttcgactgc 420
tcagggaagt acagatgtcg ctcatccttt aagtgtatcg agctgatagc tcgatgtgac 480
ggagtctcgg attgcaaaga cggggaggac gagtaccgct gtgtccgggt gggtggtcag 540
aatgccgtgc tccaggtgtt cacagctgct tcgtggaaga ccatgtgctc cgatgactgg 600
aagggtcact acgcaaatgt tgcctgtgcc caactgggtt tcccaagcta tgtgagttca 660
gataacctca gagtgagctc gctggagggg cagttccggg aggagtttgt gtccatcgat 720
cacctcttgc cagatgacaa ggtgactgca ttacaccact cagtatatgt gagggaggga 780
tgtgcctctg gccacgtggt taccttgcag tgcacagcct gtggtcatag aaggggctac 840
agctcacgca tcgtgggtgg aaacatgtcc ttgctctcgc agtggccctg gcaggccagc 900
cttcagttcc agggctacca cctgtgcggg ggctctgtca tcacgcccct gtggatcatc 960
actgctgcac actgtgttta tgacttgtac ctccccaagt catggaccat ccaggtgggt 1020
ctagtttccc tgttggacaa tccagcccca tcccacttgg tggagaagat tgtctaccac 1080
agcaagtaca agccaaagag gctgggcaat gacatcgccc ttatgaagct ggccgggcca 1140
ctcacgttca atgaaatgat ccagcctgtg tgcctgccca actctgaaga gaacttcccc 1200
gatggaaaag tgtgctggac gtcaggatgg ggggccacag aggatggagg tgacgcctcc 1260
cctgtcctga accacgcggc cgtccctttg atttccaaca agatctgcaa ccacagggac 1320
gtgtacggtg gcatcatctc cccctccatg ctctgcgcgg gctacctgac gggtggcgtg 1380
gacagctgcc agggggacag cggggggccc ctggtgtgtc aagagaggag gctgtggaag 1440
ttagtgggag cgaccagctt tggcatcggc tgcgcagagg tgaacaagcc tggggtgtac 1500
acccgtgtca cctccttcct ggactggatc cacgagcaga tggagagaga cctaaaaacc 1560
tgaagaggaa ggggacaagt agccacctga gttcctgagg tgatgaagac agcccgatcc 1620
tcccctggac tcccgtgtag gaacctgcac acgagcagac acccttggag ctctgagttc 1680
cggcaccagt agcaggcccg aaagaggcac ccttccatct gattccagca caaccttcaa 1740
gctgcttttt gttttttgtt tttttgaggt ggagtctcgc tctgttgccc aggctggagt 1800
gcagtggcga aatccctgct cactgcagcc tccgcttccc tggttcaagc gattctcttg 1860
cctcagcttc cccagtagct gggaccacag gtgcccgcca ccacacccaa ctaatttttg 1920
tatttttagt agagacaggg tttcaccatg ttggccaggc tgctctcaaa cccctgacct 1980
caaatgatgt gcctgcttca gcctcccaca gtgctgggat tacaggcatg ggccaccacg 2040
cctagcctca cgctcctttc tgatcttcac taagaacaaa agaagcagca acttgcaagg 2100
gcggcctttc ccactggtcc atctggtttt ctctccaggg gtcttgcaaa attcctgacg 2160
agataagcag ttatgtgacc tcacgtgcaa agccaccaac agccactcag aaaagacgca 2220
ccagcccaga agtgcagaac tgcagtcact gcacgttttc atctctaggg accagaacca 2280
aacccaccct ttctacttcc aagacttatt ttcacatgtg gggaggttaa tctaggaatg 2340
actcgtttaa ggcctatttt catgatttct ttgtagcatt tggtgcttga cgtattattg 2400
tcctttgatt ccaaataata tgtttccttc cctcaaaaaa aaaaaaaaaa aaaaaaaaaa 2460
aaaaa 2465
<210> 10
<211> 2034
<212> DNA
<213> Homo sapiens

<400> 10
atgctgtcat ccactgactt tacatttgct tcctgggagc ttgtggtccg cgttgaccat 60
cccaatgaag agcagcagaa agacgtcaca ctgagagtat ctggagacct tcatgttgga 120
ggagtgatgc tcaagttagt agaacagatc aatatatccc aagactggtc agactttgct 180
ctttggtggg aacagaagca ttgctggctt ctgaaaaccc actggaccct ggacaaatat 240
ggggtccagg cagatgcaaa gcttctcttc acccctcagc ataaaatgct gcgccttcgt 300
ctgccgaatt tgaagatggt gaggttgcga gtcagcttct cagctgtggt ttttaaagct 360
gtcagtgata tctgcaaaat cctgaatatt agaagatcag aagagctttc cttgttaaag 420
ccgtctggtg actattttaa gaagaagaag aaaaaagaca aaaataataa ggaacccata 480
attgaagata ttctaaacct ggagagttct ccaacagctt caggttcatc agtaagtcct 540

ggtttataca gtaaaaccat gacccctata tatgacccca tcaatggaac accagcatca 600
tccaccatga cttggttcag tgacagccct ttgacggaac aaaactgcag catcctcgca 660
ttcagccaac ccccccagtc cccagaagca cttgcggata tgtaccagcc tcggtctctg 720
gttgataaag ccaagctcaa tgcaggttgg ctagactcct cacgctccct tatggaacaa 780
ggcatccaag aggatgagca gctgctctta cgatttaaat attattcttt cttcgacttg 840
aatcctaaat atgatgctgt ccgaataaac caactctatg agcaagccag gtgggccatt 900
ctcttagaag aaattgattg cacagaggaa gaaatgttga tctttgcagc tctacagtac 960
cacattagca aactgtcgtt gtctgctgaa acacaggatt ttgcaggcga gtccgaggtt 1020
gatgaaatag aagcggcgct ttctaatttg gaagtaaccc tagaaggtgg aaaagcggac 1080
agccttttgg aggacattac tgatatccct aaacttgcag ataatctcaa attatttagg 1140
cccaagaagt tactaccaaa agctttcaaa caatattggt ttatctttaa agacacatcc 1200
atagcatact ttaaaaataa ggaacttgaa caaggagaac cactagaaaa actaaatctt 1260
agaggctgcg aagttgtgcc cgatgtaaat gtagcaggaa gaaaatttgg aatcaagtta 1320
ctaatccctg ttgccgatgg tatgaatgaa atgtatttga gatgtgacca tgagaatcaa 1380
tacgcccaat ggatggctgc ctgcatgttg gcatcgaagg gcaaaaccat ggcagacagc 1440
tcctaccagc cagaggtcct caacatcctt tcatttctga ggatgaaaaa caggaactct 1500
gcatctcagg tggcttccag tctcgaaaac atggatatga acccagaatg ttttgtgtca 1560
ccacggtgtg caaaaagaca caaatccaaa cagctggccg cccggatcct ggaggcgcac 1620
cagaacgtgg cccagatgcc cctggtcgaa gccaagctgc ggttcatcca ggcgtggcag 1680
tcactgcctg agtttggcct cacctactac cttgtcagat ttaaaggaag caaaaaagat 1740
gacattctgg gagtttcata taacaggttg attaaaattg atgcagccac cgggattcca 1800
gtgacaacat ggagattcac aaatatcaaa cagtggaatg taaactggga aacccggcag 1860
gtggtcatcg agtttgacca aaacgtcttt actgctttca cctgcctgag tgcagattgc 1920
aagattgtgc acgagtacat tggcggctac attttcttgt ccacccgctc caaggaccag 1980
aatgaaacac tcgatgagga cttgttccac aaattgaccg gcggtcagga ttga 2034

<210> 11
<211> 1200
<212> DNA
<213> Homo sapiens

<400> 11
aagatataaa agctccagaa acgttgactg ggaccactgg agacactgaa gaaggcaggg 60
gcccttagag tcttggttgc caaacagatt tgcagatcaa ggagaaccca ggagtttcaa 120
agaagcgcta gtaaggtctc tgagatcctt gcactagcta catcctcagg gtaggaggaa 180
gatggcttcc agaagcatgc ggctgctcct attgctgagc tgcctggcca aaacaggagt 240
cctgggtgat atcatcatga gacccagctg tgctcctgga tggttttacc acaagtccaa 300
ttgctatggt tacttcagga agctgaggaa ctggtctgat gccgagctcg agtgtcagtc 360
ttacggaaac ggagcccacc tggcatctat cctgagttta aaggaagcca gcaccatagc 420
agagtacata agtggctatc agagaagcca gccgatatgg attggcctgc acgacccaca 480
gaagaggcag cagtggcagt ggattgatgg ggccatgtat ctgtacagat cctggtctgg 540
caagtccatg ggtgggaaca agcactgtgc tgagatgagc tccaataaca actttttaac 600
ttggagcagc aacgaatgca acaagcgcca acacttcctg tgcaagtacc gaccatagag 660
caagaatcaa gattctgcta actcctgcac agccccgtcc tcttcctttc tgctagcctg 720
gctaaatctg ctcattattt cagaggggaa acctagcaaa ctaagagtga taagggccct 780
actacactgg cttttttagg cttagagaca gaaactttag cattggccca gtagtggctt 840
ctagctctaa atgtttgccc cgccatccct ttccacagta tccttcttcc ctcctcccct 900
gtctctggct gtctcgagca gtctagaaga gtgcatctcc agcctatgaa acagctgggt 960
ctttggccat aagaagtaaa gatttgaaga cagaaggaag aaactcagga gtaagcttct 1020
agaccccttc agcttctaca cccttctgcc ctctctccat tgcctgcacc ccaccccagc 1080
cactcaactc ctgcttgttt ttcctttggc cataggaagg tttaccagta gaatccttgc 1140
taggttgatg tgggccatac attcctttaa taaaccattg tgtacataag aaaaaaaaaa 1200

<210> 12
<211> 7080
<212> DNA
<213> Homo sapiens

<400> 12
gagctagcgc tcaagcagag cccagcgcgg tgctatcgga cagagcctgg cgagcgcaag 60
cggcgcgggg agccagcggg gctgagcgcg gccagggtct gaacccagat ttcccagact 120
agctaccact ccgcttgccc acgccccggg agctcgcggc gcctggcggt cagcgaccag 180
acgtccgggg ccgctgcgct cctggcccgc gaggcgtgac actgtctcgg ctacagaccc 240

agagggagca cactgccagg atgggagctg ctgggaggca ggacttcctc ttcaaggcca 300
tgctgaccat cagctggctc actctgacct gcttccctgg ggccacatcc acagtggctg 360
ctgggtgccc tgaccagagc cctgagttgc aaccctggaa ccctggccat gaccaagacc 420
accatgtgca tatcggccag ggcaagacac tgctgctcac ctcttctgcc acggtctatt 480
ccatccacat ctcagaggga ggcaagctgg tcattaaaga ccacgacgag ccgattgttt 540
tgcgaacccg gcacatcctg attgacaacg gaggagagct gcatgctggg agtgccctct 600
gccctttcca gggcaatttc accatcattt tgtatggaag ggctgatgaa ggtattcagc 660
cggatcctta ctatggtctg aagtacattg gggttggtaa aggaggcgct cttgagttgc 720
atggacagaa aaagctctcc tggacatttc tgaacaagac ccttcaccca ggtggcatgg 780
cagaaggagg ctattttttt gaaaggagct ggggccaccg tggagttatt gttcatgtca 840
tcgaccccaa atcaggcaca gtcatccatt ctgaccggtt tgacacctat agatccaaga 900
aagagagtga acgtctggtc cagtatttga acgcggtgcc cgatggcagg atcctttctg 960
ttgcagtgaa tgatgaaggt tctcgaaatc tggatgacat ggccaggaag gcgatgacca 1020
aattgggaag caaacacttc ctgcaccttg gatttagaca cccttggagt tttctaactg 1080
tgaaaggaaa tccatcatct tcagtggaag accatattga atatcatgga catcgaggct 1140
ctgctgctgc ccgggtattc aaattgttcc agacagagca tggcgaatat ttcaatgttt 1200
ctttgtccag tgagtgggtt caagacgtgg agtggacgga gtggttcgat catgataaag 1260
tatctcagac taaaggtggg gagaaaattt cagacctctg gaaagctcac ccaggaaaaa 1320
tatgcaatcg tcccattgat atacaggcca ctacaatgga tggagttaac ctcagcaccg 1380
aggttgtcta caaaaaaggc caggattata ggtttgcttg ctacgaccgg ggcagagcct 1440
gccggagcta ccgtgtacgg ttcctctgtg ggaagcctgt gaggcccaaa ctcacagtca 1500
ccattgacac caatgtgaac agcaccattc tgaacttgga ggataatgta cagtcatgga 1560
aacctggaga taccctggtc attgccagta ctgattactc catgtaccag gcagaagagt 1620
tccaggtgct tccctgcaga tcctgcgccc ccaaccaggt caaagtggca gggaaaccaa 1680
tgtacctgca catcggggag gagatagacg gcgtggacat gcgggcggag gttgggcttc 1740
tgagccggaa catcatagtg atgggggaga tggaggacaa atgctacccc tacagaaacc 1800
acatctgcaa tttctttgac ttcgatacct tgggggccca catcaagttt gctctgggat 1860
ttaaggcagc acacttggag ggcacggagc tgaagcatat gggacagcag ctggtgggtc 1920
agtacccgat tcacttccac ctggccggtg atgtagacga aaggggaggt tatgacccac 1980
ccacatacat cagggacctc tccatccatc atacattctc tcgctgcgtc acagtccatg 2040
gctccaatgg cttgttgatc aaggacgttg tgggctataa ctctttgggc cactgcttct 2100
tcacggaaga tgggccggag gaacgcaaca cttttgacca ctgtcttggc ctccttgtca 2160
agtctggaac cctcctcccc tcggaccgtg acagcaagat gtgcaagatg atcacagagg 2220
actcctaccc ggggtacatc cccaagccca ggcaagactg caatgctgtg tccaccttct 2280
ggatggccaa tcccaacaac aacctcatca actgtgccgc tgcaggatct gaggaaactg 2340
gattttggtt tatttttcac cacgtaccaa cgggcccctc cgtgggaatg tactccccag 2400
gttattcaga gcacattcca ctgggaaaat tctataacaa ccgagcacat tccaactacc 2460
gggctggcat gatcatagac aacggagtca aaaccaccga ggcctctgcc aaggacaagc 2520
ggccgttcct ctcaatcatc tctgccagat acagccctca ccaggacgcc gacccgctga 2580
agccccggga gccggccatc atcagacact tcattgccta caagaaccag gaccacgggg 2640
cctggctgcg cggcggggat gtgtggctgg acagctgccg gtttgctgac aatggcattg 2700
gcctgaccct ggccagtggt ggaaccttcc cgtatgacga cggctccaag caagagataa 2760
agaacagctt gtttgttggc gagagtggca acgtggggac ggaaatgatg gacaatagga 2820
tctggggccc tggcggcttg gaccatagcg gaaggaccct ccctataggc cagaattttc 2880
caattagagg aattcagtta tatgatggcc ccatcaacat ccaaaactgc actttccgaa 2940
agtttgtggc cctggagggc cggcacacca gcgccctggc cttccgcctg aataatgcct 3000
ggcagagctg cccccataac aacgtgaccg gcattgcctt tgaggacgtt ccgattactt 3060
ccagagtgtt cttcggagag cctgggccct ggttcaacca gctggacatg gatggggata 3120
agacatctgt gttccatgac gtcgacggct ccgtgtccga gtaccctggc tcctacctca 3180
cgaagaatga caactggctg gtccggcacc cagactgcat caatgttccc gactggagag 3240
gggccatttg cagtgggtgc tatgcacaga tgtacattca agcctacaag accagtaacc 3300
tgcgaatgaa gatcatcaag aatgacttcc ccagccaccc tctttacctg gagggggcgc 3360
tcaccaggag cacccattac cagcaatacc aaccggttgt caccctgcag aagggctaca 3420
ccatccactg ggaccagacg gcccccgccg aactcgccat ctggctcatc aacttcaaca 3480
agggcgactg gatccgagtg gggctctgct acccgcgagg caccacattc tccatcctct 3540
cggatgttca caatcgcctg ctgaagcaaa cgtccaagac gggcgtcttc gtgaggacct 3600
tgcagatgga caaagtggag cagagctacc ctggcaggag ccactactac tgggacgagg 3660
actcagggct gttgttcctg aagctgaaag ctcagaacga gagagagaag tttgctttct 3720
gctccatgaa aggctgtgag aggataaaga ttaaagctct gattccaaag aacgcaggcg 3780
tcagtgactg cacagccaca gcttacccca agttcaccga gagggctgtc gtagacgtgc 3840
cgatgcccaa gaagctcttt ggttctcagc tgaaaacaaa ggaccatttc ttggaggtga 3900
agatggagag ttccaagcag cacttcttcc acctctggaa cgacttcgct tacattgaag 3960
tggatgggaa gaagtacccc agttcggagg atggcatcca ggtggtggtg attgacggga 4020

accaagggcg cgtggtgagc cacacgagct tcaggaactc cattctgcaa ggcataccat 4080
ggcagctttt caactatgtg gcgaccatcc ctgacaattc catagtgctt atggcatcaa 4140
agggaagata cgtctccaga ggcccatgga ccagagtgct ggaaaagctt ggggcagaca 4200
ggggtctcaa gttgaaagag caaatggcat tcgttggctt caaaggcagc ttccggccca 4260
tctgggtgac actggacact gaggatcaca aagccaaaat cttccaagtt gtgcccatcc 4320
ctgtggtgaa gaagaagaag ttgtgaggac agctgccgcc cggtgccacc tcgtggtaga 4380
ctatgacggt gactcttggc agcagaccag tgggggatgg ctgggtcccc cagcccctgc 4440
cagcagctgc ctgggaaggc cgtgtttcag ccctgatggg ccaagggaag gctatcagag 4500
accctggtgc tgccacctgc ccctactcaa gtgtctacct ggagcccctg gggcggtgct 4560
ggccaatgct ggaaacattc actttcctgc agcctcttgg gtgcttctct cctatctgtg 4620
cctcttcagt gggggtttgg ggaccatatc aggagacctg ggttgtgctg acagcaaaga 4680
tccactttgg caggagccct gacccagcta ggaggtagtc tggagggctg gtcattcaca 4740
gatccccatg gtcttcagca gacaagtgag ggtggtaaat gtaggagaaa gagccttggc 4800
cttaaggaaa tctttactcc tgtaagcaag agccaacctc acaggattag gagctggggt 4860
agaactggct atccttgggg aagaggcaag ccctgcctct ggccgtgtcc acctttcagg 4920
agactttgag tggcaggttt ggacttggac tagatgactc tcaaaggccc ttttagttct 4980
gagattccag aaatctgctg catttcacat ggtacctgga acccaacagt tcatggatat 5040
ccactgatat ccatgatgct gggtgcccca gcgcacacgg gatggagagg tgagaactaa 5100
tgcctagctt gaggggtctg cagtccagta gggcaggcag tcaggtccat gtgcactgca 5160
atgccaggtg gagaaatcac agagaggtaa aatggaggcc agtgccattt cagaggggag 5220
gctcaggaag gcttcttgct tacaggaatg aaggctgggg gcattttgct ggggggagat 5280
gaggcagcct ctggaatggc tcagggattc agccctccct gccgctgcct gctgaagctg 5340
gtgactacgg ggtcgccctt tgctcacgtc tctctggccc actcatgatg gagaagtgtg 5400
gtcagagggg agcaatgggc tttgctgctt atgagcacag aggaattcag tccccaggca 5460
gccctgcctc tgactccaag agggtgaagt ccacagaagt gagctcctgc cttagggcct 5520
catttgctct tcatccaggg aactgagcac agggggcctc caggagaccc tagatgtgct 5580
cgtactccct cggcctggga tttcagagct ggaaatatag aaaatatcta gcccaaagcc 5640
ttcattttaa cagatgggga aagtgagccc ccaagatggg aaagaaccac acagctaagg 5700
gagggcctgg ggagccccac cctagccctt gctgccacac cacattgcct caacaaccgg 5760
ccccagagtg cccaggcact cctgaggtag cttctggaaa tggggacaag tcccctcgaa 5820
ggaaaggaaa tgactagagt agaatgacag ctagcagatc tcttccctcc tgctcccagc 5880
gcacacaaac ccgccctccc cttggtgttg gcggtccctg tggccttcac tttgttcact 5940
acctgtcagc ccagcctggg tgcacagtag ctgcaactcc ccattggtgc tacctggctc 6000
tcctgtctct gcagctctac aggtgaggcc cagcagaggg agtagggctc gccatgtttc 6060
tggtgagcca atttggctga tcttgggtgt ctgaacagct attgggtcca ccccagtccc 6120
tttcagctgc tgcttaatgc cctgctctct ccctggccca ccttatagag agcccaaaga 6180
gctcctgtaa gagggagaac tctatctgtg gtttataatc ttgcacgagg caccagagtc 6240
tccctgggtc ttgtgatgaa ctacatttat cccctttcct gccccaacca caaactcttt 6300
ccttcaaaga gggcctgcct ggctccctcc acccaactgc acccatgaga ctcggtccaa 6360
gagtccattc cccaggtggg agccaactgt cagggaggtc tttcccacca aacatctttc 6420
agctgctggg aggtgaccat agggctctgc ttttaaagat atggctgctt caaaggccag 6480
agtcacagga aggacttctt ccagggagat tagtggtgat ggagaggaga gttaaaatga 6540
cctcatgtcc ttcttgtcca cggttttgtt gagttttcac tcttctaatg caagggtctc 6600
acactgtgaa ccacttagga tgtgatcact ttcaggtggc caggaatgtt gaatgtcttt 6660
ggctcagttc atttaaaaaa gatatctatt tgaaagttct cagagttgta catatgtttc 6720
acagtacagg atctgtacat aaaagtttct ttcctaaacc attcaccaag agccaatatc 6780
taggcatttt cttggtagca caaattttct tattgcttag aaaattgtcc tccttgttat 6840
ttctgtttgt aagacttaag tgagttaggt ctttaaggaa agcaacgctc ctctgaaatg 6900
cttgtctttt ttctgttgcc gaaatagctg gtcctttttc gggagttaga tgtatagagt 6960
gtttgtatgt aaacatttct tgtaggcatc accatgaaca aagatatatt ttctatttat 7020
ttattatatg tgcacttcaa gaagtcactg tcagagaaat aaagaattgt cttaaatgtc 7080

<210> 13
<211> 3072
<212> DNA
<213> Homo sapiens

<400> 13
tcagacctgt aggcctgata gactgattaa accacagaag gtgacctgct gagaaaagtg 60
gtacaaatac tgggaaaaac ctgctcttct gcgttaagtg ggagacaatg tcacaagtta 120
aaagctctta ttcctatgat gccccctcgg atttcatcaa tttttcatcc ttggatgatg 180
aaggagatac tcaaaacata gattcatggt ttgaggagaa ggccaatttg gagaataagt 240

tactggggaa gaatggaact ggagggcttt ttcagggcaa aactcctttg agaaaggcta 300
atcttcagca agctattgtc acacctttga aaccagttga caacacttac tacaaagagg 360
cagaaaaaga aaatcttgtg gaacaatcca ttccgtcaaa tgcttgttct tccctggaag 420
ttgaggcagc catatcaaga aaaactccag cccagcctca gagaagatct cttaggcttt 480
ctgctcagaa ggatttggaa cagaaagaaa agcatcatgt aaaaatgaaa gccaagagat 540
gtgccactcc tgtaatcatc gatgaaattc taccctctaa gaaaatgaaa gtttctaaca 600
acaaaaagaa gccagaggaa gaaggcagtg ctcatcaaga tactgctgaa aagaatgcat 660
cttccccaga gaaagccaag ggtagacata ctgtgccttg tatgccacct gcaaagcaga 720
agtttctaaa aagtactgag gagcaagagc tggagaagag tatgaaaatg cagcaagagg 780
tggtggagat gcggaaaaag aatgaagaat tcaagaaact tgctctggct ggaatagggc 840
aacctgtgaa gaaatcagtg agccaggtca ccaaatcagt tgacttccac ttccgcacag 900
atgagcgaat caaacaacat cctgagaacc aggaggaata taaggaagtg aactttacat 960
ctgaactacg aaagcatcct tcatctcctg cccgagtgac taagggatgt accattgtta 1020
agcctttcaa cctgtcccaa ggaaagaaaa gaacatttga tgaaacagtt tctacatatg 1080
tgccccttgc acagcaagtt gaagacttcc ataaacgaac ccctaacaga tatcatttga 1140
ggagcaagaa ggatgatatt aacctgttac cctccaaatc ttctgtgacc aagatttgca 1200
gagacccaca gactcctgta ctgcaaacca aacaccgtgc acgggctgtg acctgcaaaa 1260
gtacagcaga gctggaggct gaggagctcg agaaattgca acaatacaaa ttcaaagcac 1320
gtgaacttga tcccagaata cttgaaggtg ggcccatctt gcccaagaaa ccacctgtga 1380
aaccacccac cgagcctatt ggctttgatt tggaaattga gaaaagaatc caggagcgag 1440
aatcaaagaa gaaaacagag gatgaacact ttgaatttca ttccagacct tgccctacta 1500
agattttgga agatgttgtg ggtgttcctg aaaagaaggt acttccaatc accgtcccca 1560
agtcaccagc ctttgcattg aagaacagaa ttcgaatgcc caccaaagaa gatgaggaag 1620
aggacgaacc ggtagtgata aaagctcaac ctgtgccaca ttatggggtg ccttttaagc 1680
cccaaatccc agaggcaaga actgtggaaa tatgcccttt ctcctttgat tctcgagaca 1740
aagaacgtca gttacagaag gagaagaaaa taaaagaact gcagaaaggg gaggtgccca 1800
agttcaaggc acttcccttg cctcattttg acaccattaa cctgccagag aagaaggtaa 1860
agaatgtgac ccagattgaa cctttctgct tggagactga cagaagaggt gctctgaagg 1920
cacagacttg gaagcaccag ctggaagaag aactgagaca gcagaaagaa gcagcttgtt 1980
tcaaggctcg tccaaacacc gtcatctctc aggagccctt tgttcccaag aaagagaaga 2040
aatcagttgc tgagggcctt tctggttctc tagttcagga accttttcag ctggctactg 2100
agaagagagc caaagagcgg caggagctgg agaagagaat ggctgaggta gaagcccaga 2160
aagcccagca gttggaggag gccagactac aggaggaaga gcagaaaaaa gaggagctgg 2220
ccaggctacg gagagaactg gtgcataagg caaatccaat acgcaagtac cagggtctgg 2280
agataaagtc aagtgaccag cctctgactg tgcctgtatc tcccaaattc tccactcgat 2340
tccactgcta aactcagctg tgagctgcgg ataccgcccg gcaatgggac ctgctcttaa 2400
cctcaaacct aggaccgtct tgctttgtca ttgggcatgg agagaaccca tttctccaga 2460
cttttaccta cccgtgcctg agaaagcata cttgacaact gtggactcca gttttgttga 2520
gaattgtttt cttacattac taaggctaat aatgagatgt aactcatgaa tgtctcgatt 2580
agactccatg tagttacttc ctttaaacca tcagccggcc ttttatatgg gtcttcactc 2640
tgactagaat ttagtctctg tgtcagcaca gtgtaatctc tattgctatt gccccttacg 2700
actctcaccc tctccccaac tttttttaaa aattttaacc agaaaataaa gatagttaaa 2760
tcctaagata gagattaagt catggtttaa atgaggaaca atcagtaaat cagattctgt 2820
cctcttctct gcataccgtg aatttatagt taaggatccc tttgctgtga gggtagaaaa 2880
cctcaccaac tgcaccagtg aggaagaaga ctgcgtggat tcatggggag cctcacagca 2940
gccacgcagc aggctctggg tggggctgcc gttaaggcac gttctttcct tactggtgct 3000
gataacaaca gggaaccgtg cagtgtgcat tttaagacct ggcctggaat aaatacgttt 3060
tgtctttccc tc 3072

<210> 14
<211> 598
<212> DNA
<213> Homo sapiens

<400> 14
atttcttgag atggctcctc ttggaactat gcatctctcc tgatacttgg atgcttttcc 60
tctgactgat gaagatcctg aataccaaag agggccgctg aaagggctct aggagtacac 120
cttctaggaa ccctaagcca gagagaggct tcactacatc atgcttcctg acatctctcc 180
ctttgaagag cagtcagact cctgctttgc tcttcagact taatttgggg gtttaacagg 240
tgaggttgct gggggaactc ttttacaaca tctctctgaa agaatccggg ctgccagttt 300
catttggttt gggtgtcagt agcatgatgg aaagacaaaa aaacacaact tgacatctgc 360
agaaatgggt tcaaatttta cctgcaactc accaattctg tggccttggt tcagcaatta 420
aactccctaa aattcagttt tttctttgta aaatggggtt atgaacagta cctacttcaa 480

aatgtgtttg tgaagattaa aaaagttaac ataaagagtt tagaagagtg tctggcatat 540
tgtgctcaat aagtgtttat ttatttattg ctgaataaac cagtaattta attagtat 598

<210> 15
<211> 3418
<212> DNA
<213> Homo sapiens

<400> 15
caggaatttg tggcggagag ggcaaataac tgcggctctc ccggcgcccc gatgctcgca 60
ccatgtcgag gcgcaagcag gcgaaacccc agcacatcaa ctcggaggag gaccagggcg 120
agcagcagcc gcagcagcag accccggagt ttgcagatgc ggccccagcg gcgcccgcgg 180
cgggggagct gggtgctcca gtgaaccacc cagggaatga cgaggtggcg agtgaggatg 240
aagccacagt aaagcggctt cgtcgggagg agacgcacgt ctgtgagaaa tgctgtgcgg 300
agttcttcag catctctgag ttcctggaac ataagaaaaa ttgcactaaa aatccacctg 360
tcctcatcat gaatgacagc gaggggcctg tgccttcaga agacttctcc ggagctgtac 420
tgagccacca gcccaccagt cccggcagta aggactgtca cagggagaat ggcggcagct 480
cagaggacat gaaggagaag ccggatgcgg agtctgtggt gtacctaaag acagagacag 540
ccctgccacc caccccccag gacataagct atttagccaa aggcaaagtg gccaacacta 600
atgtgacctt gcaggcacta cggggcacca aggtggcggt gaatcagcgg agcgcggatg 660
cactccctgc ccccgtgcct ggtgccaaca gcatcccgtg ggtcctcgag cagatcttgt 720
gtctgcagca gcagcagcta cagcagatcc agctcaccga gcagatccgc atccaggtga 780
acatgtgggc ctcccacgcc ctccactcaa gcggggcagg ggccgacact ctgaagacct 840
tgggcagcca catgtctcag caggtttctg cagctgtggc tttgctcagc cagaaagctg 900
gaagccaagg tctgtctctg gatgccttga aacaagccaa gctacctcac gccaacatcc 960
cttctgccac cagctccctg tccccagggc tggcaccctt cactctgaag ccggatggga 1020
cccgggtgct cccgaacgtc atgtcccgcc tcccgagcgc tttgcttcct caggccccgg 1080
gctcggtgct cttccagagc cctttctcca ctgtggcgct agacacatcc aagaaaggga 1140
aggggaagcc accgaacatc tccgcggtgg atgtcaaacc caaagacgag gcggccctct 1200
acaagcacaa gtgtaagtac tgtagcaagg tttttgggac tgatagctcc ttgcagatcc 1260
acctccgctc ccacactgga gagagaccct tcgtgtgctc tgtctgtggt catcgcttca 1320
ccaccaaggg caacctcaag gtgcactttc accgacatcc ccaggtgaag gcaaaccccc 1380
agctgtttgc cgagttccag gacaaagtgg cggccggcaa tggcatcccc tatgcactct 1440
ctgtacctga ccccatagat gaaccgagtc tttctttaga cagcaaacct gtccttgtaa 1500
ccacctctgt agggctacct cagaatcttt cttcggggac taatcccaag gacctcacgg 1560
gtggctcctt gcccggtgac ctgcagcctg ggccttctcc agaaagtgag ggtggaccca 1620
cactccctgg ggtgggacca aactataatt ccccaagggc tggtggcttc caagggagtg 1680
ggacccctga gccagggtca gagaccctga aattgcagca gttggtggag aacattgaca 1740
aggccaccac tgatcccaac gaatgtctca tttgccaccg agtcttaagc tgtcagagct 1800
ccctcaagat gcattatcgc acccacaccg gggagagacc gttccagtgt aagatctgtg 1860
gccgagcctt ttctaccaaa ggtaacctga agacacacct tggggttcac cgaaccaaca 1920
catccattaa gacgcagcat tcgtgcccca tctgccagaa gaagttcact aatgccgtga 1980
tgctgcagca acatattcgg atgcacatgg gcggtcagat tcccaacacg cccctgccag 2040
agaatccctg tgactttacg ggttctgagc caatgaccgt gggtgagaac ggcagcaccg 2100
gcgctatctg ccatgatgat gtcatcgaaa gcatcgatgt agaggaagtc agctcccagg 2160
aggctcccag cagctcctcc aaggtcccca cgcctcttcc cagcatccac tcggcatcac 2220
ccacgctagg gtttgccatg atggcttcct tagatgcccc agggaaagtg ggtcctgccc 2280
cttttaacct gcagcgccag ggcagcagag aaaacggttc cgtggagagc gatggcttga 2340
ccaacgactc atcctcgctg atgggagacc aggagtatca gagccgaagc ccagatatcc 2400
tggaaaccac atccttccag gcactctccc cggccaatag tcaagccgaa agcatcaagt 2460
caaagtctcc cgatgctggg agcaaagcag agagctccga gaacagccgc actgagatgg 2520
aaggtcggag cagtctccct tccacgttta tccgagcccc gccgacctat gtcaaggttg 2580
aagttcctgg cacatttgtg ggaccctcga cattgtcccc agggatgacc cctttgttag 2640
cagcccagcc acgccgacag gccaagcaac atggctgcac acggtgtggg aagaacttct 2700
cgtctgctag cgctcttcag atccacgagc ggactcacac tggagagaag ccttttgtgt 2760
gcaacatttg tgggcgagct tttaccacca aaggcaactt aaaggttcac tacatgacac 2820
acggggcgaa caataactca gcccgccgtg gaaggaagtt ggccatcgag aacaccatgg 2880
ctctgttagg tacggacgga aaaagagtct cagaaatctt tcccaaggaa atcctggccc 2940
cttcagtgaa tgtggaccct gttgtgtgga accagtacac cagcatgctc aatggcggtc 3000
tggccgtgaa gaccaatgag atctctgtga tccagagtgg gggggttcct accctcccgg 3060
tttccttggg ggccacctcc gttgtgaata acgccactgt ctccaagatg gatggctccc 3120
agtcgggtat cagtgcagat gtggaaaaac caagtgctac tgacggcgtt cccaaacacc 3180
agtttcctca cttcctggaa gaaaacaaga ttgcggtcag ctaaggagaa acttgcgtgg 3240

aaggagcaat gcagacacag tgaaatctct agaatctgct ttgttttgta agaactcatc 3300
tcctcctgtt ttctttttct tactgatatg caaatgatgt ttactacgtt ggttgtgacc 3360
acaacctcag gcaagtgcta caatcacgat tgttgctatg ctgctttgca aaaagttg 3418

<210> 16
<211> 634
<212> DNA
<213> Homo sapiens

<400> 16
cggctgagag gcagcgaact catctttgcc agtacaggag cttgtgccgt ggcccacagc 60
ccacagccca cagccatggg ctgggacctg acggtgaaga tgctggcggg caacgaattc 120
caggtgtccc tgagcagctc catgtcggtg tcagagctga aggcgcagat cacccagaag 180

attggcgtgc acgccttcca gcagcgtctg gctgtccacc cgagcggtgt ggcgctgcag 240
gacagggtcc cccttgccag ccagggcctg ggccctggca gcacggtcct gctggtggtg 300
gacaaatgcg acgaacctct gagcatcctg gtgaggaata acaagggccg cagcagcacc 360
tacgaggtcc ggctgacgca gaccgtggcc cacctgaagc agcaagtgag cgggctggag 420
ggtgtgcagg acgacctgtt ctggctgacc ttcgagggga agcccctgga ggaccagctc 480
ccgctggggg agtacggcct caagcccctg agcaccgtgt tcatgaatct gcgcctgcgg 540
ggaggcggca cagagcctgg cgggcggagc taagggcctc caccagcatc cgagcaggat 600
caagggccgg aaataaaggc tgttgtaaga gaat 634

<210> 17
<211> 1422
<212> DNA
<213> Homo sapiens

<400> 17
cactgctctg agaatttgtg agcagcccct aacaggctgt tacttcacta caactgacga 60
tatgatcatc ttaatttact tatttctctt gctatgggaa gacactcaag gatggggatt 120
caaggatgga atttttcata actccatatg gcttgaacga gcagccggtg tgtaccacag 180
agaagcacgg tctggcaaat acaagctcac ctacgcagaa gctaaggcgg tgtgtgaatt 240
tgaaggcggc catctcgcaa cttacaagca gctagaggca gccagaaaaa ttggatttca 300
tgtctgtgct gctggatgga tggctaaggg cagagttgga taccccattg tgaagccagg 360
gcccaactgt ggatttggaa aaactggcat tattgattat ggaatccgtc tcaataggag 420
tgaaagatgg gatgcctatt gctacaaccc acacgcaaag gagtgtggtg gcgtctttac 480
agatccaaag caaattttta aatctccagg cttcccaaat gagtacgaag ataaccaaat 540
ctgctactgg cacattagac tcaagtatgg tcagcgtatt cacctgagtt ttttagattt 600
tgaccttgaa gatgacccag gttgcttggc tgattatgtt gaaatatatg acagttacga 660
tgatgtccat ggctttgtgg gaagatactg tggagatgag cttccagatg acatcatcag 720
tacaggaaat gtcatgacct tgaagtttct aagtgatgct tcagtgacag ctggaggttt 780
ccaaatcaaa tatgttgcaa tggatcctgt atccaaatcc agtcaaggaa aaaatacaag 840
tactacttct actggaaata aaaacttttt agctggaaga tttagccact tataaaaaaa 900
aaaaaaagga tgatcaaaac acacagtgtt tatgttggaa tcttttggaa ctcctttgat 960
ctcactgtta ttattaacat ttatttatta tttttctaaa tgtgaaagca atacataatt 1020
tagggaaaat tggaaaatat aggaaacttt aaacgagaaa atgaaacctc tcataatccc 1080
actgcataga aataacaagc gttaacattt tcatattttt ttctttcagt catttttcta 1140
tttgtggtat atgtatatat gtacctatat gtatttgcat ttgaaatttt ggaatcctgc 1200
tctatgtaca gttttgtatt atacttttta aatcttgaac tttataaaca ttttctgaaa 1260
tcattgatta ttctacaaaa acatgatttt aaacagctgt aaaatattct atgatatgaa 1320
tgttttatgc attatttaag cctgtctcta ttgttggaat ttcaggtcat tttcataaat 1380
attgttgcaa taaatatcct tgaacacaca aaaaaaaaaa aa 1422

<210> 18
<211> 1414
<212> DNA
<213> Homo sapiens

<400> 18
gaattcgcac tgctctgaga atttgtgagc agcccctaac aggctgttac ttcactacaa 60
ctgacgatat gatcatctta atttacttat ttctcttgct atgggaagac actcaaggat 120
ggggattcaa ggatggaatt tttcataact ccatatggct tgaacgagca gccggtgtgt 180

accacagaga agcacggtct ggcaaataca agctcaccta cgcagaagct aaggcggtgt 240
gtgaatttga aggcggccat ctcgcaactt acaagcagct agaggcagcc agaaaaattg 300
gatttcatgt ctgtgctgct ggatggatgg ctaagggcag agttggatac cccattgtga 360
agccagggcc caactgatga tttggaaaaa ctggcattat tgattatgga atccgtctca 420
ataggagtga aagatgggat gcctattgct acaacccaca cgcaaaggag tgtggtggcg 480
tctttacaga tccaaagcga atttttaaat ctccaggctt cccaaatgag tacgaagata 540
accaaatctg ctactggcac attagactca agtatggtca gcgtattcac ctgagttttt 600
tagattttga ccttgaagat gacccaggtt gcttggctga ttatgttgaa atatatgaca 660
gttacgatga tgtccatggc tttgtgggaa gatactgtgg agatgagctt ccagatgaca 720
tcatcagtac aggaaatgtc atgaccttga agtttctaag tgatgcttca gtgacagctg 780
gaggtttcca aatcaaatat gttgcaatgg atcctgtatc caaatccagt caaggaaaaa 840
atacaagtac tacttctact ggaaataaaa actttttagc tggaagattt agccacttat 900
aaaaaaaaaa aaggatgatc aaaacacaca gtgtttatgt tggaatcttt tggaactcct 960
ttgatctcac tgttattatt aacatttatt tattattttt ctaaatgtga aagaaataca 1020
taatttaggg aaaattggaa aatataggaa actttaaacg agaaaatgaa acctctcata 1080
atcccactgc atagaaataa caagcgttaa cattttcata tttttttctt tcagtcattt 1140
ttgtatttgt ggtatatgta tatatgtacc tatatgtatt tgcatttgaa attttggaat 1200
cctgctctat gtacagtttt gtattatact ttttaaatct tgaactttat gaacattttc 1260
tgaaatcatt gattattcta caaaaacatg attttaaaca gctgtaaaat attctatgat 1320
atgaatgttt tatgcattat ttaagcctgt ctctattgtt ggaatttcag gtcattttca 1380
taaatattgt tgcaataaat atccttcgga attc 1414

<210> 19
<211> 777
<212> DNA
<213> Homo sapiens

<400> 19
ggccccttgt ctgcagagat ggctcccaat gcttcctgcc tctgtgtgca tgtccgttcc 60
gaggaatggg atttaatgac ctttgatgcc aacccatatg acagcgtgaa aaaaatcaaa 120
gaacatgtcc ggtctaagac caaggttcct gtgcaggacc aggttctttt gctgggctcc 180
aagatcttaa agccacggag aagcctctca tcttatggca ttgacaaaga gaagaccatc 240
caccttaccc tgaaagtggt gaagcccagt gatgaggagc tgcccttgtt tcttgtggag 300
tcaggtgatg aggcaaagag gcacctcctc caggtgcgaa ggtccagctc agtggcacaa 360
gtgaaagcaa tgatcgagac taagacgggt ataatccctg agacccagat tgtgacttgc 420
aatggaaaga gactggaaga tgggaagatg atggcagatt acggcatcag aaagggcaac 480
ttactcttcc tggcatctta ttgtattgga gggtgaccac cctggggatg gggtgttggc 540
aggggtcaaa aagcttattt cttttaatct cttactcaac gaacacatct tctgatgatt 600
tcccaaaatt aatgagaatg agatgagtag agtaagattt gggtgggatg ggtaggatga 660
agtatattgc ccaactctat gtttctttga ttctaacaca attaattaag tgacatgatt 720
tttactaatg tattactgag actagtaaat aaatttttaa ggcaaaatag agcattc 777

<210> 20
<211> 1390
<212> DNA
<213> Homo sapiens

<400> 20
gggaagtgct gttggagccg ctgtggttgc tgtccgcgga gtggaagcgc gtgcttttgt 60
ttgtgtccct ggccatggcg ctgcagctct cccgggagca gggaatcacc ctgcgcggga 120
gcgccgaaat cgtggccgag ttcttctcat tcggcatcaa cagcatttta tatcagcgtg 180
gcatatatcc atctgaaacc tttactcgag tgcagaaata cggactcacc ttgcttgtaa 240
ctactgatct tgagctcata aaatacctaa ataatgtggt ggaacaactg aaagattggt 300
tatacaagtg ttcagttcag aaactggttg tagttatctc aaatattgaa agtggtgagg 360
tcctggaaag atggcagttt gatattgagt gtgacaagac tgcaaaagat gacagtgcac 420
ccagagaaaa gtctcagaaa gctatccagg atgaaatccg ttcagtgatc agacagatca 480
cagctacggt gacatttctg ccactgttgg aagtttcttg ttcatttgat ctgctgattt 540
atacagacaa agatttggtt gtacctgaaa aatgggaaga gtcgggacca cagtttatta 600
ccaattctga ggaagtccgc cttcgttcat ttactactac aatccacaaa gtaaatagca 660
tggtggccta caaaattcct gtcaatgact gaggatgaca tgaggaaaat aatgtaattg 720
taattttgaa atgtggtttt cctgaaatca ggtcatctat agttgatatg ttttatttca 780
ttggttaatt tttacatgga gaaaaccaaa atgatactta ctgaactgtg tgtaattgtt 840
cctttatttt tttggtacct atttgactta ccatggagtt aacatcatga atttattgca 900

cattgttcaa aaggaaccag gaggtttttt tgtcaacatt gtgatgtata ttcctttgaa 960
gatagtaact gtagatggaa aaacttgtgc tataaagcta gatgctttcc taaatcagat 1020
gttttggtca agtagtttga ctcagtatag gtagggagat atttaagtat aaaatacaac 1080
aaaggaagtc taaatattca gaatctttgt taaggtcctg aaagtaactc ataatctata 1140
aacaatgaaa tattgctgta tagctccttt tgaccttcat ttcatgtata gttttcccta 1200
ttgaatcagt ttccaattat ttgactttaa tttatgtaac ttgaacctat gaagcaatgg 1260
atatttgtac tgtttaatgt tctgtgatac agaactctta aaaatgtttt ttcatgtgtt 1320
ttataaaatc aagttttaag tgaaagtgag gaaataaagt taagtttgtt ttaaaaaaaa 1380
aaaaaaaaaa 1390

<210> 21
<211> 736
<212> DNA
<213> Homo sapiens

<400> 21
ggctctcacc ctcctctcct gcagctccag ctttgtgctc tgcctctgag gagaccatgg 60
cccagtatct gagtaccctg ctgctcctgc tggccaccct agctgtggcc ctggcctgga 120
gccccaagga ggaggatagg ataatcccgg gtggcatcta taacgcagac ctcaatgatg 180
agtgggtaca gcgtgccctt cacttcgcca tcagcgagta taacaaggcc accaaagatg 240
actactacag acgtccgctg cgggtactaa gagccaggca acagaccgtt gggggggtga 300
attacttctt cgacgtagag gtgggccgca ccatatgtac caagtcccag cccaacttgg 360
acacctgtgc cttccatgaa cagccagaac tgcagaagaa acagttgtgc tctttcgaga 420
tctacgaagt tccctgggag aacagaaggt ccctggtgaa atccaggtgt caagaatcct 480
agggatctgt gccaggccat tcgcaccagc caccacccac tcccaccccc tgtagtgctc 540
ccacccctgg actggtggcc cccaccctgc gggaggcctc cccatgtgcc tgcgccaaga 600
gacagacaga gaaggctgca ggagtccttt gttgctcagc agggcgctct gccctccctc 660
cttccttctt gcttctaata gccctggtac atggtacaca cccccccacc tcctgcaatt 720
aaacagtagc atcgcc 736

<210> 22
<211> 4520
<212> DNA
<213> Homo sapiens

<400> 22
taggaacagg ggagagtgca cctgctacca gtcaagctca gccagactgc aagaggaggc 60
gaggcggagc cagccgaggg agtgaaccat ggacaagttg aaatgcccga gtttcttcaa 120
gtgcagggag aaggagaaag tgtcggcttc atcagagaat ttccatgttg gtgaaaatga 180
tgagaatcag gaccgtggta actggtccaa aaaatcggat tatcttctat ctatgattgg 240
atacgcagtg ggattaggaa atgtgtggag atttccatat ctgacctaca gcaatggtgg 300
aggcgccttc ttgatacctt atgcaattat gttagcattg gctggtttac ctttgttctt 360
tctggagtgt tcactgggac aatttgctag cttaggtcca gtttcagttt ggaggattct 420
tccattgttt caaggtgtgg gaattacaat ggtcctgatc tccatttttg tgacaatcta 480
ttacaatgtc ataattgcct atagtcttta ctacatgttt gcttcttttc aaagtgaact 540
accatggaaa aattgttctt cgtggtcaga taaaaactgt agcagatcac caatagtaac 600
tcactgtaat gtgagtacag tgaataaagg aatacaagag atcatccaaa tgaataaaag 660
ctgggtagac atcaacaatt ttacctgcat caacggcagt gaaatttatc agccaggcca 720
gcttcccagt gaacaatatt ggaataaagt ggcgctccaa cggtcaagtg gaatgaatga 780
gactggagta attgtttggt atttagcact ttgtcttctt ctggcttggc tcatagttgg 840
agcagcacta tttaaaggaa tcaaatcgtc tggcaaggtg gtatatttta cagctctttt 900
cccctatgtg gtcctactca tcctgttagt acgaggtgca actctggagg gtgcttcaaa 960
aggcatttca tactatattg gagcccagtc aaattttaca aaacttaagg aagctgaggt 1020
atggaaagat gctgccactc agatatttta ctccctttca gtggcttggg gtggcttagt 1080
tgctctatca tcttacaata agttcaaaaa caactgcttc tctgatgcca ttgtggtttg 1140
tttgacaaac tgtctcacta gcgtgtttgc tggatttgct attttttcta tattgggaca 1200
catggcccat atatctggaa aggaagtttc tcaagttgta aaatcaggtt ttgatttggc 1260
attcattgcc tatccagagg ctctagccca actcccaggt ggtccatttt ggtccatatt 1320
attttttttc atgcttttaa ctttgggtct cgattctcag tttgcttcga ttgaaacgat 1380
cacaacaaca attcaagatt tatttcccaa agtgatgaag aaaatgaggg ttcccataac 1440
tttgggctgc tgcttggttt tgtttctcct tggtctcgtc tgtgtgactc aggctggaat 1500
ttactgggtt catctgattg accacttctg tgctggatgg ggcattttaa ttgcagctat 1560

actggagcta gttggaatca tctggattta tggagggaac agattcattg aggatacaga 1620
aatgatgatt ggagcaaaga ggtggatatt ctggctatgg tggagagctt gctggtttgt 1680
aattacgcct atccttttga ttgcaatatt tatctggtca ttggtgcaat ttcatagacc 1740
taattatggc gcaattccat accctgactg gggagttgct ttaggctggt gtatgattgt 1800
tttctgcatt atttggatac caattatggc tatcataaaa ataattcagg ctaaaggaaa 1860
catctttcaa cgccttataa gttgctgcag accagcttct aactggggtc catacctgga 1920
acaacatcgt ggggaaagat ataaagacat ggtagatcct aaaaaagagg ctgaccatga 1980
aatacctact gttagtggca gcagaaaacc ggaatgagat ctcattgaaa aaaatatatg 2040
attgtataat gtgatttttt ttagaatagg gggaacctta tttatttgtg tgttaactga 2100
ataggaaaat gtacatacta tgttcatgat agtgtgattt ttttcacatt taagcaggaa 2160
tgcaatataa aaatgtgaat ctcttaattc tcagccatgt gcttattata tttcttttta 2220
gattgtctat ctgtataaca cacacacaca cacctaagag tctctatttc acaattatat 2280
ttttgtaaat agtatatgca tttttaatac attggaggct ttattttgaa ctaatttctt 2340
agagaatagt tatattttct attacacaag tttaaaaata ttattaactt gtattttctt 2400
aatatacaat ctatcttttc cacaaatatg agtgggaaat aaatcagcac atttgaaaga 2460
aagtgttaaa actgaaggcc tcacttaatt agaaacgtga taaatatatg gacaaatgga 2520
ctatacatac tataagagga ctgtagttta atacttttta cccaaatatg tttaaaaaca 2580
tcgtgcattt gttacagctc atgttttcta tatgaactta gtcattaatg ttctttataa 2640
aaagtgaaat aagatggaaa aattaggatc ctacagccag tacgtgataa atctagaaaa 2700
ttgagttttg agtacctctt ttcccatata caatcttcct tccttaggta atttggaaga 2760
aaactatgac ccatttaatt tctattgtgt ttcaccaaat tcagtgttgt tcattatacc 2820
tctctgaaat ataggtttaa tttcaaatag aatatggact taaatgttaa tgagaaactg 2880
gctttaatca attctagcat tttattactg taatacaggg ctgatagagt gattttgtct 2940
tatatgagtc agttactact tacaggtgat aacttgcata ctattggaag ataaagttgt 3000
caaacttgtc aagaatgaga aaagccaaat tagaaaatcc tatgtcctag tttccttacc 3060
aaggataatt aaatatatca ctaagagctt tatatattga ttatatattg ttgacaactg 3120
gtttaagcat catagcctat gatgataaac actgcctata tatgtaaata gcttttcatc 3180
aattcttaaa tttcttaacc taggcttcag ggagcatatg aaaccaaaat tatatggaac 3240
attttctgtg tgtacatgta catgcatttt tctagggaga gagtccgtag gtttatcaga 3300
atatcaagga aaactgtgac ccaaagaagt ttaagaatca catacagtgc tgctggcttt 3360
ttgtgcttgg caaatgagtg acaatagaag aaataatttt tcttacacat tttaaaacgt 3420
tttctcttcc ttgtgattga agatgaaagg agtaagaaat taaggcattt gtttaattta 3480
tactggtaac ttatttaggg gggaggggac atgaaggtag gtaaataggt aggcctctaa 3540
ttgaaccacc tctctaagtt atgtacgtat atataagctg aaattgtgtt tgacattctg 3600
agggttttct ttttcttttt cctttttttt tttttttggt ggggggctgg gggtcagagt 3660
cttgttctgt tgcctgggct ggagtgcagt ggcatgatct cagctcactg caacctctgc 3720
cttctggatt caagtgattc tcctgcctca gcctcttgag tagctgggac tacaggtgcc 3780
cgccaccaca ccagctaatt tttgtatttt tagtagaggc gaagtttccc catgttggcc 3840
aggctggtct tgaactcccg acctcaagtg atctgtctac ctcggcctcc taaagtgctg 3900
agattacagg tgtgagccac cgtgcccggc ccattctaag ggttttcttt gaagacaggt 3960
caaatgctgt tagtaagttt caggagattg ttaattcctc agttatacca gattttataa 4020
aatatttgag aatagatggc taacaagagg ttagaaatac ttttccttaa ttttaatcca 4080
cagtatgtta catgcattct accactacat tttggtgcta tttaaggtgt gcaattttct 4140
ataggtgact tttgcaattc agggaagatt tgggcatatt aaatgaaaga atatctaatt 4200
gggggaggtg tgaaggaaaa gaaattcttt tcaaaagctg accacaaaga gtagttaaaa 4260
gtttttgtca ctatcttcac aagtgtgtaa agcacagatt tcaacagagt gcttggcata 4320
ttgtagggtg ctcaatggtg gtttttatta ttattactca gattccacag tggcaagaaa 4380
catcattcta cataatggaa aacatttaca tcaaatccca cttactttaa tgcgaacttg 4440
gagataattt atggtattgt attgtaaacc attaatgaaa actttttcac agttgagtga 4500
aattaaaatc actatatctc 4520

<210> 23
<211> 777
<212> DNA
<213> Homo sapiens

<400> 23
ggccccttgt ctgcagagat ggctcccaat gcttcctgcc tctgtgtgca tgtccgttcc 60
gaggaatggg atttaatgac ctttgatgcc aacccatatg acagcgtgaa aaaaatcaaa 120
gaacatgtcc ggtctaagac caaggttcct gtgcaggacc aggttctttt gctgggctcc 180
aagatcttaa agccacggag aagcctctca tcttatggca ttgacaaaga gaagaccatc 240
caccttaccc tgaaagtggt gaagcccagt gatgaggagc tgcccttgtt tcttgtggag 300
tcaggtgatg aggcaaagag gcacctcctc caggtgcgaa ggtccagctc agtggcacaa 360

gtgaaagcaa tgatcgagac taagacgggt ataatccctg agacccagat tgtgacttgc 420
aatggaaaga gactggaaga tgggaagatg atggcagatt acggcatcag aaagggcaac 480
ttactcttcc tggcatctta ttgtattgga gggtgaccac cctggggatg gggtgttggc 540
aggggtcaaa aagcttattt cttttaatct cttactcaac gaacacatct tctgatgatt 600
tcccaaaatt aatgagaatg agatgagtag agtaagattt gggtgggatg ggtaggatga 660
agtatattgc ccaactctat gtttctttga ttctaacaca attaattaag tgacatgatt 720
tttactaatg tattactgag actagtaaat aaatttttaa ggcaaaatag agcattc 777

<210> 24
<211> 948
<212> DNA
<213> Homo sapiens

<400> 24
atgaaggcca actacagcgc agaggagcgc tttctcctgc tgggtttctc cgactggcct 60
tccctgcagc cggtcctctt cgcccttgtc ctcctgtgct acctcctgac cttgacgggc 120
aactcggcgc tggtgctgct ggcggtgcgc gacccgcgcc tgcacacgcc catgtactac 180
ttcctctgcc acctggcctt ggtagacgcg ggcttcacta ctagcgtggt gccgccgctg 240
ctggccaacc tgcgcggacc agcgctctgg ctgccgcgca gccactgcac ggcccagctg 300
tgcgcatcgc tggctctggg ttcggccgaa tgcgtcctcc tggcggtgat ggctctggac 360
cgcgcggccg cagtgtgccg cccgctgcgc tatgcggggc tcgtctcccc gcgcctatgt 420
cgcacgctgg ccagcgcctc ctggctaagc ggcctcacca actcggttgc gcaaaccgcg 480
ctcctggctg agcggccgct gtgcgcgccc cgcctgctgg accacttcat ctgtgagctg 540
ccggcgttgc tcaagctggc ctgcggaggc gacggagaca ctaccgagaa ccagatgttc 600
gccgcccgcg tggtcatcct gctgctgccg tttgccgtca tcctggcctc ctacggtgcc 660
gtggcccgag ctgtctgttg catgcggttc agcggaggcc ggaggagggc ggtgggcacg 720
tgtgggtccc acctgacagc cgtctgcctg ttctacggct cggccatcta cacctacctg 780
cagcccgcgc agcgctacaa ccaggcacgg ggcaagttcg tatcgctctt ctacaccgtg 840
gtcacacctg ctctcaaccc gctcatctac accctcagga ataagaaagt gaagggggca 900
gcgaggaggc tgctgcggag tctggggaga ggccaggctg ggcagtga 948

<210> 25
<211> 4722
<212> DNA
<213> Homo sapiens

<400> 25
ctggaagccg agaggagagg acagctggtt gtgggagagt tcccccgcct cagactcctg 60
gttttttcca ggagacacac tgagctgaga ctcacttttc tcttcctgaa tttgaaccac 120
cgtttccatc gtctcgtagt ccgacgcctg ggcgatggga tccgtttacg gagaaactgc 180
tggagcgaac ccgtgccagg cgagagaatc ttcagagaaa aatggctgag aggcccacag 240
cagctccaag gtctatgact catgctaagc gagctagaca gccactttca gaagcaagta 300
accagcagcc cctctctggt ggtgaagaga aatcttgtac aaaaccatcg ccatcaaaaa 360
aacgctgttc tgacaacact gaagtagaag tttctaactt ggaaaataaa caaccagttg 420
agtcgacatc tgcaaaatct tgttctccaa gtcctgtgtc tcctcaggtg cagccacaag 480
cagcagatac catcagtgat tctgttgctg tcccggcatc actgctgggc atgaggagag 540
ggctgaactc aagattggaa gcaactgcag cctcctcagt taaaacacgt atgcaaaaac 600
ttgcagagca acggcgccgt tgggataatg atgatatgac agatgacatt cctgaaagct 660
cactcttctc accaatgcca tcagaggaaa aggctgcttc ccctcccaaa cctctgcttt 720
caaatgcctc ggcaactcca gttggcagaa ggggccgtct ggccaatctt gctgcaacta 780
tttgctcctg ggaagatgat gtaaatcact catttgcaaa acaaaacagt gtacaagaac 840
agcctggtac cgcttgttta tccaaatttt cctctgcaag tggagcatct gctaggatca 900
atagcagcag tgttaagcag gaagctacat tctgttccca aagggatggc gatgcctctt 960
tgaataaagc cctatcctca agtgctgatg atgcgtcttt ggttaatgcc tcaatttcca 1020
gctctgtgaa agctacttct tctccagtga aatctactac atctatcact gatgctaaaa 1080
gttgtgaggg acaaaatcct gagctacttc caaaaactcc tattagtcct ctgaaaacgg 1140
gggtatcgaa accaattgtg aagtcaactt tatcccagac agttccatcc aagggagaat 1200
taagtagaga aatttgtctg caatctcaat ctaaagacaa atctacgaca ccaggaggaa 1260
caggaattaa gcctttcctg gaacgctttg gagagcgttg tcaagaacat agcaaagaaa 1320
gtccagctcg tagcacaccc cacagaaccc ccattattac tccaaataca aaggccatcc 1380
aagaaagatt attcaagcaa gacacatctt catctactac ccatttagca caacagctca 1440
agcaggaacg tcaaaaagaa ctagcatgtc ttcgtggccg atttgacaag ggcaatatat 1500
ggagtgcaga aaaaggcgga aactcaaaaa gcaaacaact agaaaccaaa caggaaactc 1560

actgtcagag cactcccctc aaaaaacacc aaggtgtttc aaaaactcag tcacttccag 1620
taacagaaaa ggtgaccgaa aaccagatac cagccaaaaa ttctagtaca gaacctaaag 1680
gtttcactga atgcgaaatg acgaaatcta gccctttgaa aataacattg tttttagaag 1740
aggacaaatc cttaaaagta acatcagacc caaaggttga gcagaaaatt gaagtgatac 1800
gtgaaattga gatgagtgtg gatgatgatg atatcaatag ttcgaaagta attaatgacc 1860
tcttcagtga tgtcctagag gaaggtgaac tagatatgga gaagagccaa gaggagatgg 1920
atcaagcatt agcagaaagc agcgaagaac aggaagatgc actgaatatc tcctcaatgt 1980
ctttacttgc accattggca caaacagttg gtgtggtaag tccagagagt ttagtgtcca 2040
cacctagact ggaattgaaa gacaccagca gaagtgatga aagtccaaaa ccaggaaaat 2100
tccaaagaac tcgtgtccct cgagctgaat ctggtgatag ccttggttct gaagatcgtg 2160
atcttcttta cagcattgat gcatatagat ctcaaagatt caaagaaaca gaacgtccat 2220
caataaagca ggtgattgtt cggaaggaag atgttacttc aaaactggat gaaaaaaata 2280
atgcctttcc ttgtcaagtt aatatcaaac agaaaatgca ggaactcaat aacgaaataa 2340
atatgcaaca gacagtgatc tatcaagcta gccaggctct taactgctgt gttgatgaag 2400
aacatggaaa agggtcccta gaagaagctg aagcagaaag acttcttcta attgcaactg 2460
ggaagagaac acttttgatt gatgaattga ataaattgaa gaacgaagga cctcagagga 2520
agaataaggc tagtccccaa agtgaattta tgccatccaa aggatcagtt actttgtcag 2580
aaatccgctt gcctctaaaa gcagattttg tctgcagtac ggttcagaaa ccagatgcag 2640
caaattacta ttacttaatt atactaaaag caggagctga aaatatggta gccacaccat 2700
tagcaagtac ttcaaactct cttaacggtg atgctctgac attcactact acatttactc 2760
tgcaagatgt atccaatgac tttgaaataa atattgaagt ttacagcttg gtgcaaaaga 2820
aagatccctc aggccttgat aagaagaaaa aaacatccaa gtccaaggct attactccaa 2880
agcgactcct cacatctata accacaaaaa gcaacattca ttcttcagtc atggccagtc 2940
caggaggtct tagtgctgtg cgaaccagca acttcgccct tgttggatct tacacattat 3000
cattgtcttc agtaggaaat actaagtttg ttctggacaa ggtccccttt ttatcttctt 3060
tggaaggtca tatttattta aaaataaaat gtcaagtgaa ttccagtgtt gaagaaagag 3120
gttttctaac catatttgaa gatgttagtg gttttggtgc ctggcatcga agatggtgtg 3180
ttctttctgg aaactgtata tcttattgga cttatccaga tgatgagaaa cgcaagaatc 3240
ccataggaag gataaatctg gctaattgta ccagtcgtca gatagaacca gccaacagag 3300
aattttgtgc aagacgcaac acttttgaat taattactgt ccgaccacaa agagaagatg 3360
accgagagac tcttgtcagc caatgcaggg acacactctg tgttaccaag aactggctgt 3420
ctgcagatac taaagaagag cgggatctct ggatgcaaaa actcaatcaa gttcttgttg 3480
atattcgcct ctggcaacct gatgcttgct acaaacctat tggaaagcct taaaccggga 3540
aatttccatg ctatctagag gtttttgatg tcatcttaag aaacacactt aagagcatca 3600
gatttactga ttgcatttta tgctttaagt acgaaagggt ttgtgccaat attcactacg 3660
tattatgcag tatttatatc ttttgtatgt aaaactttaa ctgatttctg tcattcatca 3720
atgagtagaa gtaaatacat tatagttgat tttgctaaat cttaatttaa aagcctcatt 3780
ttcctagaaa tctaattatt cagttattca tgacaatatt tttttaaaag taagaaattc 3840
tgagttgtct tcttggagct gtaggtcttg aagcagcaac gtctttcagg ggttggagac 3900
agaaacccat tctccaatct cagtagtttt ttcgaaaggc tgtgatcatt tattgatcgt 3960
gatatgactt gttactaggg tactgaaaaa aatgtctaag gcctttacag aaacattttt 4020
agtaatgagg atgagaactt tttcaaatag caaatatata ttggcttaaa gcatgaggct 4080
gtcttcagaa aagtgatgtg gacataggag gcaatgtgtg agacttgggg gttcaatatt 4140
ttatatagaa gagttaataa gcacatggtt tacatttact cagctactat atatgcagtg 4200
tggtgcacat tttcacagaa ttctggcttc attaagatca ttatttttgc tgcgtagctt 4260
acagacttag catattagtt ttttctactc ctacaagtgt aaattgaaaa atctttatat 4320
taaaaaagta aactgttatg aagctgctat gtactaataa tactttgctt gccaaagtgt 4380
ttgggttttg ttgttgtttg tttgtttgtt tgtttttggt tcatgaacaa cagtgtctag 4440
aaacccattt tgaaagtgga aaattattaa gtcacctatc acctttaaac gccttttttt 4500
aaaattataa aatattgtaa agcagggtct caacttttaa atacactttg aacttcttct 4560
ctgaattatt aaagttcttt atgacctcat ttataaacac taaattctgt cacctcctgt 4620
cattttattt tttattcatt caaatgtatt ttttcttgtg catattataa aaatatattt 4680
tatgagctct tactcaaata aatacctgta aatgtctaaa gg 4722

<210> 26
<211> 3916
<212> DNA
<213> Homo sapiens

<400> 26
agagtgctga tttagaagaa tacaaatcat ggctgaaaat agtgtattaa catccactac 60
tgggaggact agcttggcag actcttccat ttttgattct aaagttactg agatttccaa 120
ggaaaactta cttattggat ctacttcata tgtagaagaa gagatgcctc agattgaaac 180

aagagtgata ttggttcaag aagctggaaa acaagaagaa cttataaaag ccttaaagga 240
cattaaagtg ggctttgtaa agatggagtc agtggaagaa tttgaaggtt tggattctcc 300
ggaatttgaa aatgtatttg tagtcacgga ctttcaggat tctgtcttta atgacctcta 360
caaggctgat tgtagagtta ttggaccacc agttgtatta aattgttcac aaaaaggaga 420
gcctttgcca ttttcatgtc gcccgttgta ttgtacaagt atgatgaatc tagtactatg 480
ctttactgga tttaggaaaa aagaagaact agtcaggttg gtgacattgg tccatcacat 540
gggtggagtt attcgaaaag actttaattc aaaagttaca catttggtgg caaattgtac 600
acaaggagaa aaattcaggg ttgctgtgag tctaggtact ccaattatga agccagaatg 660
gatttataaa gcttgggaaa ggcggaatga acaggatttc tatgcagcag ttgatgactt 720
tagaaatgaa tttaaagttc ctccatttca agattgtatt ttaagtttcc tgggattttc 780
agatgaagag aaaaccaata tggaagaaat gactgaaatg caaggaggta aatatttacc 840
gcttggagat gaaagatgca ctcaccttgt agttgaagag aatatagtaa aagatcttcc 900
ctttgaacct tcaaagaaac tttatgttgt caagcaagag tggttctggg gaagcattca 960
aatggatgcc cgagctggag aaactatgta tttatatgaa aaggcaaata ctcctgagct 1020
caagaaatca gtgtcaatgc tttctctaaa tacccctaac agcaatcgca aacgacgtcg 1080
tttaaaagaa acacttgctc agctttcaag agagacagac gtgtcaccat ttccaccccg 1140
taagcgccca tcagctgagc attccctttc catagggtca ctcctagata tctccaacac 1200
accagagtct agcattaact atggagacac cccaaagtct tgtactaagt cttctaaaag 1260
ctccactcca gttccttcaa agcagtcagc aaggtggcaa gttgcaaaag agctttatca 1320
aactgaaagt aattatgtta atatattggc aacaattatt cagttatttc aagtaccatt 1380
ggaagaggaa ggacaacgtg gtggacctat ccttgcacca gaggagatta agactatttt 1440
tggtagcatc ccagatatct ttgatgtaca cactaagata aaggatgatc ttgaagacct 1500
tatagttaat tgggatgaga gcaaaagcat tggtgacatt tttctgaaat attcaaaaga 1560
tttggtaaaa acctaccctc cctttgtaaa cttctttgaa atgagcaagg aaacaattat 1620
taaatgtgaa aaacagaaac caagatttca tgcttttctc aagataaacc aagcaaaacc 1680
agaatgtgga cggcagagcc ttgttgaact tcttatccga ccagtacaga ggttacccag 1740
tgttgcatta cttttaaatg atcttaagaa gcatacagct gatgaaaatc cagacaaaag 1800
cactttagaa aaagctattg gatcactgaa ggaagtaatg acgcatatta atgaggataa 1860
gagaaaaaca gaagctcaaa agcaaatttt tgatgttgtt tatgaagtag atggatgccc 1920
agctaatctt ttatcttctc accgaagctt agtacagcgg gttgaaacaa tttctctagg 1980
tgagcacccc tgtgacagag gagaacaagt aactctcttc ctcttcaatg attgcctaga 2040
gatagcaaga aaacggcaca aggttattgg cacttttagg agtcctcatg gccaaacccg 2100
acccccagct tctcttaagc atattcacct aatgcctctt tctcagatta agaaggtatt 2160
ggacataaga gagacagaag attgccataa tgcttttgcc ttgcttgtga ggccaccaac 2220
agagcaggca aatgtgctac tcagtttcca gatgacatca gatgaacttc caaaagaaaa 2280
ctggctaaag atgctgtgtc gacatgtagc taacaccatt tgtaaagcag atgctgagaa 2340
tcttatttat actgctgatc cagaatcctt tgaagtaaat acaaaagata tggacagtac 2400
attgagtaga gcatcaagag caataaaaaa gacttcaaaa aaggttacaa gagcattctc 2460
tttctccaaa actccaaaaa gagctcttcg aagggctctt atgacatccc acggctcagt 2520
ggagggaaga agtccttcca gcaatgataa gcatgtaatg agtcgtcttt ctagcacatc 2580
atcattagca ggtatccctt ctccctccct tgtcagcctt ccttccttct ttgaaaggag 2640
aagtcatacg ttaagtagat ctacaactca tttgatatga agcgttacca aaatcttaaa 2700
ttatagaaat gtatagacac ctcatactca aataagaaac tgacttaaat ggtacttgta 2760
attagcactt ggtgaaagct ggaaggaaga taaataacac taaactatgc tatttgattt 2820
ttcttcttga aagagtaagg tttacctgtt acattttcaa gttaattcat gtaaaaaatg 2880
atagtgattt tgatgtaatt tatctcttgt ttgaatctgt cattcaaagg ccaataattt 2940
aagttgctat cagctgatat tagtagcttt gcaaccctga tagagtaaat aaattttatg 3000
ggcgggtgcc aaatactgct gtgaatctat ttgtatagta tccatgaatg aatttatgga 3060
aatagatatt tgtgcagctc aatttatgca gagattaaat gacatcataa tactggatga 3120
aaacttgcat agaattctga ttaaatagtg ggtctgtttc acatgtgcag tttgaagtat 3180
ttaaataacc actcctttca cagtttattt tcttctcaag cgttttcaag atctagcatg 3240
tggattttaa aagatttgcc ctcattaaca agaataacat ttaaaggaga ttgtttcaaa 3300
atatttttgc aaattgagat aaggacagaa agattgagaa acattgtata ttttgcaaaa 3360
acaagatgtt tgtagctgtt tcagagagag tacggtatat ttatggtaat tttatccact 3420
agcaaatctt gatttagttt gatagtgtgt ggaattttat tttgaaggat aagaccatgg 3480
gaaaattgtg gtaaagactg tttgtaccct tcatgaaata attctgaagt tgccatcagt 3540
tttactaatc ttctgtgaaa tgcatagata tgcgcatgtt caacttttta ttgtggtctt 3600
ataattaaat gtaaaattga aaattcattt gctgtttcaa agtgtgatat ctttcacaat 3660
agccttttta tagtcagtaa ttcagaataa tcaagttcat atggataaat gcatttttat 3720
ttcctatttc tttagggagt gctacaaatg tttgtcactt aaatttcaag tttctgtttt 3780
aatagttaac tgactataga ttgttttcta tgccatgtat gtgccacttc tgagagtagt 3840
aaatgactct ttgctacatt ttaaaagcaa ttgtattagt aagaactttg taaataaata 3900
cctaaaaccc aagtgt 3916

<210> 27
<211> 2119
<212> DNA
<213> Homo sapiens

<400> 27
ggcacgagta ggggtggcgg gtcagtgctg ctcgggggct tctccatcca ggtccctgga 60
gttcctggtc cctggagctc cgcacttggc gcgcaacctg cgtgaggcag cgcgactctg 120
gcgactggcc ggccatgcct tcccgggctg aggactatga agtgttgtac accattggca 180
caggctccta cggccgctgc cagaagatcc ggaggaagag tgatggcaag atattagttt 240
ggaaagaact tgactatggc tccatgacag aagctgagaa acagatgctt gtttctgaag 300
tgaatttgct tcgtgaactg aaacatccaa acatcgttcg ttactatgat cggattattg 360
accggaccaa tacaacactg tacattgtaa tggaatattg tgaaggaggg gatctggcta 420
gtgtaattac aaagggaacc aaggaaaggc aatacttaga tgaagagttt gttcttcgag 480
tgatgactca gttgactctg gccctgaagg aatgccacag acgaagtgat ggtggtcata 540
ccgtattgca tcgggatctt aaaccagcca atgttttcct ggatggcaag caaaacgtca 600
agcttggaga ctttgggcta gctagaatat taaaccatga cacgagtttt gcaaaaacat 660
ttgttggcac accttattac atgtctcctg aacaaatgaa tcgcatgtcc tacaatgaga 720
aatcagatat ctggtcattg ggctgcttgc tgtatgagtt atgtgcatta atgcctccat 780
ttacagcttt tagccagaaa gaactcgctg ggaaaatcag agaaggcaaa ttcaggcgaa 840
ttccataccg ttactctgat gaattgaatg aaattattac gaggatgtta aacttaaagg 900
attaccatcg accttctgtt gaagaaattc ttgagaaccc tttaatagca gatttggttg 960
cagacgagca aagaagaaat cttgagagaa gagggcgaca attaggagag ccagaaaaat 1020
cgcaggattc cagccctgta ttgagtgagc tgaaactgaa ggaaattcag ttacaggagc 1080
gagagcgagc tctcaaagca agagaagaaa gattggagca gaaagaacag gagctttgtg 1140
ttcgtgagag actagcagag gacaaactgg ctagagcaga aaatctgttg aagaactaca 1200
gcttgctaaa ggaacggaag ttcctgtctc tggcaagtaa tccagaactt cttaatcttc 1260
catcctcagt aattaagaag aaagttcatt tcagtgggga aagtaaagag aacatcatga 1320
ggagtgagaa ttctgagagt cagctcacat ctaagtccaa gtgcaaggac ctgaagaaaa 1380
ggcttcacgc tgcccagctg cgggctcaag ccctgtcaga tattgagaaa aattaccaac 1440
tgaaaagcag acagatcctg ggcatgcgct agccaggtag agagacacag agctgtgtac 1500
aggatgtaat attaccaacc tttaaagact gatattcaaa tgctgtagtg ttgaatactt 1560
ggccccatga gccatgcctt tctgtatagt acacatgata tttcggaatt ggttttactg 1620
ttcttcagca actattgtac aaaatgttca catttaattt ttctttcttc ttttaagaac 1680
atattataaa aagaatactt tcttggttgg gcttttaatc ctgtgtgtga ttactagtag 1740
gaacatgaga tgtgacattc taaatcttgg gagaaaaaat aatattagga aaaaaatatt 1800
tatgcaggaa gagtagcact cactgaatag ttttaaatga ctgagtggta tgcttacaat 1860
tgtcatgtct agatttaaat tttaagtctg agattttaaa tgtttttgag cttagaaaac 1920
ccagttagat gcaatttggt cattaatacc atgacatctt gcttataaat attccattgc 1980
tctgtagttc aaatctgtta gctttgtgaa aattcatcac tgtgatgttt gtattctttt 2040
tttttttctg tttaacagaa tatgagctgt ctgtcattta cctacttctt tcccactaaa 2100
taaaagaatt cttcagtta 2119

<210> 28
<211> 1480
<212> DNA
<213> Homo sapiens

<220>
<221> modified_base
<222> (309)...(360)
<223> n = g, a, c or t

<400> 28
gggagctaca atgttttgtc attattcact ctgatgtgaa aaaggcagtg aatttaatag 60
aaaataactt cgtagagcaa aatctcaggt gtgttttttt agtgccgcag tcttggatga 120
tgggttccta gaagctctca acatctcttc ttaattggag aaagtgttaa gccccaaagt 180
agctggagca gtacatcttc aatttttgac aagaaaacag gaacttgatt actttgagtg 240
ctattcatta gtttctgctt tcattgagaa tgcaacaaaa gccaactagg ctgctgctaa 300
ctccttgcnn nnnnnnnnnn nnnnnnnnnn nnnnnnnnnn nnnnnnnnnn nnnnnnnnnn 360
ctagggagtc tttcatcttg agtgactgct gcacaaatga tcttcaaagc atttagccca 420
ccagaggaat tctcttgaaa tacccaaaat ccatcagtat cttgaatcat gctggatttt 480

gaagaattct taacaagcca tgtaaagggg gctctctggc cttgaaatag tgatgttttt     540
tatacagaaa ggagaatgca gaatggtcag actaccatgc actgttaaat ttgatttcaa     600
gaaattacag gaaaactttc caaagttcca tctcacagaa attattttta caaagaattc     660
caagataagt ttagttttat ggaagacttt tatgtggttt ttactcactc ttcatctcag     720
acatcaacag atgattacat cacttattta gctagtaaat ttattaatat aaaaactcag     780
agacattcca atatccacat tgcttacacc attaggcata gattcagtgt cagctatgac     840
aattgaaaat aagctgtttt gtgatttaaa ggtttaaatt tctctaacca aactgcttga     900
tccagatgca ggactgcaaa tgttaatatt tgttctggaa gaacaatcaa ataagactta     960
agaggaaaag gaatggccac aatccacctg aaattttttt ttaaaaagtg tgcagcctac    1020
taaatcagaa tgaaaataga agtacaagat tataaacaaa atgcaatcaa acttttctta    1080
agcttaccta aagttatttc atctgaaaat ttcaagcaac tttgttcaac attaaattga    1140
caatctaaac taacaagtct tttgaattta tgcatggtag taaacattct ctctattaac    1200
tgtattacct aaggctaaac ctaaaatttt taagcaaaat tagaaaaata gtcttcactc    1260
atcaaaaaat aaagtttgtt acatttagta ttttcccaag aaaaaaaaaa aaaaaaaaaa    1320
aaaaaaaaaa agagtctgtg caaaatgtca ctaaaaataa attagcacta gaaattattt    1380
ctaaatacca aaaaaaaaaa aaaattgaga tgggtttccc aaggaaaaca ctcgcaatca    1440
aacaacaaaa acaaagaaca aaaaagaata gacaaaaaga                          1480

<210> 29
<211> 1582
<212> DNA
<213> Homo sapiens

<400> 29
tccctgcagc tgtgtttgaa caggtcattt accatgctgt cctccaggtt caacagtatg      60
gctccaaagg atgaaatttc attctgattt tctggctgaa gactattctc tttgtgtatg     120
tccaccacag ttactttatc ccttcatctg tggatgggca ggaaactcca aactgtccaa     180
ggagatagtt ctgttgtgat tacttcattg agaaatttaa cttatgagca gttgaaagga     240
atgcaagttg ctgcaaaatc agaatgaaga gtgcaaaacg assragctac aatgttttgt     300
cattattcac tctgatgtga aaaaggcagt gaatttaata gaaaataact tcgtagagya     360
aaatctcagg tgtgtttttt tagtgccgca gtcttggatg atgggttcct agaagctctc     420
aaacatctct tcttaattgg agaaagtgtt aagccccaaa gtagctggag cagtacatct     480
tcaatttttg acaagaaagc aggaacttga ttactttgag tgctattcat tagtttctgc     540
tttcattgag aatgcaacaa aagccaacta ggctgctgct aactccttgc tggacttctt     600
ctgccactgt cacaggaact gtaatctcac tggacaatta actagggagt ctttcatctt     660
gagtgactgc tgcacaaatg atcttcaaag cattttagcc accagaggaa ttctcttgaa     720
atacccaaaa tccatcagta tcttgaatca tgctggattt tgaagaattc ttaacaagcc     780
atgtaaaggg ggctctctgg ccttgaaata gtgatgtttt ttatacagaa aggagaatgc     840
agaatggtca gactaccatg cactgttaaa tttgatttca agaaattaca ggaaaacttt     900
ccaaagttcc atctcacaga aattattttt acaaagaatt ccaagataag tttagtttta     960
tggaagactt ttatgtggtt tttactcact cttcatctca gacatcaaca gatgattaca    1020
tcacttattt agctagtaaa tttattaata taaaaactca gagacattcc aatatccaca    1080
ttgcttacac cattaggcat agattcagtg tcagctatga caattgaaaa taagctgttt    1140
tgtgatttaa aggtttaaat ttctctaacc aaactgcttg atccagatgc aggactgcaa    1200
atgttaatat ttgttctgga agaacaatca aataagactt aagaggaaaa ggaatggcca    1260
caatccacct gaaatttttt ttttaaaaag tgtgcagcct actaaatcag aatgaaaata    1320
gaagtacaag attataaaca aaatgcaatc aaacttttct taagcttacc taaagttatt    1380
tcatctgaaa atttcaagca actttgttca acattaaatt gacaatctaa actaacaagt    1440
cttttgaatt tatgcatggt agtaaacatt ctctctatta actttattac ctaaggctaa    1500
acctaaaatt tttaagcaaa attagaaaaa tagtcttcac tcatcaaaaa ataaagtttg    1560
ttacatttag tattttccca at                                             1582

<210> 30
<211> 817
<212> DNA
<213> Homo sapiens

<400> 30
agatatatca tacgaaaatg aaaattataa ttcttcttgg attcctggga gccacattgt      60
cagccccagt ttacttctta atcttaataa tggtcaactt ttgccactac aacttcaggg     120
cccacttaat tcatggattc cacctttctc tggaatttta caacagcagc agcaggctca     180
aattccagga ctctcccagt tctctttatc agctctagac cagtttgctg gactgctccc     240

aaatcagata cccttaacag gagaggccag ttttgcccaa ggagcccagg caggccaagt 300
tgatccctta cagcttcaaa caccgcctca gacacaacca ggccccagtc acgtgatgcc 360
ctatgtattc tccttcaaaa tgcctcaaga gcaaggacag atgtttcaat actatccagt 420
ttacatggtc ctaccctggg aacaacctca gcaaacagtt ccaaggtcac ctcaacaaac 480
aagacagcaa cagtatgagg agcagatacc attctatgct caatttggat acattccaca 540
actagcagaa cctgctatat caggaggaca gcagcaacta gcttttgatc cccaactagg 600
cacagctcct gaaattgctg tgatgtcaac aggagaagag ataccatatt tacaaaaaga 660
agcgatcaac tttagacatg acagtgcagg agttttcatg ccctcaactt caccaaaacc 720
cagcacaacc aatgttttca cttctgctgt agaccaaact attaccccag agctcccaga 780
agagaaggac aagactgaca gcctaaggga accataa 817

<210> 31
<211> 917
<212> DNA
<213> Homo sapiens

<400> 31
agagaggaaa agaacacaga tctcgcatgg ttcagatttt tctttttagg tccaggagta 60
agatatatca tacgaaaatg aaaattataa ttcttcttgg attcctggga gccacattgt 120
cagccccact tatcccacag cgtctcatgt ctgccagcaa tagcaatgag ttacttctta 180
atcttaataa tggtcaactt ttgccactac aacttcaggg cccacttaat tcatggattc 240
cacctttctc tggaatttta caacagcagc agcaggctca aattccagga ctctcccagt 300
tctctttatc agctctagac cagtttgctg gactgctccc aaatcagata cccttaacag 360
gagaggccag ttttgcccaa ggagcccagg caggccaagt tgatccctta cagcttcaaa 420
caccgcctca gacacaacca ggccccagtc acgtgatgcc ctatgtattc tccttcaaaa 480
tgcctcaaga gcaaggacag atgtttcaat actatccagt ttacatggtc ctaccctggg 540
aacaacctca gcaaacagtt ccaaggtcac ctcaacaaac aagacagcaa cagtatgagg 600
agcagatacc attctatgct caatttggat acattccaca actagcagaa cctgctatat 660
caggaggaca gcagcaacta gcttttgatc cccaactagg cacagctcct gaaattgctg 720
tgatgtcaac aggagaagag ataccatatt tacaaaaaga agcgatcaac tttagacatg 780
acagtgcagg agttttcatg ccctcaactt caccaaaacc cagcacaacc aatgttttca 840
cttctgctgt agaccaaact attaccccag agctcccaga agagaaggac aagactgaca 900
gcctaaggga accataa 917

<210> 32
<211> 488
<212> DNA
<213> Homo sapiens

<400> 32
tttttttttt tttttgtaga gatggggtct cactttgttg cccatgcttt ggctgctctt 60
caagatttag cacttctgag ctgttgcttt tgtctccagt ctaccctgag cagttcttaa 120
ggtgtttgaa gcagaagaaa gagaaaagag gcttaggtta tactgcttag aacctcctct 180
tcaactaacc taccgaccca cctacccata aatccatacc tacacacaca cccctttcct 240
ttcctctgcc ctggttttgc ctcgcctgct ttcaattgca cgtgtgttga gtatagcctt 300
tgcctctgcc tactcagctc cttgaactga gggtgaaatt gagacccaga ggaatgggat 360
ttacagcttc ttgctttctt cttgccttgt cctagaactg aagtacaaat gggaagaagc 420
tttgatgaag gaagaccccc atccaagaac atctagtttt caggtgccat aacagcagag 480
caagttca 488

<210> 33
<211> 2907
<212> DNA
<213> Homo sapiens

<400> 33
ctcccagagt gctaggatta caggcatgag ccactgctcc cagccttcca gaggaatttt 60
aagcccatgt ccaaacattc tgtttgtata aatatattct aatttttaaa taaatagttt 120
ctacttttct gaactttata ttttttcttg ctataatgga ttttcataat cagaaaagat 180
taaattagta atcatgaatt gccttcaata tttggcagta agtcaatgaa ataataaggc 240
acttatatac catctttgac atcattaaaa gtatcaaatc ccattaatct aaaacttctt 300
taagcatttt gaaagcagaa aatgtttaca tggttctttc agttcctcag gctttttgtc 360

taatgatgcg tgacttagga taagatttga attaagtgcc cagcttgaaa cataataatt 420
tttctgtata agccacagat cctctacctc ctttgtgtta aagcctttat atgaaacaat 480
taagtagaag cattcaatag tgtgtcatta actgttcata ctaataaatg gatacagcac 540
attttcatgg cctgtaatgt agaacatact atataaagtt ctcagtttgg ggatgactag 600
gtttctggaa ggaatagaat gctaaatcaa tggatggcat tgggctgaga aacactgctg 660
ctactaatca gccttgaatg tgtaatgtga acatgcaaaa gagaacatgc atacactcaa 720
atttgtacaa tgctataact ggaagttgaa ggacttgaat ttttatattg tgctattgtt 780
atgttttctg taattgttta tatctaagga atttttgagg taatataaaa gaaaaagaga 840
ataatgaaca atgatgtcac tggagggttt ttacattaaa ttagatcatt tttcttctta 900
ttcacaataa taatcttaat ctttaagaat taattataat ttaatattat aattcataat 960
ctttaagaat taataattat aatttaatat tataattaat aatctttaag aattaataat 1020
ataatttaat attataatta ataatcttta agaattaata attacaatta ataattaata 1080
ataatcttaa tctttaagaa ttaataataa tccttaatcg caataataat cgcaaggagg 1140
agaagtaagt ccctcctcct tctgtatgaa cttttctccc acatgctgct gtatggttta 1200
gtgagagtga agttctaaag aacatcaata tgattggtgg gataatccaa agacattttt 1260
tcagaatcaa atggcatgtc gaaggtttgt ttcttgcata tgtatttact ggtccacagc 1320
acaaaataaa gtgaccacat atacatagga aagttgaatt tgtacacata cagcatctga 1380
aatgtatctg atgttcagca tcaagatttc actgaacatt gtagaaatgt gtatcttttg 1440
catgtatatt ttacattgat tttctattta tgtacatcta gaaagtttta accctaataa 1500
atagttttgt aattttgaat aatagtgtca gtttatatgt gagggagtag agacagagag 1560
gttagcactg gataataatt agtaaggcca aaggagaaaa tttcatagaa aatattgttg 1620
ttgtcataat gagtacagca tgaaaggctt cctctacaag acactagtca aagagttgag 1680
agctgcggtt tctaatcttt gtccattact cccttactcc ctatgagact gtggacctgt 1740
cacttggcct ctctggtctt cagttttctc accagtaaaa caaggaactt gaaccaaatg 1800
acctctagtg ttccccttgg gtttaaatgt ctataaatgt tcaatgacta gaatgtattg 1860
cgtttttctt tattcttttt gctttgagaa aagagaatgt gatttaagag taataatttg 1920
aataccaatt atccacatta aaattgtgtc ctctatgtgt aaggcatagc acatttagca 1980
cacatacata agcacactaa gcaccttaca aatatcctca tttattcttt acataatctt 2040
ttgaaattga ttatgtaata cacactgttt ttgaacaatt ggtgacttcc agctgtttaa 2100
aacaaactac agtatggtgc ttgagtactg acttaggagg tcagcattgg tttcactagg 2160
agcttctcaa agcacgctgc caaacatgct ccagtctcat tgtcaaggcc ttagaccagg 2220
caatcattac ggcagtggtt cttcaacttc agcagcagca aaacgatctg gcgggggctt 2280
ggtgaaacag actgctgggc tcgaccacca gaatttctca ttcagagggt ctggcctgat 2340
cacttgcatt tctaatcact tcccaggtga tgcagatgtt tctggtccag ggaccccagt 2400
ttgagaacca ctgtattaaa atttccttca tctctataga aatggaaaga ttttttataa 2460
gtcctctaat ttgctttaag ataaatgaga tttcacttaa ttctgttgga gaaattgttt 2520
taaaaattgt gctaaagaac cgaaaatcac tttatgttaa ggctctattt atagcaagtg 2580
aacttttcat gagttaataa aggcctacaa aaataatttt gactgtgaaa ctaattaaaa 2640
tctctgtgtt tcatttaaag cataaacata tttgaataaa aataggttaa caataatttg 2700
ggacatgtat tcagtataat tttaagataa ttttacaaaa tatatgtaac attgcatttg 2760
tttctgtaaa atatcttcgg aaaaagcctt gttttcccta gtgtgttatt tgttgaattt 2820
cttgttaaat gtattttttc ccattgaaaa aaatgttttt aatcaatgtg atcaatacag 2880
ctatctatat gccctgcttt cactgta 2907

<210> 34
<211> 2456
<212> DNA
<213> Homo sapiens

<400> 34
ataacagcat gaagtgccgt ggaactggaa taggcgtgtc ctctccctcg accctccccc 60
tccttgtccc tctgctcacc cctcgctcgt tccctccctc cggcgagggc cgcctttata 120
acaactgctc agagtgcgag ggcgggatag ctgtccaagg tctcccccag cactgaggag 180
ctcgcctgct gccctcttgc gcgcgggaag cagcaccaag ttcacggcca acgccttggc 240
actagggtcc agaatggcta caacagtccc tgatggttgc cgcaatggcc tgaaatccaa 300
gtactacaga ctttgtgata aggctgaagc ttggggcatc gtcctagaaa cggtggccac 360
agccggggtt gtgacctcgg tggccttcat gctcactctc ccgatcctcg tctgcaaggt 420
gcaggactcc aacaggcgaa aaatgctgcc tactcagttt ctcttcctcc tgggtgtgtt 480
gggcatcttt ggcctcacct tcgccttcat catcggactg gacgggagca cagggcccac 540
acgcttcttc ctctttggga tcctcttttc catctgcttc tcctgcctgc tggctcatgc 600
tgtcagtctg accaagctcg tccgggggag gaagcccctt tccctgttgg tgattctggg 660
tctggccgtg ggcttcagcc tagtccagga tgttatcgct attgaatata ttgtcctgac 720
catgaatagg accaacgtca atgtcttttc tgagctttcc gctcctcgtc gcaatgaaga 780

ctttgtcctc ctgctcacct acgtcctctt cttgatggcg ctgaccttcc tcatgtcctc 840
cttcaccttc tgtggttcct tcacgggctg gaagagacat ggggcccaca tctacctcac 900
gatgctcctc tccattgcca tctgggtggc ctggatcacc ctgctcatgc ttcctgactt 960
tgaccgcagg tgggatgaca ccatcctcag ctccgccttg gctgccaatg gctgggtgtt 1020
cctgttggct tatgttagtc ccgagttttg gctgctcaca aagcaacgaa accccatgga 1080
ttatcctgtt gaggatgctt tctgtaaacc tcaactcgtg aagaagagct atggtgtgga 1140
gaacagagcc tactctcaag aggaaatcac tcaaggtttt gaagagacag gggacacgct 1200
ctatgccccc tattccacac attttcagct gcagaaccag cctccccaaa aggaattctc 1260
catcccacgg gcccacgctt ggccgagccc ttacaaagac tatgaagtaa agaaagaggg 1320
cagctaactc tgtcctgaag agtgggacaa atgcagccgg gcggcagatc tagcgggagc 1380
tcaaaggatg gtgggcgaaa tcttgagtct tctgagaaaa ctgtacaaga cactacggga 1440
acagtttgcc tccctcccag cctcaaccac aattcttcca tgctggggct gatgtgggct 1500
agtaagactc cagttcttag aggcgctgta gtattttttt tttttgtctt catcctttgg 1560
atacttcttt taagtgggag tctcaggcaa ctcaagttta gacccttact cttttgtttt 1620
gttttttgaa acaggatctt gctctgtcac ccaggcttga gtgcagtggt gcgatcacag 1680
cccagtgcag cctcgaccac ctgtgctcaa gcaatcctcc catctccatc tcccaaagtg 1740
ctgggatgac aggcgtgagc cacagctccc agcctaggcc cttaatcttg ctgttatttt 1800
ccatggacta aaggtctggt catctgagct cacgctggct cacacagctc taggggcctg 1860
ctcctctaac tcacagtggg ttttgtgagg ctctgtggcc cagagcagac ctgcatatct 1920
gagcaaaaat agcaaaagcc tctctcagcc cactggcctg aatctacact ggaagccaac 1980
ttgctggcac ccccgctccc caacccttct tgcctgggta ggagaggcta aagatcaccc 2040
taaatttact catctctcta gtgctgcctc acattgggcc tcagcagctc cccagcacca 2100
attcacaggt cacccctctc ttcttgcact gtccccaaac ttgctgtcaa ttccgagatc 2160
taatctcccc ctacgctctg ccaggaattc tttcagacct cactagcaca agcccggttg 2220
ctccttgtca ggagaatttg tagatcattc tcacttcaaa ttcctggggc tgatacttct 2280
ctcatcttgc accccaacct ctgtaaatag atttaccgca tttacggctg cattctgtaa 2340
gtgggcatgg tctcctaatg gaggagtgtt cattgtataa taagttattc acctgagtat 2400
gcaataaaga tgtggtggcc actctttcat ggtggtggca gcaaaaaaaa aaaaaa 2456

<210> 35
<211> 4945
<212> DNA
<213> Homo sapiens

<400> 35
   atgcccaaga tagtcctgaa tggtgtgacc gtagacttcc ctttccagcc ctacaaatgc 60
   caacaggagt acatgaccaa ggtcctggaa tgtctgcagc agaaggtgaa tggcatcctg 120
   gagagcccta cgggtacagg gaagacgctg tgcctgctgt gcaccacgct ggcctggcga 180
   gaacacctcc gagacggcat ctctgcccgc aagattgccg agagggcgca aggagagctt 240
   ttcccggatc gggccttgtc atcctggggc aacgctgctg ctgctgctgg agaccccata 300
   gcttgctaca cggacatccc aaagattatt tacgcctcca ggacccactc gcaactcaca 360
   caggtcatca acgagcttcg gaacacctcc taccggccta aggtgtgtgt gctgggctcc 420
   cgggagcagc tgtgcatcca tcctgaggtg aagaaacaag agagtaacca tctacagatc 480
   cacttgtgcc gtaagaaggt ggcaagtcgc tcctgtcatt tctacaacaa cgtagaagaa 540
   aaaagcctgg agcaggagct ggccagcccc atcctggaca ttgaggactt ggtcaagagc 600
   ggaagcaagc acagggtgtg cccttactac ctgtcccgga acctgaagca gcaagccgac 660
   atcatattca tgccgtacaa ttacttgttg gatgccaaga gccgcagagc acacaacatt 720
   gacctgaagg ggacagtcgt gatctttgac gaagctcaca acgtggagaa gatgtgtgaa 780
   gaatcggcat cctttgacct gactccccat gacctggctt caggactgga cgtcatagac 840
   caggtgctgg aggagcagac caaggcagcg cagcagggtg agccccaccc ggagttcagc 900
   gcggactccc ccagcccagg gctgaacatg gagctggaag acattgcaaa gctgaagatg 960
   atcctgctgc gcctggaggg ggccatcgat gctgttgagc tgcctggaga cgacagcggt 1020
   gtcaccaagc cagggagcta catctttgag ctgtttgctg aagcccagat cacgtttcag 1080
   accaagggct gcatcctgga ctcgctggac cagatcatcc agcacctggc aggacgtgct 1140
   ggagtgttca ccaacacggc cggactgcag aagctggcgg acattatcca gattgtgttc 1200
   agtgtggacc cctccgaggg cagccctggt tccccagcag ggctgggggc cttacagtcc 1260
   tataaggtgc acatccatcc tgatgctggt caccggagga cggctcagcg gtctgatgcc 1320
   tggagcacca ctgcagccag aaagcgaggg aaggtgctga gctactggtg cttcagtccc 1380
   ggccacagca tgcacgagct ggtccgccag ggcgtccgct ccctcatcct taccagcggc 1440
   acgctggccc cggtgtcctc ctttgctctg gagatgcaga tccctttccc agtctgcctg 1500
   gagaacccac acatcatcga caagcaccag atctgggtgg gggtcgtccc cagaggcccc 1560
   gatggagccc agttgagctc cgcgtttgac agacggtttt ccgaggagtg cttatcctcc 1620
   ctggggaagg ctctgggcaa catcgcccgc gtggtgccct atgggctcct gatcttcttc 1680
   ccttcctatc ctgtcatgga gaagagcctg gagttctggc gggcccgcga cttggccagg 1740
   aagatggagg cgctgaagcc gctgtttgtg gagcccagga gcaaaggcag cttctccgag 1800
   accatcagtg cttactatgc aagggttgcc gcccctgggt ccaccggcgc caccttcctg 1860
   gcggtctgcc ggggcaaggc cagcgagggg ctggacttct cagacacgaa tggccgtggt 1920
   gtgattgtca cgggcctccc gtacccccca cgcatggacc cccgggttgt cctcaagatg 1980
   cagttcctgg atgagatgaa gggccagggt ggggctgggg gccagttcct ctctgggcag 2040
   gagtggtacc ggcagcaggc gtccagggct gtgaaccagg ccatcgggcg agtgatccgg 2100
   caccgccagg actacggagc tgtcttcctc tgtgaccaca ggttcgcctt tgccgacgca 2160
   agagcccaac tgccctcctg ggtgcgtccc cacgtcaggg tgtatgacaa ctttggccat 2220
   gtcatccgag acgtggccca gttcttccgt gttgccgagc gaactatgcc agcgccggcc 2280
   ccccgggcta cagcacccag tgtgcgtgga gaagatgctg tcagcgaggc caagtcgcct 2340
   ggccccttct tctccaccag gaaagctaag agtctggacc tgcatgtccc cagcctgaag 2400
   cagaggtcct cagggtcacc agctgccggg gaccccgaga gtagcctgtg tgtggagtat 2460
   gagcaggagc cagttcctgc ccggcagagg cccagggggc tgctggccgc cctggagcac 2520
   agcgaacagc gggcggggag ccctggcgag gagcaggccc acagctgctc caccctgtcc 2580
   ctcctgtctg agaagaggcc ggcagaagaa ccgcgaggag ggaggaagaa gatccggctg 2640
   gtcagccacc cggaggagcc cgtggctggt gcacagacgg acagggccaa gctcttcatg 2700
   gtggccgtga agcaggagtt gagccaagcc aactttgcca ccttcaccca ggccctgcag 2760
   gactacaagg gttccgatga cttcgccgcc ctggccgcct gtctcggccc cctctttgct 2820
   gaggacccca agaagcacaa cctgctccaa ggcttctacc agtttgtgcg gccccaccat 2880
   aagcagcagt ttgaggaggt ctgtatccag ctgacaggac gaggctgtgg ctatcggcct 2940
   gagcacagca ttccccgaag gcagcgggca cagccggtcc tggaccccac tggaagaacg 3000
   gcgccggatc ccaagctgac cgtgtccacg gctgcagccc agcagctgga cccccaagag 3060
   cacctgaacc agggcaggcc ccacctgtcg cccaggccac ccccaacagg agaccctggc 3120
   agccaaccac agtgggggtc tggagtgccc agagcaggga agcagggcca gcacgccgtg 3180
   agcgcctacc tggctgatgc ccgcagggcc ctggggtccg cgggctgtag ccaactcttg 3240
   gcagcgctga cagcctataa gcaagacgac gacctcgaca aggtgctggc tgtgttggcc 3300
   gccctgacca ctgcaaagcc agaggacttc cccctgctgc acaggttcag catgtttgtg 3360
   cgtccacacc acaagcagcg cttctcacag acgtgcacag acctgaccgg ccggccctac 3420
   ccgggcatgg agccaccggg accccaggag gagaggcttg ccgtgcctcc tgtgcttacc 3480
   cacagggctc ccaaccaggc cccctcacgg tccgagaaga ccgggaagac ccagagcaag 3540
   atctcgtcct tccttagaca gaggccagca gggactgtgg gggcggcggt tgaggatgca 3600
   ggtcccagcc agtcctcagg acctccccac gggcctgcag catctgagtg gggtgagcct 3660
   catgggagag acatcgctgg gcagcaggcc acgggagctc cgggcggccc cctctcagca 3720
   ggctgtgtgt gccagggctg tggggcagag gacgtggtgc ccttccagtg ccctgcctgt 3780
   gacttccagc gctgccaagc ctgctggcaa cggcaccttc aggcctctag gatgtgccca 3840
   gcctgccaca ccgcctccag gaagcagagc gtcatgcagg tcttctggcc agagccccac 3900
   aaggaccatg agggcgctgg aggggccagg cctgtcgctg ctgtgcctgg tgttggcgct 3960
   gcctgccctg ctgccggtgc cggctgtacg cggagtggca gaaacaccca cctacccctg 4020
   gcgggacgca gagacagggg agcggctggt gtgtgcccag tgccccccag gcacctttgt 4080
   gcagcggccg tgccgccgag acagccccac gacgtgtggc ccgtgtccac cgcgccacta 4140
   cacgcagttc tggaactacc tggagcgctg ccgctactgc aacgtcctct gcggggagcg 4200
   tgaggaggag gcacgggctt gccacgccac ccacaaccgt gcctgccgct gccgcaccgg 4260
   cttcttcgcg cacgctggtt tctgcttgga gcacgcatcg tgtccacctg gtgccggcgt 4320
   gattgccccg ggcaccccca gccagaacac gcagtgccag ccgtgccccc caggcacctt 4380
   ctcagccagc agctccagct cagagcagtg ccagccccac cgcaactgca cggccctggg 4440
   cctggccctc aatgtgccag gctcttcctc ccatgacacc ctgtgcacca gctgcactgg 4500
   cttccccctc agcaccaggg taccaggagc tgaggagtgt gagcgtgccg tcatcgactt 4560
   tgtggctttc caggacatct ccatcaagag gctgcagcgg ctgctgcagg ccctcgaggc 4620
   cccggagggc tggggtccga caccaagggc gggccgcgcg gccttgcagc tgaagctgcg 4680
   tcggcggctc acggagctcc tgggggcgca ggacggggcg ctgctggtgc ggctgctgca 4740
   ggcgctgcgc gtggccagga tgcccgggct ggagcggagc gtccgtgagc gcttcctccc 4800
   tgtgcactga tcctggcccc ctcttattta ttctacatcc ttggcacccc acttgcactg 4860
   aaagaggctt ttttttaaat agaagaaatg aggtttctta aagcttattt ttataaagct 4920
   ttttcataaa actggttgta gttgc 4945

<210> 36
<211> 4946
<212> DNA
<213> Homo sapiens

<400> 36
agtcagccct gctgccagcc agtgccgggt gctggggact cagggaggcc cgccgggacc 60

actgcgggac agtgagccga gcagaagctg gaacgcagga gaggaaggag agggggcggt 120
cagggctctc aggagccggg tcctgggcaa ggcgcagccg ttttcaaatt ttcaggaaag 180
cggtcggctc acactcgagc agtaaaaaga tgcctctggg gaggaggccc gtgcagctct 240
ccgggcaatg gtggtggctc ggcctagaga ggcggtagtg gaacgcagac cctggtgggg 300
gaatgacatc aagggaggag acgggcggga ccccagattt ctgcctgtgg gcgatggaag 360
tgaggttcac tggccagcgg agccggacac agaacgcgca aaacgccgtg taggcctgga 420
ggagccgaag agcaggcgga ccccctccgc gggggaacag tttccgccgg gagcacaaag 480
caacggaccg gaagtggggg gcggaagtgc agtgggctca gcgccgactg cgcgcctctg 540
cccgcgaaaa ctctgagctg gctgacagct ggggacgggt ggcggccctc gactggagtc 600
ggttgagttc ctgagggacc ccggttctgg aaggttcgcc gcggagacaa gtgagcagtc 660
tgtgccatag ggattctcga agagaacagc gttgtgtccc agtgcacatg ctcgcatcgc 720
ttaccaggag tgcccgagac cctaagatgt tcggagtggt tttttcgcac agacccgaat 780
agcctgcccc tcagccacgc tctgtgccct tctgagaaca ggctgatatg cccaagatag 840
tcctgaatgg tgtgaccgta gacttccctt tccagcccta caaatgccaa caggagtaca 900
tgaccaaggt cctggaatgt ctgcagcaga aggtgaatgg catcctggag agccctacgg 960
gtacagggaa gacgctgtgc ctgctgtgca ccacgctggc ctggcgagaa cacctccgag 1020
acggcatctc tgcccgcaag attgccgaga gggcgcaagg agagcttttc ccggatcggg 1080
ccttgtcatc ctggggcaac gctgctgctg ctgctggaga ccccatagct tgctacacgg 1140
acatcccaaa gattatttac gcctccagga cccactcgca actcacacag gtcatcaacg 1200
agcttcggaa cacctcctac cggcctaagg tgtgtgtgct gggctcccgg gagcagctgt 1260
gcatccatcc tgaggtgaag aaacaagaga gtaaccatct acagatccac ttgtgccgta 1320
agaaggtggc aagtcgctcc tgtcatttct acaacaacgt agaagaaaaa agcctggagc 1380
aggagctggc cagccccatc ctggacattg aggacttggt caagagcgga agcaagcaca 1440
gggtgtgccc ttactacctg tcccggaacc tgaagcagca agccgacatc atattcatgc 1500
cgtacaatta cttgttggat gccaagagcc gcagagcaca caacattgac ctgaagggga 1560
cagtcgtgat ctttgacgaa gctcacaacg tggagaagat gtgtgaagaa tcggcatcct 1620
ttgacctgac tccccatgac ctggcttcag gactggacgt catagaccag gtgctggagg 1680
agcagaccaa ggcagcgcag cagggtgagc cccacccgga gttcagcgcg gactccccca 1740
gcccagggct gaacatggag ctggaagaca ttgcaaagct gaagatgatc ctgctgcgcc 1800
tggagggggc catcgatgct gttgagctgc ctggagacga cagcggtgtc accaagccag 1860
ggagctacat ctttgagctg tttgctgaag cccagatcac gtttcagacc aagggctgca 1920
tcctggactc gctggaccag atcatccagc acctggcagg acgtgctgga gtgttcacca 1980
acacggccgg actgcagaag ctggcggaca ttatccagat tgtgttcagt gtggacccct 2040
ccgagggcag ccctggttcc ccagcagggc tgggggcctt acagtcctat aaggtgcaca 2100
tccatcctga tgctggtcac cggaggacgg ctcagcggtc tgatgcctgg agcaccactg 2160
cagccagaaa gcgagggaag gtgctgagct actggtgctt cagtcccggc cacagcatgc 2220
acgagctggt ccgccagggc gtccgctccc tcatccttac cagcggcacg ctggccccgg 2280
tgtcctcctt tgctctggag atgcagatcc ctttcccagt ctgcctggag aacccacaca 2340
tcatcgacaa gcaccagatc tgggtggggg tcgtccccag aggccccgat ggagcccagt 2400
tgagctccgc gtttgacaga cggttttccg aggagtgctt atcctccctg gggaaggctc 2460
tgggcaacat cgcccgcgtg gtgccctatg ggctcctgat cttcttccct tcctatcctg 2520
tcatggagaa gagcctggag ttctggcggg cccgcgactt ggccaggaag atggaggcgc 2580
tgaagccgct gtttgtggag cccaggagca aaggcagctt ctccgagacc atcagtgctt 2640
actatgcaag ggttgccgcc cctgggtcca ccggcgccac cttcctggcg gtctgccggg 2700
gcaaggccag cgaggggctg gacttctcag acacgaatgg ccgtggtgtg attgtcacgg 2760
gcctcccgta ccccccacgc atggaccccc gggttgtcct caagatgcag ttcctggatg 2820
agatgaaggg ccagggtggg gctgggggcc agttcctctc tgggcaggag tggtaccggc 2880
agcaggcgtc cagggctgtg aaccaggcca tcgggcgagt gatccggcac cgccaggact 2940
acggagctgt cttcctctgt gaccacaggt tcgcctttgc cgacgcaaga gcccaactgc 3000
cctcctgggt gcgtccccac gtcagggtgt atgacaactt tggccatgtc atccgagacg 3060
tggcccagtt cttccgtgtt gccgagcgaa ctatgccagc gccggccccc cgggctacag 3120
cacccagtgt gcgtggagaa gatgctgtca gcgaggccaa gtcgcctggc cccttcttct 3180
ccaccaggaa agctaagagt ctggacctgc atgtccccag cctgaagcag aggtcctcag 3240
ggtcaccagc tgccggggac cccgagagta gcctgtgtgt ggagtatgag caggagccag 3300
ttcctgcccg gcagaggccc agggggctgc tggccgccct ggagcacagc gaacagcggg 3360
cggggagccc tggcgaggag caggcccaca gctgctccac cctgtccctc ctgtctgaga 3420
agaggccggc agaagaaccg cgaggaggga ggaagaagat ccggctggtc agccacccgg 3480
aggagcccgt ggctggtgca cagacggaca gggccaagct cttcatggtg gccgtgaagc 3540
aggagttgag ccaagccaac tttgccacct tcacccaggc cctgcaggac tacaagggtt 3600
ccgatgactt cgccgccctg gccgcctgtc tcggccccct ctttgctgag gaccccaaga 3660
agcacaacct gctccaaggc ttctaccagt ttgtgcggcc ccaccataag cagcagtttg 3720
aggaggtctg tatccagctg acaggacgag gctgtggcta tcggcctgag cacagcattc 3780
cccgaaggca gcgggcacag ccggtcctgg accccactgg aagaacggcg ccggatccca 3840

agctgaccgt gtccacggct gcagcccagc agctggaccc ccaagagcac ctgaaccagg 3900
gcaggcccca cctgtcgccc aggccacccc caacaggaga ccctggcagc caaccacagt 3960
gggggtctgg agtgcccaga gcagggaagc agggccagca cgccgtgagc gcctacctgg 4020
ctgatgcccg cagggccctg gggtccgcgg gctgtagcca actcttggca gcgctgacag 4080
cctataagca agacgacgac ctcgacaagg tgctggctgt gttggccgcc ctgaccactg 4140
caaagccaga ggacttcccc ctgctgcaca ggttcagcat gtttgtgcgt ccacaccaca 4200
agcagcgctt ctcacagacg tgcacagacc tgaccggccg gccctacccg ggcatggagc 4260
caccgggacc ccaggaggag aggcttgccg tgcctcctgt gcttacccac agggctcccc 4320
aaccaggccc ctcacggtcc gagaagaccg ggaagaccca gagcaagatc tcgtccttcc 4380
ttagacagag gccagcaggg actgtggggg cgggcggtga ggatgcaggt cccagccagt 4440
cctcaggacc tccccacggg cctgcagcat ctgagtgggg cctctaggat gtgcccagcc 4500
tgccacaccg cctccaggaa gcagagcgtc atgcaggtct tctggccaga gccccagtga 4560
gtgcccacgg aggcccccag cacacccaac gtggcttgat cacctgcctg tccagctctg 4620
gtgggccaag aacccaccca acagaatagg ccagcccatg ccagccggct tggcccgctg 4680
caggcctcag gcaggcgggg cccatggttg gtccctgcgg tgggaccgga tctgggcctg 4740
cctctgagaa gccctgagct accttggggt ctggggtggg tttctgggaa agtgcttccc 4800
cagaacttcc ctggctcctg gcctgtgagt ggtgccacag gggcacccca gctgagcccc 4860
tcaccgggaa ggaggagacc cccgtgggca cgtgtccact tttaatcagg ggacagggct 4920
ctctaataaa gctgctggca gtgccc 4946

<210> 37
<211> 2288
<212> DNA
<213> Homo sapiens

<400> 37
agcctctccg gctactcggg gtcagcgtgg ggcccctgca gcagatgagg gtcttcactt 60
cggtgaactg aacccttgaa gcggctgtgg gcagggcagc agggctatgg ccacccccca 120
ggttcgcctt tgccgacgca agagcccaac tgccctcctg ggtgcgtccc cacgtcaggg 180
tgtatgacaa ctttggccat gtcatccgag acgtggccca gttcttccgt gttgccgagc 240
gaactatgcc agcgccggcc ccccgggcta cagcacccag tgtgcgtgga gaagatgctg 300
tcagcgaggc caagtcgcct ggccccttct tctccaccag gaaagctaag agtctggacc 360
tgcatgtccc cagcctgaag cagaggtcct cagggtcacc agctgccggg gaccccgaga 420
gtagcctgtg tgtggagtat gagcaggagc cagttcctgc ccggcagagg cccagggggc 480
tgctggccgc cctggagcac agcgaacagc gggcggggag ccctggcgag gagcaggccc 540
acagctgctc caccctgtcc ctcctgtctg agaagaggcc ggcagaagaa ccgcgaggag 600
ggaggaagaa gatccggctg gtcagccacc cggaggagcc cgtggctggt gcacagacgg 660
acagggccaa gctcttcatg gtggccgtga agcaggagtt gagccaagcc aactttgcca 720
ccttcaccca ggccctgcag gactacaagg gttccgatga cttcgccgcc ctggccgcct 780
gtctcggccc cctctttgct gaggacccca agaagcacaa cctgctccaa ggcttctacc 840
agtttgtgcg gccccaccat aagcagcagt ttgaggaggt ctgtatccag ctgacgggac 900
gaggctgtgg ctatcggcct gagcacagca ttccccgaag gcagcgggca cagccggtcc 960
tggaccccac tggaagaacg gcgccggatc ccaagctgac cgtgtccacg gctgcagccc 1020
agcagctgga cccccaagag cacctgaacc agggcaggcc ccacctgtcg cccaggccac 1080
ccccaacagg agaccctggc agccaaccac agtgggggtc tggagtgccc agagcaggga 1140
agcagggcca gcacgccgtg agcgcctacc tggctgatgc ccgcagggcc ctggggtccg 1200
cgggctgtag ccaactcttg gcagcgctga cagcctataa gcaagacgac gacctcgaca 1260
aggtgctggc tgtgttggcc gccctgacca ctgcaaagcc agaggacttc cccctgctgc 1320
acaggttcag catgtttgtg cgtccacacc acaagcagcg cttctcacag acgtgcacag 1380
acctgaccgg ccggccctac ccgggcatgg agccaccggg accccaggag gagaggcttg 1440
ccgtgcctcc tgtgcttacc cacagggctc cccaaccagg cccctcacgg tccgagaaga 1500
ccgggaagac ccagagcaag atctcgtcct tccttagaca gaggccagca gggactgtgg 1560
gggcgggcgg tgaggatgca ggtcccagcc agtcctcagg acctccccac gggcctgcag 1620
catctgagtg gggtgagcct catgggagag acatcgctgg gcagcaggcc acgggagctc 1680
cgggcgggcc cctctcagca ggctgtgtgt gccagggctg tggggcagag gacgtggtgc 1740
ccttccagtg ccctgcctgt gacttccagc gctgccaagc ctgctggcaa cggcaccttc 1800
aggcctctag gatgtgccca gcctgccaca ccgcctccag gaagcagagc gtcatgcagg 1860
tcttctggcc agagccccag tgagtgccca cggaggcccc cagcacaccc aacgtggctt 1920
gatcacctgc ctgtccagct ctggtgggcc aagaacccac ccaacagaat aggccagccc 1980
atgccagccg gcttggcccg ctgcaggcct caggcaggcg gggcccatgg ttggtccctg 2040
cggtgggacc ggatctgggc ctgcctctga gaagccctga gctaccttgg ggtctggggt 2100
gggtttctgg gaaagtgctt ccccagaact tccctggctc ctggcctgtg agtggtgcca 2160
caggggcacc ccagctgagc ccctcaccgg gaaggaggag acccccgtgg gcacgtgtcc 2220

acttttaatc aggggacagg gctctctaat aaagctgctg gcagtgccca ggaaaaaaaa 2280
aaaaaaaa 2288

<210> 38
<211> 1124
<212> DNA
<213> Homo sapiens

<400> 38
tccgcaggcg gaccgggggc aaaggaggtg gcatgtcggt caggcacagc agggtcctgt 60
gtccgcgctg agccgcgctc tccctgctcc agcaaggacc atgagggcgc tggaggggcc 120
aggcctgtcg ctgctgtgcc tggtgttggc gctgcctgcc ctgctgccgg tgccggctgt 180
acgcggagtg gcagaaacac ccacctaccc ctggcgggac gcagagacag gggagcggct 240
ggtgtgcgcc cagtgccccc caggcacctt tgtgcagcgg ccgtgccgcc gagacagccc 300
cacgacgtgt ggcccgtgtc caccgcgcca ctacacgcag ttctggaact acctggagcg 360
ctgccgctac tgcaacgtcc tctgcgggga gcgtgaggag gaggcacggg cttgccacgc 420
cacccacaac cgtgcctgcc gctgccgcac cggcttcttc gcgcacgctg gtttctgctt 480
ggagcacgca tcgtgtccac ctggtgccgg cgtgattgcc ccgggcaccc ccagccagaa 540
cacgcagtgc cagccgtgcc ccccaggcac cttctcagcc agcagctcca gctcagagca 600
gtgccagccc caccgcaact gcacggccct gggcctggcc ctcaatgtgc caggctcttc 660
ctcccatgac accctgtgca ccagctgcac tggcttcccc ctcagcacca gggtaccagg 720
agctgaggag tgtgagcgtg ccgtcatcga ctttgtggct ttccaggaca tctccatcaa 780
gaggctgcag cggctgctgc aggccctcga ggccccggag ggctggggtc cgacaccaag 840
ggcgggccgc gcggccttgc agctgaagct gcgtcggcgg ctcacggagc tcctgggggc 900
gcaggacggg gcgctgctgg tgcggctgct gcaggcgctg cgcgtggcca ggatgcccgg 960
gctggagcgg agcgtccgtg agcgcttcct ccctgtgcac tgatcctggc cccctcttat 1020
ttattctaca tccttggcac cccacttgca ctgaaagagg ctttttttta aatagaagaa 1080
atgaggtttc ttaaagctta tttttataaa gctttttcat aaaa 1124

<210> 39
<211> 1459
<212> DNA
<213> Homo sapiens

<400> 39
ctgaccactg caaagccaga ggacttcccc ctgctgcaca ggttcagcat gtttgtgcgt 60
ccacaccaca agcagcgctt ctcacagacg tgcacagacc tgaccggccg gccctacccg 120
ggcatggagc caccgggacc ccaggaggag aggcttgccg tgcctcctgt gcttacccac 180
agggctcccc aaccaggccc ctcacggtcc gagaagaccg ggaagaccca gagcaagatc 240
tcgtccttcc ttagacagag gccagcaggg actgtggggg cgggcggtga ggatgcaggt 300
cccagccagt cctcaggacc tccccacggg cctgcagcat ctgagtgggg cctctaggat 360
gtgcccagcc tgccacaccg cctccaggaa gcagagcgtc atgcaggtct tctggccgga 420
gccccacaag gaccatgagg gcgctggagg ggccaggcct gtcgctgctg tgcctggtgt 480
tggcgctgcc tgccctgctg ccggtgccgg ctgtacgcgg agtggcagaa acacccacct 540
acccctggcg ggacgcagag acaggggagc ggctggtgtg cgcccagtgc cccccaggca 600
cctttgtgca gcggccgtgc cgccgagaca gccccacgac gtgtggcccg tgtccaccgc 660
gccactacac gcagttctgg aactacctgg agcgctgccg ctactgcaac gtcctctgcg 720
gggagcgtga ggaggaggca cgggcttgcc acgccaccca caaccgtgcc tgccgctgcc 780
gcaccggctt cttcgcgcac gctggtttct gcttggagca cgcatcgtgt ccacctggtg 840
ccggcgtgat tgccccgggc acccccagcc agaacacgca gtgccagccg tgccccccag 900
gcaccttctc agccagcagc tccagctcag agcagtgcca gccccaccgc aactgcacgg 960
ccctgggcct ggccctcaat gtgccaggct cttcctccca tgacaccctg tgcaccagct 1020
gcactggctt ccccctcagc accagggtac caggagctga ggagtgtgag cgtgccgtca 1080
tcgactttgt ggctttccag gacatctcca tcaagaggct gcagcggctg ctgcaggccc 1140
tcgaggcccc ggagggctgg ggtccgacac caagggcggg ccgcgcggcc ttgcagctga 1200
agctgcgtcg gcggctcacg gagctcctgg gggcgcagga cggggcgctg ctggtgcggc 1260
tgctgcaggc gctgcgcgtg gccaggatgc ccgggctgga gcggagcgtc cgtgagcgct 1320
tcctccctgt gcactgatcc tggccccctc ttatttattc tacatccttg gcaccccact 1380
tgcactgaaa gaggcttttt tttaaataga agaaatgagg tttcttaaaa gcttattttt 1440
ataaagcttt ttcataaaa 1459

<210> 40
<211> 2958
<212> DNA
<213> Homo sapiens

<400> 40
ggcacgaggg tgtgcgtgat ggagaaaatt gggcaccagg gctgctcccg agattctcag 60
atctgatttc cacgcttgct accaaaatag tctgggcagg ccacttttgg aagtaggcgt 120
tatctagtga gcaggcggcc gctttcgatt tcgctttccc ctaaatggct gagcttctcg 180
ccagcgcagg atcagcctgt tcctgggact ttccgagagc cccgccctcg ttccctcccc 240
cagccgccag taggggagga ctcggcggta cccggagctt caggccccac cggggcgcgg 300
agagtcccag acccggccgg gaccgggacg gcgtccgagt gccaatggct agctctaggt 360
gtcccgctcc ccgcgggtgc cgctgcctcc ccggagcttc tctcgcatgg ctggggacag 420
tactgctact tctcgccgac tgggtgctgc tccggaccgc gctgccccgc atattctccc 480
tgctggtgcc caccgcgctg ccactgctcc gggtctgggc ggtgggcctg agccgctggg 540
ccgtgctctg gctgggggcc tgcggggtcc tcagggcaac ggttggctcc aagagcgaaa 600
acgcaggtgc ccagggctgg ctggctgctt tgaagccatt agctgcggca ctgggcttgg 660
ccctgccggg acttgccttg ttccgagagc tgatctcatg gggagccccc gggtccgcgg 720
atagcaccag gctactgcac tggggaagtc accctaccgc cttcgttgtc agttatgcag 780
cggcactgcc cgcagcagcc ctgtggcaca aactcgggag cctctgggtg cccggcggtc 840
agggcggctc tggaaaccct gtgcgtcggc ttctaggctg cctgggctcg gagacgcgcc 900
gcctctcgct gttcctggtc ctggtggtcc tctcctctct tggggagatg gccattccat 960
tctttacggg ccgcctcact gactggattc tacaagatgg ctcagccgat accttcactc 1020
gaaacttaac tctcatgtcc attctcacca tagccagtgc agtgctggag ttcgtgggtg 1080
acgggatcta taacaacacc atgggccacg tgcacagcca cttgcaggga gaggtgtttg 1140
gggctgtcct gcgccaggag acggagtttt tccaacagaa ccagacaggt aacatcatgt 1200
ctcgggtaac agaggacacg tccaccctga gtgattctct gagtgagaat ctgagcttat 1260
ttctgtggta cctggtgcga ggcctatgtc tcttggggat catgctctgg ggatcagtgt 1320
ccctcaccat ggtcaccctg atcaccctgc ctctgctttt ccttctgccc aagaaggtgg 1380
gaaaatggta ccagttgctg gaagtgcagg tgcgggaatc tctggcaaag tccagccagg 1440
tggccattga ggctctgtcg gccatgccta cagttcgaag ctttgccaac gaggagggcg 1500
aagcccagaa gtttagggaa aagctgcaag aaataaagac actcaaccag aaggaggctg 1560
tggcctatgc agtcaactcc tggaccacta gtatttcagg tatgctgctg aaagtgggaa 1620
tcctctacat tggtgggcag ctggtgacca gtggggctgt aagcagtggg aaccttgtca 1680
catttgttct ctaccagatg cagttcaccc aggctgtgga ggtactgctc tccatctacc 1740
ccagagtaca gaaggctgtg ggctcctcag agaaaatatt tgagtacctg gaccgcaccc 1800
ctcgctgccc acccagtggt ctgttgactc ccttacactt ggagggcctt gtccagttcc 1860
aagatgtctc ctttgcctac ccaaaccgcc cagatgtctt agtgctacag gggctgacat 1920
tcaccctacg ccctggcgag gtgacggcgc tggtgggacc caatgggtct gggaagagca 1980
cagtggctgc cctgctgcag aatctgtacc agcccaccgg gggacagctg ctgttggatg 2040
ggaagcccct tccccaatat gagcaccgct acctgcacag gcaggtggct gcagtgggac 2100
aagagccaca ggtatttgga agaagtcttc aagaaaatat tgcctatggc ctgacccaga 2160
agccaactat ggaggaaatc acagctgctg cagtaaagtc tggggcccat agtttcatct 2220
ctggactccc tcagggctat gacacagagg tagacgaggc tgggagccag ctgtcagggg 2280
gtcagcgaca ggcagtggcg ttggcccgag cattgatccg gaaaccgtgt gtacttatcc 2340
tggatgatgc caccagtgcc ctggatgcaa acagccagtt acaggtggag cagctcctgt 2400
acgaaagccc tgagcggtac tcccgctcag tgcttctcat cacccagcac ctcagcctgg 2460
tggagcaggc tgaccacatc ctctttctgg aaggaggcgc tatccgggag gggggaaccc 2520
accagcagct catggagaaa aaggggtgct actgggccat ggtgcaggct cctgcagatg 2580
ctccagaatg aaagccttct cagacctgcg cactccatct ccctcccttt tcttctctct 2640
gtggtggaga accacagctg cagagtagca gctgcctcca ggatgagtta cttgaaattt 2700
gccttgagtg tgttacctcc tttccaagct cctcgtgata atgcagactt cctggagtac 2760
aaacacagga tttgtaattc ctactgtaac ggagtttaga gccagggctg atgctttggt 2820
gtggccagca ctctgaaact gagaaatgtt cagaatgtac ggaaagatga tcagctattt 2880
tcaacataac tgaaggcata tgctggccca taaacaccct gtaggttctt gatatttata 2940
ataaaattgg tgttttgt 2958

<210> 41
<211> 1127
<212> DNA
<213> Homo sapiens
<400> 41
accaaatcaa ccataggtcc aagaacaatt gtctctggac ggcagctatg cgactcaccg 60

tgctgtgtgc tgtgtgcctg ctgcctggca gcctggccct gccgctgcct caggaggcgg 120
gaggcatgag tgagctacag tgggaacagg ctcaggacta tctcaagaga ttttatctct 180
atgactcaga aacaaaaaat gccaacagtt tagaagccaa actcaaggag atgcaaaaat 240
tctttggcct acctataact ggaatgttaa actcccgcgt catagaaata atgcagaagc 300
ccagatgtgg agtgccagat gttgcagaat actcactatt tccaaatagc ccaaaatgga 360
cttccaaagt ggtcacctac aggatcgtat catatactcg agacttaccg catattacag 420
tggatcgatt agtgtcaaag gctttaaaca tgtggggcaa agagatcccc ctgcatttca 480
ggaaagttgt atggggaact gctgacatca tgattggctt tgcgcgagga gctcatgggg 540
actcctaccc atttgatggg ccaggaaaca cgctggctca tgcctttgcg cctgggacag 600
gtctcggagg agatgctcac ttcgatgagg atgaacgctg gacggatggt agcagtctag 660
ggattaactt cctgtatgct gcaactcatg aacttggcca ttctttgggt atggacattt 720
cctctgatcc taatgcagtg atgtatccaa cctatggaaa tggagatccc caaaatttta 780
aactttccca ggatgatatt aaaggcattc agaaactata tggaaagaga agtaattcaa 840
gaaagaaata gaaacttcag gcagaacatc cattcattca ttcattggat tgtatatcat 900
tgttgcacaa tcagaattga taagcactgt tcctccactc catttagcaa ttatgtcacc 960
cttttttatt gcagttggtt tttgaatgtc tttcactcct tttattggtt aaactccttt 1020
atggtgtgac tgtgtcttat tccatctatg agctttgtca gtgcgcgtag atgtcaataa 1080
atgttacata cacaaataaa taaaatgttt attccatggt aaattta 1127

<210> 42
<211> 2710
<212> DNA
<213> Homo sapiens

<400> 42
cggagcacat gctgagcgga gcggctgggg ctgcgcggcg tggcggagca gcgctcgctc 60
cctcgctcac tcgctcgctc gcagggacac acgcaggggc tgacagctgt gctggtgctg 120
ataagggaag ccacaaggag acgatcgagg agagagacaa gcggcagcag aggcagcagc 180
ggcagaggca gcaccagggc tgcggagctg ctgggagtgg gagtgactcc cccacctcgg 240
gcccccaccc tgtccctgtc ctcttcccgc ttgccctgag tttagaagag cagccgctgc 300
caccactgcc actcgggagg gcaccagggc tgctggctag ggagggacag ggcagggagg 360
ctctggccag tcccagcagc cggggacaga tgccgatcga gattgtgtgc aaaatcaaat 420
ttgctgagga ggatgcgaaa cccaaggaga aggaggcagg ggatgagcag agcctcctcg 480
gggctgttgc ccctggagca gccccccgag acctggccac ctttgccagc accagcaccc 540
tgcatggact gggccgggcc tgtggcccag gcccccacgg actgcgcaga accctgtggg 600
cactggccct actcacctcg ctggctgcct tcctgtacca ggcggctggc ctggcccggg 660
gctacctgac ccggcctcac ctggtggcaa tggaccccgc tgccccagcc ccagtggcgg 720
gcttcccggc tgtcaccctc tgcaatatca accgcttccg gcattcggca ctcagcgatg 780
ccgacatctt ccacctggcc aatctgacag ggctgccccc caaagaccgg gatgggcacc 840
gtgcggctgg cctgcgctac ccagagcctg acatggtaga catcctcaac cgcactggcc 900
accagctcgc cgacatgctt aagagctgca acttcagtgg gcatcactgc tccgccagca 960
acttctctgt ggtctatact cgctatggga agtgttacac cttcaacgcg gacccgcgga 1020
gctcgctgcc cagccgggca gggggcatgg gcagtggcct ggagatcatg ctggacatcc 1080
agcaggagga gtacctgccc atctggaggg agacaaatga gacgtcgttt gaggcaggta 1140
ttcgggtgca gatccacagc caggaggagc cgccctacat ccaccagctg gggttcgggg 1200
tgtccccagg cttccagacc tttgtgtcct gccaggaaca gcggctgacc tacctgcccc 1260
agccctgggg caactgccgc gcagagagtg agctcaggga gcctgagctt cagggctact 1320
cggcctacag tgtgtctgcc tgccggctgc gctgtgaaaa ggaggccgtg cttcagcgct 1380
gccactgccg gatggtgcac atgccaggca atgagaccat ctgcccacca aatatctaca 1440
tcgagtgtgc agaccacaca ctggactccc tgggtggggg ccctgagggc ccgtgcttct 1500
gccccacccc ctgcaacctg acacgctatg ggaaagagat ctccatggtc aggatcccca 1560
acaggggctc agcccggtac ctggcgagga agtacaaccg caacgagacc tacatacggg 1620
agaacttcct ggtcctagat gtcttctttg aggccctgac ctctgaagcc atggagcagc 1680
gagcagccta tggcctgtca gccctgctgg gagacctcgg gggacagatg ggcctgttca 1740
ttggggccag catcctcacg ttgctggaga tcctcgacta catctatgag gtgtcctggg 1800
atcgactgaa gcgggtatgg aggcgtccca agacccccct gcggacctcc actgggggca 1860
tctccacttt ggggcttcag gagctgaagg aacagagtcc ctgcccgagc ctgggccgag 1920
cggagggtgg gggggtcagc agtctgctcc ccaatcacca ccacccccac ggtcccccag 1980
gaggtctctt tgaagatttt gcttgctagg acggtgctgt gactgaaagg acccaggagt 2040
ctgggacccc tcctgggatc cccagcacat tctcctgctc ctgggagagg cctgggggcg 2100
gtgctcactg ggagggccag gactcagttc ctgctctcat cctcccctgc cctgatgtca 2160
gctgctttgc acaaaggtcc ttcttgtcca caccccttat ccccaggctg gtgccccggg 2220
agggctggag accaggccat gggccctcac ggagaggaag ggaaggaagg agagggaggg 2280

ggaggataga gcccatccca gccggggagg gggagccctc tgtacatttg taaatattta 2340
gggaaagccg ggtgggggga ggggatacag atgtagaagg tgggtagggc tacaggggtg 2400
ggtgatttag ggacagccag ggtcccagcc ccaatgtcag caggataggg agagccccag 2460
gactcaggag tgctgggctg gtcctacttc ctgcccctct ccaggcccag ctcccctctt 2520
ggcaggggga gaggatggcc cagcaggcct ggcccagctc ccagttcccc ctgcaccagc 2580
cccaccccta gagtcccttc tatagggagg gggcaggaga ccttccagac ttcggctgag 2640
cttggagggt gggaagggag ccttctcagt cctctctccc tccagtctga ttttataaag 2700
tgctgacgag 2710

<210> 43
<211> 2462
<212> DNA
<213> Homo sapiens

<400> 43
gatctcagct ccttttcagc tagtgggtgg aaccccagga gggaaaactc agggaagccc 60
aggaggaagt gaaaataccc catggagtga agcttgtgtg ctacctgggt tccgggccag 120
tgatccagct cctgggggc atcagccacg gccaggcagg ggggcagctg ccaccaaagc 180
tggaggttct agaggacttg atggaggtca gctcaccctc acctgcccag aggctcagaa 240
ggaagaaaag gcccatggtg cagggccctg ctgggtgcca ggttttccag ccttctcctt 300
caggaggcac agcaggggac cctggtggcc tctctgaccc cttctaccct ccaagaagcg 360
gttccctggc ccttggcgac cccagctcgg accctgcatg ttcccagagt ggcccaatgg 420
aggctgaaga ggattctctt ccggagcagc cagaggactc agctcagctc caacaggaga 480
agccatccct gtatattggg gtgcggggca ctgttgtccg ttccatgcag gaggtactat 540
ggactcgcct tcgggagctc ccagacccag tgctgagtga ggaggtggtg gagggcattg 600
ctgctggcat tgaggcagcc ctctgggacc tgacacaagg caccaatggc cggtacaaga 660
ccaagtatcg cagcctgctg ttcaacctgc gggaccccag gaacctggac ttgtttctca 720
aagtggttca tggagatgtc accccctacg acctggtgcg gatgagctcg atgcagctgg 780
ccccccagga gctggcccgc tggcgggacc aggaggagaa aaggggcctg aatatcattg 840
agcagcaaca gaaggagccg tgcagacttc cagcctccaa aatgacccac aagggcgaag 900
tggagattca gcgggacatg gaccagacac tgaccctgga ggatctggtg ggaccgcaga 960
tgttcatgga ctgcagccca caggccctgc ccatcgcatc agaggacacc acggggcagc 1020
atgaccacca cttcttagac cccaactgcc acatctgcaa ggactgggag ccctcgaatg 1080
agctgctagg ctccttcgaa gccgccaaga gctgcgggga caatatcttc cagaaagccc 1140
taagccaaac tcctatgcct gctccagaga tgcccaaaac cagggagttg tctcccacgg 1200
aaccacagga cagggtccct ccatctgggc tccatgtgcc tgctgcaccc acaaaggccc 1260
tgccctgcct gccaccctgg gaaggtgttc tggacatgtt ctccatcaag cggttccggg 1320
ccagggccca gctggtctcg ggacacagct gtcggcttgt ccaggctctg cccaccgtga 1380
tccgctcggc aggctgcatc ccctccaaca ttgtctggga ccttctggcc agcatctgcc 1440
cagccaaggc caaggacgtc tgcgtggtca gactgtgccc acatggggcc cgggacaccc 1500
agaactgccg cctgctctac tcatacctca atgataggca gcgccacggg ctggcctctg 1560
tggagcacat ggggatggtc ctgctgcccc tgcctgcctt ccagcccctg cccaccaggc 1620
tgcgcccttt gggggggccca ggtctggagg tcactcactc aagtctgttg ctggctgtgc 1680
tgctccccaa ggaagggctt ccagacacag cagggtccag cccctggttg gggaaggttc 1740
aaaagatggt ctccttcaac agtaaggtgg agaagagata ctatcagcca gatgacagga 1800
ggccgaatgt gcccctgaag ggcacccctc ccccaggagg tgcctggcag cagagccagg 1860
gcaggggcag tatagctcca aggggaatct ctgcttggca gaggcccccc agaggcaggg 1920
ggaggctctg gccagagcct gaaaactggc agcatcctgg gcgagggcag tggcccccag 1980
agccaggctt gcgccagtcc cagcatccct attcagtagc accagctggt catggctttg 2040
gccgtggcca gcacttccac agggactcct gtccccacca agccctgctc cggcacctcg 2100
aatccctggc gaccatgagt caccagctcc aagccttact gtgcccccag accaagagct 2160
ccatcccccg ccctctgcag cgtttgtcta gcgcccttgc agctccagag ccccctggcc 2220
cagcccgtga ctcctctttg gggcctacag atgaagctgg ctctgagtgt cccttcccta 2280
gaaaggcctg accctcctta cccaccagaa caggggtttt gatgccctca ctagtgttga 2340
agcctgttcc agagagaggt gggactgcaa ggagaggatg gtcagcccta cccacctgcc 2400
ctgtttgaac ttcctgtttg acaatgtttg ctgttgattt tttgttcaat aaagaatttg 2460
gt 2462
<210> 44
<211> 2070
<212> DNA
<213> Homo sapiens

<400> 44

ccgacaccca cgggcggaga tcacctgctg ccccgcagac ccctgtccct tcctcccgga 60
ccagcagcta gaggatgtcc aaacggagtt ggtgggctgg atccagaaag cccccaagag 120
agatgctgaa actctcaggc tctgactcca gccaaagcat gaatggcctt gaagtggctc 180
ccccaggtct gatcaccaac ttctccctgg ccacggcaga gcaatgtggc caggagacgc 240
cactggagaa catgctgttc gcctccttct accttctgga ttttatcctg gctttagttg 300
gcaataccct ggctctgtgg cttttcatcc gagaccacaa gtccggaccc ccggccaacg 360
tgttcctgat gcatctggcc gtggccgact tgtcgtgcgt gctggtcctg cccacccgcc 420
tggtctacca cttctctggg aaccactggc catttgggga aatcgcatgc cgtctcaccg 480
gcttcctctt ctacctcaac atgtacgcca gcatctactt cctcacctgc atcagcgccg 540
accgtttcct ggccattgtg cacccggtca agtccctcaa gctccgcagg cccctctacg 600
cacacctggc ctgtgccttc ctgtgggtgg tggtggctgt ggccatggcc ccgctgctgg 660
tgagcccaca gaccgtgcag accaaccaca cggtggtctg cctgcagctg taccgggaga 720
aggcctccca ccatgccctg gtgtccctgg cagtggcctt caccttcccg ttcatcacca 780
cggtcacctg ctacctgctg atcatccgca gcctgcggca gggcctgcgt gtggagaagc 840
gcctcaagac caaggcagtg cgcatgatcg ccatagtgct ggccatcttc ctggtctgct 900
tcgtgcccta ccacgtcaac cgctccgtct acgtgctgca ctaccgcagc catggggcct 960
cctgcgccac ccagcgcatc ctggccctgg caaaccgcat cacctcctgc ctcaccagcc 1020
tcaacggggc actcgacccc atcatgtatt tcttcgtggc tgagaagttc cgccacgccc 1080
tgtgcaactt gctctgtggc aaaaggctca agggcccgcc ccccagcttc gaagggaaaa 1140
ccaacgagag ctcgctgagt gccaagtcag agctgtgagc ggggggcgcc gtccaggccg 1200
agcgcagact gtttaggact cagcagaccc agcaagaggc atctgccctt tccccagcca 1260
cctccccagc aagcaacctg aaatctcagc agatgcccac catttctcta gatcgcctag 1320
tctcaaccca taaaaaggaa gaactgacaa aggggatcca tcggccaccc ctctgcaggg 1380
gcttgtgatg gctacaatgg ctcctagaca ctcaacgact tcatctgtgg cagggagaga 1440
ggaggccgga agaacaaccc ctgaacaatg gaggcctttc tttcccgcta ggctcccagc 1500
ctccttcccg ctacagaatc gctcatcggc gaggctcagc agaaagaccc tgaaggcagg 1560
ctgcaaatga cccagaagag ggacctggga gtcctggtgg ggacggggag ggagtctcaa 1620
tactcctttg cagcgcaagg tactctgagt cccctctgta gtgcctctgc cagacacaca 1680
ctgcctgagt tgaagagaca caggccacac atttcaggct ggttgccagc ggacgtcagc 1740
actcacggcc tgcggggact cagcacagct ctggattctg gatctctcct gctgtaaccc 1800
cacgcacaag cctgcaaccc ccagagctct ttgacaggct cccaggcctc ccagtcctgg 1860
acaagcatgt gcagtcacgg gagctcagct caggccaggg ctgggctgtg cacctgcctc 1920
ccactgaccc agacccactt cctccagaga ggcctctctc cgcctgagct atttcccttg 1980
ctagtgtgca gatatttccc taacatgtcc ttttttgtat ttgtttgtac ggaccataaa 2040
tataactgta gctttaagac taaaaaaaaa 2070

<210> 45
<211> 884
<212> DNA
<213> Homo sapiens

<400> 45
ctactactac taaattcgcg gccggtcgac gctggtgcac aggaaggatg aggaagacca 60
ggctctgggg gctgctgtgg atgctctttg tctcagaact ccgagctgca actaaattaa 120
ctgaggaaaa gtatgaactg aaagaggggc agaccctgga tgtgaaatgt gactacacgc 180
tagagaagtt tgccagcagc cagaaagctt ggcagataat aagggacgga gagatgccca 240
agaccctggc atgcacagag aggccttcaa agaattccca tccagtccaa gtggggagga 300
tcatactaga agactaccat gatcatggtt tactgcgcgt ccgaatggtc aaccttcaag 360
tggaagattc tggactgtat cagtgtgtga tctaccagcc tcccaaggag cctcacatgc 420
tgttcgatcg catccgcttg gtggtgacca agggtttttc agggacccct ggctccaatg 480
agaattctac ccagaatgtg tataagattc ctcctaccac cactaaggcc ttgtgcccac 540
tctataccag ccccagaact gtgacccaag ctccacccaa gtcaactgcc gatgtctcca 600
ctcctgactc tgaaatcaac cttacaaatg tgacagatat catcagggtt ccggtgttca 660
acattgtcat tctcctggct ggtggattcc tgagtaagag cctggtcttc tctgtcctgt 720
ttgctgtcac gctgaggtca tttgtaccct aggcccacga acccacgaga atgtcctctg 780
acttccagcc acatccatct ggcagttgtg ccaagggagg agggaggagg taaaaggcag 840
ggagttaata acatgaatta aatctgtaat caccagctat ttct 884

<210> 46
<211> 1059
<212> DNA
<213> Homo sapiens

<400> 46
atggagggga tcagtatata cacttcagat aactacaccg aggaaatggg ctcaggggat 60
tatgactcca tgaaggaacc ctgtttccgt gaagaaaatg ctaatttcaa taaaatcttc 120
ctgcccacca tctactccat catcttctta actggcattg tgggcaatgg attggtcatc 180
ctggtcatgg gttaccagaa gaaactgaga agcatgacgg acaagtacag gctgcacctg 240
tcagtggccg acctcctctt tgtcatcacg cttcccttct gggcagttga tgccgtggca 300
aactggtact ttgggaactt cctatgcaag gcagtccatg tcatctacac agtcaacctc 360
tacagcagtg tcctcatcct ggccttcatc agtctggacc gctacctggc catcgtccac 420
gccaccaaca gtcagaggcc aaggaagctg ttggctgaaa aggtggtcta tgttggcgtc 480
tggatccctg ccctcctgct gactattccc gacttcatct ttgccaacgt cagtgaggca 540
gatgacagat atatctgtga ccgcttctac cccaatgact tgtgggtggt tgtgttccag 600
tttcagcaca tcatggttgg ccttatcctg cctggtattg tcatcctgtc ctgctattgc 660
attatcatct ccaagctgtc acactccaag ggccaccaga agcgcaaggc cctcaagacc 720
acagtcatcc tcatcctggc tttcttcgcc tgttggctgc cttactacat tgggatcagc 780
atcgactcct tcatcctcct ggaaatcatc aagcaagggt gtgagtttga gaacactgtg 840
cacaagtgga tttccatcac cgaggcccta gctttcttcc actgttgtct gaaccccatc 900
ctctatgctt tccttggagc caaatttaaa acctctgccc agcacgcact cacctctgtg 960
agcagagggt ccagcctcaa gatcctctcc aaaggaaagc gaggtggaca ttcatctgtt 1020
tccactgagt ctgagtcttc aagttttcac tccagctaa 1059

<210> 47
<211> 1542
<212> DNA
<213> Homo sapiens

<400> 47
gtgtgtagtc gggtatgaga acgattgcaa gcaaaagcag ctgagggacg ctgtccaagg 60
agtaggctca gtagcacgaa ctgcaaatac ttgagaaggg aaaagttcag ccttgtgctg 120
gaaagtggtt tagcatgctg gagctggttt tctgcttggt agccctacaa ctttggccca 180
actacttggc ctctgggact cagattcctc ctctttaaaa tggtgctaat aatagcaccc 240
accctctgtg aggaggatgc tgtgaggaca aaatgagatc atccacataa gccgtgaacc 300
ctgttcctgg taagctcctg gaaagaagtc tatggattat actcaaccta cactccagtt 360
aaaggaacat ctacacacag aggaaatgaa taacatgaag tgaagtcttc atctccattc 420
ccaacagtcc ccattctact tgcagaaagg ttgcttacac tgaaaatcag tttattttcc 480
cctggtgcaa agaacagtcg tttctccaaa actgaagctg gaaattatct gaaatatcag 540
gtcctccgga aaagggacgt gaagccccct ttgtaatttc tgcattagcg tgctctcctg 600
gcaagcagga aacctcatca gagaagtcag ccaaggaaag tctttaaatg gaaattgtgc 660
aaacgaggag caaatgcatt aaaaagttgc tgacgggcat gaaatgcttt gatgtgaaga 720
cggaaaactc caagcaggaa ggattttaac attttgaatc tgattgactc tgtggtttct 780
cagcacagtt attccatggg ctaaaataaa tgcagaaatg gtactttcag accacagctg 840
cagaggggat cgtggtgaat ttcaatgaaa atccatttga atcttgaggt tcagatctta 900
aaaaagcaaa ggacatgaga gaagtaatat tgttgcttga aatttcattg cttatatcta 960
aaagaaactc ctatttttaa gagaaatgtt gaatctttgc aacgtggtag acgctcccac 1020
aaaactttct cctgaaatag gagataaatg ttggaaagag gcaatgtatt gagtatgctg 1080
atagaggtgg agttcagaga caggcaagca tacataagag tcaggatgtt tttcagtatt 1140
atctttacaa atgagtttct tacagtggtc aatgacaaac caatttcatt caaagcttgc 1200
ttcaataggc aatggtttga tgccaatatg ttagctattt actttgacca ccgtatgcat 1260
taaaaagaag aaaaattaag aatactcaag cagaacctcc aacttagata gcactttcca 1320
caaaaagtaa tggagggata gactgaagtt aaatgggatc aggtatgtga tgagatctca 1380
gaagtgtttg cacaataatg cagatactca ttttaaacag agtcataagg attggaacta 1440
ataaaaataa tagaataaaa taccgatcaa gaatgtgtcc tcctgcgtat ctgggttttg 1500
aattctggct ccacagaact tgtcagatat atgacattaa ac 1542

<210> 48
<211> 1080
<212> DNA
<213> Homo sapiens

<400> 48
atgcatcctc aagtggtcat cttaagcctc atcctacatc tggcagattc tgtagctggt 60
tctgtaaagg ttggtggaga ggcaggtcca tctgtcacac taccctgcca ctacagtgga 120
gctgtcacat caatgtgctg gaatagaggc tcatgttctc tattcacatg ccaaaatggc 180
attgtctgga ccaatggaac ccacgtcacc tatcggaagg acacacgcta taagctattg 240

ggggaccttt caagaaggga tgtctctttg accatagaaa atacagctgt gtctgacagt 300
ggcgtatatt gttgccgtgt tgagcaccgt gggtggttca atgacatgaa aatcaccgta 360
tcattggaga ttgtgccacc caaggtcacg actactccaa ttgtcacaac tgttccaacc 420
gtcacgactg ttcgaacgag caccactgtt ccaacgacaa cgactgttcc aacgacaact 480
gttccaacaa caatgagcat tccaacgaca acgactgttc cgacgacaat gactgtttca 540
acgacaacga gcgttccaac gacaacgagc attccaacaa caacaagtgt tccagtgaca 600
acaacggtct ctacctttgt tcctccaatg cctttgccca ggcagaacca tgaaccagta 660
gccacttcac catcttcacc tcagccagca gaaacccacc ctacgacact gcagggagca 720
ataaggagag aacccaccag ctcaccattg tactcttaca caacagatgg gaatgacacc 780
gtgacagagt cttcagatgg cctttggaat aacaatcaaa ctcaactgtt cctagaacat 840
agtctactga cggccaatac cactaaagga atctatgctg gagtctgtat ttctgtcttg 900
gtgcttcttg ctcttttggg tgtcatcatt gccaaaaagt atttcttcaa aaaggaggtt 960
caacaactaa gtgtttcatt tagcagcctt caaattaaag ctttgcaaaa tgcagttgaa 1020
aaggaagtcc aagcagaaga caatatctac attgagaata gtctttatgc cacggactaa 1080

<210> 49
<211> 4521
<212> DNA
<213> Homo sapiens

<400> 49
acttcattca cttgcaaatc agtgtgtgcc cacaagagcc agctctcccg agcccgtaac 60
cttcgcatcc caagagctgc agtttcagcc gcgacagcaa gaacggcaga gccggcgacc 120
gcggcggcgg cggcggcgga ggcaggagca gcctgggcgg gtcgcagggt ctccgcgggc 180
gcaggaaggc gagcagagat atcctctgag agccaagcaa agaacattaa ggaaggaagg 240
aggaatgagg ctggatacgg tgcagtgaaa aaggcacttc caagagtggg gcactcacta 300
cgcacagact cgacggtgcc atcagcatga gaacttaccg ctacttcttg ctgctctttt 360
gggtgggcca gccctaccca actctctcaa ctccactatc aaagaggact agtggtttcc 420
cagcaaagaa aagggccctg gagctctctg gaaacagcaa aaatgagctg aaccgttcaa 480
aaaggagctg gatgtggaat cagttctttc tcctggagga atacacagga tccgattatc 540
agtatgtggg caagttacat tcagaccagg atagaggaga tggatcactt aaatatatcc 600
tttcaggaga tggagcagga gatctcttca ttattaatga aaacacaggc gacatacagg 660
ccaccaagag gctggacagg gaagaaaaac ccgtttacat ccttcgagct caagctataa 720
acagaaggac agggagaccc gtggagcccg agtctgaatt catcatcaag atccatgaca 780
tcaatgacaa tgaaccaata ttcaccaagg aggtttacac agccactgtc cctgaaatgt 840
ctgatgtcgg tacatttgtt gtccaagtca ctgcgacgga tgcagatgat ccaacatatg 900
ggaacagtgc taaagttgtc tacagtattc tacagggaca gccctatttt tcagttgaat 960
cagaaacagg tattatcaag acagctttgc tcaacatgga tcgagaaaac agggagcagt 1020
accaagtggt gattcaagcc aaggatatgg gcggccagat gggaggatta tctgggacca 1080
ccaccgtgaa catcacactg actgatgtca acgacaaccc tccccgattc ccccagagta 1140
cataccagtt taaaactcct gaatcttctc caccggggac accaattggc agaatcaaag 1200
ccagcgacgc tgatgtggga gaaaatgctg aaattgagta cagcatcaca gacggtgagg 1260
ggctggatat gtttgatgtc atcaccgacc aggaaaccca ggaagggatt ataactgtca 1320
aaaagctctt ggactttgaa aagaagaaag tgtataccct taaagtggaa gcctccaatc 1380
cttatgttga gccacgattt ctctacttgg ggcctttcaa agattcagcc acggttagaa 1440
ttgtggtgga ggatgtagat gagccacctg tcttcagcaa actggcctac atcttacaaa 1500
taagagaaga tgctcagata aacaccacaa taggctccgt cacagcccaa gatccagatg 1560
ctgccaggaa tcctgtcaag tactctgtag atcgacacac agatatggac agaatattca 1620
acattgattc tggaaatggt tcgattttta catcgaaact tcttgaccga gaaacactgc 1680
tatggcacaa cattacagtg atagcaacag agatcaataa tccaaagcaa agtagtcgag 1740
tacctctata tattaaagtt ctagatgtca atgacaacgc cccagaattt gctgagttct 1800
atgaaacttt tgtctgtgaa aaagcaaagg cagatcagtt gattcagacc ctgcatgctg 1860
ttgacaagga tgacccttat agtggacacc aattttcgtt ttccttggcc cctgaagcag 1920
ccagtggctc aaactttacc attcaagaca acaaagacaa cacggcggga atcttaactc 1980
ggaaaaatgg ctataataga cacgagatga gcacctatct cttgcctgtg gtcatttcag 2040
acaacgacta cccagttcaa agcagcactg ggacagtgac tgtccgggtc tgtgcatgtg 2100
accaccacgg gaacatgcaa tcctgccatg cggaggcgct catccacccc acgggactga 2160
gcacgggggc tctggttgcc atccttctgt gcatcgtgat cctactagtg acagtggtgc 2220
tgtttgcagc tctgaggcgg cagcgaaaaa aagagccttt gatcatttcc aaagaggaca 2280
tcagagataa cattgtcagt tacaacgacg aaggtggtgg agaggaggac acccaggctt 2340
ttgatatcgg caccctgagg aatcctgaag ccatagagga caacaaatta cgaagggaca 2400
ttgtgcccga agcccttttc ctaccccgac ggactccaac agctcgcgac aacaccgatg 2460
tcagagattt cattaaccaa aggttaaagg aaaatgacac ggaccccact gccccgccat 2520

acgactcctt ggccacttac gcctatgaag gcactggctc cgtggcggat tccctgagct 2580
cgctggagtc agtgaccacg gatgcagatc aagactatga ttaccttagt gactggggac 2640
ctcgattcaa aaagcttgca gatatgtatg gaggagtgga cagtgacaaa gactcctaat 2700
ctgttgcctt tttcattttc caatacgaca ctgaaatatg tgaagtggct atttctttat 2760
atttatccac tactccgtga aggcttctct gttctacccg ttccaaaagc caatggctgc 2820
agtccgtgtg gatccaatgt tagagacttt tttctagtac acttttatga gcttccaagg 2880
ggcaaatttt tattttttag tgcatccagt taaccaagtc agcccaacag gcaggtgccg 2940
gaggggagga cagggaacag tatttccact tgttctcagg gcagcgtgcc cgcttccgct 3000
gtcctggtgt tttactacac tccatgtcag gtcagccaac tgccctaact gtacatttca 3060
caggctaatg ggataaagga ctgtgcttta aagataaaaa tatcatcata gtaaaagaaa 3120
tgagggcata tcggctcaca aagagataaa ctacataggg gtgtttattt gtgtcacaaa 3180
gaatttaaaa taacacttgc ccatgctatt tgttcttcaa gaactttctc tgccatcaac 3240
tactattcaa aacctcaaat ccacccatat gttaaaattc tcattactct taaggaatag 3300
aagcaaatta aacggtaaca tccaaaagca accacaaacc tagtacgact tcattccttc 3360
cactaactca tagtttgtta tatcctagac tagacatgcg aaagtttgcc tttgtaccat 3420
ataaaggggg agggaaatag ctaataatgt taaccaagga aatatatttt accatacatt 3480
taaagttttg gccaccacat gtatcacggg tcacttgaaa ttctttcagc tatcagtagg 3540
ctaatgtcaa aattgtttaa aaattcttga aagaattttc ctgagacaaa ttttaacttc 3600
ttgtctatag ttgtcagtat tattctacta tactgtacat gaaagtagca gtgtgaagta 3660
caataattca tattcttcat atccttctta cacgactaag ttgaattagt aaagttagat 3720
taaataaaac ttaaatctca ctctaggagt tcagtggaga ggttagagcc agccacactt 3780
gaacctaata ccctgccctt gacatctgga aacctctaca tatttatata acgtgataca 3840
tttggataaa caacattgag attatgatga aaacctacat attccatgtt tggaagaccc 3900
ttggaagagg aaaattggat tcccttaaac aaaagtgttt aagattgtaa ttaaaatgat 3960
agttgatttt caaaagcatt aatttttttt cattgttttt aactttgctt tcatgaccat 4020
cctgccatcc ttgactttga actaatgata aagtaatgat ctcaaactat gacagaaaag 4080
taatgtaaaa tccatccaat ctattatttc tctaattatg caattagcct catagttatt 4140
atccagagga cccaactgaa ctgaactaat ccttctggca gattcaaatc gtttatttca 4200
cacgctgttc taatggcact tatcattaga atcttacctt gtgcagtcat cagaaattcc 4260
agcgtactat aatgaaaaca tccttgtttt gaaaacctaa aagacaggct ctgtatatat 4320
atatacttaa gaatatgctg acttcactta ttagtcttag ggatttattt tcaattaata 4380
ttaattttct acaaataatt ttagtgtcat ttccatttgg ggatattgtc atatcagcac 4440
atattttctg tttggaaaca cactgttgtt tagttaagtt ttaaataggt gtattaccca 4500
agaagtaaag atggaaacgt t 4521

<210> 50
<211> 2343
<212> DNA
<213> Homo sapiens

<400> 50
tttttttttt tttttagaaa aaaaccagtt tattaagtaa tatcatataa ataacttgtt 60
acaaaaattg atttgcaaaa ggcatattta ctctttgtga gaaatcactt tttaaattgc 120
attcttttca aatttataag tctaagaaaa caaaaccaaa taaaagaagc catttcaagg 180
agtgcgtatt tgccatttga ctgcaacaaa aggcccggcc acactgagct aaaaggtaat 240
actctcgacc ccattcttct aacacagaaa actttctcag gtaaactgtg gggttatgag 300
aatcccccta actagaaatg ttgatgggaa ctgagcattg cttgctttca tcaggtgttc 360
ttgttgccaa agacatgaac gatactgagg aaaacgacaa gagtgagcat tcccgccagt 420
aaatcttcaa gggtggcatc cgtttcaatt tatacttgga agtattttta attaaaaaca 480
atcaatacca aaaagctttt attttgtggg tttaaaagtc acaaatcaca gtgggagaat 540
gccaaattgc tttagcttgg aactactgaa gacgcacata gcatttatta taaggcctac 600
tcttaggcag ttcactctca aagcaatgaa aataatctca aaccaaacat tacagtgggt 660
ttgaagcgtt cctacgtttc ttccgagcag gtcagtttta catttgctac acagcattcc 720
ccacgaatgc ctggtaattc tatacatttg attctttaat aaacactaaa ctaatagatc 780
atagaaaact aaaagcttag agaaggtgcc tccagacata tttacataaa taacgtagcc 840
tcacaagaaa gaccaagatc tcattagcgt ggaatgcttt ttccacaagg ctgggtccat 900
gcctcatttg tcagattaac cccatttgag gagaaatttg agtttgtggt tcatgggttt 960
ttgaaaaaaa aaaaaaaaaa aaaattaagc aacttgtaaa agctcttttg aaattaatct 1020
aataacccag tggctcctcg gctaagtgcc tcagtcctgt ctgaaataca gcgggtaaga 1080
gcctttgttt ccatttgacc tcttttcaac actttcatct gccggctctg ttgggctaac 1140
gtttccccca aataaaaatg aaaatgcacc ataaataagg tattacaaaa aggggagtag 1200
attaaagatt gtctcagcaa tgcttaaatt aaattgacga tgcagagacc ataaacttcc 1260
ggctttaaaa atgagctatt tctttattaa cttttaacaa gtgtatgagt caatgtattt 1320

tctttctaag agggtattgc tctccacatt aaatatggtc ggggtctgat atttttagta 1380
gagaattacc ctttagaaaa ttattccata aagatgccat agggagatta aaaatgctgt 1440
gaccataaga gctactaaac atttaaggag accataatat aattctatat tctctggaaa 1500
aggaagctgc ttgtttgagc ttatgaaata gctcattttc ctgaagattc acacaccaag 1560
tgttaatttt tctactctta taatctgata aggaaggttg tggtgttaat ttaaacacag 1620
tagactgctt ggatttttga gtcccctttt ataatatcac aattcaaaca agatttcatc 1680
ttgataaaca taaaaattaa gattacacta taatgatcct tgttagagga tacatttcaa 1740
aatagtgggg gaatcatgac tttttagagc cttcttccag agtaattaca ctgcttcaaa 1800
actagttatt aggataaaat aatttatttg gtggtctgtt cctggtgaaa atgaatttag 1860
ctgataagga tgagtacctg atttgaatat ttagttggca cccaggagtc aactttgatt 1920
ttcttttcaa ctgcatactt tttatggtgc ttgagtatat agaattgtcc aagtcatagt 1980
ttacacacca ctatccccag tgggatatct atttccaggc aaagcatttg atgtgagata 2040
ttgttcagcc aatcaggaca cttaaaattt atatatgtaa aagtgaaagt aatagaaaca 2100
ttctaaagtt aatttggatt tataaaattt ggatggccta agctacagat tcaattttta 2160
aggccgggcg cggtggctca cgcctgtaat cccagcactt tgggaggccg aggcgggaga 2220
atggcgtgaa cccgggaggc ggagcttgta gtgagcagag atcgcgccac tgcattccag 2280
cctgggcgac agagcgagat tctgtctcaa aaaaaaaaaa aaaaagaaac ttatcgatac 2340
ccc 2343

<210> 51
<211> 2746
<212> DNA
<213> Homo sapiens

<400> 51
ggcatcccga gtgcaccgct cccgccccgc cccgccttgc cccaacccac gatggtctgg 60
gagctgcgcc cagggcttgg cgctggcggc cccgcaacag caccgagcgt ttcggtcggc 120
gggcggcggt agcgccccct ctcagagccc cgctcactcc cacctgggct cgctccgagt 180
cggcctgtct gtcgggcccg ccctccccgc tcactccctc cgcctcgtgc tcctcccggg 240
gtgcttggca cagcctcgga ttcctccctc tcgctgctcg agtcagtttc cctatcggcg 300
gcagcgggca aggcggcggc ggcggcggcg gcagccgcgg tggcggcgtg gggaacatct 360
cggcagccac cgcgcttctc ccgctggagc gggcgtccag cttggctgcc ctcggtcctt 420
ccctgccacg tttcgggtcg ccctgcaccc cccacccagg ctcgcttctc ttcgaagcgg 480
gaagggcgcc ttgcaggatc ctgccgcccc tccaaccgga tcctgggtct agagctcccc 540
agagcgaggc gctcgccagg actcctgccc cgccaaccct gaccgccggg gggtgccccc 600
gggacgtagc gccgcggaga ggaagcggca aaggggacca tgcggcgcct gactcgtcgg 660
ctggttctgc cagtcttcgg ggtgctctgg atcacggtgc tgctgttctt ctgggtaacc 720
aagaggaagt tggaggtgcc gacgggacct gaagtgcaga cccctaaggt ttggtccttg 780
tttttcaagg tggctgggat gagcccttgg gcgcctcagg tgcctgtatc acccactcct 840
ccctaccaaa gagggcatct tcctacagga ggacaccttg ctgtatgtca tttcccatgt 900
ctcttgcaag aagctcagtt ccatttgcag actcaggtct ttcttcaagt cagatgcaca 960
ctgctggtgt attgcacgga ccttccaccc actagcatca tcatcacctt ccacaacgag 1020
gcccgctcca cgctgctcag gaccatccgc agtgtattaa accgcacccc tacgcatctg 1080
atccgggaaa tcatattagt ggatgacttc agcaatgacc ctgatgactg taaacagctc 1140
atcaaattgc ccaaggtgaa atgcttgcgc aataatgaac ggcaaggtct ggtccggtcc 1200
cggattcggg gcgctgacat cgcccagggc accactctga ctttcctcga cagccactgt 1260
gaggtgaaca gggactggct ccagcctctg ttgcacaggg tcaaagagga ctacacgcgg 1320
gtggtgtgcc ctgtgatcga tatcattaac ctggacacct tcacctacat cgagtctgcc 1380
tcggagctca gaggggggtt tgactggagc ctccacttcc agtgggagca gctctcccca 1440
gagcagaagg ctcggcgcct ggaccccacg gagcccatca ggactcctat catagctgga 1500
gggctcttcg tgatcgacaa agcttggttt gattacctgg ggaaatatga tatggacatg 1560
gacatctggg gtggggagaa ctttgaaatc tccttccgag tgtggatgtg cgggggcagc 1620
ctagagatcg tcccctgcag ccgagtgggg cacgtcttcc ggaagaagca cccctacgtt 1680
ttccctgatg gaaatgccaa cacgtatata aagaacacca agcggacagc tgaagtgtgg 1740
atggatgaat acaagcaata ctattacgct gcccggccat tcgccctgga gaggcccttc 1800
gggaatgttg agagcagatt ggacctgagg aagaatctgc gctgccagag cttcaagtgg 1860
tacctggaga atatctaccc tgaactcagc atccccaagg agtcctccat ccagaagggc 1920
aatatccgac agagacagaa gtgcctggaa tctcaaaggc agaacaacca agaaacccca 1980
aacctaaagt tgagcccctg tgccaaggtc aaaggcgaag atgcaaagtc ccaggtatgg 2040
gccttcacat acacccagca gatcctccag gaggagctgt gcctgtcagt catcaccttg 2100
ttccctggcg ccccagtggt tcttgtcctt tgcaagaatg gagatgaccg acagcaatgg 2160
accaaaactg gttcccacat cgagcacata gcatcccacc tctgcctcga tacagatatg 2220
ttcggtgatg gcaccgagaa cggcaaggaa atcgtcgtca acccatgtga gtcctcactc 2280

atgagccagc actgggacat ggtgagctct tgaggacccc tgccagaagc agcaagggcc 2340
atggggtggt gcttccctgg accagaacag actggaaact gggcagcaag cagcctgcaa 2400
ccacctcaga catcctggac tgggaggtgg aggcagagcc ccccaggaca ggagcaactg 2460
tctcagggag gacagaggaa aacatcacaa gccaatgggg ctcaaagaca aatcccacat 2520
gttctcaagg ccgttaagtt ccagtcctgg ccagtcattc cctgattggt atctggagac 2580
agaaacctaa tgggaagtgt ttattgttcc ttttcctaca aaggaagcag tctctggagg 2640
ccagaaagaa aagccttctt tttcactagg ccaggactac attgagagat gaagaatgga 2700
ggttgtttcc aaaagaaata aagagaaact tagaagttgt ctctgg 2746

<210> 52
<211> 2741
<212> DNA
<213> Homo sapiens

<400> 52
ggcatcccga gtgcaccgct cccgccccgc cccgccttgc cccaacccac gatggtctgg 60
gagctgcgcc cagggcttgg cgctggcggc cccgcaacag caccgagcgt ttcggtcggc 120
gggcggcggt agcgccccct ctcagagccc cgctcactcc cacctgggct cgctccgagt 180
cggcctgtct gtcgggcccg ccctccccgc tcactccctc cgccctcgtg ctcctcccgg 240
ggtgcttggc acagcctcgg attcctccct ctcgctgctc gagtcagttt ccctatcggc 300
ggcagcgggc aaggcggcgg cggcggcggc ggcagccgcg gtggcggcgt ggggaacatc 360
tcggcagcca ccgcgcttct cccgctggag cgggcgtcca gcttggctgc cctcggtcct 420
tccctgccac gtttcgggtc gccctgcacc ccccacccag gctcgcttct cttcgaagcg 480
ggaagggcgc cttgcaggat cctgccgccc ctccaaccgg atcctgggtc tagagctccc 540
cagagcgagg cgctcgccag gactcctgcc ccgccaaccc tgaccgccgg ggggtgcccc 600
cgggacgtag cgccgcggag aggaagcggc aaaggggacc atgcggcgcc tgactcgtcg 660
gctggttctg ccagtcttcg gggtgctctg gatcacggtg ctgctgttct tctgggtaac 720
caagaggaag ttggaggtgc cgacgggacc tgaagtgcag acccctaagc cttcggacgc 780
tgactgggac gacctgtggg accagtttga tgagcggcgg tatctgaatg ccaaaaagtg 840
gcgcgttggt gacgacccct ataagctgta tgctttcaac cagcgggaga gtgagcggat 900
ctccagcaat cgggccatcc cggacactcg ccatctgaga tgcacactgc tggtgtattg 960
cacggacctt ccacccacta gcatcatcat caccttccac aacgaggccc gctccacgct 1020
gctcaggacc atccgcagtg tattaaaccg cacccctacg catctgatcc gggaaatcat 1080
attagtggat gacttcagca atgaccctga tgactgtaaa cagctcatca aattgcccaa 1140
ggtgaaatgc ttgcgcaata atgaacggca aggtctggtc cggtcccgga ttcggggcgc 1200
tgacatcgcc cagggcacca ctctgacttt cctcgacagc cactgtgagg tgaacaggga 1260
ctggctccag cctctgttgc acagggtcaa agaggactac acgcgggtgg tgtgccctgt 1320
gatcgatatc attaacctgg acaccttcac ctacatcgag tctgcctcgg agctcagagg 1380
ggggtttgac tggagcctcc acttccagtg ggagcagctc tccccagagc agaaggctcg 1440
gcgcctggac cccacggagc ccatcaggac tcctatcata gctggagggc tcttcgtgat 1500
cgacaaagct tggtttgatt acctggggaa atatgatatg gacatggaca tctggggtgg 1560
ggagaacttt gaaatctcct tccgagtgtg gatgtgcggg ggcagcctag agatcgtccc 1620
ctgcagccga gtggggcacg tcttccggaa gaagcacccc tacgttttcc ctgatggaaa 1680
tgccaacacg tatataaaga acaccaagcg gacagctgaa gtgtggatgg atgaatacaa 1740
gcaatactat tacgctgccc ggccattcgc cctggagagg cccttcggga atgttgagag 1800
cagattggac ctgaggaaga atctgcgctg ccagagcttc aagtggtacc tggagaatat 1860
ctaccctgaa ctcagcatcc ccaaggagtc ctccatccag aagggcaata tccgacagag 1920
acagaagtgc ctggaatctc aaaggcagaa caaccaagaa accccaaacc taaagttgag 1980
cccctgtgcc aaggtcaaag gcgaagatgc aaagtcccag gtatgggcct tcacatacac 2040
ccagcagatc ctccaggagg agctgtgcct gtcagtcatc accttgttcc ctggcgcccc 2100
agtggttctt gtcctttgca agaatggaga tgaccgacag caatggacca aaactggttc 2160
ccacatcgag cacatagcat cccacctctg cctcgataca gatatgttcg gtgatggcac 2220
cgagaacggc aaggaaatcg tcgtcaaccc atgtgagtcc tcactcatga gccagcactg 2280
ggacatggtg agctcttgag gacccctgcc agaagcagca agggccatgg ggtggtgctt 2340
ccctggacca gaacagactg gaaactgggc agcaagcagc ctgcaaccac ctcagacatc 2400
ctggactggg aggtggaggc agagcccccc aggacaggag caactgtctc agggaggaca 2460
gaggaaaaca tcacaagcca atggggctca aagacaaatc ccacatgttc tcaaggccgt 2520
taagttccag tcctggccag tcattccctg attggtatct ggagacagaa acctaatggg 2580
aagtgtttat tgttcctttt cctacaaagg aagcagtctc tggaggccag aaagaaaagc 2640
cttctttttc actaggccag gactacattg agagatgaag aatggaggtt gtttccaaaa 2700
gaaataaaga gaaacttaga agttgaaaaa aaaaaaaaaa a 2741

<210> 53

<211> 2385
<212> DNA
<213> Homo sapiens

<400> 53
ggcatcccga gtgcaccgct cccgccccgc cccgccttgc cccaacccac gatggtctgg 60
gagctgcgcc cagggcttgg cgctggcggc cccgcaacag caccgagcgt ttcggtcggc 120
gggcggcggt agcgccccct ctcagagccc cgctcactcc cacctgggct cgctccgagt 180
cggcctgtct gtcgggcccg ccctccccgc tcactccctc cgcccttctc ttcgaagcgg 240
gaagggcgcc ttgcaggatc ctgccgcccc tccaaccgga tcctgggtct agagctcccc 300
agagcgaggc gctcgccagg actcctgccc cgccaaccct gaccgccggg gggtgccccc 360
gggacgtagc gccgcggaga ggaagcggca aaggggacca tgcggcgcct gactcgtcgg 420
ctggttctgc cagtcttcgg ggtgctctgg atcacggtgc tgctgttctt ctgggtaacc 480
aagaggaagt tggaggtgcc gacgggacct gaagtgcaga cccctaagcc ttcggacgct 540
gactgggacg acctgtggga ccagtttgat gagcggcggt atctgaatgc caaaaagtgg 600
cgcgttggtg acgaccccta taagctgtat gctttcaacc agcgggagag tgagcggatc 660
tccagcaatc gggccatccc ggacactcgc catctgagtg tattaaaccg cacccctacg 720
catctgatcc gggaaatcat attagtggat gacttcagca atgaccctga tgactgtaaa 780
cagctcatca agttgcccaa ggtgaaatgc ttgcgcaata atgaacggca aggtctggtc 840
cggtcccgga ttcggggcgc tgacatcgcc cagggcacca ctctgacttt cctcgacagc 900
cactgtgagg tgaacaggga ctggctccag cctctgttgc acagggtcaa agaggactac 960
acgcgggtgg tgtgccctgt gatcgatatc attaacctgg acaccttcac ctacatcgag 1020
tctgcctcgg agctcagagg ggggtttgac tggagcctcc acttccagtg ggagcagctc 1080
tccccagagc agaaggctcg gcgcctggac cccacggagc ccatcaggac tcctatcata 1140
gctggagggc tcttcgtgat cgacaaagct tggtttgatt acctggggaa atatgatatg 1200
gacatggaca tctggggtgg ggagaacttt gaaatctcct tccgagtgtg gatgtgcggg 1260
ggcagcctag agatcgtccc ctgcagccga gtggggcacg tcttccggaa gaagcacccc 1320
tacgttttcc ctgatggaaa tgccaacacg tatataaaga acaccaagcg gacagctgaa 1380
gtgtggatgg atgaatacaa gcgatactat tacgctgccc ggccattcgc cctggagagg 1440
cccttcggga atgttgagag cagattggac ctgaggaaga atctgcgctg ccagagcttc 1500
aagtggtacc tggagaatat ctaccctgaa ctcagcatcc ccaaggagtc ctccatccag 1560
aagggcaata tccgacagag acagaagtgc ctggaatctc aaaggcagaa caaccaagaa 1620
accccaaacc taaagttgag cccctgtgcc aaggtcaaag gcgaagatgc aaagtcccag 1680
gtatgggcct tcacatacac ccagcagatc ctccaggagg agctgtgcct gtcagtcatc 1740
accttgttcc ctggcgcccc agtggttctt gtcctttgca agaatggaga tgaccgacag 1800
caatggacca aaactggttc ccacatcgag cacatagcat cccacctctg cctcgataca 1860
gatatgttcg gtgatggcac cgagaacggc aaggaaatcg tcgtcaaccc atgtgagtcc 1920
tcactcatga gccagcactg ggacatggtg agctcttgag gacccctgcc agaagcagca 1980
agggccatgg ggtggtgctt ccctggacca gaacagactg gaaactgggc agcaagcagc 2040
ctgcaaccac ctcagacatc ctggactggg aggtggaggc agagcccccc aggacaggag 2100
caactgtctc agggaggaca gaggaaaaca tcacaagcca atggggctca aagacaaatc 2160
ccacatgttc tcaaggccgt taagttccag tcctggccag tcattccctg attggtatct 2220
ggagacagaa acctaatggg aagtgtttat tgttcctttt cctacaaagg aagcagtctc 2280
tggaggccag aaagaaaagc cttctttttc actaggccag gactacattg agagatgaag 2340
aatggaggtt gtttccaaaa gaaataaaga gaaacttaga agttg 2385

<210> 54
<211> 2803
<212> DNA
<213> Homo sapiens

<400> 54
ggcatcccga gtgcaccgct cccgccccgc cccgccttgc cccaacccac gatggtctgg 60
gagctgcgcc cagggcttgg cgctggcggc cccgcaacag caccgagcgt ttcggtcggc 120
gggcggcggt agcgccccct ctcagagccc cgctcactcc cacctgggct cgctccgagt 180
cggcctgtct gtcgggcccg ccctccccgc tcactccctc cgccctcgtg ctcctcccgg 240
ggtgcttggc acagcctcgg attcctccct ctcgctgctc gagtcagttt ccctatcggc 300
ggcagcgggc aaggcggcgg cggcggcggc ggcagccgcg gtggcggcgt ggggaacatc 360
tcggcagcca ccgcgcttct cccgctggag cgggcgtcca gcttggctgc cctcggtcct 420
tccctgccac gtttcgggtc gccctgcacc ccccacccag gctcgcttct cttcgaagcg 480
ggaagggcgc cttgcaggat cctgccgccc ctccaaccgg atcctgggtc tagagctccc 540
cagagcgagg cgctcgccag gactcctgcc ccgccaaccc tgaccgccgg ggggtgcccc 600
cgggacgtag cgccgcggag aggaagcggc aaaggggacc atgcggcgcc tgactcgtcg 660

gctggttctg ccagtcttcg gggtgctctg gatcacgtgc tgctgttctt ctgggtaacc 720
aagaggaagt ggaggtgccg acgggacctg aagtgcagac ccctaagggt tcttcccaaa 780
gttagtgcag cctacaccca ggattgaaca aagacagctt ggaagttaga agcaagatgg 840
agtctgttag ccttcggacg ctgactggga cgacctgtgg gaccagtttg atgagcggcg 900
gtatctgaat gccaaaaagt ggcgcgttgg tgacgacccc tataagctgt atgctttcaa 960
ccagcgggag agtgagcgga tctccagcaa tcgggccatc ccggacactc gccatctgag 1020
atgcacactg ctggtgtatt gcacggacct tccacccact agcatcatca tcaccttcca 1080
caacgaggcc cgctccacgc tgctcaggac catccgcagt gtattaaacc gcacccctac 1140
gcatctgatc cgggaaatca tattagtgga tgacttcagc aatgaccctg atgactgtaa 1200
acagctcatc aagttgccca aggtgaaatg cttgcgcata atgaacggca aggtctggtc 1260
cggtcccgga ttcggggcgc tgacatcgcc cagggcacac tctgactttc tcgacagcca 1320
ctgtgaggtg aacagggact ggctccagcc tctgttgcac agggtcaaag aggactacac 1380
gcgggtggtg tgccctgtga tcgatatcat taacctggac accttcacct acatcgagtc 1440
tgcctcggag ctcagagggg ggtttgactg gagcctccac ttccagtggg agcagctctc 1500
cccagagcag aaggctcggc gcctggaccc cacggagccc atcaggactc ctatcatagc 1560
tggagggctc ttcgtgatcg acaaagcttg gtttgattac ctggggaaat atgatatgga 1620
catggacatc tggggtgggg agaactttga aatctccttc cgagtgtgga tgtgcggggg 1680
cagcctagag atcgtcccct gcagccgagt ggggcacgtc ttccggaaga agcaccccta 1740
cgttttccct gatggaaatg ccaacacgta tataaagaac accaagcgga cagctgaagt 1800
gtggatggat gaatacaagc aatactatta cgctgcccgg ccattcgccc tggagaggcc 1860
cttcgggaat gttgagagca gattggacct gaggaagaat ctgcgctgcc agagcttcaa 1920
gtggtacctg gagaatatct accctgaact cagcatcccc aaggagtcct ccatccagaa 1980
gggcaatatc cgacagagac agaagtgcct ggaatctcaa aggcagaaca accaagaaac 2040
cccaaaccta aagttgagcc cctgtgccaa ggtcaaaggc gaagatgcaa agtcccaggt 2100
atgggccttc acatacaccc agcagatcct ccaggaggag ctgtgcctgt cagtcatcac 2160
cttgttccct ggcgccccag tggttcttgt cctttgcaag aatggagatg accgacagca 2220
atggaccaaa actggttccc acatcgagca catagcatcc cacctctgcc tcgatacaga 2280
tatgttcggt gatggcaccg agaacggcaa ggaaatcgtc gtcaacccat gtgagtcctc 2340
actcatgagc cagcactggg acatggtgag ctcttgagga cccctgccag aagcagcaag 2400
ggccatgggg tggtgcttcc ctggaccaga acagactgga aactgggcag caagcagcct 2460
gcaaccacct cagacatcct ggactgggag gtggaggcag agccccccag gacaggagca 2520
actgtctcag ggaggacaga ggaaaacatc acaagccaat ggggctcaaa gacaaatccc 2580
acatgttctc aaggccgtta agttccagtc ctggccagtc attccctgat tggtatctgg 2640
agacagaaac ctaatgggaa gtgtttattg ttccttttcc tacaaaggaa gcagtctctg 2700
gaggccagaa agaaaagcct tctttttcac taggccagga ctacattgag agatgaagaa 2760
tggaggttgt ttccaaaaga aataaagaga aacttagaag ttg 2803

<210> 55
<211> 2775
<212> DNA
<213> Homo sapiens
<400> 55
gtactcgcca cggcacccag gctgcgcgca cgcggtcccg gtgtgcagct ggagagcgag 60
cggccaccgg gagcccccgg cacagcccgc gcccgccccg caggagcccg cgaagatgcc 120
ccggcgcagc ctgcacgcgg cggccgtgct cctgctggtg atcttaaagg aacagccttc 180
cagcccggcc ccagtgaacg gttccaagtg gacttatttt ggtcctgatg gggagaatag 240
ctggtccaag aagtacccgt cgtgtggggg cctgctgcag tcccccatag acctgcacag 300
tgacatcctc cagtatgacg ccagcctcac gcccctcgag ttccaaggct acaatctgtc 360
tgccaacaag cagtttctcc tgaccaacaa tggccattca gtgaagctga acctgccctc 420
ggacatgcac atccagggcc tccagtctcg ctacagtgcc acgcagctgc acctgcactg 480
ggggaacccg aatgacccgc acggctctga gcacaccgtc agcggacagc acttcgccgc 540
cgagctgcac attgtccatt ataactcaga cctttatcct gacgccagca ctgccagcaa 600
caagtcagaa ggcctcgctg tcctggctgt tctcattgag atgggctcct tcaatccgtc 660
ctatgacaag atcttcagtc accttcaaca tgtaaagtac aaaggccagg aagcattcgt 720
cccgggattc aacattgaag agctgcttcc ggagaggacc gctgaatatt accgctaccg 780
ggggtccctg accacacccc cttgcaaccc cactgtgctc tggacagttt tccgaaaccc 840
cgtgcaaatt tcccaggagc agctgctggc tttggagaca gccctgtact gcacacacat 900
ggacgaccct tcccccagag aaatgatcaa caacttccgg caggtccaga agttcgatga 960
gaggctggta tacacctcct tctcccaagt gcaagtctgt actgcggcag gactgagtct 1020
gggcatcatc ctctcactgg ccctggctgg cattcttggc atctgtattg tggtggtggt 1080
gtccatttgg cttttcagaa ggaagagtat caaaaaaggt gataacaagg gagtcattta 1140
caagccagcc accaagatgg agactgaggc ccacgcttga ggtccccgga gctcccgggc 1200
acatccagga aggaccttgc tttggaccct acacacttcg gctctctgga cacttgcgac 1260

acctcaaggt gttctctgta gctcaatctg caaacatgcc aggcctcagg gatcctctgc 1320
tgggtgcctc cttgccttgg gaccatggcc accccagagc catccgatcg atggatggga 1380
tgcactctca gaccaagcag caggaattca aagctgcttg ctgtaactgt gtgagattgt 1440
gaagtggtct gaattctgga atcacaaacc aagccatgct ggtgggccat taatggttgg 1500
aaaacacttt catccggggc tttgccagag cgtgctttca agtgtcctgg aaagtctgct 1560
gcttctccaa gctttcagac aagaatgtgc actctctgct taggttttgc ttgggaaact 1620
caacttcttt cctctggaga cggggcatct ccctctgatt tccttctgct atgacaaaac 1680
ctttaatctg caccttacaa ctcggggaca aatggggaca ggaaggatca agttgtagag 1740
agaaaaaaga aaacaagaga tatacattgt gatatattag ggacactttc acagtcctgt 1800
cctctggatc acagacactg cacagacctt agggaatggc aggttcaagt tccacttctt 1860
ggtggggatg agaaggagag gagagctaga gggacaaaga gaatgagaag acatggatga 1920
tctgggagag tctcactttg gaatcagaat tggaatcaca ttctgtttat caagccataa 1980
tgtaaggaca gaataataca atattaagtc caaatccaac ctcctgtcag tggagcagtt 2040
atgttttata ctctacagat tttacaaata atgaggctgt tccttgaaaa tgtgttgttg 2100
ctgtgtcctg gaggagacat gagttccgag atgacccaat ctgcctttga atctggagga 2160
aataggcaga aacaaaatga ctgtagaact tattctctgt aggccaaatt tcatttcagc 2220
cacttctgca ggatccctac tgccaacctg gaatggagac ttttatctac ttctctctct 2280
ctgaagatgt caaatcgtgg tttagatcaa atatatttca agctataaaa gcaggaggtt 2340
atctgtgcag ggggctggca tcatgtattt aggggcaagt aataatggaa tgctactaag 2400
atactccata ttcttccccg aatcacacag acagtttctg acaggcgcaa ctcctccatt 2460
ttcctcccgc aggtgagaac cctgtggaga tgagtcagtg ccatgactga gaaggaaccg 2520
acccctagtt gagagcacct tgcagttccc cgagaacttt ctgattcaca gtctcatttt 2580
gacagcatga aatgtcctct tgaagcatag ctttttaaat atctttttcc ttctactcct 2640
ccctctgact ctaagaattc tctcttctgg aatcgcttga acccaggagg cggaggttgc 2700
agtaagccaa ggtcatgcca ctgcactcta gcctgggtga cagagcgaga ctccatctca 2760
aaaaaaaaaa aaaaa 2775

<210> 56
<211> 1718
<212> DNA
<213> Homo sapiens

<400> 56
ctcgccacgg cacccaggct gcgcgcacgc ggtcccggtg tgcagctgga gagcgagcgg 60
ccaccgggag cccccggcac agcccgcgcc cgccccgcag gagcccgcga agatgccccg 120
gcgcagcctg cacgcggcgg ccgtgctcct gctggtgatc ttaaaggaac agccttccag 180
cccggcccca gtgaacggtt ccaagtggac ttattttggt cctgatgggg agaatagctg 240
gtccaagaag tacccgtcgt gtgggggcct gctgcagtcc cccatagacc tgcacagtga 300
catcctccag tatgacgcca gcctcacgcc cctcgagttc caaggctaca atctgtctgc 360
caacaagcag tttctcctga ccaacaatgg ccattcagtg aagctgaacc tgccctcgga 420
catgcacatc cagggcctcc agtctcgcta cagtgccacg cagctgcacc tgcactgggg 480
gaacccgaat gacccgcacg gctctgagca caccgtcagc ggacagcact tcgccgccga 540
gctgcacatt gtccattata actcagacct ttatcctgac gccagcactg ccagcaacaa 600
gtcagaaggc ctcgctgtcc tggctgttct cattgagatg ggctccttca atccgtccta 660
tgacaagatc ttcagtcacc ttcaacatgt aaagtacaaa ggccaggaag cattcgtccc 720
gggattcaac attgaagagc tgcttccgga gaggaccgct gaatattacc gctaccgggg 780
gtccctgacc acaccccctt gcaaccccac tgtgctctgg acagttttcc gaaaccccgt 840
gcaaatttcc caggagcagc tgctggcttt ggagacagcc ctgtactgca cacacatgga 900
cgacccttcc cccagagaaa tgatcaacaa cttccggcag gtccagaagt tcgatgagag 960
gctggtatac acctccttct cccaaggcat catcctctca ctggccctgg ctggcattct 1020
tggcatctgt attgtggtgg tggtgtccat ttggcttttc agaaggaaga gtatcaaaaa 1080
aggtgataac aagggagtca tttacaagcc agccaccaag atggagactg aggcccacgc 1140
ttgaggtccc cggagctccc gggcacatcc aggaaggacc ttgctttgga ccctacacac 1200
ttcggctctc tggacacttg cgacacctca aggtgttctc tgtagctcaa tctgcaaaca 1260
tgccaggcct cagggatcct ctgctgggtg cctccttgcc ttgggaccat ggccacccca 1320
gagccatccg atcgatggat gggatgcact ctcagaccaa gcagcaggaa ttcaaagctg 1380
cttgctgtaa ctgtgtgaga ttgtgaagtg gtctgaattc tggaatcaca aaccaagcca 1440
tgctggtggg ccattaatgg ttggaaaaca ctttcatccg gggctttgcc agagcgtgct 1500
ttcaagtgtc ctggaaagtc tgctgcttct ccaagctttc agacaagaat gtgcactctc 1560
tgcttaggtt ttgcttggga aactcaactt ctttcctctg gagacggggc atctccctct 1620
gatttccttc tgctatgaca aaacctttaa tctgcacctt acaactcggg gacaaatggg 1680
gacaggaagg atcaagttgt aaaaaaaaaa aaaaaaaa 1718

<210> 57
<211> 1795
<212> DNA
<213> Homo sapiens

<400> 57
acgcgtccgc ccacgcgtcc gcccacgcgt ccggtcgggg ccagagcgca ggtgtacctg 60
gcggccgtgc tggagcacct gaccgccgag atcctggagc tggctggcaa cccggcccgc 120
gacaagaaga cccgcatcat cctgcgccac ctgtagctgg ccattcgcaa cggcgaggag 180
cttaacaagc tgctgggcga agtcaccatc gcgcagggcg gtgtcctgcc caacattcag 240
ggcgtgcttc tgccccagaa gaccaagagc caccacaagg ccaagggtga aaaccattca 300
ctaggagagg agaaacacaa tggccaccaa gacagagttg agtcccacag caagggagag 360
caagaacgca caagatatgc aagtggatga gacactgatc cccaggaaag gtccaagttt 420
atgttctgct cgctatggaa tagccctcgt cttacatttc tgcaatttca caacgatagc 480
acaaaatgtc atcatgaaca tcaccatggt agccatggtc aacagcacaa gccctcaatc 540
ccagctcaat gattcctctg aggtgctgcc tgttgactca tttggtggcc taagtaaagc 600
cccaaagagt cttcctgcaa agtcctcaat acttgggggt cagtttgcaa tttgggaaaa 660
gtggggccct ccacaagaac gaagcagact ctgcagcatt gctttatcag gaatgttact 720
gggatgcttt actgccatcc tcataggtgg cttcattagt gaaacccttg ggtggccctt 780
tgtcttctat atctttggag gtgttggctg tgtctgctgc cttctctggt ttgttgtgat 840
ttatgatgac cccttttcct atccatggat aagcacctca gaaaaagaat acatcatatc 900
ctccttgaaa caacaggtcg ggtcttctaa gcagcctctt cccatcaaag ctatgctcag 960
atctctaccc atttggtcca tatgtttagg ctgtttcagc catcaatggt tagttagcac 1020
aatggttgta tacataccaa cttacatcag ctctgtgtac catgttaaca tcagagacaa 1080
tggacttcta tctgcccttc cttttattgt tgcctgggtc ataggcatgg tgggaggcta 1140
tctggcagat ttccttctaa ccaaaaagtt tagactcatc actgtgagga aaattgccac 1200
aattttagga agtctcccct cttcagcact cattgtgtct ctgccttacc tcaattccgg 1260
ctatatcaca gcaactgcct tgctgacgct ctcttgcgga ttaagcacat tgtgtcagtc 1320
agggatttat atcaatgtct tagatattgc tccaaggtat tccagttttc tcatgggagc 1380
atcaagagga ttttcgagca tagcacctgt cattgtaccc actgtcagcg gatttcttct 1440
tagtcaggac cctgagtttg ggtggaggaa tgtcttcttc ttgctgtttg ccgttaacct 1500
gttaggacta ctcttctacc tcatatttgg agaagcagat gtccaagaat gggctaaaga 1560
gagaaaactc actcgtttat gaagttatcc caccttggat ggaaaagtca ttaggcaccg 1620
tattgcataa aatagaaggc ttccgtgatg aaaataccag tgaaaagatt tttttttcct 1680
gtggctcttt tcaattatga gatcagttca ttattttatt cagacttttt tttgagagaa 1740
atgtaagatg aataaaaatt caaataaaat gataactaag aaaaaaaaaa aaaaa 1795

<210> 58
<211> 2257
<212> DNA
<213> Homo sapiens

<220>
<221> modified_base
<222> (1)...(2257)
<223> n = g, a, c or t

<400> 58
ggccctgccc tgaaggctgg tcacttgcag aggtaaactc ccctctttga cttctggcca 60
gggtttgtgc tgagctggct gcagccgctc tcagcctcgc tccgggcacg tcgggcagcc 120
tcgggccctc ctgcctgcag gatcatgccc accaccgtgg acgatgtcct ggagcatgga 180
ggggagtttc actttttcca gaagcaaatg tttttcctct tggctctgct ctcggctacc 240
ttcgcgccca tctacgtggg catcgtcttc ctgggcttca cccctgacca ccgctgccgg 300
agccccggag tggccgagct gagtctgcgc tgcggctgga gtcctgcaga ggaactgaac 360
tacacggtgc cgggcccagg acctgcgggc gaagcctccc caagacagtg taggcgctac 420
gaggtggact ggaaccagag cacctttgac tgcgtggacc ccctggccag cctggacacc 480
aacaggagcc gcctgccact gggcccctgc cgggacggct gggtgtacga gacgcctggc 540
tcgtccatcg tcaccgagtt taacctggta tgtgccaact cctggatgtt ggacctattc 600
cagtcatcag tgaatgtagg attctttatt ggctctatga gtatcggcta catagcagac 660
aggtttggcc gtaagctctg cctcctaact acagtcctca taaatgctgc agctggagtt 720
ctcatggcca tttccccaac ctatacgtgg atgttaattt ttcgcttaat ccaaggactg 780
gtcagcaaag caggctggtt aataggctac atcctgatta cagaatttgt tgggcggaga 840
tatcggagaa cagtggggat tttttaccaa gttgcctata cagttgggct cctggtgcta 900

gctggggtgg cttacgcact tcctcactgg aggtggttgc agttcacagt tgctctgccc 960
aacttcttct tcttgctcta ttactggtgc atacctgagt ctcccaggtg gctgatctcc 1020
cagaataaga atgctgaagc catgagaatc attaagcaca tcgcaaagaa aaatggaaaa 1080
tctctacccg cctcccttca gcgcctgaga cttgaagagg aaactggcaa gaaattgaac 1140
ccttcatttc ttgacttggt cagaactcct cagataagga aacatactat gatattgatg 1200
tacaactggt tcacgagctc tgtgctctac cagggcctca tcatgcacat gggccttgca 1260
ggtgacaata tctacctgga tttcttctac tctgccctgg ttgaattccc agctgccttc 1320
atgatcatcc tcaccatcga ccgcatcgga cgccgttacc cttgggctgc atcaaatatg 1380
gttgcagggg cagcctgtct ggcctcagtt tttatacctg gtgatctaca atggctaaaa 1440
attattatct catgcttggg aagaatgggg atcacaatgg cctatgagat agtctgcctg 1500
gtcaatgctg agctgtaccc cacattcatt aggaatcttg gcgtccacat ctgttcctca 1560
atgtgtgaca ttggtggcat catcacgcca ttcctggtct accggctcac taacatctgg 1620
cttgagctcc cgctgatggt tttcggcgta cttggcttgg ttgctggagg tctggtgctg 1680
ttgcttccag aaactaaagg gaaagctttg cctgagacca tcgaggaagc cgaaaatatg 1740
caaagaccaa gaaaaaataa agaaaagatg atttacctcc aagttcagaa actagacatt 1800
ccattgaact aagaagagag accgttgctg ctgtcatgac ctagctttga tggcagcaag 1860
accaaaagta gaaatccctg cactcatcac aaagcccata caactcaacc aaacttaccc 1920
ctgagcccta tcaacctagg tctacagcca gtggagtcta ttgtacactg tggaaaaata 1980
cccatgggac cagatcctgc caaattcttc cagctcactt tattctcagc attcctagga 2040
cattggacat tggttttctg gagggttttt tttccgatct ttgtattttt ttaaatttga 2100
ttcttttctt tgcaatgcta gcaaccagaa tacatagggg aactgtgggc taggcaaana 2160
aaatagaaaa agtgtgaaaa acagtaaagt tgggagagga gcatctattt tcttaaagaa 2220
ataaaacacc naaaacaaaa aaaaaaaaaa aaaaaaa 2257

<210> 59
<211> 5687
<212> DNA
<213> Homo sapiens

<400> 59
gtcaggggcg tgttggcccc gcacagattg agccgagttg tcgccccgct gggagaagtg 60
accctcctgc gcctgatcag aacactgagg ctcagagaag ttaactactc caaagtcata 120
tagccagtat tttctggagc tgtaattcaa atcagatgtg ttcaatacct tctactaccc 180
cattgctgtc tttatgaaaa gaaatttcat ttcaatatag gtgactatgc agcctgcaat 240
tcaagtatgg tttggagaag atctgcctct aagtcctcgg agtcctctga ctcccagaca 300
cggaccagga ttggctaatg tttgtcagta cgatgagtgg atagctgtga ggcatgaagc 360
cactttgttg cccatgcaag aagatctgtc aatctggtta tctggtttat taggtattaa 420
agttaaggca gaaaaattat tggaagaact tgataatgga gtactattat gtcaactgat 480
tgatgttctt caaaacatgg tgaaaacatg caactctgaa gaatcaggga attttccaat 540
gagaaaagtg ccctgtaaga aagatgctgc atcaggttca ttctttgctc gggacaatac 600
cgcaaacttc cttcactggt gtagggacat tggggttgat gaaacttacc tctttgaatc 660
tgaaggttta gttttgcaca aagatccaag acaggtgtat ctttgtcttc ttgaaattgg 720
tcgaattgtg tcaagatacg gggttgagcc accagtgtta gtaaaacttg agaaagaaat 780
tgagttagaa gagactttgc ttaatacttc tgggcctgaa gattccatca gcattccaaa 840
atcatgctgt cggcatgaag agctacatga agctgttaaa catattgctg aggaccctcc 900
ttgtagttgt tctcatcgat tttctattga gtatttatct gaaggacggt accgactagg 960
ggataaaata ctctttataa gaatgcttca tggaaaacat gtcatggttc gcgttggtgg 1020
aggctgggat actcttcaag gatttttgct taaatatgac ccctgtcgaa tattacagtt 1080
tgccacatta gaacaaaaaa ttttagcatt tcaaaaagga gtttctaatg aaagtgtacc 1140
tgattcgcct gccagaacac ctcagcctcc tgaaatgaat cctttgtcag cagttaacat 1200
gtttcagaaa caaaattcaa aacccagcgt gccagttagt attccaaaaa gcaaagaaaa 1260
acagggacgt ccaccaggtg cattggtgcc agcatcttca ctgaaaggag gtaatctggg 1320
ctctatgtca gtccgttcta aattgccaaa ttctccagca gcatcttctc atcccaagct 1380
caagtcttca aaaggcataa cgaagaaacc gcaggctcct tcaaacaatg catcatcttc 1440
acttgcttca ttaaatccag taggtaaaaa cacttcttca ccagctttac caagaactgc 1500
accttgtata tctgagtcac cgagaaaatg tatttcatcc cccaataccc ccaaggccaa 1560
ggttattcca gcccagaatt cagcagatct gcccgagtcc acacttttgc caaataagtg 1620
ttcaggaaaa actcaaccta agtatttgaa acataatcat atttcttcca gagataatgc 1680
agtatctcac ttagctgcac attcaaattc atcctcaaaa tgtcccaagc tgcctaaagc 1740
aaatatacct gtaagaccta aaccttcttt ccagtcctct gcaaaaatga caaaaaccag 1800
ttccaaaacc atagccacgg gtctaggaac acagtctcaa ccatccgatg gagccccaca 1860
agcaaagcca gtcccagcac agaaacttaa atcggccttg aatttaaatc agccagtttc 1920
tgtgtcctca gtttctcctg taaaagccac acagaaatca aaagataaga atatagtttc 1980

agctaccaaa aagcagcctc agaataaaag tgcatttcag aagacaggac ccagctcctt 2040
gaagtctcct ggccgtaccc cactgtccat cgtgagccta ccccagtctt ctaccaaaac 2100
acaaactgca ccgaagtcag cacagactgt cgctaagagc cagcattcaa ctaaagggcc 2160
tcccagaagt ggcaaaaccc cagcttcaat caggaaacca ccctcatctg ttaaggatgc 2220
agatagtgga gataaaaaac ctactgcaaa gaaaaaggaa gatgatgacc attattttgt 2280
catgactgga agtaagaaac ctagaaaata aatacatact cattataaaa aaagagaaaa 2340
ggaagaatga atgtgttagc ttcacatctt aaaagtttct cctatttgtg tctgtctaaa 2400
taggtgcaga cactaaggat agtgaggatg gaggctggga tgaggaaagg gttcatcaga 2460
attcacatat ctgaattcac tggaaagagc ccttctgaag caaacagttg taaaatcact 2520
gcaaggtttt tattaataat agacatgtat atgattttca gtctatagca tctttgttaa 2580
catctgcctt ttgcaggaaa tgtaaaagtt atttaacact acaagaattt taacaatagt 2640
tgctctattt ttgaatatgt attaaatatg gagttcatat acctgctaat atcaacggtg 2700
gtgctcttac tattagttaa ttgcattttg gttaaaaaaa aaaaagcaac agtttggcac 2760
ttgtcctaca aaaggcacct aatttaattt tctgatcagg attgcctgat ccaacagtgc 2820
taagtcatgg ctgctgctga ctagcttggc attattctgt gttaggtaga attcttatta 2880
tttatttttt taagctttcc aaattggaag gaactgattg tttcatgtgg cttatattta 2940
cattggtaat attttgtcac caatattttt ggttaaaaaa aatccaacaa attaacttac 3000
tgaaatataa acaaattttg taaacaattt tttatattat ctataaaaac gtagacacct 3060
tatgtttcac atgttgtgca atgtgacagg ggaagctgat ttagtagctt ttagcatatt 3120
aaaaataatt ttttataatg taatttcctg tgagtgcaga cctgacattt tacattaaaa 3180
taatgtgaaa catcagaatt atgttttaac aactttaaaa ttaagatgat gttaaaataa 3240
ttttagagtt atgctatgga aaaattctat catgaaatta tttttctcta gatagcacaa 3300
taccaatttt aattaatttc ttccaattag gttacttttc tttaataaag ttatgctgcc 3360
ttcagttttc caatggcaag tagacaggat atgttcaagg ttttctgcac tgtaggcaca 3420
gtctctcaag catatcctga tcatgtaatg actgcataaa ctccatcaac ctaaggtgat 3480
acttgtaaat aatttatttt taagggatgg tgactttaaa aattattaat gaactttgag 3540
aagttttaag agtgctttta aaacttcaca gtattgccaa ttatcttagg ttattcagta 3600
ttcaggtttg tgtttctctg ttttaaacta aaatgtgttt tctgaagaaa aaaataatag 3660
tttacacaaa tgtacaatca tagaataagc attttaagct ggcgactagt gttctataga 3720
ttacaaagca agaaaacttt ctatgaagat aaatgacctt ttgcctgaag agtacagata 3780
aaatcaaaga tgtgtgcaag ctagtttttg gaagaagtga tgcttctctt ctttaaagag 3840
acagtcacca aatacttggt ttaactcgac tattgacttg ggcattgaga gagatgatat 3900
atacatcttt ggaaagtgaa gtcaatgttc aagaggtgat agaagcttta ctttttagtg 3960
atcagaaata tttagtgcat cttttcagac aggaagaatt ttatcatcaa gtattccctt 4020
ataaaaccaa gtaacacttc tttatcagta acttttagaa cttaaaagaa agcaaaaagt 4080
aaatggaatt gtaggcaatt tatgaatcct agtagatttt acaatatgta atttatgttg 4140
tttacagtat ataaacacta agttttgtgt taaatgtgat caggaataaa agtatcccac 4200
aggcatctga cacaaattcc agaattagcc aaagaattgt ttatttgagg ccaggcaatc 4260
ccagcatttt gggaagccaa gttgggcaga tcacgaagtc aggagttcga aatcagcctg 4320
accaacatgg tgaaacctct tctctactaa aaatacaaaa ttagccgggc atggtggcac 4380
atgcctataa tctcagctac tcaggaggct gaggcaggag gatcgcttga acccgggagg 4440
cagaggttgc agtgagccaa gattgcgcca ctgcactcca gcctgggcaa cagagtgaga 4500
ctctgtctca aaaaaaaaaa aaaagaaaaa gaaaagaaaa aggtttattt gaataattgg 4560
aagtcagttt atacattact atttttcagc agtagggaat ttctccaatt acattcatgt 4620
tgaatgaatt tttatttata tatagcttac ccttccaaaa taaaagtgtt tttttaatgt 4680
tgttttgttt tgttttgttt ttttcctttt ttgagacgga gtttcactct tgttgcccag 4740
actggagtgc agtggcacaa tctcagctca ttgcaacctc tgcctcctgg gttcaagtga 4800
ttctgctgcc tcagcctcct gagtggttgg gattacaggc atccaccacc aggcccagct 4860
aatttttttg tattttcagt agagatgggg gtttcaccat gtcggccagg ctgatctcaa 4920
actcctgacc tcaggtgatc cacctgcctc agcctcccaa agcgctggga ttacaggcat 4980
gagccatcac acccagcgaa aagttttgtt tgaataaaca atatccgaaa gacaattagt 5040
ttcttcagat gtattttgaa attctcctaa agagctagtg tttctattca ttttcacaat 5100
ttaaaaacag ctcttaacat tgctgaagtt gggagaactt tccatctctt cttaataaca 5160
gtgcaagatt ttgtaaattc ttttttgtgt ttaatgttta ataaaacgag tattaagctt 5220
aaattactga agtacctggg agaagtaatg atgtgtactt tcaaaaaaat ggaaaatgct 5280
tttattttat tttctataat ttgttaacat gatatgtaaa attaaacttc ggagcacaat 5340
gaaatgccga ttatttttac cttgtttggg cttaaagtag gtatttaagg tttatgtgtt 5400
caaaatgcct tggtaaattg gatgacctct aactttactg tccatatgga gtttgtcatt 5460
ctttatggat aagagaactt aaggaaaagt tactgttttt cttcagtctt tttatatcta 5520
tctgatttaa aatctgttac tttattaaaa ggcttcaaca acaggttgtt aggatgtagt 5580
cttacatcca ggttcacata ataaccccat ttgaatccaa atttgtgtat attttcttat 5640
gccagcagta tttgtatcca attttaactt aggtttgttt tcttgag 5687

<210> 60
<211> 667
<212> DNA
<213> Homo sapiens

<400> 60
tttttttttt tttttttaaa tcacaacacc tctgaatatg tttaatgaca gagcaattag 60
aaacaaaagt ttaattttat acatcataga attactattt agaaacaaat aggacatgtt 120
agaagtaaga aaaaataaaa cactttgttg aagttatata cctatttgga tttttttaaa 180
agcattaccc aataggctat agaacgatgc caacacataa tgccaggttg caaggcatgg 240
agaatgggag aatgtgacat gactgtctaa tgggtataaa gctccctttt gaagtgaaaa 300
aattactttg gatctagata gtagtggtac tggcaaatgt gctaaacgcc aatgaataat 360
gcacattaaa tggtttaatt gtataatttt gtggtgtaaa attcctatat caattattct 420
ttaaataaac aagtggctga ccagatttga ccaagagacc atagtttgtc agcccttggt 480
ttatgctaca aatttaattc ccatatgctt gaaatagtaa atgctgtcat tttaatcatt 540
tacattagaa acatgggatg ttttgaaact ataatatgtt atgcaattat atgaacacta 600
aaattattac tgtggaattt ttttatttgt agtaacggga gtatgcaatt ttattttgct 660
gtacaca 667

<210> 61
<211> 537
<212> DNA
<213> Homo sapiens

<400> 61
gacctaagaa cttatcaaag aactcagcag tatgagaata gatcaataca tagagggttt 60
cctttaagtc ttgtagaaac tcattactct ccagtctctt tttccactta tagtagagag 120
aatcagactt gcggatatcc agttaactat caataccttt aaaaaatgtg aacaagcagt 180
ccatctgaat aattatacca aatgtcaagg cttttaaaag tgcttaactt taaaggtcct 240
ttgcagttgt tcagacctag attctcagtt cagttagcac agagagagac aaggaaaaaa 300
tgaggaacac ttttctcacg cataaatatt cactggtttt tgaaatacta ttgattcctt 360
ggataagaaa ataggtcaac tttatagctt tttgtctcga aattgatttg ccgccacaga 420
cagtggcttg gtgcctataa tcccagccta catgggaggc tgaggtggga gatcgcttga 480
gcccaaaaag tcagaggtgc agtgagctgt gattgtgcca ctgtatccca acctcat 537

<210> 62
<211> 702
<212> DNA
<213> Homo sapiens

<400> 62
tttttttttt ttttaatttt aataagttaa atttttaata attgatttat ttatactcca 60
aaggatacaa catgcaaaat gtctacccac tccagctacc atcagttcag catttataac 120
tactatcaat aatgttttca tgtaagacag ccatcaccta aaggaatact tttaaaacag 180
gttgtacaat aaaacaggtt ggaccaatat aaaagaaata taaaataagg tggtttattt 240
aaatgtaaat acacagatac ttccattaaa tctaaacaaa ctttaaaaag aggagcaaat 300
aatgagtaat gccatagaac attagaagaa cacagttaac gaatggaaac ttgtaacacg 360
atacacacac acgcatgcat atatatatgt atcatatata tcaactaaaa gacattttct 420
tagagcatat tgaattgtta ataattattt gaagtgacat gtggtgggac ataaatcaag 480
actcagtaaa tttgaaaaga ttcaatatca taaagaatat atgatccagc cttagtagaa 540
gtaagggaaa tttaaaaaat gtaactaaaa tatctttaat tttggaaatt aagaaaatac 600
acttcaaaaa aatttaaagt cctgcctcag cctccccagt agctgggatt acaagcatgc 660
accaccatgt ccagccaatt ttgtaattat agtagagaca gg 702

<210> 63
<211> 1398
<212> DNA
<213> Homo sapiens

<400> 63
acaacaggaa cacatatagc tggaatgcag atgggatctg tgctacttac gctagccatg 60
ggatgattag aacaagataa gagacatgag gaagacttct tcaaggaggt gctcaagcca 120
tgttttggaa aagagaactc aaggtctggc agggagaaag gagcaaggtc ctcttctccc 180

tgaaggctgt cacagttcct gtgcagagag agagggcact gcagcaccca ggcgctgact 240
gccttttgtc agtctcattt gcaggccccc agctggagaa cccaggagga agaggaaaaa 300
agttttttcc tcctctacac tccccatgca tgactaaaga gtaggaggag tcagcaaggc 360
agacatatat agtacccagg aggtggacaa acaactgcct tggtggaaac tctgtccttt 420
ctggacttat cctggtgcca ctgagactgt gcagatttag atctacaatc cacctggaat 480
tgatttttgt atatggagtg aggtcccaca cttacaggga aagcatcacc cgaaagtgag 540
aatgcctaga ggcaggaatc atggaggctt ccttaaccgt ctgtctgcaa cagcaggtgc 600
tagagatgac actgcagagt agagaacaaa ggaatcttag taattgttca atccaatctc 660
cacactttaa agatgaagaa actggtattg agaaaaatac acagcttatc caaggttgca 720
ctgctggttg gtagctgaga tgaatttaga acccacatct gatgactaca ccatattgct 780
cccagttttc ctgtctgttc cacatgtaaa agtctgactc ttcacttctc ctttgagtat 840
atagactttt aacatttttg tatgtcaaga tggacttttc ctcataccca gcccctgcct 900
tttctcctcc cttcatacct tgcaggatct ttaacagaat ttaaaaggag ttttttgttt 960
tgttttgatg tatctaataa aagtcaaggg agggagaggg ccagtataag caagagtaca 1020
gtttcctagt ttgtagatgc ggtagtctga ggaatcagaa acacacaaag gtttggagaa 1080
ctggtacatg ctcccaggtg ggaagccagg actcttggta ggatcttgag gacaaggcaa 1140
aggacaataa gagagcgagg ggatcctaga ggtggaatca aggaagagaa actagagaga 1200
gaaaaaggaa ctggctatcc atccatgatg gatcctgtgt ggactgatgg gtggcttggc 1260
atcatccttt agtagacttc atgtggttga ataattggcc aatggaagga atttcttttt 1320
tggtaacaga ctctgtgtgt acagttatgg gtcttaattt ataataaaag gttacattga 1380
aaattgaaaa aaaaaaaa 1398

<210> 64
<211> 2763
<212> DNA
<213> Homo sapiens

<400> 64
ggcacgagga gagaaactcc atctcaaaaa caaaacaaca caaaacaaaa aaagaagaga 60
aatcaaagct tgttccctgt ctctctctct ccacatgtga gcacacaaag aggtcacgtg 120
aacacacaat gagaaggagg ctgcctgcaa gttaagagaa gaggcctcag catgaaacct 180
gccttactgg cactttggtc ttgaacttcc cagcctctaa aactgtgaga aataagtttc 240
tgttgttcaa gccacccagt ctatggtatt ctgtatggca gccagaatta agacaccagt 300
gaagcaagat aatcagtaac tggatactta actgtgtggt ataaaacata ggggctttag 360
tagagaagaa aattggactt tgttggggac atccttacta cttctgctca tgtatcatgc 420
tttagcttgt ttctgtcttt ggaggaggct gcatattttt aaaatacccc caaaagtaca 480
aagactaatg ttatagcccc tgtgttctca ttatccaggc ttaataaatg ttggccattt 540
tccacttttg tttcatatat aagtttctac aaaatgacaa caccttagat aaagctgaag 600
ttcatgtttc attctgcatc ccttccccca agggcttctt ttgctcaata tgggactcat 660
gagagtcatc ggtgttgtgt gaggcagctg tttgttgatt ttctggacca aataatgttc 720
caccgtgtga ctggacatac cttagtctat ccattctacc actgatgagc atgtaagctg 780
ttactatttt taactattac aaattatctt gctaacacat ttttgtgcat gtcttttggt 840
gaccaaatgg actcatttct ctcaggtatg tatctcagag tgaaactgtt ttatcacagt 900
gtatgcttta tatttagtgc tttccaattc ctgattaaga aatctttgcc tgctcctaag 960
gatgtaaaat tattctctta tggcctggct cagtggctca tgcctgtaat cccagcattt 1020
tgggaggcca aggtgggagg attgcttgag gccaggagtt caagaccagc ctgggcaaca 1080
tactgagacc ctcatctcta caaaaaaaaa aaatttgttt aattagctga gcttggtggt 1140
atgcacctat agtcctagct actcaggagg ctgaggcagg aggatcgctt gagcccagga 1200
attcagagat gcagtaagct atgatcatgc cactgtatta cagcctgggt gatagggtga 1260
gaccctgtct ctaaaaagat acatctatta aaaataatat tattttattt tattttattt 1320
tattttatta ttatacttta agttttaggg tacatgtgca cattgtgcag gttagttaca 1380
tatgtataca tgtgccatgc tggtgcactg cacccactaa ctcgtcatct agcattaggt 1440
atatctccca gtgctatccc tcccccctcc cccgacccca caacagtccc cagagtgtga 1500
tgttcccctt cctgtgtcca tgtgatctca ttgttcaatt cccacctatg agtgagaata 1560
tgcggtgttt ggttttttgt tcttgcgata gtttactgag aatgatgatt tccaatttca 1620
tccatgtccc tacaaaggac atgaactcat cattttttat ggctgcatag taaaaataca 1680
ttttaaaaaa taataaatta ttctcttatg ttattgtcta gaatcttcat tattttacct 1740
ttcagattta gatctacaat ccacctggaa ttgatttttg tatatggagt gaggtcccac 1800
acttacaggg aaagcatcac ccgaaagtga gaatgcctag aggcaggaat catggaggct 1860
tccttaaccg tctgtctgca acagcaggtg ctagagatga cactgcagag tagagaacaa 1920
aggaatctta gtaattgttc aatccaatct ccacacttta aagatgaaga aactggtatt 1980
gagaaaaata cacagcttat ccaaggttgc actgctggtt ggtagctgag atgaatttag 2040
aacccacatc tgatgactac accatattgc tcccagtttt cctgtctgtt ccacatgtaa 2100

aagtctgact cttcacttct cctttgagta tatagacttt taacattttt gtatgtcaag 2160
atggactttt cctcataccc agcccctgcc ttttctcctc ccttcatacc ttgcaggatc 2220
tttaacagaa tttaaaagga gttttttgtt ttgttttgat gtatctaata aaagtcaagg 2280
gagggagagg gccagtataa gcaagagtac agtttcctag tttgtagatg cggtagtctg 2340
aggaatcaga aacacacaaa ggtttggaga actggtacat gctcccaggt gggaagccag 2400
gactcttggt aggatcttga ggacaaggca aaggacaata agagagcgag gggatcctag 2460
aggtggaatc aaggaagaga aactagagag agaaaaagga actggctatc catccatgat 2520
ggatcctgtg tggactgatg ggtggcttgg catcatcctt tagtagactt catgtggttg 2580
aataattggc caatggaagg aatttctttt ttggtaacag actctgtgtg tacagttatg 2640
ggtcttaatt tataataaaa ggttacattg aaaattgaaa aaaaaaaaaa aaaaaaaaaa 2700
gcattataag tgtacttcta gccttacttc gtcggggtaa ttcgatctcg ctcttgctgg 2760
ctg 2763

<210> 65
<211> 1863
<212> DNA
<213> Homo sapiens

<400> 65
atgagtaaga gcaaatgctc cgtgggactc atgtcttccg tggtggcccc ggctaaggag 60
cccaatgccg tgggcccgaa ggaggtggag ctcatccttg tcaaggagca gaacggagtg 120
cagctcacca gctccaccct caccaaccct cggcagagcc ccgtggaggc ccaggatcgg 180
gagacctggg gcaagaagat cgactttctc ctgtccgtca ttggctttgc tgtggacctg 240
gccaacgtct ggcggttccc ctacctgtgc tacaaaaatg gtggcggtgc cttcctggtc 300
ccctacctgc tcttcatggt cattgctggg atgccacttt tctacatgga gctggccctc 360
ggccagttca acagggaagg ggccgctggt gtctggaaga tctgccccat actgaaaggt 420
gtgggcttca cggtcatcct catctcactg tatgtcggct tcttctacaa cgtcatcatc 480
gcctgggcgc tgcactatct cttctcctcc ttcaccacgg agctcccctg gatccactgc 540
aacaactcct ggaacagccc caactgctcg gatgcccatc ctggtgactc cagtggagac 600
agctcgggcc tcaacgacac ttttgggacc acacctgctg ccgagtactt tgaacgtggc 660
gtgctgcacc tccaccagag ccatggcatc gacgacctgg ggcctccgcg gtggcagctc 720
acagcctgcc tggtgctggt catcgtgctg ctctacttca gcctctggaa gggcgtgaag 780
acctcaggga aggtggtatg gatcacagcc accatgccat acgtggtcct cactgccctg 840
ctcctgcgtg gggtcaccct ccctggagcc atagacggca tcagagcata cctgagcgtt 900
gacttctacc ggctctgcga ggcgtctgtt tggattgacg cggccaccca ggtgtgcttc 960
tccctgggcg tggggttcgg ggtgctgatc gccttctcca gctacaacaa gttcaccaac 1020
aactgctaca gggacgcgat tgtcaccacc tccatcaact ccctgacgag cttctcctcc 1080
ggcttcgtcg tcttctcctt cctggggtac atggcacaga agcacagtgt gcccatcggg 1140
gacgtggcca aggacgggcc agggctgatc ttcatcatct acccggaagc catcgccacg 1200
ctccctctgt cctcagcctg ggccgtggtc ttcttcatca tgctgctcac cctgggtatc 1260
gacagcgcca tgggtggtat ggagtcagtg atcaccgggc tcatcgatga gttccagctg 1320
ctgcacagac accgtgagct cttcacgctc ttcatcgtcc tggcgacctt cctcctgtcc 1380
ctgttctgcg tcaccaacgg tggcatctac gtcttcacgc tcctggacca ttttgcagcc 1440
ggcacgtcca tcctctttgg agtgctcatc gaagccatcg gagtggcctg gttctatggt 1500
gttgggcagt tcagcgacga catccagcag atgaccgggc agcggcccag cctgtactgg 1560
cggctgtgct ggaagctggt cagcccctgc tttctcctgt tcgtggtcgt ggtcagcatt 1620
gtgaccttca gaccccccca ctacggagcc tacatcttcc ccgactgggc caacgcgctg 1680
ggctgggtca tcgccacatc ctccatggcc atggtgccca tctatgcggc ctacaagttc 1740
tgcagcctgc ctgggtcctt tcgagagaaa ctggcctacg ccattgcacc cgagaaggac 1800
cgtgagctgg tggacagagg ggaggtgcgc cagttcacgc tccgccactg gctcaaggtg 1860
tag 1863

<210> 66
<211> 2770
<212> DNA
<213> Homo sapiens

<400> 66
gagtctggcc gcagtcgcgg cagtggtggc ttcccatccc caaaaggcgc cctccgactc 60
cttgcgccgc actgctcgcc gggccagtcc ggaaacgggt cgtggagctc cgcaccactc 120
ccgctggttc ccgaaggcag atcccttctc ccgagagttg cgagaaactt tcccttgtcc 180
ccgacgctgc agcggctcgg gtaccgtggc agccgcaggt ttctgaaccc cgggccacgc 240
tccccgcgcc tcggcttcgc gctcgtgtag atcgttccct ctctggttgc acgctgggga 300

tcccggacct cgattctgcg ggcgagatgc ccctgggaca catcatgagg ctggacctgg 360
agaaaattgc cctggagtac atcgtgccct gtctgcacga ggtgggcttc tgctacctgg 420
acaacttcct gggcgaggtg gtgggcgact gcgtcctgga gcgcgtcaag cagctgcact 480
gcaccggggc cctgcgggac ggccagctgg cggggccgcg cgccggcgtc tccaagcgac 540
acctgcgggg cgaccagatc acgtggatcg ggggcaacga ggagggctgc gaggccatca 600
gcttcctcct gtccctcatc gacaggctgg tcctctactg cgggagccgg ctgggcaaat 660
actacgtcaa ggagaggtct aaggcaatgg tggcttgcta tccgggaaat ggaacaggtt 720
atgttcgcca cgtggacaac cccaacggtg atggtcgctg catcacctgc atctactatc 780
tgaacaagaa ttgggatgcc aagctacatg gtgggatcct gcggatattt ccagagggga 840
aatcattcat agcagatgtg gagcccattt ttgacagact cctgttcttc tggtcagatc 900
gtaggaaccc acacgaagtg cagccctctt acgcaaccag atatgctatg actgtctggt 960
actttgatgc tgaagaaagg gcagaagcca aaaagaaatt caggaattta actaggaaaa 1020
ctgaatctgc cctcactgaa gactgaccgt gctctgaaat ctgctggcct tgttcatttt 1080
agtaacggtt cctgaattct cttaaattct ttgagatcca aagatggcct cttcagtgac 1140
aacaatctcc ctgctacttc ttgcatcctt cacatccctg tcttgtgtgt ggtacttcat 1200
gttttcttgc caagactgtg ttgatcttca gatactctct ttgccagatg aagttatttg 1260
ctaactccag aaattcctgc agacatccta ctcggccagc ggtttacctg atagattcgg 1320
taatactatc aagagaagag cctaggagca cagcgaggga atgaacctta cttgcacttt 1380
atgtatactt cctgatttga aggaggaggt tttgaaaaga aaaaaatgga ggtggtagat 1440
gccacagaga ggcatcacgg aagccttaac agcaggaaac agagaaattt gtgtcatctg 1500
aacaatttcc agatgttctt aatccagggc tgttggggtt tctggagaat tatcacaacc 1560
taatgacatt aatacctcta gaaagggctg ctgtcatagt gaacaattta taagtgtccc 1620
atggggcaga cactcctttt ttcccagtcc tgcaacctgg attttctgcc tcagctccat 1680
tttgctgaaa ataatgactt tctgaataaa gatggcaaca caattttttc tccattttca 1740
gttcttacct gggaacctaa ttccccagaa gctaaaaaac tagacattag ttgttttggt 1800
tgctttgttg gaatggaatt taaatttaaa tgaaaggaaa aatatatccc tggtagtttt 1860
gtgttaacca ctgataactg tggaaagagc taggtctact gatatacaat aaacatgtgt 1920
gcatcttgaa caatttgaga ggggaggtgg agttggaaat gtgggtgttc ctgttttttt 1980
tttttttttt tttttttttt agtttccctt tttaatgagc tcacccttta acacaaaaaa 2040
agcagggtga tgtattttaa aaaaggaagt ggaaataaaa aaatctcaaa gctatttgag 2100
ttctcgtctg tccctagcag tctttcttca gctcacttgg ctctctagat ccactgtggt 2160
tggcagtatg accagaatca tggaacttgc tagaactgtg gaagcttcta ctcctgcagt 2220
aagcacagat cgcactgcct caataacttg gtattgagca cgtattttgc aaaagctact 2280
tttcctagtt ttcagtatta ctttcatgtt ttaaaaatcc ctttaatttc ttgcttgaaa 2340
atcccatgaa cattaaagag ccagaaatat tttcctttgt tatgtacgga tatatatata 2400
tatatagtct tccaagatag aagtttactt tttcctcttc tggttttgga aaatttccag 2460
ataagacatg tcaccattaa ttctcaacga ctgctctatt ttgttgtacg gtaatagtta 2520
tcaccttcta aattactatg taatttactc acttattatg tttattgtct tgtatccttt 2580
ctctggagtg taagcacaat gaagacagga attttgtata tttttaacca atgcaacata 2640
ctctcagcac ctaaaatagt gccgggaaca tagtaagggc tcagtaaata cttgttgaat 2700
aaactcagtc tcctacatta gcattctaaa aaaaaaaaaa aaaaaaaaaa aaaaaaaaaa 2760
aaaaaaaaag 2770

<210> 67
<211> 1967
<212> DNA
<213> Homo sapiens

<400> 67
ataaaaaccg tcctcgggcg cggcggggag aagccgagct gagcggatcc tcacacgact 60
gtgatccgat tctttccagc ggcttctgca accaagcggg tcttaccccc ggtcctccgc 120
gtctccagtc ctcgcacctg gaaccccaac gtccccgaga gtccccgaat ccccgctccc 180
aggctaccta agaggatgag cggtgctccg acggccgggg cagccctgat gctctgcgcc 240
gccaccgccg tgctactgag cgctcagggc ggacccgtgc agtccaagtc gccgcgcttt 300
gcgtcctggg acgagatgaa tgtcctggcg cacggactcc tgcagctcgg ccaggggctg 360
cgcgaacacg cggagcgcac ccgcagtcag ctgagcgcgc tggagcggcg cctgagcgcg 420
tgcgggtccg cctgtcaggg aaccgagggg tccaccgacc tcccgttagc ccctgagagc 480
cgggtggacc ctgaggtcct tcacagcctg cagacacaac tcaaggctca gaacagcagg 540
atccagcaac tcttccacaa ggtggcccag cagcagcggc acctggagaa gcagcacctg 600
cgaattcagc atctgcaaag ccagtttggc ctcctggacc acaagcacct agaccatgag 660
gtggccaagc ctgcccgaag aaagaggctg cccgagatgg cccagccagt tgacccggct 720
cacaatgtca gccgcctgca ccggctgccc agggattgcc aggagctgtt ccaggttggg 780
gagaggcaga gtggactatt tgaaatccag cctcaggggt ctccgccatt tttggtgaac 840

tgcaagatga cctcagatgg aggctggaca gtaattcaga ggcgccacga tggctcagtg 900
gacttcaacc ggccctggga agcctacaag gcggggtttg ggatccccca cggcgagttc 960
tggctgggtc tggagaaggt gcatagcatc acgggggacc gcaacagccg cctggccgtg 1020
cagctgcggg actgggatgg caacgccgag ttgctgcagt tctccgtgca cctgggtggc 1080
gaggacacgg cctatagcct gcagctcact gcacccgtgg ccggccagct gggcgccacc 1140
accgtcccac ccagcggcct ctccgtaccc ttctccactt gggaccagga tcacgacctc 1200
cgcagggaca agaactgcgc caagagcctc tctggaggct ggtggtttgg cacctgcagc 1260
cattccaacc tcaacggcca gtacttccgc tccatcccac agcagcggca gaagcttaag 1320
aagggaatct tctggaagac ctggcggggc cgctactacc cgctgcaggc caccaccatg 1380
ttgatccagc ccatggcagc agaggcagcc tcctagcgtc ctggctgggc ctggtcccag 1440
gcccacgaaa gacggtgact cttggctctg cccgaggatg tggccgttcc ctgcctgggc 1500
aggggctcca aggaggggcc atctggaaac ttgtggacag agaagaagac cacgactgga 1560
gaagccccct ttctgagtgc aggggggctg catgcgttgc ctcctgagat cgaggctgca 1620
ggatatgctc agactctaga ggcgtggacc aaggggcatg gagcttcact ccttgctggc 1680
cagggagttg gggactcaga gggaccactt ggggccagcc agactggcct caatggcgga 1740
ctcagtcaca ttgactgacg gggaccaggg cttgtgtggg tcgagagcgc cctcatggtg 1800
ctggtgctgt tgtgtgtagg tcccctgggg acacaagcag gcgccaatgg tatctgggcg 1860
gagctcacag agttcttgga ataaaagcaa cctcagaaca cttaaaaaaa aaaaaaaaaa 1920
aaaaaaaaaa aaaaaaaaaa aaaaaaaaaa aaaaaaaaaa aaaaaaa 1967

<210> 68
<211> 1843
<212> DNA
<213> Homo sapiens

<400> 68
ataaaaaccg tcctcgggcg cggcggggag aagccgagct gagcggatcc tcacacgact 60
gtgatccgat tctttccagc ggcttctgca accaagcggg tcttaccccc ggtcctccgc 120
gtctccagtc ctcgcacctg gaaccccaac gtccccgaga gtccccgaat ccccgctccc 180
aggctaccta agaggatgag cggtgctccg acggccgggg cagccctgat gctctgcgcc 240
gccaccgccg tgctactgag cgctcagggc ggacccgtgc agtccaagtc gccgcgcttt 300
gcgtcctggg acgagatgaa tgtcctggcg cacggactcc tgcagctcgg ccaggggctg 360
cgcgaacacg cggagcgcac ccgcagtcag ctgagcgcgc tggagcggcg cctgagcgcg 420
tgcgggtccg cctgtcaggg aaccgagggg tccaccgacc tcccgttagc ccctgagagc 480
cgggtggacc ctgaggtcct tcacagcctg cagacacaac tcaaggctca gaacagcagg 540
atccagcaac tcttccacaa ggtggcccag cagcagcggc acctggagaa gcagcacctg 600
cgaattcagc atctgcaaag ccagtttggc ctcctggacc acaagcacct agaccatgag 660
gtggccaagc ctgcccgaag aaagaggctg cccgagatgg cccagccagt tgacccggct 720
cacaatgtca gccgcctgca ccggctgccc agggattgcc aggagctgtt ccaggttggg 780
gagaggcaga gtggactatt tgaaatccag cctcaggggt ctccgccatt tttggtgaac 840
tgcaagatga cctcagatgg aggctggaca gtaattcaga ggcgccacga tggctcagtg 900
gacttcaacc ggccctggga agcctacaag gcggggtttg ggatccccca cggcgagttc 960
tggctgggtc tggagaaggt gcatagcatc acgggggacc gcaacagccg cctggccgtg 1020
cagctgcggg actgggatgg caacgccgag ttgctgcagt tctccgtgca cctgggtggc 1080
gaggacacgg cctatagcct gcagctcact gcacccgtgg ccggccagct gggcgccacc 1140
accgtcccac ccagcggcct ctccgtaccc ttctccactt gggaccagga tcacgacctc 1200
cgcagggaca agaactgcgc caagagcctc tctggaggct ggtggtttgg cacctgcagc 1260
cattccaacc tcaacggcca gtacttccgc tccatcccac agcagcggca gaagcttaag 1320
aagggaatct tctggaagac ctggcggggc cgctactacc cgctgcaggc caccaccatg 1380
ttgatccagc ccatggcagc agaggcagcc tcctagcgtc ctggctgggc ctggtcccag 1440
gcccacgaaa gacggtgact cttggctctg cccgaggatg tggccaagac cacgactgga 1500
gaagccccct ttctgagtgc aggggggctg catgcgttgc ctcctgagat cgaggctgca 1560
ggatatgctc agactctaga ggcgtggacc aaggggcatg gagcttcact ccttgctggc 1620
cagggagttg gggactcaga gggaccactt ggggccagcc agactggcct caatggcgga 1680
ctcagtcaca ttgactgacg gggaccaggg cttgtgtggg tcgagagcgc cctcatggtg 1740
ctggtgctgt tgtgtgtagg tcccctgggg acacaagcag gcgccaatgg tatctgggcg 1800
gagctcacag agttcttgga ataaaagcaa cctcagaaca ctt 1843

<210> 69
<211> 1552
<212> DNA
<213> Homo sapiens

<400> 69
gcccgtacac accgtgtgct gggacacccc acagtcagcc gcatggctcc cctgtgcccc 60
agcccctggc tccctctgtt gatcccggcc cctgctccag gcctcactgt gcaactgctg 120
ctgtcactgc tgcttctgat gcctgtccat ccccagaggt tgccccggat gcaggaggat 180
tcccccttgg gaggaggctc ttctggggaa gatgacccac tgggcgagga ggatctgccc 240
agtgaagagg attcacccag agaggaggat ccacccggag aggaggatct acctggagag 300
gaggatctac ctggagagga ggatctacct gaagttaagc ctaaatcaga agaagagggc 360
tccctgaagt tagaggatct acctactgtt gaggctcctg gagatcctca agaaccccag 420
aataatgccc acagggacaa agaaggggat gaccagagtc attggcgcta tggaggcgac 480
ccgccctggc cccgggtgtc cccagcctgc gcgggccgct tccagtcccc ggtggatatc 540
cgcccccagc tcgccgcctt ctgcccggcc ctgcgccccc tggaactcct gggcttccag 600
ctcccgccgc tcccagaact gcgcctgcgc aacaatggcc acagtgtgca actgaccctg 660
cctcctgggc tagagatggc tctgggtccc gggcgggagt accgggctct gcagctgcat 720
ctgcactggg gggctgcagg tcgtccgggc tcggagcaca ctgtggaagg ccaccgtttc 780
cctgccgaga tccacgtggt tcacctcagc accgcctttg ccagagttga cgaggccttg 840
gggcgcccgg gaggcctggc cgtgttggcc gcctttctgg aggagggccc ggaagaaaac 900
agtgcctatg agcagttgct gtctcgcttg gaagaaatcg ctgaggaagg ctcagagact 960
caggtcccag gactggacat atctgcactc ctgccctctg acttcagccg ctacttccaa 1020
tatgaggggt ctctgactac accgccctgt gcccagggtg tcatctggac tgtgtttaac 1080
cagacagtga tgctgagtgc taagcagctc cacaccctct ctgacaccct gtggggacct 1140
ggtgactctc ggctacagct gaacttccga gcgacgcagc ctttgaatgg gcgagtgatt 1200
gaggcctcct tccctgctgg agtggacagc agtcctcggg ctgctgagcc agtccagctg 1260
aattcctgcc tggctgctgg tgacatccta gccctggttt ttggcctcct ttttgctgtc 1320
accagcgtcg cgttccttgt gcagatgaga aggcagcaca gaaggggaac caaagggggt 1380
gtgagctacc gcccagcaga ggtagccgag actggagcct agaggctgga tcttggagaa 1440
tgtgagaagc cagccagagg catctgaggg ggagccggta actgtcctgt cctgctcatt 1500
atgccacttc cttttaactg ccaagaaatt ttttaaaata aatatttata at 1552

<210> 70
<211> 2218
<212> DNA
<213> Homo sapiens

<400> 70
cttctctctc cattcagtgc acgcgttact ttggctaaaa ggaggtgagc ggcactctgc 60
ccttccagag caagcatgga gcaacaggat cagagcatga aggaagggag gctgacgctt 120
gtgcttgccc tggcaaccct gatagctgcc tttgggtcat ccttccagta tgggtacaac 180
gtggctgctg tcaactcccc agcactgctc atgcaacaat tttacaatga gacttactat 240
ggtaggaccg gtgaattcat ggaagacttc cccttgacgt tgctgtggtc tgtaaccgtg 300
tccatgtttc catttggagg gtttatcgga tccctcctgg tcggcccctt ggtgaataaa 360
tttggcagaa aaggggcctt gctgttcaac aacatatttt ctatcgtgcc tgcgatctta 420
atgggatgca gcagagtcgc cacatcattt gagcttatca ttatttccag acttttggtg 480
ggaatatgtg caggtgtatc ttccaacgtg gtccccatgt acttagggga gctggcccct 540
aaaaacctgc ggggggctct cggggtggtg ccccagctct tcatcactgt tggcatcctt 600
gtggcccaga tctttggtct tcggaatctc cttgcaaacg tagatggctg gccgatcctg 660
ctggggctga ccggggtccc cgcggcgctg cagctccttc tgctgccctt cttccccgag 720
agccccaggt acctgctgat tcagaagaaa gacgaagcgg ccgccaagaa agccctacag 780
acgctgcgcg gctgggactc tgtggacagg gaggtggccg agatccggca ggaggatgag 840
gcagagaagg ccgcgggctt catctccgtg ctgaagctgt tccggatgcg ctcgctgcgc 900
tggcagctgc tgtccatcat cgtcctcatg ggcggccagc agctgtcggg cgtcaacgct 960
atctactact acgcggacca gatctacctg agcgccggcg tgccggagga gcacgtgcag 1020
tacgtgacgg ccggcaccgg ggccgtgaac gtggtcatga ccttctgcgc cgtgttcgtg 1080
gtggagctcc tgggtcggag gctgctgctg ctgctgggct tctccatctg cctcatagcc 1140
tgctgcgtgc tcactgcagc tctggcactg caggacacag tgtcctggat gccatacatc 1200
agcatcgtct gtgtcatctc ctacgtcata ggacatgccc tcgggcccag tcccataccc 1260
gcgctgctca tcactgagat cttcctgcag tcctctcggc catctgcctt catggtgggg 1320
ggcagtgtgc actggctctc caacttcacc gtgggcttga tcttcccgtt catccaggag 1380
ggcctcggcc cgtacagctt cattgtcttc gccgtgatct gcctcctcac caccatctac 1440
atcttcttga ttgtcccgga gaccaaggcc aagacgttca tagagatcaa ccagattttc 1500
accaagatga ataaggtgtc tgaagtgtac ccggaaaagg aggaactgaa agagcttcca 1560
cctgtcactt cggaacagtg actctggaga ggaagccagt ggagctggtc tgccaggggc 1620
ttcccacttt ggcttatttt tctgacttct agctgtctgt gaatatccag aaataaaaca 1680

actctgatgt ggaatgcagt cctcatctcc agcctcccca ccccagtggg aactgtgcaa 1740
agggctgcct tgctgttctt gaagctgggc tgtctctctc catgttggcc tgtcaccaga 1800
cccgagtcaa ttaaacagct ggtcctccac tttgctggtt cagccttcgt gtggctcctg 1860
gtaacgtggc tccaccttga tgggtcaacc tttgtgtggc tcctggtaac ataacaacaa 1920
cagttactat agtggtgaga tggaaggaat caaattttgc cagagaaact aactcggtgg 1980
ccccaacagg tcttccgggg ccatgggcat ttgtttagag ccaaattcat cctcttacca 2040
gatccttttc cagaaatacc tgtctaggaa ggtgtgatgt cagaaacaat gacatccaga 2100
aagctgagga acaggttcct gtggagacac tgagtcagaa ttcttcatcc aaattatttt 2160
gttagtggaa aatggaattg cttctgtgta gtcaataaaa tgaacctgat cacttttc 2218

<210> 71
<211> 888
<212> DNA
<213> Homo sapiens

<400> 71
ggctggtccc ctgacaggtt gaagcaagta gacgcccagg agccccggga gggggctgca 60
gtttccttcc ttccttctcg gcagcgctcc gcgcccccat cgcccctcct gcgctagcgg 120
aggtgatcgc cgcggcgatg ccggaggagg gttcgggctg ctcggtgcgg cgcaggccct 180
atgggtgcgt cctgcgggct gctttggtcc cattggtcgc gggcttggtg atctgcctcg 240
tggtgtgcat ccagcgcttc gcacaggctc agcagcagct gccgctcgag tcacttgggt 300
gggacgtagc tgagctgcag ctgaatcaca caggacctca gcaggacccc aggctatact 360
ggcagggggg cccagcactg ggccgctcct tcctgcatgg accagagctg gacaaggggc 420
agctacgtat ccatcgtgat ggcatctaca tggtacacat ccaggtgacg ctggccatct 480
gctcctccac gacggcctcc aggcaccacc ccaccaccct ggccgtggga atctgctctc 540
ccgcctcccg tagcatcagc ctgctgcgtc tcagcttcca ccaaggttgt accattgcct 600
cccagcgcct gacgcccctg gcccgagggg acacactctg caccaacctc actgggacac 660
ttttgccttc ccgaaacact gatgagacct tctttggagt gcagtgggtg cgcccctgac 720
cactgctgct gattagggtt ttttaaattt tattttattt tatttaagtt caagagaaaa 780
agtgtacaca caggggccac ccggggttgg ggtgggagtg tggtgggggg tagtggtggc 840
aggacaagag aaggcattga gctttttctt tcattttcct attaaaaa 888

<210> 72
<211> 2308
<212> DNA
<213> Homo sapiens

<400> 72
agcaggtttc gaatgctctt tacttccttt gtggagcaaa agaaaaaagc aggagtattt 60
gaacaaatca ctaagactca tggaacaatt attggcatta cttcagggat tgtcttggtc 120
cttctcatta tttctatttt agtacaagtg aaacagcctc gaaaaaaggt catggcttgc 180
aaaaccgctt ttaataaaac cgggttccaa gaagtgtttg atcctcctca ttatgaactg 240
ttttcactaa gggacaaaga gatttctgca gacctggcag acttgtcgga agaattggac 300
aactaccaga ggatgcggcg ctcctccacc gcctcccgct gcatccacga ccaccactgt 360
gggtcgcagg cctccagcgt caaacaaagc aggaccaacc tcagttccat ggagcttcct 420
ctccgaaatg actttgcaca accacagcca atgaaaacat ttaatagcac cttcaagaaa 480
agtagttaca ctttcaaaca gggacatgag tgccctgagc aggccctgga agaccgagta 540
atggaggaga ttccctgtga aatttatgtc agggggcgag aagattctgc acaagcatcc 600
atatccattg acttctaatc ttctgctaat ggtgatgtga attcttaggg tgtgtacgta 660
cgcagcctcc agggcaccat actgtttcca gcagccaacc cttttctccc atcacaacta 720
cgaagacctt gatttaccgt taacctattg tatggtgatg tttttattct ctcaggcagt 780
ctatatatgt taaaccaatc aaggaactta ctctattcag tggaaacaat aatcatctct 840
attgcttggt gtcatttata ggaagcactg ccagttaaag agcattagaa gaggtggttg 900
gatggagcca ggctcaggct gcctcttcgt tttagcaaca agaagactgc tcttgactga 960
taacagctct gtcaatattt tgatgccaca ataaacttga tttttcttta cattcctttt 1020
atttttcctt tctctaaatt taatttgttt tataagccta tcgttttacc atttcatttt 1080
cttacataag tacaagtggt taatgtacca catacttcag tataggcatt tgttcttgag 1140
tgtgtcaaaa tacagctagt tactgtgcca attaagaccc agttgtattt cacccatctg 1200
tttcttcttg gctaatctct gtacttctgc cttttaatta ctgggccctt attccttatt 1260
ttctgtgaga aataatagat gatatgattt attacctttc aattatattt ttctcagtta 1320
tactagaaaa tttcataatc ctgggatata tgtaccattg tcagctatga ctaaaaattt 1380
gaaaaagata aaaatttcta gcaagccttt gaagtttacc aagtatagtc acattcagtg 1440
acagcccatt cattccagta aagaatcatt tcattcactt tgggagaggc ctataattac 1500

atttatttgc aatgtttctc ttcgctagat tgttacatag ctcccattct gttggttttg 1560
cttacagcat atggtaacca aggttagatg ccagttaaaa ttccttagaa attggatgag 1620
ccttgagatt gcttcttaac tgggacatga catttttcta gctcttatca agaataacaa 1680
cttccacttt tttttaaact gcacttttga ctttttttat ggtataaaaa caataattta 1740
taaacataaa agctcattgt gttttttaga cttttgatat tatttgatac tgtacaaact 1800
ttattaaatc aagatgaaag acctacagga cagattcctt tcagtgttca catcagtggc 1860
tttgtatgca aatatgctgt gttggacctg gacgctataa cttattgtaa agaccttgga 1920
aatgtggaca taagctcttt ctttcctttt gttactgtat ttagtttgtg ataaattttt 1980
cactgtgtga tatttatgct ctaaatcact acacaaatcc catattaaaa tatacattgt 2040
acctgaccct ttaatcatgt tatttatgcc accaaggttg tggatcttaa ggtatgtatg 2100
gaaaggaact catttatcaa attgtaagta atacagacat gccatttaaa agaggtaaat 2160
tcttgttttc tatattttgt tagtaaattc tcaatgaaat aagttgaagt ttcactggat 2220
ttcattaact tttaaatatt acatatatgt gttttctcag attagtgaaa attgtgacct 2280
taaatttaat acacatatac tgcctcag 2308

<210> 73
<211> 933
<212> DNA
<213> Homo sapiens

<400> 73
gctgaatgtt tttttcattt tcctgagaga tgcaaatgtt cattcattca tctcattata 60
caacacagta cataaatgta gcttcagagc gtattaagtg cttttataca caagtgctgg 120
ctgtgtggac caggtggtag ctcatttagg ccccaaattc attaagggca gggtctgact 180
cttaggcttc ttaatatttg gtttggtgca tggtcaagag ctggacccac atgttgcata 240
gcagcagggc tgatatgttt aaagacgctg ggctttttct gctctggggc cctttcccgg 300
gggttccggt gagtccctcc ccaggtgggt ctgcccccac ccgtgtgggc gggattagct 360
cccagaggct ggccaggccc cacctggggg aggcttgagg gcagggcccc aaggctgaga 420
ttcaggcttg ggggaacaga gcaggaaaga gacccggtac cgaaagtgag cggggcaggc 480
acctagtcac atgggtaatg ggcaggggtc ggtcactggc tttggctcca gggccagagc 540
agtctgactt agtgttgagc tccaagcatg gaacactgga gttggttcat tttgaccagc 600
aagcctctaa atgggtgcct tgattaccca ccgcaaggag agggcagttg cctttttatg 660
acatgttaat tccagccagg tgagtcacca ggtagctctc atcctcctgc caggctcccc 720
tgcctgtcgg tttggcattg tcagataatg tgatcattca ttgaagtgac atttgagttc 780
caaaccagtt ttctccttta accatttcac cctcaggagt gattctcctt tgtttggcat 840
tgtcagggaa tgtgatgatc cattcaaatg acttttgagt tccaaatagt gtttctactt 900
taacttccta aatgaaaaaa aaaaaaaaaa aaa 933

<210> 74
<211> 561
<212> DNA
<213> Homo sapiens

<400> 74
tttttttttt tcatttagga agttaaagta gaaacactat ttggaactca aaagtcattt 60
gaatggatca tcacattccc tgacaatgcc aaacaaagga gaatcactcc tgagggtgaa 120
atggttaaag gagaaaactg gtttggaact caaatgtcac ttcaatgaat gatcacatta 180
tctgacaatg ccaaaccgac aggcagggga gcctggcagg aggatgagag ctacctggtg 240
actcacctgg ctggaattaa catgtcataa aaaggcaact gccctctcct tgcggtgggt 300
aatcaaggca cccatttaga ggcttgctgg tcaaaatgaa ccaactccag tgttccatgc 360
ttggagctca acactaagtc agactgctct ggccctggag ccaaagccag tgaccgaccc 420
ctgcccatta cccatgtgac taggtgcctg ccccgctcac tttcggtacc gggtctcttt 480
cctgctctgt tcccccaagc ctgaatctca gccttggggc cctgccctca agcctccccc 540
aggtggggcc tggccagcct c 561

<210> 75
<211> 837
<212> DNA
<213> Homo sapiens

<400> 75
atcattgcag ccacgattaa taatgccagc atgacttccc caattgacaa tgctggactt 60
gcagcagatg acttcaaaat gaatgcatcc ttgcagacac agatctccac ggatgctgaa 120

gcagttagct cggccaagag tgagataatg gagctgaaac aagtcctcca gtccctccag 180
catgaactgc agtctgtctt ggccatgcaa agctccccag aaggaaccct ggctgacaca 240
gaagctggct acgtggctca gctgtcagaa attaagatgt atatcagcat cctggaggag 300
cagatctgcc agatccgggg cgagactgaa taccagaaca cagagtatgc acaactgcag 360
gacatcaaga cacgcctgga ggtggagatc gagacctacc accgcctgct cggtggagag 420
ggaggttctg aggccagaga agctgaatct aaaggatgat tctctgtgga ctccaaagga 480
ataaccaaca cagctcaatc gaggggaaga gtcgcttttt aaaattcaca taaaagcaga 540
ggaaccaggc tggtgacgga taatgaccaa ctaatttcat ctcaacactg tttctgatgt 600
aaaattcaag agtaagatgc aggcagctga gtcctgtccc caggatgtaa atgatgacac 660
ctaacaaagg cgtgctgaag cactgcaaca aacctgcggc cacagagctg cagctactcc 720
tgtaatcctt tctcagattc ttttgtctta ttggttcggt tttgtcttta aattgcaaaa 780
tgtgttttcc ttcttaatca ttctgtgtcg ataaactttt tgcctgttgc aaatcaa 837

<210> 76
<211> 3057
<212> DNA
<213> Homo sapiens

<400> 76
cactttcgag agtgccgtct atttgccaca cacttccctg atgaaatgtc tggatttgga 60
ctaaagaaaa aaggaaaggc tagcagtcat ccaacagaat catgagacag actttgcctt 120
gtatctactt ttgggggggc cttttgccct ttgggatgct gtgtgcatcc tccaccacca 180
agtgcactgt tagccatgaa gttgctgact gcagccacct gaagttgact caggtacccg 240
atgatctacc cacaaacata acagtgttga accttaccca taatcaactc agaagattac 300
cagccgccaa cttcacaagg tatagccagc taactagctt ggatgtagga tttaacacca 360
tctcaaaact ggagccagaa ttgtgccaga aacttcccat gttaaaagtt ttgaacctcc 420
agcacaatga gctatctcaa ctttctgata aaacctttgc cttctgcacg aatttgactg 480
aactccatct catgtccaac tcaatccaga aaattaaaaa taatcccttt gtcaagcaga 540
agaatttaat cacattagat ctgtctcata atggcttgtc atctacaaaa ttaggaactc 600
aggttcagct ggaaaatctc caagagcttc tattatcaaa caataaaatt caagcgctaa 660
aaagtgaaga actggatatc tttgccaatt catctttaaa aaaattagag ttgtcatcga 720
atcaaattaa agagttttct ccagggtgtt ttcacgcaat tggaagatta tttggcctct 780
ttctgaacaa tgtccagctg ggtcccagcc ttacagagaa gctatgtttg gaattagcaa 840
acacaagcat tcggaatctg tctctgagta acagccagct gtccaccacc agcaatacaa 900
ctttcttggg actaaagtgg acaaatctca ctatgctcga tctttcctac aacaacttaa 960
atgtggttgg taacgattcc tttgcttggc ttccacaact agaatatttc ttcctagagt 1020
ataataatat acagcatttg ttttctcact ctttgcacgg gcttttcaat gtgaggtacc 1080
tgaatttgaa acggtctttt actaaacaaa gtatttccct tgcctcactc cccaagattg 1140
atgatttttc ttttcagtgg ctaaaatgtt tggagcacct taacatggaa gataatgata 1200
ttccaggcat aaaaagcaat atgttcacag gattgataaa cctgaaatac ttaagtctat 1260
ccaactcctt tacaagtttg cgaactttga caaatgaaac atttgtatca cttgctcatt 1320
ctcccttaca catactcaac ctaaccaaga ataaaatctc aaaaatagag agtgatgctt 1380
tctcttggtt gggccaccta gaagtacttg acctgggcct taatgaaatt gggcaagaac 1440
tcacaggcca ggaatggaga ggtctagaaa atattttcga aatctatctt tcctacaaca 1500
agtacctgca gctgactagg aactcctttg ccttggtccc aagccttcaa cgactgatgc 1560
tccgaagggt ggcccttaaa aatgtggata gctctccttc accattccag cctcttcgta 1620
acttgaccat tctggatcta agcaacaaca acatagccaa cataaatgat gacatgttgg 1680
agggtcttga gaaactagaa attctcgatt tgcagcataa caacttagca cggctctgga 1740
aacacgcaaa ccctggtggt cccatttatt tcctaaaggg tctgtctcac ctccacatcc 1800
ttaacttgga gtccaacggc tttgacgaga tcccagttga ggtcttcaag gatttatttg 1860
aactaaagat catcgattta ggattgaata atttaaacac acttccagca tctgtcttta 1920
ataatcaggt gtctctaaag tcattgaacc ttcagaagaa tctcataaca tccgttgaga 1980
agaaggtttt cgggccagct ttcaggaacc tgactgagtt agatatgcgc tttaatccct 2040
ttgattgcac gtgtgaaagt attgcctggt ttgttaattg gattaacgag acccatacca 2100
acatccctga gctgtcaagc cactaccttt gcaacactcc acctcactat catgggttcc 2160
cagtgagact ttttgataca tcatcttgca aagacagtgc cccctttgaa ctctttttca 2220
tgatcaatac cagtatcctg ttgattttta tctttattgt acttctcatc cactttgagg 2280
gctggaggat atctttttat tggaatgttt cagtacatcg agttcttggt ttcaaagaaa 2340
tagacagaca gacagaacag tttgaatatg cagcatatat aattcatgcc tataaagata 2400
aggattgggt ctgggaacat ttctcttcaa tggaaaagga agaccaatct ctcaaatttt 2460
gtctggaaga aagggacttt gaggcgggtg tttttgaact agaagcaatt gttaacagca 2520
tcaaaagaag cagaaaaatt atttttgtta taacacacca tctattaaaa gacccattat 2580
gcaaaagatt caaggtacat catgcagttc aacaagctat tgaacaaaat ctggattcca 2640

ttatattggt tttccttgag gagattccag attataaact gaaccatgca ctctgtttgc 2700
gaagaggaat gtttaaatct cactgcatct tgaactggcc agttcagaaa gaacggatag 2760
gtgcctttcg tcataaattg caagtagcac ttggatccaa aaactctgta cattaaattt 2820
atttaaatat tcaattagca aaggagaaac tttctcaatt taaaaagttc tatggcaaat 2880
ttaagttttc cataaaggtg ttataatttg tttattcata tttgtaaatg attatattct 2940
atcacaatta catctcttct aggaaaatgt gtctccttat ttcaggccta tttttgacaa 3000
ttgacttaat tttacccaaa ataaaacata taagcacgta aaaaaaaaaa aaaaaaa 3057

<210> 77
<211> 604
<212> DNA
<213> Homo sapiens

<400> 77
atcaaggagg acagatttta tatgcttaag actggaggga gagagaccag ttagaaagac 60
tctggctata atccaagcaa gccatactat gggctggttt ggtggaggta gaggaatgtc 120
cagattggag aaacaggaag ttaaaaatgg gcagggcttg ctgactgttt gaaactaggg 180
ggtgtgaagg gaggcagcac tctaggataa acaccagact tgccgattgt ttgggaatat 240
ccaattctgc tgtagaagac aggaaaacaa taaactcaaa agaagtgtta cacatagata 300
actaaattag tcatctgtag atagtacaac atattcgtgt gtacagagat caaaagggac 360
agaaagacca aggaggcact aaatattcac ggtaccctaa atacgtgaga catgaggcac 420
tcaggatgca ggaggtttca atttgacagg atggatgcac actgcatgac ccacagttca 480
gctggtgtga tcattccacc caacttgccc cctctcccgt gtgttgaccc accccagcac 540
ctgcctcctg cttctcagaa tgtgttttac atttatttaa taaacgatgt tatttgagca 600
ttta 604

<210> 78
<211> 623
<212> DNA
<213> Homo sapiens

<400> 78
tttttttttt ttttttttta aatgctcaaa taacatcgtt tattaaataa atgtaaaaca 60
cattctgaga agcaggaggc aggtgctggg gtgggtcaac acacgggaga gggggcaagt 120
tgggtggaat gatcgatcac accagctgaa ctgtgggtca tgcagtgtgc atccatcctg 180
tcaaattgaa acctcctgca tcctgagtgc ctcatgtctc acgtatttag ggtaccgtga 240
atatttagtg cctccttggt ctttctgtcc cttttgatct ctgtacacac gaatacgttg 300
tactatctac agatgactaa tttagttatc tgtgtgtaac acttcttttg agtttattgt 360
tttcctgtct tctacagcag aattggatat tcccaaacaa tctgcaagtc tggtgtttat 420
cctagagtgc tgcctccctt cacaccccct agtttcaaac agtcagcaag ccctgcccat 480
ttttaacttc ctggtttctc aatctggaca ttcctctacc tccaccaaac cagcccatag 540
tatggcttgc ttggattata gccagagtct ttctaactgg tctctctccc tccagtctta 600
agcttaaaat cgtctccttg att 623

<210> 79
<211> 3655
<212> DNA
<213> Homo sapiens

<400> 79
cttcagatag attatatctg gagtgaagaa tcctgccacc tatgtatctg gcatagtatt 60
ctgtgtagtg ggatgagcag agaacaaaaa caaaataatc cagtgagaaa agcccgtaaa 120
taaactttca gaccagagat ctattctcta gcttatttta agctcaactt aaaaggaaga 180
actgttctct gattcttttc gccttcaata cacttaatga tttaactcca ccctccttca 240
aaagaaacag catttcctac ttttatactg tctatatgat tgatttgcac agctcatctg 300
gccagaagag ctgagacatc cgttccccta caagaaactc tccccgggtg gaacaagatg 360
gattatcaag tgtcaagtcc aatctatgac atcaattatt atacatcgga gccctgccaa 420
aaaatcaatg tgaagcaaat cgcagcccgc ctcctgcctc cgctctactc actggtgttc 480
atctttggtt ttgtgggcaa catgctggtc atcctcatcc tgataaactg caaaaggctg 540
aagagcatga ctgacatcta cctgctcaac ctggccatct ctgacctgtt tttccttctt 600
actgtcccct tctgggctca ctatgctgcc gcccagtggg actttggaaa tacaatgtgt 660
caactcttga cagggctcta ttttataggc ttcttctctg gaatcttctt catcatcctc 720

ctgacaatcg ataggtacct ggctgtcgtc catgctgtgt ttgctttaaa agccaggacg 780
gtcacctttg gggtggtgac aagtgtgatc acttgggtgg tggctgtgtt tgcgtctctc 840
ccaggaatca tctttaccag atctcaaaaa gaaggtcttc attacacctg cagctctcat 900
tttccataca gtcagtatca attctggaag aatttccaga cattaaagat agtcatcttg 960
gggctggtcc tgccgctgct tgtcatggtc atctgctact cgggaatcct aaaaactctg 1020
cttcggtgtc gaaatgagaa gaagaggcac agggctgtga ggcttatctt caccatcatg 1080
attgtttatt ttctcttctg ggctccctac aacattgtcc ttctcctgaa caccttccag 1140
gaattctttg gcctgaataa ttgcagtagc tctaacaggt tggaccaagc tatgcaggtg 1200
acagagactc ttgggatgac gcactgctgc atcaacccca tcatctatgc ctttgtcggg 1260
gagaagttca gaaactacct cttagtcttc ttccaaaagc acattgccaa acgcttctgc 1320
aaatgctgtt ctattttcca gcaagaggct cccgagcgag caagctcagt ttacacccga 1380
tccactgggg agcaggaaat atctgtgggc ttgtgacacg gactcaagtg ggctggtgac 1440
ccagtcagag ttgtgcacat ggcttagttt tcatacacag cctgggctgg gggtggggtg 1500
ggagaggtct tttttaaaag gaagttactg ttatagaggg tctaagattc atccatttat 1560
ttggcatctg tttaaagtag attagatctt ttaagcccat caattataga aagccaaatc 1620
aaaatatgtt gatgaaaaat agcaaccttt ttatctcccc ttcacatgca tcaagttatt 1680
gacaaactct cccttcactc cgaaagttcc ttatgtatat ttaaaagaaa gcctcagaga 1740
attgctgatt cttgagttta gtgatctgaa cagaaatacc aaaattattt cagaaatgta 1800
caacttttta cctagtacaa ggcaacatat aggttgtaaa tgtgtttaaa acaggtcttt 1860
gtcttgctat ggggagaaaa gacatgaata tgattagtaa agaaatgaca cttttcatgt 1920
gtgatttccc ctccaaggta tggttaataa gtttcactga cttagaacca ggcgagagac 1980
ttgtggcctg ggagagctgg ggaagcttct taaatgagaa ggaatttgag ttggatcatc 2040
tattgctggc aaagacagaa gcctcactgc aagcactgca tgggcaagct tggctgtaga 2100
aggagacaga gctggttggg aagacatggg gaggaaggac aaggctagat catgaagaac 2160
cttgacggca ttgctccgtc taagtcatga gctgagcagg gagatcctgg ttggtgttgc 2220
agaaggttta ctctgtggcc aaaggagggt caggaaggat gagcatttag ggcaaggaga 2280
ccaccaacag ccctcaggtc agggtgagga tggcctctgc taagctcaag gcgtgaggat 2340
gggaaggagg gaggtattcg taaggatggg aaggagggag gtattcgtgc agcatatgag 2400
gatgcagagt cagcagaact ggggtggatt tggtttggaa gtgagggtca gagaggagtc 2460
agagagaatc cctagtcttc aagcagattg gagaaaccct tgaaaagaca tcaagcacag 2520
aaggaggagg aggaggttta ggtcaagaag aagatggatt ggtgtaaaag gatgggtctg 2580
gtttgcagag cttgaacaca gtctcaccca gactccaggc tgtctttcac tgaatgcttc 2640
tgacttcata gatttccttc ccatcccagc tgaaatactg aggggtctcc aggaggagac 2700
tagatttatg aatacacgag gtatgaggtc taggaacata cttcagctca cacatgagat 2760
ctaggtgagg attgattacc tagtagtcat ttcatgggtt gttgggagga ttctatgagg 2820
caaccacagg cagcatttag cacatactac acattcaata agcatcaaac tcttagttac 2880
tcattcaggg atagcactga gcaaagcatt gagcaaaggg gtcccataga ggtgagggaa 2940
gcctgaaaaa ctaagatgct gcctgcccag tgcacacaag tgtaggtatc attttctgca 3000
tttaaccgtc aataggcaaa gggggaaggg gacatattca tttggaaata agctgccttg 3060
agccttaaaa cccacaaaag tacaatttac cagcctccgt atttcagact gaatgggggt 3120
ggggggggcg ccttaggtac ttattccaga tgccttctcc agacaaacca gaagcaacag 3180
aaaaaatcgt ctctccctcc ctttgaaatg aatatacccc ttagtgtttg ggtatattca 3240
tttcaaaggg agagagagag gtttttttct gttctttctc atatgattgt gcacatactt 3300
gagactgttt tgaatttggg ggatggctaa aaccatcata gtacaggtaa ggtgagggaa 3360
tagtaagtgg tgagaactac tcagggaatg aaggtgtcag aataataaga ggtgctactg 3420
actttctcag cctctgaata tgaacggtga gcattgtggc tgtcagcagg aagcaacgaa 3480
gggaaatgtc tttccttttg ctcttaagtt gtggagagtg caacagtagc ataggaccct 3540
accctctggg ccaagtcaaa gacattctga catcttagta tttgcatatt cttatgtatg 3600
tgaaagttac aaattgcttg aaagaaaata tgcatctaat aaaaaacacc ttcta 3655

<210> 80
<211> 1510
<212> DNA
<213> Homo sapiens

<400> 80
gcccctgaag ttagcggaag ggaacgggca tgttttggaa aggcccgccg ggaattccct 60
cagaaaccag gcgaagctcc ctgctgggcc aggcccccct cgtgtgcttg aggaggatgg 120
gagcccggcc tcggtttgtt atcctaatta agtggaaaat taagaaaata atagaatagc 180
gtaagtacta atagtaaaat tatagtaaga gaaataacaa tagctagaat ggaatgaatt 240
gctgtattaa ccaaggctag aaagaatttc agccctccga aagttaaagc gagggaagag 300
agagaccctc tcatattgtt ttatactcag tacctgtttt tttttttttt ttttaaaaaa 360
aggaagtgaa atcaaagaca ggcagcccag cgccaggccc aaaactggac ctgggcctgc 420

ctggcctaaa cctagtagtt aaaaatcaac tcatgactta gaaaccgatg ttattcatag 480
attccagaca ttctatagaa gaacattgtg aaactccctg ccctgttctg tttctctctg 540
accatcggtg catgcagccc ctgtcacgta ccccttgctt gctcaaatca atcacgaccc 600
tttcatgtga aattttagtg ttgtgagctc ttaaaagcga cagaaattgt gcactcgggg 660
agctcggatt ttaaggcagt agcttgccaa tgctcccagc tgaataaagc cctttcttct 720
acagctccgt gtctgagagg ttttgtctgt ggcttgtcct gctacagtcc ttggttccct 780
gaccaggaag cgaggtgact gacaagccct gtggagcgtc cctgcagagg actccggcca 840
gcctgagtga cgcaatccaa agagcgctcc cgggtaggaa atggccccgg tggaatgcct 900
tgccagagca gcgcatagca ggctcccgag aaggattaac actggctgaa caccgggaag 960
gaactggcac ttgaagtcca gacatctgaa gcttgtcaag actagtcttt ggaacttgcc 1020
ccactccatg tgagtggaag catggcctga tcacccacgg tgtgcctgta ttgacatctt 1080
tgttctggtt ttgacttggc ttgacttggt aagactagtc tttggaactt gccccactcc 1140
acgtgagtgg aagcgtggcc tgatcaccca cggtgtgcct gtattggcac ttttgttctg 1200
gttttgactt ggcttgactt ggtaagacta gtctttggaa cttgccccac tccatctgag 1260
tggaaacgtg gcctgatcac ccacagcatg cctgcatggg cactcttgtt ctggttttga 1320
cttgacttga attgctggat actttggttt tggttttgac ttggcttgaa ttttttggta 1380
ctcggatttt gaatttcgtg attttggttt ggtataaacg gtaaaagtgt gtgtgccctc 1440
tttacctgtt ctttgtcttg tggtatgtgt ggtgtgagcg tggtattttg tctggaaaaa 1500
aaaaaaaaaa 1510

<210> 81
<211> 5463
<212> DNA
<213> Homo sapiens

<220>
<221> modified_base
<222> (2262)...(2287)
<223> n = g, a, c or t

<400> 81
cgctgccggg ccggggctgg gccggagcgg cggcgcctgg atgccggccc ggggctgggc 60
cggagactgg cgcccgcccc gaagcgactt tcggtccccg acccgctccg cctgacccct 120
atgatgaaga gggcgtccac tggagttctt gctgtcgaca tctttgacaa gttcaacttc 180
acgggagcca gagtcccatg gttcgacgcc attcatgaag aattccccag ggagctggag 240
tcctccatct ccttcccagc caacttcttc aaaccacctg aagaaaaagt gcttaggtag 300
aggttgagca tcctaatcct gaaatctgaa atgctccaga atctgaacct tcttgagcac 360
caacctgaca cttaaaggaa gtgctcattg gaggatctgt gacaattact aaaaacttcc 420
ccaaataaaa aggacatgaa aagaatctca cgctatcgcc caggctggag tgcagtggcg 480
cgatctccgc tcactgcaag ctccgcctcc cgggttcgcg ccattctcct gcctcagcct 540
cccgagagta gctgggactg caggcgcccg ccaccacgcc cagctaattt tttgtatttt 600
tagtggagac ggggtttcac cgtgttggcc agaatggttt cgatctcctc acctcgcgat 660
ccatccgcct cagcctccca aagtgctggg attacaggcg tgagccaccg tgcctggccc 720
tcatgtgtct taaaaagagg ggtgactctg atgtggggtc atgcttgaaa ctgagaggac 780
gaggtccccg accctccatt gcagccagct ctctctggaa ctaacagagt gagaactcgc 840
tcattaccaa ggggaggaca ccaagccatt catgggggac ccacccccat gacccaaaca 900
accccgacca cgcccacctc caacccttaa gcagtccatc tccagaactc ttttcatcct 960
gcagatctga aactctagcc cacaaaacac caactccact ccccagccac tggtgcccac 1020
cgttgtttct gtctctgaat ttgactcctc tagggacctc ccataagtgg aatcatacaa 1080
tatttgtcct tttgtgcttg gcttatttca ctgagcgtaa tgttctctag gttcacctgt 1140
gttgtagcaa acgtcagact ttccctcctt ttgaaggctg agtaatattc tgttgtgtgg 1200
atggagcacg cattgtttat ccactcacct gttgacgcat gcctgggtcg tttccacctt 1260
tcggcctgtg tgaatggtgc tgctctgaac aggggtgaag acgtatctgg gcgtctgctt 1320
tcagttcttt tgggtgtata cccagaagtg ggcttgctgg gtcacgtggt aaatcctgtg 1380
gttgattttt tgaggacgca ccatactgtt ttcggtggga catctgttgg gttttgagaa 1440
gcacctgctg gcactgagag ctgtcttggg gaacacaggt gctcctgccg gagtcctcac 1500
gctctgctca cgttgcagaa ggccccctgg taaggccagc cagccggagg accaccccgc 1560
agaccacacg cccagggcct ggcaccgagg gggaggcccc gatcagcagg tgaaagcgac 1620
accctgatga cactggctag ttcaccttca ctctggtcat cgtttactgt accccggggc 1680
agctcccgcc tgagccctat gaccccaacc gccgcagcct ctggtaaggc ctgggtatgg 1740
ggcacatgga gagggcccat tccagaggct cctaagtcaa aagagattcc acaagtcaaa 1800
atcctgatcc tgatcaagca caggccttca cagtgacatt tcactgtgac cctggcccag 1860
agacagagac cactgtcctg cagtgtccct gggagggagg ttctgggtct gccacaggac 1920

accacaccct ggaacattgc acaccctttg cctggccttt tcccagcgtt tctgtcagaa 1980
atgccccctc ccaagcatac accccatctt gtgccccgag ggactcgatt ctctccccac 2040
acacccgacc cgaggctgtc atgggtagtg cctgctgctg gccccagggt tagggctgct 2100
cagccagtcc tggctggaaa ggatgaatgt cccacaagca gctttggggc ctgcttggtg 2160
ggagcagccc gaggatccgc ccttcgtcct cctgccattt cccacttccc tggctctggg 2220
gtcctcatgt tcacgtggtt ctcagtgact gggtgattga cnnnnnnnnn nnnnnnnnnn 2280
nnnnnnnggt gtcttttgct ggacatttaa gccattcctt gtgtttctgc cattagctat 2340
tatgggctgt aacatctttg cacctgtgcc ttctttcttt ttaaatttct gaagtcctaa 2400
tttgacccaa aggtgacaca tctccctgtg tgccctgtca gtgatgaaac ctgctctggg 2460
gatgaagatg ccgtggggcc cacagatcca agctctcgga gtgtgattct gtctccccta 2520
gaaacacatc catggcatgt cctcagggca tttgtgcatg ggattaagca aatgtttcct 2580
aatcatgttt gtccctgtgc tctggcttct tgacctcagg ctgcctcact ggcccatcat 2640
taatacgtcg atggtgagcg cactggtgta cagcgagggg gctccacttc cgagccccct 2700
ggagaggccc gtcctggtgg agttcaccct gctggagatg gagaagcgaa ccaagcctgt 2760
ctgtgtgttc tggaaccatt ccctggcgta aatgtgatgc cgggagttct gcatcttcat 2820
gtgagggctt tgccaagaca cagagaaccc gcagggttgt tggctcagcg gcagatcgtc 2880
ccacggggac agtctcttgt ggtctgtgtc tcccccttca gccccctgtg gggcagttag 2940
cctcctgctc tgcttctgtg ggtgcatcag agccccccca gatgcccaca cgcagacgcg 3000
cacaggcctg cacgtggaaa cacagacatg cgcacacgca ggcccacaca cagacgcaca 3060
cagagctgca cgtggagaca cgtgcgcaca cgcaggccca cacgcaggcc cacacacagg 3120
tgcacgcgga cctgcacgtg gagacacaga tgtgtgcaca tgcaggccca cacacaggtg 3180
agcgtggacc tgtgtgaaca gatgcgcgca gacctgcacg tggagacaca gacatgcagg 3240
cgcacaaaga gatccctcaa ggctgacacc caggaagggg cctctcactt gtgggactcc 3300
cgagatgcag tggccaacac tagtgtagtg cccagttcac tgctggataa gacagaggcc 3360
tgttctggtc acacatggga ggcagaaccc agagactggg cccaggagcc cttctgctga 3420
cagtgggaac tcccagctac gtgtgggggt ccccatacca gacaaaggtc cctgacctta 3480
gtcttgcccg agaggccgac acagcccagc tttggggtct ggctttaccc acaagaggcc 3540
acaccttgcc acagcactgt ttatctggcc tgtttcagaa gcaccgtcag agtggcgaag 3600
gcaggaggtg gtgcacgaga gtctacgttc tagcatccat tcaagtgagg gaaaggcgtt 3660
tcgtacttag aaaaatgcaa aattaatgat tctcacccac acatgagact gtggattcac 3720
ccatgtatga gacagtggat tcagagccat gctgtggctc tcatctgctg ggagccagta 3780
gtgtttgtgc tttggttttg tttttttagc tacatggttt atgtgaggtg cggacttact 3840
tgagttgctc tgtctttaca gggagcttgc tttttctctg ctggtttgtt ttcacttttg 3900
ctgtctctgt ttagaagaaa acattagaac tggggtgtgg tggaaggatg ggagcacaat 3960
ctgggagggg aagacctgct ctctctccac ataccaggag ggagtggcag gaaggggaga 4020
cactgtgccg gactcaggga gtcaagagcg tgagcacccc caggcttcaa tctgctgaac 4080
gttgtgcatc tgggggcgcc cgtgcagcag gaacactctc cccatcacaa ttcctctggg 4140
cacagtggag agccatgagt gggggctgtc ctgggggctg cgctatggtg agtgagccca 4200
cgccaggagc cagttcagcc cagcctcatt cttctcctgc ttctttgctt tccaagttct 4260
agttcataag caaccagcac agtacctgga atgtaagagg cgcttggctt ataaaagaga 4320
gaagaaagga acccctttca ccacgaggga cgtcaggccc agctctctgt cccgggcatt 4380
catctttttt ccggcaatta gcttcagctc taatgacctg ctgtctgctt tatgttgcac 4440
ctatgatttc cagattgtaa agtttgtggc aaatgtatta acgttcacat cccatttaca 4500
gattagcaaa ctaaggatct gagaggttat gtgattttcc cagtctggcg gagggcaggg 4560
ccctgtcaga agccgagaca cacctcgtaa cagcttgtta tttggggctt cccatcagtg 4620
aggcaggagt cataatgggg ccctggcttc tgggcaattc ccataaaaca ttgtgccacc 4680
tgaagatgga agtgtcagag gaaggcgaga tgttatttct gaaagcatct accctcagaa 4740
agagacagag gagtgcatcg cagctgtgtg aacccggggt ggtggtggtt ccagcagcgg 4800
ggcacgtggt ggacgcaaga cggccccgtt tgagttttgc tgctaggtga cacgtagaac 4860
ttgaacttca gagggtgctc cttgggggcc ctgatgagag agacacagaa gggaagggag 4920
actgttccca tggtcctgcc tctgcggcag cgagcctgct gctcttgcag ctgggtggct 4980
gttcggtgca gggggccggt gaggaacacg gtggggagca gcctgcgagg gcgccgggct 5040
ttcagcactc actatacctg ctgtttggtt tggccaggat ggcctgggag tccccagggc 5100
cgggcaaaga ctgcccccaa cagacatggg cggagggaac cgtctcaccc ctgcctcttc 5160
tcccttctct acgcagtgtc agtgggacgg gagggtggtc tgcccggggc ttcaagctcc 5220
tgtccaggaa ccggacccat gtcgtacgcc agtgcagcca cacggccagc ttcgaggtgc 5280
tcatggacgt ctccaggtgt gagatgggca tcttcttgtg gccactatgc ccgctgagcc 5340
tacccccaaa cctgcccctc ctcaaactaa atcttccatt ctaagcactt tgaacgaagc 5400
aaatcaacca attaaaaaaa tatattccag gaaaaaaaaa aaaaaaaaaa aaaaaaaaca 5460
aaa 5463

<210> 82

<211> 1731
<212> DNA
<213> Homo sapiens

<400> 82
ccacaggggc ccccagggcc accagggatc cctggagcca agggcgagct tggattgccc 60
ggtgccccag gaatcgatgg agagaagggc cccaaaggac agaaaggaga cccaggagag 120
cctgggccag caggactcaa aggggaagca ggcgagatgg gcttgtccgg cctcccgggc 180
gctgacggcc tcaaggggga gaagggggag tcggcatctg acagcctaca ggagagcctg 240
gctcagctca tagtggagcc agggccccct ggcccccctg gccccccagg cccgatgggc 300
ctccagggaa tccagggtcc caagggcttg gatggagcaa agggagagaa gggtgcgtcg 360
ggtgagagag gccccagcgg cctgcctggg ccagttggcc caccgggcct tattgggctg 420
ccaggaacca aaggagagaa gggcagaccc ggggagccag gactagatgg tttccctgga 480
ccccgaggag agaaaggtga tcggagcgag cgtggagaga agggagaacg aggggtcccc 540
ggccggaaag gagtgaaggg ccagaagggc gagccgggac caccaggcct ggaccagccg 600
tgtcccgtgg gccccgacgg gctgcctgtg cctggctgct ggcataagtg acccacaggc 660
ccagctcaca cctgtacaga tccgtgtgga catttttaat ttttgtaaaa acaaaacagt 720
aatatattga tcttttttca tggaatgcgc tacctgtggc cttttaacat tcaagagtat 780
gcccacccag ccccaaagcc accggcatgt gaagctgccg gaaagtggac aggccagacc 840
agggagatgt gtacctgagg ggcacccttg ggcctgggct ttcccaggaa ggagatgaag 900
gtagaagcac ctggctcggg caaggctaga aagatgctac gttgggcctt cagtcacctg 960
atcagcagag agactctcag ctgtggtact gccctgtaag aacctgcccc cgcaaaactc 1020
tggagtccct gggacacacc ctatccaaga agacccaggg gtggaacagc ggctgctgtt 1080
gctcctggcc tcatcagcct ccaaactcaa ccacaaccag ctgcctctgc agttggacaa 1140
gacttggccc ccggacaaga ctcgcccagc acttgcggct gggcccgggg agcagtgagt 1200
ggaaatcccc cacgagggtc tagctctacc acattcagga ggcctcagga ggccagcctg 1260
ccatgagagc acatgtcctc tggccaggag tagtggctga gctctgtgat cgctgtgatg 1320
tggacccagc tccagggagc agagtgtcga ggatggaggg gcccagcctg gactgactgc 1380
tacttcctgt ctctgtttcc attatcaccc agagagggac aagataggac atggcctgga 1440
ccagggaggc aggcctccca ctcagagtct gggtctcact ggccccaagt ctcccaccca 1500
gaactctggc caaaaatggc tctctaggtg ggctgtgcag gcaaagcaaa gctcagggct 1560
ggttcccagc tggcctgagc agggggcctg ccaccagacc cacccacgct ctgacgagag 1620
gcttttccac ctccagcaag tgttcccagc aaccagctcc atcctggctg cttgccttcc 1680
atttccgtgt agatggagat cactgtgtgt aataaaccac aagtgcgtgt c 1731

<210> 83
<211> 1782
<212> DNA
<213> Homo sapiens

<400> 83
ccacaggggc ccccagggca ccagggatcc ctggagccaa gggcgagctt ggattgcccg 60
gtgccccagg aatcgatgga gagaagggcc ccaaaggaca gaaaggagac ccaggagagc 120
ctgggccagc aggactcaaa ggggaagcag gcgagatggg cttgtccggc ctcccgggcg 180
ctgacggcct caagggggag aagggggagt cggcatctga cagcctacag gagagcctgg 240
ctcagctcat agtggagcca gggccccctg gcccccctgg ccccccaggc ccgatgggcc 300
tccagggaat ccagggtccc aagggcttgg atggagcaaa gggagagaag ggtgcgtcgg 360
gtgagagagg ccccagcggc ctgcctgggc cagttggccc accgggcctt attgggctgc 420
caggaaccaa aggagagaag ggcagacccg gggagccagg actagatggt ttccctggac 480
cccgaggaga gaaaggtgat cggagcgagc gtggagagaa gggagaacga ggggtccccg 540
gccggaaagg agtgaagggc cagaagggcg agccgggacc accaggcctg gaccagccgt 600
gtcccgtggg ccccgacggg ctgcctgtgc ctggctgctg gcataagtga cccacaggcc 660
cagctcacac ctgtacagat ccgtgtggac atttttaatt tttgtaaaaa caaaacagta 720
atatattgat cttttttcat ggaatgcgct acctgtggcc ttttaacatt caagagtatg 780
cccacccagc cccaaagcca ccggcatgtg aagctgccgg aaagtggaca ggccagacca 840
gggagatgtg tacctgaggg gcacccttgg gcctgggctt tcccaggaag gagatgaagg 900
tagaagcacc tggctcgggc aaggctagaa agatgctacg ttgggccttc agtcacctga 960
tcagcagaga gactctcagc tgtggtactg ccctgtaaga acctgctccc gcaaaactct 1020
ggagtccctg ggacacaccc tatccaagaa gacccagggg tggaacagcg gctgctgttg 1080
ctcctggcct catcagcctc caaactcaac cacaaccagc tgcctctgca gttggacaag 1140
acttggcccc cggacaagac tcgcccagca cttgcggctg ggcccgggga gcagtgagtg 1200
gaaatccccc acgagggtct agctctacca cattcaggag gcctcaggag gccagcctgc 1260
catgagagca catgtcctct ggccaggagt agtggctgag ctctgtgatc gctgtgatgt 1320

ggacccagct ccagggagca gagtgtcggg gatggagggg cccagcctgg actgactgct 1380
acttcctgtc tctgtttcca ttatcaccca gagagggaca agataggaca tggcctggac 1440
cagggaggca ggcctcccac tcagagtctg ggtctcactg gccccaagtc tcccacccag 1500
aactctggcc aaaaatggct ctctaggtgg gctgtgcagg caaagcaaag ctcagggctg 1560
gttcccagct ggcctgagca gggggcctgc caccagaccc acccacgctc tgacgagagg 1620
cttttccacc tccagcaagt gttcccagca accagctcca tcctggctgc ttgccttcca 1680
tttccgtgta gatggagatc actgtgtgta ataaaccaca agtgcgtgaa aaaaaaaaaa 1740
aaaaaaaaaa aaaaaaaaaa aaaaaaaaaa aaaaaaaaaa ag 1782

<210> 84
<211> 539
<212> DNA
<213> Homo sapiens

<400> 84
tccctgaagt ggctgctatc caggatgact gtgttgctgc agtctttgag tggttggagc 60
gctgtaatgt ctatgttaat agctgggaaa tcgcagtagc agcaaccaga ccactccaat 120
ggaacaatcc aagttggggt aagtttgatt gagcaaaaaa gtcatgctga ctgtgtgatg 180
aagaggcaga actggtaaca gtgacaggcc ctgcagtcag cagctgtgga ttttatgtgc 240
agggcagact gatctggaaa atgggaagag ctgactgcca ataacacctt ggggggaggt 300
ttgttcagtg catctcccta ctccttgtat acctctgatg acatgcagaa gcttaggaaa 360
taaaacacac ttgtaacatc acagggtgca agtaagtaca tgtggatgcc agtgtagaga 420
gaagcgaaag ctagttttca ttgcaagaat ccaaacagag taacagggct ttgtccactg 480
tctccagtcc atggttccct ggtgttccta gagtcctatt aaaaaaaaaa aaaaaaaaa 539

<210> 85
<211> 1791
<212> DNA
<213> Homo sapiens

<400> 85
ggcagcctgg gagttggacg tggctcaggc agtgggtaga aaggggcagc cagccacagc 60
ccgagggtct cactgtgtca tccagagctg gagtgcagcg gcacagtcat ggctcactgg 120
aactcaggct caagcaatcc tcccgcctca gccttccaag taactaggac tacaggcatg 180
tgccaccacg cctggtgtct gagatgctgc caccgcagaa gaagccctgg gagtccatgg 240
ctaaggggct ggtgctgggc gcgctcttca ctagtttcct gctgctggtg tactcctatg 300
ccgtgccccc gctgcatgcc ggcctggcct ccacgacccc ggaggccgca gcgtcctgct 360
ctccacctgc actcgagcca gaggcagtga tccgggccaa cggctcggcg ggggagtgcc 420
agccgcggcg caacatcgtg ttcttgaaga cgcacaagac ggccagcagc accctgctca 480
acatcctgtt ccgcttcggc cagaagcacc ggctcaagtt cgccttccct aacggccgca 540
atgacttcga ctacccgacc ttcttcgccc gcagcctggt gcaggactat cggcccgggg 600
cctgcttcaa catcatctgc aaccacatgc gcttccacta cgacgaggtg cgcggcctgg 660
tgccgaccaa cgccatcttc atcacggtgc tccgcgaccc cgcccgcttg ttcgagtcct 720
ccttccacta cttcgggccg gtggtgcccc tcacgtggaa gctctcggcc ggcgacaagc 780
tgaccgagtt cctgcaagac ccggatcgct actacgaccc caacggcttc aatgcccact 840
acctccgaaa cctgctcttc ttcgacctgg gctatgacaa cagcctggac cccagcagcc 900
cgcaggtgca ggagcacatc ctggaggtgg agcgtcgctt ccacctggtg ctccttcaag 960
agtacttcga cgagtcgctg gtgctgctga aggacctgct gtgctgggag ctggaggacg 1020
tgctctactt caagctcaac gcccgccgcg actcgcccgt gccgcggctc tcgggggagc 1080
tgtatggcgc gccaccgcc tggaacatgc tggactccca cctctaccgc cacttcaacg 1140
ccagcttctg gcgcaaggtg gaggccttcg ggcgggagcg catggcccgc gaggtggccg 1200
ccctgcgcca tgccaacgag cgcatgcgga ccatctgcat cgacgggggc cacgccgtgg 1260
acgccgccgc catccaggac gaggccatgc agccctggca gccgctgggc accaagtcca 1320
tcctgggcta caacctcaag aagagcatcg ggcagcggca cgcgcagctc tgccggcgca 1380
tgctcacgcc cgagatccag tacctgatgg acctcggcgc caacctgtgg gtcaccaagc 1440
tctggaagtt cattcgcgat ttcctgcggt ggtgacgtcc caccgcccag cggcttgcct 1500
gcctgctcgc tccctgcaga ggggctgagc aggacgccgc tggtgctggc cgcccccagc 1560
cccctcctgg tgccacctca gaccccgggg tgaggggggg ctccctgggg ggaggcagcc 1620
agccaagact gggcccatga acacagagag ggcctaaccg agatcagtat ttaactaatt 1680
ataccagttt ttattaaacc cctttccctc cccgataaag aatgttctat ttctgcctcc 1740
ccttaaaggg gagacctcag aagtaaagga atttgatgtt gtgtttttgt t 1791

<210> 86

<211> 4975
<212> DNA
<213> Homo sapiens

<400> 86
ctgcacaggg agtttgtctt gtgcaaacaa tttccaaggc agcgttttct tccctgcctg 60
ggagtgcagg gctcagcgcc ttcactttgg aactgactca gagacctaaa gaagcccacc 120
tggccagcgg gaaggggggc cgccgccgcc tcccggtttt gggcagccct ggccagctcc 180
ctgtggcctt ggaggacttc caccgggcag gcgttcccat gatgccaggc taccaggcgc 240
gggggattcc tgcaggccgg cgctgctttt cttagaaccc cctttctaga aaagtacacc 300
tggaggtttt gcttcaaaga gaggagaggc agcagcatgg cgagtgtcct gtcccgacgc 360
cttggaaagc ggtccctcct gggagcccgg gtgttgggac ccagtgcctc ggaggggccc 420
tcggctgccc caccctcgga gccactgcta gaaggggccg ctccccagcc tttcaccacc 480
tctgatgaca ccccctgcca ggagcagccc aaggaagtcc ttaaggctcc cagcacctcg 540
ggccttcagc aggtggcctt tcagcctggg cagaaggttt atgtgtggta cgggggtcaa 600
gagtgcacag gactggtgga gcagcacagc tggatggagg gtcaggtgac cgtctggctg 660
ctggagcaga agctgcaggt ctgctgcagg gtggaggagg tgtggctggc agagctgcag 720
ggcccctgtc cccaggcacc acccctggag cccggagccc aggccctggc ctacaggccc 780
gtctccagga acatcgatgt cccaaagagg aagtcggacg cagtggaaat ggatgagatg 840
atggcggcca tggtgctgac gtccctgtcc tgcagccctg ttgtacagag tcctcccggg 900
accgaggcca acttctctgc ttcccgtgcg gcctgcgacc catggaagga gagtggtgac 960
atctcggaca gcggcagcag cactaccagc ggtcactgga gtgggagcag tggtgtctcc 1020
accccctcgc ccccccaccc ccaggccagc cccaagtatt tgggggatgc ttttggttct 1080
ccccaaactg atcatggctt tgagaccgat cctgaccctt tcctgctgga cgaaccagct 1140
ccacgaaaaa gaaagaactc tgtgaaggtg atgtacaagt gcctgtggcc aaactgtggc 1200
aaagttctgc gctccattgt gggcatcaaa cgacacgtca aagccctcca tctgggggac 1260
acagtggact ctgatcagtt caagcgggag gaggatttct actacacaga ggtgcagctg 1320
aaggaggaat ctgctgctgc tgctgctgct gctgccgcag gcaccccagt ccctgggact 1380
cccacctccg agccagctcc cacccccagc atgactggcc tgcctctgtc tgctcttcca 1440
ccacctctgc acaaagccca gtcctccggc ccagaacatc ctggcccgga gtcctccctg 1500
ccctcagggg ctctcagcaa gtcagctcct gggtccttct ggcacattca ggcagatcat 1560
gcataccagg ctctgccatc cttccagatc ccagtctcac cacacatcta caccagtgtc 1620
agctgggctg ctgccccctc cgccgcctgc tctctctctc cggtccggag ccggtcgcta 1680
agcttcagcg agccccagca gccagcacct gcgatgaaat ctcatctgat cgtcacttct 1740
ccaccccggg cccagagtgg tgccaggaaa gcccgagggg aggctaagaa gtgccgcaag 1800
gtgtatggca tcgagcaccg ggaccagtgg tgcacggcct gccggtggaa gaaggcctgc 1860
cagcgctttc tggactgagc tgtgctgcag gttctactct gttcctggcc ctgccggcag 1920
ccactgacaa gaggccagtg tgtcaccagc cctcagcaga aaccgaaaga gaaagaacgg 1980
aaacacggag tttgggctct gttggctaag gtgtaacact taaagcaatt ttctcccatt 2040
gtgcgaacat ttttattttt aaaaaaaaga aacaaaaata tttttccccc taaaatagga 2100
gagagccaaa actgaccaag gctattcagc agtgaaccag tgaccaaaga attaattacc 2160
ctccgtttcc cacatcccca ctctctaggg gattagcttg tgcgtgtcaa aagaaggaac 2220
agctcgttct gcttcctgct gagtcggtga attctttgct ttctaaactc ttccagaaag 2280
gactgtgagc aagatgaatt tacttttctt aaaaaaaaaa aaaaaaaaaa aaaaaaagag 2340
tttctggctg atgggtgact cagagtgcag gactgcctgg ccgtggggca gaggggtttg 2400
cccttctcgg agggtacctc ctgttccctg tctgagcatc ctgcatggaa gtcaaaggaa 2460
atccctttct tggtgacgac ttaaatctgg gttccctcag acattgggtt gcaccccaac 2520
aaatattaaa tggcttcttc ttaaagccca gagaaagagg ttttttaaaa gactgtcgcc 2580
aaatagctga gccaaaaggc tgatcagaat tcactttttg gaatgtggca gttaaacact 2640
accttgatca ttctctcctc tttcctcgag gaactcctgg agggtttgag cgtctggaaa 2700
ctctctgctc tgacccgagg aagcaccctc ctgacgccgc cttcctccgg ttattgaaag 2760
gacgcctcag aaatgctttg ttttctttta cgatgtattc agaagccttt actgattaaa 2820
gttttctttt atttgggtgg ccgggagaga cccagggagg ttctggaggt tcctttctgt 2880
ctcctggccc caccagggat ttccccattt ctgtttgctg cctgaaagca ggatgaggaa 2940
ggccaaggag agtccttgca cccgtgagcg tcaggatgag gaaatgacag gaggaagacg 3000
tgggtttggg ttagtggctg ctggcgtttt ggcccttggt gtttctggag cctccaggga 3060
tctaggggag cctgggctgc gtgcatgtcg ataagcagag ctgttcttgg ggagaaggag 3120
ggaggtctcg ggagtgtagc accatgccaa ccagccctgc gcgaagacag agtgagccac 3180
gcccggatgg cagggcatgt ttctgttttg gtgtctcact ttcctcccag cgtgacttat 3240
ttggggattc ctcagggcct actggaatgt gactgcccac tgcccagctg cctcgggtac 3300
aagtcctggc cctatgtccc agctgtcagg ggctcaggga atcctaccca gccacctgtc 3360
ctgggatgga gtgtcagcat ccaccccttg gttgtcatcg aggccgccct cccagtcctg 3420
ggtgaagata tttgggccac cagggctccc ttggcccctt cacgtaggaa atagacacgt 3480

gctttttaat gcaggacact ttgagtgtta caaaatctgt agacctggca gtagggtcat 3540
gatgttggga agggtgtagt gccctaggtt ggtgacagaa gggacagaca cttgtgcaca 3600
ggtgtctttg gtgatggggt tttttttttt ataacttagt aaaaaaaaaa aaatgtatgt 3660
ggaattctgt ctcttggtaa agctcaaagc caggctagcc tgaggtggcg cagggctctc 3720
cttcctgtcc cttcgatctc cttgagaatt aagagctggc agctgctgat ggtgtttccc 3780
aacccccctc acttcccaag acaaccccca gcttcaggtc ctcatgggga ggggagggca 3840
cgttcttgac acatgggaac ttcgctcagg agggcctccc cttcccctct ccctcagagt 3900
tttcactgcc gtctcgtctt tagaaagctg tttgaattcc ccccgccccc agtttggacc 3960
gtgtagatat aactggatat acggattttt ctctttgtgc aggcttctta tgccgttggt 4020
atacagggca ggaaagagag gaataaaggg agagagcagt gtggaaacca cggtggtttt 4080
gctttgttct tactaggttt tggtgccacc ttccctgcct gcgcttgtgc cccctctcct 4140
ccttggcact ggcggcctcc ttgcctccct tccacccgtg ctgccatccc gtgcctgtcg 4200
tgttggttct tcacacgtgc tctgttctcg gggttgttcc attcatgcct tcttggaggg 4260
tgagggtggc ttgggaaccg acccagtgat catgcctact ttcttctttg tatctccctc 4320
cttcccagcc cacccgggca gcagactctg atggaaggaa ggtgccgtag gtgggctttt 4380
agaaactaac gggactggtt ttcaaagcag ttatcttggg aaactgttta ttccagcgat 4440
gtgacttttt tcagaatatt tcttggaatc atattcagag tctggggctg tgtgttgagc 4500
agccttaagg atgctagaca ctcatttagt gcccagggag tccagcgaat gacgtctgtg 4560
gccaagcgag gtctcaggtg caaagcaaaa ggaccattta aagtaaaata gcttggattc 4620
aatcatgtga cttttaaatt ggctcagaaa gcaattttgt aatttcagag agtgttttga 4680
gccatggcca cgttgtcatt gtgagtctat agcttgactc cttggagaac aatattcatt 4740
tggttgtgga gactgatttg ctgggagaaa tctgtcctgt tactttctgg tcatcccagg 4800
ttctgacttt taccaggggc aaaaaaaaaa aaagcaagag ggagataaat cccatctgtg 4860
agtttgtctt attggcgcct ttttcctcag ctgtcttcca agtattattt ttactgttaa 4920
aaaatttttt aaaaatgtga aatgtaatgt ttttacagca acaatatgaa atata 4975

<210> 87
<211> 702
<212> DNA
<213> Homo sapiens

<400> 87
agtgtgaagg gaaggctaag gacgcatcgg tgctggagag ttgagaatat gccttcccct 60
ccacccccaa cccagtcacc ttatttgaaa aaaaattaaa aacacctttt tatttaaaaa 120
cctaaccctg gaagtttgct cgggaaggca ttctcttcct cttcctccag ctgcctgaaa 180
atcatatgcg tggtttttcc tgtttcctgc tctgtagctg acatcagggg agcaacgtga 240
acacaggaaa atggaaatca acatttgaaa cttttaaatg gaagctttat tggggttcca 300
catatggaaa actgcaaata gcagaagtat gtgactcaat aaattattct ggagtgacca 360
catcctctaa ctgcctccca ggacaagacg gagaatgtga acaatgctcc tgaaggcccc 420
tttccctcaa agaatcactg caattgaagg aaaaatgctc catgtcccct ctgccagttg 480
gtgtttatca aaggacaacg gaggaagaga gacaaagtgc ctggatgtgt gtgttaatca 540
agagttgtca ggaccctgcc tctaaaagaa agcgctaaaa tgatgctttt gacaagactt 600
ttctcaaagt ggatgcagtc taagggttgg aaaacaaggc ccaatcaggc taggtctgac 660
ttgtcgcctg cttttgtaaa taaagtttta ttggctcaca ga 702

<210> 88
<211> 674
<212> DNA
<213> Homo sapiens

<400> 88
agtgtgaagg gaaggctaag gacgcatcgg tgctggagag ttgagaatat gccttcccct 60
ccacccccaa cccagtcacc ttatttgaaa aaaaattaaa aacacctttt tatttaaaaa 120
cctaaccctg gaagtttgct cgctgcctga aaatcatatg cgtggttttt cctgtttcct 180
gctctgtagc tgacatcagg ggagcaacgt gaacacagga aaatggaaat caacatttga 240
aacttttaaa tggaagcttt attggggttc cacatatgga aaactgcaaa tagcagaagt 300
atgtgactca ataaattatt ctggagtgac cacatcctct aactgcctcc caggacaaga 360
cggagaatgt gaacaatgct cctgaaggcc cctttccctc aaagaatcac tgcaattgaa 420
ggaaaaatgc tccatgtccc ctctgccagt tggtgtttat caaaggacaa cggaggaaga 480
gagacaaagt gcctggatgt gtgtgttaat caagagttgt caggaccctg cctctaaaag 540
aaagcgctaa aatgatgctt ttgacaagac ttttctcaaa gtggatgcag tctaagggtt 600
ggaaaacaag gcccaatcag gctaggtctg acttgtcgcc tgcttttgta aataaagttt 660
tattggctca caga 674

<210> 89
<211> 627
<212> DNA
<213> Homo sapiens

<400> 89
agtgtgaagg gaaggctaag gacgcatcgg tgctggagag ttgagaatat gccttcccct 60
ccacccccaa cccagtcacc ttatttgaaa aaaaattaaa aaacaccttt tatttaaaaa 120
cctaaccctg gaagtttgct cgctgacatc aggggagcaa cgtgaacaca ggaaaatgga 180
aatcaacatt tgaaactttt aaatggaagc tttattgggg ttccacatat ggaaaactgc 240
aaatagcaga agtatgtgac tcaataaatt attctggagt gaccacatcc tctaactgcc 300
tcccaggaca agacggagaa tgtgaacaat gctcctgaag gcccctttcc ctcaaagaat 360
cactgcaatt gaaggaaaaa tgctccatgt cccctctgcc agttggtgtt tatcaaagga 420
caacggagga agagagacaa agtgcctgga tgtgtgtgtt aatcaagagt tgtcaggacc 480
ctgcctctaa aagaaagcgc taaaatgatg cttttgacaa gacttttctc aaagtggatg 540
cagtctaagg gttggaaaac aaggcccaat caggctaggt ctgacttgtc gcctgctttt 600
gtaaataaag ttttattggc tcacaga 627

<210> 90
<211> 4018
<212> DNA
<213> Homo sapiens

<400> 90
ggcgcgccag gtaaggcagg cggacgcgcg gacccctcgg cgcagtgcgg ccccgaaggc 60
ggtagggcga gaaagagctg ggaccccccc gcaacttcgg gatcggctgg gtgaggctgg 120
gcaggcctga tgctcagggg ttcgtgccgg gaagttcagg tcctcggaca acctctctcc 180
agctctccgc cgccggtacc cacggcccag tctccacctg gggaaacccc cttggcgtgg 240
cttgtttcgt tacaagttat cctggtagag tgggcatgaa ggcctcggag gcaactgtgc 300
ctgcaggtgt ccatcacctt caaggacttg gccgtgcggt tctcggagga ggagtggcgg 360
ctcctggagg aggggcagag ggagttctac cgagacgtga tgcgggagaa ctacgagacg 420
ctggtctctg tggggacagc tgagctgctc cccctctctg ctttcctgtc accctcagag 480
cctggaagag ctgttggggg agggagccac gctgatgagg ggcaggagcc tgctggttgt 540
ggagatcccc aggggggaca gccccggcac agcctgcacc tcacagccct ggtgcagctg 600
gtgaaggaga tcccagagtt cttgtttgga gaagtcaagg gcgctatgga cagccccgag 660
agcgagagcc ggggagccag cctggatgga gagagagcga gccccgaggc agctgcggca 720
agagagccct gccctctccg aggcctgctc agctgccttc cagacggccc taccagccag 780
ccccacctgg ccaccacgcc caccgacagc tcgtgttcca gtggcccaac tggtgacggg 840
gtccagggaa gtcctctccc catcaaaact gccgacaaac cgtggcctac aaggaaggaa 900
ggcccaggag ccctgggcgg ggagcccagc cctcccaccc atagccccag caggaggaag 960
agccacagag gacaggagag agggacctca gaggccggaa tttctcctgg gaacagcccc 1020
ttgcaaggcc tcatcaactg tctgaaggaa atccttgtgc ctgggccccg gcaccccgag 1080
acatccccaa gcttcttgcc acctctccct agcctgggca cgtccaggct aaccagagca 1140
gacctggggc ctgggagccc gccctgggca gtgaagaccg aggcggtttc aggggattgt 1200
cccctccagg gtctgctaca ctgtctgaag gagctccccg aggcccagga caggcatccc 1260
agtccctcag gagtggggaa ccgacggcta caggagaatc caggagcctg gaaaaggggt 1320
tctggagggc ctggatacct cctgacccct cctccccatc ctgatcttgg agctggcggt 1380
ctgctctctg tgaagatgga gaacagctgg gtccagagcc ccccaggacc tgcatcctgt 1440
cagcctggca ggcagcccct cagtccctca gccactggag acaccagagg ggtcccccaa 1500
cccagctggg gccctgaggc tcaagctgcc agtgcctcaa gctcaccgct ggaagccctg 1560
gaagcctgtc tgaagggcat tcccccaaat gggtcgtcac cttcccagct gccacccact 1620
tcttgttccc agaaccccca gccaggagac tctaggtctc agaagcctga actgcaaccc 1680
cacagatcac acagtgaaga agcgaccaga gagcctgttc tgcctctggg tctgcagagc 1740
tgtgtgagag atggccccag caggcccctg gccccccgag gaacccccac cagcttctcc 1800
tcatccagca gcaccgactg ggacctggat tttgggagtc ctgtggggaa ccaggggcag 1860
catcctggaa aaggaagccc accaggaagc tccccactgc agggtctgga gaattgtctc 1920
aaggagatac ctgtgcctgt gctgcggcct gcctggccct gctcctcagc agcagacagg 1980
ggaccgagga gagcagagcc caggaactgg acagcagaca aggaaggact gagggctgag 2040
gcctgcgagt cagcccgtct cgggcagggt aggggagaag cgcccacccg gagcctccat 2100
ctggtcagcc cacaggtgtt cacctccagc tgcgtccccg cctgccacca gcgggggttc 2160
aaagaccctg gggccaccag gccaggagtg tggaggtggc tcccagaggg gtctgccccc 2220
aagccctccc cgctgcactg cctggagagc gccctgaggg ggatcttgcc tgtaaggccc 2280

ttgcgcttcg cctgcgtggg aggccccagc cccagcccca gccccggctc cagctcgagc 2340
ttcagcggct ctgaaggaga agacccgagg ccagagcctg agctctggaa gccgctcccc 2400
caggagaggg accgccttcc cagctgtaag cctcctgtcc ctctgtcccc atgtcctggt 2460
gggacccctg ctggcagcag tggcggcagc cctggtgaag accccaggag aacagagccc 2520
aggtactgca gcggcctcgg tgcaggtaca gctcaggatc cctgcccggt ttctcagctg 2580
gagaaaaggc ccagggttag tgaagcatcc agaggcctgg agcttggaca tggaagaccc 2640
agagttgcag ccaagaccca tgagaggctg ctcccccagg gcccgcctga gctgcccagt 2700
gagtctcccc ctccggagct gccccctccg gaagctgcgc ctcctgtgtt gccagcctcc 2760
tccctgcagc cgccatgcca ctgtgggaag cccctgcagc aggagctgca cagcctcggt 2820
gctgcccttg cggagaagct ggatcggctc gccacagcgc tggcaggcct ggctcaggaa 2880
gtggccacca tgaggaccca ggtgaatcgg ctggggaggc gcccccaagg ccctgggcca 2940
atgggccaag cttcctggat gtggaccctc ccacggggac ctcgctgggc tcatggccct 3000
ggtcacagac atctgcccta ctggaggcag aagggaccca cgaggcctaa accaaagatc 3060
ctgcgtggcc agggagagag ctgcagggct ggtgacctgc aaggactctc cagagggacc 3120
gctcgccggg cacgtccgct gcctccagac gctcccccgg cagaacctcc tgggctccac 3180
tgcagctctt cccagcagct gctgtcctct acacccagct gccatgctgc gccgcctgca 3240
caccccctcc tcgcacatac cgggggccac cagagccccc ttcccccttt agtgcctgct 3300
gccttacccc tgcagggagc ctctcctcct gcagccagtg cagatgcaga cgtgccgacc 3360
tcaggagtgg caccagacgg gatcccagag cggcccaagg agccgagcag cctgctggga 3420
ggagtgcaga gggccctcca ggaagaactg tggggtgggg agcacaggga cccgagatgg 3480
ggggcgcatt gatggcattc ctcttctcca catctgctcg ttcttgccga gggtgcagtg 3540
gtggcgtgga agccctgtca ccccacccca ggccaccctc tcccagagga cgccatctcc 3600
cttactgttg ctgggagcct cgccctttgt cccaactggg tagagccccc aggtgctgtt 3660
tgctcaggag gctgctgtgg gggtgccttc ctcagcctct ggccctcttg gctcagattc 3720
aatcaaatgt tgcttccctc tcctgtcttt cccactggag ccgcccaagc ttgtaggtgg 3780
gtggtgtgca taggccacgt gtgccccaca tatgcagggg gtgccccaca cagctagagc 3840
ggccaggaga gcgcctccta accaccagcc gttcctgatc tcaggagcct tgaagggctg 3900
ggctcttgcc ttcctggagt aaatattggc acagatttca tttgagagaa ctcagccccc 3960
tggtctaagc tggacttacc tctgtggatt ctgaaattaa agaagtgagt tgctaagg 4018

<210> 91
<211> 3464
<212> DNA
<213> Homo sapiens

<400> 91
agcctgctgg ttgtggagat ccccaggggg gacagccccg gcacagcctg cacctcacag 60
ccctggtgca gctggtgaag gagatcccag agttcttgtt tggagaagtc aagggcgcta 120
tggacagccc cgagagcgag agccggggag ccagcctgga tggagagaga gcgagccccg 180
aggctgcggc aagagagccc tgccctctcc gaggcctgct cagctgcctt ccagacggcc 240
ctaccagcca gccccacctg gccaccacgc ccaccgacag ctcgtgttcc agtggcccaa 300
ctggtgacgg ggtccaggga agtcctctcc ccatcaaaac tgccgacaaa ccgtggccta 360
caaggaagga aggcccagga gccctgggcg gggagcccag ccctcccacc catagcccca 420
gcaggaggaa gagccacaga ggacaggaga gagggacctc agaggccgga atttctcctg 480
ggaacagccc cttgcaaggc ctcatcaact gtctgaagga aatccttgtg cctgggcccc 540
ggcaccccga gacatcccca agcttcttgc cacctctccc tagcctgggc acgtccaggc 600
taaccagagc agacctgggg cctgggagcc cgccctgggc agtgaagacc gaggcggttt 660
caggggattg tcccctccag ggtctgctac actgtctgaa ggagctcccc gaggcccagg 720
acaggcatcc cagtccctca ggagtgggga accgacggct acaggagaat ccaggagcct 780
ggaaaagggg ttctggaggg cctggatacc tcctgacccc tcctccccat cctgatcttg 840
gagctggcgg tctgctctct gtgaagatgg agaacagctg ggtccagagc cccccaggac 900
ctgcatcctg tcagcctggc aggcagcccc tcagtccctc agccactgga gacaccagag 960
gggtccccca acccagctgg ggccctgagg ctcaagctgc cagtgcctca agctcaccgc 1020
tggaagccct ggaagcctgt ctgaagggca ttcccccaaa tgggtcgtca ccttcccagc 1080
tgccacccac ttcttgttcc cagaaccccc agccaggaga ctctaggtct cagaagcctg 1140
aactgcaacc ccacagatca cacagtgaag aagcgaccag agagcctgtt ctgcctctgg 1200
gtctgcagag ctgtgtgaga gatggcccca gcaggcccct ggccccccga ggaaccccca 1260
ccagcttctc ctcatccagc agcaccgact gggacctgga ttttgggagt cctgtgggga 1320
accaggggca gcatcctgga aaaggaagcc caccaggaag ctccccactg cagggtctgg 1380
agaattgtct caaggagata cctgtgcctg tgctgcggcc tgcctggccc tgctcctcag 1440
cagcagacag gggaccgagg agagcagagc ccaggaactg gacagcagac aaggaagggt 1500
ctgcccccaa gccctccccg ctgcactgcc tggagagcgc cctgaggggg atcttgcctg 1560
taaggccctt gcgcttcgcc tgcgtgggag gccccagccc cagccccagc cccggctcca 1620

gctcgagctt cagcggctct gaaggagaag acccgaggcc agagcctgat ctctggaagc 1680
cgctccccca ggagagggac cgccttccca gctgtaagcc tcctgtccct ctgtccccat 1740
gtcctggtgg gacccctgct ggcagcagtg gcggcagccc tggtgaagac cccaggagaa 1800
cagagcccag gtactgcagc ggcctcggtg caggtgagcc tggggtctcc tgagggggcg 1860
cccatgcgtt ccccagagga ccaggagtcc agactggatc ctttgagtcc tgcctgtcgc 1920
tttgcctggc tcttgccagg gagtcctgca cattggccgt gctttagttc cctgtaaggt 1980
acagctcagg atccctgccc ggtttctcag ctggagaaaa ggcccagggt tagtgaagca 2040
tccagaggcc tggagcttgg acatggaaga cccagagttg cagccaagac ccatgagagg 2100
ctgctccccc agggcccgcc tgagctgccc agtgagtctc cccctccgga gctgccccct 2160
ccggaagctg cgcctcctgt gttgccagcc tcctccctgc agccgccatg ccactgtggg 2220
aagcccctgc agcaggagct gcacagcctc ggtgctgccc ttgcggagaa gctggatcgg 2280
ctcgccacag cgctggcagg cctggctcag gaagtggcca ccatgaggac ccaggtgaat 2340
cggctgggga ggcgccccca aggccctggg ccaatgggcc aagcttcctg gatgtggacc 2400
ctcccacggg gacctcgctg ggctcatggc cctggtcaca gacatctgcc ctactggagg 2460
cagaagggac ccacgaggcc taaaccaaag atcctgcgtg gccagggaga gagctgcagg 2520
gctggtgacc tgcaaggact ctccagaggg accgctcgcc gggcacgtcc gctgcctcca 2580
gacgctcccc cggcagaacc tcctgggctc cactgcagct cttcccagca gctgctgtcc 2640
tctacaccca gctgccatgc tgcgccgcct gcacaccccc tcctcgcaca taccgggggc 2700
caccagagcc cccttccccc tttagtgcct gctgccttac ccctgcaggg agcctctcct 2760
cctgcagcca gtgcagatgc agacgtgccg acctcaggag tggcaccaga cgggatccca 2820
gagcggccca aggagccgag cagcctgctg ggaggagtgc agagggccct ccaggaagaa 2880
ctgtggggtg ggagcacagg ggacccgaga tgggggcgc attgatggca ttcctcttct 2940
ccacatctgc tcgttcttgc cgagggtgca gtggtggcgt ggaagccctg tcaccccacc 3000
ccaggccacc ctctcccaga ggacgccatc tcccttactg ttgctgggag cctcgccctt 3060
tgtcccaact gggtagagcc cccaggtgct gtttgctcag gaggctgctg tgggggtgct 3120
ttcctcagcc tctggccctc ttggctcaga ttcaatcaaa tgttgcttcc ctctcctgtc 3180
tttcccactg gagccgccca agcttgtagg tgggtggtgt gcataggcca cgtgtgcccc 3240
acatatgcag ggggtgcccc acacagctag agcggccagg agagcgcctc ctaaccacca 3300
gccgttcctg atctcaggag ccttgaaggg ctgggctctt gccttcctgg agtaaatatt 3360
ggcacagatt tcatttgaga gaactcagcc ccctggtcta agctggactt acctctgtgg 3420
attctgaaat taaagaagtg agttgccaaa aaaaaaaaaa aaaa 3464

<210> 92
<211> 2800
<212> DNA
<213> Homo sapiens

<400> 92
cggacgcgtg ggcggacgcg tgggcggctg aggagtgggg tgatgatgcc actggcagaa 60
gcaggcgccc tggcccaagg aggaggccct tcagcaacgg agtgggcctg cattctgcgg 120
aggaagaccc ccaggcacaa gcagcccacc ttactgatgg tcagagcatc cagaagaagt 180
ggcaagactt cagctgtcct caaagcagga agacagagtg tttctggcag aaagaacagc 240
acaagcaaag atctggtaac cctaggtgcc tccagtttga gggaggagag aggacacccc 300
ctccacccca gacataggaa agcagtccac ctgcgcacca ggggcaggac acgtggctgg 360
gtgcagacgc tggcccggat gtcgcggagg actcgcgggc cggtagagcg cgcggcggcg 420
gcggcggcgg cggcggcggg aggagacgca ggtcacgccc ccttcccacc acctcccgcc 480
gccgacgggg cgcgcgcgcc gaggagcccg ggacaggtga ctcctagagg actgcgtctg 540
cgcctccccc ggcgggagtc ccttcttcgc ggcctctgcc gccccctgcg tcccctcctg 600
ggcttccgag agtctgactc agccaagccg gcatcgcttc gcctcctaca acacaccccg 660
agcgcgagaa gaaattacag gattgcaggg gcacggctaa tgcgctctaa ttacccaccg 720
ccgctgtcat ccgcggcgct ccgcggcgct gggccaacgc gccgtaatta agacgccgct 780
ccccggtccc ggaaccctcc cttcgccacc ctccacccac ccacctcgcg gtccccagga 840
ccactggctg ccaactcctg ccgccctctg ggactgcccc tcagtcccag gagagctatg 900
aggcctcact gggggcagaa cttggggaga ccgaagccgg cccttccact cccctaaacc 960
tacagtcctg gaaggtctct gctggaagag actgggagag tggggagggg ccagggacct 1020
atttcgttga cagaaacagt gaggtctatt ggaaagttag ccttcagtgg gaggagtggc 1080
aaaggcacgc agggcagcag tacccccgtt tggcacttga ggcacggaga agggaaggga 1140
agtccgccca gccagaactc ccagacccgc tcgtttaaca gtggctttat gagcagcacg 1200
acggactccc tcccctggcg gggaaattac cttcctgcga agaaaggtga catccccgcc 1260
acagacacgg aggccccgcc tgctggccag ctgtggccgc cataggggag ctggtgagga 1320
gtgaggtggc tctggcctcc catgtcaggt agggtgagag aggccctgaa gatagaagcc 1380
aggcccgaag gccagaatta aaggggtagc ttggccatca gctccttccc ccaaaaccag 1440
gctcagagct ctcctctatc agcaattctt tcctgaggtc cagggtgttg caaactcatg 1500

gccttgggcc acgttttacc ttcagtgagg ttttgtttgg cttgcaatga gtacatgggt 1560
tttaggttga atgtgagagt ctgcagacaa tgtgggttct ccgactgccc tcccagtgaa 1620
ggtggacctc tgatttagac cggcccgctg ggcccgtgcc tacatccttt tcctcagcgt 1680
caggatctta gtccccatga ttgctgcccc tttgtggtct gcatgtttct tggtaagggt 1740
gcccagtcaa gtgtctctgg gagccccctc ttctctgtca tcgtcaggct ccccacttgc 1800
agtggccgtg gtggctgcac atgctcccgg atacccctcc agcactccct tttgtcatct 1860
tgagtttcag tccgtgactt gggtggcacc ctatcttctc catcagggat ggtagctatt 1920
tttctgccat gagactagag gttgcttgag aacaggaaat tggatctctc ccttcaggct 1980
gggatttcac taagggctgg agaagggggg aatatgggag gattatctcc tatcacaggg 2040
agcgctctga gggcaaggct gtgtctccga ttcagactga cggttccctg aggatggggc 2100
tgtttctccc ctccgactgg ggctccctga ggatggggct gtttctcccc tccgactggg 2160
gctccctgag gacagggctg tgtctcccct cagcctgggg ctccctgagt cagggctgtg 2220
tctcccctca gactggggct ccctgaggac ggggctgtgt ctgcctcaga ctgggaatcc 2280
ctgaagatgg ggccgtgtct ccctcaaagt ggggctccct caggacagcg ttgtgtctcc 2340
cctcagactg cggctccctg aggacggggc tgtgtctccc tcagactggg gctccctgaa 2400
gaaagggctg cttctccctc agactggagc tccctgaaga tggggctgtg tccccctcag 2460
actggggctg cctgaggacg aggctgcatc tcccctcaga ctggagctcc ctgagggcag 2520
ggctgtctcc ccccttcaga ctggggcctc ccccaaagga cagggactgt gtctcccctg 2580
ggactaggtt ctttgtgtct tctgcatcag acagattccc atgaggacag ggctgtctcc 2640
ccctccatct ggagctccct gaaggcaatg gcctctcccc tcaaatggga catttgcacc 2700
ccaccccacc ctctggctcc ccatggcgcc caggcattcc tcccaggaca ccatttgtca 2760
cgctttgatg aatgaaactt actaaaaaaa aaaaaaaaaa 2800

<210> 93
<211> 1040
<212> DNA
<213> Homo sapiens

<400> 93
aacacatttc atctgggctt cttaaatcta aatctttaaa atgactaagt tttcttcctt 60
ttctctgttt ttcctaatag ttggggctta tatgactcat gtgtgtttca atatggaaat 120
tattggaggg aaagaagtgt cacctcattc caggccattt atggcctcca tccagtatgg 180
cggacatcac gtttgtggag gtgttctgat tgatccacag tgggtgctga cagcagccca 240
ctgccaatat cggtttacca aaggccagtc tcccactgtg gttttaggcg cacactctct 300
ctcaaagaat gaggcctcca aacaaacact ggagatcaaa aaatttatac cattctcaag 360
agttacatca gatcctcaat caaatgatat catgctggtt aagcttcaaa cagccgcaaa 420
actcaataaa catgtcaaga tgctccacat aagatccaaa acctctctta gatctggaac 480
caaatgcaag gttactggct ggggagccac cgatccagat tcattaagac cttctgacac 540
cctgcgagaa gtcactgtta ctgtcctaag tcgaaaactt tgcaacagcc aaagttacta 600
caacggcgac ccttttatca ccaaagacat ggtctgtgca ggagatgcca aaggccagaa 660
ggattcctgt aagggtgact cagggggccc cttgatctgt aaaggtgtct tccacgctat 720
agtctctgga ggtcatgaat gtggtgttgc cacaaagcct ggaatctaca ccctgttaac 780
caagaaatac cagacttgga tcaaaagcaa ccttgtcccg cctcatacaa attaagttac 840
aaataatttt attggatgca cttgcttctt ttttcctaat atgctcgcag gttagagttg 900
ggtgtaagta aagcagagca catatggggt ccatttttgc acttgtaagt cattttatta 960
aggaatcaag ttctttttca cttgtatcac tgatgtattt ctaccatgct ggttttattc 1020
taaataaaat ttagaagact 1040

<210> 94
<211> 5375
<212> DNA
<213> Homo sapiens

<400> 94
gaatctgctg agcccccact aacccagagt gataaaagag agacttctca caccacagca 60
gcagcgactg gtcggagttc ccatgctgat gcaagagaat gtgctatttc aacccaggca 120
gagcaagaag caaaaaccct tcaaacttca acagactcag tctccaaaga aggcaacaca 180
aattgcaagg gagaaggcat gcaagttaat actctatttg aaacaagcca ggttccagac 240
tggagtgatc ctcctcaggt acaagttcag gaaacagtca gagagacaat ctcttgcagc 300
cagatgccag ctttctcaga gcctgctggg gaggagtccc cattcactgg gaccacaaca 360
atttccttct caaacttagg aggggtccac aaggaaaatg catcattagc tcaacactcg 420
gaggtcaaac cctgtacctg tggtccacag caggaagaaa aacaagacag agatggcaac 480
atacctgaca atttcaggga agacctaaaa tatgagcaga gcatctcaga agccaatgat 540

gagactatgt ccccaggtgt gttctcaagg catctcccca aggatgctcg tgctgacttc 600
agggagcctg tggctgtctc tgttgcttcc cctgaaccca cagatactgc cctcaccctg 660
gaaaatgtgt gtgatgagcc aagggacaga gaagcagtgt gtgcaatgga gtgttttgag 720
gctagtgacc aaggaacatg ttttgatacc atagattctc ttgttgggac accagttgat 780
aactattcgc ctcaagaaat ttgctctgta gatacggaac tggcagaagg tcaaaacaaa 840
gtatctgatt tatgttcttc taatgacaag acactggaag tcttttttca gacacaagtg 900
tctgagactt cagtgtctac gtgcaaaagc agcaaggacg gcaactcagt catgtcccct 960
ctttttatca gtactttcac cttgaacatt tcacacacag ctagtgaagg tgccacagga 1020
gaaaatctag ccaaggtgga gaaatccacc tacccactgg cctccacagt acatgctggc 1080
caggagcagc caagccccag caactcagga gggcttgatg aaacacagct cctttcttct 1140
gagaacaatc ctttagtgca atttaaagaa ggaggtgaca agagccccag tcctagtgcc 1200
gcagacacca cagccacacc agccagttat agttcaattg tgagttttcc ttgggagaag 1260
ccaacaacat taactgctaa taatgagtgc tttcaagcga ccagagagac tgttaccatt 1320
gccaccgaag tccacccagc caaatacctt gctgtgtcaa ttcctgagga caagcatgca 1380
ggtggcactg aggagaggtt ccctcgtgca tcccatgaaa aggtttccca atttccttcc 1440
caagtgcagg tggatcatat tttaagtggt gctaccatca aatctacaaa agagctactt 1500
tgcagggcac ccagtgtgcc aggagtccca caccatgtcc tgcagctccc agagggagag 1560
ggtttctgca gtaattcccc tcttcaggtt gataacctgt ctggagataa gagccagact 1620
gtggacagag cagactttag gagctatgaa gagaatttcc aagaaagagg aagtgaaaca 1680
aagcaggggg tccagcagca gagcctgtcc cagcagggtt ctctttctgc acctgatttc 1740
caacaaagtt tgcctacgac atctgctgca caagaggaaa gaaacttggt gcccacggcc 1800
ccctcaccag caagctctag ggaaggagca gggcagcgct caggttgggg gacgagggtc 1860
tccgtggtgg ctgaaactgc tggggaagaa gacagtcagg ctctgagcaa cgttccatct 1920
ctctctgata tccttttgga agagtctaaa gaatatagac ctggaaattg ggaggcaggc 1980
aacaagctga agattataac tctagaggct tccgcttctg aaatctggcc accacgacaa 2040
ctgacaaatt ctgagagcaa ggcatcagac ggtggtctca taattcctga caaggtctgg 2100
gctgtacctg atagtctaaa ggcagatgct gttgtgcctg aattggcccc ctctgaaata 2160
gcagcattgg ctcacagtcc agaggatgct gagtcagccc ttgctgatag cagagaaagc 2220
cataaaggcg aagagcccac catcagtgta cactggagaa gtctttcttc ccggggtttc 2280
agccaaccga gactcctgga gtcatccgtg gaccctgtag atgaaaagga gttatctgtc 2340
acagattcac tgtcagcggc ttctgaaact ggagggaagg aaaatgttaa caatgtgagt 2400
caagaccagg aggaaaaaca actcaagatg gatcacactg ccttctttaa aaagtttctg 2460
acctgcccta aaatcctaga gtcctctgta gatcccattg atgagataag tgtgatagag 2520
tacaccaggg ctggaaaacc agagccctct gaaaccacac cacagggcgc cagagaagga 2580
ggtcaatcaa atgacggaaa catgggccac gaagcggaaa tccagtcggc cattttgcaa 2640
gttccatgtc tccagggaac cattctgagt gaaaatagaa tcagcagaag ccaagaaggc 2700
agtatgaagc aggaggcaga acaaattcaa cctgaggagg caaaaactgc catttggcaa 2760
gtcctgcaac ccagcgaagg cggtgaaaga attccaagtg gatgtagcat aggccaaata 2820
caagaaagca gtgatgggag cttaggggag gctgagcaaa gcaaaaagga caaagcagaa 2880
ttgatttccc ccacttcacc tctttctagt tgtcttccaa taatgactca ctcttctctt 2940
ggggttgaca cgcacaactc cacaggccaa attcatgacg tccctgaaaa tgacatagtt 3000
gagcccagaa agcgtcagta tgtgtttcct gtttcacaga aaaggggaac tattgagaat 3060
gagcgtggga aacctttgcc ctcttctcct gatcttacca ggttcccttg tacttcatct 3120
cctgaaggaa atgtcacaga ctttttgata agccacaaaa tggaggaacc taaaatagag 3180
gtgcttcaaa ttggggaaac caaaccccca agctcatcta gctcctcagc gaagaccttg 3240
gcatttattt caggagaacg tgagttagag aaagccccta agttactgca ggatccatgt 3300
caaaaggcca ccctgggctg tgcgaaaaag tccagggaga gagagaagtc cctggaagcc 3360
cgagcaggca aatcgccagg gaccctcaca gcagtgacgg ggtcagagga ggtcaagagg 3420
aagccagaag ccccaggcag tggacattta gctgagggag taaagaagaa aattttgtcc 3480
agggtggcag cactgaggct gaaactggaa gaaaaggaaa atatcagaaa gaactcagcc 3540
tttcttaaaa agatgcccaa actcgaaaca tcattatcac acacagaaga gaaacaagac 3600
ccaaaaaagc catcttgcaa aagagaagga agagctccag tattactgaa aaaaatccaa 3660
gctgagatgt tccctgaaca ctctggaaat gtaaaattaa gctgccaatt tgcagaaatt 3720
catgaagatt ctactatctg ctggacaaaa gattcaaagt ccatagccca agtgcagaga 3780
agtgcagggg acaactccac tgtttccttt gccatcgtgc aagccagtcc gaaggaccag 3840
ggactctatt actgctgcat caagaacagc tacggaaaag tgactgctga atttaacctc 3900
acagctgaag ttctcaaaca gctgtcaagt cgccaggata ctaaaggatg tgaagagatt 3960
gaattcagcc aactcatctt caaagaagac ttcctccatg acagctactt tgggggccgc 4020
ctgcgtggtc agatcgccac ggaggagctg cactttggag aaggggttca ccgcaaagcc 4080
ttccgcagca cagtgatgca cggcctcatg cctgtcttca aacctggcca tgcctgtgtg 4140
cttaaggtgc acaatgccat tgcctatggg accagaaata atgatgagct catccaaagg 4200
aactacaaac tcgctgccca ggaatgctat gttcaaaata ctgccaggta ttatgccaag 4260
atctacgctg ctgaagcaca gcctctggaa ggctttggag aagtacctga gatcattcct 4320

atttttctta tccatcggcc tgagaacaat atcccgtatg ctacagtgga ggaggagctg 4380
attggagaat ttgtgaagta ttccatcagg gatgggaaag aaataaactt cttgagaaga 4440
gaatcagaag ctggtcagaa atgttgcacc ttccagcact gggtgtacca gaaaacaagt 4500
ggctgcctcc tggtgacgga catgcaaggt gtaggaatga agctaactga cgttggcata 4560
gcaacgctgg ctaaagggta caagggattt aaaggcaact gttccatgac cttcattgat 4620
cagtttaaag cactacacca gtgtaacaag tattgcaaaa tgctgggact gaaatccctt 4680
caaaacaaca accagaaaca gaagcagccg agcattggga aaagcaaagt tcaaacaaac 4740
tctatgacag taaagaaggc agggcctgag accccaggcg aaaagaaaac ctaacgtccc 4800
tgggtaacct aatggccact ggctagcagc acacaatctc gccagggaaa atctgaggcc 4860
acacaggaga gaatatacag cctgcagaga gtgcgtggca atccttaccc ccagccgact 4920
gtgcgccaag atgcttctaa acccatcacc tgctgtcttc actcaaatga tttcagaaca 4980
ggatttgcga ccaggtttat ggggagattg aatcaacgat tggtctcaaa gacaggccat 5040
tctttatata cacgtttagc atttttacca acctcacatc atgtgtatat ttgtgtattt 5100
gcacatggtt gtgctgtcga ggacctggtg ctgagaagag tctgttcaca gccaaaattc 5160
ttcccactgt cattcctaac ctgggatttc tagacacatc ctgctgtgat gtaaacagaa 5220
atcacgaatt cgctcactgg atcaagttgt tccactggtg tctaatacgc tattgttgcc 5280
ggaggtgggt tctgtgacgt gaagccattt cccatcattc aacagccagt tacaattttc 5340
tgtttaatta aattcatatt taaacaaaaa aaaaa 5375

<210> 95
<211> 1920
<212> DNA
<213> Homo sapiens

<400> 95
agcttctgcc aagggtgggg gcccacgcgg aggcgatccg ctcgttcctc ccagggccat 60
ggggcgacga ggagagccct ggcctccccg cgacccgcac cgcgacctgg gccagacgcg 120
ccaccttccc cggtcgcggt ttgcttctct ttaaaatgag gacagctcct cccttggggg 180
ctgtggtgac aggtgaaatg agaacgcact gaagacagct cttggtccaa agccccgcac 240
acagggcatg gtctagtggc ccagtcagga cgcggaaaca ctccctggag gttctgaccc 300
actccctctc agcctccgcc tggtctctgg gttggctgtt ccagctccaa agagaaggag 360
gaaccttttc cttctgcagc ccctccccac cagctccatt ccttagctgg ggtcagacct 420
ggggtcctca ctgcagctgg cctctggcag cgttctcagg ctagccctcc ctgctgaaaa 480
gagaaccgtg tgggactcac aggtgtagtc gccgccgcca gccgccatgg gcaaacagaa 540
cagcaagctg cggcccgagg tgctgcagga cctgcgggag aacacggagt tcaccgacca 600
cgagctgcag gagtggtaca agggcttcct caaggactgc cccaccggcc acctgaccgt 660
ggacgagttc aagaagatct acgccaactt cttcccctac ggcgacgctt ccaagttcgc 720
cgagcacgtc ttccgcacct tcgacaccaa cggcgacggc accatcgact tccgggagtt 780
catcattgcg ctgagcgtga cctcgcgggg caagctggag cagaagctca agtgggcctt 840
cagcatgtac gacctggacg gcaacggcta catcagccgc agcgagatgc tggagatcgt 900
gcaggccatc tacaagatgg tgtcgtctgt gatgaagatg ccggaggatg agtccacccc 960
ggagaagcgc acagacaaga tcttcaggca gatggacacc aacaatgacg gcaaactgtc 1020
cttggaagaa ttcatcagag gtgccaagag cgacccctcc atcgtccgcc tgctgcagtg 1080
cgaccccagc agtgccagtc agttctgagc gagcggcccc tggacagttg cagagaaaca 1140
caggcttgtc gtgccgttta agctttgctt gcaagagtgg atgccccgca atcgttcctg 1200
ctctcccggg ccccgggcct ggggcatgcg ttgcacctgc ccggcccggt ggctgcgcct 1260
ccctcctcca cctgaccaac gcgacattcc tcccctcacg cctggcccgg tcccttccag 1320
ggcaactccc agggatgtgg tgacatgcag ggttcaagtg ttcttggttc caggcacctc 1380
ccggctcacg gggagctcag aggtccatgc cgaggagacc aggcaggacc tcccgaggct 1440
gcgccccggc cggcccatgc gttttgtgat cccaagtgac tctgtgggaa gggtggggac 1500
gaggcgtcgg gagggtatac agggagcccc tcccgtgcat ggctgccccc ccgttcattt 1560
tctccaccac agccgcttgc acgtatagat actgtggtcc cctttctttt aatatataaa 1620
ttatgtatgg tgaagtggag tgtattgtgt aggtcccgta tttaatgcct ctgactgcct 1680
ttgaagcgca gccctctgtg gcccgcagcc ccctgagcct ggctgttgtg tggtatttat 1740
gctctctttg tctgcctgtt tctaaggaaa tgcatgtgtg ccctgagccg tgatgatcct 1800
cccatccgtg ttgtgagcac aggcatttgt gtctggtctg tcctccctgt tgattggtct 1860
ggcatttccg gtattaaaat gataaaataa atggcatttt ctgaaaaaaa aaaaaaaaaa 1920

<210> 96
<211> 1769
<212> DNA
<213> Homo sapiens

<400> 96
cggcggcggc gggcagcgga cgggcggact gacgggcgcc tccaccttgc tccctccctg 60
gctgccggct tccttttgtc tttctgggcg gcgatgagcg cagggccggc gcagcagctg 120
cgggcgcacg gaggcccgcg ctgctagtca ctcctcccgg cctcggcgcg cttgtcccgg 180
gcagcggccc gggcccgctg cagccgccgc cggcgccgaa cttgggctcg ggaagccggc 240
ggaccgcgtc ctgcgccgga gcaggggcat ggtctagtgg cccagtcagg acgcggaaac 300
actccctgga ggttctgacc cactccctct cagcctccgc ctggtctctg gtgtagtcgc 360
cgccgccagc cgccatgggc aaacagaaca gcaagctgcg gcccgaggtg ctgcaggacc 420
tgcgggagaa cacggagttc accgaccacg agctgcagga gtggtacaag ggcttcctca 480
aggactgccc caccggccac ctgaccgtgg acgagttcaa gaagatctac gccaacttct 540
tcccctacgg cgacgcttcc aagttcgccg agcacgtctt ccgcaccttc gacaccaacg 600
gcgacggcac catcgacttc cgggagttca tcattgcgct gagcgtgacc tcgcggggca 660
agctggagca gaagctcaag tgggccttca gcatgtacga cctggacggc aacggctaca 720
tcagccgcag cgagatgctg gagatcgtgc aggccatcta caagatggtg tcgtctgtga 780
tgaagatgcc ggaggatgag tccaccccgg agaagcgcac agacaagatc ttcaggcaga 840
tggacaccaa caatgacggc aaactgtcct tggaagaatt catcagaggt gccaagagcg 900
acccctccat cgtccgcctg ctgcagtgcg accccagcag tgccagtcag ttctgagcga 960
gcggcccctg gacagttgca gagaaacaca ggcttgtcgt gccgtttaag ctttgcttgc 1020
aagagtggat gccccgcaat cgttcctgct ctcccgggcc ccgggcctgg ggcatgcgtt 1080
gcacctgccc ggcccggtgg ctgcgcctcc ctcctccacc tgaccaacgc gacattcctc 1140
ccctcacgcc tggcccggtc ccttccaggg caactcccag ggatgtggtg acatgcaggg 1200
ttcaagtgtt cttggttcca ggcacctccc ggctcacggg gagctcagag gtccatgccg 1260
aggagaccag gcaggacctc ccgaggctgc gccccggccg gcccatgcgt tttgtgatcc 1320
caagtgactc tgtgggaagg gtggggacga ggcgtcggga gggtatacag ggagcccctc 1380
ccgtgcatgg ctgccccccc gttcattttc tccaccacag ccgcttgcac gtatagatac 1440
tgtggtcccc tttcttttaa tatataaatt atgtatggtg aagtggagtg tattgtgtag 1500
gtcccgtatt taatgcctct gactgccttt gaagcgcagc cctctgtggc ccgcagcccc 1560
ctgagcctgg ctgttgtgtg gtatttatgc tctctttgtc tgcctgtttc taaggaaatg 1620
catgtgtgcc ctgagccgtg atgatcctcc catccgtgtt gtgagcacag gcatttgtgt 1680
ctggtctgtc ctccctgttg attggtctgg catttccggt attaaaatga taaaataaat 1740
ggcattttct gaaaaaaaaa aaaaaaaaa 1769

<210> 97
<211> 2125
<212> DNA
<213> Homo sapiens

<400> 97
   gctgactctg aggaccccgt ggacattaaa gaaaaatgaa caggtttttc ttaaagcacc 60
   aacctgctct ttaacaaaaa ttatgaaagg ttaaaaagag tctataaaaa cttaccttat 120
   ggtcaaacat gaaaaattgg ataaatatgt ctacaaggtt ttattaaaat taagtttaac 180
   attaataaca cactaatata aaggtaaaat ttagcttatc tggtataaaa gtcatacagg 240
   aagcattagt aaatataaaa tagcgtttag ctttcttttg tctaaaaact aataaaaatt 300
   ggtgctaaag gaagcattca ttttactaga ggatcataaa agttaaagac ttaaaacaaa 360
   ctttggcaat taagacagca taccaagatg caaatgcctg gttgaaatgg atcaaatatt 420
   ccatctgcag gttaaacaaa agcaattagc atgcttgtgc acatggcagg ccagagaccc 480
   tgattgtccc ccttccacta aggtggtcct ccagtcgacc aggcatgggc tgcatggtag 540
   ctcttttcca ggattctaca gcctggagta ataagtcatg ccaagctctc tctgctatat 600
   cctgaagtcc ctgcgggtca gcccccgagg gccatccagc ttccgtctcc caacactaag 660
   ttcacttctt gtctctcatg gcagggagga gacttagcat tccttggaga cctgaaggga 720
   tgcagtgagc ttaagaattt tcaagagctt atcaatcagt cagcccttgt tcatccccga 780
   gcggatgtgt ggtggtattg tggtggacct ttattgggca ctctgccgaa taactagagt 840
   ggcacttgtg ctttagccta tttggctatc cctttcaccc tggcatttca tcaaccagag 900
   gaaggaaaaa aaaaataata agacatcgta aagcgagaga agccccttat gggtctttca 960
   actctcacat ctatttagat gcaatcgaag ccccgcaagg aacaccagat caatttaaag 1020
   tccgaaatca aatagctaca ggatttaagt caatattttg gtagatgaca gtcaataaaa 1080
   atgtagatta gataaactgc atctattaca cccaacagca atgagctttt catgagttga 1140
   aaaaaagaaa aactcatgtc ggccccagcc ctgaggctac ctgacctgac aaaactcttt 1200
   acactctatg tgtcagaaag agaaaaaatg gcagttggag ttttaaccca gactgtaggg 1260
   ccctggccaa ggccagtggc ctatctctca aaacaactag acggggtttc caaaggctgg 1320
   cccccatgtc caagggccct ggcagcaata gccctgttag cacaagaagc agataagcta 1380
   actcttaggc aaaacctaaa cataaagtcc ccccatgctg tggtaacttt aataaatacc 1440
   aaaggacatc attagctaat gaatgctaga ctgactgact agataccaaa gcttgctctg 1500
   tgaaaatccc tgcataaccg tcgaagtttg caacacccta aacccagcca ccttactcct 1560
   ggtatcagag agcccagttg aacataactg tgtagagtta ttggactcag tttattctag 1620
   tgggtccaac tccaagacca tccttaaaca tcagtagact gggagctgta cgtggatggg 1680
   agcacgttcg ccaacccctg caaagtgatt ctgaagaaga cgacaagcgc tgctccagtc 1740
   acacccggaa gctgactggt ccactcatgg ccgagcatga ggaaactcat cgcgggactc 1800
   attttcctta aaatttggac ttgcacagta aggacttcaa ctgaccttcc tcagactgag 1860
   gactgttccc aggatataca tcaagtcact gaggtaggga aaaaaggttg ctacagtcct 1920
   attattttat agttattatg aatgcctagg aactccaaaa ggaacttgtt tgtacaataa 1980
   cactcagtac aaagtatgta atccaggaag tgaccagcct gaagcgtgtt atgactcact 2040
   gtaagcctcc catgattaag gactgatcct tttctaagtg acagaagtag agtaatagct 2100
   aaaaaaaaaa aaaaaaaagt cgagc 2125

<210> 98
<211> 1411
<212> DNA
<213> Homo sapiens

<400> 98
ggcacgaggg agaggcagga ggacaccgag ttccccgtgt tggcctccag gtcctgtgct 60
tgcggagccg tccggcggct gggatcgagc cccgacaatg ggcaacgcgc aggagcggcc 120
gtcagagact atcgaccgcg agcggaaacg cctggtcgag acgctgcagg cggactcggg 180
actgctgttg gacgcgctgc tggcgcgggg cgtgctcacc gggccagagt acgaggcatt 240
ggatgcactg cctgatgccg agcgcagggt gcgccgccta ctgctgctgg tgcagggcaa 300
gggcgaggcc gcctgccagg agctgctacg ctgtgcccag cgtaccgcgg gcgcgccgga 360
ccccgcttgg gactggcagc acgtgggtcc gggctaccgg gaccgcagct atgaccctcc 420
atgcccaggc cactggacgc cggaggcacc cggctcgggg accacatgcc ccgggttgcc 480
cagagcttca gaccctgacg aggccggggg ccctgagggc tccgaggcgg tgcaatccgg 540
gaccccggag gagccagagc cagagctgga agctgaggcc tctaaagagg ctgaaccgga 600
gccggagcca gagccagagc tggaacccga ggctgaagca gaaccagagc cggaactgga 660
gccagaaccg gacccagagc ccgagcccga cttcgaggaa agggacgagt ccgaagattc 720
ctgaaggcca gagctctgac aggcggtgcc ccgcccatgc tggataggac ctgggatgct 780
gctggagctg aatcggatgc caccaaggct cggtccagcc cagtaccgct ggaagtgaat 840
aaactccgga gggtcggacg ggacctgggc tctctccacg attctggctg tttgcccagg 900
aacttagggt gggtacctct gagtcccagg gacctgggca ggcccaagcc caccacgagc 960
atcatccagt cctcagccct aatctgccct taggagtcca ggctgcaccc tggagatccc 1020
aaacctagcc ccctagtggg acaaggacct gaccctcctg cccgcataca caacccattt 1080
cccctggtga gccacttggc agcatatgta ggtaccagct caaccccacg caagttcctg 1140
agctgaacat ggagcaaggg gagggtgact tctctccaca tagggagggc ttagagctca 1200
cagccttggg aagtgagact agaagagggg agcagaaagg gaccttgagt agacaaaggc 1260
cacacacatc attgtcatta ctgttttaat tgtctggctt ctctctggac tgggagctca 1320
gtgaggattc tgaccagtga cttacacaaa aggcgctcta tacatattat aatatattcg 1380
cttactaaat gaaaaaaaaa aaaaaaaaaa a 1411

<210> 99
<211> 1982
<212> DNA
<213> Homo sapiens

<400> 99
ggcgagaggc gggctgaggc ggcccagcgg cggcaggtga ggcggaacca accctcctgg 60
ccatgggagg ggccgtggtg gacgagggcc ccacaggcgt caaggcccct gacggcggct 120
ggggctgggc cgtgctcttc ggctgtttcg tcatcactgg cttctcctac gccttcccca 180
aggccgtcag tgtcttcttc aaggagctca tacaggagtt tgggatcggc tacagcgaca 240
cagcctggat ctcctccatc ctgctggcca tgctctacgg gacaggtccg ctctgcagtg 300
tgtgcgtgaa ccgctttggc tgccggcccg tcatgcttgt ggggggtctc tttgcgtcgc 360
tgggcatggt ggctgcgtcc ttttgccgga gcatcatcca ggtctacctc accactgggg 420
tcatcacggg gttgggtttg gcactcaact tccagccctc gctcatcatg ctgaaccgct 480
acttcagcaa gcggcgcccc atggccaacg ggctggcggc agcaggtagc cctgtcttcc 540
tgtgtgccct gagcccgctg gggcagctgc tgcaggaccg ctacggctgg cggggcggct 600
tcctcatcct gggcggcctg ctgctcaact gctgcgtgtg tgccgcactc atgaggcccc 660
tggtggtcac ggcccagccg ggctcggggc cgccgcgacc ctcccggcgc ctgctagacc 720
tgagcgtctt ccgggaccgc ggctttgtgc tttacgccgt ggccgcctcg gtcatggtgc 780
tggggctctt cgtcccgccc gtgttcgtgg tgagctacgc caaggacctg ggcgtgcccg 840

acaccaaggc cgccttcctg ctcaccatcc tgggcttcat tgacatcttc gcgcggccgg 900
ccgcgggctt cgtggcgggg cttgggaagg tgcggcccta ctccgtctac ctcttcagct 960
tctccatgtt cttcaacggc ctcgcggacc tggcgggctc tacggcgggc gactacggcg 1020
gcctcgtggt cttctgcatc ttctttggca tctcctacgg catggtgggg gccctgcagt 1080
tcgaggtgct catggccatc gtgggcaccc acaagttctc cagtgccatt ggcctggtgc 1140
tgctgatgga ggcggtggcc gtgctcgtcg ggcccccttc gggaggcaaa ctcctggatg 1200
cgacccacgt ctacatgtac gtgttcatcc tggcgggggc cgaggtgctc acctcctccc 1260
tgattttgct gctgggcaac ttcttctgca ttaggaagaa gcccaaagag ccacagcctg 1320
aggtggcggc cgcggaggag gagaagctcc acaagcctcc tgcagactcg ggggtggact 1380
tgcgggaggt ggagcatttc ctgaaggctg agcctgagaa aaacggggag gtggttcaca 1440
ccccggaaac aagtgtctga gtggctgggc ggggccggca ggcacaggga ggaggtacag 1500
aagccggcaa cgcttgctat ttattttaca aactggactg gctcaggcag ggccacggct 1560
gggctccagc tgccggccca gcggatcgtc gcccgatcag tgttttgagg gggaaggtgg 1620
cggggtggga accgtgtcat tccagagtgg atctgcggtg aagccaagcc gcaaggttac 1680
aaggcatcct caccaggggc cccgcctgct gctcccaggt ggcctgcggc cactgctatg 1740
ctcaaggacc tggaaaccca tgcttcgaga caacgtgact ttaatgggag ggtgggtggg 1800
ccgcagacag gctggcaggg caggtgctgc gtggggccct ctccagcccg tcctaccctg 1860
ggctcacatg gggcctgtgc ccacccctct tgagtgtctt ggggacagct ctttccaccc 1920
ctggaagatg gaaataaacc tgcgtgtggg tggagtgttc tcgtgccgaa ttcaaaaagc 1980
tt 1982

<210> 100
<211> 2643
<212> DNA
<213> Homo sapiens

<400> 100
   acgtggtacg gaaccggcgc cgcgcttgct gctggtaaca gggccttgcc tagtgggcct 60
   tccttcccag gtcgcccctc agtctccact agagacagga ctgaccagtt gctcttcctt 120
   ccaagaacct tcgagatctg cggtctgggg tctggttgaa agatggcggc cctcactacc 180
   ctgtttaagt acatagatga aaatcaggat cgctacatta agaaactcgc aaaatgggtg 240
   gctatccaga gtgtgtctgc gtggccggag aagagaggcg aaatcaggag gatgatggaa 300
   gttgctgctg cagatgttaa gcagttgggg ggctctgtgg aactggtgga tatcggaaaa 360
   caaaagctcc ctgatggctc ggagatcccg ctccctccta ttctgctcgg caggctgggc 420
   tccgacccac agaagaagac cgtgtgcatt tacgggcacc tggatgtgca gcctgcagcc 480
   ctggaggacg gctgggacag cgagcccttc accctggtgg agcgagacgg caagctgtat 540
   gggagaggtt cgactgatga taagggcccg gtggccggct ggataaacgc cctggaagcg 600
   tatcagaaaa caggccagga gattcctgtc aacgtccgat tctgcctcga aggcatggag 660
   gagtcaggct ctgagggcct agacgagctg atttttgccc ggaaagacac attctttaag 720
   gatgtggact atgtctgcat ttctgacaat tactggctgg gaaagaagaa gccctgcatc 780
   acctacggcc tcaggggcat ttgctacttt ttcatcgagg tggagtgcag caacaaagac 840
   ctccattctg ggtgtacggg gggctcggtg catgaggcca tgactgatct cattttgctg 900
   atgggctctt tggtggacaa gagggggaac atcctgatcc ccggcattaa cgaggccgtg 960
   gccgccgtca cggaagagga gcacaagctg tacgacgaca tcgactttga catagaggag 1020
   tttgccaagg atgtgggggc gcagatcctc ctgcacagcc acaagaaaga catcctcatg 1080
   caccgatggc ggtacccgtc tctgtccctc catggcatcg aaggcgcctt ctctgggtct 1140
   ggggccaaga ccgtgattcc caggaaggtg gttggcaagt tctccatcag gctcgtgccg 1200
   aacatgactc ctgaagtcgt cggcgagcag gtcacaagct acctaactaa gaagtttgct 1260
   gaactacgca gccccaatga gttcaaggtg tacatgggcc acggtgggaa gccctgggtc 1320
   tccgacttca gtcaccctca ttacctggct gggagaagag ccatgaagac agtttttggt 1380
   gttgagccag acttgaccag ggaaggcggc agtattcccg tgaccttgac ctttcaggag 1440
   gccacgggca agaacgtcat gctgctgcct gtggggtcag cggatgacgg agcccactcc 1500
   cagaatgaaa agctcaacag gtataactac atagagggaa ccaagatgct ggccgcgtac 1560
   ctgtatgagg tctcccagct gaaggactag gccaagccct ctgtgtgcca tctccaatga 1620
   gaaggaatcc tgccctcacc tcaccctttt ccaacttgcc cagggaagtg gaggttccct 1680
   ctttcctttc cctcttgtca ggtcatccat gactttagag aacagacaca agtgtatcca 1740
   gctgtccacg ggtggagcta cccgttgggc ttatgagtga cctggagtga cagctgagtc 1800
   accctgggta agttctcaga gtggtcagga tggcttgacc tgcagaagat acccaaggtc 1860
   caaaagcaca aggtctgcgg aaagttctgg ttgtcggctg ggcaccacgg ctcacaccta 1920
   taatcgagca ctttgggagg ccaagacagg aggatcactt gaggccagga gtctgagaca 1980
   agcctaggca acaaaacaag actctgtctc tacaaaaagt ttaagaaatg agccagacat 2040
   ggtggtgtat gcctgtagtc ccagccactc agaaggctga ggcaggagga tcgcttgaga 2100
   ccaagagttt gagcctgcgg tgagctgtga atgcaccacg gcactcaagc ctgggcaatg 2160
   tagcaagatc ctgtctctac aagaaatttt ttaaaaatga gccaagtgtg gtggtgcatg 2220
   cctgtagttc cagctactca ggacactgac gtaggagggt tgcttgagac tgagagttgg 2280
   aggctgcgat gagccatgaa tgccccactg cactccagcc tgggcgacag aacgagaccc 2340
   catctcaaaa aaaataagtt ctggttgtca ttgaattggg ataaacagag agcttgatgc 2400
   tttctgcctt ctgtctcagg tgatgcattg cacatttggg atatttggaa aggaaatgag 2460
   gaaagaaatt agggcctcct ctgatctctc gctatctgcg ggtcctgtcc ttttctcaag 2520
   accttcacca ttactggcat tttcctgtct tctctttagt atgatccctc aaaacctcac 2580
   taactggaag gatgattttg tctcagtttg tactcctaaa taaaaagtaa acatgacacc 2640
   tct 2643

<210> 101
<211> 2349
<212> DNA
<213> Homo sapiens

<400> 101
gtcgcccctc agtctccact agagacagga ctgaccagtt gctcttcctt ccaagaacct 60
tcgagatctg cggtctgggg tctggttgaa agatggcggc cctcactacc ctgtttaagt 120
acatagatga aaatcaggat cgctacatta agaaactcgc aaaatgggtg gctatccaga 180
gtgtgtctgc gtggccggag aagagaggcg aaatcaggag gatgatggaa gttgctgctg 240
cagatgttaa gcagttgggg ggctctgtgg aactggtgga tatcggaaaa caaaaggaga 300
ttcctgtcaa cgtccgattc tgcctcgaag gcatggagga gtcaggctct gagggcctag 360
acgagctgat ttttgcccgg aaagacacat tctttaagga tgtggactat gtctgcattt 420
ctgacaatta ctggctggga aagaagaagc cctgcatcac ctacggcctc aggggcattt 480
gctacttttt catcgaggtg gagtgcagca acaaagacct ccattctggg gtgtacgggg 540
gctcggtgca tgaggccatg actgatctca ttttgctgat gggctctttg gtggacaaga 600
gggggaacat cctgatcccc ggcattaacg aggccgtggc cgccgtcacg gaagaggagc 660
acaagctgta cgacgacatc gactttgaca tagaggagtt tgccaaggat gtgggggcgc 720
agatcctcct gcacagccac aagaaagaca tcctcatgca ccgatggcgg tacccgtctc 780
tgtccctcca tggcatcgaa ggcgccttct ctgggtctgg ggccaagacc gtgattccca 840
ggaaggtggt tggcaagttc tccatcaggc tcgtgccgaa catgactcct gaagtcgtcg 900
gcgagcaggt cacaagctac ctaactaaga agtttgctga actacgcagc cccaatgagt 960
tcaaggtgta catgggccac ggtgggaagc cctgggtctc cgacttcagt caccctcatt 1020
acctggctgg gagaagagcc atgaagacag tttttggtgt tgagccagac ttgaccaggg 1080
aaggcggcag tattcccgtg accttgacct ttcaggaggc cacgggcaag aacgtcatgc 1140
tgctgcctgt ggggtcagcg gatgacggag cccactccca gaatgaaaag ctcaacaggt 1200
ataactacat agagggaacc aagatgctgg ccgcgtacct gtatgaggtc tcccagctga 1260
aggactaggc caagccctct gtgtgccatc tccaatgaga aggaatcctg ccctcacctc 1320
acccttttcc aacttgccca gggaagtgga ggttccctct ttcctttccc tcttgtcagg 1380
tcatccatga ctttagagaa cagacacaag tgtatccagc tgtccacggg tggagctacc 1440
cgttgggctt atgagtgacc tggagtgaca gctgagtcac cctgggtaag ttctcagagt 1500
ggtcaggatg gcttgacctg cagaagatac ccaaggtcca aaagcacaag gtctgcggaa 1560
agttctggtt gtcggctggg caccacggct cacacctata atcgagcact ttgggaggcc 1620
aagacaggag gatcacttga ggccaggagt ctgagacaag cctaggcaac aaaacaagac 1680
tctgtctcta caaaaagttt aagaaatgag ccagacatgg tggtgtatgc ctgtagtccc 1740
agccactcag aaggctgagg caggaggatc gcttgagacc aagagtttga gcctgcggtg 1800
agctgtgaat gcaccacggc actcaagcct gggcaatgta gcaagatcct gtctctacaa 1860
gaaatttttt aaaaatgagc caagtgtggt ggtgcatgcc tgtagttcca gctactcagg 1920
acactgacgt aggagggttg cttgagactg agagttggag gctgcgatga gccatgaatg 1980
ccccactgca ctccagcctg ggcgacagaa cgagacccca tctcaaaaaa aataagttct 2040
ggttgtcatt gaattgggat aaacagagag cttgatgctt tctgccttct gtctcaggtg 2100
atgcattgca catttgggat atttggaaag gaaatgagga aagaaattag ggcctcctct 2160
gatctctcgc tatctgcggg tcctgtcctt ttctcaagac cttcaccatt actggcattt 2220
tcctgtcttc tctttagtat gatccctcaa aacctcacta actggaagga tgattttgtc 2280
tcagtttgta ctcctaaata aaaagtaaac atgacacctc taaaaaaaaa aaaaaaaaaa 2340
aaaaaaaaa 2349

<210> 102
<211> 516
<212> DNA
<213> Homo sapiens

<400> 102

ttttttacaa agggacttaa aattgtcagg atgatggcat atcatcagtg agatgcttct 60
acgttgatgt gcacttgtgt atgcatgtat tatacatacc ctcatgatgg gaactggagt 120
caataatttt cttttctttc tttaggacac tttcctagat ctttccactg ttaacccaac 180
catccagatc tcctaaactc tcctgagaac ttagaaatta actcctttcc tcttccctat 240
ttggctgcgc taatagagca ggcatgcaat actaagcttc acagctaggt gtcctcataa 300
tgcaatgact tcctttggca gcctagtcat tgacagctga ctttgagtca agaaagtaag 360
ttttctctct cctttatgtt taatttctat ttagggagat tccttatgag ggatacttat 420
gaaggccact ttcctcctgc tatgtgctca gagctaactc catattttcc tgtaagttta 480
taagtagctt gcaattcagt taggtgtcca ccaatg 516

<210> 103
<211> 3224
<212> DNA
<213> Homo sapiens

<400> 103
gtcccacatc ccgcatccgg catcccagcg gccgggcatg tagcagcggc agcaacggcg 60
gaatatgggc gggaaccact cccacaagcc ccccgtgttt gacgagaatg aggaagtcaa 120
ctttgaccat tttcagattc tgcgggccat tggtaaaggg agttttggaa aggtatgcat 180
cgtgcagaag cgagacacta agaaaatgta tgcaatgaag tacatgaaca agcagaagtg 240
catcgagagg gatgaggttc ggaatgtttt ccgggagctg cagatcatgc aagggctgga 300
gcaccccttc ctggtcaatc tgtggtactc cttccaggat gaggaggaca tgttcatggt 360
ggtggacctg ctcctgggag gcgacctgcg ctaccatctg cagcagaatg tgcatttcac 420
agaggggact gtgaaactct acatctgtga gctggcactg gccctggagt atcttcagag 480
gtaccacatc atccacagag acatcaagcc agacaatatc ctgctggatg aacacggaca 540
tgttcacatt acagacttca acatagcgac ggtagtgaaa ggagcagaaa gggcttcctc 600
catggctggc accaagccct acatggctcc agaagtattc caggtgtaca tggacagagg 660
ccccggatac tcgtaccctg tcgactggtg gtccctgggc atcacagcct atgagctgct 720
gcggggctgg aggccgtacg aaatccactc ggtcacgccc atcgatgaaa tcctcaacat 780
gttcaaggtg gagcgtgtcc actactcctc cacgtggtgc aaggggatgg tggccctgct 840
gaggaagctc ctgaccaagg atcctgagag ccgcgtgtcc agccttcatg acatacagag 900
cgtgccctac ttggccgaca tgaactggga cgcggtgttc aagaaggcac tgatgcccgg 960
ctttgtgccc aataaaggga ggttgaactg cgatcccaca tttgagcttg aagagatgat 1020
tctagaatcc aagccacttc acaaaaagaa gaagcgattg gcaaagaaca gatccaggga 1080
tggcacaaag gacagctgcc cgctgaatgg acacctgcag cactgtttgg agactgtccg 1140
ggaggaattc atcatattca acagagagaa gctcaggagg cagcagggac agggcagcca 1200
gctcttggac accgacagcc gagggggagg ccaggcccaa agcaagctcc aggacgggtg 1260
caacaacaac ctcctcaccc acacctgcac ccgtggctgc agcagctgag cccacacttg 1320
ttgctgctca acaggactgc actcgtctct gccctgccca cccagagccc ctctttgtgc 1380
cctgatggtc cctgtctcac ccctgaaaac atcagatgca gaaaaagccc tggacttgga 1440
gctgggaagc ctgggttctg gtcccatctc catgactgat tcacgtgtga cctcagacaa 1500
gtcacgccct ctctgtgcct ccgttttctg catctgccaa aggggttaaa cacttctgcc 1560
ccacttcaaa ttacaagatt atggggagaa cccaattagg taggaaacat gaaaaacctt 1620
tgatatttat aaaatcattt ttacgtgcaa aatataacct taatatttga agtgaccccc 1680
attccccaaa gcaatcaaac cgtcatgact ttgcaatttg gcacatccta gcttgttaga 1740
gggcacttcc gaaaaacaca gccctgacag caaaataaag gtctgatatg ttggcccctt 1800
ctatggaaac aacgctgcca aatcctggag caaaacctga agtgtcttca tgtgcattct 1860
ctggcaggcc acagtcctga gcttgtaaga tggtgcagca tgcagaccag acttgtcccc 1920
aaggtctcag cgctgcggtc tcactcctcc cctcatttaa gaagactatc cttacctttt 1980
agtttcagca gtcctcacca ccaccatatc cccagtgctg ggatggcaca caggtgtcca 2040
ttcagatgag agttgggtcg ctgagcattg gttactcctg cagagtgtaa tcagcacccc 2100
atccaactgg cccgaaagcc cagacctgca gcagaactct ccaactctct atcagctttc 2160
agggttttct ctcctgggaa gggtgtaaaa tcagcttgtc agattcttct tacagagagt 2220
atccaatcgg tattggtgga gcggctccct atttatacaa taggaagcat gggtgcttag 2280
aaagtttatt tcaggaggaa aatgggttca cacaaaaagc aaactacatt ctgatctgct 2340
cagggagaag cttgcctttg aactggaaga tgttgggatg agcagggaaa gcttagactt 2400
tggagtcagg tttgtgttca gaatccagcc ctgctggcta ctaactaact gggagacctt 2460
aggcaaagca tgcaatcgct ctgaatggca gtttcctcat ttttaaacag ggataataaa 2520
actaatattg caggggagtt acagggttaa ataagatcct gtgtgtaacc ccaagcattg 2580
gatgactcat agaatggcct tttttgtcag cataatcgtc atcattattt agatactttc 2640
ttccttcact cacccagcag gtcagttttc tgtgcaaaca aacctgttta ggattcttcc 2700
aaatgttctt cctggggtct ttgatatttg tttgttacat cctgctgaag ttcgactgtg 2760
tttttatttt ttcatccaac ttccattttt cactttttac atgattactc aatccttggg 2820

gctgtccatg tcatctctta gatttcttaa aagacatttt aatgtatggt taggttttat 2880
atttttattt tttaaaaaag aaatagtcag tgttttcctc ctttcaaccg agactatttc 2940
tggattgtgt gctcctcgtc agttgacttg ttttgcacac ttttctttac ttcatgtccc 3000
catcaacaac cgtcctgctc cccacctccc ccaggaaata aggggcctgc tcctctccct 3060
actgtgaccc tggaggctct taagatgatg atggtttttt ttattgggct gagttcacga 3120
attaggggca ggagctggaa gtcgccctag gaacaccaga tttcctggtt ctgttcaagt 3180
tggcatttct tgtttggaat aaactatttc ttggacattc cttc 3224

<210> 104
<211> 1982
<212> DNA
<213> Homo sapiens

<400> 104
ggcgagaggc gggctgaggc ggcccagcgg cggcaggtga ggcggaacca accctcctgg 60
ccatgggagg ggccgtggtg gacgagggcc ccacaggcgt caaggcccct gacggcggct 120
ggggctgggc cgtgctcttc ggctgtttcg tcatcactgg cttctcctac gccttcccca 180
aggccgtcag tgtcttcttc aaggagctca tacaggagtt tgggatcggc tacagcgaca 240
cagcctggat ctcctccatc ctgctggcca tgctctacgg gacaggtccg ctctgcagtg 300
tgtgcgtgaa ccgctttggc tgccggcccg tcatgcttgt ggggggtctc tttgcgtcgc 360
tgggcatggt ggctgcgtcc ttttgccgga gcatcatcca ggtctacctc accactgggg 420
tcatcacggg gttgggtttg gcactcaact tccagccctc gctcatcatg ctgaaccgct 480
acttcagcaa gcggcgcccc atggccaacg ggctggcggc agcaggtagc cctgtcttcc 540
tgtgtgccct gagcccgctg gggcagctgc tgcaggaccg ctacggctgg cggggcggct 600
tcctcatcct gggcggcctg ctgctcaact gctgcgtgtg tgccgcactc atgaggcccc 660
tggtggtcac ggcccagccg ggctcggggc cgccgcgacc ctcccggcgc ctgctagacc 720
tgagcgtctt ccgggaccgc ggctttgtgc tttacgccgt ggccgcctcg gtcatggtgc 780
tggggctctt cgtcccgccc gtgttcgtgg tgagctacgc caaggacctg ggcgtgcccg 840
acaccaaggc cgccttcctg ctcaccatcc tgggcttcat tgacatcttc gcgcggccgg 900
ccgcgggctt cgtggcgggg cttgggaagg tgcggcccta ctccgtctac ctcttcagct 960
tctccatgtt cttcaacggc ctcgcggacc tggcgggctc tacggcgggc gactacggcg 1020
gcctcgtggt cttctgcatc ttctttggca tctcctacgg catggtgggg gccctgcagt 1080
tcgaggtgct catggccatc gtgggcaccc acaagttctc cagtgccatt ggcctggtgc 1140
tgctgatgga ggcggtggcc gtgctcgtcg ggcccccttc gggaggcaaa ctcctggatg 1200
cgacccacgt ctacatgtac gtgttcatcc tggcgggggc cgaggtgctc acctcctccc 1260
tgattttgct gctgggcaac ttcttctgca ttaggaagaa gcccaaagag ccacagcctg 1320
aggtggcggc cgcggaggag gagaagctcc acaagcctcc tgcagactcg ggggtggact 1380
tgcgggaggt ggagcatttc ctgaaggctg agcctgagaa aaacggggag gtggttcaca 1440
ccccggaaac aagtgtctga gtggctgggc ggggccggca ggcacaggga ggaggtacag 1500
aagccggcaa cgcttgctat ttattttaca aactggactg gctcaggcag ggccacggct 1560
gggctccagc tgccggccca gcggatcgtc gcccgatcag tgttttgagg gggaaggtgg 1620
cggggtggga accgtgtcat tccagagtgg atctgcggtg aagccaagcc gcaaggttac 1680
aaggcatcct caccaggggc cccgcctgct gctcccaggt ggcctgcggc cactgctatg 1740
ctcaaggacc tggaaaccca tgcttcgaga caacgtgact ttaatgggag ggtgggtggg 1800
ccgcagacag gctggcaggg caggtgctgc gtggggccct ctccagcccg tcctaccctg 1860
ggctcacatg gggcctgtgc ccacccctct tgagtgtctt ggggacagct ctttccaccc 1920
ctggaagatg gaaataaacc tgcgtgtggg tggagtgttc tcgtgccgaa ttcaaaaagc 1980
tt 1982

<210> 105
<211> 5239
<212> DNA
<213> Homo sapiens

<400> 105
agccatgccc acgaacttta cggtggtgcc cgtggaggct cacgccgacg gcggcgggga 60
cgagactgcc gagcggacgg aggctccggg cacccccgag ggccccgagc ccgagcgccc 120
cagcccggga gatggaaatc caagagaaaa cagcccattc ctcaacaatg tcgaggtgga 180
acaagagagc ttctttgaag ggaagaacat ggcacttttc gaggaggaga tggacagtaa 240
ccccatggtg tcctcgctgc tcaacaagct ggccaactac accaacctga gccagggcgt 300
ggtggagcac gaggaggacg aggagagccg gcggcgggag gccaaggctc cgcgcatggg 360
caccttcatc ggcgtctacc tgccgtgcct gcagaacatc ctgggcgtca tcctcttcct 420
gcgcctgacg tggatcgtgg gggtggctgg tgtcctggag tccttcctca tcgtggccat 480

gtgctgcaca tgtacaatgc tgaccgccat ttccatgagt gcgatcgcta ccaacggtgt 540
ggtcccagct ggcgggtcct actacatgat atcgcgctcg ctgggacccg agtttggagg 600
cgctgtcggc ctctgcttct acctgggcac gacgtttgca ggggccatgt atattttggg 660
gaccatcgag atttttctga cgtacatctc cccgggtgcg gccatcttcc aggcggaggc 720
tgcaggtggc gaggcggccg ccatgctgca caacatgcgt gtgtacggca cgtgcacgct 780
cgtgctcatg gccctggtgg tcttcgtggg cgtcaagtat gtcaacaagc tggcgctggt 840
cttcctggcc tgcgtcgtgc tgtccatcct ggccatctat gccggcgtca tcaagtctgc 900
cttcgacccc ccggacatcc cggtctgcct cctggggaac cgcacgctgt cacggcgcag 960
cttcgatgcc tgcgtcaagg cctacggcat ccacaacaac tcagccacct ccgcgctctg 1020
gggcctcttc tgcaacggct cccagcccag cgccgcctgt gacgagtact tcatccagaa 1080
caacgtcacc gaaatccagg gcatcccggg cgcggccagt ggtgtcttcc tggagaacct 1140
gtggagtacg tacgcgcacg cgggggcgtt tgtggagaag aaaggtgtgc cctcggtgcc 1200
cgtggcagag gagagccgtg ccagcacact gccctacgtg ctcaccgaca tcgcggcctc 1260
cttcaccctg ctggttggca tctacttccc ttccgtgacc ggtatcatgg cgggttcaaa 1320
ccggtccggg gacctcaagg atgcacagaa gtccatcccc acggggacca tcctggccat 1380
agtgacgacg tctttcatct atctctcctg cattgtgctg tttggggcct gcattgaagg 1440
cgtggtctta cgagataagt tcggggaggc cctgcagggg aacctggtca tcggcatgct 1500
ggcctggccc tccccctggg tcatcgtcat cggctccttc ttctccacct gcggtgccgg 1560
cctgcagacc ctcacggggg caccgcgcct actgcaggcc attgcccgtg acggcatcgt 1620
ccccttcctg caggtgtttg gccacgggaa ggccaacggg gagcccacgt gggcgctgct 1680
gctgacagtc ctcatctgcg agactggcat cctcatcgcc tctctggaca gcgtggcccc 1740
gatcctctcc atgttcttcc tcatgtgcta cctgttcgtg aacctggcct gcgccgtgca 1800
gaccctgcta cgtaccccca actggcgtcc acgcttcaag ttctaccact ggaccctgtc 1860
ctttctgggt atgagcctgt gcctggcgct gatgttcatc tgctcctggt actacgcgct 1920
gtccgccatg ctcatcgctg gctgcatcta caagtacatc gagtaccgcg gggccgagaa 1980
ggagtggggc gatggcatcc gtggcctatc cctgaacgcc gcccgctacg ccctgctgcg 2040
cgtggagcac ggtccccccc acaccaagaa ctggaggccc caggtgctgg tgatgctgaa 2100
cctggacgcg gagcaggccg tgaagcaccc ccgcctgctg tccttcacgt cgcagctgaa 2160
ggccggcaag ggcctgacca tcgtgggctc ggtgctggag gggacgtacc tggacaagca 2220
catggaggct cagcgggccg aggagaacat acggtcccta atgagcacag agaagaccaa 2280
gggcttctgc cagctggtgg tctcgtccag cctgcgggat ggcatgtccc acctgatcca 2340
gtcggccggc ctgggcggcc tgaagcacaa cacggtgctc atggcctggc ccgcatcctg 2400
gaagcaggag gacaacccct tctcctggaa gaactttgta gacaccgtcc gcgacaccac 2460
cgccgcgcac caggctctgc tggtggccaa gaacgtcgac tcgtttccgc aaaaccagga 2520
gcgcttcggc gggggccaca tcgacgtgtg gtggatcgtg cacgacggcg gcatgctcat 2580
gctgctgccc ttcctgctgc gccagcacaa ggtgtggagg aagtgccgga tgcgtatctt 2640
caccgtggcc caggtggacg acaacagcat ccagatgaag aaggacctgc agatgttctt 2700
gtatcacttg cgcatcagcg ccgaggtgga ggtggtggag atggttgaaa acgacatatc 2760
tgctttcacc tacgagagga cactaatgat ggagcagagg tcgcagatgc tgaagcagat 2820
gcagctgtcc aagaacgagc aggagcgaga ggcccagctg atccacgaca ggaacaccgc 2880
gtcccacacc gcggcggcag ccaggaccca agcgccgcct acgccagaca aggtgcagat 2940
gacctggacc agggagaagc tgatcgctga gaagtacagg agcagagaca ccagcctatc 3000
cggtttcaaa gacctcttca gcatgaagcc ggaccagtcc aacgtcaggc ggatgcacac 3060
ggctgtgaag ctcaatggcg tcgtcctcaa caagtcccag gatgcgcagc tggtcctgct 3120
caacatgcca ggtcctccca aaaaccggca gggagacgag aactacatgg agtttcttga 3180
agtcctgacc gaggggctga acagagtcct cctggtcagg ggtggcggcc gggaggtgat 3240
caccatctac tcctaatgcc caacagcatc acggcactct gggacaggca cggaggacgg 3300
cgtgggcagc ctgggcctgg gcttggccca gggaaccaga cggcagacac acctgtcccc 3360
cagtgatgcc acccaagctg cccatggggc ttcctacgga agtttctagg cccgtcacct 3420
agggctctcc tgttcagcct taacaggctc agcaaatcag ggcgtggctg gacgatttcc 3480
ttgcatctga gggcagacgc tgctaccgga gtgacctgga cgtggccaga tcttctcgca 3540
ggtcacaaga agccagtgag cccttgcctt ggtttctgga agttcttttc cttggctgga 3600
tttacccagt ggttaggttg catttctacc ccatccagaa cattcttgga agagcacccg 3660
gagctgaagc tgtccctgat gatgaaggtg aaacgtcagc cctggccatg gctccgctca 3720
gggccccggt cacctccgag tcactctgtt ccttgactgt ctttgtgttt ctgtacctca 3780
aggcactgaa gctggaggac tctgtccatg cccgtgtcac cctcgtgtgg gagcctctgg 3840
gctcggcagg tccacatttc atgagctgag gcgtgggcca gggccatctg gaaagggaac 3900
tcggcttttc cagaacgtgg tggatcatct gtcgggtgtg tggtgaacac gttcagttca 3960
tcagggccta cgctccggga aggggccccc agctgtggct ctgccatgcc gggctgtgtt 4020
tgcagctgtc cgagtctcca tccaccttta gaaaaccagt cacttctttt cataagcact 4080
gacagggccc agcccacagc cacaggtgcg atcagtgcct cacgcaggca aatgcactga 4140
aacccagggg cacacgcgcg cagagtgaac agtgagttcc cccgacagcc cacgacagcc 4200
aggactgccc tccccacccc accccacccc aggagcacgg cacacagttc agcctctgag 4260

ctggctcaca cgtgccatcc ccaccccggt gctccaggga aggaggacac ggacccgacg 4320
tgggaggtcc tcaggcagca gtggcgcctg gtgtcaggtc tgtctggctg agtcccgggc 4380
gtcccctgcc atggcctgtg ccttgcatgg aggcggcggt ggcactgaag agatagcttt 4440
caagggccca acactttgca cttcggctgg ctgtgagttt ctgctttgta ggttgtggtc 4500
acatttgcag gctgcgggca gtggcaccga cttgggcctc cctttctatg tggcatattt 4560
atttatttaa acaccccagg gagttacgtg gtaacaaggt tgtccataaa gaggttgctt 4620
ctatatacta gaggccccag atggcaggcc ttgggctacg tctggcttgc atggtctccc 4680
aagggaatca accccatcaa caaagttcaa atcggggcag aggctgcact tgtgccccca 4740
gatgtttctg aggagccaga ttagggctgg cattgctgta gagtgacggc tgctgcccag 4800
agcgtgtccc agacatcaca gcggggctca gcagttccca cagcctctgc ctgccttggc 4860
taagcatgag ttaagcagca aaacgctcct ccatgtctgg atggggccgg caggtcctgt 4920
gtcccctgca cctggaggag agcaggctag aggcacagcg gccacatggt gctggctctg 4980
aacgttggtt ggtggctgga aaacagccct gcttctgagg gccgctcagt tctgcacacg 5040
aaaccacctc ctgagggctc agctctgccc ccgccctggg ctgcagcctc tgcacgcaag 5100
caccaggcat cctttgtgtt gtcaactccg tgtaaccagt aactacagcc atttacaatt 5160
gactccgttt ccttttgtag gtttccctgt ctgtctgtgt tagtagaaaa ataaaatcct 5220
atgaaatctg aaaaaaaaa 5239

<210> 106
<211> 768
<212> DNA
<213> Homo sapiens

<400> 106
ccttcagcat aaaagctgat ccacaaacaa gaggagcacc agacctcctc ttggcttcga 60
gatggcttcg ccacaccaag agcccaaacc tggagacctg attgagattt tccgccttgg 120
ctatgagcac tgggccctgt atataggaga tggctacgtg atccatctgg ctcctccaag 180
tgagtacccc ggggctggct cctccagtgt cttctcagtc ctgagcaaca gtgcagaggt 240
gaaacggggg cgcctggaag atgtggtggg aggctgttgc tatcgggtca acaacagctt 300
ggaccatgag taccaaccac ggcccgtgga ggtgatcatc agttctgcga aggagatggt 360
tggtcagaag atgaagtaca gtattgtgag caggaactgt gagcactttg tcgcccagct 420
gagatatggc aagtcccgct gtaaacaggt ggaaaaggcc aaggttgaag tcggtgtggc 480
cacggcgctt ggaatcctgg ttgttgctgg atgctctttt gcgattagga gataccaaaa 540
aaaagcaaca gcctgaagca gccacaaaat cctgtgttag aagcagctgt gggggtccca 600
gtggagatga gcctccccca tgcctccagc agcctgaccc tcgtgccctg tctcaggcgt 660
tctctagatc ctttcctctg tttccctctc tcgctggcaa aagtatgatc taattgaaac 720
aagactgaag gatcaataaa cagccatctg ccccttcaaa aaaaaaaa 768

<210> 107
<211> 2529
<212> DNA
<213> Homo sapiens

<400> 107
cttggctctt acaatgctca cttgttttca caatgcagca aaatgaaatg ccttagaaaa 60
agagtaacat tccagaaaac ggtgtaattt atttttcttc cttaattgcc ccatctgtgg 120
aggatttctt tgctgaacac cacatcaaag ggatcttctg catttaaaat agaagaggca 180
tcatgctgaa gagggagggg aaggtccaac cttacactaa aaccctggat ggaggatggg 240
gatggatgat tgtgattcat tttttcctgg tgaatgtgtt tgtgatgggg atgaccaaga 300
cttttgcaat tttctttgtg gtctttcaag aagagtttga aggcacctca gagcaaattg 360
gttggattgg atccatcatg tcatctcttc gtttttgtgc aggtcccctg gttgctatta 420
tttgtgacat acttggagag aaaactacct ccattcttgg ggctttcgtt gttactggtg 480
gatatctgat cagcagctgg gccacaagta ttccttttct ttgtgtgact atgggacttc 540
tacccggttt gggttctgct ttcttatacc aagtggctgc tgtggtaact accaaatact 600
tcaaaaaacg attggctctt tctacagcta ttgcccgttc tgggatggga ctgacttttc 660
ttttggcacc ctttacaaaa ttcctgatag atctgtatga ctggacagga gcccttatat 720
tatttggagc tatcgcattg aatttggtgc cttctagtat gctcttaaga cccatccata 780
tcaaaagtga gaacaattct ggtattaaag ataaaggcag cagtttgtct gcacatggtc 840
cagaggcaca tgcaacagaa acacactgcc atgagacaga agagtctacc atcaaggaca 900
gtactacgca gaaggctgga ctacctagca aaaatttaac agtctcacaa aatcaaagtg 960
aagagttcta caatgggcct aacaggaaca gactgttatt aaagagtgat gaagaaagtg 1020
ataaggttat ttcgtggagc tgcaaacaac tgtttgacat ttctctcttt agaaatcctt 1080
tcttctacat atttacttgg tcttttctcc tcagtcagtt agcatacttc atccctacct 1140

ttcacctggt agccagagcc aaaacactgg ggattgacat catggatgcc tcttaccttg 1200
tttctgtagc aggtatcctt gagacggtca gtcagattat ttctggatgg gttgctgatc 1260
aaaactggat taagaagtat cattaccaca agtcttacct catcctctgc ggcatcacta 1320
acctgcttgc tcctttagcc accacatttc cactacttat gacctacacc atctgctttg 1380
ccatctttgc tggtggttac ctggcattga tactgcctgt actggttgat ctgtgtagga 1440
attctacagt aaacaggttt ttgggacttg ccagtttctt tgctgggatg gctgtccttt 1500
ctggaccacc tatagcaggc tggttatatg attataccca gacatacaat ggctctttct 1560
acttctctgg catatgctat ctcctctctt cagtttcctt tttttttgta ccattggccg 1620
aaagatggaa aaacagtctg acctgaaaga aagaagactg caatcaagtg agagctaaac 1680
aaaagaaaac ctaaactaat gtcattggaa acaaaagctt gaaagaaaca catcgcatct 1740
acatttgtaa catgagaagg aaaacaattt tttttttttt ttttttgaga cggagtctcg 1800
ctctttcgcc caggctggag tgcagtggcg caatctcggc tcactgtaat ctccgcctcc 1860
tgggttcaag ggattctcct gcctcagcct cccaagtagc tgggactaca ggcacacgcc 1920
accacaccca gctaattttt tgtattttta gtagaggcgg ggtttcacca tgttagccag 1980
gatggtctcc atctcctgac ctcgtgatcc gcccgccttg tcctccaaag tgctgggatt 2040
acaggcatga gccactgggc gcggccagat aagtttttaa ggttccttct tgctttagca 2100
ttctgagaaa tgtctaattg gtagtaagac aagagtaata gcaacctgta ttgttagtat 2160
ttaaccaaat aggctaaaat tttaatcagg taccttatgt attaaataga aatcggaatg 2220
taccataata aatccaaact ctcaattacg ccatggtaat tcagtcacta aaatatgtaa 2280
agatagaaaa ttttttaatt taaagaagtg tgaaacatag ccattgattg atcagaattc 2340
tggaatctga atattaaaac cttacttagt gactggaatg gtatatgctc cctccaaaag 2400
tttatctttg tttattgatt aaaggtaatc cttactttct ttgtattact taggttctca 2460
attaaaggta atccttactt tctttgtatt acttaggttc ttaaatttct atgataaaca 2520
tgtattgct 2529
<210> 108
<211> 2691
<212> DNA
<213> Homo sapiens

<400> 108
gcttgcccgt cggtcgctag ctcgctcggt gcgcgtcgtc ccgctccatg gcgctcttcg 60
tgcggctgct ggctctcgcc ctggctctgg ccctgggccc cgccgcgacc ctggcgggtc 120
ccgccaagtc gccctaccag ctggtgctgc agcacagcag gctccggggc cgccagcacg 180
gccccaacgt gtgtgctgtg cagaaggtta ttggcactaa taggaagtac ttcaccaact 240
gcaagcagtg gtaccaaagg aaaatctgtg gcaaatcaac agtcatcagc tacgagtgct 300
gtcctggata tgaaaaggtc cctggggaga agggctgtcc agcagcccta ccactctcaa 360
acctttacga gaccctggga gtcgttggat ccaccaccac tcagctgtac acggaccgca 420
cggagaagct gaggcctgag atggaggggc ccggcagctt caccatcttc gcccctagca 480
acgaggcctg ggcctccttg ccagctgaag tgctggactc cctggtcagc aatgtcaaca 540
ttgagctgct caatgccctc cgctaccata tggtgggcag gcgagtcctg actgatgagc 600
tgaaacacgg catgaccctc acctctatgt accagaattc caacatccag atccaccact 660
atcctaatgg gattgtaact gtgaactgtg cccggctcct gaaagccgac caccatgcaa 720
ccaacggggt ggtgcacctc atcgataagg tcatctccac catcaccaac aacatccagc 780
agatcattga gatcgaggac acctttgaga cccttcgggc tgctgtggct gcatcagggc 840
tcaacacgat gcttgaaggt aacggccagt acacgctttt ggccccgacc aatgaggcct 900
tcgagaagat ccctagtgag actttgaacc gtatcctggg cgacccagaa gccctgagag 960
acctgctgaa caaccacatc ttgaagtcag ctatgtgtgc tgaagccatc gttgcggggc 1020
tgtctgtaga gaccctggag ggcacgacac tggaggtggg ctgcagcggg gacatgctca 1080
ctatcaacgg gaaggcgatc atctccaata aagacatcct agccaccaac ggggtgatcc 1140
actacattga tgagctactc atcccagact cagccaagac actatttgaa ttggctgcag 1200
agtctgatgt gtccacagcc attgaccttt tcagacaagc cggcctcggc aatcatctct 1260
ctggaagtga gcggttgacc ctcctggctc ccctgaattc tgtattcaaa gatggaaccc 1320
ctccaattga tgcccataca aggaatttgc ttcggaacca cataattaaa gaccagctgg 1380
cctctaagta tctgtaccat ggacagaccc tggaaactct gggcggcaaa aaactgagag 1440
tttttgttta tcgtaatagc ctctgcattg agaacagctg catcgcggcc cacgacaaga 1500
gggggaggta cgggaccctg ttcacgatgg accgggtgct gaccccccca atggggactg 1560
tcatggatgt cctgaaggga gacaatcgct ttagcatgct ggtagctgcc atccagtctg 1620
caggactgac ggagaccctc aaccgggaag gagtctacac agtctttgct cccacaaatg 1680
aagccttccg agccctgcca ccaagagaac ggagcagact cttgggagat gccaaggaac 1740
ttgccaacat cctgaaatac cacattggtg atgaaatcct ggttagcgga ggcatcgggg 1800
ccctggtgcg gctaaagtct ctccaaggtg acaagctgga agtcagcttg aaaaacaatg 1860
tggtgagtgt caacaaggag cctgttgccg agcctgacat catggccaca aatggcgtgg 1920
tccatgtcat caccaatgtt ctgcagcctc cagccaacag acctcaggaa agaggggatg 1980

aacttgcaga ctctgcgctt gagatcttca aacaagcatc agcgttttcc agggcttccc 2040
agaggtctgt gcgactagcc cctgtctatc aaaagttatt agagaggatg aagcattagc 2100
ttgaagcact acaggaggaa tgcaccacgg cagctctccg ccaatttctc tcagatttcc 2160
acagagactg tttgaatgtt ttcaaaacca agtatcacac tttaatgtac atgggccgca 2220
ccataatgag atgtgagcct tgtgcatgtg ggggaggagg gagagagatg tactttttaa 2280
atcatgttcc ccctaaacat ggctgttaac ccactgcatg cagaaacttg gatgtcactg 2340
cctgacattc acttccagag aggacctatc ccaaatgtgg aattgactgc ctatgccaag 2400
tccctggaaa aggagcttca gtattgtggg gctcataaaa catgaatcaa gcaatccagc 2460
ctcatgggaa gtcctggcac agtttttgta aagcccttgc acagctggag aaatggcatc 2520
attataagct atgagttgaa atgttctgtc aaatgtgtct cacatctaca cgtggcttgg 2580
aggcttttat ggggccctgt ccaggtagaa aagaaatggt atgtagagct tagatttccc 2640
tattgtgaca gagccatggt gtgtttgtaa taataaaacc aaagaaacat a 2691

<210> 109
<211> 2807
<212> DNA
<213> Homo sapiens

<400> 109
caggttgggt tctgggacag gtgacccggc ggcggggcga ggcagctggc ggcgtcgcat 60
ggagggctct gggggcggtg cgggcgagcg ggcgccgctg ctgggcgcgc ggcgggcggc 120
ggcggccgcg gcggcggctg ggcgttcgcc gggccggcgc gcggcgtgcg gggccgtgct 180
gctgacggag ctgctggagc gcgccgcttt ctacggcatc acgtccaacc tggtgctatt 240
cctgaacggg gcgccgttct gctgggaggg cgcgcaggcc agcgaggcgc tgctgctctt 300
catgggcctc acctacctgg gctcgccgtt cggaggctgg ctggccgacg cgcggctggg 360
ccgggcgcgc gccatcctgc tgagcctggc gctctacctg ctgggcatgc tggccttccc 420
gctgctggcc gcgcccgcca cgcgagccgc gctctgcggt tccgcgcgcc tgctcaactg 480
cacggcgcct ggtcccgacg ccgccgcccg ctgctgctca ccggccacct tcgcggggct 540
ggtgctggtg ggcctgggcg tggccaccgt caaggccaac atcacgccct tcggcgccga 600
ccaggttaaa gatcgaggtc cggaagccac taggagattt tttaattggt tttattggag 660
cattaacctg ggagcgatcc tgtcgttagg tggcattgcc tatattcagc agaacgtcag 720
ctttgtcact ggttatgcga tccccactgt ctgcgtcggc cttgcttttg tggtcttcct 780
ctgtggccag agcgttttca tcaccaagcc tcctgatggc agtgccttca ccgacatgtt 840
caagatactg acgtattcct gctgttccca gaagcgaagt ggagagcgcc agagtaatgg 900
tgaaggcatt ggagtctttc agcaatcttc taaacaaagt ctgtttgatt catgtaagat 960
gtctcatggt gggccattta cagaagagaa agtggaagat gtgaaagctc tggtcaagat 1020
tgtccctgtt ttcttggctt tgatacctta ctggacagtg tatttccaaa tgcagacaac 1080
atatgtttta cagagtcttc atttgaggat tccagaaatt tcaaatatta caaccactcc 1140
tcacacgctc cctgcagcct ggctgaccat gtttgatgct gtgctcatcc tcctgctcat 1200
ccctctgaag gacaaactgg tcgatcccat tttgagaaga catggcctgc tcccatcctc 1260
cctgaagagg atcgccgtgg gcatgttctt tgtcatgtgc tcggcctttg ctgcaggaat 1320
tttggagagt aaaaggctga accttgttaa agagaaaacc attaatcaga ccatcggcaa 1380
cgtcgtctac catgctgccg atctgtcgct gtggtggcag gtgccgcagt acttgctgat 1440
tgggatcagc gagatctttg caagtatcgc aggcctggaa tttgcatact cagctgcccc 1500
caagtccatg cagagtgcca taatgggctt gttctttttc ttctctggcg tcgggtcgtt 1560
cgtgggttct ggactgctgg cactggtgtc tatcaaagcc atcggatgga tgagcagtca 1620
cacagacttt ggtaatatta acggctgcta tttgaactat tacttttttc ttctggctgc 1680
tattcaagga gctaccctcc tgcttttcct cattatttct gtgaaatatg accatcatcg 1740
agaccatcag cgatcaagag ccaatggcgt gcccaccagc aggagggcct gaccttcctg 1800
aggccatgtg cggtttctga ggctgacatg tcagtaactg actggggtgc actgagaaca 1860
ggcaagactt taaattccca taaaatgtct gacttcactg aaacttgcat gttgcctgga 1920
ttgatttctt ctttccctct atccaaagga gcttggtaag tgccttactg cagcgtgtct 1980
cctggcacgc tgggccctcc gggaggagag ctgcagattt cgagtatgtc gcttgtcatt 2040
caaggtctct gtgaatcctc tagctgggtt ccctttttta cagaaactca caaatggaga 2100
ttgcaaagtc ttggggaact ccacgtgtta gttggcatcc cagtttctta aacaaatagt 2160
atcacctgct tcccatagcc atatctcact gtaaaaaaaa aaaattaata aactgttact 2220
tatatttaag aaagtgagga tttttttttt ttaaagataa aagcatggtc agatgctgca 2280
aggattttac ataaatgcca tatttatggt ttccttcctg agaacagtct tgctcttgcc 2340
atgttctttg atttaggctg gtagtaaaca catttcatct gctgcttcaa aaagtactta 2400
ctttttaaac catcaacatt acttttcttt cttaaggcaa ggcatgcata agagtcattt 2460
gagaccatgt gtcccatctc aagccacaga gcaactcaca gggtacttca caccttacct 2520
agtcagagtg cttatatata gctttatttt ggtacgattg agactaaaga ctgatcatgg 2580
ttgtatgtaa ggaaaacatt cttttgaaca gaaatagtgt aattaaaaat aattgaaagt 2640

gttaaatgtg aacttgagct gtttgaccag ccacattttt gtattgttac tgtacgtgta 2700
tctggggctt ctccgtttgt taatactttt tctgtatttg ttgctgtatt tttggcataa 2760
ctttattata aaaaaaaaaa aaaaaaaaaa aaaaaaaaaa aaaaaaa 2807

<210> 110
<211> 1004
<212> DNA
<213> Homo sapiens

<400> 110
   gcctcgctcg ggcgcccagt ggtcctgccg cctggtctca cctcgccatg gttcgtctgc 60
   ctctgcagtg cgtcctctgg ggctgcttgc tgaccgctgt ccatccagaa ccacccactg 120
   catgcagaga aaaacagtac ctaataaaca gtcagtgctg ttctttgtgc cagccaggac 180
   agaaactggt gagtgactgc acagagttca ctgaaacgga atgccttcct tgcggtgaaa 240
   gcgaattcct agacacctgg aacagagaga cacactgcca ccagcacaaa tactgcgacc 300
   ccaacctagg gcttcgggtc cagcagaagg gcacctcaga aacagacacc atctgcacct 360
   gtgaagaagg ctggcactgt acgagtgagg cctgtgagag ctgtgtcctg caccgctcat 420
   gctcgcccgg ctttggggtc aagcagattg ctacaggggt ttctgatacc atctgcgagc 480
   cctgcccagt cggcttcttc tccaatgtgt catctgcttt cgaaaaatgt cacccttgga 540
   caagctgtga gaccaaagac ctggttgtgc aacaggcagg cacaaacaag actgatgttg 600
   tctgtggtcc ccaggatcgg ctgagagccc tggtggtgat ccccatcatc ttcgggatcc 660
   tgtttgccat cctcttggtg ctggtcttta tcaaaaaggt ggccaagaag ccaaccaata 720
   aggcccccca ccccaagcag gaaccccagg agatcaattt tcccgacgat cttcctggct 780
   ccaacactgc tgctccagtg caggagactt tacatggatg ccaaccggtc acccaggagg 840
   atggcaaaga gagtcgcatc tcagtgcagg agagacagtg aggctgcacc cacccaggag 900
   tgtggccacg tgggcaaaca ggcagttggc cagagagcct ggtgctgctg ctgcaggggt 960
   gcaggcagaa gcggggagct atgcccagtc agtgccagcc cctc 1004

<210> 111
<211> 4160
<212> DNA
<213> Homo sapiens

<400> 111
   gcgcttgcgg aggattgcgt tgacgagact cttatttatt gtcaccaacc tgtggtggaa 60
   tttgcagttg cacattggat ctgattcgcc ccgccccgaa tgacgcctgc ccggaggcag 120
   tgaaagtaca gccgcgccgc cccaagtcag cctggacaca taaatcagca cgcggccgga 180
   gaaccccgca atctctgcgc ccacaaaata caccgacgat gcccgatcta ctttaagggc 240
   tgaaacccac gggcctgaga gactataaga gcgttcccta ccgccatgga acaacgggga 300
   cagaacgccc cggccgcttc gggggcccgg aaaaggcacg gcccaggacc cagggaggcg 360
   cggggagcca ggcctgggct ccgggtcccc aagacccttg tgctcgttgt cgccgcggtc 420
   ctgctgttgg tctcagctga gtctgctctg atcacccaac aagacctagc tccccagcag 480
   agagcggccc cacaacaaaa gaggtccagc cctcagaggg gattgtgtcc acctggacac 540
   catatctcag aagacggtag agattgcatc tcctgcaaat atggacagga ctatagcact 600
   cactggaatg acctcctttt ctgcttgcgc tgcaccaggt gtgattcagg tgaagtggag 660
   ctaagtccct gcaccacgac cagaaacaca gtgtgtcagt gcgaagaagg caccttccgg 720
   gaagaagatt ctcctgagat gtgccggaag tgccgcacag ggtgtcccag agggatggtc 780
   aaggtcggtg attgtacacc ctggagtgac atcgaatgtg tccacaaaga atcaggtaca 840
   aagcacagtg gggaagcccc agctgtggag gagacggtga cctccagccc agggactcct 900
   gcctctccct gttctctctc aggcatcatc ataggagtca cagttgcagc cgtagtcttg 960
   attgtggctg tgtttgtttg caagtcttta ctgtggaaga aagtccttcc ttacctgaaa 1020
   ggcatctgct caggtggtgg tggggaccct gagcgtgtgg acagaagctc acaacgacct 1080
   ggggctgagg acaatgtcct caatgagatc gtgagtatct tgcagcccac ccaggtccct 1140
   gagcaggaaa tggaagtcca ggagccagca gagccaacag gtgtcaacat gttgtccccc 1200
   ggggagtcag agcatctgct ggaaccggca gaagctgaaa ggtctcagag gaggaggctg 1260
   ctggttccag caaatgaagg tgatcccact gagactctga gacagtgctt cgatgacttt 1320
   gcagacttgg tgccctttga ctcctgggag ccgctcatga ggaagttggg cctcatggac 1380
   aatgagataa aggtggctaa agctgaggca gcgggccaca gggacacctt gtacacgatg 1440
   ctgataaagt gggtcaacaa aaccgggcga gatgcctctg tccacaccct gctggatgcc 1500
   ttggagacgc tgggagagag acttgccaag cagaagattg aggaccactt gttgagctct 1560
   ggaaagttca tgtatctaga aggtaatgca gactctgcca tgtcctaagt gtgattctct 1620
   tcaggaagtc agaccttccc tggtttacct ttttctggaa aaaagcccaa ctggactcca 1680
   gtcagtagga aagtgccaca attgtcacat gaccggtact ggaagaaact ctcccatcca 1740
   acatcaccca gtggatggaa catcctgtaa cttttcactg cacttggcat tatttttata 1800
   agctgaatgt gataataagg acactatgga aatgtctgga tcattccgtt tgtgcgtact 1860
   ttgagatttg gtttgggatg tcattgtttt cacagcactt ttttatccta atgtaaatgc 1920
   tttatttatt tatttgggct acattgtaag atccatctac acagtcgttg tccgacttca 1980
   cttgatacta tatgatatga accttttttg ggtggggggt gcggggcagt tcactctgtc 2040
   tcccaggctg gagtgcaatg gtgcaatctt ggctcactat agccttgacc tctcaggctc 2100
   aagcgattct cccacctcag ccatccaaat agctgggacc acaggtgtgc accaccacgc 2160
   ccggctaatt ttttgtattt tgtctagata taggggctct ctatgttgct cagggtggtc 2220
   tcgaattcct ggactcaagc agtctgccca cctcagactc ccaaagcggt ggaattagag 2280
   gcgtgagccc ccatgcttgg ccttaccttt ctacttttat aattctgtat gttattattt 2340
   tatgaacatg aagaaacttt agtaaatgta cttgtttaca tagttatgtg aatagattag 2400
   ataaacataa aaggaggaga catacaatgg gggaagaaga agaagtcccc tgtaagatgt 2460
   cactgtctgg gttccagccc tccctcagat gtactttggc ttcaatgatt ggcaacttct 2520
   acaggggcca gtcttttgaa ctggacaacc ttacaagtat atgagtatta tttataggta 2580
   gttgtttaca tatgagtcgg gaccaaagag aactggatcc acgtgaagtc ctgtgtgtgg 2640
   ctggtcccta cctgggcagt ctcatttgca cccatagccc ccatctatgg acaggctggg 2700
   acagaggcag atgggttaga tcacacataa caatagggtc tatgtcatat cccaagtgaa 2760
   cttgagccct gtttgggctc aggagataga agacaaaatc tgtctcccac gtctgccatg 2820
   gcatcaaggg ggaagagtag atggtgcttg agaatggtgt gaaatggttg ccatctcagg 2880
   agtagatggc ccggctcact tctggttatc tgtcaccctg agcccatgag ctgcctttta 2940
   gggtacagat tgcctacttg aggaccttgg ccgctctgta agcatctgac tcatctcaga 3000
   aatgtcaatt cttaaacact gtggcaacag gacctagaat ggctgacgca ttaaggtttt 3060
   cttcttgtgt cctgttctat tattgtttta agacctcagt aaccatttca gcctctttcc 3120
   agcaaaccct tctccatagt atttcagtca tggaaggatc atttatgcag gtagtcattc 3180
   caggagtttt tggtcttttc tgtctcaagg cattgtgtgt tttgttccgg gactggtttg 3240
   ggtgggacaa agttagaatt gcctgaagat cacacattca gactgttgtg tctgtggagt 3300
   tttaggagtg gggggtgacc tttctggtct ttgcacttcc atcctctccc acttccatct 3360
   ggcatcccac gcgttgtccc ctgcacttct ggaaggcaca gggtgctgct gcctcctggt 3420
   ctttgccttt gctgggcctt ctgtgcagga cgctcagcct cagggctcag aaggtgccag 3480
   tccggtccca ggtcccttgt cccttccaca gaggccttcc tagaagatgc atctagagtg 3540
   tcagccttat cagtgtttaa gatttgtctt ttatttttaa tttttttgag acagaatctc 3600
   actctctcgc ccaggctgga gtgcaacggt acgatcttgg ctcagtgcaa cctccgcctc 3660
   ctgggttcaa gcgattctcg tgcctcagcc tccggagtag ctgggattgc aggcacccgc 3720
   caccacgcct ggctaatttt tgtattttta gtagagacgg ggtttcacca tgttggtcag 3780
   gctggtctcg aactcctgac ctcaggtgat ccaccttggc ctccgaaagt gctgggatta 3840
   caggcgtgag ccaccagcca ggccaagcta ttcttttaaa gtaagcttcc tgacgacatg 3900
   aaataattgg gggttttgtt gtttagttac attaggcttt gctatatccc caggccaaat 3960
   agcatgtgac acaggacagc catagtatag tgtgtcactc gtggttggtg tcctttcatg 4020
   cttctgccct gtcaaaggtc cctatttgaa atgtgttata atacaaacaa ggaagcacat 4080
   tgtgtacaaa atacttatgt atttatgaat ccatgaccaa attaaatatg aaaccttata 4140
   taaaaaaaaa aaaaaaaaaa 4160

<210> 112
<211> 4073
<212> DNA
<213> Homo sapiens

<400> 112
gcgcttgcgg aggattgcgt tgacgagact cttatttatt gtcaccaacc tgtggtggaa 60
tttgcagttg cacattggat ctgattcgcc ccgccccgaa tgacgcctgc ccggaggcag 120
tgaaagtaca gccgcgccgc cccaagtcag cctggacaca taaatcagca cgcggccgga 180
gaaccccgca atctctgcgc ccacaaaata caccgacgat gcccgatcta ctttaagggc 240
tgaaacccac gggcctgaga gactataaga gcgttcccta ccgccatgga acaacgggga 300
cagaacgccc cggccgcttc gggggcccgg aaaaggcacg gcccaggacc cagggaggcg 360
cggggagcca ggcctgggct ccgggtcccc aagacccttg tgctcgttgt cgccgcggtc 420
ctgctgttgg tctcagctga gtctgctctg atcacccaac aagacctagc tccccagcag 480
agagcggccc cacaacaaaa gaggtccagc ccctcagagg gattgtgtcc acctggacac 540
catatctcag aagacggtag agattgcatc tcctgcaaat atggacagga ctatagcact 600
cactggaatg acctcctttt ctgcttgcgc tgcaccaggt gtgattcagg tgaagtggag 660
ctaagtccct gcaccacgac cagaaacaca gtgtgtcagt gcgaagaagg caccttccgg 720
gaagaagatt ctcctgagat gtgccggaag tgccgcacag ggtgtcccag agggatggtc 780
aaggtcggtg attgtacacc ctggagtgac atcgaatgtg tccacaaaga atcaggcatc 840
atcataggag tcacagttgc agccgtagtc ttgattgtgg ctgtgtttgt ttgcaagtct 900

ttactgtgga agaaagtcct tccttacctg aaaggcatct gctcaggtgg tggtggggac 960
cctgagcgtg tggacagaag ctcacaacga cctggggctg aggacaatgt cctcaatgag 1020
atcgtgagta tcttgcagcc cacccaggtc cctgagcagg aaatggaagt ccaggagcca 1080
gcagagccaa caggtgtcaa catgttgtcc cccggggagt cagagcatct gctggaaccg 1140
gcagaagctg aaaggtctca gaggaggagg ctgctggttc cagcaaatga aggtgatccc 1200
actgagactc tgagacagtg cttcgatgac tttgcagact tggtgccctt tgactcctgg 1260
gagccgctca tgaggaagtt gggcctcatg gacaatgaga taaaggtggc taaagctgag 1320
gcagcgggcc acagggacac cttgtacacg atgctgataa agtgggtcaa caaaaccggg 1380
cgagatgcct ctgtccacac cctgctggat gccttggaga cgctgggaga gagacttgcc 1440
aagcagaaga ttgaggacca cttgttgagc tctggaaagt tcatgtatct agaaggtaat 1500
gcagactctg ccatgtccta agtgtgattc tcttcaggaa gtcagacctt ccctggttta 1560
ccttttttct ggaaaaagcc caactggact ccagtcagta ggaaagtgcc acaattgtca 1620
catgaccggt actggaagaa actctcccat ccaacatcac ccagtggatg gaacatcctg 1680
taacttttca ctgcacttgg cattattttt ataagctgaa tgtgataata aggacactat 1740
ggaaatgtct ggatcattcc gtttgtgcgt actttgagat ttggtttggg atgtcattgt 1800
tttcacagca cttttttatc ctaatgtaaa tgctttattt atttatttgg gctacattgt 1860
aagatccatc tacacagtcg ttgtccgact tcacttgata ctatatgata tgaacctttt 1920
ttgggtgggg ggtgcggggc agttcactct gtctcccagg ctggagtgca atggtgcaat 1980
cttggctcac tatagccttg acctctcagg ctcaagcgat tctcccacct cagccatcca 2040
aatagctggg accacaggtg tgcaccacca cgcccggcta attttttgta ttttgtctag 2100
atataggggc tctctatgtt gctcagggtg gtctcgaatt cctggactca agcagtctgc 2160
ccacctcaga ctcccaaagc ggtggaatta gaggcgtgag cccccatgct tggccttacc 2220
tttctacttt tataattctg tatgttatta ttttatgaac atgaagaaac tttagtaaat 2280
gtacttgttt acatagttat gtgaatagat tagataaaca taaaaggagg agacatacaa 2340
tgggggaaga agaagaagtc ccctgtaaga tgtcactgtc tgggttccag ccctccctca 2400
gatgtacttt ggcttcaatg attggcaact tctacagggg ccagtctttt gaactggaca 2460
accttacaag tatatgagta ttatttatag gtagttgttt acatatgagt cgggaccaaa 2520
gagaactgga tccacgtgaa gtcctgtgtg tggctggtcc ctacctgggc agtctcattt 2580
gcacccatag cccccatcta tggacaggct gggacagagg cagatgggtt agatcacaca 2640
taacaatagg gtctatgtca tatcccaagt gaacttgagc cctgtttggg ctcaggagat 2700
agaagacaaa atctgtctcc cacgtctgcc atggcatcaa gggggaagag tagatggtgc 2760
ttgagaatgg tgtgaaatgg ttgccatctc aggagtagat ggcccggctc acttctggtt 2820
atctgtcacc ctgagcccat gagctgcctt ttagggtaca gattgcctac ttgaggacct 2880
tggccgctct gtaagcatct gactcatctc agaaatgtca attcttaaac actgtggcaa 2940
caggacctag aatggctgac gcattaaggt tttcttcttg tgtcctgttc tattattgtt 3000
ttaagacctc agtaaccatt tcagcctctt tccagcaaac ccttctccat agtatttcag 3060
tcatggaagg atcatttatg caggtagtca ttccaggagt tttggtcttt ttctgtctca 3120
aggcattgtg tgttttgttc cgggactggt ttgggtggga caaagttaga attgcctgaa 3180
gatcacacat tcagactgtt gtgtctgtgg agttttagga gtggggggtg acctttctgg 3240
tctttgcact tccatcctct cccacttcca tctggcatcc cacgcgttgt cccctgcact 3300
tctggaaggc acagggtgct gctgcctcct ggtctttgcc tttgctgggc cttctgtgca 3360
ggacgctcag cctcagggct cagaaggtgc cagtccggtc ccaggtccct tgtcccttcc 3420
acagaggcct tcctagaaga tgcatctaga gtgtcagcct tatcagtgtt taagatttgt 3480
cttttatttt taattttttt gagacagaat ctcactctct cgcccaggct ggagtgcaac 3540
ggtacgatct tggctcagtg caacctccgc ctcctgggtt caagcgattc tcgtgcctca 3600
gcctccggag tagctgggat tgcaggcacc cgccaccacg cctggctaat ttttgtattt 3660
ttagtagaga cggggtttca ccatgttggt caggctggtc tcgaactcct gacctcaggt 3720
gatccacctt ggcctccgaa agtgctggga ttacaggcgt gagccaccag ccaggccaag 3780
ctattctttt aaagtaagct tcctgacgac atgaaataat tgggggtttt gttgtttagt 3840
tacattaggc tttgctatat ccccaggcca aatagcatgt gacacaggac agccatagta 3900
tagtgtgtca ctcgtggttg gtgtcctttc atgcttctgc cctgtcaaag gtccctattt 3960
gaaatgtgtt ataatacaaa caaggaagca cattgtgtac aaaatactta tgtatttatg 4020
aatccatgac caaattaaat atgaaacctt atataaaaaa aaaaaaaaaa aaa 4073

<210> 113
<211> 3015
<212> DNA
<213> Homo sapiens

<400> 113
   ccgaggagcg ctcgggctgt ctgcggaccc tgccgcgtgc aggggtcgcg gccggctgga 60
   gctgggagtg aggcggcgga ggagccaggt gaggaggagc caggaaggca gttggtggga 120
   agtccagctt gggtccctga gagctgtgag aaggagatgc ggctgctgct ggccctgttg 180
   ggggtcctgc tgagtgtgcc tgggcctcca gtcttgtccc tggaggcctc tgaggaagtg 240
   gagcttgagc cctgcctggc tcccagcctg gagcagcaag agcaggagct gacagtagcc 300
   cttgggcagc ctgtgcggct gtgctgtggg cgggctgagc gtggtggcca ctggtacaag 360
   gagggcagtc gcctggcacc tgctggccgt gtacggggct ggaggggccg cctagagatt 420
   gccagcttcc tacctgagga tgctggccgc tacctctgcc tggcacgagg ctccatgatc 480
   gtcctgcaga atctcacctt gattacaggt gactccttga cctccagcaa cgatgatgag 540
   gaccccaagt cccataggga cctctcgaat aggcacagtt acccccagca agcaccctac 600
   tggacacacc cccagcgcat ggagaagaaa ctgcatgcag tacctgcggg gaacaccgtc 660
   aagttccgct gtccagctgc aggcaacccc acgcccacca tccgctggct taaggatgga 720
   caggcctttc atggggagaa ccgcattgga ggcattcggc tgcgccatca gcactggagt 780
   ctcgtgatgg agagcgtggt gccctcggac cgcggcacat acacctgcct ggtagagaac 840
   gctgtgggca gcatccgcta taactacctg ctagatgtgc tggagcggtc cccgcaccgg 900
   cccatcctgc aggccgggct cccggccaac accacagccg tggtgggcag cgacgtggag 960
   ctgctgtgca aggtgtacag cgatgcccag ccccacatcc agtggctgaa gcacatcgtc 1020
   atcaacggca gcagcttcgg agccgacggt ttcccctatg tgcaagtcct aaagactgca 1080
   gacatcaata gctcagaggt ggaggtcctg tacctgcgga acgtgtcagc cgaggacgca 1140
   ggcgagtaca cctgcctcgc aggcaattcc atcggcctct cctaccagtc tgcctggctc 1200
   acggtgctgc cagaggagga ccccacatgg accgcagcag cgcccgaggc caggtatacg 1260
   gacatcatcc tgtacgcgtc gggctccctg gccttggctg tgctcctgct gctggccggg 1320
   ctgtatcgag ggcaggcgct ccacggccgg cacccccgcc cgcccgccac tgtgcagaag 1380
   ctctcccgct tccctctggc ccgacagttc tccctggagt caggctcttc cggcaagtca 1440
   agctcatccc tggtacgagg cgtgcgtctc tcctccagcg gccccgcctt gctcgccggc 1500
   ctcgtgagtc tagatctacc tctcgaccca ctatgggagt tcccccggga caggctggtg 1560
   cttgggaagc ccctaggcga gggctgcttt ggccaggtag tacgtgcaga ggcctttggc 1620
   atggaccctg cccggcctga ccaagccagc actgtggccg tcaagatgct caaagacaac 1680
   gcctctgaca aggacctggc cgacctggtc tcggagatgg aggtgatgaa gctgatcggc 1740
   cgacacaaga acatcatcaa cctgcttggt gtctgcaccc aggaagggcc cctgtacgtg 1800
   atcgtggagt gcgccgccaa gggaaacctg cgggagttcc tgcgggcccg gcgcccccca 1860
   ggccccgacc tcagccccga cggtcctcgg agcagtgagg ggccgctctc cttcccagtc 1920
   ctggtctcct gcgcctacca ggtggcccga ggcatgcagt atctggagtc ccggaagtgt 1980
   atccaccggg acctggctgc ccgcaatgtg ctggtgactg aggacaatgt gatgaagatt 2040
   gctgactttg ggctggcccg cggcgtccac cacattgact actataagaa aaccagcaac 2100
   ggccgcctgc ctgtgaagtg gatggcgccc gaggccttgt ttgaccgggt gtacacacac 2160
   cagagtgacg tgtggtcttt tgggatcctg ctatgggaga tcttcaccct cgggggctcc 2220
   ccgtatcctg gcatcccggt ggaggagctg ttctcgctgc tgcgggaggg acatcggatg 2280
   gaccgacccc cacactgccc cccagagctg tacgggctga tgcgtgagtg ctggcacgca 2340
   gcgccctccc agaggcctac cttcaagcag ctggtggagg cgctggacaa ggtcctgctg 2400
   gccgtctctg aggagtacct cgacctccgc ctgaccttcg gaccctattc cccctctggt 2460
   ggggacgcca gcagcacctg ctcctccagc gattctgtct tcagccacga ccccctgcca 2520
   ttgggatcca gctccttccc cttcgggtct ggggtgcaga catgagcaag gctcaaggct 2580
   gtgcaggcac ataggctggt ggccttgggc cttggggctc agccacagcc tgacacagtg 2640
   ctcgaccttg atagcatggg gcccctggcc cagagttgct gtgccgtgtc caagggccgt 2700
   gcccttgccc ttggagctgc cgtgcctgtg tcctgatggc ccaaatgtca gggttctgct 2760
   cggcttcttg gaccatggcg cttagtcccc atcccgggtt tggctgagcc tggctggaga 2820
   gctgctatgc taaacctcct gcctcccaat accagcagga ggttctgggc ctctgaaccc 2880
   cctttcccca cacctccccc tgctgctgct gccccagcgt cttgacggga gcattggccc 2940
   ctgagcccag agaagctgga agcctgccga aaacaggagc aaatggcgtt ttataaatta 3000
   tttttttgaa ataaa 3015

<210> 114
<211> 2895
<212> DNA
<213> Homo sapiens

<400> 114
   ccgaggagcg ctcgggctgt ctgcggaccc tgccgcgtgc aggggtcgcg gccggctgga 60
   gctgggagtg aggcggcgga ggagccaggt gaggaggagc caggaaggca gttggtggga 120
   agtccagctt gggtccctga gagctgtgag aaggagatgc ggctgctgct ggccctgttg 180
   ggggtcctgc tgagtgtgcc tgggcctcca gtcttgtccc tggaggcctc tgaggaagtg 240
   gagcttgagc cctgcctggc tcccagcctg gagcagcaag agcaggagct gacagtagcc 300
   cttgggcagc ctgtgcggct gtgctgtggg cgggctgagc gtggtggcca ctggtacaag 360
   gagggcagtc gcctggcacc tgctggccgt gtacggggct ggaggggccg cctagagatt 420
   gccagcttcc tacctgagga tgctggccgc tacctctgcc tggcacgagg ctccatgatc 480
   gtcctgcaga atctcacctt gattacaggt gactccttga cctccagcaa cgatgatgag 540
   gaccccaagt cccataggga cctctcgaat aggcacagtt acccccagca agcaccctac 600
   tggacacacc cccagcgcat ggagaagaaa ctgcatgcag tacctgcggg gaacaccgtc 660
   aagttccgct gtccagctgc aggcaacccc acgcccacca tccgctggct taaggatgga 720
   caggcctttc atggggagaa ccgcattgga ggcattcggc tgcgccatca gcactggagt 780
   ctcgtgatgg agagcgtggt gccctcggac cgcggcacat acacctgcct ggtagagaac 840
   gctgtgggca gcatccgcta taactacctg ctagatgtgc tggagcggtc cccgcaccgg 900
   cccatcctgc aggccgggct cccggccaac accacagccg tggtgggcag cgacgtggag 960
   ctgctgtgca aggtgtacag cgatgcccag ccccacatcc agtggctgaa gcacatcgtc 1020
   atcaacggca gcagcttcgg agccgacggt ttcccctatg tgcaagtcct aaagactgca 1080
   gacatcaata gctcagaggt ggaggtcctg tacctgcgga acgtgtcagc cgaggacgca 1140
   ggcgagtaca cctgcctcgc aggcaattcc atcggcctct cctaccagtc tgcctggctc 1200
   acggtgctgc caggtactgg gcgcatcccc cacctcacat gtgacagcct gactccagca 1260
   ggcagaacca agtctcccac tttgcagttc tccctggagt caggctcttc cggcaagtca 1320
   agctcatccc tggtacgagg cgtgcgtctc tcctccagcg gccccgcctt gctcgccggc 1380
   ctcgtgagtc tagatctacc tctcgaccca ctatgggagt tcccccggga caggctggtg 1440
   cttgggaagc ccctaggcga gggctgcttt ggccaggtag tacgtgcaga ggcctttggc 1500
   atggaccctg cccggcctga ccaagccagc actgtggccg tcaagatgct caaagacaac 1560
   gcctctgaca aggacctggc cgacctggtc tcggagatgg aggtgatgaa gctgatcggc 1620
   cgacacaaga acatcatcaa cctgcttggt gtctgcaccc aggaagggcc cctgtacgtg 1680
   atcgtggagt gcgccgccaa gggaaacctg cgggagttcc tgcgggcccg gcgcccccca 1740
   ggccccgacc tcagccccga cggtcctcgg agcagtgagg ggccgctctc cttcccagtc 1800
   ctggtctcct gcgcctacca ggtggcccga ggcatgcagt atctggagtc ccggaagtgt 1860
   atccaccggg acctggctgc ccgcaatgtg ctggtgactg aggacaatgt gatgaagatt 1920
   gctgactttg ggctggcccg cggcgtccac cacattgact actataagaa aaccagcaac 1980
   ggccgcctgc ctgtgaagtg gatggcgccc gaggccttgt ttgaccgggt gtacacacac 2040
   cagagtgacg tgtggtcttt tgggatcctg ctatgggaga tcttcaccct cgggggctcc 2100
   ccgtatcctg gcatcccggt ggaggagctg ttctcgctgc tgcgggaggg acatcggatg 2160
   gaccgacccc cacactgccc cccagagctg tacgggctga tgcgtgagtg ctggcacgca 2220
   gcgccctccc agaggcctac cttcaagcag ctggtggagg cgctggacaa ggtcctgctg 2280
   gccgtctctg aggagtacct cgacctccgc ctgaccttcg gaccctattc cccctctggt 2340
   ggggacgcca gcagcacctg ctcctccagc gattctgtct tcagccacga ccccctgcca 2400
   ttgggatcca gctccttccc cttcgggtct ggggtgcaga catgagcaag gctcaaggct 2460
   gtgcaggcac ataggctggt ggccttgggc cttggggctc agccacagcc tgacacagtg 2520
   ctcgaccttg atagcatggg gcccctggcc cagagttgct gtgccgtgtc caagggccgt 2580
   gcccttgccc ttggagctgc cgtgcctgtg tcctgatggc ccaaatgtca gggttctgct 2640
   cggcttcttg gaccatggcg cttagtcccc atcccgggtt tggctgagcc tggctggaga 2700
   gctgctatgc taaacctcct gcctcccaat accagcagga ggttctgggc ctctgaaccc 2760
   cctttcccca cacctccccc tgctgctgct gccccagcgt cttgacggga gcattggccc 2820
   ctgagcccag agaagctgga agcctgccga aaacaggagc aaatggcgtt ttataaatta 2880
   tttttttgaa ataaa 2895

<210> 115
<211> 5065
<212> DNA
<213> Homo sapiens

<400> 115
atgaggggac ttgggacttg cctggcgact ttggccggac ttttgctaac tgcggcgggc 60
gagacgttct caggtggctg cctctttgat gagccgtata gcacatgtgg atatagtcaa 120
tctgaaggtg atgacttcaa ttgggagcaa gtgaacacct tgactaaacc gacttctgat 180
ccatggatgc catcaggttc tctcatgctg gtgaatgcct ctgggagacc tgaggggcag 240
agagcccacc tgctcttacc ccaacttaag gaaaatgaca cccactgcat cgattttcac 300
tattttgtgt ccagcaagag taattctcct ccggggttac tcaatgtcta cgtgaaggtc 360
aataacgggc cactggggaa tcctatctgg aatatatctg gagacccaac acgtacatgg 420
aacagggcag aactggccat tagtactttc tggcctaact tttatcaggt gatttttgaa 480
gtgataactt ctggacatca aggctatctc gctatcgatg aggtgaaggt gttaggacat 540
ccatgtacca ggactcctca cttcctgcgg attcagaatg tggaagttaa tgctggccag 600
tttgctacct tccagtgcag tgccatcggc aggaccgtgg caggagacag gctctggtta 660
cagggcattg atgtgcgaga tgctcctctg aaggaaatca aggtgaccag ctcccgacgc 720
ttcattgctt catttaatgt tgtgaatacc accaaacgag atgctggaaa gtaccgctgc 780
atgattcgca ctgaaggagg tgttggaata tcaaactatg cagagttggt agttaaagaa 840
ccacccgttc ctattgcccc acctcagctc gcctctgtag gagccaccta cctgtggata 900

cagctcaacg ccaactccat caatggggat gggcccattg tggcccgaga ggtggagtac 960
tgcacggcca gtgggagctg gaatgaccgg cagccagtcg attccacgag ctataaaatt 1020
ggacaccttg acccagatac agaatatgag attagtgtgc tcctgaccag gccaggggag 1080
ggtggcactg gctctcctgg tccagctctc aggacaagaa caaagtgtgc tgatcccatg 1140
cgaggcccaa gaaaactaga agtagtggag gtcaaatctc ggcaaatcac tatccgctgg 1200
gagccatttg gatataatgt aactcgttgc cacagttata atctcactgt ccactactgt 1260
taccaagttg gaggacaaga acaagtgcga gaagaagtaa gctgggatac agaaaattca 1320
caccctcaac acacgatcac taacctgtca ccatacacca atgtcagtgt gaaactgatc 1380
ctcatgaacc cagagggccg gaaggaaagc caagaactca tagtgcagac agatgaagac 1440
ctcccaggtg ctgttcccac tgaatccata caaggaagta cctttgaaga gaagatattt 1500
cttcagtgga gagaaccaac tcaaacatat ggtgtaatca ctttatatga gatcacctac 1560
aaagcagtca gttcctttga cccagaaata gatttatcca atcagagtgg aagagtttca 1620
aagctgggaa atgaaaccca ttttctgttt tttggactgt atccggggac cacatactcc 1680
tttaccatcc gagctagcac agctaagggt tttggccctc cagcaacaaa ccagttcacc 1740
accaaaatat cagcaccctc tatgccagct tatgaacttg agacaccttt gaatcaaact 1800
gacaataccg tgacagtcat gctgaaacct gcccacagca gaggagcacc tgtcagtgtc 1860
tatcaaatag ttgttgagga agaacgtcct cgaagaacta aaaagacgac agaaatctta 1920
aagtgctacc cagtgccaat tcacttccag aatgcttctc tgctgaactc acagtactac 1980
tttgctgcag aatttcctgc agacagcctc caagctgcgc agccttttac aattggtgat 2040
aataagacat ataatggata ctggaacact ccccttctcc cctataaaag ctacagaatt 2100
tatttccaag ctgctagtag agccaatggg gaaaccaaaa tagactgtgt ccaagtggcc 2160
acaaaaggag ctgccactcc gaaaccagtc ccagaacccg agaaacagac agaccataca 2220
gttaaaattg ctggagtcat cgcgggcatc ttgctgttcg tgattatatt tcttggagtt 2280
gtgttggtaa tgaagaaaag gaaactggcc aagaagcgga aagagaccat gagcagcacc 2340
cgacaggaga tgactgtgat ggtgaactca atggacaaga gctatgctga gcagggcaca 2400
aactgcgacg aggctttctc attcatggac acgcacaatc tgaatgggag atctgtgtct 2460
tcaccatcgt ccttcacaat gaaaacaaat acactgagca catcggtgcc taattcctat 2520
tacccagatg aaacccacac aatggccagc gataccagca gcctggtgca gtcccatact 2580
tacaagaagc gagagccggc cgacgtgccc tatcagactg ggcagctcca ccccgccatc 2640
cgggtggcag acctccttca gcacatcaca cagatgaagt gtgcggaggg ctacggcttc 2700
aaggaggaat acgagagctt ctttgaaggg cagtctgcac catgggactc ggctaagaaa 2760
gatgagaaca gaatgaagaa cagatacggg aatatcattg catacgatca ttcccgagtg 2820
aggctgcaga caatagaagg agacacaaac tcagactata tcaatggcaa ttatatcgat 2880
ggttatcatc gacccaatca ttacattgct acccaaggcc caatgcagga aaccatctat 2940
gacttctgga ggatggtgtg gcacgaaaac actgcaagta tcatcatggt gaccaatctt 3000
gtggaagtgg gaagggtcaa atgctgcaaa tactggccag atgacacaga gatatataaa 3060
gacattaaag ttaccctaat agaaacagaa ctactggcag aatatgtgat aagaacattt 3120
gctgttgaaa agagaggtgt gcatgaaatc cgagagatca gacagtttca cttcactggc 3180
tggccggatc atggggtccc ctaccatgcc accggcctgc tgggattcgt gcggcaagtc 3240
aagtccaaga gcccgcccag tgcaggccca ctggtggtgc actgcagtgc tggtgcaggg 3300
aggactggct gtttcatcgt cattgatatc atgttggaca tggccgaaag ggaaggggtc 3360
gtagacatct acaactgcgt cagggagctg cggtcacgga gggtgaacat ggtgcaaaca 3420
gaggagcagt atgtgtttat ccacgatgcg atcctggaag cctgtctttg tggggacacc 3480
tctgtgcctg cttcccaagt taggtctctg tattatgaca tgaacaaact ggatccacag 3540
acaaactcaa gccagattaa agaggaattc cggacgctaa acatggtgac accaacgctg 3600
cgagtagagg actgcagcat cgcactgttg ccccggaacc atgagaaaaa ccggtgcatg 3660
gacatcctgc ccccagaccg ctgcctgccc ttcctcatca ccatcgatgg ggagagcagc 3720
aactacatca atgctgccct catggacagc tataaacagc cttcagcttt tatagtcacc 3780
cagcatcctt tgccaaacac agtgaaagac ttttggagac tggtcctgga ttatcactgc 3840
acatccgtag ttatgctaaa tgatgtggat cctgcccagt tgtgtccaca gtactggcca 3900
gaaaacggag tacacagaca cggccccatc caggtggaat ttgtctctgc tgacctggaa 3960
gaggacatca tcagcaggat attccgcatt tacaatgccg ccagacccca agatggatat 4020
cggatggtgc agcaattcca gttcctgggc tggccgatgt acagggacac accagtgtct 4080
aagcgctcct tcttgaagct cattcgccag gtggacaagt ggcaagagga atacaatggc 4140
ggggaaggcc cgaccgttgt gcactgcttg aacgggggag gccgcagtgg gacgttctgc 4200
gccatcagca tcgtatgtga gatgctccgg caccagagaa ccgtggatgt ctttcacgct 4260
gtgaagacac tgaggaacaa caagcccaac atggtcgacc tcctggatca gtacaagttc 4320
tgctacgagg tggccctgga atacttgaat tctggctgat ggtgtaaaca gctctgcaaa 4380
caatcccttt cataccacaa agccaagacg ttccatggta tttgtgcaaa agagatgaag 4440
acttctcaat atgcttattt tgctttgcat aattggctct ttttaagagc ccaagaaagt 4500
gtttctaaaa ttgcttgcac tgcccaatcc cagtaatgct gctgcctgac agaaacacac 4560
acacagccac agttgccaaa tcccgtactc cttgccaccg gctttctaga gcagcgtaga 4620
cagctggtaa actgaagagc acaactatat tcttatgaag gaatttgtac ctttggggta 4680

ttattttgtg gcccgtgacc ctcgttattg ttacagctga gtgtatgttt ttgttctgtg 4740
gagaatgcta tctggcatta tggtaatata ttattttagg taatatttgt actttaacat 4800
gttgcataat atatgcttat gtagctttcc aggactaaca gataaatgtg taatgaacaa 4860
agatatgttg tatgagtcgt cgtttctgtc agatttgtat tgtttccaag ggaaaagctt 4920
gggggaggac tcagttcaca aaatgcaaaa ctcaacgatc agattcacgg acccagagct 4980
tttccatgtg tttatattgt aaatattttt gatttcatca aattatttat tcattaaaag 5040
aaatttttgt gaaaaaaaaa aaaaa 5065

<210> 116
<211> 2527
<212> DNA
<213> Homo sapiens

<400> 116
ccagccctgg cgtgggccca gcccggccca ggcagcaatg gggttcctgc agctgctggt 60
cgtagcggtg ctggcatccg aacaccgggt ggctggtgca gccgaggtct tcgggaattc 120
cagcgagggt cttattgaat tttctgtggg gaaatttaga tacttcgagc tcaataggcc 180
ctttccagag gaagctattt tgcatgatat ttcaagcaat gtgacttttc ttattttcca 240
aatacactca cagtatcaga atacaactgt ttccttttct ccgactctcc tttccaattc 300
ctcggaaaca ggcactgcca gtggactggt tttcatcctt agaccagagc agagtacatg 360
cacttggtac ttggggactt caggcataca gcctgtccag aatatggcta tcctactctc 420
ctactcagaa agagatcctg tccctggagg ctgtaatttg gagttcgatt tagatattga 480
tcccaacatt tacttggagt ataatttctt tgaaacgact atcaagtttg ccccagcaaa 540
cctaggctat gcgagaggcg tagatccccc accatgtgac gctgggacag accaggactc 600
caggtggagg ttgcagtatg atgtctatca gtattttctg cctgagaatg acctcactga 660
ggagatgttg ctgaagcatc tgcagaggat ggtcagtgtg ccccaggtga aggccagtgc 720
tctcaaggtg gttaccctaa cagctaatga taagacaagt gtttccttct cctccctccc 780
gggacaaggt gtcatataca atgtcattgt ttgggacccg tttctaaata catctgctgc 840
ctacattcct gctcacacat acgcttgcag ctttgaggca ggagagggta gttgtgcttc 900
cctaggaaga gtgtcttcca aagtgttctt cactcttttt gccctgcttg gtttcttcat 960
ttgtttcttt ggacacagat tctggaaaac agaattattc ttcataggct ttatcatcat 1020
gggattcttc ttttatatac tgattacaag actgacacct atcaagtatg atgtgaatct 1080
gattctgaca gctgtcactg gaagcgtcgg tggaatgttc ttggtagctg tgtggtggcg 1140
atttggaatc ctctcgatct gcatgctctg tgttggacta gtgctggggt tcctcatctc 1200
gtcagtgact ttctttactc cactgggaaa cctaaagatt tttcatgatg atggtgtatt 1260
ctgggtcact ttctcttgca tagctatcct cattccagta gttttcatgg gctgcctaag 1320
aatactgaac atactgactt gtggagtcat tggctcctat tcggtggttt tagccattga 1380
cagttactgg tccacaagcc tttcctacat cactttgaac gtactcaaga gagcgctcaa 1440
caaggatttc cacagagctt tcacaaatgt gccttttcaa actaatgact tcattatcct 1500
ggcagtatgg ggcatgctgg ctgtaagtgg aattacgtta cagattcgaa gagagagagg 1560
acgaccgttc ttccctcccc acccatacaa gttatggaag caagagagag agcgccgagt 1620
gacaaacatt ctggacccta gctaccacat tcctccattg agagagaggc tctatggccg 1680
attaacccag attaaagggc tcttccagaa ggagcagcca gctggagaga gaacgccttt 1740
gcttctgtag atgcccaggg gcttggtcag tgtgcctcag ctttggagtt catgcctgga 1800
gtggttcaac agtctctggt gcaagtctaa taagagatca ggcatatata tctgttcttt 1860
gcataatatt atggtgccct tattgatata tggtaagggt gtactagggg attaggatga 1920
ttgtaagaga atgagaaaga tgaccaaaag gttggtggta gggaggcttt ttcttatttc 1980
caaatacttg agaaattacc ttttggttta caaatctatg atcaacttat tccattaaat 2040
agatacatta aaaaaattaa aaactgcaaa aaaaaaaaaa aaactggtgt ttctttttat 2100
aaccccttga aacaagtctc tcacctgagc ctgtctaaac tttcggaggg agtttattat 2160
tgagtcttta tctgtgacag tatttggaga tttagggatt tgatacttag gcctttgaat 2220
tttagaatac aaaaagagaa gcaagccaga catggtggct cacacctgta atcccaatac 2280
tgggaagcca aggtgggagt atcgcttgag cccaggagtt tgagaccgac atgggcaaca 2340
tgacaagacc ccatctctac aaaaaaaatt aaaaaattag ccaggcatgg tggcacatgc 2400
ctactcccag ctcccaagga gactgagatg ggaggatccc tggagccctg aagattgagg 2460
ctacagtgag ccttgattgt gtcactgcac tccagcttgg gtgacagaga ccctgtctcg 2520
agaaatt 2527

<210> 117
<211> 1724
<212> DNA
<213> Homo sapiens

<400> 117
ccttcatacc ggcccttccc ctcggctttg cctggacagc tcctgcctcc cgcagggccc 60
acctgtgtcc cccagcgccg ctccacccag caggcctgag cccctctctg ctgccagaca 120
ccccctgctg cccactctcc tgctgctcgg gttctgaggc acagcttgtc acaccgaggc 180
ggattctctt tctctttctc ttctggccca cagccgcagc aatggcgctg agttcctctg 240
ctggagttca tcctgctagc tgggttcccg agctgccggt ctgagcctga ggcatggagc 300
ctcctggaga ctgggggcct cctccctgga gatccacccc cagaaccgac gtcttgaggc 360
tggtgctgta tctcaccttc ctgggagccc cctgctacgc cccagctctg ccgtcctgca 420
aggaggacga gtacccagtg ggctccgagt gctgccccaa gtgcagtcca ggttatcgtg 480
tgaaggaggc ctgcggggag ctgacgggca cagtgtgtga accctgccct ccaggcacct 540
acattgccca cctcaatggc ctaagcaagt gtctgcagtg ccaaatgtgt gacccagcca 600
tgggcctgcg cgcgagccgg aactgctcca ggacagagaa cgccgtgtgt ggctgcagcc 660
caggccactt ctgcatcgtc caggacgggg accactgcgc cgcgtgccgc gcttacgcca 720
cctccagccc gggccagagg gtgcagaagg gaggcaccga gagtcaggac accctgtgtc 780
agaactgccc cccggggacc ttctctccca atgggaccct ggaggaatgt cagcaccaga 840
ccaagtgcag ctggctggtg acgaaggccg gagctgggac cagcagctcc cactgggtat 900
ggtggtttct ctcagggagc ctcgtcatcg tcattgtttg ctccacagtt ggcctaatca 960
tatgtgtgaa aagaagaaag ccaaggggtg atgtagtcaa ggtgatcgtc tccgtccagc 1020
ggaaaagaca ggaggcagaa ggtgaggcca cagtcattga ggccctgcag gcccctccgg 1080
acgtcaccac ggtggccgtg gaggagacaa taccctcatt cacggggagg agcccaaacc 1140
actgacccac agactctgca ccccgacgcc agagatacct ggagcgacgg ctgctgaaag 1200
aggctgtcca cctggcgaaa ccaccggagc ccggaggctt gggggctccg ccctgggctg 1260
gcttccgtct cctccagtgg agggagaggt ggggcccctg ctggggtaga gctggggacg 1320
ccacgtgcca ttcccatggg ccagtgaggg cctggggcct ctgttctgct gtggcctgag 1380
ctccccagag tcctgaggag gagcgccagt tgcccctcgc tcacagacca cacacccagc 1440
cctcctgggc cagcccagag ggcccttcag accccagctg tctgcgcgtc tgactcttgt 1500
ggcctcagca ggacaggccc cgggcactgc ctcacagcca aggctggact gggttggctg 1560
cagtgtggtg tttagtggat accacatcgg aagtgatttt ctaaattgga tttgaattcc 1620
ggtcctgtct tctatttgtc atgaaacagt gtatttgggg agatgctgtg ggaggatgta 1680
aatatcttgt ttctcctcaa aaaaaaaaaa aaaaaaaaaa aaaa 1724

<210> 118
<211> 3908
<212> DNA
<213> Homo sapiens

<400> 118
caggcaccac caggaccagc tgatcattcc agcccacagc aatggagcca catgactcct 60
cccacatgga ctctgagttc cgatacactc tcttcccgat tgtttacagc atcatctttg 120
tgctcggggt cattgctaat ggctacgtgc tgtgggtctt tgcccgcctg tacccttgca 180
agaaattcaa tgagataaag atcttcatgg tgaacctcac catggcggac atgctcttct 240
tgatcaccct gccactttgg attgtctact accaaaacca gggcaactgg atactcccca 300
aattcctgtg caacgtggct ggctgccttt tcttcatcaa cacctactgc tctgtggcct 360
tcctgggcgt catcacttat aaccgcttcc aggcagtaac tcggcccatc aagactgctc 420
aggccaacac ccgcaagcgt ggcatctctt tgtccttggt catctgggtg gccattgtgg 480
gagctgcatc ctacttcctc atcctggact ccaccaacac agtgcccgac agtgctggct 540
caggcaacgt cactcgctgc tttgagcatt acgagaaggg cagcgtgcca gtcctcatca 600
tccacatctt catcgtgttc agcttcttcc tggtcttcct catcatcctc ttctgcaacc 660
tggtcatcat ccgtaccttg ctcatgcagc cggtgcagca gcagcgcaac gctgaagtca 720
agcgccgggc gctgtggatg gtgtgcacgg tcttggcggt gttcatcatc tgcttcgtgc 780
cccaccacgt ggtgcagctg ccctggaccc ttgctgagct gggcttccag gacagcaaat 840
tccaccaggc cattaatgat gcacatcagg tcaccctctg cctccttagc accaactgtg 900
tcttagaccc tgttatctac tgtttcctca ccaagaagtt ccgcaagcac ctcaccgaaa 960
agttctacag catgcgcagt agccggaaat gctcccgggc caccacggat acggtcactg 1020
aagtggttgt gccattcaac cagatccctg gcaattccct caaaaattag tccctgcttc 1080
caggcctgaa gtcttctcct ccatgaacat catggactga gctgggggaa gaagggatat 1140
ctactgtggt ctgggcacca cctctgtggg cactggtggg ccattagatt tggaggctac 1200
ctcacctggg cagggatgat ggcagagcca ggctgttgga aaatccagaa ctcaaatgag 1260
ccccttcatc cgcctgtggg cgcatactac agtaactgtg actgatgact ttatcctgag 1320
tcccttaatc ttatggggcc ggaaggaatg tcagggccag gtgcagacct tgggggaaga 1380
ctttaaacca cctagttctc cccgatgggg catcggtcta aagctttggg ggagtgggcg 1440
cagtggctca cacctgtaat cccagcactt tgggaggccg aggtgggcag atcatgggtc 1500
aagagatcga gaccatcctg gccaacattg taaaacccca tctctactaa aacatacaaa 1560

aattagccgg gcatggtggc acacgcctgt agtcccagct actcaggagg ctgaggcagg 1620
agaatcgctt gaacctggga ggcagaggtt gcagtgaacc tagattgcac cattgcactc 1680
tagcctggca acagagcgag attccatctc aaaaaaaaaa aaaaaaaaaa aaaaaaagct 1740
ttgggcaact actctcaagt gatagaggta gactgggtta ggaaaggaca ctgccctcac 1800
tgtatggcac taccttgggg cagacttttg agctaagatg ttgccatcct ctctccaccc 1860
tccggcacaa ggtctttgtg acctttctca ggggaagggg cttgatggaa ccttctctga 1920
ctcatctggc aacctgtccc taatggtgac caaagaagat aaaacatgaa cagaccaaaa 1980
gctaggacaa gagcttgctt cccaagacag acgtgtctgt ctgcaaaggc cttgcctcta 2040
gtccagtcag ctctggggga gtgggaagga gatgcccacc tcctctttct gtctcaacaa 2100
tagtccctaa ttaagtacct tctgtggggc agccacccac tgagggtggg caaaggcccc 2160
ttgaaagcaa aattggggct gggctcagtg gctcatgcct ataatcccag cactttggga 2220
ggccaaggca ggcagatcac ctgaggtccg gagttcgaga ccagcctggc caacgtggtg 2280
aaacgccatc tccactaaga atacaaaaat atggctcatg cctgtaatcc cagtactttg 2340
ggaggctgag gtgggccgat catttgaagt taggagtttg agaccagcct gaccaatatg 2400
gtaaaacccc gtctctacta aaaatacaaa aaaattagcc tggcgtggtg gcgcatgcct 2460
gtagtcccat aggctgtagc tactgggagg aggcggagct tgcagtgagc cgagatcacg 2520
ccactgctct ccagcctggg cgacagagtg agactctgtg tcaaaaacaa acaaacaaac 2580
aaaaaaggca aaattggagc tgtccaggcc aggacacccc gatgtccagg ggcttccata 2640
cgaacaggca catgggctgg gaagtacatg aggcccctgg gtagaagggt ctgtcagttc 2700
tctcctccct tgccctctgg gagggtcctc ctaacatagc ttccaggagg tgggaggagc 2760
agttactgtc agcaggtgtc agccaggtgt cagcttctcc tggggatctc tagatgtctg 2820
cttgtgattt ttggcaagta tatgcaaatg agcctcctct cctgccctga gacaagtatc 2880
tgcagtgtga acctggcagc ctcagaccca aggggctcag aggaaacttc tctggtttct 2940
agagctctgt gctccttcag agaagtcttc cttccttcca gtcagtgtcc ctgtgaagct 3000
gggatactca tttcctgtgt accgggcaaa caccggattg ctgattttga gaaatgcctc 3060
tcgatggacc tgtaacctgc tggagtctgg gatggtagct gtgggctgga cttggctgat 3120
gggatgaccc ggtggctagt gcagcatcac acaagcctgg ttcaagtctt ggctgtgtca 3180
tttcctgctg agggaccagg cactgaattt cctacctctt agggtcatta cctatgaggt 3240
taaagctacc tcatgggatt gttatacgcc actaatgttg aggcagacac ctcttggcag 3300
ggtgactgct catcttagac cctccccttt tctgcgaatt tgggcccctt gatcctctga 3360
tgggagctga aaggatgaga ggtgggcatc tagatttagg gaggctgttc aggctttgca 3420
ggtcccttac ctgaacacat agaaaccctg gagctgtgac tgtgtccatg tgtgtgtgtt 3480
tgtctgtgtg tgttgcgggg gatgggcacc tgcatgaatg tggtagagaa aatggctctg 3540
ctcagaggga agatacgcat agcaaggcag ggaccagagg aatcacaggc gcctggagag 3600
cagccgggca ccgcctccag ggacctgccg gcttccctca gtcctccagg ggcccagcac 3660
tcttccttta ggccctgtga gcgtcccttg tcaggataca ttctctcatt ttgctgaagc 3720
tgatttgatt gggtgtctgt ttctcgcagc caaaagagct ctgaatgagg aaagtgcttc 3780
tgtgctaact ccccgcgtct cctgaatttc agtcattcat gtacccgcct cgaaattttt 3840
gcaatatctg tgtaccaact gtccatttac ttaataaaga agttttcttt aaattaagtc 3900
acttttaa 3908

<210> 119
<211> 1353
<212> DNA
<213> Homo sapiens

<400> 119
gcgggaggcg gcggcgcaga gcttggggct tccttggtcg cacccaccac ctgcctgccc 60
actggtcagc cttcagggac cctgagcacc gcctggtctc tttcctgtgg ccagcccaga 120
actgaagcgc tgcggcatgg cgcgcgcctg cctccaggcc gtcaagtacc tcatgttcgc 180
cttcaacctg ctcttctggc tgggaggctg tggcgtgctg ggtgtcggca tctggctggc 240
cgccacacag gggagcttcg ccacgctgtc ctcttccttc ccgtccctgt cggctgccaa 300
cctgctcatc atcaccggcg cctttgtcat ggccatcggc ttcgtgggct gcctgggtgc 360
catcaaggag aacaagtgcc tcctgctcac tttcttcctg ctgctgctgc tggtgttcct 420
gctggaggcc accatcgcca tcctcttctt cgcctacacg gacaagattg acaggtatgc 480
ccagcaagac ctgaagaaag gcttgcacct gtacggcacg cagggcaacg tgggcctcac 540
caacgcctgg agcatcatcc agaccgactt ccgctgctgt ggcgtctcca actacactga 600
ctggttcgag gtgtacaacg ccacgcgggt acctgactcc tgctgcttgg agttcagtga 660
gagctgtggg ctgcacgccc ccggcacctg gtggaaggcg ccgtgctacg agacggtgaa 720
ggtgtggctt caggagaacc tgctggctgt gggcatcttt gggctgtgca cggcgctggt 780
gcagatcctg ggcctgacct tcgccatgac catgtactgc caagtggtca aggcagacac 840
ctactgcgcg taggccgccc accgcccgct tctctgccaa aaggacgccc acggggagat 900
ggccgcaccc acagctgcct ttcccaccac cagcctcggt gctctgcccc atgctgggag 960

gagggaggga gggacaggtg cctggagccc ccggaaccct gtttctggaa ggccctagct 1020
caggtggctt cagggcctcc ggaccccccc tgggaggggt ggccacgtgc tggctgcgga 1080
acccagggca ggggtgggag gggcctccag cactttttat atttacgtat tctccaaagc 1140
agtgttcaca cgggagccag cctgtggccc ccagcctcct ggaaaacagg ttggcgctgg 1200
aggagccggg tcttggcatc ctggaggtgg ccccactggt cctggtgctc caggcggggc 1260
cgtggacccc tcacctacat tccatagtgg gcccgtgggg ctcctggtgc atcttaataa 1320
agtgtgagca gcaaaaaaaa aaaaaaaaaa aaa 1353

<210> 120
<211> 3917
<212> DNA
<213> Homo sapiens

<400> 120
tagtcggagc gaggtggcga gtcgctgagc ccgccgcggc cccgagagcg gctgcagccg 60
ccgccgccgg gaaggagagg gcgaggcgcg cccgagccgc cgccgccgcc gccaccgccg 120
ccgccgccac caccgccacc ggagtcgcgg gccagccggg cagcctccgc gggccccggc 180
cggggcgggg ggcgcgggcc acaggcccct gctccggccg tcgtttgcag accgcgggcg 240
ccgatgtcgc ccgcgccccg ttaggatgag tctcgggtcg ggcgaggagc cgccgcagcc 300
gccgccgccc gagccgcggg caggagcctc gggagccgcc gccgccgccg ccgccgcccg 360
gccgggcccc gacgccgccc gcgcgccccc gggcccccga cacacatgag attcttcagg 420
ctcactttca agtgcttcgt ggactgcttc tgactgcgcc gcccgcgccc cgcaccccgc 480
cgtccgcccg ccgccccgtc ccccggcccg gccgcccccc ggcccccggc cggcccgcgc 540
cctcggggcc ctccccggtg ccgccggtgc cccccgcctg accgccgccc cccgtgaggc 600
gccgcgaccc cggcccggcc gtgcggcccg ccggggccat ggcgaagaag agcgccgaga 660
acggcatcta tagcgtgtcc ggcgacgaga agaagggccc cctcatcgcg cccgggcccg 720
acggggcccc ggccaagggc gacggccccg tgggcctggg gacacccggc ggccgcctgg 780
ccgtgccgcc gcgcgagacc tggacgcgcc agatggactt catcatgtcg tgcgtgggct 840
tcgccgtggg cttgggcaac gtgtggcgct tcccctacct gtgctacaag aacggcggag 900
gtgtgttcct tattccctac gtcctgatcg ccctggttgg aggaatcccc attttcttct 960
tagagatctc gctgggccag ttcatgaagg ccggcagcat caatgtctgg aacatctgtc 1020
ccctgttcaa aggcctgggc tacgcctcca tggtgatcgt cttctactgc aacacctact 1080
acatcatggt gctggcctgg ggcttctatt acctggtcaa gtcctttacc accacgctgc 1140
cctgggccac atgtggccac acctggaaca ctcccgactg cgtggagatc ttccgccatg 1200
aagactgtgc caatgccagc ctggccaacc tcacctgtga ccagcttgct gaccgccggt 1260
cccctgtcat cgagttctgg gagaacaaag tcttgaggct gtctggggga ctggaggtgc 1320
caggggccct caactgggag gtgacccttt gtctgctggc ctgctgggtg ctggtctact 1380
tctgtgtctg gaagggggtc aaatccacgg gaaagatcgt gtacttcact gctacattcc 1440
cctacgtggt cctggtcgtg ctgctggtgc gtggagtgct gctgcctggc gccctggatg 1500
gcatcattta ctatctcaag cctgactggt caaagctggg gtcccctcag gtgtggatag 1560
atgcggggac ccagattttc ttttcttacg ccattggcct gggggccctc acagccctgg 1620
gcagctacaa ccgcttcaac aacaactgct acaaggacgc catcatcctg gctctcatca 1680
acagtgggac cagcttcttt gctggcttcg tggtcttctc catcctgggc ttcatggctg 1740
cagagcaggg cgtgcacatc tccaaggtgg cagagtcagg gccgggcctg gccttcatcg 1800
cctacccgcg ggctgtcacg ctgatgccag tggccccact ctgggctgcc ctgttcttct 1860
tcatgctgtt gctgcttggt ctcgacagcc agtttgtagg tgtggagggc ttcatcaccg 1920
gcctcctcga cctcctcccg gcctcctact acttccgttt ccaaagggag atctctgtgg 1980
ccctctgttg tgccctctgc tttgtcatcg atctctccat ggtgactgat ggcgggatgt 2040
acgtcttcca gctgtttgac tactactcgg ccagcggcac caccctgctc tggcaggcct 2100
tttgggagtg cgtggtggtg gcctgggtgt acggagctga ccgcttcatg gacgacattg 2160
cctgtatgat cgggtaccga ccttgcccct ggatgaaatg gtgctggtcc ttcttcaccc 2220
cgctggtctg catgggcatc ttcatcttca acgttgtgta ctacgagccg ctggtctaca 2280
acaacaccta cgtgtacccg tggtggggtg aggccatggg ctgggccttc gccctgtcct 2340
ccatgctgtg cgtgccgctg cacctcctgg gctgcctcct cagggccaag ggcaccatgg 2400
ctgagcgctg gcagcacctg acccagccca tctggggcct ccaccacttg gagtaccgag 2460
ctcaggacgc agatgtcagg ggcctgacca ccctgacccc agtgtccgag agcagcaagg 2520
tcgtcgtggt ggagagtgtc atgtgacaac tcagctcaca tcaccagctc acctctggta 2580
gccatagcag cccctgcttc agccccaccg cacccctcca gggggcctgc ctttccctga 2640
cacttttggg gtctgcctgg gggaggaggg gagaaagcac catgagtgct cactaaaaca 2700
actttttcca tttttaataa aacgccaaaa atatcacaac ccaccaaaaa tagatgcctc 2760
tccccctcca gccctagccg agctggtcct aggccccgcc tagtgcccca cccccaccca 2820
cagtgctgca ctcctcctgc ccctgccacg cccaccccct gcccacctct ccaggctctg 2880
ctctgcagca cacccgtggg tgacccctca ccccagaagc agcagtggca gcttgggaaa 2940

tgtgaggaag ggaaggaggg agagacggga gggaggagag agaggagaag ggaggcaggg 3000
gaggggcagc agaaccaagg caaatatttc agctgggcta tacccctctc cccatccctg 3060
ttatagaagc ttagagagcc agccagcaat ggaaccttct ggttcctgcg ccaatcgcca 3120
ccagtatcaa ttgtgtgagc ttgggtgcga gtgcacgcgt gcgtgagtac ggagagtata 3180
tatagatctc tatctcttag caaaggtgaa tgccagatgt aaatggcgcc tctgggcaaa 3240
ggaggcttgt attttgcaca ttttataaaa acttgagaga atgagatttc tgcttgtata 3300
tttctaaaaa gaggaaggag cccaaaccat cctctcctta ccactcccat ccctgtgagc 3360
cctaccttac ccctctgccc ctagccaagg agtgtgaatt tatagatcta actttcatag 3420
gcaaaacaaa agcttcgagc tgttgcgtgt gtgagtctgt tgtgtggatg tgcgtgtgtg 3480
gtccccagcc ccagactgga ttggaaaagt gcatggtggg ggcctcgggg ctgtccccac 3540
gctgtccctt tgccacaagt ctgtggggca agaggctgca atattccgtc ctgggtgtct 3600
gggctgctaa cctggcctgc tcaggcttcc caccctgtgc ggggcacacc cccaggaagg 3660
gaccctggac acggctccca cgtccaggct taaggtggat gcacttcccg cacctccagt 3720
cttctgtgta gcagctttaa cccacgtttg tctgtcacgt ccagtcccga gacggctgag 3780
tgaccccaag aaaggcttcc ccgacaccca gacagaggct gcagggctgg ggctgggtga 3840
gggtggcggg cctgcgggga cattctactg tgctaaaaag ccactgcaga catagcaata 3900
aaaacatgtc attttcc 3917

<210> 121
<211> 1372
<212> DNA
<213> Homo sapiens

<400> 121
gcacgagggc gcttttgtct ccggtgagtt ttgtggcggg aagcttctgc gctggtgctt 60
agtaaccgac tttcctccgg actcctgcac gacctgctcc tacagccggc gatccactcc 120
cggctgttcc cccggagggt ccagaggcct ttcagaagga gaaggcagct ctgtttctct 180
gcagaggagt agggtccttt cagccatgaa gcatgtgttg aacctctacc tgttaggtgt 240
ggtactgacc ctactctcca tcttcgttag agtgatggag tccctagaag gcttactaga 300
gagcccatcg cctgggacct cctggaccac cagaagccaa ctagccaaca cagagcccac 360
caagggcctt ccagaccatc catccagaag catgtgataa gacctccttc catactggcc 420
atattttgga acactgacct agacatgtcc agatgggagt cccattccta gcagacaagc 480
tgagcaccgt tgtaaccaga gaactattac taggccttga agaacctgtc taactggatg 540
ctcattgcct gggcaaggcc tgtttaggcc ggttgcggtg gctcatgcct gtaatcctag 600
cactttggga ggctgaggtg ggtggatcac ctgaggtcag gagttcgaga ccagcctcgc 660
caacatggcg aaaccccatc tctactaaaa atacaaaagt tagctgggtg tggtggcaga 720
ggcctgtaat cccagttcct tgggaggctg aggcgggaga attgcttgaa cccggggacg 780
gaggttgcag tgaaccgaga tcgcactgct gtacccagcc tgggccacag tgcaagactc 840
catctcaaaa aaaaaaagaa aagaaaaagc ctgtttaatg cacaggtgtg agtggattgc 900
ttatggctat gagataggtt gatctcgccc ttaccccggg gtctggtgta tgctgtgctt 960
tcctcagcag tatggctctg acatctctta gatgtcccaa cttcagctgt tgggagatgg 1020
tgatattttc aaccctactt cctaaacatc tgtctggggt tcctttagtc ttgaatgtct 1080
tatgctcaat tatttggtgt tgagcctctc ttccacaaga gctcctccat gtttggatag 1140
cagttgaaga ggttgtgtgg gtgggctgtt gggagtgagg atggagtgtt cagtgcccat 1200
ttctcatttt acattttaaa gtcgttcctc caacatagtg tgtattggtc tgaagggggt 1260
ggtgggatgc caaagcctgc tcaagttatg gacattgtgg ccaccatgtg gcttaaatga 1320
ttttttctaa ctaataaagt ggaatatata tttcaaaaaa aaaaaaaaaa aa 1372

<210> 122
<211> 1284
<212> DNA
<213> Homo sapiens

<400> 122
ggcacgaggg tctccgcctg caggtgcaga catctggagg agagagtcgg agagcagaaa 60
ccacttggct cccagacaat tcccctacag gctttgggcc tggaattgag gagaaagtga 120
gctaagttgg ggtggggtga gtccaaagaa gcacgggctg ggccaagcta agctgctctg 180
ggctgggctg atccctcccc actcaggggc gggaccccag gaggagggag aggacagagc 240
cactgcagag gaccagactg ggaaaacaac gatatggcag gagccagtct tggggcccgc 300
ttctaccggc agatcaaaag acatccgggg atcatcccga tgatcggctt aatctgcctg 360
ggcatgggca gcgctgcgct ttacttgctg cgactcgccc ttcgcagccc cgacgtctgc 420
tgggacagaa agaacaaccc ggagccctgg aaccgcctga gccccaatga ccaatacaag 480
ttccttgcag tttccactga ctataagaag ctgaagaagg accggccaga cttctaagcc 540

aggctgggct gccagtgcca tgcaagccac agccagccag cccatccact tcttccactc 600
ctccccgcag gccccaaggc atcactccgg ccaccctgtc ccgctactgc ttacacaggc 660
cgggttccca cgcagagggg aggctgctcc acccctactc tcctcccttg ctcccagcag 720
cggaagcgcc tctgaccctt ggcttgagtc ccacgtgggg gaggaggagg caggcagcac 780
cagcaggggt ccaccaagag cccagaccag cccctctgcc ctcctacccg ggcctcgaag 840
ggtgtggcac aggctacgtg ttgagcgtgg cctacgtgag ccaacaagaa gcaggggcct 900
ctgagtgcca agcgacgtgg cgggctccac gttagcccag gctctgagag ccagcccagg 960
ggcggcgctg ctcagcttgg gctggtccag ggcctgccca ggctggggca cctttgcctc 1020
ctgaggcgca gcgcactcct cccctgccca agcctactgc ctcccgctgc cgccagtacc 1080
ccctccagcc ccacacctgg gcctccccct gccactcccc tcccttgctc ccctctgtcc 1140
ccagggatca aacagaagca gccgtgggca aaatacaatt tcatttaaca aattgaaaaa 1200
aaaaaaaaaa aaaaaaaaaa aaaaaaaaaa aaaaaaaaaa aaaaaaaaaa aaaaaaaaaa 1260
aaaaaaaaaa aaaaaaaaaa aaaa 1284

<210> 123
<211> 584
<212> DNA
<213> Homo sapiens

<400> 123
tcttgactga aattctctcc tgatgctgag ccctgcaccc caccttcctt ttcctaacta 60
tgaattgatg gcaaagtcca ctcaaaacaa ccagttaagt gctcacgaga gagtagtcaa 120
gcacctccag aaagaaaccg ggtttttgtt cacatagcag gaagtgactc cctgggtggt 180
aatttatctt ggaaacacag gtagattggc agaaaaacgg gaacatgtag gtaccgcgat 240
gttggtgcat gtccattact ttgggatagg ctttctcagt ctttcctcaa atgatagttg 300
agccagtttt ccagtggcaa ttctgagtga cttgcgcttg tcttatggtg tggtcaaggg 360
acgttcagaa ctacggaaaa cttttactga aacagcgaag cagagtatac cggcatgaga 420
gggaagatga acactcacct atgtaccact ctttgacaat aaatatagta tttctcaaat 480
cagtctcaag actgatcctg tctcagaaca gaccattgtt ctctcatttc tctaatacgc 540
tgagaggaac actctgaata aaggctaagt ggtggagcat gcaa 584

<210> 124
<211> 4637
<212> DNA
<213> Homo sapiens

<400> 124
aggtgaacag gtcctcacgc ccagctccgc cccctcacgc gctctcgccg ggaccccgct 60
tccgctggca gccatgggcc ccggccccag ccgcgcgccc cgcgccccac gcctgatgct 120
ctgtgcgctc gccttgatgg tggcggccgg cggctgcgtc gtctccgcct tcaacctgga 180
tacccgattc ctggtagtga aggaggccgg gaacccgggc agcctcttcg gctactcggt 240
cgccctccat cggcagacag agcggcagca gcgctacctg ctcctggctg gtgccccccg 300
ggagctcgct gtgcccgatg gctacaccaa ccggactggt gctgtgtacc tgtgcccact 360
cactgcccac aaggatgact gtgagcggat gaacatcaca gtgaaaaatg accctggcca 420
tcacattatt gaggacatgt ggcttggagt gactgtggcc agccagggcc ctgcaggcag 480
agttctggtc tgtgcccacc gctacaccca ggtgctgtgg tcagggtcag aagaccagcg 540
gcgcatggtg ggcaagtgct acgtgcgagg caatgaccta gagctggact ccagtgatga 600
ctggcagacc taccacaacg agatgtgcaa tagcaacaca gactacctgg agacgggcat 660
gtgccagctg ggcaccagcg gtggcttcac ccagaacact gtgtacttcg gcgcccccgg 720
tgcctacaac tggaaaggaa acagctacat gattcagcgc aaggagtggg acttatctga 780
gtatagttac aaggacccag aggaccaagg aaacctctat attgggtaca cgatgcaggt 840
aggcagcttc atcctgcacc ccaaaaacat caccattgtg acaggtgccc cacggcaccg 900
acatatgggc gcggtgttct tgctgagcca ggaggcaggc ggagacctgc ggaggaggca 960
ggtgctggag ggctcgcagg tgggcgccta ttttggcagc gcaattgccc tggcagacct 1020
gaacaatgat gggtggcagg acctcctggt gggcgccccc tactacttcg agaggaaaga 1080
ggaagtaggg ggtgccatct atgtcttcat gaaccaggcg ggaacctcct tccctgctca 1140
cccctcactc cttcttcatg gccccagtgg ctctgccttt ggtttatctg tggccagcat 1200
tggtgacatc aaccaggatg gatttcagga tattgctgtg ggagctccgt ttgaaggctt 1260
gggcaaagtg tacatctatc acagtagctc taaggggctc cttagacagc cccagcaggt 1320
aatccatgga gagaagctgg gactgcctgg gttggccacc ttcggctatt ccctcagtgg 1380
gcagatggat gtggatgaga acttctaccc agaccttcta gtgggaagcc tgtcagacca 1440
cattgtgctg ctgcgggccc ggccagtcat caacatcgtc cacaagacct tggtgcccag 1500
gccagctgtg ctggaccctg cactttgcac ggccacctct tgtgtgcaag tggagctgtg 1560

ctttgcttac aaccagagtg ccgggaaccc caactacagg cgaaacatca ccctggccta 1620
cactctggag gctgacaggg accgccggcc gccccggctc cgctttgccg gcagtgagtc 1680
cgctgtcttc cacggcttct tctccatgcc cgagatgcgc tgccagaagc tggagctgct 1740
cctgatggac aacctccgtg acaaactccg ccccatcatc atctccatga actactcttt 1800
acctttgcgg atgcccgatc gcccccggct ggggctgcgg tccctggacg cctacccgat 1860
cctcaaccag gcacaggctc tggagaacca cactgaggtc cagttccaga aggagtgcgg 1920
gcctgacaac aagtgtgaga gcaacttgca gatgcgggca gccttcgtgt cagagcagca 1980
gcagaagctg agcaggctcc agtacagcag agacgtccgg aaattgctcc tgagcatcaa 2040
cgtgacgaac acccggacct cggagcgctc cggggaggac gcccacgagg cgctgctcac 2100
cctggtggtg cctcccgccc tgctgctgtc ctcagtgcgc ccccccgggg cctgccaagc 2160
taatgagacc atcttttgcg agctggggaa ccccttcaaa cggaaccaga ggatggagct 2220
gctcatcgcc tttgaggtca tcggggtgac cctgcacaca agggaccttc aggtgcagct 2280
gcagctctcc acgtcgagtc accaggacaa cctgtggccc atgatcctca ctctgctggt 2340
ggactataca ctccagacct cgcttagcat ggtaaatcac cggctacaaa gcttctttgg 2400
ggggacagtg atgggtgagt ctggcatgaa aactgtggag gatgtaggaa gccccctcaa 2460
gtatgaattc caggtgggcc caatggggga ggggctggtg ggcctgggga ccctggtcct 2520
aggtctggag tggccctacg aagtcagcaa tggcaagtgg ctgctgtatc ccacggagat 2580
caccgtccat ggcaatgggt cctggccctg ccgaccacct ggagacctta tcaaccctct 2640
caacctcact ctttctgacc ctggggacag gccatcatcc ccacagcgca ggcgccgaca 2700
gctggatcca gggggaggcc agggcccccc acctgtcact ctggctgctg ccaaaaaagc 2760
caagtctgag actgtgctga cctgtgccac agggcgtgcc cactgtgtgt ggctagagtg 2820
ccccatccct gatgcccccg ttgtcaccaa cgtgactgtg aaggcacgag tgtggaacag 2880
caccttcatc gaggattaca gagactttga ccgagtccgg gtaaatggct gggctaccct 2940
attcctccga accagcatcc ccaccatcaa catggagaac aagaccacgt ggttctctgt 3000
ggacattgac tcggagctgg tggaggagct gccggccgaa atcgagctgt ggctggtgct 3060
ggtggccgtg ggtgcagggc tgctgctgct ggggctgatc atcctcctgc tgtggaagtg 3120
cggcttcttc aagcgagccc gcactcgcgc cctgtatgaa gctaagaggc agaaggcgga 3180
gatgaagagc cagccgtcag agacagagag gctgaccgac gactactgag gggcagcccc 3240
cccgcccccg gcccacctgg tgtgacttct ttaagcggac ccgctattat cagatcatgc 3300
ccaagtacca cgcagtgcgg atccgggagg aggagcgcta cccacctcca gggagcaccc 3360
tgcccaccaa gaagcactgg gtgaccagct ggcagactcg ggaccaatac tactgacgtc 3420
ctccctgatc ccaccccctc ctcccccagt gtcccctttc ttcctattta tcataagtta 3480
tgcctctgac agtccacagg ggccaccacc tttggctggt agcagcaggc tcaggcacat 3540
acacctcgtc aagagcatgc acatgctgtc tggccctggg gatcttccca caggagggcc 3600
agcgctgtgg accttacaac gccgagtgca ctgcattcct gtgccctaga tgcacgtggg 3660
gcccactgct cgtggactgt gctggtgcat cacggatggt gcatgggctc gccgtgtctc 3720
agcctctgcc agcgccagcg ccaaaacaag ccaaagagcc tcccaccaga gccgggagga 3780
aaaggcccct gcaatgtggt gacacctccc ctttcacacc tggatccatc ttgagagcca 3840
cagtcactgg attgactttg ctgtcaaaac tactgacagg gagcagcccc cgggccgctg 3900
gctggtgggc ccccaattga cacccatgcc agagaggtgg ggatcctgcc taaggttgtc 3960
tacgggggca cttggaggac ctggcgtgct cagacccaac agcaaaggaa ctagaaagaa 4020
ggacccagaa ggcttgcttt cctgcatctc tgtgaagcct ctctccttgg ccacagactg 4080
aactcgcagg gagtgcagca ggaaggaaca aagacaggca aacggcaacg tagcctgggc 4140
tcactgtgct ggggcatggc gggatcctcc acagagagga ggggaccaat tctggacaga 4200
cagatgttgg gaggatacag aggagatgcc acttctcact caccactacc agccagcctc 4260
cagaaggccc cagagagacc ctgcaagacc acggagggag ccgacacttg aatgtagtaa 4320
taggcagggg gccctgccac cccatccagc cagaccccag ctgaaccatg cgtcaggggc 4380
ctagaggtgg agttcttagc tatccttggc tttctgtgcc agcctggctc tgcccctccc 4440
ccatgggctg tgtcctaagg cccatttgag aagctgaggc tagttccaaa aacctctcct 4500
gacccctgcc tgttggcagc ccactcccca gccccagccc cttccatggt actgtagcag 4560
gggaattccc tccccctcct tgtgccttct ttgtatatag gcttctcacc gcgaccaata 4620
aacagctccc agtttgt 4637

<210> 125
<211> 4495
<212> DNA
<213> Homo sapiens
<400> 125
aggtgaacag gtcctcacgc ccagctccgc cccctcacgc gctctcgccg ggaccccgct 60
tccgctggca gccatgggcc ccggccccag ccgcgcgccc cgcgccccac gcctgatgct 120
ctgtgcgctc gccttgatgg tggcggccgg cggctgcgtc gtctccgcct tcaacctgga 180
tacccgattc ctggtagtga aggaggccgg gaacccgggc agcctcttcg gctactcggt 240
cgccctccat cggcagacag agcggcagca gcgctacctg ctcctggctg gtgccccccg 300

ggagctcgct gtgcccgatg gctacaccaa ccggactggt gctgtgtacc tgtgcccact 360
cactgcccac aaggatgact gtgagcggat gaacatcaca gtgaaaaatg accctggcca 420
tcacattatt gaggacatgt ggcttggagt gactgtggcc agccagggcc ctgcaggcag 480
agttctggtc tgtgcccacc gctacaccca ggtgctgtgg tcagggtcag aagaccagcg 540
gcgcatggtg ggcaagtgct acgtgcgagg caatgaccta gagctggact ccagtgatga 600
ctggcagacc taccacaacg agatgtgcaa tagcaacaca gactacctgg agacgggcat 660
gtgccagctg ggcaccagcg gtggcttcac ccagaacact gtgtacttcg gcgcccccgg 720
tgcctacaac tggaaaggaa acagctacat gattcagcgc aaggagtggg acttatctga 780
gtatagttac aaggacccag aggaccaagg aaacctctat attgggtaca cgatgcaggt 840
aggcagcttc atcctgcacc ccaaaaacat caccattgtg acaggtgccc cacggcaccg 900
acatatgggc gcggtgttct tgctgagcca ggaggcaggc ggagacctgc ggaggaggca 960
ggtgctggag ggctcgcagg tgggcgccta ttttggcagc gcaattgccc tggcagacct 1020
gaacaatgat gggtggcagg acctcctggt gggcgccccc tactacttcg agaggaaaga 1080
ggaagtaggg ggtgccatct atgtcttcat gaaccaggcg ggaacctcct tccctgctca 1140
cccctcactc cttcttcatg gccccagtgg ctctgccttt ggtttatctg tggccagcat 1200
tggtgacatc aaccaggatg gatttcagga tattgctgtg ggagctccgt ttgaaggctt 1260
gggcaaagtg tacatctatc acagtagctc taaggggctc cttagacagc cccagcaggt 1320
aatccatgga gagaagctgg gactgcctgg gttggccacc ttcggctatt ccctcagtgg 1380
gcagatggat gtggatgaga acttctaccc agaccttcta gtgggaagcc tgtcagacca 1440
cattgtgctg ctgcgggccc ggccagtcat caacatcgtc cacaagacct tggtgcccag 1500
gccagctgtg ctggaccctg cactttgcac ggccacctct tgtgtgcaag tggagctgtg 1560
ctttgcttac aaccagagtg ccgggaaccc caactacagg cgaaacatca ccctggccta 1620
cactctggag gctgacaggg accgccggcc gccccggctc cgctttgccg gcagtgagtc 1680
cgctgtcttc cacggcttct tctccatgcc cgagatgcgc tgccagaagc tggagctgct 1740
cctgatggac aacctccgtg acaaactccg ccccatcatc atctccatga actactcttt 1800
acctttgcgg atgcccgatc gcccccggct ggggctgcgg tccctggacg cctacccgat 1860
cctcaaccag gcacaggctc tggagaacca cactgaggtc cagttccaga aggagtgcgg 1920
gcctgacaac aagtgtgaga gcaacttgca gatgcgggca gccttcgtgt cagagcagca 1980
gcagaagctg agcaggctcc agtacagcag agacgtccgg aaattgctcc tgagcatcaa 2040
cgtgacgaac acccggacct cggagcgctc cggggaggac gcccacgagg cgctgctcac 2100
cctggtggtg cctcccgccc tgctgctgtc ctcagtgcgc ccccccgggg cctgccaagc 2160
taatgagacc atcttttgcg agctggggaa ccccttcaaa cggaaccaga ggatggagct 2220
gctcatcgcc tttgaggtca tcggggtgac cctgcacaca agggaccttc aggtgcagct 2280
gcagctctcc acgtcgagtc accaggacaa cctgtggccc atgatcctca ctctgctggt 2340
ggactataca ctccagacct cgcttagcat ggtaaatcac cggctacaaa gcttctttgg 2400
ggggacagtg atgggtgagt ctggcatgaa aactgtggag gatgtaggaa gccccctcaa 2460
gtatgaattc caggtgggcc caatggggga ggggctggtg ggcctgggga ccctggtcct 2520
aggtctggag tggccctacg aagtcagcaa tggcaagtgg ctgctgtatc ccacggagat 2580
caccgtccat ggcaatgggt cctggccctg ccgaccacct ggagacctta tcaaccctct 2640
caacctcact ctttctgacc ctggggacag gccatcatcc ccacagcgca ggcgccgaca 2700
gctggatcca gggggaggcc agggcccccc acctgtcact ctggctgctg ccaaaaaagc 2760
caagtctgag actgtgctga cctgtgccac agggcgtgcc cactgtgtgt ggctagagtg 2820
ccccatccct gatgcccccg ttgtcaccaa cgtgactgtg aaggcacgag tgtggaacag 2880
caccttcatc gaggattaca gagactttga ccgagtccgg gtaaatggct gggctaccct 2940
attcctccga accagcatcc ccaccatcaa catggagaac aagaccacgt ggttctctgt 3000
ggacattgac tcggagctgg tggaggagct gccggccgaa atcgagctgt ggctggtgct 3060
ggtggccgtg ggtgcagggc tgctgctgct ggggctgatc atcctcctgc tgtggaagtg 3120
tgacttcttt aagcggaccc gctattatca gatcatgccc aagtaccacg cagtgcggat 3180
ccgggaggag gagcgctacc cacctccagg gagcaccctg cccaccaaga agcactgggt 3240
gaccagctgg cagactcggg accaatacta ctgacgtcct ccctgatccc accccctcct 3300
cccccagtgt cccctttctt cctatttatc ataagttatg cctctgacag tccacagggg 3360
ccaccacctt tggctggtag cagcaggctc aggcacatac acctcgtcaa gagcatgcac 3420
atgctgtctg gccctgggga tcttcccaca ggagggccag cgctgtggac cttacaacgc 3480
cgagtgcact gcattcctgt gccctagatg cacgtggggc ccactgctcg tggactgtgc 3540
tggtgcatca cggatggtgc atgggctcgc cgtgtctcag cctctgccag cgccagcgcc 3600
aaaacaagcc aaagagcctc ccaccagagc cgggaggaaa aggcccctgc aatgtggtga 3660
cacctcccct ttcacacctg gatccatctt gagagccaca gtcactggat tgactttgct 3720
gtcaaaacta ctgacaggga gcagcccccg ggccgctggc tggtgggccc ccaattgaca 3780
cccatgccag agaggtgggg atcctgccta aggttgtcta cgggggcact tggaggacct 3840
ggcgtgctca gacccaacag caaaggaact agaaagaagg acccagaagg cttgctttcc 3900
tgcatctctg tgaagcctct ctccttggcc acagactgaa ctcgcaggga gtgcagcagg 3960
aaggaacaaa gacaggcaaa cggcaacgta gcctgggctc actgtgctgg ggcatggcgg 4020
gatcctccac agagaggagg ggaccaattc tggacagaca gatgttggga ggatacagag 4080

gagatgccac ttctcactca ccactaccag ccagcctcca gaaggcccca gagagaccct 4140
gcaagaccac ggagggagcc gacacttgaa tgtagtaata ggcagggggc cctgccaccc 4200
catccagcca gaccccagct gaaccatgcg tcaggggcct agaggtggag ttcttagcta 4260
tccttggctt tctgtgccag cctggctctg cccctccccc atgggctgtg tcctaaggcc 4320
catttgagaa gctgaggcta gttccaaaaa cctctcctga cccctgcctg ttggcagccc 4380
actccccagc cccagcccct tccatggtac tgtagcaggg gaattccctc cccctccttg 4440
tgccttcttt gtatataggc ttctcaccgc gaccaataaa cagctcccag tttgt 4495

<210> 126
<211> 5176
<212> DNA
<213> Homo sapiens

<400> 126
ctccggcgcc cgctctgccc gccgctgggt ccgaccgcgc tcgccttcct tgcagccgcg 60
cctcggcccc atggacgccc tgtgcggttc cggggagctc ggctccaagt tctgggactc 120
caacctgtct gtgcacacag aaaacccgga cctcactccc tgcttccaga actccctgct 180
ggcctgggtg ccctgcatct acctgtgggt cgccctgccc tgctacttgc tctacctgcg 240
gcaccattgt cgtggctaca tcatcctctc ccacctgtcc aagctcaaga tggtcctggg 300
tgtcctgctg tggtgcgtct cctgggcgga ccttttttac tccttccatg gcctggtcca 360
tggccgggcc cctgcccctg ttttctttgt cacccccttg gtggtggggg tcaccatgct 420
gctggccacc ctgctgatac agtatgagcg gctgcagggc gtacagtctt cgggggtcct 480
cattatcttc tggttcctgt gtgtggtctg cgccatcgtc ccattccgct ccaagatcct 540
tttagccaag gcagagggtg agatctcaga ccccttccgc ttcaccacct tctacatcca 600
ctttgccctg gtactctctg ccctcatctt ggcctgcttc agggagaaac ctccattttt 660
ctccgcaaag aatgtcgacc ctaaccccta ccctgagacc agcgctggct ttctctcccg 720
cctgtttttc tggtggttca caaagatggc catctatggc taccggcatc ccctggagga 780
gaaggacctc tggtccctaa aggaagagga cagatcccag atggtggtgc agcagctgct 840
ggaggcatgg aggaagcagg aaaagcagac ggcacgacac aaggcttcag cagcacctgg 900
gaaaaatgcc tccggcgagg acgaggtgct gctgggtgcc cggcccaggc cccggaagcc 960
ctccttcctg aaggccctgc tggccacctt cggctccagc ttcctcatca gtgcctgctt 1020
caagcttatc caggacctgc tctccttcat caatccacag ctgctcagca tcctgatcag 1080
gtttatctcc aaccccatgg ccccctcctg gtggggcttc ctggtggctg ggctgatgtt 1140
cctgtgctcc atgatgcagt cgctgatctt acaacactat taccactaca tctttgtgac 1200
tggggtgaag tttcgtactg ggatcatggg tgtcatctac aggaaggctc tggttatcac 1260
caactcagtc aaacgtgcgt ccactgtggg ggaaattgtc aacctcatgt cagtggatgc 1320
ccagcgcttc atggaccttg cccccttcct caatctgctg tggtcagcac ccctgcagat 1380
catcctggcg atctacttcc tctggcagaa cctaggtccc tctgtcctgg ctggagtcgc 1440
tttcatggtc ttgctgattc cactcaacgg agctgtggcc gtgaagatgc gcgccttcca 1500
ggtaaagcaa atgaaattga aggactcgcg catcaagctg atgagtgaga tcctgaacgg 1560
catcaaggtg ctgaagctgt acgcctggga gcccagcttc ctgaagcagg tggagggcat 1620
caggcagggt gagctccagc tgctgcgcac ggcggcctac ctccacacca caaccacctt 1680
cacctggatg tgcagcccct tcctggtgac cctgatcacc ctctgggtgt acgtgtacgt 1740
ggacccaaac aatgtgctgg acgccgagaa ggcctttgtg tctgtgtcct tgtttaatat 1800
cttaagactt cccctcaaca tgctgcccca gttaatcagc aacctgactc aggccagtgt 1860
gtctctgaaa cggatccagc aattcctgag ccaagaggaa cttgaccccc agagtgtgga 1920
aagaaagacc atctccccag gctatgccat caccatacac agtggcacct tcacctgggc 1980
ccaggacctg ccccccactc tgcacagcct agacatccag gtcccgaaag gggcactggt 2040
ggccgtggtg gggcctgtgg gctgtgggaa gtcctccctg gtgtctgccc tgctgggaga 2100
gatggagaag ctagaaggca aagtgcacat gaagggctcc gtggcctatg tgccccagca 2160
ggcatggatc cagaactgca ctcttcagga aaacgtgctt ttcggcaaag ccctgaaccc 2220
caagcgctac cagcagactc tggaggcctg tgccttgcta gctgacctgg agatgctgcc 2280
tggtggggat cagacagaga ttggagagaa gggcattaac ctgtctgggg gccagcggca 2340
gcgggtcagt ctggctcgag ctgtttacag tgatgccgat attttcttgc tggatgaccc 2400
actgtccgcg gtggactctc atgtggccaa gcacatcttt gaccacgtca tcgggccaga 2460
aggcgtgctg gcaggcaaga cgcgagtgct ggtgacgcac ggcattagct tcctgcccca 2520
gacagacttc atcattgtgc tagctgatgg acaggtgtct gagatgggcc cgtacccagc 2580
cctgctgcag cgcaacggct cctttgccaa ctttctctgc aactatgccc ccgatgagga 2640
ccaagggcac ctggaggaca gctggaccgc gttggaaggt gcagaggata aggaggcact 2700
gctgattgaa gacacactca gcaaccacac ggatctgaca gacaatgatc cagtcaccta 2760
tgtggtccag aagcagttta tgagacagct gagtgccctg tcctcagatg gggagggaca 2820
gggtcggcct gtaccccgga ggcacctggg tccatcagag aaggtgcagg tgacagaggc 2880
gaaggcagat ggggcactga cccaggagga gaaagcagcc attggcactg tggagctcag 2940

tgtgttctgg gattatgcca aggccgtggg gctctgtacc acgctggcca tctgtctcct 3000
gtatgtgggt caaagtgcgg ctgccattgg agccaatgtg tggctcagtg cctggacaaa 3060
tgatgccatg gcagacagta gacagaacaa cacttccctg aggctgggcg tctatgctgc 3120
tttaggaatt ctgcaagggt tcttggtgat gctggcagcc atggccatgg cagcgggtgg 3180
catccaggct gcccgtgtgt tgcaccaggc actgctgcac aacaagatac gctcgccaca 3240
gtccttcttt gacaccacac catcaggccg catcctgaac tgcttctcca aggacatcta 3300
tgtcgttgat gaggttctgg cccctgtcat cctcatgctg ctcaattcct tcttcaacgc 3360
catctccact cttgtggtca tcatggccag cacgccgctc ttcactgtgg tcatcctgcc 3420
cctggctgtg ctctacacct tagtgcagcg cttctatgca gccacatcac ggcaactgaa 3480
gcggctggaa tcagtcagcc gctcacctat ctactcccac ttttcggaga cagtgactgg 3540
tgccagtgtc atccgggcct acaaccgcag ccgggatttt gagatcatca gtgatactaa 3600
ggtggatgcc aaccagagaa gctgctaccc ctacatcatc tccaaccggt ggctgagcat 3660
cggagtggag ttcgtgggga actgcgtggt gctctttgct gcactatttg ccgtcatcgg 3720
gaggagcagc ctgaacccgg ggctggtggg cctttctgtg tcctactcct tgcaggtgac 3780
atttgctctg aactggatga tacgaatgat gtcagatttg gaatctaaca tcgtggctgt 3840
ggagagggtc aaggagtact ccaagacaga gacagaggcg ccctgggtgg tggaaggcag 3900
ccgccctccc gaaggttggc cccacgtggg ggaggtggag ttccggaatt attctgtgcg 3960
ctaccggccg ggcctagacc tggtgctgag agacctgagt ctgcatgtgc acggtggcga 4020
gaaggtgggg atcgtgggcc gcactggggc tggcaagtct tccatgaccc tttgcctgtt 4080
ccgcatcctg gaggcggcaa agggtgaaat ccgcattgat ggcctcaatg tggcagacat 4140
cggcctccat gacctgcgct ctcagctgac catcatcccg caggacccca tcctgttctc 4200
ggggaccctg cgcatgaacc tggacccctt cggcagctac tcagaggagg acatttggtg 4260
ggctttggag ctgtcccacc tgcacacgtt tgtgagctcc cagccggcag gcctggactt 4320
ccagtgctca gagggcgggg agaatctcag cgtgggccag aggcagctcg tgtgcctggc 4380
ccgagccctg ctccgcaaga gccgcatcct ggttttagac gaggccacag ctgccatcga 4440
cctggagact gacaacctca tccaggctac catccgcacc cagtttgata cctgcactgt 4500
cctgaccatc gcacaccggc ttaacactat catggactac accagggtcc tggtcctgga 4560
caaaggagta gtagctgaat ttgattctcc agccaacctc attgcagcta gaggcatctt 4620
ctacgggatg gccagagatg ctggacttgc ctaaaatata ttcctgagat ttcctcctgg 4680
cctttcctgg ttttcatcag gaaggaaatg acaccaaata tgtccgcaga atggacttga 4740
tagcaaacac tgggggcacc ttaagatttt gcacctgtaa agtgccttac agggtaactg 4800
tgctgaatgc tttagatgag gaaatgatcc ccaagtggtg aatgacacgc ctaaggtcac 4860
agctagtttg agccagttag actagtcccc cggtctcccg attcccaact gagtgttatt 4920
tgcacactgc actgttttca aataacgatt ttatgaaatg acctctgtcc tccctctgat 4980
ttttcatatt ttcctaaagt ttcgtttctg ttttttaata aaaagctttt tcctcctgga 5040
acagaagaca gctgctgggt caggccaccc ctaggaactc agtcctgtac tctggggtgc 5100
tgcctgaatc cattaaaaat gggagtactg atgaaataaa actacatggt caacagtaaa 5160
aaaaaaaaaa aaaaaa 5176

<210> 127
<211> 1158
<212> DNA
<213> Homo sapiens

<400> 127
gtagtccttt gttacatgca tgagtcagtg aacagggaat gggtgaatga catttgtggg 60
taggttattt ctagaagtta ggtgggcagc tcggaaggca gatgcacttc tacagactat 120
tccttggggc cacacgtagg ttcttgaatc ccgaatggaa aggggagatt gataactggt 180
gtgtttatgt tcttacaagt cttctgcctt ttaaaatcca gtcccaggac atcaaagctc 240
tgcagaaaga actcgagcaa tttgccaagc tcctgaagca gaagaggatc accctgggat 300
atacacaggc cgatgtgggg ctcaccctgg gggttctatt tgggaaggta ttcagccaaa 360
cgaccatctg ccgctttgag gctctgcagc ttagcttcaa gaacatgtgt aagctgcggc 420
ccttgctgca gaagtgggtg gaggaagctg acaacaatga aaatcttcag gagatatgca 480
aagcagaaac cctcgtgcag gcccgaaaga gaaagcgaac cagtatcgag aaccgagtga 540
gaggcaacct ggagaatttg ttcctgcagt gcccgaaacc cacactgcag cagatcagcc 600
acatcgccca gcagcttggg ctcgagaagg atgtggtccg agtgtggttc tgtaaccggc 660
gccagaaggg caagcgatca agcagcgact atgcacaacg agaggatttt gaggctgctg 720
ggtctccttt ctcaggggga ccagtgtcct ttcctctggc cccagggccc cattttggtg 780
ccccaggcta tgggagccct cacttcactg cactgtactc ctcggtccct ttccctgagg 840
gggaagcctt tccccctgtc tctgtcacca ctctgggctc tcccttgcat tcaaactgag 900
gtgcctgcct gcccttctag gaatggggga cagggggagg ggaggagcta gggaaagaaa 960
acctggagtt tgtgccaggg tttttggatt aagttcttca ttcactaagg aaggaattgg 1020
gaacacaaag ggtgggggca ggggagtttg gggcaactgg ttggagggaa ggtgaagttc 1080

aatgatgctc ttgattttaa tcccacatca tgtatcactt ttttcttaaa taaagaagct 1140
tgggacacag tagataga 1158

<210> 128
<211> 2068
<212> DNA
<213> Homo sapiens

<400> 128
aaataagtat atctgtcaaa aatcatattt ttatgagatg tgtcaatact ggtctcgtgt 60
catttaggct acttggaaaa aagataaaaa aaatcctgtt tggctccaaa aaggaaaaat 120
cagcccctcc tgcatgagtg ggagctgcaa ccctttagaa ctgataatca caaacccctc 180
agaaccaaag tgaaatgaag gaaaatatgt aacattaggc attgatggaa gaggactaga 240
tcctagtgta agcatcctaa taaaaggagg ggttcaaaga tgctctccag aaccagtatt 300
tcagacttcc tatgataaac taaatgtgcc agtaccagag actccaggaa aaaccagaaa 360
tttgtttttg caattagccg agcatgtagt ccagtcttta aatgtcaatt catgttatgt 420
ttgtggaaaa actgtagtaa gagtttccat aagaagaact tccataagaa gcccaagaat 480
tagttcctac agacccagtt cctgatgaat tcccagccca aaagaaccac cctgacaatt 540
tttaggttct aaaagtctga attattagac agtattgcat agctagagaa ggaaaaggat 600
tcactcatcc tataaggcgg cttagttgtc ttaggcaaaa gctgtataat ggtaccacaa 660
atacagttac atggtggagt tccaattaca cagaaagaga tccattcagt caatttccaa 720
ggttgcagac tgcttgggcc cacccagaat tccaccggga ctggacggcc cccaccaggt 780
tatactggat atgtgggcac agagcttatg ctaagctgcc tgatcagtgg acaggtagct 840
gtgtaattga caccattaaa ccatctttct tcttactgcc cataaaagca ggggaacttc 900
tgggcttccc agtctatgct tcccgcaaaa aacaaagcat agccataggt gattggaaag 960
atgataaatg gccccctgaa agaatcatac aatactatgg acccaccact tgggcacaag 1020
atgactcttg gggatatcgg acccccatct acatgctcaa cggaatcata cggttacaag 1080
ctgttttcga aattattact aatacaaccg gtcaagcctt gactgttctt gcccagcagg 1140
agactctgat gagaaatgct atctatcaaa atagactagc tcttgactac ttgctagcag 1200
ctgaaagaga agtttgtgaa aaatttaact ttactaattg ttttctacac acaggtgatc 1260
aagggcaagt agttaaaaat atagttaaag atataacaaa actggcacat gtacccatac 1320
aagtgtggca cggactcaat ccaggatcca tgtttaaaaa ttagttccca gcaataggaa 1380
gatttaaaac tcttataata ggagttataa tagtaataaa aacctgctta ctgctccctt 1440
gtttactact tttacttcta caaatgataa aaagcttcat tgctacctta gttcaccaaa 1500
atgcttcagc acaagtgtac tatatgaatc actatcaatc tattgcacaa aaagacataa 1560
gtagcaaaaa taagagtgag aactcccact aataaaaagt gagagtctca aagggggaaa 1620
atgaggaaag agagcgaccc tctcatattg ttctatattg ttttatactc agtacctgtt 1680
ttaaggaaaa aacaacaagg aagtaaaatc aaagacaggc agcccagcgc caggcccaaa 1740
accaggcctg ggcctgcctg gcctaaaccc agtagttaaa aatcaactca taacttagaa 1800
actgatgtta ttcatagatt ccagacattg tatagaagaa cattgtgtaa ctccctgccc 1860
tgttctgttt ctctctgact gccggtgcat gcagcccctg tcacgtaccg cctgcttgct 1920
caaatcaatc acgacccttt catgtgaaat gtttagtgtt gtgagccctt aaaagggaca 1980
gaaatcgtgc actcggggag ctcagatttt aaggcagtag tttgctgatg ctcccagctg 2040
aataaagccc ttccttctac aaaaaaaa 2068

<210> 129
<211> 795
<212> DNA
<213> Homo sapiens
<400> 129
tttttttttt tttttttttt tttcctgtaa cactttatgt ttattagcag tctggtttta 60
gcctaaaagt cacaactttt ttggaagttt tccctaacca gtcaccccag attaggcaca 120
tgcctctagg ttagagtcct gtcaccagag attgatcaca cttgattgtg attgttagac 180
aactgtctca ttcaatagac tgtcagtttc tggagggcag agatcttgtc tctgttgttc 240
ctcttttaat ccccagtgtc tagcatctca gagacacttg ttgaatgaat tcattaacga 300
ctggctgaat aatgagcaat tcatgaaaaa acactttata ttcacaggtt ttgggtaaga 360
cagtagctcc cttaaaacac acacacactc ttcatggtat gtcacagaac tacagtctac 420
actcagtgca aagggtttca aaatccagta ggaaggacaa atattgtccc tcttctgtcc 480
ttggagaaga tattgtgcaa gtgtggagtt ctcctggaga aatattcttt tttttctttt 540
gagatggagt ttcactcttg ttgcccaggc tggagtgcaa tggcacgatc ttagctcact 600
gcaacctctg cctcatgggt tgaagcaatt caagacctgc cttagcctcc tgggtagctg 660
gaattacaag cacccaccac cacatccagc taattttttg taatttcagt agagatgggg 720
tttcactata ttggccaggc tggtctcgaa ctcctgactt cagacaatcc acccgcctca 780

atctcccaaa gtgct 795

<210> 130
<211> 5786
<212> DNA
<213> Homo sapiens

<400> 130
gccgcccctg agcagcctcg ccttcgcctc ccgcgtttcc tgccgtccgc cctcccccgg 60
ccgagctcca ggggctgccg cctagcagct cccggcggga gagcggttca gagcgcgcac 120
gggggcgggc ggaggcggcc cggtgcgggg cggccgcgct ggagagaggc gcgcgcggag 180
acgccggccc ccctctccgc gtttgctcgc ttgctccccg cctcccgcac tccgctcgct 240
cccacccctt cccggcgtga ttgatccgtc acgggcgccg ccgctgccgc cgccgccgcc 300
gcggccgttc tgagccgagc cggaacccta gcccgagacg gagccggggc ccgggccggc 360
gccattgcgc gggcgccgcg ggaagacctt ggcgcggggc ggcgggccgg gccaggccat 420
gcgggccgag tgagccggcg cccgcagccc gcggcgcggc atggcttccc cgcggagctc 480
cgggcagccc gggccgccgc cgccgccgcc accgccgccc gcgcgcctgc tactgctact 540
gctgctgccg ctgctgctgc ctctggcgcc cggggcctgg ggctgggcgc ggggcgcccc 600
ccggccgccg cccagcagcc cgccgctctc catcatgggc ctcatgccgc tcaccaagga 660
ggtggccaag ggcagcatcg ggcgcggtgt gctccccgcc gtggaactgg ccatcgagca 720
gatccgcaac gagtcactcc tgcgccccta cttcctcgac ctgcggctct atgacacgga 780
gtgcgacaac gcaaaagggt tgaaagcctt ctacgatgca ataaaatacg ggcctaacca 840
cttgatggtg tttggaggcg tctgtccatc cgtcacatcc atcattgcag agtccctcca 900
aggctggaat ctggtgcagc tttcttttgc tgcaaccacg cctgttctag ccgataagaa 960
aaaataccct tatttctttc ggaccgtccc atcagacaat gcggtgaatc cagccattct 1020
gaagttgctc aagcactacc agtggaagcg cgtgggcacg ctgacgcaag acgttcagag 1080
gttctctgag gtgcggaatg acctgactgg agttctgtat ggcgaggaca ttgagatttc 1140
agacaccgag agcttctcca acgatccctg taccagtgtc aaaaagctga aggggaatga 1200
tgtgcggatc atccttggcc agtttgacca gaatatggca gcaaaagtgt tctgttgtgc 1260
atacgaggag aacatgtatg gtagtaaata tcagtggatc attccgggct ggtacgagcc 1320
ttcttggtgg gagcaggtgc acacggaagc caactcatcc cgctgcctcc ggaagaatct 1380
gcttgctgcc atggagggct acattggcgt ggatttcgag cccctgagct ccaagcagat 1440
caagaccatc tcaggaaaga ctccacagca gtatgagaga gagtacaaca acaagcggtc 1500
aggcgtgggg cccagcaagt tccacgggta cgcctacgat ggcatctggg tcatcgccaa 1560
gacactgcag agggccatgg agacactgca tgccagcagc cggcaccagc ggatccagga 1620
cttcaactac acggaccaca cgctgggcag gatcatcctc aatgccatga acgagaccaa 1680
cttcttcggg gtcacgggtc aagttgtatt ccggaatggg gagagaatgg ggaccattaa 1740
atttactcaa tttcaagaca gcagggaggt gaaggtggga gagtacaacg ctgtggccga 1800
cacactggag atcatcaatg acaccatcag gttccaagga tccgaaccac caaaagacaa 1860
gaccatcatc ctggagcagc tgcggaagat ctccctacct ctctacagca tcctctctgc 1920
cctcaccatc ctcgggatga tcatggccag tgcttttctc ttcttcaaca tcaagaaccg 1980
gaatcagaag ctcataaaga tgtcgagtcc atacatgaac aaccttatca tccttggagg 2040
gatgctctcc tatgcttcca tatttctctt tggccttgat ggatcctttg tctctgaaaa 2100
gacctttgaa acactttgca ccgtcaggac ctggattctc accgtgggct acacgaccgc 2160
ttttggggcc atgtttgcaa agacctggag agtccacgcc atcttcaaaa atgtgaaaat 2220
gaagaagaag atcatcaagg accagaaact gcttgtgatc gtggggggca tgctgctgat 2280
cgacctgtgt atcctgatct gctggcaggc tgtggacccc ctgcgaagga cagtggagaa 2340
gtacagcatg gagccggacc cagcaggacg ggatatctcc atccgccctc tcctggagca 2400
ctgtgagaac acccatatga ccatctggct tggcatcgtc tatgcctaca agggacttct 2460
catgttgttc ggttgtttct tagcttggga gacccgcaac gtcagcatcc ccgcactcaa 2520
cgacagcaag tacatcggga tgagtgtcta caacgtgggg atcatgtgca tcatcggggc 2580
cgctgtctcc ttcctgaccc gggaccagcc caatgtgcag ttctgcatcg tggctctggt 2640
catcatcttc tgcagcacca tcaccctctg cctggtattc gtgccgaagc tcatcaccct 2700
gagaacaaac ccagatgcag caacgcagaa caggcgattc cagttcactc agaatcagaa 2760
gaaagaagat tctaaaacgt ccacctcggt caccagtgtg aaccaagcca gcacatcccg 2820
cctggagggc ctacagtcag aaaaccatcg cctgcgaatg aagatcacag agctggataa 2880
agacttggaa gaggtcacca tgcagctgca ggacacacca gaaaagacca cctacattaa 2940
acagaaccac taccaagagc tcaatgacat cctcaacctg ggaaacttca ctgagagcac 3000
agatggagga aaggccattt taaaaaatca cctcgatcaa aatccccagc tacagtggaa 3060
cacaacagag ccctctcgaa catgcaaaga tcctatagaa gatataaact ctccagaaca 3120
catccagcgt cggctgtccc tccagctccc catcctccac cacgcctacc tcccatccat 3180
cggaggcgtg gacgccagct gtgtcagccc ctgcgtcagc cccaccgcca gcccccgcca 3240
cagacatgtg ccaccctcct tccgagtcat ggtctcgggc ctgtaagggt gggaggcctg 3300

ggcccggggc ctcccccgtg acagaaccac actgggcaga ggggtctgct gcagaaacac 3360
tgtcggctct ggctgcggag aagctgggca ccatggctgg cctctcagga ccactcggat 3420
ggcactcagg tggacaggac ggggcagggg gagacttggc acctgacctc gagccttatt 3480
tgtgaagtcc ttatttcttc acaaagaaga ggaacggaaa tgggacgtct tccttaacat 3540
ctgcaaacaa ggaggcgctg ggatatcaaa cttgcaaaaa aaaaaaaaaa aaaaaaaaaa 3600
caaaaaaact agacaaggag agaggcacta gaactccagc tggaagtcac ggagtggctc 3660
gagcagcctt gggaagaggc aaggagcttc tgaagaaact gcctctgcac acacatcact 3720
ggctgtgacc cctcaggcta gcccttctcc actctggggg aggaggtggg aagggccacc 3780
aggcccccag ctgccaggcc agctgacccc agccttcctg gaacagggag tctgcaggag 3840
cgcagacagg cacagccctg gagcaggcag gccgagggct gcggcactgg agcaggctga 3900
cttacatgct ccacatggga cctgtgtcac ccaatgagat gtttgttact ctggtaaatg 3960
ccacacgtta acacaataac acccattcct gggaccgtgg ggatttaggg cacgtcactg 4020
cagacacgct ctgcagcatt caccgacagt ctgtcatgca cccaccacgt tggccatgtc 4080
cttgtgttcc tatcggatgc tcccagtaac cagggggacc acccgagcta atcatggaat 4140
gtctgttccc agcaaacacg ataaagaaag attgtgcact ttaacctctc tcatcagggc 4200
ccaagggctg gctgggattt tttttttttt tttcccacta actttgtttc tgaccaaagt 4260
gaattggagg cactctgcta aaagacatcc ccgtagacat aggggagaga gttgctggct 4320
gagggcttcc cttggcttcc agaaggcagc cttccatcca gacaagccag tgagctctcc 4380
ccttgggatc actggggtga tcagtcagca gattgattct cattcataag atcattcctc 4440
cctttaaatt gagcccctaa gagcactggc ctgggagtca gacagacctg ggttcaagtc 4500
ctcagtcccc tgcccactcc ctacgtgact ttgatcaggt cactagtgtc tctctgagcc 4560
tcagtttccc ctctgtaact tggggttgaa ctaaaacacc tgtcctgcct acctcacaag 4620
gtcactctga ggattgaaac ttgatcttgt ccaggaaagc tttgtaccaa acagtgaagc 4680
cgccctgatc cgtgaggtat gagtatgact ctgaccttca gccctcccta cagccggggg 4740
tgtggcccag agaagcttcc agcacagccc tctacccaga acatccgggc tggagggagg 4800
ctcccagtga cttttctgac attcctagac aggttcattc tttgctcaag aaaggcctga 4860
atgacaatgt ccaggatgtc tgcacaactg agcagctcgc tcactcccta aagaaaccta 4920
ttggcagctt caacaggcag gcaataatct cttcccagaa ccactgcagt caggaataaa 4980
ctgttttctc caccaggctt tgacaaaagg gcccacagga atcttaccaa tgccaacatt 5040
tcaaagcacc ctatttcacg tagcatagct ttctgctccc cttccccaaa gagaggttat 5100
ggaggtactg tagcttttag ggaaaaaaaa atgttaacac atcacaggtc aagttgaagt 5160
cattctctgt ttaggcacta aaaatcggtg ttgtcactca ctgtgtatta ccagtattta 5220
cttgctttct tgatttcacc aaaaccaaat ttaatttaaa ggaccacatt aatttttcaa 5280
agggaaagag acaattaatt gtacataatg tatacacaca cacaaaaaaa aatacctgta 5340
gaaatattat tccagcatag caggaaaaca aacaaaagta ttggactgtc ggaggtgagc 5400
ctgtgcgtct gtaacccttt gtgactcctg agcgtgcgct gtcttctagg ttaactcacg 5460
aagtacattc tctgtcttac tgatactgta ggttcaccca ttttttttta atttcctcgc 5520
aaataacaag acccacagaa gtgactctag ctacttaatg gttctgttct tttatatgca 5580
gcaaacacac cgtccatttc tgaagaggct tcggcctgaa ggcattttcc aatgatgtta 5640
gtgcacaaaa cgctttaaat tagactggaa ctgccagaat caaatgtaaa tgaggaattt 5700
ctcgtacccc tactgcatgg tatcgatttt taataaattg ttgcaaattt gtttttatga 5760
ataaaaggaa aaaacctgtc gtcttt 5786

<210> 131
<211> 4933
<212> DNA
<213> Homo sapiens

<400> 131
cagaaatgca acaagtagta cccggggtct gcagagcgcc ccgcgccgcc tgacttggcc 60
gggcgaagcc cgcctgcaga gacccgggcc ggcctccgga caaaggacgg aggaggggct 120
ggacggcgct gcgaagtccg aaagaggcca tttagcgact ctggccaggc taaggggaat 180
gcagaggaga cacagagccg gcgggccaag aggacgatcc ggccgctgca cgcagggcgg 240
gaggcgatgg aggctgcccg cgccttgcgc ctcctgctcg tggtgtgcgg ctgcctcgcg 300
ctcccgccgc tggccgagcc cgtgtgcccg gagcgctgcg actgccagca tccccagcat 360
ctcctgtgca ccaacagggg gctccgcgta gtgcccaaga ccagctcgct gccgagcccc 420
cacgacgtgc tcacctacag cctcggcggc aacttcataa ccaacatcac ggccttcgac 480
ttccaccgtc tggggcagct cagacggctg gacctgcagt acaaccagat ccgctctctg 540
caccccaaga ccttcgagaa gctctcgcgg ctggaagagc tgtacctggg gaacaacctc 600
ttgcaggcgc tcgccccggg cacgctggcc ccgctgcgca agctgcgcat cctctacgcc 660
aacgggaacg agatcagccg cctaagccgc ggctccttcg agggcctgga gagtctagtc 720
aagctgcggc tggacgggaa cgccctgggg gcgctgccgg acgcggtctt cgctcccttg 780

ggcaacctgc tctacctaca tctggagtcc aaccggatcc gctttctggg caagaacgcc 840
ttcgcccagc taggcaagct gcgcttcctc aacctctctg ccaacgagct acagccctcc 900
ctgcgccacg cggccacctt cgcaccgctg cgctccctct cctccctcat cctctcggcc 960
aacagcctgc agcacctcgg gccgcgcatc ttccagcacc tgccacgtct cggcctgctc 1020
tcgctcaggg gcaaccagct cacgcacctc gcgcctgagg ccttttgggg cttggaggcc 1080
ctgcgcgagc tgcgcctgga gggtaatcgg ctgagccagc tgccaactgc gctgctggag 1140
cctctgcaca gcctggaggc gctggacctg agcggcaatg agctgtccgc cctgcacccg 1200
gccaccttcg gccacctggg ccggctgcgc gagctcagcc tgcgcaacaa cgcgctcagc 1260
gccctatccg gggacatctt cgccgccagc ccagcccttt atcggctgga tctagacggc 1320
aacggctgga cctgcgactg ccggctgcga ggcctgaagc gctggatggg cgactggcac 1380
tcgcagggcc ggctcctcac tgtcttcgtg cagtgtcgcc accccccggc cctgcgaggc 1440
aaatacctgg attacctgga tgaccagcag ctgcaaaatg gatcctgcgc ggatccctcg 1500
ccctcagctt ccctgaccgc tgaccgcagg cggcagcccc tacccacggc cgcaggggag 1560
gagatgacgc cacctgcagg tctcgcggag gagctgccgc cgcagccgca gctccagcag 1620
caggggcgat ttctagctgg ggtggcctgg gatggggccg ccagggagct ggtaggcaac 1680
cgcagcgctc taaggctgag tcggcggggc ccgggcctcc agcagcccag cccctccgtc 1740
gctgccgccg cgggcccggc tccacagtcc ctagacctgc acaagaagcc ccagcggggc 1800
cgtccgactc gggcagatcc cgccctcgcg gagcccaccc caacggcctc tcctggctct 1860
gcgccatcgc ccgccggcga cccctggcag cgcgcgacga agcatcgtct gggcacggag 1920
caccaggagc gtgccgccca gtccgacggt ggggccgggc tgccgccgct ggtgtccgac 1980
ccatgcgact tcaacaagtt cattctgtgc aacctgacgg tggaggcggt gggcgcagac 2040
agcgcctcgg tgcgctgggc cgtgcgcgag caccgcagtc cccggccgct gggcggcgcg 2100
cgcttccgcc tgctctttga ccgctttggc cagcagccca agttccaccg cttcgtctac 2160
ctgcctgaga gcagcgactc ggccacgctg cgcgagctgc gcggggacac cccctacctg 2220
gtgtgcgtgg agggcgtgct tgggggccgt gtctgccctg tggctccccg ggaccactgc 2280
gcggggctgg tcaccctacc ggaggccggg agccggggcg gcgtcgacta ccagctgctg 2340
accttggccc tgctgacggt caacgcgctg ctggtgctcc tggccttggc ggcctgggcg 2400
tctcgctggc tgcgtaggaa actgcgggct aggcggaagg gcggggcccc ggtccacgtt 2460
cggcacatgt actccacccg acggcccctg cgctccatgg gcaccggcgt gtccgccgac 2520
ttctcgggat tccagtcgca ccggccacgc accaccgtgt gcgcgctcag tgaggcggac 2580
ctcatcgaat tcccctgcga ccgcttcatg gacagtgcgg gcggcggcgc gggcggcagc 2640
ctgagacggg aggaccgtct cctgcagcga tttgccgact aggtccaggg catatagaga 2700
ccatctcatt ggccctaagg agccgcctct ccgtgaggcc caccagccca cctcagggga 2760
agtgccgttt tgtgcacttg ccagagaggc cggtggggac agagggccaa ttcgggcacc 2820
atcccccaat tcccaatact cgagaagtaa atggatggta cttggtggtc agagccagag 2880
taggggacaa atggatggtg ggatgctgag gtcggaggct tcgcttggag gctttgacac 2940
agctatgcga atggcttttg tagcactgca atgcagaagc caggctttgg gggtagaaag 3000
ggggtgcttg tgcccccaac atggccagaa atatttgggt gcatgctttt tgtttgctca 3060
gtgtcaaaca gagaagtttt gtttctattt aaggaaggaa cttattacca ttacaaagga 3120
ggcttggcca gggactccac tggtttggtg atctctgcca agaaggggga tgtaaacagg 3180
tggtaaagtt taacataccc gccaaggaac tcgtttatgt tggctgatag agcattcagg 3240
ataccttaaa gtttaataag agacgcattt ttttttttca aaaatgtgaa aagctctgac 3300
atttaacgaa ctgaccaata gactcaagga ctgttttagt tggactgggc cattttatta 3360
tgttcctttt aatattaaca gtacaagagc gcttgctgac ttcgaggata actaagatta 3420
catactttct caggagaagg ctgcatgcaa gacttctctg tcagggttgc tcctgtggct 3480
tttttaattt tattttttta aacctatata tggaaaagga aatttcaatg ccagatttga 3540
taaaagaatg tgatgtatat gtagctgatg acccactggg gaacaccagt gttccagttc 3600
acttaccaca tctgtgacag tgtgtttaga ttggaataaa tgtgatgcat tacttcttat 3660
gtttttatca gtgacatggt tgactgtgcc ctaattctct tgagttgcag ttaagcaatg 3720
aaggttattt cctaataggg aagcaaaagg tgattgtcaa ttgatagttt aatgtttgac 3780
cacattagtg tctttatatg aaatagtaga ggggaagaaa ttatagaaaa caaatgtgaa 3840
aaaaatacac cagtgggtat ctgttctact aaaaccagaa gattgttatg agtacttaaa 3900
cctcactgtg aaataatgat atattttgca attaatccct ccccaactga gtgtcttact 3960
gtgttattaa atcttatctt ttagttaata gttgcagtat ttcttttaaa tgtttttgtt 4020
ttaaacttag gggtaggatc ctttatttgt tcagttgttt ccaactattt gggatacttt 4080
cattccctgc tatttatgaa gtacatgttt atcactaagt gtagtggttt ggtttacatt 4140
aataatttta tgtgtggtct aaaaatgcag tcactaagag aatgtacact gtggttatgg 4200
tcacagtggt tatggattta ttggcagtgg agtaccatgg aggtcactgc aatggtgcta 4260
aggctgacag tggaatcttc tgtttagggc cttaagcccc tgagggtcct ggtgactcag 4320
ggaatccatc acagccccgg gtctttcacc ccagttcact ccttttatgt ttggcctaga 4380
ttgacccatg cctgccctct tctcaacagc tgagtaggga accagccatc tgaatgagct 4440
gatcgttgtt tatgttcaaa tgagtaaatg tccagaccac ttaaaactac agctgctttt 4500
ctggcagtct cgtctgcggg gccaaaactt aataaaccac aggaaataga ctgtcattct 4560

tagtttgctg ccagggctta ttttagattg agagcatact ggtacatgag agcagtagtg 4620
ttgtttgctc ttattttcaa ccagggagct atctggcacc ttttgtgctc ctggcttttt 4680
tcaatcatag cactattgca tctcctagct atttcttttg cccagcaggg taatattgag 4740
tcccattgca agtatggaca aggcctctgg ttctcctcac cacccacctt ttcagccata 4800
gaacatcact gaaaatgcct aatgcctgga tctgtgttct actttagttt cactgggaag 4860
tctttcagtg ggagatgaat aaatgttata cattgttatg tccagttatg tcaataaacc 4920
cactaataga gat 4933

<210> 132
<211> 4122
<212> DNA
<213> Homo sapiens

<400> 132
tttgtcatca gctcgctctc cattggcggg gagcggagag cagcgaagaa gggggtgggg 60
aggggagggg aagggaaggg ggtggaaact gcctggagcc gtttctccgc gccgctgttg 120
gtgctgccgc tgcctcctcc tcctccgccg ccgccgccgc cgccgccgcc tcctccggct 180
cttcgctcgg cccctctccg cctccatgtg ccggatagcg ggagcgctgc ggaccctgct 240
gccgctgctg gcggccctgc ttcaggcgtc tgtagaggct tctggtgaaa tcgcattatg 300
caagactgga tttcctgaag atgtttacag tgcagtctta tcgaaggatg tgcatgaagg 360
acagcctctt ctcaatgtga agtttagcaa ctgcaatgga aaaagaaaag tacaatatga 420
gagcagtgag cctgcagatt ttaaggtgga tgaagatggc atggtgtatg ccgtgagaag 480
ctttccactc tcttctgagc atgccaagtt cctgatatat gcccaagaca aagagaccca 540
ggaaaagtgg caagtggcag taaaattgag cctgaagcca accttaactg aggagtcagt 600
gaaggagtca gcagaagttg aagaaatagt gttcccaaga caattcagta agcacagtgg 660
ccacctacaa aggcagaaga gagactgggt catccctcca atcaacttgc cagaaaactc 720
caggggacct tttcctcaag agcttgtcag gatcaggtct gatagagata aaaacctttc 780
actgcggtac agtgtaactg ggccaggagc tgaccagcct ccaactggta tcttcattat 840
caaccccatc tcgggtcagc tgtcggtgac aaagcccctg gatcgcgagc agatagcccg 900
gtttcatttg agggcacatg cagtagatat taatggaaat caagtggaga accccattga 960
cattgtcatc aatgttattg acatgaatga caacagacct gagttcttac accaggtttg 1020
gaatgggaca gttcctgagg gatcaaagcc tggaacatat gtgatgaccg taacagcaat 1080
tgatgctgac gatcccaatg ccctcaatgg gatgttgagg tacagaatcg tgtctcaggc 1140
tccaagcacc ccttcaccca acatgtttac aatcaacaat gagactggtg acatcatcac 1200
agtggcagct ggacttgatc gagaaaaagt gcaacagtat acgttaataa ttcaagctac 1260
agacatggaa ggcaatccca catatggcct ttcaaacaca gccacggccg tcatcacagt 1320
gacagatgtc aatgacaatc ctccagagtt tactgccatg acgttttatg gtgaagttcc 1380
tgagaacagg gtagacatca tagtagctaa tctaactgtg accgataagg atcaacccca 1440
tacaccagcc tggaacgcag tgtacagaat cagtggcgga gatcctactg gacggttcgc 1500
catccagacc gacccaaaca gcaacgacgg gttagtcacc gtggtcaaac caatcgactt 1560
tgaaacaaat aggatgtttg tccttactgt tgctgcagaa aatcaagtgc cattagccaa 1620
gggaattcag cacccgcctc agtcaactgc aaccgtgtct gttacagtta ttgacgtaaa 1680
tgaaaaccct tattttgccc ccaatcctaa gatcattcgc caagaagaag ggcttcatgc 1740
cggtaccatg ttgacaacat tcactgctca ggacccagat cgatatatgc agcaaaatat 1800
tagatacact aaattatctg atcctgccaa ttggctaaaa atagatcctg tgaatggaca 1860
aataactaca attgctgttt tggaccgaga atcaccaaat gtgaaaaaca atatatataa 1920
tgctactttc cttgcttctg acaatggaat tcctcctatg agtggaacag gaacgctgca 1980
gatctattta cttgatatta atgacaatgc ccctcaagtg ttacctcaag aggcagagac 2040
ttgcgaaact ccagacccca attcaattaa tattacagca cttgattatg acattgatcc 2100
aaatgctgga ccatttgctt ttgatcttcc tttatctcca gtgactatta agagaaattg 2160
gaccatcact cggcttaatg gtgattttgc tcagcttaat ttaaagataa aatttcttga 2220
agctggtatc tatgaagttc ccatcataat cacagattcg ggtaatcctc ccaaatcaaa 2280
tatttccatc ctgcgcgtga aggtttgcca gtgtgactcc aacggggact gcacagatgt 2340
ggacaggatt gtgggtgcgg ggcttggcac cggtgccatc attgccatcc tgctctgcat 2400
catcatcctg cttatccttg tgctgatgtt tgtggtatgg atgaaacgcc gggataaaga 2460
acgccaggcc aaacaacttt taattgatcc agaagatgat gtaagagata atattttaaa 2520
atatgatgaa gaaggtggag gagaagaaga ccaggactat gacttgagcc agctgcagca 2580
gcctgacact gtggagcctg atgccatcaa gcctgtggga atccgacgaa tggatgaaag 2640
acccatccac gctgagcccc agtatccggt ccgatctgca gccccacacc ctggagacat 2700
tggggacttc attaatgagg gccttaaagc ggctgacaat gaccccacag ctccaccata 2760
tgactccctg ttagtgtttg actatgaagg cagtggctcc actgctgggt ccttgagctc 2820
ccttaattcc tcaagtagtg gtggtgagca ggactatgat tacctgaacg actgggggcc 2880
acggttcaag aaacttgctg acatgtatgg tggaggtgat gactgaactt cagggtgaac 2940

ttggtttttg gacaagtaca aacaatttca actgatattc ccaaaaagca ttcagaagct 3000
aggctttaac tttgtagtct actagcacag tgcttgctgg aggctttggc ataggctgca 3060
aaccaatttg ggctcagagg gaatatcagt gatccatact gtttggaaaa acactgagct 3120
cagttacact tgaattttac agtacagaag cactgggatt ttatgtgcct ttttgtacct 3180
ttttcagatt ggaattagtt ttctgtttaa ggctttaatg gtactgattt ctgaaacgat 3240
aagtaaaaga caaaatattt tgtggtggga gcagtaagtt aaaccatgat atgcttcaac 3300
acgcttttgt tacattgcat ttgcttttat taaaatacaa aattaaacaa acaaaaaaac 3360
tcatggagcg attttattat cttgggggat gagaccatga gattggaaaa tgtacattac 3420
ttctagtttt agactttagt ttgttttttt tttttcacta aaatcttaaa acttactcag 3480
ctggttgcaa ataaagggag ttttcatatc accaatttgt agcaaaattg aattttttca 3540
taaactagaa tgttagacac attttggtct taatccatgt acactttttt atttctgtat 3600
ttttccactt cactgtaaaa atagtatgtg tacataatgt tttattggca tagtctatgg 3660
agaagtgcag aaacttcaga acatgtgtat gtattatttg gactatggat tcaggttttt 3720
tgcatgttta tatctttcgt tatggataaa gtatttacaa aacagtgaca tttgattcaa 3780
ttgttgagct gtagttagaa tactcaattt ttaatttttt taattttttt attttttatt 3840
ttctttttgg tttggggagg gagaaaagtt cttagcacaa atgttttaca taatttgtac 3900
caaaaaaaaa aaaaaggaaa ggaaagaaag gggtggcctg acactggtgg cactactaag 3960
tgtgtgtttt ttaaaaaaaa aaatggaaaa aaaaaagctt ttaaactgga gagacttctg 4020
acaacagctt tgcctctgta ttgtgtacca gaatataaat gatacacctc tgaccccagc 4080
gttctgaata aaatgctaat tttggaaaaa aaaaaaaaaa aa 4122

<210> 133
<211> 1920
<212> DNA
<213> Homo sapiens

<400> 133
gaaggccatg gtctccccac ggatgtccgg gctcctctcc cagactgtga tcctagcgct 60
cattttcctc ccccagacac ggcccgctgg cgtcttcgag ctgcagatcc actctttcgg 120
gccgggtcca ggccctgggg ccccgcggtc cccctgcagc gcccggctcc cctgccgcct 180
cttcttcaga gtctgcctga agcctgggct ctcagaggag gccgccgagt ccccgtgcgc 240
cctgggcgcg gcgctgagtg cgcgcggacc ggtctacacc gagcagcccg gagcgcccgc 300
gcctgatctc ccactgcccg acgggctctt gcaggtgccc ttccgggacg cctggcctgg 360
caccttctct ttcatcatcg aaacctggag agaggagtta ggagaccaga ttggagggcc 420
cgcctggagc ctgctggcgc gcgtggctgg caggcggcgc ttggcagccg gaggcccgtg 480
ggcccgggac attcagcgcg caggcgcctg ggagctgcgc ttctcgtacc gcgcgcgctg 540
cgagccgcct gccgtcggga ccgcgtgcac gcgcctctgc cgtccgcgca gcgccccctc 600
gcggtgcggt ccgggactgc gcccctgcgc accgctcgag gacgaatgtg aggcgccgct 660
ggtgtgccga gcaggctgca gccctgagca tggcttctgt gaacagcccg gtgaatgccg 720
atgcctagag ggctggactg gacccctctg cacggtccct gtctccacca gcagctgcct 780
cagccccagg ggcccgtcct ctgctaccac cggatgcctt gtccctgggc ctgggccctg 840
tgacgggaac ccgtgtgcca atggaggcag ctgtagtgag acacccaggt cctttgaatg 900
cacctgcccg cgtgggttct acgggctgcg gtgtgaggtg agcggggtga catgtgcaga 960
tggaccctgc ttcaacggcg gcttgtgtgt cgggggtgca gaccctgact ctgcctacat 1020
ctgccactgc ccacctggtt tccaaggctc caactgtgag aagagggtgg accggtgcag 1080
cctgcagcca tgccgcaatg gcggactctg cctggacctg ggccacgccc tgcgctgccg 1140
ctgccgcgcc ggcttcgcgg gtcctcgctg cgagcacgac ctggacgact gcgcgggccg 1200
cgcctgcgct aacggcggca cgtgtgtgga gggcggcggc gcgcaccgct gctcctgcgc 1260
gctgggcttc ggcggccgcg actgccgcga gcgcgcggac ccgtgcgccg cgcgcccctg 1320
tgctcacggc ggccgctgct acgcccactt ctccggcctc gtctgcgctt gcgctcccgg 1380
ctacatggga gcgcggtgtg agttcccagt gcaccccgac ggcgcaagcg ccttgcccgc 1440
ggccccgccg ggcctcaggc ccggggaccc tcagcgctac cttttgcctc cggctctggg 1500
actgctcgtg gccgcgggcg tggccggcgc tgcgctcttg ctggtccacg tgcgccgccg 1560
tggccactcc caggatgctg ggtctcgctt gctggctggg accccggagc cgtcagtcca 1620
cgcactcccg gatgcactca acaacctaag gacgcaggag ggttccgggg atggtccgag 1680
ctcgtccgta gattggaatc gccctgaaga tgtagaccct caagggattt atgtcatatc 1740
tgctccttcc atctacgctc gggaggtagc gacgcccctt ttccccccgc tacacactgg 1800
gcgcgctggg cagaggcagc acctgctttt tccctaccct tcctcgattc tgtccgtgaa 1860
atgaattggg tagagtctct ggaaggtttt aagcccattt tcagttctaa cttactttca 1920

<210> 134
<211> 411
<212> DNA

<213> Homo sapiens

<400> 134
atgtgccagg ctttcctctg ggtgctaggg acactgtggc ttctcaaaaa tgcccgttgt 60
ctgcagccat acccacctga acatgcccaa tcttgcctga tctcagaagc taagcagggt 120
caggcccagt taccacttgg atgggtgaag tggcccttgc acttgaggag ctcactgtcc 180
aaaaggttgg aaaggaagta ccccagccta ttgaatgggg aaattgaagc acagatatgt 240
aaaacatctt ctctagagtt acccagctgt gatctggtga cagctgatgg ttccactgaa 300
gtaactatct cggaaaatct ccctgctgtt ggattccata tctgtcaaca gcaggactct 360
catgtagaag gcatggttaa catctccaaa gcctcttctg gccaaatgta g 411

<210> 135
<211> 2826
<212> DNA
<213> Homo sapiens

<400> 135
gagagggccc ggactagggg cggcgggcac cgcaggagct ccgcgcggct gcagcgcggg 60
cgggagcggg gacgcgatgt cgccgccgcc gcctccttgc gggccggggc tgcgcctccg 120
gggctgagcc gccgccagag ccgacagccg agcagccgct gggcgctccc gcggcgcagg 180
aggatgggct gcggcgggag ccgggcggat gccatcgagc cccgctacta cgagagctgg 240
acccgggaga cagaatccac ctggctcacc tacaccgact cggacgcgcc gcccagcgcc 300
gccgccccgg acagcggccc cgaagcgggc ggcctgcact cgggcatgct ggaagatgga 360
ctgccctcca atggtgtgcc ccgatctaca gccccaggtg gaatacccaa cccagagaag 420
aagacgaact gtgagaccca gtgcccaaat ccccagagcc tcagctcagg ccctctgacc 480
cagaaacaga atggccttca gaccacagag gctaaaagag atgctaagag aatgcctgca 540
aaagaagtca ccattaatgt aacagatagc atccaacaga tggacagaag tcgaagaatc 600
acaaagaact gtgtcaacta gcagagagtc caagcagaag ggcagatgga cttcttcagt 660
gtccttcacg gcactggatc ccatcaaaga accttgaaga agtggctgcc ccttgctgga 720
cctgaattct actgagtccc tggcaagacc gtcttacctg gcagcaaact gctgcctgat 780
ttgttgggac cttctgagcc ttctacttat catgtaaatg tattggcaca gtgcttacat 840
atgttaataa actgcaaatg tgcagttcag tttgtctctt tgcaactcct gtaatacggt 900
ctggtgtaaa agtagtgagt taaagctaca ggtcagttta tgaaacagaa aagtaggaat 960
gcattttctg ggtgaaagag tcacacctta gtgctataac tctcctgccc atgatagtgt 1020
attctgtttc aggcaagctt attctttcct tccttcattt taaatattgt cattacaaat 1080
cttaccaggt tcacttaaaa gctggctttc atccaactct aaacccacat attgaaaaaa 1140
tcaaggtaca ggaaaactcc ttgttatcct tgtttcctta gcttggtatg agacagatcg 1200
gatccagttt cccatgcacc aacccactgc ccatggcatg tctttgggag gtgtctgtga 1260
agcagtcata cctgctcctc atctgcctgg aaagtcctcc tattccagtg tccatgttgg 1320
cctccagtcc ttaatgtcac catgcttgtg gccaatgcat ccaaataagg atacccctca 1380
gggctcagct agacattgca attttgcata gctttccagt tccctttgct tgtcttcttg 1440
actgtcttcc ctctctaccg gggtcacttg caattgttaa tcaaagattg aacactgcgt 1500
aggagaggga gatgatccag agacatgtgg cagcaggcat ggcttcccct tggcctctct 1560
gtacactgcc ccaggactgt cattttggca tctgcaaagg aatcacttta gaaagccagc 1620
acctggttga tgtgtattca tactgacatt agattgatgt gcactgcatt agaaatgagg 1680
tagctgacac agaaaaagga tgttttgata ggaataattt tctagtatgt cttgaaacat 1740
gttcatctgg aagtattttc ctccaaagta atgtagcatg atttttcaag gattgttaac 1800
atgcctggga ttgggaaaga taggactaaa gttgtgccaa actatatcaa taaattccat 1860
gtttagcaga aataggcagc ctattggtgt tatgtttatg taacatagtc cagagaactg 1920
acatgcaggt caaaagtcag atacgcaacc tccttatctg ctaactctgt tattcttcaa 1980
acacaagtgg gtagtgtcat ttttccttcc ttccttccat tggcagattg tatatttatt 2040
cacaaaacat taaatgtcca tcctgtgcca ggtactatgc agatgttgag ggatttgggg 2100
tctggttagt cgtgactatc tatcctgaat ctaacagtga cttcataact aggagactga 2160
attagaccct taaggtatag tgtgtgttgc aaatcactct gcaatggaaa cttttatatt 2220
cagggtaggt ttgtgtctta aactaggtgt tctaatcaat gtacaagact ttaccataca 2280
cgcaactata gtttttctaa accttcatca ttttgtgatt ctttgagaaa gggcttttag 2340
gaactttatg ttctaaaaaa tgtttttaac aataataaga taaaagaaaa acctgtgatt 2400
catatgtccc cactggcatt actcagcagg agcccccagc tgccaaaggt tggcagtgat 2460
cctgcaagtt caagggctct ttctccctgg ggatgtgctt tgtggcttct ctttacagct 2520
ttgtttctgc atcagttcac tgctgcatgt tgtttggaat ttatcacctt aagaaagtgt 2580
ctctgtttta tatagaaaca ctttctcact tacaggggag aaggaaatgc agggcacatg 2640
atctggccct ccccagaaca atctggattt cacggagaca gcaaccagaa gttaaaccat 2700
gtgactaaaa atgcatctgg ctactttttc atgtatgtat gagacagaaa ctaatcctta 2760

ctatcctatt aggataccac ttttcattgc aaagtttgtg tcaataaagt cattaatttt 2820
aaacat 2826

<210> 136
<211> 4716
<212> DNA
<213> Homo sapiens

<400> 136
ccttggtttc cgttgcagat tcccacaact ccatgctgtg tgctgcaggc tggtcctgaa 60
cccagatctc tggctgagag gatgggggca gatggggaaa cagtggttct gaagaacatg 120
ctcattggca tcaacctgat ccttctgggc tccatgatca agccttcaga gtgtcagctg 180
gaggtcacca cagaaagggt ccagagacag tcagtggagg aggagggagg cattgccaac 240
tacaacacat ccagcaaaga gcagcctgtg gtcttcaacc acgtgtacaa cattaacgtg 300
cccttggaca acctctgctc ctcagggcta gaggcctctg ctgagcagga ggtgagtgca 360
gaagacgaga ctctggcaga gtacatgggc cagacctcag accacgagag ccaggtcacc 420
tttacacaca ggatcaactt ccccaaaaag gcctgtccat gtgccagttc agcccaggtg 480
ctgcaggagc tgctgagccg gatcgagatg ctggagaggg aggtgtcggt gctgcgagac 540
cagtgcaacg ccaactgctg ccaagaaagt gctgccacag gacaactgga ctatatccct 600
cactgcagtg gccacggcaa ctttagcttt gagtcctgtg gctgcatctg caacgaaggc 660
tggtttggca agaattgctc ggagccctac tgcccgctgg gttgctccag ccggggggtg 720
tgtgtggatg gccagtgcat ctgtgacagc gagtacagcg gggatgactg ttccgaactc 780
cggtgcccaa cagactgcag ctcccggggg ctctgcgtgg acggggagtg tgtctgtgaa 840
gagccctaca ctggcgagga ctgcagggaa ctgaggtgcc ctggggactg ttcggggaag 900
gggagatgtg ccaacggtac ctgtttatgc gaggagggct acgttggtga ggactgcggc 960
cagcggcagt gtctgaatgc ctgcagtggg cgaggacaat gtgaggaggg gctctgcgtc 1020
tgtgaagagg gctaccaggg ccctgactgc tcagcagttg cccctccaga ggacttgcga 1080
gtggctggta tcagcgacag gtccattgag ctggaatggg acgggccgat ggcagtgacg 1140
gaatatgtga tctcttacca gccgacggcc ctggggggcc tccagctcca gcagcgggtg 1200
cctggagatt ggagtggtgt caccatcacg gagctggagc caggtctcac ctacaacatc 1260
agcgtctacg ctgtcattag caacatcctc agccttccca tcactgccaa ggtggccacc 1320
catctctcca ctcctcaagg gctacaattt aagacgatca cagagaccac cgtggaggtg 1380
cagtgggagc ccttctcatt ttccttcgat gggtgggaaa tcagcttcat tccaaagaac 1440
aatgaagggg gagtgattgc tcaggtcccc agcgatgtta cgtcctttaa ccagacagga 1500
ctaaagcctg gggaggaata cattgtcaat gtggtggctc tgaaagaaca ggcccgcagc 1560
ccccctacct cggccagcgt ctccacagtc attgacggcc ccacgcagat cctggttcgc 1620
gatgtctcgg acactgtggc ttttgtggag tggattcccc ctcgagccaa agtcgatttc 1680
attcttttga aatatggcct ggtgggcggg gaaggtggga ggaccacctt ccggctgcag 1740
cctcccctga gccaatactc agtgcaggcc ctgcggcctg gctcccgata cgaggtgtca 1800
gtcagtgccg tccgagggac caacgagagc gattctgcca ccactcagtt cacaacagag 1860
atcgatgccc ccaagaactt gcgagttggt tctcgcacag caaccagcct tgacctcgag 1920
tgggataaca gtgaagccga agttcaggag tacaaggttg tgtacagcac cctggcgggt 1980
gagcaatatc atgaggtact ggtccccagg ggcattggtc caaccaccag ggccaccctg 2040
acagatctgg tacctggcac tgagtatgga gttggaatat ctgccgtcat gaactcacag 2100
caaagcgtgc cagccaccat gaatgccagg actgaacttg acagtccccg agacctcatg 2160
gtgacagcct cctcggagac ctccatctcc ctcatctgga ccaaggccag tggccccatt 2220
gaccactacc gaattacctt taccccatcc tctgggattg cctcagaagt caccgtaccc 2280
aaggacagga cctcatacac actaacagat ctagagcctg gggcagagta catcatttcc 2340
gtcactgctg agaggggtcg gcagcagagc ttggagtcca ctgtggatgc tttcacaggc 2400
ttccgtccca tctctcatct gcacttttct catgtgacct cctccagtgt gaacatcact 2460
tggagtgatc catctccccc agcagacaga ctcattctta actacagccc cagggatgag 2520
gaggaagaga tgatggaggt ctccctggat gccaccaaga ggcatgctgt cctgatgggc 2580
ctgcaaccag ccacagagta tattgtgaac cttgtggctg tccatggcac agtgacctct 2640
gagcccattg tgggctccat caccacagga attgatcccc caaaagacat cacaattagc 2700
aatgtgacca aggactcagt gatggtctcc tggagccctc ctgttgcatc tttcgattac 2760
taccgagtat catatcgacc cacccaagtg ggacgactag acagctcagt ggtgcccaac 2820
actgtgacag aattcaccat caccagactg aacccagcta ccgaatacga aatcagcctc 2880
aacagcgtgc ggggcaggga ggaaagcgag cgcatctgta ctcttgtgca cacagccatg 2940
gacaaccctg tggatctgat tgctaccaat atcactccaa cagaagccct gctgcagtgg 3000
aaggcaccag tgggtgaggt ggagaactac gtcattgttc ttacacactt tgcagtcgct 3060
ggagagacca tccttgttga cggagtcagt gaggaatttc ggcttgttga cctgcttcct 3120
agcacccact atactgccac catgtatgcc accaatggac ctctcaccag tggcaccatc 3180
agcaccaact tttctactct cctggaccct ccggcaaacc tgacagccag tgaagtcacc 3240

agacaaagtg ccctgatctc ctggcagcct cccagggcag agattgaaaa ttatgtcttg 3300
acctacaaat ccaccgatgg aagccgcaag gagctgattg tggatgcaga agacacctgg 3360
attcgactgg agggcctgtt ggagaacaca gactacacgg tgctcctgca ggcagcacag 3420
gacaccacgt ggagcagcat cacctccacc gctttcacca caggaggccg ggtgttccct 3480
catccccaag actgtgccca gcatttgatg aatggagaca ctttgagtgg ggtttacccc 3540
atcttcctca atggggagct gagccagaaa ttacaagtgt actgtgatat gaccaccgac 3600
gggggcggct ggattgtatt ccagaggcgg cagaatggcc aaactgattt tttccggaaa 3660
tgggctgatt accgtgttgg cttcgggaac gtggaggatg agttctggct ggggctggac 3720
aatatacaca ggatcacatc ccagggccgc tatgagctgc gcgtggacat gcgggatggc 3780
caagaggccg ccttcgcctc ctacgacagg ttctctgtcg aggacagcag aaacctgtac 3840
aaactccgca taggaagcta caacggcact gcgggggact ccctcagcta tcatcaagga 3900
cgccctttct ccacagagga tagagacaat gatgttgcag tgactaactg tgccatgtcg 3960
tacaagggag catggtggta taagaactgc caccggacca acctcaatgg gaagtacggg 4020
gagtccaggc acagtcaggg catcaactgg taccattgga aaggccatga gttctccatc 4080
ccctttgtgg aaatgaagat gcgcccctac aaccaccgtc tcatggcagg gagaaaacgg 4140
cagtccttac agttctgagc agtgggcggc tgcaagccaa ccaatatttt ctgtcatttg 4200
tttgtatttt ataatatgaa acaagggggg agggtaatag caatgtgttt tgcaacatat 4260
taagagtatg tgaaggaagc agggatgtcg caggaatccg ctggctaaca tctgctcttg 4320
gtttctgctg ccctggagcc tgaccctcag tctccattct ccctcctacc caggcctcct 4380
caaccttcac ctcctttccc accaaggagg agaagtagga agttttctta aagggccaat 4440
tcaaagccaa gtcgtggggt gcagattgtt atggtgacag gcacacacat ttttctaccc 4500
ttcttctgag atgtcctctg ccttccaggt atttgtgatt ttgtcacagc ctgacatggc 4560
caggttctca cactggccca gagaaaagag cctcagcaag agagttttgc caacaattcc 4620
ccttaaaagg aaacagatca actacaccgc atcccaacaa cccaggttct tttccttcct 4680
tccttccttc ctcccttcct tctttcctgc cttccc 4716

<210> 137
<211> 11649
<212> DNA
<213> Homo sapiens

<220>
<221> modified_base
<222> (1402)...(1402)
<223> n = g, a, c or t

<400> 137
   gggtttgggg agcaggaggc agacatgtgt tgctttgcac gtcatctggt ccttccacct 60
   ggctgggcat gggatggtga cctcagctgg aacatggggc tcgagccaga cctcagggtc 120
   tccctgcgtg tagcccccaa ccccaagcct gatccccacc ggagacctga acagccttgg 180
   acatcatcgg atcagggtgg gaggtgccag cctctgccac ctgacttcca gtccctgtgc 240
   ccctcccacc ctgcccttgg gcactcctgc cctgcaggtt cctcagaggg gcaacccaaa 300
   gccagagagg ggcgggtctt gtcgggcctc cagcctggac ttcccagccc ctctggggca 360
   gcatctgggt gccagacctc ggttcctagg gcctcgtttc tccctctggg aacagccaaa 420
   tggtggtccc tgagactcag gacagaccaa gaagaagccg gatgacatgg caagccacac 480
   actgcgtccc ctgagcagga cgggacagcc cgactccatc ctcccacctg cgactgagcc 540
   cccaccccta ctcctgtccc agccgtgccc ctggtgacat ttgggtggga ggggagggga 600
   tgagggggca cctgggggtc agagaacaaa tgacgggtga atacatgtgt gtggccaggt 660
   ggaggaggga ggggaggagc aggtgggctg gacagggccg gtgtgaggaa ggggctcagg 720
   ctggcagggg gaggtggcaa gagggatggg ctgccattgg tggtctgaga caaaggcgga 780
   gggaggaggc gagcgctgat gggaaaggaa caaagaggga aggggggcgt gaagggggtt 840
   ccggcgggag ggccgagccg agggaggagg cgccggccag ctggacagag ggaggaggcc 900
   aggccagagc cagaagacgg ccagaggcac aaagaagcca gcgcgctggc ggagtcaagg 960
   gatggggcag agactgccgg ggccagcagg gaccagctga aggctgcgca gggggtgcgg 1020
   gccacacagg tagccaccct gagctcagcc accgatggag ggtcgtgggg ctgctgcggt 1080
   gatggcggtg ggcttgggtc catctgtcct gccgtttctg cacagcttag gtgtcaccca 1140
   ctggccttcg tggtgttttc attgtccatc ggcagggaca gctggtggtc tgtctgcccc 1200
   gcctgtctgg ctgtcagcct ctgggcaggc ttgcttttat ggggggaggt cctgtctgtc 1260
   tgtctgtcgc cctctctggc tgtgagcctg ggggtgctgg gctggccagt cggcttgctg 1320
   ggttaggctg tcccagctgt ctgagtgttt gtccggctgt caggatgtgt cctgggggct 1380
   gggaaggaga ggccgaccca tngtctgtcg gtcgactggt cagttggacg ttcagctgtc 1440
   tgtacgtctg tctgctggcc catctgtctc cccttggggc cacctctcac tccacctgcc 1500
   cctctgcgcc ccggattgcc tggccagcac cacgtgggcc tgtacttgtc cacaccagtg 1560
   actcctgcct gagacccccc cccaacccag gatcaggcag gacggctggg gcttaggtca 1620
   ggggccgtct gtccggaagg catcaccgcg ccctccccag accatcagct gaaccctctg 1680
   accctgtgat cccagacgct gcaggagctg aagatggcga gctccgtggc gccctacgag 1740
   cagctggtga ggcaggtgga ggccttgaag gctgagaaca gccacctgag gcaggagcta 1800
   agggacaact ccagccacct gtccaagctg gagacagaga cgtcgggcat gaaggaggtc 1860
   ctgaagcacc tacagggaaa actggagcag gaggcccgag tgctggtgtc ctcggggcag 1920
   acggaggtgc tggagcagct gaaggcccta cagatggaca tcaccagcct gtacaacctc 1980
   aagttccagc cgcccaccct gggcccggag cctgccgccc ggacccccga gggcagccca 2040
   gtacacggct ccgggccctc caaggacagc tttggggagc tgagccgggc caccatccgg 2100
   ctgctggagg aactggaccg ggaacggtgt ttcctgctga atgagattga gaaggaggag 2160
   aaggagaagc tctggtacta ctctcagctg cagggcctgt ccaagcgcct ggacgagctg 2220
   ccgcacgtgg agacgcagtt ctcgatgcag atggacctga tccggcagca gcttgagttc 2280
   gaggcccagc acatccgctc gctgatggag gagcgcttcg gcacctcgga cgagatggtg 2340
   cagcgggcac agatccgcgc ctcgcgcctg gagcagattg acaaggagct gctggaggcg 2400
   caggaccgag tgcagcagac ggagccccag gccttgctgg cggtgaagtc ggtgccggtg 2460
   gacgaggacc ccgagacaga ggtccccaca caccctgagg atggcacccc tcagccgggc 2520
   aacagcaagg tggaggtggt cttctggctg ttgtccatgt tggcgacgcg cgaccaggag 2580
   gatacagcgc gcacgctgct ggccatgtcc agctcgcccg agagctgcgt ggccatgcgc 2640
   cgctcgggct gtctgcctct gctgctgcaa atcctccacg gcaccgaggc cgcggccggg 2700
   ggtcgcgccg ggccccaggg ggcaccgggc gccaaggacg cacgcatgcg cgccaacgcg 2760
   gcgctgcaca acatcgtctt ctcgcagccg gaccagggcc tggcgcgcaa ggagatgcgc 2820
   gtcctgcacg tgctggagca gatccgggcc tactgcgaga cctgctggga ctggctgcag 2880
   gcccgagacg gcgggcccga gggaggtggc gccggcagcg ccccgatccc catcgagccg 2940
   cagatctgcc aggccacctg tgctgttatg aagctgtcct ttgatgagga gtaccgccgt 3000
   gccatgaacg agctaggtgg gctgcaggcc gtggcagagc tgctgcaggt tgactatgag 3060
   atgcacaaga tgacccggga cccgctgaac ctggcgctgc gccgctacgc gggcatgacc 3120
   ctcaccaacc tcacctttgg ggacgttgcc aacaaggcca ccctgtgtgc gcgccgcggc 3180
   tgcatggagg ccatcgtggc ccagctggcc tccgacagtg aggagctcca ccaggtggtg 3240
   tccagcatcc ttcggaactt gtcctggagg gccgacatca acagcaagaa ggtgctgagg 3300
   gaggcgggca gcgtgactgc cctggtgcag tgtgtcctgc gggccaccaa ggagtccacc 3360
   ctgaagagcg tgctgagcgc cctgtggaat ctgtctgcac acagcacaga gaacaaggcg 3420
   gccatctgcc aggtggatgg cgccctgggc ttcctggtga gcaccctgac ctacaagtgt 3480
   cagagcaact cgctggccat catcgagagc ggcggcggca tcctccgcaa tgtgtccagc 3540
   ctcgtcgcca cccgtgagga ctacaggcag gtgctccggg atcacaactg tctgcagacg 3600
   ctgctgcagc atctgacttc gcacagcctg accatcgtga gcaacgcgtg cggcacgctc 3660
   tggaacctgt cggcccgcag cgcccgtgac caggagctgc tgtgggacct gggcgccgtg 3720
   ggcatgctgc gtaatctggt gcactccaag cacaagatga tcgccatggg cagcgccgcc 3780
   gccctgcgca acctgctggc ccatcggccc gccaagcacc aggcggccgc caccgccgtg 3840
   tccccaggca gctgcgtgcc cagcctgtac gtgcgcaagc agcgggcgct ggaggccgag 3900
   ctggacgcac ggcacctcgc gcaggcgctg gagcacctgg agaagcaggg cccgccggca 3960
   gccgaggccg ccactaagaa gccgctgccg cccctgcgac acctggacgg cctggcccaa 4020
   gactatgctt ccgattcggg ctgctttgac gacgacgatg caccgtcatc cctggctgcg 4080
   gccgcggcca ccggggagcc agccagccct gccgcgctgt ccctcttcct gggcagcccc 4140
   ttcctgcagg ggcaggcgct ggctcgcacc ccgcccaccc gccgaggcgg caaggaggca 4200
   gagaaggaca ccagtgggga ggcagccgtg gcggccaagg ccaaggccaa gctggcgctt 4260
   gcagtggcgc gcatcgacca gctggtggag gacatctccg ccctgcacac ctcgtccgac 4320
   gatagcttca gcctcagctc tggagacccg ggacaggagg cgccacggga gggccgcgcc 4380
   cagtcctgct cgccatgccg cggcccggag ggcgggcggc gagaggcagg aagccgggcg 4440
   cacccgctgc tgcggctcaa ggcggcccac gccagcctct ccaacgacag cctcaacagc 4500
   ggcagtgcca gcgacgggta ctgcccacgc gaacatatgc tgccctgccc gctggccgca 4560
   ctggcttcgc gccgcgagga ccccaggtgt gggcagcctc ggcccagccg gcttgacctt 4620
   gacctgcccg gctgccaggc cgagcccccg gcccgcgagg ccacctccgc cgacgcccgc 4680
   gtgcgcacca tcaagctgtc gcctacctat cagcacgtgc cactgcttga gggtgcctca 4740
   agggcgggtg cagagcccct cgcggggcct ggaatctctc caggggcccg gaagcaggcc 4800
   tggctgccgg cagaccacct gagcaaggtt cccgagaagc tggcggctgc cccgctgtct 4860
   gtggccagca aggcactgca gaaactggcg gcgcaagagg ggccactctc gctgtcccga 4920
   tgcagctccc tttcctcgct gtcctcggcc ggccgcccag gccccagcga gggtggtgac 4980
   ctggatgaca gtgactcctc cctggagggg ctggaggagg ccggccccag cgaggctgag 5040
   ctggacagca cgtggcgggc gcccggggcc acctcgctgc ccgtagccat tccggctccc 5100
   cggcgtaacc gaggccgggg cctgggggtg gaagacgcca cgccgtccag ctcgtcggag 5160
   aactacgtgc aggagacacc gcttgtgctg agccgctgca gctctgtgag ctcgctgggc 5220
   agcttcgaga gcccgtccat cgccagctcc atccccagtg aaccttgcag cgggcagggc 5280
   agcggcacca tcagccctag cgagctgccc gacagccccg gacagaccat gcctcccagc 5340
   cggagcaaga cgccaccgct ggcgcccgcg ccacagggtc cccccgaggc cacccagttc 5400
   agcctgcagt gggagagcta cgtgaagcgc ttcctggaca tcgccgactg ccgggagcgc 5460
   tgccggctgc catctgagct ggacgcaggc agcgtgcgct ttaccgtgga gaagccagac 5520
   gagaacttct cgtgcgcctc cagcctcagc gcgctggcct tgcacgagca ctacgtgcag 5580
   caggacgtgg agctgcggct gctgccctcg gcctgccccg agcgcggcgg gggcgccggg 5640
   ggcgccggcc tccattttgc agggcaccgg cggcgggagg aggggccggc gcccacgggt 5700
   tctcgccctc gcggcgccgc ggaccaggag ctggaactgc tgcgggagtg cctgggagcc 5760
   gccgtgcctg cccggctgcg caaggtggcc tccgcgctgg tgccaggtcg ccgcgcactc 5820
   cccgtgcccg tctacatgtt ggtgcccgcc ccggccccgg cccaggagga cgactcctgc 5880
   actgactccg cggagggcac gccggtcaac ttctctagcg ccgcctcgct cagcgacgag 5940
   acgctgcagg gaccccccag ggaccagccc gggggaccag cgggcaggca aagacccacc 6000
   ggccgcccca cctctgccag acaggccatg gggcaccggc acaaggcggg aggcgccggc 6060
   cgcagcgcgg agcagtctcg gggcgcgggc aagaaccgag cagggctgga gctgcccctg 6120
   ggccggcccc cgagcgcccc cgcagacaag gacggctcaa agcccggccg gacccgcggg 6180
   gacggggcgc tccagtcgct gtgcctcacg acgcccactg aggaggccgt gtactgcttc 6240
   tacggcaacg actcggacga ggagcccccg gcggccgcgc ccacgccaac ccaccggcgc 6300
   acatcggcca tccctcgcgc ttttacgcgg gagcgtccgc agggccggaa ggaggcccct 6360
   gccccgtcca aggctgcacc agctgccccg ccgcccgccc ggacccagcc cagcctcatt 6420
   gctgacgaga ccccgccctg ctactccctg agctcctccg ccagctccct cagcgagccc 6480
   gagccctcgg agccgccggc cgtccatcca cgaggccggg agcccgcggt caccaaggac 6540
   ccgggcccag gaggcggacg cgacagctcg cccagcccgc gggccgcgga ggagcttctg 6600
   cagcggtgca tcagctcggc cctgcccagg cgccggcccc ccgtgtctgg cctgcggcgc 6660
   cgcaagcccc gagccacccg gctggatgag cggcccgcag aggggtcccg ggaacgcggc 6720
   gaggaggcag cgggctcgga ccgggcctcc gacctggata gcgtggagtg gcgcgccatc 6780
   caggagggcg ccaattcaat tgtcacgtgg ctgcaccagg cagcagctgc cacgcgggag 6840
   gcctcgtccg agtccgactc catcctgtcc ttcgtatccg ggctgtcagt gggatccacc 6900
   ctacagcccc ccaagcacag gaagggacga caggcggagg gagaaatggg cagtgcccgg 6960
   cggccagaga aaaggggcgc agcctcagtc aagaccagcg ggagcccccg ttcccctgca 7020
   ggccccgaga agccacgtgg cacacagaag accacgcccg gggtgccagc tgtgctccgg 7080
   ggacgaacag tgatctacgt ccccagcccg gcaccccgtg cccagcccaa agggaccccc 7140
   ggcccccgcg ccacaccgcg gaaggtggcg cccccttgcc tggcacagcc cgcggctcca 7200
   gccaaagtcc cgagccccgg gcagcagcgg tcgcggagcc tacaccggcc tgccaagacc 7260
   tcggagctgg cgacgctgag ccagcccccc agaagcgcca caccgcccgc ccgcctcgcc 7320
   aagaccccct cctccagctc ctcccagacc tcgcccgcct cccagcccct gcccagaaag 7380
   cgccccccgg tcacccaggc tgctggggcc ctgcccggcc ccggagcctc cccggtgccc 7440
   aaaacgccgg cgcgcaccct tctggcgaag cagcacaaga cgcagagatc gcccgtgcgg 7500
   atcccgttca tgcagaggcc ggcccggcgt gggccgccac cgctggctcg ggcagtcccg 7560
   gagccgggcc ccaggggccg ggcggggacc gaggcgggcc cgggggcgcg cgggggccgc 7620
   ctgggcctgg tgcgtgtggc ctcagccctc tccagcggca gcgagtcctc cgaccgctcg 7680
   ggcttccggc gacagctaac cttcatcaag gagtcgccgg gcttgcggcg ccgccgctcc 7740
   gagctgtcct cggccgagtc cgcggcctct gccccccagg gcgcctcgcc ccgccgcggc 7800
   cggcccgcgc tgcccgccgt cttcctctgc tcctcgcgct gcgaagagct ccgagcggca 7860
   ccccggcagg gcccggcccc ggcccggcag cggccccccg cggcccgacc cagccctggc 7920
   gagcgccctg cccggcgcac cacctccgag agcccgtccc gcctgcctgt gcgcgcgccc 7980
   gccgcccggc cggagactgt caagcgctac gcgtcgctgc cgcacatcag cgtggcccgc 8040
   aggcccgacg gcgccgtccc cgcggcccct gcctcagccg acgccgcgcg ccgcagcagc 8100
   gacggggagc cccggccgct ccccagggtg gccgcgccgg gcacgacctg gcggcgcatc 8160
   cgagatgagg acgtgcccca catcctgcgc agcacgcttc ccgccacggc cctgccactg 8220
   cggggctcca cgcccgagga cgccccggcc gggcccccgc cgcgcaagac cagcgacgcc 8280
   gtggtccaga ccgaggaggt cgccgccccc aagaccaact ccagcacgtc cccgagcctg 8340
   gagaccaggg agccccccgg ggcccccgcc ggcggccagc tctccctcct cggcagcgac 8400
   gtggacggtc ccagcctcgc caaggctccc atctccgcac ccttcgtgca cgagggcctg 8460
   ggggtcgccg tggggggctt ccccgccagc cggcacggct cccccagccg ctcggcccga 8520
   gtacccccct tcaactatgt gcccagcccc atggtggtcg cagccaccac cgactcggcc 8580
   gcggagaaag ccccggccac tgcctccgcc accctcctgg aatagtggcc taggccggcc 8640
   ttctggaacg ttctctcccg gccctgcggc gcggtctggc tgccccatgg gcctgcgctg 8700
   tagacgtccc ccataggtcg ccccagggcc tctgcccacc cgagccccac cactctcaga 8760
   acccccgccc agcgcacggc gacctcgcgc ctcaccggaa gaccttgcct ctgtgccgcg 8820
   gaggtccagg aggaaacggg gcggccgcta ggcctcaagt cccgaccgtg gagcgctggc 8880
   aagggcgtcc tggcccagcc ctgagcgcgc ggcccttccc ctgtcggaag ccgttgcttg 8940
   accccgggcg agggaggcgg tagcctccgg gtccgggtct gggtctgggt ccgctgcttc 9000
   gcagggacag cgctggggag gtgacggcgc ccgccgcagg tggggcgagg ctgggggagg 9060
   gcggcgccgc ggcgggcctg ccagctgggg gcctttgcgg cgcgcagggg cgaagcctgt 9120
   aatcactgca gccgccggta attcgctaat gagggctttg cagggattgt tttcattctc 9180
   agccccagct gtgggagtgc gggtgggggt gtggccgagc cccggcagga agccccgccc 9240
   agacggtgtt cagggaaccc ggagcccaag cgctccggcg gagcccaaaa gggtgggggt 9300
   gggaggggca gaggccaacg gatccccctg cctgtcgcac cccttggcgg gagacgggaa 9360
   ggcagcgggc tgcgtacgat gggacctggt gcagacgccg ggccggctga catttggacc 9420
   ccatcccaga ggagatgctg gctaccagct ggggcgaccc caagggtcgc tggagtcagt 9480
   atcggcccgg cgcagccgcg gcgggcgagg ccaatggaaa ggagactgag gggagtcccg 9540
   gcagtgagcc cgaggccctg ggacctggag cccgcgctgg cctctcccca gcggagcctg 9600
   cacgttacgg agaccatcac atgtgggcgt ggtcagtgcc caggaccgca ccgctgctca 9660
   tcttgtccct tttcaattcc cttctggttc atgatgcata aagcgctagg ccctagaact 9720
   ccagaaacag cacagctggg gcggggaccc agccttgccc tccacccgag gctctgggac 9780
   aaggcgggag gttcgggggc cttccggcag gtgaacgcag ggctggagag tatttggtgc 9840
   cagatgaggt gaaagcttat agaagggcct gaggggctcg gctgcctcat cccctggcgg 9900
   gggaggctgg gagctgggcc tcctgcgtgg ggtgggactc gcaggggccg ggtctccgtg 9960
   actggggcaa cgcctcgtcc tgcagaggga gccgacaacc tcttttctgc agaaaagctc 10020
   cagcaggcgc tgccttcacc cacggatctg cccaggctga aggcacacgc tcaatgcccc 10080
   acgtgccttc tccaggagga acgaaccagg gtttgagggt tgggtggatg gagctcaaaa 10140
   ggaaacccca gccccaccac ggatgaccca tccctcccgt cccatcccca gcatgggcaa 10200
   ggccagcctt tctggcaaaa ggagctgtcc tcaactcagg gccgctgtga gcaaagctga 10260
   ccccagcccc cacccccagt taacactgct gcttctctga atgcatgtca ccctgcaccc 10320
   catgctccgg gcccacaccc tgcaggacaa ggagctccag acaggacgtc cataagtcac 10380
   cgaggtgtgc cacccagcag gtgctggagg tgcccaatgc tccctcctag gacctcgcag 10440
   ccaggcaagg ctgtcaggtt gttttggggg aaaagggggt catggatggc tgaacaaaaa 10500
   gcggggaaaa tgcaggctga gtggggcgac ctcctgcctg ccaggagccc cctttcagga 10560
   cacagcgggg gtctcacact tgctgtcccc atccatggcc cgagggggaa cctggtggtc 10620
   tcttctgagc ttttggactt ggggatgcca aacacgtgct caccctcaca ctcgccccgg 10680
   cccgctgcgc ccctaattgc caaagggtag ggaaatggcg aagccagcca ccaggtcgct 10740
   ggtgacaggg ccagggttat gcaggaaggt ggtgcggcat tgccttccac atatgtaagt 10800
   ctctgggcgg cgccctccca gctccctgcc tctgtttccc catgtgggcc gtggggaact 10860
   cccagagcta cctcttgggg gagcgtggtg gcagcgatga tggggagacg cctggaagct 10920
   cacagaactt gggtctggct ggctcctgcc cgtgacgcct tgcccagcag caaggtgcgc 10980
   aacatggctg ccagccccgc ctcccacccc caccccgagt cctgagctca ctttcgcctt 11040
   ctccatcccc tgccgtgggg gccacagcca cacctcaccg cccagtccag ctgtgctcca 11100
   gaaggggaca ggcagtccgc ggtctctgga caatcaactc aaggtacgcc cactgcaagg 11160
   cctccctccc accgcggccc ctgcctggcc acctggcctc tctgcaccag ggtgacaagg 11220
   ggtcctcgtc tgccccccaa tgctccaggg ccagtcctaa ggagctgagg gtctgaggac 11280
   gcagggaggg tggaggtgtc ctgaggctga tggacagtga ccgccactgg cccccaacat 11340
   gaccacacgt gggtgctgaa ctcggggcgc cgtgcccacc ggcatggtcc tcccgagctc 11400
   cgacagcatt acctcacccg gccccatctg ttgccccggt ccagccctga tggcgcgcgc 11460
   ctggtctgtc tgattcccct agccgccacc ccacgtttct gtaccgggtt tctgcagtgt 11520
   taaacggacg tgtaaatagt ggtaaatagt gaaagcctgt ccttccctaa atgtaaagcc 11580
   atctgtccgg cgtaaggacg acaccgtcag ctgtccgact cgcacacatt taataaactg 11640
   agctcttgc 11649

<210> 138
<211> 879
<212> DNA
<213> Homo sapiens

<400> 138
ctcctcacag aagcctggag ctgggcatcc aagaagaagc agcctcattt gttttctggt 60
gtcatcgtag gtggccacct atggcttttg ggaatgtaaa aagggcagct ctctggcatg 120
ttcctgactg aggatctcat aacatttaac ttgaggaact tcctcctttt ccagctttgg 180
gagtcaagct tctcacctgg ggcgggtggg ttctgcacca ccctcccacc ctccttcctc 240
cgtgtggacg atagagccac atccagcacc acggacagct cccgggcgcc ttcatctcct 300
cgtcctccag gcagcacaag ccattgtgga atctccacca ggtgtacaga acggtgcctc 360
tgcgtcctgc cactcaggac ctctcaagtc cccgatgtga tggctcctca gcatgatcag 420
gagaaattcc atgatcttgc ttattcctgt cttgggaagt ccttctccat gtctaaccaa 480
gatctatatg gctatagcac cagctctttg gctcttggct tggcatggct aagttgggag 540
accaaaaaga agaatgtact tcatctggtt gggctggatt ccctctgata agccttccca 600
gttgactgaa agatgaggct aggctctagc aagttgaagt caaaccagct ccttcaagaa 660
gctttgagca gaatgaagtg gggaggaccc agcttccagc ccaggaagcc cactgtacct 720

ggagccatct gggataagac tttgacccat gactcccata tccacagcct gtccatccta 780
gcccatccca gtttatcctg tatcatttga gctgggattc ccacatcctc tgagttggaa 840
gtcccatctc aagtcttcaa taaagactct tgaatattg 879

<210> 139
<211> 3626
<212> DNA
<213> Homo sapiens

<400> 139
ccacgcgtcc ggaacgtctt tttgcggacg ccctcggagc agccgcgatg gccagcacca 60
ggagtatcga gctggagcac tttgaggaac gggacaaaag gccgcggccg gggtcgcgga 120
gaggggcccc cagctcctcc gggggcagca gcagctcggg ccccaagggg aacgggctca 180
tccccagtcc ggcgcacagt gcccactgca gcttctaccg cacgcggacc ctgcaggccc 240
tcagctcgga gaagaaggcc aagaaggcgc gcttctaccg gaacggggac cgctacttca 300
agggcctggt gtttgccatc tccagcgacc gcttccggtc cttcgatgcg ctcctcatag 360
agctcacccg ctccctgtcg gacaacgtga acctgcccca gggtgtccgc actatctaca 420
ccatcgacgg cagccggaag gtcaccagcc tggacgagct gctggaaggt gagagttacg 480
tgtgtgcatc caatgaacca tttcgtaaag tcgattacac caaaaatatt aatccaaact 540
ggtctgtgaa catcaagggt gggacatccc gagcgctggc tgctgcctcc tctgtgaaaa 600
gtgaagtaaa agaaagtaaa gatttcatca aacccaagtt agtgactgtg attcgaagtg 660
gagtgaagcc tagaaaagcc gtgcggatcc ttctgaataa aaagactgct cattcctttg 720
aacaagtctt aacagatatc accgaagcca ttaaactaga ctcaggagtc gtcaagaggc 780
tctgcaccct ggatggaaag caggttactt gtctgcaaga cttttttggt gatgacgatg 840
tttttattgc atgtggacca gaaaaatttc gttatgccca agatgacttt gtcctggatc 900
atagtgaatg tcgtgtcctg aagtcatctt attctcgatc ctcagctgtt aagtattctg 960
gatccaaaag ccctgggccc tctcgacgca gcaaatcacc agcttcagtt aatggaactc 1020
ccagcagcca actttctact cctaaatcta cgaaatcctc cagttcctct ccaactagtc 1080
caggaagttt cagaggatta aagatttctg ctcatggcag atcttcttcc aatgtaaacg 1140
gtggacctga gcttgaccgt tgcataagtc ctgaaggtgt gaatggaaac agatgctctg 1200
aatcatcaac tcttcttgag aaatacaaaa ttggaaaggt cattggtgat ggcaattttg 1260
cagtagtcaa agagtgtata gacaggtcca ctggaaagga gtttgcccta aagattatag 1320
acaaagccaa atgttgtgga aaggaacacc tgattgagaa tgaagtgtca atactgcgcc 1380
gagtgaaaca tcccaatatc attatgctgg tcgaggagat ggaaacagca actgagctct 1440
ttctggtgat ggaattggtc aaaggtggag atctctttga tgcaattact tcgtcgacca 1500
agtacactga gagagatggc agtgccatgg tgtacaactt agccaatgcc ctcaggtatc 1560
tccatggcct cagcatcgtg cacagagaca tcaaaccaga gaatctcttg gtgtgtgaat 1620
atcctgatgg aaccaagtct ttgaaactgg gagactttgg gcttgcgact gtggtagaag 1680
gccctttata cacagtctgt ggcacaccca cttatgtggc tccagaaatc attgctgaaa 1740
ctggctatgg cctgaaggtg gacatttggg cagctggtgt gatcacatac atacttctct 1800
gtggattccc accattccga agtgagaaca atctccagga agatctcttc gaccagatct 1860
tggctgggaa gctggagttt ccggccccct actgggataa catcacggac tctgccaagg 1920
aattaatcag tcaaatgctt caggtaaatg ttgaagctcg gtgtaccgcg ggacaaatcc 1980
tgagtcaccc ctgggtgtca gatgatgcct cccaggagaa taacatgcaa gctgaggtga 2040
caggtaaact aaaacagcac tttaataatg cgctccccaa acagaacagc actaccaccg 2100
gggtctccgt catcatgttt gatttgacag tttgagaggc acctcagagt ttccttcttt 2160
accccgctga gacagtggga gagcctgaag cagaatcctg aacacggctc tagataagga 2220
ggggcagatt ttctgcagca agcactgtca agacagcggc aggcctggga tggagcccat 2280
ctctccagtt cctccctcag tggaggagat ccctgtgcct ggggaagcag tcccggcccc 2340
cacccctccg gaatctccca ccccccactg tcctcccgct gccccgggtg gtgagcgggc 2400
aggaacctgg cgccgccacc gagactgagc ctcctgcaga cgggcgaagc cgcctgctgc 2460
cgcccaggaa gccagccctc tgctcggcct cgccggcctc cctgctgcag gcctccctct 2520
cttcaccgcc tgcgcctgag ttcgcgggtc ctccgcaggc cgcctgggaa ccggagcctg 2580
gcgtgccgga gcctggcctg gtgctctggg ctctgccttc tggttcctgg aggcatcaaa 2640
ggctgcatcc gttctgccaa cagctgttcg gagagactcg ttccagatca tcccgtcatt 2700
ttcagtttgt tggacatttt acagcttcac caggagaatg tgcaacttta ttccagcatt 2760
cgatgcattt ttatagaaac actttggaaa cactttggat gaaccaaggc cttttcctta 2820
tttaagtaga ctcagaacac tccctttctt ttcttttctc tctctctctc tctttttttt 2880
tacgaaagac ttagaattgc atttgtcctt ttgtgggtgt cctgtgagag gtgatatggg 2940
ggctaagagg actggctttc taatagaaga agtgagcgcc tgagaggaca atttggtcat 3000
tggacacgga ttgcaggctt tgagaagcgc tcagaggccc aggcggcggg gctcagccat 3060
tcggcttggg gcaccaggct ccccagagac aatgctcagt attcattcat acacagacga 3120
tggaagaagc cacttcttcc ctgggcggtg tgggtttccc ccagctcttc ccacacgtgt 3180

gttaggaaat gcccgtgaac ttgccctctg ggctttttaa tgagaggctt ggcgcatgcg 3240
gcacccagcg gctgcttccc tgcaagccag cgacttgccg agcagaatga gctctgctcc 3300
tgagccccgg tagctgcttc ctcatctgca tttcatctgg agcagggcag gtaaccacag 3360
gaggaggcat gcccgaggca gccaggactg ggcgggaagg tgctccctgg atatactctg 3420
tgaaccttct ggaaggctgc tggcagtttt tcctttttgt ccaccaccct gctcttttta 3480
ataattgtac ataatccgtg tatttgtttt acctgctcat cttctaaact ggcgagccct 3540
atagttcgtt ctcattgtta gattttgcct tttacaagtg tccccaacct gcaataaact 3600
tttccctctt gaaaaaaaaa aaaaaa 3626

<210> 140
<211> 5978
<212> DNA
<213> Homo sapiens

<400> 140
gaagaaatcg agaaaacaag aaagcacgaa agcaagcaag caagaaagca agcaagaaag 60
aaacaaaaga aagaaagaaa gaaagaaaac aggaaagcaa gaaagcacga aagcaagcaa 120
gcaagcaaga aagcaagcaa gaaagaaaca aaagaaagaa agaaagaaag aaagaaaaag 180
aaaacaggaa agcaagaaag cacgaaagca agcaagcaag caagaaagca agcatgaaag 240
aaacgaaaga aagaaagaaa gcaagaaaac gggaaagcaa gaaagcacga aagcaagcaa 300
gcaagcgagc gagagagaga gagagagaga gagagagaga gagagagaga gagagagagg 360
ctgggcgcgg tggctcacgc ctgtcatccc agcactttgg gaggctaagg caggcggacc 420
acctgaggtt gggagtggga gaccagcctg accaacatgg aaaaacaccg tctctactaa 480
aagtacaaac atcagccagg cacggtggcc catgcctgta atcccagcta atcaggaggc 540
tgaggcagga gaatcgcttg aacctgggag gcggagggtg cggtgagccg agatcgcacc 600
attgccctct agcctgggca acaagagtga aactctgtct caaaaaaaaa aagaagaaga 660
agaagaagaa gaagaagaag aagaagaaga agaagaagaa gaagaagaag aagaagagaa 720
agtaataaag aaagaaagaa agaaaaggca aggccaggca agtccaggca aggcaaatct 780
acctgctttc actacatctg gggagaatca ggaaagtccc caacaacaac aaggcctaaa 840
gtggagctgc catctgtcaa acccgagcgg aagagtccac gcgggttaaa gacacgaaga 900
aagacaagga aacccctgac caaggagaag aacaatcggg cccagccagg gtctgtctcc 960
cggggttgtc tgggcaacca gggagggcgg gcctccgaga ctccgtctcg aaacatcaat 1020
cacgataata acataaaatg aagttaaaaa aagaaatcac gcataattcc taacgtgttt 1080
gaggcctcga aaggcgagag gcgtacgtgt atgtcacggt ggggttgttc tgttttgttg 1140
tttttttctt ttttcttttc ttctttttcc ccagaaactc actttttaat tattttgttg 1200
cgtttcattt tcattttcat tttttggaga cggagtctcg ctctgtcgcc caggctgggt 1260
tgcagtggcg cgatctcggc tcactgcaac ctccgcctcc caggttcaag cgattctcct 1320
gcctcagctc ggcctcccga gtagctggga ttacagacag tacagcacag cacagcgccc 1380
ggctaatgtt gtgtattgtg agtagagacg gggtttcacc atactggccc tgttggtctg 1440
accgcctctt gatccaccgg ccttggcctc ccaaagtgag gggatgacag gcttgagcca 1500
ccgcgcaggg cccatttatt tattttattt tatttattta ttttaattta tgtatgtatg 1560
tatgtatgta tgtatgtatt tatttatgta tttatttttg agacggagtt tcgctcttgt 1620
tgctcagact ggagtgcgag ctagcgcctc tctgggtgtc cgtgccagtg ctgcgcggga 1680
aggcaccacc accacagccg ccgccgctgg ccagggtgct ggacaagacg ggcccgtcgg 1740
ccacctcgcc agctcaggca cccgctgcag ccgtcgctgt ctcctcccag accggtcccc 1800
atggaggaga tggaagagga gttgaaatgc cccgtgtgcg gctccttcta tcgggagccc 1860
atcatcctgc cctgctctca caatttgtgt caggcgtgcg cccgcaacat cctggtgcag 1920
accccagagt ctgaatcccc ccagagccat cgggccgcgg gctccggggt ctccgactat 1980
gactatctgg acctggacaa gatgagccta tacagcgagg cggacagcgg ctatggctcc 2040
tacggggggt tcgccagcgc ccccactacc ccgtgccaga agtcccccaa cggcgtccgc 2100
gtgtttcccc cggctatgcc gccaccggcc acccacttgt caccggccct ggccccggtg 2160
ccccgcaact cctgtatcac ctgcccccag tgtcaccgca gcctcatcct ggatgaccgg 2220
gggctccgcg gcttccccaa gaatcgcgta ctggaagggg taattgaccg ctaccagcag 2280
agcaaagccg cggccctcaa gtgccagctc tgcgagaagg cgcccaagga agccaccgtc 2340
atgtgcgaac agtgcgatgt cttctactgc gatccgtgcc gcctgcgctg ccacccgccc 2400
cgggggcccc tagccaagca ccgcctggtg cccccggccc agggtcgtgt gagccggagg 2460
ctgagcccac gcaaggtctc cacctgcaca gaccacgagc tggagaacca cagcatgtac 2520
tgcgtgcaat gcaagatgcc cgtgtgctac cagtgcttgg aggagggcaa acactccagc 2580
cacgaagtca aggctctggg ggccatgtgg aaactacata agagccagct ctcccaggcg 2640
ctgaacggac tgtcagacag ggccaaagaa gccaaggagt ttctggtaca gctgcgcaac 2700
atggtccagc agatccagga gaacagtgtg gagtttgaag cctgtctggt ggcccaatgt 2760
gatgccctca tcgatgccct caacagaaga aaagcccagc tgctggcccg cgtcaacaag 2820
gagcatgagc acaagctgaa ggtggttcga gatcagatct ctcactgcac agtgaaattg 2880

cgccagacca caggtctcat ggagtactgc ttggaggtga ttaaggaaaa tgatcctagt 2940
ggttttttgc agatttctga cgccctcata agaagagtgc acctgactga ggatcagtgg 3000
ggtaaaggca cactcactcc aaggatgacc acggactttg acttgagtct ggacaacagc 3060
cctctgctgc aatccatcca ccagctggat ttcgtgcaag tgaaagcttc ctctccagtc 3120
ccagcaaccc ctatcctaca gctggaggaa tgttgtaccc acaacaacag cgctacgttg 3180
tcctggaaac agccacctct gtccacggtg cccgccgatg gatacattct ggagctggat 3240
gatggcaacg gtggtcaatt ccgggaggtg tatgtgggga aggagacaat gtgcactgtg 3300
gatggtcttc acttcaacag cacatacaac gctcgggtca aggccttcaa caaaacagga 3360
gtcagcccgt acagcaagac cctggtcctc caaacgtctg aggtggcctg gtttgctttc 3420
gaccctggct cggcgcactc ggacatcatc ctctccaatg acaacctgac agtgacctgt 3480
agtagctatg atgaccgggt ggtgctaggg aagactggct tctccaaggg catccactac 3540
tgggagctca cggtagatcg ctatgacaac caccctgatc ctgcctttgg tgtggctcgc 3600
atggacgtga tgaaggatgt gatgttagga aaagacgaca aagcttgggc aatgtatgtg 3660
gacaataacc ggagctggtt catgcacaac aactcgcaca ccaacagaac tgagggaggg 3720
atcacaaaag gggccacaat tggggtcctc ctcgacttaa atagaaaaaa cttgacattt 3780
tttatcaacg atgaacaaca aggtcccata gcatttgata acgtggaggg cctcttcttc 3840
cctgcggtca gcctgaacag gaacgtgcag gtcacgctgc acaccgggct cccagtcccc 3900
gacttctact ccagcagagc atcaatagcc taaggatgtg ccgtggaggc gccagctgcc 3960
tgttcttacc tccgcctgcg agagccacag caaggagctc agccagccgt ggtggggtgc 4020
agagttggca ggagtgggag aaggaggaga gaaaagctgg tcctctgcag tctttacacc 4080
cacagctctg cccttttccc tttcaacctc tcctccgctg tcatgcctgc ttccgcttcc 4140
atgtccaaca attctaacca acaaaggacc tagacagccc accaagtcac ttggttccca 4200
ctcccagatt ttgcttttat ttaacttaat tttttatgta ggtgagttat attttctttc 4260
ttttctgatc aggttattgg tgacttactg gactggcacc gccaggagaa aattctcctg 4320
ctaacttttt ttcttaagct ttctgtcaaa caatgaggtt gtagggggag gtagggaaga 4380
atgagctgaa tttgtagcat acagcttacc tgctagaatg ttcttaccct cttacctctc 4440
ctgtagcgtt agctctgcag agctaagctt tgggagaatg aatctatcac tgagaagttt 4500
tactactcat tgaagcacaa aaatatccgc tacacaggtt cacatcaggg taggatcgtt 4560
aggatggctc tataattagg ataggccaga accctggcca ctgtataaaa ttggaaagtc 4620
tctaagacac aggcaaacca ggggacttaa tgatttggtt cgtttattta ataaaagctt 4680
ttaattgaga acctactatt gaccaggcac tgttcaagac actagaacat tatgttctag 4740
acactgtggg gacacagtgg tgaacagaca ggcatggtct ctgcccttcg gagcttatat 4800
tccagtagga gaaaaagcaa aaataaaata attataaatg gtcatatgta ctagaaagga 4860
aatgggcaag ggtcaaagag actcatgggc aagatctttc aggcttccta gaatgatgct 4920
aaaaatccac acattctggc catagcacca gaaatgtacc aactcaatgc cttttcagct 4980
gcactattta attatggtac ctgctgccac tcatcacaga gcccttcttg gtagaaatga 5040
tgtcagcatt cttatttgct gatgctgcta tgataccacg ccttaaaaat tgacagttga 5100
aaaaaaaaag agtgaccaga gggcaaagga cccattctca gtaatgggag atcttgagtt 5160
gcagtccaca gcattcacat tctcaggata aacatgcttg tctccatata tactatctgt 5220
gcctgttatg aatgaaaaat ccatccactc tgctgccatt cacagcctaa tcttctggag 5280
tagtccaaac aatgtttgga aaaacatggg actgtatgtc attactatga ctgatagcag 5340
aataataatg catcttactt ctatatagag atagacttac ataggttacc cttgaaattc 5400
attagtttgt cataaagttt taggaaaggt aggacccgga aagaagttct aattagttgt 5460
ctaaatattt ttcagtgagc caagaaattc accatgaaaa aacaagaata acaaatagaa 5520
gggaagagat aggatgggaa agctaacaaa ttaaagtttt ggcaaaaagg aatatatgta 5580
aatagctaat tatttacttt tgtgcttact ttatttagat tatttctatc agttacaatc 5640
tttttctagt taagtgtacc taatttatgg aatgggtgct atcctgttta tgtgtgtctt 5700
ggtttttctt ggctacagaa aaactgttgc agggcaacac tagtttgata tttgatttac 5760
tctccaatga gactcaatgg ctgggccgtg gtagactcat agttcctctt gttctttatt 5820
aaattcatcc tgctaattag atttctagtg acttgtaaca tgtagtttac actgaattgc 5880
aattacagat gcatacaact actatactag aaaaaattgt catttctgat gtgccaataa 5940
atgattttta tgagagaaaa aaaaaaaaaa aaaaaaaa 5978

<210> 141
<211> 1707
<212> DNA
<213> Homo sapiens

<400> 141
gggagaaagc ctgttgcgtg gaagataagg cggcgcggga agtggacaca gggtgggctg 60
gagctcagat ctaactggac tctcgctcct gctggctgga catggaggat ttggaggaag 120
atgtaaggtt tattgtggat gagaccttgg actttggggg gctgtcacca tctgacagcc 180
gtgaggagga agacataaca gtgttggtga ctccagagaa accacttcga cggggcctct 240

cccaccgaag tgacccaaat gcagtggcac ctgcccccca gggtgtgagg ctcagcctag 300
gccccctcag tccagagaag ctggaggaga tcctcgatga ggccaaccgg ctggccgctc 360
agctggagca gtgtgccctg caggatcggg agagcgcagg cgagggcctg gggcctcgcc 420
gagtgaagcc cagtcctcgg cgggagacct ttgtgctgaa ggatagtcct gtccgagacc 480
tgctgcccac tgtgaactct ttgacgcgga gcaccccctc cccaagcagc ctgacgcctc 540
gactccggag taatgatagg aaggggtcag tcagggctct ccgggctaca tctggaaaga 600
ggccctccaa catgaagagg gagtcaccca cttgcaatct gttccctgca tccaaaagcc 660
cagcatcttc tcctcttacc cgatcgactc ccccagtccg ggggagagcc gggcccagtg 720
ggagagcagc agccagccca cccaccccca tcagatccgt cctggcccca cagccttcta 780
ccagcaactc tcaacgcctg ccccggccgc agggagcagc tgctaaatct tccagtcaac 840
tgcccattcc ctcggccatc cccaggcctg ccagccgaat gccactcacc agccggagtg 900
tgccacctgg cagaggtgcc ctacctccgg attctctgtc aactcgaaaa gggcttccaa 960
gaccaagcac tgcaggacac agagtgcggg aaagtggaca caaggttcct gtttcccagc 1020
gactaaatct tcctgtcatg ggtgccactc gcagcaatct gcagcccccc aggaaagtgg 1080
cagtcccagg acctaccagg taaagagatc aggacagcaa gcaagacttc agtagcaaac 1140
cactacagtc agtacctgga ctcgcctcta cccagcagac cctgactcca gcagattctg 1200
gcccagggac aggaggaaga gatgccacca gggctggtct cccaggagta gagaccatgg 1260
gaaatggggt ggattaggat tgagctggag aagacttaaa ctctctgggt tgaaagaaga 1320
ttaggggaaa agaggtcacc ttccagcagt gaaatgaaca aatagaagat gagaagtaca 1380
ggcaagtggt ttgtctttat ccacccccac tgttgtggtc agccccagag aattttatct 1440
tcttccttgg cattggttca ctggacattt ccacgtgagc ggcctccgta gctaacctcc 1500
ctgccctctg aggagccatc ttcctgaatc gcattctcta ctggactctg gcctgcttgg 1560
agaggtggca gcaggcacct ggtcttcaga aattgtttcc tgtgaattct gtgactccta 1620
ataggccagt ttgtgataag cttactctat gagtcttcat ttttctaaaa taaagtgaat 1680
gtatttttaa aaaaaaaaaa aaaaaaa 1707

<210> 142
<211> 1641
<212> DNA
<213> Homo sapiens

<400> 142
atgggctctg actctcggct tcctgagatg gaggagaaag ggtctgggga caaagctggc 60
tggtccggag ctcttctggc tgaggtgaag aaccagggct tgaaactatg tgagtggatg 120
ctggttttga aggcagagct ggtggaatgg tacacgtcag tgattgtagg gcaacagggg 180
catcgattaa aaatggacct tcttaaggag aggcaccacc gcaagtacct gcagaagcct 240
atcaagcgct gcagcggcct catgggcctg aactggagtt tgagaccagc catctcttcc 300
tcaccccttg cgaggagtca agagaaacac cacggggcaa agggaaacga aggagttaag 360
gcgttgagct tcagaaagct cagaaaacta aagcgacccc ccactcctcc aagccaggct 420
aagagctggc gggggcgcac gggcagcgct gcggacagcc cgggagccgc ggcgatggcg 480
gtgcaggcgg cgctcctcag cacgcaccct ttcgtgccct tcggcttcgg gggctccccg 540
gacgggctag ggggcgcctt cggagccctg gacaagggct gctgtttcga ggacgatgag 600
accggggctc cggcgggtgc gctgctgtcg ggagccgaag gaggggacgt gcgcgaggcc 660
acccgcgatc tactcagctt cattgactcg gcgtccagca acatcaagct ggcgctggac 720
aagccgggca agtcgaagcg gaaggtgaac caccgcaacg ccccgacagt cgcggccccg 780
gcccacggca aggctgcccc ccggcgggag gcgtcgcagg ccgccgcggc cgccagcttg 840
caaagccgaa gtctggccgc gctcttcgac tcgctgcgcc acgtccccgg gggtgccgag 900
ccggcggggg gtgaggtggc tgcgccggcg gccgggctag aggtgcgggg cactgggggc 960
gcgggagggg acgtggcagg ccccgcgggg gccacggcga tcccaggggc caggaaggtc 1020
ccgctgcggg cacgcaatct gcctccgtcc ttcttcacgg agccgtcccg ggcaggcggc 1080
ggcgggtgtg gcccgtcggg gccggacgtg agcttgggcg acctggagaa gggcgcggag 1140
gccgtggagt tctttgagct gctgggcccc gactacggcg ccggcacgga ggcggcagtc 1200
ttgcttgccg ccgagcctct cgacgtgttc cccgccggag cctccgtact gcggggaccc 1260
ccggagctgg agcccggcct ctttgagccg ccgccggcag tggtgggaaa cctactgtac 1320
cccgagccct ggagcgtccc gggctgctcc ccgaccaaaa agagccccct gactgccccc 1380
cgcggcggct tgaccttgaa cgagcccttg agccccctgt accccgccgc tgcggattct 1440
cccggcgggg aggacgggcg gggccatttg gcctctttcg cccccttctt tccagactgc 1500
gccctgcccc cgccgccgcc gccccatcag gtgtcctacg attacagcgc gggctacagc 1560
cgcaccgcct attccagcct ttggagatcc gacggggttt gggaaggggc gccgggggag 1620
gagggggcgc accgggactg a 1641

<210> 143
<211> 3428

<212> DNA
<213> Homo sapiens

<400> 143
ccacgcgtcc gacgcccccc acccgggagg ggggagagag gcaaaaagta agagaggaaa 60
aaaaatagca ggaagatggc gcccaccaag cccagctttc agcaggatcc ttccaggcga 120
gaacgtttac aagcattgag aaaggagaaa tcccgagatg ctgctcgctc ccgccgggga 180
aaagaaaact ttgagttcta tgaattggcc aagttgttgc ctcttcctgc agccattacc 240
agccagctcg acaaggcatc catcattcga cttacaatta gctatctgaa aatgagggac 300
tttgctaacc agggggaccc tccgtggaac ttgcgaatgg aaggccctcc acctaacaca 360
tcagtaaaag gtgcacagcg aaggagaagc cccagtgcac tagccattga agtatttgaa 420
gcacatttgg gaagccacat tttgcagtcc ctggatggct ttgtatttgc actaaatcag 480
gaaggaaaat ttttgtacat ttccgaaaca gtctccatct acctaggcct ctcacaagtg 540
gagctgacag gcagcagtgt ctttgactat gtccaccccg gagatcacgt ggagatggct 600
gagcagctgg gcatgaagct cccccctggg cggggtctcc tgtcacaggg cactgctgag 660
gacggagcca gctcagcatc ttcctcctct cagtcggaga cccccgagcc agtggagtca 720
accagcccca gtctgctaac cactgacaac actcttgagc gttccttttt catccgaatg 780
aaatctactc tgaccaaacg cggtgtgcac atcaaatcat caggatataa ggtgattcac 840
ataacaggcc ggctacgcct gagagtgtcg ctgtcccacg ggaggaccgt ccccagccaa 900
atcatgggtc tcgtggttgt tgcgcatgcc ttgcctcccc ctacgatcaa tgaagtcaga 960
attgactgcc atatgttcgt cactcgagta aatatggacc tcaatatcat ttactgtgaa 1020
aataggatta gtgattatat ggatctgacc cctgtagata tcgtagggaa gagatgctac 1080
cacttcatcc atgctgaaga cgtggagggc atcaggcaca gtcacttgga cttgctgaat 1140
aagggtcagt gtgtgacaaa gtactatcgc tggatgcaga agaacggagg atatatttgg 1200
atacagtcca gtgccaccat agctattaat gccaagaatg caaatgaaaa gaatatcatc 1260
tgggtgaatt accttcttag caatcctgag tacaaggaca cacccatgga catcgcacag 1320
ctcccccatc tgccggagaa aacttccgaa tcctcggaga catccgactc tgagtcagac 1380
tctaaagaca cctcaggtat tacagaggac aacgagaact ccaagtccga cgagaagggg 1440
aaccagtccg agaacagcga agacccggag cccgaccgga agaagtcggg caacgcgtgt 1500
gacaacgaca tgaactgcaa cgacgacggc cacagctcca gtaacccgga cagccgcgac 1560
agcgacgaca gcttcgagca ctcggacttt gagaacccca aggcgggcga ggacggcttc 1620
ggtgctctgg gcgcgatgca gatcaaggtg gagcgctacg tggagagcga gtcggacctg 1680
cggctgcaga actgcgagtc actcacgtcc gacagcgcca aggactcgga cagcgcaggc 1740
gaggcgggcg cgcaggcctc cagcaagcac cagaagcgca agaaaaggcg gaaacggcaa 1800
aagggcggca gcgccagccg ccggcgcctg tccagcgcgt cgagcccagg cggcctggac 1860
gcgggcctgg tggagccccc gcggctgctg tcctccccca acagtgcctc ggtgctcaag 1920
atcaagacgg agatctcaga acccatcaat ttcgacaatg acagcagcat ctggaactac 1980
ccgcccaacc gggagatctc caggaacgag tccccctaca gcatgaccaa gccccccagc 2040
tctgagcact tcccgtcccc gcagggcggc ggcggtgggg gtggcggtgg cggggggctg 2100
cacgtggcca ttcccgactc ggtcctcacc ccgcccggcg ccgacggcgc ggccgcccgc 2160
aagactcagt tcggcgcctc ggccaccgcg gccctggccc ccgtcgcctc cgacccgctg 2220
tcacccccgc tctcggcgtc cccgcgggac aagcaccccg ggaacggcgg cgggggcggg 2280
ggcgggggcg gcggcgcggg gggcggcggc cccagcgcgt ccaactcctt gctgtacact 2340
ggggacctgg aggcgctgca gaggttgcag gcgggcaacg tcgtgctccc gctggtgcac 2400
agggtgaccg ggaccctggc cgccaccagc acggccgcgc agagggtcta caccacgggc 2460
accatccgct acgcgcccgc cgaggtgacc ctggccatgc agagcaacct gctgcccaac 2520
gcgcacgctg ttaacttcgt ggacgttaac agccccggct ttggcctcga ccccaagacg 2580
cccatggaga tgctctacca ccacgtgcac cggctcaaca tgtcaggacc gttcggcggc 2640
gcagtgagcg cagctagcct gacgcagatg cccgccggca acgtgttcac cacggccgag 2700
ggactcttct ccacgctgcc cttccccgtc tacagcaacg gcatccacgc ggcacagact 2760
ctggagcgca aggaggactg aggcgccgcc cgtcctgggc ccggccaggc cccgcttgga 2820
ggaggcatcg tcggcatttt cgtttagacc tttaattcta gcactttgaa ttcgagcagg 2880
tcagcgtctt ctctcgccac gacggtcccc attccacccc ctctttcttt cacctgactt 2940
attctttcgt gtaaagatat gtttattttt tgccttcaga gggtcagacg accagttgcc 3000
tgccgttttg tcttcttcta aggtgtgtgt tgggttgttt tgctttcctt tgcatcttta 3060
ttaagatgtc tttcatgtgt atatgcctct gccatagaat actcagtctt gtggtcaaga 3120
gagttctcaa gtgacaacca ttggggtttc ttcataaaga tcttgatatg atcaagatgg 3180
aaagagacaa gcataaacaa tgtgccctgt ttgactaagt caaatgaaat agggtggttt 3240
ttgtttctgt tcctaattcc tttaaaaaat agggggaata gtattttaga attttatgca 3300
gaatttaatt ctctttttac ggttaagatt ttaagatttt cttacttgca cataaaaata 3360
atttgggttc ttaaacttaa tttctggcct gtgactagaa tgtttaaaaa aaaaaaaaac 3420
cctcgtgc 3428

<210> 144
<211> 2435
<212> DNA
<213> Homo sapiens

<400> 144
ccctgaggct tttcggagcg agctcctcaa atcgcatcca gattttcggg tccgagggaa 60
ggaggaccct gcgaaagctg cgacgactat cttcccctgg ggccatggac tcggacgcca 120
gcctggtgtc cagccgcccg tcgtcgccag agcccgatga cctttttctg ccggcccgga 180
gtaagggcag cagcggcagc gccttcactg ggggcaccgt gtcctcgtcc accccgagtg 240
actgcccgcc ggagctgagc gccgagctgc gcggcgctat gggctctgcg ggcgcgcatc 300
ctggggacaa gctaggaggc agtggcttca agtcgtcctc gtccagcacc tcgtcgtcta 360
cgtcgtcggc ggctgcgtcg tccaccaaga aggacaagaa gcaaatgaca gagccggagc 420
tgcagcagct gcgtctcaag atcaacagcc gcgagcgcaa gcgcatgcac gacctcaaca 480
tcgccatgga tggcctccgc gaggtcatgc cgtacgcaca cggcccttcg gtgcgcaagc 540
tttccaagat cgccacgctg ctgctggcgc gcaactacat cctcatgctc accaactcgc 600
tggaggagat gaagcgactg gtgagcgaga tctacggggg ccaccacgct ggcttccacc 660
cgtcggcctg cggcggcctg gcgcactccg cgcccctgcc cgccgccacc gcgcacccgg 720
cagcagcagc gcacgccgca catcaccccg cggtgcacca ccccatcctg ccgcccgccg 780
ccgcagcggc tgctgccgcc gctgcagccg cggctgtgtc cagcgcctct ctgcccggat 840
ccgggctgcc gtcggtcggc tccatccgtc caccgcacgg cctactcaag tctccgtctg 900
ctgccgcggc cgccccgctg gggggcgggg gcggcggcag tggggcgagc gggggcttcc 960
agcactgggg cggcatgccc tgcccctgca gcatgtgcca ggtgccgccg ccgcaccacc 1020
acgtgtcggc tatgggcgcc ggcagcctgc cgcgcctcac ctccgacgcc aagtgagccg 1080
actggcgccg gcgcgttctg gcgacagggg agccaggggc cgcggggaag cgaggactgg 1140
cctgcgctgg gctcgggagc tctgtcgcga ggaggggcgc aggaccatgg actgggggtg 1200
gggcatggtg gggattccag catctgcgaa cccaagcaat gggggcgccc acagagcagt 1260
ggggagtgag gggatgttct ctccgggacc tgatcgagcg ctgtctggct ttaacctgag 1320
ctggtccagt agacatcgtt ttatgaaaag gtaccgctgt gtgcattcct cactagaact 1380
catccgaccc ccgaccacca cctccgggaa aagattctaa aaacttcttt ccctgagagc 1440
gtggcctgac ttgcagactc ggcttgggca gcacttcggg gggggagggg gtgttatggg 1500
agggggacac attggggcct tgctcctctt cctcctttct tggcgggtgg gagactccgg 1560
gtagccgcac tgcagaagca acagcccgac cgcgccctcc agggtcgtcc ctggcccaag 1620
gccaggggcc acaagttagt tggaagccgg cgttcggtat cagaagcgct gatggtcata 1680
tccaatctca atatctgggt caatccacac cctcttagaa ctgtggccgt tcctccctgt 1740
ctctcgttga tttgggagaa tatggttttc taataaatct gtggatgttc cttcttcaac 1800
agtatgagca agtttataga cattcagagt agaaccactt gtggattgga ataacccaaa 1860
actgccgatt tcaggggcgg gtgcattgta gttattattt taaaatagaa actaccccac 1920
cgactcatct ttccttctct aagcacaaag tgatttggtt attttggtac ctgagaacgt 1980
aacagaatta aaaggcagtt gctgtggaaa cagtttgggt tatttggggg ttctgttggc 2040
tttttaaaat ttttcttttt ggatgtgtaa atttatcaat gatgaggtaa gtgcgcaatg 2100
ctaagctgtt tgctcacgtg actgccagcc ccatcggagt ctaagccggc tttcctctat 2160
tttggtttat ttttgccacg tttaacacaa atggtaaact cctccacgtg cttcctgcgt 2220
tccgtgcaag ccgcctcggc gctgcctgcg ttgcaaactg ggctttgtag cgtctgccgt 2280
gtaacaccct tcctctgatc gcaccgcccc tcgcagagag tgtatcatct gttttatttt 2340
tgtaaaaaca aagtgctaaa taatatttat tacttgtttg gttgcaaaaa cggaataaat 2400
gactgagtgt tgagatttta aataaaattt aaagt 2435

<210> 145
<211> 5264
<212> DNA
<213> Homo sapiens

<400> 145
atggtgccga gactctgtgc atgaccgtgc tggaagaaag cagcatctct ctaatgagta 60
gcgtcgtagc agacttctct gaatttgagg aagatcctca agtatttaat acgcttttcc 120
cctccagacc tatcgtccca ctttcttcta gatccatgga aatctcagag acgagtgttg 180
gcatttctgc cgaggtggat atgagtagtg ttacaaccac acaggttccc cctgcccacg 240
gccgcctctc tgtgccggcg tcacttgatc ctactgctgg ctccttgtct gttgctgaaa 300
cccaagtgac gccatccagc gtgaccactg catttttctc ggtcatcacc agcattctcc 360
ttgactcatc tttctctgtc atagcaaaca aaaacacacc gtcgcttgcc gtcagagacc 420
cgagtgtttt tacgccttat agtctggttc cttcagtgga gtcttcactt ttctctgacc 480
aagaacgttc cagtttttct gagcataaac ccagaggtgc tttggatttt gcatccagct 540

ttttctcaac acccccgctg gaactcagcg gctccatctc ttcgccttcg gaagcacctg 600
cgtctctgtc tctgatgccg agtgacttgt cccccttcac atctcagtct ttttctccct 660
tggttgagac atttacattg tttgactcta gtgatctgca gtcatctcag ctgtctcttc 720
ccagttccac aaatcttgag ttttcgcagc tccagccaag ttccgagctg cctttaaaca 780
ccatcatgtt gctacctagc cgttctgagg tgtcaccatg gtcaagcttc ccttctgatt 840
ctctcgagtt tgttgaagcg tctacggttt cactgacgga ttcagaagct cattttacct 900
cagctttcat tgaaactacc tcctatcttg agtcttcact catttcccat gaatccgcag 960
tcactgcact ggtgcccccc ggctctgagt cttttgacat tttgactgcc gggattcaag 1020
caacatcacc attgaccact gtccacacaa cgcccatttt aactgagtct tctttgttct 1080
caactctgac acctcctgac gaccaaatca gtgctctaga cggtcacgtg tctgtcctgg 1140
cctctttctc caaagccatt cccactggta cggtgttgat cactgacgcg tacctgccat 1200
caggatcctc gtttgtttct gaagcaaccc ccttccctct gcccacagag ctgaccgtcg 1260
tgggcccatc actcacaccc acagaggtgc cactgaacac ctccacggaa gtgagcacaa 1320
ccagcaccgg tgctgccact ggtggtcccc tcgactccac cctgatgggt gacgccgcaa 1380
gtcagagccc cccagagagt agtgctgctc ctcccctgcc atccctgcgt cccgtgactg 1440
ccttcactct cgaagcaaca gtcgacacac caacactggc tactgccaag ccgccatatg 1500
tttgtgatat cacagtcccc gatgcctatc tgatcacaac tgtgctggcc agaagagctg 1560
tgcaggagta catcattaca gcaatcaaag aagtactgag gattcacttc aaccgtgcag 1620
tggaactgaa ggtttacgaa ctatttactg acttcacttt tctggtaaca tccggtcctt 1680
tcgtttacac ggcaatatcc gtcataaatg tgcttataaa cagtaagctt gtccgtgacc 1740
agactccttt aatcctgtct gtgaaacctt ctttccttgt gccagagtcc aggttccaag 1800
ttcaaacagt acttcagttt gtgcctccga gtgtggatac tggcttctgc aacttcaccc 1860
agcgcattga gaaaggccta atgacagctc tctttgaagt gagaaaacac caccagggaa 1920
cgtataacct cacggtgcag atcttgaata tcaccatcag ttcctcaagg gtgactcctc 1980
ggcgggggcc ggtgaatatc atctttgcgg ttaaaagcac acaggatttt ttgaatgggt 2040
cggaagtgag cgagctgctc agaaacttga gtgtggtgga gttcagtttc tatctgggat 2100
acccagtgct gcagatcgca gagcccttcc agtatccaca gctcaactta tctcagttgc 2160
tgaagtcctc ttgggtcaga acagttctcc tgggcgtcat ggagaagcaa ctccagaatg 2220
aagtgtttca agccgagatg gaacgcaagc tggcccagct gctcagcgag gtttccacca 2280
gaaggcggat gtggagaagg gccactgtag ctgcagggaa cagtgtggtg caggtggtaa 2340
atgtgtcgag gctggaggga gatgacaatc cggtacagct catctacttt gtggaggatc 2400
aagatggaga aagactcagt gcagtcaagt cttcggacct gattaacaaa atggacctcc 2460
agagagcagc catcatcttg ggttaccgaa ttcaaggtgt cattgcccag cctgtcgaca 2520
gggtgaagag gccgtctccg gaatcccaga gcaacaactt gtgggtcatt gttggcgtgg 2580
tcatcccagt gctggtggtg atggtgattg ttgtcatcct ctactggaaa ctatgccgca 2640
cagacaagct agactttcag cctgacactg tggccaacat tcagcagcgt cagaagctgc 2700
agatccctag tgtgaagggc ttcgattttg ctaagcagca tctgggtcag cacaataaag 2760
acgacatatt gattattcat gagccagcgc cactgccagg acctctgaag gaccacacca 2820
cgccctcgga aatggagacg gtgccaagcc ccaagtcaaa gatcccttcc aagaatgttc 2880
gtcacagagg aagagtttct ccctcagatg ctgactctac ggtcagtgaa gagtccagcg 2940
agagggacgc aggagataag acgccgggag ccgtcaacga tggcaggtcc cacagagctc 3000
cgcagagcgg gccaccactg cccagttcgg gaaatgagca gcactcatca gcctccatct 3060
tcgagcacgt ggacaggatc tcccgccccc cggaggctag ccggcgggtc cccagtaaga 3120
tccagcttat cgccatgcag ccgatcccgg cacctcccgt ccagcgcccc tccccagccg 3180
accgagtggc ggaaagcaat aaaatcaaca aagagattca gaccgcgctg cggcacaagt 3240
ctgagatcga gcaccatcgc aacaagatcc gcctgcgcgc caagcgccgc gggcactacg 3300
agttcccggt ggtagacgac ctgtcctcgg gcgacactaa ggagcgacac cgggtgtacc 3360
gcagggcaca gatgcagatc gacaagatcc tggaccccac ggccagcgtg ccctccgtgt 3420
tcatagagcc caggaagagc tcacggataa aacgttctcc caagcctcgc cggaaacacc 3480
aggtcaacgg ctgtcctgcc gacgctgaga aggaccggct catcaccaca gacagcgatg 3540
gcacctacag gaggcccccc ggcgtccaca actcagccta catcggatgc ccatcggatc 3600
ctgacctccc agccgatgtg cagacaccat cctcggtgga actggggagg tatccagccc 3660
ttcccttccc ggcctcccag tacatcccac cccagccgtc catcgaggag gcacgccaga 3720
ccatgcactc cctcctggac gacgcctttg ccctcgtggc ccccagcagc cagcctgcca 3780
gcaccgcagg tgtaggcccc ggagtcccac ccggcctgcc cgcaaacagc accccttccc 3840
aggaagagag gcgagccacc cagtgggggt ccttctacag cccagcccag acggccaaca 3900
atccctgcag tagatacgaa gactatggaa tgactccccc gacgggtcca ttgccaagac 3960
caggttttgg ccccggtttg ctgcagtcta cagagctggt gccccctgac cctcagcagc 4020
cacaggcctc cgccgaagcc ccatttgctg ccagagggat ctactcggag gagatgccgt 4080
cggtggcccg gcctcggcct gtcgggggta ccacaggctc ccagatccag cacctgacac 4140
aggtggggat tgccagcaga attggagctc agccagtgga aatcccgcca agcagaggca 4200
gccagtatgg ggggccaggc tggccttcgt acggggagga cgaagcgggg cgaagagagg 4260
ccgtgccaag gacttcaggc agggagccct cagctccttc cgggaacctc ccccaccggg 4320

gactgcaggg ccctgggctg ggttacccca ccagctccac ggaagacctc cagcctggcc 4380
actcctcggc ctctctcatc aaagcaatcc gcgaggagct cctccggctc tcccagaaac 4440
agagcaccgt gcagaacttc cacagctgat cggcctcgcc tcgcagattt gccaagtatc 4500
cgcttcctgt ggaagcaaga ccaaaaggaa atcaactgag tgggtgtttg gaagaggaag 4560
gagcaactct cgggcagcct gcccaaggga gggagcaagt tgcaatttag aagatgccat 4620
acgtcgtgtg acagctcatg agcctttcac tgggctggca attgtctgaa cacttgggtt 4680
cagttgaaat atatgtattt tggccaaaag ccagcagcac ttcacaaaaa caaaacacaa 4740
acctaagcta acaaaatgac tgcattcgtc tcttttttaa aggtagagat taaactgtat 4800
agacagcata gggatgaaag gaaccaagcg tttctgtggg attgagactg gtacgtgtac 4860
gatgaacctg ctgctttgtt ttctgagaag aggtttgaag acattttatt aacagcttaa 4920
tttttctctt ttactccata ggaacttatt ttaatagtaa cattaacaac aagaatacta 4980
agactgtttg ggaattttaa aaagctacta gtgagaaacc aaatgatagg ttgtagagcc 5040
tgatgactcc aaacaaagcc atcacccgca ttcttcctcc ttcttctggt gctacagctc 5100
caagggccct tcaccttcat gtctgaaatg gaactttggc tttttcagtg gaagaatatg 5160
ttgaaggttt cattttgttc tagaaaaaaa aaatccctcc caaagtgggg caaaaagctt 5220
tatatttatt tgattatcca aaatacagat caaagtttag atct 5264

<210> 146
<211> 2858
<212> DNA
<213> Homo sapiens

<400> 146
atcttcggcg ggcgagtggg ctcggcctgt gcaacccgca cctgcgtccc tcgcccggcc 60
cgatggcgcc gcggccgctg ggccccttgg tgctggcgct cggcggcgcc gcggcggtgc 120
tgggctcggt gctcttcatc ctctggaaga cctacttcgg ccgcggccga gagcggcgct 180
gggaccgggg agaggcctgg tggggcgcgg aggctgcccg cctccccgag tgggacgagt 240
gggaccccga ggacgaggag gacgaggagc cggcgctgga ggagctggaa cagcgcgagg 300
tgctggtgct ggggctggat ggcgcaggca agagcacgtt cctgcgcgtg ttgtcgggga 360
agccaccgct ggaaggccac atccccacct ggggcttcaa ctccgtgcgt ctgcccacca 420
aggactttga ggtggacctg ctagaaattg ggggcagcca gaacctgcgc ttctactgga 480
aggagtttgt gagcgaggtg gatgtgctgg tgtttgtggt ggactcggct gaccgactgc 540
ggctgccctg ggcccgacag gagctgcaca agctgctgga caaggaccct gacctgcctg 600
tcgtcgtggt ggccaacaag caggacctga gcgaggccat gagtatgggg gagctgcagc 660
gggagctggg tctacaggct atcgataacc agcgggaggt tttcctcttg gcagccagca 720
ttgcccctgc aggacccacc tttgaagagc ctggcaccgt gcacatctgg aaactgctct 780
tggagctcct ctcctaggct ggagctctcc tgcttgccac ctgcctgtca agaccatagt 840
tgtactgctg ctgcttcatt gccagactgg gcctggggca agagccacat ggcagcattt 900
cccttttccc ctcctttgcc tttcaagagc agggcctggg caaggccaag aaccatgcag 960
aagccttcct ggtgaggtgg ccgtgaagcc gaagcaggga ggtgggtgag acagagggtg 1020
gggaggatag tgtctggctc attccaggct ggaatgtgga tccagctttc ccttctctta 1080
cctgtacagt gagatgctca gtgggctcaa tcctccacta caggtcccgg tacctgagga 1140
accagtgtag gtgtcagaaa tactcctaga gcctcaaggt ctcccagtcc agaaacagtc 1200
tggtgacgtc atgcccttct catgtgggca gcttctgagt ggtgacacag caagcctttg 1260
ttcctgtcct gcattgtcca gccccagctc cacctaagtg acttgtggcc ttgtgcaatc 1320
tctgcctctc tgaccccagg gccattattt ttaaagggag gtggtttcct aattcggaga 1380
tgcctttccc agccatggga gtgtgaagtg ctaggatgaa cctggccatc ctagcaagga 1440
gctttctgaa gacctccctg cctttccctg agcccaggcc tggcccgcca gcctctcttg 1500
actacagaat aactgatatt cacccaccaa acagaaaaag tgaaggctgg gtttttcccc 1560
tctaatctgg aggcaagctg ctgctctcgt actaactgtg ccagtgccca tgtttacaga 1620
agtcagggga aggaaggagc ctgtgtccct gggacgacag tcaactggag ctaggtgttg 1680
acctcagaac tgcattttat ttattaattt ataagcagaa caggccagag ttctaggctc 1740
tgtttctagg tgctgttttc aaaaccccag atgacagtca tagaaaattt ggaacttagg 1800
aaaatagctg gaatcatgaa tgacaatgag ataacataca gatgtcagtg gagacaaagt 1860
tgtgggttcc tcctcccacc tggctttgag gctgtcgtcg atatcatagt actttacatg 1920
gattcacatg aactgaaacg ccaccacttg gcccaggatg ttgaaagggt gcaaattcct 1980
tctgggtaga taagaaatga ctctgggaga ggatttccct tatgtgaatc taggtaaaaa 2040
gatgaaaaaa aattgtatta tgtgatccta aggacaggaa tagcagacca gccaacggga 2100
tggccttggg tacatcactc agcctttctg gacccaattt ttccccagtg aaagccaagt 2160
tggactgaat ttctggagtt ctcatcagtg cacattccat agttctccag tgcttggcga 2220
tcagcccaat tgaaggactg gctctgtact gacacttatt atcggtacag gcaaagagga 2280
gcctgttgtc tgttagggac cactaaatca acaaccacaa atggattttt tttttaagag 2340
gagctgtgca cctcaatttg ctgtctagtt gagaatagag attgtgtgcc ttcatttcat 2400

tttcattgct ttgtagtatt ttattgtatg catgtaccat aatttattta tccagttgga 2460
atttatcctg gcaaagattt gcagtaagtc tccagctgaa tttcttttca ggcgactagc 2520
cagttgcacc aacaatattt agtgagtaat ccgtcttttc tccactaatg taaatgccac 2580
ttttattatt tattaaatga ccttaaatat tgtggtctac ttccagactt gctcttctgc 2640
tccattcatc tttctatttc tgaatttgca atgaacagtt tattattgtc actttataat 2700
acactttaag atctggcaga gtttgtctcc cgcatttctc tttctttttc agaacttttc 2760
ttgatttccc tgcatgatct ttggaatcag cttgtcaaga tccaaaataa tcctgttggt 2820
atgtttgttt gtatcccatt aaaattatag atgaaaag 2858
<210> 147
<211> 639
<212> DNA
<213> Homo sapiens

<400> 147
cgcgcgcccg aacgaagccg cggcccgggc acagccatgg cccggcgggc ggggggcgct 60
cggatgttcg gcagcctcct gctcttcgcc ctgctcgctg ccggcgtcgc cccgctcagc 120
tgggatctcc cggagccccg cagccgagcc agcaagatcc gagtgcactc gcgaggcaac 180
ctctgggcca ccggtcactt catgggcaag aagagtctgg agccttccag cccatcccat 240
tggggacagc tccccacacc tcccctgagg gaccagcgac tgcagctgag tcatgatctg 300
ctcggaatcc tcctgctaaa gaaggctctg ggcgtgagcc tcagccgccc cgcaccccaa 360
atccagtaca ggaggctgct ggtacaaata ctgcagaaat gacaccaata ataggggcag 420
acacaacagc gtggcttaga ttgtgcccac ccaggaaggg tgctgaatgg gaccctgttg 480
atggccccat ctggatgtaa atcctgagct caaatctctg ttactccatt actgtgattt 540
ctggctgggt caccagaaat atcgctgatg cagacacaga ttatgttcct gctgtatttc 600
ctgcttccct gttgaattgg tgaataaaac cttgctctt 639

<210> 148
<211> 3033
<212> DNA
<213> Homo sapiens

<400> 148
agagccagag caggatggag aggagacgca tcacctccgc tgctcgccgc tcctacgtct 60
cctcagggga gatgatggtg gggggcctgg ctcctggccg ccgtctgggt cctggcaccc 120
gcctctccct ggctcgaatg ccccctccac tcccgacccg ggtggatttc tccctggctg 180
gggcactcaa tgctggcttc aaggagaccc gggccagtga gcgggcagag atgatggagc 240
tcaatgaccg ctttgccagc tacatcgaga aggttcgctt cctggaacag caaaacaagg 300
cgctggctgc tgagctgaac cagctgcggg ccaaggagcc caccaagctg gcagacgtct 360
accaggctga gctgcgagag ctgcggctgc ggctcgatca actcaccgcc aacagcgccc 420
ggctggaggt tgagagggac aatctggcac aggacctggc cactgtgagg cagaagctcc 480
aggatgaaac caacctgagg ctggaagccg agaacaacct ggctgcctat agacaggaag 540
cagatgaagc caccctggcc cgtctggatc tggagaggaa gattgagtcg ctggaggagg 600
agatccggtt cttgaggaag atccacgagg aggaggttcg ggaactccag gagcagctgg 660
cccgacagca ggtccatgtg gagcttgacg tggccaagcc agacctcacc gcagccctga 720
aagagatccg cacgcagtat gaggcaatgg cgtccagcaa catgcatgaa gccgaagagt 780
ggtaccgctc caagtttgca gacctgacag acgctgctgc ccgcaacgcg gagctgctcc 840
gccaggccaa gcacgaagcc aacgactacc ggcgccagtt gcagtccttg acctgcgacc 900
tggagtctct gcgcggcacg aacgagtccc tggagaggca gatgcgcgag caggaggagc 960
ggcacgtgcg ggaggcggcc agttatcagg aggcgctggc gcggctggag gaagaggggc 1020
agagcctcaa ggacgagatg gcccgccact tgcaggagta ccaggacctg ctcaatgtca 1080
agctggccct ggacatcgag atcgccacct acaggaagct gctagagggc gaggagaacc 1140
ggatcaccat tcccgtgcag accttctcca acctgcagat tcgagaaacc agcctggaca 1200
ccaagtctgt gtcagaaggc cacctcaaga ggaacatcgt ggtgaagacc gtggagatgc 1260
gggatggaga ggtcattaag gagtccaagc aggagcacaa ggatgtgatg tgaggcagga 1320
cccacctggt ggcctctgcc ccgtctcatg aggggcccga gcagaagcag gatagttgct 1380
ccgcctctgc tggcacattt ccccagacct gagctcccca ccaccccagc tgctcccctc 1440
cctcctctgt ccctaggtca gcttgctgcc ctaggctccg tcagtatcag gcctgccaga 1500
cggcacccac ccagcaccca gcaactccaa ctaacaagaa actcaccccc aagggcagtc 1560
tggaggggca tggccagcag cttgcgttag aatgaggagg aaggagagaa ggggaggagg 1620
gcggggggca cctactacat cgccctccac atccctgatt cctgttgtta tggaaactgt 1680
tgccagagat ggaggttctc tcggagtatc tgggaactgt gcctttgagt ttcctcaggc 1740
tgctggagga aaactgagac tcagacagga aagggaaggc cccacagaca aggtagccct 1800
ggccagaggc ttgttttgtc ttttggtttt tatgaggtgg gatatcccta tgctgcctag 1860

gctgaccttg aactcctggg ctcaagcagt ctacccacct cagcctcctg tgtagctggg 1920
attatagatt ggagccacca tgcccagctc agagggttgt tctcctagac tgaccctgat 1980
cagtctaaga tgggtgggga cgtcctgcca cctggggcag tcacctgccc agatcccaga 2040
aggacctcct gagcgatgac tcaagtgtct cagtccacct gagctgccat ccagggatgc 2100
catctgtggg cacgctgtgg gcaggtggga gcttgattct cagcacttgg gggatctgtt 2160
gtgtacgtgg agagggatga ggtgctggga gggatagagg ggggctgcct ggcccccagc 2220
tgtgggtaca gagaggtcaa gcccaggagg actgccccgt gcagactgga ggggacgctg 2280
gtagagatgg aggaggaggc aattgggatg gcactaggca tacaagtagg ggttgtgggt 2340
gaccagttgc acttggcctc tggattgtgg gaattaagga agtgactcat cctcttgaag 2400
atgctgaaac aggagagaaa ggggatgtat ccatgggggc agggcatgac tttgtcccat 2460
ttctaaaggc ctcttccttg ctgtgtcata ccaggccgcc ccagcctctg agcccctggg 2520
actgctgctt cttaacccca gtaagccact gccacacgtc tgaccctctc caccccatag 2580
tgaccggctg cttttcccta agccaagggc tcttgcggtc ccttcttact cacacacaaa 2640
atgtacccag tattctaggt agtgccctat tttacaattg taaaactgag gcacgagcaa 2700
agtgaagaca ctggctcata ttcctgcagc ctggaggccg ggtgctcagg gctgacacgt 2760
ccaccccagt gcacccactc tgctttgact gagcagactg gtgagcagac tggtgggatc 2820
tgtgcccaga gatgggactg ggagggccca cttcagggtt ctcctctccc ctctaaggcc 2880
gaagaagggt ccttccctct ccccaagact tggtgtcctt tccctccact tcttcctgcc 2940
acctgctgct gctgctgctg ctaatcttca gggcactgct gctgccttta gtcgctgagg 3000
aaaaataaag acaaatgctg cgcccttaaa aaa 3033

<210> 149
<211> 2453
<212> DNA
<213> Homo sapiens

<400> 149
gaattccgcc tcacagtggc caggtcctgt gccagattgt ccctctcaac ctccagccgg 60
gcgctgttgg cgtgagttga tcgagccgca gccgcagctc tcgcagctca gcctggtaga 120
cgtctgccag cttggtgggc tccttggccc gcagctggtt cagcctcagc agccagcgcc 180
ttgttttgct gttccaggaa gcgaaccttc tcgatgtagc tggcaaagcg gtattgagct 240
ccatcatctc tgcccgctca ctggcccggg tctccttgaa gccagcattg agtgccccag 300
ccagggagaa atccacccgg gtcggagtgg agggggcatt cgagccaggg agaggcgggt 360
gcaggaccca gacggcggcc aggagccagg ccccccacca tcatctcccc tgaggagacg 420
taggagcggc gagcagcgga ggtgatgcgt ctcctctcca gcctgtccgg ctcgtcggtg 480
tcctccgatg ctgaagaata ccagcctcct atatggaaat catacttata tcagttacag 540
caagaggcac ctcgtcccaa gagaatcatt tgtcctcggg aggtggaaaa cagaccaaaa 600
tattatggaa gagagtttca tgggatcatc tctcgggagc aggcggatga gcttcttgga 660
ggcgtggagg gtgcctacat ccttagagaa agccagcggc aaccaggatg ctacacgctg 720
gctctcaggt ttggaaacca gaccttaaac tacaggctct tccacgacgg gaaacacttt 780
gtgggtgaga agaggtttga gtcgattcat gatctggtga cagatggctt gataacactg 840
tacatagaaa caaaagctgc cgagtacatt tcaaaaatga caactaaccc catctatgaa 900
cacattggat atgccaccct actcagagaa aaagtatcca gaaggctgag caggtctaaa 960
aatgaaccaa gaaaaacaaa cgtcacacat gaagaacaca cagcggtgga aaagatctcc 1020
tccctggttc gaagggctgc cctcacacac aacgacaacc acttcaatta tgagaagaca 1080
cacaacttta aggtccacac gttccgaggc ccacactggt gtgaatattg tgccaatttc 1140
atgtgggggc tcatcgccca aggggtccgg tgctcagact gtggattgaa cgtacacaaa 1200
cagtgttcca agcacgttcc caatgactgc caacctgatc tcaagaggat caagaaagtg 1260
tactgttgtg acctcacaac acttgtgaag gctcacaaca ctcagagacc catggtggta 1320
gacatatgca ttcgggaaat tgaagcaaga ggattaaaat cggaaggcct ttacagagtc 1380
tctgggttca ctgaacacat tgaagatgtc aaaatggcat ttgacagaga tggtgaaaag 1440
gccgatatat ctgccaatgt ctatccagac ataaacatca tcactggagc ccttaaactg 1500
tatttcagag acttacccat ccctgtcatc acatatgata cctattccaa atttatagat 1560
gcagcaaaaa tctccaatgc agatgagagg ctggaagccg tccatgaagt gctgatgctg 1620
ctgcctcctg cccactatga aaccctccgg tacctaatga tccacctcaa aaaggttact 1680
atgaatgaaa aagacaattt catgaatgca gaaaatctgg ggatcgtgtt tgggcccact 1740
ctgatgaggc cccctgagga cagcaccctg accaccctgc atgatatgcg gtaccaaaag 1800
ctgattgtgc agattttaat agaaaacgaa gacgttttat tctaatccat cagggaaatg 1860
agctgaatgg cccagcacca tcaagttgac acagctaagg ataaaacatt tcttaccact 1920
tgatttgttt tccaagcaag tgctagaatt tgctggactg cagaggatcg ctgagtgggg 1980
tactgtgtct catagacatg cgccacctcc acgtgagaac aagggtgaag gtgagggaag 2040
cccctcaggt tgggtctttt gctgtgcctc ctatgtatgt ctggtttgct ggaagagtga 2100
ttaatacatc tttaatttat taaaaaacaa tgtagacctt taaacttcag tcttattggg 2160

aataaaaggg aacttaattc atacaggtac ttgatacagt tatacatttt ccacttacaa 2220
aaagaagaca attctgttaa atgaaacgtg tatcgtaaaa tgtaatttta tttacccacg 2280
agaatgttgt tattttagca atagaactca atgcagatgc attggttatt accctgtgta 2340
ccttgtccct cattttgctg tgacaccctg aaaaagctga ccacaaatgc agtattatca 2400
ttgacatacc tctgtcctcc tcagtgcttt ttaatgtaat ttcaccggaa ttc 2453

<210> 150
<211> 2103
<212> DNA
<213> Homo sapiens

<400> 150
cccaggaacc cccagggagc cagcatgaag cgagctcacc ccgagtacag ctcctcggac 60
agcgagctgg acgagaccat cgaggtggag aaggagagtg cggacgagaa tggaaacttg 120
agttcggctc taggttccat gtccccaact acatcttccc agattttggc cagaaaaaga 180
cggagaggaa taattgagaa gcgccgacga gaccggatca ataacagttt gtctgagctg 240
agaaggctgg tacccagtgc ttttgagaag cagggatctg ctaagctaga aaaagccgag 300
atcctgcaga tgaccgtgga tcacctgaaa atgctgcata cggcaggagg gaaaggttac 360
tttgacgcgc acgcccttgc tatggactat cggagtttgg gatttcggga atgcctggca 420
gaagttgcgc gttatctgag catcattgaa ggactagatg cctctgaccc gcttcgagtt 480
cgactggttt cgcatctcaa caactacgct tcccagcggg aagccgcgag cggcgcccac 540
gcgggcctcg gacacattcc ctgggggacc gtcttcggac atcacccgca catcgcgcac 600
ccgctgttgc tgccccagaa cggccacggg aacgcgggca ccacggcctc acccacggaa 660
ccgcaccacc agggcaggct gggctcggca catccggagg cgcctgcttt gcgagcgccc 720
cctagcggca gcctcggacc ggtgctccct gtggtcacct ccgcctccaa actgtcgccg 780
cctctgctct cctcagtggc ctccctgtcg gccttcccct tctctttcgg ctccttccac 840
ttactgtctc ccaatgcact gagcccttca gcacccacgc aggctgcaaa ccttggcaag 900
ccctatagac cttgggggac ggagatcgga gctttttaaa gaactgatgt agaatgaggg 960
aggggaaagt ttaaaatccc agctgggctg gactgttgcc aacatcacct taaagtcgtc 1020
agtaaaagta aaaaggaaaa aggtacactt tcagataatt ttttttttaa agactaaagg 1080
tttgttggtt tacttttatc ttttttaatg tttttttcat catgtcatgt attagcagtt 1140
tttaaaaact agttgttaaa ttttgttcaa gacattaaat tgaaatagtg agtataagcc 1200
aacactttgt gataggtttg tactgtgcct aatttacttt gtaaaccaga atgattccgt 1260
ttttgcctca aaatttgggg aatcttaaca tttagtattt ttggtctgtt tttctccttg 1320
tatagttatg gtctgttttt agaattaatt ttccaaacca ctatgcttaa tgttaacatg 1380
attctgtttg ttaatatttt gacagattaa ggtgttgtat aaataatatt cttttggggg 1440
gaggggaact atattgaatt ttatatttct gagcaaagcg ttgacaaatc agatgatcag 1500
ctttatccaa gaaagaagac tagtaaattg tctgcctcct atagcagaaa ggtgaatgta 1560
caaactgttg gtggccctga atccatctga ccagctgctg gtatctgcca ggactggcag 1620
ttctgattta gttaggagag agccgctgat aggttaggtc tcatttggag tgttggtgga 1680
aaggaaactg aaggtaattg aatagaatac gcctgcattt accagcccca gcaacacaaa 1740
gaatttttaa tcacacggat ctcaaattca caaatgttaa catggataag tgatcatggt 1800
gtgcgagtgg tcaattgagt agtacagtgg aaactgttaa atgcataacc taattttcct 1860
gggactgcca tattttcttt taactggaaa tttttatgtg agttttcctt ttggtgcatg 1920
gaactgtggt tgccaaggta tttaaaaggg ctttcctgcc tccttctctt tgatttattt 1980
aatttgattt gggctataaa atatcatttt tcaggtttat tcttttagca ggtgtagtta 2040
aacgacctcc actgaactgg gtttgacctc tgttgtactg atgtgttgtg actaaataaa 2100
aaa 2103

<210> 151
<211> 3560
<212> DNA
<213> Homo sapiens

<400> 151
agcaaactcg tcttgtctac ccaccctccc tcccccatcc tccccaaaat agccttgtga 60
tttcggaagt atggactaaa atcacactcc tccttacctt accgcttgga ctctggtggc 120
tcccaactcg ccgtcagacc ccacctgccc cggtggtggg aagcgcctgg acagaccatg 180
accacagcca aggagccaag cgcttcgggg aaatccgtgc agcagcagga acaggagctg 240
gtggggagta accctccgca gaggaattgg aaaggaatag caattgcact gcttgtcatt 300
ctggtcatct gctccttgat cgtcacctcg gtcatacttc tgacaccagc ggaagataat 360
agtctgtctc aaaagaagaa ggtcactgta gaagatctct tcagtgaaga cttcaaaatt 420
catgaccccg aggctaagtg gataagtgat acagaattca tctacagaga acagaaagga 480

acagtgagac tgtggaatgt tgaaacaaat acttctactg tcttaataga aggcaaaaaa 540
attgaatcat taagagccat cagatatgaa atatctccag atagagagta tgcacttttt 600
tcatacaatg tggaacccat atatcaacac tcgtatactg gatattacgt cctgagcaaa 660
attcctcatg gggatcctca aagtctggac ccaccagaag tcagcaatgc aaagcttcag 720
tatgcaggat ggggccctaa aggccaacag ctgatattta tttttgaaaa caatatctac 780
tactgtgcac atgtcgggaa acaggccatc cgtgtggtct ccactggcaa ggaaggtgtg 840
atttacaatg gcctcagtga ctggctgtat gaagaggaga ttttgaagac acacatcgca 900
cactggtggt ctccggatgg cacgagactc gcctacgccg ccatcaatga ttcccgtgtc 960
cccatcatgg agctcccaac ttacaccggc tccatctacc ccaccgtgaa gccctaccac 1020
tatcccaagg ctggaagtga gaaccccagc atttccctac acgttattgg cttaaatgga 1080
cccacccatg atctggagat gatgccgcct gatgatccac ggatgaggga gtactacatc 1140
accatggtga agtgggccac cagcaccaag gtcgccgtga cctggctgaa ccgggcgcag 1200
aacgtgtcca tcctcaccct ctgcgacgcc accacggggg tctgcacgaa gaaacacgag 1260
gatgaaagtg aggcctggct ccacagacag aatgaagaac ctgtgttctc caaggatggc 1320
cgaaagtttt tcttcatcag agccatcccc cagggaggac gagggaaatt ctatcacatc 1380
acggtgtcct cgtcccagcc caacagcagc aacgacaaca tccagtccat cacctccggg 1440
gactgggacg tgaccaagat cctagcctac gatgagaagg ggaataagat ctacttcctg 1500
agcacggagg acctgcctcg gagacgacaa ctctacagtg ccaacacggt gggcaacttc 1560
aacaggcagt gcctctcctg tgacctggtt gagaactgca cctacttcag cgcttccttc 1620
agccatagca tggacttctt cctgctcaag tgcgaaggtc ctggtgttcc tatggtgacg 1680
gtgcacaaca caacagataa gaaaaaaatg tttgacctag aaacaaatga acatgtcaag 1740
aaggccataa atgaccgaca gatgcctaaa gtggaataca gggacattga gattgatgat 1800
tacaacctgc ccatgcagat actgaagcca gcaaccttca ccgacaccac ccactaccct 1860
ctgctcctgg tggtggatgg caccccgggc agccagagtg tagctgagaa gttcgaggtg 1920
agctgggaga cggtgatggt gagcagccac ggcgcggtgg tggtaaagtg tgacggccgt 1980
ggcagcggct tccaagggac caagctcctg cacgaagtga ggcggcggct gggcttgctg 2040
gaggagaagg accagatgga ggccgtgcgg acgatgctga aggagcagta cattgacagg 2100
acgcgcgtgg ccgtgtttgg gaaggattac ggtggctacc tgagcaccta catcctccca 2160
gcaaagggag aaaatcaagg ccagacattc acctgcggct ctgctctctc tccaataaca 2220
gacttcaaac tctatgcctc tgcgttttcc gagaggtact tgggcctcca tggacttgac 2280
aacagagcat acgagatgac caaggtagcc catcgagtct ccgcgctgga agaacagcag 2340
ttcctgatca ttcatcccac tgccgatgaa aaaattcatt tccagcacac agcagaactc 2400
attacacaac taattagggg aaaggctaat tacagcttac agatttaccc ggacgaaagc 2460
cattacttta ccagctccag cctcaaacag catctgtacc ggtccatcat caacttcttc 2520
gtggaatgct tcaggatcca ggacaaactg ccgacagtca cagcgaaaga ggacgaggag 2580
gaggactaag ctcaggtcgc tctaagcaca aacgtggctc tttctacaac cagatgcaac 2640
cgagggattt ccctgccctc cctcttccct cggaggggcg gggcggggcg gggccgggtg 2700
ttccatagca tgtgtgtctc ggatgcggaa ggcagttttg cttgggaaac aagctccttc 2760
cccggggtca tcactcacgg cctccatggc accagggaca acgctgtccc cgcagcagcg 2820
cctcctcccg gcgcccgaga gaccggcacg ccacggcccc tcccccaagg aacagagcaa 2880
aggatggtgg ccgcaggccc cacgcgagcc cacaggacac cggcccctag attccagcca 2940
ccaagcggaa gcatgagacc cgcccacact agcctctgtg ttcccgttag ggacatcaca 3000
ccctgtctca cgtcgcagtg ccatggacgc agcagttaca gcaccattgt tttagcagtg 3060
cgtgttcata tatgggcttg ctacttcctg taatgaggac gttcaacatg gtgaggggct 3120
acaagaaaac gcttttctgt acagagtctt actgtagcta cgctaatggt taacctgata 3180
gaattaactc gtatttttct atggttttaa cctgatgctc cactgtctcc gtcatggggt 3240
tgttttgctg tttggggttg ggccttgttt ccctttcctt tctccagtcc acgtgtagac 3300
tttgcgcttg atgaagaagc agatcggaag taactgctcc ctcctcaagg ttgtcttcag 3360
acgtcttggg gacgttccta aacactgagg gggaagacag ccaatagcac ccattaaaag 3420
aaatacctaa ataaaacctc tctcccactc agctatgcta gggcttggct gtaggtgtgc 3480
actgtctatt tacatccgtc cttacaacca tccttgtcct ccttggtacc gtatcaagct 3540
ctttcccatg acatttggtt 3560

<210> 152
<211> 1796
<212> DNA
<213> Homo sapiens

<400> 152
ccggcgccgg gggcggcggg gaaccccaaa cgcaaccggg tctggaggga tccccgcgcc 60
gagccagccg ccgtcaccgc ctccgcgccg cccctgcggg cttggcaggc gcccggcgcg 120
cccgcactgc gcccggccgc cggctcccgc ggtcccaccg tgagctcgcc ggcccgtcgc 180
ccgctcgcca tgcaaccgcc gccggcctcg cgcgcgtagg cgcccgccgc aggccatgct 240

gcccctgctc gccgcgctcc tggccgccgc ctgcccgctg ccgcccgtcc gcggcggggc     300
cgcggacgcg cccggcctcc tcggggtgcc ctccaatgct tcagtcaacg cgtcctccgc     360
ggcgagccca tcgccccgcg gctgctggcc tcggcggccc ccgggccccc cgagcgcccg     420
ggcccggagg aggcggcggc ggcggcggcg cctgtgcaac atcagcgtgc agcggcagat     480
gctgagctcg ctgctcgtgc gctggggccg cccgcggggc ttccagtgcg acctactgct     540
cttctccacc aacgcgcacg gccgcgcttt cttcgccgcc gccttccacc gcgtcgggcc     600
gccgctgctc atcgagcacc tggggctggc ggcgggcggc gcgcagcagg acctgcgcct     660
ctgcgtgggc tgcggctggg tgcgcggtcg ccgcaccggc cgcctccggc ccgccgccgc     720
ccccagcgcc gccgccgcca ccgccggggc gcccaccgcg ctgccagcct accccgcggc     780
cgagccgccc gggccgctgt ggctgcaggg cgagccgctg catttctgct gcctagactt     840
cagcctggag gagctgcagg gcgagccggg ctggcggctg aaccgtaagc ccattgagtc     900
cacgctggtg gcctgcttca tgaccctggt catcgtggtg tggagcgtgg ccgccctcat     960
ctggccggtg cccatcatcg ccggcttcct gcccaacggc atggaacagc gccggaccac    1020
cgccagcacc accgcagcca cccccgccgc agtgcccgca gggaccaccg cagccgccgc    1080
cgccgccgcc gctgccgccg ccgccgcggc cgtcacttcg ggggtggcga ccaagtgacc    1140
cgctccgctc ctccctgtgt ccgtcctgtg tccgcgcgcg cgggtgcctt tcccgccgga    1200
gactcggccg gtgtgcttcg tgctgtagtt atcgttagtt cctcttcccg agatggggcc    1260
gccgagagac cccagcgcct ttgaaaagca aggtttgtgc tgcgcttcca gttccgaaaa    1320
gcagatgttt aagcccttgg actgagggtg ggatcgcagc tccgaagacg gagaggaggg    1380
aaatggggcc ctttcccctc tattgcatcc ccctgcccga ctccttcccc gcacccacgt    1440
gccctagatt catggcagaa aatgaccaaa tcctgtgtat ttgttttata tatttaataa    1500
ctgttttaaa tgaaagtttt agtaaaaaaa atacaaaaca aaaagattaa attgctattg    1560
ctgtagtaag agaagctctt tgtatctgaa catagttgta tttgaaattt gtggtttttt    1620
aatttattta aaattggggg gagggcatgg gaaggattta acaccgatat attgttaccg    1680
ctgaaaatga actttatgaa ccttttccaa gttgatctat ccagtgacgt ggcctggtgg    1740
gcgtttcttc ttgtacttat gtggtttttt ggcttttaat acagacattt tcctcc        1796

<210> 153
<211> 843
<212> DNA
<213> Homo sapiens

<400> 153
atggatctcc aaggaagagg ggtccccagc atcgacagac ttcgagttct cctgatgttg      60
ttccatacaa tggctcaaat catggcagaa caagaagtgg aaaatctctc aggcctttcc     120
actaaccctg aaaaagatat atttgtggtg cgggaaaatg ggacgacgtg tctcatggca     180
gagtttgcag ccaaatttat tgtaccttat gatgtgtggg ccagcaacta cgtagatctg     240
atcacagaac aggccgatat cgcattgacc cggggagctg aggtgaaggg ccgctgtggc     300
cacagccagt cggagctgca agtgttctgg gtggatcgcg catatgcact caaaatgctc     360
tttgtaaagg aaagccacaa catgtccaag ggacctgagg cgacttggag gctgagcaaa     420
gtgcagtttg tctacgactc ctcggagaaa acccacttca aagacgcagt cagtgctggg     480
aagcacacag ccaactcgca ccacctctct gccttggtca cccccgctgg gaagtcctat     540
gagtgtcaag ctcaacaaac catttcactg gcctctagtg atccgcagaa gacggtcacc     600
atgatcctgt ctgcggtcca catccaacct tttgacatta tctcagattt tgtcttcagt     660
gaagagcata aatgcccagt ggatgagcgg gagcaactgg aagaaacctt gcccctgatt     720
ttggggctca tcttgggcct cgtcatcatg gtaacactcg cgatttacca cgtccaccac     780
aaaatgactg ccaaccaggt gcagatccct cgggacagat cccagtataa gcacatgggc     840
tag                                                                   843

<210> 154
<211> 946
<212> DNA
<213> Homo sapiens

<400> 154
ggcacgaggc cggaggcgcg gtgctcggcc cgggagcgcg agcgggagga gcagagaccc      60
gcagccggga gcccgagcgc gggcgatgca ggctccgcga gcggcacctg cggctcctct     120
aagctacgac cgtcgtctcc gcggcagcag cgcgggcccc agcagcctcg gcagccacag     180
ccgctgcagc cggggcagcc tccgctgctg tcgcctcctc tgatgcgctt gccctctccc     240
ggccccggga ctccgggaga atgtgggtcc taggcatcgc ggcaactttt tgcggattgt     300
tcttgcttcc aggctttgcg ctgcaaatcc agtgctacca gtgtgaagaa ttccagctga     360
acaacgactg ctcctccccc gagttcattg tgaattgcac ggtgaacgtt caagacatgt     420
gtcagaaaga agtgatggag caaagtgccg ggatcatgta ccgcaagtcc tgtgcatcat     480

cagcggcctg tctcatcgcc tctgccgggt accagtcctt ctgctcccca gggaaactga 540
actcagtttg catcagctgc tgcaacaccc ctctttgtaa cgggccaagg cccaagaaaa 600
ggggaagttc tgcctcggcc ctcaggccag ggctccgcac caccatcctg ttcctcaaat 660
tagccctctt ctcggcacac tgctgaagct gaaggagatg ccaccccctc ctgcattgtt 720
cttccagccc tcgcccccaa ccccccacct ccctgagtga gtttcttctg ggtgtccttt 780
tattctgggt agggagcggg agtccgtgtt ctcttttgtt cctgtgcaaa taatgaaaga 840
gctcggtaaa gcattctgaa taaattcagc ctgactgaat tttcagtatg tacttgaagg 900
aaggaggtgg agtgaaagtt cacccccaaa aaaaaaaaaa aaaaaa 946

<210> 155
<211> 2211
<212> DNA
<213> Homo sapiens

<400> 155
ttgggggttt attctcttcc cttctaactt gacagggtct tgctctgtca ttcaggcaag 60
agtgcagtag tgtgatcact tcttactgcc gcctcaagct tccagcctca actcaagcaa 120
tcctcccacc tcagccaccc aagtggctgg gactacagat taagaatgac ccaaaataaa 180
ttaaagcttt gttccaaagc caatgtgtat actgaagtgc ctgatggagg atggggctgg 240
gcggtagctg tttcattttt cttcgttgaa gtcttcacct acggcatcat caagacattt 300
ggtgtcttct ttaatgactt aatggacagt tttaatgaat ccaatagcag gatctcatgg 360
ataatctcaa tctgtgtgtt tgtcttaaca ttttcagctc ccctcgccac agtcctgagc 420
aatcgtttcg gacaccgtct ggtagtgatg ttgggggggc tacttgtcag caccgggatg 480
gtggccgcct ccttctcaca agaggtttct catatgtacg tcgccatcgg catcatctct 540
ggtctgggat actgctttag ttttctccca actgtaacca tcctatcaca atattttggc 600
aaaagacgtt ccatagtcac tgcagttgct tccacaggag aatgtttcgc tgtgtttgct 660
ttcgcaccag caatcatggc tctgaaggag cgcattggct ggagatacag cctcctcttc 720
gtgggcctac tacagttaaa cattgtcatc ttcggagcac tgctcagacc catcattatc 780
agaggaccag cgtcaccgaa aatagtcatc caggaaaatc ggaaagaagc gcagtatatg 840
cttgaaaatg agaaaacacg aacctcaata gactccattg actcaggagt agaactaact 900
acctcaccta aaaatgtgcc tactcacact aacctggaac tggagccgaa ggccgacatg 960
cagcaggtcc tggtgaagac cagccccagg ccaagcgaaa agaaagcccc gctattagac 1020
ttctccattt tgaaagagaa aagttttatt tgttatgcat tatttggtct ctttgcaaca 1080
ctgggattct ttgcaccttc cttgtacatc attcctctgg gcattagtct gggcattgac 1140
caggaccgcg ctgctttttt attatctacg atggccattg cagaagtttt cggaaggatc 1200
ggagctggtt ttgtcctcaa cagggagccc attcgtaaga tttacattga gctcatctgc 1260
gtcatcttat tgactgtgtc tctgtttgcc tttacttttg ctactgaatt ctggggtcta 1320
atgtcatgca gcatattttt tgggtttatg gttggaacaa taggaggact cacattccac 1380
tgcttgctga agatgatgtc gtgggcattg cagaagatgt cttctgcagc tggggtctac 1440
atcttcattc agagcatagc aggactggct ggaccgcccc ttgcaggttt gttggtggac 1500
caaagtaaga tctacagcag ggccttctac tcctgcgcag ctggcatggc cctggctgct 1560
gtgtgcctcg ccctggtgag accgtgtaag atgggactgt gccagcgtca tcactcaggt 1620
gaaacaaagg tagtgagcca tcgtgggaag actttacagg acatacctga agactttctg 1680
gaaatggatc ttgcaaaaaa tgagcacaga gttcacgtgc aaatggagcc ggtatgacac 1740
actttcttac aacaacagcc actgtgttgg ctggagaggg atggggtggg cccaacgggg 1800
acacaaggag gcagaggagc taacccctct actccacttt caaaactaca ttttaaaggg 1860
aatgtgtatg tgaagagcac taccaacatc gcttttgttt tgttttgttt tgttttaagc 1920
tttttttttt tgcttgtttt taaagccaaa acaaaaaaca accaagcact cttccatata 1980
taaatctggc tgtattcagt agcaatacaa gagatatgta gaaagactct ttggttcaca 2040
ttccgatatt aaaatagtga catgaactgg caaagtggtt ttaaaagctt tcacgtggga 2100
taaatgattt tctttttttc ttttctttct tcctatggtc ttgtctgaat aaactactct 2160
cctgaataaa acaacatcca acccaggtca ttgaaatgaa attggccagt c 2211

<210> 156
<211> 1485
<212> DNA
<213> Homo sapiens

<400> 156
tcagccaatt agagctccag ttgtcactcc tacccacact gggcctgggg gtgaagggaa 60
gtgtttatta ggggtacatg tgaagccgtc cagaagtgtc agagtctttg tagctttgaa 120
agtcacctag gttatttggg catgctctcc tgagtcctct gctagttaag ctctctgaaa 180

agaaggtggc agacccggtt tgctgatcgc cccagggatc aggaggctga tcccaaagtt 240
gtcagatgga gagtaaatac aaggagatac tcttgctaac aggcctggat aacatcactg 300
atgaggaact ggataggttt aagttctttc tttcagacga gtttaatatt gccacaggca 360
aactacatac tgcaaacaga atacaagtag ctaccttgat gattcaaaat gctggggcgg 420
tgtctgcagt gatgaagacc attcgtattt ttcagaagtt gaattatatg cttttggcaa 480
aacgtcttca ggaggagaag gagaaagttg ataagcaata caaatcggta acaaaaccaa 540
agccactaag tcaagctgaa atgagtcctg ctgcatctgc agccatcaga aatgatgtcg 600
caaagcaacg tgctgcacca aaagtctctc ctcatgttaa gcctgaacag aaacagatgg 660
tggcccagca ggaatctatc agagaagggt ttcagaagcg ctgtttgcca gttatggtac 720
tgaaagcaaa gaagcccttc acgtttgaga cccaagaagg caagcaggag atgtttcatg 780
ctacagtggc tacagaaaag gaattcttct ttgtaaaagt ttttaataca ctgctgaaag 840
ataaattcat tccaaagaga ataattataa tagcaagata ttatcggcac agtggtttct 900
tagaggtaaa tagcgcctca cgtgtgttag atgctgaatc tgaccaaaag gttaatgtcc 960
cgctgaacat tatcagaaaa gctggtgaaa ccccgaagat caacacgctt caaactcagc 1020
cccttggaac aattgtgaat ggtttgtttg tagtccagaa ggtaacagaa aagaagaaaa 1080
acatattatt tgacctaagt gacaacactg ggaaaatgga agtactgggg gttagaaacg 1140
aggacacaat gaaatgtaag gaaggagata aggttcgact tacattcttc acactgtcaa 1200
aaaatggaga aaaactacag ctgacatctg gagttcatag caccataaag gttattaagg 1260
ccaaaaaaaa aacatagaga agtaaaaagg accaattcaa gccaactggt ctaagcagca 1320
tttaattgaa gaatatgtga tacagcctct tcaatcagat tgtaagttac ctgaaagctg 1380
cagttcacag gctcctctct ccaccaaatt aggatagaat aattgctgga taaacaaatt 1440
cagaatatca acagatgatc acaataaaca tctgtttctc attcc 1485

<210> 157
<211> 1493
<212> DNA
<213> Homo sapiens

<400> 157
ttcctttcat gttcagcatt tctactcctt ccaagaagag cagcaaagct gaagtagcag 60
caacagcacc agcagcaaca gcaaaaaaca aacatgagtg tgaagggcat ggctatagcc 120
ttggctgtga tattgtgtgc tacagttgtt caaggcttcc ccatgttcaa aagaggacgc 180
tgtctttgca taggccctgg ggtaaaagca gtgaaagtgg cagatattga gaaagcctcc 240
ataatgtacc caagtaacaa ctgtgacaaa atagaagtga ttattaccct gaaagaaaat 300
aaaggacaac gatgcctaaa tcccaaatcg aagcaagcaa ggcttataat caaaaaagtt 360
gaaagaaaga atttttaaaa atatcaaaac atatgaagtc ctggaaaagg gcatctgaaa 420
aacctagaac aagtttaact gtgactactg aaatgacaag aattctacag taggaaactg 480
agacttttct atggttttgt gactttcaac ttttgtacag ttatgtgaag gatgaaaggt 540
gggtgaaagg accaaaaaca gaaatacagt cttcctgaat gaatgacaat cagaattcca 600
ctgcccaaag gagtccagca attaaatgga tttctaggaa aagctacctt aagaaaggct 660
ggttaccatc ggagtttaca aagtgctttc acgttcttac ttgttgtatt atacattcat 720
gcatttctag gctagagaac cttctagatt tgatgcttac aactattctg ttgtgactat 780
gagaacattt ctgtctctag aagttatctg tctgtattga tctttatgct atattactat 840
ctgtggttac agtggagaca ttgacattat tactggagtc aagcccttat aagtcaaaag 900
catctatgtg tcgtaaagca ttcctcaaac attttttcat gcaaatacac acttctttcc 960
ccaaatatca tgtagcacat caatatgtag ggaaacattc ttatgcatca tttggtttgt 1020
tttataacca attcattaaa tgtaattcat aaaatgtact atgaaaaaaa ttatacgcta 1080
tgggatactg gcaacagtgc acatatttca taaccaaatt agcagcaccg gtcttaattt 1140
gatgtttttc aacttttatt cattgagatg ttttgaagca attaggatat gtgtgtttac 1200
tgtacttttt gttttgatcc gtttgtataa atgatagcaa tatcttggac acatttgaaa 1260
tacaaaatgt ttttgtctac caaagaaaaa tgttgaaaaa taagcaaatg tatacctagc 1320
aatcactttt actttttgta attctgtctc ttagaaaaat acataatcta atcaatttct 1380
ttgttcatgc ctatatactg taaaatttag gtatactcaa gactagttta aagaatcaaa 1440
gtcatttttt tctctaataa actaccacaa cctttctttt ttaaaaaaaa aaa 1493

<210> 158
<211> 1853
<212> DNA
<213> Homo sapiens

<400> 158
aaaacgcagg gagggaggct gtcaccatgc cggcctgctg cagctgcagt gatgttttcc 60

agtatgagac gaacaaagtc actcggatcc agagcatgaa ttatggcacc attaagtggt 120
tcttccacgt gatcatcttt tcctacgttt gctttgctct ggtgagtgac aagctgtacc 180
agcggaaaga gcctgtcatc agttctgtgc acaccaaggt gaaggggata gcagaggtga 240
aagaggagat cgtggagaat ggagtgaaga agttggtgca cagtgtcttt gacaccgcag 300
actacacctt ccctttgcag gggaactctt tcttcgtgat gacaaacttt ctcaaaacag 360
aaggccaaga gcagcggttg tgtcccgagt atcccacccg caggacgctc tgttcctctg 420
accgaggttg taaaaaggga tggatggacc cgcagagcaa aggaattcag accggaaggt 480
gtgtagtgca tgaagggaac cagaagacct gtgaagtctc tgcctggtgc cccatcgagg 540
cagtggaaga ggccccccgg cctgctctct tgaacagtgc cgaaaacttc actgtgctca 600
tcaagaacaa tatcgacttc cccggccaca actacaccac gagaaacatc ctgccaggtt 660
taaacatcac ttgtaccttc cacaagactc agaatccaca gtgtcccatt ttccgactag 720
gagacatctt ccgagaaaca ggcgataatt tttcagatgt ggcaattcag ggcggaataa 780
tgggcattga gatctactgg gactgcaacc tagaccgttg gttccatcac tgccatccca 840
aatacagttt ccgtcgcctt gacgacaaga ccaccaacgt gtccttgtac cctggctaca 900
acttcagata cgccaagtac tacaaggaaa acaatgttga gaaacggact ctgataaaag 960
tcttcgggat ccgttttgac atcctggttt ttggcaccgg aggaaaattt gacattatcc 1020
agctggttgt gtacatcggc tcaaccctct cctacttcgg tctggccgct gtgttcatcg 1080
acttcctcat cgacacttac tccagtaact gctgtcgctc ccatatttat ccctggtgca 1140
agtgctgtca gccctgtgtg gtcaacgaat actactacag gaagaagtgc gagtccattg 1200
tggagccaaa gccgacatta aagtatgtgt cctttgtgga tgaatcccac attaggatgg 1260
tgaaccagca gctactaggg agaagtctgc aagatgtcaa gggccaagaa gtcccaagac 1320
ctgcgatgga cttcacagat ttgtccaggc tgcccctggc cctccatgac acacccccga 1380
ttcctggaca accagaggag atacagctgc ttagaaagga ggcgactcct agatccaggg 1440
atagccccgt ctggtgccag tgtggaagct gcctcccatc tcaactccct gagagccaca 1500
ggtgcctgga ggagctgtgc tgccggaaaa agccgggggc ctgcatcacc acctcagagc 1560
tgttcaggaa gctggtcctg tccagacacg tcctgcagtt cctcctgctc taccaggagc 1620
ccttgctggc gctggatgtg gattccacca acagccggct gcggcactgt gcctacaggt 1680
gctacgccac ctggcgcttc ggctcccagg acatggctga ctttgccatc ctgcccagct 1740
gctgccgctg gaggatccgg aaagagtttc cgaagagtga agggcagtac agtggcttca 1800
agagtcctta ctgaagccag gcaccgtggc tcacgtctgt aatcccacct ttt 1853

<210> 159
<211> 3205
<212> DNA
<213> Homo sapiens

<400> 159
aaaggggcaa gagctgagcg gaacaccggc ccgccgtcgc ggcagctgct tcacccctct 60
ctctgcagcc atggggctcc ctcgtggacc tctcgcgtct ctcctccttc tccaggtttg 120
ctggctgcag tgcgcggcct ccgagccgtg ccgggcggtc ttcagggagg ctgaagtgac 180
cttggaggcg ggaggcgcgg agcaggagcc cggccaggcg ctggggaaag tattcatggg 240
ctgccctggg caagagccag ctctgtttag cactgataat gatgacttca ctgtgcggaa 300
tggcgagaca gtccaggaaa gaaggtcact gaaggaaagg aatccattga agatcttccc 360
atccaaacgt atcttacgaa gacacaagag agattgggtg gttgctccaa tatctgtccc 420
tgaaaatggc aagggtccct tcccccagag actgaatcag ctcaagtcta ataaagatag 480
agacaccaag attttctaca gcatcacggg gccgggggca gacagccccc ctgagggtgt 540
cttcgctgta gagaaggaga caggctggtt gttgttgaat aagccactgg accgggagga 600
gattgccaag tatgagctct ttggccacgc tgtgtcagag aatggtgcct cagtggagga 660
ccccatgaac atctccatca tcgtgaccga ccagaatgac cacaagccca agtttaccca 720
ggacaccttc cgagggagtg tcttagaggg agtcctacca ggtacttctg tgatgcaggt 780
gacagccacg gatgaggatg atgccatcta cacctacaat ggggtggttg cttactccat 840
ccatagccaa gaaccaaagg acccacacga cctcatgttc accattcacc ggagcacagg 900
caccatcagc gtcatctcca gtggcctgga ccgggaaaaa gtccctgagt acacactgac 960
catccaggcc acagacatgg atggggacgg ctccaccacc acggcagtgg cagtagtgga 1020
gatccttgat gccaatgaca atgctcccat gtttgacccc cagaagtacg aggcccatgt 1080
gcctgagaat gcagtgggcc atgaggtgca gaggctgacg gtcactgatc tggacgcccc 1140
caactcacca gcgtggcgtg ccacctacct tatcatgggc ggtgacgacg gggaccattt 1200
taccatcacc acccaccctg agagcaacca gggcatcctg acaaccagga agggtttgga 1260
ttttgaggcc aaaaaccagc acaccctgta cgttgaagtg accaacgagg ccccttttgt 1320
gctgaagctc ccaacctcca cagccaccat agtggtccac gtggaggatg tgaatgaggc 1380
acctgtgttt gtcccaccct ccaaagtcgt tgaggtccag gagggcatcc ccactgggga 1440
gcctgtgtgt gtctacactg cagaagaccc tgacaaggag aatcaaaaga tcagctaccg 1500
catcctgaga gacccagcag ggtggctagc catggaccca gacagtgggc aggtcacagc 1560

tgtgggcacc ctcgaccgtg aggatgagca gtttgtgagg aacaacatct atgaagtcat 1620
ggtcttggcc atggacaatg gaagccctcc caccactggc acgggaaccc ttctgctaac 1680
actgattgat gtcaatgacc atggcccagt ccctgagccc cgtcagatca ccatctgcaa 1740
ccaaagccct gtgcgccagg tgctgaacat cacggacaag gacctgtctc cccacacctc 1800
ccctttccag gcccagctca cagatgactc agacatctac tggacggcag aggtcaacga 1860
ggaaggtgac acagtggtct tgtccctgaa gaagttcctg aagcaggata catatgacgt 1920
gcacctttct ctgtctgacc atggcaacaa agagcagctg acggtgatca gggccactgt 1980
gtgcgactgc catggccatg tcgaaacctg ccctggaccc tggaagggag gtttcatcct 2040
ccctgtgctg ggggctgtcc tggctctgct gttcctcctg ctggtgctgc ttttgttggt 2100
gagaaagaag cggaagatca aggagcccct cctactccca gaagatgaca cccgtgacaa 2160
cgtcttctac tatggcgaag aggggggtgg cgaagaggac caggactatg acatcaccca 2220
gctccaccga ggtctggagg ccaggccgga ggtggttctc cgcaatgacg tggcaccaac 2280
catcatcccg acacccatgt accgtcctcg gccagccaac ccagatgaaa tcggcaactt 2340
tataattgag aacctgaagg cggctaacac agaccccaca gccccgccct acgacaccct 2400
cttggtgttc gactatgagg gcagcggctc cgacgccgcg tccctgagct ccctcacctc 2460
ctccgcctcc gaccaagacc aagattacga ttatctgaac gagtggggca gccgcttcaa 2520
gaagctggca gacatgtacg gtggcgggga ggacgactag gcggcctgcc tgcagggctg 2580
gggaccaaac gtcaggccac agagcatctc caaggggtct cagttccccc ttcagctgag 2640
gacttcggag cttgtcagga agtggccgta gcaacttggc ggagacaggc tatgagtctg 2700
acgttagagt ggttgcttcc ttagcctttc aggatggagg aatgtgggca gtttgacttc 2760
agcactgaaa acctctccac ctgggccagg gttgcctcag aggccaagtt tccagaagcc 2820
tcttacctgc cgtaaaatgc tcaaccctgt gtcctgggcc tgggcctgct gtgactgacc 2880
tacagtggac tttctctctg gaatggaacc ttcttaggcc tcctggtgca acttaatttt 2940
tttttttaat gctatcttca aaacgttaga gaaagttctt caaaagtgca gcccagagct 3000
gctgggccca ctggccgtcc tgcatttctg gtttccagac cccaatgcct cccattcgga 3060
tggatctctg cgtttttata ctgagtgtgc ctaggttgcc ccttattttt tattttccct 3120
gttgcgttgc tatagatgaa gggtgaggac aatcgtgtat atgtactaga acttttttat 3180
taaagaaact tttcccagaa aaaaa 3205

<210> 160
<211> 811
<212> DNA
<213> Homo sapiens

<400> 160
catccctctg gctccagagc tcagagccac ccacagccgc agccatgctg tgcctcctgc 60
tcaccctggg cgtggccctg gtctgtggtg tcccggccat ggacatcccc cagaccaagc 120
aggacctgga gctcccaaag ttggcaggga cctggcactc catggccatg gcgaccaaca 180
acatctccct catggcgaca ctgaaggccc ctctgagggt ccacatcacc tcactgttgc 240
ccacccccga ggacaacctg gagatcgttc tgcacagatg ggagaacaac agctgtgttg 300
agaagaaggt ccttggagag aagactggga atccaaagaa gttcaagatc aactatacgg 360
tggcgaacga ggccacgctg ctcgatactg actacgacaa tttcctgttt ctctgcctac 420
aggacaccac cacccccatc cagagcatga tgtgccagta cctggccaga gtcctggtgg 480
aggacgatga gatcatgcag ggattcatca gggctttcag gcccctgccc aggcacctat 540
ggtacttgct ggacttgaaa cagatggaag agccgtgccg tttctagctc acctccgcct 600
ccaggaagac cagactccca cccttccaca cctccagagc agtgggactt cctcctgccc 660
tttcaaagaa taaccacagc tcagaagacg atgacgtggt catctgtgtc gccatcccct 720
tcctgctgca cacctgcacc attgccatgg ggaggctgct ccctgggggc agagtctctg 780
gcagaggtta ttaataaacc cttggagcat g 811

<210> 161
<211> 780
<212> DNA
<213> Homo sapiens

<400> 161
agcagggggc gctgtgtgta ccgagaatac gagaatacct cgtgggccct gaccttctct 60
ctgagagccg ggcagaggct ccggagccat gcaggccgaa ggccggggca cagggggttc 120
gacgggcgat gctgatggcc caggaggccc tggcattcct gatggcccag ggggcaatgc 180
tggcggccca ggagaggcgg gtgccacggg cggcagaggt ccccggggcg caggggcagc 240
aagggcctcg gggccgggag gaggcgcccc gcggggtccg catggcggcg cggcttcagg 300
gctgaatgga tgctgcagat gcggggccag ggggccggag agccgcctgc ttgagttcta 360

cctcgccatg cctttcgcga cacccatgga agcagagctg gcccgcagga gcctggccca 420
ggatgcccca ccgcttcccg tgccaggggt gcttctgaag gagttcactg tgtccggcaa 480
catactgact atccgactga ctgctgcaga ccaccgccaa ctgcagctct ccatcagctc 540
ctgtctccag cagctttccc tgttgatgtg gatcacgcag tgctttctgc ccgtgttttt 600
ggctcagcct ccctcagggc agaggcgcta agcccagcct ggcgcccctt cctaggtcat 660
gcctcctccc ctagggaatg gtcccagcac gagtggccag ttcattgtgg gggcctgatt 720
gtttgtcgct ggaggaggac ggcttacatg tttgtttctg tagaaaataa aactgagcta 780

<210> 162
<211> 608
<212> DNA
<213> Homo sapiens

<400> 162
cctcgtgggc cctgaccttc tctctgagag ccgggcagag gctccggagc catgcaggcc 60
gaaggccagg gcacaggggg ttcgacgggc gatgctgatg gcccaggagg ccctggcatt 120
cctgatggcc cagggggcaa tgctggcggc ccaggagagg cgggtgccac gggcggcaga 180
ggtccccggg gcgcaggggc agcaagggcc tcggggccga gaggaggcgc cccgcggggt 240
ccgcatggcg gtgccgcttc tgcgcaggat ggaaggtgcc cctgcggggc caggaggccg 300
gacagccgcc tgcttcagtt ccgactgact gctgcagacc accgccaact gcagctctcc 360
atcagctcct gtctccagca gctttccctg ttgatgtgga tcacgcagtg ctttctgccc 420
gtgtttttgg ctcaggctcc ctcagggcag aggcgctaag cccagcctgg cgccccttcc 480
taggtcatgc ctcctcccct agggaatggt cccagcacga gtggccagtt cattgtgggg 540
gcctgattgt ttgtcgctgg aggaggacgg cttacatgtt tgtttctgta gaaaataaag 600
ctgagcta 608

<210> 163
<211> 1986
<212> DNA
<213> Homo sapiens

<400> 163
atggatctgg tgctaaaaag atgccttctt catttggctg tgataggtgc tttgctggct 60
gtgggggcta caaaagtacc cagaaaccag gactggcttg gtgtctcaag gcaactcaga 120
accaaagcct ggaacaggca gctgtatcca gagtggacag aagcccagag acttgactgc 180
tggagaggtg gtcaagtgtc cctcaaggtc agtaatgatg ggcctacact gattggtgca 240
aatgcctcct tctctattgc cttgaacttc cctggaagcc aaaaggtatt gccagatggg 300
caggttatct gggtcaacaa taccatcatc aatgggagcc aggtgtgggg aggacagcca 360
gtgtatcccc aggaaactga cgatgcctgc atcttccctg atggtggacc ttgcccatct 420
ggctcttggt ctcagaagag aagctttgtt tatgtctgga agacctgggg ccaatactgg 480
caagttctag ggggcccagt gtctgggctg agcattggga caggcagggc aatgctgggc 540
acacacacca tggaagtgac tgtctaccat cgccggggat cccggagcta tgtgcctctt 600
gctcattcca gctcagcctt caccattact gaccaggtgc ctttctccgt gagcgtgtcc 660
cagttgcggg ccttggatgg agggaacaag cacttcctga gaaatcagcc tctgaccttt 720
gccctccagc tccatgaccc cagtggctat ctggctgaag ctgacctctc ctacacctgg 780
gactttggag acagtagtgg aaccctgatc tctcgggcac ttgtggtcac tcatacttac 840
ctggagcctg gcccagtcac tgcccaggtg gtcctgcagg ctgccattcc tctcacctcc 900
tgtggctcct ccccagttcc aggcaccaca gatgggcaca ggccaactgc agaggcccct 960
aacaccacag ctggccaagt gcctactaca gaagttgtgg gtactacacc tggtcaggcg 1020
ccaactgcag agccctctgg aaccacatct gtgcaggtgc caaccactga agtcataagc 1080
actgcacctg tgcagatgcc aactgcagag agcacaggta tgacacctga gaaggtgcca 1140
gtttcagagg tcatgggtac cacactggca gagatgtcaa ctccagaggc tacaggtatg 1200
acacctgcag aggtatcaat tgtggtgctt tctggaacca cagctgcaca ggtaacaact 1260
acagagtggg tggagaccac agctagagag ctacctatcc ctgagcctga aggtccagat 1320
gccagctcaa tcatgtctac ggaaagtatt acaggttccc tgggccccct gctggatggt 1380
acagccacct taaggctggt gaagagacaa gtccccctgg attgtgttct gtatcgatat 1440
ggttcctttt ccgtcaccct ggacattgtc cagggtattg aaagtgccga gatcctgcag 1500
gctgtgccgt ccggtgaggg ggatgcattt gagctgactg tgtcctgcca aggcgggctg 1560
cccaaggaag cctgcatgga gatctcatcg ccagggtgcc agccccctgc ccagcggctg 1620
tgccagcctg tgctacccag cccagcctgc cagctggttc tgcaccagat actgaagggt 1680
ggctcgggga catactgcct caatgtgtct ctggctgata ccaacagcct ggcagtggtc 1740
agcacccagc ttatcatgcc tggtcaagaa gcaggccttg ggcaggttcc gctgatcgtg 1800
ggcatcttgc tggtgttgat ggctgtggtc cttgcatctc tgatatatag gcgcagactt 1860

atgaagcaag acttctccgt accccagttg ccacatagca gcagtcactg gctgcgtcta 1920
ccccgcatct tctgctcttg tcccattggt gagaatagcc ccctcctcag tgggcagcag 1980
gtctga 1986

<210> 164
<211> 2291
<212> DNA
<213> Homo sapiens

<400> 164
gcaattaaag tcaagagcta aggagggagg gagagggttt agaaatacca gcataataag 60
tagtatgact gggtgctctg taaattaact caattagaca aagcctgact taacggggga 120
agatggtgag aagcgctacc ctcattaaat ttggttgtta gaggcgcttc taaggaaatt 180
aagtctgtta gttgtttgaa tcacataaaa ttgtgtgtgc acgttcatgt acacatgtgc 240
acacatgtaa cctctgtgat tcttgtgggt atttttttaa gaagaaagga atagaaagca 300
aagaaaaata aaaaatactg aaaagaaaag actgaaagag tagaagataa ggagaaaagt 360
acgacagaga caaggaaagt aagagagaga gagagctctc ccaattataa agccatgagc 420
cccctttggt gggggtttct gctcagttgc ttgggctgca aaatcctgcc aggagcccag 480
ggtcagttcc cccgagtctg catgacggtg gacagcctag tgaacaagga gtgctgccca 540
cgcctgggtg cagagtcggc caatgtctgt ggctctcagc aaggccgggg gcagtgcaca 600
gaggtgcgag ccgacacaag gccctggagt ggtccctaca tcctacgaaa ccaggatgac 660
cgtgagctgt ggccaagaaa attcttccac cggacctgca agtgcacagg aaactttgcc 720
ggctataatt gtggagactg caagtttggc tggaccggtc ccaactgcga gcggaagaaa 780
ccaccagtga ttcggcagaa catccattcc ttgagtcctc aggaaagaga gcagttcttg 840
ggcgccttag atctcgcgaa gaagagagta caccccgact acgtgatcac cacacaacac 900
tggctgggcc tgcttgggcc caatggaacc cagccgcagt ttgccaactg cagtgtttat 960
gatttttttg tgtggctcca ttattattct gttagagata cattattagg accaggacgc 1020
ccctacaggg ccatagattt ctcacatcaa ggacctgcat ttgttacctg gcaccggtac 1080
catttgttgt gtctggaaag agatctccag cgactcattg gcaatgagtc ttttgctttg 1140
ccctactgga actttgccac tgggaggaac gagtgtgatg tgtgtacaga ccagctgttt 1200
ggggcagcga gaccagacga tccgactctg attagtcgga actcaagatt ctccagctgg 1260
gaaactgtct gtgatagctt ggatgactac aaccacctgg tcaccttgtg caatggaacc 1320
tatgaaggtt tgctgagaag aaatcaaatg ggaagaaaca gcatgaaatt gccaacctta 1380
aaagacatac gagattgcct gtctctccag aagtttgaca atcctccctt cttccagaac 1440
tctaccttca gtttcaggaa tgctttggaa gggtttgata aagcagatgg gactctggat 1500
tctcaagtga tgagccttca taatttggtt cattccttcc tgaacgggac aaacgctttg 1560
ccacattcag ccgccaatga tcccattttt gtggttcttc attcctttac tgatgccatc 1620
tttgatgagt ggatgaaaag atttaatcct cctgcagatg cctggcctca ggagctggcc 1680
cctattggtc acaatcggat gtacaacatg gttcctttct tccctccagt gactaatgaa 1740
gaactctttt taacctcaga ccaacttggc tacagctatg ccatcgatct gccagtttca 1800
gttgaagaaa ctccaggttg gcccacaact ctcttagtag tcatgggaac actggtggct 1860
ttggttggtc tttttgtgct gttggctttt cttcaatata gaagacttcg aaaaggatat 1920
acacccctaa tggagacaca tttaagcagc aagagataca cagaagaagc ctagggtgct 1980
catgccttac ctaagagaag aggctggcca agccacagtt ctgacgctga caataaagga 2040
actaatcctc actgttcctt cttgagttga agatctttga cataggttct tctatagtga 2100
tgatgatctc attcagaaga tgcttagctg tagtttccgc tttgcttgct tgtttaacaa 2160
acccaactaa agtgcttgag gctacctcta ccttcaaata aagatagacc tgacaatttg 2220
tgatatctaa taataacccc ccccccaata ttgattaagc ctcctccttt tctgaaagca 2280
tttaaaaaaa a 2291

<210> 165
<211> 2105
<212> DNA
<213> Homo sapiens

<400> 165
tgtggggctg cccgggctgc gcggcgtctg caggcgccac cgctgcctct ttccggctgt 60
gaccctcctc gccgccgccg cttggctgcg tcctccgact ccccgcgccg ccgagaccag 120
gctcccgctc cggttgcggc cgcaccgccc tccgcggccg ccccctgggg atccagcgag 180
cgcggtcgtc cttggtggaa ggaaccatga actggcatct ccccctcttc ctcttggcct 240
ctgtgacgct gccttccatc tgctcccact tcaatcctct gtctctcgag gaactaggct 300
ccaacacggg gatccaggtt ttcaatcaga ttgtgaagtc gaggcctcat gacaacatcg 360
tgatctctcc ccatgggatt gcgtcggtcc tggggatgct tcagctgggg gcggacggca 420

ggaccaagaa gcagctcgcc atggtgatga gatacggcgt aaatggagtt ggtaaaatat 480
taaagaagat caacaaggcc atcgtctcca agaagaataa agacattgtg acagtggcta 540
acgccgtgtt tgttaagaat gcctctgaaa ttgaagtgcc ttttgttaca aggaacaaag 600
atgtgttcca gtgtgaggtc cggaatgtga actttgagga tccagcctct gcctgtgatt 660
ccatcaatgc atgggttaaa aatgaaacca gggatatgat tgacaatctg ctgtccccag 720
atcttattga tggtgtgctc accagactgg tcctcgtcaa cgcagtgtat ttcaagggtc 780
tgtggaaatc acggttccaa cccgagaaca caaagaaacg cactttcgtg gcagccgacg 840
ggaaatccta tcaagtgcca atgctggccc agctctccgt gttccggtgt gggtcgacaa 900
gtgcccccaa tgatttatgg tacaacttca ttgaactgcc ctaccacggg gaaagcatca 960
gcatgctgat tgcactgccg actgagagct ccactccgct gtctgccatc atcccacaca 1020
tcagcaccaa gaccatagac agctggatga gcatcatggt gcccaagagg gtgcaggtga 1080
tcctgcccaa gttcacagct gtagcacaaa cagatttgaa ggagccgctg aaagttcttg 1140
gcattactga catgtttgat tcatcaaagg caaattttgc aaaaataaca aggtcagaaa 1200
acctccatgt ttctcatatc ttgcaaaaag caaaaattga agtcagtgaa gatggaacca 1260
aagcttcagc agcaacaact gcaattctca ttgcaagatc atcgcctccc tggtttatag 1320
tagacagacc ttttctgttt ttcatccgac ataatcctac aggtgctgtg ttattcatgg 1380
ggcagataaa caaaccctga agagtataca aaagaaacca tgcaaagcaa cgactacttt 1440
gctacgaaga aagactcctt tcctgcatct ttcatagttc tgttaaatat ttttgtacat 1500
cgcttctttt tcaaaactag ttcttaggaa cagactcgat gcaagtgttt ctgttctggg 1560
aggtattgga gggaaaaaac aagcaggatg gctggaacac tgtactgagg aatgaataga 1620
aaggcttcca gatgtctaaa agattcttta aactactgaa ctgttaccta ggttaacaac 1680
cctgttgagt atttgctgtt tgtccagttc aggaattttt gttttgtttt gtctatatgt 1740
gcggcttttc agaagaaatt taatcagtgt gacagaaaaa aaaatgtttt atggtagctt 1800
ttacttttta tgaaaaaaaa attatttgcc ttttaaattc ttttccccca tccccctcca 1860
aagtcttgat agcaagcgtt attttggtgg tagaaacggt gaaatctcta gcctctttgt 1920
gtttttgttg ttgttgttgt tgttgtttta tataatgcat gtattcacta aaataaaatt 1980
taaaaaactc ctgtcttgct agacaaggtt gctgttgtgc agtgtgcctg tcactactgg 2040
tctgtactcc ttggatttgc atttttgtat tttgtacaaa gtaaaaataa actgttatga 2100
gtagt 2105

<210> 166
<211> 2587
<212> DNA
<213> Homo sapiens

<400> 166
ggggccgccg ggcaggagga gttggaggag gacgatgcct ttatcagcgt gcaggtgccg 60
gcggccgcct ttttgggctc cgggaccccc gggagtggga gcggcagtcg gggacgcctc 120
aactcgttca ctcagggaat cctgcccatc gccttctcca ggccgacttc gcagaactac 180
tgctccctgg agcagccagg ccagggcggc agcaccagcg ccttcgagca gctgcagagg 240
tcccgacggc gcctcatctc ccagagatct tccttggaga ccctggaaga tattgaggag 300
aacgcccctc tccggagatg tcgaactctc tcaggttcac ccagaccaaa gaattttaag 360
aagattcatt ttatcaagaa catgcggcaa cacgatacca ggaatggcag aatagtcctt 420
atcagtggca gaagatcctt ctgtagtata ttttcagtgc tgccgtatcg cgacagtacc 480
caagtcgggg acttgaagtt ggacggagga agacaatcaa ctggtgcagt gagtttgaaa 540
gagatcattg gtctggaagg tgtggagctg ggtgctgatg ggaagactgt ttcctatacc 600
caatttctgt tacccacaaa tgcctttgga gcccggagaa ataccataga ctccacctcc 660
tctttctccc agttccgtaa cctgagccac cgcagcctct ccataggccg ggcaagcggc 720
acccagggga gcctcgacac aggtagtgac ctgggagact ttatggacta tgacccaaat 780
ctcttggatg acccccagtg gccttgtggc aaacacaaac gcgttctgat cttcccttcc 840
tacatgacaa cagtgattga ctacgtgaag ccctcggatc tcaagaagga catgaacgag 900
accttcaagg agaagtttcc tcacattaag ctgacactca gcaaaattag gagtctgaaa 960
cgagagatgc ggaagcttgc gcaggaggac tgtggccttg aggagcccac ggtggccatg 1020
gccttcgtct actttgaaaa gctcgccctc aaggggaaac tcaacaaaca gaaccggaag 1080
ctgtgtgctg gggcatgtgt gctgttagca gccaaaattg gaagtgacct caaaaaacac 1140
gaagtcaagc atttaattga caaactggaa gagaagttcc ggctgaacag gcgagaactg 1200
attgcctttg aattcccggt gttagtggcc ttggaattcg ccctccactt gcccgagcac 1260
gaagtcatgc cccactacag acggctggtc cagagttcct agcactggcc ccgaggacag 1320
ccaagggcca tttcttctca gcttggtgga gcagcactta cttactactg gaaatgaaaa 1380
aaagtagaac tcagaatacc agacttttct tcctctcgac atagtttggg gagaagcagt 1440
actagaaact ttccaaggag tcttgggtgt gtagccaaga ggagccatga gctatggact 1500
cctcaagcac gggaagagga ggtgtgtgct gagaacagag aggccctgcc ctctgtccac 1560
tagcgagaat ccctagctgc cccagcccag tctttctccc cggcattcac aaactttgca 1620

agcgtggtcc agggccttct ccagatctgt tccaacttgg agtgtgaagg gcttgagcat 1680
acgggggaag agagtctgca gaagttgggg gaaaactttt aaaagatacc ctcattgtgt 1740
caaagagtgt gccaatctat ttttgtatca gcattggaag tgcactttcc cctggggcgt 1800
gtgggtgtgt gaatgtgcaa gtgtctgaga gatactgcat cagccctaga cccccagagc 1860
cagtcccgcc ctttacagag cagcccttag cctggggcca tgggtcaggc tgaccttcaa 1920
caattatttc tagatgattt ctggataaga attgctctct cggtaccaga cagtttgaca 1980
tcctccaccc ttagaaaatg actgacattg ttttgttact gctcctaccc accaagggga 2040
taaagaaggc gagttctgag tgttggatga gtcagtcgcg tggaaggacg tggagcgtgg 2100
cgctctgtaa cttcctgccg tctgccaccc cgccacgtgt atttaaccct cgcactttct 2160
ccactgtgga gatggctggg gcggcgcccc acagtgtgta ttcctgtcct ctatgttaga 2220
gtgcatcaga agcacattta ctgtgctatc tatatcgcta tataaaagtg ttttataaaa 2280
acccagaata ggagcacgac gcatgattgg tgtttgaggc gtttgccagc tgggacaaac 2340
tgcgtttgga gctgtggtta agctgactaa ggaggcggtg gctctttctt aacattccca 2400
cgtgcccagg gctgttcatg caagatttta atggtgactt gtcctggctt actgggacag 2460
tctgtatgag gcatgtcacc acactgtcgc ctcatagctg caagagagag gcaccagctg 2520
aagttcccct gactgaagag agcctgtggc catgtaaaaa gagaattaaa ctcttgttgc 2580
tttttgt 2587

<210> 167
<211> 2827
<212> DNA
<213> Homo sapiens

<400> 167
ggcacgaggc tccggcctct ggactaggaa ccgacagccc ccctccccgc gtccctccct 60
ctctctccag ccgttttggg gaggggctct ccacgctccg gatagttccc gagggtcatc 120
cgcgccgcac tcgcctttcc gtttcgcctt cacctggata taatttccga gcgaagctgc 180
ccccaggatg accacgctgg ccggcgctgt gcccaggatg atgcggccgg gcccggggca 240
gaactacccg cgtagcgggt tcccgctgga agtgtccact cccctcggcc agggccgcgt 300
caaccagctc ggcggcgttt ttatcaacgg caggccgctg cccaaccaca tccgccacaa 360
gatcgtggag atggcccacc acggcatccg gccctgcgtc atctcgcgcc agctgcgcgt 420
gtcccacggc tgcgtctcca agatcctgtg caggtaccag gagactggct ccatacgtcc 480
tggtgccatc ggcggcagca agcccaaggt gacaacgcct gacgtggaga agaaaattga 540
ggaatacaaa agagagaacc cgggcatgtt cagctgggaa atccgagaca aattactcaa 600
ggacgcggtc tgtgatcgaa acaccgtgcc gtcagtgagt tccatcagcc gcatcctgag 660
aagtaaattc gggaaaggtg aagaggagga ggccgacttg gagaggaagg aggcagagga 720
aagcgagaag aaggccaaac acagcatcga cggcatcctg agcgagcgag cctcagcacc 780
ccaatcagat gaaggctctg atattgactc tgaaccagat ttaccactaa agaggaaaca 840
gcgcagaagc cgaaccacct tcacagcaga acagctggag gaactggagc gtgcttttga 900
gagaactcat taccctgaca tttatactag ggaggaactg gcccagaggg cgaagctcac 960
cgaggcccga gtacaggtct ggtttagcaa ccgccgtgca agatggagga agcaagctgg 1020
ggccaatcaa ctgatggctt tcaaccatct cattcccggg gggttccctc ccactgccat 1080
gccgaccttg ccaacgtacc agctgtcgga gacctcttac cagcccacat ctattccaca 1140
agctgtgtca gatcccagca gcaccgttca cagacctcaa ccgcttcctc caagcactgt 1200
acaccaaagc acgattcctt ccaacccaga cagcagctct gcctactgcc tccccagcac 1260
caggcatgga ttttccagct atacagacag ctttgtgcct ccgtcggggc cctccaaccc 1320
catgaacccc accattggca atggcctctc acctcagaat tcaattcgtc ataatctgtc 1380
cctacacagc aagttcattc gtgtgcagaa tgaaggaact ggaaaaagtt cttggtggat 1440
gctcaatcca gagggtggca agagcgggaa atctcctagg agaagagctg catccatgga 1500
caacaacagt aaatttgcta agagccgaag ccgagctgcc aagaagaaag catctctcca 1560
gtctggccag gagggtgctg gggacagccc tggatcacag ttttccaaat ggcctgcaag 1620
ccctggctct cacagcaatg atgactttga taactggagt acatttcgcc ctcgaactag 1680
ctcaaatgct agtactatta gtgggagact ctcacccatt atgaccgaac aggatgatct 1740
tggagaaggg gatgtgcatt ctatggtgta cccgccatct gccgcaaaga tggcctctac 1800
tttacccagt ctgtctgaga taagcaatcc cgaaaacatg gaaaatcttt tggataatct 1860
caaccttctc tcatcaccaa catcattaac tgtttcgacc cagtcctcac ctggcaccat 1920
gatgcagcag acgccgtgct actcgtttgc gccaccaaac accagtttga attcacccag 1980
cccaaactac caaaaatata catatggcca atccagcatg agccctttgc cccagatgcc 2040
tatacaaaca cttcaggaca ataagtcgag ttatggaggt atgagtcagt ataactgtgc 2100
gcctggactc ttgaaggagt tgctgacttc tgactctcct ccccataatg acattatgac 2160
accagttgat cctggggtag cccagcccaa cagccgggtt ctgggccaga acgtcatgat 2220
gggccctaat tcggtcatgt caacctatgg cagccaggca tctcataaca aaatgatgaa 2280
tcccagctcc catacccacc ctggacatgc tcagcagaca tctgcagtta acgggcgtcc 2340

cctgccccac acggtaagca ccatgcccca cacctcgggt atgaaccgcc tgacccaagt 2400
gaagacacct gtacaagtgc ctctgcccca ccccatgcag atgagtgccc tggggggcta 2460
ctcctccgtg agcagctgca atggctatgg cagaatgggc cttctccacc aggagaagct 2520
cccaagtgac ttggatggca tgttcattga gcgcttagac tgtgacatgg aatccatcat 2580
tcggaatgac ctcatggatg gagatacatt ggattttaac tttgacaatg tgttgcccaa 2640
ccaaagcttc ccacacagtg tcaagacaac gacacatagc tgggtgtcag gctgagggtt 2700
agtgagcagg ttacacttaa aagtacttca gattgtctga cagcaggaac tgagagaagc 2760
agtccaaaga tgtctttcac caactccctt ttagttttct tggttaaaaa aaaaaaaaaa 2820
aaaaaaa 2827

<210> 168
<211> 5121
<212> DNA
<213> Homo sapiens

<400> 168
gcgaggagga aacggtgccg gagcgcgcag ggcttgctgc cgccaccgcc gctgcacagg 60
ctgccggagc gagcctgccg cgcgccgccc tccccgctct ccttcctggg cgagctgcgg 120
ggatggggcg gccgcgggag cccgagcgcg cgcaggaacc gccgccgccg ccgcccgcgt 180
ctccgttgcc gcgcgcctga gccgccgtcg ccgccgcgcg ccctgcccgg gggcggcccc 240
cccagcccca tggaggtctc ccggaggaag gcgccgccgc gccccccgcg ccccgcagcg 300
ccactgcccc tgctcgccta tctgctggca ctggcggctc ccggccgggg cgcggacgag 360
cccgtgtggc ggtcggagca agccatcgga gccatcgcgg cgagccagga ggacggcgtg 420
tttgtggcga gcggcagctg cctggaccag ctggactaca gcctggagca cagcctctcg 480
cgcctgtacc gggaccaagc gggcaactgc acagagccgg tctcgctggc gccccccgcg 540
cggccccggc ccgggagcag cttcagcaag ctgctgctgc cctaccgcga gggggcggcc 600
ggcctcgggg ggctgctgct caccggctgg accttcgacc ggggcgcctg cgaggtgcgg 660
cccctgggca acctgagccg caactccctg cgcaacggca ccgaggtggt gtcgtgccac 720
ccgcagggct cgacggccgg cgtggtgtac cgcgcgggcc ggaacaaccg ctggtacctg 780
gcggtggccg ccacctacgt gctgcctgag ccggagacgg cgagccgctg caaccccgcg 840
gcatccgacc acgacacggc catcgcgctc aaggacacgg aggggcgcag cctggccacg 900
caggagctgg ggcgcctcaa gctgtgcgag ggcgcgggca gcctgcactt cgtggacgcc 960
tttctctgga acggcagcat ctacttcccc tactacccct acaactatac gagcggcgct 1020
gccaccggct ggcccagcat ggcgcgcatc gcgcagagca ccgaggtgct gttccagggc 1080
caggcatccc tcgactgcgg ccacggccac cccgacggcc gccgcctgct cctctcctcc 1140
agcctagtgg aggccctgga cgtctgggcg ggagtgttca gcgcggccgc tggagagggc 1200
caggagcggc gctcccccac caccacggcg ctctgcctct tcagaatgag tgagatccag 1260
gcgcgcgcca agagggtcag ctgggacttc aagacggccg agagccactg caaagaaggg 1320
gatcaacctg aaagagtcca accaatcgca tcatctacct tgatccattc cgacctgaca 1380
tccgtttatg gcaccgtggt aatgaacagg actgttttat tcttggggac tggagatggc 1440
cagttactta aggttattct tggtgagaat ttgacttcaa attgtccaga ggttatctat 1500
gaaattaaag aagagacacc tgttttctac aaactcgttc ctgatcctgt gaagaatatc 1560
tacatttatc taacagctgg gaaagaggtg aggagaattc gtgttgcaaa ctgcaataaa 1620
cataaatcct gttcggagtg tttaacagcc acagaccctc actgcggttg gtgccattcg 1680
ctacaaaggt gcacttttca aggagattgt gtacattcag agaacttaga aaactggctg 1740
gatatttcgt ctggagcaaa aaagtgccct aaaattcaga taattcgaag cagtaaagaa 1800
aagactacag tgactatggt gggaagcttc tctccaagac actcaaagtg catggtgaag 1860
aatgtggact ctagcaggga gctctgccag aataaaagtc agcccaaccg gacctgcacc 1920
tgtagcatcc caaccagagc aacctacaaa gatgtttcag ttgtcaacgt gatgttctcc 1980
ttcggttctt ggaatttatc agacagattc aactttacca actgctcatc attaaaagaa 2040
tgcccagcat gcgtagaaac tggctgcgcg tggtgtaaaa gtgcaagaag gtgtatccac 2100
cccttcacag cttgcgaccc ttctgattat gagagaaacc aggaacagtg tccagtggct 2160
gtcgagaaga catcaggagg aggaagaccc aaggagaaca aggggaacag aaccaaccag 2220
gctttacagg tcttctacat taagtccatt gagccacaga aagtatcgac attagggaaa 2280
agcaacgtga tagtaacggg agcaaacttt acccgggcat cgaacatcac aatgatcctg 2340
aaaggaacca gtacctgtga taaggatgtg atacaggtta gccatgtgct aaatgacacc 2400
cacatgaaat tctctcttcc atcaagccgg aaagaaatga aggatgtgtg tatccagttt 2460
gatggtggga actgctcttc tgtgggatcc ttatcctaca ttgctctgcc acattgttcc 2520
cttatatttc ctgctaccac ctggatcagt ggtggtcaaa atataaccat gatgggcaga 2580
aattttgatg taattgacaa cttaatcatt tcacatgaat taaaaggaaa cataaatgtc 2640
tctgaatatt gtgtggcgac ttactgcggg tttttagccc ccagtttaaa gagttcaaaa 2700
gtgcgcacga atgtcactgt gaagctgaga gtacaagaca cctacttgga ttgtggaacc 2760
ctgcagtatc gggaggaccc cagattcacg gggtatcggg tggaatccga ggtggacaca 2820

gaactggaag tgaaaattca aaaagaaaat gacaacttca atatttccaa aaaagacatt 2880
gaaattactc tcttccatgg ggaaaatggg caattaaatt gcagttttga aaatattact 2940
agaaatcaag atcttaccac catcctttgc aaaattaaag gcatcaagac tgcaagcacc 3000
attgccaact cttctaagaa agttcgggtc aagctgggaa acctggagct ctacgtcgag 3060
caggagtcag ttccttccac atggtatttt ctgattgtgc tccctgtctt gctagtgatt 3120
gtcatttttg cggccgtggg ggtgaccagg cacaaatcga aggagctgag tcgcaaacag 3180
agtcaacaac tagaattgct ggaaagcgag ctccggaaag agatacgtga cggctttgct 3240
gagctgcaga tggataaatt ggatgtggtt gatagttttg gaactgttcc cttccttgac 3300
tacaaacatt ttgctctgag aactttcttc cctgagtcag gtggcttcac ccacatcttc 3360
actgaagata tgcataacag agacgccaac gacaagaatg aaagtctcac agctttggat 3420
gccctaatct gtaataaaag ctttcttgtt actgtcatcc acacccttga aaagcagaag 3480
aacttttctg tgaaggacag gtgtctgttt gcctccttcc taaccattgc actgcaaacc 3540
aagctggtct acctgaccag catcctagag gtgctgacca gggacttgat ggaacagtgt 3600
agtaacatgc agccgaaact catgctgaga cgcacggagt ccgtcgtcga aaaactcctc 3660
acaaactgga tgtccgtctg cctttctgga tttctccggg agactgtcgg agagcccttc 3720
tatttgctgg tgacgactct gaaccagaaa attaacaagg gtcccgtgga tgtaatcact 3780
tgcaaagccc tgtacacact taatgaagac tggctgttgt ggcaggttcc ggaattcagt 3840
actgtggcat taaacgtcgt ctttgaaaaa atcccggaaa acgagagtgc agatgtctgt 3900
cggaatattt cagtcaatgt tctcgactgt gacaccattg gccaagccaa agaaaagatt 3960
ttccaagcat tcttaagcaa aaatggctct ccttatggac ttcagcttaa tgaaattggt 4020
cttgagcttc aaatgggcac acgacagaaa gaacttctgg acatcgacag ttcctccgtg 4080
attcttgaag atggaatcac caagctaaac accattggcc actatgagat atcaaatgga 4140
tccactataa aagtctttaa gaagatagca aattttactt cagatgtgga gtactcggat 4200
gaccactgcc atttgatttt accagattcg gaagcattcc aagatgtgca aggaaagaga 4260
catcgaggga agcacaagtt caaagtaaaa gaaatgtatc tgacaaagct gctgtcgacc 4320
aaggtggcaa ttcattctgt gcttgaaaaa ctttttagaa gcatttggag tttacccaac 4380
agcagagctc catttgctat aaaatacttt tttgactttt tggacgccca ggctgaaaac 4440
aaaaaaatca cagatcctga cgtcgtacat atttggaaaa caaacagcct tcctcttcgc 4500
ttctgggtaa acatcctgaa gaaccctcag tttgtctttg acattaagaa gacaccacat 4560
atagacggct gtttgtcagt gattgcccag gcattcatgg atgcattttc tctcacagag 4620
cagcaactag ggaaggaagc accaactaat aagcttctct atgccaagga tatcccaacc 4680
tacaaagaag aagtaaaatc ttattacaaa gcaatcaggg atttgcctcc attgtcatcc 4740
tcagaaatgg aagaattttt aactcaggaa tctaagaaac atgaaaatga atttaatgaa 4800
gaagtggcct tgacagaaat ttacaaatac atcgtaaaat attttgatga gattctaaat 4860
aaactagaaa gagaacgagg gctggaagaa gctcagaaac aactcttgca tgtaaaagtc 4920
ttatttgatg aaaagaagaa atgcaagtgg atgtaagcac tctggggcct ggcttaatct 4980
ggcaaagttc ttcagacgac ttgggagcaa aatggctgct tgagctactc tgtgtcgtta 5040
atttgttgtt tgcacatagg ttccactttg ggcactgtct ttttaagaga ccaaggcaca 5100
tgcacagctt ttagaaagca a 5121
<210> 169
<211> 708
<212> DNA
<213> Homo sapiens

<400> 169
atgcagagaa tccttgagga accagctgat gattgcacat ggtcaactcg gattcttctt 60
accctgagtg aatttataat gtcgctgcag cgtactgttt atccccacag tgagaagtgg 120
cgtgctctct cagtcccttc atcttcctat tttcaggagc tggtgggcac cagccaggaa 180
ctggctctca ccttctggca tttactgtct atgtttggat tcttcatcgt gtcctatggc 240
tttctcacag catttggcag gactcttttc cacttggatc tgctacaacc caaccttaca 300
ccttcacgct ttgacaagta cactggtctc ttcatctatg aaatagaggg agatgggtta 360
gatccatgtt ttcaaagcat ggtccaagga atcctagagg ttttgtggat gtccaaagtg 420
gagagtgcat accacactaa tgatggtgat actgctggag agggggtgga aaatggtacc 480
agtcaaaccc gaggcggagg aggagcagcc cgcagtcgct gccgcgagat ggaggagccc 540
acgcccgagc ccgtctatgt cgacgtagac aaagggctga ccttggcctg cttcgtcttc 600
ctctgcctct tccttgtcgt gatgatcatc cgctgtgcca aggtcatcat ggacccttac 660
agcgccatcc ccacatccac ctgggaggag cagcacctgg acgactga 708

<210> 170
<211> 936
<212> DNA
<213> Homo sapiens

<400> 170
atggagccaa gccacagcca acccacaatg gacatgaagc acatgcaaga gcgtccagct 60
gcctcgcctt taaaatgcca ggacggccgc ggctccgctc agagccccct ctcggctgct 120
ggcccgggct cgcccgcgcc gctgggggct ctgactcctg ccgagccggg cctgggcagc 180
gccccccgcg ccccgcgagc ccgcgccccc gtcaccggga aacgccgtag ccggcgatcc 240
gcggtcgccc tgggctcggc gccggcccag ccaggacccc gggagcgcac gggacgggcc 300
tcgccagccc tagaaaataa ctctgacctc ttgtccaagg catctgctat agctactggg 360
acaccaccct gtgaagactc taccattgca aggttctacc tgcccccacc tctcaccact 420
caccccgagc tggtgggcac cagccaggaa ctggctctca ccttctggca tttactgtct 480
atgtttggat tcttcatcgt gtcctatggc tttctcacag catttggcag gactcttttc 540
cacttggatc tgctacaacc caaccttaca ccttcacgct ttgacaagta cactggccag 600
ctcctcgagc tcccagttca ctcaaagtat gtactgacca tccactggag ccagggactg 660
tgcatttggt cgtcacatcc ctgtgaagca ggggtggaaa atggtaccag tcaaacccga 720
ggcggaggag gagcagcccg cagtcgctgc cgcgagatgg aggagcccac gcccgagccc 780
gtctatgtcg acgtagacaa agggctgacc ttggcctgct tcgtcttcct ctgcctcttc 840
cttgtcgtga tgatcatccg ctgtgccaag gtcatcatgg acccttacag cgccatcccc 900
acatccacct gggaggagca gcacctggac gactga 936

<210> 171
<211> 2097
<212> DNA
<213> Homo sapiens

<400> 171
atgggcaagg atttcatgtc taaaacacca aaagcaatgg caacacaagc caaaattgac 60
aaatgggatc taattaaact aaagagcttc tgcacagcaa aagaaactac catcagagtg 120
aacagacaac ctacagaatg ggagaaaatt tttgcaatct actcatctga cgaagggcta 180
atatccagaa tctacaatga acccaaacaa atttacaaga aaaaaacaaa caaccctatc 240
aacaagtggg cgaaggatat gaacagacac ttctcaaaag aagacattta tgcagccaac 300
agacacatga aaaaatgctc atcatcactg gccatcagag aaatgcaaat caaaaccaca 360
acgagatgcc atctcacacc agttagaatg gcgatcatta aaaagtcagg aaacaacaga 420
actgcagaag aacagccaaa actgagaaag gaagcagttg gatctattga gatattccgc 480
tttgctgatg gactggacat cacactcatg atcctgggta tactggcatc actggtcaat 540
ggagcctgcc ttcctttaat gccactggtt ttaggagaaa tgagtgataa ccttattagt 600
ggatgtctag tccaaactaa cacaacaaat tatcagaact gtactcagtc tcaagagaag 660
ctgaatgaag atatgactct gttgaccctg tattatgttg gaataggtgt tgctgccttg 720
atttttggtt acatacagat ttccttgtgg attataactg cagcacgaca gaccaagagg 780
attcgaaaac agttttttca ttcagttttg gcacaggaca tcggctggtt tgatagctgt 840
gacatcggtg aacttaacac tcgcatgaca gatgacattg acaaaatcag tgatggtatt 900
ggagataaga ttgctctgtt gtttcaaaac atgtctactt tttcgattgg cctggcagtt 960
ggtttggtga agggctggaa actcacccta gtgactctat ccacgtctcc tcttataatg 1020
gcttcagcgg cagcatgttc taggatggtc atctcattga ccagtaagga attaagtgcc 1080
tattccaaag ctggggctgt ggcagaagaa gtcttgtcat caatccgaac agtcatagcc 1140
tttagggccc aggagaaaga acttcaaagg tatacacaga atctcaaaga tgcaaaggat 1200
tttggcataa aaaggactat agcttcaaaa gtgtctcttg gtgctgtgta cttctttatg 1260
aatggaacct atggacttgc tttttggtat ggaacctcct tgattcttaa tggagaacct 1320
ggatatacca tcgggactgt tcttgctgtt ttctttagtg taatccatag cagttattgc 1380
attggagcag cagtccctca ctttgaaacc ttcgcaatag cccgaggagc tgcctttcat 1440
attttccagg ttattgataa gaaacccagt atagataact tttccacagc tggatataaa 1500
cctgaatcca tagaaggaac tgtggaattt aaaaatgttt ctttcaatta tccatcaaga 1560
ccatctatca agattctgaa aggtctgaat ctcagaatta agtctggaga gacagtcgcc 1620
ttggtcggtc tcaatggcag tgggaagagt acggtagtcc agcttctgca gaggttatat 1680
gatccggatg atggctttat catggtggat gagaatgaca tcagagcttt aaatgtgcgg 1740
cattatcgag accatattgg agtggttagt caagagcctg ttttgttcgg gaccaccatc 1800
agtaacaata tcaagtatgg acgagatgat gtgactgatg aagagatgga gagagcagca 1860
agggaagcaa atgcgtatga ttttatcatg gagtttccta ataaatttaa tacattggta 1920
ggggaaaaag gagctcaaat gagtggaggg cagaaacaga ggatcgcaat tgctcgtgcc 1980
ttagttcgaa accccaagat tctgatttta gatgaggcta cgtctgccct ggattcagaa 2040
agcaagtcag ctgttcaagc tgcactggag aaggataccc ccaggtattc attttga 2097

<210> 172
<211> 878
<212> DNA

<213> Homo sapiens

<400> 172
cctaattcct ctaatatctc tctgttttct ttagtgtaat ccatagcagt tattgcattg 60
gagcagcagt ccctcacttt gaaaccttcg caatagcccg aggagctgcc tttcatattt 120
tccaggttat tgataagaaa cccagtatag ataacttttc cacagctgga tataaacctg 180
aatccataga aggaactgtg gaatttaaaa atgtttcttt caattatcca tcaagaccat 240
ctatcaagat tctgaaaggt ctgaatctca gaattaagtc tggagagaca gtcgccttgg 300
tcggtctcaa tggcagtggg aagagtacgg tagtccagct tctgcagagg ttatatgatc 360
cggatgatgg ctttatcatg gtggatgaga atgacatcag agctttaaat gtgcggcatt 420
atcgagacca tattggagtg gttagtcaag agcctgtttt gttcgggacc accatcagta 480
acaatatcaa gtatggacga gatgatgtga ctgatgaaga gatggagaga gcagcaaggg 540
aagcaaatgc gtatgatttt atcatggagt ttcctaataa atttaataca ttggtagggg 600
aaaaaggagc tcaaatgagt ggagggcaga aacagaggat cgcaattgct cgtgccttag 660
ttcgaaaccc caagattctg attttagatg aggctacgtc tgccctggat tcagaaagca 720
agtcagctgt tcaagctgca ctggagaagg atacccccag gtattcattt tgacctaatt 780
tcacctcaag tggagaatcg ctgaccttga accagcgccc ttcgacagct ctggcccctc 840
aaacctcacc ctgacctcct gctgcctatg agctactg 878

<210> 173
<211> 1175
<212> DNA
<213> Homo sapiens

<400> 173
gagaagccct cttcccttta aaaaaaaaaa aaaaaaggct gcttctcgca gagtggaaag 60
ccccggtccc catcccacca aaaccatttg acaagcagga caacgaagag gcagaaggat 120
ctgggcctgt gcgcgacgcc ccgggggacg aggctcatgg agaagtttcg ggcggtgctg 180
gacctgcacg tcaagcacca cagcgccttg ggctacggcc tggtgaccct gctgacggcg 240
ggcggggagc gcatcttctc cgccgtggca ttccagtgcc cgtgcagcgc cgcctggaac 300
ctgccctacg gcctggtctt cttgctggtg ccggcgctcg cgctcttcct cctgggctac 360
gtgctgagcg cacgcacgtg gcgcctgctc accggatgct gctccagcgc ccgcgcgagt 420
tgcggatcgg cgctgcgcgg ctccctggtg tgcacgcaaa tcagcgcggc cgccgcgctc 480
gcgcccctca cctgggtggc cgtggcgctg ctcgggggcg ccttttacga gtgcgcggcc 540
accgggagcg cggccttcgc gcagcgcctg tgcctcggcc gcaaccgcag ctgcgccgcg 600
gagctgccgc tggtgccgtg caaccaggcc aaggcgtcgg acgtgcagga cctcctgaag 660
gatctgaagg ctcagtcgca ggtgttgggc tggatcttga tagcagttgt tatcatcatt 720
cttctgattt ttacatctgt cacccgatgc ctatctccag ttagttttct gcagctgaaa 780
ttctggaaaa tctatttgga acaggagcag cagatcctta aaagtaaagc cacagagcat 840
gcaactgaat tggcaaaaga gaatattaaa tgtttctttg agggctcgca tccaaaagaa 900
tataacactc caagcatgaa agagtggcag caaatttcat cactgtatac tttcaatccg 960
aagggccagt actacagcat gttgcacaaa tatgtcaaca gaaaagagaa gactcacagt 1020
atcaggtcta ctgaaggaga tacggtgatt cctgttcttg gctttgtaga ttcatctggt 1080
ataaacagca ctcctgagtt atgacctttt gaatgagtag aaaaaaaaat tgttttgaat 1140
tattgcttta ttaaaaaata aacattggta ttttt 1175

<210> 174
<211> 3665
<212> DNA
<213> Homo sapiens

<400> 174
ggctgggggg ccgcgggcgg gaggcggata aaaaggcccc agggcgcccg gggagggagc 60
ccgttagcgc tgctccgccg cggcgcccgc ccagccccgg actgtccgcg ctccatctgg 120
tatcttggcc tcagctgtcc ttgaagtcac catggcgagg tccccaccag ccaccctctt 180
tctgttcctg ctgctgctag gccagccccc tcccagcagg ccacagtcac tgggcaccac 240
taagctccgg ctggtgggcc cagagagcaa gccagaggag ggccgcctgg aggtgctgca 300
ccagggccag tggggcaccg tgtgtgatga caactttgct atccaggagg ccacagtggc 360
ttgccgccag ctgggcttcg aagctgcctt gacctgggcc cacagtgcca agtacggcca 420
aggggaggga cccatctggc tggacaatgt gcagtgtgtg ggcacagaga gctccttgga 480
ccagtgcggg tctaatggct ggggagtcag tgactgcagt cactcagaag acgtaggggt 540
gatatgccac ccccggcgcc atcgtggcta cctttctgaa actgtctcca atgcccttgg 600
gccccagggc cggcggctgg aggaggtgcg gctcaagccc atccttgcca gtgccaagca 660

gcatagccca gtgaccgagg gagccgtgga ggtgaagtat gagggccact ggcggcaggt 720
gtgtgaccag ggctggacca tgaacaacag cagggtggtg tgcgggatgc tgggcttccc 780
cagcgaggtg cctgtcgaca gccactacta caggaaagtc tgggatctga agatgaggga 840
ccctaagtct aggctgaaga gcctgacgaa taagaactcc ttctggatcc accaggtcac 900
ctgcctgggg acagagcccc acatggccaa ctgccaggtg caggtggctc cagcccgggg 960
caagctgcgg ccagcctgcc caggtggcat gcatgctgtg gtcagctgtg tggcagggcc 1020
tcacttccgc ccaccgaaga caaagccaca acgcaaaggg tcctgggcag aggagccgag 1080
ggtgcgcctg cgctccgggg cccaggtggg cgagggccgg gtggaagtgc tcatgaaccg 1140
ccagtggggc acggtctgtg accacaggtg gaacctcatc tctgccagtg tcgtgtgtcg 1200
tcagctgggc tttggctctg ctcgggaggc cctctttggg gcccggctgg gccaagggct 1260
agggcccatc cacctgagtg aggtgcgctg caggggatat gagcggaccc tcagcgactg 1320
ccctgccctg gaagggtccc agaatggttg ccaacatgag aatgctgctg ctgtcaggtg 1380
caatgtccct aacatgggct ttcagaatca ggtgcgcttg gctggtgggc gtatccctga 1440
ggaggggcta ttggaggtgc aggtggaggt gaacggggtc ccacgctggg ggagcgtgtg 1500
cagtgaaaac tgggggctca ccgaagccat ggtggcctgc cgacagctcg gcctgggttt 1560
tgccatccat gcctacaagg aaacctggtt ctggtcgggg acgccaaggg cccaggaggt 1620
ggtgatgagt ggggtgcgct gctcaggcac agagctggcc ctgcagcagt gccagaggca 1680
cgggccggtg cactgctccc acggtggcgg gcgcttcctg gctggagtct cctgcatgga 1740
cagtgcacca gacctggtga tgaacgccca gctagtgcag gagacggcct acttggagga 1800
ccgcccgctc agccagctgt attgtgccca cgaggagaac tgcctctcca agtctgcgga 1860
tcacatggac tggccctacg gataccgccg cctattgcgc ttctccacac agatctacaa 1920
tctgggccgg actgactttc gtccaaagac tggacgcgat agctgggttt ggcaccagtg 1980
ccacaggcat taccacagca ttgaggtctt cacccactac gacctcctca ctctcaatgg 2040
ctccaaggtg gctgaggggc acaaggccag cttctgtctg gaggacacaa actgccccac 2100
aggactgcag cggcgctacg catgtgccaa ctttggagaa cagggagtga ctgtaggctg 2160
ctgggacacc taccggcatg acattgattg ccagtgggtg gatatcacag atgtgggccc 2220
cgggaattat atcttccagg tgattgtgaa cccccactat gaagtggcag agtcagattt 2280
ctccaacaat atgctgcagt gccgctgcaa gtatgatggg caccgggtct ggctgcacaa 2340
ctgccacaca gggaattcat acccagccaa tgcagaactc tccctggagc aggaacagcg 2400
tctcaggaac aacctcatct gaagctgtca ctgcacactc ctagctgctg ccgatacacc 2460
agatacctca gcttattgga gccatgccct tcacagagtc ccaactcaga ggaaaagggc 2520
cagtgccaag gggcaccaag aacctgctca ggaagccttt tgatggcaag atcaccaatc 2580
cagatggtat tgctccctca ggatggctct gggcctgccc ctaagggcct gtggcctatg 2640
gaatatgtcc tccaggcttt gctcagctga gctcctcttc tgtaaggaaa cccagtcatc 2700
cctgaatctt gccacagaga tccgggattc aggagctctc agtttcttag ggatggacta 2760
tggcccagtc ccccatctaa gtggtgcttt gcaaatgtct tggaggagta taggacagag 2820
gaccaaaata cacagcaggt agtgttagct ctctgctagg agctcaaagc aacacaactt 2880
gtatcaaaat cacaactggc agagaagctg gtggatccaa tcctttcttc atctgttgtt 2940
atttagaact cacctctcac actctgttct ttagtgtcct tacctttatc ttaccacaca 3000
catgggtgtt tctattatcc ttggaagcac agacctcggg catcccctta ttgcctgatg 3060
ggccaacacc aacagttacg gagtgcttga gaaggggcaa gtttcacaga aatggccaga 3120
tagggccttc ctacagagca gcaagagtag gccaagcaga aagactgctg aggtaacacg 3180
gaccccagcc cctgtcaggg cctctgccaa ggaaataata tggaccattt acctggcagg 3240
cagtctgctc tctctcagga tcaccacgca tctcaggatt ggtctaaact tcaagtctca 3300
accaagtgtc tgaagtgaac tttgcattga ataaattttt gccatggaaa gaacatcaaa 3360
caagccactc atctctacag agataagaaa acaagtttgg cagagcaaga gacagaagac 3420
cgtggagaaa tcagaagggg gaacagtcag tttagttaag gatggaacct gggaaaggcc 3480
accattcctg cttgatgggg ctctgatttg ctcttgctca agtggaataa aaccccatgg 3540
tcttcttgac atgattcttg atcttttctc cactgagaca cacttaagtg atgatcctta 3600
caggactgac accctaatgc caataaaagt tgctcattat ggactgctac aaaaaaaaaa 3660
aaaaa 3665

<210> 175
<211> 2328
<212> DNA
<213> Homo sapiens

<400> 175
ggaagcaggg cggggcctct ggtggcggtc gggaactcgg tgggaggcgg caacattgtt 60
tcaagttggc caaattgaca agagcgagag gtatactgcg ttccatcccg acccggggcc 120
acggtactgg gccctgtttc cccctcctcg gcccccgaga gccagggtcc gccttctgca 180
gggttcccag gcccccgctc cagggccggg ctgacccgac tcgctggcgc ttcatggaga 240
acttccaaaa ggtggaaaag atcggagagg gcacgtacgg agttgtgtac aaagccagaa 300

acaagttgac gggagaggtg gtggcgctta agaaaatccg cctggacact gagactgagg 360
gtgtgcccag tactgccatc cgagagatct ctctgcttaa ggagcttaac catcctaata 420
ttgtcaagct gctggatgtc attcacacag aaaataaact ctacctggtt tttgaatttc 480
tgcaccaaga tctcaagaaa ttcatggatg cctctgctct cactggcatt cctcttcccc 540
tcatcaagag ctatctgttc cagctgctcc agggcctagc tttctgccat tctcatcggg 600
tcctccaccg agaccttaaa cctcagaatc tgcttattaa cacagagggg gccatcaagc 660
tagcagactt tggactagcc agagcttttg gagtccctgt tcgtacttac acccatgagg 720
tggtgaccct gtggtaccga gctcctgaaa tcctcctggg ctgcaaatat tattccacag 780
ctgtggacat ctggagcctg ggctgcatct ttgctgagat ggtgactcgc cgggccctat 840
tccctggaga ttctgagatt gaccagctct tccggatctt tcggactctg gggaccccag 900
atgaggtggt gtggccagga gttacttcta tgcctgatta caagccaagt ttccccaagt 960
gggcccggca agattttagt aaagttgtac ctcccctgga tgaagatgga cggagcttgt 1020
tatcgcaaat gctgcactac gaccctaaca agcggatttc ggccaaggca gccctggctc 1080
accctttctt ccaggatgtg accaagccag taccccatct tcgactctga tagccttctt 1140
gaagccccca gccctaatct caccctctcc tccagtgtgg gcttgaccag gcttggcctt 1200
gggctatttg gactcaggtg ggccctctga acttgcctta aacactcacc ttctagtctt 1260
ggccagccaa ctctgggaat acaggggtga aaggggggaa ccagtgaaaa tgaaaggaag 1320
tttcagtatt agatgcactt aagttagcct ccaccaccct ttcccccttc tcttagttat 1380
tgctgaagag ggttggtata aaaataattt taaaaaagcc ttcctacacg ttagatttgc 1440
cgtaccaatc tctgaatgcc ccataattat tatttccagt gtttgggatg accaggatcc 1500
caagcctcct gctgccacaa tgtttataaa ggccaaatga tagcgggggc taagttggtg 1560
cttttgagaa ccaagtaaaa caaaaccact gggaggagtc tattttaaag aattcggttg 1620
aaaaaataga tccaatcagt ttatacccta gttagtgttt tgcctcacct aataggctgg 1680
gagactgaag actcagcccg ggtggggctg cagaaaaatg attggcccca gtccccttgt 1740
ttgtcccttc tacaggcatg aggaatctgg gaggccctga gacagggatt gtgcttcatt 1800
ccaatctatt gcttcaccat ggccttatga ggcaggtgag agatgtttga atttttctct 1860
tccttttagt attcttagtt gttcagttgc caaggatccc tgatcccatt ttcctctgac 1920
gtccacctcc taccccatag gagttagaag ttagggttta ggcatcattt tgagaatgct 1980
gacacttttt cagggctgtg attgagtgag ggcatgggta aaaatatttc tttaaaagaa 2040
ggatgaacaa ttatatttat atttcaggtt atatccaata gtagagttgg cttttttttt 2100
tttttttttg gtcatagtgg gtggatttgt tgccatgtgc accttggggt tttgtaatga 2160
cagtgctaaa aaaaaaaaag catttttttt tatgatttgt ctctgtcacc cttgtccttg 2220
agtgctcttg ctattaacgt tatttgtaat ttagtttgta gctcattaaa aaaatgtgcc 2280
tagttttata aaaaaaaaaa aaaaaaaaaa aaaaaaaaaa aaaaaaaa 2328

<210> 176
<211> 2226
<212> DNA
<213> Homo sapiens

<400> 176
ggaagcaggg cggggcctct ggtggcggtc gggaactcgg tgggaggcgg caacattgtt 60
tcaagttggc caaattgaca agagcgagag gtatactgcg ttccatcccg acccggggcc 120
acggtactgg gccctgtttc cccctcctcg gcccccgaga gccagggtcc gccttctgca 180
gggttcccag gcccccgctc cagggccggg ctgacccgac tcgctggcgc ttcatggaga 240
acttccaaaa ggtggaaaag atcggagagg gcacgtacgg agttgtgtac aaagccagaa 300
acaagttgac gggagaggtg gtggcgctta agaaaatccg cctggacact gagactgagg 360
gtgtgcccag tactgccatc cgagagatct ctctgcttaa ggagcttaac catcctaata 420
ttgtcaagct gctggatgtc attcacacag aaaataaact ctacctggtt tttgaatttc 480
tgcaccaaga tctcaagaaa ttcatggatg cctctgctct cactggcatt cctcttcccc 540
tcatcaagag ctatctgttc cagctgctcc agggcctagc tttctgccat tctcatcggg 600
tcctccaccg agaccttaaa cctcagaatc tgcttattaa cacagagggg gccatcaagc 660
tagcagactt tggactagcc agagcttttg gagtccctgt tcgtacttac acccatgagg 720
tgactcgccg ggccctattc cctggagatt ctgagattga ccagctcttc cggatctttc 780
ggactctggg gaccccagat gaggtggtgt ggccaggagt tacttctatg cctgattaca 840
agccaagttt ccccaagtgg gcccggcaag attttagtaa agttgtacct cccctggatg 900
aagatggacg gagcttgtta tcgcaaatgc tgcactacga cccttacaag aggatttcgg 960
ccaaggcagc cctggctcac cctttcttcc aggatgtgac caagccagta ccccatcttc 1020
gactctgata gccttcttga agcccccagc cctaatctca ccctctcctc cagtgtgggc 1080
ttgaccaggc ttggccttgg gctatttgga ctcaggtggg ccctctgaac ttgccttaaa 1140
cactcacctt ctagtcttgg ccagccaact ctgggaatac aggggtgaaa ggggggaacc 1200
agtgaaaatg aaaggaagtt tcagtattag atgcacttaa gttagcctcc accacccttt 1260
cccccttctc ttagttattg ctgaagaggg ttggtataaa aataatttta aaaaagcctt 1320

cctacacgtt agatttgccg taccaatctc tgaatgcccc ataattatta tttccagtgt 1380
ttgggatgac caggatccca agcctcctgc tgccacaatg tttataaagg ccaaatgata 1440
gcgggggcta agttggtgct tttgagaacc aagtaaaaca aaaccactgg gaggagtcta 1500
ttttaaagaa ttcggttgaa aaaatagatc caatcagttt ataccctagt tagtgttttg 1560
cctcacctaa taggctggga gactgaagac tcagcccggg tggggctgca gaaaaatgat 1620
tggccccagt ccccttgttt gtcccttcta caggcatgag gaatctggga ggccctgaga 1680
cagggattgt gcttcattcc aatctattgc ttcaccatgg ccttatgagg caggtgagag 1740
atgtttgaat ttttctcttc cttttagtat tcttagttgt tcagttgcca aggatccctg 1800
atcccatttt cctctgacgt ccacctccta ccccatagga gttagaagtt agggtttagg 1860
catcattttg agaatgctga cactttttca gggctgtgat tgagtgaggg catgggtaaa 1920
aatatttctt taaaagaagg atgaacaatt atatttatat ttcaggttat atccaatagt 1980
agagttggct tttttttttt tttttttggt catagtgggt ggatttgttg ccatgtgcac 2040
cttggggttt tgtaatgaca gtgctaaaaa aaaaaaagca ttttttttta tgatttgtct 2100
ctgtcaccct tgtccttgag tgctcttgct attaacgtta tttgtaattt agtttgtagc 2160
tcattaaaaa aatgtgccta gttttataaa aaaaaaaaaa aaaaaaaaaa aaaaaaaaaa 2220
aaaaaa 2226

<210> 177
<211> 2181
<212> DNA
<213> Homo sapiens

<400> 177
ggcacgaggg atgcaaggag atgagacagt tagatttact tcctcttttc taatctgaga 60
ggtttcatgt tgaagaaaat cagtgttggg gttgcaggag acctaaacac agtcaccatg 120
aagctgggct gtgtcctcat ggcctgggcc ctctaccttt cccttggtgt gctctgggtg 180
gcccagatgc tactggctgc cagttttgag acgctgcagt gtgagggacc tgtctgcact 240
gaggagagca gctgccacac ggaggatgac ttgactgatg caagggaagc tggcttccag 300
gtcaaggcct acactttcag tgaacccttc cacctgattg tgtcctatga ctggctgatc 360
ctccaaggtc cagccaagcc agtttttgaa ggggacctgc tggttctgcg ctgccaggcc 420
tggcaagact ggccactgac tcaggtgacc ttctaccgag atggctcagc tctgggtccc 480
cccgggccta acagggaatt ctccatcacc gtggtacaaa aggcagacag cgggcactac 540
cactgcagtg gcatcttcca gagccctggt cctgggatcc cagaaacagc atctgttgtg 600
gctatcacag tccaagaact gtttccagcg ccaattctca gagctgtacc ctcagctgaa 660
ccccaagcag gaagccccat gaccctgagt tgtcagacaa agttgcccct gcagaggtca 720
gctgcccgcc tcctcttctc cttctacaag gatggaagga tagtgcaaag cagggggctc 780
tcctcagaat tccagatccc cacagcttca gaagatcact ccgggtcata ctggtgtgag 840
gcagccactg aggacaacca agtttggaaa cagagccccc agctagagat cagagtgcag 900
ggtgcttcca gctctgctgc acctcccaca ttgaatccag ctcctcagaa atcagctgct 960
ccaggaactg ctcctgagga ggcccctggg cctctgcctc cgccgccaac cccatcttct 1020
gaggatccag gcttttcttc tcctctgggg atgccagatc ctcatctgta tcaccagatg 1080
ggccttcttc tcaaacacat gcaggatgtg agagtcctcc tcggtcacct gctcatggag 1140
ttgagggaat tatctggcca ccagaagcct gggaccacaa aggctactgc tgaatagaag 1200
taaacagttc atccatgatc tcacttaacc accccaataa atctgattct ttattttctc 1260
ttcctgtcct gcacatatgc ataagtactt ttacaagttg tcccagtgtt ttgttagaat 1320
aatgtagtta ggtgagtgta aataaattta tataaagtga gaattagagt ttagctataa 1380
ttgtgtattc tctcttaaca caacagaatt ctgctgtcta gatcaggaat ttctatctgt 1440
tatatcgacc agaatgttgt gatttaaaga gaactaatgg aagtggattg aatacagcag 1500
tctcaactgg gggcaatttt gccccccaga ggacattggg caatgtttgg agacattttg 1560
gtcattatac ttggggggtt gggggatggt gggatgtgtg tgctactggc atccagtaaa 1620
tagaagccag gggtgccgct aaacatccta taatgcacag ggcagtaccc cacaacgaaa 1680
aataatctgg cccaaaatgt cagttgtact gagtttgaga aaccccagcc taatgaaacc 1740
ctaggtgttg ggctctggaa tgggactttg tcccttctaa ttattatctc tttccagcct 1800
cattcagcta ttcttactga cataccagtc tttagctggt gctatggtct gttctttagt 1860
tctagtttgt atcccctcaa aagccattat gttgaaatcc taatccccaa ggtgatggca 1920
ttaagaagtg ggcctttggg aagtgattag atcaggagtg cagagccctc atgattagga 1980
ttagtgccct tatttaaaaa ggccccagag agctaactca cccttccacc atatgaggac 2040
gtggcaagaa gatgacatgt atgagaacca aaaaacagct gtcgccaaac accgactctg 2100
tcgttgcctt gatcttgaac ttccagcctc cagaactatg agaaataaaa ttctgttgtt 2160
tgtaaaaaaa aaaaaaaaaa a 2181

<210> 178
<211> 1145

<212> DNA
<213> Homo sapiens

<400> 178
gctccggcca gccgcggtcc agagcgcgcg aggttcgggg agctccgcca ggctgctggt 60
acctgcgtcc gcccggcgag caggacaggc tgctttggtt tgtgacctcc aggcaggacg 120
gccatcctct ccagaatgaa gatcttcttg ccagtgctgc tggctgccct tctgggtgtg 180
gagcgagcca gctcgctgat gtgcttctcc tgcttgaacc agaagagcaa tctgtactgc 240
ctgaagccga ccatctgctc cgaccaggac aactactgcg tgactgtgtc tgctagtgcc 300
ggcattggga atctcgtgac atttggccac agcctgagca agacctgttc cccggcctgc 360
cccatcccag aaggcgtcaa tgttggtgtg gcttccatgg gcatcagctg ctgccagagc 420
tttctgtgca atttcagtgc ggccgatggc gggctgcggg caagcgtcac cctgctgggt 480
gccgggctgc tgctgagcct gctgccggcc ctgctgcggt ttggcccctg accgcccaga 540
ccctgtcccc cgatccccca gctcaggaag gaaagcccag ccctttctgg atcccacagt 600
gtatgggagc ccctgactcc tcacgtgcct gatctgtgcc cttggtccca ggtcaggccc 660
accccctgca cctccacctg ccccagcccc tgcctctgcc caagtgggcc agctgccctc 720
acttctgggg tggatgatgt gaccttcctt gggggactgc ggaagggacg agggttccct 780
ggagtcttac ggtccaacat cagaccaagt cccatggaca tgctgacagg gtccccaggg 840
agaccgtgtc agtagggatg tgtgcctggc tgtgtacgtg ggtgtgcagt gcacgtgaga 900
gcacgtggcg gcttctgggg gccatgtttg gggagggagg tgtgccagca gcctggagag 960
cctcagtccc tgtagccccc tgccctggca cagctgcatg cacttcaagg gcagcctttg 1020
ggggttgggg tttctgccac ttccgggtct aggccctgcc caaatccagc cagtcctgcc 1080
ccagcccacc cccacattgg agccctcctg ctgctttggt gcctcaaata aatacagatg 1140
tcccc 1145

<210> 179
<211> 2683
<212> DNA
<213> Homo sapiens

<400> 179
tgggcgccgg cggagcggag ggagatccga gcggcggcgg caagctggct gcgagcggct 60
gagcagcact ccaagctcgc cggcctttgg tctccaggac ttgtcccagc agcccctcga 120
actgagaatt acaccatcgg acccctggct ctgaggcctt cagacttgga ctgtgtcaca 180
ctgccaggct tccagggctc caacttgcag acggcctgtt gtgggacagt ctctgtaatc 240
gcgaaagcaa ccatggaaga cctgggggaa aacaccatgg ttttatccac cctgagatct 300
ttgaacaact tcatctctca gcgtgtggag ggaggctctg gactggatat ttctacctcg 360
gccccaggtt ctctgcagat gcagtaccag cagagcatgc agctggagga aagagcagag 420
cagatccgtt cgaagtccca cctcatccag gtggagcggg agaaaatgca gatggagctg 480
agtcacaaga gggctcgagt ggagctggag agagcagcca gcaccagtgc caggaactac 540
gagcgtgagg tcgaccgcaa ccaggagctc ctgacgcgca tccggcagct tcaggagcgg 600
gaggccgggg cggaggagaa gatgcaggag cagctggagc gcaacaggca gtgtcagcag 660
aacttggatg ctgccagcaa gaggctgcgt gagaaagagg acagtctggc ccaggctggc 720
gagaccatca acgcactgaa ggggaggatc tcggaactgc agtggagcgt gatggaccag 780
gagatgcggg tgaagcgcct ggagtcggag aagcaggagc tgcaggagca gctggacctg 840
caacacaaaa aatgccagga agccaatcag aaaatccagg aactccaggc cagccaagaa 900
gcaagagcag accacgagca gcagattaag gatctggagc agaagctgtc cctgcaagag 960
caggatgcag cgattgtgaa gaacatgaag tctgagctgg tacggctccc taggctggaa 1020
cgggagctga agcagctgcg ggaggagagc gcgcacctgc gggagatgag agagaccaac 1080
gggctgctcc aggaagagct ggaagggctg cagaggaagc tggggcgcca ggagaagatg 1140
caggagacgc tggttggctt ggagctggag aacgagaggc tgctggccaa gctgcaaagc 1200
tgggagagac tggaccagac catgggcctg agcatcagga ctccagaaga cctttccaga 1260
ttcgtggttg agctgcagca gagggagctt gccttgaagg acaagaacag cgccgtcacc 1320
agcagcgccc gggggctgga gaaggccagg cagcagctgc aggaggagct ccggcaggtc 1380
agcggccagc tgttggagga gaggaagaag cgcgagaccc acgaggcgct ggcccggagg 1440
ctccagaaac gggtcctgct gctcaccaag gagcgggacg gtatgcgggc catcctgggg 1500
tcctacgaca gcgagctgac cccggccgag tactcacccc agctgacgcg gcgcatgcgg 1560
gaggctgagg atatggtgca gaaggtgcac agccacagcg ccgagatgga ggctcagctg 1620
tcgcaggccc tggaggagct gggaggccag aaacaaagag cagacatgct ggagatggag 1680
ctgaagatgc tgaagtctca gtccagctct gccgaacaga gcttcctgtt ctccagggag 1740
gaggcggaca cgctcaggtt gaaggtcgag gagctggaag gcgagcggag tcggctggag 1800
gaggaaaaga ggatgctgga ggcacagctg gagcggcgag ctctgcaggg tgactatgac 1860

cagagcagga ccaaagtgct gcacatgagc ctgaacccca ccagtgtggc caggcagcgc 1920
ctgcgcgagg accacagcca gctgcaggcg gagtgcgagc gactgcgcgg gctcctgcgc 1980
gccatggaga gaggaggcac cgtcccagcc gaccttgagg ctgccgccgc gagtctgcca 2040
tcgtccaagg aggtggcaga gctgaagaag caggtggaga gtgccgagct gaagaaccag 2100
cggctcaagg aggttttcca gaccaagatc caggagttcc gcaaggcctg ctacacgctc 2160
accggctacc agatcgacat caccacggag aaccagtacc ggctgacctc gctgtacgcc 2220
gagcacccag gcgactgcct catcttcaag gccaccagcc cctcgggttc caagatgcag 2280
ctactggaga cagagttctc acacaccgtg ggcgagctca tcgaggtgca cctgcggcgc 2340
caggacagca tccctgcctt cctcagctcg ctcaccctcg agctcttcag ccgccagacc 2400
gtggcgtagc ctgcaggctc gggggcatag ccggagccac tctgcttggc ctgacctgca 2460
ggtcccctgc cccgccagcc acaggctggg tgcacgtcct gcctctccag ccccacaggg 2520
cagcagcatg actgacagac acgctgggac ctacgtcggg cttcctgctg gggcggccag 2580
caccctctcc acgtgcagac cccatgcgtc ccggagcctg gtgtgtgggc gtcggccacc 2640
agcctgggtt cctcaccttg tgaaataaaa tcttctcccc tag 2683

<210> 180
<211> 1607
<212> DNA
<213> Homo sapiens

<400> 180
atgacccagg caggccggcg gggtcctggc acacccgagc cgcgtccgcg aacacagccc 60
atggcctccc cgcgcctagg gaccttctgc tgccccacgc gggacgcagc cacgcagctc 120
gtgctgagct tccagccgcg ggccttccac gcgctctgcc tgggcagcgg cgggctccgc 180
ttggcgctgg gccttctgca gctgctgccc ggccgccggc ccgcgggccc cgggtccccc 240
gcgacgtccc cgccggcctc ggtccgcatc ctgcgcgctg ccgctgcctg cgaccttctc 300
ggctgcctgg gtatggtgat ccggtccacc gtgtggttag gattcccaaa ttttgttgac 360
agcgtctcgg atatgaacca cacggaaatt tggcctgctg ctttctgcgt ggggagtgcg 420
atgtggatcc agctgttgta cagtgcctgc ttctggtggc tgttttgcta tgcagtggat 480
gcttatctgg tgatccggag atcggcagga ctgagcacca tcctgctgta tcacatcatg 540
gcgtggggcc tggccaccct gctctgtgtg gagggagccg ccatgctcta ctacccttcc 600
gtgtccaggt gtgagcgggg cctggaccac gccatccccc actatgtcac catgtacctg 660
cccctgctgc tggttctcgt ggcgaacccc atcctgttcc aaaagacagt gactgcagtg 720
gcctctttac ttaaaggaag acaaggcatt tacacggaga acgagaggag gatgggagcc 780
gtgatcaaga tccgattttt caaaatcatg ctggttttaa ttatttgttg gttgtcgaat 840
atcatcaatg aaagcctttt attctatctt gagatgcaaa cagatatcaa tggaggttct 900
ttgaaacctg tcagaactgc agccaagacc acatggttta ttatgggaat cctgaatcca 960
gcccagggat ttctcttgtc tttggccttc tacggctgga caggatgcag cctgggtttt 1020
cagtctccca ggaaggagat ccagtgggaa tcactgacca cctcggctgc tgagggggct 1080
cacccatccc cactgatgcc ccatgaaaac cctgcttccg ggaaggtgtc tcaagtgggt 1140
gggcagactt ctgacgaagc cctgagcatg ctgtctgaag gttctgatgc cagcacaatt 1200
gaaattcaca ctgcaagtga atcctgcaac aaaaatgagg gtgaccctgc tctcccaacc 1260
catggagacc tatgaagggg atgtgctggg ggtccagacc ccatattcct cagactcaac 1320
aattcttgtt ctttagaact gtgttctcac cttcccaaca ctgcactgcc gaagtgtagc 1380
ggcccccaaa ccttgctctc atcaccagct agagcttctt cccgaagggc ctttaggata 1440
ggagaaaggg ttcatgcaca cacgtgtgag aatggaagag ccccctccag accactctac 1500
agctgctcta gccttagttg ccactaggaa gttttctgag gctggctgta aagtaagtgt 1560
aaggtccaca tccttgggga agtagttaaa taaaatagtt atgactg 1607

<210> 181
<211> 1322
<212> DNA
<213> Homo sapiens

<400> 181
ccctgggata ctcccctccc agggtgtctg gtggcaggcc tgtgcctatc cctgctgtcc 60
ccagggtggg ccccgggggt caggagctcc agaagggcca gctgggcata ttctgagatt 120
ggccatcagc ccccatttct gctgcaaacc tggtcagagc cagtgttccc tccatgggac 180
ctaaagacag tgccaagtgc ctgcaccgtg gaccacagcc gagccactgg gcagccggtg 240
atggtcccac gcaggagcgc tgtggacccc gctctctggg cagccctgtc ctaggcctgg 300
acacctgcag agcctgggac cacgtggatg ggcagatcct gggccagctg cggcccctga 360
cagaggagga agaggaggag ggcgccgggg ccaccttgtc cagggggcct gccttccccg 420
gcatgggctc tgaggagttg cgtctggcct ccttctatga ctggccgctg actgctgagg 480

tgccacccga gctgctggct gctgccggct tcttccacac aggccatcag gacaaggtga 540
ggtgcttctt ctgctatggg ggcctgcaga gctggaagcg cggggacgac ccctggacgg 600
agcatgccaa gtggttcccc agctgtcagt tcctgctccg gtcaaaagga agagactttg 660
tccacagtgt gcaggagact cactcccagc tgctgggctc ctgggacccg tgggaagaac 720
cggaagacgc agcccctgtg gccccctccg tccctgcctc tgggtaccct gagctgccca 780
cacccaggag agaggtccag tctgaaagtg cccaggagcc aggaggggtc agtccagccg 840
aggcccagag ggcgtggtgg gttcttgagc ccccaggagc cagggatgtg gaggcgcagc 900
tgcggcggct gcaggaggag aggacgtgca aggtgtgcct ggaccgcgcc gtgtccatcg 960
tctttgtgcc gtgcggccac ctggtctgtg ctgagtgtgc ccccggcctg cagctgtgcc 1020
ccatctgcag agcccccgtc cgcagccgcg tgcgcacctt cctgtcctag gccaggtgcc 1080
atggccggcc aggtgggctg cagagtgggc tccctgcccc tctctgcctg ttctggactg 1140
tgttctgggc ctgctgagga tggcagagct ggtgtccatc cagcactgac cagccctgat 1200
tccccgacca ccgcccaggg tggagaagga ggcccttgct tggcgtgggg gatggcttaa 1260
ctgtacctgt ttggatgctt ctgaatagaa ataaagtggg ttttccctgg aggtacccag 1320
ca 1322

<210> 182
<211> 1268
<212> DNA
<213> Homo sapiens

<400> 182
ccctgggata ctcccctccc agggtgtctg gtggcaggcc tgtgcctatc cctgctgtcc 60
ccagggtggg ccccgggggt caggagctcc agaagggcca gctgggcata ttctgagatt 120
ggccatcagc ccccatttct gctgcaaacc tggtcagagc cagtgttccc tccatgggac 180
ctaaagacag tgccaagtgc ctgcaccgtg gaccacagcc gagccactgg gcagccggtg 240
atggtcccac gcaggagcgc tgtggacccc gctctctggg cagccctgtc ctaggcctgg 300
acacctgcag agcctgggac cacgtggatg ggcagatcct gggccagctg cggcccctga 360
cagaggagga agaggaggag ggcgccgggg ccaccttgtc cagggggcct gccttccccg 420
gcatgggctc tgaggagttg cgtctggcct ccttctatga ctggccgctg actgctgagg 480
tgccacccga gctgctggct gctgccggct tcttccacac aggccatcag gacaaggtga 540
ggtgcttctt ctgctatggg ggcctgcaga gctggaagcg cggggacgac ccctggacgg 600
agcatgccaa gtggttcccc agctgtcagt tcctgctccg gtcaaaagga agagactttg 660
tccacagtgt gcaggagact cactcccagc tgctgggctc ctgggacccg tgggaagaac 720
cggaagacgc agcccctgtg gccccctccg tccctgcctc tgggtaccct gagctgccca 780
cacccaggag agaggtccag tctgaaagtg cccaggagcc aggagccagg gatgtggagg 840
cgcagctgcg gcggctgcag gaggagagga cgtgcaaggt gtgcctggac cgcgccgtgt 900
ccatcgtctt tgtgccgtgc ggccacctgg tctgtgctga gtgtgccccc ggcctgcagc 960
tgtgccccat ctgcagagcc cccgtccgca gccgcgtgcg caccttcctg tcctaggcca 1020
ggtgccatgg ccggccaggt gggctgcaga gtgggctccc tgcccctctc tgcctgttct 1080
ggactgtgtt ctgggcctgc tgaggatggc agagctggtg tccatccagc actgaccagc 1140
cctgattccc cgaccaccgc ccagggtgga gaaggaggcc cttgcttggc gtgggggatg 1200
gcttaactgt acctgtttgg atgcttctga atagaaataa agtgggtttt ccctggaggt 1260
acccagca 1268

<210> 183
<211> 5492
<212> DNA
<213> Homo sapiens

<400> 183
tgagggcttt gctatgacct cagtcccctc acggagccac gactgcccct tgctgccaca 60
gcctttccaa gaccctgccc ggccctgccc catcctcagc cccgagtcac catgggcagc 120
gtcagtagcc tcatctccgg ccacagcttc cacagcaagc actgccgggc ttcgcagtac 180
aagctgcgca agtcctccca cctcaagaag ctcaaccggt attccgacgg gctgctgagg 240
tttggcttct cccaggactc cggtcacggc aagtccagct ccaaaatggg caagagcgaa 300
gacttcttct acatcaaggt cagccagaaa gcccggggct cccatcaccc agattacacg 360
gcactgtcca gcggggattt agggggccag gctggggtgg actttgaccc gtccacaccc 420
cccaagctca tgcccttctc caatcagcta gaaatgggct ccgagaaggg tgcagtgagg 480
cccacagcct tcaagcctgt gctgccacgg tcaggagcca tcctgcactc ctccccggag 540
agtgccagcc accagctgca ccccgcccct ccagacaagc ccaaggagca ggagctgaag 600
cctggcctgt gctctggggc gctgtcagac tccggccgga actccatgtc cagcctgccc 660

acacacagca ccagcagcag ctaccagctg gacccgctgg tcacacccgt gggacccaca 720
agccgttttg ggggctccgc ccacaacatc acccagggca tcgtcctcca ggacagcaac 780
atgatgagcc tgaaggctct gtccttctcc gacggaggta gcaagctggg ccactcgaac 840
aaggcagaca agggcccctc gtgtgtccgc tcccccatct ccacggacga gtgcagcatc 900
caggagctgg agcagaagct gttggagagg gagggcgccc tccagaagct gcagcgcagc 960
tttgaggaga aggagcttgc ctccagcctg gcctacgagg agcggccgcg gcgctgcagg 1020
gacgagctgg agggcccgga gcccaaaggc ggcaacaagc tcaagcaggc ctcgcagaag 1080
agccagcgcg cgcagcaggt cctgcacctg caggtactgc agcttcagca ggagaagcgg 1140
cagctccggc aggagctcga gagcctcatg aaggagcagg acctgctgga gaccaagctc 1200
aggtcctacg agagggagaa gaccagcttc ggccccgcgc tggaggagac ccagtgggag 1260
gtgtgccaga agtcaggcga gatctccctc ctgaagcagc agctgaagga gtcccagacg 1320
gaggtgaacg ccaaggctag cgagatcctg ggtctcaagg cacagctgaa ggacacgcgg 1380
ggcaagctgg agggcctgga gctgaggacc caggacctgg agggcgccct gcgcaccaag 1440
ggcctggagc tggaggtctg tgagaatgag ctgcagcgca agaagaacga ggcggagctg 1500
ctgcgggaga aggtgaacct gctggagcag gagctgcagg agctgcgggc ccaggccgcc 1560
ctggcccgcg acatgggccc gcccaccttc cccgaggacg tccctgccct gcagcgggag 1620
ctggagcggc tgcgggccga gctgcgggag gagcggcaag gccatgacca gatgtcctcg 1680
ggcttccagc atgagcggct cgtgtggaag gaggagaagg agaaggtgat tcagtaccag 1740
aaacagctgc agcagagcta cgtggccatg taccagcgga accagcgcct ggagaaggcc 1800
ctgcagcagc tggcacgtgg ggacagcgcc ggggagccct tggaggttga cctggaaggg 1860
gctgacatcc cctacgagga catcatagcc actgagatct gaggggctgc ctgggaaggc 1920
gagtctgggg acctggcact gggaggcagg gctctcccgt gcatcccccc tgctcagcaa 1980
ttcagacccc tctgagagac gccactccct gggacacaga cccaggaccc ccgaggggag 2040
ggcaggatgg cctttccttc cctctctgat gtcccagtgc tcaccagccc tgcagcccac 2100
cagacgtcag gccctgactc ctctggcttt cccaggagat gggtccaggg gtctgtctgc 2160
tttggttaag ggctccctaa actttggcct ttgttcgaaa tagatatcct ctccccctcc 2220
tccagggaag gtggccacag caagaacagc ggctcccctc cgcttctcat cccaacctct 2280
ttttcctcct ggacacattg gaatgccttg gaaatagaaa gaagccatat atgaccagaa 2340
gccttggaac cagccccatc agaacctgag ctattttcct ctggccgcag aggtgtaggg 2400
gtggaatgag ccgcggggaa gctggctttg aaacctcagg gctgtcccag ccccggcaag 2460
ccacaggaag gaggggagag acaggcagcc cagcagtgtg gagaccctgc cacagccaga 2520
ggagggcaga gggagaatcc aagggttgag agccagtggc gggtgatggc cagcccctgg 2580
ggcccagccc ctgtttactg gttcttgcaa atgggagctg agcagcctct ggacagccag 2640
tgacctttga cctcggtgac cactcttctt taagccatag accctgaggc cctgggctgg 2700
gtgctgggaa gggagggttg aaaccaccgt gaaccagagg gtgtggcttt ccaggcaccc 2760
tcagggagcc tccccatctg tccagctggg gccagaggct gggagtccct acctgcttca 2820
cgttggccgg cggctactct ggaatgtttt tccctcccca gaatcaagct tttgcttgat 2880
ccagaagagc ccatatcact aagatggcat atatgtgatc tgggcatttt cctcctctgc 2940
ctacagccag gtttagcggc aaacctttcc cccttagcac cttcagggct gagttctggg 3000
tttctagagg tcaggacggc tcctcagagc gccaggaagc cagagcccca agcaggacga 3060
aaaagaggca tacacacagc agtgtgaata gcctggccac cagccatcct ccctccacct 3120
caagaccccc atttgtccca gactaaagga tccagagagc agctcccttt ctcaggagct 3180
tgggcagtgc cccagggagt ccagggtttc tctgcagatg tgcggagcgg gaggcggtgg 3240
tagagagaga taaaaggtgg agtttctctg ttgtttggtt cagggatttt atttttaatt 3300
ttatgagaca gggtcttgct ctgtccccca ggctggagtg cagtggcatg atcatagctc 3360
actgcagcct catactcctg ggctcaagca atcctcctgc ctcagccttc caactagctg 3420
ggactacagg tgcgcgccac cgtgcctggc taacttttca ttttttttgt agggacgggg 3480
tctcgttttg ttgccaaagc tggtctcaaa cttgtggcct caagcaatcc acctgccttg 3540
gcctcccaaa gtgctgagat tgcagatgtg agccaccgtg cctggccaga tttttctttt 3600
attcttcttt ctttttcttt tttgctttct tgtcttttca gaagcaagcc agacctagca 3660
ggctgttcca tgttctattt ttgactgtag ccacagctgc tgttctcagg acagcatccc 3720
ttcccacatg cctgcgcctg ctgcctgctg agatgaggag gggagcgtct gggaacttgc 3780
gagtccaagg ccagtcccca tttctgcctc gctcaccgct ggcccttaga gaccccgagg 3840
taggggtggg gagatgcttc tctccttgcc ccccgccctc atgggtccta gcccttccct 3900
gagtgcgggc tgaggccaga gtcacctttt ctgtggctgg ctctaccttc ctgtccctga 3960
ggttaaacgg tgcccatcct gccatcctca aacgacagag gagcttttct ggaatttcaa 4020
accattgctc ttagtcccaa gctaggctta aacctggaat ctacaagcca aaagtccctc 4080
cctgcctgag ggcagtaccc tccattgggc acagtccaga cccaagtcaa agatgcccca 4140
ttccttgcgc ctcagccctc agttccttca tttccaccag gccgtgcctt gtttgagttt 4200
ttcctcccag tgagactgcc ccacggagac agaggaaagg gctggctccc cctccccagg 4260
ctggagaccc cccccaactc caggaaagag cagtcagagt ccagtgctct gcctcagacg 4320
ttgcctgaga agaagtggct gccacaccca ggggaaggcc ctgaggcgga ggctgtgctc 4380
cgccatggtg tcccggtacc ttccatacac agaggagtgc agccttctcc atatctccat 4440

ggccctgtcc caggccggcc cagatgtgtc ccccccaggc cttgtcctac gtccaaggtg 4500
gcagatgtct tccctgggct gccaccagcc cccgccccag agtggcccac cgtggcacta 4560
gaatgcaagt atcctgcgac cttgcaacct caccttcctg tgggtgttct ttcctgccct 4620
gtccaaaagc gccctcacta ttcttggacc atgccagatt ctgcctctct ggaaagaggc 4680
tctggacagc agaagcctcc aagcacagag cctggcccca ggccccagac agggtgggct 4740
tcctgccctt ccctctgggc acgcctgctg gccgacccac tgacccactc ggatggacca 4800
acctgctctg tccccaaagg acgcctgcag gagagagcag cactccgcat cacctcacca 4860
aggatcggac tctgcccctg gacctgggaa cgactggact gtcacggggt tccctcctag 4920
ctctcccagt gaactcctgc caggcacaca cagcccctat agcactgagc tcacatggga 4980
ctgggatatg ggggcatctc ttccccagag aggcactcag tgagcctcct gtgcctggcc 5040
ccagtctggg ccatctctta ggtgagacag ttgcccgaaa ctaagccagg cctggctgga 5100
ggagcagcag cttggggaga gggatttccc tgcagacctc aagccatcat gcggtgggtg 5160
ctgccatgac agaggctgca cccctgggcc agcggggctg ctcacccacc tcttgtgcaa 5220
ggtggccttt gtgctgcgcc tgcaggcaga gctggagccc ccagcagagg caggctggga 5280
cggaccagca tctggaagat gtacatagtt atttttctct ttgtggtttc ttgtttggtt 5340
tggtttgctt ttgacagctt cattttattt ttgacgtcac tttttggcca tgtaaactat 5400
ttgtggcaat tttatgtttt tatttatgaa taaagaatgc catttctcac gccctctaaa 5460
aaaaaaaaaa aaaaaaaaaa aaaaaaaaaa aa 5492
<210> 184
<211> 2193
<212> DNA
<213> Homo sapiens

<400> 184
atgcccaaaa atagcaaggt ggtaaaaaga gaattagatg atgatgttac tgagtctgtc 60
aaagaccttc tttccaatga agacgcagct gatgatgctt ttaagacaag tgaactaatt 120
gttgatggcc aggaagagaa agatacagat gttgaagaag gatctgaagt cgaagatgaa 180
agaccagctt ggaacagtaa actacaatac atcctggccc aagttggatt ttctgtaggt 240
ttaggaaatg tgtggcgatt tccataccta tgtcagaaga atgggggcgg tgcatatctt 300
ttaccatatt taatactact tatggtaata ggtattcccc tttttttctt ggaactctct 360
gtgggtcaaa gaattcggcg aggcagcatt ggtgtatgga attacataag ccctaaactg 420
ggcgggattg gatttgcaag ttgtgtagtg tgctattttg tagctctcta ctacaacgtc 480
atcattggct ggagtttgtt ttatttttct cagtcttttc agcaacccct gccttgggat 540
cagtgtcctt tggtgaaaaa tgcttcacac acttttgtag aaccagaatg tgaacaaagt 600
tctgccacca cctattactg gtacagggaa gcactgaata tttcaagttc catttctgaa 660
agtgggggct taaactggaa gatgaccatc tgcttgttgg ctgcctgggt catggtttgc 720
ttggctatga tcaaaggcat tcagtcttct ggaaaaatca tatattttag ttctctgttt 780
ccatatgtgg tacttatttg cttcctcatc agagcattcc ttttaaatgg ttcaattgat 840
ggcattcgcc acatgtttac ccctaagctt gaaataatgc tggagcccaa ggtctggaga 900
gaagctgcta ctcaagtgtt ctttgcctta ggtctgggat ttggtggtgt cattgccttt 960
tcaagctaca acaagagaga caacaactgc cactttgatg ctgtcctggt gtccttcatc 1020
aattttttca cttctgtcct ggcaacattg gtggtgtttg cagttctggg cttcaaagca 1080
aatgtcataa atgagaaatg cattacacaa aattcagaga cgatcatgaa atttttgaaa 1140
atggggaaca ttagtcagga tattattccc catcatatca acctttcaac tgttactgca 1200
gaagattatc atttagttta tgacatcatt caaaaagtga aagaagaaga gtttcctgct 1260
cttcatctca attcctgtaa aattgaagaa gagctaaata aagctgttca ggggaccggc 1320
ttagctttta ttgcctttac agaagcgatg acacattttc ctgcatctcc cttctggtca 1380
gtgatgtttt tcctcatgct ggtcaatcta ggccttggca gtatgtttgg aaccattgaa 1440
gggattgtca cgcctattgt ggacactttc aaagtgagga aagaaattct tactgttatc 1500
tgttgtcttc tggcattttg tattggcctg atatttgtgc aacgctctgg aaattacttt 1560
gttacaatgt ttgatgatta ttctgctaca ctgcctctgc taattgtagt cattttggag 1620
aatattgctg tatgctttgt ttatggcata gataagttta tggaagacct aaaagatatg 1680
ctgggctttg ctcccagcag atattactac tatatgtgga aatatatttc tcctctaatg 1740
ctattatcat tgctaatagc tagtgttgtg aatatgggat taagtcctcc tggctataac 1800
gcatggattg aagataaggc atctgaagaa tttctgagct atccaacatg gggactggtt 1860
gtttgtgtct ctctggttgt ctttgcaata ctcccagtcc ctgtagtttt cattgttcgt 1920
cgcttcaacc ttatagatga tagttctggt aatttagcat ctgtgaccta taagagagga 1980
agggtcctga aagagcctgt gaacttagag ggcgatgata caagcctcat tcacggaaaa 2040
ataccgagcg agatgccatc tccaaatttt ggtaaaaata tttatcgaaa acagagtgga 2100
tccccaactc tggatactgc tcccaatgga cggtatggaa tagggtactt gatggcagat 2160
attatgccag atatgccaga atctgatttg tag 2193

<210> 185

<211> 1601
<212> DNA
<213> Homo sapiens

<400> 185
cacagaggag ccagcgaacc tctcccggcg cctgttctgg gggctttctg ttccagcgtc 60
aaggatggag gagggggaga ggagcccctt actgtcccag gaaactgcag gccagaagcc 120
cctctctgtg cacaggccac ccacctcagg ctgcctaggt ccagtgccca gggaggacca 180
ggcggaggcc tggggctgca gctgctgtcc cccggagacc aagcaccagg ccttgagtgg 240
cactcccaag aaaggaccag ccccttccct ctccccaggg agcagctgcg tcaagtatct 300
gatcttcctc tccaacttcc ccttctccct gctggggctg ctggccctgg ccatcgggct 360
ctggggcctg gctgtcaagg ggtctctggg aagtgatctg ggggggcccc tgcccacaga 420
ccccatgctg gggctggcac tgggagggct ggtggtcagc gcagcgagcc tggctggctg 480
cctgggcgcc ctctgcgaga acacctgcct gttacgtggc ttctccgggg gcatccttgc 540
cttcctggtg cttgaggccg tggcgggggc cctggtggtg gccctctggg gcccgctgca 600
agacagcctg gagcacaccc tgcgtgtggc catcgcccac taccaggacg acccagacct 660
gcgcttcctc ctcgaccaag tccagctcgg gctgaggtgc tgcggagctg cctcctacca 720
ggactggcag cagaacctgt actttaactg cagctccccc ggggtgcagg cctgcagcct 780
tcccgcctcc tgctgcatcg acccccgcga agatggagcc tctgtcaacg accagtgcgg 840
cttcggggtc ctgcgcctgg atgcggacgc agctcagaga gtggtgtacc tggagggctg 900
cggcccgccg ctccggcggt ggctgcgcgc gaacctggct gcctcgggcg gctacgcaat 960
cgcggtggtg ctgctgcagg gggcggagct cctgctggcc gcccggctac tcggggccct 1020
cgctgcccgc agtggggcgg cgtacggccc cggagcgcac ggggaggacc gcgctggccc 1080
ccagagcccc agccccggcg ccccgcccgc tgccaaaccc gcccggggct gagcgcacgc 1140
cccgaggtcc gagaccgcca cgcacaggga tacagggggc gcctccgccc ggctaaaaag 1200
cgctgcctgc gccgccgccg ccgcctgatt tcgctcgggc ttcgggtgac ttcgccgcag 1260
gacctaccca gctcgctcac ttcgctcgct ccgcgtcccc catgccagcc cccaacgcag 1320
ggcgcccggc gaagccacgg gactggcggg aggagcacgc ggggccggag gaaatcctgg 1380
agctgaccct cacctccgag cccccactcc caccccagcc gcacagttcc cacctcctgg 1440
cacctccctc ccctggggcc gccacccctt ctgggctcgt gatggtggag ctaaggtcca 1500
ggcctctccc tcccgagtgc atttttgggg agatagtaaa tgttttattc gggtgtatca 1560
ttcatacagt aaagacacca atcttcaaaa aaaaaaaaaa a 1601

<210> 186
<211> 1900
<212> DNA
<213> Homo sapiens

<400> 186
gcgccctcgc cgcggacacc ggagctgcgg ccgctccccg ctgtccccca gagatggcag 60
atcctgaggt agttgtgagt agctgcagct ctcatgaaga ggaaaatcgc tgcaatttta 120
accagcaaac atctccatct gaggagcttc tattagaaga ccagatgagg cgaaaactca 180
aatttttttt catgaatccc tgtgagaagt tctgggctcg aggtagaaaa ccatggaaac 240
ttgccataca aattctaaaa attgcaatgg tgactatcca gctggtctta tttgggctaa 300
gtaaccagat ggtggtagct ttcaaggaag agaatactat agcattcaaa caccttttcc 360
taaaaggata tatggaccga atggatgaca catatgcagt gtacacacaa agtgacgtgt 420
atgatcagtt aatcttcgca gtaaaccagt acttgcagct atacaatgtc tccgttggga 480
atcatgctta tgagaacaaa ggtaccaagc aatctgctat ggcaatctgt cagcacttct 540
acaagcgagg aaacatctac cctggaaatg atacctttga catcgatcca gaaattgaaa 600
ctgagtgttt ctttgtggag ccagatgaac cttttcacat tgggacacca gcagaaaata 660
aactgaactt aacactggac ttccacagac tcctaacagt ggagcttcag tttaaactga 720
aagccattaa tctgcagaca gttcgtcatc aagaactccc tgactgttat gactttactc 780
tgactataac atttgacaac aaggcccata gtggaagaat taaaataagt ttagataatg 840
acatttccat cagagaatgt aaagactggc atgtatctgg atcaattcag aagaacactc 900
attacatgat gatctttgat gcctttgtca ttctgacttg cttggtttca ttaatcctct 960
gcattagatc tgtgattaga ggacttcagc ttcagcagga gtttgtcaat tttttcctcc 1020
tccattataa gaaggaagtt tctgtttctg atcaaatgga atttgtcaat ggatggtaca 1080
ttatgattat tattagtgac atattgacaa tcattggatc aattctaaaa atggaaatcc 1140
aagctaagag tctaactagt tatgatgtct gtagcatact tcttgggact tctaccatgc 1200
tcgtgtggct tggagtcatc cgatacctcg gtttctttgc aaagtacaac ctcctcattt 1260
tgacccttca ggcagcgctg cccaatgtca tcaggttctg ctgctgtgca gctatgattt 1320
acttaggtta ctgcttctgt ggatggatcg tgctggggcc ttaccatgac aagtttcgtt 1380
ctctgaacat ggtctctgag tgccttttct ctctgataaa tggagatgat atgtttgcca 1440

cgtttgcaaa aatgcagcaa aaaagttact tagtctggct gtttagtaga atttacctct 1500
actcattcat cagcctcttt atatatatga ttttaagtct tttcattgca ctgatcactg 1560
atacatacga aacaattaag caataccaac aagatggctt cccagagact gaacttcgta 1620
catttatatc agaatgcaaa gatctaccca actctggaaa atacagatta gaagatgacc 1680
ctccagtatc tttattctgc tgttgtaaaa agtagctatc aggtttatct gtactttaga 1740
ggaaaatata atgtgtagct gagttggaac actgtggata ttctgagatc agatgtagta 1800
tgtttggaga ctgttatttt gagctaattg agacctataa ttcaccaata actgtttata 1860
tttttaaaag caatatttaa tgtctttgca actttatgct 1900

<210> 187
<211> 2223
<212> DNA
<213> Homo sapiens

<400> 187
cgggtagcgc gctctgaaag tttatgaccg ccgcagccaa ctcctggccg gagctggaga 60
cgcagcgagc gatcggccgg cctcgaaccc ccacagctgg agggcgaggc cagctgtacc 120
cggccccagt gccctttcgc ggccacaagc ggccgtcctc ctggtccggt gctccggcgc 180
ctgatctagg ttcatggagc cggggctgtg gctccttttc gggctcacag tgacctccgc 240
cgcaggattc gtgccttgct cccagtctgg ggatgctggc aggcgcggcg tgtcccaggc 300
ccccactgca gccagatctg agggggactg tgaagagact gtggctggcc ctggcgagga 360
gactgtggct ggccctggcg aggggactgt ggccccgaca gcactgcagg gtccaagccc 420
tggaagccct gggcaggagc aggcggccga gggggcccct gagcaccacc gatccaggcg 480
ctgcacgtgc ttcacctaca aggacaagga gtgtgtctac tattgccacc tggacatcat 540
ttggatcaac actcccgaac agacggtgcc ctatggactg tccaactaca gaggaagctt 600
ccggggcaag aggtctgcgg ggccacttcc agggaatctg cagctctcac atcggccaca 660
cttgcgctgc gcttgtgtgg ggagatatga caaggcctgc ctgcactttt gcacccaaac 720
tctggacgtc agcagtaatt caaggacggc agaaaaaaca gacaaagaag aggaagggaa 780
ggttgaagtc aaggaccaac aaagcaagca ggctttagac ctccaccatc caaagctcat 840
gcccggcagt ggactcgccc tcgctccatc tacctgcccc cgctgcctct ttcaggaagg 900
agccccttag gaggacaggc ctgcagctcc aatttcatgc aggaaattgg ttttggagag 960
ttttggcaag ttggaaagcc acttactggc ttttgacatg acttctcttg gagaataagt 1020
ggactccaag ctaactcttt gcaaatgtaa acacatgtcc atcttgttaa taaatgcaaa 1080
atgcccgtgc agcagaagca tgcgactttc atatccttgc ctagaatagg ctgcatggtg 1140
tatgtcagtg agggccacga ggcgtcggct ttagacacag atcatagctc tacaggagtt 1200
tatgaatttg aagcttatgg gattttggca gagaaatttt cagctgtgct tgatacccac 1260
caaaagaatg tatctcgaaa gaatgaagga agaagaaaaa aggatccttg atgtttgtga 1320
caagaaaatg agaaagttag tatctgcaat acagagcttg ttcctgttca gtgactgacc 1380
ctctgtattc tgtatagaca ccaggccgat acacagtgga gttcccaggc cttgtttgca 1440
ggaagccgac tgtaaagaca gccccagctc aaggctatta ggttgaatat ttgctttcat 1500
gagtaaatgt ggatctttgg ggaatggctt caaaataagt cacgaacaca aattctttgt 1560
aaattatgta aattcctgtt tatataaatt ggcaacaact tataccgtct gacagttcaa 1620
aatctctttc agctgcgctc ttcccaccga gccgagctta ctgtgagtgt ggagatgtta 1680
tcccaccatg taaagtcgcc tgcgcagggg agggctgccc atctccccaa cccagtcaca 1740
gagagatagg aaacggcatt tgagtgggtg tccagggccc cgtagagaga catttaagat 1800
ggtgtatgac agagcattgg ccttgaccaa atgttaaatc ctctgtgtgt atttcataag 1860
ttattacagg tataaaagtg atgacctatc atgaggaaat gaaagtggct gatttgctgg 1920
taggattttg tacagtttag agaagcgatt atttattgtg aaactgttct ccactccaac 1980
tcctttatgt ggatctgttc aaagtagtca ctgtatatac gtatagagag gtagataggt 2040
aggtagattt taaattgcat tctgaataca aactcatact ccttagagct tgaattacat 2100
ttttaaaatg catatgtgct gtttggcacc gtggcaagat ggtatcagag agaaacccat 2160
caattgctca aatactcaga aagtactgtc aaaagcctaa taaaaaacct aaagtttgct 2220
ctg 2223

<210> 188
<211> 2032
<212> DNA
<213> Homo sapiens

<400> 188
cgcctggacc atgtgaatgg ggccagaggg ctcccgggct gggcagggac catgggctgt 60
ggctgcagct cacacccgga agatgactgg atggaaaaca tcgatgtgtg tgagaactgc 120
cattatccca tagtcccact ggatggcaag ggcacgctgc tcatccgaaa tggctctgag 180

gtgcgggacc cactggttac ctacgaaggc tccaatccgc cggcttcccc actgcaagac 240
aacctggtta tcgctctgca cagctatgag ccctctcacg acggagatct gggctttgag 300
aagggggaac cactccgcat cctggagcag agcggcgagt ggtggaaggc gcagtccctg 360
accacgggcc aggaaggctt catccccttc aattttgtgg ccaaagcgaa cagcctggag 420
cccgaaccct ggttcttcaa gaacctgagc cgcaaggacg cggagcggca gctcctggcg 480
cccgggaaca ctcacggctc cttcctcatc cgggagagcg agagcaccgc cgggtccttt 540
tcactgtcgg tccgggactt cgaccaaaac cagggagagg tggtgaaaca ttacaagatc 600
cgtaatctgg acaacggtgg cttctacatc tcccctcgaa tcacttttcc cggcctgcat 660
gaactggtcc gccattacac caatgcttca gatgggctgt gcacacggtt gagccgcccc 720
tgccagaccc agaagcccca gaagccgtgg tgggaggacg agtgggaggt tcccagggag 780
acgctgaagc tggtggagcg gctgggggct ggacagttcg gggaggtgtg gatggggtac 840
tacaacgggc acacgaaggt ggcggtgaag agcctgaagc agggcagcat gtccccggac 900
gccttcctgg ccgaggccaa cctcatgaag cagctgcaac accagcggct ggttcggctc 960
tacgctgtgg tcacccagga gcccatctac atcatcactg aatacatgga gaatgggagt 1020
ctagtggatt ttctcaagac cccttcaggc atcaagttga ccatcaacaa actcctggac 1080
atggcagccc aaattgcaga aggcatggca ttcattgaag agcggaatta tattcatcgt 1140
gaccttcggg ctgccaacat tctggtgtct gacaccctga gctgcaagat tgcagacttt 1200
ggcctagcac gcctcattga ggacaacgag tacacagcca gggagggggc caagtttccc 1260
attaagtgga cagcgccaga agccattaac tacgggacat tcaccatcaa gtcagatgtg 1320
tggtcttttg ggatcctgct gacggaaatt gtcacccacg gccgcatccc ttacccaggg 1380
atgaccaacc cggaggtgat tcagaacctg gagcgaggct accgcatggt gcgccctgac 1440
aactgtccag aggagctgta ccaactcatg aggctgtgct ggaaggagcg cccagaggac 1500
cggcccacct ttgactacct gcgcagtgtg ctggaggact tcttcacggc cacagagggc 1560
cagtaccagc ctcagccttg agaggaggcc ttgagaggcc ctggggttct ccccctttct 1620
ctccagcctg acttggggag atggagttct tgtgccatag tcacatggcc tatgcacata 1680
tggactctgc acatgaatcc cacccacatg tgacacatat gcaccttgtg tctgtacacg 1740
tgtcctgtag ttgcgtggac tctgcacatg tcttgtgcat gtgtagcctg tgcatgtatg 1800
tcttggacac tgtacaaggt acccctttct ggctctccca tttcctgaga ccaccagaga 1860
gaggggagaa gcctgggatt gacagaagct tctgcccacc tacttttctt tcctcagatc 1920
atccagaagt tcctcaaggg ccaggacttt atctaatacc tctgtgtgct cctccttggt 1980
gcctggcctg gcacacatca ggagttcaat aaatgtctgt tgatgactgc cg 2032

<210> 189
<211> 6400
<212> DNA
<213> Homo sapiens

<400> 189
ctgagatgca ggcccagcgc tggcgcagtg agaacttcga gagacccgtg gacctggagg 60
gctctgggga tgatgactcc tttcccgatg atgaactgga tgacctctac tcggggtcgg 120
gctcgggctg taagtacccc cctccctacc tctgcctggg ggcagtgacc agtctgtatc 180
cagcctcaac tctgagaagc ccacgggggg cttgcttcag tgaggcaggg ggcattcact 240
gcctgggtca ggcaagttct tcttgctctg gatccccaga acaagaaggg cagaacatcc 300
ttctggggat agaacaagac ctgggaggac ctcagggccc agaatgtcca ggtctccagg 360
gtcccacgtc tgcatctttg tgtgtgctat gtggctttct ctgcagacct tgcttggttc 420
cggtggtccc gtcaggtgca ataagctgtg gccaatggtg gggttagggc agtgatgaca 480
cagggcaggt gcttcaagtg gtgcctggga tggccccgcc ctggggaagg ggagccaggt 540
cagctgggtc tagcagaatc tcgggagaga agggcaccgc gaaccccact ctgccatgta 600
gcaactgtgt gcccctcggt gctccccctc cctgaatccc tgtattttca cctgtggatg 660
gtgagaaata cgacttcttc tcagtgcctc taggagggtt tgggatagga agctggggtc 720
tggcacagag gaggagctct caaaatggta gcaaaaagaa ggcagctgag actgggccca 780
gtgagggcac gggctggcat ctgaggagat ggtacggggt ctgcacgcca cagagctggc 840
tccccacagc cctcgcctct ggtctcccct ccttagccaa gtcctttgag cctctttcaa 900
attaatcttt gtaaaacccg tttatattgg gtcacatctc tattcgcaaa gttccctggc 960
tcccctccct cctgcctggt tccgagccca ggagctggct gtggcttctc cctcccccat 1020
cccctactcc ccgtctcatt tcctcccaag ttccttcact gtcccggctt tgcgtcagcc 1080
agattgctca agcagttccc ctgcctcaag ggcttctttt cccttttacc ctccgcctct 1140
gcctctgggg gaagccctta gtgtaccacc cctgccttga gcttgtggta ggggtgcctt 1200
tggggtctga ctcttcccag aatgccactc ctgtgttccc ctgcaagccc cagcaagttt 1260
tgcagccagt agcgcctcag ttgctgctgc ctggattcaa cctcatagaa actgtaggct 1320
gggtgggcag gaatagtcaa ggaaggcttc ccgggagagg cggcccccag aatttttgac 1380
ttggcagaga gaagttgggg aacatcagca actgaccacc tttagctggc tgtgccttcc 1440
tcctttctct catcttttct gggaaaattg ggtaaagaga gggggttggg agacctaatc 1500

ttgatggcca ttgcttacct gggatcctca tgcccctcac aaccacccag ctccctagct 1560
ctctccctct cccccacccc ctcagacttc gagcaggagt cgggcattga gacagccatg 1620
cgcttcagcc cagatgtagc cctggcggtg tccaccacac ctgcggtgct gcccaccacg 1680
aacatccagc ctgtgggcac accatttgaa gagctcccct ctgagcgccc caccctggag 1740
ccagccacca gccccctggt ggtgacagaa gtcccggaag agcccagcca gagagccacc 1800
accgtctcca ctaccatggc taccactgct gccacaagca caggggaccc gactgtggcc 1860
acagtgcctg ccacagtggc caccgccacc cccagcaccc ctgcagcacc cccttttacg 1920
gccaccactg ctgttataag gaccactggc gtacggaggc ttctgcctct cccactgacc 1980
acagtggcta cggcacgggc cactaccccc gaggcgccct ccccgcccac cacggcggct 2040
gtcttggaca ccgaggcccc aacacccagg ctggtcagca cagctacctc ccggccaaga 2100
gcccttccca ggccggccac cacccaggag cctgacatcc ctgagaggag caccctgccc 2160
ctggggacca ctgcccctgg acccacagag gtggctcaga ccccaactcc agagaccttc 2220
ctgaccacaa tccgggatga gccagaggtt ccggtgagtg gggggcccag tggagacttc 2280
gagctgccag aagaagagac cacacaacca gacacagcca atgaggtggt agctgtggga 2340
ggggctgcgg ccaaggcatc atctccacct gggacactgc ccaagggtgc ccgcccgggc 2400
cctggcctcc tggacaatgc catcgactcg ggcagctcag ctgctcagct gcctcagaag 2460
agtatcctgg agcggaagga ggtgctcgta gctgtgattg tgggcggggt ggtgggcgcc 2520
ctctttgctg ccttcttggt cacactgctc atctatcgta tgaagaaaaa ggatgagggc 2580
agctacacgc tggaggaacc caagcaggcg agcgtcacat accagaagcc tgacaagcag 2640
gaggagttct atgcctagtg gagccacagt gcctccctgc agcctcaaca ccaccctgct 2700
gtccagtccc cagcctggcc ccaccagccc aagcctggga ctgggcctgg aacctggccc 2760
cagttcttct ctgccctctc tcccaaggtc tgcccaggct gccagcctca cacagatctt 2820
ccccgaggaa gaggggctgc tgccatctgc cccagactgt gcccttacga gctcatctct 2880
tgttcccctc atccctgcca ccagtctggg gcttcaggac ctcatgtcag atggatggga 2940
ggaagaaagc tcctgattgg ctggtggtgg aagaaagggt ggggcttgag atgagcctga 3000
gccctgactt ggcacccaca gtgctcactg agatctcctt tttggggcag agaggcactc 3060
aggctggttt ccaggacaaa catttggtaa acacagccct tgaaatcatc tagacactgc 3120
aacctcttgc tcgtatccca gggcctctct ctagctgggt gagagggtgt cccttgtcac 3180
cagcctgttt tgtcctggtc tctctggggt tgttgaatct ctcctcttgc ctgccaagta 3240
cacatgtacc cagacttcat ttctttctgc atcttccccc aagaaacagc ttcctgaggg 3300
tgctggggca gccactggtg aggaggggct gctctgatgt ccctcctatg aggggactct 3360
gcacagacac cattgcccac actatcacca tattttcact cagtcacaca caagacaaaa 3420
gcatgcaatg acaaaaccat acgcaatcct gaccgcccag ccaatcaaga catatcacag 3480
aacacacgcg tccttccaag aatgtttatc ctcatgcatc acttacacac ccccagacac 3540
gtactgcaat gcaagtcact agtcatggtc acatgacagt gacagtgtgg cctcctccta 3600
ccccaaatac acccacactc tggcaccaca cacattgtct ccagctttca ggcttactgg 3660
ggagggtgga atcgagccag aacaatcagc ccatattggg tccccctaag ttgccccgtc 3720
atactcagtc ccatgccatg gtgcccacac caaccatgca gccaccaacc ccagccagtg 3780
tcagacacaa tcccatgtgg atgcacagtc tcactccaca tgacctgctc tcaatgctgg 3840
agggaaacag gcaggccctt gcctttctcg gaaaaagtgt ggggccacag cccttttagg 3900
gcattgcatg caggtgggcc tggcttcacc cctacctgct tcctccccac cgcagctggc 3960
agagggggag gtttggggcc agacccccac tagctgggag cctgggggct cctctaaggc 4020
tgaggaagga aatttggccc caggttgttg gggggtcttg ggtctcccag gacggaaggc 4080
ccagggcagg gagggggcat gtggttgggc tcctttatct ccctgtgtcc ccttcctgct 4140
ttgagctagg gggctgactc tgcctcccag gacacaagtc tccaaagtgc ctgtgagggc 4200
gggccctccg caccctctgc cctctgcctg gcaggccaac ctcagcccac ctgcccagag 4260
gccctccctg tggacacccc ctcacctatt tggccaaaca attctggctg cagcttcagg 4320
ggccatggct ggaagcagcc cctgcagatc cctcaggccc cgaggtcagg gtttgaggga 4380
tgagaccagg tgatagtggg ggaggggtta cttcctttgt tacctagcaa gtagggctat 4440
ttccatcggt attttaaatg tggggtcaca gatcttttgg ggagggtgtg cttggcaggg 4500
ggcctcttgg agccaaaggg atgtggtgtg agttgcgatt ggctggcact cacaccccca 4560
cccctcaccc acatcccaga ttcaagtcag gaaggcaggt tttatttcag ggcccttttc 4620
aagatgccct ggcagcagat ttctgcagga tgaggggtag cggtgtgtag gcagtgaggg 4680
ggaggttcca ggggctgtcc cacagcctgt cttttccagg ctgggctcca tcttccagtc 4740
ccaaaaccct ccttcacagg gcccagaggc ttgtgaggaa gccaggtgga cccagccttg 4800
gaagagtggg catggggggc ccctgatatc tggagggggc gggttggcct cagtcatctt 4860
tggagcagaa gggctgggtc ctggggccac agaccacaag gctcagcctc cctaccctgc 4920
tccctggggt gctgctgtct tggagagcac agctctggtg agacggcctg ggcaggccga 4980
ggctgagaaa ccagggagga tagaggagaa aaggcttggg gcccccagcc ccagaagatg 5040
ctgggcccca ggtgggagac ccaacagtgg gtgcagtttc tcagtggggc tggagccaat 5100
ggtgggggtg gccccggcag gcctggctcc tcacatccca ggggttggct tctgatttgg 5160
ggcttgggct ccaggcactg gcttctcttc tctgtgtcct tagcatttga gagaagaggc 5220
caggggcctt gttcatggat ccctggaccc aaggcagatg tccaggcttt atcctcctga 5280

ggatgaggag tctgaccagc ccaaatctgc cctggccggc ctgaccgggg caaggcagtc 5340
caagagagtt cagtgaggac cagctaggct ctcccaggtg caatgtgggt gcagggccct 5400
catgtccccc tacccctgcc tgtgatggag tgttctgagg ggctttggca tttgctggaa 5460
gcacagggag ttccaaatga gagggagctt ctgtggcttg agagcctctg gggccttggc 5520
tgccagagca cgaggcaggc caggacctgg agagcccaga ccctgtctcc aggaggccag 5580
gaggccagat gggggccttg cctgaaagac tggtcccctt gatcgctgga ggcatgtggg 5640
tggcaaccag ggctgggcag ggcttagggt gtgtgggcca aacccccctg gggttggcaa 5700
agccgcctgt caggcctcct ggtgggggcc cctggacaca gggagcagac cctctgcctc 5760
atggggtagg agtggctgcc tcctgtgttc tctggatttc ttctcccaac aactacaacc 5820
ctggacttgc ctccccaggc ctcttgcctg taaatagaag cccgcaaact gtacagattt 5880
acagaggcat cgagactggg ccctgggagt tgccatctga gagccgatgg ccccagcatc 5940
ccccaggtgc ctgcctggca ccacagtgac cctggcctca gcgtggcaaa tgcatgtaaa 6000
tatttttcgt aggcagcgtg gctccagaga gccccctgaa gacagtgtcc ctccctcctg 6060
tgagtccttt ctcctgtaca gaacctgcct ggggtgggtg ggggtctgcc attccctccc 6120
ccaggccttc cctgcccctt ctctcccctg taacctgttt attaaccata cctgtcctga 6180
gttcatggcc aaaaccttaa ataagaaaaa caaaagaaaa agacagtgga aaaaagagac 6240
caaggcgcct gccccactgc gggtactctc ctgttccagc cttgtgaagg aactggtttt 6300
gtttttgttt ttgttttttt tttttttgtt tgtttgtttg ttttttaaca cttcctgtgc 6360
tgtgcccatt tataagagga aataaaatta agctgaaatg 6400

<210> 190
<211> 1742
<212> DNA
<213> Homo sapiens

<400> 190
ctccgatcta gaggcagatt cctgactaat cccagagggc tggcccagcc tgtgctcccc 60
gggctgctag gaagcgatga ccactcttgt tagcccaagt tgaagaaagc cgggctgtgc 120
ctgggagccg agagaggcgg taatatttag aagctgcaca ggagaggaac atgaactgac 180
gagtaaacat gtatggaaat tattctcact tcatgaagtt tcccgcaggc tatggaggct 240
cccctggcca cactggctct acatccatga gcccatcagc agccttgtcc acagggaagc 300
caatggacag ccaccccagc tacacagata ccccagtgag tgccccacgg actctgagtg 360
cagtggggac ccccctcaat gccctgggct ctccatatcg agtcatcacc tctgccatgg 420
gcccaccctc aggagcactt gcagcgcctc caggaatcaa cttggttgcc ccacccagct 480
ctcagctaaa tgtggtcaac agtgtcagca gttcagagga catcaagccc ttaccagggc 540
ttcccgggat tggaaacatg aactacccat ccaccagccc cggatctctg gttaaacaca 600
tctgtgccat ctgtggagac agatcctcag gaaagcacta cggggtatac agttgtgaag 660
gctgcaaagg gttcttcaag aggacgataa ggaaggacct catctacacg tgtcgggata 720
ataaagactg cctcattgac aagcgtcagc gcaaccgctg ccagtactgt cgctatcaga 780
agtgccttgt catgggcatg aagagggaag ctgtgcaaga agaaagacag aggagccgag 840
agcgagctga gagtgaggca gaatgtgcta ccagtggtca tgaagacatg cctgtggaga 900
ggattctaga agctgaactt gctgttgaac caaagacaga atcctatggt gacatgaata 960
tggagaactc gacaaatgac cctgttacca acatatgtca tgctgctgac aagcagcttt 1020
tcaccctcgt tgaatggccc aagcgtattc cccacttctc tgacctcacc ttggaggacc 1080
aggtcatttt gcttcgggca gggtggaatg aattgctgat tgcctctttc tcccaccgct 1140
cagtttccgt gcaggatggc atccttctgg ccacgggttt acatgtccac cggagcagtg 1200
cccacagtgc tggggtcggc tccatctttg acagagtcct aactgagctg gtttccaaaa 1260
tgaaagacat gcagatggac aagtcggaac tgggatgcct gcgagccatt gtactcttta 1320
acccagatgc caagggcctg tccaacccct ctgaggtgga gactctgcga gagaaggttt 1380
atgccaccct tgaggcctac accaagcaga agtatccgga acagccaggc aggtttgcca 1440
agctgctgct gcgcctccca gctctgcgtt ccattggctt gaaatgcctg gagcacctct 1500
tcttcttcaa gctcatcggg gacaccccca ttgacacctt cctcatggag atgttggaga 1560
ccccgctgca gatcacctga gccccaccag ccacagcctc cccacccagg atgacccctg 1620
ggcaggtgtg tgtggacccc caccctgcac tttcctccac ctcccaccct gacccccttc 1680
ctgtccccaa aatgtgatgc ttataataaa gaaaaccttt ctacaaaaaa aaaaaaaaaa 1740
aa 1742

<210> 191
<211> 1658
<212> DNA
<213> Homo sapiens

<400> 191

ctctctctct atctctctca gaatgacaat tctaggtaca acttttggca tggttttttc 60
tttacttcaa gtcgtttctg gagaaagtgg ctatgctcaa aatggagact tggaagatgc 120
agaactggat gactactcat tctcatgcta tagccagttg gaagtgaatg gatcgcagca 180
ttcactgacc tgtgcttttg aggacccaga tgtcaacacc accaatctgg aatttgaaat 240
atgtggggcc ctcgtggagg taaagtgcct gaatttcagg aaactacaag agatatattt 300
catcgagaca aagaaattct tactgattgg aaagagcaat atatgtgtga aggttggaga 360
aaagagtcta acctgcaaaa aaatagacct aaccactata gttaaacctg aggctccttt 420
tgacctgagt gtcatctatc gggaaggagc caatgacttt gtggtgacat ttaatacatc 480
acacttgcaa aagaagtatg taaaagtttt aatgcatgat gtagcttacc gccaggaaaa 540
ggatgaaaac aaatggacgc atgtgaattt atccagcaca aagctgacac tcctgcagag 600
aaagctccaa ccggcagcaa tgtatgagat taaagttcga tccatccctg atcactattt 660
taaaggcttc tggagtgaat ggagtccaag ttattacttc agaactccag agatcaataa 720
tagctcaggg gagatggatc ctatcttact aaccatcagc attttgagtt ttttctctgt 780
cgctctgttg gtcatcttgg cctgtgtgtt atggaaaaaa aggattaagc ctatcgtatg 840
gcccagtctc cccgatcata agaagactct ggaacatctt tgtaagaaac caagaaaaaa 900
tttaaatgtg agtttcaatc ctgaaagttt cctggactgc cagattcata gggtggatga 960
cattcaagct agagatgaag tggaaggttt tctgcaagat acgtttcctc agcaactaga 1020
agaatctgag aagcagaggc ttggagggga tgtgcagagc cccaactgcc catctgagga 1080
tgtagtcgtc actccagaaa gctttggaag agattcatcc ctcacatgcc tggctgggaa 1140
tgtcagtgca tgtgacgccc ctattctctc ctcttccagg tccctagact gcagggagag 1200
tggcaagaat gggcctcatg tgtaccagga cctcctgctt agccttggga ctacaaacag 1260
cacgctgccc cctccatttt ctctccaatc tggaatcctg acattgaacc cagttgctca 1320
gggtcagccc attcttactt ccctgggatc aaatcaagaa gaagcatatg tcaccatgtc 1380
cagcttctac caaaaccagt gaagtgtaag aaacccagac tgaacttacc gtgagcgaca 1440
aagatgattt aaaagggaag tctagagttc ctagtctccc tcacagcaca gagaagacaa 1500
aattagcaaa accccactac acagtctgca agattctgaa acattgcttt gaccactctt 1560
cctgagttca gtggcactca acatgagtca agagcatcct gcttctacca tgtggatttg 1620
gtcacaaggt ttaaggtgac ccaatgattc agctattt 1658

<210> 192
<211> 1095
<212> DNA
<213> Homo sapiens

<400> 192
tgccgcccag gacccgcagc agagacgacg cctgcagcaa ggagaccagg aaggggtgag 60
acaaggaaga ggatgtctga gctggagaag gccatggtgg ccctcatcga cgttttccac 120
caatattctg gaagggaggg agacaagcac aagctgaaga aatccgaact caaggagctc 180
atcaacaatg agctttccca tttcttagag gaaatcaaag agcaggaggt tgtggacaaa 240
gtcatggaaa cactggacaa tgatggagac ggcgaatgtg acttccagga attcatggcc 300
tttgttgcca tggttactac tgcctgccac gagttctttg aacatgagtg agattagaaa 360
gcagccaaac ctttcctgta acagagacgg tcatgcaaga aagcagacag caagggcttg 420
cagcctagta ggagctgagc tttccagccg tgttgtagct aattaggaag cttgatttgc 480
tttgtgattg aaaaattgaa aacctctttc caaaggctgt tttaacggcc tgcatcattc 540
tttctgctat attaggcctg tgtgtaagct gactggcccc agggactctt gttaacagta 600
acttaggagt caggtctcag tgataaagcg tgcaccgtgc agcccgccat ggccgtgtag 660
accctaaccc ggagggaacc ctgactacag aaattacccc ggggcaccct taaaacttcc 720
actaccttta aaaaacaaag ccttatccag cattatttga aaacactgct gttctttaaa 780
tgcgttcctc atccatgcag ataacagctg gttggccggt gtggccctgc aagggcgtgg 840
tggcttcggc ctgcttcccg ggatgcgcct gatcaccagg tgaacgctca gcgctggcag 900
cgtcctggaa aaagcaactc catcagaact cgcaatccga gccagctctg ggggctccag 960
cgtggcctcc gtgacccatg cgattcaagt cgcggctgca ggatccttgc ctccaacgtg 1020
cctccagcac atgcggcttc cgagggcact accgggggct ctgagccacc gcgagggcct 1080
gcgttcaata aaaag 1095

<210> 193
<211> 2977
<212> DNA
<213> Homo sapiens

<400> 193
tagcaagttt ggcggctcca agccaggcgc gcctcaggat ccaggctcat ttgcttccac 60
ctagcttcgg tgccccctgc taggcgggga ccctcgagag cgatgccgat ggatttgatt 120

ttagttgtgt ggttctgtgt gtgcactgcc aggacagtgg tgggctttgg gatggaccct 180
gaccttcaga tggatatcgt caccgagctt gaccttgtga acaccaccct tggagttgct 240
caggtgtctg gaatgcacaa tgccagcaaa gcatttttat ttcaagacat agaaagagag 300
atccatgcag ctcctcatgt gagtgagaaa ttaattcagc tgttccagaa caagagtgaa 360
ttcaccattt tggccactgt acagcagaag ccatccactt caggagtgat actgtccatt 420
cgagaactgg agcacagcta ttttgaactg gagagcagtg gcctgaggga tgagattcgg 480
tatcactaca tacacaatgg gaagccaagg acagaggcac ttccttaccg catggcagat 540
ggacaatggc acaaggttgc actgtcagtt agcgcctctc atctcctgct ccatgtcgac 600
tgtaacagga tttatgagcg tgtgatagac cctccagata ccaaccttcc cccaggaatc 660
aatttatggc ttggccagcg caaccaaaag catggcttat tcaaagggat catccaagat 720
gggaagatca tctttatgcc gaatggatat ataacacagt gtccaaatct aaatcacact 780
tgcccaacct gcagtgattt cttaagcctg gtgcaaggaa taatggattt acaagagctt 840
ttggccaaga tgactgcaaa actaaattat gcagagacaa gacttagtca attggaaaac 900
tgtcattgtg agaagacttg tcaagtgagt ggactgctct atcgagatca agactcttgg 960
gtagatggtg accattgcag gaactgcact tgcaaaagtg gtgccgtgga atgccgaagg 1020
atgtcctgtc cccctctcaa ttgctcccca gactccctcc cagtacacat tgctggccag 1080
tgctgtaagg tctgccgacc aaaatgtatc tatggaggaa aagttcttgc agaaggccag 1140
cggattttaa ccaagagctg tcgggaatgc cgaggtggag ttttagtaaa aattacagaa 1200
atgtgtcctc ctttgaactg ctcagaaaag gatcacattc ttcctgagaa tcagtgctgc 1260
cgtgtctgta gaggtcataa cttttgtgca gaaggaccta aatgtggtga aaactcagag 1320
tgcaaaaact ggaatacaaa agctacttgt gagtgcaaga gtggttacat ctctgtccag 1380
ggagactctg cctactgtga agatattgat gagtgtgcag ctaagatgca ttactgtcat 1440
gccaatactg tgtgtgtcaa ccttcctggg ttatatcgct gtgactgtgt cccaggatac 1500
attcgtgtgg atgacttctc ttgtacagaa cacgatgaat gtggcagcgg ccagcacaac 1560
tgtgatgaga atgccatctg caccaacact gtccagggac acagctgcac ctgcaaaccg 1620
ggctacgtgg ggaacgggac catctgcaga gctttctgtg aagagggctg cagatacggt 1680
ggaacgtgtg tggctcccaa caaatgtgtc tgtccatctg gattcacagg aagccactgc 1740
gagaaagata ttgatgaatg ttcagaggga atcattgagt gccacaacca ttcccgctgc 1800
gttaacctgc cagggtggta ccactgtgag tgcagaagcg gtttccatga cgatgggacc 1860
tattcactgt ccggggagtc ctgtattgac attgatgaat gtgccttaag aactcacacc 1920
tgttggaacg attctgcctg catcaacctg gcagggggtt ttgactgtct ctgcccctct 1980
gggccctcct gctctggtga ctgtcctcat gaaggggggc tgaagcacaa tggccaggtg 2040
tggaccttga aagaagacag gtgttctgtc tgctcctgca aggatggcaa gatattctgc 2100
cgacggacag cttgtgattg ccagaatcca agtgctgacc tattctgttg cccagaatgt 2160
gacaccagag tcacaagtca atgtttagac caaaatggtc acaagctgta tcgaagtgga 2220
gacaattgga cccatagctg tcagcagtgt cggtgtctgg aaggagaggt agattgctgg 2280
ccactcactt gccccaactt gagctgtgag tatacagcta tcttagaagg ggaatgttgt 2340
ccccgctgtg tcagtgaccc ctgcctagct gataacatca cctatgacat cagaaaaact 2400
tgcctggaca gctatggtgt ttcacggctt agtggctcag tgtggacgat ggctggatct 2460
ccctgcacaa cctgtaaatg caagaatgga agagtctgtt gttctgtgga ttttgagtgt 2520
cttcaaaata attgaagtat ttacagtgga ctcaacgcag aagaatggac gaaatgacca 2580
tccaacgtga ttaaggatag gaatcggtag tttggttttt ttgtttgttt tgttttttta 2640
accacagata attgccaaag tttccacctg aggacggtgt ttcggaggtt gccttttgga 2700
cctaccactt tgctcattct tgctaaccta gtctaggtga cctacagtgc cgtgcattta 2760
agtcaatggt tgttaaaaga agtttcccgt gttgtaaatc atgtttccct tatcagatca 2820
tttgcaaata catttaaatg atctcatggt aaatggttga tgtatttttt gggtttattt 2880
tgtgtactaa ccataataga gagagactca gctcctttta tttattttgt tgatttatgg 2940
atcaaattct aaaataaagt tgcctgttgt gacttttt 2977

<210> 194
<211> 1650
<212> DNA
<213> Homo sapiens

<400> 194
caggaaggtt cctctcccag tggccatggg tagcaacagt gggcaggctg gccgccacat 60
ctataaatcc ctagctgatg atggcccctt tgactctgtg gagccgccta aaagacccac 120
cagcagactc atcatgcaca gcatggccat gttcggaaga gagttctgct acgcggtgga 180
ggcagcgtat gtgaccccag tcctgctcag cgtaggtctg cccagcagcc tgtacagcat 240
tgtgtggttc ctcagcccca tcctgggatt cctgctgcag cccgtggtcg gatcggccag 300
cgaccactgc cggtccaggt ggggccgccg gagaccctac atcctcaccc tgggagtcat 360
gatgctcgtg ggcatggctc tgtacctcaa tggggctact gttgtagcag ctttgattgc 420
taacccaagg aggaagctgg tttgggccat aagtgtcacc atgataggtg tcgttctctt 480

tgattttgct gccgacttca ttgatgggcc catcaaagcc tacttatttg atgtctgctc 540
ccatcaggac aaggagaagg gcctccacta ccatgccctc ttcacaggtt ttggaggtgc 600
cctgggttac cttttgggtg ctatagactg ggcccatctg gagctgggaa gactgttggg 660
tacagaattc caggtcatgt tcttcttctc tgcattggtg ctcactttgt gttttactgt 720
tcatctgtgc agtatctctg aagccccact tacagaggtt gcaaagggca ttcccccaca 780
gcaaacccct caggaccctc cattgtcatc agatggaatg tacgagtatg gttctatcga 840
gaaagttaaa aatggttacg taaatccaga gctggcaatg cagggagcaa aaaacaaaaa 900
tcatgctgaa cagactcgca gggcaatgac attaaagtca ctgctgagag cactggtgaa 960
catgcctcct cactaccgct acctttgcat cagccacctc attggatgga cggccttcct 1020
gtccaacatg ctgttcttca cagatttcat gggccagatt gtgtaccgcg gggatcccta 1080
tagtgcacac aactccacag agtttctcat ctacgaaaga ggagtcgagg ttggatgttg 1140
gggcttctgc atcaactccg tgttttcctc actttattct tactttcaga aagttttggt 1200
atcctacatt ggattaaagg gtctttactt cacgggatat ttgctgtttg gcctggggac 1260
gggatttatt gggctcttcc cgaatgtcta ctccaccctg gtcctgtgca gcctgtttgg 1320
tgtaatgtcc agcaccctgt acactgtgcc ctttaacctc attactgagt accaccgcga 1380
ggaagaaaag gagaggcagc aggccccagg aggggaccca gacaacagcg tgagagggaa 1440
gggcatggac tgcgccaccc tcacatgcat ggtgcagctg gctcagatcc tggtcggagg 1500
tggcctgggc tttctggtca acacagccgg gaccgttgtc gtcgtggtga tcacagcgtc 1560
tgcggtggca ctgataggct gttgctttgt cgctctcttt gttagatatg tggattaggt 1620
caataaagag acaatgaccc taaaaaaaaa 1650

<210> 195
<211> 2747
<212> DNA
<213> Homo sapiens

<400> 195
tgctctgtgc tgggatcatg gaacttgcac tgctgtgtgg gctggtggtg atggctggtg 60
tgattccaat ccagggcggg atcctgaacc tgaacaagat ggtcaagcaa gtgactggga 120
aaatgcccat cctctcctac tggccctacg gctgtcactg cggactaggt ggcagaggcc 180
aacccaaaga tgccacggac tggtgctgcc agacccatga ctgctgctat gaccacctga 240
agacccaggg gtgcagcatc tacaaggact attacagata caacttttcc caggggaaca 300
tccactgctc tgacaaggga agctggtgtg agcagcagct gtgtgcctgt gacaaggagg 360
tggccttctg cctgaagcgc aacctggaca cctaccagaa gcgactgcgt ttctactggc 420
ggccccactg ccgggggcag acccctgggt gctagaagcc cacaccctct accctgttcc 480
tcagcatgga gctctggcat ccccacctca gtatctaacc tgaaccagcc tggcttttca 540
aacactccgg ggggaggtag tcccagcctc ccccggaacc ctctaccaat gccttctgac 600
cttctgaagc tttccgaatc ctcccagttg aggcagtagc tgtgtcctct gagggtggat 660
gggaatcttg ggagaagccc aagcaaggga gccctcagag gtggtgtttg gaccaaagca 720
tcggggtggg ggaggggtct gccgctgtcc cccacctgct ggcccccttg tccttcctca 780
ccccctccaa tatagtctcg gagctacaac cgcagcagcc actataaagg gcaatattga 840
tctttctgtc catgtggctc tatcttttaa aacctcaagg ccctccactg tcctaagata 900
aagcctctca taggcactgg ggaccctgca cagtctggcc atgtgaccct ctccccaggc 960
aagctctgaa gtccctgcag gtggaggcca tgcctgtctt aaactcagtt gcatccctgg 1020
tgcccaaagc aacaccagaa ccaagaagga gctccataaa tccttcttgg gtgaagccta 1080
gacaaagccg ccaggtcttg tggctccagg caccagagcc ttgagtactt tctcctgcct 1140
ccaggcattg gctcagggtg aattacaagg ggctactgaa tggctattac tttcatcacg 1200
actgatcccc acctcctcag ggtcaaaggg ctactttctg gaagtctccc caggctgact 1260
ccttctccct gactgcaagg gctcactccc tcctccaagc tcccacaatg cttcatggct 1320
ctgccgctta cctagcttgg cctagagtgg caaatggaac ttctctgatc tcccccaact 1380
agactggagc ccccgaagga tggagaccat gtctgtgcca tctctgtttc ccctgttttc 1440
ccacatacta ggtgctcaat tcatgcctgt gaatggcgtg agcccataat ggatacacag 1500
aggttgcagc agatggtgtg ggtacctcac ccagatatca tccaggccca aggcccctct 1560
ccctgagtga ggccaggtgt tggcagccaa ctgctccaat ctgcctcctt cccctaaata 1620
ctgccctggt ctagtgggag ctgccttccc cctgccccac ctctcccacc aagaggccac 1680
ctgtcactca tggccaggag agtgacacca tggagggtac aattgccagc tcccccgtgt 1740
ctgtgcagga ttgtctgggt tgaatgacac tctcaaattg ttcctgggat cgggctgagg 1800
ccaggcctct cctggaacca cctctctgct tggtctgacc ccttggccta tccagttttc 1860
ctggttccct cacaggtttc tccagaaagt actccctcag taaagcattt gcacaagaat 1920
ccttgtctca ggctctgctt ctaaggaaag agactgaaga gggacaactt tttcctatga 1980
ggactcccaa tctgccattt gtgtgtggct agctgtttcc agccaggggg ctgaggcacc 2040
tgcagtcacc cacactctgg gcctgtggca ccatctgtgc ctcagctgac tgagcagttc 2100
tgagatgtgg tgactcatgt gaagtggtgt cataggaatg aaggactgtg ggcgctgggt 2160

tcaaattcca gttctatgca ccactccagg tagggatacc catgggggca gctgtgcctg 2220
tgtgggggaa cagggtaggg aaatctccat accttctcaa ttttgctgtg aacctaaaac 2280
tgccatataa agataaagtc taggccaggc atggtggctc atgcctgtaa tcccaacact 2340
tagggtggct gaggcaggca gatcacctgg ggtcaggagt tcaagaccag cctgcccaac 2400
atggcaaaac ctcgtctcta ctaaaaatac aaaaattagc caggcatcgt ggtgggtgcc 2460
tgtaatccca gctattcagg aggctgaggc aggagaatca cttggacctg ggaggcagag 2520
gtttcagtga gcccagatcg cgacactgca ctccagcctg ggcaacagag tgacactctg 2580
tctcaaaaaa taaaataaaa taaaataaaa taaaataaaa taaaataaaa aaaaaaaaaa 2640
aaaaaaaaaa aaaaaaaaaa aaaaaaaaaa aaaaaaaaaa aaaaaaaaaa aaaaaaaaaa 2700
aaaaaaaaaa aaaaaaaaaa aaaaaaaaaa aaaaaaaaaa aaaaaaa 2747

<210> 196
<211> 2497
<212> DNA
<213> Homo sapiens

<400> 196
gaggttggga gagacggagc ggacctcagc gctgaagcag aagtccccgg agctgcggtc 60
tccccgccgc ggctgagcca tgcggctccc tgacctgaga ccctggacct ccctgctgct 120
ggtggacgcg gctttactgt ggctgcttca gggccctctg gggactttgc ttcctcaagg 180
gctgccagga ctatggctgg aggggaccct gcggctggga gggctgtggg ggctgctaaa 240
gctaagaggg ctgctgggat ttgtggggac actgctgctc ccgctctgtc tggccacccc 300
cctgactgtc tccctgagag ccctggtcgc gggggcctca cgtgctcccc cagccagagt 360
cgcttcagcc ccttggagct ggctgctggt ggggtacggg gctgcggggc tcagctggtc 420
actgtgggct gttctgagcc ctcctggagc ccaggagaag gagcaggacc aggtgaacaa 480
caaagtcttg atgtggaggc tgctgaagct ctccaggccg gacctgcctc tcctcgttgc 540
cgccttcttc ttccttgtcc ttgctgtttt gggtgagaca ttaatccctc actattctgg 600
tcgtgtcatt gacatcctgg gaggtgattt tgacccccat gcctttgcca gtgccatctt 660
cttcatgtgc ctcttctcct ttggcagctc actgtctgca ggctgccgag gaggctgctt 720
cacctacacc atgtctcgaa tcaacttgcg gatccgggag cagcttttct cctccctgct 780
gcgccaggac ctcggtttct tccaggagac taagacaggg gagctgaact cacggctgag 840
ctcggatacc accctgatga gtaactggct tcctttaaat gccaatgtgc tcttgcgaag 900
cctggtgaaa gtggtggggc tgtatggctt catgctcagc atatcgcctc gactcaccct 960
cctttctctg ctgcacatgc ccttcacaat agcagcggag aaggtgtaca acacccgcca 1020
tcaggaagtg cttcgggaga tccaggatgc agtggccagg gcggggcagg tggtgcggga 1080
agccgttgga gggctgcaga ccgttcgcag ttttggggcc gaggagcatg aagtctgtcg 1140
ctataaagag gcccttgaac aatgtcggca gctgtattgg cggagagacc tggaacgcgc 1200
cttgtacctg ctcataagga gggtgctgca cttgggtgtg cagatgctga tgctgagctg 1260
tgggctgcag cagatgcagg atggggagct cacccagggc agcctgcttt cctttatgat 1320
ctaccaggag agcgtgggga gctatgtgca gaccctggta tacatatatg gggatatgct 1380
cagcaacgtg ggagctgcag agaaggtttt ctcctacatg gaccgacagc caaatctgcc 1440
ttcacctggc acgcttgccc ccaccactct gcagggggtt gtgaaattcc aagacgtctc 1500
ctttgcatat cccaatcgcc ctgacaggcc tgtgctcaag gggctgacgt ttaccctacg 1560
tcctggtgag gtgacggcgc tggtgggacc caatgggtct gggaagagca cagtggctgc 1620
cctgctgcag aatctgtacc agcccacagg gggacaggtg ctgctggatg aaaagcccat 1680
ctcacagtat gaacactgct acctgcacag ccaggtggtt tcagttgggc aggagcctgt 1740
gctgttctcc ggttctgtga ggaacaacat tgcttatggg ctgcagagct gcgaagatga 1800
taaggtgatg gcggctgccc aggctgccca cgcagatgac ttcatccagg aaatggagca 1860
tggaatatac acagatgtag gggagaaggg aagccagctg gctgcgggac agaaacaacg 1920
tctggccatt gcccgggccc ttgtacgaga cccgcgggtc ctcatcctgg atgaggctac 1980
tagtgcccta gatgtgcagt gcgagcaggc caaaaccctt tggaagttca tgatattttg 2040
aatttcaatg gatatttcct gggaataatg agttcaaatg aacgaatatg tggaacaaag 2100
catcaccaac atttattttt tcaggatgag gtgatggaca aaaccatcac agggaaattg 2160
aggcaaatag tacatgtaaa acaatacttc gggtgagtcc acctatccca aagtcgtatc 2220
aaagaagtgg ctgcagattg gagcccaaag cctttggttc ctcagtttcc aaatggattc 2280
tcactaggtg ggatcatgag tttgctttgg acaccccaaa ttctaactat ttcttttgtt 2340
tcttacatcc tttccctctt ccccagcccc ttcccctcat gttacacctc ttgctggttt 2400
gagacgtcaa tcaccactga gaaagaatta aaccagtatt ttgagctggc aaaattctta 2460
gcctagtaca attccttcaa ttaaactgta gctcaac 2497

<210> 197
<211> 2545

<212> DNA
<213> Homo sapiens

<400> 197
atccaataca ggagtgactt ggaactccat tctatcacta tgaagaaaag tggtgttctt 60
ttcctcttgg gcatcatctt gctggttctg attggagtgc aaggaacccc agtagtgaga 120
aagggtcgct gttcctgcat cagcaccaac caagggacta tccacctaca atccttgaaa 180
gaccttaaac aatttgcccc aagcccttcc tgcgagaaaa ttgaaatcat tgctacactg 240
aagaatggag ttcaaacatg tctaaaccca gattcagcag atgtgaagga actgattaaa 300
aagtgggaga aacaggtcag ccaaaagaaa aagcaaaaga atgggaaaaa acatcaaaaa 360
aagaaagttc tgaaagttcg aaaatctcaa cgttctcgtc aaaagaagac tacataagag 420
accacttcac caataagtat tctgtgttaa aaatgttcta ttttaattat accgctatca 480
ttccaaagga ggatggcata taatacaaag gcttattaat ttgactagaa aatttaaaac 540
attactctga aattgtaact aaagttagaa agttgatttt aagaatccaa acgttaagaa 600
ttgttaaagg ctatgattgt ctttgttctt ctaccaccca ccagttgaat ttcatcatgc 660
ttaaggccat gattttagca atacccatgt ctacacagat gttcacccaa ccacatccca 720
ctcacaacag ctgcctggaa gagcagccct aggcttccac gtactgcagc ctccagagag 780
tatctgaggc acatgtcagc aagtcctaag cctgttagca tgctggtgag ccaagcagtt 840
tgaaattgag ctggacctca ccaagctgct gtggccatca acctctgtat ttgaatcagc 900
ctacaggcct cacacacaat gtgtctgaga gattcatgct gattgttatt gggtatcacc 960
actggagatc accagtgtgt ggctttcaga gcctcctttc tggctttgga agccatgtga 1020
ttccatcttg cccgctcagg ctgaccactt tatttctttt tgttcccctt tgcttcattc 1080
aagtcagctc ttctccatcc taccacaatg cagtgccttt cttctctcca gtgcacctgt 1140
catatgctct gatttatctg agtcaactcc tttctcatct tgtccccaac accccacaga 1200
agtgctttct tctcccaatt catcctcact cagtccagct tagttcaagt cctgcctctt 1260
aaataaacct ttttggacac acaaattatc ttaaaactcc tgtttcactt ggttcagtac 1320
cacatgggtg aacactcaat ggttaactaa ttcttgggtg tttatcctat ctctccaacc 1380
agattgtcag ctccttgagg gcaagagcca cagtatattt ccctgtttct tccacagtgc 1440
ctaataatac tgtggaacta ggttttaata attttttaat tgatgttgtt atgggcagga 1500
tggcaaccag accattgtct cagagcaggt gctggctctt tcctggctac tccatgttgg 1560
ctagcctctg gtaacctctt acttattatc ttcaggacac tcactacagg gaccagggat 1620
gatgcaacat ccttgtcttt ttatgacagg atgtttgctc agcttctcca acaataagaa 1680
gcacgtggta aaacacttgc ggatattctg gactgttttt aaaaaatata cagtttaccg 1740
aaaatcatat aatcttacaa tgaaaaggac tttatagatc agccagtgac caaccttttc 1800
ccaaccatac aaaaattcct tttcccgaag gaaaagggct ttctcaataa gcctcagctt 1860
tctaagatct aacaagatag ccaccgagat ccttatcgaa actcatttta ggcaaatatg 1920
agttttattg tccgtttact tgtttcagag tttgtattgt gattatcaat taccacacca 1980
tctcccatga agaaagggaa cggtgaagta ctaagcgcta gaggaagcag ccaagtcggt 2040
tagtggaagc atgattggtg cccagttagc ctctgcagga tgtggaaacc tccttccagg 2100
ggaggttcag tgaattgtgt aggagaggtt gtctgtggcc agaatttaaa cctatactca 2160
ctttcccaaa ttgaatcact gctcacactg ctgatgattt agagtgctgt ccggtggaga 2220
tcccacccga acgtcttatc taatcatgaa actccctagt tccttcatgt aacttccctg 2280
aaaaatctaa gtgtttcata aatttgagag tctgtgaccc acttaccttg catctcacag 2340
gtagacagta tataactaac aaccaaagac tacatattgt cactgacaca cacgttataa 2400
tcatttatca tatatataca tacatgcata cactctcaaa gcaaataatt tttcacttca 2460
aaacagtatt gacttgtata ccttgtaatt tgaaatattt tctttgttaa aatagaatgg 2520
tatcaataaa tagaccatta atcag 2545

<210> 198
<211> 538
<212> DNA
<213> Homo sapiens

<400> 198
agggagagag ggaggggagg aaattggaga ccccagcacc cccttgctca ctctcttgct 60
cacagtccac gatggcccgg tccctggtgt gccttggtgt catcatcttg ctgtctgcct 120
tctccggacc tggtgtcagg ggtggtccta tgcccaagct ggctgaccgg aagctgtgtg 180
cggaccagga gtgcagccac cctatctcca tggctgtggc ccttcaggac tacatggccc 240
ccgactgccg attcctgacc attcaccggg gccaagtggt gtatgtcttc tccaagctga 300
agggccgtgg gcggctcttc tggggaggca gcgttcaggg agattactat ggagatctgg 360
ctgctcgcct gggctatttc cccagtagca ttgtccgaga ggaccagacc ctgaaacctg 420
gcaaagtcga tgtgaagaca gacaaatggg atttctactg ccagtgagct cagcctaccg 480
ctggccctgc cgtttcccct ccttgggttt atgcaaatac aatcagccca gtgcaaac 538

<210> 199
<211> 469
<212> PRT
<213> Homo sapiens

<400> 199
Met His Ser Phe Pro Pro Leu Leu Leu Leu Leu Phe Trp Gly Val Val
1               5                   10                  15
Ser His Ser Phe Pro Ala Thr Leu Glu Thr Gln Glu Gln Asp Val Asp
            20                  25                  30
Leu Val Gln Lys Tyr Leu Glu Lys Tyr Tyr Asn Leu Lys Asn Asp Gly
        35                  40                  45
Arg Gln Val Glu Lys Arg Arg Asn Ser Gly Pro Val Val Glu Lys Leu
    50                  55                  60
Lys Gln Met Gln Glu Phe Phe Gly Leu Lys Val Thr Gly Lys Pro Asp
65                  70                  75                  80
Ala Glu Thr Leu Lys Val Met Lys Gln Pro Arg Cys Gly Val Pro Asp
                85                  90                  95
Val Ala Gln Phe Val Leu Thr Glu Gly Asn Pro Arg Trp Glu Gln Thr
            100                 105                 110
His Leu Thr Tyr Arg Ile Glu Asn Tyr Thr Pro Asp Leu Pro Arg Ala
        115                 120                 125
Asp Val Asp His Ala Ile Glu Lys Ala Phe Gln Leu Trp Ser Asn Val
    130                 135                 140
Thr Pro Leu Thr Phe Thr Lys Val Ser Glu Gly Gln Ala Asp Ile Met
145                 150                 155                 160
Ile Ser Phe Val Arg Gly Asp His Arg Asp Asn Ser Pro Phe Asp Gly
                165                 170                 175
Pro Gly Gly Asn Leu Ala His Ala Phe Gln Pro Gly Pro Gly Ile Gly
            180                 185                 190
Gly Asp Ala His Phe Asp Glu Asp Glu Arg Trp Thr Asn Asn Phe Arg
        195                 200                 205
Glu Tyr Asn Leu His Arg Val Ala Ala His Glu Leu Gly His Ser Leu
    210                 215                 220
Gly Leu Ser His Ser Thr Asp Ile Gly Ala Leu Met Tyr Pro Ser Tyr
225                 230                 235                 240
Thr Phe Ser Gly Asp Val Gln Leu Ala Gln Asp Asp Ile Asp Gly Ile
                245                 250                 255
Gln Ala Ile Tyr Gly Arg Ser Gln Asn Pro Val Gln Pro Ile Gly Pro
            260                 265                 270
Gln Thr Pro Lys Ala Cys Asp Ser Lys Leu Thr Phe Asp Ala Ile Thr
        275                 280                 285
Thr Ile Arg Gly Glu Val Met Phe Phe Lys Asp Arg Phe Tyr Met Arg
    290                 295                 300
Thr Asn Pro Phe Tyr Pro Glu Val Glu Leu Asn Phe Ile Ser Val Phe
305                 310                 315                 320
Trp Pro Gln Leu Pro Asn Gly Leu Glu Ala Ala Tyr Glu Phe Ala Asp
                325                 330                 335
Arg Asp Glu Val Arg Phe Phe Lys Gly Asn Lys Tyr Trp Ala Val Gln
            340                 345                 350
Gly Gln Asn Val Leu His Gly Tyr Pro Lys Asp Ile Tyr Ser Ser Phe
        355                 360                 365
Gly Phe Pro Arg Thr Val Lys His Ile Asp Ala Ala Leu Ser Glu Glu
    370                 375                 380
Asn Thr Gly Lys Thr Tyr Phe Phe Val Ala Asn Lys Tyr Trp Arg Tyr
385                 390                 395                 400
Asp Glu Tyr Lys Arg Ser Met Asp Pro Gly Tyr Pro Lys Met Ile Ala
                405                 410                 415
His Asp Phe Pro Gly Ile Gly His Lys Val Asp Ala Val Phe Met Lys
            420                 425                 430
Asp Gly Phe Phe Tyr Phe Phe His Gly Thr Arg Gln Tyr Lys Phe Asp
        435                 440                 445

Pro Lys Thr Lys Arg Ile Leu Thr Leu Gln Lys Ala Asn Ser Trp Phe
    450                 455                 460
Asn Cys Arg Lys Asn
465

<210> 200
<211> 469
<212> PRT
<213> Homo sapiens

<400> 200
Met His Ser Phe Pro Pro Leu Leu Leu Leu Leu Phe Trp Gly Val Val
1               5                   10                  15
Ser His Ser Phe Pro Ala Thr Leu Glu Thr Gln Glu Gln Asp Val Asp
            20                  25                  30
Leu Val Gln Lys Tyr Leu Glu Lys Tyr Tyr Asn Leu Lys Asn Asp Gly
        35                  40                  45
Arg Gln Val Glu Lys Arg Arg Asn Ser Gly Pro Val Val Glu Lys Leu
    50                  55                  60
Lys Gln Met Gln Glu Phe Phe Gly Leu Lys Val Thr Gly Lys Pro Asp
65                  70                  75                  80
Ala Glu Thr Leu Lys Val Met Lys Gln Pro Arg Cys Gly Val Pro Asp
                85                  90                  95
Val Ala Gln Phe Val Leu Thr Glu Gly Asn Pro Arg Trp Glu Gln Thr
            100                 105                 110
His Leu Thr Tyr Arg Ile Glu Asn Tyr Thr Pro Asp Leu Pro Arg Ala
        115                 120                 125
Asp Val Asp His Ala Ile Glu Lys Ala Phe Gln Leu Trp Ser Asn Val
    130                 135                 140
Thr Pro Leu Thr Phe Thr Lys Val Ser Glu Gly Gln Ala Asp Ile Met
145                 150                 155                 160
Ile Ser Phe Val Arg Gly Asp His Arg Asp Asn Ser Pro Phe Asp Gly
                165                 170                 175
Pro Gly Gly Asn Leu Ala His Ala Phe Gln Pro Gly Pro Gly Ile Gly
            180                 185                 190
Gly Asp Ala His Phe Asp Glu Asp Glu Arg Trp Thr Asn Asn Phe Arg
        195                 200                 205
Glu Tyr Asn Leu His Arg Val Ala Ala His Ala Leu Gly His Ser Leu
    210                 215                 220
Gly Leu Ser His Ser Thr Asp Ile Gly Ala Leu Met Tyr Pro Ser Tyr
225                 230                 235                 240
Thr Phe Ser Gly Asp Val Gln Leu Ala Gln Asp Asp Ile Asp Gly Ile
                245                 250                 255
Gln Ala Ile Tyr Gly Arg Ser Gln Asn Pro Val Gln Pro Ile Gly Pro
            260                 265                 270
Gln Thr Pro Lys Ala Cys Asp Ser Lys Leu Thr Phe Asp Ala Ile Thr
        275                 280                 285
Thr Ile Arg Gly Glu Val Met Phe Phe Lys Asp Arg Phe Tyr Met Arg
    290                 295                 300
Thr Asn Pro Phe Tyr Pro Glu Val Glu Leu Asn Phe Ile Ser Val Phe
305                 310                 315                 320
Trp Pro Gln Leu Pro Asn Gly Leu Glu Ala Ala Tyr Glu Phe Ala Asp
                325                 330                 335
Arg Asp Glu Val Arg Phe Phe Lys Gly Asn Lys Tyr Trp Ala Val Gln
            340                 345                 350
Gly Gln Asn Val Leu His Gly Tyr Pro Lys Asp Ile Tyr Ser Ser Phe
        355                 360                 365
Gly Phe Pro Arg Thr Val Lys His Ile Asp Ala Ala Leu Ser Glu Glu
    370                 375                 380
Asn Thr Gly Lys Thr Tyr Phe Phe Val Ala Asn Lys Tyr Trp Arg Tyr
385                 390                 395                 400
Asp Glu Tyr Lys Arg Ser Met Asp Pro Gly Tyr Pro Lys Met Ile Ala
                405                 410                 415

His Asp Phe Pro Gly Ile Gly His Lys Val Asp Ala Val Phe Met Lys
            420                 425                 430
Asp Gly Phe Phe Tyr Phe Phe His Gly Thr Arg Gln Tyr Lys Phe Asp
        435                 440                 445
Pro Lys Thr Lys Arg Ile Leu Thr Leu Gln Lys Ala Asn Ser Trp Phe
    450                 455                 460
Asn Cys Arg Lys Asn
465

<210> 201
<211> 477
<212> PRT
<213> Homo sapiens

<400> 201

<210> 202
<211> 1806
<212> PRT
<213> Homo sapiens

<220>
<221> MOD_RES
<222> (1)...(1806)
<223> Xaa = any amino acid

<400> 202
Met Glu Pro Trp Ser Ser Arg Trp Lys Thr Lys Arg Trp Leu Trp Asp
 1               5                  10                  15
Phe Thr Val Thr Thr Leu Ala Leu Thr Phe Leu Phe Gln Ala Arg Glu
            20                  25                  30
Val Arg Gly Ala Ala Pro Val Asp Val Leu Lys Ala Leu Asp Phe His
        35                  40                  45
Asn Ser Pro Glu Gly Ile Ser Lys Thr Thr Gly Phe Cys Thr Asn Arg
    50                  55                  60
Lys Asn Ser Lys Gly Ser Asp Thr Ala Tyr Arg Val Ser Lys Gln Ala
65                  70                  75                  80
Gln Leu Ser Ala Pro Thr Lys Gln Leu Phe Pro Gly Gly Thr Phe Pro
                85                  90                  95
Glu Asp Phe Ser Ile Leu Phe Thr Val Lys Pro Lys Lys Gly Ile Gln
            100                 105                 110
Ser Phe Leu Leu Ser Ile Tyr Asn Glu His Gly Ile Gln Gln Ile Gly
        115                 120                 125
Val Glu Val Gly Arg Ser Pro Val Phe Leu Phe Glu Asp His Thr Gly
    130                 135                 140
Lys Pro Ala Pro Glu Asp Tyr Pro Leu Phe Arg Thr Val Asn Ile Ala
145                 150                 155                 160
Asp Gly Lys Trp His Arg Val Ala Ile Ser Val Glu Lys Lys Thr Val
                165                 170                 175
Thr Met Ile Val Asp Cys Lys Lys Lys Thr Thr Lys Pro Leu Asp Arg
            180                 185                 190
Ser Glu Arg Ala Ile Val Asp Thr Asn Gly Ile Thr Val Phe Gly Thr
        195                 200                 205

Arg Ile Leu Asp Glu Glu Val Phe Glu Gly Asp Ile Gln Gln Phe Leu
    210                 215                 220
Ile Thr Gly Asp Pro Lys Ala Ala Tyr Asp Tyr Cys Glu His Tyr Ser
225                 230                 235                 240
Pro Asp Cys Asp Ser Ser Ala Pro Lys Ala Ala Gln Ala Gln Glu Pro
                245                 250                 255
Gln Ile Asp Glu Tyr Ala Pro Glu Asp Ile Ile Glu Tyr Asp Tyr Glu
            260                 265                 270
Tyr Gly Glu Ala Glu Tyr Lys Glu Ala Glu Ser Val Thr Glu Gly Pro
        275                 280                 285
Thr Val Thr Glu Glu Thr Ile Ala Gln Thr Glu Ala Asn Ile Val Asp
    290                 295                 300

Asp Phe Gln Glu Tyr Asn Tyr Gly Thr Met Glu Ser Tyr Gln Thr Glu
305                 310                 315                 320
Ala Pro Arg His Val Ser Gly Thr Asn Glu Pro Asn Pro Val Glu Glu
                325                 330                 335
Ile Phe Thr Glu Glu Tyr Leu Thr Gly Glu Asp Tyr Asp Ser Gln Arg
            340                 345                 350
Lys Asn Ser Glu Asp Thr Leu Tyr Glu Asn Lys Glu Ile Asp Gly Arg
        355                 360                 365
Asp Ser Asp Leu Leu Val Asp Gly Asp Leu Gly Glu Tyr Asp Phe Tyr
    370                 375                 380
Glu Tyr Lys Glu Tyr Glu Asp Lys Pro Thr Ser Pro Pro Asn Glu Glu
385                 390                 395                 400
Phe Gly Pro Gly Val Pro Ala Glu Thr Asp Ile Thr Glu Thr Ser Ile
                405                 410                 415
Asn Gly His Gly Ala Tyr Gly Glu Lys Gly Gln Lys Gly Glu Pro Ala
            420                 425                 430
Val Val Glu Pro Gly Met Leu Val Glu Gly Pro Pro Gly Pro Ala Gly
        435                 440                 445
Pro Ala Gly Ile Met Gly Pro Pro Gly Leu Gln Gly Pro Thr Gly Pro
    450                 455                 460
Pro Gly Asp Pro Gly Asp Arg Gly Pro Pro Gly Arg Pro Gly Leu Pro
465                 470                 475                 480
Gly Ala Asp Gly Leu Pro Gly Pro Pro Gly Thr Met Leu Met Leu Pro
                485                 490                 495
Phe Arg Tyr Gly Gly Asp Gly Ser Lys Gly Pro Thr Ile Ser Ala Gln
            500                 505                 510
Glu Ala Gln Ala Gln Ala Ile Leu Gln Gln Ala Arg Ile Ala Leu Arg
        515                 520                 525
Gly Pro Pro Gly Pro Met Gly Leu Thr Gly Arg Pro Gly Pro Val Gly
    530                 535                 540
Gly Pro Gly Ser Ser Gly Ala Lys Gly Glu Ser Gly Asp Pro Gly Pro
545                 550                 555                 560
Gln Gly Pro Arg Gly Val Gln Gly Pro Pro Gly Pro Thr Gly Lys Pro
                565                 570                 575
Gly Lys Arg Gly Arg Pro Gly Ala Asp Gly Gly Arg Gly Met Pro Gly
            580                 585                 590
Glu Pro Gly Ala Lys Gly Asp Arg Gly Phe Asp Gly Leu Pro Gly Leu
        595                 600                 605
Pro Gly Asp Lys Gly His Arg Gly Glu Arg Gly Pro Gln Gly Pro Pro
    610                 615                 620
Gly Pro Pro Gly Asp Asp Gly Met Arg Gly Glu Asp Gly Glu Ile Gly
625                 630                 635                 640
Pro Arg Gly Leu Pro Gly Glu Ala Gly Pro Arg Gly Leu Leu Gly Pro
                645                 650                 655
Arg Gly Thr Pro Gly Ala Pro Gly Gln Pro Gly Met Ala Gly Val Asp
            660                 665                 670
Gly Pro Pro Gly Pro Lys Gly Asn Met Gly Pro Gln Gly Glu Pro Gly
        675                 680                 685

Pro Pro Gly Gln Gln Gly Asn Pro Gly Pro Gln Gly Leu Pro Gly Pro
    690                 695                 700
Gln Gly Pro Ile Gly Pro Pro Gly Glu Lys Gly Pro Gln Gly Lys Pro
705                 710                 715                 720
Gly Leu Ala Gly Leu Pro Gly Ala Asp Gly Pro Pro Gly His Pro Gly
                725                 730                 735
Lys Glu Gly Gln Ser Gly Glu Lys Gly Ala Leu Gly Pro Pro Gly Pro
            740                 745                 750
Gln Gly Pro Ile Gly Xaa Pro Gly Pro Arg Gly Val Lys Gly Ala Asp
        755                 760                 765
Gly Val Arg Gly Leu Lys Gly Ser Lys Gly Glu Lys Gly Glu Asp Gly
    770                 775                 780
Phe Pro Gly Phe Lys Gly Asp Met Gly Leu Lys Gly Asp Arg Gly Glu
785                 790                 795                 800

Val Gly Gln Ile Gly Pro Arg Gly Xaa Asp Gly Pro Glu Gly Pro Lys
                805                 810                 815
Gly Arg Ala Gly Pro Thr Gly Asp Pro Gly Pro Ser Gly Gln Ala Gly
            820                 825                 830
Glu Lys Gly Lys Leu Gly Val Pro Gly Leu Pro Gly Tyr Pro Gly Arg
        835                 840                 845
Gln Gly Pro Lys Gly Ser Thr Gly Phe Pro Gly Phe Pro Gly Ala Asn
    850                 855                 860
Gly Glu Lys Gly Ala Arg Gly Val Ala Gly Lys Pro Gly Pro Arg Gly
865                 870                 875                 880
Gln Arg Gly Pro Thr Gly Pro Arg Gly Ser Arg Gly Ala Arg Gly Pro
                885                 890                 895
Thr Gly Lys Pro Gly Pro Lys Gly Thr Ser Gly Gly Asp Gly Pro Pro
            900                 905                 910
Gly Pro Pro Gly Glu Arg Gly Pro Gln Gly Pro Gln Gly Pro Val Gly
        915                 920                 925
Phe Pro Gly Pro Lys Gly Pro Pro Gly Pro Pro Gly Arg Met Gly Cys
    930                 935                 940
Pro Gly His Pro Gly Gln Arg Gly Glu Thr Gly Phe Gln Gly Lys Thr
945                 950                 955                 960
Gly Pro Pro Gly Pro Gly Gly Val Val Gly Pro Gln Gly Pro Thr Gly
                965                 970                 975
Glu Thr Gly Pro Ile Gly Glu Arg Gly Tyr Pro Gly Pro Pro Gly Pro
            980                 985                 990
Pro Gly Glu Gln Gly Leu Pro Gly Ala Ala Gly Lys Glu Gly Ala Lys
        995                 1000                1005
Gly Asp Pro Gly Pro Gln Gly Ile Ser Gly Lys Asp Gly Pro Ala Gly
    1010                1015                1020
Leu Arg Gly Phe Pro Gly Glu Arg Gly Leu Pro Gly Ala Gln Gly Ala
1025                1030                1035                1040
Pro Gly Leu Lys Gly Gly Glu Gly Pro Gln Gly Pro Pro Gly Pro Val
                1045                1050                1055
Gly Ser Pro Gly Glu Arg Gly Ser Ala Gly Thr Ala Gly Pro Ile Gly
            1060                1065                1070
Leu Arg Gly Arg Pro Gly Pro Gln Gly Pro Pro Gly Pro Ala Gly Glu
        1075                1080                1085
Lys Gly Ala Pro Gly Glu Lys Gly Pro Gln Gly Pro Ala Gly Arg Asp
    1090                1095                1100
Gly Val Gln Gly Pro Val Gly Leu Pro Gly Pro Ala Gly Pro Ala Gly
1105                1110                1115                1120
Ser Pro Gly Glu Asp Gly Asp Lys Gly Glu Ile Gly Glu Pro Gly Gln
                1125                1130                1135
Lys Gly Ser Lys Gly Gly Lys Gly Glu Asn Gly Pro Pro Gly Pro Pro
            1140                1145                1150
Gly Leu Gln Gly Pro Val Gly Ala Pro Gly Ile Ala Gly Gly Asp Gly
        1155                1160                1165

Glu Pro Gly Pro Arg Gly Gln Gln Gly Met Phe Gly Gln Lys Gly Asp
    1170                1175                1180
Glu Gly Ala Arg Gly Phe Pro Gly Pro Pro Gly Pro Ile Gly Leu Gln
1185                1190                1195                1200
Gly Leu Pro Gly Pro Pro Gly Glu Lys Gly Glu Asn Gly Asp Val Gly
                1205                1210                1215
Pro Trp Gly Pro Pro Gly Pro Pro Gly Pro Arg Gly Pro Gln Gly Pro
            1220                1225                1230
Asn Gly Ala Asp Gly Pro Gln Gly Pro Pro Gly Ser Val Gly Ser Val
        1235                1240                1245
Gly Gly Val Gly Glu Lys Gly Glu Pro Gly Glu Ala Gly Asn Pro Gly
    1250                1255                1260
Pro Pro Gly Glu Ala Gly Val Gly Gly Pro Lys Gly Glu Arg Gly Glu
1265                1270                1275                1280
Lys Gly Glu Ala Gly Pro Pro Gly Ala Ala Gly Pro Pro Gly Ala Lys
                1285                1290                1295

Gly Pro Pro Gly Asp Asp Gly Pro Lys Gly Asn Pro Gly Pro Val Gly
            1300                1305                1310
Phe Pro Gly Asp Pro Gly Pro Pro Gly Glu Leu Gly Pro Ala Gly Gln
        1315                1320                1325
Asp Gly Val Gly Gly Asp Lys Gly Glu Asp Gly Asp Pro Gly Gln Pro
    1330                1335                1340
Gly Pro Pro Gly Pro Ser Gly Glu Ala Gly Pro Pro Gly Pro Pro Gly
1345                1350                1355                1360
Lys Arg Gly Pro Pro Gly Ala Ala Gly Ala Glu Gly Arg Gln Gly Glu
                1365                1370                1375
Lys Gly Ala Lys Gly Glu Ala Gly Ala Glu Gly Pro Pro Gly Lys Thr
            1380                1385                1390
Gly Pro Val Gly Pro Gln Gly Pro Ala Gly Lys Pro Gly Pro Glu Gly
        1395                1400                1405
Leu Arg Gly Ile Pro Gly Pro Val Gly Glu Gln Gly Leu Pro Gly Ala
    1410                1415                1420
Ala Gly Gln Asp Gly Pro Pro Gly Pro Met Gly Pro Pro Gly Leu Pro
1425                1430                1435                1440
Gly Leu Lys Gly Asp Pro Gly Ser Lys Gly Glu Lys Gly His Pro Gly
                1445                1450                1455
Leu Ile Gly Leu Ile Gly Pro Pro Gly Glu Gln Gly Glu Lys Gly Asp
            1460                1465                1470
Arg Gly Leu Pro Gly Thr Gln Gly Ser Pro Gly Ala Lys Gly Asp Gly
        1475                1480                1485
Gly Ile Pro Gly Pro Ala Gly Pro Leu Gly Pro Pro Gly Pro Pro Gly
    1490                1495                1500
Leu Pro Gly Pro Gln Gly Pro Lys Gly Asn Lys Gly Ser Thr Gly Pro
1505                1510                1515                1520
Ala Gly Gln Lys Gly Asp Ser Gly Leu Pro Gly Pro Pro Gly Pro Pro
                1525                1530                1535
Gly Pro Pro Gly Glu Val Ile Gln Pro Leu Pro Ile Leu Ser Ser Lys
            1540                1545                1550
Lys Thr Arg Arg His Thr Glu Gly Met Gln Ala Asp Ala Asp Asp Asn
        1555                1560                1565
Ile Leu Asp Tyr Ser Asp Gly Met Glu Glu Ile Phe Gly Ser Leu Asn
    1570                1575                1580
Ser Leu Lys Gln Asp Ile Glu His Met Lys Phe Pro Met Gly Thr Gln
1585                1590                1595                1600
Thr Asn Pro Ala Arg Thr Cys Lys Asp Leu Gln Leu Ser His Pro Asp
                1605                1610                1615
Phe Pro Asp Gly Glu Tyr Trp Ile Asp Pro Asn Gln Gly Cys Ser Gly
            1620                1625                1630
Asp Ser Phe Lys Val Tyr Cys Asn Phe Thr Ser Gly Gly Glu Thr Cys
        1635                1640                1645

Ile Tyr Pro Asp Lys Lys Ser Glu Gly Val Arg Ile Ser Ser Trp Pro
    1650                1655                1660
Lys Glu Lys Pro Gly Ser Trp Phe Ser Glu Phe Lys Arg Gly Lys Leu
1665                1670                1675                1680
Leu Ser Tyr Leu Asp Val Glu Gly Asn Ser Ile Asn Met Val Gln Met
                1685                1690                1695
Thr Phe Leu Lys Leu Leu Thr Ala Ser Ala Arg Gln Asn Phe Thr Tyr
            1700                1705                1710
His Cys His Gln Ser Ala Ala Trp Tyr Asp Val Ser Ser Gly Ser Tyr
        1715                1720                1725
Asp Lys Ala Leu Arg Phe Leu Gly Ser Asn Asp Glu Glu Met Ser Tyr
    1730                1735                1740
Asp Asn Asn Pro Phe Ile Lys Thr Leu Tyr Asp Gly Cys Thr Ser Arg
1745                1750                1755                1760
Lys Gly Tyr Glu Lys Thr Val Ile Glu Ile Asn Thr Pro Lys Ile Asp
                1765                1770                1775
Gln Val Pro Ile Val Asp Val Met Ile Ser Asp Phe Gly Asp Gln Asn
            1780                1785                1790

Gln Lys Phe Gly Phe Glu Val Gly Pro Val Cys Phe Leu Gly
        1795                1800                1805

<210> 203
<211> 243
<212> PRT
<213> Homo sapiens

<400> 203
Met Arg Pro Gln Gly Pro Ala Ala Ser Pro Gln Arg Leu Arg Gly Leu
1               5                   10                  15
Leu Leu Leu Leu Leu Leu Gln Leu Pro Ala Pro Ser Ser Ala Ser Glu
            20                  25                  30
Ile Pro Lys Gly Lys Gln Lys Ala Gln Leu Arg Gln Arg Glu Val Val
        35                  40                  45
Asp Leu Tyr Asn Gly Met Cys Leu Gln Gly Pro Ala Gly Val Pro Gly
    50                  55                  60
Arg Asp Gly Ser Pro Gly Ala Asn Gly Ile Pro Gly Thr Pro Gly Ile
65                  70                  75                  80
Pro Gly Arg Asp Gly Phe Lys Gly Glu Lys Gly Glu Cys Leu Arg Glu
                85                  90                  95
Ser Phe Glu Glu Ser Trp Thr Pro Asn Tyr Lys Gln Cys Ser Trp Ser
            100                 105                 110
Ser Leu Asn Tyr Gly Ile Asp Leu Gly Lys Ile Ala Glu Cys Thr Phe
        115                 120                 125
Thr Lys Met Arg Ser Asn Ser Ala Leu Arg Val Leu Phe Ser Gly Ser
    130                 135                 140
Leu Arg Leu Lys Cys Arg Asn Ala Cys Cys Gln Arg Trp Tyr Phe Thr
145                 150                 155                 160
Phe Asn Gly Ala Glu Cys Ser Gly Pro Leu Pro Ile Glu Ala Ile Ile
                165                 170                 175
Tyr Leu Asp Gln Gly Ser Pro Glu Met Asn Ser Thr Ile Asn Ile His
            180                 185                 190
Arg Thr Ser Ser Val Glu Gly Leu Cys Glu Gly Ile Gly Ala Gly Leu
        195                 200                 205
Val Asp Val Ala Ile Trp Val Gly Thr Cys Ser Asp Tyr Pro Lys Gly
    210                 215                 220
Asp Ala Ser Thr Gly Trp Asn Ser Val Ser Arg Ile Ile Ile Glu Glu
225                 230                 235                 240
Leu Pro Lys

<210> 204
<211> 1665
<212> PRT
<213> Homo sapiens

<400> 204
Met Pro Gly Thr Lys Leu Thr Arg Thr Gly Ala Pro Ala Asp Tyr Arg
1               5                   10                  15
Val Ile Leu Lys Thr Ser Gln Glu Asp Glu Leu Asp Val Pro Asp Asp
            20                  25                  30
Ile Ser Val Arg Val Met Ser Ser Gln Ser Val Leu Val Ser Trp Val
        35                  40                  45
Asp Pro Val Leu Glu Lys Gln Lys Lys Val Val Ala Ser Arg Gln Tyr
    50                  55                  60
Thr Val Arg Tyr Arg Glu Lys Gly Glu Leu Ala Arg Trp Asp Tyr Lys
65                  70                  75                  80
Gln Ile Ala Asn Arg Arg Val Leu Ile Glu Asn Leu Ile Pro Asp Thr
                85                  90                  95
Val Tyr Glu Phe Ala Val Arg Ile Ser Gln Gly Glu Arg Asp Gly Lys
            100                 105                 110
Trp Ser Thr Ser Val Phe Gln Arg Thr Pro Glu Ser Ala Pro Thr Thr

            115                 120                 125
Ala Pro Glu Asn Leu Asn Val Trp Pro Val Asn Gly Lys Pro Thr Val
    130                 135                 140
Val Ala Ala Ser Trp Asp Ala Leu Pro Glu Thr Glu Gly Lys Val Lys
145                 150                 155                 160
Val Cys Leu Leu Asp Thr Gly Leu Phe Ser Val Ser Ser Phe Gln Pro
                165                 170                 175
Ser Ala Lys Ser Phe Gln Asn Thr Phe Phe His Thr Pro Arg Leu Ser
            180                 185                 190
Asn His Leu Glu Gln Ser Pro Ser Pro Ile Leu Glu Thr Leu Leu Leu
        195                 200                 205
Pro Trp Trp Met Val Cys Ser Leu Gly Asn Ala Ile Phe Ser Lys Ser
    210                 215                 220
Gly Pro Gln Thr Gly Glu Ala Trp Asp Leu Thr Pro Lys Pro Ser Leu
225                 230                 235                 240
Ser Leu Cys Gln Gln Glu Cys Ser Cys Thr Gln Lys Asp Phe Ser Cys
                245                 250                 255
Leu Ala Tyr Leu Ile Asp Ile Gln Thr Lys Gln Val Asn Lys Asp Pro
            260                 265                 270
Gln Leu Glu Gly Ser Val Phe Gly Pro Cys Phe Leu Phe Tyr Phe Leu
        275                 280                 285
Thr Phe Met Leu Asp Ile Gly Gly Phe Ser Phe Ile Met Cys Tyr Glu
    290                 295                 300
Asp Pro Val Ser Ser Leu Thr Gly Asn Ser Leu Lys Ser Val Ala Ala
305                 310                 315                 320
Ser Lys Ala Asp Val Gln Gln Asn Thr Glu Asp Asn Gly Lys Pro Glu
                325                 330                 335
Lys Pro Glu Pro Ser Ser Pro Ser Pro Arg Ala Pro Ala Ser Ser Gln
            340                 345                 350
His Pro Ser Val Pro Ala Ser Pro Gln Gly Arg Asn Ala Lys Asp Leu
        355                 360                 365
Leu Leu Asp Leu Lys Asn Lys Ile Leu Ala Asn Gly Gly Ala Pro Arg
    370                 375                 380
Lys Pro Gln Leu Arg Ala Lys Lys Ala Glu Glu Leu Asp Leu Gln Ser
385                 390                 395                 400
Thr Glu Ile Thr Gly Glu Glu Glu Leu Gly Ser Arg Glu Asp Ser Pro
                405                 410                 415
Met Ser Pro Ser Asp Thr Gln Asp Gln Lys Arg Thr Leu Arg Pro Pro
            420                 425                 430

Ser Arg His Gly His Ser Val Val Ala Pro Gly Arg Thr Ala Val Arg
        435                 440                 445
Ala Arg Met Pro Ala Leu Pro Arg Arg Glu Gly Val Asp Lys Pro Gly
    450                 455                 460
Phe Ser Leu Ala Thr Gln Pro Arg Pro Gly Ala Pro Pro Ser Ala Ser
465                 470                 475                 480
Ala Ser Pro Ala His His Ala Ser Thr Gln Gly Thr Ser His Arg Pro
                485                 490                 495
Ser Leu Pro Ala Ser Leu Asn Asp Asn Asp Leu Val Asp Ser Asp Glu
            500                 505                 510
Asp Glu Arg Ala Val Gly Ser Leu His Pro Lys Gly Ala Phe Ala Gln
        515                 520                 525
Pro Arg Pro Ala Leu Ser Pro Ser Arg Gln Ser Pro Ser Ser Val Leu
    530                 535                 540
Arg Asp Arg Ser Ser Val His Pro Gly Ala Lys Pro Ala Ser Pro Ala
545                 550                 555                 560
Arg Arg Thr Pro His Ser Gly Ala Ala Glu Glu Asp Ser Ser Ala Ser
                565                 570                 575
Ala Pro Pro Ser Arg Leu Ser Pro Pro His Gly Gly Ser Ser Arg Leu
            580                 585                 590
Leu Pro Thr Gln Pro His Leu Ser Ser Pro Leu Ser Lys Gly Gly Lys
        595                 600                 605
Asp Gly Glu Asp Ala Pro Ala Thr Asn Ser Asn Ala Pro Ser Arg Ser

610                 615                 620
Thr Met Ser Ser Ser Val Ser Ser His Leu Ser Ser Arg Thr Gln Val
625                 630                 635                 640
Ser Glu Gly Ala Glu Ala Ser Asp Gly Glu Ser His Gly Asp Gly Asp
                645                 650                 655
Arg Glu Asp Gly Gly Arg Gln Ala Glu Ala Thr Ala Gln Thr Leu Arg
            660                 665                 670
Ala Arg Pro Ala Ser Gly His Phe His Leu Leu Arg His Lys Pro Phe
        675                 680                 685
Ala Ala Asn Gly Arg Ser Pro Ser Arg Phe Ser Ile Gly Arg Gly Pro
    690                 695                 700
Arg Leu Gln Pro Ser Ser Ser Pro Gln Ser Thr Val Pro Ser Arg Ala
705                 710                 715                 720
His Pro Arg Val Pro Ser His Ser Asp Ser His Pro Lys Leu Ser Ser
                725                 730                 735
Gly Ile His Gly Asp Glu Glu Asp Glu Lys Pro Leu Pro Ala Thr Val
            740                 745                 750
Val Asn Asp His Val Pro Ser Ser Ser Arg Gln Pro Ile Ser Arg Gly
        755                 760                 765
Trp Glu Asp Leu Arg Arg Ser Pro Gln Arg Gly Ala Ser Leu His Arg
    770                 775                 780
Lys Glu Pro Ile Pro Glu Asn Pro Lys Ser Thr Gly Ala Asp Thr His
785                 790                 795                 800
Pro Gln Gly Lys Tyr Ser Ser Leu Ala Ser Lys Ala Gln Asp Val Gln
                805                 810                 815
Gln Ser Thr Asp Ala Asp Thr Glu Gly His Ser Pro Lys Ala Gln Pro
            820                 825                 830
Gly Ser Thr Asp Arg His Ala Ser Pro Ala Arg Pro Pro Ala Ala Arg
        835                 840                 845
Ser Gln Gln His Pro Ser Val Pro Arg Arg Met Thr Pro Gly Arg Ala
    850                 855                 860
Pro Glu Gln Gln Pro Pro Pro Pro Val Ala Thr Ser Gln His His Pro
865                 870                 875                 880
Gly Pro Gln Ser Arg Asp Ala Gly Arg Ser Pro Ser Gln Pro Arg Leu
                885                 890                 895
Ser Leu Thr Gln Ala Gly Arg Pro Arg Pro Thr Ser Gln Gly Arg Ser
            900                 905                 910

His Ser Ser Ser Asp Pro Tyr Thr Ala Ser Ser Arg Gly Met Leu Pro
        915                 920                 925
Thr Ala Leu Gln Asn Gln Asp Glu Asp Ala Gln Gly Ser Tyr Asp Asp
    930                 935                 940
Asp Ser Thr Glu Val Glu Ala Gln Asp Val Arg Ala Pro Ala His Ala
945                 950                 955                 960
Ala Arg Ala Lys Glu Ala Ala Ala Ser Leu Pro Lys His Gln Gln Val
                965                 970                 975
Glu Ser Pro Thr Gly Ala Gly Ala Gly Gly Asp His Arg Ser Gln Arg
            980                 985                 990
Gly His Ala Ala Ser Pro Ala Arg Pro Ser Arg Pro Gly Gly Pro Gln
        995                 1000                1005
Ser Arg Ala Arg Val Pro Ser Arg Ala Ala Pro Gly Lys Ser Glu Pro
    1010                1015                1020
Pro Ser Lys Arg Pro Leu Ser Ser Lys Ser Gln Gln Ser Val Ser Ala
1025                1030                1035                1040
Glu Asp Glu Glu Glu Glu Asp Ala Gly Phe Phe Lys Gly Gly Lys Glu
                1045                1050                1055
Asp Leu Leu Ser Ser Ser Val Pro Lys Trp Pro Ser Ser Ser Thr Pro
            1060                1065                1070
Arg Gly Gly Lys Asp Ala Asp Gly Ser Leu Ala Lys Glu Glu Arg Glu
        1075                1080                1085
Pro Ala Ile Ala Leu Ala Pro Arg Gly Gly Ser Leu Ala Pro Val Lys
    1090                1095                1100
Arg Pro Leu Pro Pro Pro Pro Gly Ser Ser Pro Arg Ala Ser His Val

1105                1110                1115                1120
Pro Ser Arg Pro Pro Pro Arg Ser Ala Ala Thr Val Ser Pro Val Ala
                1125                1130                1135
Gly Thr His Pro Trp Pro Arg Tyr Thr Thr Arg Ala Pro Pro Gly His
            1140                1145                1150
Phe Ser Thr Thr Pro Met Leu Ser Leu Arg Gln Arg Met Met His Ala
        1155                1160                1165
Arg Phe Arg Asn Pro Leu Ser Arg Gln Pro Ala Arg Pro Ser Tyr Arg
    1170                1175                1180
Gln Gly Tyr Asn Gly Arg Pro Asn Val Glu Gly Lys Val Leu Pro Gly
1185                1190                1195                1200
Ser Asn Gly Lys Pro Asn Gly Gln Arg Ile Ile Asn Gly Pro Gln Gly
                1205                1210                1215
Thr Lys Trp Val Val Asp Leu Asp Arg Gly Leu Val Leu Asn Ala Glu
            1220                1225                1230
Gly Arg Tyr Leu Gln Asp Ser His Gly Asn Pro Leu Arg Ile Lys Leu
        1235                1240                1245
Gly Gly Asp Gly Arg Thr Ile Val Asp Leu Glu Gly Thr Pro Val Val
    1250                1255                1260
Ser Pro Asp Gly Leu Pro Leu Phe Gly Gln Gly Arg His Gly Thr Pro
1265                1270                1275                1280
Leu Ala Asn Ala Gln Asp Lys Pro Ile Leu Ser Leu Gly Gly Lys Pro
                1285                1290                1295
Leu Val Gly Leu Glu Val Ile Lys Lys Thr Thr His Pro Pro Thr Thr
            1300                1305                1310
Thr Met Gln Pro Thr Thr Thr Thr Thr Pro Leu Pro Thr Thr Thr Thr
        1315                1320                1325
Pro Arg Pro Thr Thr Ala Thr Thr Met Gln Pro Thr Thr Thr Thr Thr
    1330                1335                1340
Pro Leu Pro Thr Thr Thr Pro Arg Pro Thr Thr Ala Thr Thr Arg Arg
1345                1350                1355                1360
Thr Thr Thr Arg Arg Pro Thr Thr Thr Val Arg Thr Thr Thr Arg Thr
                1365                1370                1375
Thr Thr Thr Thr Thr Pro Lys Pro Thr Thr Pro Ile Pro Thr Cys Pro
            1380                1385                1390

Pro Gly Thr Leu Glu Arg His Asp Asp Asp Gly Asn Leu Ile Met Ser
        1395                1400                1405
Ser Asn Gly Ile Pro Glu Cys Tyr Ala Glu Glu Asp Glu Phe Ser Gly
    1410                1415                1420
Leu Glu Thr Asp Thr Ala Val Pro Thr Glu Glu Ala Tyr Val Ile Tyr
1425                1430                1435                1440
Asp Glu Asp Tyr Glu Phe Glu Thr Ser Arg Pro Pro Thr Thr Thr Glu
                1445                1450                1455
Pro Ser Thr Thr Ala Thr Thr Pro Arg Val Ile Pro Glu Glu Gly Ala
            1460                1465                1470
Ile Ser Ser Phe Pro Glu Glu Glu Phe Asp Leu Ala Gly Arg Lys Arg
        1475                1480                1485
Phe Val Ala Pro Tyr Val Thr Tyr Leu Asn Lys Asp Pro Ser Ala Pro
    1490                1495                1500
Cys Ser Leu Thr Asp Ala Leu Asp His Phe Gln Val Asp Ser Leu Asp
1505                1510                1515                1520
Glu Ile Ile Pro Asn Asp Leu Lys Lys Ser Asp Leu Pro Pro Gln His
                1525                1530                1535
Ala Pro Arg Asn Ile Thr Val Val Ala Val Glu Gly Cys His Ser Phe
            1540                1545                1550
Val Ile Val Asp Trp Asp Lys Ala Thr Pro Gly Asp Leu Val Thr Gly
        1555                1560                1565
Tyr Leu Val Tyr Ser Ala Ser Tyr Glu Asp Phe Ile Arg Asn Lys Phe
    1570                1575                1580
Ser Thr Gln Ala Ser Ser Val Thr His Leu Pro Ile Glu Asn Leu Lys
1585                1590                1595                1600
Pro Asn Thr Arg Tyr Tyr Phe Lys Val Gln Ala Gln Asn Pro His Gly

1605                1610                1615
Tyr Gly Pro Ile Ser Pro Ser Val Ser Phe Val Thr Glu Ser Asp Asn
            1620                1625                1630
Pro Leu Leu Val Val Arg Pro Pro Gly Gly Glu Leu Ser Gly Ser His
        1635                1640                1645
Ser Leu Ser Asn Met Ile Pro Ala Thr Arg Thr Ala Met Asp Gly Asn
    1650                1655                1660
Met
1665

<210> 205
<211> 1211
<212> PRT
<213> Homo sapiens

<400> 205
Met Asp Pro Pro Ala Gly Ala Ala Arg Arg Leu Leu Cys Pro Ala Leu
1               5                   10                  15
Leu Leu Leu Leu Leu Leu Leu Pro Pro Pro Leu Leu Pro Pro Pro Pro
            20                  25                  30
Pro Pro Ala Asn Ala Arg Leu Ala Ala Ala Ala Asp Pro Pro Gly Gly
        35                  40                  45
Pro Leu Gly His Gly Ala Glu Arg Ile Leu Ala Val Pro Val Arg Thr
    50                  55                  60
Asp Ala Gln Gly Arg Leu Val Ser His Val Val Ser Ala Ala Thr Ser
65                  70                  75                  80
Arg Ala Gly Val Arg Ala Arg Arg Ala Ala Pro Val Arg Thr Pro Ser
                85                  90                  95
Phe Pro Gly Gly Asn Glu Glu Glu Pro Gly Ser His Leu Phe Tyr Asn
            100                 105                 110
Val Thr Val Phe Gly Arg Asp Leu His Leu Arg Leu Arg Pro Asn Ala
        115                 120                 125
Arg Leu Val Ala Pro Gly Ala Thr Met Glu Trp Gln Gly Glu Lys Gly
    130                 135                 140
Thr Thr Arg Val Glu Pro Leu Leu Gly Ser Cys Leu Tyr Val Gly Asp
145                 150                 155                 160
Val Ala Gly Leu Ala Glu Ala Ser Ser Val Ala Leu Ser Asn Cys Asp
                165                 170                 175
Gly Leu Ala Gly Leu Ile Arg Met Glu Glu Glu Glu Phe Phe Ile Glu
            180                 185                 190
Pro Leu Glu Lys Gly Leu Ala Ala Gln Glu Ala Glu Gln Gly Arg Val
        195                 200                 205
His Val Val Tyr Arg Arg Pro Pro Thr Ser Pro Pro Leu Gly Gly Pro
    210                 215                 220
Gln Ala Leu Asp Thr Gly Ala Ser Leu Asp Ser Leu Asp Ser Leu Ser
225                 230                 235                 240
Arg Ala Leu Gly Val Leu Glu Glu His Ala Asn Ser Ser Arg Arg Arg
                245                 250                 255
Ala Arg Arg His Ala Ala Asp Asp Asp Tyr Asn Ile Glu Val Leu Leu
            260                 265                 270
Gly Val Asp Asp Ser Val Val Gln Phe His Gly Lys Glu His Val Gln
        275                 280                 285
Lys Tyr Leu Leu Thr Leu Met Asn Ile Val Asn Glu Ile Tyr His Asp
    290                 295                 300
Glu Ser Leu Gly Ala His Ile Asn Val Val Leu Val Arg Ile Ile Leu
305                 310                 315                 320
Leu Ser Tyr Gly Lys Ser Met Ser Leu Ile Glu Ile Gly Asn Pro Ser
                325                 330                 335
Gln Ser Leu Glu Asn Val Cys Arg Trp Ala Tyr Leu Gln Gln Lys Pro
            340                 345                 350
Asp Thr Gly His Asp Glu Tyr His Asp His Ala Ile Phe Leu Thr Arg
        355                 360                 365
Gln Asp Phe Gly Pro Ser Gly Met Gln Gly Tyr Ala Pro Val Thr Gly

370                 375                 380
Met Cys His Pro Val Arg Ser Cys Thr Leu Asn His Glu Asp Gly Phe
385                 390                 395                 400
Ser Ser Ala Phe Val Val Ala His Glu Thr Gly His Val Leu Gly Met
                405                 410                 415
Glu His Asp Gly Gln Gly Asn Arg Cys Gly Asp Glu Val Arg Leu Gly
            420                 425                 430
Ser Ile Met Ala Pro Leu Val Gln Ala Ala Phe His Arg Phe His Trp
        435                 440                 445
Ser Arg Cys Ser Gln Gln Glu Leu Ser Arg Tyr Leu His Ser Tyr Asp
    450                 455                 460
Cys Leu Leu Asp Asp Pro Phe Ala His Asp Trp Pro Ala Leu Pro Gln
465                 470                 475                 480
Leu Pro Gly Leu His Tyr Ser Met Asn Glu Gln Cys Arg Phe Asp Phe
                485                 490                 495
Gly Leu Gly Tyr Met Met Cys Thr Ala Phe Arg Thr Phe Asp Pro Cys
            500                 505                 510
Lys Gln Leu Trp Cys Ser His Pro Asp Asn Pro Tyr Phe Cys Lys Thr
        515                 520                 525
Lys Lys Gly Pro Pro Leu Asp Gly Thr Met Cys Ala Pro Gly Lys His
    530                 535                 540
Cys Phe Lys Gly His Cys Ile Trp Leu Thr Pro Asp Ile Leu Lys Arg
545                 550                 555                 560
Asp Gly Ser Trp Gly Ala Trp Ser Pro Phe Gly Ser Cys Ser Arg Thr
                565                 570                 575
Cys Gly Thr Gly Val Lys Phe Arg Thr Arg Gln Cys Asp Asn Pro His
            580                 585                 590
Pro Ala Asn Gly Gly Arg Thr Cys Ser Gly Leu Ala Tyr Asp Phe Gln
        595                 600                 605
Leu Cys Ser Arg Gln Asp Cys Pro Asp Ser Leu Ala Asp Phe Arg Glu
    610                 615                 620

Glu Gln Cys Arg Gln Trp Asp Leu Tyr Phe Glu His Gly Asp Ala Gln
625                 630                 635                 640
His His Trp Leu Pro His Glu His Arg Asp Ala Lys Glu Arg Cys His
                645                 650                 655
Leu Tyr Cys Glu Ser Arg Glu Thr Gly Glu Val Val Ser Met Lys Arg
            660                 665                 670
Met Val His Asp Gly Thr Arg Cys Ser Tyr Lys Asp Ala Phe Ser Leu
        675                 680                 685
Cys Val Arg Gly Asp Cys Arg Lys Val Gly Cys Asp Gly Val Ile Gly
    690                 695                 700
Ser Ser Lys Gln Glu Asp Lys Cys Gly Val Cys Gly Gly Asp Asn Ser
705                 710                 715                 720
His Cys Lys Val Val Lys Gly Thr Phe Thr Arg Ser Pro Lys Lys His
                725                 730                 735
Gly Tyr Ile Lys Met Phe Glu Ile Pro Ala Gly Ala Arg His Leu Leu
            740                 745                 750
Ile Gln Glu Val Asp Ala Thr Ser His His Leu Ala Val Lys Asn Leu
        755                 760                 765
Glu Thr Gly Lys Phe Ile Leu Asn Glu Glu Asn Asp Val Asp Ala Ser
    770                 775                 780
Ser Lys Thr Phe Ile Ala Met Gly Val Glu Trp Glu Tyr Arg Asp Glu
785                 790                 795                 800
Asp Gly Arg Glu Thr Leu Gln Thr Met Gly Pro Leu His Gly Thr Ile
                805                 810                 815
Thr Val Leu Val Ile Pro Val Gly Asp Thr Arg Val Ser Leu Thr Tyr
            820                 825                 830
Lys Tyr Met Ile His Glu Asp Ser Leu Asn Val Asp Asp Asn Asn Val
        835                 840                 845
Leu Glu Glu Asp Ser Val Val Tyr Glu Trp Ala Leu Lys Lys Trp Ser
    850                 855                 860
Pro Cys Ser Lys Pro Cys Gly Gly Gly Ser Gln Phe Thr Lys Tyr Gly

865                 870                 875                 880
Cys Arg Arg Arg Leu Asp His Lys Met Val His Arg Gly Phe Cys Ala
                885                 890                 895
Ala Leu Ser Lys Pro Lys Ala Ile Arg Arg Ala Cys Asn Pro Gln Glu
            900                 905                 910
Cys Ser Gln Pro Val Trp Val Thr Gly Glu Trp Glu Pro Cys Ser Gln
        915                 920                 925
Thr Cys Gly Arg Thr Gly Met Gln Val Arg Ser Val Arg Cys Ile Gln
    930                 935                 940
Pro Leu His Asp Asn Thr Thr Arg Ser Val His Ala Lys His Cys Asn
945                 950                 955                 960
Asp Ala Arg Pro Glu Ser Arg Arg Ala Cys Ser Arg Glu Leu Cys Pro
                965                 970                 975
Gly Arg Trp Arg Ala Gly Pro Trp Ser Gln Cys Ser Val Thr Cys Gly
            980                 985                 990
Asn Gly Thr Gln Glu Arg Pro Val Pro Cys Arg Thr Ala Asp Asp Ser
        995                 1000                1005
Phe Gly Ile Cys Gln Glu Glu Arg Pro Glu Thr Ala Arg Thr Cys Arg
    1010                1015                1020
Leu Gly Pro Cys Pro Arg Asn Ile Ser Asp Pro Ser Lys Lys Ser Tyr
1025                1030                1035                1040
Val Val Gln Trp Leu Ser Arg Pro Asp Pro Asp Ser Pro Ile Arg Lys
                1045                1050                1055
Ile Ser Ser Lys Gly His Cys Gln Gly Asp Lys Ser Ile Phe Cys Arg
            1060                1065                1070
Met Glu Val Leu Ser Arg Tyr Cys Ser Ile Pro Gly Tyr Asn Lys Leu
        1075                1080                1085
Ser Cys Lys Ser Cys Asn Leu Tyr Asn Asn Leu Thr Asn Val Glu Gly
    1090                1095                1100

Arg Ile Glu Pro Pro Pro Gly Lys His Asn Asp Ile Asp Val Phe Met
1105                1110                1115                1120
Pro Thr Leu Pro Val Pro Thr Val Ala Met Glu Val Arg Pro Ser Pro
                1125                1130                1135
Ser Thr Pro Leu Glu Val Pro Leu Asn Ala Ser Ser Thr Asn Ala Thr
            1140                1145                1150
Glu Asp His Pro Glu Thr Asn Ala Val Asp Glu Pro Tyr Lys Ile His
        1155                1160                1165
Gly Leu Glu Asp Glu Val Gln Pro Pro Asn Leu Ile Pro Arg Arg Pro
    1170                1175                1180
Ser Pro Tyr Glu Lys Thr Arg Asn Gln Arg Ile Gln Glu Leu Ile Asp
1185                1190                1195                1200
Glu Met Arg Lys Lys Glu Met Leu Gly Lys Phe
                1205                1210

<210> 206
<211> 453
<212> PRT
<213> Homo sapiens

<400> 206
Met Gly Glu Asn Asp Pro Pro Ala Val Glu Ala Pro Phe Ser Phe Arg
1               5                   10                  15
Ser Leu Phe Gly Leu Asp Asp Leu Lys Ile Ser Pro Val Ala Pro Asp
            20                  25                  30
Ala Asp Ala Val Ala Ala Gln Ile Leu Ser Leu Leu Pro Leu Lys Phe
        35                  40                  45
Phe Pro Ile Ile Val Ile Gly Ile Ile Ala Leu Ile Leu Ala Leu Ala
    50                  55                  60
Ile Gly Leu Gly Ile His Phe Asp Cys Ser Gly Lys Tyr Arg Cys Arg
65                  70                  75                  80
Ser Ser Phe Lys Cys Ile Glu Leu Ile Ala Arg Cys Asp Gly Val Ser
                85                  90                  95

Asp Cys Lys Asp Gly Glu Asp Glu Tyr Arg Cys Val Arg Val Gly Gly
            100                 105                 110
Gln Asn Ala Val Leu Gln Val Phe Thr Ala Ala Ser Trp Lys Thr Met
        115                 120                 125
Cys Ser Asp Asp Trp Lys Gly His Tyr Ala Asn Val Ala Cys Ala Gln
    130                 135                 140
Leu Gly Phe Pro Ser Tyr Val Ser Ser Asp Asn Leu Arg Val Ser Ser
145                 150                 155                 160
Leu Glu Gly Gln Phe Arg Glu Glu Phe Val Ser Ile Asp His Leu Leu
                165                 170                 175
Pro Asp Asp Lys Val Thr Ala Leu His His Ser Val Tyr Val Arg Glu
            180                 185                 190
Gly Cys Ala Ser Gly His Val Val Thr Leu Gln Cys Thr Ala Cys Gly
        195                 200                 205
His Arg Arg Gly Tyr Ser Ser Arg Ile Val Gly Gly Asn Met Ser Leu
    210                 215                 220
Leu Ser Gln Trp Pro Trp Gln Ala Ser Leu Gln Phe Gln Gly Tyr His
225                 230                 235                 240
Leu Cys Gly Gly Ser Val Ile Thr Pro Leu Trp Ile Ile Thr Ala Ala
                245                 250                 255
His Cys Val Tyr Asp Leu Tyr Leu Pro Lys Ser Trp Thr Ile Gln Val
            260                 265                 270
Gly Leu Val Ser Leu Leu Asp Asn Pro Ala Pro Ser His Leu Val Glu
        275                 280                 285
Lys Ile Val Tyr His Ser Lys Tyr Lys Pro Lys Arg Leu Gly Asn Asp
    290                 295                 300
Ile Ala Leu Met Lys Leu Ala Gly Pro Leu Thr Phe Asn Glu Met Ile
305                 310                 315                 320
Gln Pro Val Cys Leu Pro Asn Ser Glu Glu Asn Phe Pro Asp Gly Lys
                325                 330                 335
Val Cys Trp Thr Ser Gly Trp Gly Ala Thr Glu Asp Gly Gly Asp Ala
            340                 345                 350
Ser Pro Val Leu Asn His Ala Ala Val Pro Leu Ile Ser Asn Lys Ile
        355                 360                 365
Cys Asn His Arg Asp Val Tyr Gly Gly Ile Ile Ser Pro Ser Met Leu
    370                 375                 380
Cys Ala Gly Tyr Leu Thr Gly Gly Val Asp Ser Cys Gln Gly Asp Ser
385                 390                 395                 400
Gly Gly Pro Leu Val Cys Gln Glu Arg Arg Leu Trp Lys Leu Val Gly
                405                 410                 415
Ala Thr Ser Phe Gly Ile Gly Cys Ala Glu Val Asn Lys Pro Gly Val
            420                 425                 430
Tyr Thr Arg Val Thr Ser Phe Leu Asp Trp Ile His Glu Gln Met Glu
        435                 440                 445
Arg Asp Leu Lys Thr
    450

<210> 207
<211> 677
<212> PRT
<213> Homo sapiens

<400> 207
Met Leu Ser Ser Thr Asp Phe Thr Phe Ala Ser Trp Glu Leu Val Val
1               5                   10                  15
Arg Val Asp His Pro Asn Glu Glu Gln Gln Lys Asp Val Thr Leu Arg
            20                  25                  30
Val Ser Gly Asp Leu His Val Gly Gly Val Met Leu Lys Leu Val Glu
        35                  40                  45
Gln Ile Asn Ile Ser Gln Asp Trp Ser Asp Phe Ala Leu Trp Trp Glu
    50                  55                  60
Gln Lys His Cys Trp Leu Leu Lys Thr His Trp Thr Leu Asp Lys Tyr
65                  70                  75                  80

Gly Val Gln Ala Asp Ala Lys Leu Leu Phe Thr Pro Gln His Lys Met
                85                  90                  95
Leu Arg Leu Arg Leu Pro Asn Leu Lys Met Val Arg Leu Arg Val Ser
            100                 105                 110
Phe Ser Ala Val Val Phe Lys Ala Val Ser Asp Ile Cys Lys Ile Leu
        115                 120                 125
Asn Ile Arg Arg Ser Glu Glu Leu Ser Leu Leu Lys Pro Ser Gly Asp
    130                 135                 140
Tyr Phe Lys Lys Lys Lys Lys Lys Asp Lys Asn Asn Lys Glu Pro Ile
145                 150                 155                 160
Ile Glu Asp Ile Leu Asn Leu Glu Ser Ser Pro Thr Ala Ser Gly Ser
                165                 170                 175
Ser Val Ser Pro Gly Leu Tyr Ser Lys Thr Met Thr Pro Ile Tyr Asp
            180                 185                 190
Pro Ile Asn Gly Thr Pro Ala Ser Ser Thr Met Thr Trp Phe Ser Asp
        195                 200                 205
Ser Pro Leu Thr Glu Gln Asn Cys Ser Ile Leu Ala Phe Ser Gln Pro
    210                 215                 220
Pro Gln Ser Pro Glu Ala Leu Ala Asp Met Tyr Gln Pro Arg Ser Leu
225                 230                 235                 240
Val Asp Lys Ala Lys Leu Asn Ala Gly Trp Leu Asp Ser Ser Arg Ser
                245                 250                 255
Leu Met Glu Gln Gly Ile Gln Glu Asp Glu Gln Leu Leu Leu Arg Phe
            260                 265                 270
Lys Tyr Tyr Ser Phe Phe Asp Leu Asn Pro Lys Tyr Asp Ala Val Arg
        275                 280                 285
Ile Asn Gln Leu Tyr Glu Gln Ala Arg Trp Ala Ile Leu Leu Glu Glu
    290                 295                 300
Ile Asp Cys Thr Glu Glu Glu Met Leu Ile Phe Ala Ala Leu Gln Tyr
305                 310                 315                 320
His Ile Ser Lys Leu Ser Leu Ser Ala Glu Thr Gln Asp Phe Ala Gly
                325                 330                 335
Glu Ser Glu Val Asp Glu Ile Glu Ala Ala Leu Ser Asn Leu Glu Val
            340                 345                 350
Thr Leu Glu Gly Gly Lys Ala Asp Ser Leu Leu Glu Asp Ile Thr Asp
        355                 360                 365
Ile Pro Lys Leu Ala Asp Asn Leu Lys Leu Phe Arg Pro Lys Lys Leu
    370                 375                 380
Leu Pro Lys Ala Phe Lys Gln Tyr Trp Phe Ile Phe Lys Asp Thr Ser
385                 390                 395                 400
Ile Ala Tyr Phe Lys Asn Lys Glu Leu Glu Gln Gly Glu Pro Leu Glu
                405                 410                 415
Lys Leu Asn Leu Arg Gly Cys Glu Val Val Pro Asp Val Asn Val Ala
            420                 425                 430
Gly Arg Lys Phe Gly Ile Lys Leu Leu Ile Pro Val Ala Asp Gly Met
        435                 440                 445
Asn Glu Met Tyr Leu Arg Cys Asp His Glu Asn Gln Tyr Ala Gln Trp
    450                 455                 460
Met Ala Ala Cys Met Leu Ala Ser Lys Gly Lys Thr Met Ala Asp Ser
465                 470                 475                 480
Ser Tyr Gln Pro Glu Val Leu Asn Ile Leu Ser Phe Leu Arg Met Lys
                485                 490                 495
Asn Arg Asn Ser Ala Ser Gln Val Ala Ser Ser Leu Glu Asn Met Asp
            500                 505                 510
Met Asn Pro Glu Cys Phe Val Ser Pro Arg Cys Ala Lys Lys His Lys
        515                 520                 525
Ser Lys Gln Leu Ala Ala Arg Ile Leu Glu Ala His Gln Asn Val Ala
    530                 535                 540
Gln Met Pro Leu Val Glu Ala Lys Leu Arg Phe Ile Gln Ala Trp Gln
545                 550                 555                 560
Ser Leu Pro Glu Phe Gly Leu Thr Tyr Tyr Leu Val Arg Phe Lys Gly
                565                 570                 575
Ser Lys Lys Asp Asp Ile Leu Gly Val Ser Tyr Asn Arg Leu Ile Lys

                580                 585                 590
Ile Asp Ala Ala Thr Gly Ile Pro Val Thr Thr Trp Arg Phe Thr Asn
        595                 600                 605
Ile Lys Gln Trp Asn Val Asn Trp Glu Thr Arg Gln Val Val Ile Glu
    610                 615                 620
Phe Asp Gln Asn Val Phe Thr Ala Phe Thr Cys Leu Ser Ala Asp Cys
625                 630                 635                 640
Lys Ile Val His Glu Tyr Ile Gly Gly Tyr Ile Phe Leu Ser Thr Arg
                645                 650                 655
Ser Lys Asp Gln Asn Glu Thr Leu Asp Glu Asp Leu Phe His Lys Leu
            660                 665                 670
Thr Gly Gly Gln Asp
        675

<210> 208
<211> 158
<212> PRT
<213> Homo sapiens

<400> 208
Met Ala Ser Arg Ser Met Arg Leu Leu Leu Leu Leu Ser Cys Leu Ala
1               5                   10                  15
Lys Thr Gly Val Leu Gly Asp Ile Ile Met Arg Pro Ser Cys Ala Pro
            20                  25                  30
Gly Trp Phe Tyr His Lys Ser Asn Cys Tyr Gly Tyr Phe Arg Lys Leu
        35                  40                  45
Arg Asn Trp Ser Asp Ala Glu Leu Glu Cys Gln Ser Tyr Gly Asn Gly
    50                  55                  60
Ala His Leu Ala Ser Ile Leu Ser Leu Lys Glu Ala Ser Thr Ile Ala
65                  70                  75                  80
Glu Tyr Ile Ser Gly Tyr Gln Arg Ser Gln Pro Ile Trp Ile Gly Leu
                85                  90                  95
His Asp Pro Gln Lys Arg Gln Gln Trp Gln Trp Ile Asp Gly Ala Met
            100                 105                 110
Tyr Leu Tyr Arg Ser Trp Ser Gly Lys Ser Met Gly Gly Asn Lys His
        115                 120                 125
Cys Ala Glu Met Ser Ser Asn Asn Asn Phe Leu Thr Trp Ser Ser Asn
    130                 135                 140
Glu Cys Asn Lys Arg Gln His Phe Leu Cys Lys Tyr Arg Pro
145                 150                 155

<210> 209
<211> 1361
<212> PRT
<213> Homo sapiens

<400> 209
Met Gly Ala Ala Gly Arg Gln Asp Phe Leu Phe Lys Ala Met Leu Thr
1               5                   10                  15
Ile Ser Trp Leu Thr Leu Thr Cys Phe Pro Gly Ala Thr Ser Thr Val
            20                  25                  30
Ala Ala Gly Cys Pro Asp Gln Ser Pro Glu Leu Gln Pro Trp Asn Pro
        35                  40                  45
Gly His Asp Gln Asp His His Val His Ile Gly Gln Gly Lys Thr Leu
    50                  55                  60
Leu Leu Thr Ser Ser Ala Thr Val Tyr Ser Ile His Ile Ser Glu Gly
65                  70                  75                  80
Gly Lys Leu Val Ile Lys Asp His Asp Glu Pro Ile Val Leu Arg Thr
                85                  90                  95
Arg His Ile Leu Ile Asp Asn Gly Gly Glu Leu His Ala Gly Ser Ala
            100                 105                 110
Leu Cys Pro Phe Gln Gly Asn Phe Thr Ile Ile Leu Tyr Gly Arg Ala
        115                 120                 125

Asp Glu Gly Ile Gln Pro Asp Pro Tyr Tyr Gly Leu Lys Tyr Ile Gly
    130                 135                 140
Val Gly Lys Gly Gly Ala Leu Glu Leu His Gly Gln Lys Lys Leu Ser
145                 150                 155                 160
Trp Thr Phe Leu Asn Lys Thr Leu His Pro Gly Gly Met Ala Glu Gly
                165                 170                 175
Gly Tyr Phe Phe Glu Arg Ser Trp Gly His Arg Gly Val Ile Val His
            180                 185                 190
Val Ile Asp Pro Lys Ser Gly Thr Val Ile His Ser Asp Arg Phe Asp
        195                 200                 205
Thr Tyr Arg Ser Lys Lys Glu Ser Glu Arg Leu Val Gln Tyr Leu Asn
    210                 215                 220
Ala Val Pro Asp Gly Arg Ile Leu Ser Val Ala Val Asn Asp Glu Gly
225                 230                 235                 240
Ser Arg Asn Leu Asp Asp Met Ala Arg Lys Ala Met Thr Lys Leu Gly
                245                 250                 255
Ser Lys His Phe Leu His Leu Gly Phe Arg His Pro Trp Ser Phe Leu
            260                 265                 270
Thr Val Lys Gly Asn Pro Ser Ser Ser Val Glu Asp His Ile Glu Tyr
        275                 280                 285
His Gly His Arg Gly Ser Ala Ala Ala Arg Val Phe Lys Leu Phe Gln
    290                 295                 300
Thr Glu His Gly Glu Tyr Phe Asn Val Ser Leu Ser Ser Glu Trp Val
305                 310                 315                 320
Gln Asp Val Glu Trp Thr Glu Trp Phe Asp His Asp Lys Val Ser Gln
                325                 330                 335
Thr Lys Gly Gly Glu Lys Ile Ser Asp Leu Trp Lys Ala His Pro Gly
            340                 345                 350
Lys Ile Cys Asn Arg Pro Ile Asp Ile Gln Ala Thr Thr Met Asp Gly
        355                 360                 365
Val Asn Leu Ser Thr Glu Val Val Tyr Lys Lys Gly Gln Asp Tyr Arg
    370                 375                 380
Phe Ala Cys Tyr Asp Arg Gly Arg Ala Cys Arg Ser Tyr Arg Val Arg
385                 390                 395                 400
Phe Leu Cys Gly Lys Pro Val Arg Pro Lys Leu Thr Val Thr Ile Asp
                405                 410                 415
Thr Asn Val Asn Ser Thr Ile Leu Asn Leu Glu Asp Asn Val Gln Ser
            420                 425                 430
Trp Lys Pro Gly Asp Thr Leu Val Ile Ala Ser Thr Asp Tyr Ser Met
        435                 440                 445
Tyr Gln Ala Glu Glu Phe Gln Val Leu Pro Cys Arg Ser Cys Ala Pro
    450                 455                 460
Asn Gln Val Lys Val Ala Gly Lys Pro Met Tyr Leu His Ile Gly Glu
465                 470                 475                 480
Glu Ile Asp Gly Val Asp Met Arg Ala Glu Val Gly Leu Leu Ser Arg
                485                 490                 495
Asn Ile Ile Val Met Gly Glu Met Glu Asp Lys Cys Tyr Pro Tyr Arg
            500                 505                 510
Asn His Ile Cys Asn Phe Phe Asp Phe Asp Thr Phe Gly Gly His Ile
        515                 520                 525
Lys Phe Ala Leu Gly Phe Lys Ala Ala His Leu Glu Gly Thr Glu Leu
    530                 535                 540
Lys His Met Gly Gln Gln Leu Val Gly Gln Tyr Pro Ile His Phe His
545                 550                 555                 560
Leu Ala Gly Asp Val Asp Glu Arg Gly Gly Tyr Asp Pro Pro Thr Tyr
                565                 570                 575
Ile Arg Asp Leu Ser Ile His His Thr Phe Ser Arg Cys Val Thr Val
            580                 585                 590
His Gly Ser Asn Gly Leu Leu Ile Lys Asp Val Val Gly Tyr Asn Ser
        595                 600                 605
Leu Gly His Cys Phe Phe Thr Glu Asp Gly Pro Glu Glu Arg Asn Thr
    610                 615                 620
Phe Asp His Cys Leu Gly Leu Leu Val Lys Ser Gly Thr Leu Leu Pro

625                 630                 635                 640
Ser Asp Arg Asp Ser Lys Met Cys Lys Met Ile Thr Glu Asp Ser Tyr
                645                 650                 655
Pro Gly Tyr Ile Pro Lys Pro Arg Gln Asp Cys Asn Ala Val Ser Thr
            660                 665                 670
Phe Trp Met Ala Asn Pro Asn Asn Asn Leu Ile Asn Cys Ala Ala Ala
        675                 680                 685
Gly Ser Glu Glu Thr Gly Phe Trp Phe Ile Phe His His Val Pro Thr
    690                 695                 700
Gly Pro Ser Val Gly Met Tyr Ser Pro Gly Tyr Ser Glu His Ile Pro
705                 710                 715                 720
Leu Gly Lys Phe Tyr Asn Asn Arg Ala His Ser Asn Tyr Arg Ala Gly
                725                 730                 735
Met Ile Ile Asp Asn Gly Val Lys Thr Thr Glu Ala Ser Ala Lys Asp
            740                 745                 750
Lys Arg Pro Phe Leu Ser Ile Ile Ser Ala Arg Tyr Ser Pro His Gln
        755                 760                 765
Asp Ala Asp Pro Leu Lys Pro Arg Glu Pro Ala Ile Ile Arg His Phe
    770                 775                 780

Ile Ala Tyr Lys Asn Gln Asp His Gly Ala Trp Leu Arg Gly Gly Asp
785                 790                 795                 800
Val Trp Leu Asp Ser Cys Arg Phe Ala Asp Asn Gly Ile Gly Leu Thr
                805                 810                 815
Leu Ala Ser Gly Gly Thr Phe Pro Tyr Asp Asp Gly Ser Lys Gln Glu
            820                 825                 830
Ile Lys Asn Ser Leu Phe Val Gly Glu Ser Gly Asn Val Gly Thr Glu
        835                 840                 845
Met Met Asp Asn Arg Ile Trp Gly Pro Gly Gly Leu Asp His Ser Gly
    850                 855                 860
Arg Thr Leu Pro Ile Gly Gln Asn Phe Pro Ile Arg Gly Ile Gln Leu
865                 870                 875                 880
Tyr Asp Gly Pro Ile Asn Ile Gln Asn Cys Thr Phe Arg Lys Phe Val
                885                 890                 895
Ala Leu Glu Gly Arg His Thr Ser Ala Leu Ala Phe Arg Leu Asn Asn
            900                 905                 910
Ala Trp Gln Ser Cys Pro His Asn Asn Val Thr Gly Ile Ala Phe Glu
        915                 920                 925
Asp Val Pro Ile Thr Ser Arg Val Phe Phe Gly Glu Pro Gly Pro Trp
    930                 935                 940
Phe Asn Gln Leu Asp Met Asp Gly Asp Lys Thr Ser Val Phe His Asp
945                 950                 955                 960
Val Asp Gly Ser Val Ser Glu Tyr Pro Gly Ser Tyr Leu Thr Lys Asn
                965                 970                 975
Asp Asn Trp Leu Val Arg His Pro Asp Cys Ile Asn Val Pro Asp Trp
            980                 985                 990
Arg Gly Ala Ile Cys Ser Gly Cys Tyr Ala Gln Met Tyr Ile Gln Ala
        995                 1000                1005
Tyr Lys Thr Ser Asn Leu Arg Met Lys Ile Ile Lys Asn Asp Phe Pro
    1010                1015                1020
Ser His Pro Leu Tyr Leu Glu Gly Ala Leu Thr Arg Ser Thr His Tyr
1025                1030                1035                1040
Gln Gln Tyr Gln Pro Val Val Thr Leu Gln Lys Gly Tyr Thr Ile His
                1045                1050                1055
Trp Asp Gln Thr Ala Pro Ala Glu Leu Ala Ile Trp Leu Ile Asn Phe
            1060                1065                1070
Asn Lys Gly Asp Trp Ile Arg Val Gly Leu Cys Tyr Pro Arg Gly Thr
        1075                1080                1085
Thr Phe Ser Ile Leu Ser Asp Val His Asn Arg Leu Leu Lys Gln Thr
    1090                1095                1100
Ser Lys Thr Gly Val Phe Val Arg Thr Leu Gln Met Asp Lys Val Glu
1105                1110                1115                1120
Gln Ser Tyr Pro Gly Arg Ser His Tyr Tyr Trp Asp Glu Asp Ser Gly

1125                1130                1135
Leu Leu Phe Leu Lys Leu Lys Ala Gln Asn Glu Arg Glu Lys Phe Ala
        1140                1145                1150
Phe Cys Ser Met Lys Gly Cys Glu Arg Ile Lys Ile Lys Ala Leu Ile
    1155                1160                1165
Pro Lys Asn Ala Gly Val Ser Asp Cys Thr Ala Thr Ala Tyr Pro Lys
1170                1175                1180
Phe Thr Glu Arg Ala Val Val Asp Val Pro Met Pro Lys Lys Leu Phe
1185                1190                1195                1200
Gly Ser Gln Leu Lys Thr Lys Asp His Phe Leu Glu Val Lys Met Glu
                1205                1210                1215
Ser Ser Lys Gln His Phe Phe His Leu Trp Asn Asp Phe Ala Tyr Ile
        1220                1225                1230
Glu Val Asp Gly Lys Lys Tyr Pro Ser Ser Glu Asp Gly Ile Gln Val
    1235                1240                1245
Val Val Ile Asp Gly Asn Gln Gly Arg Val Val Ser His Thr Ser Phe
1250                1255                1260

Arg Asn Ser Ile Leu Gln Gly Ile Pro Trp Gln Leu Phe Asn Tyr Val
1265                1270                1275                1280
Ala Thr Ile Pro Asp Asn Ser Ile Val Leu Met Ala Ser Lys Gly Arg
                1285                1290                1295
Tyr Val Ser Arg Gly Pro Trp Thr Arg Val Leu Glu Lys Leu Gly Ala
        1300                1305                1310
Asp Arg Gly Leu Lys Leu Lys Glu Gln Met Ala Phe Val Gly Phe Lys
    1315                1320                1325
Gly Ser Phe Arg Pro Ile Trp Val Thr Leu Asp Thr Glu Asp His Lys
1330                1335                1340
Ala Lys Ile Phe Gln Val Val Pro Ile Pro Val Val Lys Lys Lys Lys
1345                1350                1355                1360
Leu

<210> 210
<211> 747
<212> PRT
<213> Homo sapiens

<400> 210
Met Ser Gln Val Lys Ser Ser Tyr Ser Tyr Asp Ala Pro Ser Asp Phe
1               5                   10                  15
Ile Asn Phe Ser Ser Leu Asp Asp Glu Gly Asp Thr Gln Asn Ile Asp
            20                  25                  30
Ser Trp Phe Glu Glu Lys Ala Asn Leu Glu Asn Lys Leu Leu Gly Lys
        35                  40                  45
Asn Gly Thr Gly Gly Leu Phe Gln Gly Lys Thr Pro Leu Arg Lys Ala
    50                  55                  60
Asn Leu Gln Gln Ala Ile Val Thr Pro Leu Lys Pro Val Asp Asn Thr
65                  70                  75                  80
Tyr Tyr Lys Glu Ala Glu Lys Glu Asn Leu Val Glu Gln Ser Ile Pro
                85                  90                  95
Ser Asn Ala Cys Ser Ser Leu Glu Val Glu Ala Ala Ile Ser Arg Lys
            100                 105                 110
Thr Pro Ala Gln Pro Gln Arg Arg Ser Leu Arg Leu Ser Ala Gln Lys
        115                 120                 125
Asp Leu Glu Gln Lys Glu Lys His His Val Lys Met Lys Ala Lys Arg
    130                 135                 140
Cys Ala Thr Pro Val Ile Ile Asp Glu Ile Leu Pro Ser Lys Lys Met
145                 150                 155                 160
Lys Val Ser Asn Asn Lys Lys Lys Pro Glu Glu Glu Gly Ser Ala His
                165                 170                 175
Gln Asp Thr Ala Glu Lys Asn Ala Ser Ser Pro Glu Lys Ala Lys Gly
            180                 185                 190
Arg His Thr Val Pro Cys Met Pro Pro Ala Lys Gln Lys Phe Leu Lys

195                 200                 205
Ser Thr Glu Glu Gln Glu Leu Glu Lys Ser Met Lys Met Gln Gln Glu
    210                 215                 220
Val Val Glu Met Arg Lys Lys Asn Glu Glu Phe Lys Lys Leu Ala Leu
225                 230                 235                 240
Ala Gly Ile Gly Gln Pro Val Lys Lys Ser Val Ser Gln Val Thr Lys
                245                 250                 255
Ser Val Asp Phe His Phe Arg Thr Asp Glu Arg Ile Lys Gln His Pro
            260                 265                 270
Lys Asn Gln Glu Glu Tyr Lys Glu Val Asn Phe Thr Ser Glu Leu Arg
        275                 280                 285
Lys His Pro Ser Ser Pro Ala Arg Val Thr Lys Gly Cys Thr Ile Val
    290                 295                 300
Lys Pro Phe Asn Leu Ser Gln Gly Lys Lys Arg Thr Phe Asp Glu Thr
305                 310                 315                 320

Val Ser Thr Tyr Val Pro Leu Ala Gln Gln Val Glu Asp Phe His Lys
                325                 330                 335
Arg Thr Pro Asn Arg Tyr His Leu Arg Ser Lys Lys Asp Asp Ile Asn
            340                 345                 350
Leu Leu Pro Ser Lys Ser Ser Val Thr Lys Ile Cys Arg Asp Pro Gln
        355                 360                 365
Thr Pro Val Leu Gln Thr Lys His Arg Ala Arg Ala Val Thr Cys Lys
    370                 375                 380
Ser Thr Ala Glu Leu Glu Ala Glu Glu Leu Glu Lys Leu Gln Gln Tyr
385                 390                 395                 400
Lys Phe Lys Ala Arg Glu Leu Asp Pro Arg Ile Leu Glu Gly Gly Pro
                405                 410                 415
Ile Leu Pro Lys Lys Pro Pro Val Lys Pro Pro Thr Glu Pro Ile Gly
            420                 425                 430
Phe Asp Leu Glu Ile Glu Lys Arg Ile Gln Glu Arg Glu Ser Lys Lys
        435                 440                 445
Lys Thr Glu Asp Glu His Phe Glu Phe His Ser Arg Pro Cys Pro Thr
    450                 455                 460
Lys Ile Leu Glu Asp Val Val Gly Val Pro Glu Lys Lys Val Leu Pro
465                 470                 475                 480
Ile Thr Val Pro Lys Ser Pro Ala Phe Ala Leu Lys Asn Arg Ile Arg
                485                 490                 495
Met Pro Thr Lys Glu Asp Glu Glu Glu Asp Glu Pro Val Val Ile Lys
            500                 505                 510
Ala Gln Pro Val Pro His Tyr Gly Val Pro Phe Lys Pro Gln Ile Pro
        515                 520                 525
Glu Ala Arg Thr Val Glu Ile Cys Pro Phe Ser Phe Asp Ser Arg Asp
    530                 535                 540
Lys Glu Arg Gln Leu Gln Lys Glu Lys Lys Ile Lys Glu Leu Gln Lys
545                 550                 555                 560
Gly Glu Val Pro Lys Phe Lys Ala Leu Pro Leu Pro His Phe Asp Thr
                565                 570                 575
Ile Asn Leu Pro Glu Lys Lys Val Lys Asn Val Thr Gln Ile Glu Pro
            580                 585                 590
Phe Cys Leu Glu Thr Asp Arg Arg Gly Ala Leu Lys Ala Gln Thr Trp
        595                 600                 605
Lys His Gln Leu Glu Glu Glu Leu Arg Gln Gln Lys Glu Ala Ala Cys
    610                 615                 620
Phe Lys Ala Arg Pro Asn Thr Val Ile Ser Gln Glu Pro Phe Val Pro
625                 630                 635                 640
Lys Lys Glu Lys Lys Ser Val Ala Glu Gly Leu Ser Gly Ser Leu Val
                645                 650                 655
Gln Glu Pro Phe Gln Leu Ala Thr Glu Lys Arg Ala Lys Glu Arg Gln
            660                 665                 670
Glu Leu Glu Lys Arg Met Ala Glu Val Glu Ala Gln Lys Ala Gln Gln
        675                 680                 685
Leu Glu Glu Ala Arg Leu Gln Glu Glu Glu Gln Lys Lys Glu Glu Leu

690                 695                 700
Ala Arg Leu Arg Arg Glu Leu Val His Lys Ala Asn Pro Ile Arg Lys
705                 710                 715                 720
Tyr Gln Gly Leu Glu Ile Lys Ser Ser Asp Gln Pro Leu Thr Val Pro
                725                 730                 735
Val Ser Pro Lys Phe Ser Thr Arg Phe His Cys
            740                 745

<210> 211
<211> 1053
<212> PRT
<213> Homo sapiens

<400> 211
Met Ser Arg Arg Lys Gln Ala Lys Pro Gln His Ile Asn Ser Glu Glu
1               5                   10                  15
Asp Gln Gly Glu Gln Gln Pro Gln Gln Gln Thr Pro Glu Phe Ala Asp
            20                  25                  30
Ala Ala Pro Ala Ala Pro Ala Ala Gly Glu Leu Gly Ala Pro Val Asn
        35                  40                  45
His Pro Gly Asn Asp Glu Val Ala Ser Glu Asp Glu Ala Thr Val Lys
    50                  55                  60
Arg Leu Arg Arg Glu Glu Thr His Val Cys Glu Lys Cys Cys Ala Glu
65                  70                  75                  80
Phe Phe Ser Ile Ser Glu Phe Leu Glu His Lys Lys Asn Cys Thr Lys
                85                  90                  95
Asn Pro Pro Val Leu Ile Met Asn Asp Ser Glu Gly Pro Val Pro Ser
            100                 105                 110
Glu Asp Phe Ser Gly Ala Val Leu Ser His Gln Pro Thr Ser Pro Gly
        115                 120                 125
Ser Lys Asp Cys His Arg Glu Asn Gly Gly Ser Ser Glu Asp Met Lys
    130                 135                 140
Glu Lys Pro Asp Ala Glu Ser Val Val Tyr Leu Lys Thr Glu Thr Ala
145                 150                 155                 160
Leu Pro Pro Thr Pro Gln Asp Ile Ser Tyr Leu Ala Lys Gly Lys Val
                165                 170                 175
Ala Asn Thr Asn Val Thr Leu Gln Ala Leu Arg Gly Thr Lys Val Ala
            180                 185                 190
Val Asn Gln Arg Ser Ala Asp Ala Leu Pro Ala Pro Val Pro Gly Ala
        195                 200                 205
Asn Ser Ile Pro Trp Val Leu Glu Gln Ile Leu Cys Leu Gln Gln Gln
    210                 215                 220
Gln Leu Gln Gln Ile Gln Leu Thr Glu Gln Ile Arg Ile Gln Val Asn
225                 230                 235                 240
Met Trp Ala Ser His Ala Leu His Ser Ser Gly Ala Gly Ala Asp Thr
                245                 250                 255
Leu Lys Thr Leu Gly Ser His Met Ser Gln Gln Val Ser Ala Ala Val
            260                 265                 270
Ala Leu Leu Ser Gln Lys Ala Gly Ser Gln Gly Leu Ser Leu Asp Ala
        275                 280                 285
Leu Lys Gln Ala Lys Leu Pro His Ala Asn Ile Pro Ser Ala Thr Ser
    290                 295                 300
Ser Leu Ser Pro Gly Leu Ala Pro Phe Thr Leu Lys Pro Asp Gly Thr
305                 310                 315                 320
Arg Val Leu Pro Asn Val Met Ser Arg Leu Pro Ser Ala Leu Leu Pro
                325                 330                 335
Gln Ala Pro Gly Ser Val Leu Phe Gln Ser Pro Phe Ser Thr Val Ala
            340                 345                 350
Leu Asp Thr Ser Lys Lys Gly Lys Gly Lys Pro Pro Asn Ile Ser Ala
        355                 360                 365
Val Asp Val Lys Pro Lys Asp Glu Ala Ala Leu Tyr Lys His Lys Cys
    370                 375                 380

Lys Tyr Cys Ser Lys Val Phe Gly Thr Asp Ser Ser Leu Gln Ile His
385                 390                 395                 400
Leu Arg Ser His Thr Gly Glu Arg Pro Phe Val Cys Ser Val Cys Gly
                405                 410                 415
His Arg Phe Thr Thr Lys Gly Asn Leu Lys Val His Phe His Arg His
            420                 425                 430
Pro Gln Val Lys Ala Asn Pro Gln Leu Phe Ala Glu Phe Gln Asp Lys
        435                 440                 445
Val Ala Ala Gly Asn Gly Ile Pro Tyr Ala Leu Ser Val Pro Asp Pro
    450                 455                 460
Ile Asp Glu Pro Ser Leu Ser Leu Asp Ser Lys Pro Val Leu Val Thr
465                 470                 475                 480
Thr Ser Val Gly Leu Pro Gln Asn Leu Ser Ser Gly Thr Asn Pro Lys
                485                 490                 495
Asp Leu Thr Gly Gly Ser Leu Pro Gly Asp Leu Gln Pro Gly Pro Ser
            500                 505                 510
Pro Glu Ser Glu Gly Gly Pro Thr Leu Pro Gly Val Gly Pro Asn Tyr
        515                 520                 525
Asn Ser Pro Arg Ala Gly Gly Phe Gln Gly Ser Gly Thr Pro Glu Pro
    530                 535                 540
Gly Ser Glu Thr Leu Lys Leu Gln Gln Leu Val Glu Asn Ile Asp Lys
545                 550                 555                 560
Ala Thr Thr Asp Pro Asn Glu Cys Leu Ile Cys His Arg Val Leu Ser
                565                 570                 575
Cys Gln Ser Ser Leu Lys Met His Tyr Arg Thr His Thr Gly Glu Arg
            580                 585                 590
Pro Phe Gln Cys Lys Ile Cys Gly Arg Ala Phe Ser Thr Lys Gly Asn
        595                 600                 605
Leu Lys Thr His Leu Gly Val His Arg Thr Asn Thr Ser Ile Lys Thr
    610                 615                 620
Gln His Ser Cys Pro Ile Cys Gln Lys Lys Phe Thr Asn Ala Val Met
625                 630                 635                 640
Leu Gln Gln His Ile Arg Met His Met Gly Gly Gln Ile Pro Asn Thr
                645                 650                 655
Pro Leu Pro Glu Asn Pro Cys Asp Phe Thr Gly Ser Glu Pro Met Thr
            660                 665                 670
Val Gly Glu Asn Gly Ser Thr Gly Ala Ile Cys His Asp Asp Val Ile
        675                 680                 685
Glu Ser Ile Asp Val Glu Glu Val Ser Ser Gln Glu Ala Pro Ser Ser
    690                 695                 700
Ser Ser Lys Val Pro Thr Pro Leu Pro Ser Ile His Ser Ala Ser Pro
705                 710                 715                 720
Thr Leu Gly Phe Ala Met Met Ala Ser Leu Asp Ala Pro Gly Lys Val
                725                 730                 735
Gly Pro Ala Pro Phe Asn Leu Gln Arg Gln Gly Ser Arg Glu Asn Gly
            740                 745                 750
Ser Val Glu Ser Asp Gly Leu Thr Asn Asp Ser Ser Ser Leu Met Gly
        755                 760                 765
Asp Gln Glu Tyr Gln Ser Arg Ser Pro Asp Ile Leu Glu Thr Thr Ser
    770                 775                 780
Phe Gln Ala Leu Ser Pro Ala Asn Ser Gln Ala Glu Ser Ile Lys Ser
785                 790                 795                 800
Lys Ser Pro Asp Ala Gly Ser Lys Ala Glu Ser Ser Glu Asn Ser Arg
                805                 810                 815
Thr Glu Met Glu Gly Arg Ser Ser Leu Pro Ser Thr Phe Ile Arg Ala
            820                 825                 830
Pro Pro Thr Tyr Val Lys Val Glu Val Pro Gly Thr Phe Val Gly Pro
        835                 840                 845
Ser Thr Leu Ser Pro Gly Met Thr Pro Leu Leu Ala Ala Gln Pro Arg
    850                 855                 860
Arg Gln Ala Lys Gln His Gly Cys Thr Arg Cys Gly Lys Asn Phe Ser
865                 870                 875                 880
Ser Ala Ser Ala Leu Gln Ile His Glu Arg Thr His Thr Gly Glu Lys

                885                 890                 895
Pro Phe Val Cys Asn Ile Cys Gly Arg Ala Phe Thr Thr Lys Gly Asn
            900                 905                 910
Leu Lys Val His Tyr Met Thr His Gly Ala Asn Asn Asn Ser Ala Arg
        915                 920                 925
Arg Gly Arg Lys Leu Ala Ile Glu Asn Thr Met Ala Leu Leu Gly Thr
    930                 935                 940
Asp Gly Lys Arg Val Ser Glu Ile Phe Pro Lys Glu Ile Leu Ala Pro
945                 950                 955                 960

Ser Val Asn Val Asp Pro Val Val Trp Asn Gln Tyr Thr Ser Met Leu
                965                 970                 975
Asn Gly Gly Leu Ala Val Lys Thr Asn Glu Ile Ser Val Ile Gln Ser
            980                 985                 990
Gly Gly Val Pro Thr Leu Pro Val Ser Leu Gly Ala Thr Ser Val Val
        995                 1000                1005
Asn Asn Ala Thr Val Ser Lys Met Asp Gly Ser Gln Ser Gly Ile Ser
    1010                1015                1020
Ala Asp Val Glu Lys Pro Ser Ala Thr Asp Gly Val Pro Lys His Gln
1025                1030                1035                1040
Phe Pro His Phe Leu Glu Glu Asn Lys Ile Ala Val Ser
                1045                1050

<210> 212
<211> 165
<212> PRT
<213> Homo sapiens

<400> 212
Met Gly Trp Asp Leu Thr Val Lys Met Leu Ala Gly Asn Glu Phe Gln
1               5                   10                  15
Val Ser Leu Ser Ser Ser Met Ser Val Ser Glu Leu Lys Ala Gln Ile
            20                  25                  30
Thr Gln Lys Ile Gly Val His Ala Phe Gln Gln Arg Leu Ala Val His
        35                  40                  45
Pro Ser Gly Val Ala Leu Gln Asp Arg Val Pro Leu Ala Ser Gln Gly
    50                  55                  60
Leu Gly Pro Gly Ser Thr Val Leu Leu Val Val Asp Lys Cys Asp Glu
65                  70                  75                  80
Pro Leu Ser Ile Leu Val Arg Asn Asn Lys Gly Arg Ser Ser Thr Tyr
                85                  90                  95
Glu Val Arg Leu Thr Gln Thr Val Ala His Leu Lys Gln Gln Val Ser
            100                 105                 110
Gly Leu Glu Gly Val Gln Asp Asp Leu Phe Trp Leu Thr Phe Glu Gly
        115                 120                 125
Lys Pro Leu Glu Asp Gln Leu Pro Leu Gly Glu Tyr Gly Leu Lys Pro
    130                 135                 140
Leu Ser Thr Val Phe Met Asn Leu Arg Leu Arg Gly Gly Gly Thr Glu
145                 150                 155                 160
Pro Gly Gly Arg Ser
                165

<210> 213
<211> 277
<212> PRT
<213> Homo sapiens

<400> 213
Met Ile Ile Leu Ile Tyr Leu Phe Leu Leu Leu Trp Glu Asp Thr Gln
1               5                   10                  15
Gly Trp Gly Phe Lys Asp Gly Ile Phe His Asn Ser Ile Trp Leu Glu
            20                  25                  30
Arg Ala Ala Gly Val Tyr His Arg Glu Ala Arg Ser Gly Lys Tyr Lys

35                  40                  45
Leu Thr Tyr Ala Glu Ala Lys Ala Val Cys Glu Phe Glu Gly Gly His
    50                  55                  60
Leu Ala Thr Tyr Lys Gln Leu Glu Ala Ala Arg Lys Ile Gly Phe His
65                  70                  75                  80
Val Cys Ala Ala Gly Trp Met Ala Lys Gly Arg Val Gly Tyr Pro Ile
                85                  90                  95

Val Lys Pro Gly Pro Asn Cys Gly Phe Gly Lys Thr Gly Ile Ile Asp
            100                 105                 110
Tyr Gly Ile Arg Leu Asn Arg Ser Glu Arg Trp Asp Ala Tyr Cys Tyr
        115                 120                 125
Asn Pro His Ala Lys Glu Cys Gly Gly Val Phe Thr Asp Pro Lys Gln
    130                 135                 140
Ile Phe Lys Ser Pro Gly Phe Pro Asn Glu Tyr Glu Asp Asn Gln Ile
145                 150                 155                 160
Cys Tyr Trp His Ile Arg Leu Lys Tyr Gly Gln Arg Ile His Leu Ser
                165                 170                 175
Phe Leu Asp Phe Asp Leu Glu Asp Asp Pro Gly Cys Leu Ala Asp Tyr
            180                 185                 190
Val Glu Ile Tyr Asp Ser Tyr Asp Asp Val His Gly Phe Val Gly Arg
        195                 200                 205
Tyr Cys Gly Asp Glu Leu Pro Asp Asp Ile Ile Ser Thr Gly Asn Val
    210                 215                 220
Met Thr Leu Lys Phe Leu Ser Asp Ala Ser Val Thr Ala Gly Gly Phe
225                 230                 235                 240
Gln Ile Lys Tyr Val Ala Met Asp Pro Val Ser Lys Ser Ser Gln Gly
                245                 250                 255
Lys Asn Thr Ser Thr Thr Ser Thr Gly Asn Lys Asn Phe Leu Ala Gly
            260                 265                 270
Arg Phe Ser His Leu
        275

<210> 214
<211> 277
<212> PRT
<213> Homo sapiens

<220>
<221> MOD_RES
<222> (103)...(104)
<223> Xaa = any amino acid

<400> 214
   Met Ile Ile Leu Ile Tyr Leu Phe Leu Leu Leu Trp Glu Asp Thr Gln
    1               5                  10                  15
   Gly Trp Gly Phe Lys Asp Gly Ile Phe His Asn Ser Ile Trp Leu Glu
               20                  25                  30
   Arg Ala Ala Gly Val Tyr His Arg Glu Ala Arg Ser Gly Lys Tyr Lys
           35                  40                  45
   Leu Thr Tyr Ala Glu Ala Lys Ala Val Cys Glu Phe Glu Gly Gly His
       50                  55                  60
   Leu Ala Thr Tyr Lys Gln Leu Glu Ala Ala Arg Lys Ile Gly Phe His
   65                  70                  75                  80
   Val Cys Ala Ala Gly Trp Met Ala Lys Gly Arg Val Gly Tyr Pro Ile
                   85                  90                  95
   Val Lys Pro Gly Pro Asn Xaa Xaa Phe Gly Lys Thr Gly Ile Ile Asp
               100                 105                 110
   Tyr Gly Ile Arg Leu Asn Arg Ser Glu Arg Trp Asp Ala Tyr Cys Tyr
           115                 120                 125
   Asn Pro His Ala Lys Glu Cys Gly Gly Val Phe Thr Asp Pro Lys Arg
       130                 135                 140
   Ile Phe Lys Ser Pro Gly Phe Pro Asn Glu Tyr Glu Asp Asn Gln Ile
   145                 150                 155                 160
   Cys Tyr Trp His Ile Arg Leu Lys Tyr Gly Gln Arg Ile His Leu Ser
                   165                 170                 175
   Phe Leu Asp Phe Asp Leu Glu Asp Asp Pro Gly Cys Leu Ala Asp Tyr
               180                 185                 190
   Val Glu Ile Tyr Asp Ser Tyr Asp Asp Val His Gly Phe Val Gly Arg
           195                 200                 205
   Tyr Cys Gly Asp Glu Leu Pro Asp Asp Ile Ile Ser Thr Gly Asn Val
       210                 215                 220
   Met Thr Leu Lys Phe Leu Ser Asp Ala Ser Val Thr Ala Gly Gly Phe
   225                 230                 235                 240
   Gln Ile Lys Tyr Val Ala Met Asp Pro Val Ser Lys Ser Ser Gln Gly
                   245                 250                 255
   Lys Asn Thr Ser Thr Thr Ser Thr Gly Asn Lys Asn Phe Leu Ala Gly
               260                 265                 270
   Arg Phe Ser His Leu
           275

<210> 215
<211> 165
<212> PRT
<213> Homo sapiens

<400> 215
Met Ala Pro Asn Ala Ser Cys Leu Cys Val His Val Arg Ser Glu Glu
1               5                   10                  15
Trp Asp Leu Met Thr Phe Asp Ala Asn Pro Tyr Asp Ser Val Lys Lys
            20                  25                  30
Ile Lys Glu His Val Arg Ser Lys Thr Lys Val Pro Val Gln Asp Gln
        35                  40                  45
Val Leu Leu Leu Gly Ser Lys Ile Leu Lys Pro Arg Arg Ser Leu Ser
    50                  55                  60
Ser Tyr Gly Ile Asp Lys Glu Lys Thr Ile His Leu Thr Leu Lys Val
65                  70                  75                  80
Val Lys Pro Ser Asp Glu Glu Leu Pro Leu Phe Leu Val Glu Ser Gly
                85                  90                  95
Asp Glu Ala Lys Arg His Leu Leu Gln Val Arg Arg Ser Ser Ser Val
            100                 105                 110
Ala Gln Val Lys Ala Met Ile Glu Thr Lys Thr Gly Ile Ile Pro Glu
        115                 120                 125
Thr Gln Ile Val Thr Cys Asn Gly Lys Arg Leu Glu Asp Gly Lys Met
    130                 135                 140
Met Ala Asp Tyr Gly Ile Arg Lys Gly Asn Leu Leu Phe Leu Ala Ser
145                 150                 155                 160
Tyr Cys Ile Gly Gly
                165

<210> 216
<211> 205
<212> PRT
<213> Homo sapiens

<400> 216
Met Ala Leu Gln Leu Ser Arg Glu Gln Gly Ile Thr Leu Arg Gly Ser
1               5                   10                  15
Ala Glu Ile Val Ala Glu Phe Phe Ser Phe Gly Ile Asn Ser Ile Leu
            20                  25                  30
Tyr Gln Arg Gly Ile Tyr Pro Ser Glu Thr Phe Thr Arg Val Gln Lys
        35                  40                  45
Tyr Gly Leu Thr Leu Leu Val Thr Thr Asp Leu Glu Leu Ile Lys Tyr
    50                  55                  60
Leu Asn Asn Val Val Glu Gln Leu Lys Asp Trp Leu Tyr Lys Cys Ser

65                  70                  75                  80
Val Gln Lys Leu Val Val Val Ile Ser Asn Ile Glu Ser Gly Glu Val
                85                  90                  95

Leu Glu Arg Trp Gln Phe Asp Ile Glu Cys Asp Lys Thr Ala Lys Asp
            100                 105                 110
Asp Ser Ala Pro Arg Glu Lys Ser Gln Lys Ala Ile Gln Asp Glu Ile
        115                 120                 125
Arg Ser Val Ile Arg Gln Ile Thr Ala Thr Val Thr Phe Leu Pro Leu
    130                 135                 140
Leu Glu Val Ser Cys Ser Phe Asp Leu Leu Ile Tyr Thr Asp Lys Asp
145                 150                 155                 160
Leu Val Val Pro Glu Lys Trp Glu Glu Ser Gly Pro Gln Phe Ile Thr
                165                 170                 175
Asn Ser Glu Glu Val Arg Leu Arg Ser Phe Thr Thr Thr Ile His Lys
            180                 185                 190
Val Asn Ser Met Val Ala Tyr Lys Ile Pro Val Asn Asp
        195                 200                 205

<210> 217
<211> 141
<212> PRT
<213> Homo sapiens

<400> 217
Met Ala Gln Tyr Leu Ser Thr Leu Leu Leu Leu Leu Ala Thr Leu Ala
 1               5                  10                  15
Val Ala Leu Ala Trp Ser Pro Lys Glu Glu Asp Arg Ile Ile Pro Gly
            20                  25                  30
Gly Ile Tyr Asn Ala Asp Leu Asn Asp Glu Trp Val Gln Arg Ala Leu
        35                  40                  45
His Phe Ala Ile Ser Glu Tyr Asn Lys Ala Thr Lys Asp Asp Tyr Tyr
    50                  55                  60
Arg Arg Pro Leu Arg Val Leu Arg Ala Arg Gln Gln Thr Val Gly Gly
65                  70                  75                  80
Val Asn Tyr Phe Phe Asp Val Glu Val Gly Arg Thr Ile Cys Thr Lys
                85                  90                  95
Ser Gln Pro Asn Leu Asp Thr Cys Ala Phe His Glu Gln Pro Glu Leu
            100                 105                 110
Gln Lys Lys Gln Leu Cys Ser Phe Glu Ile Tyr Glu Val Pro Trp Glu
        115                 120                 125
Asn Arg Arg Ser Leu Val Lys Ser Arg Cys Gln Glu Ser
    130                 135                 140

<210> 218
<211> 642
<212> PRT
<213> Homo sapiens

<400> 218
Met Asp Lys Leu Lys Cys Pro Ser Phe Phe Lys Cys Arg Glu Lys Glu
 1               5                  10                  15
Lys Val Ser Ala Ser Ser Glu Asn Phe His Val Gly Glu Asn Asp Glu
            20                  25                  30
Asn Gln Asp Arg Gly Asn Trp Ser Lys Lys Ser Asp Tyr Leu Leu Ser
        35                  40                  45
Met Ile Gly Tyr Ala Val Gly Leu Gly Asn Val Trp Arg Phe Pro Tyr
    50                  55                  60
Leu Thr Tyr Ser Asn Gly Gly Gly Ala Phe Leu Ile Pro Tyr Ala Ile
65                  70                  75                  80
Met Leu Ala Leu Ala Gly Leu Pro Leu Phe Phe Leu Glu Cys Ser Leu
                85                  90                  95
Gly Gln Phe Ala Ser Leu Gly Pro Val Ser Val Trp Arg Ile Leu Pro

            100                 105                 110

Leu Phe Gln Gly Val Gly Ile Thr Met Val Leu Ile Ser Ile Phe Val
        115                 120                 125
Thr Ile Tyr Tyr Asn Val Ile Ile Ala Tyr Ser Leu Tyr Tyr Met Phe
    130                 135                 140
Ala Ser Phe Gln Ser Glu Leu Pro Trp Lys Asn Cys Ser Ser Trp Ser
145                 150                 155                 160
Asp Lys Asn Cys Ser Arg Ser Pro Ile Val Thr His Cys Asn Val Ser
                165                 170                 175
Thr Val Asn Lys Gly Ile Gln Glu Ile Ile Gln Met Asn Lys Ser Trp
            180                 185                 190
Val Asp Ile Asn Asn Phe Thr Cys Ile Asn Gly Ser Glu Ile Tyr Gln
        195                 200                 205
Pro Gly Gln Leu Pro Ser Glu Gln Tyr Trp Asn Lys Val Ala Leu Gln
    210                 215                 220
Arg Ser Ser Gly Met Asn Glu Thr Gly Val Ile Val Trp Tyr Leu Ala
225                 230                 235                 240
Leu Cys Leu Leu Leu Ala Trp Leu Ile Val Gly Ala Ala Leu Phe Lys
                245                 250                 255
Gly Ile Lys Ser Ser Gly Lys Val Val Tyr Phe Thr Ala Leu Phe Pro
            260                 265                 270
Tyr Val Val Leu Leu Ile Leu Leu Val Arg Gly Ala Thr Leu Glu Gly
        275                 280                 285
Ala Ser Lys Gly Ile Ser Tyr Tyr Ile Gly Ala Gln Ser Asn Phe Thr
    290                 295                 300
Lys Leu Lys Glu Ala Glu Val Trp Lys Asp Ala Ala Thr Gln Ile Phe
305                 310                 315                 320
Tyr Ser Leu Ser Val Ala Trp Gly Gly Leu Val Ala Leu Ser Ser Tyr
                325                 330                 335
Asn Lys Phe Lys Asn Asn Cys Phe Ser Asp Ala Ile Val Val Cys Leu
            340                 345                 350
Thr Asn Cys Leu Thr Ser Val Phe Ala Gly Phe Ala Ile Phe Ser Ile
        355                 360                 365
Leu Gly His Met Ala His Ile Ser Gly Lys Glu Val Ser Gln Val Val
    370                 375                 380
Lys Ser Gly Phe Asp Leu Ala Phe Ile Ala Tyr Pro Glu Ala Leu Ala
385                 390                 395                 400
Gln Leu Pro Gly Gly Pro Phe Trp Ser Ile Leu Phe Phe Phe Met Leu
                405                 410                 415
Leu Thr Leu Gly Leu Asp Ser Gln Phe Ala Ser Ile Glu Thr Ile Thr
            420                 425                 430
Thr Thr Ile Gln Asp Leu Phe Pro Lys Val Met Lys Lys Met Arg Val
        435                 440                 445
Pro Ile Thr Leu Gly Cys Cys Leu Val Leu Phe Leu Leu Gly Leu Val
    450                 455                 460
Cys Val Thr Gln Ala Gly Ile Tyr Trp Val His Leu Ile Asp His Phe
465                 470                 475                 480
Cys Ala Gly Trp Gly Ile Leu Ile Ala Ala Ile Leu Glu Leu Val Gly
                485                 490                 495
Ile Ile Trp Ile Tyr Gly Gly Asn Arg Phe Ile Glu Asp Thr Glu Met
            500                 505                 510
Met Ile Gly Ala Lys Arg Trp Ile Phe Trp Leu Trp Trp Arg Ala Cys
        515                 520                 525
Trp Phe Val Ile Thr Pro Ile Leu Leu Ile Ala Ile Phe Ile Trp Ser
    530                 535                 540
Leu Val Gln Phe His Arg Pro Asn Tyr Gly Ala Ile Pro Tyr Pro Asp
545                 550                 555                 560
Trp Gly Val Ala Leu Gly Trp Cys Met Ile Val Phe Cys Ile Ile Trp
                565                 570                 575
Ile Pro Ile Met Ala Ile Ile Lys Ile Ile Gln Ala Lys Gly Asn Ile
            580                 585                 590

Phe Gln Arg Leu Ile Ser Cys Cys Arg Pro Ala Ser Asn Trp Gly Pro
        595                 600                 605
Tyr Leu Glu Gln His Arg Gly Glu Arg Tyr Lys Asp Met Val Asp Pro
    610                 615                 620
Lys Lys Glu Ala Asp His Glu Ile Pro Thr Val Ser Gly Ser Arg Lys
625                 630                 635                 640
Pro Glu

<210> 219
<211> 165
<212> PRT
<213> Homo sapiens

<400> 219
Met Ala Pro Asn Ala Ser Cys Leu Cys Val His Val Arg Ser Glu Glu
1               5                   10                  15
Trp Asp Leu Met Thr Phe Asp Ala Asn Pro Tyr Asp Ser Val Lys Lys
            20                  25                  30
Ile Lys Glu His Val Arg Ser Lys Thr Lys Val Pro Val Gln Asp Gln
        35                  40                  45
Val Leu Leu Leu Gly Ser Lys Ile Leu Lys Pro Arg Arg Ser Leu Ser
    50                  55                  60
Ser Tyr Gly Ile Asp Lys Glu Lys Thr Ile His Leu Thr Leu Lys Val
65                  70                  75                  80
Val Lys Pro Ser Asp Glu Glu Leu Pro Leu Phe Leu Val Glu Ser Gly
                85                  90                  95
Asp Glu Ala Lys Arg His Leu Leu Gln Val Arg Arg Ser Ser Ser Val
            100                 105                 110
Ala Gln Val Lys Ala Met Ile Glu Thr Lys Thr Gly Ile Ile Pro Glu
        115                 120                 125
Thr Gln Ile Val Thr Cys Asn Gly Lys Arg Leu Glu Asp Gly Lys Met
    130                 135                 140
Met Ala Asp Tyr Gly Ile Arg Lys Gly Asn Leu Leu Phe Leu Ala Ser
145                 150                 155                 160
Tyr Cys Ile Gly Gly
                165

<210> 220
<211> 315
<212> PRT
<213> Homo sapiens

<400> 220
Met Lys Ala Asn Tyr Ser Ala Glu Glu Arg Phe Leu Leu Leu Gly Phe
1               5                   10                  15
Ser Asp Trp Pro Ser Leu Gln Pro Val Leu Phe Ala Leu Val Leu Leu
            20                  25                  30
Cys Tyr Leu Leu Thr Leu Thr Gly Asn Ser Ala Leu Val Leu Leu Ala
        35                  40                  45
Val Arg Asp Pro Arg Leu His Thr Pro Met Tyr Tyr Phe Leu Cys His
    50                  55                  60
Leu Ala Leu Val Asp Ala Gly Phe Thr Thr Ser Val Val Pro Pro Leu
65                  70                  75                  80
Leu Ala Asn Leu Arg Gly Pro Ala Leu Trp Leu Pro Arg Ser His Cys
                85                  90                  95
Thr Ala Gln Leu Cys Ala Ser Leu Ala Leu Gly Ser Ala Glu Cys Val
            100                 105                 110
Leu Leu Ala Val Met Ala Leu Asp Arg Ala Ala Ala Val Cys Arg Pro
        115                 120                 125
Leu Arg Tyr Ala Gly Leu Val Ser Pro Arg Leu Cys Arg Thr Leu Ala
    130                 135                 140
Ser Ala Ser Trp Leu Ser Gly Leu Thr Asn Ser Val Ala Gln Thr Ala
145                 150                 155                 160

Leu Leu Ala Glu Arg Pro Leu Cys Ala Pro Arg Leu Leu Asp His Phe
                165                 170                 175
Ile Cys Glu Leu Pro Ala Leu Leu Lys Leu Ala Cys Gly Gly Asp Gly
            180                 185                 190
Asp Thr Thr Glu Asn Gln Met Phe Ala Ala Arg Val Val Ile Leu Leu
        195                 200                 205
Leu Pro Phe Ala Val Ile Leu Ala Ser Tyr Gly Ala Val Ala Arg Ala
    210                 215                 220
Val Cys Cys Met Arg Phe Ser Gly Gly Arg Arg Arg Ala Val Gly Thr
225                 230                 235                 240
Cys Gly Ser His Leu Thr Ala Val Cys Leu Phe Tyr Gly Ser Ala Ile
                245                 250                 255
Tyr Thr Tyr Leu Gln Pro Ala Gln Arg Tyr Asn Gln Ala Arg Gly Lys
            260                 265                 270
Phe Val Ser Leu Phe Tyr Thr Val Val Thr Pro Ala Leu Asn Pro Leu
        275                 280                 285
Ile Tyr Thr Leu Arg Asn Lys Lys Val Lys Gly Ala Ala Arg Arg Leu
    290                 295                 300
Leu Arg Ser Leu Gly Arg Gly Gln Ala Gly Gln
305                 310                 315

<210> 221
<211> 1124
<212> PRT
<213> Homo sapiens

<400> 221
Met Asp Pro Phe Thr Glu Lys Leu Leu Glu Arg Thr Arg Ala Arg Arg
 1               5                  10                  15
Glu Asn Leu Gln Arg Lys Met Ala Glu Arg Pro Thr Ala Ala Pro Arg
            20                  25                  30
Ser Met Thr His Ala Lys Arg Ala Arg Gln Pro Leu Ser Glu Ala Ser
        35                  40                  45
Asn Gln Gln Pro Leu Ser Gly Gly Glu Glu Lys Ser Cys Thr Lys Pro
    50                  55                  60
Ser Pro Ser Lys Lys Arg Cys Ser Asp Asn Thr Glu Val Glu Val Ser
65                  70                  75                  80
Asn Leu Glu Asn Lys Gln Pro Val Glu Ser Thr Ser Ala Lys Ser Cys
                85                  90                  95
Ser Pro Ser Pro Val Ser Pro Gln Val Gln Pro Gln Ala Ala Asp Thr
            100                 105                 110
Ile Ser Asp Ser Val Ala Val Pro Ala Ser Leu Leu Gly Met Arg Arg
        115                 120                 125
Gly Leu Asn Ser Arg Leu Glu Ala Thr Ala Ala Ser Ser Val Lys Thr
    130                 135                 140
Arg Met Gln Lys Leu Ala Glu Gln Arg Arg Arg Trp Asp Asn Asp Asp
145                 150                 155                 160
Met Thr Asp Asp Ile Pro Glu Ser Ser Leu Phe Ser Pro Met Pro Ser
                165                 170                 175
Glu Glu Lys Ala Ala Ser Pro Pro Lys Pro Leu Leu Ser Asn Ala Ser
            180                 185                 190
Ala Thr Pro Val Gly Arg Arg Gly Arg Leu Ala Asn Leu Ala Ala Thr
        195                 200                 205
Ile Cys Ser Trp Glu Asp Asp Val Asn His Ser Phe Ala Lys Gln Asn
    210                 215                 220
Ser Val Gln Glu Gln Pro Gly Thr Ala Cys Leu Ser Lys Phe Ser Ser
225                 230                 235                 240
Ala Ser Gly Ala Ser Ala Arg Ile Asn Ser Ser Ser Val Lys Gln Glu
                245                 250                 255
Ala Thr Phe Cys Ser Gln Arg Asp Gly Asp Ala Ser Leu Asn Lys Ala
            260                 265                 270
Leu Ser Ser Ser Ala Asp Asp Ala Ser Leu Val Asn Ala Ser Ile Ser
        275                 280                 285

Ser Ser Val Lys Ala Thr Ser Pro Val Lys Ser Thr Thr Ser Ile Thr
    290                 295                 300
Asp Ala Lys Ser Cys Glu Gly Gln Asn Pro Glu Leu Leu Pro Lys Thr
305                 310                 315                 320
Pro Ile Ser Pro Leu Lys Thr Gly Val Ser Lys Pro Ile Val Lys Ser
                325                 330                 335
Thr Leu Ser Gln Thr Val Pro Ser Lys Gly Glu Leu Ser Arg Glu Ile
            340                 345                 350
Cys Leu Gln Ser Gln Ser Lys Asp Lys Ser Thr Thr Pro Gly Gly Thr
        355                 360                 365
Gly Ile Lys Pro Phe Leu Glu Arg Phe Gly Glu Arg Cys Gln Glu His
    370                 375                 380
Ser Lys Glu Ser Pro Ala Arg Ser Thr Pro His Arg Thr Pro Ile Ile
385                 390                 395                 400
Thr Pro Asn Thr Lys Ala Ile Gln Glu Arg Leu Phe Lys Gln Asp Thr
                405                 410                 415
Ser Ser Ser Thr Thr His Leu Ala Gln Gln Leu Lys Gln Glu Arg Gln
            420                 425                 430
Lys Glu Leu Ala Cys Leu Arg Gly Arg Phe Asp Lys Gly Asn Ile Trp
        435                 440                 445
Ser Ala Glu Lys Gly Gly Asn Ser Lys Ser Lys Gln Leu Glu Thr Lys
    450                 455                 460
Gln Glu Thr His Cys Gln Ser Thr Pro Leu Lys Lys His Gln Gly Val
465                 470                 475                 480
Ser Lys Thr Gln Ser Leu Pro Val Thr Glu Lys Val Thr Glu Asn Gln
                485                 490                 495
Ile Pro Ala Lys Asn Ser Ser Thr Glu Pro Lys Gly Phe Thr Glu Cys
            500                 505                 510
Glu Met Thr Lys Ser Ser Pro Leu Lys Ile Thr Leu Phe Leu Glu Glu
        515                 520                 525
Asp Lys Ser Leu Lys Val Thr Ser Asp Pro Lys Val Glu Gln Lys Ile
    530                 535                 540
Glu Val Ile Arg Glu Ile Glu Met Ser Val Asp Asp Asp Ile Asn
545                 550                 555                 560
Ser Ser Lys Val Ile Asn Asp Leu Phe Ser Asp Val Leu Glu Glu Gly
                565                 570                 575
Glu Leu Asp Met Glu Lys Ser Gln Glu Glu Met Asp Gln Ala Leu Ala
            580                 585                 590
Glu Ser Ser Glu Glu Gln Glu Asp Ala Leu Asn Ile Ser Ser Met Ser
        595                 600                 605
Leu Leu Ala Pro Leu Ala Gln Thr Val Gly Val Val Ser Pro Glu Ser
    610                 615                 620
Leu Val Ser Thr Pro Arg Leu Glu Leu Lys Asp Thr Ser Arg Ser Asp
625                 630                 635                 640
Glu Ser Pro Lys Pro Gly Lys Phe Gln Arg Thr Arg Val Pro Arg Ala
                645                 650                 655
Glu Ser Gly Asp Ser Leu Gly Ser Glu Asp Arg Asp Leu Leu Tyr Ser
            660                 665                 670
Ile Asp Ala Tyr Arg Ser Gln Arg Phe Lys Glu Thr Glu Arg Pro Ser
        675                 680                 685
Ile Lys Gln Val Ile Val Arg Lys Glu Asp Val Thr Ser Lys Leu Asp
    690                 695                 700
Glu Lys Asn Asn Ala Phe Pro Cys Gln Val Asn Ile Lys Gln Lys Met
705                 710                 715                 720
Gln Glu Leu Asn Asn Glu Ile Asn Met Gln Gln Thr Val Ile Tyr Gln
                725                 730                 735

Ala Ser Gln Ala Leu Asn Cys Cys Val Asp Glu Glu His Gly Lys Gly
            740                 745                 750
Ser Leu Glu Glu Ala Glu Ala Glu Arg Leu Leu Leu Ile Ala Thr Gly
        755                 760                 765
Lys Arg Thr Leu Leu Ile Asp Glu Leu Asn Lys Leu Lys Asn Glu Gly
    770                 775                 780

Pro Gln Arg Lys Asn Lys Ala Ser Pro Gln Ser Glu Phe Met Pro Ser
785                 790                 795                 800
Lys Gly Ser Val Thr Leu Ser Glu Ile Arg Leu Pro Leu Lys Ala Asp
                805                 810                 815
Phe Val Cys Ser Thr Val Gln Lys Pro Asp Ala Ala Asn Tyr Tyr Tyr
                820                 825                 830
Leu Ile Ile Leu Lys Ala Gly Ala Glu Asn Met Val Ala Thr Pro Leu
        835                 840                 845
Ala Ser Thr Ser Asn Ser Leu Asn Gly Asp Ala Leu Thr Phe Thr Thr
    850                 855                 860
Thr Phe Thr Leu Gln Asp Val Ser Asn Asp Phe Glu Ile Asn Ile Glu
865                 870                 875                 880
Val Tyr Ser Leu Val Gln Lys Lys Asp Pro Ser Gly Leu Asp Lys Lys
                885                 890                 895
Lys Lys Thr Ser Lys Ser Lys Ala Ile Thr Pro Lys Arg Leu Leu Thr
                900                 905                 910
Ser Ile Thr Thr Lys Ser Asn Ile His Ser Ser Val Met Ala Ser Pro
        915                 920                 925
Gly Gly Leu Ser Ala Val Arg Thr Ser Asn Phe Ala Leu Val Gly Ser
    930                 935                 940
Tyr Thr Leu Ser Leu Ser Ser Val Gly Asn Thr Lys Phe Val Leu Asp
945                 950                 955                 960
Lys Val Pro Phe Leu Ser Ser Leu Glu Gly His Ile Tyr Leu Lys Ile
                965                 970                 975
Lys Cys Gln Val Asn Ser Ser Val Glu Glu Arg Gly Phe Leu Thr Ile
                980                 985                 990
Phe Glu Asp Val Ser Gly Phe Gly Ala Trp His Arg Arg Trp Cys Val
        995                 1000                1005
Leu Ser Gly Asn Cys Ile Ser Tyr Trp Thr Tyr Pro Asp Asp Glu Lys
    1010                1015                1020
Arg Lys Asn Pro Ile Gly Arg Ile Asn Leu Ala Asn Cys Thr Ser Arg
1025                1030                1035                1040
Gln Ile Glu Pro Ala Asn Arg Glu Phe Cys Ala Arg Arg Asn Thr Phe
                1045                1050                1055
Glu Leu Ile Thr Val Arg Pro Gln Arg Glu Asp Asp Arg Glu Thr Leu
                1060                1065                1070
Val Ser Gln Cys Arg Asp Thr Leu Cys Val Thr Lys Asn Trp Leu Ser
        1075                1080                1085
Ala Asp Thr Lys Glu Glu Arg Asp Leu Trp Met Gln Lys Leu Asn Gln
    1090                1095                1100
Val Leu Val Asp Ile Arg Leu Trp Gln Pro Asp Ala Cys Tyr Lys Pro
1105                1110                1115                1120
Ile Gly Lys Pro

<210> 222
<211> 883
<212> PRT
<213> Homo sapiens

<400> 222
Met Ala Glu Asn Ser Val Leu Thr Ser Thr Thr Gly Arg Thr Ser Leu
1               5                   10                  15
Ala Asp Ser Ser Ile Phe Asp Ser Lys Val Thr Glu Ile Ser Lys Glu
            20                  25                  30
Asn Leu Leu Ile Gly Ser Thr Ser Tyr Val Glu Glu Glu Met Pro Gln
        35                  40                  45
Ile Glu Thr Arg Val Ile Leu Val Gln Glu Ala Gly Lys Gln Glu Glu
    50                  55                  60
Leu Thr Lys Ala Leu Lys Asp Ile Lys Val Gly Phe Val Lys Met Glu
65                  70                  75                  80
Ser Val Glu Glu Phe Glu Gly Leu Asp Ser Pro Glu Phe Glu Asn Val
                85                  90                  95

Phe Val Val Thr Asp Phe Gln Asp Ser Val Phe Asn Asp Leu Tyr Lys
                100                 105                 110
Ala Asp Cys Arg Val Ile Gly Pro Pro Val Val Leu Asn Cys Ser Gln
        115                 120                 125
Lys Gly Glu Pro Leu Pro Phe Ser Cys Arg Pro Leu Tyr Cys Thr Ser
    130                 135                 140
Met Met Asn Leu Val Leu Cys Phe Thr Gly Phe Arg Lys Lys Glu Glu
145                 150                 155                 160
Leu Val Arg Leu Val Thr Leu Val His His Met Gly Gly Val Ile Arg
                165                 170                 175
Lys Asp Phe Asn Ser Lys Val Thr His Leu Val Ala Asn Cys Thr Gln
            180                 185                 190
Gly Glu Lys Phe Arg Val Ala Val Ser Leu Gly Thr Pro Ile Met Lys
        195                 200                 205
Pro Glu Trp Ile Tyr Lys Ala Trp Glu Arg Arg Asn Glu Gln Asp Phe
    210                 215                 220
Tyr Ala Ala Val Asp Asp Phe Arg Asn Glu Phe Lys Val Pro Pro Phe
225                 230                 235                 240
Gln Asp Cys Ile Phe Ser Phe Leu Gly Phe Ser Asp Glu Glu Lys Thr
                245                 250                 255
Asn Met Glu Glu Met Thr Glu Met Gln Gly Gly Lys Tyr Leu Pro Leu
            260                 265                 270
Gly Asp Glu Arg Cys Thr His Leu Val Val Glu Glu Asn Ile Val Lys
        275                 280                 285
Asp Leu Pro Phe Glu Pro Ser Lys Lys Leu Tyr Val Val Lys Gln Glu
    290                 295                 300
Trp Phe Trp Gly Ser Ile Gln Met Asp Ala Arg Ala Gly Glu Thr Met
305                 310                 315                 320
Tyr Leu Tyr Glu Lys Ala Asn Thr Pro Glu Leu Lys Lys Ser Val Ser
                325                 330                 335
Met Leu Ser Leu Asn Thr Pro Asn Ser Asn Arg Lys Arg Arg Arg Leu
            340                 345                 350
Lys Glu Thr Leu Ala Gln Leu Ser Arg Asp Thr Asp Val Ser Pro Phe
        355                 360                 365
Pro Pro Arg Lys Arg Pro Ser Ala Glu His Ser Leu Ser Ile Gly Ser
    370                 375                 380
Leu Leu Asp Ile Ser Asn Thr Pro Glu Ser Ser Ile Asn Tyr Gly Asp
385                 390                 395                 400
Thr Pro Lys Ser Cys Thr Lys Ser Ser Lys Ser Ser Thr Pro Val Pro
                405                 410                 415
Ser Lys Gln Ser Ala Arg Trp Gln Val Ala Lys Glu Leu Tyr Gln Thr
            420                 425                 430
Glu Ser Asn Tyr Val Asn Ile Leu Ala Thr Ile Ile Gln Leu Phe Gln
        435                 440                 445
Val Pro Leu Glu Glu Glu Gly Gln Arg Gly Gly Pro Ile Leu Ala Pro
    450                 455                 460
Glu Glu Ile Lys Thr Ile Phe Gly Ser Ile Pro Asp Ile Phe Asp Val
465                 470                 475                 480
His Thr Lys Ile Lys Asp Asp Leu Glu Asp Leu Ile Val Asn Trp Asp
                485                 490                 495
Glu Ser Lys Ser Ile Gly Asp Ile Phe Leu Lys Tyr Ser Lys Asp Leu
            500                 505                 510

Val Lys Thr Tyr Pro Pro Phe Val Asn Phe Phe Glu Met Ser Lys Glu
        515                 520                 525
Thr Ile Ile Lys Cys Glu Lys Gln Lys Pro Arg Phe His Ala Phe Leu
    530                 535                 540
Lys Ile Asn Gln Ala Lys Pro Glu Cys Gly Arg Gln Ser Leu Val Glu
545                 550                 555                 560
Leu Leu Ile Arg Pro Val Gln Arg Leu Pro Ser Val Ala Leu Leu Leu
                565                 570                 575
Asn Asp Leu Lys Lys His Thr Ala Asp Glu Asn Pro Asp Lys Ser Thr
            580                 585                 590

Leu Glu Lys Ala Ile Gly Ser Leu Lys Glu Val Met Thr His Ile Asn
        595                 600                 605
Glu Asp Lys Arg Lys Thr Glu Ala Gln Lys Gln Ile Phe Asp Val Val
    610                 615                 620
Tyr Glu Val Asp Gly Cys Pro Ala Asn Leu Leu Ser Ser His Arg Ser
625                 630                 635                 640
Leu Val Gln Arg Val Glu Thr Ile Ser Leu Gly Glu His Pro Cys Asp
                645                 650                 655
Arg Gly Glu Gln Val Thr Leu Phe Leu Phe Asn Asp Cys Leu Glu Ile
            660                 665                 670
Ala Arg Lys Arg His Lys Val Ile Gly Thr Phe Arg Ser Pro His Gly
        675                 680                 685
Gln Thr Arg Pro Pro Ala Ser Leu Lys His Ile His Leu Met Pro Leu
    690                 695                 700
Ser Gln Ile Lys Lys Val Leu Asp Ile Arg Glu Thr Glu Asp Cys His
705                 710                 715                 720
Asn Ala Phe Ala Leu Leu Val Arg Pro Pro Thr Glu Gln Ala Asn Val
                725                 730                 735
Leu Leu Ser Phe Gln Met Thr Ser Asp Glu Leu Pro Lys Glu Asn Trp
            740                 745                 750
Leu Lys Met Leu Cys Arg His Val Ala Asn Thr Ile Cys Lys Ala Asp
        755                 760                 765
Ala Glu Asn Leu Ile Tyr Thr Ala Asp Pro Glu Ser Phe Glu Val Asn
    770                 775                 780
Thr Lys Asp Met Asp Ser Thr Leu Ser Arg Ala Ser Arg Ala Ile Lys
785                 790                 795                 800
Lys Thr Ser Lys Lys Val Thr Arg Ala Phe Ser Phe Ser Lys Thr Pro
                805                 810                 815
Lys Arg Ala Leu Arg Arg Ala Leu Met Thr Ser His Gly Ser Val Glu
            820                 825                 830
Gly Arg Ser Pro Ser Ser Asn Asp Lys His Val Met Ser Arg Leu Ser
        835                 840                 845
Ser Thr Ser Ser Leu Ala Gly Ile Pro Ser Pro Ser Leu Val Ser Leu
    850                 855                 860
Pro Ser Phe Phe Glu Arg Arg Ser His Thr Leu Ser Arg Ser Thr Thr
865                 870                 875                 880
His Leu Ile

<210> 223
<211> 445
<212> PRT
<213> Homo sapiens

<400> 223
Met Pro Ser Arg Ala Glu Asp Tyr Glu Val Leu Tyr Thr Ile Gly Thr
 1               5                  10                  15
Gly Ser Tyr Gly Arg Cys Gln Lys Ile Arg Arg Lys Ser Asp Gly Lys
            20                  25                  30
Ile Leu Val Trp Lys Glu Leu Asp Tyr Gly Ser Met Thr Glu Ala Glu
        35                  40                  45
Lys Gln Met Leu Val Ser Glu Val Asn Leu Leu Arg Glu Leu Lys His
    50                  55                  60
Pro Asn Ile Val Arg Tyr Tyr Asp Arg Ile Ile Asp Arg Thr Asn Thr
65                  70                  75                  80
Thr Leu Tyr Ile Val Met Glu Tyr Cys Glu Gly Gly Asp Leu Ala Ser
                85                  90                  95
Val Ile Thr Lys Gly Thr Lys Glu Arg Gln Tyr Leu Asp Glu Glu Phe
            100                 105                 110
Val Leu Arg Val Met Thr Gln Leu Thr Leu Ala Leu Lys Glu Cys His
        115                 120                 125
Arg Arg Ser Asp Gly Gly His Thr Val Leu His Arg Asp Leu Lys Pro
    130                 135                 140

Ala Asn Val Phe Leu Asp Gly Lys Gln Asn Val Lys Leu Gly Asp Phe
145                 150                 155                 160
Gly Leu Ala Arg Ile Leu Asn His Asp Thr Ser Phe Ala Lys Thr Phe
                165                 170                 175
Val Gly Thr Pro Tyr Tyr Met Ser Pro Glu Gln Met Asn Arg Met Ser
            180                 185                 190
Tyr Asn Glu Lys Ser Asp Ile Trp Ser Leu Gly Cys Leu Leu Tyr Glu
        195                 200                 205
Leu Cys Ala Leu Met Pro Pro Phe Thr Ala Phe Ser Gln Lys Glu Leu
    210                 215                 220
Ala Gly Lys Ile Arg Glu Gly Lys Phe Arg Arg Ile Pro Tyr Arg Tyr
225                 230                 235                 240
Ser Asp Glu Leu Asn Glu Ile Ile Thr Arg Met Leu Asn Leu Lys Asp
                245                 250                 255
Tyr His Arg Pro Ser Val Glu Glu Ile Leu Glu Asn Pro Leu Ile Ala
            260                 265                 270
Asp Leu Val Ala Asp Glu Gln Arg Arg Asn Leu Glu Arg Arg Gly Arg
        275                 280                 285
Gln Leu Gly Glu Pro Glu Lys Ser Gln Asp Ser Ser Pro Val Leu Ser
    290                 295                 300
Glu Leu Lys Leu Lys Glu Ile Gln Leu Gln Glu Arg Glu Arg Ala Leu
305                 310                 315                 320
Lys Ala Arg Glu Glu Arg Leu Glu Gln Lys Glu Gln Glu Leu Cys Val
                325                 330                 335
Arg Glu Arg Leu Ala Glu Asp Lys Leu Ala Arg Ala Glu Asn Leu Leu
            340                 345                 350
Lys Asn Tyr Ser Leu Leu Lys Glu Arg Lys Phe Leu Ser Leu Ala Ser
        355                 360                 365
Asn Pro Glu Leu Leu Asn Leu Pro Ser Ser Val Ile Lys Lys Lys Val
    370                 375                 380
His Phe Ser Gly Glu Ser Lys Glu Asn Ile Met Arg Ser Glu Asn Ser
385                 390                 395                 400
Glu Ser Gln Leu Thr Ser Lys Ser Lys Cys Lys Asp Leu Lys Lys Arg
                405                 410                 415
Leu His Ala Ala Gln Leu Arg Ala Gln Ala Leu Ser Asp Ile Glu Lys
            420                 425                 430
Asn Tyr Gln Leu Lys Ser Arg Gln Ile Leu Gly Met Arg
        435                 440                 445

<210> 224
<211> 271
<212> PRT
<213> Homo sapiens

<400> 224
Asp Ile Ser Tyr Glu Asn Glu Asn Tyr Asn Ser Ser Trp Ile Pro Gly
1               5                   10                  15
Ser His Ile Val Ser Pro Ser Leu Leu Leu Asn Leu Asn Asn Gly Gln
            20                  25                  30
Leu Leu Pro Leu Gln Leu Gln Gly Pro Leu Asn Ser Trp Ile Pro Pro
        35                  40                  45
Phe Ser Gly Ile Leu Gln Gln Gln Gln Gln Ala Gln Ile Pro Gly Leu
    50                  55                  60
Ser Gln Phe Ser Leu Ser Ala Leu Asp Gln Phe Ala Gly Leu Leu Pro
65                  70                  75                  80
Asn Gln Ile Pro Leu Thr Gly Glu Ala Ser Phe Ala Gln Gly Ala Gln
                85                  90                  95
Ala Gly Gln Val Asp Pro Leu Gln Leu Gln Thr Pro Pro Gln Thr Gln
            100                 105                 110
Pro Gly Pro Ser His Val Met Pro Tyr Val Phe Ser Phe Lys Met Pro
        115                 120                 125
Gln Glu Gln Gly Gln Met Phe Gln Tyr Tyr Pro Val Tyr Met Val Leu
    130                 135                 140

Pro Trp Glu Gln Pro Gln Gln Thr Val Pro Arg Ser Pro Gln Gln Thr
145                 150                 155                 160
Arg Gln Gln Gln Tyr Glu Glu Gln Ile Pro Phe Tyr Ala Gln Phe Gly
                165                 170                 175
Tyr Ile Pro Gln Leu Ala Glu Pro Ala Ile Ser Gly Gly Gln Gln Gln
            180                 185                 190
Leu Ala Phe Asp Pro Gln Leu Gly Thr Ala Pro Glu Ile Ala Val Met
        195                 200                 205
Ser Thr Gly Glu Glu Ile Pro Tyr Leu Gln Lys Glu Ala Ile Asn Phe
    210                 215                 220
Arg His Asp Ser Ala Gly Val Phe Met Pro Ser Thr Ser Pro Lys Pro
225                 230                 235                 240
Ser Thr Thr Asn Val Phe Thr Ser Ala Val Asp Gln Thr Ile Thr Pro
                245                 250                 255
Glu Leu Pro Glu Glu Lys Asp Lys Thr Asp Ser Leu Arg Glu Pro
            260                 265                 270

<210> 225
<211> 304
<212> PRT
<213> Homo sapiens

<400> 225
Arg Gly Lys Glu His Arg Ser Arg Met Val Gln Ile Phe Leu Phe Arg
1               5                   10                  15
Ser Arg Ser Lys Ile Tyr His Thr Lys Met Lys Ile Ile Ile Leu Leu
            20                  25                  30
Gly Phe Leu Gly Ala Thr Leu Ser Ala Pro Leu Ile Pro Gln Arg Leu
        35                  40                  45
Met Ser Ala Ser Asn Ser Asn Glu Leu Leu Leu Asn Leu Asn Asn Gly
    50                  55                  60
Gln Leu Leu Pro Leu Gln Leu Gln Gly Pro Leu Asn Ser Trp Ile Pro
65                  70                  75                  80
Pro Phe Ser Gly Ile Leu Gln Gln Gln Gln Gln Ala Gln Ile Pro Gly
                85                  90                  95
Leu Ser Gln Phe Ser Leu Ser Ala Leu Asp Gln Phe Ala Gly Leu Leu
            100                 105                 110
Pro Asn Gln Ile Pro Leu Thr Gly Glu Ala Ser Phe Ala Gln Gly Ala
        115                 120                 125
Gln Ala Gly Gln Val Asp Pro Leu Gln Leu Gln Thr Pro Pro Gln Thr
    130                 135                 140
Gln Pro Gly Pro Ser His Val Met Pro Tyr Val Phe Ser Phe Lys Met
145                 150                 155                 160
Pro Gln Glu Gln Gly Gln Met Phe Gln Tyr Tyr Pro Val Tyr Met Val
                165                 170                 175
Leu Pro Trp Glu Gln Pro Gln Gln Thr Val Pro Arg Ser Pro Gln Gln
            180                 185                 190
Thr Arg Gln Gln Gln Tyr Glu Glu Gln Ile Pro Phe Tyr Ala Gln Phe
        195                 200                 205
Gly Tyr Ile Pro Gln Leu Ala Glu Pro Ala Ile Ser Gly Gly Gln Gln
    210                 215                 220
Gln Leu Ala Phe Asp Pro Gln Leu Gly Thr Ala Pro Glu Ile Ala Val
225                 230                 235                 240
Met Ser Thr Gly Glu Glu Ile Pro Tyr Leu Gln Lys Glu Ala Ile Asn
                245                 250                 255
Phe Arg His Asp Ser Ala Gly Val Phe Met Pro Ser Thr Ser Pro Lys
            260                 265                 270
Pro Ser Thr Thr Asn Val Phe Thr Ser Ala Val Asp Gln Thr Ile Thr
        275                 280                 285
Pro Glu Leu Pro Glu Glu Lys Asp Lys Thr Asp Ser Leu Arg Glu Pro
    290                 295                 300

<210> 226

<211> 357
<212> PRT
<213> Homo sapiens

<400> 226
Met Ala Thr Thr Val Pro Asp Gly Cys Arg Asn Gly Leu Lys Ser Lys
1               5                   10                  15
Tyr Tyr Arg Leu Cys Asp Lys Ala Glu Ala Trp Gly Ile Val Leu Glu
            20                  25                  30
Thr Val Ala Thr Ala Gly Val Val Thr Ser Val Ala Phe Met Leu Thr
        35                  40                  45
Leu Pro Ile Leu Val Cys Lys Val Gln Asp Ser Asn Arg Arg Lys Met
    50                  55                  60
Leu Pro Thr Gln Phe Leu Phe Leu Leu Gly Val Leu Gly Ile Phe Gly
65                  70                  75                  80
Leu Thr Phe Ala Phe Ile Ile Gly Leu Asp Gly Ser Thr Gly Pro Thr
                85                  90                  95
Arg Phe Phe Leu Phe Gly Ile Leu Phe Ser Ile Cys Phe Ser Cys Leu
            100                 105                 110
Leu Ala His Ala Val Ser Leu Thr Lys Leu Val Arg Gly Arg Lys Pro
        115                 120                 125
Leu Ser Leu Leu Val Ile Leu Gly Leu Ala Val Gly Phe Ser Leu Val
    130                 135                 140
Gln Asp Val Ile Ala Ile Glu Tyr Ile Val Leu Thr Met Asn Arg Thr
145                 150                 155                 160
Asn Val Asn Val Phe Ser Glu Leu Ser Ala Pro Arg Arg Asn Glu Asp
                165                 170                 175
Phe Val Leu Leu Leu Thr Tyr Val Leu Phe Leu Met Ala Leu Thr Phe
            180                 185                 190
Leu Met Ser Ser Phe Thr Phe Cys Gly Ser Phe Thr Gly Trp Lys Arg
        195                 200                 205
His Gly Ala His Ile Tyr Leu Thr Met Leu Leu Ser Ile Ala Ile Trp
    210                 215                 220
Val Ala Trp Ile Thr Leu Leu Met Leu Pro Asp Phe Asp Arg Arg Trp
225                 230                 235                 240
Asp Asp Thr Ile Leu Ser Ser Ala Leu Ala Ala Asn Gly Trp Val Phe
                245                 250                 255
Leu Leu Ala Tyr Val Ser Pro Glu Phe Trp Leu Leu Thr Lys Gln Arg
            260                 265                 270
Asn Pro Met Asp Tyr Pro Val Glu Asp Ala Phe Cys Lys Pro Gln Leu
        275                 280                 285
Val Lys Lys Ser Tyr Gly Val Glu Asn Arg Ala Tyr Ser Gln Glu Glu
    290                 295                 300
Ile Thr Gln Gly Phe Glu Glu Thr Gly Asp Thr Leu Tyr Ala Pro Tyr
305                 310                 315                 320
Ser Thr His Phe Gln Leu Gln Asn Gln Pro Pro Gln Lys Glu Phe Ser
                325                 330                 335
Ile Pro Arg Ala His Ala Trp Pro Ser Pro Tyr Lys Asp Tyr Glu Val
            340                 345                 350
Lys Lys Glu Gly Ser
        355

<210> 227
<211> 1400
<212> PRT
<213> Homo sapiens

<400> 227
Met Pro Lys Ile Val Leu Asn Gly Val Thr Val Asp Phe Pro Phe Gln
1               5                   10                  15
Pro Tyr Lys Cys Gln Gln Glu Tyr Met Thr Lys Val Leu Glu Cys Leu
            20                  25                  30
Gln Gln Lys Val Asn Gly Ile Leu Glu Ser Pro Thr Gly Thr Gly Lys

        35                  40                  45
Thr Leu Cys Leu Leu Cys Thr Thr Leu Ala Trp Arg Glu His Leu Arg
    50                  55                  60
Asp Gly Ile Ser Ala Arg Lys Ile Ala Glu Arg Ala Gln Gly Glu Leu
65                  70                  75                  80
Phe Pro Asp Arg Ala Leu Ser Ser Trp Gly Asn Ala Ala Ala Ala Ala
                85                  90                  95
Gly Asp Pro Ile Ala Cys Tyr Thr Asp Ile Pro Lys Ile Ile Tyr Ala
            100                 105                 110
Ser Arg Thr His Ser Gln Leu Thr Gln Val Ile Asn Glu Leu Arg Asn
        115                 120                 125
Thr Ser Tyr Arg Pro Lys Val Cys Val Leu Gly Ser Arg Glu Gln Leu
    130                 135                 140
Cys Ile His Pro Glu Val Lys Lys Gln Glu Ser Asn His Leu Gln Ile
145                 150                 155                 160
His Leu Cys Arg Lys Lys Val Ala Ser Arg Ser Cys His Phe Tyr Asn
                165                 170                 175
Asn Val Glu Glu Lys Ser Leu Glu Gln Glu Leu Ala Ser Pro Ile Leu
            180                 185                 190
Asp Ile Glu Asp Leu Val Lys Ser Gly Ser Lys His Arg Val Cys Pro
        195                 200                 205
Tyr Tyr Leu Ser Arg Asn Leu Lys Gln Gln Ala Asp Ile Ile Phe Met
    210                 215                 220
Pro Tyr Asn Tyr Leu Leu Asp Ala Lys Ser Arg Arg Ala His Asn Ile
225                 230                 235                 240
Asp Leu Lys Gly Thr Val Val Ile Phe Asp Glu Ala His Asn Val Glu
                245                 250                 255
Lys Met Cys Glu Glu Ser Ala Ser Phe Asp Leu Thr Pro His Asp Leu
            260                 265                 270
Ala Ser Gly Leu Asp Val Ile Asp Gln Val Leu Glu Glu Gln Thr Lys
        275                 280                 285
Ala Ala Gln Gln Gly Glu Pro His Pro Glu Phe Ser Ala Asp Ser Pro
    290                 295                 300
Ser Pro Gly Leu Asn Met Glu Leu Glu Asp Ile Ala Lys Leu Lys Met
305                 310                 315                 320
Ile Leu Leu Arg Leu Glu Gly Ala Ile Asp Ala Val Glu Leu Pro Gly
                325                 330                 335
Asp Asp Ser Gly Val Thr Lys Pro Gly Ser Tyr Ile Phe Glu Leu Phe
            340                 345                 350
Ala Glu Ala Gln Ile Thr Phe Gln Thr Lys Gly Cys Ile Leu Asp Ser
        355                 360                 365
Leu Asp Gln Ile Ile Gln His Leu Ala Gly Arg Ala Gly Val Phe Thr
    370                 375                 380
Asn Thr Ala Gly Leu Gln Lys Leu Ala Asp Ile Ile Gln Ile Val Phe
385                 390                 395                 400
Ser Val Asp Pro Ser Glu Gly Ser Pro Gly Ser Pro Ala Gly Leu Gly
                405                 410                 415
Ala Leu Gln Ser Tyr Lys Val His Ile His Pro Asp Ala Gly His Arg
            420                 425                 430
Arg Thr Ala Gln Arg Ser Asp Ala Trp Ser Thr Thr Ala Ala Arg Lys
        435                 440                 445
Arg Gly Lys Val Leu Ser Tyr Trp Cys Phe Ser Pro Gly His Ser Met
    450                 455                 460
His Glu Leu Val Arg Gln Gly Val Arg Ser Leu Ile Leu Thr Ser Gly
465                 470                 475                 480
Thr Leu Ala Pro Val Ser Ser Phe Ala Leu Glu Met Gln Ile Pro Phe
                485                 490                 495
Pro Val Cys Leu Glu Asn Pro His Ile Ile Asp Lys His Gln Ile Trp
            500                 505                 510
Val Gly Val Val Pro Arg Gly Pro Asp Gly Ala Gln Leu Ser Ser Ala
        515                 520                 525
Phe Asp Arg Arg Phe Ser Glu Glu Cys Leu Ser Ser Leu Gly Lys Ala
    530                 535                 540

Leu Gly Asn Ile Ala Arg Val Val Pro Tyr Gly Leu Leu Ile Phe Phe
545                 550                 555                 560
Pro Ser Tyr Pro Val Met Glu Lys Ser Leu Glu Phe Trp Arg Ala Arg
                565                 570                 575
Asp Leu Ala Arg Lys Met Glu Ala Leu Lys Pro Leu Phe Val Glu Pro
            580                 585                 590
Arg Ser Lys Gly Ser Phe Ser Glu Thr Ile Ser Ala Tyr Tyr Ala Arg
        595                 600                 605
Val Ala Ala Pro Gly Ser Thr Gly Ala Thr Phe Leu Ala Val Cys Arg
    610                 615                 620
Gly Lys Ala Ser Glu Gly Leu Asp Phe Ser Asp Thr Asn Gly Arg Gly
625                 630                 635                 640
Val Ile Val Thr Gly Leu Pro Tyr Pro Pro Arg Met Asp Pro Arg Val
                645                 650                 655
Val Leu Lys Met Gln Phe Leu Asp Glu Met Lys Gly Gln Gly Gly Ala
            660                 665                 670
Gly Gly Gln Phe Leu Ser Gly Gln Glu Trp Tyr Arg Gln Gln Ala Ser
        675                 680                 685
Arg Ala Val Asn Gln Ala Ile Gly Arg Val Ile Arg His Arg Gln Asp
    690                 695                 700
Tyr Gly Ala Val Phe Leu Cys Asp His Arg Phe Ala Phe Ala Asp Ala
705                 710                 715                 720
Arg Ala Gln Leu Pro Ser Trp Val Arg Pro His Val Arg Val Tyr Asp
                725                 730                 735
Asn Phe Gly His Val Ile Arg Asp Val Ala Gln Phe Phe Arg Val Ala
            740                 745                 750
Glu Arg Thr Met Pro Ala Pro Ala Pro Arg Ala Thr Ala Pro Ser Val
        755                 760                 765
Arg Gly Glu Asp Ala Val Ser Glu Ala Lys Ser Pro Gly Pro Phe Phe
    770                 775                 780
Ser Thr Arg Lys Ala Lys Ser Leu Asp Leu His Val Pro Ser Leu Lys
785                 790                 795                 800
Gln Arg Ser Ser Gly Ser Pro Ala Ala Gly Asp Pro Glu Ser Ser Leu
                805                 810                 815
Cys Val Glu Tyr Glu Gln Glu Pro Val Pro Ala Arg Gln Arg Pro Arg
            820                 825                 830
Gly Leu Leu Ala Ala Leu Glu His Ser Glu Gln Arg Ala Gly Ser Pro
        835                 840                 845
Gly Glu Glu Gln Ala His Ser Cys Ser Thr Leu Ser Leu Leu Ser Glu
    850                 855                 860

Lys Arg Pro Ala Glu Glu Pro Arg Gly Gly Arg Lys Lys Ile Arg Leu
865                 870                 875                 880
Val Ser His Pro Glu Glu Pro Val Ala Gly Ala Gln Thr Asp Arg Ala
                885                 890                 895
Lys Leu Phe Met Val Ala Val Lys Gln Glu Leu Ser Gln Ala Asn Phe
            900                 905                 910
Ala Thr Phe Thr Gln Ala Leu Gln Asp Tyr Lys Gly Ser Asp Asp Phe
        915                 920                 925
Ala Ala Leu Ala Ala Cys Leu Gly Pro Leu Phe Ala Glu Asp Pro Lys
    930                 935                 940
Lys His Asn Leu Leu Gln Gly Phe Tyr Gln Phe Val Arg Pro His His
945                 950                 955                 960
Lys Gln Gln Phe Glu Glu Val Cys Ile Gln Leu Thr Gly Arg Gly Cys
                965                 970                 975
Gly Tyr Arg Pro Glu His Ser Ile Pro Arg Arg Gln Arg Ala Gln Pro
            980                 985                 990
Val Leu Asp Pro Thr Gly Arg Thr Ala Pro Asp Pro Lys Leu Thr Val
        995                 1000                1005
Ser Thr Ala Ala Ala Gln Gln Leu Asp Pro Gln Glu His Leu Asn Gln
    1010                1015                1020
Gly Arg Pro His Leu Ser Pro Arg Pro Pro Pro Thr Gly Asp Pro Gly
1025                1030                1035                1040

Ser Gln Pro Gln Trp Gly Ser Gly Val Pro Arg Ala Gly Lys Gln Gly
                1045                1050                1055
Gln His Ala Val Ser Ala Tyr Leu Ala Asp Ala Arg Arg Ala Leu Gly
            1060                1065                1070
Ser Ala Gly Cys Ser Gln Leu Leu Ala Ala Leu Thr Ala Tyr Lys Gln
        1075                1080                1085
Asp Asp Asp Leu Asp Lys Val Leu Ala Val Leu Ala Ala Leu Thr Thr
    1090                1095                1100
Ala Lys Pro Glu Asp Phe Pro Leu Leu His Arg Phe Ser Met Phe Val
1105                1110                1115                1120
Arg Pro His His Lys Gln Arg Phe Ser Gln Thr Cys Thr Asp Leu Thr
                1125                1130                1135
Gly Arg Pro Tyr Pro Gly Met Glu Pro Pro Gly Pro Gln Glu Glu Arg
            1140                1145                1150
Leu Ala Val Pro Pro Val Leu Thr His Arg Ala Pro Gln Pro Gly Pro
        1155                1160                1165
Ser Arg Ser Glu Lys Thr Gly Lys Thr Gln Ser Lys Ile Ser Ser Phe
    1170                1175                1180
Leu Arg Gln Arg Pro Ala Gly Thr Val Gly Ala Gly Gly Glu Asp Ala
1185                1190                1195                1200
Gly Pro Ser Gln Ser Ser Gly Pro Pro His Gly Pro Ala Ala Ser Glu
                1205                1210                1215
Trp Gly Glu Pro His Gly Arg Asp Ile Ala Gly Gln Gln Ala Thr Gly
            1220                1225                1230
Ala Pro Gly Gly Pro Leu Ser Ala Gly Cys Val Cys Gln Gly Cys Gly
        1235                1240                1245
Ala Glu Asp Val Val Pro Phe Gln Cys Pro Ala Cys Asp Phe Gln Arg
    1250                1255                1260
Cys Gln Ala Cys Trp Gln Arg His Leu Gln Ala Ser Arg Met Cys Pro
1265                1270                1275                1280
Ala Cys His Thr Ala Ser Arg Lys Gln Ser Val Met Gln Val Phe Trp
                1285                1290                1295
Pro Glu Pro His Lys Asp His Glu Gly Ala Gly Gly Ala Arg Pro Val
            1300                1305                1310
Ala Ala Val Pro Gly Val Gly Ala Ala Cys Pro Ala Ala Gly Ala Gly
        1315                1320                1325
Cys Thr Arg Ser Gly Arg Asn Thr His Leu Pro Leu Ala Gly Arg Arg
    1330                1335                1340

Asp Arg Gly Ala Ala Gly Val Cys Pro Val Pro Pro Arg His Leu Cys
1345                1350                1355                1360
Ala Ala Ala Val Pro Pro Arg Gln Pro His Asp Val Trp Pro Val Ser
                1365                1370                1375
Thr Ala Pro Leu His Ala Val Leu Glu Leu Pro Gly Ala Leu Pro Leu
            1380                1385                1390
Leu Gln Arg Pro Leu Arg Gly Ala
        1395                1400


<210> 228
<211> 1219
<212> PRT
<213> Homo sapiens

<400> 228
Met Pro Lys Ile Val Leu Asn Gly Val Thr Val Asp Phe Pro Phe Gln
1               5                   10                  15
Pro Tyr Lys Cys Gln Gln Glu Tyr Met Thr Lys Val Leu Glu Cys Leu
            20                  25                  30
Gln Gln Lys Val Asn Gly Ile Leu Glu Ser Pro Thr Gly Thr Gly Lys
        35                  40                  45
Thr Leu Cys Leu Leu Cys Thr Thr Leu Ala Trp Arg Glu His Leu Arg
    50                  55                  60
Asp Gly Ile Ser Ala Arg Lys Ile Ala Glu Arg Ala Gln Gly Glu Leu

65                  70                  75                  80
Phe Pro Asp Arg Ala Leu Ser Ser Trp Gly Asn Ala Ala Ala Ala Ala
                85                  90                  95
Gly Asp Pro Ile Ala Cys Tyr Thr Asp Ile Pro Lys Ile Ile Tyr Ala
            100                 105                 110
Ser Arg Thr His Ser Gln Leu Thr Gln Val Ile Asn Glu Leu Arg Asn
        115                 120                 125
Thr Ser Tyr Arg Pro Lys Val Cys Val Leu Gly Ser Arg Glu Gln Leu
    130                 135                 140
Cys Ile His Pro Glu Val Lys Lys Gln Glu Ser Asn His Leu Gln Ile
145                 150                 155                 160
His Leu Cys Arg Lys Lys Val Ala Ser Arg Ser Cys His Phe Tyr Asn
                165                 170                 175
Asn Val Glu Glu Lys Ser Leu Glu Gln Glu Leu Ala Ser Pro Ile Leu
            180                 185                 190
Asp Ile Glu Asp Leu Val Lys Ser Gly Ser Lys His Arg Val Cys Pro
        195                 200                 205
Tyr Tyr Leu Ser Arg Asn Leu Lys Gln Gln Ala Asp Ile Ile Phe Met
    210                 215                 220
Pro Tyr Asn Tyr Leu Leu Asp Ala Lys Ser Arg Arg Ala His Asn Ile
225                 230                 235                 240
Asp Leu Lys Gly Thr Val Val Ile Phe Asp Glu Ala His Asn Val Glu
                245                 250                 255
Lys Met Cys Glu Glu Ser Ala Ser Phe Asp Leu Thr Pro His Asp Leu
            260                 265                 270
Ala Ser Gly Leu Asp Val Ile Asp Gln Val Leu Glu Glu Gln Thr Lys
        275                 280                 285
Ala Ala Gln Gln Gly Glu Pro His Pro Glu Phe Ser Ala Asp Ser Pro
    290                 295                 300
Ser Pro Gly Leu Asn Met Glu Leu Glu Asp Ile Ala Lys Leu Lys Met
305                 310                 315                 320
Ile Leu Leu Arg Leu Glu Gly Ala Ile Asp Ala Val Glu Leu Pro Gly
                325                 330                 335
Asp Asp Ser Gly Val Thr Lys Pro Gly Ser Tyr Ile Phe Glu Leu Phe
            340                 345                 350
Ala Glu Ala Gln Ile Thr Phe Gln Thr Lys Gly Cys Ile Leu Asp Ser
        355                 360                 365
Leu Asp Gln Ile Ile Gln His Leu Ala Gly Arg Ala Gly Val Phe Thr
    370                 375                 380
Asn Thr Ala Gly Leu Gln Lys Leu Ala Asp Ile Ile Gln Ile Val Phe
385                 390                 395                 400
Ser Val Asp Pro Ser Glu Gly Ser Pro Gly Ser Pro Ala Gly Leu Gly
                405                 410                 415
Ala Leu Gln Ser Tyr Lys Val His Ile His Pro Asp Ala Gly His Arg
            420                 425                 430
Arg Thr Ala Gln Arg Ser Asp Ala Trp Ser Thr Thr Ala Ala Arg Lys
        435                 440                 445
Arg Gly Lys Val Leu Ser Tyr Trp Cys Phe Ser Pro Gly His Ser Met
    450                 455                 460
His Glu Leu Val Arg Gln Gly Val Arg Ser Leu Ile Leu Thr Ser Gly
465                 470                 475                 480
Thr Leu Ala Pro Val Ser Ser Phe Ala Leu Glu Met Gln Ile Pro Phe
                485                 490                 495
Pro Val Cys Leu Glu Asn Pro His Ile Ile Asp Lys His Gln Ile Trp
            500                 505                 510
Val Gly Val Val Pro Arg Gly Pro Asp Gly Ala Gln Leu Ser Ser Ala
        515                 520                 525
Phe Asp Arg Arg Phe Ser Glu Glu Cys Leu Ser Ser Leu Gly Lys Ala
    530                 535                 540
Leu Gly Asn Ile Ala Arg Val Val Pro Tyr Gly Leu Leu Ile Phe Phe
545                 550                 555                 560
Pro Ser Tyr Pro Val Met Glu Lys Ser Leu Glu Phe Trp Arg Ala Arg
                565                 570                 575

Asp Leu Ala Arg Lys Met Glu Ala Leu Lys Pro Leu Phe Val Glu Pro
            580                 585                 590
Arg Ser Lys Gly Ser Phe Ser Glu Thr Ile Ser Ala Tyr Tyr Ala Arg
        595                 600                 605
Val Ala Ala Pro Gly Ser Thr Gly Ala Thr Phe Leu Ala Val Cys Arg
    610                 615                 620
Gly Lys Ala Ser Glu Gly Leu Asp Phe Ser Asp Thr Asn Gly Arg Gly
625                 630                 635                 640
Val Ile Val Thr Gly Leu Pro Tyr Pro Pro Arg Met Asp Pro Arg Val
                645                 650                 655
Val Leu Lys Met Gln Phe Leu Asp Glu Met Lys Gly Gln Gly Gly Ala
            660                 665                 670
Gly Gly Gln Phe Leu Ser Gly Gln Glu Trp Tyr Arg Gln Gln Ala Ser
        675                 680                 685
Arg Ala Val Asn Gln Ala Ile Gly Arg Val Ile Arg His Arg Gln Asp
    690                 695                 700
Tyr Gly Ala Val Phe Leu Cys Asp His Arg Phe Ala Phe Ala Asp Ala
705                 710                 715                 720
Arg Ala Gln Leu Pro Ser Trp Val Arg Pro His Val Arg Val Tyr Asp
                725                 730                 735
Asn Phe Gly His Val Ile Arg Asp Val Ala Gln Phe Phe Arg Val Ala
            740                 745                 750
Glu Arg Thr Met Pro Ala Pro Ala Pro Arg Ala Thr Ala Pro Ser Val
        755                 760                 765
Arg Gly Glu Asp Ala Val Ser Glu Ala Lys Ser Pro Gly Pro Phe Phe
    770                 775                 780
Ser Thr Arg Lys Ala Lys Ser Leu Asp Leu His Val Pro Ser Leu Lys
785                 790                 795                 800
Gln Arg Ser Ser Gly Ser Pro Ala Ala Gly Asp Pro Glu Ser Ser Leu
                805                 810                 815
Cys Val Glu Tyr Glu Gln Glu Pro Val Pro Ala Arg Gln Arg Pro Arg
            820                 825                 830
Gly Leu Leu Ala Ala Leu Glu His Ser Glu Gln Arg Ala Gly Ser Pro
        835                 840                 845

Gly Glu Glu Gln Ala His Ser Cys Ser Thr Leu Ser Leu Leu Ser Glu
    850                 855                 860
Lys Arg Pro Ala Glu Glu Pro Arg Gly Gly Arg Lys Lys Ile Arg Leu
865                 870                 875                 880
Val Ser His Pro Glu Glu Pro Val Ala Gly Ala Gln Thr Asp Arg Ala
                885                 890                 895
Lys Leu Phe Met Val Ala Val Lys Gln Glu Leu Ser Gln Ala Asn Phe
            900                 905                 910
Ala Thr Phe Thr Gln Ala Leu Gln Asp Tyr Lys Gly Ser Asp Asp Phe
        915                 920                 925
Ala Ala Leu Ala Ala Cys Leu Gly Pro Leu Phe Ala Glu Asp Pro Lys
    930                 935                 940
Lys His Asn Leu Leu Gln Gly Phe Tyr Gln Phe Val Arg Pro His His
945                 950                 955                 960
Lys Gln Gln Phe Glu Glu Val Cys Ile Gln Leu Thr Gly Arg Gly Cys
                965                 970                 975
Gly Tyr Arg Pro Glu His Ser Ile Pro Arg Arg Gln Arg Ala Gln Pro
            980                 985                 990
Val Leu Asp Pro Thr Gly Arg Thr Ala Pro Asp Pro Lys Leu Thr Val
        995                 1000                1005
Ser Thr Ala Ala Ala Gln Gln Leu Asp Pro Gln Glu His Leu Asn Gln
    1010                1015                1020
Gly Arg Pro His Leu Ser Pro Arg Pro Pro Pro Thr Gly Asp Pro Gly
1025                1030                1035                1040
Ser Gln Pro Gln Trp Gly Ser Gly Val Pro Arg Ala Gly Lys Gln Gly
                1045                1050                1055
Gln His Ala Val Ser Ala Tyr Leu Ala Asp Ala Arg Arg Ala Leu Gly
            1060                1065                1070

Ser Ala Gly Cys Ser Gln Leu Leu Ala Ala Leu Thr Ala Tyr Lys Gln
        1075                1080                1085
Asp Asp Asp Leu Asp Lys Val Leu Ala Val Leu Ala Ala Leu Thr Thr
    1090                1095                1100
Ala Lys Pro Glu Asp Phe Pro Leu Leu His Arg Phe Ser Met Phe Val
1105                1110                1115                1120
Arg Pro His His Lys Gln Arg Phe Ser Gln Thr Cys Thr Asp Leu Thr
                1125                1130                1135
Gly Arg Pro Tyr Pro Gly Met Glu Pro Pro Gly Pro Gln Glu Glu Arg
            1140                1145                1150
Leu Ala Val Pro Pro Val Leu Thr His Arg Ala Pro Gln Pro Gly Pro
        1155                1160                1165
Ser Arg Ser Glu Lys Thr Gly Lys Thr Gln Ser Lys Ile Ser Ser Phe
    1170                1175                1180
Leu Arg Gln Arg Pro Ala Gly Thr Val Gly Ala Gly Gly Glu Asp Ala
1185                1190                1195                1200
Gly Pro Ser Gln Ser Ser Gly Pro Pro His Gly Pro Ala Ala Ser Glu
                1205                1210                1215
Trp Gly Leu

<210> 229
<211> 545
<212> PRT
<213> Homo sapiens

<400> 229
Met Pro Ala Pro Ala Pro Arg Ala Thr Ala Pro Ser Val Arg Gly Glu
 1               5                  10                  15
Asp Ala Val Ser Glu Ala Lys Ser Pro Gly Pro Phe Phe Ser Thr Arg
            20                  25                  30
Lys Ala Lys Ser Leu Asp Leu His Val Pro Ser Leu Lys Gln Arg Ser
        35                  40                  45

Ser Gly Ser Pro Ala Ala Gly Asp Pro Glu Ser Ser Leu Cys Val Glu
    50                  55                  60
Tyr Glu Gln Glu Pro Val Pro Ala Arg Gln Arg Pro Arg Gly Leu Leu
65                  70                  75                  80
Ala Ala Leu Glu His Ser Glu Gln Arg Ala Gly Ser Pro Gly Glu Glu
                85                  90                  95
Gln Ala His Ser Cys Ser Thr Leu Ser Leu Leu Ser Glu Lys Arg Pro
            100                 105                 110
Ala Glu Glu Pro Arg Gly Gly Arg Lys Lys Ile Arg Leu Val Ser His
        115                 120                 125
Pro Glu Glu Pro Val Ala Gly Ala Gln Thr Asp Arg Ala Lys Leu Phe
    130                 135                 140
Met Val Ala Val Lys Gln Glu Leu Ser Gln Ala Asn Phe Ala Thr Phe
145                 150                 155                 160
Thr Gln Ala Leu Gln Asp Tyr Lys Gly Ser Asp Asp Phe Ala Ala Leu
                165                 170                 175
Ala Ala Cys Leu Gly Pro Leu Phe Ala Glu Asp Pro Lys Lys His Asn
            180                 185                 190
Leu Leu Gln Gly Phe Tyr Gln Phe Val Arg Pro His His Lys Gln Gln
        195                 200                 205
Phe Glu Glu Val Cys Ile Gln Leu Thr Gly Arg Gly Cys Gly Tyr Arg
    210                 215                 220
Pro Glu His Ser Ile Pro Arg Arg Gln Arg Ala Gln Pro Val Leu Asp
225                 230                 235                 240
Pro Thr Gly Arg Thr Ala Pro Asp Pro Lys Leu Thr Val Ser Thr Ala
                245                 250                 255
Ala Ala Gln Gln Leu Asp Pro Gln Glu His Leu Asn Gln Gly Arg Pro
            260                 265                 270
His Leu Ser Pro Arg Pro Pro Pro Thr Gly Asp Pro Gly Ser Gln Pro
        275                 280                 285

Gln Trp Gly Ser Gly Val Pro Arg Ala Gly Lys Gln Gly Gln His Ala
    290                 295                 300
Val Ser Ala Tyr Leu Ala Asp Ala Arg Arg Ala Leu Gly Ser Ala Gly
305                 310                 315                 320
Cys Ser Gln Leu Leu Ala Ala Leu Thr Ala Tyr Lys Gln Asp Asp Asp
                325                 330                 335
Leu Asp Lys Val Leu Ala Val Leu Ala Ala Leu Thr Thr Ala Lys Pro
            340                 345                 350
Glu Asp Phe Pro Leu Leu His Arg Phe Ser Met Phe Val Arg Pro His
        355                 360                 365
His Lys Gln Arg Phe Ser Gln Thr Cys Thr Asp Leu Thr Gly Arg Pro
    370                 375                 380
Tyr Pro Gly Met Glu Pro Pro Gly Pro Gln Glu Glu Arg Leu Ala Val
385                 390                 395                 400
Pro Pro Val Leu Thr His Arg Ala Pro Gln Pro Gly Pro Ser Arg Ser
                405                 410                 415
Glu Lys Thr Gly Lys Thr Gln Ser Lys Ile Ser Ser Phe Leu Arg Gln
            420                 425                 430
Arg Pro Ala Gly Thr Val Gly Ala Gly Gly Glu Asp Ala Gly Pro Ser
        435                 440                 445
Gln Ser Ser Gly Pro Pro His Gly Pro Ala Ala Ser Glu Trp Gly Glu
    450                 455                 460
Pro His Gly Arg Asp Ile Ala Gly Gln Gln Ala Thr Gly Ala Pro Gly
465                 470                 475                 480
Gly Pro Leu Ser Ala Gly Cys Val Cys Gln Gly Cys Gly Ala Glu Asp
                485                 490                 495
Val Val Pro Phe Gln Cys Pro Ala Cys Asp Phe Gln Arg Cys Gln Ala
            500                 505                 510
Cys Trp Gln Arg His Leu Gln Ala Ser Arg Met Cys Pro Ala Cys His
        515                 520                 525

Thr Ala Ser Arg Lys Gln Ser Val Met Gln Val Phe Trp Pro Glu Pro
    530                 535                 540
Gln
545

<210> 230
<211> 300
<212> PRT
<213> Homo sapiens

<400> 230
Met Arg Ala Leu Glu Gly Pro Gly Leu Ser Leu Leu Cys Leu Val Leu
 1               5                  10                  15
Ala Leu Pro Ala Leu Leu Pro Val Pro Ala Val Arg Gly Val Ala Glu
            20                  25                  30
Thr Pro Thr Tyr Pro Trp Arg Asp Ala Glu Thr Gly Glu Arg Leu Val
        35                  40                  45
Cys Ala Gln Cys Pro Pro Gly Thr Phe Val Gln Arg Pro Cys Arg Arg
    50                  55                  60
Asp Ser Pro Thr Thr Cys Gly Pro Cys Pro Pro Arg His Tyr Thr Gln
65                  70                  75                  80
Phe Trp Asn Tyr Leu Glu Arg Cys Arg Tyr Cys Asn Val Leu Cys Gly
                85                  90                  95
Glu Arg Glu Glu Glu Ala Arg Ala Cys His Ala Thr His Asn Arg Ala
            100                 105                 110
Cys Arg Cys Arg Thr Gly Phe Phe Ala His Ala Gly Phe Cys Leu Glu
        115                 120                 125
His Ala Ser Cys Pro Pro Gly Ala Gly Val Ile Ala Pro Gly Thr Pro
    130                 135                 140
Ser Gln Asn Thr Gln Cys Gln Pro Cys Pro Pro Gly Thr Phe Ser Ala
145                 150                 155                 160
Ser Ser Ser Ser Ser Glu Gln Cys Gln Pro His Arg Asn Cys Thr Ala

                165                 170                 175
Leu Gly Leu Ala Leu Asn Val Pro Gly Ser Ser Ser His Asp Thr Leu
            180                 185                 190
Cys Thr Ser Cys Thr Gly Phe Pro Leu Ser Thr Arg Val Pro Gly Ala
        195                 200                 205
Glu Glu Cys Glu Arg Ala Val Ile Asp Phe Val Ala Phe Gln Asp Ile
    210                 215                 220
Ser Ile Lys Arg Leu Gln Arg Leu Leu Gln Ala Leu Glu Ala Pro Glu
225                 230                 235                 240
Gly Trp Gly Pro Thr Pro Arg Ala Gly Arg Ala Ala Leu Gln Leu Lys
                245                 250                 255
Leu Arg Arg Arg Leu Thr Glu Leu Leu Gly Ala Gln Asp Gly Ala Leu
            260                 265                 270
Leu Val Arg Leu Leu Gln Ala Leu Arg Val Ala Arg Met Pro Gly Leu
        275                 280                 285
Glu Arg Ser Val Arg Glu Arg Phe Leu Pro Val His
    290                 295                 300

<210> 231
<211> 300
<212> PRT
<213> Homo sapiens

<400> 231
Met Arg Ala Leu Glu Gly Pro Gly Leu Ser Leu Leu Cys Leu Val Leu
1               5                   10                  15
Ala Leu Pro Ala Leu Leu Pro Val Pro Ala Val Arg Gly Val Ala Glu
            20                  25                  30

Thr Pro Thr Tyr Pro Trp Arg Asp Ala Glu Thr Gly Glu Arg Leu Val
        35                  40                  45
Cys Ala Gln Cys Pro Pro Gly Thr Phe Val Gln Arg Pro Cys Arg Arg
    50                  55                  60
Asp Ser Pro Thr Thr Cys Gly Pro Cys Pro Pro Arg His Tyr Thr Gln
65                  70                  75                  80
Phe Trp Asn Tyr Leu Glu Arg Cys Arg Tyr Cys Asn Val Leu Cys Gly
                85                  90                  95
Glu Arg Glu Glu Glu Ala Arg Ala Cys His Ala Thr His Asn Arg Ala
            100                 105                 110
Cys Arg Cys Arg Thr Gly Phe Phe Ala His Ala Gly Phe Cys Leu Glu
        115                 120                 125
His Ala Ser Cys Pro Pro Gly Ala Gly Val Ile Ala Pro Gly Thr Pro
    130                 135                 140
Ser Gln Asn Thr Gln Cys Gln Pro Cys Pro Pro Gly Thr Phe Ser Ala
145                 150                 155                 160
Ser Ser Ser Ser Ser Glu Gln Cys Gln Pro His Arg Asn Cys Thr Ala
                165                 170                 175
Leu Gly Leu Ala Leu Asn Val Pro Gly Ser Ser Ser His Asp Thr Leu
            180                 185                 190
Cys Thr Ser Cys Thr Gly Phe Pro Leu Ser Thr Arg Val Pro Gly Ala
        195                 200                 205
Glu Glu Cys Glu Arg Ala Val Ile Asp Phe Val Ala Phe Gln Asp Ile
    210                 215                 220
Ser Ile Lys Arg Leu Gln Arg Leu Leu Gln Ala Leu Glu Ala Pro Glu
225                 230                 235                 240
Gly Trp Gly Pro Thr Pro Arg Ala Gly Arg Ala Ala Leu Gln Leu Lys
                245                 250                 255
Leu Arg Arg Arg Leu Thr Glu Leu Leu Gly Ala Gln Asp Gly Ala Leu
            260                 265                 270
Leu Val Arg Leu Leu Gln Ala Leu Arg Val Ala Arg Met Pro Gly Leu
        275                 280                 285
Glu Arg Ser Val Arg Glu Arg Phe Leu Pro Val His
    290                 295                 300

<210> 232
<211> 808
<212> PRT
<213> Homo sapiens

<400> 232
Met Ala Glu Leu Leu Ala Ser Ala Gly Ser Ala Cys Ser Trp Asp Phe
1               5                   10                  15
Pro Arg Ala Pro Pro Ser Phe Pro Pro Pro Ala Ala Ser Arg Gly Gly
            20                  25                  30
Leu Gly Gly Thr Arg Ser Phe Arg Pro His Arg Gly Ala Glu Ser Pro
        35                  40                  45
Arg Pro Gly Arg Asp Arg Asp Gly Val Arg Val Pro Met Ala Ser Ser
    50                  55                  60
Arg Cys Pro Ala Pro Arg Gly Cys Arg Cys Leu Pro Gly Ala Ser Leu
65                  70                  75                  80
Ala Trp Leu Gly Thr Val Leu Leu Leu Leu Ala Asp Trp Val Leu Leu
                85                  90                  95
Arg Thr Ala Leu Pro Arg Ile Phe Ser Leu Leu Val Pro Thr Ala Leu
            100                 105                 110
Pro Leu Leu Arg Val Trp Ala Val Gly Leu Ser Arg Trp Ala Val Leu
        115                 120                 125
Trp Leu Gly Ala Cys Gly Val Leu Arg Ala Thr Val Gly Ser Lys Ser
    130                 135                 140
Glu Asn Ala Gly Ala Gln Gly Trp Leu Ala Ala Leu Lys Pro Leu Ala
145                 150                 155                 160
Ala Ala Leu Gly Leu Ala Leu Pro Gly Leu Ala Leu Phe Arg Glu Leu
                165                 170                 175
Ile Ser Trp Gly Ala Pro Gly Ser Ala Asp Ser Thr Arg Leu Leu His
            180                 185                 190
Trp Gly Ser His Pro Thr Ala Phe Val Val Ser Tyr Ala Ala Ala Leu
        195                 200                 205
Pro Ala Ala Ala Leu Trp His Lys Leu Gly Ser Leu Trp Val Pro Gly
    210                 215                 220
Gly Gln Gly Gly Ser Gly Asn Pro Val Arg Arg Leu Leu Gly Cys Leu
225                 230                 235                 240
Gly Ser Glu Thr Arg Arg Leu Ser Leu Phe Leu Val Leu Val Val Leu
                245                 250                 255
Ser Ser Leu Gly Glu Met Ala Ile Pro Phe Phe Thr Gly Arg Leu Thr
            260                 265                 270
Asp Trp Ile Leu Gln Asp Gly Ser Ala Asp Thr Phe Thr Arg Asn Leu
        275                 280                 285
Thr Leu Met Ser Ile Leu Thr Ile Ala Ser Ala Val Leu Glu Phe Val
    290                 295                 300
Gly Asp Gly Ile Tyr Asn Asn Thr Met Gly His Val His Ser His Leu
305                 310                 315                 320
Gln Gly Glu Val Phe Gly Ala Val Leu Arg Gln Glu Thr Glu Phe Phe
                325                 330                 335
Gln Gln Asn Gln Thr Gly Asn Ile Met Ser Arg Val Thr Glu Asp Thr
            340                 345                 350
Ser Thr Leu Ser Asp Ser Leu Ser Glu Asn Leu Ser Leu Phe Leu Trp
        355                 360                 365
Tyr Leu Val Arg Gly Leu Cys Leu Leu Gly Ile Met Leu Trp Gly Ser
    370                 375                 380
Val Ser Leu Thr Met Val Thr Leu Ile Thr Leu Pro Leu Leu Phe Leu
385                 390                 395                 400
Leu Pro Lys Lys Val Gly Lys Trp Tyr Gln Leu Leu Glu Val Gln Val
                405                 410                 415
Arg Glu Ser Leu Ala Lys Ser Ser Gln Val Ala Ile Glu Ala Leu Ser
            420                 425                 430
Ala Met Pro Thr Val Arg Ser Phe Ala Asn Glu Glu Gly Glu Ala Gln
        435                 440                 445

Lys Phe Arg Glu Lys Leu Gln Glu Ile Lys Thr Leu Asn Gln Lys Glu
    450                 455                 460
Ala Val Ala Tyr Ala Val Asn Ser Trp Thr Thr Ser Ile Ser Gly Met
465                 470                 475                 480
Leu Leu Lys Val Gly Ile Leu Tyr Ile Gly Gly Gln Leu Val Thr Ser
                485                 490                 495
Gly Ala Val Ser Ser Gly Asn Leu Val Thr Phe Val Leu Tyr Gln Met
            500                 505                 510
Gln Phe Thr Gln Ala Val Glu Val Leu Leu Ser Ile Tyr Pro Arg Val
        515                 520                 525
Gln Lys Ala Val Gly Ser Ser Glu Lys Ile Phe Glu Tyr Leu Asp Arg
    530                 535                 540
Thr Pro Arg Cys Pro Pro Ser Gly Leu Leu Thr Pro Leu His Leu Glu
545                 550                 555                 560
Gly Leu Val Gln Phe Gln Asp Val Ser Phe Ala Tyr Pro Asn Arg Pro
                565                 570                 575
Asp Val Leu Val Leu Gln Gly Leu Thr Phe Thr Leu Arg Pro Gly Glu
            580                 585                 590
Val Thr Ala Leu Val Gly Pro Asn Gly Ser Gly Lys Ser Thr Val Ala
        595                 600                 605
Ala Leu Leu Gln Asn Leu Tyr Gln Pro Thr Gly Gly Gln Leu Leu Leu
    610                 615                 620
Asp Gly Lys Pro Leu Pro Gln Tyr Glu His Arg Tyr Leu His Arg Gln
625                 630                 635                 640

Val Ala Ala Val Gly Gln Glu Pro Gln Val Phe Gly Arg Ser Leu Gln
                645                 650                 655
Glu Asn Ile Ala Tyr Gly Leu Thr Gln Lys Pro Thr Met Glu Glu Ile
            660                 665                 670
Thr Ala Ala Ala Val Lys Ser Gly Ala His Ser Phe Ile Ser Gly Leu
        675                 680                 685
Pro Gln Gly Tyr Asp Thr Glu Val Asp Glu Ala Gly Ser Gln Leu Ser
    690                 695                 700
Gly Gly Gln Arg Gln Ala Val Ala Leu Ala Arg Ala Leu Ile Arg Lys
705                 710                 715                 720
Pro Cys Val Leu Ile Leu Asp Asp Ala Thr Ser Ala Leu Asp Ala Asn
                725                 730                 735
Ser Gln Leu Gln Val Glu Gln Leu Leu Tyr Glu Ser Pro Glu Arg Tyr
            740                 745                 750
Ser Arg Ser Val Leu Leu Ile Thr Gln His Leu Ser Leu Val Glu Gln
        755                 760                 765
Ala Asp His Ile Leu Phe Leu Glu Gly Gly Ala Ile Arg Glu Gly Gly
    770                 775                 780
Thr His Gln Gln Leu Met Glu Lys Lys Gly Cys Tyr Trp Ala Met Val
785                 790                 795                 800
Gln Ala Pro Ala Asp Ala Pro Glu
                805

<210> 233
<211> 267
<212> PRT
<213> Homo sapiens

<400> 233
Met Arg Leu Thr Val Leu Cys Ala Val Cys Leu Leu Pro Gly Ser Leu
1               5                   10                  15
Ala Leu Pro Leu Pro Gln Glu Ala Gly Gly Met Ser Glu Leu Gln Trp
            20                  25                  30
Glu Gln Ala Gln Asp Tyr Leu Lys Arg Phe Tyr Leu Tyr Asp Ser Glu
        35                  40                  45
Thr Lys Asn Ala Asn Ser Leu Glu Ala Lys Leu Lys Glu Met Gln Lys
    50                  55                  60
Phe Phe Gly Leu Pro Ile Thr Gly Met Leu Asn Ser Arg Val Ile Glu

65                  70                  75                  80
Ile Met Gln Lys Pro Arg Cys Gly Val Pro Asp Val Ala Glu Tyr Ser
                85                  90                  95
Leu Phe Pro Asn Ser Pro Lys Trp Thr Ser Lys Val Val Thr Tyr Arg
            100                 105                 110
Ile Val Ser Tyr Thr Arg Asp Leu Pro His Ile Thr Val Asp Arg Leu
        115                 120                 125
Val Ser Lys Ala Leu Asn Met Trp Gly Lys Glu Ile Pro Leu His Phe
    130                 135                 140
Arg Lys Val Val Trp Gly Thr Ala Asp Ile Met Ile Gly Phe Ala Arg
145                 150                 155                 160
Gly Ala His Gly Asp Ser Tyr Pro Phe Asp Gly Pro Gly Asn Thr Leu
                165                 170                 175
Ala His Ala Phe Ala Pro Gly Thr Gly Leu Gly Gly Asp Ala His Phe
            180                 185                 190
Asp Glu Asp Glu Arg Trp Thr Asp Gly Ser Ser Leu Gly Ile Asn Phe
        195                 200                 205
Leu Tyr Ala Ala Thr His Glu Leu Gly His Ser Leu Gly Met Gly His
    210                 215                 220
Ser Ser Asp Pro Asn Ala Val Met Tyr Pro Thr Tyr Gly Asn Gly Asp
225                 230                 235                 240
Pro Gln Asn Phe Lys Leu Ser Gln Asp Asp Ile Lys Gly Ile Gln Lys
                245                 250                 255
Leu Tyr Gly Lys Arg Ser Asn Ser Arg Lys Lys
            260                 265

<210> 234
<211> 539
<212> PRT
<213> Homo sapiens

<400> 234
Met Pro Ile Glu Ile Val Cys Lys Ile Lys Phe Ala Glu Glu Asp Ala
1               5                   10                  15
Lys Pro Lys Glu Lys Glu Ala Gly Asp Glu Gln Ser Leu Leu Gly Ala
            20                  25                  30
Val Ala Pro Gly Ala Ala Pro Arg Asp Leu Ala Thr Phe Ala Ser Thr
        35                  40                  45
Ser Thr Leu His Gly Leu Gly Arg Ala Cys Gly Pro Gly Pro His Gly
    50                  55                  60
Leu Arg Arg Thr Leu Trp Ala Leu Ala Leu Leu Thr Ser Leu Ala Ala
65                  70                  75                  80
Phe Leu Tyr Gln Ala Ala Gly Leu Ala Arg Gly Tyr Leu Thr Arg Pro
                85                  90                  95
His Leu Val Ala Met Asp Pro Ala Ala Pro Ala Pro Val Ala Gly Phe
            100                 105                 110
Pro Ala Val Thr Leu Cys Asn Ile Asn Arg Phe Arg His Ser Ala Leu
        115                 120                 125
Ser Asp Ala Asp Ile Phe His Leu Ala Asn Leu Thr Gly Leu Pro Pro
    130                 135                 140
Lys Asp Arg Asp Gly His Arg Ala Ala Gly Leu Arg Tyr Pro Glu Pro
145                 150                 155                 160
Asp Met Val Asp Ile Leu Asn Arg Thr Gly His Gln Leu Ala Asp Met
                165                 170                 175
Leu Lys Ser Cys Asn Phe Ser Gly His His Cys Ser Ala Ser Asn Phe
            180                 185                 190
Ser Val Val Tyr Thr Arg Tyr Gly Lys Cys Tyr Thr Phe Asn Ala Asp
        195                 200                 205
Pro Arg Ser Ser Leu Pro Ser Arg Ala Gly Gly Met Gly Ser Gly Leu
    210                 215                 220
Glu Ile Met Leu Asp Ile Gln Gln Glu Glu Tyr Leu Pro Ile Trp Arg
225                 230                 235                 240
Glu Thr Asn Glu Thr Ser Phe Glu Ala Gly Ile Arg Val Gln Ile His

                245                 250                 255
Ser Gln Glu Glu Pro Pro Tyr Ile His Gln Leu Gly Phe Gly Val Ser
            260                 265                 270
Pro Gly Phe Gln Thr Phe Val Ser Cys Gln Glu Gln Arg Leu Thr Tyr
        275                 280                 285
Leu Pro Gln Pro Trp Gly Asn Cys Arg Ala Glu Ser Glu Leu Arg Glu
    290                 295                 300
Pro Glu Leu Gln Gly Tyr Ser Ala Tyr Ser Val Ser Ala Cys Arg Leu
305                 310                 315                 320
Arg Cys Glu Lys Glu Ala Val Leu Gln Arg Cys His Cys Arg Met Val
                325                 330                 335
His Met Pro Gly Asn Glu Thr Ile Cys Pro Pro Asn Ile Tyr Ile Glu
            340                 345                 350
Cys Ala Asp His Thr Leu Asp Ser Leu Gly Gly Gly Pro Glu Gly Pro
        355                 360                 365
Cys Phe Cys Pro Thr Pro Cys Asn Leu Thr Arg Tyr Gly Lys Glu Ile
    370                 375                 380
Ser Met Val Arg Ile Pro Asn Arg Gly Ser Ala Arg Tyr Leu Ala Arg
385                 390                 395                 400
Lys Tyr Asn Arg Asn Glu Thr Tyr Ile Arg Glu Asn Phe Leu Val Leu
                405                 410                 415
Asp Val Phe Phe Glu Ala Leu Thr Ser Glu Ala Met Glu Gln Arg Ala
            420                 425                 430
Ala Tyr Gly Leu Ser Ala Leu Leu Gly Asp Leu Gly Gly Gln Met Gly
        435                 440                 445
Leu Phe Ile Gly Ala Ser Ile Leu Thr Leu Leu Glu Ile Leu Asp Tyr
    450                 455                 460
Ile Tyr Glu Val Ser Trp Asp Arg Leu Lys Arg Val Trp Arg Arg Pro
465                 470                 475                 480
Lys Thr Pro Leu Arg Thr Ser Thr Gly Gly Ile Ser Thr Leu Gly Leu
                485                 490                 495
Gln Glu Leu Lys Glu Gln Ser Pro Cys Pro Ser Leu Gly Arg Ala Glu
            500                 505                 510
Gly Gly Gly Val Ser Ser Leu Leu Pro Asn His His His Pro His Gly
        515                 520                 525
Pro Pro Gly Gly Leu Phe Glu Asp Phe Ala Cys
    530                 535

<210> 235
<211> 696
<212> PRT
<213> Homo sapiens

<400> 235
Met Glu Val Ser Ser Pro Ser Pro Ala Gln Arg Leu Arg Arg Lys Lys
1               5                   10                  15
Arg Pro Met Val Gln Gly Pro Ala Gly Cys Gln Val Phe Gln Pro Ser
            20                  25                  30
Pro Ser Gly Gly Thr Ala Gly Asp Pro Gly Gly Leu Ser Asp Pro Phe
        35                  40                  45
Tyr Pro Pro Arg Ser Gly Ser Leu Ala Leu Gly Asp Pro Ser Ser Asp
    50                  55                  60
Pro Ala Cys Ser Gln Ser Gly Pro Met Glu Ala Glu Glu Asp Ser Leu
65                  70                  75                  80
Pro Glu Gln Pro Glu Asp Ser Ala Gln Leu Gln Gln Glu Lys Pro Ser
                85                  90                  95
Leu Tyr Ile Gly Val Arg Gly Thr Val Val Arg Ser Met Gln Glu Val
            100                 105                 110
Leu Trp Thr Arg Leu Arg Glu Leu Pro Asp Pro Val Leu Ser Glu Glu
        115                 120                 125
Val Val Glu Gly Ile Ala Ala Gly Ile Glu Ala Ala Leu Trp Asp Leu
    130                 135                 140
Thr Gln Gly Thr Asn Gly Arg Tyr Lys Thr Lys Tyr Arg Ser Leu Leu

145                 150                 155                 160
Phe Asn Leu Arg Asp Pro Arg Asn Leu Asp Leu Phe Leu Lys Val Val
                165                 170                 175
His Gly Asp Val Thr Pro Tyr Asp Leu Val Arg Met Ser Ser Met Gln
            180                 185                 190
Leu Ala Pro Gln Glu Leu Ala Arg Trp Arg Asp Gln Glu Glu Lys Arg
        195                 200                 205
Gly Leu Asn Ile Ile Glu Gln Gln Gln Lys Glu Pro Cys Arg Leu Pro
    210                 215                 220
Ala Ser Lys Met Thr His Lys Gly Glu Val Glu Ile Gln Arg Asp Met
225                 230                 235                 240
Asp Gln Thr Leu Thr Leu Glu Asp Leu Val Gly Pro Gln Met Phe Met
                245                 250                 255
Asp Cys Ser Pro Gln Ala Leu Pro Ile Ala Ser Glu Asp Thr Thr Gly
            260                 265                 270
Gln His Asp His His Phe Leu Asp Pro Asn Cys His Ile Cys Lys Asp
        275                 280                 285
Trp Glu Pro Ser Asn Glu Leu Leu Gly Ser Phe Glu Ala Ala Lys Ser
    290                 295                 300
Cys Gly Asp Asn Ile Phe Gln Lys Ala Leu Ser Gln Thr Pro Met Pro
305                 310                 315                 320
Ala Pro Glu Met Pro Lys Thr Arg Glu Leu Ser Pro Thr Glu Pro Gln
                325                 330                 335
Asp Arg Val Pro Pro Ser Gly Leu His Val Pro Ala Ala Pro Thr Lys
            340                 345                 350
Ala Leu Pro Cys Leu Pro Pro Trp Glu Gly Val Leu Asp Met Phe Ser
        355                 360                 365
Ile Lys Arg Phe Arg Ala Arg Ala Gln Leu Val Ser Gly His Ser Cys
    370                 375                 380
Arg Leu Val Gln Ala Leu Pro Thr Val Ile Arg Ser Ala Gly Cys Ile
385                 390                 395                 400
Pro Ser Asn Ile Val Trp Asp Leu Leu Ala Ser Ile Cys Pro Ala Lys
                405                 410                 415
Ala Lys Asp Val Cys Val Val Arg Leu Cys Pro His Gly Ala Arg Asp
            420                 425                 430
Thr Gln Asn Cys Arg Leu Leu Tyr Ser Tyr Leu Asn Asp Arg Gln Arg
        435                 440                 445
His Gly Leu Ala Ser Val Glu His Met Gly Met Val Leu Leu Pro Leu
    450                 455                 460
Pro Ala Phe Gln Pro Leu Pro Thr Arg Leu Arg Pro Leu Gly Gly Pro
465                 470                 475                 480
Gly Leu Glu Val Thr His Ser Ser Leu Leu Leu Ala Val Leu Leu Pro
                485                 490                 495
Lys Glu Gly Leu Pro Asp Thr Ala Gly Ser Ser Pro Trp Leu Gly Lys
            500                 505                 510
Val Gln Lys Met Val Ser Phe Asn Ser Lys Val Glu Lys Arg Tyr Tyr
        515                 520                 525
Gln Pro Asp Asp Arg Arg Pro Asn Val Pro Leu Lys Gly Thr Pro Pro
    530                 535                 540
Pro Gly Gly Ala Trp Gln Gln Ser Gln Gly Arg Gly Ser Ile Ala Pro
545                 550                 555                 560
Arg Gly Ile Ser Ala Trp Gln Arg Pro Pro Arg Gly Arg Gly Arg Leu
                565                 570                 575
Trp Pro Glu Pro Glu Asn Trp Gln His Pro Gly Arg Gly Gln Trp Pro
            580                 585                 590
Pro Glu Pro Gly Leu Arg Gln Ser Gln His Pro Tyr Ser Val Ala Pro
        595                 600                 605
Ala Gly His Gly Phe Gly Arg Gly Gln His Phe His Arg Asp Ser Cys
    610                 615                 620
Pro His Gln Ala Leu Leu Arg His Leu Glu Ser Leu Ala Thr Met Ser
625                 630                 635                 640
His Gln Leu Gln Ala Leu Leu Cys Pro Gln Thr Lys Ser Ser Ile Pro
                645                 650                 655

Arg Pro Leu Gln Arg Leu Ser Ser Ala Leu Ala Ala Pro Glu Pro Pro
            660                 665                 670
Gly Pro Ala Arg Asp Ser Ser Leu Gly Pro Thr Asp Glu Ala Gly Ser
        675                 680                 685
Glu Cys Pro Phe Pro Arg Lys Ala
    690                 695

<210> 236
<211> 367
<212> PRT
<213> Homo sapiens

<400> 236
Met Ser Lys Arg Ser Trp Trp Ala Gly Ser Arg Lys Pro Pro Arg Glu
1               5                   10                  15
Met Leu Lys Leu Ser Gly Ser Asp Ser Ser Gln Ser Met Asn Gly Leu
            20                  25                  30
Glu Val Ala Pro Pro Gly Leu Ile Thr Asn Phe Ser Leu Ala Thr Ala
        35                  40                  45
Glu Gln Cys Gly Gln Glu Thr Pro Leu Glu Asn Met Leu Phe Ala Ser
    50                  55                  60
Phe Tyr Leu Leu Asp Phe Ile Leu Ala Leu Val Gly Asn Thr Leu Ala
65                  70                  75                  80
Leu Trp Leu Phe Ile Arg Asp His Lys Ser Gly Thr Pro Ala Asn Val
                85                  90                  95
Phe Leu Met His Leu Ala Val Ala Asp Leu Ser Cys Val Leu Val Leu
            100                 105                 110
Pro Thr Arg Leu Val Tyr His Phe Ser Gly Asn His Trp Pro Phe Gly
        115                 120                 125
Glu Ile Ala Cys Arg Leu Thr Gly Phe Leu Phe Tyr Leu Asn Met Tyr
    130                 135                 140
Ala Ser Ile Tyr Phe Leu Thr Cys Ile Ser Ala Asp Arg Phe Leu Ala
145                 150                 155                 160
Ile Val His Pro Val Lys Ser Leu Lys Leu Arg Arg Pro Leu Tyr Ala
                165                 170                 175
His Leu Ala Cys Ala Phe Leu Trp Val Val Val Ala Val Ala Met Ala
            180                 185                 190
Pro Leu Leu Val Ser Pro Gln Thr Val Gln Thr Asn His Thr Val Val
        195                 200                 205
Cys Leu Gln Leu Tyr Arg Glu Lys Ala Ser His His Ala Leu Val Ser
    210                 215                 220
Leu Ala Val Ala Phe Thr Phe Pro Phe Ile Thr Thr Val Thr Cys Tyr
225                 230                 235                 240
Leu Leu Ile Ile Arg Ser Leu Arg Gln Gly Leu Arg Val Glu Lys Arg
                245                 250                 255
Leu Lys Thr Lys Ala Val Arg Met Ile Ala Ile Val Leu Ala Ile Phe
            260                 265                 270
Leu Val Cys Phe Val Pro Tyr His Val Asn Arg Ser Val Tyr Val Leu
        275                 280                 285
His Tyr Arg Ser His Gly Ala Ser Cys Ala Thr Gln Arg Ile Leu Ala
    290                 295                 300
Leu Ala Asn Arg Ile Thr Ser Cys Leu Thr Ser Leu Asn Gly Ala Leu
305                 310                 315                 320
Asp Pro Ile Met Tyr Phe Phe Val Ala Glu Lys Phe Arg His Ala Leu
                325                 330                 335
Cys Asn Leu Leu Cys Gly Lys Arg Leu Lys Gly Pro Pro Pro Ser Phe
            340                 345                 350
Glu Gly Lys Thr Asn Glu Ser Ser Leu Ser Ala Lys Ser Glu Leu
        355                 360                 365

<210> 237
<211> 234
<212> PRT

<213> Homo sapiens

<400> 237
Met Arg Lys Thr Arg Leu Trp Gly Leu Leu Trp Met Leu Phe Val Ser
1               5                   10                  15
Glu Leu Arg Ala Ala Thr Lys Leu Thr Glu Glu Lys Tyr Glu Leu Lys
            20                  25                  30
Glu Gly Gln Thr Leu Asp Val Lys Cys Asp Tyr Thr Leu Glu Lys Phe
        35                  40                  45
Ala Ser Ser Gln Lys Ala Trp Gln Ile Ile Arg Asp Gly Glu Met Pro
    50                  55                  60
Lys Thr Leu Ala Cys Thr Glu Arg Pro Ser Lys Asn Ser His Pro Val
65                  70                  75                  80
Gln Val Gly Arg Ile Ile Leu Glu Asp Tyr His Asp His Gly Leu Leu
                85                  90                  95
Arg Val Arg Met Val Asn Leu Gln Val Glu Asp Ser Gly Leu Tyr Gln
            100                 105                 110
Cys Val Ile Tyr Gln Pro Pro Lys Glu Pro His Met Leu Phe Asp Arg
        115                 120                 125
Ile Arg Leu Val Val Thr Lys Gly Phe Ser Gly Thr Pro Gly Ser Asn
    130                 135                 140
Glu Asn Ser Thr Gln Asn Val Tyr Lys Ile Pro Pro Thr Thr Thr Lys
145                 150                 155                 160
Ala Leu Cys Pro Leu Tyr Thr Ser Pro Arg Thr Val Thr Gln Ala Pro
                165                 170                 175
Pro Lys Ser Thr Ala Asp Val Ser Thr Pro Asp Ser Glu Ile Asn Leu
            180                 185                 190
Thr Asn Val Thr Asp Ile Ile Arg Val Pro Val Phe Asn Ile Val Ile
        195                 200                 205
Leu Leu Ala Gly Gly Phe Leu Ser Lys Ser Leu Val Phe Ser Val Leu
    210                 215                 220
Phe Ala Val Thr Leu Arg Ser Phe Val Pro
225                 230

<210> 238
<211> 352
<212> PRT
<213> Homo sapiens

<400> 238
Met Glu Gly Ile Ser Ile Tyr Thr Ser Asp Asn Tyr Thr Glu Glu Met
1               5                   10                  15
Gly Ser Gly Asp Tyr Asp Ser Met Lys Glu Pro Cys Phe Arg Glu Glu
            20                  25                  30
Asn Ala Asn Phe Asn Lys Ile Phe Leu Pro Thr Ile Tyr Ser Ile Ile
        35                  40                  45
Phe Leu Thr Gly Ile Val Gly Asn Gly Leu Val Ile Leu Val Met Gly
    50                  55                  60
Tyr Gln Lys Lys Leu Arg Ser Met Thr Asp Lys Tyr Arg Leu His Leu
65                  70                  75                  80
Ser Val Ala Asp Leu Leu Phe Val Ile Thr Leu Pro Phe Trp Ala Val
                85                  90                  95
Asp Ala Val Ala Asn Trp Tyr Phe Gly Asn Phe Leu Cys Lys Ala Val
            100                 105                 110
His Val Ile Tyr Thr Val Asn Leu Tyr Ser Ser Val Leu Ile Leu Ala
        115                 120                 125
Phe Ile Ser Leu Asp Arg Tyr Leu Ala Ile Val His Ala Thr Asn Ser
    130                 135                 140
Gln Arg Pro Arg Lys Leu Leu Ala Glu Lys Val Val Tyr Val Gly Val
145                 150                 155                 160
Trp Ile Pro Ala Leu Leu Leu Thr Ile Pro Asp Phe Ile Phe Ala Asn
                165                 170                 175
Val Ser Glu Ala Asp Asp Arg Tyr Ile Cys Asp Arg Phe Tyr Pro Asn

            180                 185                 190
Asp Leu Trp Val Val Val Phe Gln Phe Gln His Ile Met Val Gly Leu
        195                 200                 205
Ile Leu Pro Gly Ile Val Ile Leu Ser Cys Tyr Cys Ile Ile Ile Ser
    210                 215                 220
Lys Leu Ser His Ser Lys Gly His Gln Lys Arg Lys Ala Leu Lys Thr
225                 230                 235                 240
Thr Val Ile Leu Ile Leu Ala Phe Phe Ala Cys Trp Leu Pro Tyr Tyr
                245                 250                 255
Ile Gly Ile Ser Ile Asp Ser Phe Ile Leu Leu Glu Ile Ile Lys Gln
            260                 265                 270
Gly Cys Glu Phe Glu Asn Thr Val His Lys Trp Ile Ser Ile Thr Glu
        275                 280                 285
Ala Leu Ala Phe Phe His Cys Cys Leu Asn Pro Ile Leu Tyr Ala Phe
    290                 295                 300
Leu Gly Ala Lys Phe Lys Thr Ser Ala Gln His Ala Leu Thr Ser Val
305                 310                 315                 320
Ser Arg Gly Ser Ser Leu Lys Ile Leu Ser Lys Gly Lys Arg Gly Gly
                325                 330                 335
His Ser Ser Val Ser Thr Glu Ser Glu Ser Ser Ser Phe His Ser Ser
            340                 345                 350

<210> 239
<211> 359
<212> PRT
<213> Homo sapiens

<400> 239
Met His Pro Gln Val Val Ile Leu Ser Leu Ile Leu His Leu Ala Asp
1               5                   10                  15
Ser Val Ala Gly Ser Val Lys Val Gly Gly Glu Ala Gly Pro Ser Val
            20                  25                  30
Thr Leu Pro Cys His Tyr Ser Gly Ala Val Thr Ser Met Cys Trp Asn
        35                  40                  45
Arg Gly Ser Cys Ser Leu Phe Thr Cys Gln Asn Gly Ile Val Trp Thr
    50                  55                  60
Asn Gly Thr His Val Thr Tyr Arg Lys Asp Thr Arg Tyr Lys Leu Leu
65                  70                  75                  80
Gly Asp Leu Ser Arg Arg Asp Val Ser Leu Thr Ile Glu Asn Thr Ala
                85                  90                  95
Val Ser Asp Ser Gly Val Tyr Cys Cys Arg Val Glu His Arg Gly Trp
            100                 105                 110
Phe Asn Asp Met Lys Ile Thr Val Ser Leu Glu Ile Val Pro Pro Lys
        115                 120                 125
Val Thr Thr Thr Pro Ile Val Thr Thr Val Pro Thr Val Thr Thr Val
    130                 135                 140
Arg Thr Ser Thr Thr Val Pro Thr Thr Thr Thr Val Pro Thr Thr Thr
145                 150                 155                 160
Val Pro Thr Thr Met Ser Ile Pro Thr Thr Thr Thr Val Pro Thr Thr
                165                 170                 175
Met Thr Val Ser Thr Thr Thr Ser Val Pro Thr Thr Thr Ser Ile Pro
            180                 185                 190
Thr Thr Thr Ser Val Pro Val Thr Thr Thr Val Ser Thr Phe Val Pro
        195                 200                 205
Pro Met Pro Leu Pro Arg Gln Asn His Glu Pro Val Ala Thr Ser Pro
    210                 215                 220
Ser Ser Pro Gln Pro Ala Glu Thr His Pro Thr Thr Leu Gln Gly Ala
225                 230                 235                 240
Ile Arg Arg Glu Pro Thr Ser Ser Pro Leu Tyr Ser Tyr Thr Thr Asp
                245                 250                 255
Gly Asn Asp Thr Val Thr Glu Ser Ser Asp Gly Leu Trp Asn Asn Asn
            260                 265                 270
Gln Thr Gln Leu Phe Leu Glu His Ser Leu Leu Thr Ala Asn Thr Thr

275                 280                 285
Lys Gly Ile Tyr Ala Gly Val Cys Ile Ser Val Leu Val Leu Leu Ala
    290                 295                 300
Leu Leu Gly Val Ile Ile Ala Lys Lys Tyr Phe Phe Lys Lys Glu Val
305                 310                 315                 320
Gln Gln Leu Ser Val Ser Phe Ser Ser Leu Gln Ile Lys Ala Leu Gln
                325                 330                 335
Asn Ala Val Glu Lys Glu Val Gln Ala Glu Asp Asn Ile Tyr Ile Glu
            340                 345                 350
Asn Ser Leu Tyr Ala Thr Asp
        355
<210> 240
<211> 790
<212> PRT
<213> Homo sapiens

<400> 240
Met Arg Thr Tyr Arg Tyr Phe Leu Leu Leu Phe Trp Val Gly Gln Pro
1               5                   10                  15
Tyr Pro Thr Leu Ser Thr Pro Leu Ser Lys Arg Thr Ser Gly Phe Pro
            20                  25                  30
Ala Lys Lys Arg Ala Leu Glu Leu Ser Gly Asn Ser Lys Asn Glu Leu
        35                  40                  45
Asn Arg Ser Lys Arg Ser Trp Met Trp Asn Gln Phe Phe Leu Leu Glu
    50                  55                  60
Glu Tyr Thr Gly Ser Asp Tyr Gln Tyr Val Gly Lys Leu His Ser Asp
65                  70                  75                  80
Gln Asp Arg Gly Asp Gly Ser Leu Lys Tyr Ile Leu Ser Gly Asp Gly
                85                  90                  95
Ala Gly Asp Leu Phe Ile Ile Asn Glu Asn Thr Gly Asp Ile Gln Ala
            100                 105                 110
Thr Lys Arg Leu Asp Arg Glu Glu Lys Pro Val Tyr Ile Leu Arg Ala
        115                 120                 125
Gln Ala Ile Asn Arg Arg Thr Gly Arg Pro Val Glu Pro Glu Ser Glu
    130                 135                 140
Phe Ile Ile Lys Ile His Asp Ile Asn Asp Asn Glu Pro Ile Phe Thr
145                 150                 155                 160
Lys Glu Val Tyr Thr Ala Thr Val Pro Glu Met Ser Asp Val Gly Thr
                165                 170                 175
Phe Val Val Gln Val Thr Ala Thr Asp Ala Asp Asp Pro Thr Tyr Gly
            180                 185                 190
Asn Ser Ala Lys Val Val Tyr Ser Ile Leu Gln Gly Gln Pro Tyr Phe
        195                 200                 205
Ser Val Glu Ser Glu Thr Gly Ile Ile Lys Thr Ala Leu Leu Asn Met
    210                 215                 220
Asp Arg Glu Asn Arg Glu Gln Tyr Gln Val Val Ile Gln Ala Lys Asp
225                 230                 235                 240
Met Gly Gly Gln Met Gly Gly Leu Ser Gly Thr Thr Thr Val Asn Ile
                245                 250                 255
Thr Leu Thr Asp Val Asn Asp Asn Pro Pro Arg Phe Pro Gln Ser Thr
            260                 265                 270
Tyr Gln Phe Lys Thr Pro Glu Ser Ser Pro Pro Gly Thr Pro Ile Gly
        275                 280                 285
Arg Ile Lys Ala Ser Asp Ala Asp Val Gly Glu Asn Ala Glu Ile Glu
    290                 295                 300
Tyr Ser Ile Thr Asp Gly Glu Gly Leu Asp Met Phe Asp Val Ile Thr
305                 310                 315                 320
Asp Gln Glu Thr Gln Glu Gly Ile Ile Thr Val Lys Lys Leu Leu Asp
                325                 330                 335
Phe Glu Lys Lys Lys Val Tyr Thr Leu Lys Val Glu Ala Ser Asn Pro
            340                 345                 350
Tyr Val Glu Pro Arg Phe Leu Tyr Leu Gly Pro Phe Lys Asp Ser Ala
        355                 360                 365

Thr Val Arg Ile Val Val Glu Asp Val Asp Glu Pro Pro Val Phe Ser
    370                 375                 380
Lys Leu Ala Tyr Ile Leu Gln Ile Arg Glu Asp Ala Gln Ile Asn Thr
385                 390                 395                 400
Thr Ile Gly Ser Val Thr Ala Gln Asp Pro Asp Ala Ala Arg Asn Pro
                405                 410                 415
Val Lys Tyr Ser Val Asp Arg His Thr Asp Met Asp Arg Ile Phe Asn
            420                 425                 430

Ile Asp Ser Gly Asn Gly Ser Ile Phe Thr Ser Lys Leu Leu Asp Arg
        435                 440                 445
Glu Thr Leu Leu Trp His Asn Ile Thr Val Ile Ala Thr Glu Ile Asn
    450                 455                 460
Asn Pro Lys Gln Ser Ser Arg Val Pro Leu Tyr Ile Lys Val Leu Asp
465                 470                 475                 480
Val Asn Asp Asn Ala Pro Glu Phe Ala Glu Phe Tyr Glu Thr Phe Val
                485                 490                 495
Cys Glu Lys Ala Lys Ala Asp Gln Leu Ile Gln Thr Leu His Ala Val
            500                 505                 510
Asp Lys Asp Asp Pro Tyr Ser Gly His Gln Phe Ser Phe Ser Leu Ala
        515                 520                 525
Pro Glu Ala Ala Ser Gly Ser Asn Phe Thr Ile Gln Asp Asn Lys Asp
    530                 535                 540
Asn Thr Ala Gly Ile Leu Thr Arg Lys Asn Gly Tyr Asn Arg His Glu
545                 550                 555                 560
Met Ser Thr Tyr Leu Leu Pro Val Val Ile Ser Asp Asn Asp Tyr Pro
                565                 570                 575
Val Gln Ser Ser Thr Gly Thr Val Thr Val Arg Val Cys Ala Cys Asp
            580                 585                 590
His His Gly Asn Met Gln Ser Cys His Ala Glu Ala Leu Ile His Pro
        595                 600                 605
Thr Gly Leu Ser Thr Gly Ala Leu Val Ala Ile Leu Leu Cys Ile Val
    610                 615                 620
Ile Leu Leu Val Thr Val Val Leu Phe Ala Ala Leu Arg Arg Gln Arg
625                 630                 635                 640
Lys Lys Glu Pro Leu Ile Ile Ser Lys Glu Asp Ile Arg Asp Asn Ile
                645                 650                 655
Val Ser Tyr Asn Asp Glu Gly Gly Gly Glu Glu Asp Thr Gln Ala Phe
            660                 665                 670
Asp Ile Gly Thr Leu Arg Asn Pro Glu Ala Ile Glu Asp Asn Lys Leu
        675                 680                 685
Arg Arg Asp Ile Val Pro Glu Ala Leu Phe Leu Pro Arg Arg Thr Pro
    690                 695                 700
Thr Ala Arg Asp Asn Thr Asp Val Arg Asp Phe Ile Asn Gln Arg Leu
705                 710                 715                 720
Lys Glu Asn Asp Thr Asp Pro Thr Ala Pro Pro Tyr Asp Ser Leu Ala
                725                 730                 735
Thr Tyr Ala Tyr Glu Gly Thr Gly Ser Val Ala Asp Ser Leu Ser Ser
            740                 745                 750
Leu Glu Ser Val Thr Thr Asp Ala Asp Gln Asp Tyr Asp Tyr Leu Ser
        755                 760                 765
Asp Trp Gly Pro Arg Phe Lys Lys Leu Ala Asp Met Tyr Gly Gly Val
    770                 775                 780
Asp Ser Asp Lys Asp Ser
785                 790

<210> 241
<211> 557
<212> PRT
<213> Homo sapiens

<400> 241
Met Arg Arg Leu Thr Arg Arg Leu Val Leu Pro Val Phe Gly Val Leu

1               5                   10                  15
Trp Ile Thr Val Leu Leu Phe Phe Trp Val Thr Lys Arg Lys Leu Glu
            20                  25                  30
Val Pro Thr Gly Pro Glu Val Gln Thr Pro Lys Val Trp Ser Leu Phe
        35                  40                  45
Phe Lys Val Ala Gly Met Ser Pro Trp Ala Pro Gln Val Pro Val Ser
    50                  55                  60
Pro Thr Pro Pro Tyr Gln Arg Gly His Leu Pro Thr Gly Gly His Leu
65                  70                  75                  80
Ala Val Cys His Phe Pro Cys Leu Leu Gln Glu Ala Gln Phe His Leu
                85                  90                  95
Gln Thr Gln Val Phe Leu Gln Val Arg Cys Thr Leu Leu Val Tyr Cys
            100                 105                 110
Thr Asp Leu Pro Pro Thr Ser Ile Ile Ile Thr Phe His Asn Glu Ala
        115                 120                 125
Arg Ser Thr Leu Leu Arg Thr Ile Arg Ser Val Leu Asn Arg Thr Pro
    130                 135                 140
Thr His Leu Ile Arg Glu Ile Ile Leu Val Asp Asp Phe Ser Asn Asp
145                 150                 155                 160
Pro Asp Asp Cys Lys Gln Leu Ile Lys Leu Pro Lys Val Lys Cys Leu
                165                 170                 175
Arg Asn Asn Glu Arg Gln Gly Leu Val Arg Ser Arg Ile Arg Gly Ala
            180                 185                 190
Asp Ile Ala Gln Gly Thr Thr Leu Thr Phe Leu Asp Ser His Cys Glu
        195                 200                 205
Val Asn Arg Asp Trp Leu Gln Pro Leu Leu His Arg Val Lys Glu Asp
    210                 215                 220
Tyr Thr Arg Val Val Cys Pro Val Ile Asp Ile Ile Asn Leu Asp Thr
225                 230                 235                 240
Phe Thr Tyr Ile Glu Ser Ala Ser Glu Leu Arg Gly Gly Phe Asp Trp
                245                 250                 255
Ser Leu His Phe Gln Trp Glu Gln Leu Ser Pro Glu Gln Lys Ala Arg
            260                 265                 270
Arg Leu Asp Pro Thr Glu Pro Ile Arg Thr Pro Ile Ile Ala Gly Gly
        275                 280                 285
Leu Phe Val Ile Asp Lys Ala Trp Phe Asp Tyr Leu Gly Lys Tyr Asp
    290                 295                 300
Met Asp Met Asp Ile Trp Gly Gly Glu Asn Phe Glu Ile Ser Phe Arg
305                 310                 315                 320
Val Trp Met Cys Gly Gly Ser Leu Glu Ile Val Pro Cys Ser Arg Val
                325                 330                 335
Gly His Val Phe Arg Lys Lys His Pro Tyr Val Phe Pro Asp Gly Asn
            340                 345                 350
Ala Asn Thr Tyr Ile Lys Asn Thr Lys Arg Thr Ala Glu Val Trp Met
        355                 360                 365
Asp Glu Tyr Lys Gln Tyr Tyr Tyr Ala Ala Arg Pro Phe Ala Leu Glu
    370                 375                 380
Arg Pro Phe Gly Asn Val Glu Ser Arg Leu Asp Leu Arg Lys Asn Leu
385                 390                 395                 400
Arg Cys Gln Ser Phe Lys Trp Tyr Leu Glu Asn Ile Tyr Pro Glu Leu
                405                 410                 415
Ser Ile Pro Lys Glu Ser Ser Ile Gln Lys Gly Asn Ile Arg Gln Arg
            420                 425                 430
Gln Lys Cys Leu Glu Ser Gln Arg Gln Asn Asn Gln Glu Thr Pro Asn
        435                 440                 445
Leu Lys Leu Ser Pro Cys Ala Lys Val Lys Gly Glu Asp Ala Lys Ser
    450                 455                 460
Gln Val Trp Ala Phe Thr Tyr Thr Gln Gln Ile Leu Gln Glu Glu Leu
465                 470                 475                 480
Cys Leu Ser Val Ile Thr Leu Phe Pro Gly Ala Pro Val Val Leu Val
                485                 490                 495
Leu Cys Lys Asn Gly Asp Asp Arg Gln Gln Trp Thr Lys Thr Gly Ser
            500                 505                 510

His Ile Glu His Ile Ala Ser His Leu Cys Leu Asp Thr Asp Met Phe
        515                 520                 525
Gly Asp Gly Thr Glu Asn Gly Lys Glu Ile Val Val Asn Pro Cys Glu
    530                 535                 540

Ser Ser Leu Met Ser Gln His Trp Asp Met Val Ser Ser
545                 550                 555

<210> 242
<211> 552
<212> PRT
<213> Homo sapiens

<400> 242
Met Arg Arg Leu Thr Arg Arg Leu Val Leu Pro Val Phe Gly Val Leu
1               5                   10                  15
Trp Ile Thr Val Leu Leu Phe Phe Trp Val Thr Lys Arg Lys Leu Glu
            20                  25                  30
Val Pro Thr Gly Pro Glu Val Gln Thr Pro Lys Pro Ser Asp Ala Asp
        35                  40                  45
Trp Asp Asp Leu Trp Asp Gln Phe Asp Glu Arg Arg Tyr Leu Asn Ala
    50                  55                  60
Lys Lys Trp Arg Val Gly Asp Asp Pro Tyr Lys Leu Tyr Ala Phe Asn
65                  70                  75                  80
Gln Arg Glu Ser Glu Arg Ile Ser Ser Asn Arg Ala Ile Pro Asp Thr
                85                  90                  95
Arg His Leu Arg Cys Thr Leu Leu Val Tyr Cys Thr Asp Leu Pro Pro
            100                 105                 110
Thr Ser Ile Ile Ile Thr Phe His Asn Glu Ala Arg Ser Thr Leu Leu
        115                 120                 125
Arg Thr Ile Arg Ser Val Leu Asn Arg Thr Pro Thr His Leu Ile Arg
    130                 135                 140
Glu Ile Ile Leu Val Asp Asp Phe Ser Asn Asp Pro Asp Asp Cys Lys
145                 150                 155                 160
Gln Leu Ile Lys Leu Pro Lys Val Lys Cys Leu Arg Asn Asn Glu Arg
                165                 170                 175
Gln Gly Leu Val Arg Ser Arg Ile Arg Gly Ala Asp Ile Ala Gln Gly
            180                 185                 190
Thr Thr Leu Thr Phe Leu Asp Ser His Cys Glu Val Asn Arg Asp Trp
        195                 200                 205
Leu Gln Pro Leu Leu His Arg Val Lys Glu Asp Tyr Thr Arg Val Val
    210                 215                 220
Cys Pro Val Ile Asp Ile Ile Asn Leu Asp Thr Phe Thr Tyr Ile Glu
225                 230                 235                 240
Ser Ala Ser Glu Leu Arg Gly Gly Phe Asp Trp Ser Leu His Phe Gln
                245                 250                 255
Trp Glu Gln Leu Ser Pro Glu Gln Lys Ala Arg Arg Leu Asp Pro Thr
            260                 265                 270
Glu Pro Ile Arg Thr Pro Ile Ile Ala Gly Gly Leu Phe Val Ile Asp
        275                 280                 285
Lys Ala Trp Phe Asp Tyr Leu Gly Lys Tyr Asp Met Asp Met Asp Ile
    290                 295                 300
Trp Gly Gly Glu Asn Phe Glu Ile Ser Phe Arg Val Trp Met Cys Gly
305                 310                 315                 320
Gly Ser Leu Glu Ile Val Pro Cys Ser Arg Val Gly His Val Phe Arg
                325                 330                 335
Lys Lys His Pro Tyr Val Phe Pro Asp Gly Asn Ala Asn Thr Tyr Ile
            340                 345                 350
Lys Asn Thr Lys Arg Thr Ala Glu Val Trp Met Asp Glu Tyr Lys Gln
        355                 360                 365
Tyr Tyr Tyr Ala Ala Arg Pro Phe Ala Leu Glu Arg Pro Phe Gly Asn
    370                 375                 380
Val Glu Ser Arg Leu Asp Leu Arg Lys Asn Leu Arg Cys Gln Ser Phe

385                 390                 395                 400
Lys Trp Tyr Leu Glu Asn Ile Tyr Pro Glu Leu Ser Ile Pro Lys Glu
                405                 410                 415
Ser Ser Ile Gln Lys Gly Asn Ile Arg Gln Arg Gln Lys Cys Leu Glu
            420                 425                 430
Ser Gln Arg Gln Asn Asn Gln Glu Thr Pro Asn Leu Lys Leu Ser Pro
        435                 440                 445
Cys Ala Lys Val Lys Gly Glu Asp Ala Lys Ser Gln Val Trp Ala Phe
    450                 455                 460
Thr Tyr Thr Gln Gln Ile Leu Gln Glu Glu Leu Cys Leu Ser Val Ile
465                 470                 475                 480
Thr Leu Phe Pro Gly Ala Pro Val Val Leu Val Leu Cys Lys Asn Gly
                485                 490                 495
Asp Asp Arg Gln Gln Trp Thr Lys Thr Gly Ser His Ile Glu His Ile
            500                 505                 510
Ala Ser His Leu Cys Leu Asp Thr Asp Met Phe Gly Asp Gly Thr Glu
        515                 520                 525
Asn Gly Lys Glu Ile Val Val Asn Pro Cys Glu Ser Ser Leu Met Ser
    530                 535                 540
Gln His Trp Asp Met Val Ser Ser
545                 550

<210> 243
<211> 519
<212> PRT
<213> Homo sapiens

<400> 243
Met Arg Arg Leu Thr Arg Arg Leu Val Leu Pro Val Phe Gly Val Leu
1               5                   10                  15
Trp Ile Thr Val Leu Leu Phe Phe Trp Val Thr Lys Arg Lys Leu Glu
            20                  25                  30
Val Pro Thr Gly Pro Glu Val Gln Thr Pro Lys Pro Ser Asp Ala Asp
        35                  40                  45
Trp Asp Asp Leu Trp Asp Gln Phe Asp Glu Arg Arg Tyr Leu Asn Ala
    50                  55                  60
Lys Lys Trp Arg Val Gly Asp Asp Pro Tyr Lys Leu Tyr Ala Phe Asn
65                  70                  75                  80
Gln Arg Glu Ser Glu Arg Ile Ser Ser Asn Arg Ala Ile Pro Asp Thr
                85                  90                  95
Arg His Leu Ser Val Leu Asn Arg Thr Pro Thr His Leu Ile Arg Glu
            100                 105                 110
Ile Ile Leu Val Asp Asp Phe Ser Asn Asp Pro Asp Asp Cys Lys Gln
        115                 120                 125
Leu Ile Lys Leu Pro Lys Val Lys Cys Leu Arg Asn Asn Glu Arg Gln
    130                 135                 140
Gly Leu Val Arg Ser Arg Ile Arg Gly Ala Asp Ile Ala Gln Gly Thr
145                 150                 155                 160
Thr Leu Thr Phe Leu Asp Ser His Cys Glu Val Asn Arg Asp Trp Leu
                165                 170                 175
Gln Pro Leu Leu His Arg Val Lys Glu Asp Tyr Thr Arg Val Val Cys
            180                 185                 190
Pro Val Ile Asp Ile Ile Asn Leu Asp Thr Phe Thr Tyr Ile Glu Ser
        195                 200                 205
Ala Ser Glu Leu Arg Gly Gly Phe Asp Trp Ser Leu His Phe Gln Trp
    210                 215                 220
Glu Gln Leu Ser Pro Glu Gln Lys Ala Arg Arg Leu Asp Pro Thr Glu
225                 230                 235                 240
Pro Ile Arg Thr Pro Ile Ile Ala Gly Gly Leu Phe Val Ile Asp Lys
                245                 250                 255
Ala Trp Phe Asp Tyr Leu Gly Lys Tyr Asp Met Asp Met Asp Ile Trp
            260                 265                 270
Gly Gly Glu Asn Phe Glu Ile Ser Phe Arg Val Trp Met Cys Gly Gly

275                 280                 285
Ser Leu Glu Ile Val Pro Cys Ser Arg Val Gly His Val Phe Arg Lys
    290                 295                 300
Lys His Pro Tyr Val Phe Pro Asp Gly Asn Ala Asn Thr Tyr Ile Lys
305                 310                 315                 320
Asn Thr Lys Arg Thr Ala Glu Val Trp Met Asp Glu Tyr Lys Arg Tyr
                325                 330                 335
Tyr Tyr Ala Ala Arg Pro Phe Ala Leu Glu Arg Pro Phe Gly Asn Val
            340                 345                 350
Glu Ser Arg Leu Asp Leu Arg Lys Asn Leu Arg Cys Gln Ser Phe Lys
        355                 360                 365
Trp Tyr Leu Glu Asn Ile Tyr Pro Glu Leu Ser Ile Pro Lys Glu Ser
    370                 375                 380
Ser Ile Gln Lys Gly Asn Ile Arg Gln Arg Gln Lys Cys Leu Glu Ser
385                 390                 395                 400
Gln Arg Gln Asn Asn Gln Glu Thr Pro Asn Leu Lys Leu Ser Pro Cys
                405                 410                 415
Ala Lys Val Lys Gly Glu Asp Ala Lys Ser Gln Val Trp Ala Phe Thr
            420                 425                 430
Tyr Thr Gln Gln Ile Leu Gln Glu Glu Leu Cys Leu Ser Val Ile Thr
        435                 440                 445
Leu Phe Pro Gly Ala Pro Val Val Leu Val Leu Cys Lys Asn Gly Asp
    450                 455                 460
Asp Arg Gln Gln Trp Thr Lys Thr Gly Ser His Ile Glu His Ile Ala
465                 470                 475                 480
Ser His Leu Cys Leu Asp Thr Asp Met Phe Gly Asp Gly Thr Glu Asn
                485                 490                 495
Gly Lys Glu Ile Val Val Asn Pro Cys Glu Ser Ser Leu Met Ser Gln
            500                 505                 510
His Trp Asp Met Val Ser Ser
        515

<210> 244
<211> 378
<212> PRT
<213> Homo sapiens

<400> 244
Met Asn Gly Lys Val Trp Ser Gly Pro Gly Phe Gly Ala Leu Thr Ser
1               5                   10                  15
Pro Arg Ala His Ser Asp Phe Leu Asp Ser His Cys Glu Val Asn Arg
            20                  25                  30
Asp Trp Leu Gln Pro Leu Leu His Arg Val Lys Glu Asp Tyr Thr Arg
        35                  40                  45
Val Val Cys Pro Val Ile Asp Ile Ile Asn Leu Asp Thr Phe Thr Tyr
    50                  55                  60
Ile Glu Ser Ala Ser Glu Leu Arg Gly Gly Phe Asp Trp Ser Leu His
65                  70                  75                  80
Phe Gln Trp Glu Gln Leu Ser Pro Glu Gln Lys Ala Arg Arg Leu Asp
                85                  90                  95
Pro Thr Glu Pro Ile Arg Thr Pro Ile Ile Ala Gly Gly Leu Phe Val
            100                 105                 110
Ile Asp Lys Ala Trp Phe Asp Tyr Leu Gly Lys Tyr Asp Met Asp Met
        115                 120                 125
Asp Ile Trp Gly Gly Glu Asn Phe Glu Ile Ser Phe Arg Val Trp Met
    130                 135                 140
Cys Gly Gly Ser Leu Glu Ile Val Pro Cys Ser Arg Val Gly His Val
145                 150                 155                 160
Phe Arg Lys Lys His Pro Tyr Val Phe Pro Asp Gly Asn Ala Asn Thr
                165                 170                 175
Tyr Ile Lys Asn Thr Lys Arg Thr Ala Glu Val Trp Met Asp Glu Tyr
            180                 185                 190
Lys Gln Tyr Tyr Tyr Ala Ala Arg Pro Phe Ala Leu Glu Arg Pro Phe

195                 200                 205
Gly Asn Val Glu Ser Arg Leu Asp Leu Arg Lys Asn Leu Arg Cys Gln
    210                 215                 220
Ser Phe Lys Trp Tyr Leu Glu Asn Ile Tyr Pro Glu Leu Ser Ile Pro
225                 230                 235                 240
Lys Glu Ser Ser Ile Gln Lys Gly Asn Ile Arg Gln Arg Gln Lys Cys
                245                 250                 255
Leu Glu Ser Gln Arg Gln Asn Asn Gln Glu Thr Pro Asn Leu Lys Leu
            260                 265                 270
Ser Pro Cys Ala Lys Val Lys Gly Glu Asp Ala Lys Ser Gln Val Trp
        275                 280                 285
Ala Phe Thr Tyr Thr Gln Gln Ile Leu Gln Glu Glu Leu Cys Leu Ser
    290                 295                 300
Val Ile Thr Leu Phe Pro Gly Ala Pro Val Val Leu Val Leu Cys Lys
305                 310                 315                 320
Asn Gly Asp Asp Arg Gln Gln Trp Thr Lys Thr Gly Ser His Ile Glu
                325                 330                 335
His Ile Ala Ser His Leu Cys Leu Asp Thr Asp Met Phe Gly Asp Gly
            340                 345                 350
Thr Glu Asn Gly Lys Glu Ile Val Val Asn Pro Cys Glu Ser Ser Leu
        355                 360                 365
Met Ser Gln His Trp Asp Met Val Ser Ser
    370                 375

<210> 245
<211> 354
<212> PRT
<213> Homo sapiens

<400> 245

<210> 246
<211> 343
<212> PRT
<213> Homo sapiens

<400> 246
Met Pro Arg Arg Ser Leu His Ala Ala Ala Val Leu Leu Leu Val Ile
 1               5                  10                  15
Leu Lys Glu Gln Pro Ser Ser Pro Ala Pro Val Asn Gly Ser Lys Trp
            20                  25                  30
Thr Tyr Phe Gly Pro Asp Gly Glu Asn Ser Trp Ser Lys Lys Tyr Pro
        35                  40                  45
Ser Cys Gly Gly Leu Leu Gln Ser Pro Ile Asp Leu His Ser Asp Ile
    50                  55                  60
Leu Gln Tyr Asp Ala Ser Leu Thr Pro Leu Glu Phe Gln Gly Tyr Asn
65                  70                  75                  80
Leu Ser Ala Asn Lys Gln Phe Leu Leu Thr Asn Asn Gly His Ser Val
                85                  90                  95
Lys Leu Asn Leu Pro Ser Asp Met His Ile Gln Gly Leu Gln Ser Arg
            100                 105                 110
Tyr Ser Ala Thr Gln Leu His Leu His Trp Gly Asn Pro Asn Asp Pro
        115                 120                 125
His Gly Ser Glu His Thr Val Ser Gly Gln His Phe Ala Ala Glu Leu
    130                 135                 140
His Ile Val His Tyr Asn Ser Asp Leu Tyr Pro Asp Ala Ser Thr Ala
145                 150                 155                 160
Ser Asn Lys Ser Glu Gly Leu Ala Val Leu Ala Val Leu Ile Glu Met
                165                 170                 175
Gly Ser Phe Asn Pro Ser Tyr Asp Lys Ile Phe Ser His Leu Gln His
            180                 185                 190
Val Lys Tyr Lys Gly Gln Glu Ala Phe Val Pro Gly Phe Asn Ile Glu
        195                 200                 205
Glu Leu Leu Pro Glu Arg Thr Ala Glu Tyr Tyr Arg Tyr Arg Gly Ser
    210                 215                 220
Leu Thr Thr Pro Pro Cys Asn Pro Thr Val Leu Trp Thr Val Phe Arg
225                 230                 235                 240
Asn Pro Val Gln Ile Ser Gln Glu Gln Leu Leu Ala Leu Glu Thr Ala
                245                 250                 255
Leu Tyr Cys Thr His Met Asp Asp Pro Ser Pro Arg Glu Met Ile Asn
            260                 265                 270
Asn Phe Arg Gln Val Gln Lys Phe Asp Glu Arg Leu Val Tyr Thr Ser
        275                 280                 285
Phe Ser Gln Gly Ile Ile Leu Ser Leu Ala Leu Ala Gly Ile Leu Gly
    290                 295                 300

Ile Cys Ile Val Val Val Val Ser Ile Trp Leu Phe Arg Arg Lys Ser
305                 310                 315                 320
Ile Lys Lys Gly Asp Asn Lys Gly Val Ile Tyr Lys Pro Ala Thr Lys
                325                 330                 335
Met Glu Thr Glu Ala His Ala

            340

<210> 247
<211> 401
<212> PRT
<213> Homo sapiens

<400> 247
Met Gln Val Asp Glu Thr Leu Ile Pro Arg Lys Gly Pro Ser Leu Cys
1               5                   10                  15
Ser Ala Arg Tyr Gly Ile Ala Leu Val Leu His Phe Cys Asn Phe Thr
            20                  25                  30
Thr Ile Ala Gln Asn Val Ile Met Asn Ile Thr Met Val Ala Met Val
        35                  40                  45
Asn Ser Thr Ser Pro Gln Ser Gln Leu Asn Asp Ser Ser Glu Val Leu
    50                  55                  60
Pro Val Asp Ser Phe Gly Gly Leu Ser Lys Ala Pro Lys Ser Leu Pro
65                  70                  75                  80
Ala Lys Ser Ser Ile Leu Gly Gly Gln Phe Ala Ile Trp Glu Lys Trp
                85                  90                  95
Gly Pro Pro Gln Glu Arg Ser Arg Leu Cys Ser Ile Ala Leu Ser Gly
            100                 105                 110
Met Leu Leu Gly Cys Phe Thr Ala Ile Leu Ile Gly Gly Phe Ile Ser
        115                 120                 125
Glu Thr Leu Gly Trp Pro Phe Val Phe Tyr Ile Phe Gly Gly Val Gly
    130                 135                 140
Cys Val Cys Cys Leu Leu Trp Phe Val Val Ile Tyr Asp Asp Pro Phe
145                 150                 155                 160
Ser Tyr Pro Trp Ile Ser Thr Ser Glu Lys Glu Tyr Ile Ile Ser Ser
                165                 170                 175
Leu Lys Gln Gln Val Gly Ser Ser Lys Gln Pro Leu Pro Ile Lys Ala
            180                 185                 190
Met Leu Arg Ser Leu Pro Ile Trp Ser Ile Cys Leu Gly Cys Phe Ser
        195                 200                 205
His Gln Trp Leu Val Ser Thr Met Val Val Tyr Ile Pro Thr Tyr Ile
    210                 215                 220
Ser Ser Val Tyr His Val Asn Ile Arg Asp Asn Gly Leu Leu Ser Ala
225                 230                 235                 240
Leu Pro Phe Ile Val Ala Trp Val Ile Gly Met Val Gly Gly Tyr Leu
                245                 250                 255
Ala Asp Phe Leu Leu Thr Lys Lys Phe Arg Leu Ile Thr Val Arg Lys
            260                 265                 270
Ile Ala Thr Ile Leu Gly Ser Leu Pro Ser Ser Ala Leu Ile Val Ser
        275                 280                 285
Leu Pro Tyr Leu Asn Ser Gly Tyr Ile Thr Ala Thr Ala Leu Leu Thr
    290                 295                 300
Leu Ser Cys Gly Leu Ser Thr Leu Cys Gln Ser Gly Ile Tyr Ile Asn
305                 310                 315                 320
Val Leu Asp Ile Ala Pro Arg Tyr Ser Ser Phe Leu Met Gly Ala Ser
                325                 330                 335
Arg Gly Phe Ser Ser Ile Ala Pro Val Ile Val Pro Thr Val Ser Gly
            340                 345                 350
Phe Leu Leu Ser Gln Asp Pro Glu Phe Gly Trp Arg Asn Val Phe Phe
        355                 360                 365
Leu Leu Phe Ala Val Asn Leu Leu Gly Leu Leu Phe Tyr Leu Ile Phe
    370                 375                 380
Gly Glu Ala Asp Val Gln Glu Trp Ala Lys Glu Arg Lys Leu Thr Arg
385                 390                 395                 400
Leu

<210> 248
<211> 555
<212> PRT

<213> Homo sapiens

<400> 248
Met Pro Thr Thr Val Asp Asp Val Leu Glu His Gly Gly Glu Phe His
1               5                   10                  15
Phe Phe Gln Lys Gln Met Phe Phe Leu Leu Ala Leu Leu Ser Ala Thr
            20                  25                  30
Phe Ala Pro Ile Tyr Val Gly Ile Val Phe Leu Gly Phe Thr Pro Asp
        35                  40                  45
His Arg Cys Arg Ser Pro Gly Val Ala Glu Leu Ser Leu Arg Cys Gly
    50                  55                  60
Trp Ser Pro Ala Glu Glu Leu Asn Tyr Thr Val Pro Gly Pro Gly Pro
65                  70                  75                  80
Ala Gly Glu Ala Ser Pro Arg Gln Cys Arg Arg Tyr Glu Val Asp Trp
                85                  90                  95
Asn Gln Ser Thr Phe Asp Cys Val Asp Pro Leu Ala Ser Leu Asp Thr
            100                 105                 110
Asn Arg Ser Arg Leu Pro Leu Gly Pro Cys Arg Asp Gly Trp Val Tyr
        115                 120                 125
Glu Thr Pro Gly Ser Ser Ile Val Thr Glu Phe Asn Leu Val Cys Ala
    130                 135                 140
Asn Ser Trp Met Leu Asp Leu Phe Gln Ser Ser Val Asn Val Gly Phe
145                 150                 155                 160
Phe Ile Gly Ser Met Ser Ile Gly Tyr Ile Ala Asp Arg Phe Gly Arg
                165                 170                 175
Lys Leu Cys Leu Leu Thr Thr Val Leu Ile Asn Ala Ala Ala Gly Val
            180                 185                 190
Leu Met Ala Ile Ser Pro Thr Tyr Thr Trp Met Leu Ile Phe Arg Leu
        195                 200                 205
Ile Gln Gly Leu Val Ser Lys Ala Gly Trp Leu Ile Gly Tyr Ile Leu
    210                 215                 220
Ile Thr Glu Phe Val Gly Arg Arg Tyr Arg Arg Thr Val Gly Ile Phe
225                 230                 235                 240
Tyr Gln Val Ala Tyr Thr Val Gly Leu Leu Val Leu Ala Gly Val Ala
                245                 250                 255
Tyr Ala Leu Pro His Trp Arg Trp Leu Gln Phe Thr Val Ala Leu Pro
            260                 265                 270
Asn Phe Phe Phe Leu Leu Tyr Tyr Trp Cys Ile Pro Glu Ser Pro Arg
        275                 280                 285
Trp Leu Ile Ser Gln Asn Lys Asn Ala Glu Ala Met Arg Ile Ile Lys
    290                 295                 300
His Ile Ala Lys Lys Asn Gly Lys Ser Leu Pro Ala Ser Leu Gln Arg
305                 310                 315                 320
Leu Arg Leu Glu Glu Glu Thr Gly Lys Lys Leu Asn Pro Ser Phe Leu
                325                 330                 335
Asp Leu Val Arg Thr Pro Gln Ile Arg Lys His Thr Met Ile Leu Met
            340                 345                 350
Tyr Asn Trp Phe Thr Ser Ser Val Leu Tyr Gln Gly Leu Ile Met His
        355                 360                 365
Met Gly Leu Ala Gly Asp Asn Ile Tyr Leu Asp Phe Phe Tyr Ser Ala
    370                 375                 380
Leu Val Glu Phe Pro Ala Ala Phe Met Ile Ile Leu Thr Ile Asp Arg
385                 390                 395                 400

Ile Gly Arg Arg Tyr Pro Trp Ala Ala Ser Asn Met Val Ala Gly Ala
                405                 410                 415
Ala Cys Leu Ala Ser Val Phe Ile Pro Gly Asp Leu Gln Trp Leu Lys
            420                 425                 430
Ile Ile Ile Ser Cys Leu Gly Arg Met Gly Ile Thr Met Ala Tyr Glu
        435                 440                 445
Ile Val Cys Leu Val Asn Ala Glu Leu Tyr Pro Thr Phe Ile Arg Asn
    450                 455                 460
Leu Gly Val His Ile Cys Ser Ser Met Cys Asp Ile Gly Gly Ile Ile

465                 470                 475                 480
Thr Pro Phe Leu Val Tyr Arg Leu Thr Asn Ile Trp Leu Glu Leu Pro
                485                 490                 495
Leu Met Val Phe Gly Val Leu Gly Leu Val Ala Gly Gly Leu Val Leu
            500                 505                 510
Leu Leu Pro Glu Thr Lys Gly Lys Ala Leu Pro Glu Thr Ile Glu Glu
        515                 520                 525
Ala Glu Asn Met Gln Arg Pro Arg Lys Asn Lys Glu Lys Met Ile Tyr
    530                 535                 540
Leu Gln Val Gln Lys Leu Asp Ile Pro Leu Asn
545                 550                 555

<210> 249
<211> 694
<212> PRT
<213> Homo sapiens

<400> 249
Met Gln Pro Ala Ile Gln Val Trp Phe Gly Glu Asp Leu Pro Leu Ser
1               5                   10                  15
Pro Arg Ser Pro Leu Thr Pro Arg His Gly Pro Gly Leu Ala Asn Val
            20                  25                  30
Cys Gln Tyr Asp Glu Trp Ile Ala Val Arg His Glu Ala Thr Leu Leu
        35                  40                  45
Pro Met Gln Glu Asp Leu Ser Ile Trp Leu Ser Gly Leu Leu Gly Ile
    50                  55                  60
Lys Val Lys Ala Glu Lys Leu Leu Glu Glu Leu Asp Asn Gly Val Leu
65                  70                  75                  80
Leu Cys Gln Leu Ile Asp Val Leu Gln Asn Met Val Lys Thr Cys Asn
                85                  90                  95
Ser Glu Glu Ser Gly Asn Phe Pro Met Arg Lys Val Pro Cys Lys Lys
            100                 105                 110
Asp Ala Ala Ser Gly Ser Phe Phe Ala Arg Asp Asn Thr Ala Asn Phe
        115                 120                 125
Leu His Trp Cys Arg Asp Ile Gly Val Asp Glu Thr Tyr Leu Phe Glu
    130                 135                 140
Ser Glu Gly Leu Val Leu His Lys Asp Pro Arg Gln Val Tyr Leu Cys
145                 150                 155                 160
Leu Leu Glu Ile Gly Arg Ile Val Ser Arg Tyr Gly Val Glu Pro Pro
                165                 170                 175
Val Leu Val Lys Leu Glu Lys Glu Ile Glu Leu Glu Glu Thr Leu Leu
            180                 185                 190
Asn Thr Ser Gly Pro Glu Asp Ser Ile Ser Ile Pro Lys Ser Cys Cys
        195                 200                 205
Arg His Glu Glu Leu His Glu Ala Val Lys His Ile Ala Glu Asp Pro
    210                 215                 220
Pro Cys Ser Cys Ser His Arg Phe Ser Ile Glu Tyr Leu Ser Glu Gly
225                 230                 235                 240
Arg Tyr Arg Leu Gly Asp Lys Ile Leu Phe Ile Arg Met Leu His Gly
                245                 250                 255
Lys His Val Met Val Arg Val Gly Gly Gly Trp Asp Thr Leu Gln Gly
            260                 265                 270
Phe Leu Leu Lys Tyr Asp Pro Cys Arg Ile Leu Gln Phe Ala Thr Leu
        275                 280                 285
Glu Gln Lys Ile Leu Ala Phe Gln Lys Gly Val Ser Asn Glu Ser Val
    290                 295                 300
Pro Asp Ser Pro Ala Arg Thr Pro Gln Pro Pro Glu Met Asn Pro Leu
305                 310                 315                 320
Ser Ala Val Asn Met Phe Gln Lys Gln Asn Ser Lys Pro Ser Val Pro
                325                 330                 335
Val Ser Ile Pro Lys Ser Lys Glu Lys Gln Gly Arg Pro Pro Gly Ala
            340                 345                 350
Leu Val Pro Ala Ser Ser Leu Lys Gly Gly Asn Leu Gly Ser Met Ser

355                 360                 365
Val Arg Ser Lys Leu Pro Asn Ser Pro Ala Ala Ser Ser His Pro Lys
    370                 375                 380
Leu Lys Ser Ser Lys Gly Ile Thr Lys Lys Pro Gln Ala Pro Ser Asn
385                 390                 395                 400
Asn Ala Ser Ser Ser Leu Ala Ser Leu Asn Pro Val Gly Lys Asn Thr
                405                 410                 415
Ser Ser Pro Ala Leu Pro Arg Thr Ala Pro Cys Ile Ser Glu Ser Pro
            420                 425                 430
Arg Lys Cys Ile Ser Ser Pro Asn Thr Pro Lys Ala Lys Val Ile Pro
        435                 440                 445
Ala Gln Asn Ser Ala Asp Leu Pro Glu Ser Thr Leu Leu Pro Asn Lys
    450                 455                 460
Cys Ser Gly Lys Thr Gln Pro Lys Tyr Leu Lys His Asn His Ile Ser
465                 470                 475                 480
Ser Arg Asp Asn Ala Val Ser His Leu Ala Ala His Ser Asn Ser Ser
                485                 490                 495
Ser Lys Cys Pro Lys Leu Pro Lys Ala Asn Ile Pro Val Arg Pro Lys
            500                 505                 510
Pro Ser Phe Gln Ser Ser Ala Lys Met Thr Lys Thr Ser Ser Lys Thr
        515                 520                 525
Ile Ala Thr Gly Leu Gly Thr Gln Ser Gln Pro Ser Asp Gly Ala Pro
    530                 535                 540
Gln Ala Lys Pro Val Pro Ala Gln Lys Leu Lys Ser Ala Leu Asn Leu
545                 550                 555                 560
Asn Gln Pro Val Ser Val Ser Ser Val Ser Pro Val Lys Ala Thr Gln
                565                 570                 575
Lys Ser Lys Asp Lys Asn Ile Val Ser Ala Thr Lys Lys Gln Pro Gln
            580                 585                 590
Asn Lys Ser Ala Phe Gln Lys Thr Gly Pro Ser Ser Leu Lys Ser Pro
        595                 600                 605
Gly Arg Thr Pro Leu Ser Ile Val Ser Leu Pro Gln Ser Ser Thr Lys
    610                 615                 620
Thr Gln Thr Ala Pro Lys Ser Ala Gln Thr Val Ala Lys Ser Gln His
625                 630                 635                 640
Ser Thr Lys Gly Pro Pro Arg Ser Gly Lys Thr Pro Ala Ser Ile Arg
                645                 650                 655
Lys Pro Pro Ser Ser Val Lys Asp Ala Asp Ser Gly Asp Lys Lys Pro
            660                 665                 670
Thr Ala Lys Lys Lys Glu Asp Asp Asp His Tyr Phe Val Met Thr Gly
        675                 680                 685
Ser Lys Lys Pro Arg Lys
    690

<210> 250
<211> 620
<212> PRT
<213> Homo sapiens

<400> 250

<210> 251
<211> 239
<212> PRT
<213> Homo sapiens

<400> 251
Met Pro Leu Gly His Ile Met Arg Leu Asp Leu Glu Lys Ile Ala Leu
1               5                   10                  15
Glu Tyr Ile Val Pro Cys Leu His Glu Val Gly Phe Cys Tyr Leu Asp
            20                  25                  30
Asn Phe Leu Gly Glu Val Val Gly Asp Cys Val Leu Glu Arg Val Lys
        35                  40                  45
Gln Leu His Cys Thr Gly Ala Leu Arg Asp Gly Gln Leu Ala Gly Pro
    50                  55                  60
Arg Ala Gly Val Ser Lys Arg His Leu Arg Gly Asp Gln Ile Thr Trp
65                  70                  75                  80
Ile Gly Gly Asn Glu Glu Gly Cys Glu Ala Ile Ser Phe Leu Leu Ser
                85                  90                  95
Leu Ile Asp Arg Leu Val Leu Tyr Cys Gly Ser Arg Leu Gly Lys Tyr
            100                 105                 110
Tyr Val Lys Glu Arg Ser Lys Ala Met Val Ala Cys Tyr Pro Gly Asn
        115                 120                 125
Gly Thr Gly Tyr Val Arg His Val Asp Asn Pro Asn Gly Asp Gly Arg
    130                 135                 140
Cys Ile Thr Cys Ile Tyr Tyr Leu Asn Lys Asn Trp Asp Ala Lys Leu
145                 150                 155                 160
His Gly Gly Ile Leu Arg Ile Phe Pro Glu Gly Lys Ser Phe Ile Ala
                165                 170                 175
Asp Val Glu Pro Ile Phe Asp Arg Leu Leu Phe Phe Trp Ser Asp Arg
            180                 185                 190
Arg Asn Pro His Glu Val Gln Pro Ser Tyr Ala Thr Arg Tyr Ala Met
        195                 200                 205
Thr Val Trp Tyr Phe Asp Ala Glu Glu Arg Ala Glu Ala Lys Lys Lys
    210                 215                 220
Phe Arg Asn Leu Thr Arg Lys Thr Glu Ser Ala Leu Thr Glu Asp
225                 230                 235

<210> 252
<211> 406
<212> PRT
<213> Homo sapiens

<400> 252
Met Ser Gly Ala Pro Thr Ala Gly Ala Ala Leu Met Leu Cys Ala Ala
1               5                   10                  15
Thr Ala Val Leu Leu Ser Ala Gln Gly Gly Pro Val Gln Ser Lys Ser
            20                  25                  30
Pro Arg Phe Ala Ser Trp Asp Glu Met Asn Val Leu Ala His Gly Leu
        35                  40                  45
Leu Gln Leu Gly Gln Gly Leu Arg Glu His Ala Glu Arg Thr Arg Ser
    50                  55                  60
Gln Leu Ser Ala Leu Glu Arg Arg Leu Ser Ala Cys Gly Ser Ala Cys
65                  70                  75                  80
Gln Gly Thr Glu Gly Ser Thr Asp Leu Pro Leu Ala Pro Glu Ser Arg
                85                  90                  95
Val Asp Pro Glu Val Leu His Ser Leu Gln Thr Gln Leu Lys Ala Gln
            100                 105                 110
Asn Ser Arg Ile Gln Gln Leu Phe His Lys Val Ala Gln Gln Gln Arg
        115                 120                 125

His Leu Glu Lys Gln His Leu Arg Ile Gln His Leu Gln Ser Gln Phe
    130                 135                 140
Gly Leu Leu Asp His Lys His Leu Asp His Glu Val Ala Lys Pro Ala
145                 150                 155                 160
Arg Arg Lys Arg Leu Pro Glu Met Ala Gln Pro Val Asp Pro Ala His
                165                 170                 175
Asn Val Ser Arg Leu His Arg Leu Pro Arg Asp Cys Gln Glu Leu Phe
            180                 185                 190
Gln Val Gly Glu Arg Gln Ser Gly Leu Phe Glu Ile Gln Pro Gln Gly
        195                 200                 205
Ser Pro Pro Phe Leu Val Asn Cys Lys Met Thr Ser Asp Gly Gly Trp
    210                 215                 220
Thr Val Ile Gln Arg Arg His Asp Gly Ser Val Asp Phe Asn Arg Pro
225                 230                 235                 240
Trp Glu Ala Tyr Lys Ala Gly Phe Gly Asp Pro His Gly Glu Phe Trp
                245                 250                 255
Leu Gly Leu Glu Lys Val His Ser Ile Thr Gly Asp Arg Asn Ser Arg
            260                 265                 270
Leu Ala Val Gln Leu Arg Asp Trp Asp Gly Asn Ala Glu Leu Leu Gln
        275                 280                 285
Phe Ser Val His Leu Gly Gly Glu Asp Thr Ala Tyr Ser Leu Gln Leu
    290                 295                 300
Thr Ala Pro Val Ala Gly Gln Leu Gly Ala Thr Thr Val Pro Pro Ser
305                 310                 315                 320
Gly Leu Ser Val Pro Phe Ser Thr Trp Asp Gln Asp His Asp Leu Arg
                325                 330                 335
Arg Asp Lys Asn Cys Ala Lys Ser Leu Ser Gly Gly Trp Trp Phe Gly
            340                 345                 350
Thr Cys Ser His Ser Asn Leu Asn Gly Gln Tyr Phe Arg Ser Ile Pro
        355                 360                 365
Gln Gln Arg Gln Lys Leu Lys Lys Gly Ile Phe Trp Lys Thr Trp Arg
    370                 375                 380
Gly Arg Tyr Tyr Pro Leu Gln Ala Thr Thr Met Leu Ile Gln Pro Met
385                 390                 395                 400
Ala Ala Glu Ala Ala Ser
                405

<210> 253
<211> 459
<212> PRT
<213> Homo sapiens

<400> 253
Met Ala Pro Leu Cys Pro Ser Pro Trp Leu Pro Leu Leu Ile Pro Ala
1               5                   10                  15
Pro Ala Pro Gly Leu Thr Val Gln Leu Leu Leu Ser Leu Leu Leu Leu
            20                  25                  30
Met Pro Val His Pro Gln Arg Leu Pro Arg Met Gln Glu Asp Ser Pro
        35                  40                  45
Leu Gly Gly Gly Ser Ser Gly Glu Asp Asp Pro Leu Gly Glu Glu Asp
    50                  55                  60
Leu Pro Ser Glu Glu Asp Ser Pro Arg Glu Glu Asp Pro Pro Gly Glu
65                  70                  75                  80
Glu Asp Leu Pro Gly Glu Glu Asp Leu Pro Gly Glu Glu Asp Leu Pro
                85                  90                  95
Glu Val Lys Pro Lys Ser Glu Glu Glu Gly Ser Leu Lys Leu Glu Asp
            100                 105                 110
Leu Pro Thr Val Glu Ala Pro Gly Asp Pro Gln Glu Pro Gln Asn Asn
        115                 120                 125
Ala His Arg Asp Lys Glu Gly Asp Asp Gln Ser His Trp Arg Tyr Gly
    130                 135                 140
Gly Asp Pro Pro Trp Pro Arg Val Ser Pro Ala Cys Ala Gly Arg Phe

145                 150                 155                 160
Gln Ser Pro Val Asp Ile Arg Pro Gln Leu Ala Ala Phe Cys Pro Ala
                165                 170                 175
Leu Arg Pro Leu Glu Leu Leu Gly Phe Gln Leu Pro Pro Leu Pro Glu
            180                 185                 190
Leu Arg Leu Arg Asn Asn Gly His Ser Val Gln Leu Thr Leu Pro Pro
        195                 200                 205
Gly Leu Glu Met Ala Leu Gly Pro Gly Arg Glu Tyr Arg Ala Leu Gln
    210                 215                 220
Leu His Leu His Trp Gly Ala Ala Gly Arg Pro Gly Ser Glu His Thr
225                 230                 235                 240
Val Glu Gly His Arg Phe Pro Ala Glu Ile His Val Val His Leu Ser
                245                 250                 255
Thr Ala Phe Ala Arg Val Asp Glu Ala Leu Gly Arg Pro Gly Gly Leu
            260                 265                 270
Ala Val Leu Ala Ala Phe Leu Glu Glu Gly Pro Glu Glu Asn Ser Ala
        275                 280                 285
Tyr Glu Gln Leu Leu Ser Arg Leu Glu Glu Ile Ala Glu Glu Gly Ser
    290                 295                 300
Glu Thr Gln Val Pro Gly Leu Asp Ile Ser Ala Leu Leu Pro Ser Asp
305                 310                 315                 320
Phe Ser Arg Tyr Phe Gln Tyr Glu Gly Ser Leu Thr Thr Pro Pro Cys
                325                 330                 335
Ala Gln Gly Val Ile Trp Thr Val Phe Asn Gln Thr Val Met Leu Ser
            340                 345                 350
Ala Lys Gln Leu His Thr Leu Ser Asp Thr Leu Trp Gly Pro Gly Asp
        355                 360                 365
Ser Arg Leu Gln Leu Asn Phe Arg Ala Thr Gln Pro Leu Asn Gly Arg
    370                 375                 380
Val Ile Glu Ala Ser Phe Pro Ala Gly Val Asp Ser Ser Pro Arg Ala
385                 390                 395                 400
Ala Glu Pro Val Gln Leu Asn Ser Cys Leu Ala Ala Gly Asp Ile Leu
                405                 410                 415
Ala Leu Val Phe Gly Leu Leu Phe Ala Val Thr Ser Val Ala Phe Leu
            420                 425                 430
Val Gln Met Arg Arg Gln His Arg Arg Gly Thr Lys Gly Gly Val Ser
        435                 440                 445
Tyr Arg Pro Ala Glu Val Ala Glu Thr Gly Ala
    450                 455

<210> 254
<211> 501
<212> PRT
<213> Homo sapiens

<400> 254
   Met Glu Gln Gln Asp Gln Ser Met Lys Glu Gly Arg Leu Thr Leu Val
   1               5                   10                  15
   Leu Ala Leu Ala Thr Leu Ile Ala Ala Phe Gly Ser Ser Phe Gln Tyr
               20                  25                  30
   Gly Tyr Asn Val Ala Ala Val Asn Ser Pro Ala Leu Leu Met Gln Gln
           35                  40                  45
   Phe Tyr Asn Glu Thr Tyr Tyr Gly Arg Thr Gly Glu Phe Met Glu Asp
       50                  55                  60
   Phe Pro Leu Thr Leu Leu Trp Ser Val Thr Val Ser Met Phe Pro Phe
   65                  70                  75                  80
   Gly Gly Phe Ile Gly Ser Leu Leu Val Gly Pro Leu Val Asn Lys Phe
                   85                  90                  95
   Gly Arg Lys Gly Ala Leu Leu Phe Asn Asn Ile Phe Ser Ile Val Pro
               100                 105                 110
   Ala Ile Leu Met Gly Cys Ser Arg Val Ala Thr Ser Phe Glu Leu Ile
           115                 120                 125
   Ile Ile Ser Arg Leu Leu Val Gly Ile Cys Ala Gly Val Ser Ser Asn
       130                 135                 140
   Val Val Pro Met Tyr Leu Gly Glu Leu Ala Pro Lys Asn Leu Arg Gly
   145                 150                 155                 160
   Ala Leu Gly Val Val Pro Gln Leu Phe Ile Thr Val Gly Ile Leu Val
                   165                 170                 175
   Ala Gln Ile Phe Gly Leu Arg Asn Leu Leu Ala Asn Val Asp Gly Trp
               180                 185                 190
   Pro Ile Leu Leu Gly Leu Thr Gly Val Pro Ala Ala Leu Gln Leu Leu
           195                 200                 205
   Leu Leu Pro Phe Phe Pro Glu Ser Pro Arg Tyr Leu Leu Ile Gln Lys
       210                 215                 220
   Lys Asp Glu Ala Ala Ala Lys Lys Ala Leu Gln Thr Leu Arg Gly Trp
   225                 230                 235                 240
   Asp Ser Val Asp Arg Glu Val Ala Glu Ile Arg Gln Glu Asp Glu Ala
                   245                 250                 255
   Glu Lys Ala Ala Gly Phe Ile Ser Val Leu Lys Leu Phe Arg Met Arg
               260                 265                 270
   Ser Leu Arg Trp Gln Leu Leu Ser Ile Ile Val Leu Met Gly Gly Gln
           275                 280                 285
   Gln Leu Ser Gly Val Asn Ala Ile Tyr Tyr Tyr Ala Asp Gln Ile Tyr
       290                 295                 300
   Leu Ser Ala Gly Val Pro Glu Glu His Val Gln Tyr Val Thr Ala Gly
   305                 310                 315                 320
   Thr Gly Ala Val Asn Val Val Met Thr Phe Cys Ala Val Phe Val Val
                   325                 330                 335
   Glu Leu Leu Gly Arg Arg Leu Leu Leu Leu Leu Gly Phe Ser Ile Cys
               340                 345                 350
   Leu Ile Ala Cys Cys Val Leu Thr Ala Ala Leu Ala Leu Gln Asp Thr
           355                 360                 365
   Val Ser Trp Met Pro Tyr Ile Ser Ile Val Cys Val Ile Ser Tyr Val
       370                 375                 380
   Ile Gly His Ala Leu Gly Pro Ser Pro Ile Pro Ala Leu Leu Ile Thr
   385                 390                 395                 400
   Glu Ile Phe Leu Gln Ser Ser Arg Pro Ser Ala Phe Met Val Gly Gly
                   405                 410                 415
   Ser Val His Trp Leu Ser Asn Phe Thr Val Gly Leu Ile Phe Pro Phe
               420                 425                 430
   Ile Gln Glu Gly Leu Gly Pro Tyr Ser Phe Ile Val Phe Ala Val Ile
           435                 440                 445
   Cys Leu Leu Thr Thr Ile Tyr Ile Phe Leu Ile Val Pro Glu Thr Lys
       450                 455                 460
   Ala Lys Thr Phe Ile Glu Ile Asn Gln Ile Phe Thr Lys Met Asn Lys
   465                 470                 475                 480
   Val Ser Glu Val Tyr Pro Glu Lys Glu Glu Leu Lys Glu Leu Pro Pro
                   485                 490                 495
   Val Thr Ser Glu Gln
               500

<210> 255
<211> 193
<212> PRT
<213> Homo sapiens

<400> 255
Met Pro Glu Glu Gly Ser Gly Cys Ser Val Arg Arg Arg Pro Tyr Gly
1               5                   10                  15
Cys Val Leu Arg Ala Ala Leu Val Pro Leu Val Ala Gly Leu Val Ile
            20                  25                  30
Cys Leu Val Val Cys Ile Gln Arg Phe Ala Gln Ala Gln Gln Gln Leu
        35                  40                  45
Pro Leu Glu Ser Leu Gly Trp Asp Val Ala Glu Leu Gln Leu Asn His

    50                  55                  60
Thr Gly Pro Gln Gln Asp Pro Arg Leu Tyr Trp Gln Gly Gly Pro Ala
65                  70                  75                  80
Leu Gly Arg Ser Phe Leu His Gly Pro Glu Leu Asp Lys Gly Gln Leu
                85                  90                  95
Arg Ile His Arg Asp Gly Ile Tyr Met Val His Ile Gln Val Thr Leu
            100                 105                 110
Ala Ile Cys Ser Ser Thr Thr Ala Ser Arg His His Pro Thr Thr Leu
        115                 120                 125
Ala Val Gly Ile Cys Ser Pro Ala Ser Arg Ser Ile Ser Leu Leu Arg
    130                 135                 140
Leu Ser Phe His Gln Gly Cys Thr Ile Ala Ser Gln Arg Leu Thr Pro
145                 150                 155                 160
Leu Ala Arg Gly Asp Thr Leu Cys Thr Asn Leu Thr Gly Thr Leu Leu
                165                 170                 175
Pro Ser Arg Asn Thr Asp Glu Thr Phe Phe Gly Val Gln Trp Val Arg
            180                 185                 190
Pro

<210> 256
<211> 201
<212> PRT
<213> Homo sapiens

<400> 256
Met Leu Phe Thr Ser Phe Val Glu Gln Lys Lys Lys Ala Gly Val Phe
 1               5                  10                  15
Glu Gln Ile Thr Lys Thr His Gly Thr Ile Ile Gly Ile Thr Ser Gly
            20                  25                  30
Ile Val Leu Val Leu Leu Ile Ile Ser Ile Leu Val Gln Val Lys Gln
        35                  40                  45
Pro Arg Lys Lys Val Met Ala Cys Lys Thr Ala Phe Asn Lys Thr Gly
    50                  55                  60
Phe Gln Glu Val Phe Asp Pro Pro His Tyr Glu Leu Phe Ser Leu Arg
65                  70                  75                  80
Asp Lys Glu Ile Ser Ala Asp Leu Ala Asp Leu Ser Glu Glu Leu Asp
                85                  90                  95
Asn Tyr Gln Arg Met Arg Arg Ser Ser Thr Ala Ser Arg Cys Ile His
            100                 105                 110
Asp His His Cys Gly Ser Gln Ala Ser Ser Val Lys Gln Ser Arg Thr
        115                 120                 125
Asn Leu Ser Ser Met Glu Leu Pro Leu Arg Asn Asp Phe Ala Gln Pro
    130                 135                 140
Gln Pro Met Lys Thr Phe Asn Ser Thr Phe Lys Lys Ser Ser Tyr Thr
145                 150                 155                 160
Phe Lys Gln Gly His Glu Cys Pro Glu Gln Ala Leu Glu Asp Arg Val
                165                 170                 175
Met Glu Glu Ile Pro Cys Glu Ile Tyr Val Arg Gly Arg Glu Asp Ser
            180                 185                 190
Ala Gln Ala Ser Ile Ser Ile Asp Phe
        195                 200

<210> 257
<211> 152
<212> PRT
<213> Homo sapiens

<400> 257
Ile Ile Ala Ala Thr Ile Asn Asn Ala Ser Met Thr Ser Pro Ile Asp
 1               5                  10                  15
Asn Ala Gly Leu Ala Ala Asp Asp Phe Lys Met Asn Ala Ser Leu Gln
            20                  25                  30
Thr Gln Ile Ser Thr Asp Ala Glu Ala Val Ser Ser Ala Lys Ser Glu

35                  40                  45
Ile Met Glu Leu Lys Gln Val Leu Gln Ser Leu Gln His Glu Leu Gln
    50                  55                  60
Ser Val Leu Ala Met Gln Ser Ser Pro Glu Gly Thr Leu Ala Asp Thr
65                  70                  75                  80
Glu Ala Gly Tyr Val Ala Gln Leu Ser Glu Ile Lys Met Tyr Ile Ser
                85                  90                  95
Ile Leu Glu Glu Gln Ile Cys Gln Ile Arg Gly Glu Thr Glu Tyr Gln
            100                 105                 110
Asn Thr Glu Tyr Ala Gln Leu Gln Asp Ile Lys Thr Arg Leu Glu Val
        115                 120                 125
Glu Ile Glu Thr Tyr His Arg Leu Leu Gly Gly Glu Gly Gly Ser Glu
    130                 135                 140
Ala Arg Glu Ala Glu Ser Lys Gly
145                 150

<210> 258
<211> 904
<212> PRT
<213> Homo sapiens

<400> 258
Met Arg Gln Thr Leu Pro Cys Ile Tyr Phe Trp Gly Gly Leu Leu Pro
1               5                   10                  15
Phe Gly Met Leu Cys Ala Ser Ser Thr Thr Lys Cys Thr Val Ser His
            20                  25                  30
Glu Val Ala Asp Cys Ser His Leu Lys Leu Thr Gln Val Pro Asp Asp
        35                  40                  45
Leu Pro Thr Asn Ile Thr Val Leu Asn Leu Thr His Asn Gln Leu Arg
    50                  55                  60
Arg Leu Pro Ala Ala Asn Phe Thr Arg Tyr Ser Gln Leu Thr Ser Leu
65                  70                  75                  80
Asp Val Gly Phe Asn Thr Ile Ser Lys Leu Glu Pro Glu Leu Cys Gln
                85                  90                  95

Lys Leu Pro Met Leu Lys Val Leu Asn Leu Gln His Asn Glu Leu Ser
            100                 105                 110
Gln Leu Ser Asp Lys Thr Phe Ala Phe Cys Thr Asn Leu Thr Glu Leu
        115                 120                 125
His Leu Met Ser Asn Ser Ile Gln Lys Ile Lys Asn Asn Pro Phe Val
    130                 135                 140
Lys Gln Lys Asn Leu Ile Thr Leu Asp Leu Ser His Asn Gly Leu Ser
145                 150                 155                 160
Ser Thr Lys Leu Gly Thr Gln Val Gln Leu Glu Asn Leu Gln Glu Leu
                165                 170                 175
Leu Leu Ser Asn Asn Lys Ile Gln Ala Leu Lys Ser Glu Glu Leu Asp
            180                 185                 190
Ile Phe Ala Asn Ser Ser Leu Lys Lys Leu Glu Leu Ser Ser Asn Gln
        195                 200                 205
Ile Lys Glu Phe Ser Pro Gly Cys Phe His Ala Ile Gly Arg Leu Phe
    210                 215                 220
Gly Leu Phe Leu Asn Asn Val Gln Leu Gly Pro Ser Leu Thr Glu Lys
225                 230                 235                 240
Leu Cys Leu Glu Leu Ala Asn Thr Ser Ile Arg Asn Leu Ser Leu Ser
                245                 250                 255
Asn Ser Gln Leu Ser Thr Thr Ser Asn Thr Thr Phe Leu Gly Leu Lys
            260                 265                 270
Trp Thr Asn Leu Thr Met Leu Asp Leu Ser Tyr Asn Asn Leu Asn Val
        275                 280                 285
Val Gly Asn Asp Ser Phe Ala Trp Leu Pro Gln Leu Glu Tyr Phe Phe
    290                 295                 300
Leu Glu Tyr Asn Asn Ile Gln His Leu Phe Ser His Ser Leu His Gly
305                 310                 315                 320

Leu Phe Asn Val Arg Tyr Leu Asn Leu Lys Arg Ser Phe Thr Lys Gln
                325                 330                 335
Ser Ile Ser Leu Ala Ser Leu Pro Lys Ile Asp Asp Phe Ser Phe Gln
            340                 345                 350
Trp Leu Lys Cys Leu Glu His Leu Asn Met Glu Asp Asn Asp Ile Pro
        355                 360                 365
Gly Ile Lys Ser Asn Met Phe Thr Gly Leu Ile Asn Leu Lys Tyr Leu
    370                 375                 380
Ser Leu Ser Asn Ser Phe Thr Ser Leu Arg Thr Leu Thr Asn Glu Thr
385                 390                 395                 400
Phe Val Ser Leu Ala His Ser Pro Leu His Ile Leu Asn Leu Thr Lys
                405                 410                 415
Asn Lys Ile Ser Lys Ile Glu Ser Asp Ala Phe Ser Trp Leu Gly His
            420                 425                 430
Leu Glu Val Leu Asp Leu Gly Leu Asn Glu Ile Gly Gln Glu Leu Thr
        435                 440                 445
Gly Gln Glu Trp Arg Gly Leu Glu Asn Ile Phe Glu Ile Tyr Leu Ser
    450                 455                 460
Tyr Asn Lys Tyr Leu Gln Leu Thr Arg Asn Ser Phe Ala Leu Val Pro
465                 470                 475                 480
Ser Leu Gln Arg Leu Met Leu Arg Arg Val Ala Leu Lys Asn Val Asp
                485                 490                 495
Ser Ser Pro Ser Pro Phe Gln Pro Leu Arg Asn Leu Thr Ile Leu Asp
            500                 505                 510
Leu Ser Asn Asn Asn Ile Ala Asn Ile Asn Asp Asp Met Leu Glu Gly
        515                 520                 525
Leu Glu Lys Leu Glu Ile Leu Asp Leu Gln His Asn Asn Leu Ala Arg
    530                 535                 540
Leu Trp Lys His Ala Asn Pro Gly Gly Pro Ile Tyr Phe Leu Lys Gly
545                 550                 555                 560
Leu Ser His Leu His Ile Leu Asn Leu Glu Ser Asn Gly Phe Asp Glu
                565                 570                 575

Ile Pro Val Glu Val Phe Lys Asp Leu Phe Glu Leu Lys Ile Ile Asp
            580                 585                 590
Leu Gly Leu Asn Asn Leu Asn Thr Leu Pro Ala Ser Val Phe Asn Asn
        595                 600                 605
Gln Val Ser Leu Lys Ser Leu Asn Leu Gln Lys Asn Leu Ile Thr Ser
    610                 615                 620
Val Glu Lys Lys Val Phe Gly Pro Ala Phe Arg Asn Leu Thr Glu Leu
625                 630                 635                 640
Asp Met Arg Phe Asn Pro Phe Asp Cys Thr Cys Glu Ser Ile Ala Trp
                645                 650                 655
Phe Val Asn Trp Ile Asn Glu Thr His Thr Asn Ile Pro Glu Leu Ser
            660                 665                 670
Ser His Tyr Leu Cys Asn Thr Pro Pro His Tyr His Gly Phe Pro Val
        675                 680                 685
Arg Leu Phe Asp Thr Ser Ser Cys Lys Asp Ser Ala Pro Phe Glu Leu
    690                 695                 700
Phe Phe Met Ile Asn Thr Ser Ile Leu Leu Ile Phe Ile Phe Ile Val
705                 710                 715                 720
Leu Leu Ile His Phe Glu Gly Trp Arg Ile Ser Phe Tyr Trp Asn Val
                725                 730                 735
Ser Val His Arg Val Leu Gly Phe Lys Glu Ile Asp Arg Gln Thr Glu
            740                 745                 750
Gln Phe Glu Tyr Ala Ala Tyr Ile Ile His Ala Tyr Lys Asp Lys Asp
        755                 760                 765
Trp Val Trp Glu His Phe Ser Ser Met Glu Lys Glu Asp Gln Ser Leu
    770                 775                 780
Lys Phe Cys Leu Glu Glu Arg Asp Phe Glu Ala Gly Val Phe Glu Leu
785                 790                 795                 800
Glu Ala Ile Val Asn Ser Ile Lys Arg Ser Arg Lys Ile Ile Phe Val
                805                 810                 815

Ile Thr His His Leu Leu Lys Asp Pro Leu Cys Lys Arg Phe Lys Val
            820                 825                 830
His His Ala Val Gln Gln Ala Ile Glu Gln Asn Leu Asp Ser Ile Ile
        835                 840                 845
Leu Val Phe Leu Glu Glu Ile Pro Asp Tyr Lys Leu Asn His Ala Leu
    850                 855                 860
Cys Leu Arg Arg Gly Met Phe Lys Ser His Cys Ile Leu Asn Trp Pro
865                 870                 875                 880
Val Gln Lys Glu Arg Ile Gly Ala Phe Arg His Lys Leu Gln Val Ala
                885                 890                 895
Leu Gly Ser Lys Asn Ser Val His
            900

<210> 259
<211> 352
<212> PRT
<213> Homo sapiens

<400> 259

<210> 260
<211> 216
<212> PRT
<213> Homo sapiens

<400> 260
Pro Gln Gly Pro Pro Gly Pro Pro Gly Ile Pro Gly Ala Lys Gly Glu
1               5                   10                  15
Leu Gly Leu Pro Gly Ala Pro Gly Ile Asp Gly Glu Lys Gly Pro Lys
            20                  25                  30
Gly Gln Lys Gly Asp Pro Gly Glu Pro Gly Pro Ala Gly Leu Lys Gly
        35                  40                  45
Glu Ala Gly Glu Met Gly Leu Ser Gly Leu Pro Gly Ala Asp Gly Leu
    50                  55                  60
Lys Gly Glu Lys Gly Glu Ser Ala Ser Asp Ser Leu Gln Glu Ser Leu
65                  70                  75                  80
Ala Gln Leu Ile Val Glu Pro Gly Pro Pro Gly Pro Pro Gly Pro Pro
                85                  90                  95
Gly Pro Met Gly Leu Gln Gly Ile Gln Gly Pro Lys Gly Leu Asp Gly
            100                 105                 110
Ala Lys Gly Glu Lys Gly Ala Ser Gly Glu Arg Gly Pro Ser Gly Leu
        115                 120                 125
Pro Gly Pro Val Gly Pro Pro Gly Leu Ile Gly Leu Pro Gly Thr Lys
    130                 135                 140
Gly Glu Lys Gly Arg Pro Gly Glu Pro Gly Leu Asp Gly Phe Pro Gly
145                 150                 155                 160
Pro Arg Gly Glu Lys Gly Asp Arg Ser Glu Arg Gly Glu Lys Gly Glu
                165                 170                 175
Arg Gly Val Pro Gly Arg Lys Gly Val Lys Gly Gln Lys Gly Glu Pro
            180                 185                 190
Gly Pro Pro Gly Leu Asp Gln Pro Cys Pro Val Gly Pro Asp Gly Leu
        195                 200                 205
Pro Val Pro Gly Cys Trp His Lys
    210                 215

<210> 261
<211> 423
<212> PRT
<213> Homo sapiens

<400> 261
Met Leu Pro Pro Gln Lys Lys Pro Trp Glu Ser Met Ala Lys Gly Leu
1               5                   10                  15
Val Leu Gly Ala Leu Phe Thr Ser Phe Leu Leu Leu Val Tyr Ser Tyr
            20                  25                  30
Ala Val Pro Pro Leu His Ala Gly Leu Ala Ser Thr Thr Pro Glu Ala
        35                  40                  45
Ala Ala Ser Cys Ser Pro Pro Ala Leu Glu Pro Glu Ala Val Ile Arg
    50                  55                  60
Ala Asn Gly Ser Ala Gly Glu Cys Gln Pro Arg Arg Asn Ile Val Phe
65                  70                  75                  80
Leu Lys Thr His Lys Thr Ala Ser Ser Thr Leu Leu Asn Ile Leu Phe
                85                  90                  95
Arg Phe Gly Gln Lys His Arg Leu Lys Phe Ala Phe Pro Asn Gly Arg
            100                 105                 110
Asn Asp Phe Asp Tyr Pro Thr Phe Phe Ala Arg Ser Leu Val Gln Asp
        115                 120                 125
Tyr Arg Pro Gly Ala Cys Phe Asn Ile Ile Cys Asn His Met Arg Phe
    130                 135                 140
His Tyr Asp Glu Val Arg Gly Leu Val Pro Thr Asn Ala Ile Phe Ile
145                 150                 155                 160
Thr Val Leu Arg Asp Pro Ala Arg Leu Phe Glu Ser Ser Phe His Tyr

                165                 170                 175
Phe Gly Pro Val Val Pro Leu Thr Trp Lys Leu Ser Ala Gly Asp Lys
            180                 185                 190
Leu Thr Glu Phe Leu Gln Asp Pro Asp Arg Tyr Tyr Asp Pro Asn Gly
        195                 200                 205
Phe Asn Ala His Tyr Leu Arg Asn Leu Leu Phe Phe Asp Leu Gly Tyr
    210                 215                 220
Asp Asn Ser Leu Asp Pro Ser Ser Pro Gln Val Gln Glu His Ile Leu
225                 230                 235                 240
Glu Val Glu Arg Arg Phe His Leu Val Leu Leu Gln Glu Tyr Phe Asp
                245                 250                 255
Glu Ser Leu Val Leu Leu Lys Asp Leu Leu Cys Trp Glu Leu Glu Asp
            260                 265                 270
Val Leu Tyr Phe Lys Leu Asn Ala Arg Arg Asp Ser Pro Val Pro Arg
        275                 280                 285
Leu Ser Gly Glu Leu Tyr Gly Arg Ala Thr Ala Trp Asn Met Leu Asp
    290                 295                 300
Ser His Leu Tyr Arg His Phe Asn Ala Ser Phe Trp Arg Lys Val Glu
305                 310                 315                 320
Ala Phe Gly Arg Glu Arg Met Ala Arg Glu Val Ala Ala Leu Arg His
                325                 330                 335
Ala Asn Glu Arg Met Arg Thr Ile Cys Ile Asp Gly Gly His Ala Val
            340                 345                 350
Asp Ala Ala Ala Ile Gln Asp Glu Ala Met Gln Pro Trp Gln Pro Leu
        355                 360                 365
Gly Thr Lys Ser Ile Leu Gly Tyr Asn Leu Lys Lys Ser Ile Gly Gln
    370                 375                 380
Arg His Ala Gln Leu Cys Arg Arg Met Leu Thr Pro Glu Ile Gln Tyr
385                 390                 395                 400
Leu Met Asp Leu Gly Ala Asn Leu Trp Val Thr Lys Leu Trp Lys Phe
                405                 410                 415
Ile Arg Asp Phe Leu Arg Trp
            420

<210> 262
<211> 513
<212> PRT
<213> Homo sapiens

<400> 262
Met Ala Ser Val Leu Ser Arg Arg Leu Gly Lys Arg Ser Leu Leu Gly
1               5                   10                  15
Ala Arg Val Leu Gly Pro Ser Ala Ser Glu Gly Pro Ser Ala Ala Pro
            20                  25                  30
Pro Ser Glu Pro Leu Leu Glu Gly Ala Ala Pro Gln Pro Phe Thr Thr
        35                  40                  45
Ser Asp Asp Thr Pro Cys Gln Glu Gln Pro Lys Glu Val Leu Lys Ala
    50                  55                  60
Pro Ser Thr Ser Gly Leu Gln Gln Val Ala Phe Gln Pro Gly Gln Lys
65                  70                  75                  80
Val Tyr Val Trp Tyr Gly Gly Gln Glu Cys Thr Gly Leu Val Glu Gln
                85                  90                  95
His Ser Trp Met Glu Gly Gln Val Thr Val Trp Leu Leu Glu Gln Lys
            100                 105                 110
Leu Gln Val Cys Cys Arg Val Glu Glu Val Trp Leu Ala Glu Leu Gln
        115                 120                 125
Gly Pro Cys Pro Gln Ala Pro Pro Leu Glu Pro Gly Ala Gln Ala Leu
    130                 135                 140
Ala Tyr Arg Pro Val Ser Arg Asn Ile Asp Val Pro Lys Arg Lys Ser
145                 150                 155                 160
Asp Ala Val Glu Met Asp Glu Met Met Ala Ala Met Val Leu Thr Ser
                165                 170                 175
Leu Ser Cys Ser Pro Val Val Gln Ser Pro Pro Gly Thr Glu Ala Asn

                180                 185                 190
Phe Ser Ala Ser Arg Ala Ala Cys Asp Pro Trp Lys Glu Ser Gly Asp
        195                 200                 205
Ile Ser Asp Ser Gly Ser Ser Thr Thr Ser Gly His Trp Ser Gly Ser
    210                 215                 220
Ser Gly Val Ser Thr Pro Ser Pro Pro His Pro Gln Ala Ser Pro Lys
225                 230                 235                 240
Tyr Leu Gly Asp Ala Phe Gly Ser Pro Gln Thr Asp His Gly Phe Glu
                245                 250                 255
Thr Asp Pro Asp Pro Phe Leu Leu Asp Glu Pro Ala Pro Arg Lys Arg
            260                 265                 270
Lys Asn Ser Val Lys Val Met Tyr Lys Cys Leu Trp Pro Asn Cys Gly
        275                 280                 285
Lys Val Leu Arg Ser Ile Val Gly Ile Lys Arg His Val Lys Ala Leu
    290                 295                 300
His Leu Gly Asp Thr Val Asp Ser Asp Gln Phe Lys Arg Glu Glu Asp
305                 310                 315                 320
Phe Tyr Tyr Thr Glu Val Gln Leu Lys Glu Glu Ser Ala Ala Ala Ala
                325                 330                 335
Ala Ala Ala Ala Ala Gly Thr Pro Val Pro Gly Thr Pro Thr Ser Glu
            340                 345                 350
Pro Ala Pro Thr Pro Ser Met Thr Gly Leu Pro Leu Ser Ala Leu Pro
        355                 360                 365
Pro Pro Leu His Lys Ala Gln Ser Ser Gly Pro Glu His Pro Gly Pro
    370                 375                 380
Glu Ser Ser Leu Pro Ser Gly Ala Leu Ser Lys Ser Ala Pro Gly Ser
385                 390                 395                 400
Phe Trp His Ile Gln Ala Asp His Ala Tyr Gln Ala Leu Pro Ser Phe
                405                 410                 415
Gln Ile Pro Val Ser Pro His Ile Tyr Thr Ser Val Ser Trp Ala Ala
            420                 425                 430
Ala Pro Ser Ala Ala Cys Ser Leu Ser Pro Val Arg Ser Arg Ser Leu
        435                 440                 445
Ser Phe Ser Glu Pro Gln Gln Pro Ala Pro Ala Met Lys Ser His Leu
    450                 455                 460
Ile Val Thr Ser Pro Pro Arg Ala Gln Ser Gly Ala Arg Lys Ala Arg
465                 470                 475                 480
Gly Glu Ala Lys Lys Cys Arg Lys Val Tyr Gly Ile Glu His Arg Asp
                485                 490                 495
Gln Trp Cys Thr Ala Cys Arg Trp Lys Lys Ala Cys Gln Arg Phe Leu
            500                 505                 510
Asp

<210> 263
<211> 1030
<212> PRT
<213> Homo sapiens

<400> 263
Met Arg Glu Asn Tyr Glu Thr Leu Val Ser Val Gly Thr Ala Glu Leu
1               5                   10                  15
Leu Pro Leu Ser Ala Phe Leu Ser Pro Ser Glu Pro Gly Arg Ala Val
            20                  25                  30
Gly Gly Gly Ser His Ala Asp Glu Gly Gln Glu Pro Ala Gly Cys Gly
        35                  40                  45
Asp Pro Gln Gly Gly Gln Pro Arg His Ser Leu His Leu Thr Ala Leu
    50                  55                  60
Val Gln Leu Val Lys Glu Ile Pro Glu Phe Leu Phe Gly Glu Val Lys
65                  70                  75                  80
Gly Ala Met Asp Ser Pro Glu Ser Glu Ser Arg Gly Ala Ser Leu Asp
                85                  90                  95
Gly Glu Arg Ala Ser Pro Glu Ala Ala Ala Ala Arg Glu Pro Cys Pro
            100                 105                 110

Leu Arg Gly Leu Leu Ser Cys Leu Pro Asp Gly Pro Thr Ser Gln Pro
        115                 120                 125
His Leu Ala Thr Thr Pro Thr Asp Ser Ser Cys Ser Ser Gly Pro Thr
    130                 135                 140
Gly Asp Gly Val Gln Gly Ser Pro Leu Pro Ile Lys Thr Ala Asp Lys
145                 150                 155                 160
Pro Trp Pro Thr Arg Lys Glu Gly Pro Gly Ala Leu Gly Gly Glu Pro
                165                 170                 175
Ser Pro Pro Thr His Ser Pro Ser Arg Arg Lys Ser His Arg Gly Gln
            180                 185                 190
Glu Arg Gly Thr Ser Glu Ala Gly Ile Ser Pro Gly Asn Ser Pro Leu
        195                 200                 205
Gln Gly Leu Ile Asn Cys Leu Lys Glu Ile Leu Val Pro Gly Pro Arg
    210                 215                 220
His Pro Glu Thr Ser Pro Ser Phe Leu Pro Pro Leu Pro Ser Leu Gly
225                 230                 235                 240
Thr Ser Arg Leu Thr Arg Ala Asp Leu Gly Pro Gly Ser Pro Pro Trp
                245                 250                 255
Ala Val Lys Thr Glu Ala Val Ser Gly Asp Cys Pro Leu Gln Gly Leu
            260                 265                 270
Leu His Cys Leu Lys Glu Leu Pro Glu Ala Gln Asp Arg His Pro Ser
        275                 280                 285

Pro Ser Gly Val Gly Asn Arg Arg Leu Gln Glu Asn Pro Gly Ala Trp
    290                 295                 300
Lys Arg Gly Ser Gly Gly Pro Gly Tyr Leu Leu Thr Pro Pro Pro His
305                 310                 315                 320
Pro Asp Leu Gly Ala Gly Gly Leu Leu Ser Val Lys Met Glu Asn Ser
                325                 330                 335
Trp Val Gln Ser Pro Pro Gly Pro Ala Ser Cys Gln Pro Gly Arg Gln
            340                 345                 350
Pro Leu Ser Pro Ser Ala Thr Gly Asp Thr Arg Gly Val Pro Gln Pro
        355                 360                 365
Ser Trp Gly Pro Glu Ala Gln Ala Ala Ser Ala Ser Ser Ser Pro Leu
    370                 375                 380
Glu Ala Leu Glu Ala Cys Leu Lys Gly Ile Pro Pro Asn Gly Ser Ser
385                 390                 395                 400
Pro Ser Gln Leu Pro Pro Thr Ser Cys Ser Gln Asn Pro Gln Pro Gly
                405                 410                 415
Asp Ser Arg Ser Gln Lys Pro Glu Leu Gln Pro His Arg Ser His Ser
            420                 425                 430
Glu Glu Ala Thr Arg Glu Pro Val Leu Pro Leu Gly Leu Gln Ser Cys
        435                 440                 445
Val Arg Asp Gly Pro Ser Arg Pro Leu Ala Pro Arg Gly Thr Pro Thr
    450                 455                 460
Ser Phe Ser Ser Ser Ser Ser Thr Asp Trp Asp Leu Asp Phe Gly Ser
465                 470                 475                 480
Pro Val Gly Asn Gln Gly Gln His Pro Gly Lys Gly Ser Pro Pro Gly
                485                 490                 495
Ser Ser Pro Leu Gln Gly Leu Glu Asn Cys Leu Lys Glu Ile Pro Val
            500                 505                 510
Pro Val Leu Arg Pro Ala Trp Pro Cys Ser Ser Ala Ala Asp Arg Gly
        515                 520                 525
Pro Arg Arg Ala Glu Pro Arg Asn Trp Thr Ala Asp Lys Glu Gly Leu
    530                 535                 540
Arg Ala Glu Ala Cys Glu Ser Ala Arg Leu Gly Gln Gly Arg Gly Glu
545                 550                 555                 560
Ala Pro Thr Arg Ser Leu His Leu Val Ser Pro Gln Val Phe Thr Ser
                565                 570                 575
Ser Cys Val Pro Ala Cys His Gln Arg Gly Phe Lys Asp Pro Gly Ala
            580                 585                 590
Thr Arg Pro Gly Val Trp Arg Trp Leu Pro Glu Gly Ser Ala Pro Lys
        595                 600                 605

Pro Ser Pro Leu His Cys Leu Glu Ser Ala Leu Arg Gly Ile Leu Pro
    610                 615                 620
Val Arg Pro Leu Arg Phe Ala Cys Val Gly Gly Pro Ser Pro Ser Pro
625                 630                 635                 640
Ser Pro Gly Ser Ser Ser Ser Phe Ser Gly Ser Glu Gly Glu Asp Pro
                645                 650                 655
Arg Pro Glu Pro Glu Leu Trp Lys Pro Leu Pro Gln Glu Arg Asp Arg
            660                 665                 670
Leu Pro Ser Cys Lys Pro Pro Val Pro Leu Ser Pro Cys Pro Gly Gly
        675                 680                 685
Thr Pro Ala Gly Ser Ser Gly Gly Ser Pro Gly Glu Asp Pro Arg Arg
    690                 695                 700
Thr Glu Pro Arg Tyr Cys Ser Gly Leu Gly Ala Gly Thr Ala Gln Asp
705                 710                 715                 720
Pro Cys Pro Val Ser Gln Leu Glu Lys Arg Pro Arg Val Ser Glu Ala
                725                 730                 735
Ser Arg Gly Leu Glu Leu Gly His Gly Arg Pro Arg Val Ala Ala Lys
            740                 745                 750
Thr His Glu Arg Leu Leu Pro Gln Gly Pro Pro Glu Leu Pro Ser Glu
        755                 760                 765

Ser Pro Pro Pro Glu Leu Pro Pro Pro Glu Ala Ala Pro Pro Val Leu
    770                 775                 780
Pro Ala Ser Ser Leu Gln Pro Pro Cys His Cys Gly Lys Pro Leu Gln
785                 790                 795                 800
Gln Glu Leu His Ser Leu Gly Ala Ala Leu Ala Glu Lys Leu Asp Arg
                805                 810                 815
Leu Ala Thr Ala Leu Ala Gly Leu Ala Gln Glu Val Ala Thr Met Arg
            820                 825                 830
Thr Gln Val Asn Arg Leu Gly Arg Arg Pro Gln Gly Pro Gly Pro Met
        835                 840                 845
Gly Gln Ala Ser Trp Met Trp Thr Leu Pro Arg Gly Pro Arg Trp Ala
    850                 855                 860
His Gly Pro Gly His Arg His Leu Pro Tyr Trp Arg Gln Lys Gly Pro
865                 870                 875                 880
Thr Arg Pro Lys Pro Lys Ile Leu Arg Gly Gln Gly Glu Ser Cys Arg
                885                 890                 895
Ala Gly Asp Leu Gln Gly Leu Ser Arg Gly Thr Ala Arg Arg Ala Arg
            900                 905                 910
Pro Leu Pro Pro Asp Ala Pro Pro Ala Glu Pro Pro Gly Leu His Cys
        915                 920                 925
Ser Ser Ser Gln Gln Leu Leu Ser Ser Thr Pro Ser Cys His Ala Ala
    930                 935                 940
Pro Pro Ala His Pro Leu Leu Ala His Thr Gly Gly His Gln Ser Pro
945                 950                 955                 960
Leu Pro Pro Leu Val Pro Ala Ala Leu Pro Leu Gln Gly Ala Ser Pro
                965                 970                 975
Pro Ala Ala Ser Ala Asp Ala Asp Val Pro Thr Ser Gly Val Ala Pro
            980                 985                 990
Asp Gly Ile Pro Glu Arg Pro Lys Glu Pro Ser Ser Leu Leu Gly Gly
        995                 1000                1005
Val Gln Arg Ala Leu Gln Glu Glu Leu Trp Gly Gly Glu His Arg Asp
    1010                1015                1020
Pro Arg Trp Gly Ala His
1025                1030

<210> 264
<211> 616
<212> PRT
<213> Homo sapiens

<400> 264
Pro Ala Gly Cys Gly Asp Pro Gln Gly Gly Gln Pro Arg His Ser Leu

1               5                   10                  15
His Leu Thr Ala Leu Val Gln Leu Val Lys Glu Ile Pro Glu Phe Leu
                20                  25                  30
Phe Gly Glu Val Lys Gly Ala Met Asp Ser Pro Glu Ser Glu Ser Arg
        35                  40                  45
Gly Ala Ser Leu Asp Gly Glu Arg Ala Ser Pro Glu Ala Ala Ala Arg
    50                  55                  60
Glu Pro Cys Pro Leu Arg Gly Leu Leu Ser Cys Leu Pro Asp Gly Pro
65                  70                  75                  80
Thr Ser Gln Pro His Leu Ala Thr Thr Pro Thr Asp Ser Ser Cys Ser
                85                  90                  95
Ser Gly Pro Thr Gly Asp Gly Val Gln Gly Ser Pro Leu Pro Ile Lys
            100                 105                 110
Thr Ala Asp Lys Pro Trp Pro Thr Arg Lys Glu Gly Pro Gly Ala Leu
        115                 120                 125
Gly Gly Glu Pro Ser Pro Pro Thr His Ser Pro Ser Arg Arg Lys Ser
    130                 135                 140
His Arg Gly Gln Glu Arg Gly Thr Ser Glu Ala Gly Ile Ser Pro Gly
145                 150                 155                 160
Asn Ser Pro Leu Gln Gly Leu Ile Asn Cys Leu Lys Glu Ile Leu Val
                165                 170                 175
Pro Gly Pro Arg His Pro Glu Thr Ser Pro Ser Phe Leu Pro Pro Leu
            180                 185                 190
Pro Ser Leu Gly Thr Ser Arg Leu Thr Arg Ala Asp Leu Gly Pro Gly
        195                 200                 205
Ser Pro Pro Trp Ala Val Lys Thr Glu Ala Val Ser Gly Asp Cys Pro
    210                 215                 220
Leu Gln Gly Leu Leu His Cys Leu Lys Glu Leu Pro Glu Ala Gln Asp
225                 230                 235                 240
Arg His Pro Ser Pro Ser Gly Val Gly Asn Arg Arg Leu Gln Glu Asn
                245                 250                 255
Pro Gly Ala Trp Lys Arg Gly Ser Gly Gly Pro Gly Tyr Leu Leu Thr
            260                 265                 270
Pro Pro Pro His Pro Asp Leu Gly Ala Gly Gly Leu Leu Ser Val Lys
        275                 280                 285
Met Glu Asn Ser Trp Val Gln Ser Pro Pro Gly Pro Ala Ser Cys Gln
    290                 295                 300
Pro Gly Arg Gln Pro Leu Ser Pro Ser Ala Thr Gly Asp Thr Arg Gly
305                 310                 315                 320
Val Pro Gln Pro Ser Trp Gly Pro Glu Ala Gln Ala Ala Ser Ala Ser
                325                 330                 335
Ser Ser Pro Leu Glu Ala Leu Glu Ala Cys Leu Lys Gly Ile Pro Pro
            340                 345                 350
Asn Gly Ser Ser Pro Ser Gln Leu Pro Pro Thr Ser Cys Ser Gln Asn
        355                 360                 365
Pro Gln Pro Gly Asp Ser Arg Ser Gln Lys Pro Glu Leu Gln Pro His
    370                 375                 380
Arg Ser His Ser Glu Glu Ala Thr Arg Glu Pro Val Leu Pro Leu Gly
385                 390                 395                 400
Leu Gln Ser Cys Val Arg Asp Gly Pro Ser Arg Pro Leu Ala Pro Arg
                405                 410                 415
Gly Thr Pro Thr Ser Phe Ser Ser Ser Ser Ser Thr Asp Trp Asp Leu
            420                 425                 430
Asp Phe Gly Ser Pro Val Gly Asn Gln Gly Gln His Pro Gly Lys Gly
        435                 440                 445
Ser Pro Pro Gly Ser Ser Pro Leu Gln Gly Leu Glu Asn Cys Leu Lys
    450                 455                 460
Glu Ile Pro Val Pro Val Leu Arg Pro Ala Trp Pro Cys Ser Ser Ala
465                 470                 475                 480
Ala Asp Arg Gly Pro Arg Arg Ala Glu Pro Arg Asn Trp Thr Ala Asp
                485                 490                 495
Lys Glu Gly Ser Ala Pro Lys Pro Ser Pro Leu His Cys Leu Glu Ser
            500                 505                 510

Ala Leu Arg Gly Ile Leu Pro Val Arg Pro Leu Arg Phe Ala Cys Val
        515                 520                 525
Gly Gly Pro Ser Pro Ser Pro Ser Pro Gly Ser Ser Ser Ser Phe Ser
    530                 535                 540
Gly Ser Glu Gly Glu Asp Pro Arg Pro Glu Pro Asp Leu Trp Lys Pro
545                 550                 555                 560
Leu Pro Gln Glu Arg Asp Arg Leu Pro Ser Cys Lys Pro Pro Val Pro
                565                 570                 575
Leu Ser Pro Cys Pro Gly Gly Thr Pro Ala Gly Ser Ser Gly Gly Ser
            580                 585                 590
Pro Gly Glu Asp Pro Arg Arg Thr Glu Pro Arg Tyr Cys Ser Gly Leu
        595                 600                 605
Gly Ala Gly Glu Pro Gly Val Ser
    610                 615

<210> 265
<211> 256
<212> PRT
<213> Homo sapiens

<400> 265
Arg Thr Arg Gly Arg Thr Arg Gly Arg Leu Arg Ser Gly Val Met Met
1               5                   10                  15
Pro Leu Ala Glu Ala Gly Ala Leu Ala Gln Gly Gly Gly Pro Ser Ala
            20                  25                  30
Thr Glu Trp Ala Cys Ile Leu Arg Arg Lys Thr Pro Arg His Lys Gln
        35                  40                  45
Pro Thr Leu Leu Met Val Arg Ala Ser Arg Arg Ser Gly Lys Thr Ser
    50                  55                  60
Ala Val Leu Lys Ala Gly Arg Gln Ser Val Ser Gly Arg Lys Asn Ser
65                  70                  75                  80
Thr Ser Lys Asp Leu Val Thr Leu Gly Ala Ser Ser Leu Arg Glu Glu
                85                  90                  95
Arg Gly His Pro Leu His Pro Arg His Arg Lys Ala Val His Leu Arg
            100                 105                 110
Thr Arg Gly Arg Thr Arg Gly Trp Val Gln Thr Leu Ala Arg Met Ser
        115                 120                 125
Arg Arg Thr Arg Gly Pro Val Glu Arg Ala Ala Ala Ala Ala Ala Ala
    130                 135                 140
Ala Ala Gly Gly Asp Ala Gly His Ala Pro Phe Pro Pro Pro Pro Ala
145                 150                 155                 160
Ala Asp Gly Ala Arg Ala Pro Arg Ser Pro Gly Gln Val Thr Pro Arg
                165                 170                 175
Gly Leu Arg Leu Arg Leu Pro Arg Arg Glu Ser Leu Leu Arg Gly Leu
            180                 185                 190
Cys Arg Pro Leu Arg Pro Leu Leu Gly Phe Arg Glu Ser Asp Ser Ala
        195                 200                 205
Lys Pro Ala Ser Leu Arg Leu Leu Gln His Thr Pro Ser Ala Arg Arg
    210                 215                 220
Asn Tyr Arg Ile Ala Gly Ala Arg Leu Met Arg Ser Asn Tyr Pro Pro
225                 230                 235                 240
Pro Leu Ser Ser Ala Ala Leu Arg Gly Ala Gly Pro Thr Arg Arg Asn
                245                 250                 255

<210> 266
<211> 264
<212> PRT
<213> Homo sapiens

<400> 266
Met Thr Lys Phe Ser Ser Phe Ser Leu Phe Phe Leu Ile Val Gly Ala
1               5                   10                  15

Tyr Met Thr His Val Cys Phe Asn Met Glu Ile Ile Gly Gly Lys Glu
            20                  25                  30
Val Ser Pro His Ser Arg Pro Phe Met Ala Ser Ile Gln Tyr Gly Gly
        35                  40                  45
His His Val Cys Gly Gly Val Leu Ile Asp Pro Gln Trp Val Leu Thr
    50                  55                  60
Ala Ala His Cys Gln Tyr Arg Phe Thr Lys Gly Gln Ser Pro Thr Val
65                  70                  75                  80
Val Leu Gly Ala His Ser Leu Ser Lys Asn Glu Ala Ser Lys Gln Thr
                85                  90                  95
Leu Glu Ile Lys Lys Phe Ile Pro Phe Ser Arg Val Thr Ser Asp Pro
            100                 105                 110
Gln Ser Asn Asp Ile Met Leu Val Lys Leu Gln Thr Ala Ala Lys Leu
        115                 120                 125
Asn Lys His Val Lys Met Leu His Ile Arg Ser Lys Thr Ser Leu Arg
    130                 135                 140
Ser Gly Thr Lys Cys Lys Val Thr Gly Trp Gly Ala Thr Asp Pro Asp
145                 150                 155                 160
Ser Leu Arg Pro Ser Asp Thr Leu Arg Glu Val Thr Val Thr Val Leu
                165                 170                 175
Ser Arg Lys Leu Cys Asn Ser Gln Ser Tyr Tyr Asn Gly Asp Pro Phe
            180                 185                 190
Ile Thr Lys Asp Met Val Cys Ala Gly Asp Ala Lys Gly Gln Lys Asp
        195                 200                 205
Ser Cys Lys Gly Asp Ser Gly Gly Pro Leu Ile Cys Lys Gly Val Phe
    210                 215                 220
His Ala Ile Val Ser Gly Gly His Glu Cys Gly Val Ala Thr Lys Pro
225                 230                 235                 240
Gly Ile Tyr Thr Leu Leu Thr Lys Lys Tyr Gln Thr Trp Ile Lys Ser
                245                 250                 255
Asn Leu Val Pro Pro His Thr Asn
            260

<210> 267
<211> 1531
<212> PRT
<213> Homo sapiens

<400> 267
Met Gln Val Asn Thr Leu Phe Glu Thr Ser Gln Val Pro Asp Trp Ser
1               5                   10                  15
Asp Pro Pro Gln Val Gln Val Gln Glu Thr Val Arg Glu Thr Ile Ser
            20                  25                  30
Cys Ser Gln Met Pro Ala Phe Ser Glu Pro Ala Gly Glu Glu Ser Pro
        35                  40                  45
Phe Thr Gly Thr Thr Thr Ile Ser Phe Ser Asn Leu Gly Gly Val His
    50                  55                  60
Lys Glu Asn Ala Ser Leu Ala Gln His Ser Glu Val Lys Pro Cys Thr
65                  70                  75                  80
Cys Gly Pro Gln Gln Glu Glu Lys Gln Asp Arg Asp Gly Asn Ile Pro
                85                  90                  95
Asp Asn Phe Arg Glu Asp Leu Lys Tyr Glu Gln Ser Ile Ser Glu Ala
            100                 105                 110
Asn Asp Glu Thr Met Ser Pro Gly Val Phe Ser Arg His Leu Pro Lys
        115                 120                 125
Asp Ala Arg Ala Asp Phe Arg Glu Pro Val Ala Val Ser Val Ala Ser
    130                 135                 140
Pro Glu Pro Thr Asp Thr Ala Leu Thr Leu Glu Asn Val Cys Asp Glu
145                 150                 155                 160
Pro Arg Asp Arg Glu Ala Val Cys Ala Met Glu Cys Phe Glu Ala Ser
                165                 170                 175
Asp Gln Gly Thr Cys Phe Asp Thr Ile Asp Ser Leu Val Gly Thr Pro
            180                 185                 190

Val Asp Asn Tyr Ser Pro Gln Glu Ile Cys Ser Val Asp Thr Glu Leu
        195                 200                 205
Ala Glu Gly Gln Asn Lys Val Ser Asp Leu Cys Ser Ser Asn Asp Lys
    210                 215                 220
Thr Leu Glu Val Phe Phe Gln Thr Gln Val Ser Glu Thr Ser Val Ser
225                 230                 235                 240
Thr Cys Lys Ser Ser Lys Asp Gly Asn Ser Val Met Ser Pro Leu Phe
                245                 250                 255
Ile Ser Thr Phe Thr Leu Asn Ile Ser His Thr Ala Ser Glu Gly Ala
            260                 265                 270
Thr Gly Glu Asn Leu Ala Lys Val Glu Lys Ser Thr Tyr Pro Leu Ala
        275                 280                 285
Ser Thr Val His Ala Gly Gln Glu Gln Pro Ser Pro Ser Asn Ser Gly
    290                 295                 300
Gly Leu Asp Glu Thr Gln Leu Leu Ser Ser Glu Asn Asn Pro Leu Val
305                 310                 315                 320
Gln Phe Lys Glu Gly Gly Asp Lys Ser Pro Ser Pro Ser Ala Ala Asp
                325                 330                 335
Thr Thr Ala Thr Pro Ala Ser Tyr Ser Ser Ile Val Ser Phe Pro Trp
            340                 345                 350
Glu Lys Pro Thr Thr Leu Thr Ala Asn Asn Glu Cys Phe Gln Ala Thr
        355                 360                 365
Arg Glu Thr Val Thr Ile Ala Thr Glu Val His Pro Ala Lys Tyr Leu
    370                 375                 380
Ala Val Ser Ile Pro Glu Asp Lys His Ala Gly Gly Thr Glu Glu Arg
385                 390                 395                 400
Phe Pro Arg Ala Ser His Glu Lys Val Ser Gln Phe Pro Ser Gln Val
                405                 410                 415
Gln Val Asp His Ile Leu Ser Gly Ala Thr Ile Lys Ser Thr Lys Glu
            420                 425                 430
Leu Leu Cys Arg Ala Pro Ser Val Pro Gly Val Pro His His Val Leu
        435                 440                 445
Gln Leu Pro Glu Gly Glu Gly Phe Cys Ser Asn Ser Pro Leu Gln Val
    450                 455                 460
Asp Asn Leu Ser Gly Asp Lys Ser Gln Thr Val Asp Arg Ala Asp Phe
465                 470                 475                 480
Arg Ser Tyr Glu Glu Asn Phe Gln Glu Arg Gly Ser Glu Thr Lys Gln
                485                 490                 495
Gly Val Gln Gln Gln Ser Leu Ser Gln Gln Gly Ser Leu Ser Ala Pro
            500                 505                 510
Asp Phe Gln Gln Ser Leu Pro Thr Thr Ser Ala Ala Gln Glu Glu Arg
        515                 520                 525
Asn Leu Val Pro Thr Ala Pro Ser Pro Ala Ser Ser Arg Glu Gly Ala
    530                 535                 540
Gly Gln Arg Ser Gly Trp Gly Thr Arg Val Ser Val Val Ala Glu Thr
545                 550                 555                 560
Ala Gly Glu Glu Asp Ser Gln Ala Leu Ser Asn Val Pro Ser Leu Ser
                565                 570                 575
Asp Ile Leu Leu Glu Glu Ser Lys Glu Tyr Arg Pro Gly Asn Trp Glu
            580                 585                 590
Ala Gly Asn Lys Leu Lys Ile Ile Thr Leu Glu Ala Ser Ala Ser Glu
        595                 600                 605
Ile Trp Pro Pro Arg Gln Leu Thr Asn Ser Glu Ser Lys Ala Ser Asp
    610                 615                 620
Gly Gly Leu Ile Ile Pro Asp Lys Val Trp Ala Val Pro Asp Ser Leu
625                 630                 635                 640
Lys Ala Asp Ala Val Val Pro Glu Leu Ala Pro Ser Glu Ile Ala Ala
                645                 650                 655
Leu Ala His Ser Pro Glu Asp Ala Glu Ser Ala Leu Ala Asp Ser Arg
            660                 665                 670
Glu Ser His Lys Gly Glu Glu Pro Thr Ile Ser Val His Trp Arg Ser
        675                 680                 685
Leu Ser Ser Arg Gly Phe Ser Gln Pro Arg Leu Leu Glu Ser Ser Val

690                 695                 700
Asp Pro Val Asp Glu Lys Glu Leu Ser Val Thr Asp Ser Leu Ser Ala
705                 710                 715                 720
Ala Ser Glu Thr Gly Gly Lys Glu Asn Val Asn Asn Val Ser Gln Asp
                725                 730                 735
Gln Glu Glu Lys Gln Leu Lys Met Asp His Thr Ala Phe Phe Lys Lys
            740                 745                 750
Phe Leu Thr Cys Pro Lys Ile Leu Glu Ser Ser Val Asp Pro Ile Asp
        755                 760                 765

Glu Ile Ser Val Ile Glu Tyr Thr Arg Ala Gly Lys Pro Glu Pro Ser
    770                 775                 780
Glu Thr Thr Pro Gln Gly Ala Arg Glu Gly Gly Gln Ser Asn Asp Gly
785                 790                 795                 800
Asn Met Gly His Glu Ala Glu Ile Gln Ser Ala Ile Leu Gln Val Pro
                805                 810                 815
Cys Leu Gln Gly Thr Ile Leu Ser Glu Asn Arg Ile Ser Arg Ser Gln
            820                 825                 830
Glu Gly Ser Met Lys Gln Glu Ala Glu Gln Ile Gln Pro Glu Glu Ala
        835                 840                 845
Lys Thr Ala Ile Trp Gln Val Leu Gln Pro Ser Glu Gly Gly Glu Arg
    850                 855                 860
Ile Pro Ser Gly Cys Ser Ile Gly Gln Ile Gln Glu Ser Ser Asp Gly
865                 870                 875                 880
Ser Leu Gly Glu Ala Glu Gln Ser Lys Lys Asp Lys Ala Glu Leu Ile
                885                 890                 895
Ser Pro Thr Ser Pro Leu Ser Ser Cys Leu Pro Ile Met Thr His Ser
            900                 905                 910
Ser Leu Gly Val Asp Thr His Asn Ser Thr Gly Gln Ile His Asp Val
        915                 920                 925
Pro Glu Asn Asp Ile Val Glu Pro Arg Lys Arg Gln Tyr Val Phe Pro
    930                 935                 940
Val Ser Gln Lys Arg Gly Thr Ile Glu Asn Glu Arg Gly Lys Pro Leu
945                 950                 955                 960
Pro Ser Ser Pro Asp Leu Thr Arg Phe Pro Cys Thr Ser Ser Pro Glu
                965                 970                 975
Gly Asn Val Thr Asp Phe Leu Ile Ser His Lys Met Glu Glu Pro Lys
            980                 985                 990
Ile Glu Val Leu Gln Ile Gly Glu Thr Lys Pro Pro Ser Ser Ser Ser
        995                 1000                1005
Ser Ser Ala Lys Thr Leu Ala Phe Ile Ser Gly Glu Arg Glu Leu Glu
    1010                1015                1020
Lys Ala Pro Lys Leu Leu Gln Asp Pro Cys Gln Lys Gly Thr Leu Gly
1025                1030                1035                1040
Cys Ala Lys Lys Ser Arg Glu Arg Glu Lys Ser Leu Glu Ala Arg Ala
                1045                1050                1055
Gly Lys Ser Pro Gly Thr Leu Thr Ala Val Thr Gly Ser Glu Glu Val
            1060                1065                1070
Lys Arg Lys Pro Glu Ala Pro Gly Ser Gly His Leu Ala Glu Gly Val
        1075                1080                1085
Lys Lys Lys Ile Leu Ser Arg Val Ala Ala Leu Arg Leu Lys Leu Glu
    1090                1095                1100
Glu Lys Glu Asn Ile Arg Lys Asn Ser Ala Phe Leu Lys Lys Met Pro
1105                1110                1115                1120
Lys Leu Glu Thr Ser Leu Ser His Thr Glu Glu Lys Gln Asp Pro Lys
                1125                1130                1135
Lys Pro Ser Cys Lys Arg Glu Gly Arg Ala Pro Val Leu Leu Lys Lys
            1140                1145                1150
Ile Gln Ala Glu Met Phe Pro Glu His Ser Gly Asn Val Lys Leu Ser
        1155                1160                1165
Cys Gln Phe Ala Glu Ile His Glu Asp Ser Thr Ile Cys Trp Thr Lys
    1170                1175                1180
Asp Ser Lys Ser Ile Ala Gln Val Gln Arg Ser Ala Gly Asp Asn Ser

1185                1190                1195                1200
Thr Val Ser Phe Ala Ile Val Gln Ala Ser Pro Lys Asp Gln Gly Leu
                1205                1210                1215
Tyr Tyr Cys Cys Ile Lys Asn Ser Tyr Gly Lys Val Thr Ala Glu Phe
            1220                1225                1230
Asn Leu Thr Ala Glu Val Leu Lys Gln Leu Ser Ser Arg Gln Asp Thr
        1235                1240                1245

Lys Gly Cys Glu Glu Ile Glu Phe Ser Gln Leu Ile Phe Lys Glu Asp
    1250                1255                1260
Phe Leu His Asp Ser Tyr Phe Gly Gly Arg Leu Arg Gly Gln Ile Ala
1265                1270                1275                1280
Thr Glu Glu Leu His Phe Gly Glu Gly Val His Arg Lys Ala Phe Arg
                1285                1290                1295
Ser Thr Val Met His Gly Leu Met Pro Val Phe Lys Pro Gly His Ala
            1300                1305                1310
Cys Val Leu Lys Val His Asn Ala Ile Ala Tyr Gly Thr Arg Asn Asn
        1315                1320                1325
Asp Glu Leu Ile Gln Arg Asn Tyr Lys Leu Ala Ala Gln Glu Cys Tyr
    1330                1335                1340
Val Gln Asn Thr Ala Arg Tyr Tyr Ala Lys Ile Tyr Ala Ala Glu Ala
1345                1350                1355                1360
Gln Pro Leu Glu Gly Phe Gly Glu Val Pro Glu Ile Ile Pro Ile Phe
                1365                1370                1375
Leu Ile His Arg Pro Glu Asn Asn Ile Pro Tyr Ala Thr Val Glu Glu
            1380                1385                1390
Glu Leu Ile Gly Glu Phe Val Lys Tyr Ser Ile Arg Asp Gly Lys Glu
        1395                1400                1405
Ile Asn Phe Leu Arg Arg Glu Ser Glu Ala Gly Gln Lys Cys Cys Thr
    1410                1415                1420
Phe Gln His Trp Val Tyr Gln Lys Thr Ser Gly Cys Leu Leu Val Thr
1425                1430                1435                1440
Asp Met Gln Gly Val Gly Met Lys Leu Thr Asp Val Gly Ile Ala Thr
                1445                1450                1455
Leu Ala Lys Gly Tyr Lys Gly Phe Lys Gly Asn Cys Ser Met Thr Phe
            1460                1465                1470
Ile Asp Gln Phe Lys Ala Leu His Gln Cys Asn Lys Tyr Cys Lys Met
        1475                1480                1485
Leu Gly Leu Lys Ser Leu Gln Asn Asn Asn Gln Lys Gln Lys Gln Pro
    1490                1495                1500
Ser Ile Gly Lys Ser Lys Val Gln Thr Asn Ser Met Thr Val Lys Lys
1505                1510                1515                1520
Ala Gly Pro Glu Thr Pro Gly Glu Lys Lys Thr
                1525                1530

<210> 268
<211> 193
<212> PRT
<213> Homo sapiens

<400> 268
Met Gly Lys Gln Asn Ser Lys Leu Arg Pro Glu Val Leu Gln Asp Leu
1               5                   10                  15
Arg Glu Asn Thr Glu Phe Thr Asp His Glu Leu Gln Glu Trp Tyr Lys
            20                  25                  30
Gly Phe Leu Lys Asp Cys Pro Thr Gly His Leu Thr Val Asp Glu Phe
        35                  40                  45
Lys Lys Ile Tyr Ala Asn Phe Phe Pro Tyr Gly Asp Ala Ser Lys Phe
    50                  55                  60
Ala Glu His Val Phe Arg Thr Phe Asp Thr Asn Gly Asp Gly Thr Ile
65                  70                  75                  80
Asp Phe Arg Glu Phe Ile Ile Ala Leu Ser Val Thr Ser Arg Gly Lys
                85                  90                  95

Leu Glu Gln Lys Leu Lys Trp Ala Phe Ser Met Tyr Asp Leu Asp Gly
            100                 105                 110
Asn Gly Tyr Ile Ser Arg Ser Glu Met Leu Glu Ile Val Gln Ala Ile
        115                 120                 125
Tyr Lys Met Val Ser Ser Val Met Lys Met Pro Glu Asp Glu Ser Thr
    130                 135                 140
Pro Glu Lys Arg Thr Asp Lys Ile Phe Arg Gln Met Asp Thr Asn Asn
145                 150                 155                 160
Asp Gly Lys Leu Ser Leu Glu Glu Phe Ile Arg Gly Ala Lys Ser Asp
                165                 170                 175
Pro Ser Ile Val Arg Leu Leu Gln Cys Asp Pro Ser Ser Ala Ser Gln
            180                 185                 190
Phe

<210> 269
<211> 193
<212> PRT
<213> Homo sapiens

<400> 269
Met Gly Lys Gln Asn Ser Lys Leu Arg Pro Glu Val Leu Gln Asp Leu
1               5                   10                  15
Arg Glu Asn Thr Glu Phe Thr Asp His Glu Leu Gln Glu Trp Tyr Lys
            20                  25                  30
Gly Phe Leu Lys Asp Cys Pro Thr Gly His Leu Thr Val Asp Glu Phe
        35                  40                  45
Lys Lys Ile Tyr Ala Asn Phe Phe Pro Tyr Gly Asp Ala Ser Lys Phe
    50                  55                  60
Ala Glu His Val Phe Arg Thr Phe Asp Thr Asn Gly Asp Gly Thr Ile
65                  70                  75                  80
Asp Phe Arg Glu Phe Ile Ile Ala Leu Ser Val Thr Ser Arg Gly Lys
                85                  90                  95
Leu Glu Gln Lys Leu Lys Trp Ala Phe Ser Met Tyr Asp Leu Asp Gly
            100                 105                 110
Asn Gly Tyr Ile Ser Arg Ser Glu Met Leu Glu Ile Val Gln Ala Ile
        115                 120                 125
Tyr Lys Met Val Ser Ser Val Met Lys Met Pro Glu Asp Glu Ser Thr
    130                 135                 140
Pro Glu Lys Arg Thr Asp Lys Ile Phe Arg Gln Met Asp Thr Asn Asn
145                 150                 155                 160
Asp Gly Lys Leu Ser Leu Glu Glu Phe Ile Arg Gly Ala Lys Ser Asp
                165                 170                 175
Pro Ser Ile Val Arg Leu Leu Gln Cys Asp Pro Ser Ser Ala Ser Gln
            180                 185                 190
Phe

<210> 270
<211> 208
<212> PRT
<213> Homo sapiens

<400> 270
Met Gly Asn Ala Gln Glu Arg Pro Ser Glu Thr Ile Asp Arg Glu Arg
1               5                   10                  15
Lys Arg Leu Val Glu Thr Leu Gln Ala Asp Ser Gly Leu Leu Leu Asp
            20                  25                  30
Ala Leu Leu Ala Arg Gly Val Leu Thr Gly Pro Glu Tyr Glu Ala Leu
        35                  40                  45
Asp Ala Leu Pro Asp Ala Glu Arg Arg Val Arg Arg Leu Leu Leu Leu
    50                  55                  60
Val Gln Gly Lys Gly Glu Ala Ala Cys Gln Glu Leu Leu Arg Cys Ala
65                  70                  75                  80
Gln Arg Thr Ala Gly Ala Pro Asp Pro Ala Trp Asp Trp Gln His Val

85                  90                  95
Gly Pro Gly Tyr Arg Asp Arg Ser Tyr Asp Pro Pro Cys Pro Gly His
            100                 105                 110

Trp Thr Pro Glu Ala Pro Gly Ser Gly Thr Thr Cys Pro Gly Leu Pro
        115                 120                 125
Arg Ala Ser Asp Pro Asp Glu Ala Gly Gly Pro Glu Gly Ser Glu Ala
    130                 135                 140
Val Gln Ser Gly Thr Pro Glu Glu Pro Glu Pro Glu Leu Glu Ala Glu
145                 150                 155                 160
Ala Ser Lys Glu Ala Glu Pro Glu Pro Glu Pro Glu Pro Glu Leu Glu
                165                 170                 175
Pro Glu Ala Glu Ala Glu Pro Glu Pro Glu Leu Glu Pro Glu Pro Asp
            180                 185                 190
Pro Glu Pro Glu Pro Asp Phe Glu Glu Arg Asp Glu Ser Glu Asp Ser
        195                 200                 205

<210> 271
<211> 465
<212> PRT
<213> Homo sapiens

<400> 271
Met Gly Gly Ala Val Val Asp Glu Gly Pro Thr Gly Val Lys Ala Pro
1               5                   10                  15
Asp Gly Gly Trp Gly Trp Ala Val Leu Phe Gly Cys Phe Val Ile Thr
            20                  25                  30
Gly Phe Ser Tyr Ala Phe Pro Lys Ala Val Ser Val Phe Phe Lys Glu
        35                  40                  45
Leu Ile Gln Glu Phe Gly Ile Gly Tyr Ser Asp Thr Ala Trp Ile Ser
    50                  55                  60
Ser Ile Leu Leu Ala Met Leu Tyr Gly Thr Gly Pro Leu Cys Ser Val
65                  70                  75                  80
Cys Val Asn Arg Phe Gly Cys Arg Pro Val Met Leu Val Gly Gly Leu
                85                  90                  95
Phe Ala Ser Leu Gly Met Val Ala Ala Ser Phe Cys Arg Ser Ile Ile
            100                 105                 110
Gln Val Tyr Leu Thr Thr Gly Val Ile Thr Gly Leu Gly Leu Ala Leu
        115                 120                 125
Asn Phe Gln Pro Ser Leu Ile Met Leu Asn Arg Tyr Phe Ser Lys Arg
    130                 135                 140
Arg Pro Met Ala Asn Gly Leu Ala Ala Ala Gly Ser Pro Val Phe Leu
145                 150                 155                 160
Cys Ala Leu Ser Pro Leu Gly Gln Leu Leu Gln Asp Arg Tyr Gly Trp
                165                 170                 175
Arg Gly Gly Phe Leu Ile Leu Gly Gly Leu Leu Leu Asn Cys Cys Val
            180                 185                 190
Cys Ala Ala Leu Met Arg Pro Leu Val Val Thr Ala Gln Pro Gly Ser
        195                 200                 205
Gly Pro Pro Arg Pro Ser Arg Arg Leu Leu Asp Leu Ser Val Phe Arg
    210                 215                 220
Asp Arg Gly Phe Val Leu Tyr Ala Val Ala Ala Ser Val Met Val Leu
225                 230                 235                 240
Gly Leu Phe Val Pro Pro Val Phe Val Val Ser Tyr Ala Lys Asp Leu
                245                 250                 255
Gly Val Pro Asp Thr Lys Ala Ala Phe Leu Leu Thr Ile Leu Gly Phe
            260                 265                 270
Ile Asp Ile Phe Ala Arg Pro Ala Ala Gly Phe Val Ala Gly Leu Gly
        275                 280                 285
Lys Val Arg Pro Tyr Ser Val Tyr Leu Phe Ser Phe Ser Met Phe Phe
    290                 295                 300
Asn Gly Leu Ala Asp Leu Ala Gly Ser Thr Ala Gly Asp Tyr Gly Gly
305                 310                 315                 320

Leu Val Val Phe Cys Ile Phe Phe Gly Ile Ser Tyr Gly Met Val Gly
                325                 330                 335
Ala Leu Gln Phe Glu Val Leu Met Ala Ile Val Gly Thr His Lys Phe
                340                 345                 350
Ser Ser Ala Ile Gly Leu Val Leu Leu Met Glu Ala Val Ala Val Leu
        355                 360                 365
Val Gly Pro Pro Ser Gly Gly Lys Leu Leu Asp Ala Thr His Val Tyr
    370                 375                 380
Met Tyr Val Phe Ile Leu Ala Gly Ala Glu Val Leu Thr Ser Ser Leu
385                 390                 395                 400
Ile Leu Leu Leu Gly Asn Phe Phe Cys Ile Arg Lys Lys Pro Lys Glu
                405                 410                 415
Pro Gln Pro Glu Val Ala Ala Ala Glu Glu Glu Lys Leu His Lys Pro
                420                 425                 430
Pro Ala Asp Ser Gly Val Asp Leu Arg Glu Val Glu His Phe Leu Lys
        435                 440                 445
Ala Glu Pro Glu Lys Asn Gly Glu Val Val His Thr Pro Glu Thr Ser
    450                 455                 460
Val
465

<210> 272
<211> 475
<212> PRT
<213> Homo sapiens

<400> 272
Met Ala Ala Leu Thr Thr Leu Phe Lys Tyr Ile Asp Glu Asn Gln Asp
 1               5                  10                  15
Arg Tyr Ile Lys Lys Leu Ala Lys Trp Val Ala Ile Gln Ser Val Ser
                20                  25                  30
Ala Trp Pro Glu Lys Arg Gly Glu Ile Arg Arg Met Met Glu Val Ala
        35                  40                  45
Ala Ala Asp Val Lys Gln Leu Gly Gly Ser Val Glu Leu Val Asp Ile
    50                  55                  60
Gly Lys Gln Lys Leu Pro Asp Gly Ser Glu Ile Pro Leu Pro Pro Ile
65                  70                  75                  80
Leu Leu Gly Arg Leu Gly Ser Asp Pro Gln Lys Lys Thr Val Cys Ile
                85                  90                  95
Tyr Gly His Leu Asp Val Gln Pro Ala Ala Leu Glu Asp Gly Trp Asp
                100                 105                 110
Ser Glu Pro Phe Thr Leu Val Glu Arg Asp Gly Lys Leu Tyr Gly Arg
        115                 120                 125
Gly Ser Thr Asp Asp Lys Gly Pro Val Ala Gly Trp Ile Asn Ala Leu
    130                 135                 140
Glu Ala Tyr Gln Lys Thr Gly Gln Glu Ile Pro Val Asn Val Arg Phe
145                 150                 155                 160
Cys Leu Glu Gly Met Glu Glu Ser Gly Ser Glu Gly Leu Asp Glu Leu
                165                 170                 175
Ile Phe Ala Arg Lys Asp Thr Phe Phe Lys Asp Val Asp Tyr Val Cys
                180                 185                 190
Ile Ser Asp Asn Tyr Trp Leu Gly Lys Lys Lys Pro Cys Ile Thr Tyr
        195                 200                 205
Gly Leu Arg Gly Ile Cys Tyr Phe Phe Ile Glu Val Glu Cys Ser Asn
    210                 215                 220
Lys Asp Leu His Ser Gly Val Tyr Gly Gly Ser Val His Glu Ala Met
225                 230                 235                 240
Thr Asp Leu Ile Leu Leu Met Gly Ser Leu Val Asp Lys Arg Gly Asn
                245                 250                 255
Ile Leu Ile Pro Gly Ile Asn Glu Ala Val Ala Ala Val Thr Glu Glu
                260                 265                 270
Glu His Lys Leu Tyr Asp Asp Ile Asp Phe Asp Ile Glu Glu Phe Ala
        275                 280                 285

Lys Asp Val Gly Ala Gln Ile Leu Leu His Ser His Lys Lys Asp Ile
    290                 295                 300
Leu Met His Arg Trp Arg Tyr Pro Ser Leu Ser Leu His Gly Ile Glu
305                 310                 315                 320
Gly Ala Phe Ser Gly Ser Gly Ala Lys Thr Val Ile Pro Arg Lys Val
                325                 330                 335
Val Gly Lys Phe Ser Ile Arg Leu Val Pro Asn Met Thr Pro Glu Val
            340                 345                 350
Val Gly Glu Gln Val Thr Ser Tyr Leu Thr Lys Lys Phe Ala Glu Leu
        355                 360                 365
Arg Ser Pro Asn Glu Phe Lys Val Tyr Met Gly His Gly Gly Lys Pro
    370                 375                 380
Trp Val Ser Asp Phe Ser His Pro His Tyr Leu Ala Gly Arg Arg Ala
385                 390                 395                 400
Met Lys Thr Val Phe Gly Val Glu Pro Asp Leu Thr Arg Glu Gly Gly
                405                 410                 415
Ser Ile Pro Val Thr Leu Thr Phe Gln Glu Ala Thr Gly Lys Asn Val
            420                 425                 430
Met Leu Leu Pro Val Gly Ser Ala Asp Asp Gly Ala His Ser Gln Asn
        435                 440                 445
Glu Lys Leu Asn Arg Tyr Asn Tyr Ile Glu Gly Thr Lys Met Leu Ala
    450                 455                 460
Ala Tyr Leu Tyr Glu Val Ser Gln Leu Lys Asp
465                 470                 475

<210> 273
<211> 391
<212> PRT
<213> Homo sapiens

<400> 273
Met Ala Ala Leu Thr Thr Leu Phe Lys Tyr Ile Asp Glu Asn Gln Asp
 1               5                  10                  15
Arg Tyr Ile Lys Lys Leu Ala Lys Trp Val Ala Ile Gln Ser Val Ser
            20                  25                  30
Ala Trp Pro Glu Lys Arg Gly Glu Ile Arg Arg Met Met Glu Val Ala
        35                  40                  45
Ala Ala Asp Val Lys Gln Leu Gly Gly Ser Val Glu Leu Val Asp Ile
    50                  55                  60
Gly Lys Gln Lys Glu Ile Pro Val Asn Val Arg Phe Cys Leu Glu Gly
65                  70                  75                  80
Met Glu Glu Ser Gly Ser Glu Gly Leu Asp Glu Leu Ile Phe Ala Arg
                85                  90                  95
Lys Asp Thr Phe Phe Lys Asp Val Asp Tyr Val Cys Ile Ser Asp Asn
            100                 105                 110
Tyr Trp Leu Gly Lys Lys Lys Pro Cys Ile Thr Tyr Gly Leu Arg Gly
        115                 120                 125
Ile Cys Tyr Phe Phe Ile Glu Val Glu Cys Ser Asn Lys Asp Leu His
    130                 135                 140
Ser Gly Val Tyr Gly Gly Ser Val His Glu Ala Met Thr Asp Leu Ile
145                 150                 155                 160
Leu Leu Met Gly Ser Leu Val Asp Lys Arg Gly Asn Ile Leu Ile Pro
                165                 170                 175
Gly Ile Asn Glu Ala Val Ala Ala Val Thr Glu Glu Glu His Lys Leu
            180                 185                 190
Tyr Asp Asp Ile Asp Phe Asp Ile Glu Glu Phe Ala Lys Asp Val Gly
        195                 200                 205
Ala Gln Ile Leu Leu His Ser His Lys Lys Asp Ile Leu Met His Arg
    210                 215                 220
Trp Arg Tyr Pro Ser Leu Ser Leu His Gly Ile Glu Gly Ala Phe Ser
225                 230                 235                 240
Gly Ser Gly Ala Lys Thr Val Ile Pro Arg Lys Val Val Gly Lys Phe
                245                 250                 255

Ser Ile Arg Leu Val Pro Asn Met Thr Pro Glu Val Val Gly Glu Gln
                260                 265                 270
Val Thr Ser Tyr Leu Thr Lys Lys Phe Ala Glu Leu Arg Ser Pro Asn
        275                 280                 285
Glu Phe Lys Val Tyr Met Gly His Gly Gly Lys Pro Trp Val Ser Asp
    290                 295                 300
Phe Ser His Pro His Tyr Leu Ala Gly Arg Arg Ala Met Lys Thr Val
305                 310                 315                 320
Phe Gly Val Glu Pro Asp Leu Thr Arg Glu Gly Gly Ser Ile Pro Val
                325                 330                 335
Thr Leu Thr Phe Gln Glu Ala Thr Gly Lys Asn Val Met Leu Leu Pro
            340                 345                 350
Val Gly Ser Ala Asp Asp Gly Ala His Ser Gln Asn Glu Lys Leu Asn
        355                 360                 365
Arg Tyr Asn Tyr Ile Glu Gly Thr Lys Met Leu Ala Ala Tyr Leu Tyr
    370                 375                 380
Glu Val Ser Gln Leu Lys Asp
385                 390

<210> 274
<211> 414
<212> PRT
<213> Homo sapiens

<400> 274
Met Gly Gly Asn His Ser His Lys Pro Pro Val Phe Asp Glu Asn Glu
1               5                   10                  15
Glu Val Asn Phe Asp His Phe Gln Ile Leu Arg Ala Ile Gly Lys Gly
            20                  25                  30
Ser Phe Gly Lys Val Cys Ile Val Gln Lys Arg Asp Thr Lys Lys Met
        35                  40                  45
Tyr Ala Met Lys Tyr Met Asn Lys Gln Lys Cys Ile Glu Arg Asp Glu
    50                  55                  60
Val Arg Asn Val Phe Arg Glu Leu Gln Ile Met Gln Gly Leu Glu His
65                  70                  75                  80
Pro Phe Leu Val Asn Leu Trp Tyr Ser Phe Gln Asp Glu Glu Asp Met
                85                  90                  95
Phe Met Val Val Asp Leu Leu Leu Gly Gly Asp Leu Arg Tyr His Leu
            100                 105                 110
Gln Gln Asn Val His Phe Thr Glu Gly Thr Val Lys Leu Tyr Ile Cys
        115                 120                 125
Glu Leu Ala Leu Ala Leu Glu Tyr Leu Gln Arg Tyr His Ile Ile His
    130                 135                 140
Arg Asp Ile Lys Pro Asp Asn Ile Leu Leu Asp Glu His Gly His Val
145                 150                 155                 160
His Ile Thr Asp Phe Asn Ile Ala Thr Val Val Lys Gly Ala Glu Arg
                165                 170                 175
Ala Ser Ser Met Ala Gly Thr Lys Pro Tyr Met Ala Pro Glu Val Phe
            180                 185                 190
Gln Val Tyr Met Asp Arg Gly Pro Gly Tyr Ser Tyr Pro Val Asp Trp
        195                 200                 205
Trp Ser Leu Gly Ile Thr Ala Tyr Glu Leu Leu Arg Gly Trp Arg Pro
    210                 215                 220
Tyr Glu Ile His Ser Val Thr Pro Ile Asp Glu Ile Leu Asn Met Phe
225                 230                 235                 240
Lys Val Glu Arg Val His Tyr Ser Ser Thr Trp Cys Lys Gly Met Val
                245                 250                 255
Ala Leu Leu Arg Lys Leu Leu Thr Lys Asp Pro Glu Ser Arg Val Ser
            260                 265                 270
Ser Leu His Asp Ile Gln Ser Val Pro Tyr Leu Ala Asp Met Asn Trp
        275                 280                 285
Asp Ala Val Phe Lys Lys Ala Leu Met Pro Gly Phe Val Pro Asn Lys
    290                 295                 300

Gly Arg Leu Asn Cys Asp Pro Thr Phe Glu Leu Glu Glu Met Ile Leu
305                 310                 315                 320
Glu Ser Lys Pro Leu His Lys Lys Lys Lys Arg Leu Ala Lys Asn Arg
                325                 330                 335
Ser Arg Asp Gly Thr Lys Asp Ser Cys Pro Leu Asn Gly His Leu Gln
            340                 345                 350
His Cys Leu Glu Thr Val Arg Glu Glu Phe Ile Ile Phe Asn Arg Glu
        355                 360                 365
Lys Leu Arg Arg Gln Gln Gly Gln Gly Ser Gln Leu Leu Asp Thr Asp
    370                 375                 380
Ser Arg Gly Gly Gly Gln Ala Gln Ser Lys Leu Gln Asp Gly Cys Asn
385                 390                 395                 400
Asn Asn Leu Leu Thr His Thr Cys Thr Arg Gly Cys Ser Ser
                405                 410

<210> 275
<211> 465
<212> PRT
<213> Homo sapiens

<400> 275
Met Gly Gly Ala Val Val Asp Glu Gly Pro Thr Gly Val Lys Ala Pro
 1               5                  10                  15
Asp Gly Gly Trp Gly Trp Ala Val Leu Phe Gly Cys Phe Val Ile Thr
            20                  25                  30
Gly Phe Ser Tyr Ala Phe Pro Lys Ala Val Ser Val Phe Phe Lys Glu
        35                  40                  45
Leu Ile Gln Glu Phe Gly Ile Gly Tyr Ser Asp Thr Ala Trp Ile Ser
    50                  55                  60
Ser Ile Leu Leu Ala Met Leu Tyr Gly Thr Gly Pro Leu Cys Ser Val
65                  70                  75                  80
Cys Val Asn Arg Phe Gly Cys Arg Pro Val Met Leu Val Gly Gly Leu
                85                  90                  95
Phe Ala Ser Leu Gly Met Val Ala Ala Ser Phe Cys Arg Ser Ile Ile
            100                 105                 110
Gln Val Tyr Leu Thr Thr Gly Val Ile Thr Gly Leu Gly Leu Ala Leu
        115                 120                 125
Asn Phe Gln Pro Ser Leu Ile Met Leu Asn Arg Tyr Phe Ser Lys Arg
    130                 135                 140
Arg Pro Met Ala Asn Gly Leu Ala Ala Ala Gly Ser Pro Val Phe Leu
145                 150                 155                 160
Cys Ala Leu Ser Pro Leu Gly Gln Leu Leu Gln Asp Arg Tyr Gly Trp
                165                 170                 175
Arg Gly Gly Phe Leu Ile Leu Gly Gly Leu Leu Leu Asn Cys Cys Val
            180                 185                 190
Cys Ala Ala Leu Met Arg Pro Leu Val Val Thr Ala Gln Pro Gly Ser
        195                 200                 205
Gly Pro Pro Arg Pro Ser Arg Arg Leu Leu Asp Leu Ser Val Phe Arg
    210                 215                 220
Asp Arg Gly Phe Val Leu Tyr Ala Val Ala Ala Ser Val Met Val Leu
225                 230                 235                 240
Gly Leu Phe Val Pro Pro Val Phe Val Val Ser Tyr Ala Lys Asp Leu
                245                 250                 255
Gly Val Pro Asp Thr Lys Ala Ala Phe Leu Leu Thr Ile Leu Gly Phe
            260                 265                 270
Ile Asp Ile Phe Ala Arg Pro Ala Ala Gly Phe Val Ala Gly Leu Gly
        275                 280                 285
Lys Val Arg Pro Tyr Ser Val Tyr Leu Phe Ser Phe Ser Met Phe Phe
    290                 295                 300
Asn Gly Leu Ala Asp Leu Ala Gly Ser Thr Ala Gly Asp Tyr Gly Gly
305                 310                 315                 320
Leu Val Val Phe Cys Ile Phe Phe Gly Ile Ser Tyr Gly Met Val Gly
                325                 330                 335

Ala Leu Gln Phe Glu Val Leu Met Ala Ile Val Gly Thr His Lys Phe
            340                 345                 350
Ser Ser Ala Ile Gly Leu Val Leu Leu Met Glu Ala Val Ala Val Leu
        355                 360                 365
Val Gly Pro Pro Ser Gly Gly Lys Leu Leu Asp Ala Thr His Val Tyr
    370                 375                 380
Met Tyr Val Phe Ile Leu Ala Gly Ala Glu Val Leu Thr Ser Ser Leu
385                 390                 395                 400
Ile Leu Leu Leu Gly Asn Phe Phe Cys Ile Arg Lys Lys Pro Lys Glu
                405                 410                 415
Pro Gln Pro Glu Val Ala Ala Ala Glu Glu Glu Lys Leu His Lys Pro
            420                 425                 430
Pro Ala Asp Ser Gly Val Asp Leu Arg Glu Val Glu His Phe Leu Lys
        435                 440                 445
Ala Glu Pro Glu Lys Asn Gly Glu Val Val His Thr Pro Glu Thr Ser
    450                 455                 460
Val
465

<210> 276
<211> 1083
<212> PRT
<213> Homo sapiens

<400> 276
Met Pro Thr Asn Phe Thr Val Val Pro Val Glu Ala His Ala Asp Gly
1               5                   10                  15
Gly Gly Asp Glu Thr Ala Glu Arg Thr Glu Ala Pro Gly Thr Pro Glu
            20                  25                  30
Gly Pro Glu Pro Glu Arg Pro Ser Pro Gly Asp Gly Asn Pro Arg Glu
        35                  40                  45
Asn Ser Pro Phe Leu Asn Asn Val Glu Val Glu Gln Glu Ser Phe Phe
    50                  55                  60
Glu Gly Lys Asn Met Ala Leu Phe Glu Glu Glu Met Asp Ser Asn Pro
65                  70                  75                  80
Met Val Ser Ser Leu Leu Asn Lys Leu Ala Asn Tyr Thr Asn Leu Ser
                85                  90                  95
Gln Gly Val Val Glu His Glu Glu Asp Glu Glu Ser Arg Arg Arg Glu
            100                 105                 110
Ala Lys Ala Pro Arg Met Gly Thr Phe Ile Gly Val Tyr Leu Pro Cys
        115                 120                 125
Leu Gln Asn Ile Leu Gly Val Ile Leu Phe Leu Arg Leu Thr Trp Ile
    130                 135                 140
Val Gly Val Ala Gly Val Leu Glu Ser Phe Leu Ile Val Ala Met Cys
145                 150                 155                 160
Cys Thr Cys Thr Met Leu Thr Ala Ile Ser Met Ser Ala Ile Ala Thr
                165                 170                 175
Asn Gly Val Val Pro Ala Gly Gly Ser Tyr Tyr Met Ile Ser Arg Ser
            180                 185                 190
Leu Gly Pro Glu Phe Gly Gly Ala Val Gly Leu Cys Phe Tyr Leu Gly
        195                 200                 205
Thr Thr Phe Ala Gly Ala Met Tyr Ile Leu Gly Thr Ile Glu Ile Phe
    210                 215                 220
Leu Thr Tyr Ile Ser Pro Gly Ala Ala Ile Phe Gln Ala Glu Ala Ala
225                 230                 235                 240
Gly Gly Glu Ala Ala Ala Met Leu His Asn Met Arg Val Tyr Gly Thr
                245                 250                 255
Cys Thr Leu Val Leu Met Ala Leu Val Val Phe Val Gly Val Lys Tyr
            260                 265                 270
Val Asn Lys Leu Ala Leu Val Phe Leu Ala Cys Val Val Leu Ser Ile
        275                 280                 285
Leu Ala Ile Tyr Ala Gly Val Ile Lys Ser Ala Phe Asp Pro Pro Asp
    290                 295                 300

Ile Pro Val Cys Leu Leu Gly Asn Arg Thr Leu Ser Arg Arg Ser Phe
305                 310                 315                 320
Asp Ala Cys Val Lys Ala Tyr Gly Ile His Asn Asn Ser Ala Thr Ser
                325                 330                 335
Ala Leu Trp Gly Leu Phe Cys Asn Gly Ser Gln Pro Ser Ala Ala Cys
            340                 345                 350
Asp Glu Tyr Phe Ile Gln Asn Asn Val Thr Glu Ile Gln Gly Ile Pro
        355                 360                 365
Gly Ala Ala Ser Gly Val Phe Leu Glu Asn Leu Trp Ser Thr Tyr Ala
    370                 375                 380
His Ala Gly Ala Phe Val Glu Lys Lys Gly Val Pro Ser Val Pro Val
385                 390                 395                 400
Ala Glu Glu Ser Arg Ala Ser Thr Leu Pro Tyr Val Leu Thr Asp Ile
                405                 410                 415
Ala Ala Ser Phe Thr Leu Leu Val Gly Ile Tyr Phe Pro Ser Val Thr
            420                 425                 430
Gly Ile Met Ala Gly Ser Asn Arg Ser Gly Asp Leu Lys Asp Ala Gln
        435                 440                 445
Lys Ser Ile Pro Thr Gly Thr Ile Leu Ala Ile Val Thr Thr Ser Phe
    450                 455                 460
Ile Tyr Leu Ser Cys Ile Val Leu Phe Gly Ala Cys Ile Glu Gly Val
465                 470                 475                 480
Val Leu Arg Asp Lys Phe Gly Glu Ala Leu Gln Gly Asn Leu Val Ile
                485                 490                 495
Gly Met Leu Ala Trp Pro Ser Pro Trp Val Ile Val Ile Gly Ser Phe
            500                 505                 510
Phe Ser Thr Cys Gly Ala Gly Leu Gln Thr Leu Thr Gly Ala Pro Arg
        515                 520                 525
Leu Leu Gln Ala Ile Ala Arg Asp Gly Ile Val Pro Phe Leu Gln Val
    530                 535                 540
Phe Gly His Gly Lys Ala Asn Gly Glu Pro Thr Trp Ala Leu Leu Leu
545                 550                 555                 560
Thr Val Leu Ile Cys Glu Thr Gly Ile Leu Ile Ala Ser Leu Asp Ser
                565                 570                 575
Val Ala Pro Ile Leu Ser Met Phe Phe Leu Met Cys Tyr Leu Phe Val
            580                 585                 590
Asn Leu Ala Cys Ala Val Gln Thr Leu Leu Arg Thr Pro Asn Trp Arg
        595                 600                 605
Pro Arg Phe Lys Phe Tyr His Trp Thr Leu Ser Phe Leu Gly Met Ser
    610                 615                 620
Leu Cys Leu Ala Leu Met Phe Ile Cys Ser Trp Tyr Tyr Ala Leu Ser
625                 630                 635                 640
Ala Met Leu Ile Ala Gly Cys Ile Tyr Lys Tyr Ile Glu Tyr Arg Gly
                645                 650                 655
Ala Glu Lys Glu Trp Gly Asp Gly Ile Arg Gly Leu Ser Leu Asn Ala
            660                 665                 670
Ala Arg Tyr Ala Leu Leu Arg Val Glu His Gly Pro Pro His Thr Lys
        675                 680                 685
Asn Trp Arg Pro Gln Val Leu Val Met Leu Asn Leu Asp Ala Glu Gln
    690                 695                 700
Ala Val Lys His Pro Arg Leu Leu Ser Phe Thr Ser Gln Leu Lys Ala
705                 710                 715                 720

Gly Lys Gly Leu Thr Ile Val Gly Ser Val Leu Glu Gly Thr Tyr Leu
                725                 730                 735
Asp Lys His Met Glu Ala Gln Arg Ala Glu Glu Asn Ile Arg Ser Leu
            740                 745                 750
Met Ser Thr Glu Lys Thr Lys Gly Phe Cys Gln Leu Val Val Ser Ser
        755                 760                 765
Ser Leu Arg Asp Gly Met Ser His Leu Ile Gln Ser Ala Gly Leu Gly
    770                 775                 780
Gly Leu Lys His Asn Thr Val Leu Met Ala Trp Pro Ala Ser Trp Lys
785                 790                 795                 800

Gln Glu Asp Asn Pro Phe Ser Trp Lys Asn Phe Val Asp Thr Val Arg
                805                 810                 815
Asp Thr Thr Ala Ala His Gln Ala Leu Leu Val Ala Lys Asn Val Asp
            820                 825                 830
Ser Phe Pro Gln Asn Gln Glu Arg Phe Gly Gly Gly His Ile Asp Val
        835                 840                 845
Trp Trp Ile Val His Asp Gly Gly Met Leu Met Leu Leu Pro Phe Leu
    850                 855                 860
Leu Arg Gln His Lys Val Trp Arg Lys Cys Arg Met Arg Ile Phe Thr
865                 870                 875                 880
Val Ala Gln Val Asp Asp Asn Ser Ile Gln Met Lys Lys Asp Leu Gln
                885                 890                 895
Met Phe Leu Tyr His Leu Arg Ile Ser Ala Glu Val Glu Val Val Glu
            900                 905                 910
Met Val Glu Asn Asp Ile Ser Ala Phe Thr Tyr Glu Arg Thr Leu Met
        915                 920                 925
Met Glu Gln Arg Ser Gln Met Leu Lys Gln Met Gln Leu Ser Lys Asn
    930                 935                 940
Glu Gln Glu Arg Glu Ala Gln Leu Ile His Asp Arg Asn Thr Ala Ser
945                 950                 955                 960
His Thr Ala Ala Ala Ala Arg Thr Gln Ala Pro Pro Thr Pro Asp Lys
                965                 970                 975
Val Gln Met Thr Trp Thr Arg Glu Lys Leu Ile Ala Glu Lys Tyr Arg
            980                 985                 990
Ser Arg Asp Thr Ser Leu Ser Gly Phe Lys Asp Leu Phe Ser Met Lys
        995                 1000                1005
Pro Asp Gln Ser Asn Val Arg Arg Met His Thr Ala Val Lys Leu Asn
    1010                1015                1020
Gly Val Val Leu Asn Lys Ser Gln Asp Ala Gln Leu Val Leu Leu Asn
1025                1030                1035                1040
Met Pro Gly Pro Pro Lys Asn Arg Gln Gly Asp Glu Asn Tyr Met Glu
                1045                1050                1055
Phe Leu Glu Val Leu Thr Glu Gly Leu Asn Arg Val Leu Leu Val Arg
            1060                1065                1070
Gly Gly Gly Arg Glu Val Ile Thr Ile Tyr Ser
        1075                1080

<210> 277
<211> 164
<212> PRT
<213> Homo sapiens

<400> 277
Met Ala Ser Pro His Gln Glu Pro Lys Pro Gly Asp Leu Ile Glu Ile
 1               5                  10                  15
Phe Arg Leu Gly Tyr Glu His Trp Ala Leu Tyr Ile Gly Asp Gly Tyr
            20                  25                  30
Val Ile His Leu Ala Pro Pro Ser Glu Tyr Pro Gly Ala Gly Ser Ser
        35                  40                  45
Ser Val Phe Ser Val Leu Ser Asn Ser Ala Glu Val Lys Arg Gly Arg
    50                  55                  60
Leu Glu Asp Val Val Gly Gly Cys Cys Tyr Arg Val Asn Asn Ser Leu
65                  70                  75                  80
Asp His Glu Tyr Gln Pro Arg Pro Val Glu Val Ile Ile Ser Ser Ala
                85                  90                  95
Lys Glu Met Val Gly Gln Lys Met Lys Tyr Ser Ile Val Ser Arg Asn
            100                 105                 110
Cys Glu His Phe Val Ala Gln Leu Arg Tyr Gly Lys Ser Arg Cys Lys
        115                 120                 125
Gln Val Glu Lys Ala Lys Val Glu Val Gly Val Ala Thr Ala Leu Gly
    130                 135                 140
Ile Leu Val Val Ala Gly Cys Ser Phe Ala Ile Arg Arg Tyr Gln Lys
145                 150                 155                 160

Lys Ala Thr Ala

<210> 278
<211> 487
<212> PRT
<213> Homo sapiens

<400> 278
Met Leu Lys Arg Glu Gly Lys Val Gln Pro Tyr Thr Lys Thr Leu Asp
1               5                   10                  15
Gly Gly Trp Gly Trp Met Ile Val Ile His Phe Phe Leu Val Asn Val
            20                  25                  30
Phe Val Met Gly Met Thr Lys Thr Phe Ala Ile Phe Phe Val Val Phe
        35                  40                  45
Gln Glu Glu Phe Glu Gly Thr Ser Glu Gln Ile Gly Trp Ile Gly Ser
    50                  55                  60
Ile Met Ser Ser Leu Arg Phe Cys Ala Gly Pro Leu Val Ala Ile Ile
65                  70                  75                  80
Cys Asp Ile Leu Gly Glu Lys Thr Thr Ser Ile Leu Gly Ala Phe Val
                85                  90                  95
Val Thr Gly Gly Tyr Leu Ile Ser Ser Trp Ala Thr Ser Ile Pro Phe
            100                 105                 110
Leu Cys Val Thr Met Gly Leu Leu Pro Gly Leu Gly Ser Ala Phe Leu
        115                 120                 125
Tyr Gln Val Ala Ala Val Val Thr Thr Lys Tyr Phe Lys Lys Arg Leu
    130                 135                 140
Ala Leu Ser Thr Ala Ile Ala Arg Ser Gly Met Gly Leu Thr Phe Leu
145                 150                 155                 160
Leu Ala Pro Phe Thr Lys Phe Leu Ile Asp Leu Tyr Asp Trp Thr Gly
                165                 170                 175
Ala Leu Ile Leu Phe Gly Ala Ile Ala Leu Asn Leu Val Pro Ser Ser
            180                 185                 190
Met Leu Leu Arg Pro Ile His Ile Lys Ser Glu Asn Asn Ser Gly Ile
        195                 200                 205
Lys Asp Lys Gly Ser Ser Leu Ser Ala His Gly Pro Glu Ala His Ala
    210                 215                 220
Thr Glu Thr His Cys His Glu Thr Glu Glu Ser Thr Ile Lys Asp Ser
225                 230                 235                 240
Thr Thr Gln Lys Ala Gly Leu Pro Ser Lys Asn Leu Thr Val Ser Gln
                245                 250                 255
Asn Gln Ser Glu Glu Phe Tyr Asn Gly Pro Asn Arg Asn Arg Leu Leu
            260                 265                 270
Leu Lys Ser Asp Glu Glu Ser Asp Lys Val Ile Ser Trp Ser Cys Lys
        275                 280                 285
Gln Leu Phe Asp Ile Ser Leu Phe Arg Asn Pro Phe Phe Tyr Ile Phe
    290                 295                 300
Thr Trp Ser Phe Leu Leu Ser Gln Leu Ala Tyr Phe Ile Pro Thr Phe
305                 310                 315                 320

His Leu Val Ala Arg Ala Lys Thr Leu Gly Ile Asp Ile Met Asp Ala
                325                 330                 335
Ser Tyr Leu Val Ser Val Ala Gly Ile Leu Glu Thr Val Ser Gln Ile
            340                 345                 350
Ile Ser Gly Trp Val Ala Asp Gln Asn Trp Ile Lys Lys Tyr His Tyr
        355                 360                 365
His Lys Ser Tyr Leu Ile Leu Cys Gly Ile Thr Asn Leu Leu Ala Pro
    370                 375                 380
Leu Ala Thr Thr Phe Pro Leu Leu Met Thr Tyr Thr Ile Cys Phe Ala
385                 390                 395                 400
Ile Phe Ala Gly Gly Tyr Leu Ala Leu Ile Leu Pro Val Leu Val Asp
                405                 410                 415
Leu Cys Arg Asn Ser Thr Val Asn Arg Phe Leu Gly Leu Ala Ser Phe
            420                 425                 430

Phe Ala Gly Met Ala Val Leu Ser Gly Pro Pro Ile Ala Gly Trp Leu
        435                 440                 445
Tyr Asp Tyr Thr Gln Thr Tyr Asn Gly Ser Phe Tyr Phe Ser Gly Ile
    450                 455                 460
Cys Tyr Leu Leu Ser Ser Val Ser Phe Phe Phe Val Pro Leu Ala Glu
465                 470                 475                 480
Arg Trp Lys Asn Ser Leu Thr
                485

<210> 279
<211> 683
<212> PRT
<213> Homo sapiens

<400> 279

<210> 280
<211> 577
<212> PRT
<213> Homo sapiens

<400> 280
Met Glu Gly Ser Gly Gly Gly Ala Gly Glu Arg Ala Pro Leu Leu Gly
1               5                   10                  15
Ala Arg Arg Ala Ala Ala Ala Ala Ala Ala Ala Gly Ala Phe Ala Gly
            20                  25                  30
Arg Arg Ala Ala Cys Gly Ala Val Leu Leu Thr Glu Leu Leu Glu Arg
        35                  40                  45
Ala Ala Phe Tyr Gly Ile Thr Ser Asn Leu Val Leu Phe Leu Asn Gly
    50                  55                  60
Ala Pro Phe Cys Trp Glu Gly Ala Gln Ala Ser Glu Ala Leu Leu Leu
65                  70                  75                  80
Phe Met Gly Leu Thr Tyr Leu Gly Ser Pro Phe Gly Gly Trp Leu Ala
                85                  90                  95
Asp Ala Arg Leu Gly Arg Ala Arg Ala Ile Leu Leu Ser Leu Ala Leu
            100                 105                 110
Tyr Leu Leu Gly Met Leu Ala Phe Pro Leu Leu Ala Ala Pro Ala Thr
        115                 120                 125
Arg Ala Ala Leu Cys Gly Ser Ala Arg Leu Leu Asn Cys Thr Ala Pro

130                 135                 140
Gly Pro Asp Ala Ala Ala Arg Cys Cys Ser Pro Ala Thr Phe Ala Gly
145                 150                 155                 160
Leu Val Leu Val Gly Leu Gly Val Ala Thr Val Lys Ala Asn Ile Thr
                165                 170                 175
Pro Phe Gly Ala Asp Gln Val Lys Asp Arg Gly Pro Glu Ala Thr Arg
            180                 185                 190
Arg Phe Phe Asn Trp Phe Tyr Trp Ser Ile Asn Leu Gly Ala Ile Leu
        195                 200                 205
Ser Leu Gly Gly Ile Ala Tyr Ile Gln Gln Asn Val Ser Phe Val Thr
    210                 215                 220
Gly Tyr Ala Ile Pro Thr Val Cys Val Gly Leu Ala Phe Val Val Phe
225                 230                 235                 240
Leu Cys Gly Gln Ser Val Phe Ile Thr Lys Pro Pro Asp Gly Ser Ala
                245                 250                 255
Phe Thr Asp Met Phe Lys Ile Leu Thr Tyr Ser Cys Cys Ser Gln Lys
            260                 265                 270
Arg Ser Gly Glu Arg Gln Ser Asn Gly Glu Gly Ile Gly Val Phe Gln
        275                 280                 285
Gln Ser Ser Lys Gln Ser Leu Phe Asp Ser Cys Lys Met Ser His Gly
    290                 295                 300
Gly Pro Phe Thr Glu Glu Lys Val Glu Asp Val Lys Ala Leu Val Lys
305                 310                 315                 320
Ile Val Pro Val Phe Leu Ala Leu Ile Pro Tyr Trp Thr Val Tyr Phe
                325                 330                 335
Gln Met Gln Thr Thr Tyr Val Leu Gln Ser Leu His Leu Arg Ile Pro
            340                 345                 350
Glu Ile Ser Asn Ile Thr Thr Thr Pro His Thr Leu Pro Ala Ala Trp
        355                 360                 365
Leu Thr Met Phe Asp Ala Val Leu Ile Leu Leu Leu Ile Pro Leu Lys
    370                 375                 380
Asp Lys Leu Val Asp Pro Ile Leu Arg Arg His Gly Leu Leu Pro Ser
385                 390                 395                 400
Ser Leu Lys Arg Ile Ala Val Gly Met Phe Phe Val Met Cys Ser Ala
                405                 410                 415
Phe Ala Ala Gly Ile Leu Glu Ser Lys Arg Leu Asn Leu Val Lys Glu
            420                 425                 430
Lys Thr Ile Asn Gln Thr Ile Gly Asn Val Val Tyr His Ala Ala Asp
        435                 440                 445
Leu Ser Leu Trp Trp Gln Val Pro Gln Tyr Leu Leu Ile Gly Ile Ser
    450                 455                 460
Glu Ile Phe Ala Ser Ile Ala Gly Leu Glu Phe Ala Tyr Ser Ala Ala
465                 470                 475                 480
Pro Lys Ser Met Gln Ser Ala Ile Met Gly Leu Phe Phe Phe Phe Ser
                485                 490                 495
Gly Val Gly Ser Phe Val Gly Ser Gly Leu Leu Ala Leu Val Ser Ile
            500                 505                 510
Lys Ala Ile Gly Trp Met Ser Ser His Thr Asp Phe Gly Asn Ile Asn
        515                 520                 525
Gly Cys Tyr Leu Asn Tyr Tyr Phe Phe Leu Leu Ala Ala Ile Gln Gly
    530                 535                 540
Ala Thr Leu Leu Leu Phe Leu Ile Ile Ser Val Lys Tyr Asp His His
545                 550                 555                 560
Arg Asp His Gln Arg Ser Arg Ala Asn Gly Val Pro Thr Ser Arg Arg
                565                 570                 575
Ala

<210> 281
<211> 277
<212> PRT
<213> Homo sapiens

<400> 281

Met Val Arg Leu Pro Leu Gln Cys Val Leu Trp Gly Cys Leu Leu Thr
1               5                   10                  15
Ala Val His Pro Glu Pro Pro Thr Ala Cys Arg Glu Lys Gln Tyr Leu
            20                  25                  30
Ile Asn Ser Gln Cys Cys Ser Leu Cys Gln Pro Gly Gln Lys Leu Val
        35                  40                  45
Ser Asp Cys Thr Glu Phe Thr Glu Thr Glu Cys Leu Pro Cys Gly Glu
    50                  55                  60
Ser Glu Phe Leu Asp Thr Trp Asn Arg Glu Thr His Cys His Gln His
65                  70                  75                  80
Lys Tyr Cys Asp Pro Asn Leu Gly Leu Arg Val Gln Gln Lys Gly Thr
                85                  90                  95
Ser Glu Thr Asp Thr Ile Cys Thr Cys Glu Glu Gly Trp His Cys Thr
            100                 105                 110
Ser Glu Ala Cys Glu Ser Cys Val Leu His Arg Ser Cys Ser Pro Gly
        115                 120                 125
Phe Gly Val Lys Gln Ile Ala Thr Gly Val Ser Asp Thr Ile Cys Glu
    130                 135                 140
Pro Cys Pro Val Gly Phe Phe Ser Asn Val Ser Ser Ala Phe Glu Lys
145                 150                 155                 160
Cys His Pro Trp Thr Ser Cys Glu Thr Lys Asp Leu Val Val Gln Gln
                165                 170                 175
Ala Gly Thr Asn Lys Thr Asp Val Val Cys Gly Pro Gln Asp Arg Leu
            180                 185                 190
Arg Ala Leu Val Val Ile Pro Ile Ile Phe Gly Ile Leu Phe Ala Ile
        195                 200                 205
Leu Leu Val Leu Val Phe Ile Lys Lys Val Ala Lys Lys Pro Thr Asn
    210                 215                 220
Lys Ala Pro His Pro Lys Gln Glu Pro Gln Glu Ile Asn Phe Pro Asp
225                 230                 235                 240
Asp Leu Pro Gly Ser Asn Thr Ala Ala Pro Val Gln Glu Thr Leu His
                245                 250                 255
Gly Cys Gln Pro Val Thr Gln Glu Asp Gly Lys Glu Ser Arg Ile Ser
            260                 265                 270
Val Gln Glu Arg Gln
        275

<210> 282
<211> 440
<212> PRT
<213> Homo sapiens
<400> 282
Met Glu Gln Arg Gly Gln Asn Ala Pro Ala Ala Ser Gly Ala Arg Lys
1               5                   10                  15
Arg His Gly Pro Gly Pro Arg Glu Ala Arg Gly Ala Arg Pro Gly Leu
            20                  25                  30
Arg Val Pro Lys Thr Leu Val Leu Val Val Ala Ala Val Leu Leu Leu
        35                  40                  45
Val Ser Ala Glu Ser Ala Leu Ile Thr Gln Gln Asp Leu Ala Pro Gln
    50                  55                  60
Gln Arg Ala Ala Pro Gln Gln Lys Arg Ser Ser Pro Ser Glu Gly Leu
65                  70                  75                  80
Cys Pro Pro Gly His His Ile Ser Glu Asp Gly Arg Asp Cys Ile Ser
                85                  90                  95
Cys Lys Tyr Gly Gln Asp Tyr Ser Thr His Trp Asn Asp Leu Leu Phe
            100                 105                 110
Cys Leu Arg Cys Thr Arg Cys Asp Ser Gly Glu Val Glu Leu Ser Pro
        115                 120                 125
Cys Thr Thr Thr Arg Asn Thr Val Cys Gln Cys Glu Glu Gly Thr Phe
    130                 135                 140
Arg Glu Glu Asp Ser Pro Glu Met Cys Arg Lys Cys Arg Thr Gly Cys
145                 150                 155                 160
Pro Arg Gly Met Val Lys Val Gly Asp Cys Thr Pro Trp Ser Asp Ile

165                 170                 175
Glu Cys Val His Lys Glu Ser Gly Thr Lys His Ser Gly Glu Ala Pro
            180                 185                 190
Ala Val Glu Glu Thr Val Thr Ser Ser Pro Gly Thr Pro Ala Ser Pro
        195                 200                 205
Cys Ser Leu Ser Gly Ile Ile Ile Gly Val Thr Val Ala Ala Val Val
    210                 215                 220
Leu Ile Val Ala Val Phe Val Cys Lys Ser Leu Leu Trp Lys Lys Val
225                 230                 235                 240
Leu Pro Tyr Leu Lys Gly Ile Cys Ser Gly Gly Gly Gly Asp Pro Glu
                245                 250                 255
Arg Val Asp Arg Ser Ser Gln Arg Pro Gly Ala Glu Asp Asn Val Leu
            260                 265                 270
Asn Glu Ile Val Ser Ile Leu Gln Pro Thr Gln Val Pro Glu Gln Glu
        275                 280                 285
Met Glu Val Gln Glu Pro Ala Glu Pro Thr Gly Val Asn Met Leu Ser
    290                 295                 300
Pro Gly Glu Ser Glu His Leu Leu Glu Pro Ala Glu Ala Glu Arg Ser
305                 310                 315                 320
Gln Arg Arg Arg Leu Leu Val Pro Ala Asn Glu Gly Asp Pro Thr Glu
                325                 330                 335
Thr Leu Arg Gln Cys Phe Asp Asp Phe Ala Asp Leu Val Pro Phe Asp
            340                 345                 350
Ser Trp Glu Pro Leu Met Arg Lys Leu Gly Leu Met Asp Asn Glu Ile
        355                 360                 365
Lys Val Ala Lys Ala Glu Ala Ala Gly His Arg Asp Thr Leu Tyr Thr
    370                 375                 380
Met Leu Ile Lys Trp Val Asn Lys Thr Gly Arg Asp Ala Ser Val His
385                 390                 395                 400
Thr Leu Leu Asp Ala Leu Glu Thr Leu Gly Glu Arg Leu Ala Lys Gln
                405                 410                 415
Lys Ile Glu Asp His Leu Leu Ser Ser Gly Lys Phe Met Tyr Leu Glu
            420                 425                 430
Gly Asn Ala Asp Ser Ala Met Ser
        435                 440

<210> 283
<211> 411
<212> PRT
<213> Homo sapiens

<400> 283
Met Glu Gln Arg Gly Gln Asn Ala Pro Ala Ala Ser Gly Ala Arg Lys
1               5                   10                  15
Arg His Gly Pro Gly Pro Arg Glu Ala Arg Gly Ala Arg Pro Gly Leu
            20                  25                  30
Arg Val Pro Lys Thr Leu Val Leu Val Val Ala Ala Val Leu Leu Leu
        35                  40                  45
Val Ser Ala Glu Ser Ala Leu Ile Thr Gln Gln Asp Leu Ala Pro Gln
    50                  55                  60
Gln Arg Ala Ala Pro Gln Gln Lys Arg Ser Ser Pro Ser Glu Gly Leu
65                  70                  75                  80
Cys Pro Pro Gly His His Ile Ser Glu Asp Gly Arg Asp Cys Ile Ser
                85                  90                  95
Cys Lys Tyr Gly Gln Asp Tyr Ser Thr His Trp Asn Asp Leu Leu Phe
            100                 105                 110
Cys Leu Arg Cys Thr Arg Cys Asp Ser Gly Glu Val Glu Leu Ser Pro
        115                 120                 125
Cys Thr Thr Thr Arg Asn Thr Val Cys Gln Cys Glu Glu Gly Thr Phe
    130                 135                 140
Arg Glu Glu Asp Ser Pro Glu Met Cys Arg Lys Cys Arg Thr Gly Cys
145                 150                 155                 160

Pro Arg Gly Met Val Lys Val Gly Asp Cys Thr Pro Trp Ser Asp Ile
                165                 170                 175
Glu Cys Val His Lys Glu Ser Gly Ile Ile Ile Gly Val Thr Val Ala
            180                 185                 190
Ala Val Val Leu Ile Val Ala Val Phe Val Cys Lys Ser Leu Leu Trp
        195                 200                 205
Lys Lys Val Leu Pro Tyr Leu Lys Gly Ile Cys Ser Gly Gly Gly Gly
    210                 215                 220
Asp Pro Glu Arg Val Asp Arg Ser Ser Gln Arg Pro Gly Ala Glu Asp
225                 230                 235                 240
Asn Val Leu Asn Glu Ile Val Ser Ile Leu Gln Pro Thr Gln Val Pro
                245                 250                 255
Glu Gln Glu Met Glu Val Gln Glu Pro Ala Glu Pro Thr Gly Val Asn
            260                 265                 270
Met Leu Ser Pro Gly Glu Ser Glu His Leu Leu Glu Pro Ala Glu Ala
        275                 280                 285
Glu Arg Ser Gln Arg Arg Arg Leu Leu Val Pro Ala Asn Glu Gly Asp
    290                 295                 300
Pro Thr Glu Thr Leu Arg Gln Cys Phe Asp Asp Phe Ala Asp Leu Val
305                 310                 315                 320
Pro Phe Asp Ser Trp Glu Pro Leu Met Arg Lys Leu Gly Leu Met Asp
                325                 330                 335
Asn Glu Ile Lys Val Ala Lys Ala Glu Ala Ala Gly His Arg Asp Thr
            340                 345                 350
Leu Tyr Thr Met Leu Ile Lys Trp Val Asn Lys Thr Gly Arg Asp Ala
        355                 360                 365
Ser Val His Thr Leu Leu Asp Ala Leu Glu Thr Leu Gly Glu Arg Leu
    370                 375                 380
Ala Lys Gln Lys Ile Glu Asp His Leu Leu Ser Ser Gly Lys Phe Met
385                 390                 395                 400
Tyr Leu Glu Gly Asn Ala Asp Ser Ala Met Ser
                405                 410

<210> 284
<211> 802
<212> PRT
<213> Homo sapiens

<400> 284
Met Arg Leu Leu Leu Ala Leu Leu Gly Val Leu Leu Ser Val Pro Gly
1               5                   10                  15
Pro Pro Val Leu Ser Leu Glu Ala Ser Glu Glu Val Glu Leu Glu Pro
            20                  25                  30
Cys Leu Ala Pro Ser Leu Glu Gln Gln Glu Gln Glu Leu Thr Val Ala
        35                  40                  45
Leu Gly Gln Pro Val Arg Leu Cys Cys Gly Arg Ala Glu Arg Gly Gly
    50                  55                  60
His Trp Tyr Lys Glu Gly Ser Arg Leu Ala Pro Ala Gly Arg Val Arg
65                  70                  75                  80
Gly Trp Arg Gly Arg Leu Glu Ile Ala Ser Phe Leu Pro Glu Asp Ala
                85                  90                  95
Gly Arg Tyr Leu Cys Leu Ala Arg Gly Ser Met Ile Val Leu Gln Asn
            100                 105                 110
Leu Thr Leu Ile Thr Gly Asp Ser Leu Thr Ser Ser Asn Asp Asp Glu
        115                 120                 125
Asp Pro Lys Ser His Arg Asp Leu Ser Asn Arg His Ser Tyr Pro Gln
    130                 135                 140
Gln Ala Pro Tyr Trp Thr His Pro Gln Arg Met Glu Lys Lys Leu His
145                 150                 155                 160
Ala Val Pro Ala Gly Asn Thr Val Lys Phe Arg Cys Pro Ala Ala Gly
                165                 170                 175
Asn Pro Thr Pro Thr Ile Arg Trp Leu Lys Asp Gly Gln Ala Phe His

            180                 185                 190
Gly Glu Asn Arg Ile Gly Gly Ile Arg Leu Arg His Gln His Trp Ser
        195                 200                 205
Leu Val Met Glu Ser Val Val Pro Ser Asp Arg Gly Thr Tyr Thr Cys
    210                 215                 220
Leu Val Glu Asn Ala Val Gly Ser Ile Arg Tyr Asn Tyr Leu Leu Asp
225                 230                 235                 240
Val Leu Glu Arg Ser Pro His Arg Pro Ile Leu Gln Ala Gly Leu Pro
                245                 250                 255
Ala Asn Thr Thr Ala Val Val Gly Ser Asp Val Glu Leu Leu Cys Lys
            260                 265                 270
Val Tyr Ser Asp Ala Gln Pro His Ile Gln Trp Leu Lys His Ile Val
        275                 280                 285
Ile Asn Gly Ser Ser Phe Gly Ala Asp Gly Phe Pro Tyr Val Gln Val
    290                 295                 300
Leu Lys Thr Ala Asp Ile Asn Ser Ser Glu Val Glu Val Leu Tyr Leu
305                 310                 315                 320
Arg Asn Val Ser Ala Glu Asp Ala Gly Glu Tyr Thr Cys Leu Ala Gly
                325                 330                 335
Asn Ser Ile Gly Leu Ser Tyr Gln Ser Ala Trp Leu Thr Val Leu Pro
            340                 345                 350
Glu Glu Asp Pro Thr Trp Thr Ala Ala Ala Pro Glu Ala Arg Tyr Thr
        355                 360                 365
Asp Ile Ile Leu Tyr Ala Ser Gly Ser Leu Ala Leu Ala Val Leu Leu
    370                 375                 380
Leu Leu Ala Gly Leu Tyr Arg Gly Gln Ala Leu His Gly Arg His Pro
385                 390                 395                 400
Arg Pro Pro Ala Thr Val Gln Lys Leu Ser Arg Phe Pro Leu Ala Arg
                405                 410                 415
Gln Phe Ser Leu Glu Ser Gly Ser Ser Gly Lys Ser Ser Ser Ser Leu
            420                 425                 430

Val Arg Gly Val Arg Leu Ser Ser Ser Gly Pro Ala Leu Leu Ala Gly
        435                 440                 445
Leu Val Ser Leu Asp Leu Pro Leu Asp Pro Leu Trp Glu Phe Pro Arg
    450                 455                 460
Asp Arg Leu Val Leu Gly Lys Pro Leu Gly Glu Gly Cys Phe Gly Gln
465                 470                 475                 480
Val Val Arg Ala Glu Ala Phe Gly Met Asp Pro Ala Arg Pro Asp Gln
                485                 490                 495
Ala Ser Thr Val Ala Val Lys Met Leu Lys Asp Asn Ala Ser Asp Lys
            500                 505                 510
Asp Leu Ala Asp Leu Val Ser Glu Met Glu Val Met Lys Leu Ile Gly
        515                 520                 525
Arg His Lys Asn Ile Ile Asn Leu Leu Gly Val Cys Thr Gln Glu Gly
    530                 535                 540
Pro Leu Tyr Val Ile Val Glu Cys Ala Ala Lys Gly Asn Leu Arg Glu
545                 550                 555                 560
Phe Leu Arg Ala Arg Arg Pro Pro Gly Pro Asp Leu Ser Pro Asp Gly
                565                 570                 575
Pro Arg Ser Ser Glu Gly Pro Leu Ser Phe Pro Val Leu Val Ser Cys
            580                 585                 590
Ala Tyr Gln Val Ala Arg Gly Met Gln Tyr Leu Glu Ser Arg Lys Cys
        595                 600                 605
Ile His Arg Asp Leu Ala Ala Arg Asn Val Leu Val Thr Glu Asp Asn
    610                 615                 620
Val Met Lys Ile Ala Asp Phe Gly Leu Ala Arg Gly Val His His Ile
625                 630                 635                 640
Asp Tyr Tyr Lys Lys Thr Ser Asn Gly Arg Leu Pro Val Lys Trp Met
                645                 650                 655
Ala Pro Glu Ala Leu Phe Asp Arg Val Tyr Thr His Gln Ser Asp Val
            660                 665                 670
Trp Ser Phe Gly Ile Leu Leu Trp Glu Ile Phe Thr Leu Gly Gly Ser

        675                 680                 685
Pro Tyr Pro Gly Ile Pro Val Glu Glu Leu Phe Ser Leu Leu Arg Glu
    690                 695                 700
Gly His Arg Met Asp Arg Pro Pro His Cys Pro Pro Glu Leu Tyr Gly
705                 710                 715                 720
Leu Met Arg Glu Cys Trp His Ala Ala Pro Ser Gln Arg Pro Thr Phe
                725                 730                 735
Lys Gln Leu Val Glu Ala Leu Asp Lys Val Leu Leu Ala Val Ser Glu
            740                 745                 750
Glu Tyr Leu Asp Leu Arg Leu Thr Phe Gly Pro Tyr Ser Pro Ser Gly
        755                 760                 765
Gly Asp Ala Ser Ser Thr Cys Ser Ser Ser Asp Ser Val Phe Ser His
    770                 775                 780
Asp Pro Leu Pro Leu Gly Ser Ser Ser Phe Pro Phe Gly Ser Gly Val
785                 790                 795                 800
Gln Thr

      <210> 285
      <211> 762
      <212> PRT
      <213> Homo sapiens

      <400> 285
Met Arg Leu Leu Leu Ala Leu Leu Gly Val Leu Leu Ser Val Pro Gly
 1               5                  10                  15
Pro Pro Val Leu Ser Leu Glu Ala Ser Glu Glu Val Glu Leu Glu Pro
            20                  25                  30
Cys Leu Ala Pro Ser Leu Glu Gln Gln Glu Gln Glu Leu Thr Val Ala
        35                  40                  45

Leu Gly Gln Pro Val Arg Leu Cys Cys Gly Arg Ala Glu Arg Gly Gly
    50                  55                  60
His Trp Tyr Lys Glu Gly Ser Arg Leu Ala Pro Ala Gly Arg Val Arg
65                  70                  75                  80
Gly Trp Arg Gly Arg Leu Glu Ile Ala Ser Phe Leu Pro Glu Asp Ala
                85                  90                  95
Gly Arg Tyr Leu Cys Leu Ala Arg Gly Ser Met Ile Val Leu Gln Asn
            100                 105                 110
Leu Thr Leu Ile Thr Gly Asp Ser Leu Thr Ser Ser Asn Asp Asp Glu
        115                 120                 125
Asp Pro Lys Ser His Arg Asp Leu Ser Asn Arg His Ser Tyr Pro Gln
    130                 135                 140
Gln Ala Pro Tyr Trp Thr His Pro Gln Arg Met Glu Lys Lys Leu His
145                 150                 155                 160
Ala Val Pro Ala Gly Asn Thr Val Lys Phe Arg Cys Pro Ala Ala Gly
                165                 170                 175
Asn Pro Thr Pro Thr Ile Arg Trp Leu Lys Asp Gly Gln Ala Phe His
            180                 185                 190
Gly Glu Asn Arg Ile Gly Gly Ile Arg Leu Arg His Gln His Trp Ser
        195                 200                 205
Leu Val Met Glu Ser Val Val Pro Ser Asp Arg Gly Thr Tyr Thr Cys
    210                 215                 220
Leu Val Glu Asn Ala Val Gly Ser Ile Arg Tyr Asn Tyr Leu Leu Asp
225                 230                 235                 240
Val Leu Glu Arg Ser Pro His Arg Pro Ile Leu Gln Ala Gly Leu Pro
                245                 250                 255
Ala Asn Thr Thr Ala Val Val Gly Ser Asp Val Glu Leu Leu Cys Lys
            260                 265                 270
Val Tyr Ser Asp Ala Gln Pro His Ile Gln Trp Leu Lys His Ile Val
        275                 280                 285
Ile Asn Gly Ser Ser Phe Gly Ala Asp Gly Phe Pro Tyr Val Gln Val
    290                 295                 300
Leu Lys Thr Ala Asp Ile Asn Ser Ser Glu Val Glu Val Leu Tyr Leu

305                 310                 315                 320
Arg Asn Val Ser Ala Glu Asp Ala Gly Glu Tyr Thr Cys Leu Ala Gly
                325                 330                 335
Asn Ser Ile Gly Leu Ser Tyr Gln Ser Ala Trp Leu Thr Val Leu Pro
            340                 345                 350
Gly Thr Gly Arg Ile Pro His Leu Thr Cys Asp Ser Leu Thr Pro Ala
        355                 360                 365
Gly Arg Thr Lys Ser Pro Thr Leu Gln Phe Ser Leu Glu Ser Gly Ser
    370                 375                 380
Ser Gly Lys Ser Ser Ser Ser Leu Val Arg Gly Val Arg Leu Ser Ser
385                 390                 395                 400
Ser Gly Pro Ala Leu Leu Ala Gly Leu Val Ser Leu Asp Leu Pro Leu
                405                 410                 415
Asp Pro Leu Trp Glu Phe Pro Arg Asp Arg Leu Val Leu Gly Lys Pro
            420                 425                 430
Leu Gly Glu Gly Cys Phe Gly Gln Val Val Arg Ala Glu Ala Phe Gly
        435                 440                 445
Met Asp Pro Ala Arg Pro Asp Gln Ala Ser Thr Val Ala Val Lys Met
    450                 455                 460
Leu Lys Asp Asn Ala Ser Asp Lys Asp Leu Ala Asp Leu Val Ser Glu
465                 470                 475                 480
Met Glu Val Met Lys Leu Ile Gly Arg His Lys Asn Ile Ile Asn Leu
                485                 490                 495
Leu Gly Val Cys Thr Gln Glu Gly Pro Leu Tyr Val Ile Val Glu Cys
            500                 505                 510
Ala Ala Lys Gly Asn Leu Arg Glu Phe Leu Arg Ala Arg Arg Pro Pro
        515                 520                 525

Gly Pro Asp Leu Ser Pro Asp Gly Pro Arg Ser Ser Glu Gly Pro Leu
    530                 535                 540
Ser Phe Pro Val Leu Val Ser Cys Ala Tyr Gln Val Ala Arg Gly Met
545                 550                 555                 560
Gln Tyr Leu Glu Ser Arg Lys Cys Ile His Arg Asp Leu Ala Ala Arg
                565                 570                 575
Asn Val Leu Val Thr Glu Asp Asn Val Met Lys Ile Ala Asp Phe Gly
            580                 585                 590
Leu Ala Arg Gly Val His His Ile Asp Tyr Tyr Lys Lys Thr Ser Asn
        595                 600                 605
Gly Arg Leu Pro Val Lys Trp Met Ala Pro Glu Ala Leu Phe Asp Arg
    610                 615                 620
Val Tyr Thr His Gln Ser Asp Val Trp Ser Phe Gly Ile Leu Leu Trp
625                 630                 635                 640
Glu Ile Phe Thr Leu Gly Gly Ser Pro Tyr Pro Gly Ile Pro Val Glu
                645                 650                 655
Glu Leu Phe Ser Leu Leu Arg Glu Gly His Arg Met Asp Arg Pro Pro
            660                 665                 670
His Cys Pro Pro Glu Leu Tyr Gly Leu Met Arg Glu Cys Trp His Ala
        675                 680                 685
Ala Pro Ser Gln Arg Pro Thr Phe Lys Gln Leu Val Glu Ala Leu Asp
    690                 695                 700
Lys Val Leu Leu Ala Val Ser Glu Glu Tyr Leu Asp Leu Arg Leu Thr
705                 710                 715                 720
Phe Gly Pro Tyr Ser Pro Ser Gly Gly Asp Ala Ser Ser Thr Cys Ser
                725                 730                 735
Ser Ser Asp Ser Val Phe Ser His Asp Pro Leu Pro Leu Gly Ser Ser
            740                 745                 750
Ser Phe Pro Phe Gly Ser Gly Val Gln Thr
        755                 760

<210> 286
<211> 1452
<212> PRT
<213> Homo sapiens

<400> 286
Met Arg Gly Leu Gly Thr Cys Leu Ala Thr Leu Ala Gly Leu Leu Leu
1               5                   10                  15
Thr Ala Ala Gly Glu Thr Phe Ser Gly Gly Cys Leu Phe Asp Glu Pro
            20                  25                  30
Tyr Ser Thr Cys Gly Tyr Ser Gln Ser Glu Gly Asp Asp Phe Asn Trp
        35                  40                  45
Glu Gln Val Asn Thr Leu Thr Lys Pro Thr Ser Asp Pro Trp Met Pro
    50                  55                  60
Ser Gly Ser Leu Met Leu Val Asn Ala Ser Gly Arg Pro Glu Gly Gln
65                  70                  75                  80
Arg Ala His Leu Leu Leu Pro Gln Leu Lys Glu Asn Asp Thr His Cys
                85                  90                  95
Ile Asp Phe His Tyr Phe Val Ser Ser Lys Ser Asn Ser Pro Pro Gly
            100                 105                 110
Leu Leu Asn Val Tyr Val Lys Val Asn Asn Gly Pro Leu Gly Asn Pro
        115                 120                 125
Ile Trp Asn Ile Ser Gly Asp Pro Thr Arg Thr Trp Asn Arg Ala Glu
    130                 135                 140
Leu Ala Ile Ser Thr Phe Trp Pro Asn Phe Tyr Gln Val Ile Phe Glu
145                 150                 155                 160
Val Ile Thr Ser Gly His Gln Gly Tyr Leu Ala Ile Asp Glu Val Lys
                165                 170                 175
Val Leu Gly His Pro Cys Thr Arg Thr Pro His Phe Leu Arg Ile Gln
            180                 185                 190
Asn Val Glu Val Asn Ala Gly Gln Phe Ala Thr Phe Gln Cys Ser Ala
        195                 200                 205
Ile Gly Arg Thr Val Ala Gly Asp Arg Leu Trp Leu Gln Gly Ile Asp
    210                 215                 220
Val Arg Asp Ala Pro Leu Lys Glu Ile Lys Val Thr Ser Ser Arg Arg
225                 230                 235                 240
Phe Ile Ala Ser Phe Asn Val Val Asn Thr Thr Lys Arg Asp Ala Gly
                245                 250                 255
Lys Tyr Arg Cys Met Ile Arg Thr Glu Gly Gly Val Gly Ile Ser Asn
            260                 265                 270
Tyr Ala Glu Leu Val Val Lys Glu Pro Pro Val Pro Ile Ala Pro Pro
        275                 280                 285
Gln Leu Ala Ser Val Gly Ala Thr Tyr Leu Trp Ile Gln Leu Asn Ala
    290                 295                 300
Asn Ser Ile Asn Gly Asp Gly Pro Ile Val Ala Arg Glu Val Glu Tyr
305                 310                 315                 320
Cys Thr Ala Ser Gly Ser Trp Asn Asp Arg Gln Pro Val Asp Ser Thr
                325                 330                 335
Ser Tyr Lys Ile Gly His Leu Asp Pro Asp Thr Glu Tyr Glu Ile Ser
            340                 345                 350
Val Leu Leu Thr Arg Pro Gly Glu Gly Gly Thr Gly Ser Pro Gly Pro
        355                 360                 365
Ala Leu Arg Thr Arg Thr Lys Cys Ala Asp Pro Met Arg Gly Pro Arg
    370                 375                 380
Lys Leu Glu Val Val Glu Val Lys Ser Arg Gln Ile Thr Ile Arg Trp
385                 390                 395                 400
Glu Pro Phe Gly Tyr Asn Val Thr Arg Cys His Ser Tyr Asn Leu Thr
                405                 410                 415
Val His Tyr Cys Tyr Gln Val Gly Gly Gln Glu Gln Val Arg Glu Glu
            420                 425                 430
Val Ser Trp Asp Thr Glu Asn Ser His Pro Gln His Thr Ile Thr Asn
        435                 440                 445
Leu Ser Pro Tyr Thr Asn Val Ser Val Lys Leu Ile Leu Met Asn Pro
    450                 455                 460
Glu Gly Arg Lys Glu Ser Gln Glu Leu Ile Val Gln Thr Asp Glu Asp
465                 470                 475                 480
Leu Pro Gly Ala Val Pro Thr Glu Ser Ile Gln Gly Ser Thr Phe Glu

                485                 490                 495
Glu Lys Ile Phe Leu Gln Trp Arg Glu Pro Thr Gln Thr Tyr Gly Val
            500                 505                 510
Ile Thr Leu Tyr Glu Ile Thr Tyr Lys Ala Val Ser Ser Phe Asp Pro
        515                 520                 525
Glu Ile Asp Leu Ser Asn Gln Ser Gly Arg Val Ser Lys Leu Gly Asn
    530                 535                 540
Glu Thr His Phe Leu Phe Phe Gly Leu Tyr Pro Gly Thr Thr Tyr Ser
545                 550                 555                 560
Phe Thr Ile Arg Ala Ser Thr Ala Lys Gly Phe Gly Pro Pro Ala Thr
                565                 570                 575
Asn Gln Phe Thr Thr Lys Ile Ser Ala Pro Ser Met Pro Ala Tyr Glu
            580                 585                 590
Leu Glu Thr Pro Leu Asn Gln Thr Asp Asn Thr Val Thr Val Met Leu
        595                 600                 605
Lys Pro Ala His Ser Arg Gly Ala Pro Val Ser Val Tyr Gln Ile Val
    610                 615                 620
Val Glu Glu Glu Arg Pro Arg Arg Thr Lys Lys Thr Thr Glu Ile Leu
625                 630                 635                 640
Lys Cys Tyr Pro Val Pro Ile His Phe Gln Asn Ala Ser Leu Leu Asn
                645                 650                 655
Ser Gln Tyr Tyr Phe Ala Ala Glu Phe Pro Ala Asp Ser Leu Gln Ala
            660                 665                 670

Ala Gln Pro Phe Thr Ile Gly Asp Asn Lys Thr Tyr Asn Gly Tyr Trp
        675                 680                 685
Asn Thr Pro Leu Leu Pro Tyr Lys Ser Tyr Arg Ile Tyr Phe Gln Ala
    690                 695                 700
Ala Ser Arg Ala Asn Gly Glu Thr Lys Ile Asp Cys Val Gln Val Ala
705                 710                 715                 720
Thr Lys Gly Ala Ala Thr Pro Lys Pro Val Pro Glu Pro Glu Lys Gln
                725                 730                 735
Thr Asp His Thr Val Lys Ile Ala Gly Val Ile Ala Gly Ile Leu Leu
            740                 745                 750
Phe Val Ile Ile Phe Leu Gly Val Val Leu Val Met Lys Lys Arg Lys
        755                 760                 765
Leu Ala Lys Lys Arg Lys Glu Thr Met Ser Ser Thr Arg Gln Glu Met
    770                 775                 780
Thr Val Met Val Asn Ser Met Asp Lys Ser Tyr Ala Glu Gln Gly Thr
785                 790                 795                 800
Asn Cys Asp Glu Ala Phe Ser Phe Met Asp Thr His Asn Leu Asn Gly
                805                 810                 815
Arg Ser Val Ser Ser Pro Ser Ser Phe Thr Met Lys Thr Asn Thr Leu
            820                 825                 830
Ser Thr Ser Val Pro Asn Ser Tyr Tyr Pro Asp Glu Thr His Thr Met
        835                 840                 845
Ala Ser Asp Thr Ser Ser Leu Val Gln Ser His Thr Tyr Lys Lys Arg
    850                 855                 860
Glu Pro Ala Asp Val Pro Tyr Gln Thr Gly Gln Leu His Pro Ala Ile
865                 870                 875                 880
Arg Val Ala Asp Leu Leu Gln His Ile Thr Gln Met Lys Cys Ala Glu
                885                 890                 895
Gly Tyr Gly Phe Lys Glu Glu Tyr Glu Ser Phe Phe Glu Gly Gln Ser
            900                 905                 910
Ala Pro Trp Asp Ser Ala Lys Lys Asp Glu Asn Arg Met Lys Asn Arg
        915                 920                 925
Tyr Gly Asn Ile Ile Ala Tyr Asp His Ser Arg Val Arg Leu Gln Thr
    930                 935                 940
Ile Glu Gly Asp Thr Asn Ser Asp Tyr Ile Asn Gly Asn Tyr Ile Asp
945                 950                 955                 960
Gly Tyr His Arg Pro Asn His Tyr Ile Ala Thr Gln Gly Pro Met Gln
                965                 970                 975
Glu Thr Ile Tyr Asp Phe Trp Arg Met Val Trp His Glu Asn Thr Ala

980                 985                 990
Ser Ile Ile Met Val Thr Asn Leu Val Glu Val Gly Arg Val Lys Cys
        995                 1000                1005
Cys Lys Tyr Trp Pro Asp Asp Thr Glu Ile Tyr Lys Asp Ile Lys Val
    1010                1015                1020
Thr Leu Ile Glu Thr Glu Leu Leu Ala Glu Tyr Val Ile Arg Thr Phe
1025                1030                1035                1040
Ala Val Glu Lys Arg Gly Val His Glu Ile Arg Glu Ile Arg Gln Phe
                1045                1050                1055
His Phe Thr Gly Trp Pro Asp His Gly Val Pro Tyr His Ala Thr Gly
            1060                1065                1070
Leu Leu Gly Phe Val Arg Gln Val Lys Ser Lys Ser Pro Pro Ser Ala
        1075                1080                1085
Gly Pro Leu Val Val His Cys Ser Ala Gly Ala Gly Arg Thr Gly Cys
    1090                1095                1100
Phe Ile Val Ile Asp Ile Met Leu Asp Met Ala Glu Arg Glu Gly Val
1105                1110                1115                1120
Val Asp Ile Tyr Asn Cys Val Arg Glu Leu Arg Ser Arg Arg Val Asn
                1125                1130                1135
Met Val Gln Thr Glu Glu Gln Tyr Val Phe Ile His Asp Ala Ile Leu
            1140                1145                1150

Glu Ala Cys Leu Cys Gly Asp Thr Ser Val Pro Ala Ser Gln Val Arg
        1155                1160                1165
Ser Leu Tyr Tyr Asp Met Asn Lys Leu Asp Pro Gln Thr Asn Ser Ser
    1170                1175                1180
Gln Ile Lys Glu Glu Phe Arg Thr Leu Asn Met Val Thr Pro Thr Leu
1185                1190                1195                1200
Arg Val Glu Asp Cys Ser Ile Ala Leu Leu Pro Arg Asn His Glu Lys
                1205                1210                1215
Asn Arg Cys Met Asp Ile Leu Pro Pro Asp Arg Cys Leu Pro Phe Leu
            1220                1225                1230
Ile Thr Ile Asp Gly Glu Ser Ser Asn Tyr Ile Asn Ala Ala Leu Met
        1235                1240                1245
Asp Ser Tyr Lys Gln Pro Ser Ala Phe Ile Val Thr Gln His Pro Leu
    1250                1255                1260
Pro Asn Thr Val Lys Asp Phe Trp Arg Leu Val Leu Asp Tyr His Cys
1265                1270                1275                1280
Thr Ser Val Val Met Leu Asn Asp Val Asp Pro Ala Gln Leu Cys Pro
                1285                1290                1295
Gln Tyr Trp Pro Glu Asn Gly Val His Arg His Gly Pro Ile Gln Val
            1300                1305                1310
Glu Phe Val Ser Ala Asp Leu Glu Glu Asp Ile Ile Ser Arg Ile Phe
        1315                1320                1325
Arg Ile Tyr Asn Ala Ala Arg Pro Gln Asp Gly Tyr Arg Met Val Gln
    1330                1335                1340
Gln Phe Gln Phe Leu Gly Trp Pro Met Tyr Arg Asp Thr Pro Val Ser
1345                1350                1355                1360
Lys Arg Ser Phe Leu Lys Leu Ile Arg Gln Val Asp Lys Trp Gln Glu
                1365                1370                1375
Glu Tyr Asn Gly Gly Glu Gly Pro Thr Val Val His Cys Leu Asn Gly
            1380                1385                1390
Gly Gly Arg Ser Gly Thr Phe Cys Ala Ile Ser Ile Val Cys Glu Met
        1395                1400                1405
Leu Arg His Gln Arg Thr Val Asp Val Phe His Ala Val Lys Thr Leu
    1410                1415                1420
Arg Asn Asn Lys Pro Asn Met Val Asp Leu Leu Asp Gln Tyr Lys Phe
1425                1430                1435                1440
Cys Tyr Glu Val Ala Leu Glu Tyr Leu Asn Ser Gly
                1445                1450

<210> 287
<211> 570

<212> PRT
<213> Homo sapiens

<400> 287
Met Gly Phe Leu Gln Leu Leu Val Val Ala Val Leu Ala Ser Glu His
1               5                   10                  15
Arg Val Ala Gly Ala Ala Glu Val Phe Gly Asn Ser Ser Glu Gly Leu
            20                  25                  30
Ile Glu Phe Ser Val Gly Lys Phe Arg Tyr Phe Glu Leu Asn Arg Pro
        35                  40                  45
Phe Pro Glu Glu Ala Ile Leu His Asp Ile Ser Ser Asn Val Thr Phe
    50                  55                  60
Leu Ile Phe Gln Ile His Ser Gln Tyr Gln Asn Thr Thr Val Ser Phe
65                  70                  75                  80
Ser Pro Thr Leu Leu Ser Asn Ser Ser Glu Thr Gly Thr Ala Ser Gly
                85                  90                  95
Leu Val Phe Ile Leu Arg Pro Glu Gln Ser Thr Cys Thr Trp Tyr Leu
            100                 105                 110
Gly Thr Ser Gly Ile Gln Pro Val Gln Asn Met Ala Ile Leu Leu Ser
        115                 120                 125
Tyr Ser Glu Arg Asp Pro Val Pro Gly Gly Cys Asn Leu Glu Phe Asp
    130                 135                 140
Leu Asp Ile Asp Pro Asn Ile Tyr Leu Glu Tyr Asn Phe Phe Glu Thr
145                 150                 155                 160
Thr Ile Lys Phe Ala Pro Ala Asn Leu Gly Tyr Ala Arg Gly Val Asp
                165                 170                 175
Pro Pro Pro Cys Asp Ala Gly Thr Asp Gln Asp Ser Arg Trp Arg Leu
            180                 185                 190
Gln Tyr Asp Val Tyr Gln Tyr Phe Leu Pro Glu Asn Asp Leu Thr Glu
        195                 200                 205
Glu Met Leu Leu Lys His Leu Gln Arg Met Val Ser Val Pro Gln Val
    210                 215                 220
Lys Ala Ser Ala Leu Lys Val Val Thr Leu Thr Ala Asn Asp Lys Thr
225                 230                 235                 240
Ser Val Ser Phe Ser Ser Leu Pro Gly Gln Gly Val Ile Tyr Asn Val
                245                 250                 255
Ile Val Trp Asp Pro Phe Leu Asn Thr Ser Ala Ala Tyr Ile Pro Ala
            260                 265                 270
His Thr Tyr Ala Cys Ser Phe Glu Ala Gly Glu Gly Ser Cys Ala Ser
        275                 280                 285
Leu Gly Arg Val Ser Ser Lys Val Phe Phe Thr Leu Phe Ala Leu Leu
    290                 295                 300
Gly Phe Phe Ile Cys Phe Phe Gly His Arg Phe Trp Lys Thr Glu Leu
305                 310                 315                 320
Phe Phe Ile Gly Phe Ile Ile Met Gly Phe Phe Phe Tyr Ile Leu Ile
                325                 330                 335
Thr Arg Leu Thr Pro Ile Lys Tyr Asp Val Asn Leu Ile Leu Thr Ala
            340                 345                 350
Val Thr Gly Ser Val Gly Gly Met Phe Leu Val Ala Val Trp Trp Arg
        355                 360                 365
Phe Gly Ile Leu Ser Ile Cys Met Leu Cys Val Gly Leu Val Leu Gly
    370                 375                 380
Phe Leu Ile Ser Ser Val Thr Phe Phe Thr Pro Leu Gly Asn Leu Lys
385                 390                 395                 400
Ile Phe His Asp Asp Gly Val Phe Trp Val Thr Phe Ser Cys Ile Ala
                405                 410                 415
Ile Leu Ile Pro Val Val Phe Met Gly Cys Leu Arg Ile Leu Asn Ile
            420                 425                 430
Leu Thr Cys Gly Val Ile Gly Ser Tyr Ser Val Val Leu Ala Ile Asp
        435                 440                 445
Ser Tyr Trp Ser Thr Ser Leu Ser Tyr Ile Thr Leu Asn Val Leu Lys
    450                 455                 460
Arg Ala Leu Asn Lys Asp Phe His Arg Ala Phe Thr Asn Val Pro Phe

465                 470                 475                 480
Gln Thr Asn Asp Phe Ile Ile Leu Ala Val Trp Gly Met Leu Ala Val
                485                 490                 495
Ser Gly Ile Thr Leu Gln Ile Arg Arg Glu Arg Gly Arg Pro Phe Phe
            500                 505                 510
Pro Pro His Pro Tyr Lys Leu Trp Lys Gln Glu Arg Glu Arg Arg Val
        515                 520                 525
Thr Asn Ile Leu Asp Pro Ser Tyr His Ile Pro Pro Leu Arg Glu Arg
    530                 535                 540
Leu Tyr Gly Arg Leu Thr Gln Ile Lys Gly Leu Phe Gln Lys Glu Gln
545                 550                 555                 560
Pro Ala Gly Glu Arg Thr Pro Leu Leu Leu
                565                 570

<210> 288
<211> 283
<212> PRT
<213> Homo sapiens
<400> 288

<210> 289
<211> 342
<212> PRT
<213> Homo sapiens

<400> 289
   Met Glu Pro His Asp Ser Ser His Met Asp Ser Glu Phe Arg Tyr Thr
   1                   5                   10                  15
   Leu Phe Pro Ile Val Tyr Ser Ile Ile Phe Val Leu Gly Val Ile Ala
                   20                  25                  30
   Asn Gly Tyr Val Leu Trp Val Phe Ala Arg Leu Tyr Pro Cys Lys Lys
               35                  40                  45
   Phe Asn Glu Ile Lys Ile Phe Met Val Asn Leu Thr Met Ala Asp Met
           50                  55                  60
   Leu Phe Leu Ile Thr Leu Pro Leu Trp Ile Val Tyr Tyr Gln Asn Gln
   65                  70                  75                  80
   Gly Asn Trp Ile Leu Pro Lys Phe Leu Cys Asn Val Ala Gly Cys Leu
                   85                  90                  95
   Phe Phe Ile Asn Thr Tyr Cys Ser Val Ala Phe Leu Gly Val Ile Thr
               100                 105                 110
   Tyr Asn Arg Phe Gln Ala Val Thr Arg Pro Ile Lys Thr Ala Gln Ala
           115                 120                 125
   Asn Thr Arg Lys Arg Gly Ile Ser Leu Ser Leu Val Ile Trp Val Ala
       130                 135                 140
   Ile Val Gly Ala Ala Ser Tyr Phe Leu Ile Leu Asp Ser Thr Asn Thr
   145                 150                 155                 160
   Val Pro Asp Ser Ala Gly Ser Gly Asn Val Thr Arg Cys Phe Glu His
                   165                 170                 175
   Tyr Glu Lys Gly Ser Val Pro Val Leu Ile Ile His Ile Phe Ile Val
               180                 185                 190
   Phe Ser Phe Phe Leu Val Phe Leu Ile Ile Leu Phe Cys Asn Leu Val
           195                 200                 205
   Ile Ile Arg Thr Leu Leu Met Gln Pro Val Gln Gln Gln Arg Asn Ala
       210                 215                 220
   Glu Val Lys Arg Arg Ala Leu Trp Met Val Cys Thr Val Leu Ala Val
   225                 230                 235                 240
   Phe Ile Ile Cys Phe Val Pro His His Val Val Gln Leu Pro Trp Thr
                   245                 250                 255
   Leu Ala Glu Leu Gly Phe Gln Asp Ser Lys Phe His Gln Ala Ile Asn
               260                 265                 270
   Asp Ala His Gln Val Thr Leu Cys Leu Leu Ser Thr Asn Cys Val Leu
           275                 280                 285
   Asp Pro Val Ile Tyr Cys Phe Leu Thr Lys Lys Phe Arg Lys His Leu
       290                 295                 300
   Thr Glu Lys Phe Tyr Ser Met Arg Ser Ser Arg Lys Cys Ser Arg Ala
   305                 310                 315                 320
   Thr Thr Asp Thr Val Thr Glu Val Val Val Pro Phe Asn Gln Ile Pro
                   325                 330                 335
   Gly Asn Ser Leu Lys Asn
               340

<210> 290
<211> 168
<212> PRT
<213> Homo sapiens

<400> 290
Gly Cys Leu Gly Ala Ile Lys Glu Asn Lys Cys Leu Leu Leu Thr Phe
1               5                   10                  15
Phe Leu Leu Leu Leu Leu Val Phe Leu Leu Glu Ala Thr Ile Ala Ile
            20                  25                  30
Leu Phe Phe Ala Tyr Thr Asp Lys Ile Asp Arg Tyr Ala Gln Gln Asp
        35                  40                  45
Leu Lys Lys Gly Leu His Leu Tyr Gly Thr Gln Gly Asn Val Gly Leu
    50                  55                  60
Thr Asn Ala Trp Ser Ile Ile Gln Thr Asp Phe Arg Cys Cys Gly Val
65                  70                  75                  80
Ser Asn Tyr Thr Asp Trp Phe Glu Val Tyr Asn Ala Thr Arg Val Pro
                85                  90                  95

Asp Ser Cys Cys Leu Glu Phe Ser Glu Ser Cys Gly Leu His Ala Pro
            100                 105                 110
Gly Thr Trp Trp Lys Ala Pro Cys Tyr Glu Thr Val Lys Val Trp Leu
        115                 120                 125
Gln Glu Asn Leu Leu Ala Val Gly Ile Phe Gly Leu Cys Thr Ala Leu
    130                 135                 140
Val Gln Ile Leu Gly Leu Thr Phe Ala Met Thr Met Tyr Cys Gln Val
145                 150                 155                 160
Val Lys Ala Asp Thr Tyr Cys Ala
                165

<210> 291
<211> 635
<212> PRT
<213> Homo sapiens

<400> 291

<210> 292
<211> 63
<212> PRT
<213> Homo sapiens

<400> 292
Met Lys His Val Leu Asn Leu Tyr Leu Leu Gly Val Val Leu Thr Leu
1               5                   10                  15
Leu Ser Ile Phe Val Arg Val Met Glu Ser Leu Glu Gly Leu Leu Glu
            20                  25                  30
Ser Pro Ser Pro Gly Thr Ser Trp Thr Thr Arg Ser Gln Leu Ala Asn
        35                  40                  45
Thr Glu Pro Thr Lys Gly Leu Pro Asp His Pro Ser Arg Ser Met
    50                  55                  60

<210> 293
<211> 87
<212> PRT
<213> Homo sapiens

<400> 293
Met Ala Gly Ala Ser Leu Gly Ala Arg Phe Tyr Arg Gln Ile Lys Arg
1               5                   10                  15
His Pro Gly Ile Ile Pro Met Ile Gly Leu Ile Cys Leu Gly Met Gly
            20                  25                  30
Ser Ala Ala Leu Tyr Leu Leu Arg Leu Ala Leu Arg Ser Pro Asp Val

35                  40                  45
Cys Trp Asp Arg Lys Asn Asn Pro Glu Pro Trp Asn Arg Leu Ser Pro
    50                  55                  60
Asn Asp Gln Tyr Lys Phe Leu Ala Val Ser Thr Asp Tyr Lys Lys Leu
65                  70                  75                  80
Lys Lys Asp Arg Pro Asp Phe
                85

<210> 294
<211> 1051
<212> PRT
<213> Homo sapiens

<400> 294

<210> 295
<211> 1066
<212> PRT
<213> Homo sapiens

<400> 295
Met Gly Pro Gly Pro Ser Arg Ala Pro Arg Ala Pro Arg Leu Met Leu
1               5                   10                  15
Cys Ala Leu Ala Leu Met Val Ala Ala Gly Gly Cys Val Val Ser Ala
            20                  25                  30
Phe Asn Leu Asp Thr Arg Phe Leu Val Val Lys Glu Ala Gly Asn Pro
        35                  40                  45
Gly Ser Leu Phe Gly Tyr Ser Val Ala Leu His Arg Gln Thr Glu Arg
    50                  55                  60
Gln Gln Arg Tyr Leu Leu Leu Ala Gly Ala Pro Arg Glu Leu Ala Val
65                  70                  75                  80
Pro Asp Gly Tyr Thr Asn Arg Thr Gly Ala Val Tyr Leu Cys Pro Leu
                85                  90                  95
Thr Ala His Lys Asp Asp Cys Glu Arg Met Asn Ile Thr Val Lys Asn
            100                 105                 110
Asp Pro Gly His His Ile Ile Glu Asp Met Trp Leu Gly Val Thr Val
        115                 120                 125
Ala Ser Gln Gly Pro Ala Gly Arg Val Leu Val Cys Ala His Arg Tyr
    130                 135                 140
Thr Gln Val Leu Trp Ser Gly Ser Glu Asp Gln Arg Arg Met Val Gly
145                 150                 155                 160
Lys Cys Tyr Val Arg Gly Asn Asp Leu Glu Leu Asp Ser Ser Asp Asp
                165                 170                 175
Trp Gln Thr Tyr His Asn Glu Met Cys Asn Ser Asn Thr Asp Tyr Leu
            180                 185                 190
Glu Thr Gly Met Cys Gln Leu Gly Thr Ser Gly Gly Phe Thr Gln Asn
        195                 200                 205
Thr Val Tyr Phe Gly Ala Pro Gly Ala Tyr Asn Trp Lys Gly Asn Ser
    210                 215                 220
Tyr Met Ile Gln Arg Lys Glu Trp Asp Leu Ser Glu Tyr Ser Tyr Lys
225                 230                 235                 240
Asp Pro Glu Asp Gln Gly Asn Leu Tyr Ile Gly Tyr Thr Met Gln Val
                245                 250                 255
Gly Ser Phe Ile Leu His Pro Lys Asn Ile Thr Ile Val Thr Gly Ala
            260                 265                 270

Pro Arg His Arg His Met Gly Ala Val Phe Leu Leu Ser Gln Glu Ala
        275                 280                 285
Gly Gly Asp Leu Arg Arg Arg Gln Val Leu Glu Gly Ser Gln Val Gly
    290                 295                 300
Ala Tyr Phe Gly Ser Ala Ile Ala Leu Ala Asp Leu Asn Asn Asp Gly
305                 310                 315                 320
Trp Gln Asp Leu Leu Val Gly Ala Pro Tyr Tyr Phe Glu Arg Lys Glu
                325                 330                 335
Glu Val Gly Gly Ala Ile Tyr Val Phe Met Asn Gln Ala Gly Thr Ser
            340                 345                 350
Phe Pro Ala His Pro Ser Leu Leu Leu His Gly Pro Ser Gly Ser Ala
        355                 360                 365
Phe Gly Leu Ser Val Ala Ser Ile Gly Asp Ile Asn Gln Asp Gly Phe
    370                 375                 380
Gln Asp Ile Ala Val Gly Ala Pro Phe Glu Gly Leu Gly Lys Val Tyr
385                 390                 395                 400
Ile Tyr His Ser Ser Ser Lys Gly Leu Leu Arg Gln Pro Gln Gln Val
                405                 410                 415
Ile His Gly Glu Lys Leu Gly Leu Pro Gly Leu Ala Thr Phe Gly Tyr
            420                 425                 430
Ser Leu Ser Gly Gln Met Asp Val Asp Glu Asn Phe Tyr Pro Asp Leu
        435                 440                 445
Leu Val Gly Ser Leu Ser Asp His Ile Val Leu Leu Arg Ala Arg Pro
    450                 455                 460
Val Ile Asn Ile Val His Lys Thr Leu Val Pro Arg Pro Ala Val Leu
465                 470                 475                 480
Asp Pro Ala Leu Cys Thr Ala Thr Ser Cys Val Gln Val Glu Leu Cys
                485                 490                 495
Phe Ala Tyr Asn Gln Ser Ala Gly Asn Pro Asn Tyr Arg Arg Asn Ile
            500                 505                 510
Thr Leu Ala Tyr Thr Leu Glu Ala Asp Arg Asp Arg Arg Pro Pro Arg
        515                 520                 525
Leu Arg Phe Ala Gly Ser Glu Ser Ala Val Phe His Gly Phe Phe Ser
    530                 535                 540
Met Pro Glu Met Arg Cys Gln Lys Leu Glu Leu Leu Leu Met Asp Asn
545                 550                 555                 560
Leu Arg Asp Lys Leu Arg Pro Ile Ile Ile Ser Met Asn Tyr Ser Leu
                565                 570                 575
Pro Leu Arg Met Pro Asp Arg Pro Arg Leu Gly Leu Arg Ser Leu Asp
            580                 585                 590
Ala Tyr Pro Ile Leu Asn Gln Ala Gln Ala Leu Glu Asn His Thr Glu
        595                 600                 605
Val Gln Phe Gln Lys Glu Cys Gly Pro Asp Asn Lys Cys Glu Ser Asn
    610                 615                 620
Leu Gln Met Arg Ala Ala Phe Val Ser Glu Gln Gln Gln Lys Leu Ser
625                 630                 635                 640
Arg Leu Gln Tyr Ser Arg Asp Val Arg Lys Leu Leu Leu Ser Ile Asn
                645                 650                 655
Val Thr Asn Thr Arg Thr Ser Glu Arg Ser Gly Glu Asp Ala His Glu
            660                 665                 670
Ala Leu Leu Thr Leu Val Val Pro Pro Ala Leu Leu Leu Ser Ser Val
        675                 680                 685
Arg Pro Pro Gly Ala Cys Gln Ala Asn Glu Thr Ile Phe Cys Glu Leu
    690                 695                 700
Gly Asn Pro Phe Lys Arg Asn Gln Arg Met Glu Leu Leu Ile Ala Phe
705                 710                 715                 720
Glu Val Ile Gly Val Thr Leu His Thr Arg Asp Leu Gln Val Gln Leu
                725                 730                 735
Gln Leu Ser Thr Ser Ser His Gln Asp Asn Leu Trp Pro Met Ile Leu
            740                 745                 750
Thr Leu Leu Val Asp Tyr Thr Leu Gln Thr Ser Leu Ser Met Val Asn
        755                 760                 765

His Arg Leu Gln Ser Phe Phe Gly Gly Thr Val Met Gly Glu Ser Gly
    770                 775                 780
Met Lys Thr Val Glu Asp Val Gly Ser Pro Leu Lys Tyr Glu Phe Gln
785                 790                 795                 800
Val Gly Pro Met Gly Glu Gly Leu Val Gly Leu Gly Thr Leu Val Leu
                805                 810                 815
Gly Leu Glu Trp Pro Tyr Glu Val Ser Asn Gly Lys Trp Leu Leu Tyr
            820                 825                 830
Pro Thr Glu Ile Thr Val His Gly Asn Gly Ser Trp Pro Cys Arg Pro
        835                 840                 845
Pro Gly Asp Leu Ile Asn Pro Leu Asn Leu Thr Leu Ser Asp Pro Gly
    850                 855                 860
Asp Arg Pro Ser Ser Pro Gln Arg Arg Arg Arg Gln Leu Asp Pro Gly
865                 870                 875                 880
Gly Gly Gln Gly Pro Pro Pro Val Thr Leu Ala Ala Ala Lys Lys Ala
                885                 890                 895
Lys Ser Glu Thr Val Leu Thr Cys Ala Thr Gly Arg Ala His Cys Val
            900                 905                 910
Trp Leu Glu Cys Pro Ile Pro Asp Ala Pro Val Val Thr Asn Val Thr
        915                 920                 925
Val Lys Ala Arg Val Trp Asn Ser Thr Phe Ile Glu Asp Tyr Arg Asp
    930                 935                 940
Phe Asp Arg Val Arg Val Asn Gly Trp Ala Thr Leu Phe Leu Arg Thr
945                 950                 955                 960
Ser Ile Pro Thr Ile Asn Met Glu Asn Lys Thr Thr Trp Phe Ser Val
                965                 970                 975
Asp Ile Asp Ser Glu Leu Val Glu Glu Leu Pro Ala Glu Ile Glu Leu
            980                 985                 990
Trp Leu Val Leu Val Ala Val Gly Ala Gly Leu Leu Leu Leu Gly Leu
        995                 1000                1005
Ile Ile Leu Leu Leu Trp Lys Cys Asp Phe Phe Lys Arg Thr Arg Tyr
    1010                1015                1020
Tyr Gln Ile Met Pro Lys Tyr His Ala Val Arg Ile Arg Glu Glu Glu
1025                1030                1035                1040
Arg Tyr Pro Pro Pro Gly Ser Thr Leu Pro Thr Lys Lys His Trp Val
                1045                1050                1055
Thr Ser Trp Gln Thr Arg Asp Gln Tyr Tyr
            1060                1065

<210> 296
<211> 1527
<212> PRT
<213> Homo sapiens

<400> 296
Met Asp Ala Leu Cys Gly Ser Gly Glu Leu Gly Ser Lys Phe Trp Asp
 1               5                  10                  15
Ser Asn Leu Ser Val His Thr Glu Asn Pro Asp Leu Thr Pro Cys Phe
            20                  25                  30
Gln Asn Ser Leu Leu Ala Trp Val Pro Cys Ile Tyr Leu Trp Val Ala
        35                  40                  45
Leu Pro Cys Tyr Leu Leu Tyr Leu Arg His His Cys Arg Gly Tyr Ile
    50                  55                  60
Ile Leu Ser His Leu Ser Lys Leu Lys Met Val Leu Gly Val Leu Leu
65                  70                  75                  80
Trp Cys Val Ser Trp Ala Asp Leu Phe Tyr Ser Phe His Gly Leu Val
                85                  90                  95
His Gly Arg Ala Pro Ala Pro Val Phe Phe Val Thr Pro Leu Val Val
            100                 105                 110
Gly Val Thr Met Leu Leu Ala Thr Leu Leu Ile Gln Tyr Glu Arg Leu
        115                 120                 125
Gln Gly Val Gln Ser Ser Gly Val Leu Ile Ile Phe Trp Phe Leu Cys
    130                 135                 140

Val Val Cys Ala Ile Val Pro Phe Arg Ser Lys Ile Leu Leu Ala Lys
145                 150                 155                 160
Ala Glu Gly Glu Ile Ser Asp Pro Phe Arg Phe Thr Thr Phe Tyr Ile
                165                 170                 175
His Phe Ala Leu Val Leu Ser Ala Leu Ile Leu Ala Cys Phe Arg Glu
            180                 185                 190
Lys Pro Pro Phe Phe Ser Ala Lys Asn Val Asp Pro Asn Pro Tyr Pro
        195                 200                 205
Glu Thr Ser Ala Gly Phe Leu Ser Arg Leu Phe Phe Trp Trp Phe Thr
    210                 215                 220
Lys Met Ala Ile Tyr Gly Tyr Arg His Pro Leu Glu Glu Lys Asp Leu
225                 230                 235                 240
Trp Ser Leu Lys Glu Glu Asp Arg Ser Gln Met Val Val Gln Gln Leu
                245                 250                 255
Leu Glu Ala Trp Arg Lys Gln Glu Lys Gln Thr Ala Arg His Lys Ala
            260                 265                 270
Ser Ala Ala Pro Gly Lys Asn Ala Ser Gly Glu Asp Glu Val Leu Leu
        275                 280                 285
Gly Ala Arg Pro Arg Pro Arg Lys Pro Ser Phe Leu Lys Ala Leu Leu
    290                 295                 300
Ala Thr Phe Gly Ser Ser Phe Leu Ile Ser Ala Cys Phe Lys Leu Ile
305                 310                 315                 320
Gln Asp Leu Leu Ser Phe Ile Asn Pro Gln Leu Leu Ser Ile Leu Ile
                325                 330                 335
Arg Phe Ile Ser Asn Pro Met Ala Pro Ser Trp Trp Gly Phe Leu Val
            340                 345                 350
Ala Gly Leu Met Phe Leu Cys Ser Met Met Gln Ser Leu Ile Leu Gln
        355                 360                 365
His Tyr Tyr His Tyr Ile Phe Val Thr Gly Val Lys Phe Arg Thr Gly
    370                 375                 380
Ile Met Gly Val Ile Tyr Arg Lys Ala Leu Val Ile Thr Asn Ser Val
385                 390                 395                 400
Lys Arg Ala Ser Thr Val Gly Glu Ile Val Asn Leu Met Ser Val Asp
                405                 410                 415
Ala Gln Arg Phe Met Asp Leu Ala Pro Phe Leu Asn Leu Leu Trp Ser
            420                 425                 430
Ala Pro Leu Gln Ile Ile Leu Ala Ile Tyr Phe Leu Trp Gln Asn Leu
        435                 440                 445
Gly Pro Ser Val Leu Ala Gly Val Ala Phe Met Val Leu Leu Ile Pro
    450                 455                 460
Leu Asn Gly Ala Val Ala Val Lys Met Arg Ala Phe Gln Val Lys Gln
465                 470                 475                 480
Met Lys Leu Lys Asp Ser Arg Ile Lys Leu Met Ser Glu Ile Leu Asn
                485                 490                 495
Gly Ile Lys Val Leu Lys Leu Tyr Ala Trp Glu Pro Ser Phe Leu Lys
            500                 505                 510
Gln Val Glu Gly Ile Arg Gln Gly Glu Leu Gln Leu Leu Arg Thr Ala
        515                 520                 525
Ala Tyr Leu His Thr Thr Thr Thr Phe Thr Trp Met Cys Ser Pro Phe
    530                 535                 540
Leu Val Thr Leu Ile Thr Leu Trp Val Tyr Val Tyr Val Asp Pro Asn
545                 550                 555                 560
Asn Val Leu Asp Ala Glu Lys Ala Phe Val Ser Val Ser Leu Phe Asn
                565                 570                 575
Ile Leu Arg Leu Pro Leu Asn Met Leu Pro Gln Leu Ile Ser Asn Leu
            580                 585                 590
Thr Gln Ala Ser Val Ser Leu Lys Arg Ile Gln Gln Phe Leu Ser Gln
        595                 600                 605

Glu Glu Leu Asp Pro Gln Ser Val Glu Arg Lys Thr Ile Ser Pro Gly
    610                 615                 620
Tyr Ala Ile Thr Ile His Ser Gly Thr Phe Thr Trp Ala Gln Asp Leu
625                 630                 635                 640

Pro Pro Thr Leu His Ser Leu Asp Ile Gln Val Pro Lys Gly Ala Leu
                645                 650                 655
Val Ala Val Val Gly Pro Val Gly Cys Gly Lys Ser Ser Leu Val Ser
            660                 665                 670
Ala Leu Leu Gly Glu Met Glu Lys Leu Glu Gly Lys Val His Met Lys
        675                 680                 685
Gly Ser Val Ala Tyr Val Pro Gln Gln Ala Trp Ile Gln Asn Cys Thr
    690                 695                 700
Leu Gln Glu Asn Val Leu Phe Gly Lys Ala Leu Asn Pro Lys Arg Tyr
705                 710                 715                 720
Gln Gln Thr Leu Glu Ala Cys Ala Leu Leu Ala Asp Leu Glu Met Leu
                725                 730                 735
Pro Gly Gly Asp Gln Thr Glu Ile Gly Glu Lys Gly Ile Asn Leu Ser
            740                 745                 750
Gly Gly Gln Arg Gln Arg Val Ser Leu Ala Arg Ala Val Tyr Ser Asp
        755                 760                 765
Ala Asp Ile Phe Leu Leu Asp Asp Pro Leu Ser Ala Val Asp Ser His
    770                 775                 780
Val Ala Lys His Ile Phe Asp His Val Ile Gly Pro Glu Gly Val Leu
785                 790                 795                 800
Ala Gly Lys Thr Arg Val Leu Val Thr His Gly Ile Ser Phe Leu Pro
                805                 810                 815
Gln Thr Asp Phe Ile Ile Val Leu Ala Asp Gly Gln Val Ser Glu Met
            820                 825                 830
Gly Pro Tyr Pro Ala Leu Leu Gln Arg Asn Gly Ser Phe Ala Asn Phe
        835                 840                 845
Leu Cys Asn Tyr Ala Pro Asp Glu Asp Gln Gly His Leu Glu Asp Ser
    850                 855                 860
Trp Thr Ala Leu Glu Gly Ala Glu Asp Lys Glu Ala Leu Leu Ile Glu
865                 870                 875                 880
Asp Thr Leu Ser Asn His Thr Asp Leu Thr Asp Asn Asp Pro Val Thr
                885                 890                 895
Tyr Val Val Gln Lys Gln Phe Met Arg Gln Leu Ser Ala Leu Ser Ser
            900                 905                 910
Asp Gly Glu Gly Gln Gly Arg Pro Val Pro Arg Arg His Leu Gly Pro
        915                 920                 925
Ser Glu Lys Val Gln Val Thr Glu Ala Lys Ala Asp Gly Ala Leu Thr
    930                 935                 940
Gln Glu Glu Lys Ala Ala Ile Gly Thr Val Glu Leu Ser Val Phe Trp
945                 950                 955                 960
Asp Tyr Ala Lys Ala Val Gly Leu Cys Thr Thr Leu Ala Ile Cys Leu
                965                 970                 975
Leu Tyr Val Gly Gln Ser Ala Ala Ala Ile Gly Ala Asn Val Trp Leu
            980                 985                 990
Ser Ala Trp Thr Asn Asp Ala Met Ala Asp Ser Arg Gln Asn Asn Thr
        995                 1000                1005
Ser Leu Arg Leu Gly Val Tyr Ala Ala Leu Gly Ile Leu Gln Gly Phe
    1010                1015                1020
Leu Val Met Leu Ala Ala Met Ala Met Ala Ala Gly Gly Ile Gln Ala
1025                1030                1035                1040
Ala Arg Val Leu His Gln Ala Leu Leu His Asn Lys Ile Arg Ser Pro
                1045                1050                1055
Gln Ser Phe Phe Asp Thr Thr Pro Ser Gly Arg Ile Leu Asn Cys Phe
            1060                1065                1070
Ser Lys Asp Ile Tyr Val Val Asp Glu Val Leu Ala Pro Val Ile Leu
        1075                1080                1085

Met Leu Leu Asn Ser Phe Phe Asn Ala Ile Ser Thr Leu Val Val Ile
    1090                1095                1100
Met Ala Ser Thr Pro Leu Phe Thr Val Val Ile Leu Pro Leu Ala Val
1105                1110                1115                1120
Leu Tyr Thr Leu Val Gln Arg Phe Tyr Ala Ala Thr Ser Arg Gln Leu
                1125                1130                1135

Lys Arg Leu Glu Ser Val Ser Arg Ser Pro Ile Tyr Ser His Phe Ser
            1140                1145                1150
Glu Thr Val Thr Gly Ala Ser Val Ile Arg Ala Tyr Asn Arg Ser Arg
        1155                1160                1165
Asp Phe Glu Ile Ile Ser Asp Thr Lys Val Asp Ala Asn Gln Arg Ser
    1170                1175                1180
Cys Tyr Pro Tyr Ile Ile Ser Asn Arg Trp Leu Ser Ile Gly Val Glu
1185                1190                1195                1200
Phe Val Gly Asn Cys Val Val Leu Phe Ala Ala Leu Phe Ala Val Ile
                1205                1210                1215
Gly Arg Ser Ser Leu Asn Pro Gly Leu Val Gly Leu Ser Val Ser Tyr
            1220                1225                1230
Ser Leu Gln Val Thr Phe Ala Leu Asn Trp Met Ile Arg Met Met Ser
        1235                1240                1245
Asp Leu Glu Ser Asn Ile Val Ala Val Glu Arg Val Lys Glu Tyr Ser
    1250                1255                1260
Lys Thr Glu Thr Glu Ala Pro Trp Val Val Glu Gly Ser Arg Pro Pro
1265                1270                1275                1280
Glu Gly Trp Pro Pro Arg Gly Glu Val Glu Phe Arg Asn Tyr Ser Val
                1285                1290                1295
Arg Tyr Arg Pro Gly Leu Asp Leu Val Leu Arg Asp Leu Ser Leu His
            1300                1305                1310
Val His Gly Gly Glu Lys Val Gly Ile Val Gly Arg Thr Gly Ala Gly
        1315                1320                1325
Lys Ser Ser Met Thr Leu Cys Leu Phe Arg Ile Leu Glu Ala Ala Lys
    1330                1335                1340
Gly Glu Ile Arg Ile Asp Gly Leu Asn Val Ala Asp Ile Gly Leu His
1345                1350                1355                1360
Asp Leu Arg Ser Gln Leu Thr Ile Ile Pro Gln Asp Pro Ile Leu Phe
                1365                1370                1375
Ser Gly Thr Leu Arg Met Asn Leu Asp Pro Phe Gly Ser Tyr Ser Glu
            1380                1385                1390
Glu Asp Ile Trp Trp Ala Leu Glu Leu Ser His Leu His Thr Phe Val
        1395                1400                1405
Ser Ser Gln Pro Ala Gly Leu Asp Phe Gln Cys Ser Glu Gly Gly Glu
    1410                1415                1420
Asn Leu Ser Val Gly Gln Arg Gln Leu Val Cys Leu Ala Arg Ala Leu
1425                1430                1435                1440
Leu Arg Lys Ser Arg Ile Leu Val Leu Asp Glu Ala Thr Ala Ala Ile
                1445                1450                1455
Asp Leu Glu Thr Asp Asn Leu Ile Gln Ala Thr Ile Arg Thr Gln Phe
            1460                1465                1470
Asp Thr Cys Thr Val Leu Thr Ile Ala His Arg Leu Asn Thr Ile Met
        1475                1480                1485
Asp Tyr Thr Arg Val Leu Val Leu Asp Lys Gly Val Val Ala Glu Phe
    1490                1495                1500
Asp Ser Pro Ala Asn Leu Ile Ala Ala Arg Gly Ile Phe Tyr Gly Met
1505                1510                1515                1520
Ala Arg Asp Ala Gly Leu Ala
                1525

<210> 297
<211> 265
<212> PRT
<213> Homo sapiens
<400> 297
Met His Phe Tyr Arg Leu Phe Leu Gly Ala Thr Arg Arg Phe Leu Asn
1               5                   10                  15
Pro Glu Trp Lys Gly Glu Ile Asp Asn Trp Cys Val Tyr Val Leu Thr
            20                  25                  30
Ser Leu Leu Pro Phe Lys Ile Gln Ser Gln Asp Ile Lys Ala Leu Gln
        35                  40                  45
Lys Glu Leu Glu Gln Phe Ala Lys Leu Leu Lys Gln Lys Arg Ile Thr

50                  55                  60
Leu Gly Tyr Thr Gln Ala Asp Val Gly Leu Thr Leu Gly Val Leu Phe
65                  70                  75                  80
Gly Lys Val Phe Ser Gln Thr Thr Ile Cys Arg Phe Glu Ala Leu Gln
                85                  90                  95
Leu Ser Phe Lys Asn Met Cys Lys Leu Arg Pro Leu Leu Gln Lys Trp
            100                 105                 110
Val Glu Glu Ala Asp Asn Asn Glu Asn Leu Gln Glu Ile Cys Lys Ala
        115                 120                 125
Glu Thr Leu Val Gln Ala Arg Lys Arg Lys Arg Thr Ser Ile Glu Asn
    130                 135                 140
Arg Val Arg Gly Asn Leu Glu Asn Leu Phe Leu Gln Cys Pro Lys Pro
145                 150                 155                 160
Thr Leu Gln Gln Ile Ser His Ile Ala Gln Gln Leu Gly Leu Glu Lys
                165                 170                 175
Asp Val Val Arg Val Trp Phe Cys Asn Arg Arg Gln Lys Gly Lys Arg
            180                 185                 190
Ser Ser Ser Asp Tyr Ala Gln Arg Glu Asp Phe Glu Ala Ala Gly Ser
        195                 200                 205
Pro Phe Ser Gly Gly Pro Val Ser Phe Pro Leu Ala Pro Gly Pro His
    210                 215                 220
Phe Gly Ala Pro Gly Tyr Gly Ser Pro His Phe Thr Ala Leu Tyr Ser
225                 230                 235                 240
Ser Val Pro Phe Pro Glu Gly Glu Ala Phe Pro Pro Val Ser Val Thr
                245                 250                 255
Thr Leu Gly Ser Pro Leu His Ser Asn
            260                 265

<210> 298
<211> 941
<212> PRT
<213> Homo sapiens

<400> 298
Met Ala Ser Pro Arg Arg Ser Gly Gln Pro Gly Arg Pro Pro Pro Pro
1               5                   10                  15
Pro Pro Pro Pro Ala Arg Leu Leu Leu Leu Leu Leu Leu Pro Leu Leu
            20                  25                  30
Leu Pro Leu Ala Pro Gly Ala Trp Gly Trp Ala Arg Gly Ala Pro Arg
        35                  40                  45
Pro Pro Pro Ser Ser Pro Pro Leu Ser Ile Met Gly Leu Met Pro Leu
    50                  55                  60
Thr Lys Glu Val Ala Lys Gly Ser Ile Gly Arg Gly Val Leu Pro Ala
65                  70                  75                  80
Val Glu Leu Ala Ile Glu Gln Ile Arg Asn Glu Ser Leu Leu Arg Pro
                85                  90                  95
Tyr Phe Leu Asp Leu Arg Leu Tyr Asp Thr Glu Cys Asp Asn Ala Lys
            100                 105                 110
Gly Leu Lys Ala Phe Tyr Asp Ala Ile Lys Tyr Gly Pro Asn His Leu
        115                 120                 125
Met Val Phe Gly Gly Val Cys Pro Ser Val Thr Ser Ile Ile Ala Glu
    130                 135                 140

Ser Leu Gln Gly Trp Asn Leu Val Gln Leu Ser Phe Ala Ala Thr Thr
145                 150                 155                 160
Pro Val Leu Ala Asp Lys Lys Lys Tyr Pro Tyr Phe Phe Arg Thr Val
                165                 170                 175
Pro Ser Asp Asn Ala Val Asn Pro Ala Ile Leu Lys Leu Leu Lys His
            180                 185                 190
Tyr Gln Trp Lys Arg Val Gly Thr Leu Thr Gln Asp Val Gln Arg Phe
        195                 200                 205
Ser Glu Val Arg Asn Asp Leu Thr Gly Val Leu Tyr Gly Glu Asp Ile
    210                 215                 220

Glu Ile Ser Asp Thr Glu Ser Phe Ser Asn Asp Pro Cys Thr Ser Val
225                 230                 235                 240
Lys Lys Leu Lys Gly Asn Asp Val Arg Ile Ile Leu Gly Gln Phe Asp
                245                 250                 255
Gln Asn Met Ala Ala Lys Val Phe Cys Cys Ala Tyr Glu Glu Asn Met
            260                 265                 270
Tyr Gly Ser Lys Tyr Gln Trp Ile Ile Pro Gly Trp Tyr Glu Pro Ser
        275                 280                 285
Trp Trp Glu Gln Val His Thr Glu Ala Asn Ser Ser Arg Cys Leu Arg
    290                 295                 300
Lys Asn Leu Leu Ala Ala Met Glu Gly Tyr Ile Gly Val Asp Phe Glu
305                 310                 315                 320
Pro Leu Ser Ser Lys Gln Ile Lys Thr Ile Ser Gly Lys Thr Pro Gln
                325                 330                 335
Gln Tyr Glu Arg Glu Tyr Asn Asn Lys Arg Ser Gly Val Gly Pro Ser
            340                 345                 350
Lys Phe His Gly Tyr Ala Tyr Asp Gly Ile Trp Val Ile Ala Lys Thr
        355                 360                 365
Leu Gln Arg Ala Met Glu Thr Leu His Ala Ser Ser Arg His Gln Arg
    370                 375                 380
Ile Gln Asp Phe Asn Tyr Thr Asp His Thr Leu Gly Arg Ile Ile Leu
385                 390                 395                 400
Asn Ala Met Asn Glu Thr Asn Phe Phe Gly Val Thr Gly Gln Val Val
                405                 410                 415
Phe Arg Asn Gly Glu Arg Met Gly Thr Ile Lys Phe Thr Gln Phe Gln
            420                 425                 430
Asp Ser Arg Glu Val Lys Val Gly Glu Tyr Asn Ala Val Ala Asp Thr
        435                 440                 445
Leu Glu Ile Ile Asn Asp Thr Ile Arg Phe Gln Gly Ser Glu Pro Pro
    450                 455                 460
Lys Asp Lys Thr Ile Ile Leu Glu Gln Leu Arg Lys Ile Ser Leu Pro
465                 470                 475                 480
Leu Tyr Ser Ile Leu Ser Ala Leu Thr Ile Leu Gly Met Ile Met Ala
                485                 490                 495
Ser Ala Phe Leu Phe Phe Asn Ile Lys Asn Arg Asn Gln Lys Leu Ile
            500                 505                 510
Lys Met Ser Ser Pro Tyr Met Asn Asn Leu Ile Ile Leu Gly Gly Met
        515                 520                 525
Leu Ser Tyr Ala Ser Ile Phe Leu Phe Gly Leu Asp Gly Ser Phe Val
    530                 535                 540
Ser Glu Lys Thr Phe Glu Thr Leu Cys Thr Val Arg Thr Trp Ile Leu
545                 550                 555                 560
Thr Val Gly Tyr Thr Thr Ala Phe Gly Ala Met Phe Ala Lys Thr Trp
                565                 570                 575
Arg Val His Ala Ile Phe Lys Asn Val Lys Met Lys Lys Lys Ile Ile
            580                 585                 590
Lys Asp Gln Lys Leu Leu Val Ile Val Gly Gly Met Leu Leu Ile Asp
        595                 600                 605
Leu Cys Ile Leu Ile Cys Trp Gln Ala Val Asp Pro Leu Arg Arg Thr
    610                 615                 620

Val Glu Lys Tyr Ser Met Glu Pro Asp Pro Ala Gly Arg Asp Ile Ser
625                 630                 635                 640
Ile Arg Pro Leu Leu Glu His Cys Glu Asn Thr His Met Thr Ile Trp
                645                 650                 655
Leu Gly Ile Val Tyr Ala Tyr Lys Gly Leu Leu Met Leu Phe Gly Cys
            660                 665                 670
Phe Leu Ala Trp Glu Thr Arg Asn Val Ser Ile Pro Ala Leu Asn Asp
        675                 680                 685
Ser Lys Tyr Ile Gly Met Ser Val Tyr Asn Val Gly Ile Met Cys Ile
    690                 695                 700
Ile Gly Ala Ala Val Ser Phe Leu Thr Arg Asp Gln Pro Asn Val Gln
705                 710                 715                 720

Phe Cys Ile Val Ala Leu Val Ile Ile Phe Cys Ser Thr Ile Thr Leu
                725                 730                 735
Cys Leu Val Phe Val Pro Lys Leu Ile Thr Leu Arg Thr Asn Pro Asp
            740                 745                 750
Ala Ala Thr Gln Asn Arg Arg Phe Gln Phe Thr Gln Asn Gln Lys Lys
        755                 760                 765
Glu Asp Ser Lys Thr Ser Thr Ser Val Thr Ser Val Asn Gln Ala Ser
    770                 775                 780
Thr Ser Arg Leu Glu Gly Leu Gln Ser Glu Asn His Arg Leu Arg Met
785                 790                 795                 800
Lys Ile Thr Glu Leu Asp Lys Asp Leu Glu Glu Val Thr Met Gln Leu
                805                 810                 815
Gln Asp Thr Pro Glu Lys Thr Thr Tyr Ile Lys Gln Asn His Tyr Gln
            820                 825                 830
Glu Leu Asn Asp Ile Leu Asn Leu Gly Asn Phe Thr Glu Ser Thr Asp
        835                 840                 845
Gly Gly Lys Ala Ile Leu Lys Asn His Leu Asp Gln Asn Pro Gln Leu
    850                 855                 860
Gln Trp Asn Thr Thr Glu Pro Ser Arg Thr Cys Lys Asp Pro Ile Glu
865                 870                 875                 880
Asp Ile Asn Ser Pro Glu His Ile Gln Arg Arg Leu Ser Leu Gln Leu
                885                 890                 895
Pro Ile Leu His His Ala Tyr Leu Pro Ser Ile Gly Gly Val Asp Ala
            900                 905                 910
Ser Cys Val Ser Pro Cys Val Ser Pro Thr Ala Ser Pro Arg His Arg
        915                 920                 925
His Val Pro Pro Ser Phe Arg Val Met Val Ser Gly Leu
    930                 935                 940

<210> 299
<211> 811
<212> PRT
<213> Homo sapiens

<400> 299
Met Glu Ala Ala Arg Ala Leu Arg Leu Leu Leu Val Val Cys Gly Cys
 1               5                  10                  15
Leu Ala Leu Pro Pro Leu Ala Glu Pro Val Cys Pro Glu Arg Cys Asp
            20                  25                  30
Cys Gln His Pro Gln His Leu Leu Cys Thr Asn Arg Gly Leu Arg Val
        35                  40                  45
Val Pro Lys Thr Ser Ser Leu Pro Ser Pro His Asp Val Leu Thr Tyr
    50                  55                  60
Ser Leu Gly Gly Asn Phe Ile Thr Asn Ile Thr Ala Phe Asp Phe His
65                  70                  75                  80
Arg Leu Gly Gln Leu Arg Arg Leu Asp Leu Gln Tyr Asn Gln Ile Arg
                85                  90                  95
Ser Leu His Pro Lys Thr Phe Glu Lys Leu Ser Arg Leu Glu Glu Leu
            100                 105                 110
Tyr Leu Gly Asn Asn Leu Leu Gln Ala Leu Ala Pro Gly Thr Leu Ala
        115                 120                 125
Pro Leu Arg Lys Leu Arg Ile Leu Tyr Ala Asn Gly Asn Glu Ile Ser
    130                 135                 140
Arg Leu Ser Arg Gly Ser Phe Glu Gly Leu Glu Ser Leu Val Lys Leu
145                 150                 155                 160
Arg Leu Asp Gly Asn Ala Leu Gly Ala Leu Pro Asp Ala Val Phe Ala
                165                 170                 175
Pro Leu Gly Asn Leu Leu Tyr Leu His Leu Glu Ser Asn Arg Ile Arg
            180                 185                 190
Phe Leu Gly Lys Asn Ala Phe Ala Gln Leu Gly Lys Leu Arg Phe Leu
        195                 200                 205
Asn Leu Ser Ala Asn Glu Leu Gln Pro Ser Leu Arg His Ala Ala Thr
    210                 215                 220

Phe Ala Pro Leu Arg Ser Leu Ser Ser Leu Ile Leu Ser Ala Asn Ser
225                 230                 235                 240
Leu Gln His Leu Gly Pro Arg Ile Phe Gln His Leu Pro Arg Leu Gly
                245                 250                 255
Leu Leu Ser Leu Arg Gly Asn Gln Leu Thr His Leu Ala Pro Glu Ala
            260                 265                 270
Phe Trp Gly Leu Glu Ala Leu Arg Glu Leu Arg Leu Glu Gly Asn Arg
        275                 280                 285
Leu Ser Gln Leu Pro Thr Ala Leu Leu Glu Pro Leu His Ser Leu Glu
    290                 295                 300
Ala Leu Asp Leu Ser Gly Asn Glu Leu Ser Ala Leu His Pro Ala Thr
305                 310                 315                 320
Phe Gly His Leu Gly Arg Leu Arg Glu Leu Ser Leu Arg Asn Asn Ala
                325                 330                 335
Leu Ser Ala Leu Ser Gly Asp Ile Phe Ala Ala Ser Pro Ala Leu Tyr
            340                 345                 350
Arg Leu Asp Leu Asp Gly Asn Gly Trp Thr Cys Asp Cys Arg Leu Arg
        355                 360                 365
Gly Leu Lys Arg Trp Met Gly Asp Trp His Ser Gln Gly Arg Leu Leu
    370                 375                 380
Thr Val Phe Val Gln Cys Arg His Pro Pro Ala Leu Arg Gly Lys Tyr
385                 390                 395                 400
Leu Asp Tyr Leu Asp Asp Gln Gln Leu Gln Asn Gly Ser Cys Ala Asp
                405                 410                 415
Pro Ser Pro Ser Ala Ser Leu Thr Ala Asp Arg Arg Arg Gln Pro Leu
            420                 425                 430
Pro Thr Ala Ala Gly Glu Glu Met Thr Pro Pro Ala Gly Leu Ala Glu
        435                 440                 445
Glu Leu Pro Pro Gln Pro Gln Leu Gln Gln Gln Gly Arg Phe Leu Ala
    450                 455                 460
Gly Val Ala Trp Asp Gly Ala Ala Arg Glu Leu Val Gly Asn Arg Ser
465                 470                 475                 480
Ala Leu Arg Leu Ser Arg Arg Gly Pro Gly Leu Gln Gln Pro Ser Pro
                485                 490                 495
Ser Val Ala Ala Ala Ala Gly Pro Ala Pro Gln Ser Leu Asp Leu His
            500                 505                 510
Lys Lys Pro Gln Arg Gly Arg Pro Thr Arg Ala Asp Pro Ala Leu Ala
        515                 520                 525
Glu Pro Thr Pro Thr Ala Ser Pro Gly Ser Ala Pro Ser Pro Ala Gly
    530                 535                 540
Asp Pro Trp Gln Arg Ala Thr Lys His Arg Leu Gly Thr Glu His Gln
545                 550                 555                 560
Glu Arg Ala Ala Gln Ser Asp Gly Gly Ala Gly Leu Pro Pro Leu Val
                565                 570                 575
Ser Asp Pro Cys Asp Phe Asn Lys Phe Ile Leu Cys Asn Leu Thr Val
            580                 585                 590

Glu Ala Val Gly Ala Asp Ser Ala Ser Val Arg Trp Ala Val Arg Glu
        595                 600                 605
His Arg Ser Pro Arg Pro Leu Gly Gly Ala Arg Phe Arg Leu Leu Phe
    610                 615                 620
Asp Arg Phe Gly Gln Gln Pro Lys Phe His Arg Phe Val Tyr Leu Pro
625                 630                 635                 640
Glu Ser Ser Asp Ser Ala Thr Leu Arg Glu Leu Arg Gly Asp Thr Pro
                645                 650                 655
Tyr Leu Val Cys Val Glu Gly Val Leu Gly Gly Arg Val Cys Pro Val
            660                 665                 670
Ala Pro Arg Asp His Cys Ala Gly Leu Val Thr Leu Pro Glu Ala Gly
        675                 680                 685
Ser Arg Gly Gly Val Asp Tyr Gln Leu Leu Thr Leu Ala Leu Leu Thr
    690                 695                 700
Val Asn Ala Leu Leu Val Leu Leu Ala Leu Ala Ala Trp Ala Ser Arg
705                 710                 715                 720

Trp Leu Arg Arg Lys Leu Arg Ala Arg Arg Lys Gly Gly Ala Pro Val
                725                 730                 735
His Val Arg His Met Tyr Ser Thr Arg Arg Pro Leu Arg Ser Met Gly
            740                 745                 750
Thr Gly Val Ser Ala Asp Phe Ser Gly Phe Gln Ser His Arg Pro Arg
        755                 760                 765
Thr Thr Val Cys Ala Leu Ser Glu Ala Asp Leu Ile Glu Phe Pro Cys
    770                 775                 780
Asp Arg Phe Met Asp Ser Ala Gly Gly Gly Ala Gly Gly Ser Leu Arg
785                 790                 795                 800
Arg Glu Asp Arg Leu Leu Gln Arg Phe Ala Asp
                805                 810

<210> 300
<211> 906
<212> PRT
<213> Homo sapiens

<400> 300

<210> 301
<211> 618
<212> PRT
<213> Homo sapiens

<400> 301

<210> 302
<211> 136
<212> PRT
<213> Homo sapiens

<400> 302

<210> 303
<211> 145
<212> PRT
<213> Homo sapiens

<400> 303
Met Gly Cys Gly Gly Ser Arg Ala Asp Ala Ile Glu Pro Arg Tyr Tyr

1               5                   10                  15
Glu Ser Trp Thr Arg Glu Thr Glu Ser Thr Trp Leu Thr Tyr Thr Asp
            20                  25                  30
Ser Asp Ala Pro Pro Ser Ala Ala Ala Pro Asp Ser Gly Pro Glu Ala
        35                  40                  45
Gly Gly Leu His Ser Gly Met Leu Glu Asp Gly Leu Pro Ser Asn Gly
    50                  55                  60
Val Pro Arg Ser Thr Ala Pro Gly Gly Ile Pro Asn Pro Glu Lys Lys
65                  70                  75                  80
Thr Asn Cys Glu Thr Gln Cys Pro Asn Pro Gln Ser Leu Ser Ser Gly
                85                  90                  95
Pro Leu Thr Gln Lys Gln Asn Gly Leu Gln Thr Thr Glu Ala Lys Arg
            100                 105                 110
Asp Ala Lys Arg Met Pro Ala Lys Glu Val Thr Ile Asn Val Thr Asp
        115                 120                 125
Ser Ile Gln Gln Met Asp Arg Ser Arg Arg Ile Thr Lys Asn Cys Val
    130                 135                 140
Asn
145

<210> 304
<211> 1358
<212> PRT
<213> Homo sapiens

<400> 304

<210> 305
<211> 2303
<212> PRT
<213> Homo sapiens

<400> 305
Met Ala Ser Ser Val Ala Pro Tyr Glu Gln Leu Val Arg Gln Val Glu
 1               5                  10                  15
Ala Leu Lys Ala Glu Asn Ser His Leu Arg Gln Glu Leu Arg Asp Asn
            20                  25                  30
Ser Ser His Leu Ser Lys Leu Glu Thr Glu Thr Ser Gly Met Lys Glu
        35                  40                  45
Val Leu Lys His Leu Gln Gly Lys Leu Glu Gln Glu Ala Arg Val Leu
    50                  55                  60
Val Ser Ser Gly Gln Thr Glu Val Leu Glu Gln Leu Lys Ala Leu Gln
65                  70                  75                  80
Met Asp Ile Thr Ser Leu Tyr Asn Leu Lys Phe Gln Pro Pro Thr Leu
                85                  90                  95
Gly Pro Glu Pro Ala Ala Arg Thr Pro Glu Gly Ser Pro Val His Gly
            100                 105                 110
Ser Gly Pro Ser Lys Asp Ser Phe Gly Glu Leu Ser Arg Ala Thr Ile
        115                 120                 125
Arg Leu Leu Glu Glu Leu Asp Arg Glu Arg Cys Phe Leu Leu Asn Glu
    130                 135                 140
Ile Glu Lys Glu Glu Lys Glu Lys Leu Trp Tyr Tyr Ser Gln Leu Gln
145                 150                 155                 160
Gly Leu Ser Lys Arg Leu Asp Glu Leu Pro His Val Glu Thr Gln Phe
                165                 170                 175
Ser Met Gln Met Asp Leu Ile Arg Gln Gln Leu Glu Phe Glu Ala Gln
            180                 185                 190
His Ile Arg Ser Leu Met Glu Glu Arg Phe Gly Thr Ser Asp Glu Met
        195                 200                 205
Val Gln Arg Ala Gln Ile Arg Ala Ser Arg Leu Glu Gln Ile Asp Lys
    210                 215                 220
Glu Leu Leu Glu Ala Gln Asp Arg Val Gln Gln Thr Glu Pro Gln Ala
225                 230                 235                 240
Leu Leu Ala Val Lys Ser Val Pro Val Asp Glu Asp Pro Glu Thr Glu
                245                 250                 255
Val Pro Thr His Pro Glu Asp Gly Thr Pro Gln Pro Gly Asn Ser Lys
            260                 265                 270
Val Glu Val Val Phe Trp Leu Leu Ser Met Leu Ala Thr Arg Asp Gln
        275                 280                 285
Glu Asp Thr Ala Arg Thr Leu Leu Ala Met Ser Ser Ser Pro Glu Ser
    290                 295                 300
Cys Val Ala Met Arg Arg Ser Gly Cys Leu Pro Leu Leu Leu Gln Ile
305                 310                 315                 320
Leu His Gly Thr Glu Ala Ala Ala Gly Gly Arg Ala Gly Ala Pro Gly
                325                 330                 335
Ala Pro Gly Ala Lys Asp Ala Arg Met Arg Ala Asn Ala Ala Leu His
            340                 345                 350
Asn Ile Val Phe Ser Gln Pro Asp Gln Gly Leu Ala Arg Lys Glu Met
        355                 360                 365

Arg Val Leu His Val Leu Glu Gln Ile Arg Ala Tyr Cys Glu Thr Cys
    370                 375                 380
Trp Asp Trp Leu Gln Ala Arg Asp Gly Gly Pro Glu Gly Gly Gly Ala
385                 390                 395                 400
Gly Ser Ala Pro Ile Pro Ile Glu Pro Gln Ile Cys Gln Ala Thr Cys
                405                 410                 415
Ala Val Met Lys Leu Ser Phe Asp Glu Glu Tyr Arg Arg Ala Met Asn
            420                 425                 430
Glu Leu Gly Gly Leu Gln Ala Val Ala Glu Leu Leu Gln Val Asp Tyr
        435                 440                 445
Glu Met His Lys Met Thr Arg Asp Pro Leu Asn Leu Ala Leu Arg Arg
    450                 455                 460
Tyr Ala Gly Met Thr Leu Thr Asn Leu Thr Phe Gly Asp Val Ala Asn
465                 470                 475                 480
Lys Ala Thr Leu Cys Ala Arg Arg Gly Cys Met Glu Ala Ile Val Ala
                485                 490                 495
Gln Leu Ala Ser Asp Ser Glu Glu Leu His Gln Val Val Ser Ser Ile
            500                 505                 510
Leu Arg Asn Leu Ser Trp Arg Ala Asp Ile Asn Ser Lys Lys Val Leu
        515                 520                 525
Arg Glu Ala Gly Ser Val Thr Ala Leu Val Gln Cys Val Leu Arg Ala
    530                 535                 540
Thr Lys Glu Ser Thr Leu Lys Ser Val Leu Ser Ala Leu Trp Asn Leu
545                 550                 555                 560
Ser Ala His Ser Thr Glu Asn Lys Ala Ala Ile Cys Gln Val Asp Gly
                565                 570                 575
Ala Leu Gly Phe Leu Val Ser Thr Leu Thr Tyr Lys Cys Gln Ser Asn
            580                 585                 590

Ser Leu Ala Ile Ile Glu Ser Gly Gly Gly Ile Leu Arg Asn Val Ser
        595                 600                 605
Ser Leu Val Ala Thr Arg Glu Asp Tyr Arg Gln Val Leu Arg Asp His
    610                 615                 620
Asn Cys Leu Gln Thr Leu Leu Gln His Leu Thr Ser His Ser Leu Thr
625                 630                 635                 640
Ile Val Ser Asn Ala Cys Gly Thr Leu Trp Asn Leu Ser Ala Arg Ser
                645                 650                 655
Ala Arg Asp Gln Glu Leu Leu Trp Asp Leu Gly Ala Val Gly Met Leu
            660                 665                 670
Arg Asn Leu Val His Ser Lys His Lys Met Ile Ala Met Gly Ser Ala
        675                 680                 685
Ala Ala Leu Arg Asn Leu Leu Ala His Arg Pro Ala Lys His Gln Ala
    690                 695                 700
Ala Ala Thr Ala Val Ser Pro Gly Ser Cys Val Pro Ser Leu Tyr Val
705                 710                 715                 720
Arg Lys Gln Arg Ala Leu Glu Ala Glu Leu Asp Ala Arg His Leu Ala
                725                 730                 735
Gln Ala Leu Glu His Leu Glu Lys Gln Gly Pro Pro Ala Ala Glu Ala
            740                 745                 750
Ala Thr Lys Lys Pro Leu Pro Pro Leu Arg His Leu Asp Gly Leu Ala
        755                 760                 765
Gln Asp Tyr Ala Ser Asp Ser Gly Cys Phe Asp Asp Asp Asp Ala Pro
    770                 775                 780
Ser Ser Leu Ala Ala Ala Ala Ala Thr Gly Glu Pro Ala Ser Pro Ala
785                 790                 795                 800
Ala Leu Ser Leu Phe Leu Gly Ser Pro Phe Leu Gln Gly Gln Ala Leu
                805                 810                 815
Ala Arg Thr Pro Pro Thr Arg Arg Gly Gly Lys Glu Ala Glu Lys Asp
            820                 825                 830
Thr Ser Gly Glu Ala Ala Val Ala Ala Lys Ala Lys Ala Lys Leu Ala
        835                 840                 845
Leu Ala Val Ala Arg Ile Asp Gln Leu Val Glu Asp Ile Ser Ala Leu
    850                 855                 860

His Thr Ser Ser Asp Asp Ser Phe Ser Leu Ser Ser Gly Asp Pro Gly
865                 870                 875                 880
Gln Glu Ala Pro Arg Glu Gly Arg Ala Gln Ser Cys Ser Pro Cys Arg
                885                 890                 895
Gly Pro Glu Gly Gly Arg Arg Glu Ala Gly Ser Arg Ala His Pro Leu
            900                 905                 910
Leu Arg Leu Lys Ala Ala His Ala Ser Leu Ser Asn Asp Ser Leu Asn
        915                 920                 925
Ser Gly Ser Ala Ser Asp Gly Tyr Cys Pro Arg Glu His Met Leu Pro
    930                 935                 940
Cys Pro Leu Ala Ala Leu Ala Ser Arg Arg Glu Asp Pro Arg Cys Gly
945                 950                 955                 960
Gln Pro Arg Pro Ser Arg Leu Asp Leu Asp Leu Pro Gly Cys Gln Ala
                965                 970                 975
Glu Pro Pro Ala Arg Glu Ala Thr Ser Ala Asp Ala Arg Val Arg Thr
            980                 985                 990
Ile Lys Leu Ser Pro Thr Tyr Gln His Val Pro Leu Leu Glu Gly Ala
        995                 1000                1005
Ser Arg Ala Gly Ala Glu Pro Leu Ala Gly Pro Gly Ile Ser Pro Gly
    1010                1015                1020
Ala Arg Lys Gln Ala Trp Leu Pro Ala Asp His Leu Ser Lys Val Pro
1025                1030                1035                1040
Glu Lys Leu Ala Ala Ala Pro Leu Ser Val Ala Ser Lys Ala Leu Gln
                1045                1050                1055
Lys Leu Ala Ala Gln Glu Gly Pro Leu Ser Leu Ser Arg Cys Ser Ser
            1060                1065                1070

Leu Ser Ser Leu Ser Ser Ala Gly Arg Pro Gly Pro Ser Glu Gly Gly
        1075                1080                1085
Asp Leu Asp Asp Ser Asp Ser Ser Leu Glu Gly Leu Glu Glu Ala Gly
    1090                1095                1100
Pro Ser Glu Ala Glu Leu Asp Ser Thr Trp Arg Ala Pro Gly Ala Thr
1105                1110                1115                1120
Ser Leu Pro Val Ala Ile Pro Ala Pro Arg Arg Asn Arg Gly Arg Gly
                1125                1130                1135
Leu Gly Val Glu Asp Ala Thr Pro Ser Ser Ser Ser Glu Asn Tyr Val
            1140                1145                1150
Gln Glu Thr Pro Leu Val Leu Ser Arg Cys Ser Ser Val Ser Ser Leu
        1155                1160                1165
Gly Ser Phe Glu Ser Pro Ser Ile Ala Ser Ser Ile Pro Ser Glu Pro
    1170                1175                1180
Cys Ser Gly Gln Gly Ser Gly Thr Ile Ser Pro Ser Glu Leu Pro Asp
1185                1190                1195                1200
Ser Pro Gly Gln Thr Met Pro Pro Ser Arg Ser Lys Thr Pro Pro Leu
                1205                1210                1215
Ala Pro Ala Pro Gln Gly Pro Pro Glu Ala Thr Gln Phe Ser Leu Gln
            1220                1225                1230
Trp Glu Ser Tyr Val Lys Arg Phe Leu Asp Ile Ala Asp Cys Arg Glu
        1235                1240                1245
Arg Cys Arg Leu Pro Ser Glu Leu Asp Ala Gly Ser Val Arg Phe Thr
    1250                1255                1260
Val Glu Lys Pro Asp Glu Asn Phe Ser Cys Ala Ser Ser Leu Ser Ala
1265                1270                1275                1280
Leu Ala Leu His Glu His Tyr Val Gln Gln Asp Val Glu Leu Arg Leu
                1285                1290                1295
Leu Pro Ser Ala Cys Pro Glu Arg Gly Gly Gly Ala Gly Gly Ala Gly
            1300                1305                1310
Leu His Phe Ala Gly His Arg Arg Arg Glu Glu Gly Pro Ala Pro Thr
        1315                1320                1325
Gly Ser Arg Pro Arg Gly Ala Ala Asp Gln Glu Leu Glu Leu Leu Arg
    1330                1335                1340
Glu Cys Leu Gly Ala Ala Val Pro Ala Arg Leu Arg Lys Val Ala Ser
1345                1350                1355                1360

Ala Leu Val Pro Gly Arg Arg Ala Leu Pro Val Pro Val Tyr Met Leu
                1365                1370                1375
Val Pro Ala Pro Ala Pro Ala Gln Glu Asp Asp Ser Cys Thr Asp Ser
            1380                1385                1390
Ala Glu Gly Thr Pro Val Asn Phe Ser Ser Ala Ala Ser Leu Ser Asp
        1395                1400                1405
Glu Thr Leu Gln Gly Pro Pro Arg Asp Gln Pro Gly Gly Pro Ala Gly
    1410                1415                1420
Arg Gln Arg Pro Thr Gly Arg Pro Thr Ser Ala Arg Gln Ala Met Gly
1425                1430                1435                1440
His Arg His Lys Ala Gly Gly Ala Gly Arg Ser Ala Glu Gln Ser Arg
                1445                1450                1455
Gly Ala Gly Lys Asn Arg Ala Gly Leu Glu Leu Pro Leu Gly Arg Pro
            1460                1465                1470
Pro Ser Ala Pro Ala Asp Lys Asp Gly Ser Lys Pro Gly Arg Thr Arg
        1475                1480                1485
Gly Asp Gly Ala Leu Gln Ser Leu Cys Leu Thr Thr Pro Thr Glu Glu
    1490                1495                1500
Ala Val Tyr Cys Phe Tyr Gly Asn Asp Ser Asp Glu Glu Pro Pro Ala
1505                1510                1515                1520
Ala Ala Pro Thr Pro Thr His Arg Arg Thr Ser Ala Ile Pro Arg Ala
                1525                1530                1535
Phe Thr Arg Glu Arg Pro Gln Gly Arg Lys Glu Ala Pro Ala Pro Ser
            1540                1545                1550

Lys Ala Ala Pro Ala Ala Pro Pro Pro Ala Arg Thr Gln Pro Ser Leu
        1555                1560                1565
Ile Ala Asp Glu Thr Pro Pro Cys Tyr Ser Leu Ser Ser Ser Ala Ser
    1570                1575                1580
Ser Leu Ser Glu Pro Glu Pro Ser Glu Pro Pro Ala Val His Pro Arg
1585                1590                1595                1600
Gly Arg Glu Pro Ala Val Thr Lys Asp Pro Gly Pro Gly Gly Gly Arg
                1605                1610                1615
Asp Ser Ser Pro Ser Pro Arg Ala Ala Glu Glu Leu Leu Gln Arg Cys
            1620                1625                1630
Ile Ser Ser Ala Leu Pro Arg Arg Arg Pro Pro Val Ser Gly Leu Arg
        1635                1640                1645
Arg Arg Lys Pro Arg Ala Thr Arg Leu Asp Glu Arg Pro Ala Glu Gly
    1650                1655                1660
Ser Arg Glu Arg Gly Glu Glu Ala Ala Gly Ser Asp Arg Ala Ser Asp
1665                1670                1675                1680
Leu Asp Ser Val Glu Trp Arg Ala Ile Gln Glu Gly Ala Asn Ser Ile
                1685                1690                1695
Val Thr Trp Leu His Gln Ala Ala Ala Ala Thr Arg Glu Ala Ser Ser
            1700                1705                1710
Glu Ser Asp Ser Ile Leu Ser Phe Val Ser Gly Leu Ser Val Gly Ser
        1715                1720                1725
Thr Leu Gln Pro Pro Lys His Arg Lys Gly Arg Gln Ala Glu Gly Glu
    1730                1735                1740
Met Gly Ser Ala Arg Arg Pro Glu Lys Arg Gly Ala Ala Ser Val Lys
1745                1750                1755                1760
Thr Ser Gly Ser Pro Arg Ser Pro Ala Gly Pro Glu Lys Pro Arg Gly
                1765                1770                1775
Thr Gln Lys Thr Thr Pro Gly Val Pro Ala Val Leu Arg Gly Arg Thr
            1780                1785                1790
Val Ile Tyr Val Pro Ser Pro Ala Pro Arg Ala Gln Pro Lys Gly Thr
        1795                1800                1805
Pro Gly Pro Arg Ala Thr Pro Arg Lys Val Ala Pro Pro Cys Leu Ala
    1810                1815                1820
Gln Pro Ala Ala Pro Ala Lys Val Pro Ser Pro Gly Gln Gln Arg Ser
1825                1830                1835                1840
Arg Ser Leu His Arg Pro Ala Lys Thr Ser Glu Leu Ala Thr Leu Ser
                1845                1850                1855

Gln Pro Pro Arg Ser Ala Thr Pro Pro Ala Arg Leu Ala Lys Thr Pro
            1860                1865                1870
Ser Ser Ser Ser Ser Gln Thr Ser Pro Ala Ser Gln Pro Leu Pro Arg
        1875                1880                1885
Lys Arg Pro Pro Val Thr Gln Ala Ala Gly Ala Leu Pro Gly Pro Gly
    1890                1895                1900
Ala Ser Pro Val Pro Lys Thr Pro Ala Arg Thr Leu Leu Ala Lys Gln
1905                1910                1915                1920
His Lys Thr Gln Arg Ser Pro Val Arg Ile Pro Phe Met Gln Arg Pro
                1925                1930                1935
Ala Arg Arg Gly Pro Pro Pro Leu Ala Arg Ala Val Pro Glu Pro Gly
            1940                1945                1950
Pro Arg Gly Arg Ala Gly Thr Glu Ala Gly Pro Gly Ala Arg Gly Gly
        1955                1960                1965
Arg Leu Gly Leu Val Arg Val Ala Ser Ala Leu Ser Ser Gly Ser Glu
    1970                1975                1980
Ser Ser Asp Arg Ser Gly Phe Arg Arg Gln Leu Thr Phe Ile Lys Glu
1985                1990                1995                2000
Ser Pro Gly Leu Arg Arg Arg Arg Ser Glu Leu Ser Ser Ala Glu Ser
                2005                2010                2015
Ala Ala Ser Ala Pro Gln Gly Ala Ser Pro Arg Arg Gly Arg Pro Ala
            2020                2025                2030

Leu Pro Ala Val Phe Leu Cys Ser Ser Arg Cys Glu Glu Leu Arg Ala
        2035                2040                2045
Ala Pro Arg Gln Gly Pro Ala Pro Ala Arg Gln Arg Pro Pro Ala Ala
    2050                2055                2060
Arg Pro Ser Pro Gly Glu Arg Pro Ala Arg Arg Thr Thr Ser Glu Ser
2065                2070                2075                2080
Pro Ser Arg Leu Pro Val Arg Ala Pro Ala Ala Arg Pro Glu Thr Val
                2085                2090                2095
Lys Arg Tyr Ala Ser Leu Pro His Ile Ser Val Ala Arg Arg Pro Asp
            2100                2105                2110
Gly Ala Val Pro Ala Ala Pro Ala Ser Ala Asp Ala Ala Arg Arg Ser
        2115                2120                2125
Ser Asp Gly Glu Pro Arg Pro Leu Pro Arg Val Ala Ala Pro Gly Thr
    2130                2135                2140
Thr Trp Arg Arg Ile Arg Asp Glu Asp Val Pro His Ile Leu Arg Ser
2145                2150                2155                2160
Thr Leu Pro Ala Thr Ala Leu Pro Leu Arg Gly Ser Thr Pro Glu Asp
                2165                2170                2175
Ala Pro Ala Gly Pro Pro Pro Arg Lys Thr Ser Asp Ala Val Val Gln
            2180                2185                2190
Thr Glu Glu Val Ala Ala Pro Lys Thr Asn Ser Ser Thr Ser Pro Ser
        2195                2200                2205
Leu Glu Thr Arg Glu Pro Pro Gly Ala Pro Ala Gly Gly Gln Leu Ser
    2210                2215                2220
Leu Leu Gly Ser Asp Val Asp Gly Pro Ser Leu Ala Lys Ala Pro Ile
2225                2230                2235                2240
Ser Ala Pro Phe Val His Glu Gly Leu Gly Val Ala Val Gly Gly Phe
                2245                2250                2255
Pro Ala Ser Arg His Gly Ser Pro Ser Arg Ser Ala Arg Val Pro Pro
            2260                2265                2270
Phe Asn Tyr Val Pro Ser Pro Met Val Val Ala Ala Thr Thr Asp Ser
        2275                2280                2285
Ala Ala Glu Lys Ala Pro Ala Thr Ala Ser Ala Thr Leu Leu Glu
    2290                2295                2300

<210> 306
<211> 156
<212> PRT
<213> Homo sapiens

<400> 306
Met Phe Leu Thr Glu Asp Leu Ile Thr Phe Asn Leu Arg Asn Phe Leu
1               5                   10                  15
Leu Phe Gln Leu Trp Glu Ser Ser Phe Ser Pro Gly Ala Gly Gly Phe
            20                  25                  30
Cys Thr Thr Leu Pro Pro Ser Phe Leu Arg Val Asp Asp Arg Ala Thr
        35                  40                  45
Ser Ser Thr Thr Asp Ser Ser Arg Ala Pro Ser Ser Pro Arg Pro Pro
    50                  55                  60
Gly Ser Thr Ser His Cys Gly Ile Ser Thr Arg Cys Thr Glu Arg Cys
65                  70                  75                  80
Leu Cys Val Leu Pro Leu Arg Thr Ser Gln Val Pro Asp Val Met Ala
                85                  90                  95
Pro Gln His Asp Gln Glu Lys Phe His Asp Leu Ala Tyr Ser Cys Leu
            100                 105                 110
Gly Lys Ser Phe Ser Met Ser Asn Gln Asp Leu Tyr Gly Tyr Ser Thr
        115                 120                 125
Ser Ser Leu Ala Leu Gly Leu Ala Trp Leu Ser Trp Glu Thr Lys Lys
    130                 135                 140
Lys Asn Val Leu His Leu Val Gly Leu Asp Ser Leu
145                 150                 155
<210> 307
<211> 695
<212> PRT
<213> Homo sapiens

<400> 307
Met Ala Ser Thr Arg Ser Ile Glu Leu Glu His Phe Glu Glu Arg Asp
1               5                   10                  15
Lys Arg Pro Arg Pro Gly Ser Arg Arg Gly Ala Pro Ser Ser Ser Gly
            20                  25                  30
Gly Ser Ser Ser Ser Gly Pro Lys Gly Asn Gly Leu Ile Pro Ser Pro
        35                  40                  45
Ala His Ser Ala His Cys Ser Phe Tyr Arg Thr Arg Thr Leu Gln Ala
    50                  55                  60
Leu Ser Ser Glu Lys Lys Ala Lys Lys Ala Arg Phe Tyr Arg Asn Gly
65                  70                  75                  80
Asp Arg Tyr Phe Lys Gly Leu Val Phe Ala Ile Ser Ser Asp Arg Phe
                85                  90                  95
Arg Ser Phe Asp Ala Leu Leu Ile Glu Leu Thr Arg Ser Leu Ser Asp
            100                 105                 110
Asn Val Asn Leu Pro Gln Gly Val Arg Thr Ile Tyr Thr Ile Asp Gly
        115                 120                 125
Ser Arg Lys Val Thr Ser Leu Asp Glu Leu Leu Glu Gly Glu Ser Tyr
    130                 135                 140
Val Cys Ala Ser Asn Glu Pro Phe Arg Lys Val Asp Tyr Thr Lys Asn
145                 150                 155                 160
Ile Asn Pro Asn Trp Ser Val Asn Ile Lys Gly Gly Thr Ser Arg Ala
                165                 170                 175
Leu Ala Ala Ala Ser Ser Val Lys Ser Glu Val Lys Glu Ser Lys Asp
            180                 185                 190
Phe Ile Lys Pro Lys Leu Val Thr Val Ile Arg Ser Gly Val Lys Pro
        195                 200                 205
Arg Lys Ala Val Arg Ile Leu Leu Asn Lys Lys Thr Ala His Ser Phe
    210                 215                 220
Glu Gln Val Leu Thr Asp Ile Thr Glu Ala Ile Lys Leu Asp Ser Gly
225                 230                 235                 240
Val Val Lys Arg Leu Cys Thr Leu Asp Gly Lys Gln Val Thr Cys Leu
                245                 250                 255
Gln Asp Phe Phe Gly Asp Asp Asp Val Phe Ile Ala Cys Gly Pro Glu
            260                 265                 270
Lys Phe Arg Tyr Ala Gln Asp Asp Phe Val Leu Asp His Ser Glu Cys
        275                 280                 285

Arg Val Leu Lys Ser Ser Tyr Ser Arg Ser Ser Ala Val Lys Tyr Ser
    290                 295                 300
Gly Ser Lys Ser Pro Gly Pro Ser Arg Arg Ser Lys Ser Pro Ala Ser
305                 310                 315                 320
Val Asn Gly Thr Pro Ser Ser Gln Leu Ser Thr Pro Lys Ser Thr Lys
                325                 330                 335
Ser Ser Ser Ser Ser Pro Thr Ser Pro Gly Ser Phe Arg Gly Leu Lys
            340                 345                 350
Ile Ser Ala His Gly Arg Ser Ser Ser Asn Val Asn Gly Gly Pro Glu
        355                 360                 365
Leu Asp Arg Cys Ile Ser Pro Glu Gly Val Asn Gly Asn Arg Cys Ser
    370                 375                 380
Glu Ser Ser Thr Leu Leu Glu Lys Tyr Lys Ile Gly Lys Val Ile Gly
385                 390                 395                 400
Asp Gly Asn Phe Ala Val Val Lys Glu Cys Ile Asp Arg Ser Thr Gly
                405                 410                 415
Lys Glu Phe Ala Leu Lys Ile Ile Asp Lys Ala Lys Cys Cys Gly Lys
            420                 425                 430

Glu His Leu Ile Glu Asn Glu Val Ser Ile Leu Arg Arg Val Lys His
        435                 440                 445
Pro Asn Ile Ile Met Leu Val Glu Glu Met Glu Thr Ala Thr Glu Leu
    450                 455                 460
Phe Leu Val Met Glu Leu Val Lys Gly Gly Asp Leu Phe Asp Ala Ile
465                 470                 475                 480
Thr Ser Ser Thr Lys Tyr Thr Glu Arg Asp Gly Ser Ala Met Val Tyr
                485                 490                 495
Asn Leu Ala Asn Ala Leu Arg Tyr Leu His Gly Leu Ser Ile Val His
            500                 505                 510
Arg Asp Ile Lys Pro Glu Asn Leu Leu Val Cys Glu Tyr Pro Asp Gly
        515                 520                 525
Thr Lys Ser Leu Lys Leu Gly Asp Phe Gly Leu Ala Thr Val Val Glu
    530                 535                 540
Gly Pro Leu Tyr Thr Val Cys Gly Thr Pro Thr Tyr Val Ala Pro Glu
545                 550                 555                 560
Ile Ile Ala Glu Thr Gly Tyr Gly Leu Lys Val Asp Ile Trp Ala Ala
                565                 570                 575
Gly Val Ile Thr Tyr Ile Leu Leu Cys Gly Phe Pro Pro Phe Arg Ser
            580                 585                 590
Glu Asn Asn Leu Gln Glu Asp Leu Phe Asp Gln Ile Leu Ala Gly Lys
        595                 600                 605
Leu Glu Phe Pro Ala Pro Tyr Trp Asp Asn Ile Thr Asp Ser Ala Lys
    610                 615                 620
Glu Leu Ile Ser Gln Met Leu Gln Val Asn Val Glu Ala Arg Cys Thr
625                 630                 635                 640
Ala Gly Gln Ile Leu Ser His Pro Trp Val Ser Asp Asp Ala Ser Gln
                645                 650                 655
Glu Asn Asn Met Gln Ala Glu Val Thr Gly Lys Leu Lys Gln His Phe
            660                 665                 670
Asn Asn Ala Leu Pro Lys Gln Asn Ser Thr Thr Thr Gly Val Ser Val
        675                 680                 685
Ile Met Phe Asp Leu Thr Val
    690                 695

<210> 308
<211> 710
<212> PRT
<213> Homo sapiens

<400> 308
Met Glu Glu Met Glu Glu Glu Leu Lys Cys Pro Val Cys Gly Ser Phe
1               5                   10                  15
Tyr Arg Glu Pro Ile Ile Leu Pro Cys Ser His Asn Leu Cys Gln Ala

            20                  25                  30
Cys Ala Arg Asn Ile Leu Val Gln Thr Pro Glu Ser Glu Ser Pro Gln
        35                  40                  45
Ser His Arg Ala Ala Gly Ser Gly Val Ser Asp Tyr Asp Tyr Leu Asp
    50                  55                  60
Leu Asp Lys Met Ser Leu Tyr Ser Glu Ala Asp Ser Gly Tyr Gly Ser
65                  70                  75                  80
Tyr Gly Gly Phe Ala Ser Ala Pro Thr Thr Pro Cys Gln Lys Ser Pro
                85                  90                  95
Asn Gly Val Arg Val Phe Pro Pro Ala Met Pro Pro Pro Ala Thr His
            100                 105                 110
Leu Ser Pro Ala Leu Ala Pro Val Pro Arg Asn Ser Cys Ile Thr Cys
        115                 120                 125
Pro Gln Cys His Arg Ser Leu Ile Leu Asp Asp Arg Gly Leu Arg Gly
    130                 135                 140
Phe Pro Lys Asn Arg Val Leu Glu Gly Val Ile Asp Arg Tyr Gln Gln
145                 150                 155                 160
Ser Lys Ala Ala Ala Leu Lys Cys Gln Leu Cys Glu Lys Ala Pro Lys
                165                 170                 175
Glu Ala Thr Val Met Cys Glu Gln Cys Asp Val Phe Tyr Cys Asp Pro
            180                 185                 190
Cys Arg Leu Arg Cys His Pro Pro Arg Gly Pro Leu Ala Lys His Arg
        195                 200                 205
Leu Val Pro Pro Ala Gln Gly Arg Val Ser Arg Arg Leu Ser Pro Arg
    210                 215                 220
Lys Val Ser Thr Cys Thr Asp His Glu Leu Glu Asn His Ser Met Tyr
225                 230                 235                 240
Cys Val Gln Cys Lys Met Pro Val Cys Tyr Gln Cys Leu Glu Glu Gly
                245                 250                 255
Lys His Ser Ser His Glu Val Lys Ala Leu Gly Ala Met Trp Lys Leu
            260                 265                 270
His Lys Ser Gln Leu Ser Gln Ala Leu Asn Gly Leu Ser Asp Arg Ala
        275                 280                 285
Lys Glu Ala Lys Glu Phe Leu Val Gln Leu Arg Asn Met Val Gln Gln
    290                 295                 300
Ile Gln Glu Asn Ser Val Glu Phe Glu Ala Cys Leu Val Ala Gln Cys
305                 310                 315                 320
Asp Ala Leu Ile Asp Ala Leu Asn Arg Arg Lys Ala Gln Leu Leu Ala
                325                 330                 335
Arg Val Asn Lys Glu His Glu His Lys Leu Lys Val Val Arg Asp Gln
            340                 345                 350
Ile Ser His Cys Thr Val Lys Leu Arg Gln Thr Thr Gly Leu Met Glu
        355                 360                 365
Tyr Cys Leu Glu Val Ile Lys Glu Asn Asp Pro Ser Gly Phe Leu Gln
    370                 375                 380
Ile Ser Asp Ala Leu Ile Arg Arg Val His Leu Thr Glu Asp Gln Trp
385                 390                 395                 400
Gly Lys Gly Thr Leu Thr Pro Arg Met Thr Thr Asp Phe Asp Leu Ser
                405                 410                 415
Leu Asp Asn Ser Pro Leu Leu Gln Ser Ile His Gln Leu Asp Phe Val
            420                 425                 430
Gln Val Lys Ala Ser Ser Pro Val Pro Ala Thr Pro Ile Leu Gln Leu
        435                 440                 445
Glu Glu Cys Cys Thr His Asn Asn Ser Ala Thr Leu Ser Trp Lys Gln
    450                 455                 460
Pro Pro Leu Ser Thr Val Pro Ala Asp Gly Tyr Ile Leu Glu Leu Asp
465                 470                 475                 480
Asp Gly Asn Gly Gly Gln Phe Arg Glu Val Tyr Val Gly Lys Glu Thr
                485                 490                 495
Met Cys Thr Val Asp Gly Leu His Phe Asn Ser Thr Tyr Asn Ala Arg
            500                 505                 510
Val Lys Ala Phe Asn Lys Thr Gly Val Ser Pro Tyr Ser Lys Thr Leu
        515                 520                 525

Val Leu Gln Thr Ser Glu Val Ala Trp Phe Ala Phe Asp Pro Gly Ser
    530                 535                 540
Ala His Ser Asp Ile Ile Leu Ser Asn Asp Asn Leu Thr Val Thr Cys
545                 550                 555                 560
Ser Ser Tyr Asp Asp Arg Val Val Leu Gly Lys Thr Gly Phe Ser Lys
                565                 570                 575
Gly Ile His Tyr Trp Glu Leu Thr Val Asp Arg Tyr Asp Asn His Pro
            580                 585                 590
Asp Pro Ala Phe Gly Val Ala Arg Met Asp Val Met Lys Asp Val Met
        595                 600                 605
Leu Gly Lys Asp Asp Lys Ala Trp Ala Met Tyr Val Asp Asn Asn Arg
    610                 615                 620
Ser Trp Phe Met His Asn Asn Ser His Thr Asn Arg Thr Glu Gly Gly
625                 630                 635                 640

Ile Thr Lys Gly Ala Thr Ile Gly Val Leu Leu Asp Leu Asn Arg Lys
                645                 650                 655
Asn Leu Thr Phe Phe Ile Asn Asp Glu Gln Gln Gly Pro Ile Ala Phe
            660                 665                 670
Asp Asn Val Glu Gly Leu Phe Phe Pro Ala Val Ser Leu Asn Arg Asn
        675                 680                 685
Val Gln Val Thr Leu His Thr Gly Leu Pro Val Pro Asp Phe Tyr Ser
    690                 695                 700
Ser Arg Ala Ser Ile Ala
705                 710

<210> 309
<211> 333
<212> PRT
<213> Homo sapiens

<400> 309
Met Glu Asp Leu Glu Glu Asp Val Arg Phe Ile Val Asp Glu Thr Leu
 1               5                  10                  15
Asp Phe Gly Gly Leu Ser Pro Ser Asp Ser Arg Glu Glu Glu Asp Ile
            20                  25                  30
Thr Val Leu Val Thr Pro Glu Lys Pro Leu Arg Arg Gly Leu Ser His
        35                  40                  45
Arg Ser Asp Pro Asn Ala Val Ala Pro Ala Pro Gln Gly Val Arg Leu
    50                  55                  60
Ser Leu Gly Pro Leu Ser Pro Glu Lys Leu Glu Glu Ile Leu Asp Glu
65                  70                  75                  80
Ala Asn Arg Leu Ala Ala Gln Leu Glu Gln Cys Ala Leu Gln Asp Arg
                85                  90                  95
Glu Ser Ala Gly Glu Gly Leu Gly Pro Arg Arg Val Lys Pro Ser Pro
            100                 105                 110
Arg Arg Glu Thr Phe Val Leu Lys Asp Ser Pro Val Arg Asp Leu Leu
        115                 120                 125
Pro Thr Val Asn Ser Leu Thr Arg Ser Thr Pro Ser Pro Ser Ser Leu
    130                 135                 140
Thr Pro Arg Leu Arg Ser Asn Asp Arg Lys Gly Ser Val Arg Ala Leu
145                 150                 155                 160
Arg Ala Thr Ser Gly Lys Arg Pro Ser Asn Met Lys Arg Glu Ser Pro
                165                 170                 175
Thr Cys Asn Leu Phe Pro Ala Ser Lys Ser Pro Ala Ser Ser Pro Leu
            180                 185                 190
Thr Arg Ser Thr Pro Pro Val Arg Gly Arg Ala Gly Pro Ser Gly Arg
        195                 200                 205
Ala Ala Ala Ser Pro Pro Thr Pro Ile Arg Ser Val Leu Ala Pro Gln
    210                 215                 220
Pro Ser Thr Ser Asn Ser Gln Arg Leu Pro Arg Pro Gln Gly Ala Ala
225                 230                 235                 240
Ala Lys Ser Ser Ser Gln Leu Pro Ile Pro Ser Ala Ile Pro Arg Pro

245                 250                 255
Ala Ser Arg Met Pro Leu Thr Ser Arg Ser Val Pro Pro Gly Arg Gly
            260                 265                 270
Ala Leu Pro Pro Asp Ser Leu Ser Thr Arg Lys Gly Leu Pro Arg Pro
        275                 280                 285
Ser Thr Ala Gly His Arg Val Arg Glu Ser Gly His Lys Val Pro Val
    290                 295                 300
Ser Gln Arg Leu Asn Leu Pro Val Met Gly Ala Thr Arg Ser Asn Leu
305                 310                 315                 320
Gln Pro Pro Arg Lys Val Ala Val Pro Gly Pro Thr Arg
                325                 330

<210> 310
<211> 546
<212> PRT
<213> Homo sapiens

<400> 310
Met Gly Ser Asp Ser Arg Leu Pro Glu Met Glu Glu Lys Gly Ser Gly
1               5                   10                  15
Asp Lys Ala Gly Trp Ser Gly Ala Leu Leu Ala Glu Val Lys Asn Gln
            20                  25                  30
Gly Leu Lys Leu Cys Glu Trp Met Leu Val Leu Lys Ala Glu Leu Val
        35                  40                  45
Glu Trp Tyr Thr Ser Val Ile Val Gly Gln Gln Gly His Arg Leu Lys
    50                  55                  60
Met Asp Leu Leu Lys Glu Arg His His Arg Lys Tyr Leu Gln Lys Pro
65                  70                  75                  80
Ile Lys Arg Cys Ser Gly Leu Met Gly Leu Asn Trp Ser Leu Arg Pro
                85                  90                  95
Ala Ile Ser Ser Ser Pro Leu Ala Arg Ser Gln Glu Lys His His Gly
            100                 105                 110
Ala Lys Gly Asn Glu Gly Val Lys Ala Leu Ser Phe Arg Lys Leu Arg
        115                 120                 125
Lys Leu Lys Arg Pro Pro Thr Pro Pro Ser Gln Ala Lys Ser Trp Arg
    130                 135                 140
Gly Arg Thr Gly Ser Ala Ala Asp Ser Pro Gly Ala Ala Ala Met Ala
145                 150                 155                 160
Val Gln Ala Ala Leu Leu Ser Thr His Pro Phe Val Pro Phe Gly Phe
                165                 170                 175
Gly Gly Ser Pro Asp Gly Leu Gly Gly Ala Phe Gly Ala Leu Asp Lys
            180                 185                 190
Gly Cys Cys Phe Glu Asp Asp Glu Thr Gly Ala Pro Ala Gly Ala Leu
        195                 200                 205
Leu Ser Gly Ala Glu Gly Gly Asp Val Arg Glu Ala Thr Arg Asp Leu
    210                 215                 220
Leu Ser Phe Ile Asp Ser Ala Ser Ser Asn Ile Lys Leu Ala Leu Asp
225                 230                 235                 240
Lys Pro Gly Lys Ser Lys Arg Lys Val Asn His Arg Asn Ala Pro Thr
                245                 250                 255
Val Ala Ala Pro Ala His Gly Lys Ala Ala Pro Arg Arg Glu Ala Ser
            260                 265                 270
Gln Ala Ala Ala Ala Ala Ser Leu Gln Ser Arg Ser Leu Ala Ala Leu
        275                 280                 285
Phe Asp Ser Leu Arg His Val Pro Gly Gly Ala Glu Pro Ala Gly Gly
    290                 295                 300
Glu Val Ala Ala Pro Ala Ala Gly Leu Gly Gly Ala Gly Thr Gly Gly
305                 310                 315                 320
Ala Gly Gly Asp Val Ala Gly Pro Ala Gly Ala Thr Ala Ile Pro Gly
                325                 330                 335
Ala Arg Lys Val Pro Leu Arg Ala Arg Asn Leu Pro Pro Ser Phe Phe
            340                 345                 350

Thr Glu Pro Ser Arg Ala Gly Gly Gly Gly Cys Gly Pro Ser Gly Pro
        355                 360                 365
Asp Val Ser Leu Gly Asp Leu Glu Lys Gly Ala Glu Ala Val Glu Phe
    370                 375                 380
Phe Glu Leu Leu Gly Pro Asp Tyr Gly Ala Gly Thr Glu Ala Ala Val
385                 390                 395                 400
Leu Leu Ala Ala Glu Pro Leu Asp Val Phe Pro Ala Gly Ala Ser Val
                405                 410                 415
Leu Arg Gly Pro Pro Glu Leu Glu Pro Gly Leu Phe Glu Pro Pro Pro
            420                 425                 430

Ala Val Val Gly Asn Leu Leu Tyr Pro Glu Pro Trp Ser Val Pro Gly
        435                 440                 445
Cys Ser Pro Thr Lys Lys Ser Pro Leu Thr Ala Pro Arg Gly Gly Leu
    450                 455                 460
Thr Leu Asn Glu Pro Leu Ser Pro Leu Tyr Pro Ala Ala Ala Asp Ser
465                 470                 475                 480
Pro Gly Gly Glu Asp Gly Arg Gly His Leu Ala Ser Phe Ala Pro Phe
                485                 490                 495
Phe Pro Asp Cys Ala Leu Pro Pro Pro Pro Pro Pro His Gln Val Ser
            500                 505                 510
Tyr Asp Tyr Ser Ala Gly Tyr Ser Arg Thr Ala Tyr Ser Ser Leu Trp
        515                 520                 525
Arg Ser Asp Gly Val Trp Glu Gly Ala Pro Gly Glu Glu Gly Ala His
    530                 535                 540
Arg Asp
545

<210> 311
<211> 901
<212> PRT
<213> Homo sapiens

<400> 311
   Met Ala Pro Thr Lys Pro Ser Phe Gln Gln Asp Pro Ser Arg Arg Glu
   1               5                   10                  15
   Arg Leu Gln Ala Leu Arg Lys Glu Lys Ser Arg Asp Ala Ala Arg Ser
               20                  25                  30
   Arg Arg Gly Lys Glu Asn Phe Glu Phe Tyr Glu Leu Ala Lys Leu Leu
           35                  40                  45
   Pro Leu Pro Ala Ala Ile Thr Ser Gln Leu Asp Lys Ala Ser Ile Ile
       50                  55                  60
   Arg Leu Thr Ile Ser Tyr Leu Lys Met Arg Asp Phe Ala Asn Gln Gly
   65                  70                  75                  80
   Asp Pro Pro Trp Asn Leu Arg Met Glu Gly Pro Pro Pro Asn Thr Ser
                   85                  90                  95
   Val Lys Gly Ala Gln Arg Arg Arg Ser Pro Ser Ala Leu Ala Ile Glu
               100                 105                 110
   Val Phe Glu Ala His Leu Gly Ser His Ile Leu Gln Ser Leu Asp Gly
           115                 120                 125
   Phe Val Phe Ala Leu Asn Gln Glu Gly Lys Phe Leu Tyr Ile Ser Glu
       130                 135                 140
   Thr Val Ser Ile Tyr Leu Gly Leu Ser Gln Val Glu Leu Thr Gly Ser
   145                 150                 155                 160
   Ser Val Phe Asp Tyr Val His Pro Gly Asp His Val Glu Met Ala Glu
                   165                 170                 175
   Gln Leu Gly Met Lys Leu Pro Pro Gly Arg Gly Leu Leu Ser Gln Gly
               180                 185                 190
   Thr Ala Glu Asp Gly Ala Ser Ser Ala Ser Ser Ser Ser Gln Ser Glu
           195                 200                 205
   Thr Pro Glu Pro Val Glu Ser Thr Ser Pro Ser Leu Leu Thr Thr Asp
       210                 215                 220
   Asn Thr Leu Glu Arg Ser Phe Phe Ile Arg Met Lys Ser Thr Leu Thr
   225                     230                     235                     240
   Lys Arg Gly Val His Ile Lys Ser Ser Gly Tyr Lys Val Ile His Ile
                   245                     250                     255
   Thr Gly Arg Leu Arg Leu Arg Val Ser Leu Ser His Gly Arg Thr Val
               260                     265                     270
   Pro Ser Gln Ile Met Gly Leu Val Val Val Ala His Ala Leu Pro Pro
           275                     280                     285
   Pro Thr Ile Asn Glu Val Arg Ile Asp Cys His Met Phe Val Thr Arg
       290                     295                     300
   Val Asn Met Asp Leu Asn Ile Ile Tyr Cys Glu Asn Arg Ile Ser Asp
   305                     310                     315                     320
   Tyr Met Asp Leu Thr Pro Val Asp Ile Val Gly Lys Arg Cys Tyr His
                   325                     330                     335
   Phe Ile His Ala Glu Asp Val Glu Gly Ile Arg His Ser His Leu Asp
               340                     345                     350
   Leu Leu Asn Lys Gly Gln Cys Val Thr Lys Tyr Tyr Arg Trp Met Gln
           355                     360                     365
   Lys Asn Gly Gly Tyr Ile Trp Ile Gln Ser Ser Ala Thr Ile Ala Ile
       370                     375                     380
   Asn Ala Lys Asn Ala Asn Glu Lys Asn Ile Ile Trp Val Asn Tyr Leu
   385                     390                     395                     400
   Leu Ser Asn Pro Glu Tyr Lys Asp Thr Pro Met Asp Ile Ala Gln Leu
                   405                     410                     415
   Pro His Leu Pro Glu Lys Thr Ser Glu Ser Ser Glu Thr Ser Asp Ser
               420                     425                     430
   Glu Ser Asp Ser Lys Asp Thr Ser Gly Ile Thr Glu Asp Asn Glu Asn
           435                     440                     445
   Ser Lys Ser Asp Glu Lys Gly Asn Gln Ser Glu Asn Ser Glu Asp Pro
       450                     455                     460
   Glu Pro Asp Arg Lys Lys Ser Gly Asn Ala Cys Asp Asn Asp Met Asn
   465                     470                     475                     480
   Cys Asn Asp Asp Gly His Ser Ser Ser Asn Pro Asp Ser Arg Asp Ser
                   485                     490                     495
   Asp Asp Ser Phe Glu His Ser Asp Phe Glu Asn Pro Lys Ala Gly Glu
               500                     505                     510
   Asp Gly Phe Gly Ala Leu Gly Ala Met Gln Ile Lys Val Glu Arg Tyr
           515                     520                     525
   Val Glu Ser Glu Ser Asp Leu Arg Leu Gln Asn Cys Glu Ser Leu Thr
       530                     535                     540
   Ser Asp Ser Ala Lys Asp Ser Asp Ser Ala Gly Glu Ala Gly Ala Gln
   545                     550                     555                     560
   Ala Ser Ser Lys His Gln Lys Arg Lys Lys Arg Arg Lys Arg Gln Lys
                   565                     570                     575
   Gly Gly Ser Ala Ser Arg Arg Arg Leu Ser Ser Ala Ser Ser Pro Gly
               580                     585                     590
   Gly Leu Asp Ala Gly Leu Val Glu Pro Pro Arg Leu Leu Ser Ser Pro
           595                     600                     605
   Asn Ser Ala Ser Val Leu Lys Ile Lys Thr Glu Ile Ser Glu Pro Ile
       610                     615                     620
   Asn Phe Asp Asn Asp Ser Ser Ile Trp Asn Tyr Pro Pro Asn Arg Glu
   625                     630                     635                     640
   Ile Ser Arg Asn Glu Ser Pro Tyr Ser Met Thr Lys Pro Pro Ser Ser
                   645                     650                     655
   Glu His Phe Pro Ser Pro Gln Gly Gly Gly Gly Gly Gly Gly Gly Gly
               660                     665                     670
   Gly Gly Leu His Val Ala Ile Pro Asp Ser Val Leu Thr Pro Pro Gly
           675                     680                     685
   Ala Asp Gly Ala Ala Ala Arg Lys Thr Gln Phe Gly Ala Ser Ala Thr
       690                     695                     700
   Ala Ala Leu Ala Pro Val Ala Ser Asp Pro Leu Ser Pro Pro Leu Ser
   705                     710                     715                     720
   Ala Ser Pro Arg Asp Lys His Pro Gly Asn Gly Gly Gly Gly Gly Gly
                   725                     730                     735
   Gly Gly Gly Gly Ala Gly Gly Gly Gly Pro Ser Ala Ser Asn Ser Leu
   740 745 750
   Leu Tyr Thr Gly Asp Leu Glu Ala Leu Gln Arg Leu Gln Ala Gly Asn
   755 760 765
   Val Val Leu Pro Leu Val His Arg Val Thr Gly Thr Leu Ala Ala Thr
   770 775 780
   Ser Thr Ala Ala Gln Arg Val Tyr Thr Thr Gly Thr Ile Arg Tyr Ala
   785 790 795 800
   Pro Ala Glu Val Thr Leu Ala Met Gln Ser Asn Leu Leu Pro Asn Ala
   805 810 815
   His Ala Val Asn Phe Val Asp Val Asn Ser Pro Gly Phe Gly Leu Asp
   820 825 830
   Pro Lys Thr Pro Met Glu Met Leu Tyr His His Val His Arg Leu Asn
   835 840 845
   Met Ser Gly Pro Phe Gly Gly Ala Val Ser Ala Ala Ser Leu Thr Gln
   850 855 860
   Met Pro Ala Gly Asn Val Phe Thr Thr Ala Glu Gly Leu Phe Ser Thr
   865 870 875 880
   Leu Pro Phe Pro Val Tyr Ser Asn Gly Ile His Ala Ala Gln Thr Leu
   885 890 895
   Glu Arg Lys Glu Asp
   900

<210> 312
<211> 323
<212> PRT
<213> Homo sapiens

<400> 312
Met Asp Ser Asp Ala Ser Leu Val Ser Ser Arg Pro Ser Ser Pro Glu
1               5                   10                  15
Pro Asp Asp Leu Phe Leu Pro Ala Arg Ser Lys Gly Ser Ser Gly Ser
            20                  25                  30
Ala Phe Thr Gly Gly Thr Val Ser Ser Ser Thr Pro Ser Asp Cys Pro
        35                  40                  45
Pro Glu Leu Ser Ala Glu Leu Arg Gly Ala Met Gly Ser Ala Gly Ala
    50                  55                  60
His Pro Gly Asp Lys Leu Gly Gly Ser Gly Phe Lys Ser Ser Ser Ser
65                  70                  75                  80
Ser Thr Ser Ser Ser Thr Ser Ser Ala Ala Ala Ser Ser Thr Lys Lys
                85                  90                  95
Asp Lys Lys Gln Met Thr Glu Pro Glu Leu Gln Gln Leu Arg Leu Lys
            100                 105                 110
Ile Asn Ser Arg Glu Arg Lys Arg Met His Asp Leu Asn Ile Ala Met
        115                 120                 125
Asp Gly Leu Arg Glu Val Met Pro Tyr Ala His Gly Pro Ser Val Arg
    130                 135                 140
Lys Leu Ser Lys Ile Ala Thr Leu Leu Leu Ala Arg Asn Tyr Ile Leu
145                 150                 155                 160
Met Leu Thr Asn Ser Leu Glu Glu Met Lys Arg Leu Val Ser Glu Ile
                165                 170                 175
Tyr Gly Gly His His Ala Gly Phe His Pro Ser Ala Cys Gly Gly Leu
            180                 185                 190
Ala His Ser Ala Pro Leu Pro Ala Ala Thr Ala His Pro Ala Ala Ala
        195                 200                 205
Ala His Ala Ala His His Pro Ala Val His His Pro Ile Leu Pro Pro
    210                 215                 220
Ala Ala Ala Ala Ala Ala Ala Ala Ala Ala Ala Ala Ala Val Ser Ser
225                 230                 235                 240
Ala Ser Leu Pro Gly Ser Gly Leu Pro Ser Val Gly Ser Ile Arg Pro
                245                 250                 255
Pro His Gly Leu Leu Lys Ser Pro Ser Ala Ala Ala Ala Ala Pro Leu

            260                 265                 270
Gly Gly Gly Gly Gly Gly Ser Gly Ala Ser Gly Gly Phe Gln His Trp
        275                 280                 285
Gly Gly Met Pro Cys Pro Cys Ser Met Cys Gln Val Pro Pro Pro His
    290                 295                 300
His His Val Ser Ala Met Gly Ala Gly Ser Leu Pro Arg Leu Thr Ser
305                 310                 315                 320
Asp Ala Lys

<210> 313
<211> 1482
<212> PRT
<213> Homo sapiens

<400> 313
Met Thr Val Leu Glu Glu Ser Ser Ile Ser Leu Met Ser Ser Val Val
1               5                   10                  15
Ala Asp Phe Ser Glu Phe Glu Glu Asp Pro Gln Val Phe Asn Thr Leu
            20                  25                  30
Phe Pro Ser Arg Pro Ile Val Pro Leu Ser Ser Arg Ser Met Glu Ile
        35                  40                  45
Ser Glu Thr Ser Val Gly Ile Ser Ala Glu Val Asp Met Ser Ser Val
    50                  55                  60
Thr Thr Thr Gln Val Pro Pro Ala His Gly Arg Leu Ser Val Pro Ala
65                  70                  75                  80
Ser Leu Asp Pro Thr Ala Gly Ser Leu Ser Val Ala Glu Thr Gln Val
                85                  90                  95
Thr Pro Ser Ser Val Thr Thr Ala Phe Phe Ser Val Ile Thr Ser Ile
            100                 105                 110
Leu Leu Asp Ser Ser Phe Ser Val Ile Ala Asn Lys Asn Thr Pro Ser
        115                 120                 125
Leu Ala Val Arg Asp Pro Ser Val Phe Thr Pro Tyr Ser Leu Val Pro
    130                 135                 140
Ser Val Glu Ser Ser Leu Phe Ser Asp Gln Glu Arg Ser Ser Phe Ser
145                 150                 155                 160
Glu His Lys Pro Arg Gly Ala Leu Asp Phe Ala Ser Ser Phe Phe Ser
                165                 170                 175
Thr Pro Pro Leu Glu Leu Ser Gly Ser Ile Ser Ser Pro Ser Glu Ala
            180                 185                 190
Pro Ala Ser Leu Ser Leu Met Pro Ser Asp Leu Ser Pro Phe Thr Ser
        195                 200                 205
Gln Ser Phe Ser Pro Leu Val Glu Thr Phe Thr Leu Phe Asp Ser Ser
    210                 215                 220
Asp Leu Gln Ser Ser Gln Leu Ser Leu Pro Ser Ser Thr Asn Leu Glu
225                 230                 235                 240
Phe Ser Gln Leu Gln Pro Ser Ser Glu Leu Pro Leu Asn Thr Ile Met
                245                 250                 255
Leu Leu Pro Ser Arg Ser Glu Val Ser Pro Trp Ser Ser Phe Pro Ser
            260                 265                 270
Asp Ser Leu Glu Phe Val Glu Ala Ser Thr Val Ser Leu Thr Asp Ser
        275                 280                 285
Glu Ala His Phe Thr Ser Ala Phe Ile Glu Thr Thr Ser Tyr Leu Glu
    290                 295                 300
Ser Ser Leu Ile Ser His Glu Ser Ala Val Thr Ala Leu Val Pro Pro
305                 310                 315                 320
Gly Ser Glu Ser Phe Asp Ile Leu Thr Ala Gly Ile Gln Ala Thr Ser
                325                 330                 335
Pro Leu Thr Thr Val His Thr Thr Pro Ile Leu Thr Glu Ser Ser Leu
            340                 345                 350
Phe Ser Thr Leu Thr Pro Pro Asp Asp Gln Ile Ser Ala Leu Asp Gly
        355                 360                 365
His Val Ser Val Leu Ala Ser Phe Ser Lys Ala Ile Pro Thr Gly Thr
    370                 375                 380

Val Leu Ile Thr Asp Ala Tyr Leu Pro Ser Gly Ser Ser Phe Val Ser
385                 390                 395                 400

Glu Ala Thr Pro Phe Pro Leu Pro Thr Glu Leu Thr Val Val Gly Pro
                405                 410                 415
Ser Leu Thr Pro Thr Glu Val Pro Leu Asn Thr Ser Thr Glu Val Ser
            420                 425                 430
Thr Thr Ser Thr Gly Ala Ala Thr Gly Gly Pro Leu Asp Ser Thr Leu
        435                 440                 445
Met Gly Asp Ala Ala Ser Gln Ser Pro Pro Glu Ser Ser Ala Ala Pro
    450                 455                 460
Pro Leu Pro Ser Leu Arg Pro Val Thr Ala Phe Thr Leu Glu Ala Thr
465                 470                 475                 480
Val Asp Thr Pro Thr Leu Ala Thr Ala Lys Pro Pro Tyr Val Cys Asp
                485                 490                 495
Ile Thr Val Pro Asp Ala Tyr Leu Ile Thr Thr Val Leu Ala Arg Arg
            500                 505                 510
Ala Val Gln Glu Tyr Ile Ile Thr Ala Ile Lys Glu Val Leu Arg Ile
        515                 520                 525
His Phe Asn Arg Ala Val Glu Leu Lys Val Tyr Glu Leu Phe Thr Asp
    530                 535                 540
Phe Thr Phe Leu Val Thr Ser Gly Pro Phe Val Tyr Thr Ala Ile Ser
545                 550                 555                 560
Val Ile Asn Val Leu Ile Asn Ser Lys Leu Val Arg Asp Gln Thr Pro
                565                 570                 575
Leu Ile Leu Ser Val Lys Pro Ser Phe Leu Val Pro Glu Ser Arg Phe
            580                 585                 590
Gln Val Gln Thr Val Leu Gln Phe Val Pro Pro Ser Val Asp Thr Gly
        595                 600                 605
Phe Cys Asn Phe Thr Gln Arg Ile Glu Lys Gly Leu Met Thr Ala Leu
    610                 615                 620
Phe Glu Val Arg Lys His His Gln Gly Thr Tyr Asn Leu Thr Val Gln
625                 630                 635                 640
Ile Leu Asn Ile Thr Ile Ser Ser Ser Arg Val Thr Pro Arg Arg Gly
                645                 650                 655
Pro Val Asn Ile Ile Phe Ala Val Lys Ser Thr Gln Gly Phe Leu Asn
            660                 665                 670
Gly Ser Glu Val Ser Glu Leu Leu Arg Asn Leu Ser Val Val Glu Phe
        675                 680                 685
Ser Phe Tyr Leu Gly Tyr Pro Val Leu Gln Ile Ala Glu Pro Phe Gln
    690                 695                 700
Tyr Pro Gln Leu Asn Leu Ser Gln Leu Leu Lys Ser Ser Trp Val Arg
705                 710                 715                 720
Thr Val Leu Leu Gly Val Met Glu Lys Gln Leu Gln Asn Glu Val Phe
                725                 730                 735
Gln Ala Glu Met Glu Arg Lys Leu Ala Gln Leu Leu Ser Glu Val Ser
            740                 745                 750
Thr Arg Arg Arg Met Trp Arg Arg Ala Thr Val Ala Ala Gly Asn Ser
        755                 760                 765
Val Val Gln Val Val Asn Val Ser Arg Leu Glu Gly Asp Asp Asn Pro
    770                 775                 780
Val Gln Leu Ile Tyr Phe Val Glu Asp Gln Asp Gly Glu Arg Leu Ser
785                 790                 795                 800
Ala Val Lys Ser Ser Asp Leu Ile Asn Lys Met Asp Leu Gln Arg Ala
                805                 810                 815
Ala Ile Ile Leu Gly Tyr Arg Ile Gln Gly Val Ile Ala Gln Pro Val
            820                 825                 830
Asp Arg Val Lys Arg Pro Ser Pro Glu Ser Gln Ser Asn Asn Leu Trp
        835                 840                 845
Val Ile Val Gly Val Val Ile Pro Val Leu Val Val Met Val Ile Val
    850                 855                 860
Val Ile Leu Tyr Trp Lys Leu Cys Arg Thr Asp Lys Leu Asp Phe Gln
865                 870                 875                 880

Pro Asp Thr Val Ala Asn Ile Gln Gln Arg Gln Lys Leu Gln Ile Pro
                885                 890                 895
Ser Val Lys Gly Phe Asp Phe Ala Lys Gln His Leu Gly Gln His Asn
            900                 905                 910
Lys Asp Asp Ile Leu Ile Ile His Glu Pro Ala Pro Leu Pro Gly Pro
        915                 920                 925
Leu Lys Asp His Thr Thr Pro Ser Glu Asn Gly Asp Val Pro Ser Pro
    930                 935                 940
Lys Ser Lys Ile Pro Ser Lys Asn Val Arg His Arg Gly Arg Val Ser
945                 950                 955                 960
Pro Ser Asp Ala Asp Ser Thr Val Ser Glu Glu Ser Ser Glu Arg Asp
                965                 970                 975
Ala Gly Asp Lys Thr Pro Gly Ala Val Asn Asp Gly Arg Ser His Arg
            980                 985                 990
Ala Pro Gln Ser Gly Pro Pro Leu Pro Ser Ser Gly Asn Glu Gln His
        995                 1000                1005
Ser Ser Ala Ser Ile Phe Glu His Val Asp Arg Ile Ser Arg Pro Pro
    1010                1015                1020
Glu Ala Ser Arg Arg Val Pro Ser Lys Ile Gln Leu Ile Ala Met Gln
1025                1030                1035                1040
Pro Ile Pro Ala Pro Pro Val Gln Arg Pro Ser Pro Ala Asp Arg Val
                1045                1050                1055
Ala Glu Ser Asn Lys Ile Asn Lys Glu Ile Gln Thr Ala Leu Arg His
            1060                1065                1070
Lys Ser Glu Ile Glu His His Arg Asn Lys Ile Arg Leu Arg Ala Lys
        1075                1080                1085
Arg Arg Gly His Tyr Glu Phe Pro Val Val Asp Asp Leu Ser Ser Gly
    1090                1095                1100
Asp Thr Lys Glu Arg His Arg Val Tyr Arg Arg Ala Gln Met Gln Ile
1105                1110                1115                1120
Asp Lys Ile Leu Asp Pro Thr Ala Ser Val Pro Ser Val Phe Ile Glu
                1125                1130                1135
Pro Arg Lys Ser Ser Arg Ile Lys Arg Ser Pro Lys Pro Arg Arg Lys
            1140                1145                1150
His Gln Val Asn Gly Cys Pro Ala Asp Ala Glu Lys Asp Arg Leu Ile
        1155                1160                1165
Thr Thr Asp Ser Asp Gly Thr Tyr Arg Arg Pro Pro Gly Val His Asn
    1170                1175                1180
Ser Ala Tyr Ile Gly Cys Pro Ser Asp Pro Asp Leu Pro Ala Asp Val
1185                1190                1195                1200
Gln Thr Pro Ser Ser Val Glu Leu Gly Arg Tyr Pro Ala Leu Pro Phe
                1205                1210                1215
Pro Ala Ser Gln Tyr Ile Pro Pro Gln Pro Ser Ile Glu Glu Ala Arg
            1220                1225                1230
Gln Thr Met His Ser Leu Leu Asp Asp Ala Phe Ala Leu Val Ala Pro
        1235                1240                1245
Ser Ser Gln Pro Ala Ser Thr Ala Gly Val Gly Pro Gly Val Pro Pro
    1250                1255                1260
Gly Leu Pro Ala Asn Ser Thr Pro Ser Gln Glu Glu Arg Arg Ala Thr
1265                1270                1275                1280
Gln Trp Gly Ser Phe Tyr Ser Pro Ala Gln Thr Ala Asn Asn Pro Cys
                1285                1290                1295
Ser Arg Tyr Glu Asp Tyr Gly Met Thr Pro Pro Thr Gly Pro Leu Pro
            1300                1305                1310
Arg Pro Gly Phe Gly Pro Gly Leu Leu Gln Ser Thr Glu Leu Val Pro
        1315                1320                1325
Pro Asp Pro Gln Gln Pro Gln Ala Ser Ala Glu Ala Pro Phe Ala Ala
    1330                1335                1340
Arg Gly Ile Tyr Ser Glu Glu Met Pro Ser Val Ala Arg Pro Arg Pro
1345                1350                1355                1360

Val Gly Gly Thr Thr Gly Ser Gln Ile Gln His Leu Thr Gln Val Gly

                1365                1370                1375
Ile Ala Ser Arg Ile Gly Ala Gln Pro Val Glu Ile Pro Pro Ser Arg
            1380                1385                1390
Gly Ser Gln Tyr Gly Gly Pro Gly Trp Pro Ser Tyr Gly Glu Asp Glu
        1395                1400                1405
Ala Gly Arg Arg Glu Ala Val Pro Arg Thr Ser Gly Arg Glu Pro Ser
    1410                1415                1420
Ala Pro Ser Gly Asn Leu Pro His Arg Gly Leu Gln Gly Pro Gly Leu
1425                1430                1435                1440
Gly Tyr Pro Thr Ser Ser Thr Glu Asp Leu Gln Pro Gly His Ser Ser
                1445                1450                1455
Ala Ser Leu Ile Lys Ala Ile Arg Glu Glu Leu Leu Arg Leu Ser Gln
            1460                1465                1470
Lys Gln Ser Thr Val Gln Asn Phe His Ser
        1475                1480

<210> 314
<211> 244
<212> PRT
<213> Homo sapiens

<400> 314
Met Ala Pro Arg Pro Leu Gly Pro Leu Val Leu Ala Leu Gly Gly Ala
1               5                   10                  15
Ala Ala Val Leu Gly Ser Val Leu Phe Ile Leu Trp Lys Thr Tyr Phe
            20                  25                  30
Gly Arg Gly Arg Glu Arg Arg Trp Asp Arg Gly Glu Ala Trp Trp Gly
        35                  40                  45
Ala Glu Ala Ala Arg Leu Pro Glu Trp Asp Glu Trp Asp Pro Glu Asp
    50                  55                  60
Glu Glu Asp Glu Glu Pro Ala Leu Glu Glu Leu Glu Gln Arg Glu Val
65                  70                  75                  80
Leu Val Leu Gly Leu Asp Gly Ala Gly Lys Ser Thr Phe Leu Arg Val
                85                  90                  95
Leu Ser Gly Lys Pro Pro Leu Glu Gly His Ile Pro Thr Trp Gly Phe
            100                 105                 110
Asn Ser Val Arg Leu Pro Thr Lys Asp Phe Glu Val Asp Leu Leu Glu
        115                 120                 125
Ile Gly Gly Ser Gln Asn Leu Arg Phe Tyr Trp Lys Glu Phe Val Ser
    130                 135                 140
Glu Val Asp Val Leu Val Phe Val Val Asp Ser Ala Asp Arg Leu Arg
145                 150                 155                 160
Leu Pro Trp Ala Arg Gln Glu Leu His Lys Leu Leu Asp Lys Asp Pro
                165                 170                 175
Asp Leu Pro Val Val Val Val Ala Asn Lys Gln Asp Leu Ser Glu Ala
            180                 185                 190
Met Ser Met Gly Glu Leu Gln Arg Glu Leu Gly Leu Gln Ala Ile Asp
        195                 200                 205
Asn Gln Arg Glu Val Phe Leu Leu Ala Ala Ser Ile Ala Pro Ala Gly
    210                 215                 220
Pro Thr Phe Glu Glu Pro Gly Thr Val His Ile Trp Lys Leu Leu Leu
225                 230                 235                 240
Glu Leu Leu Ser

<210> 315
<211> 121
<212> PRT
<213> Homo sapiens

<400> 315
Met Ala Arg Arg Ala Gly Gly Ala Arg Met Phe Gly Ser Leu Leu Leu
1               5                   10                  15

Phe Ala Leu Leu Ala Ala Gly Val Ala Pro Leu Ser Trp Asp Leu Pro
            20                  25                  30
Glu Pro Arg Ser Arg Ala Ser Lys Ile Arg Val His Ser Arg Gly Asn
        35                  40                  45
Leu Trp Ala Thr Gly His Phe Met Gly Lys Lys Ser Leu Glu Pro Ser
    50                  55                  60
Ser Pro Ser His Trp Gly Gln Leu Pro Thr Pro Pro Leu Arg Asp Gln
65                  70                  75                  80
Arg Leu Gln Leu Ser His Asp Leu Leu Gly Ile Leu Leu Leu Lys Lys
                85                  90                  95
Ala Leu Gly Val Ser Leu Ser Arg Pro Ala Pro Gln Ile Gln Tyr Arg
            100                 105                 110
Arg Leu Leu Val Gln Ile Leu Gln Lys
        115                 120

<210> 316
<211> 432
<212> PRT
<213> Homo sapiens

<400> 316

<210> 317
<211> 466
<212> PRT
<213> Homo sapiens

<400> 317
Met Arg Leu Leu Ser Ser Leu Ser Gly Ser Ser Val Ser Ser Asp Ala
1               5                   10                  15
Glu Glu Tyr Gln Pro Pro Ile Trp Lys Ser Tyr Leu Tyr Gln Leu Gln
            20                  25                  30
Gln Glu Ala Pro Arg Pro Lys Arg Ile Ile Cys Pro Arg Glu Val Glu
        35                  40                  45
Asn Arg Pro Lys Tyr Tyr Gly Arg Glu Phe His Gly Ile Ile Ser Arg
    50                  55                  60
Glu Gln Ala Asp Glu Leu Leu Gly Gly Val Glu Gly Ala Tyr Ile Leu
65                  70                  75                  80
Arg Glu Ser Gln Arg Gln Pro Gly Cys Tyr Thr Leu Ala Leu Arg Phe
                85                  90                  95
Gly Asn Gln Thr Leu Asn Tyr Arg Leu Phe His Asp Gly Lys His Phe
            100                 105                 110
Val Gly Glu Lys Arg Phe Glu Ser Ile His Asp Leu Val Thr Asp Gly
        115                 120                 125
Leu Ile Thr Leu Tyr Ile Glu Thr Lys Ala Ala Glu Tyr Ile Ser Lys
    130                 135                 140
Met Thr Thr Asn Pro Ile Tyr Glu His Ile Gly Tyr Ala Thr Leu Leu
145                 150                 155                 160
Arg Glu Lys Val Ser Arg Arg Leu Ser Arg Ser Lys Asn Glu Pro Arg
                165                 170                 175
Lys Thr Asn Val Thr His Glu Glu His Thr Ala Val Glu Lys Ile Ser
            180                 185                 190
Ser Leu Val Arg Arg Ala Ala Leu Thr His Asn Asp Asn His Phe Asn
        195                 200                 205
Tyr Glu Lys Thr His Asn Phe Lys Val His Thr Phe Arg Gly Pro His
    210                 215                 220
Trp Cys Glu Tyr Cys Ala Asn Phe Met Trp Gly Leu Ile Ala Gln Gly
225                 230                 235                 240
Val Arg Cys Ser Asp Cys Gly Leu Asn Val His Lys Gln Cys Ser Lys
                245                 250                 255
His Val Pro Asn Asp Cys Gln Pro Asp Leu Lys Arg Ile Lys Lys Val
            260                 265                 270
Tyr Cys Cys Asp Leu Thr Thr Leu Val Lys Ala His Asn Thr Gln Arg
        275                 280                 285
Pro Met Val Val Asp Ile Cys Ile Arg Glu Ile Glu Ala Arg Gly Leu
    290                 295                 300
Lys Ser Glu Gly Leu Tyr Arg Val Ser Gly Phe Thr Glu His Ile Glu
305                 310                 315                 320
Asp Val Lys Met Ala Phe Asp Arg Asp Gly Glu Lys Ala Asp Ile Ser
                325                 330                 335
Ala Asn Val Tyr Pro Asp Ile Asn Ile Ile Thr Gly Ala Leu Lys Leu

                340                 345                 350
Tyr Phe Arg Asp Leu Pro Ile Pro Val Ile Thr Tyr Asp Thr Tyr Ser
        355                 360                 365
Lys Phe Ile Asp Ala Ala Lys Ile Ser Asn Ala Asp Glu Arg Leu Glu
    370                 375                 380
Ala Val His Glu Val Leu Met Leu Leu Pro Pro Ala His Tyr Glu Thr
385                 390                 395                 400
Leu Arg Tyr Leu Met Ile His Leu Lys Lys Val Thr Met Asn Glu Lys
                405                 410                 415
Asp Asn Phe Met Asn Ala Glu Asn Leu Gly Ile Val Phe Gly Pro Thr
            420                 425                 430
Leu Met Arg Pro Pro Glu Asp Ser Thr Leu Thr Thr Leu His Asp Met
        435                 440                 445
Arg Tyr Gln Lys Leu Ile Val Gln Ile Leu Ile Glu Asn Glu Asp Val
    450                 455                 460
Leu Phe
465

<210> 318
<211> 304
<212> PRT
<213> Homo sapiens

<400> 318
Met Lys Arg Ala His Pro Glu Tyr Ser Ser Ser Asp Ser Glu Leu Asp
1               5                   10                  15
Glu Thr Ile Glu Val Glu Lys Glu Ser Ala Asp Glu Asn Gly Asn Leu
            20                  25                  30
Ser Ser Ala Leu Gly Ser Met Ser Pro Thr Thr Ser Ser Gln Ile Leu
        35                  40                  45
Ala Arg Lys Arg Arg Arg Gly Ile Ile Glu Lys Arg Arg Arg Asp Arg
    50                  55                  60
Ile Asn Asn Ser Leu Ser Glu Leu Arg Arg Leu Val Pro Ser Ala Phe
65                  70                  75                  80
Glu Lys Gln Gly Ser Ala Lys Leu Glu Lys Ala Glu Ile Leu Gln Met
                85                  90                  95
Thr Val Asp His Leu Lys Met Leu His Thr Ala Gly Gly Lys Gly Tyr
            100                 105                 110
Phe Asp Ala His Ala Leu Ala Met Asp Tyr Arg Ser Leu Gly Phe Arg
        115                 120                 125
Glu Cys Leu Ala Glu Val Ala Arg Tyr Leu Ser Ile Ile Glu Gly Leu
    130                 135                 140
Asp Ala Ser Asp Pro Leu Arg Val Arg Leu Val Ser His Leu Asn Asn
145                 150                 155                 160
Tyr Ala Ser Gln Arg Glu Ala Ala Ser Gly Ala His Ala Gly Leu Gly
                165                 170                 175
His Ile Pro Trp Gly Thr Val Phe Gly His His Pro His Ile Ala His
            180                 185                 190
Pro Leu Leu Leu Pro Gln Asn Gly His Gly Asn Ala Gly Thr Thr Ala
        195                 200                 205
Ser Pro Thr Glu Pro His His Gln Gly Arg Leu Gly Ser Ala His Pro
    210                 215                 220
Glu Ala Pro Ala Leu Arg Ala Pro Pro Ser Gly Ser Leu Gly Pro Val
225                 230                 235                 240
Leu Pro Val Val Thr Ser Ala Ser Lys Leu Ser Pro Pro Leu Leu Ser
                245                 250                 255
Ser Val Ala Ser Leu Ser Ala Phe Pro Phe Ser Phe Gly Ser Phe His
            260                 265                 270
Leu Leu Ser Pro Asn Ala Leu Ser Pro Ser Ala Pro Thr Gln Ala Ala
        275                 280                 285
Asn Leu Gly Lys Pro Tyr Arg Pro Trp Gly Thr Glu Ile Gly Ala Phe
    290                 295                 300

<210> 319
<211> 803
<212> PRT
<213> Homo sapiens

<400> 319
Met Thr Thr Ala Lys Glu Pro Ser Ala Ser Gly Lys Ser Val Gln Gln
1               5                   10                  15
Gln Glu Gln Glu Leu Val Gly Ser Asn Pro Pro Gln Arg Asn Trp Lys
            20                  25                  30
Gly Ile Ala Ile Ala Leu Leu Val Ile Leu Val Ile Cys Ser Leu Ile
        35                  40                  45
Val Thr Ser Val Ile Leu Leu Thr Pro Ala Glu Asp Asn Ser Leu Ser
    50                  55                  60
Gln Lys Lys Lys Val Thr Val Glu Asp Leu Phe Ser Glu Asp Phe Lys
65                  70                  75                  80
Ile His Asp Pro Glu Ala Lys Trp Ile Ser Asp Thr Glu Phe Ile Tyr
                85                  90                  95
Arg Glu Gln Lys Gly Thr Val Arg Leu Trp Asn Val Glu Thr Asn Thr
            100                 105                 110
Ser Thr Val Leu Ile Glu Gly Lys Lys Ile Glu Ser Leu Arg Ala Ile
        115                 120                 125
Arg Tyr Glu Ile Ser Pro Asp Arg Glu Tyr Ala Leu Phe Ser Tyr Asn
    130                 135                 140
Val Glu Pro Ile Tyr Gln His Ser Tyr Thr Gly Tyr Tyr Val Leu Ser
145                 150                 155                 160
Lys Ile Pro His Gly Asp Pro Gln Ser Leu Asp Pro Pro Glu Val Ser
                165                 170                 175
Asn Ala Lys Leu Gln Tyr Ala Gly Trp Gly Pro Lys Gly Gln Gln Leu
            180                 185                 190
Ile Phe Ile Phe Glu Asn Asn Ile Tyr Tyr Cys Ala His Val Gly Lys
        195                 200                 205
Gln Ala Ile Arg Val Val Ser Thr Gly Lys Glu Gly Val Ile Tyr Asn
    210                 215                 220
Gly Leu Ser Asp Trp Leu Tyr Glu Glu Glu Ile Leu Lys Thr His Ile
225                 230                 235                 240
Ala His Trp Trp Ser Pro Asp Gly Thr Arg Leu Ala Tyr Ala Ala Ile
                245                 250                 255
Asn Asp Ser Arg Val Pro Ile Met Glu Leu Pro Thr Tyr Thr Gly Ser
            260                 265                 270
Ile Tyr Pro Thr Val Lys Pro Tyr His Tyr Pro Lys Ala Gly Ser Glu
        275                 280                 285
Asn Pro Ser Ile Ser Leu His Val Ile Gly Leu Asn Gly Pro Thr His
    290                 295                 300
Asp Leu Glu Met Met Pro Pro Asp Asp Pro Arg Met Arg Glu Tyr Tyr
305                 310                 315                 320
Ile Thr Met Val Lys Trp Ala Thr Ser Thr Lys Val Ala Val Thr Trp
                325                 330                 335
Leu Asn Arg Ala Gln Asn Val Ser Ile Leu Thr Leu Cys Asp Ala Thr
            340                 345                 350
Thr Gly Val Cys Thr Lys Lys His Glu Asp Glu Ser Glu Ala Trp Leu
        355                 360                 365
His Arg Gln Asn Glu Glu Pro Val Phe Ser Lys Asp Gly Arg Lys Phe
    370                 375                 380
Phe Phe Ile Arg Ala Ile Pro Gln Gly Gly Arg Gly Lys Phe Tyr His
385                 390                 395                 400
Ile Thr Val Ser Ser Ser Gln Pro Asn Ser Ser Asn Asp Asn Ile Gln
                405                 410                 415
Ser Ile Thr Ser Gly Asp Trp Asp Val Thr Lys Ile Leu Ala Tyr Asp
            420                 425                 430
Glu Lys Gly Asn Lys Ile Tyr Phe Leu Ser Thr Glu Asp Leu Pro Arg
        435                 440                 445
Arg Arg Gln Leu Tyr Ser Ala Asn Thr Val Gly Asn Phe Asn Arg Gln

450                 455                 460
Cys Leu Ser Cys Asp Leu Val Glu Asn Cys Thr Tyr Phe Ser Ala Ser
465                 470                 475                 480
Phe Ser His Ser Met Asp Phe Phe Leu Leu Lys Cys Glu Gly Pro Gly
                485                 490                 495
Val Pro Met Val Thr Val His Asn Thr Thr Asp Lys Lys Lys Met Phe
            500                 505                 510
Asp Leu Glu Thr Asn Glu His Val Lys Lys Ala Ile Asn Asp Arg Gln
        515                 520                 525
Met Pro Lys Val Glu Tyr Arg Asp Ile Glu Ile Asp Asp Tyr Asn Leu
    530                 535                 540
Pro Met Gln Ile Leu Lys Pro Ala Thr Phe Thr Asp Thr Thr His Tyr
545                 550                 555                 560
Pro Leu Leu Leu Val Val Asp Gly Thr Pro Gly Ser Gln Ser Val Ala
                565                 570                 575
Glu Lys Phe Glu Val Ser Trp Glu Thr Val Met Val Ser Ser His Gly
            580                 585                 590
Ala Val Val Val Lys Cys Asp Gly Arg Gly Ser Gly Phe Gln Gly Thr
        595                 600                 605
Lys Leu Leu His Glu Val Arg Arg Arg Leu Gly Leu Leu Glu Glu Lys
    610                 615                 620
Asp Gln Met Glu Ala Val Arg Thr Met Leu Lys Glu Gln Tyr Ile Asp
625                 630                 635                 640
Arg Thr Arg Val Ala Val Phe Gly Lys Asp Tyr Gly Gly Tyr Leu Ser
                645                 650                 655
Thr Tyr Ile Leu Pro Ala Lys Gly Glu Asn Gln Gly Gln Thr Phe Thr
            660                 665                 670
Cys Gly Ser Ala Leu Ser Pro Ile Thr Asp Phe Lys Leu Tyr Ala Ser
        675                 680                 685
Ala Phe Ser Glu Arg Tyr Leu Gly Leu His Gly Leu Asp Asn Arg Ala
    690                 695                 700
Tyr Glu Met Thr Lys Val Ala His Arg Val Ser Ala Leu Glu Glu Gln
705                 710                 715                 720
Gln Phe Leu Ile Ile His Pro Thr Ala Asp Glu Lys Ile His Phe Gln
                725                 730                 735
His Thr Ala Glu Leu Ile Thr Gln Leu Ile Arg Gly Lys Ala Asn Tyr
            740                 745                 750
Ser Leu Gln Ile Tyr Pro Asp Glu Ser His Tyr Phe Thr Ser Ser Ser
        755                 760                 765
Leu Lys Gln His Leu Tyr Arg Ser Ile Ile Asn Phe Phe Val Glu Cys
    770                 775                 780
Phe Arg Ile Gln Asp Lys Leu Pro Thr Val Thr Ala Lys Glu Asp Glu
785                 790                 795                 800
Glu Glu Asp

<210> 320
<211> 299
<212> PRT
<213> Homo sapiens

<400> 320
Met Leu Pro Leu Leu Ala Ala Leu Leu Ala Ala Ala Cys Pro Leu Pro
1               5                   10                  15
Pro Val Arg Gly Gly Ala Ala Asp Ala Pro Gly Leu Leu Gly Val Pro
            20                  25                  30
Ser Asn Ala Ser Val Asn Ala Ser Ser Ala Ala Ser Pro Ser Pro Arg
        35                  40                  45
Gly Cys Trp Pro Arg Arg Pro Pro Gly Pro Pro Ser Ala Arg Ala Arg
    50                  55                  60
Arg Arg Arg Arg Arg Arg Arg Arg Leu Cys Asn Ile Ser Val Gln Arg
65                  70                  75                  80
Gln Met Leu Ser Ser Leu Leu Val Arg Trp Gly Arg Pro Arg Gly Phe
                85                  90                  95

Gln Cys Asp Leu Leu Leu Phe Ser Thr Asn Ala His Gly Arg Ala Phe
            100                 105                 110
Phe Ala Ala Ala Phe His Arg Val Gly Pro Pro Leu Leu Ile Glu His
        115                 120                 125
Leu Gly Leu Ala Ala Gly Gly Ala Gln Gln Asp Leu Arg Leu Cys Val
    130                 135                 140
Gly Cys Gly Trp Val Arg Gly Arg Arg Thr Gly Arg Leu Arg Pro Ala
145                 150                 155                 160
Ala Ala Pro Ser Ala Ala Ala Ala Thr Ala Gly Ala Pro Thr Ala Leu
                165                 170                 175
Pro Ala Tyr Pro Ala Ala Glu Pro Pro Gly Pro Leu Trp Leu Gln Gly
            180                 185                 190
Glu Pro Leu His Phe Cys Cys Leu Asp Phe Ser Leu Glu Glu Leu Gln
        195                 200                 205
Gly Glu Pro Gly Trp Arg Leu Asn Arg Lys Pro Ile Glu Ser Thr Leu
    210                 215                 220
Val Ala Cys Phe Met Thr Leu Val Ile Val Val Trp Ser Val Ala Ala
225                 230                 235                 240
Leu Ile Trp Pro Val Pro Ile Ile Ala Gly Phe Leu Pro Asn Gly Met
                245                 250                 255
Glu Gln Arg Arg Thr Thr Ala Ser Thr Thr Ala Ala Thr Pro Ala Ala
            260                 265                 270
Val Pro Ala Gly Thr Thr Ala Ala Ala Ala Ala Ala Ala Ala Ala Ala
        275                 280                 285
Ala Ala Ala Val Thr Ser Gly Val Ala Thr Lys
    290                 295

<210> 321
<211> 280
<212> PRT
<213> Homo sapiens

<400> 321
Met Asp Leu Gln Gly Arg Gly Val Pro Ser Ile Asp Arg Leu Arg Val
1               5                   10                  15
Leu Leu Met Leu Phe His Thr Met Ala Gln Ile Met Ala Glu Gln Glu
            20                  25                  30
Val Glu Asn Leu Ser Gly Leu Ser Thr Asn Pro Glu Lys Asp Ile Phe
        35                  40                  45
Val Val Arg Glu Asn Gly Thr Thr Cys Leu Met Ala Glu Phe Ala Ala
    50                  55                  60
Lys Phe Ile Val Pro Tyr Asp Val Trp Ala Ser Asn Tyr Val Asp Leu
65                  70                  75                  80
Ile Thr Glu Gln Ala Asp Ile Ala Leu Thr Arg Gly Ala Glu Val Lys
                85                  90                  95
Gly Arg Cys Gly His Ser Gln Ser Glu Leu Gln Val Phe Trp Val Asp
            100                 105                 110

Arg Ala Tyr Ala Leu Lys Met Leu Phe Val Lys Glu Ser His Asn Met
        115                 120                 125
Ser Lys Gly Pro Glu Ala Thr Trp Arg Leu Ser Lys Val Gln Phe Val
    130                 135                 140
Tyr Asp Ser Ser Glu Lys Thr His Phe Lys Asp Ala Val Ser Ala Gly
145                 150                 155                 160
Lys His Thr Ala Asn Ser His His Leu Ser Ala Leu Val Thr Pro Ala
                165                 170                 175
Gly Lys Ser Tyr Glu Cys Gln Ala Gln Gln Thr Ile Ser Leu Ala Ser
            180                 185                 190
Ser Asp Pro Gln Lys Thr Val Thr Met Ile Leu Ser Ala Val His Ile
        195                 200                 205
Gln Pro Phe Asp Ile Ile Ser Asp Phe Val Phe Ser Glu Glu His Lys
    210                 215                 220
Cys Pro Val Asp Glu Arg Glu Gln Leu Glu Glu Thr Leu Pro Leu Ile

225                 230                 235                 240
Leu Gly Leu Ile Leu Gly Leu Val Ile Met Val Thr Leu Ala Ile Tyr
                245                 250                 255
His Val His His Lys Met Thr Ala Asn Gln Val Gln Ile Pro Arg Asp
            260                 265                 270
Arg Ser Gln Tyr Lys His Met Gly
        275                 280

<210> 322
<211> 141
<212> PRT
<213> Homo sapiens

<400> 322
Met Trp Val Leu Gly Ile Ala Ala Thr Phe Cys Gly Leu Phe Leu Leu
 1               5                  10                  15
Pro Gly Phe Ala Leu Gln Ile Gln Cys Tyr Gln Cys Glu Glu Phe Gln
            20                  25                  30
Leu Asn Asn Asp Cys Ser Ser Pro Glu Phe Ile Val Asn Cys Thr Val
        35                  40                  45
Asn Val Gln Asp Met Cys Gln Lys Glu Val Met Glu Gln Ser Ala Gly
    50                  55                  60
Ile Met Tyr Arg Lys Ser Cys Ala Ser Ser Ala Ala Cys Leu Ile Ala
65                  70                  75                  80
Ser Ala Gly Tyr Gln Ser Phe Cys Ser Pro Gly Lys Leu Asn Ser Val
                85                  90                  95
Cys Ile Ser Cys Cys Asn Thr Pro Leu Cys Asn Gly Pro Arg Pro Lys
            100                 105                 110
Lys Arg Gly Ser Ser Ala Ser Ala Leu Arg Pro Gly Leu Arg Thr Thr
        115                 120                 125
Ile Leu Phe Leu Lys Leu Ala Leu Phe Ser Ala His Cys
    130                 135                 140

<210> 323
<211> 523
<212> PRT
<213> Homo sapiens

<400> 323
Met Thr Gln Asn Lys Leu Lys Leu Cys Ser Lys Ala Asn Val Tyr Thr
 1               5                  10                  15
Glu Val Pro Asp Gly Gly Trp Gly Trp Ala Val Ala Val Ser Phe Phe
            20                  25                  30
Phe Val Glu Val Phe Thr Tyr Gly Ile Ile Lys Thr Phe Gly Val Phe
        35                  40                  45

Phe Asn Asp Leu Met Asp Ser Phe Asn Glu Ser Asn Ser Arg Ile Ser
    50                  55                  60
Trp Ile Ile Ser Ile Cys Val Phe Val Leu Thr Phe Ser Ala Pro Leu
65                  70                  75                  80
Ala Thr Val Leu Ser Asn Arg Phe Gly His Arg Leu Val Val Met Leu
                85                  90                  95
Gly Gly Leu Leu Val Ser Thr Gly Met Val Ala Ala Ser Phe Ser Gln
            100                 105                 110
Glu Val Ser His Met Tyr Val Ala Ile Gly Ile Ile Ser Gly Leu Gly
        115                 120                 125
Tyr Cys Phe Ser Phe Leu Pro Thr Val Thr Ile Leu Ser Gln Tyr Phe
    130                 135                 140
Gly Lys Arg Arg Ser Ile Val Thr Ala Val Ala Ser Thr Gly Glu Cys
145                 150                 155                 160
Phe Ala Val Phe Ala Phe Ala Pro Ala Ile Met Ala Leu Lys Glu Arg
                165                 170                 175
Ile Gly Trp Arg Tyr Ser Leu Leu Phe Val Gly Leu Leu Gln Leu Asn

                180                 185                 190
Ile Val Ile Phe Gly Ala Leu Leu Arg Pro Ile Ile Ile Arg Gly Pro
        195                 200                 205
Ala Ser Pro Lys Ile Val Ile Gln Glu Asn Arg Lys Glu Ala Gln Tyr
    210                 215                 220
Met Leu Glu Asn Glu Lys Thr Arg Thr Ser Ile Asp Ser Ile Asp Ser
225                 230                 235                 240
Gly Val Glu Leu Thr Thr Ser Pro Lys Asn Val Pro Thr His Thr Asn
                245                 250                 255
Leu Glu Leu Glu Pro Lys Ala Asp Met Gln Gln Val Leu Val Lys Thr
            260                 265                 270
Ser Pro Arg Pro Ser Glu Lys Lys Ala Pro Leu Leu Asp Phe Ser Ile
        275                 280                 285
Leu Lys Glu Lys Ser Phe Ile Cys Tyr Ala Leu Phe Gly Leu Phe Ala
    290                 295                 300
Thr Leu Gly Phe Phe Ala Pro Ser Leu Tyr Ile Ile Pro Leu Gly Ile
305                 310                 315                 320
Ser Leu Gly Ile Asp Gln Asp Arg Ala Ala Phe Leu Leu Ser Thr Met
                325                 330                 335
Ala Ile Ala Glu Val Phe Gly Arg Ile Gly Ala Gly Phe Val Leu Asn
            340                 345                 350
Arg Glu Pro Ile Arg Lys Ile Tyr Ile Glu Leu Ile Cys Val Ile Leu
        355                 360                 365
Leu Thr Val Ser Leu Phe Ala Phe Thr Phe Ala Thr Glu Phe Trp Gly
    370                 375                 380
Leu Met Ser Cys Ser Ile Phe Phe Gly Phe Met Val Gly Thr Ile Gly
385                 390                 395                 400
Gly Leu Thr Phe His Cys Leu Leu Lys Met Met Ser Trp Ala Leu Gln
                405                 410                 415
Lys Met Ser Ser Ala Ala Gly Val Tyr Ile Phe Ile Gln Ser Ile Ala
            420                 425                 430
Gly Leu Ala Gly Pro Pro Leu Ala Gly Leu Leu Val Asp Gln Ser Lys
        435                 440                 445
Ile Tyr Ser Arg Ala Phe Tyr Ser Cys Ala Ala Gly Met Ala Leu Ala
    450                 455                 460
Ala Val Cys Leu Ala Leu Val Arg Pro Cys Lys Met Gly Leu Cys Gln
465                 470                 475                 480
Arg His His Ser Gly Glu Thr Lys Val Val Ser His Arg Gly Lys Thr
                485                 490                 495
Leu Gln Asp Ile Pro Glu Asp Phe Leu Glu Met Asp Leu Ala Lys Asn
            500                 505                 510
Glu His Arg Val His Val Gln Met Glu Pro Val
        515                 520

<210> 324
<211> 343
<212> PRT
<213> Homo sapiens

<400> 324
Met Glu Ser Lys Tyr Lys Glu Ile Leu Leu Leu Thr Gly Leu Asp Asn
1               5                   10                  15
Ile Thr Asp Glu Glu Leu Asp Arg Phe Lys Phe Phe Leu Ser Asp Glu
            20                  25                  30
Phe Asn Ile Ala Thr Gly Lys Leu His Thr Ala Asn Arg Ile Gln Val
        35                  40                  45
Ala Thr Leu Met Ile Gln Asn Ala Gly Ala Val Ser Ala Val Met Lys
    50                  55                  60
Thr Ile Arg Ile Phe Gln Lys Leu Asn Tyr Met Leu Leu Ala Lys Arg
65                  70                  75                  80
Leu Gln Glu Glu Lys Glu Lys Val Asp Lys Gln Tyr Lys Ser Val Thr
                85                  90                  95
Lys Pro Lys Pro Leu Ser Gln Ala Glu Met Ser Pro Ala Ala Ser Ala

                100                 105                 110
Ala Ile Arg Asn Asp Val Ala Lys Gln Arg Ala Ala Pro Lys Val Ser
        115                 120                 125
Pro His Val Lys Pro Glu Gln Lys Gln Met Val Ala Gln Gln Glu Ser
    130                 135                 140
Ile Arg Glu Gly Phe Gln Lys Arg Cys Leu Pro Val Met Val Leu Lys
145                 150                 155                 160
Ala Lys Lys Pro Phe Thr Phe Glu Thr Gln Glu Gly Lys Gln Glu Met
                165                 170                 175
Phe His Ala Thr Val Ala Thr Glu Lys Glu Phe Phe Phe Val Lys Val
            180                 185                 190
Phe Asn Thr Leu Leu Lys Asp Lys Phe Ile Pro Lys Arg Ile Ile Ile
        195                 200                 205
Ile Ala Arg Tyr Tyr Arg His Ser Gly Phe Leu Glu Val Asn Ser Ala
    210                 215                 220
Ser Arg Val Leu Asp Ala Glu Ser Asp Gln Lys Val Asn Val Pro Leu
225                 230                 235                 240
Asn Ile Ile Arg Lys Ala Gly Glu Thr Pro Lys Ile Asn Thr Leu Gln
                245                 250                 255
Thr Gln Pro Leu Gly Thr Ile Val Asn Gly Leu Phe Val Val Gln Lys
            260                 265                 270
Val Thr Glu Lys Lys Lys Asn Ile Leu Phe Asp Leu Ser Asp Asn Thr
        275                 280                 285
Gly Lys Met Glu Val Leu Gly Val Arg Asn Glu Asp Thr Met Lys Cys
    290                 295                 300
Lys Glu Gly Asp Lys Val Arg Leu Thr Phe Phe Thr Leu Ser Lys Asn
305                 310                 315                 320
Gly Glu Lys Leu Gln Leu Thr Ser Gly Val His Ser Thr Ile Lys Val
                325                 330                 335
Ile Lys Ala Lys Lys Lys Thr
            340

<210> 325
<211> 94
<212> PRT
<213> Homo sapiens

<400> 325
Met Ser Val Lys Gly Met Ala Ile Ala Leu Ala Val Ile Leu Cys Ala
 1               5                  10                  15
Thr Val Val Gln Gly Phe Pro Met Phe Lys Arg Gly Arg Cys Leu Cys
            20                  25                  30
Ile Gly Pro Gly Val Lys Ala Val Lys Val Ala Asp Ile Glu Lys Ala
        35                  40                  45
Ser Ile Met Tyr Pro Ser Asn Asn Cys Asp Lys Ile Glu Val Ile Ile
    50                  55                  60
Thr Leu Lys Glu Asn Lys Gly Gln Arg Cys Leu Asn Pro Lys Ser Lys
65                  70                  75                  80
Gln Ala Arg Leu Ile Ile Lys Lys Val Glu Arg Lys Asn Phe
                85                  90

<210> 326
<211> 595
<212> PRT
<213> Homo sapiens

<400> 326
Met Pro Ala Cys Cys Ser Cys Ser Asp Val Phe Gln Tyr Glu Thr Asn
 1               5                  10                  15
Lys Val Thr Arg Ile Gln Ser Met Asn Tyr Gly Thr Ile Lys Trp Phe
            20                  25                  30
Phe His Val Ile Ile Phe Ser Tyr Val Cys Phe Ala Leu Val Ser Asp
        35                  40                  45

Lys Leu Tyr Gln Arg Lys Glu Pro Val Ile Ser Ser Val His Thr Lys
        50                  55                  60
Val Lys Gly Ile Ala Glu Val Lys Glu Glu Ile Val Glu Asn Gly Val
65                  70                  75                  80
Lys Lys Leu Val His Ser Val Phe Asp Thr Ala Asp Tyr Thr Phe Pro
                85                  90                  95
Leu Gln Gly Asn Ser Phe Phe Val Met Thr Asn Phe Leu Lys Thr Glu
            100                 105                 110
Gly Gln Glu Gln Arg Leu Cys Pro Glu Tyr Pro Thr Arg Arg Thr Leu
        115                 120                 125
Cys Ser Ser Asp Arg Gly Cys Lys Lys Gly Trp Met Asp Pro Gln Ser
    130                 135                 140
Lys Gly Ile Gln Thr Gly Arg Cys Val Val His Glu Gly Asn Gln Lys
145                 150                 155                 160
Thr Cys Glu Val Ser Ala Trp Cys Pro Ile Glu Ala Val Glu Glu Ala
                165                 170                 175
Pro Arg Pro Ala Leu Leu Asn Ser Ala Glu Asn Phe Thr Val Leu Ile
            180                 185                 190
Lys Asn Asn Ile Asp Phe Pro Gly His Asn Tyr Thr Thr Arg Asn Ile
        195                 200                 205
Leu Pro Gly Leu Asn Ile Thr Cys Thr Phe His Lys Thr Gln Asn Pro
    210                 215                 220
Gln Cys Pro Ile Phe Arg Leu Gly Asp Ile Phe Arg Glu Thr Gly Asp
225                 230                 235                 240
Asn Phe Ser Asp Val Ala Ile Gln Gly Gly Ile Met Gly Ile Glu Ile
                245                 250                 255
Tyr Trp Asp Cys Asn Leu Asp Arg Trp Phe His His Cys His Pro Lys
            260                 265                 270
Tyr Ser Phe Arg Arg Leu Asp Asp Lys Thr Thr Asn Val Ser Leu Tyr
        275                 280                 285
Pro Gly Tyr Asn Phe Arg Tyr Ala Lys Tyr Tyr Lys Glu Asn Asn Val
    290                 295                 300
Glu Lys Arg Thr Leu Ile Lys Val Phe Gly Ile Arg Phe Asp Ile Leu
305                 310                 315                 320
Val Phe Gly Thr Gly Gly Lys Phe Asp Ile Ile Gln Leu Val Val Tyr
                325                 330                 335
Ile Gly Ser Thr Leu Ser Tyr Phe Gly Leu Ala Ala Val Phe Ile Asp
            340                 345                 350
Phe Leu Ile Asp Thr Tyr Ser Ser Asn Cys Cys Arg Ser His Ile Tyr
        355                 360                 365
Pro Trp Cys Lys Cys Cys Gln Pro Cys Val Val Asn Glu Tyr Tyr Tyr
    370                 375                 380
Arg Lys Lys Cys Glu Ser Ile Val Glu Pro Lys Pro Thr Leu Lys Tyr
385                 390                 395                 400
Val Ser Phe Val Asp Glu Ser His Ile Arg Met Val Asn Gln Gln Leu
                405                 410                 415
Leu Gly Arg Ser Leu Gln Asp Val Lys Gly Gln Glu Val Pro Arg Pro
            420                 425                 430
Ala Met Asp Phe Thr Asp Leu Ser Arg Leu Pro Leu Ala Leu His Asp
        435                 440                 445
Thr Pro Pro Ile Pro Gly Gln Pro Glu Glu Ile Gln Leu Leu Arg Lys
    450                 455                 460
Glu Ala Thr Pro Arg Ser Arg Asp Ser Pro Val Trp Cys Gln Cys Gly
465                 470                 475                 480
Ser Cys Leu Pro Ser Gln Leu Pro Glu Ser His Arg Cys Leu Glu Glu
                485                 490                 495
Leu Cys Cys Arg Lys Lys Pro Gly Ala Cys Ile Thr Thr Ser Glu Leu
            500                 505                 510
Phe Arg Lys Leu Val Leu Ser Arg His Val Leu Gln Phe Leu Leu Leu
        515                 520                 525
Tyr Gln Glu Pro Leu Leu Ala Leu Asp Val Asp Ser Thr Asn Ser Arg
    530                 535                 540
Leu Arg His Cys Ala Tyr Arg Cys Tyr Ala Thr Trp Arg Phe Gly Ser

545                 550                 555                 560
Gln Asp Met Ala Asp Phe Ala Ile Leu Pro Ser Cys Cys Arg Trp Arg
                565                 570                 575
Ile Arg Lys Glu Phe Pro Lys Ser Glu Gly Gln Tyr Ser Gly Phe Lys
            580                 585                 590
Ser Pro Tyr
        595

<210> 327
<211> 829
<212> PRT
<213> Homo sapiens

<400> 327

<210> 328
<211> 162
<212> PRT
<213> Homo sapiens

<400> 328

Met Asp Ile Pro Gln Thr Lys Gln Asp Leu Glu Leu Pro Lys Leu Ala
1               5                   10                  15
Gly Thr Trp His Ser Met Ala Met Ala Thr Asn Asn Ile Ser Leu Met
            20                  25                  30
Ala Thr Leu Lys Ala Pro Leu Arg Val His Ile Thr Ser Leu Leu Pro
        35                  40                  45
Thr Pro Glu Asp Asn Leu Glu Ile Val Leu His Arg Trp Glu Asn Asn
    50                  55                  60
Ser Cys Val Glu Lys Lys Val Leu Gly Glu Lys Thr Gly Asn Pro Lys
65                  70                  75                  80
Lys Phe Lys Ile Asn Tyr Thr Val Ala Asn Glu Ala Thr Leu Leu Asp
                85                  90                  95
Thr Asp Tyr Asp Asn Phe Leu Phe Leu Cys Leu Gln Asp Thr Thr Thr
            100                 105                 110
Pro Ile Gln Ser Met Met Cys Gln Tyr Leu Ala Arg Val Leu Val Glu
        115                 120                 125
Asp Asp Glu Ile Met Gln Gly Phe Ile Arg Ala Phe Arg Pro Leu Pro
    130                 135                 140
Arg His Leu Trp Tyr Leu Leu Asp Leu Lys Gln Met Glu Glu Pro Cys
145                 150                 155                 160
Arg Phe

<210> 329
<211> 180
<212> PRT
<213> Homo sapiens

<400> 329
Met Gln Ala Glu Gly Arg Gly Thr Gly Gly Ser Thr Gly Asp Ala Asp
1               5                   10                  15
Gly Pro Gly Gly Pro Gly Ile Pro Asp Gly Pro Gly Gly Asn Ala Gly
            20                  25                  30
Gly Pro Gly Glu Ala Gly Ala Thr Gly Gly Arg Gly Pro Arg Gly Ala
        35                  40                  45
Gly Ala Ala Arg Ala Ser Gly Pro Gly Gly Gly Ala Pro Arg Gly Pro
    50                  55                  60
His Gly Gly Ala Ala Ser Gly Leu Asn Gly Cys Cys Arg Cys Gly Ala
65                  70                  75                  80
Arg Gly Pro Glu Ser Arg Leu Leu Glu Phe Tyr Leu Ala Met Pro Phe
                85                  90                  95
Ala Thr Pro Met Glu Ala Glu Leu Ala Arg Arg Ser Leu Ala Gln Asp
            100                 105                 110
Ala Pro Pro Leu Pro Val Pro Gly Val Leu Leu Lys Glu Phe Thr Val
        115                 120                 125
Ser Gly Asn Ile Leu Thr Ile Arg Leu Thr Ala Ala Asp His Arg Gln
    130                 135                 140
Leu Gln Leu Ser Ile Ser Ser Cys Leu Gln Gln Leu Ser Leu Leu Met
145                 150                 155                 160
Trp Ile Thr Gln Cys Phe Leu Pro Val Phe Leu Ala Gln Pro Pro Ser
                165                 170                 175
Gly Gln Arg Arg
            180

<210> 330
<211> 135
<212> PRT
<213> Homo sapiens

<400> 330
Met Gln Ala Glu Gly Gln Gly Thr Gly Gly Ser Thr Gly Asp Ala Asp
1               5                   10                  15
Gly Pro Gly Gly Pro Gly Ile Pro Asp Gly Pro Gly Gly Asn Ala Gly
            20                  25                  30

Gly Pro Gly Glu Ala Gly Ala Thr Gly Gly Arg Gly Pro Arg Gly Ala
        35                  40                  45
Gly Ala Ala Arg Ala Ser Gly Pro Arg Gly Gly Ala Pro Arg Gly Pro
    50                  55                  60
His Gly Gly Ala Ala Ser Ala Gln Asp Gly Arg Cys Pro Cys Gly Ala
65                  70                  75                  80
Arg Arg Pro Asp Ser Arg Leu Leu Gln Phe Arg Leu Thr Ala Ala Asp
                85                  90                  95
His Arg Gln Leu Gln Leu Ser Ile Ser Ser Cys Leu Gln Gln Leu Ser
            100                 105                 110
Leu Leu Met Trp Ile Thr Gln Cys Phe Leu Pro Val Phe Leu Ala Gln
        115                 120                 125
Ala Pro Ser Gly Gln Arg Arg
    130                 135

<210> 331
<211> 661
<212> PRT
<213> Homo sapiens

<400> 331
Met Asp Leu Val Leu Lys Arg Cys Leu Leu His Leu Ala Val Ile Gly
 1               5                  10                  15
Ala Leu Leu Ala Val Gly Ala Thr Lys Val Pro Arg Asn Gln Asp Trp
            20                  25                  30
Leu Gly Val Ser Arg Gln Leu Arg Thr Lys Ala Trp Asn Arg Gln Leu
        35                  40                  45
Tyr Pro Glu Trp Thr Glu Ala Gln Arg Leu Asp Cys Trp Arg Gly Gly
    50                  55                  60
Gln Val Ser Leu Lys Val Ser Asn Asp Gly Pro Thr Leu Ile Gly Ala
65                  70                  75                  80

Asn Ala Ser Phe Ser Ile Ala Leu Asn Phe Pro Gly Ser Gln Lys Val
                85                  90                  95
Leu Pro Asp Gly Gln Val Ile Trp Val Asn Asn Thr Ile Ile Asn Gly
            100                 105                 110
Ser Gln Val Trp Gly Gly Gln Pro Val Tyr Pro Gln Glu Thr Asp Asp
        115                 120                 125
Ala Cys Ile Phe Pro Asp Gly Gly Pro Cys Pro Ser Gly Ser Trp Ser
    130                 135                 140
Gln Lys Arg Ser Phe Val Tyr Val Trp Lys Thr Trp Gly Gln Tyr Trp
145                 150                 155                 160
Gln Val Leu Gly Gly Pro Val Ser Gly Leu Ser Ile Gly Thr Gly Arg
                165                 170                 175
Ala Met Leu Gly Thr His Thr Met Glu Val Thr Val Tyr His Arg Arg
            180                 185                 190
Gly Ser Arg Ser Tyr Val Pro Leu Ala His Ser Ser Ser Ala Phe Thr
        195                 200                 205
Ile Thr Asp Gln Val Pro Phe Ser Val Ser Val Ser Gln Leu Arg Ala
    210                 215                 220
Leu Asp Gly Gly Asn Lys His Phe Leu Arg Asn Gln Pro Leu Thr Phe
225                 230                 235                 240
Ala Leu Gln Leu His Asp Pro Ser Gly Tyr Leu Ala Glu Ala Asp Leu
                245                 250                 255
Ser Tyr Thr Trp Asp Phe Gly Asp Ser Ser Gly Thr Leu Ile Ser Arg
            260                 265                 270
Ala Leu Val Val Thr His Thr Tyr Leu Glu Pro Gly Pro Val Thr Ala
        275                 280                 285
Gln Val Val Leu Gln Ala Ala Ile Pro Leu Thr Ser Cys Gly Ser Ser
    290                 295                 300
Pro Val Pro Gly Thr Thr Asp Gly His Arg Pro Thr Ala Glu Ala Pro
305                 310                 315                 320
Asn Thr Thr Ala Gly Gln Val Pro Thr Thr Glu Val Val Gly Thr Thr

                325                 330                 335
Pro Gly Gln Ala Pro Thr Ala Glu Pro Ser Gly Thr Thr Ser Val Gln
            340                 345                 350
Val Pro Thr Thr Glu Val Ile Ser Thr Ala Pro Val Gln Met Pro Thr
        355                 360                 365
Ala Glu Ser Thr Gly Met Thr Pro Glu Lys Val Pro Val Ser Glu Val
    370                 375                 380
Met Gly Thr Thr Leu Ala Glu Met Ser Thr Pro Glu Ala Thr Gly Met
385                 390                 395                 400
Thr Pro Ala Glu Val Ser Ile Val Val Leu Ser Gly Thr Thr Ala Ala
                405                 410                 415
Gln Val Thr Thr Thr Glu Trp Val Glu Thr Thr Ala Arg Glu Leu Pro
            420                 425                 430
Ile Pro Glu Pro Glu Gly Pro Asp Ala Ser Ser Ile Met Ser Thr Glu
        435                 440                 445
Ser Ile Thr Gly Ser Leu Gly Pro Leu Leu Asp Gly Thr Ala Thr Leu
    450                 455                 460
Arg Leu Val Lys Arg Gln Val Pro Leu Asp Cys Val Leu Tyr Arg Tyr
465                 470                 475                 480
Gly Ser Phe Ser Val Thr Leu Asp Ile Val Gln Gly Ile Glu Ser Ala
                485                 490                 495
Glu Ile Leu Gln Ala Val Pro Ser Gly Glu Gly Asp Ala Phe Glu Leu
            500                 505                 510
Thr Val Ser Cys Gln Gly Gly Leu Pro Lys Glu Ala Cys Met Glu Ile
        515                 520                 525
Ser Ser Pro Gly Cys Gln Pro Pro Ala Gln Arg Leu Cys Gln Pro Val
    530                 535                 540
Leu Pro Ser Pro Ala Cys Gln Leu Val Leu His Gln Ile Leu Lys Gly
545                 550                 555                 560

Gly Ser Gly Thr Tyr Cys Leu Asn Val Ser Leu Ala Asp Thr Asn Ser
                565                 570                 575
Leu Ala Val Val Ser Thr Gln Leu Ile Met Pro Gly Gln Glu Ala Gly
            580                 585                 590
Leu Gly Gln Val Pro Leu Ile Val Gly Ile Leu Leu Val Leu Met Ala
        595                 600                 605
Val Val Leu Ala Ser Leu Ile Tyr Arg Arg Arg Leu Met Lys Gln Asp
    610                 615                 620
Phe Ser Val Pro Gln Leu Pro His Ser Ser Ser His Trp Leu Arg Leu
625                 630                 635                 640
Pro Arg Ile Phe Cys Ser Cys Pro Ile Gly Glu Asn Ser Pro Leu Leu
                645                 650                 655
Ser Gly Gln Gln Val
            660

<210> 332
<211> 519
<212> PRT
<213> Homo sapiens

<400> 332
Met Ser Pro Leu Trp Trp Gly Phe Leu Leu Ser Cys Leu Gly Cys Lys
1               5                   10                  15
Ile Leu Pro Gly Ala Gln Gly Gln Phe Pro Arg Val Cys Met Thr Val
            20                  25                  30
Asp Ser Leu Val Asn Lys Glu Cys Cys Pro Arg Leu Gly Ala Glu Ser
        35                  40                  45
Ala Asn Val Cys Gly Ser Gln Gln Gly Arg Gly Gln Cys Thr Glu Val
    50                  55                  60
Arg Ala Asp Thr Arg Pro Trp Ser Gly Pro Tyr Ile Leu Arg Asn Gln
65                  70                  75                  80
Asp Asp Arg Glu Leu Trp Pro Arg Lys Phe Phe His Arg Thr Cys Lys
                85                  90                  95

Cys Thr Gly Asn Phe Ala Gly Tyr Asn Cys Gly Asp Cys Lys Phe Gly
            100                 105                 110
Trp Thr Gly Pro Asn Cys Glu Arg Lys Lys Pro Pro Val Ile Arg Gln
        115                 120                 125
Asn Ile His Ser Leu Ser Pro Gln Glu Arg Glu Gln Phe Leu Gly Ala
    130                 135                 140
Leu Asp Leu Ala Lys Lys Arg Val His Pro Asp Tyr Val Ile Thr Thr
145                 150                 155                 160
Gln His Trp Leu Gly Leu Leu Gly Pro Asn Gly Thr Gln Pro Gln Phe
                165                 170                 175
Ala Asn Cys Ser Val Tyr Asp Phe Phe Val Trp Leu His Tyr Tyr Ser
            180                 185                 190
Val Arg Asp Thr Leu Leu Gly Pro Gly Arg Pro Tyr Arg Ala Ile Asp
        195                 200                 205
Phe Ser His Gln Gly Pro Ala Phe Val Thr Trp His Arg Tyr His Leu
    210                 215                 220
Leu Cys Leu Glu Arg Asp Leu Gln Arg Leu Ile Gly Asn Glu Ser Phe
225                 230                 235                 240
Ala Leu Pro Tyr Trp Asn Phe Ala Thr Gly Arg Asn Glu Cys Asp Val
                245                 250                 255
Cys Thr Asp Gln Leu Phe Gly Ala Ala Arg Pro Asp Asp Pro Thr Leu
            260                 265                 270
Ile Ser Arg Asn Ser Arg Phe Ser Ser Trp Glu Thr Val Cys Asp Ser
        275                 280                 285
Leu Asp Asp Tyr Asn His Leu Val Thr Leu Cys Asn Gly Thr Tyr Glu
    290                 295                 300
Gly Leu Leu Arg Arg Asn Gln Met Gly Arg Asn Ser Met Lys Leu Pro
305                 310                 315                 320
Thr Leu Lys Asp Ile Arg Asp Cys Leu Ser Leu Gln Lys Phe Asp Asn
                325                 330                 335
Pro Pro Phe Phe Gln Asn Ser Thr Phe Ser Phe Arg Asn Ala Leu Glu
            340                 345                 350
Gly Phe Asp Lys Ala Asp Gly Thr Leu Asp Ser Gln Val Met Ser Leu
        355                 360                 365
His Asn Leu Val His Ser Phe Leu Asn Gly Thr Asn Ala Leu Pro His
    370                 375                 380
Ser Ala Ala Asn Asp Pro Ile Phe Val Val Leu His Ser Phe Thr Asp
385                 390                 395                 400
Ala Ile Phe Asp Glu Trp Met Lys Arg Phe Asn Pro Pro Ala Asp Ala
                405                 410                 415
Trp Pro Gln Glu Leu Ala Pro Ile Gly His Asn Arg Met Tyr Asn Met
            420                 425                 430
Val Pro Phe Phe Pro Pro Val Thr Asn Glu Glu Leu Phe Leu Thr Ser
        435                 440                 445
Asp Gln Leu Gly Tyr Ser Tyr Ala Ile Asp Leu Pro Val Ser Val Glu
    450                 455                 460
Glu Thr Pro Gly Trp Pro Thr Thr Leu Leu Val Val Met Gly Thr Leu
465                 470                 475                 480
Val Ala Leu Val Gly Leu Phe Val Leu Leu Ala Phe Leu Gln Tyr Arg
                485                 490                 495
Arg Leu Arg Lys Gly Tyr Thr Pro Leu Met Glu Thr His Leu Ser Ser
            500                 505                 510
Lys Arg Tyr Thr Glu Glu Ala
        515

<210> 333
<211> 397
<212> PRT
<213> Homo sapiens

<400> 333
Met Asn Trp His Leu Pro Leu Phe Leu Leu Ala Ser Val Thr Leu Pro
1               5                   10                  15

Ser Ile Cys Ser His Phe Asn Pro Leu Ser Leu Glu Glu Leu Gly Ser
            20                  25                  30
Asn Thr Gly Ile Gln Val Phe Asn Gln Ile Val Lys Ser Arg Pro His
        35                  40                  45
Asp Asn Ile Val Ile Ser Pro His Gly Ile Ala Ser Val Leu Gly Met
    50                  55                  60
Leu Gln Leu Gly Ala Asp Gly Arg Thr Lys Lys Gln Leu Ala Met Val
65                  70                  75                  80
Met Arg Tyr Gly Val Asn Gly Val Gly Lys Ile Leu Lys Lys Ile Asn
                85                  90                  95
Lys Ala Ile Val Ser Lys Lys Asn Lys Asp Ile Val Thr Val Ala Asn
            100                 105                 110
Ala Val Phe Val Lys Asn Ala Ser Glu Ile Glu Val Pro Phe Val Thr
        115                 120                 125
Arg Asn Lys Asp Val Phe Gln Cys Glu Val Arg Asn Val Asn Phe Glu
    130                 135                 140
Asp Pro Ala Ser Ala Cys Asp Ser Ile Asn Ala Trp Val Lys Asn Glu
145                 150                 155                 160
Thr Arg Asp Met Ile Asp Asn Leu Leu Ser Pro Asp Leu Ile Asp Gly
                165                 170                 175
Val Leu Thr Arg Leu Val Leu Val Asn Ala Val Tyr Phe Lys Gly Leu
            180                 185                 190
Trp Lys Ser Arg Phe Gln Pro Glu Asn Thr Lys Lys Arg Thr Phe Val
        195                 200                 205
Ala Ala Asp Gly Lys Ser Tyr Gln Val Pro Met Leu Ala Gln Leu Ser
    210                 215                 220
Val Phe Arg Cys Gly Ser Thr Ser Ala Pro Asn Asp Leu Trp Tyr Asn
225                 230                 235                 240
Phe Ile Glu Leu Pro Tyr His Gly Glu Ser Ile Ser Met Leu Ile Ala
                245                 250                 255
Leu Pro Thr Glu Ser Ser Thr Pro Leu Ser Ala Ile Ile Pro His Ile
            260                 265                 270
Ser Thr Lys Thr Ile Asp Ser Trp Met Ser Ile Met Val Pro Lys Arg
        275                 280                 285
Val Gln Val Ile Leu Pro Lys Phe Thr Ala Val Ala Gln Thr Asp Leu
    290                 295                 300
Lys Glu Pro Leu Lys Val Leu Gly Ile Thr Asp Met Phe Asp Ser Ser
305                 310                 315                 320
Lys Ala Asn Phe Ala Lys Ile Thr Arg Ser Glu Asn Leu His Val Ser
                325                 330                 335
His Ile Leu Gln Lys Ala Lys Ile Glu Val Ser Glu Asp Gly Thr Lys
            340                 345                 350
Ala Ser Ala Ala Thr Thr Ala Ile Leu Ile Ala Arg Ser Ser Pro Pro
        355                 360                 365
Trp Phe Ile Val Asp Arg Pro Phe Leu Phe Phe Ile Arg His Asn Pro
    370                 375                 380
Thr Gly Ala Val Leu Phe Met Gly Gln Ile Asn Lys Pro
385                 390                 395

<210> 334
<211> 306
<212> PRT
<213> Homo sapiens

<400> 334
Met Arg Gln His Asp Thr Arg Asn Gly Arg Ile Val Leu Ile Ser Gly
1               5                   10                  15
Arg Arg Ser Phe Cys Ser Ile Phe Ser Val Leu Pro Tyr Arg Asp Ser
            20                  25                  30
Thr Gln Val Gly Asp Leu Lys Leu Asp Gly Gly Arg Gln Ser Thr Gly
        35                  40                  45
Ala Val Ser Leu Lys Glu Ile Ile Gly Leu Glu Gly Val Glu Leu Gly
    50                  55                  60

Ala Asp Gly Lys Thr Val Ser Tyr Thr Gln Phe Leu Leu Pro Thr Asn
65                  70                  75                  80
Ala Phe Gly Ala Arg Arg Asn Thr Ile Asp Ser Thr Ser Ser Phe Ser
                85                  90                  95
Gln Phe Arg Asn Leu Ser His Arg Ser Leu Ser Ile Gly Arg Ala Ser
            100                 105                 110
Gly Thr Gln Gly Ser Leu Asp Thr Gly Ser Asp Leu Gly Asp Phe Met
        115                 120                 125
Asp Tyr Asp Pro Asn Leu Leu Asp Asp Pro Gln Trp Pro Cys Gly Lys
    130                 135                 140
His Lys Arg Val Leu Ile Phe Pro Ser Tyr Met Thr Thr Val Ile Asp
145                 150                 155                 160
Tyr Val Lys Pro Ser Asp Leu Lys Lys Asp Met Asn Glu Thr Phe Lys
                165                 170                 175
Glu Lys Phe Pro His Ile Lys Leu Thr Leu Ser Lys Ile Arg Ser Leu
            180                 185                 190
Lys Arg Glu Met Arg Lys Leu Ala Gln Glu Asp Cys Gly Leu Glu Glu
        195                 200                 205
Pro Thr Val Ala Met Ala Phe Val Tyr Phe Glu Lys Leu Ala Leu Lys
    210                 215                 220
Gly Lys Leu Asn Lys Gln Asn Arg Lys Leu Cys Ala Gly Ala Cys Val
225                 230                 235                 240
Leu Leu Ala Ala Lys Ile Gly Ser Asp Leu Lys Lys His Glu Val Lys
                245                 250                 255
His Leu Ile Asp Lys Leu Glu Glu Lys Phe Arg Leu Asn Arg Arg Glu
            260                 265                 270
Leu Ile Ala Phe Glu Phe Pro Val Leu Val Ala Leu Glu Phe Ala Leu
        275                 280                 285
His Leu Pro Glu His Glu Val Met Pro His Tyr Arg Arg Leu Val Gln
    290                 295                 300
Ser Ser
305

<210> 335
<211> 835
<212> PRT
<213> Homo sapiens

<400> 335
Met Thr Thr Leu Ala Gly Ala Val Pro Arg Met Met Arg Pro Gly Pro
1               5                   10                  15
Gly Gln Asn Tyr Pro Arg Ser Gly Phe Pro Leu Glu Val Ser Thr Pro
            20                  25                  30
Leu Gly Gln Gly Arg Val Asn Gln Leu Gly Gly Val Phe Ile Asn Gly
        35                  40                  45
Arg Pro Leu Pro Asn His Ile Arg His Lys Ile Val Glu Met Ala His
    50                  55                  60
His Gly Ile Arg Pro Cys Val Ile Ser Arg Gln Leu Arg Val Ser His
65                  70                  75                  80
Gly Cys Val Ser Lys Ile Leu Cys Arg Tyr Gln Glu Thr Gly Ser Ile
                85                  90                  95
Arg Pro Gly Ala Ile Gly Gly Ser Lys Pro Lys Val Thr Thr Pro Asp
            100                 105                 110
Val Glu Lys Lys Ile Glu Glu Tyr Lys Arg Glu Asn Pro Gly Met Phe
        115                 120                 125
Ser Trp Glu Ile Arg Asp Lys Leu Leu Lys Asp Ala Val Cys Asp Arg
    130                 135                 140
Asn Thr Val Pro Ser Val Ser Ser Ile Ser Arg Ile Leu Arg Ser Lys
145                 150                 155                 160
Phe Gly Lys Gly Glu Glu Glu Ala Asp Leu Glu Arg Lys Glu Ala
                165                 170                 175
Glu Glu Ser Glu Lys Lys Ala Lys His Ser Ile Asp Gly Ile Leu Ser
            180                 185                 190

Glu Arg Ala Ser Ala Pro Gln Ser Asp Glu Gly Ser Asp Ile Asp Ser
        195                 200                 205
Glu Pro Asp Leu Pro Leu Lys Arg Lys Gln Arg Arg Ser Arg Thr Thr
    210                 215                 220
Phe Thr Ala Glu Gln Leu Glu Glu Leu Glu Arg Ala Phe Glu Arg Thr
225                 230                 235                 240
His Tyr Pro Asp Ile Tyr Thr Arg Glu Glu Leu Ala Gln Arg Ala Lys
                245                 250                 255
Leu Thr Glu Ala Arg Val Gln Val Trp Phe Ser Asn Arg Arg Ala Arg
            260                 265                 270
Trp Arg Lys Gln Ala Gly Ala Asn Gln Leu Met Ala Phe Asn His Leu
        275                 280                 285
Ile Pro Gly Gly Phe Pro Pro Thr Ala Met Pro Thr Leu Pro Thr Tyr
    290                 295                 300
Gln Leu Ser Glu Thr Ser Tyr Gln Pro Thr Ser Ile Pro Gln Ala Val
305                 310                 315                 320
Ser Asp Pro Ser Ser Thr Val His Arg Pro Gln Pro Leu Pro Pro Ser
                325                 330                 335
Thr Val His Gln Ser Thr Ile Pro Ser Asn Pro Asp Ser Ser Ser Ala
            340                 345                 350
Tyr Cys Leu Pro Ser Thr Arg His Gly Phe Ser Ser Tyr Thr Asp Ser
        355                 360                 365
Phe Val Pro Pro Ser Gly Pro Ser Asn Pro Met Asn Pro Thr Ile Gly
    370                 375                 380
Asn Gly Leu Ser Pro Gln Asn Ser Ile Arg His Asn Leu Ser Leu His
385                 390                 395                 400
Ser Lys Phe Ile Arg Val Gln Asn Glu Gly Thr Gly Lys Ser Ser Trp
                405                 410                 415
Trp Met Leu Asn Pro Glu Gly Gly Lys Ser Gly Lys Ser Pro Arg Arg
            420                 425                 430
Arg Ala Ala Ser Met Asp Asn Asn Ser Lys Phe Ala Lys Ser Arg Ser
        435                 440                 445
Arg Ala Ala Lys Lys Lys Ala Ser Leu Gln Ser Gly Gln Glu Gly Ala
    450                 455                 460
Gly Asp Ser Pro Gly Ser Gln Phe Ser Lys Trp Pro Ala Ser Pro Gly
465                 470                 475                 480
Ser His Ser Asn Asp Asp Phe Asp Asn Trp Ser Thr Phe Arg Pro Arg
                485                 490                 495
Thr Ser Ser Asn Ala Ser Thr Ile Ser Gly Arg Leu Ser Pro Ile Met
            500                 505                 510
Thr Glu Gln Asp Asp Leu Gly Glu Gly Asp Val His Ser Met Val Tyr
        515                 520                 525
Pro Pro Ser Ala Ala Lys Met Ala Ser Thr Leu Pro Ser Leu Ser Glu
    530                 535                 540
Ile Ser Asn Pro Glu Asn Met Glu Asn Leu Leu Asp Asn Leu Asn Leu
545                 550                 555                 560
Leu Ser Ser Pro Thr Ser Leu Thr Val Ser Thr Gln Ser Ser Pro Gly
                565                 570                 575
Thr Met Met Gln Gln Thr Pro Cys Tyr Ser Phe Ala Pro Pro Asn Thr
            580                 585                 590
Ser Leu Asn Ser Pro Ser Pro Asn Tyr Gln Lys Tyr Thr Tyr Gly Gln
        595                 600                 605
Ser Ser Met Ser Pro Leu Pro Gln Met Pro Ile Gln Thr Leu Gln Asp
    610                 615                 620
Asn Lys Ser Ser Tyr Gly Gly Met Ser Gln Tyr Asn Cys Ala Pro Gly
625                 630                 635                 640
Leu Leu Lys Glu Leu Leu Thr Ser Asp Ser Pro Pro His Asn Asp Ile
                645                 650                 655
Met Thr Pro Val Asp Pro Gly Val Ala Gln Pro Asn Ser Arg Val Leu
            660                 665                 670
Gly Gln Asn Val Met Met Gly Pro Asn Ser Val Met Ser Thr Tyr Gly
        675                 680                 685
Ser Gln Ala Ser His Asn Lys Met Met Asn Pro Ser Ser His Thr His

690                 695                 700
Pro Gly His Ala Gln Gln Thr Ser Ala Val Asn Gly Arg Pro Leu Pro
705                 710                 715                 720
His Thr Val Ser Thr Met Pro His Thr Ser Gly Met Asn Arg Leu Thr
                725                 730                 735
Gln Val Lys Thr Pro Val Gln Val Pro Leu Pro His Pro Met Gln Met
            740                 745                 750
Ser Ala Leu Gly Gly Tyr Ser Ser Val Ser Ser Cys Asn Gly Tyr Gly
        755                 760                 765
Arg Met Gly Leu Leu His Gln Glu Lys Leu Pro Ser Asp Leu Asp Gly
    770                 775                 780
Met Phe Ile Glu Arg Leu Asp Cys Asp Met Glu Ser Ile Ile Arg Asn
785                 790                 795                 800
Asp Leu Met Asp Gly Asp Thr Leu Asp Phe Asn Phe Asp Asn Val Leu
                805                 810                 815
Pro Asn Gln Ser Phe Pro His Ser Val Lys Thr Thr Thr His Ser Trp
            820                 825                 830
Val Ser Gly
        835

<210> 336
<211> 1568
<212> PRT
<213> Homo sapiens

<400> 336
Met Glu Val Ser Arg Arg Lys Ala Pro Pro Arg Pro Pro Arg Pro Ala
1               5                   10                  15
Ala Pro Leu Pro Leu Leu Ala Tyr Leu Leu Ala Leu Ala Ala Pro Gly
            20                  25                  30
Arg Gly Ala Asp Glu Pro Val Trp Arg Ser Glu Gln Ala Ile Gly Ala
        35                  40                  45
Ile Ala Ala Ser Gln Glu Asp Gly Val Phe Val Ala Ser Gly Ser Cys
    50                  55                  60
Leu Asp Gln Leu Asp Tyr Ser Leu Glu His Ser Leu Ser Arg Leu Tyr
65                  70                  75                  80
Arg Asp Gln Ala Gly Asn Cys Thr Glu Pro Val Ser Leu Ala Pro Pro
                85                  90                  95
Ala Arg Pro Arg Pro Gly Ser Ser Phe Ser Lys Leu Leu Leu Pro Tyr
            100                 105                 110
Arg Glu Gly Ala Ala Gly Leu Gly Gly Leu Leu Leu Thr Gly Trp Thr
        115                 120                 125
Phe Asp Arg Gly Ala Cys Glu Val Arg Pro Leu Gly Asn Leu Ser Arg
    130                 135                 140
Asn Ser Leu Arg Asn Gly Thr Glu Val Val Ser Cys His Pro Gln Gly
145                 150                 155                 160
Ser Thr Ala Gly Val Val Tyr Arg Ala Gly Arg Asn Asn Arg Trp Tyr
                165                 170                 175
Leu Ala Val Ala Ala Thr Tyr Val Leu Pro Glu Pro Glu Thr Ala Ser
            180                 185                 190
Arg Cys Asn Pro Ala Ala Ser Asp His Asp Thr Ala Ile Ala Leu Lys
        195                 200                 205
Asp Thr Glu Gly Arg Ser Leu Ala Thr Gln Glu Leu Gly Arg Leu Lys
    210                 215                 220
Leu Cys Glu Gly Ala Gly Ser Leu His Phe Val Asp Ala Phe Leu Trp
225                 230                 235                 240
Asn Gly Ser Ile Tyr Phe Pro Tyr Tyr Pro Tyr Asn Tyr Thr Ser Gly
                245                 250                 255
Ala Ala Thr Gly Trp Pro Ser Met Ala Arg Ile Ala Gln Ser Thr Glu
            260                 265                 270
Val Leu Phe Gln Gly Gln Ala Ser Leu Asp Cys Gly His Gly His Pro
        275                 280                 285
Asp Gly Arg Arg Leu Leu Leu Ser Ser Ser Leu Val Glu Ala Leu Asp

290                 295                 300
Val Trp Ala Gly Val Phe Ser Ala Ala Ala Gly Glu Gly Gln Glu Arg
305                 310                 315                 320
Arg Ser Pro Thr Thr Thr Ala Leu Cys Leu Phe Arg Met Ser Glu Ile
                325                 330                 335
Gln Ala Arg Ala Lys Arg Val Ser Trp Asp Phe Lys Thr Ala Glu Ser
            340                 345                 350
His Cys Lys Glu Gly Asp Gln Pro Glu Arg Val Gln Pro Ile Ala Ser
        355                 360                 365
Ser Thr Leu Ile His Ser Asp Leu Thr Ser Val Tyr Gly Thr Val Val
    370                 375                 380
Met Asn Arg Thr Val Leu Phe Leu Gly Thr Gly Asp Gly Gln Leu Leu
385                 390                 395                 400
Lys Val Ile Leu Gly Glu Asn Leu Thr Ser Asn Cys Pro Glu Val Ile
                405                 410                 415
Tyr Glu Ile Lys Glu Glu Thr Pro Val Phe Tyr Lys Leu Val Pro Asp
            420                 425                 430

Pro Val Lys Asn Ile Tyr Ile Tyr Leu Thr Ala Gly Lys Glu Val Arg
        435                 440                 445
Arg Ile Arg Val Ala Asn Cys Asn Lys His Lys Ser Cys Ser Glu Cys
    450                 455                 460
Leu Thr Ala Thr Asp Pro His Cys Gly Trp Cys His Ser Leu Gln Arg
465                 470                 475                 480
Cys Thr Phe Gln Gly Asp Cys Val His Ser Glu Asn Leu Glu Asn Trp
                485                 490                 495
Leu Asp Ile Ser Ser Gly Ala Lys Lys Cys Pro Lys Ile Gln Ile Ile
            500                 505                 510
Arg Ser Ser Lys Glu Lys Thr Thr Val Thr Met Val Gly Ser Phe Ser
        515                 520                 525
Pro Arg His Ser Lys Cys Met Val Lys Asn Val Asp Ser Ser Arg Glu
    530                 535                 540
Leu Cys Gln Asn Lys Ser Gln Pro Asn Arg Thr Cys Thr Cys Ser Ile
545                 550                 555                 560
Pro Thr Arg Ala Thr Tyr Lys Asp Val Ser Val Val Asn Val Met Phe
                565                 570                 575
Ser Phe Gly Ser Trp Asn Leu Ser Asp Arg Phe Asn Phe Thr Asn Cys
            580                 585                 590
Ser Ser Leu Lys Glu Cys Pro Ala Cys Val Glu Thr Gly Cys Ala Trp
        595                 600                 605
Cys Lys Ser Ala Arg Arg Cys Ile His Pro Phe Thr Ala Cys Asp Pro
    610                 615                 620
Ser Asp Tyr Glu Arg Asn Gln Glu Gln Cys Pro Val Ala Val Glu Lys
625                 630                 635                 640
Thr Ser Gly Gly Gly Arg Pro Lys Glu Asn Lys Gly Asn Arg Thr Asn
                645                 650                 655
Gln Ala Leu Gln Val Phe Tyr Ile Lys Ser Ile Glu Pro Gln Lys Val
            660                 665                 670
Ser Thr Leu Gly Lys Ser Asn Val Ile Val Thr Gly Ala Asn Phe Thr
        675                 680                 685
Arg Ala Ser Asn Ile Thr Met Ile Leu Lys Gly Thr Ser Thr Cys Asp
    690                 695                 700
Lys Asp Val Ile Gln Val Ser His Val Leu Asn Asp Thr His Met Lys
705                 710                 715                 720
Phe Ser Leu Pro Ser Ser Arg Lys Glu Met Lys Asp Val Cys Ile Gln
                725                 730                 735
Phe Asp Gly Gly Asn Cys Ser Ser Val Gly Ser Leu Ser Tyr Ile Ala
            740                 745                 750
Leu Pro His Cys Ser Leu Ile Phe Pro Ala Thr Thr Trp Ile Ser Gly
        755                 760                 765
Gly Gln Asn Ile Thr Met Met Gly Arg Asn Phe Asp Val Ile Asp Asn
    770                 775                 780
Leu Ile Ile Ser His Glu Leu Lys Gly Asn Ile Asn Val Ser Glu Tyr

785                 790                 795                 800
Cys Val Ala Thr Tyr Cys Gly Phe Leu Ala Pro Ser Leu Lys Ser Ser
                805                 810                 815
Lys Val Arg Thr Asn Val Thr Val Lys Leu Arg Val Gln Asp Thr Tyr
            820                 825                 830
Leu Asp Cys Gly Thr Leu Gln Tyr Arg Glu Asp Pro Arg Phe Thr Gly
        835                 840                 845
Tyr Arg Val Glu Ser Glu Val Asp Thr Glu Leu Glu Val Lys Ile Gln
    850                 855                 860
Lys Glu Asn Asp Asn Phe Asn Ile Ser Lys Lys Asp Ile Glu Ile Thr
865                 870                 875                 880
Leu Phe His Gly Glu Asn Gly Gln Leu Asn Cys Ser Phe Glu Asn Ile
                885                 890                 895
Thr Arg Asn Gln Asp Leu Thr Thr Ile Leu Cys Lys Ile Lys Gly Ile
            900                 905                 910

Lys Thr Ala Ser Thr Ile Ala Asn Ser Ser Lys Lys Val Arg Val Lys
        915                 920                 925
Leu Gly Asn Leu Glu Leu Tyr Val Glu Gln Glu Ser Val Pro Ser Thr
    930                 935                 940
Trp Tyr Phe Leu Ile Val Leu Pro Val Leu Leu Val Ile Val Ile Phe
945                 950                 955                 960
Ala Ala Val Gly Val Thr Arg His Lys Ser Lys Glu Leu Ser Arg Lys
                965                 970                 975
Gln Ser Gln Gln Leu Glu Leu Leu Glu Ser Glu Leu Arg Lys Glu Ile
            980                 985                 990
Arg Asp Gly Phe Ala Glu Leu Gln Met Asp Lys Leu Asp Val Val Asp
        995                 1000                1005
Ser Phe Gly Thr Val Pro Phe Leu Asp Tyr Lys His Phe Ala Leu Arg
    1010                1015                1020
Thr Phe Phe Pro Glu Ser Gly Gly Phe Thr His Ile Phe Thr Glu Asp
1025                1030                1035                1040
Met His Asn Arg Asp Ala Asn Asp Lys Asn Glu Ser Leu Thr Ala Leu
                1045                1050                1055
Asp Ala Leu Ile Cys Asn Lys Ser Phe Leu Val Thr Val Ile His Thr
            1060                1065                1070
Leu Glu Lys Gln Lys Asn Phe Ser Val Lys Asp Arg Cys Leu Phe Ala
        1075                1080                1085
Ser Phe Leu Thr Ile Ala Leu Gln Thr Lys Leu Val Tyr Leu Thr Ser
    1090                1095                1100
Ile Leu Glu Val Leu Thr Arg Asp Leu Met Glu Gln Cys Ser Asn Met
1105                1110                1115                1120
Gln Pro Lys Leu Met Leu Arg Arg Thr Glu Ser Val Val Glu Lys Leu
                1125                1130                1135
Leu Thr Asn Trp Met Ser Val Cys Leu Ser Gly Phe Leu Arg Glu Thr
            1140                1145                1150
Val Gly Glu Pro Phe Tyr Leu Leu Val Thr Thr Leu Asn Gln Lys Ile
        1155                1160                1165
Asn Lys Gly Pro Val Asp Val Ile Thr Cys Lys Ala Leu Tyr Thr Leu
    1170                1175                1180
Asn Glu Asp Trp Leu Leu Trp Gln Val Pro Glu Phe Ser Thr Val Ala
1185                1190                1195                1200
Leu Asn Val Val Phe Glu Lys Ile Pro Glu Asn Glu Ser Ala Asp Val
                1205                1210                1215
Cys Arg Asn Ile Ser Val Asn Val Leu Asp Cys Asp Thr Ile Gly Gln
            1220                1225                1230
Ala Lys Glu Lys Ile Phe Gln Ala Phe Leu Ser Lys Asn Gly Ser Pro
        1235                1240                1245
Tyr Gly Leu Gln Leu Asn Glu Ile Gly Leu Glu Leu Gln Met Gly Thr
    1250                1255                1260
Arg Gln Lys Glu Leu Leu Asp Ile Asp Ser Ser Ser Val Ile Leu Glu
1265                1270                1275                1280
Asp Gly Ile Thr Lys Leu Asn Thr Ile Gly His Tyr Glu Ile Ser Asn

1285                1290                1295
Gly Ser Thr Ile Lys Val Phe Lys Lys Ile Ala Asn Phe Thr Ser Asp
        1300                1305                1310
Val Glu Tyr Ser Asp Asp His Cys His Leu Ile Leu Pro Asp Ser Glu
    1315                1320                1325
Ala Phe Gln Asp Val Gln Gly Lys Arg His Arg Gly Lys His Lys Phe
    1330                1335                1340
Lys Val Lys Glu Met Tyr Leu Thr Lys Leu Leu Ser Thr Lys Val Ala
1345                1350                1355                1360
Ile His Ser Val Leu Glu Lys Leu Phe Arg Ser Ile Trp Ser Leu Pro
                1365                1370                1375
Asn Ser Arg Ala Pro Phe Ala Ile Lys Tyr Phe Phe Asp Phe Leu Asp
        1380                1385                1390

Ala Gln Ala Glu Asn Lys Lys Ile Thr Asp Pro Asp Val Val His Ile
    1395                1400                1405
Trp Lys Thr Asn Ser Leu Pro Leu Arg Phe Trp Val Asn Ile Leu Lys
    1410                1415                1420
Asn Pro Gln Phe Val Phe Asp Ile Lys Lys Thr Pro His Ile Asp Gly
1425                1430                1435                1440
Cys Leu Ser Val Ile Ala Gln Ala Phe Met Asp Ala Phe Ser Leu Thr
                1445                1450                1455
Glu Gln Gln Leu Gly Lys Glu Ala Pro Thr Asn Lys Leu Leu Tyr Ala
        1460                1465                1470
Lys Asp Ile Pro Thr Tyr Lys Glu Glu Val Lys Ser Tyr Tyr Lys Ala
    1475                1480                1485
Ile Arg Asp Leu Pro Pro Leu Ser Ser Ser Glu Met Glu Glu Phe Leu
    1490                1495                1500
Thr Gln Glu Ser Lys Lys His Glu Asn Glu Phe Asn Glu Glu Val Ala
1505                1510                1515                1520
Leu Thr Glu Ile Tyr Lys Tyr Ile Val Lys Tyr Phe Asp Glu Ile Leu
                1525                1530                1535
Asn Lys Leu Glu Arg Glu Arg Gly Leu Glu Glu Ala Gln Lys Gln Leu
        1540                1545                1550
Leu His Val Lys Val Leu Phe Asp Glu Lys Lys Lys Cys Lys Trp Met
    1555                1560                1565

<210> 337
<211> 235
<212> PRT
<213> Homo sapiens

<400> 337

<210> 338
<211> 311
<212> PRT
<213> Homo sapiens

<400> 338
Met Glu Pro Ser His Ser Gln Pro Thr Met Asp Met Lys His Met Gln
1               5                   10                  15
Glu Arg Pro Ala Ala Ser Pro Leu Lys Cys Gln Asp Gly Arg Gly Ser
            20                  25                  30
Ala Gln Ser Pro Leu Ser Ala Ala Gly Pro Gly Ser Pro Ala Pro Leu
        35                  40                  45
Gly Ala Leu Thr Pro Ala Glu Pro Gly Leu Gly Ser Ala Pro Arg Ala
    50                  55                  60
Pro Arg Ala Arg Ala Pro Val Thr Gly Lys Arg Arg Ser Arg Arg Ser
65                  70                  75                  80
Ala Val Ala Leu Gly Ser Ala Pro Ala Gln Pro Gly Pro Arg Glu Arg
                85                  90                  95
Thr Gly Arg Ala Ser Pro Ala Leu Glu Asn Asn Ser Asp Leu Leu Ser
            100                 105                 110
Lys Ala Ser Ala Ile Ala Thr Gly Thr Pro Pro Cys Glu Asp Ser Thr
        115                 120                 125
Ile Ala Arg Phe Tyr Leu Pro Pro Pro Leu Thr Thr His Pro Glu Leu
    130                 135                 140
Val Gly Thr Ser Gln Glu Leu Ala Leu Thr Phe Trp His Leu Leu Ser
145                 150                 155                 160
Met Phe Gly Phe Phe Ile Val Ser Tyr Gly Phe Leu Thr Ala Phe Gly
                165                 170                 175
Arg Thr Leu Phe His Leu Asp Leu Leu Gln Pro Asn Leu Thr Pro Ser
            180                 185                 190
Arg Phe Asp Lys Tyr Thr Gly Gln Leu Leu Glu Leu Pro Val His Ser
        195                 200                 205
Lys Tyr Val Leu Thr Ile His Trp Ser Gln Gly Leu Cys Ile Trp Ser
    210                 215                 220
Ser His Pro Cys Glu Ala Gly Val Glu Asn Gly Thr Ser Gln Thr Arg
225                 230                 235                 240
Gly Gly Gly Gly Ala Ala Arg Ser Arg Cys Arg Glu Met Glu Glu Pro
                245                 250                 255
Thr Pro Glu Pro Val Tyr Val Asp Val Asp Lys Gly Leu Thr Leu Ala
            260                 265                 270
Cys Phe Val Phe Leu Cys Leu Phe Leu Val Val Met Ile Ile Arg Cys
        275                 280                 285
Ala Lys Val Ile Met Asp Pro Tyr Ser Ala Ile Pro Thr Ser Thr Trp
    290                 295                 300
Glu Glu Gln His Leu Asp Asp
305                 310

<210> 339
<211> 698
<212> PRT
<213> Homo sapiens

<400> 339
Met Gly Lys Asp Phe Met Ser Lys Thr Pro Lys Ala Met Ala Thr Gln
1               5                   10                  15
Ala Lys Ile Asp Lys Trp Asp Leu Ile Lys Leu Lys Ser Phe Cys Thr
            20                  25                  30
Ala Lys Glu Thr Thr Ile Arg Val Asn Arg Gln Pro Thr Glu Trp Glu
        35                  40                  45
Lys Ile Phe Ala Ile Tyr Ser Ser Asp Glu Gly Leu Ile Ser Arg Ile
    50                  55                  60
Tyr Asn Glu Pro Lys Gln Ile Tyr Lys Lys Lys Thr Asn Asn Pro Ile
65                  70                  75                  80
Asn Lys Trp Ala Lys Asp Met Asn Arg His Phe Ser Lys Glu Asp Ile
                85                  90                  95
Tyr Ala Ala Asn Arg His Met Lys Lys Cys Ser Ser Ser Leu Ala Ile
            100                 105                 110
Arg Glu Met Gln Ile Lys Thr Thr Thr Arg Cys His Leu Thr Pro Val
        115                 120                 125
Arg Met Ala Ile Ile Lys Lys Ser Gly Asn Asn Arg Thr Ala Glu Glu
    130                 135                 140
Gln Pro Lys Leu Arg Lys Glu Ala Val Gly Ser Ile Glu Ile Phe Arg
145                 150                 155                 160
Phe Ala Asp Gly Leu Asp Ile Thr Leu Met Ile Leu Gly Ile Leu Ala
                165                 170                 175
Ser Leu Val Asn Gly Ala Cys Leu Pro Leu Met Pro Leu Val Leu Gly
            180                 185                 190
Glu Met Ser Asp Asn Leu Ile Ser Gly Cys Leu Val Gln Thr Asn Thr
        195                 200                 205
Thr Asn Tyr Gln Asn Cys Thr Gln Ser Gln Glu Lys Leu Asn Glu Asp
    210                 215                 220
Met Thr Leu Leu Thr Leu Tyr Tyr Val Gly Ile Gly Val Ala Ala Leu
225                 230                 235                 240
Ile Phe Gly Tyr Ile Gln Ile Ser Leu Trp Ile Ile Thr Ala Ala Arg
                245                 250                 255
Gln Thr Lys Arg Ile Arg Lys Gln Phe Phe His Ser Val Leu Ala Gln
            260                 265                 270
Asp Ile Gly Trp Phe Asp Ser Cys Asp Ile Gly Glu Leu Asn Thr Arg
        275                 280                 285
Met Thr Asp Asp Ile Asp Lys Ile Ser Asp Gly Ile Gly Asp Lys Ile
    290                 295                 300
Ala Leu Leu Phe Gln Asn Met Ser Thr Phe Ser Ile Gly Leu Ala Val
305                 310                 315                 320
Gly Leu Val Lys Gly Trp Lys Leu Thr Leu Val Thr Leu Ser Thr Ser
                325                 330                 335
Pro Leu Ile Met Ala Ser Ala Ala Ala Cys Ser Arg Met Val Ile Ser
            340                 345                 350
Leu Thr Ser Lys Glu Leu Ser Ala Tyr Ser Lys Ala Gly Ala Val Ala
        355                 360                 365
Glu Glu Val Leu Ser Ser Ile Arg Thr Val Ile Ala Phe Arg Ala Gln
    370                 375                 380
Glu Lys Glu Leu Gln Arg Tyr Thr Gln Asn Leu Lys Asp Ala Lys Asp
385                 390                 395                 400
Phe Gly Ile Lys Arg Thr Ile Ala Ser Lys Val Ser Leu Gly Ala Val
                405                 410                 415
Tyr Phe Phe Met Asn Gly Thr Tyr Gly Leu Ala Phe Trp Tyr Gly Thr
            420                 425                 430
Ser Leu Ile Leu Asn Gly Glu Pro Gly Tyr Thr Ile Gly Thr Val Leu
        435                 440                 445
Ala Val Phe Phe Ser Val Ile His Ser Ser Tyr Cys Ile Gly Ala Ala
    450                 455                 460
Val Pro His Phe Glu Thr Phe Ala Ile Ala Arg Gly Ala Ala Phe His
465                 470                 475                 480
Ile Phe Gln Val Ile Asp Lys Lys Pro Ser Ile Asp Asn Phe Ser Thr

                485                 490                 495
Ala Gly Tyr Lys Pro Glu Ser Ile Glu Gly Thr Val Glu Phe Lys Asn
            500                 505                 510
Val Ser Phe Asn Tyr Pro Ser Arg Pro Ser Ile Lys Ile Leu Lys Gly
        515                 520                 525
Leu Asn Leu Arg Ile Lys Ser Gly Glu Thr Val Ala Leu Val Gly Leu
    530                 535                 540

Asn Gly Ser Gly Lys Ser Thr Val Val Gln Leu Leu Gln Arg Leu Tyr
545                 550                 555                 560
Asp Pro Asp Asp Gly Phe Ile Met Val Asp Glu Asn Asp Ile Arg Ala
                565                 570                 575
Leu Asn Val Arg His Tyr Arg Asp His Ile Gly Val Val Ser Gln Glu
            580                 585                 590
Pro Val Leu Phe Gly Thr Thr Ile Ser Asn Asn Ile Lys Tyr Gly Arg
        595                 600                 605
Asp Asp Val Thr Asp Glu Glu Met Glu Arg Ala Ala Arg Glu Ala Asn
    610                 615                 620
Ala Tyr Asp Phe Ile Met Glu Phe Pro Asn Lys Phe Asn Thr Leu Val
625                 630                 635                 640
Gly Glu Lys Gly Ala Gln Met Ser Gly Gly Gln Lys Gln Arg Ile Ala
                645                 650                 655
Ile Ala Arg Ala Leu Val Arg Asn Pro Lys Ile Leu Ile Leu Asp Glu
            660                 665                 670
Ala Thr Ser Ala Leu Asp Ser Glu Ser Lys Ser Ala Val Gln Ala Ala
        675                 680                 685
Leu Glu Lys Asp Thr Pro Arg Tyr Ser Phe
    690                 695

<210> 340
<211> 131
<212> PRT
<213> Homo sapiens

<400> 340
Met Val Asp Glu Asn Asp Ile Arg Ala Leu Asn Val Arg His Tyr Arg
1               5                   10                  15
Asp His Ile Gly Val Val Ser Gln Glu Pro Val Leu Phe Gly Thr Thr
            20                  25                  30
Ile Ser Asn Asn Ile Lys Tyr Gly Arg Asp Asp Val Thr Asp Glu Glu
        35                  40                  45
Met Glu Arg Ala Ala Arg Glu Ala Asn Ala Tyr Asp Phe Ile Met Glu
    50                  55                  60
Phe Pro Asn Lys Phe Asn Thr Leu Val Gly Glu Lys Gly Ala Gln Met
65                  70                  75                  80
Ser Gly Gly Gln Lys Gln Arg Ile Ala Ile Ala Arg Ala Leu Val Arg
                85                  90                  95
Asn Pro Lys Ile Leu Ile Leu Asp Glu Ala Thr Ser Ala Leu Asp Ser
            100                 105                 110
Glu Ser Lys Ser Ala Val Gln Ala Ala Leu Glu Lys Asp Thr Pro Arg
        115                 120                 125
Tyr Ser Phe
    130

<210> 341
<211> 315
<212> PRT
<213> Homo sapiens

<400> 341
Met Glu Lys Phe Arg Ala Val Leu Asp Leu His Val Lys His His Ser
1               5                   10                  15
Ala Leu Gly Tyr Gly Leu Val Thr Leu Leu Thr Ala Gly Gly Glu Arg

                20                  25                  30
Ile Phe Ser Ala Val Ala Phe Gln Cys Pro Cys Ser Ala Ala Trp Asn
        35                  40                  45
Leu Pro Tyr Gly Leu Val Phe Leu Leu Val Pro Ala Leu Ala Leu Phe
    50                  55                  60

Leu Leu Gly Tyr Val Leu Ser Ala Arg Thr Trp Arg Leu Leu Thr Gly
65                  70                  75                  80
Cys Cys Ser Ser Ala Arg Ala Ser Cys Gly Ser Ala Leu Arg Gly Ser
                85                  90                  95
Leu Val Cys Thr Gln Ile Ser Ala Ala Ala Ala Leu Ala Pro Leu Thr
            100                 105                 110
Trp Val Ala Val Ala Leu Leu Gly Gly Ala Phe Tyr Glu Cys Ala Ala
        115                 120                 125
Thr Gly Ser Ala Ala Phe Ala Gln Arg Leu Cys Leu Gly Arg Asn Arg
    130                 135                 140
Ser Cys Ala Ala Glu Leu Pro Leu Val Pro Cys Asn Gln Ala Lys Ala
145                 150                 155                 160
Ser Asp Val Gln Asp Leu Leu Lys Asp Leu Lys Ala Gln Ser Gln Val
                165                 170                 175
Leu Gly Trp Ile Leu Ile Ala Val Val Ile Ile Ile Leu Leu Ile Phe
            180                 185                 190
Thr Ser Val Thr Arg Cys Leu Ser Pro Val Ser Phe Leu Gln Leu Lys
        195                 200                 205
Phe Trp Lys Ile Tyr Leu Glu Gln Glu Gln Gln Ile Leu Lys Ser Lys
    210                 215                 220
Ala Thr Glu His Ala Thr Glu Leu Ala Lys Glu Asn Ile Lys Cys Phe
225                 230                 235                 240
Phe Glu Gly Ser His Pro Lys Glu Tyr Asn Thr Pro Ser Met Lys Glu
                245                 250                 255
Trp Gln Gln Ile Ser Ser Leu Tyr Thr Phe Asn Pro Lys Gly Gln Tyr
            260                 265                 270
Tyr Ser Met Leu His Lys Tyr Val Asn Arg Lys Glu Lys Thr His Ser
        275                 280                 285
Ile Arg Ser Thr Glu Gly Asp Thr Val Ile Pro Val Leu Gly Phe Val
    290                 295                 300
Asp Ser Ser Gly Ile Asn Ser Thr Pro Glu Leu
305                 310                 315

<210> 342
<211> 756
<212> PRT
<213> Homo sapiens

<400> 342
Met Ala Arg Ser Pro Pro Ala Thr Leu Phe Leu Phe Leu Leu Leu Leu
 1               5                  10                  15
Gly Gln Pro Pro Pro Ser Arg Pro Gln Ser Leu Gly Thr Thr Lys Leu
            20                  25                  30
Arg Leu Val Gly Pro Glu Ser Lys Pro Glu Glu Gly Arg Leu Glu Val
        35                  40                  45
Leu His Gln Gly Gln Trp Gly Thr Val Cys Asp Asp Asn Phe Ala Ile
    50                  55                  60
Gln Glu Ala Thr Val Ala Cys Arg Gln Leu Gly Phe Glu Ala Ala Leu
65                  70                  75                  80
Thr Trp Ala His Ser Ala Lys Tyr Gly Gln Gly Glu Gly Pro Ile Trp
                85                  90                  95
Leu Asp Asn Val Gln Cys Val Gly Thr Glu Ser Ser Leu Asp Gln Cys
            100                 105                 110
Gly Ser Asn Gly Trp Gly Val Ser Asp Cys Ser His Ser Glu Asp Val
        115                 120                 125
Gly Val Ile Cys His Pro Arg Arg His Arg Gly Tyr Leu Ser Glu Thr
    130                 135                 140

Val Ser Asn Ala Leu Gly Pro Gln Gly Arg Arg Leu Glu Glu Val Arg
145                 150                 155                 160
Leu Lys Pro Ile Leu Ala Ser Ala Lys Gln His Ser Pro Val Thr Glu
                165                 170                 175
Gly Ala Val Glu Val Lys Tyr Glu Gly His Trp Arg Gln Val Cys Asp
            180                 185                 190
Gln Gly Trp Thr Met Asn Asn Ser Arg Val Val Cys Gly Met Leu Gly
        195                 200                 205
Phe Pro Ser Glu Val Pro Val Asp Ser His Tyr Tyr Arg Lys Val Trp
    210                 215                 220
Asp Leu Lys Met Arg Asp Pro Lys Ser Arg Leu Lys Ser Leu Thr Asn
225                 230                 235                 240
Lys Asn Ser Phe Trp Ile His Gln Val Thr Cys Leu Gly Thr Glu Pro
                245                 250                 255
His Met Ala Asn Cys Gln Val Gln Val Ala Pro Ala Arg Gly Lys Leu
            260                 265                 270
Arg Pro Ala Cys Pro Gly Gly Met His Ala Val Val Ser Cys Val Ala
        275                 280                 285
Gly Pro His Phe Arg Pro Pro Lys Thr Lys Pro Gln Arg Lys Gly Ser
    290                 295                 300
Trp Ala Glu Glu Pro Arg Val Arg Leu Arg Ser Gly Ala Gln Val Gly
305                 310                 315                 320
Glu Gly Arg Val Glu Val Leu Met Asn Arg Gln Trp Gly Thr Val Cys
                325                 330                 335
Asp His Arg Trp Asn Leu Ile Ser Ala Ser Val Val Cys Arg Gln Leu
            340                 345                 350
Gly Phe Gly Ser Ala Arg Glu Ala Leu Phe Gly Ala Arg Leu Gly Gln
        355                 360                 365
Gly Leu Gly Pro Ile His Leu Ser Glu Val Arg Cys Arg Gly Tyr Glu
    370                 375                 380
Arg Thr Leu Ser Asp Cys Pro Ala Leu Glu Gly Ser Gln Asn Gly Cys
385                 390                 395                 400
Gln His Glu Asn Ala Ala Ala Val Arg Cys Asn Val Pro Asn Met Gly
                405                 410                 415
Phe Gln Asn Gln Val Arg Leu Ala Gly Gly Arg Ile Pro Glu Glu Gly
            420                 425                 430
Leu Leu Glu Val Gln Val Glu Val Asn Gly Val Pro Arg Trp Gly Ser
        435                 440                 445
Val Cys Ser Glu Asn Trp Gly Leu Thr Glu Ala Met Val Ala Cys Arg
    450                 455                 460
Gln Leu Gly Leu Gly Phe Ala Ile His Ala Tyr Lys Glu Thr Trp Phe
465                 470                 475                 480
Trp Ser Gly Thr Pro Arg Ala Gln Glu Val Val Met Ser Gly Val Arg
                485                 490                 495
Cys Ser Gly Thr Glu Leu Ala Leu Gln Gln Cys Gln Arg His Gly Pro
            500                 505                 510
Val His Cys Ser His Gly Gly Gly Arg Phe Leu Ala Gly Val Ser Cys
        515                 520                 525
Met Asp Ser Ala Pro Asp Leu Val Met Asn Ala Gln Leu Val Gln Glu
    530                 535                 540
Thr Ala Tyr Leu Glu Asp Arg Pro Leu Ser Gln Leu Tyr Cys Ala His
545                 550                 555                 560
Glu Glu Asn Cys Leu Ser Lys Ser Ala Asp His Met Asp Trp Pro Tyr
                565                 570                 575
Gly Tyr Arg Arg Leu Leu Arg Phe Ser Thr Gln Ile Tyr Asn Leu Gly
            580                 585                 590
Arg Thr Asp Phe Arg Pro Lys Thr Gly Arg Asp Ser Trp Val Trp His
        595                 600                 605
Gln Cys His Arg His Tyr His Ser Ile Glu Val Phe Thr His Tyr Asp
    610                 615                 620
Leu Leu Thr Leu Asn Gly Ser Lys Val Ala Glu Gly His Lys Ala Ser
625                 630                 635                 640
Phe Cys Leu Glu Asp Thr Asn Cys Pro Thr Gly Leu Gln Arg Arg Tyr

                645                 650                 655
Ala Cys Ala Asn Phe Gly Glu Gln Gly Val Thr Val Gly Cys Trp Asp
            660                 665                 670
Thr Tyr Arg His Asp Ile Asp Cys Gln Trp Val Asp Ile Thr Asp Val
        675                 680                 685
Gly Pro Gly Asn Tyr Ile Phe Gln Val Ile Val Asn Pro His Tyr Glu
    690                 695                 700
Val Ala Glu Ser Asp Phe Ser Asn Asn Met Leu Gln Cys Arg Cys Lys
705                 710                 715                 720
Tyr Asp Gly His Arg Val Trp Leu His Asn Cys His Thr Gly Asn Ser
                725                 730                 735
Tyr Pro Ala Asn Ala Glu Leu Ser Leu Glu Gln Glu Gln Arg Leu Arg
            740                 745                 750
Asn Asn Leu Ile
        755

<210> 343
<211> 298
<212> PRT
<213> Homo sapiens

<400> 343
Met Glu Asn Phe Gln Lys Val Glu Lys Ile Gly Glu Gly Thr Tyr Gly
1               5                   10                  15
Val Val Tyr Lys Ala Arg Asn Lys Leu Thr Gly Glu Val Val Ala Leu
            20                  25                  30
Lys Lys Ile Arg Leu Asp Thr Glu Thr Glu Gly Val Pro Ser Thr Ala
        35                  40                  45
Ile Arg Glu Ile Ser Leu Leu Lys Glu Leu Asn His Pro Asn Ile Val
    50                  55                  60
Lys Leu Leu Asp Val Ile His Thr Glu Asn Lys Leu Tyr Leu Val Phe
65                  70                  75                  80
Glu Phe Leu His Gln Asp Leu Lys Lys Phe Met Asp Ala Ser Ala Leu
                85                  90                  95
Thr Gly Ile Pro Leu Pro Leu Ile Lys Ser Tyr Leu Phe Gln Leu Leu
            100                 105                 110
Gln Gly Leu Ala Phe Cys His Ser His Arg Val Leu His Arg Asp Leu
        115                 120                 125
Lys Pro Gln Asn Leu Leu Ile Asn Thr Glu Gly Ala Ile Lys Leu Ala
    130                 135                 140
Asp Phe Gly Leu Ala Arg Ala Phe Gly Val Pro Val Arg Thr Tyr Thr
145                 150                 155                 160
His Glu Val Val Thr Leu Trp Tyr Arg Ala Pro Glu Ile Leu Leu Gly
                165                 170                 175
Cys Lys Tyr Tyr Ser Thr Ala Val Asp Ile Trp Ser Leu Gly Cys Ile
            180                 185                 190
Phe Ala Glu Met Val Thr Arg Arg Ala Leu Phe Pro Gly Asp Ser Glu
        195                 200                 205
Ile Asp Gln Leu Phe Arg Ile Phe Arg Thr Leu Gly Thr Pro Asp Glu
    210                 215                 220
Val Val Trp Pro Gly Val Thr Ser Met Pro Asp Tyr Lys Pro Ser Phe
225                 230                 235                 240
Pro Lys Trp Ala Arg Gln Asp Phe Ser Lys Val Val Pro Pro Leu Asp
                245                 250                 255
Glu Asp Gly Arg Ser Leu Leu Ser Gln Met Leu His Tyr Asp Pro Asn
            260                 265                 270
Lys Arg Ile Ser Ala Lys Ala Ala Leu Ala His Pro Phe Phe Gln Asp
        275                 280                 285
Val Thr Lys Pro Val Pro His Leu Arg Leu
    290                 295

<210> 344
<211> 264
<212> PRT
<213> Homo sapiens

<400> 344
Met Glu Asn Phe Gln Lys Val Glu Lys Ile Gly Glu Gly Thr Tyr Gly
1               5                   10                  15
Val Val Tyr Lys Ala Arg Asn Lys Leu Thr Gly Glu Val Val Ala Leu
            20                  25                  30
Lys Lys Ile Arg Leu Asp Thr Glu Thr Glu Gly Val Pro Ser Thr Ala
        35                  40                  45
Ile Arg Glu Ile Ser Leu Leu Lys Glu Leu Asn His Pro Asn Ile Val
    50                  55                  60
Lys Leu Leu Asp Val Ile His Thr Glu Asn Lys Leu Tyr Leu Val Phe
65                  70                  75                  80
Glu Phe Leu His Gln Asp Leu Lys Lys Phe Met Asp Ala Ser Ala Leu
                85                  90                  95
Thr Gly Ile Pro Leu Pro Leu Ile Lys Ser Tyr Leu Phe Gln Leu Leu
            100                 105                 110
Gln Gly Leu Ala Phe Cys His Ser His Arg Val Leu His Arg Asp Leu
        115                 120                 125
Lys Pro Gln Asn Leu Leu Ile Asn Thr Glu Gly Ala Ile Lys Leu Ala
    130                 135                 140
Asp Phe Gly Leu Ala Arg Ala Phe Gly Val Pro Val Arg Thr Tyr Thr
145                 150                 155                 160
His Glu Val Thr Arg Arg Ala Leu Phe Pro Gly Asp Ser Glu Ile Asp
                165                 170                 175
Gln Leu Phe Arg Ile Phe Arg Thr Leu Gly Thr Pro Asp Glu Val Val
            180                 185                 190
Trp Pro Gly Val Thr Ser Met Pro Asp Tyr Lys Pro Ser Phe Pro Lys
        195                 200                 205
Trp Ala Arg Gln Asp Phe Ser Lys Val Val Pro Pro Leu Asp Glu Asp
    210                 215                 220
Gly Arg Ser Leu Leu Ser Gln Met Leu His Tyr Asp Pro Tyr Lys Arg
225                 230                 235                 240
Ile Ser Ala Lys Ala Ala Leu Ala His Pro Phe Phe Gln Asp Val Thr
                245                 250                 255
Lys Pro Val Pro His Leu Arg Leu
            260

<210> 345
<211> 359
<212> PRT
<213> Homo sapiens

<400> 345
Met Lys Leu Gly Cys Val Leu Met Ala Trp Ala Leu Tyr Leu Ser Leu
1               5                   10                  15
Gly Val Leu Trp Val Ala Gln Met Leu Leu Ala Ala Ser Phe Glu Thr
            20                  25                  30
Leu Gln Cys Glu Gly Pro Val Cys Thr Glu Glu Ser Ser Cys His Thr
        35                  40                  45
Glu Asp Asp Leu Thr Asp Ala Arg Glu Ala Gly Phe Gln Val Lys Ala
    50                  55                  60
Tyr Thr Phe Ser Glu Pro Phe His Leu Ile Val Ser Tyr Asp Trp Leu
65                  70                  75                  80
Ile Leu Gln Gly Pro Ala Lys Pro Val Phe Glu Gly Asp Leu Leu Val
                85                  90                  95
Leu Arg Cys Gln Ala Trp Gln Asp Trp Pro Leu Thr Gln Val Thr Phe
            100                 105                 110
Tyr Arg Asp Gly Ser Ala Leu Gly Pro Pro Gly Pro Asn Arg Glu Phe
        115                 120                 125

Ser Ile Thr Val Val Gln Lys Ala Asp Ser Gly His Tyr His Cys Ser
    130                 135                 140
Gly Ile Phe Gln Ser Pro Gly Pro Gly Ile Pro Glu Thr Ala Ser Val
145                 150                 155                 160
Val Ala Ile Thr Val Gln Glu Leu Phe Pro Ala Pro Ile Leu Arg Ala
                165                 170                 175
Val Pro Ser Ala Glu Pro Gln Ala Gly Ser Pro Met Thr Leu Ser Cys
            180                 185                 190
Gln Thr Lys Leu Pro Leu Gln Arg Ser Ala Ala Arg Leu Leu Phe Ser
        195                 200                 205
Phe Tyr Lys Asp Gly Arg Ile Val Gln Ser Arg Gly Leu Ser Ser Glu
    210                 215                 220
Phe Gln Ile Pro Thr Ala Ser Glu Asp His Ser Gly Ser Tyr Trp Cys
225                 230                 235                 240
Glu Ala Ala Thr Glu Asp Asn Gln Val Trp Lys Gln Ser Pro Gln Leu
                245                 250                 255
Glu Ile Arg Val Gln Gly Ala Ser Ser Ser Ala Ala Pro Pro Thr Leu
            260                 265                 270
Asn Pro Ala Pro Gln Lys Ser Ala Ala Pro Gly Thr Ala Pro Glu Glu
        275                 280                 285
Ala Pro Gly Pro Leu Pro Pro Pro Pro Thr Pro Ser Ser Glu Asp Pro
    290                 295                 300
Gly Phe Ser Ser Pro Leu Gly Met Pro Asp Pro His Leu Tyr His Gln
305                 310                 315                 320
Met Gly Leu Leu Leu Lys His Met Gln Asp Val Arg Val Leu Leu Gly
                325                 330                 335
His Leu Leu Met Glu Leu Arg Glu Leu Ser Gly His Gln Lys Pro Gly
            340                 345                 350
Thr Thr Lys Ala Thr Ala Glu
        355

<210> 346
<211> 131
<212> PRT
<213> Homo sapiens

<400> 346
Met Lys Ile Phe Leu Pro Val Leu Leu Ala Ala Leu Leu Gly Val Glu
1               5                   10                  15
Arg Ala Ser Ser Leu Met Cys Phe Ser Cys Leu Asn Gln Lys Ser Asn
            20                  25                  30
Leu Tyr Cys Leu Lys Pro Thr Ile Cys Ser Asp Gln Asp Asn Tyr Cys
        35                  40                  45
Val Thr Val Ser Ala Ser Ala Gly Ile Gly Asn Leu Val Thr Phe Gly
    50                  55                  60
His Ser Leu Ser Lys Thr Cys Ser Pro Ala Cys Pro Ile Pro Glu Gly
65                  70                  75                  80
Val Asn Val Gly Val Ala Ser Met Gly Ile Ser Cys Cys Gln Ser Phe
                85                  90                  95
Leu Cys Asn Phe Ser Ala Ala Asp Gly Gly Leu Arg Ala Ser Val Thr
            100                 105                 110
Leu Leu Gly Ala Gly Leu Leu Leu Ser Leu Leu Pro Ala Leu Leu Arg
        115                 120                 125
Phe Gly Pro
    130

<210> 347
<211> 718
<212> PRT
<213> Homo sapiens

<400> 347

Met Glu Asp Leu Gly Glu Asn Thr Met Val Leu Ser Thr Leu Arg Ser
1               5                   10                  15
Leu Asn Asn Phe Ile Ser Gln Arg Val Glu Gly Gly Ser Gly Leu Asp
            20                  25                  30
Ile Ser Thr Ser Ala Pro Gly Ser Leu Gln Met Gln Tyr Gln Gln Ser
        35                  40                  45
Met Gln Leu Glu Glu Arg Ala Glu Gln Ile Arg Ser Lys Ser His Leu
    50                  55                  60
Ile Gln Val Glu Arg Glu Lys Met Gln Met Glu Leu Ser His Lys Arg
65                  70                  75                  80
Ala Arg Val Glu Leu Glu Arg Ala Ala Ser Thr Ser Ala Arg Asn Tyr
                85                  90                  95
Glu Arg Glu Val Asp Arg Asn Gln Glu Leu Leu Thr Arg Ile Arg Gln
            100                 105                 110
Leu Gln Glu Arg Glu Ala Gly Ala Glu Glu Lys Met Gln Glu Gln Leu
        115                 120                 125
Glu Arg Asn Arg Gln Cys Gln Gln Asn Leu Asp Ala Ala Ser Lys Arg
    130                 135                 140
Leu Arg Glu Lys Glu Asp Ser Leu Ala Gln Ala Gly Glu Thr Ile Asn
145                 150                 155                 160
Ala Leu Lys Gly Arg Ile Ser Glu Leu Gln Trp Ser Val Met Asp Gln
                165                 170                 175
Glu Met Arg Val Lys Arg Leu Glu Ser Glu Lys Gln Glu Leu Gln Glu
            180                 185                 190
Gln Leu Asp Leu Gln His Lys Lys Cys Gln Glu Ala Asn Gln Lys Ile
        195                 200                 205
Gln Glu Leu Gln Ala Ser Gln Glu Ala Arg Ala Asp His Glu Gln Gln
    210                 215                 220
Ile Lys Asp Leu Glu Gln Lys Leu Ser Leu Gln Glu Gln Asp Ala Ala
225                 230                 235                 240
Ile Val Lys Asn Met Lys Ser Glu Leu Val Arg Leu Pro Arg Leu Glu
                245                 250                 255
Arg Glu Leu Lys Gln Leu Arg Glu Glu Ser Ala His Leu Arg Glu Met
            260                 265                 270
Arg Glu Thr Asn Gly Leu Leu Gln Glu Glu Leu Glu Gly Leu Gln Arg
        275                 280                 285
Lys Leu Gly Arg Gln Glu Lys Met Gln Glu Thr Leu Val Gly Leu Glu
    290                 295                 300
Leu Glu Asn Glu Arg Leu Leu Ala Lys Leu Gln Ser Trp Glu Arg Leu
305                 310                 315                 320
Asp Gln Thr Met Gly Leu Ser Ile Arg Thr Pro Glu Asp Leu Ser Arg
                325                 330                 335
Phe Val Val Glu Leu Gln Gln Arg Glu Leu Ala Leu Lys Asp Lys Asn
            340                 345                 350
Ser Ala Val Thr Ser Ser Ala Arg Gly Leu Glu Lys Ala Arg Gln Gln
        355                 360                 365
Leu Gln Glu Glu Leu Arg Gln Val Ser Gly Gln Leu Leu Glu Glu Arg
    370                 375                 380
Lys Lys Arg Glu Thr His Glu Ala Leu Ala Arg Arg Leu Gln Lys Arg
385                 390                 395                 400
Val Leu Leu Leu Thr Lys Glu Arg Asp Gly Met Arg Ala Ile Leu Gly
                405                 410                 415
Ser Tyr Asp Ser Glu Leu Thr Pro Ala Glu Tyr Ser Pro Gln Leu Thr
            420                 425                 430

Arg Arg Met Arg Glu Ala Glu Asp Met Val Gln Lys Val His Ser His
        435                 440                 445
Ser Ala Glu Met Glu Ala Gln Leu Ser Gln Ala Leu Glu Glu Leu Gly
    450                 455                 460
Gly Gln Lys Gln Arg Ala Asp Met Leu Glu Met Glu Leu Lys Met Leu
465                 470                 475                 480
Lys Ser Gln Ser Ser Ser Ala Glu Gln Ser Phe Leu Phe Ser Arg Glu
                485                 490                 495

Glu Ala Asp Thr Leu Arg Leu Lys Val Glu Glu Leu Glu Gly Glu Arg
            500                 505                 510
Ser Arg Leu Glu Glu Glu Lys Arg Met Leu Glu Ala Gln Leu Glu Arg
        515                 520                 525
Arg Ala Leu Gln Gly Asp Tyr Asp Gln Ser Arg Thr Lys Val Leu His
    530                 535                 540
Met Ser Leu Asn Pro Thr Ser Val Ala Arg Gln Arg Leu Arg Glu Asp
545                 550                 555                 560
His Ser Gln Leu Gln Ala Glu Cys Glu Arg Leu Arg Gly Leu Leu Arg
                565                 570                 575
Ala Met Glu Arg Gly Gly Thr Val Pro Ala Asp Leu Glu Ala Ala Ala
            580                 585                 590
Ala Ser Leu Pro Ser Ser Lys Glu Val Ala Glu Leu Lys Lys Gln Val
        595                 600                 605
Glu Ser Ala Glu Leu Lys Asn Gln Arg Leu Lys Glu Val Phe Gln Thr
    610                 615                 620
Lys Ile Gln Glu Phe Arg Lys Ala Cys Tyr Thr Leu Thr Gly Tyr Gln
625                 630                 635                 640
Ile Asp Ile Thr Thr Glu Asn Gln Tyr Arg Leu Thr Ser Leu Tyr Ala
                645                 650                 655
Glu His Pro Gly Asp Cys Leu Ile Phe Lys Ala Thr Ser Pro Ser Gly
            660                 665                 670
Ser Lys Met Gln Leu Leu Glu Thr Glu Phe Ser His Thr Val Gly Glu
        675                 680                 685
Leu Ile Glu Val His Leu Arg Arg Gln Asp Ser Ile Pro Ala Phe Leu
    690                 695                 700
Ser Ser Leu Thr Leu Glu Leu Phe Ser Arg Gln Thr Val Ala
705                 710                 715

<210> 348
<211> 424
<212> PRT
<213> Homo sapiens

<400> 348
Met Thr Gln Ala Gly Arg Arg Gly Pro Gly Thr Pro Glu Pro Arg Pro
1               5                   10                  15
Arg Thr Gln Pro Met Ala Ser Pro Arg Leu Gly Thr Phe Cys Cys Pro
            20                  25                  30
Thr Arg Asp Ala Ala Thr Gln Leu Val Leu Ser Phe Gln Pro Arg Ala
        35                  40                  45
Phe His Ala Leu Cys Leu Gly Ser Gly Gly Leu Arg Leu Ala Leu Gly
    50                  55                  60
Leu Leu Gln Leu Leu Pro Gly Arg Arg Pro Ala Gly Pro Gly Ser Pro
65                  70                  75                  80
Ala Thr Ser Pro Pro Ala Ser Val Arg Ile Leu Arg Ala Ala Ala Ala
                85                  90                  95
Cys Asp Leu Leu Gly Cys Leu Gly Met Val Ile Arg Ser Thr Val Trp
            100                 105                 110
Leu Gly Phe Pro Asn Phe Val Asp Ser Val Ser Asp Met Asn His Thr
        115                 120                 125
Glu Ile Trp Pro Ala Ala Phe Cys Val Gly Ser Ala Met Trp Ile Gln
    130                 135                 140
Leu Leu Tyr Ser Ala Cys Phe Trp Trp Leu Phe Cys Tyr Ala Val Asp
145                 150                 155                 160
Ala Tyr Leu Val Ile Arg Arg Ser Ala Gly Leu Ser Thr Ile Leu Leu
                165                 170                 175
Tyr His Ile Met Ala Trp Gly Leu Ala Thr Leu Leu Cys Val Glu Gly
            180                 185                 190
Ala Ala Met Leu Tyr Tyr Pro Ser Val Ser Arg Cys Glu Arg Gly Leu
        195                 200                 205
Asp His Ala Ile Pro His Tyr Val Thr Met Tyr Leu Pro Leu Leu Leu
    210                 215                 220

Val Leu Val Ala Asn Pro Ile Leu Phe Gln Lys Thr Val Thr Ala Val
225                 230                 235                 240
Ala Ser Leu Leu Lys Gly Arg Gln Gly Ile Tyr Thr Glu Asn Glu Arg
                245                 250                 255
Arg Met Gly Ala Val Ile Lys Ile Arg Phe Phe Lys Ile Met Leu Val
            260                 265                 270
Leu Ile Ile Cys Trp Leu Ser Asn Ile Ile Asn Glu Ser Leu Leu Phe
        275                 280                 285
Tyr Leu Glu Met Gln Thr Asp Ile Asn Gly Gly Ser Leu Lys Pro Val
    290                 295                 300
Arg Thr Ala Ala Lys Thr Thr Trp Phe Ile Met Gly Ile Leu Asn Pro
305                 310                 315                 320
Ala Gln Gly Phe Leu Leu Ser Leu Ala Phe Tyr Gly Trp Thr Gly Cys
                325                 330                 335
Ser Leu Gly Phe Gln Ser Pro Arg Lys Glu Ile Gln Trp Glu Ser Leu
            340                 345                 350
Thr Thr Ser Ala Ala Glu Gly Ala His Pro Ser Pro Leu Met Pro His
        355                 360                 365
Glu Asn Pro Ala Ser Gly Lys Val Ser Gln Val Gly Gly Gln Thr Ser
    370                 375                 380
Asp Glu Ala Leu Ser Met Leu Ser Glu Gly Ser Asp Ala Ser Thr Ile
385                 390                 395                 400
Glu Ile His Thr Ala Ser Glu Ser Cys Asn Lys Asn Glu Gly Asp Pro
                405                 410                 415
Ala Leu Pro Thr His Gly Asp Leu
            420

<210> 349
<211> 298
<212> PRT
<213> Homo sapiens

<400> 349
Met Gly Pro Lys Asp Ser Ala Lys Cys Leu His Arg Gly Pro Gln Pro
1               5                   10                  15
Ser His Trp Ala Ala Gly Asp Gly Pro Thr Gln Glu Arg Cys Gly Pro
            20                  25                  30
Arg Ser Leu Gly Ser Pro Val Leu Gly Leu Asp Thr Cys Arg Ala Trp
        35                  40                  45
Asp His Val Asp Gly Gln Ile Leu Gly Gln Leu Arg Pro Leu Thr Glu
    50                  55                  60
Glu Glu Glu Glu Glu Gly Ala Gly Ala Thr Leu Ser Arg Gly Pro Ala
65                  70                  75                  80
Phe Pro Gly Met Gly Ser Glu Glu Leu Arg Leu Ala Ser Phe Tyr Asp
                85                  90                  95
Trp Pro Leu Thr Ala Glu Val Pro Pro Glu Leu Leu Ala Ala Ala Gly
            100                 105                 110
Phe Phe His Thr Gly His Gln Asp Lys Val Arg Cys Phe Phe Cys Tyr
        115                 120                 125
Gly Gly Leu Gln Ser Trp Lys Arg Gly Asp Asp Pro Trp Thr Glu His
    130                 135                 140
Ala Lys Trp Phe Pro Ser Cys Gln Phe Leu Leu Arg Ser Lys Gly Arg
145                 150                 155                 160
Asp Phe Val His Ser Val Gln Glu Thr His Ser Gln Leu Leu Gly Ser
                165                 170                 175
Trp Asp Pro Trp Glu Glu Pro Glu Asp Ala Ala Pro Val Ala Pro Ser
            180                 185                 190
Val Pro Ala Ser Gly Tyr Pro Glu Leu Pro Thr Pro Arg Arg Glu Val
        195                 200                 205
Gln Ser Glu Ser Ala Gln Glu Pro Gly Gly Val Ser Pro Ala Glu Ala
    210                 215                 220
Gln Arg Ala Trp Trp Val Leu Glu Pro Pro Gly Ala Arg Asp Val Glu
225                 230                 235                 240

Ala Gln Leu Arg Arg Leu Gln Glu Glu Arg Thr Cys Lys Val Cys Leu
                245                 250                 255
Asp Arg Ala Val Ser Ile Val Phe Val Pro Cys Gly His Leu Val Cys
            260                 265                 270
Ala Glu Cys Ala Pro Gly Leu Gln Leu Cys Pro Ile Cys Arg Ala Pro
        275                 280                 285
Val Arg Ser Arg Val Arg Thr Phe Leu Ser
    290                 295

<210> 350
<211> 280
<212> PRT
<213> Homo sapiens

<400> 350
Met Gly Pro Lys Asp Ser Ala Lys Cys Leu His Arg Gly Pro Gln Pro
1               5                   10                  15
Ser His Trp Ala Ala Gly Asp Gly Pro Thr Gln Glu Arg Cys Gly Pro
            20                  25                  30
Arg Ser Leu Gly Ser Pro Val Leu Gly Leu Asp Thr Cys Arg Ala Trp
        35                  40                  45
Asp His Val Asp Gly Gln Ile Leu Gly Gln Leu Arg Pro Leu Thr Glu
    50                  55                  60
Glu Glu Glu Glu Glu Gly Ala Gly Ala Thr Leu Ser Arg Gly Pro Ala
65                  70                  75                  80
Phe Pro Gly Met Gly Ser Glu Glu Leu Arg Leu Ala Ser Phe Tyr Asp
                85                  90                  95
Trp Pro Leu Thr Ala Glu Val Pro Pro Glu Leu Leu Ala Ala Ala Gly
            100                 105                 110
Phe Phe His Thr Gly His Gln Asp Lys Val Arg Cys Phe Phe Cys Tyr
        115                 120                 125
Gly Gly Leu Gln Ser Trp Lys Arg Gly Asp Asp Pro Trp Thr Glu His
    130                 135                 140
Ala Lys Trp Phe Pro Ser Cys Gln Phe Leu Leu Arg Ser Lys Gly Arg
145                 150                 155                 160
Asp Phe Val His Ser Val Gln Glu Thr His Ser Gln Leu Leu Gly Ser
                165                 170                 175
Trp Asp Pro Trp Glu Glu Pro Glu Asp Ala Ala Pro Val Ala Pro Ser
            180                 185                 190
Val Pro Ala Ser Gly Tyr Pro Glu Leu Pro Thr Pro Arg Arg Glu Val
        195                 200                 205
Gln Ser Glu Ser Ala Gln Glu Pro Gly Ala Arg Asp Val Glu Ala Gln
    210                 215                 220
Leu Arg Arg Leu Gln Glu Glu Arg Thr Cys Lys Val Cys Leu Asp Arg
225                 230                 235                 240
Ala Val Ser Ile Val Phe Val Pro Cys Gly His Leu Val Cys Ala Glu
                245                 250                 255
Cys Ala Pro Gly Leu Gln Leu Cys Pro Ile Cys Arg Ala Pro Val Arg
            260                 265                 270
Ser Arg Val Arg Thr Phe Leu Ser
        275                 280

<210> 351
<211> 596
<212> PRT
<213> Homo sapiens

<400> 351
Met Gly Ser Val Ser Ser Leu Ile Ser Gly His Ser Phe His Ser Lys
1               5                   10                  15
His Cys Arg Ala Ser Gln Tyr Lys Leu Arg Lys Ser Ser His Leu Lys
            20                  25                  30
Lys Leu Asn Arg Tyr Ser Asp Gly Leu Leu Arg Phe Gly Phe Ser Gln

35                  40                  45
Asp Ser Gly His Gly Lys Ser Ser Ser Lys Met Gly Lys Ser Glu Asp
    50                  55                  60
Phe Phe Tyr Ile Lys Val Ser Gln Lys Ala Arg Gly Ser His His Pro
65                  70                  75                  80
Asp Tyr Thr Ala Leu Ser Ser Gly Asp Leu Gly Gly Gln Ala Gly Val
                85                  90                  95
Asp Phe Asp Pro Ser Thr Pro Pro Lys Leu Met Pro Phe Ser Asn Gln
            100                 105                 110
Leu Glu Met Gly Ser Glu Lys Gly Ala Val Arg Pro Thr Ala Phe Lys
        115                 120                 125
Pro Val Leu Pro Arg Ser Gly Ala Ile Leu His Ser Ser Pro Glu Ser
    130                 135                 140
Ala Ser His Gln Leu His Pro Ala Pro Pro Asp Lys Pro Lys Glu Gln
145                 150                 155                 160
Glu Leu Lys Pro Gly Leu Cys Ser Gly Ala Leu Ser Asp Ser Gly Arg
                165                 170                 175
Asn Ser Met Ser Ser Leu Pro Thr His Ser Thr Ser Ser Ser Tyr Gln
            180                 185                 190
Leu Asp Pro Leu Val Thr Pro Val Gly Pro Thr Ser Arg Phe Gly Gly
        195                 200                 205
Ser Ala His Asn Ile Thr Gln Gly Ile Val Leu Gln Asp Ser Asn Met
    210                 215                 220
Met Ser Leu Lys Ala Leu Ser Phe Ser Asp Gly Gly Ser Lys Leu Gly
225                 230                 235                 240
His Ser Asn Lys Ala Asp Lys Gly Pro Ser Cys Val Arg Ser Pro Ile
                245                 250                 255
Ser Thr Asp Glu Cys Ser Ile Gln Glu Leu Glu Gln Lys Leu Leu Glu
            260                 265                 270
Arg Glu Gly Ala Leu Gln Lys Leu Gln Arg Ser Phe Glu Glu Lys Glu
        275                 280                 285
Leu Ala Ser Ser Leu Ala Tyr Glu Glu Arg Pro Arg Arg Cys Arg Asp
    290                 295                 300
Glu Leu Glu Gly Pro Glu Pro Lys Gly Gly Asn Lys Leu Lys Gln Ala
305                 310                 315                 320
Ser Gln Lys Ser Gln Arg Ala Gln Gln Val Leu His Leu Gln Val Leu
                325                 330                 335
Gln Leu Gln Gln Glu Lys Arg Gln Leu Arg Gln Glu Leu Glu Ser Leu
            340                 345                 350
Met Lys Glu Gln Asp Leu Leu Glu Thr Lys Leu Arg Ser Tyr Glu Arg
        355                 360                 365
Glu Lys Thr Ser Phe Gly Pro Ala Leu Glu Glu Thr Gln Trp Glu Val
    370                 375                 380
Cys Gln Lys Ser Gly Glu Ile Ser Leu Leu Lys Gln Gln Leu Lys Glu
385                 390                 395                 400
Ser Gln Thr Glu Val Asn Ala Lys Ala Ser Glu Ile Leu Gly Leu Lys
                405                 410                 415
Ala Gln Leu Lys Asp Thr Arg Gly Lys Leu Glu Gly Leu Glu Leu Arg
            420                 425                 430
Thr Gln Asp Leu Glu Gly Ala Leu Arg Thr Lys Gly Leu Glu Leu Glu
        435                 440                 445
Val Cys Glu Asn Glu Leu Gln Arg Lys Lys Asn Glu Ala Glu Leu Leu
    450                 455                 460
Arg Glu Lys Val Asn Leu Leu Glu Gln Glu Leu Gln Glu Leu Arg Ala
465                 470                 475                 480
Gln Ala Ala Leu Ala Arg Asp Met Gly Pro Pro Thr Phe Pro Glu Asp
                485                 490                 495
Val Pro Ala Leu Gln Arg Glu Leu Glu Arg Leu Arg Ala Glu Leu Arg
            500                 505                 510
Glu Glu Arg Gln Gly His Asp Gln Met Ser Ser Gly Phe Gln His Glu
        515                 520                 525
Arg Leu Val Trp Lys Glu Glu Lys Glu Lys Val Ile Gln Tyr Gln Lys
    530                 535                 540

Gln Leu Gln Gln Ser Tyr Val Ala Met Tyr Gln Arg Asn Gln Arg Leu
545                 550                 555                 560
Glu Lys Ala Leu Gln Gln Leu Ala Arg Gly Asp Ser Ala Gly Glu Pro
                565                 570                 575
Leu Glu Val Asp Leu Glu Gly Ala Asp Ile Pro Tyr Glu Asp Ile Ile
            580                 585                 590
Ala Thr Glu Ile
        595

<210> 352
<211> 730
<212> PRT
<213> Homo sapiens

<400> 352
Met Pro Lys Asn Ser Lys Val Val Lys Arg Glu Leu Asp Asp Asp Val
1               5                   10                  15
Thr Glu Ser Val Lys Asp Leu Leu Ser Asn Glu Asp Ala Ala Asp Asp
            20                  25                  30
Ala Phe Lys Thr Ser Glu Leu Ile Val Asp Gly Gln Glu Glu Lys Asp
        35                  40                  45
Thr Asp Val Glu Glu Gly Ser Glu Val Glu Asp Glu Arg Pro Ala Trp
    50                  55                  60
Asn Ser Lys Leu Gln Tyr Ile Leu Ala Gln Val Gly Phe Ser Val Gly
65                  70                  75                  80
Leu Gly Asn Val Trp Arg Phe Pro Tyr Leu Cys Gln Lys Asn Gly Gly
                85                  90                  95
Gly Ala Tyr Leu Leu Pro Tyr Leu Ile Leu Leu Met Val Ile Gly Ile
            100                 105                 110
Pro Leu Phe Phe Leu Glu Leu Ser Val Gly Gln Arg Ile Arg Arg Gly
        115                 120                 125
Ser Ile Gly Val Trp Asn Tyr Ile Ser Pro Lys Leu Gly Gly Ile Gly
    130                 135                 140
Phe Ala Ser Cys Val Val Cys Tyr Phe Val Ala Leu Tyr Tyr Asn Val
145                 150                 155                 160
Ile Ile Gly Trp Ser Leu Phe Tyr Phe Ser Gln Ser Phe Gln Gln Pro
                165                 170                 175
Leu Pro Trp Asp Gln Cys Pro Leu Val Lys Asn Ala Ser His Thr Phe
            180                 185                 190
Val Glu Pro Glu Cys Glu Gln Ser Ser Ala Thr Thr Tyr Tyr Trp Tyr
        195                 200                 205
Arg Glu Ala Leu Asn Ile Ser Ser Ser Ile Ser Glu Ser Gly Gly Leu
    210                 215                 220
Asn Trp Lys Met Thr Ile Cys Leu Leu Ala Ala Trp Val Met Val Cys
225                 230                 235                 240
Leu Ala Met Ile Lys Gly Ile Gln Ser Ser Gly Lys Ile Ile Tyr Phe
                245                 250                 255
Ser Ser Leu Phe Pro Tyr Val Val Leu Ile Cys Phe Leu Ile Arg Ala
            260                 265                 270
Phe Leu Leu Asn Gly Ser Ile Asp Gly Ile Arg His Met Phe Thr Pro
        275                 280                 285
Lys Leu Glu Ile Met Leu Glu Pro Lys Val Trp Arg Glu Ala Ala Thr
    290                 295                 300
Gln Val Phe Phe Ala Leu Gly Leu Gly Phe Gly Gly Val Ile Ala Phe
305                 310                 315                 320
Ser Ser Tyr Asn Lys Arg Asp Asn Asn Cys His Phe Asp Ala Val Leu
                325                 330                 335
Val Ser Phe Ile Asn Phe Phe Thr Ser Val Leu Ala Thr Leu Val Val
            340                 345                 350
Phe Ala Val Leu Gly Phe Lys Ala Asn Val Ile Asn Glu Lys Cys Ile
        355                 360                 365
Thr Gln Asn Ser Glu Thr Ile Met Lys Phe Leu Lys Met Gly Asn Ile
    370                 375                 380

Ser Gln Asp Ile Ile Pro His His Ile Asn Leu Ser Thr Val Thr Ala
385                 390                 395                 400
Glu Asp Tyr His Leu Val Tyr Asp Ile Ile Gln Lys Val Lys Glu Glu
                405                 410                 415
Glu Phe Pro Ala Leu His Leu Asn Ser Cys Lys Ile Glu Glu Glu Leu
            420                 425                 430
Asn Lys Ala Val Gln Gly Thr Gly Leu Ala Phe Ile Ala Phe Thr Glu
        435                 440                 445
Ala Met Thr His Phe Pro Ala Ser Pro Phe Trp Ser Val Met Phe Phe
    450                 455                 460
Leu Met Leu Val Asn Leu Gly Leu Gly Ser Met Phe Gly Thr Ile Glu
465                 470                 475                 480
Gly Ile Val Thr Pro Ile Val Asp Thr Phe Lys Val Arg Lys Glu Ile
                485                 490                 495
Leu Thr Val Ile Cys Cys Leu Leu Ala Phe Cys Ile Gly Leu Ile Phe
            500                 505                 510
Val Gln Arg Ser Gly Asn Tyr Phe Val Thr Met Phe Asp Asp Tyr Ser
        515                 520                 525
Ala Thr Leu Pro Leu Leu Ile Val Val Ile Leu Glu Asn Ile Ala Val
    530                 535                 540
Cys Phe Val Tyr Gly Ile Asp Lys Phe Met Glu Asp Leu Lys Asp Met
545                 550                 555                 560
Leu Gly Phe Ala Pro Ser Arg Tyr Tyr Tyr Tyr Met Trp Lys Tyr Ile
                565                 570                 575
Ser Pro Leu Met Leu Leu Ser Leu Leu Ile Ala Ser Val Val Asn Met
            580                 585                 590
Gly Leu Ser Pro Pro Gly Tyr Asn Ala Trp Ile Glu Asp Lys Ala Ser
        595                 600                 605
Glu Glu Phe Leu Ser Tyr Pro Thr Trp Gly Leu Val Val Cys Val Ser
    610                 615                 620
Leu Val Val Phe Ala Ile Leu Pro Val Pro Val Val Phe Ile Val Arg
625                 630                 635                 640
Arg Phe Asn Leu Ile Asp Asp Ser Ser Gly Asn Leu Ala Ser Val Thr
                645                 650                 655
Tyr Lys Arg Gly Arg Val Leu Lys Glu Pro Val Asn Leu Glu Gly Asp
            660                 665                 670
Asp Thr Ser Leu Ile His Gly Lys Ile Pro Ser Glu Met Pro Ser Pro
        675                 680                 685
Asn Phe Gly Lys Asn Ile Tyr Arg Lys Gln Ser Gly Ser Pro Thr Leu
    690                 695                 700
Asp Thr Ala Pro Asn Gly Arg Tyr Gly Ile Gly Tyr Leu Met Ala Asp
705                 710                 715                 720

Ile Met Pro Asp Met Pro Glu Ser Asp Leu
                725                 730

<210> 353
<211> 355
<212> PRT
<213> Homo sapiens

<400> 353
Met Glu Glu Gly Glu Arg Ser Pro Leu Leu Ser Gln Glu Thr Ala Gly
1               5                   10                  15
Gln Lys Pro Leu Ser Val His Arg Pro Pro Thr Ser Gly Cys Leu Gly
            20                  25                  30
Pro Val Pro Arg Glu Asp Gln Ala Glu Ala Trp Gly Cys Ser Cys Cys
        35                  40                  45
Pro Pro Glu Thr Lys His Gln Ala Leu Ser Gly Thr Pro Lys Lys Gly
    50                  55                  60
Pro Ala Pro Ser Leu Ser Pro Gly Ser Ser Cys Val Lys Tyr Leu Ile
65                  70                  75                  80
Phe Leu Ser Asn Phe Pro Phe Ser Leu Leu Gly Leu Leu Ala Leu Ala

                  85                  90                  95
Ile Gly Leu Trp Gly Leu Ala Val Lys Gly Ser Leu Gly Ser Asp Leu
            100                 105                 110
Gly Gly Pro Leu Pro Thr Asp Pro Met Leu Gly Leu Ala Leu Gly Gly
        115                 120                 125
Leu Val Val Ser Ala Ala Ser Leu Ala Gly Cys Leu Gly Ala Leu Cys
    130                 135                 140
Glu Asn Thr Cys Leu Leu Arg Gly Phe Ser Gly Gly Ile Leu Ala Phe
145                 150                 155                 160
Leu Val Leu Glu Ala Val Ala Gly Ala Leu Val Val Ala Leu Trp Gly
                165                 170                 175
Pro Leu Gln Asp Ser Leu Glu His Thr Leu Arg Val Ala Ile Ala His
            180                 185                 190
Tyr Gln Asp Asp Pro Asp Leu Arg Phe Leu Leu Asp Gln Val Gln Leu
        195                 200                 205
Gly Leu Arg Cys Cys Gly Ala Ala Ser Tyr Gln Asp Trp Gln Gln Asn
    210                 215                 220
Leu Tyr Phe Asn Cys Ser Ser Pro Gly Val Gln Ala Cys Ser Leu Pro
225                 230                 235                 240
Ala Ser Cys Cys Ile Asp Pro Arg Glu Asp Gly Ala Ser Val Asn Asp
                245                 250                 255
Gln Cys Gly Phe Gly Val Leu Arg Leu Asp Ala Asp Ala Ala Gln Arg
            260                 265                 270
Val Val Tyr Leu Glu Gly Cys Gly Pro Pro Leu Arg Arg Trp Leu Arg
        275                 280                 285
Ala Asn Leu Ala Ala Ser Gly Gly Tyr Ala Ile Ala Val Val Leu Leu
    290                 295                 300
Gln Gly Ala Glu Leu Leu Leu Ala Ala Arg Leu Leu Gly Ala Leu Ala
305                 310                 315                 320
Ala Arg Ser Gly Ala Ala Tyr Gly Pro Gly Ala His Gly Glu Asp Arg
                325                 330                 335
Ala Gly Pro Gln Ser Pro Ser Pro Gly Ala Pro Pro Ala Ala Lys Pro
            340                 345                 350
Ala Arg Gly
        355

<210> 354
<211> 553
<212> PRT
<213> Homo sapiens

<400> 354
   Met Ala Asp Pro Glu Val Val Val Ser Ser Cys Ser Ser His Glu Glu
   1               5                   10                  15
   Glu Asn Arg Cys Asn Phe Asn Gln Gln Thr Ser Pro Ser Glu Glu Leu
               20                  25                  30
   Leu Leu Glu Asp Gln Met Arg Arg Lys Leu Lys Phe Phe Phe Met Asn
           35                  40                  45
   Pro Cys Glu Lys Phe Trp Ala Arg Gly Arg Lys Pro Trp Lys Leu Ala
       50                  55                  60
   Ile Gln Ile Leu Lys Ile Ala Met Val Thr Ile Gln Leu Val Leu Phe
   65                  70                  75                  80
   Gly Leu Ser Asn Gln Met Val Val Ala Phe Lys Glu Glu Asn Thr Ile
                   85                  90                  95
   Ala Phe Lys His Leu Phe Leu Lys Gly Tyr Met Asp Arg Met Asp Asp
               100                 105                 110
   Thr Tyr Ala Val Tyr Thr Gln Ser Asp Val Tyr Asp Gln Leu Ile Phe
           115                 120                 125
   Ala Val Asn Gln Tyr Leu Gln Leu Tyr Asn Val Ser Val Gly Asn His
       130                 135                 140
   Ala Tyr Glu Asn Lys Gly Thr Lys Gln Ser Ala Met Ala Ile Cys Gln
   145                 150                 155                 160
   His Phe Tyr Lys Arg Gly Asn Ile Tyr Pro Gly Asn Asp Thr Phe Asp
   165 170 175
   Ile Asp Pro Glu Ile Glu Thr Glu Cys Phe Phe Val Glu Pro Asp Glu
   180 185 190
   Pro Phe His Ile Gly Thr Pro Ala Glu Asn Lys Leu Asn Leu Thr Leu
   195 200 205
   Asp Phe His Arg Leu Leu Thr Val Glu Leu Gln Phe Lys Leu Lys Ala
   210 215 220
   Ile Asn Leu Gln Thr Val Arg His Gln Glu Leu Pro Asp Cys Tyr Asp
   225 230 235 240
   Phe Thr Leu Thr Ile Thr Phe Asp Asn Lys Ala His Ser Gly Arg Ile
   245 250 255
   Lys Ile Ser Leu Asp Asn Asp Ile Ser Ile Arg Glu Cys Lys Asp Trp
   260 265 270
   His Val Ser Gly Ser Ile Gln Lys Asn Thr His Tyr Met Met Ile Phe
   275 280 285
   Asp Ala Phe Val Ile Leu Thr Cys Leu Val Ser Leu Ile Leu Cys Ile
   290 295 300
   Arg Ser Val Ile Arg Gly Leu Gln Leu Gln Gln Glu Phe Val Asn Phe
   305 310 315 320
   Phe Leu Leu His Tyr Lys Lys Glu Val Ser Val Ser Asp Gln Met Glu
   325 330 335
   Phe Val Asn Gly Trp Tyr Ile Met Ile Ile Ile Ser Asp Ile Leu Thr
   340 345 350
   Ile Ile Gly Ser Ile Leu Lys Met Glu Ile Gln Ala Lys Ser Leu Thr
   355 360 365
   Ser Tyr Asp Val Cys Ser Ile Leu Leu Gly Thr Ser Thr Met Leu Val
   370 375 380
   Trp Leu Gly Val Ile Arg Tyr Leu Gly Phe Phe Ala Lys Tyr Asn Leu
   385 390 395 400
   Leu Ile Leu Thr Leu Gln Ala Ala Leu Pro Asn Val Ile Arg Phe Cys
   405 410 415
   Cys Cys Ala Ala Met Ile Tyr Leu Gly Tyr Cys Phe Cys Gly Trp Ile
   420 425 430
   Val Leu Gly Pro Tyr His Asp Lys Phe Arg Ser Leu Asn Met Val Ser
   435 440 445
   Glu Cys Leu Phe Ser Leu Ile Asn Gly Asp Asp Met Phe Ala Thr Phe
   450 455 460
   Ala Lys Met Gln Gln Lys Ser Tyr Leu Val Trp Leu Phe Ser Arg Ile
   465 470 475 480
   Tyr Leu Tyr Ser Phe Ile Ser Leu Phe Ile Tyr Met Ile Leu Ser Leu
   485 490 495
   Phe Ile Ala Leu Ile Thr Asp Thr Tyr Glu Thr Ile Lys Gln Tyr Gln
   500 505 510
   Gln Asp Gly Phe Pro Glu Thr Glu Leu Arg Thr Phe Ile Ser Glu Cys
   515 520 525
   Lys Asp Leu Pro Asn Ser Gly Lys Tyr Arg Leu Glu Asp Asp Pro Pro
   530 535 540
   Val Ser Leu Phe Cys Cys Cys Lys Lys
   545 550

<210> 355
<211> 238
<212> PRT
<213> Homo sapiens

<400> 355

<210> 356
<211> 509
<212> PRT
<213> Homo sapiens

<400> 356
Met Gly Cys Gly Cys Ser Ser His Pro Glu Asp Asp Trp Met Glu Asn
1               5                   10                  15
Ile Asp Val Cys Glu Asn Cys His Tyr Pro Ile Val Pro Leu Asp Gly
            20                  25                  30
Lys Gly Thr Leu Leu Ile Arg Asn Gly Ser Glu Val Arg Asp Pro Leu
        35                  40                  45

Val Thr Tyr Glu Gly Ser Asn Pro Pro Ala Ser Pro Leu Gln Asp Asn
    50                  55                  60
Leu Val Ile Ala Leu His Ser Tyr Glu Pro Ser His Asp Gly Asp Leu
65                  70                  75                  80
Gly Phe Glu Lys Gly Glu Pro Leu Arg Ile Leu Glu Gln Ser Gly Glu
                85                  90                  95
Trp Trp Lys Ala Gln Ser Leu Thr Thr Gly Gln Glu Gly Phe Ile Pro
            100                 105                 110
Phe Asn Phe Val Ala Lys Ala Asn Ser Leu Glu Pro Glu Pro Trp Phe
        115                 120                 125
Phe Lys Asn Leu Ser Arg Lys Asp Ala Glu Arg Gln Leu Leu Ala Pro
    130                 135                 140
Gly Asn Thr His Gly Ser Phe Leu Ile Arg Glu Ser Glu Ser Thr Ala
145                 150                 155                 160
Gly Ser Phe Ser Leu Ser Val Arg Asp Phe Asp Gln Asn Gln Gly Glu
                165                 170                 175
Val Val Lys His Tyr Lys Ile Arg Asn Leu Asp Asn Gly Gly Phe Tyr
            180                 185                 190
Ile Ser Pro Arg Ile Thr Phe Pro Gly Leu His Glu Leu Val Arg His
        195                 200                 205
Tyr Thr Asn Ala Ser Asp Gly Leu Cys Thr Arg Leu Ser Arg Pro Cys
    210                 215                 220
Gln Thr Gln Lys Pro Gln Lys Pro Trp Trp Glu Asp Glu Trp Glu Val
225                 230                 235                 240
Pro Arg Glu Thr Leu Lys Leu Val Glu Arg Leu Gly Ala Gly Gln Phe
                245                 250                 255

Gly Glu Val Trp Met Gly Tyr Tyr Asn Gly His Thr Lys Val Ala Val
                260                 265                 270
Lys Ser Leu Lys Gln Gly Ser Met Ser Pro Asp Ala Phe Leu Ala Glu
        275                 280                 285
Ala Asn Leu Met Lys Gln Leu Gln His Gln Arg Leu Val Arg Leu Tyr
    290                 295                 300
Ala Val Val Thr Gln Glu Pro Ile Tyr Ile Ile Thr Glu Tyr Met Glu
305                 310                 315                 320
Asn Gly Ser Leu Val Asp Phe Leu Lys Thr Pro Ser Gly Ile Lys Leu
                325                 330                 335
Thr Ile Asn Lys Leu Leu Asp Met Ala Ala Gln Ile Ala Glu Gly Met
                340                 345                 350
Ala Phe Ile Glu Glu Arg Asn Tyr Ile His Arg Asp Leu Arg Ala Ala
        355                 360                 365
Asn Ile Leu Val Ser Asp Thr Leu Ser Cys Lys Ile Ala Asp Phe Gly
    370                 375                 380
Leu Ala Arg Leu Ile Glu Asp Asn Glu Tyr Thr Ala Arg Glu Gly Ala
385                 390                 395                 400
Lys Phe Pro Ile Lys Trp Thr Ala Pro Glu Ala Ile Asn Tyr Gly Thr
                405                 410                 415
Phe Thr Ile Lys Ser Asp Val Trp Ser Phe Gly Ile Leu Leu Thr Glu
                420                 425                 430
Ile Val Thr His Gly Arg Ile Pro Tyr Pro Gly Met Thr Asn Pro Glu
        435                 440                 445
Val Ile Gln Asn Leu Glu Arg Gly Tyr Arg Met Val Arg Pro Asp Asn
    450                 455                 460
Cys Pro Glu Glu Leu Tyr Gln Leu Met Arg Leu Cys Trp Lys Glu Arg
465                 470                 475                 480
Pro Glu Asp Arg Pro Thr Phe Asp Tyr Leu Arg Ser Val Leu Glu Asp
                485                 490                 495
Phe Phe Thr Ala Thr Glu Gly Gln Tyr Gln Pro Gln Pro
                500                 505

<210> 357
<211> 384
<212> PRT
<213> Homo sapiens

<400> 357
Met Ala Ile Ala Tyr Leu Gly Ser Ser Cys Pro Ser Gln Pro Pro Ser
 1               5                  10                  15
Ser Leu Ala Leu Ser Leu Ser Pro Thr Pro Ser Asp Phe Glu Gln Glu
                20                  25                  30
Ser Gly Ile Glu Thr Ala Met Arg Phe Ser Pro Asp Val Ala Leu Ala
        35                  40                  45
Val Ser Thr Thr Pro Ala Val Leu Pro Thr Thr Asn Ile Gln Pro Val
    50                  55                  60
Gly Thr Pro Phe Glu Glu Leu Pro Ser Glu Arg Pro Thr Leu Glu Pro
65                  70                  75                  80
Ala Thr Ser Pro Leu Val Val Thr Glu Val Pro Glu Glu Pro Ser Gln
                85                  90                  95
Arg Ala Thr Thr Val Ser Thr Thr Met Ala Thr Thr Ala Ala Thr Ser
                100                 105                 110
Thr Gly Asp Pro Thr Val Ala Thr Val Pro Ala Thr Val Ala Thr Ala
        115                 120                 125
Thr Pro Ser Thr Pro Ala Ala Pro Pro Phe Thr Ala Thr Thr Ala Val
    130                 135                 140
Ile Arg Thr Thr Gly Val Arg Arg Leu Leu Pro Leu Pro Leu Thr Thr
145                 150                 155                 160
Val Ala Thr Ala Arg Ala Thr Thr Pro Glu Ala Pro Ser Pro Pro Thr
                165                 170                 175
Thr Ala Ala Val Leu Asp Thr Glu Ala Pro Thr Pro Arg Leu Val Ser

180                 185                 190
Thr Ala Thr Ser Arg Pro Arg Ala Leu Pro Arg Pro Ala Thr Thr Gln
        195                 200                 205
Glu Pro Asp Ile Pro Glu Arg Ser Thr Leu Pro Leu Gly Thr Thr Ala
    210                 215                 220
Pro Gly Pro Thr Glu Val Ala Gln Thr Pro Thr Pro Glu Thr Phe Leu
225                 230                 235                 240
Thr Thr Ile Arg Asp Glu Pro Glu Val Pro Val Ser Gly Gly Pro Ser
                245                 250                 255
Gly Asp Phe Glu Leu Pro Glu Glu Glu Thr Thr Gln Pro Asp Thr Ala
            260                 265                 270
Asn Glu Val Val Ala Val Gly Gly Ala Ala Ala Lys Ala Ser Ser Pro
        275                 280                 285
Pro Gly Thr Leu Pro Lys Gly Ala Arg Pro Gly Pro Gly Leu Leu Asp
    290                 295                 300
Asn Ala Ile Asp Ser Gly Ser Ser Ala Ala Gln Leu Pro Gln Lys Ser
305                 310                 315                 320
Ile Leu Glu Arg Lys Glu Val Leu Val Ala Val Ile Val Gly Gly Val
                325                 330                 335
Val Gly Ala Leu Phe Ala Ala Phe Leu Val Thr Leu Leu Ile Tyr Arg
            340                 345                 350
Met Lys Lys Lys Asp Glu Gly Ser Tyr Thr Leu Glu Glu Pro Lys Gln
        355                 360                 365
Ala Ser Val Thr Tyr Gln Lys Pro Asp Lys Gln Glu Glu Phe Tyr Ala
    370                 375                 380

<210> 358
<211> 463
<212> PRT
<213> Homo sapiens

<400> 358
Met Tyr Gly Asn Tyr Ser His Phe Met Lys Phe Pro Ala Gly Tyr Gly
1               5                   10                  15
Gly Ser Pro Gly His Thr Gly Ser Thr Ser Met Ser Pro Ser Ala Ala
            20                  25                  30
Leu Ser Thr Gly Lys Pro Met Asp Ser His Pro Ser Tyr Thr Asp Thr
        35                  40                  45
Pro Val Ser Ala Pro Arg Thr Leu Ser Ala Val Gly Thr Pro Leu Asn
    50                  55                  60
Ala Leu Gly Ser Pro Tyr Arg Val Ile Thr Ser Ala Met Gly Pro Pro
65                  70                  75                  80
Ser Gly Ala Leu Ala Ala Pro Pro Gly Ile Asn Leu Val Ala Pro Pro
                85                  90                  95
Ser Ser Gln Leu Asn Val Val Asn Ser Val Ser Ser Ser Glu Asp Ile
            100                 105                 110
Lys Pro Leu Pro Gly Leu Pro Gly Ile Gly Asn Met Asn Tyr Pro Ser
        115                 120                 125
Thr Ser Pro Gly Ser Leu Val Lys His Ile Cys Ala Ile Cys Gly Asp
    130                 135                 140
Arg Ser Ser Gly Lys His Tyr Gly Val Tyr Ser Cys Glu Gly Cys Lys
145                 150                 155                 160
Gly Phe Phe Lys Arg Thr Ile Arg Lys Asp Leu Ile Tyr Thr Cys Arg
                165                 170                 175
Asp Asn Lys Asp Cys Leu Ile Asp Lys Arg Gln Arg Asn Arg Cys Gln
            180                 185                 190
Tyr Cys Arg Tyr Gln Lys Cys Leu Val Met Gly Met Lys Arg Glu Ala
        195                 200                 205
Val Gln Glu Glu Arg Gln Arg Ser Arg Glu Arg Ala Glu Ser Glu Ala
    210                 215                 220
Glu Cys Ala Thr Ser Gly His Glu Asp Met Pro Val Glu Arg Ile Leu
225                 230                 235                 240

Glu Ala Glu Leu Ala Val Glu Pro Lys Thr Glu Ser Tyr Gly Asp Met
                245                 250                 255
Asn Met Glu Asn Ser Thr Asn Asp Pro Val Thr Asn Ile Cys His Ala
                260                 265                 270
Ala Asp Lys Gln Leu Phe Thr Leu Val Glu Trp Ala Lys Arg Ile Pro
        275                 280                 285
His Phe Ser Asp Leu Thr Leu Glu Asp Gln Val Ile Leu Leu Arg Ala
    290                 295                 300
Gly Trp Asn Glu Leu Leu Ile Ala Ser Phe Ser His Arg Ser Val Ser
305                 310                 315                 320
Val Gln Asp Gly Ile Leu Leu Ala Thr Gly Leu His Val His Arg Ser
                325                 330                 335
Ser Ala His Ser Ala Gly Val Gly Ser Ile Phe Asp Arg Val Leu Thr
                340                 345                 350
Glu Leu Val Ser Lys Met Lys Asp Met Gln Met Asp Lys Ser Glu Leu
        355                 360                 365
Gly Cys Leu Arg Ala Ile Val Leu Phe Asn Pro Asp Ala Lys Gly Leu
    370                 375                 380
Ser Asn Pro Ser Glu Val Glu Thr Leu Arg Glu Lys Val Tyr Ala Thr
385                 390                 395                 400
Leu Glu Ala Tyr Thr Lys Gln Lys Tyr Pro Glu Gln Pro Gly Arg Phe
                405                 410                 415
Ala Lys Leu Leu Leu Arg Leu Pro Ala Leu Arg Ser Ile Gly Leu Lys
                420                 425                 430
Cys Leu Glu His Leu Phe Phe Phe Lys Leu Ile Gly Asp Thr Pro Ile
        435                 440                 445
Asp Thr Phe Leu Met Glu Met Leu Glu Thr Pro Leu Gln Ile Thr
    450                 455                 460

<210> 359
<211> 459
<212> PRT
<213> Homo sapiens

<400> 359

<210> 360
<211> 92
<212> PRT
<213> Homo sapiens

<400> 360
Met Ser Glu Leu Glu Lys Ala Met Val Ala Leu Ile Asp Val Phe His
1               5                   10                  15
Gln Tyr Ser Gly Arg Glu Gly Asp Lys His Lys Leu Lys Lys Ser Glu
            20                  25                  30
Leu Lys Glu Leu Ile Asn Asn Glu Leu Ser His Phe Leu Glu Glu Ile
        35                  40                  45
Lys Glu Gln Glu Val Val Asp Lys Val Met Glu Thr Leu Asp Asn Asp
    50                  55                  60
Gly Asp Gly Glu Cys Asp Phe Gln Glu Phe Met Ala Phe Val Ala Met
65                  70                  75                  80
Val Thr Thr Ala Cys His Glu Phe Phe Glu His Glu
                85                  90

<210> 361
<211> 810
<212> PRT
<213> Homo sapiens

<400> 361
Met Pro Met Asp Leu Ile Leu Val Val Trp Phe Cys Val Cys Thr Ala
1               5                   10                  15
Arg Thr Val Val Gly Phe Gly Met Asp Pro Asp Leu Gln Met Asp Ile
            20                  25                  30
Val Thr Glu Leu Asp Leu Val Asn Thr Thr Leu Gly Val Ala Gln Val

35                  40                  45
Ser Gly Met His Asn Ala Ser Lys Ala Phe Leu Phe Gln Asp Ile Glu
    50                  55                  60
Arg Glu Ile His Ala Ala Pro His Val Ser Glu Lys Leu Ile Gln Leu
65                  70                  75                  80
Phe Gln Asn Lys Ser Glu Phe Thr Ile Leu Ala Thr Val Gln Gln Lys
                85                  90                  95
Pro Ser Thr Ser Gly Val Ile Leu Ser Ile Arg Glu Leu Glu His Ser
            100                 105                 110
Tyr Phe Glu Leu Glu Ser Ser Gly Leu Arg Asp Glu Ile Arg Tyr His
        115                 120                 125
Tyr Ile His Asn Gly Lys Pro Arg Thr Glu Ala Leu Pro Tyr Arg Met
    130                 135                 140
Ala Asp Gly Gln Trp His Lys Val Ala Leu Ser Val Ser Ala Ser His
145                 150                 155                 160
Leu Leu Leu His Val Asp Cys Asn Arg Ile Tyr Glu Arg Val Ile Asp
                165                 170                 175
Pro Pro Asp Thr Asn Leu Pro Pro Gly Ile Asn Leu Trp Leu Gly Gln
            180                 185                 190
Arg Asn Gln Lys His Gly Leu Phe Lys Gly Ile Ile Gln Asp Gly Lys
        195                 200                 205
Ile Ile Phe Met Pro Asn Gly Tyr Ile Thr Gln Cys Pro Asn Leu Asn
    210                 215                 220
His Thr Cys Pro Thr Cys Ser Asp Phe Leu Ser Leu Val Gln Gly Ile
225                 230                 235                 240

Met Asp Leu Gln Glu Leu Leu Ala Lys Met Thr Ala Lys Leu Asn Tyr
                245                 250                 255
Ala Glu Thr Arg Leu Ser Gln Leu Glu Asn Cys His Cys Glu Lys Thr
            260                 265                 270
Cys Gln Val Ser Gly Leu Leu Tyr Arg Asp Gln Asp Ser Trp Val Asp
        275                 280                 285
Gly Asp His Cys Arg Asn Cys Thr Cys Lys Ser Gly Ala Val Glu Cys
    290                 295                 300
Arg Arg Met Ser Cys Pro Pro Leu Asn Cys Ser Pro Asp Ser Leu Pro
305                 310                 315                 320
Val His Ile Ala Gly Gln Cys Cys Lys Val Cys Arg Pro Lys Cys Ile
                325                 330                 335
Tyr Gly Gly Lys Val Leu Ala Glu Gly Gln Arg Ile Leu Thr Lys Ser
            340                 345                 350
Cys Arg Glu Cys Arg Gly Gly Val Leu Val Lys Ile Thr Glu Met Cys
        355                 360                 365
Pro Pro Leu Asn Cys Ser Glu Lys Asp His Ile Leu Pro Glu Asn Gln
    370                 375                 380
Cys Cys Arg Val Cys Arg Gly His Asn Phe Cys Ala Glu Gly Pro Lys
385                 390                 395                 400
Cys Gly Glu Asn Ser Glu Cys Lys Asn Trp Asn Thr Lys Ala Thr Cys
                405                 410                 415
Glu Cys Lys Ser Gly Tyr Ile Ser Val Gln Gly Asp Ser Ala Tyr Cys
            420                 425                 430
Glu Asp Ile Asp Glu Cys Ala Ala Lys Met His Tyr Cys His Ala Asn
        435                 440                 445
Thr Val Cys Val Asn Leu Pro Gly Leu Tyr Arg Cys Asp Cys Val Pro
    450                 455                 460
Gly Tyr Ile Arg Val Asp Asp Phe Ser Cys Thr Glu His Asp Glu Cys
465                 470                 475                 480
Gly Ser Gly Gln His Asn Cys Asp Glu Asn Ala Ile Cys Thr Asn Thr
                485                 490                 495
Val Gln Gly His Ser Cys Thr Cys Lys Pro Gly Tyr Val Gly Asn Gly
            500                 505                 510
Thr Ile Cys Arg Ala Phe Cys Glu Glu Gly Cys Arg Tyr Gly Gly Thr
        515                 520                 525
Cys Val Ala Pro Asn Lys Cys Val Cys Pro Ser Gly Phe Thr Gly Ser

530                 535                 540
His Cys Glu Lys Asp Ile Asp Glu Cys Ser Glu Gly Ile Ile Glu Cys
545                 550                 555                 560
His Asn His Ser Arg Cys Val Asn Leu Pro Gly Trp Tyr His Cys Glu
                565                 570                 575
Cys Arg Ser Gly Phe His Asp Asp Gly Thr Tyr Ser Leu Ser Gly Glu
            580                 585                 590
Ser Cys Ile Asp Ile Asp Glu Cys Ala Leu Arg Thr His Thr Cys Trp
        595                 600                 605
Asn Asp Ser Ala Cys Ile Asn Leu Ala Gly Gly Phe Asp Cys Leu Cys
    610                 615                 620
Pro Ser Gly Pro Ser Cys Ser Gly Asp Cys Pro His Glu Gly Gly Leu
625                 630                 635                 640
Lys His Asn Gly Gln Val Trp Thr Leu Lys Glu Asp Arg Cys Ser Val
                645                 650                 655
Cys Ser Cys Lys Asp Gly Lys Ile Phe Cys Arg Arg Thr Ala Cys Asp
            660                 665                 670
Cys Gln Asn Pro Ser Ala Asp Leu Phe Cys Cys Pro Glu Cys Asp Thr
        675                 680                 685
Arg Val Thr Ser Gln Cys Leu Asp Gln Asn Gly His Lys Leu Tyr Arg
    690                 695                 700
Ser Gly Asp Asn Trp Thr His Ser Cys Gln Gln Cys Arg Cys Leu Glu
705                 710                 715                 720

Gly Glu Val Asp Cys Trp Pro Leu Thr Cys Pro Asn Leu Ser Cys Glu
                725                 730                 735
Tyr Thr Ala Ile Leu Glu Gly Glu Cys Cys Pro Arg Cys Val Ser Asp
            740                 745                 750
Pro Cys Leu Ala Asp Asn Ile Thr Tyr Asp Ile Arg Lys Thr Cys Leu
        755                 760                 765
Asp Ser Tyr Gly Val Ser Arg Leu Ser Gly Ser Val Trp Thr Met Ala
    770                 775                 780
Gly Ser Pro Cys Thr Thr Cys Lys Cys Lys Asn Gly Arg Val Cys Cys
785                 790                 795                 800
Ser Val Asp Phe Glu Cys Leu Gln Asn Asn
                805                 810

<210> 362
<211> 530
<212> PRT
<213> Homo sapiens

<400> 362
   Met Gly Ser Asn Ser Gly Gln Ala Gly Arg His Ile Tyr Lys Ser Leu
   1               5                   10                  15
   Ala Asp Asp Gly Pro Phe Asp Ser Val Glu Pro Pro Lys Arg Pro Thr
               20                  25                  30
   Ser Arg Leu Ile Met His Ser Met Ala Met Phe Gly Arg Glu Phe Cys
           35                  40                  45
   Tyr Ala Val Glu Ala Ala Tyr Val Thr Pro Val Leu Leu Ser Val Gly
       50                  55                  60
   Leu Pro Ser Ser Leu Tyr Ser Ile Val Trp Phe Leu Ser Pro Ile Leu
   65                  70                  75                  80
   Gly Phe Leu Leu Gln Pro Val Val Gly Ser Ala Ser Asp His Cys Arg
                   85                  90                  95
   Ser Arg Trp Gly Arg Arg Arg Pro Tyr Ile Leu Thr Leu Gly Val Met
               100                 105                 110
   Met Leu Val Gly Met Ala Leu Tyr Leu Asn Gly Ala Thr Val Val Ala
           115                 120                 125
   Ala Leu Ile Ala Asn Pro Arg Arg Lys Leu Val Trp Ala Ile Ser Val
       130                 135                 140
   Thr Met Ile Gly Val Val Leu Phe Asp Phe Ala Ala Asp Phe Ile Asp
   145                 150                 155                 160
   Gly Pro Ile Lys Ala Tyr Leu Phe Asp Val Cys Ser His Gln Asp Lys
                   165                 170                 175
   Glu Lys Gly Leu His Tyr His Ala Leu Phe Thr Gly Phe Gly Gly Ala
               180                 185                 190
   Leu Gly Tyr Leu Leu Gly Ala Ile Asp Trp Ala His Leu Glu Leu Gly
           195                 200                 205
   Arg Leu Leu Gly Thr Glu Phe Gln Val Met Phe Phe Phe Ser Ala Leu
       210                 215                 220
   Val Leu Thr Leu Cys Phe Thr Val His Leu Cys Ser Ile Ser Glu Ala
   225                 230                 235                 240
   Pro Leu Thr Glu Val Ala Lys Gly Ile Pro Pro Gln Gln Thr Pro Gln
                   245                 250                 255
   Asp Pro Pro Leu Ser Ser Asp Gly Met Tyr Glu Tyr Gly Ser Ile Glu
               260                 265                 270
   Lys Val Lys Asn Gly Tyr Val Asn Pro Glu Leu Ala Met Gln Gly Ala
           275                 280                 285
   Lys Asn Lys Asn His Ala Glu Gln Thr Arg Arg Ala Met Thr Leu Lys
       290                 295                 300
   Ser Leu Leu Arg Ala Leu Val Asn Met Pro Pro His Tyr Arg Tyr Leu
   305                 310                 315                 320
   Cys Ile Ser His Leu Ile Gly Trp Thr Ala Phe Leu Ser Asn Met Leu
                   325                 330                 335
   Phe Phe Thr Asp Phe Met Gly Gln Ile Val Tyr Arg Gly Asp Pro Tyr
               340                 345                 350
   Ser Ala His Asn Ser Thr Glu Phe Leu Ile Tyr Glu Arg Gly Val Glu
           355                 360                 365
   Val Gly Cys Trp Gly Phe Cys Ile Asn Ser Val Phe Ser Ser Leu Tyr
       370                 375                 380
   Ser Tyr Phe Gln Lys Val Leu Val Ser Tyr Ile Gly Leu Lys Gly Leu
   385                 390                 395                 400
   Tyr Phe Thr Gly Tyr Leu Leu Phe Gly Leu Gly Thr Gly Phe Ile Gly
                   405                 410                 415
   Leu Phe Pro Asn Val Tyr Ser Thr Leu Val Leu Cys Ser Leu Phe Gly
               420                 425                 430
   Val Met Ser Ser Thr Leu Tyr Thr Val Pro Phe Asn Leu Ile Thr Glu
           435                 440                 445
   Tyr His Arg Glu Glu Glu Lys Glu Arg Gln Gln Ala Pro Gly Gly Asp
       450                 455                 460
   Pro Asp Asn Ser Val Arg Gly Lys Gly Met Asp Cys Ala Thr Leu Thr
   465                 470                 475                 480
   Cys Met Val Gln Leu Ala Gln Ile Leu Val Gly Gly Gly Leu Gly Phe
                   485                 490                 495
   Leu Val Asn Thr Ala Gly Thr Val Val Val Val Val Ile Thr Ala Ser
               500                 505                 510
   Ala Val Ala Leu Ile Gly Cys Cys Phe Val Ala Leu Phe Val Arg Tyr
           515                 520                 525
   Val Asp
       530

<210> 363
<211> 145
<212> PRT
<213> Homo sapiens

<400> 363
Met Glu Leu Ala Leu Leu Cys Gly Leu Val Val Met Ala Gly Val Ile
 1               5                  10                  15
Pro Ile Gln Gly Gly Ile Leu Asn Leu Asn Lys Met Val Lys Gln Val
            20                  25                  30
Thr Gly Lys Met Pro Ile Leu Ser Tyr Trp Pro Tyr Gly Cys His Cys
        35                  40                  45
Gly Leu Gly Gly Arg Gly Gln Pro Lys Asp Ala Thr Asp Trp Cys Cys
    50                  55                  60

Gln Thr His Asp Cys Cys Tyr Asp His Leu Lys Thr Gln Gly Cys Ser
65                  70                  75                  80
Ile Tyr Lys Asp Tyr Tyr Arg Tyr Asn Phe Ser Gln Gly Asn Ile His
                85                  90                  95
Cys Ser Asp Lys Gly Ser Trp Cys Glu Gln Gln Leu Cys Ala Cys Asp
            100                 105                 110
Lys Glu Val Ala Phe Cys Leu Lys Arg Asn Leu Asp Thr Tyr Gln Lys
        115                 120                 125
Arg Leu Arg Phe Tyr Trp Arg Pro His Cys Arg Gly Gln Thr Pro Gly
    130                 135                 140
Cys
145

<210> 364
<211> 630
<212> PRT
<213> Homo sapiens

<400> 364
Met Arg Leu Pro Asp Leu Arg Pro Trp Thr Ser Leu Leu Leu Val Asp
1               5                   10                  15
Ala Ala Leu Leu Trp Leu Leu Gln Gly Pro Leu Gly Thr Leu Leu Pro
            20                  25                  30
Gln Gly Leu Pro Gly Leu Trp Leu Glu Gly Thr Leu Arg Leu Gly Gly
        35                  40                  45
Leu Trp Gly Leu Leu Lys Leu Arg Gly Leu Leu Gly Phe Val Gly Thr
    50                  55                  60
Leu Leu Leu Pro Leu Cys Leu Ala Thr Pro Leu Thr Val Ser Leu Arg
65                  70                  75                  80
Ala Leu Val Ala Gly Ala Ser Arg Ala Pro Pro Ala Arg Val Ala Ser
                85                  90                  95
Ala Pro Trp Ser Trp Leu Leu Val Gly Tyr Gly Ala Ala Gly Leu Ser
            100                 105                 110
Trp Ser Leu Trp Ala Val Leu Ser Pro Pro Gly Ala Gln Glu Lys Glu
        115                 120                 125
Gln Asp Gln Val Asn Asn Lys Val Leu Met Trp Arg Leu Leu Lys Leu
    130                 135                 140
Ser Arg Pro Asp Leu Pro Leu Leu Val Ala Ala Phe Phe Phe Leu Val
145                 150                 155                 160
Leu Ala Val Leu Gly Glu Thr Leu Ile Pro His Tyr Ser Gly Arg Val
                165                 170                 175
Ile Asp Ile Leu Gly Gly Asp Phe Asp Pro His Ala Phe Ala Ser Ala
            180                 185                 190
Ile Phe Phe Met Cys Leu Phe Ser Phe Gly Ser Ser Leu Ser Ala Gly
        195                 200                 205
Cys Arg Gly Gly Cys Phe Thr Tyr Thr Met Ser Arg Ile Asn Leu Arg
    210                 215                 220
Ile Arg Glu Gln Leu Phe Ser Ser Leu Leu Arg Gln Asp Leu Gly Phe
225                 230                 235                 240
Phe Gln Glu Thr Lys Thr Gly Glu Leu Asn Ser Arg Leu Ser Ser Asp
                245                 250                 255
Thr Thr Leu Met Ser Asn Trp Leu Pro Leu Asn Ala Asn Val Leu Leu
            260                 265                 270
Arg Ser Leu Val Lys Val Val Gly Leu Tyr Gly Phe Met Leu Ser Ile
        275                 280                 285
Ser Pro Arg Leu Thr Leu Leu Ser Leu Leu His Met Pro Phe Thr Ile
    290                 295                 300
Ala Ala Glu Lys Val Tyr Asn Thr Arg His Gln Glu Val Leu Arg Glu
305                 310                 315                 320
Ile Gln Asp Ala Val Ala Arg Ala Gly Gln Val Val Arg Glu Ala Val
                325                 330                 335
Gly Gly Leu Gln Thr Val Arg Ser Phe Gly Ala Glu Glu His Glu Val

            340                 345                 350
Cys Arg Tyr Lys Glu Ala Leu Glu Gln Cys Arg Gln Leu Tyr Trp Arg
        355                 360                 365
Arg Asp Leu Glu Arg Ala Leu Tyr Leu Leu Ile Arg Arg Val Leu His
    370                 375                 380
Leu Gly Val Gln Met Leu Met Leu Ser Cys Gly Leu Gln Gln Met Gln
385                 390                 395                 400
Asp Gly Glu Leu Thr Gln Gly Ser Leu Leu Ser Phe Met Ile Tyr Gln
                405                 410                 415
Glu Ser Val Gly Ser Tyr Val Gln Thr Leu Val Tyr Ile Tyr Gly Asp
            420                 425                 430
Met Leu Ser Asn Val Gly Ala Ala Glu Lys Val Phe Ser Tyr Met Asp
        435                 440                 445
Arg Gln Pro Asn Leu Pro Ser Pro Gly Thr Leu Ala Pro Thr Thr Leu
    450                 455                 460
Gln Gly Val Val Lys Phe Gln Asp Val Ser Phe Ala Tyr Pro Asn Arg
465                 470                 475                 480
Pro Asp Arg Pro Val Leu Lys Gly Leu Thr Phe Thr Leu Arg Pro Gly
                485                 490                 495
Glu Val Thr Ala Leu Val Gly Pro Asn Gly Ser Gly Lys Ser Thr Val
            500                 505                 510
Ala Ala Leu Leu Gln Asn Leu Tyr Gln Pro Thr Gly Gly Gln Val Leu
        515                 520                 525
Leu Asp Glu Lys Pro Ile Ser Gln Tyr Glu His Cys Tyr Leu His Ser
    530                 535                 540
Gln Val Val Ser Val Gly Gln Glu Pro Val Leu Phe Ser Gly Ser Val
545                 550                 555                 560
Arg Asn Asn Ile Ala Tyr Gly Leu Gln Ser Cys Glu Asp Asp Lys Val
                565                 570                 575
Met Ala Ala Ala Gln Ala Ala His Ala Asp Asp Phe Ile Gln Glu Met
            580                 585                 590
Glu His Gly Ile Tyr Thr Asp Val Gly Glu Lys Gly Ser Gln Leu Ala
        595                 600                 605
Ala Gly Gln Lys Gln Arg Leu Ala Ile Ala Arg Ala Leu Val Arg Asp
    610                 615                 620
Pro Arg Val Leu Ile Leu
625                 630

<210> 365
<211> 125
<212> PRT
<213> Homo sapiens

<400> 365
Met Lys Lys Ser Gly Val Leu Phe Leu Leu Gly Ile Ile Leu Leu Val
1               5                   10                  15
Leu Ile Gly Val Gln Gly Thr Pro Val Val Arg Lys Gly Arg Cys Ser
            20                  25                  30
Cys Ile Ser Thr Asn Gln Gly Thr Ile His Leu Gln Ser Leu Lys Asp
        35                  40                  45
Leu Lys Gln Phe Ala Pro Ser Pro Ser Cys Glu Lys Ile Glu Ile Ile
    50                  55                  60
Ala Thr Leu Lys Asn Gly Val Gln Thr Cys Leu Asn Pro Asp Ser Ala
65                  70                  75                  80
Asp Val Lys Glu Leu Ile Lys Lys Trp Glu Lys Gln Val Ser Gln Lys
                85                  90                  95
Lys Lys Gln Lys Asn Gly Lys Lys His Gln Lys Lys Lys Val Leu Lys
            100                 105                 110
Val Arg Lys Ser Gln Arg Ser Arg Gln Lys Lys Thr Thr
        115                 120                 125

<210> 366
<211> 131

<212> PRT
<213> Homo sapiens

<400> 366
Met Ala Arg Ser Leu Val Cys Leu Gly Val Ile Ile Leu Leu Ser Ala
1               5                   10                  15
Phe Ser Gly Pro Gly Val Arg Gly Gly Pro Met Pro Lys Leu Ala Asp
            20                  25                  30
Arg Lys Leu Cys Ala Asp Gln Glu Cys Ser His Pro Ile Ser Met Ala
        35                  40                  45
Val Ala Leu Gln Asp Tyr Met Ala Pro Asp Cys Arg Phe Leu Thr Ile
    50                  55                  60
His Arg Gly Gln Val Val Tyr Val Phe Ser Lys Leu Lys Gly Arg Gly
65                  70                  75                  80

Arg Leu Phe Trp Gly Gly Ser Val Gln Gly Asp Tyr Tyr Gly Asp Leu
                85                  90                  95
Ala Ala Arg Leu Gly Tyr Phe Pro Ser Ser Ile Val Arg Glu Asp Gln
            100                 105                 110
Thr Leu Lys Pro Gly Lys Val Asp Val Lys Thr Asp Lys Trp Asp Phe
        115                 120                 125
Tyr Cys Gln
    130

<210> 367
<211> 7941
<212> DNA
<213> Homo sapiens

<400> 367
cacacatacg cacgcacgat ctcacttcga tctatacact ggaggattaa aacaaacaaa      60
caaaaaaaac atttccttcg ctccccctcc ctctccactc tgagaagcag aggagccgca      120
cggcgagggg ccgcagaccg tctggaaatg cgaatcctaa agcgtttcct cgcttgcatt      180
cagctcctct gtgtttgccg cctggattgg gctaatggat actacagaca acagagaaaa      240
cttgttgaag agattggctg gtcctataca ggagcactga atcaaaaaaa ttggggaaag      300
aaatatccaa catgtaatag cccaaaacaa tctcctatca atattgatga agatcttaca      360
caagtaaatg tgaatcttaa gaaacttaaa tttcagggtt gggataaaac atcattggaa      420
aacacattca ttcataacac tgggaaaaca gtggaaatta atctcactaa tgactaccgt      480
gtcagcggag gagtttcaga aatggtgttt aaagcaagca agataacttt tcactgggga      540
aaatgcaata tgtcatctga tggatcagag catagtttag aaggacaaaa atttccactt      600
gagatgcaaa tctactgctt tgatgcggac cgattttcaa gttttgagga agcagtcaaa      660
ggaaaaggga agttaagagc tttatccatt ttgtttgagg ttgggacaga agaaaatttg      720
gatttcaaag cgattattga tggagtcgaa agtgttagtc gttttgggaa gcaggctgct      780
ttagatccat tcatactgtt gaaccttctg ccaaactcaa ctgacaagta ttacatttac      840
aatggctcat tgacatctcc tccctgcaca gacacagttg actggattgt ttttaaagat      900
acagttagca tctctgaaag ccagttggct gttttttgtg aagttcttac aatgcaacaa      960
tctggttatg tcatgctgat ggactactta caaaacaatt ttcgagagca acagtacaag     1020
ttctctagac aggtgttttc ctcatacact ggaaaggaag agattcatga agcagtttgt     1080
agttcagaac cagaaaatgt tcaggctgac ccagagaatt ataccagcct tcttgttaca     1140
tgggaaagac ctcgagtcgt ttatgatacc atgattgaga agtttgcagt tttgtaccag     1200
cagttggatg gagaggacca aaccaagcat gaatttttga cagatggcta tcaagacttg     1260
ggtgctattc tcaataattt gctacccaat atgagttatg ttcttcagat agtagccata     1320
tgcactaatg gcttatatgg aaaatacagc gaccaactga ttgtcgacat gcctactgat     1380
aatcctgaac ttgatctttt ccctgaatta attggaactg aagaaataat caaggaggag     1440
gaagagggaa aagacattga agaaggcgct attgtgaatc ctggtagaga cagtgctaca     1500
aaccaaatca ggaaaaagga accccagatt tctaccacaa cacactacaa tcgcataggg     1560
acgaaataca atgaagccaa gactaaccga tccccaacaa gaggaagtga attctctgga     1620
aagggtgatg ttcccaatac atctttaaat tccacttccc aaccagtcac taaattagcc     1680
acagaaaaag atatttcctt gacttctcag actgtgactg aactgccacc tcacactgtg     1740
gaaggtactt cagcctcttt aaatgatggc tctaaaactg ttcttagatc tccacatatg     1800
aacttgtcgg ggactgcaga atccttaaat acagtttcta taacagaata tgaggaggag     1860
agtttattga ccagtttcaa gcttgatact ggagctgaag attcttcagg ctccagtccc     1920
gcaacttctg ctatcccatt catctctgag aacatatccc aagggtatat attttcctcc     1980

gaaaacccag agacaataac atatgatgtc cttataccag aatctgctag aaatgcttcc 2040
gaagattcaa cttcatcagg ttcagaagaa tcactaaagg atccttctat ggagggaaat 2100
gtgtggtttc ctagctctac agacataaca gcacagcccg atgttggatc aggcagagag 2160
agctttctcc agactaatta cactgagata cgtgttgatg aatctgagaa gacaaccaag 2220
tccttttctg caggcccagt gatgtcacag ggtccctcag ttacagatct ggaaatgcca 2280
cattattcta cctttgccta cttcccaact gaggtaacac ctcatgcttt taccccatcc 2340
tccagacaac aggatttggt ctccacggtc aacgtggtat actcgcagac aacccaaccg 2400
gtatacaatg gtgagacacc tcttcaacct tcctacagta gtgaagtctt tcctctagtc 2460
acccctttgt tgcttgacaa tcagatcctc aacactaccc ctgctgcttc aagtagtgat 2520
tcggccttgc atgctacgcc tgtatttccc agtgtcgatg tgtcatttga atccatcctg 2580
tcttcctatg atggtgcacc tttgcttcca ttttcctctg cttccttcag tagtgaattg 2640
tttcgccatc tgcatacagt ttctcaaatc cttccacaag ttacttcagc taccgagagt 2700
gataaggtgc ccttgcatgc ttctctgcca gtggctgggg gtgatttgct attagagccc 2760
agccttgctc agtattctga tgtgctgtcc actactcatg ctgcttcaga gacgctggaa 2820
tttggtagtg aatctggtgt tctttataaa acgcttatgt tttctcaagt tgaaccaccc 2880
agcagtgatg ccatgatgca tgcacgttct tcagggcctg aaccttctta tgccttgtct 2940
gataatgagg gctcccaaca catcttcact gtttcttaca gttctgcaat acctgtgcat 3000
gattctgtgg gtgtaactta tcagggttcc ttatttagcg gccctagcca tataccaata 3060
cctaagtctt cgttaataac cccaactgca tcattactgc agcctactca tgccctctct 3120
ggtgatgggg aatggtctgg agcctcttct gatagtgaat ttcttttacc tgacacagat 3180
gggctgacag cccttaacat ttcttcacct gtttctgtag ctgaatttac atatacaaca 3240
tctgtgtttg gtgatgataa taaggcgctt tctaaaagtg aaataatata tggaaatgag 3300
actgaactgc aaattccttc tttcaatgag atggtttacc cttctgaaag cacagtcatg 3360
cccaacatgt atgataatgt aaataagttg aatgcgtctt tacaagaaac ctctgtttcc 3420
atttctagca ccaagggcat gtttccaggg tcccttgctc ataccaccac taaggttttt 3480
gatcatgaga ttagtcaagt tccagaaaat aacttttcag ttcaacctac acatactgtc 3540
tctcaagcat ctggtgacac ttcgcttaaa cctgtgctta gtgcaaactc agagccagca 3600
tcctctgacc ctgcttctag tgaaatgtta tctccttcaa ctcagctctt attttatgag 3660
acctcagctt cttttagtac tgaagtattg ctacaacctt cctttcaggc ttctgatgtt 3720
gacaccttgc ttaaaactgt tcttccagct gtgcccagtg atccaatatt ggttgaaacc 3780
cccaaagttg ataaaattag ttctacaatg ttgcatctca ttgtatcaaa ttctgcttca 3840
agtgaaaaca tgctgcactc tacatctgta ccagtttttg atgtgtcgcc tacttctcat 3900
atgcactctg cttcacttca aggtttgacc atttcctatg caagtgagaa atatgaacca 3960
gttttgttaa aaagtgaaag ttcccaccaa gtggtacctt ctttgtacag taatgatgag 4020
ttgttccaaa cggccaattt ggagattaac caggcccatc ccccaaaagg aaggcatgta 4080
tttgctacac ctgttttatc aattgatgaa ccattaaata cactaataaa taagcttata 4140
cattccgatg aaattttaac ctccaccaaa agttctgtta ctggtaaggt atttgctggt 4200
attccaacag ttgcttctga tacatttgta tctactgatc attctgttcc tataggaaat 4260
gggcatgttg ccattacagc tgtttctccc cacagagatg gttctgtaac ctcaacaaag 4320
ttgctgtttc cttctaaggc aacttctgag ctgagtcata gtgccaaatc tgatgccggt 4380
ttagtgggtg gtggtgaaga tggtgacact gatgatgatg gtgatgatga tgatgacaga 4440
gatagtgatg gcttatccat tcataagtgt atgtcatgct catcctatag agaatcacag 4500
gaaaaggtaa tgaatgattc agacacccac gaaaacagtc ttatggatca gaataatcca 4560
atctcatact cactatctga gaattctgaa gaagataata gagtcacaag tgtatcctca 4620
gacagtcaaa ctggtatgga cagaagtcct ggtaaatcac catcagcaaa tgggctatcc 4680
caaaagcaca atgatggaaa agaggaaaat gacattcaga ctggtagtgc tctgcttcct 4740
ctcagccctg aatctaaagc atgggcagtt ctgacaagtg atgaagaaag tggatcaggg 4800
caaggtacct cagatagcct taatgagaat gagacttcca cagatttcag ttttgcagac 4860
actaatgaaa aagatgctga tgggatcctg gcagcaggtg actcagaaat aactcctgga 4920
ttcccacagt ccccaacatc atctgttact agcgagaact cagaagtgtt ccacgtttca 4980
gaggcagagg ccagtaatag tagccatgag tctcgtattg gtctagctga ggggttggaa 5040
tccgagaaga aggcagttat accccttgtg atcgtgtcag ccctgacttt tatctgtcta 5100
gtggttcttg tgggtattct catctactgg aggaaatgct tccagactgc acacttttac 5160
ttagaggaca gtacatcccc tagagttata tccacacctc caacacctat ctttccaatt 5220
tcagatgatg tcggagcaat tccaataaag cactttccaa agcatgttgc agatttacat 5280
gcaagtagtg ggtttactga agaatttgag acactgaaag agttttacca ggaagtgcag 5340
agctgtactg ttgacttagg tattacagca gacagctcca accacccaga caacaagcac 5400
aagaatcgat acataaatat cgttgcctat gatcatagca gggttaagct agcacagctt 5460
gctgaaaagg atggcaaact gactgattat atcaatgcca attatgttga tggctacaac 5520
agaccaaaag cttatattgc tgcccaaggc ccactgaaat ccacagctga agatttctgg 5580
agaatgatat gggaacataa tgtggaagtt attgtcatga taacaaacct cgtggagaaa 5640
ggaaggagaa aatgtgatca gtactggcct gccgatggga gtgaggagta cgggaacttt 5700
ctggtcactc agaagagtgt gcaagtgctt gcctattata ctgtgaggaa ttttactcta 5760

agaaacacaa aaataaaaaa gggctcccag aaaggaagac ccagtggacg tgtggtcaca 5820
cagtatcact acacgcagtg gcctgacatg ggagtaccag agtactccct gccagtgctg 5880
acctttgtga gaaaggcagc ctatgccaag cgccatgcag tggggcctgt tgtcgtccac 5940
tgcagtgctg gagttggaag aacaggcaca tatattgtgc tagacagtat gttgcagcag 6000
attcaacacg aaggaactgt caacatattt ggcttcttaa aacacatccg ttcacaaaga 6060
aattatttgg tacaaactga ggagcaatat gtcttcattc atgatacact ggttgaggcc 6120
atacttagta aagaaactga ggtgctggac agtcatattc atgcctatgt taatgcactc 6180
ctcattcctg gaccagcagg caaaacaaag ctagagaaac aattccagct cctgagccag 6240
tcaaatatac agcagagtga ctattctgca gccctaaagc aatgcaacag ggaaaagaat 6300
cgaacttctt ctatcatccc tgtggaaaga tcaagggttg gcatttcatc cctgagtgga 6360
gaaggcacag actacatcaa tgcctcctat atcatgggct attaccagag caatgaattc 6420
atcattaccc agcaccctct ccttcatacc atcaaggatt tctggaggat gatatgggac 6480
cataatgccc aactggtggt tatgattcct gatggccaaa acatggcaga agatgaattt 6540
gtttactggc caaataaaga tgagcctata aattgtgaga gctttaaggt cactcttatg 6600
gctgaagaac acaaatgtct atctaatgag gaaaaactta taattcagga ctttatctta 6660
gaagctacac aggatgatta tgtacttgaa gtgaggcact ttcagtgtcc taaatggcca 6720
aatccagata gccccattag taaaactttt gaacttataa gtgttataaa agaagaagct 6780
gccaataggg atgggcctat gattgttcat gatgagcatg gaggagtgac ggcaggaact 6840
ttctgtgctc tgacaaccct tatgcaccaa ctagaaaaag aaaattccgt ggatgtttac 6900
caggtagcca agatgatcaa tctgatgagg ccaggagtct ttgctgacat tgagcagtat 6960
cagtttctct acaaagtgat cctcagcctt gtgagcacaa ggcaggaaga gaatccatcc 7020
acctctctgg acagtaatgg tgcagcattg cctgatggaa atatagctga gagcttagag 7080
tctttagttt aacacagaaa ggggtggggg gactcacatc tgagcattgt tttcctcttc 7140
ctaaaattag gcaggaaaat cagtctagtt ctgttatctg ttgatttccc atcacctgac 7200
agtaactttc atgacatagg attctgccgc caaatttata tcattaacaa tgtgtgcctt 7260
tttgcaagac ttgtaattta cttattatgt ttgaactaaa atgattgaat tttacagtat 7320
ttctaagaat ggaattgtgg tatttttttc tgtattgatt ttaacagaaa atttcaattt 7380
atagaggtta ggaattccaa actacagaaa atgtttgttt ttagtgtcaa atttttagct 7440
gtatttgtag caattatcag gtttgctaga aatataactt ttaatacagt agcctgtaaa 7500
taaaacactc ttccatatga tattcaacat tttacaactg cagtattcac ctaaagtaga 7560
aataatctgt tacttattgt aaatactgcc ctagtgtctc catggaccaa atttatattt 7620
ataattgtag atttttatat tttactactg agtcaagttt tctagttctg tgtaattgtt 7680
tagtttaatg acgtagttca ttagctggtc ttactctacc agttttctga cattgtattg 7740
tgttacctaa gtcattaact ttgtttcagc atgtaatttt aacttttgtg gaaaatagaa 7800
ataccttcat tttgaaagaa gtttttatga gaataacacc ttaccaaaca ttgttcaaat 7860
ggtttttatc caaggaattg caaaaataaa tataaatatt gccattaaaa aaaaaaaaaa 7920
aaaaaaaaaa aaaaaaaaaa a 7941

<210> 368
<211> 2314
<212> PRT
<213> Homo sapiens

<400> 368
Met Arg Ile Leu Lys Arg Phe Leu Ala Cys Ile Gln Leu Leu Cys Val
1               5                   10                  15
Cys Arg Leu Asp Trp Ala Asn Gly Tyr Tyr Arg Gln Gln Arg Lys Leu
            20                  25                  30
Val Glu Glu Ile Gly Trp Ser Tyr Thr Gly Ala Leu Asn Gln Lys Asn
        35                  40                  45
Trp Gly Lys Lys Tyr Pro Thr Cys Asn Ser Pro Lys Gln Ser Pro Ile
    50                  55                  60
Asn Ile Asp Glu Asp Leu Thr Gln Val Asn Val Asn Leu Lys Lys Leu
65                  70                  75                  80
Lys Phe Gln Gly Trp Asp Lys Thr Ser Leu Glu Asn Thr Phe Ile His
                85                  90                  95
Asn Thr Gly Lys Thr Val Glu Ile Asn Leu Thr Asn Asp Tyr Arg Val
            100                 105                 110
Ser Gly Gly Val Ser Glu Met Val Phe Lys Ala Ser Lys Ile Thr Phe
        115                 120                 125
His Trp Gly Lys Cys Asn Met Ser Ser Asp Gly Ser Glu His Ser Leu
    130                 135                 140
Glu Gly Gln Lys Phe Pro Leu Glu Met Gln Ile Tyr Cys Phe Asp Ala

145                 150                 155                 160
Asp Arg Phe Ser Ser Phe Glu Glu Ala Val Lys Gly Lys Gly Lys Leu
                165                 170                 175
Arg Ala Leu Ser Ile Leu Phe Glu Val Gly Thr Glu Glu Asn Leu Asp
            180                 185                 190
Phe Lys Ala Ile Ile Asp Gly Val Glu Ser Val Ser Arg Phe Gly Lys
        195                 200                 205
Gln Ala Ala Leu Asp Pro Phe Ile Leu Leu Asn Leu Leu Pro Asn Ser
    210                 215                 220
Thr Asp Lys Tyr Tyr Ile Tyr Asn Gly Ser Leu Thr Ser Pro Pro Cys
225                 230                 235                 240
Thr Asp Thr Val Asp Trp Ile Val Phe Lys Asp Thr Val Ser Ile Ser
                245                 250                 255
Glu Ser Gln Leu Ala Val Phe Cys Glu Val Leu Thr Met Gln Gln Ser
            260                 265                 270
Gly Tyr Val Met Leu Met Asp Tyr Leu Gln Asn Asn Phe Arg Glu Gln
        275                 280                 285
Gln Tyr Lys Phe Ser Arg Gln Val Phe Ser Ser Tyr Thr Gly Lys Glu
    290                 295                 300
Glu Ile His Glu Ala Val Cys Ser Ser Glu Pro Glu Asn Val Gln Ala
305                 310                 315                 320
Asp Pro Glu Asn Tyr Thr Ser Leu Leu Val Thr Trp Glu Arg Pro Arg
                325                 330                 335
Val Val Tyr Asp Thr Met Ile Glu Lys Phe Ala Val Leu Tyr Gln Gln
            340                 345                 350
Leu Asp Gly Glu Asp Gln Thr Lys His Glu Phe Leu Thr Asp Gly Tyr
        355                 360                 365
Gln Asp Leu Gly Ala Ile Leu Asn Asn Leu Leu Pro Asn Met Ser Tyr
    370                 375                 380
Val Leu Gln Ile Val Ala Ile Cys Thr Asn Gly Leu Tyr Gly Lys Tyr
385                 390                 395                 400
Ser Asp Gln Leu Ile Val Asp Met Pro Thr Asp Asn Pro Glu Leu Asp
                405                 410                 415
Leu Phe Pro Glu Leu Ile Gly Thr Glu Glu Ile Ile Lys Glu Glu Glu
            420                 425                 430
Glu Gly Lys Asp Ile Glu Glu Gly Ala Ile Val Asn Pro Gly Arg Asp
        435                 440                 445
Ser Ala Thr Asn Gln Ile Arg Lys Lys Glu Pro Gln Ile Ser Thr Thr
    450                 455                 460
Thr His Tyr Asn Arg Ile Gly Thr Lys Tyr Asn Glu Ala Lys Thr Asn
465                 470                 475                 480
Arg Ser Pro Thr Arg Gly Ser Glu Phe Ser Gly Lys Gly Asp Val Pro
                485                 490                 495
Asn Thr Ser Leu Asn Ser Thr Ser Gln Pro Val Thr Lys Leu Ala Thr
            500                 505                 510
Glu Lys Asp Ile Ser Leu Thr Ser Gln Thr Val Thr Glu Leu Pro Pro
        515                 520                 525
His Thr Val Glu Gly Thr Ser Ala Ser Leu Asn Asp Gly Ser Lys Thr
    530                 535                 540
Val Leu Arg Ser Pro His Met Asn Leu Ser Gly Thr Ala Glu Ser Leu
545                 550                 555                 560
Asn Thr Val Ser Ile Thr Glu Tyr Glu Glu Glu Ser Leu Leu Thr Ser
                565                 570                 575
Phe Lys Leu Asp Thr Gly Ala Glu Asp Ser Ser Gly Ser Ser Pro Ala
            580                 585                 590
Thr Ser Ala Ile Pro Phe Ile Ser Glu Asn Ile Ser Gln Gly Tyr Ile
        595                 600                 605
Phe Ser Ser Glu Asn Pro Glu Thr Ile Thr Tyr Asp Val Leu Ile Pro
    610                 615                 620
Glu Ser Ala Arg Asn Ala Ser Glu Asp Ser Thr Ser Ser Gly Ser Glu
625                 630                 635                 640
Glu Ser Leu Lys Asp Pro Ser Met Glu Gly Asn Val Trp Phe Pro Ser
                645                 650                 655

Ser Thr Asp Ile Thr Ala Gln Pro Asp Val Gly Ser Gly Arg Glu Ser
            660                 665                 670
Phe Leu Gln Thr Asn Tyr Thr Glu Ile Arg Val Asp Glu Ser Glu Lys
        675                 680                 685
Thr Thr Lys Ser Phe Ser Ala Gly Pro Val Met Ser Gln Gly Pro Ser
    690                 695                 700

Val Thr Asp Leu Glu Met Pro His Tyr Ser Thr Phe Ala Tyr Phe Pro
705                 710                 715                 720
Thr Glu Val Thr Pro His Ala Phe Thr Pro Ser Ser Arg Gln Gln Asp
                725                 730                 735
Leu Val Ser Thr Val Asn Val Val Tyr Ser Gln Thr Thr Gln Pro Val
            740                 745                 750
Tyr Asn Gly Glu Thr Pro Leu Gln Pro Ser Tyr Ser Ser Glu Val Phe
        755                 760                 765
Pro Leu Val Thr Pro Leu Leu Leu Asp Asn Gln Ile Leu Asn Thr Thr
    770                 775                 780
Pro Ala Ala Ser Ser Ser Asp Ser Ala Leu His Ala Thr Pro Val Phe
785                 790                 795                 800
Pro Ser Val Asp Val Ser Phe Glu Ser Ile Leu Ser Ser Tyr Asp Gly
                805                 810                 815
Ala Pro Leu Leu Pro Phe Ser Ser Ala Ser Phe Ser Ser Glu Leu Phe
            820                 825                 830
Arg His Leu His Thr Val Ser Gln Ile Leu Pro Gln Val Thr Ser Ala
        835                 840                 845
Thr Glu Ser Asp Lys Val Pro Leu His Ala Ser Leu Pro Val Ala Gly
    850                 855                 860
Gly Asp Leu Leu Leu Glu Pro Ser Leu Ala Gln Tyr Ser Asp Val Leu
865                 870                 875                 880
Ser Thr Thr His Ala Ala Ser Glu Thr Leu Glu Phe Gly Ser Glu Ser
                885                 890                 895
Gly Val Leu Tyr Lys Thr Leu Met Phe Ser Gln Val Glu Pro Pro Ser
            900                 905                 910
Ser Asp Ala Met Met His Ala Arg Ser Ser Gly Pro Glu Pro Ser Tyr
        915                 920                 925
Ala Leu Ser Asp Asn Glu Gly Ser Gln His Ile Phe Thr Val Ser Tyr
    930                 935                 940
Ser Ser Ala Ile Pro Val His Asp Ser Val Gly Val Thr Tyr Gln Gly
945                 950                 955                 960
Ser Leu Phe Ser Gly Pro Ser His Ile Pro Ile Pro Lys Ser Ser Leu
                965                 970                 975
Ile Thr Pro Thr Ala Ser Leu Leu Gln Pro Thr His Ala Leu Ser Gly
            980                 985                 990
Asp Gly Glu Trp Ser Gly Ala Ser Ser Asp Ser Glu Phe Leu Leu Pro
        995                 1000                1005
Asp Thr Asp Gly Leu Thr Ala Leu Asn Ile Ser Ser Pro Val Ser Val
    1010                1015                1020
Ala Glu Phe Thr Tyr Thr Thr Ser Val Phe Gly Asp Asp Asn Lys Ala
1025                1030                1035                1040
Leu Ser Lys Ser Glu Ile Ile Tyr Gly Asn Glu Thr Glu Leu Gln Ile
                1045                1050                1055
Pro Ser Phe Asn Glu Met Val Tyr Pro Ser Glu Ser Thr Val Met Pro
            1060                1065                1070
Asn Met Tyr Asp Asn Val Asn Lys Leu Asn Ala Ser Leu Gln Glu Thr
        1075                1080                1085
Ser Val Ser Ile Ser Ser Thr Lys Gly Met Phe Pro Gly Ser Leu Ala
    1090                1095                1100
His Thr Thr Thr Lys Val Phe Asp His Glu Ile Ser Gln Val Pro Glu
1105                1110                1115                1120
Asn Asn Phe Ser Val Gln Pro Thr His Thr Val Ser Gln Ala Ser Gly
                1125                1130                1135
Asp Thr Ser Leu Lys Pro Val Leu Ser Ala Asn Ser Glu Pro Ala Ser
            1140                1145                1150

Ser Asp Pro Ala Ser Ser Glu Met Leu Ser Pro Ser Thr Gln Leu Leu
        1155                1160                1165
Phe Tyr Glu Thr Ser Ala Ser Phe Ser Thr Glu Val Leu Leu Gln Pro
    1170                1175                1180

Ser Phe Gln Ala Ser Asp Val Asp Thr Leu Leu Lys Thr Val Leu Pro
1185                1190                1195                1200
Ala Val Pro Ser Asp Pro Ile Leu Val Glu Thr Pro Lys Val Asp Lys
                1205                1210                1215
Ile Ser Ser Thr Met Leu His Leu Ile Val Ser Asn Ser Ala Ser Ser
            1220                1225                1230
Glu Asn Met Leu His Ser Thr Ser Val Pro Val Phe Asp Val Ser Pro
        1235                1240                1245
Thr Ser His Met His Ser Ala Ser Leu Gln Gly Leu Thr Ile Ser Tyr
    1250                1255                1260
Ala Ser Glu Lys Tyr Glu Pro Val Leu Leu Lys Ser Glu Ser Ser His
1265                1270                1275                1280
Gln Val Val Pro Ser Leu Tyr Ser Asn Asp Glu Leu Phe Gln Thr Ala
                1285                1290                1295
Asn Leu Glu Ile Asn Gln Ala His Pro Pro Lys Gly Arg His Val Phe
            1300                1305                1310
Ala Thr Pro Val Leu Ser Ile Asp Glu Pro Leu Asn Thr Leu Ile Asn
        1315                1320                1325
Lys Leu Ile His Ser Asp Glu Ile Leu Thr Ser Thr Lys Ser Ser Val
    1330                1335                1340
Thr Gly Lys Val Phe Ala Gly Ile Pro Thr Val Ala Ser Asp Thr Phe
1345                1350                1355                1360
Val Ser Thr Asp His Ser Val Pro Ile Gly Asn Gly His Val Ala Ile
                1365                1370                1375
Thr Ala Val Ser Pro His Arg Asp Gly Ser Val Thr Ser Thr Lys Leu
            1380                1385                1390
Leu Phe Pro Ser Lys Ala Thr Ser Glu Leu Ser His Ser Ala Lys Ser
        1395                1400                1405
Asp Ala Gly Leu Val Gly Gly Gly Glu Asp Gly Asp Thr Asp Asp Asp
    1410                1415                1420
Gly Asp Asp Asp Asp Asp Arg Asp Ser Asp Gly Leu Ser Ile His Lys
1425                1430                1435                1440
Cys Met Ser Cys Ser Ser Tyr Arg Glu Ser Gln Glu Lys Val Met Asn
                1445                1450                1455
Asp Ser Asp Thr His Glu Asn Ser Leu Met Asp Gln Asn Asn Pro Ile
            1460                1465                1470
Ser Tyr Ser Leu Ser Glu Asn Ser Glu Glu Asp Asn Arg Val Thr Ser
        1475                1480                1485
Val Ser Ser Asp Ser Gln Thr Gly Met Asp Arg Ser Pro Gly Lys Ser
    1490                1495                1500
Pro Ser Ala Asn Gly Leu Ser Gln Lys His Asn Asp Gly Lys Glu Glu
1505                1510                1515                1520
Asn Asp Ile Gln Thr Gly Ser Ala Leu Leu Pro Leu Ser Pro Glu Ser
                1525                1530                1535
Lys Ala Trp Ala Val Leu Thr Ser Asp Glu Glu Ser Gly Ser Gly Gln
            1540                1545                1550
Gly Thr Ser Asp Ser Leu Asn Glu Asn Glu Thr Ser Thr Asp Phe Ser
        1555                1560                1565
Phe Ala Asp Thr Asn Glu Lys Asp Ala Asp Gly Ile Leu Ala Ala Gly
    1570                1575                1580
Asp Ser Glu Ile Thr Pro Gly Phe Pro Gln Ser Pro Thr Ser Ser Val
1585                1590                1595                1600
Thr Ser Glu Asn Ser Glu Val Phe His Val Ser Glu Ala Glu Ala Ser
                1605                1610                1615
Asn Ser Ser His Glu Ser Arg Ile Gly Leu Ala Glu Gly Leu Glu Ser
            1620                1625                1630
Glu Lys Lys Ala Val Ile Pro Leu Val Ile Val Ser Ala Leu Thr Phe
        1635                1640                1645

Ile Cys Leu Val Val Leu Val Gly Ile Leu Ile Tyr Trp Arg Lys Cys
    1650                1655                1660

Phe Gln Thr Ala His Phe Tyr Leu Glu Asp Ser Thr Ser Pro Arg Val
1665                1670                1675                1680
Ile Ser Thr Pro Pro Thr Pro Ile Phe Pro Ile Ser Asp Asp Val Gly
                1685                1690                1695
Ala Ile Pro Ile Lys His Phe Pro Lys His Val Ala Asp Leu His Ala
            1700                1705                1710
Ser Ser Gly Phe Thr Glu Glu Phe Glu Thr Leu Lys Glu Phe Tyr Gln
        1715                1720                1725
Glu Val Gln Ser Cys Thr Val Asp Leu Gly Ile Thr Ala Asp Ser Ser
    1730                1735                1740
Asn His Pro Asp Asn Lys His Lys Asn Arg Tyr Ile Asn Ile Val Ala
1745                1750                1755                1760
Tyr Asp His Ser Arg Val Lys Leu Ala Gln Leu Ala Glu Lys Asp Gly
                1765                1770                1775
Lys Leu Thr Asp Tyr Ile Asn Ala Asn Tyr Val Asp Gly Tyr Asn Arg
            1780                1785                1790
Pro Lys Ala Tyr Ile Ala Ala Gln Gly Pro Leu Lys Ser Thr Ala Glu
        1795                1800                1805
Asp Phe Trp Arg Met Ile Trp Glu His Asn Val Glu Val Ile Val Met
    1810                1815                1820
Ile Thr Asn Leu Val Glu Lys Gly Arg Arg Lys Cys Asp Gln Tyr Trp
1825                1830                1835                1840
Pro Ala Asp Gly Ser Glu Glu Tyr Gly Asn Phe Leu Val Thr Gln Lys
                1845                1850                1855
Ser Val Gln Val Leu Ala Tyr Tyr Thr Val Arg Asn Phe Thr Leu Arg
            1860                1865                1870
Asn Thr Lys Ile Lys Lys Gly Ser Gln Lys Gly Arg Pro Ser Gly Arg
        1875                1880                1885
Val Val Thr Gln Tyr His Tyr Thr Gln Trp Pro Asp Met Gly Val Pro
    1890                1895                1900
Glu Tyr Ser Leu Pro Val Leu Thr Phe Val Arg Lys Ala Ala Tyr Ala
1905                1910                1915                1920
Lys Arg His Ala Val Gly Pro Val Val Val His Cys Ser Ala Gly Val
                1925                1930                1935
Gly Arg Thr Gly Thr Tyr Ile Val Leu Asp Ser Met Leu Gln Gln Ile
            1940                1945                1950
Gln His Glu Gly Thr Val Asn Ile Phe Gly Phe Leu Lys His Ile Arg
        1955                1960                1965
Ser Gln Arg Asn Tyr Leu Val Gln Thr Glu Glu Gln Tyr Val Phe Ile
    1970                1975                1980
His Asp Thr Leu Val Glu Ala Ile Leu Ser Lys Glu Thr Glu Val Leu
1985                1990                1995                2000
Asp Ser His Ile His Ala Tyr Val Asn Ala Leu Leu Ile Pro Gly Pro
                2005                2010                2015
Ala Gly Lys Thr Lys Leu Glu Lys Gln Phe Gln Leu Leu Ser Gln Ser
            2020                2025                2030
Asn Ile Gln Gln Ser Asp Tyr Ser Ala Ala Leu Lys Gln Cys Asn Arg
        2035                2040                2045
Glu Lys Asn Arg Thr Ser Ser Ile Ile Pro Val Glu Arg Ser Arg Val
    2050                2055                2060
Gly Ile Ser Ser Leu Ser Gly Glu Gly Thr Asp Tyr Ile Asn Ala Ser
2065                2070                2075                2080
Tyr Ile Met Gly Tyr Tyr Gln Ser Asn Glu Phe Ile Ile Thr Gln His
                2085                2090                2095
Pro Leu Leu His Thr Ile Lys Asp Phe Trp Arg Met Ile Trp Asp His
            2100                2105                2110
Asn Ala Gln Leu Val Val Met Ile Pro Asp Gly Gln Asn Met Ala Glu
        2115                2120                2125
Asp Glu Phe Val Tyr Trp Pro Asn Lys Asp Glu Pro Ile Asn Cys Glu
    2130                2135                2140

Ser Phe Lys Val Thr Leu Met Ala Glu Glu His Lys Cys Leu Ser Asn
2145                2150                2155                2160
Glu Glu Lys Leu Ile Ile Gln Asp Phe Ile Leu Glu Ala Thr Gln Asp
                2165                2170                2175
Asp Tyr Val Leu Glu Val Arg His Phe Gln Cys Pro Lys Trp Pro Asn
            2180                2185                2190
Pro Asp Ser Pro Ile Ser Lys Thr Phe Glu Leu Ile Ser Val Ile Lys
        2195                2200                2205
Glu Glu Ala Ala Asn Arg Asp Gly Pro Met Ile Val His Asp Glu His
    2210                2215                2220
Gly Gly Val Thr Ala Gly Thr Phe Cys Ala Leu Thr Thr Leu Met His
2225                2230                2235                2240
Gln Leu Glu Lys Glu Asn Ser Val Asp Val Tyr Gln Val Ala Lys Met
                2245                2250                2255
Ile Asn Leu Met Arg Pro Gly Val Phe Ala Asp Ile Glu Gln Tyr Gln
            2260                2265                2270
Phe Leu Tyr Lys Val Ile Leu Ser Leu Val Ser Thr Arg Gln Glu Glu
        2275                2280                2285
Asn Pro Ser Thr Ser Leu Asp Ser Asn Gly Ala Ala Leu Pro Asp Gly
    2290                2295                2300
Asn Ile Ala Glu Ser Leu Glu Ser Leu Val
2305                2310

<210> 369
<211> 1768
<212> DNA
<213> Homo sapiens

<400> 369
gtagactgtc catggcctga acattttccg aaaatcattt tgagcaaaat atctgtttaa      60
taacaagata accacatcaa gatggttgga aagctgaagc agaacttact attggcatgt     120
ctggtgatta gttctgtgac tgtgttttac ctgggccagc atgccatgga atgccatcac     180
cggatagagg aacgtagcca gccagtcaaa ttggagagca caaggaccac tgtgagaact     240
ggcctggacc tcaaagccaa caaaaccttt gcctatcaca aagatatgcc tttaatattt     300
attggrggtg tgcctcggag tggaaccaca ctcatgaggg ccatgctgga cgcacatcct     360
gacattcgct gtggagagga aaccagggtc attccccgaa tcctggccct gaagcagatg     420
tggtcacggt caagtaaaga gaagatccgc ctggatgagg ctggtgttac tgatgaagtg     480
ctggattctg ccatgcaagc cttcttacta gaaattatcg ttaagcatgg ggagccagcc     540
ccttatttat gtaataaaga tccttttgcc ctgaaatctt taacttacct ttctaggtta     600
ttccccaatg ccaaatttct cctgatggtc cgagatggcc gggcatcagt acattcaatg     660
atttctcgaa aagttactat agctggattt gatctgaaca gctataggga ctgtttgaca     720
aagtggaatc gtgctataga gaccatgtat aaccagtgta tggaggttgg ttataaaaag     780
tgcatgttgg ttcactatga acaacttgtc ttacatcctg aacggtggat gagaacactc     840
ttaaagttcc tccagattcc atggaaccac tcagtattgc accatgaaga gatgattggg     900
aaagctgggg gagtgtctct gtcaaaagtg gagagatcta cagaccaagt aatcaagcca     960
gtcaatgtag gagctctatc aaaatgggtt gggaagatac cgccagatgt tttacaagac    1020
atggcagtga ttgctcctat gcttgccaag cttggatatg acccatatgc caacccacct    1080
aactacggaa aacctgatcc caaaattatt gaaaacactc gaagggtcta taagggagaa    1140
ttccaactac ctgactttct taaagaaaaa ccacagactg agcaagtgga gtagcagaac    1200
caggagcctc ttccatacat gaggaaagat tgctgccttt tcagcagaag ggaaattcct    1260
aggattggct gtcccctgcc aagcttggtg gagcgtctgc accttggctg cgccgcctgt    1320
gcatttgcca gtttcctccc actgagagga tggaggtgtc cgcacagctt tgggcctcgt    1380
gagggatctg cctcctgagc aaagagctct tgatcccgat ttcatgcaca gccctgcagt    1440
aaggagccca gaaggaacat gtgtttcctg ttaaaactcc tcttgttctc ttttcttaca    1500
ttatgacgtt tgttttcaag gagagggttt aaaaatggga tcctgtaagc agacttgggc    1560
agtctccttt tgaaataggt tgtctgtaca tgttctaatg ttttgtagaa cacgtgtgcc    1620
tgtttaagtg tattgatgtg aataatatta aatatcctaa ttatttaatt cattgtattg    1680
tttctgagaa gttgggaaat taccattata catttacaac ctaatgactt ttgtatttta    1740
tttttcaaaa taaaagcttt caatgtga                                       1768

<210> 370

<211> 370
<212> PRT
<213> Homo sapiens

<400> 370
Met Val Gly Lys Leu Lys Gln Asn Leu Leu Leu Ala Cys Leu Val Ile
1               5                   10                  15
Ser Ser Val Thr Val Phe Tyr Leu Gly Gln His Ala Met Glu Cys His
            20                  25                  30
His Arg Ile Glu Glu Arg Ser Gln Pro Val Lys Leu Glu Ser Thr Arg
        35                  40                  45
Thr Thr Val Arg Thr Gly Leu Asp Leu Lys Ala Asn Lys Thr Phe Ala
    50                  55                  60
Tyr His Lys Asp Met Pro Leu Ile Phe Ile Gly Gly Val Pro Arg Ser
65                  70                  75                  80
Gly Thr Thr Leu Met Arg Ala Met Leu Asp Ala His Pro Asp Ile Arg
                85                  90                  95
Cys Gly Glu Glu Thr Arg Val Ile Pro Arg Ile Leu Ala Leu Lys Gln
            100                 105                 110
Met Trp Ser Arg Ser Ser Lys Glu Lys Ile Arg Leu Asp Glu Ala Gly
        115                 120                 125
Val Thr Asp Glu Val Leu Asp Ser Ala Met Gln Ala Phe Leu Leu Glu
    130                 135                 140
Ile Ile Val Lys His Gly Glu Pro Ala Pro Tyr Leu Cys Asn Lys Asp
145                 150                 155                 160
Pro Phe Ala Leu Lys Ser Leu Thr Tyr Leu Ser Arg Leu Phe Pro Asn
                165                 170                 175
Ala Lys Phe Leu Leu Met Val Arg Asp Gly Arg Ala Ser Val His Ser
            180                 185                 190
Met Ile Ser Arg Lys Val Thr Ile Ala Gly Phe Asp Leu Asn Ser Tyr
        195                 200                 205
Arg Asp Cys Leu Thr Lys Trp Asn Arg Ala Ile Glu Thr Met Tyr Asn
    210                 215                 220
Gln Cys Met Glu Val Gly Tyr Lys Lys Cys Met Leu Val His Tyr Glu
225                 230                 235                 240
Gln Leu Val Leu His Pro Glu Arg Trp Met Arg Thr Leu Leu Lys Phe
                245                 250                 255
Leu Gln Ile Pro Trp Asn His Ser Val Leu His His Glu Glu Met Ile
            260                 265                 270
Gly Lys Ala Gly Gly Val Ser Leu Ser Lys Val Glu Arg Ser Thr Asp
        275                 280                 285
Gln Val Ile Lys Pro Val Asn Val Gly Ala Leu Ser Lys Trp Val Gly
    290                 295                 300
Lys Ile Pro Pro Asp Val Leu Gln Asp Met Ala Val Ile Ala Pro Met
305                 310                 315                 320
Leu Ala Lys Leu Gly Tyr Asp Pro Tyr Ala Asn Pro Pro Asn Tyr Gly
                325                 330                 335
Lys Pro Asp Pro Lys Ile Ile Glu Asn Thr Arg Arg Val Tyr Lys Gly
            340                 345                 350
Glu Phe Gln Leu Pro Asp Phe Leu Lys Glu Lys Pro Gln Thr Glu Gln
        355                 360                 365
Val Glu
    370

<210> 371
<211> 1382
<212> DNA
<213> Homo sapiens

<400> 371
cggatgaagg ctatttgaag tcgccataac ctggtcagaa gtgtgcctgt cggcggggag      60
agaggcaata tcaaggtttt aaatctcgga gaaatggctt tcgtttgctt ggctatcgga     120

tgcttatata cctttctgat aagcacaaca tttggctgta cttcatcttc agacaccgag 180
ataaaagtta accctcctca ggattttgag atagtggatc ccggatactt aggttatctc 240
tatttgcaat ggcaaccccc actgtctctg gatcatttta aggaatgcac agtggaatat 300
gaactaaaat accgaaacat tggtagtgaa acatggaaga ccatcattac taagaatcta 360
cattacaaag atgggtttga tcttaacaag ggcattgaag cgaagataca cacgctttta 420
ccatggcaat gcacaaatgg atcagaagtt caaagttcct gggcagaaac tacttattgg 480
atatcaccac aaggaattcc agaaactaaa gttcaggata tggattgcgt atattacaat 540
tggcaatatt tactctgttc ttggaaacct ggcataggtg tacttcttga taccaattac 600
aacttgtttt actggtatga gggcttggat catgcattac agtgtgttga ttacatcaag 660
gctgatggac aaaatatagg atgcagattt ccctatttgg aggcatcaga ctataaagat 720
ttctatattt gtgttaatgg atcatcagag aacaagccta tcagatccag ttatttcact 780
tttcagcttc aaaatatagt taaacctttg ccgccagtct atcttacttt tactcgggag 840
agttcatgtg aaattaagct gaaatggagc atacctttgg gacctattcc agcaaggtgt 900
tttgattatg aaattgagat cagagaagat gatactacct tggtgactgc tacagttgaa 960
aatgaaacat acaccttgaa aacaacaaat gaaacccgac aattatgctt tgtagtaaga 1020
agcaaagtga atatttattg ctcagatgac ggaatttgga gtgagtggag tgataaacaa 1080
tgctgggaag gtgaagacct atcgaagaaa actttgctac gtttctggct accatttggt 1140
ttcatcttaa tattagttat atttgtaacc ggtctgcttt tgcgtaagcc aaacacctac 1200
ccaaaaatga ttccagaatt tttctgtgat acatgaagac tttccatatc aagagacatg 1260
gtattgactc aacagtttcc agtcatggcc aaatgttcaa tatgagtctc aataaactga 1320
atttttcttg cgaaaaaaaa aaaaaaaaaa aaaaaaaaaa aaaaaaaaaa aaaaaaaaaa 1380
aa 1382

<210> 372
<211> 380
<212> PRT
<213> Homo sapiens

<400> 372
Met Ala Phe Val Cys Leu Ala Ile Gly Cys Leu Tyr Thr Phe Leu Ile
1               5                   10                  15
Ser Thr Thr Phe Gly Cys Thr Ser Ser Ser Asp Thr Glu Ile Lys Val
            20                  25                  30
Asn Pro Pro Gln Asp Phe Glu Ile Val Asp Pro Gly Tyr Leu Gly Tyr
        35                  40                  45
Leu Tyr Leu Gln Trp Gln Pro Pro Leu Ser Leu Asp His Phe Lys Glu
    50                  55                  60
Cys Thr Val Glu Tyr Glu Leu Lys Tyr Arg Asn Ile Gly Ser Glu Thr
65                  70                  75                  80
Trp Lys Thr Ile Ile Thr Lys Asn Leu His Tyr Lys Asp Gly Phe Asp
                85                  90                  95
Leu Asn Lys Gly Ile Glu Ala Lys Ile His Thr Leu Leu Pro Trp Gln
            100                 105                 110
Cys Thr Asn Gly Ser Glu Val Gln Ser Ser Trp Ala Glu Thr Thr Tyr
        115                 120                 125
Trp Ile Ser Pro Gln Gly Ile Pro Glu Thr Lys Val Gln Asp Met Asp
    130                 135                 140
Cys Val Tyr Tyr Asn Trp Gln Tyr Leu Leu Cys Ser Trp Lys Pro Gly
145                 150                 155                 160
Ile Gly Val Leu Leu Asp Thr Asn Tyr Asn Leu Phe Tyr Trp Tyr Glu
                165                 170                 175
Gly Leu Asp His Ala Leu Gln Cys Val Asp Tyr Ile Lys Ala Asp Gly
            180                 185                 190
Gln Asn Ile Gly Cys Arg Phe Pro Tyr Leu Glu Ala Ser Asp Tyr Lys
        195                 200                 205
Asp Phe Tyr Ile Cys Val Asn Gly Ser Ser Glu Asn Lys Pro Ile Arg
    210                 215                 220

Ser Ser Tyr Phe Thr Phe Gln Leu Gln Asn Ile Val Lys Pro Leu Pro
225                 230                 235                 240
Pro Val Tyr Leu Thr Phe Thr Arg Glu Ser Ser Cys Glu Ile Lys Leu
                245                 250                 255
Lys Trp Ser Ile Pro Leu Gly Pro Ile Pro Ala Arg Cys Phe Asp Tyr

            260                 265                 270
Glu Ile Glu Ile Arg Glu Asp Asp Thr Thr Leu Val Thr Ala Thr Val
        275                 280                 285
Glu Asn Glu Thr Tyr Thr Leu Lys Thr Thr Asn Glu Thr Arg Gln Leu
    290                 295                 300
Cys Phe Val Val Arg Ser Lys Val Asn Ile Tyr Cys Ser Asp Asp Gly
305                 310                 315                 320
Ile Trp Ser Glu Trp Ser Asp Lys Gln Cys Trp Glu Gly Glu Asp Leu
                325                 330                 335
Ser Lys Lys Thr Leu Leu Arg Phe Trp Leu Pro Phe Gly Phe Ile Leu
            340                 345                 350
Ile Leu Val Ile Phe Val Thr Gly Leu Leu Leu Arg Lys Pro Asn Thr
        355                 360                 365
Tyr Pro Lys Met Ile Pro Glu Phe Phe Cys Asp Thr
    370                 375                 380

<210> 373
<211> 1487
<212> DNA
<213> Homo sapiens

<400> 373
ggggcagatc cagattccct ttgcagtcca cgccaggcct tcaccatgga tcagttccct 60
gaatcagtga cagaaaactt tgagtacgat gatttggctg aggcctgtta tattggggac 120
atcgtggtct ttgggactgt gttcctgtcc atattctact ccgtcatctt tgccattggc 180
ctggtgggaa atttgttggt agtgtttgcc ctcaccaaca gcaagaagcc caagagtgtc 240
accgacattt acctcctgaa cctggccttg tctgatctgc tgtttgtagc cactttgccc 300
ttctggactc actatttgat aaatgaaaag ggcctccaca atgccatgtg caaattcact 360
accgccttct tcttcatcgg cttttttgga agcatattct tcatcaccgt catcagcatt 420
gataggtacc tggccatcgt cctggccgcc aactccatga acaaccggac cgtgcagcat 480
ggcgtcacca tcagcctagg cgtctgggca gcagccattt tggtggcagc accccagttc 540
atgttcacaa agcagaaaga aaatgaatgc cttggtgact accccgaggt ccttcaggaa 600
atctggcccg tgctccgcaa tgtggaaaca aattttcttg gcttcctact ccccctgctc 660
attatgagtt attgctactt cagaatcatc cagacgctgt tttcctgcaa gaaccacaag 720
aaagccaaag ccattaaact gatccttctg gtggtcatcg tgtttttcct cttctggaca 780
ccctacaacg ttatgatttt cctggagacg cttaagctct atgacttctt tcccagttgt 840
gacatgagga aggatctgag gctggccctc agtgtgactg agacggttgc atttagccat 900
tgttgcctga atcctctcat ctatgcattt gctggggaga agttcagaag atacctttac 960
cacctgtatg ggaaatgcct ggctgtcctg tgtgggcgct cagtccacgt tgatttctcc 1020
tcatctgaat cacaaaggag caggcatgga agtgttctga gcagcaattt tacttaccac 1080
acgagtgatg gagatgcatt gctccttctc tgaagggaat cccaaagcct tgtgtctaca 1140
gagaacctgg agttcctgaa cctgatgctg actagtgagg aagatttttg ttgttatttc 1200
ttacaggcac aaaatgatgg acccaatgca cacaaaacaa ccctagagtg ttgttgagaa 1260
ttgtgctcaa aatttgaaga atgaacaaat tgaactcttt gaatgacaaa gagtagacat 1320
ttctcttact gcaaatgtca tcagaacttt ttggtttgca gatgacaaaa attcaactca 1380
gactagttta gttaaatgag ggtggtgaat attgttcata ttgtggcaca agcaaaaagg 1440
gtgtctgagc cctcaaagtg aggggaacca gggcctgagc caagcta 1487

<210> 374
<211> 355
<212> PRT
<213> Homo sapiens

<400> 374
Met Asp Gln Phe Pro Glu Ser Val Thr Glu Asn Phe Glu Tyr Asp Asp
1               5                   10                  15
Leu Ala Glu Ala Cys Tyr Ile Gly Asp Ile Val Val Phe Gly Thr Val
            20                  25                  30
Phe Leu Ser Ile Phe Tyr Ser Val Ile Phe Ala Ile Gly Leu Val Gly
        35                  40                  45
Asn Leu Leu Val Val Phe Ala Leu Thr Asn Ser Lys Lys Pro Lys Ser
    50                  55                  60
Val Thr Asp Ile Tyr Leu Leu Asn Leu Ala Leu Ser Asp Leu Leu Phe

65                  70                  75                  80
Val Ala Thr Leu Pro Phe Trp Thr His Tyr Leu Ile Asn Glu Lys Gly
                85                  90                  95
Leu His Asn Ala Met Cys Lys Phe Thr Thr Ala Phe Phe Phe Ile Gly
            100                 105                 110
Phe Phe Gly Ser Ile Phe Phe Ile Thr Val Ile Ser Ile Asp Arg Tyr
        115                 120                 125
Leu Ala Ile Val Leu Ala Ala Asn Ser Met Asn Asn Arg Thr Val Gln
    130                 135                 140
His Gly Val Thr Ile Ser Leu Gly Val Trp Ala Ala Ala Ile Leu Val
145                 150                 155                 160
Ala Ala Pro Gln Phe Met Phe Thr Lys Gln Lys Glu Asn Glu Cys Leu
                165                 170                 175
Gly Asp Tyr Pro Glu Val Leu Gln Glu Ile Trp Pro Val Leu Arg Asn
            180                 185                 190
Val Glu Thr Asn Phe Leu Gly Phe Leu Leu Pro Leu Leu Ile Met Ser
        195                 200                 205
Tyr Cys Tyr Phe Arg Ile Ile Gln Thr Leu Phe Ser Cys Lys Asn His
    210                 215                 220
Lys Lys Ala Lys Ala Ile Lys Leu Ile Leu Leu Val Val Ile Val Phe
225                 230                 235                 240
Phe Leu Phe Trp Thr Pro Tyr Asn Val Met Ile Phe Leu Glu Thr Leu
                245                 250                 255
Lys Leu Tyr Asp Phe Phe Pro Ser Cys Asp Met Arg Lys Asp Leu Arg
            260                 265                 270
Leu Ala Leu Ser Val Thr Glu Thr Val Ala Phe Ser His Cys Cys Leu
        275                 280                 285
Asn Pro Leu Ile Tyr Ala Phe Ala Gly Glu Lys Phe Arg Arg Tyr Leu
    290                 295                 300
Tyr His Leu Tyr Gly Lys Cys Leu Ala Val Leu Cys Gly Arg Ser Val
305                 310                 315                 320
His Val Asp Phe Ser Ser Ser Glu Ser Gln Arg Ser Arg His Gly Ser
                325                 330                 335
Val Leu Ser Ser Asn Phe Thr Tyr His Thr Ser Asp Gly Asp Ala Leu
            340                 345                 350
Leu Leu Leu
        355

<210> 375
<211> 2135
<212> DNA
<213> Homo sapiens

<400> 375
tcggcttatt tgttttccct cctcttagga ttgccccctg tgggtcactt tctcagtcat      60
tttgagctca gcctaatcaa agactgaggt tatgaagtcg atcctagatg gccttgcaga     120
taccaccttc cgcaccatca ccactgacct cctgtacgtg ggctcaaatg acattcagta     180
cgaagacatc aaaggtgaca tggcatccaa attagggtac ttcccacaga aattcccttt     240
aacttccttt aggggaagtc ccttccaaga gaagatgact gcgggagaca acccccagct     300
agtcccagca gaccaggtga acattacaga attttacaac aagtctctct cgtccttcaa     360
ggagaatgag gagaacatcc agtgtgggga gaacttcatg gacatagagt gtttcatggt     420
cctgaacccc agccagcagc tggccattgc agtcctgtcc ctcacgctgg gcaccttcac     480
ggtcctggag aacctcctgg tgctgtgcgt catcctccac tcccgcagcc tccgctgcag     540
gccttcctac cacttcatcg gcagcctggc ggtggcagac ctcctgggga gtgtcatttt     600
tgtctacagc ttcattgact tccacgtgtt ccaccgcaaa gatagccgca acgtgtttct     660
gttcaaactg ggtggggtca cggcctcctt cactgcctcc gtgggcagcc tgttcctcac     720
agccatcgac aggtacatat ccattcacag gcccctggcc tataagagga ttgtcaccag     780
gcccaaggcc gtggtggcgt tttgcctgat gtggaccata gccattgtga tcgccgtgct     840
gcctctcctg ggctggaact gcgagaaact gcaatctgtt tgctcagaca ttttcccaca     900
cattgatgaa acctacctga tgttctggat cggggtcacc agcgtactgc ttctgttcat     960
cgtgtatgcg tacatgtata ttctctggaa ggctcacagc cacgccgtcc gcatgattca    1020
gcgtggcacc cagaagagca tcatcatcca cacgtctgag gatgggaagg tacaggtgac    1080
ccggccagac caagcccgca tggacattag gttagccaag accctggtcc tgatcctggt    1140

ggtgttgatc atctgctggg gccctctgct tgcaatcatg gtgtatgatg tctttgggaa 1200
gatgaacaag ctcattaaga cggtgtttgc attctgcagt atgctctgcc tgctgaactc 1260
caccgtgaac cccatcatct atgctctgag gagtaaggac ctgcgacacg ctttccggag 1320
catgtttccc tcttgtgaag gcactgcgca gcctctggat aacagcatgg gggactcgga 1380
ctgcctgcac aaacacgcaa acaatgcagc cagtgttcac agggccgcag aaagctgcat 1440
caagagcaca gtcaagattg ccaaggtaac catgtctgtg tccacagaca cgtctgccga 1500
ggctctgtga gcctgatgcc tccctggcag cacaggaaaa gaattttttt ttttaagctc 1560
aaaatctaga agagtctatt gtctccttgg ttatattttt ttaactttac catgctcaat 1620
gaaaaggtga ttgtcaccat gatcacttat cagtttgcta atgtttccat agtttaggta 1680
ctcaaactcc attctccagg ggtttacagt gaagaaagcc tgttgtttaa gtgactgaac 1740
gatccttcaa agtctcaatg aaataggagg gaaacctttg gctacacaat tggaagtcta 1800
agaacccatg gaaaaatgcc atcaaatgaa taatgccttt gtaaccacaa ctttcactat 1860
aatgtgaaat gtaactgtcc gtagtatcag agatgtccat ttttacaagt tatagtacta 1920
gagatatttt gtaaaatgta ttatgtcctg tgagatgtgt atcagtgttt atgtgctatt 1980
aatatttgtt tagttcagcc aaactgaaag gtagactttt atgagaacaa tggacaagca 2040
gtggatacgt gtcaatgtgt gcactttttt tctatattat tgcccatgat ataactttag 2100
aaataaacct taatatttct tcccaaaaaa aaaaa 2135

<210> 376
<211> 472
<212> PRT
<213> Homo sapiens

<400> 376
Met Lys Ser Ile Leu Asp Gly Leu Ala Asp Thr Thr Phe Arg Thr Ile
1               5                   10                  15
Thr Thr Asp Leu Leu Tyr Val Gly Ser Asn Asp Ile Gln Tyr Glu Asp
            20                  25                  30
Ile Lys Gly Asp Met Ala Ser Lys Leu Gly Tyr Phe Pro Gln Lys Phe
        35                  40                  45
Pro Leu Thr Ser Phe Arg Gly Ser Pro Phe Gln Glu Lys Met Thr Ala
    50                  55                  60
Gly Asp Asn Pro Gln Leu Val Pro Ala Asp Gln Val Asn Ile Thr Glu
65                  70                  75                  80
Phe Tyr Asn Lys Ser Leu Ser Ser Phe Lys Glu Asn Glu Glu Asn Ile
                85                  90                  95
Gln Cys Gly Glu Asn Phe Met Asp Ile Glu Cys Phe Met Val Leu Asn
            100                 105                 110
Pro Ser Gln Gln Leu Ala Ile Ala Val Leu Ser Leu Thr Leu Gly Thr
        115                 120                 125
Phe Thr Val Leu Glu Asn Leu Leu Val Leu Cys Val Ile Leu His Ser
    130                 135                 140
Arg Ser Leu Arg Cys Arg Pro Ser Tyr His Phe Ile Gly Ser Leu Ala
145                 150                 155                 160
Val Ala Asp Leu Leu Gly Ser Val Ile Phe Val Tyr Ser Phe Ile Asp
                165                 170                 175
Phe His Val Phe His Arg Lys Asp Ser Arg Asn Val Phe Leu Phe Lys
            180                 185                 190
Leu Gly Gly Val Thr Ala Ser Phe Thr Ala Ser Val Gly Ser Leu Phe
        195                 200                 205
Leu Thr Ala Ile Asp Arg Tyr Ile Ser Ile His Arg Pro Leu Ala Tyr
    210                 215                 220
Lys Arg Ile Val Thr Arg Pro Lys Ala Val Val Ala Phe Cys Leu Met
225                 230                 235                 240
Trp Thr Ile Ala Ile Val Ile Ala Val Leu Pro Leu Leu Gly Trp Asn
                245                 250                 255
Cys Glu Lys Leu Gln Ser Val Cys Ser Asp Ile Phe Pro His Ile Asp
            260                 265                 270
Glu Thr Tyr Leu Met Phe Trp Ile Gly Val Thr Ser Val Leu Leu Leu
        275                 280                 285
Phe Ile Val Tyr Ala Tyr Met Tyr Ile Leu Trp Lys Ala His Ser His
    290                 295                 300
Ala Val Arg Met Ile Gln Arg Gly Thr Gln Lys Ser Ile Ile Ile His

305                 310                 315                 320
Thr Ser Glu Asp Gly Lys Val Gln Val Thr Arg Pro Asp Gln Ala Arg
                325                 330                 335
Met Asp Ile Arg Leu Ala Lys Thr Leu Val Leu Ile Leu Val Val Leu
            340                 345                 350
Ile Ile Cys Trp Gly Pro Leu Leu Ala Ile Met Val Tyr Asp Val Phe
        355                 360                 365
Gly Lys Met Asn Lys Leu Ile Lys Thr Val Phe Ala Phe Cys Ser Met
    370                 375                 380
Leu Cys Leu Leu Asn Ser Thr Val Asn Pro Ile Ile Tyr Ala Leu Arg
385                 390                 395                 400
Ser Lys Asp Leu Arg His Ala Phe Arg Ser Met Phe Pro Ser Cys Glu
                405                 410                 415
Gly Thr Ala Gln Pro Leu Asp Asn Ser Met Gly Asp Ser Asp Cys Leu
            420                 425                 430
His Lys His Ala Asn Asn Ala Ala Ser Val His Arg Ala Ala Glu Ser
        435                 440                 445
Cys Ile Lys Ser Thr Val Lys Ile Ala Lys Val Thr Met Ser Val Ser
    450                 455                 460
Thr Asp Thr Ser Ala Glu Ala Leu
465                 470

<210> 377
<211> 4286
<212> DNA
<213> Homo sapiens

<400> 377
gagacattcc ggtgggggac tctggccagc ccgagcaacg tggatcctga gagcactccc      60
aggtaggcat ttgccccggt gggacgcctt gccagagcag tgtgtggcag gcccccgtgg     120
aggatcaaca cagtggctga acactgggaa ggaactggta cttggagtct ggacatctga     180
aacttggctc tgaaactgcg cagcggccac cggacgcctt ctggagcagg tagcagcatg     240
cagccgcctc caagtctgtg cggacgcgcc ctggttgcgc tggttcttgc ctgcggcctg     300
tcgcggatct ggggagagga gagaggcttc ccgcctgaca gggccactcc gcttttgcaa     360
accgcagaga taatgacgcc acccactaag accttatggc ccaagggttc caacgccagt     420
ctggcgcggt cgttggcacc tgcggaggtg cctaaaggag acaggacggc aggatctccg     480
ccacgcacca tctcccctcc cccgtgccaa ggacccatcg agatcaagga gactttcaaa     540
tacatcaaca cggttgtgtc ctgccttgtg ttcgtgctgg ggatcatcgg gaactccaca     600
cttctgagaa ttatctacaa gaacaagtgc atgcgaaacg gtcccaatat cttgatcgcc     660
agcttggctc tgggagacct gctgcacatc gtcattgaca tccctatcaa tgtctacaag     720
ctgctggcag aggactggcc atttggagct gagatgtgta agctggtgcc tttcatacag     780
aaagcctccg tgggaatcac tgtgctgagt ctatgtgctc tgagtattga cagatatcga     840
gctgttgctt cttggagtag aattaaagga attggggttc caaaatggac agcagtagaa     900
attgttttga tttgggtggt ctctgtggtt ctggctgtcc ctgaagccat aggttttgat     960
ataattacga tggactacaa aggaagttat ctgcgaatct gcttgcttca tcccgttcag    1020
aagacagctt tcatgcagtt ttacaagaca gcaaaagatt ggtggctgtt cagtttctat    1080
ttctgcttgc cattggccat cactgcattt ttttatacac taatgacctg tgaaatgttg    1140
agaaagaaaa gtggcatgca gattgcttta aatgatcacc taaagcagag acgggaagtg    1200
gccaaaaccg tcttttgcct ggtccttgtc tttgccctct gctggcttcc ccttcacctc    1260
agcaggattc tgaagctcac tctttataat cagaatgatc ccaatagatg tgaacttttg    1320
agctttctgt tggtattgga ctatattggt atcaacatgg cttcactgaa ttcctgcatt    1380
aacccaattg ctctgtattt ggtgagcaaa agattcaaaa actgctttaa gtcatgctta    1440
tgctgctggt gccagtcatt tgaagaaaaa cagtccttgg aggaaaagca gtcgtgctta    1500
aagttcaaag ctaatgatca cggatatgac aacttccgtt ccagtaataa atacagctca    1560
tcttgaaaga agaactattc actgtatttc attttcttta tattggaccg aagtcattaa    1620
aacaaaatga aacatttgcc aaaacaaaac aaaaaactat gtatttgcac agcacactat    1680
taaaatatta agtgtaatta ttttaacact cacagctaca tatgacattt tatgagctgt    1740
ttacggcatg gaaagaaaat cagtgggaat taagaaagcc tcgtcgtgaa agcacttaat    1800
tttttacagt tagcacttca acatagctct taacaacttc caggatattc acacaacact    1860
taggcttaaa aatgagctca ctcagaattt ctattctttc taaaaagaga tttattttta    1920
aatcaatggg actctgatat aaaggaagaa taagtcactg taaaacagaa cttttaaatg    1980
aagcttaaat tactcaattt aaaattttaa aatcctttaa aacaactttt caattaatat    2040
tatcacacta ttatcagatt gtaattagat gcaaatgaga gagcagttta gttgttgcat    2100

ttttcggaca ctggaaacat ttaaatgatc aggagggagt aacagaaaga gcaaggctgt 2160
ttttgaaaat cattacactt tcactagaag cccaaacctc agcattctgc aatatgtaac 2220
caacatgtca caaacaagca gcatgtaaca gactggcaca tgtgccagct gaatttaaaa 2280
tataatactt ttaaaaagaa aattattaca tcctttacat tcagttaaga tcaaacctca 2340
caaagagaaa tagaatgttt gaaaggctat cccaaaagac ttttttgaat ctgtcattca 2400
cataccctgt gaagacaata ctatctacaa ttttttcagg attattaaaa tcttcttttt 2460
tcactatcgt agcttaaact ctgtttggtt ttgtcatctg taaatactta cctacataca 2520
ctgcatgtag atgattaaat gagggcaggc cctgtgctca tagctttacg atggagagat 2580
gccagtgacc tcataataaa gactgtgaac tgcctggtgc agtgtccaca tgacaaaggg 2640
gcaggtagca ccctctctca cccatgctgt ggttaaaatg gtttctagca tatgtataat 2700
gctatagtta aaatactatt tttcaaaatc atacagatta gtacatttaa cagctacctg 2760
taaagcttat tactaatttt tgtattattt ttgtaaatag ccaatagaaa agtttgcttg 2820
acatggtgct tttctttcat ctagaggcaa aactgctttt tgagaccgta agaacctctt 2880
agctttgtgc gttcctgcct aatttttata tcttctaagc aaagtgcctt aggatagctt 2940
gggatgagat gtgtgtgaaa gtatgtacaa gagaaaacgg aagagagagg aaatgaggtg 3000
gggttggagg aaacccatgg ggacagattc ccattcttag cctaacgttc gtcattgcct 3060
cgtcacatca atgcaaaagg tcctgatttt gttccagcaa aacacagtgc aatgttctca 3120
gagtgacttt cgaaataaat tgggcccaag agctttaact cggtcttaaa atatgcccaa 3180
atttttactt tgtttttctt ttaataggct gggccacatg ttggaaataa gctagtaatg 3240
ttgttttctg tcaatattga atgtgatggt acagtaaacc aaaacccaac aatgtggcca 3300
gaaagaaaga gcaataataa ttaattcaca caccatatgg attctattta taaatcaccc 3360
acaaacttgt tctttaattt catcccaatc actttttcag aggcctgtta tcatagaagt 3420
cattttagac tctcaatttt aaattaattt tgaatcacta atattttcac agtttattaa 3480
tatatttaat ttctatttaa attttagatt atttttatta ccatgtactg aatttttaca 3540
tcctgatacc ctttccttct ccatgtcagt atcatgttct ctaattatct tgccaaattt 3600
tgaaactaca cacaaaaagc atacttgcat tatttataat aaaattgcat tcagtggctt 3660
tttaaaaaaa atgtttgatt caaaacttta acatactgat aagtaagaaa caattataat 3720
ttctttacat actcaaaacc aagatagaaa aaggtgctat cgttcaactt caaaacatgt 3780
ttcctagtat taaggacttt aatatagcaa cagacaaaat tattgttaac atggatgtta 3840
cagctcaaaa gatttataaa agattttaac ctattttctc ccttattatc cactgctaat 3900
gtggatgtat gttcaaacac cttttagtat tgatagctta catatggcca aaggaataca 3960
gtttatagca aaacatgggt atgctgtagc taactttata aaagtgtaat ataacaatgt 4020
aaaaaattat atatctggga ggattttttg gttgcctaaa gtggctatag ttactgattt 4080
tttattatgt aagcaaaacc aataaaaatt taagtttttt taacaactac cttatttttc 4140
actgtacaga cactaattca ttaaatacta attgattgtt taaaagaaat ataaatgtga 4200
caagtggaca ttatttatgt taaatataca attatcaagc aagtatgaag ttattcaatt 4260
aaaatgccac atttctggtc tctggg 4286

<210> 378
<211> 442
<212> PRT
<213> Homo sapiens

<400> 378
Met Gln Pro Pro Pro Ser Leu Cys Gly Arg Ala Leu Val Ala Leu Val
1               5                   10                  15
Leu Ala Cys Gly Leu Ser Arg Ile Trp Gly Glu Glu Arg Gly Phe Pro
            20                  25                  30
Pro Asp Arg Ala Thr Pro Leu Leu Gln Thr Ala Glu Ile Met Thr Pro
        35                  40                  45
Pro Thr Lys Thr Leu Trp Pro Lys Gly Ser Asn Ala Ser Leu Ala Arg
    50                  55                  60
Ser Leu Ala Pro Ala Glu Val Pro Lys Gly Asp Arg Thr Ala Gly Ser
65                  70                  75                  80
Pro Pro Arg Thr Ile Ser Pro Pro Pro Cys Gln Gly Pro Ile Glu Ile
                85                  90                  95
Lys Glu Thr Phe Lys Tyr Ile Asn Thr Val Val Ser Cys Leu Val Phe
            100                 105                 110
Val Leu Gly Ile Ile Gly Asn Ser Thr Leu Leu Arg Ile Ile Tyr Lys
        115                 120                 125
Asn Lys Cys Met Arg Asn Gly Pro Asn Ile Leu Ile Ala Ser Leu Ala
    130                 135                 140
Leu Gly Asp Leu Leu His Ile Val Ile Asp Ile Pro Ile Asn Val Tyr

145                 150                 155                 160
Lys Leu Leu Ala Glu Asp Trp Pro Phe Gly Ala Glu Met Cys Lys Leu
                165                 170                 175
Val Pro Phe Ile Gln Lys Ala Ser Val Gly Ile Thr Val Leu Ser Leu
            180                 185                 190
Cys Ala Leu Ser Ile Asp Arg Tyr Arg Ala Val Ala Ser Trp Ser Arg
        195                 200                 205
Ile Lys Gly Ile Gly Val Pro Lys Trp Thr Ala Val Glu Ile Val Leu
    210                 215                 220
Ile Trp Val Val Ser Val Val Leu Ala Val Pro Glu Ala Ile Gly Phe
225                 230                 235                 240
Asp Ile Ile Thr Met Asp Tyr Lys Gly Ser Tyr Leu Arg Ile Cys Leu
                245                 250                 255
Leu His Pro Val Gln Lys Thr Ala Phe Met Gln Phe Tyr Lys Thr Ala
            260                 265                 270
Lys Asp Trp Trp Leu Phe Ser Phe Tyr Phe Cys Leu Pro Leu Ala Ile
        275                 280                 285
Thr Ala Phe Phe Tyr Thr Leu Met Thr Cys Glu Met Leu Arg Lys Lys
    290                 295                 300
Ser Gly Met Gln Ile Ala Leu Asn Asp His Leu Lys Gln Arg Arg Glu
305                 310                 315                 320
Val Ala Lys Thr Val Phe Cys Leu Val Leu Val Phe Ala Leu Cys Trp
                325                 330                 335
Leu Pro Leu His Leu Ser Arg Ile Leu Lys Leu Thr Leu Tyr Asn Gln
            340                 345                 350
Asn Asp Pro Asn Arg Cys Glu Leu Leu Ser Phe Leu Leu Val Leu Asp
        355                 360                 365
Tyr Ile Gly Ile Asn Met Ala Ser Leu Asn Ser Cys Ile Asn Pro Ile
    370                 375                 380
Ala Leu Tyr Leu Val Ser Lys Arg Phe Lys Asn Cys Phe Lys Ser Cys
385                 390                 395                 400
Leu Cys Cys Trp Cys Gln Ser Phe Glu Glu Lys Gln Ser Leu Glu Glu
                405                 410                 415
Lys Gln Ser Cys Leu Lys Phe Lys Ala Asn Asp His Gly Tyr Asp Asn
            420                 425                 430
Phe Arg Ser Ser Asn Lys Tyr Ser Ser Ser
        435                 440

<210> 379
<211> 1374
<212> DNA
<213> Homo sapiens

<400> 379
aaaaacctga agacataaga actacacatg aggaatatgt catttagcac tttcactttt      60
tgatctccac agaagacaat gagaagtcat accataacaa tgacgacaac ttcagtcagc     120
agctggcctt actcctccca cagaatgcgc tttataacca atcatagcga ccaaccgcca     180
caaaacttct cagcaacacc aaatgttact acctgtccca tggatgaaaa attgctatct     240
actgtgttaa ccacatccta ctctgttatt ttcatcgtgg gactggttgg gaacataatc     300
gccctctatg tatttctggg tattcaccgt aaaagaaatt ccattcaaat ttatctactt     360
aacgtagcca ttgcagacct cctactcatc ttctgcctcc ctttccgaat catgtatcat     420
attaaccaaa acaagtggac actaggtgtg attctgtgca aggttgtggg aacactgttt     480
tatatgaaca tgtacattag cattattttg cttggattca tcagtttgga tcgctatata     540
aaaattaatc ggtctataca gcaacggaag gcaataacaa ccaaacaaag tatttatgtc     600
tgttgtatag tatggatggt tgctcttggt ggattcctaa ctatgattat tttaacactt     660
aagaaaggag ggcataattc cacaatgtgt ttccattaca gagataagca taacgcaaaa     720
ggagaagcca tttttaactt cattcttgtg gtaatgttct ggctaatttt cttactaata     780
atcctttcat atattaagat tgggaagaat ctattgagga tttctaaaag gaggtcaaaa     840
tttcctaatt ctggtaaata tgccactaca gctcgtaact cctttattgt acttatcatt     900
tttactatat gttttgttcc ctatcatgcc tttcgattca tctacatttc ttcacagcta     960
aatgtatcat cttgctactg gaaagaaatt gttcacaaaa ccaatgagat catgctggtt    1020
ctctcatctt tcaatagttg cttagatcca gtcatgtatt tcctgatgtc cagtaacatt    1080
cgcaaaataa tgtgccaact tctttttaga cgatttcaag gtgaaccaag taggagtgaa    1140

agcacttcag aatttaaacc aggatactcc ctgcatgata catctgtggc agtgaaaata 1200
cagtctagtt ctaaaagtac ttgaggtaaa catactaaaa tgaattatat aatgcagcct 1260
cttaattctt tgaagaacta aaaaattagg aaacaaagtt ctagcattta caaaactcag 1320
atctcaaagc tctgcttgta tttgtgatat ttcatttgct taactgtaaa ccat 1374

<210> 380
<211> 381
<212> PRT
<213> Homo sapiens

<400> 380

<210> 381
<211> 2833

<212> DNA
<213> Homo sapiens

<400> 381
cactttgcgg gcggcacttt ttccaggttg ttaatccagc taatggagaa ggatagatgc 60
acgctacttg gtttagaaaa aaaaacaaaa atgagcaaac gagacgcccc ttccgtttta 120
tgataactaa gctgcaggga aataaatcgg ctggccctac tgcaatctac tgcactcgag 180
aaacatcaca gaaaattctt tgatttatct taatagtgac aagtgagcct gcttctgtca 240
attactgaag ctataaggag attttttaaa aattaaactt caacacaatg aggtgttgcc 300
acatctgcaa acttcctggg agagtaatgg ggattcgagt gcttcgatta tctttggtgg 360
tcatcctcgt attattactg gtagctggtg ctttgactgc cttacttccc agtgttaaag 420
aagacaagat gctcatgttg cgtagggaaa taaaatccca gggcaagtcc accatggact 480
cctttactct cataatgcag acgtacaaca gaacagatct cttattgaaa cttttaaatc 540
attatcaggc tgtaccaaat ctgcacaaag tgattgtggt atggaacaat attggagaga 600
aggcaccaga tgaattatgg aattctctag ggccccaccc tatccctgtg atcttcaaac 660
aacagacagc aaacaggatg agaaatcgac tccaggtctt tcctgaactg gaaaccaatg 720
cagtgttgat ggtagatgat gacacactca tcagcacccc agaccttgtt tttgctttct 780
cagtttggca gcaatttcct gatcaaattg taggatttgt tcctagaaag cacgtctcta 840
cttcatcagg tatctacagt tatggaagtt ttgaaatgca agcaccaggg tctggaaatg 900
gtgaccagta ctctatggtg ctgattggag cctcattctt caatagcaaa tatcttgaat 960
tatttcagag gcaacctgca gctgtccatg ctttgataga tgatactcaa aactgtgatg 1020
atattgccat gaattttatc attgccaagc atattggcaa gacttcaggg atatttgtga 1080
agcctgtaaa catggacaat ttggaaaaag aaaccaacag tggctattct ggaatgtggc 1140
atcgagctga gcacgctctg cagaggtctt attgtataaa taagcttgtt aatatctatg 1200
atagcatgcc cttaagatac tccaacatta tgatttccca gtttggtttt ccatatgcca 1260
actacaaaag aaaaatataa aagtaaaaca aacaaaaaca aacctgaaaa ctgcttggca 1320
tttgagtagc ttctccatgc tatgtatttt tttaagcaac atcatgaatt ttatctactc 1380
cagaagtctc tacaatagaa aaaaaagtgc agtgcttcta ggatataaaa ttcacattac 1440
ttttgaaagc caagaagttg gtcttatcca gttaggtctt cttatgaaga gttttcatcc 1500
agggatataa ctccttggtc agtgatttta ttgtttacat cctgagactg ttctacagtt 1560
tctttgactc ctggcatttg ccttaaggac ctatagcaag ctgtttctag gatcagaaac 1620
tcaagagagg catttctctg ctttttcact aaaggtcagt tgttttaatt tgaaacctga 1680
aatgcctctt tagcaaagcc tgtggtatgg ggtaaagcca tgtaagaaga gaatagtctc 1740
agtcacatat gaagaggaaa atttgcagct gccagtgctt tccttgtggc cctgccaacc 1800
agctcttcca ggacgaactc agtccagcat ggttttgatg taaccatcca tgcttttatt 1860
tttgttaagt cttttgtgac tgggacagtt aattttagta gctgaagaac gtctagttgt 1920
ttgcttgata tttgtgaaca tttactgcat ggatcacaaa acaatatacc ctgtatttct 1980
tacacgccac ttatatgcag caaggagtaa atgtgttact agattcgggt agtgcatttt 2040
gtcactgaat ctgaccttga gaatgtacat taattcttat attttacata atgtatgtgt 2100
tgtttaagaa atgtataaaa aacctgaaaa aaatgagtaa gaactggcag aagttaaaac 2160
cctttgtatc aaaagatctt tattggtaga gcactggtta tcttctggat actaaaaagt 2220
tgtattacaa agccaaacac ttgcattcac aactttaaaa aaagatccaa ggaactattc 2280
ataatgatga aatttcaact acatacaagg aggagaaaat aagaacccag tcataacaga 2340
ggaattctat aggagtctgc atcaattcat tcttaaggtt gcctactctc tgttatgtga 2400
attagcgtct gtgtttcacc cattgtctgt gtttagtcct tgttcaccac taaggcaagg 2460
aattcttaac taggcctctg tttaccaact tctctttctc ctcctttccc tcttattcct 2520
ccttctcctc ttccttctta tataatgcta gtatattctc aaaattgcaa agctgtgaga 2580
atattaaaat aatcatggct aatgttccaa taatgaggtc tttgtgcatt tagttccgca 2640
tatgatggtt ttttttttac attaaagagt atatgtgtct taatgcagtc agattgtaaa 2700
aaacaaaaac aaagaaacta agaatcttac taaaaatcga taatgtcagt tatctgtttt 2760
gtccaatatt ggtagtactt ttttgcctct tatgattcct ctagcagata aataaaagaa 2820
acttttgcca tcc 2833

<210> 382
<211> 330
<212> PRT
<213> Homo sapiens

<400> 382
Met Arg Cys Cys His Ile Cys Lys Leu Pro Gly Arg Val Met Gly Ile
1               5                   10                  15
Arg Val Leu Arg Leu Ser Leu Val Val Ile Leu Val Leu Leu Leu Val
            20                  25                  30

Ala Gly Ala Leu Thr Ala Leu Leu Pro Ser Val Lys Glu Asp Lys Met
        35                  40                  45
Leu Met Leu Arg Arg Glu Ile Lys Ser Gln Gly Lys Ser Thr Met Asp
    50                  55                  60
Ser Phe Thr Leu Ile Met Gln Thr Tyr Asn Arg Thr Asp Leu Leu Leu
65                  70                  75                  80
Lys Leu Leu Asn His Tyr Gln Ala Val Pro Asn Leu His Lys Val Ile
                85                  90                  95
Val Val Trp Asn Asn Ile Gly Glu Lys Ala Pro Asp Glu Leu Trp Asn
            100                 105                 110
Ser Leu Gly Pro His Pro Ile Pro Val Ile Phe Lys Gln Gln Thr Ala
        115                 120                 125
Asn Arg Met Arg Asn Arg Leu Gln Val Phe Pro Glu Leu Glu Thr Asn
    130                 135                 140
Ala Val Leu Met Val Asp Asp Asp Thr Leu Ile Ser Thr Pro Asp Leu
145                 150                 155                 160
Val Phe Ala Phe Ser Val Trp Gln Gln Phe Pro Asp Gln Ile Val Gly
                165                 170                 175
Phe Val Pro Arg Lys His Val Ser Thr Ser Ser Gly Ile Tyr Ser Tyr
            180                 185                 190
Gly Ser Phe Glu Met Gln Ala Pro Gly Ser Gly Asn Gly Asp Gln Tyr
        195                 200                 205
Ser Met Val Leu Ile Gly Ala Ser Phe Phe Asn Ser Lys Tyr Leu Glu
    210                 215                 220
Leu Phe Gln Arg Gln Pro Ala Ala Val His Ala Leu Ile Asp Asp Thr
225                 230                 235                 240
Gln Asn Cys Asp Asp Ile Ala Met Asn Phe Ile Ile Ala Lys His Ile
                245                 250                 255
Gly Lys Thr Ser Gly Ile Phe Val Lys Pro Val Asn Met Asp Asn Leu
            260                 265                 270
Glu Lys Glu Thr Asn Ser Gly Tyr Ser Gly Met Trp His Arg Ala Glu
        275                 280                 285
His Ala Leu Gln Arg Ser Tyr Cys Ile Asn Lys Leu Val Asn Ile Tyr
    290                 295                 300
Asp Ser Met Pro Leu Arg Tyr Ser Asn Ile Met Ile Ser Gln Phe Gly
305                 310                 315                 320
Phe Pro Tyr Ala Asn Tyr Lys Arg Lys Ile
                325                 330

<210> 383
<211> 2520
<212> DNA
<213> Homo sapiens

<400> 383
acgcggggcg cgcggctccg tcggctaccg cgggcgggcg caggcgacgg gcacggcggg      60
cgagcgggcg gtatggcggc ggcggggccc gcggcggggc cgacggggcc cgagcccatg     120
ccgagctacg cgcagctagt gcagcgcggc tggggcagcg cgctggcggc ggcgcggggc     180
tgcacggact gcggctgggg gctggcgcgt cgcggcctgg ctgagcacgc gcacctggcg     240
ccgcccgagc tgctgctgct ggcgctcggc gcgctgggct ggaccgcgct gcgctccgcg     300
gccactgcgc gcctctttcg gcccctggcg aagcggtgct gcctccagcc cagagatgcc     360
gccaagatgc ccgagagcgc ttggaagttt ctcttctacc tgggcagctg gagctacagt     420
gcctacctgc tgtttggcac cgactacccc ttcttccatg acccaccatc tgtcttctac     480
gactggacgc cgggcatggc agtgccacgg gacattgcag ccgcctacct gctccaggga     540
agcttctatg gccactccat ctacgctacg ctatacatgg acacctggcg caaggactcg     600
gtggtcatgc tgctccacca cgtggtcact ctcatcctca tcgtctcctc ctacgccttc     660
cggtaccaca atgtgggcat ccttgtgctc ttcctgcacg atatcagtga cgtgcagctt     720
gagttcacca agctcaacat ttacttcaag tcccgcggcg gctcctacca tcggctgcat     780
gccttggcag cagacttggg ctgcctcagc ttcggcttca gctggttctg gttccgcctc     840
tactggttcc cgctcaaggt cctgtatgcc accagtcact gcagtctgcg cacggtgcct     900
gacatcccct tctacttctt cttcaatgcg ctcctgctgc tgctcaccct tatgaacctc     960
tactggttcc tgtacatcgt ggcgtttgca gccaaggtgt tgacaggcca ggtgcacgag    1020

ctgaaggacc tgcgggagta tgacacagcc gaggcccaga gcctgaagcc cagcaaagcc 1080
gagaagccac tgaggaacgg cctggtgaag gacaagcgct tctgaacccc tcggccccgc 1140
ccccgtggac ccggccccac cccgaatacc ccggccacgc tccccgtcct tggccgcccc 1200
tccaccccct ccaactctgc tcctctaggg ccgccgccac ctcccctggg accccgcccc 1260
ctcatcctgc ctccatttcc cggccacgcc ccccaggacc cctgcccctc cggggacacc 1320
ggccccgccc tcagcccact ggtcccgggc cgccgcggac cctgcgcact ctctggtcat 1380
cgcctgggag gaagatgcca ccgccgcagc aaggtccctg cggccaccac ctcctcctcc 1440
tcctggccct gctgctgccc tcgctgcccc tgacccgcgc ccccgtgccc ccaggcccag 1500
ccgccgccct gctccaggct ctaggactgc gcgatgagcc ccagggtgcc cccaggctcc 1560
ggccggttcc cccggtcatg tggcgcctgt ttcgacgccg ggacccccag gagaccaggt 1620
ctggctcgcg gcggacgtcc ccaggggtca ccctgcaacc gtgccacgtg gaggagctgg 1680
gggtcgccgg aaacatcgtg cgccacatcc cggaccgcgg tgcgcccacc cgggcctcgg 1740
agcctgtctc ggccgcgggg cattgccctg agtggacagt cgtcttcgac ctgtcggctg 1800
tggaacccgc tgagcgcccg agccgggccc gcctggagct gcgtttcgcg gcggcggcgg 1860
cggcagcccc ggagggcggc tgggagctga gcgtggcgca agcgggccag ggcgcgggcg 1920
cggaccccgg gccggtgctg ctccgccagt tggtgcccgc cctggggccg ccagtgcgcg 1980
cggagctgct gggcgccgct tgggctcgca acgcctcatg gccgcgcagc ctccgcctgg 2040
cgctggcgct acgcccccgg gcccctgccg cctgcgcgcg cctggccgag gcctcgctgc 2100
tgctggtgac cctcgacccg cgcctgtgcc accccctggc ccggccgcgg cgcgacgccg 2160
aacccgtgtt gggcggcggc cccgggggcg cttgtcgcgc gcggcggctg tacgtgagct 2220
tccgcgaggt gggctggcac cgctgggtca tcgcgccgcg cggcttcctg gccaactact 2280
gccagggtca gtgcgcgctg cccgtcgcgc tgtcggggtc cggggggccg ccggcgctca 2340
accacgctgt gctgcgcgcg ctcatgcacg cggccgcccc gggagccgcc gacctgccct 2400
gctgcgtgcc cgcgcgcctg tcgcccatct ccgtgctctt ctttgacaac agcgacaacg 2460
tggtgctgcg gcagtatgag gacatggtgg tggacgagtg cggctgccgc taacccgggg 2520

<210> 384
<211> 350
<212> PRT
<213> Homo sapiens

<400> 384
Met Ala Ala Ala Gly Pro Ala Ala Gly Pro Thr Gly Pro Glu Pro Met
1               5                   10                  15
Pro Ser Tyr Ala Gln Leu Val Gln Arg Gly Trp Gly Ser Ala Leu Ala
            20                  25                  30
Ala Ala Arg Gly Cys Thr Asp Cys Gly Trp Gly Leu Ala Arg Arg Gly
        35                  40                  45
Leu Ala Glu His Ala His Leu Ala Pro Pro Glu Leu Leu Leu Leu Ala
    50                  55                  60
Leu Gly Ala Leu Gly Trp Thr Ala Leu Arg Ser Ala Ala Thr Ala Arg
65                  70                  75                  80
Leu Phe Arg Pro Leu Ala Lys Arg Cys Cys Leu Gln Pro Arg Asp Ala
                85                  90                  95
Ala Lys Met Pro Glu Ser Ala Trp Lys Phe Leu Phe Tyr Leu Gly Ser
            100                 105                 110
Trp Ser Tyr Ser Ala Tyr Leu Leu Phe Gly Thr Asp Tyr Pro Phe Phe
        115                 120                 125
His Asp Pro Pro Ser Val Phe Tyr Asp Trp Thr Pro Gly Met Ala Val
    130                 135                 140
Pro Arg Asp Ile Ala Ala Ala Tyr Leu Leu Gln Gly Ser Phe Tyr Gly
145                 150                 155                 160
His Ser Ile Tyr Ala Thr Leu Tyr Met Asp Thr Trp Arg Lys Asp Ser
                165                 170                 175
Val Val Met Leu Leu His His Val Val Thr Leu Ile Leu Ile Val Ser
            180                 185                 190
Ser Tyr Ala Phe Arg Tyr His Asn Val Gly Ile Leu Val Leu Phe Leu
        195                 200                 205
His Asp Ile Ser Asp Val Gln Leu Glu Phe Thr Lys Leu Asn Ile Tyr
    210                 215                 220
Phe Lys Ser Arg Gly Gly Ser Tyr His Arg Leu His Ala Leu Ala Ala
225                 230                 235                 240
Asp Leu Gly Cys Leu Ser Phe Gly Phe Ser Trp Phe Trp Phe Arg Leu

                245                 250                 255
Tyr Trp Phe Pro Leu Lys Val Leu Tyr Ala Thr Ser His Cys Ser Leu
            260                 265                 270
Arg Thr Val Pro Asp Ile Pro Phe Tyr Phe Phe Phe Asn Ala Leu Leu
        275                 280                 285
Leu Leu Leu Thr Leu Met Asn Leu Tyr Trp Phe Leu Tyr Ile Val Ala
    290                 295                 300
Phe Ala Ala Lys Val Leu Thr Gly Gln Val His Glu Leu Lys Asp Leu
305                 310                 315                 320
Arg Glu Tyr Asp Thr Ala Glu Ala Gln Ser Leu Lys Pro Ser Lys Ala
                325                 330                 335
Glu Lys Pro Leu Arg Asn Gly Leu Val Lys Asp Lys Arg Phe
            340                 345                 350

<210> 385
<211> 5532
<212> DNA
<213> Homo sapiens

<400> 385
gccgcgctgc gccggagtcc cgagctagcc ccggcgccgc cgccgcccag accggacgac 60
aggccacctc gtcggcgtcc gcccgagtcc ccgcctcgcc gccaacgcca caaccaccgc 120
gcacggcccc ctgactccgt ccagtattga tcgggagagc cggagcgagc tcttcgggga 180
gcagcgatgc gaccctccgg gacggccggg gcagcgctcc tggcgctgct ggctgcgctc 240
tgcccggcga gtcgggctct ggaggaaaag aaagtttgcc aaggcacgag taacaagctc 300
acgcagttgg gcacttttga agatcatttt ctcagcctcc agaggatgtt caataactgt 360
gaggtggtcc ttgggaattt ggaaattacc tatgtgcaga ggaattatga tctttccttc 420
ttaaagacca tccaggaggt ggctggttat gtcctcattg ccctcaacac agtggagcga 480
attcctttgg aaaacctgca gatcatcaga ggaaatatgt actacgaaaa ttcctatgcc 540
ttagcagtct tatctaacta tgatgcaaat aaaaccggac tgaaggagct gcccatgaga 600
aatttacagg aaatcctgca tggcgccgtg cggttcagca acaaccctgc cctgtgcaac 660
gtggagagca tccagtggcg ggacatagtc agcagtgact ttctcagcaa catgtcgatg 720
gacttccaga accacctggg cagctgccaa aagtgtgatc caagctgtcc caatgggagc 780
tgctggggtg caggagagga gaactgccag aaactgacca aaatcatctg tgcccagcag 840
tgctccgggc gctgccgtgg caagtccccc agtgactgct gccacaacca gtgtgctgca 900
ggctgcacag gcccccggga gagcgactgc ctggtctgcc gcaaattccg agacgaagcc 960
acgtgcaagg acacctgccc cccactcatg ctctacaacc ccaccacgta ccagatggat 1020
gtgaaccccg agggcaaata cagctttggt gccacctgcg tgaagaagtg tccccgtaat 1080
tatgtggtga cagatcacgg ctcgtgcgtc cgagcctgtg gggccgacag ctatgagatg 1140
gaggaagacg gcgtccgcaa gtgtaagaag tgcgaagggc cttgccgcaa agtgtgtaac 1200
ggaataggta ttggtgaatt taaagactca ctctccataa atgctacgaa tattaaacac 1260
ttcaaaaact gcacctccat cagtggcgat ctccacatcc tgccggtggc atttaggggt 1320
gactccttca cacatactcc tcctctggat ccacaggaac tggatattct gaaaaccgta 1380
aaggaaatca cagggttttt gctgattcag gcttggcctg aaaacaggac ggacctccat 1440
gcctttgaga acctagaaat catacgcggc aggaccaagc aacatggtca gttttctctt 1500
gcagtcgtca gcctgaacat aacatccttg ggattacgct ccctcaagga gataagtgat 1560
ggagatgtga taatttcagg aaacaaaaat ttgtgctatg caaatacaat aaactggaaa 1620
aaactgtttg ggacctccgg tcagaaaacc aaaattataa gcaacagagg tgaaaacagc 1680
tgcaaggcca caggccaggt ctgccatgcc ttgtgctccc ccgagggctg ctggggcccg 1740
gagcccaggg actgcgtctc ttgccggaat gtcagccgag gcagggaatg cgtggacaag 1800
tgcaagcttc tggagggtga gccaagggag tttgtggaga actctgagtg catacagtgc 1860
cacccagagt gcctgcctca ggccatgaac atcacctgca caggacgggg accagacaac 1920
tgtatccagt gtgcccacta cattgacggc ccccactgcg tcaagacctg cccggcagga 1980
gtcatgggag aaaacaacac cctggtctgg aagtacgcag acgccggcca tgtgtgccac 2040
ctgtgccatc caaactgcac ctacggatgc actgggccag gtcttgaagg ctgtccaacg 2100
aatgggccta agatcccgtc catcgccact gggatggtgg gggccctcct cttgctgctg 2160
gtggtggccc tggggatcgg cctcttcatg cgaaggcgcc acatcgttcg gaagcgcacg 2220
ctgcggaggc tgctgcagga gagggagctt gtggagcctc ttacacccag tggagaagct 2280
cccaaccaag ctctcttgag gatcttgaag gaaactgaat tcaaaaagat caaagtgctg 2340
ggctccggtg cgttcggcac ggtgtataag ggactctgga tcccagaagg tgagaaagtt 2400
aaaattcccg tcgctatcaa ggaattaaga gaagcaacat ctccgaaagc caacaaggaa 2460
atcctcgatg aagcctacgt gatggccagc gtggacaacc cccacgtgtg ccgcctgctg 2520
ggcatctgcc tcacctccac cgtgcaactc atcacgcagc tcatgccctt cggctgcctc 2580

ctggactatg tccgggaaca caaagacaat attggctccc agtacctgct caactggtgt 2640
gtgcagatcg caaagggcat gaactacttg gaggaccgtc gcttggtgca ccgcgacctg 2700
gcagccagga acgtactggt gaaaacaccg cagcatgtca agatcacaga ttttgggctg 2760
gccaaactgc tgggtgcgga agagaaagaa taccatgcag aaggaggcaa agtgcctatc 2820
aagtggatgg cattggaatc aattttacac agaatctata cccaccagag tgatgtctgg 2880
agctacgggg tgaccgtttg ggagttgatg acctttggat ccaagccata tgacggaatc 2940
cctgccagcg agatctcctc catcctggag aaaggagaac gcctccctca gccacccata 3000
tgtaccatcg atgtctacat gatcatggtc aagtgctgga tgatagacgc agatagtcgc 3060
ccaaagttcc gtgagttgat catcgaattc tccaaaatgg cccgagaccc ccagcgctac 3120
cttgtcattc agggggatga aagaatgcat ttgccaagtc ctacagactc caacttctac 3180
cgtgccctga tggatgaaga agacatggac gacgtggtgg atgccgacga gtacctcatc 3240
ccacagcagg gcttcttcag cagcccctcc acgtcacgga ctcccctcct gagctctctg 3300
agtgcaacca gcaacaattc caccgtggct tgcattgata gaaatgggct gcaaagctgt 3360
cccatcaagg aagacagctt cttgcagcga tacagctcag accccacagg cgccttgact 3420
gaggacagca tagacgacac cttcctccca gtgcctgaat acataaacca gtccgttccc 3480
aaaaggcccg ctggctctgt gcagaatcct gtctatcaca atcagcctct gaaccccgcg 3540
cccagcagag acccacacta ccaggacccc cacagcactg cagtgggcaa ccccgagtat 3600
ctcaacactg tccagcccac ctgtgtcaac agcacattcg acagccctgc ccactgggcc 3660
cagaaaggca gccaccaaat tagcctggac aaccctgact accagcagga cttctttccc 3720
aaggaagcca agccaaatgg catctttaag ggctccacag ctgaaaatgc agaataccta 3780
agggtcgcgc cacaaagcag tgaatttatt ggagcatgac cacggaggat agtatgagcc 3840
ctaaaaatcc agactctttc gatacccagg accaagccac agcaggtcct ccatcccaac 3900
agccatgccc gcattagctc ttagacccac agactggttt tgcaacgttt acaccgacta 3960
gccaggaagt acttccacct cgggcacatt ttgggaagtt gcattccttt gtcttcaaac 4020
tgtgaagcat ttacagaaac gcatccagca agaatattgt ccctttgagc agaaatttat 4080
ctttcaaaga ggtatatttg aaaaaaaaaa aaaaagtata tgtgaggatt tttattgatt 4140
ggggatcttg gagtttttca ttgtcgctat tgatttttac ttcaatgggc tcttccaaca 4200
aggaagaagc ttgctggtag cacttgctac cctgagttca tccaggccca actgtgagca 4260
aggagcacaa gccacaagtc ttccagagga tgcttgattc cagtggttct gcttcaaggc 4320
ttccactgca aaacactaaa gatccaagaa ggccttcatg gccccagcag gccggatcgg 4380
tactgtatca agtcatggca ggtacagtag gataagccac tctgtccctt cctgggcaaa 4440
gaagaaacgg aggggatgaa ttcttcctta gacttacttt tgtaaaaatg tccccacggt 4500
acttactccc cactgatgga ccagtggttt ccagtcatga gcgttagact gacttgtttg 4560
tcttccattc cattgttttg aaactcagta tgccgcccct gtcttgctgt catgaaatca 4620
gcaagagagg atgacacatc aaataataac tcggattcca gcccacattg gattcatcag 4680
catttggacc aatagcccac agctgagaat gtggaatacc taaggataac accgcttttg 4740
ttctcgcaaa aacgtatctc ctaatttgag gctcagatga aatgcatcag gtcctttggg 4800
gcatagatca gaagactaca aaaatgaagc tgctctgaaa tctcctttag ccatcacccc 4860
aaccccccaa aattagtttg tgttacttat ggaagatagt tttctccttt tacttcactt 4920
caaaagcttt ttactcaaag agtatatgtt ccctccaggt cagctgcccc caaaccccct 4980
ccttacgctt tgtcacacaa aaagtgtctc tgccttgagt catctattca agcacttaca 5040
gctctggcca caacagggca ttttacaggt gcgaatgaca gtagcattat gagtagtgtg 5100
aattcaggta gtaaatatga aactagggtt tgaaattgat aatgctttca caacatttgc 5160
agatgtttta gaaggaaaaa agttccttcc taaaataatt tctctacaat tggaagattg 5220
gaagattcag ctagttagga gcccattttt tcctaatctg tgtgtgccct gtaacctgac 5280
tggttaacag cagtcctttg taaacagtgt tttaaactct cctagtcaat atccacccca 5340
tccaatttat caaggaagaa atggttcaga aaatattttc agcctacagt tatgttcagt 5400
cacacacaca tacaaaatgt tccttttgct tttaaagtaa tttttgactc ccagatcagt 5460
cagagcccct acagcattgt taagaaagta tttgattttt gtctcaatga aaataaaact 5520
atattcattt cc 5532

<210> 386
<211> 1210
<212> PRT
<213> Homo sapiens

<400> 386
Met Arg Pro Ser Gly Thr Ala Gly Ala Ala Leu Leu Ala Leu Leu Ala
1               5                   10                  15
Ala Leu Cys Pro Ala Ser Arg Ala Leu Glu Glu Lys Lys Val Cys Gln
            20                  25                  30
Gly Thr Ser Asn Lys Leu Thr Gln Leu Gly Thr Phe Glu Asp His Phe
        35                  40                  45

Leu Ser Leu Gln Arg Met Phe Asn Asn Cys Glu Val Val Leu Gly Asn
    50                  55                  60
Leu Glu Ile Thr Tyr Val Gln Arg Asn Tyr Asp Leu Ser Phe Leu Lys
65                  70                  75                  80
Thr Ile Gln Glu Val Ala Gly Tyr Val Leu Ile Ala Leu Asn Thr Val
                85                  90                  95
Glu Arg Ile Pro Leu Glu Asn Leu Gln Ile Ile Arg Gly Asn Met Tyr
            100                 105                 110
Tyr Glu Asn Ser Tyr Ala Leu Ala Val Leu Ser Asn Tyr Asp Ala Asn
        115                 120                 125
Lys Thr Gly Leu Lys Glu Leu Pro Met Arg Asn Leu Gln Glu Ile Leu
    130                 135                 140
His Gly Ala Val Arg Phe Ser Asn Asn Pro Ala Leu Cys Asn Val Glu
145                 150                 155                 160
Ser Ile Gln Trp Arg Asp Ile Val Ser Ser Asp Phe Leu Ser Asn Met
                165                 170                 175
Ser Met Asp Phe Gln Asn His Leu Gly Ser Cys Gln Lys Cys Asp Pro
            180                 185                 190
Ser Cys Pro Asn Gly Ser Cys Trp Gly Ala Gly Glu Glu Asn Cys Gln
        195                 200                 205
Lys Leu Thr Lys Ile Ile Cys Ala Gln Gln Cys Ser Gly Arg Cys Arg
    210                 215                 220
Gly Lys Ser Pro Ser Asp Cys Cys His Asn Gln Cys Ala Ala Gly Cys
225                 230                 235                 240
Thr Gly Pro Arg Glu Ser Asp Cys Leu Val Cys Arg Lys Phe Arg Asp
                245                 250                 255
Glu Ala Thr Cys Lys Asp Thr Cys Pro Pro Leu Met Leu Tyr Asn Pro
            260                 265                 270
Thr Thr Tyr Gln Met Asp Val Asn Pro Glu Gly Lys Tyr Ser Phe Gly
        275                 280                 285
Ala Thr Cys Val Lys Lys Cys Pro Arg Asn Tyr Val Val Thr Asp His
    290                 295                 300
Gly Ser Cys Val Arg Ala Cys Gly Ala Asp Ser Tyr Glu Met Glu Glu
305                 310                 315                 320
Asp Gly Val Arg Lys Cys Lys Lys Cys Glu Gly Pro Cys Arg Lys Val
                325                 330                 335
Cys Asn Gly Ile Gly Ile Gly Glu Phe Lys Asp Ser Leu Ser Ile Asn
            340                 345                 350
Ala Thr Asn Ile Lys His Phe Lys Asn Cys Thr Ser Ile Ser Gly Asp
        355                 360                 365
Leu His Ile Leu Pro Val Ala Phe Arg Gly Asp Ser Phe Thr His Thr
    370                 375                 380
Pro Pro Leu Asp Pro Gln Glu Leu Asp Ile Leu Lys Thr Val Lys Glu
385                 390                 395                 400
Ile Thr Gly Phe Leu Leu Ile Gln Ala Trp Pro Glu Asn Arg Thr Asp
                405                 410                 415
Leu His Ala Phe Glu Asn Leu Glu Ile Ile Arg Gly Arg Thr Lys Gln
            420                 425                 430
His Gly Gln Phe Ser Leu Ala Val Val Ser Leu Asn Ile Thr Ser Leu
        435                 440                 445
Gly Leu Arg Ser Leu Lys Glu Ile Ser Asp Gly Asp Val Ile Ile Ser
    450                 455                 460
Gly Asn Lys Asn Leu Cys Tyr Ala Asn Thr Ile Asn Trp Lys Lys Leu
465                 470                 475                 480
Phe Gly Thr Ser Gly Gln Lys Thr Lys Ile Ile Ser Asn Arg Gly Glu
                485                 490                 495
Asn Ser Cys Lys Ala Thr Gly Gln Val Cys His Ala Leu Cys Ser Pro
            500                 505                 510
Glu Gly Cys Trp Gly Pro Glu Pro Arg Asp Cys Val Ser Cys Arg Asn
        515                 520                 525
Val Ser Arg Gly Arg Glu Cys Val Asp Lys Cys Lys Leu Leu Glu Gly
    530                 535                 540
Glu Pro Arg Glu Phe Val Glu Asn Ser Glu Cys Ile Gln Cys His Pro

545                 550                 555                 560
Glu Cys Leu Pro Gln Ala Met Asn Ile Thr Cys Thr Gly Arg Gly Pro
                565                 570                 575
Asp Asn Cys Ile Gln Cys Ala His Tyr Ile Asp Gly Pro His Cys Val
            580                 585                 590
Lys Thr Cys Pro Ala Gly Val Met Gly Glu Asn Asn Thr Leu Val Trp
        595                 600                 605
Lys Tyr Ala Asp Ala Gly His Val Cys His Leu Cys His Pro Asn Cys
    610                 615                 620
Thr Tyr Gly Cys Thr Gly Pro Gly Leu Glu Gly Cys Pro Thr Asn Gly
625                 630                 635                 640
Pro Lys Ile Pro Ser Ile Ala Thr Gly Met Val Gly Ala Leu Leu Leu
                645                 650                 655
Leu Leu Val Val Ala Leu Gly Ile Gly Leu Phe Met Arg Arg Arg His
            660                 665                 670
Ile Val Arg Lys Arg Thr Leu Arg Arg Leu Leu Gln Glu Arg Glu Leu
        675                 680                 685
Val Glu Pro Leu Thr Pro Ser Gly Glu Ala Pro Asn Gln Ala Leu Leu
    690                 695                 700
Arg Ile Leu Lys Glu Thr Glu Phe Lys Lys Ile Lys Val Leu Gly Ser
705                 710                 715                 720
Gly Ala Phe Gly Thr Val Tyr Lys Gly Leu Trp Ile Pro Glu Gly Glu
                725                 730                 735
Lys Val Lys Ile Pro Val Ala Ile Lys Glu Leu Arg Glu Ala Thr Ser
            740                 745                 750
Pro Lys Ala Asn Lys Glu Ile Leu Asp Glu Ala Tyr Val Met Ala Ser
        755                 760                 765
Val Asp Asn Pro His Val Cys Arg Leu Leu Gly Ile Cys Leu Thr Ser
    770                 775                 780

Thr Val Gln Leu Ile Thr Gln Leu Met Pro Phe Gly Cys Leu Leu Asp
785                 790                 795                 800
Tyr Val Arg Glu His Lys Asp Asn Ile Gly Ser Gln Tyr Leu Leu Asn
                805                 810                 815
Trp Cys Val Gln Ile Ala Lys Gly Met Asn Tyr Leu Glu Asp Arg Arg
            820                 825                 830
Leu Val His Arg Asp Leu Ala Ala Arg Asn Val Leu Val Lys Thr Pro
        835                 840                 845
Gln His Val Lys Ile Thr Asp Phe Gly Leu Ala Lys Leu Leu Gly Ala
    850                 855                 860
Glu Glu Lys Glu Tyr His Ala Glu Gly Gly Lys Val Pro Ile Lys Trp
865                 870                 875                 880
Met Ala Leu Glu Ser Ile Leu His Arg Ile Tyr Thr His Gln Ser Asp
                885                 890                 895
Val Trp Ser Tyr Gly Val Thr Val Trp Glu Leu Met Thr Phe Gly Ser
            900                 905                 910
Lys Pro Tyr Asp Gly Ile Pro Ala Ser Glu Ile Ser Ser Ile Leu Glu
        915                 920                 925
Lys Gly Glu Arg Leu Pro Gln Pro Pro Ile Cys Thr Ile Asp Val Tyr
    930                 935                 940
Met Ile Met Val Lys Cys Trp Met Ile Asp Ala Asp Ser Arg Pro Lys
945                 950                 955                 960
Phe Arg Glu Leu Ile Ile Glu Phe Ser Lys Met Ala Arg Asp Pro Gln
                965                 970                 975
Arg Tyr Leu Val Ile Gln Gly Asp Glu Arg Met His Leu Pro Ser Pro
            980                 985                 990
Thr Asp Ser Asn Phe Tyr Arg Ala Leu Met Asp Glu Glu Asp Met Asp
        995                 1000                1005
Asp Val Val Asp Ala Asp Glu Tyr Leu Ile Pro Gln Gln Gly Phe Phe
    1010                1015                1020
Ser Ser Pro Ser Thr Ser Arg Thr Pro Leu Leu Ser Ser Leu Ser Ala
1025                1030                1035                1040
Thr Ser Asn Asn Ser Thr Val Ala Cys Ile Asp Arg Asn Gly Leu Gln

                  1045                1050                1055
Ser Cys Pro Ile Lys Glu Asp Ser Phe Leu Gln Arg Tyr Ser Ser Asp
            1060                1065                1070
Pro Thr Gly Ala Leu Thr Glu Asp Ser Ile Asp Asp Thr Phe Leu Pro
        1075                1080                1085
Val Pro Glu Tyr Ile Asn Gln Ser Val Pro Lys Arg Pro Ala Gly Ser
    1090                1095                1100
Val Gln Asn Pro Val Tyr His Asn Gln Pro Leu Asn Pro Ala Pro Ser
1105                1110                1115                1120
Arg Asp Pro His Tyr Gln Asp Pro His Ser Thr Ala Val Gly Asn Pro
                1125                1130                1135
Glu Tyr Leu Asn Thr Val Gln Pro Thr Cys Val Asn Ser Thr Phe Asp
            1140                1145                1150
Ser Pro Ala His Trp Ala Gln Lys Gly Ser His Gln Ile Ser Leu Asp
        1155                1160                1165
Asn Pro Asp Tyr Gln Gln Asp Phe Phe Pro Lys Glu Ala Lys Pro Asn
    1170                1175                1180
Gly Ile Phe Lys Gly Ser Thr Ala Glu Asn Ala Glu Tyr Leu Arg Val
1185                1190                1195                1200
Ala Pro Gln Ser Ser Glu Phe Ile Gly Ala
                1205                1210

<210> 387
<211> 1330
<212> DNA
<213> Homo sapiens

<400> 387
aggggactgg ggccaagagc cgggagcgcg ggcgcaaagg caccagggcc cgcccagggc      60
gccgcgcagc acggccttgg gggttctgcg ggccttcggg tgcgcgtctc gcctctagcc      120
atggggtccg cagcgttgga gatcctgggc ctggtgctgt gcctggtggg ctgggggggt      180
ctgatcctgg cgtgcgggct gcccatgtgg caggtgaccg ccttcctgga ccacaacatc      240
gtgacggcgc agaccacctg gaagggcctg tggatgtcgt gcgtggtgca gagcaccggg      300
cacatgcagt gcaaagtgta cgactcggtg ctggctctga gcaccgaggt gcaggcggcg      360
cgggcgctca ccgtgagcgc cgtgctgctg gcgttcgttg cgctcttcgt gaccctggcg      420
ggcgcgcagt gcaccacctg cgtggccccg ggcccggcca aggcgcgtgt ggccctcacg      480
ggaggcgtgc tctacctgtt ttgcgggctg ctggcgctcg tgccactctg ctggttcgcc      540
aacattgtcg tccgcgagtt ttacgacccg tctgtgcccg tgtcgcagaa gtacgagctg      600
ggcgcagcgc tgtacatcgg ctgggcggcc accgcgctgc tcatggtagg cggctgcctc      660
ttgtgctgcg gcgcctgggt ctgcaccggc cgtcccgacc tcagcttccc cgtgaagtac      720
tcagcgccgc ggcggcccac ggccaccggc gactacgaca agaagaacta cgtctgaggg      780
cgctgggcac ggccgggccc ctcctgccag ccacgcctgc gaggcgttgg ataagcctgg      840
ggagccccgc atggaccgcg gcttccgccg ggtagcgcgg cgcgcaggct cctcggaacg      900
tccggctctg cgccccgacg cggctcctgg atccgctcct gcctgcgccc gcagctgacc      960
ttctcctgcc actagcccgg ccctgccctt aacagacgga atgaagtttc cttttctgtg     1020
cgcggcgctg tttccatagg cagagcgggt gtcagactga ggatttcgct tcccctccaa     1080
gacgctgggg gtcttggctg ctgccttact tcccagaggc tcctgctgac ttcggagggg     1140
cggatgcaga gcccggggcc cccaccggaa gatgtgtaca gctggtcttt actccatcgg     1200
caggcccgag cccagggacc agtgacttgg cctggacctc ccggtctcac tccagcatct     1260
ccccaggcaa ggcttgtggg caccggagct tgagagaggg cgggagtggg aaggctaaga     1320
atctgcttag                                                            1330

<210> 388
<211> 218
<212> PRT
<213> Homo sapiens

<400> 388
Met Gly Ser Ala Ala Leu Glu Ile Leu Gly Leu Val Leu Cys Leu Val
1               5                   10                  15
Gly Trp Gly Gly Leu Ile Leu Ala Cys Gly Leu Pro Met Trp Gln Val
            20                  25                  30

Thr Ala Phe Leu Asp His Asn Ile Val Thr Ala Gln Thr Thr Trp Lys
        35                  40                  45
Gly Leu Trp Met Ser Cys Val Val Gln Ser Thr Gly His Met Gln Cys
    50                  55                  60
Lys Val Tyr Asp Ser Val Leu Ala Leu Ser Thr Glu Val Gln Ala Ala
65                  70                  75                  80
Arg Ala Leu Thr Val Ser Ala Val Leu Leu Ala Phe Val Ala Leu Phe
                85                  90                  95
Val Thr Leu Ala Gly Ala Gln Cys Thr Thr Cys Val Ala Pro Gly Pro
            100                 105                 110
Ala Lys Ala Arg Val Ala Leu Thr Gly Gly Val Leu Tyr Leu Phe Cys
        115                 120                 125
Gly Leu Leu Ala Leu Val Pro Leu Cys Trp Phe Ala Asn Ile Val Val
    130                 135                 140
Arg Glu Phe Tyr Asp Pro Ser Val Pro Val Ser Gln Lys Tyr Glu Leu
145                 150                 155                 160
Gly Ala Ala Leu Tyr Ile Gly Trp Ala Ala Thr Ala Leu Leu Met Val
                165                 170                 175
Gly Gly Cys Leu Leu Cys Cys Gly Ala Trp Val Cys Thr Gly Arg Pro
            180                 185                 190
Asp Leu Ser Phe Pro Val Lys Tyr Ser Ala Pro Arg Arg Pro Thr Ala
        195                 200                 205
Thr Gly Asp Tyr Asp Lys Lys Asn Tyr Val
    210                 215

<210> 389
<211> 1397
<212> DNA
<213> Homo sapiens

<400> 389
ttaccattgt gttgggctgc gagaagacga cagaaggggg acccgcctct tggcagccag      60
ctgagaaggc gccccgggga gggggaaact gacatcccat ctagagccgt ccctcctctt     120
cctcccctcc cgactctctg ctcctttccc gccccagaag ttcaaggccc cccggcctcc     180
tgcgctcctg ccgccgggac cctcgacctc ctcagagcag ccggctgccg ccccgggaag     240
atggcgagga ggagccgcca ccgcctcctc ctgctgctgc tgcgctacct ggtggtcgcc     300
ctgggctatc ataaggccta tgggttttct gccccaaaag accaacaggt agtcacagca     360
gtagagtacc aagaggctat tttagcctgc aaaaccccaa agaagactgt ttcctccaga     420
ttagagtgga agaaactggg tcggagtgtc tcctttgtct actatcaaca gactcttcaa     480
ggtgatttta aaaatcgagc tgagatgata gatttcaata tccggatcaa aaatgtgaca     540
agaagtgatg cggggaaata tcgttgtgaa gttagtgccc catctgagca aggccaaaac     600
ctggaagagg atacagtcac tctggaagta ttagtggctc cagcagttcc atcatgtgaa     660
gtaccctctt ctgctctgag tggaactgtg gtagagctac gatgtcaaga caaagaaggg     720
aatccagctc ctgaatacac atggtttaag gatggcatcc gtttgctaga aaatcccaga     780
cttggctccc aaagcaccaa cagctcatac acaatgaata caaaaactgg aactctgcaa     840
tttaatactg tttccaaact ggacactgga gaatattcct gtgaagcccg caattctgtt     900
ggatatcgca ggtgtcctgg gaaacgaatg caagtagatg atctcaacat aagtggcatc     960
atagcagccg tagtagttgt ggccttagtg atttccgttt gtggccttgg tgtatgctat    1020
gctcagagga aaggctactt ttcaaaagaa acctccttcc agaagagtaa ttcttcatct    1080
aaagccacga caatgagtga aaatgatttc aagcacacaa aatcctttat aatttaaaga    1140
ctccacttta gagatacacc aaagccaccg ttgttacaca agttattaaa ctattataaa    1200
actctgcttt gtccgacatt tgcaaagagg tacacgagga aatggaattg gtatttcatt    1260
ttaattttca tgactactaa ctcacctgaa cttgctattt taaamaaata gttctgtcga    1320
cacctaaaat ataatctggc ttcttgtgtc tggactaagt taaaagaatt aaaatacttt    1380
gtaatgtcaa aaaaaaa                                                   1397

<210> 390
<211> 298
<212> PRT
<213> Homo sapiens

<400> 390

Met Ala Arg Arg Ser Arg His Arg Leu Leu Leu Leu Leu Leu Arg Tyr
1               5                   10                  15
Leu Val Val Ala Leu Gly Tyr His Lys Ala Tyr Gly Phe Ser Ala Pro
            20                  25                  30
Lys Asp Gln Gln Val Val Thr Ala Val Glu Tyr Gln Glu Ala Ile Leu
        35                  40                  45
Ala Cys Lys Thr Pro Lys Lys Thr Val Ser Ser Arg Leu Glu Trp Lys
    50                  55                  60
Lys Leu Gly Arg Ser Val Ser Phe Val Tyr Tyr Gln Gln Thr Leu Gln
65                  70                  75                  80
Gly Asp Phe Lys Asn Arg Ala Glu Met Ile Asp Phe Asn Ile Arg Ile
                85                  90                  95
Lys Asn Val Thr Arg Ser Asp Ala Gly Lys Tyr Arg Cys Glu Val Ser
            100                 105                 110
Ala Pro Ser Glu Gln Gly Gln Asn Leu Glu Glu Asp Thr Val Thr Leu
        115                 120                 125
Glu Val Leu Val Ala Pro Ala Val Pro Ser Cys Glu Val Pro Ser Ser
    130                 135                 140
Ala Leu Ser Gly Thr Val Val Glu Leu Arg Cys Gln Asp Lys Glu Gly
145                 150                 155                 160
Asn Pro Ala Pro Glu Tyr Thr Trp Phe Lys Asp Gly Ile Arg Leu Leu
                165                 170                 175
Glu Asn Pro Arg Leu Gly Ser Gln Ser Thr Asn Ser Ser Tyr Thr Met
            180                 185                 190
Asn Thr Lys Thr Gly Thr Leu Gln Phe Asn Thr Val Ser Lys Leu Asp
        195                 200                 205
Thr Gly Glu Tyr Ser Cys Glu Ala Arg Asn Ser Val Gly Tyr Arg Arg
    210                 215                 220
Cys Pro Gly Lys Arg Met Gln Val Asp Asp Leu Asn Ile Ser Gly Ile
225                 230                 235                 240
Ile Ala Ala Val Val Val Val Ala Leu Val Ile Ser Val Cys Gly Leu
                245                 250                 255
Gly Val Cys Tyr Ala Gln Arg Lys Gly Tyr Phe Ser Lys Glu Thr Ser
            260                 265                 270
Phe Gln Lys Ser Asn Ser Ser Ser Lys Ala Thr Thr Met Ser Glu Asn
        275                 280                 285
Asp Phe Lys His Thr Lys Ser Phe Ile Ile
    290                 295

<210> 391
<211> 1653
<212> DNA
<213> Homo sapiens

<220>
<221> modified_base
<222> (1)...(1653)
<223> n = g, a, c or t

<400> 391
atgacaggtg acaagggtcc ccaaaggcta agcgggtcca gctatggttc catctccagc      60
ccgaccagcc cgaccagccc agggccacag caagcacctc ccagagagac ctacctgagt     120
gagaagatcc ccatcccaga cacaaaaccg ggcaccttca ggctgcggaa gctatgggcc     180
ttcacggggc ctggcttcct catgagcatt gctttcctgg acccaggaaa catcgagtca     240
gatcttcagc tnggnccngt ggcgggattc aaacttctct gggtgctgct ctgggccacc     300
gtgttgggct tgctctgcca gcgactggct gcacgtctgg gcgtggtgac aggcaaggac     360
ttgggcgagg tctgccatct ctactaccct aaggtgcccc gcaccgtcct ctggctgacc     420
atcgagctag ccattgtggg ctccgacatg caggaagtca tcggcacggc cattgcattc     480
aatctgctct cagctggacg aatcccactc tggggtggcg tcctcatcac catcgtggac     540
accttcttct tcctcttcct cgataactac gggctgcgga agctggaagc tttttttgga     600
ctccttataa ccattatggc cttgaccttt ggctatgagt atgtggtggc gcgtcctgag     660
cagggagcgc ttcttcgggg cctgttcctg ccctcgtgcc cgggctgcgg ccaccccgag     720
ctgctgcagg cggtgggcat tgttggcgcc atcatcatgc cccacaacat ctacctgcac     780

tcggccctgg tcaagtctcg agagatagac cgggcccgcc gagtcgacat cagagaagcc 840
aacatgtact tcctgattga ggccaccatc gccctgtccg tctcctttat catcaacctc 900
tttgtcatgg ctgcatttgg gcaggccttc taccagaaaa ccaagcaggc tgcgttcaac 960
atctgtgcca acagcagcct ccacgactac gctaagatct tccccatgaa caacgccacc 1020
gtggccgtgg acatttacca ggggggcgtg atcctgggct gcctgttcgg ccccgcggcc 1080
ctctacatct gggccatagg tctcctggcg gctgggcaga gctccaccat gacgggcacc 1140
tacgcgggac agttcgtgat ggagggcttc ctgaggctgc ggtggtcaag cttcgcccgt 1200
gtcctcctca cccgctcctg cgccatcctg cccaccgtgc tcgtggctgt cttccgggac 1260
ctgagggact tgtcgggcct caatgatctg ctcaacgtgc tgcagagcct gctgctcccg 1320
gttgccgtgc tgcccatcct cacgttcacc agcatgccca ccctcatgca ggagtttgcc 1380
aatggcctgc tgaacaaggt cgtcacctct tccatcatgg tgctagtctg caccatcaac 1440
ctctacttcg tggtcagcta tctgcccagc ctgccccacc ctgcctactt cggccttgca 1500
gccttgctgg ccgcagccta cctgggcctc agcacctacc tggtctggac ctgttgcctt 1560
gcccacggag ccacctttct ggcccacagc tcccaccacc acttcctgta tgggctcctt 1620
gaagaggacc acaaagggga gacctctggc tag 1653

<210> 392
<211> 550
<212> PRT
<213> Homo sapiens

<400> 392
Met Thr Gly Asp Lys Gly Pro Gln Arg Leu Ser Gly Ser Ser Tyr Gly
1               5                   10                  15
Ser Ile Ser Ser Pro Thr Ser Pro Thr Ser Pro Gly Pro Gln Gln Ala
            20                  25                  30
Pro Pro Arg Glu Thr Tyr Leu Ser Glu Lys Ile Pro Ile Pro Asp Thr
        35                  40                  45
Lys Pro Gly Thr Phe Arg Leu Arg Lys Leu Trp Ala Phe Thr Gly Pro
    50                  55                  60
Gly Phe Leu Met Ser Ile Ala Phe Leu Asp Pro Gly Asn Ile Glu Ser
65                  70                  75                  80
Asp Leu Gln Leu Gly Pro Val Ala Gly Phe Lys Leu Leu Trp Val Leu
                85                  90                  95
Leu Trp Ala Thr Val Leu Gly Leu Leu Cys Gln Arg Leu Ala Ala Arg
            100                 105                 110
Leu Gly Val Val Thr Gly Lys Asp Leu Gly Glu Val Cys His Leu Tyr
        115                 120                 125
Tyr Pro Lys Val Pro Arg Thr Val Leu Trp Leu Thr Ile Glu Leu Ala
    130                 135                 140
Ile Val Gly Ser Asp Met Gln Glu Val Ile Gly Thr Ala Ile Ala Phe
145                 150                 155                 160
Asn Leu Leu Ser Ala Gly Arg Ile Pro Leu Trp Gly Gly Val Leu Ile
                165                 170                 175
Thr Ile Val Asp Thr Phe Phe Phe Leu Phe Leu Asp Asn Tyr Gly Leu
            180                 185                 190
Arg Lys Leu Glu Ala Phe Phe Gly Leu Leu Ile Thr Ile Met Ala Leu
        195                 200                 205
Thr Phe Gly Tyr Glu Tyr Val Val Ala Arg Pro Glu Gln Gly Ala Leu
    210                 215                 220
Leu Arg Gly Leu Phe Leu Pro Ser Cys Pro Gly Cys Gly His Pro Glu
225                 230                 235                 240
Leu Leu Gln Ala Val Gly Ile Val Gly Ala Ile Ile Met Pro His Asn
                245                 250                 255
Ile Tyr Leu His Ser Ala Leu Val Lys Ser Arg Glu Ile Asp Arg Ala
            260                 265                 270
Arg Arg Val Asp Ile Arg Glu Ala Asn Met Tyr Phe Leu Ile Glu Ala
        275                 280                 285
Thr Ile Ala Leu Ser Val Ser Phe Ile Ile Asn Leu Phe Val Met Ala
    290                 295                 300
Ala Phe Gly Gln Ala Phe Tyr Gln Lys Thr Lys Gln Ala Ala Phe Asn
305                 310                 315                 320

Ile Cys Ala Asn Ser Ser Leu His Asp Tyr Ala Lys Ile Phe Pro Met
                325                 330                 335
Asn Asn Ala Thr Val Ala Val Asp Ile Tyr Gln Gly Gly Val Ile Leu
            340                 345                 350
Gly Cys Leu Phe Gly Pro Ala Ala Leu Tyr Ile Trp Ala Ile Gly Leu
        355                 360                 365
Leu Ala Ala Gly Gln Ser Ser Thr Met Thr Gly Thr Tyr Ala Gly Gln
    370                 375                 380
Phe Val Met Glu Gly Phe Leu Arg Leu Arg Trp Ser Ser Phe Ala Arg
385                 390                 395                 400
Val Leu Leu Thr Arg Ser Cys Ala Ile Leu Pro Thr Val Leu Val Ala
                405                 410                 415
Val Phe Arg Asp Leu Arg Asp Leu Ser Gly Leu Asn Asp Leu Leu Asn
            420                 425                 430
Val Leu Gln Ser Leu Leu Leu Pro Val Ala Val Leu Pro Ile Leu Thr
        435                 440                 445
Phe Thr Ser Met Pro Thr Leu Met Gln Glu Phe Ala Asn Gly Leu Leu
    450                 455                 460
Asn Lys Val Val Thr Ser Ser Ile Met Val Leu Val Cys Thr Ile Asn
465                 470                 475                 480
Leu Tyr Phe Val Val Ser Tyr Leu Pro Ser Leu Pro His Pro Ala Tyr
                485                 490                 495
Phe Gly Leu Ala Ala Leu Leu Ala Ala Ala Tyr Leu Gly Leu Ser Thr
            500                 505                 510
Tyr Leu Val Trp Thr Cys Cys Leu Ala His Gly Ala Thr Phe Leu Ala
        515                 520                 525
His Ser Ser His His His Phe Leu Tyr Gly Leu Leu Glu Glu Asp His
    530                 535                 540
Lys Gly Glu Thr Ser Gly
545                 550

<210> 393
<211> 1609
<212> DNA
<213> Homo sapiens

<400> 393
gctcgctggg ccgctgctcc cgggtgtccc aggcccggcc ggtgcgcaga gcatggcggg      60
tgcgggcccg aagcggcgcg cgctagcggc gccggcggcc gaggagaagg aagaggcgcg     120
ggagaagatg ctggccgcca agagcgcgga cggctcggcg ccggcaggcg agggcgaggg     180
cgtgaccctg cagcggaaca tcacgctgct caacggcgtg gccatcatcg tggggaccat     240
tatcggctcg ggcatcttcg tgacgcccac gggcgtgctc aaggaggcag gctcgccggg     300
gctggcgctg gtggtgtggg ccgcgtgcgg cgtcttctcc atcgtgggcg cgctctgcta     360
cgcggagctc ggcaccacca tctccaaatc gggcggcgac tacgcctaca tgctggaggt     420
ctacggctcg ctgcccgcct tcctcaagct ctggatcgag ctgctcatca tccggccttc     480
atcgcagtac atcgtggccc tggtcttcgc cacctacctg ctcaagccgc tcttccccac     540
ctgcccggtg cccgaggagg cagccaagct cgtggcctgc ctgtgcgtgc tgctgctcac     600
ggccgtgaac tgctacagcg tgaaggccgc cacccgggtc caggatgcct ttgccgccgc     660
caagctcctg gccctggccc tgatcatcct gctgggcttc gtccagatcg gaaagggtga     720
tgtgtccaat ctagatccca agttctcatt tgaaggcacc aaactggatg tggggaacat     780
tgtgctggca ttatacagcg gcctctttgc ctatggagga tggaattact tgaatttcgt     840
cacagaggaa atgatcaacc cctacagaaa cctgcccctg gccatcatca tctccctgcc     900
catcgtgacg ctggtgtacg tgctgaccaa cctggcctac ttcaccaccc tgtccaccga     960
gcagatgctg tcgtccgagg ccgtggccgt ggacttcggg aactatcacc tgggcgtcat    1020
gtcctggatc atccccgtct tcgtgggcct gtcctgcttt ggctccgtca atgggtccct    1080
gttcacatcc tccaggctct tcttcgtggg gtcccgggaa ggccacctgc cctccatcct    1140
ctccatgatc cacccacagc tcctcacccc cgtgccgtcc ctcgtgttca cgtgtgtgat    1200
gacgctgctc tacgccttct ccaaggacat cttctccgtc atcaacttct tcagcttctt    1260
caactggctc tgcgtggccc tggccatcat cggcatgatc tggctgcgcc acagaaagcc    1320
tgagcttgag cggcccatca aggtgaacct ggccctgcct gtgttcttca tcctggcctg    1380
cctcttcctg atcgccgtct ccttctggaa gacacccgtg gagtgtggca tcggcttcac    1440
catcatcctc agcgggctgc ccgtctactt cttcggggtc tggtggaaaa acaagcccaa    1500
gtggctcctc cagggcatct tctccacgac cgtcctgtgt cagaagctca tgcaggtggt    1560

cccccaggag acatagccag gaggccgagt ggctgccgga ggagcatgc 1609

<210> 394
<211> 507
<212> PRT
<213> Homo sapiens

<400> 394
Met Ala Gly Ala Gly Pro Lys Arg Arg Ala Leu Ala Ala Pro Ala Ala
1               5                   10                  15
Glu Glu Lys Glu Glu Ala Arg Glu Lys Met Leu Ala Ala Lys Ser Ala
            20                  25                  30
Asp Gly Ser Ala Pro Ala Gly Glu Gly Glu Gly Val Thr Leu Gln Arg
        35                  40                  45
Asn Ile Thr Leu Leu Asn Gly Val Ala Ile Ile Val Gly Thr Ile Ile
    50                  55                  60
Gly Ser Gly Ile Phe Val Thr Pro Thr Gly Val Leu Lys Glu Ala Gly
65                  70                  75                  80
Ser Pro Gly Leu Ala Leu Val Val Trp Ala Ala Cys Gly Val Phe Ser
                85                  90                  95
Ile Val Gly Ala Leu Cys Tyr Ala Glu Leu Gly Thr Thr Ile Ser Lys
            100                 105                 110
Ser Gly Gly Asp Tyr Ala Tyr Met Leu Glu Val Tyr Gly Ser Leu Pro
        115                 120                 125
Ala Phe Leu Lys Leu Trp Ile Glu Leu Leu Ile Ile Arg Pro Ser Ser
    130                 135                 140
Gln Tyr Ile Val Ala Leu Val Phe Ala Thr Tyr Leu Leu Lys Pro Leu
145                 150                 155                 160
Phe Pro Thr Cys Pro Val Pro Glu Glu Ala Ala Lys Leu Val Ala Cys
                165                 170                 175
Leu Cys Val Leu Leu Leu Thr Ala Val Asn Cys Tyr Ser Val Lys Ala
            180                 185                 190
Ala Thr Arg Val Gln Asp Ala Phe Ala Ala Ala Lys Leu Leu Ala Leu
        195                 200                 205
Ala Leu Ile Ile Leu Leu Gly Phe Val Gln Ile Gly Lys Gly Asp Val
    210                 215                 220
Ser Asn Leu Asp Pro Lys Phe Ser Phe Glu Gly Thr Lys Leu Asp Val
225                 230                 235                 240
Gly Asn Ile Val Leu Ala Leu Tyr Ser Gly Leu Phe Ala Tyr Gly Gly
                245                 250                 255
Trp Asn Tyr Leu Asn Phe Val Thr Glu Glu Met Ile Asn Pro Tyr Arg
            260                 265                 270
Asn Leu Pro Leu Ala Ile Ile Ile Ser Leu Pro Ile Val Thr Leu Val
        275                 280                 285
Tyr Val Leu Thr Asn Leu Ala Tyr Phe Thr Thr Leu Ser Thr Glu Gln
    290                 295                 300
Met Leu Ser Ser Glu Ala Val Ala Val Asp Phe Gly Asn Tyr His Leu
305                 310                 315                 320
Gly Val Met Ser Trp Ile Ile Pro Val Phe Val Gly Leu Ser Cys Phe
                325                 330                 335
Gly Ser Val Asn Gly Ser Leu Phe Thr Ser Ser Arg Leu Phe Phe Val
            340                 345                 350
Gly Ser Arg Glu Gly His Leu Pro Ser Ile Leu Ser Met Ile His Pro
        355                 360                 365
Gln Leu Leu Thr Pro Val Pro Ser Leu Val Phe Thr Cys Val Met Thr
    370                 375                 380
Leu Leu Tyr Ala Phe Ser Lys Asp Ile Phe Ser Val Ile Asn Phe Phe
385                 390                 395                 400
Ser Phe Phe Asn Trp Leu Cys Val Ala Leu Ala Ile Ile Gly Met Ile
                405                 410                 415
Trp Leu Arg His Arg Lys Pro Glu Leu Glu Arg Pro Ile Lys Val Asn
            420                 425                 430
Leu Ala Leu Pro Val Phe Phe Ile Leu Ala Cys Leu Phe Leu Ile Ala

435                 440                 445
Val Ser Phe Trp Lys Thr Pro Val Glu Cys Gly Ile Gly Phe Thr Ile
    450                 455                 460
Ile Leu Ser Gly Leu Pro Val Tyr Phe Phe Gly Val Trp Trp Lys Asn
465                 470                 475                 480
Lys Pro Lys Trp Leu Leu Gln Gly Ile Phe Ser Thr Thr Val Leu Cys
                485                 490                 495
Gln Lys Leu Met Gln Val Val Pro Gln Glu Thr
            500                 505

<210> 395
<211> 2943
<212> DNA
<213> Homo sapiens

<400> 395
atgccggctg agctgctgct gctgctgatt gttgccttcg ccagccccag ctgccaggtg      60
ctctcatcac tgcgcatggc tgcaatcctg gatgatcaga cagtgtgtgg ccgcggtgag     120
cgtctggcct tggccttggc ccgggagcag atcaacggga tcatcgaggt cccagccaag     180
gcccgagtgg aagtagacat ctttgagctg cagcgggaca gccagtacga gaccacggac     240
accatgtgtc agatcttacc caaaggggtt gtgtctgtcc ttgggccctc ctctagccca     300
gcatctgcct ccaccgtgag ccatatctgt ggagagaagg agatccccca catcaaggtg     360
ggtcccgagg agacaccccg ccttcagtac cttcgcttcg cgtctgtcag cctgtacccc     420
agtaacgagg acgtcagctt ggcggtctcc cgaatcctca agtccttcaa ctacccctcg     480
gccagcctca tctgcgccaa ggctgagtgc ctgctgcgat tggaggaact ggtgcgtggc     540
ttcctcatct ccaaggagac gctgtcagtg aggatgttgg acgacagccg ggaccccaca     600
ccactgctca aggagatccg tgatgacaag gtgtccacca tcatcatcga cgccaacgcc     660
tccatctccc acctcatcct ccgtaaggcc tcggaactgg gaatgacctc agcgttttac     720
aagtacatcc tcaccaccat ggacttcccc atcctgcatc tggacggtat tgtggaggac     780
tcctccaaca tcctgggctt ctccatgttc aacacgtccc accccttcta ccctgagttt     840
gtccgcagcc tcaacatgtc ctggagggag aactgtgaag ccagcaccta cctgggccct     900
gcgctgtcag ccgccctgat gtttgacgcc gtgcacgtgg tggtgagcgc tgtccgagag     960
ctgaaccgca gccaggagat cggtgtgaag cctctggcct gtacatcggc caacatttgg    1020
ccccacggga ccagcctcat gaactacctg cgcatggtag agtatgatgg gctgaccggg    1080
cgggtcgagt tcaacagcaa agggcagaga accaactaca ccctgcgcat cctagaaaag    1140
tcccggcagg gccaccgtga gattggggtg tggtactcta accgcaccct ggccatgaat    1200
gccaccaccc tggacatcaa cctgtcgcag acactggcca acaagaccct ggtggtcaca    1260
accatcctgg agaacccata cgtcatgcgc cggcccaact tccagggcct gtcggggaac    1320
gaacgcttcg agggcttctg cgtggacatg ctgcgggagc tggccgagct gctgccgttc    1380
ccgtaccgcc tgcggttggt ggaggatggg ctgtacgggg cgcccgagcc caacggctcc    1440
tggacgggca tggttggcga gctcatcaac cggaaggcag acctggctgt ggccgccttc    1500
accatcacag ctgagcggga gaaggtcatc gacttttcca agccctttat gaccctgggg    1560
atcagcatcc tctaccgagt gcacatgggc cgcaagcctg gctacttctc cttcctggac    1620
cccttctccc ctgctgtgtg gctcttcatg cttcttgcct acctggctgt cagctgcgtc    1680
ctgtttctgg ctgccaggct gagcccctat gagtggtata acccacaccc atgcctgcgg    1740
gcacgccccc acatcctgga gaaccagtac acgctgggca acagcctgtg gtttcccgtg    1800
gggggcttca tgcagcaggg ctcggagatc atgccccggg cgctgtccac gcgctgtgtc    1860
agcggagtct ggtgggcctt caccttgatc atcatctcct cctacacggc caacctggcc    1920
gccttcctca ccgtgcagcg catggaggtg cctgtggagt cggccgatga cctggcagat    1980
cagaccaaca tcgagtatgg caccatccac gccggctcca ccatgacctt cttccagaat    2040
tcacggtacc aaacgtacca gcgcatgtgg aactacatgc agtcgaagca gcccagcgtg    2100
ttcgtcaaga gcacagaaga gggcattgcc gccgtcctca actcccgcta cgccttcctg    2160
ctcgagtcca ccatgaacga ataccaccgg cgcctcaact gcaacctcac ccagatcggg    2220
ggactcctcg acaccaaggg ctacggcatt ggcatgccgc tgggctcccc gttccgggat    2280
gagatcacac tggccatcct gcagcttcag gagaacaacc ggctggagat cctgaagcgc    2340
aagtggtggg aggggggccg gtgccccaag gaggaggacc atcgagctaa aggtttgggc    2400
atggagaaca ttggtggcat ttttatcgtg ctcatctgtg gcctcatcat tgctgtcttc    2460
gtggcggtca tggaattcat atggtccaca cggaggtcag ctgagtccga ggaggtgtcg    2520
gtgtgccagg agatgctgca ggagctgcgc cacgccgttt cttgccgcaa gacgtcgcgt    2580
tcccgccggc gccgacgccc gggcggcccg agccgggccc tgctgtcact gcgcgcggtc    2640
cgcgagatgc gcctcagcaa cggcaagctc tactcggccg gcgcgggcgg ggatgcgggc    2700
agcgcgcacg ggggcccgca gcgcctcctg gacgacccgg ggccccccag cggagcccga    2760

cccgccgccc ccaccccctg cacccacgtg cgcgtctgcc aggagtgccg gcgcatccag 2820
gcgctgcggg cctcgggggc cggcgcgcct ccgcgtggcc tgggcgtccc cgccgaagcc 2880
accagcccgc cccggccgcg gcctggcccc gccggccccc gggagctggc ggagcacgag 2940
tga 2943

<210> 396
<211> 980
<212> PRT
<213> Homo sapiens

<400> 396

<210> 397
<211> 2241
<212> DNA
<213> Homo sapiens

<400> 397
atctttgctg caaaggctgg gtatcggctg tgctcagcaa agcgtcaact cgtgcaagaa 60
cttagcagga atagttctgg ctaaggttag gaggctgcca ccaaagtctc ttttttgttc 120
ctctgcttct cccgtttgcc tccttatcat gagatctttt tgctaagctg gcagaaagat 180
tgcatagtca gtgcttccag ctctgctccc acctgatcct gcactgtcct ctggtccctg 240
aatgaatgaa ctctgatacc caatcttgtc tcgagccttc tctatgccac tcatggctcc 300
tcttctgctc tttccatctt tttgctgaga gttctgagct ctgtacttcc tcttggccca 360
tctcacttcc tgaaacaccc ctgaagaggg ttgcttatct tgatggaact caaaaagcca 420
aaaagctgca ggcagaggcg ttgaggacat ctgtttgggg aactaagagc agcagcactt 480
tcagattcag tccatataga gctgtcctac agcattctgg aaacttgagg atgtgcggtg 540
cataaagggg ctggaagtga cccacctgtg atgagccctt tctaaggaga agggtttcca 600
agagatcacc ccaccagaaa agggtaggaa tgagcaagtt gggaatttta gactgtcact 660
gcacatggac ctctgggaag acgtctggcg agagctaggc ccactggccc tacagacgga 720
tcttgctggc tcacctgtcc ctgtggaggt tcccctggga aggcaagatg cccaacaaca 780
gcactgctct gtcattggcc aatgttacct acatcaccat ggaaattttc attggactct 840
gcgccatagt gggcaacgtg ctggtcatct gcgtggtcaa gctgaacccc agcctgcaga 900
ccaccacctt ctatttcatt gtctctctag ccctggctga cattgctgtt ggggtgctgg 960
tcatgccttt ggccattgtt gtcagcctgg gcatcacaat ccacttctac agctgccttt 1020
ttatgacttg cctactgctt atctttaccc acgcctccat catgtccttg ctggccatcg 1080
ctgtggaccg atacttgcgg gtcaagctta ccgtcagata caagagggtc accactcaca 1140
gaagaatatg gctggccctg ggcctttgct ggctggtgtc attcctggtg ggattgaccc 1200
ccatgtttgg ctggaacatg aaactgacct cagagtacca cagaaatgtc accttccttt 1260
catgccaatt tgtttccgtc atgagaatgg actacatggt atacttcagc ttcctcacct 1320
ggattttcat ccccctggtt gtcatgtgcg ccatctatct tgacatcttt tacatcattc 1380
ggaacaaact cagtctgaac ttatctaact ccaaagagac aggtgcattt tatggacggg 1440
agttcaagac ggctaagtcc ttgtttctgg ttcttttctt gtttgctctg tcatggctgc 1500
ctttatctat catcaactgc atcatctact ttaatggtga ggtaccacag cttgtgctgt 1560
acatgggcat cctgctgtcc catgccaact ccatgatgaa ccctatcgtc tatgcctata 1620
aaataaagaa gttcaaggaa acctaccttt tgatcctcaa agcctgtgtg gtctgccatc 1680
cctctgattc tttggacaca agcattgaga agaattctga gtagttatcc atcagagatg 1740
actctgtctc attgaccttc agattcccca tcaacaaaca cttgagggcc tgtatgcctg 1800
ggccaaggga tttttacatc cttgattact tccactgagg tgggagcatc tccagtgctc 1860
cccaattata tctcccccac tccactactc tcttcctcca cttcattttt cctttgtcct 1920
ttctctctaa ttcagtgttt tggaggcctg acttggggac aacgtattat tgatattatt 1980
gtctgttttc cttcttccca atagaagaat aagtcatgga gcctgaaggg tgcctagttg 2040
acttactgac aaaaggctct agttgggctg aacatgtgtg tggtggtgac tcatttccat 2100
gccattgtgg aattgagcag agaacctgct ctcggaggat gcctagaaga tgttgggaac 2160
agaagaaata aactgagttt aagggggact taaactgctg aattcacctg tggatgtttt 2220
tgagtaaata aaagctaata g 2241

<210> 398
<211> 318
<212> PRT
<213> Homo sapiens

<400> 398
Met Pro Asn Asn Ser Thr Ala Leu Ser Leu Ala Asn Val Thr Tyr Ile
1               5                   10                  15
Thr Met Glu Ile Phe Ile Gly Leu Cys Ala Ile Val Gly Asn Val Leu
            20                  25                  30
Val Ile Cys Val Val Lys Leu Asn Pro Ser Leu Gln Thr Thr Thr Phe
        35                  40                  45

Tyr Phe Ile Val Ser Leu Ala Leu Ala Asp Ile Ala Val Gly Val Leu
    50                  55                  60
Val Met Pro Leu Ala Ile Val Val Ser Leu Gly Ile Thr Ile His Phe
65                  70                  75                  80
Tyr Ser Cys Leu Phe Met Thr Cys Leu Leu Leu Ile Phe Thr His Ala
                85                  90                  95
Ser Ile Met Ser Leu Leu Ala Ile Ala Val Asp Arg Tyr Leu Arg Val
            100                 105                 110
Lys Leu Thr Val Arg Tyr Lys Arg Val Thr Thr His Arg Arg Ile Trp
        115                 120                 125
Leu Ala Leu Gly Leu Cys Trp Leu Val Ser Phe Leu Val Gly Leu Thr
    130                 135                 140
Pro Met Phe Gly Trp Asn Met Lys Leu Thr Ser Glu Tyr His Arg Asn
145                 150                 155                 160
Val Thr Phe Leu Ser Cys Gln Phe Val Ser Val Met Arg Met Asp Tyr
                165                 170                 175
Met Val Tyr Phe Ser Phe Leu Thr Trp Ile Phe Ile Pro Leu Val Val
            180                 185                 190
Met Cys Ala Ile Tyr Leu Asp Ile Phe Tyr Ile Ile Arg Asn Lys Leu
        195                 200                 205
Ser Leu Asn Leu Ser Asn Ser Lys Glu Thr Gly Ala Phe Tyr Gly Arg
    210                 215                 220
Glu Phe Lys Thr Ala Lys Ser Leu Phe Leu Val Leu Phe Leu Phe Ala
225                 230                 235                 240
Leu Ser Trp Leu Pro Leu Ser Ile Ile Asn Cys Ile Ile Tyr Phe Asn
                245                 250                 255
Gly Glu Val Pro Gln Leu Val Leu Tyr Met Gly Ile Leu Leu Ser His
            260                 265                 270
Ala Asn Ser Met Met Asn Pro Ile Val Tyr Ala Tyr Lys Ile Lys Lys
        275                 280                 285
Phe Lys Glu Thr Tyr Leu Leu Ile Leu Lys Ala Cys Val Val Cys His
    290                 295                 300
Pro Ser Asp Ser Leu Asp Thr Ser Ile Glu Lys Asn Ser Glu
305                 310                 315

<210> 399
<211> 3692
<212> DNA
<213> Homo sapiens

<400> 399
ggctgcccga gcgagcgttc ggacctcgca ccccgcgcgc cccgcgccgc cgccgccgcc      60
ggcttttgtt gtctccgcct cctcggccgc cgccgcctct ggaccgcgag ccgcgcgcgc     120
cgggaccttg gctctgccct tcgcgggcgg gaactgcgca ggacccggcc aggatccgag     180
agaggcgcgg gcgggtggcc gggggcgccg ccggccccgc catggagctc cgggcccgag     240
gctggtggct gctatgtgcg gccgcagcgc tggtcgcctg cgcccgcggg gacccggcca     300
gcaagagccg gagctgcggc gaggtccgcc agatctacgg agccaagggc ttcagcctga     360
gcgacgtgcc ccaggcggag atctcgggtg agcacctgcg gatctgtccc cagggctaca     420
cctgctgcac cagcgagatg gaggagaacc tggccaaccg cagccatgcc gagctggaga     480
ccgcgctccg ggacagcagc cgcgtcctgc aggccatgct tgccacccag ctgcgcagct     540
tcgatgacca cttccagcac ctgctgaacg actcggagcg gacgctgcag gccaccttcc     600
ccggcgcctt cggagagctg tacacgcaga acgcgagggc cttccgggac ctgtactcag     660
agctgcgcct gtactaccgc ggtgccaacc tgcacctgga ggagacgctg gccgagttct     720
gggcccgcct gctcgagcgc ctcttcaagc agctgcaccc ccagctgctg ctgcctgatg     780
actacctgga ctgcctgggc aagcaggccg aggcgctgcg gcccttcggg gaggccccga     840

gagagctgcg cctgcgggcc acccgtgcct tcgtggctgc tcgctccttt gtgcagggcc 900
tgggcgtggc cagcgacgtg gtccggaaag tggctcaggt ccccctgggc ccggagtgct 960
cgagagctgt catgaagctg gtctactgtg ctcactgcct gggagtcccc ggcgccaggc 1020
cctgccctga ctattgccga aatgtgctca agggctgcct tgccaaccag gccgacctgg 1080
acgccgagtg gaggaacctc ctggactcca tggtgctcat caccgacaag ttctggggta 1140
catcgggtgt ggagagtgtc atcggcagcg tgcacacgtg gctggcggag gccatcaacg 1200
ccctccagga caacagggac acgctcacgg ccaaggtcat ccagggctgc gggaacccca 1260
aggtcaaccc ccagggccct gggcctgagg agaagcggcg ccggggcaag ctggccccgc 1320
gggagaggcc accttcaggc acgctggaga agctggtctc tgaagccaag gcccagctcc 1380
gcgacgtcca ggacttctgg atcagcctcc cagggacact gtgcagtgag aagatggccc 1440
tgagcactgc cagtgatgac cgctgctgga acgggatggc cagaggccgg tacctccccg 1500
aggtcatggg tgacggcctg gccaaccaga tcaacaaccc cgaggtggag gtggacatca 1560
ccaagccgga catgaccatc cggcagcaga tcatgcagct gaagatcatg accaaccggc 1620
tgcgcagcgc ctacaacggc aacgacgtgg acttccagga cgccagtgac gacggcagcg 1680
gctcgggcag cggtgatggc tgtctggatg acctctgcgg ccggaaggtc agcaggaaga 1740
gctccagctc ccggacgccc ttgacccatg ccctcccagg cctgtcagag caggaaggac 1800
agaagacctc ggctgccagc tgcccccagc cccgaccttt cctcctgccc ctcctcctct 1860
tcctggccct tacagtagcc aggccccggt ggcggtaact gccccaaggc cccagggaca 1920
gaggccaagg actgactttg ccaaaaatac aacacagacg atatttaatt cacctcagcc 1980
tggagaggcc tggggtggga cagggagggc cggcggctct gagcaggggc aggcgcagag 2040
gtcccagccc caggcctggc ctcgcctgcc tttctgcctt ttaattttgt atgaggtcct 2100
caggtcagct gggagccagt gtgcccaaaa gccatgtatt tcagggacct caggggcacc 2160
tccggctgcc tagccctccc cccagctccc tgcaccgccg cagaagcagc ccctcgaggc 2220
ctacagagga ggcctcaaag caacccgctg gagcccacag cgagcctgtg ccttcctccc 2280
cgcctcctcc cactgggact cccagcagag cccaccagcc agccctggcc caccccccag 2340
cctccagaga agccccgcac gggctgtctg ggtgtccgcc atccagggtc tggcagagcc 2400
tctgagatga tgcatgatgc cctcccctca gcgcaggctg cagagcccgg ccccacctcc 2460
ctgcgccctt gaggggcccc agcgtctgca gggtgacgcc tgagacagca ccactgctga 2520
ggagtctgag gactgtcctc ccacagaccc tgcagtgagg ggccctccat gcgcagatga 2580
ggggccactg acccacctgc gcttctgctg gaggagggga agctgggccc aaaggcccag 2640
ggaggcagcg tgggctctgc caatgtgggc tgcccctcgc acacagggct cacagggcag 2700
gccttgctgg ggtccagggc tgttggagga ccccgagggc tgaggagcag ccaggacccg 2760
cctgctccca tcctcaccca gatcaggaac cagggcctcc ctgttcacgg tgacacaggt 2820
cagggctcag agtgaccctc ggctgtcacc tgctcacagg gatgctggtg gctggtgaga 2880
ccccgcactg cacacgggaa tgcctaggtc ccttcccgac ccagccagct gcactgcagg 2940
gcacggggac ctggatagtt aagggctttt ccaaacatgc atccatttac tgacacttcc 3000
tgtccttgtt catggagagc tgttcgctcc tcccagatgg cttcggaggc ccgcagggcc 3060
caccttggac cctggtgacc tcctgtcact cactgaggcc atcagggccc tgccccaggc 3120
ctggacgggc cctccttccc tcctgtgccc cagctgccag gtggccctgg ggaggggtgg 3180
tgtggtgttg ggaaggggtc ctgcaggggg aggaggactt ggagggtctg ggggcagctg 3240
tcctgaaccg actgaccctg aggaggccgc ttagtgctgc tttgcttttc atcaccgtcc 3300
cgcacagtgg acggaggtcc ccggttgctg gtcaggtccc catggcttgt tctctggaac 3360
ctgactttag atgttttggg atcaggagcc cccaacacag gcaagtccac cccataataa 3420
ccctgccagt gccagggtgg gctggggact ctggcacagt gatgccgggc gccaggacag 3480
cagcactccc gctgcacaca gacggcctag gggtggcgct cagaccccac cctacgctca 3540
tctctggaag gggcagccct gagtggtcac tggtcagggc agtggccaag cctgctgtgt 3600
ccttcctcca caaggtcccc ccaccgctca gtgtcagcgg gtgacgtgtg ttcttttgag 3660
tccttgtatg aataaaaggc tggaaaccta aa 3692

<210> 400
<211> 490
<212> PRT
<213> Homo sapiens

<400> 400
Met Glu Leu Arg Ala Arg Gly Trp Trp Leu Leu Cys Ala Ala Ala Ala
1               5                   10                  15
Leu Val Ala Cys Ala Arg Gly Asp Pro Ala Ser Lys Ser Arg Ser Cys
            20                  25                  30
Gly Glu Val Arg Gln Ile Tyr Gly Ala Lys Gly Phe Ser Leu Ser Asp
        35                  40                  45
Val Pro Gln Ala Glu Ile Ser Gly Glu His Leu Arg Ile Cys Pro Gln
    50                  55                  60

Gly Tyr Thr Cys Cys Thr Ser Glu Met Glu Glu Asn Leu Ala Asn Arg
65                  70                  75                  80
Ser His Ala Glu Leu Glu Thr Ala Leu Arg Asp Ser Ser Arg Val Leu
                85                  90                  95
Gln Ala Met Leu Ala Thr Gln Leu Arg Ser Phe Asp Asp His Phe Gln
            100                 105                 110
His Leu Leu Asn Asp Ser Glu Arg Thr Leu Gln Ala Thr Phe Pro Gly
        115                 120                 125
Ala Phe Gly Glu Leu Tyr Thr Gln Asn Ala Arg Ala Phe Arg Asp Leu
    130                 135                 140
Tyr Ser Glu Leu Arg Leu Tyr Tyr Arg Gly Ala Asn Leu His Leu Glu
145                 150                 155                 160
Glu Thr Leu Ala Glu Phe Trp Ala Arg Leu Leu Glu Arg Leu Phe Lys
                165                 170                 175
Gln Leu His Pro Gln Leu Leu Leu Pro Asp Asp Tyr Leu Asp Cys Leu
            180                 185                 190
Gly Lys Gln Ala Glu Ala Leu Arg Pro Phe Gly Glu Ala Pro Arg Glu
        195                 200                 205
Leu Arg Leu Arg Ala Thr Arg Ala Phe Val Ala Ala Arg Ser Phe Val
    210                 215                 220
Gln Gly Leu Gly Val Ala Ser Asp Val Val Arg Lys Val Ala Gln Val
225                 230                 235                 240
Pro Leu Gly Pro Glu Cys Ser Arg Ala Val Met Lys Leu Val Tyr Cys
                245                 250                 255
Ala His Cys Leu Gly Val Pro Gly Ala Arg Pro Cys Pro Asp Tyr Cys
            260                 265                 270
Arg Asn Val Leu Lys Gly Cys Leu Ala Asn Gln Ala Asp Leu Asp Ala
        275                 280                 285
Glu Trp Arg Asn Leu Leu Asp Ser Met Val Leu Ile Thr Asp Lys Phe
    290                 295                 300
Trp Gly Thr Ser Gly Val Glu Ser Val Ile Gly Ser Val His Thr Trp
305                 310                 315                 320
Leu Ala Glu Ala Ile Asn Ala Leu Gln Asp Asn Arg Asp Thr Leu Thr
                325                 330                 335
Ala Lys Val Ile Gln Gly Cys Gly Asn Pro Lys Val Asn Pro Gln Gly
            340                 345                 350
Pro Gly Pro Glu Glu Lys Arg Arg Arg Gly Lys Leu Ala Pro Arg Glu
        355                 360                 365
Arg Pro Pro Ser Gly Thr Leu Glu Lys Leu Val Ser Glu Ala Lys Ala
    370                 375                 380
Gln Leu Arg Asp Val Gln Asp Phe Trp Ile Ser Leu Pro Gly Thr Leu
385                 390                 395                 400
Cys Ser Glu Lys Met Ala Leu Ser Thr Ala Ser Asp Asp Arg Cys Trp
                405                 410                 415
Asn Gly Met Ala Arg Gly Arg Tyr Leu Pro Glu Val Met Gly Asp Gly
            420                 425                 430
Leu Ala Asn Gln Ile Asn Asn Pro Glu Val Glu Val Asp Ile Thr Lys
        435                 440                 445
Pro Asp Met Thr Ile Arg Gln Gln Ile Met Gln Leu Lys Ile Met Thr
    450                 455                 460
Asn Arg Leu Arg Ser Ala Tyr Asn Gly Asn Asp Val Asp Phe Gln Asp
465                 470                 475                 480
Ala Ser Asp Asp Gly Ser Gly Ser Gly Ser
                485                 490

<210> 401
<211> 1026
<212> DNA
<213> Homo sapiens

<400> 401
gcgagggcga gggcgaggcg gtgctcatgg aggaggacct gatccagcag agcctggacg      60
actacgacgc cggcaggtac agcccgcggc tgctcacggc gcacgagctg ccactggacg     120

cgcacgtgct ggaaccggat gaggacctgc agcgcctgca gctctcgcgc cagcagctcc 180
aggtcacggg agacgccagc gagagcgccg aggacatctt cttccggcgg gccaaggagg 240
gcatgggcca ggacgaggcg cagttcagcg tggagatgcc actcaccggc aaggcctacc 300
tgtgggccga caagtaccgg ccacgcaagc cgcgcttctt caaccgcgtg cacacgggct 360
tcgagtggaa caagtacaac cagacgcact acgactttga caacccaccg cccaagatcg 420
tgcagggata caagttcaac atcttctacc ccgacctcat cgacaagcgc tccacgcccg 480
agtacttcct ggaggcctgc gccgacaaca aggatttcgc catcctgcgc ttcacgcggg 540
gccgcctacg aggacatcgc tttcaagatc gtcaaccgcg agtgggaata ctcgcaccgc 600
cacggcttcc gctgccagtt tgccaacggc atcttccagc tgtgctttca cttcaagcgc 660
taccgctatc ggcggtgacg gccctgggga acggcaggcc aggagggccg agggccacac 720
gggtgccaca gcccaggtcg gagtggccca gccggcaggc ttgttcttca gcatccgacg 780
ggaacatctc caacagaagc aaaacggaaa gtgcctcccg gacccccaga gggccaccca 840
acctcaccag tcaccagccc cagaccaccc acagcccctc ccagacaccc cgcctcatct 900
ggaaatagtt ccgtttgttt ctctaaaaag acttgtaggt gggaaaaaaa atcttttgtt 960
ctcatggaat tggcctattg gcaagatcgc atgttttttt aataaacgtt gtattttaga 1020
ataaaa 1026

<210> 402
<211> 295
<212> PRT
<213> Homo sapiens

<400> 402

<210> 403
<211> 2161
<212> DNA
<213> Homo sapiens

<400> 403
cggcccagtg atcttgaacc ccaaaggcca gaactggagc ctcagtccag agaattctga 60
gaaaattaaa gcagagagga ggggagagat cactgggacc aggccgtgat ctctatgccc 120
gagtctcaac cctcaactgt caccccaagg cacttgggac gtcctggaca gaccgagtcc 180
cgggaagccc cagcactgcc gctgccacac tgccctgagc ccaaatgggg gagtgagagg 240
ccatagctgt ctggcatggg cctctccacc gtgcctgacc tgctgctgcc gctggtgctc 300
ctggagctgt tggtgggaat atacccctca ggggttattg gactggtccc tcacctaggg 360
gacagggaga agagagatag tgtgtgtccc caaggaaaat atatccaccc tcaaaataat 420
tcgatttgct gtaccaagtg ccacaaagga acctacttgt acaatgactg tccaggcccg 480
gggcaggata cggactgcag ggagtgtgag agcggctcct tcaccgcttc agaaaaccac 540
ctcagacact gcctcagctg ctccaaatgc cgaaaggaaa tgggtcaggt ggagatctct 600
tcttgcacag tggaccggga caccgtgtgt ggctgcagga agaaccagta ccggcattat 660
tggagtgaaa accttttcca gtgcttcaat tgcagcctct gcctcaatgg gaccgtgcac 720
ctctcctgcc aggagaaaca gaacaccgtg tgcacctgcc atgcaggttt ctttctaaga 780
gaaaacgagt gtgtctcctg tagtaactgt aagaaaagcc tggagtgcac gaagttgtgc 840
ctaccccaga ttgagaatgt taagggcact gaggactcag gcaccacagt gctgttgccc 900
ctggtcattt tctttggtct ttgcctttta tccctcctct tcattggttt aatgtatcgc 960
taccaacggt ggaagtccaa gctctactcc attgtttgtg ggaaatcgac acctgaaaaa 1020
gagggggagc ttgaaggaac tactactaag cccctggccc caaacccaag cttcagtccc 1080
actccaggct tcacccccac cctgggcttc agtcccgtgc ccagttccac cttcacctcc 1140
agctccacct ataccccggg tgactgtccc aactttgcgg ctccccgcag agaggtggca 1200
ccaccctatc agggggctga ccccatcctt gcgacagccc tcgcctccga ccccatcccc 1260
aacccccttc agaagtggga ggacagcgcc cacaagccac agagcctaga cactgatgac 1320
cccgcgacgc tgtacgccgt ggtggagaac gtgcccccgt tgcgctggaa ggaattcgtg 1380
cggcgcctag ggctgagcga ccacgagatc gatcggctgg agctgcagaa cgggcgctgc 1440
ctgcgcgagg cgcaatacag catgctggcg acctggaggc ggcgcacgcc gcggcgcgag 1500
gccacgctgg agctgctggg acgcgtgctc cgcgacatgg acctgctggg ctgcctggag 1560
gacatcgagg aggcgctttg cggccccgcc gccctcccgc ccgcgcccag tcttctcaga 1620
tgaggctgcg cccctgcggg cagctctaag gaccgtcctg cgagatcgcc ttccaacccc 1680
acttttttct ggaaaggagg ggtcctgcag gggcaagcag gagctagcag ccgcctactt 1740
ggtgctaacc cctcgatgta catagctttt ctcagctgcc tgcgcgccgc cgacagtcag 1800
cgctgtgcgc gcggagagag gtgcgccgtg ggctcaagag cctgagtggg tggtttgcga 1860
ggatgaggga cgctatgcct catgcccgtt ttgggtgtcc tcaccagcaa ggctgctcgg 1920
gggcccctgg ttcgtccctg agcctttttc acagtgcata agcagttttt tttgtttttg 1980
ttttgttttg ttttgttttt aaatcaatca tgttacacta atagaaactt ggcactcctg 2040
tgccctctgc ctggacaagc acatagcaag ctgaactgtc ctaaggcagg ggcgagcacg 2100
gaacaatggg gccttcagct ggagctgtgg acttttgtac atacactaaa attctgaagt 2160
t 2161

<210> 404
<211> 455
<212> PRT
<213> Homo sapiens

<400> 404

<210> 405
<211> 3795
<212> DNA
<213> Homo sapiens

<400> 405
ccaagttcta cctcatgttt ggaggatctt gctagctatg gccctcgtac tcggctccct      60
gttgctgctg gggctgtgcg ggaactcctt ttcaggaggg cagccttcat ccacagatgc      120
tcctaaggct tggaattatg aattgcctgc aacaaattat gagacccaag actcccataa      180
agctggaccc attggcattc tctttgaact agtgcatatc tttctctatg tggtacagcc      240
gcgtgatttc ccagaagata ctttgagaaa attcttacag aaggcatatg aatccaaaat      300
tgattatgac aagccagaaa ctgtaatctt aggtctaaag attgtctact atgaagcagg      360
gattattcta tgctgtgtcc tggggctgct gtttattatt ctgatgcctc tggtggggta      420
tttcttttgt atgtgtcgtt gctgtaacaa atgtggtgga gaaatgcacc agcgacagaa      480
ggaaaatggg cccttcctga ggaaatgctt tgcaatctcc ctgttggtga tttgtataat      540
aataagcatt ggcatcttct atggttttgt ggcaaatcac caggtaagaa cccggatcaa      600

aaggagtcgg aaactggcag atagcaattt caaggacttg cgaactctct tgaatgaaac 660
tccagagcaa atcaaatata tattggccca gtacaacact accaaggaca aggcgttcac 720
agatctgaac agtatcaatt cagtgctagg aggcggaatt cttgaccgac tgagacccaa 780
catcatccct gttcttgatg agattaagtc catggcaaca gcgatcaagg agaccaaaga 840
ggcgttggag aacatgaaca gcaccttgaa gagcttgcac caacaaagta cacagcttag 900
cagcagtctg accagcgtga aaactagcct gcggtcatct ctcaatgacc ctctgtgctt 960
ggtgcatcca tcaagtgaaa cctgcaacag catcagattg tctctaagcc agctgaatag 1020
caaccctgaa ctgaggcagc ttccacccgt ggatgcagaa cttgacaacg ttaataacgt 1080
tcttaggaca gatttggatg gcctggtcca acagggctat caatccctta atgatatacc 1140
tgacagagta caacgccaaa ccacgactgt cgtagcaggt atcaaaaggg tcttgaattc 1200
cattggttca gatatcgaca atgtaactca gcgtcttcct attcaggata tactctcagc 1260
attctctgtt tatgttaata acactgaaag ttacatccac agaaatttac ctacattgga 1320
agagtatgat tcatactggt ggctgggtgg cctggtcatc tgctctctgc tgaccctcat 1380
cgtgattttt tactacctgg gcttactgtg tggcgtgtgc ggctatgaca ggcatgccac 1440
cccgaccacc cgaggctgtg tctccaacac cggaggcgtc ttcctcatgg ttggagttgg 1500
attaagtttc ctcttttgct ggatattgat gatcattgtg gttcttacct ttgtctttgg 1560
tgcaaatgtg gaaaaactga tctgtgaacc ttacacgagc aaggaattat tccgggtttt 1620
ggatacaccc tacttactaa atgaagactg ggaatactat ctctctggga agctatttaa 1680
taaatcaaaa atgaagctca cttttgaaca agtttacagt gactgcaaaa aaaatagagg 1740
cacttacggc actcttcacc tgcagaacag cttcaatatc agtgaacatc tcaacattaa 1800
tgagcatact ggaagcataa gcagtgaatt ggaaagtctg aaggtaaatc ttaatatctt 1860
tctgttgggt gcagcaggaa gaaaaaacct tcaggatttt gctgcttgtg gaatagacag 1920
aatgaattat gacagctact tggctcagac tggtaaatcc cccgcaggag tgaatctttt 1980
atcatttgca tatgatctag aagcaaaagc aaacagtttg cccccaggaa atttgaggaa 2040
ctccctgaaa agagatgcac aaactattaa aacaattcac cagcaacgag tccttcctat 2100
agaacaatca ctgagcactc tataccaaag cgtcaagata cttcaacgca cagggaatgg 2160
attgttggag agagtaacta ggattctagc ttctctggat tttgctcaga acttcatcac 2220
aaacaatact tcctctgtta ttattgagga aactaagaag tatgggagaa caataatagg 2280
atattttgaa cattatctgc agtggatcga gttctctatc agtgagaaag tggcatcgtg 2340
caaacctgtg gccaccgctc tagatactgc tgttgatgtc tttctgtgta gctacattat 2400
cgaccccttg aatttgtttt ggtttggcat aggaaaagct actgtatttt tacttccggc 2460
tctaattttt gcggtaaaac tggctaagta ctatcgtcga atggattcgg aggacgtgta 2520
cgatgatgtt gaaactatac ccatgaaaaa tatggaaaat ggtaataatg gttatcataa 2580
agatcatgta tatggtattc acaatcctgt tatgacaagc ccatcacaac attgatagct 2640
gatgttgaaa ctgcttgagc atcaggatac tcaaagtgga aaggatcaca gatttttggt 2700
agtttctggg tctacaagga ctttccaaat ccaggagcaa cgccagtggc aacgtagtga 2760
ctcaggcggg caccaaggca acggcaccat tggtctctgg gtagtgcttt aagaatgaac 2820
acaatcacgt tatagtccat ggtccatcac tattcaagga tgactccctc ccttcctgtc 2880
tatttttgtt ttttactttt ttacactgag tttctattta gacactacaa catatggggt 2940
gtttgttccc attggatgca tttctatcaa aactctatca aatgtgatgg ctagattcta 3000
acatattgcc atgtgtggag tgtgctgaac acacaccagt ttacaggaaa gatgcatttt 3060
gtgtacagta aacggtgtat ataccttttg ttaccacaga gttttttaaa caaatgagta 3120
ttataggact ttcttctaaa tgagctaaat aagtcaccat tgacttcttg gtgctgttga 3180
aaataatcca ttttcactaa aagtgtgtga aacctacagc atattcttca cgcagagatt 3240
ttcatctatt atactttatc aaagattggc catgttccac ttggaaatgg catgcaaaag 3300
ccatcataga gaaacctgcg taactccatc tgacaaattc aaaagagaga gagagatctt 3360
gagagagaaa tgctgttcgt tcaaaagtgg agttgtttta acagatgcca attacggtgt 3420
acagtttaac agagttttct gttgcattag gataaacatt aattggagtg cagctaacat 3480
gagtatcatc agactagtat caagtgttct aaaatgaaat atgagaagat cctgtcacaa 3540
ttcttagatc tggtgtccag catggatgaa acctttgagt ttggtcccta aatttgcatg 3600
aaagcacaag gtaaatattc atttgcttca ggagtttcat gttggatctg tcattatcaa 3660
aagtgatcag caatgaagaa ctggtcggac aaaatttaac gttgatgtaa tggaattcca 3720
gatgtaggca ttccccccag gtcttttcat gtgcagattg cagttctgat tcatttgaat 3780
aaaaaggaac ttggc 3795

<210> 406
<211> 865
<212> PRT
<213> Homo sapiens

<400> 406

<210> 407
<211> 1362
<212> DNA
<213> Homo sapiens
<400> 407
atggcttcac ccagcctccc gggcagtgac tgctcccaaa tcattgatca cagtcatgtc 60
cccgagtttg aggtggccac ctggatcaaa atcaccctta ttctggtgta cctgatcatc 120
ttcgtgatgg gccttctggg gaacagcgcc accattcggg tcacccaggt gctgcagaag 180
aaaggatact tgcagaagga ggtgacagac cacatggtga gtttggcttg ctcggacatc 240
ttggtgttcc tcatcggcat gcccatggag ttctacagca tcatctggaa tcccctgacc 300
acgtccagct acaccctgtc ctgcaagctg cacactttcc tcttcgaggc ctgcagctac 360
gctacgctgc tgcacgtgct gacactcagc tttgagcgct acatcgccat ctgtcacccc 420
ttcaggtaca aggctgtgtc gggaccttgc caggtgaagc tgctgattgg cttcgtctgg 480
gtcacctccg ccctggtggc actgcccttg ctgtttgcca tgggtactga gtaccccctg 540
gtgaacgtgc ccagccaccg gggtctcact tgcaaccgct ccagcacccg ccaccacgag 600

cagcccgaga cctccaatat gtccatctgt accaacctct ccagccgctg gaccgtgttc      660
cagtccagca tcttcggcgc cttcgtggtc tacctcgtgg tcctgctctc cgtagccttc      720
atgtgctgga acatgatgca ggtgctcatg aaaagccaga agggctcgct ggccgggggc      780
acgcggcctc cgcagctgag gaagtccgag agcgaagaga gcaggaccgc caggaggcag      840
accatcatct tcctgaggct gattgttgtg acattggccg tatgctggat gcccaaccag      900
attcggagga tcatggctgc ggccaaaccc aagcacgact ggacgaggtc ctacttccgg      960
gcgtacatga tcctcctccc cttctcggag acgtttttct acctcagctc ggtcatcaac     1020
ccgctcctgt acacggtgtc ctcgcagcag tttcggcggg tgttcgtgca ggtgctgtgc     1080
tgccgcctgt cgctgcagca cgccaaccac gagaagcgcc tgcgcgtaca tgcgcactcc     1140
accaccgaca gcgcccgctt tgtgcagcgc ccgttgctct tcgcgtcccg gcgccagtcc     1200
tctgcaagga gaactgagaa gatttcttta agcacttttc agagcgaggc cgagccccag     1260
tctaagtccc agtcattgag tctcgagtca ctagagccca actcaggcgc gaaaccagcc     1320
aattctgctg cagagaatgg ttttcaggag catgaagttt ga                        1362

<210> 408
<211> 453
<212> PRT
<213> Homo sapiens

<400> 408
Met Ala Ser Pro Ser Leu Pro Gly Ser Asp Cys Ser Gln Ile Ile Asp
1               5                   10                  15
His Ser His Val Pro Glu Phe Glu Val Ala Thr Trp Ile Lys Ile Thr
            20                  25                  30
Leu Ile Leu Val Tyr Leu Ile Ile Phe Val Met Gly Leu Leu Gly Asn
        35                  40                  45
Ser Ala Thr Ile Arg Val Thr Gln Val Leu Gln Lys Lys Gly Tyr Leu
    50                  55                  60
Gln Lys Glu Val Thr Asp His Met Val Ser Leu Ala Cys Ser Asp Ile
65                  70                  75                  80
Leu Val Phe Leu Ile Gly Met Pro Met Glu Phe Tyr Ser Ile Ile Trp
                85                  90                  95
Asn Pro Leu Thr Thr Ser Ser Tyr Thr Leu Ser Cys Lys Leu His Thr
            100                 105                 110
Phe Leu Phe Glu Ala Cys Ser Tyr Ala Thr Leu Leu His Val Leu Thr
        115                 120                 125
Leu Ser Phe Glu Arg Tyr Ile Ala Ile Cys His Pro Phe Arg Tyr Lys
    130                 135                 140
Ala Val Ser Gly Pro Cys Gln Val Lys Leu Leu Ile Gly Phe Val Trp
145                 150                 155                 160
Val Thr Ser Ala Leu Val Ala Leu Pro Leu Leu Phe Ala Met Gly Thr
                165                 170                 175
Glu Tyr Pro Leu Val Asn Val Pro Ser His Arg Gly Leu Thr Cys Asn
            180                 185                 190
Arg Ser Ser Thr Arg His His Glu Gln Pro Glu Thr Ser Asn Met Ser
        195                 200                 205
Ile Cys Thr Asn Leu Ser Ser Arg Trp Thr Val Phe Gln Ser Ser Ile
    210                 215                 220
Phe Gly Ala Phe Val Val Tyr Leu Val Val Leu Leu Ser Val Ala Phe
225                 230                 235                 240
Met Cys Trp Asn Met Met Gln Val Leu Met Lys Ser Gln Lys Gly Ser
                245                 250                 255
Leu Ala Gly Gly Thr Arg Pro Pro Gln Leu Arg Lys Ser Glu Ser Glu
            260                 265                 270
Glu Ser Arg Thr Ala Arg Arg Gln Thr Ile Ile Phe Leu Arg Leu Ile
        275                 280                 285
Val Val Thr Leu Ala Val Cys Trp Met Pro Asn Gln Ile Arg Arg Ile
    290                 295                 300
Met Ala Ala Ala Lys Pro Lys His Asp Trp Thr Arg Ser Tyr Phe Arg
305                 310                 315                 320
Ala Tyr Met Ile Leu Leu Pro Phe Ser Glu Thr Phe Phe Tyr Leu Ser
                325                 330                 335
Ser Val Ile Asn Pro Leu Leu Tyr Thr Val Ser Ser Gln Gln Phe Arg

                340                 345                 350
Arg Val Phe Val Gln Val Leu Cys Cys Arg Leu Ser Leu Gln His Ala
        355                 360                 365
Asn His Glu Lys Arg Leu Arg Val His Ala His Ser Thr Thr Asp Ser
    370                 375                 380
Ala Arg Phe Val Gln Arg Pro Leu Leu Phe Ala Ser Arg Arg Gln Ser
385                 390                 395                 400
Ser Ala Arg Arg Thr Glu Lys Ile Phe Leu Ser Thr Phe Gln Ser Glu
                405                 410                 415
Ala Glu Pro Gln Ser Lys Ser Gln Ser Leu Ser Leu Glu Ser Leu Glu
            420                 425                 430
Pro Asn Ser Gly Ala Lys Pro Ala Asn Ser Ala Ala Glu Asn Gly Phe
        435                 440                 445
Gln Glu His Glu Val
    450

<210> 409
<211> 6
<212> PRT
<213> Artificial Sequence

<220>
<223> HIS6 epitope tag

<400> 409
His His His His His His
1               5

<210> 410
<211> 4
<212> PRT
<213> Artificial Sequence

<220>
<223> DEAD box domain

<400> 410
Asp Glu Ala Asp
1

<210> 411
<211> 4
<212> PRT
<213> Artificial Sequence

<220>
<223> KDEL endoplasmic reticulum retension sequence

<400> 411
Lys Asp Glu Leu
1

<210> 412
<211> 3428
<212> DNA
<213> Homo sapiens

<400> 412
ccacgcgtcc gacgcccccc acccgggagg ggggagagag gcaaaaagta agagaggaaa      60
aaaaatagca ggaagatggc gcccaccaag cccagctttc agcaggatcc ttccaggcga     120
gaacgtttac aagcattgag aaaggagaaa tcccgagatg ctgctcgctc ccgccgggga     180
aaagaaaact ttgagttcta tgaattggcc aagttgttgc ctcttcctgc agccattacc     240
agccagctcg acaaggcatc catcattcga cttacaatta gctatctgaa aatgagggac     300

tttgctaacc agggggaccc tccgtggaac ttgcgaatgg aaggccctcc acctaacaca 360
tcagtaaaag gtgcacagcg aaggagaagc cccagtgcac tagccattga agtatttgaa 420
gcacatttgg gaagccacat tttgcagtcc ctggatggct ttgtatttgc actaaatcag 480
gaaggaaaat ttttgtacat ttccgaaaca gtctccatct acctaggcct ctcacaagtg 540
gagctgacag gcagcagtgt ctttgactat gtccaccccg gagatcacgt ggagatggct 600
gagcagctgg gcatgaagct cccccctggg cggggtctcc tgtcacaggg cactgctgag 660
gacggagcca gctcagcatc ttcctcctct cagtcggaga cccccgagcc agtggagtca 720
accagcccca gtctgctaac cactgacaac actcttgagc gttccttttt catccgaatg 780
aaatctactc tgaccaaacg cggtgtgcac atcaaatcat caggatataa ggtgattcac 840
ataacaggcc ggctacgcct gagagtgtcg ctgtcccacg ggaggaccgt ccccagccaa 900
atcatgggtc tcgtggttgt tgcgcatgcc ttgcctcccc ctacgatcaa tgaagtcaga 960
attgactgcc atatgttcgt cactcgagta aatatggacc tcaatatcat ttactgtgaa 1020
aataggatta gtgattatat ggatctgacc cctgtagata tcgtagggaa gagatgctac 1080
cacttcatcc atgctgaaga cgtggagggc atcaggcaca gtcacttgga cttgctgaat 1140
aagggtcagt gtgtgacaaa gtactatcgc tggatgcaga agaacggagg atatatttgg 1200
atacagtcca gtgccaccat agctattaat gccaagaatg caaatgaaaa gaatatcatc 1260
tgggtgaatt accttcttag caatcctgag tacaaggaca cacccatgga catcgcacag 1320
ctcccccatc tgccggagaa aacttccgaa tcctcggaga catccgactc tgagtcagac 1380
tctaaagaca cctcaggtat tacagaggac aacgagaact ccaagtccga cgagaagggg 1440
aaccagtccg agaacagcga agacccggag cccgaccgga agaagtcggg caacgcgtgt 1500
gacaacgaca tgaactgcaa cgacgacggc cacagctcca gtaacccgga cagccgcgac 1560
agcgacgaca gcttcgagca ctcggacttt gagaacccca aggcgggcga ggacggcttc 1620
ggtgctctgg gcgcgatgca gatcaaggtg gagcgctacg tggagagcga gtcggacctg 1680
cggctgcaga actgcgagtc actcacgtcc gacagcgcca aggactcgga cagcgcaggc 1740
gaggcgggcg cgcaggcctc cagcaagcac cagaagcgca agaaaaggcg gaaacggcaa 1800
aagggcggca gcgccagccg ccggcgcctg tccagcgcgt cgagcccagg cggcctggac 1860
gcgggcctgg tggagccccc gcggctgctg tcctccccca acagtgcctc ggtgctcaag 1920
atcaagacgg agatctcaga acccatcaat ttcgacaatg acagcagcat ctggaactac 1980
ccgcccaacc gggagatctc caggaacgag tccccctaca gcatgaccaa gccccccagc 2040
tctgagcact tcccgtcccc gcagggcggc ggcggtgggg gtggcggtgg cggggggctg 2100
cacgtggcca ttcccgactc ggtcctcacc ccgcccggcg ccgacggcgc ggccgcccgc 2160
aagactcagt tcggcgcctc ggccaccgcg gccctggccc ccgtcgcctc cgacccgctg 2220
tcacccccgc tctcggcgtc cccgcgggac aagcaccccg ggaacggcgg cgggggcggg 2280
ggcgggggcg gcggcgcggg gggcggcggc cccagcgcgt ccaactcctt gctgtacact 2340
ggggacctgg aggcgctgca gaggttgcag gcgggcaacg tcgtgctccc gctggtgcac 2400
agggtgaccg ggaccctggc cgccaccagc acggccgcgc agagggtcta caccacgggc 2460
accatccgct acgcgcccgc cgaggtgacc ctggccatgc agagcaacct gctgcccaac 2520
gcgcacgctg ttaacttcgt ggacgttaac agccccggct ttggcctcga ccccaagacg 2580
cccatggaga tgctctacca ccacgtgcac cggctcaaca tgtcaggacc gttcggcggc 2640
gcagtgagcg cagctagcct gacgcagatg cccgccggca acgtgttcac cacggccgag 2700
ggactcttct ccacgctgcc cttccccgtc tacagcaacg gcatccacgc ggcacagact 2760
ctggagcgca aggaggactg aggcgccgcc cgtcctgggc ccggccaggc cccgcttgga 2820
ggaggcatcg tcggcatttt cgtttagacc tttaattcta gcactttgaa ttcgagcagg 2880
tcagcgtctt ctctcgccac gacggtcccc attccacccc ctctttcttt cacctgactt 2940
attctttcgt gtaaagatat gtttattttt tgccttcaga gggtcagacg accagttgcc 3000
tgccgttttg tcttcttcta aggtgtgtgt tgggttgttt tgctttcctt tgcatcttta 3060
ttaagatgtc tttcatgtgt atatgcctct gccatagaat actcagtctt gtggtcaaga 3120
gagttctcaa gtgacaacca ttggggtttc ttcataaaga tcttgatatg atcaagatgg 3180
aaagagacaa gcataaacaa tgtgccctgt ttgactaagt caaatgaaat agggtggttt 3240
ttgtttctgt tcctaattcc tttaaaaaat agggggaata gtattttaga attttatgca 3300
gaatttaatt ctctttttac ggttaagatt ttaagatttt cttacttgca cataaaaata 3360
atttgggttc ttaaacttaa tttctggcct gtgactagaa tgtttaaaaa aaaaaaaaac 3420
cctcgtgc 3428

## Description

### CROSS-REFERENCES TO RELATED APPLICATIONS

This application claims priority to USSN 60/323,469, filed Setember 17,2001; USSN 60/355,145, filed February 8, 2002; USSN 60/369,899, filed April 4, 2002; USSN 60/323,887, filed September 20, 2001; USSN 60/355,257, filed February 8, 2002; USSN 60/325,114, filed September 25, 2001; USSN 60/340,944, filed October 29, 2001; USSN 60/350,666, filed November 13, 2001; and USSN 60/372,246, filed April 12, 2002; each of which is incorporated herein by reference for all purposes.

### FIELD OF THE INVENTION

The invention relates to the identification of nucleic acid and protein expression profiles and nucleic acids, products, and antibodies thereto that are involved in cancer; and to the use of such expression profiles and compositions in the diagnosis, prognosis, and therapy of cancer. The invention further relates to methods for identifying and using agents and/or targets that modulate cancer.

### BACKGROUND OF THE INVENTION

Cancer is a major cause of morbidity in the United States. For example, in 1996, the American Cancer Society estimated that 1,359,150 people were diagnosed with a malignant neoplasm and 554,740 died from one of these diseases. Cancer is responsible for 23.9 percent of all American deaths and is exceeded only by heart disease as a cause of mortality (33 percent). Unfortunately, cancer mortality is increasing and sometime early in this century, cancer is expected to become the leading cause of mortality in the United States as it already is in Japan.

Cancers share the charactaristic of disordered control over normal cell division, growth, and differentiation. Their initial clinical manifestations are extremely heterogeneous, with over 70 types of cancer arising in virtually every organ and tissue of the body. Moreover, some of those similarly classified cancer types may represent multiple different molecular diseases. Unfortunately, some cancers may be virtually asymptomatic until late in the disease course, when treatment is more difficult, and prognosis grim.

Treatment for cancer typically includes surgery, chemotherapy, and/or radiation therapy. Although nearly 50 percent of cancer patients can be effectively treated using these methods, the current therapies all induce serious side effects which diminish quality of life. The identification of novel therapeutic targets and diagnostic markers will be important for improving the diagnosis and treatment of cancer patients.

Recent advances in molecular medicine have increased the interest in tumor-specific antigens that could serve as targets for various immunotherapeutic or small molecule strategies. Antigens suitable for immunotherapeutic strategies should be highly expressed in cancer tissues, preferably accessible from the vasculature and at the cell surface, and ideally not expressed in normal adult tissues. Expression in tissues that are dispensable for life, however, may be tolerated, e.g., reproductive organs. Examples of antigens that are currently available for the detection and treatment of certain cancers include Her2/neu and the B-cell antigen CD20. Humanized monclonal antibodies directed to Her2/neu (Herceptin®/trastuzumab) are currently in use for the treatment of metastatic breast cancer. See Ross and Fletcher (1998) Stem Cells 16:413-428. Similarly, anti-CD20 monoclonal antibodies (Rituxin®/rituximab) are used to effectively treat non-Hodgkin's lymphoma. See Maloney, et al. (1997) Blood 90:2188-2195; Leget and Czuczman (1998) Curr. Opin. Oncol. 10:548-551.

The elucidation of a role for novel proteins and compounds in disease states for identification of therapeutic targets and diagnostic markers is valuable for improving the current treatment of cancer patients. Accordingly, provided herein are molecular targets for therapeutic intervention in various defined cancers. Additionally, provided herein are methods that can be used in diagnosis and prognosis of cancer. Further provided are methods that can be used to screen candidate bioactive agents for the ability to modulate cancer.

### SUMMARY OF THE INVENTION

The present invention provides methods for determining the presence or absence of a pathological cell in a patient, the method comprising detecting a nucleic acid comprising a sequence at least 80% identical to a sequence as described in Tables 2A-68 in a biological sample from the patient, thereby determining the presence or absence of the pathological cell. In certain embodiments of the method, the pathology is described in Table 1, including a cancer; the biological sample comprises isolated nucleic acids; the nucleic acids are mRNA; the biological sample is tissue from an organ which is affected by the pathology of Table 1, including a cancer; a further step is used of amplifying nucleic acids before the step of detecting the nucleic acid; the detecting is of a protein encoded by the nucleic acid; the nucleic acid comprises a sequence as described in Tables 2A-68; the detecting step is carried out by using a labeled nucleic acid probe, utilizing a biochip comprising at sequence at least 80% identical to a sequence as described in Tables 2A-68, or detecting a polypeptide encoded by the nucleic acid; or the patient is undergoing a therapeutic regimen to treat the pathology of Table 1, or is suspected of having the pathology or cancer.

Compostions are also provided, e.g., an isolated nucleic acid molecule comprising a sequence as described in Tables 2A-68, including, e.g., those which are labeled; an expression vector comprising such nucleic acid; a host cell comprising such expression vector; an isolated polypeptide which is encoded by such a nucleic acid molecule comprising a sequence as described in Tables 2A-68; or an antibody that specifically binds the polypeptide. In particular embodiments, the antibody is: conjugated to an effector component, is conjugated to a detectable label (including, e.g., a fluorescent label, a radioisotope, or a cytotoxic chemical), an antibody fragment, or is a humanized antibody.

Additional methods are provided, including methods for specifically targeting a compound to a pathological cell in a patient, the method comprising administering to the patient an antibody, as described, thereby providing the targetting. Others include, e.g., methods for determining the presence or absence of a pathological cell in a patient, the methods comprising contacting a biological sample with an antibody, as described. In more particular methods, the antibody is: conjugated to an effector component, or to a fluorescent label; or the biological sample is a blood, serum, urine, or stool sample.

Further methods include those for identifying a compound that modulates a pathology-associated polypeptide, the method comprising steps of: contacting the compound with a pathology-associated polypeptide, the polypeptide encoded by a polynucleotide that selectively hybridizes to a sequence at least 80% identical to a sequence as described in Tables 2A-68; and determining the functional effect of the compound upon the polypeptide. Another drug screening assay method comprises steps of: administering a test compound to a mammal having a pathology of Table 1 or a cell isolated therefrom; and comparing the level of gene expression of a polynucleotide that selectively hybridizes to a sequence at least 80% identical to a sequence as described in Tables 2A-68 in a treated cell or mammal with the level of gene expression of the polynucleotide in a control cell or mammal, wherein a test compound that modulates the level of expression of the polynucleotide is a candidate for the treatment of the pathology.

### DETAILED DESCRIPTION OF THE INVENTION

In accordance with the objects outlined above, the present invention provides novel methods for diagnosis and prognosis evaluation for various disorders, e.g., angiogenesis, fibrosis, and various defined forms of cancer, including metastatic cancer, as well as methods for screening for compositions which modulate such conditions. Also provided are methods for treating such disorders or cancers. See, e.g., American Society of Clinical Oncology (ed. 2001) ASCO Curriculum: Symptom Management Kendall/Hunt, ISBN: 0787277851; Bonadonna, et al. (2001) Textbook of Breast Cancer (2d ed.) Dunitz Martin, ISBN: 1853178241; Devita and Hellman (eds. 2001) Cancer Principles and Practice of Oncology (2 vols.), Lippincott Williams, ISBN: 0781723876; Howell, et al. (2001) Breast Cancer Isis Medical Media, ISBN: 1901865584; Kaye and Laws (2001) Brain Tumours: An Encyclopedic Approach (2d ed.) Churchill Livingstone, ISBN: 0443064261; Mihm, et al. (2001) The Melanocytic Proliferation: A Comprehensive Textbook of Pigmented Lesions Wiley-Liss, ISBN: 0471252719; Montgomery and Aaron (2001) Clinical Pathology of Soft-Tissue Tumors Marcel Dekker, ISBN: 0824702905; Petrovich, et al. (eds. 2001) Combined Modality of Central Nervous System Tumors (Medical Radiology) Springer Verlag, ISBN: 3540660534; Rosen (2001) Rosen's Breast Pathology Lippincott Williams and Wilkins, ISBN: 0781723795; Shah, et al. (2001) Oral Cancer Isis Medical Media, ISBN: 189906687X; Weiss and Goldblum (2001) Enzinger and Weiss's Soft Tissue Tumors (4th ed.) Mosby, ISBN: 0323012000; Abeloff, et al. (eds. 2000) Clinical Oncology (2d ed.) Churchill Livingstone, ISBN: 044307545X; American Society of Clinical Oncology (ed. 2000) Cancer Genetics and Cancer Predisposition Testing Kendall/Hunt, ISBN: 0787276154; Fletcher (2000) Diagnostic Histopathology of Tumors (2 vols. 2d ed.) Churchill Livingstone, ISBN: 0443079927; Vogelzang (ed. 2000) Comprehensive Textbook of Genitourinary Oncology (2d ed.) Lippincott Williams and Wilkins, ISBN: 0683306456; Holland, et al. (eds. 2000) Holland-Frei Cancer Medicine (Book with CD-ROM 5th ed.) Decker, ISBN: 1550091131; Turrisi, et al. (2000) Lung Cancer Isis Medical Media, ISBN: 1901865428; Bartolozzi and Lencioni (eds. 1999) Liver Malignancies: Diagnostic and Interventional Radiology (Medical Radiology) Springer Verlag, ISBN: 3540647562; Gasparini (ed. 1999) Prognostic Variables in Node-Negative and Node-Positive Breast Cancer Kluwer, ISBN: 0792384474; Hansen (ed. 1999) The LASLC Textbook of Lung Cancer: International Association for the Study of Lung Cancer Dunitz Martin, ISBN: 1853177083; Raghavan, et al. (eds. 1999) Textbook of Uncommon Cancer (2nd ed.) Wiley, ISBN: 0471929212; Thawley, et al. (eds. 1999) Comprehensive Management of Head and Neck Tumors (2 vols.) Saunders, ISBN: 0721655823; Whittaker and Holmes (eds. 1999) Leukemia and Related Disorders (3d ed.) Blackwell Science, ISBN: 0865426074; Aapro (ed. 1998) OncoMedia: Medical Oncology (CD-ROM) Elsevier Science, ISBN: 0080427480; Abeloff (1998) Clinical Oncology (Library Version 2 CD-ROM Individual Version 2.0 Windows and Macintosh) Harcourt Brace, ISBN: 0443075557; Benson (ed. 1998) Gastrointestinal Oncology (Cancer Treatment and Research, CTAR 98) Kluwer, ISBN: 0792382056; Brambilla and Brambilla (eds. 1998) Lung Tumors: Fundamental Biology and Clinical Management (Vol 124) Marcel Dekker, ISBN: 0824701607; Canellos, et al. (eds. 1998) The Lymphomas Saunders, ISBN: 0721650309; Greenspan and Remagen (1998) Differential Diagnosis of Tumors and Tumor-Like Lesions of Bones and Joints Lippincott Williams and Wilkins Publishers, ISBN: 0397517106; Hiddemann (ed. 1998) Acute Leukemias VII: Experimental Approaches and Novel Therapies (Haematologie Und Bluttransfusion, Vol 39), Springer Verlag, ISBN: 3540635041; Husband and Reznek (1998) Imaging in Oncology (2 vols.) Mosby, ISBN: 1899066489; Leibel and Phillips (eds. 1998) Textbook of Radiation Oncology Saunders, ISBN: 0721653367; Maloney and Miller (eds. 1998) Cutaneous Oncology: Pathophysiology, Diagnosis, and Management Blackwell Science, ISBN: 0865425175; Mittal, et al. (eds. 1998) Advances in Radiation Therapy Kluwe, ISBN: 0792399811; Oldham (ed. 1998) Principles of Cancer Biotherapy (3d ed.) Kluwer, ISBN: 0792335074; Ozols (ed. 1998) Gynecologic Oncology Kluwer, ISBN: 0792380703; Parkin, et al. (eds. 1998) Cancer Incidence in Five Continents (Iarc Scientific Publications, No 143) Oxford University Press, ISBN: 9283221435; Perez and Brady (eds. 1998) Principles and Practice of Radiation Oncology Lippincott Williams and Wilkins, ISBN: 0397584164; Black, et al. (eds. 1997) Cancer of the Nervous System Blackwell Science, ISBN: 0865423849; Bonadonna, et al. (1997) Textbook of Breast Cancer: A Clinical Guide to Therapy Blackwell Science, ISBN: 1853173487; Pollock (ed. 1997) Surgical Oncology Kluwer, ISBN: 0792399005; Sheaves, et al. (eds. 1997) Clinical Endocrine Oncology Blackwell Science, ISBN: 086542862X; Vahrson (1997) Radiation Oncology of Gynecological Cancers Springer Verlag, ISBN: 0387567682; Walterhouse and Cohn (eds. 1997) Diagnostic and Therapeutic Advances in Pediatric Oncology Kluwer, ISBN: 0792399781; Aisner (ed. 1996) Comprehensive Textbook of Thoracic Oncology Lippincott, Williams and Wilkins, ISBN: 0683000624; Bertino, et al. (eds. 1996) Encyclopedia of Cancer (3 vols.) Academic, ISBN: 012093230X; Cavalli, et al. (1996) Textbook of Medical Oncology Dunitz Martin, ISBN: 1853172901; Peckham, et al. (eds. 1995) Oxford Textbook of Oncology (2-Vols.) Oxford University Press, ISBN: 0192616854; and Freireich and Kantarjian (eds. 1996) Molecular Genetics and Therapy of Leukemia (Cancer Treatment and Research, V. 84) Kluwer, ISBN: 0792339126.

In particular, identification of markers selectively expressed on defined cancers allows for use of that expression in diagnostic, prognostic, or therapeutic methods. As such, the invention defines various .compositions, e.g., nucleic acids, polypeptides, antibodies, and small molecule agonists/antagonists, which will be useful to selectively identify those markers. For example, therapeutic methods may take the form of protein therapeutics which use the marker expression for selective localization or modulation of function (for those markers which have a causative disease effect), for vaccines, identification of binding partners, or antagonism, e.g., using antisense or RNAi. The markers may be useful for molecular characterization of subsets of the diseases, e.g., as provided in Table 1, which subsets may actually require very different treatments. Moreover, the markers may also be important in related diseases to the specific disorders and cancers, e.g., which affect similar tissues in non-malignant diseases, or have similar mechanisms of induction/maintenance. Metastatic processes or characteristics may also be targeted. Diagnostic and prognostic uses are made available, e.g., to subset related but distinct diseases, or to determine treatment strategy. The detection methods may be based upon nucleic acid, e.g., PCR or hybridization techniques, or protein, e.g., ELISA, imaging, IHC, etc. The diagnosis may be qualitative or quantitative, and may detect increases or decreases in expression levels.

Tables 2B-66C provide unigene cluster identification numbers for the nucleotide sequence of genes that exhibit increased or decreased expression in cancer samples, particularly sequences involved in angiogenisis, prostate cancer (including androgen independent and taxol resistant prostate cancer), breast cancer, colorectal cancer, cervical cancer, bladder cancer, lung cancer, ovarian cancer, uterine cancer, glioblastoma, Ewing sarcoma, and lung fibrosis. Tables 2A-67 also provide an exemplar accession number that provides a nucleotide sequence that is part of the unigene cluster. Definitions

The term "cancer protein" or "cancer polynucleotide" or "cancer-associated transcript" refers to nucleic acid and polypeptide polymorphic variants, alleles, mutants, and interspecies homologues that: (1) have a nucleotide sequence that has greater than about 60% nucleotide sequence identity, 65%, 70%, 75%, 80%, 85%, 90%, preferably about 92%, 94%, 96%, 97%, 98%, or 99% or greater nucleotide sequence identity, preferably over a region of over a region of at least about 25, 50, 100, 200, 500, 1000, or more nucleotides, to a nucleotide sequence of or associated with a gene of Tables 1-68; (2) bind to antibodies, e.g., polyclonal antibodies, raised against an immunogen comprising an amino acid sequence encoded by a nucleotide sequence of or associated with a gene of Tables 1-68, and conservatively modified variants thereof; (3) specifically hybridize under stringent hybridization conditions to a nucleic acid sequence, or the complement thereof of Tables 1-68 and conservatively modified variants thereof; or (4) have an amino acid sequence that has greater than about 60% amino acid sequence identity, 65%, 70%, 75%, 80%, 85%, preferably 90%, 91%, 93%, 95%, 97%, 98%, or 99% or greater amino sequence identity, preferably over a region of over a region of at least about 25, 50, 100, 200, 500, 1000, or more amino acids, to an amino acid sequence encoded by a nucleotide sequence of or associated with a gene of Tables 1-68. A polynucleotide or polypeptide sequence is typically from a mammal including, but not limited to, primate, e.g., human; rodent, e.g., rat, mouse, hamster; cow, pig, horse, sheep, or other mammal. A "cancer polypeptide" and a "cancer polynucleotide," include both naturally occurring or recombinant forms.

A "full length" cancer protein or nucleic acid refers to a cancer polypeptide or polynucleotide sequence, or a variant thereof, that contains elements normally contained in one or more naturally occurring, wild type cancer polynucleotide or polypeptide sequences. The "full length" may be prior to, or after, various stages of post-translational processing or splicing, including alternative splicing.

"Biological sample" as used herein is a sample of biological tissue or fluid that contains nucleic acids or polypeptides, e.g., of a cancer protein, polynucleotide, or transcript. Such samples include, but are not limited to, tissue isolated from primates, e.g., humans, or rodents, e.g., mice, and rats. Biological samples may also include sections of tissues such as biopsy and autopsy samples, frozen sections taken for histologic purposes, archival samples, blood, plasma, serum, sputum, stool, tears, mucus, hair, skin, etc. Biological samples also include explants and primary and/or transformed cell cultures derived from patient tissues. A biological sample is typically obtained from a eukaryotic organism, most preferably a mammal such as a primate e.g., chimpanzee or human; cow; dog; cat; a rodent, e.g., guinea pig, rat, mouse; rabbit; or a bird; reptile; or fish. Livestock and domestic animals are of interest.

"Providing a biological sample" means to obtain a biological sample for use in methods described in this invention. Most often, this will be done by removing a sample of cells from an animal, but can also be accomplished by using previously isolated cells (e.g., isolated by another person, at another time, and/or for another purpose), or by performing the methods of the invention in vivo. Archival tissues or materials, having treatment or outcome history, will be particularly useful.

The terms "identical" or percent "identity," in the context of two or more nucleic acids or polypeptide sequences, refer to two or more sequences or subsequences that are the same or have a specified percentage of amino acid residues or nucleotides that are the same (e.g., about 70% identity, preferably 75%, 80%, 85%, 90%, 91%, 93%, 95%, 97%, 98%, 99%, or higher identity over a specified region, when compared and aligned for maximum correspondence over a comparison window or designated region) as measured using, e.g., a BLAST or BLAST 2.0 sequence comparison algorithms with default parameters described below, or by manual alignment and visual inspection (see, e.g., NCBI web site http://www.ncbi.nlm.nih.gov/BLAST/ or the like). Such sequences are then said to be "substantially identical." This definition also refers to, or may be applied to, the complement of a test sequence. The definition also includes sequences that have deletions and/or insertions, substitutions, and naturally occurring, e.g., polymorphic or allelic variants, and man-made variants. As described below, the preferred algorithms can account for gaps and the like. Preferably, identity exists over a region that is at least about 25 amino acids or nucleotides in length, or more preferably over a region that is 50-100 amino acids or nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of contiguous positions selected from the group consisting typically of from 20 to 600, usually about 50 to about 200, more usually about 100 to about 150, in which a sequence may be compared to a reference sequence of the same number of contiguous positions after the two sequences are optimally aligned. Methods of alignment of sequences for comparison are well-known. Optimal alignment of sequences for comparison can be conducted, e.g., by the local homology algorithm of Smith and Waterman (1981) Adv. Appl. Math. 2:482-489, by the homology alignment algorithm of Needleman and Wunsch (1970) J. Mol. Biol. 48:443-453, by the search for similarity method of Pearson and Lipman (1988) Proc. Nat'l. Acad. Sci. USA 85:2444-2448, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Ausubel, et al. (eds. 1995 and supplements) Current Protocols in Molecular Biology Wiley).

Preferred examples of algorithms that are suitable for determining percent sequence identity and sequence similarity include the BLAST and BLAST 2.0 algorithms, which are described in Altschul, et al. (1977) Nuc. Acids Res. 25:3389-3402 and Altschul, et al. (1990) J. Mol. Biol. 215:403-410. BLAST and BLAST 2.0 are used, with the parameters described herein, to determine percent sequence identity for the nucleic acids and proteins of the invention. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul, et al., supra). These initial neighborhood word hits act as seeds for initiating searches to find longer HSPs containing them. The word hits are extended in both directions along each sequence for as far as the cumulative alignment score can be increased.-Cumulative scores are calculated using, e.g., for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (see Henikoff and Henikoff(1992) Proc. Natl. Acad. Sci. USA 89:10915-919) alignments (B) of 50, expectation (E) of 10, M=5, N=4, and a comparison of both strands.

The BLAST algorithm also performs a statistical analysis of the similarity between two sequences. See, e.g., Karlin and Altschul (1993) Proc. Nat'l. Acad. Sci. USA 90:5873-5787. One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001. Log values may be negative large numbers, e.g., 5, 10, 20, 30, 40, 40, 70, 90, 110, 150, 170, etc.

An indication that two nucleic acid sequences are substantially identical is that the polypeptide encoded by the first nucleic acid is immunologically cross reactive with the antibodies raised against the polypeptide encoded by the second nucleic acid. Thus, a polypeptide is typically substantially identical to a second polypeptide, e.g., where the two peptides differ only by conservative substitutions. Another indication that two nucleic acid sequences are substantially identical is that the two molecules or their complements hybridize to each other under stringent conditions. Yet another indication that two nucleic acid sequences are substantially identical is that the same primers can be used to amplify the sequences.

A "host cell" is a naturally occurring cell or a transformed cell that contains an expression vector and supports the replication or expression of the expression vector. Host cells may be cultured cells, explants, cells in vivo, and the like. Host cells may be prokaryotic cells such as E. coli, or eukaryotic cells such as yeast, insect, amphibian, or mammalian cells such as CHO, HeLa, and the like (see, e.g., the American Type Culture Collection catalog or web site, www.atcc.org).

The terms "isolated," "purified," or "biologically pure" refer to material that is substantially or essentially free from components that normally accompany it as found in its native state. Purity and homogeneity are typically determined using analytical chemistry techniques such as polyacrylamide gel electrophoresis or high performance liquid chromatography. A protein or nucleic acid that is the predominant species present in a preparation is substantially purified. In particular, an isolated nucleic acid is separated from some open reading frames that naturally flank the gene and encode proteins other than protein encoded by the gene. The term "purified" in some embodiments denotes that a nucleic acid or protein gives rise to essentially one band in an electrophoretic gel. Preferably, it means that the nucleic acid or protein is at least about 85% pure, more preferably at least 95% pure, and most preferably at least 99% pure. "Purify" or "purification" in other embodiments means removing at least one contaminant or component from the composition to be purified. In this sense, purification does not require that the purified compound be homogeneous, e.g., 100% pure.

The terms "polypeptide," "peptide," and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers, those containing modified residues, and non-naturally occurring amino acid polymers.

The term "amino acid" refers to naturally occurring and synthetic amino acids, as well as amino acid analogs and amino acid mimetics that function similarly to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, e.g., an α carbon that is bound to a hydrogen, a carboxyl group, an amino group, and an R group, e.g., homoserine, norleucine, methionine sulfoxide, methionine methyl sulfonium. Such analogs may have modified R groups (e.g., norleucine) or modified peptide backbones, but retain somebasic chemical structure as a naturally occurring amino acid. Amino acid mimetic refers to a chemical compound that has a structure that is different from the general chemical structure of an amino acid, but that functions similarly to another amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variant" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical or associated, e.g., naturally contiguous, sequences. Because of the degeneracy of the genetic code, a large number of functionally identical nucleic acids encode most proteins. For instance, the codons GCA, GCC, GCG, and GCU each encode the amino acid alanine. Thus, at each position where an alanine is specified by a codon, the codon can be altered to another of the corresponding codons described without altering the encoded polypeptide. Such nucleic acid variations are "silent variations," which are one species of conservatively modified variations. Every nucleic acid sequence herein which encodes a polypeptide also describes silent variations of the nucleic acid. In certain contexts each codon in a nucleic acid (except AUG, which is ordinarily the only codon for methionine, and TGG, which is ordinarily the only codon for tryptophan) can be modified to yield a functionally similar molecule. Accordingly, a silent variation of a nucleic acid which encodes a polypeptide is implicit in a described sequence with respect to the expression product, but not necessarily with respect to actual probe sequences.

As to amino acid sequences, one of skill will recognize that individual substitutions, deletions, or additions to a nucleic acid, peptide, polypeptide, or protein sequence which alters, adds, or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known. Such conservatively modified variants are in addition to and do not exclude polymorphic variants, interspecies homologs, and alleles of the invention. Typically conservative substitutions include for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton (1984) Proteins: Structure and Molecular Properties Freeman).

Macromolecular structures such as polypeptide structures can be described in terms of various levels of organization. For a general discussion of this organization, see, e.g., Alberts, et al. (eds. 2001) Molecular Biology of the Cell (4th ed.) Garland; and Cantor and Schimmel (1980) Biophysical Chemistry Part I: The Conformation of Biological Macromolecules Freeman. "Primary structure" refers to the amino acid sequence of a particular peptide. "Secondary structure" refers to locally ordered, three dimensional structures within a polypeptide. These structures are commonly known as domains. Domains are portions of a polypeptide that often form a compact unit of the polypeptide and are typically 25 to approximately 500 amino acids long. Typical domains are made up of sections of lesser organization such as stretches of β-sheet and α-helices. "Tertiary structure" refers to the complete three dimensional structure of a polypeptide monomer. "Quaternary structure" refers to the three dimensional structure formed, usually by the noncovalent association of independent tertiary units. Anisotropic terms are also known as energy terms.

"Nucleic acid" or "oligonucleotide" or "polynucleotide" or grammatical equivalents used herein means at least two nucleotides covalently linked together. Oligonucleotides are typically from about 5, 6, 7, 8, 9, 10, 12,15, 25, 30, 40, 50, or more nucleotides in length, up to about 100 nucleotides in length. Nucleic acids and polynucleotides are a polymers of any length, including longer lengths, e.g., 200, 300, 500, 1000, 2000, 3000, 5000, 7000, 10,000, etc. A nucleic acid of the present invention will generally contain phosphodiester bonds, although in some cases, nucleic acid analogs are included that may have at least one different linkahge, e.g., phosphoramidate, phosphorothioate, phosphorodithioate, or O-methylphophoroamidite linkages (see Eckstein (1992) Oligonucleotides and Analogues: A Practical Approach Oxford Univ. Press); and peptide nucleic acid backbones and linkages. Other analog nucleic acids include those with positive backbones; non-ionic backbones, and non-ribose backbones, including those described in U.S. Patent Nos. 5,235,033 and 5,034,506, and Chapters 6 and 7 of Sanghvi and Cook (eds. 1994) Carbohydrate Modifications in Antisense Research ACS Symposium Series 580. Nucleic acids containing one or more carbocyclic sugars are also included within one definition of nucleic acids. Modifications of the ribose-phosphate backbone may be done for a variety of reasons, e.g., to increase the stability and half-life of such molecules in physiological environments or as probes on a biochip. Mixtures of naturally occurring nucleic acids and analogs can be made; alternatively, mixtures of different nucleic acid analogs, and mixtures of naturally occurring nucleic acids and analogs may be made.

A variety of references disclose such nucleic acid analogs, including, e.g., phosphoramidate (Beaucage, et al. (1993) Tetrahedron 49:1925-1963 and references therein; Letsinger (1970) J. Org. Chem. 35:3800-3803; Sprinzl, et al. (1977) Eur. J. Biochem. 81:579-589; Letsinger, et al. (1986) Nucl. Acids Res. 14:3487-499; Sawai, et al. (1984) Chem. Lett. 805, Letsinger, et al. (1988) J. Am. Chem. Soc. 110:4470-4471; and Pauwels, et al. (1986) Chemica Scripta 26:141-149), phosphorothioate (Mag, et al. (1991) Nucleic Acids Res. 19:1437-441; and U.S. Patent No. 5,644,048), phosphorodithioate (Brill, et al. (1989) J. Am. Chem. Soc. 111:2321-2322), O-methylphophoroamidite linkages (see Eckstein (1992) Oligonucleotides and Analogues: A Practical Approach, Oxford Univ. Press), and peptide nucleic acid backbones and linkages (see Egholm (1992) J. Am. Chem. Soc. 114:1895-1897; Meier, et al. (1992) Chem. Int. Ed. Engl. 31:1008-1010; Nielsen (1993) Nature 365:566-568; Carlsson, et al. (1996) Nature 380:207, all of which are incorporated by reference). Other analog nucleic acids include those with positive backbones (Denpcy, et al. (1995) Proc. Natl. Acad. Sci. USA 92:6097-101; non-ionic backbones (U.S. Patent Nos. 5,386,023, 5,637,684, 5,602,240, 5,216,141, and 4,469,863; Kiedrowski, et al. (1991) Angew. Chem. Intl. Ed. English 30:423-426; Letsinger, et al. (1988) J. Am. Chem. Soc. 110:4470-4471; Letsinger, et al. (1994) Nucleoside and Nucleotide 13:1597-xxx; Chapters 2 and 3 in Sanghvi and Cook (eds. 1994) Carbohydrate Modifications in Antisense Research ACS Symposium Series 580; Mesmaeker, et al. (1994) Bioorganic and Medicinal Chem. Lett. 4:395-398; Jeffs, et al. (1994) J. Biomolecular NMR 34:17; Horn, et al. (1996) Tetrahedron Lett. 37:743-xxx) and non-ribose backbones, including those described in U.S. Patent Nos. 5,235,033 and 5,034,506, and Chapters 6 and 7 in Sanghvi and Cook (eds. 1994) Carbohydrate Modifications in Antisense Research ACS Symposium Series 580. Nucleic acids containing one or more carbocyclic sugars are also included within one definition of nucleic acids (see Jenkins, et al. (1995) Chem. Soc. Rev. pp 169-176). Several nucleic acid analogs are described in Rawls (page 35, June 2, 1997) C&E News.

Particularly preferred are peptide nucleic acids (PNA) which includes peptide nucleic acid analogs. These backbones are substantially non-ionic under neutral conditions, in contrast to the highly charged phosphodiester backbone of naturally occurring nucleic acids. This results in at least two advantages. The PNA backbone exhibits improved hybridization kinetics. PNAs have larger changes in the melting temperature (Tₘ) for mismatched versus perfectly matched basepairs. DNA and RNA typically exhibit a 2-4° C drop in Tₘ for an internal mismatch. With the non-ionic PNA backbone, the drop is closer to 7-9° C. Similarly, due to their non-ionic nature, hybridization of the bases attached to these backbones is relatively insensitive to salt concentration. In addition, PNAs are not degraded by cellular enzymes, and thus can be more stable.

The nucleic acids may be single stranded or double stranded, as specified, or contain portions of both double stranded or single stranded sequence. The depiction of a single strand also defines the sequence of the complementary strand; thus the sequences described herein also provide the complement of the sequence. The nucleic acid may be DNA, both genomic and cDNA, RNA, or a hybrid, where the nucleic acid may contain combinations of deoxyribo- and ribo-nucleotides, and combinations of bases, including uracil, adenine, thymine, cytosine, guanine, inosine, xanthine hypoxanthine, isocytosine, isoguanine, etc. "Transcript" typically refers to a naturally occurring RNA, e.g., a pre-mRNA, hnRNA, or mRNA. As used herein, the term "nucleoside" includes nucleotides and nucleoside and nucleotide analogs, and modified nucleosides such as amino modified nucleosides. In addition, "nucleoside" includes non-naturally occurring analog structures. Thus, e.g., the individual units of a peptide nucleic acid, each containing a base, are referred to herein as a nucleoside.

A "label" or a "detectable moiety" is a composition detectable by spectroscopic, photochemical, biochemical, immunochemical, physiological, chemical, or other physical means. In general, labels fall into three classes: a) isotopic labels, which may be radioactive or heavy isotopes; b) immune labels, which may be antibodies, antigens, or epitope tags; and c) colored or fluorescent dyes. The labels may be incorporated into the cancer nucleic acids, proteins, and antibodies. For example, the label should be capable of producing, either directly or indirectly, a detectable signal. The detectable moiety may be a radioisotope, such as ³H, ¹⁴C, ^{32p}, ³⁵S, or ¹²⁵I, electron-dense reagents, a fluorescent or chemiluminescent compound, such as fluorescein isothiocyanate, rhodamine, or luciferin, or an enzyme (e.g., as commonly used in an ELISA), biotin, digoxigenin, or haptens and proteins or other entities which can be made detectable such as alkaline phosphatase, beta-galactosidase, or horseradish peroxidase. Methods are known for conjugating the antibody to the label. See, e.g., Hunter, et al. (1962) Nature 144:945; David, et al. (1974) Biochemistry 13:1014-1021; Pain, et al. (1981) J. Immunol. Meth. 40:219-230; and Nygren (1982) J. Histochem. and Cytochem. 30:407-412.

An "effector" or "effector moiety" or "effector component" is a molecule that is bound (or linked, or conjugated), either covalently, through a linker or a chemical bond, or noncovalently, through ionic, van der Waals, electrostatic, or hydrogen bonds, to an antibody. The "effector" can be a variety of molecules including, e.g., detection moieties including radioactive compounds, fluorescent compounds, enzymes or substrates, tags such as epitope tags, toxins; activatable moieties, chemotherapeutic agents; lipases; antibiotics; or radioisotopes, e.g., emitting "hard" beta, radiation.

A "labeled nucleic acid probe or oligonucleotide" is one that is bound, e.g., covalently, through a linker or a chemical bond, or noncovalently, through ionic, van der Waals, electrostatic, or hydrogen bonds to a label such that the presence of the probe may be detected by detecting the presence of the label bound to the probe. Alternatively, methods using high affinity interactions may achieve the same results where one of a pair of binding partners binds to the other, e.g., biotin, streptavidin.

As used herein a "nucleic acid probe or oligonucleotide" is a nucleic acid capable of binding to a target nucleic acid of complementary sequence through one or more types of chemical bonds, usually through complementary base pairing, e.g., through hydrogen bond formation. As used herein, a probe may include natural (e.g., A, G, C, or T) or modified bases (7-deazaguanosine, inosine, etc.). In addition, the bases in a probe may be joined by a linkage other than a phosphodiester bond, preferably one that does not functionally interfere with hybridization. Thus, e.g., probes may be peptide nucleic acids in which the constituent bases are joined by peptide bonds rather than phosphodiester linkages. Probes may bind target sequences lacking complete complementarity with the probe sequence depending upon the stringency of the hybridization conditions. The probes are preferably directly labeled, e.g., with isotopes, chromophores, lumiphores, chromogens, or indirectly labeled, e.g., with biotin to which a streptavidin complex may later bind. By assaying for the presence or absence of the probe, one can detect the presence or absence of the select sequence or subsequence. Diagnosis or prognosis may be based at the genomic level, or at the level of RNA or protein expression.

The term "recombinant" when used with reference, e.g., to a cell, or nucleic acid, protein, or vector, indicates that the cell, nucleic acid, protein, or vector, has been modified by the introduction of a heterologous nucleic acid or protein or the alteration of a native nucleic acid or protein, or that the cell is derived from a cell so modified. Thus, e.g., recombinant cells express genes that are not found within the native (non-recombinant) form of the cell or express native genes that are otherwise abnormally expressed, under expressed, or not expressed at all. By the term "recombinant nucleic acid" herein is meant nucleic acid, originally formed in vitro, in general, by the manipulation of nucleic acid, e.g., using polymerases and endonucleases, in a form not normally found in nature. In this manner, operably linkage of different sequences is achieved. Thus an isolated nucleic acid, in a linear form, or an expression vector formed in vitro by ligating DNA molecules that are not normally joined, are both considered recombinant for the purposes of this invention. It is understood that once a recombinant nucleic acid is made and reintroduced into a host cell or organism, it will replicate non-recombinantly, e.g., using the in vivo cellular machinery of the host cell rather than in vitro manipulations; however, such nucleic acids, once produced recombinantly, although subsequently replicated non-recombinantly, are still considered recombinant for the purposes of the invention.

Similarly, a "recombinant protein" is a protein made using recombinant techniques, e.g., through the expression of a recombinant nucleic acid as depicted above. A recombinant protein is distinguished from naturally occurring protein by at least one or more characteristics. The protein may be isolated or purified away from some or most of the proteins and compounds with which it is normally associated in its wild type host, and thus may be substantially pure. An isolated protein is unaccompanied by at least some of the material with which it is normally associated in its natural state, preferably constituting at least about 0.5%, more preferably at least about 5% by weight of the total protein in a given sample. A substantially pure protein comprises at least about 75% by weight of the total protein, with at least about 80% being preferred, and at least about 90% being particularly preferred. The definition includes the production of a skin cancer protein from one organism in a different organism or host cell. Alternatively, the protein may be made at a significantly higher concentration than is normally seen, through the use of an inducible promoter or high expression promoter, such that the protein is made at increased concentration levels. Alternatively, the protein may be in a form not normally found in nature, as in the addition of an epitope tag or amino acid substitutions, insertions and deletions, as discussed below.

The term "heterologous" when used with reference to portions of a nucleic acid indicates that the nucleic acid comprises two or more subsequences that are not normally found in the same relationship to each other in nature. For instance, the nucleic acid is typically recombinantly produced, having two or more sequences, e.g., from unrelated genes arranged to make a new functional nucleic acid, e.g., a promoter from one source and a coding region from another source. Similarly, a heterologous protein will often refer to two or more subsequences that are not found in the same relationship to each other in nature (e.g., a fusion protein).

A "promoter" is typically an array of nucleic acid control sequences that direct transcription of a nucleic acid. As used herein, a promoter includes necessary nucleic acid sequences near the start site of transcription, such as, in the case of a polymerase II type promoter, a TATA element. A promoter also optionally includes distal enhancer or repressor elements, which can be located as much as several thousand base pairs from the start site of transcription. A "constitutive" promoter is a promoter that is active under most environmental and developmental conditions. An "inducible" promoter is a promoter that is active under environmental or developmental regulation. The term "operably linked" refers to a functional linkage between a nucleic acid expression control sequence (such as a promoter, or array of transcription factor binding sites) and a second nucleic acid sequence, e.g., wherein the expression control sequence directs transcription of the nucleic acid corresponding to the second sequence.

An "expression vector" is a nucleic acid construct, generated recombinantly or synthetically, with a series of specified nucleic acid elements that permit transcription of a particular nucleic acid in a host cell. The expression vector can be part of a plasmid, virus, or nucleic acid fragment. Typically, the expression vector includes a nucleic acid to be transcribed in operable linkage to a promoter.

The phrase "selectively (or specifically) hybridizes to" refers to the binding, duplexing, or hybridizing of a molecule selectively to a particular nucleotide sequence under stringent hybridization conditions when that sequence is present in a complex mixture (e.g., total cellular or library DNA or RNA).

The phrase "stringent hybridization conditions" refers to conditions under which a probe will hybridize to its target subsequence, typically in a complex mixture of nucleic acids, but to no other sequences. Stringent conditions are sequence-dependent and will be different in different circumstances. Longer sequences hybridize specifically at higher temperatures. An extensive guide to the hybridization of nucleic acids is found in "Overview of principles of hybridization and the strategy of nucleic acid assays" in Tijssen (1993) Hybridization with Nucleic Probes (Laboratory Techniques in Biochemistry and Molecular Biology) (vol. 24) Elsevier. Generally, stringent conditions are selected to be about 5-10° C lower than the thermal melting point (Tₘ) for the specific sequence at a defined ionic strength pH. The Tₘ is the temperature (under defined ionic strength, pH, and nucleic concentration) at which 50% of the probes complementary to the target hybridize to the target sequence at equilibrium (as the target sequences are present in excess, at Tₘ, 50% of the probes are occupied at equilibrium). Stringent conditions will be those in which the salt concentration is less than about 1.0 M sodium ion, typically about 0.01-1.0 M sodium ion concentration (or other salts) at pH 7.0 to 8.3 and the temperature is at least about 30° C for short probes (e.g., about 10-50 nucleotides) and at least about 60° C for long probes (e.g., greater than about 50 nucleotides). Stringent conditions may also be achieved with the addition of destabilizing agents such as formamide. For selective or specific hybridization, a positive signal is typically at least two times background, preferably 10 times background hybridization. Exemplary stringent hybridization conditions can be as following: 50% formamide, 5x SSC, and 1% SDS, incubating at 42° C, or, 5x SSC, 1% SDS, incubating at 65° C, with wash in 0.2x SSC, and 0.1% SDS at 65° C. For PCR, a temperature of about 36° C is typical for low stringency amplification, although annealing temperatures may vary between about 32°-48° C depending on primer length. For high stringency PCR amplification, a temperature of about 62° C is typical, although high stringency annealing temperatures can range from about 50-65° C, depending on the primer length and specificity. Typical cycle conditions for both high and low stringency amplifications include a denaturation phase of 90-95° C for 30-120 sec, an annealing phase lasting 30-120 sec, and an extension phase of about 72° C for 1-2 min. Protocols and guidelines for low and high stringency amplification reactions are provided, e.g., in Innis, et al. (1990) PCR Protocols: A Guide to Methods and Applications, Academic Press, NY.

Nucleic acids that do not hybridize to each other under stringent conditions are still substantially identical if the polypeptides which they encode are substantially identical. This occurs, e.g., when a copy of a nucleic acid is created using the maximum codon degeneracy permitted by the genetic code. In such cases, the nucleic acids typically hybridize under moderately stringent hybridization conditions. Exemplary "moderately stringent hybridization conditions" include a hybridization in a buffer of 40% formamide, 1 M NaCl, 1% SDS at 37° C, and a wash in 1X SSC at 45° C. A positive hybridization is typically at least twice background. Alternative hybridization and wash conditions can be utilized to provide conditions of similar stringency. Additional guidelines for determining hybridization parameters are provided in numerous references, e.g., Ausubel, et al. (eds. 1991 and supplements) Current Protocols in Molecular Biology Wiley.

The phrase "functional effects" in the context of assays for testing compounds that modulate activity of a cancer protein includes the determination of a parameter that is indirectly or directly under the influence of the cancer protein or nucleic acid, e.g., a physiological, functional, physical, or chemical effect, such as the ability to decrease cancer. It includes ligand binding activity; cell viability; cell growth on soft agar; anchorage dependence; contact inhibition and density limitation of growth; cellular proliferation; cellular transformation; growth factor or serum dependence; tumor specific marker levels; invasiveness into Matrigel; tumor growth and metastasis in vivo; mRNA and protein expression in cells undergoing metastasis; and other characteristics of cancer cells. "Functional effects" include in vitro, in vivo, and ex vivo activities.

By "determining the functional effect" is meant assaying for a compound that increases or decreases a parameter that is indirectly or directly under the influence of a cancer protein sequence, e.g., physiological, functional, enzymatic, physical, or chemical effects. Such functional effects can be measured, e.g., changes in spectroscopic characteristics (e.g., fluorescence, absorbance, refractive index), hydrodynamic (e.g., shape), chromatographic, or solubility properties for the protein, measuring inducible markers or transcriptional activation of the cancer protein, measuring binding activity or binding assays, e.g., binding to antibodies or other ligands, and measuring growth, cellular proliferation, cell viability, cellular transformation, growth factor or serum dependence, tumor specific marker levels, invasiveness into Matrigel, tumor growth and metastasis in vivo, mRNA and protein expression, and other characteristics of cancer cells. The functional effects can be evaluated by many means, e.g., microscopy for quantitative or qualitative measures of alterations in morphological features, measurement of changes in RNA or protein levels for cancer-associated sequences, measurement of RNA stability, identification of downstream or reporter gene expression (CAT, luciferase, β-gal, GFP, and the like), e.g., via chemiluminescence, fluorescence, colorimetric reactions, antibody binding, inducible markers, and ligand binding assays.

"Inhibitors", "activators," and "modulators" of cancer polynucleotide and polypeptide sequences are used to refer to activating, inhibitory, or modulating molecules or compounds identified using in vitro and in vivo assays of cancer polynucleotide and polypeptide sequences. Inhibitors are compounds that, e.g., bind to, partially or totally block activity, decrease, prevent, delay activation, inactivate, desensitize, or down regulate the activity or expression of cancer proteins, e.g., antagonists. Antisense or inhibitory nucleic acids may seem to inhibit expression and subsequent function of the protein. "Activators" are compounds that increase, open, activate, facilitate, enhance activation, sensitize, agonize, or up regulate cancer protein activity. Inhibitors, activators, or modulators also include genetically modified versions of cancer proteins, e.g., versions with altered activity, as well as naturally occurring and synthetic ligands, antagonists, agonists, antibodies, small chemical molecules, and the like. Such assays for inhibitors and activators include, e.g., expressing the cancer protein in vitro, in cells, or cell membranes, applying putative modulator compounds, and then determining the functional effects on activity, as described above. Activators and inhibitors of cancer can also be identified by incubating cancer cells with the test compound and determining increases or decreases in the expression of 1 or more cancer proteins, e.g., 1,2, 3,4, 5,10,15,20,25, 30, 40, 50, or more cancer proteins, such as cancer proteins encoded by the sequences set out in Tables 1-68.

Samples or assays comprising cancer proteins that are treated with a potential activator, inhibitor, or modulator are compared to control samples without the inhibitor, activator, or modulator to examine the extent of inhibition. Control samples (untreated with inhibitors) are assigned a relative protein activity value of 100%. Inhibition of a polypeptide is achieved when the activity value relative to the control is about 80%, preferably 50%, more preferably 25-0%. Activation of a cancer polypeptide is achieved when the activity value relative to the control (untreated with activators) is 110%, more preferably 150%, more preferably 200-500% (e.g., two to five fold higher relative to the control), more preferably 1000-3000% higher.

The phrase "changes in cell growth" refers to any change in cell growth and proliferation characteristics in vitro or in vivo, such as cell viability, formation of foci, anchorage independence, semi-solid or soft agar growth, changes in contact inhibition and density limitation of growth, loss of growth factor or serum requirements, changes in cell morphology, gaining or losing immortalization, gaining or losing tumor specific markers, ability to form or suppress tumors when injected into suitable animal hosts, and/or immortalization of the cell. See, e.g., pp. 231-241 in Freshney (1994) Culture of Animal Cells a Manual of Basic Technique (2d ed.) Wiley-Liss.

"Tumor cell" refers to precancerous, cancerous, and normal cells in a tumor.

"Cancer cells," "transformed" cells or "transformation" in tissue culture, refers to spontaneous or induced phenotypic changes that do not necessarily involve the uptake of new genetic material. Although transformation can arise from infection with a transforming virus and incorporation of new genomic DNA, or uptake of exogenous DNA, it can also arise spontaneously or following exposure to a carcinogen, thereby mutating an endogenous gene. Transformation is associated with phenotypic changes, such as immortalization of cells, aberrant growth control, nonmorphological changes, and/or malignancy. See, Freshney (2000) Culture of Animal Cells: A Manual of Basic Technique (4th ed.) Wiley-Liss.

"Antibody" refers to a polypeptide comprising a framework region from an immunoglobulin gene or fragments thereof that specifically binds and recognizes an antigen. The recognized immunoglobulin genes include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively. Typically, the antigen-binding region of an antibody or its functional equivalent will be most critical in specificity and affinity of binding. See Paul (ed. 1999) Fundamental Immunology (4th ed.) Raven.

An exemplary immunoglobulin (antibody) structural unit comprises a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

Antibodies exist, e.g., as intact immunoglobulins or as a number of well-characterized fragments produced by digestion with various peptidases. Thus, e.g., pepsin digests an antibody below the disulfide linkages in the hinge region to produce F(ab)'₂, a dimer of Fab which itself is a light chain joined to V_{H}-C_{H}1 by a disulfide bond. The F(ab)'₂ may be reduced under mild conditions to break the disulfide linkage in the hinge region, thereby converting the F(ab)'₂ dimer into an Fab' monomer. The Fab' monomer is essentially Fab with part of the hinge region (see Paul (ed. 1999) Fundamental Immunology (4th ed.) Raven. While various antibody fragments are defined in terms of the digestion of an intact antibody, one of skill will appreciate that such fragments may be synthesized de novo either chemically or by using recombinant DNA methodology. Thus, the term antibody, as used herein, also includes antibody fragments either produced by the modification of whole antibodies, or those synthesized de novo using recombinant DNA methodologies (e.g., single chain Fv) or those identified using phage display libraries (see, e.g., McCafferty, et al. (1990) Nature 348:552-554).

For preparation of antibodies, e.g., recombinant, monoclonal, or polyclonal antibodies, many techniques known. See, e.g., Kohler and Milstein (1975) Nature 256:495-497; Kozbor, et al. (1983) Immunology Today 4:72; Cole, et al. (1985) pp. 77-96 in Reisfeld and Sell (1985) Monoclonal Antibodies and Cancer Therapy Liss; Coligan (1991) Current Protocols in Immunology Lippincott; Harlow and Lane (1988) Antibodies: A Laboratory Manual CSH Press; and Goding (1986) Monoclonal Antibodies: Principles and Practice (2d ed.) Academic Press. Techniques for the production of single chain antibodies (U.S. Patent 4,946,778) can be adapted to produce antibodies to polypeptides of this invention. Also, transgenic mice, or other organisms such as other mammals, may be used to express humanized antibodies. Alternatively, phage display technology can be used to identify antibodies and heteromeric Fab fragments that specifically bind to selected antigens. See, e.g., McCafferty, et al. (1990) Nature 348:552-554; Marks, et al. (1992) Biotechnology 10:779-783.

A "chimeric antibody" is an antibody molecule in which (a) the constant region, or a portion thereof, is altered, replaced, or exchanged so that the antigen binding site (variable region) is linked to a constant region of a different or altered class, effector function, and/or species, or an entirely different molecule which confers new properties to the chimeric antibody, e.g., an enzyme, toxin, hormone, growth factor, drug, etc.; or (b) the variable region, or a portion thereof, is altered, replaced, or exchanged with a variable region having a different or altered antigen specificity. Identification of cancer-associated sequences

In one aspect, the expression levels of genes are determined in different patient samples for which diagnosis information is desired, to provide expression profiles. An expression profile of a particular sample is essentially a "fingerprint" of the state of the sample; while two states may have any particular gene similarly expressed, the evaluation of a number of genes simultaneously allows the generation of a gene expression profile that is characteristic of the state of the cell. That is, normal tissue may be distinguished from cancerous or metastatic cancerous tissue, or cancer tissue or metastatic cancerous tissue can be compared with tissue from surviving cancer patients. By comparing expression profiles of tissue in known different cancer states, information regarding which genes are important (including both up-and down-regulation of genes) in each of these states is obtained. Molecular profiling may distinguish subtypes of a currently collective disease designation, e.g., different forms of a cancer.

The identification of sequences that are differentially expressed in cancer versus non-cancer tissue allows the use of this information in a number of ways. For example, a particular treatment regime may be evaluated: does a chemotherapeutic drug act to down-regulate cancer, and thus tumor growth or recurrence, in a particular patient. Alternatively, a treatment step may induce other markers which may be used as targets to destroy tumor cells. Similarly, diagnosis and treatment outcomes may be done or confirmed by comparing patient samples with the known expression profiles. Maliganant disease may be compared to non-malignant conditions. Metastatic tissue can also be analyzed to determine the stage of cancer in the tissue, or origin of primary tumor, e.g., metastasis from a remote primary site. Furthermore, these gene expression profiles (or individual genes) allow screening of drug candidates with an eye to mimicking or altering a particular expression profile; e.g., screening can be done for drugs that suppress the cancer expression profile. This may be done by making biochips comprising sets of the important cancer genes, which can then be used in these screens. These methods can also be done on the protein basis; that is, protein expression levels of the cancer proteins can be evaluated for diagnostic purposes or to screen candidate agents. In addition, the cancer nucleic acid sequences can be administered for gene therapy purposes, including the administration of antisense nucleic acids, or the cancer proteins (including antibodies and other modulators thereof) administered as therapeutic drugs.

Thus the present invention provides nucleic acid and protein sequences that are differentially expressed in cancer relative to normal tissues and/or non-malignant disease, or in different types of related diseases, herein termed "cancer sequences." As outlined below, cancer sequences include those that are up-regulated (e.g., expressed at a higher level) in cancer, as well as those that are down-regulated (e.g., expressed at a lower level). In a preferred embodiment, the cancer sequences are from humans; however, cancer sequences from other organisms may be useful in animal models of disease and drug evaluation; thus, other cancer sequences are provided, from vertebrates, including mammals, including rodents (rats, mice, hamsters, guinea pigs, etc.), primates, farm animals (including sheep, goats, pigs, cows, horses, etc.) and pets (e.g., dogs, cats, etc.). Cancer sequences from other organisms may be obtained using the techniques outlined below.

Cancer sequences can include both nucleic acid and amino acid sequences. In a preferred embodiment, the skin cancer sequences are recombinant nucleic acids. These nucleic acid sequences are useful in a variety of applications, including diagnostic applications, which will detect naturally occurring nucleic acids, as well as screening applications; e.g., biochips comprising nucleic acid probes or PCR microtiter plates with selected probes to the cancer sequences.

A cancer sequence can be initially identified by substantial nucleic acid and/or amino acid sequence homology to the cancer sequences outlined herein. Such homology can be based upon the overall nucleic acid or amino acid sequence, and is generally determined as outlined below, e.g., using homology programs or hybridization conditions.

For identifying cancer-associated sequences, the cancer screen typically includes comparing genes identified in different tissues, e.g., normal and cancerous tissues, cancer and non-malignant conditions, non-malignant conditions and normal tissues, or tumor tissue samples from patients who have metastatic disease vs. non metastatic tissue. Other suitable tissue comparisons include comparing cancer samples with metastatic cancer samples from other cancers, such as lung, stomach, gastrointestinal cancers, etc. Samples of different stages of cancer, e.g., survivor tissue, drug resistant states, and tissue undergoing metastasis, are applied to biochips comprising nucleic acid probes. The samples are first microdissected, if applicable, and treated for preparation of mRNA. Suitable biochips are commercially available, e.g., from Affymetrix, Santa Clara, CA. Gene expression profiles as described herein are generated and the data analyzed.

In one embodiment, the genes showing changes in expression as between normal and disease states are compared to genes expressed in other normal tissues, including, and not limited to lung, heart, brain, liver, stomach, kidney, muscle, colon, small intestine, large intestine, spleen, bone, and/or placenta. In a preferred embodiment, those genes identified during the cancer screen that are expressed in a significant amount in other tissues (e.g., essential organs) are removed from the profile, although in some embodiments, this is not necessary (e.g., where organs may be dispensible, e.g., female or male specific). That is, when screening for drugs, it is usually preferable that the target expression be disease specific, to minimize possible side effects on other organs were there expression.

In a preferred embodiment, cancer sequences are those that are up-regulated in cancer; that is, the expression of these genes is higher in the cancer tissue as compared to non-cancer or non-malignant tissue. "Up-regulation" as used herein often means at least about a two-fold change, preferably at least about a three fold change, with at least about five-fold or higher being preferred. Another embodiment is directed to sequences up-regulated in non-malignant conditions relative to normal. Uniformity among relevant samples is also preferred.

Unigene cluster identification numbers and accession numbers herein are for the GenBank sequence database and the sequences of the accession numbers are hereby expressly incorporated by reference. GenBank is available, see, e.g., Benson, et al. (1998) Nuc. Acids Res. 26:1-7; and http://www.ncbi.nhn.nih.gov/. Sequences are also available in other databases, e.g., European Molecular Biology Laboratory (EMBL) and DNA Database of Japan (DDBJ). In some situations, the sequences may be derived from assembly of available sequences or be predicted from genomic DNA using exon prediction algorithms, such as FGENESH. See Salamov and Solovyev (2000) Genome Res. 10:516-522. In other situations, sequences have been derived from cloning and sequencing of isolated nucleic acids.

In another preferred embodiment, cancer sequences are those that are down-regulated in the cancer; that is, the expression of these genes is lower in cancer tissue as compared to non-cancerous tissue. "Down-regulation" as used herein often means at least about a two-fold change, preferably at least about a three fold change, with at least about five-fold or higher being preferred. Informatics

The ability to identify genes that are over or under expressed in cancer can additionally provide high-resolution, high-sensitivity datasets which can be used in the areas of diagnostics, therapeutics, drug development, pharmacogenetics, protein structure, biosensor development, and other related areas. For example, the expression profiles can be used in diagnostic or prognostic evaluation of patients with cancer or related diseases. See Tables 1 and 3. Or as another example, subcellular toxicological information can be generated to better direct drug structure and activity correlation (see Anderson (June 11-12, 1998) Pharmaceutical Proteomics: Targets, Mechanism, and Function, paper presented at the IBC Proteomics conference, Coronado, CA). Subcellular toxicological information can also be utilized in a biological sensor device to predict the likely toxicological effect of chemical exposures and likely tolerable exposure thresholds (see U.S. Patent No. 5,811,231). Similar advantages accrue from datasets relevant to other biomolecules and bioactive agents (e.g., nucleic acids, saccharides, lipids, drugs, and the like).

Thus, in another embodiment, the present invention provides a database that includes at least one set of assay data. The data contained in the database is acquired, e.g., using array analysis either singly or in a library format. The database can be in a form in which data can be maintained and transmitted, but is preferably an electronic database. The electronic database of the invention can be maintained on any electronic device allowing for the storage of and access to the database, such as a personal computer, but is preferably distributed on a wide area network, such as the World Wide Web.

The focus of the present section on databases that include peptide sequence data is for clarity of illustration only. Similar databases can be assembled for assay data acquired using an assay of the invention.

The compositions and methods for identifying and/or quantitating the relative and/or absolute abundance of a variety of molecular and macromolecular species from a biological sample representing cancer, e.g., the identification of cancer-associated sequences described herein, provide an abundance of information which can be correlated with pathological conditions, predisposition to disease, drug testing, therapeutic monitoring, gene-disease causal linkages, identification of correlates of immunity and physiological status, among others. Although the data generated from the assays of the invention is suited for manual review and analysis, in a preferred embodiment, data processing using high-speed computers is utilized.

An array of methods for indexing and retrieving biomolecular information is available. For example, U.S. Patents 6,023,659 and 5,966,712 disclose a relational database'system for storing biomolecular sequence information in a manner that allows sequences to be catalogued and searched according to one or more protein function hierarchies. U.S. Patent 5,953,727 discloses a relational database having sequence records containing information in a format that allows a collection of partial-length DNA sequences to be catalogued and searched according to association with one or more sequencing projects for obtaining full-length sequences from the collection of partial length sequences. U.S. Patent 5,706,498 discloses a gene database retrieval system for making a retrieval of a gene sequence similar to a sequence data item in a gene database based on the degree of similarity between a key sequence and a target sequence. U.S: Patent 5,538,897 discloses a method using mass spectroscopy fragmentation patterns of peptides to identify amino acid sequences in computer databases by comparison of predicted mass spectra with experimentally-derived mass spectra using a closeness-of-fit measure. U.S. Patent 5,926,818 discloses a multi-dimensional database comprising a functionality for multi-dimensional data analysis described as on-line analytical processing (OLAP), which entails the consolidation of projected and actual data according to more than one consolidation path or dimension. U.S. Patent 5,295,261 reports a hybrid database structure in which the fields of each database record are divided into two classes, navigational and informational data, with navigational fields stored in a hierarchical topological map which can be viewed as a tree structure or as the merger of two or more such tree structures. See also Baxevanis, et al. (2001) Bioinformatics: A Practical Guuide to the Analysis of Genes and Proteins Wiley; Mount (2001) Bioinformatics: Sequence and Genome Analysis CSH Press, NY; Durbin, et al. (eds. 1999) Biological Sequence Analysis: Probabilistic Models of Proteins and Nucleic Acids Cambridge University Press; Baxevanis and Oeullette (eds. 1998) Bioinformatics: A Practical Guide to the Analysis of Genes and Proteins (2d. ed.) Wiley-Liss; Rashidi and Buehler (1999) Bioinformatics: Basic Applications in Biological Science and Medicine CRC Press; Setubal, et al. (eds. 1997) Introduction to Computational Molecular Biology Brooks/Cole; Misener and Krawetz (eds. 2000) Bioinformatics: Methods and Protocols Humana Press; Higgins and Taylor (eds. 2000) Bioinformatics: Sequence, Structure, and Databanks: A Practical Approach Oxford University Press; Brown (2001) Bioinformatics: A Biologist's Guide to Biocomputing and the Internet Eaton Pub.; Han and Kamber (2000) Data Mining: Concepts and Techniques Kaufmann Pub.; and Waterman (1995) Introduction to Computational Biology: Maps, Sequences, and Genomes Chap and Hall.

The present invention provides a computer database comprising a computer and software for storing in computer-retrievable form assay data records cross-tabulated, e.g., with data specifying the source of the target-containing sample from which each sequence specificity record was obtained.

In an exemplary embodiment, at least one of the sources of target-containing sample is from a control tissue sample known to be free of pathological disorders. In a variation, at least one of the sources is a known pathological tissue specimen, e.g., a neoplastic lesion or another tissue specimen to be analyzed for cancer. In another variation, the assay records cross-tabulate one or more of the following parameters for each target species in a sample: (1) a unique identification code, which can include, e.g., a target molecular structure and/or characteristic separation coordinate (e.g., electrophoretic coordinates); (2) sample source; and (3) absolute and/or relative quantity of the target species present in the sample.

The invention also provides for the storage and retrieval of a collection of target data in a computer data storage apparatus, which can include magnetic disks, optical disks, magnetooptical disks, DRAM, SRAM, SGRAM, SDRAM, RDRAM, DDR RAM, magnetic bubble memory devices, and other data storage devices, including CPU registers and on-CPU data storage arrays. Typically, the target data records are stored as a bit pattern in an array of magnetic domains on a magnetizable medium or as an array of charge states or transistor gate states, such as an array of cells in a DRAM device (e.g., each cell comprised of a transistor and a charge storage area, which may be on the transistor). In one embodiment, the invention provides such storage devices, and computer systems built therewith, comprising a bit pattern encoding a protein expression fingerprint record comprising unique identifiers for at least 10 target data records cross-tabulated with target source.

When the target is a peptide or nucleic acid, the invention preferably provides a method for identifying related peptide or nucleic acid sequences, comprising performing a computerized comparison between a peptide or nucleic acid sequence assay record stored in or retrieved from a computer storage device or database and at least one other sequence. The comparison can include a sequence analysis or comparison algorithm or computer program embodiment thereof (e.g., FASTA, TFASTA, GAP, BESTFIT) and/or the comparison may be of the relative amount of a peptide or nucleic acid sequence in a pool of sequences determined from a polypeptide or nucleic acid sample of a specimen.

The invention also preferably provides a magnetic disk, such as an IBM-compatible (DOS, Windows, Windows95/98/2000, Windows NT, OS/2) or other format (e.g., Linux, SunOS, Solaris, AIX, SCO Unix, VMS, MV, Macintosh, etc.) floppy diskette or hard (fixed, Winchester) disk drive, comprising a bit pattern encoding data from an assay of the invention in a file format suitable for retrieval and processing in a computerized sequence analysis, comparison, or relative quantitation method.

The invention also provides a network, comprising a plurality of computing devices linked via a data link, such as an Ethernet cable (coax or 10BaseT), telephone line, ISDN line, wireless network, optical fiber, or other suitable signal transmission medium, whereby at least one network device (e.g., computer, disk array, etc.) comprises a pattern of magnetic domains (e.g., magnetic disk) and/or charge domains (e.g., an array of DRAM cells) composing a bit pattern encoding data acquired from an assay of the invention.

The invention also provides a method for transmitting assay data that includes generating an electronic signal on an electronic communications device, such as a modem, ISDN terminal adapter, DSL, cable modem, ATM switch, or the like, wherein the signal includes (in native or encrypted format) a bit pattern encoding data from an assay or a database comprising a plurality of assay results obtained by the method of the invention.

In a preferred embodiment, the invention provides a computer system for comparing a query target to a database containing an array of data structures, such as an assay result obtained by the method of the invention, and ranking database targets based on the degree of identity and gap weight to the target data. A central processor is preferably initialized to load and execute the computer program for alignment and/or comparison of the assay results. Data for a query target is entered into the central processor via an I/O device. Execution of the computer program results in the central processor retrieving the assay data from the data file, which comprises a binary description of an assay result.

The target data or record and the computer program can be transferred to secondary memory, which is typically random access memory (e.g., DRAM, SRAM, SGRAM, or SDRAM). Targets are ranked according to the degree of correspondence between a selected assay characteristic (e.g., binding to a selected affinity moiety) and the same characteristic of the query target and results are output via an I/O device. For example, a central processor can be a conventional computer (e.g., Intel Pentium, PowerPC, Alpha, PA-8000, SPARC, MIPS 4400, MIPS 10000, VAX, etc.); a program can be a commercial or public domain molecular biology software package (e.g., UWGCG Sequence Analysis Software, Darwin); a data file can be an optical or magnetic disk, a data server, a memory device (e.g., DRAM, SRAM, SGRAM, SDRAM, EPROM, bubble memory, flash memory, etc.); an I/O device can be a terminal comprising a video display and a keyboard, a modem, an ISDN terminal adapter, an Ethernet port, a punched card reader, a magnetic strip reader, or other suitable I/O device.

The invention also preferably provides the use of a computer system, such as that described above, which comprises: (1) a computer; (2) a stored bit pattern encoding a collection of peptide sequence specificity records obtained by the methods of the invention, which may be stored in the computer; (3) a comparison target, such as a query target; and (4) a program for alignment and comparison, typically with rank-ordering of comparison results on the basis of computed similarity values. See, e.g., Ewens and Grant (2001) Statistical Methods in Bioinformatics: An Introduction Springer-Verlag. Mathematical approaches can also be used to conclude whether similarities or differences in the gene expression exhibited by different samples are significant. See, e.g., Golub, et al. (1999) Science 286:531-537; Duda, et al. (2001) Pattern Classification Wiley; and Hastie, et al. (2001) The Elements of Statistical Learning: Data Mining, Inference, and Prediction Springer-Verlag. One approach to determine whether a sample is more similar to or has maximum similarity with a given condition between the sample and one or more pools representing different conditions for comparison; the pool with the smallest vector angle is then chosen as the most similar to the biological sample among the pools compared. Characteristics of cancer-associated proteins

Cancer proteins of the present invention may be classified as secreted proteins, transmembrane proteins, or intracellular proteins. In one embodiment, the cancer protein is an intracellular protein. Intracellular proteins may be found in the cytoplasm and/or in the nucleus. Intracellular proteins are involved in all aspects of cellular function and replication (including, e.g., signaling pathways); aberrant expression of such proteins often results in unregulated or disregulated cellular processes (see, e.g., Alberts, et al. (eds. 1994) Molecular Biology of the Cell (3d ed.) Garland). For example, many intracellular proteins have enzymatic activity such as protein kinase activity, protein phosphatase activity, protease activity, nucleotide cyclase activity, polymerase activity, and the like. Intracellular proteins also serve as docking proteins that are involved in organizing complexes of proteins, or targeting proteins to various subcellular localizations, and are involved in maintaining the structural integrity of organelles.

An increasingly appreciated concept in characterizing proteins is the presence in the proteins of one or more structural motifs for which defined functions have been attributed. In addition to the highly conserved sequences found in the enzymatic domain of proteins, highly conserved sequences have been identified in proteins that are involved in protein-protein interaction. For example, Src-homology-2 (SH2) domains bind tyrosine-phosphorylated targets in a sequence dependent manner. PTB domains, which are distinct from SH2 domains, also bind tyrosine phosphorylated targets. SH3 domains bind to proline-rich targets. In addition, PH domains, tetratricopeptide repeats and WD domains to name only a few, have been shown to mediate protein-protein interactions. Some of these may also be involved in binding to phospholipids or other second messengers. These motifs can be identified on the basis of amino acid sequence; thus, an analysis of the sequence of proteins may provide insight into both the enzymatic potential of the molecule and/or molecules with which the protein may associate. One useful database is Pfam (protein families), which is a large collection of multiple sequence alignments and hidden Markov models covering many common protein domains. Versions are available via the internet from Washington University in St. Louis, the Sanger Center in England, and the Karolinska Institute in Sweden. See, e.g., Bateman, et al. (2000) Nuc. Acids Res. 28:263-266; Sonnhammer, et al. (1997) Proteins 28:405-420 ; Bateman, et al. (1999) Nuc. Acids Res. 27:260-262; and Sonnhammer, et al. (1998) Nuc. Acids Res. 26:320-322.

In another embodiment, the cancer sequences are transmembrane proteins. Transmembrane proteins are molecules that span a phospholipid bilayer of a cell. They may have an intracellular domain, an extracellular domain, or both. The intracellular domains of such proteins may have a number of functions including those already described for intracellular proteins. For example, the intracellular domain may have enzymatic activity and/or may serve as a binding site for additional proteins. Frequently the intracellular domain of transmembrane proteins serves both roles. For example certain receptor tyrosine kinases have both protein kinase activity and SH2 domains. In addition, autophosphorylation of tyrosines on the receptor molecule itself, creates binding sites for additional SH2 domain containing proteins.

Transmembrane proteins may contain from one to many transmembrane domains. For example, receptor tyrosine kinases, certain cytokine receptors, receptor guanylyl cyclases and receptor serine/threonine protein kinases contain a single transmembrane domain. However, various other proteins including channels and adenylyl cyclases contain numerous transmembrane domains. Many important cell surface receptors such as G protein coupled receptors (GPCRs) are classified as "seven transmembrane domain" proteins, as they contain 7 membrane spanning regions. Characteristics of transmembrane domains include approximately 17 consecutive hydrophobic amino acids that may be followed by charged amino acids. Therefore, upon analysis of the amino acid sequence of a particular protein, the localization and number of transmembrane domains within the protein may be predicted (see, e.g., PSORT web site http://psort.nibb.ac.jp/). Important transmembrane protein receptors include, but are not limited to the insulin receptor, insulin-like growth factor receptor, human growth hormone receptor, glucose transporters, transferrin receptor, epidermal growth factor receptor, low density lipoprotein receptor, epidermal growth factor receptor, leptin receptor, and interleukin receptors, e.g., IL-1 receptor, IL-2 receptor, etc.

The extracellular domains of transmembrane proteins are diverse; however, conserved motifs are found repeatedly among various extracellular domains. Conserved structure and/or functions have been ascribed to different extracellular motifs. Many extracellular domains are involved in binding to other molecules. In one aspect, extracellular domains are found on receptors. Factors that bind the receptor domain include circulating ligands, which may be peptides, proteins, or small molecules such as adenosine and the like. For example, growth factors such as EGF, FGF, and PDGF are circulating growth factors that bind to their cognate receptors to initiate a variety of cellular responses. Other factors include cytokines, mitogenic factors, neurotrophic factors, and the like. Extracellular domains also bind to cell-associated molecules. In this respect, they may mediate cell-cell interactions. Cell-associated ligands can be tethered to the cell, e.g., via a glycosylphosphatidylinositol (GPI) anchor, or may themselves be transmembrane proteins. Extracellular domains may also associate with the extracellular matrix and contribute to the maintenance of the cell structure.

Cancer proteins that are transmembrane are particularly preferred in the present invention as they are readily accessible targets for immunotherapeutics, as are described herein. In addition, as outlined below, transmembrane proteins can be also useful in imaging modalities. Antibodies may be used to label such readily accessible proteins in situ. Alternatively, antibodies can also label intracellular proteins, in which case samples are typically permeablized to provide access to intracellular proteins. In addition, some membrane proteins can be processed to release a soluble protein, or to expose a residual fragment. Released soluble proteins may be useful diagnostic markers, processed residual protein fragments may be useful lung markers of disease.

It will also be appreciated that a transmembrane protein can be made soluble by removing transmembrane sequences, e.g., through recombinant methods. Furthermore, transmembrane proteins that have been made soluble can be made to be secreted through recombinant means by adding an appropriate signal sequence.

In another embodiment, the cancer proteins are secreted proteins; the secretion of which can be either constitutive or regulated. These proteins may have a signal peptide or signal sequence that targets the molecule to the secretory pathway. Secreted proteins are involved in numerous physiological events; e.g., if circulating, they often serve to transmit signals to various other cell types. The secreted protein may function in an autocrine manner (acting on the cell that secreted the factor), a paracrine manner (acting on cells in close proximity to the cell that secreted the factor), an endocrine manner (acting on cells at a distance, e.g, secretion into the blood stream), or exocrine (secretion, e.g., through a duct or to adjacent epithelial surface as sweat glands, sebaceous glands, pancreatic ducts, lacrimal glands, mammary glands, wax producing glands of the ear, etc.). Thus secreted molecules often find use in modulating or altering numerous aspects of physiology. Cancer proteins that are secreted proteins are particularly preferred in the present invention as they serve as good targets for diagnostic markers, e.g., for blood, plasma, serum, or stool tests. Those which are enzymes may be antibody or small molecule targets. Others may be useful as vaccine targets, e.g., via CTL mechanisms.

### Use of cancer nucleic acids

As described above, cancer sequence is initially identified by substantial nucleic acid and/or amino acid sequence homology or linkage to the cancer sequences outlined herein. Such homology can be based upon the overall nucleic acid or amino acid sequence, and is generally determined as outlined below, using either homology programs or hybridization conditions. Typically, linked sequences on a mRNA are found on the same molecule.

As detailed elsewhere, percent identity can be determined using an algorithm such as BLAST. A preferred method utilizes the BLASTN module of WU-BLAST-2 set to the default parameters, with overlap span and overlap fraction set to 1 and 0.125, respectively. Alignment may include the introduction of gaps in the sequences to be aligned. In addition, for sequences which contain either more or fewer nucleotides than those of the nucleic acids described, the percentage of homology may be determined based on the number of homologous nucleosides in relation to the total number of nucleosides. Thus, e.g., homology of sequences shorter than those of the sequences identified will be determined using the number of nucleosides in the shorter sequence.

In one embodiment, the nucleic acid homology is determined through hybridization studies. Thus, e.g., nucleic acids which hybridize under high stringency to a described nucleic acid, or its complement, or is also found on naturally occurring mRNAs is considered a cancer sequence. In another embodiment, less stringent hybridization conditions are used; e.g., moderate or low stringency conditions may be used; see Ausubel, supra, and Tijssen, supra.

The cancer nucleic acid sequences of the invention, e.g., the sequences in Tables 1-68, can be fragments of larger genes, e.g., they are nucleic acid segments. "Genes" in this context includes coding regions, non-coding regions, and mixtures of coding and non-coding regions. Accordingly, using the sequences provided herein, extended sequences, in either direction, of the cancer genes can be obtained, using techniques well known for cloning either longer sequences or the full length sequences; see Ausubel, et al., supra. Much can be done by informatics and many sequences can be clustered to include multiple sequences corresponding to a single gene, e.g., systems such as UniGene (see, http://www.ncbi.nlm.nih.gov/UniGeneo.

Once a cancer nucleic acid is identified, it can be cloned and, if necessary, its constituent parts recombined to form the entire cancer nucleic acid coding regions or the entire mRNA sequence. Once isolated from its natural source, e.g., contained within a plasmid or other vector or excised therefrom as a linear nucleic acid segment, the recombinant cancer nucleic acid can be further used as a probe to identify and isolate other cancer nucleic acids, e.g., extended coding regions. It can also be used as a "precursor" nucleic acid to make modified or variant cancer nucleic acids and proteins.

The cancer nucleic acids of the present invention are used in several ways. In one embodiment, nucleic acid probes to the cancer nucleic acids are made and attached to biochips to be used in screening and diagnostic methods, as outlined below, or for administration, e.g., for gene therapy, vaccine, RNAi, and/or antisense applications. Alternatively, cancer nucleic acids that include coding regions of cancer proteins can be put into expression vectors for the expression of cancer proteins, again for screening purposes or for administration to a patient.

In a preferred embodiment, nucleic acid probes to cancer nucleic acids (both the nucleic acid sequences outlined in the figures and/or the complements thereof) are made. The nucleic acid probes attached to the biochip are designed to be substantially complementary to the cancer nucleic acids, e.g., the target sequence (either the target sequence of the sample or to other probe sequences, e.g., in sandwich assays), such that hybridization of the target sequence and the probes of the present invention occurs. As outlined below, this complementarity need not be perfect; there may be any number of base pair mismatches which will interfere with hybridization between the target sequence and the single stranded nucleic acids of the present invention. However, if the number of mutations is so great that no hybridization can occur under even the least stringent of hybridization conditions, the sequence is not a complementary target sequence. Thus, by "substantially complementary" herein is meant that the probes are sufficiently complementary to the target sequences to hybridize under normal reaction conditions, particularly high stringency conditions, as outlined herein.

A nucleic acid probe is generally single stranded but can be partially single and partially double stranded. The strandedness of the probe is dictated by the structure, composition, and properties of the target sequence. In general, the nucleic acid probes range from about 8-100 bases long, with from about 10-80 bases being preferred, and from about 30-50 bases being particularly preferred. That is, generally whole genes are not used. In some embodiments, much longer nucleic acids can be used, up to hundreds of bases.

In a preferred embodiment, more than one probe per sequence is used, with either overlapping probes or probes to different sections of the target being used. That is, two, three, four or more probes, with three being preferred, are used to build in a redundancy for a particular target. The probes can be overlapping (e.g., have some sequence in common), or separate. In some cases, PCR primers may be used to amplify signal for higher sensitivity.

Nucleic acids can be attached or immobilized to a solid support in a wide variety of ways. By "immobilized" and grammatical equivalents herein is meant the association or binding between the nucleic acid probe and the solid support is sufficient to be stable under the conditions of binding, washing, analysis, and removal as outlined. The binding can typically be covalent or non-covalent. By "non-covalent binding" and grammatical equivalents herein is meant one or more of electrostatic, hydrophilic, and hydrophobic interactions. Included in non-covalent binding is the covalent attachment of a molecule, e.g., streptavidin to the support and the non-covalent binding of the biotinylated probe to the streptavidin. By "covalent binding" and grammatical equivalents herein is meant that the two moieties, the solid support and the probe, are attached by at least one bond, including sigma bonds, pi bonds, and coordination bonds. Covalent bonds can be formed directly between the probe and the solid support or can be formed by a cross linker or by inclusion of a specific reactive group on either the solid support or the probe or both molecules. Immobilization may also involve a combination of covalent and non-covalent interactions.

In general, the probes are attached to the biochip in a wide variety of ways. As described herein, the nucleic acids can either be synthesized first, with subsequent attachment to the biochip, or can be directly synthesized on the biochip.

The biochip comprises a suitable solid substrate. By "substrate" or "solid support" or other grammatical equivalents herein is meant a material that can be modified for the attachment or association of the nucleic acid probes and is amenable to at least one detection method. Often, the substrate may contain discrete individual sites appropriate for individual partitioning and identification. The number of possible substrates is very large, and include, but are not limited to, glass and modified or functionalized glass, plastics (including acrylics, polystyrene and copolymers of styrene and other materials, polypropylene, polyethylene, polybutylene, polyurethanes, TeflonJ, etc.), polysaccharides, nylon or nitrocellulose, resins, silica or silica-based materials including silicon and modified silicon, carbon, metals, inorganic glasses, plastics, etc. In general, the substrates allow optical detection and do not appreciably fluoresce. See WO 0055627.

Generally the substrate is planar, although other configurations of substrates may be used as well. For example, the probes may be placed on the inside surface of a tube for flow-through sample analysis to minimize sample volume. Similarly, the substrate may be flexible, such as a flexible foam, including closed cell foams made of particular plastics.

In a preferred embodiment, the surface of the biochip and the probe may be derivatized with chemical functional groups for subsequent attachment of the two. Thus, e.g., the biochip is derivatized with a chemical functional group including, but not limited to, amino groups, carboxy groups, oxo groups, and thiol groups, with amino groups being particularly preferred. Using these functional groups, the probes can be attached using functional groups on the probes. For example, nucleic acids containing amino groups can be attached to surfaces comprising amino groups, e.g., using linkers; e.g., homo-or hetero-bifunctional linkers as are well known (see 1994 Pierce Chemical Company catalog, technical section on cross-linkers, pages 155-200). In addition, in some cases, additional linkers, such as alkyl groups (including substituted and heteroalkyl groups) may be used.

In this embodiment, oligonucleotides are synthesized, and then attached to the surface of the solid support. Either the 5' or 3' terminus may be attached to the solid support, or attachment may be via linkage to an internal nucleoside. In another embodiment, the immobilization to the solid support may be very strong, yet non-covalent. For example, biotinylated oligonucleotides can be made, which bind to surfaces covalently coated with streptavidin, resulting in attachment.

Alternatively, the oligonucleotides may be synthesized on the surface. For example, photoactivation techniques utilizing photopolymerization compounds and techniques are used. In a preferred embodiment, the nucleic acids can be synthesized in situ, using known photolithographic techniques, such as those described in WO 95/25116; WO 95/35505; U.S. Patent Nos. 5,700,637 and 5,445,934; and references cited within, all of which are expressly incorporated by reference; these methods of attachment form the basis of the Affymetrix GeneChip^{TM} technology.

Often, amplification-based assays are performed to measure the expression level of cancer-associated sequences. These assays are typically performed in conjunction with reverse transcription. In such assays, a cancer-associated nucleic acid sequence acts as a template in an amplification reaction (e.g., Polymerase Chain Reaction, or PCR). In a quantitative amplification, the amount of amplification product will be proportional to the amount of template in the original sample. Comparison to appropriate controls provides a measure of the amount of cancer-associated RNA. Methods of quantitative amplification are well known. Detailed protocols for quantitative PCR are provided, e.g., in Innis, et al. (1990) PCR Protocols. A Guide to Methods and Applications Academic Press.

In some embodiments, a TaqMan based assay is used to measure expression. TaqMan based assays use a fluorogenic oligonucleotide probe that contains a 5' fluorescent dye and a 3' quenching agent. The probe hybridizes to a PCR product, but cannot itself be extended due to a blocking agent at the 3' end. When the PCR product is amplified in subsequent cycles, the 5' nuclease activity of the polymerase, e.g., AmpliTaq, results in the cleavage of the TaqMan probe. This cleavage separates the 5' fluorescent dye and the 3' quenching agent, thereby resulting in an increase in fluorescence as a function of amplification (see, e.g., literature provided by Perkin-Elmer, e.g., www2.perkin-elmer.com).

Other suitable amplification methods include, but are not limited to, ligase chain reaction (LCR) (see Wu and Wallace (1989) Genomics 4:560-569, Landegren, et al. (1988) Science 241:1077-1080, and Barringer, et al. (1990) Gene 89:117-122), transcription amplification (Kwoh, et al. (1989) Proc. Natl. Acad. Sci. USA 86:1173-1177), self-sustained sequence replication (Guatelli, et al. (1990) Proc. Nat. Acad. Sci. USA 87:1874-1878), dot PCR, linker adapter PCR, etc.

### Expression of cancer proteins from nucleic acids

In a preferred embodiment, cancer nucleic acids, e.g., encoding cancer proteins, are used to make a variety of expression vectors to express cancer proteins which can then be used in screening assays, as described below. Expression vectors and recombinant DNA technology are well known (see, e.g., Ausubel, supra, and Fernandez and Hoeffler (eds. 1999) Gene Expression Systems Academic Press) to express proteins. The expression vectors may be either self-replicating extrachromosomal vectors or vectors which integrate into a host genome. Generally, these expression vectors include transcriptional and translational regulatory nucleic acid operably linked to the nucleic acid encoding the cancer protein. The term "control sequences" refers to DNA sequences used for the expression of an operably linked coding sequence in a particular host organism. Control sequences that are suitable for prokaryotes, e.g., include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is typically accomplished by ligation at convenient restriction sites. If such sites do not exist, synthetic oligonucleotide adaptors or linkers are used in accordance with conventional practice. Transcriptional and translational regulatory nucleic acid will generally be appropriate to the host cell used to express the cancer protein. Numerous types of appropriate expression vectors and suitable regulatory sequences are known for a variety of host cells.

In general, transcriptional and translational regulatory sequences may include, but are not limited to, promoter sequences, ribosomal binding sites, transcriptional start and stop sequences, translational start and stop sequences, and enhancer or activator sequences. In a preferred embodiment, the regulatory sequences include a promoter and transcriptional start and stop sequences.

Promoter sequences may be either constitutive or inducible promoters. The promoters may be either naturally occurring promoters or hybrid promoters. Hybrid promoters, which combine elements of more than one promoter, are also known, and are useful in the present invention.

An expression vector may comprise additional elements. For example, the expression vector may have two replication systems, thus allowing it to be maintained in two organisms, e.g., in mammalian or insect cells for expression and in a prokaryotic host for cloning and amplification. Furthermore, for integrating expression vectors, the expression vector often contains at least one sequence homologous to the host cell genome, and preferably two homologous sequences which flank the expression construct. The integrating vector may be directed to a specific locus in the host cell by selecting the appropriate homologous sequence for inclusion in the vector. Constructs for integrating vectors are available. See, e.g., Fernandez and Hoeffler, supra; and Kitamura, et al. (1995) Proc. Nat'1 Acad. Sci. USA 92:9146-9150.

In addition, in a preferred embodiment, the expression vector contains a selectable marker gene to allow the selection of transformed host cells. Selection genes are well known and will vary with the host cell used.

The cancer proteins of the present invention are usually produced by culturing a host cell transformed with an expression vector containing nucleic acid encoding a cancer protein, under the appropriate conditions to induce or cause expression of the cancer protein. Conditions appropriate for cancer protein expression will vary with the choice of the expression vector and the host cell, and will be easily ascertained through routine experimentation or optimization. For example, the use of constitutive promoters in the expression vector will require optimizing the growth and proliferation of the host cell, while the use of an inducible promoter requires the appropriate growth conditions for induction. In addition, in some embodiments, the timing of the harvest is important. For example, the baculoviral systems used in insect cell expression are lytic viruses, and thus harvest time selection can be crucial for product yield.

Appropriate host cells include yeast, bacteria, archaebacteria, fungi, and insect and animal cells, including mammalian cells. Of particular interest are Saccharomyces cerevisiae and other yeasts, E. coli, Bacillus subtilis, Sf9 cells, C129 cells, 293 cells, Neurospora, BHK, CHO, COS, HeLa cells, HUVEC (human umbilical vein endothelial cells), THP1 cells (a macrophage cell line), and various other human cells and cell lines.

In a preferred embodiment, the cancer proteins are expressed in mammalian cells. Mammalian expression systems may be used, and include retroviral and adenoviral systems. One expression vector system is a retroviral vector system such as is generally described in PCT/US97/01019 and PCT/US97/01048. Of particular use as mammalian promoters are the promoters from mammalian viral genes, since the viral genes are often highly expressed and have a broad host range. Examples include the SV40 early promoter, mouse mammary tumor virus LTR promoter, adenovirus major late promoter, herpes simplex virus promoter, and the CMV promoter (see, e.g., Fernandez and Hoeffler, supra). Typically, transcription termination and polyadenylation sequences recognized by mammalian cells are regulatory regions located 3' to the translation stop codon and thus, together with the promoter elements, flank the coding sequence. Examples of transcription terminator and polyadenlyation signals include those derived from SV40.

Methods of introducing exogenous nucleic acid into mammalian hosts, as well as other hosts, are available, and will vary with the host cell used. Techniques include dextran-mediated transfection, calcium phosphate precipitation, polybrene mediated transfection, protoplast fusion, electroporation, viral infection, encapsulation of the polynucleotide(s) in liposomes, and direct microinjection of the DNA into nuclei.

In a preferred embodiment, cancer proteins are expressed in bacterial systems. Promoters from bacteriophage may also be used. In addition, synthetic promoters and hybrid promoters are also useful; e.g., the tac promoter is a hybrid of the trp and lac promoter sequences. Furthermore, a bacterial promoter can include naturally occurring promoters of non-bacterial origin that have the ability to bind bacterial RNA polymerase and initiate transcription. In addition to a functioning promoter sequence, an efficient ribosome binding site is desirable. The expression vector may also include a signal peptide sequence that provides for secretion of the cancer protein in bacteria. The protein is either secreted into the growth media (gram-positive bacteria) or into the periplasmic space, located between the inner and outer membrane of the cell (gram-negative bacteria). The bacterial expression vector may also include a selectable marker gene to allow for the selection of bacterial strains that have been transformed. Suitable selection genes include genes which render the bacteria resistant to drugs such as ampicillin, chloramphenicol, erythromycin, kanamycin, neomycin, and tetracycline. Selectable markers also include biosynthetic genes, such as those in the histidine, tryptophan, and leucine biosynthetic pathways. These components are assembled into expression vectors. Expression vectors for bacteria are well known, and include vectors for Bacillus subtilis, E. coli, Streptococcus cremoris, and Streptococcus lividans, among others (e.g., Fernandez and Hoeffler, supra). The bacterial expression vectors are transformed into bacterial host cells using techniques such as calcium chloride treatment, electroporation, and others.

In one embodiment, cancer proteins are produced in insect cells using, e.g., expression vectors for the transformation of insect cells, and in particular, baculovirus-based expression vectors.

In a preferred embodiment, a cancer protein is produced in yeast cells. Yeast expression systems are well known, and include expression vectors for Saccharomyces cerevisiae, Candida albicans and C. maltosa, Hansenula polymorpha, Kluyveromyces fragilis and K. lactis, Pichia guillerimondii and P. pastoris, Schizosaccharomyces pombe, and Yarrowia lipolytica.

The cancer protein may also be made as a fusion protein, using available techniques. Thus, e.g., for the creation of monoclonal antibodies, if the desired epitope is small, the cancer protein may be fused to a carrier protein to form an immunogen. Alternatively, the cancer protein may be made as a fusion protein to increase expression, or for other reasons. For example, when the cancer protein is a cancer peptide, the nucleic acid encoding the peptide may be linked to other nucleic acid for expression purposes. Fusion with detection epitope tags can be made, e.g., with FLAG, His6, myc, HA, etc.

In a preferred embodiment, the cancer protein is purified or isolated after expression. Cancer proteins may be isolated or purified in a variety of ways depending on what other components are present in the sample and the requirements for purified product, e.g., natural conformation or denatured. Standard purification methods include ammonium sulfate precipitations, electrophoretic, molecular, immunological, and chromatographic techniques, including ion exchange, hydrophobic, affinity, and reverse-phase HPLC chromatography, and chromatofocusing. For example, the cancer protein may be purified using a standard anti-cancer protein antibody column. Ultrafiltration and diafiltration techniques, in conjunction with protein concentration, are also useful. See, e.g., Walsh (2002) Proteins: Biochemistry and Biotechnology Wiley; Hardin, et al. (eds. 2001) Cloning, Gene Expression and Protein Purification Oxford Univ. Press; Wilson, et al. (eds. 2000) Encyclopedia of Separation Science Academic Press; and Scopes (1993) Protein Purification Springer-Verlag. The degree of purification necessary will vary depending on the use of the cancer protein. In some instances no purification will be necessary.

Once expressed and purified if necessary, the cancer proteins and nucleic acids are useful in a number of applications. They may be used as immunoselection reagents, as vaccine reagents, as screening agents, therapeutic entities, for production of antibodies, as transcription or translation inhibitors, etc. Variants of cancer proteins

Also included within one embodiment of cancer proteins are amino acid variants of the naturally occurring sequences, as determined herein. Preferably, the variants are preferably greater than about 75% homologous to the wild-type sequence, more preferably greater than about 80%, even more preferably greater than about 85%, and most preferably greater than 90%. In some embodiments the homology will be as high as about 93-95% or 98%. As for nucleic acids, homology in this context means sequence similarity or identity, with identity being preferred. This homology will be determined using standard techniques, as are outlined above for nucleic acid homologies.

Cancer proteins of the present invention may be shorter or longer than the wild type amino acid sequences. Thus, in a preferred embodiment, included within the definition of cancer proteins are portions or fragments of the wild type sequences herein. In addition, as outlined above, the cancer nucleic acids of the invention may be used to obtain additional coding regions, and thus additional protein sequence.

In one embodiment, the cancer proteins are derivative or variant cancer proteins as compared to the wild-type sequence. That is, as outlined more fully below, the derivative cancer peptide will often contain at least one amino acid substitution, deletion, or insertion, with amino acid substitutions being particularly preferred. The amino acid substitution, insertion, or deletion may occur at many residue positions within the cancer peptide.

Also included within one embodiment of cancer proteins of the present invention are amino acid sequence variants. These variants typically fall into one or more of three classes: substitutional, insertional, or deletional variants. These variants ordinarily are prepared by site specific mutagenesis of nucleotides in the DNA encoding the cancer protein, using cassette or PCR mutagenesis or other techniques, to produce DNA encoding the variant, and thereafter expressing the DNA in recombinant cell culture as outlined above. However, variant cancer protein fragments having up to about 100-150 residues may be prepared by in vitro synthesis using established techniques. Amino acid sequence variants are characterized by the predetermined nature of the variation, a feature that sets them apart from naturally occurring allelic or interspecies variation of the cancer protein amino acid sequence. The variants typically exhibit a similar qualitative biological activity as a naturally occurring analogue, although variants can also be selected which have modified characteristics.

While the site or region for introducing an amino acid sequence variation is often predetermined, the mutation per se need not be predetermined. For example, in order to optimize the performance of a mutation at a given site, random mutagenesis may be conducted at the target codon or region and the expressed cancer variants screened for the optimal combination of desired activity. Techniques for making substitution mutations at predetermined sites in DNA having a known sequence are well known, e.g., M13 primer mutagenesis and PCR mutagenesis. Screening of mutants is often done using assays of cancer protein activities.

Amino acid substitutions are typically of single residues; insertions usually will be on the order of from about 1-20 amino acids, although considerably larger insertions may be tolerated. Deletions generally range from about 1-20 residues, although in some cases deletions may be much larger.

Substitutions, deletions, insertions, or combination thereof may be used to arrive at a final derivative. Generally these changes are done on a few amino acids to minimize the alteration of the molecule. However, larger changes may be tolerated in certain circumstances. When small alterations in the characteristics of the cancer protein are desired, substitutions are generally made in accordance with the amino acid substitution relationships described.

The variants typically exhibit essentially the same qualitative biological activity and will elicit the same immune response as a naturally-occurring analog, although variants also are selected to modify the characteristics of cancer proteins as needed. Alternatively, the variant may be designed such that a biological activity of the cancer protein is altered. For example, glycosylation sites may be added, altered, or removed.

Substantial changes in function or immunological identity are sometimes made by selecting substitutions that are less conservative than those described above. For example, substitutions may be made which more significantly affect: the structure of the polypeptide backbone in the area of the alteration, for example the alpha-helical or beta-sheet structure; the charge or hydrophobicity of the molecule at the target site; or the bulk of the side chain. Substitutions which generally are expected to produce the greatest changes in the polypeptide's properties are those in which (a) a hydrophilic residue, e.g., serine or threone is substituted for (or by) a hydrophobic residue, e.g., leucine, isoleucine, phenylalanine, valine, or alanine; (b) a cysteine or proline is substituted for (or by) another residue; (c) a residue having an electropositive side chain, e.g., lysine, arginine, or histidine, is substituted for (or by) an electronegative residue, e.g., glutamic or aspartic acid; (d) a residue having a bulky side chain, e.g., phenylalanine, is substituted for (or by) one not having a side chain, e.g., glycine; or (e) a proline residue is incorporated or substituted, which changes the degree of rotational freedom of the peptidyl bond.

Variants typically exhibit a similar qualitative biological activity and will elicit the same immune response as the naturally-occurring analog, although variants also are selected to modify the characteristics of the skin cancer proteins as needed. Alternatively, the variant may be designed such that the biological activity of the cancer protein is altered. For example, glycosylation sites may be altered or removed.

Covalent modifications of cancer polypeptides are included within the scope of this invention. One type of covalent modification includes reacting targeted amino acid residues of a cancer polypeptide with an organic derivatizing agent that is capable of reacting with selected side chains or the N-or C-terminal residues of a cancer polypeptide. Derivatization with bifunctional agents is useful, for instance, for crosslinking cancer polypeptides to a water-insoluble support matrix or surface for use in a method for purifying anti-cancer polypeptide antibodies or screening assays, as is more fully described below. Commonly used crosslinking agents include, e.g., 1,1-bis(diazoacetyl)-2-phenylethane, glutaraldehyde, N-hydroxysuccinimide esters, e.g., esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-((p-azidophenyl)dithio)propioimidate.

Other modifications include deamidation of glutaminyl and asparaginyl residues to the corresponding glutamyl and aspartyl residues, respectively, hydroxylation of proline and lysine, phosphorylation of hydroxyl groups of serinyl, threonyl, or tyrosyl residues, methylation of the amino groups of the lysine, arginine, and histidine side chains (e.g., pp. 79-86, Creighton (1992) Proteins: Structure and Molecular Properties Freeman), acetylation of the N-terminal amine, and amidation of a C-terminal carboxyl group.

Another type of covalent modification of the cancer polypeptide included within the scope of this invention comprises altering the native glycosylation pattern of the polypeptide. "Altering the native glycosylation pattern" is intended for purposes herein to mean deleting one or more carbohydrate moieties found in native sequence cancer polypeptide, and/or adding one or more glycosylation sites that are not present in the native sequence cancer polypeptide. Glycosylation patterns can be altered in many ways. Different cell types to express cancer-associated sequences can result in different glycosylation patterns.

Addition of glycosylation sites to cancer polypeptides may also be accomplished by altering the amino acid sequence thereof. The alteration may be made, e.g., by the addition of, or substitution by, one or more serine or threonine residues to the native sequence cancer polypeptide (for O-linked glycosylation sites). The cancer amino acid sequence may optionally be altered through changes at the DNA level, particularly by mutating the DNA encoding the cancer polypeptide at preselected bases such that codons are generated that will translate into the desired amino acids.

Another means of increasing the number of carbohydrate moieties on the cancer polypeptide is by chemical or enzymatic coupling of glycosides to the polypeptide. See, e.g., WO 87/05330; pp. 259-306 in Aplin and Wriston (1981) CRC Crit. Rev. Biochem.

Removal of carbohydrate moieties present on the cancer polypeptide may be accomplished chemically or enzymatically or by mutational substitution of codons encoding for amino acid residues that serve as targets for glycosylation. Chemical deglycosylation techniques are applicable. See, e.g., Sojar and Bahl (1987) Arch. Biochem. Biophys. 259:52-57 and Edge, et al. (1981) Anal. Biochem. 118:131-137. Enzymatic cleavage of carbohydrate moieties on polypeptides can be achieved by the use of a variety of endo-and exo-glycosidases. See, e.g., Thotakura, et al. (1987) Meth. Enzymol. 138:350-359.

Another type of covalent modification of cancer comprises linking the cancer polypeptide to one of a variety of nonproteinaceous polymers, e.g., polyethylene glycol, polypropylene glycol, or polyoxyalkylenes, in the manner set forth in U.S. Patent Nos. 4,640,835; 4,496,689; 4,301,144; 4,670,417; 4,791,192, or 4,179,337.

Cancer polypeptides of the present invention may also be modified in a way to form chimeric molecules comprising a cancer polypeptide fused to another heterologous polypeptide or amino acid sequence. In one embodiment, such a chimeric molecule comprises a fusion of a cancer polypeptide with a tag polypeptide which provides an epitope to which an anti-tag antibody can selectively bind. The epitope tag is generally placed at the amino-or carboxyl-terminus of the cancer polypeptide. The presence of such epitope-tagged forms of a cancer polypeptide can be detected using an antibody against the tag polypeptide. Also, provision of the epitope tag enables the cancer polypeptide to be readily purified by affinity purification using an anti-tag antibody or another type of affinity matrix that binds to the epitope tag. In an alternative embodiment, the chimeric molecule may comprise a fusion of a cancer polypeptide with an immunoglobulin or a particular region of an immunoglobulin. For a bivalent form of the chimeric molecule, such a fusion could-be to the Fc region of an IgG molecule.

Various tag polypeptides and their respective antibodies are available. Examples include poly-histidine (poly-his) or poly-histidine-glycine (poly-his-gly) tags; HIS6 and metal chelation tags, the flu HA tag polypeptide and its antibody 12CA5 (Field, et al. (1988) Mol. Cell. Biol. 8:2159-2165); the c-myc tag and the 8F9, 3C7, 6E10, G4, B7, and 9E10 antibodies thereto (Evan, et al. (1985) Molecular and Cellular Biology 5:3610-3616); and the Herpes Simplex virus glycoprotein D (gD) tag and its antibody (Paborsky, et al. (1990) Protein Engineering 3(6):547-553). Other tag polypeptides include the Flag-peptide (Hopp, et al. (1988) BioTechnology 6:1204-1210); the KT3 epitope peptide (Martin, et al. (1992) Science 255:192-194); tubulin epitope peptide (Skinner, et al. (1991) J. Biol. Chem. 266:15163-15166); and the T7 gene 10 protein peptide tag (Lutz-Freyermuth, et al. (1990) Proc. Natl. Acad. Sci. USA 87:6393-6397).

Also included are other cancer proteins of the cancer family, and cancer proteins from other organisms, which are cloned and expressed as outlined below. Thus, probe or degenerate polymerase chain reaction (PCR) primer sequences may be used to find other related cancer proteins from humans or other organisms. Particularly useful probe and/or PCR primer sequences include the unique areas of the cancer nucleic acid sequence. Preferred PCR primers are from about 15-35 nucleotides in length, with from about 20-30 being preferred, and may contain inosine as needed. The conditions for PCR reaction have been well described (e.g., Innis, PCR Protocols, supra).

In addition, cancer proteins can be made that are longer than those encoded by the nucleic acids of the Tables, e.g., by the elucidation of extended sequences, the addition of epitope or purification tags, the addition of other fusion sequences, etc.

Cancer proteins may also be identified as being encoded by cancer nucleic acids. Thus, cancer proteins are encoded by nucleic acids that will hybridize to the sequences of the sequence listings, or their complements, as outlined herein.

### Antibodies to cancer proteins

In a preferred embodiment, when the cancer protein is to be used to generate antibodies, e.g., for immunotherapy or immunodiagnosis, the cancer protein should share at least one epitope or determinant with the full length protein. By "epitope" or "determinant" herein is typically meant a portion of a protein which will generate and/or bind an antibody or T-cell receptor in the context of MHC. Thus, in most instances, antibodies made to a smaller cancer protein will be able to bind to the full-length protein, particularly linear epitopes. In a preferred embodiment, the epitope is unique; that is, antibodies generated to a unique epitope show little or no cross-reactivity. In a preferred embodiment, the epitope is selected from a protein sequence set out in the tables.

Methods of preparing polyclonal antibodies exist (e.g., Coligan, supra; and Harlow and Lane, supra). Polyclonal antibodies can be raised in a mammal, e.g., by one or more injections of an immunizing agent and, if desired, an adjuvant. Typically, the immunizing agent and/or adjuvant will be injected in the mammal by multiple subcutaneous or intraperitoneal injections. The immunizing agent may include a protein encoded by a nucleic acid of Tables 1-68 or fragment thereof or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). Various immunization protocols may be used.

The antibodies may, alternatively, be monoclonal antibodies. Monoclonal antibodies may.be prepared using hybridoma methods, such as those described by Kohler and Milstein (1975) Nature 256:495. In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized in vitro. The immunizing agent will typically include a polypeptide encoded by a nucleic acid of the tables or fragment thereof, or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (e.g., pp. 59-103 in Goding (1986) Monoclonal Antibodies: Principles and Practice Academic Press). Immortalized cell lines are usually transformed mammalian cells, particularly myeloma cells of rodent, bovine, or human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

In one embodiment, the antibodies are bispecific antibodies. Bispecific antibodies are monoclonal, preferably human or humanized, antibodies that have binding specificities for at least two different antigens or that have binding specificities for two epitopes on the same antigen. In one embodiment, one of the binding specificities is for a protein encoded by a nucleic acid of the tables or a fragment thereof, the other one is for another antigen, and preferably for a cell-surface protein or receptor or receptor subunit, preferably one that is tumor specific. Alternatively, tetramer-type technology may create multivalent reagents.

In a preferred embodiment, the antibodies to cancer protein are capable of reducing or eliminating a biological function of a cancer protein, in a naked form or conjugated to an effector moiety, as is described below. That is, the addition of anti-cancer protein antibodies (either polyclonal or preferably monoclonal) to cancer tissue (or cells containing cancer) may reduce or eliminate the cancer. Generally, at least a 25% decrease in activity, growth, size, or the like is preferred, with at least about 50% being particularly preferred and about a 95-100% decrease being especially preferred.

In a preferred embodiment the antibodies to the cancer proteins are humanized antibodies (e.g., Xenerex Biosciences, Medarex, Inc., Abgenix, Inc., Protein Design Labs, Inc.) Humanized forms of non-human (e.g., murine) antibodies are chimeric molecules of immunoglobulins, immunoglobulin chains or fragments thereof (such as Fv, Fab, Fab', F(ab')2 or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat, or rabbit having the desired specificity, affinity, and capacity. In some instances, Fv framework residues of a human immunoglobulin are replaced by corresponding non-human residues. Humanized antibodies may also comprise residues which are found neither in the recipient antibody nor in the imported CDR or framework sequences. In general, a humanized antibody will comprise substantially all of at least one, and typically two, variable domains, in which all or substantially all of the CDR regions correspond to those of a non-human immunoglobulin and all or substantially all of the framework (FR) regions are those of a human immunoglobulin consensus sequence. The humanized antibody optimally also will typically comprise at least a portion of an immunoglobulin constant region (Fc), typically that of a human immunoglobulin (Jones, et al. (1986) Nature 321:522-525; Riechmann, et al. (1988) Nature 332:323-329; and Presta (1992) Curr. Op. Struct. Biol. 2:593-596). Humanization can be essentially performed following the method of Winter and co-workers (Jones, et al. (1986) Nature 321:522-525; Riechmann, et al. (1988) Nature 332:323-327; Verhoeyen, et al. (1988) Science 239:1534-1536), by substituting rodent CDRs or CDR sequences for corresponding sequences of a human antibody. Accordingly, such humanized antibodies are chimeric antibodies (U.S. Patent No. 4,816,567), wherein substantially less than an intact human variable domain has been substituted by corresponding sequence from a non-human species.

Human antibodies can also be produced using phage display libraries (Hoogenboom and Winter (1992) J. Mol. Biol. 227:381-388; Marks, et al. (1991) J. Mol. Biol. 222:581-597) or human monoclonal antibodies (e.g., p. 77, Cole, et al. in Reisfeld and Sell (1985) Monoclonal Antibodies and Cancer Therapy Liss; and Boerner, et al. (1991) J. Immunol. 147:86-95). Similarly, human antibodies can be made by introducing human immunoglobulin loci into transgenic animals, e.g., mice in which the endogenous immunoglobulin genes have been partially or completely inactivated. Upon challenge, human antibody production is observed, which closely resembles that seen in humans in nearly all respects, including gene rearrangement, assembly, and antibody repertoire. This approach is described, e.g., in U.S. Patent Nos. 5,545,807; 5,545,806; 5,569,825; 5,625,126; 5,633,425; 5,661,016, and in the following scientific publications: Marks, et al. (1992) Bio/Technology 10:779-783; Lonberg, et al. (1994) Nature 368:856-859; Morrison (1994) Nature 368:812-13; Fishwild, et al. (1996) Nature Biotechnology 14:845-851; Neuberger (1996) Nature Biotechnology 14:826; and Lonberg and Huszar (1995) Intern. Rev. Immunol. 13:65-93.

By immunotherapy is meant treatment of cancer with an antibody raised against cancer proteins. As used herein, immunotherapy can be passive or active. Passive immunotherapy as defined herein is the passive transfer of antibody to a recipient (patient). Active immunization is the induction of antibody and/or T-cell responses in a recipient (patient). Induction of an immune response is the result of providing the recipient with an antigen to which antibodies are raised. The antigen may be provided by injecting a polypeptide against which antibodies are desired to be raised into a recipient, or contacting the recipient with a nucleic acid capable of expressing the antigen and under conditions for expression of the antigen, leading to an immune response.

In a preferred embodiment the cancer proteins against which antibodies are raised are secreted proteins as described above. Without being bound by theory, antibodies used for treatment may bind and prevent the secreted protein from binding to its receptor, thereby inactivating the secreted cancer protein, e.g., in autocrine signaling.

In another preferred embodiment, the cancer protein to which antibodies are raised is a transmembrane protein. Without being bound by theory, antibodies used for treatment may bind the extracellular domain of the cancer protein and prevent it from binding to other proteins, such as circulating ligands or cell-associated molecules. The antibody may cause down-regulation of the transmembrane cancer protein. The antibody may be a competitive, non-competitive or uncompetitive inhibitor of protein binding to the extracellular domain of the cancer protein. The antibody may also be an antagonist of the cancer protein. Further, the antibody may prevent activation of the transmembrane cancer protein, or may induce or suppress a particular cellular pathway. In one aspect, when the antibody prevents the binding of other molecules to the cancer protein, the antibody prevents growth of the cell. The antibody may also be used to target or sensitize the cell to cytotoxic agents, including, but not limited to TNF-α, TNF-β, EL-1, INF-γ, and EL-2, or chemotherapeutic agents including 5FU, vinblastine, actinomycin D, cisplatin, methotrexate, and the like. In some instances the antibody may belong to a sub-type that activates serum complement when complexed with the transmembrane protein thereby mediating cytotoxicity or antigen-dependent cytotoxicity (ADCC). Thus, cancer may be treated by administering to a patient antibodies directed against the transmembrane cancer protein. Antibody-labeling may activate a co-toxin, localize a toxin payload, or otherwise provide means to locally ablate cells.

In another preferred embodiment, the antibody is conjugated to an effector moiety. The effector moiety can be various molecules, including labeling moieties such as radioactive labels or fluorescent labels, or can be a therapeutic moiety. In one aspect the therapeutic moiety is a small molecule that modulates the activity of a cancer protein. In another aspect the therapeutic moiety may modulate the activity of molecules associated with or in close proximity to a cancer protein. The therapeutic moiety may inhibit enzymatic or signaling activity such as protease or collagenase or protein kinase activity associated with cancer, or be an attractant of other cells, such as NK cells.

In a preferred embodiment, the therapeutic moiety can also be a cytotoxic agent. In this method, targeting the cytotoxic agent to cancer tissue or cells results in a reduction in the number of afflicted cells, thereby reducing symptoms associated with cancer. Cytotoxic agents are numerous and varied and include, but are not limited to, cytotoxic drugs or toxins or active fragments of such toxins. Suitable toxins and their corresponding fragments include diphtheria A chain, exotoxin A chain, ricin A chain, abrin A chain, curcin, crotin, phenomycin, enomycin, saporin, auristatin, and the like. Cytotoxic agents also include radiochemicals made by conjugating radioisotopes to antibodies raised against cancer proteins, or binding of a radionuclide to a chelating agent that has been covalently attached to the antibody. Targeting the therapeutic moiety to transmembrane cancer proteins not only serves to increase the local concentration of therapeutic moiety in the cancer afflicted area, but also serves to reduce deleterious side effects that may be associated with the untargeted therapeutic moiety.

In another preferred embodiment, the cancer protein against which the antibodies are raised is an intracellular protein. In this case, the antibody may be conjugated to a protein which facilitates entry into the cell. In one case, the antibody enters the cell by endocytosis. In another embodiment, a nucleic acid encoding the antibody is administered to the individual or cell. Moreover, wherein the cancer protein can be targeted within a cell, e.g., the nucleus, an antibody thereto may contain a signal for that target localization, e.g., a nuclear localization signal.

The cancer antibodies of the invention specifically bind to cancer proteins. By "specifically bind" herein is meant that the antibodies bind to the protein with a K_{d} of at least about 0.1 mM, more usually at least about 1 *µ*M, preferably at least about 0.1 *µ*M or better, and most preferably, 0.01 *µ*M or better. Selectivity of binding to the specific target and not to related sequences is often also important.

### Detection of cancer sequence for diagnostic and therapeutic applications

In one aspect, the RNA expression levels of genes are determined for different cellular states in the cancer phenotype. Expression levels of genes in normal tissue (e.g., not undergoing cancer) and in cancer tissue (and in some cases, for varying severities of cancer that relate to prognosis, as outlined below), or in non-malignant disease are evaluated to provide expression profiles. A gene expression profile of a particular cell state or point of development is essentially a "fingerprint" of the state of the cell. While two states may have a particular gene similarly expressed, the evaluation of a number of genes simultaneously allows the generation of a gene expression profile that is reflective of the state of the cell. By comparing expression profiles of cells in different states, information regarding which genes are important (including both up- and down-regulation of genes) in each of these states is obtained. Then, diagnosis may be performed or confirmed to determine whether a tissue sample has the gene expression profile of normal or cancerous tissue. This will provide for molecular diagnosis of related conditions.

"Differential expression," or grammatical equivalents as used herein, refers to qualitative or quantitative differences in the temporal and/or cellular gene expression patterns within and among cells and tissue. Thus, a differentially expressed gene can qualitatively have its expression altered, including an activation or inactivation, in, e.g., normal versus cancer tissue. Genes may be turned on or turned off in a particular state, relative to another state thus permitting comparison of two or more states. A qualitatively regulated gene will exhibit an expression pattern within a state or cell type which is detectable by standard techniques. Some genes will be expressed in one state or cell type, but not in both. Alternatively, the difference in expression may be quantitative, e.g., in that expression is increased or decreased; e.g., gene expression is either upregulated, resulting in an increased amount of transcript, or downregulated, resulting in a decreased amount of transcript. The degree to which expression differs need only be large enough to quantify via standard characterization techniques as outlined below, such as by use of Affymetrix GeneChip™ expression arrays. See, Lockhart (1996) Nature Biotechnology 14:1675-1680. Other techniques include, but are not limited to, quantitative reverse transcriptase PCR, northern analysis, and RNase protection. As outlined above, preferably the change in expression (e.g., upregulation or downregulation) is at least about 50%, more preferably at least about 100%, more preferably at least about 150%, more preferably at least about 200%, with from 300 to at least 1000% being especially preferred.

Evaluation may be at the gene transcript or the protein level. The amount of gene expression may be monitored using nucleic acid probes to the RNA or DNA equivalent of the gene transcript, and the quantification of gene expression levels, or, alternatively, the final gene product itself (protein) can be monitored, e.g., with antibodies to the cancer protein and standard immunoassays (ELISAs, etc.) or other techniques, including mass spectroscopy assays, 2D gel electrophoresis assays, etc. Proteins corresponding to cancer genes, e.g., those identified as being important in a cancer or disease phenotype, can be evaluated in a cancer diagnostic test. In a preferred embodiment, gene expression monitoring is performed simultaneously on a number of genes. Multiple protein expression monitoring can be performed as well.

In this embodiment, the cancer nucleic acid probes are attached to biochips as outlined herein for the detection and quantification of cancer sequences in a particular cell. The assays are further described below in the example. PCR techniques can be used to provide greater sensitivity.

In a preferred embodiment nucleic acids encoding the cancer protein are detected. Although DNA or RNA encoding the cancer protein may be detected, of particular interest are methods wherein an mRNA encoding a cancer protein is detected. Probes to detect mRNA can be a nucleotide/deoxynucleotide probe that is complementary to and hybridizes with the mRNA and includes, but is not limited to, oligonucleotides, cDNA, or RNA. Probes also should contain a detectable label, as defined herein. In one method the mRNA is detected after immobilizing the nucleic acid to be examined on a solid support such as nylon membranes and hybridizing the probe with the sample. Following washing to remove the non-specifically bound probe, the label is detected. In another method, detection of the mRNA is performed in situ. In this method permeabilized cells or tissue samples are contacted with a detectably labeled nucleic acid probe for sufficient time to allow the probe to hybridize with the target mRNA. Following washing to remove the non-specifically bound probe, the label is detected. For example a digoxygenin labeled riboprobe (RNA probe) that is complementary to the mRNA encoding a cancer protein is detected by binding the digoxygenin with an anti-digoxygenin secondary antibody and developed with nitro blue tetrazolium and 5-bromo-4-chloro-3-indoyl phosphate.

In a preferred embodiment, various proteins from the three classes of proteins as described herein (secreted, transmembrane, or intracellular proteins) are used in diagnostic assays. The cancer proteins, antibodies, nucleic acids, modified proteins, and cells containing cancer sequences are used in diagnostic assays. This can be performed on an individual gene or corresponding polypeptide level. In a preferred embodiment, the expression profiles are used, preferably in conjunction with high throughput screening techniques to allow monitoring for expression profile genes and/or corresponding polypeptides.

As described and defined herein, cancer proteins, including intracellular, transmembrane, or secreted proteins, find use as markers of cancer, e.g., for prognostic or diagnostic purposes. Detection of these proteins in putative cancer tissue allows for detection, prognosis, or diagnosis of cancer or similar disease, and for selection of therapeutic strategy. In one embodiment, antibodies are used to detect cancer proteins. A preferred method separates proteins from a sample by electrophoresis on a gel (typically a denaturing and reducing protein gel, but may be another type of gel, including isoelectric focusing gels and the like). Following separation of proteins, the cancer protein is detected, e.g., by immunoblotting with antibodies raised against the cancer protein.

In another preferred method, antibodies to the cancer protein find use in in situ imaging techniques, e.g., in histology. See, e.g., Asai, et al. (eds. 1993) Methods in Cell Biology: Antibodies in Cell Biology (vol. 37) Academic Press. In this method, cells are contacted with from one to many antibodies to the cancer protein(s). Following washing to remove non-specific antibody binding, the presence of the antibody or antibodies is detected. In one embodiment the antibody is detected by incubating with a secondary antibody that contains a detectable label. In another method the primary antibody to the cancer protein(s) contains a detectable label, e.g., an enzyme marker that can act on a substrate. In another preferred embodiment each one of multiple primary antibodies contains a distinct and detectable label. This method finds particular use in simultaneous screening for a plurality of cancer proteins. Many other histological imaging techniques are also provided by the invention.

In a preferred embodiment the label is detected in a fluorometer which has the ability to detect and distinguish emissions of different wavelengths. In addition, a fluorescence activated cell sorter (FACS) can be used in the method.

In another preferred embodiment, antibodies find use in diagnosing cancer from blood, serum, plasma, stool, and other samples. Such samples, therefore, are useful as samples to be probed or tested for the presence of cancer proteins. Antibodies can be used to detect a cancer protein by previously described immunoassay techniques including ELISA, immunoblotting (western blotting), immunoprecipitation, BIACORE technology and the like. Conversely, the presence of antibodies may indicate an immune response against an endogenous cancer protein.

In a preferred embodiment, in situ hybridization of labeled cancer nucleic acid probes to tissue arrays is done. For example, arrays of tissue samples, including cancer tissue and/or normal tissue, are made. In situ hybridization (see, e.g., Ausubel, supra) is then performed. When comparing the fingerprints between an individual and a standard, a diagnosis, a prognosis, or a prediction may be based on the findings. It is further understood that the genes which indicate the diagnosis may differ from those which indicate the prognosis and molecular profiling of the condition of the cells may lead to distinctions between responsive or refractory conditions or may be predictive of outcomes.

In a preferred embodiment, the cancer proteins, antibodies, nucleic acids, modified proteins, and cells containing cancer sequences are used in prognosis assays. As above, gene expression profiles can be generated that correlate to cancer, clinical, pathological, or other information, in terms of long term prognosis. Again, this may be done on either a protein or gene level, with the use of genes being preferred. Single or multiple genes may be useful in various combinations. As above, cancer probes may be attached to biochips for the detection and quantification of cancer sequences in a tissue or patient. The assays proceed as outlined above for diagnosis. PCR method may provide more sensitive and accurate quantification. Assays for therapeutic compounds

In a preferred embodiment, the proteins, nucleic acids, and antibodies as described herein are used in drug screening assays. The cancer proteins, antibodies, nucleic acids, modified proteins, and cells containing cancer sequences are used in drug screening assays or by evaluating the effect of drug candidates on a "gene expression profile" or expression profile of polypeptides. In a preferred embodiment, the expression profiles are used, preferably in conjunction with high throughput screening techniques, to allow monitoring for expression profile genes after treatment with a candidate agent (e.g., Zlokarnik, et al. (1998) Science 279:84-88; Heid (1996) Genome Res. 6:986-994.

In a preferred embodiment, the cancer proteins, antibodies, nucleic acids, modified proteins and cells containing the native or modified cancer proteins are used in screening assays. That is, the present invention provides novel methods for screening for compositions which modulate the cancer phenotype or an identified physiological function of a cancer protein. As above, this can be done on an individual gene level or by evaluating the effect of drug candidates on a "gene expression profile". In a preferred embodiment, the expression profiles are used, preferably in conjunction with high throughput screening techniques, to allow monitoring for expression profile genes after treatment with a candidate agent, see Zlokarnik, supra.

Having identified the differentially expressed genes herein, a variety of assays may be performed. In a preferred embodiment, assays may be run on an individual gene or protein level. That is, having identified a particular gene as up regulated in cancer, test compounds can be screened for the ability to modulate gene expression or for binding to the cancer protein. "Modulation" thus includes both an increase and a decrease in gene expression. The preferred amount of modulation will depend on the original change of the gene expression in normal versus tissue undergoing cancer, with changes of at least 10%, preferably 50%, more preferably 100-300%, and in some embodiments 300-1000% or greater. Thus, if a gene exhibits a 4-fold increase in cancer tissue compared to normal tissue, a decrease of about four-fold is often desired; similarly, a 10-fold decrease in cancer tissue compared to normal tissue often provides a target value of a 10-fold increase in expression to be induced by the test compound.

The amount of gene expression may be monitored using nucleic acid probes and the quantification of gene expression levels, or, alternatively, the gene product itself can be monitored, e.g., through the use of antibodies to the cancer protein and standard immunoassays. Proteomics and separation techniques may also allow quantification of expression.

In a preferred embodiment, gene expression or protein monitoring of a number of entities, e.g., an expression profile, is monitored simultaneously. Such profiles will typically involve a plurality of those entities described herein.

In this embodiment, the cancer nucleic acid probes are attached to biochips as outlined herein for the detection and quantification of cancer sequences in a particular cell. Alternatively, PCR may be used. Thus, a series, e.g., of microtiter plate, may be used with dispensed primers in desired wells. A PCR reaction can then be performed and analyzed for each well.

### Modulators of cancer

Expression monitoring can be performed to identify compounds that modify the expression of one or more cancer-associated sequences, e.g., a polynucleotide sequence set out in the tables. Generally, in a preferred embodiment, a test modulator is added to the cells prior to analysis. Moreover, screens are also provided to identify agents that modulate cancer, modulate cancer proteins, bind to a cancer protein, or interfere with the binding of a cancer protein and an antibody or other binding partner.

The term "test compound" or "drug candidate" or "modulator" or grammatical equivalents as used herein describes a molecule, e.g., protein, oligopeptide, small organic molecule, polysaccharide, polynucleotide, etc., to be tested for the capacity to directly or indirectly alter the cancer phenotype or the expression of a cancer sequence, e.g., a nucleic acid or protein sequence. In preferred embodiments, modulators alter expression profiles, or expression profile nucleic acids or proteins provided herein. In one embodiment, the modulator suppresses a cancer phenotype, e.g., to a normal or non-malignant tissue fingerprint. In another embodiment, a modulator induced a cancer phenotype. Generally, a plurality of assay mixtures are run in parallel with different agent concentrations to obtain a differential response to the various concentrations. Typically, one of these concentrations serves as a negative control, e.g., at zero concentration or below the level of detection.

Drug candidates encompass numerous chemical classes, though typically they are organic molecules, preferably small organic compounds having a molecular weight of more than 100 and less than about 2,500 daltons. Preferred small molecules are less than 2000, or less than 1500, or less than 1000, or less than 500 D. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs, or combinations thereof. Particularly preferred are peptides.

In one aspect, a modulator will neutralize the effect of a cancer protein. By "neutralize" is meant that activity of a protein is inhibited or blocked and the consequent effect on the cell.

In certain embodiments, combinatorial libraries of potential modulators will be screened for an ability to bind to a cancer polypeptide or to modulate activity. Conventionally, new chemical entities with useful properties are generated by identifying a chemical compound (called a "lead compound") with some desirable property or activity, e.g., inhibiting activity, creating variants of the lead compound, and evaluating the property and activity of those variant compounds. Often, high throughput screening (HTS) methods are employed for such an analysis. See, e.g., Janzen (2002) High Throughput Screening: Methods and Protocols Humana; Devlin (ed. 1997) High Throughput Screening: The Discovery of Bioactive Substances Dekker; and Mei and Czarnik (eds. 2002) Integrated Drug Discovery Techniques Dekker.

In one preferred embodiment, high throughput screening methods involve providing a library containing a large number of potential therapeutic compounds (candidate compounds). Such "combinatorial chemical libraries" are then screened in one or more assays to identify those library members (particular chemical species or subclasses) that display a desired characteristic activity. The compounds thus identified can serve as conventional "lead compounds" or can themselves be used as potential or actual therapeutics.

A combinatorial chemical library is a collection of diverse chemical compounds generated by either chemical synthesis or biological synthesis by combining a number of chemical "building blocks" such as reagents. For example, a linear combinatorial chemical library, such as a polypeptide (e.g., mutein) library, is formed by combining a set of chemical building blocks called amino acids in every possible way for a given compound length (e.g., the number of amino acids in a polypeptide compound). Millions of chemical compounds can be synthesized through such combinatorial mixing of chemical building blocks (Gallop, et al. (1994) J. Med. Chem. 37:1233-1251).

Preparation and screening of combinatorial chemical libraries is well known. Such combinatorial chemical libraries include, but are not limited to, peptide libraries (see, e.g., U.S. Patent No. 5,010,175, Furka (1991) Pept. Prot. Res. 37:487-493, Houghton, et al. (1991) Nature 354:84-88), peptoids (PCT Publication No WO 91/19735), encoded peptides (PCT Publication WO 93/20242), random bio-oligomers (PCT Publication WO 92/00091), benzodiazepines (U.S. Pat. No. 5,288,514), diversomers such as hydantoins, benzodiazepines and dipeptides (Hobbs, et al. (1993) Proc. Nat. Acad. Sci. USA 90:6909-6913, vinylogous polypeptides (Hagihara, et al. (1992) J. Amer. Chem. Soc. 114:6568-570), nonpeptidal peptidomimetics with a Beta-D-Glucose scaffolding (Hirschmann, et al. (1992) J. Amer. Chem. Soc. 114:9217-9218), analogous organic syntheses of small compound libraries (Chen, et al. (1994) J. Amer. Chem. Soc. 116:2661-662), oligocarbamates (Cho, et al. (1993) Science 261:1303-1305), and/or peptidyl phosphonates (Campbell, et al. (1994) J. Org. Chem. 59:658-xxx). See, generally, Gordon, et al. (1994) J. Med. Chem. 37:1385-1401, nucleic acid libraries (see, e.g., Stratagene, Corp.), peptide nucleic acid libraries (see, e.g., U.S. Patent 5,539,083), antibody libraries (see, e.g., Vaughn, et al. (1996) Nature Biotechnology 14(3):309-314, and PCT/US96/10287), carbohydrate libraries (see, e.g., Liang, et al. (1996) Science 274:1520-1522, and U.S. Patent No. 5,593,853), and small organic molecule libraries (see, e.g., benzodiazepines, page 33 Baum (Jan 18, 1993) C&EN; isoprenoids, U.S. Patent No. 5,569,588; thiazolidinones and metathiazanones, U.S. Patent No. 5,549,974; pyrrolidines, U.S. Patent Nos. 5,525,735 and 5,519,134; morpholino compounds, U.S. Patent No. 5,506,337; benzodiazepines, U.S. Patent No. 5,288,514; and the like).

Devices for the preparation of combinatorial libraries are commercially available (see, e.g., 357 MPS, 390 MPS, Advanced Chem Tech, Louisville KY, Symphony, Rainin, Woburn, MA, 433A Applied Biosystems, Foster City, CA, 9050 Plus, Millipore, Bedford, MA).

A number of well known robotic systems have also been developed for solution phase chemistries. These systems include automated workstations like the automated synthesis apparatus developed by Takeda Chemical Industries, LTD. (Osaka, Japan) and many robotic systems utilizing robotic arms (Zymate II, Zymark Corporation, Hopkinton, Mass.; Orca, Hewlett-Packard, Palo Alto, Calif.), which mimic the manual synthetic operations performed by a chemist. The above devices are suitable for use with the present invention. The nature and implementation of modifications to these devices (if any) so that they can operate as discussed herein will be apparent. In addition, numerous combinatorial libraries are themselves commercially available (see, e.g., ComGenex, Princeton, N.J., Asinex, Moscow, Ru, Tripos, Inc., St. Louis, MO, ChemStar, Ltd, Moscow, RU, 3D Pharmaceuticals, Exton, PA, Martek Biosciences, Columbia, MD, etc.).

The assays to identify modulators are amenable to high throughput screening. Preferred assays thus detect enhancement or inhibition of cancer gene transcription, inhibition, or enhancement of polypeptide expression, and inhibition or enhancement of polypeptide activity.

High throughput assays for the presence, absence, quantification, or other properties of particular nucleic acids or protein products are well known. Similarly, binding assays and reporter gene assays are similarly well known. Thus, e.g., U.S. Patent No. 5,559,410 discloses high throughput screening methods for proteins, U.S. Patent No. 5,585,639 discloses high throughput screening methods for nucleic acid binding (e.g., in arrays), while U.S. Patent Nos. 5,576,220 and 5,541,061 disclose high throughput methods of screening for ligand/antibody binding.

In addition, high throughput screening systems are commercially available (see, e.g., Zymark Corp., Hopkinton, MA; Air Technical Industries, Mentor, OH; Beckman Instruments, Inc. Fullerton, CA; Precision Systems, Inc., Natick, MA, etc.). These systems typically automate entire procedures, including sample and reagent pipetting, liquid dispensing, timed incubations, and final readings of the microplate in detector(s) appropriate for the assay. These configurable systems provide high throughput and rapid start up as well as a high degree of flexibility and customization. The manufacturers of such systems provide detailed protocols for various high throughput systems. Thus, e.g., Zymark Corp. provides technical bulletins describing screening systems for detecting the modulation of gene transcription, ligand binding, and the like.

In one embodiment, modulators are proteins, often naturally occurring proteins or fragments of naturally occurring proteins. Thus, e.g., cellular extracts containing proteins, or random or directed digests of proteinaceous cellular extracts, may be used. In this way libraries of proteins may be made for screening in the methods of the invention. Particularly preferred in this embodiment are libraries of bacterial, fungal, viral, and mammalian proteins, with the latter being preferred, and human proteins being especially preferred. Particularly useful test compound will be directed to the class of proteins to which the target belongs, e.g., substrates for enzymes or ligands and receptors.

In a preferred embodiment, modulators are peptides of from about 5-30 amino acids, with from about 5-20 amino acids being preferred, and from about 7-15 being particularly preferred. The peptides may be digests of naturally occurring proteins, random peptides, or "biased" random peptides. By "randomized" or grammatical equivalents herein is meant that each nucleic acid and peptide consists of essentially random nucleotides and amino acids, respectively. Since generally these random peptides (or nucleic acids, discussed below) are chemically synthesized, they may incorporate a nucleotide or amino acid at any position. The synthetic process can be designed to generate randomized proteins or nucleic acids, to allow the formation of all or most of the possible combinations over the length of the sequence, thus forming a library of randomized candidate bioactive proteinaceous agents.

In one embodiment, the library is fully randomized, with no sequence preferences or constants at any position. In a preferred embodiment, the library is biased. That is, some positions within the sequence are either held constant, or are selected from a limited number of possibilities. For example, in a preferred embodiment, the nucleotides or amino acid residues are randomized within a defined class, e.g., of hydrophobic amino acids, hydrophilic residues, sterically biased (either small or large) residues, towards the creation of nucleic acid binding domains, the creation of cysteines, for cross-linking, prolines for SH-3 domains, serines, threonines, tyrosines, or histidines for phosphorylation sites, etc., or to purines, etc.

Modulators of cancer can also be nucleic acids, as defined above.

As described above generally for proteins, nucleic acid modulating agents may be naturally occurring nucleic acids, random nucleic acids, or "biased" random nucleic acids. For example, digests of prokaryotic or eukaryotic genomes may be used as is outlined above for proteins.

In a preferred embodiment, the candidate compounds are organic chemical moieties, a wide variety of which are available in the literature.

After the candidate agent has been added and the cells allowed to incubate for some period of time, the sample containing a target sequence to be analyzed is added to the biochip. If required, the target sequence is prepared using known techniques. For example, the sample may be treated to lyse the cells, using known lysis buffers, electroporation, etc., with purification and/or amplification such as PCR performed as appropriate. For example, an in vitro transcription with labels covalently attached to the nucleotides is performed. Generally, the nucleic acids are labeled with biotin-FITC or PE, or with cy3 or cy5.

In a preferred embodiment, the target sequence is labeled with, e.g., a fluorescent, a chemiluminescent, a chemical, or a radioactive signal, to provide a means of detecting the target sequence's specific binding to a probe. The label also can be an enzyme, such as, alkaline phosphatase or horseradish peroxidase, which when provided with an appropriate substrate produces a product that can be detected. Alternatively, the label can be a labeled compound or small molecule, such as an enzyme inhibitor, that binds but is not catalyzed or altered by the enzyme. The label also can be a moiety or compound, such as, an epitope tag or biotin which specifically binds to streptavidin. For the example of biotin, the streptavidin is labeled as described above, thereby, providing a detectable signal for the bound target sequence. Unbound labeled streptavidin is typically removed prior to analysis.

These assays can be direct hybridization assays or can comprise "sandwich assays", which include the use of multiple probes, as is generally outlined in U.S. Patent Nos. 5,681,702, 5,597,909, 5,545,730, 5,594,117, 5,591,584, 5,571,670, 5,580,731, 5,571,670, 5,591,584, 5,624,802, 5,635,352, 5,594,118, 5,359,100, 5,124,246, and 5,681,697, all of which are hereby incorporated by reference. In this embodiment, in general, the target nucleic acid is prepared as outlined above, and then added to the biochip comprising a plurality of nucleic acid probes, under conditions that allow the formation of a hybridization complex.

A variety of hybridization conditions may be used in the present invention, including high, moderate, and low stringency conditions as outlined above. The assays are generally run under stringency conditions which allows formation of the label probe hybridization complex only in the presence of target. Stringency can be controlled by altering a step parameter that is a thermodynamic variable, including, but not limited to, temperature, formamide concentration, salt concentration, chaotropic salt concentration, pH, organic solvent concentration, etc.

These parameters may also be used to control non-specific binding, as is generally outlined in U.S. Patent No. 5,681,697. Thus it may be desirable to perform certain steps at higher stringency conditions to reduce non-specific binding.

The reactions outlined herein may be accomplished in a variety of ways. Components of the reaction may be added simultaneously, or sequentially, in different orders, with preferred embodiments outlined below. In addition, the reaction may include a variety of other reagents. These include salts, buffers, neutral proteins, e.g., albumin, detergents, etc. which may be used to facilitate optimal hybridization and detection, and/or reduce non-specific or background interactions. Reagents that otherwise improve the efficiency of the assay, such as protease inhibitors, nuclease inhibitors, anti-microbial agents, etc., may also be used as appropriate, depending on the sample preparation methods and purity of the target.

The assay data are analyzed to determine the expression levels, and changes in expression levels as between states of individual genes, forming a gene expression profile.

Screens are performed to identify modulators of the cancer phenotype. In one embodiment, screening is performed to identify modulators that can induce or suppress a particular expression profile, thus preferably generating the associated phenotype. In another embodiment, e.g., for diagnostic applications, having identified differentially expressed genes important in a particular state, screens can be performed to identify modulators that alter expression of individual genes. In an another embodiment, screening is performed to identify modulators that alter a biological function of the expression product of a differentially expressed gene. Again, having identified the importance of a gene in a particular state, screens are performed to identify agents that bind and/or modulate the biological activity of the gene product.

In addition, screens can be done for genes that are induced in response to a candidate agent or treatment process. After identifying a modulator based upon its ability to suppress a cancer expression pattern leading to a normal expression pattern (or its converse), or to modulate a single cancer gene expression profile so as to mimic the expression of the gene from normal tissue, a screen as described above can be performed to identify genes that are specifically modulated in response to the agent. Comparing expression profiles between normal tissue and agent treated cancer tissue reveals genes that are not expressed in normal tissue or cancer tissue, but are expressed in agent treated tissue. These agent-specific sequences can be identified and used by methods described herein for cancer genes or proteins. In particular, these sequences and the proteins they encode find use in marking or identifying agent treated cells. In addition, antibodies can be raised against the agent induced proteins and used to target novel therapeutics to the treated cancer tissue sample.

Thus, in one embodiment, a test compound is administered to a population of cancer cells that have an associated cancer expression profile. By "administration" or "contacting" herein is meant that the candidate agent is added to the cells in such a manner as to allow the agent to act upon the cell, whether by uptake and intracellular action, or by action at the cell surface. In some embodiments, nucleic acid encoding a proteinaceous candidate agent (e.g., a peptide) may be put into a viral construct such as an adenoviral or retroviral construct, and added to the cell, such that expression of the peptide agent is accomplished, e.g., PCT US97/01019. Regulatable gene therapy systems can also be used.

Once a test compound has been administered to the cells, the cells can be washed if desired and are allowed to incubate under preferably physiological conditions for some period of time. The cells are then harvested and a new gene expression profile is generated, as outlined herein.

Thus, e.g., cancer or non-malignant tissue may be screened for agents that modulate, e.g., induce or suppress a cancer phenotype. A change in at least one gene, preferably many, of the expression profile indicates that the agent has an effect on cancer activity. By defining such a signature for the cancer phenotype, screens for new drugs that alter the phenotype can be devised. With this approach, the drug target need not be known and need not be represented in the original expression screening platform, nor does the level of transcript for the target protein need to change.

In a preferred embodiment, as outlined above, screens may be done on individual genes and gene products (proteins). That is, having identified a particular differentially expressed gene as important in a particular state, screening of modulators of either the expression of the gene or the gene product itself can be done. The gene products of differentially expressed genes are sometimes referred to herein as "cancer proteins" or a "cancer modulatory protein". The cancer modulatory protein may be a fragment, or alternatively, be the full length protein to the fragment encoded by the nucleic acids of the Tables. Preferably, the cancer modulatory protein is a fragment. In a preferred embodiment, the cancer amino acid sequence which is used to determine sequence identity or similarity is encoded by a nucleic acid of the Tables. In another embodiment, the sequences are naturally occurring allelic variants of a protein encoded by a nucleic acid of the Tables. In another embodiment, the sequences are sequence variants as further described herein.

Preferably, the cancer modulatory protein is a fragment of about 14-24 amino acids long. More preferably the fragment is a soluble fragment. Preferably, the fragment includes a non-transmembrane region. In a preferred embodiment, the fragment has an N-terminal Cys to aid in solubility. In one embodiment, the C-terminus of the fragment is kept as a free acid and the N-terminus is a free amine to aid in coupling, e.g., to cysteine.

In one embodiment the cancer proteins are conjugated to an immunogenic agent as discussed herein. In one embodiment the cancer protein is conjugated to BSA.

Measurements of cancer polypeptide activity, or of cancer or the cancer phenotype can be performed using a variety of assays. For example, the effects of the test compounds upon the function of the cancer polypeptides can be measured by examining parameters described above. A suitable physiological change that affects activity can be used to assess the influence of a test compound on the polypeptides of this invention. When the functional consequences are determined using intact cells or animals, one can also measure a variety of effects such as, in the case of cancer associated with tumors, tumor growth, tumor metastasis, neovascularization, hormone release, transcriptional changes to both known and uncharacterized genetic markers (e.g., northern blots), changes in cell metabolism such as cell growth or pH changes, and changes in intracellular second messengers such as cGMP. In the assays of the invention, mammalian cancer polypeptide is typically used, e.g., mouse, preferably human.

Assays to identify compounds with modulating activity can be performed in vitro. For example, a cancer polypeptide is first contacted with a potential modulator and incubated for a suitable amount of time, e.g., from 0.5-48 hours. In one embodiment, the cancer polypeptide levels are determined in vitro by measuring the level of protein or mRNA. The level of protein is typically measured using immunoassays such as western blotting, ELISA, and the like with an antibody that selectively binds to the cancer polypeptide or a fragment thereof. For measurement of mRNA, amplification, e.g., using PCR, LCR, or hybridization assays, e.g., northern hybridization, RNAse protection, dot blotting, are preferred. The level of protein or mRNA is typically detected using directly or indirectly labeled detection agents, e.g., fluorescently or radioactively labeled nucleic acids, radioactively or enzymatically labeled antibodies, and the like, as described herein.

Alternatively, a reporter gene system can be devised using a cancer protein promoter operably linked to a reporter gene such as luciferase, green fluorescent protein, CAT, or β-gal. The reporter construct is typically transfected into a cell. After treatment with a potential modulator, the amount of reporter gene transcription, translation, or activity is measured according to standard techniques.

In a preferred embodiment, as outlined above, screens may be done on individual genes and gene products (proteins). That is, having identified a particular differentially expressed gene as important in a particular state, screening of modulators of the expression of the gene or the gene product itself can be done. The gene products of differentially expressed genes are sometimes referred to herein as "cancer proteins." The cancer protein may be a fragment, or alternatively, the full length protein to a fragment shown herein.

In one embodiment, screening for modulators of expression of specific genes is performed. Typically, the expression of only one or a few genes are evaluated. In another embodiment, screens are designed to first find compounds that bind to differentially expressed proteins. These compounds are then evaluated for the ability to modulate differentially expressed activity. Moreover, once initial candidate compounds are identified, variants can be further screened to better evaluate structure activity relationships.

In a preferred embodiment, binding assays are done. In general, purified or isolated gene product is used; that is, the gene products of one or more differentially expressed nucleic acids are made. For example, antibodies are generated to the protein gene products, and standard immunoassays are run to determine the amount of protein present. Alternatively, cells comprising the cancer proteins can be used in the assays.

Thus, in a preferred embodiment, the methods comprise combining a cancer protein and a candidate compound, and determining the binding of the compound to the cancer protein. Preferred embodiments utilize the human cancer protein, although other mammalian proteins may also be used, e.g., for the development of animal models of human disease. In some embodiments, as outlined herein, variant or derivative cancer proteins may be used.

Generally, in a preferred embodiment of the methods herein, the cancer protein or the candidate agent is non-diffusably bound to an insoluble support, preferably having isolated sample receiving areas (e.g., a microtiter plate, an array, etc.). The insoluble supports may be made of a composition to which the compositions can be bound, is readily separated from soluble material, and is otherwise compatible with the overall method of screening. The surface of such supports may be solid or porous and of a convenient shape. Examples of suitable insoluble supports include microtiter plates, arrays, membranes, and beads. These are typically made of glass, plastic (e.g., polystyrene), polysaccharides, nylon or nitrocellulose, teflon™, etc. Microtiter plates and arrays are especially convenient because a large number of assays can be carried out simultaneously, using small amounts of reagents and samples. The particular manner of binding of the composition is typically not crucial so long as it is compatible with the reagents and overall methods of the invention, maintains the activity of the composition, and is nondiffusable. Preferred methods of binding include the use of antibodies (which do not sterically block either the ligand binding site or activation sequence when the protein is bound to the support), direct binding to "sticky" or ionic supports, chemical crosslinking, the synthesis of the protein or agent on the surface, etc. Following binding of the protein or agent, excess unbound material is removed by washing. The sample receiving areas may then be blocked through incubation with bovine serum albumin (BSA), casein, or other innocuous protein or other moiety.

In a preferred embodiment, the cancer protein is bound to the support, and a test compound is added to the assay. Alternatively, the candidate agent is bound to the support and the cancer protein is added. Novel binding agents include specific antibodies, non-natural binding agents identified in screens of chemical libraries, peptide analogs, etc. Of particular interest are screening assays for agents that have a low toxicity for human cells. A wide variety of assays may be used for this purpose, including labeled in vitro protein-protein binding assays, electrophoretic mobility shift assays, immunoassays for protein binding, functional assays (phosphorylation assays, etc.), and the like.

The determination of the binding of the test modulating compound to the cancer protein may be done in a number of ways. In a preferred embodiment, the compound is labeled, and binding determined directly, e.g., by attaching all or a portion of the cancer protein to a solid support, adding a labeled candidate agent (e.g., a fluorescent label), washing off excess reagent, and determining whether the label is present on the solid support. Various blocking and washing steps may be utilized as appropriate.

In some embodiments, only one of the components is labeled, e.g., the proteins (or proteinaceous candidate compounds) can be labeled. Alternatively, more than one component can be labeled with different labels, e.g., ¹²⁵I for the proteins and a fluorophor for the compound. Proximity reagents, e.g., quenching or energy transfer reagents are also useful.

In one embodiment, the binding of the test compound is determined by competitive binding assay. The competitor may be a binding moiety known to bind to the target molecule (e.g., a cancer protein), such as an antibody, peptide, binding partner, ligand, etc. Under certain circumstances, there may be competitive binding between the compound and the binding moiety, with the binding moiety displacing the compound. In one embodiment, the test compound is labeled. Either the compound, or the competitor, or both, is added first to the protein for a time sufficient to allow binding, if present. Incubations may be performed at a temperature which facilitates optimal activity, typically between about 4-40° C. Incubation periods are typically optimized, e.g., to facilitate rapid high throughput screening. Typically between 0.1-1 hour will be sufficient. Excess reagent is generally removed or washed away. The second component is then added, and the presence or absence of the labeled component is followed, to indicate binding.

In a preferred embodiment, the competitor is added first, followed by a test compound. Displacement of the competitor is an indication that the test compound is binding to the cancer protein and thus is capable of binding to, and potentially modulating, the activity of the cancer protein. In this embodiment, either component can be labeled. Thus, e.g., if the competitor is labeled, the presence of label in the wash solution indicates displacement by the agent. Alternatively, if the test compound is labeled, the presence of the label on the support indicates displacement.

In an alternative embodiment, the test compound is added first, with incubation and washing, followed by the competitor. The absence of binding by the competitor may indicate that the test compound is bound to the cancer protein with a higher affinity. Thus, if the test compound is labeled, the presence of the label on the support, coupled with a lack of competitor binding, may indicate that the test compound is capable of binding to the cancer protein.

In a preferred embodiment, the methods comprise differential screening to identity agents that are capable of modulating the activity of the cancer proteins. In one embodiment, the methods comprise combining a cancer protein and a competitor in a first sample. A second sample comprises a test compound, a cancer protein, and a competitor. The binding of the competitor is determined for both samples, and a change, or difference in binding between the two samples indicates the presence of an agent capable of binding to the cancer protein and potentially modulating its activity. That is, if the binding of the competitor is different in the second sample relative to the first sample, the agent is capable of binding to the cancer protein.

Alternatively, differential screening is used to identify drug candidates that bind to the native cancer protein, but cannot bind to modified cancer proteins. The structure of the cancer protein may be modeled, and used in rational drug design to synthesize agents that interact with that site. Drug candidates that affect the activity of a cancer protein are also identified by screening drugs for the ability to either enhance or reduce the activity of the protein.

Positive controls and negative controls may be used in the assays. Preferably control and test samples are performed in at least triplicate to obtain statistically significant results. Incubation of all samples is for a time sufficient for the binding of the agent to the protein. Following incubation, samples are washed free of non-specifically bound material and the amount of bound, generally labeled agent determined. For example, where a radiolabel is employed, the samples may be counted in a scintillation counter to determine the amount of bound compound.

A variety of other reagents may be included in the screening assays. These include reagents like salts, neutral proteins, e.g., albumin, detergents, etc., which may be used to facilitate optimal protein-protein binding and/or reduce non-specific or background interactions. Also reagents that otherwise improve the efficiency of the assay, such as protease inhibitors, nuclease inhibitors, anti-microbial agents, etc., may be used. The mixture of components may be added in an order that provides for the requisite binding.

In a preferred embodiment, the invention provides methods for screening for a compound capable of modulating the activity of a cancer protein. The methods comprise adding a test compound, as defined above, to a cell comprising cancer proteins. Preferred cell types include almost any cell. The cells contain a recombinant nucleic acid that encodes a cancer protein. In a preferred embodiment, a library of candidate agents are tested on a plurality of cells.

In one aspect, the assays are evaluated in the presence or absence or previous or subsequent exposure of physiological signals, e.g., hormones, antibodies, peptides, antigens, cytokines, growth factors, action potentials, pharmacological agents including chemotherapeutics, radiation, carcinogenics, or other cells (e.g., cell-cell contacts). In another example, the determinations are determined at different stages of the cell cycle process.

In this way, compounds that modulate cancer agents are identified. Compounds with pharmacological activity are able to enhance or interfere with the activity of the cancer protein. Once identified, similar structures are evaluated to identify critical structural feature of the compound.

In one embodiment, a method of inhibiting cancer cell division is provided. The method comprises administration of a cancer inhibitor. In another embodiment, a method of inhibiting cancer is provided. The method may comprise administration of a cancer inhibitor. In a further embodiment, methods of treating cells or individuals with cancer are provided, e.g., comprising administration of a cancer inhibitor.

In one embodiment, a cancer inhibitor is an antibody as discussed above. In another embodiment, the cancer inhibitor is an antisense molecule.

A variety of cell growth, proliferation, viability, and metastasis assays are available, as described below. Soft agar growth or colony formation in suspension

Normal cells require a solid substrate to attach and grow. When the cells are transformed, they lose this phenotype and grow detached from the substrate. For example, transformed cells can grow in stirred suspension culture or suspended in semi-solid media, such as semi-solid or soft agar. The transformed cells, when transfected with tumor suppressor genes, regenerate normal phenotype and require a solid substrate to attach and grow. Soft agar growth or colony formation in suspension assays can be used to identify modulators of cancer sequences, which when expressed in host cells, inhibit abnormal cellular proliferation and transformation. A therapeutic compound would reduce or eliminate the host cells' ability to grow in stirred suspension culture or suspended in semi-solid media, such as semi-solid or soft.

Techniques for soft agar growth or colony formation in suspension assays are described, e.g., in Freshney (1998) Culture of Animal Cells: A Manual of Basic Technique (3d ed.) Wiley-Liss; Freshney (2000) Culture of Animal Cells: A Manual of Basic Technique (4th ed.) Wiley-Liss; and Garkavtsev, et al. (1996) Nature Genet. 14:415-20. Contact inhibition and density limitation of growth

Normal cells typically grow in a flat and organized pattern in a petri dish until they touch other cells. When the cells touch one another, they are contact inhibited and stop growing. When cells are transformed, however, the cells are not contact inhibited and continue to grow to high densities in disorganized foci. Thus, the transformed cells grow to a higher saturation density than normal cells. This can be detected morphologically by the formation of a disoriented monolayer of cells or rounded cells in foci within the regular pattern of normal surrounding cells. Alternatively, labeling index with (³H)-thymidine at saturation density can be used to measure density limitation of growth. See Freshney (2000), supra. The transformed cells, when transfected with tumor suppressor genes, regenerate a normal phenotype and become contact inhibited and would grow to a lower density.

In this assay, labeling index with (³H)-thymidine at saturation density is a preferred method of measuring density limitation of growth. Transformed host cells are transfected with a cancer-associated sequence and are grown for 24 hours at saturation density in non-limiting medium conditions. The percentage of cells labeling with (³H)-thymidine is determined autoradiographically. See, Freshney (1998), supra. Growth factor or serum dependence

Transformed cells typically have a lower serum dependence than their normal counterparts (see, e.g., Temin (1966) J. Natl. Cancer Insti. 37:167-175; Eagle, et al.(1970) J. Exp. Med. 131:836-879); Freshney, supra. This is in part due to release of various growth factors by the transformed cells. Growth factor or serum dependence of transformed host cells can be compared with that of control. Tumor specific markers levels

Tumor cells release an increased amount of certain factors (hereinafter "tumor specific markers") than their normal counterparts. For example, plasminogen activator (PA) is released from human glioma at a higher level than from normal brain cells (see, e.g., Gullino "Angiogenesis, tumor vascularization, and potential interference with tumor growth" pp. 178-184 in Mihich (ed. 1985) Biological Responses in Cancer Plenum. Similarly, tumor angiogenesis factor (TAF) is released at a higher level in tumor cells than their normal counterparts. See, e.g., Folkman (1992) Sem. Cancer Biol. 3:89-96.

Various techniques which measure the release of these factors are described in Freshney (1998), supra. Also, see, Unkeless, et al. (1974) J. Biol. Chem. 249:4295-4305; Strickland and Beers (1976) J. Biol. Chem. 251:5694-5702; Whur, et al. (1980) Br. J. Cancer 42:305-312; Gullino "Angiogenesis, tumor vascularization, and potential interference with tumor growth" pp. 178-184 in Mihich (ed. 1985) Biological Responses in Cancer Plenum; Freshney (1985) Anticancer Res. 5:111-130. Invasiveness into Matrigel

The degree of invasiveness into Matrigel or some other extracellular matrix constituent can be used as an assay to identify compounds that modulate cancer-associated sequences. Tumor cells exhibit a good correlation between malignancy and invasiveness of cells into Matrigel or some other extracellular matrix constituent. In this assay, tumorigenic cells are typically used as host cells. Expression of a tumor suppressor gene in these host cells would decrease invasiveness of the host cells.

Techniques described in Freshney (1994), supra, can be used. Briefly, the level of invasion of host cells can be measured by using filters coated with Matrigel or some other extracellular matrix constituent. Penetration into the gel, or through to the distal side of the filter, is rated as invasiveness, and rated histologically by number of cells and distance moved, or by prelabeling the cells with ¹²⁵I and counting the radioactivity on the distal side of the filter or bottom of the dish. See, e.g., Freshney (1984), supra. Tumor growth in vivo

Effects of cancer-associated sequences on cell growth can be tested in transgenic or immune-suppressed mice. Knock-out transgenic mice can be made, in which the cancer gene is disrupted or in which a cancer gene is inserted. Knock-out transgenic mice can be made by insertion of a marker gene or other heterologous gene into the endogenous cancer gene site in the mouse genome via homologous recombination. Such mice can also be made by substituting the endogenous cancer gene with a mutated version of the cancer gene, or by mutating the endogenous cancer gene, e.g., by exposure to carcinogens.

A DNA construct is introduced into the nuclei of embryonic stem cells. Cells containing the newly engineered genetic lesion are injected into a host mouse embryo, which is reimplanted into a recipient female. Some of these embryos develop into chimeric mice that possess germ cells partially derived from the mutant cell line. Therefore, by breeding the chimeric mice it is possible to obtain a new line of mice containing the introduced genetic lesion (see, e.g., Capecchi, et al. (1989) Science 244:1288-1292). Chimeric targeted mice can be derived according to Hogan, et al. (1988) Manipulating the Mouse Embryo: A Laboratory Manual CSH Press; and Robertson (ed. 1987) Teratocarcinomas and Embryonic Stem Cells: A Practical Approach IRL Press, Washington, D.C.

Alternatively, various immune-suppressed or immune-deficient host animals can be used. For example, genetically athymic "nude" mouse (see, e.g., Giovanella, et al. (1974) J. Natl. Cancer Inst. 52:921-930), a SCID mouse, a thymectomized mouse, or an irradiated mouse (see, e.g., Bradley, et al. (1978) Br. J. Cancer 38:263-272; Selby, et al. (1980) Br. J. Cancer 41:52-61) can be used as a host. Transplantable tumor cells (typically about 10⁶ cells) injected into isogenic hosts will produce invasive tumors in a high proportions of cases, while normal cells of similar origin will not. In hosts which developed invasive tumors, cells expressing a cancer-associated sequences are injected subcutaneously. After a suitable length of time, preferably 4-8 weeks, tumor growth is measured (e.g., by volume or by its two largest dimensions) and compared to the control. Tumors that have statistically significant reduction (using, e.g., Student's T test) are said to have inhibited growth.

### Polynucleotide modulators of cancer

### Antisense and RNAi Polynucleotides

In certain embodiments, the activity of a cancer-associated protein is down-regulated, or entirely inhibited, by the use of an inhibitory or antisense polynucleotide, e.g., a nucleic acid complementary to, and which can preferably hybridize specifically to, a coding mRNA nucleic acid sequence, e.g., a cancer protein mRNA, or a subsequence thereof. Binding of the antisense polynucleotide to the mRNA reduces the translation and/or stability of the mRNA.

In the context of this invention, antisense polynucleotides can comprise naturally-occurring nucleotides, or synthetic species formed from naturally-occurring subunits or their close homologs. Antisense polynucleotides may also have altered sugar moieties or inter-sugar linkages. Exemplary among these are the phosphorothioate and other sulfur containing species. Analogs are comprehended by this invention so long as they function effectively to hybridize with the cancer protein mRNA. See, e.g., Isis Pharmaceuticals, Carlsbad, CA; Sequitor, Inc., Natick, MA.

Such antisense polynucleotides can readily be synthesized using recombinant means, or can be synthesized in vitro. Equipment for such synthesis is sold by several vendors, including Applied Biosystems. The preparation of other oligonucleotides such as phosphorothioates and alkylated derivatives is also well known.

Antisense molecules as used herein include antisense or sense oligonucleotides. Sense oligonucleotides can, e.g., be employed to block transcription by binding to the anti-sense strand. The antisense and sense oligonucleotide comprise a single-stranded nucleic acid sequence (either RNA or DNA) capable of binding to target mRNA (sense) or DNA (antisense) sequences for cancer molecules. A preferred antisense molecule is for a cancer sequences in the Tables, or for a ligand or activator thereof. Antisense or sense oligonucleotides, according to the present invention, comprise a fragment generally at least about 14 nucleotides, preferably from about 14-30 nucleotides. The ability to derive an antisense or a sense oligonucleotide, based upon a cDNA sequence encoding a given protein is described in, e.g., Stein and Cohen (1988) Cancer Res. 48:2659-2668; and van der Krol, et al. (1988) BioTechniques 6:958-976.

RNA interference is a mechanism to suppress gene expression in a sequence specific manner. See, e.g., Brumelkamp, et al. (2002) Sciencexpress (21March2002); Sharp (1999) Genes Dev. 13:139-141; and Cathew (2001) Curr. Op. Cell Biol. 13:244-248. In mammalian cells, short, e.g., 21 nt, double stranded small interfering RNAs (siRNA) have been shown to be effective at inducing an RNAi response. See, e.g., Elbashir, et al. (2001) Nature 411:494-498. The mechanism may be used to downregulate expression levels of identified genes, e.g., treatment of or validation of relevance to disease.

### Ribozymes

In addition to antisense polynucleotides, ribozymes can be used to target and inhibit transcription of cancer-associated nucleotide sequences. A ribozyme is an RNA molecule that catalytically cleaves other RNA molecules. Different kinds of ribozymes have been described, including group I ribozymes, hammerhead ribozymes, hairpin ribozymes, RNase P, and axhead ribozymes (see, e.g., Castanotto, et al. (1994) Adv. in Pharmacology 25: 289-317 for a general review of the properties of different ribozymes).

The general features of hairpin ribozymes are described, e.g., in Hampel, et al. (1990) Nucl. Acids Res. 18:299-304; European Patent Publication No. 0 360 257; U.S. Patent No. 5,254,678. Methods of preparation are described in, e.g., WO 94/26877; Ojwang, et al. (1993) Proc. Natl. Acad. Sci. USA 90:6340-6344; Yamada, et al. (1994) Human Gene Therapy 1:39-45; Leavitt, et al.(1995) Proc. Natl. Acad. Sci. USA 92:699-703; Leavitt, et al. (1994) Human Gene Therapy 5:1151-120; and Yamada, et al. (1994) Virology 205: 121-126.

Polynucleotide modulators of cancer may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell. Alternatively, a polynucleotide modulator of cancer may be introduced into a cell containing the target nucleic acid sequence, e.g., by formation of an polynucleotide-lipid complex, as described in WO 90/10448. It is understood that the use of antisense molecules or knock out and knock in models may also be used in screening assays as discussed above, in addition to methods of treatment.

Thus, in one embodiment, methods of modulating cancer in cells or organisms are provided. In one embodiment, the methods comprise administering to a cell an anti-cancer antibody that reduces or eliminates the biological activity of an endogenous cancer protein. Alternatively, the methods comprise administering to a cell or organism a recombinant nucleic acid encoding a cancer protein. This may be accomplished in any number of ways. In a preferred embodiment, e.g., when the cancer sequence is down-regulated in cancer, such state may be reversed by increasing the amount of cancer gene product in the cell. This can be accomplished, e.g., by overexpressing the endogenous cancer gene or administering a gene encoding the cancer sequence, using known gene-therapy techniques. In a preferred embodiment, the gene therapy techniques include the incorporation of the exogenous gene using enhanced homologous recombination (EHR), e.g., as described in PCT/US93/0386. Alternatively, e.g., when the cancer sequence is up-regulated in cancer, the activity of the endogenous cancer gene is decreased, e.g., by the administration of a cancer antisense or other inhibitor, e.g., RNAi.

In one embodiment, the cancer proteins of the present invention may be used to generate polyclonal and monoclonal antibodies to cancer proteins. Similarly, the cancer proteins can be coupled, using standard technology, to affinity chromatography columns. These columns may then be used to purify cancer antibodies useful for production, diagnostic, or therapeutic purposes. In a preferred embodiment, the antibodies are generated to epitopes unique to a cancer protein; that is, the antibodies show little or no cross-reactivity to other proteins. The cancer antibodies may be coupled to standard affinity chromatography columns and used to purify cancer proteins. The antibodies may also be used as blocking polypeptides, as outlined above, since they will specifically bind to the cancer protein.

### Methods of identifying variant cancer-associated sequences

Without being bound by theory, expression of various cancer sequences is correlated with cancer. Accordingly, disorders based on mutant or variant cancer genes may be determined. In one embodiment, the invention provides methods for identifying cells containing variant cancer genes, e.g., determining all or part of the sequence of at least one endogenous cancer gene in a cell. In a preferred embodiment, the invention provides methods of identifying the cancer genotype of an individual, e.g., determining all or part of the sequence of at least one cancer gene of the individual. This is generally done in at least one tissue of the individual, and may include the evaluation of a number of tissues or different samples of the same tissue. The method may include comparing the sequence of the sequenced cancer gene to a known cancer gene, e.g., a wild-type gene.

The sequence of all or part of the cancer gene can then be compared to the sequence of a known cancer gene to determine if any differences exist. This can be done using known homology programs, such as Bestfit, etc. In a preferred embodiment, the presence of a difference in the sequence between the cancer gene of the patient and the known cancer gene correlates with a disease state or a propensity for a disease state, as outlined herein.

In a preferred embodiment, the cancer genes are used as probes to determine the number of copies of the cancer gene in the genome.

In another preferred embodiment, the cancer genes are used as probes to determine the chromosomal localization of the cancer genes. Information such as chromosomal localization finds use in providing a diagnosis or prognosis in particular when chromosomal abnormalities such as translocations, and the like are identified in the cancer gene locus.

### Administration of pharmaceutical and vaccine compositions

In one embodiment, a therapeutically effective dose of a cancer protein or modulator thereof, is administered to a patient. By "therapeutically effective dose" herein is meant a dose that produces effects for which it is administered. The exact dose will depend on the purpose of the treatment, and will be ascertainable using known techniques. See, e.g., Ansel, et al. (1999) Pharmaceutical Dosage Forms and Drug Delivery Lippincott; Lieberman (1992) Pharmaceutical Dosage Forms (vols. 1-3) Dekker, ISBN 0824770846, 082476918X, 0824712692, 0824716981; Lloyd (1999) The Art, Science and Technology of Pharmaceutical Compounding Amer. Pharmaceut. Assn.; and Pickar (1998) Dosage Calculations Thomson. Adjustments for cancer degradation, systemic versus localized delivery, and rate of new protease synthesis, as well as the age, body weight, general health, sex, diet, time of administration, drug interaction and the severity of the condition may be necessary. U.S. Patent Application No. 09/687,576, further discloses the use of compositions and methods of diagnosis and treatment in cancer.

A "patient" for the purposes of the present invention includes both humans and other animals, particularly mammals. Thus the methods are applicable to both human therapy and veterinary applications. In the preferred embodiment the patient is a mammal, preferably a primate, and in the most preferred embodiment the patient is human.

The administration of the cancer proteins and modulators thereof of the present invention can be done in a variety of ways, including, but not limited to, orally, subcutaneously, intravenously, intranasally, transdermally, intraperitoneally, intramuscularly, intrapulmonary, vaginally, rectally, or intraocularly. In some instances, e.g., in the treatment of wounds and inflammation, the cancer proteins and modulators may be directly applied as a solution or spray.

The pharmaceutical compositions of the present invention comprise a cancer protein in a form suitable for administration to a patient. In the preferred embodiment, the pharmaceutical compositions are in a water soluble form, such as being present as pharmaceutically acceptable salts, which is meant to include both acid and base addition salts. "Pharmaceutically acceptable acid addition salt" refers to those salts that retain the biological effectiveness of the free bases and that are not biologically or otherwise undesirable, formed with inorganic acids such as hydrochloric acid, hydrobromic acid, sulfuric acid, nitric acid, phosphoric acid, and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. "Pharmaceutically acceptable base addition salts" include those derived from inorganic bases such as sodium, potassium, lithium, ammonium, calcium, magnesium, iron, zinc, copper, manganese, aluminum salts, and the like. Particularly preferred are the ammonium, potassium, sodium, calcium, and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, and ethanolamine.

The pharmaceutical compositions may also include one or more of the following: carrier proteins such as serum albumin; buffers; fillers such as microcrystalline cellulose, lactose, com and other starches; binding agents; sweeteners and other flavoring agents; coloring agents; and polyethylene glycol.

The pharmaceutical compositions can be administered in a variety of unit dosage forms depending upon the method of administration. For example, unit dosage forms suitable for oral administration include, but are not limited to, powder, tablets, pills, capsules and lozenges. It is recognized that cancer protein modulators (e.g., antibodies, antisense constructs, ribozymes, small organic molecules, etc.) when administered orally, should be protected from digestion. This is typically accomplished either by complexing the molecule(s) with a composition to render it resistant to acidic and enzymatic hydrolysis, or by packaging the molecule(s) in an appropriately resistant carrier, such as a liposome or a protection barrier. Means of protecting agents from digestion are available.

The compositions for administration will commonly comprise a cancer protein modulator dissolved in a pharmaceutically acceptable carrier, preferably an aqueous carrier. A variety of aqueous carriers can be used, e.g., buffered saline and the like. These solutions are sterile and generally free of undesirable matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents, and the like, e.g., sodium acetate, sodium chloride, potassium chloride, calcium chloride, sodium lactate, and the like. The concentration of active agent in these formulations can vary widely, and will be selected primarily based on fluid volumes, viscosities, body weight, and the like in accordance with the particular mode of administration selected and the patient's needs (e.g., (1980) Remington's Pharmaceutical Science (18th ed.) Mack, and Hardman and Limbird (eds. 2001) Goodman and Gilman: The Pharmacologial Basis of Therapeutics (10th ed.) McGraw-Hill.

Thus, a typical pharmaceutical composition for intravenous administration would be about 0.1 to 10 mg per patient per day. Dosages from 0.1 up to about 100 mg per patient per day may be used, particularly when the drug is administered to a secluded site and not into the blood stream, such as into a body cavity or into a lumen of an organ. Substantially higher dosages are possible in topical administration. Actual methods for preparing parenterally administrable compositions will be known or apparent.

The compositions containing modulators of cancer proteins can be administered for therapeutic or prophylactic treatments. In therapeutic applications, compositions are administered to a patient suffering from a disease (e.g., a cancer) in an amount sufficient to cure or at least partially arrest the disease and its complications. An amount adequate to accomplish this is defined as a "therapeutically effective dose." Amounts effective for this use will depend upon the severity of the disease and the general state of the patient's health. Single or multiple administrations of the compositions may be administered depending on the dosage and frequency as required and tolerated by the patient. In any event, the composition should provide a sufficient quantity of the agents of this invention to effectively treat the patient. An amount of modulator that is capable of preventing or slowing the development of cancer in a mammal is referred to as a "prophylactically effective dose." The particular dose required for a prophylactic treatment will depend upon the medical condition and history of the mammal, the particular cancer being prevented, as well as other factors such as age, weight, gender, administration route, efficiency, etc. Such prophylactic treatments may be used, e.g., in a mammal who has previously had cancer to prevent a recurrence of the cancer, or in a mammal who is suspected of having a significant likelihood of developing cancer based, at least in part, upon gene expression profiles. Vaccine strategies may be used, in either a DNA vaccine form, or protein vaccine.

It will be appreciated that the present cancer protein-modulating compounds can be administered alone or in combination with additional cancer modulating compounds or with other therapeutic agent, e.g., other anti-cancer agents or treatments.

In numerous embodiments, one or more nucleic acids, e.g., polynucleotides comprising nucleic acid sequences set forth in the Tables, such as RNAi, antisense polynucleotides or ribozymes, will be introduced into cells, in vitro or in vivo. The present invention provides methods, reagents, vectors, and cells useful for expression of cancer-associated polypeptides and nucleic acids using in vitro (cell-free), ex vivo or in vivo (cell or organism-based) recombinant expression systems.

The particular procedure used to introduce the nucleic acids into a host cell for expression of a protein or nucleic acid is application specific. Many procedures for introducing foreign nucleotide sequences into host cells may be used. These include the use of calcium phosphate transfection, spheroplasts, electroporation, liposomes, microinjection, plasma vectors, viral vectors, and other well known methods for introducing cloned genomic DNA, cDNA, synthetic DNA, or other foreign genetic material into a host cell (see, e.g., Berger and Kimmel (1987) Guide to Molecular Cloning Techniques from Methods in Enzymology (vol. 152) Academic Press; Ausubel, et al. (eds. 1999 and supplements) Current Protocols Lippincott; and Sambrook, et al. (2001) Molecular Cloning: A Laboratory Manual (3d ed., Vol. 1-3) CSH Press.

In a preferred embodiment, cancer proteins and modulators are administered as therapeutic agents, and can be formulated as outlined above. Similarly, cancer genes (including both the full-length sequence, partial sequences, or regulatory sequences of the cancer coding regions) can be administered in a gene therapy application. These cancer genes can include inhibitory applications, e.g., as inhibitory RNA, gene therapy (e.g., for incorporation into the genome), or antisense compositions.

Cancer polypeptides and polynucleotides can also be administered as vaccine compositions to stimulate HTL, CTL, and antibody responses. Such vaccine compositions can include, e.g., lipidated peptides (see, e.g.,Vitiello, et al. (1995) J. Clin. Invest. 95:341-349), peptide compositions encapsulated in poly(DL-lactide-co-glycolide) ("PLG") microspheres (see, e.g., Eldridge, et al. (1991) Molec. Immunol. 28:287-294,; Alonso, et al. (1994) Vaccine 12:299-306; Jones, et al. (1995) Vaccine 13:675-681), peptide compositions contained in immune stimulating complexes (ISCOMS) (see, e.g., Takahashi, et al. (1990) Nature 344:873-875; Hu, et al. (1998) Clin Exp Immunol. 113:235-243), multiple antigen peptide systems (MAPs) (see, e.g., Tam (1988) Proc. Natl. Acad. Sci. USA 85:5409-5413; Tam (1996) J. Immunol. Methods 196:17-32), peptides formulated as multivalent peptides; peptides for use in ballistic delivery systems, typically crystallized peptides, viral delivery vectors (Perkus, et al., p. 379, in Kaufmann (ed. 1996) Concepts in Vaccine Development de Gruyter; Chakrabarti, et al. (1986) Nature 320:535-537; Hu, et al. (1986) Nature 320:537-540; Kieny, et al. (1986) Bio/Technology 4:790-795; Top, et al. (1971) J. Infect. Dis. 124:148-154; Chanda, et al. (1990) Virology 175:535-547), particles of viral or synthetic origin (see, e.g., Kofler, et al. (1996) J. Immunol. Methods 192:25-35; Eldridge, et al. (1993) Sem. Hematol. 30:16-24; Falo, et al. (1995) Nature Med. 1:649-653), adjuvants (Warren, et al. (1986) Annu. Rev. Immunol. 4:369-388; Gupta, et al. (1993) Vaccine 11:293-306), liposomes (Reddy, et al. (1992) J. Immunol. 148:1585-1589; Rock (1996) Immunol. Today 17:131-137), or, naked or particle absorbed cDNA (Ulmer, et al. (1993) Science 259:1745-1749; Robinson, et al. (1993) Vaccine 11:957-960; Shiver, et al., p 423, in Kaufinann (ed. 1996) Concepts in Vaccine Development de Gruyter; Cease and Berzofsky (1994) Annu. Rev. Immunol. 12:923-989; and Eldridge, et al. (1993) Sem. Hematol. 30:16-24). Toxin-targeted delivery technologies, also known as receptor mediated targeting, such as those of Avant Immunotherapeutics, Inc. (Needham, Massachusetts) may also be used.

Vaccine compositions often include adjuvants. Many adjuvants contain a substance designed to protect the antigen from rapid catabolism, such as aluminum hydroxide or mineral oil, and a stimulator of immune responses, such as lipid A, Bortadella pertussis, or Mycobacterium tuberculosis derived proteins. Certain adjuvants are commercially available as, e.g., Freund's Incomplete Adjuvant and Complete Adjuvant (Difco Laboratories, Detroit, MI); Merck Adjuvant 65 (Merck and Company, Inc., Rahway, NJ); AS-2 (SmithKline Beecham, Philadelphia, PA); aluminum salts such as aluminum hydroxide gel (alum) or aluminum phosphate; salts of calcium, iron, or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF, interleukin-2, -7, -12, and other like growth factors, may also be used as adjuvants.

Vaccines can be administered as nucleic acid compositions wherein DNA or RNA encoding one or more of the polypeptides, or a fragment thereof, is administered to a patient. This approach is described, for instance, in Wolff et. al. (1990) Science 247:1465-1468, as well as U.S. Patent Nos. 5,580,859; 5,589,466; 5,804,566; 5,739,118; 5,736,524; 5,679,647; WO 98/04720; and in more detail below. Examples of DNA-based delivery technologies include "naked DNA", facilitated (bupivicaine, polymers, peptide-mediated) delivery, cationic lipid complexes, and particle-mediated ("gene gun") or pressure-mediated delivery (see, e.g., U.S. Patent No. 5,922,687).

For therapeutic or prophylactic immunization purposes, the peptides of the invention can be expressed by viral or bacterial vectors. Examples of expression vectors include attenuated viral hosts, such as vaccinia or fowlpox. This approach involves the use of vaccinia virus, e.g., as a vector to express nucleotide sequences that encode cancer polypeptides or polypeptide fragments. Upon introduction into a host, the recombinant vaccinia virus expresses the immunogenic peptide, and thereby elicits an immune response. Vaccinia vectors and methods useful in immunization protocols are described in, e.g., U.S. Patent No. 4,722,848. Another vector is BCG (Bacille Calmette Guerin). BCG vectors are described in Stover, et al. (1991) Nature 351:456-460. A wide variety of other vectors are availablel for therapeutic administration or immunization, e.g., adeno and adeno-associated virus vectors, retroviral vectors, Salmonella typhi vectors, detoxified anthrax toxin vectors, and the like. See, e.g., Shata, et al. (2000) Mol Med Today 6:66-71; Shedlock, et al. (2000) J. Leukoc. Biol. 68:793-806; Hipp, et al. (2000) In Vivo 14:571-85.

Methods for the use of genes as DNA vaccines are well known, and include placing a cancer gene or portion of a cancer gene under the control of a regulatable promoter or a tissue-specific promoter for expression in a cancer patient. The cancer gene used for DNA vaccines can encode full-length cancer proteins, but more preferably encodes portions of the cancer proteins including peptides derived from the cancer protein. In one embodiment, a patient is immunized with a DNA vaccine comprising a plurality of nucleotide sequences derived from a cancer gene. For example, cancer-associated genes or sequence encoding subfragments of a cancer protein are introduced into expression vectors and tested for their immunogenicity in the context of Class I MHC and an ability to generate cytotoxic T cell responses. This procedure provides for production of cytotoxic T cell responses against cells which present antigen, including intracellular epitopes.

In a preferred embodiment, DNA vaccines include a gene encoding an adjuvant molecule with the DNA vaccine. Such adjuvant molecules include cytokines that increase the immunogenic response to the cancer polypeptide encoded by the DNA vaccine. Additional or alternative adjuvants are available.

In another preferred embodiment, cancer genes find use in generating animal models of cancer. When the cancer gene identified is repressed or diminished in cancer tissue, gene therapy technology, e.g., wherein inhibitory or antisense RNA directed to the cancer gene will also diminish or repress expression of the gene. Animal models of cancer find use in screening for modulators of a cancer-associated sequence or modulators of cancer. Similarly, transgenic animal technology, including gene knockout technology, e.g., as a result of homologous recombination with an appropriate gene targeting vector, will result in the absence or increased expression of the cancer protein. When desired, tissue-specific expression or knockout of the cancer protein may be necessary.

It is also possible that the cancer protein is overexpressed in cancer. As such, transgenic animals can be generated that overexpress the cancer protein. Depending on the desired expression level, promoters of various strengths can be employed to express the transgene. Also, the number of copies of the integrated transgene can be determined and compared for a determination of the expression level of the transgene. Animals generated by such methods will find use as animal models of cancer and are additionally useful in screening for modulators to treat cancer.

### Kits for Use in Diagnostic and/or Prognostic Applications

For use in diagnostic, research, and therapeutic applications suggested above, kits are also provided by the invention. In diagnostic and research applications, such kits may include at least one of the following: assay reagents, buffers, cancer-specific nucleic acids or antibodies, hybridization probes and/or primers, antisense polynucleotides, ribozymes, dominant negative cancer polypeptides or polynucleotides, small molecule inhibitors of cancer-associated sequences etc. A therapeutic product may include sterile saline or another pharmaceutically acceptable emulsion and suspension base.

In addition, the kits may include instructional materials containing instructions (e.g., protocols) for the practice of the methods of this invention. While the instructional materials typically comprise written or printed materials, they are not limited to such. A medium capable of storing such instructions and communicating them to an end user is contemplated by this invention. Such media include, but are not limited to, electronic storage media (e.g., magnetic discs, tapes, cartridges, chips), optical media (e.g., CD ROM), and the like. Such media may include addresses to internet sites that provide such instructional materials.

The present invention also provides for kits for screening for modulators of cancer-associated sequences. Such kits can be prepared from readily available materials and reagents. For example, such kits can comprise one or more of the following materials: a cancer-associated polypeptide or polynucleotide, reaction tubes, and instructions for testing cancer-associated activity. Optionally, the kit contains biologically active cancer protein. A wide variety of kits and components can be prepared according to the present invention, depending upon the intended user of the kit and the particular needs of the user. Diagnosis would typically involve evaluation of a plurality of genes or products. The genes will typically be selected based on correlations with important parameters in disease which may be identified in historical or outcome data.

### EXAMPLES

### Example 1: Gene Chip Analysis

Molecular profiles of various normal and cancerous tissues were determined and analyzed using gene chips. RNA was isolated and gene chip analysis was performed as described (Glynne, et al. (2000) Nature 403:672-676; Zhao, et al. (2000) Genes Dev. 14:981-993).

Table 1 lists medical conditions, abnormalities, or organs affected by disease, referred to in Tables 2A-68, for which markers have been identified, and other related medical conditions (including various stages and/or metastases) in which those markers will also be useful, e.g., in therapeutic, diagnostic, prognostic, subsetting, vaccine, and other uses.
blood vessels/angiogenesis: hemangiomas, lymphangiomas, wound healing, tissue remodeling, psoriasis, ischemic, heart disease, inflammatory diseases (e.g, arthritis, asthma, chronic bronchitis), atherosclerosis, endometriosis, presumed ocular histoplasmosis syndrame, hypoxia, solid tumors, lymphomas, autoimmune diseases (e.g., RA, SLE, juvenile chronic arthritis, pigmented villonodular synovitis, etc.), retinal neovascularization syndromes (e.g., diabetic retinopathy, macular degeneration, presumed ocular histoplasmosis syndrome, etc.), scleritis/conjunctivitis, hypertrophic scars (keloid), birth control, uterine fibroids
bladder: carcinoma in situ, papillary carcinomas, transitional cell carcinoma
bone marrow: Ewing sarcoma, sarcomas arising from skeletal and extrasketetal connective tissues, including the peripheral nervous system
brain: glioblastoma, oligodendroglioma, anablastic astrocyloma, meningioma, medulablastoma, neuroblastoma, ependymoma, schwannoma, craniopharyngiama, pineoblastoma, pineocytoma
breast: ductal carcinoma in situ, lobular carcinoma in situ
cervix: cancer of the cervix, vagina, or vulva
colon/rectum: precancerous colarectal disease (e.g., neoplastic polyps (adenomas), familial adenomatous polyposis, ulcerative colitis), colon cancer, e.g. epithelial tumor (e.g. adenocarcinoma, mucinous adenocarcinoma, signet-ring cell adenocarcinoma, squamous cell carcinoma, adenosquamous carcinoma, undifferentiated carcinoma, unclassified carcinoma), carinoid tumor (e.g., argenlaffin, nonargentaffin, composite), non-epithelial tumor (e.g., leimyo sarcoma, others), inflammatory bowel disease (e.g., ulcerative colitis. Crohn's disease (granulomatous colitis), dysplasia), rectal cancer, cancer of the anal region (e.g., squamous cell carcinoma, transitional carcinoma, adenocarcinoma, carcinoma, papillary vilous carcinoma, mucinous adenocarcinoma, melanoma)
esophagus: premalignant or predisposing conditions (e.g., esophagitis), squamous cell cancers (e.g., cancers of the head and neck lung, or cervix), gastrodigestive carcinomas (e.g., cancers of the stomach, colon, or rectum)
fibrosis: lung fibrosis (idiopathic pulmonary fibrosis, hypersensitivity pneumonilis, interstital, pneumonitis, nonspecific idiopathic pneumonitis), chronic obstructive pulmonary disease (e.g., emphysema, chronic bronchitis), asthma, bronchlectasis, cirrhosis (liver fibrosis), renal fibrosis, sderoderma, wound healing
head and neck: tumors of the nasal cavity, paranasal sinuses, nasopharynx, oral cavity, oral pharynx, tip, larynx, hypopharynx, salivary glands, paragangliomas, esophagus
kidney: dear cell (nonpapillary) carcinoma, papillary carcinoma, chromophobe renal carcinoma, hypemephroma, adenocarcioma, sporadic renal carcinomas, hereditary renal carcinomas (von Hippel-Lindau disease), carcionoma of the renal pelvis, ureteral carcinoma, fibroma, papillary adenoma, angiomyolipoma, oncocytoma
leukocytes: acute lymphoblastic leukemia/lymphoma, malignant transformation of immature, precursor B (pre-B) or precursor T (pre-T) lymphocytes, or lymphoblasts, arthritis, Inflammation, wound healing
liver: hepatitis (e.g., types A, B, C), benign epithelial tumors and tumor bile conditions, primary malignant epithelial tumors, primary malignant mesenchymal tumors, tumors of the galibladder or bile duct
lung: lung cancer, small cell lung carcinoma (oat cell carcinoma), non-small cell carcinomas (e.g., squamous cell carcinoma, adenocarcinoma, large cell lung carcinoma, carcinoid, granulomatous), fibrosis (idiopathic pulmonary fibrosis, hypersensitivity pneumonitis,interslitial pneumonitis, nonspecific idiopathic pneumonitis), chronic obstructive pulmonary disease (e.g., emphysema, chronic bronchitis), asthma, bronchieclass, esophageal cancer
ovary: ovarian carcinoma (e.g., epithelial (serous tumors, mucinous tumors, endometrioid tumors), germ cell (e.g., teratomas, choriocarcinomas, polyembryomas, embryomal carcinoma, endodermal sinus tumor, dysgerminoma, gonadoblastoma), stromal carcinomas (e.g., granulosal stromal cell tumors)), fallopian tube carcinoma, peritoneal carcinoma, leiomyoma
pancreas: adenocarcinoma, ductal adenocarcinoma, mudnous cyst adenocarcinoma, acinar cell carcinoma, unclassified large cell carcinoma, small call carcinoma, pancreatoblastoma, duct-ectatic mucin-hypersecreling tumor, mucinous cyst adenoma, papillary cystic neoplasm, serous cyst adenoma, diabetes metitis, chronic pancreatilis
prostate: epithelial neoplasms (e.g., adenocarcinoma, small cell tumors, transitional cell carcinoma, carcinoma in situ, and basal call carcinoma), carcinosarcoma, non-epithetial neoplasms (e.g., mesenchymal and lymphoma), germ cell tumors, prostatic intraepithelial neoplasia (PIN), hormone independent prostate cancer, benign prostate hyperplasia, prostatitis
skin/melanoma: melanoma, lentigo (common benign localized hyperplasia of melanocytes), nevocellular nevi (congenital or acquired neoplasm of melanocytes), actinic keratosis (overgrowth of outer layers of skin), basal cell carcinoma, Merkel cell carcinoma, benign fibrous histiocytoma (dermal neoplasms of fibroblasts and histiocytes), dermatotibrosarcoma protuberans (well differentiated fibrosarcoma of the skin), xanthomas (tumor-like collections of foamy histiocyles within the dermis), dermal vascular tumors, seborrheic keratoses (benign tumor), acanthosis nigricans (benign or malignant hyperplasia and hyperpigmentation of skin), and squamous cell carcinomas of the skin, lung, cervix, esophagus, uterus, head, neck, or bladder
stomach: adenocarcinoma, squamous call carcinoma, adenoacanthoma, carcinoid, leiomyosarcoma, gastritis (chronic atrophic, H. pylori associated), hyperplastic polyps, lipoma, leiomyoma, esophageal adenocarcinomas
testicles: germ cell tumors (including seminomas, embryonal carcinomas, teratomas, choriocarcinomas, yolk sac tumors), sex chord stromal tumors (including Leydig cell tumors, Sertoli cell tumors, and Granulosa cell tumors), germ cell and gonadal stromal elements (e.g., gonadoblastomas), adnexal and paratesticular tumors (e.g., mesotheliomas, soft tissue sarcomas, and adnexal of the rete testes), miscellaneous neoplasms (including carcinoid, lymphoma, and cysts)
uterus: epithelial tumors (e.g., endometrioid, papillary endometrioid, papillary serous, clear cell, mucinous), mesenchymal tumors (e.g., endometrial stromal sarcoma, leiomyosarcoma, nonspecific sarcomas), mixed tumors (e.g., malignant mixed mullerian tumors, adenosarcoma)

Tables 2B-66C list accession numbers for Pkeys lacking UnigenelD's for Tables 2A-66C, respectively. For each probeset is listed gene duster number from which oligonucleotides were designed. Gene clusters were compiled using sequences derived from Genbank ESTs and mRNAs. These sequences were clustered based on sequence similarity using Clustering and Alignment Tools (DoubleTwist. Oakland California). Genbank accession numbers for sequences comprising each cluster are listed in the Accession" column.

Tables 2C-66C list genomic positioning for Pkeys lacking Unigene ID's and accession numbers in Tables 2A-66C, respectively. For each predicted exon is listed genomic sequence source used for prediction. Nucleotide locations of each predicted exon are also listed.

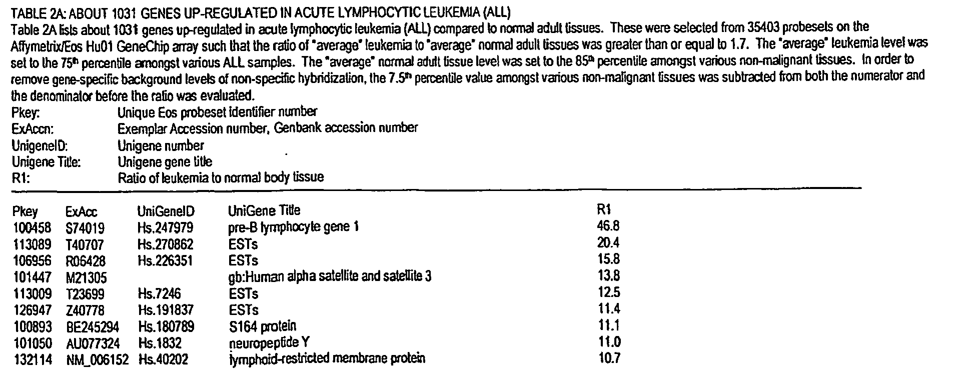

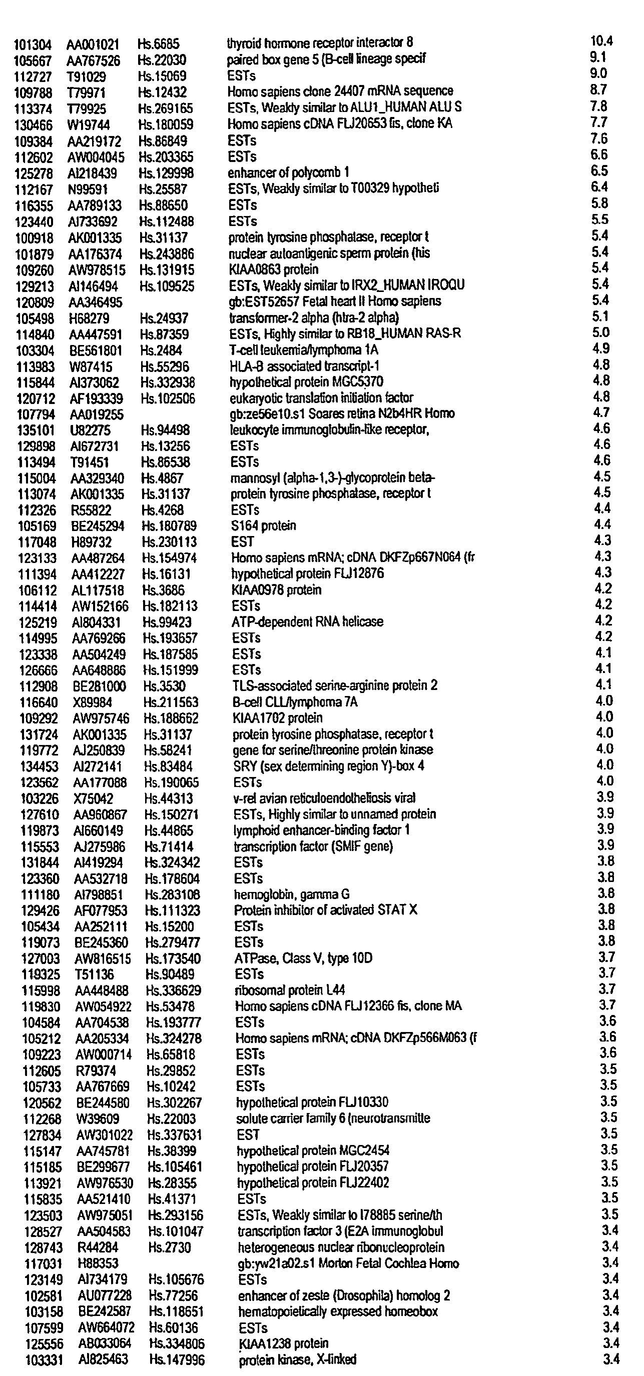

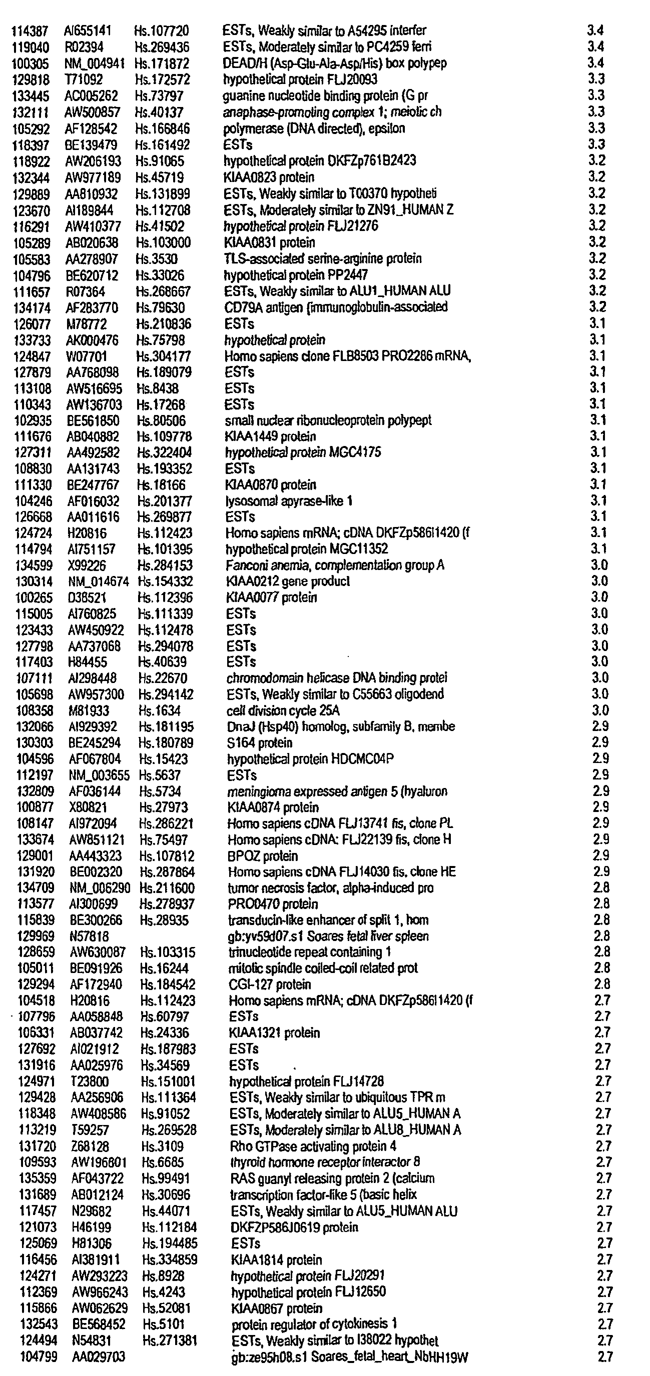

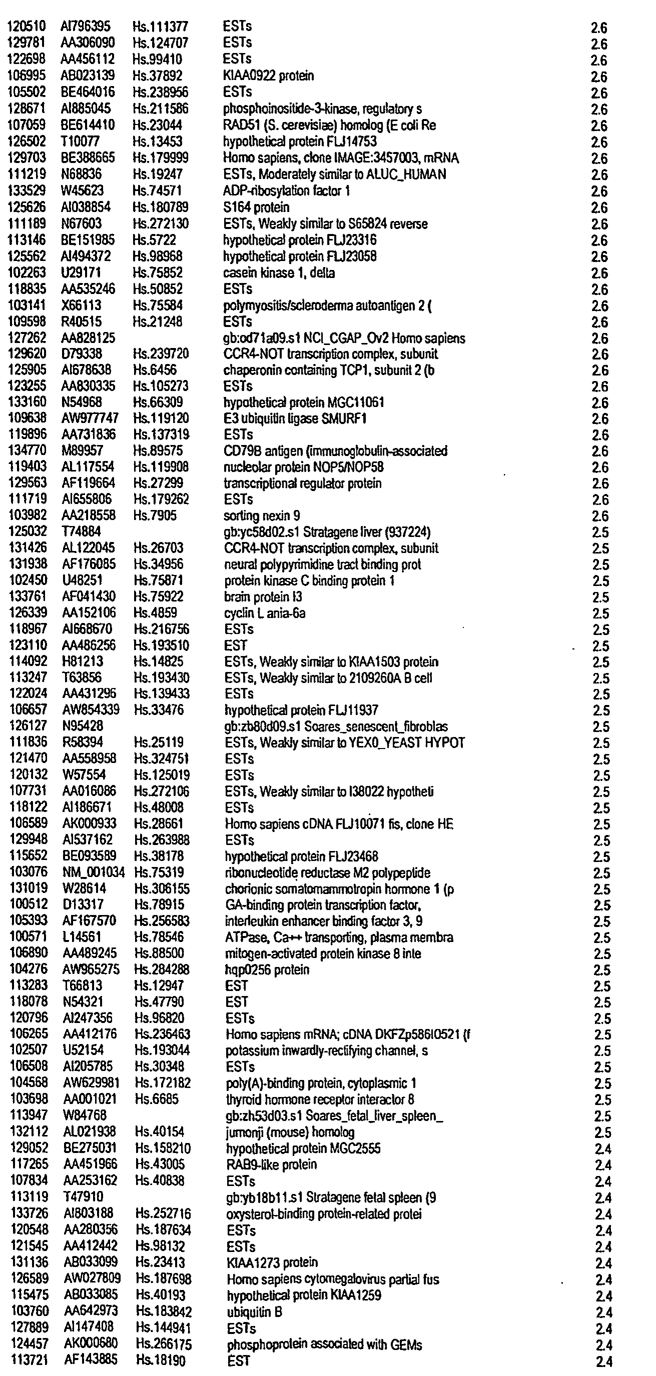

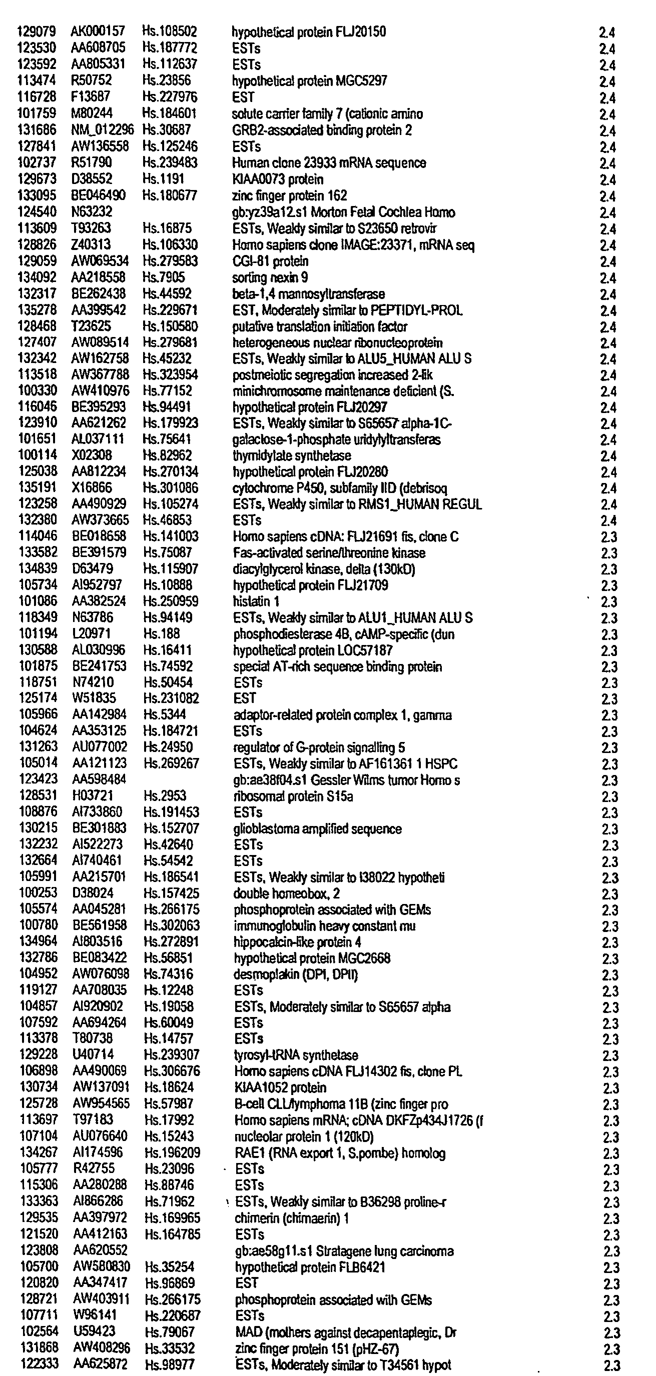

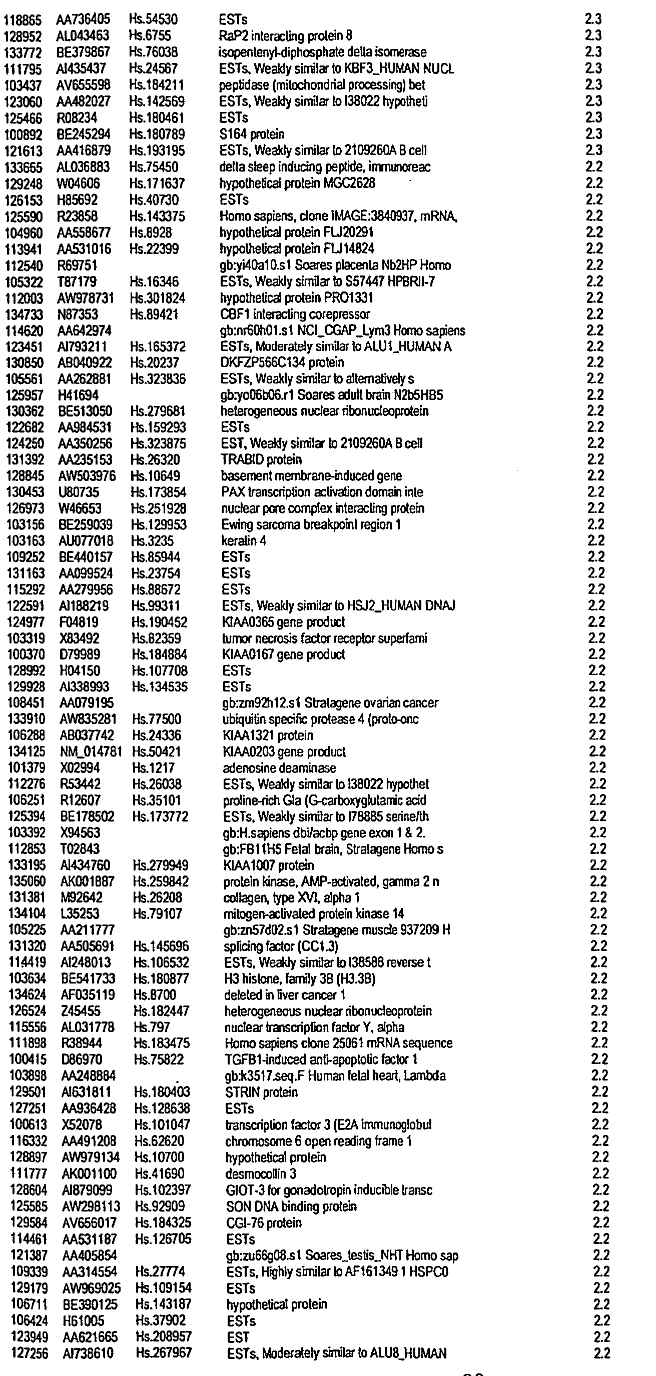

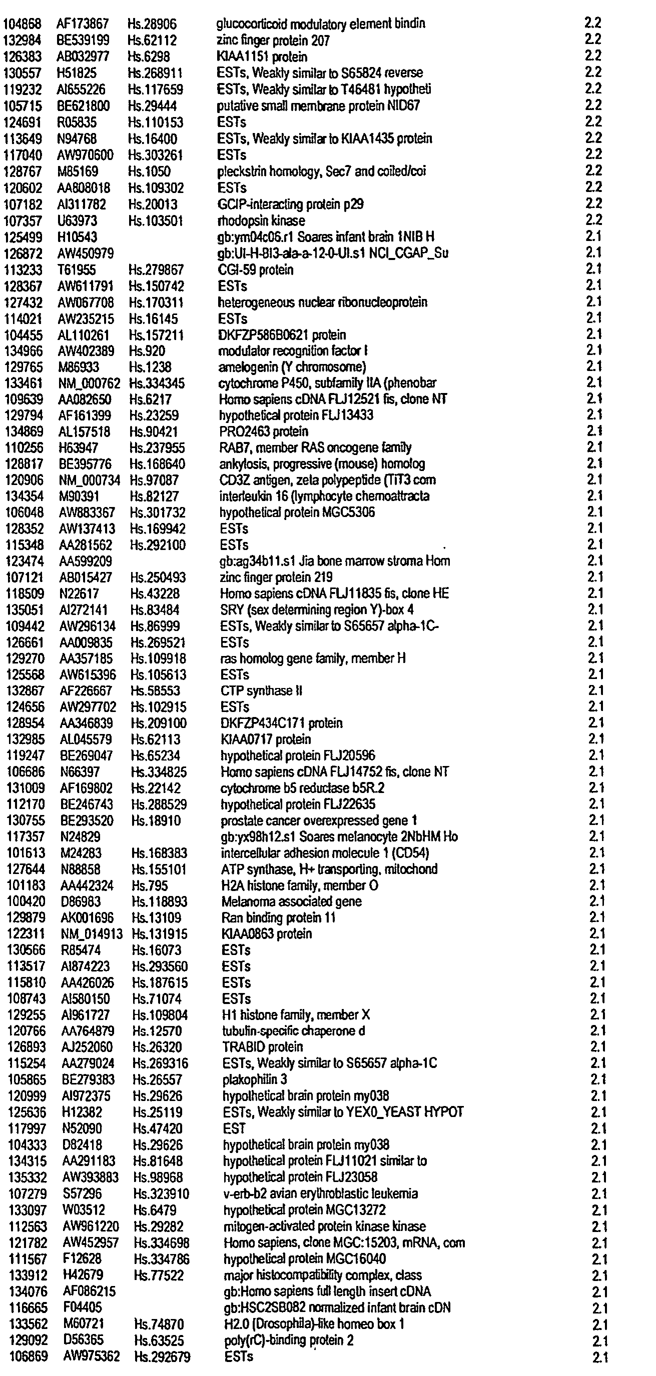

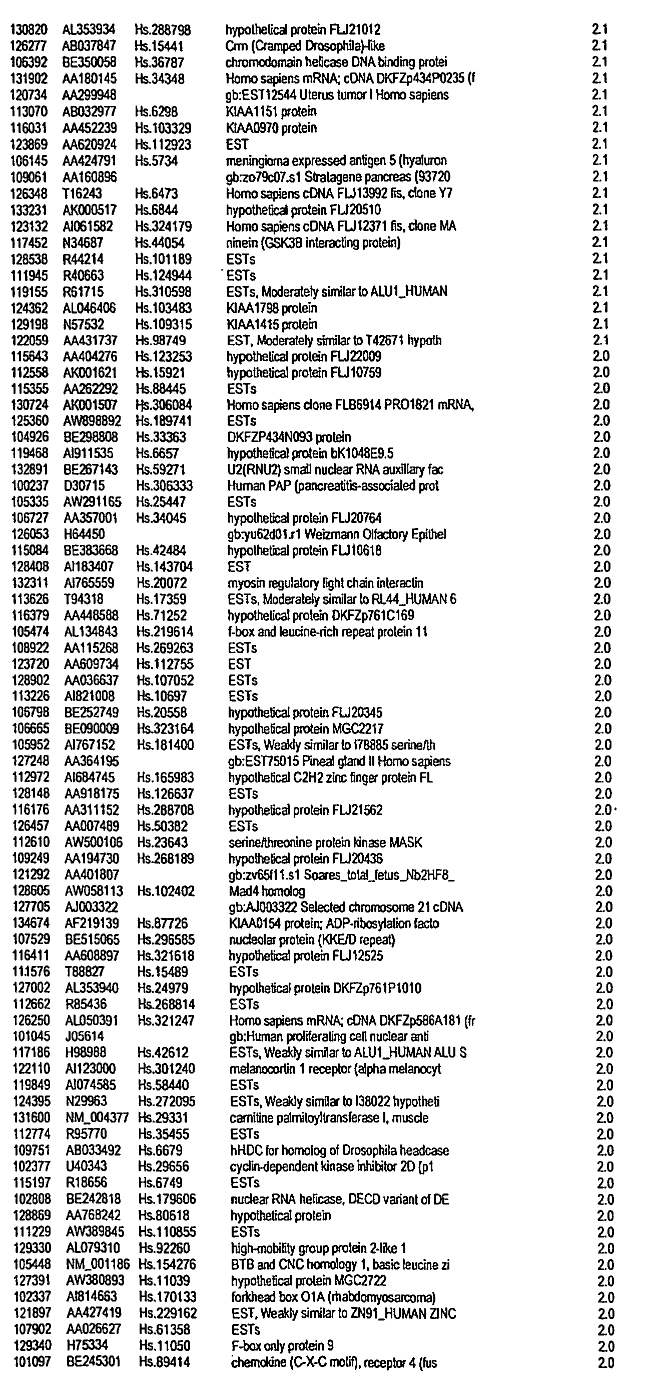

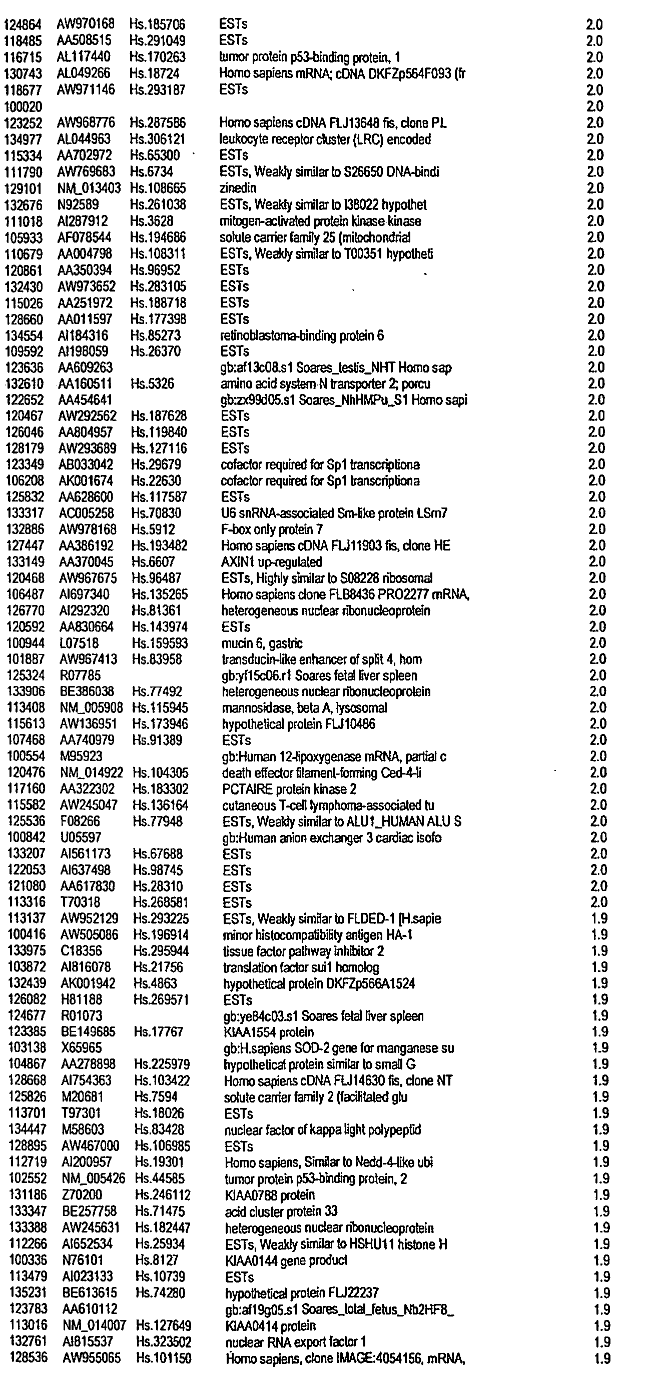

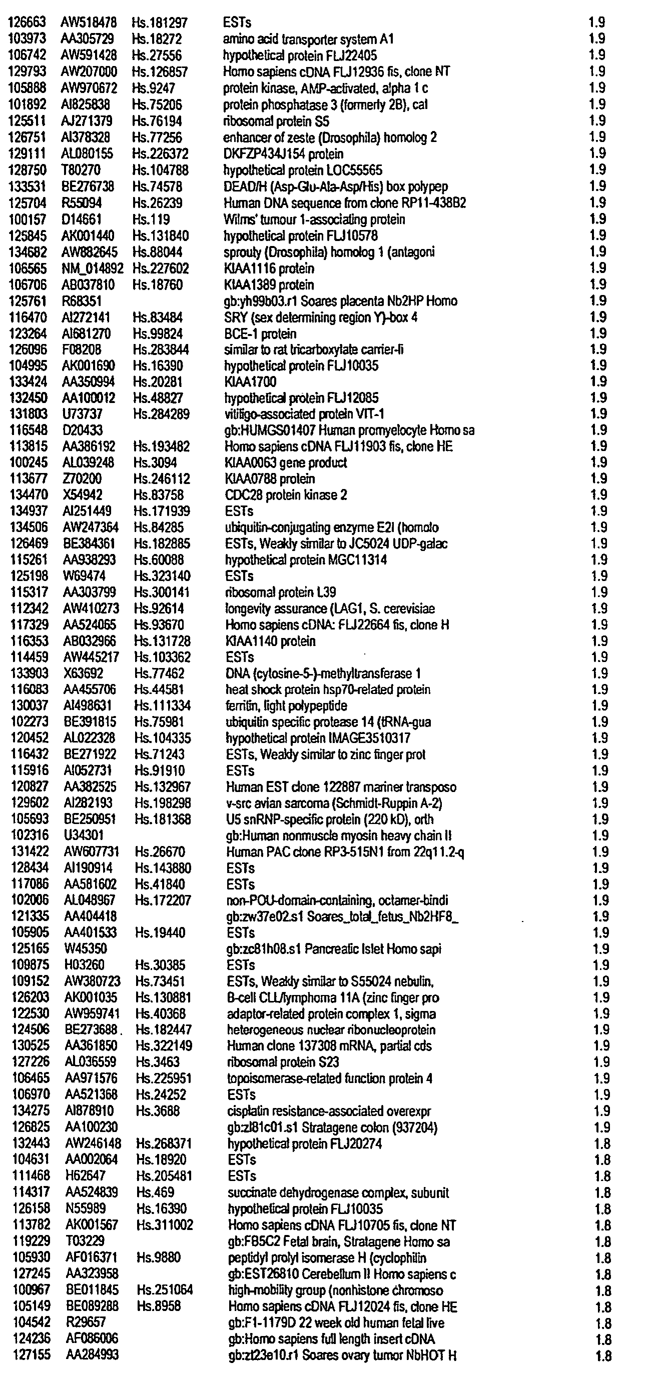

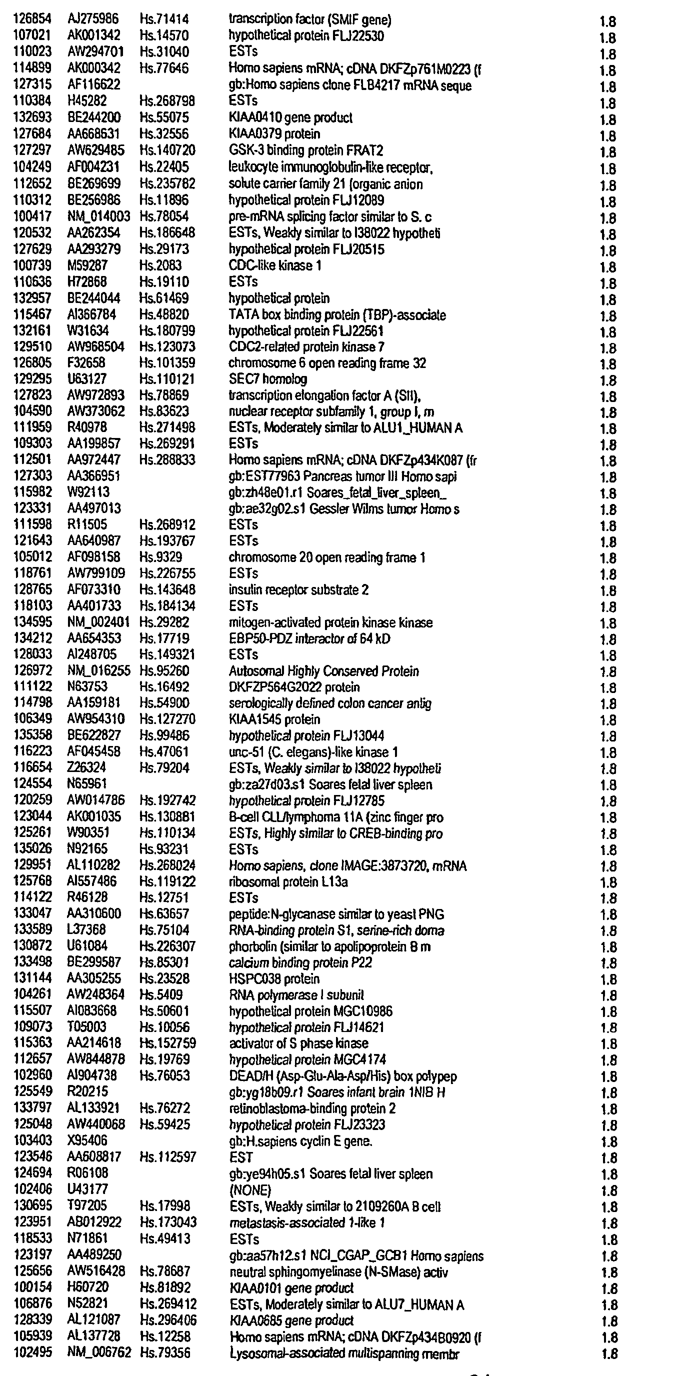

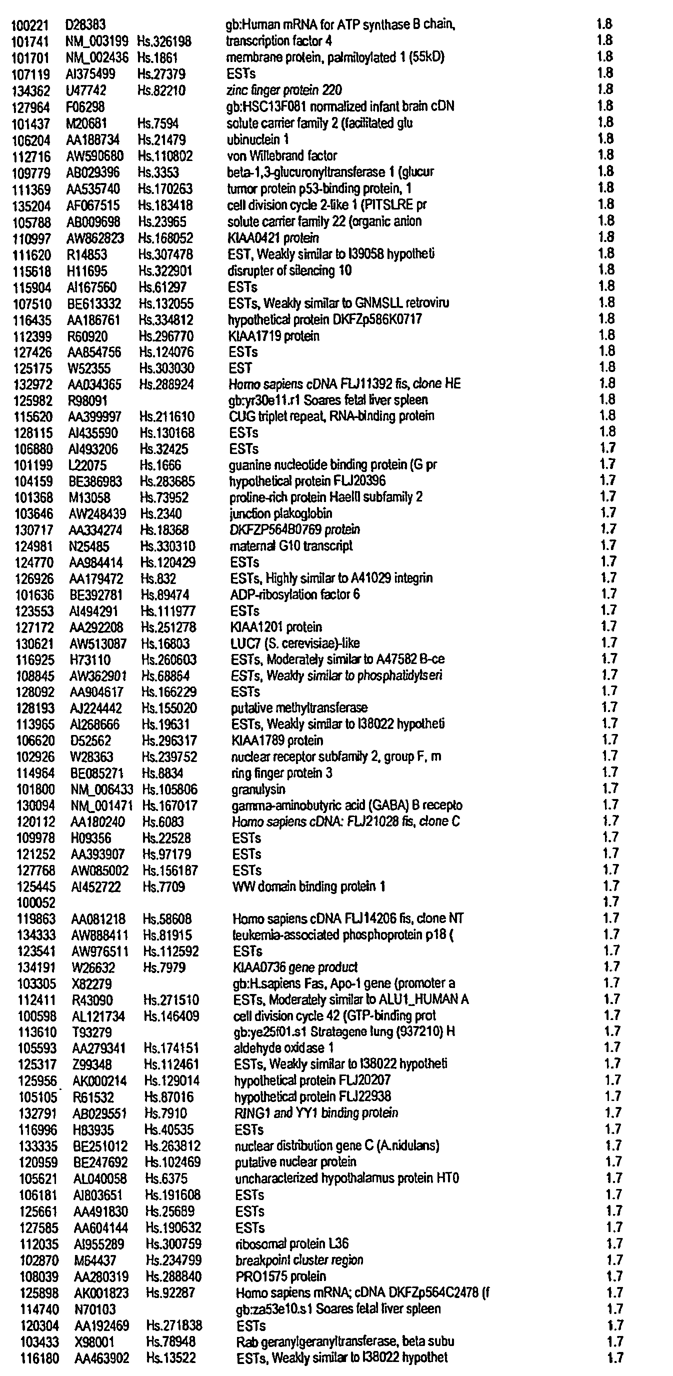

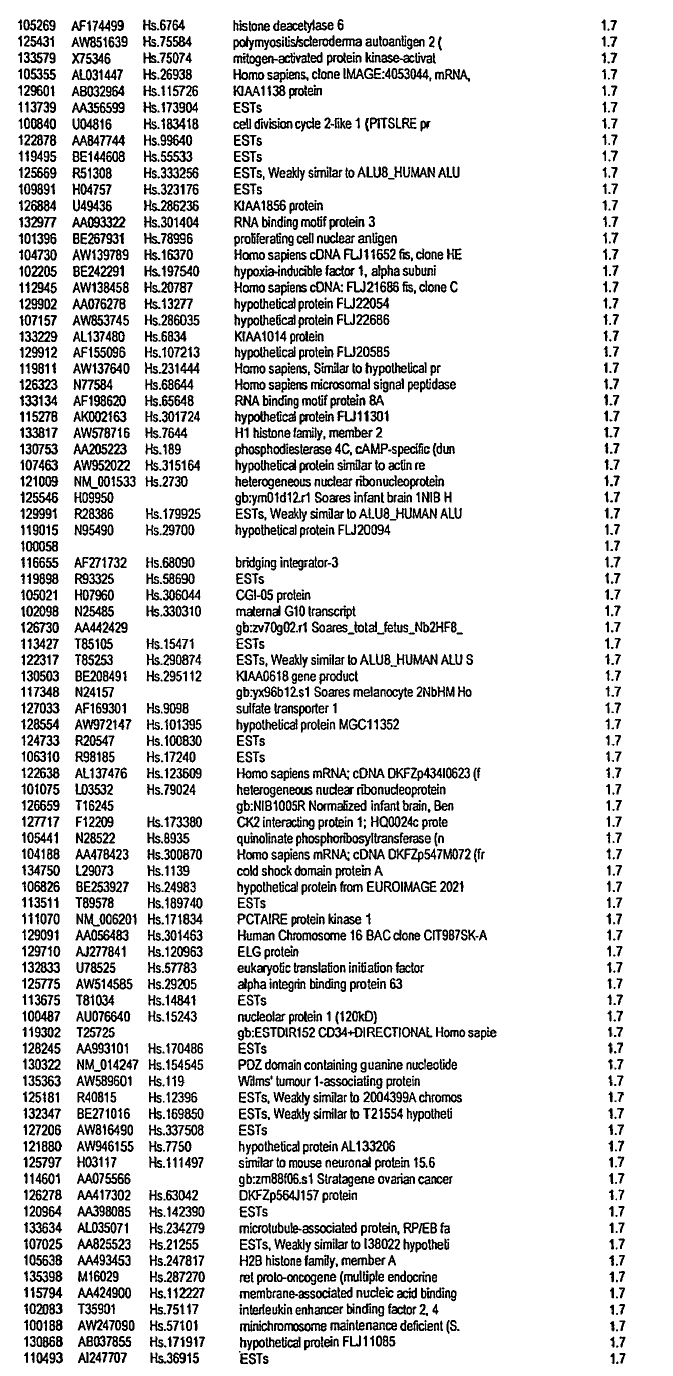

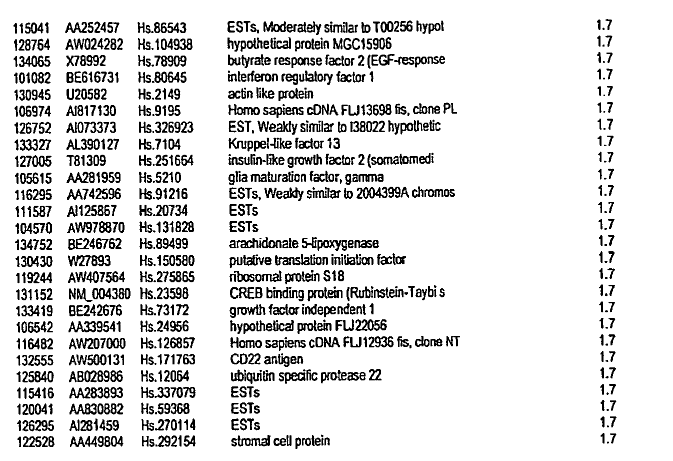

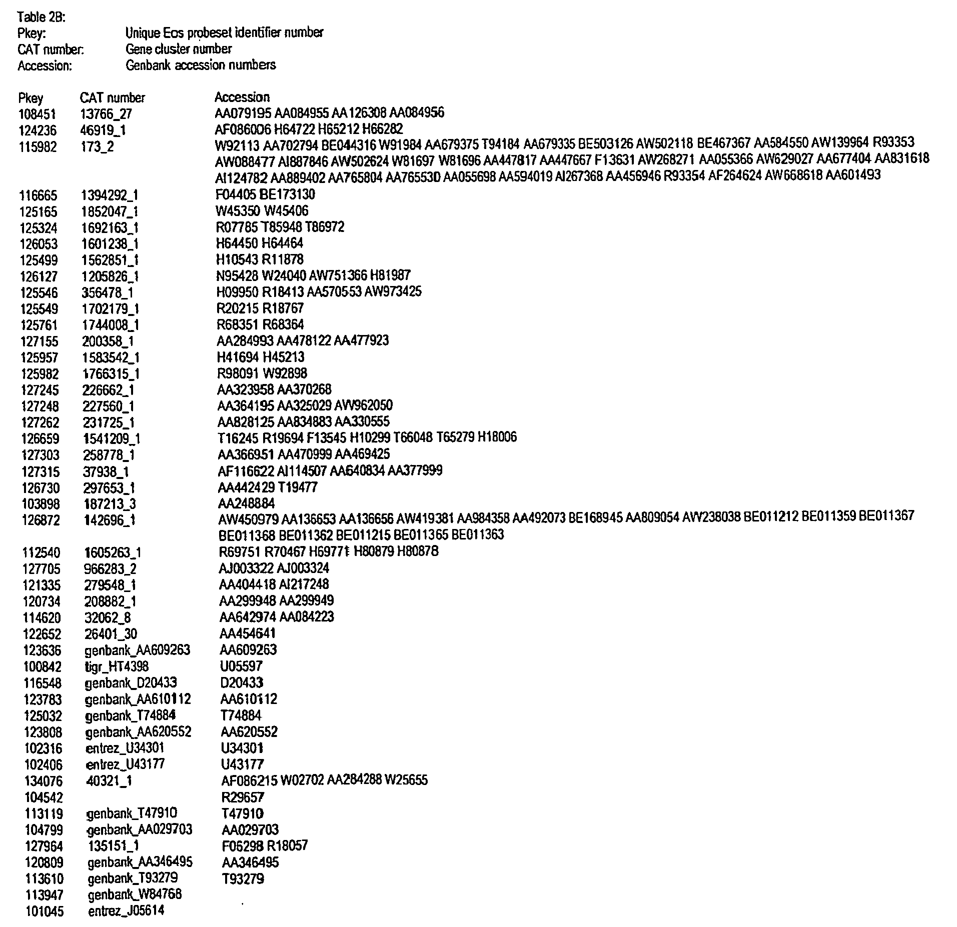

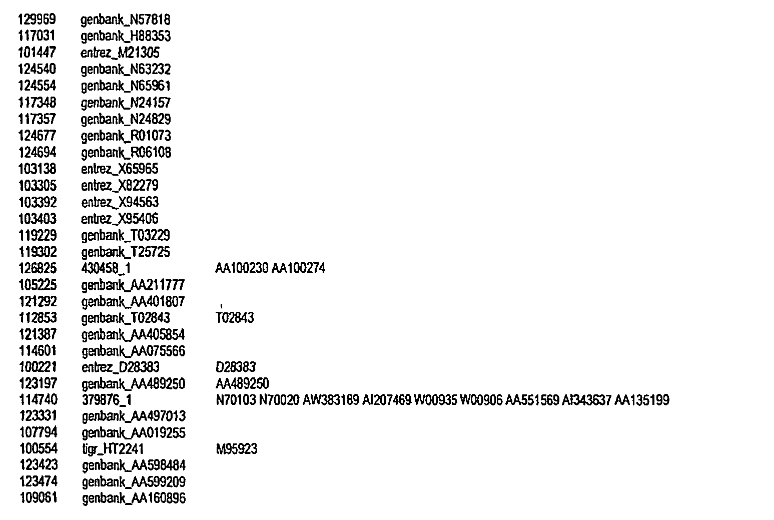

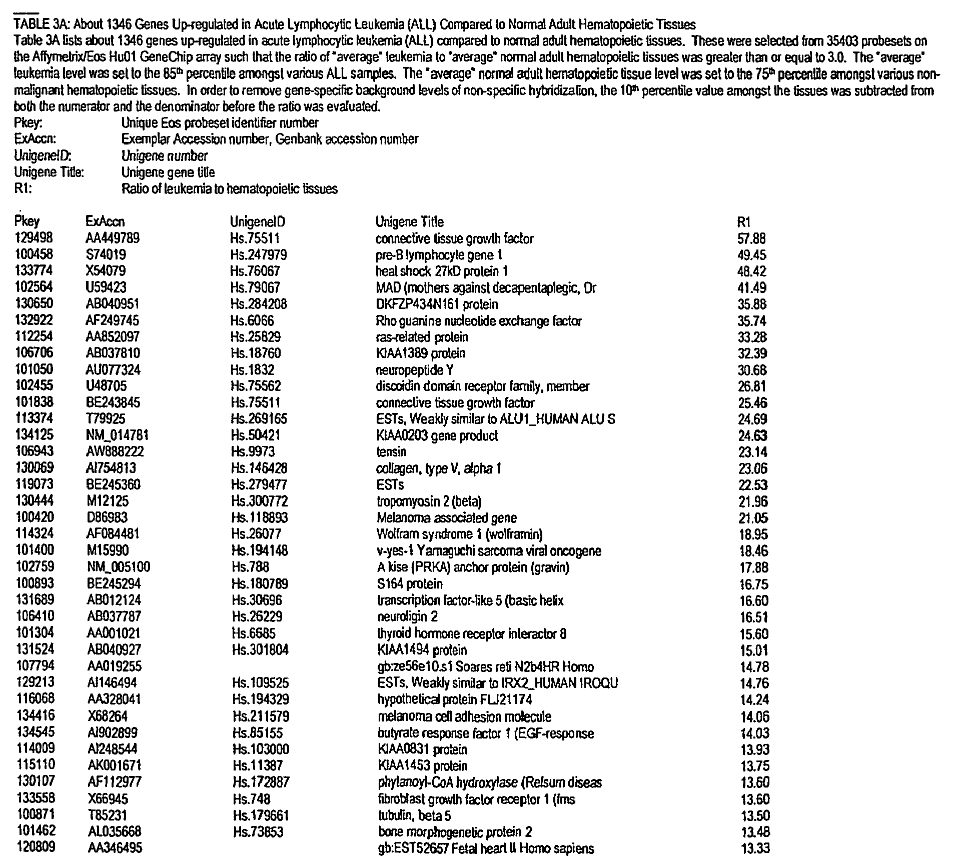

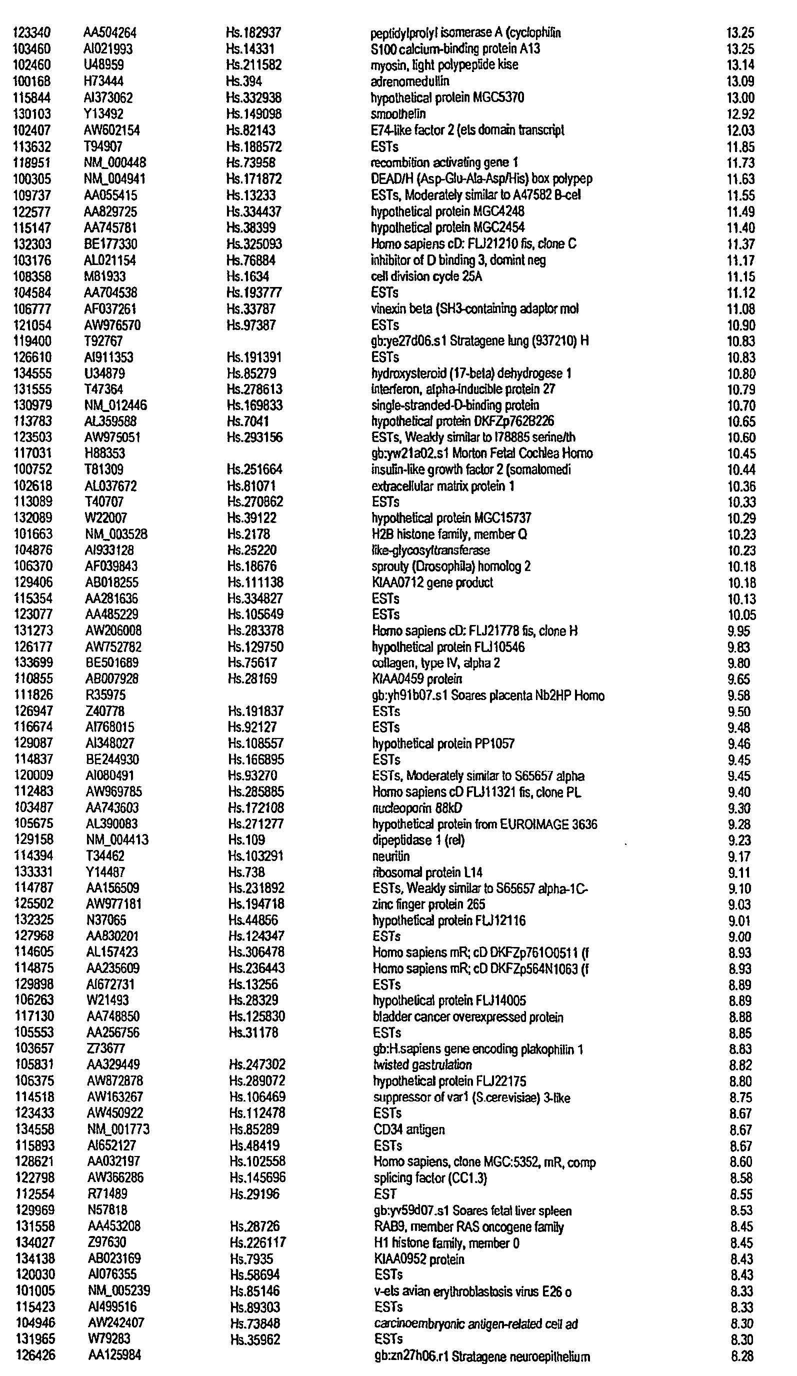

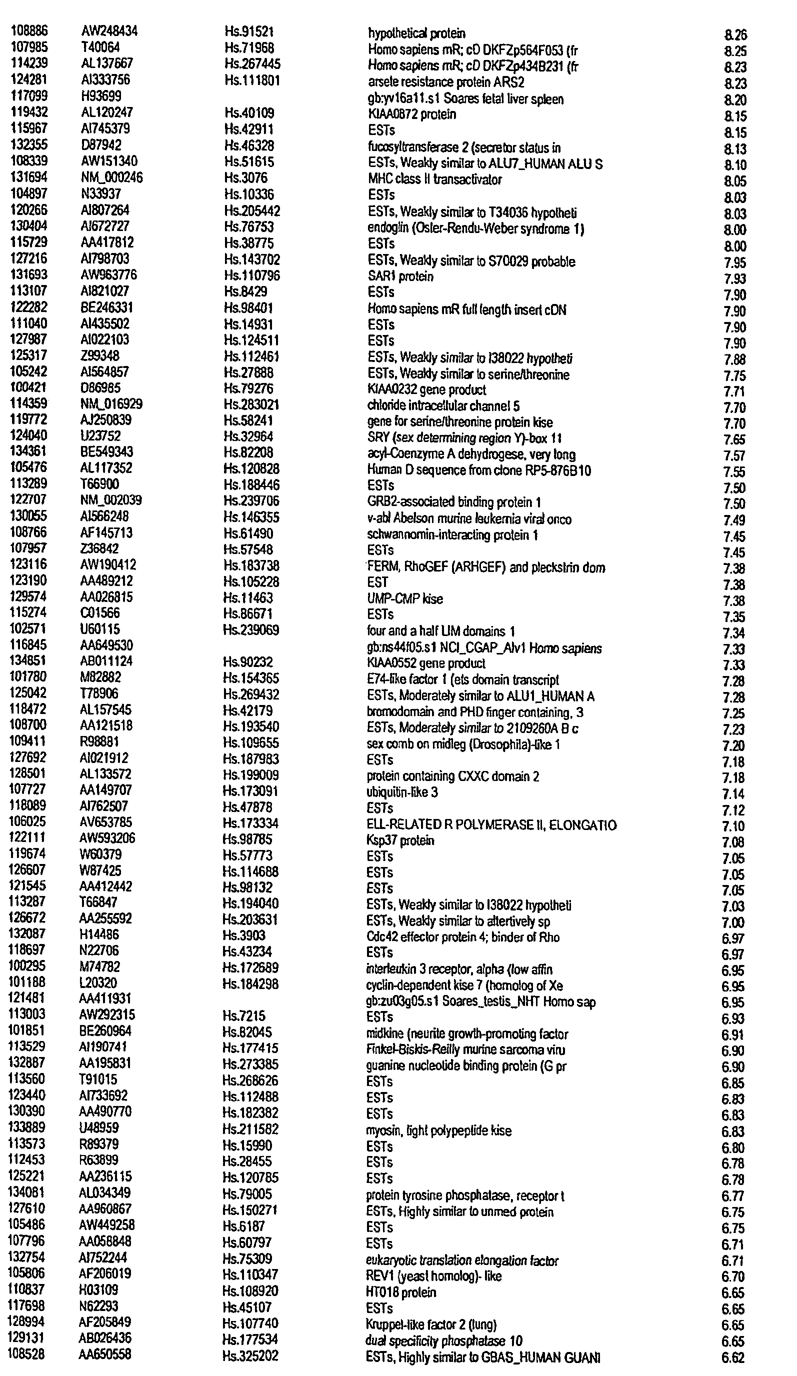

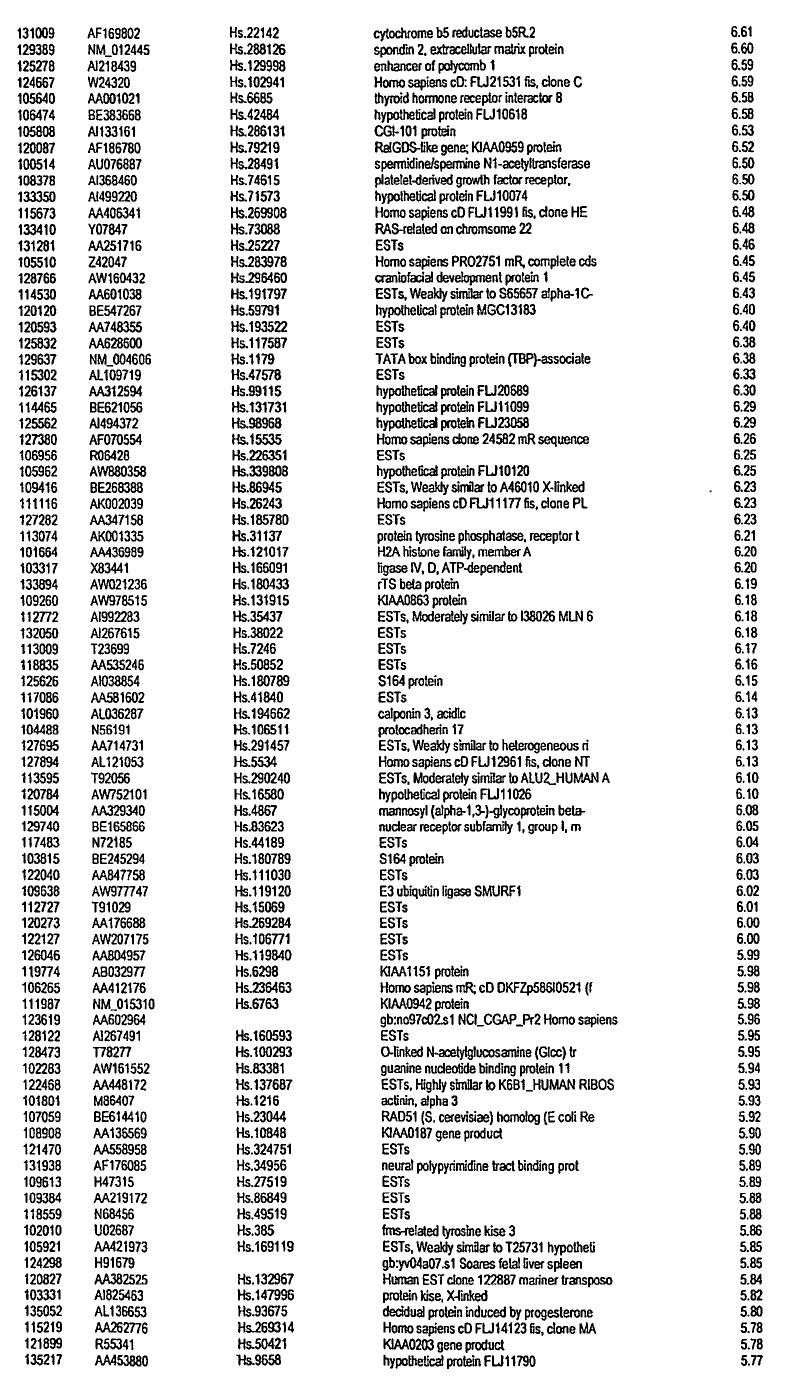

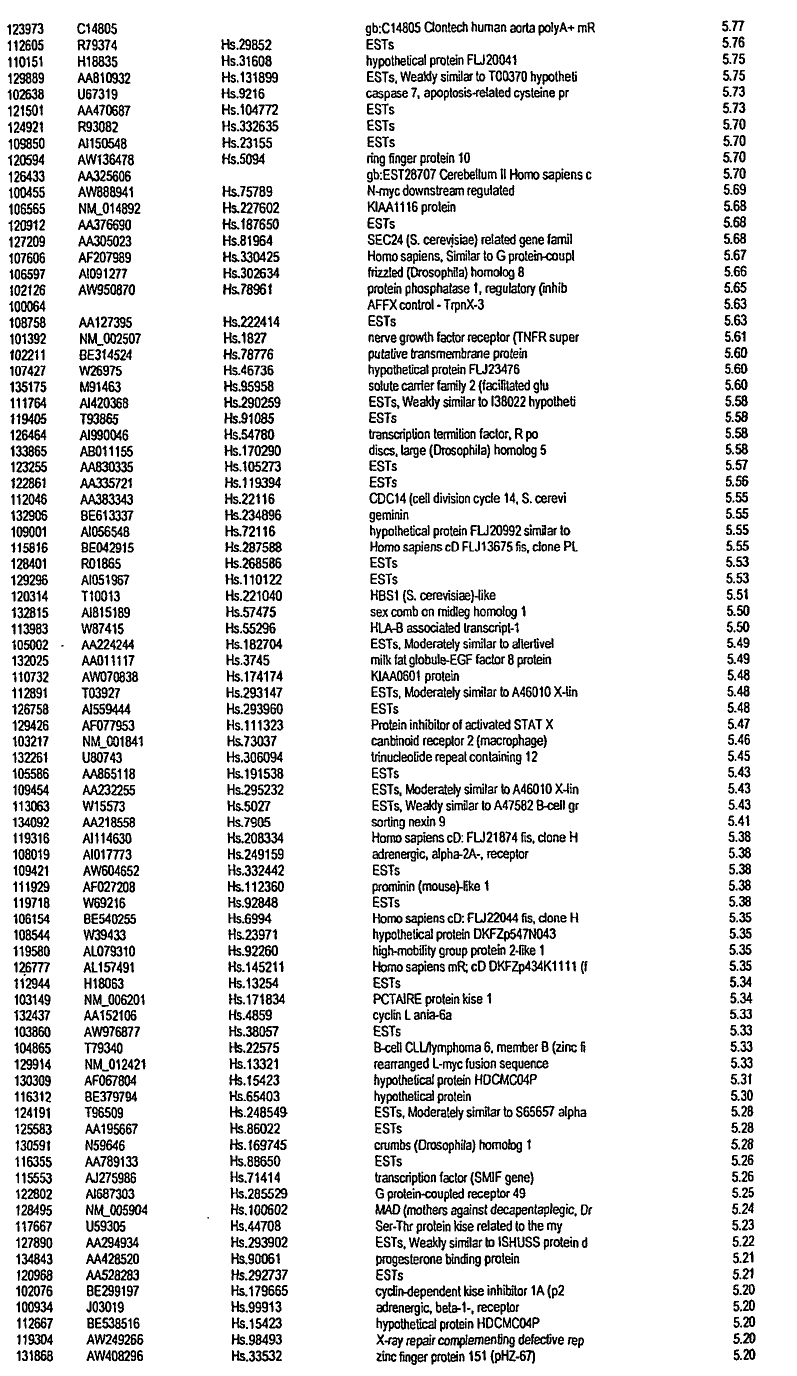

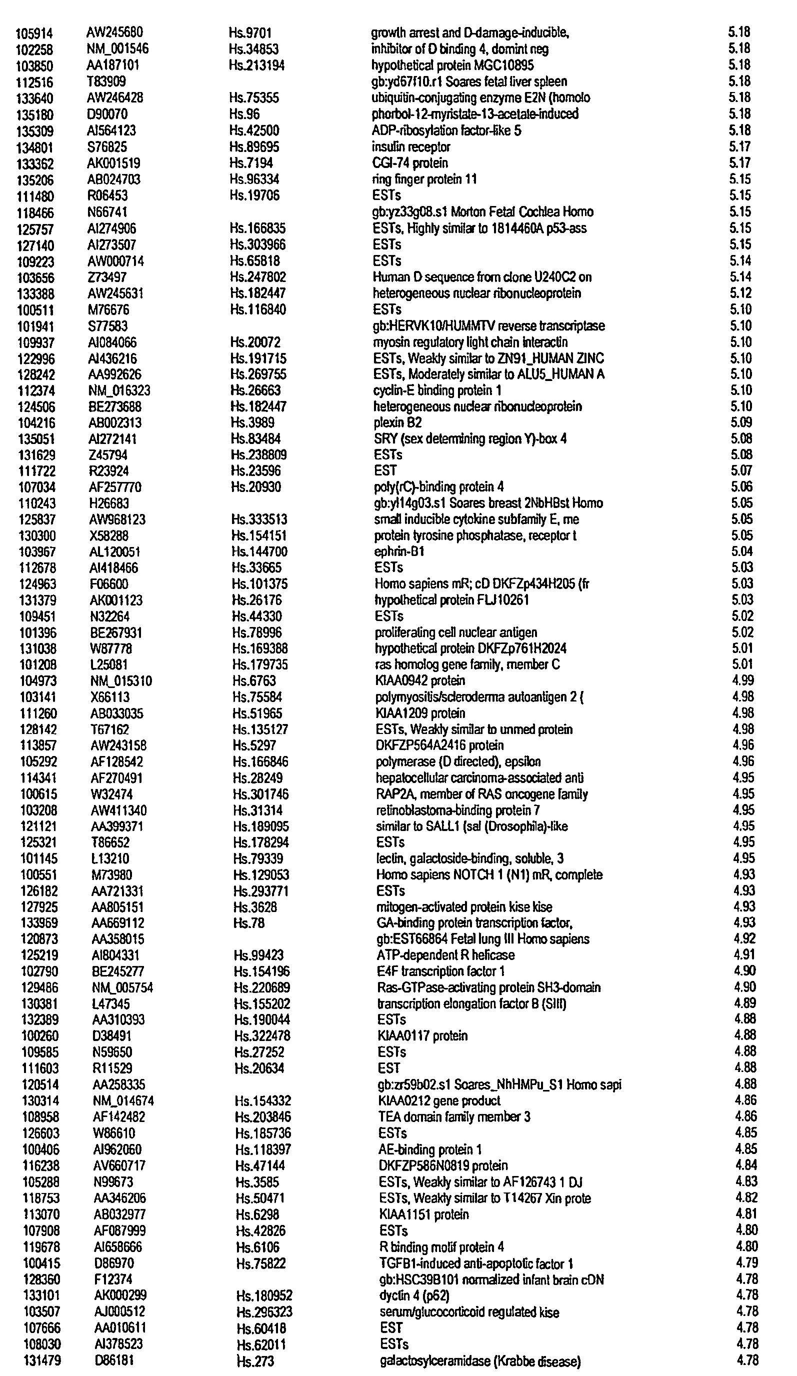

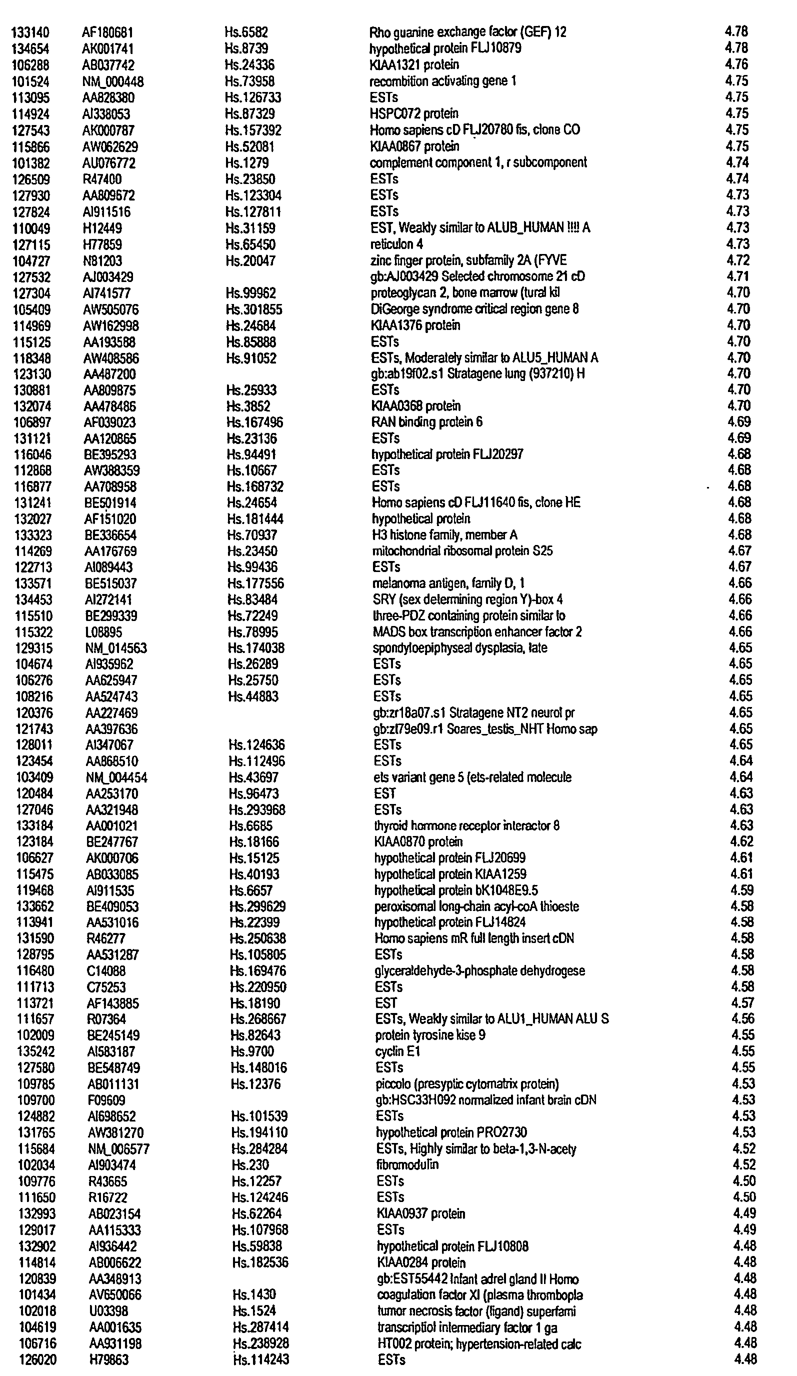

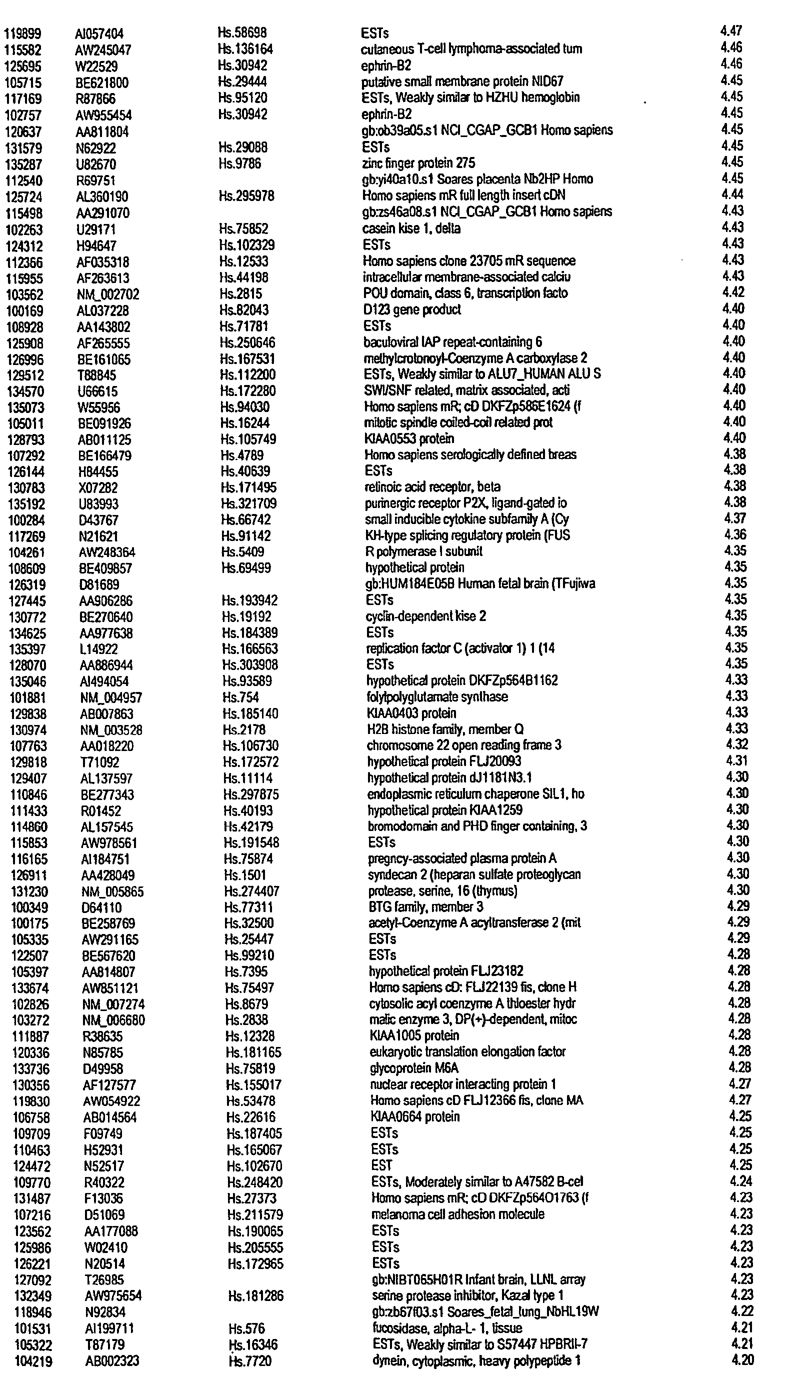

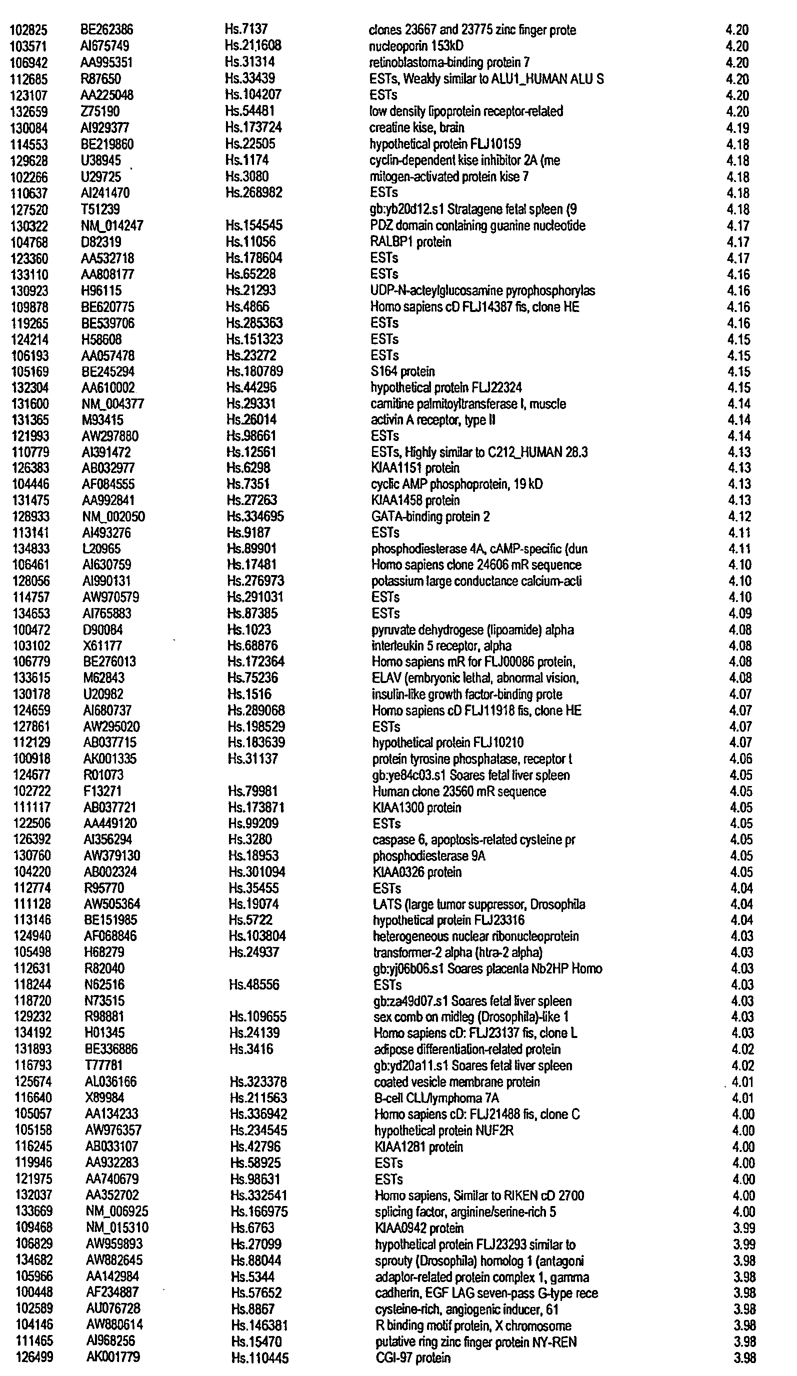

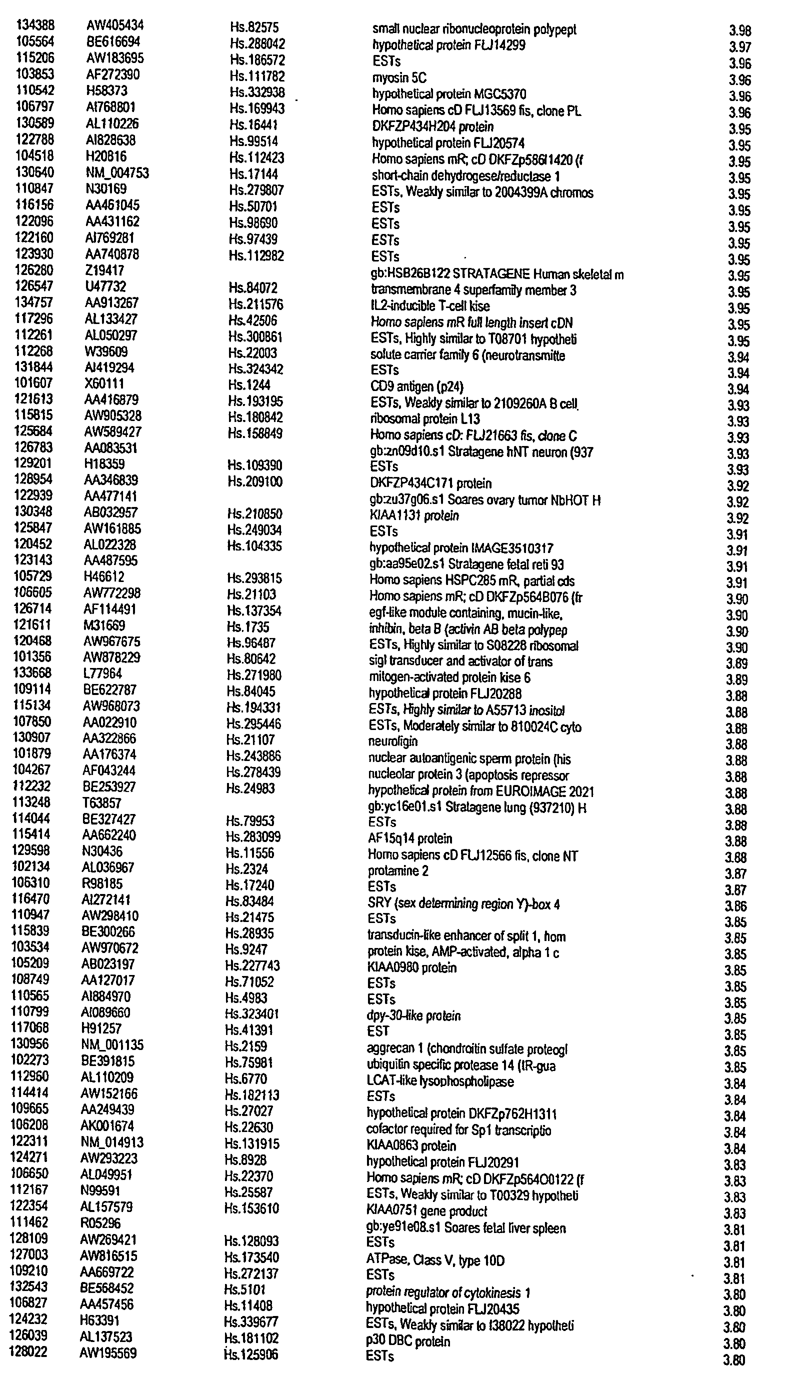

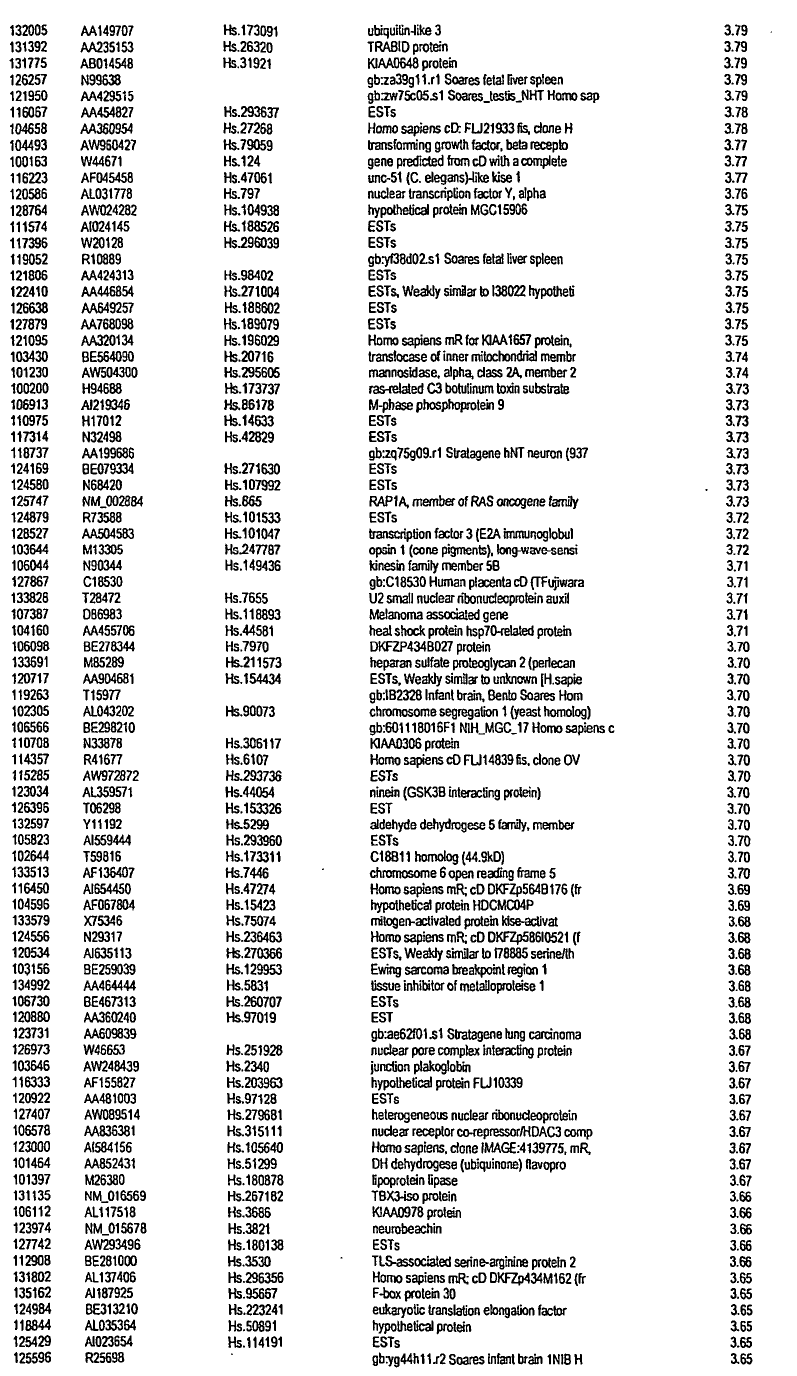

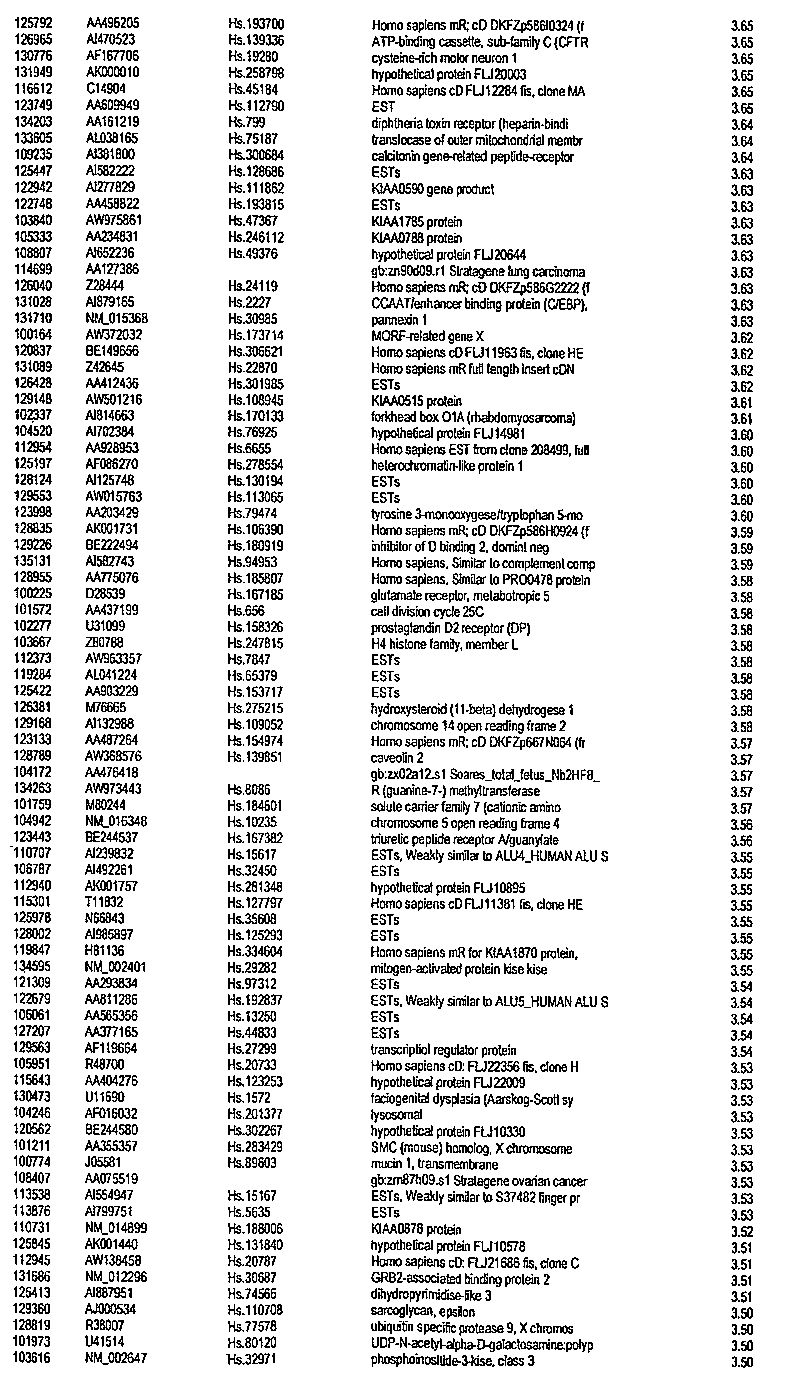

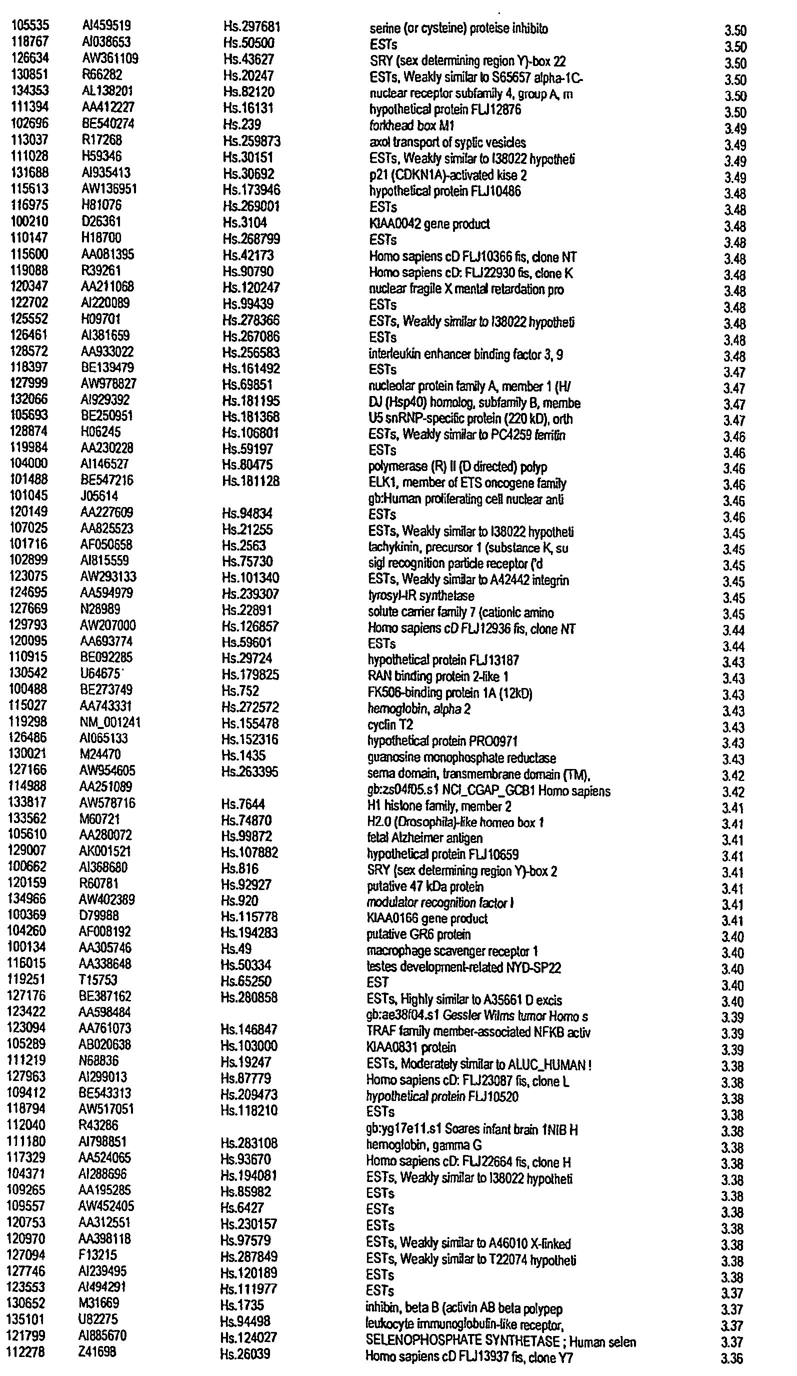

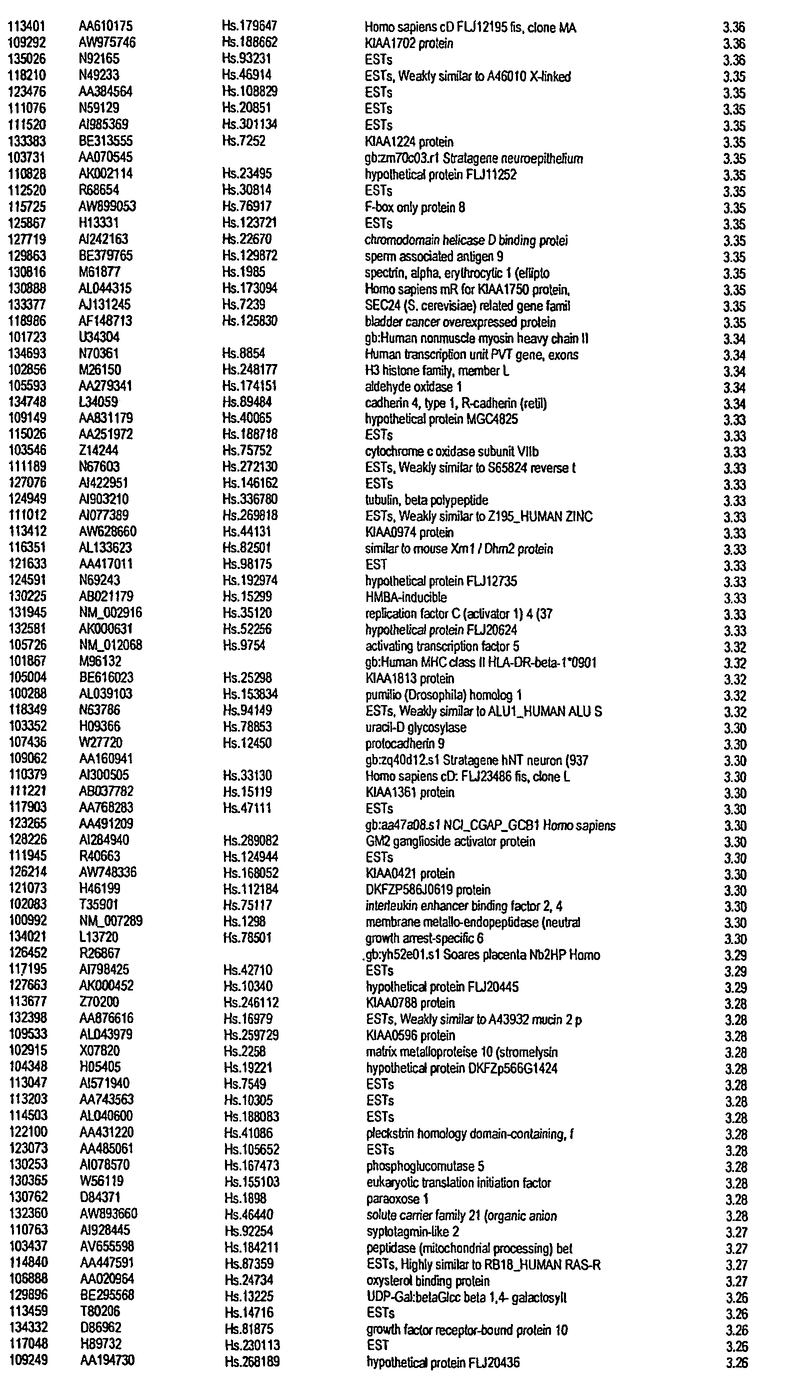

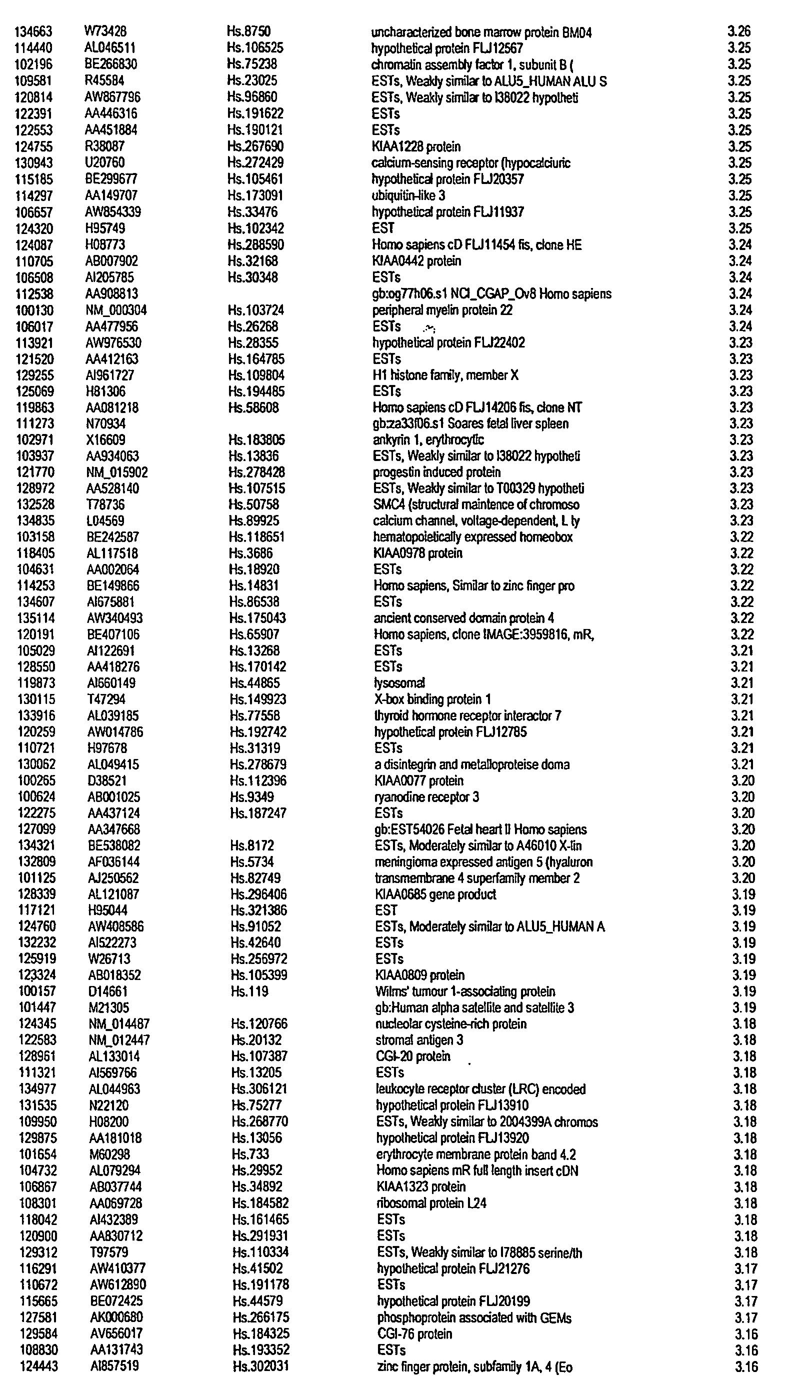

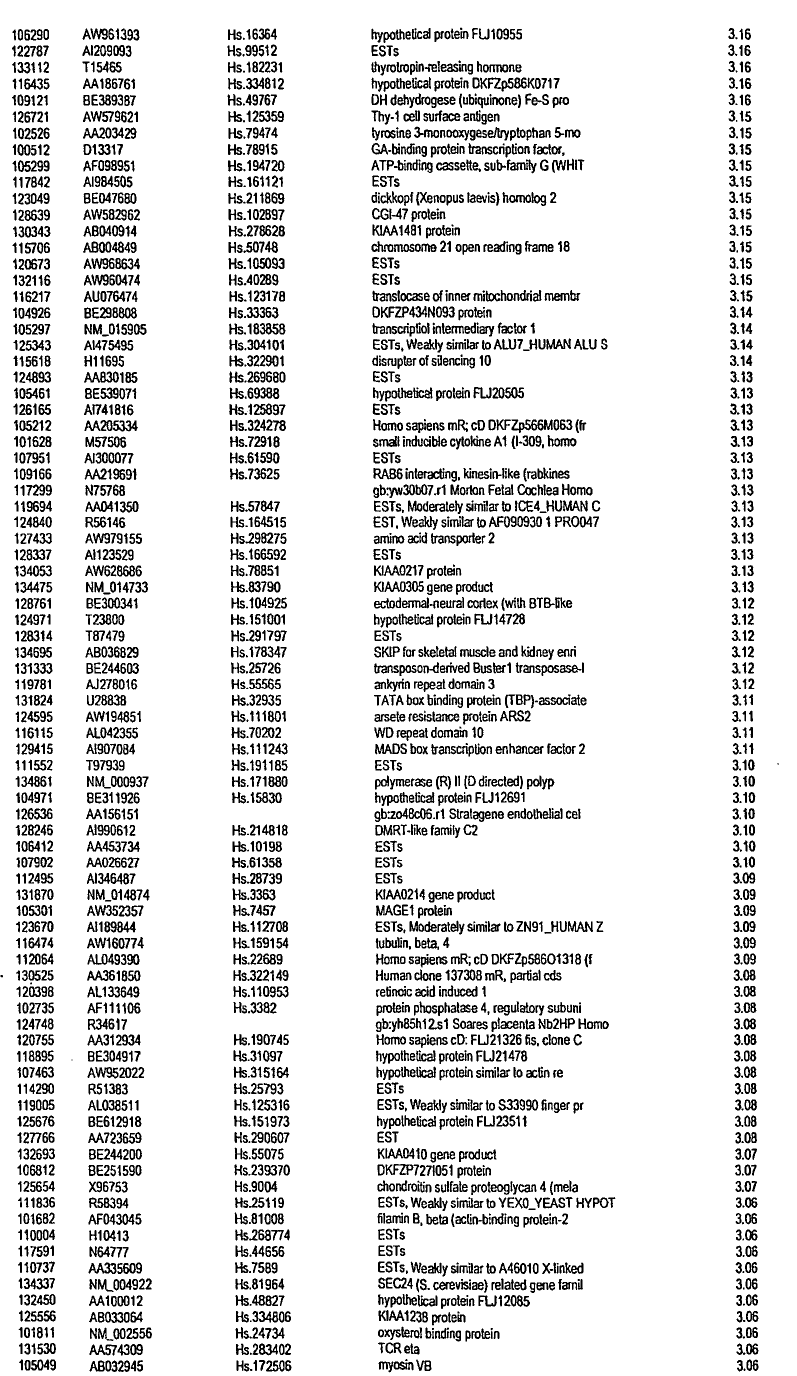

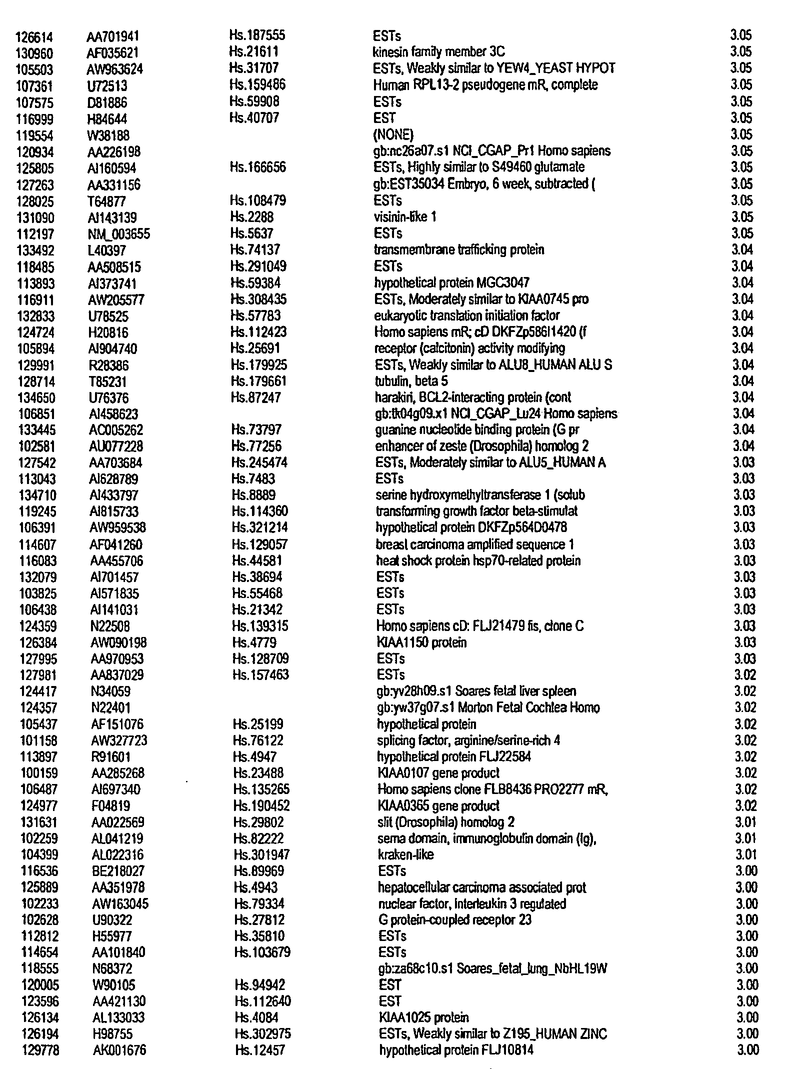

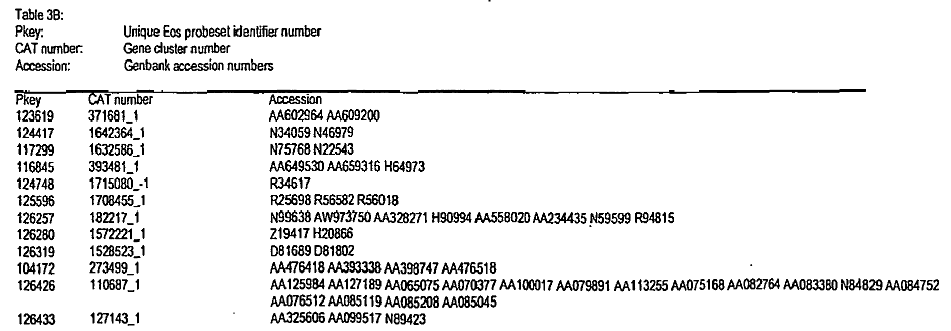

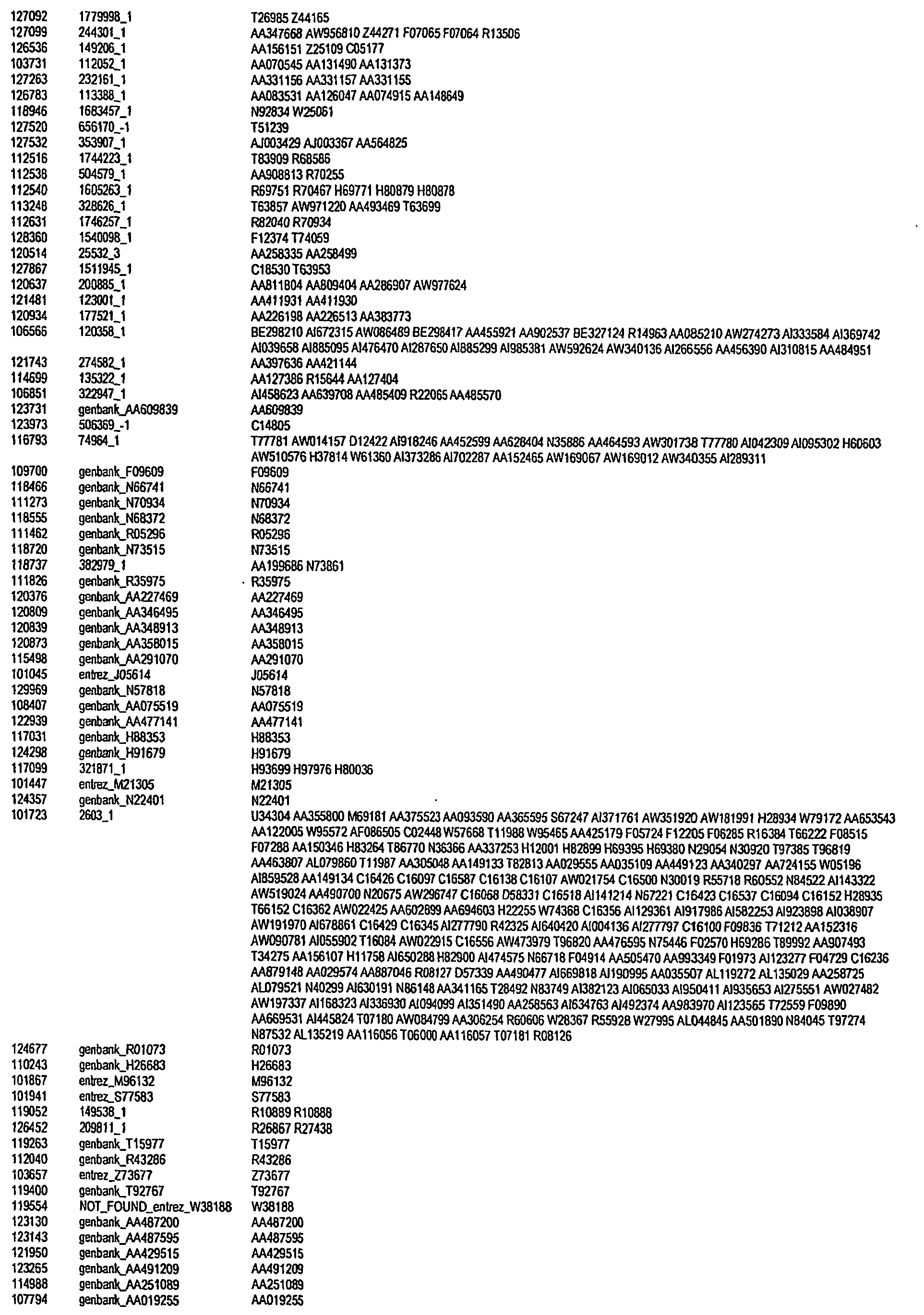

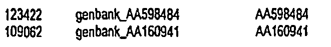

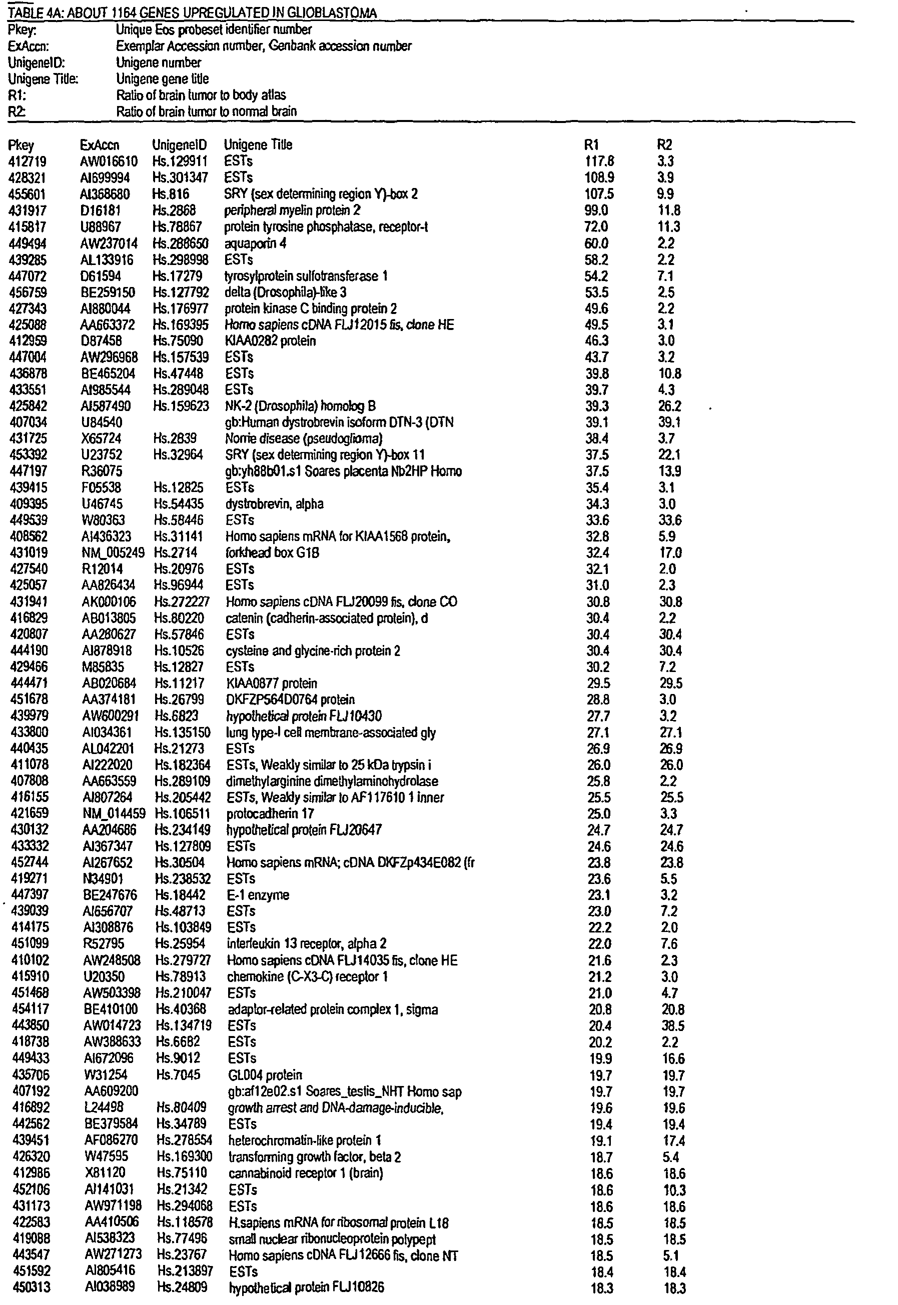

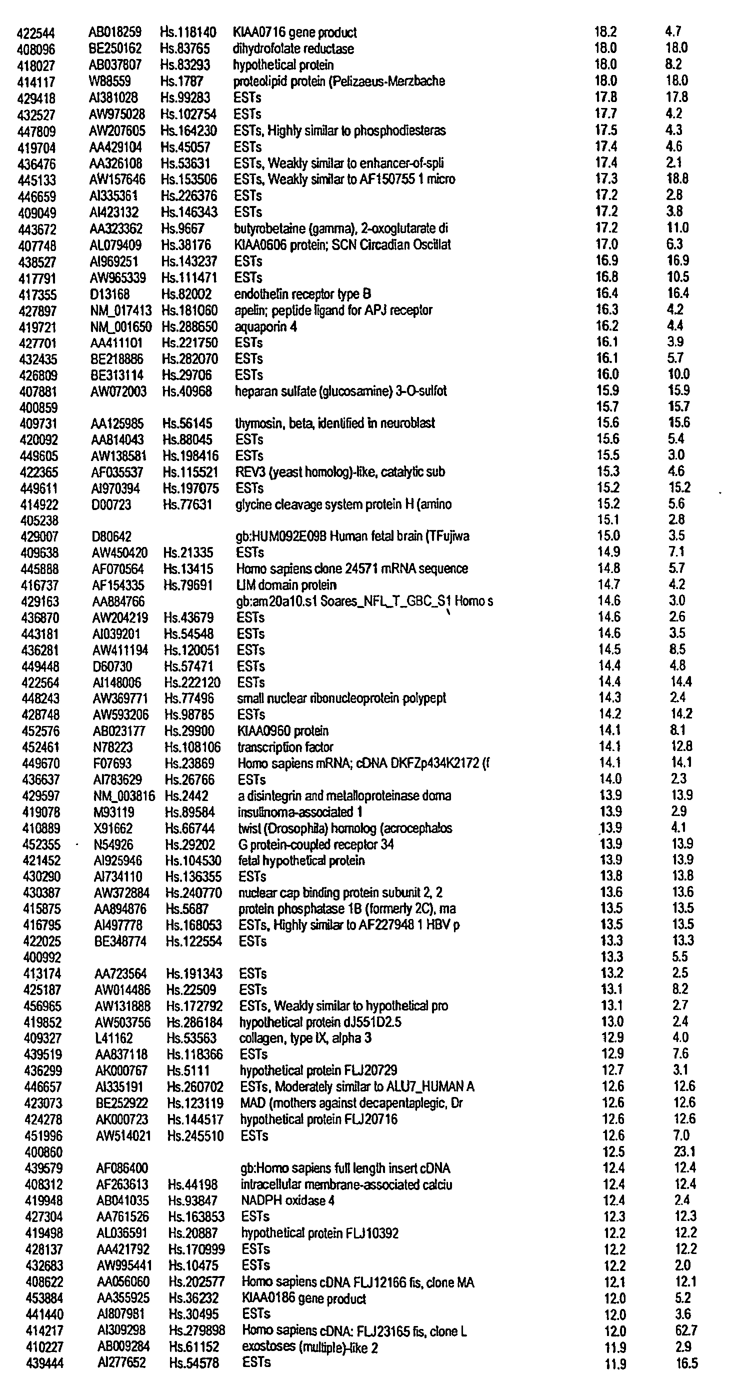

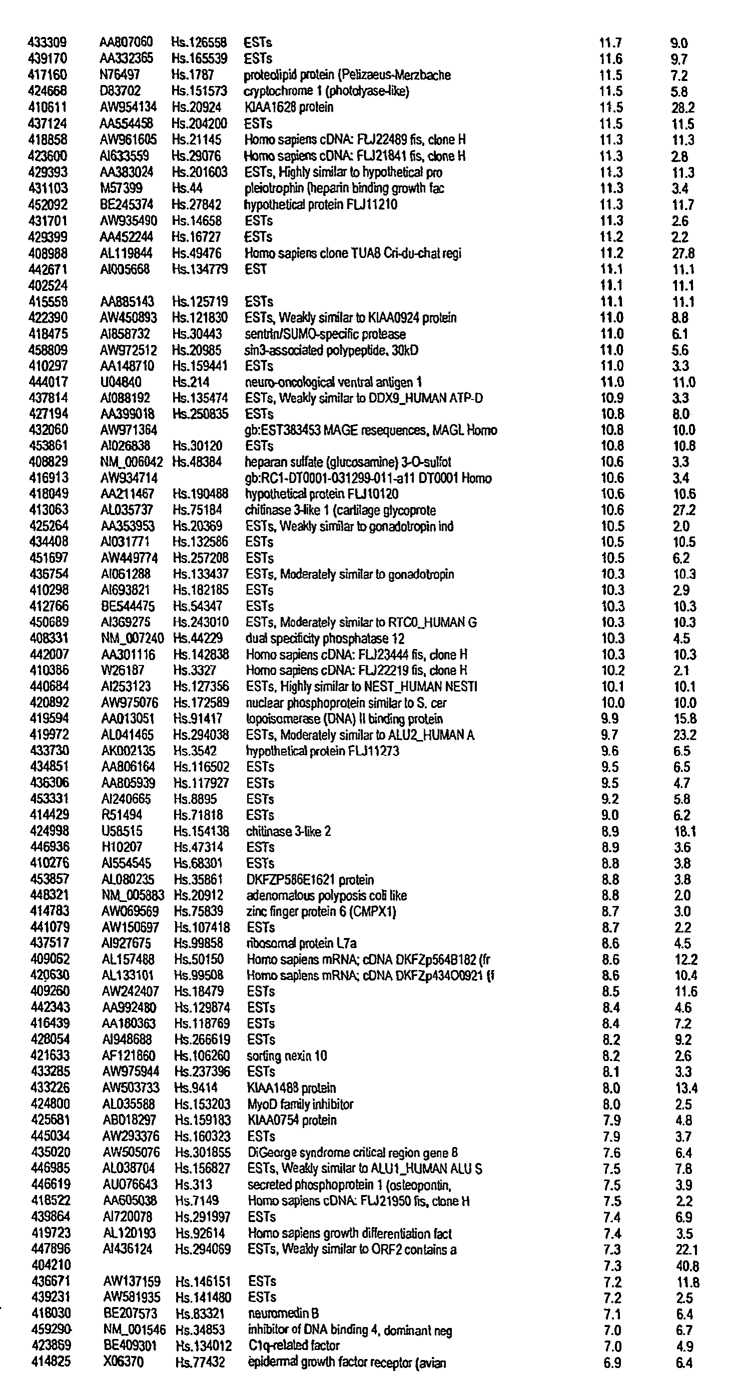

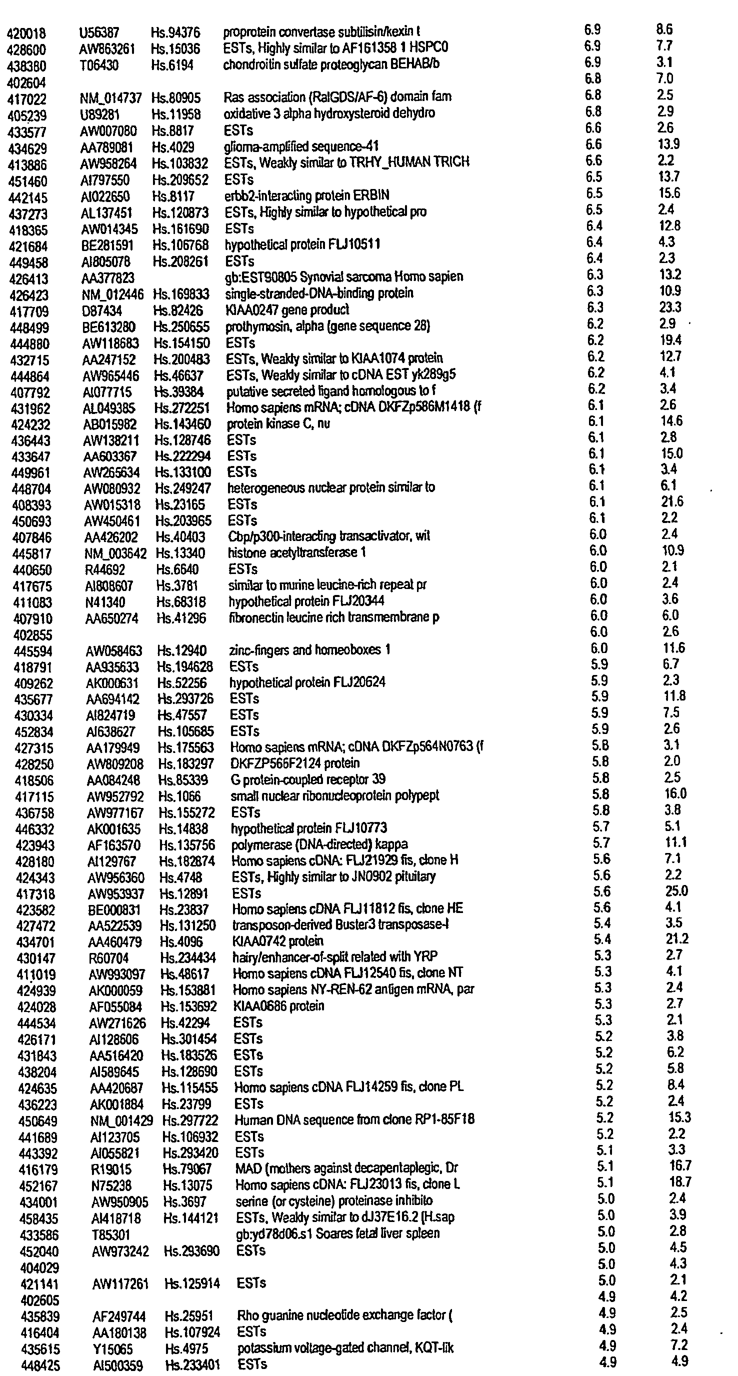

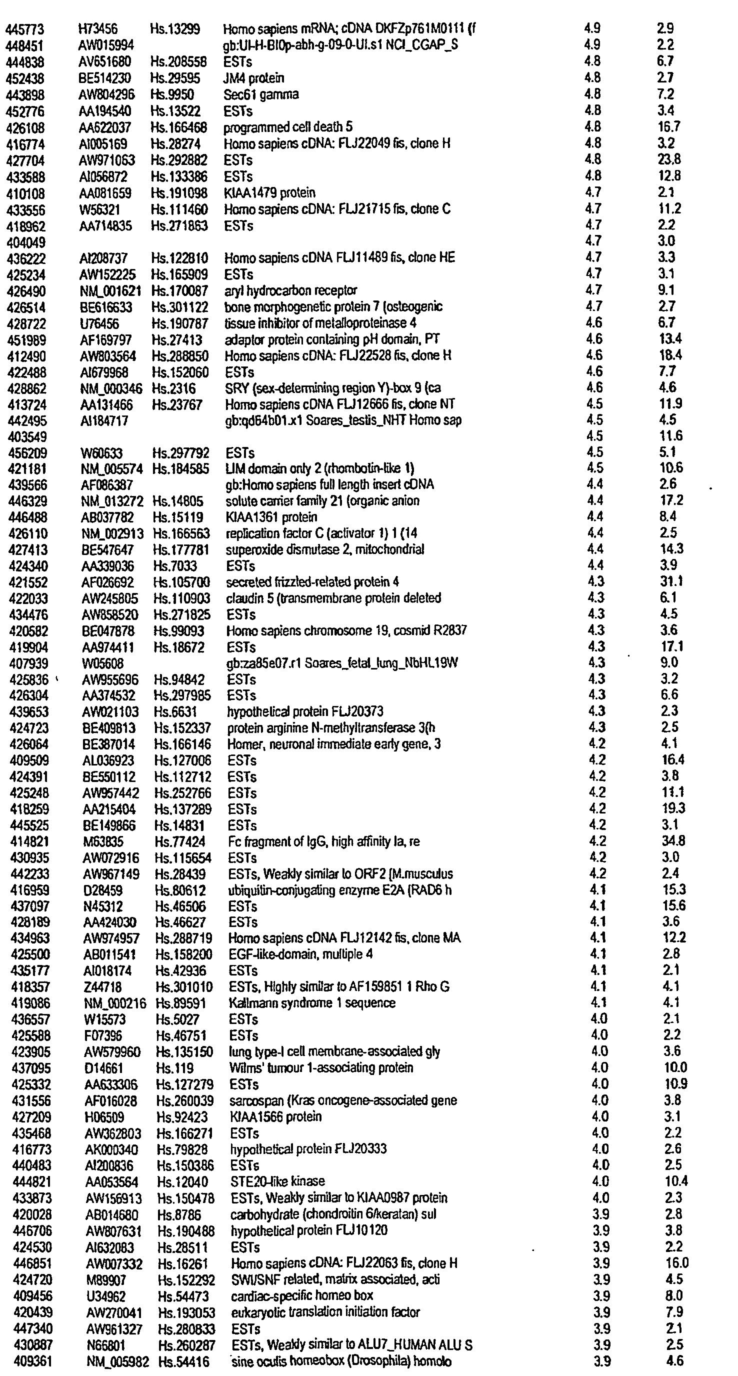

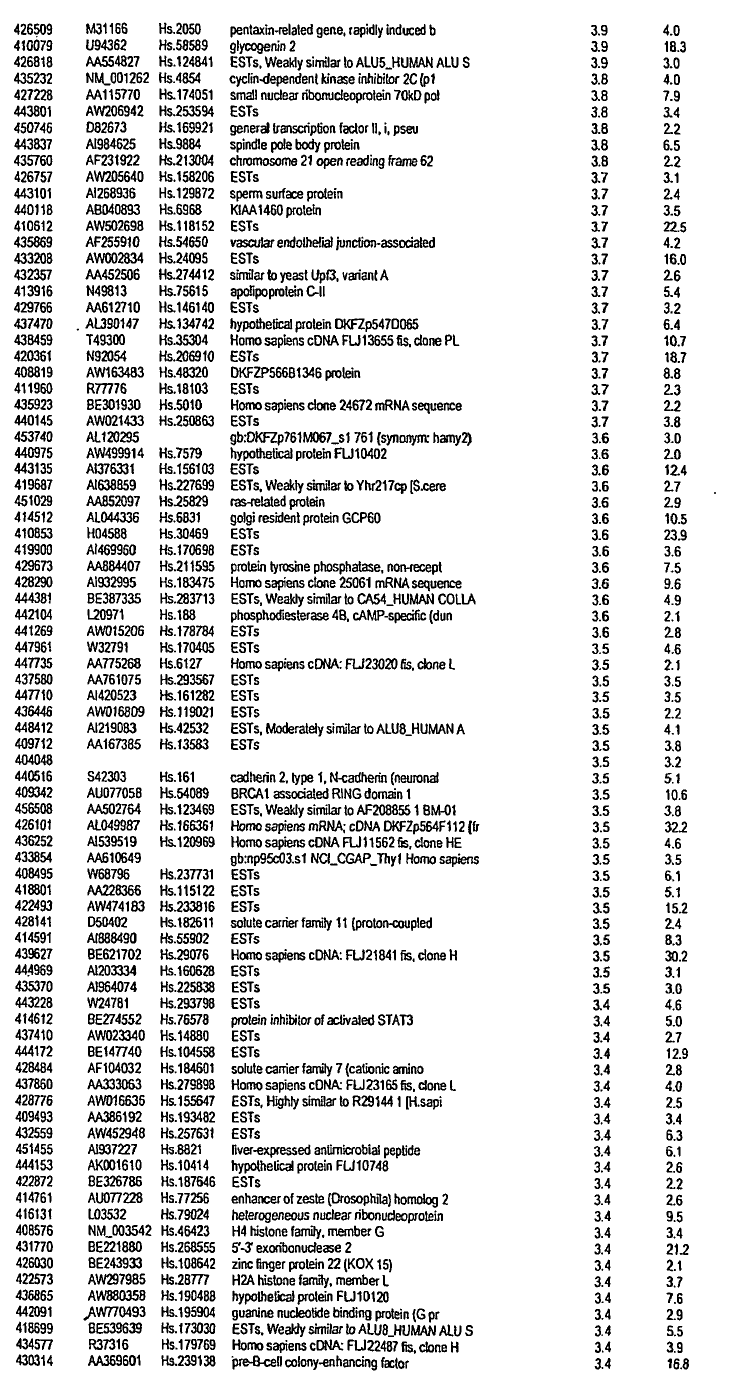

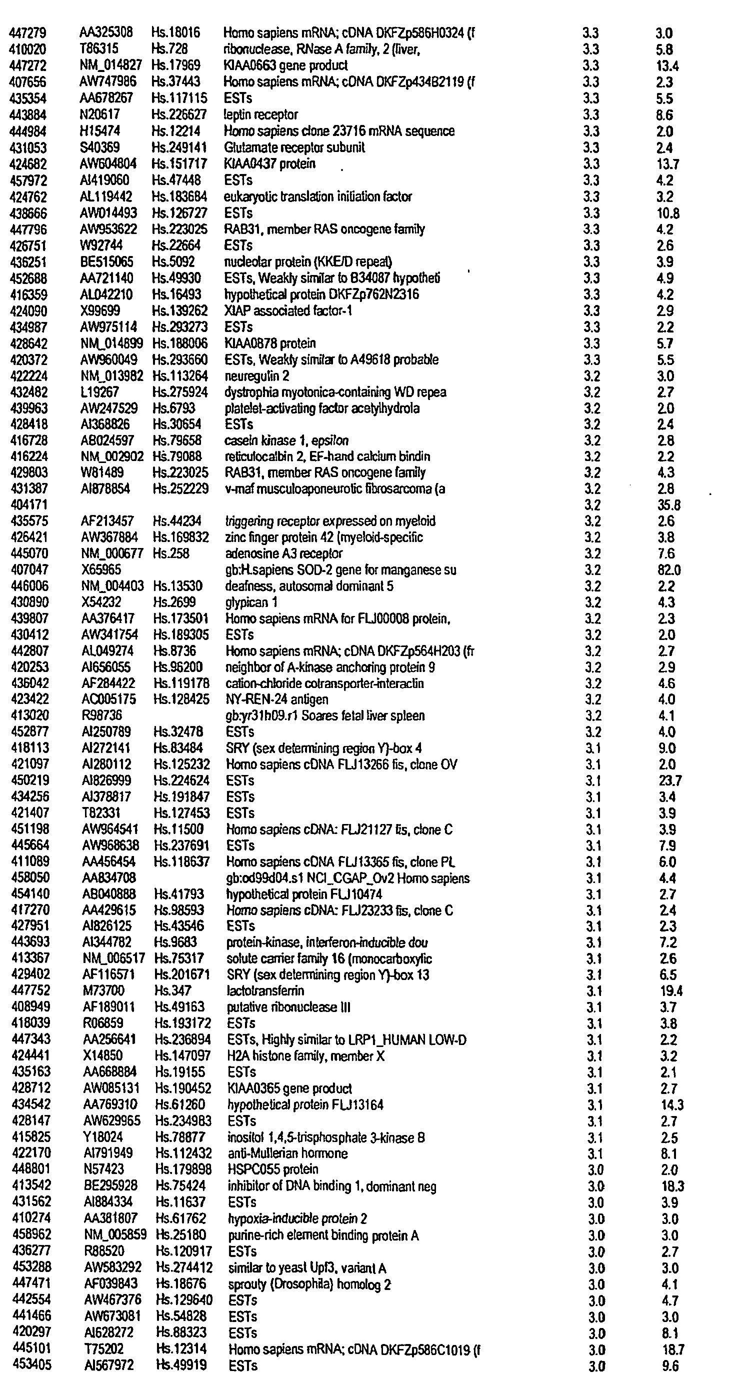

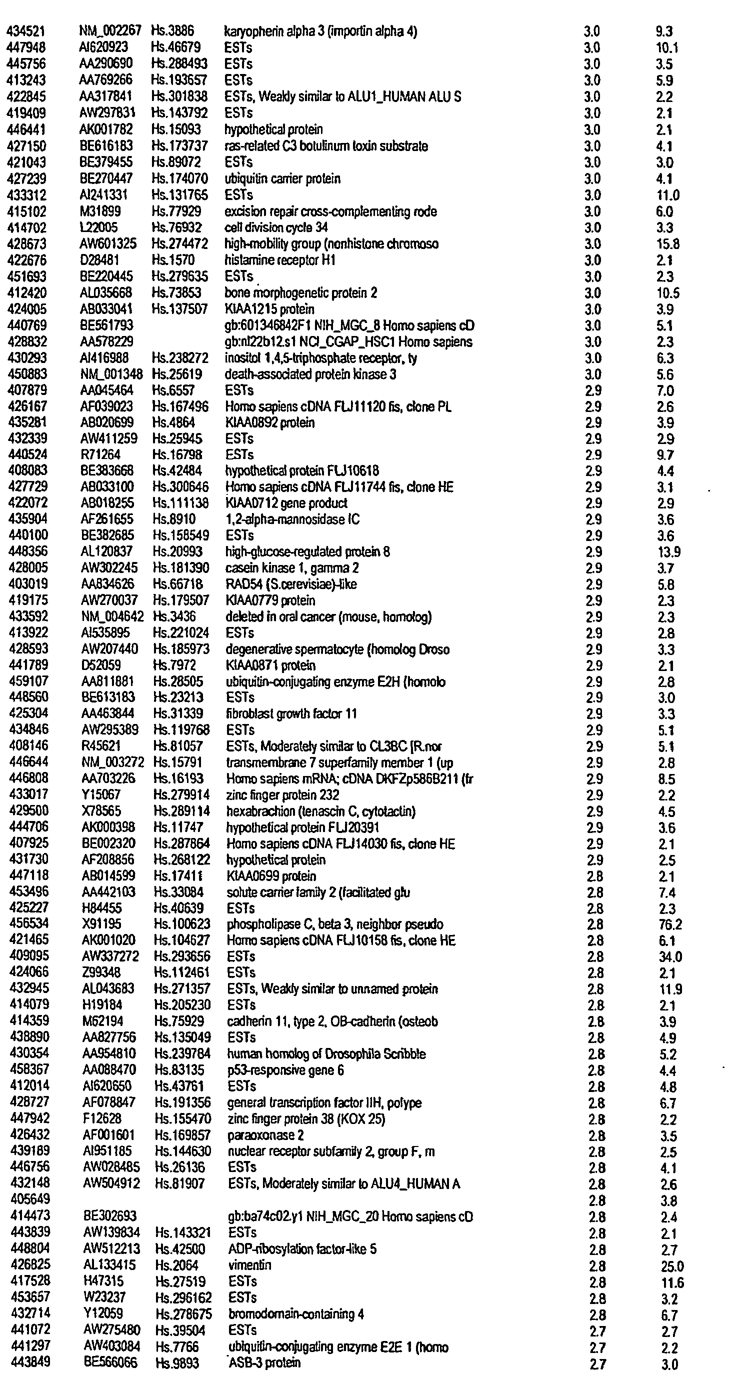

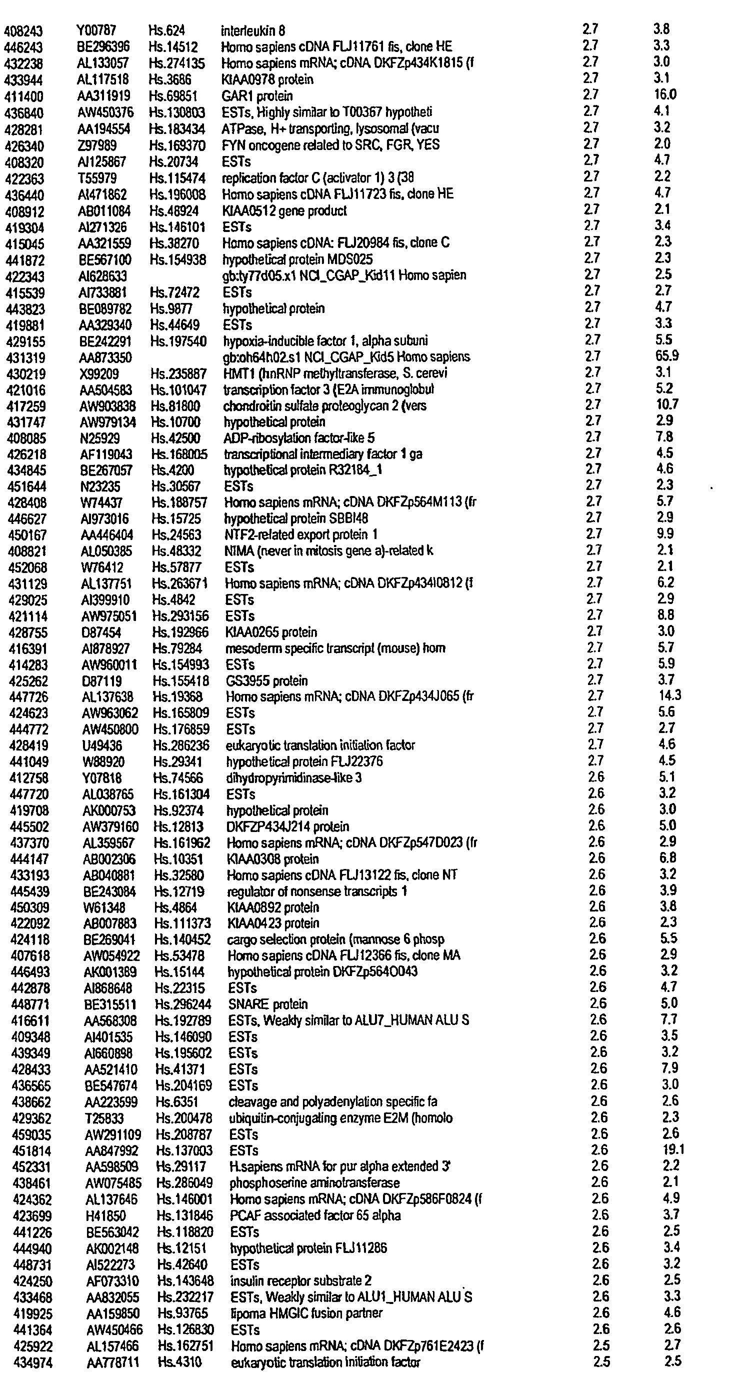

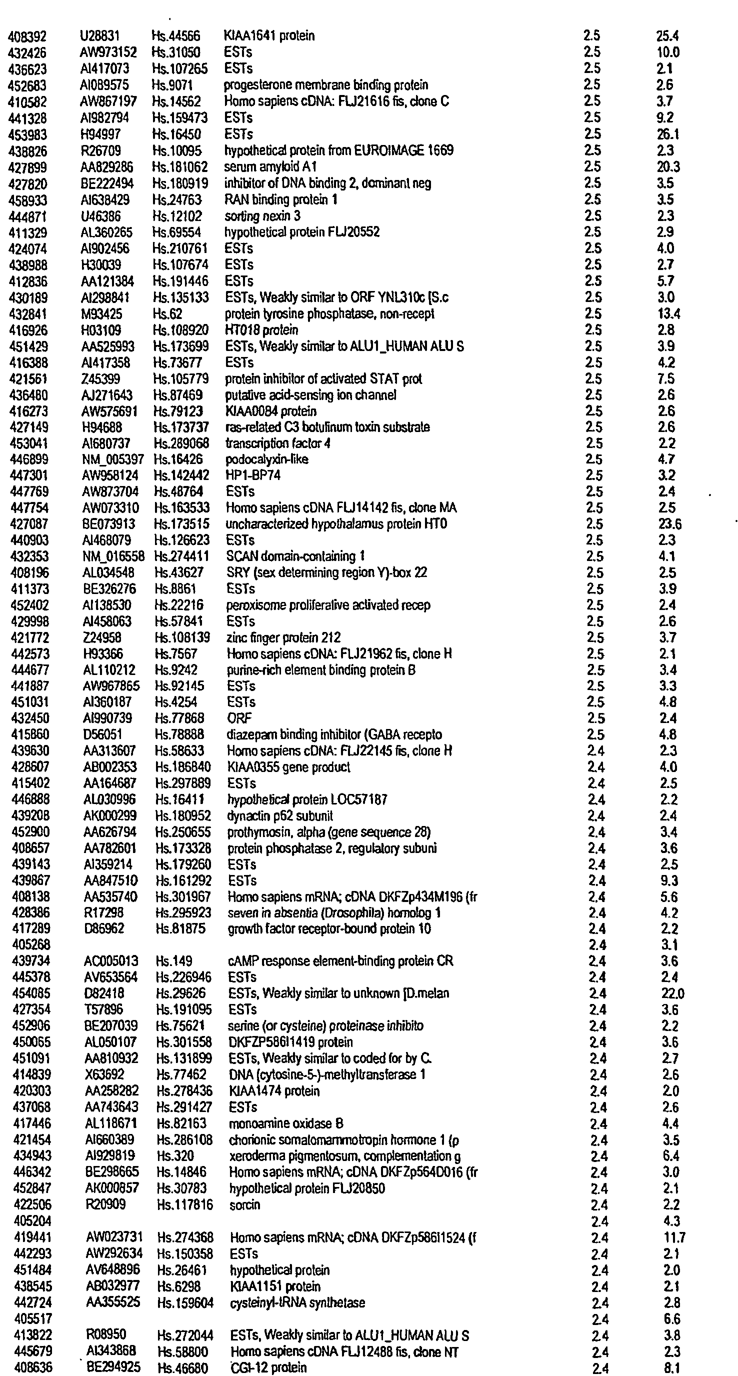

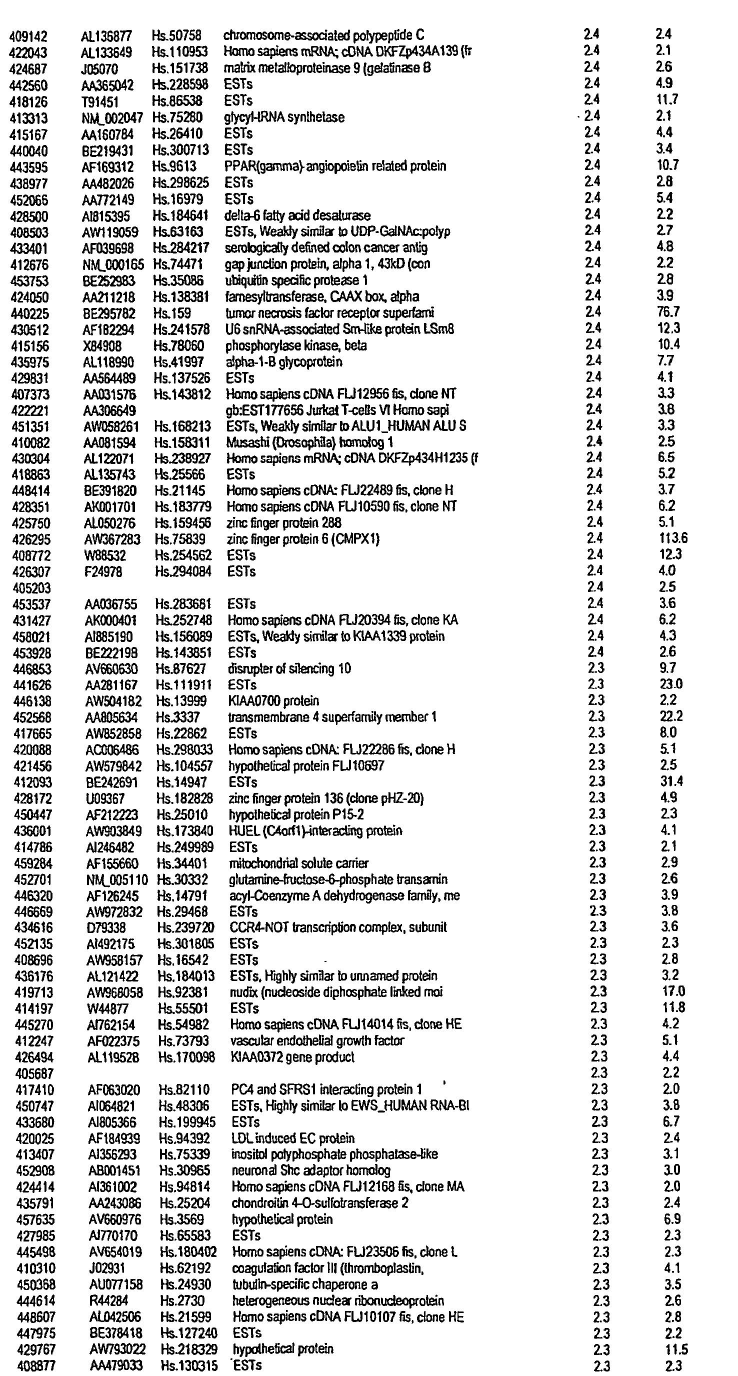

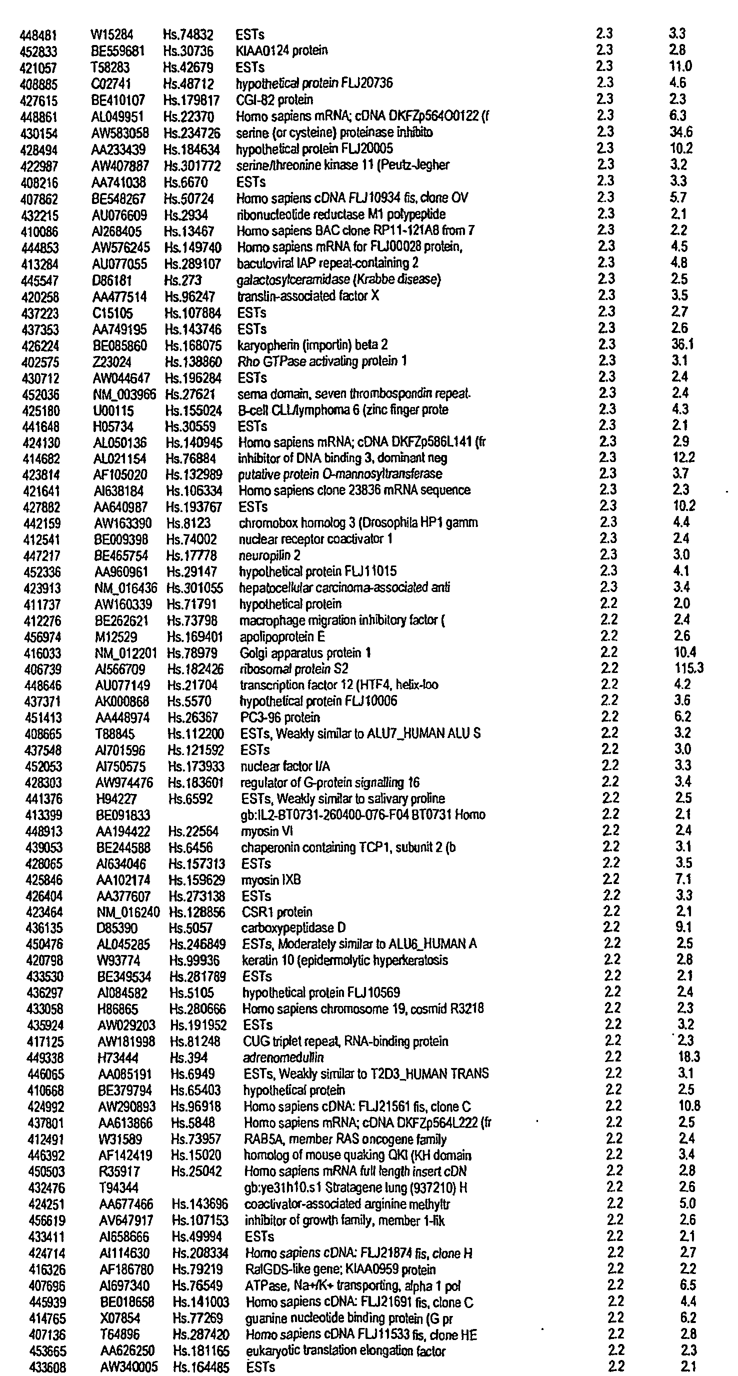

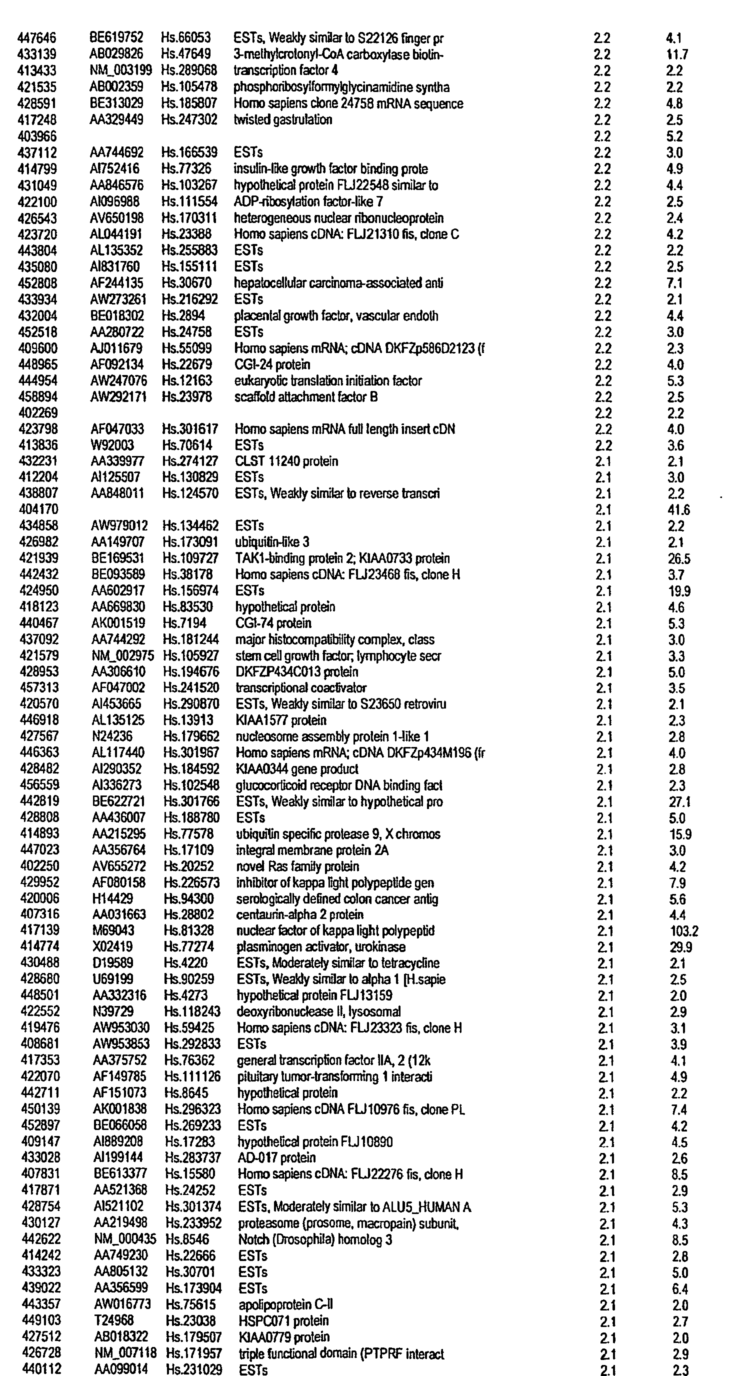

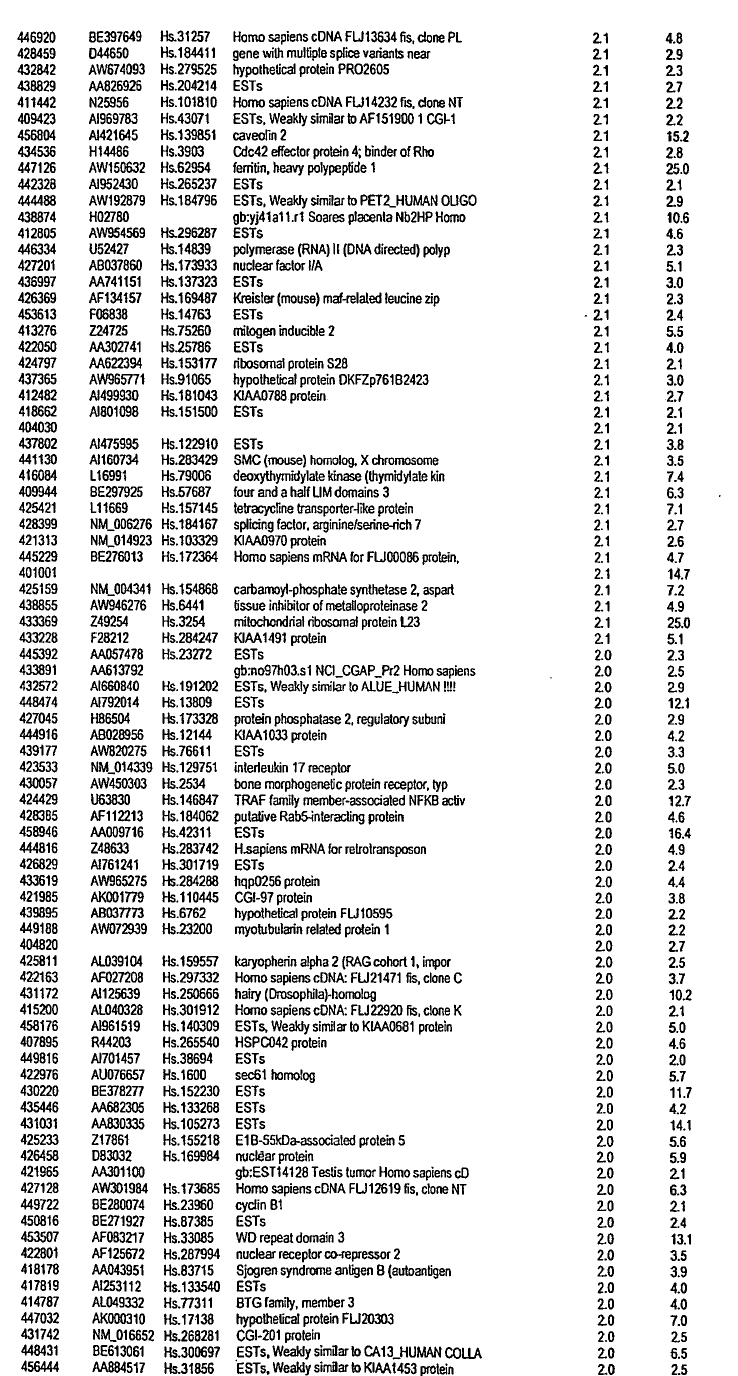

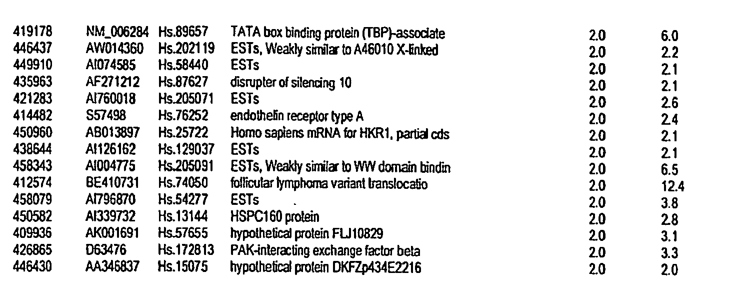

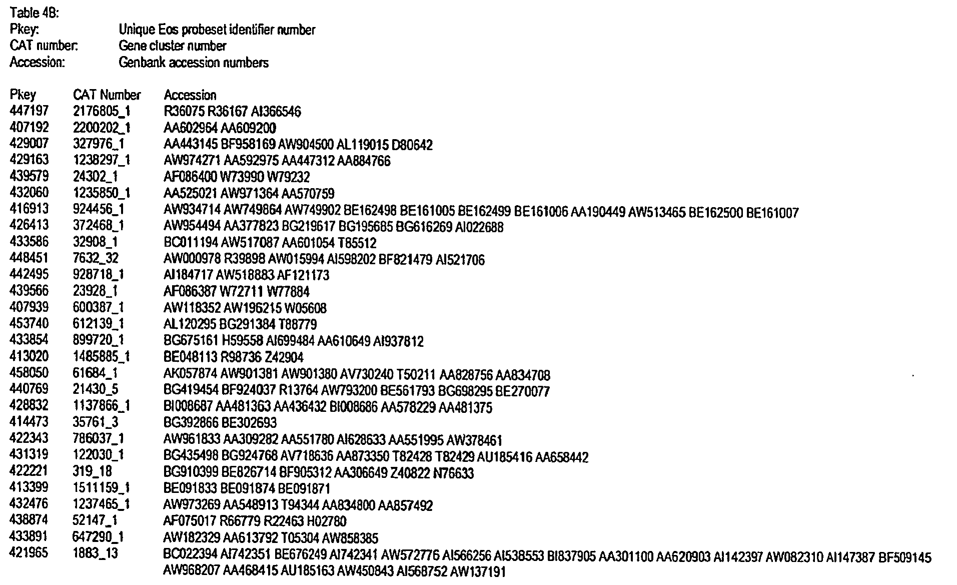

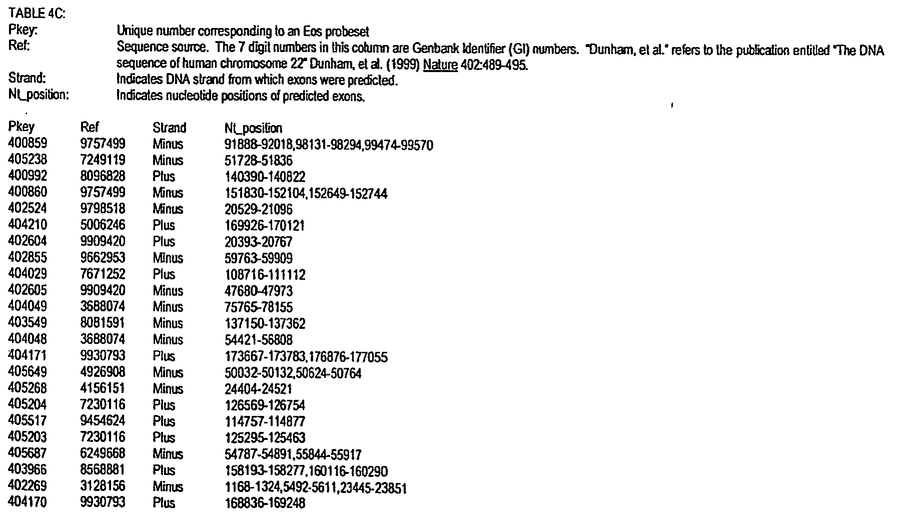

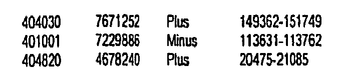

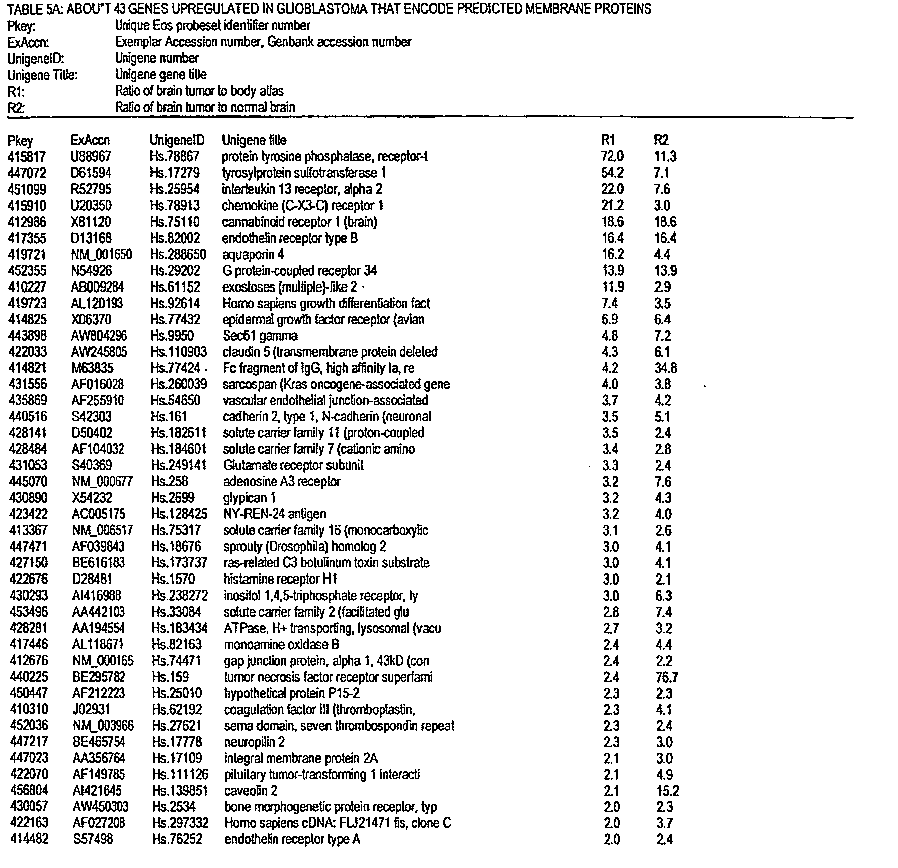

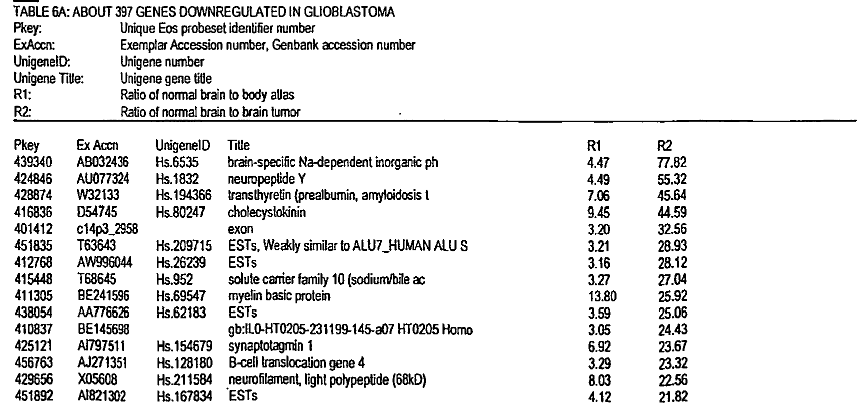

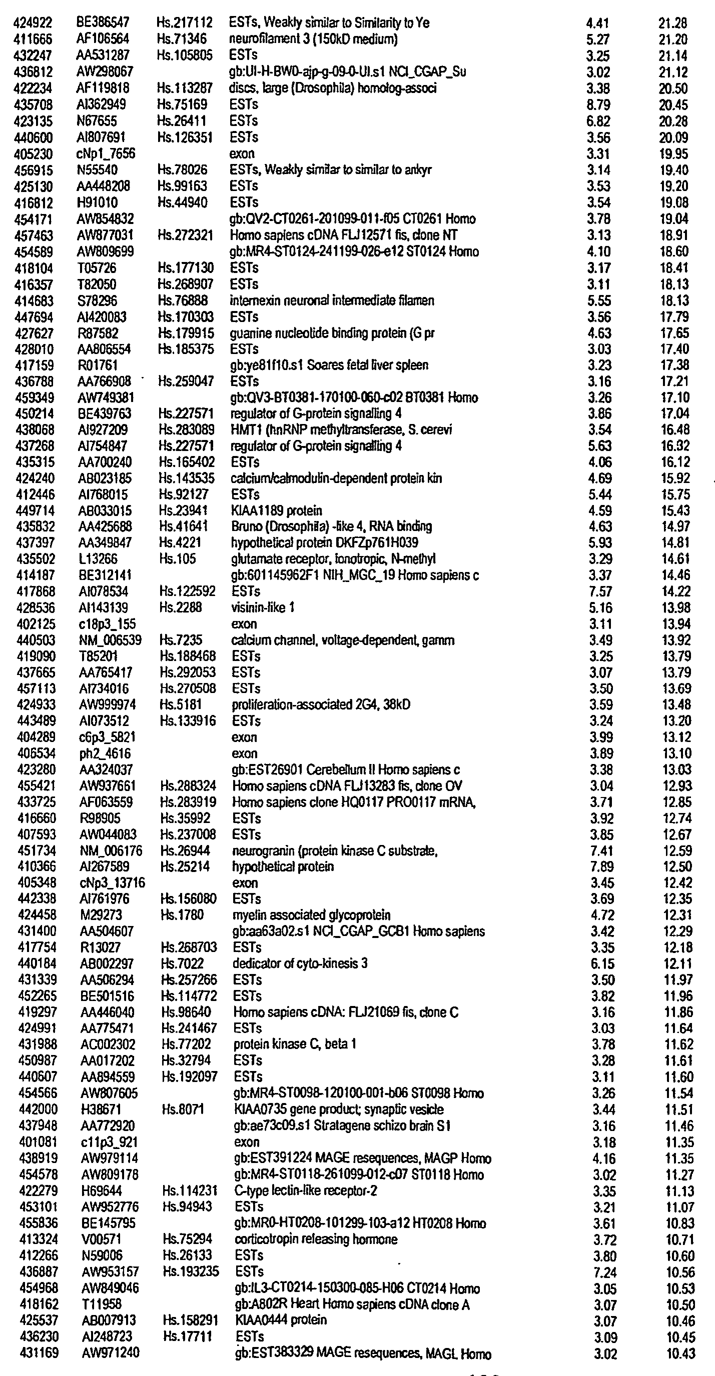

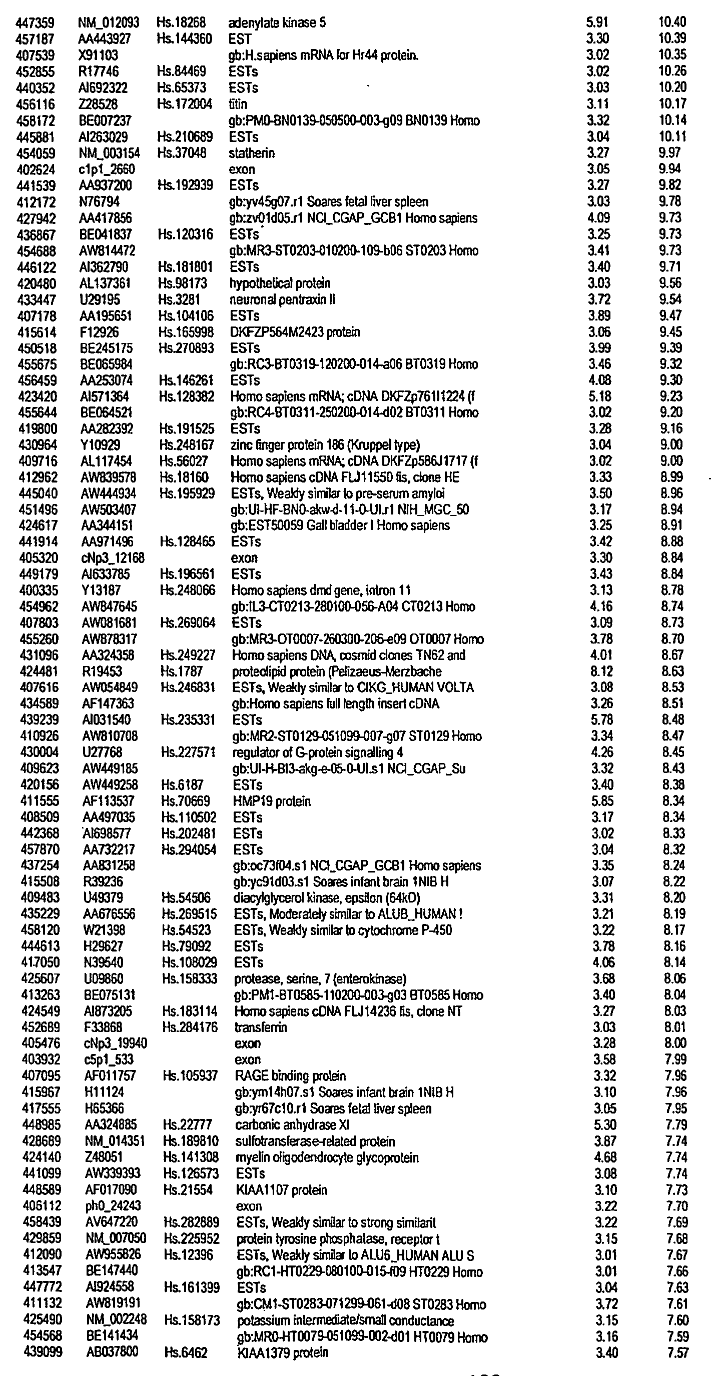

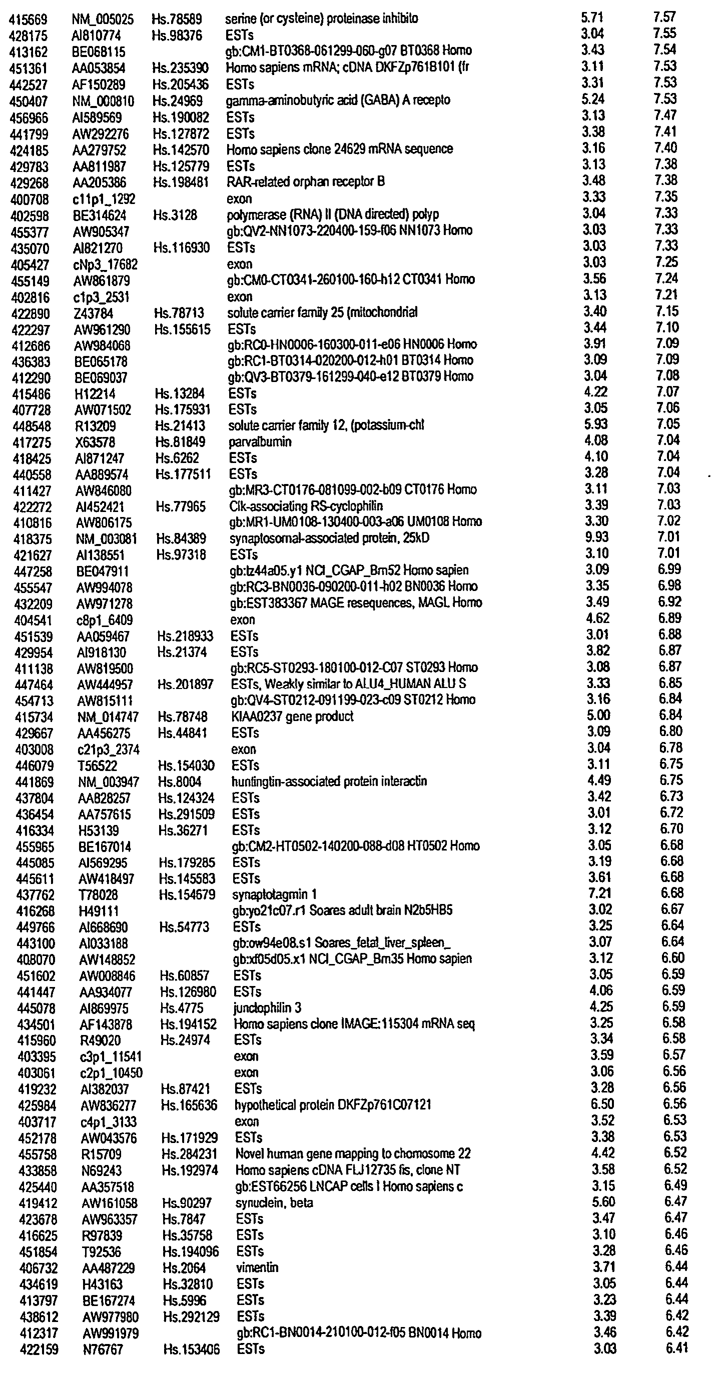

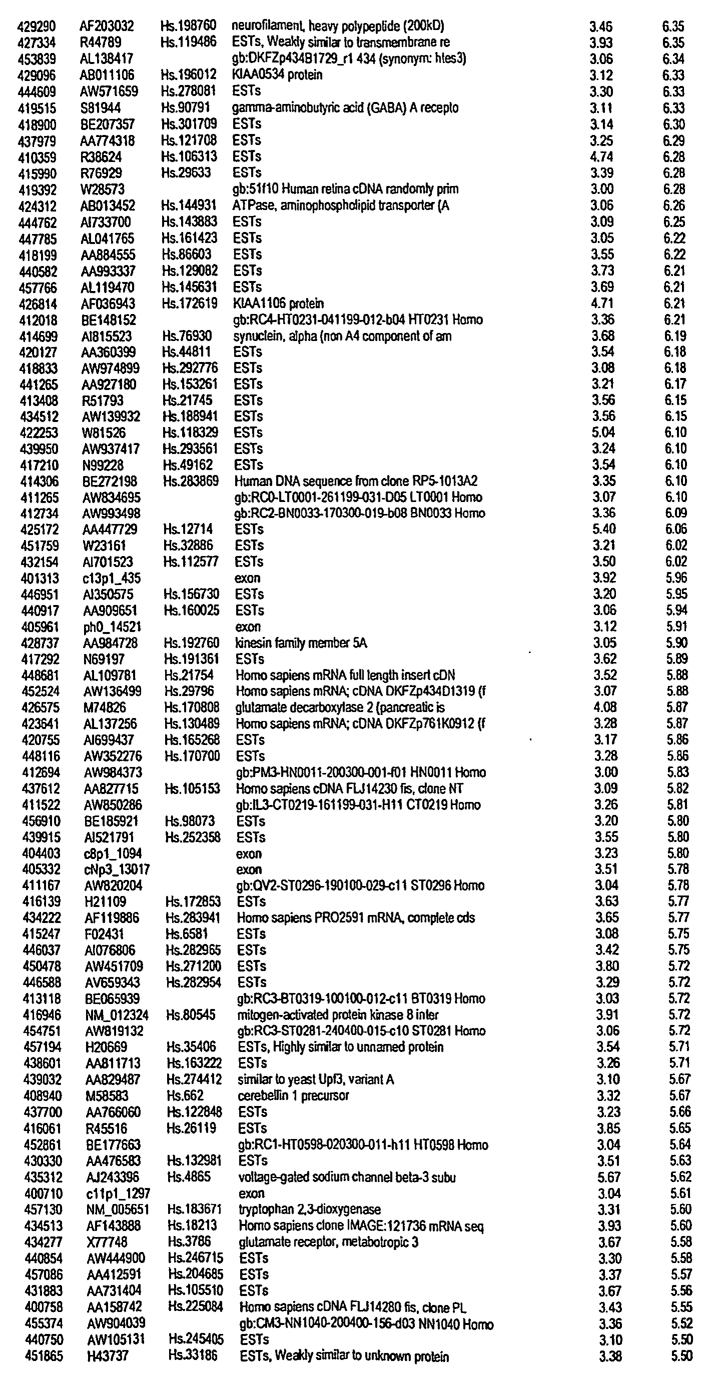

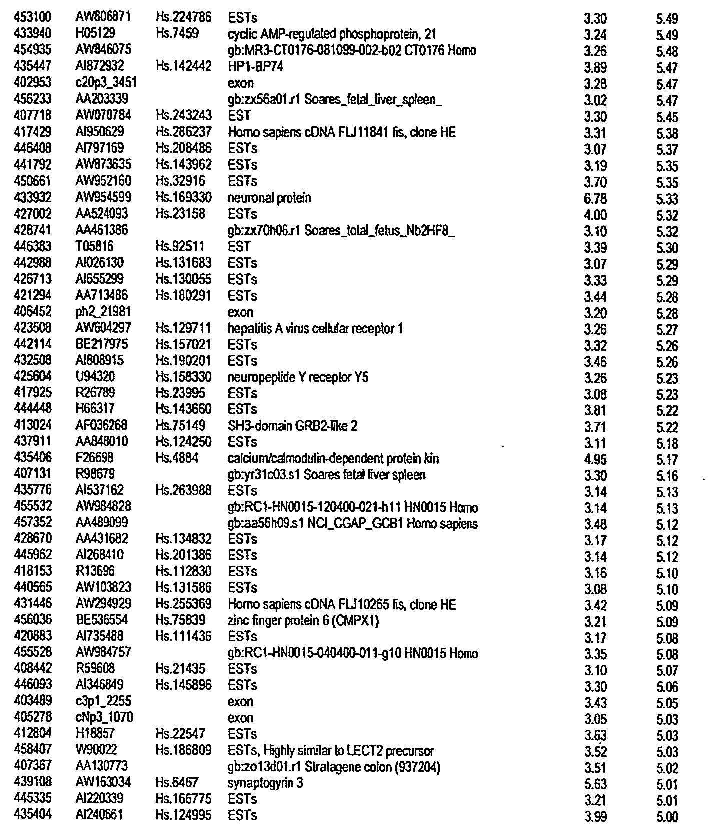

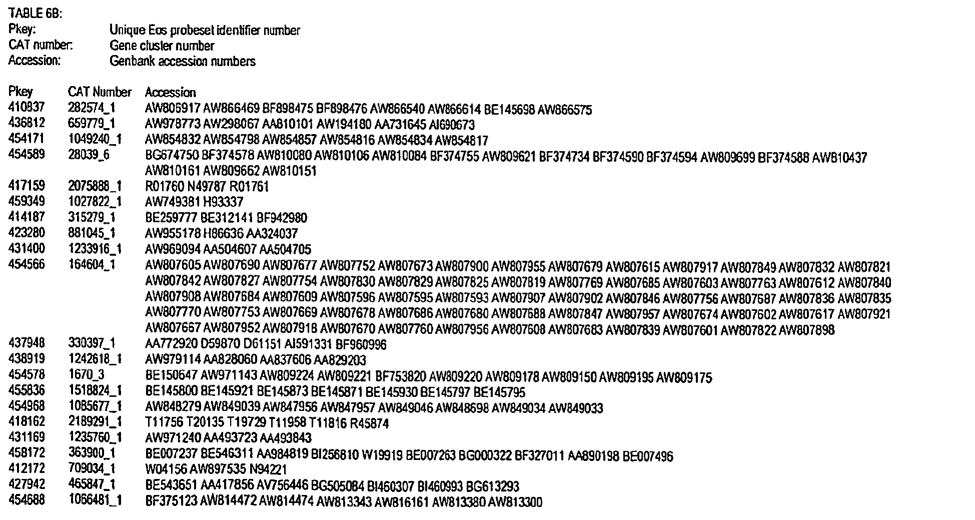

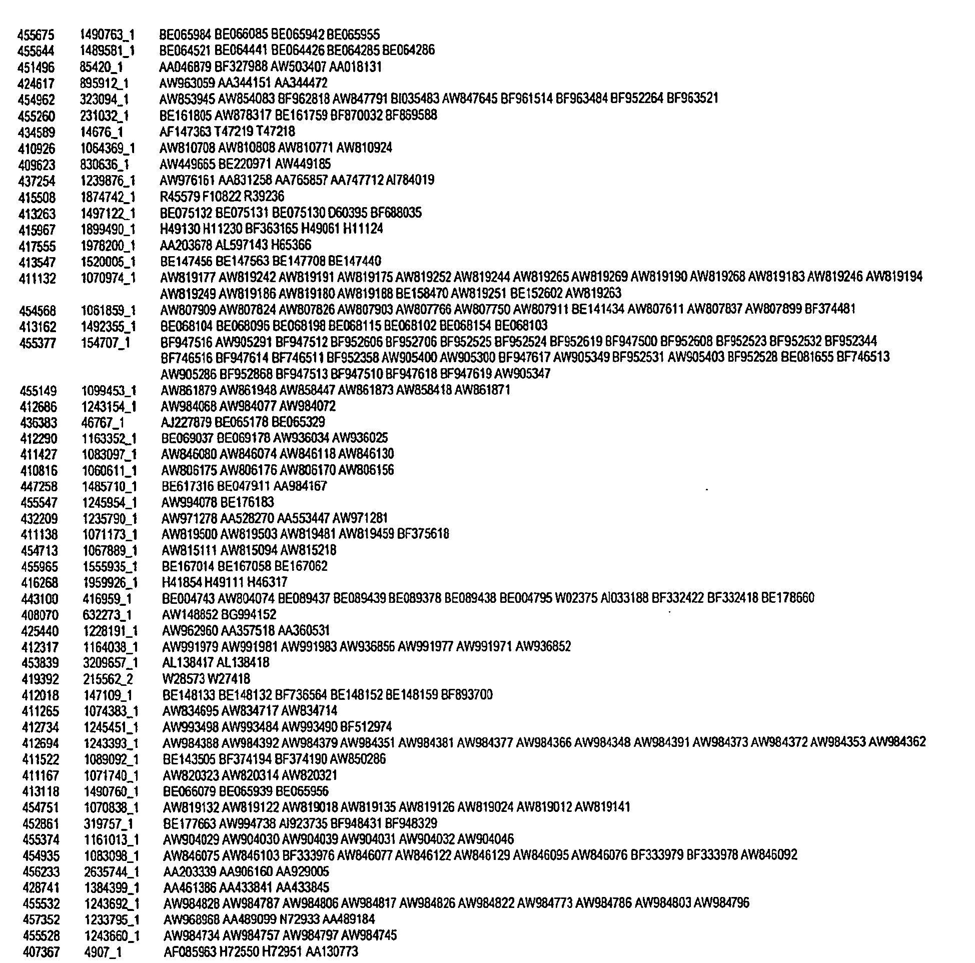

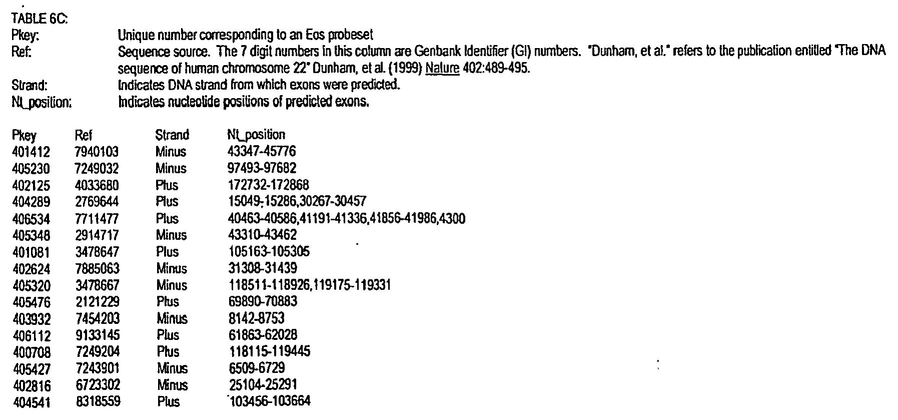

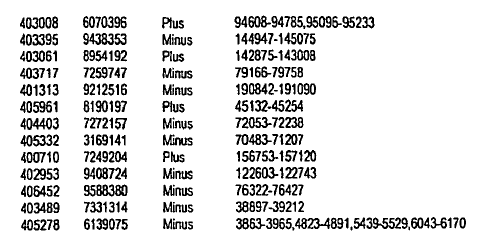

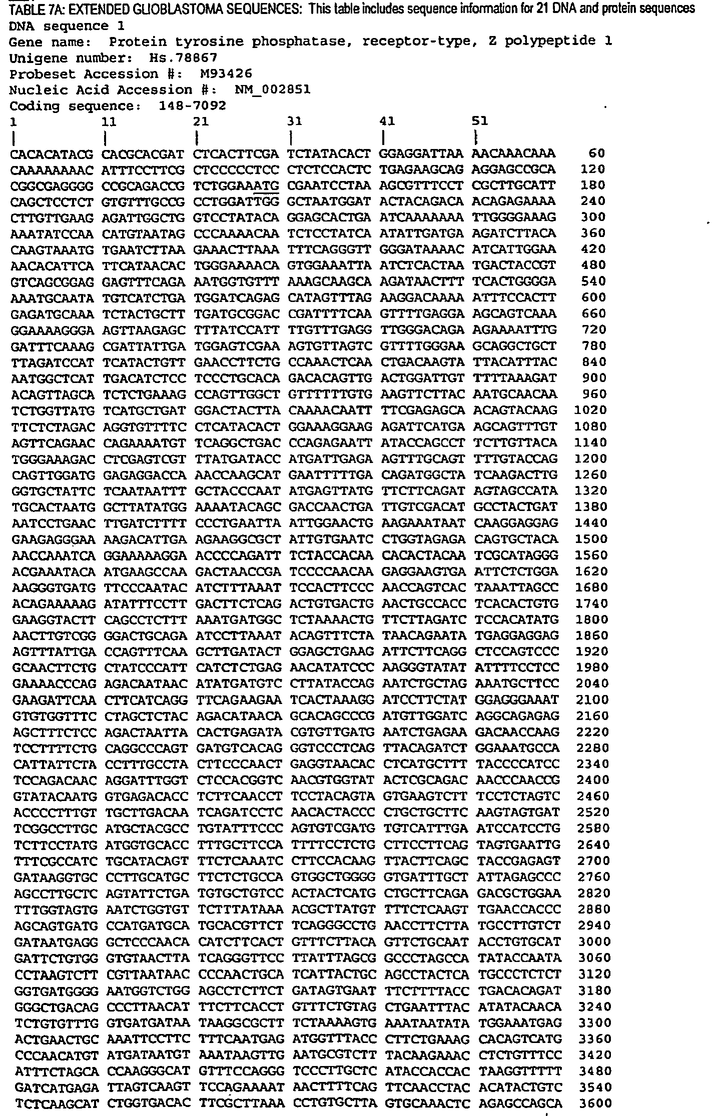

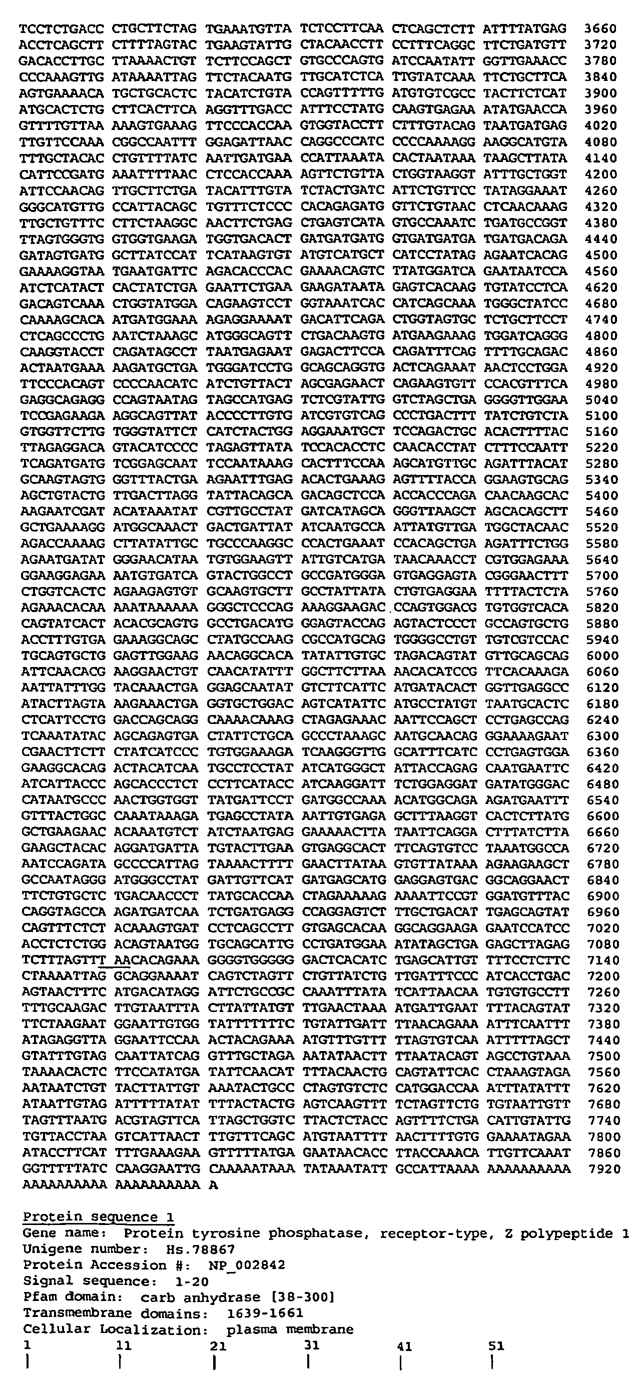

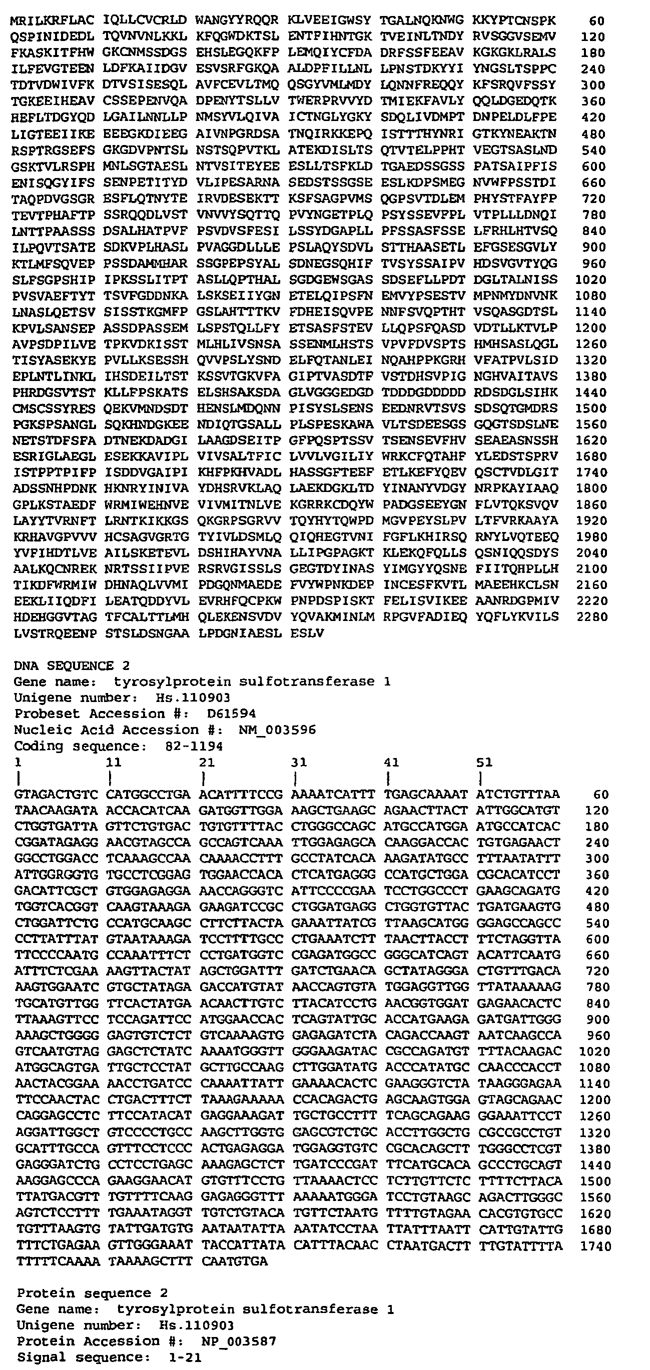

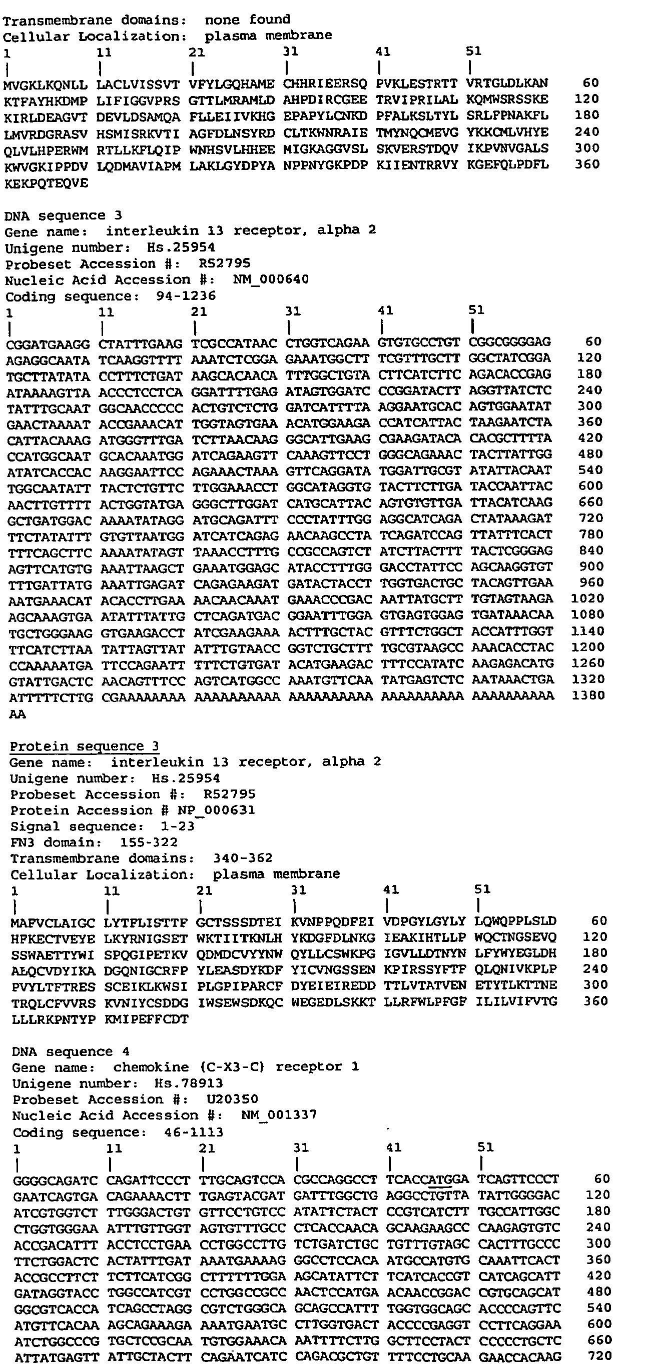

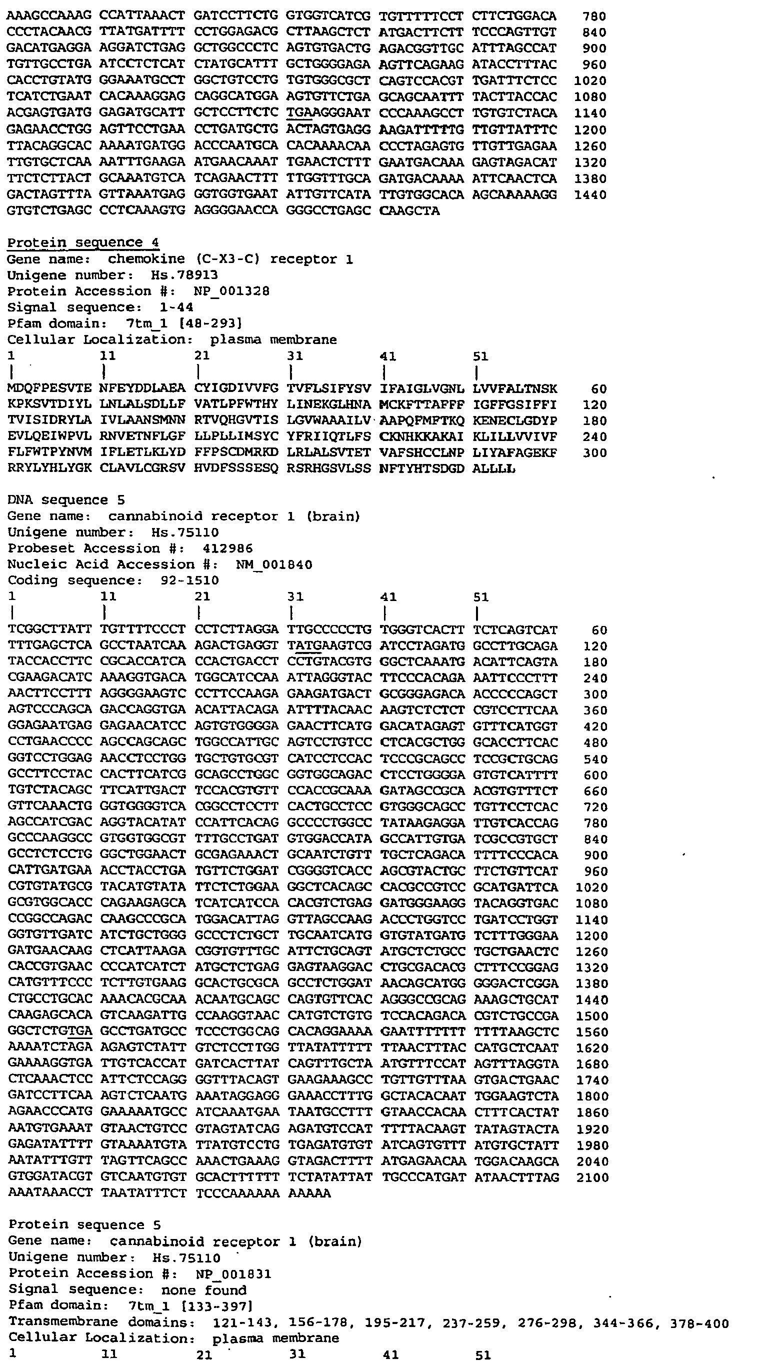

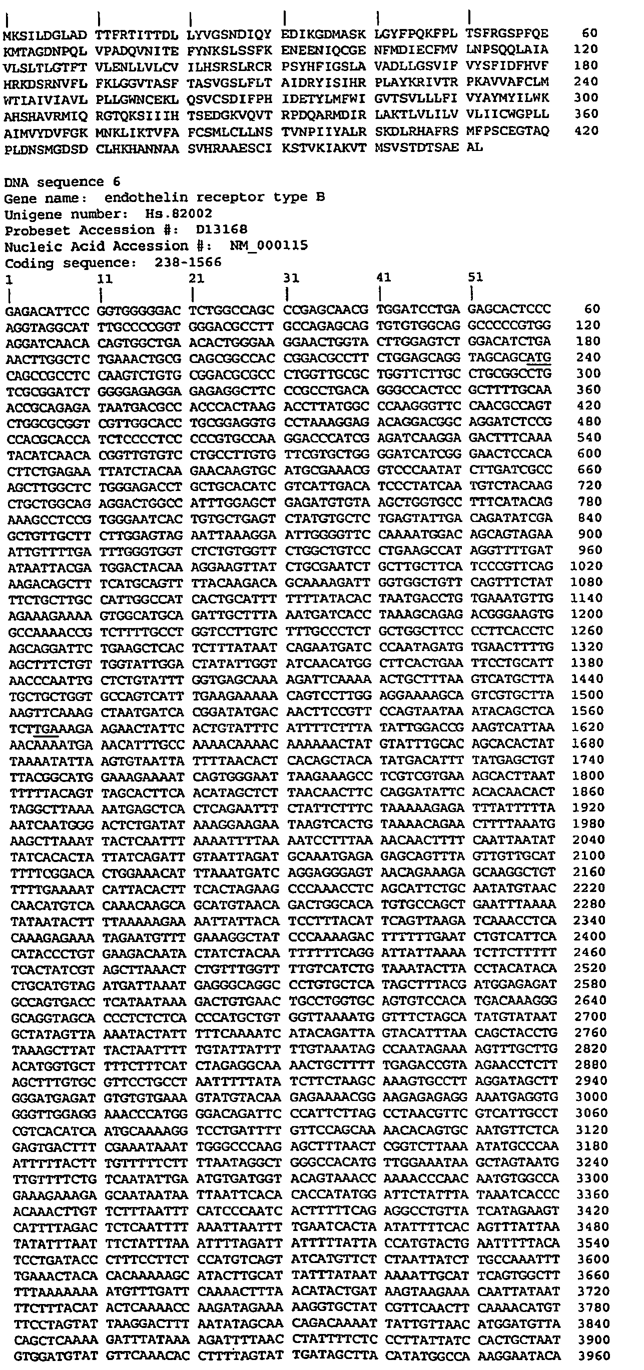

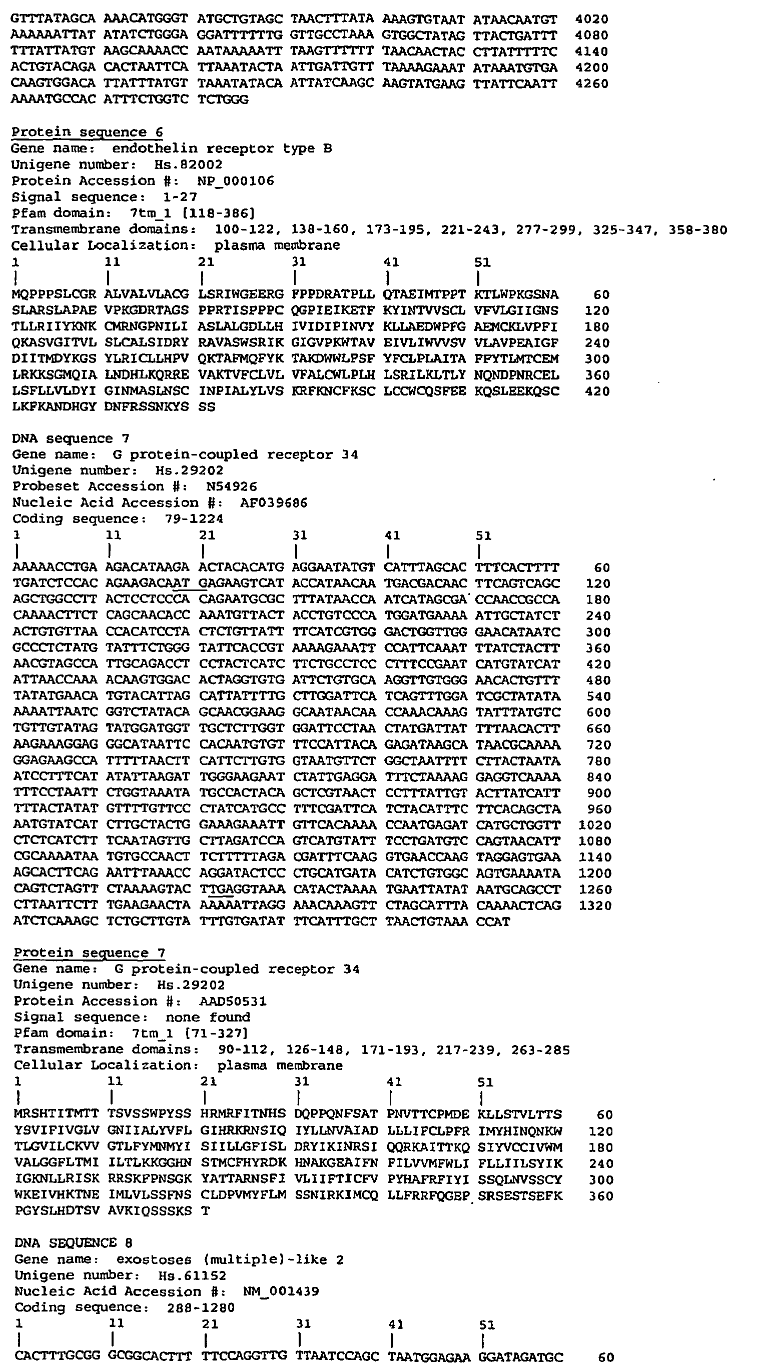

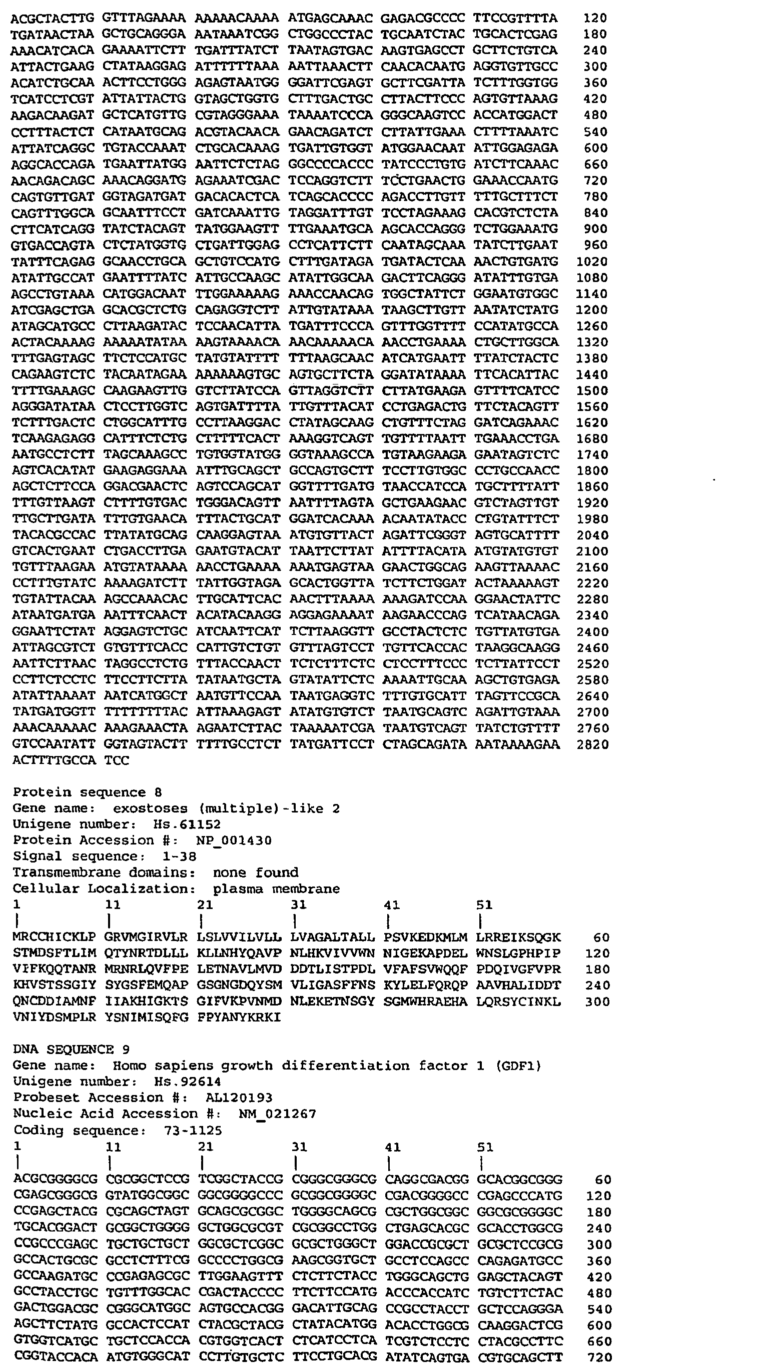

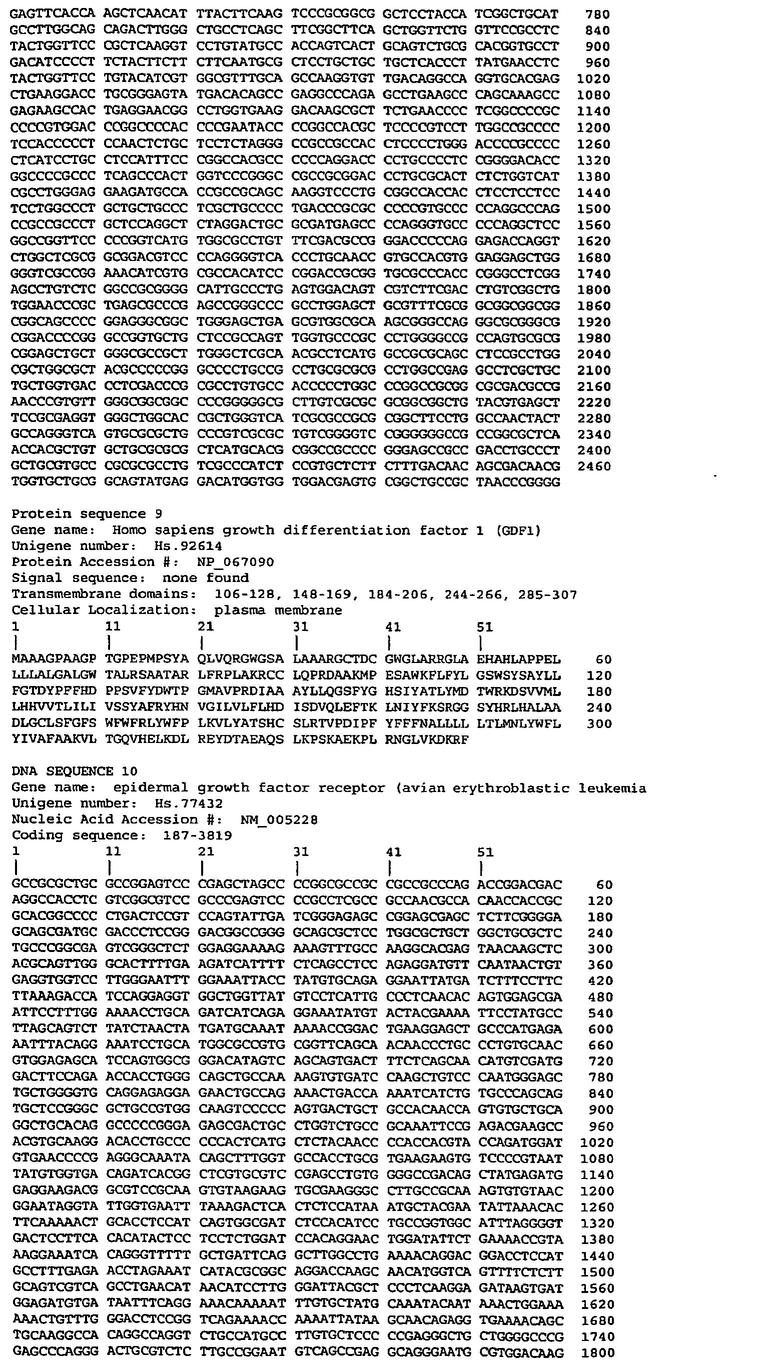

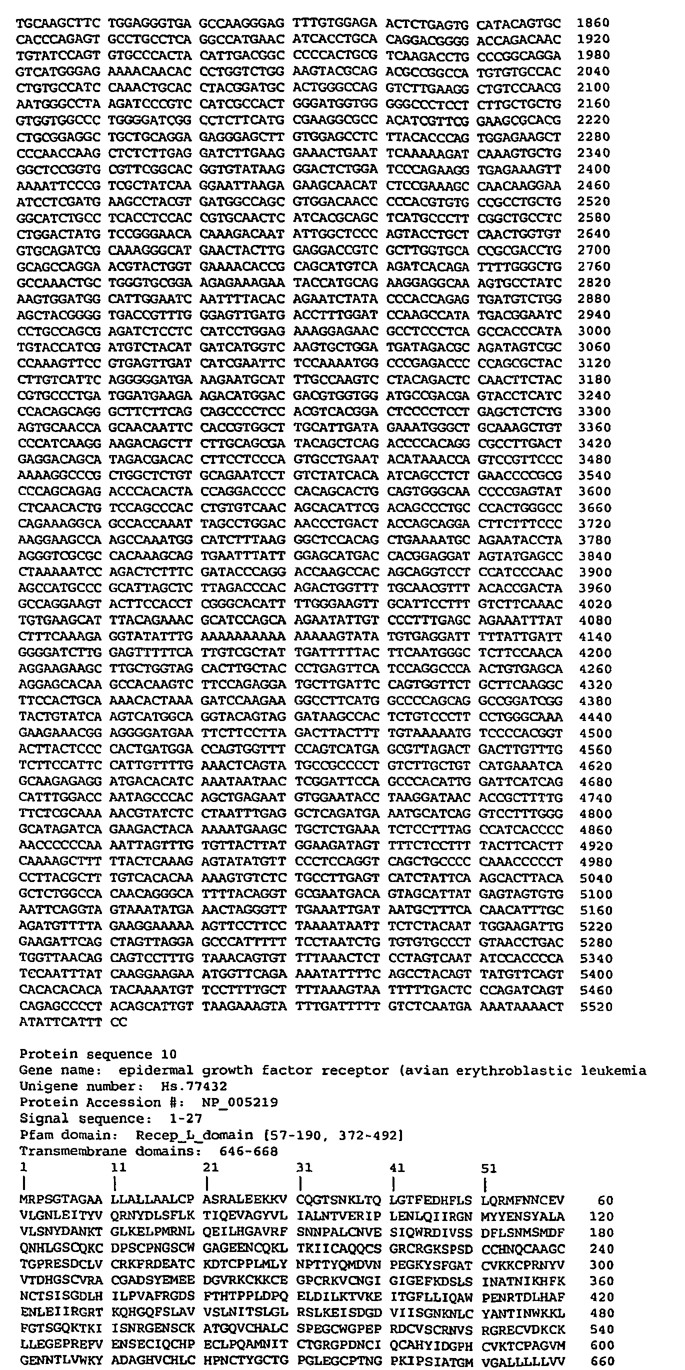

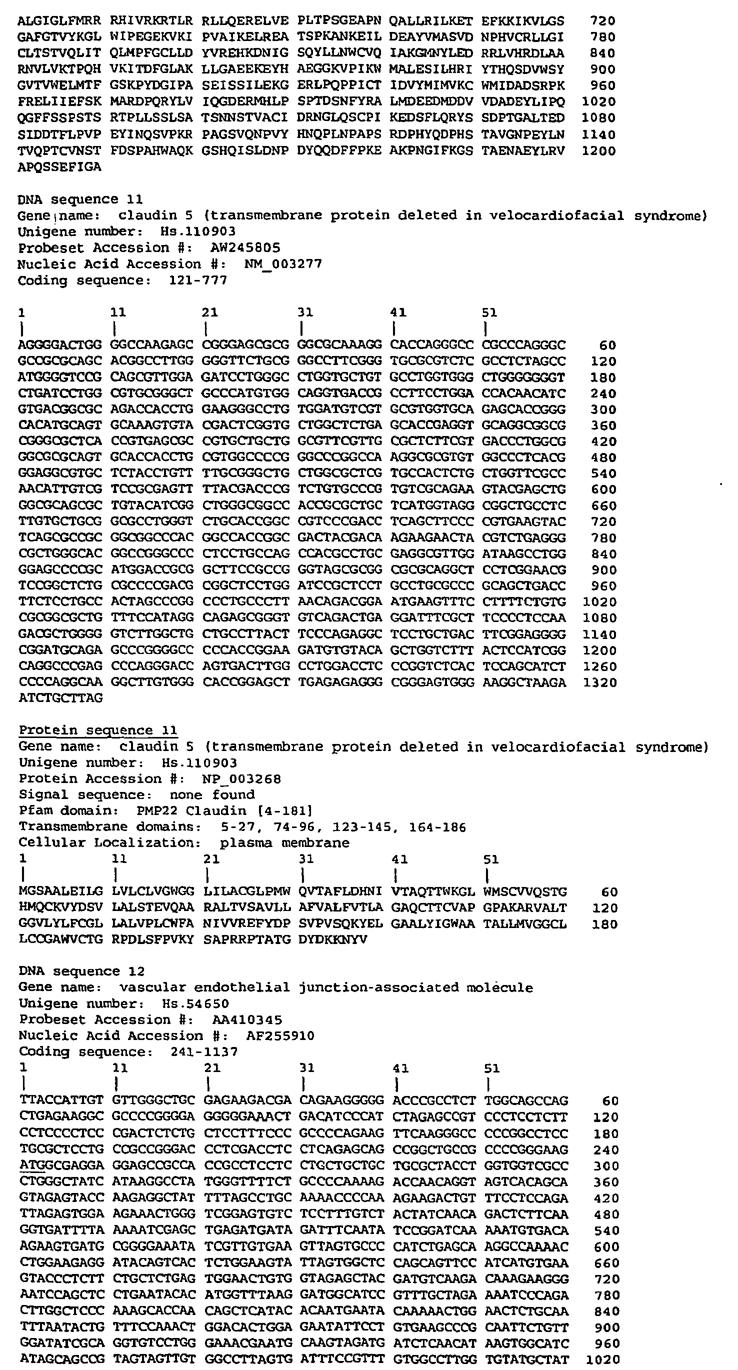

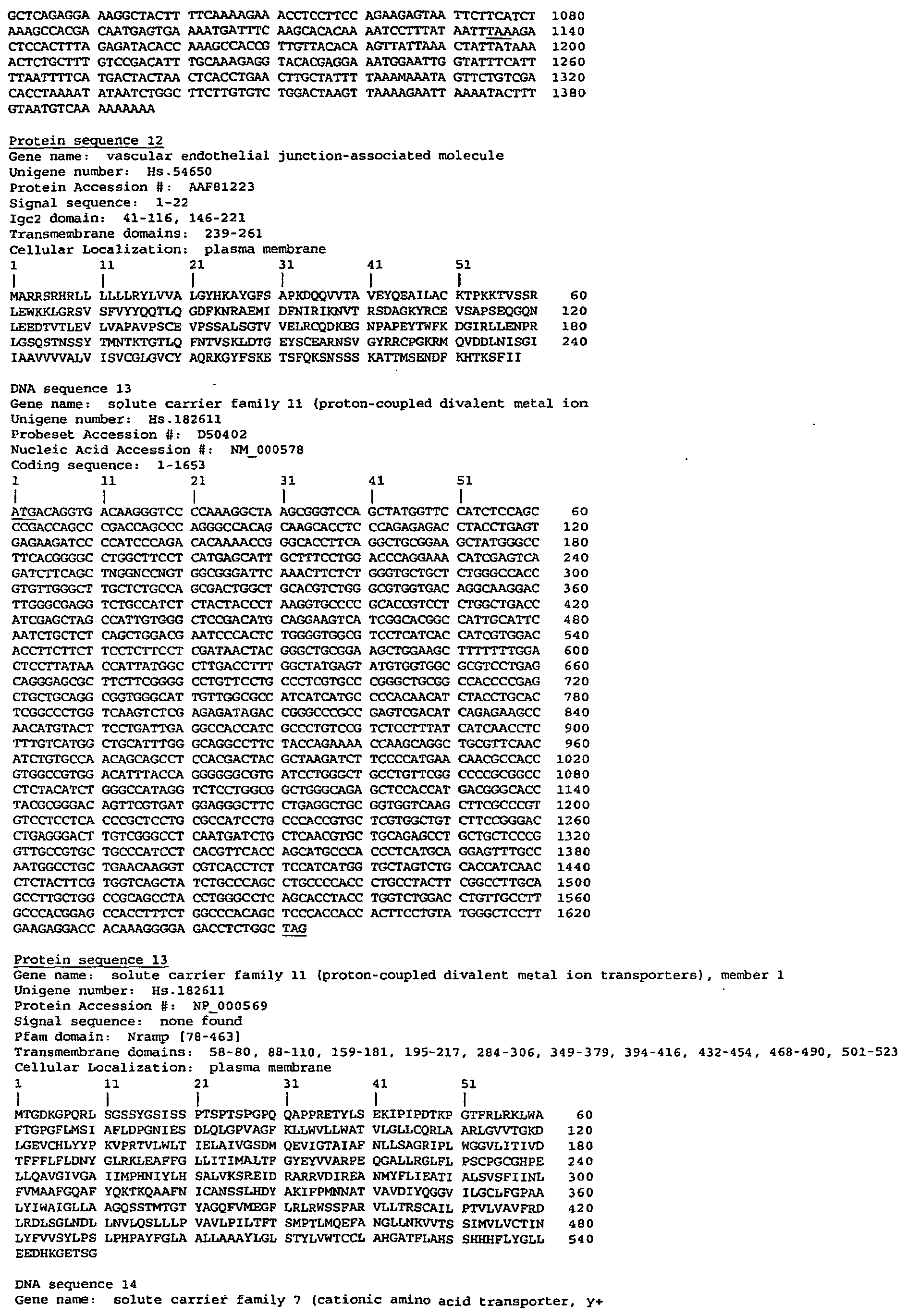

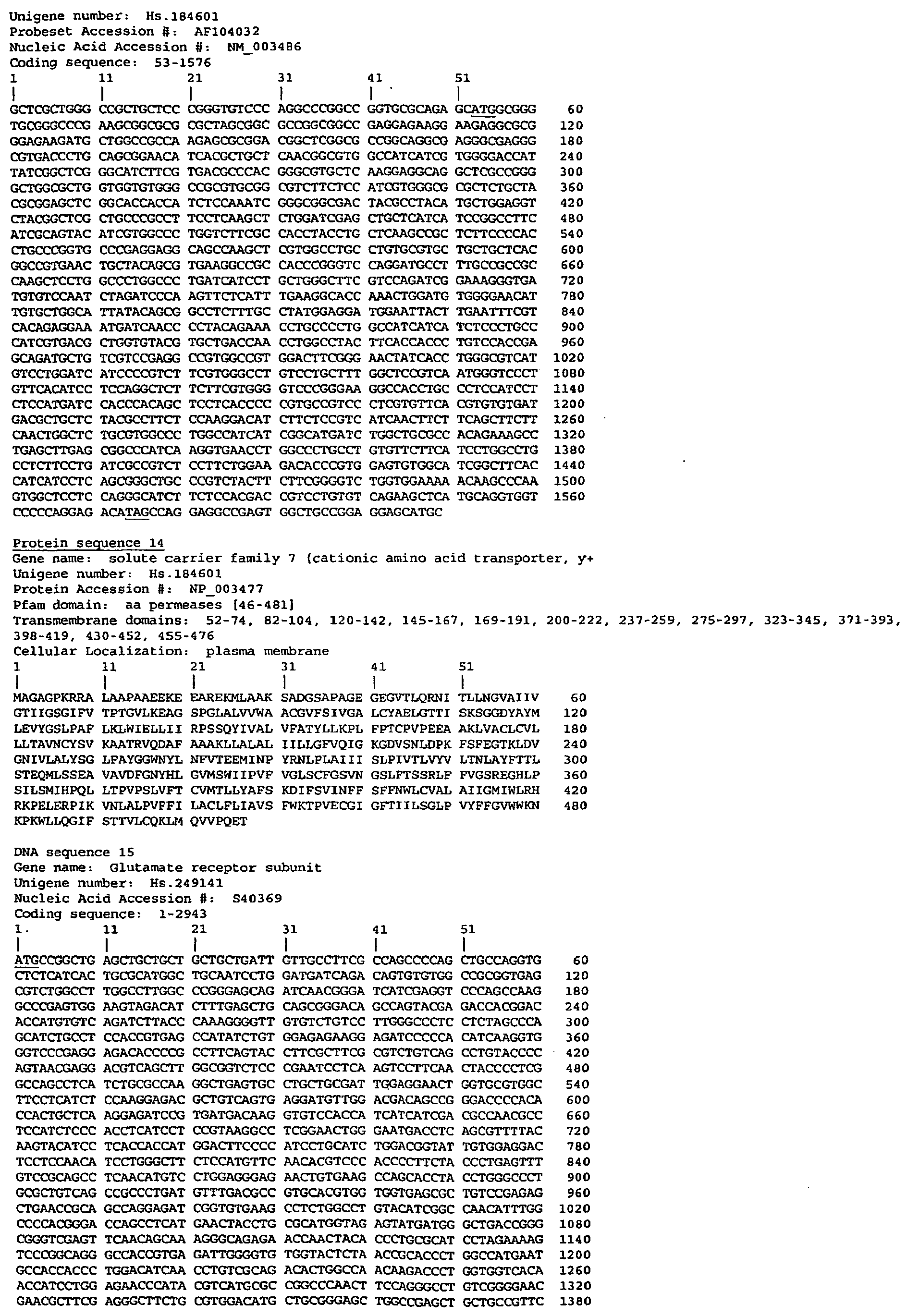

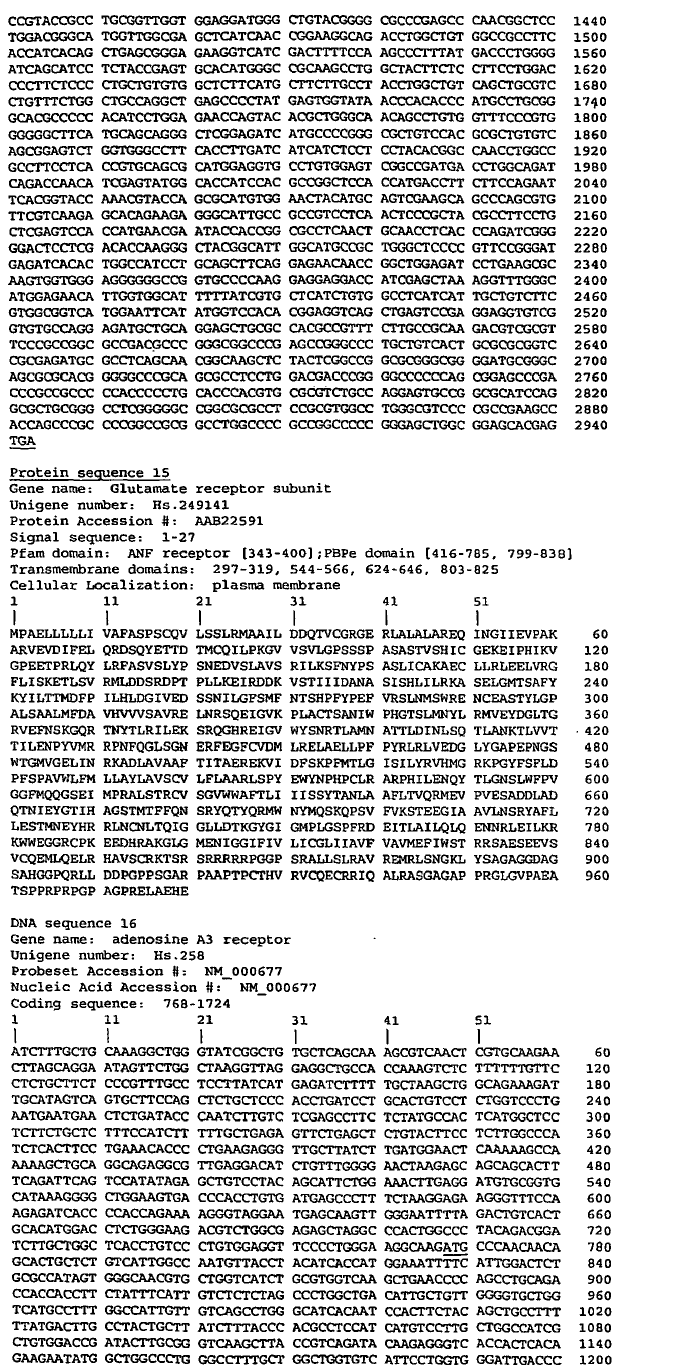

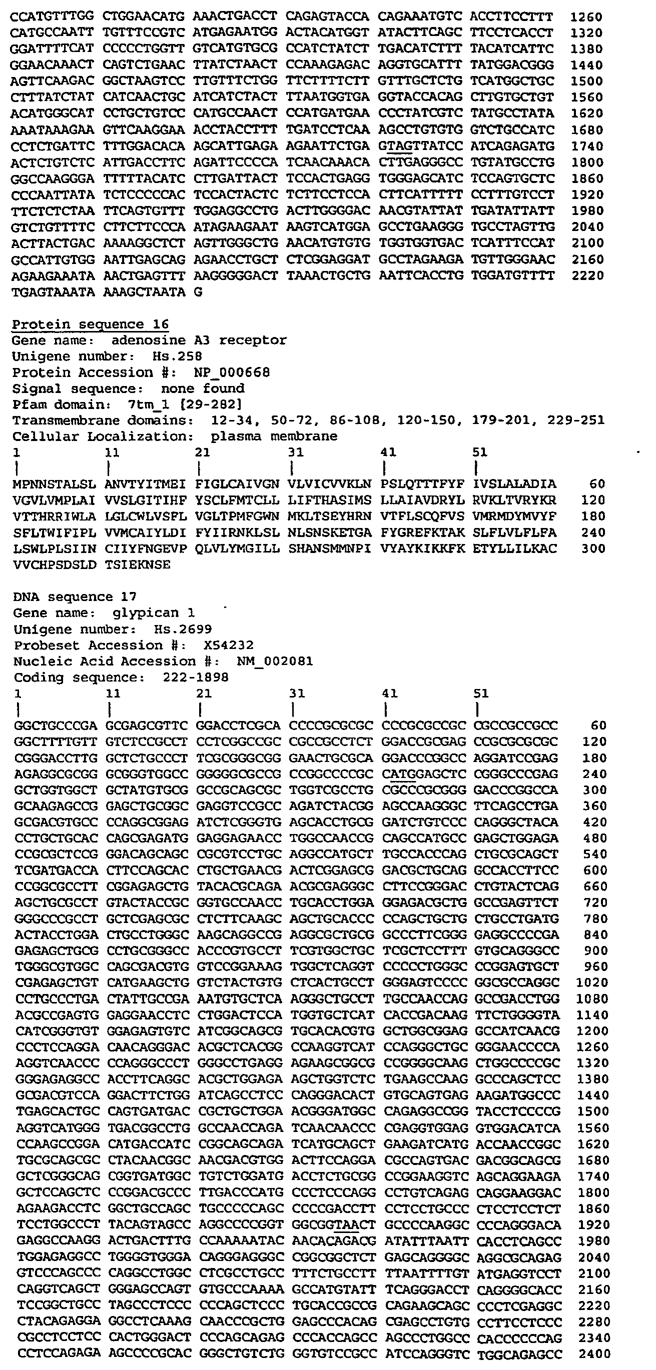

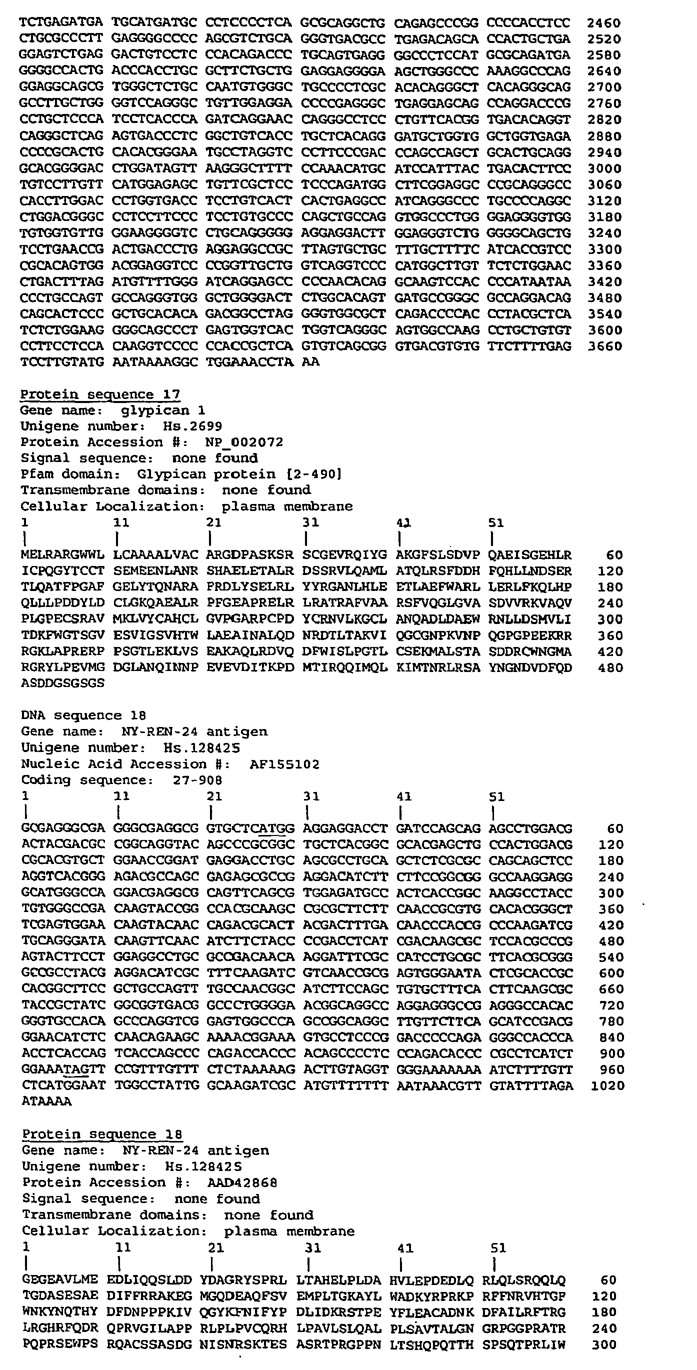

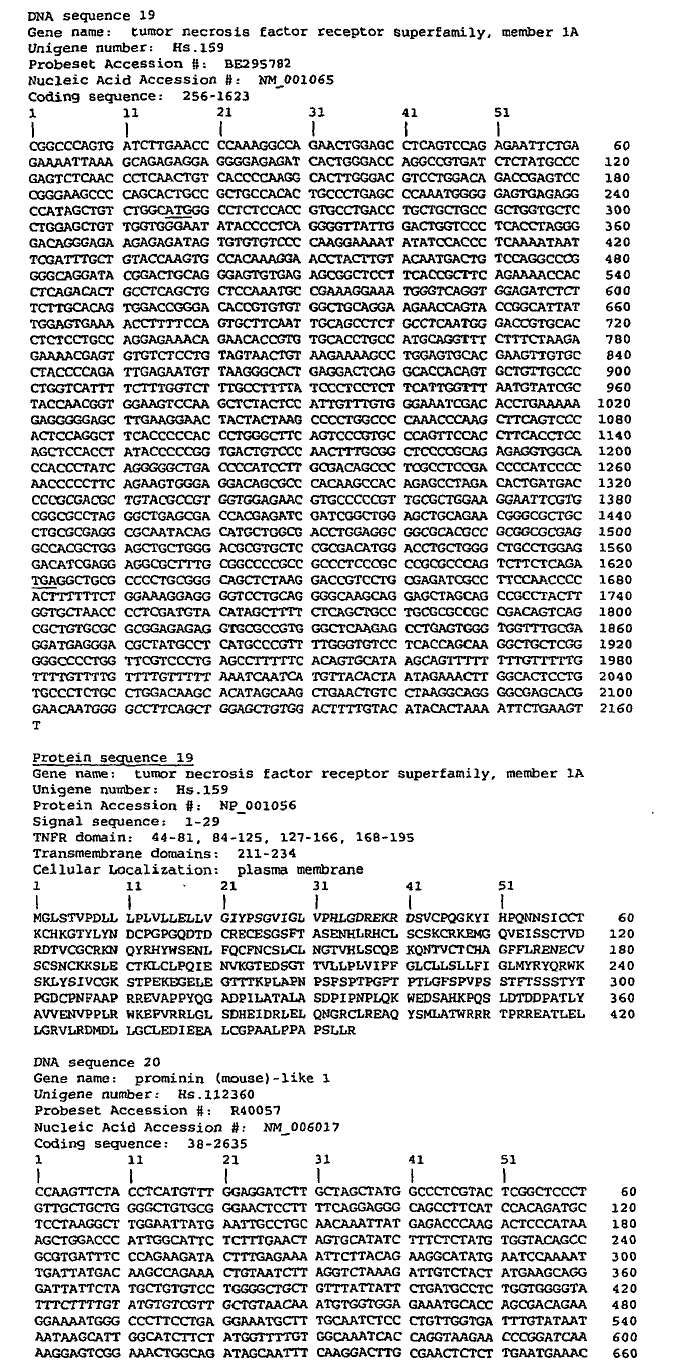

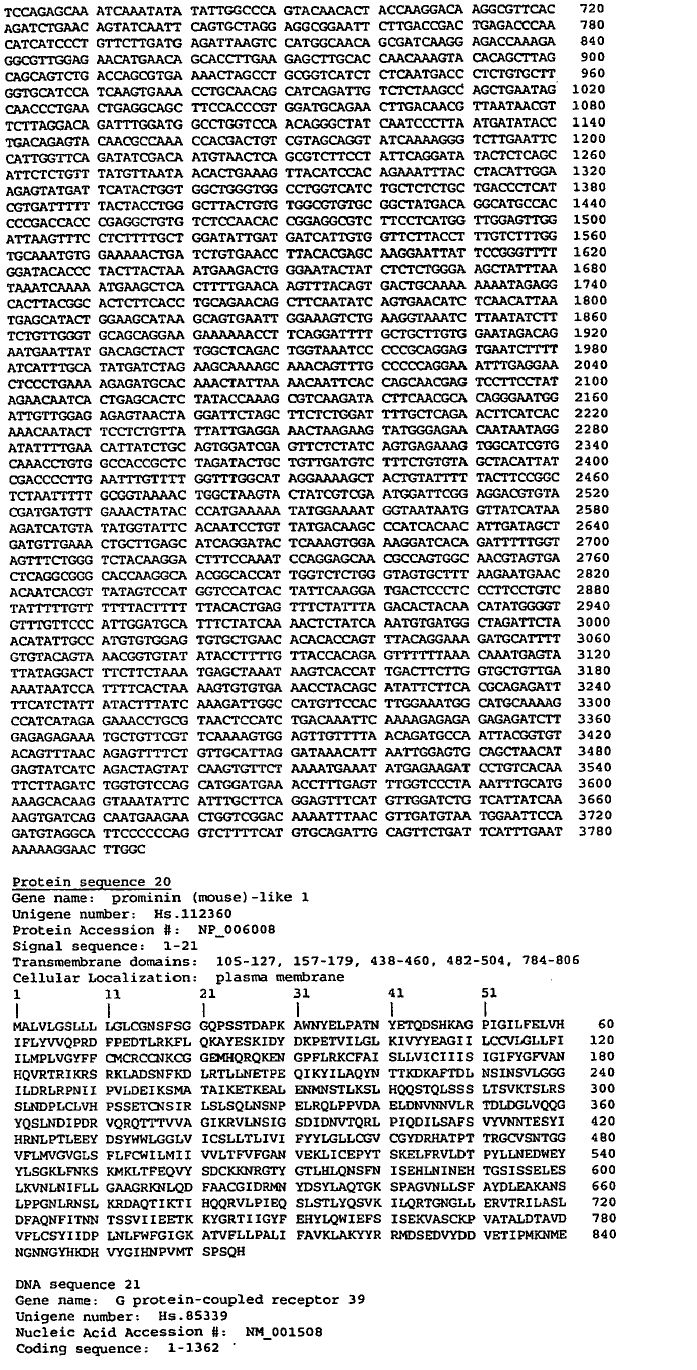

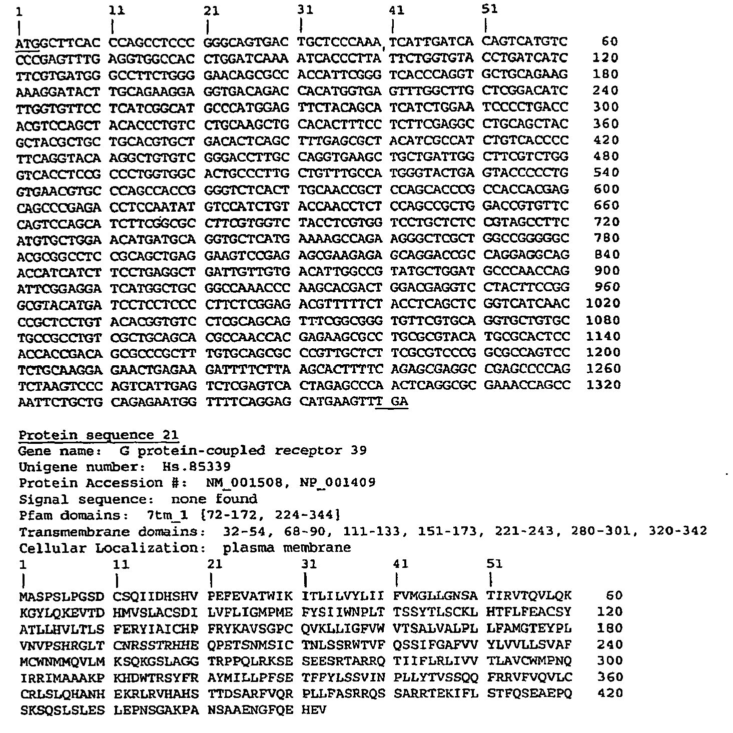

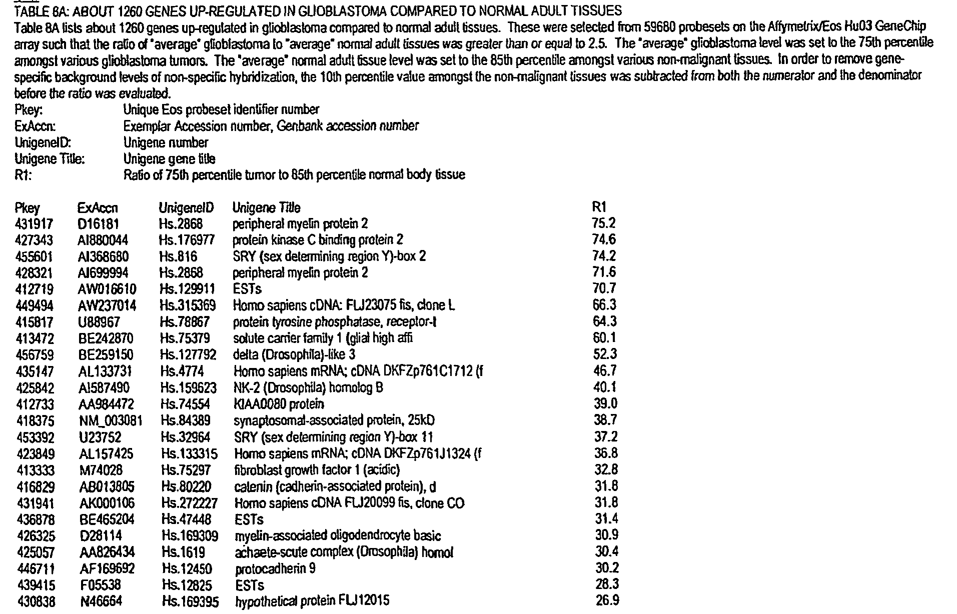

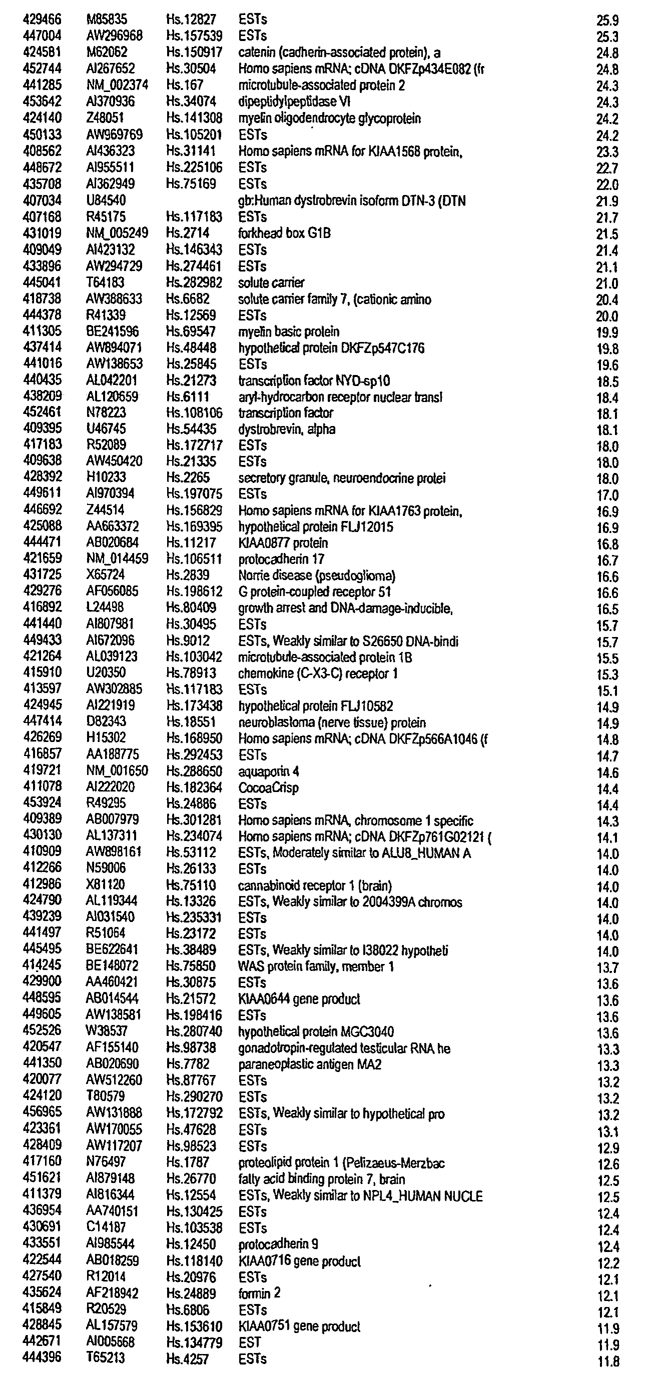

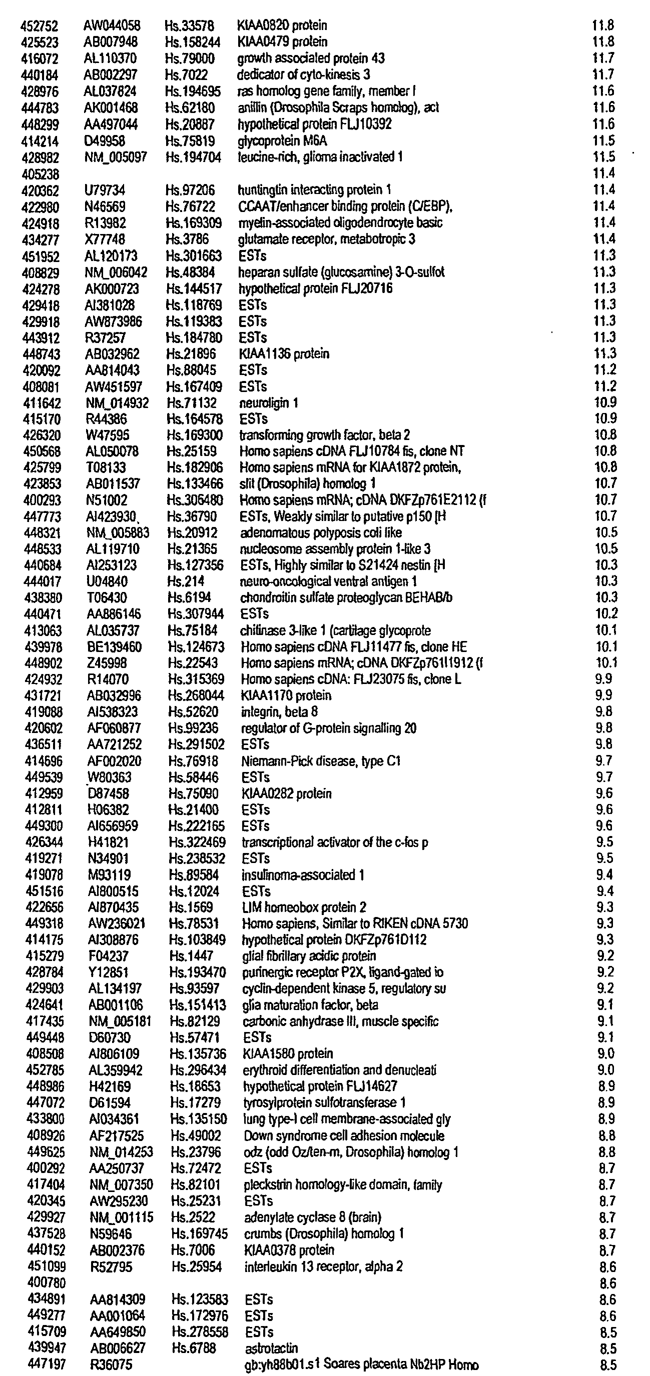

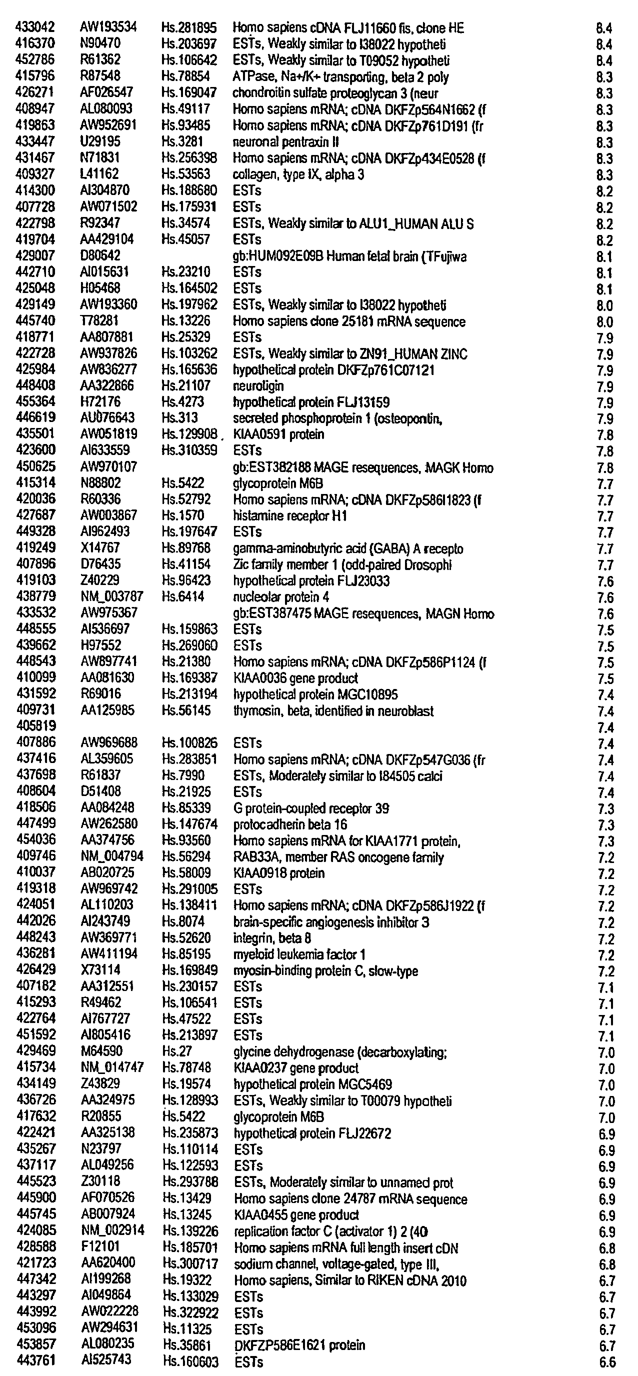

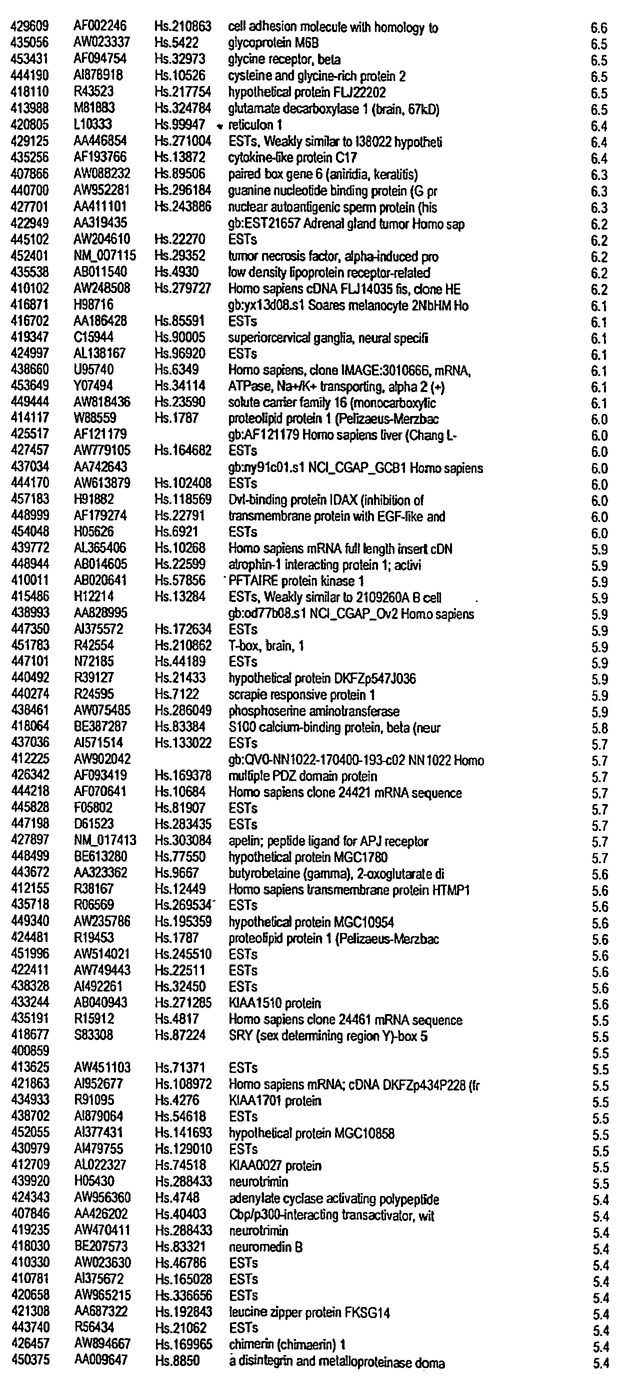

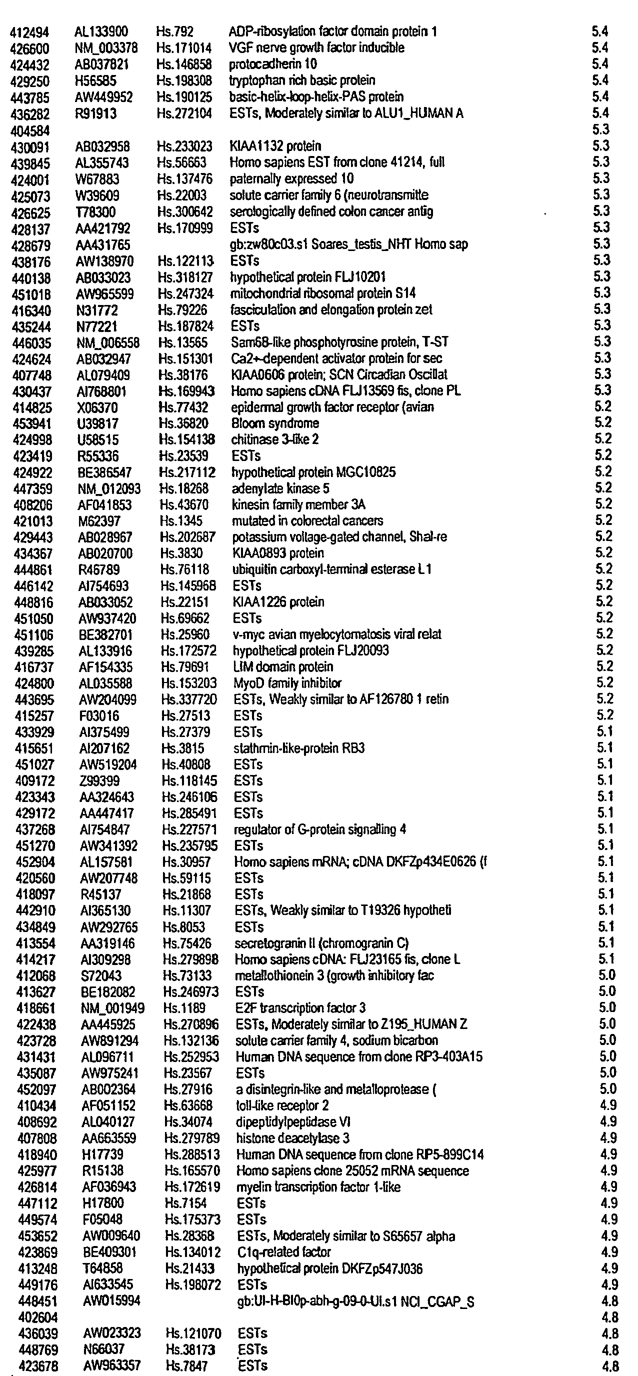

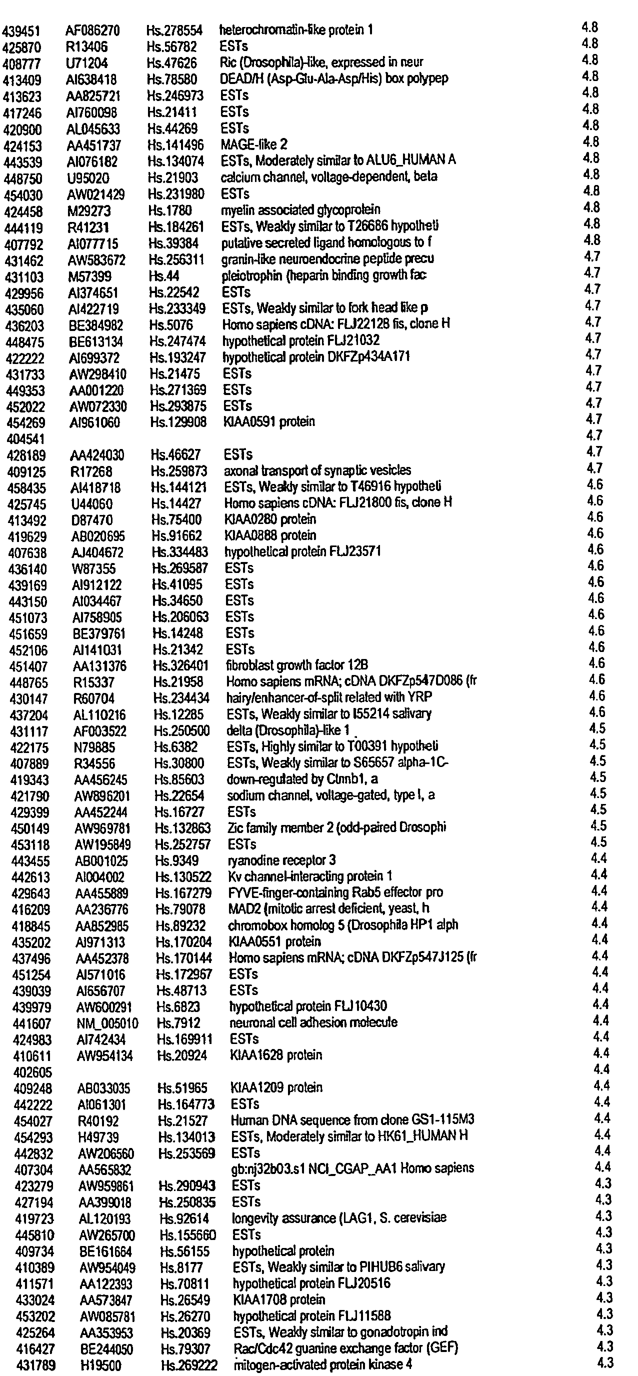

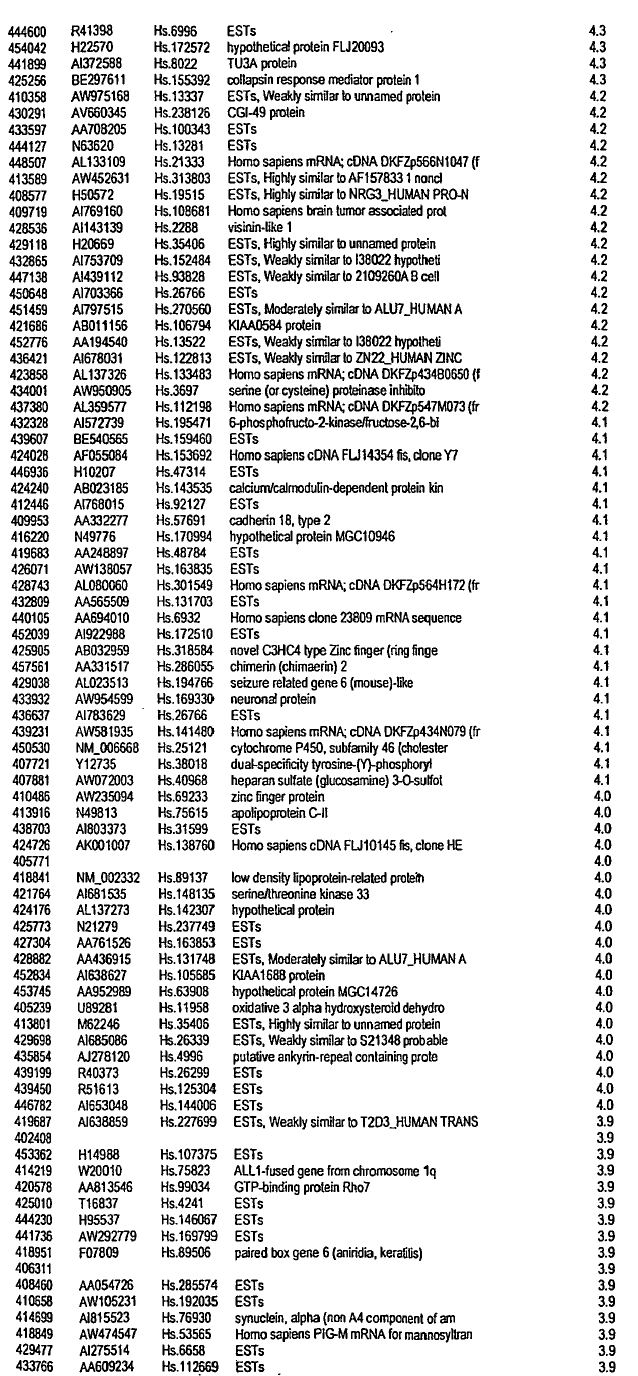

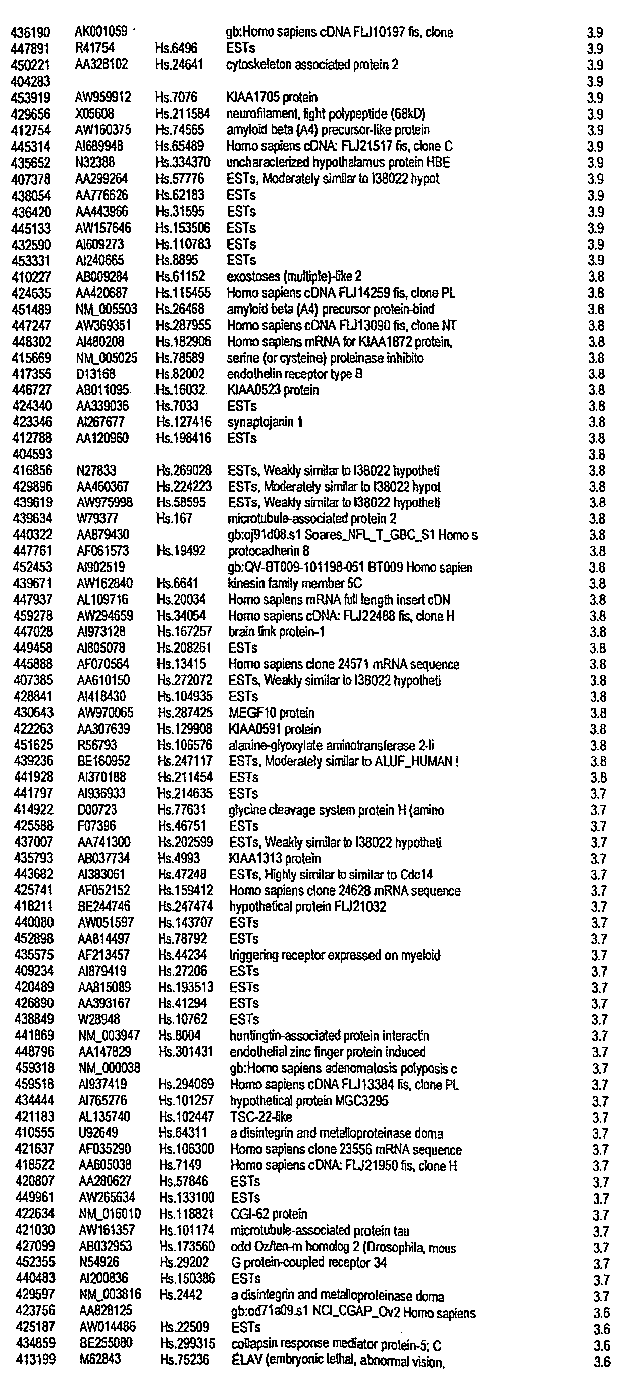

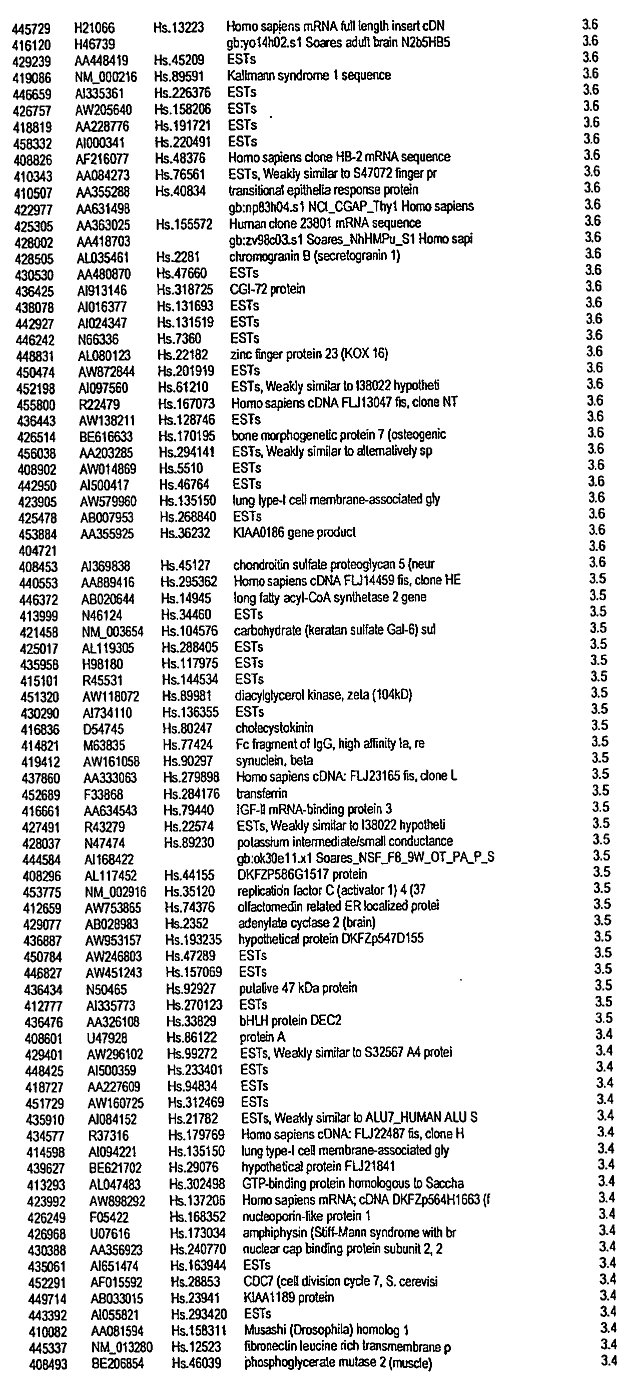

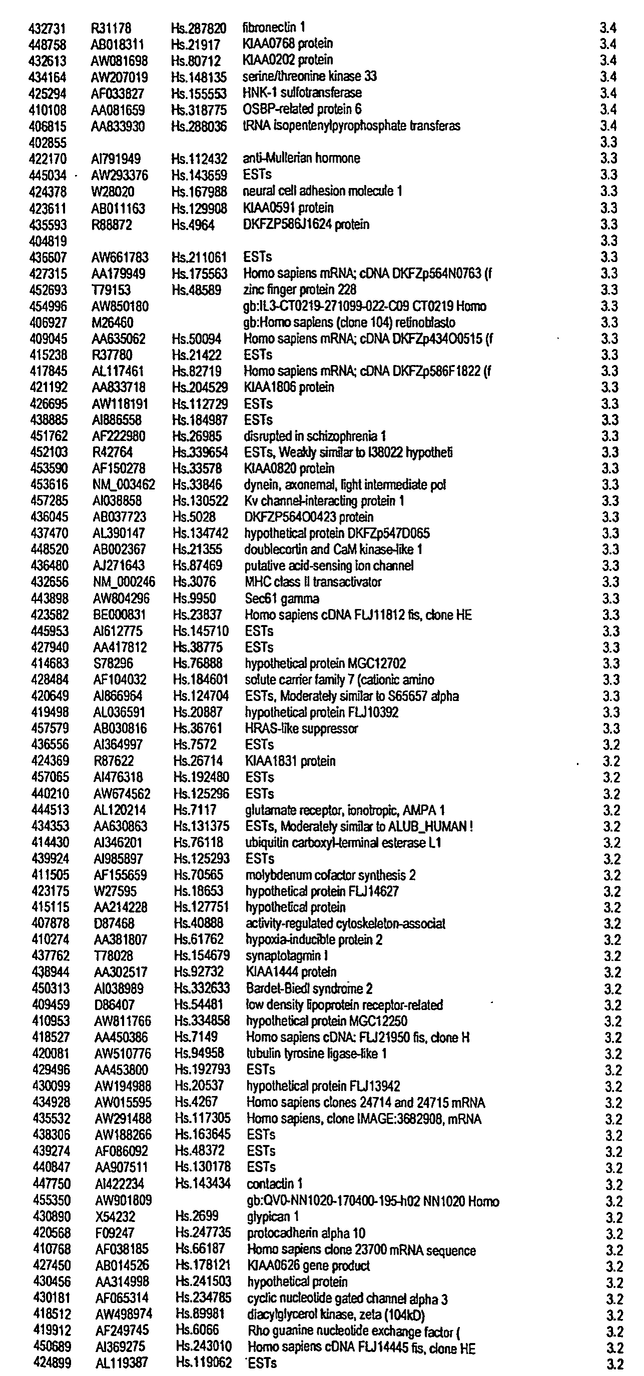

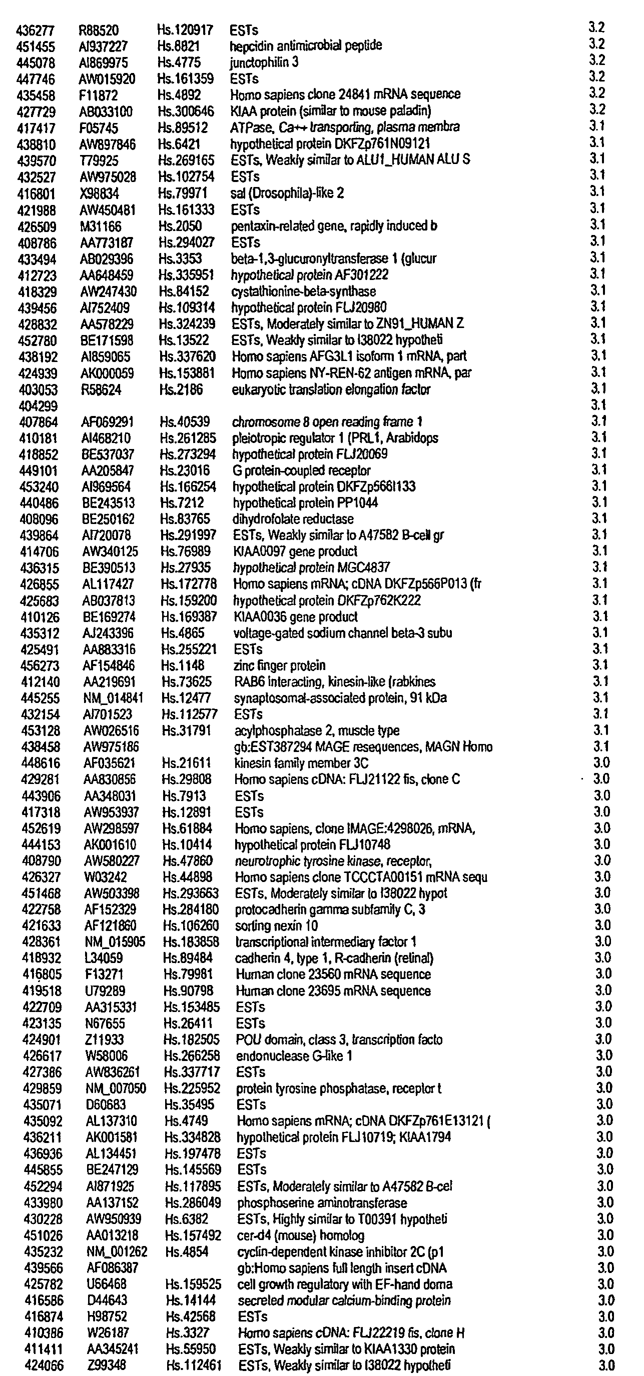

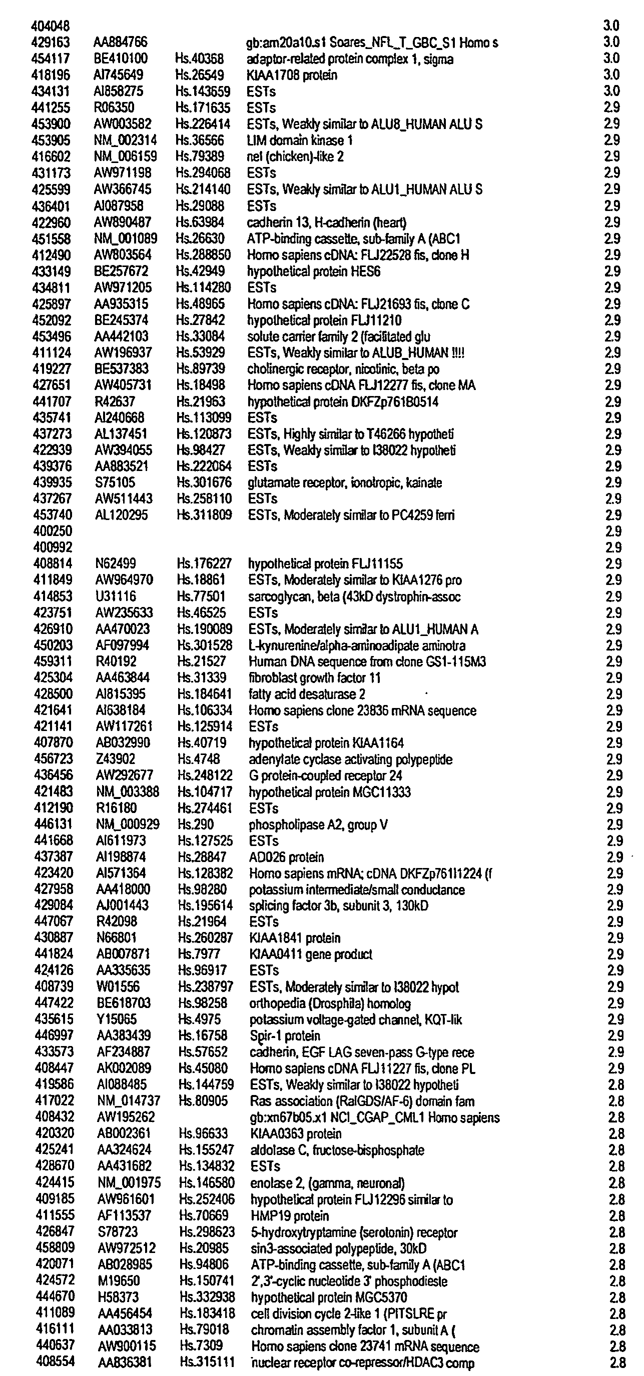

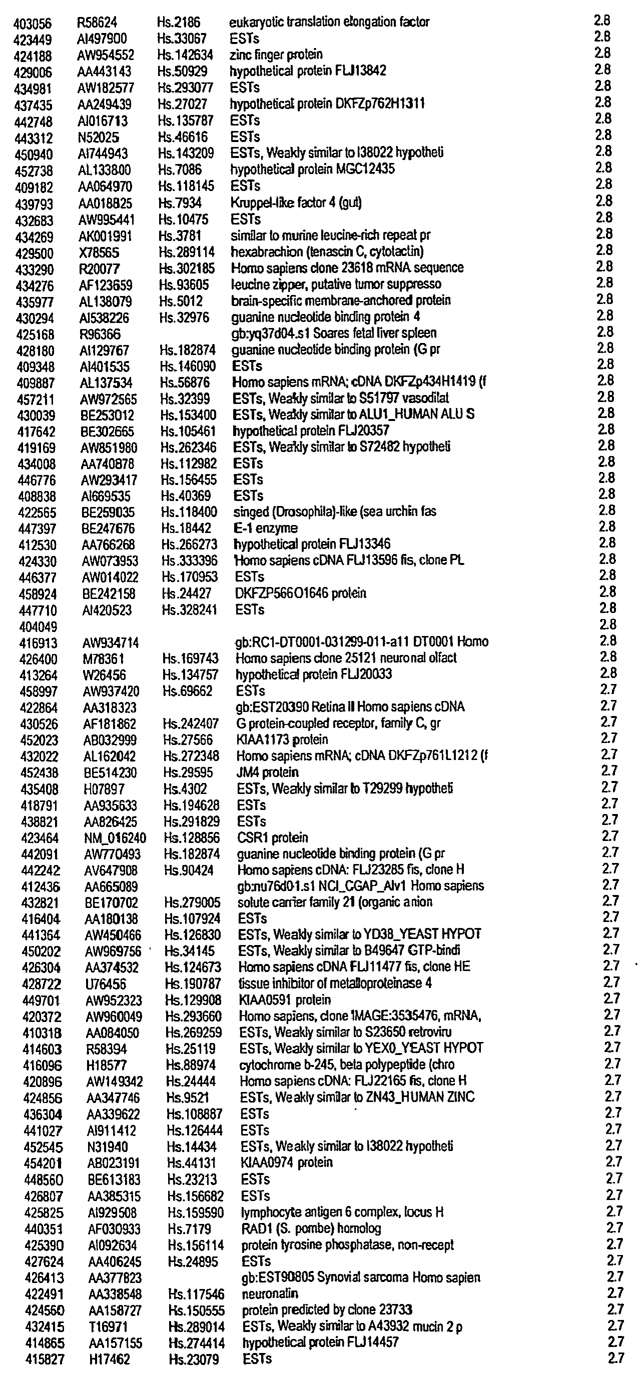

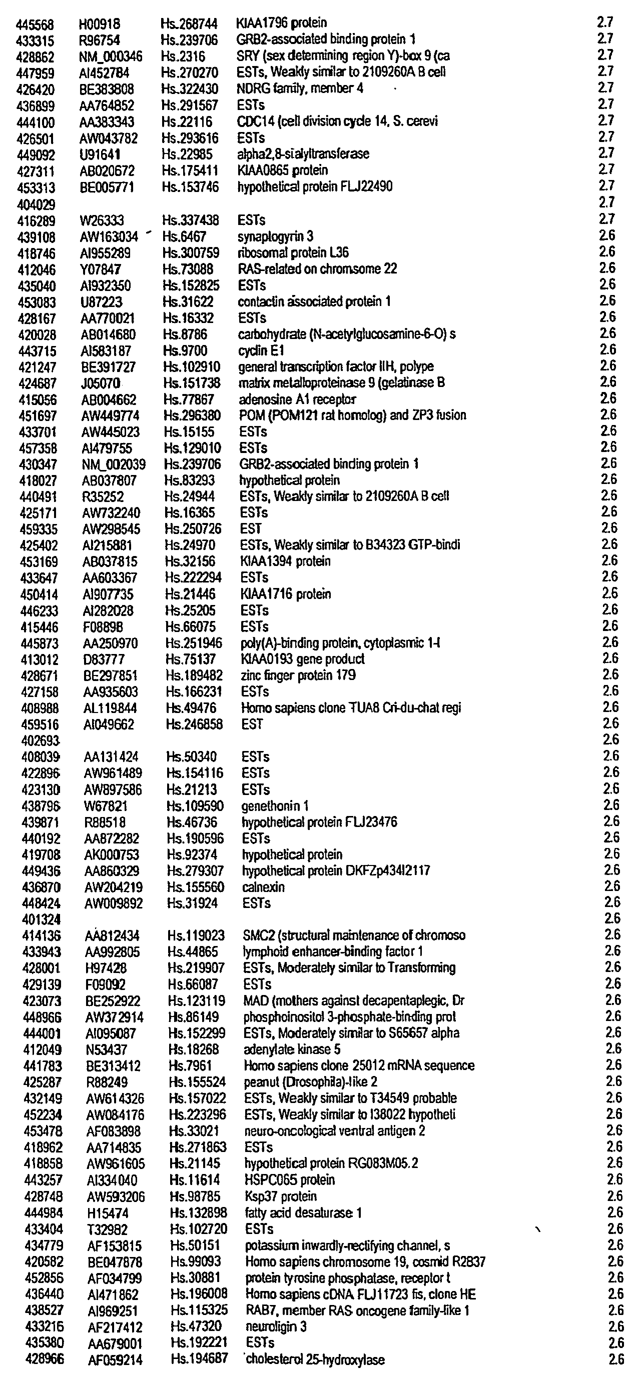

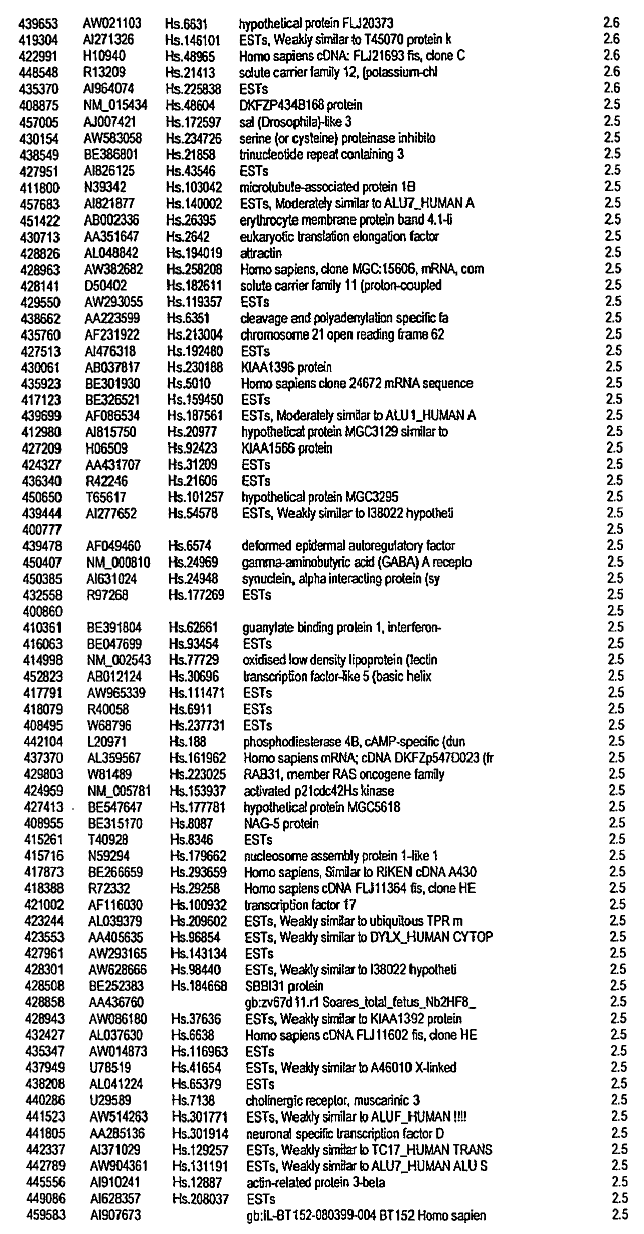

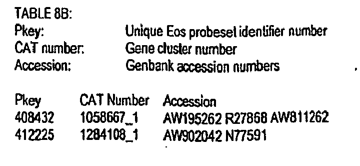

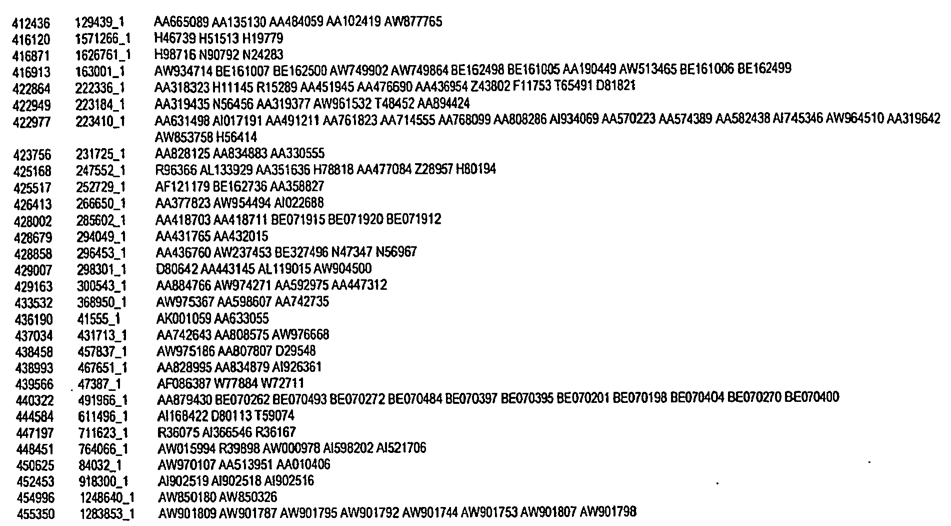

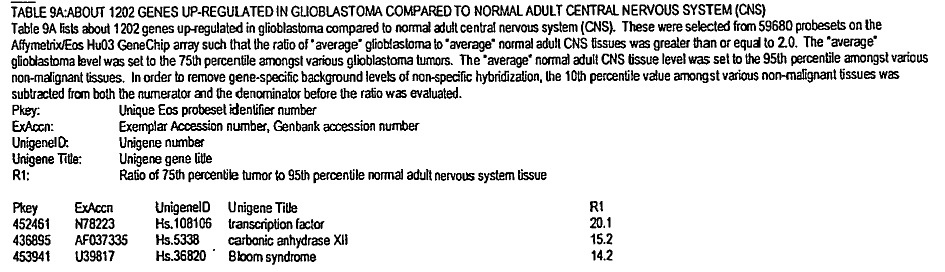

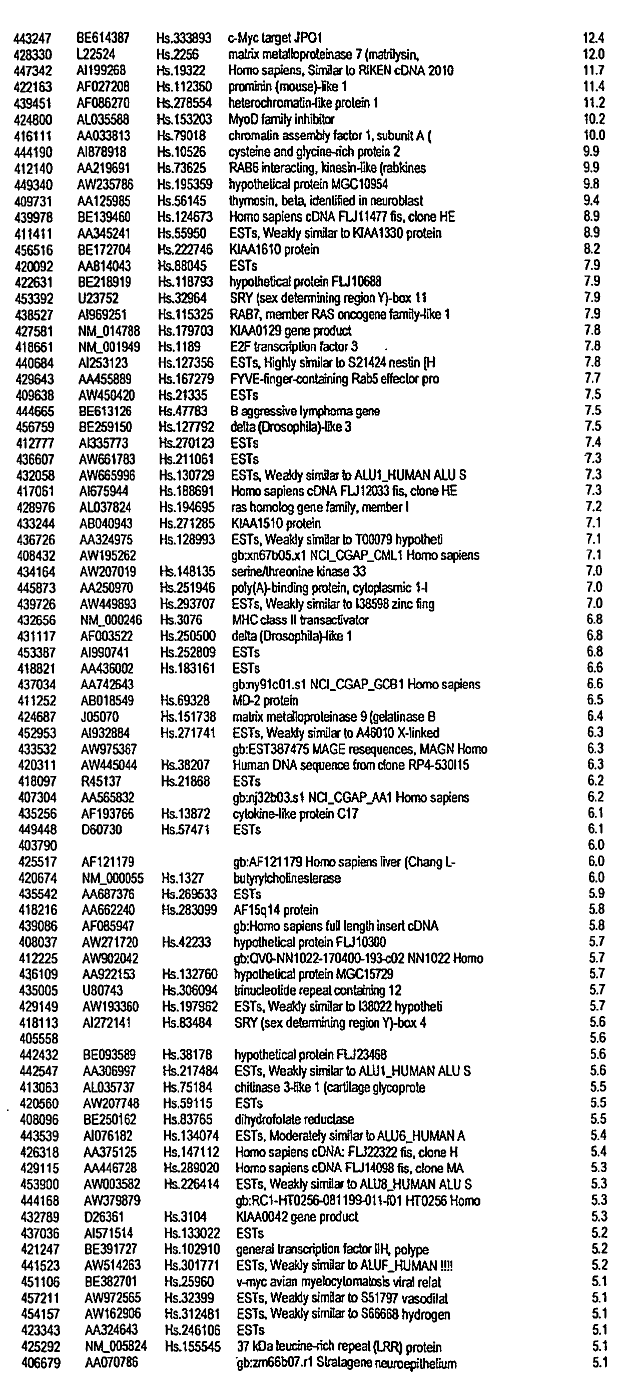

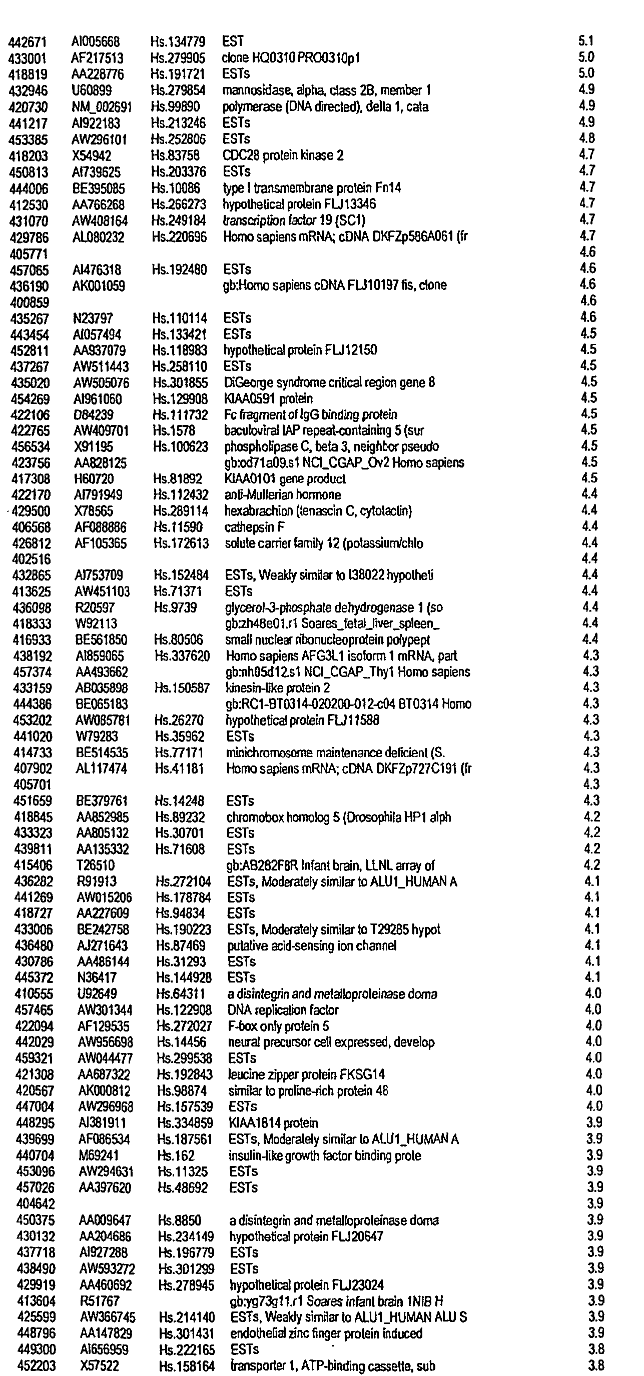

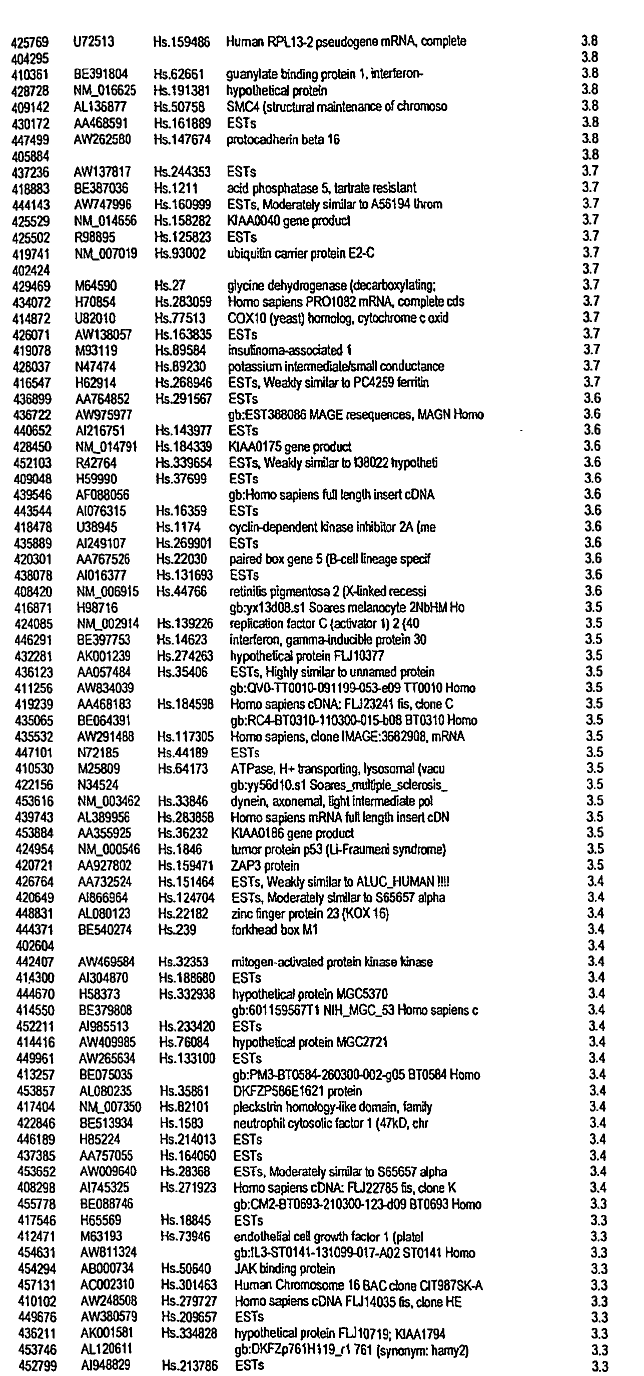

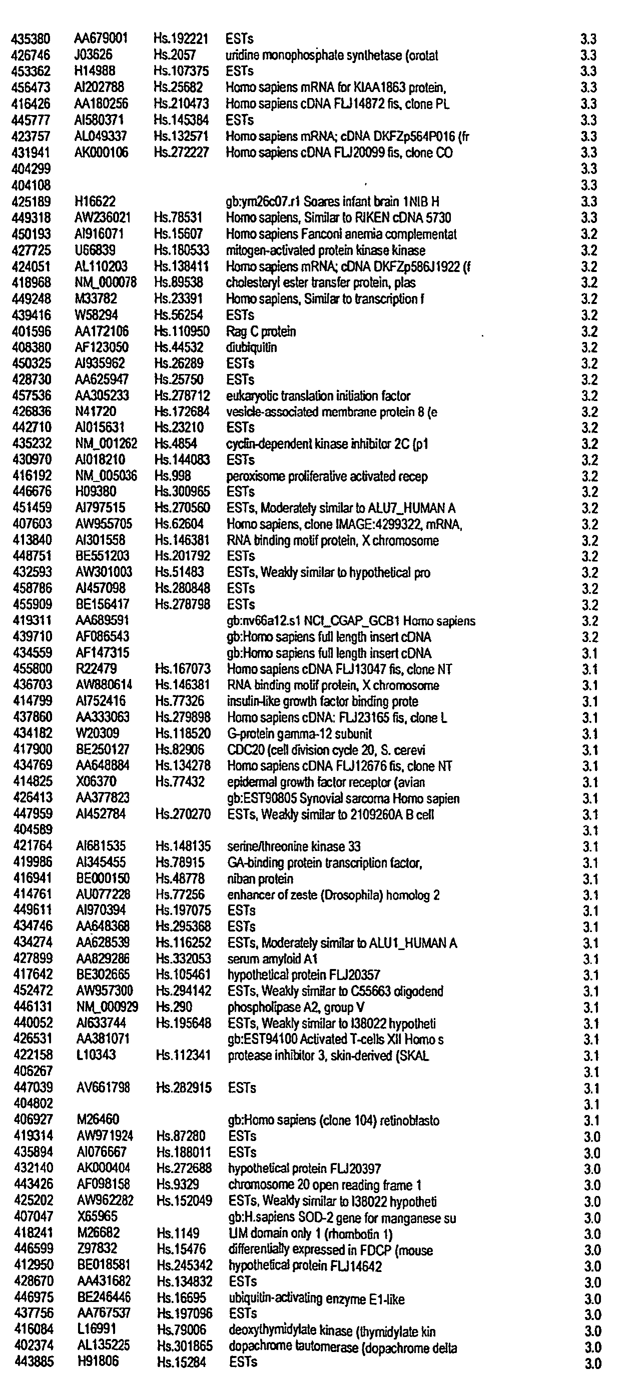

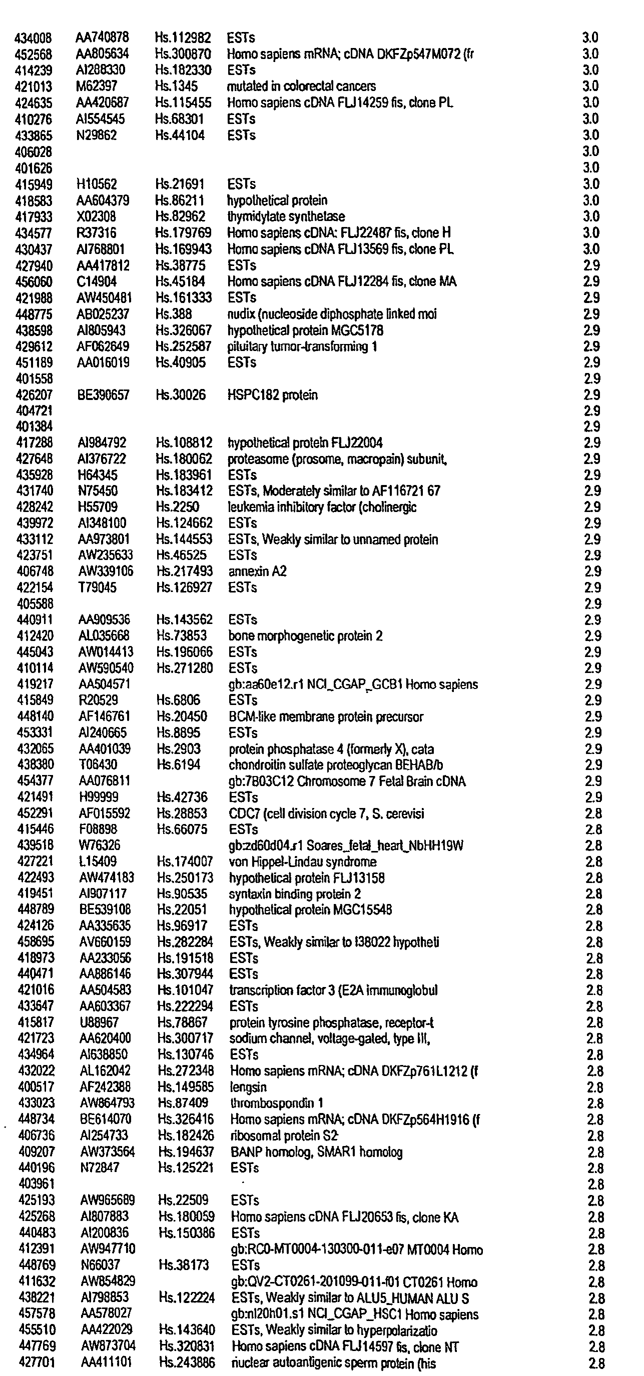

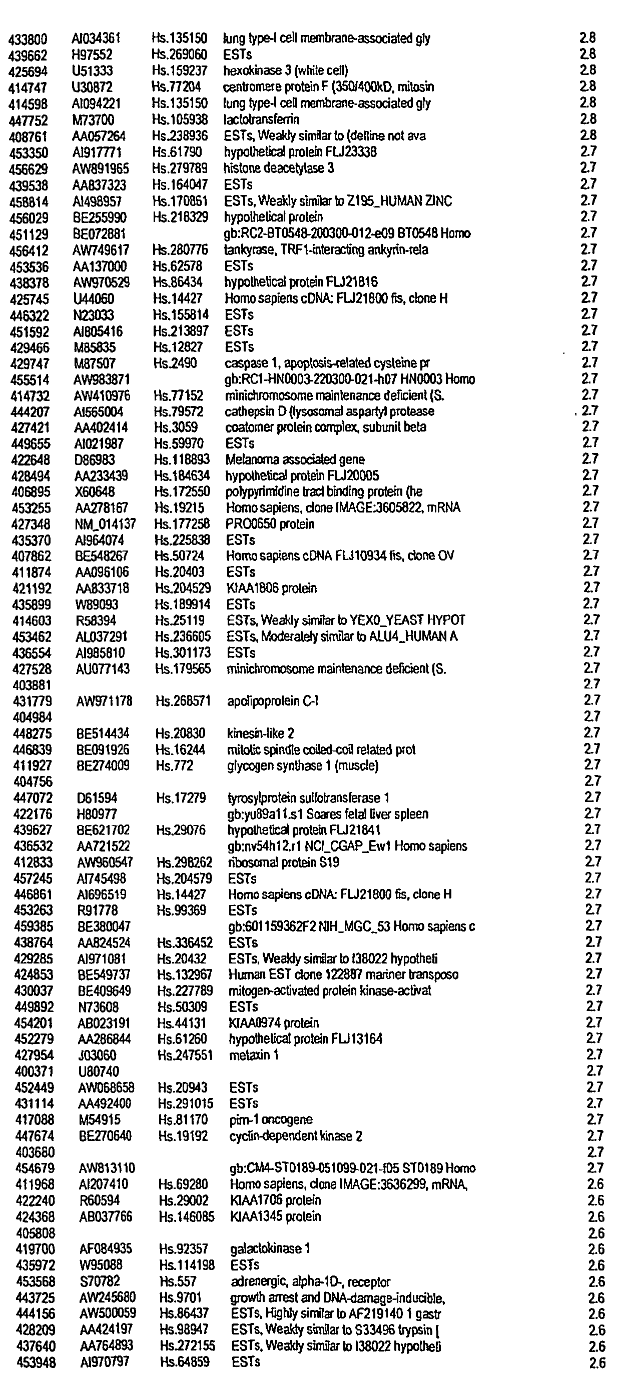

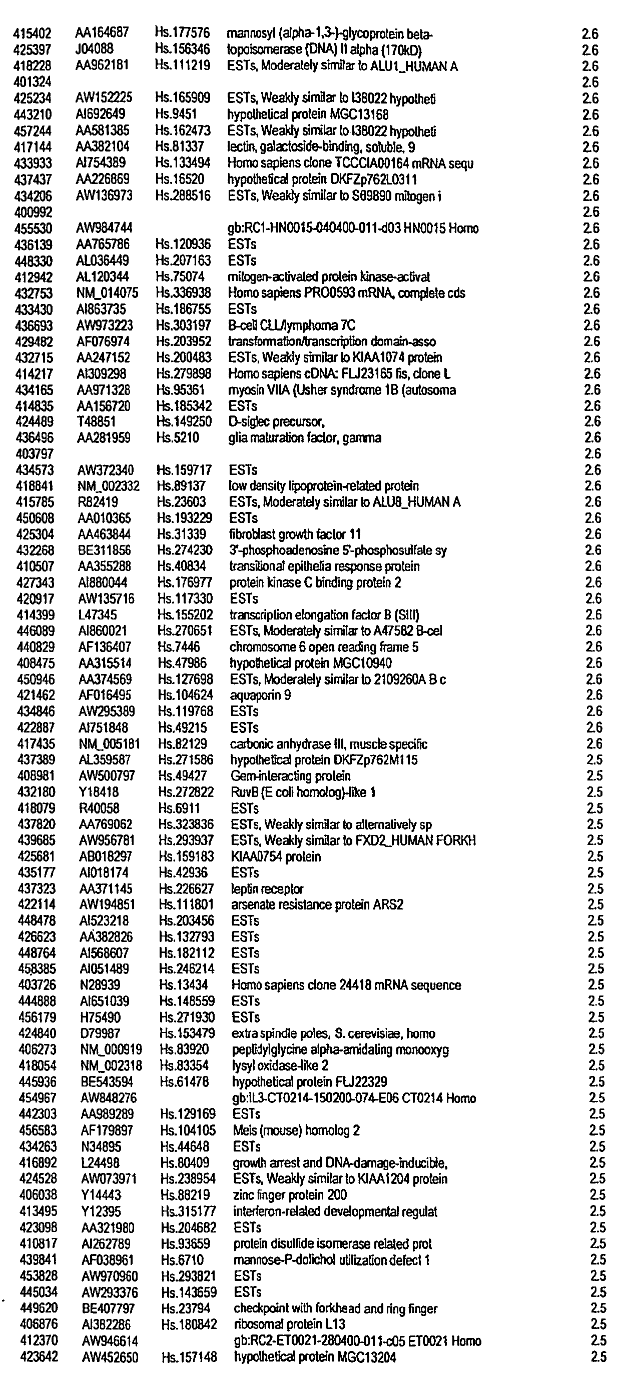

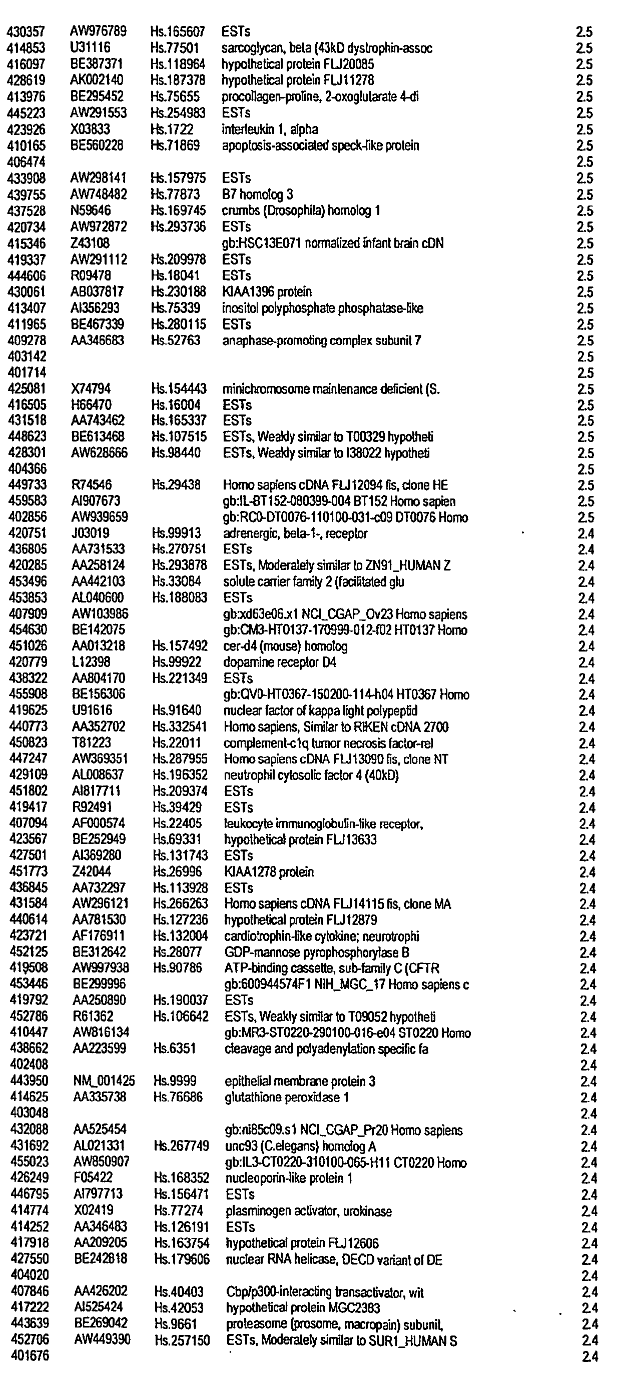

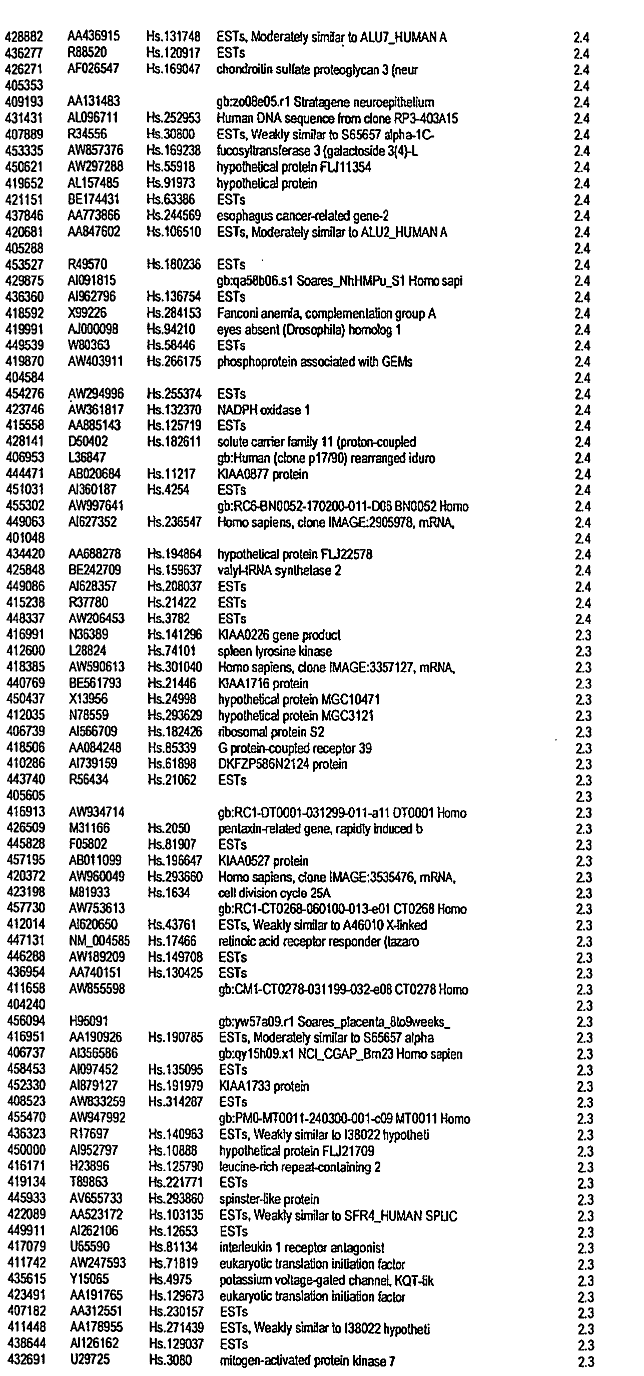

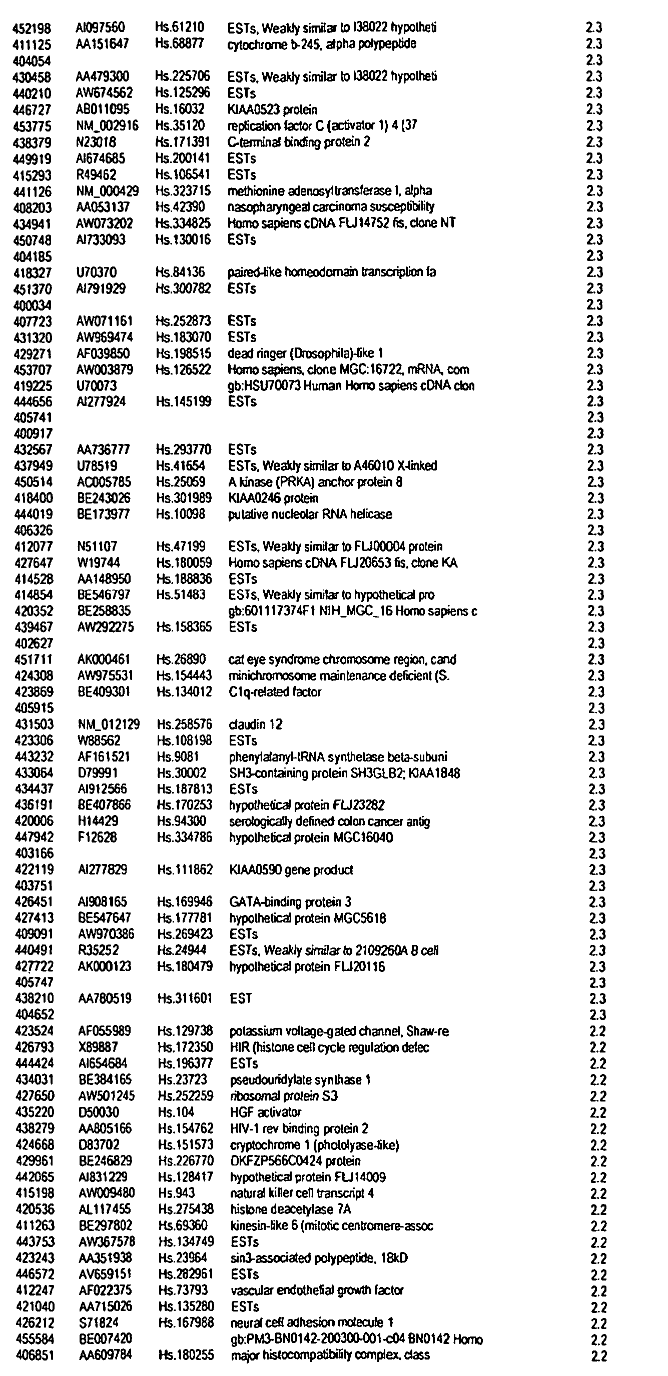

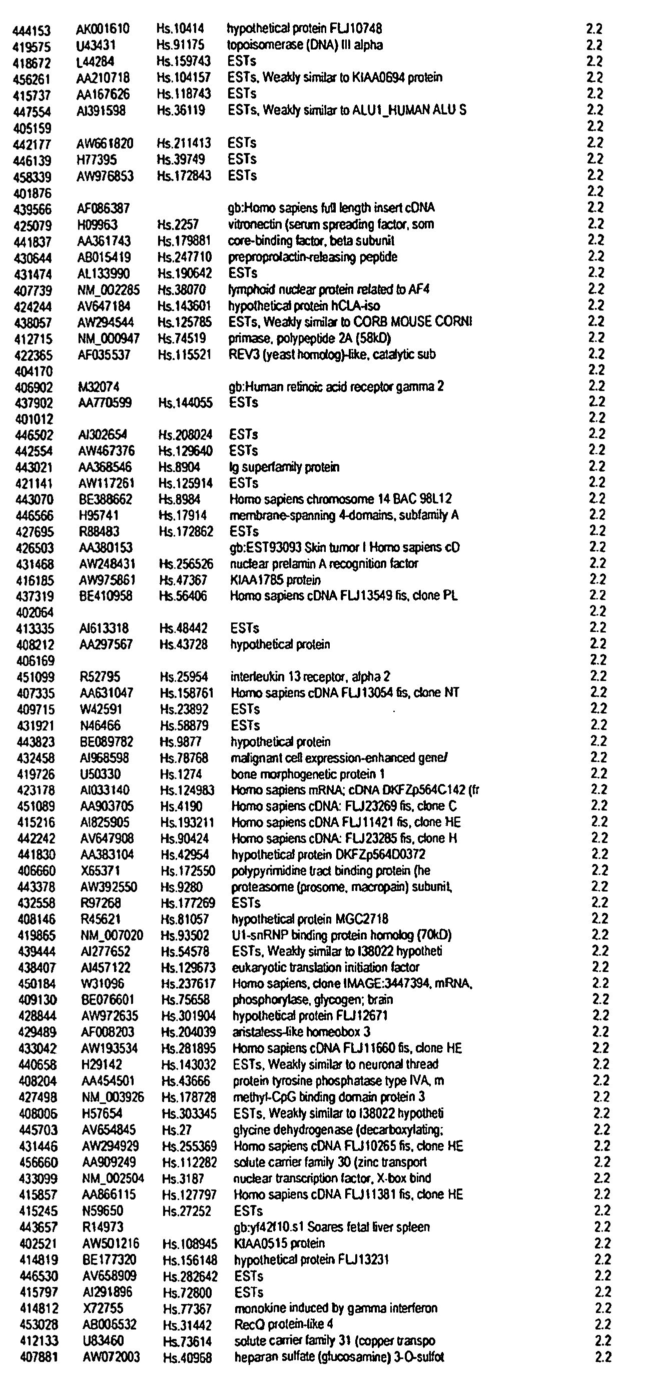

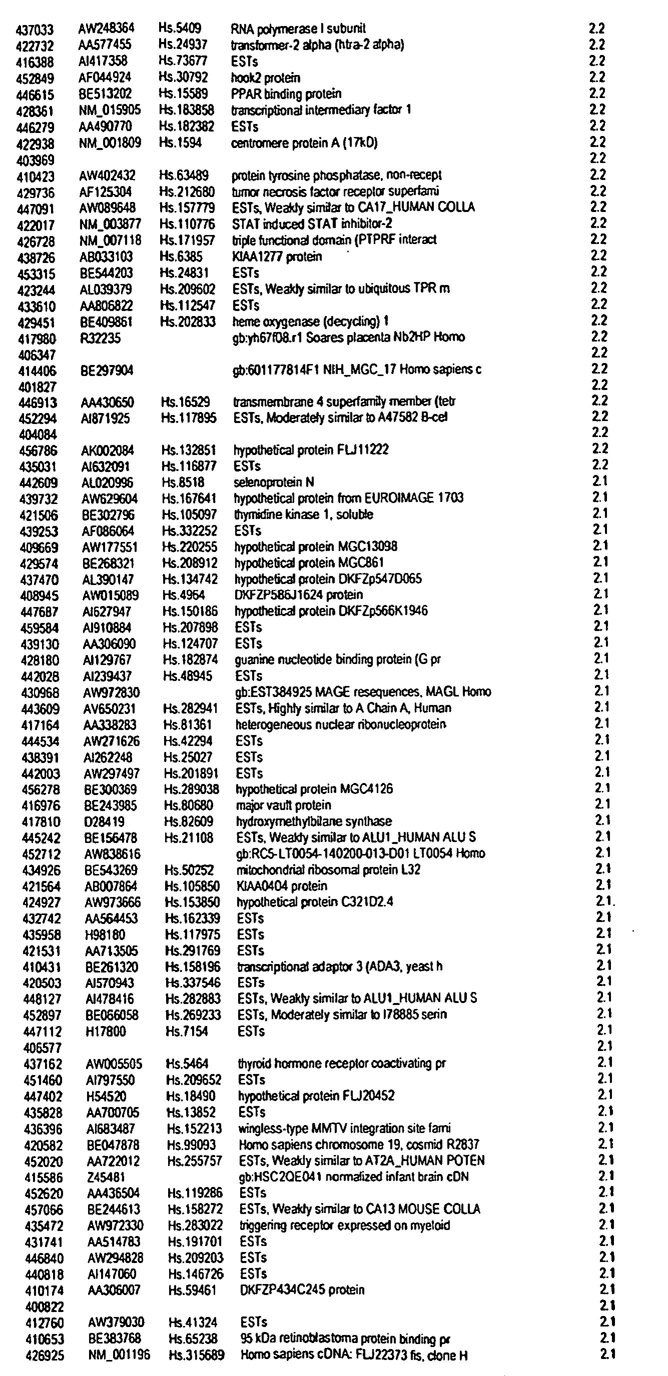

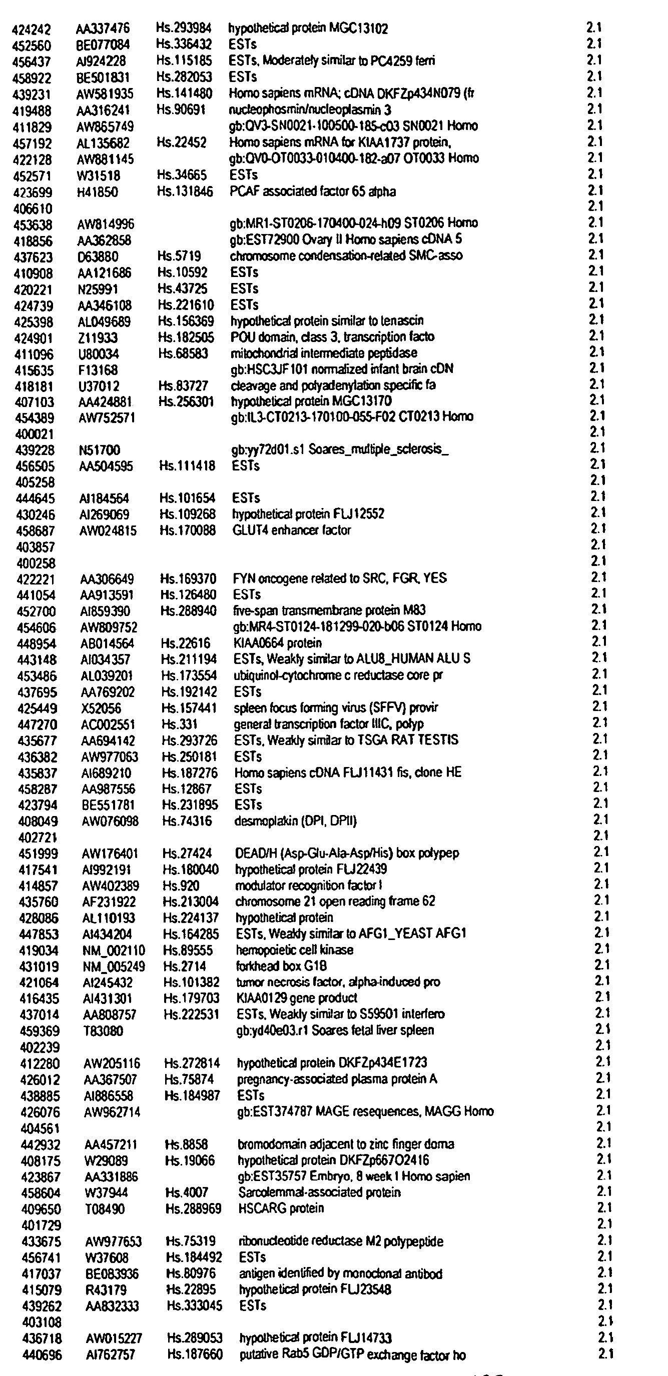

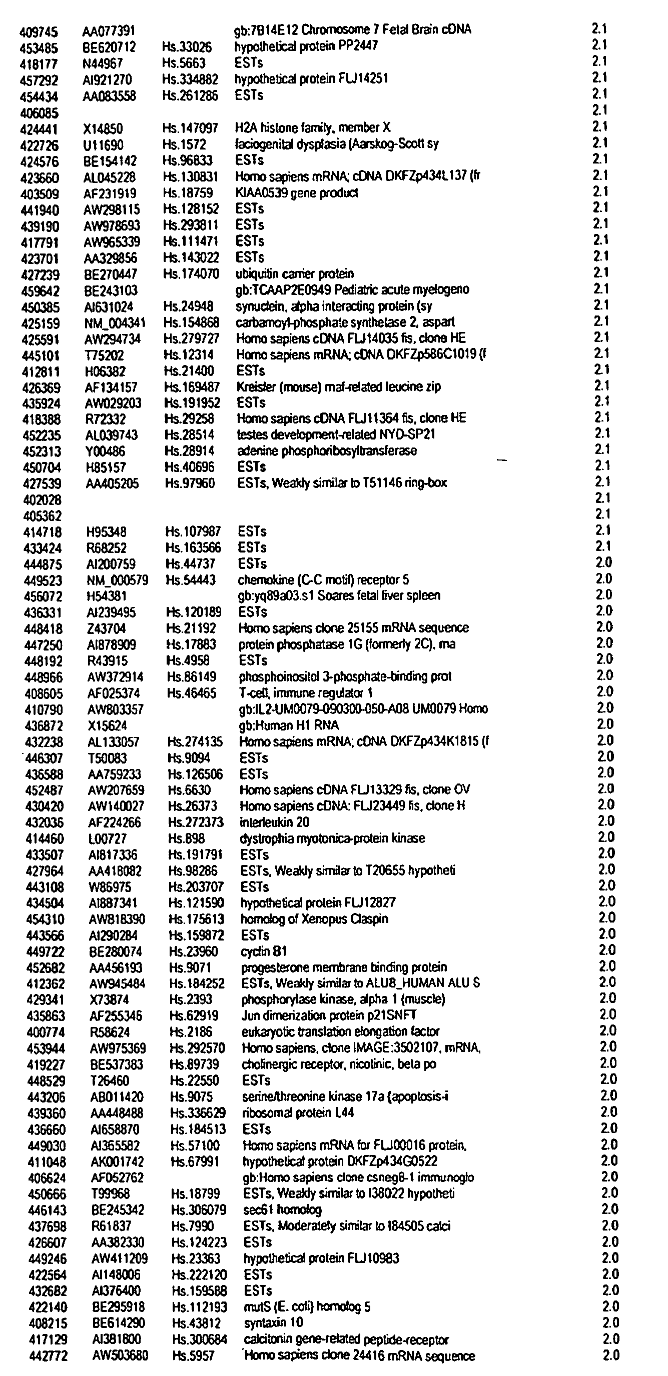

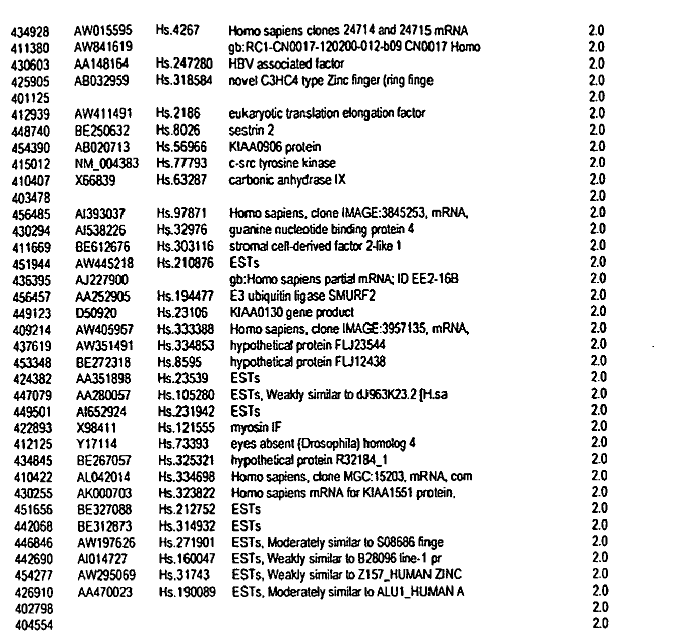

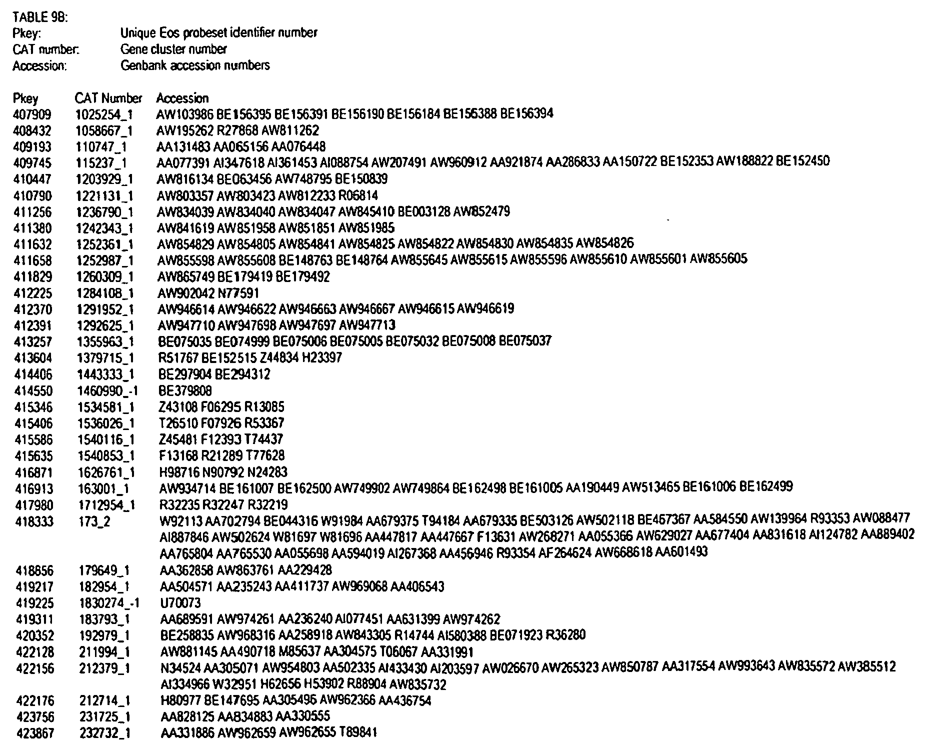

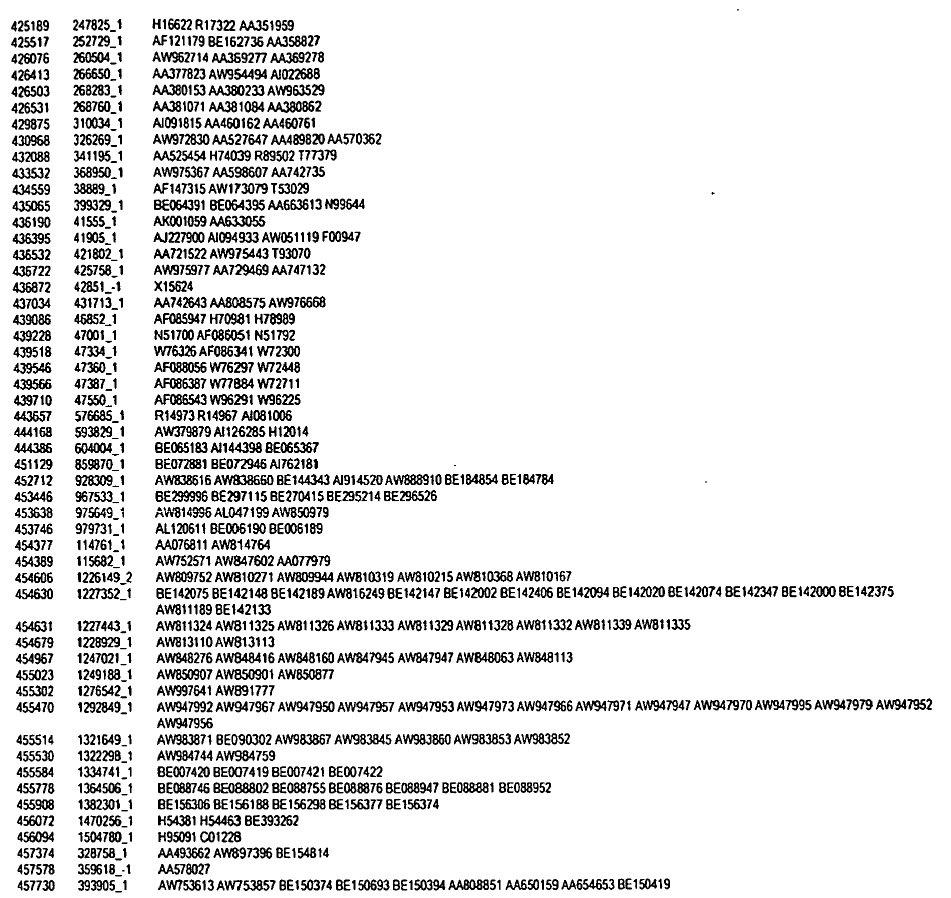

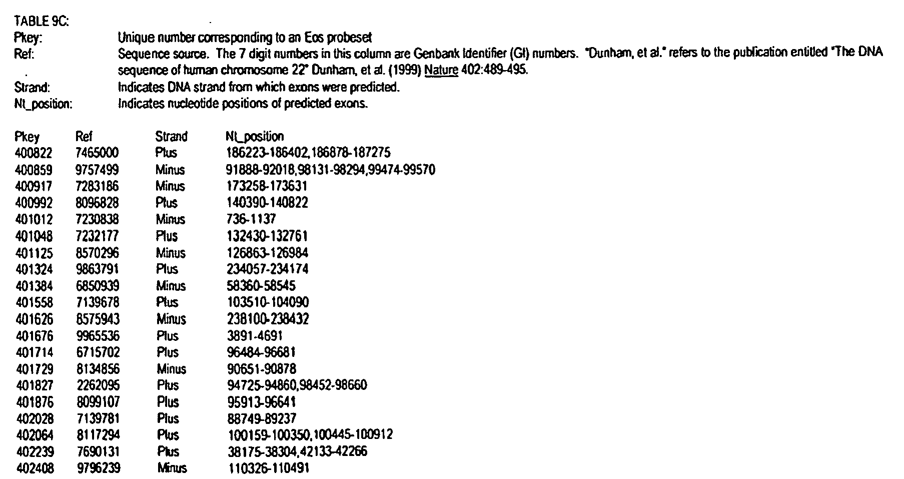

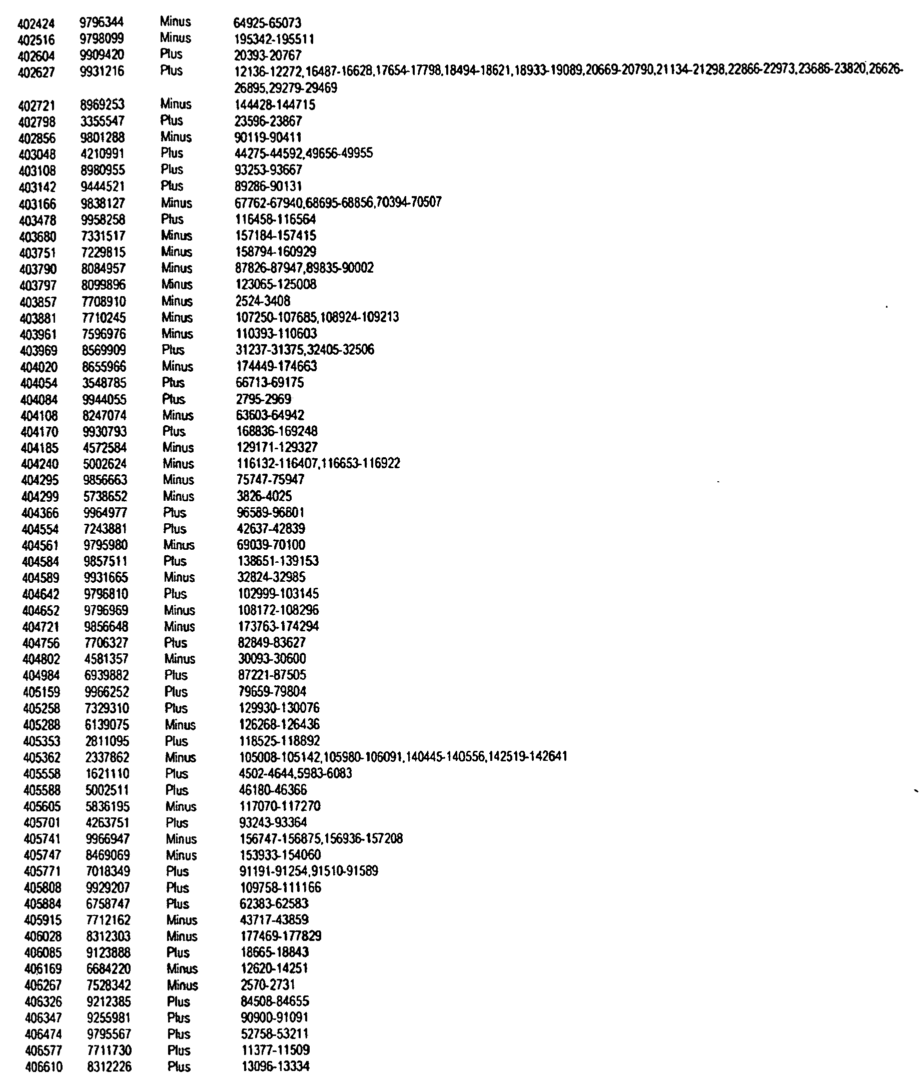

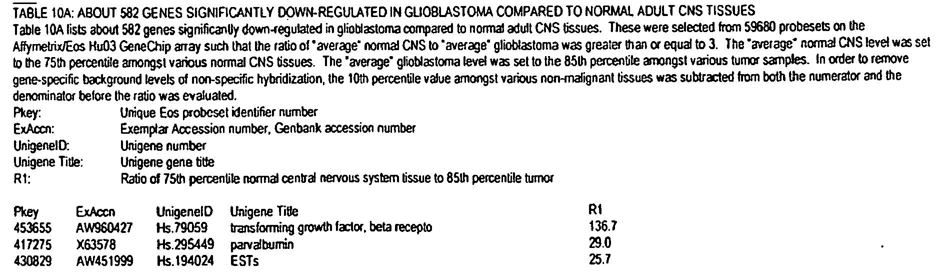

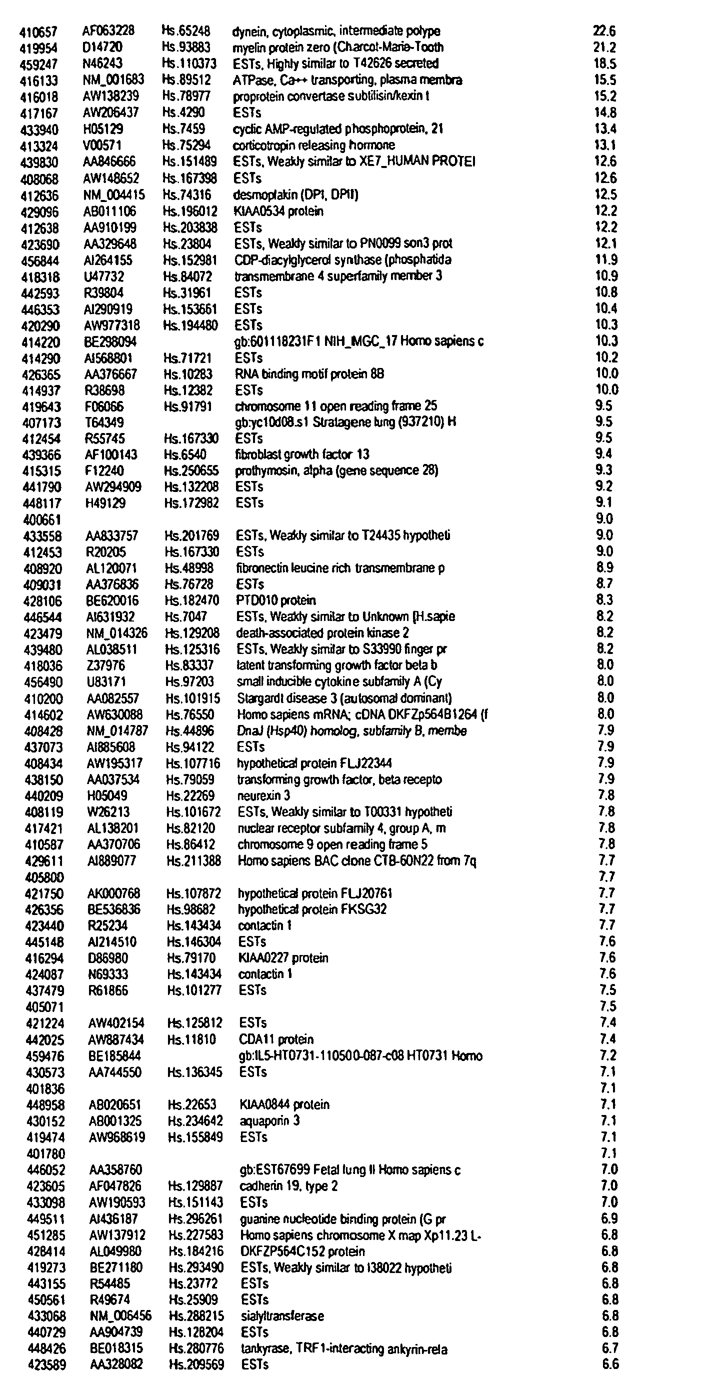

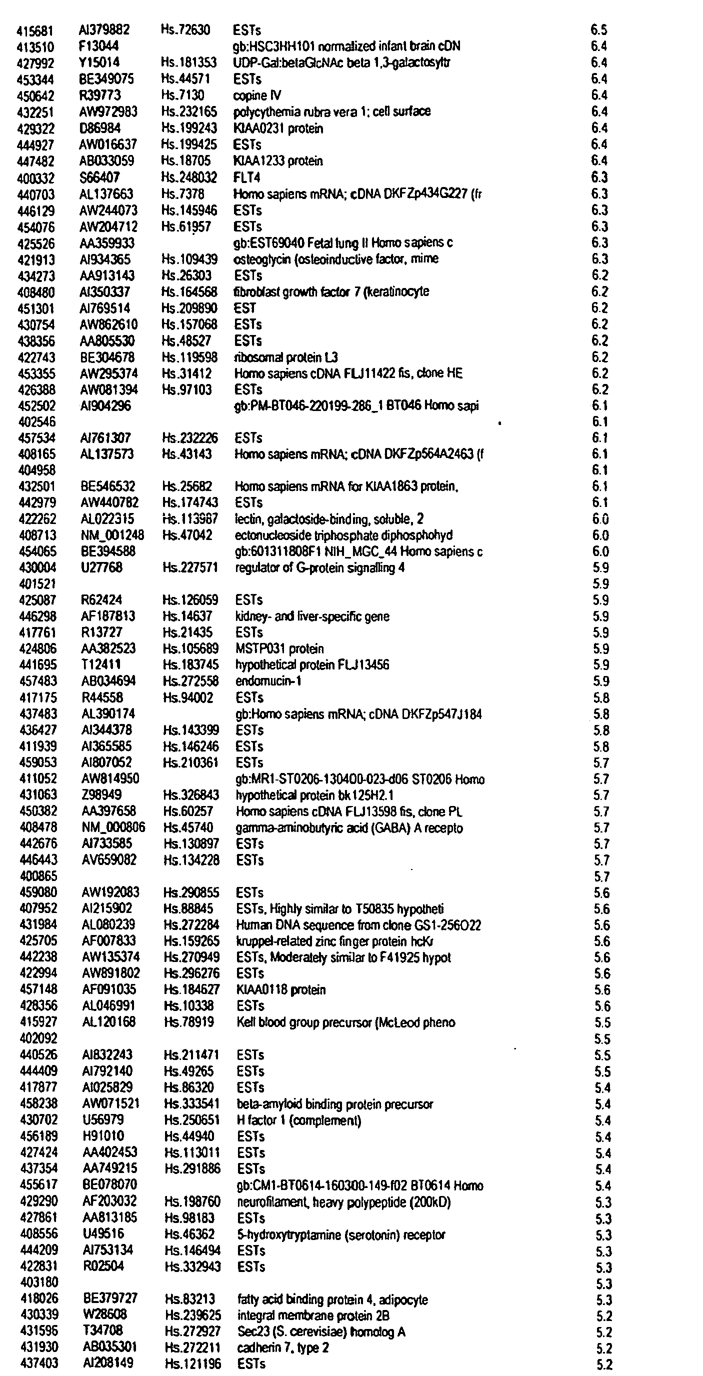

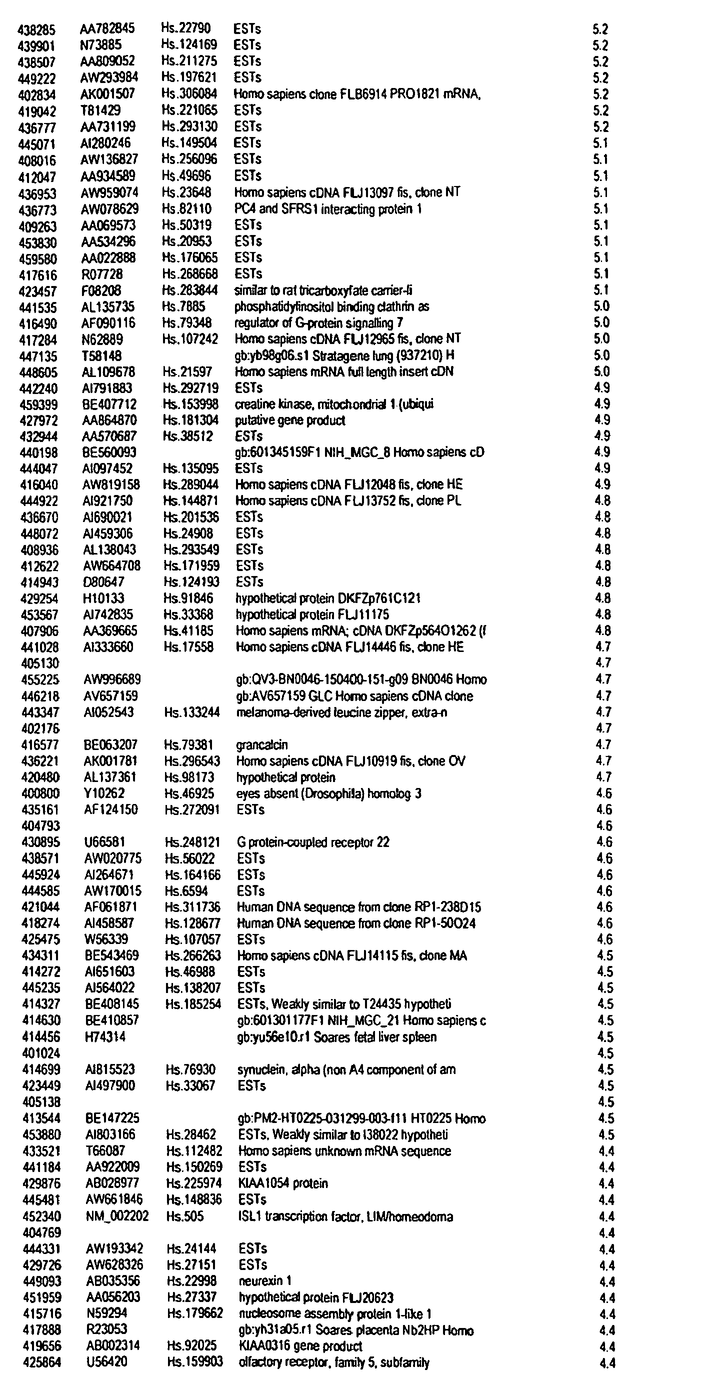

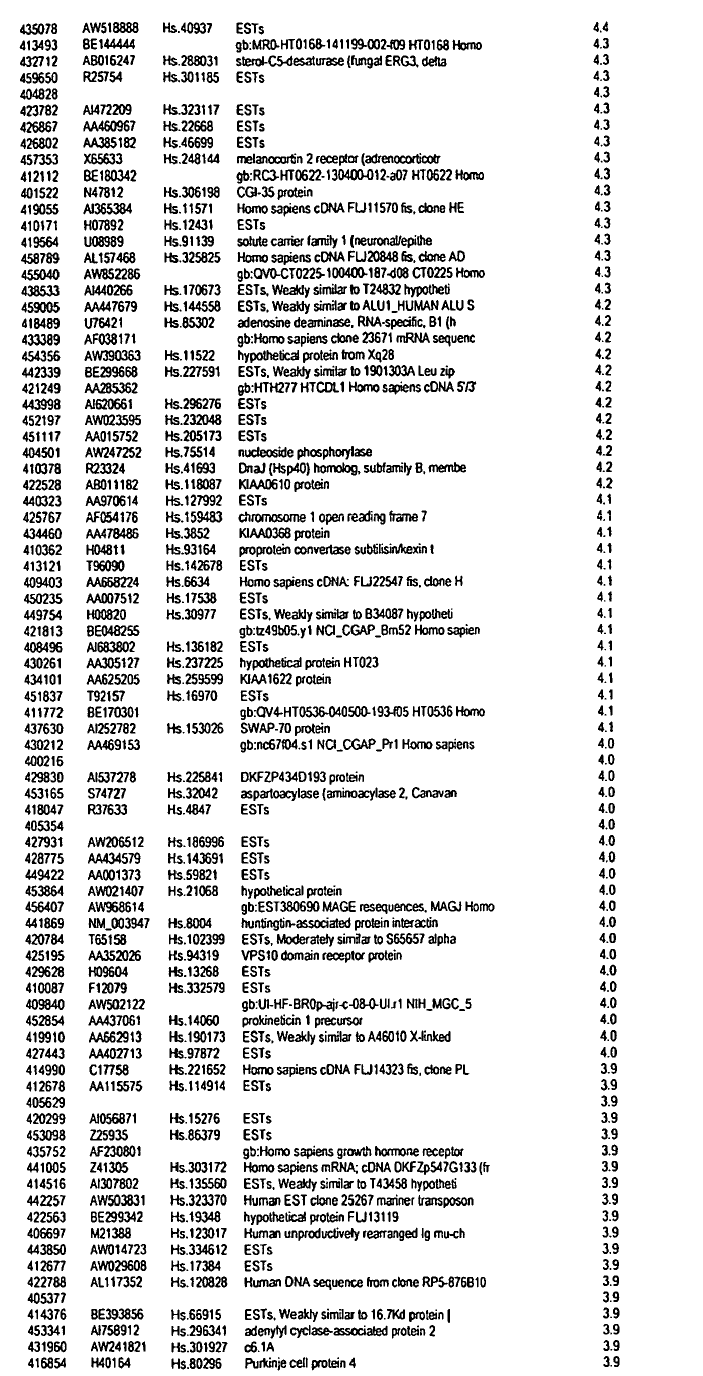

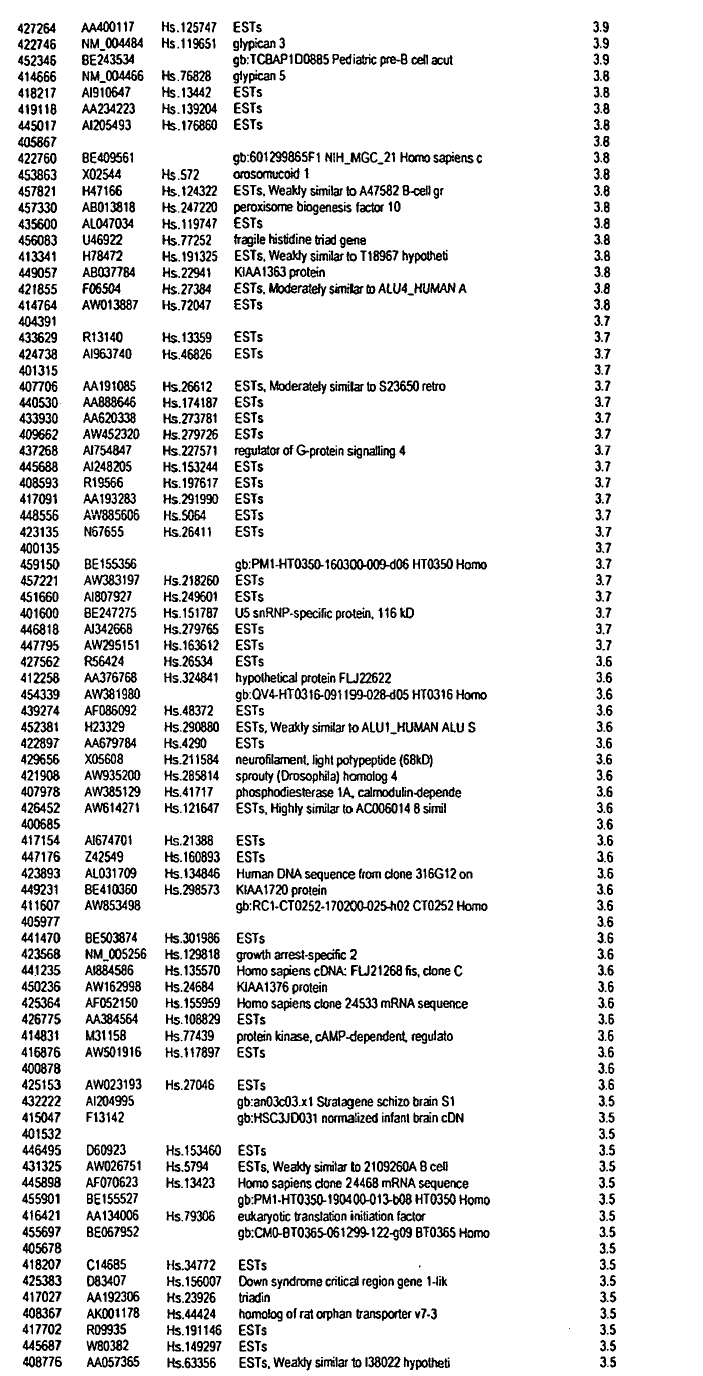

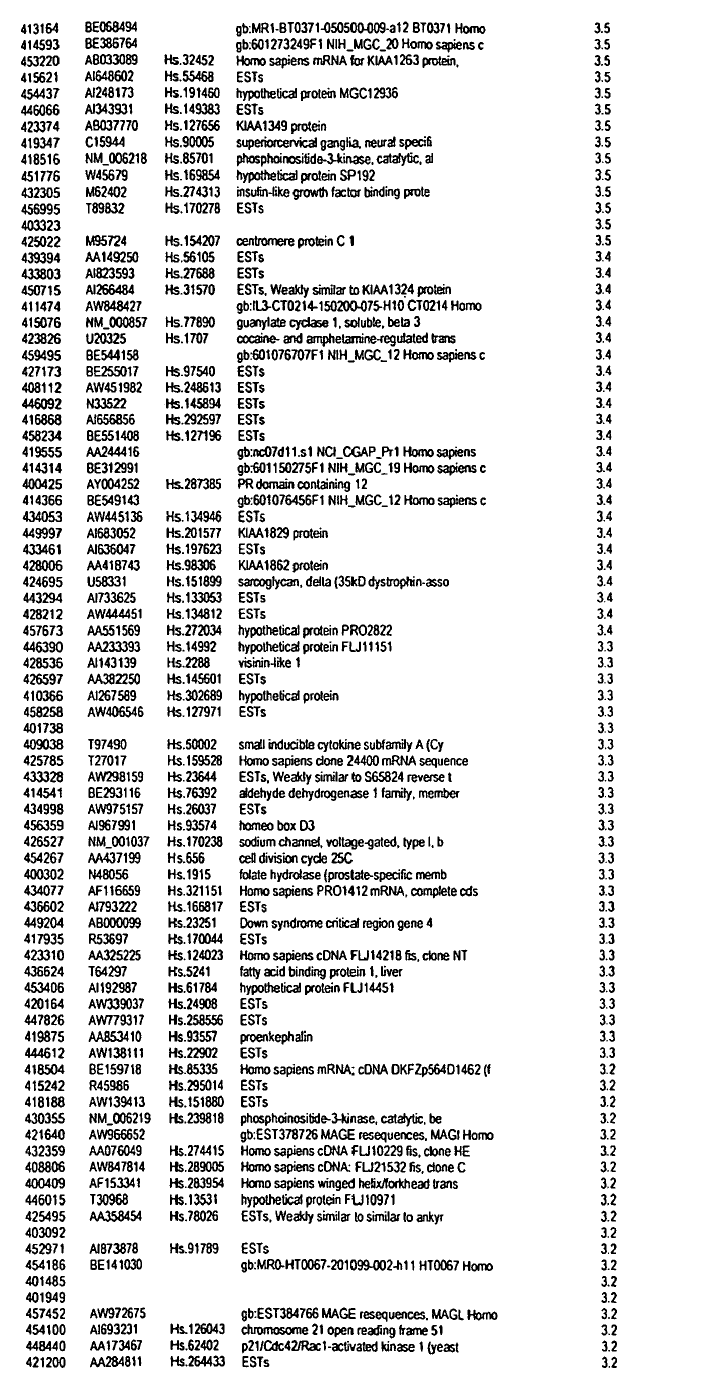

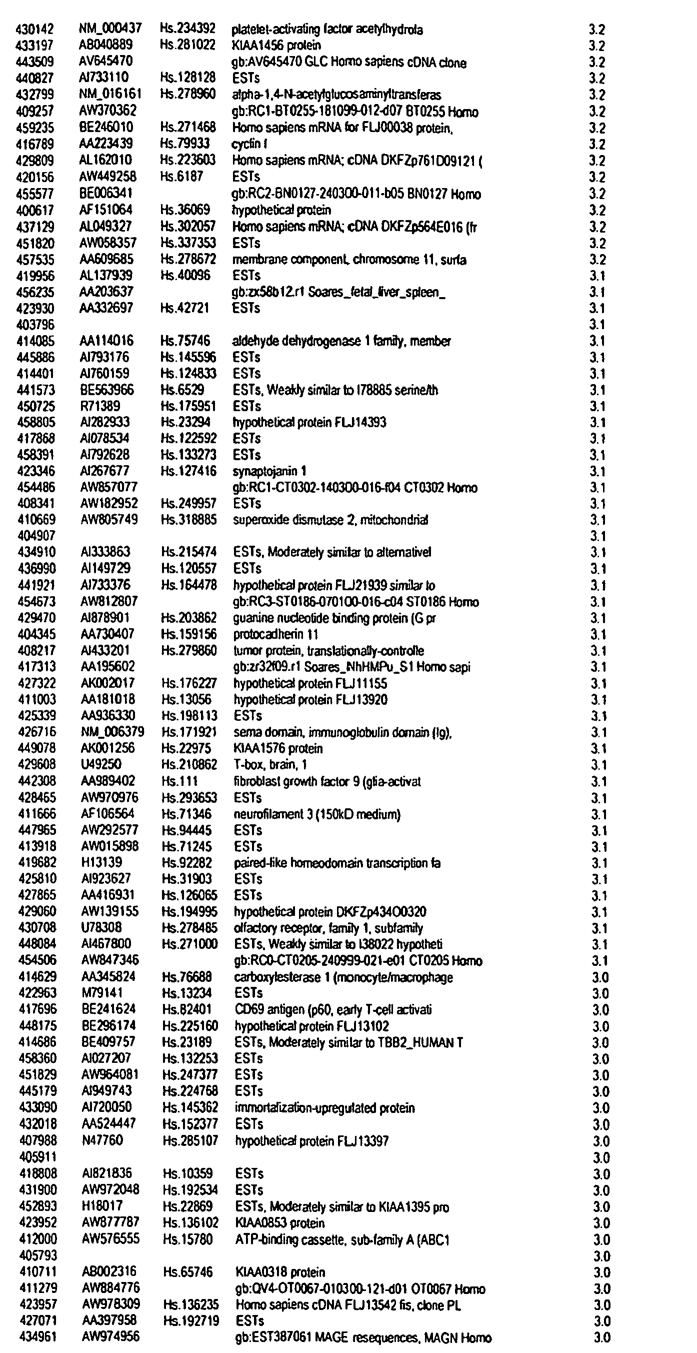

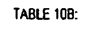

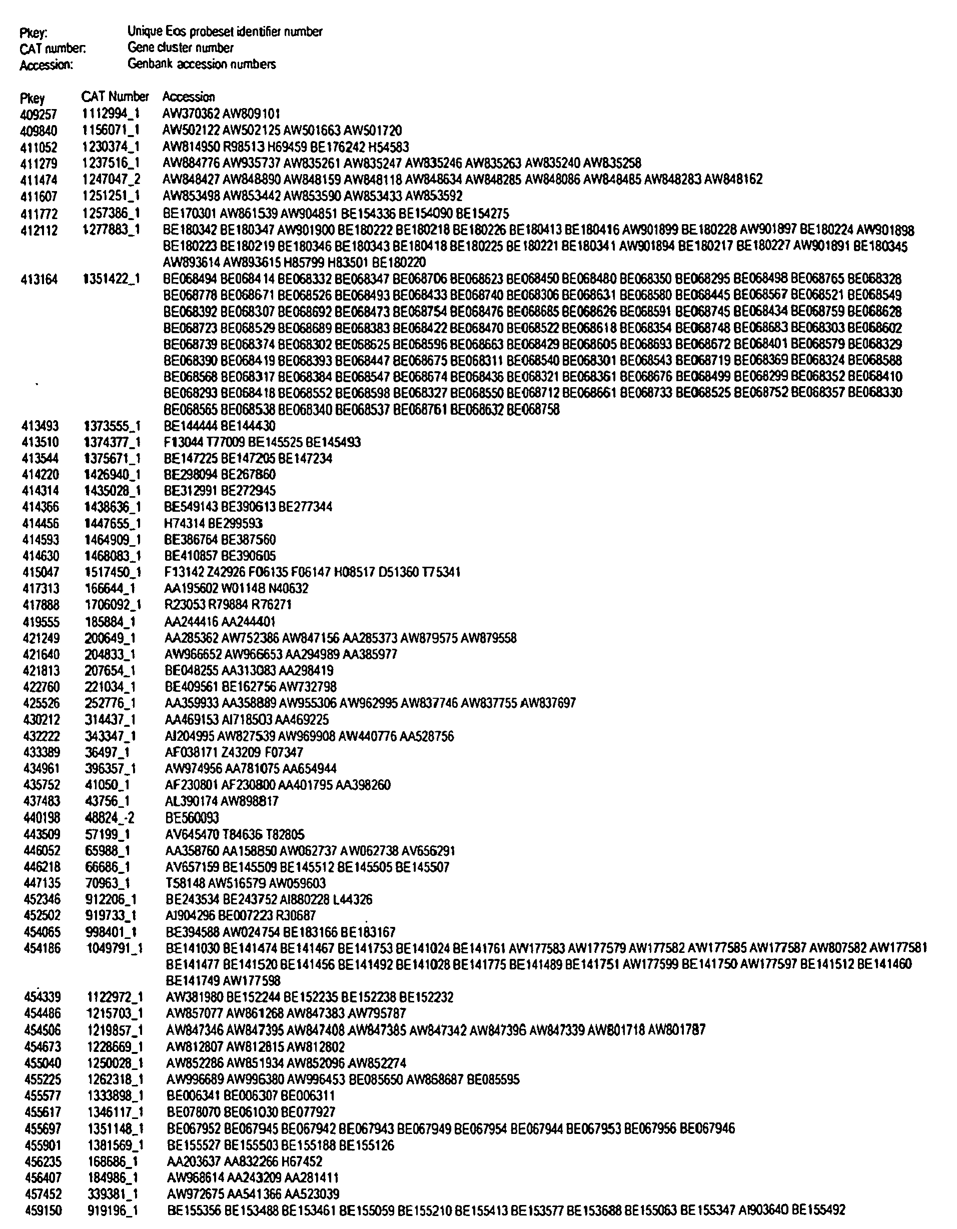

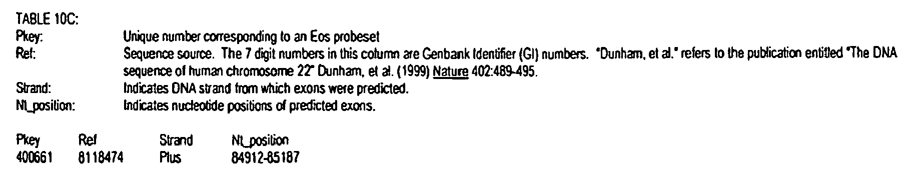

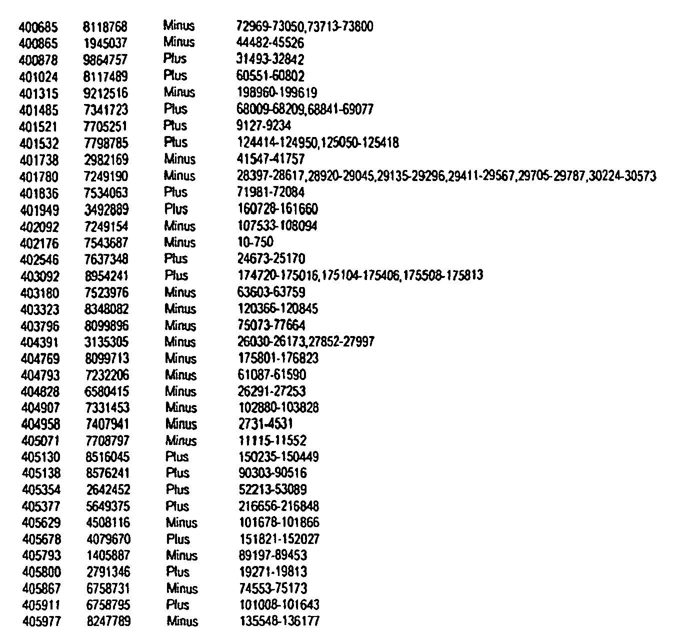

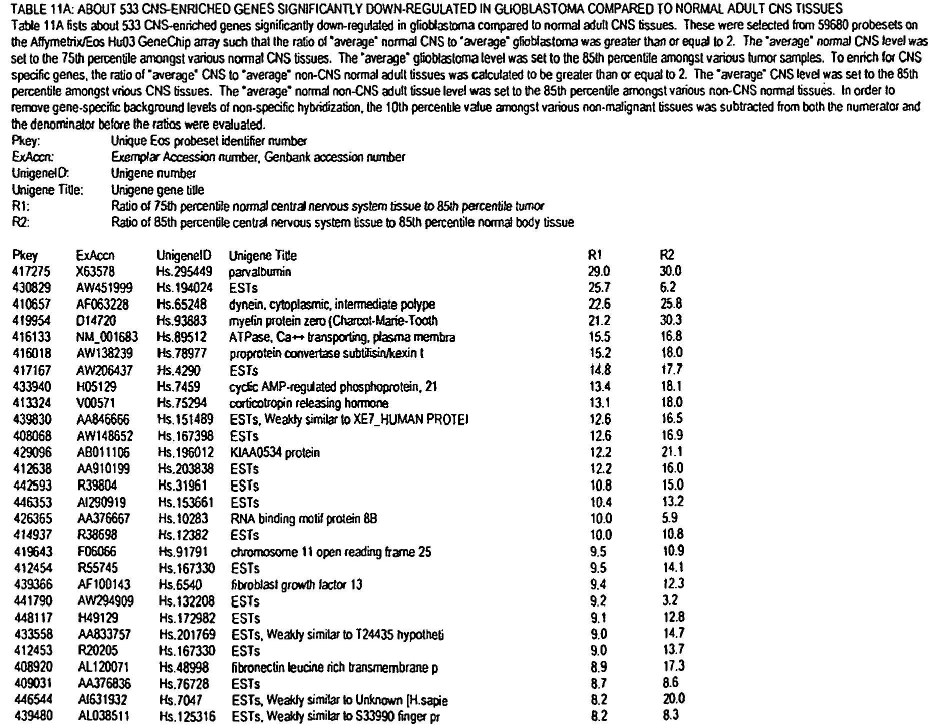

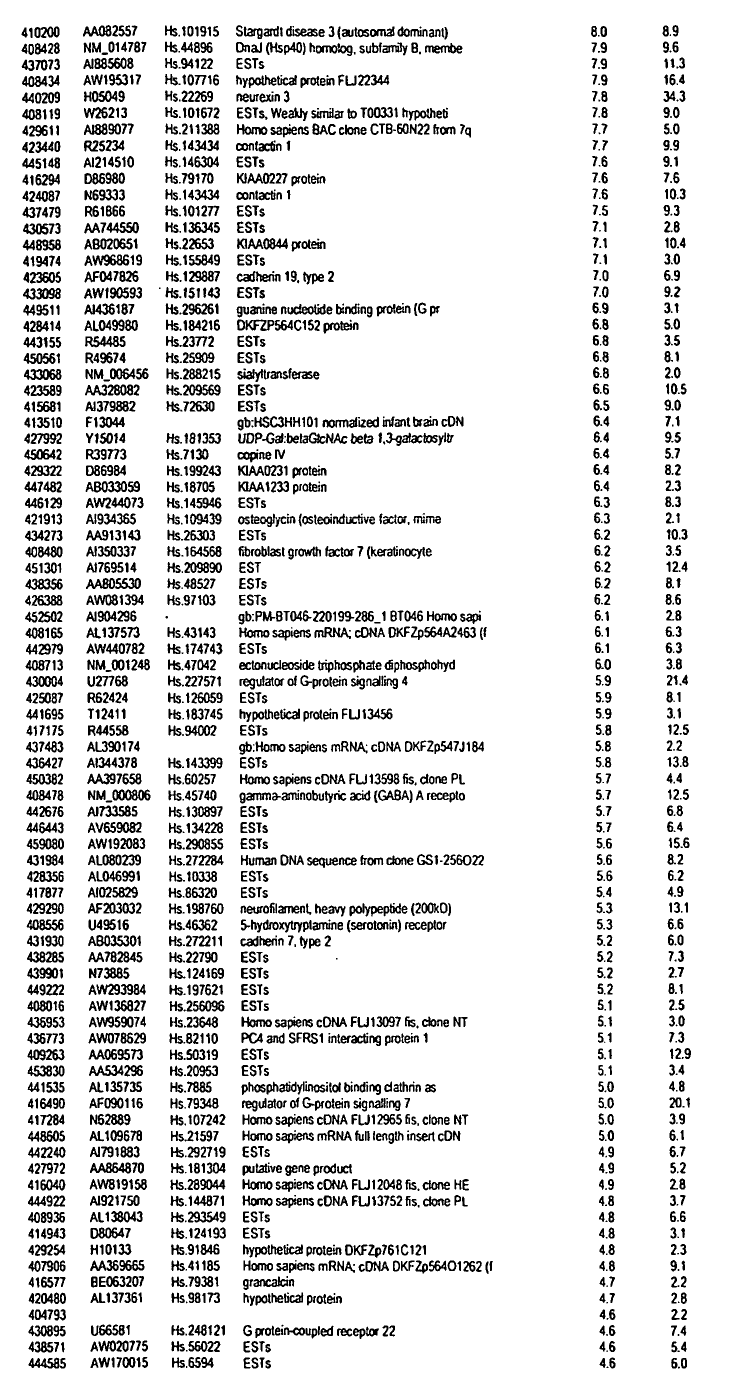

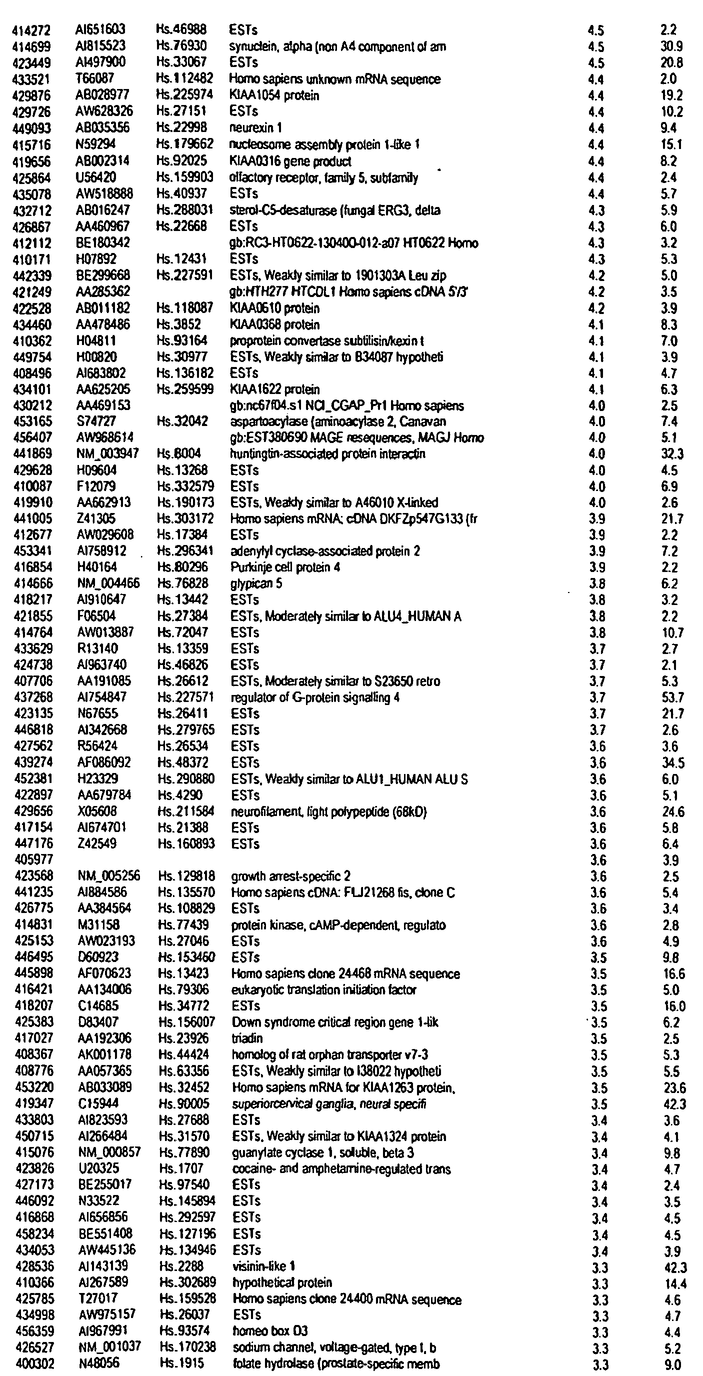

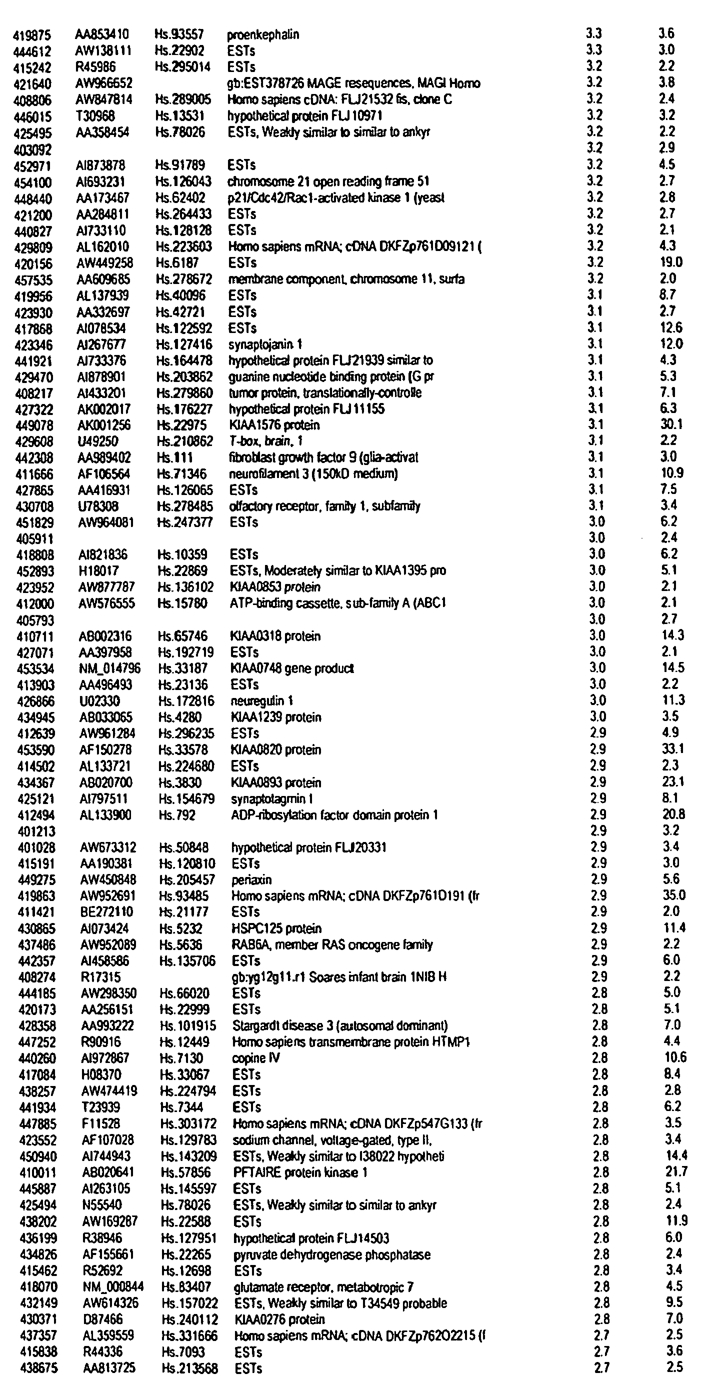

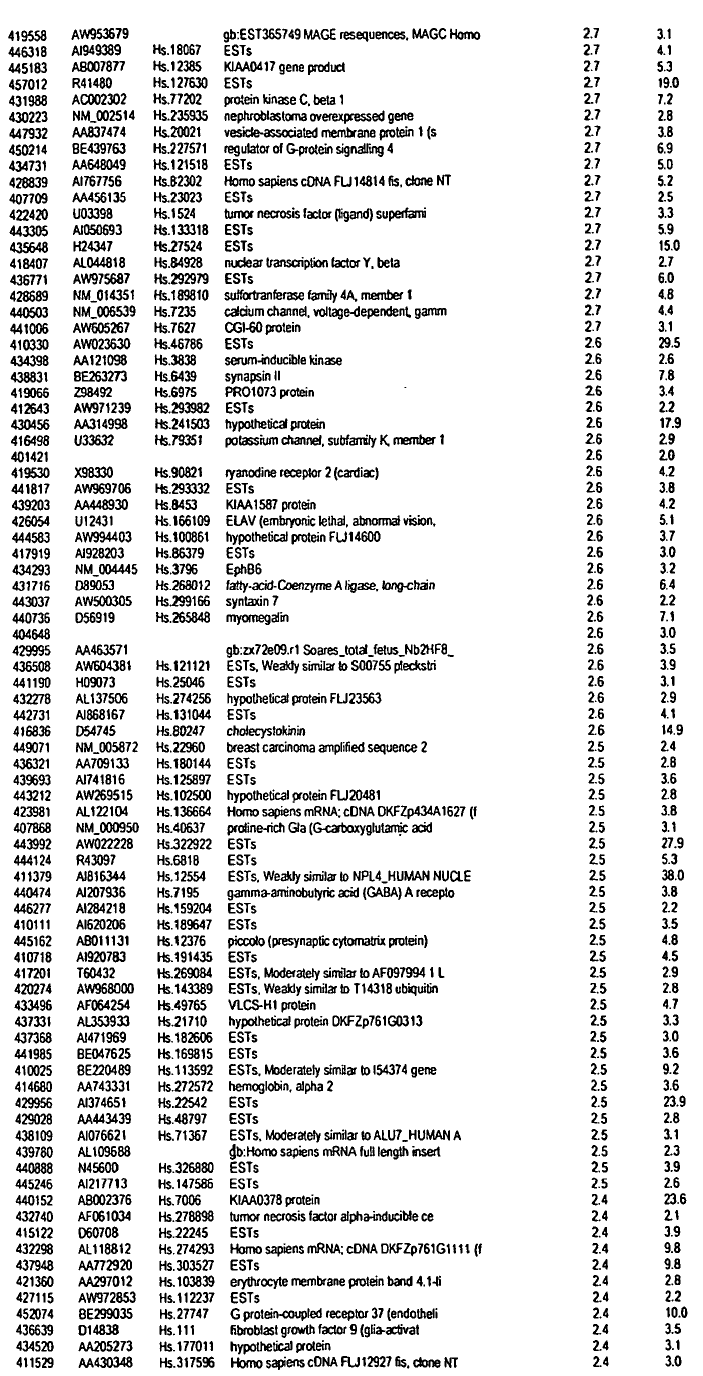

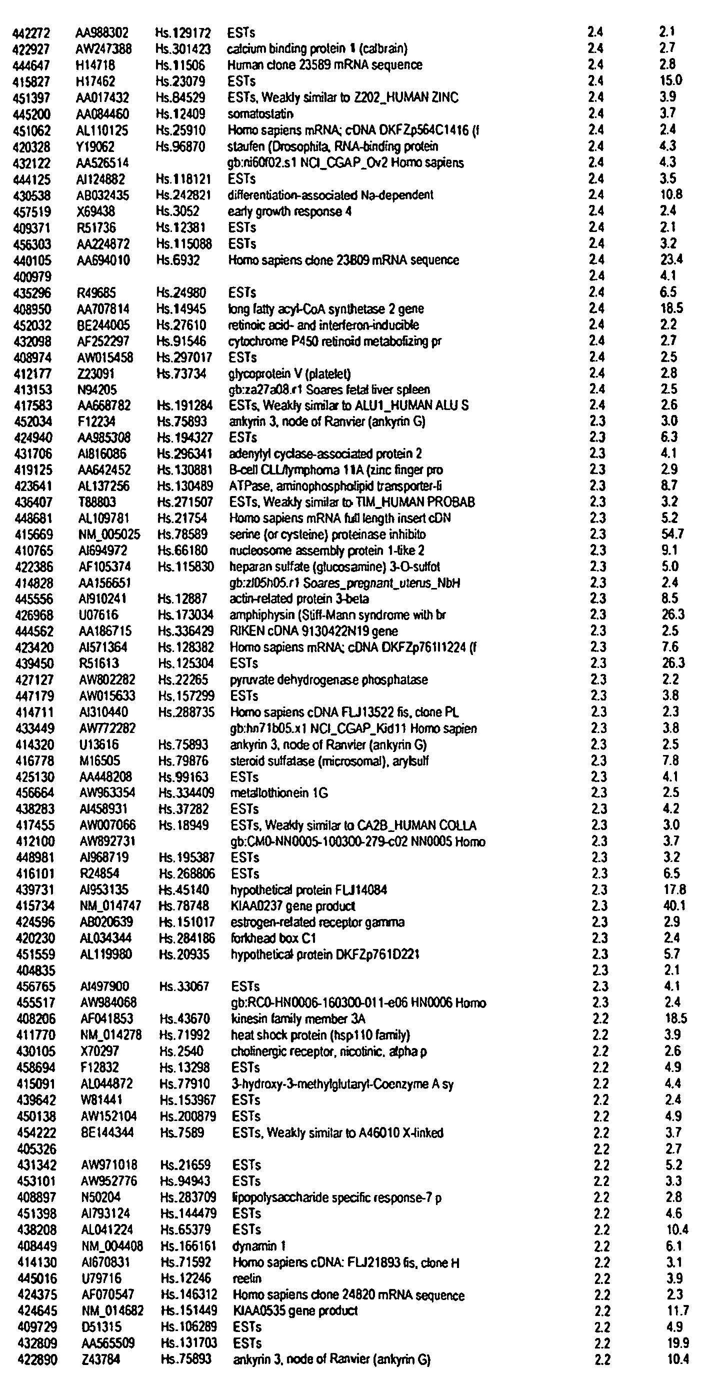

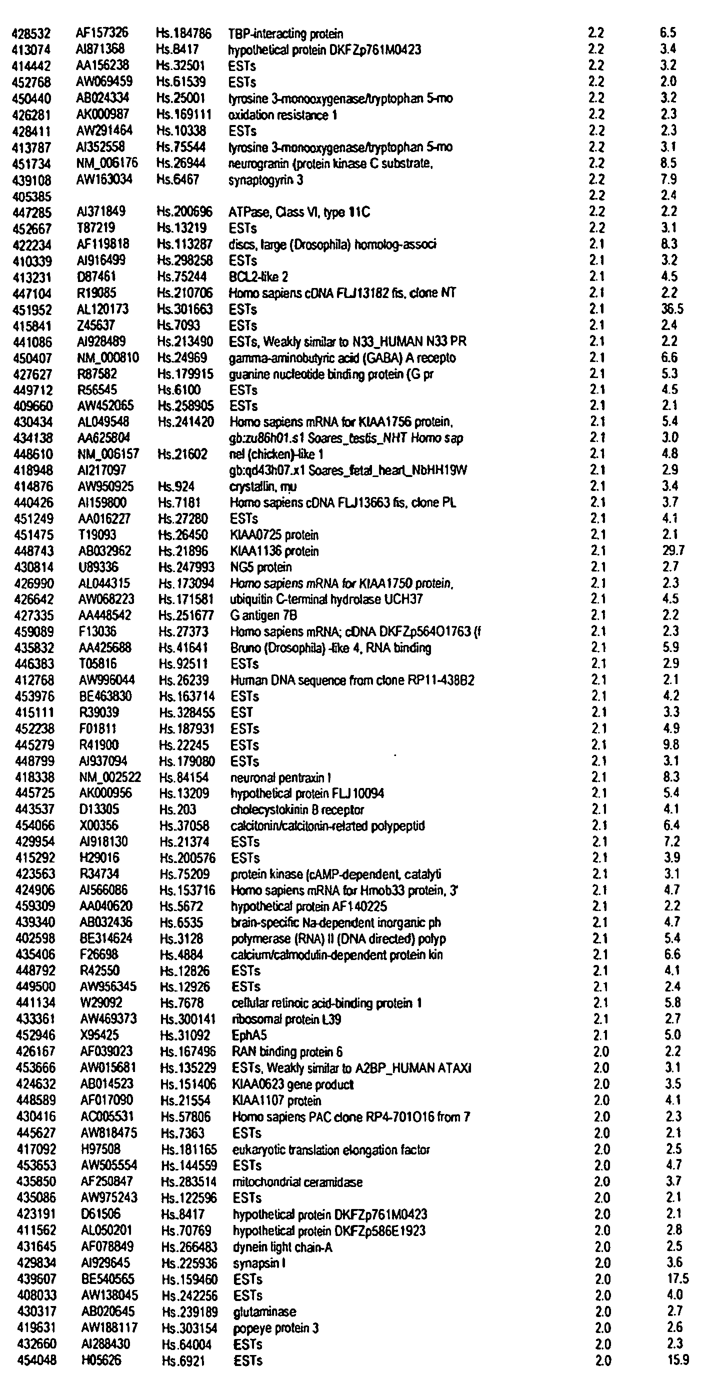

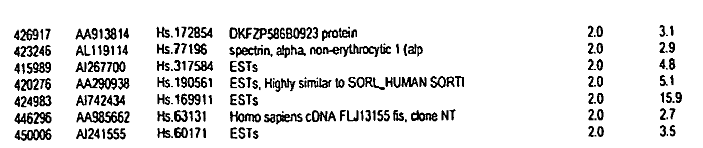

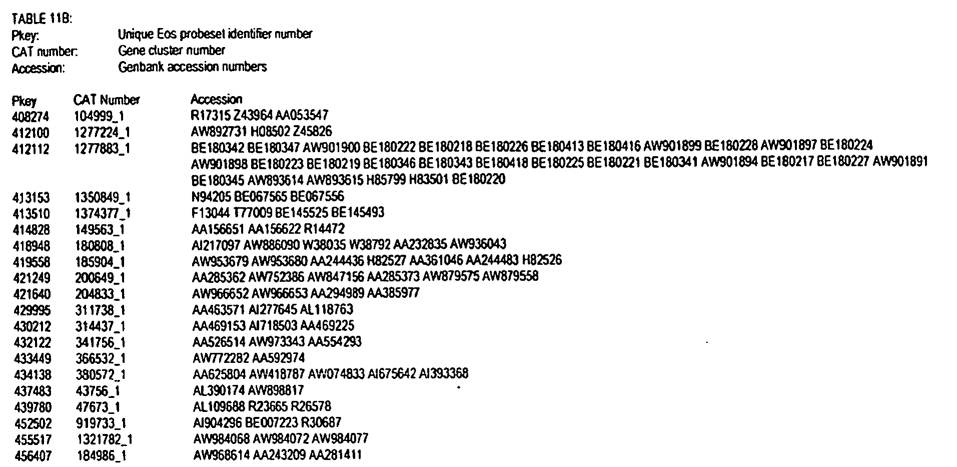

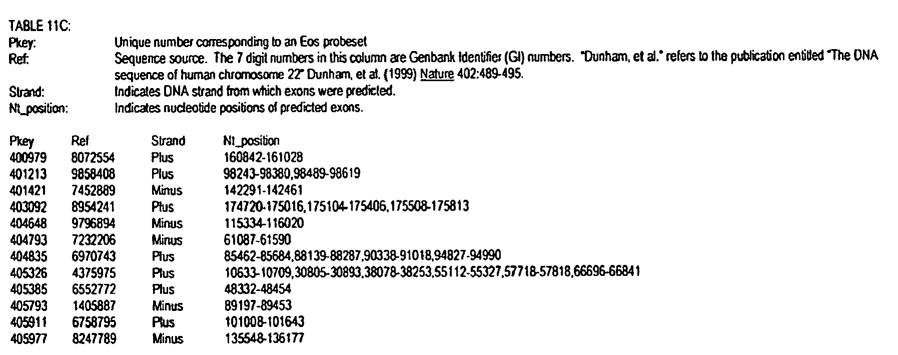

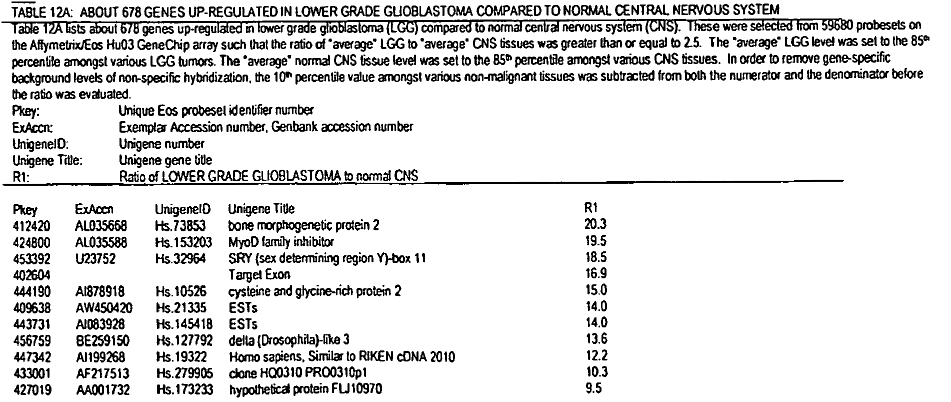

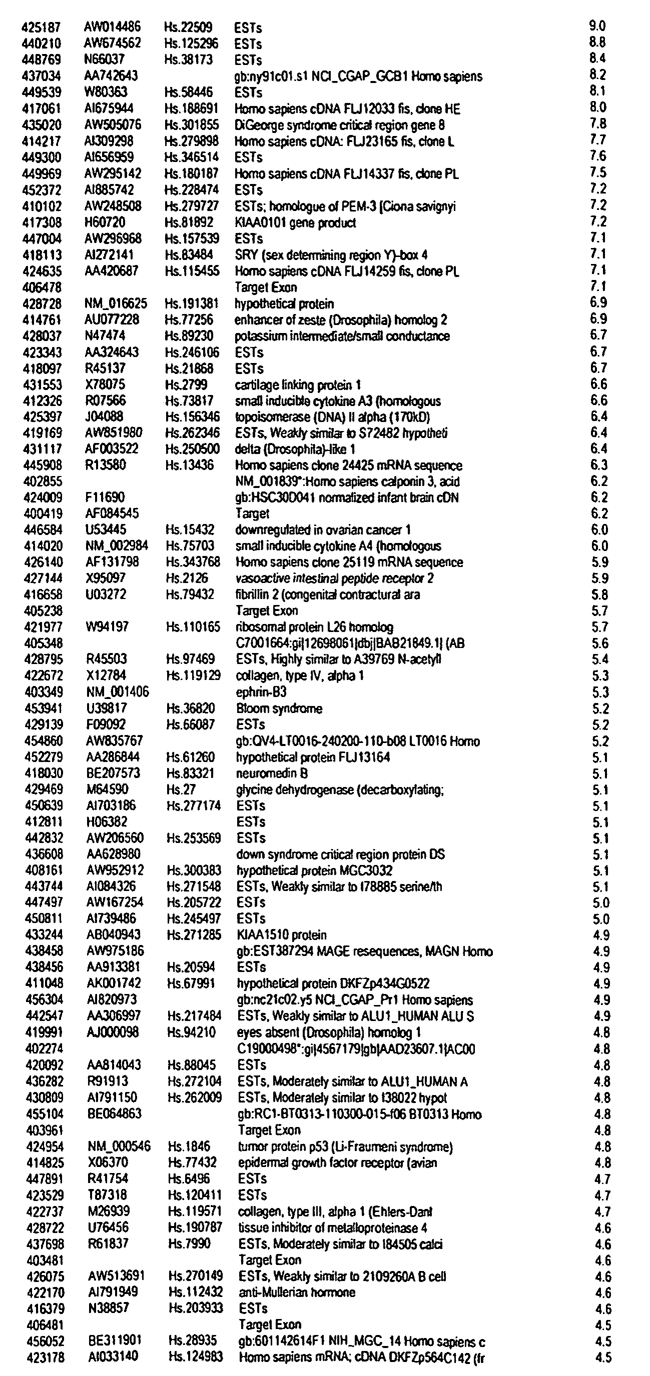

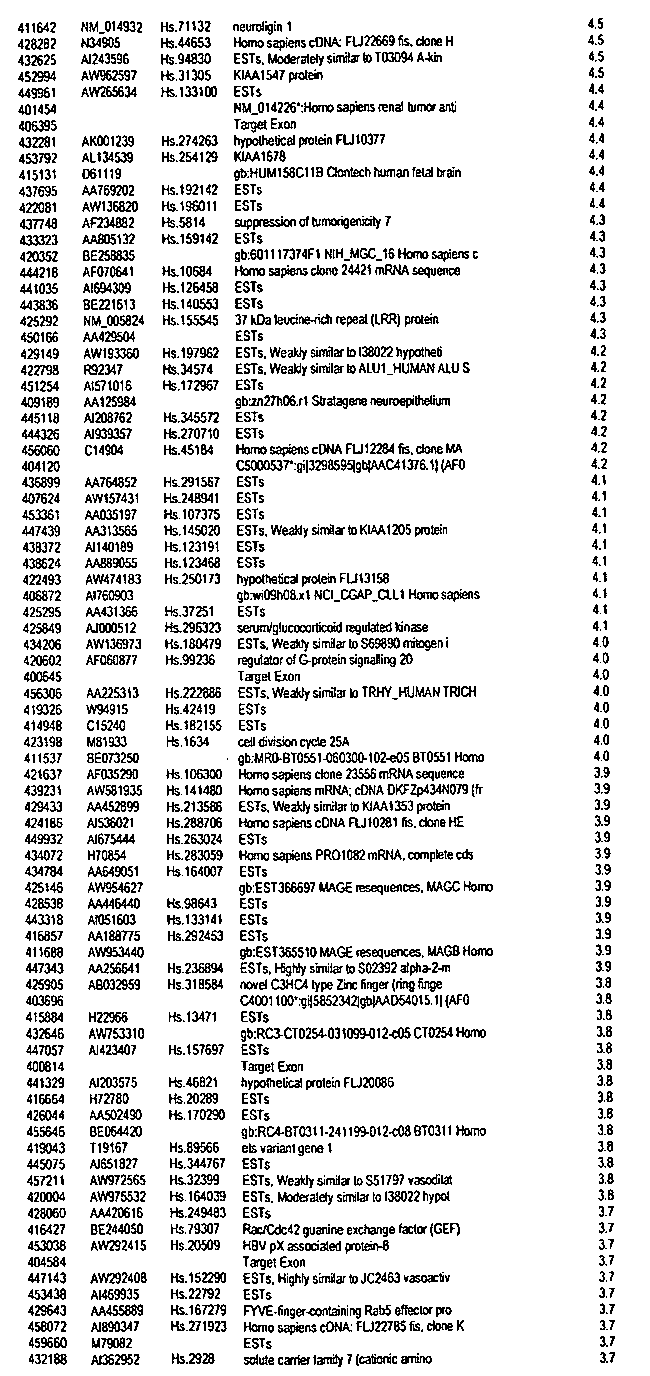

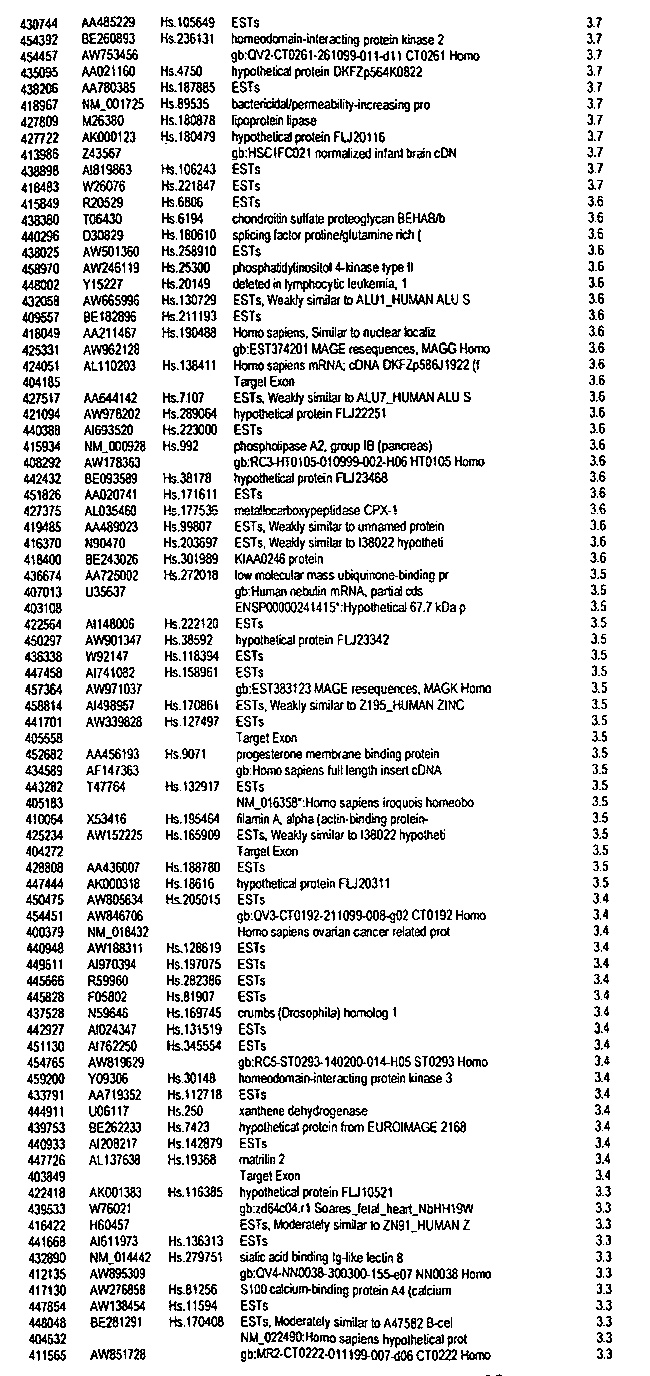

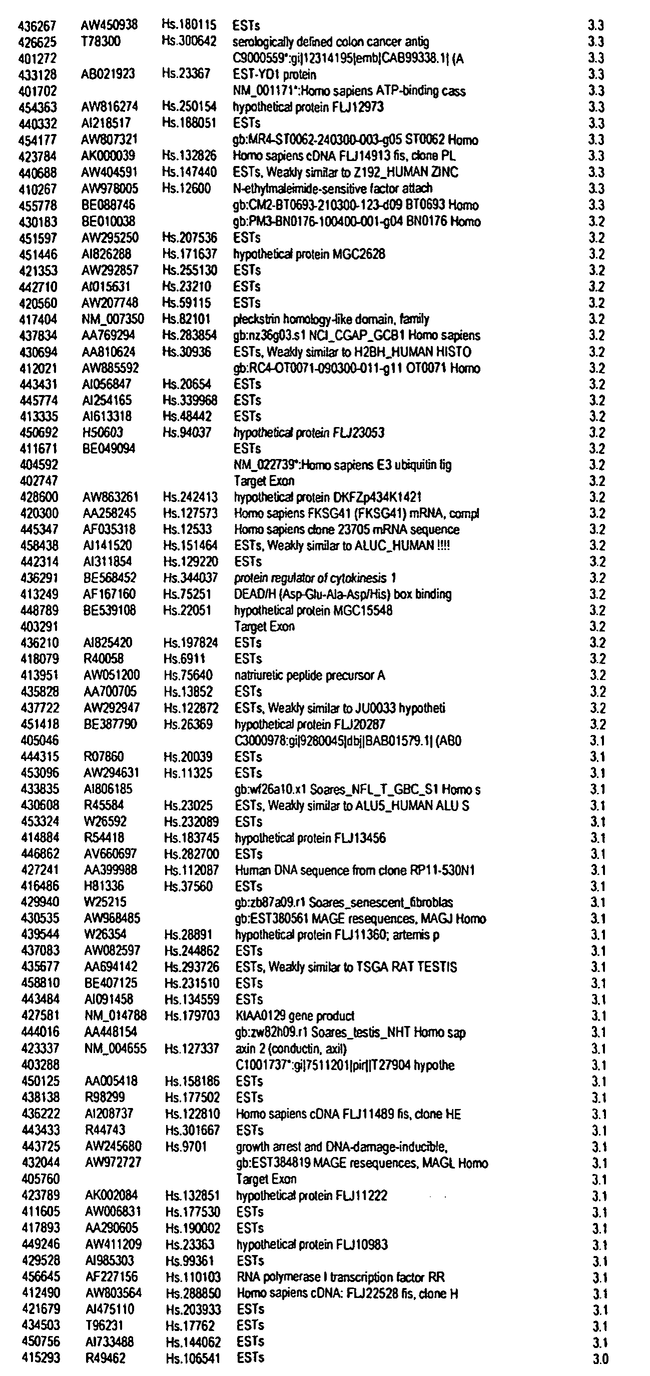

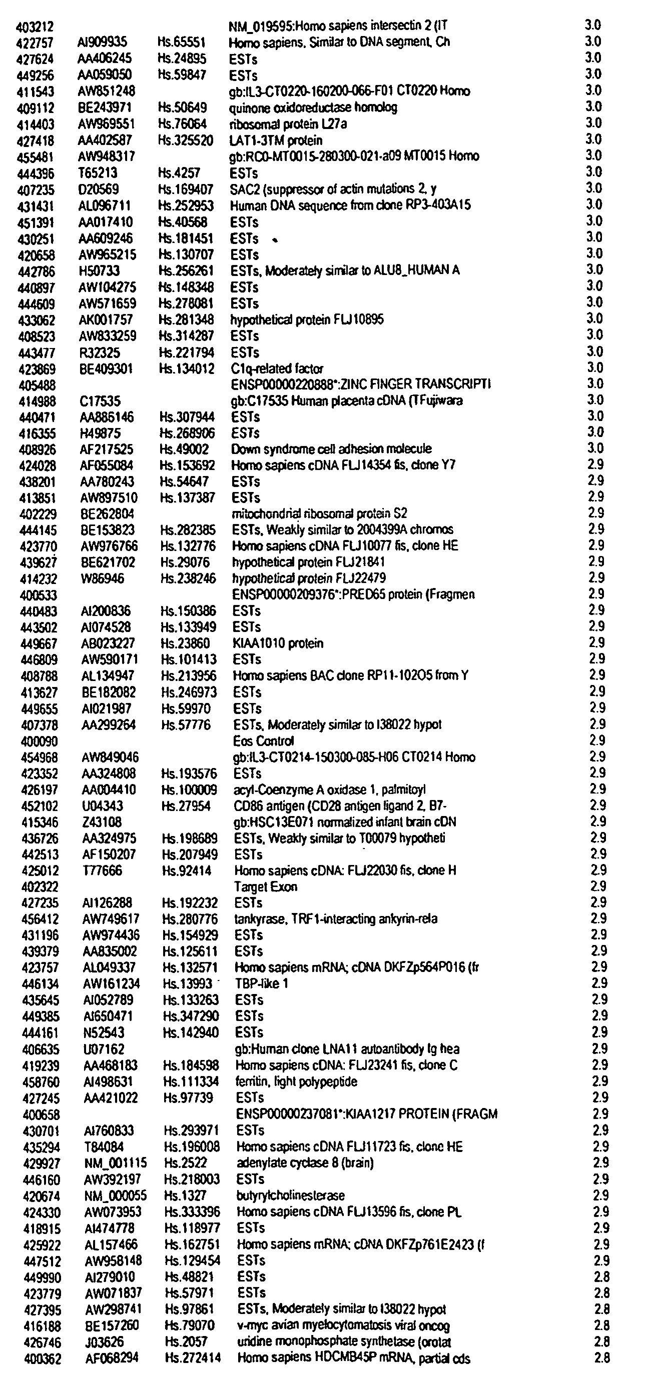

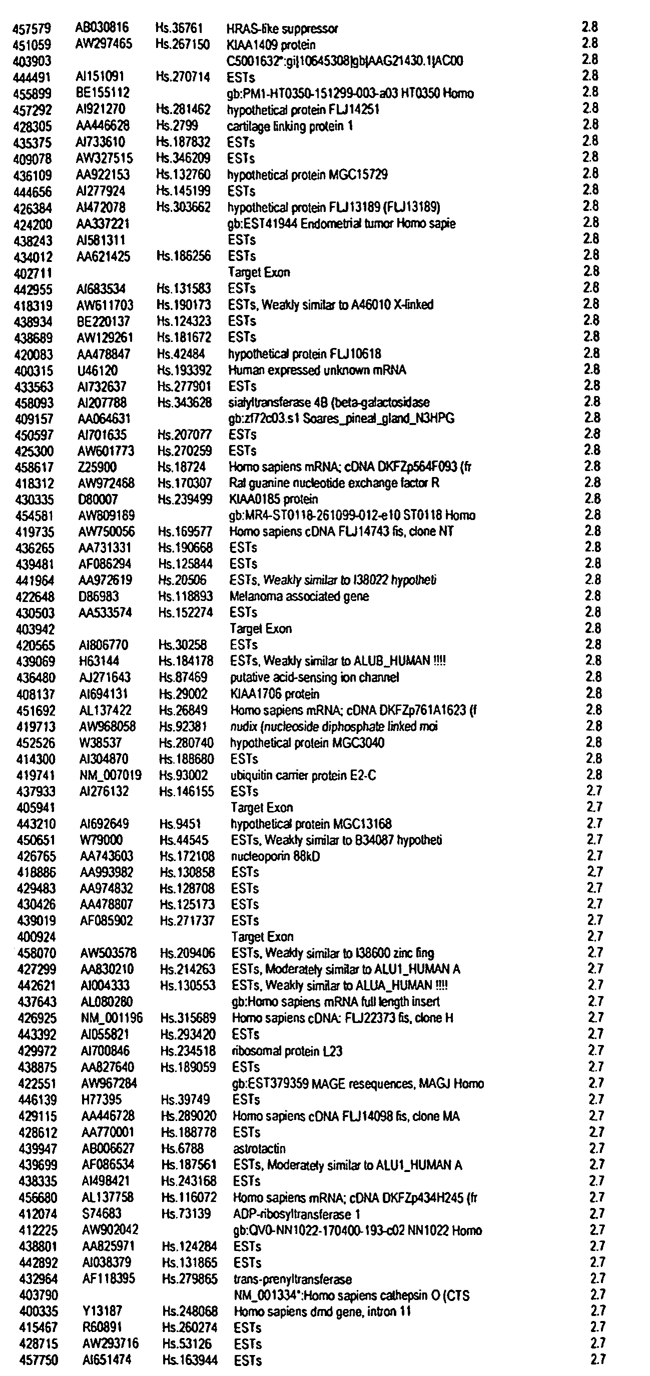

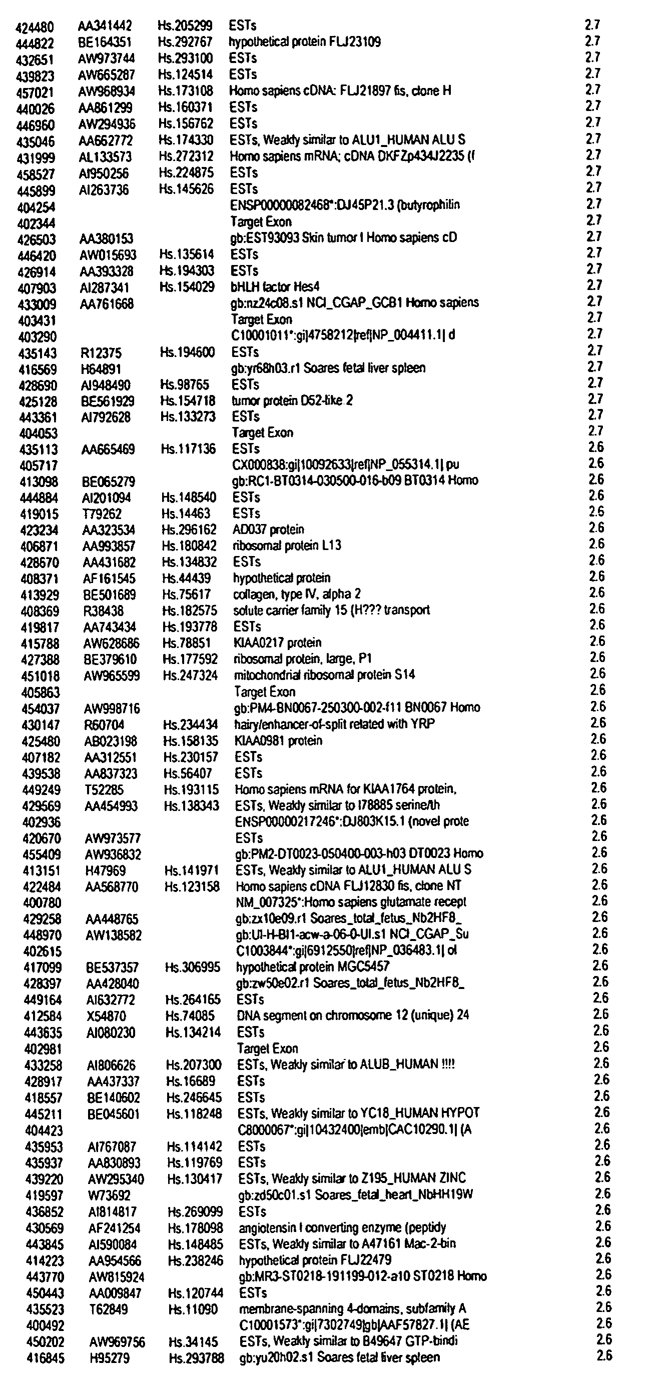

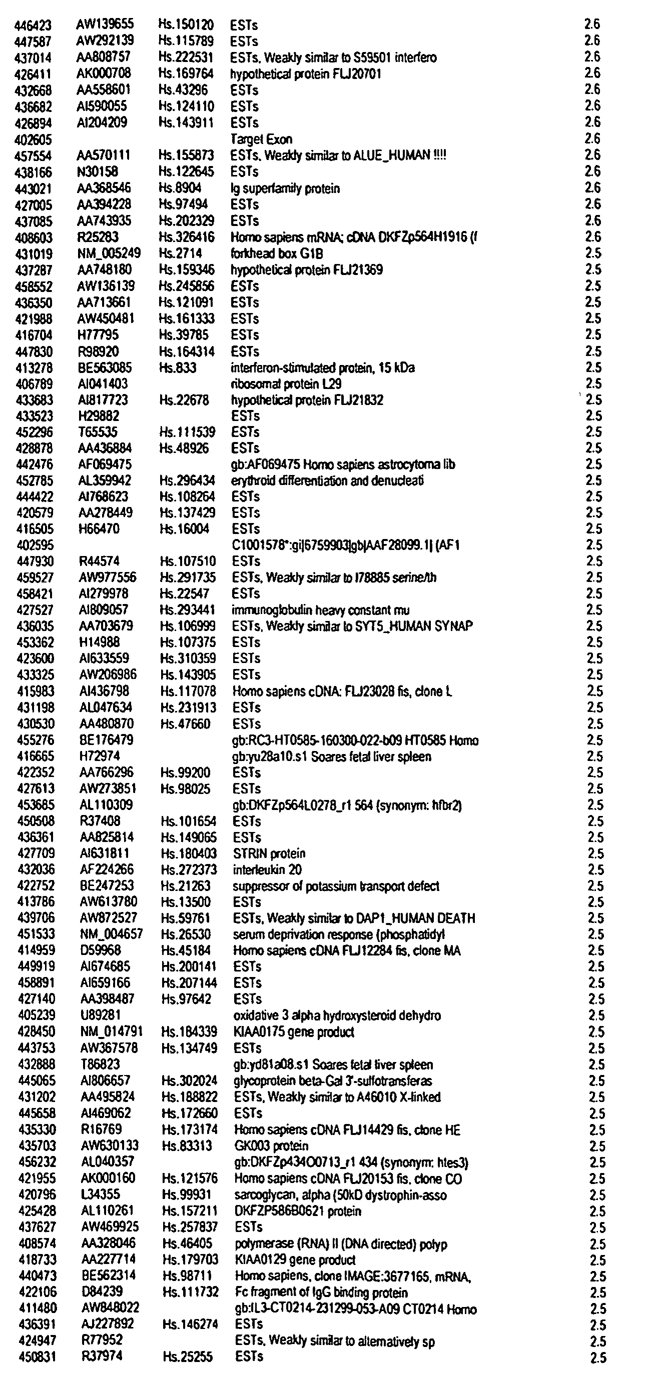

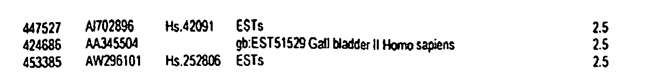

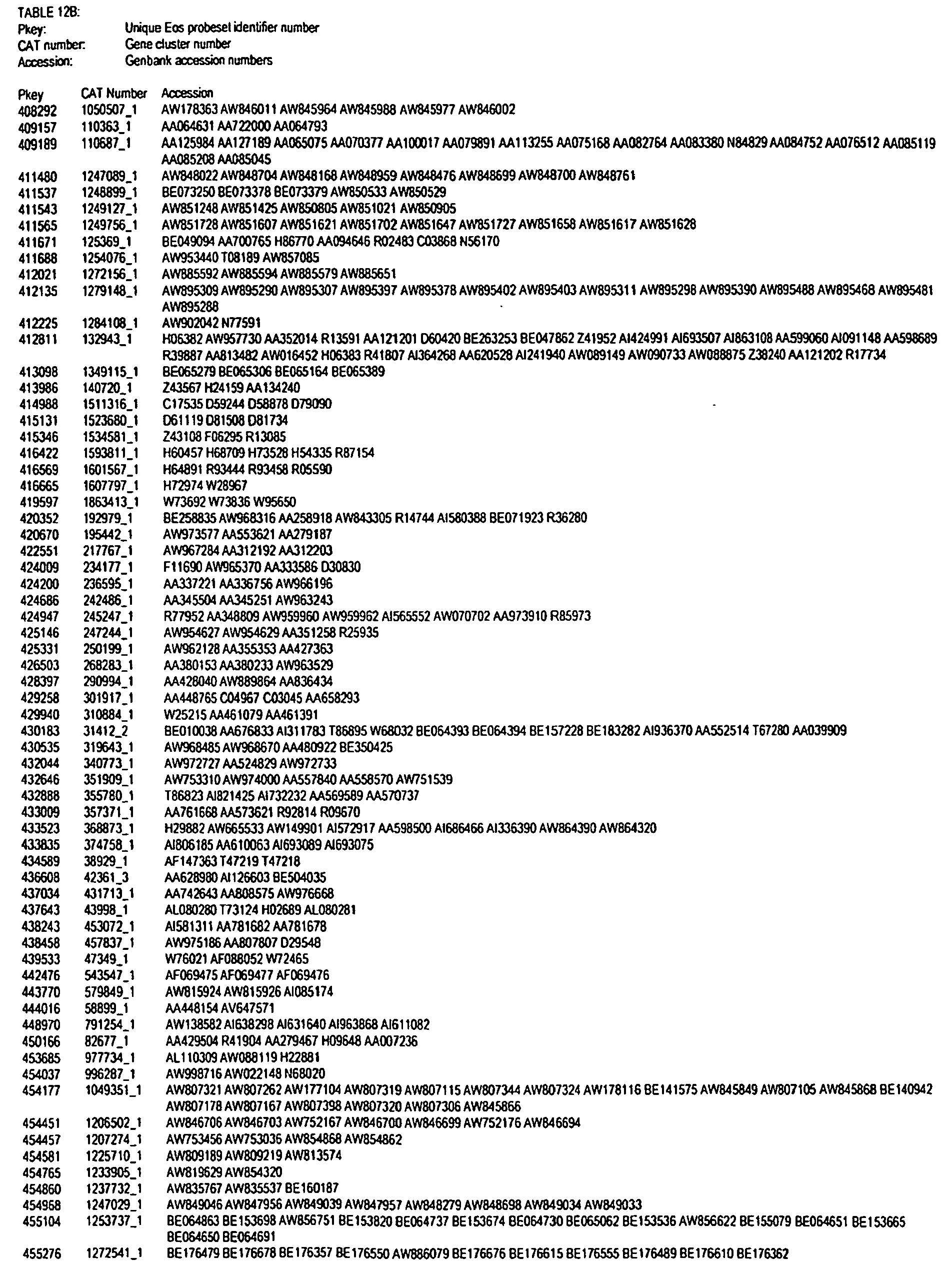

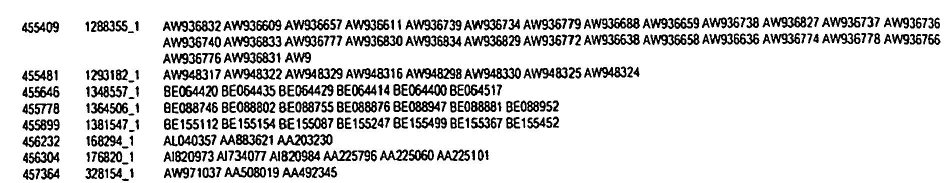

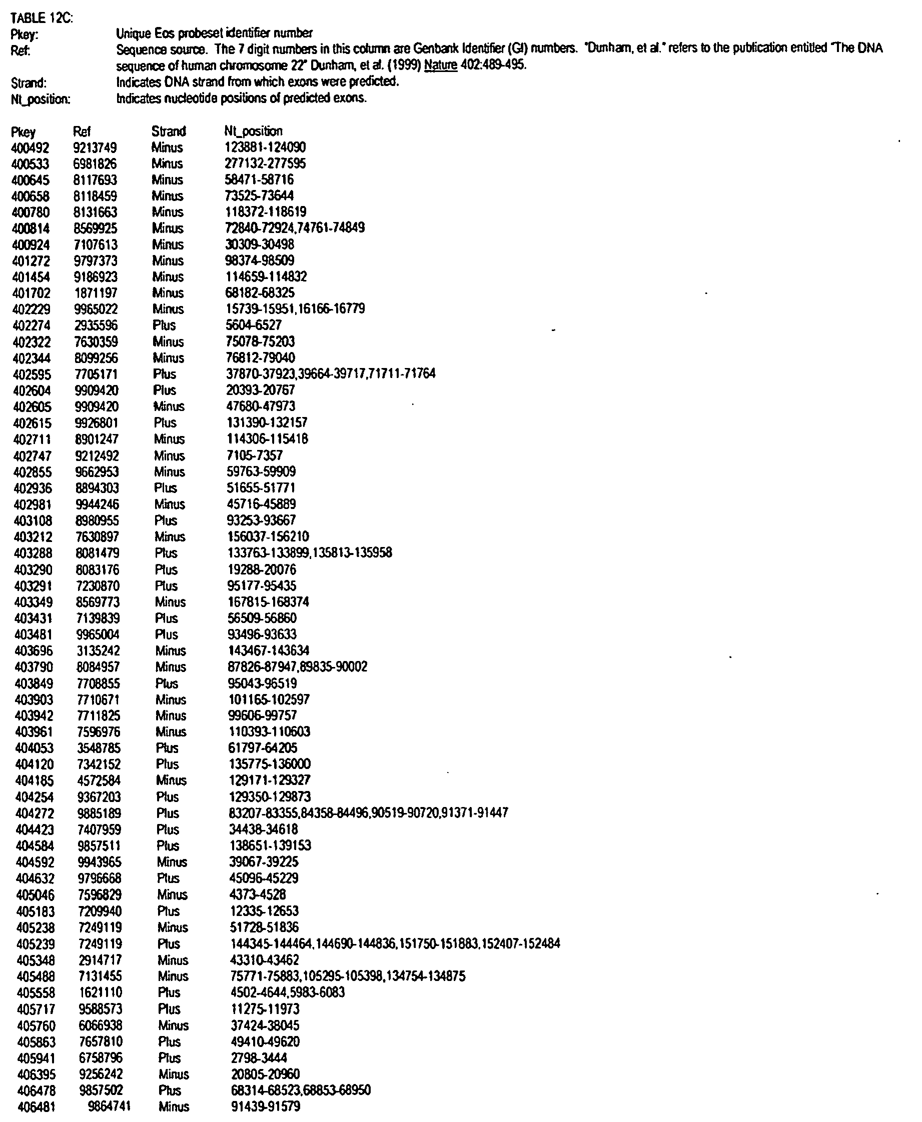

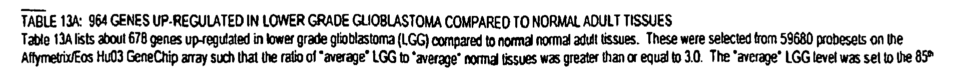

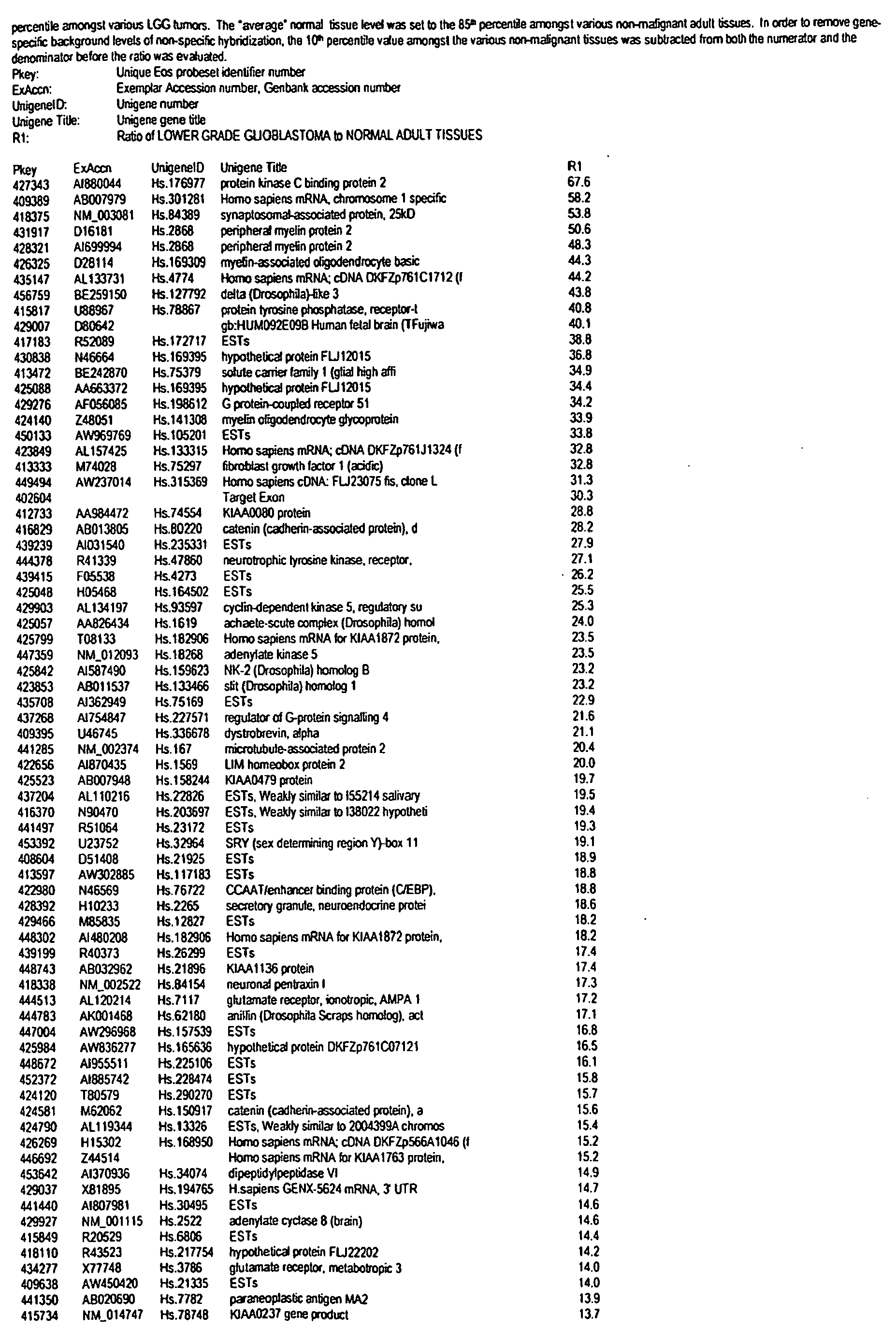

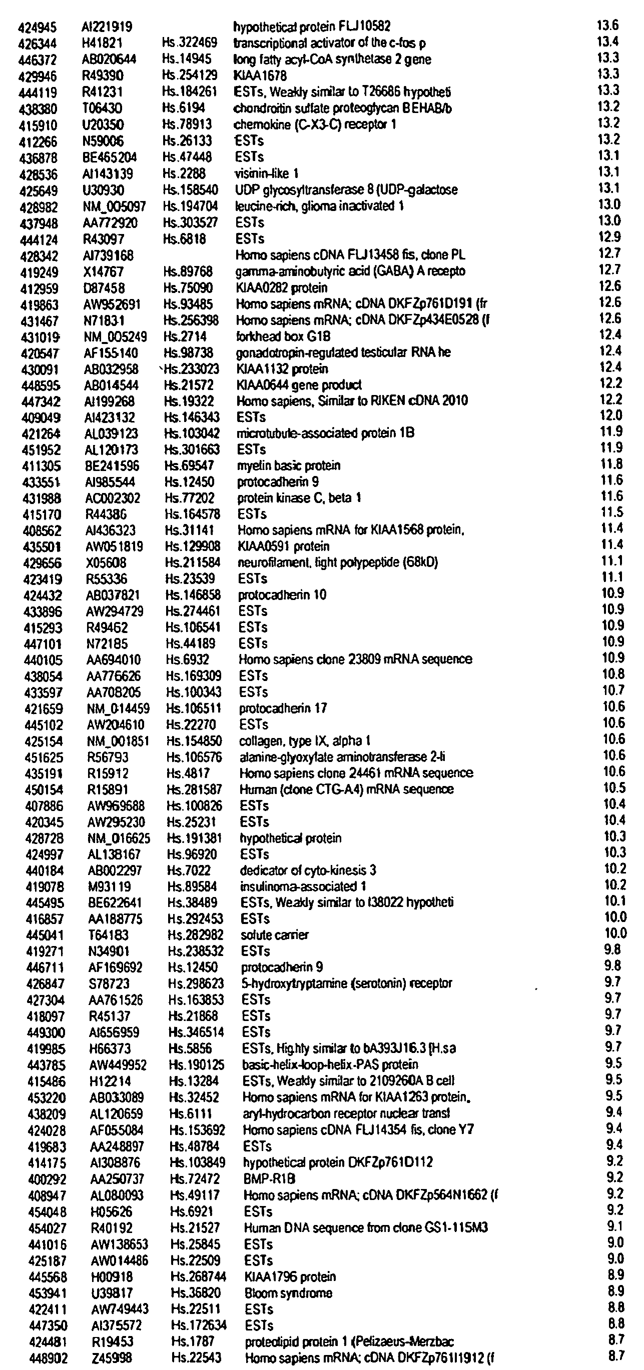

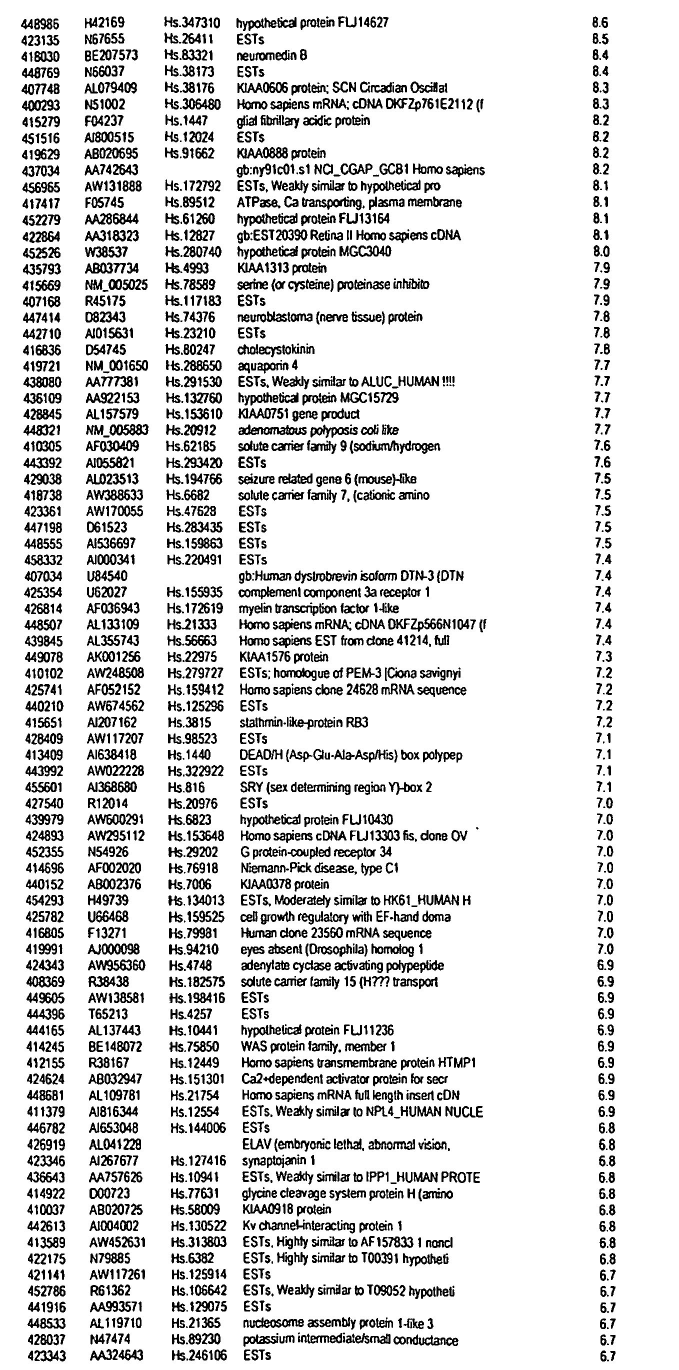

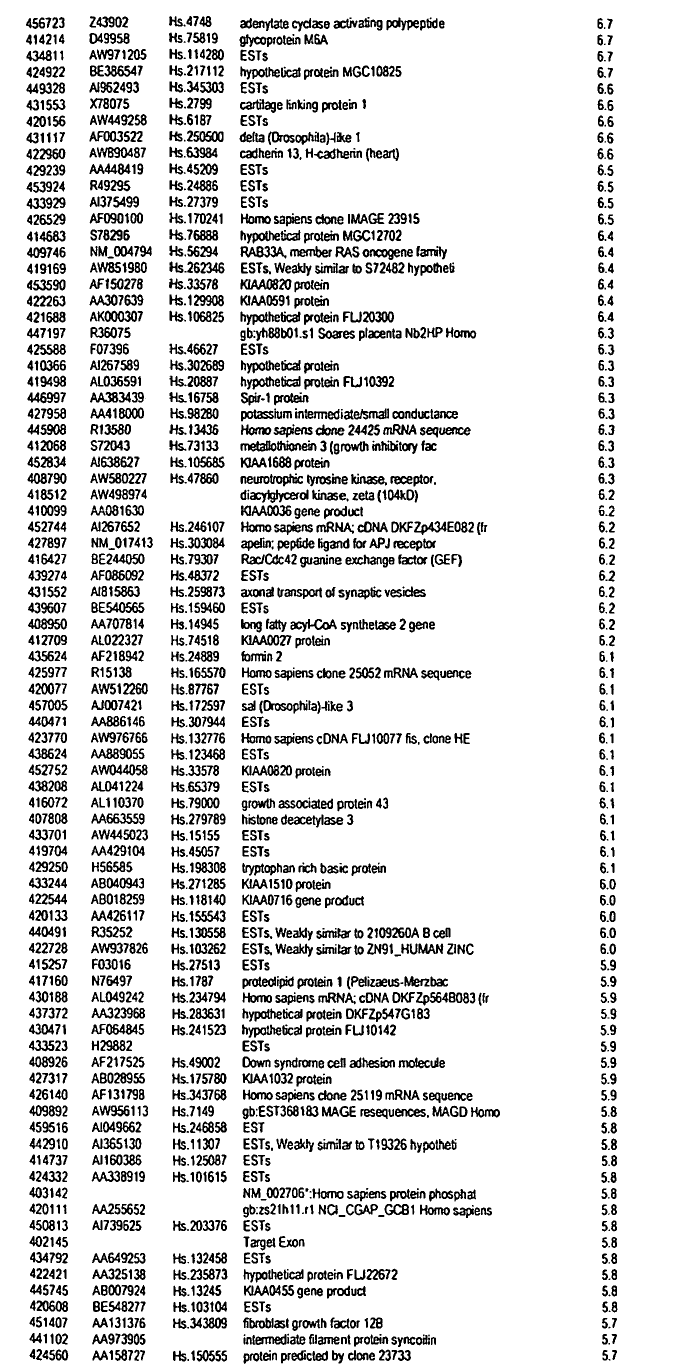

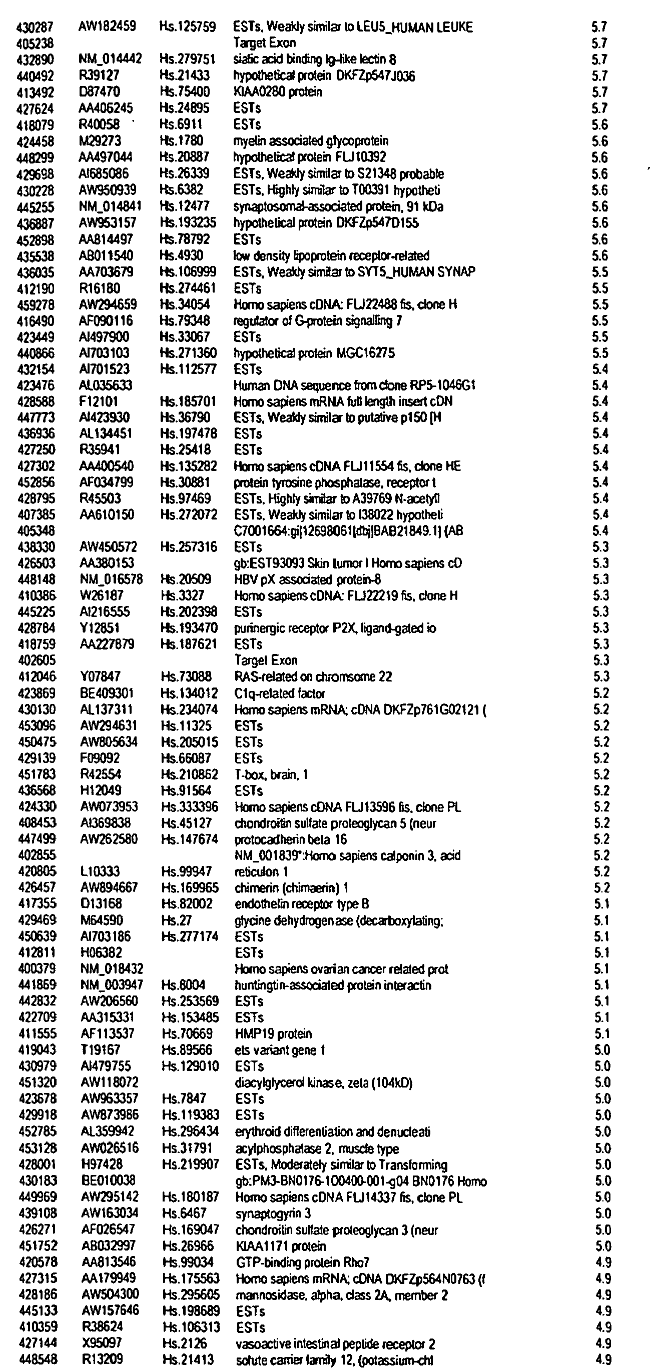

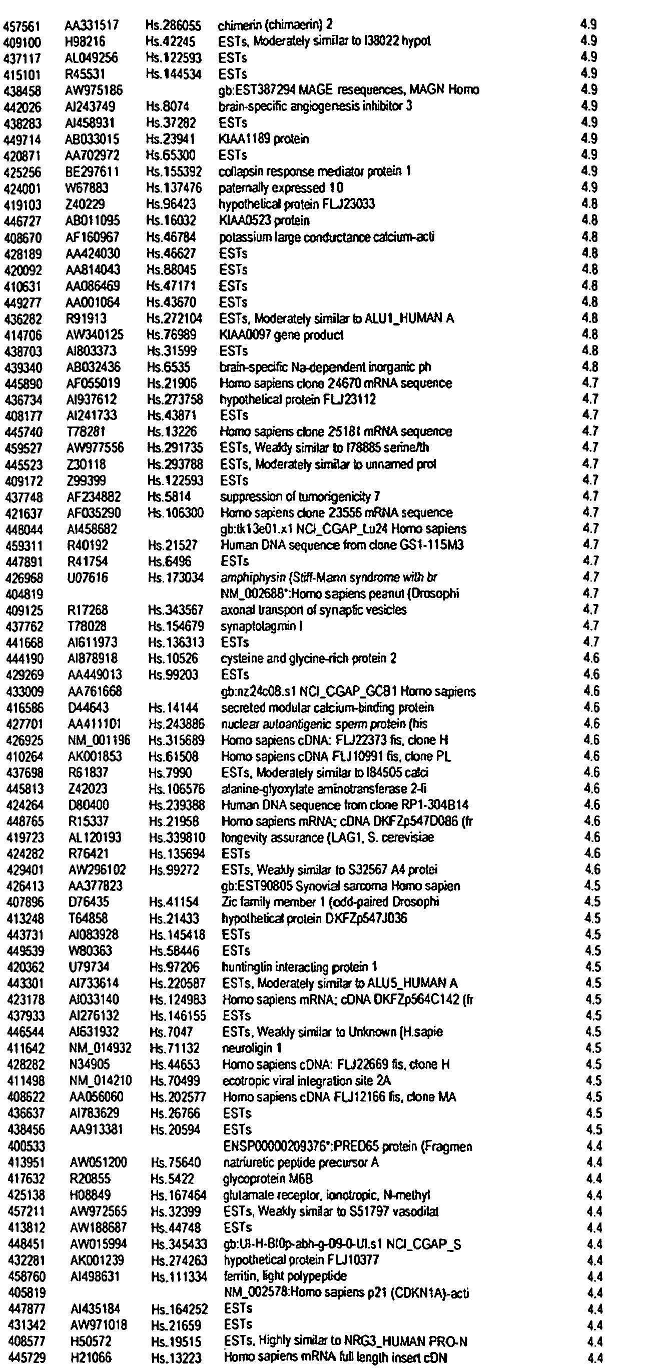

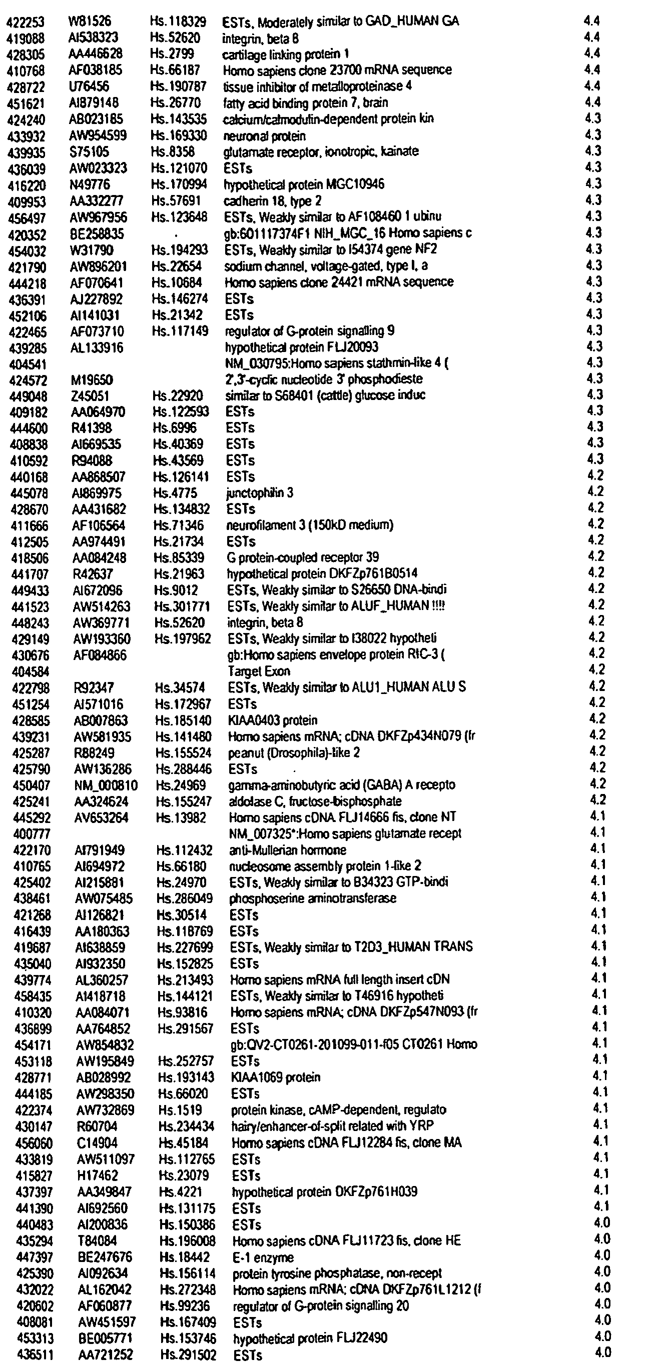

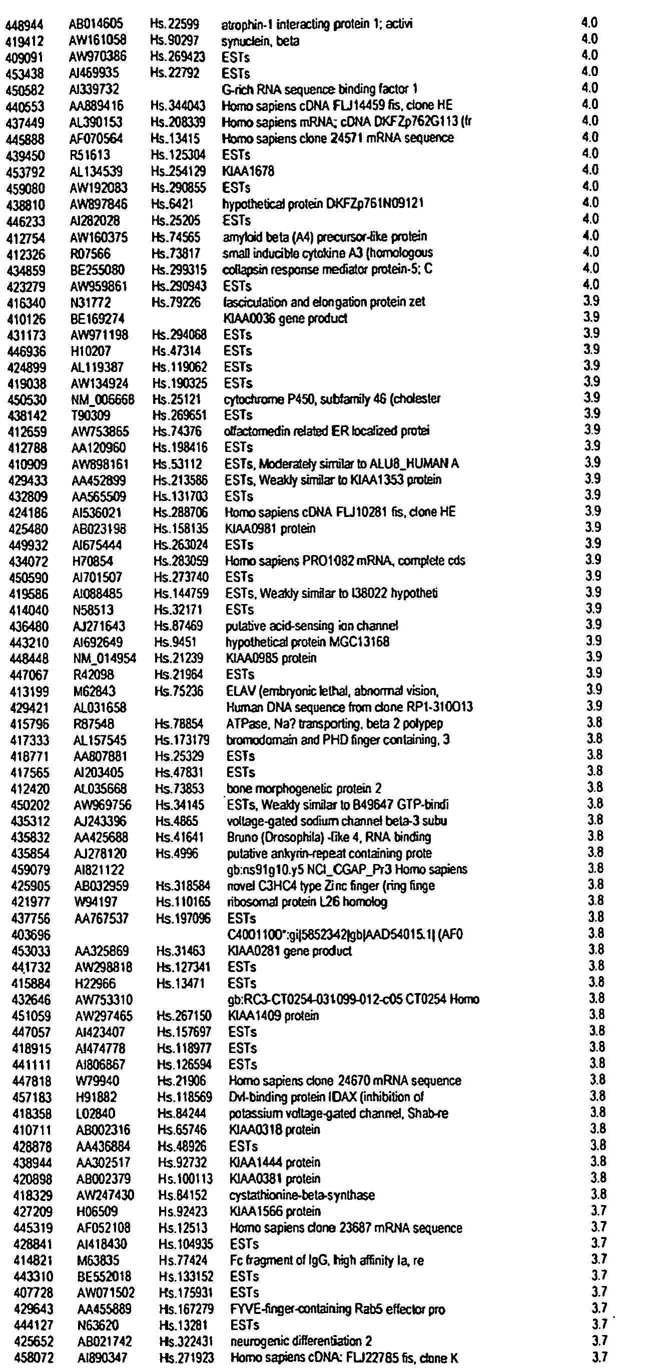

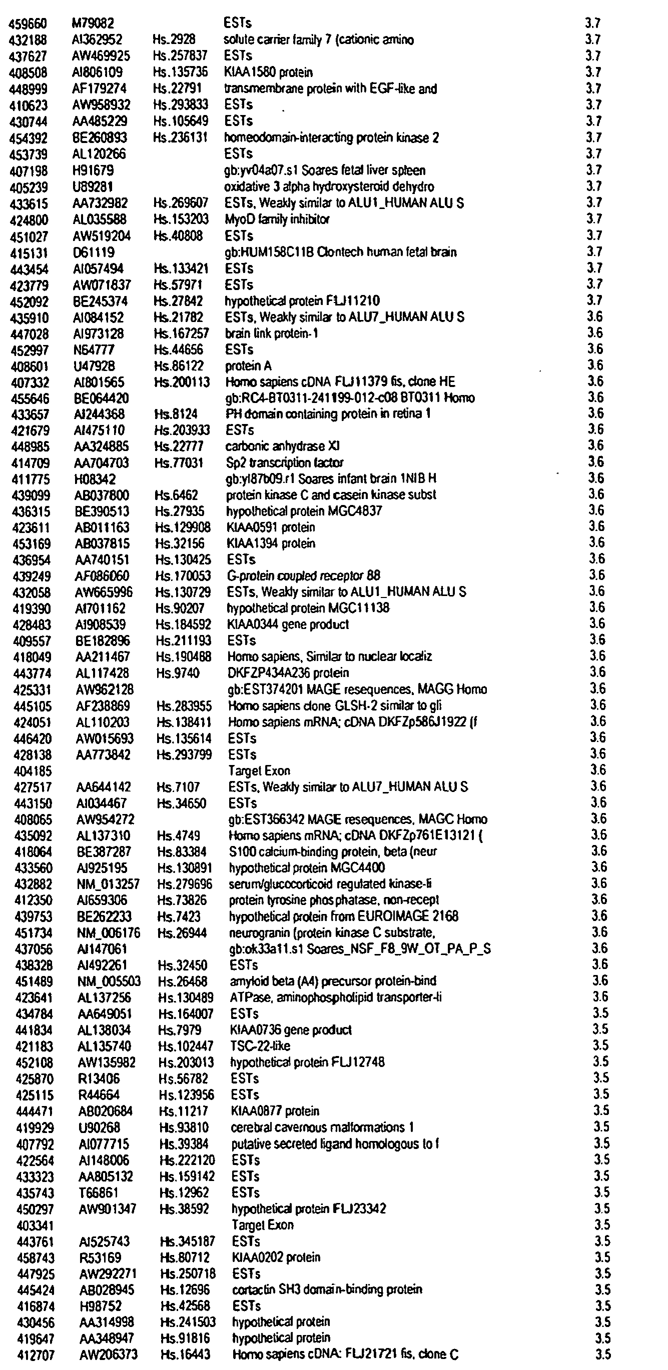

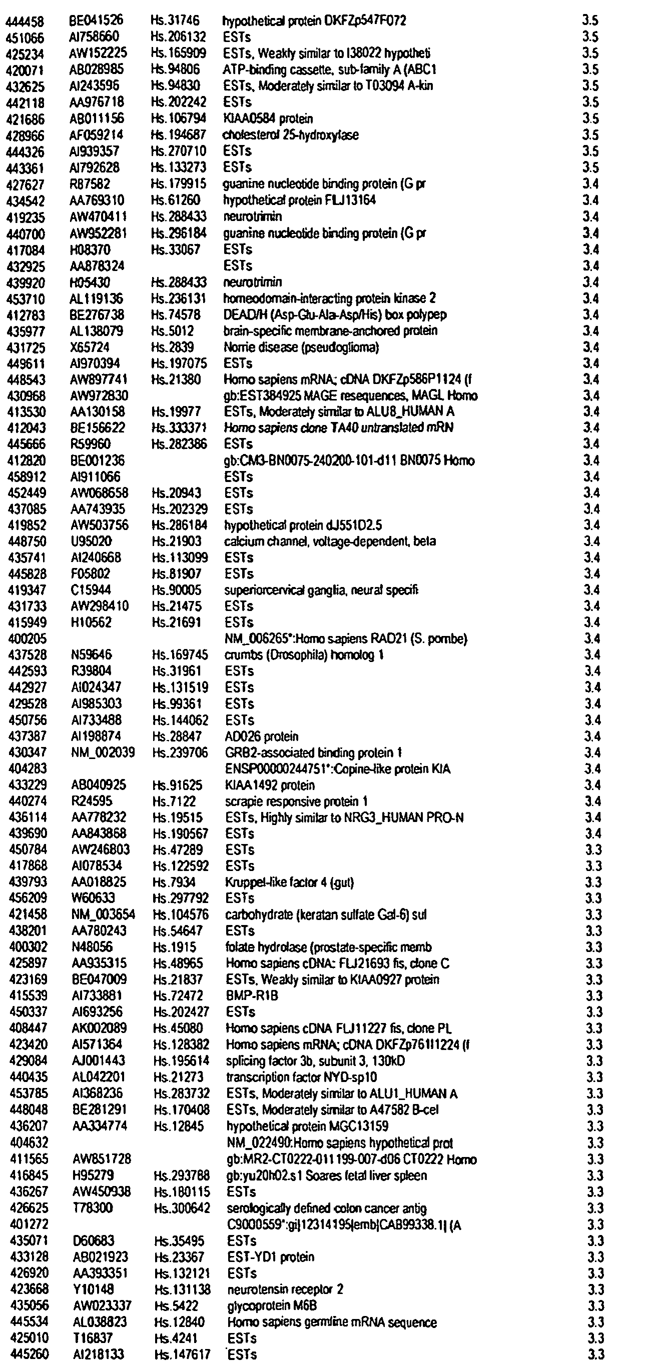

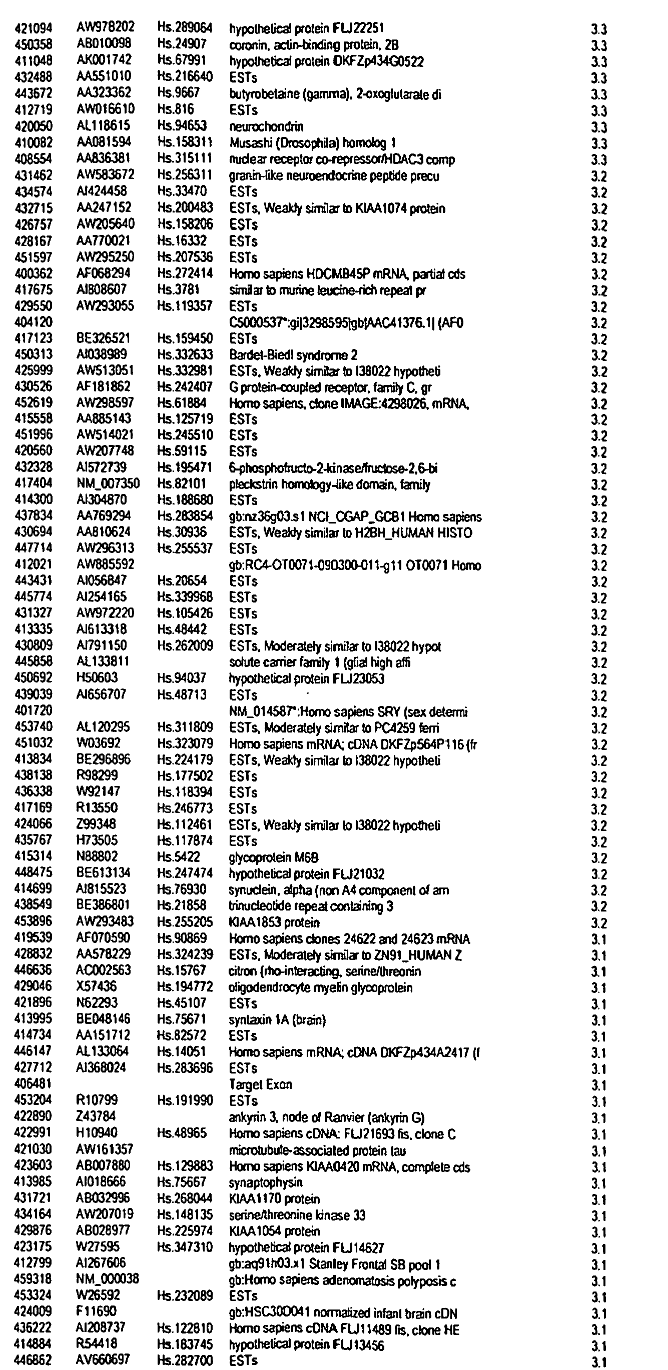

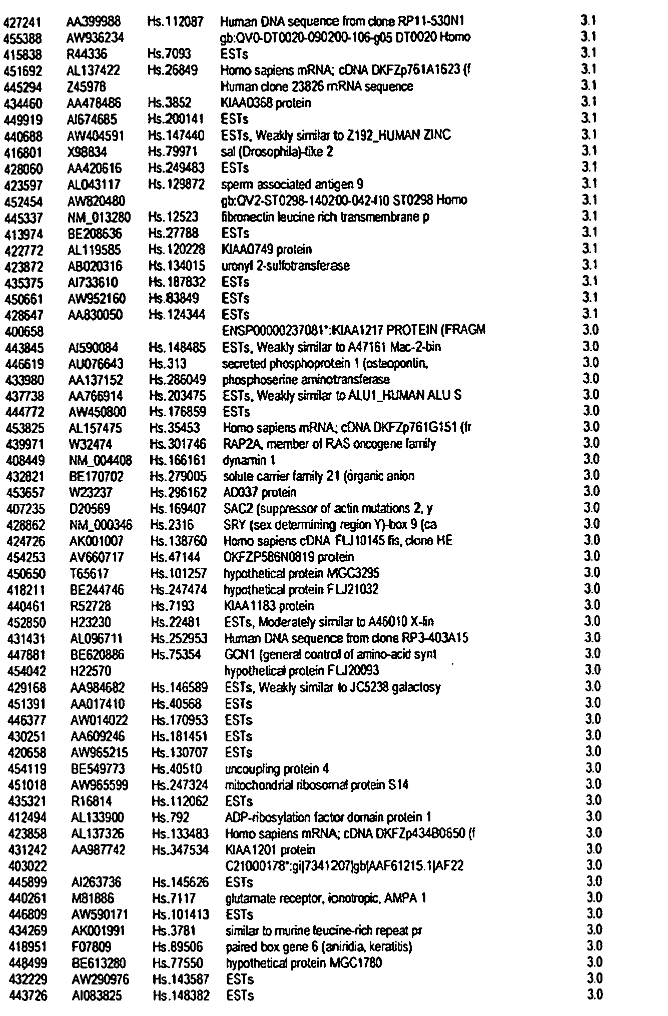

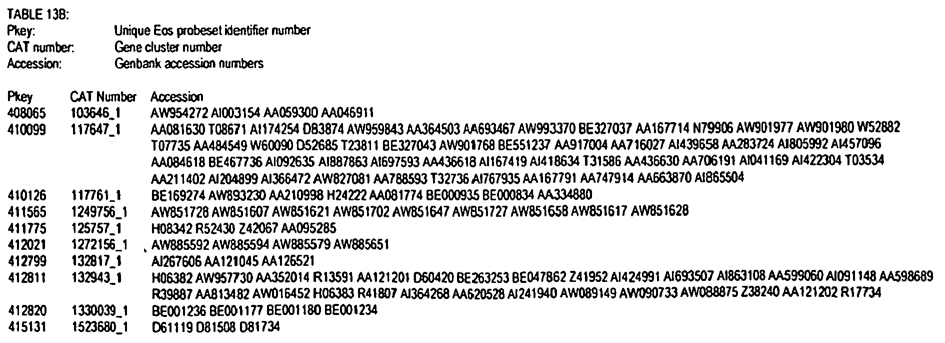

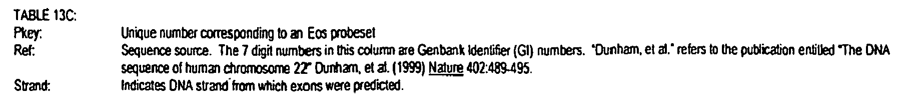

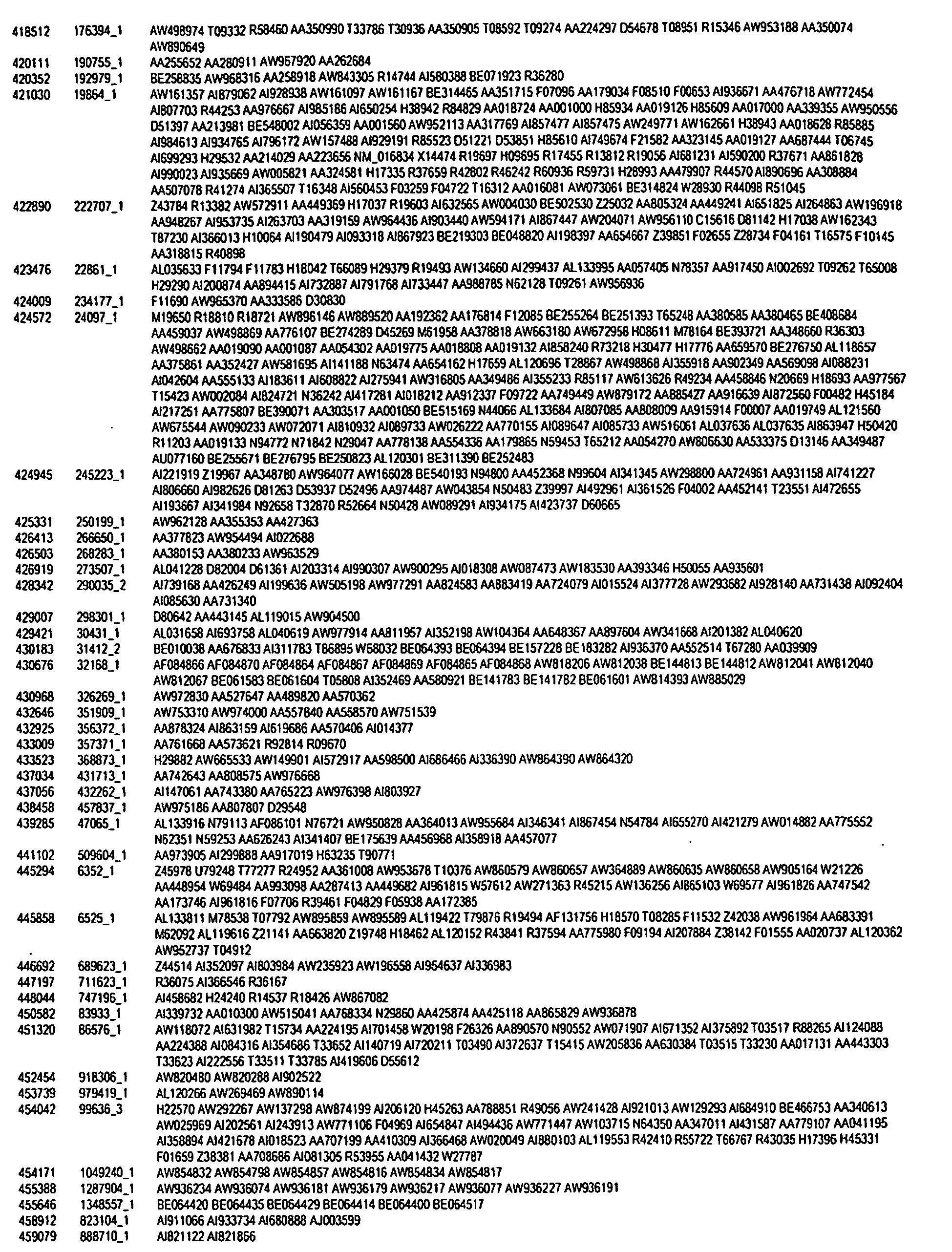

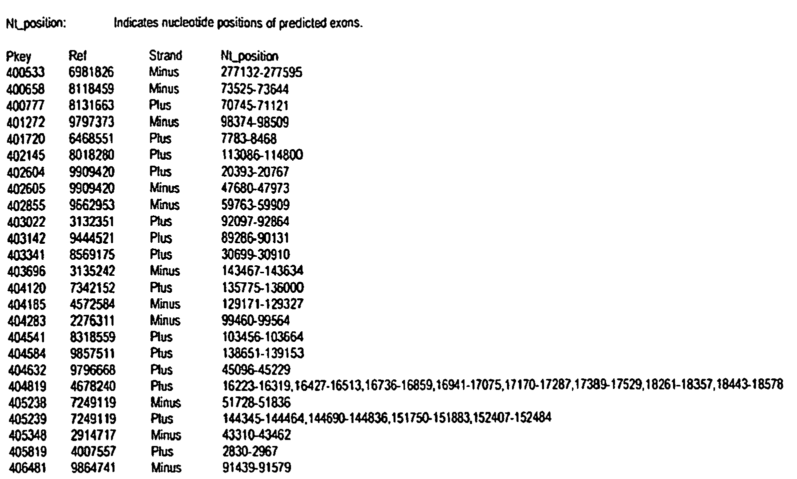

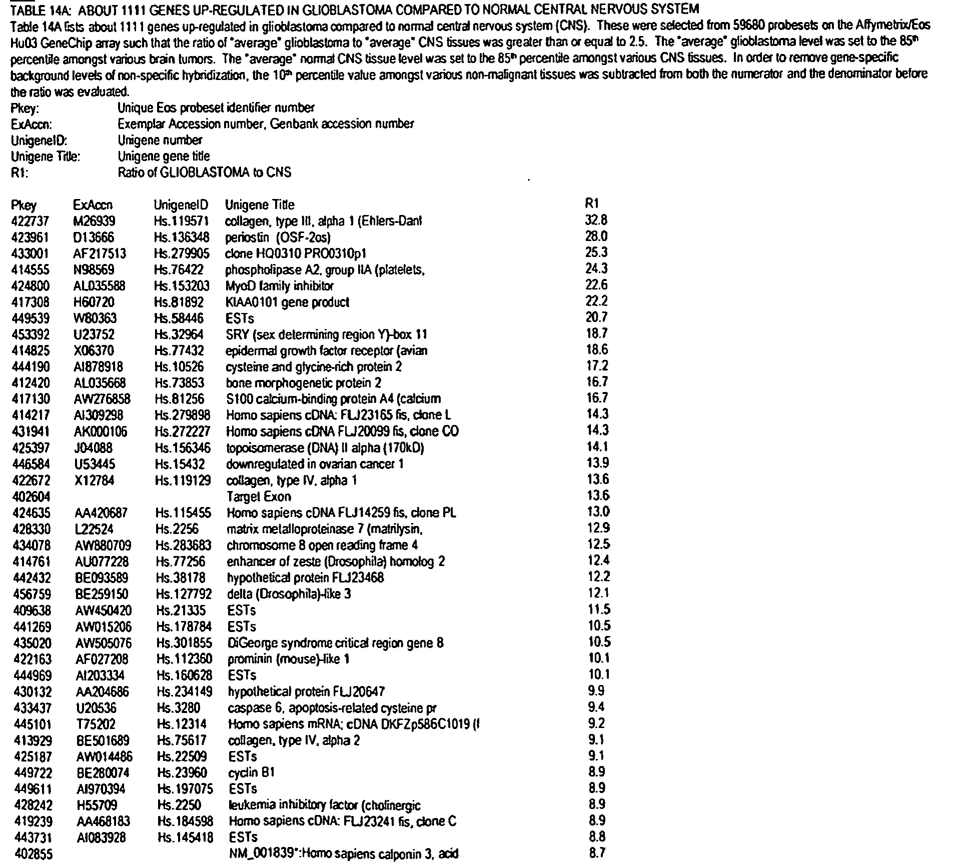

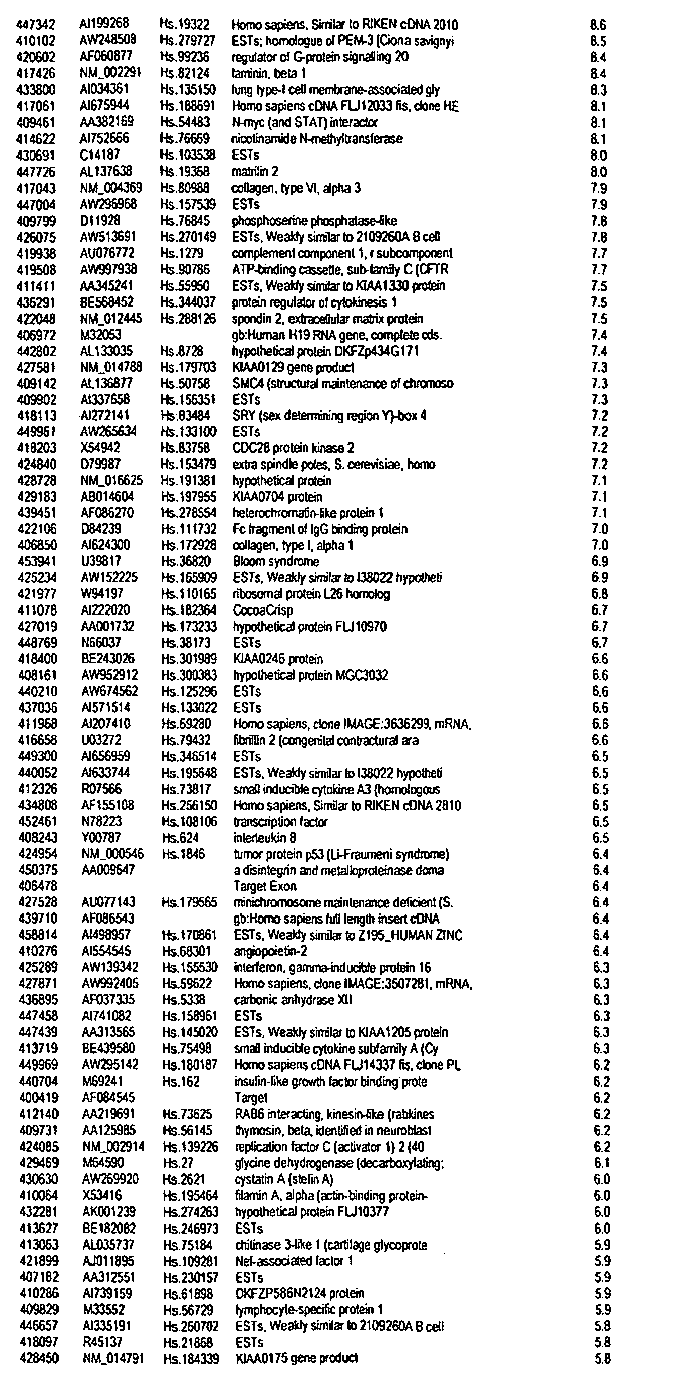

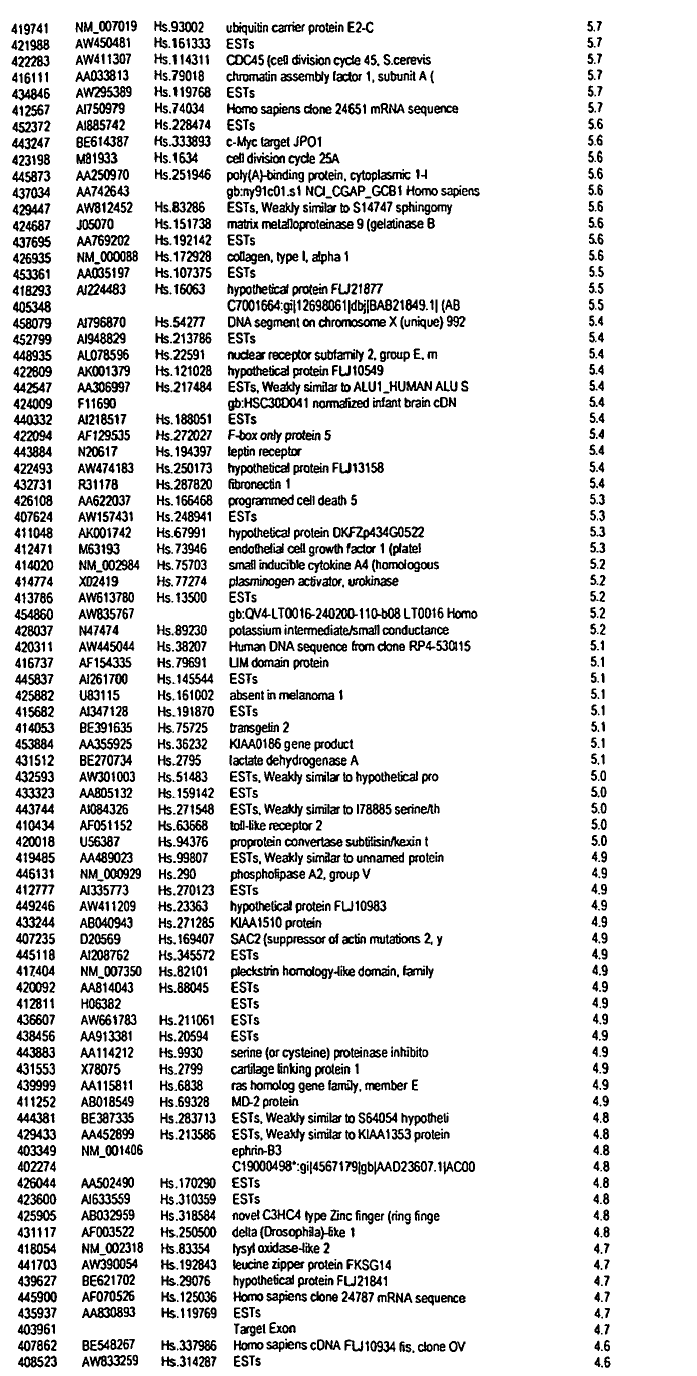

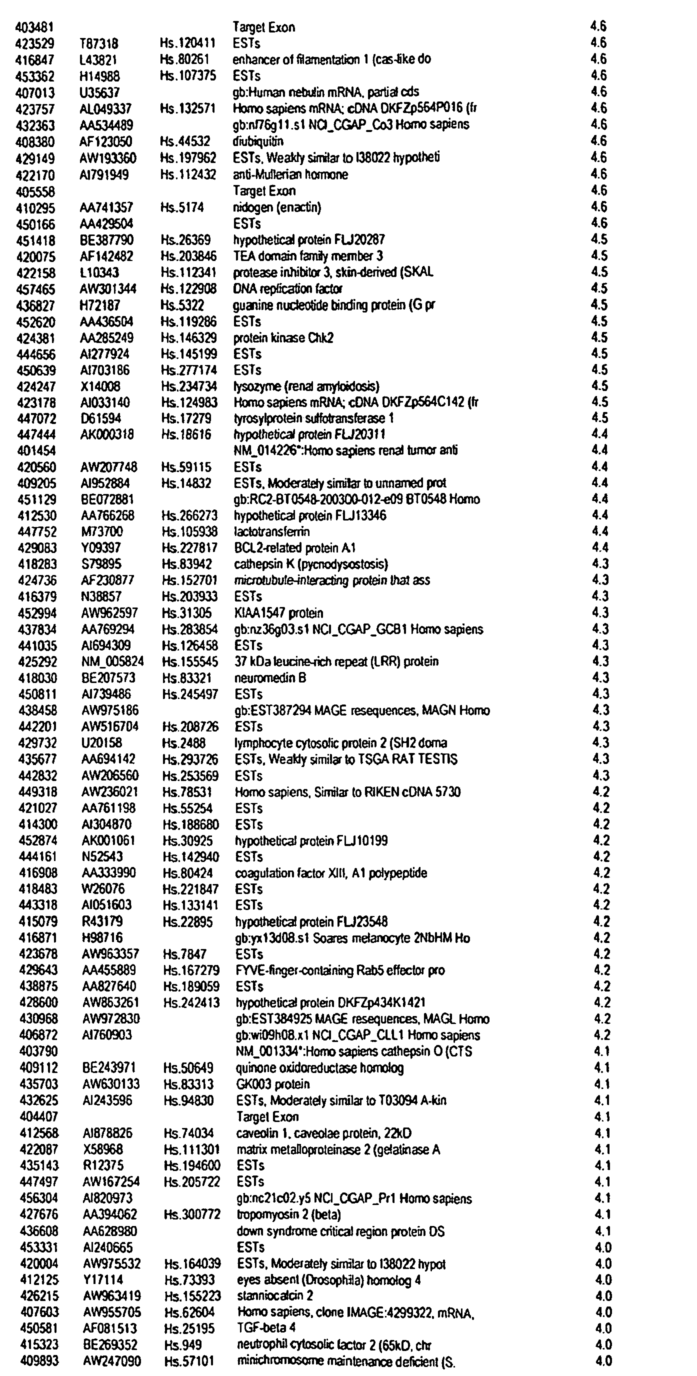

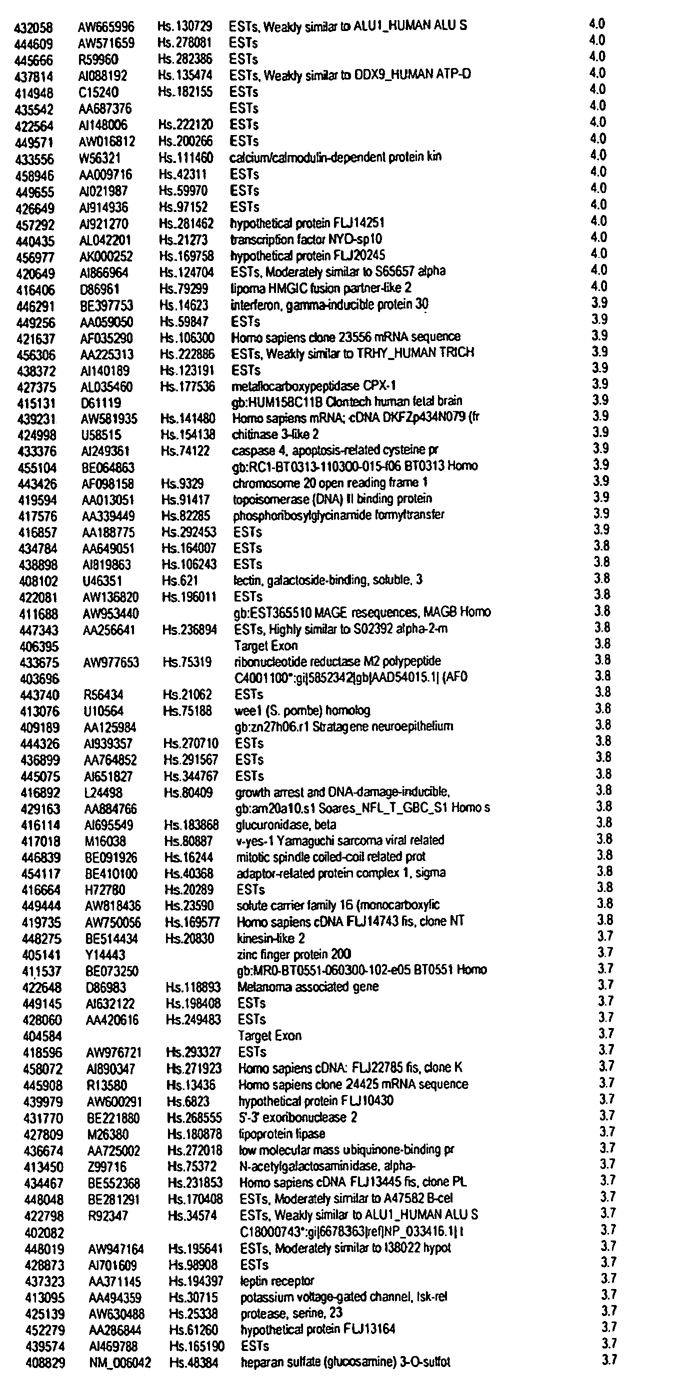

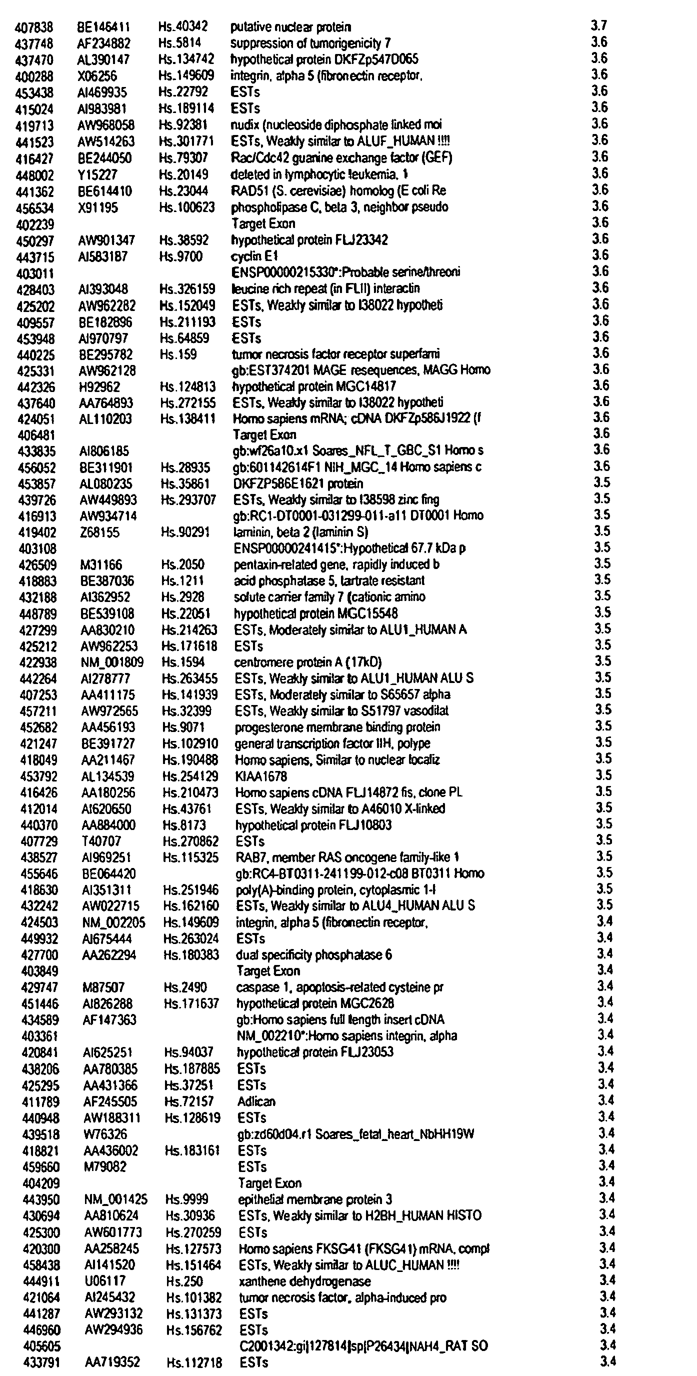

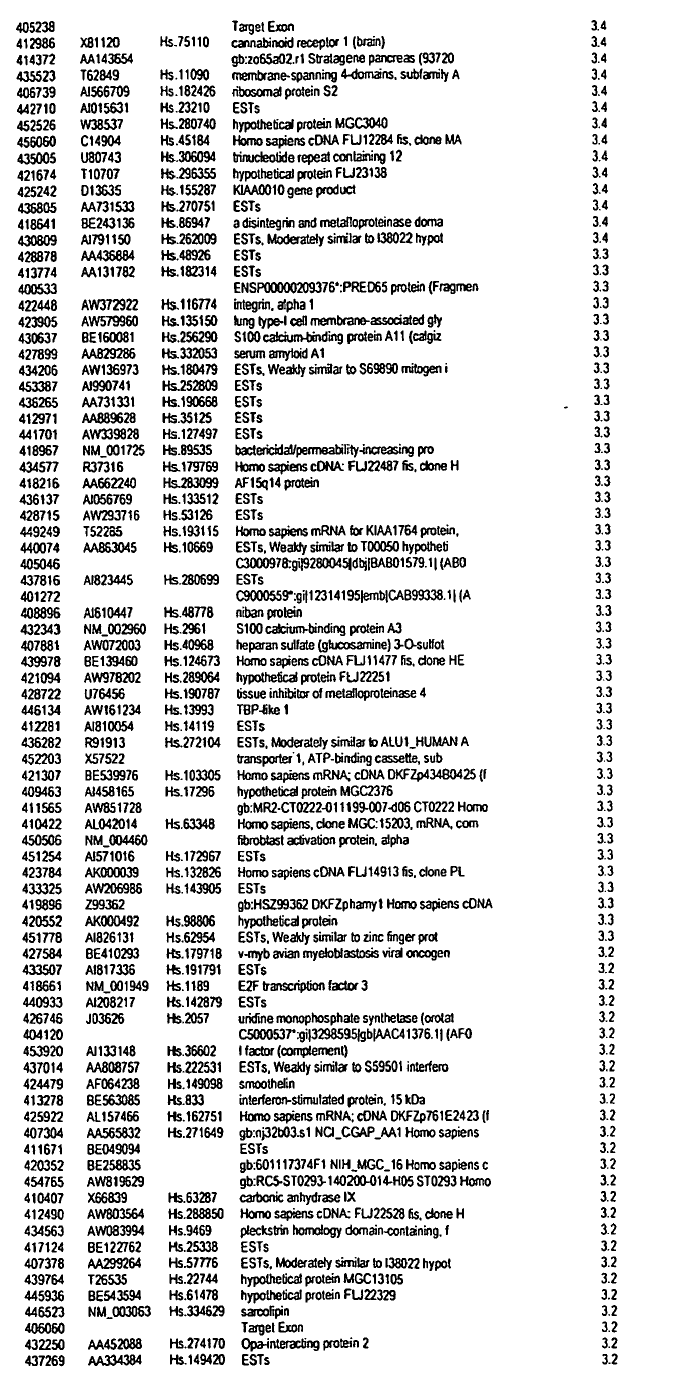

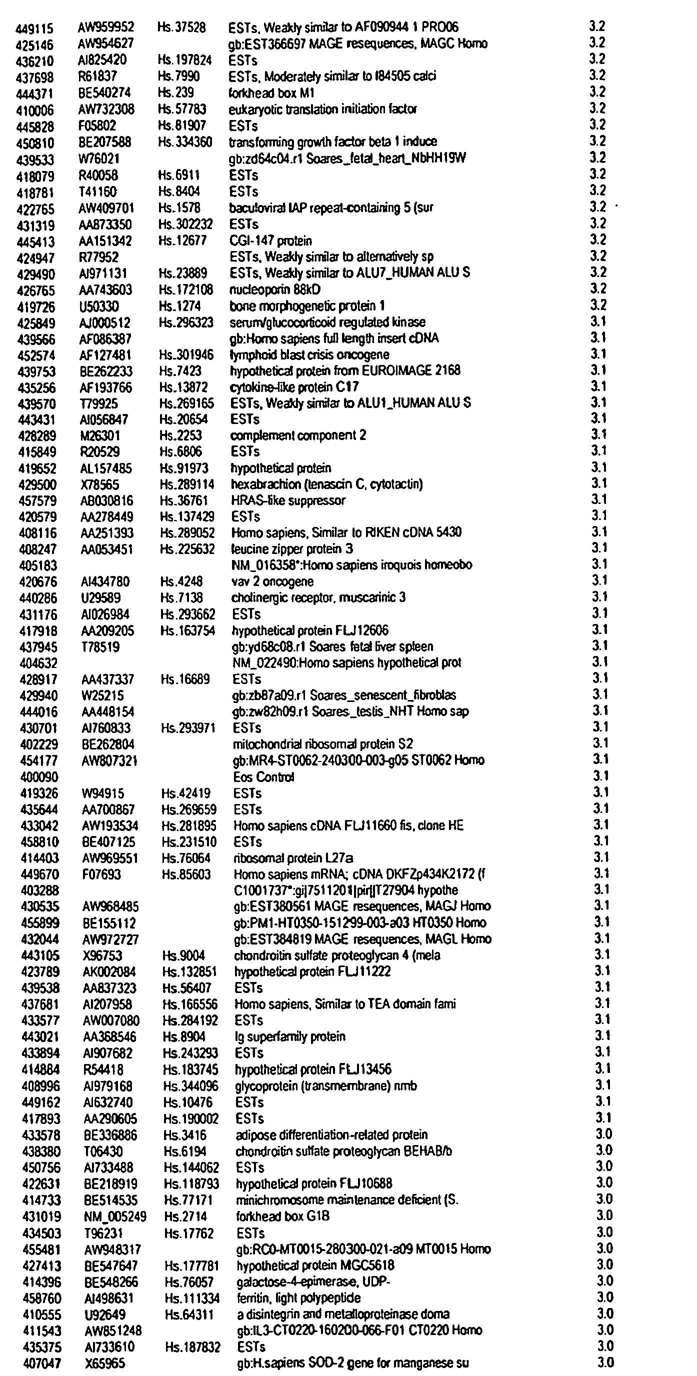

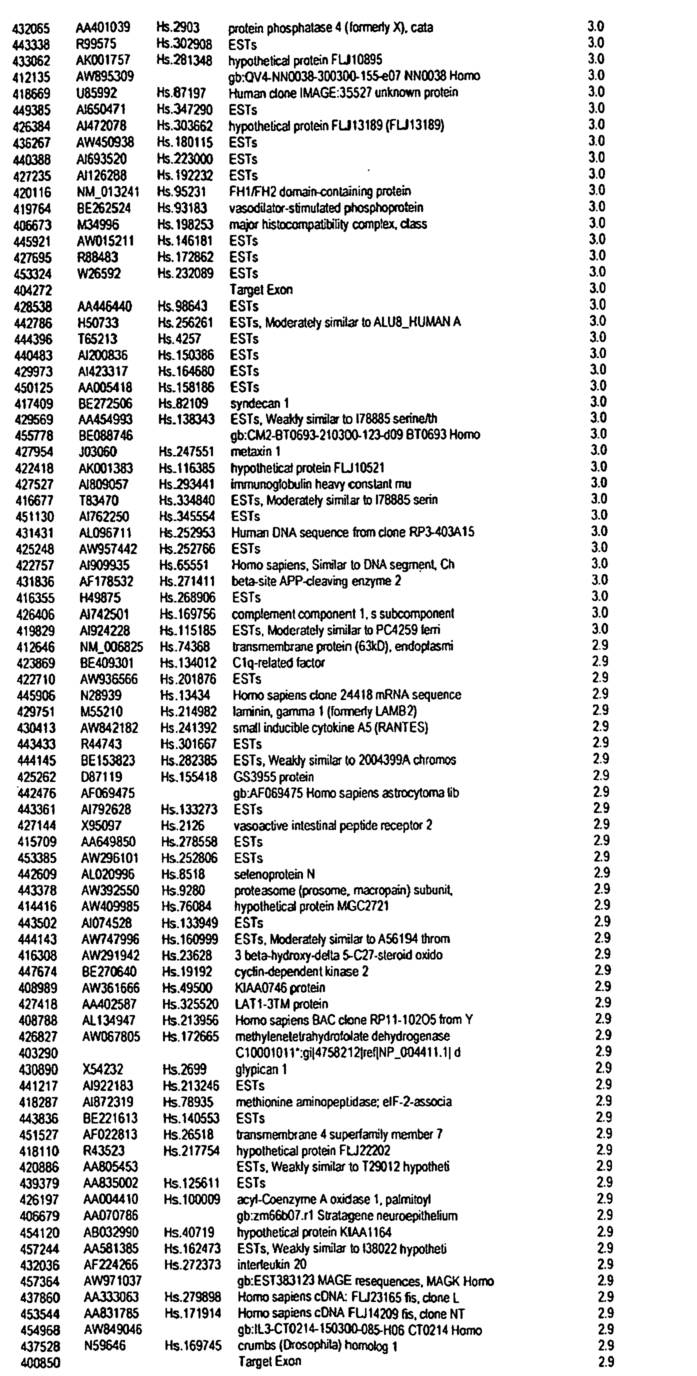

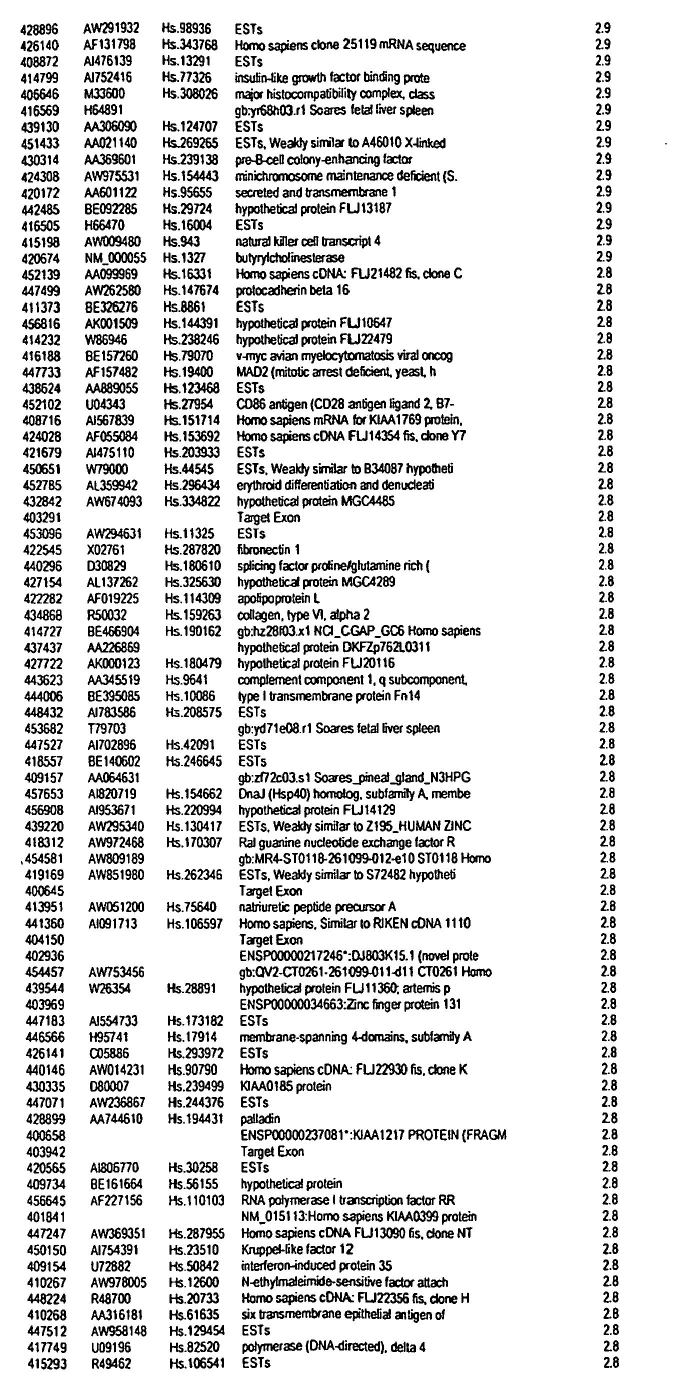

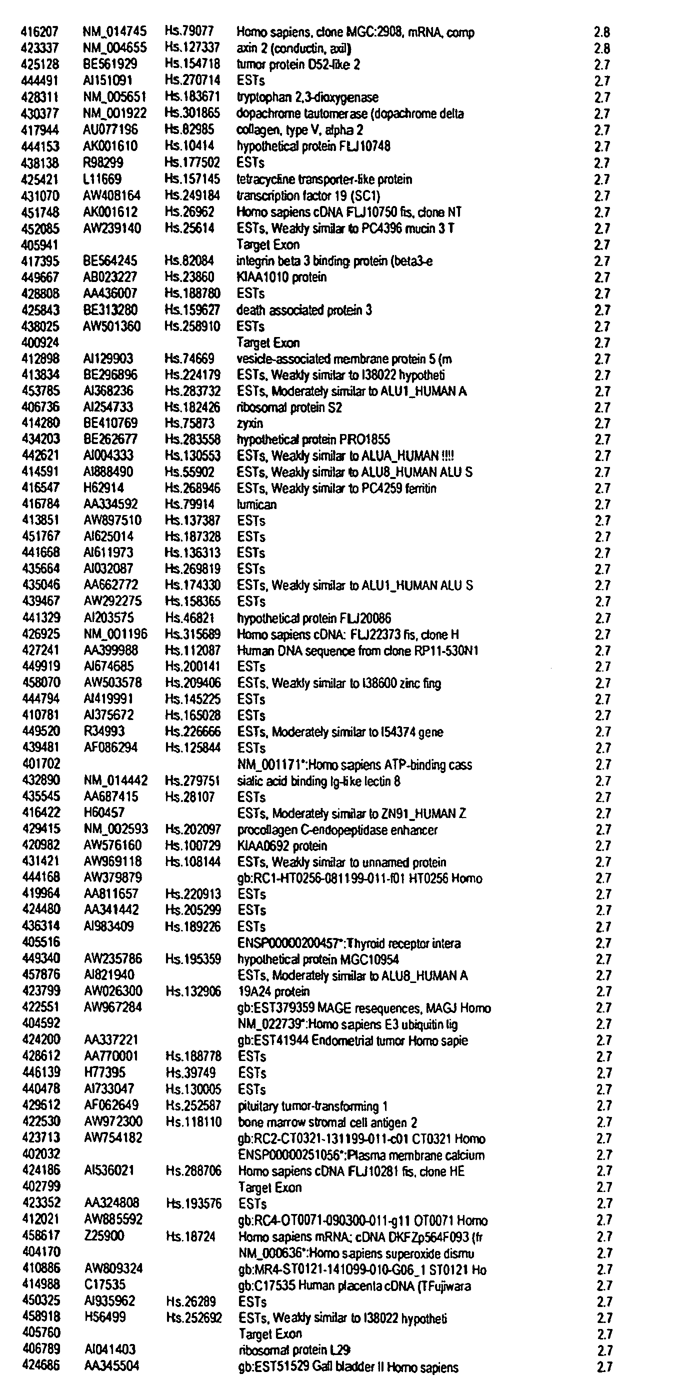

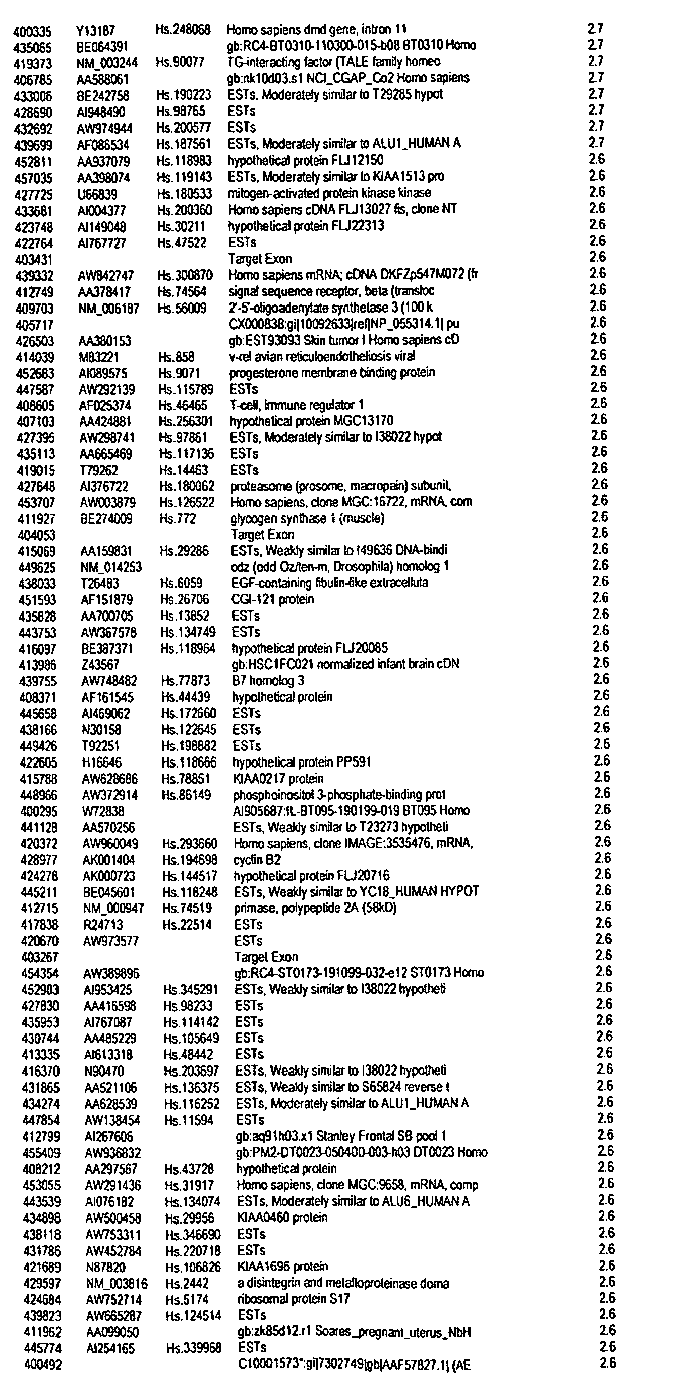

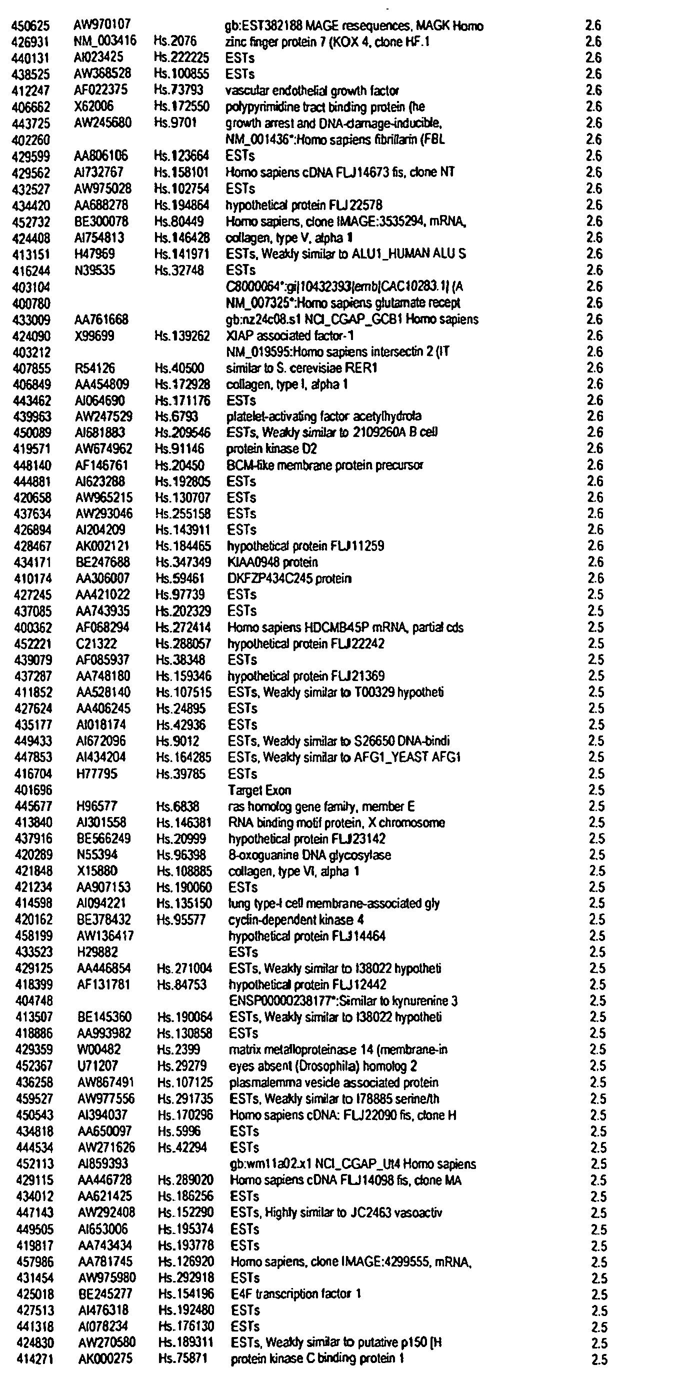

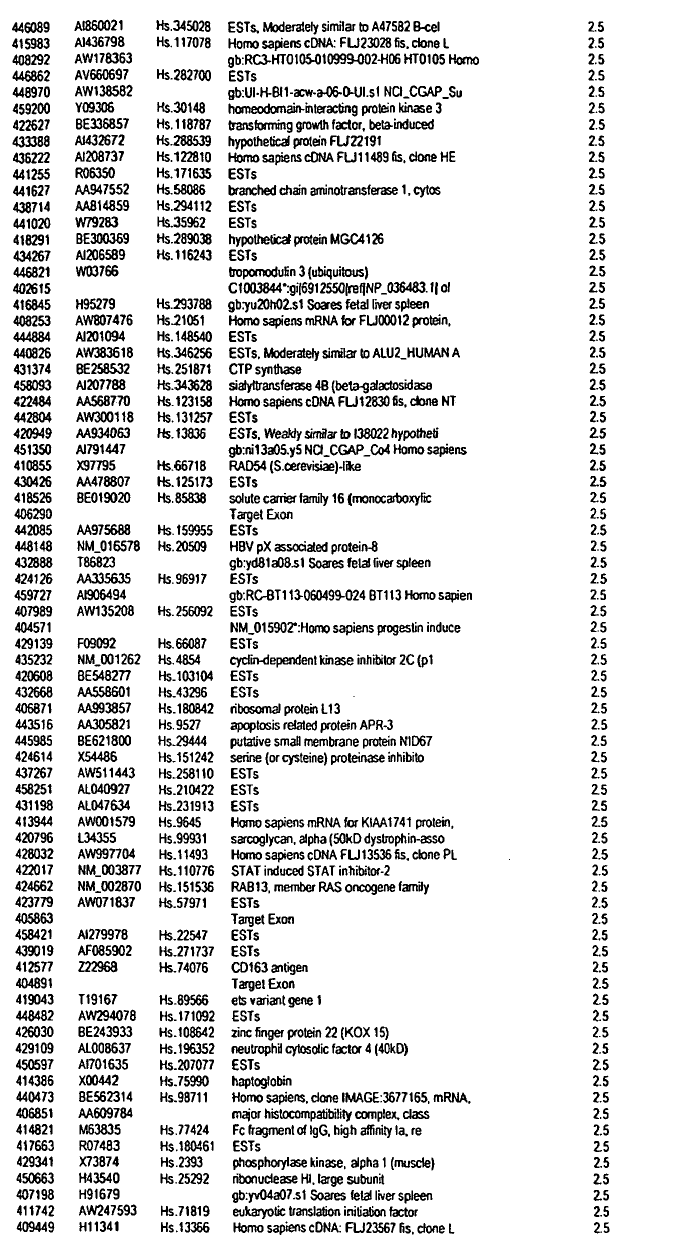

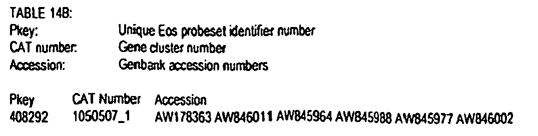

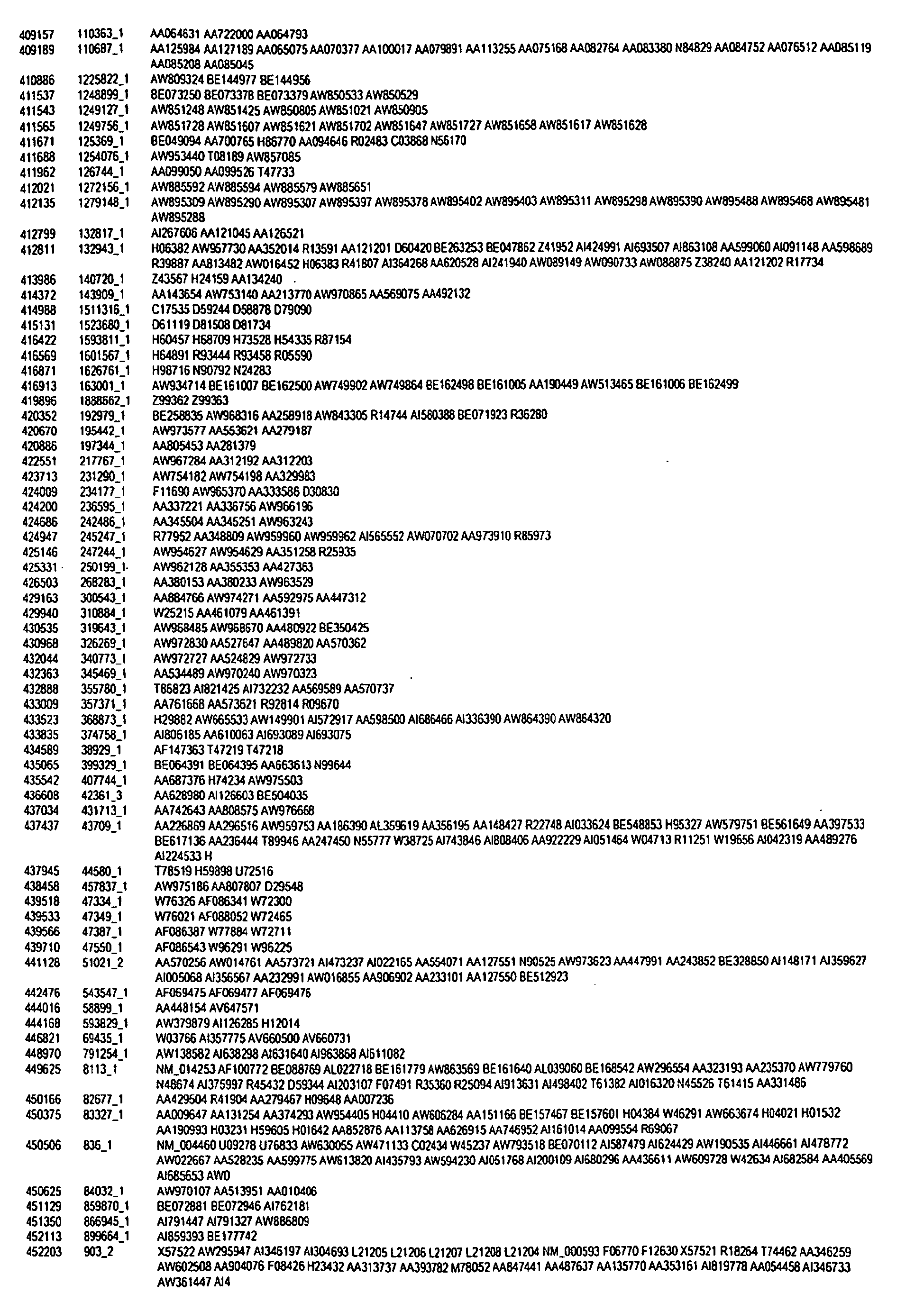

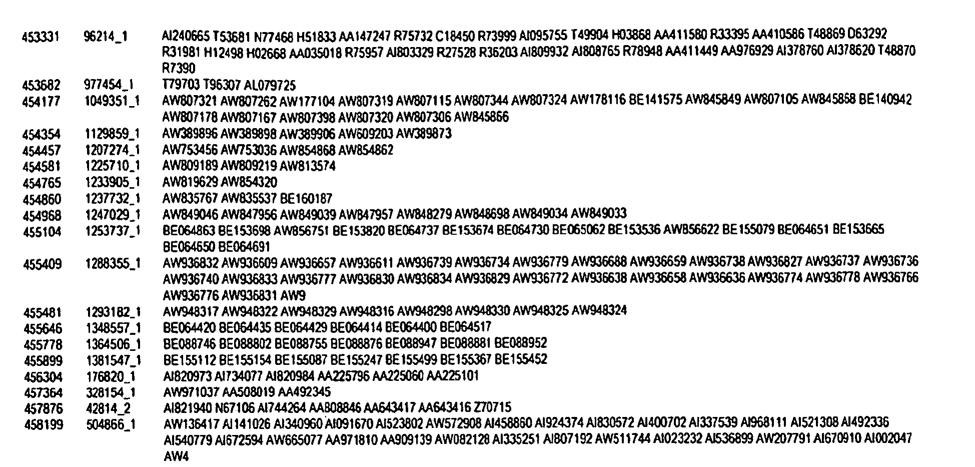

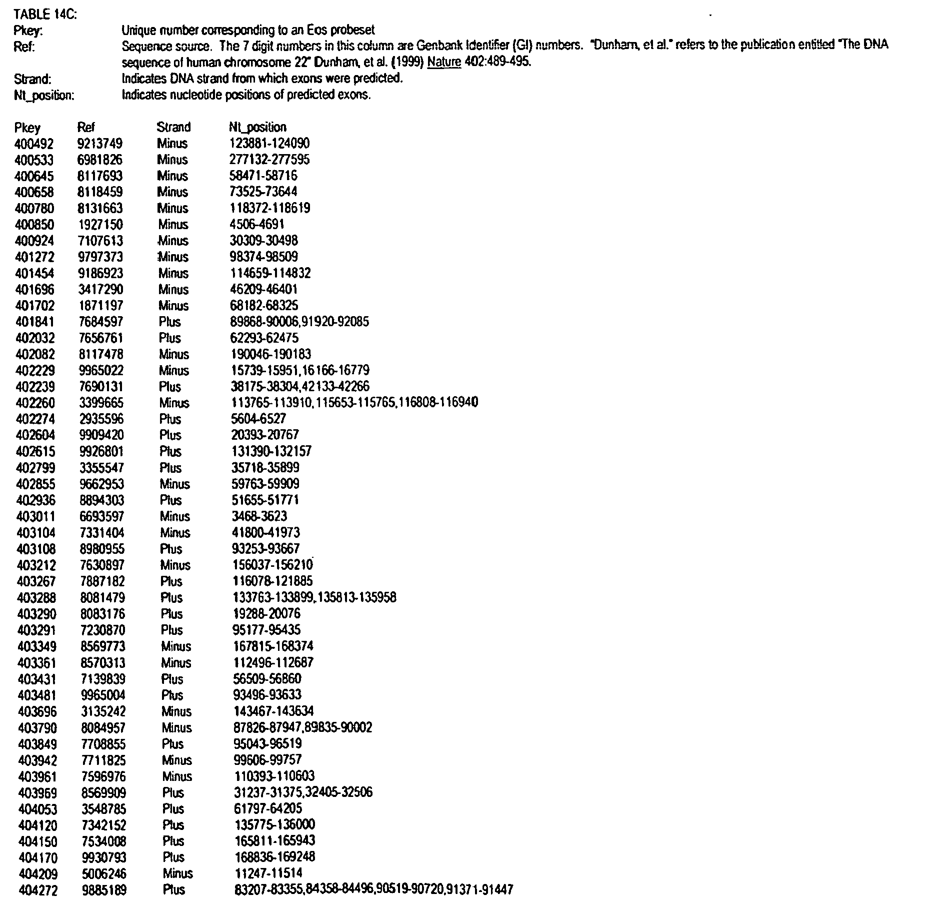

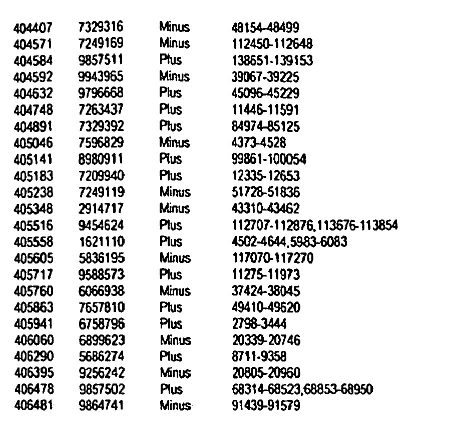

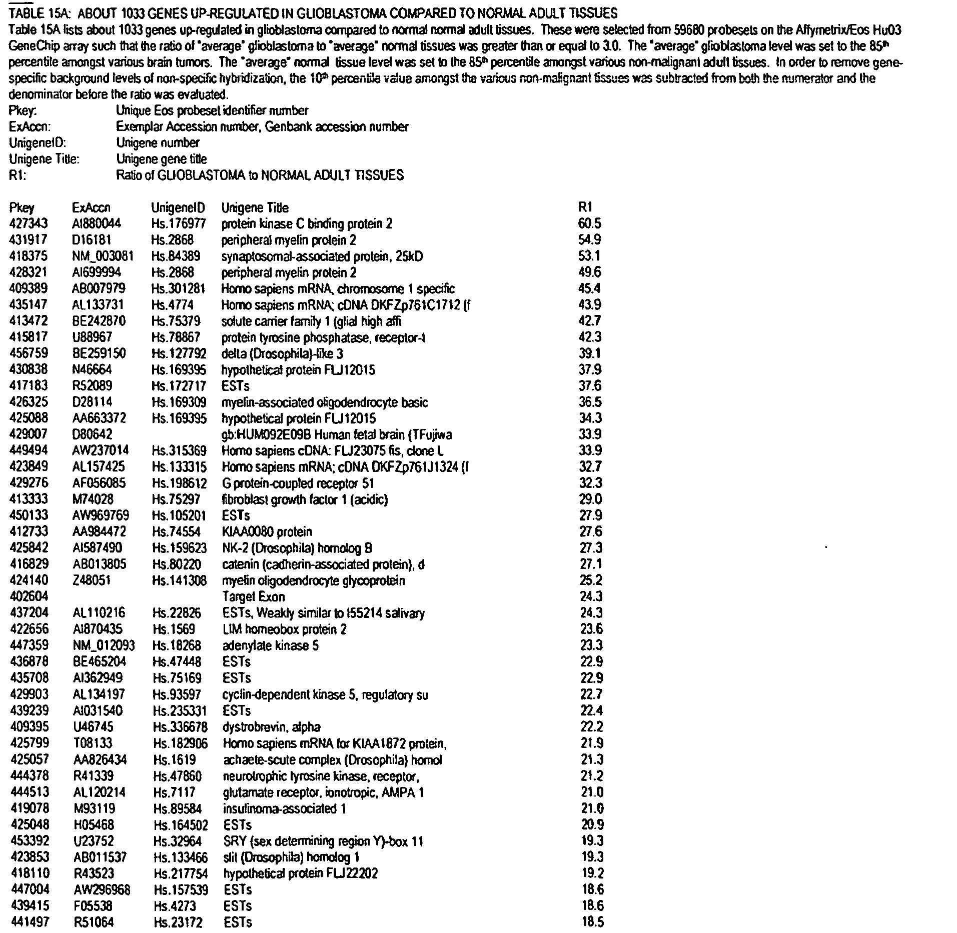

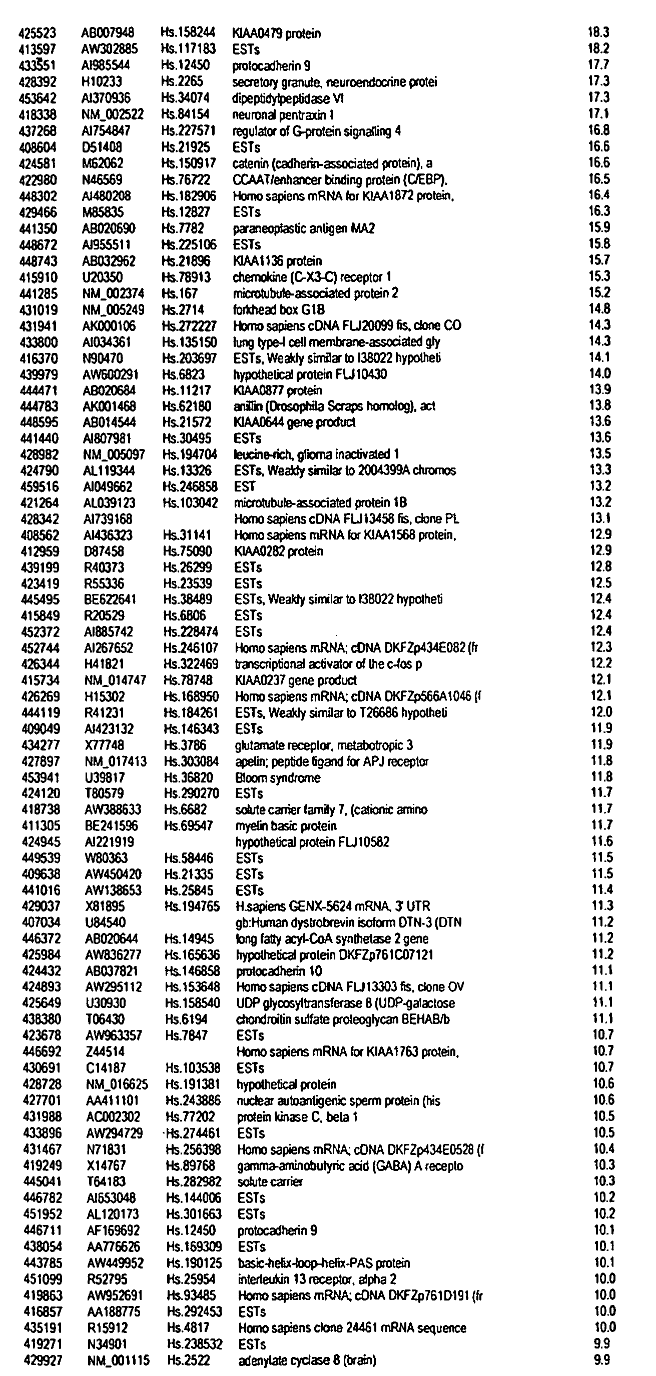

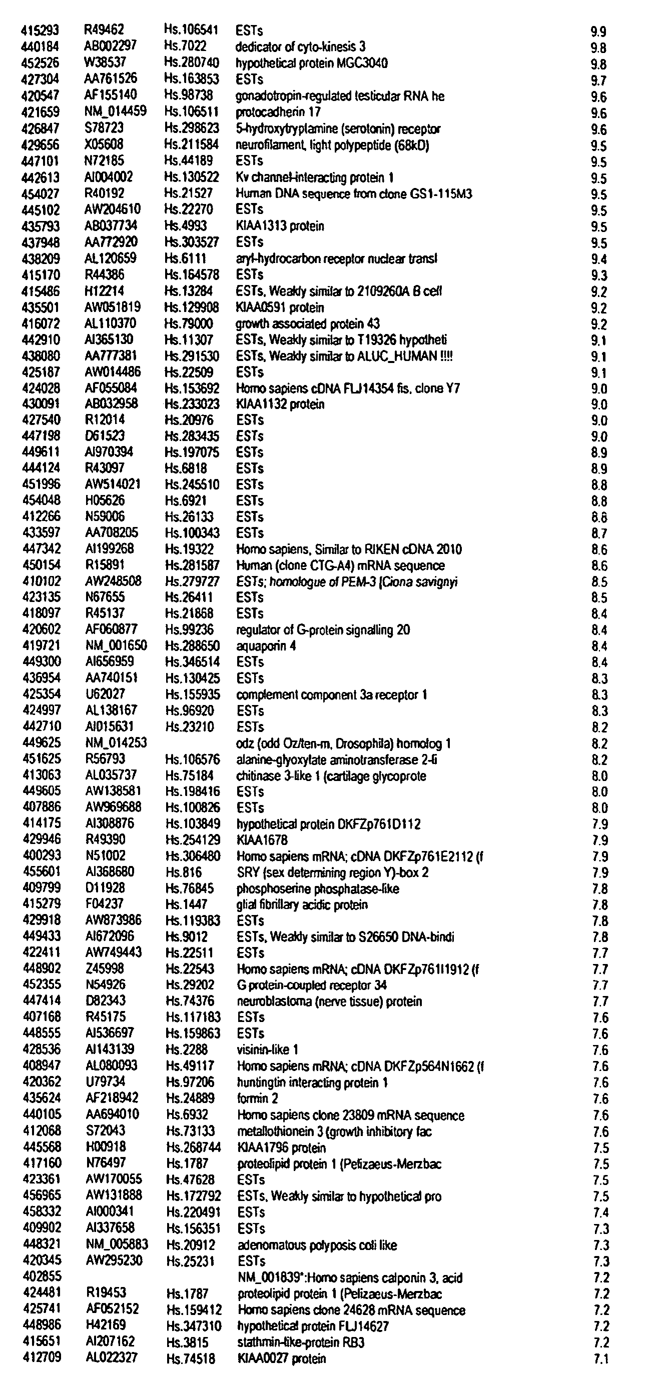

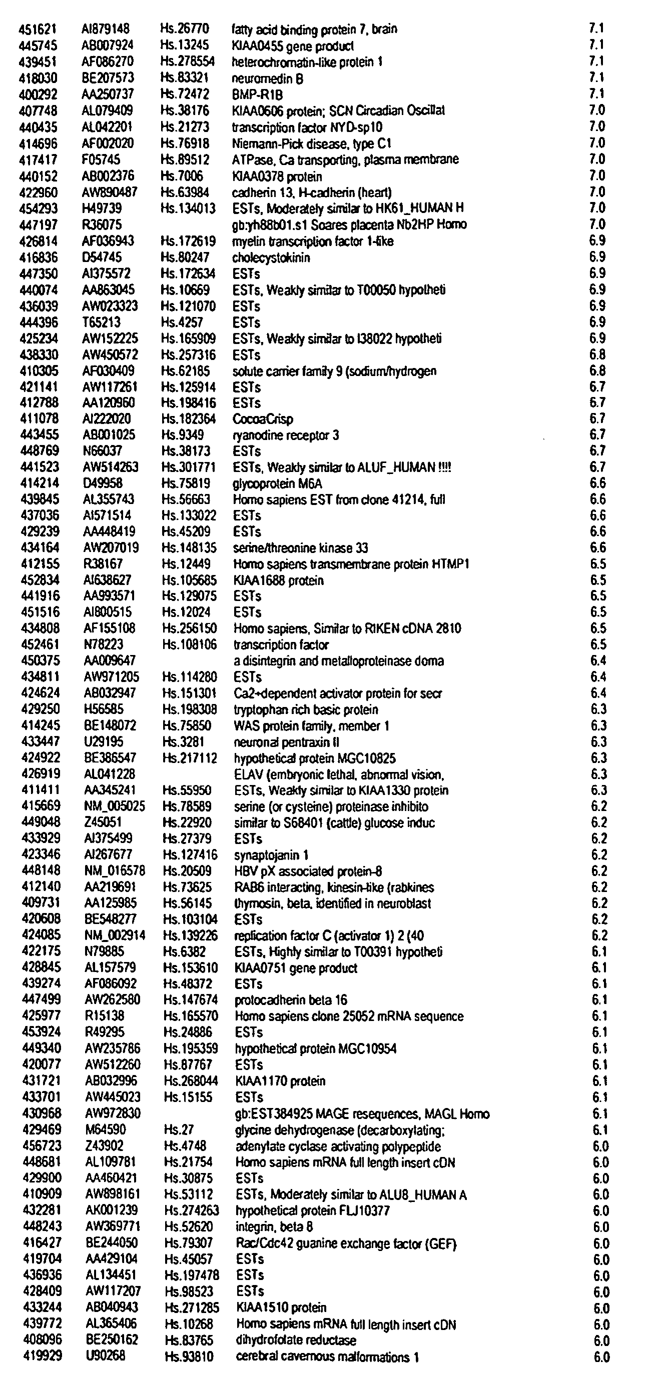

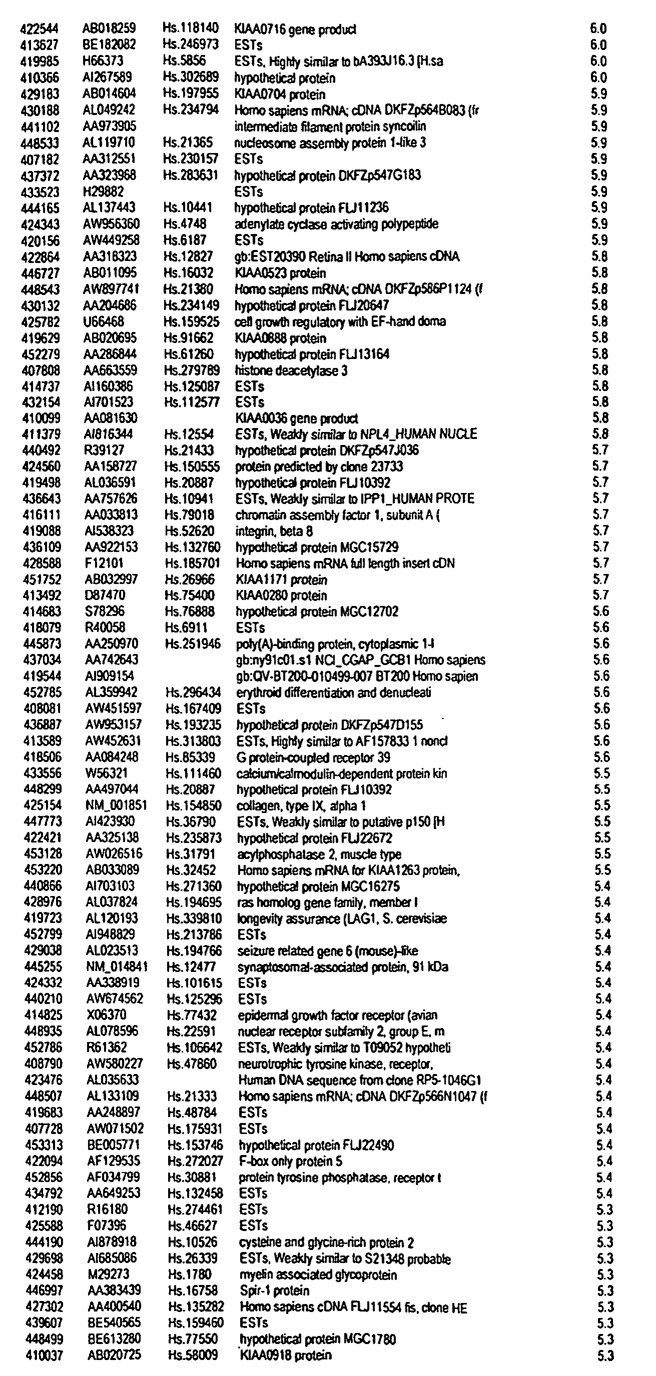

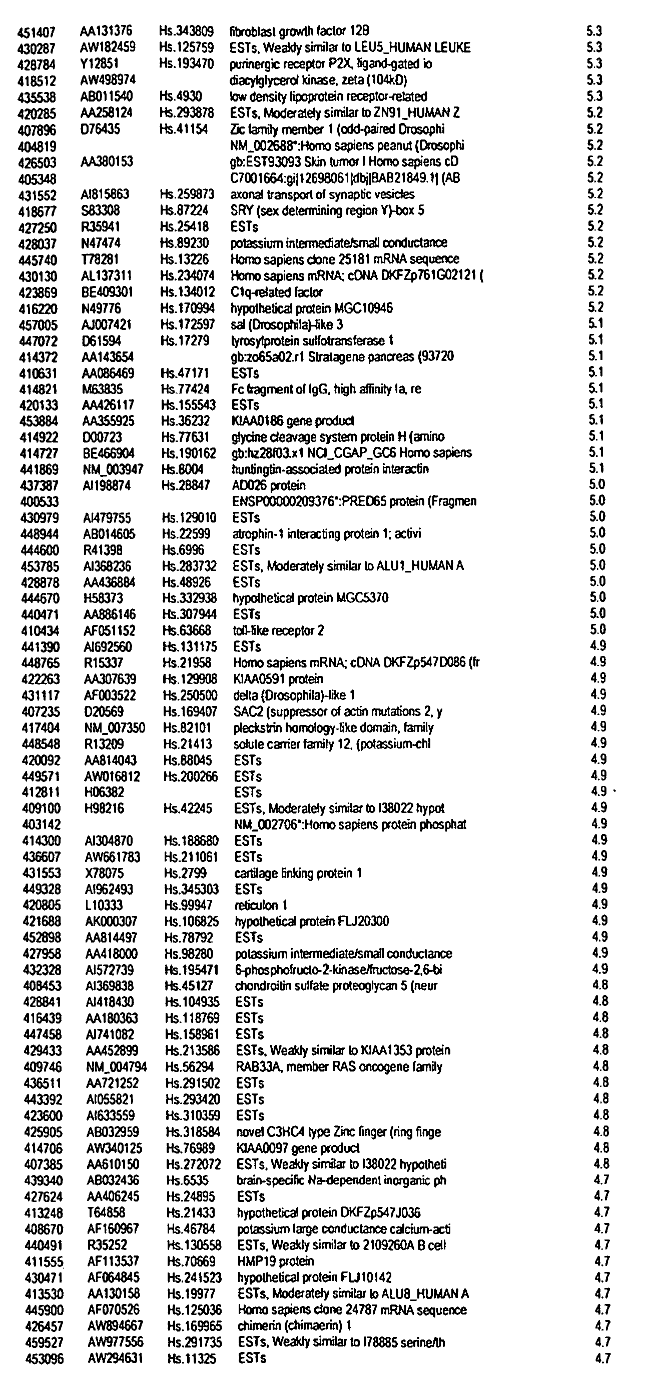

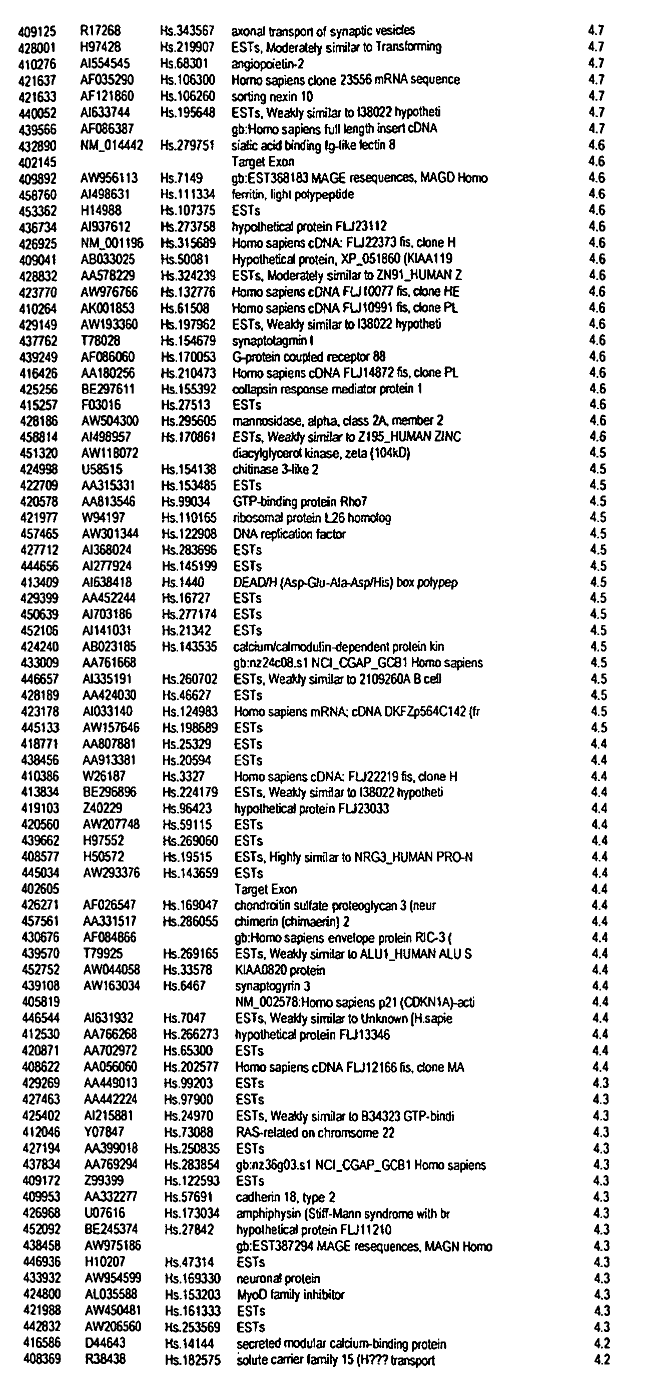

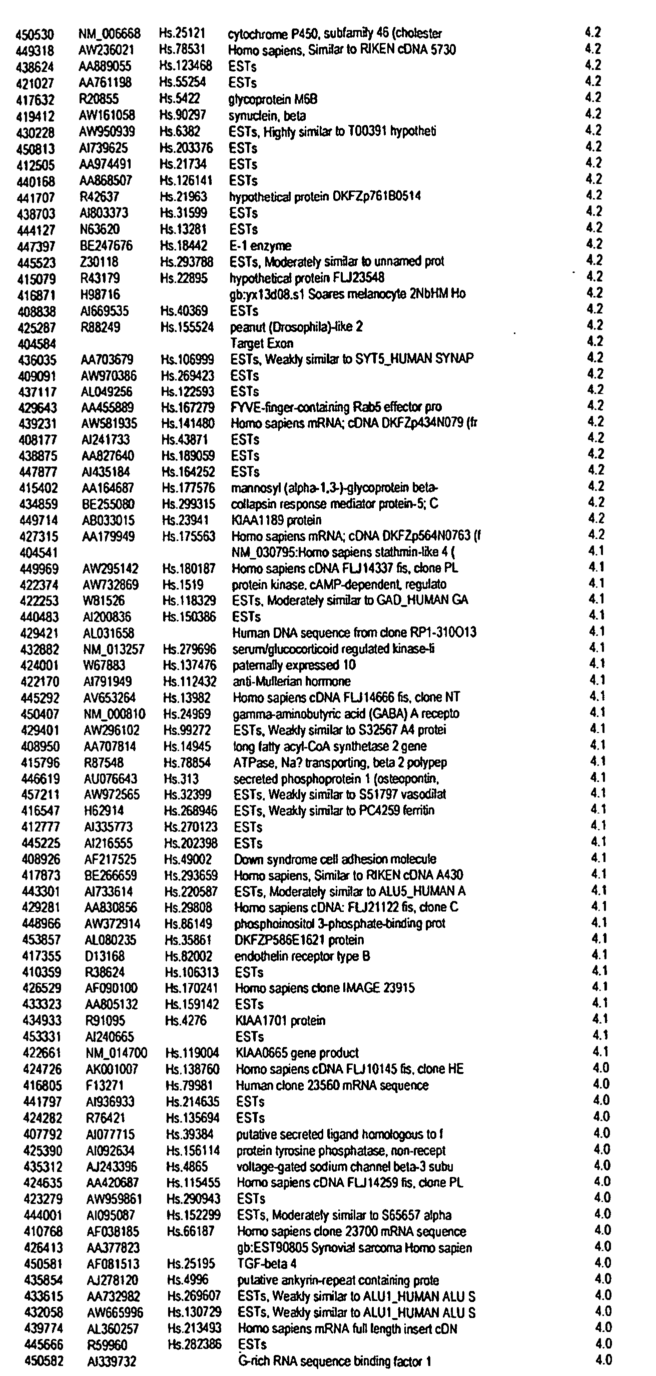

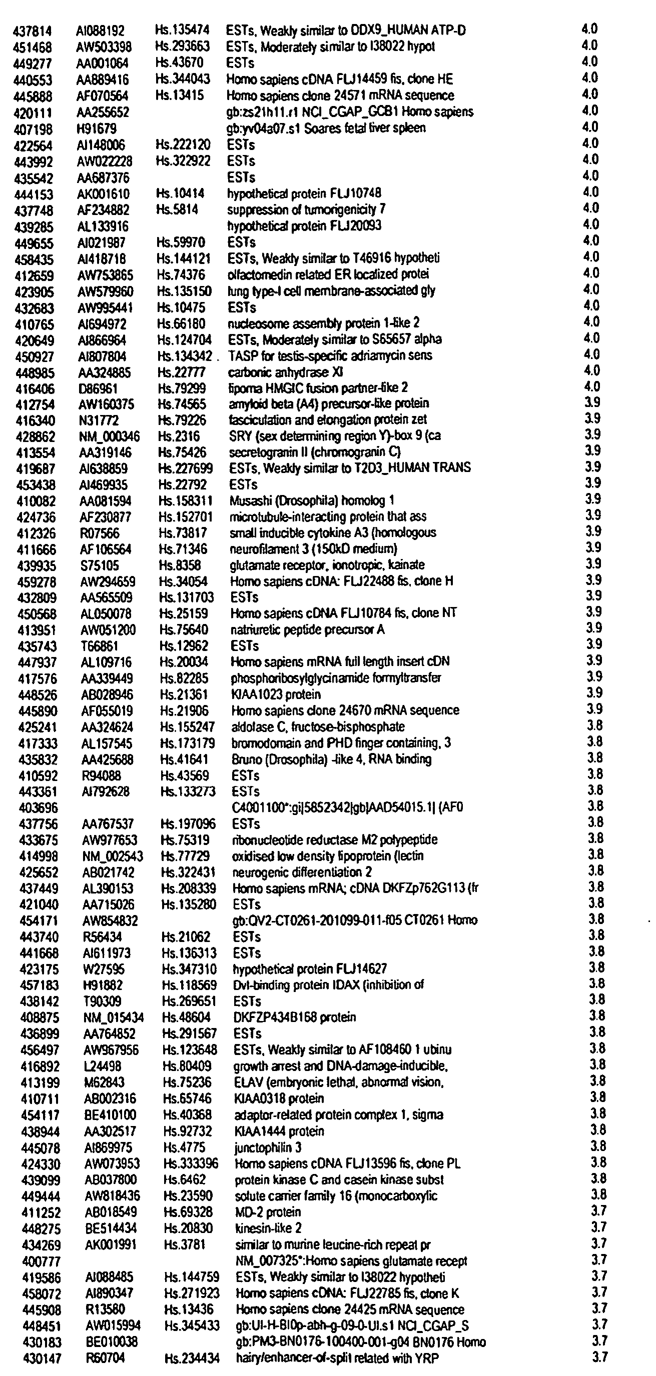

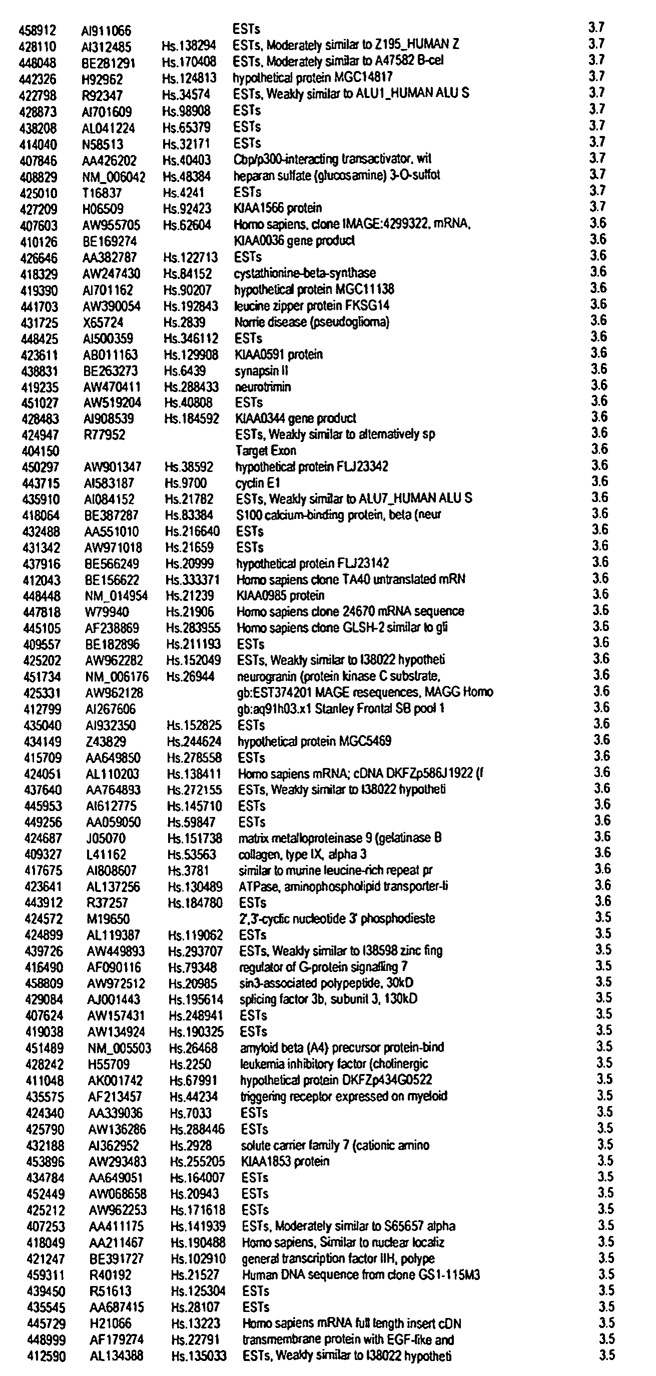

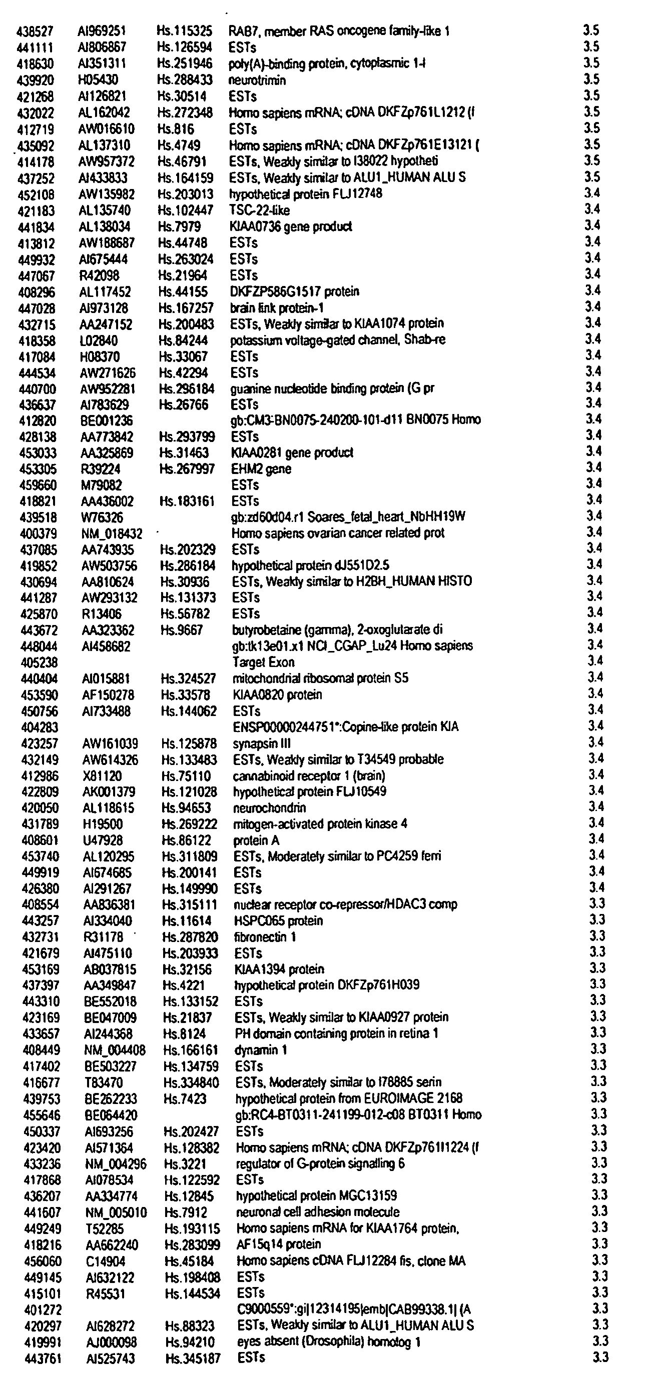

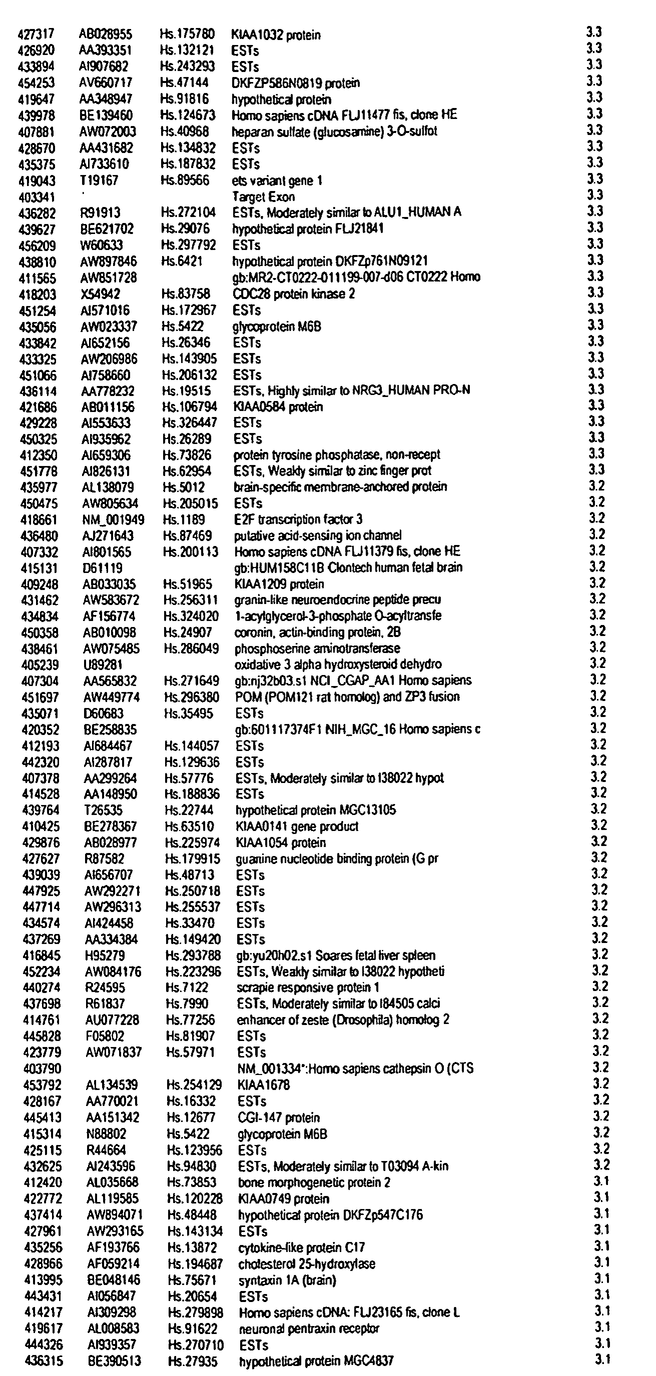

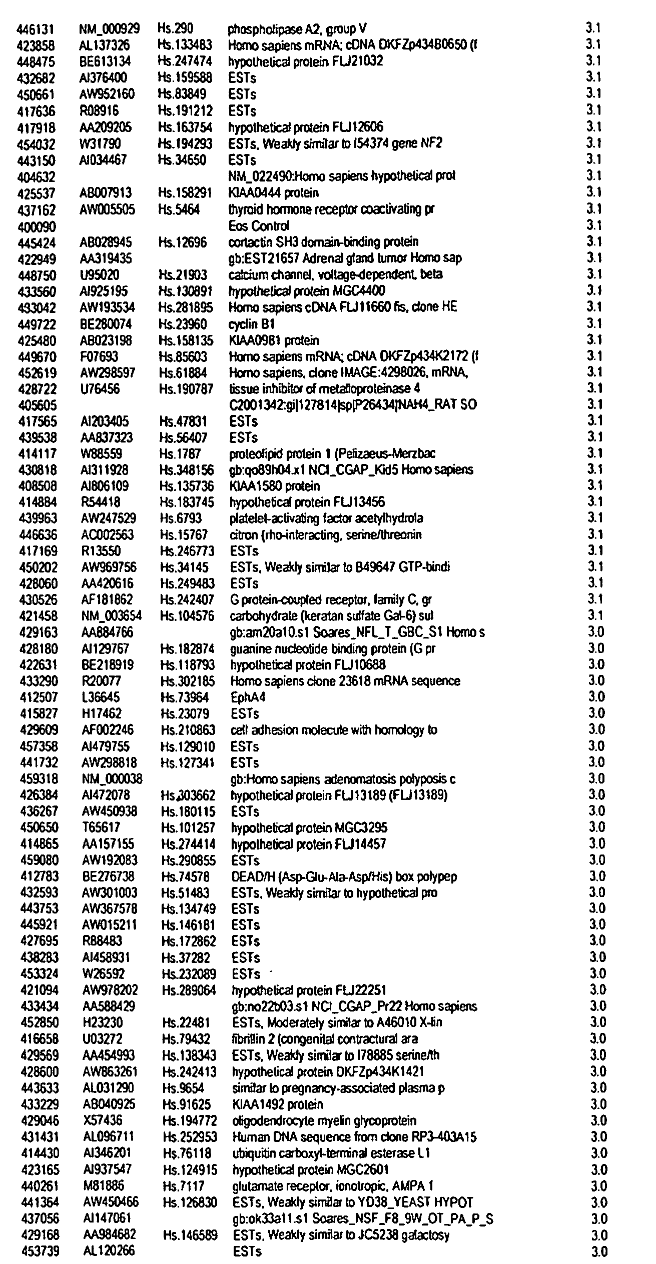

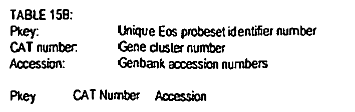

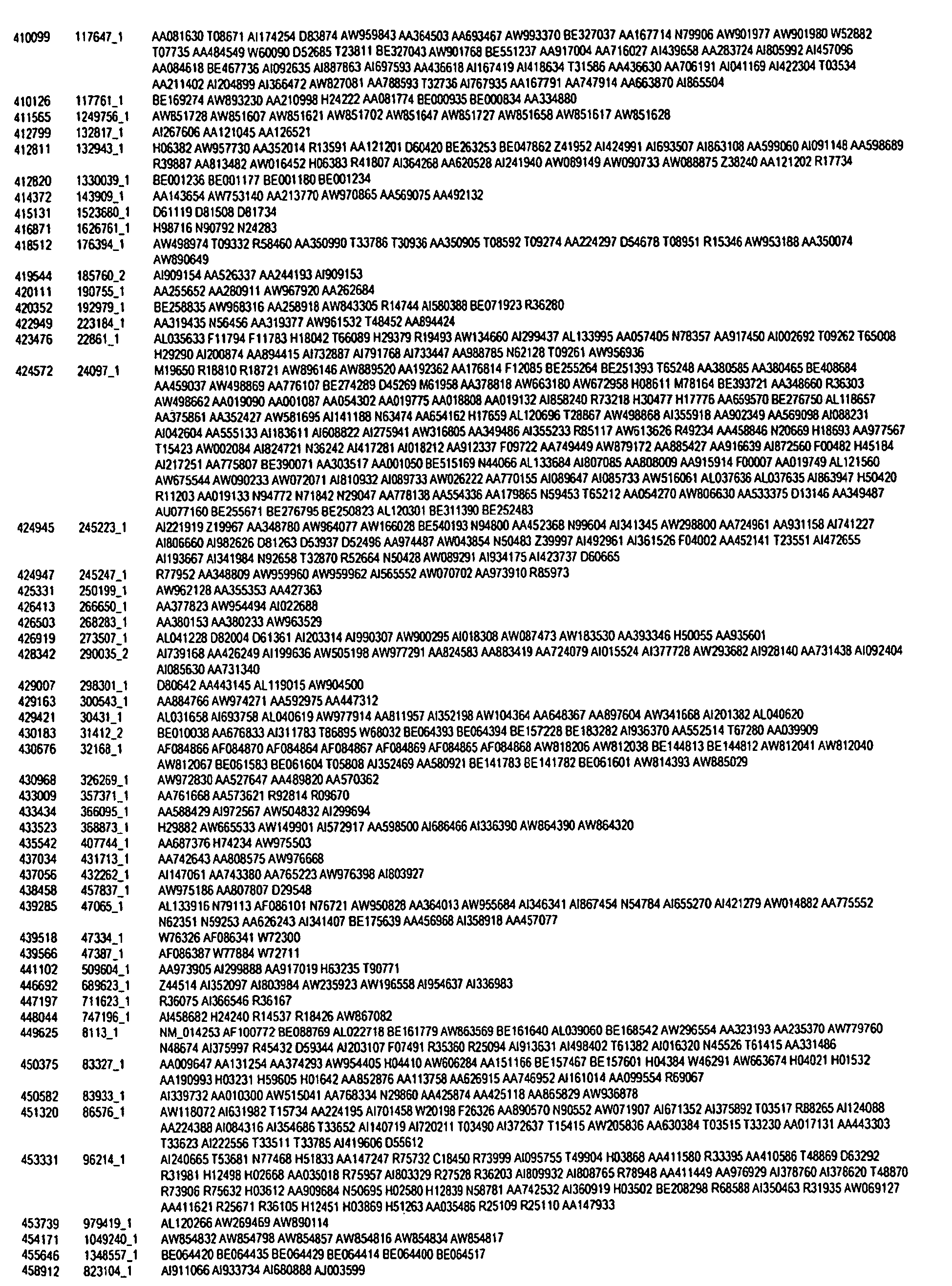

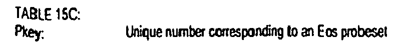

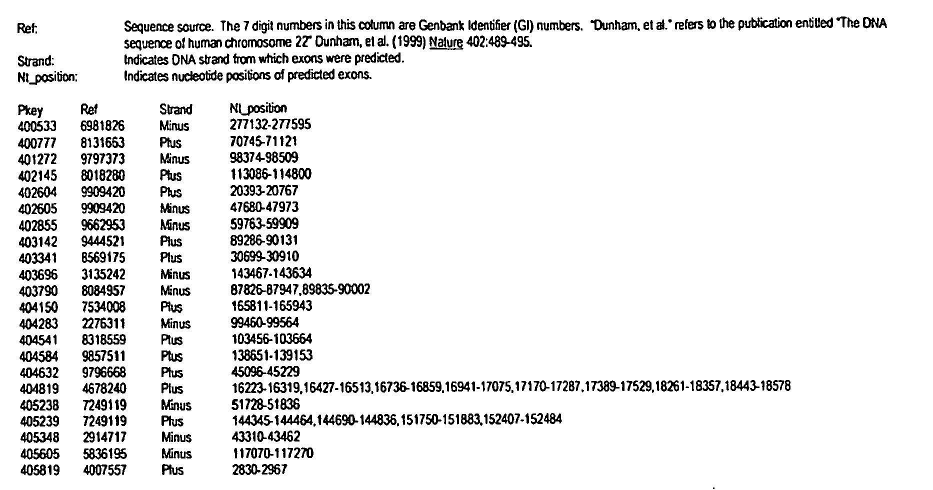

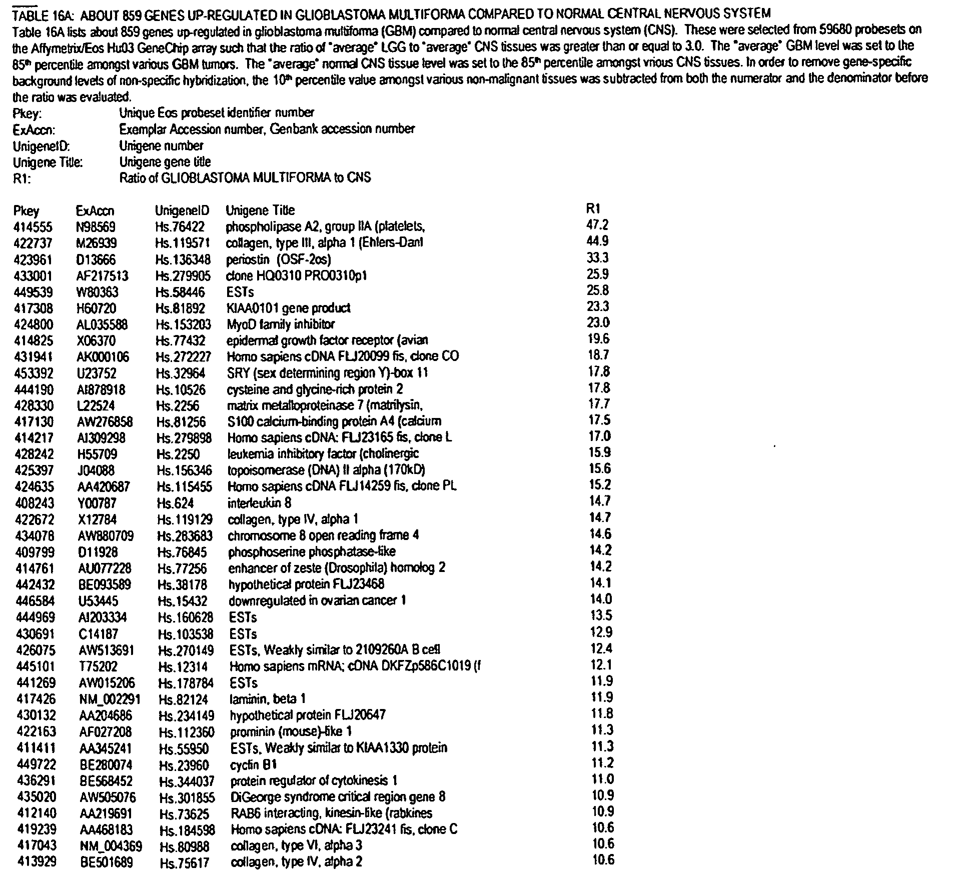

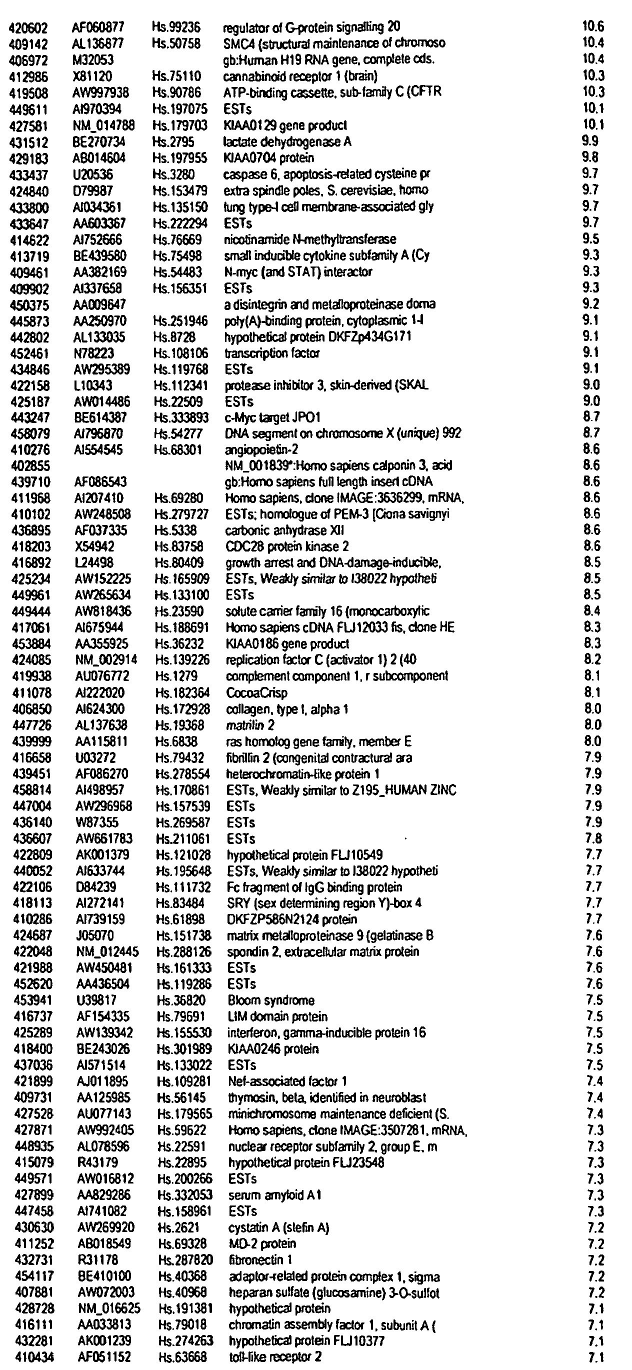

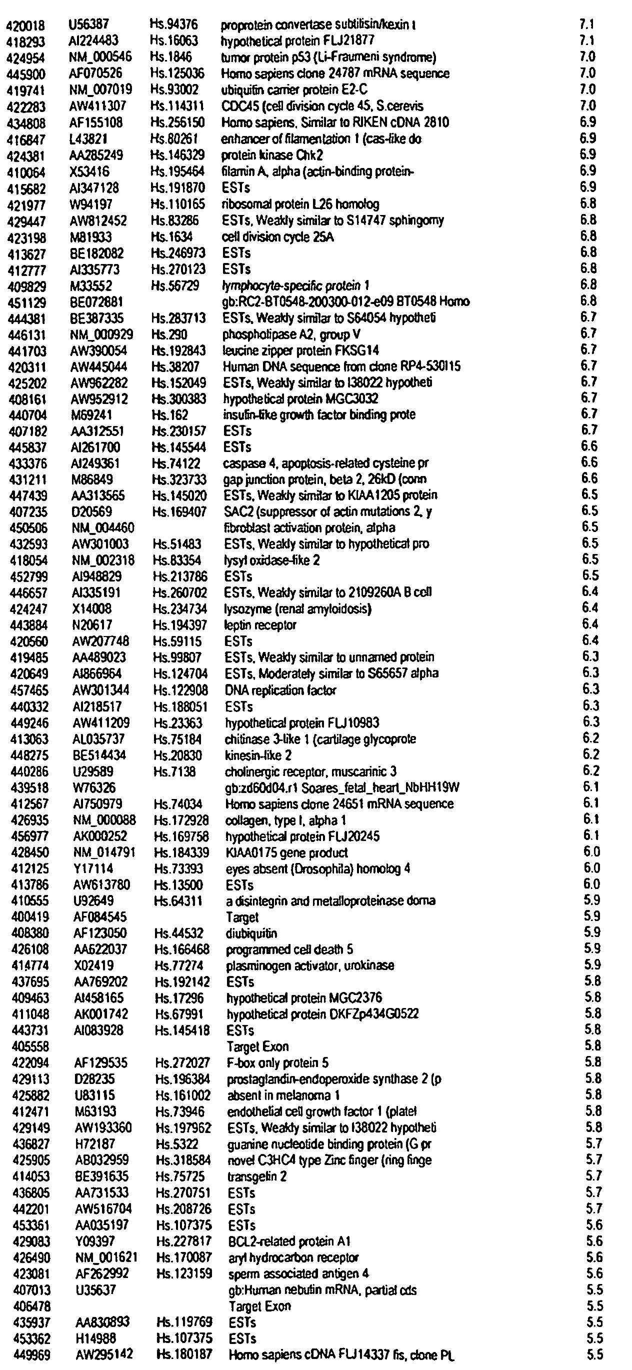

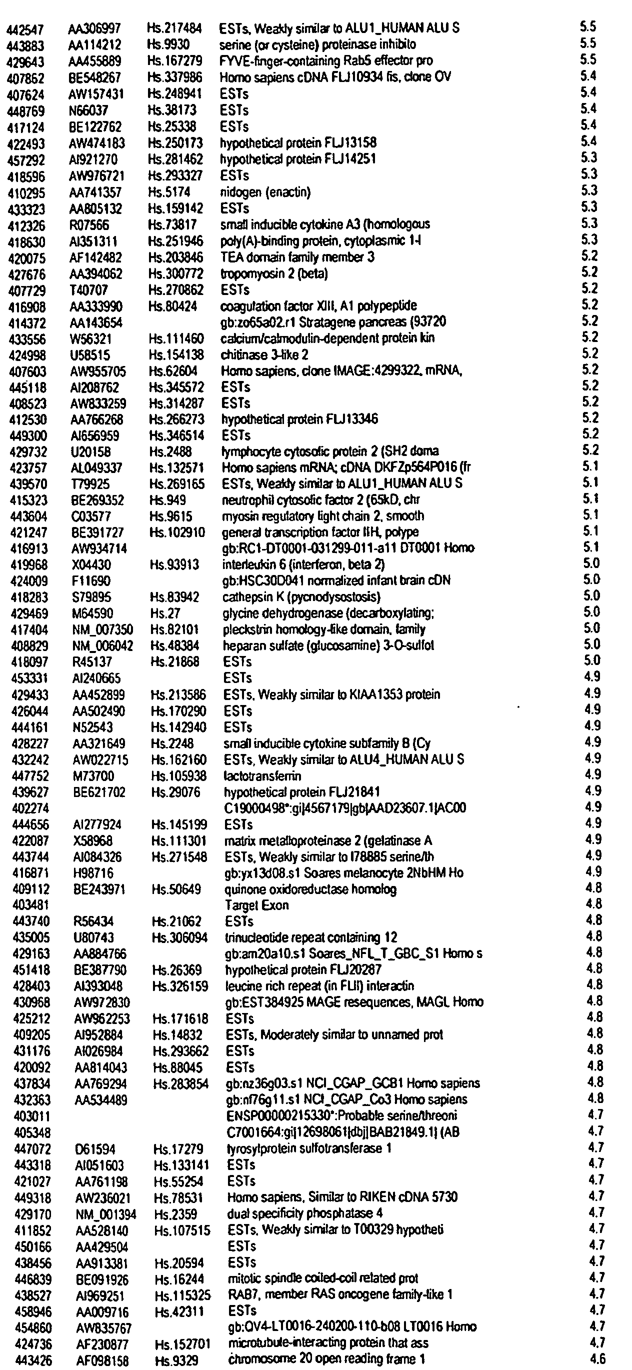

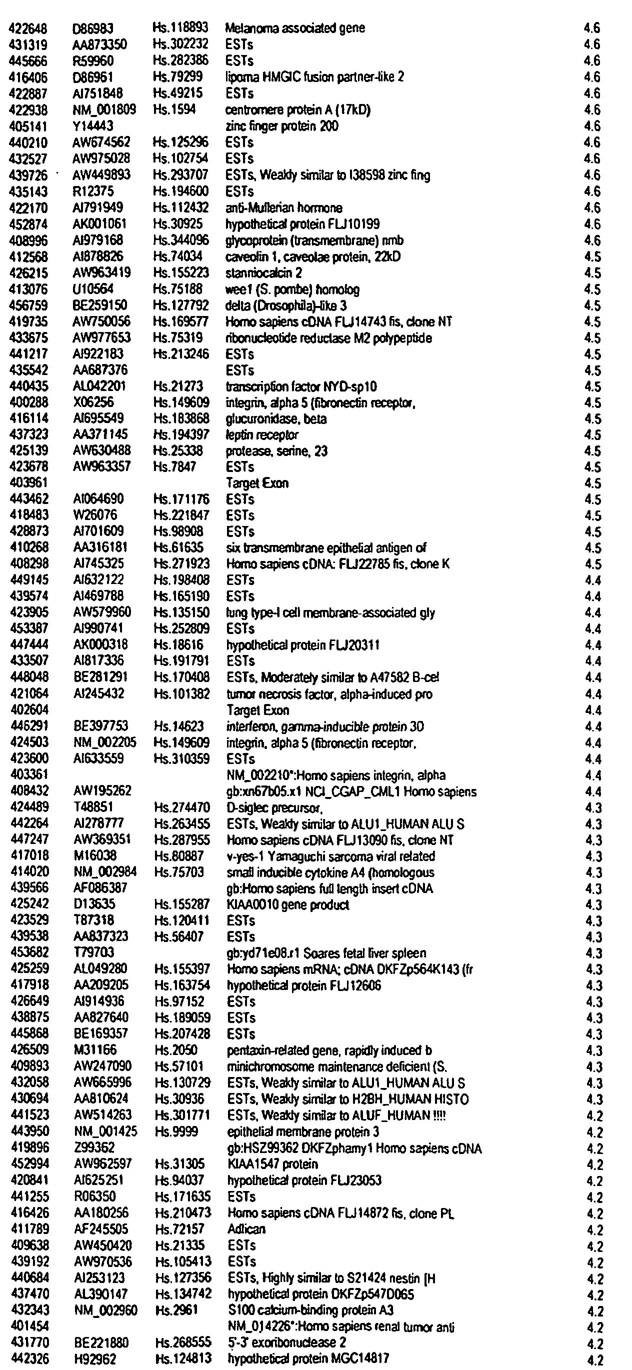

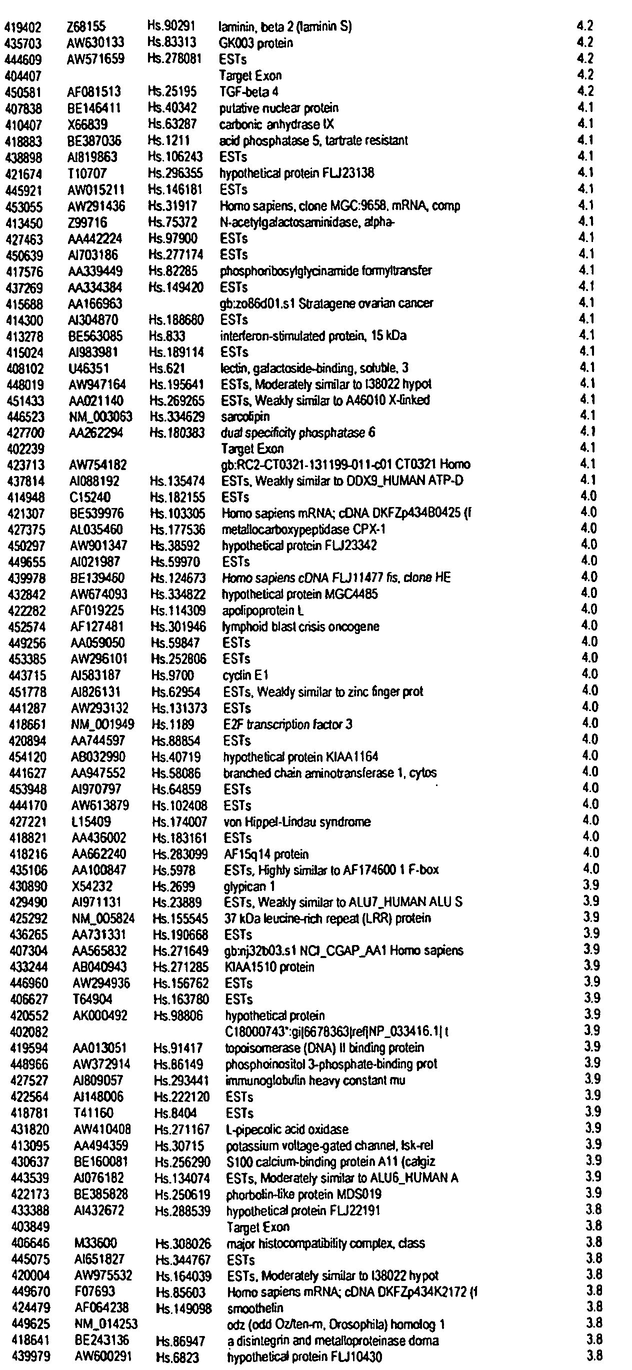

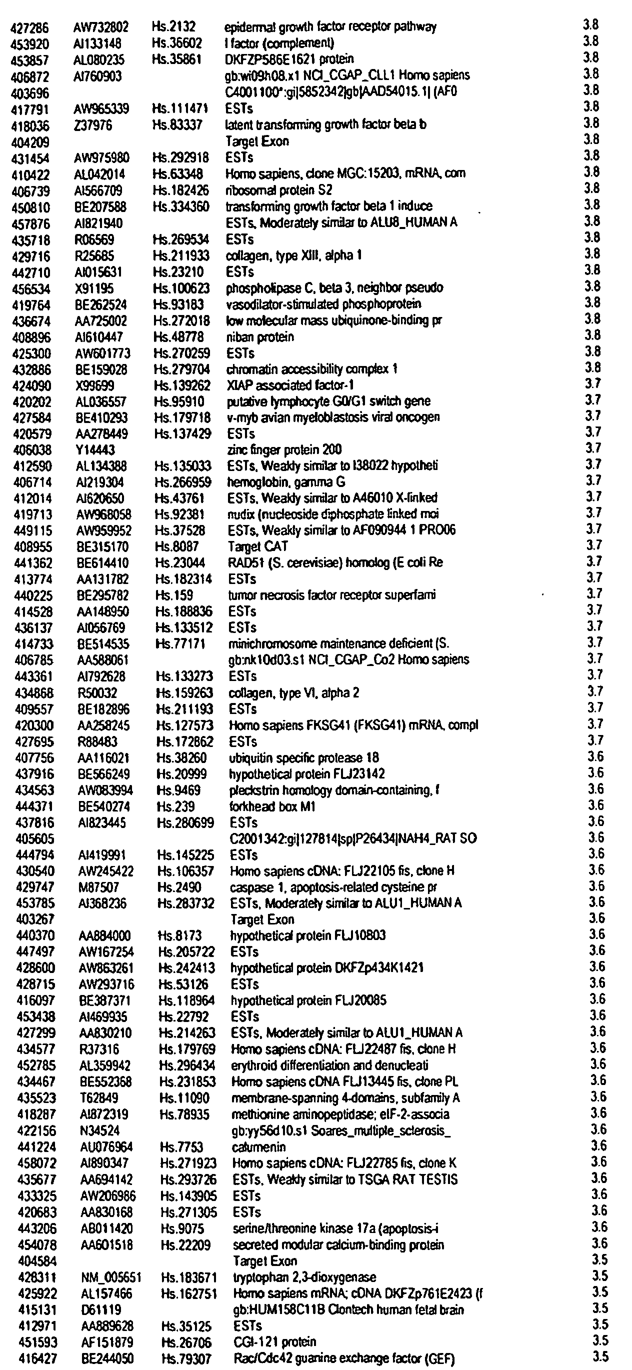

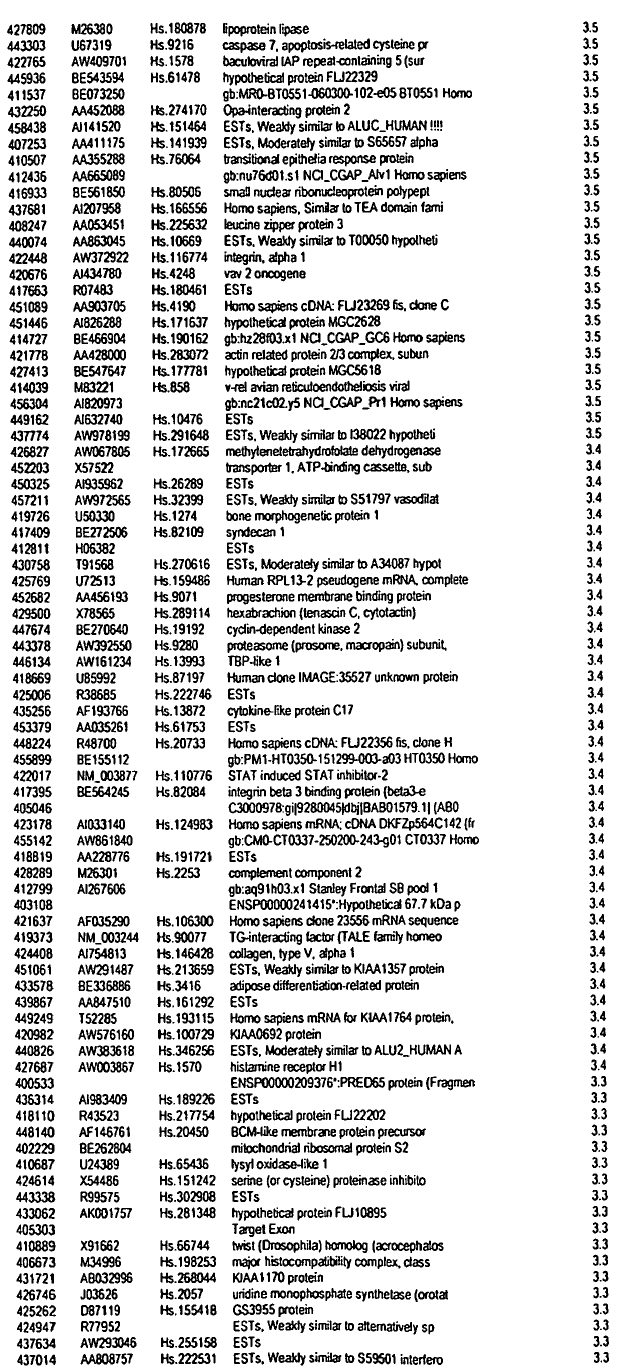

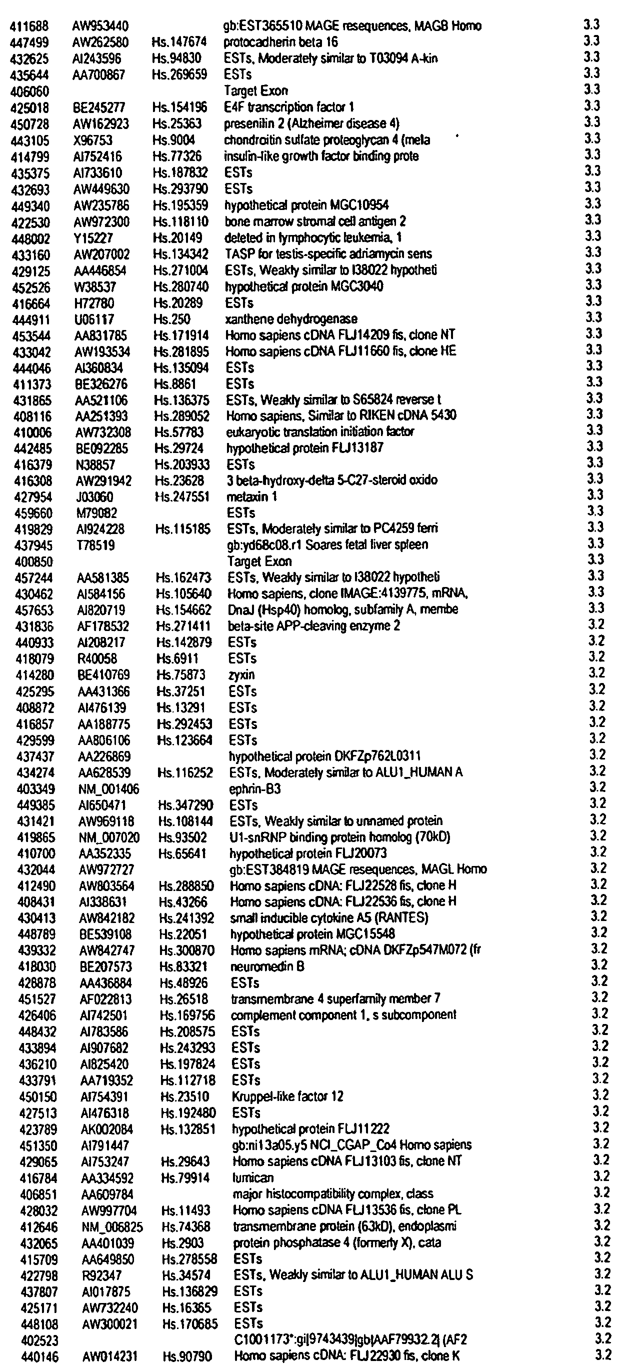

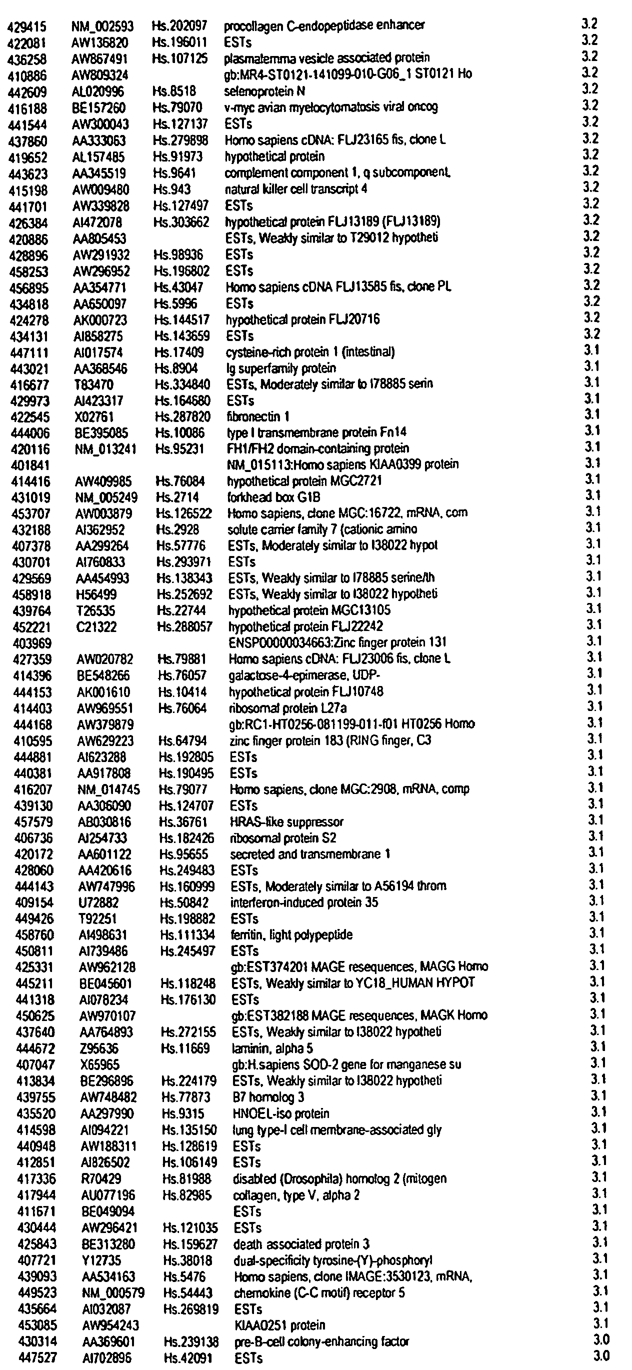

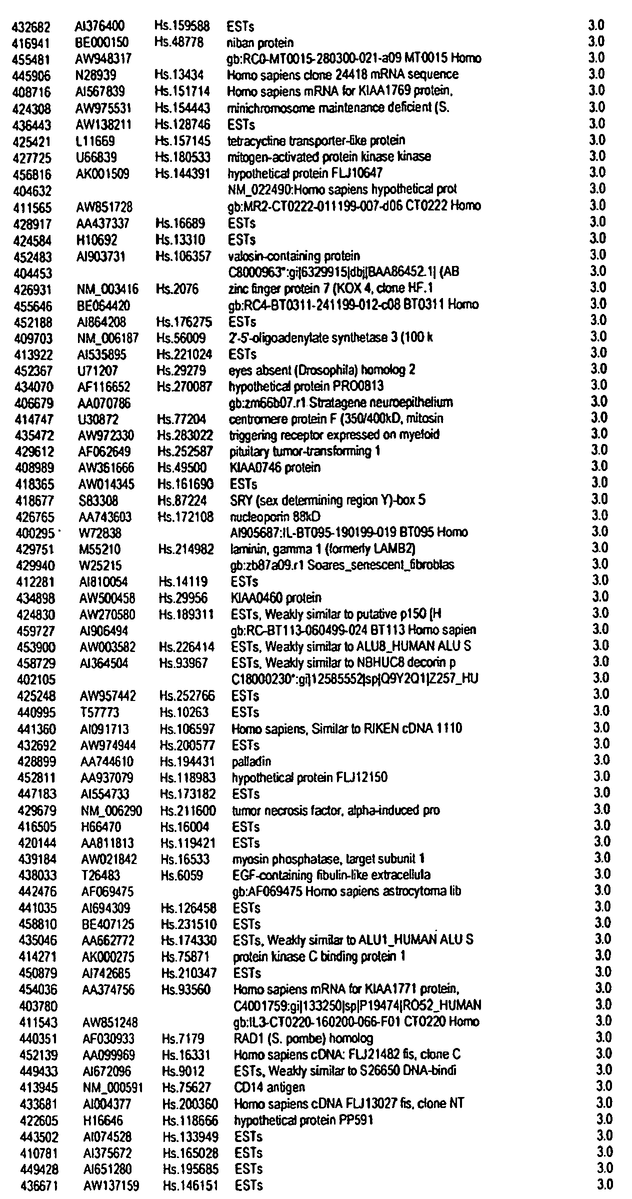

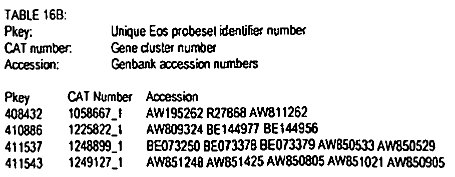

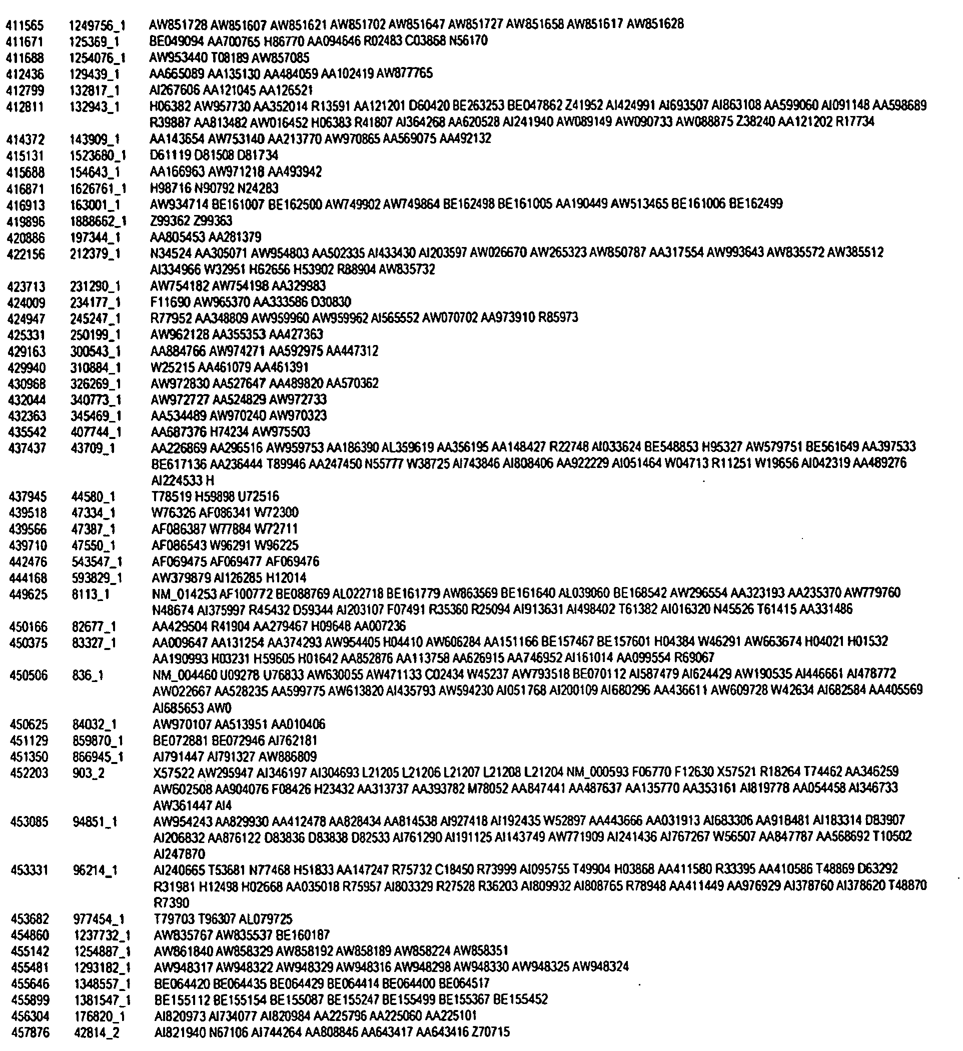

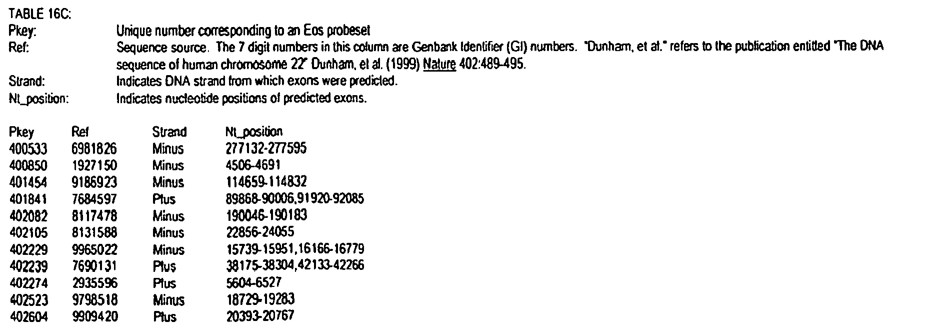

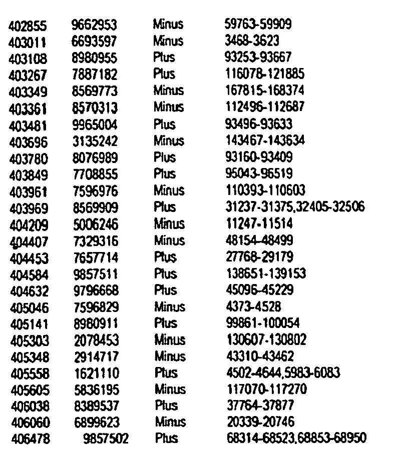

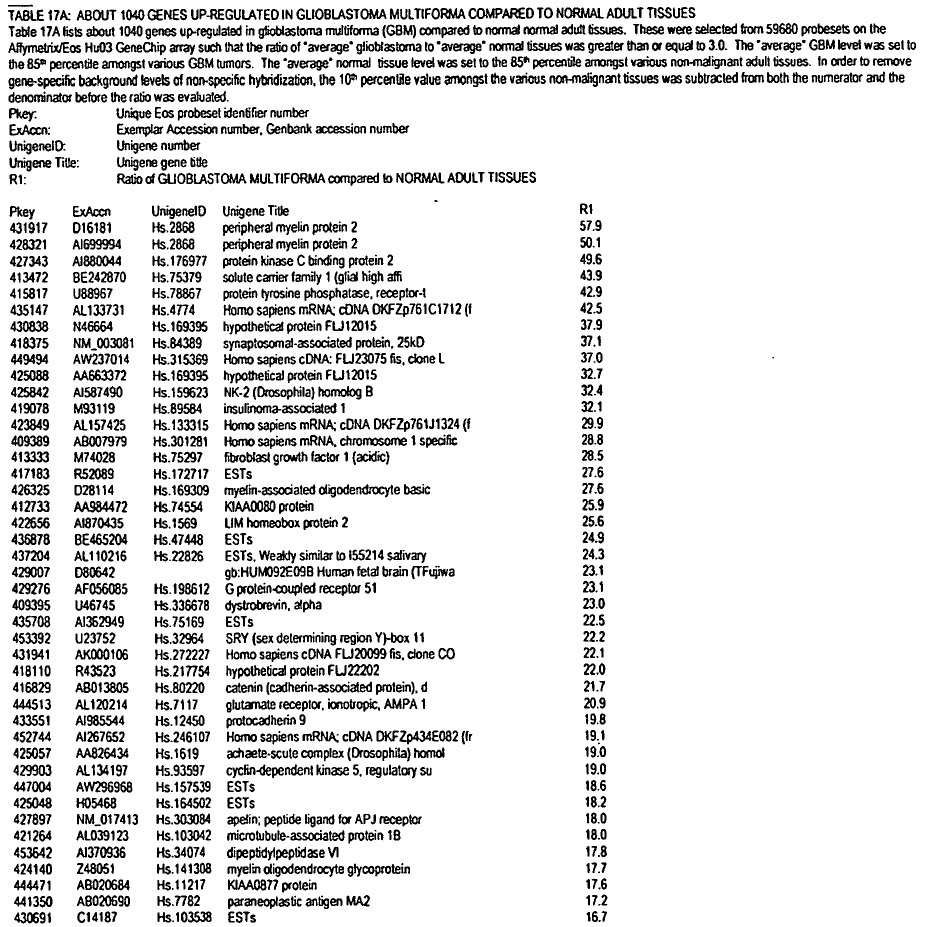

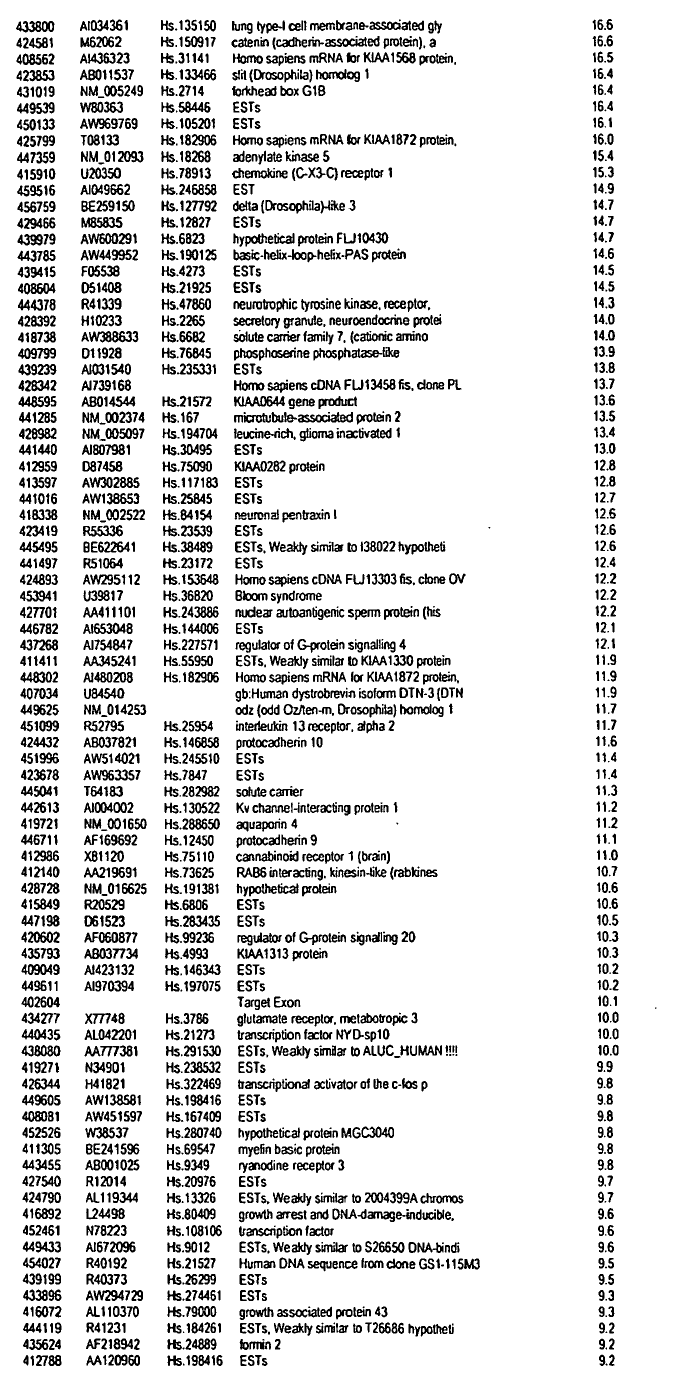

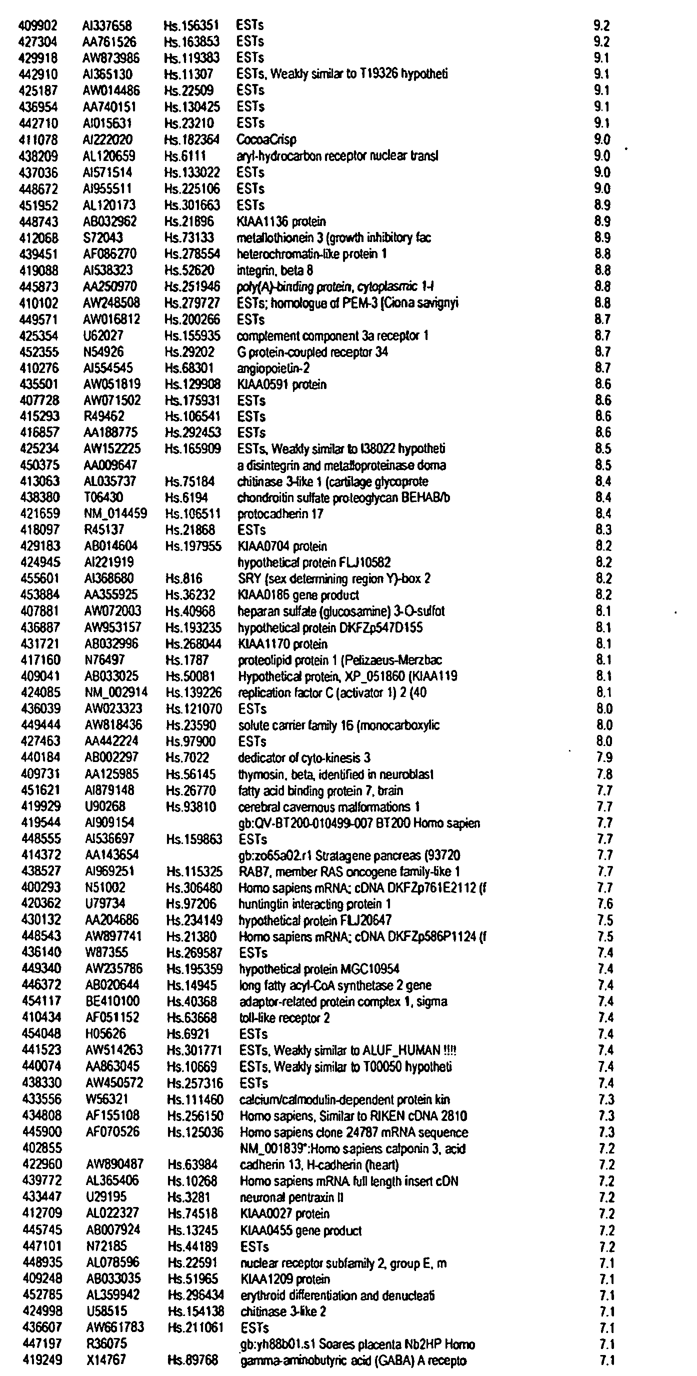

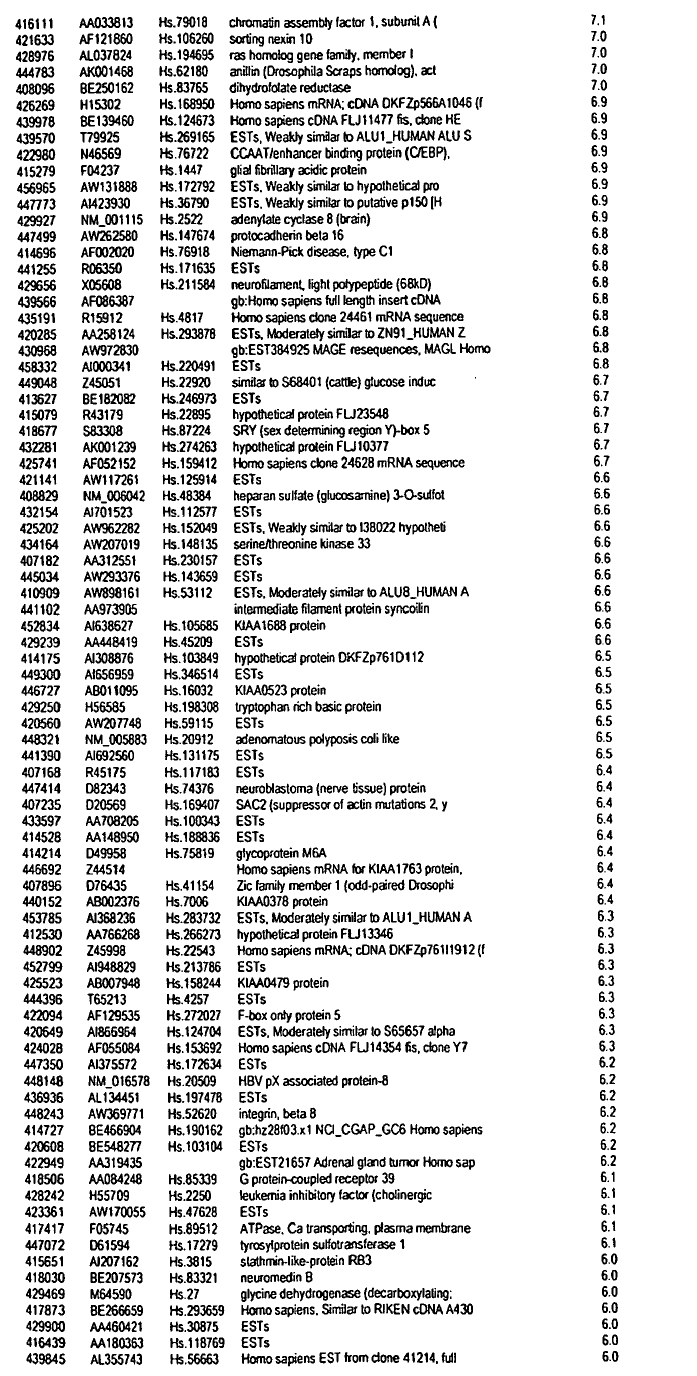

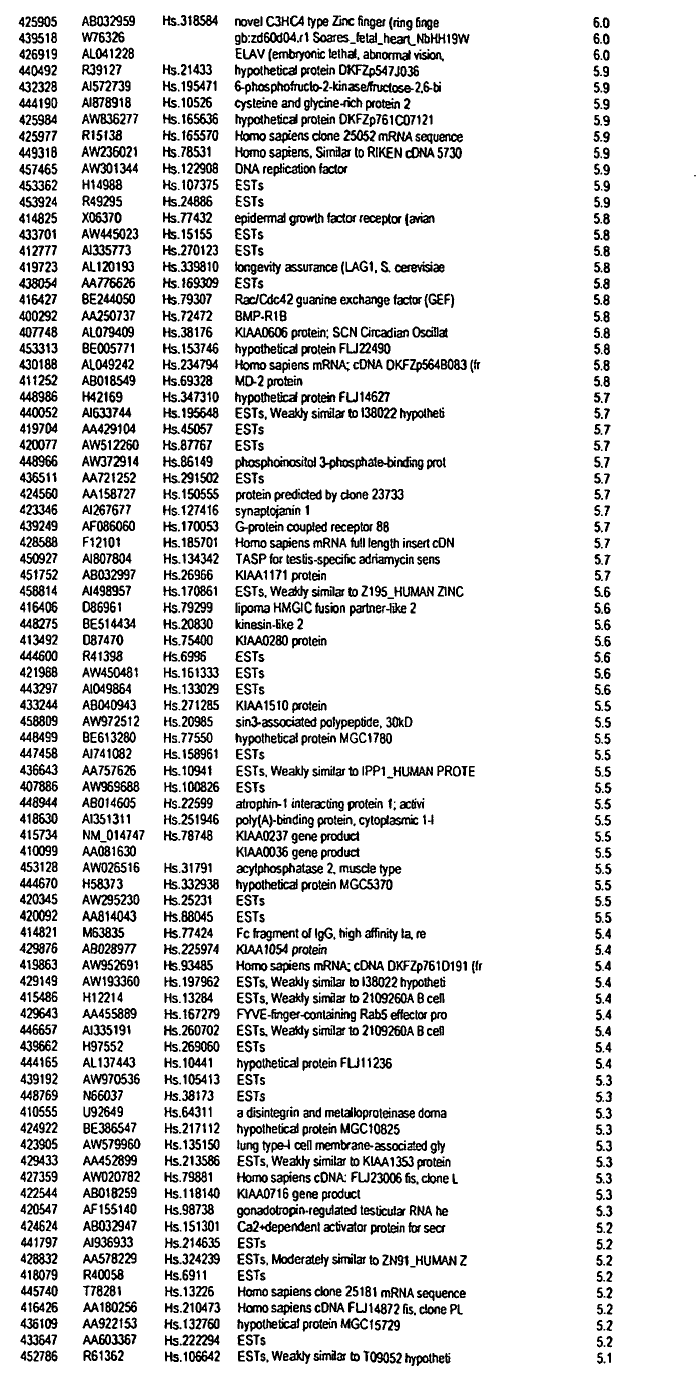

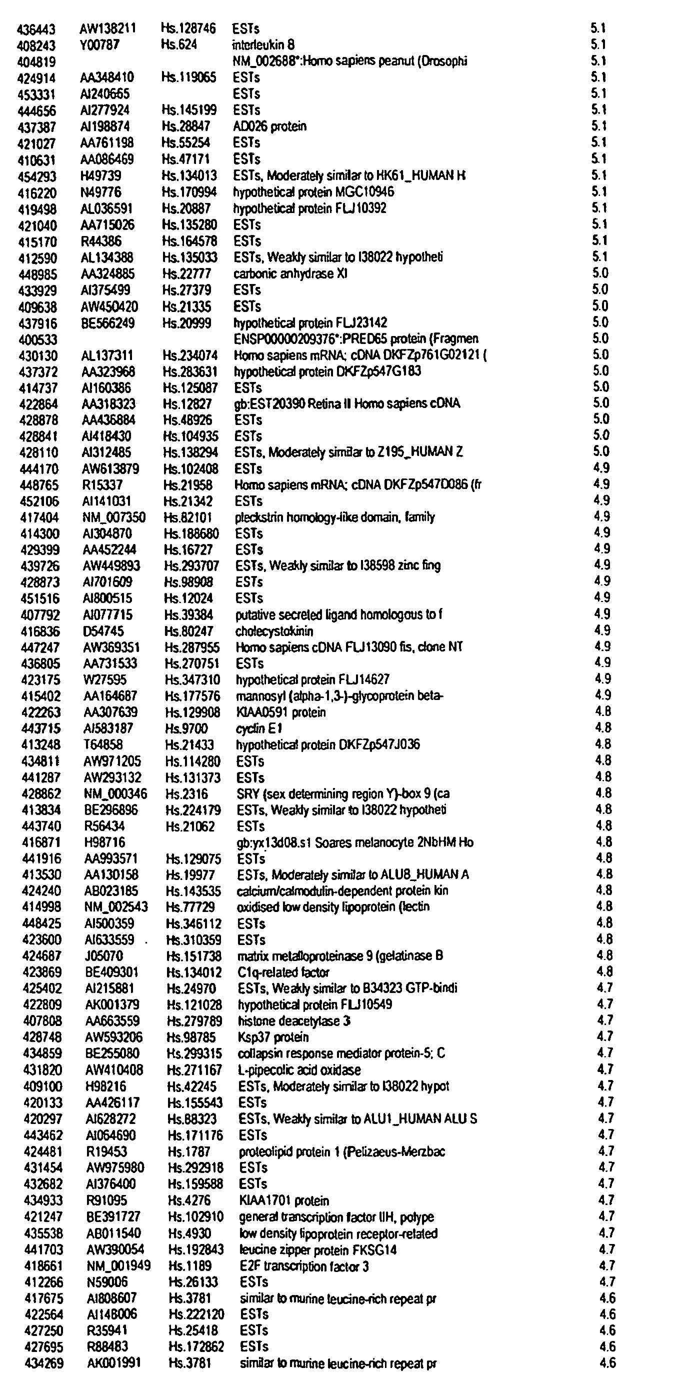

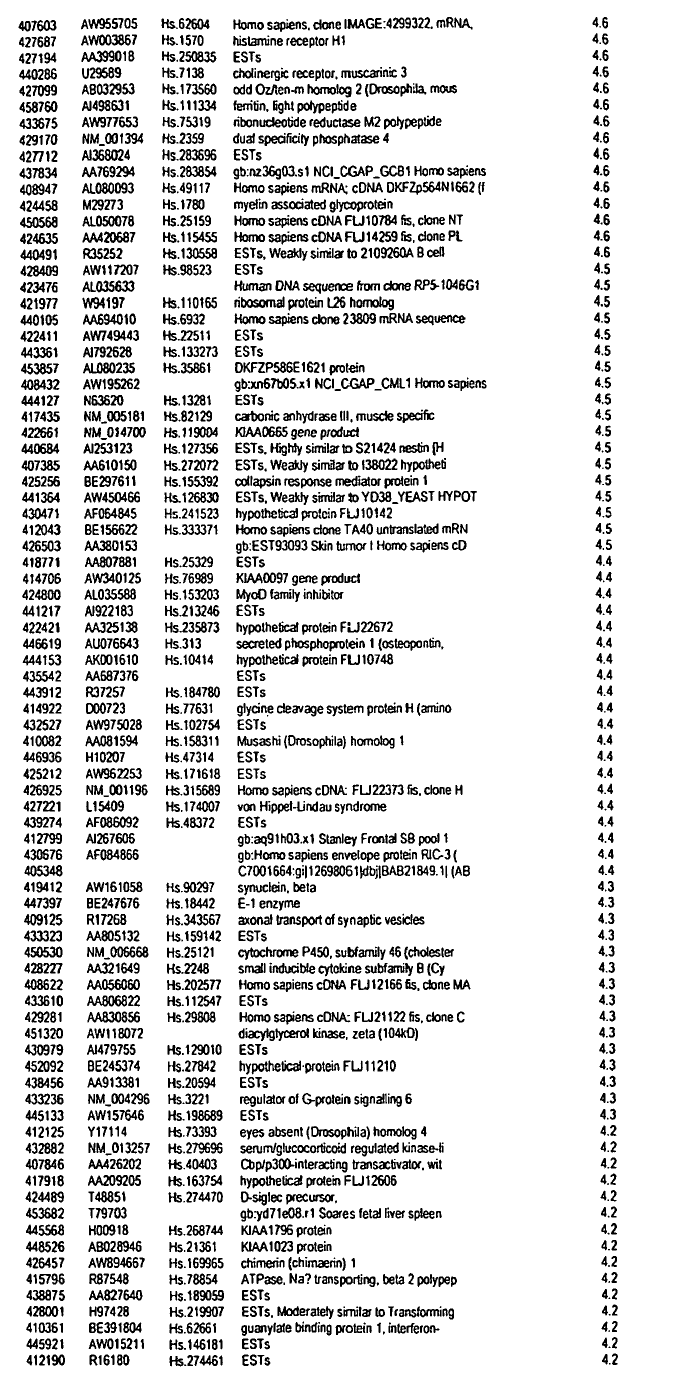

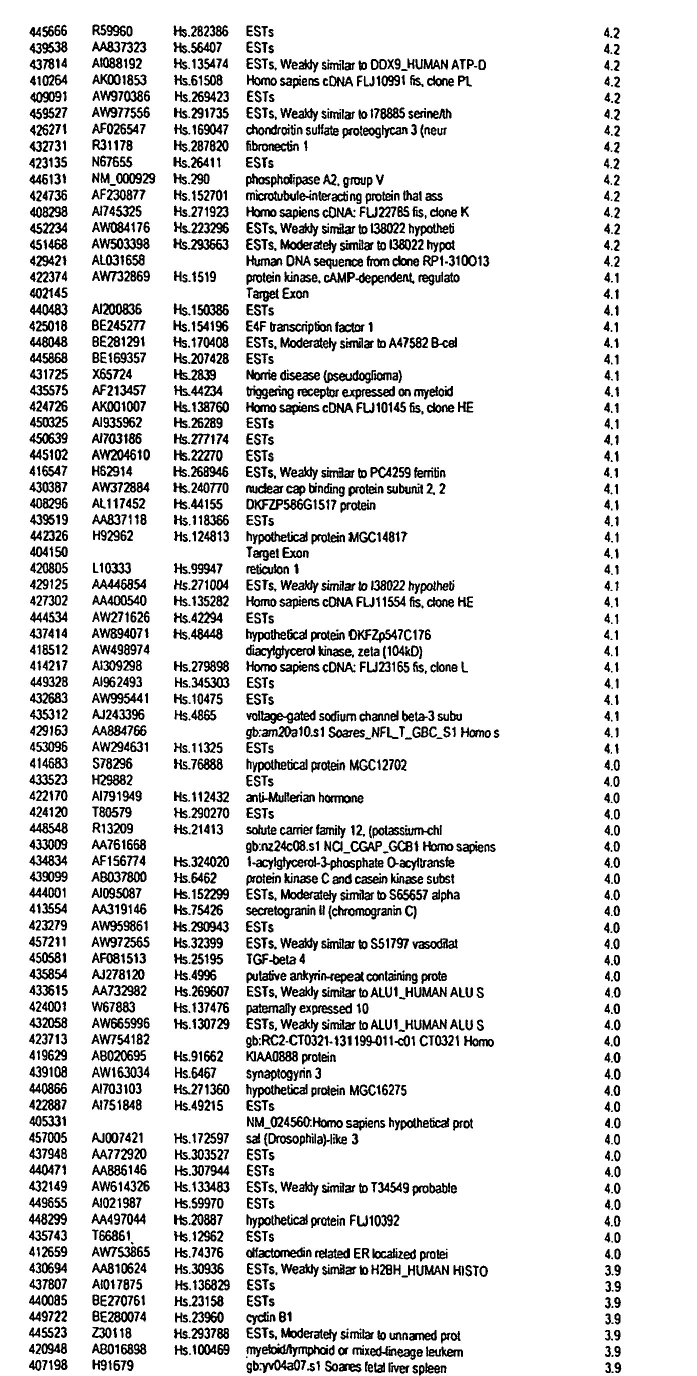

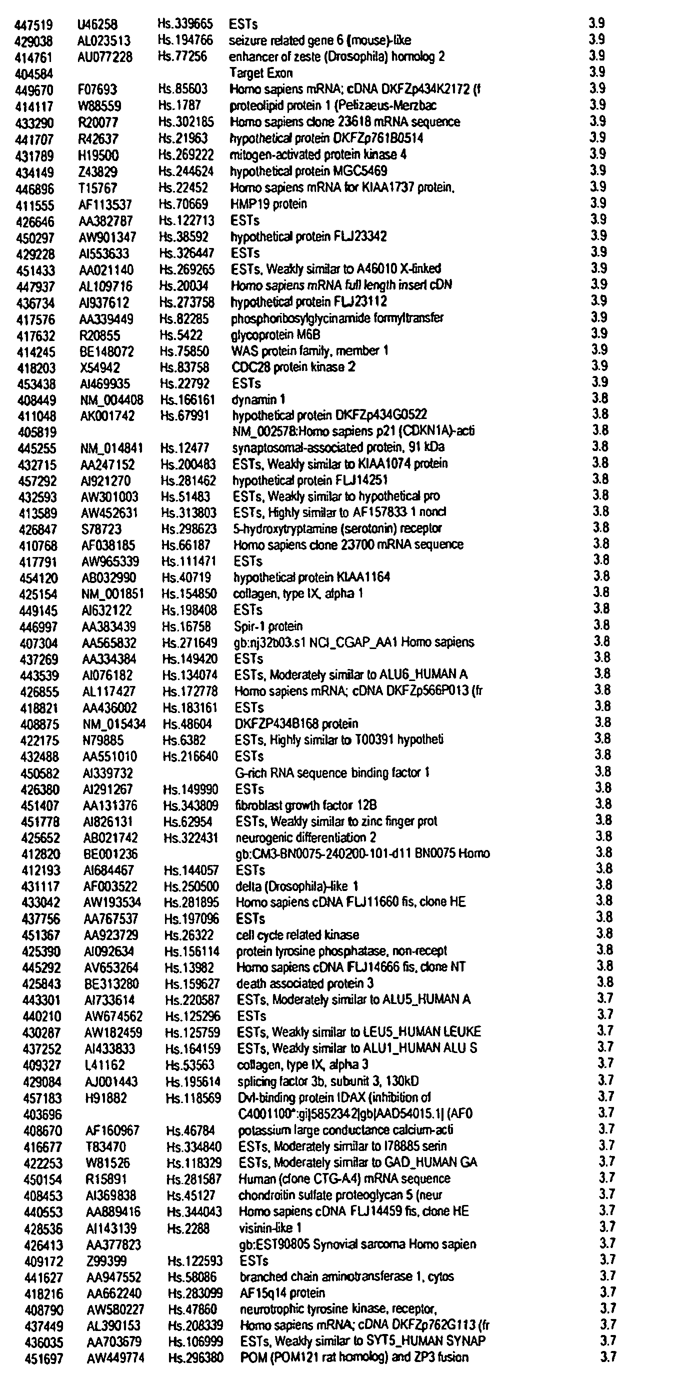

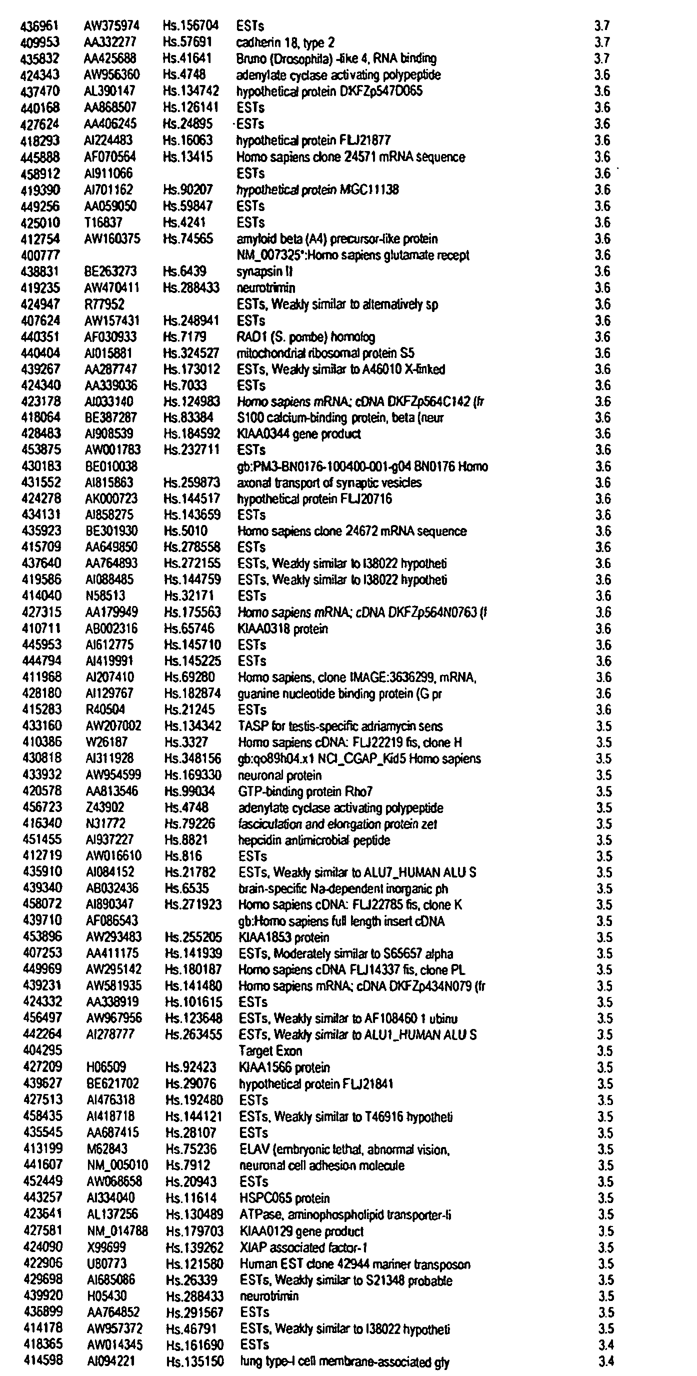

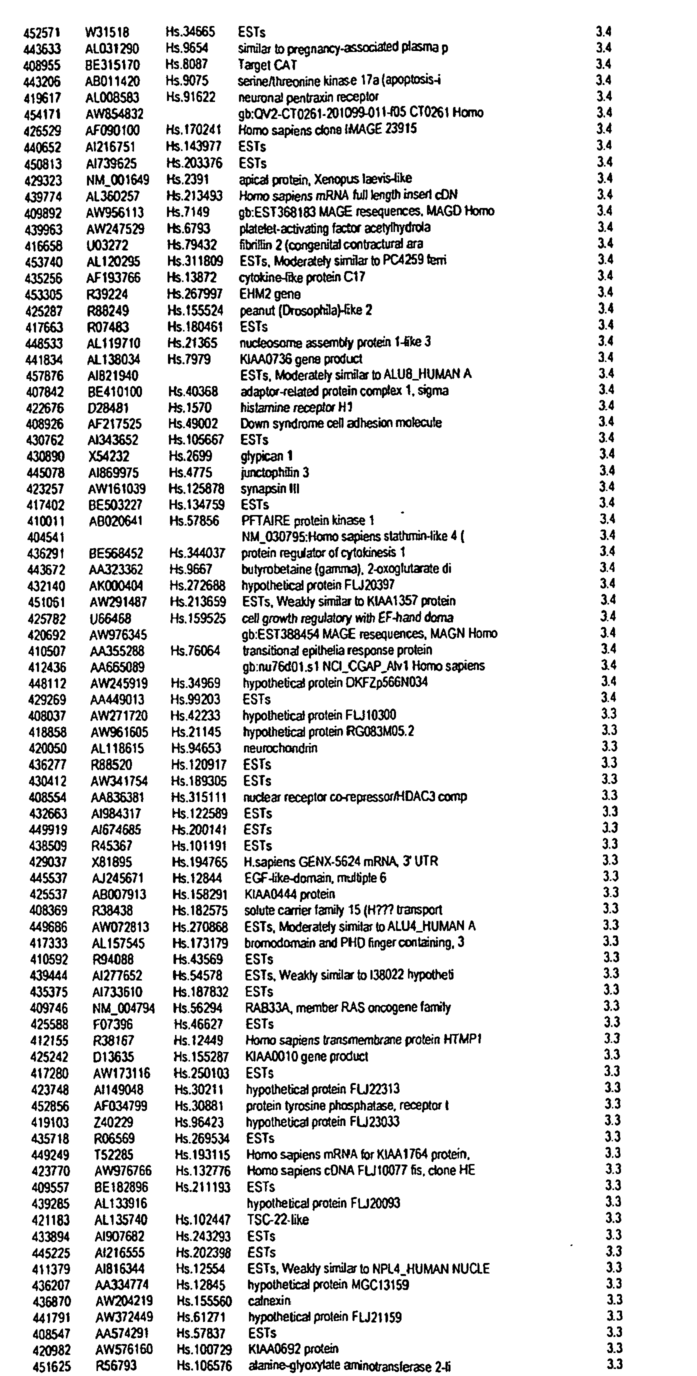

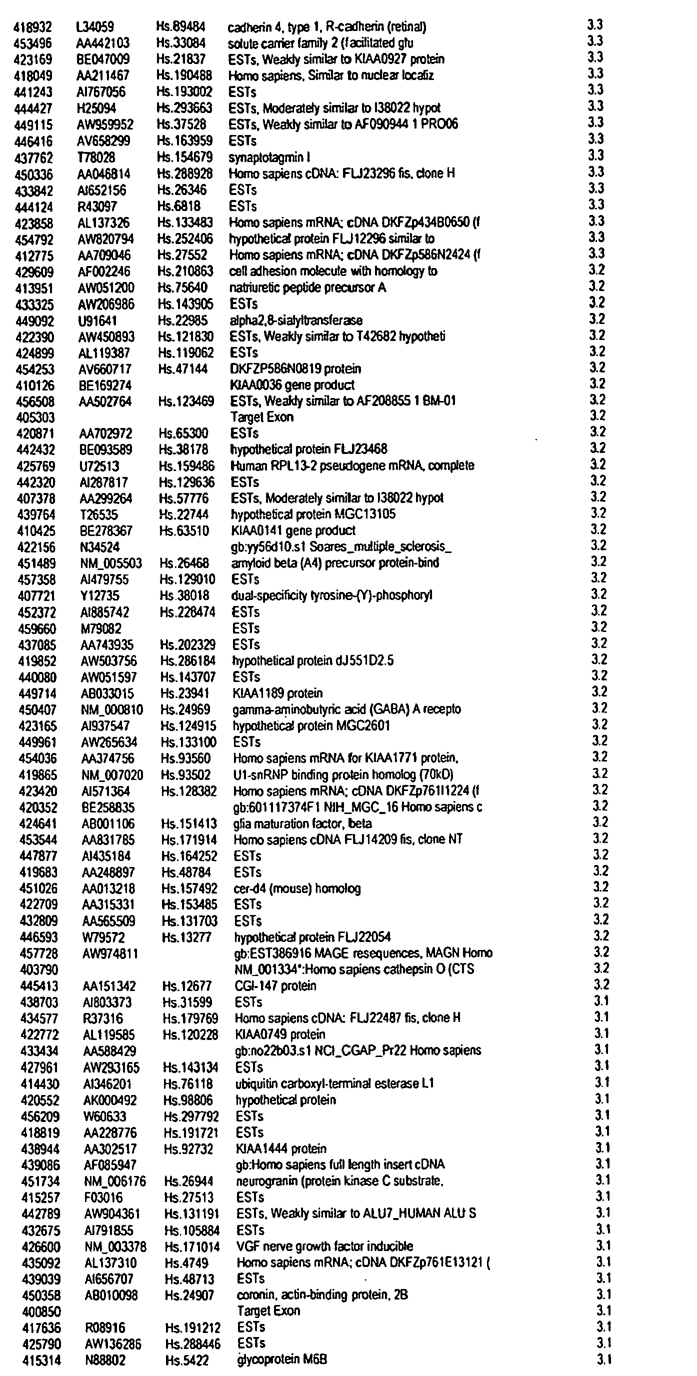

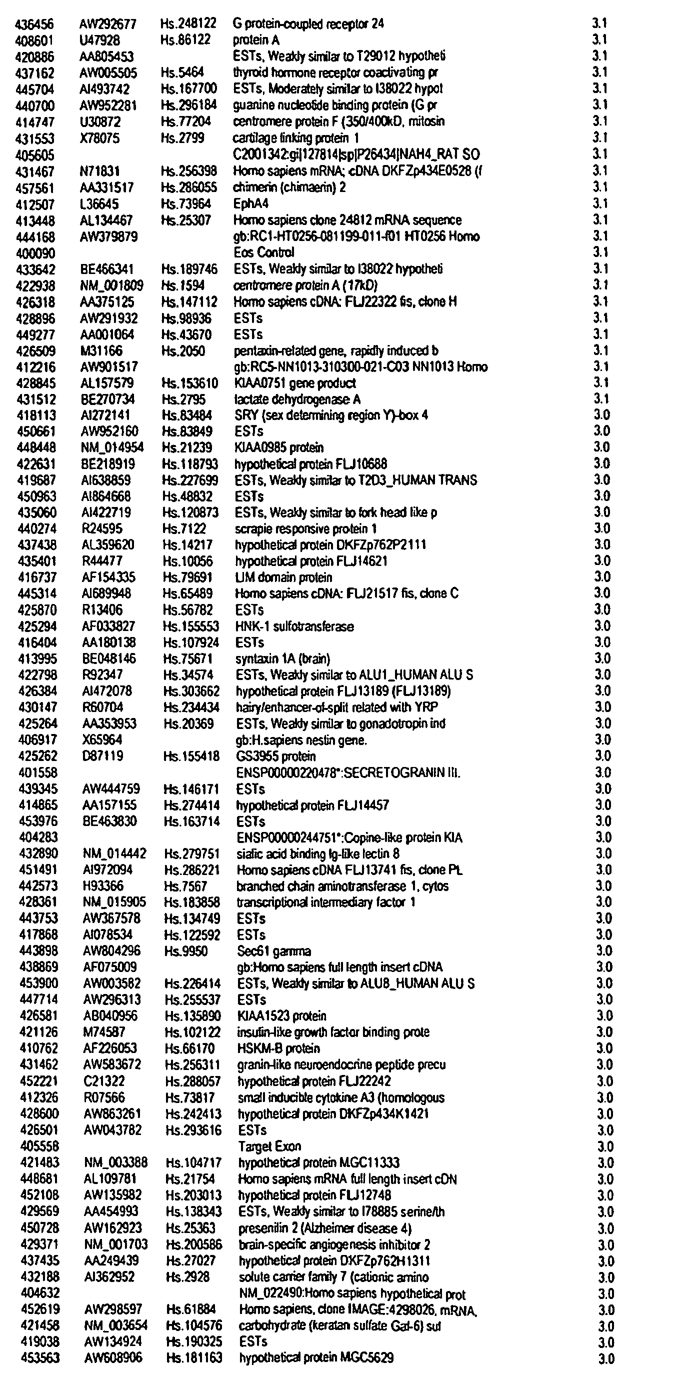

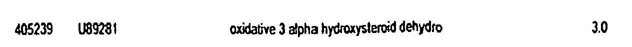

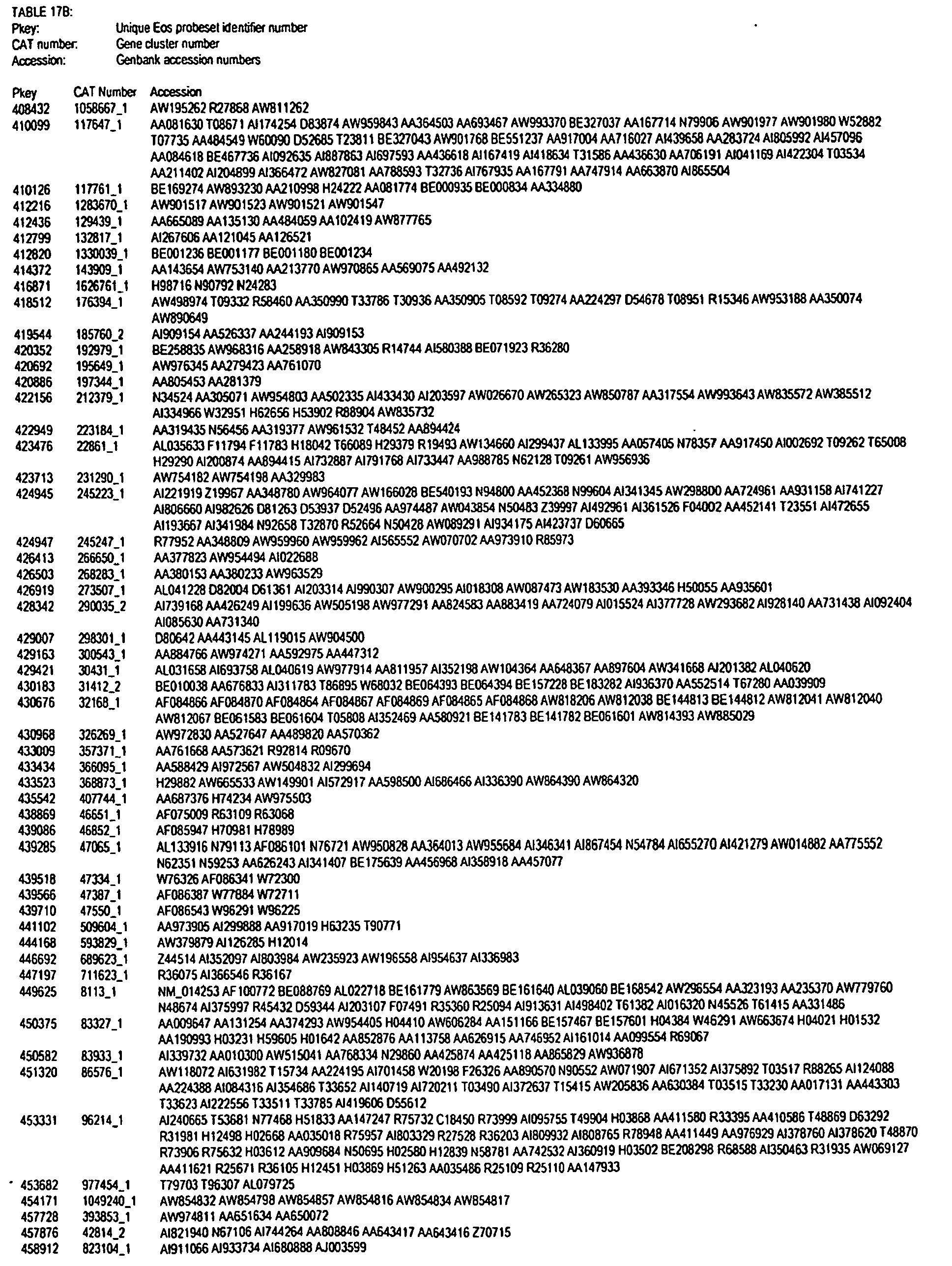

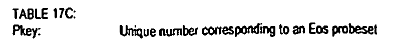

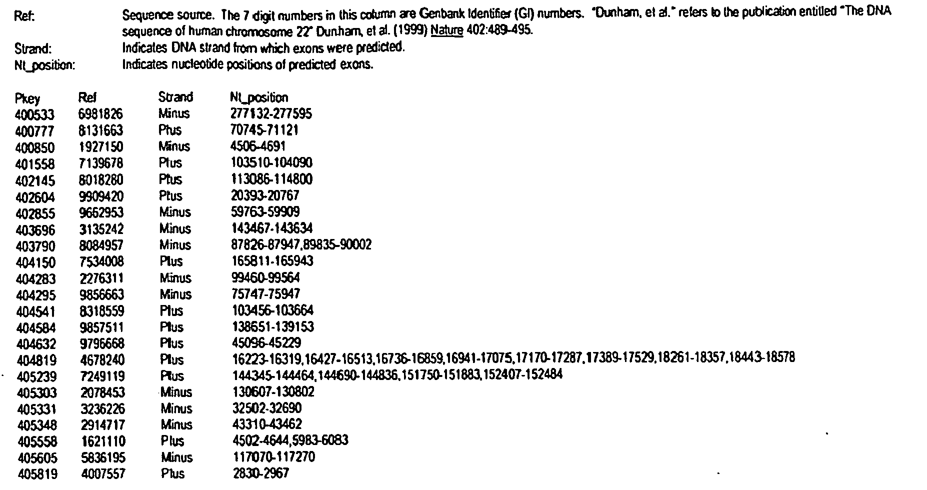

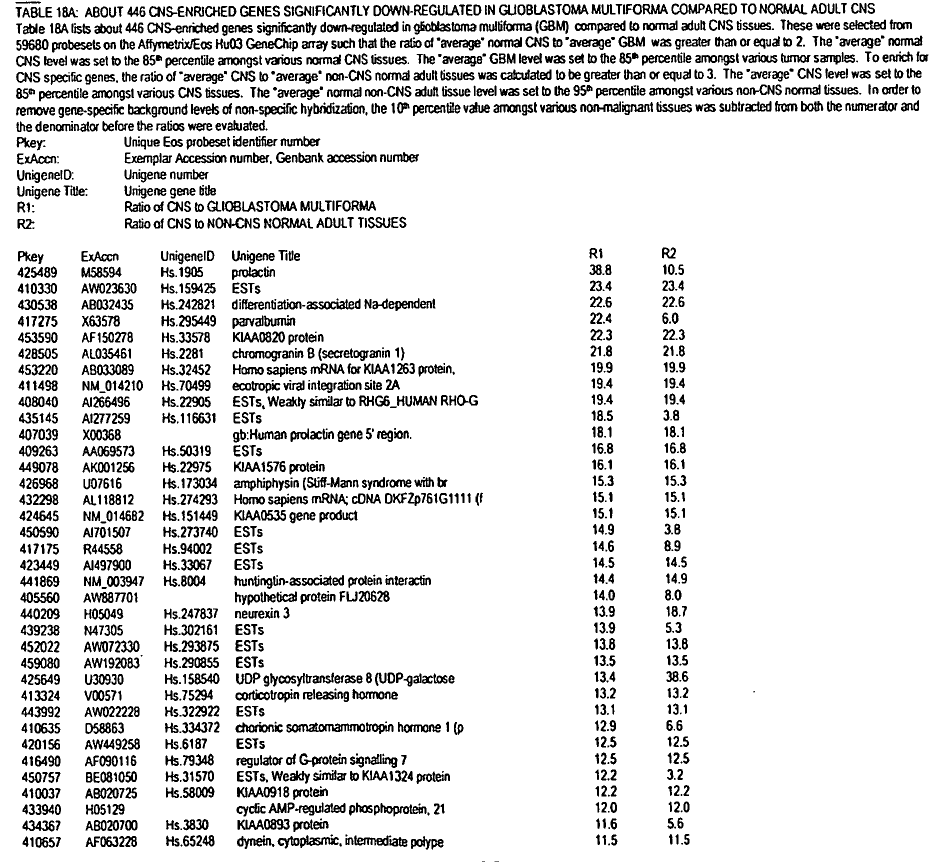

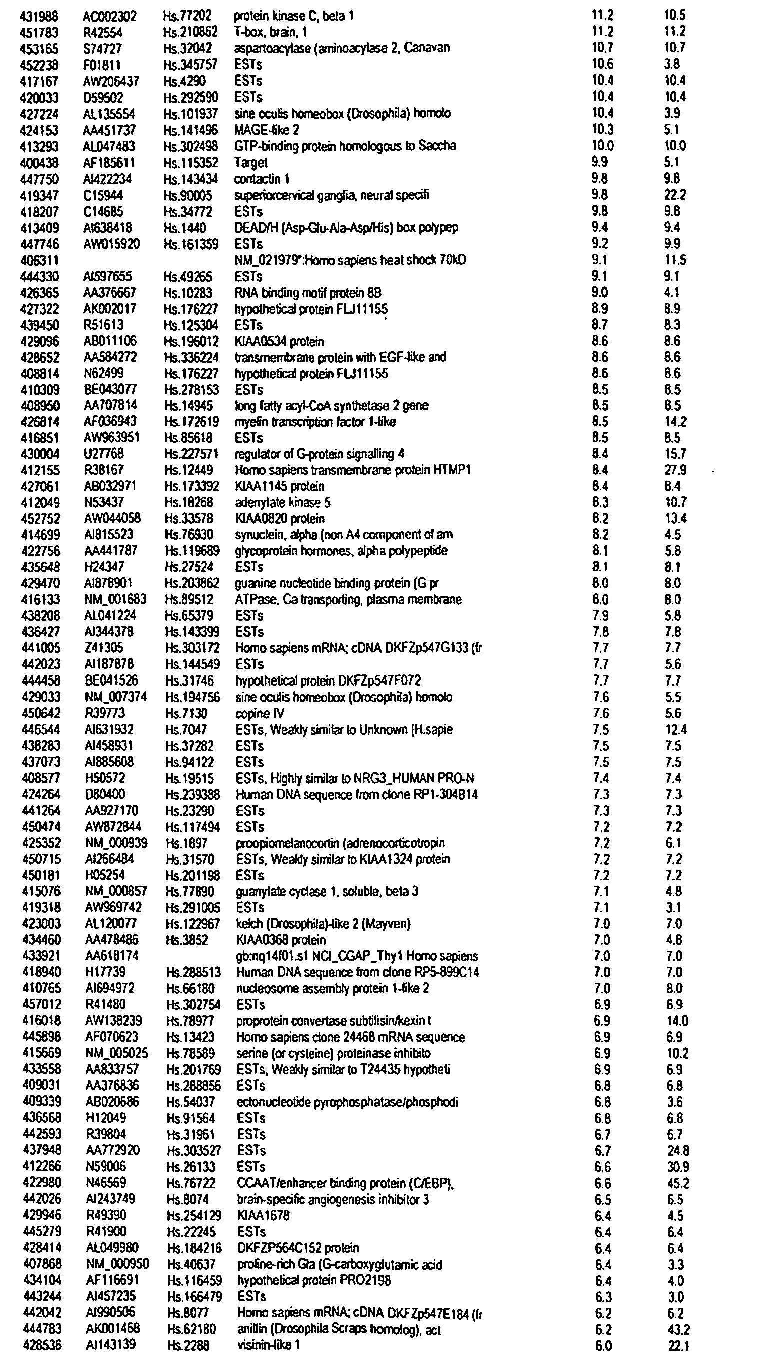

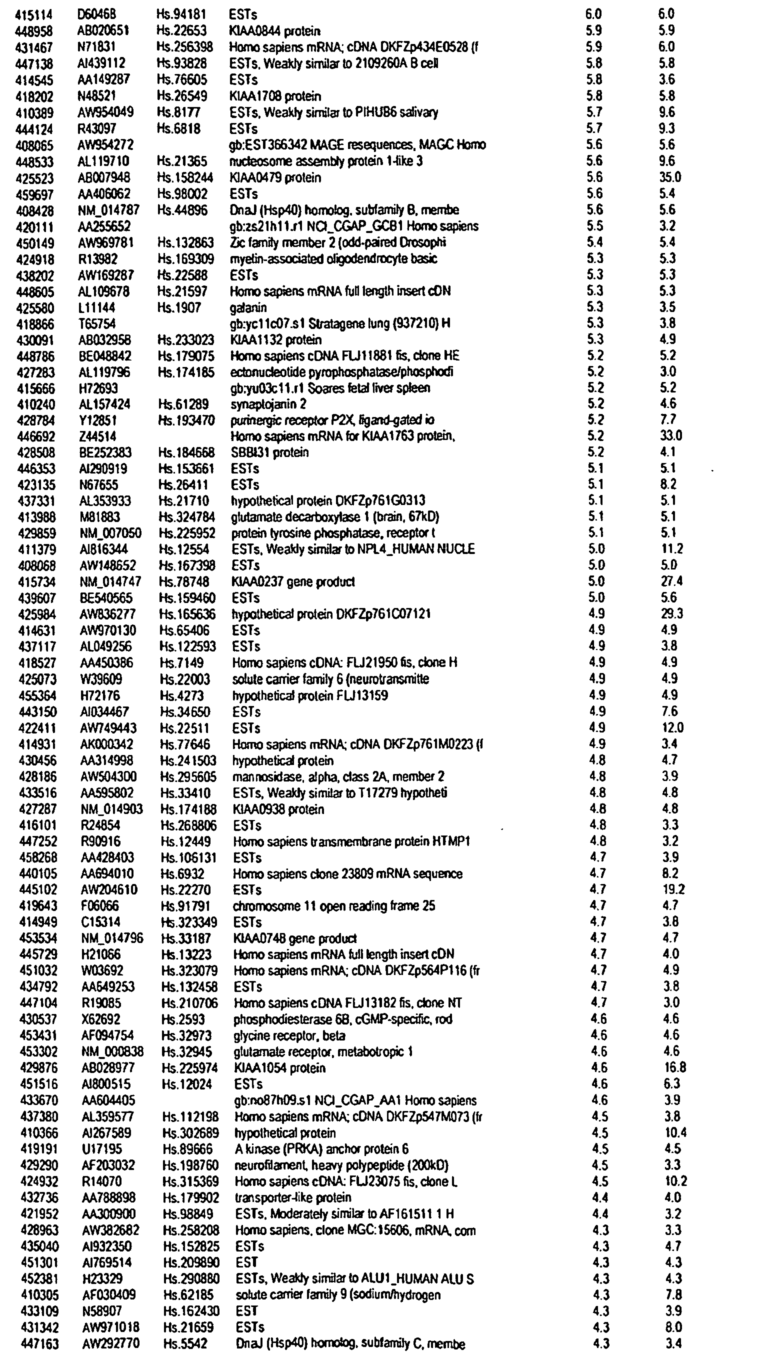

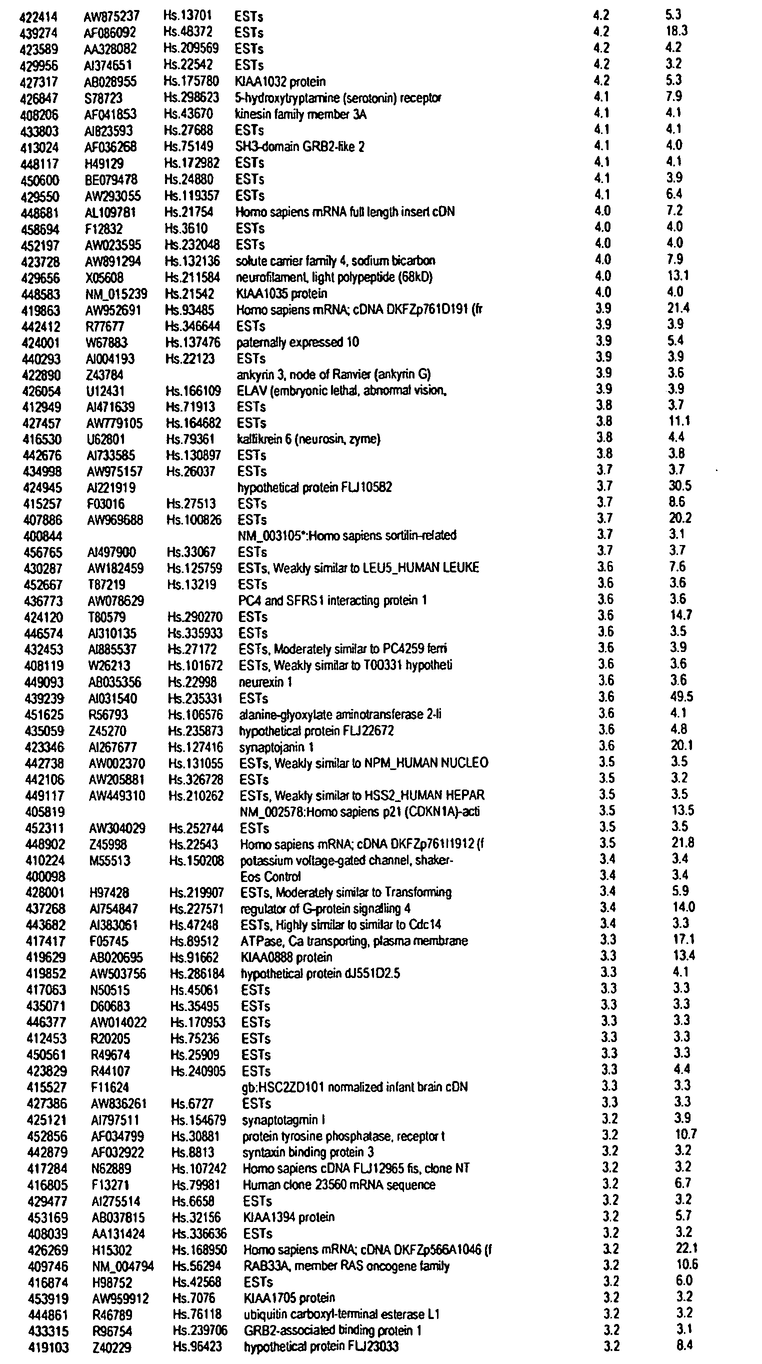

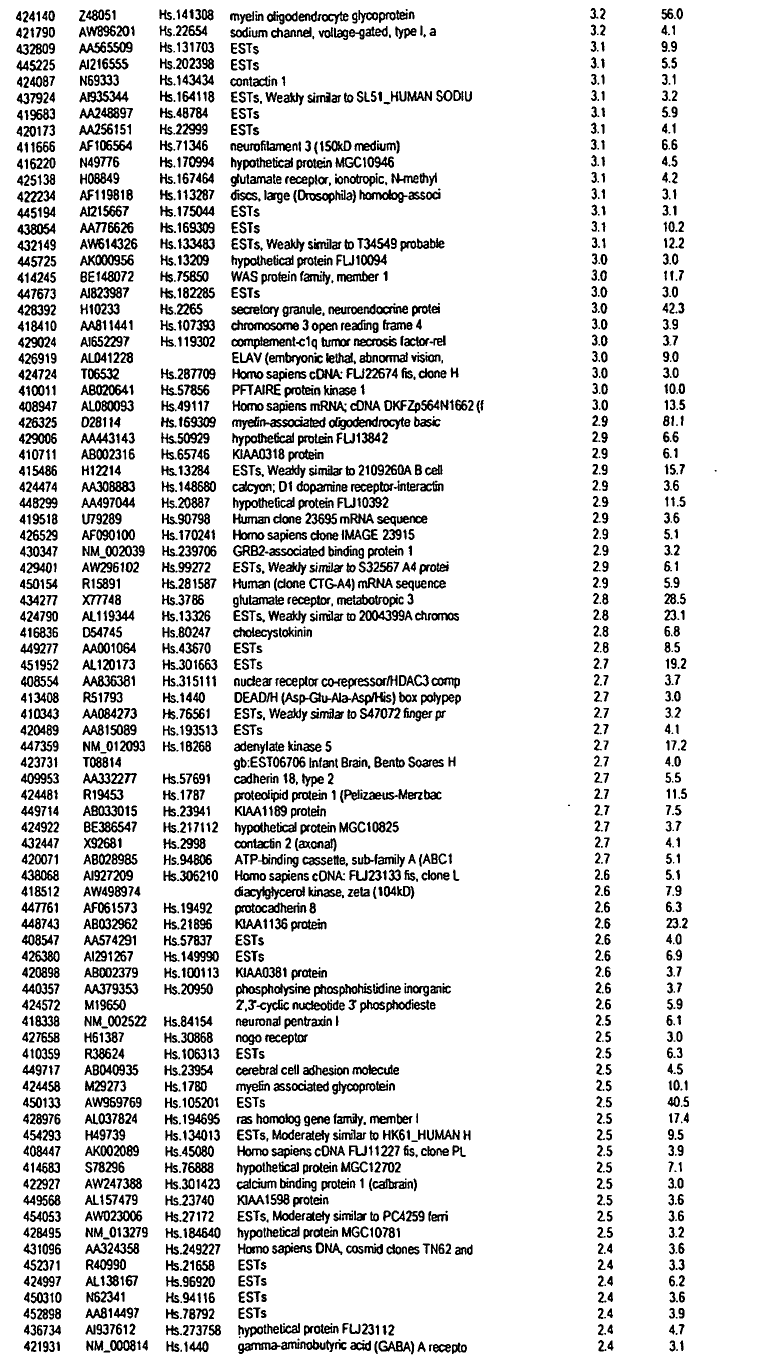

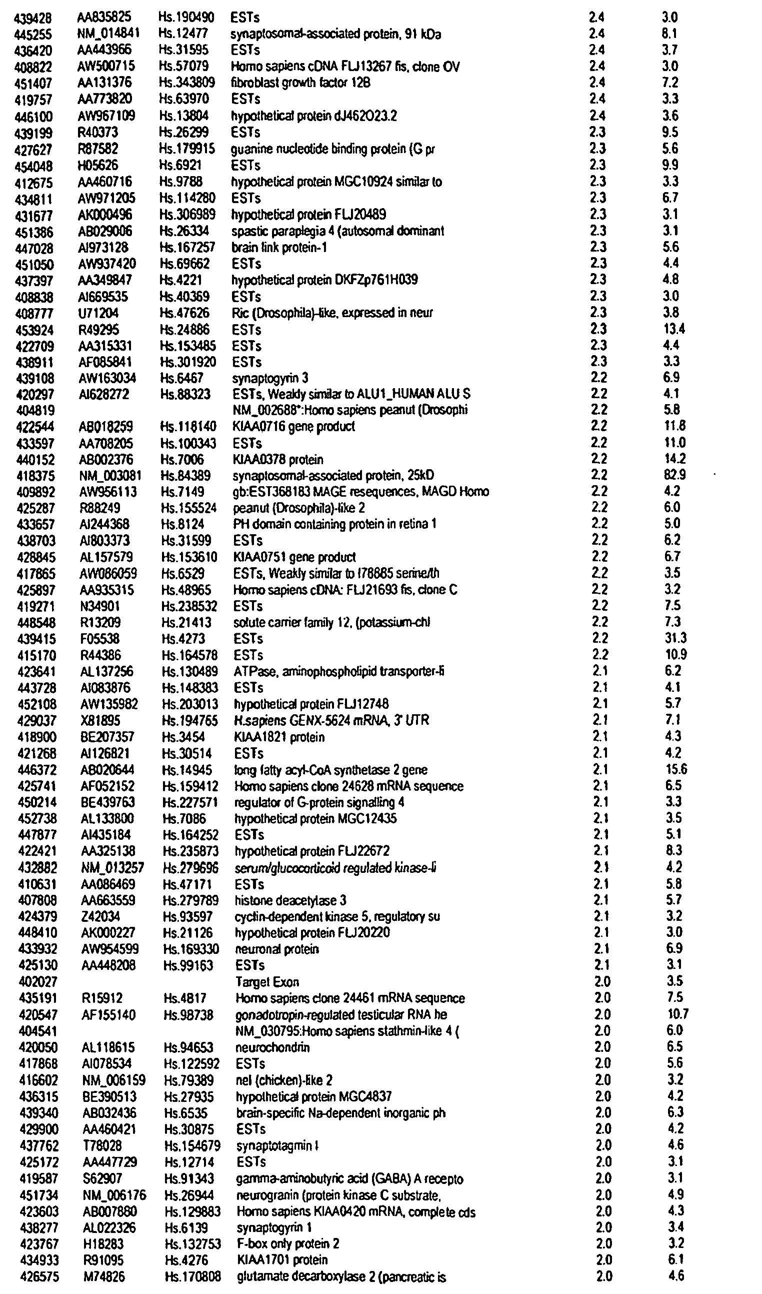

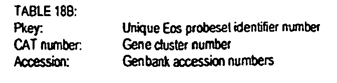

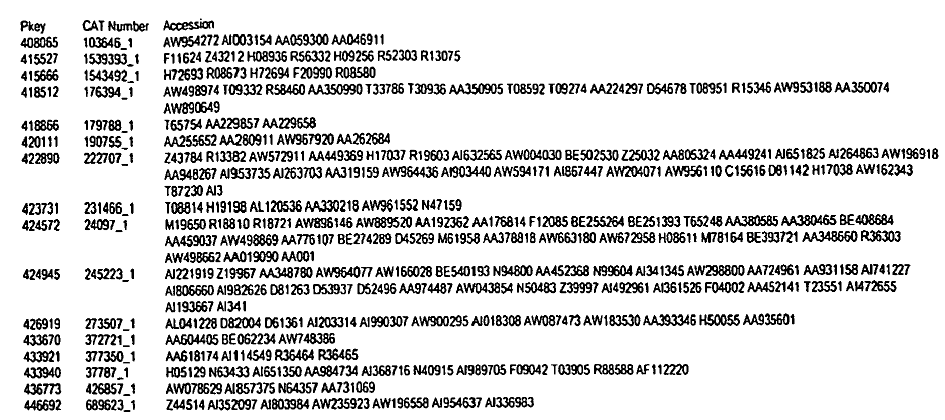

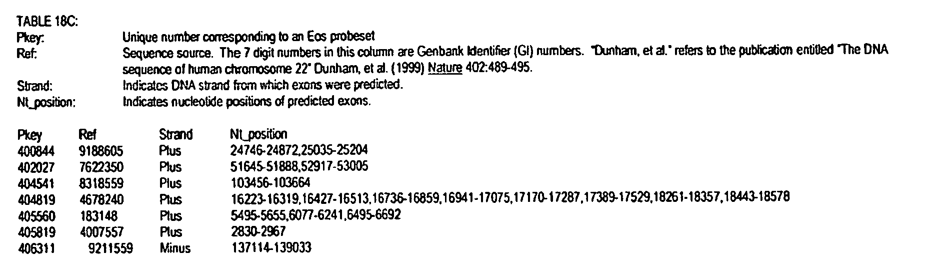

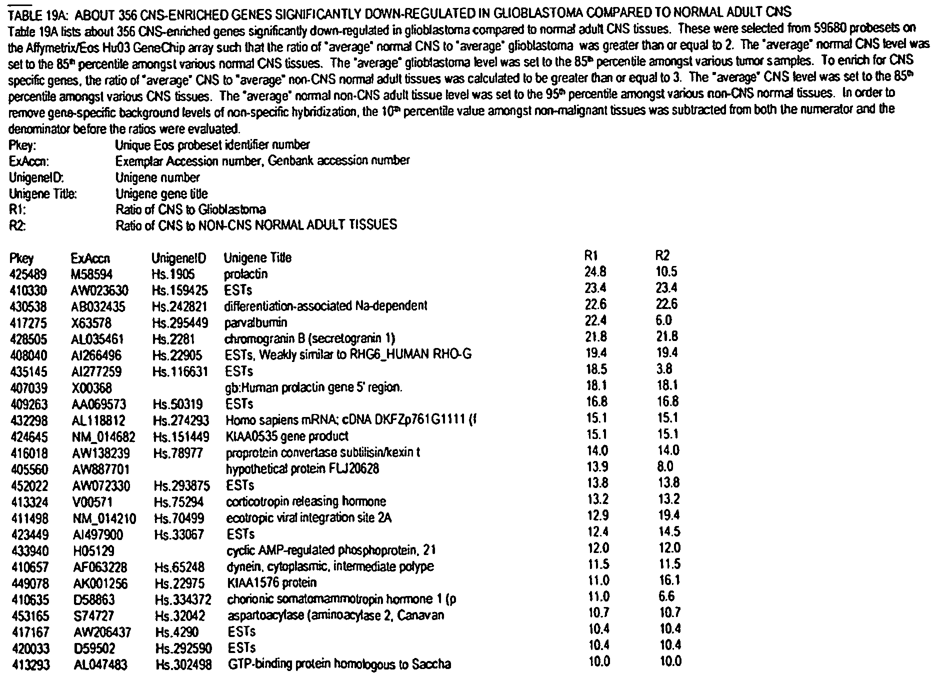

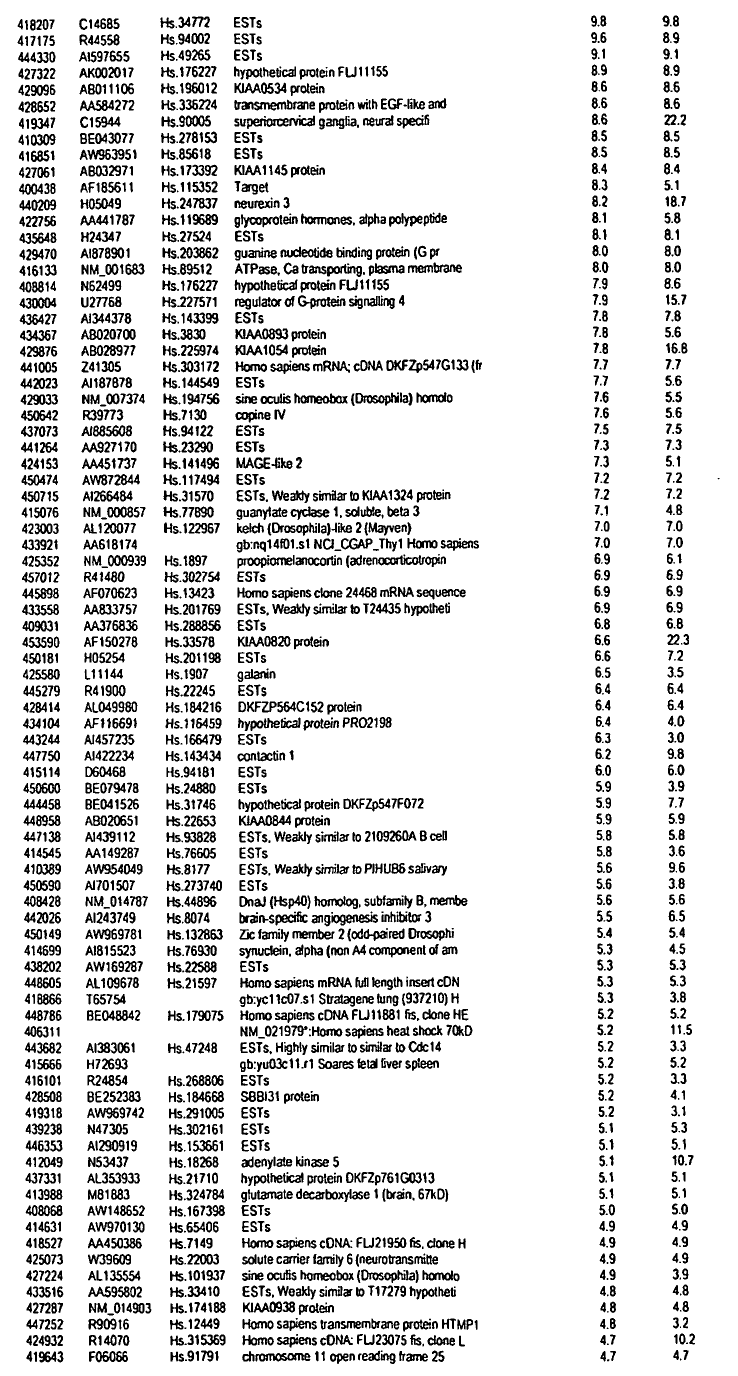

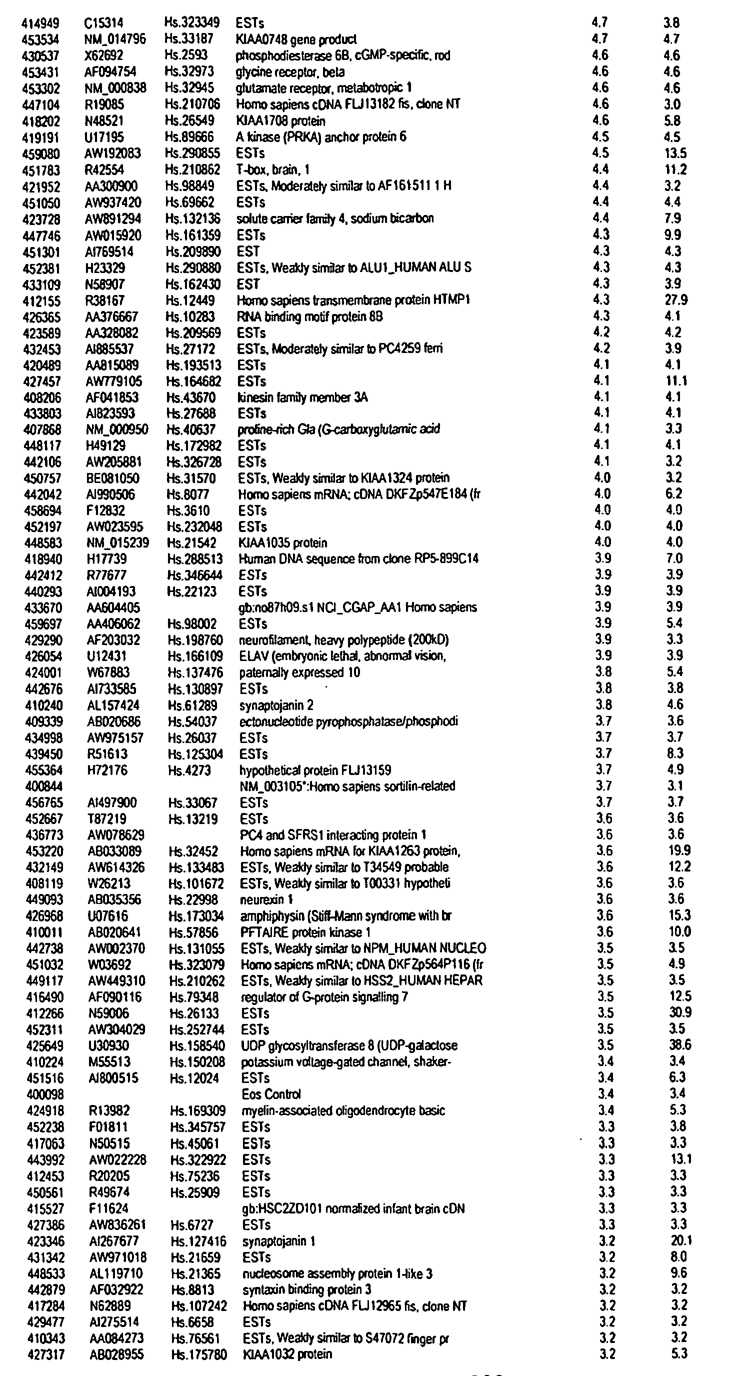

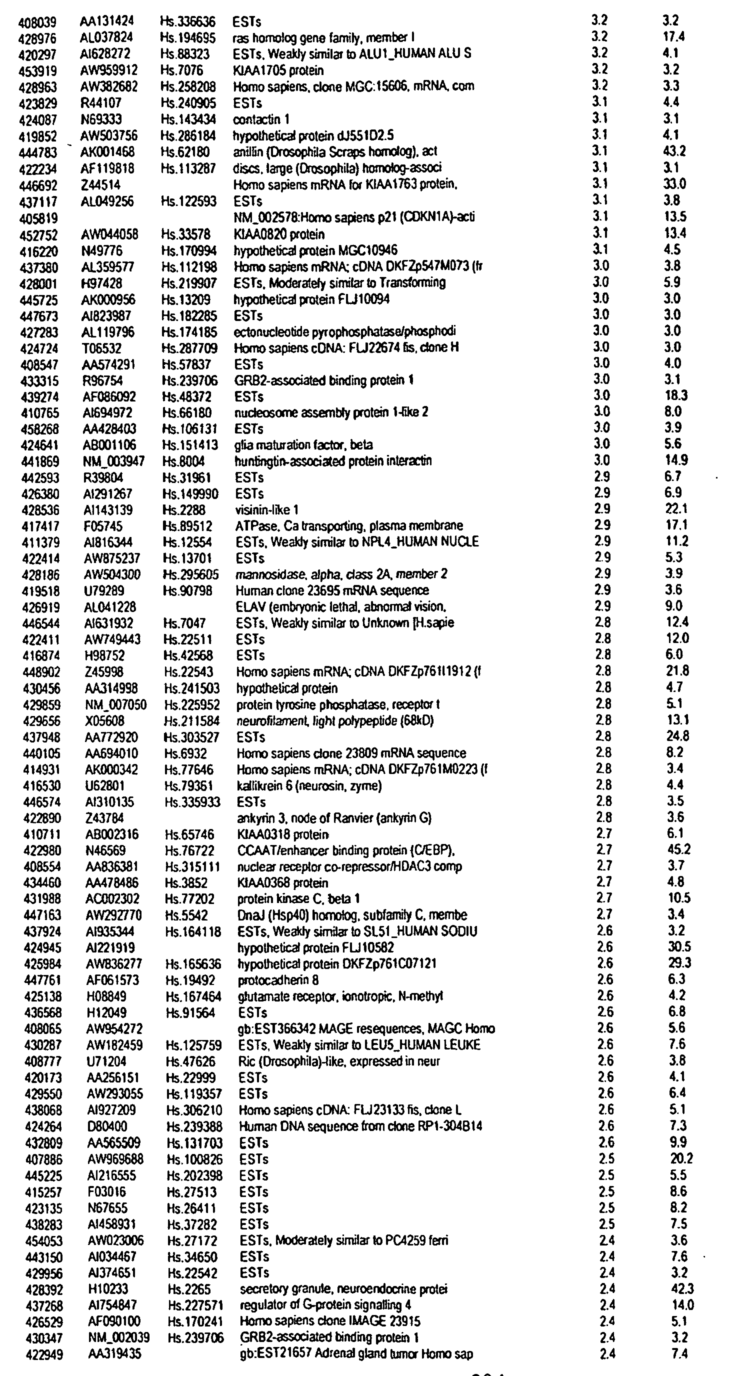

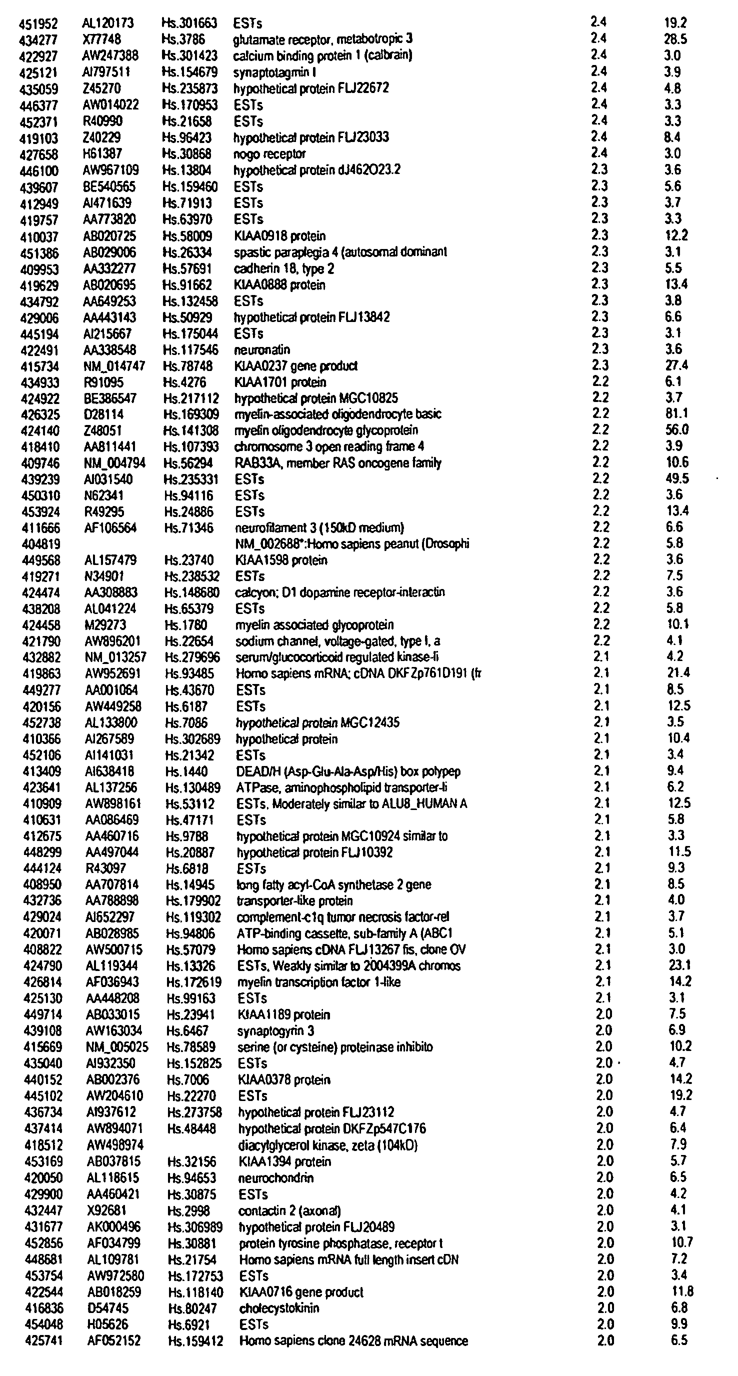

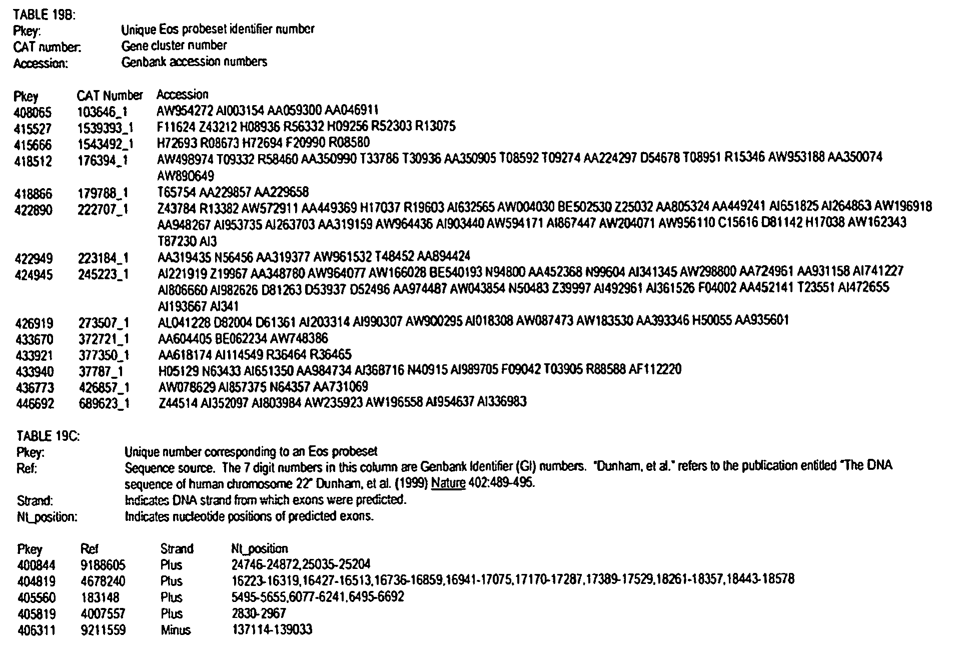

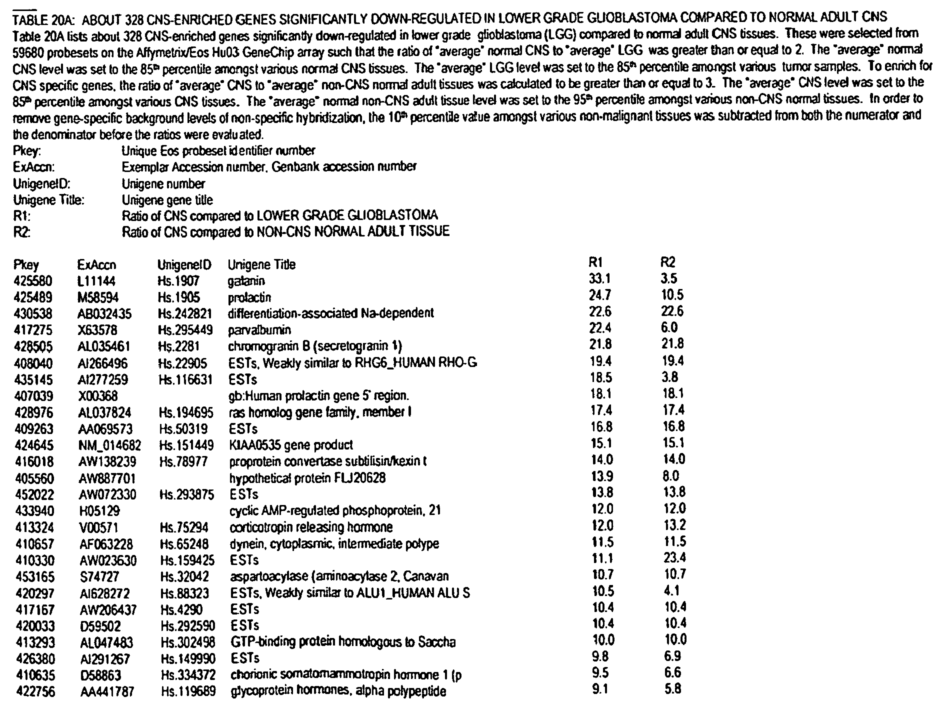

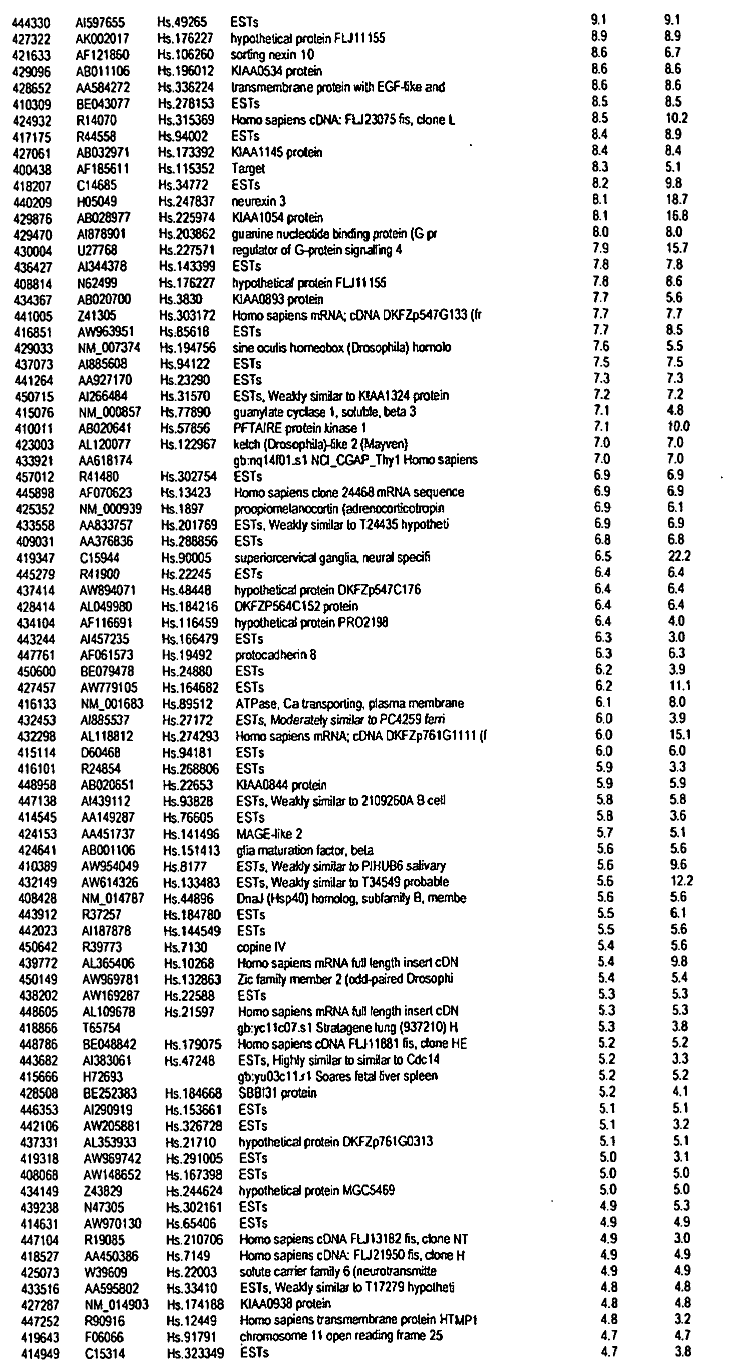

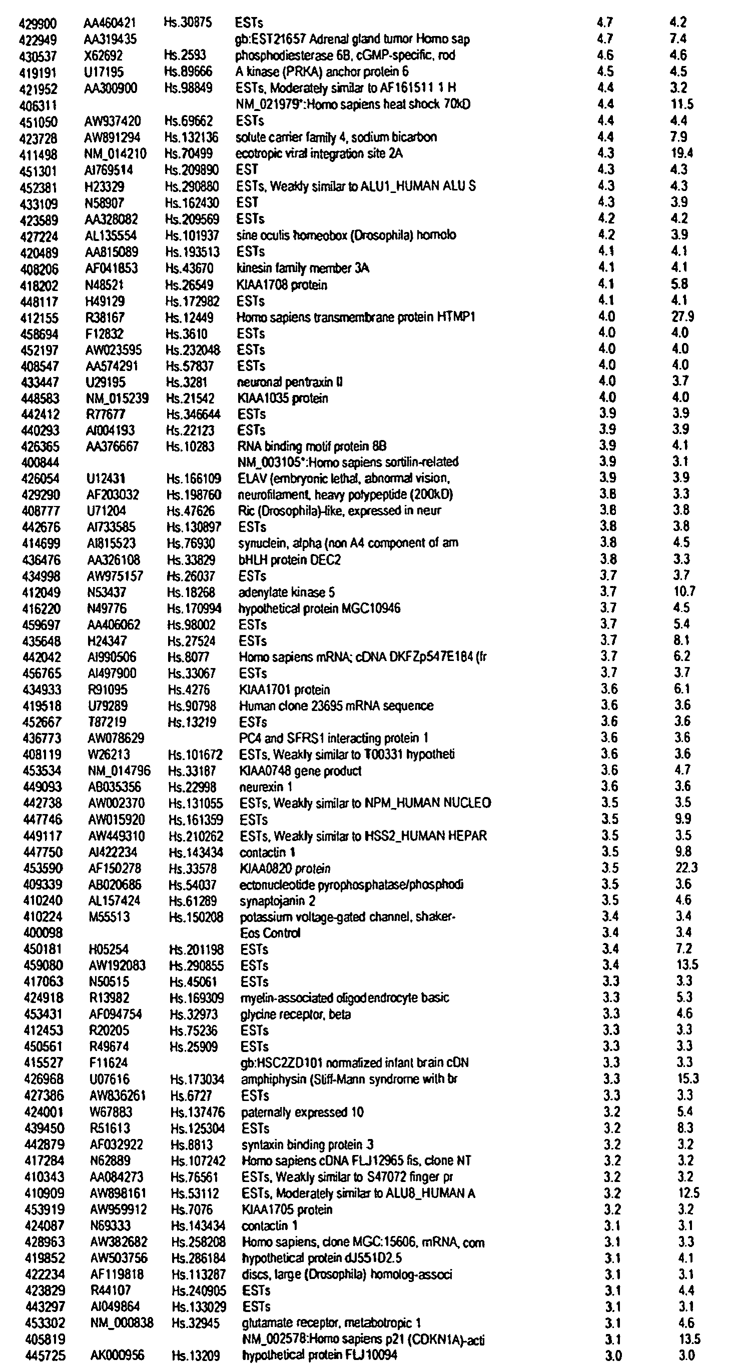

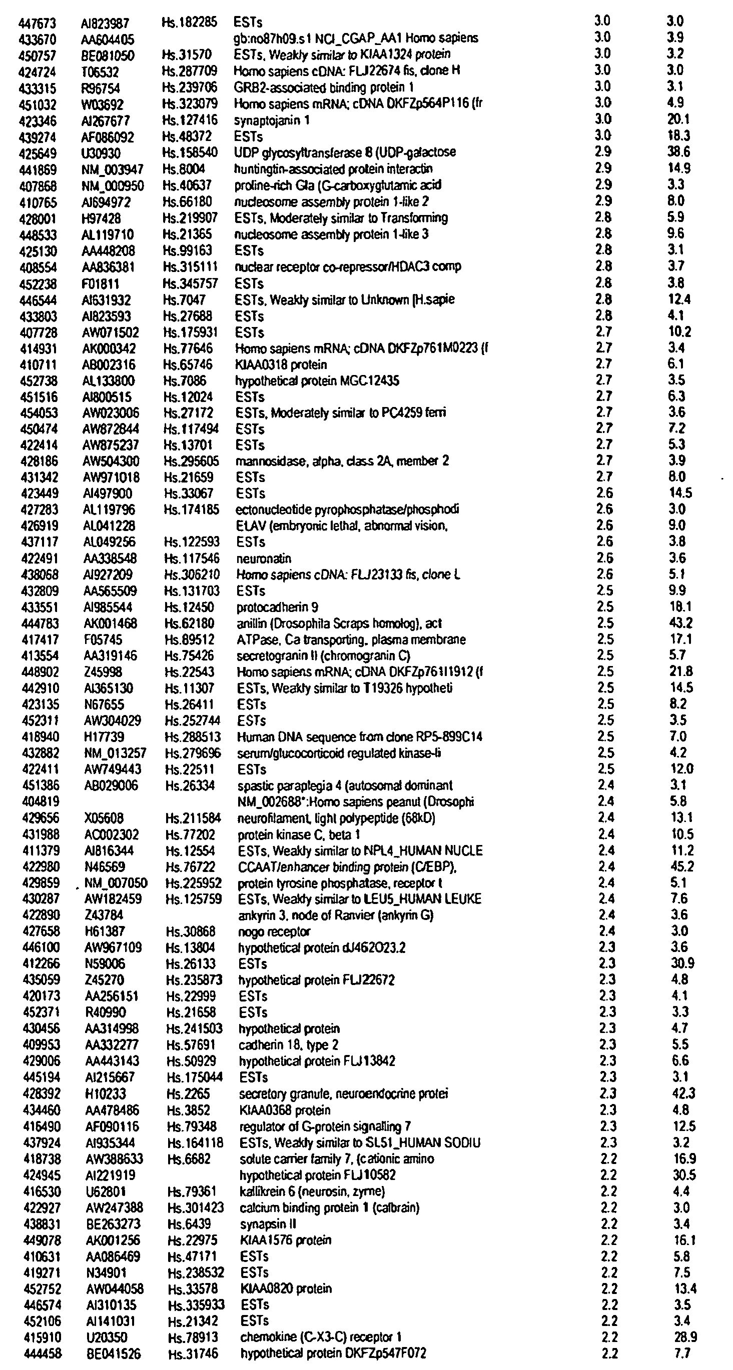

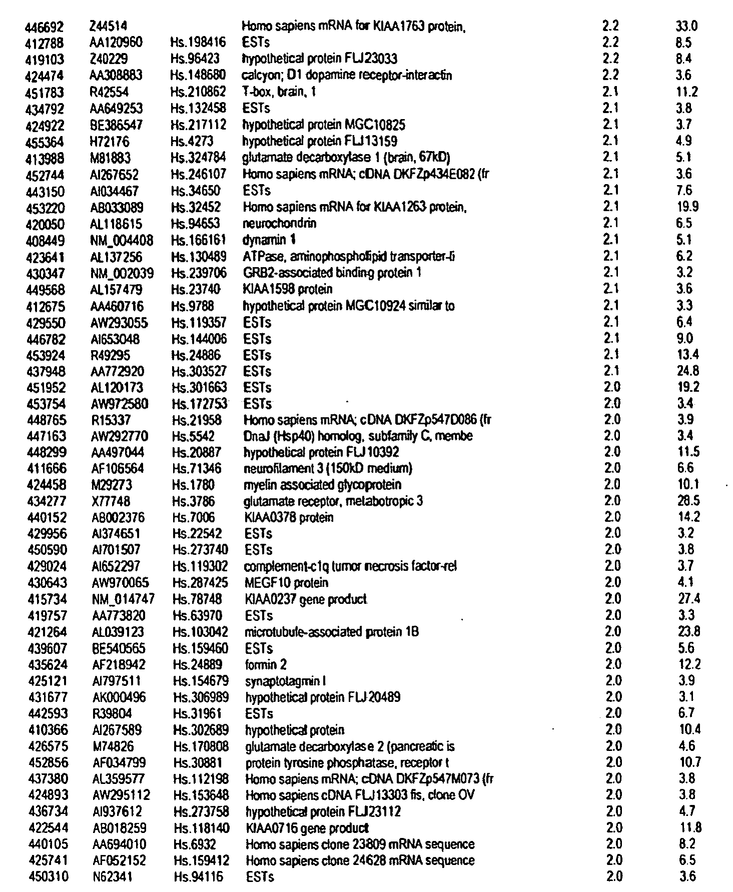

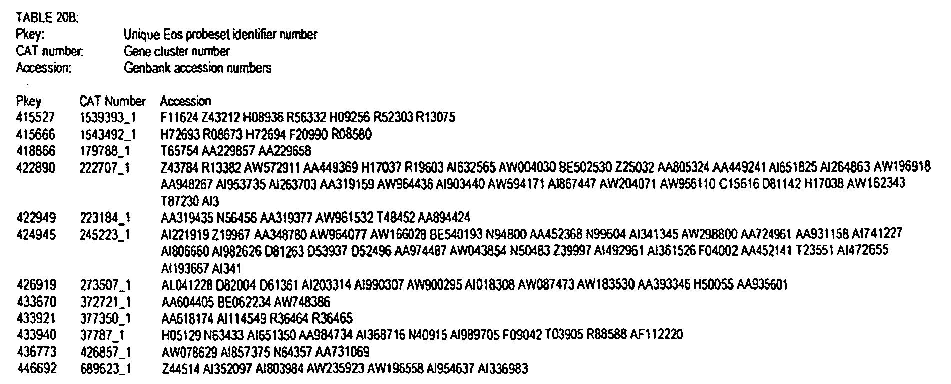

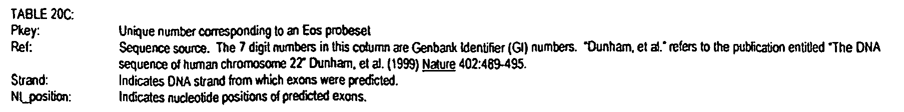

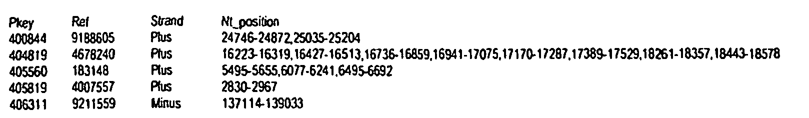

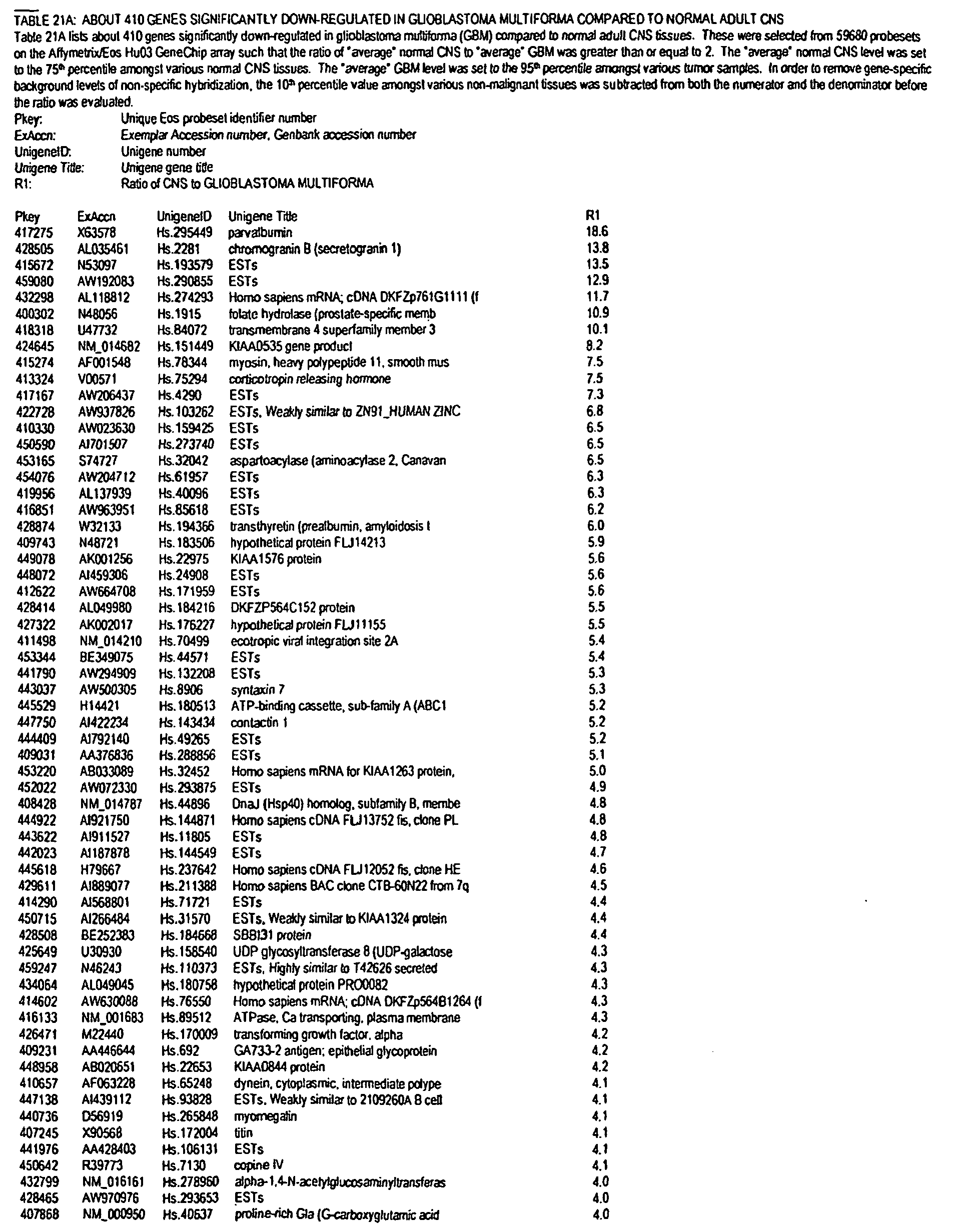

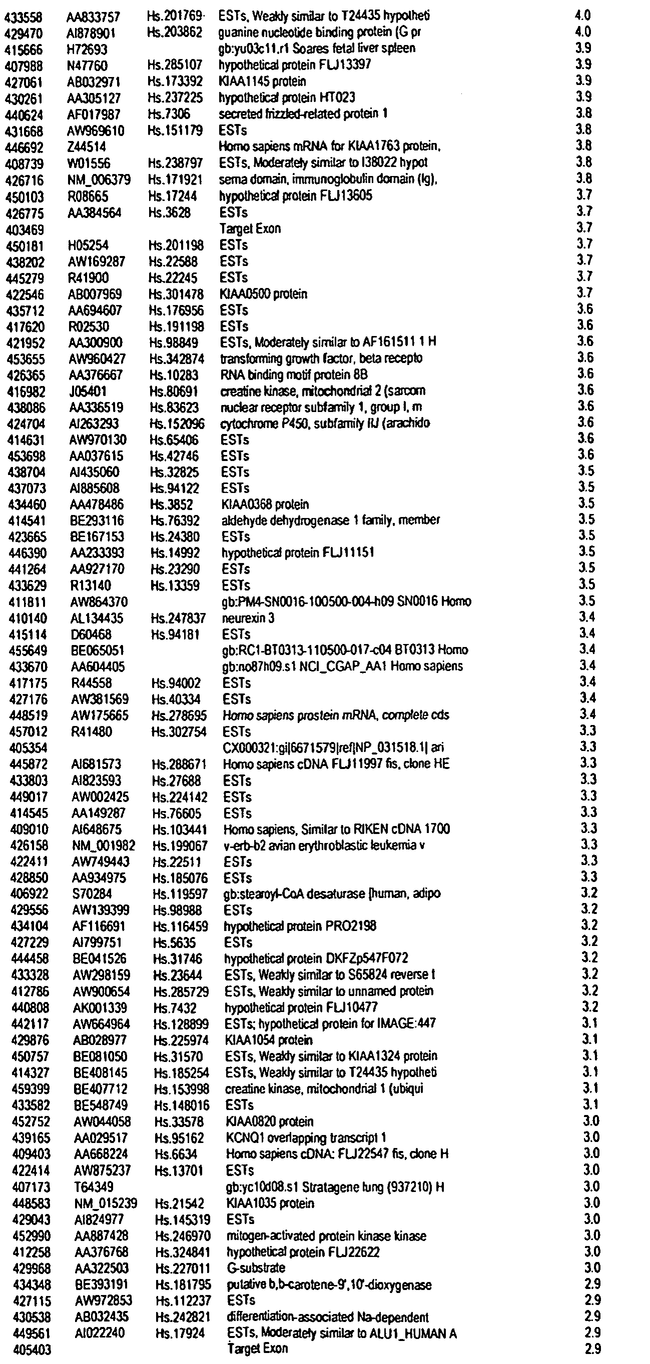

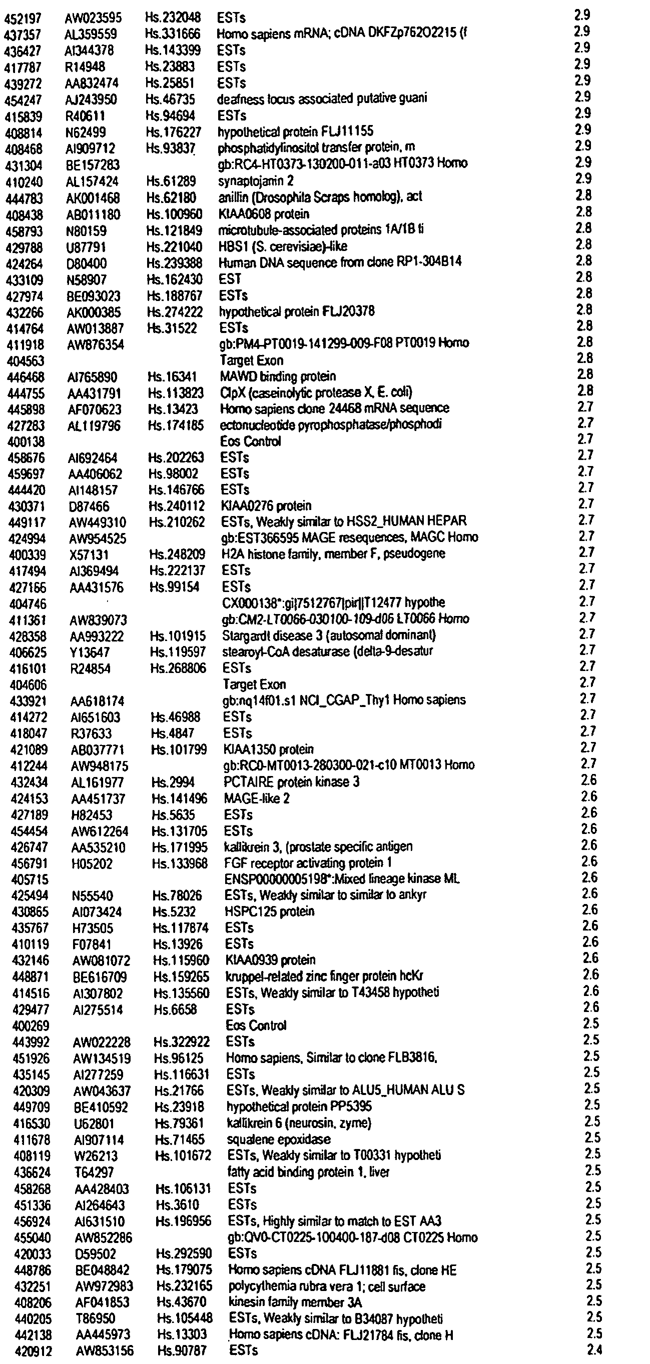

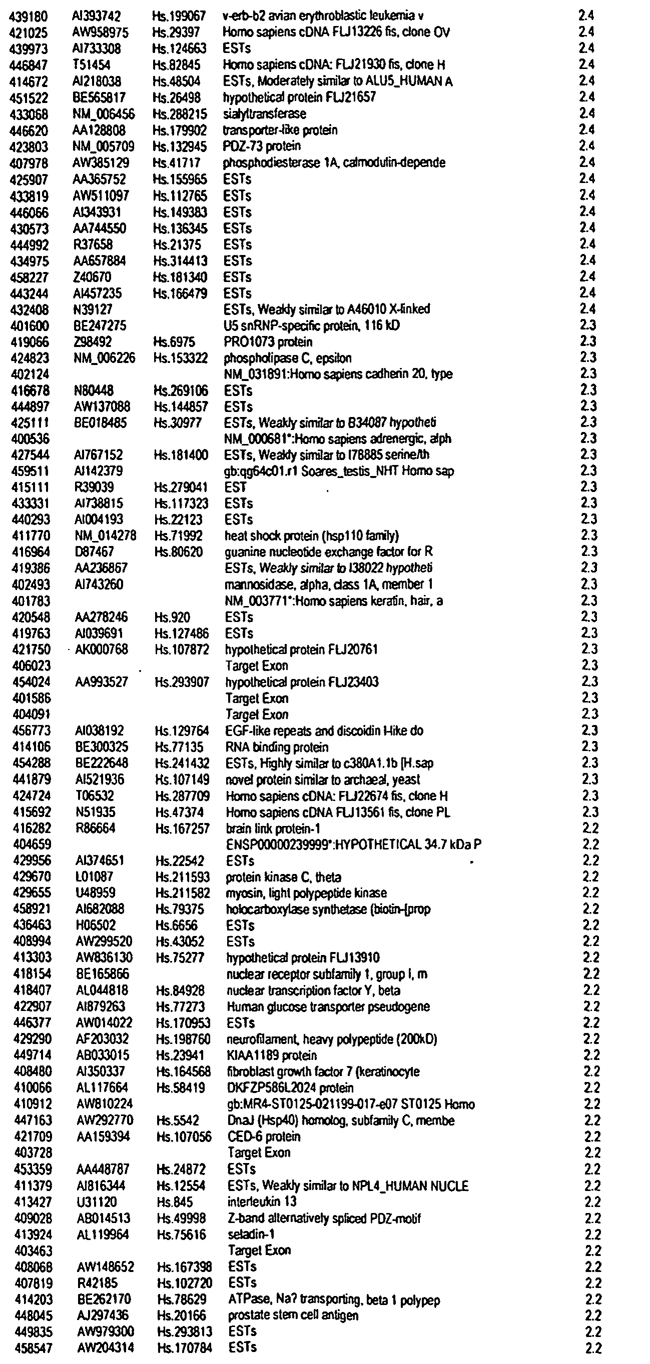

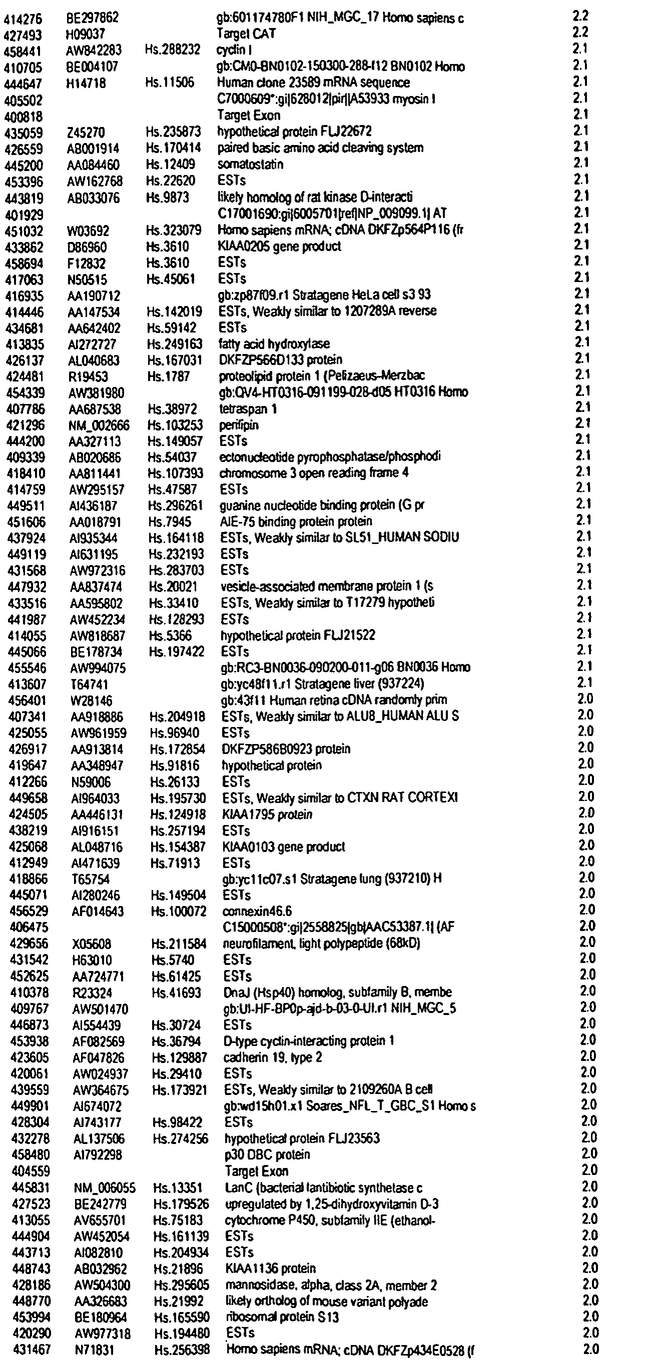

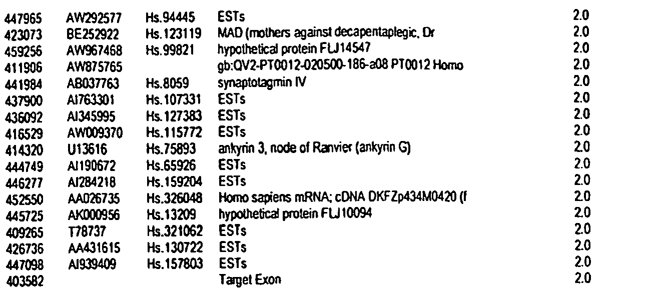

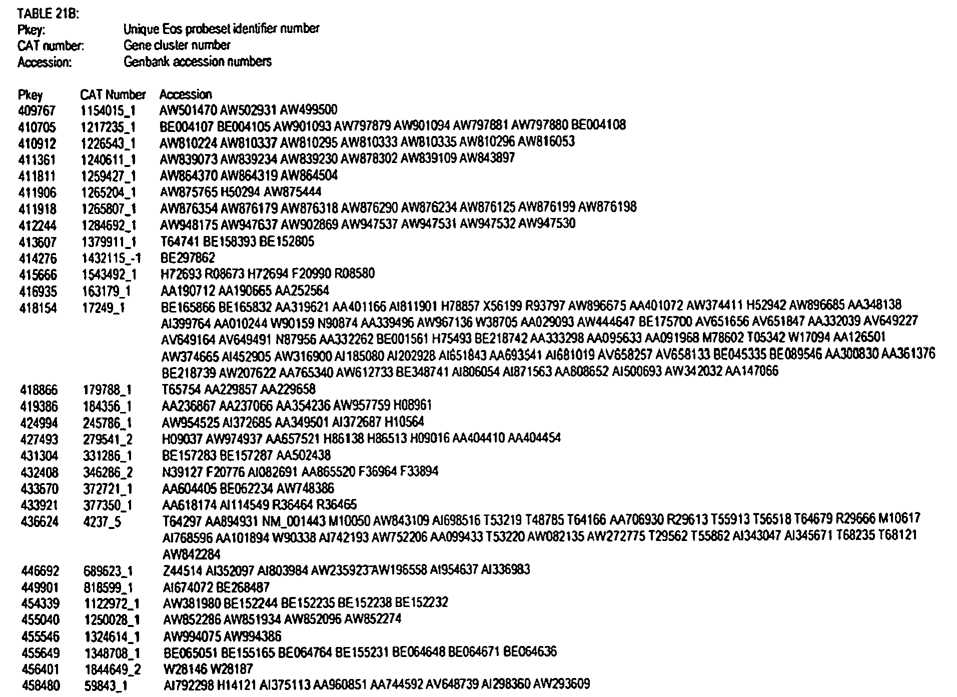

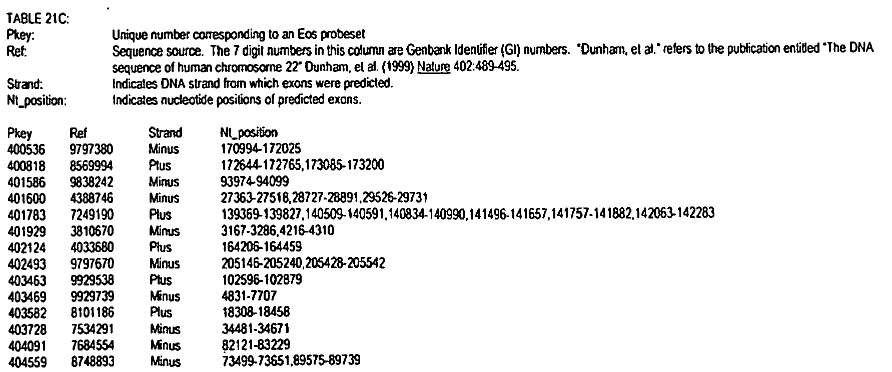

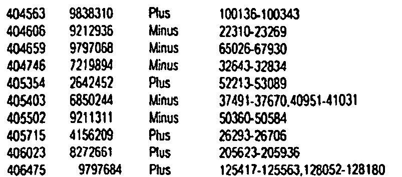

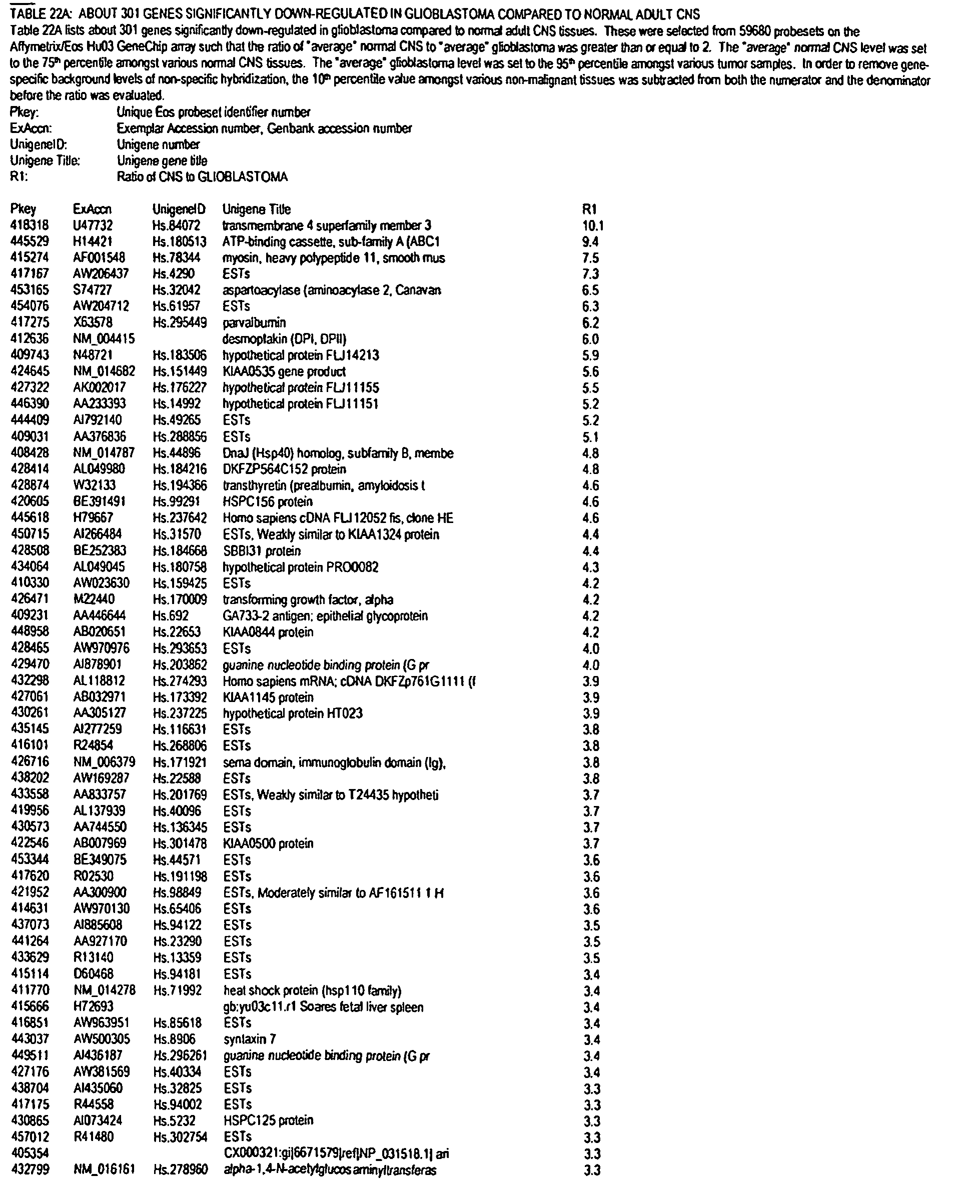

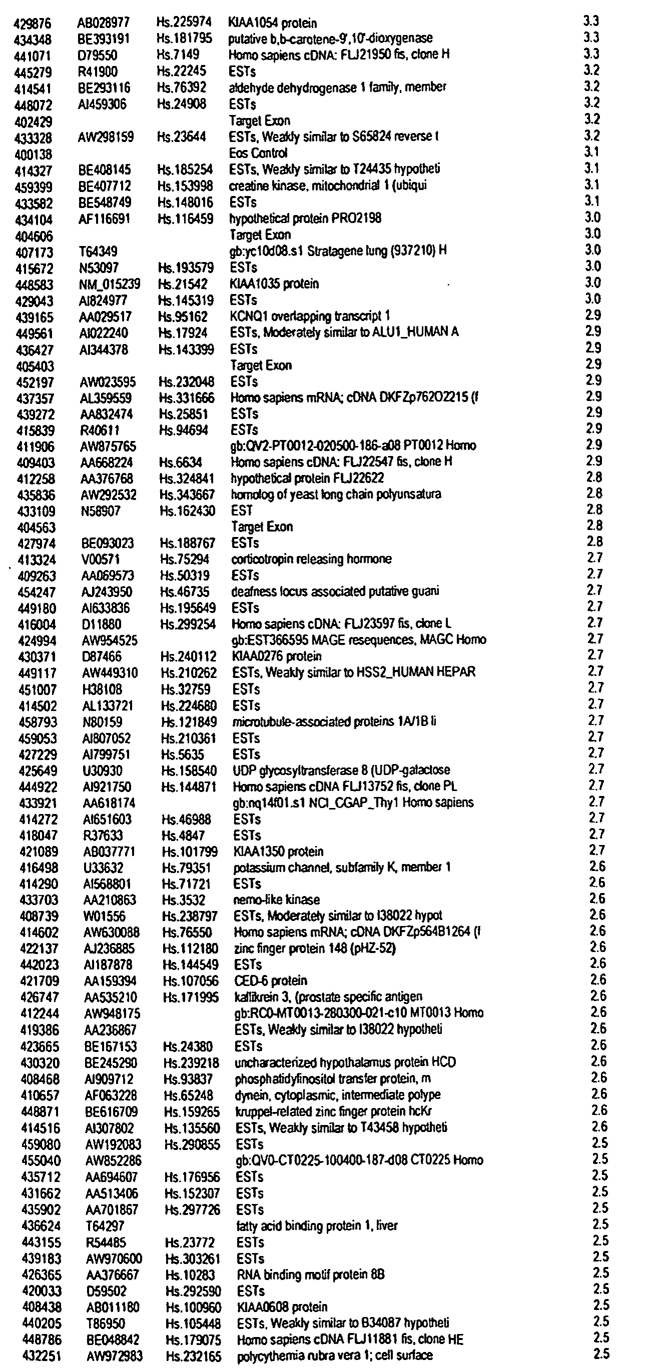

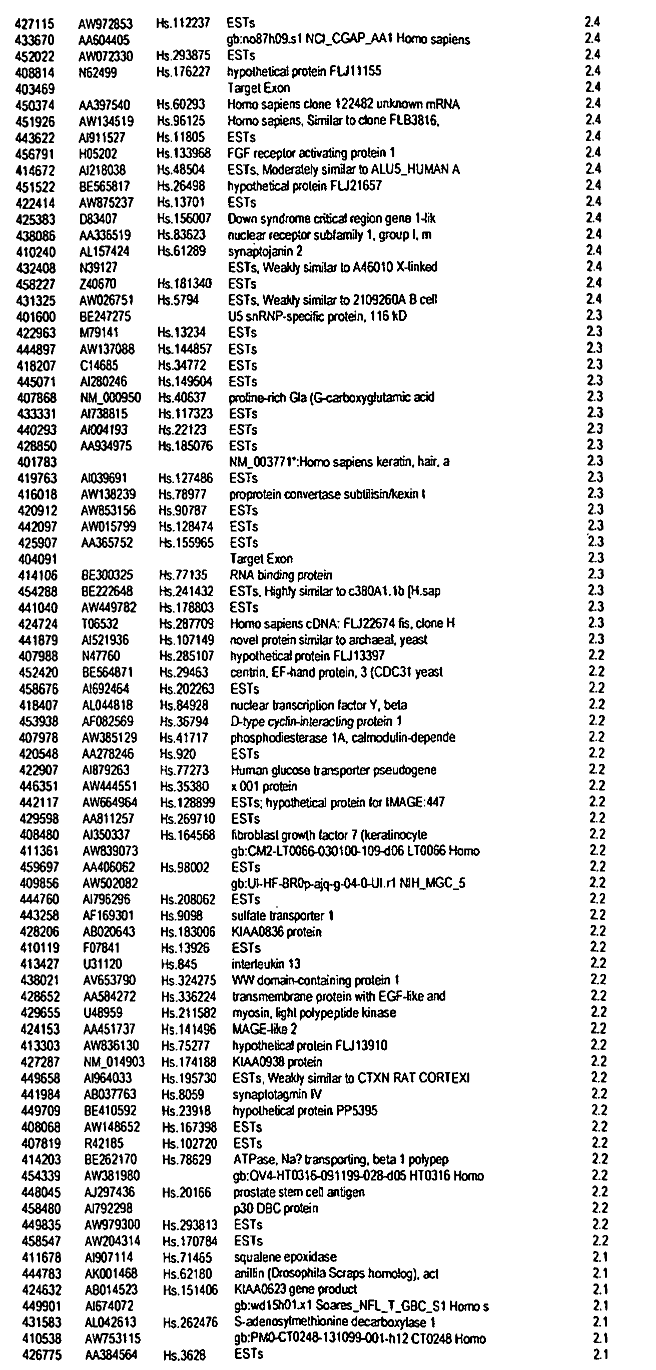

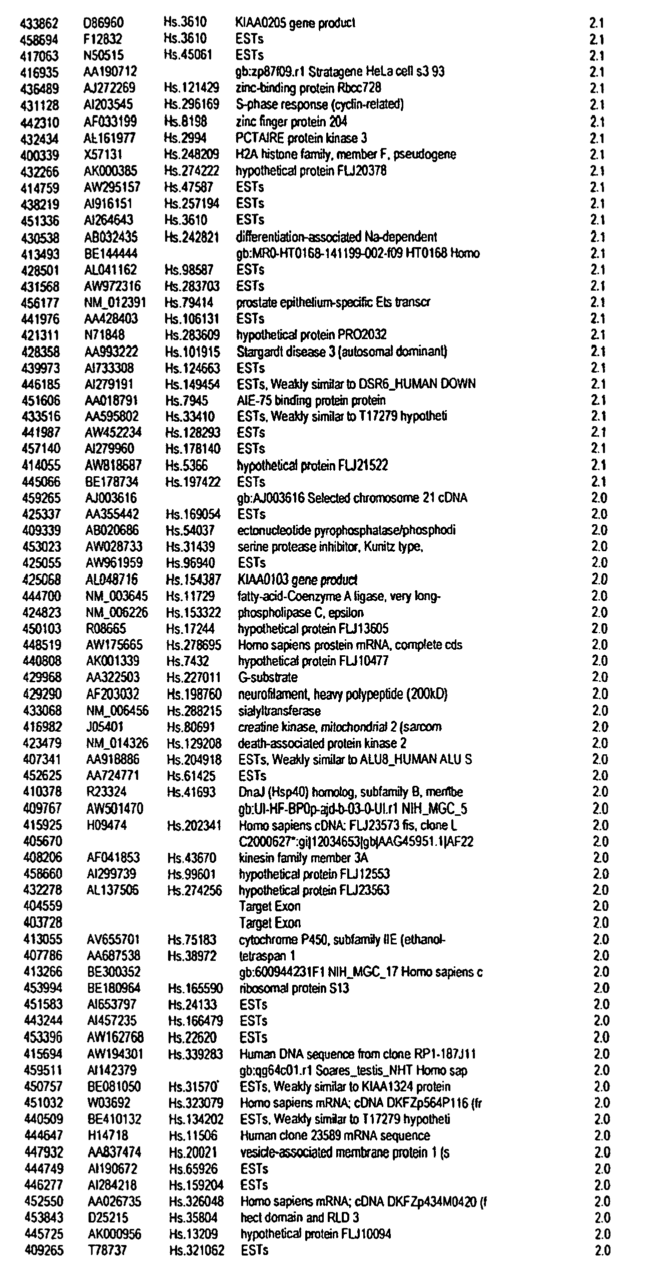

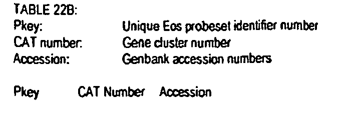

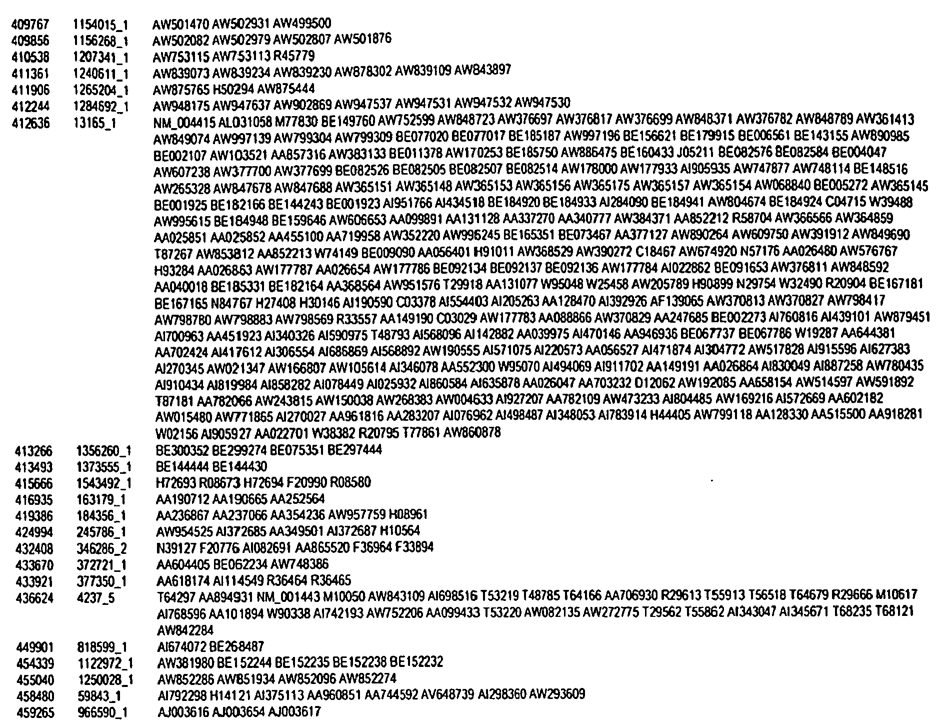

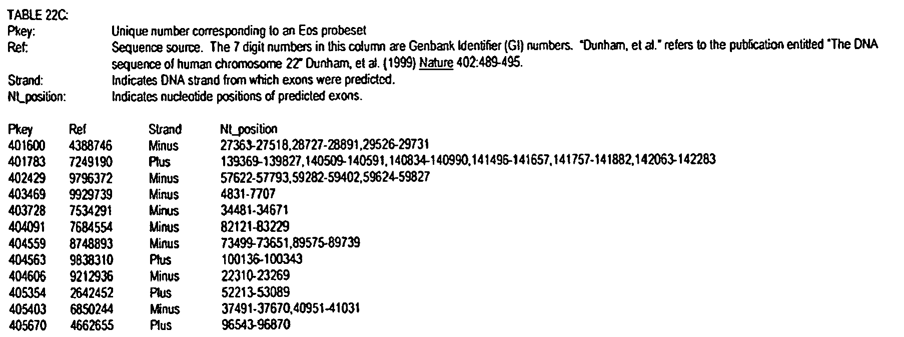

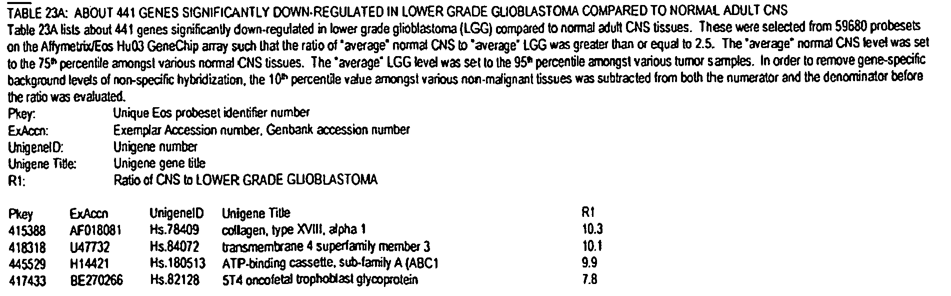

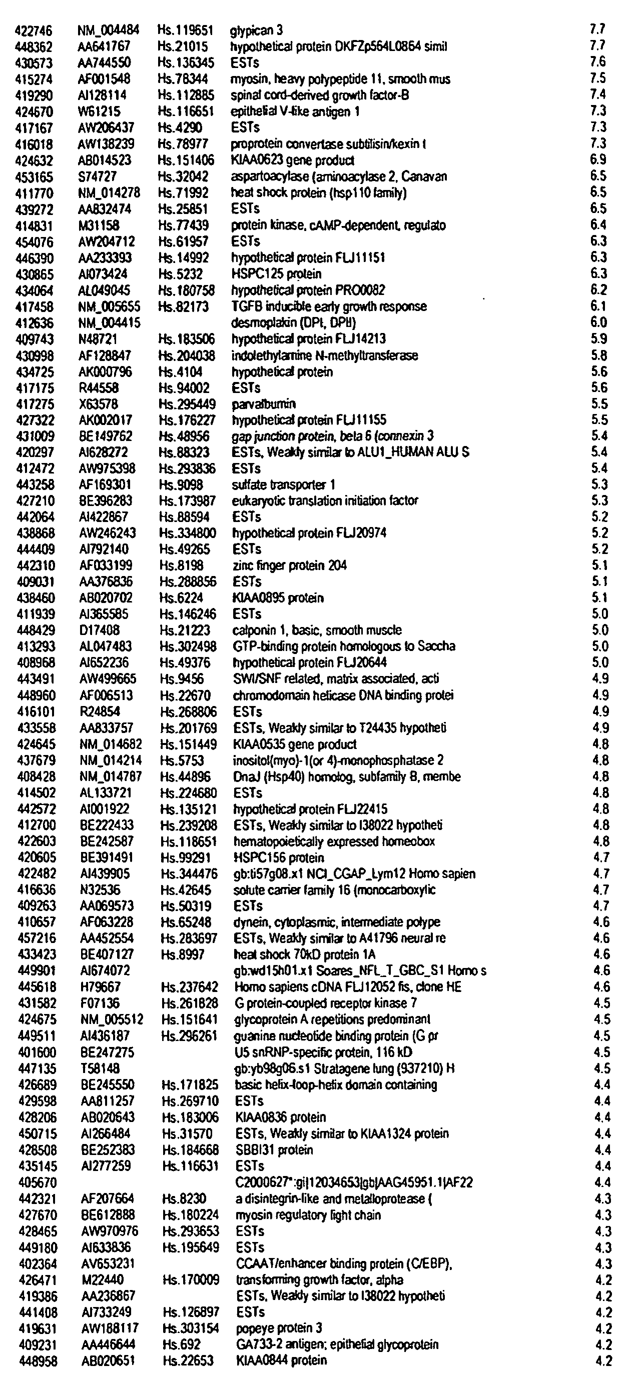

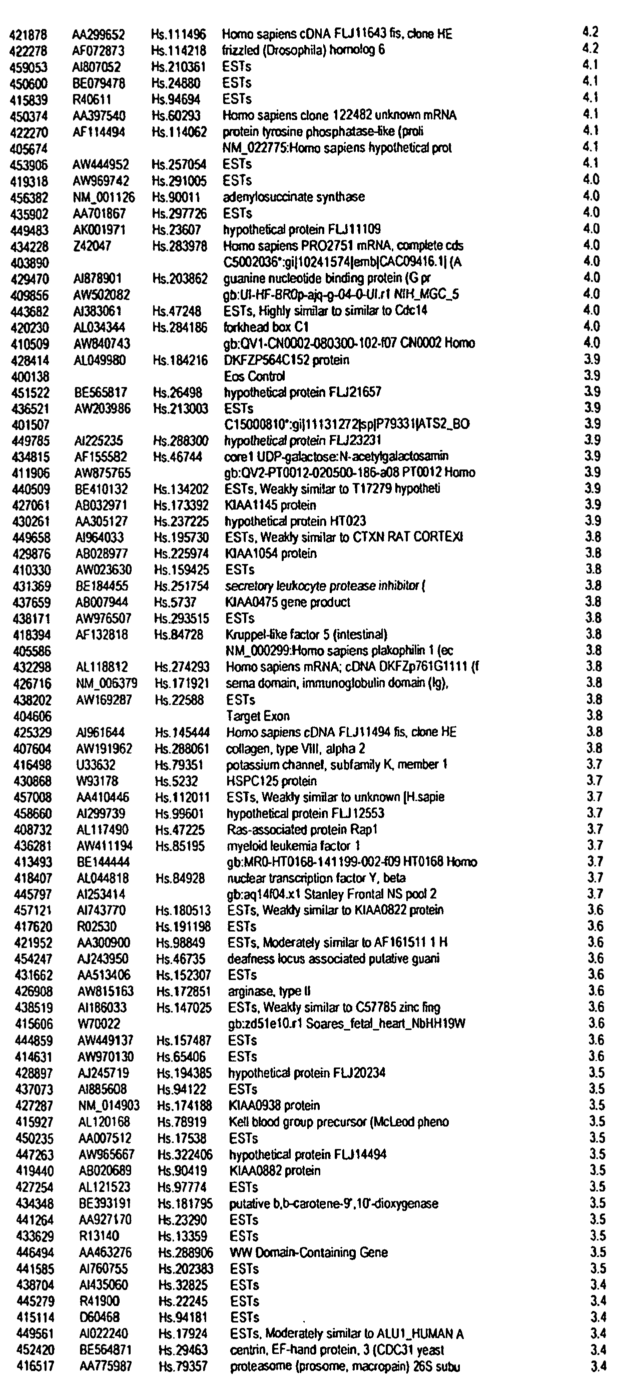

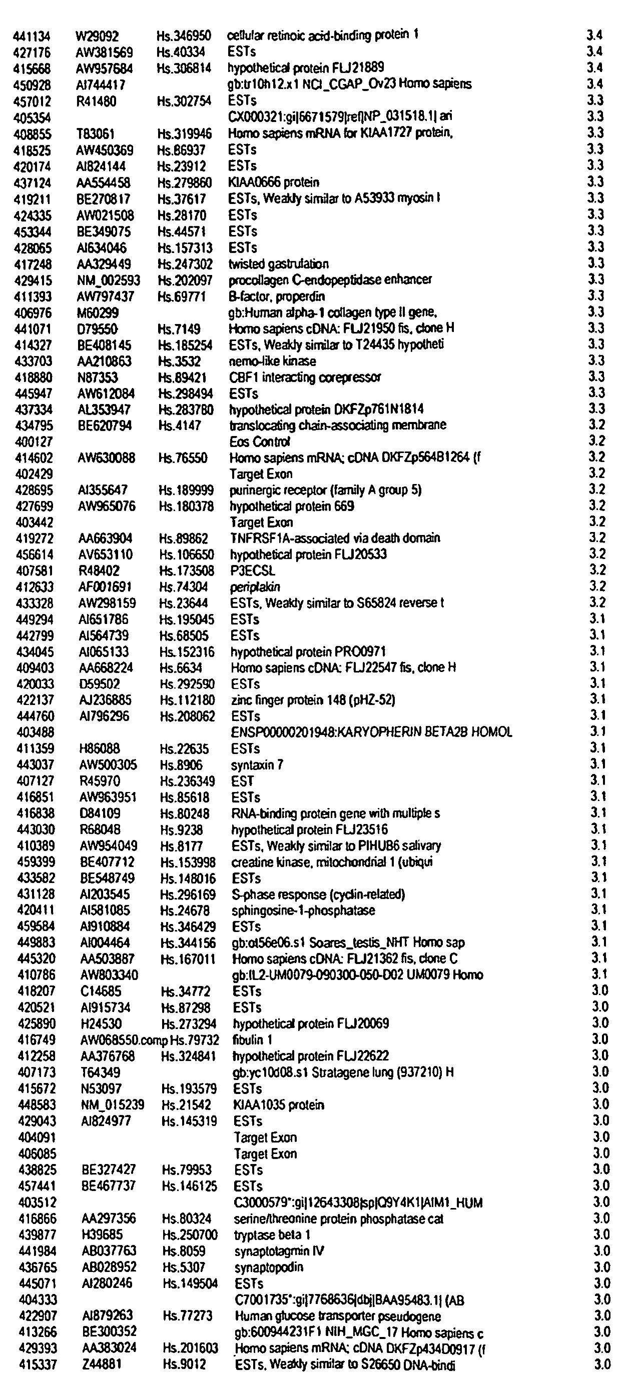

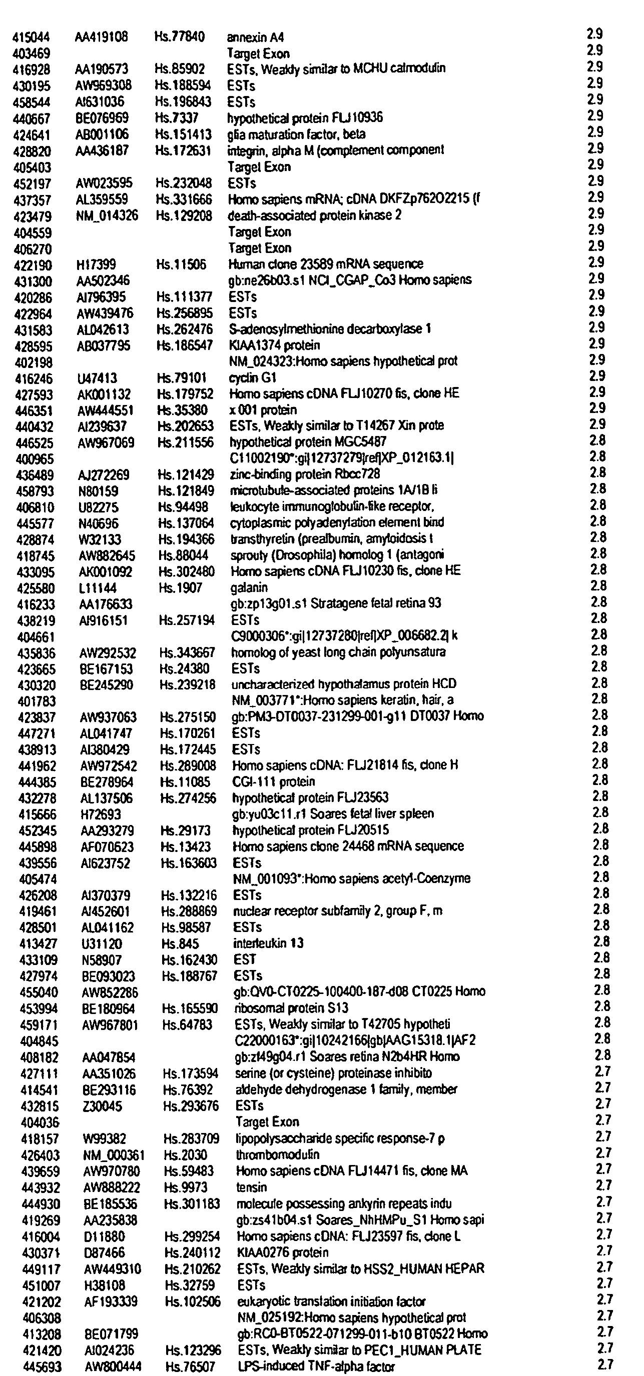

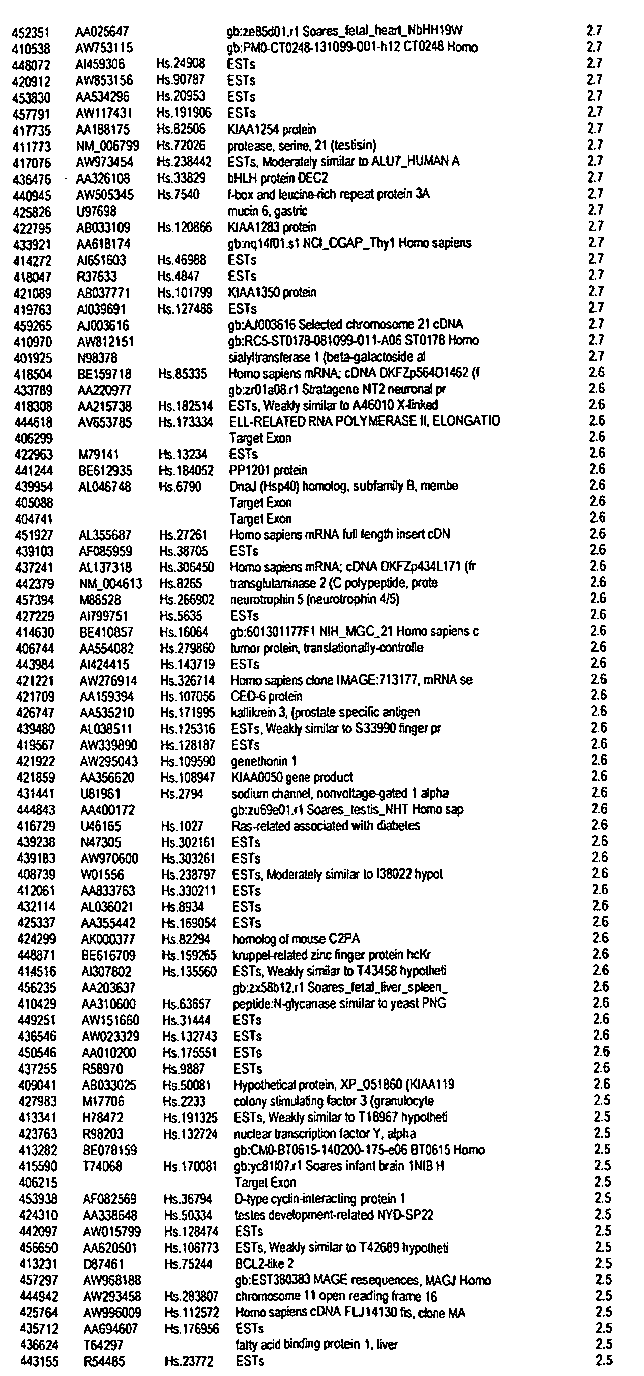

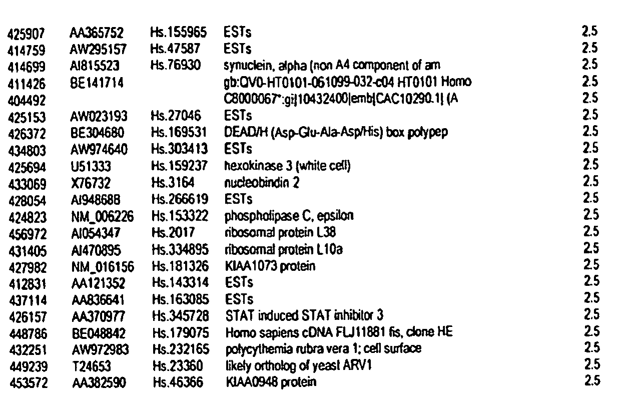

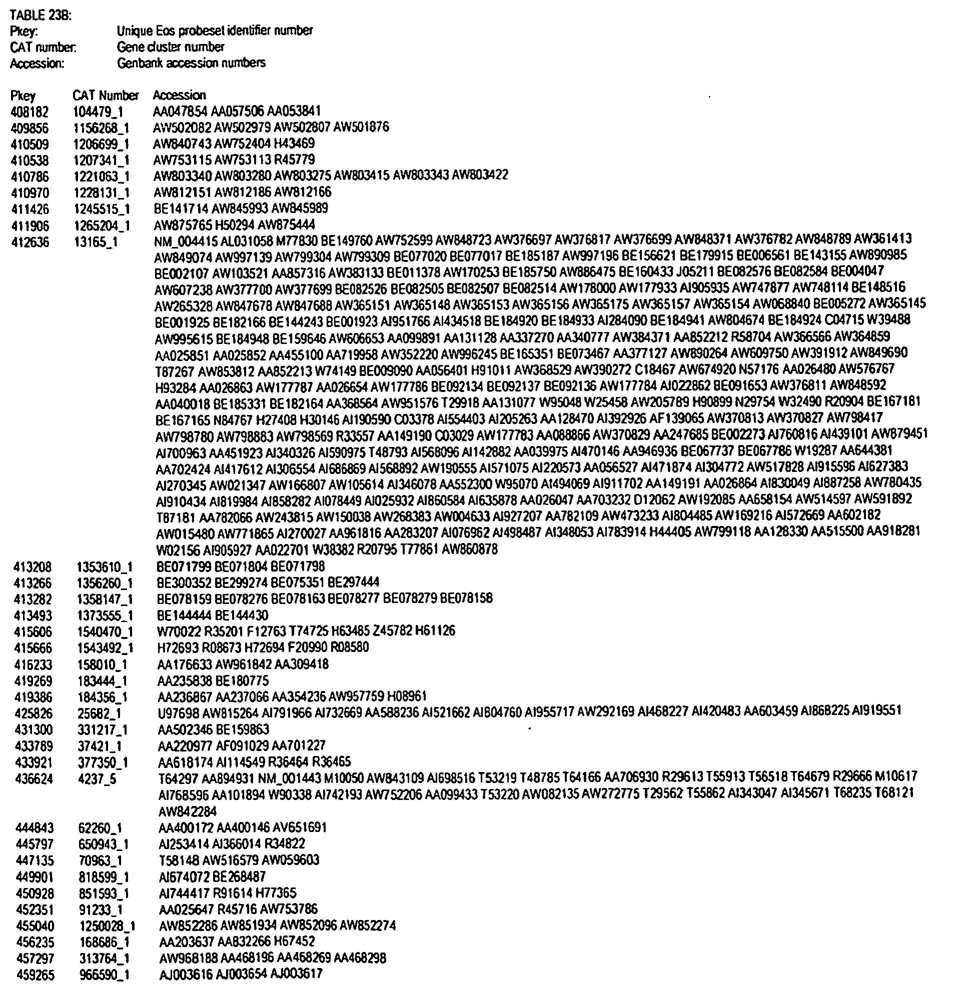

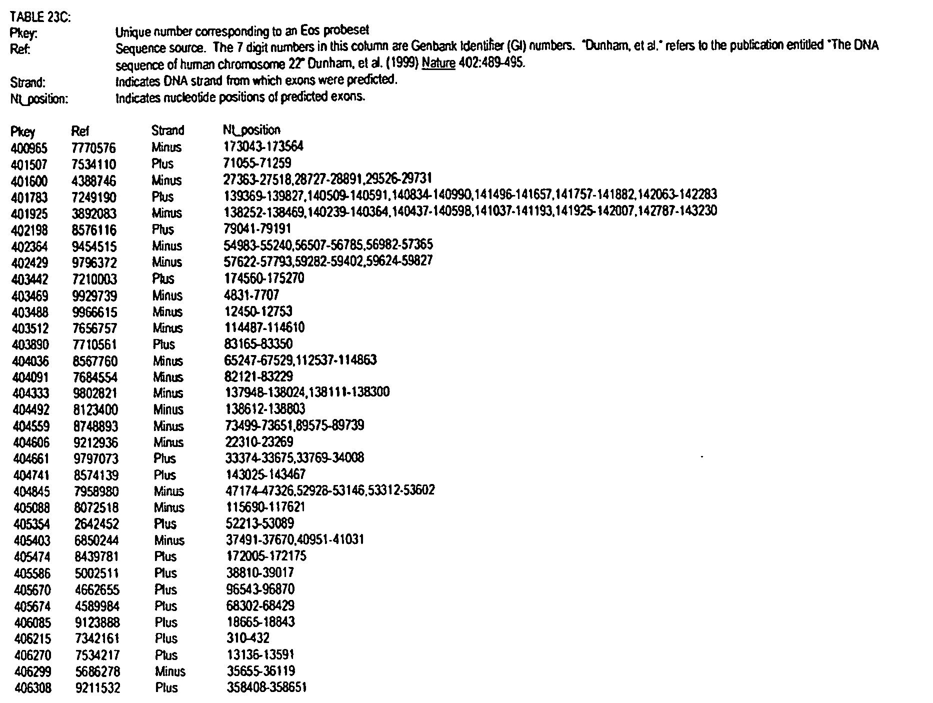

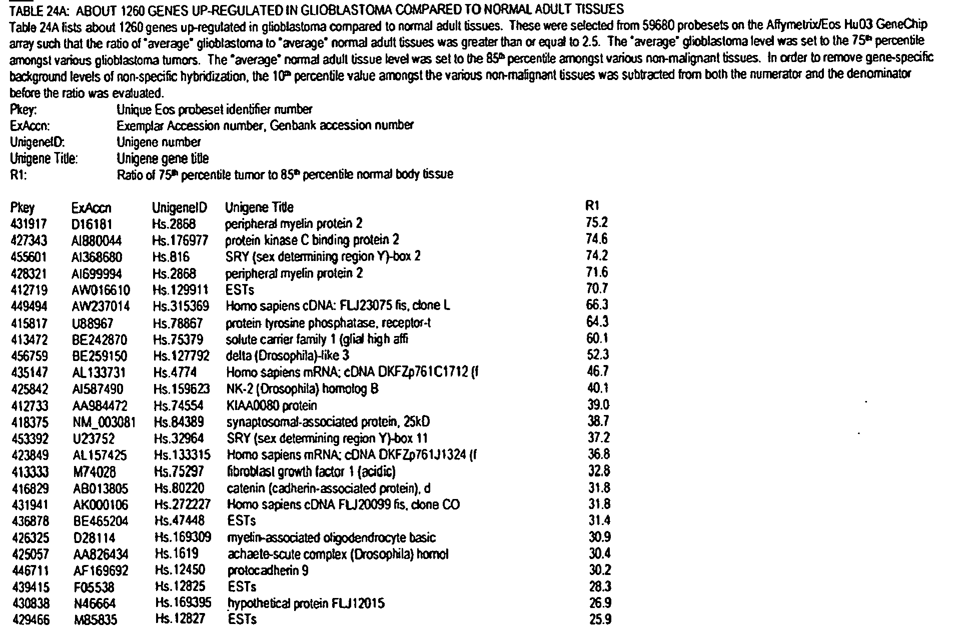

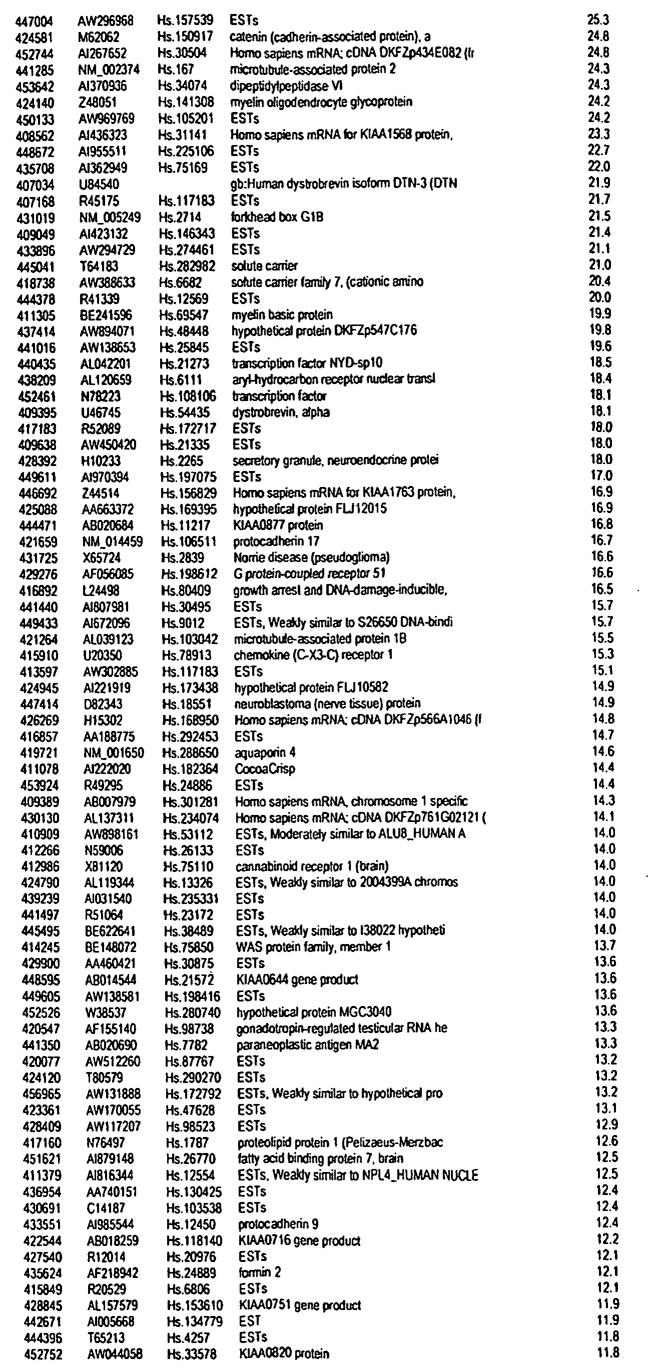

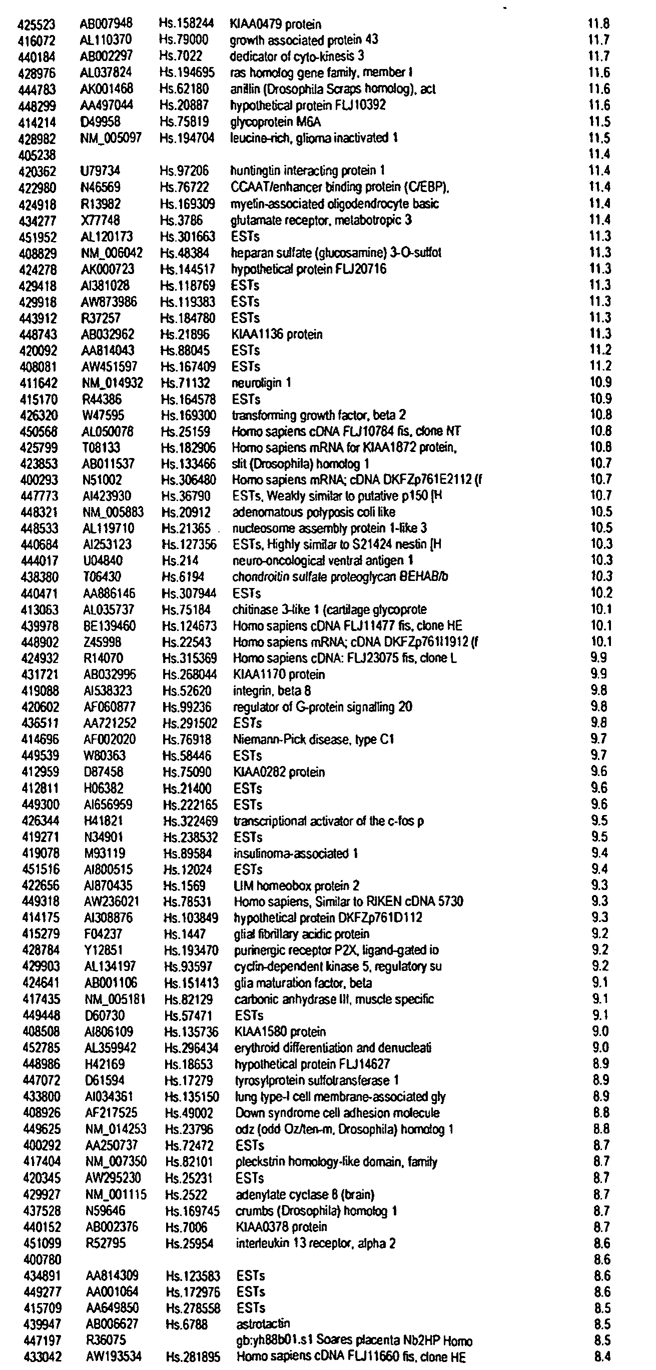

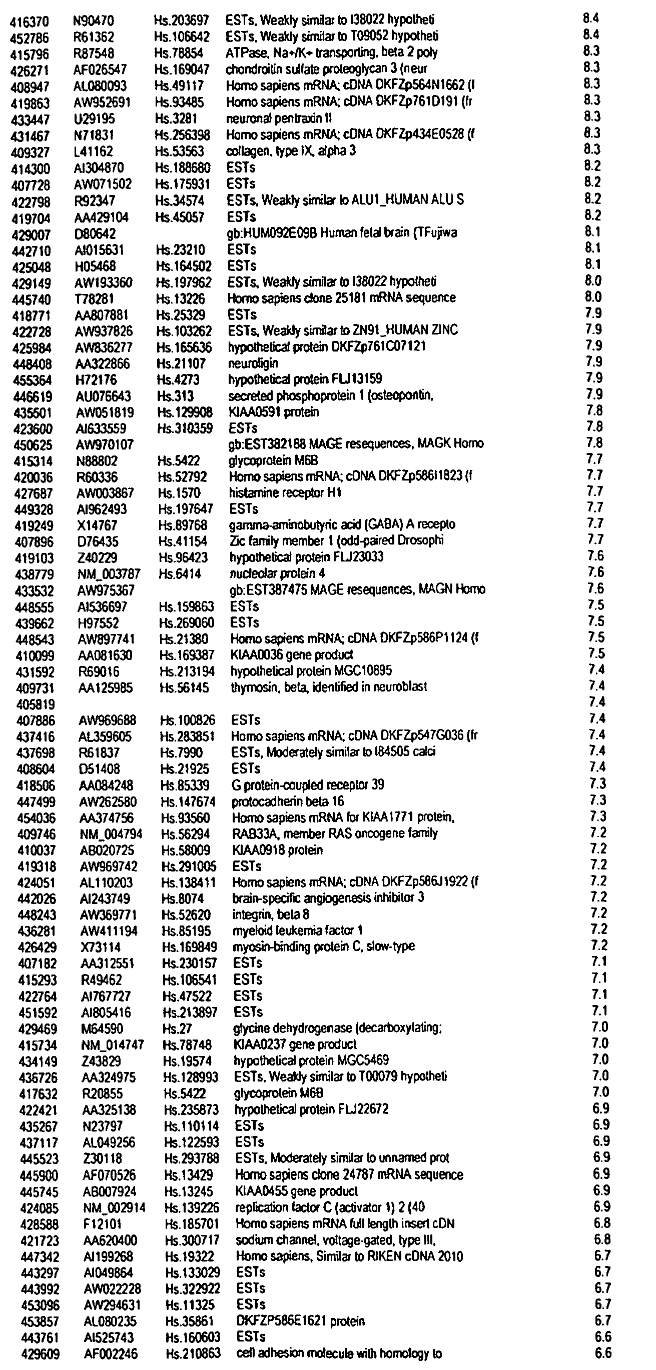

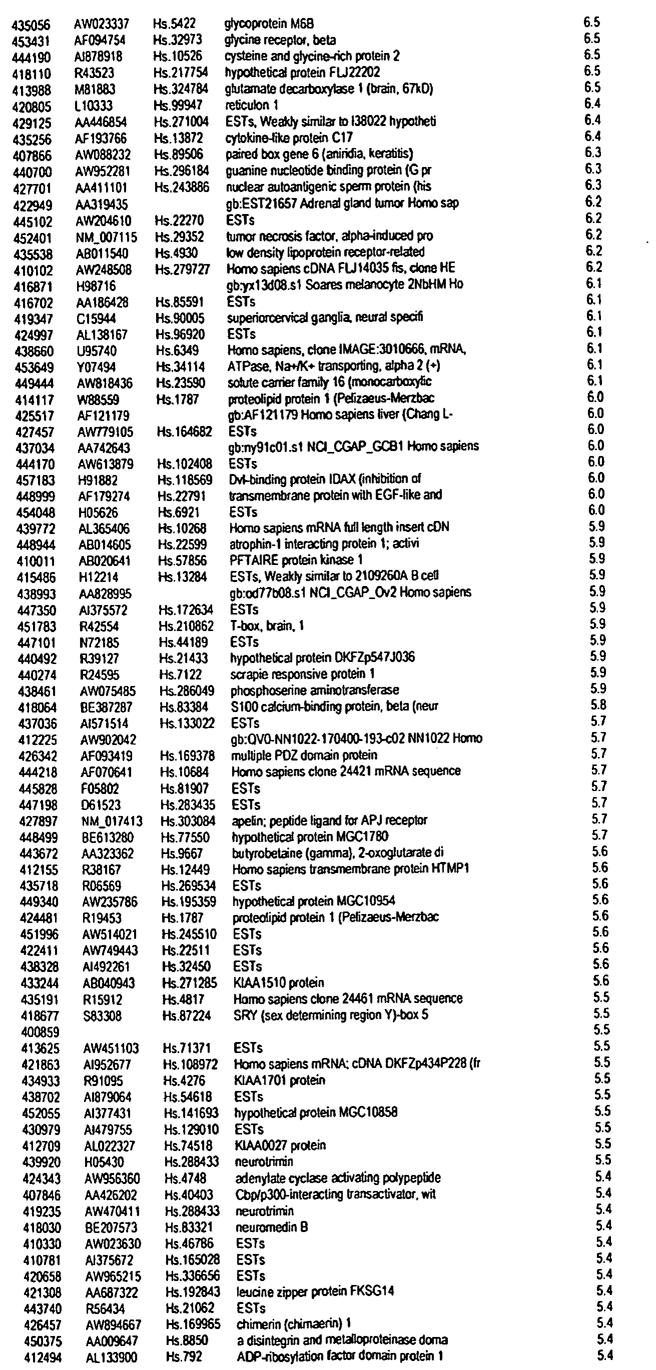

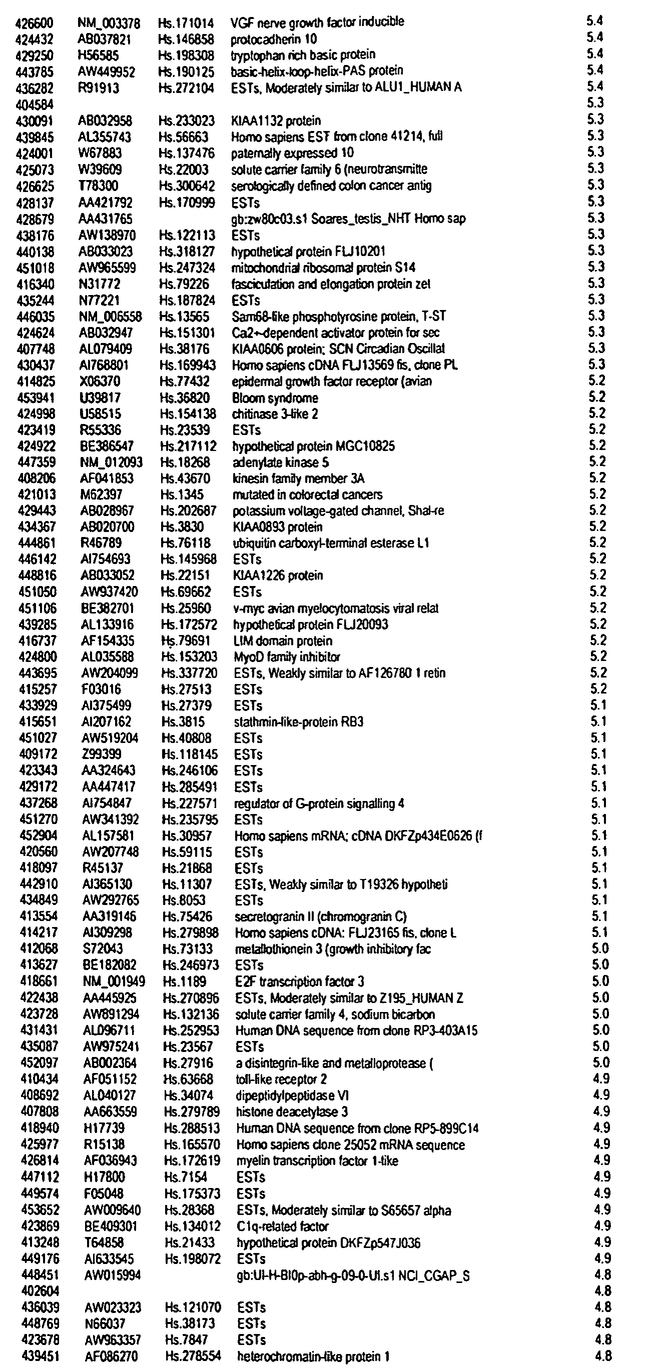

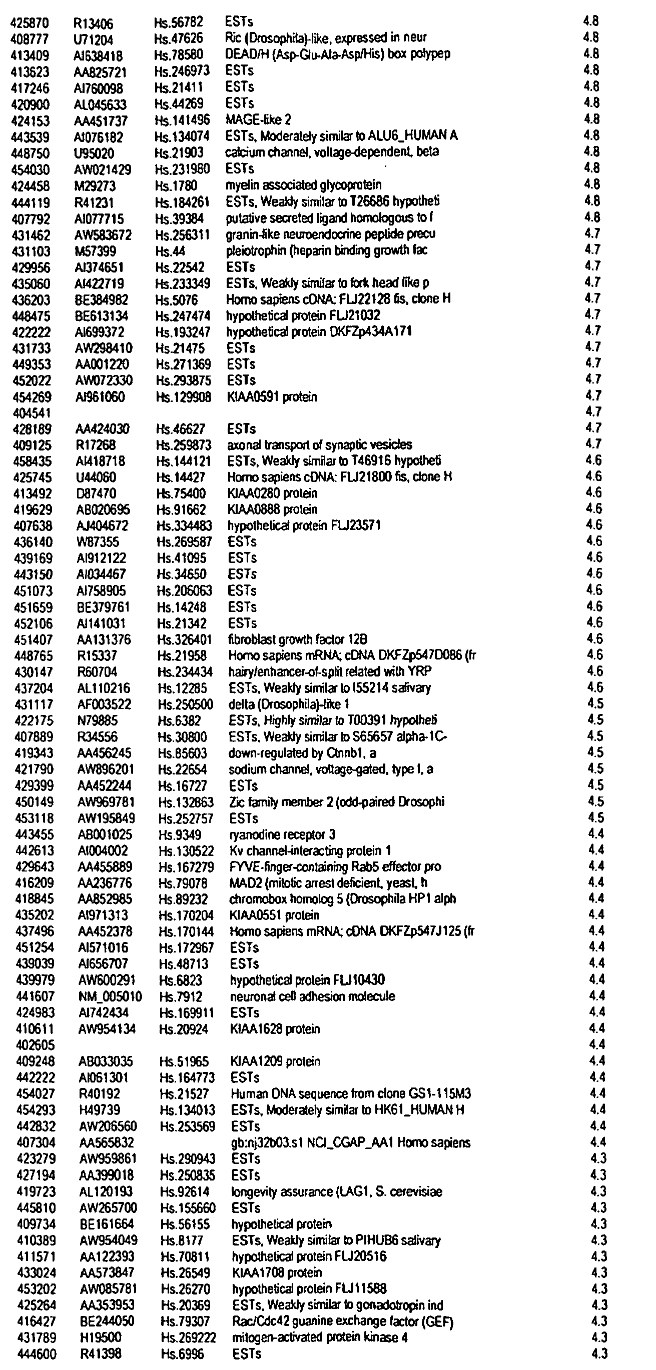

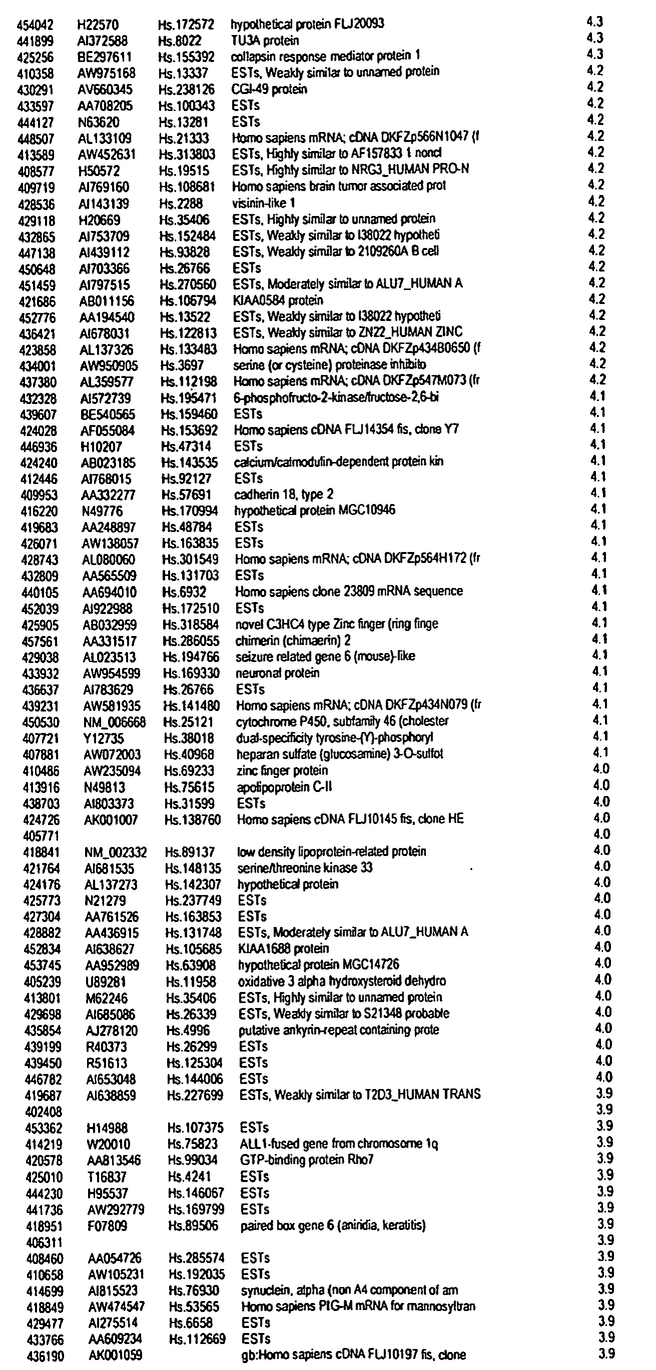

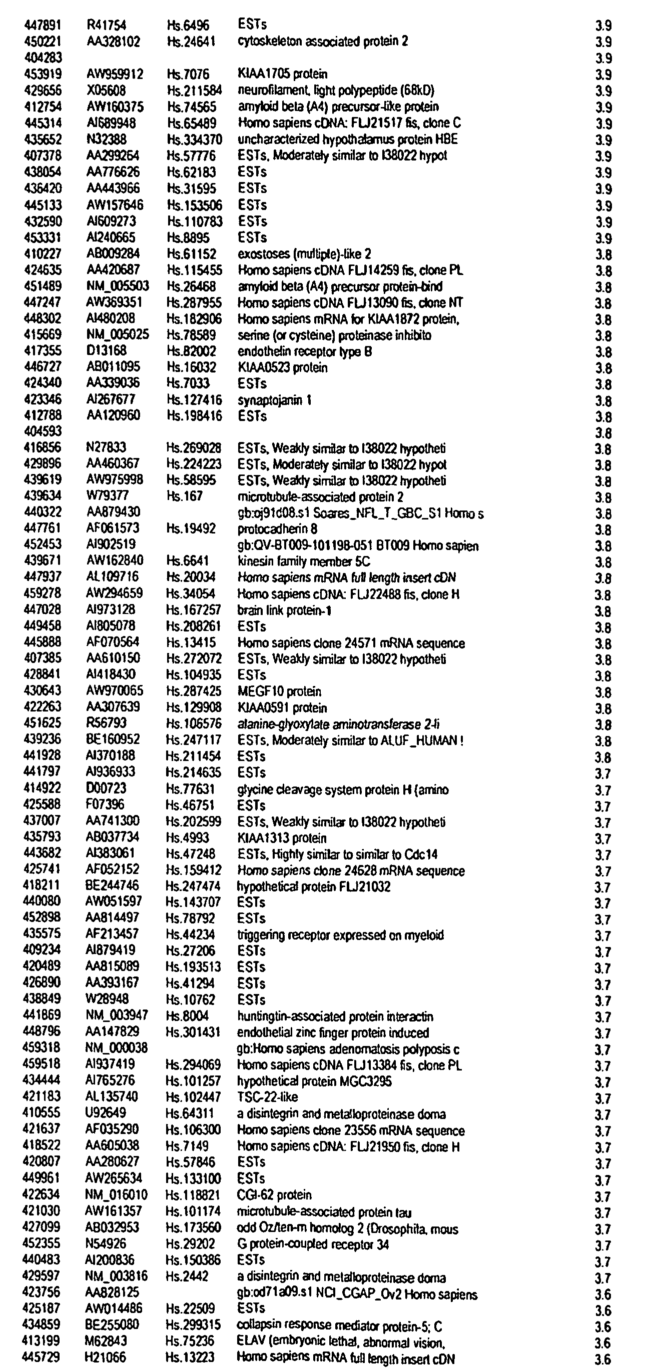

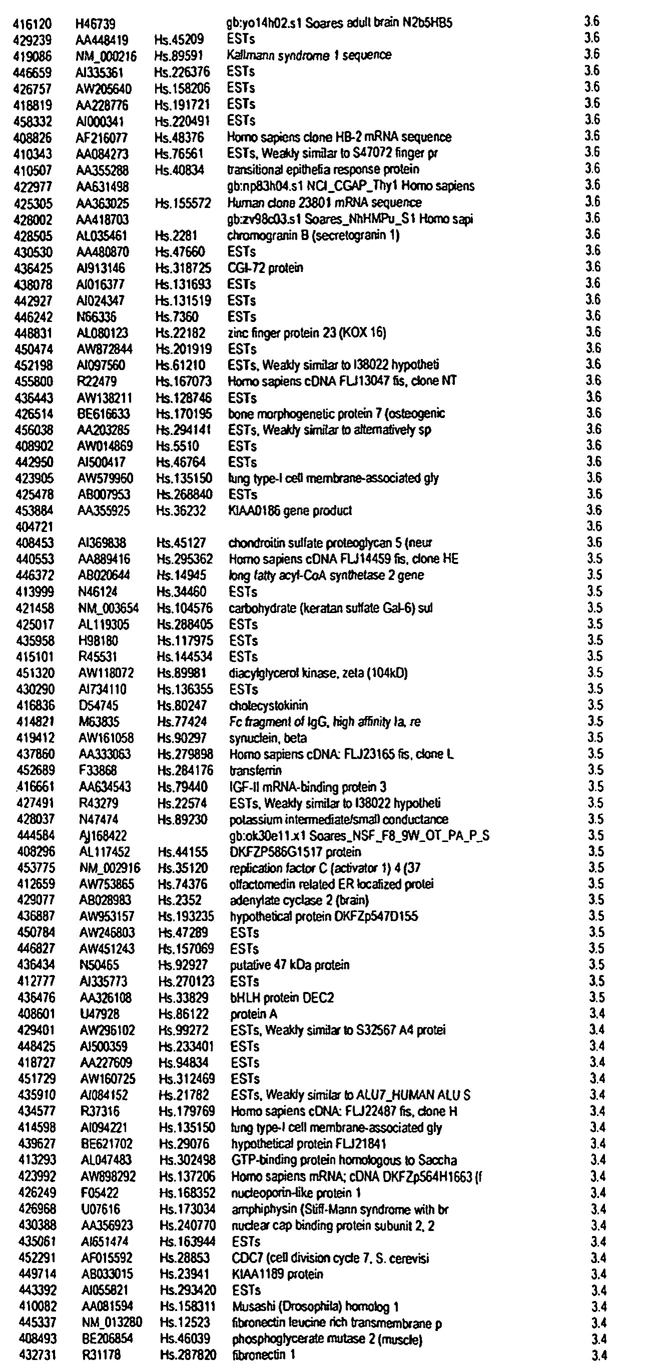

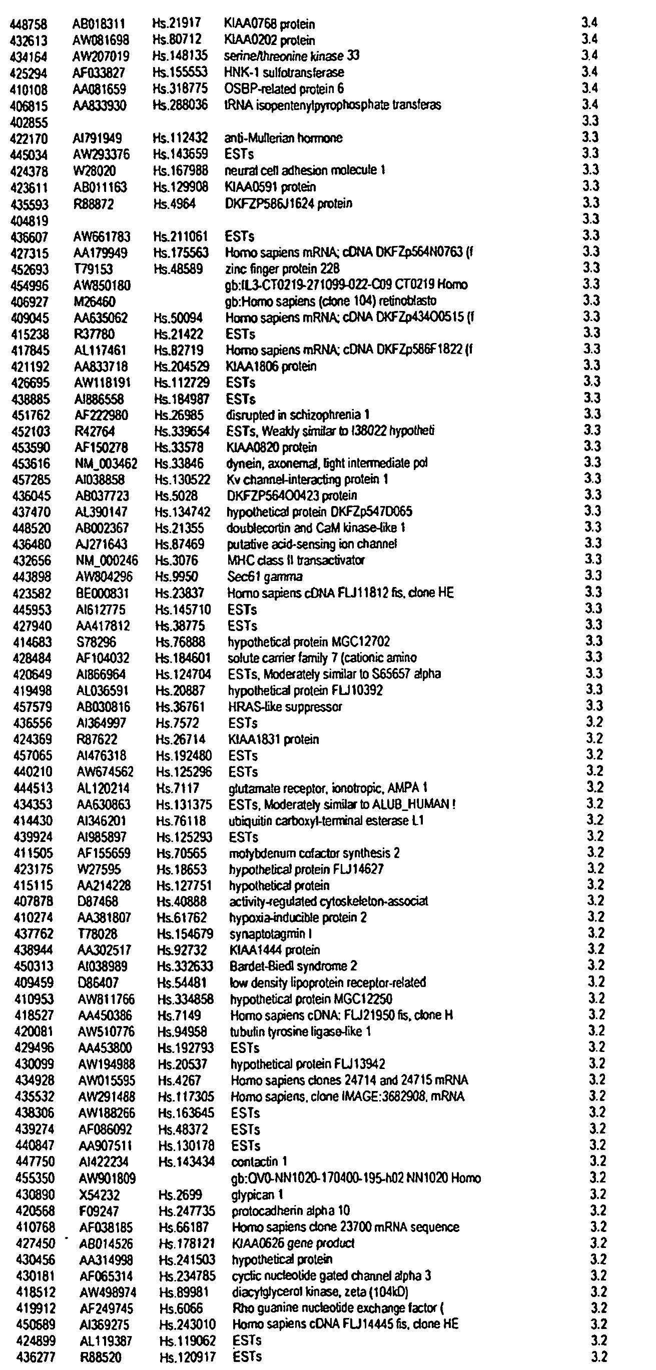

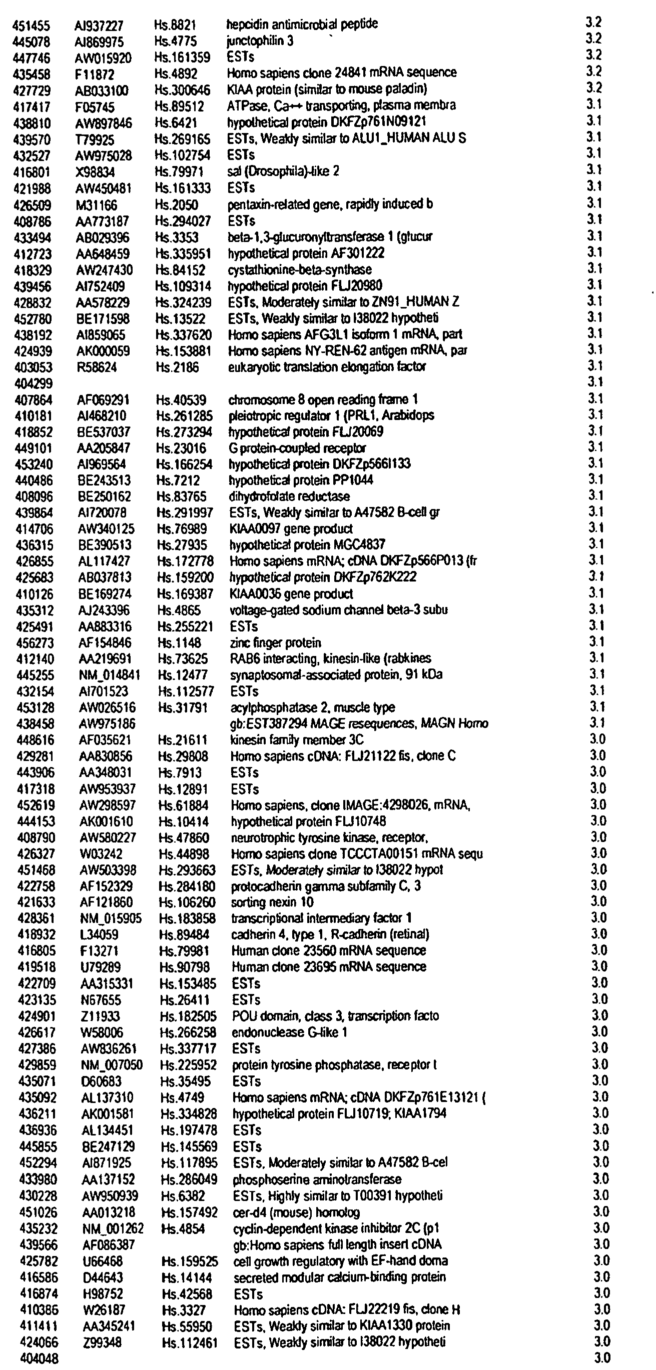

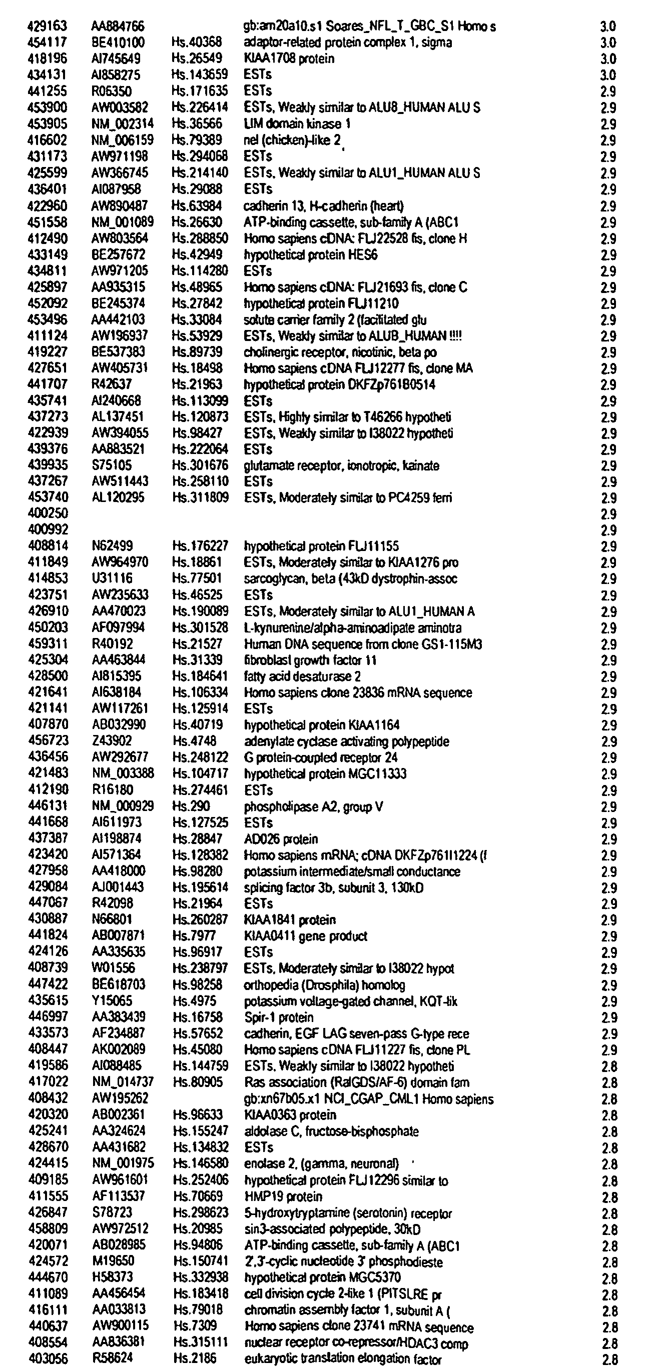

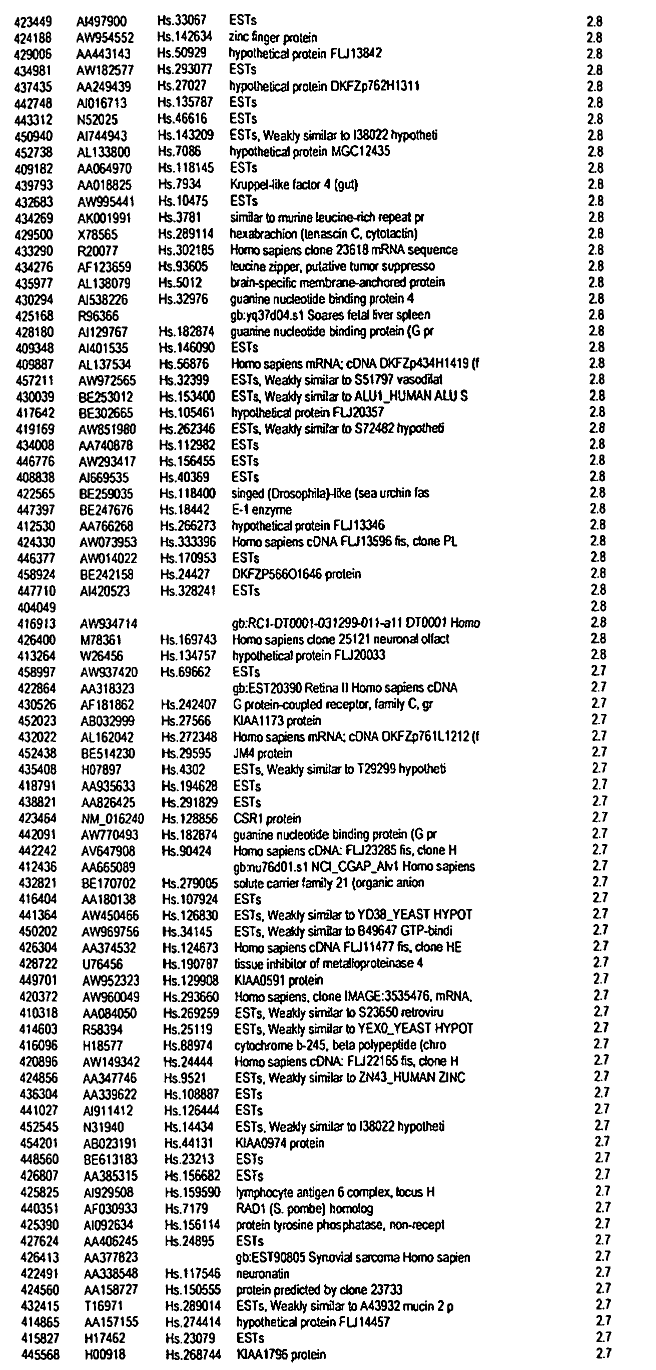

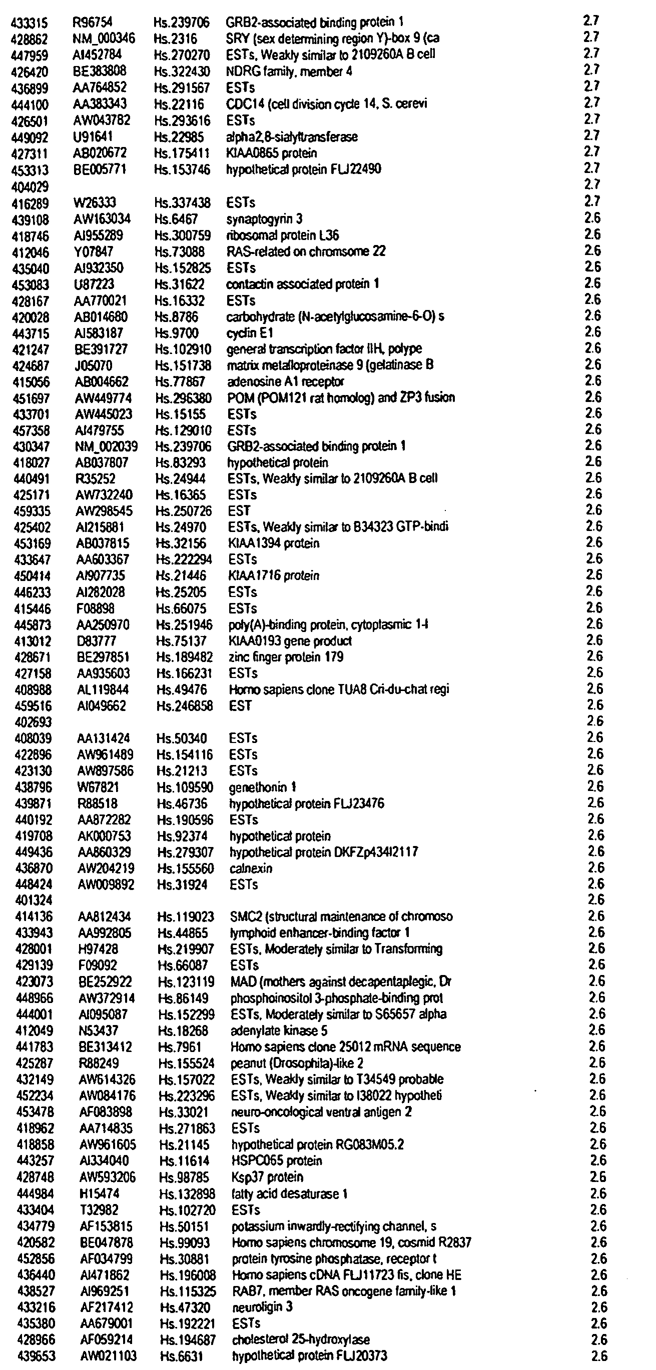

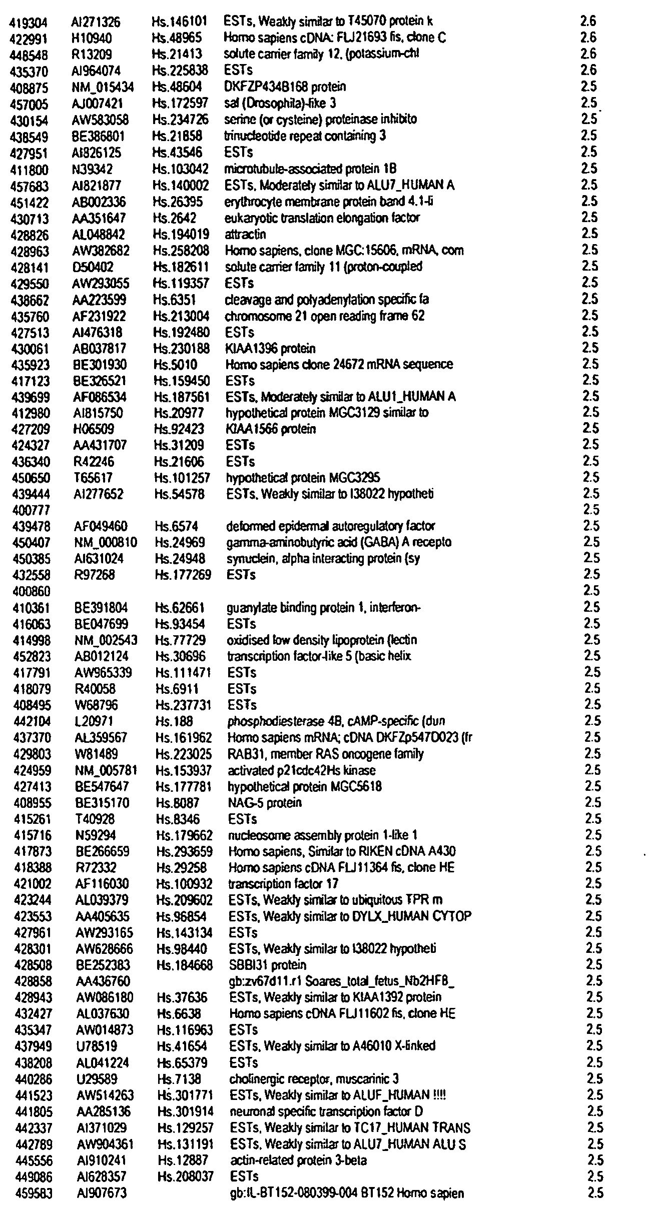

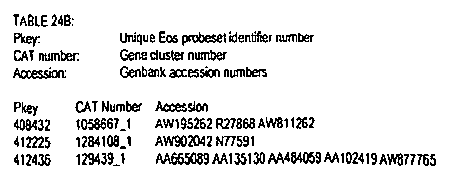

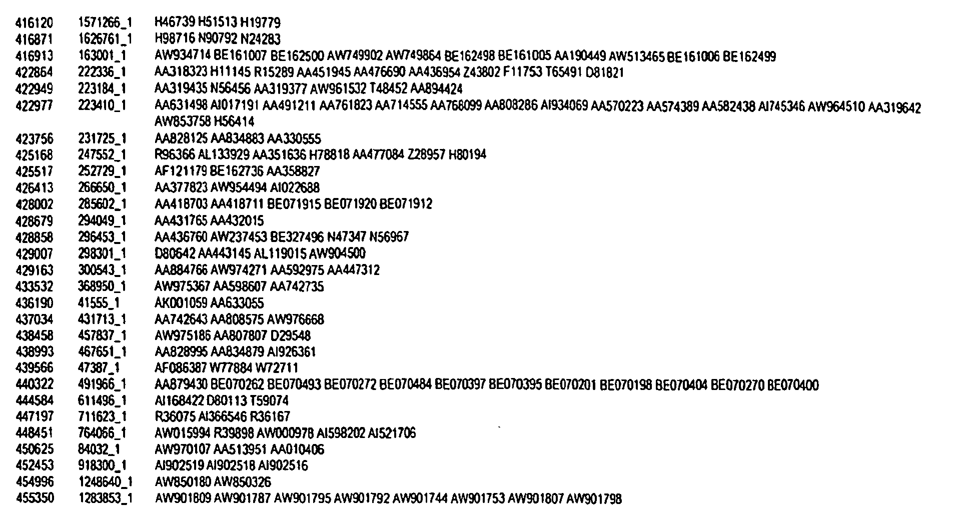

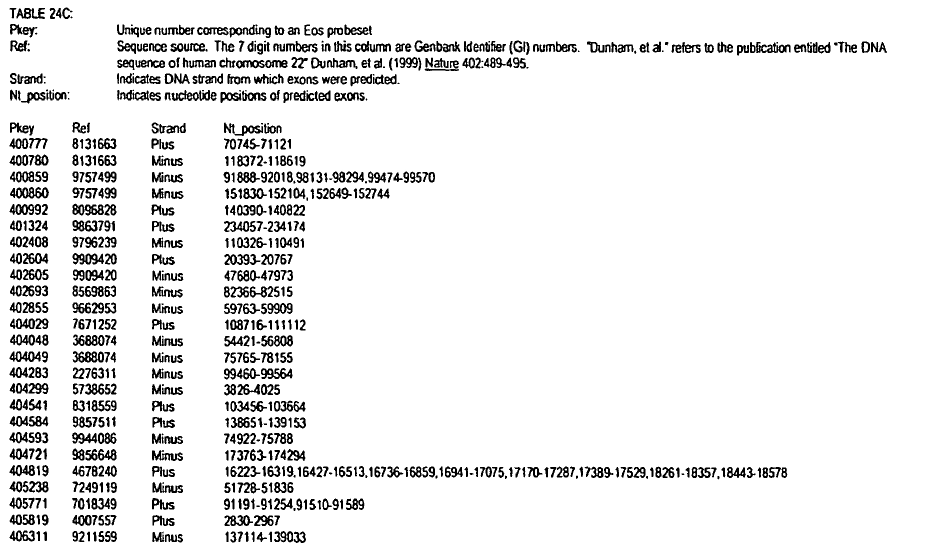

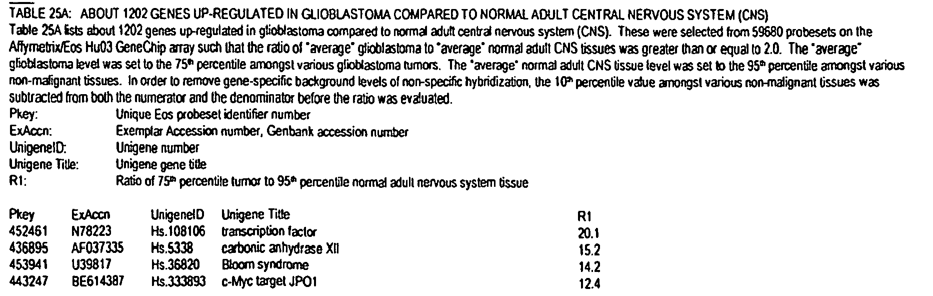

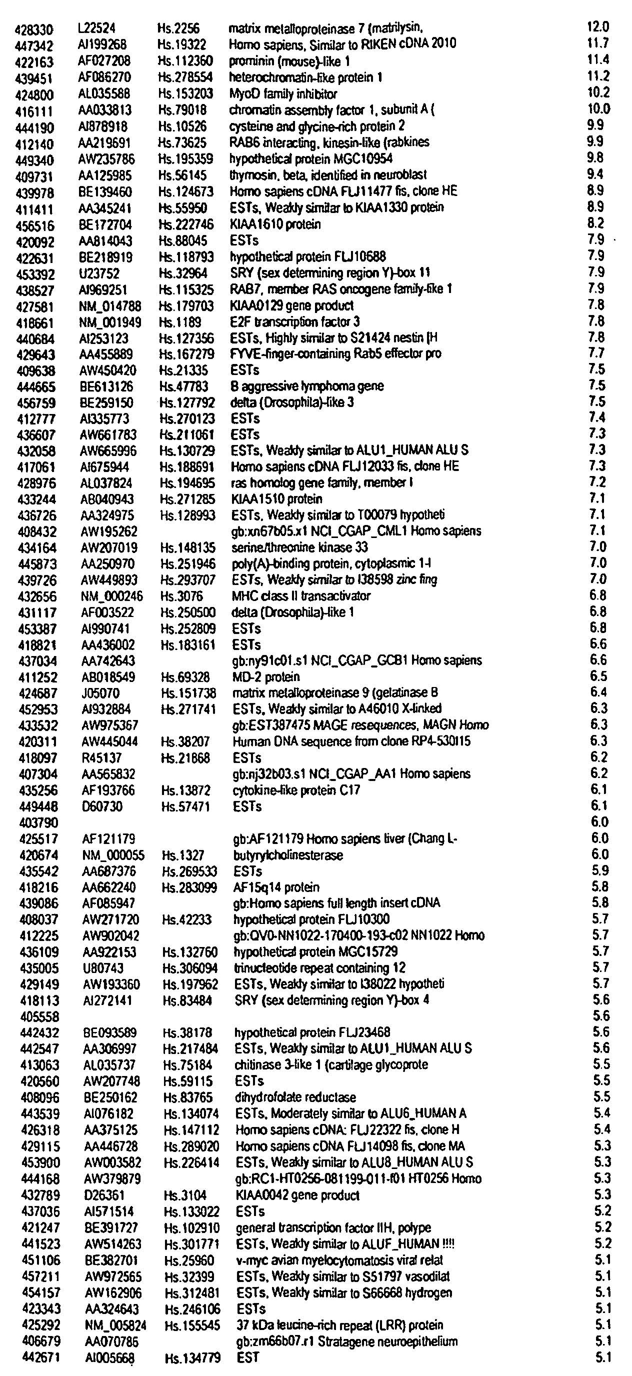

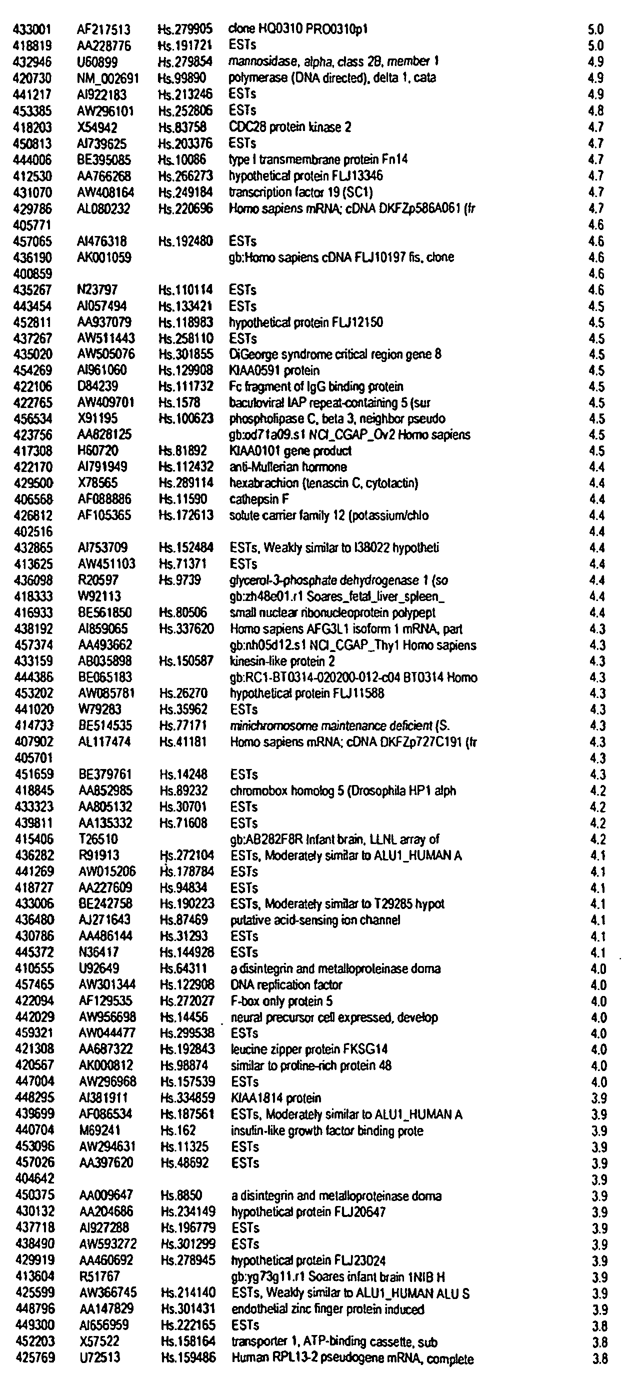

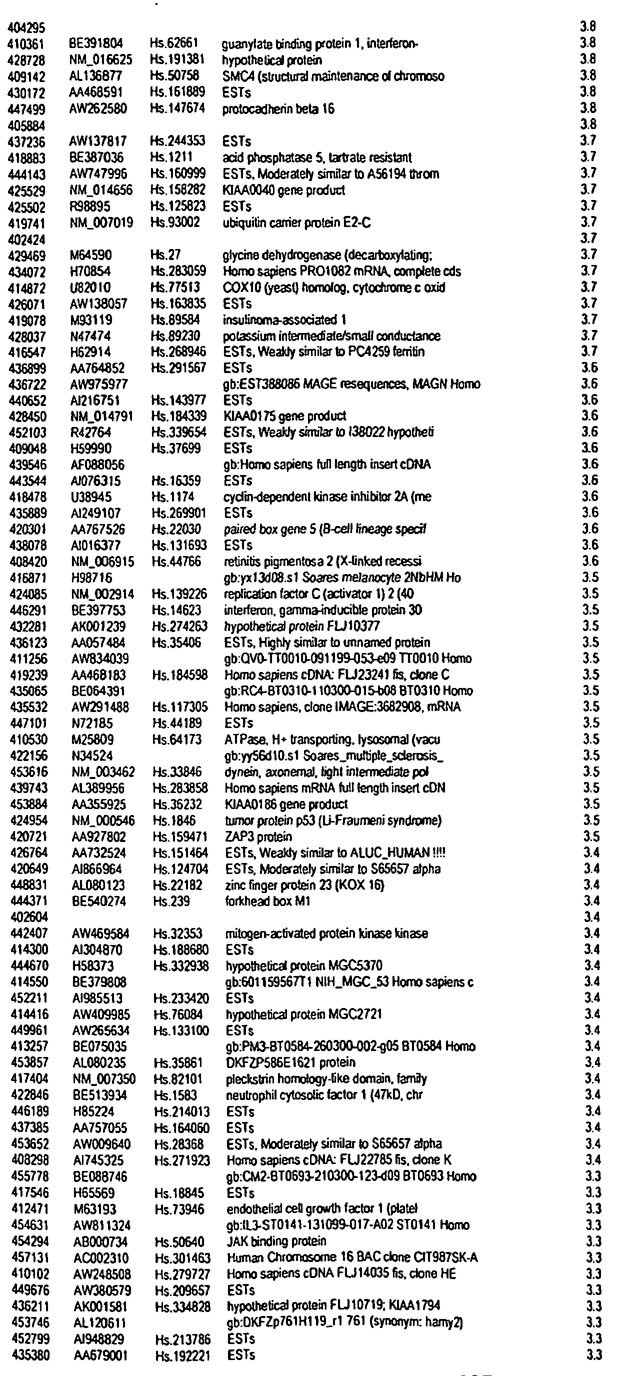

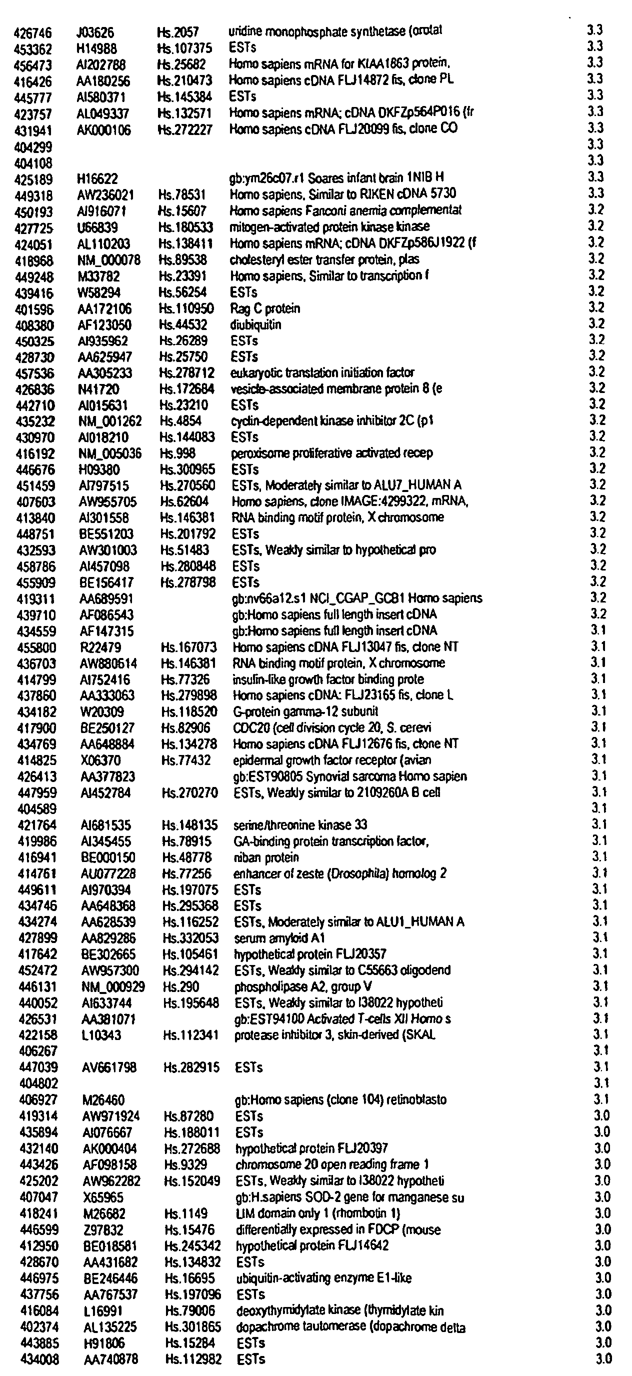

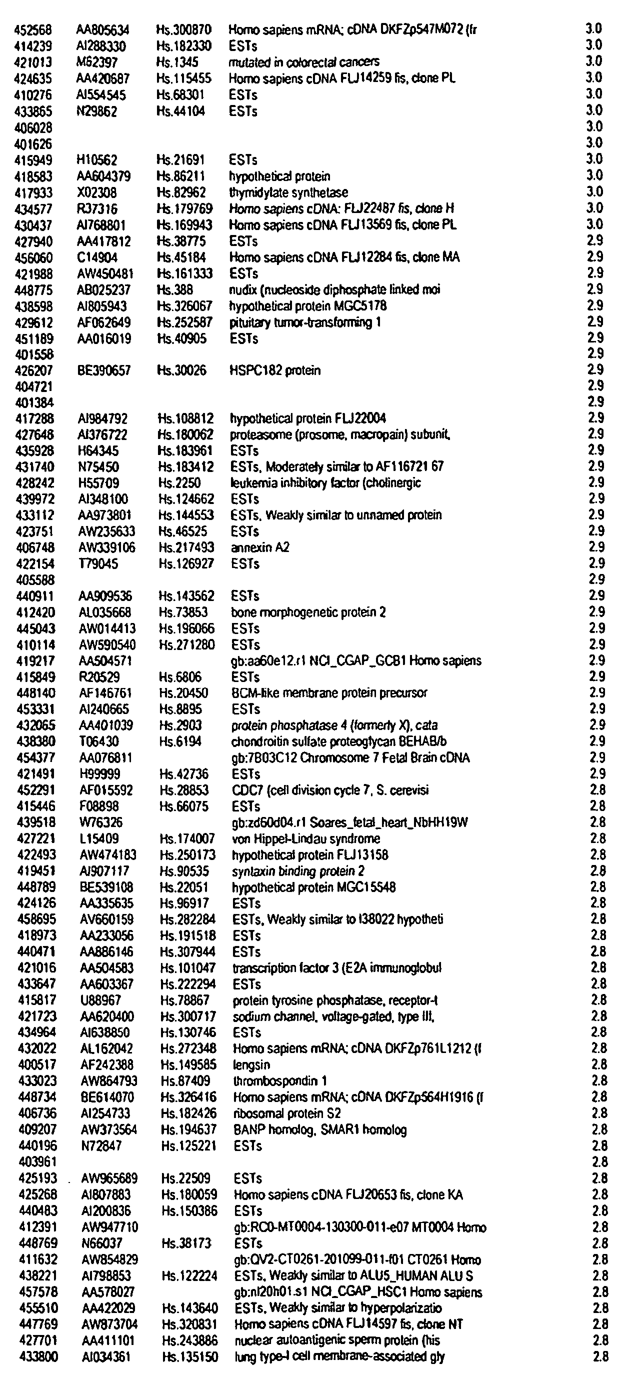

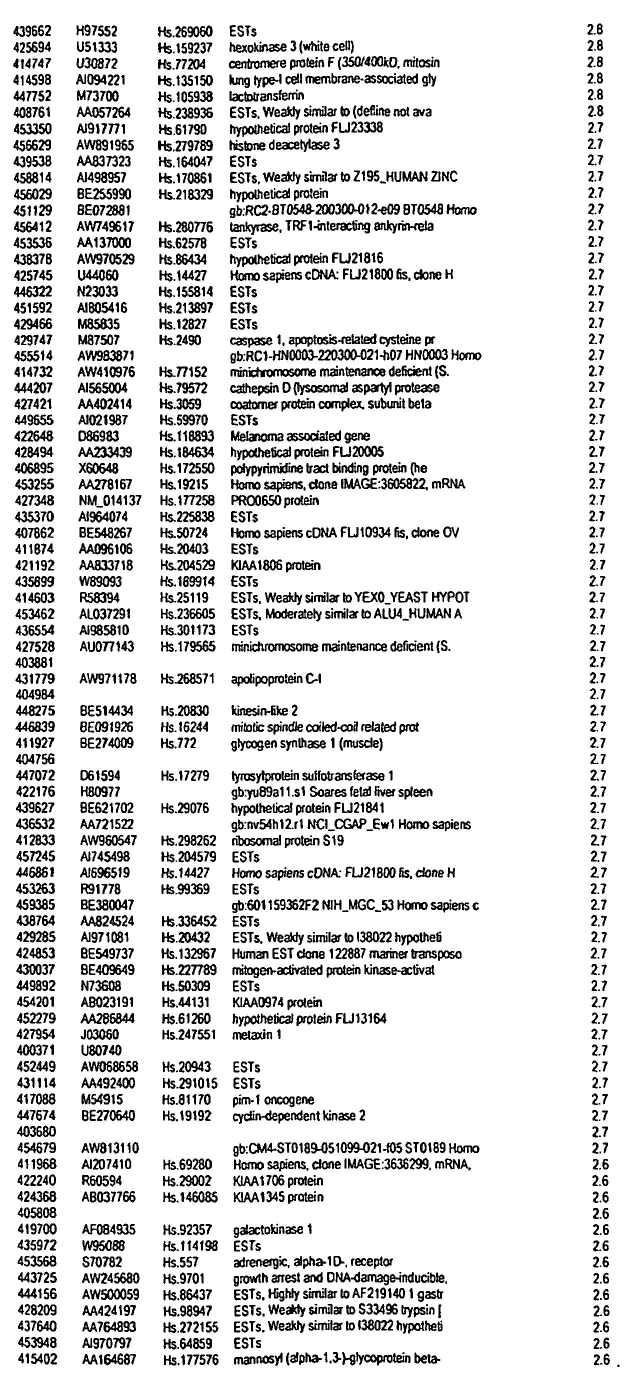

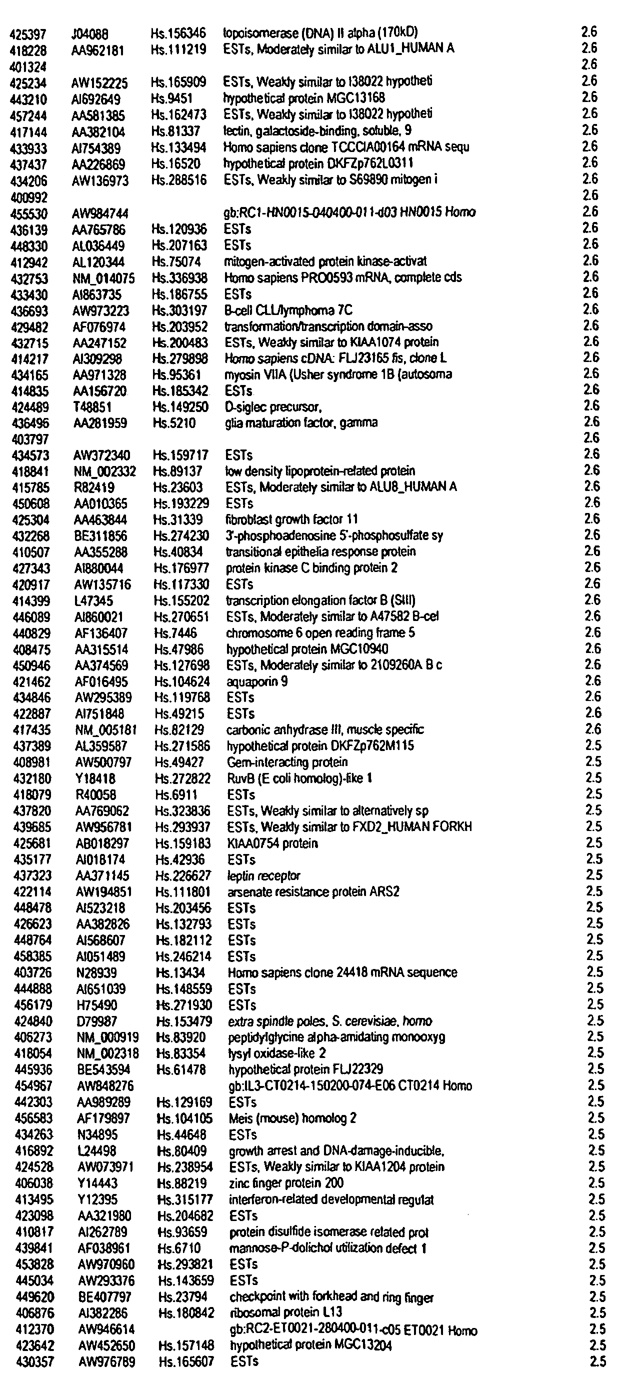

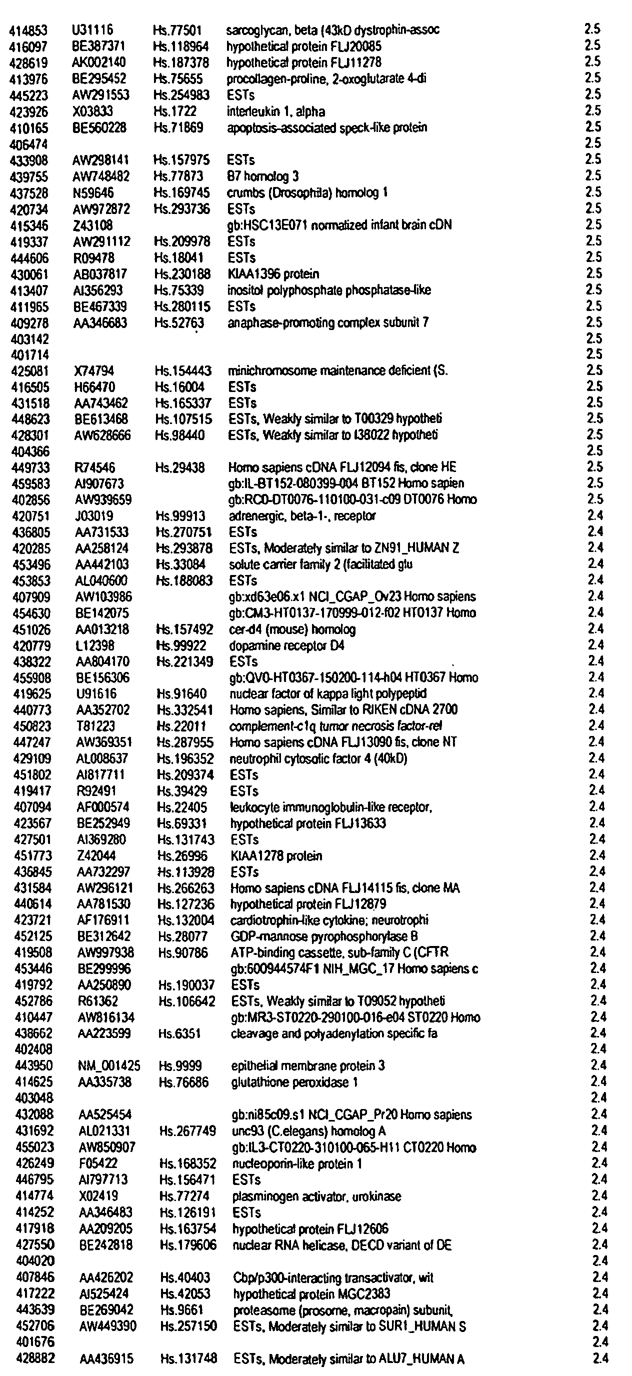

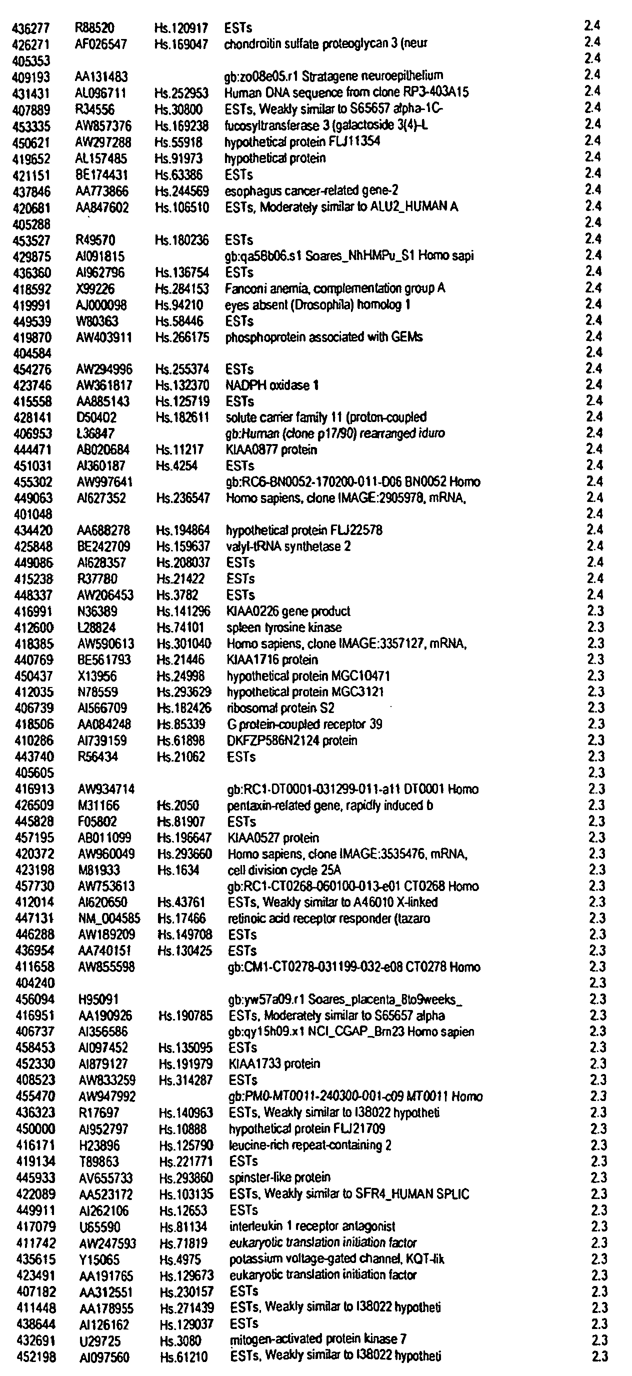

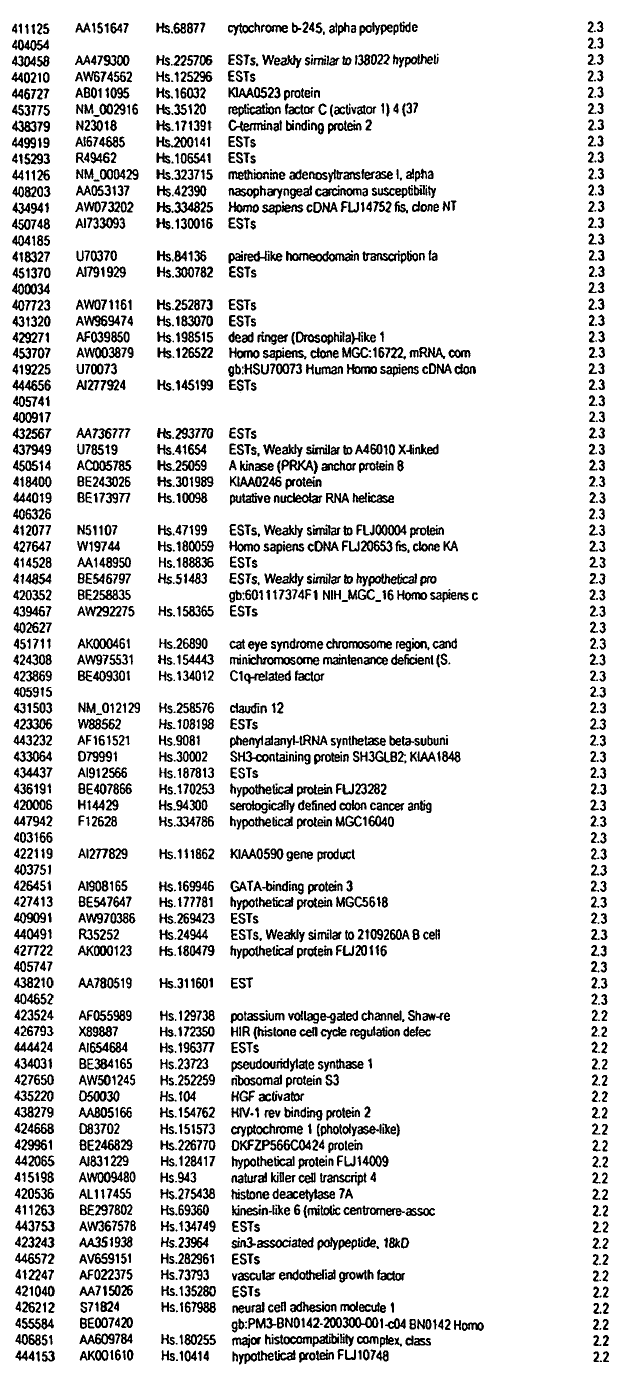

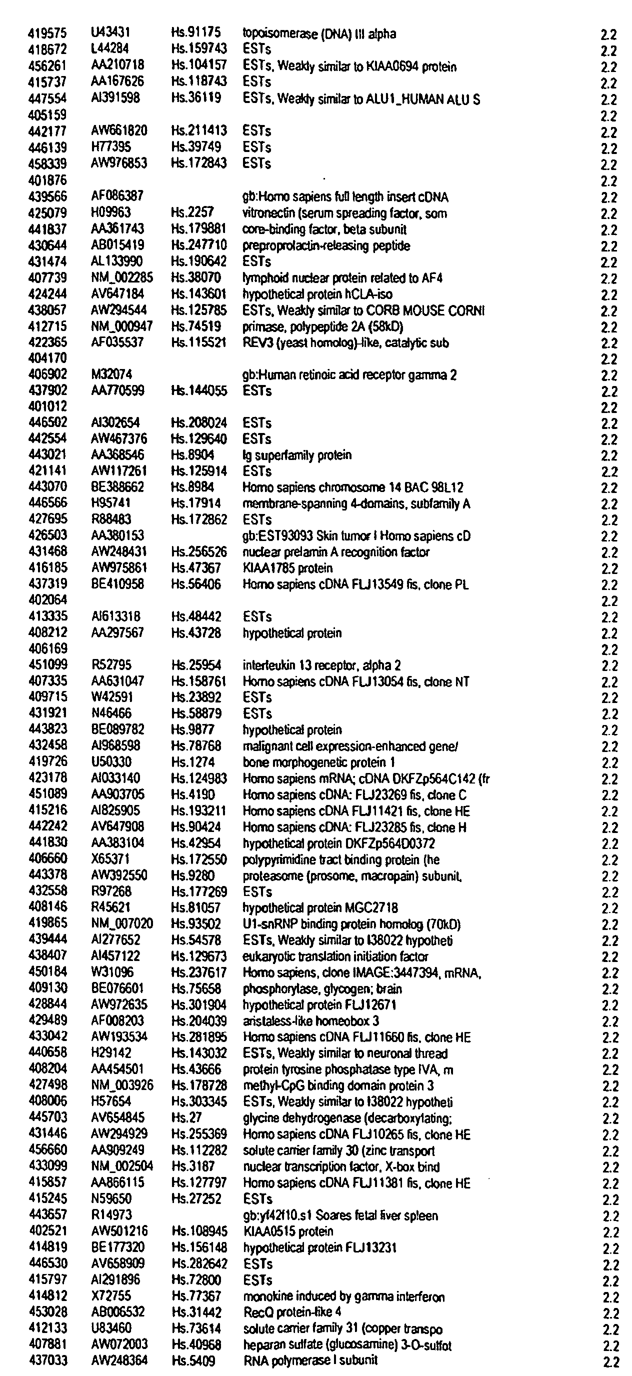

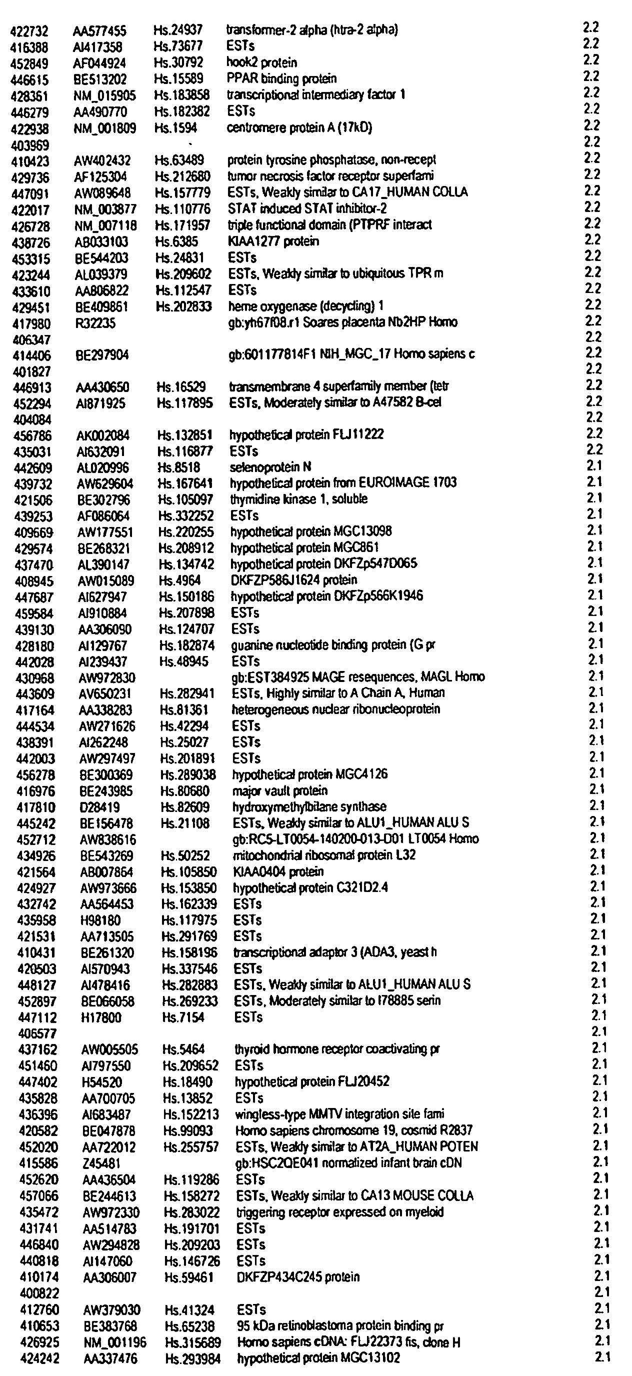

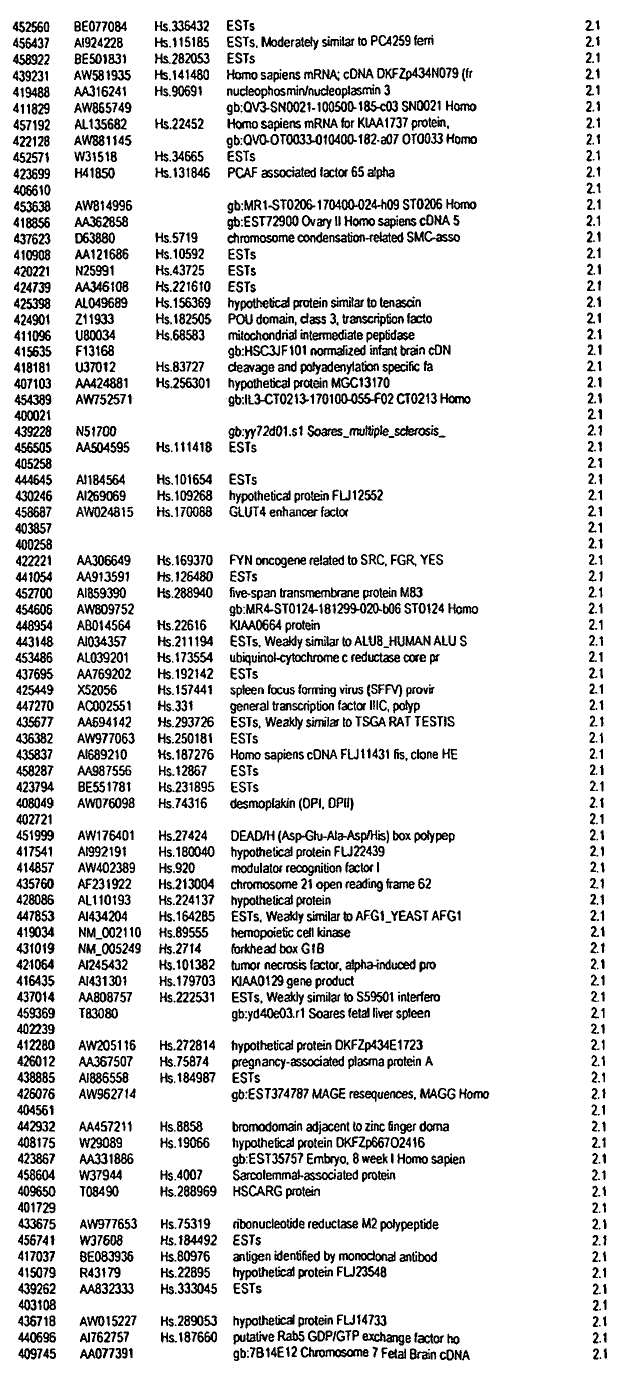

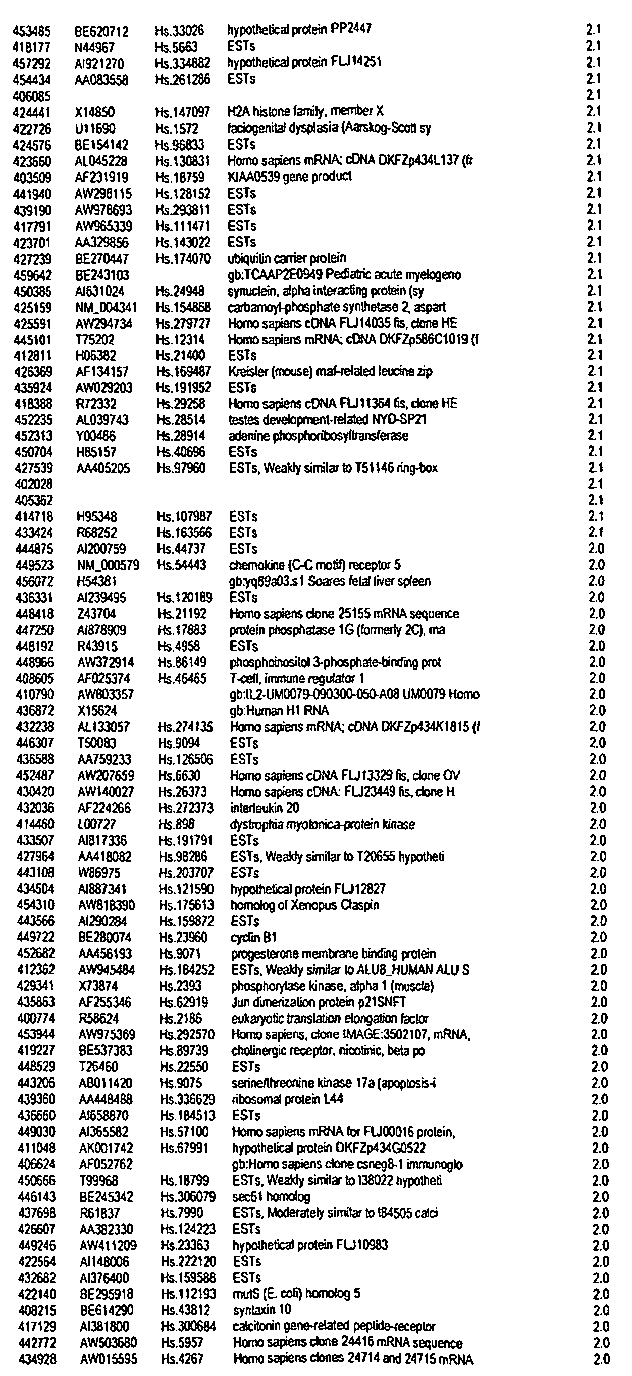

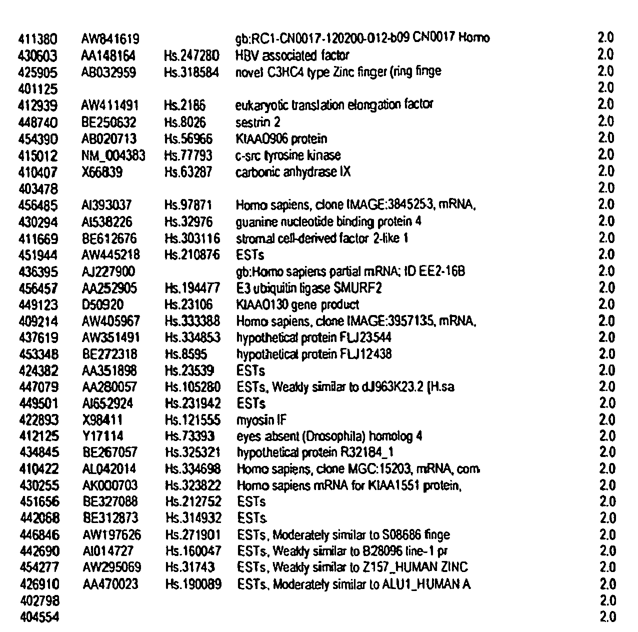

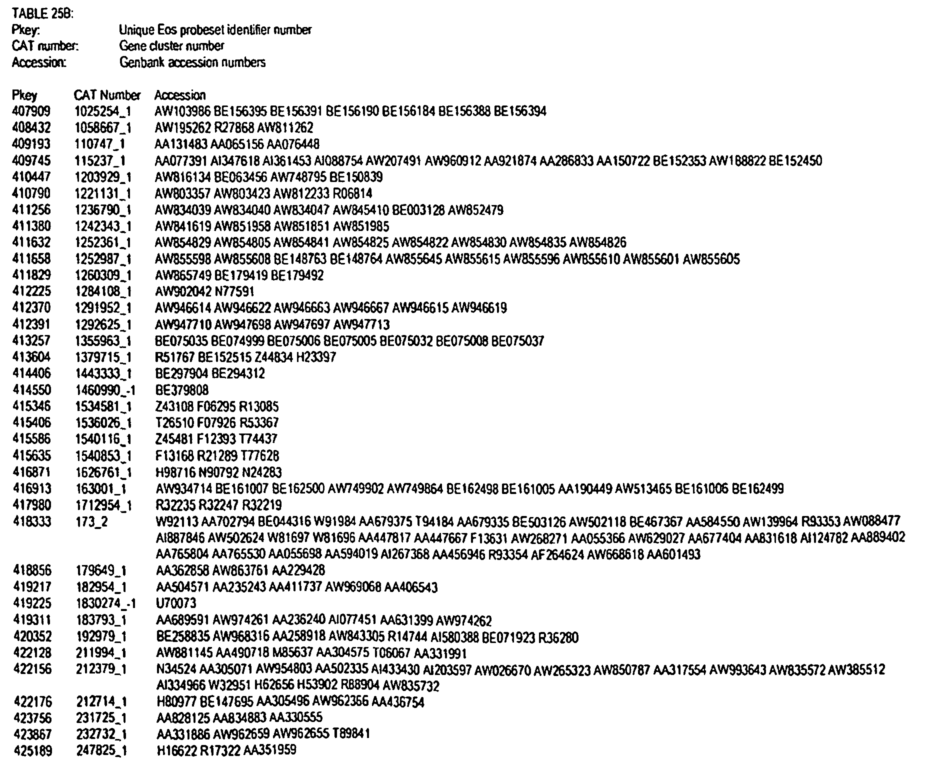

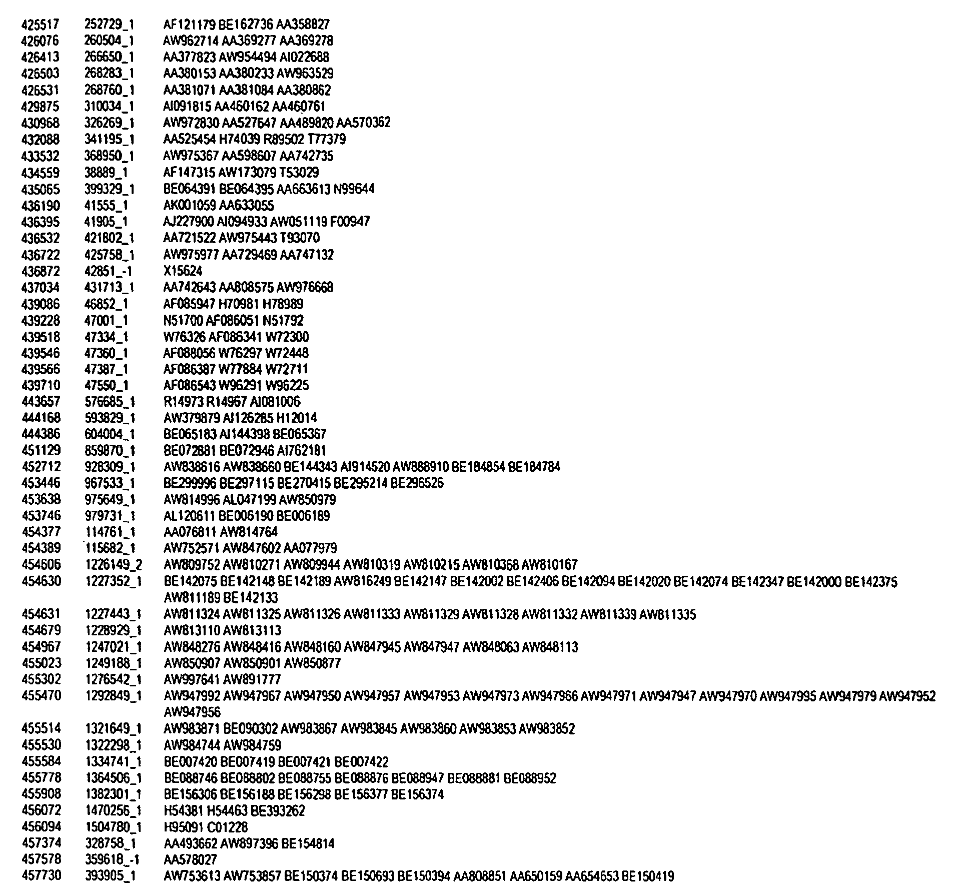

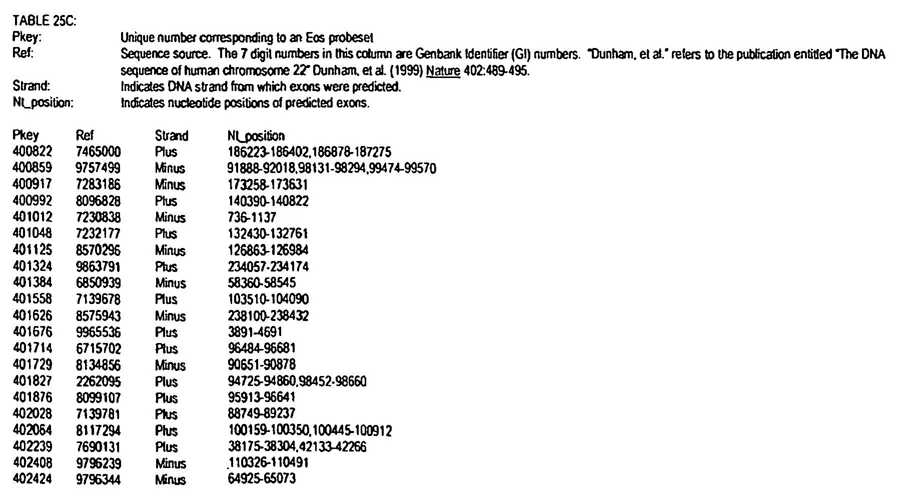

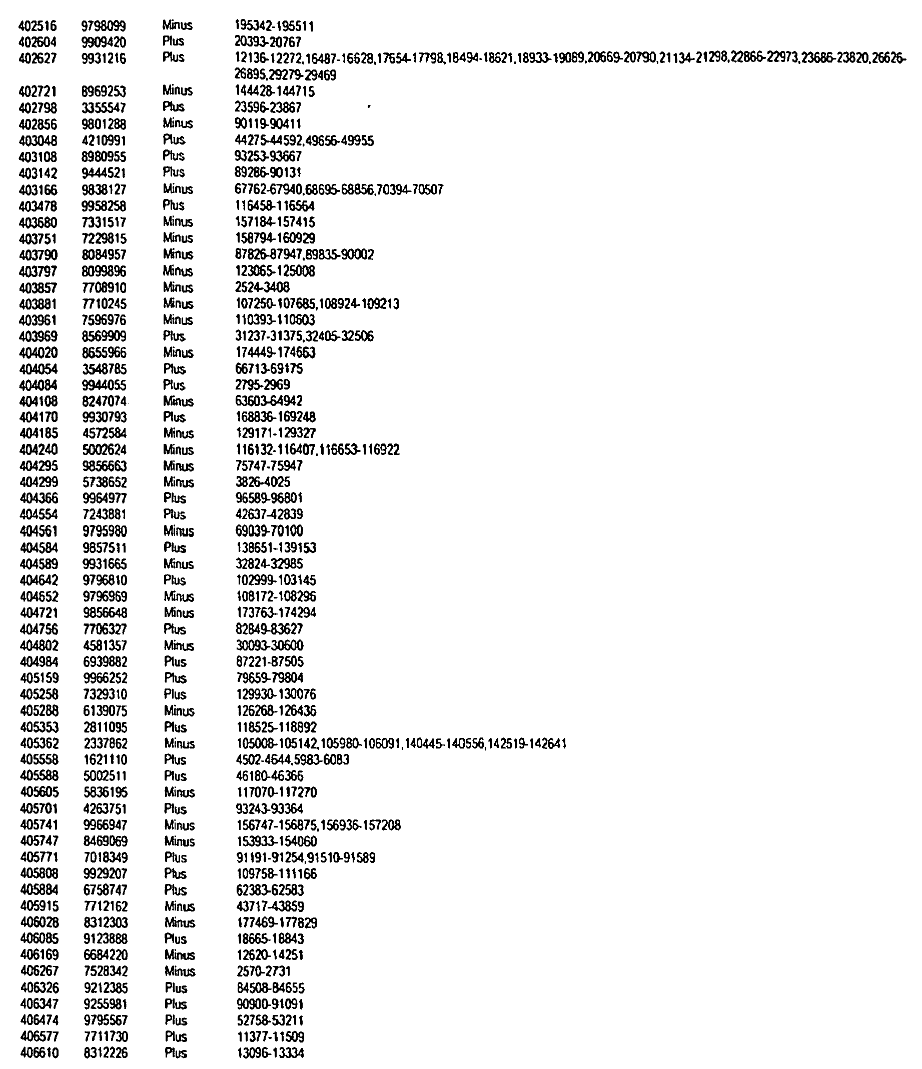

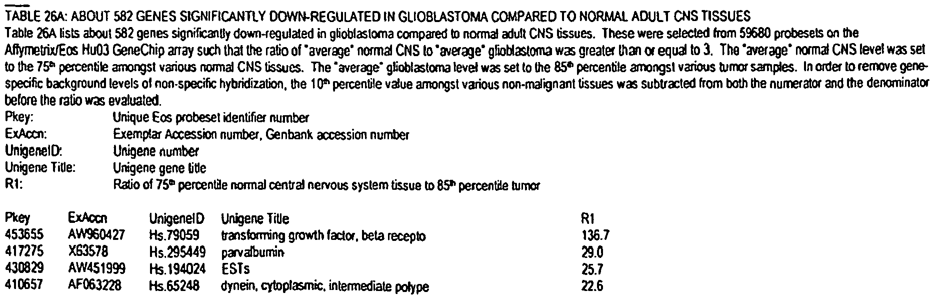

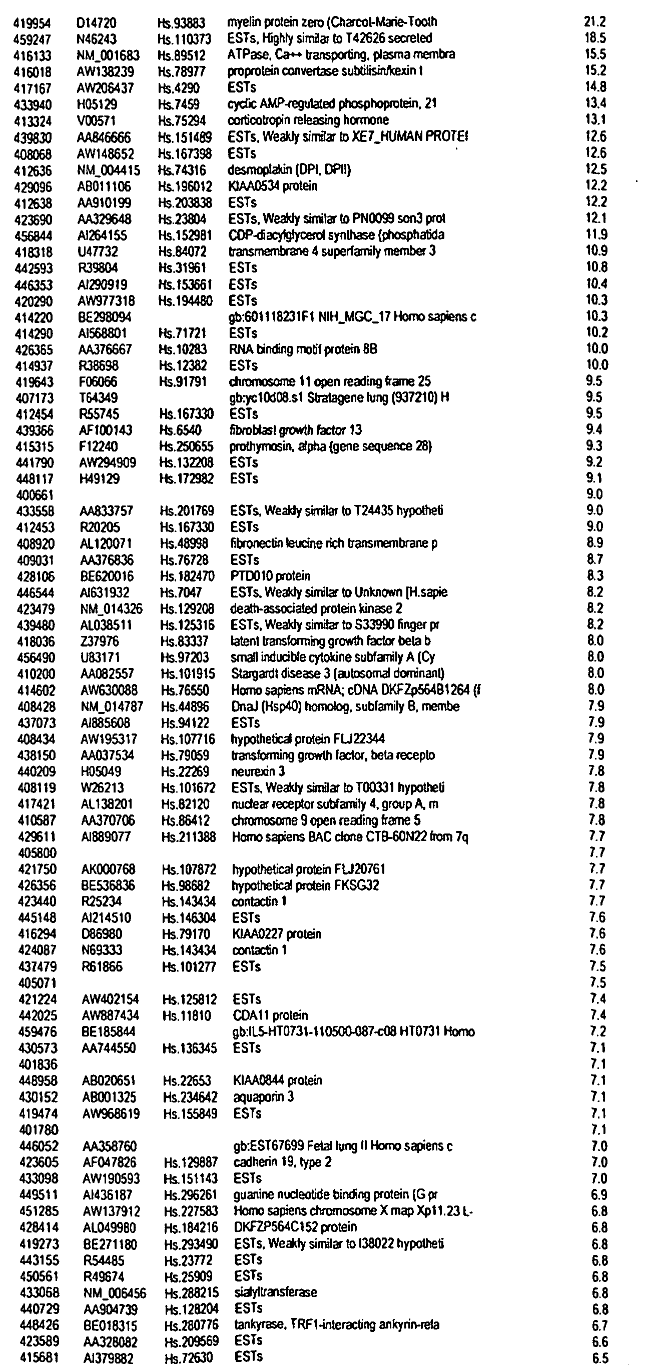

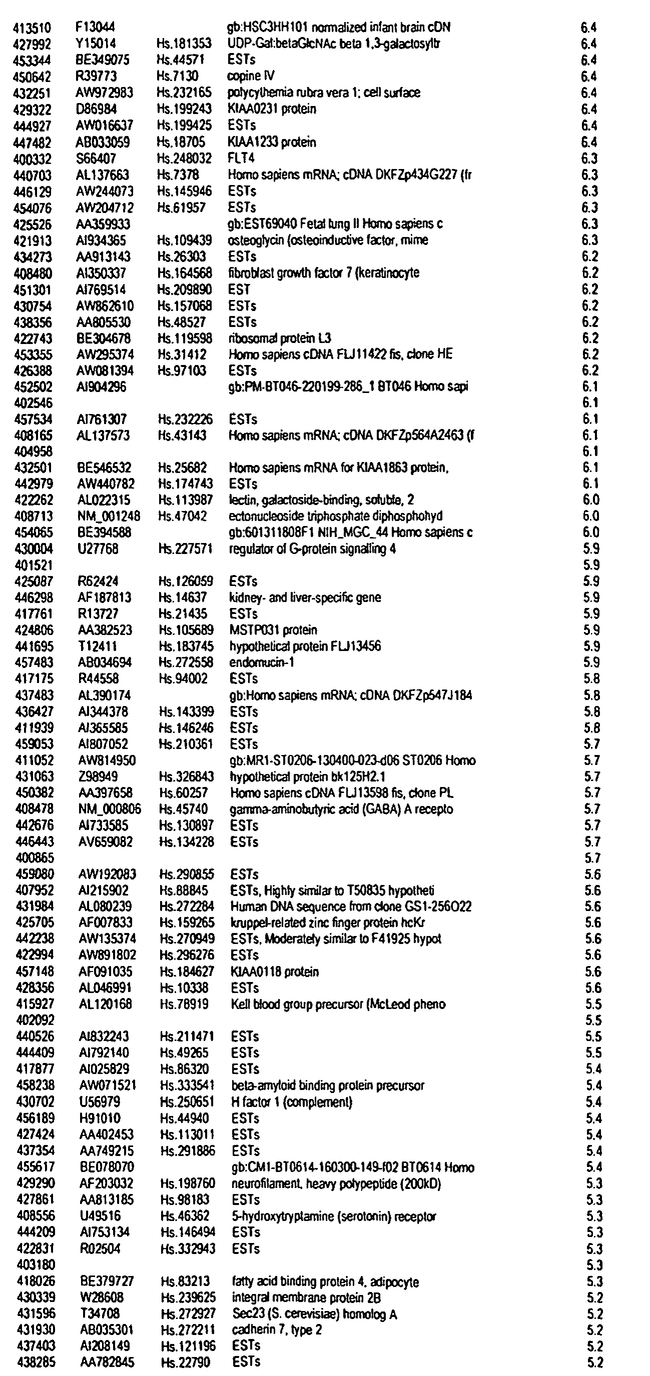

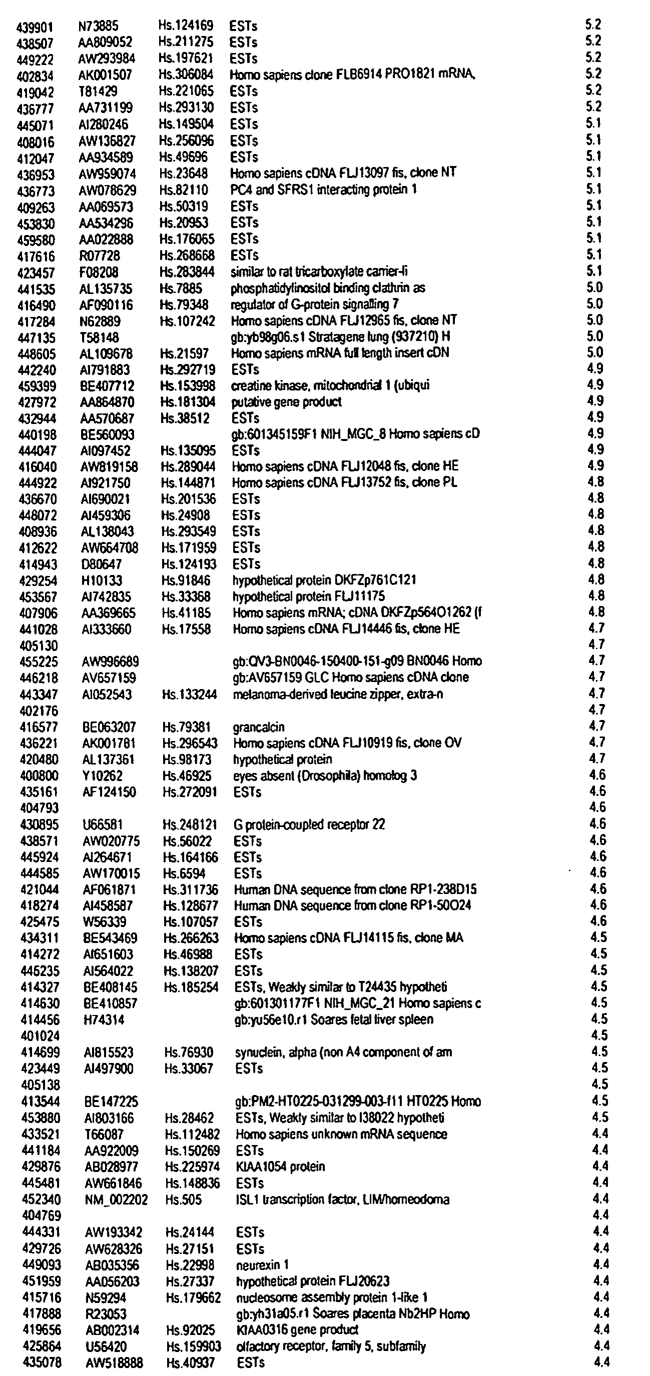

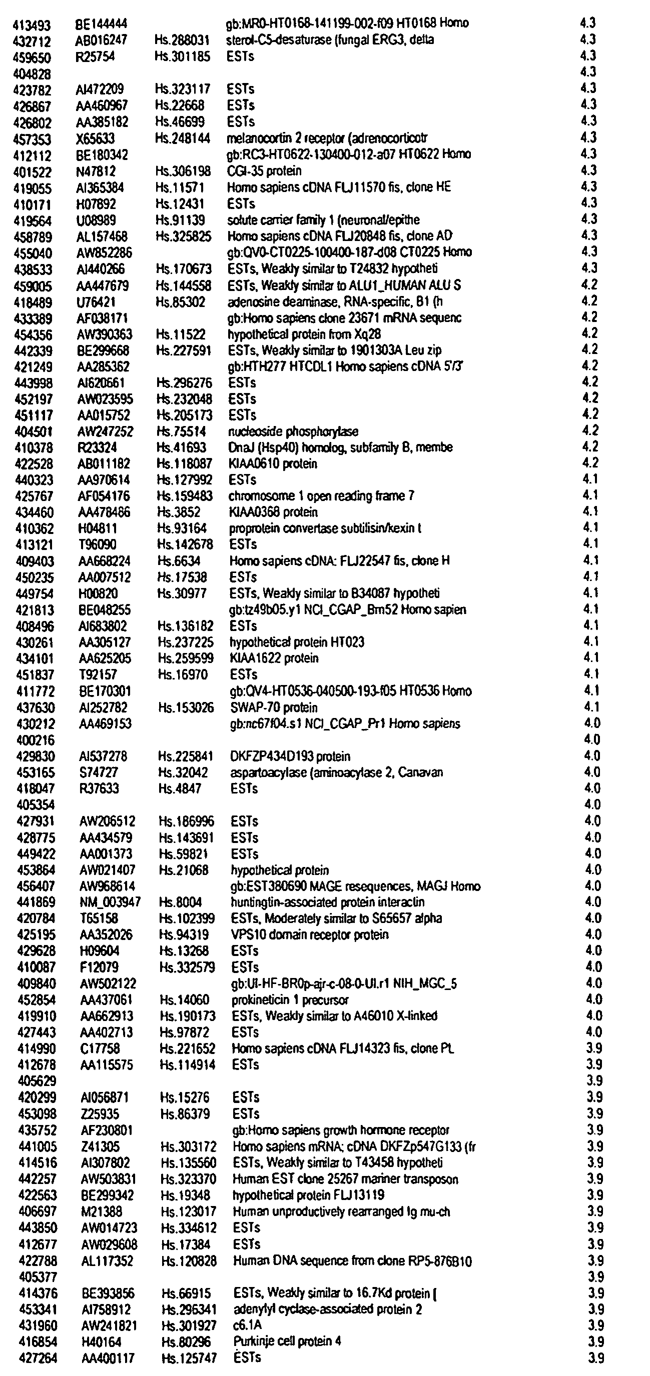

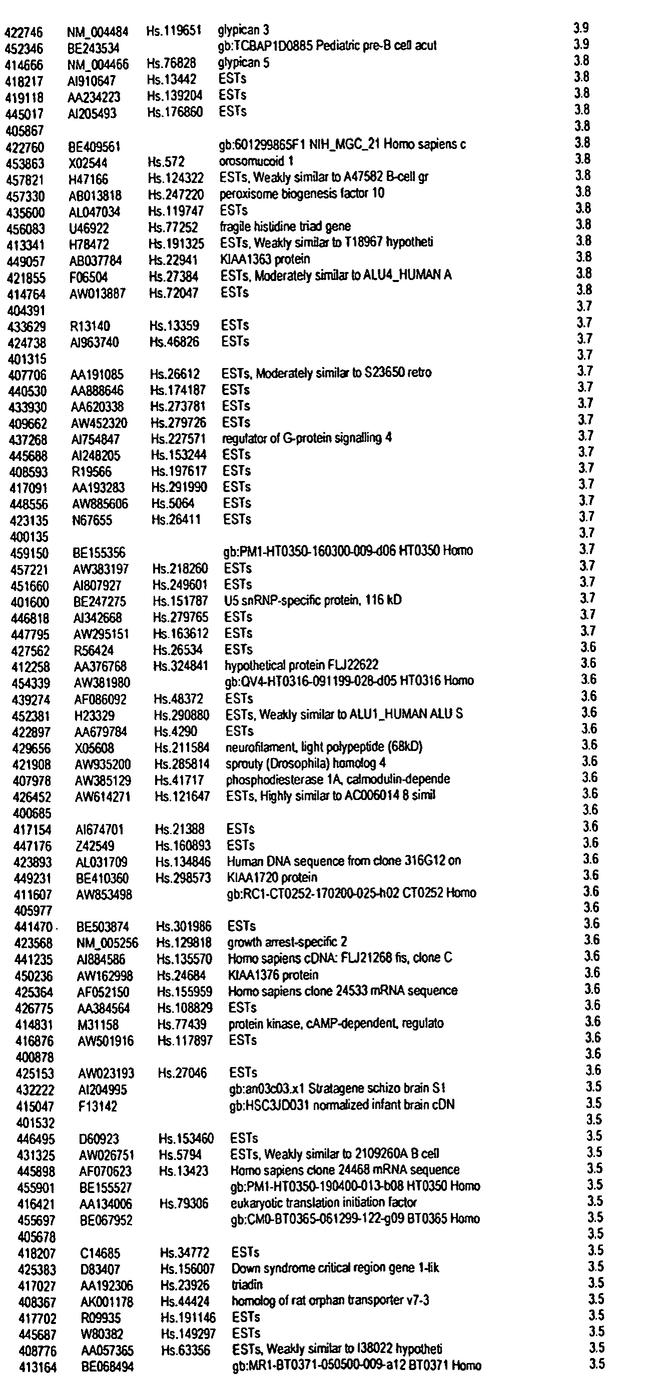

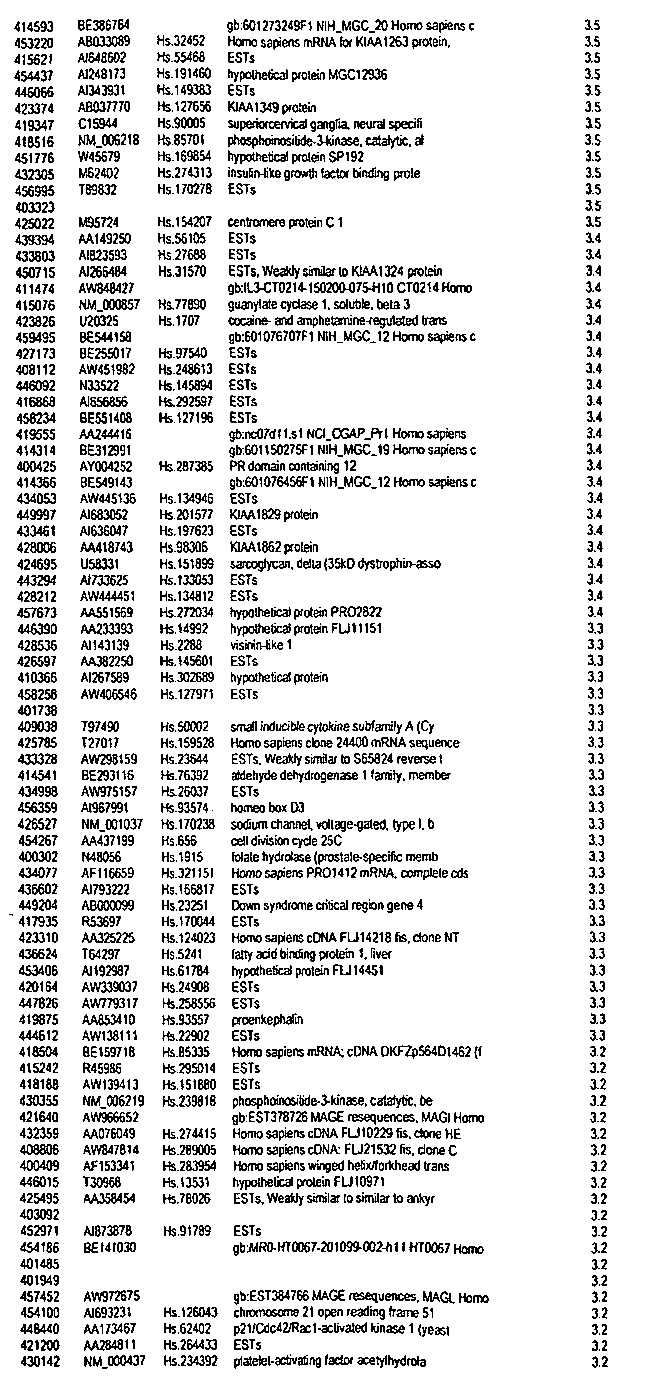

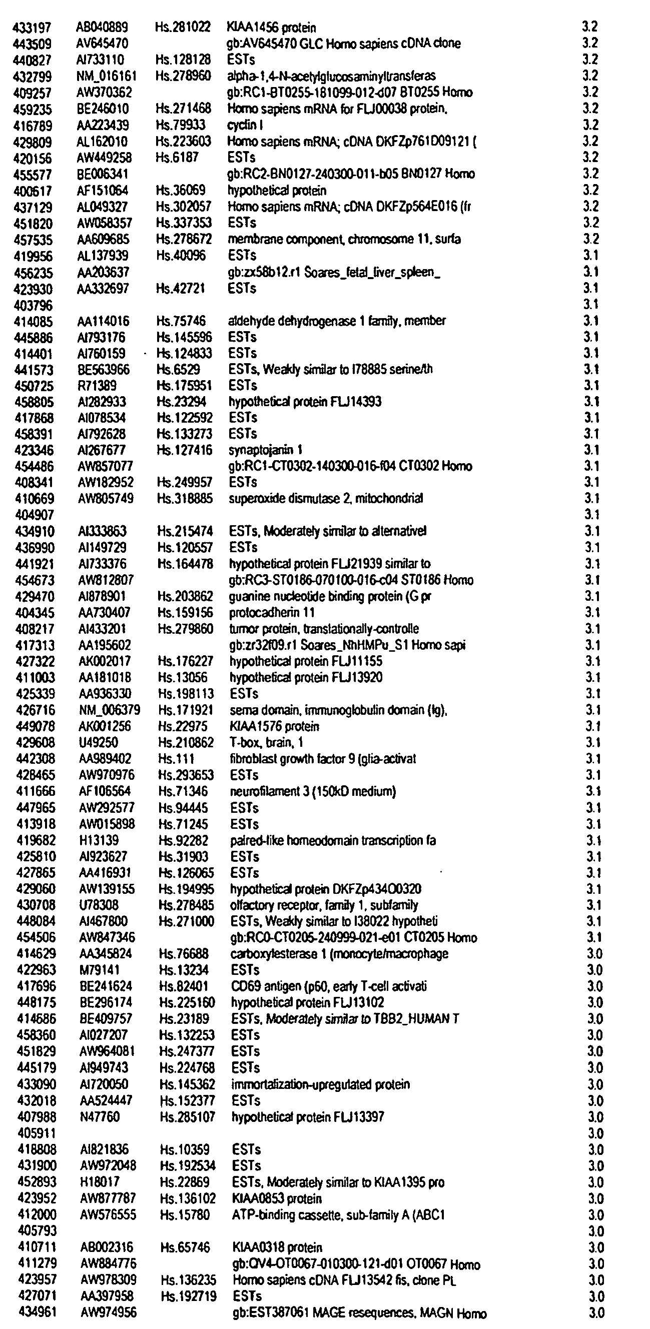

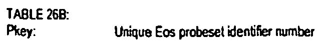

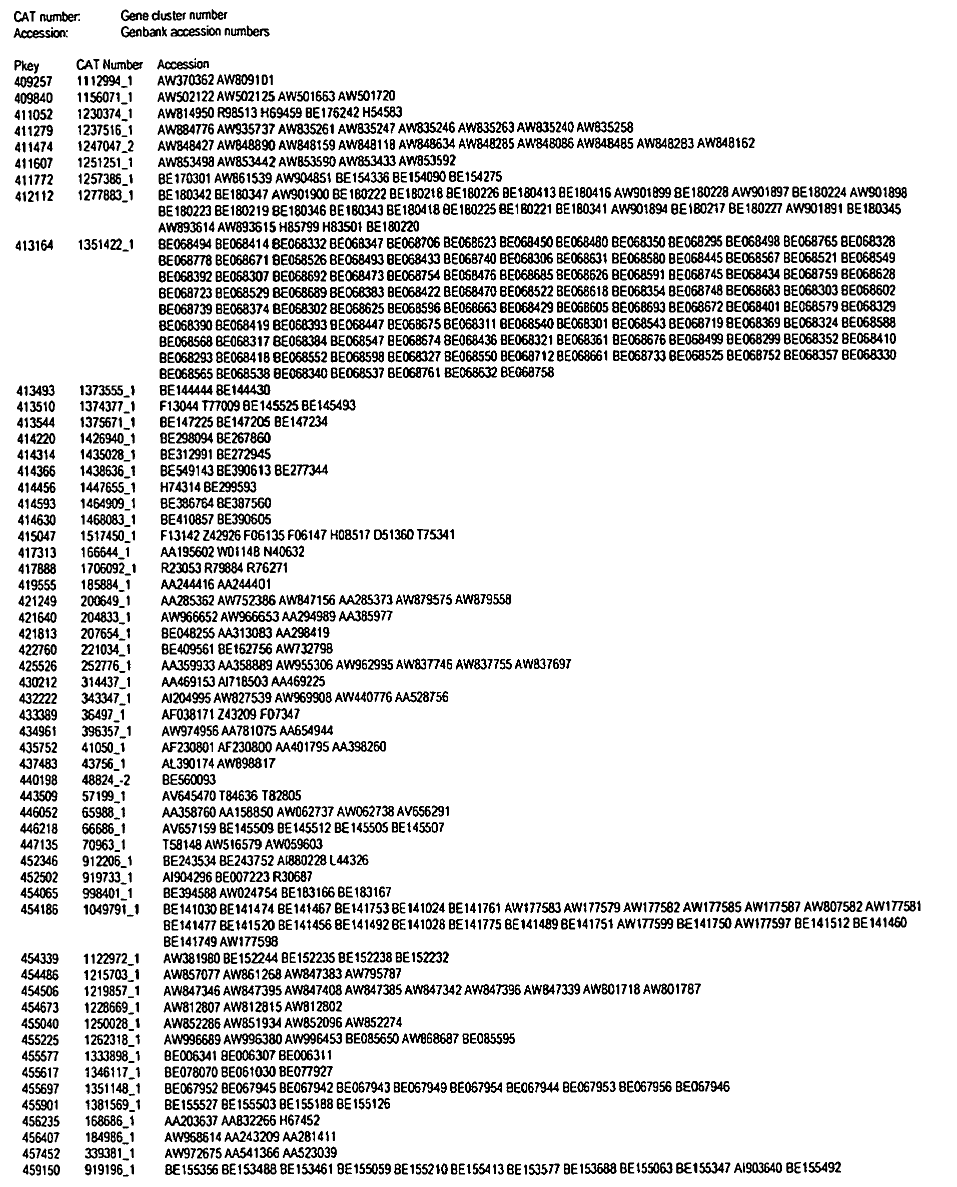

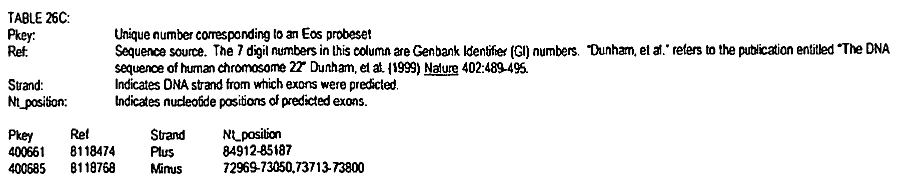

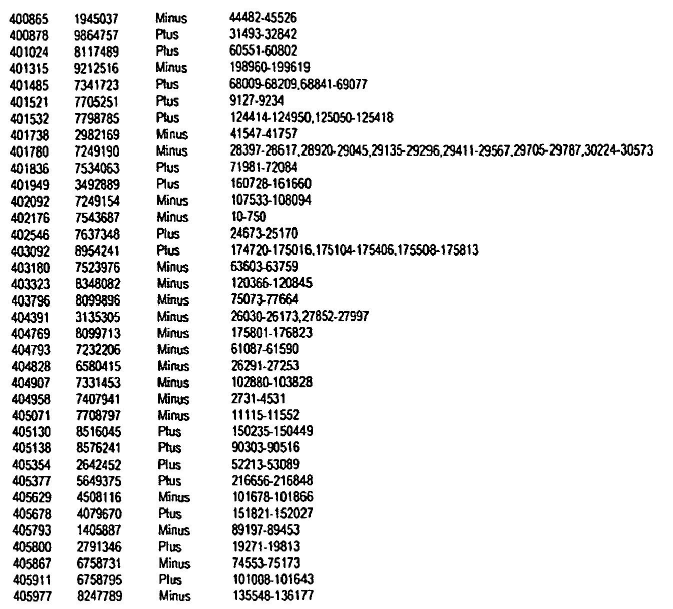

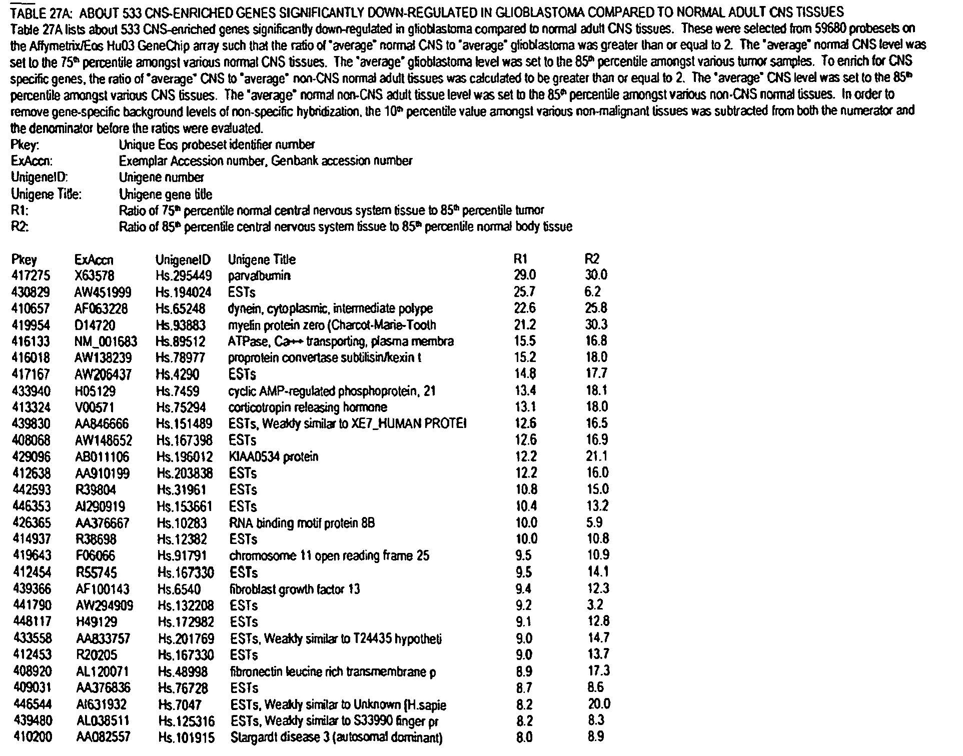

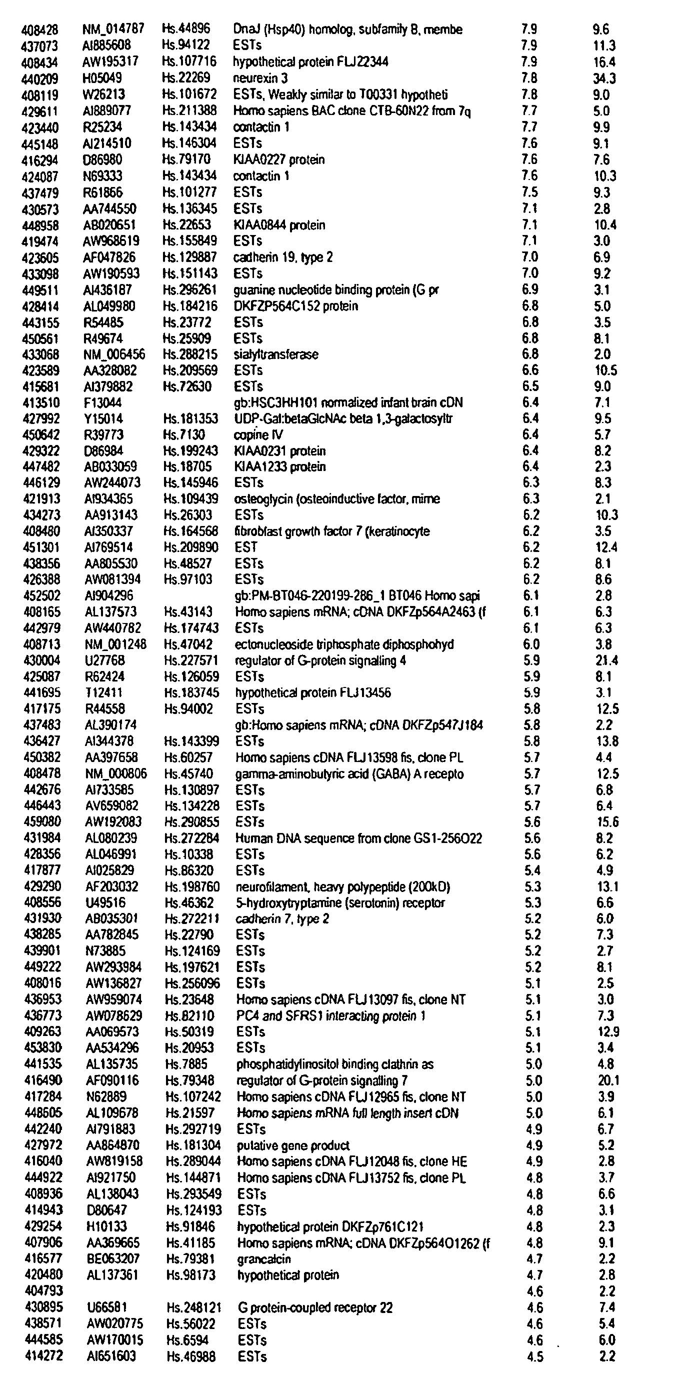

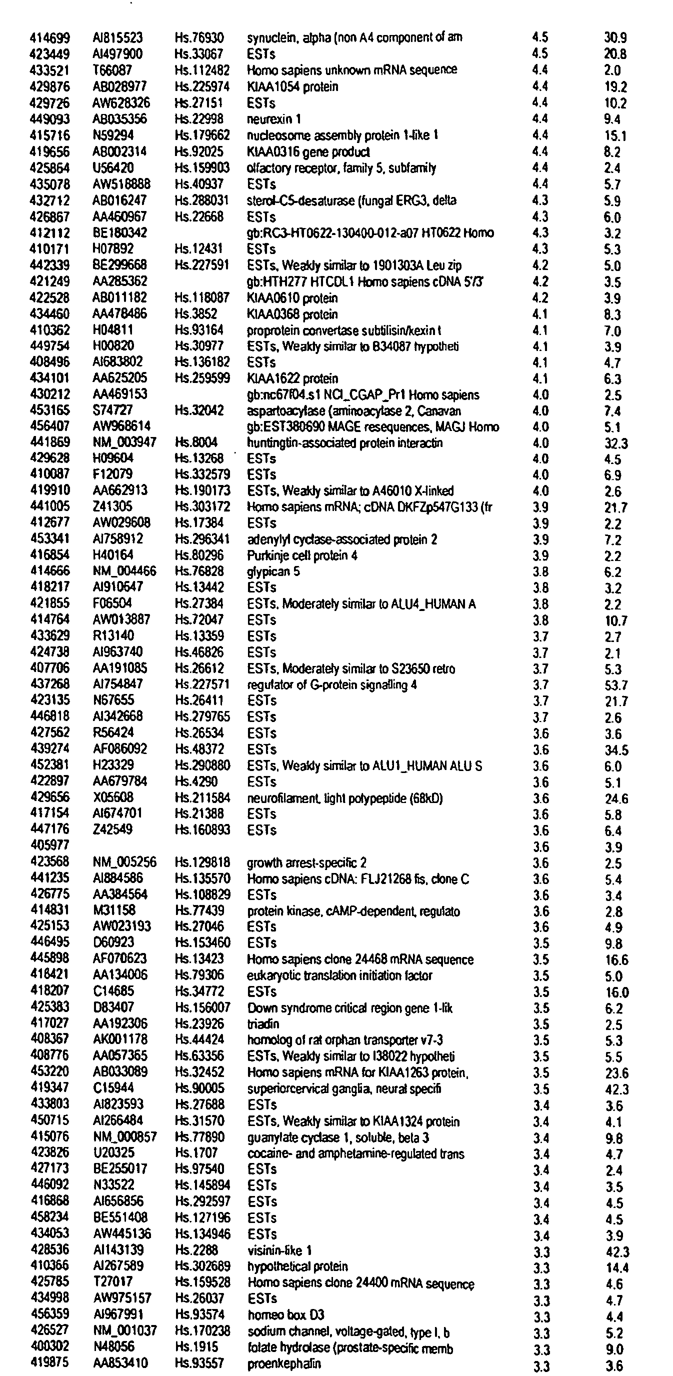

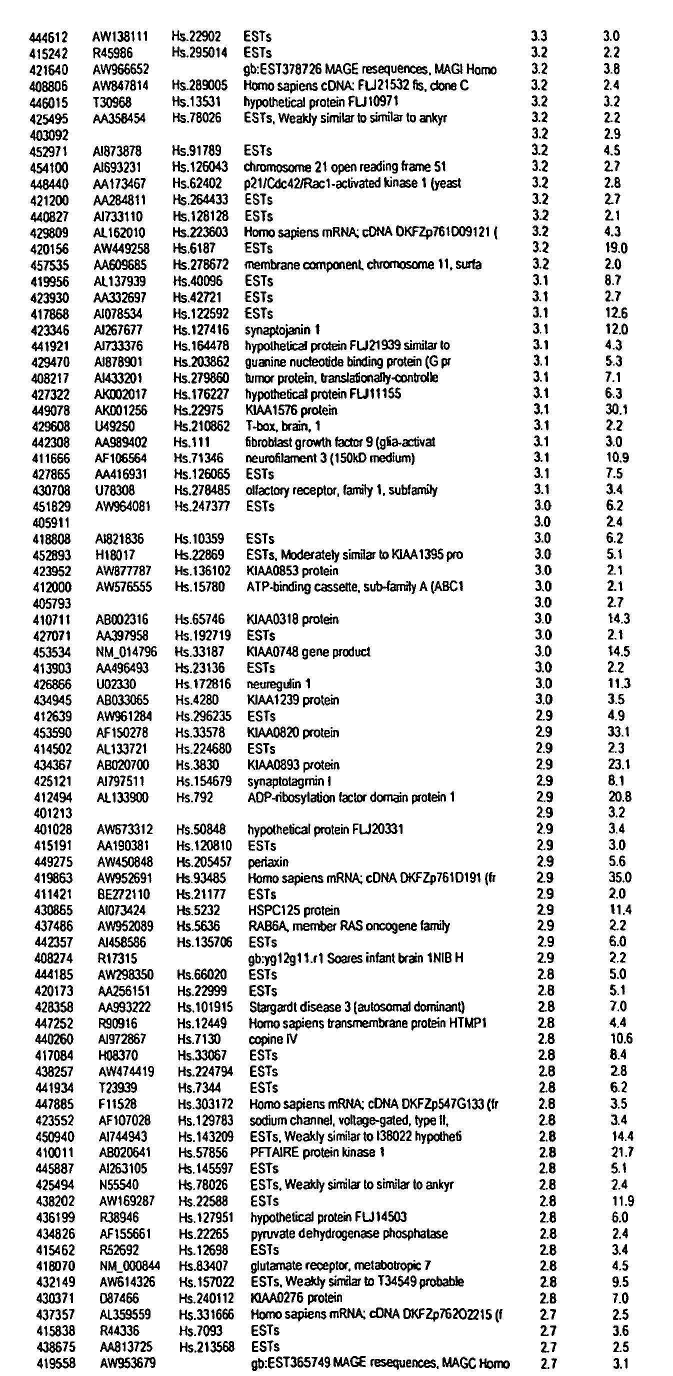

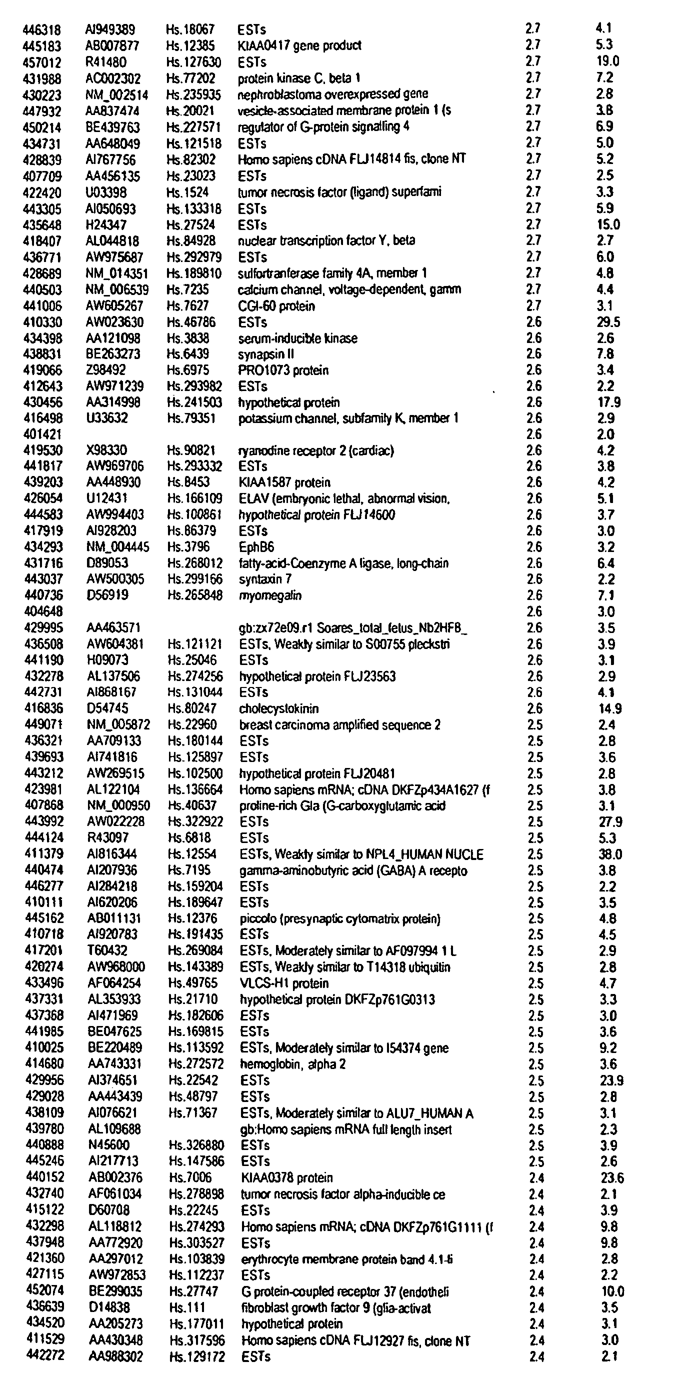

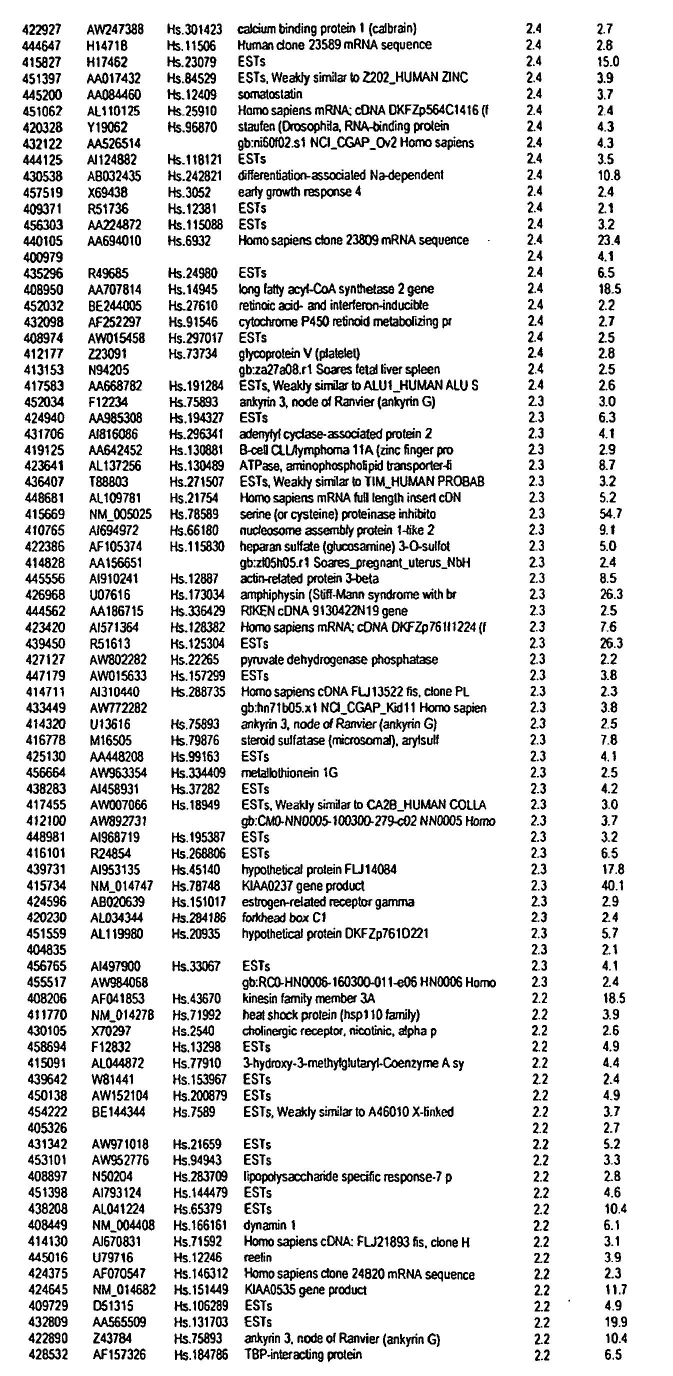

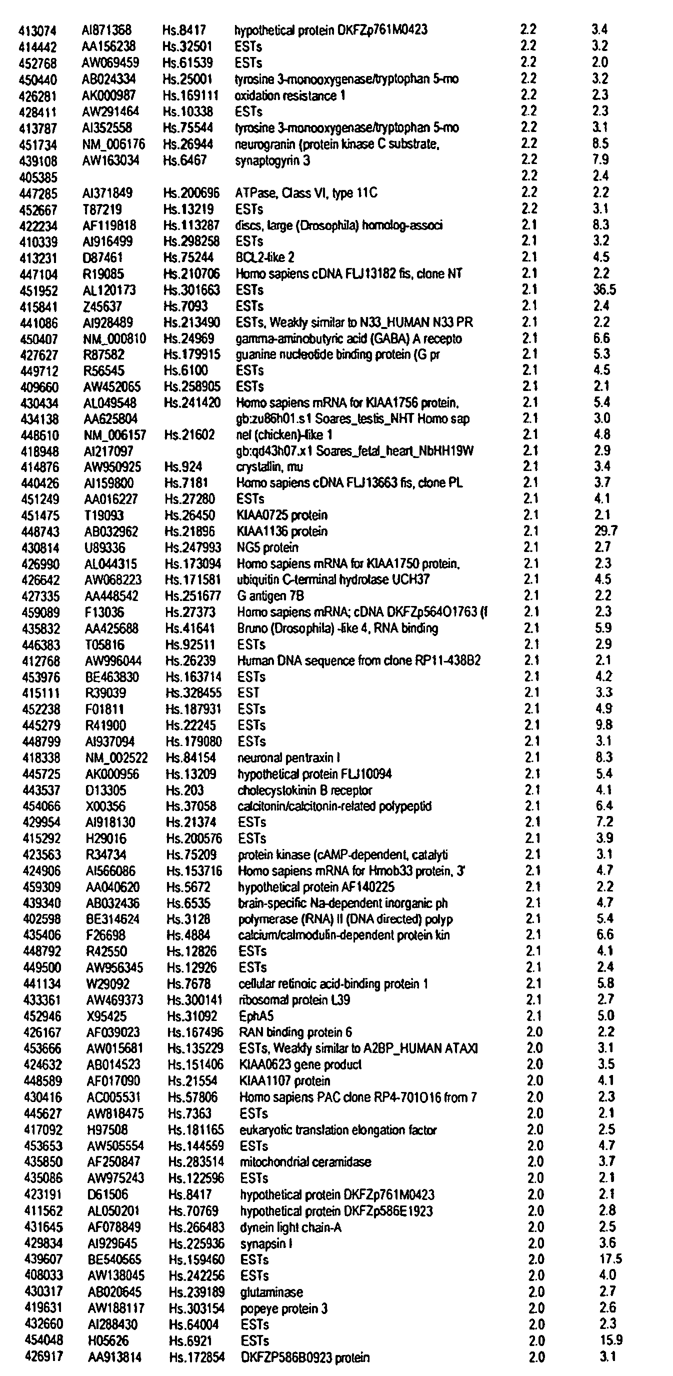

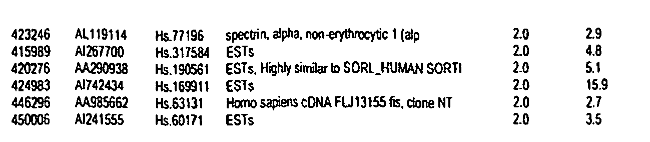

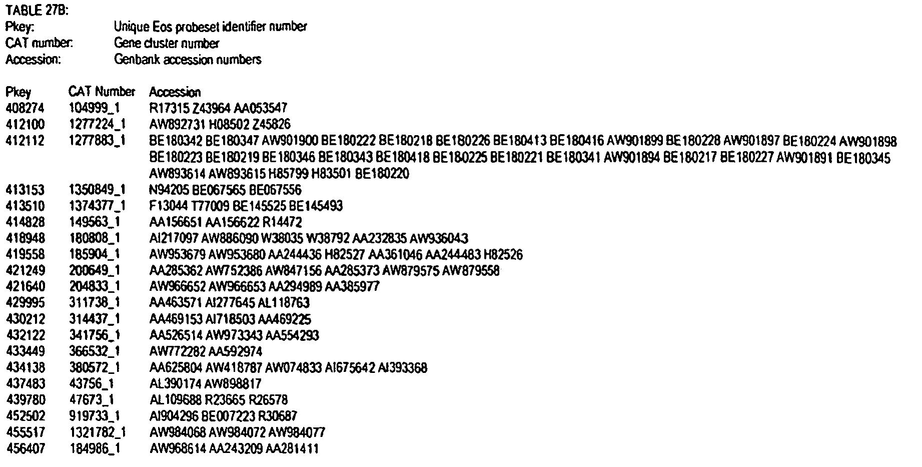

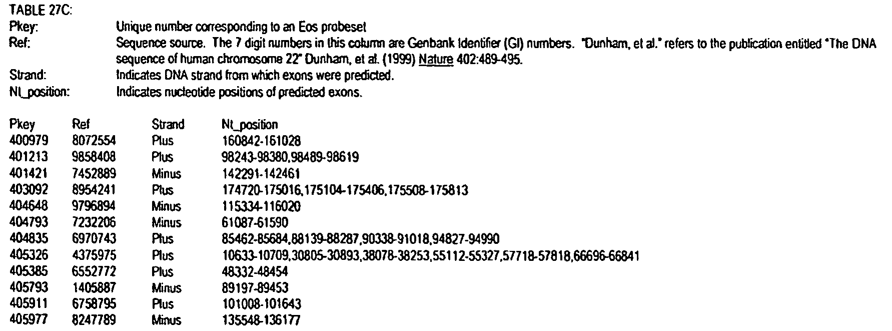

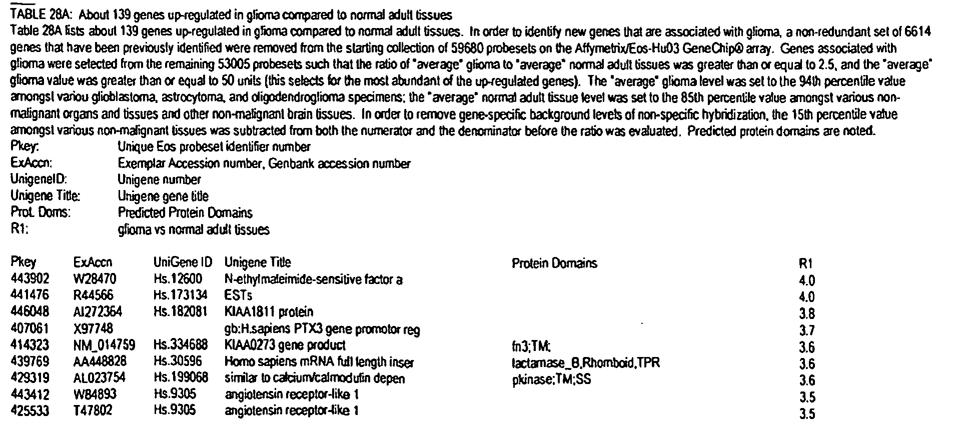

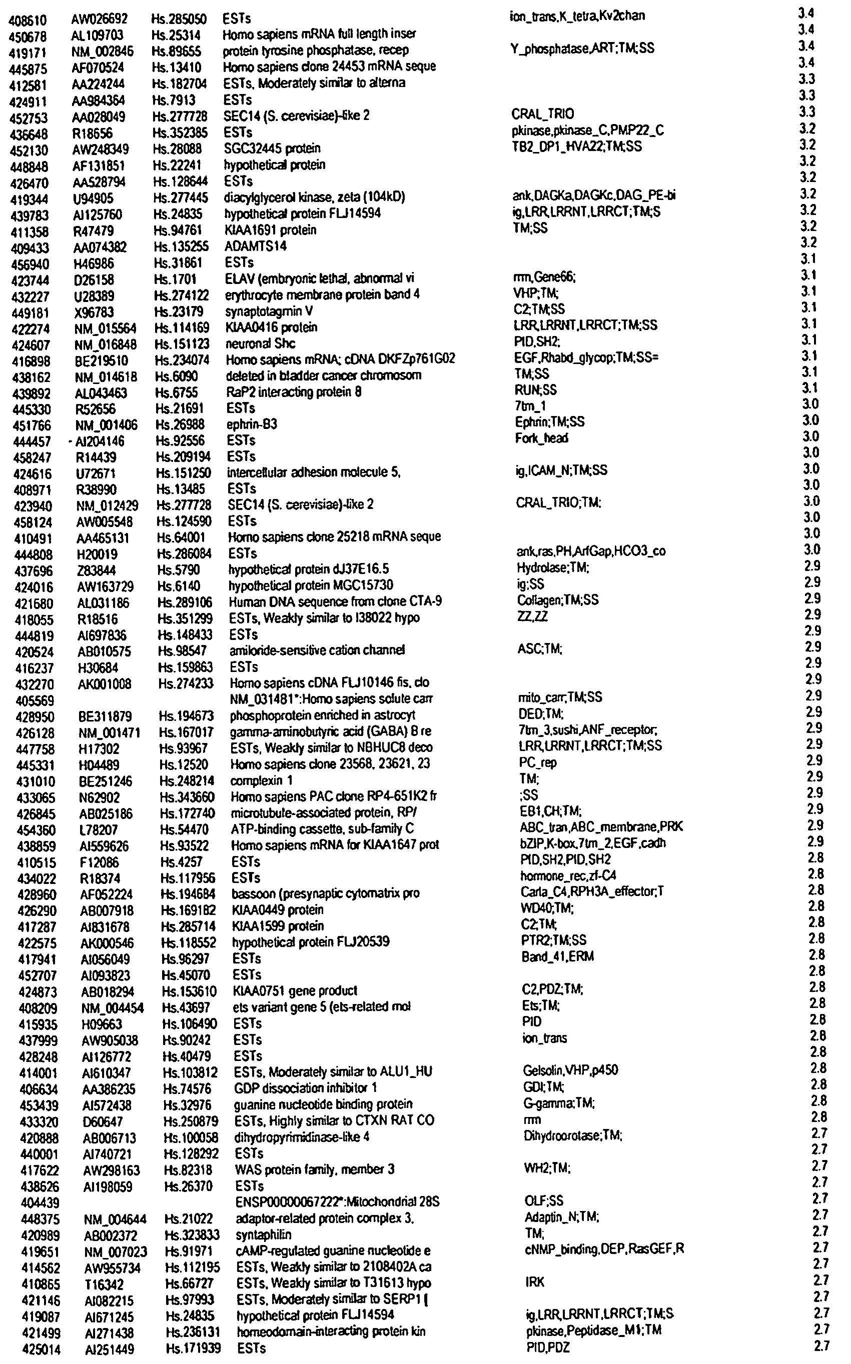

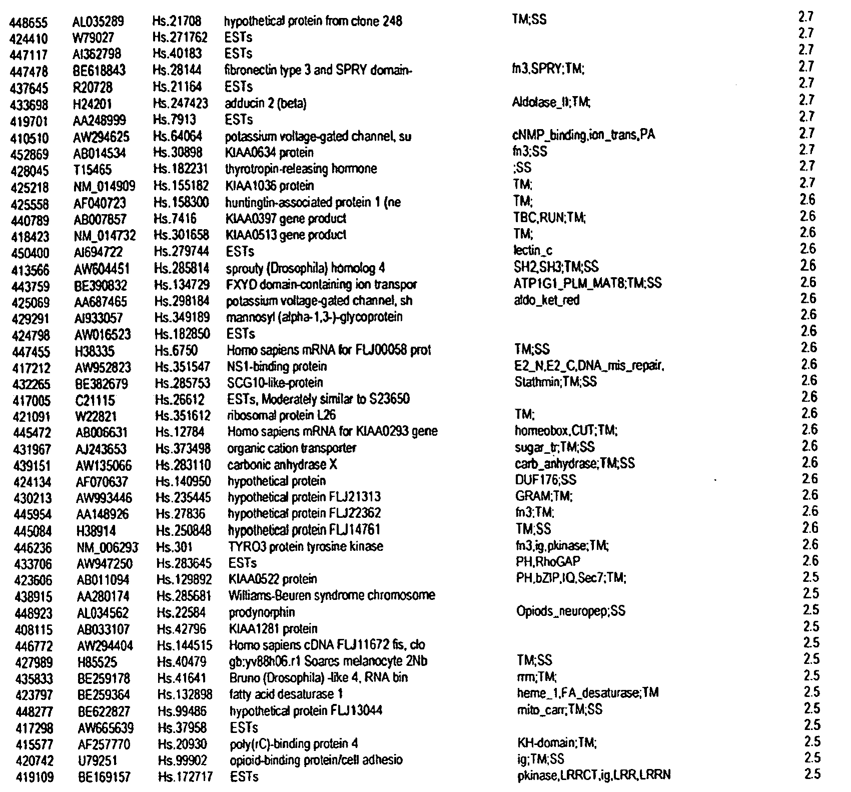

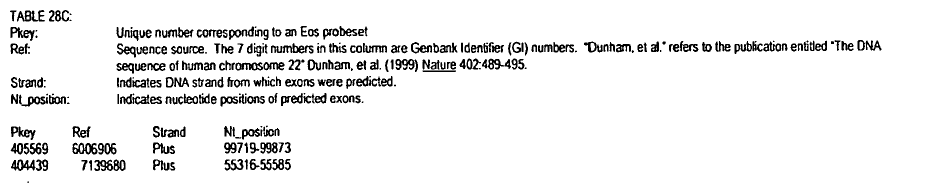

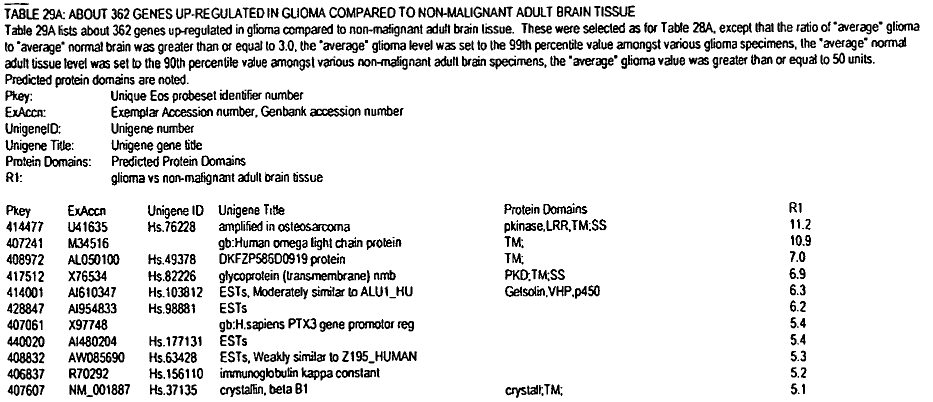

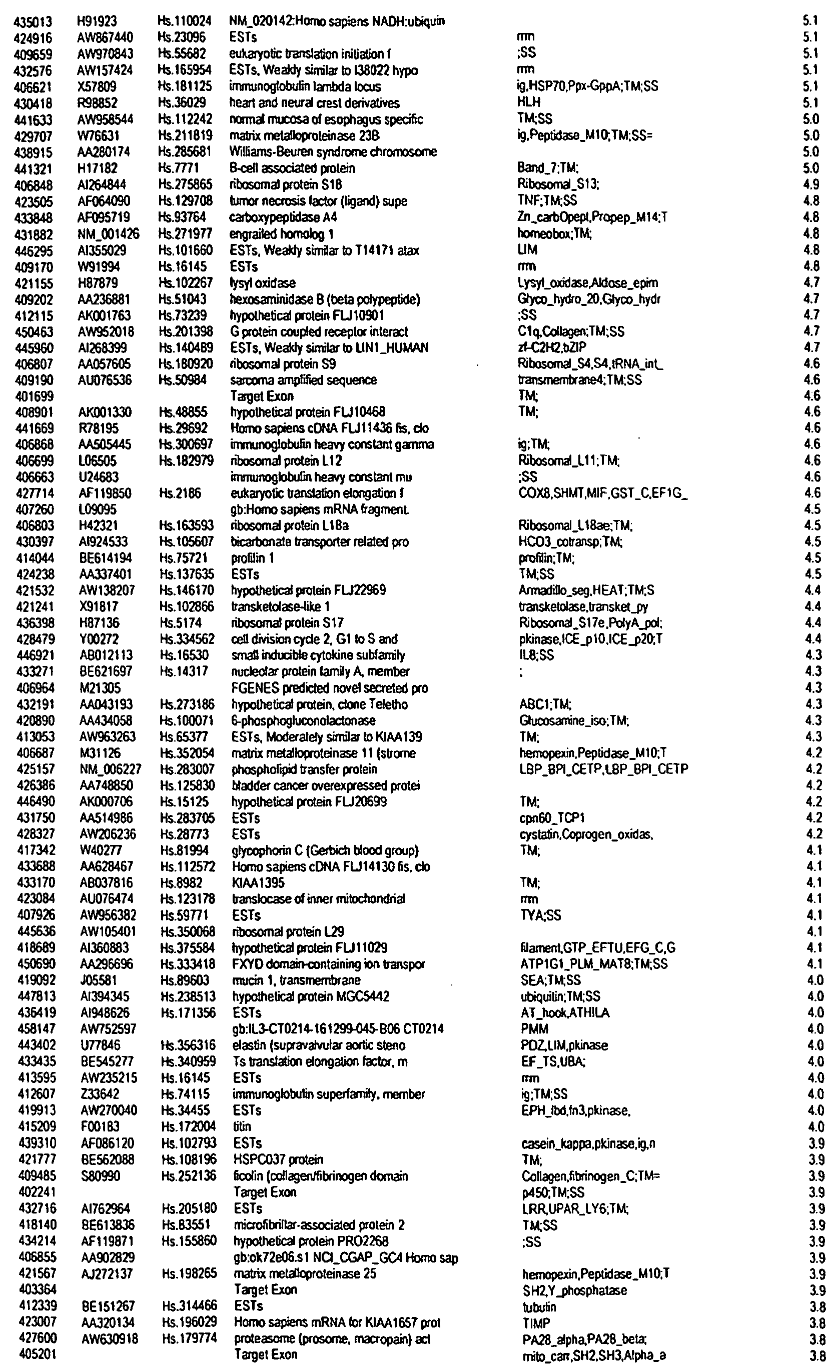

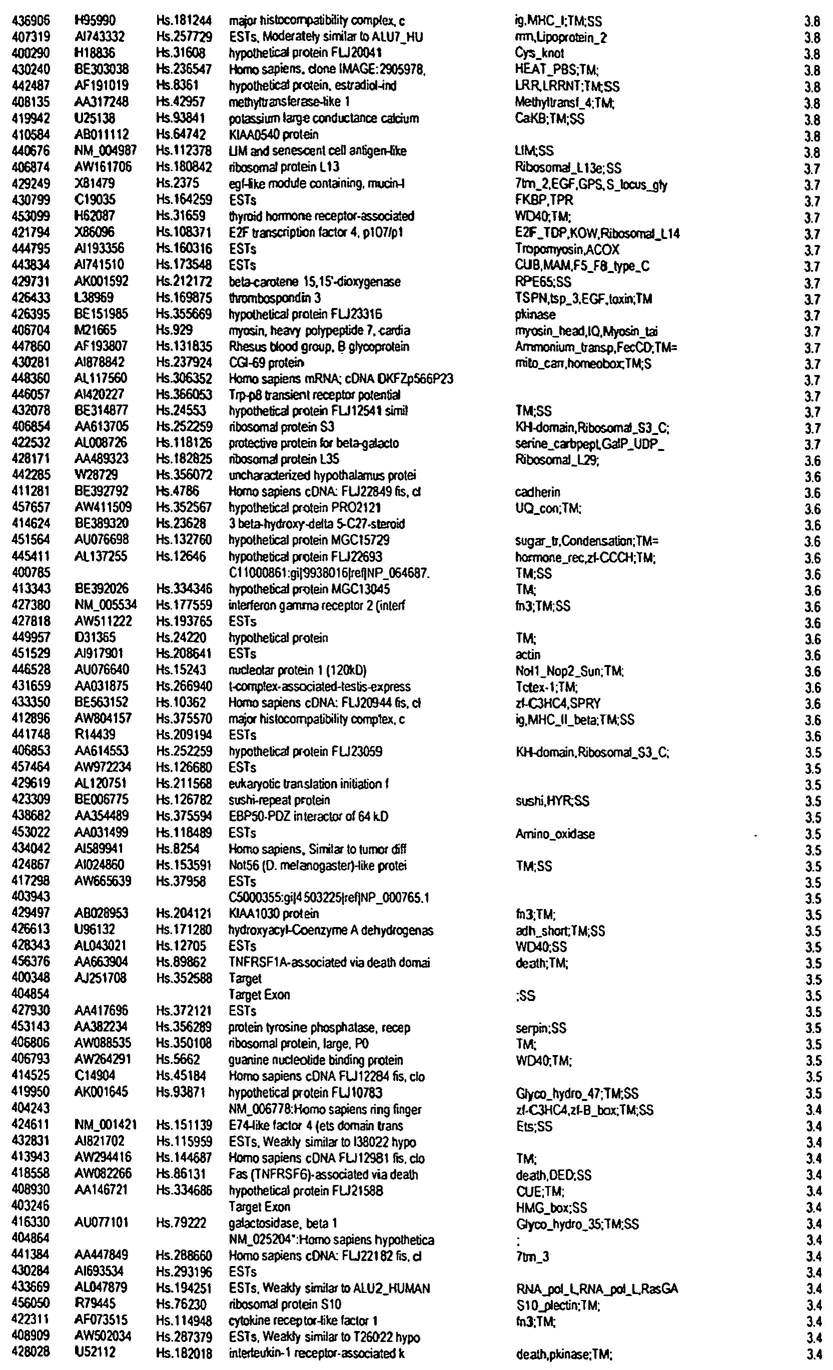

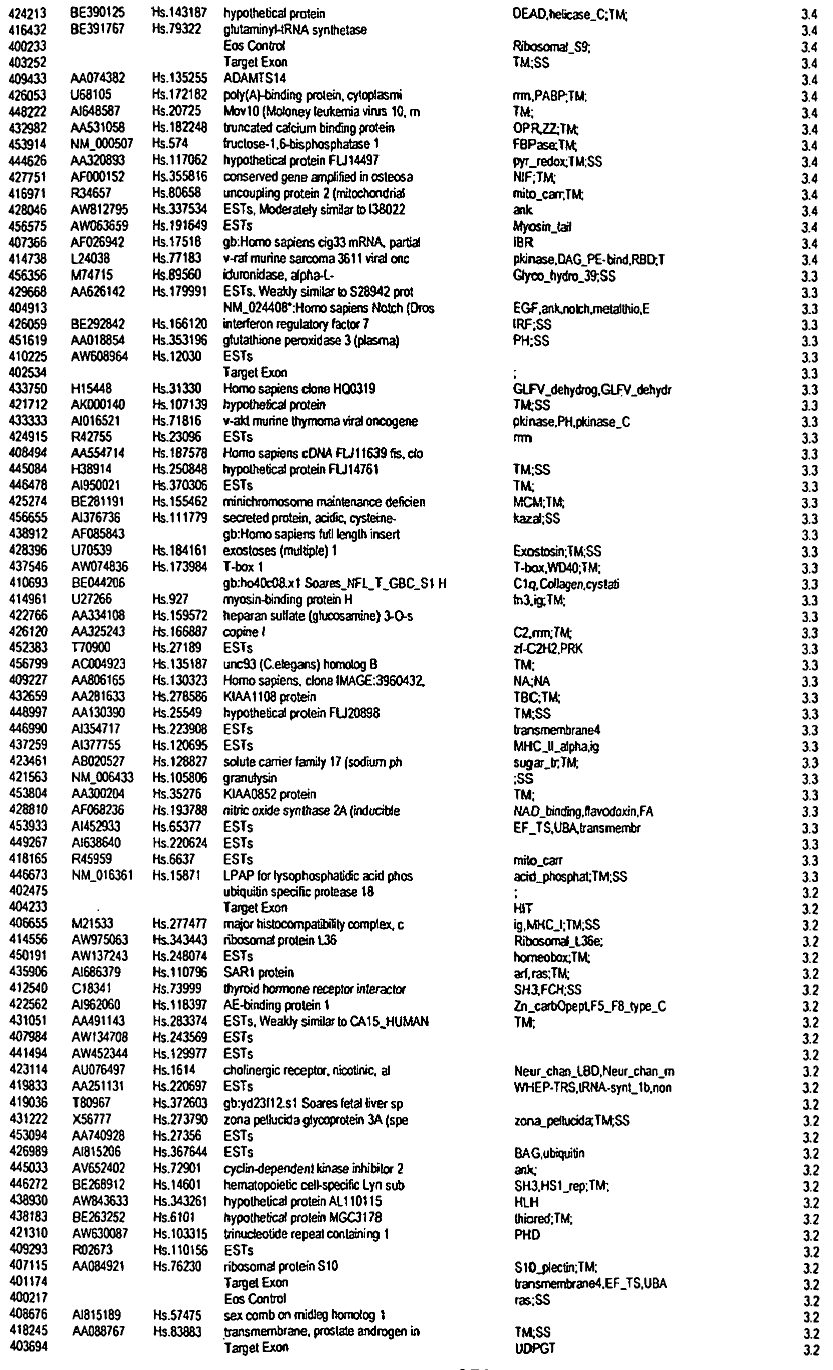

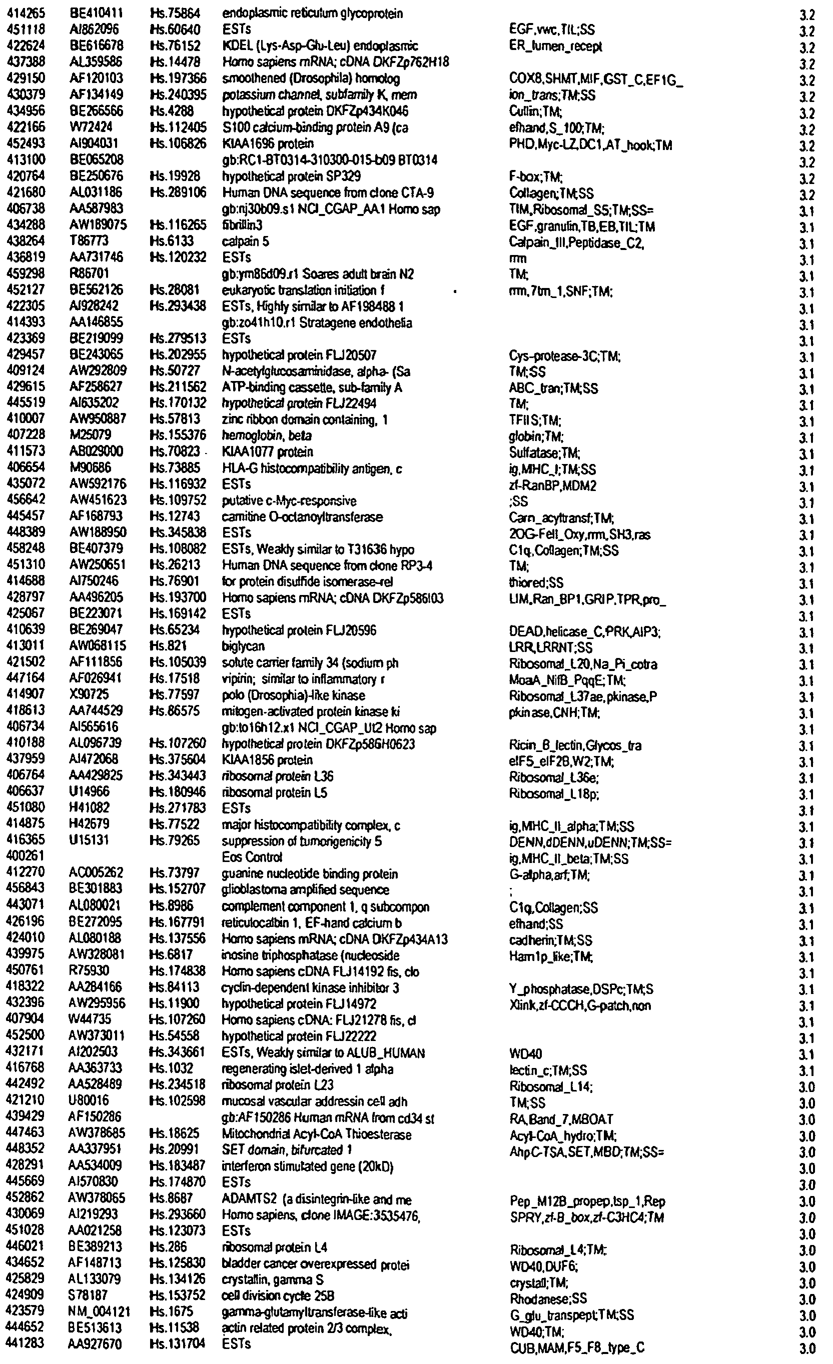

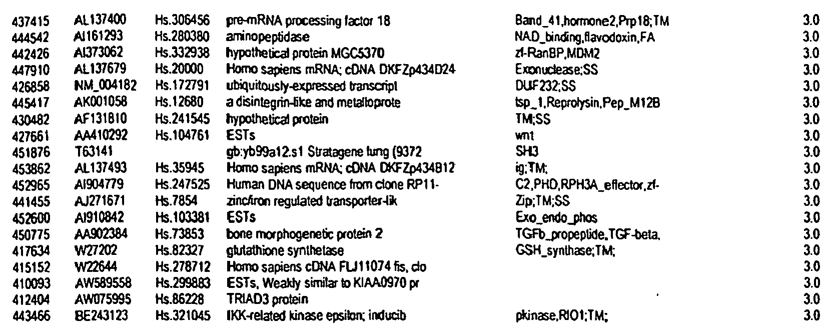

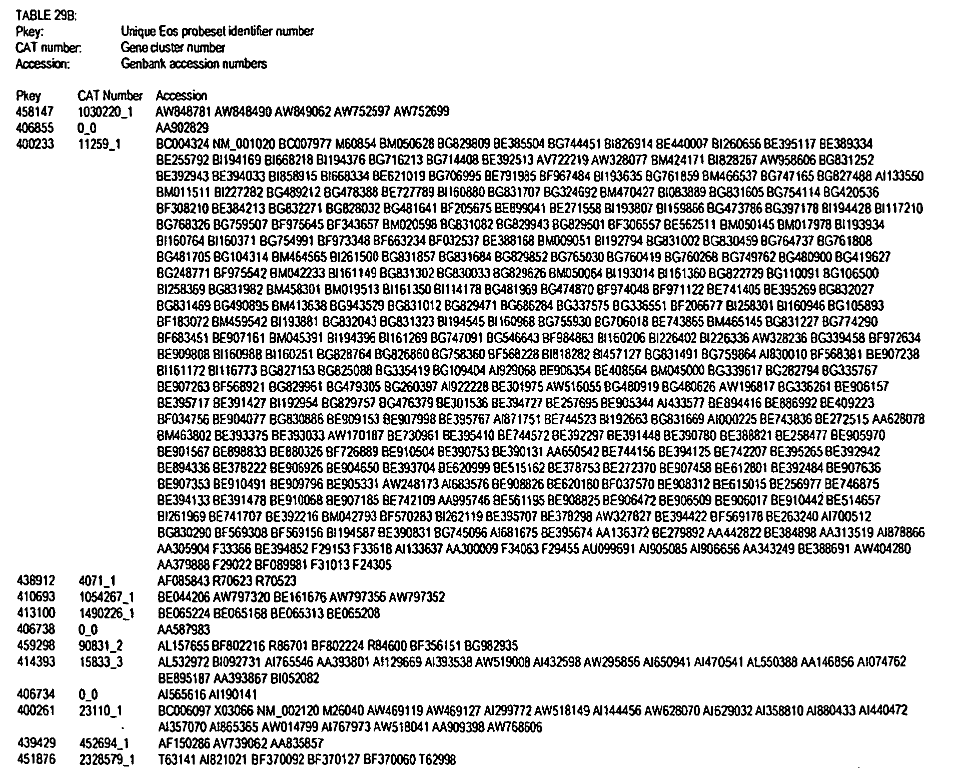

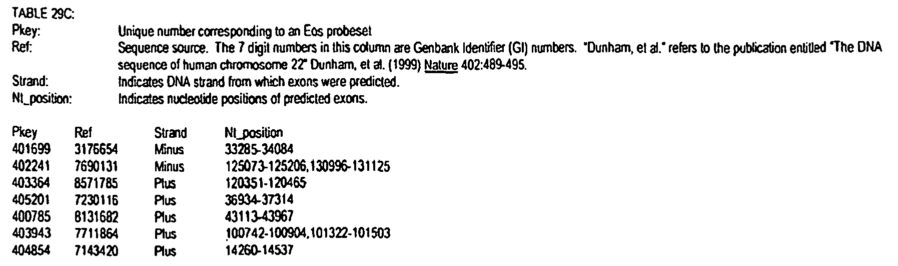

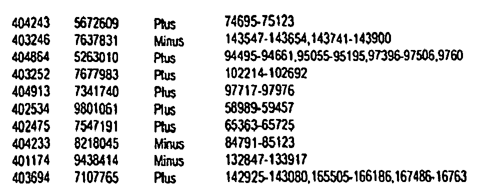

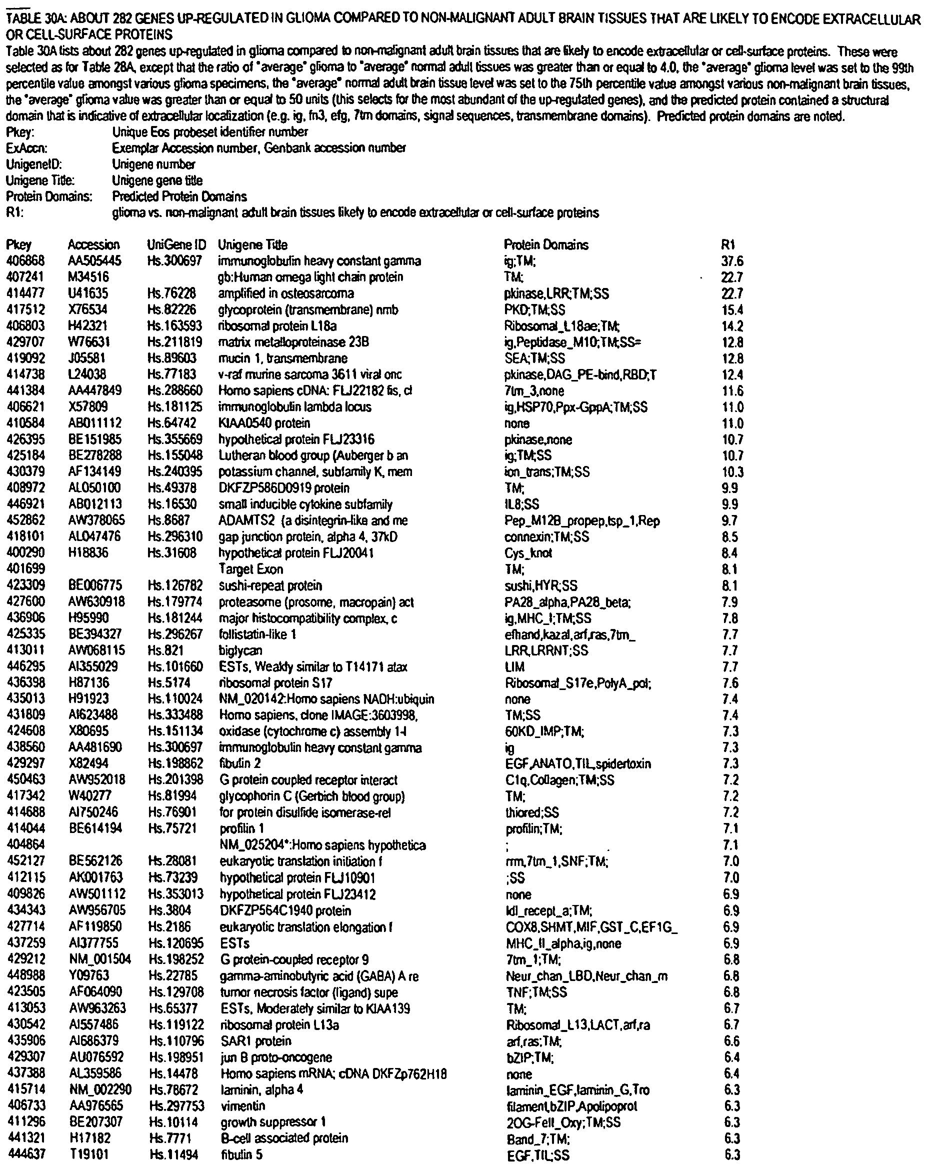

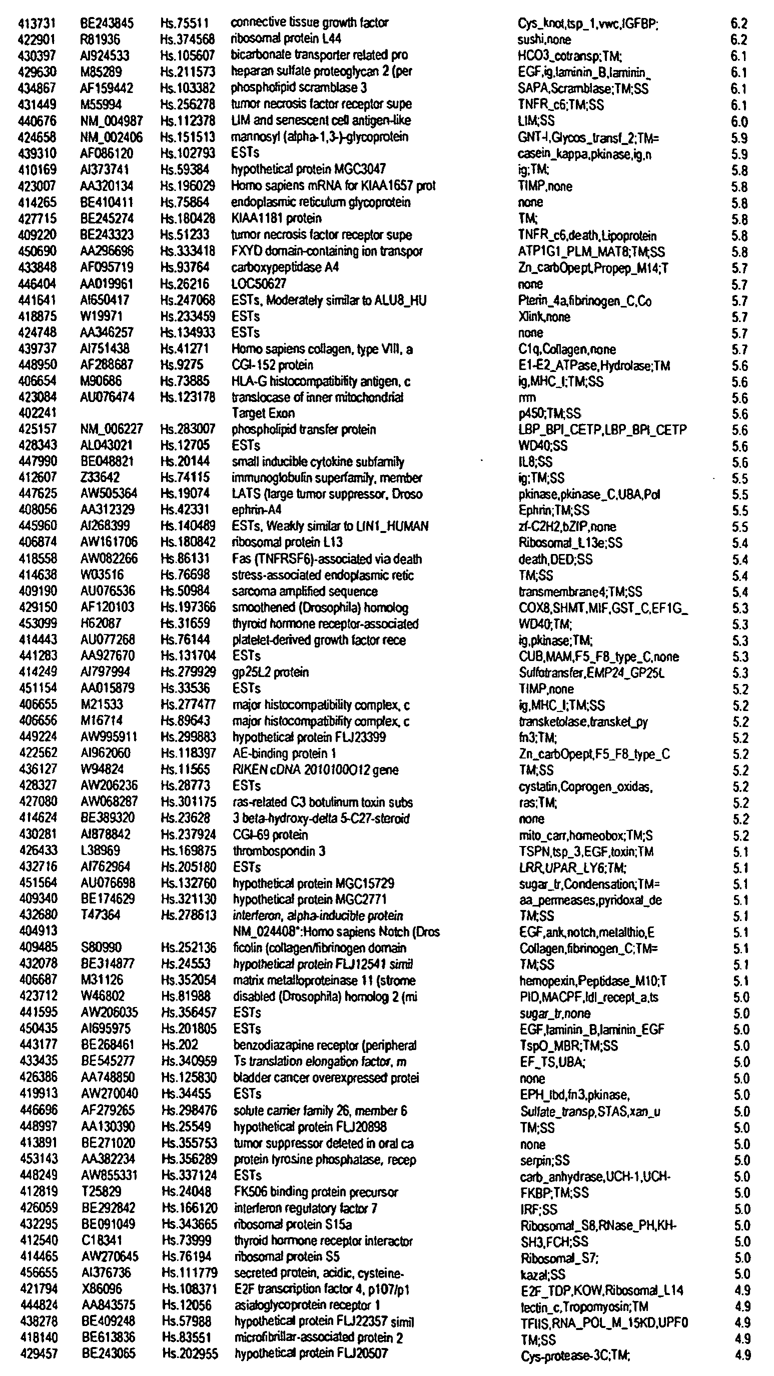

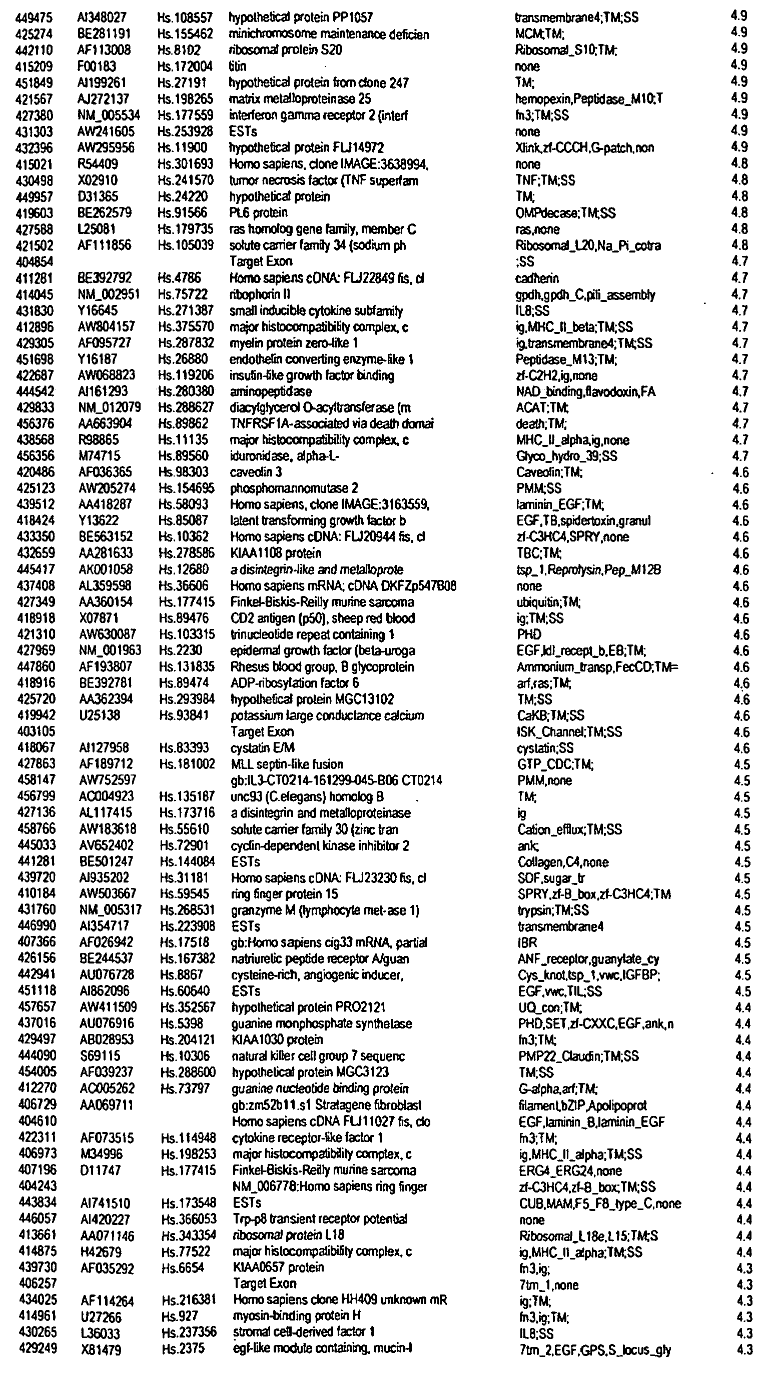

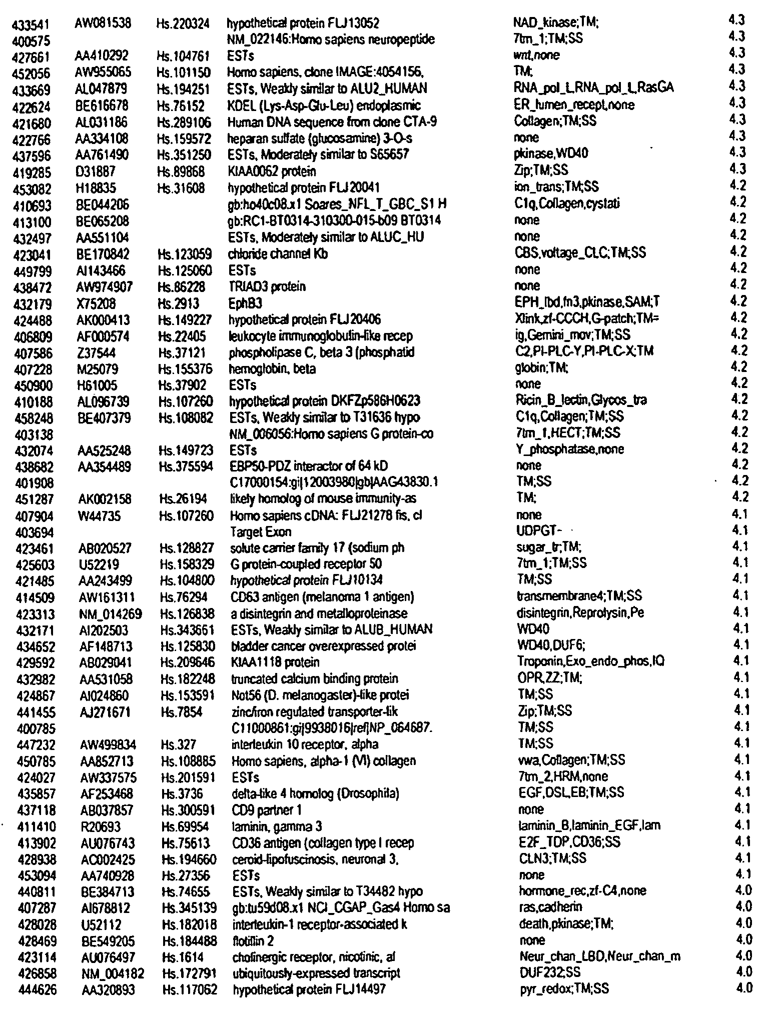

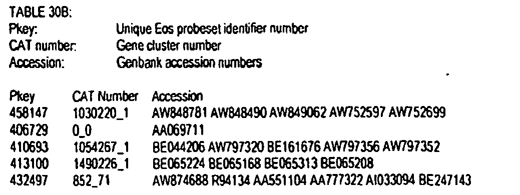

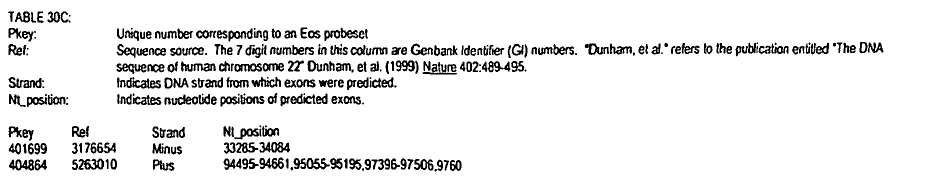

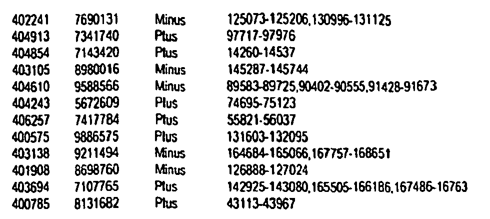

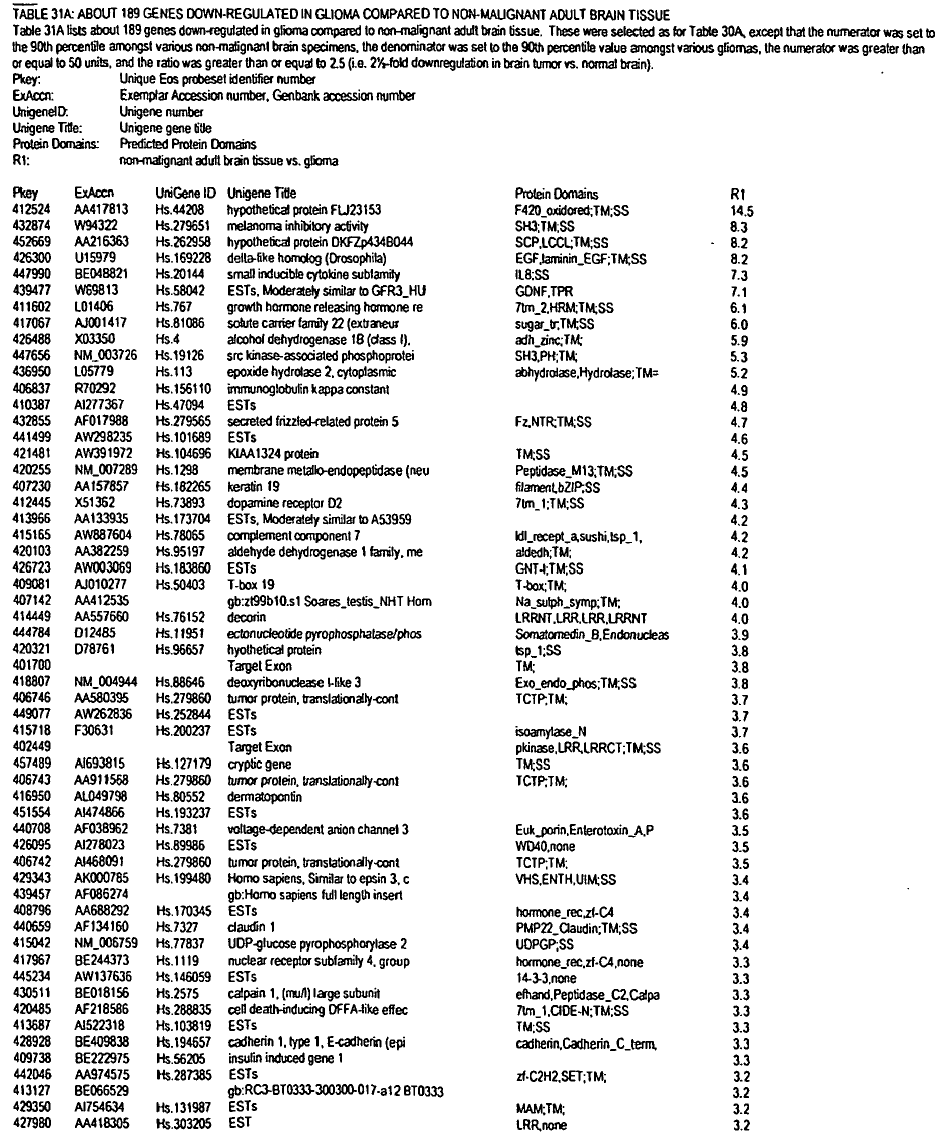

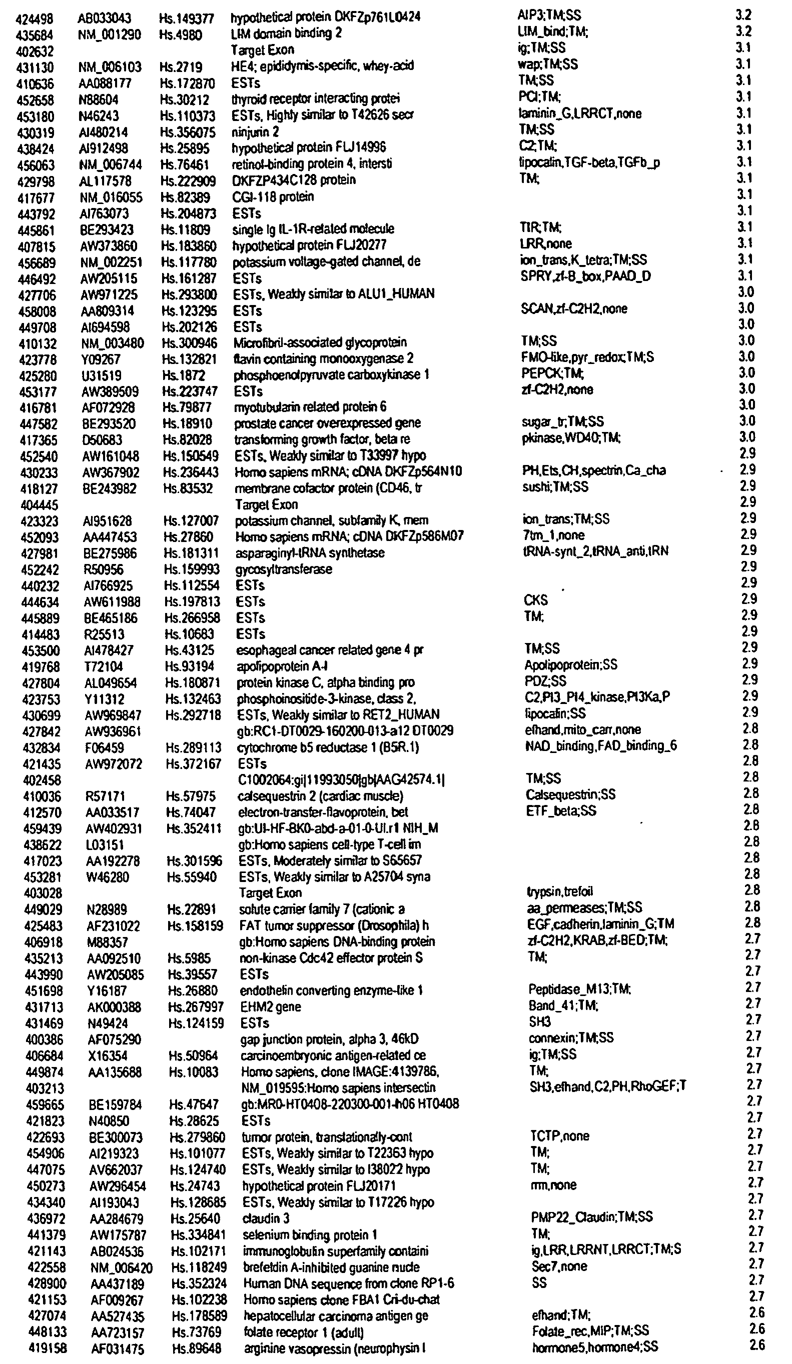

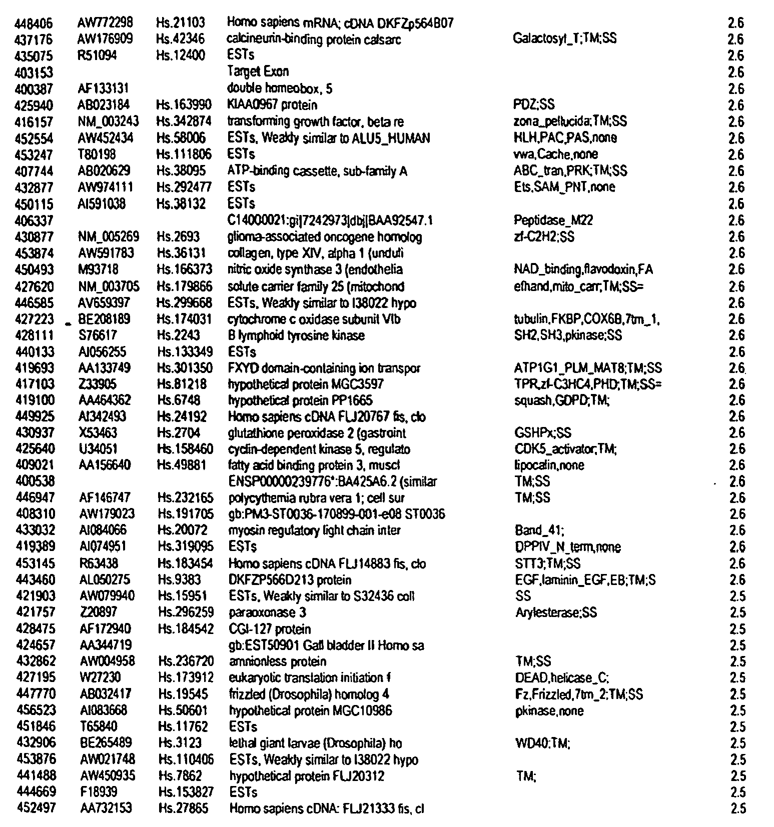

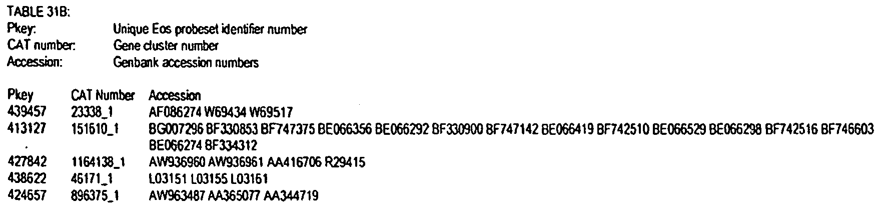

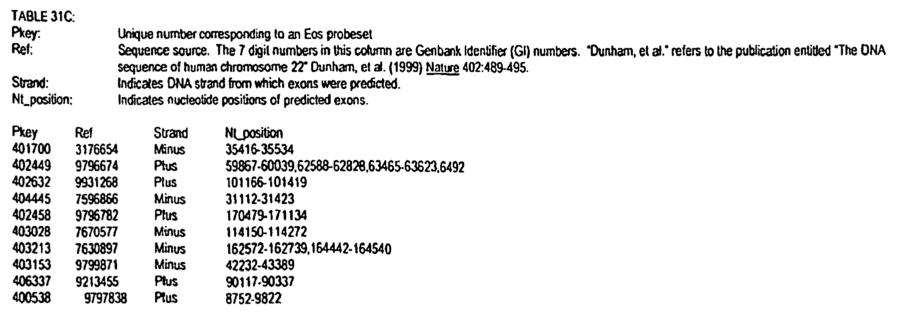

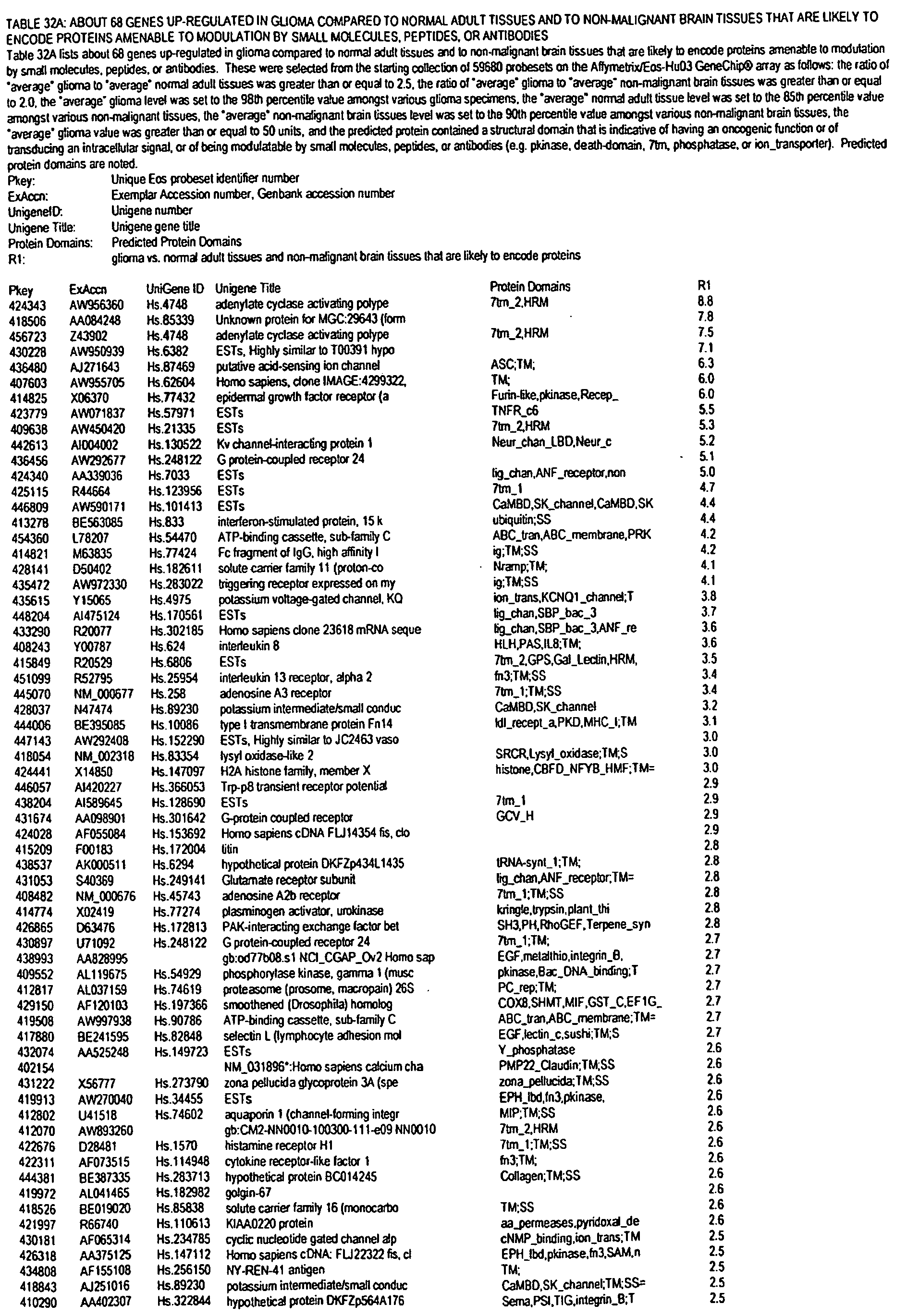

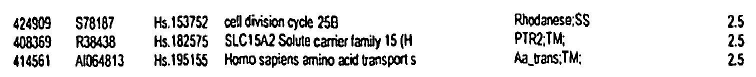

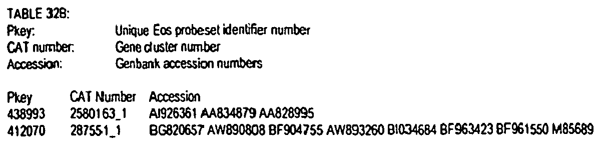

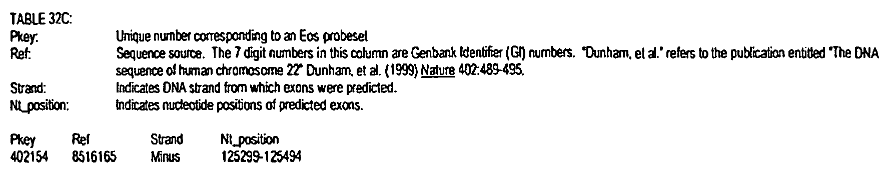

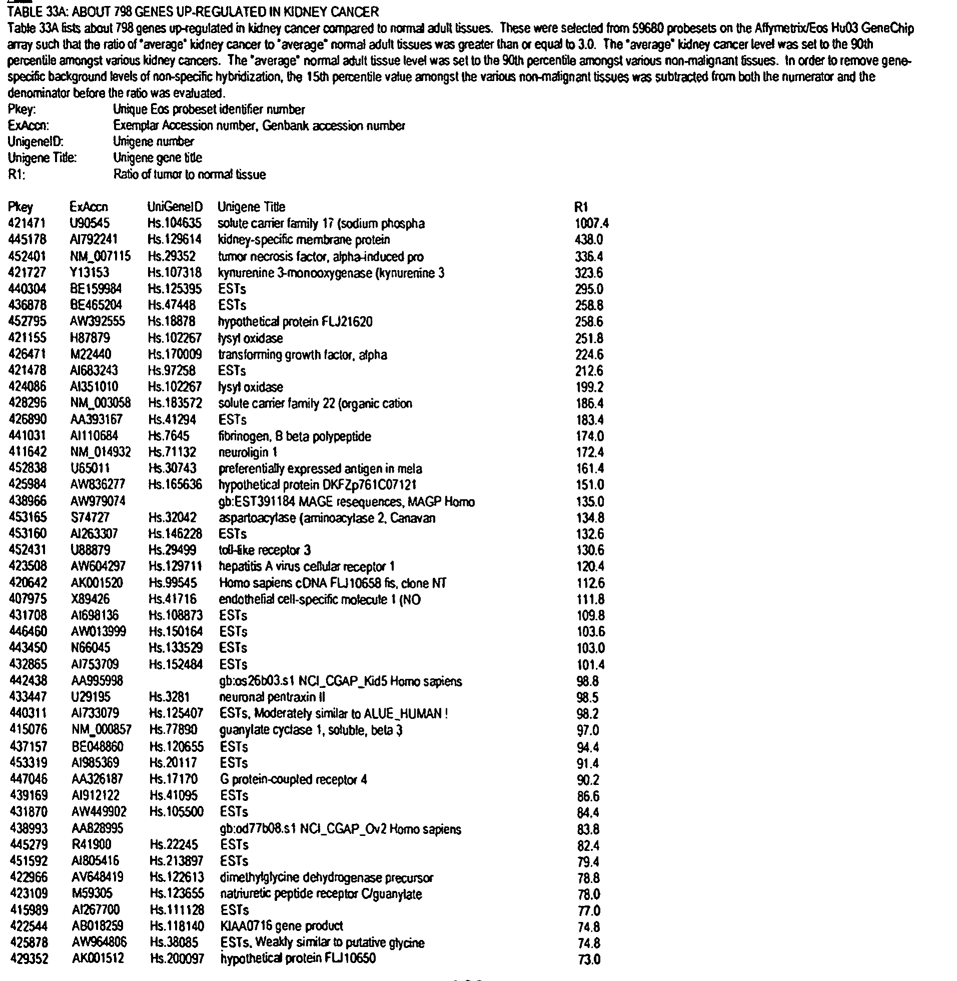

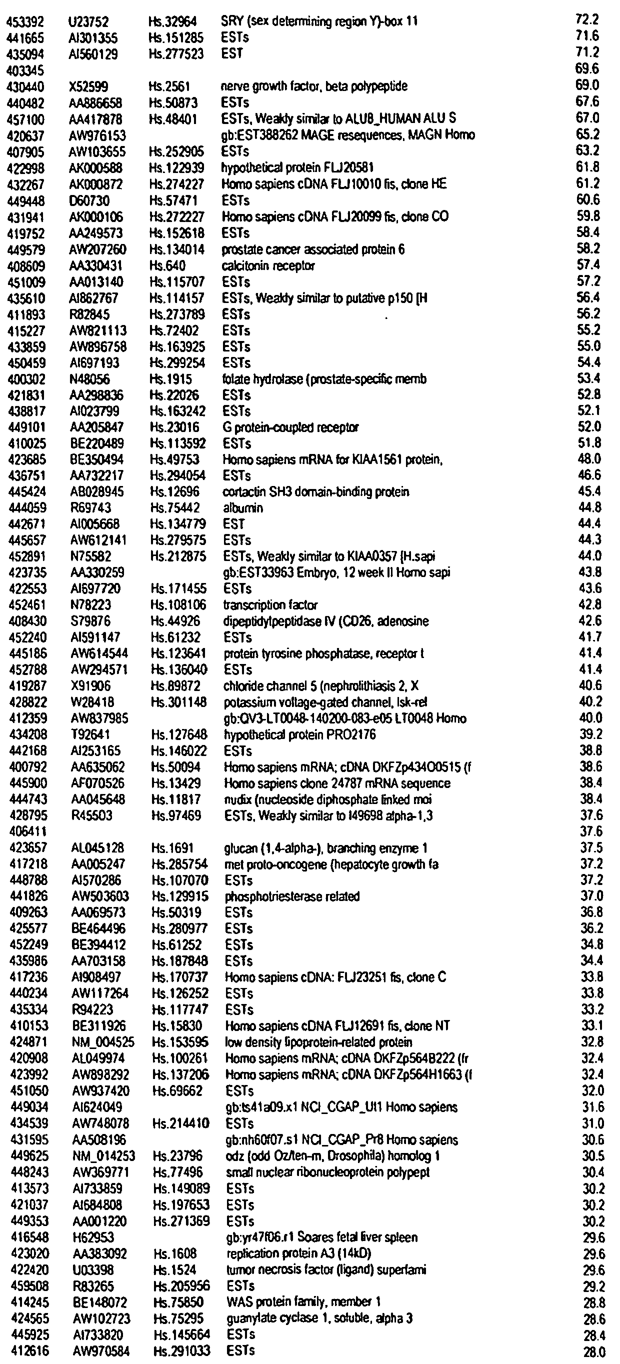

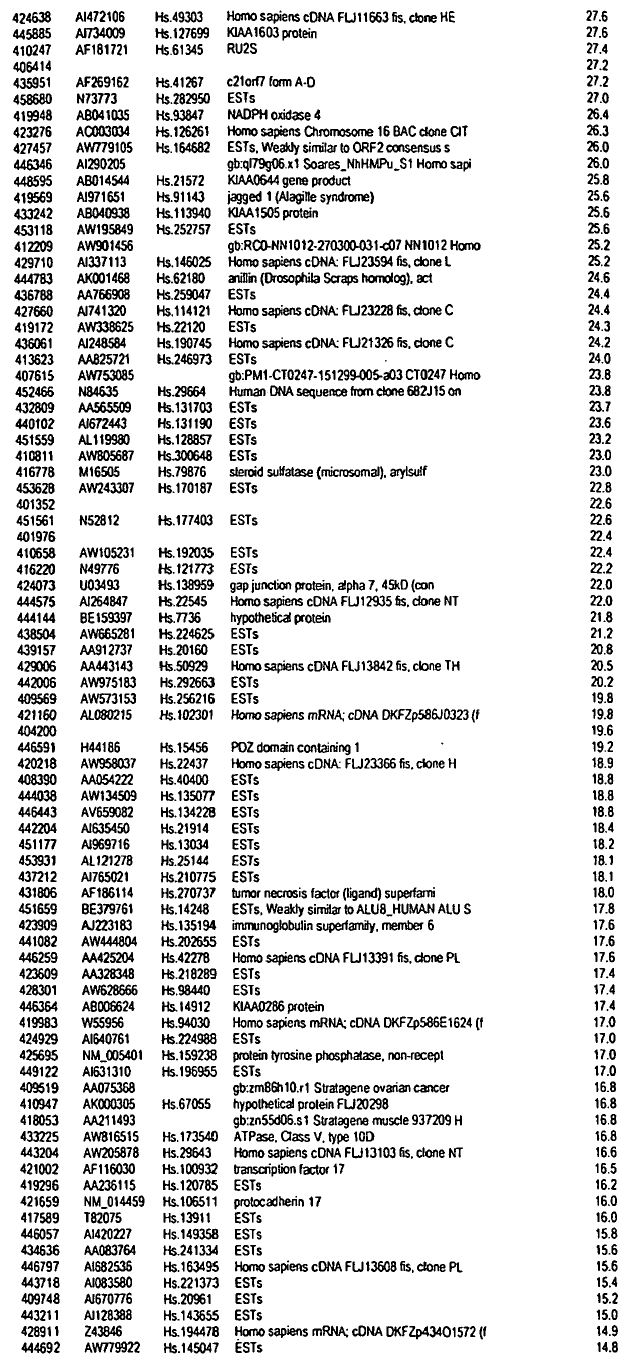

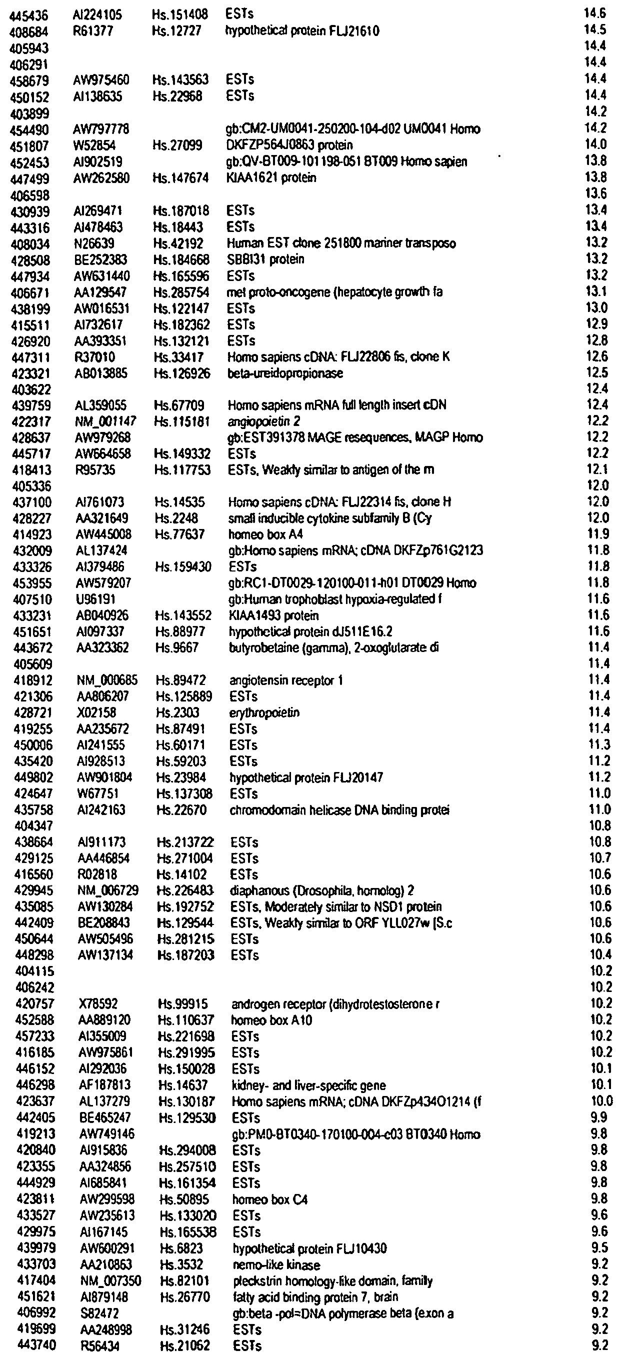

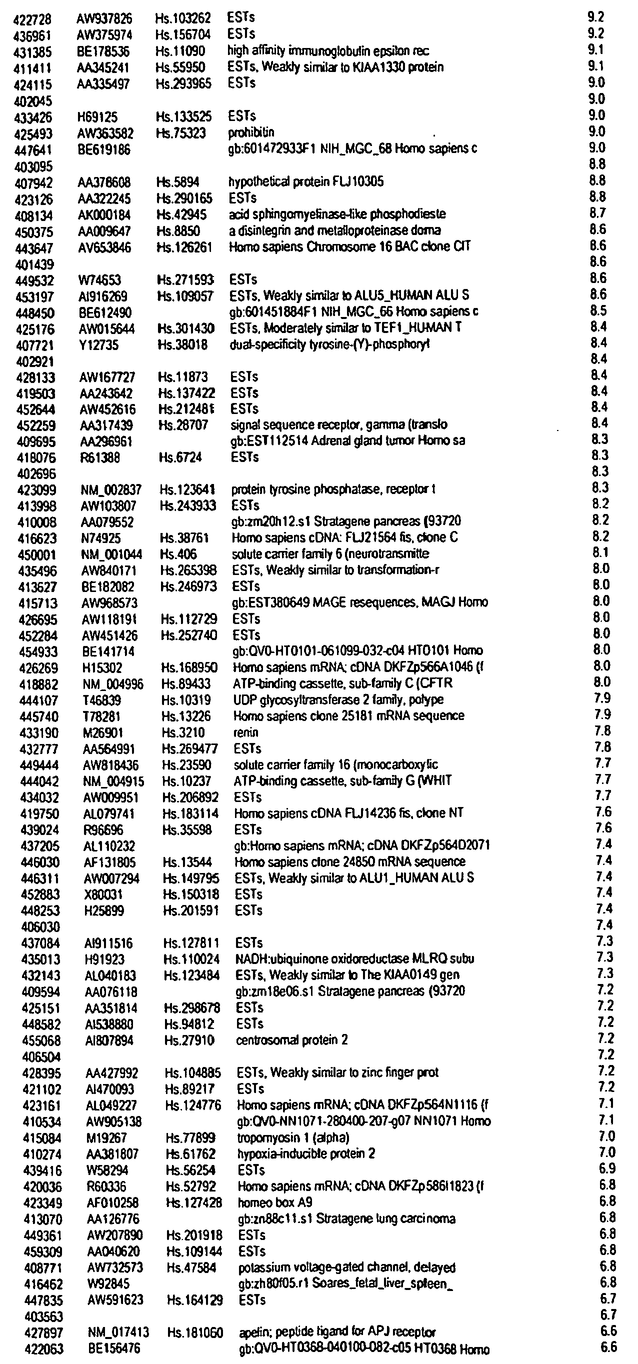

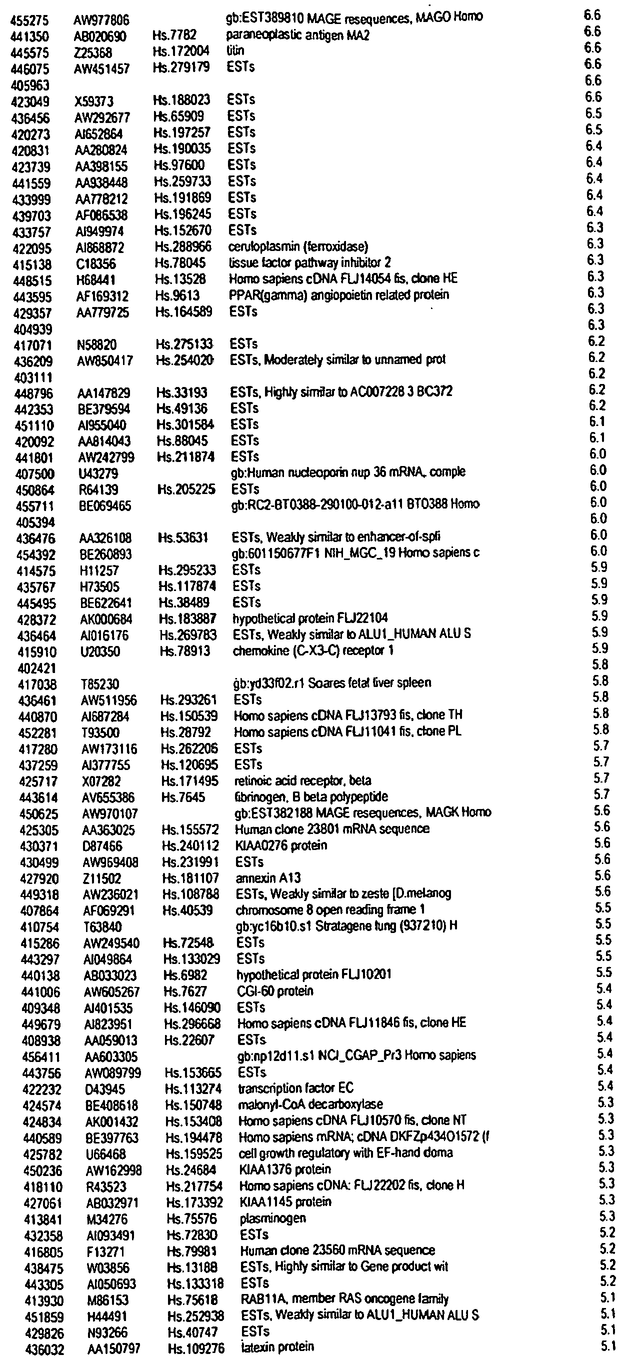

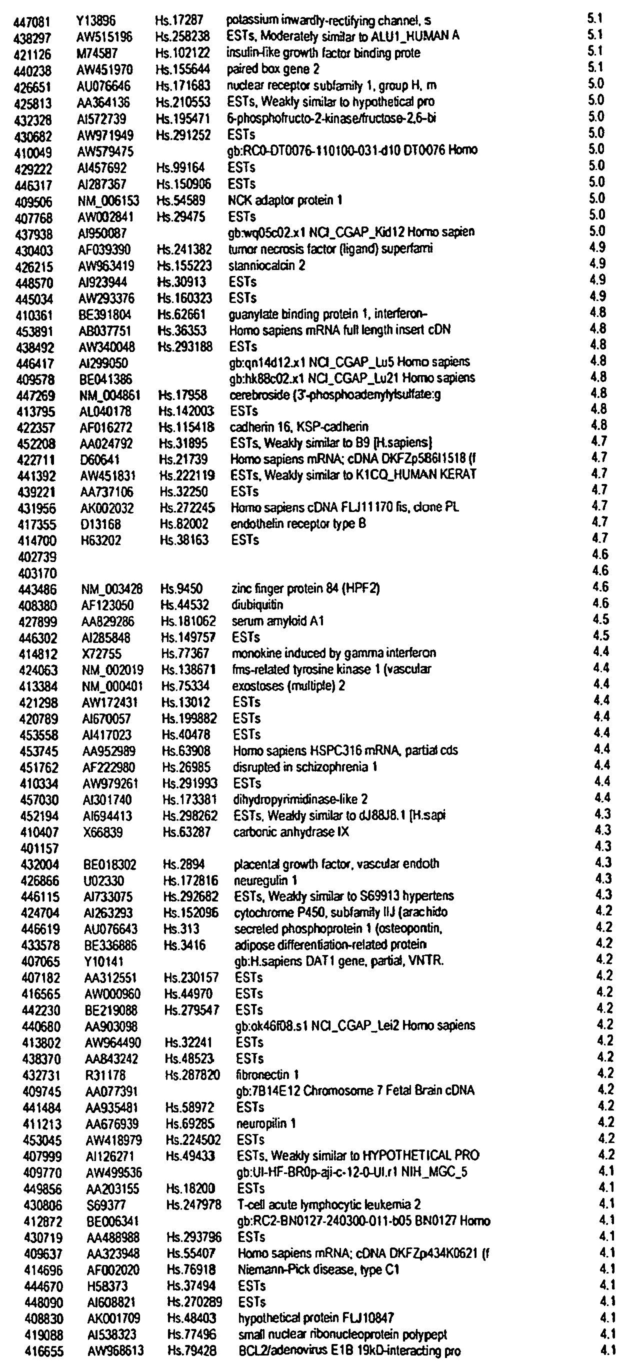

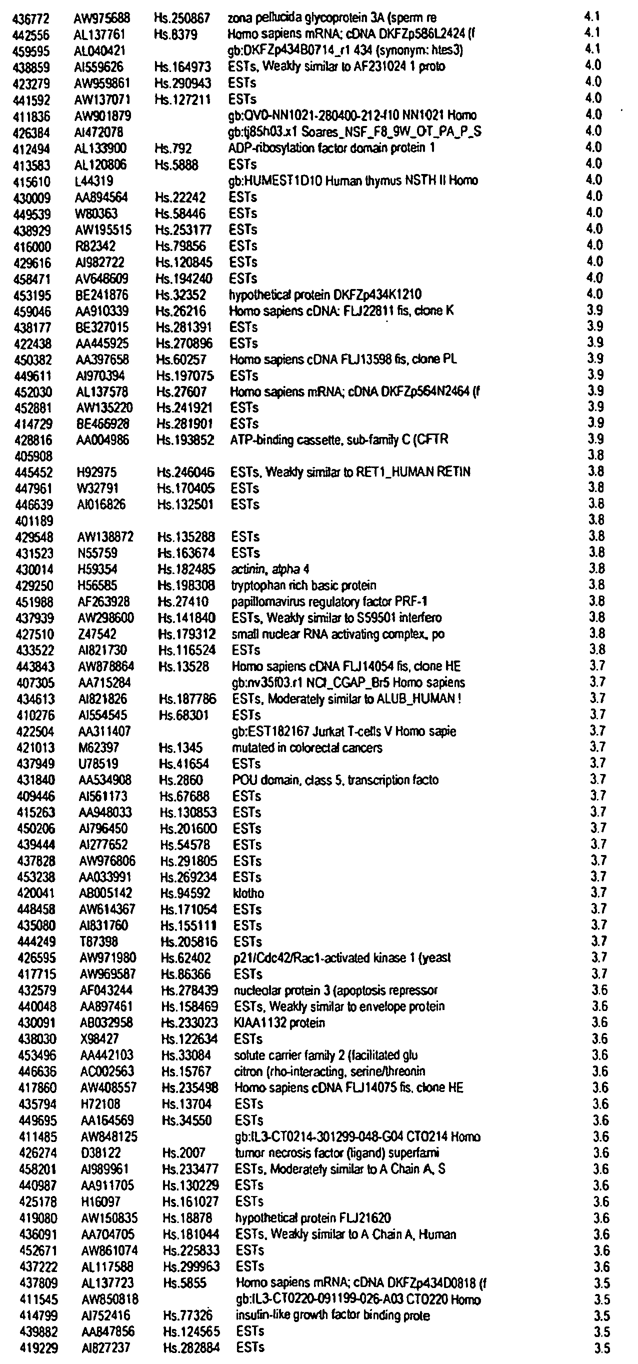

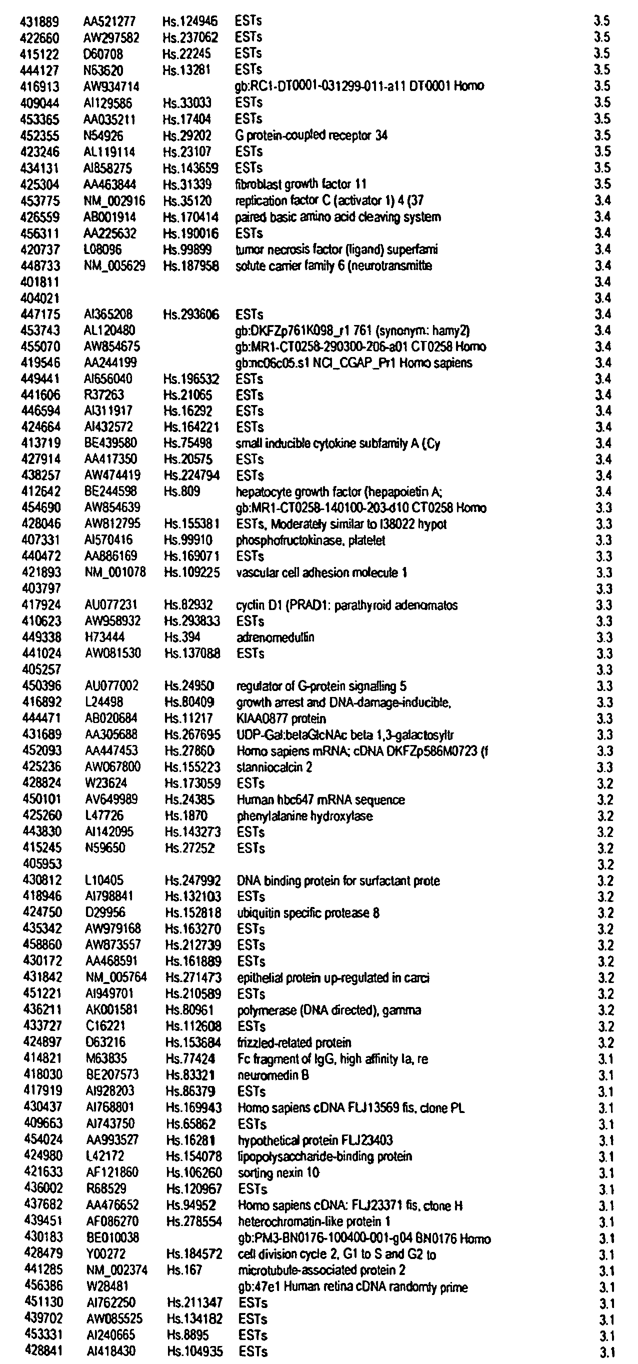

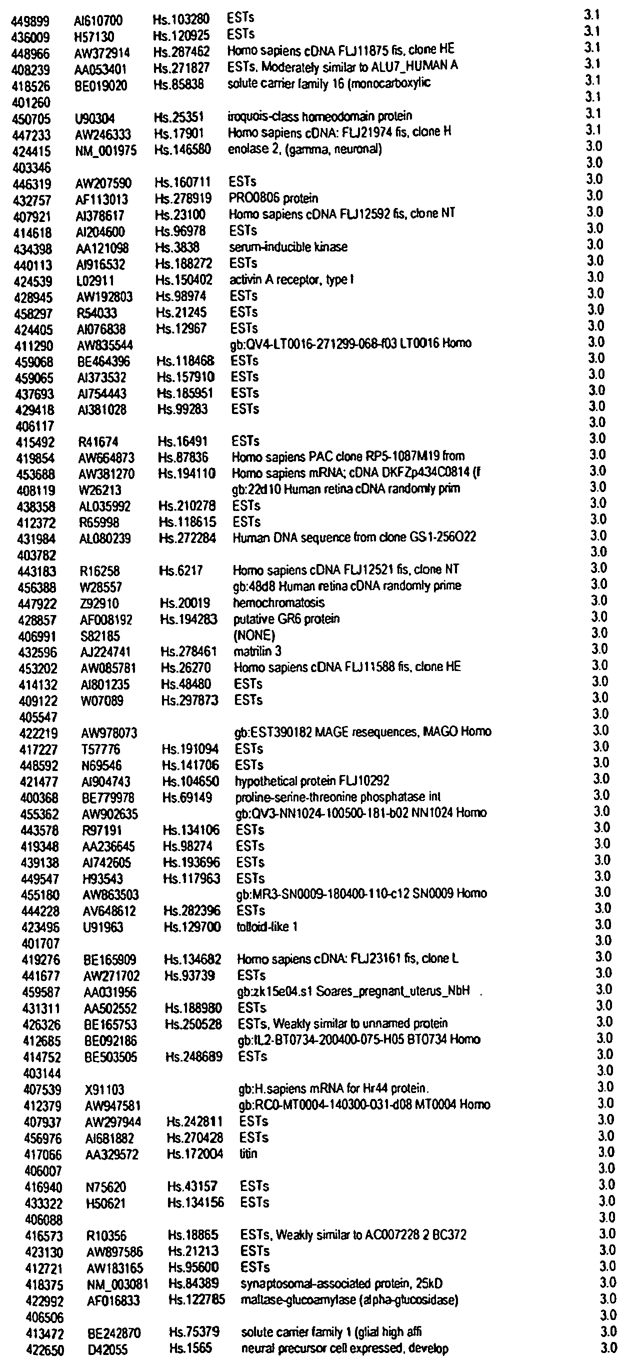

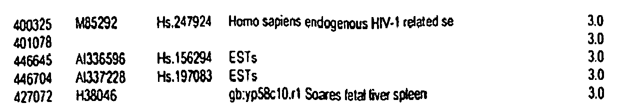

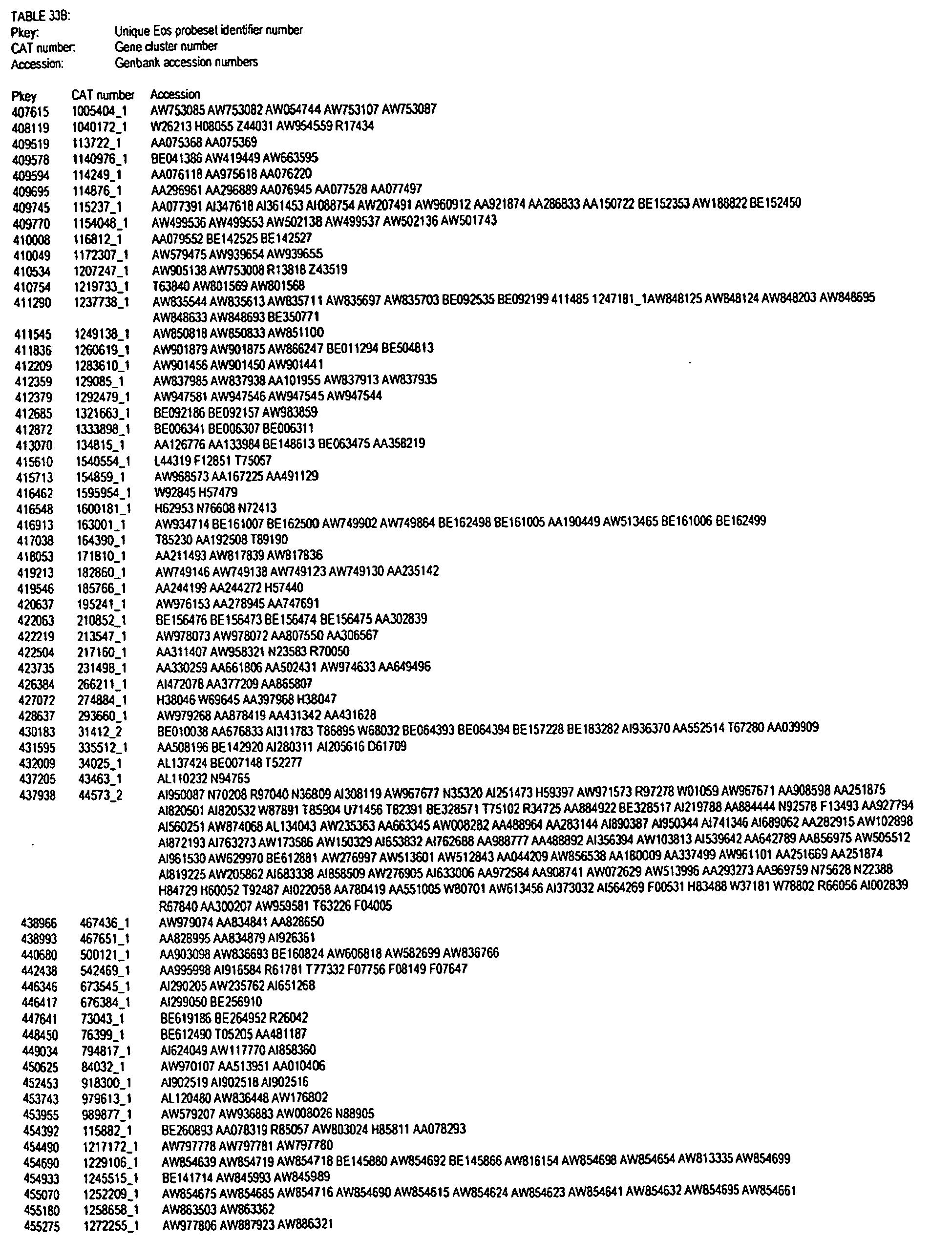

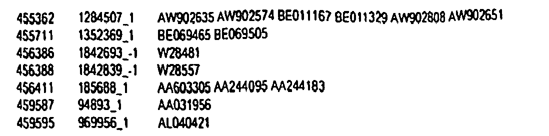

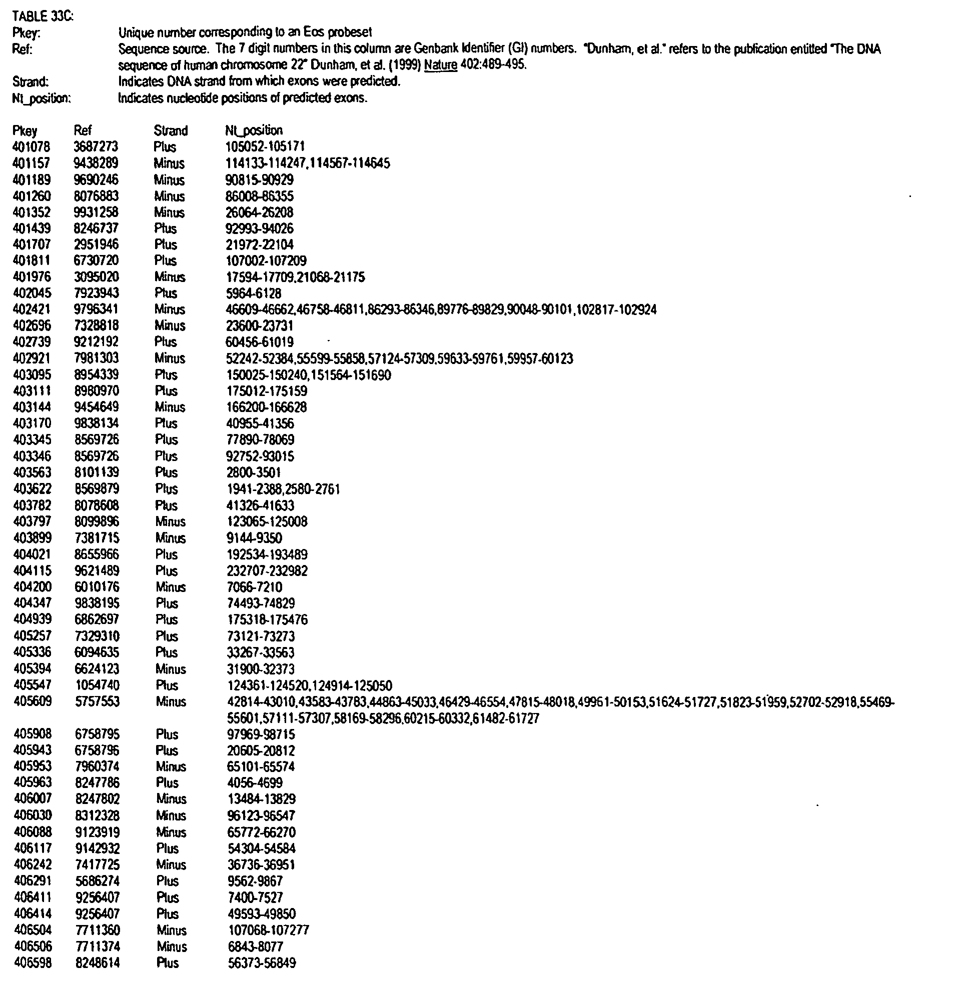

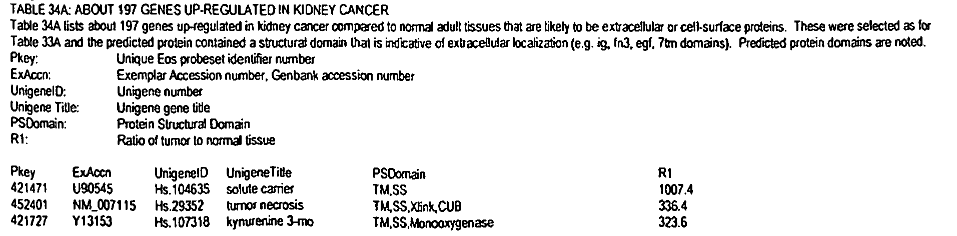

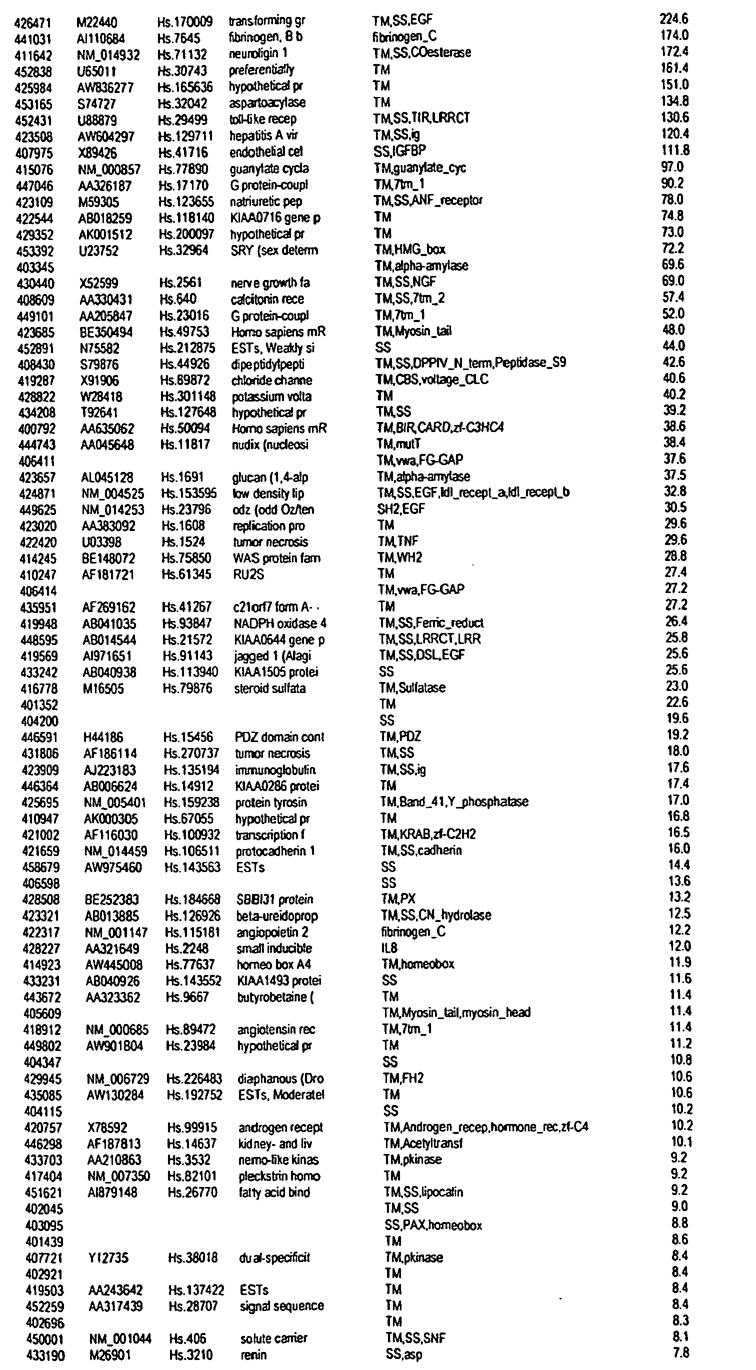

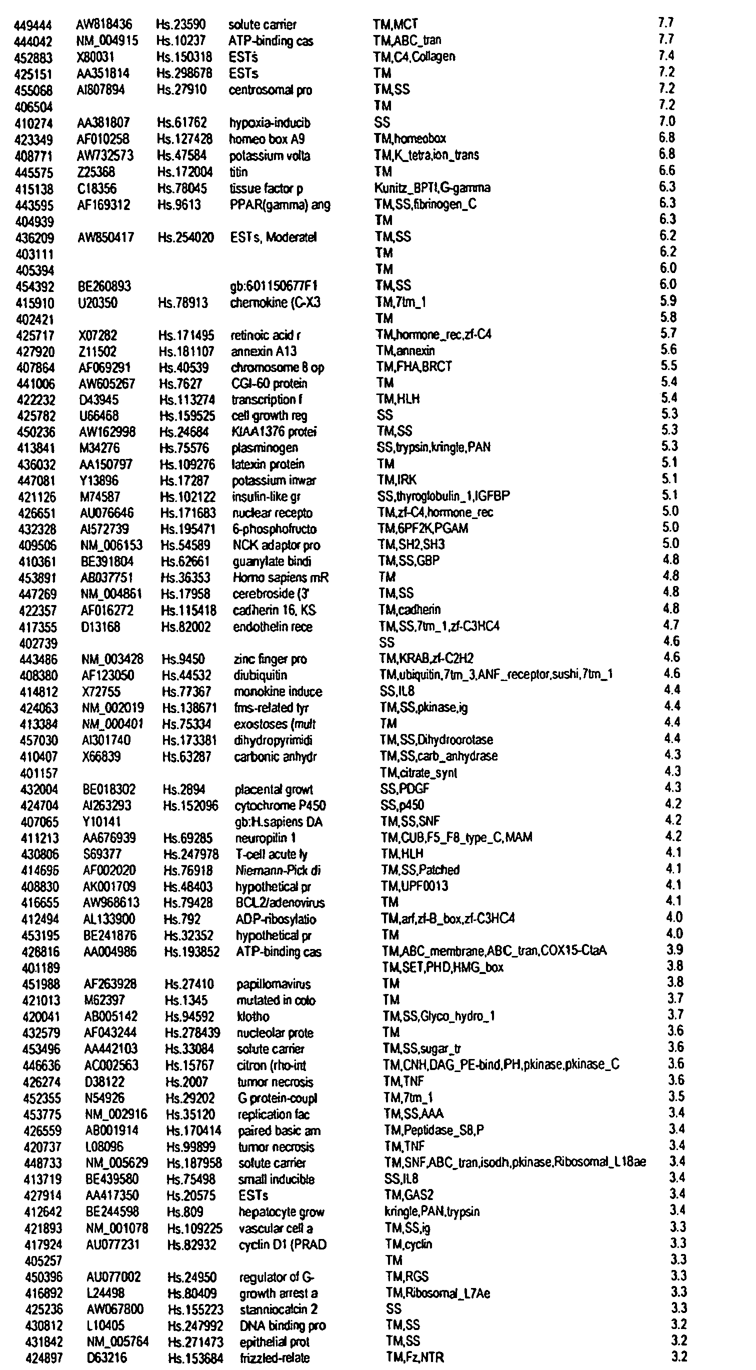

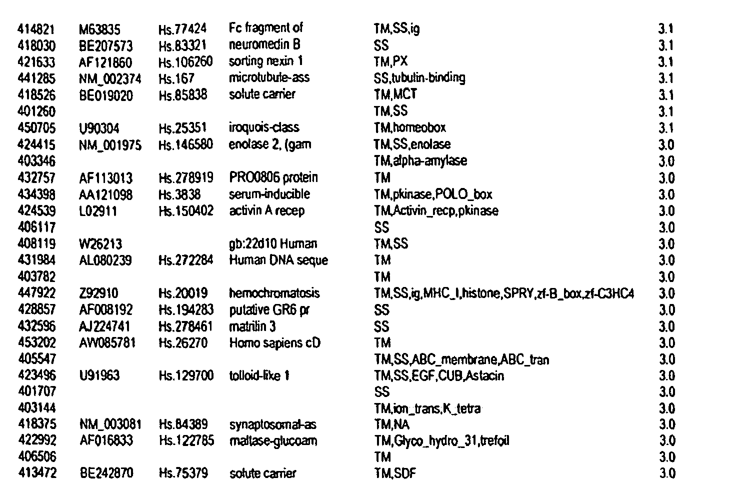

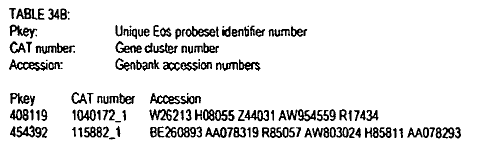

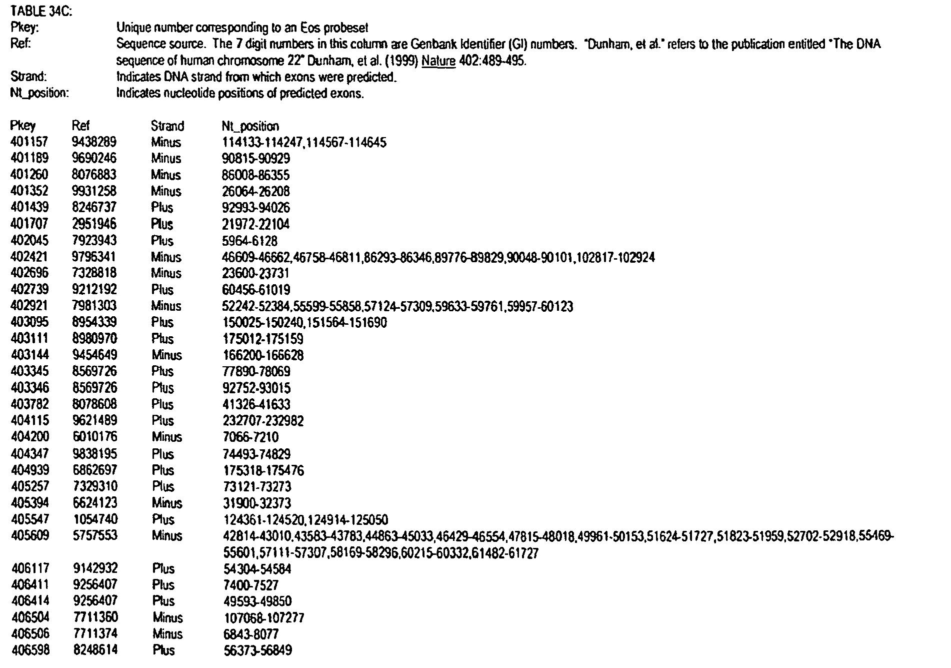

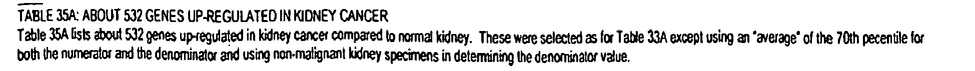

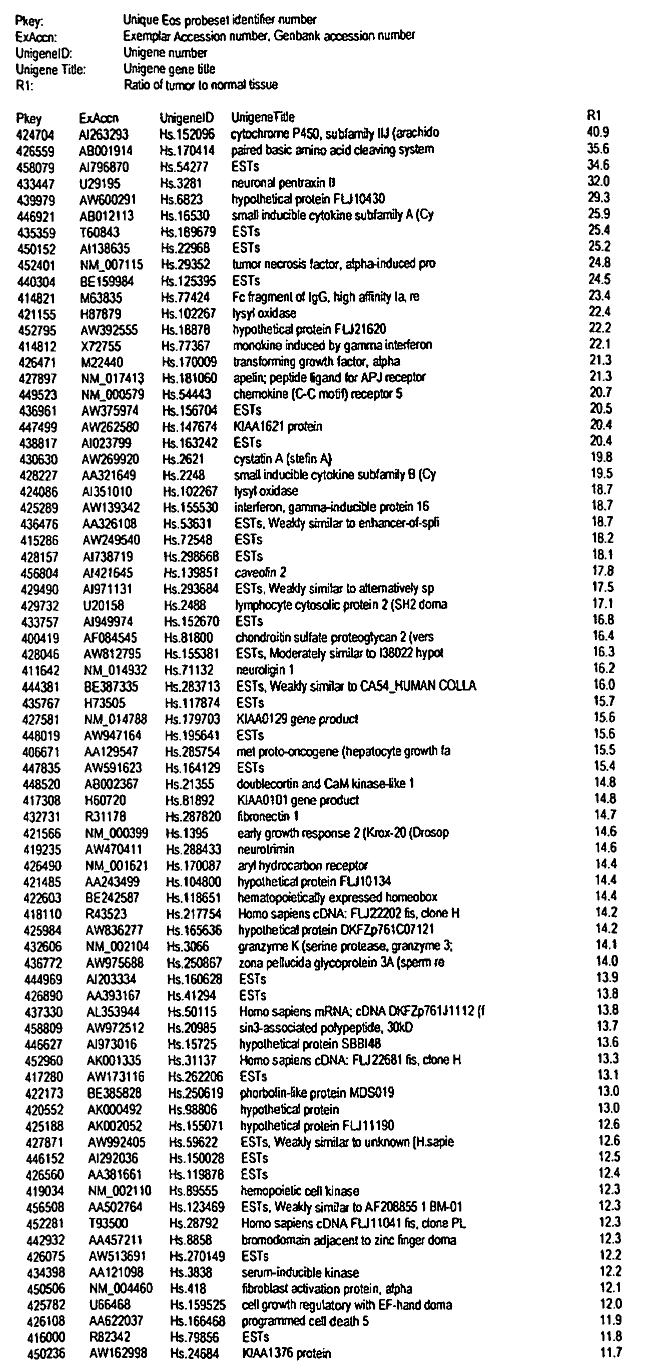

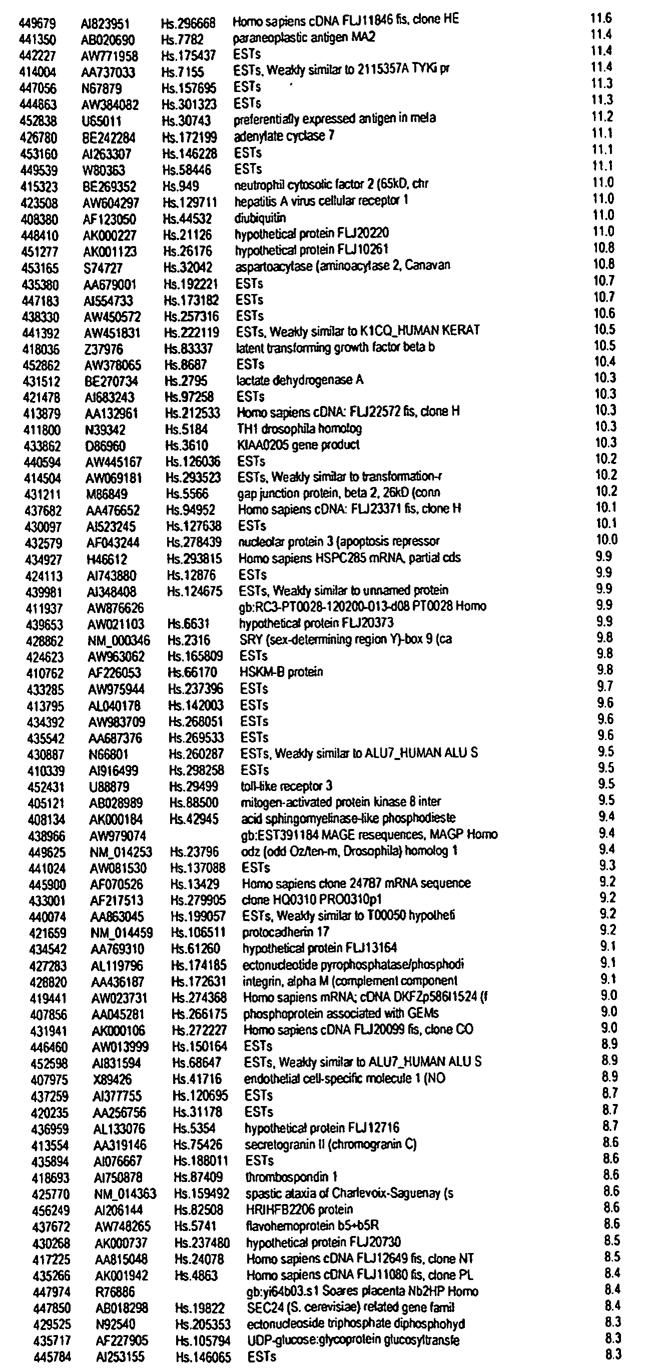

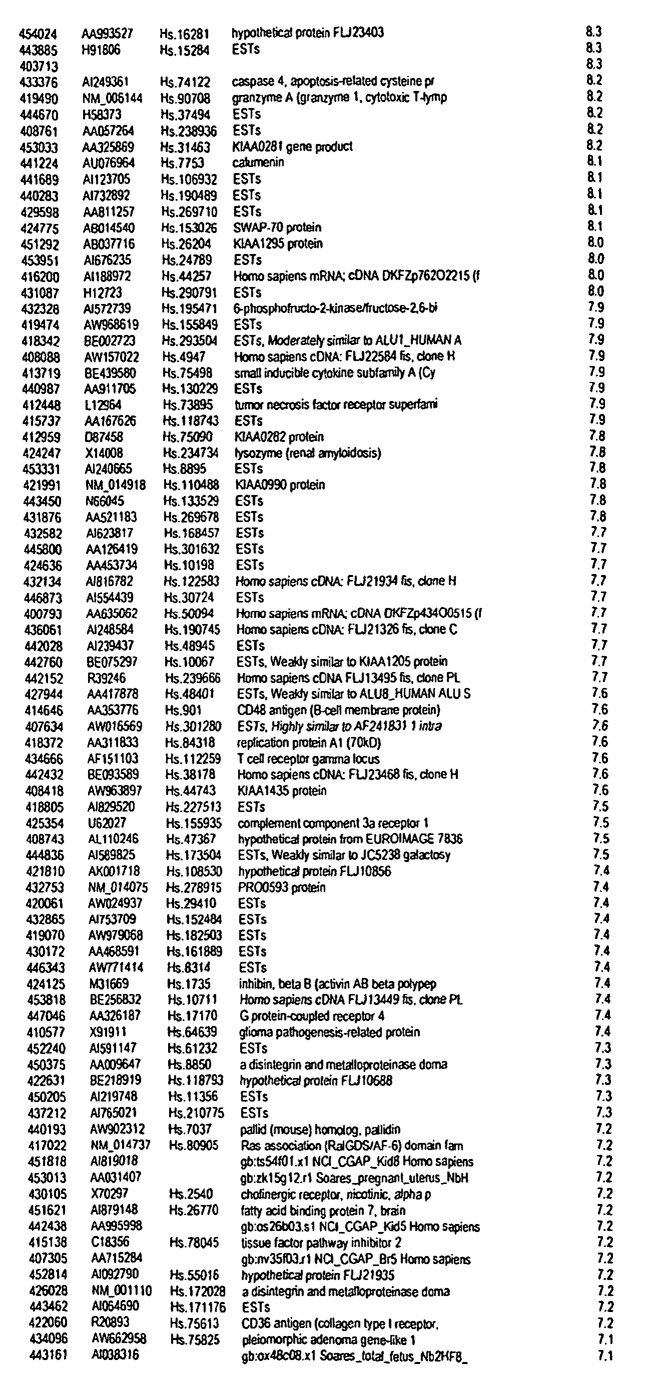

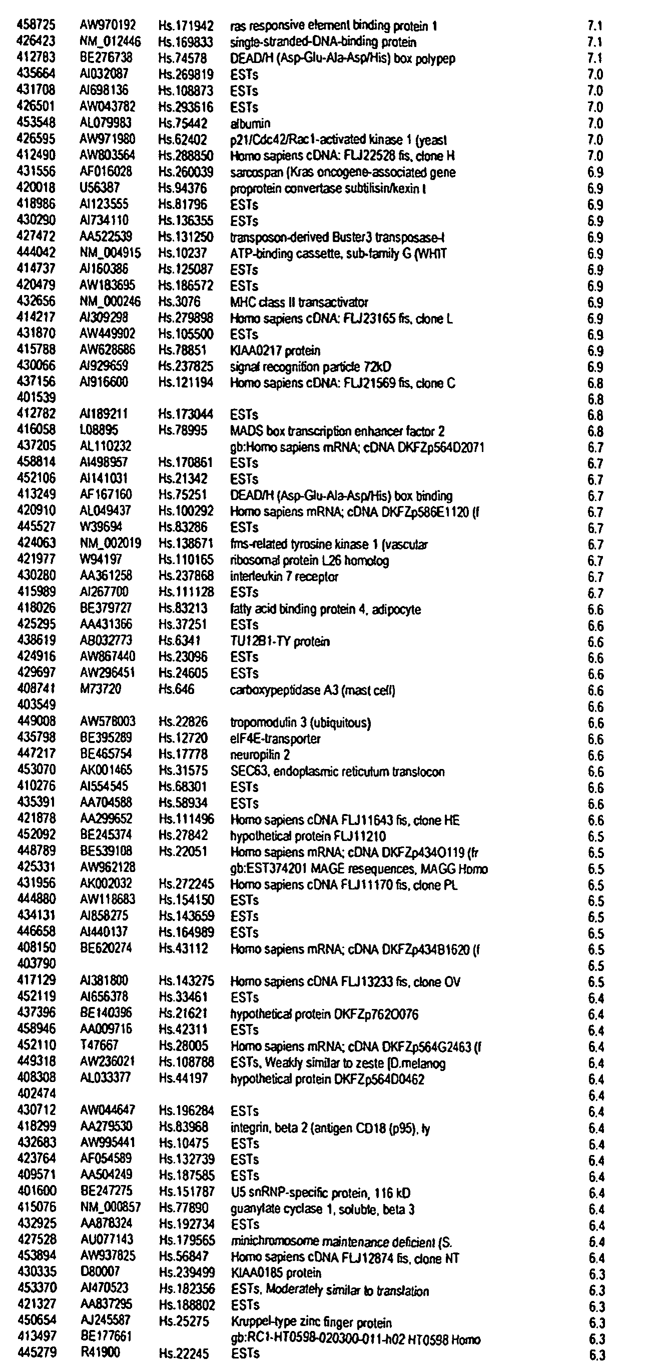

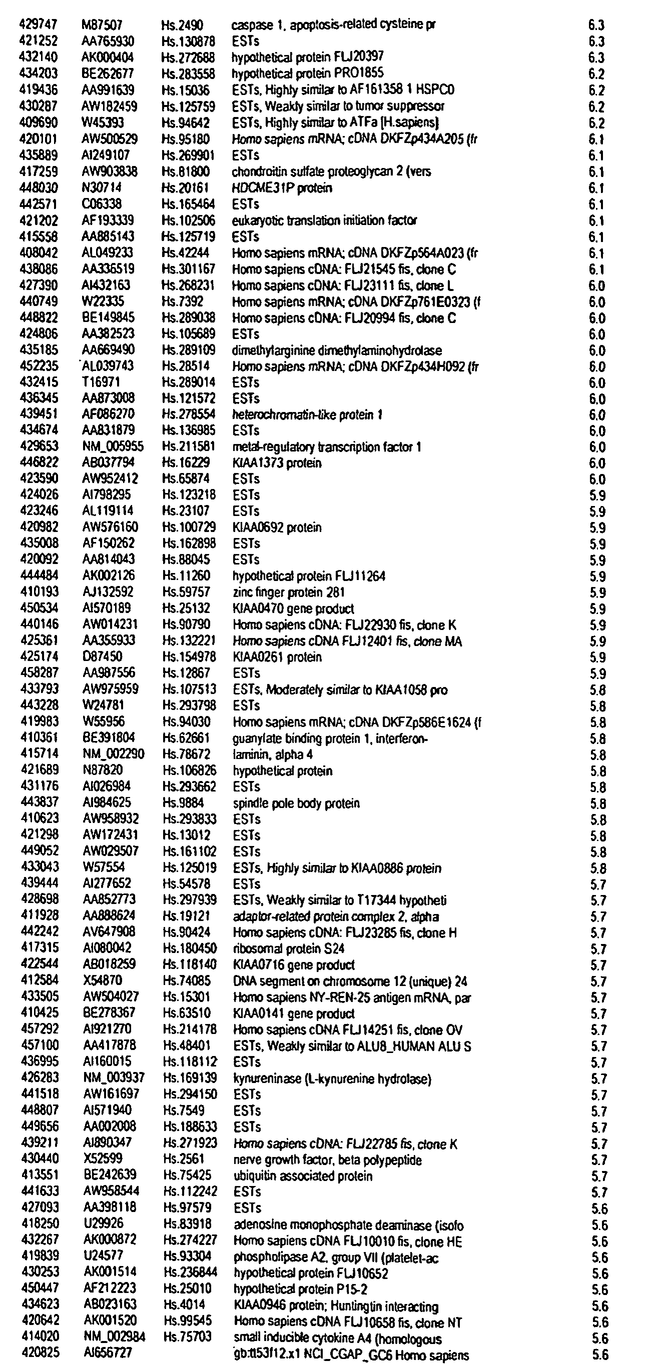

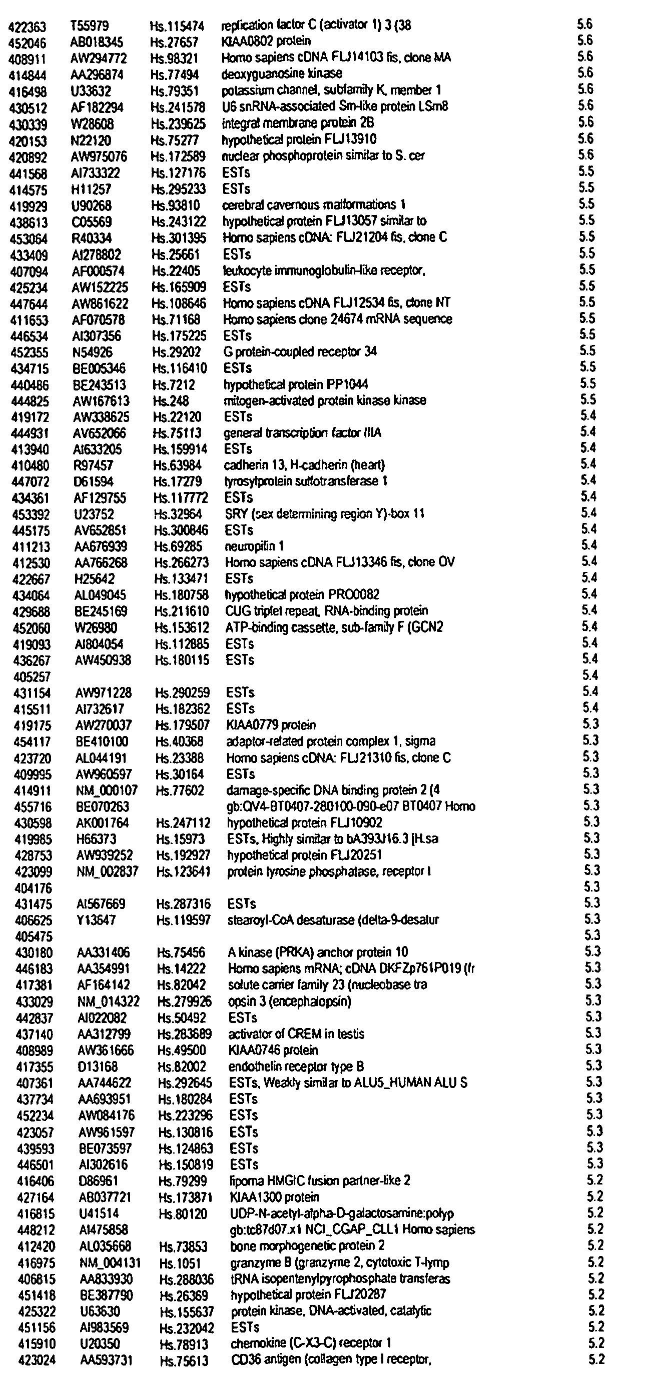

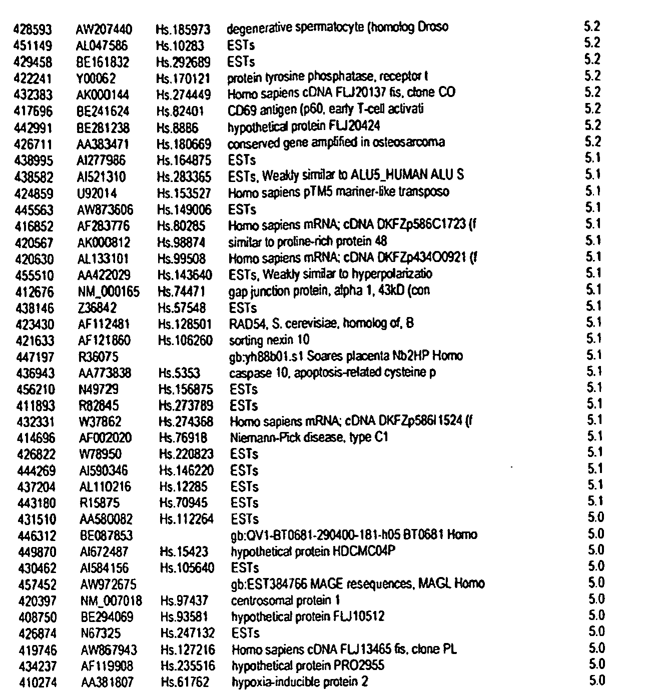

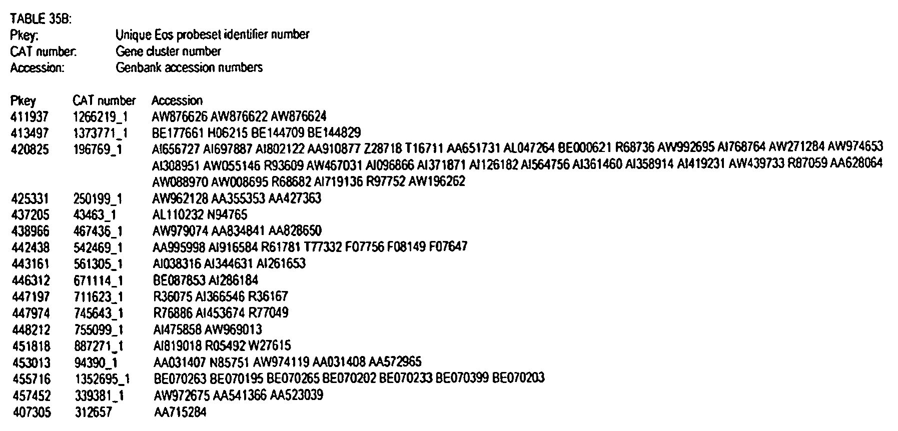

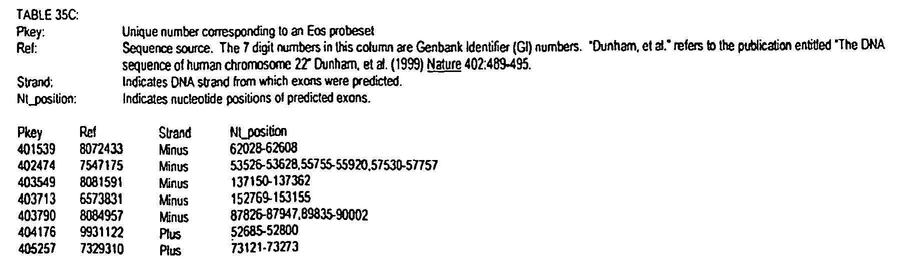

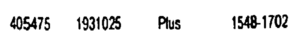

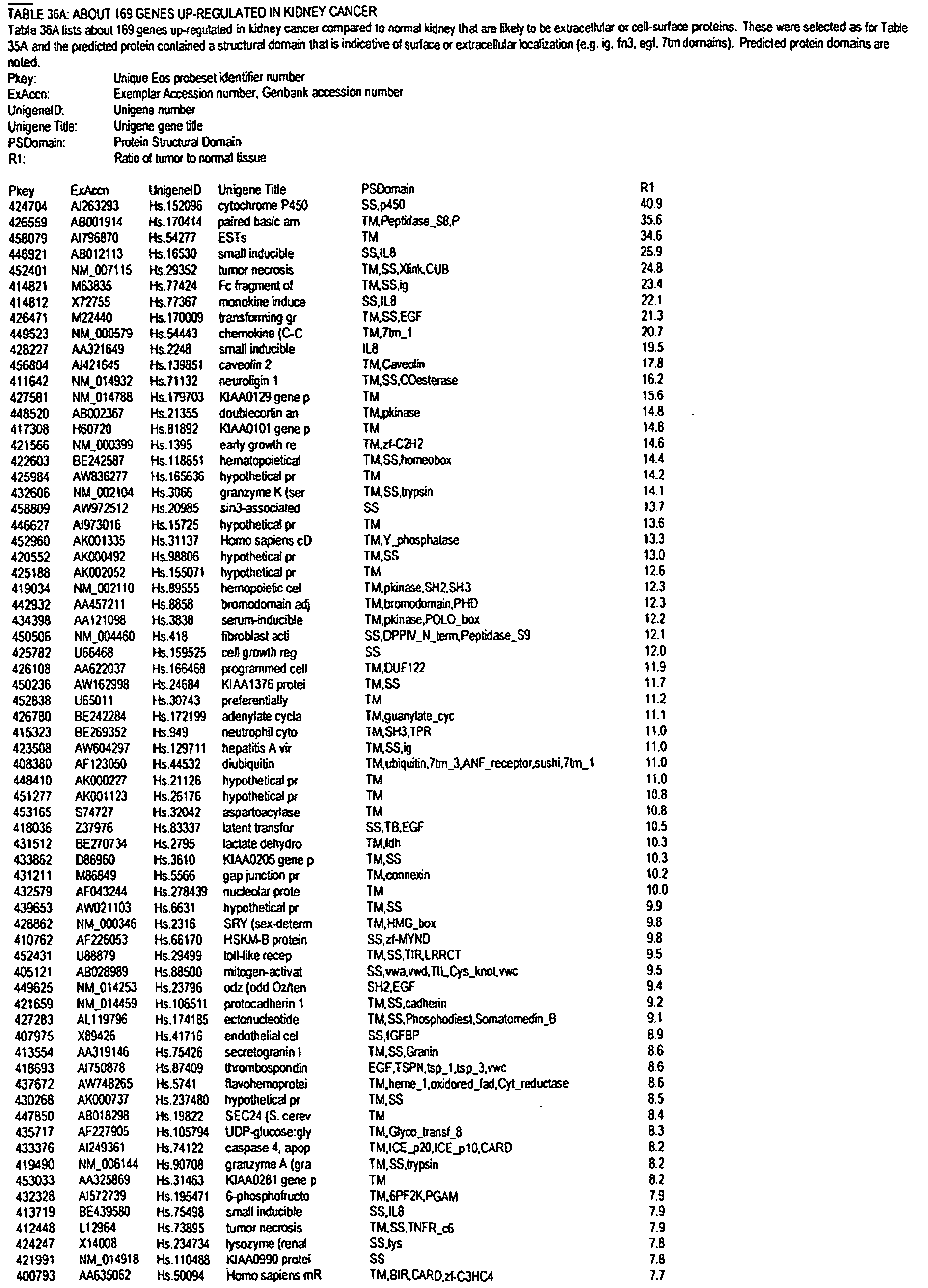

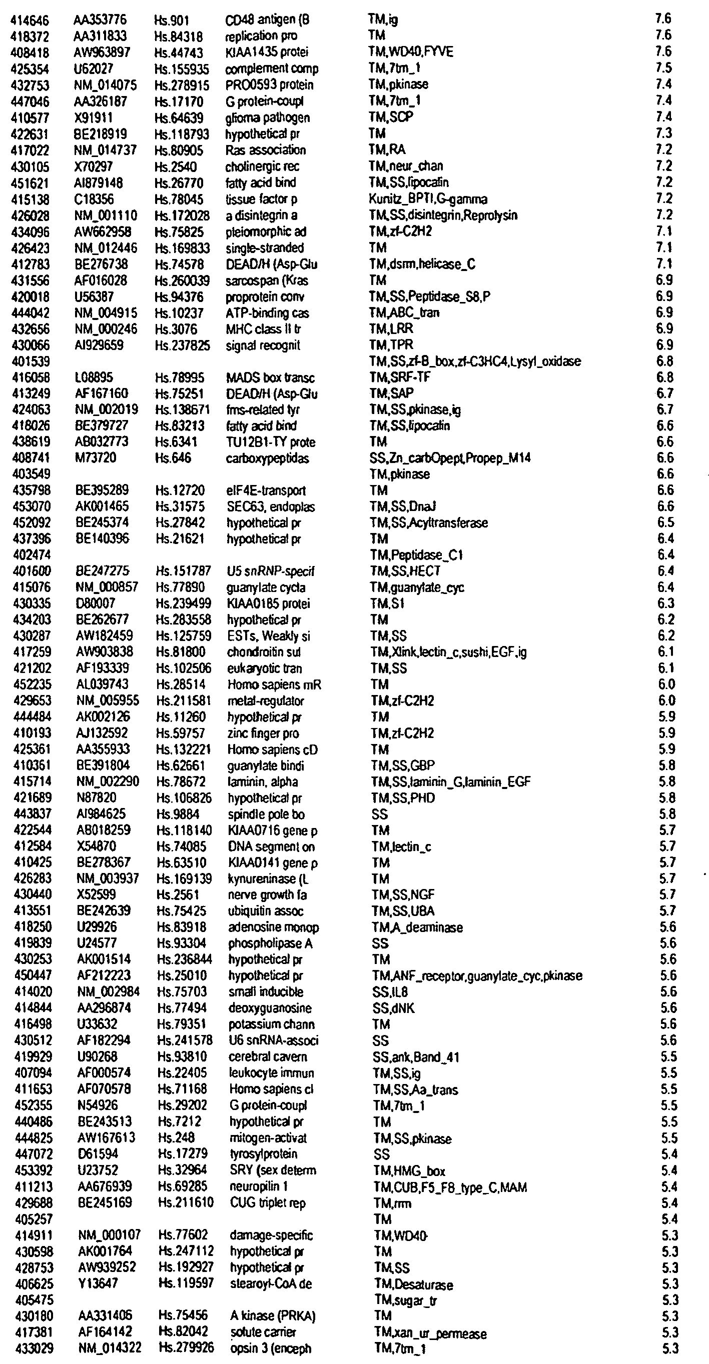

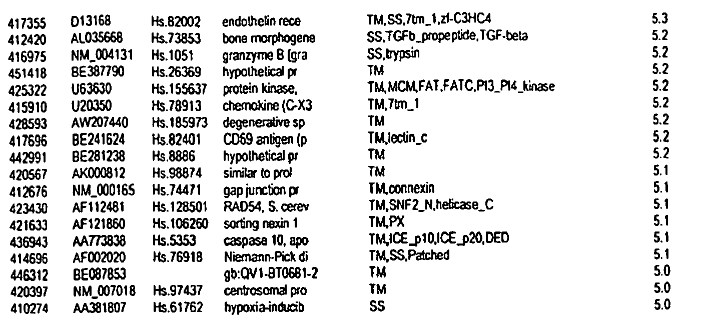

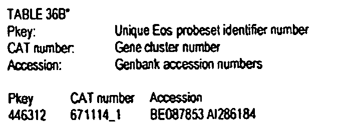

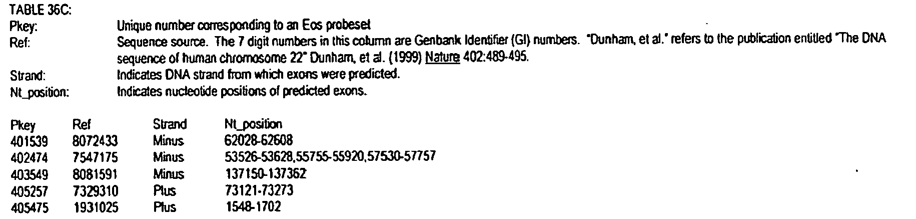

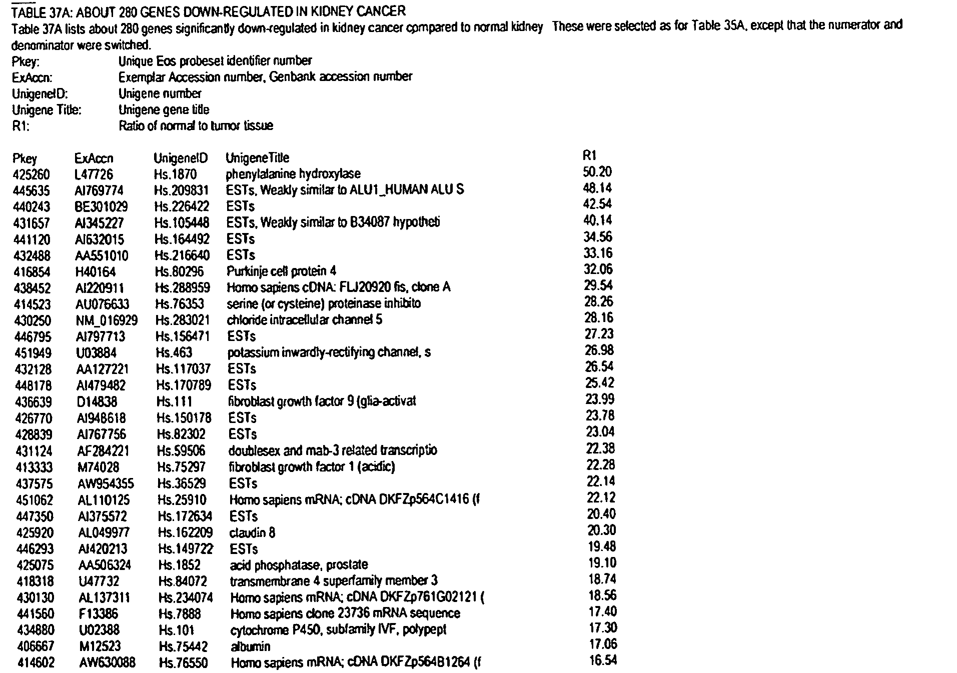

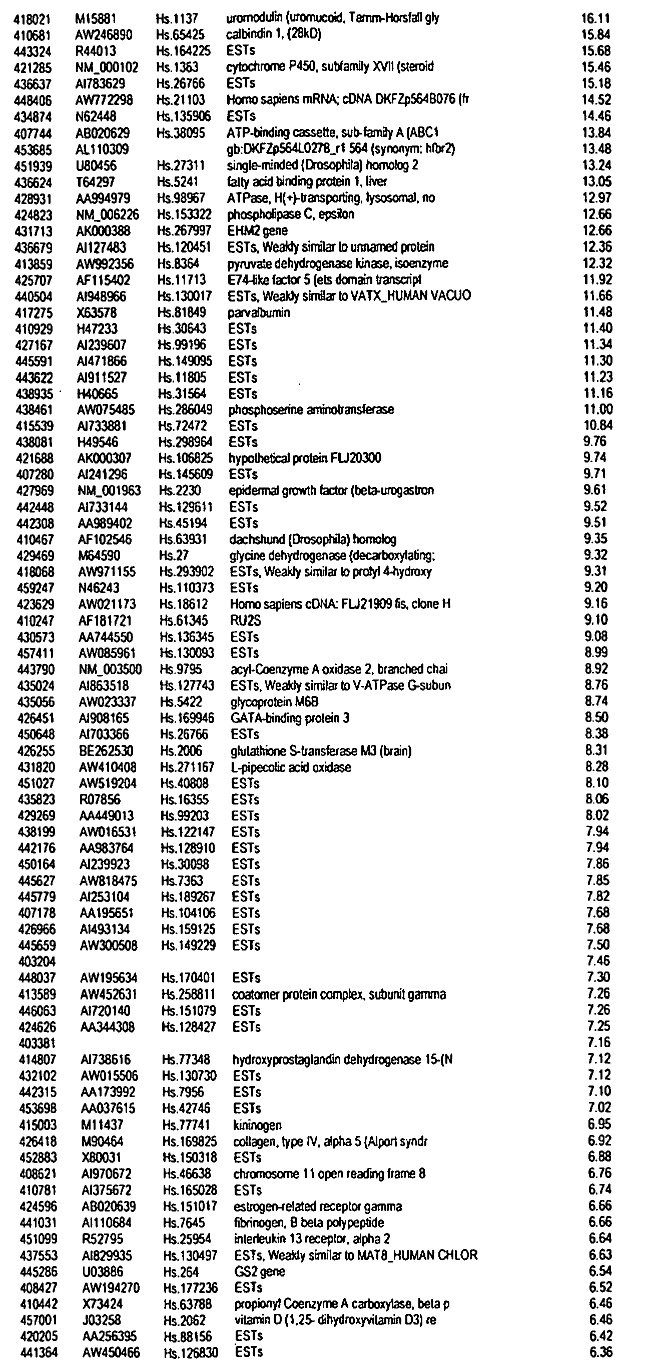

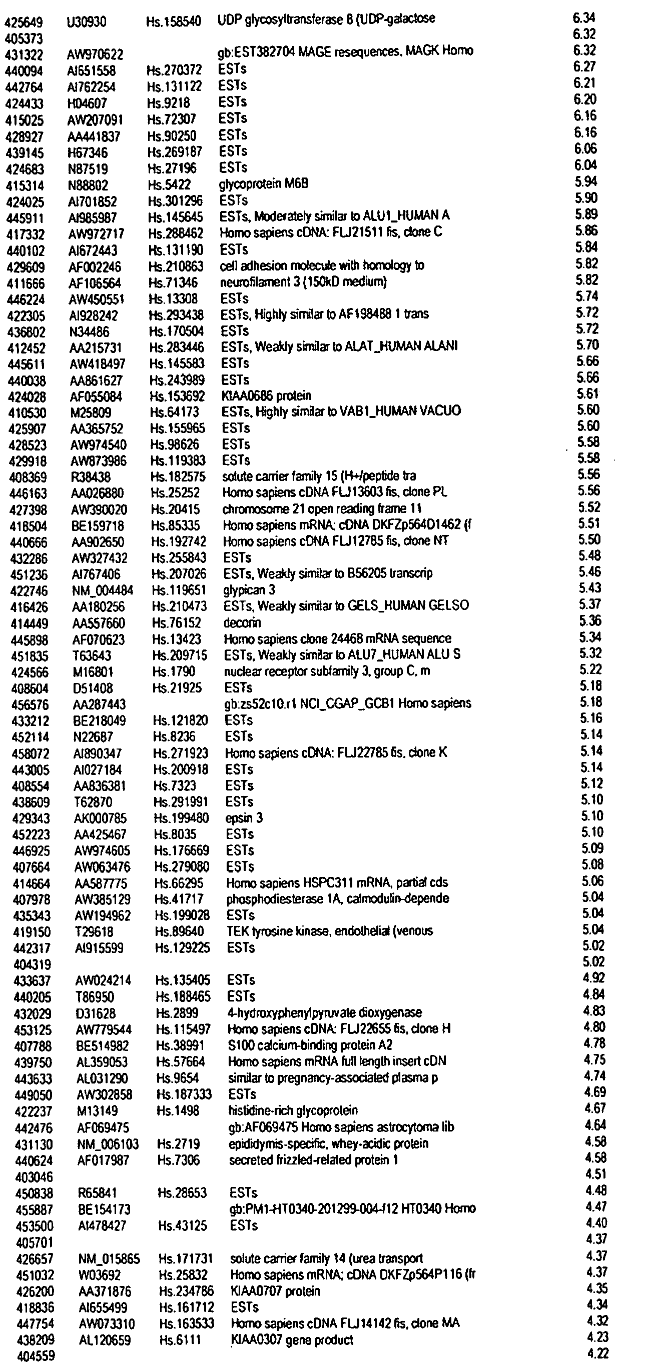

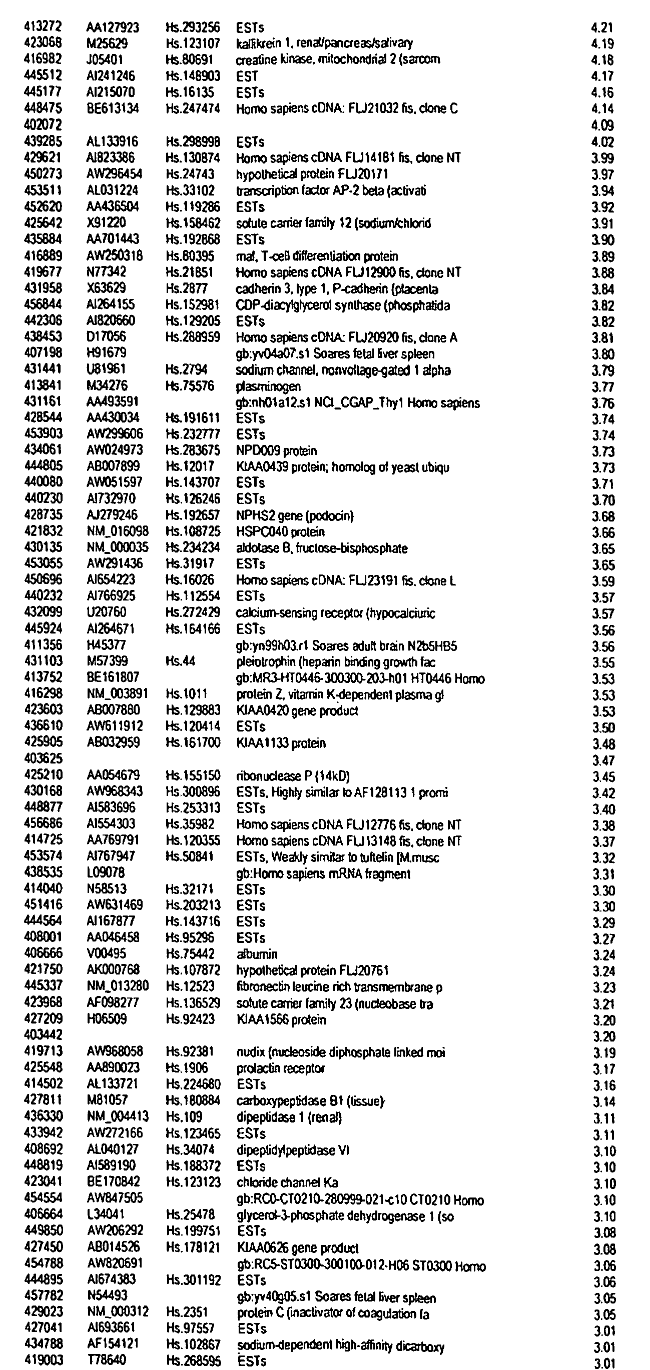

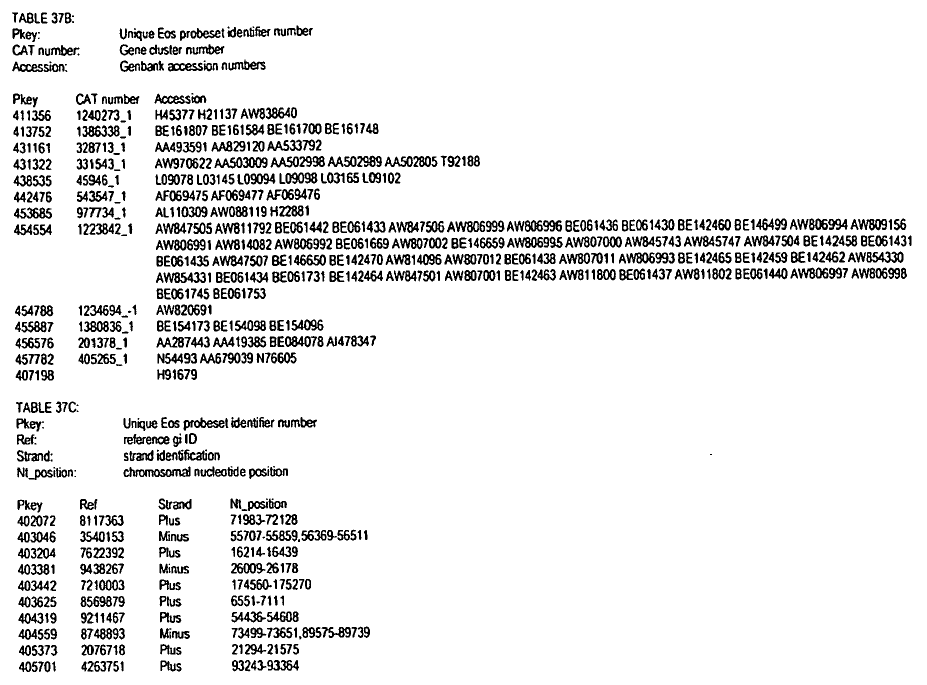

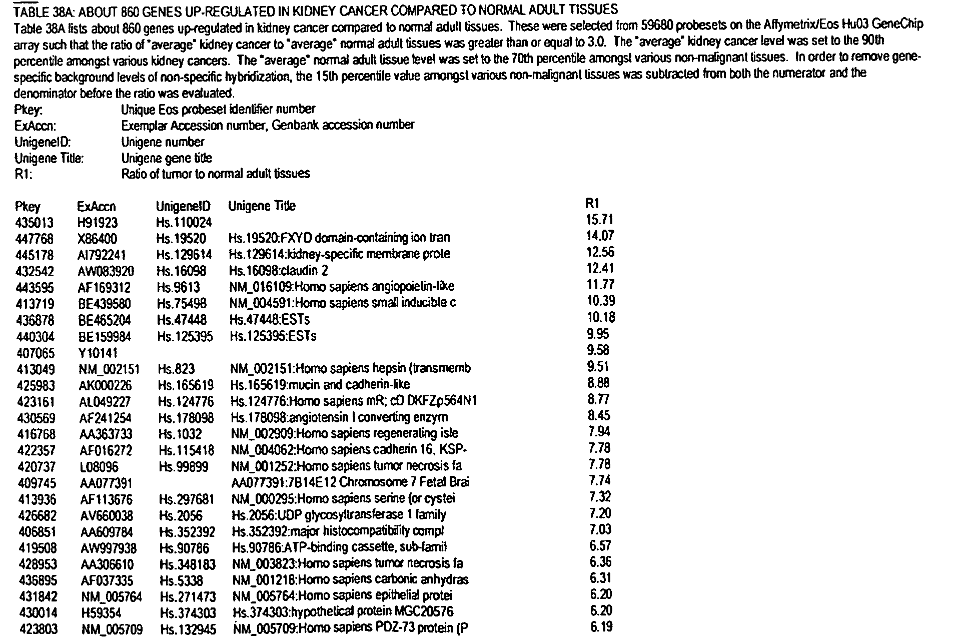

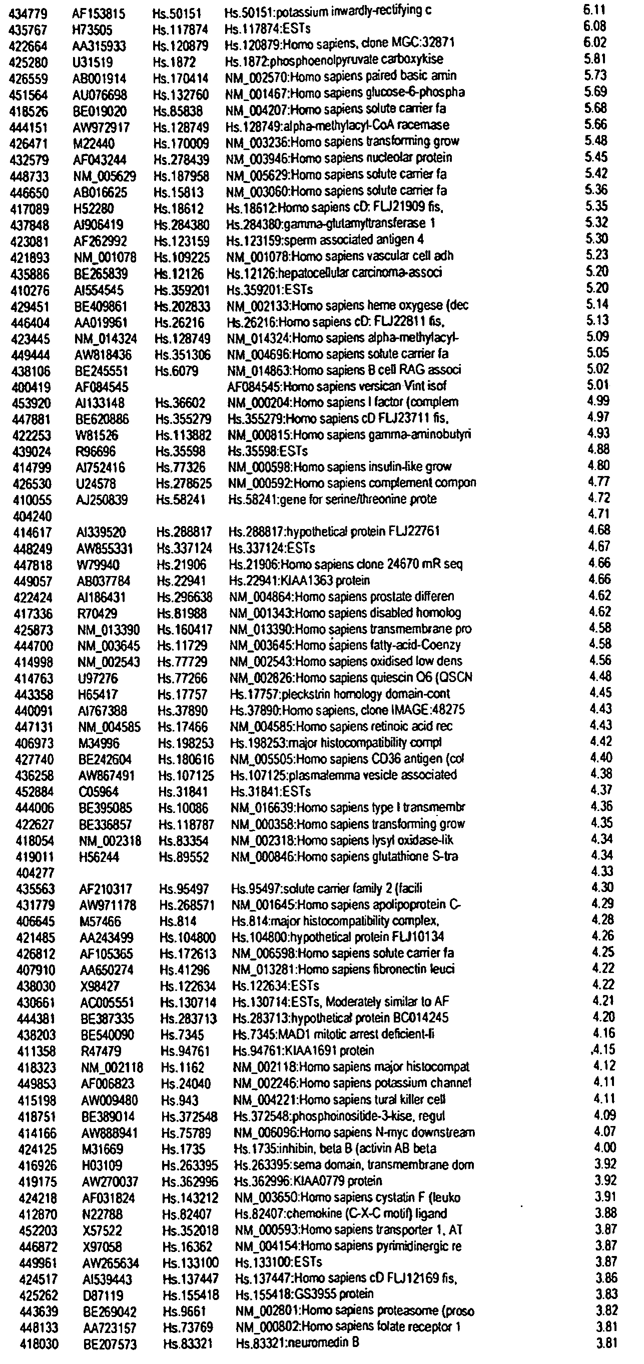

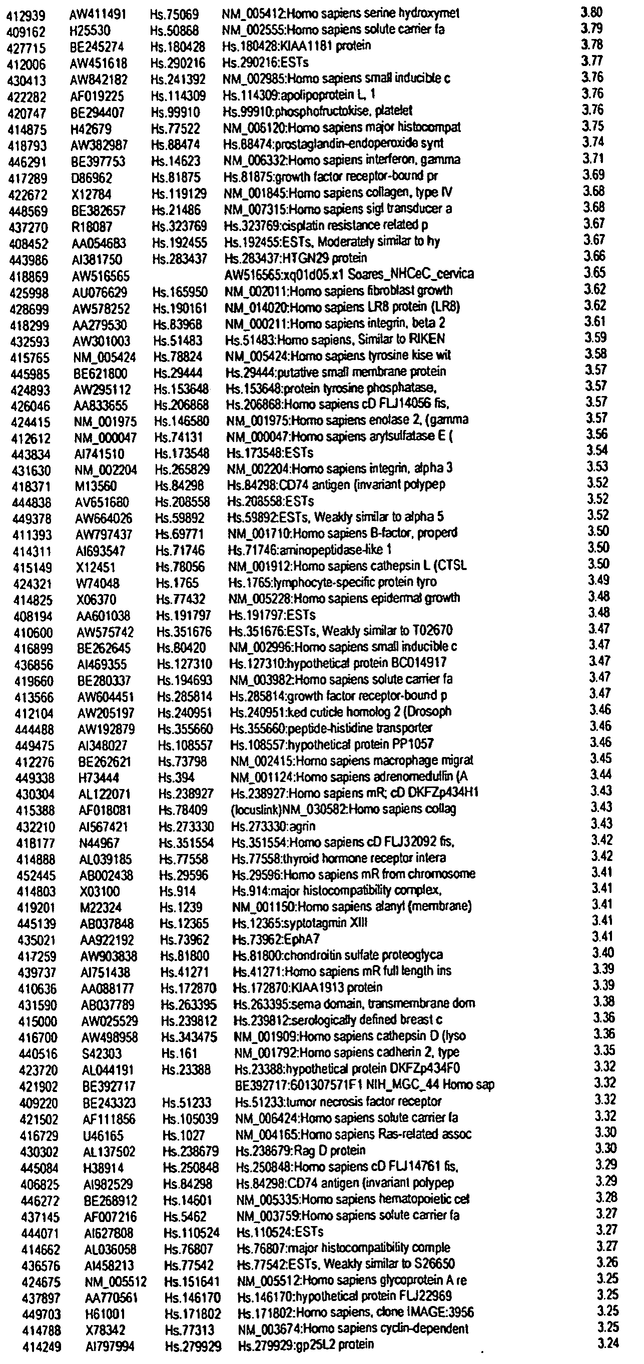

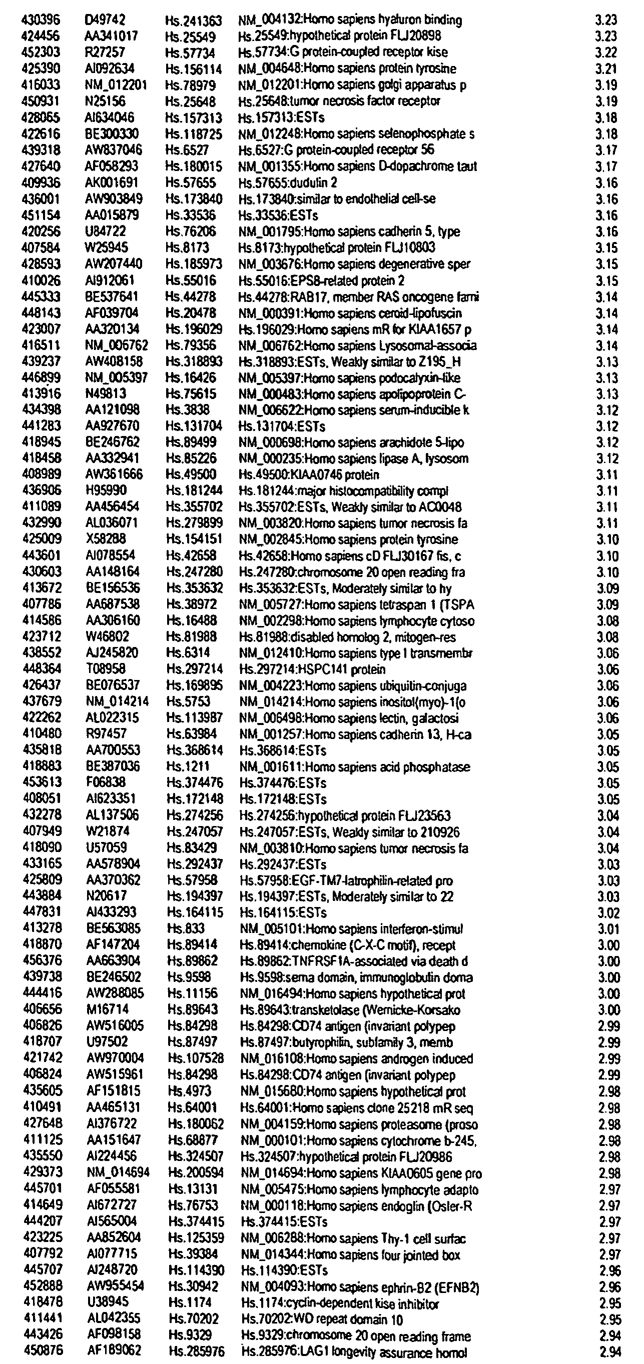

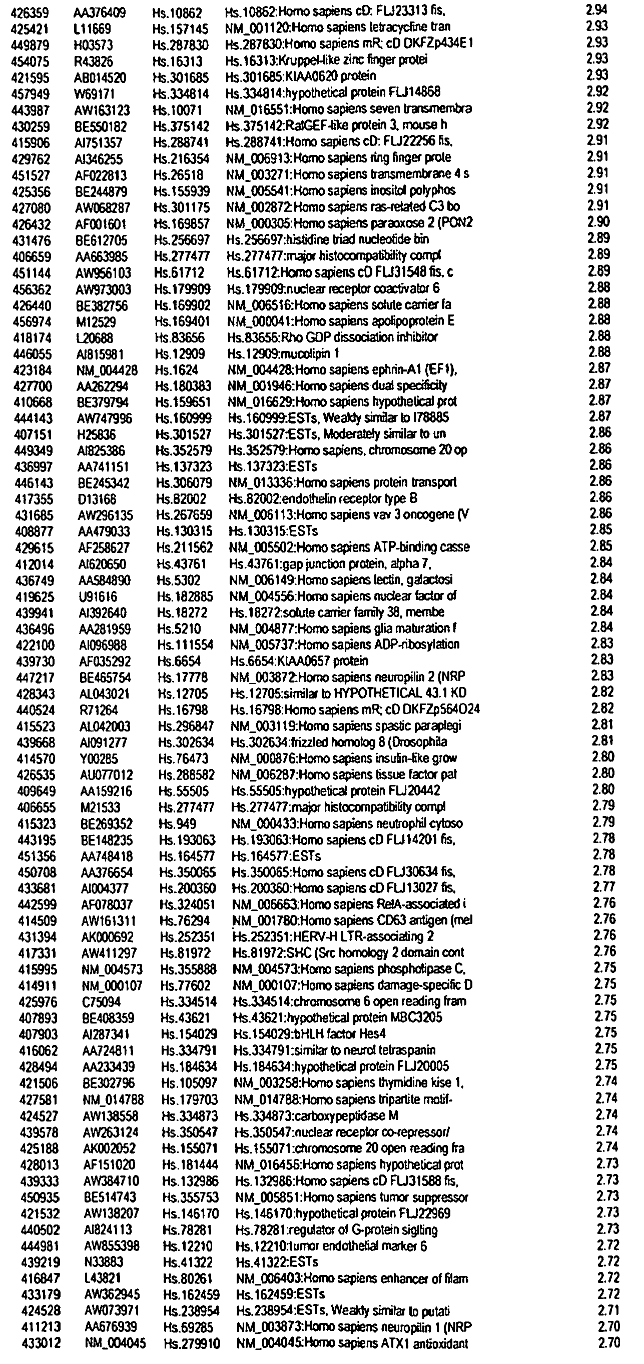

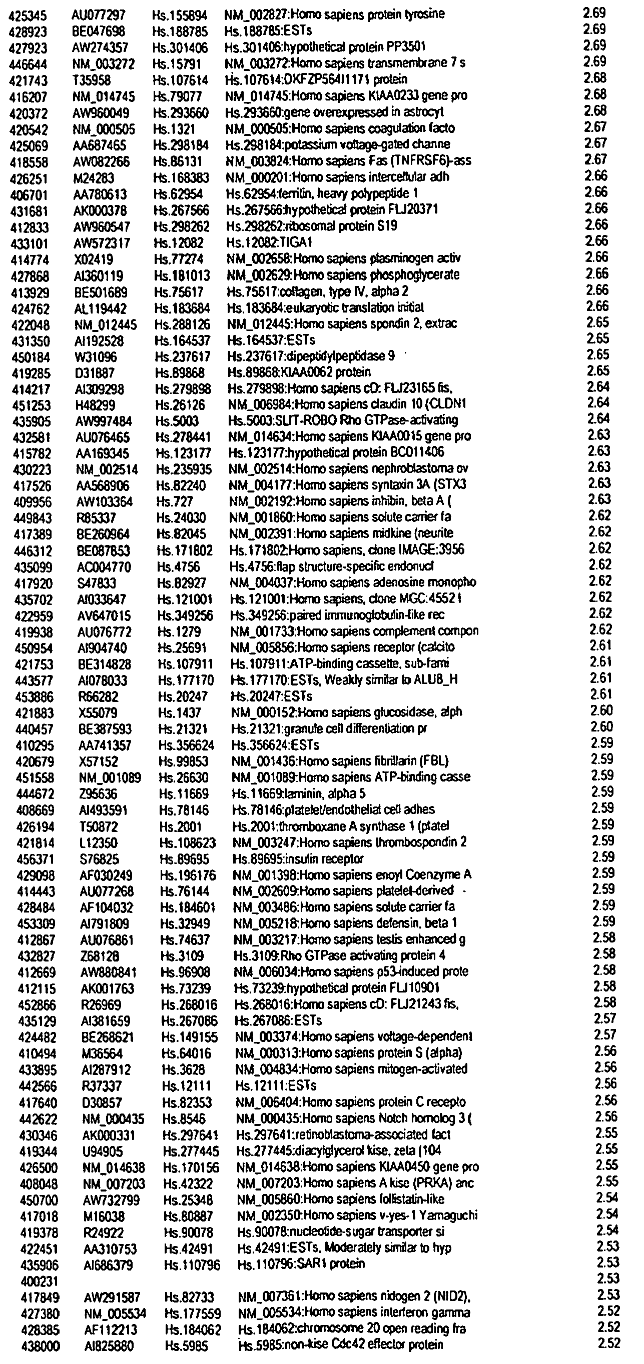

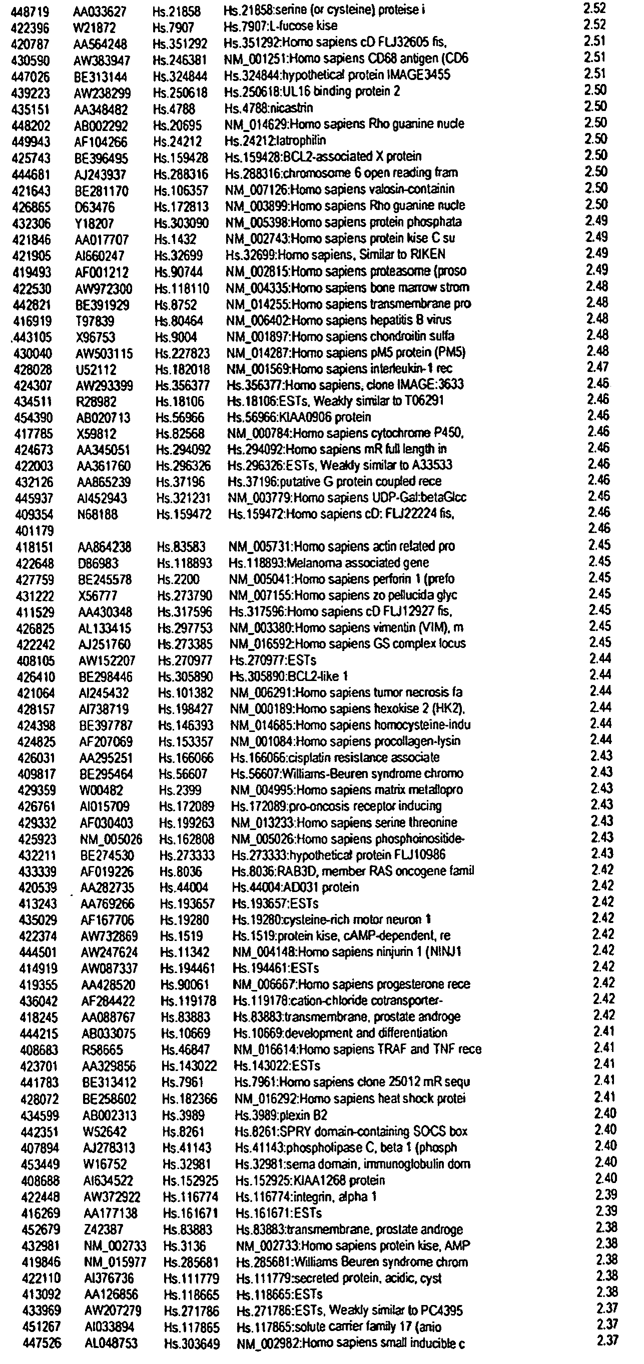

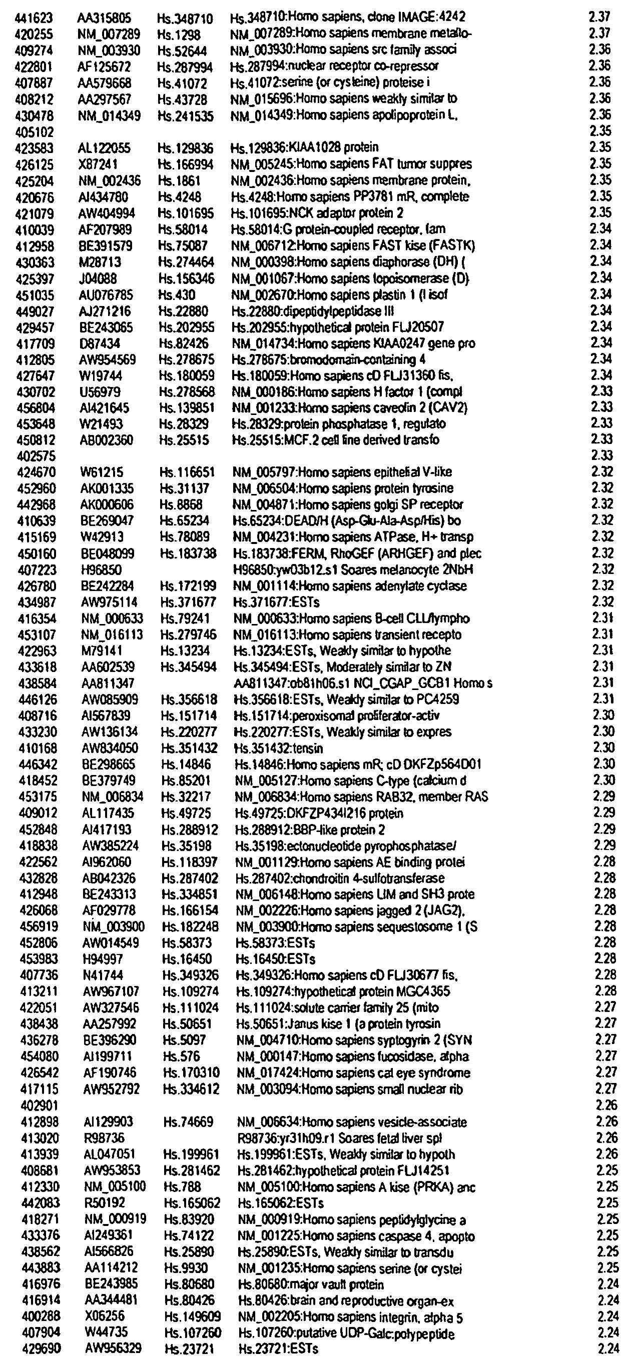

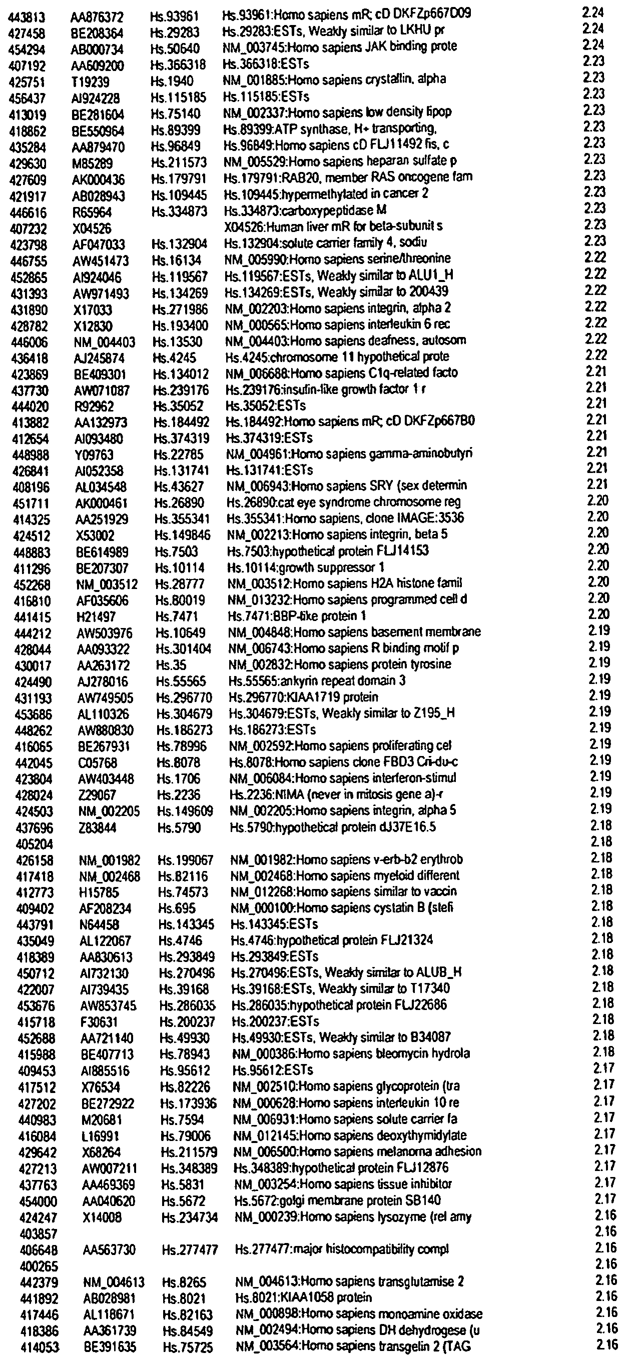

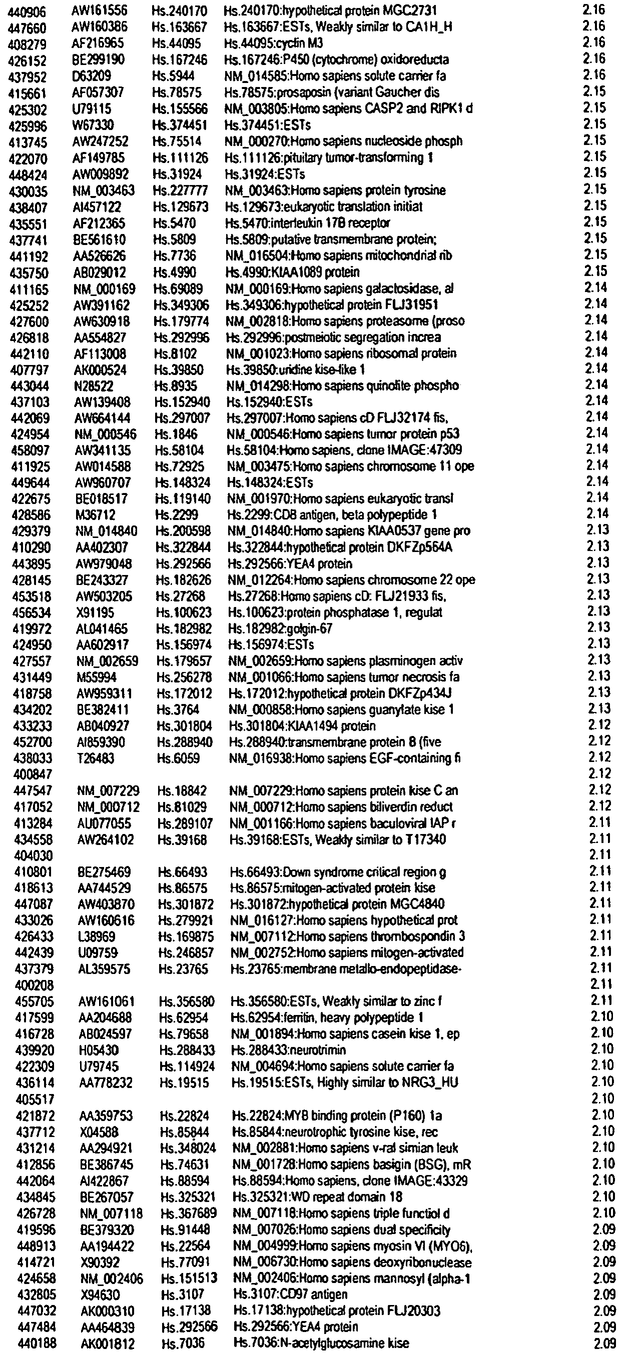

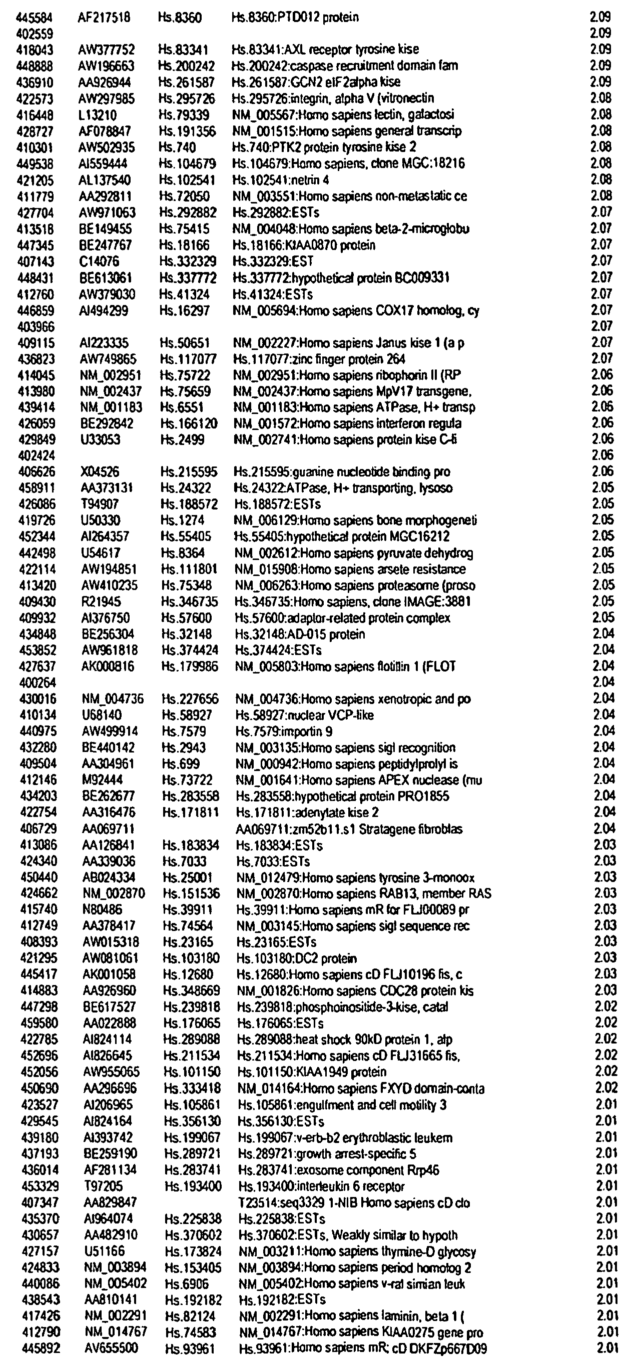

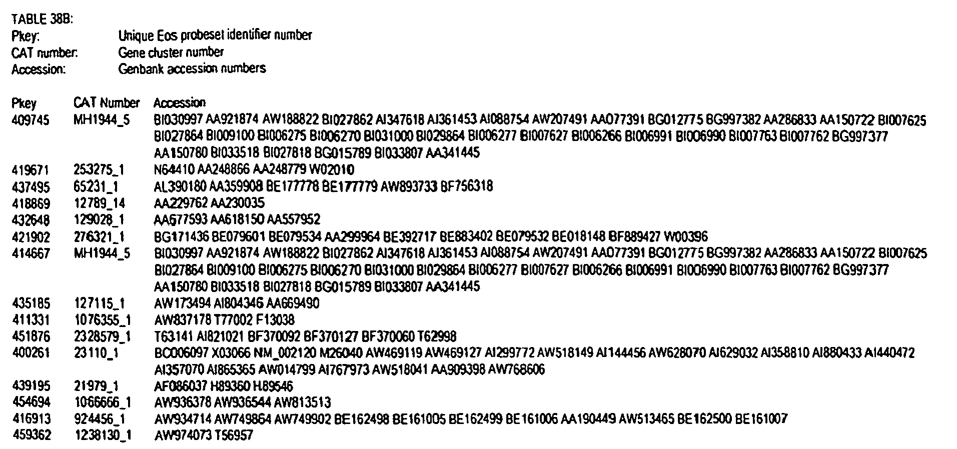

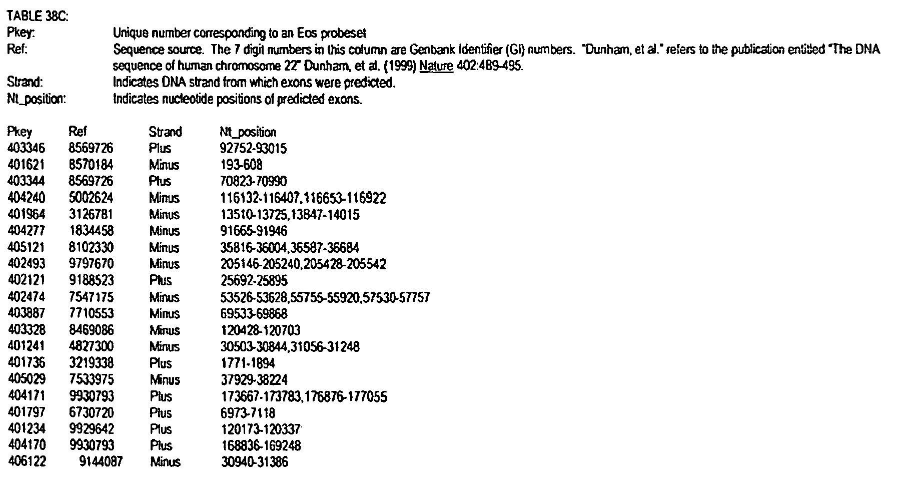

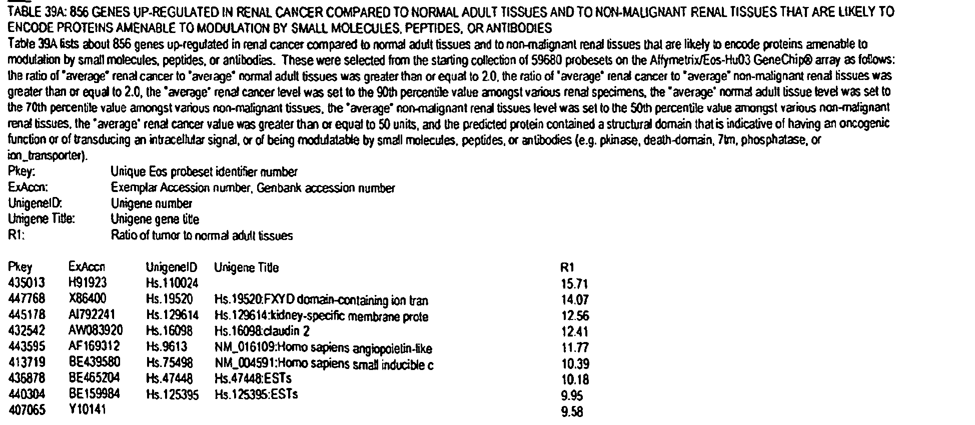

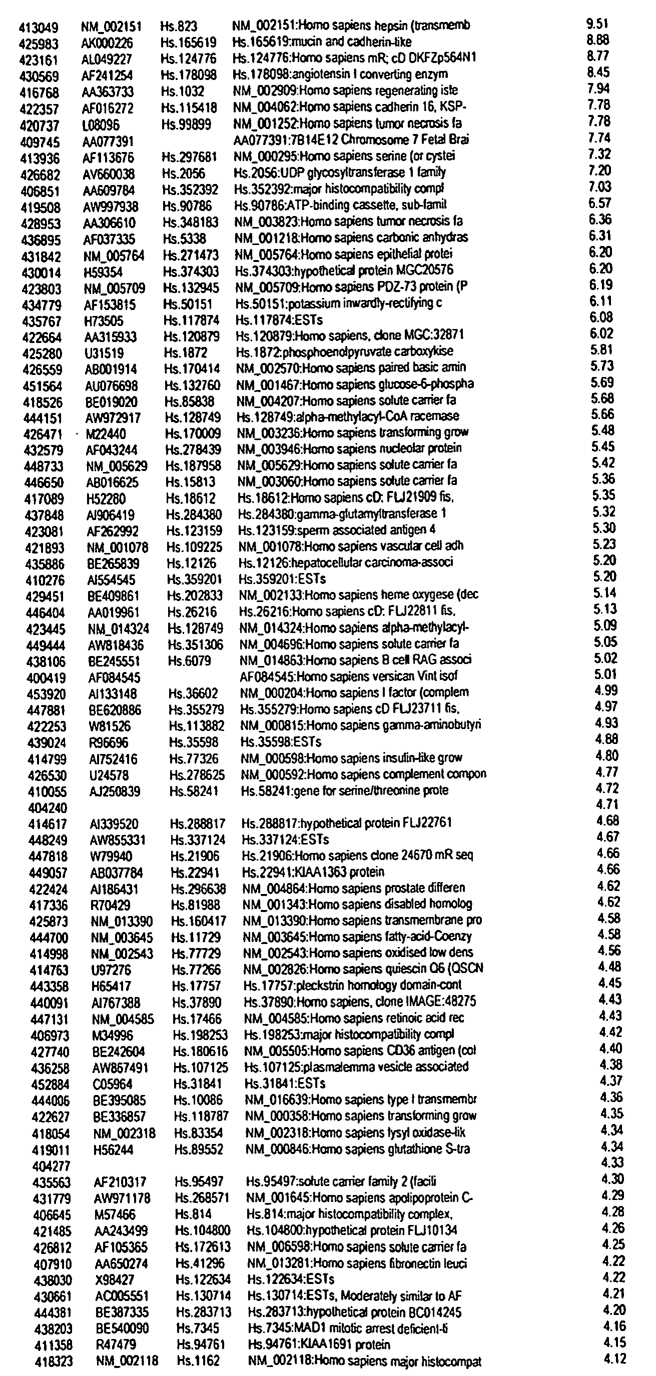

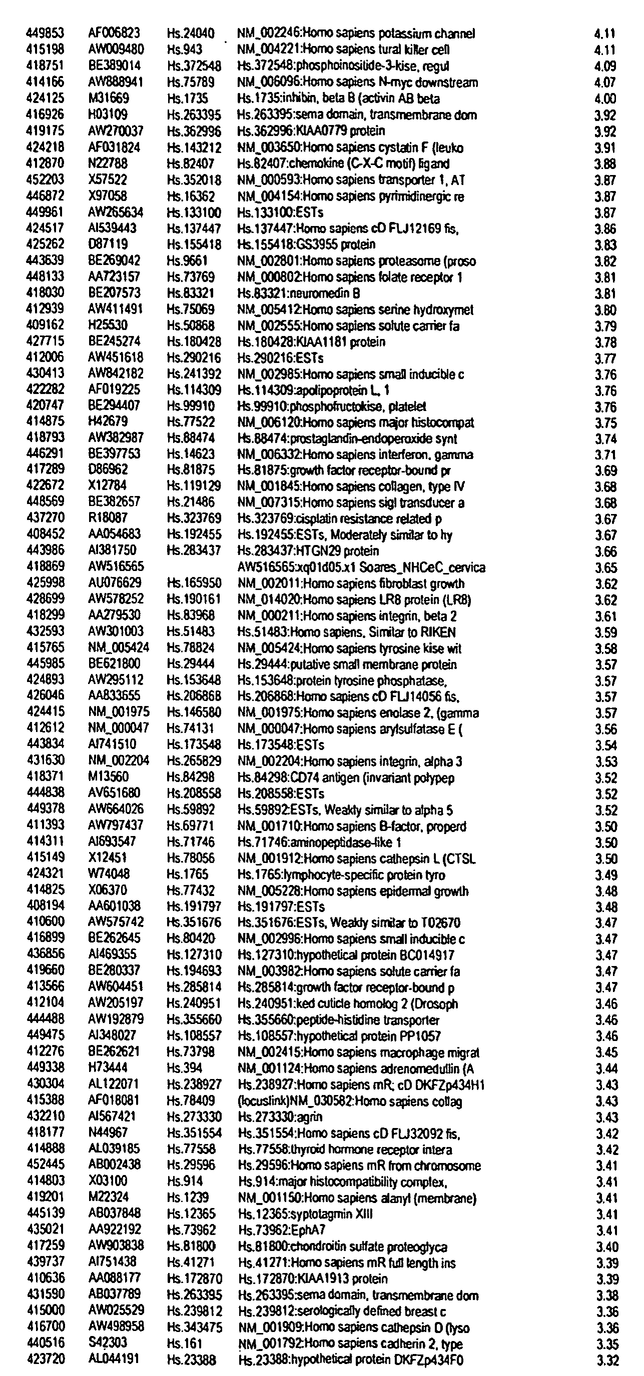

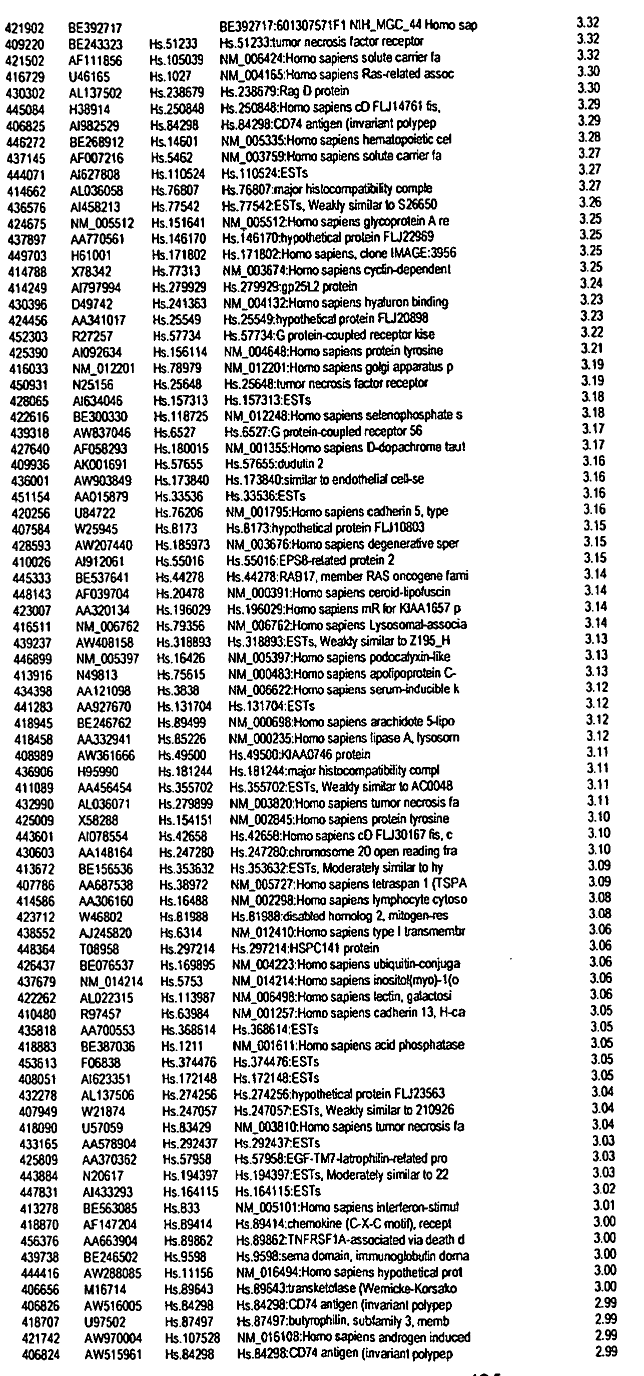

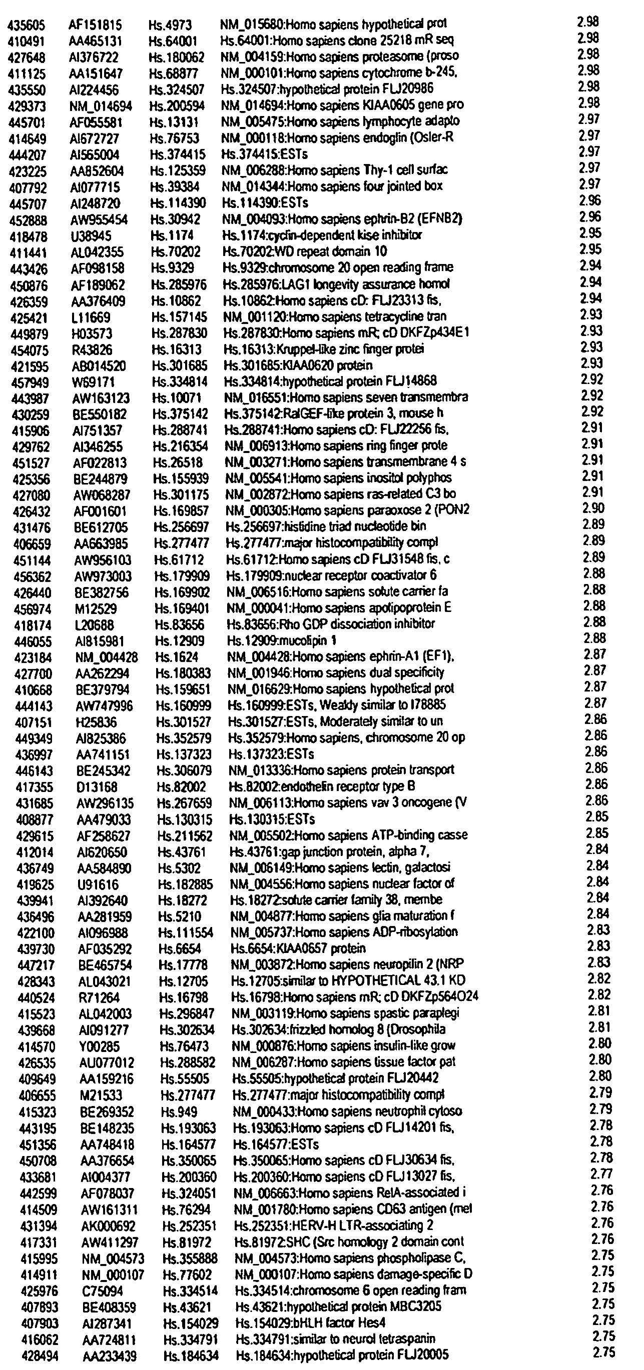

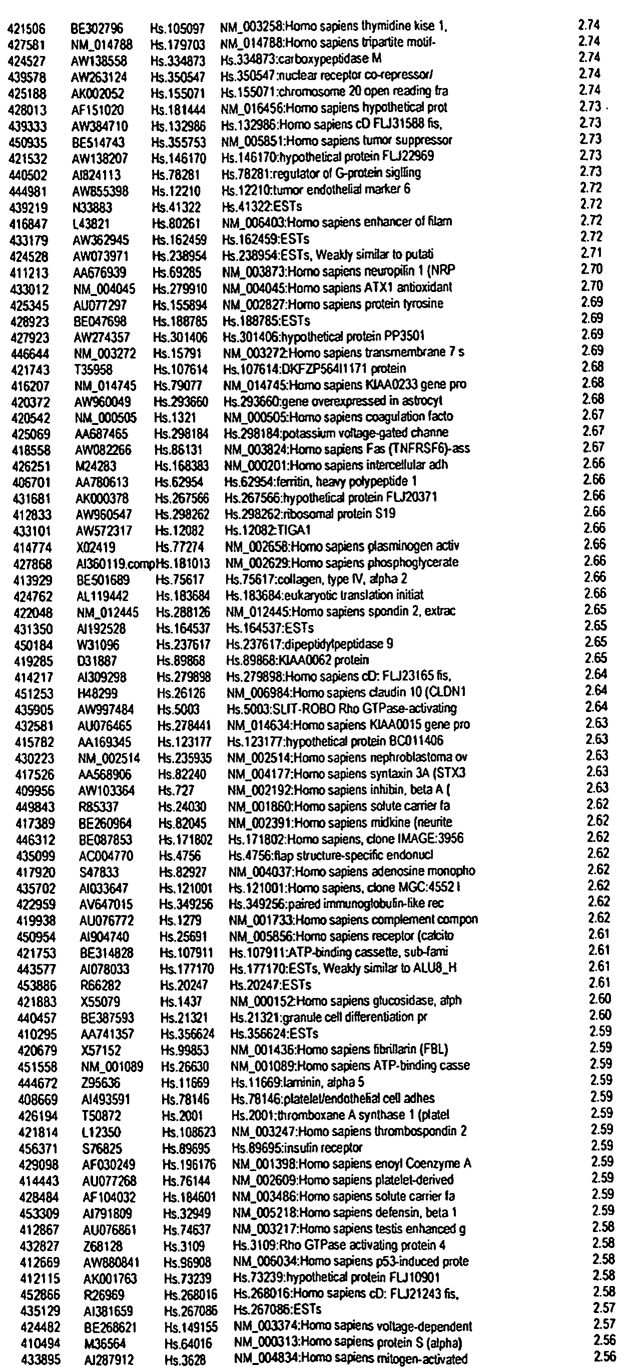

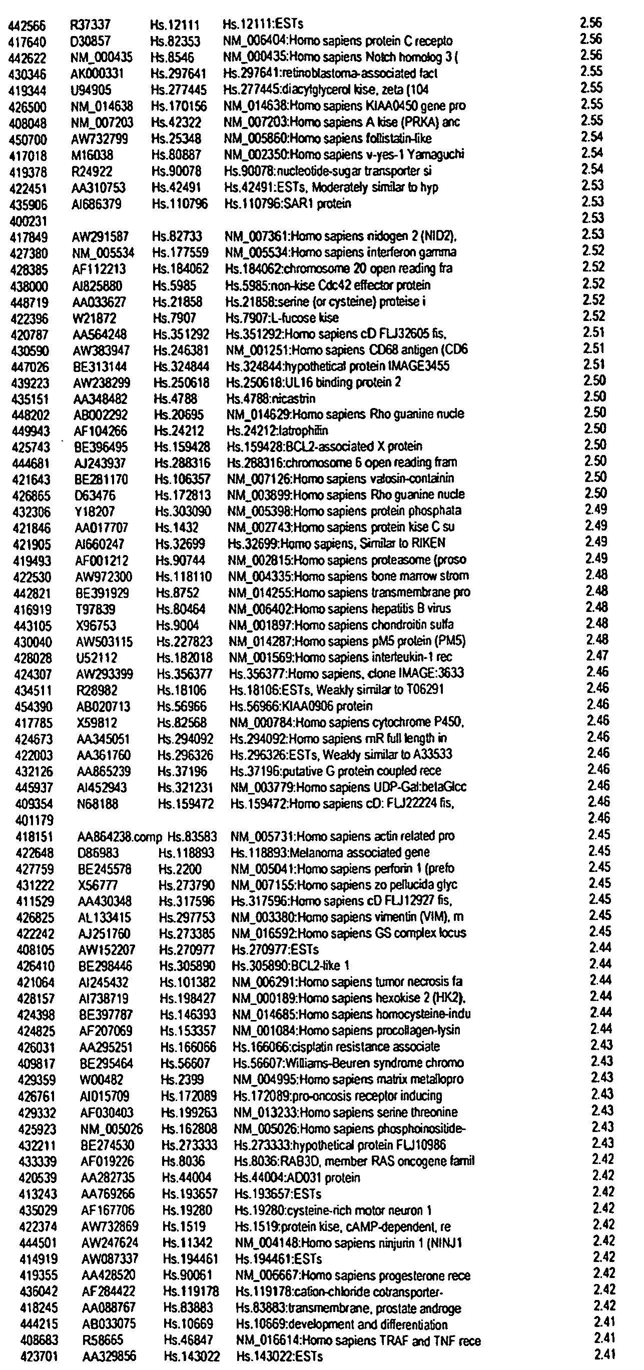

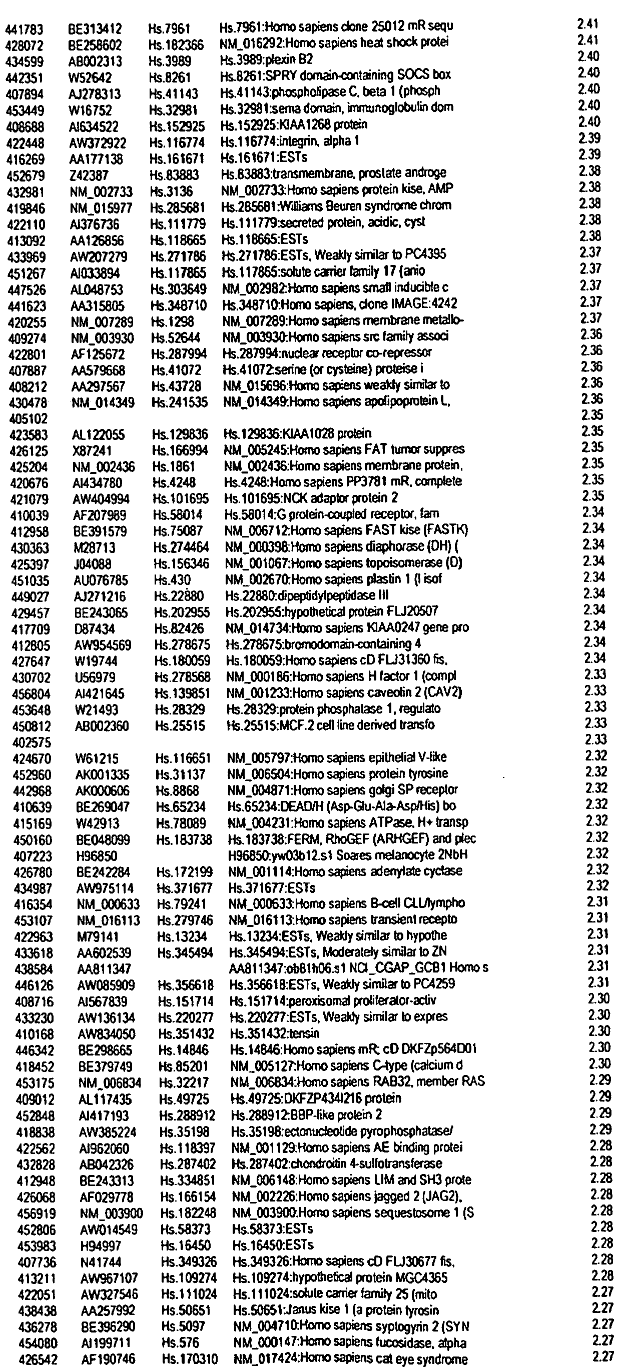

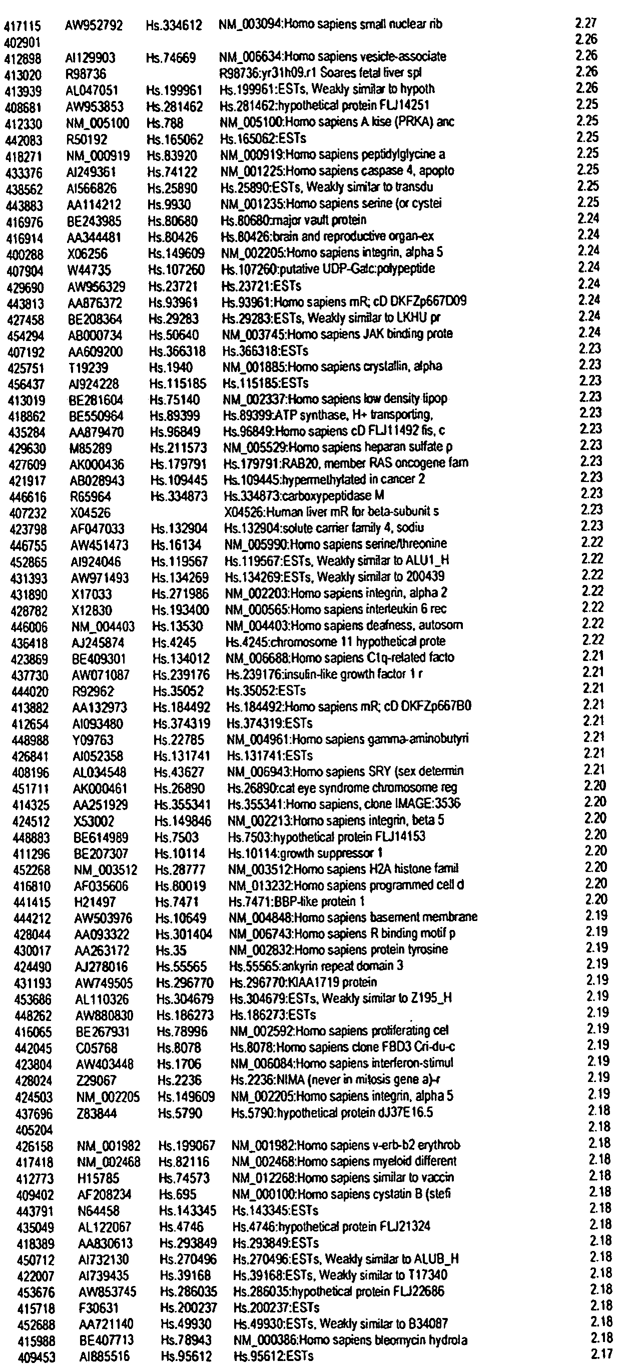

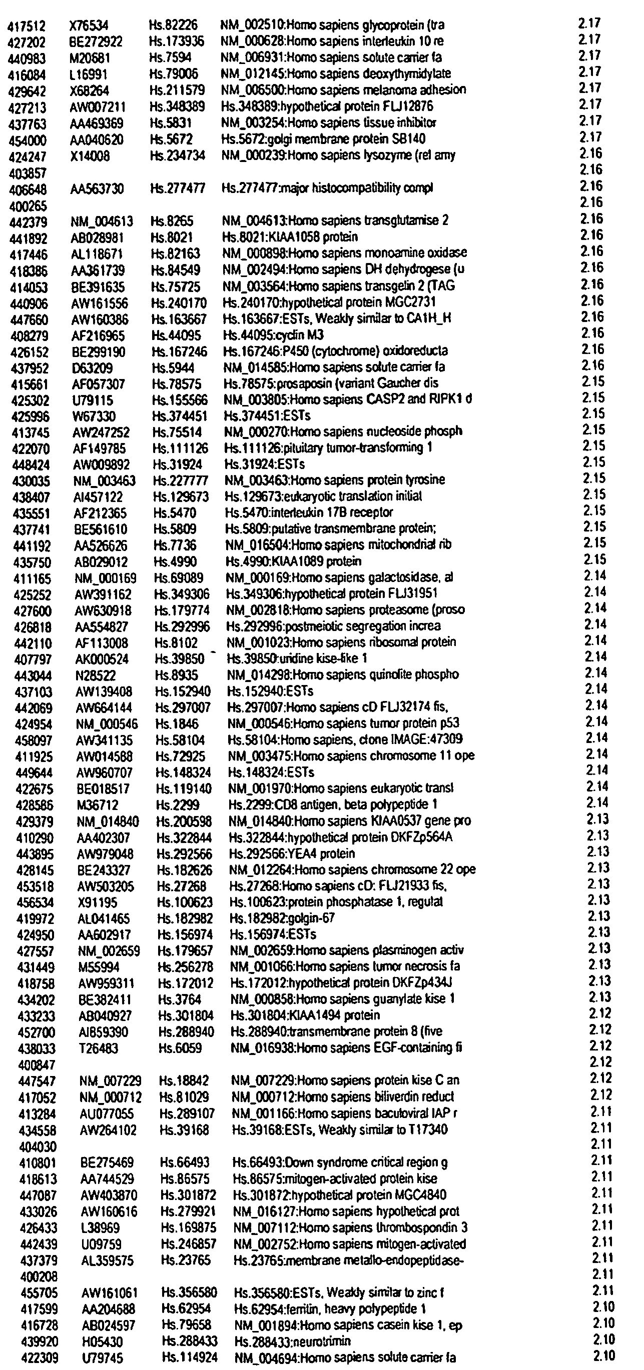

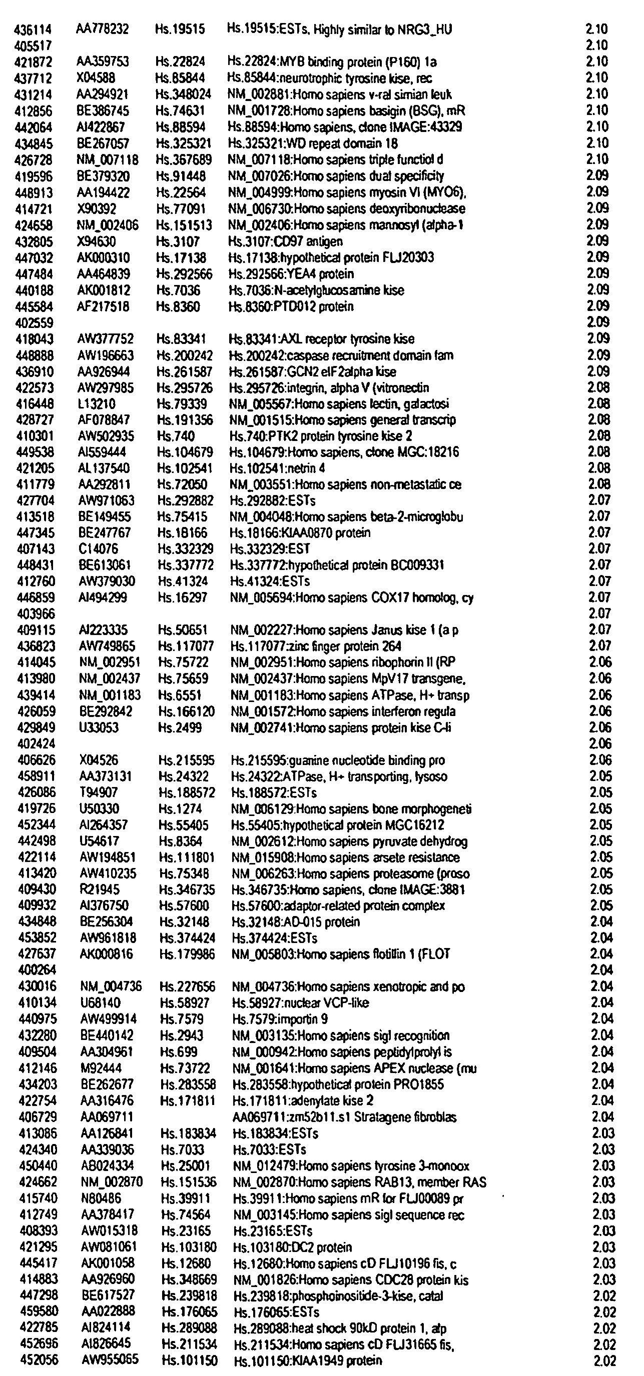

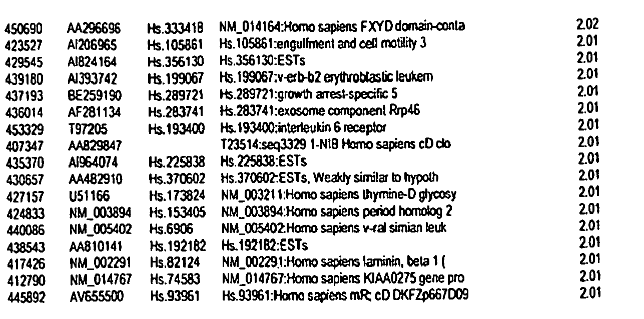

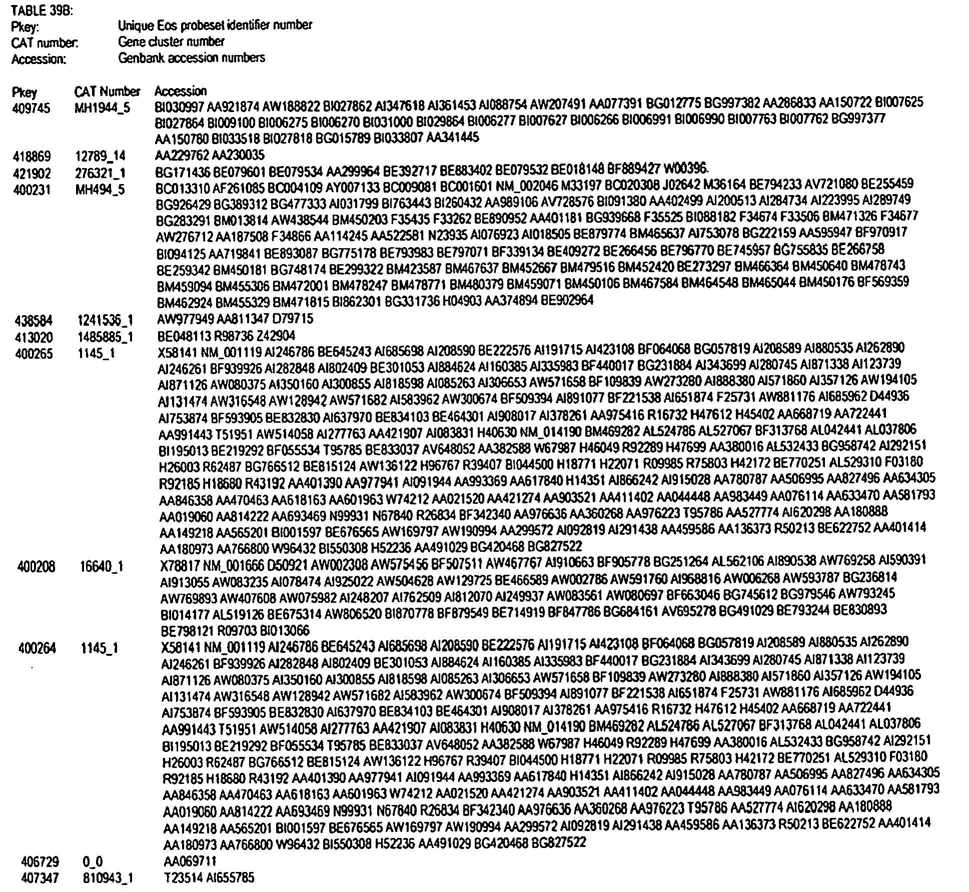

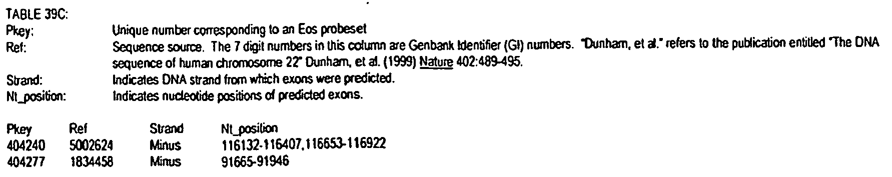

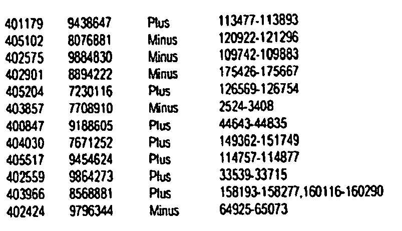

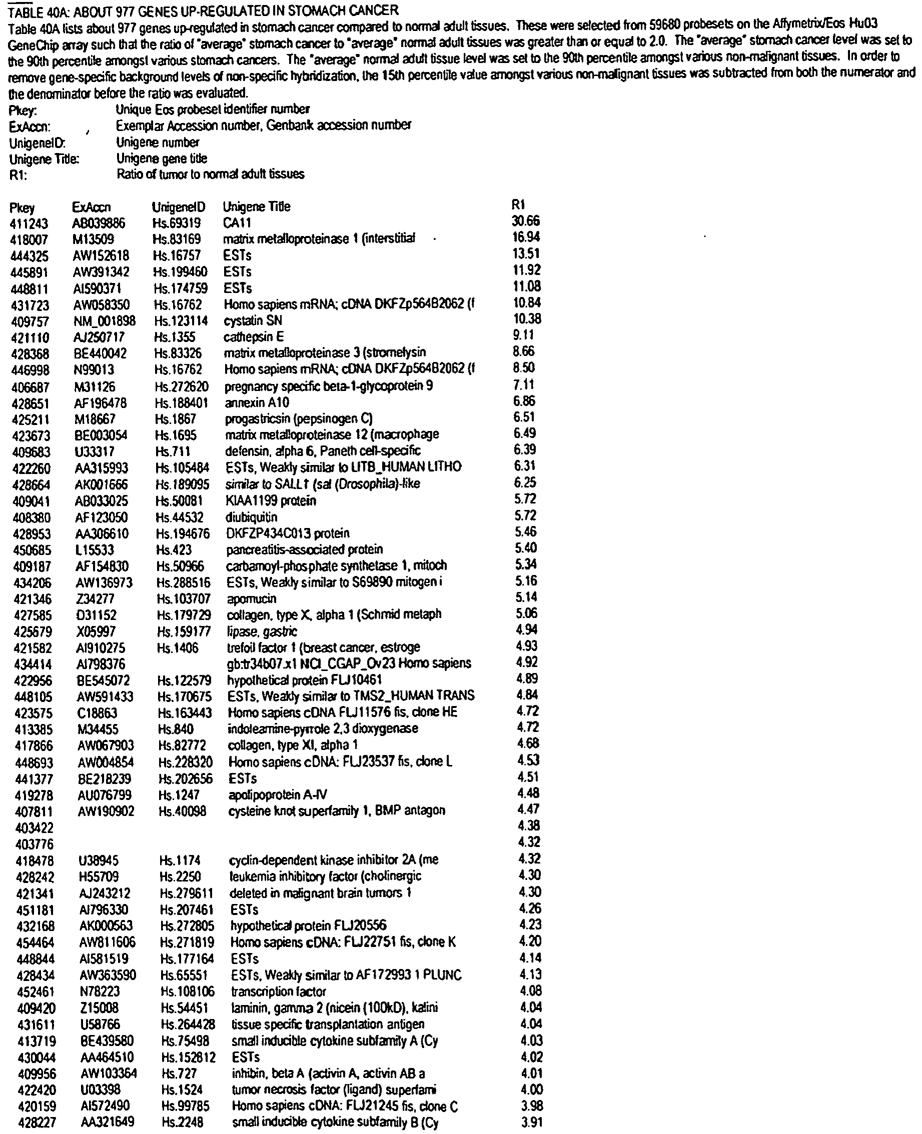

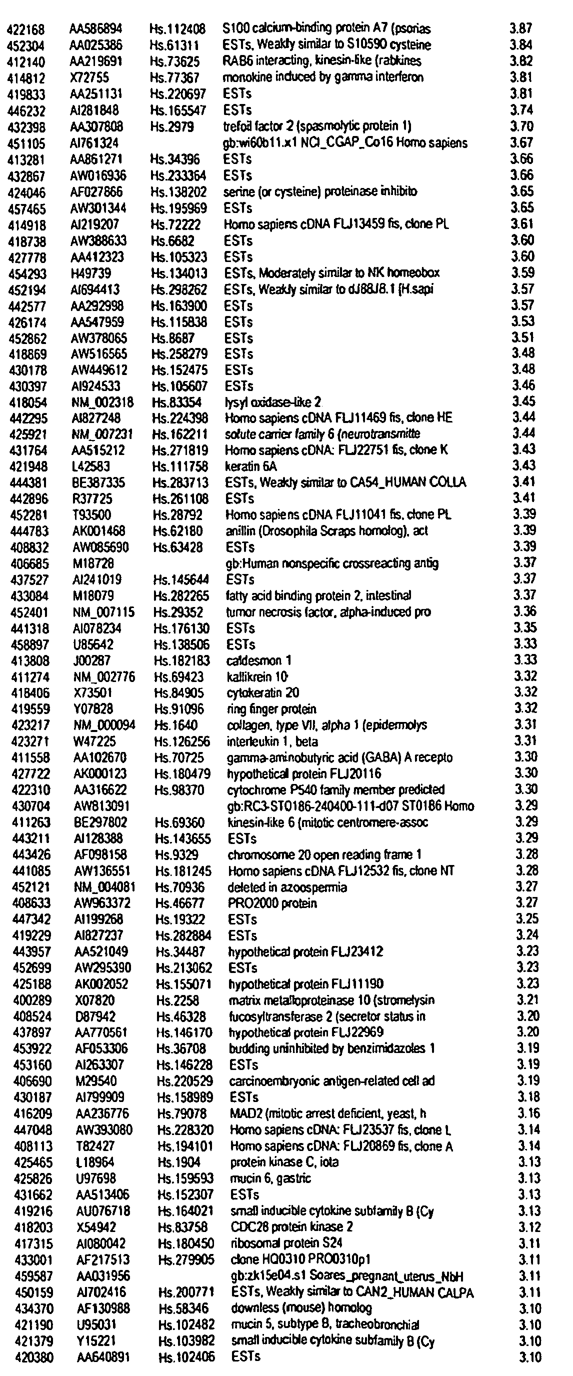

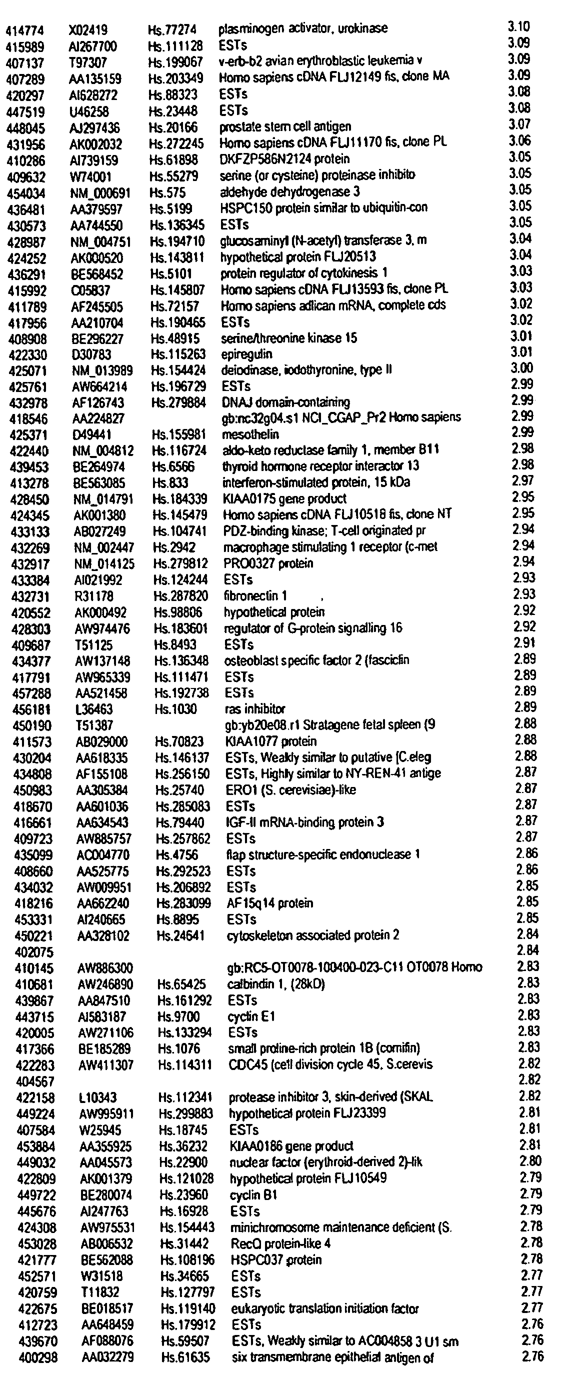

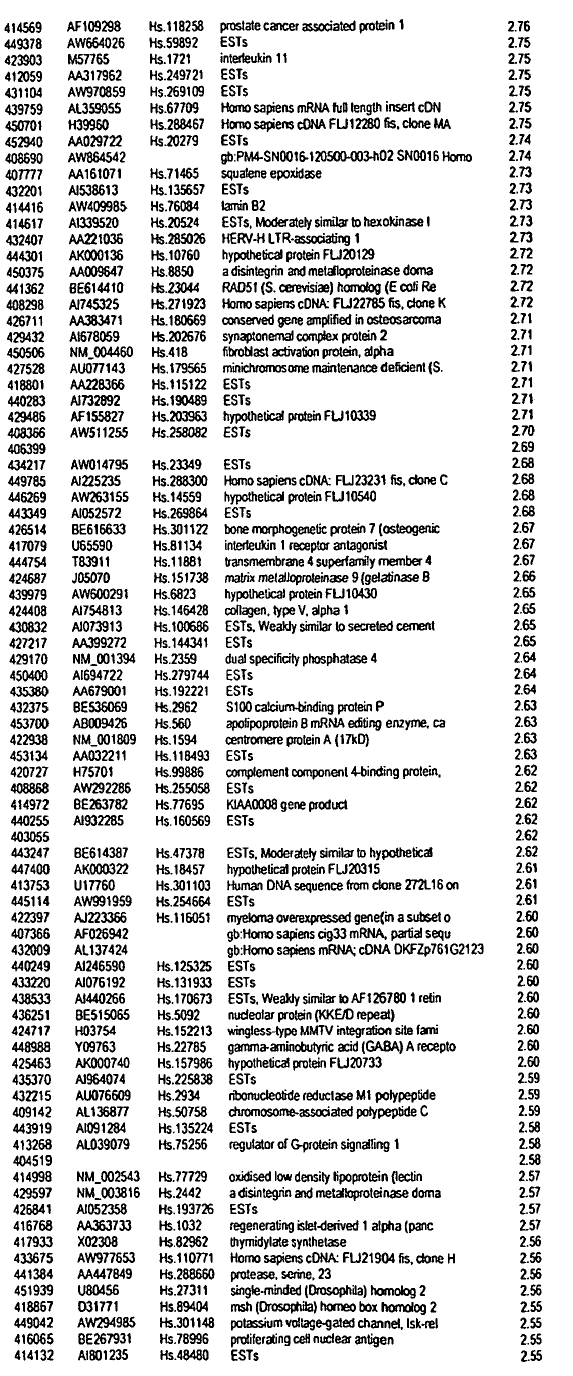

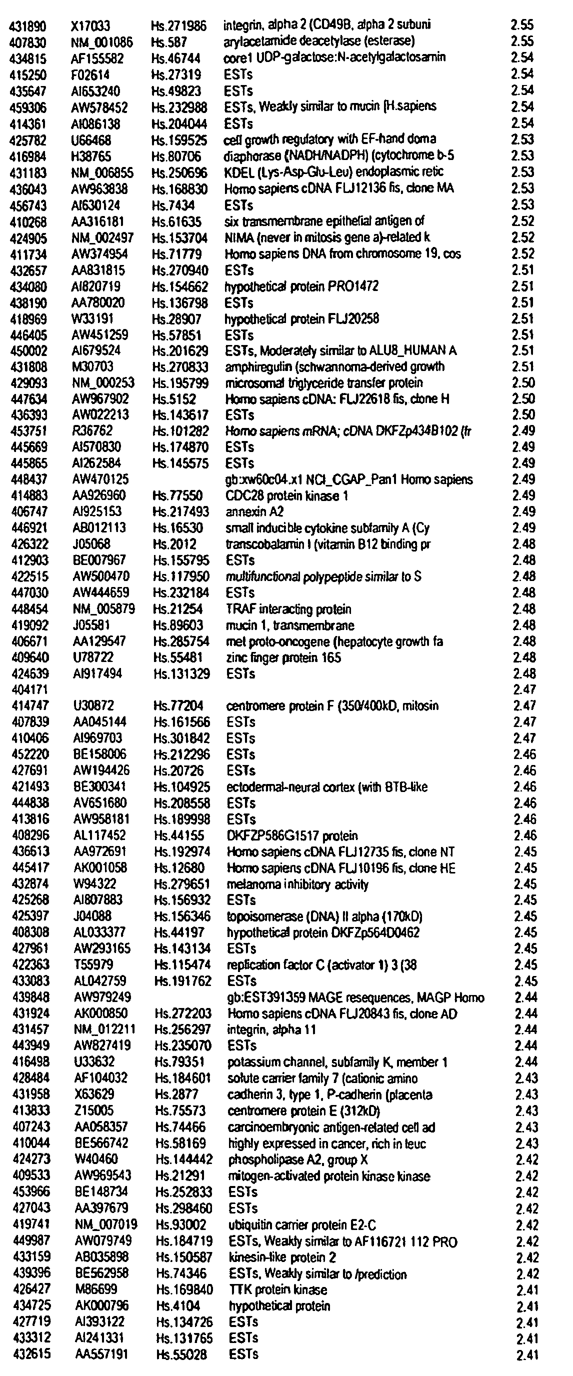

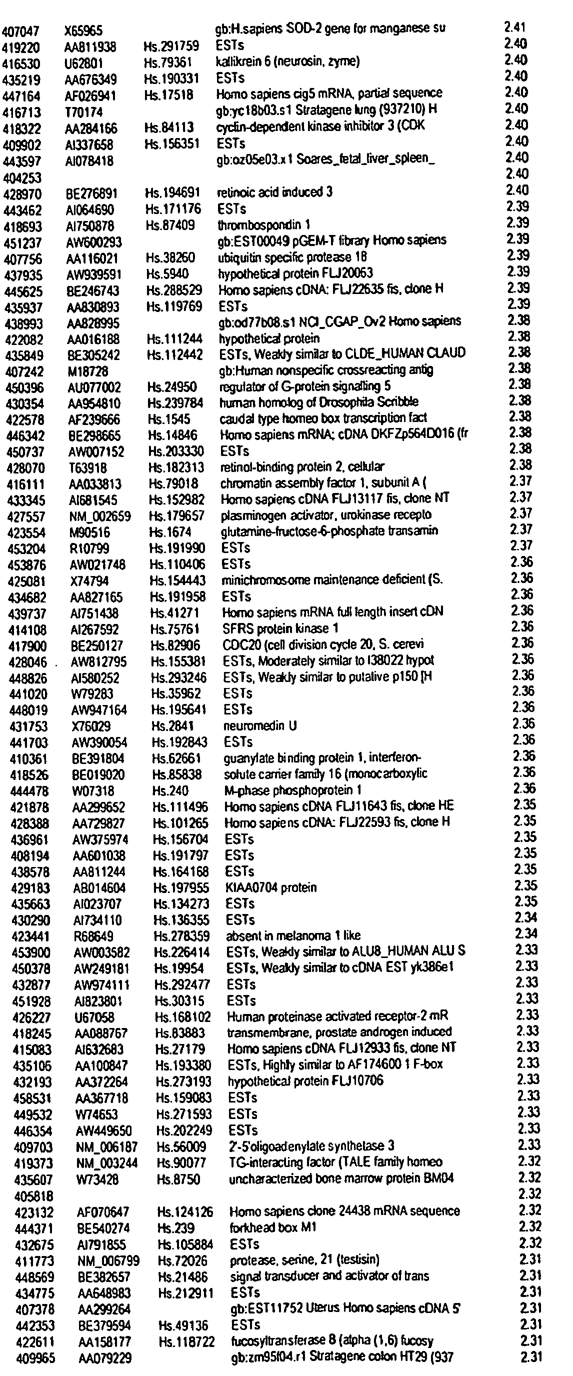

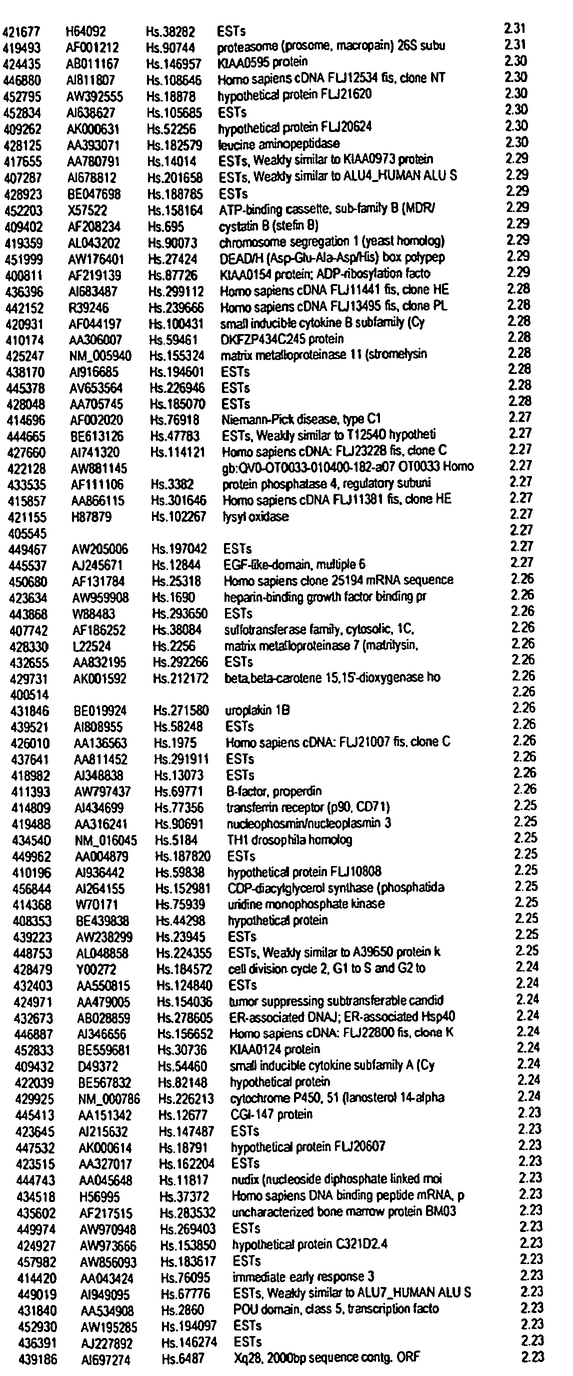

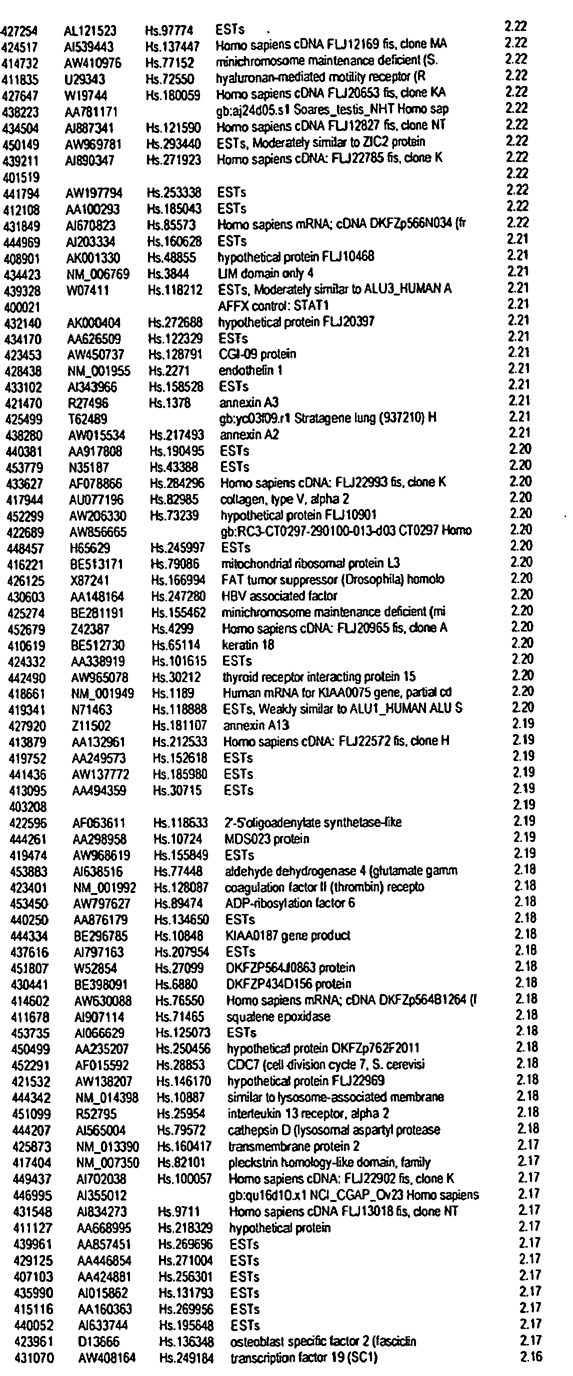

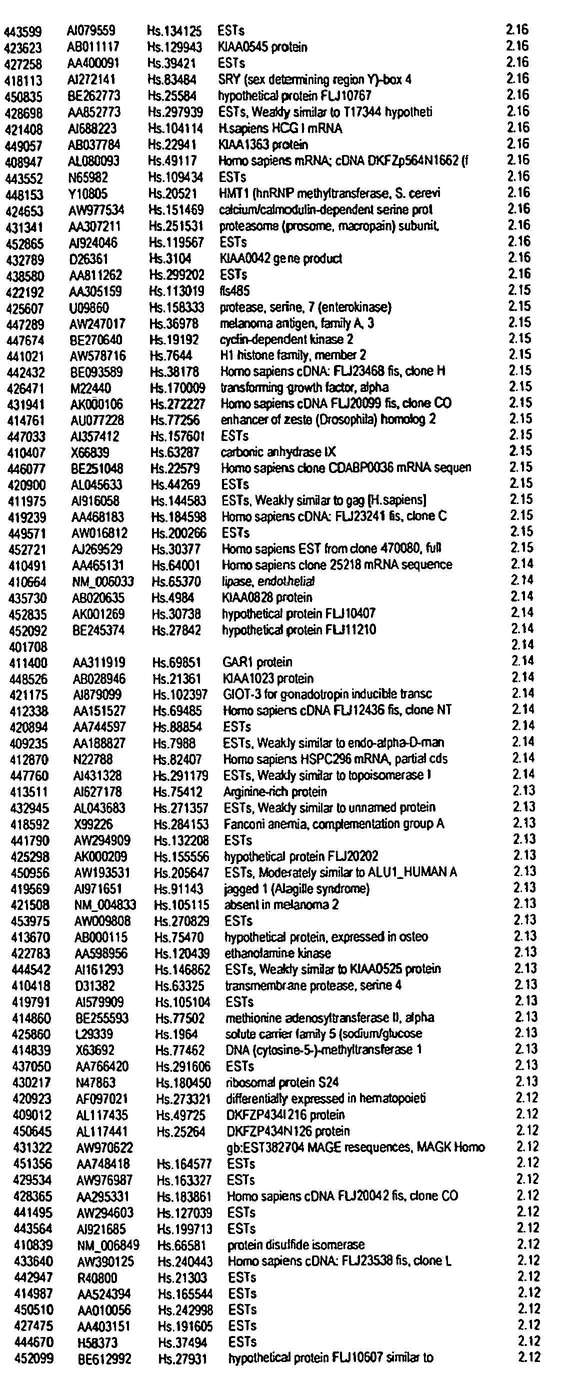

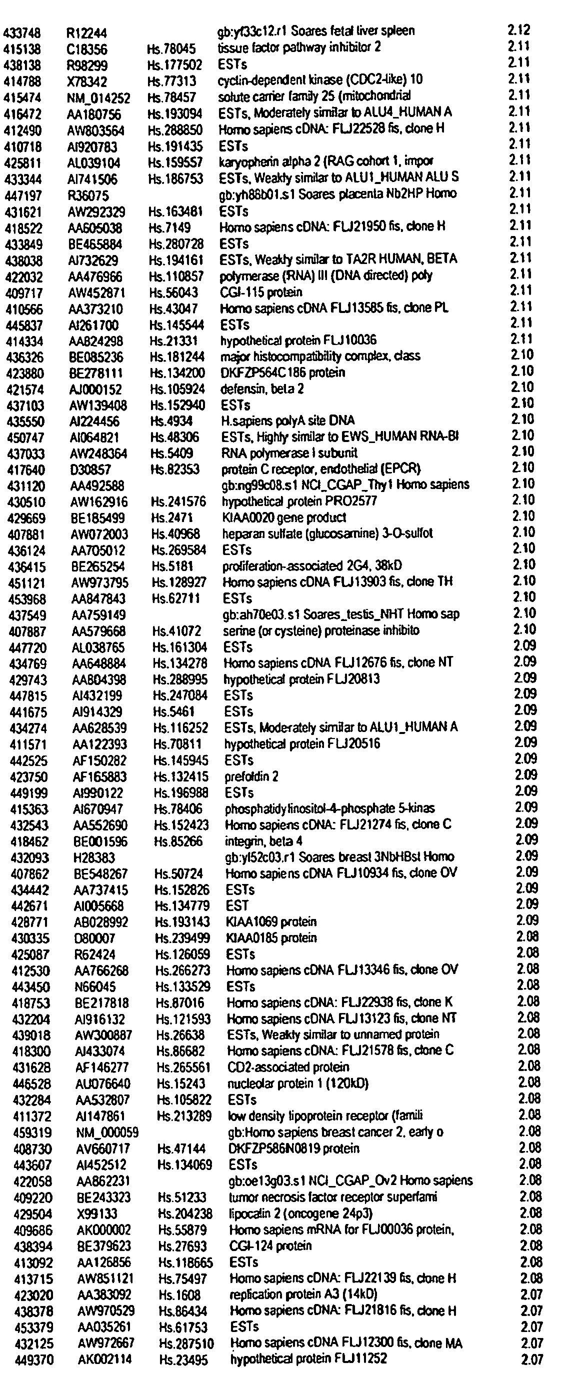

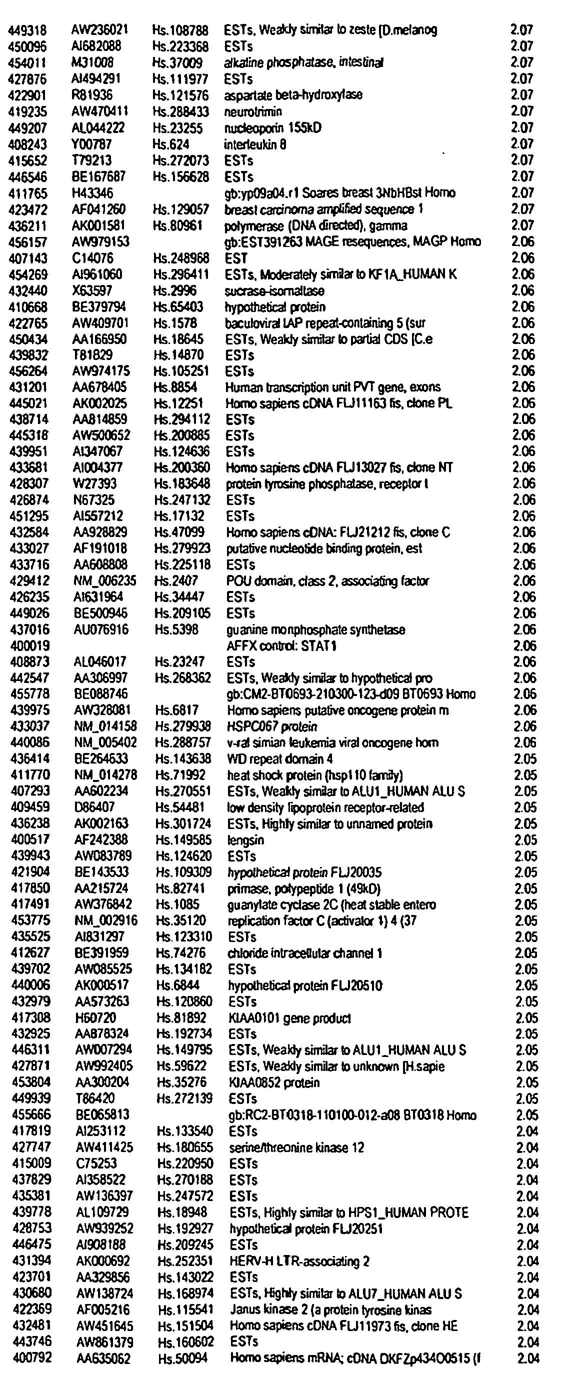

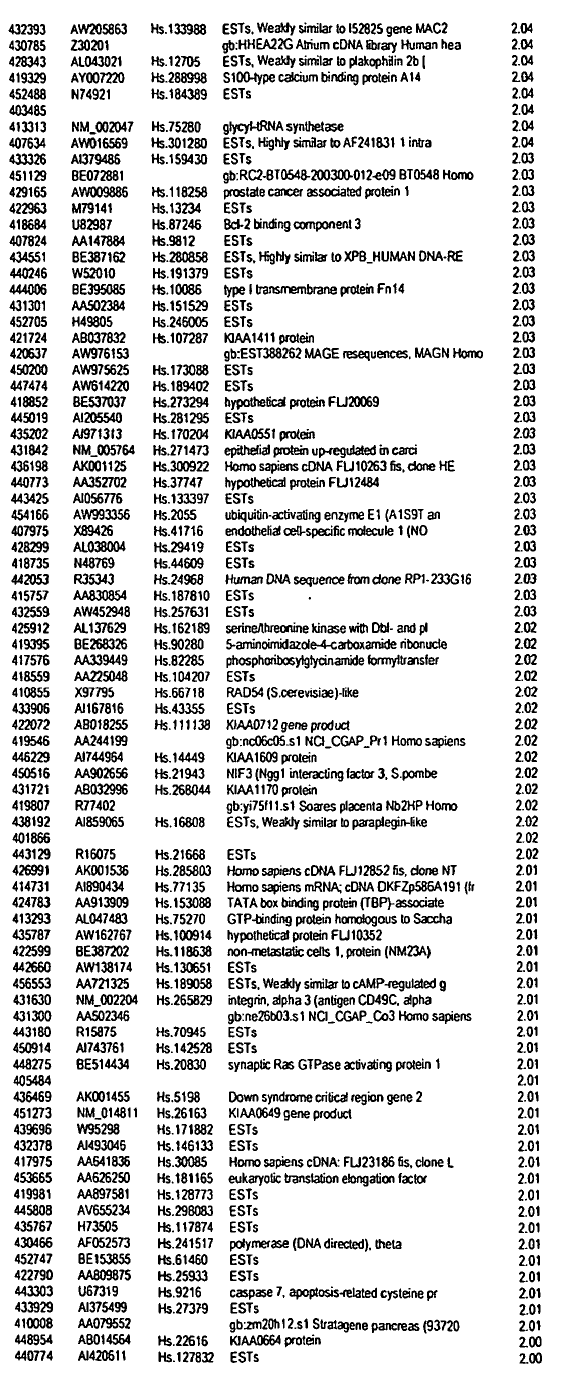

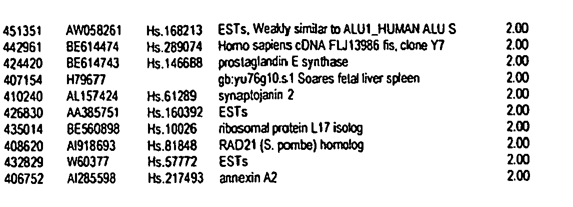

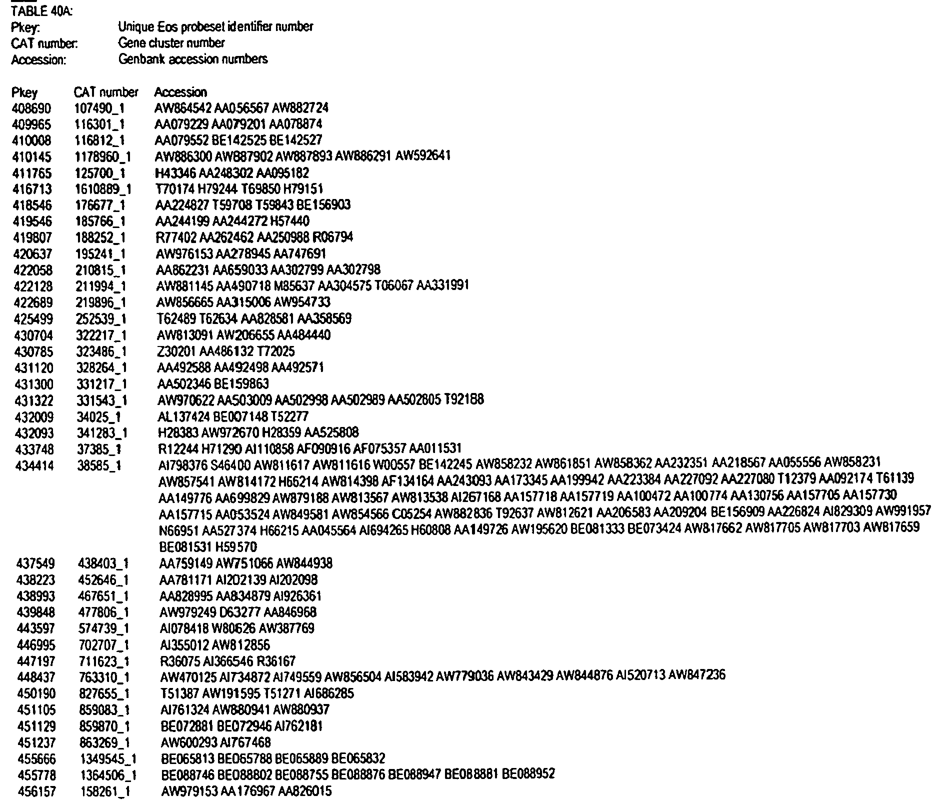

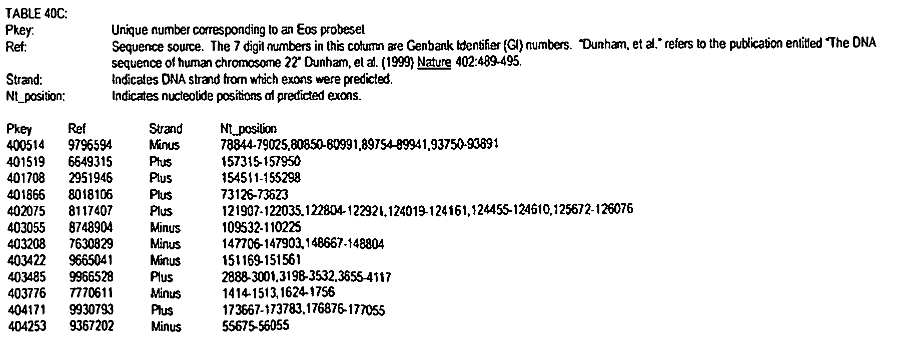

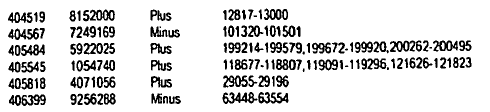

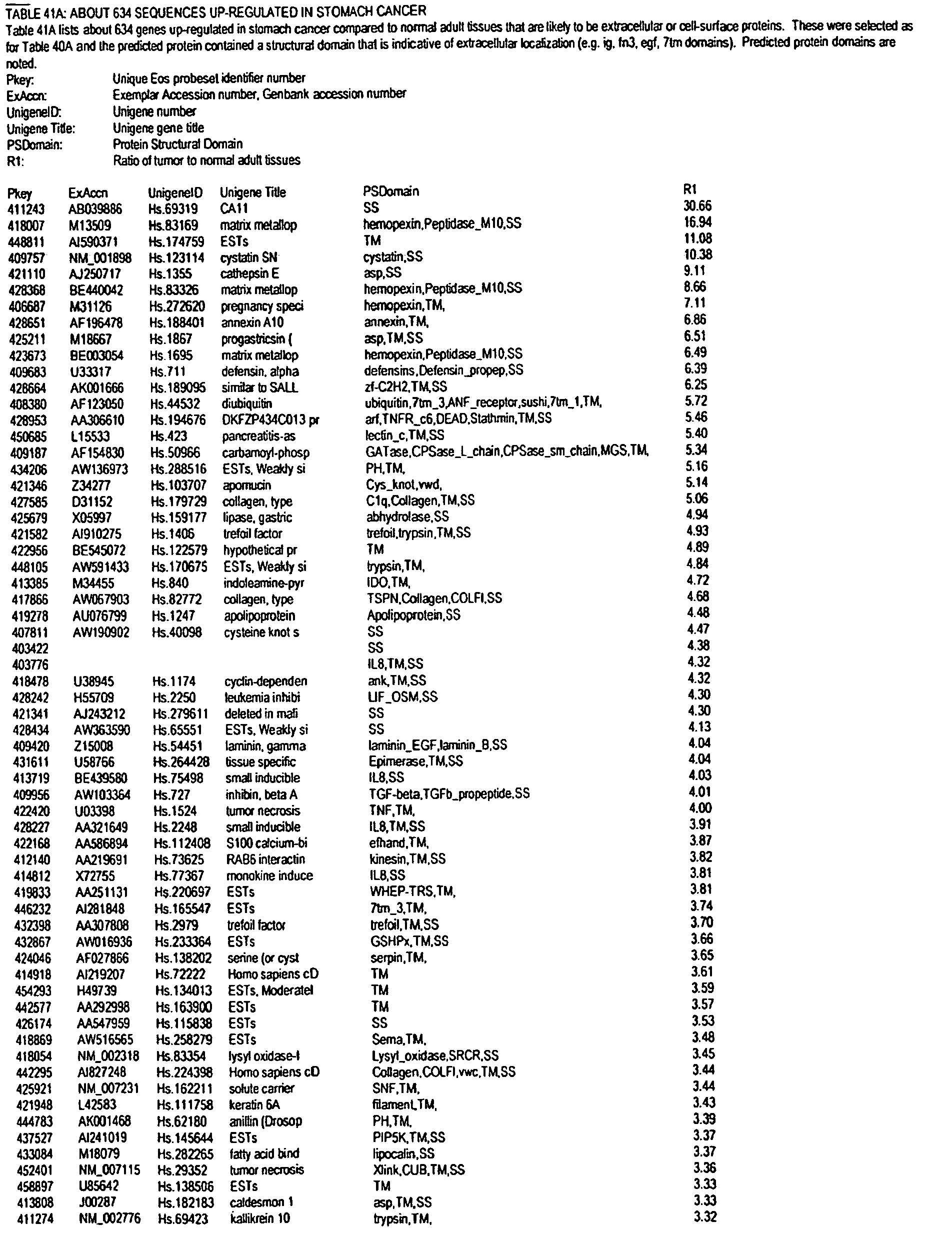

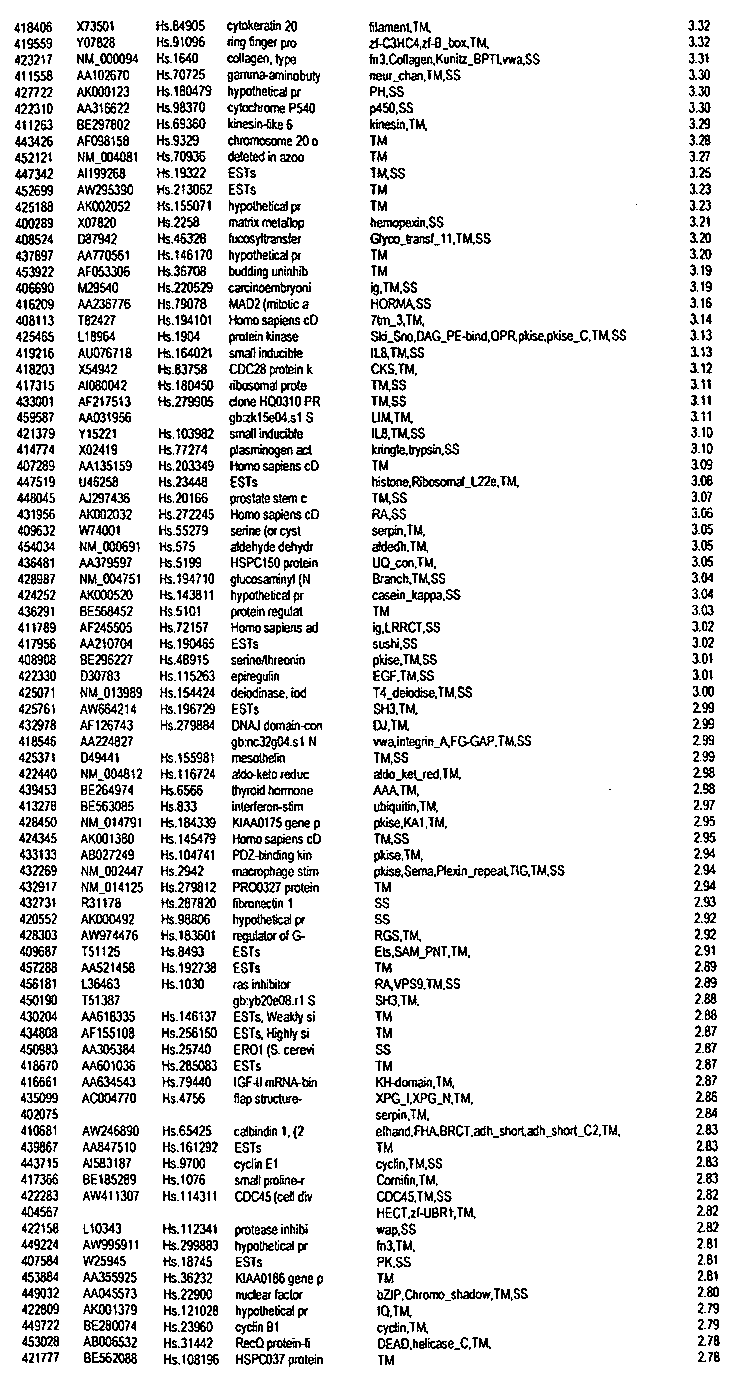

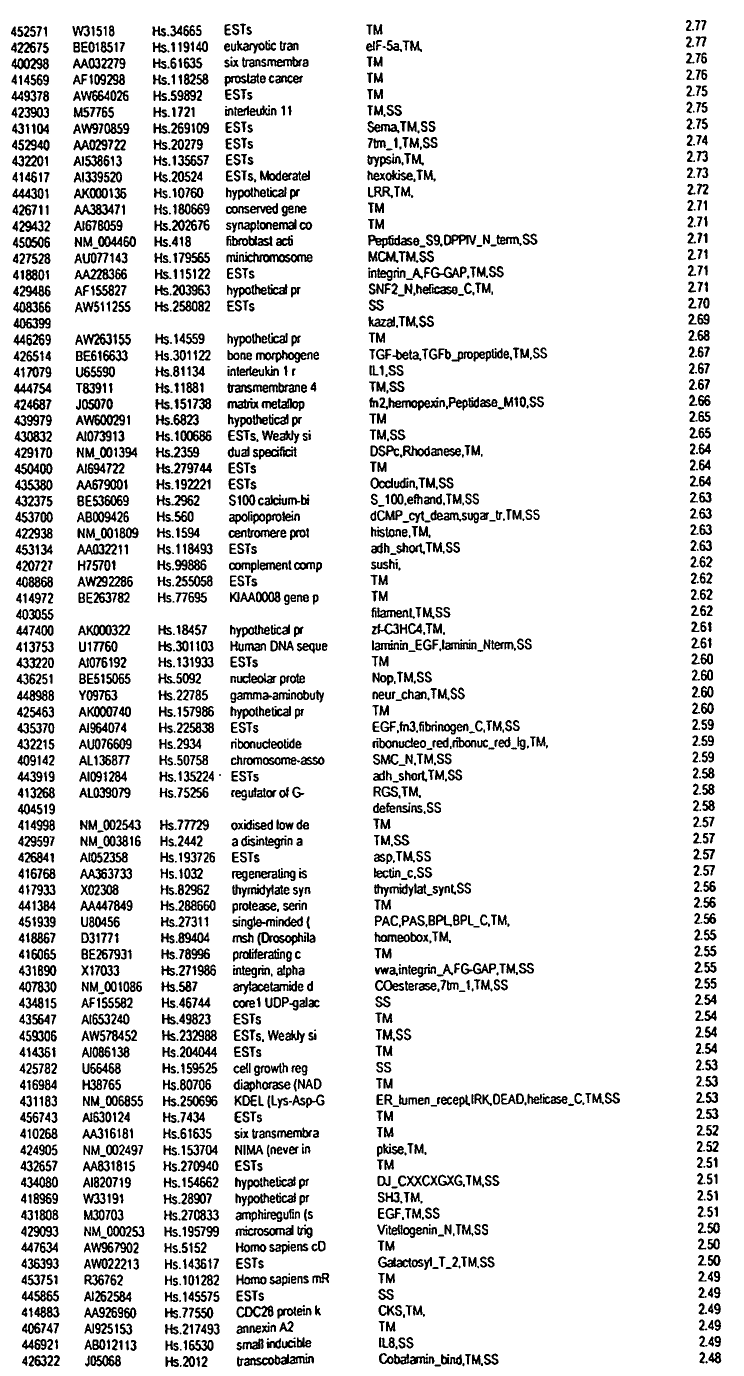

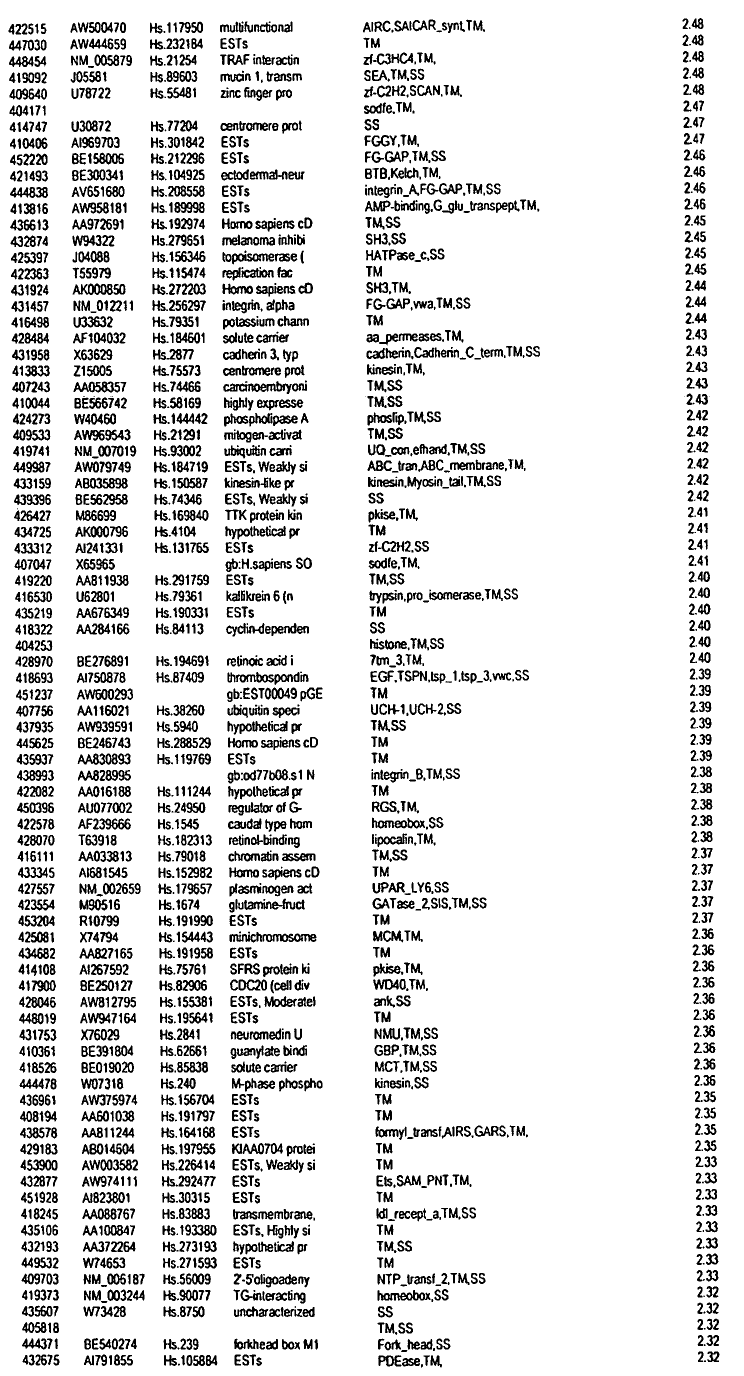

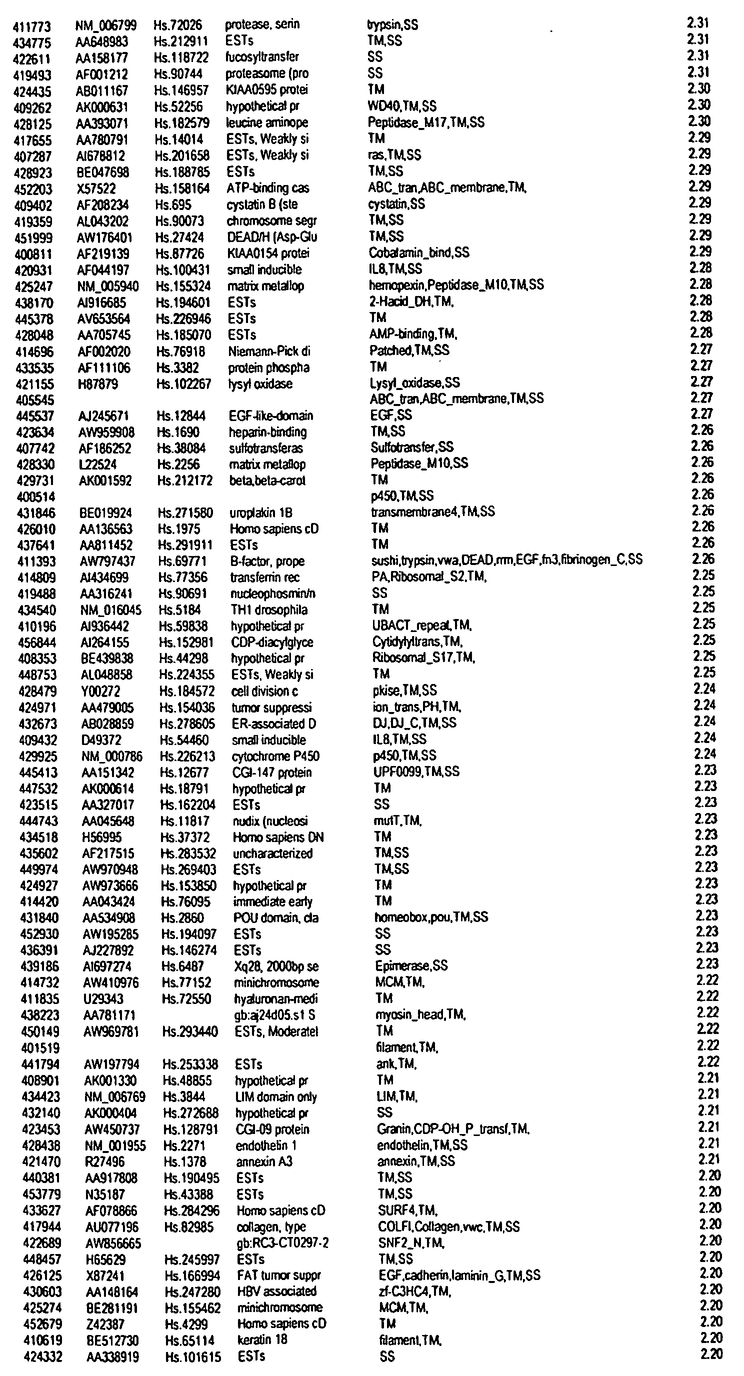

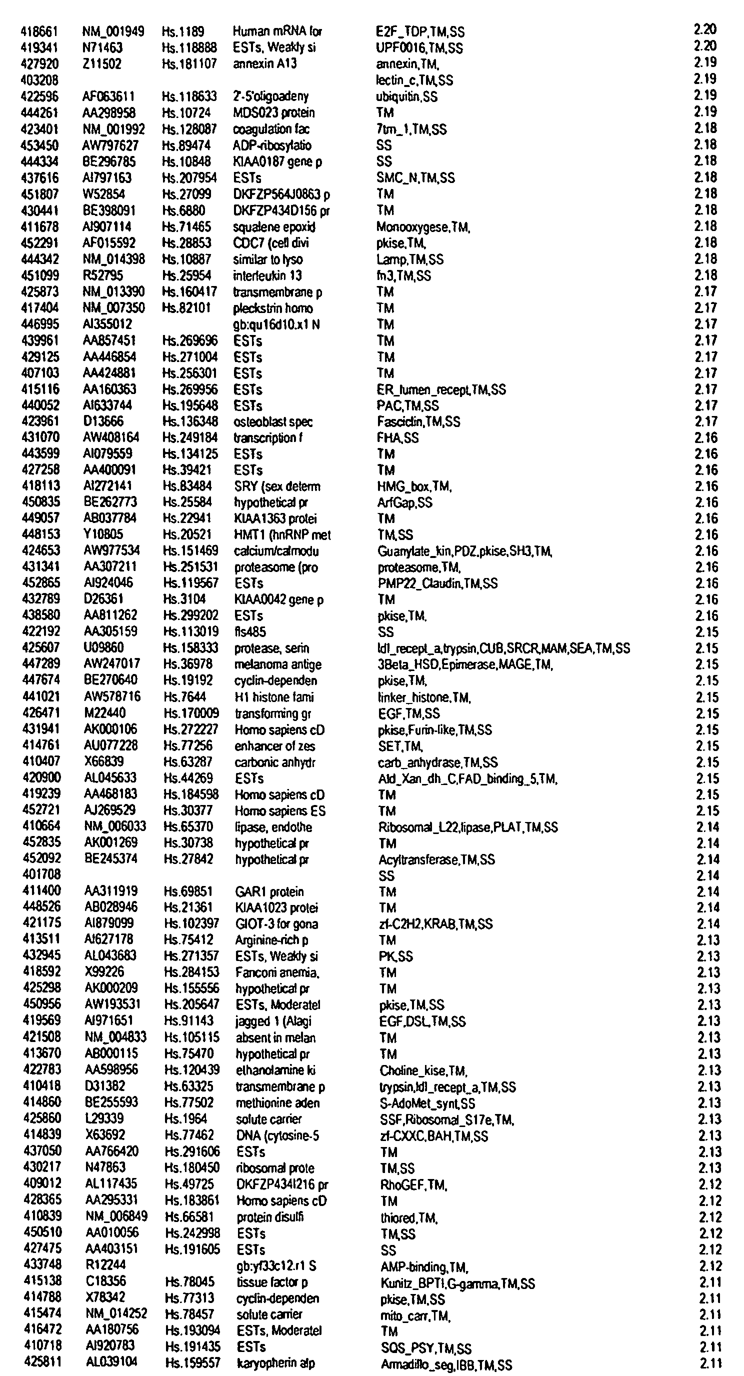

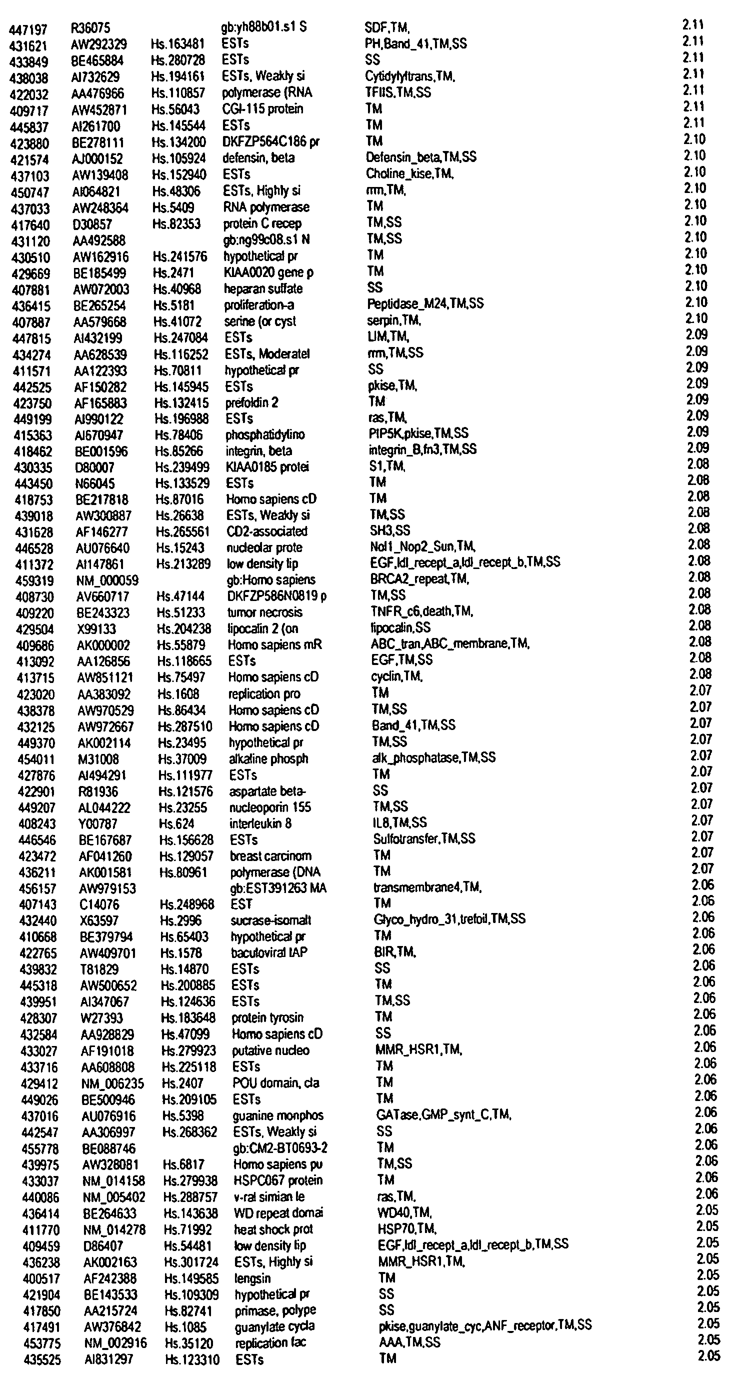

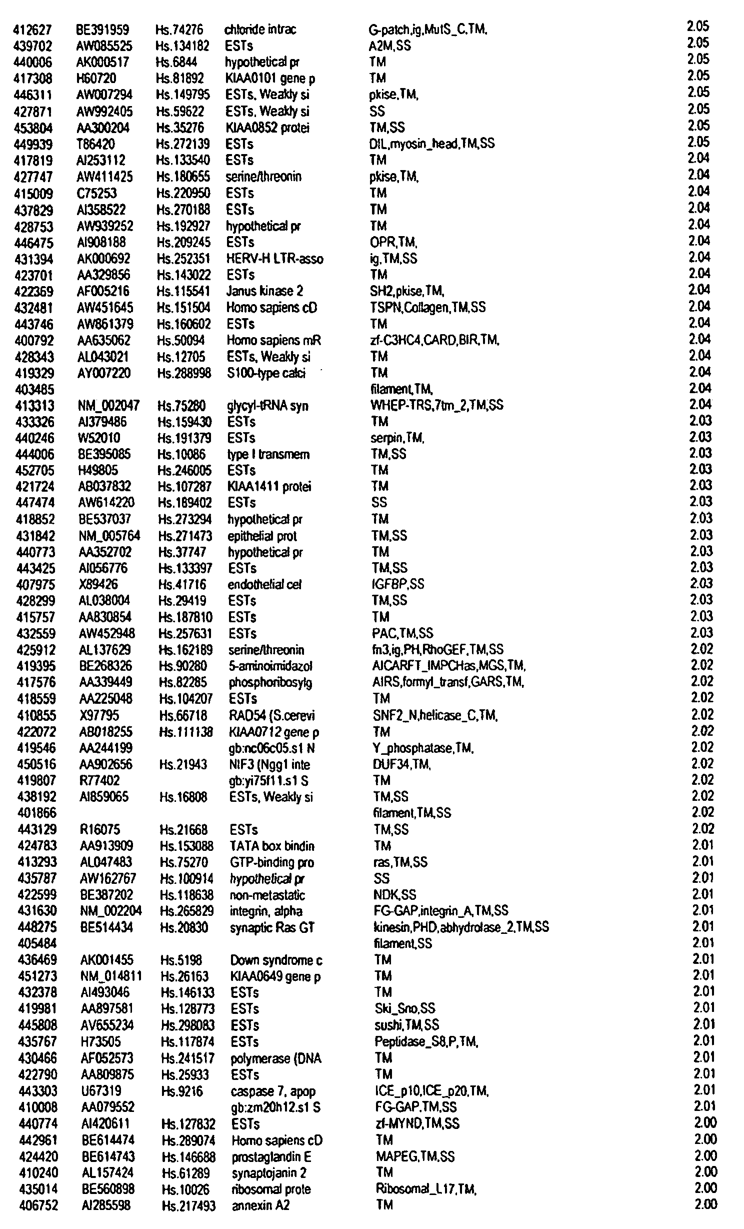

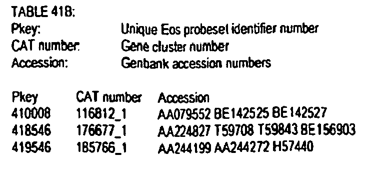

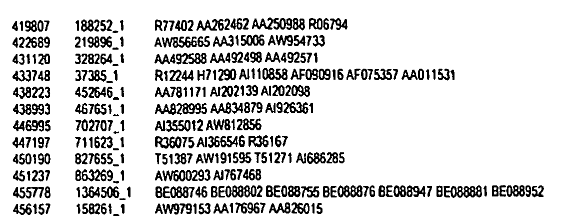

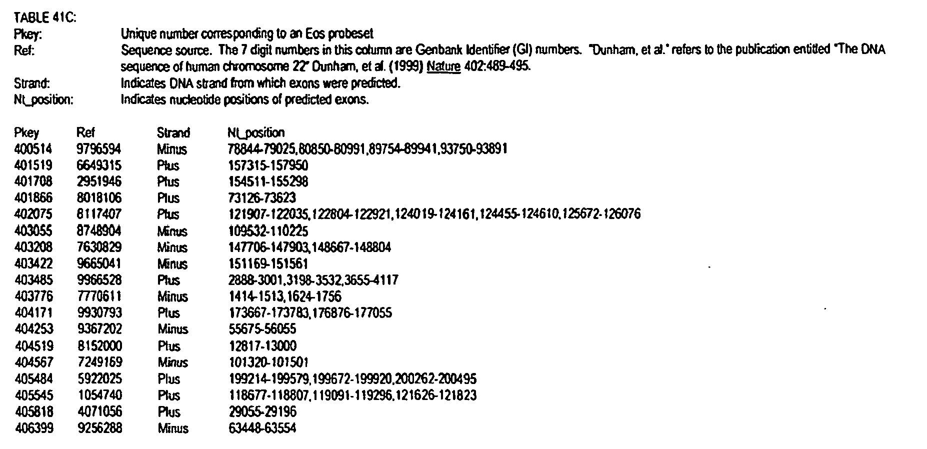

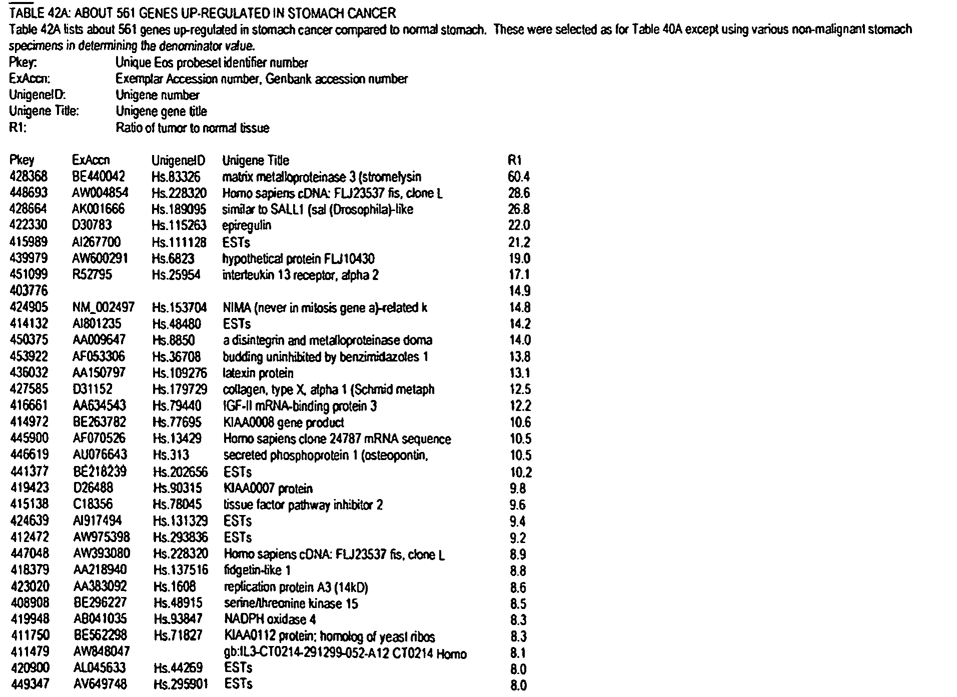

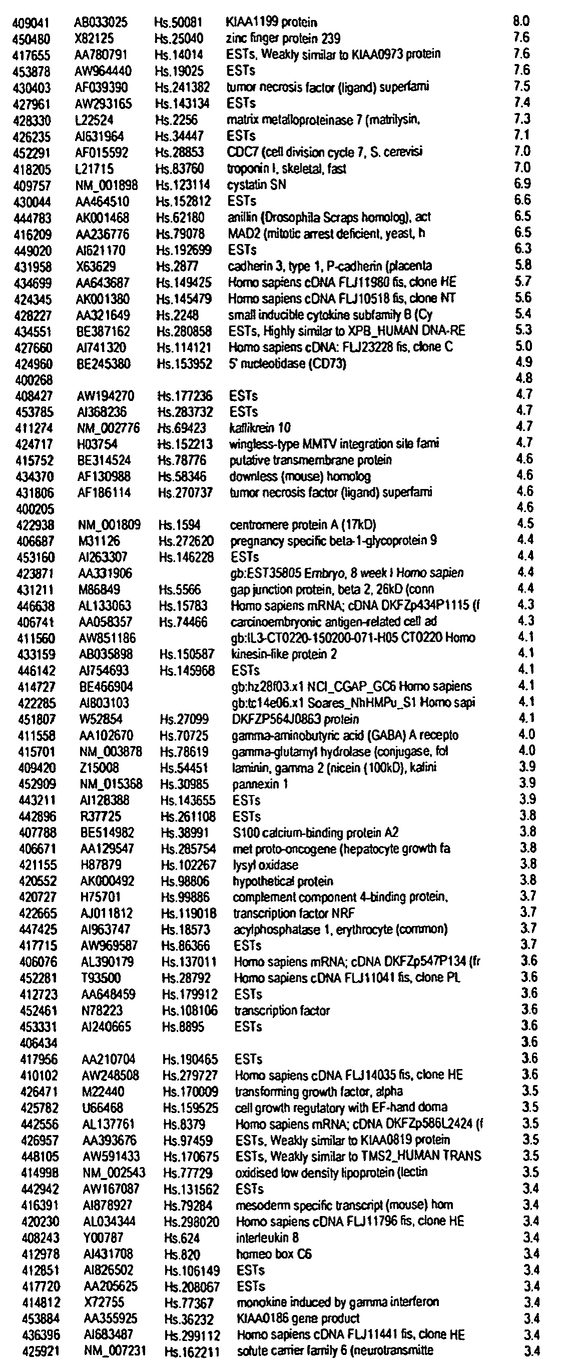

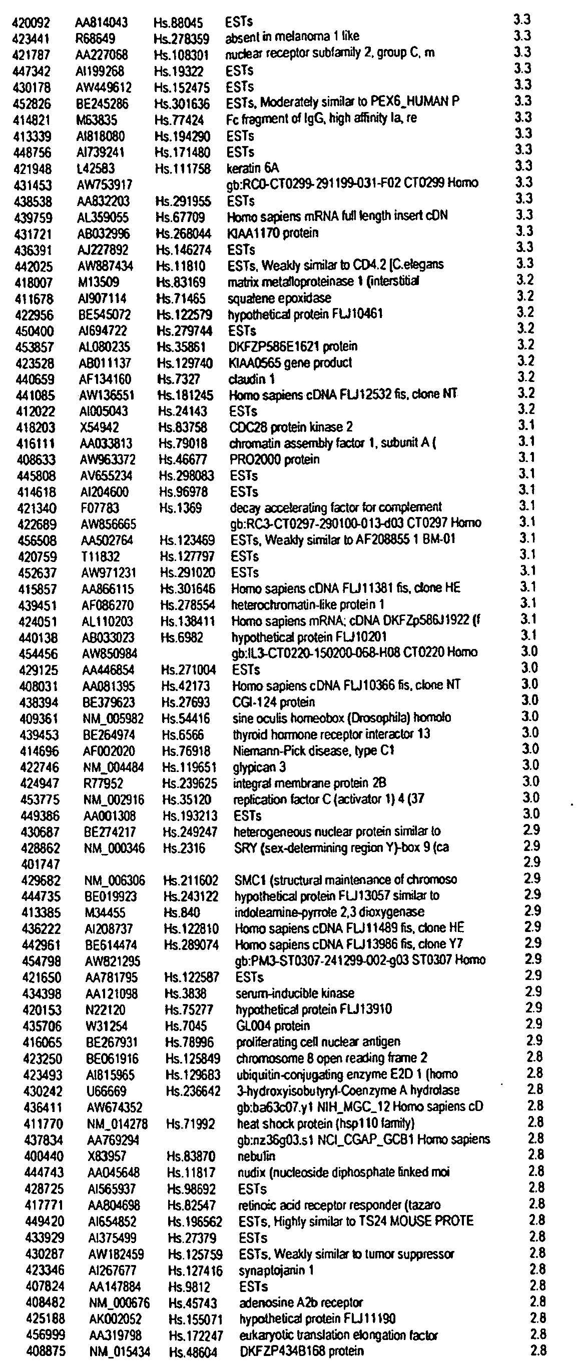

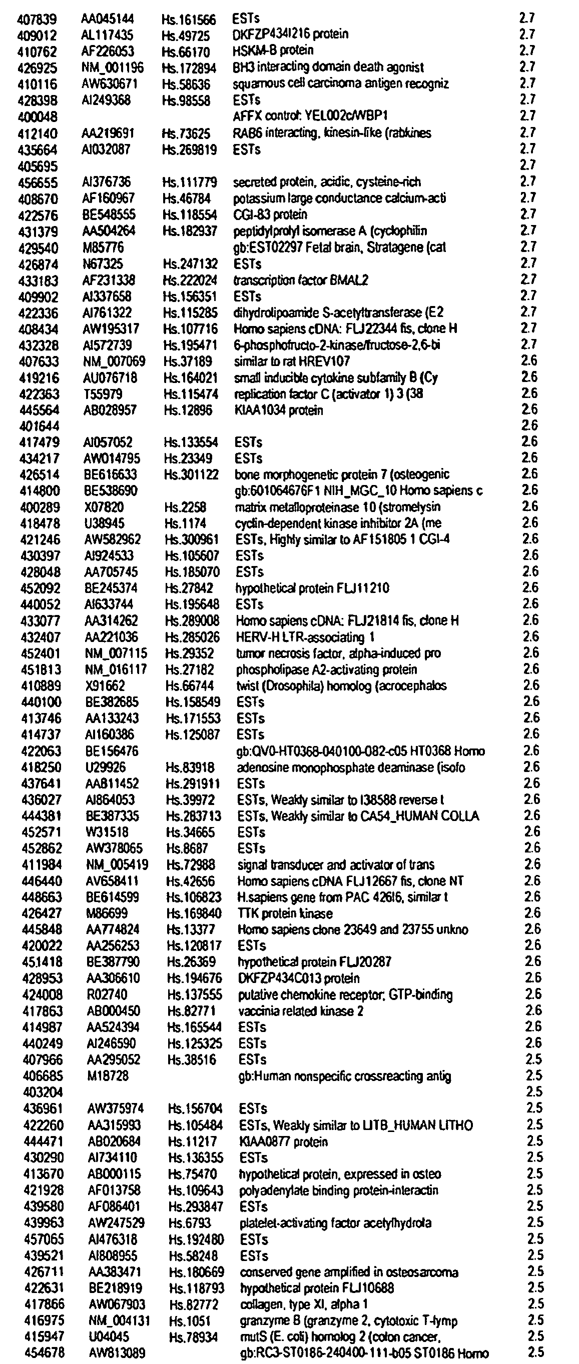

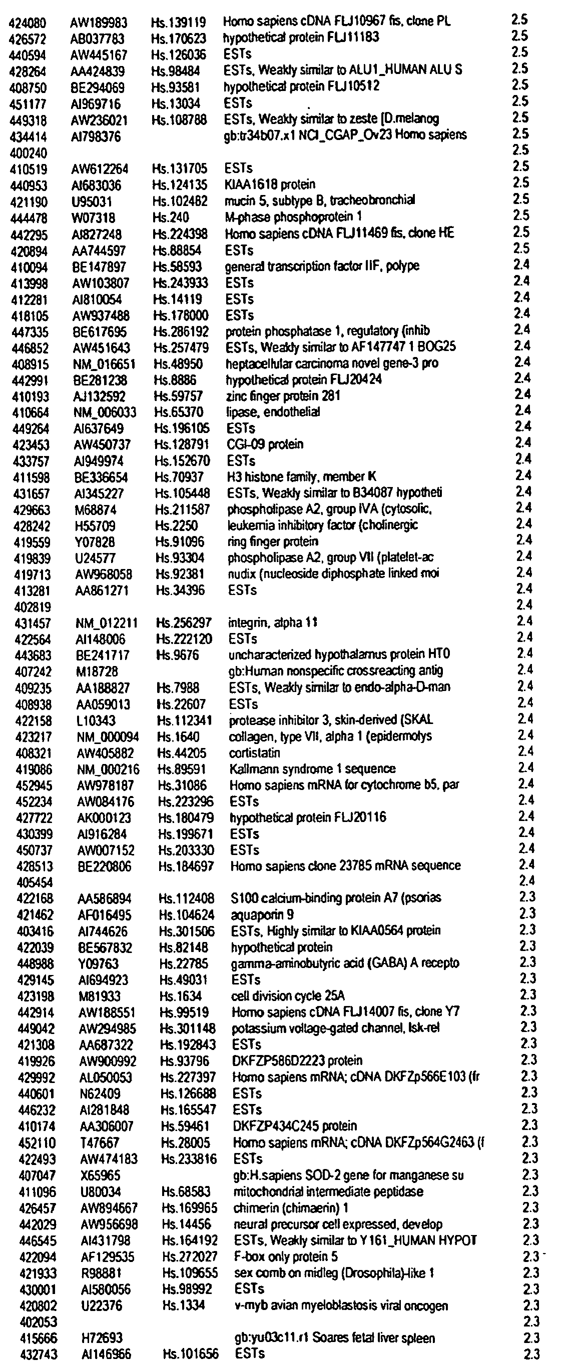

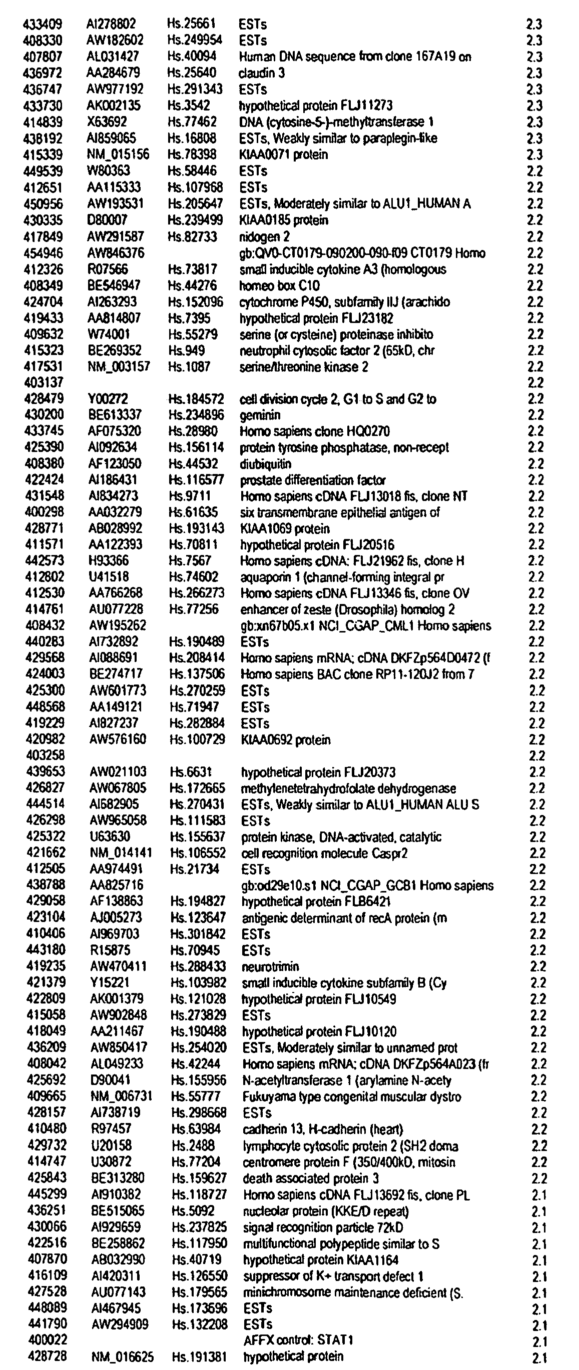

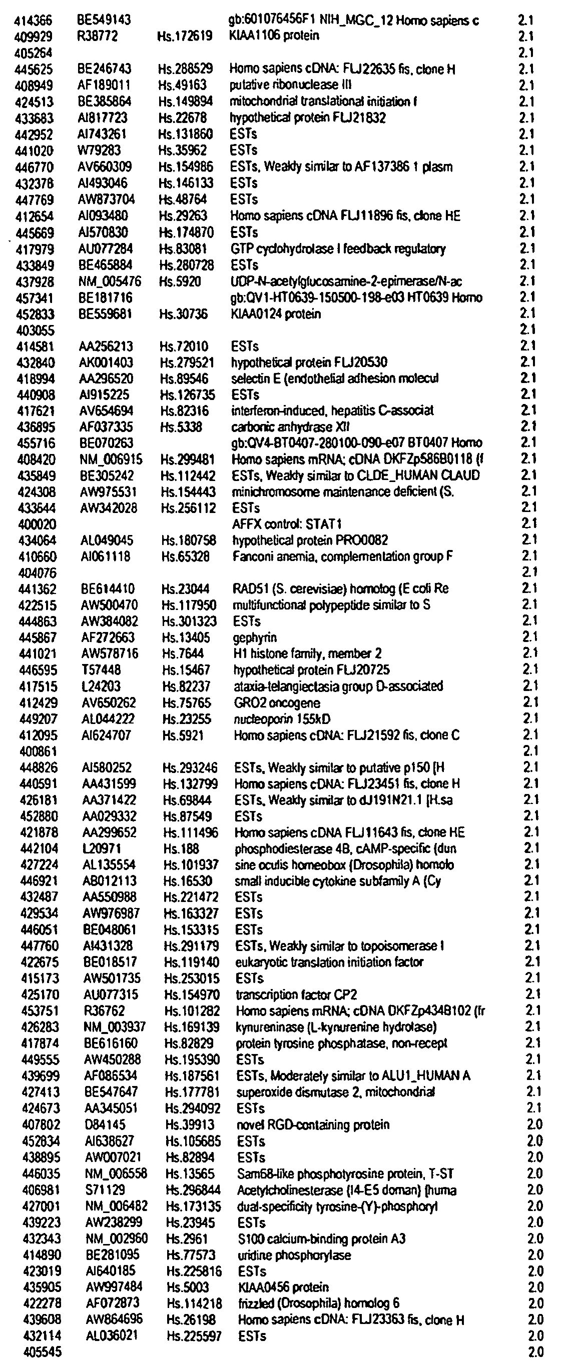

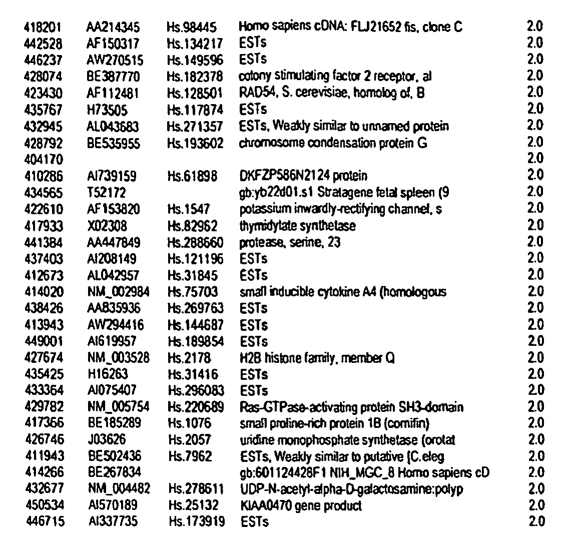

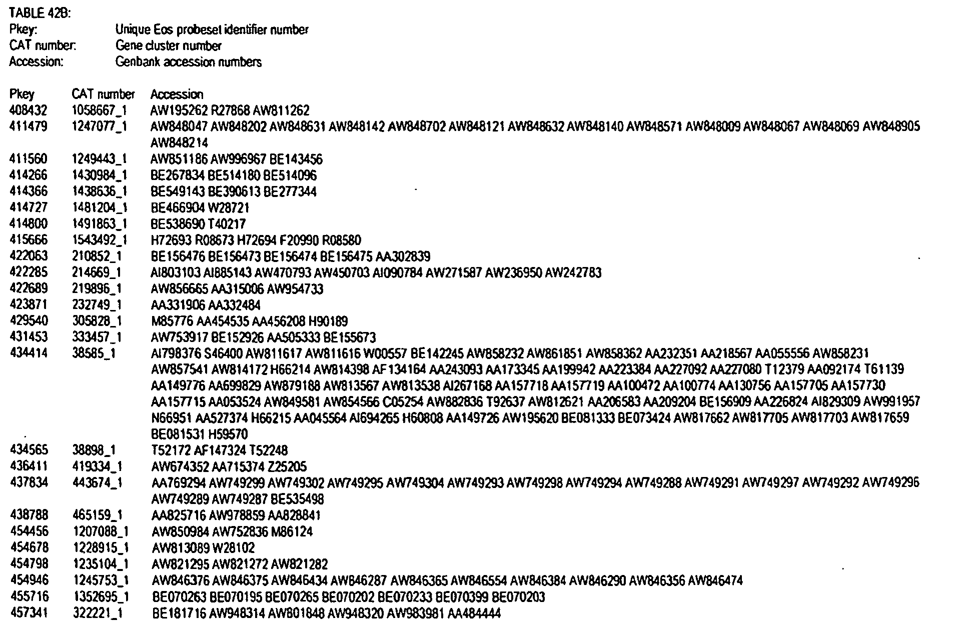

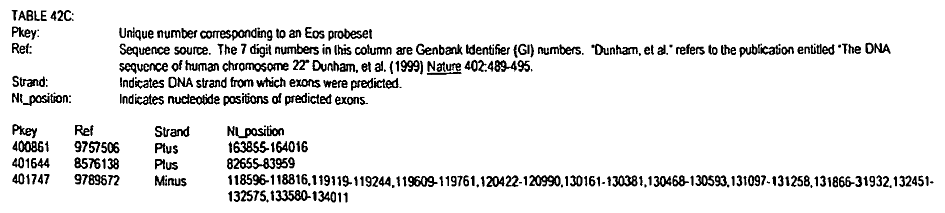

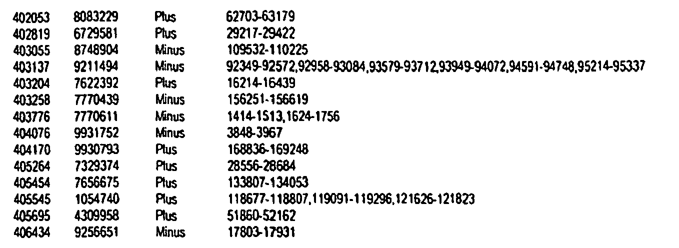

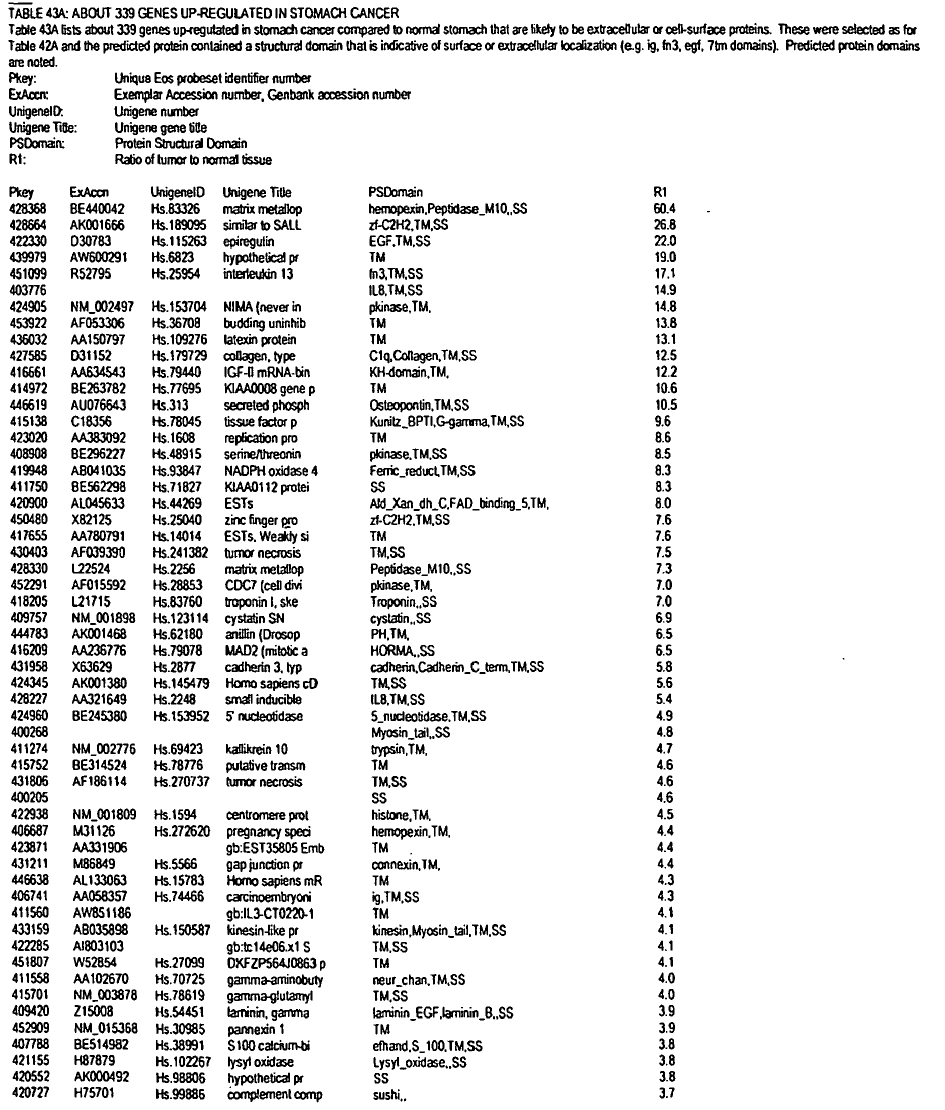

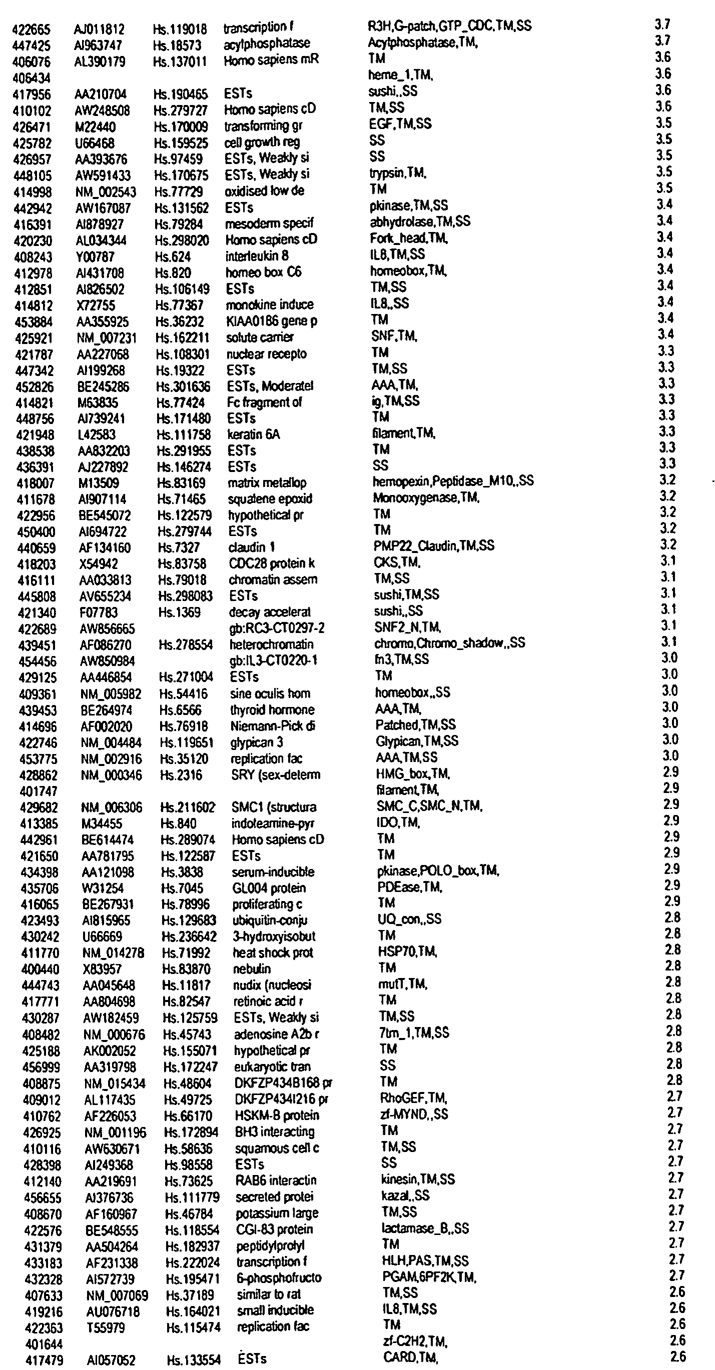

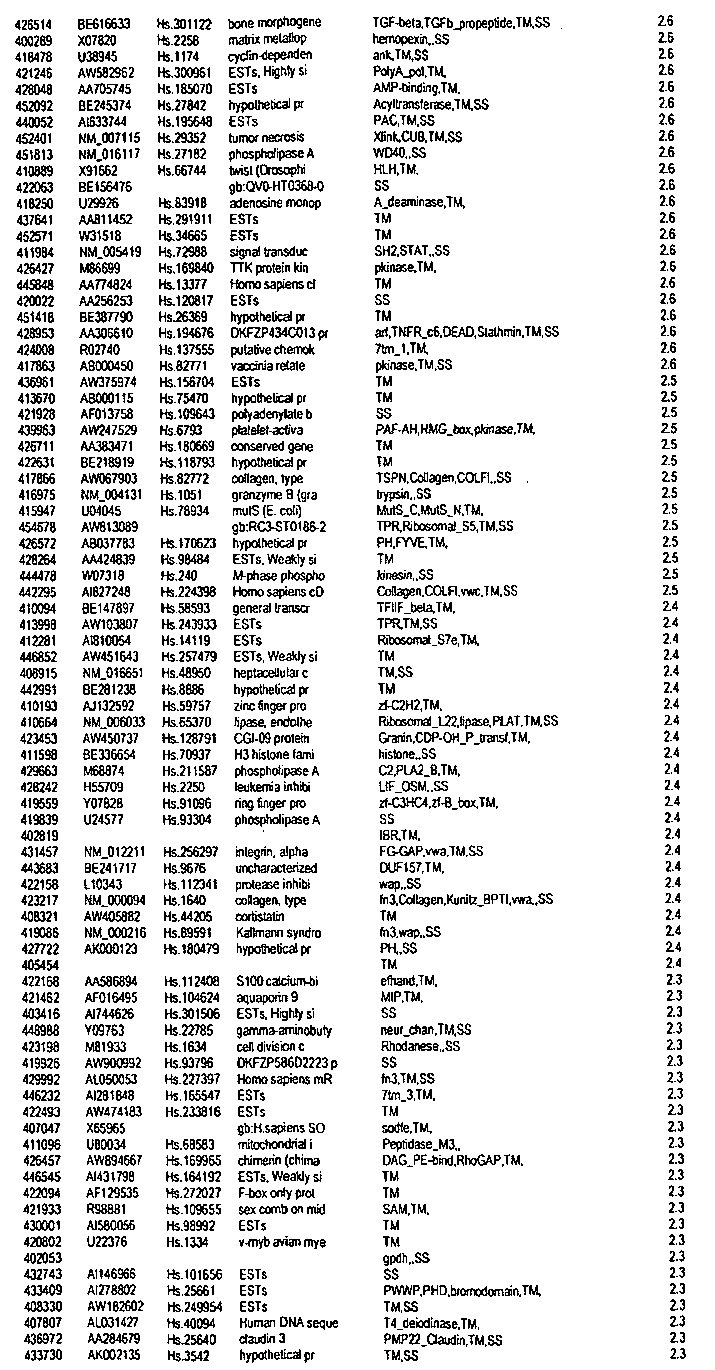

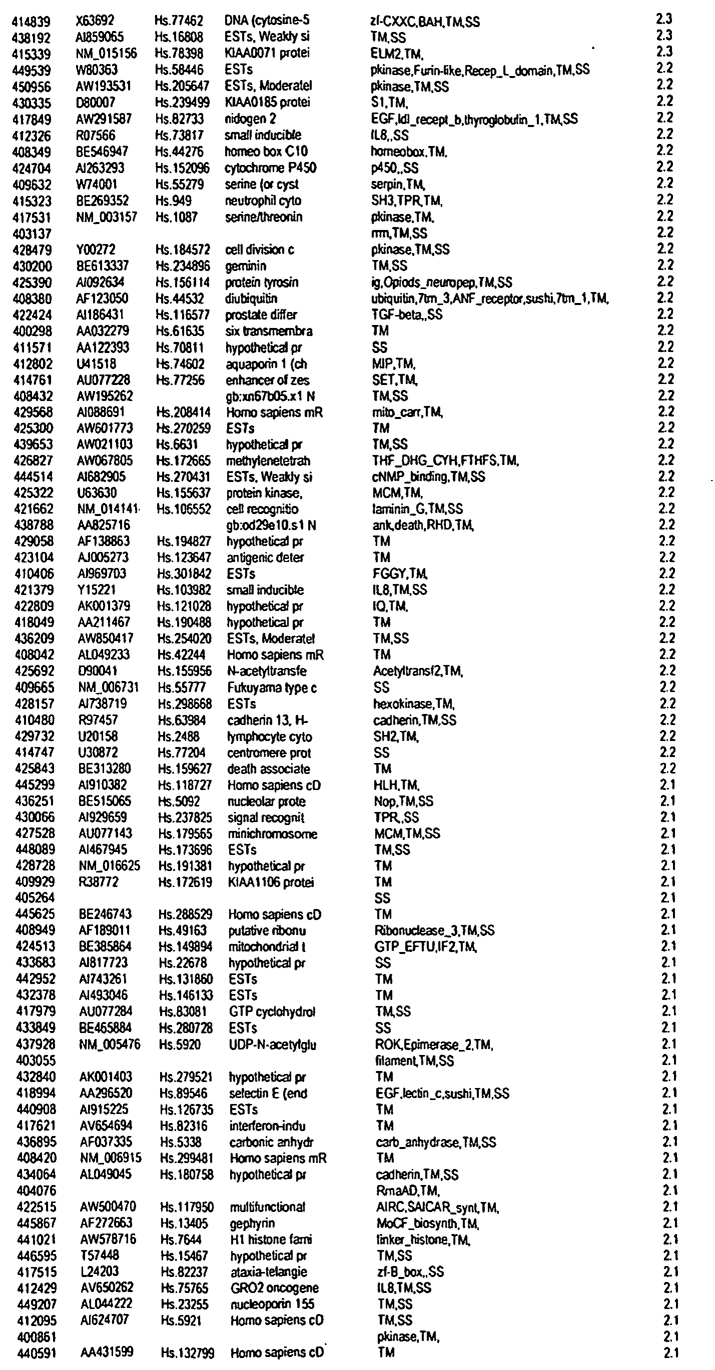

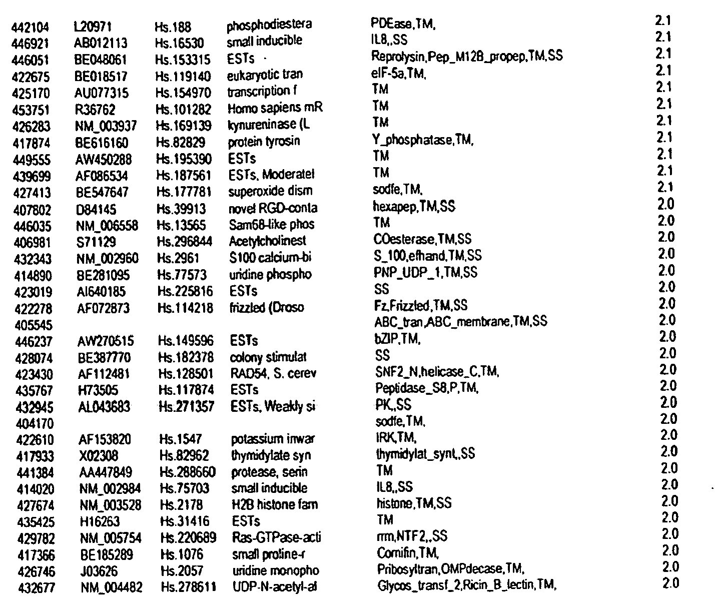

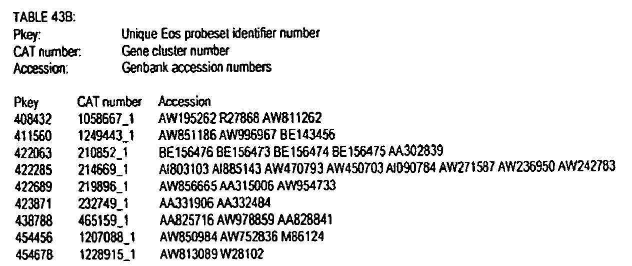

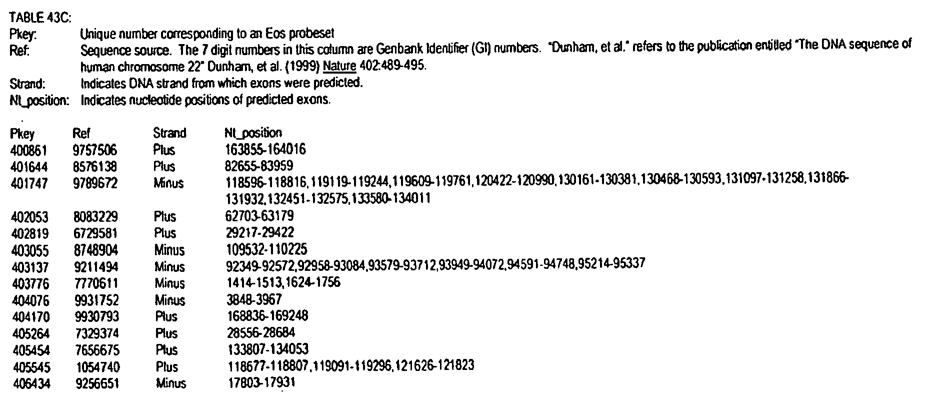

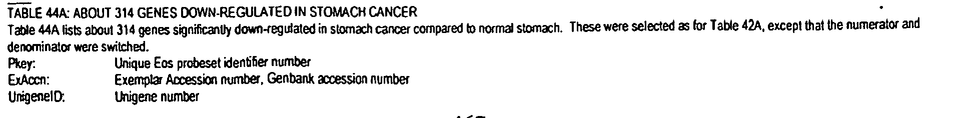

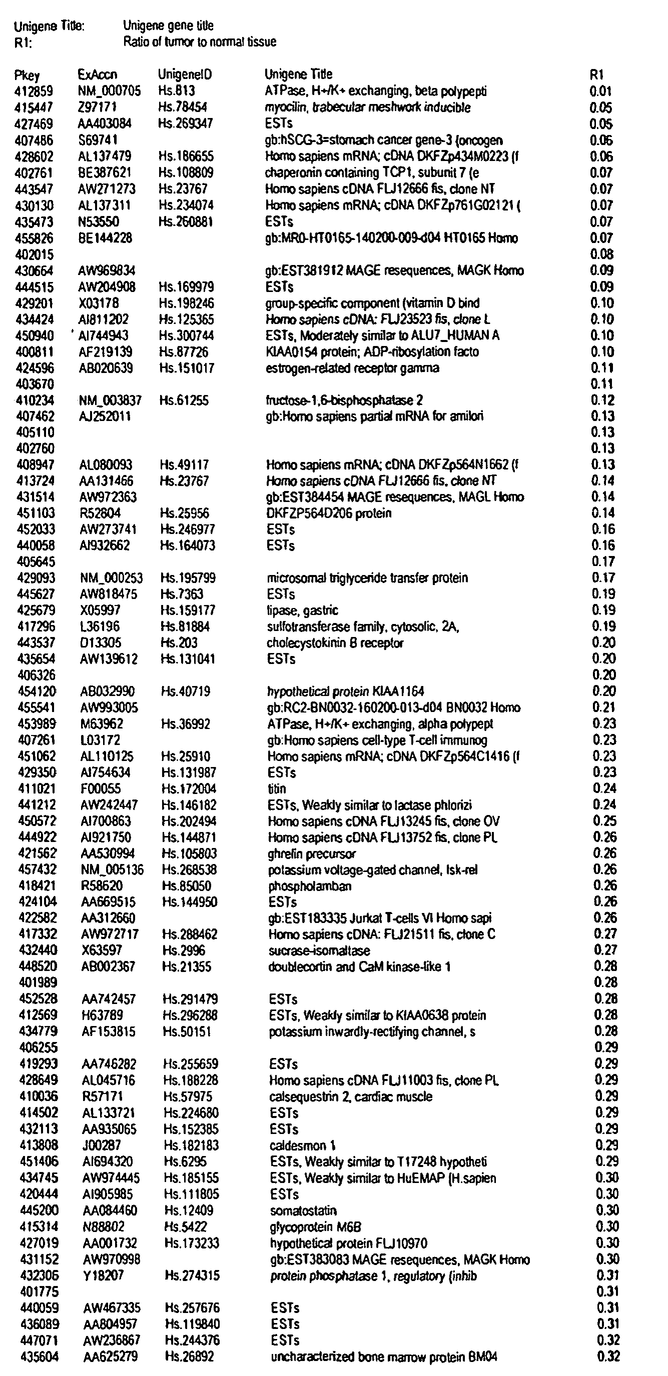

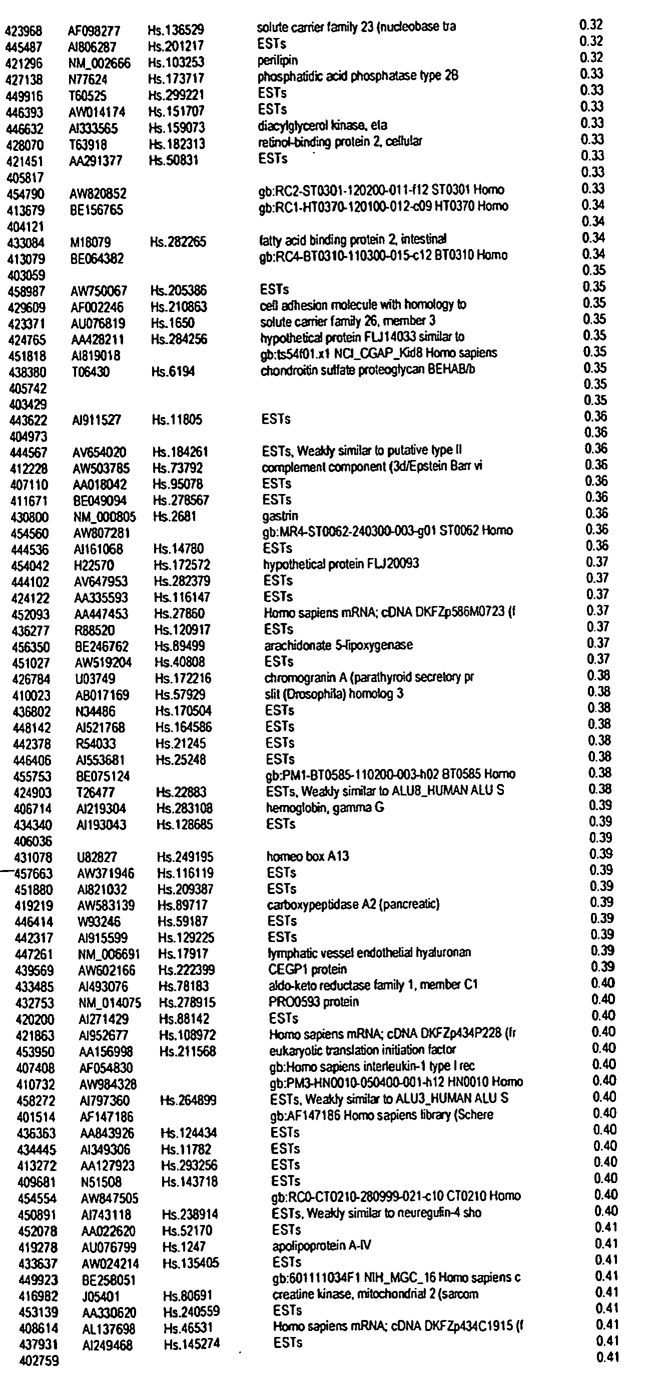

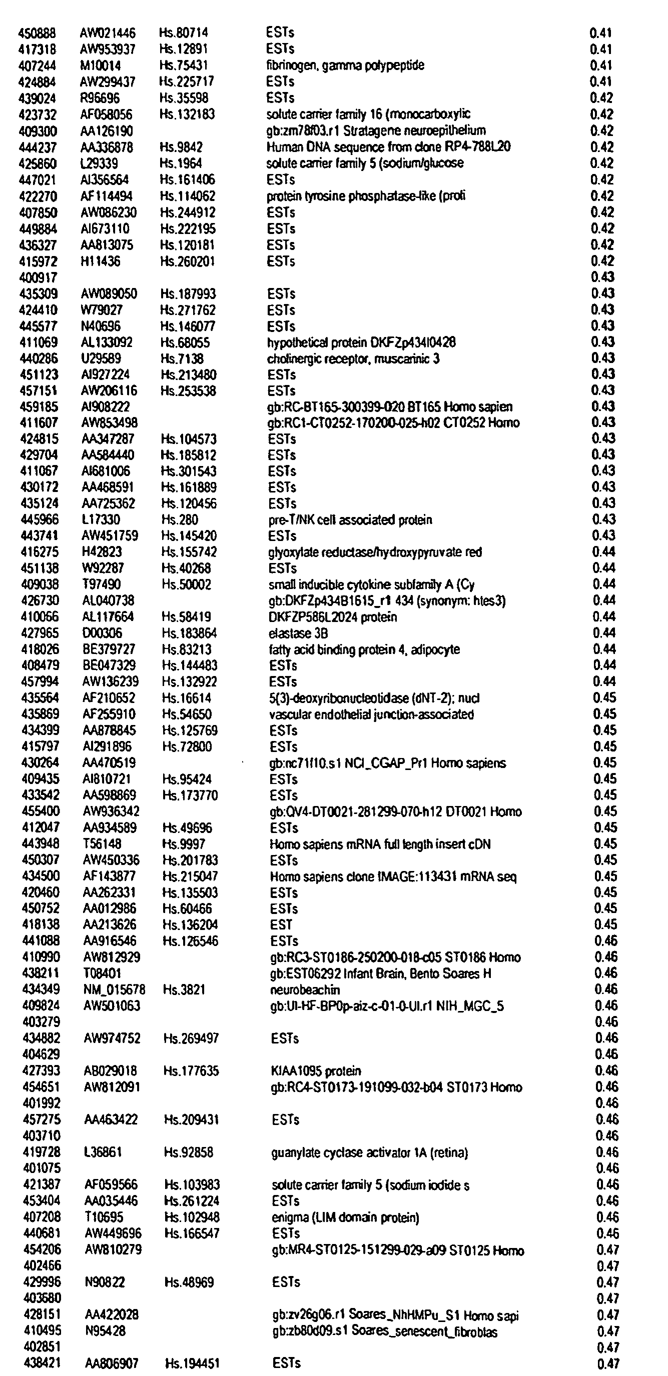

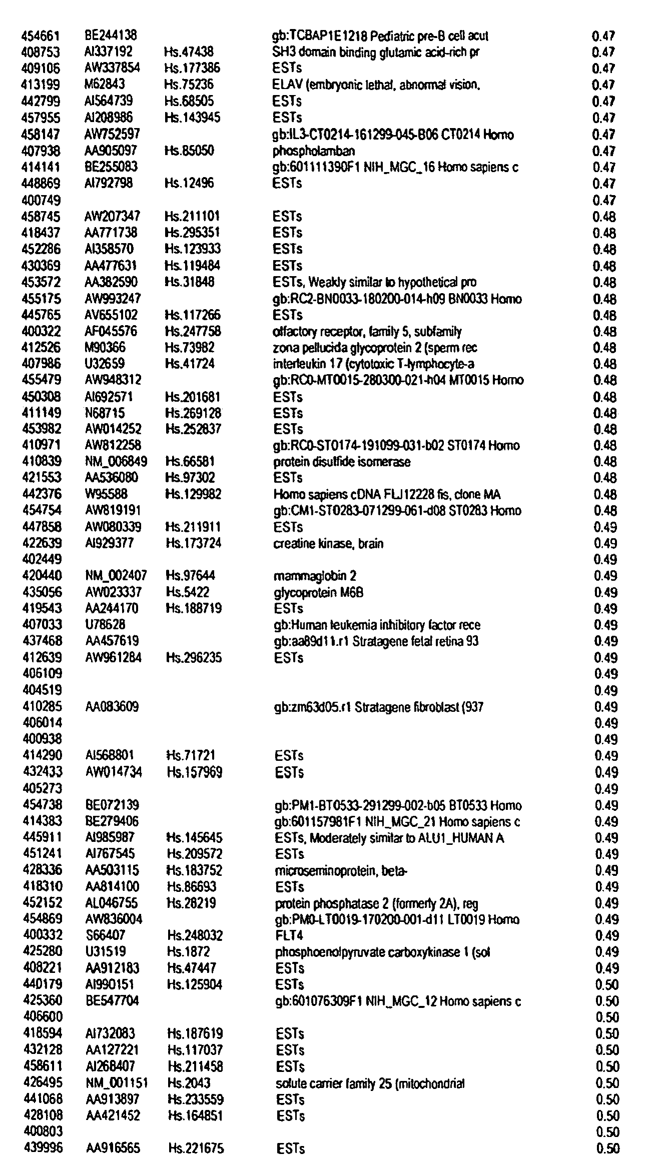

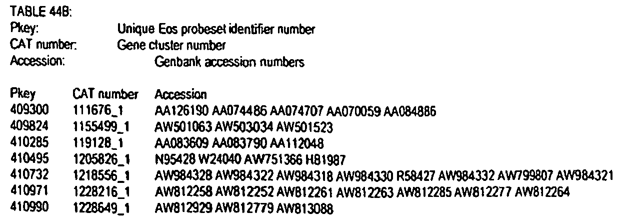

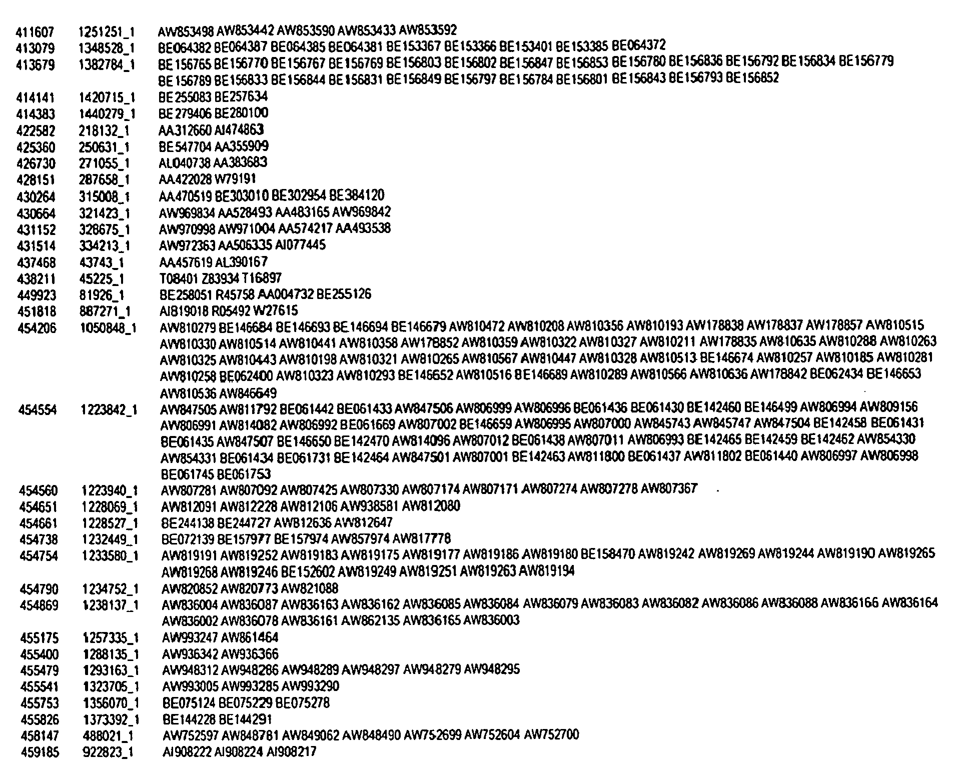

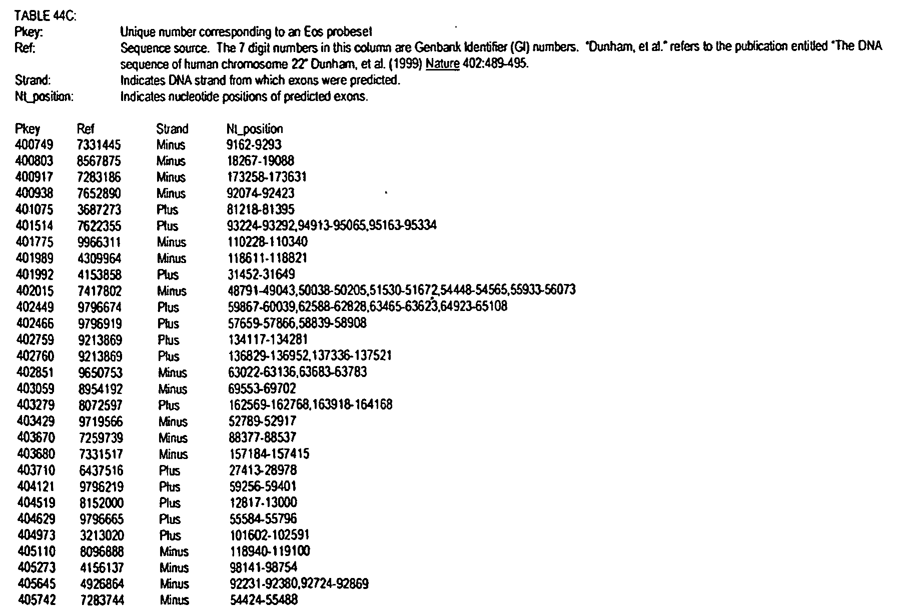

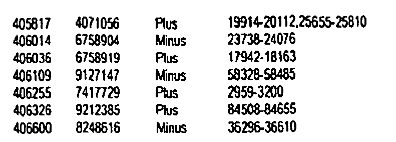

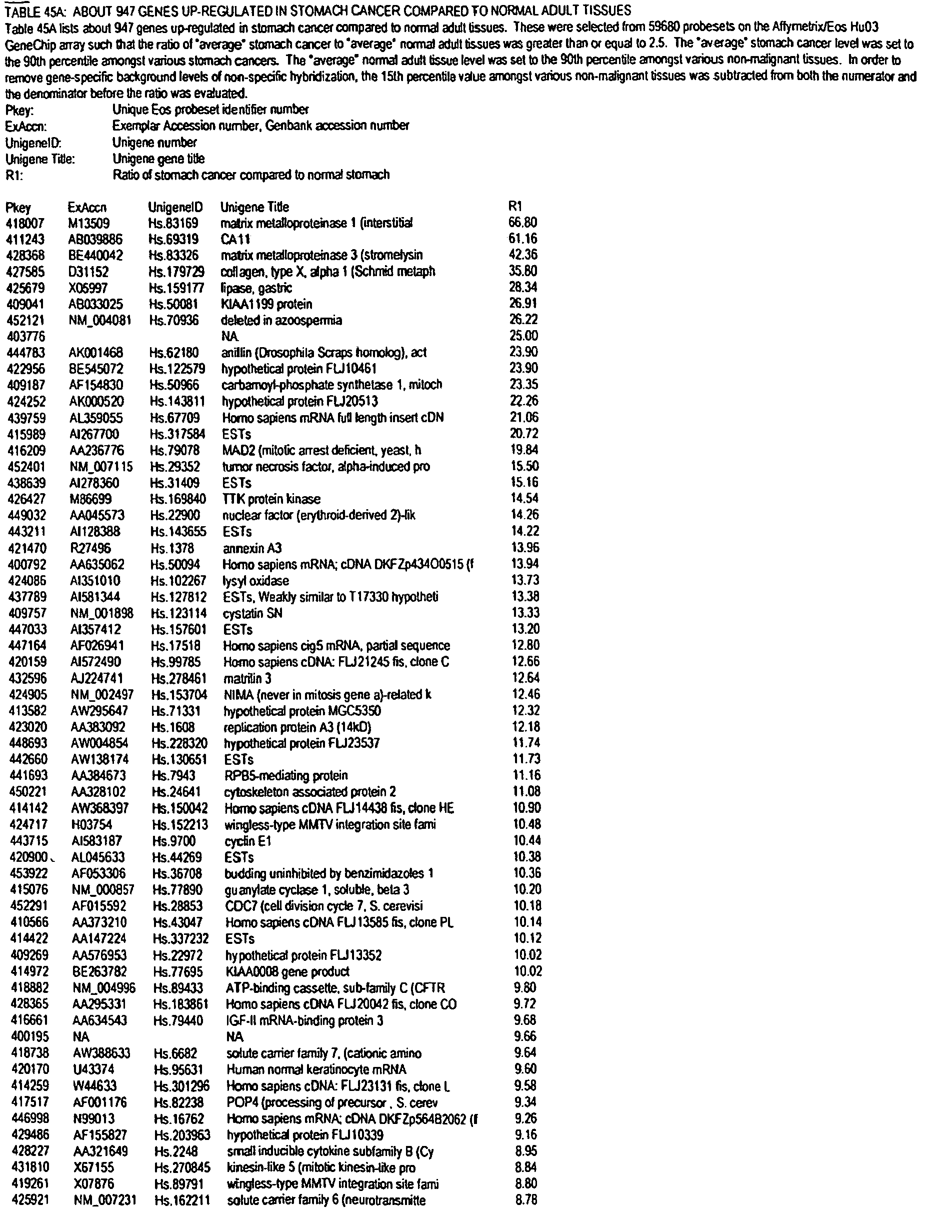

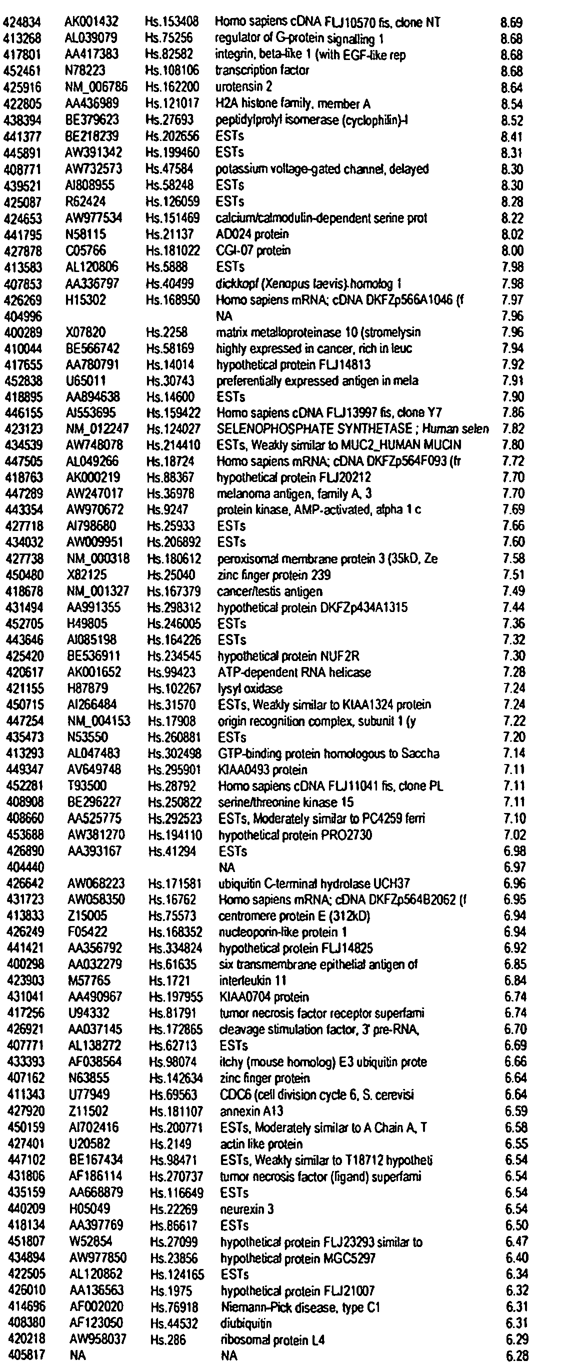

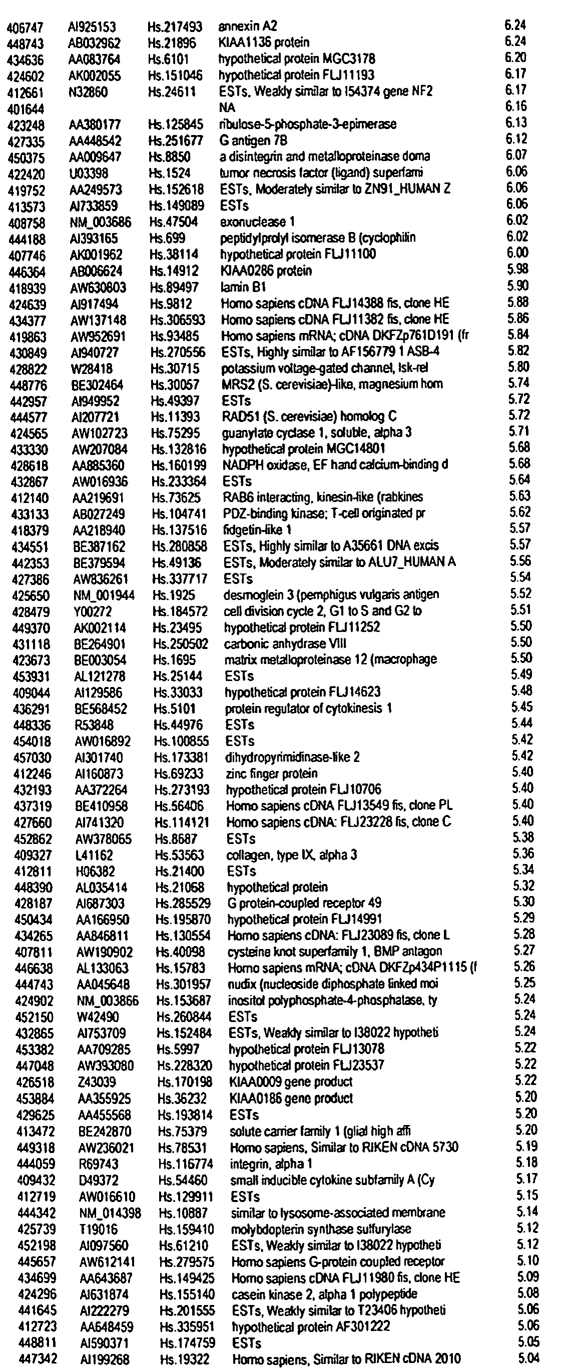

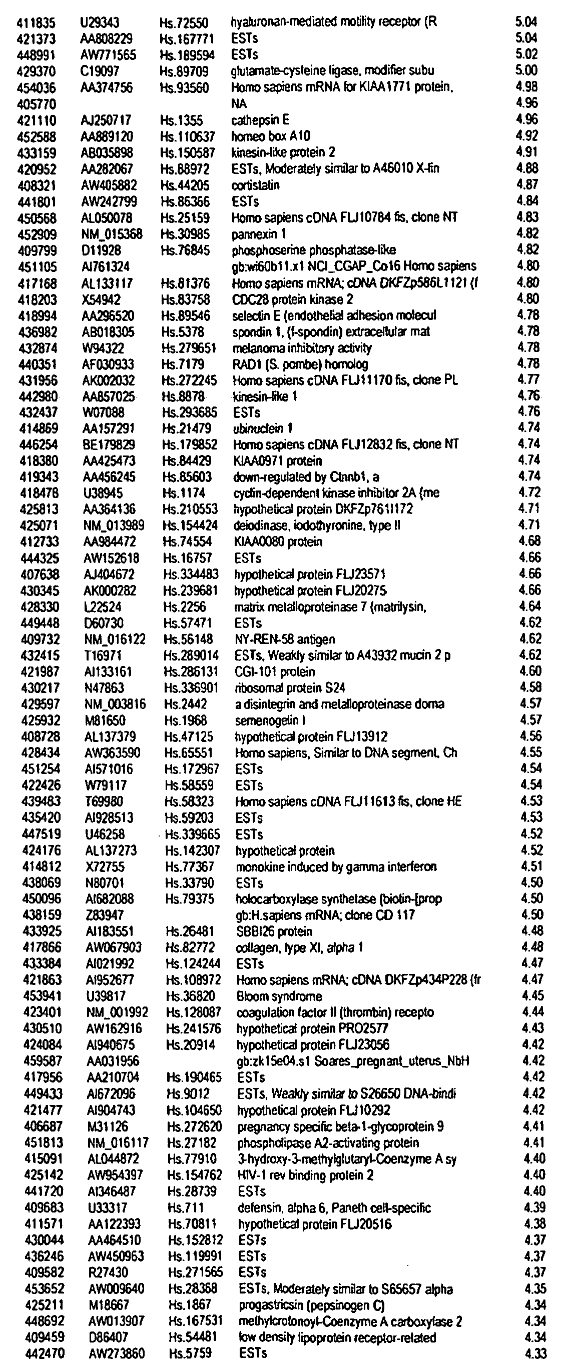

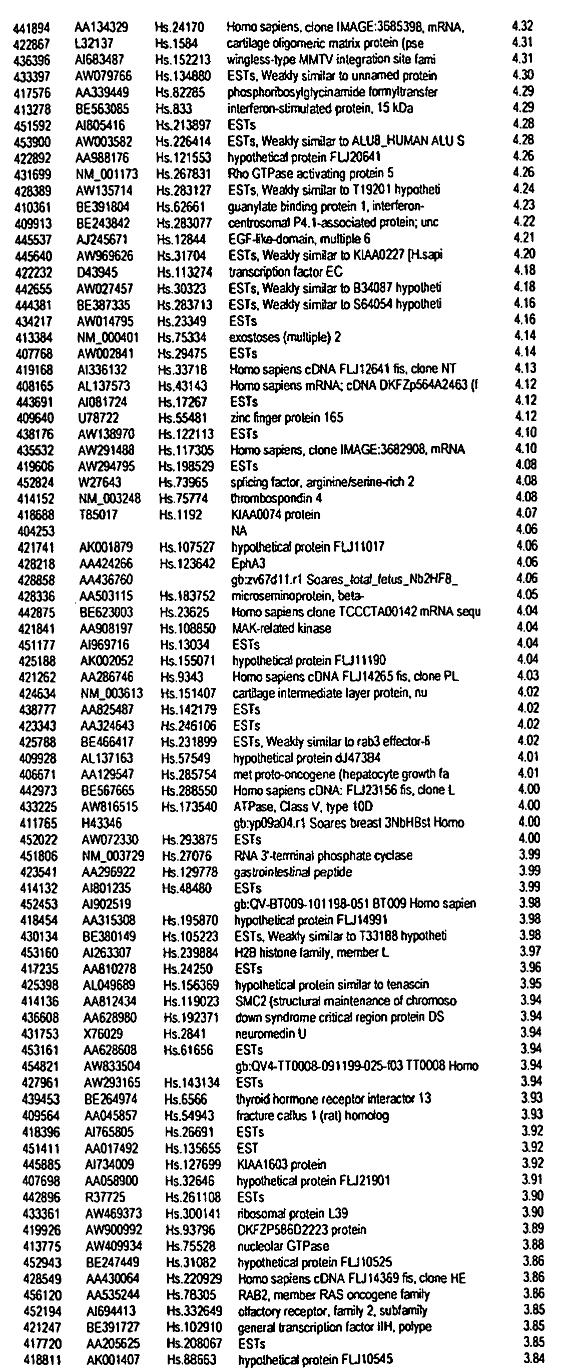

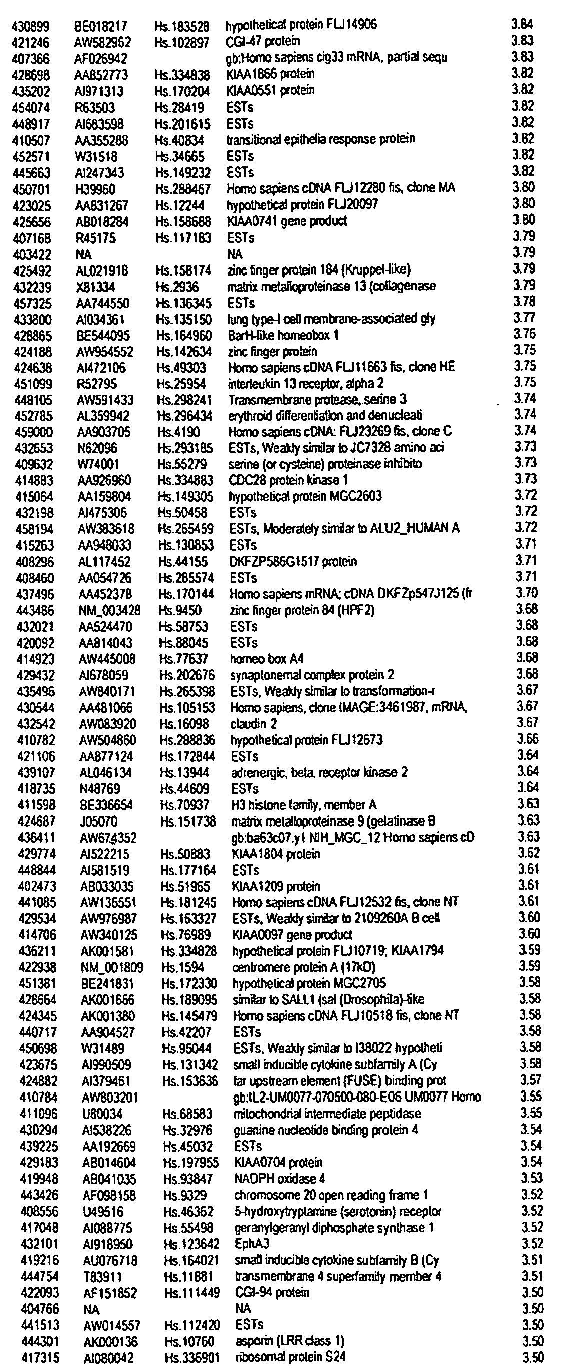

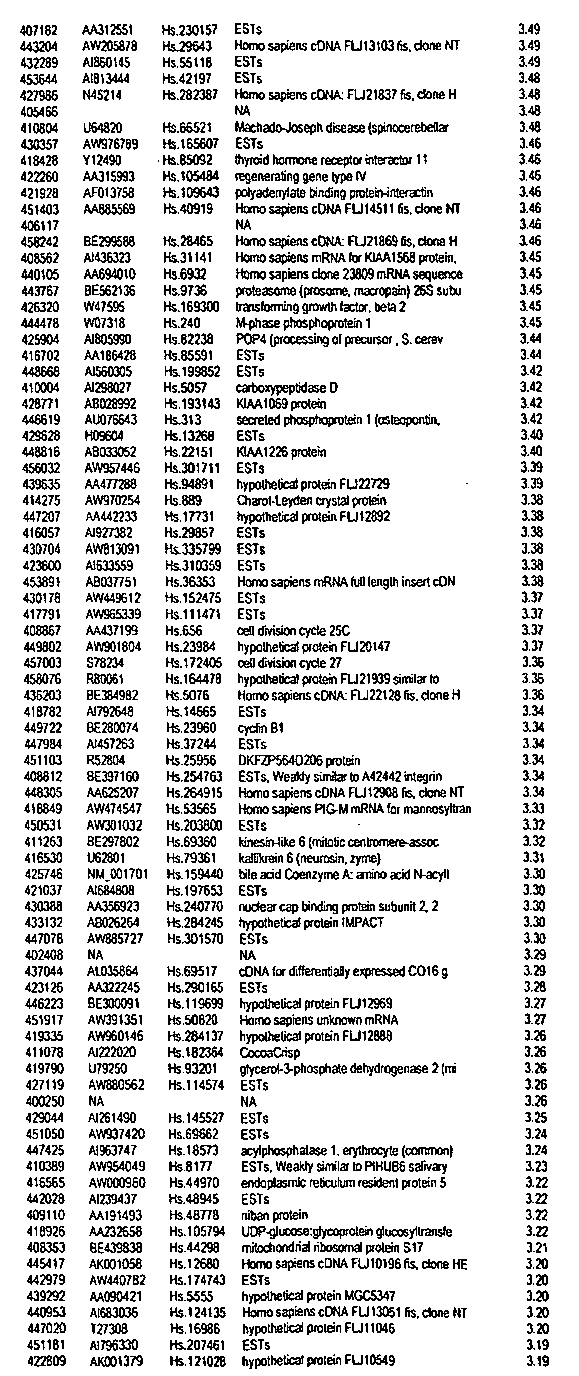

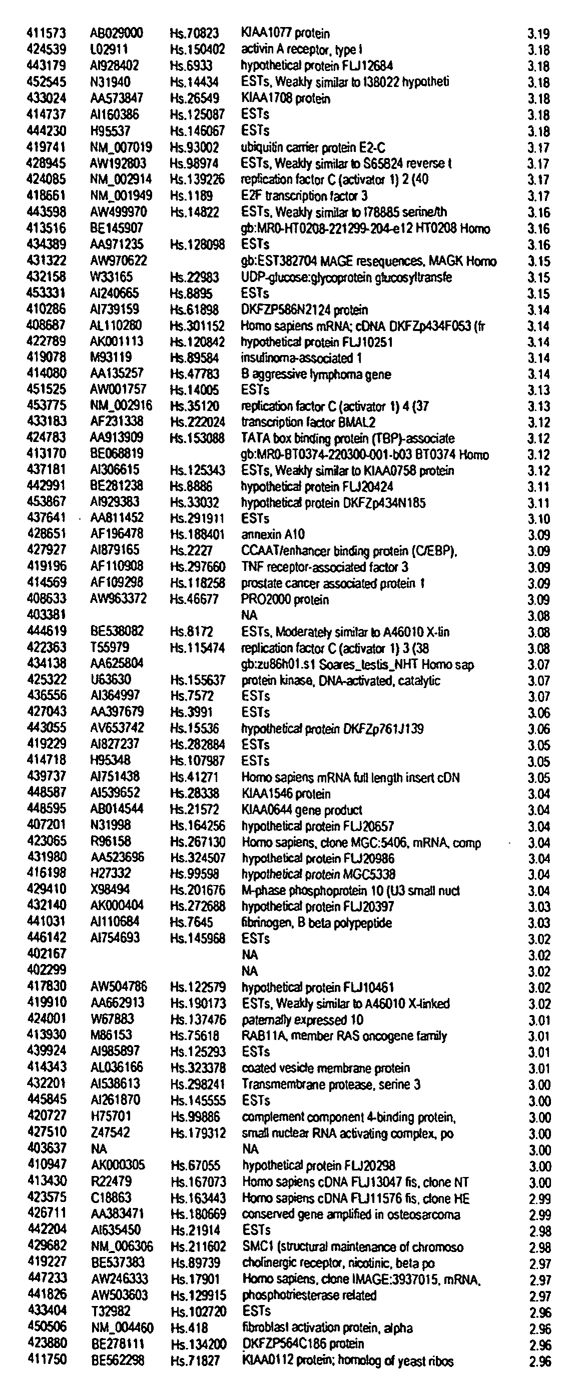

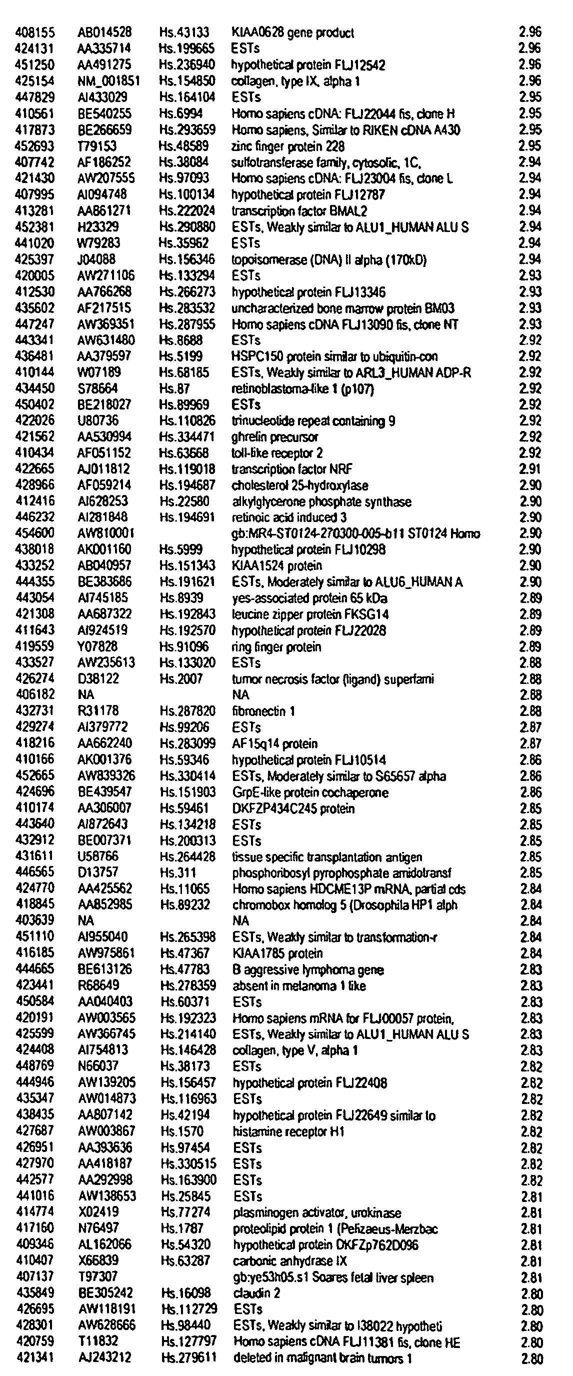

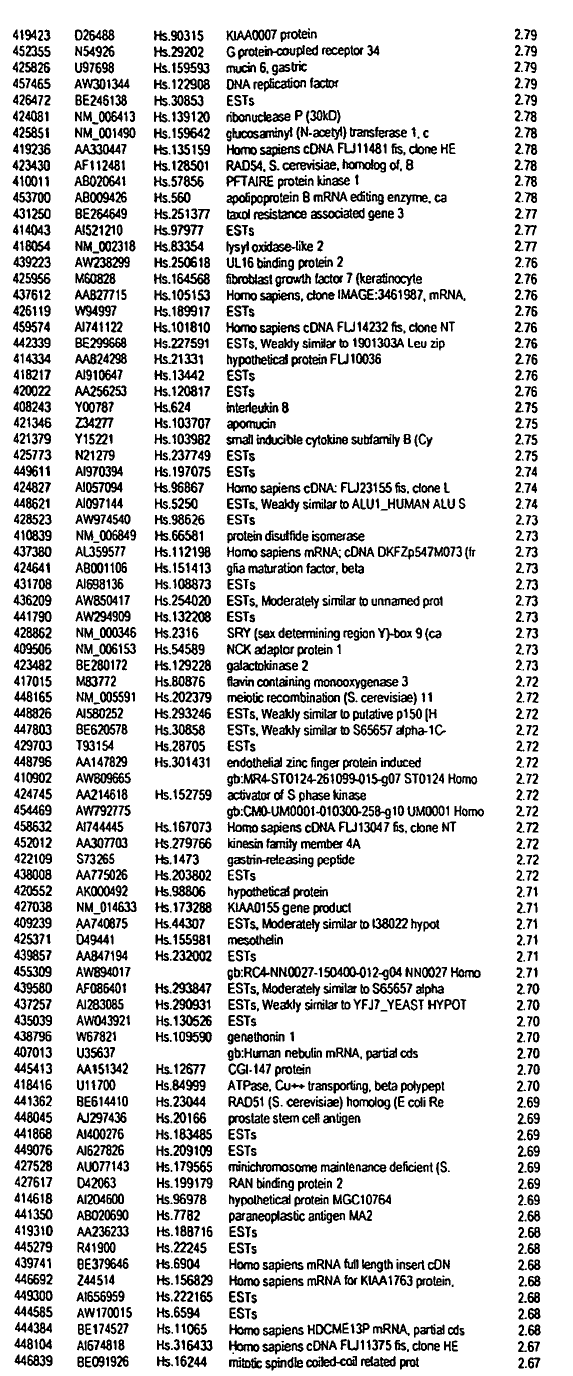

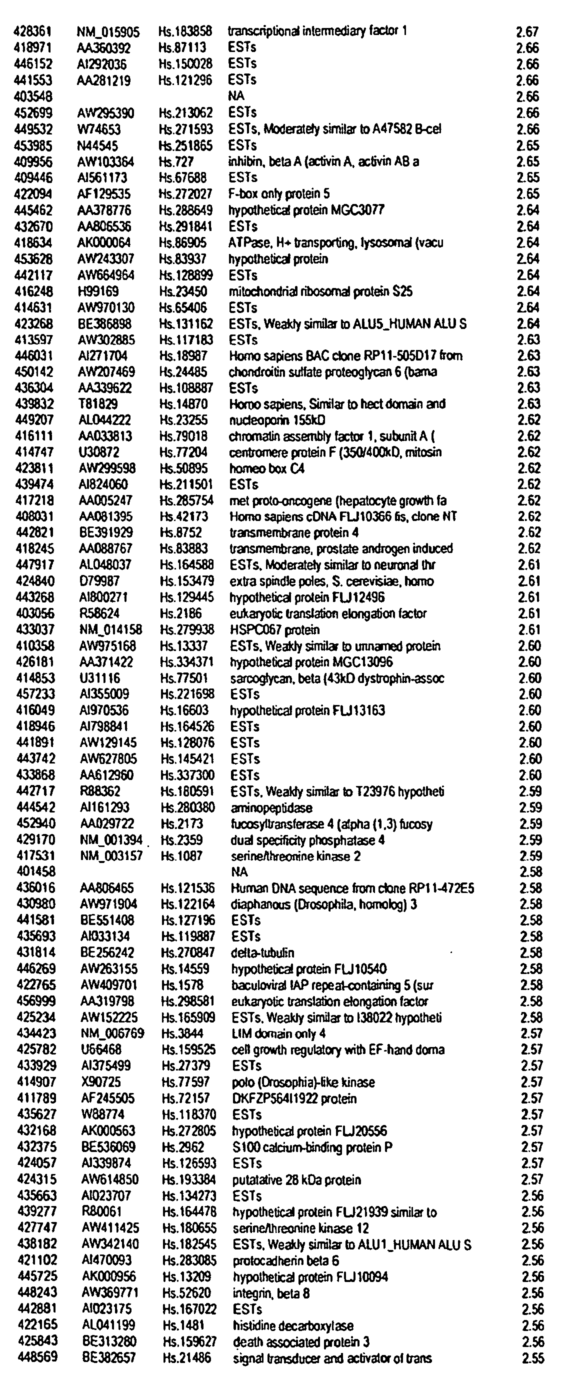

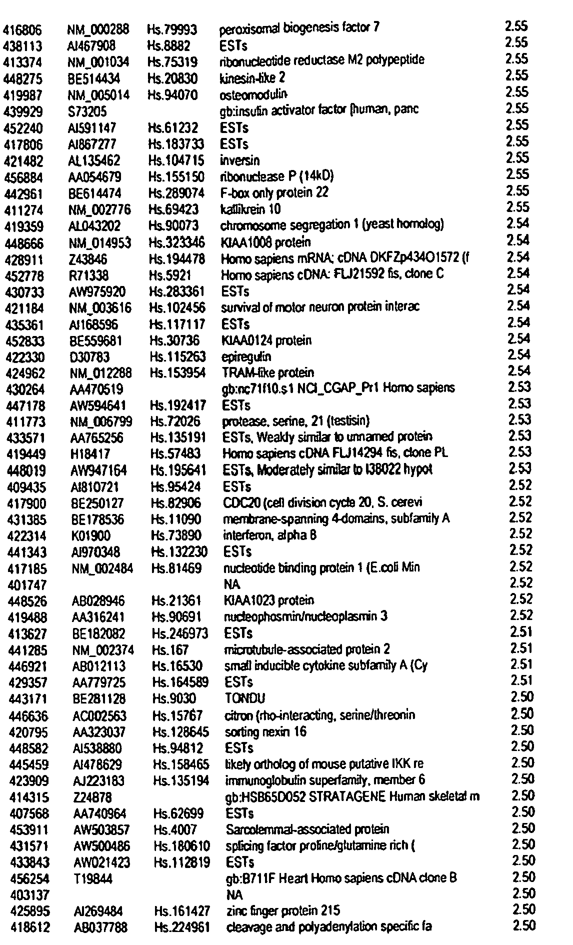

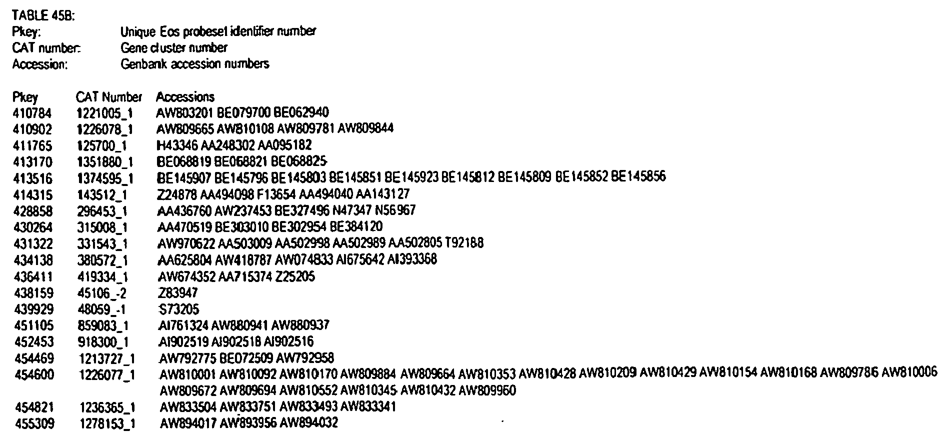

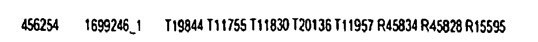

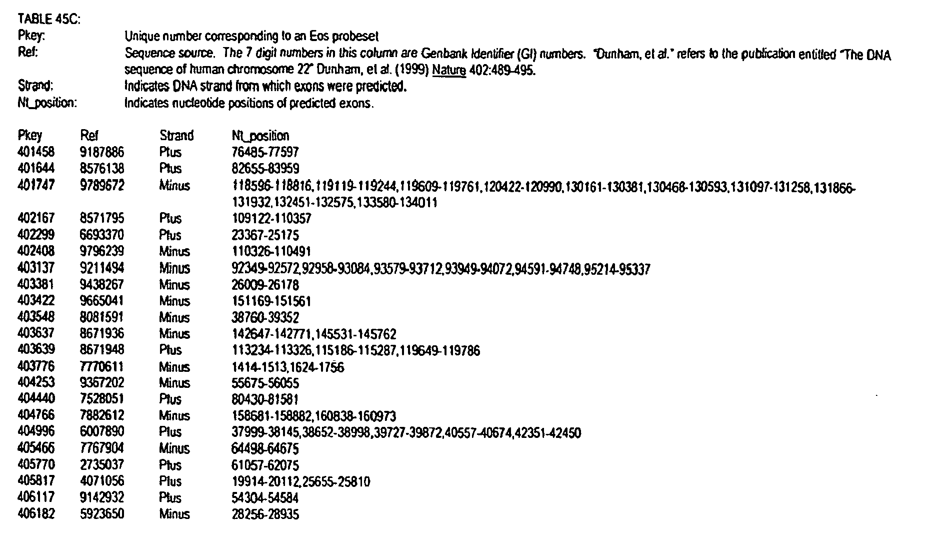

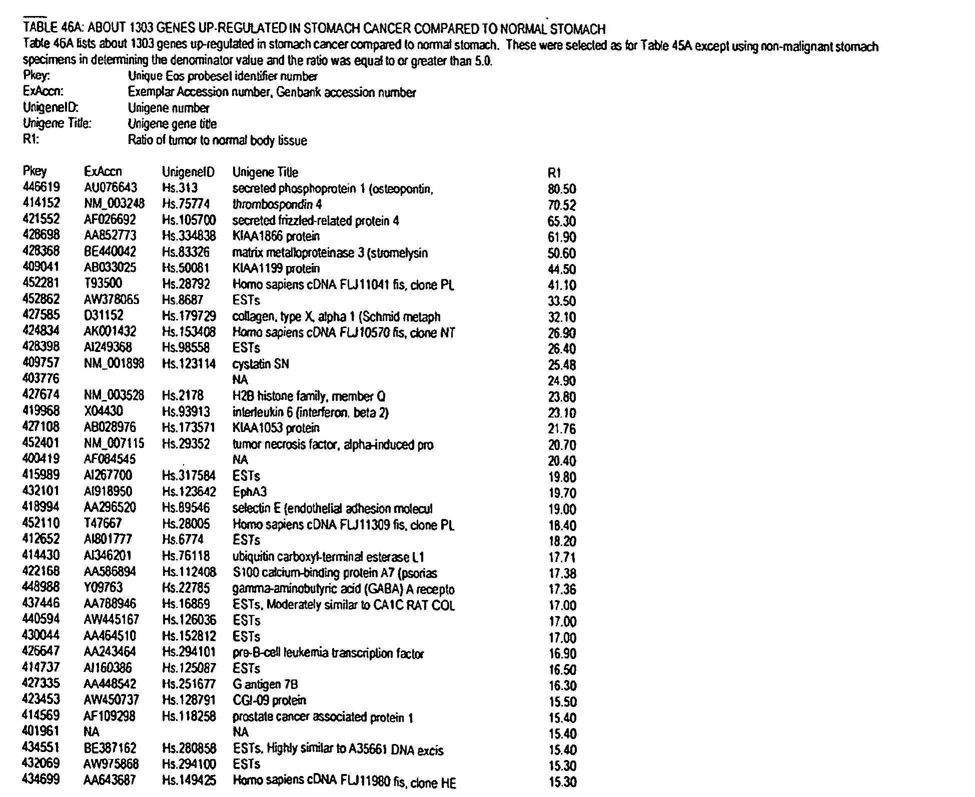

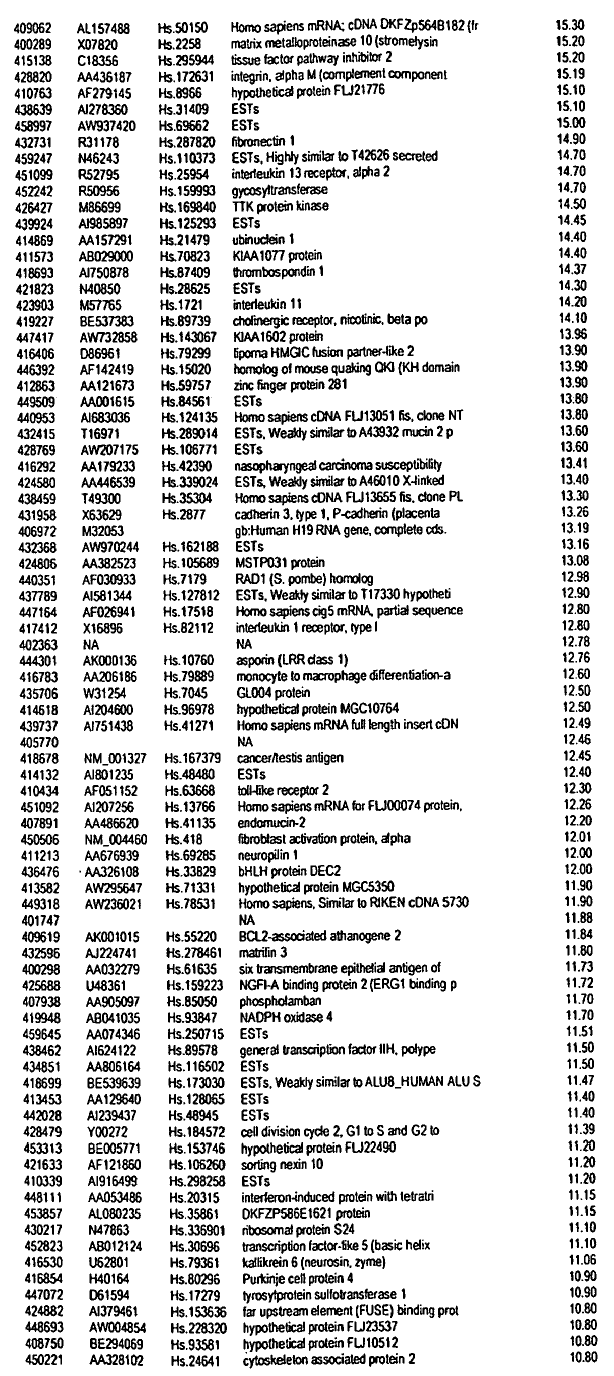

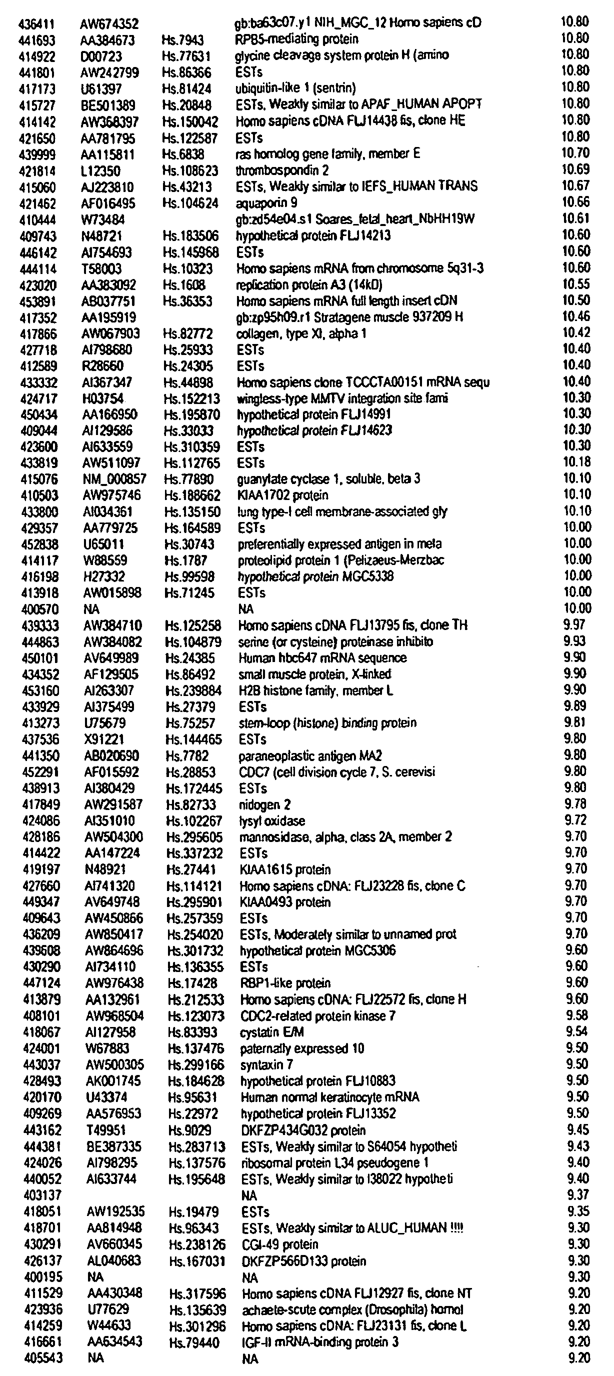

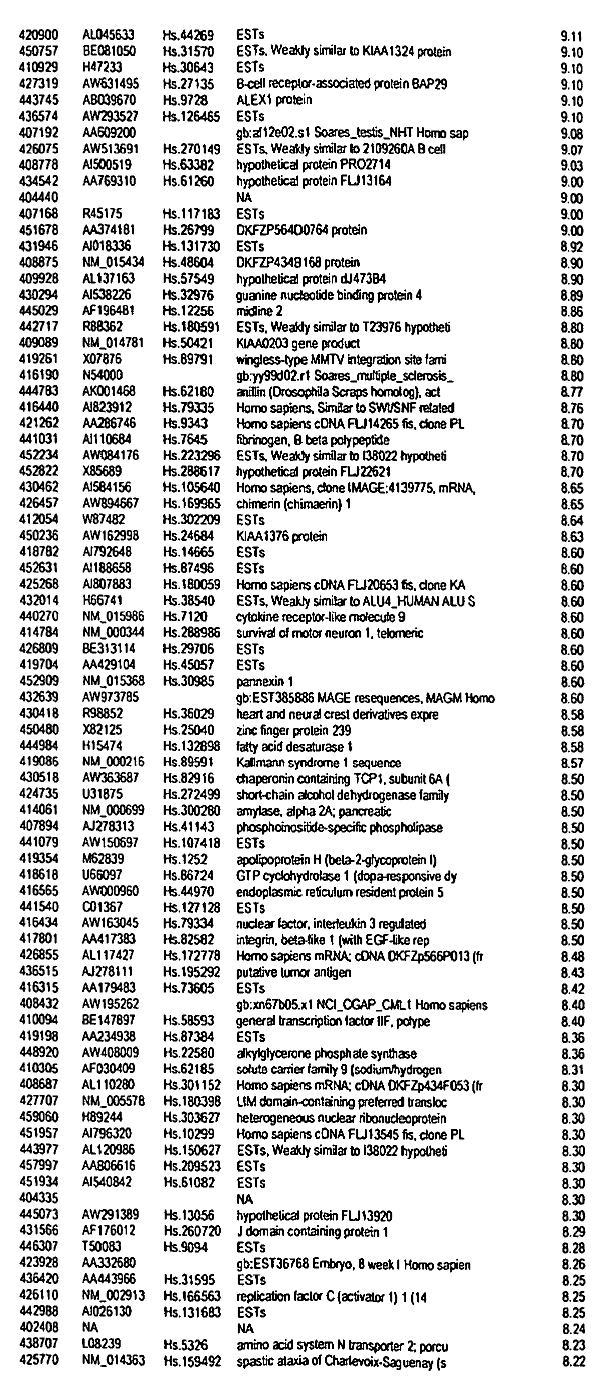

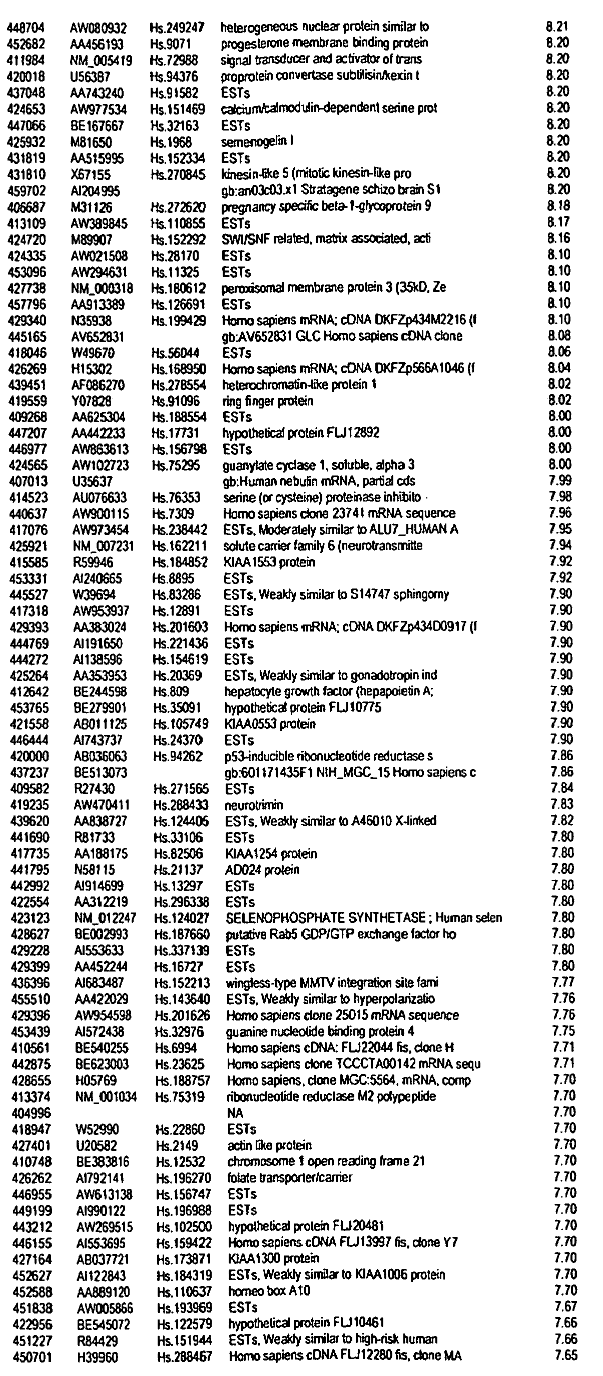

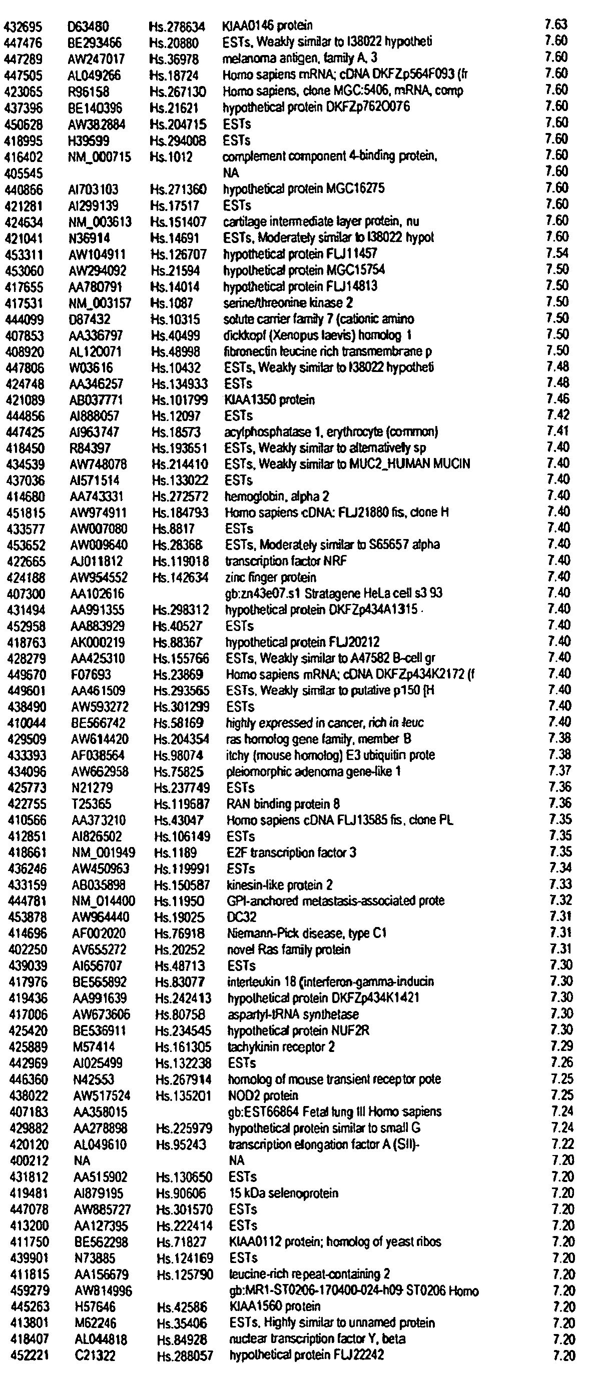

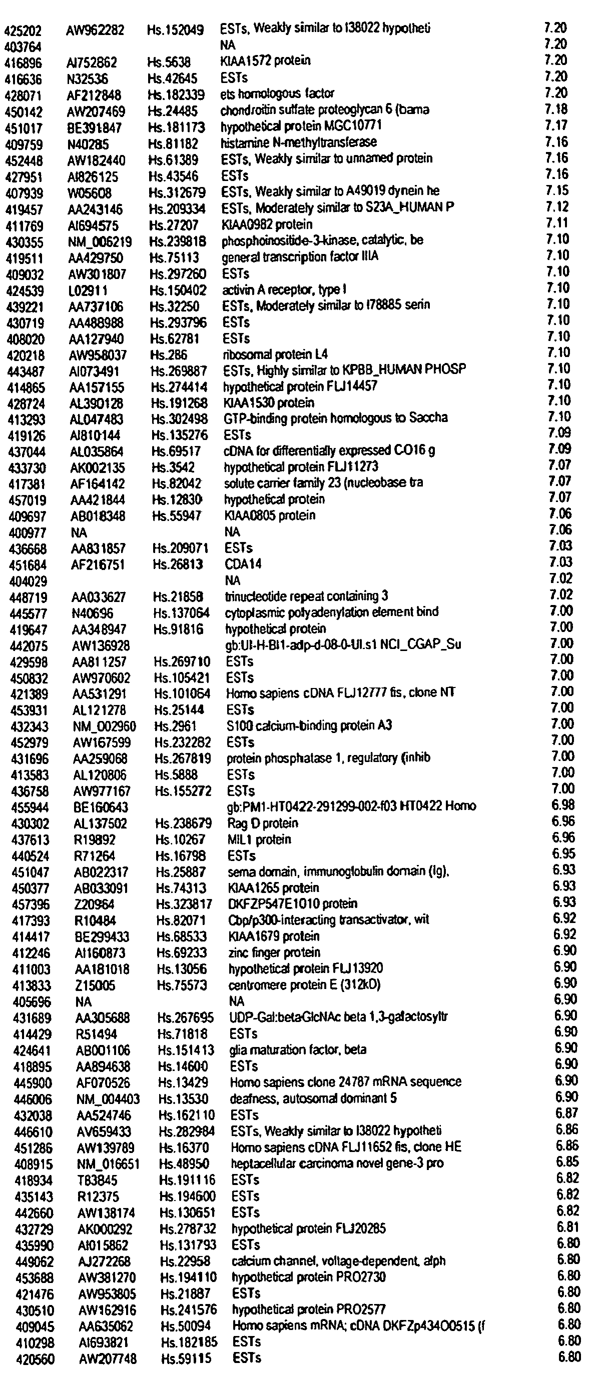

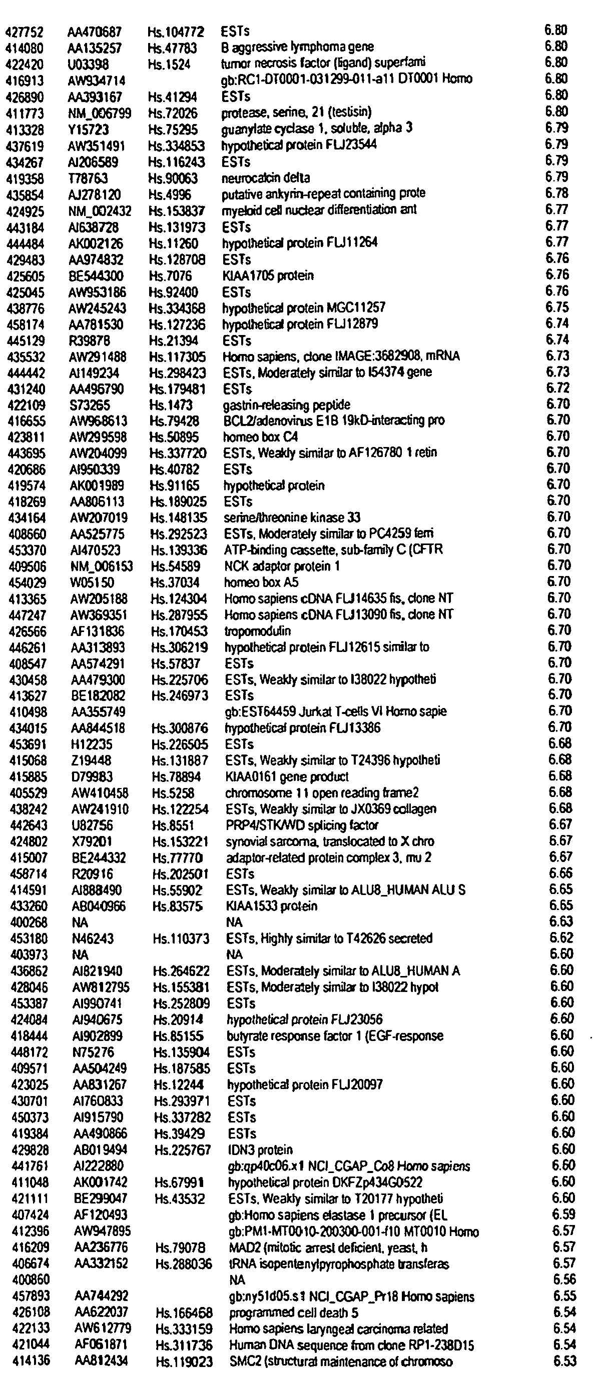

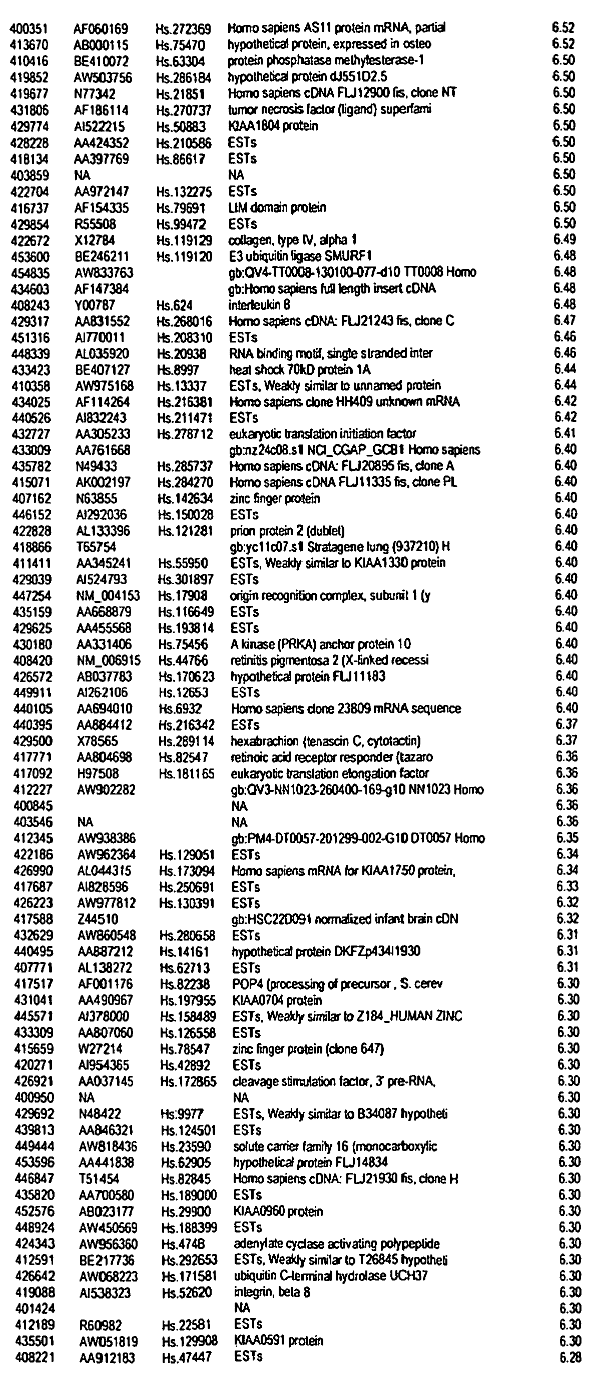

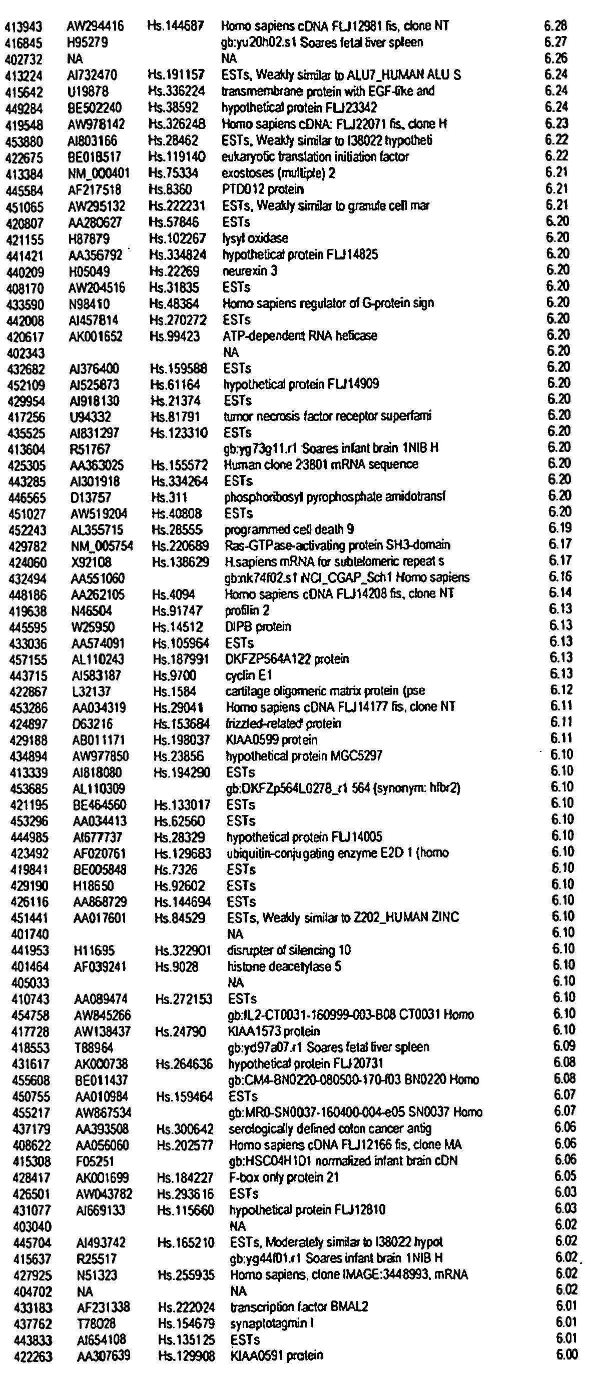

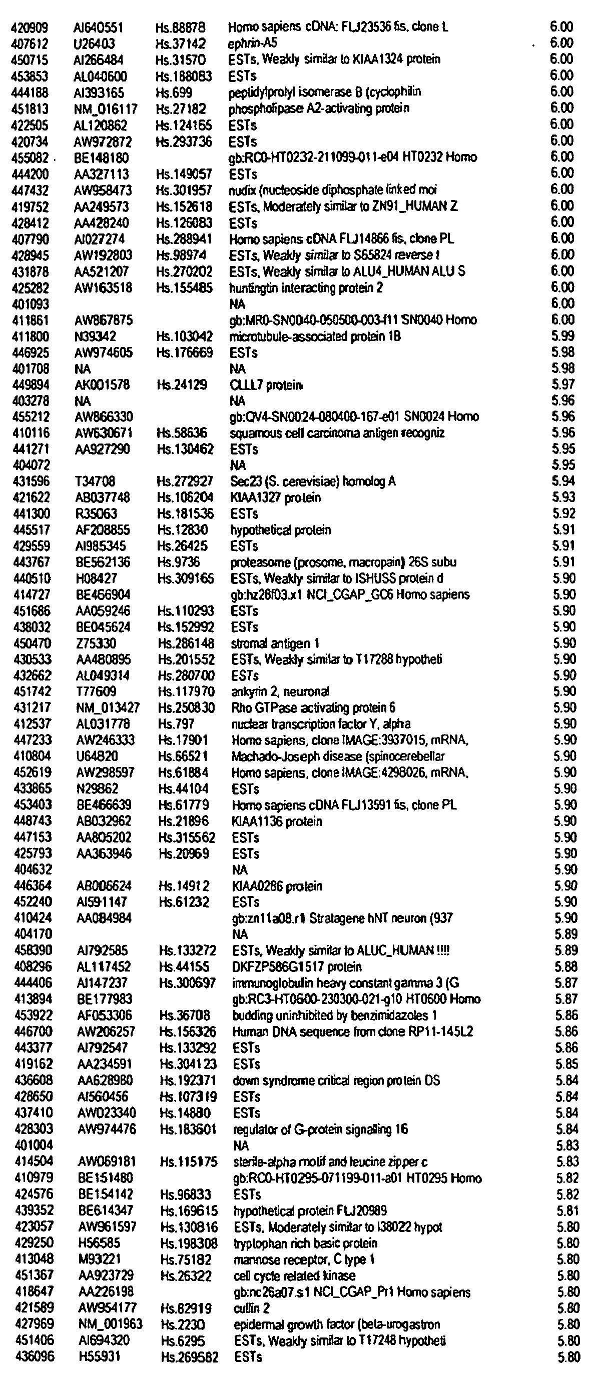

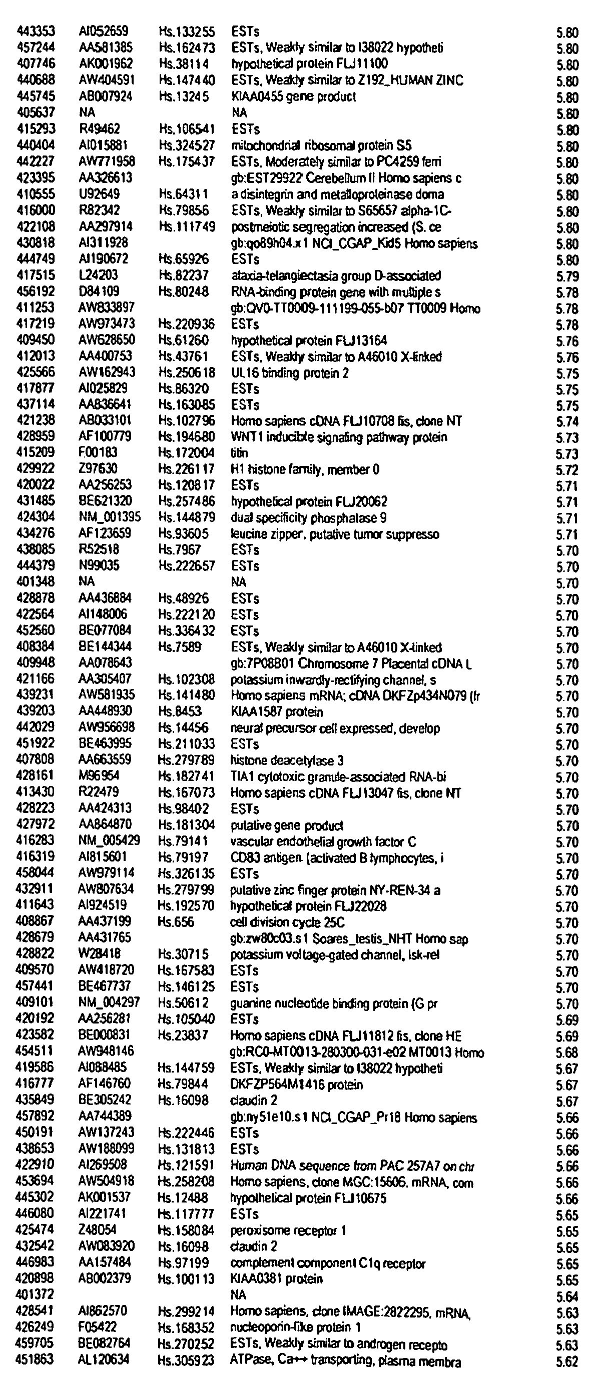

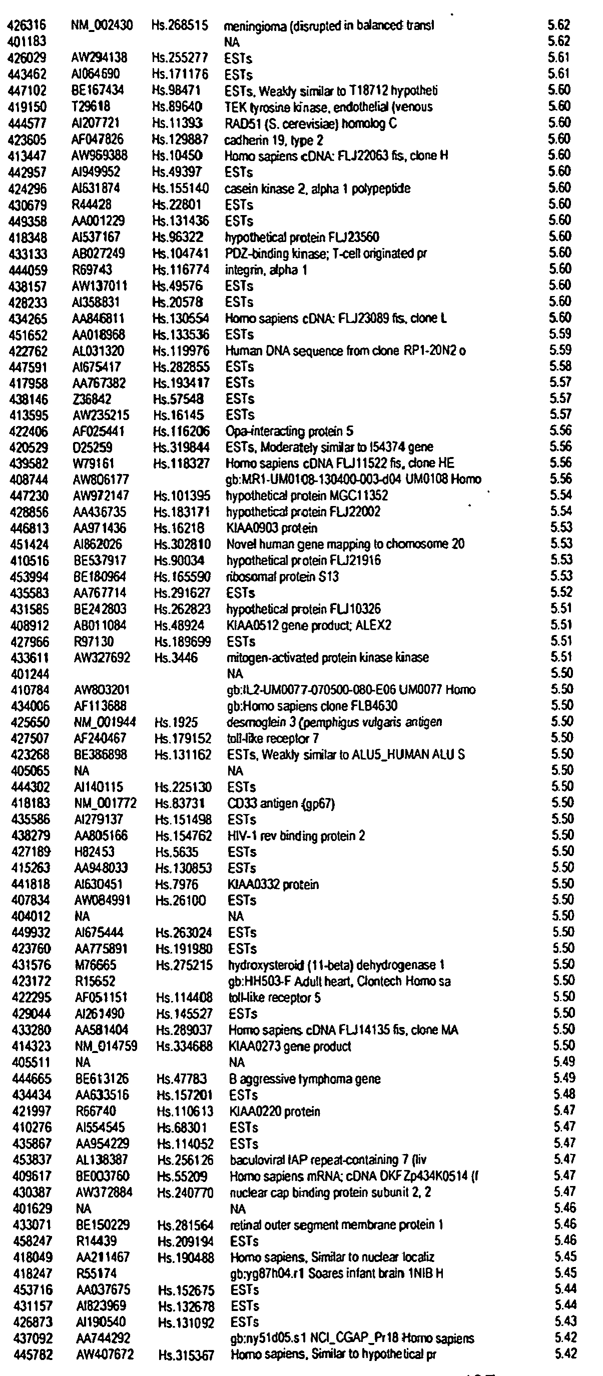

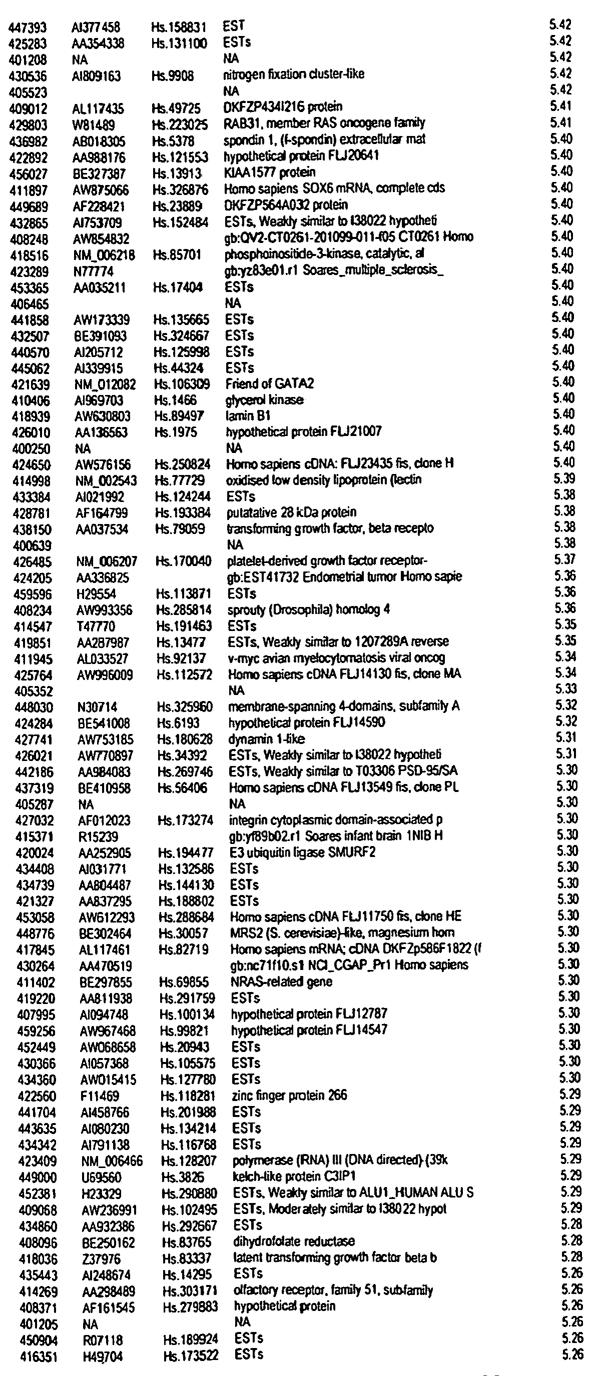

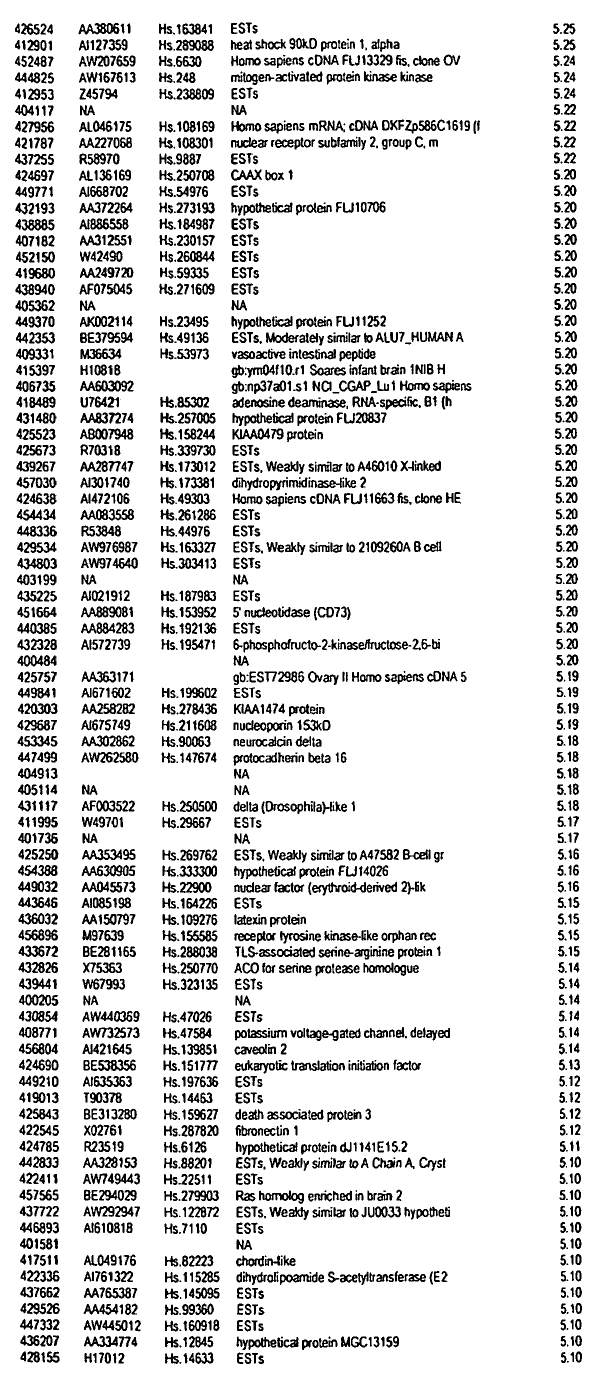

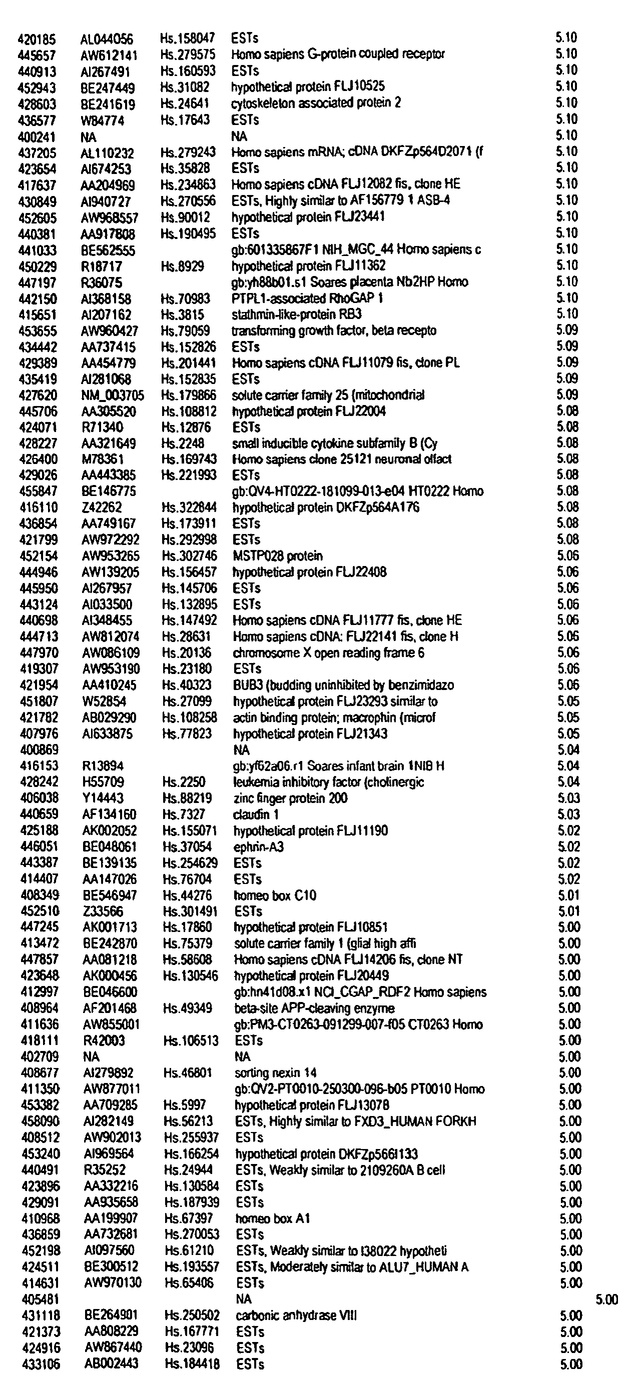

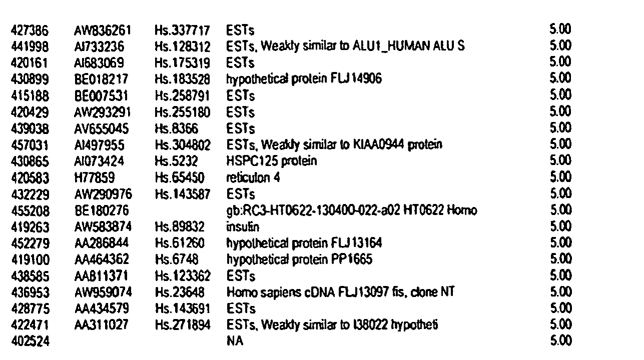

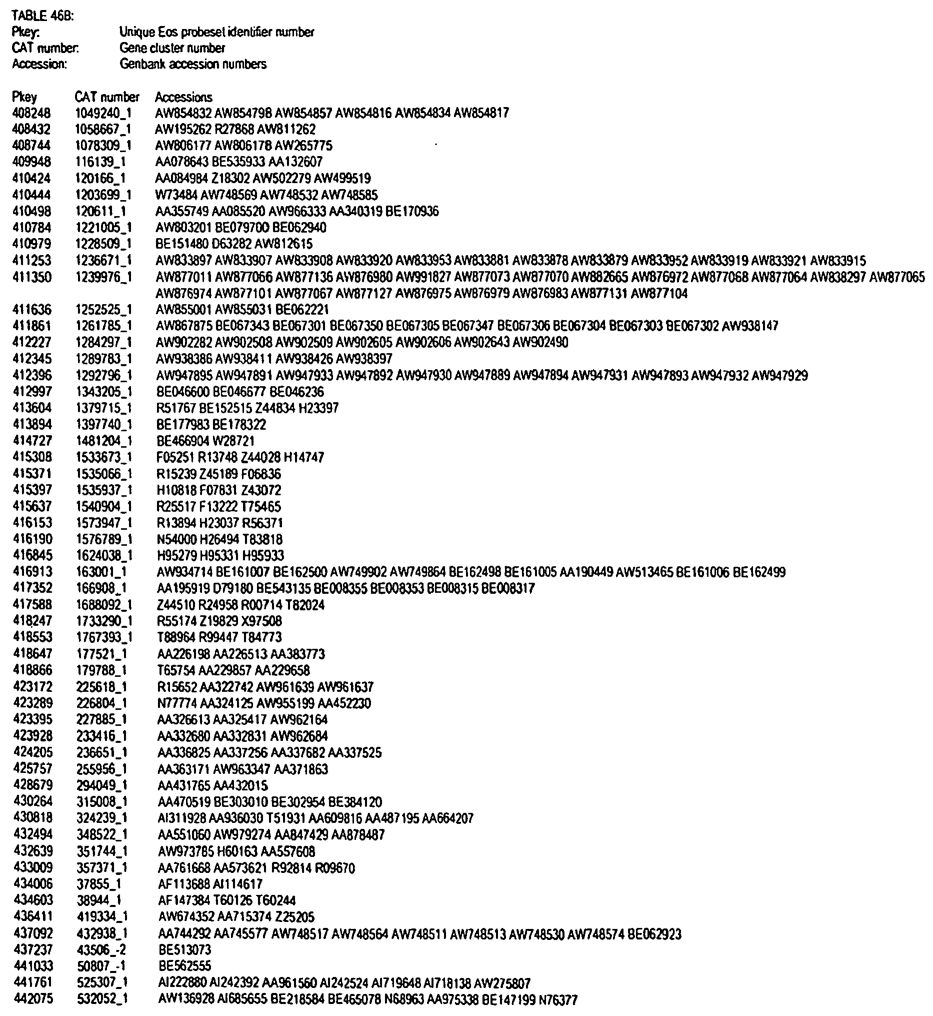

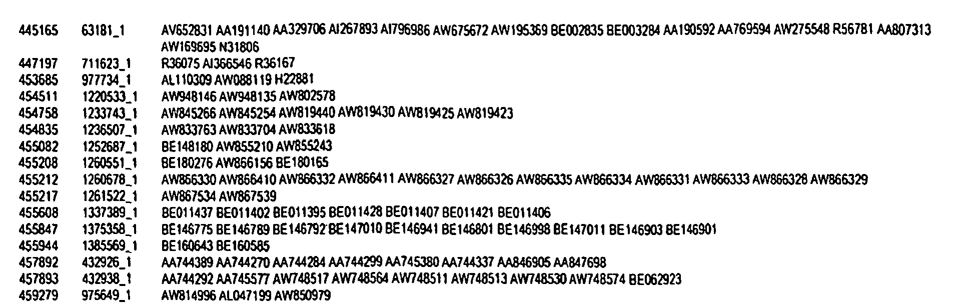

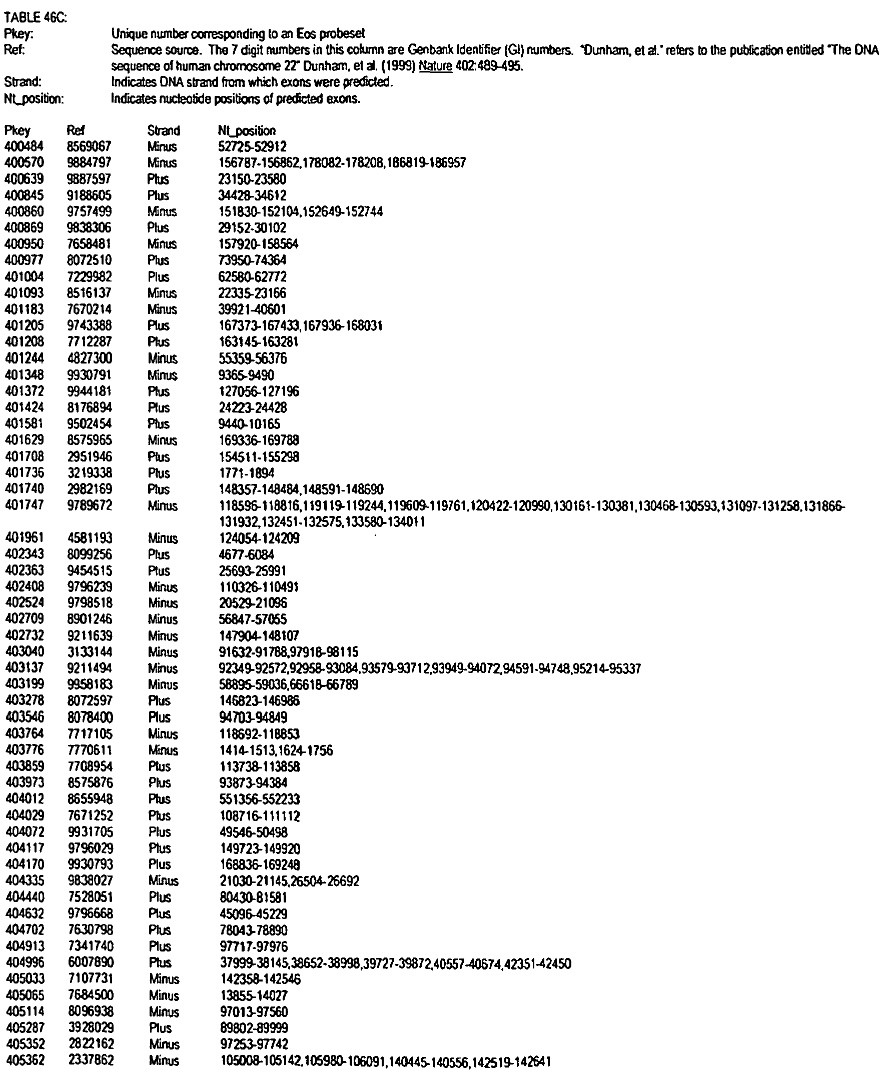

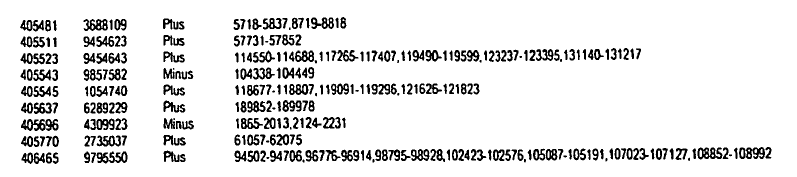

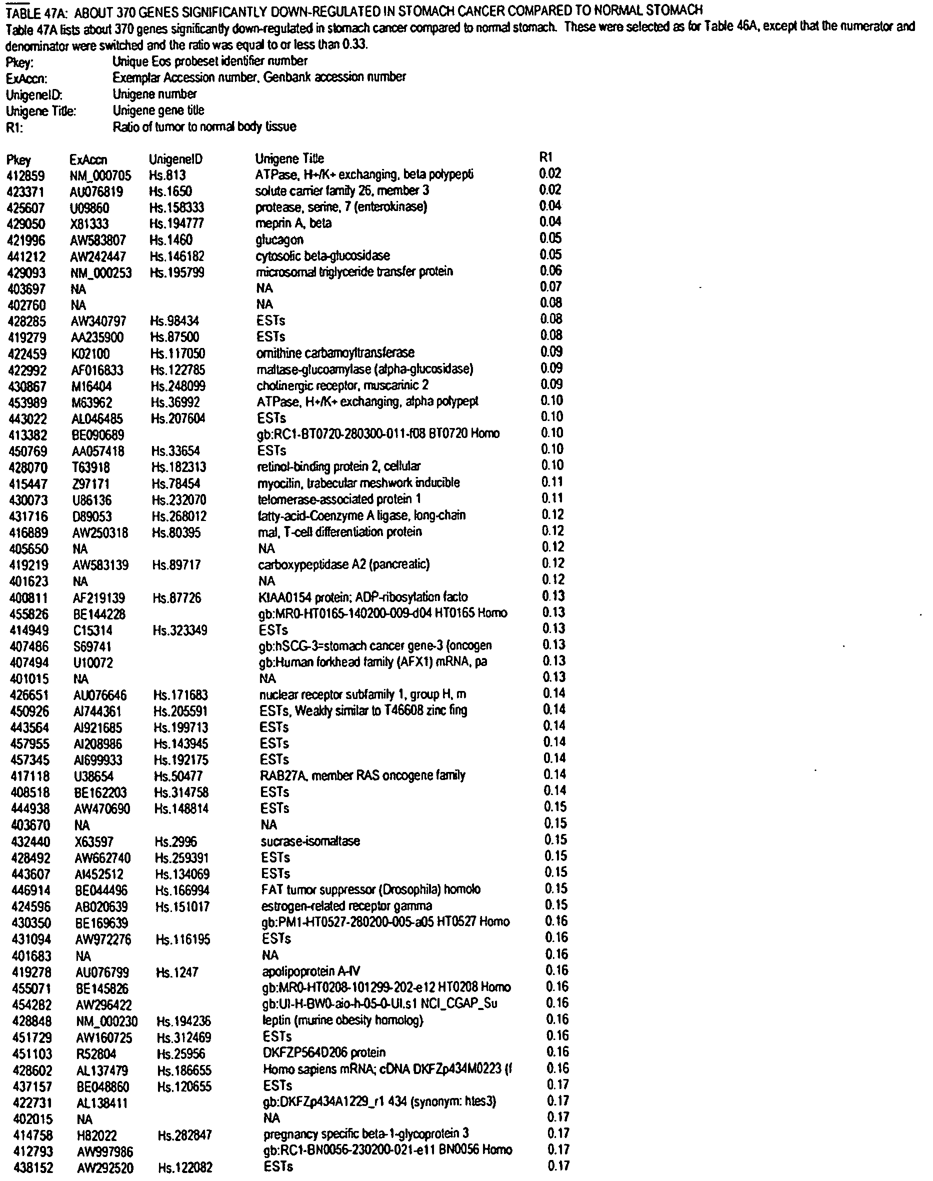

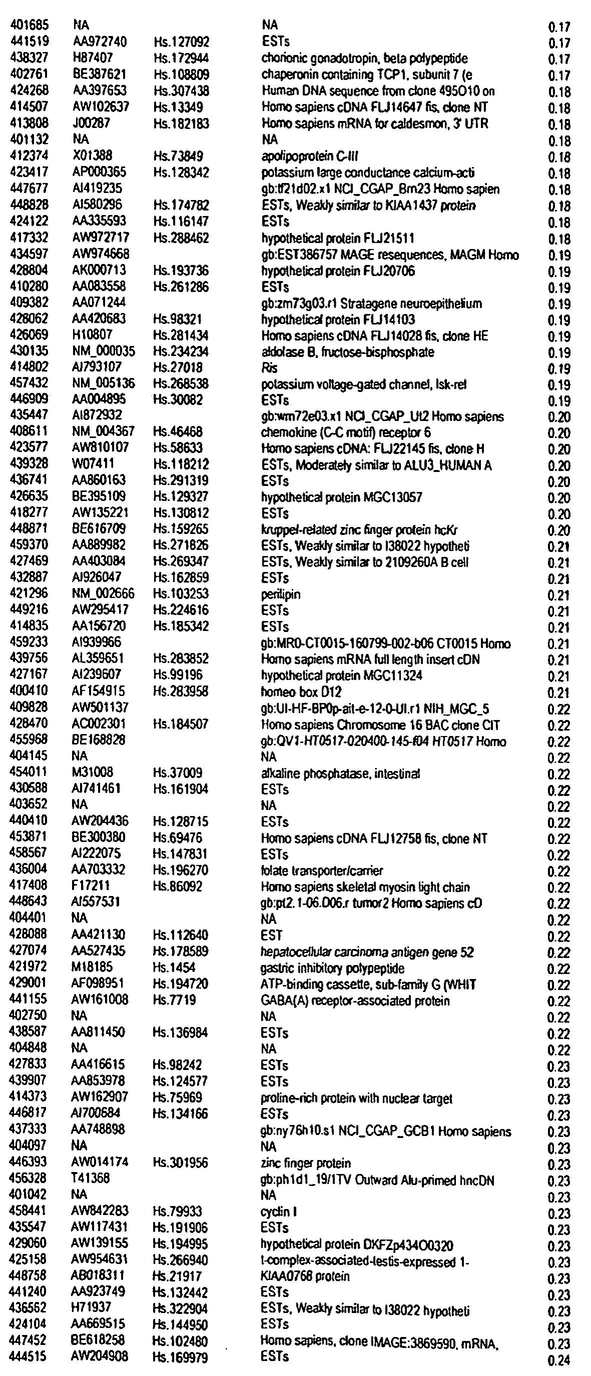

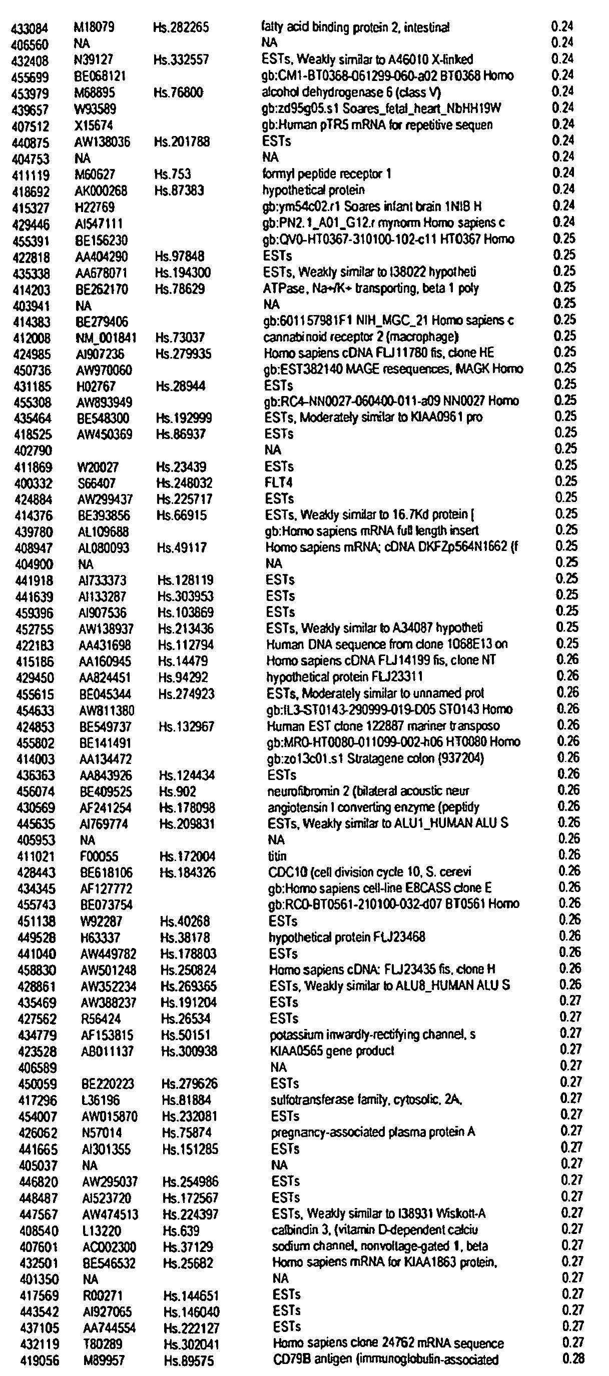

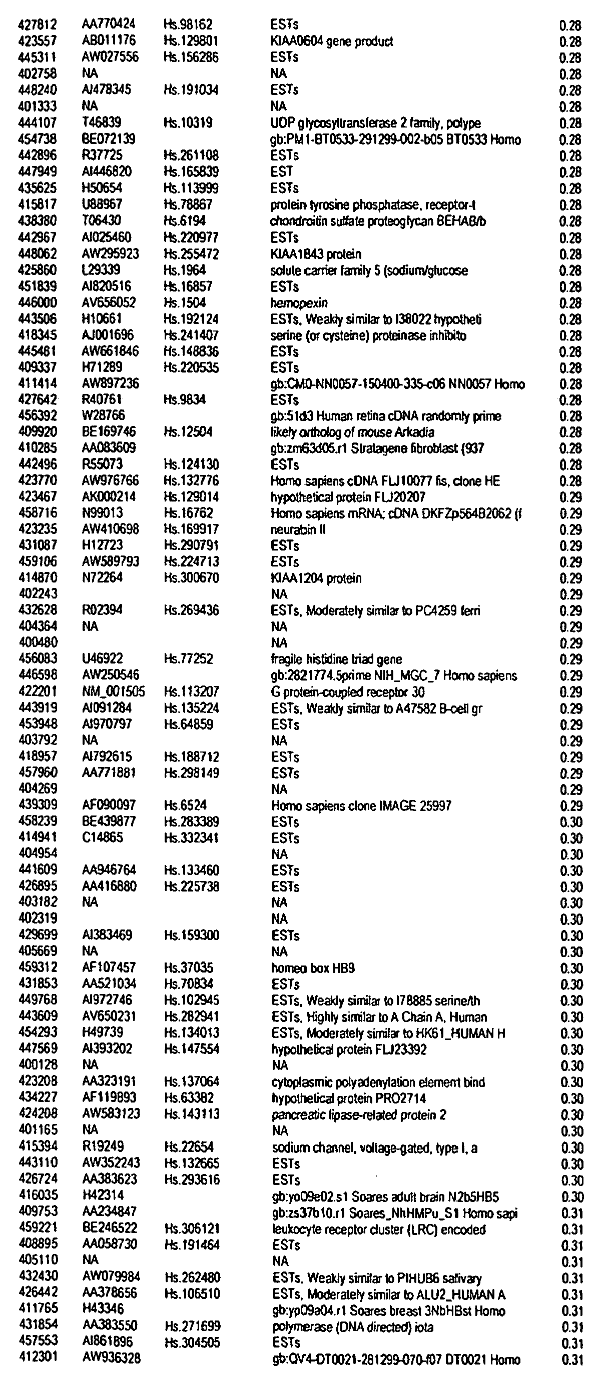

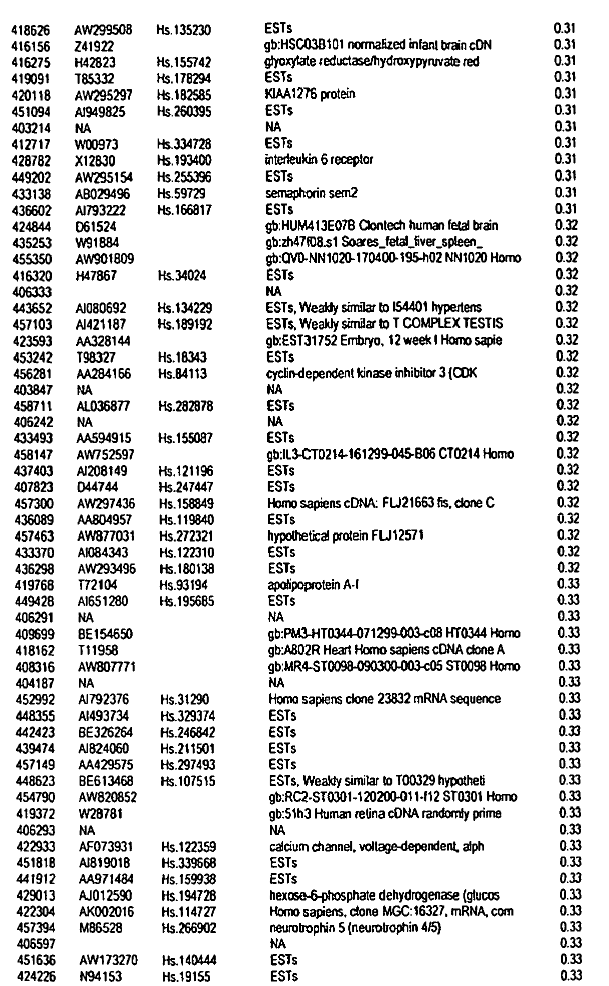

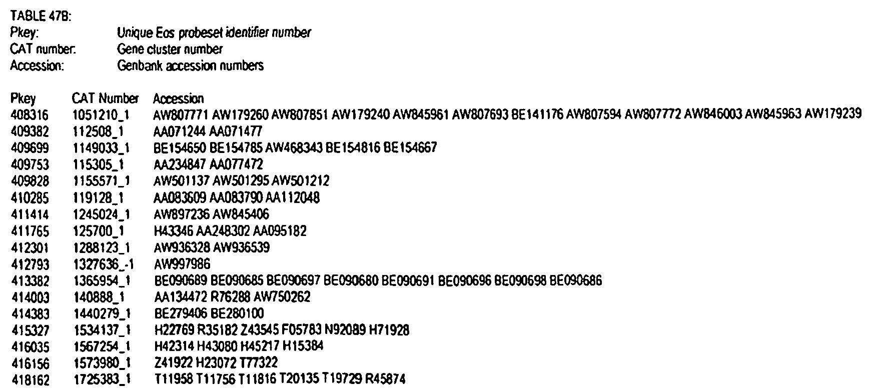

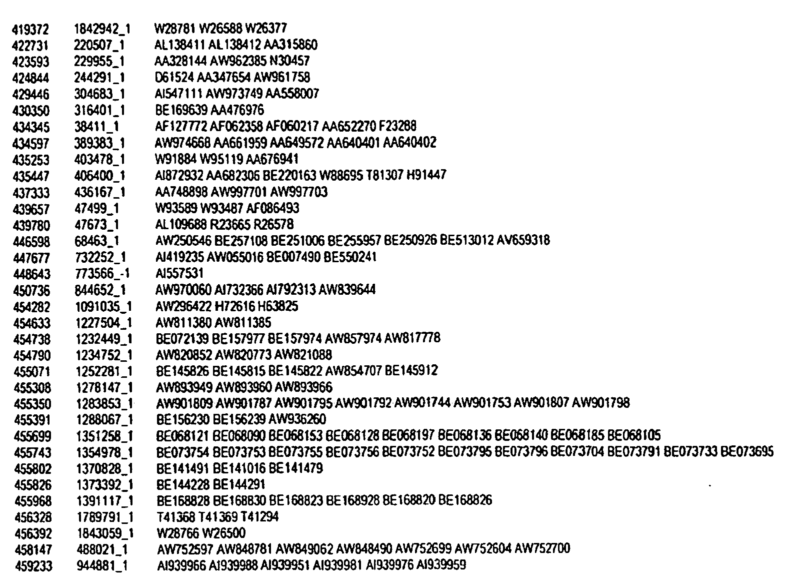

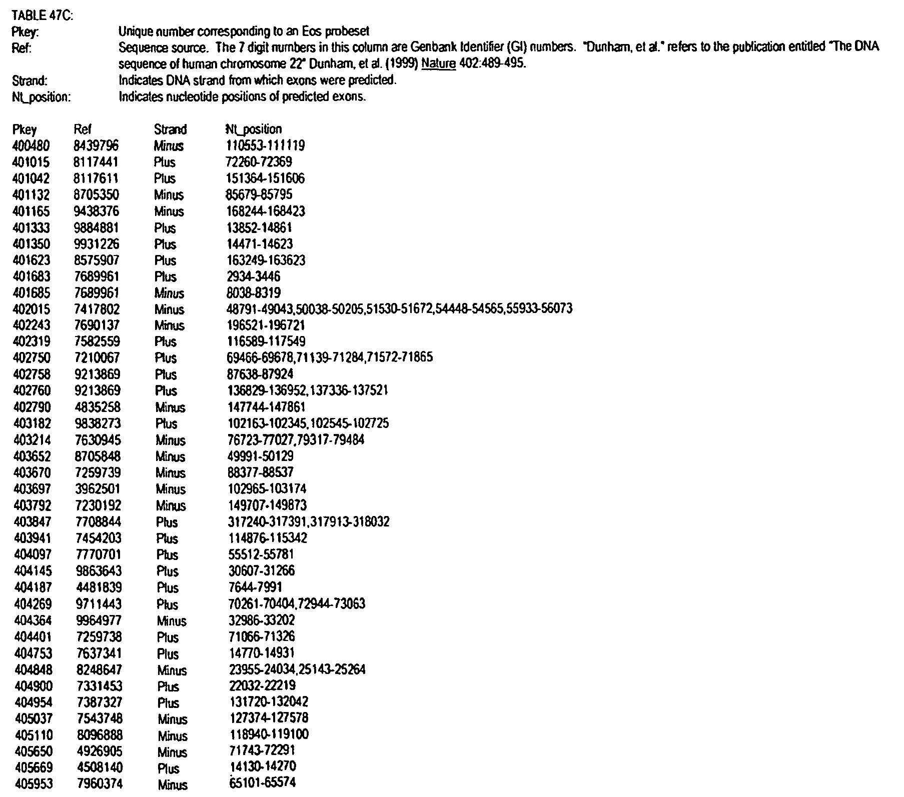

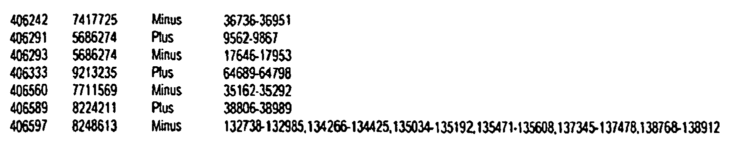

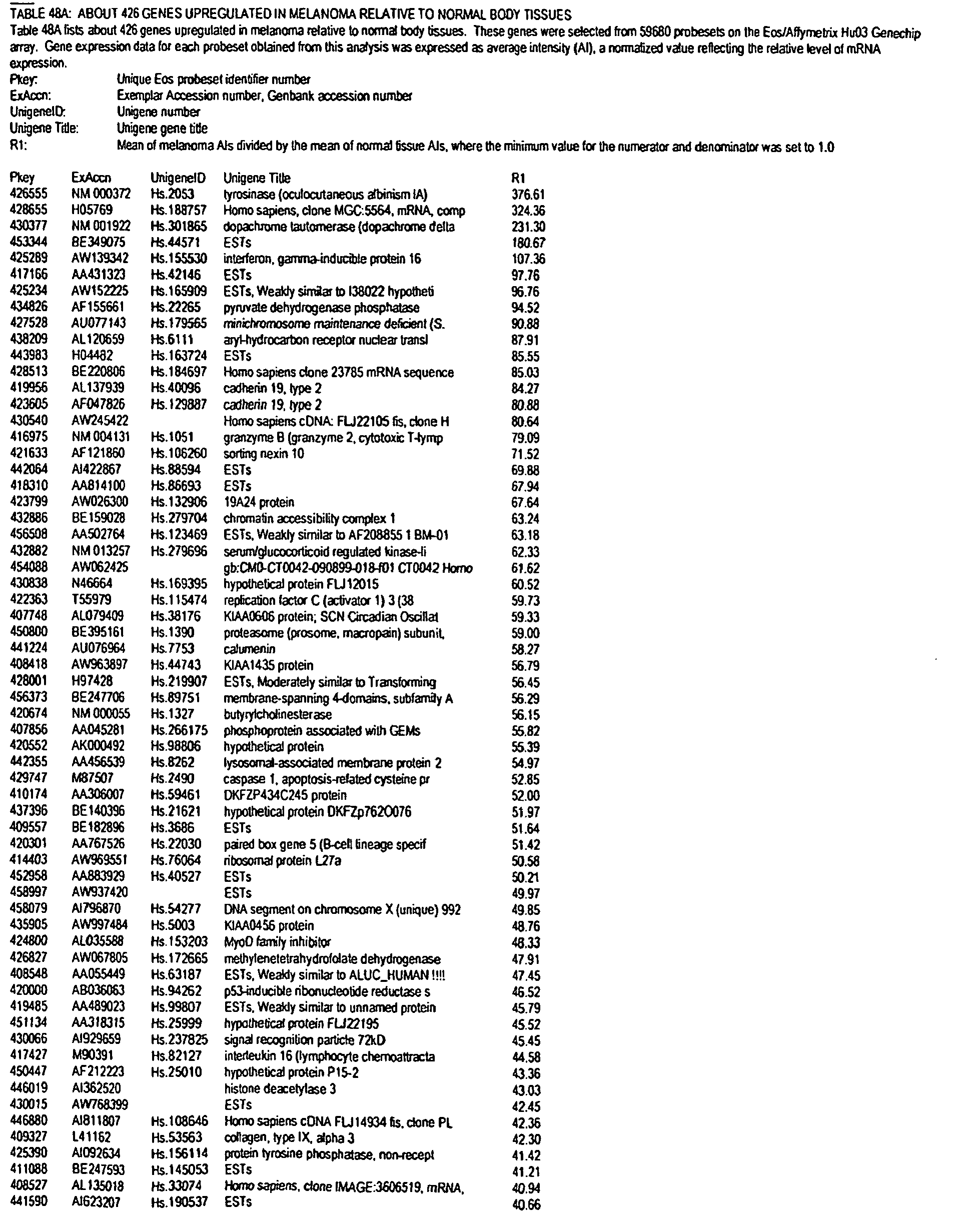

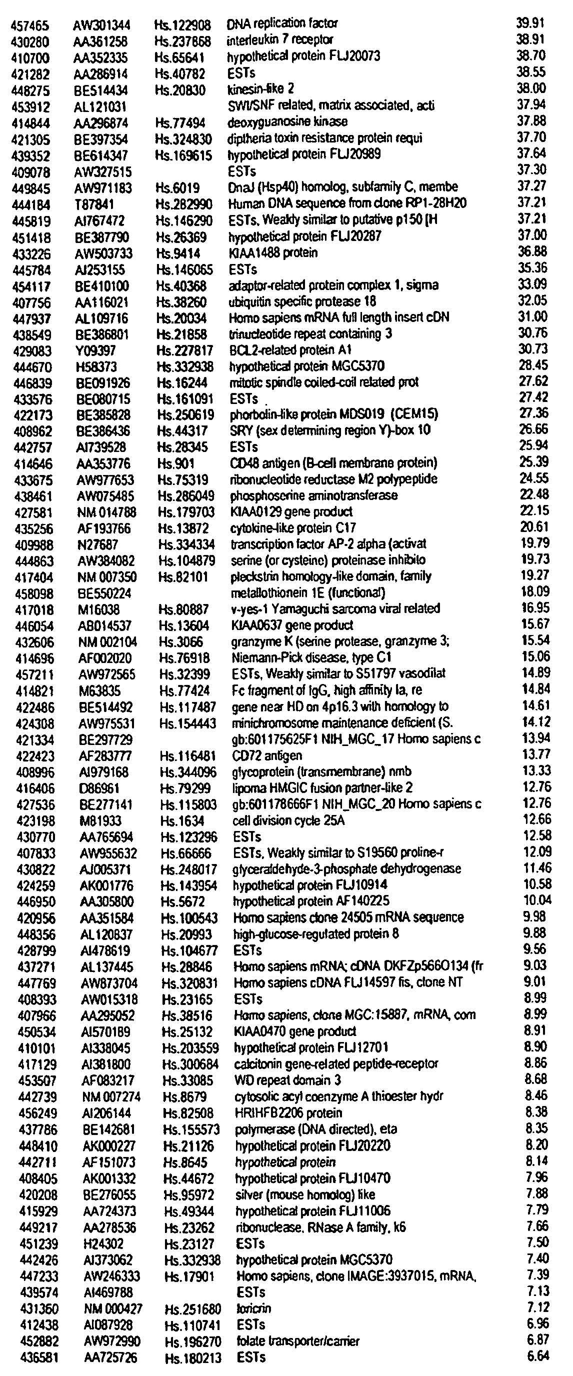

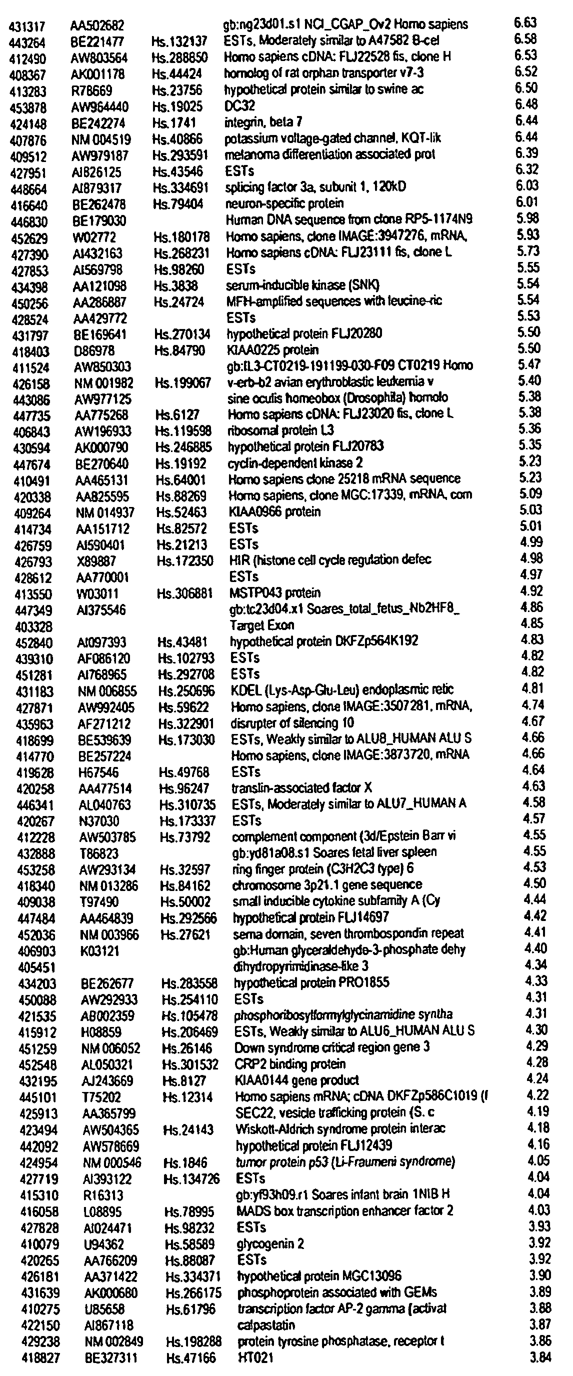

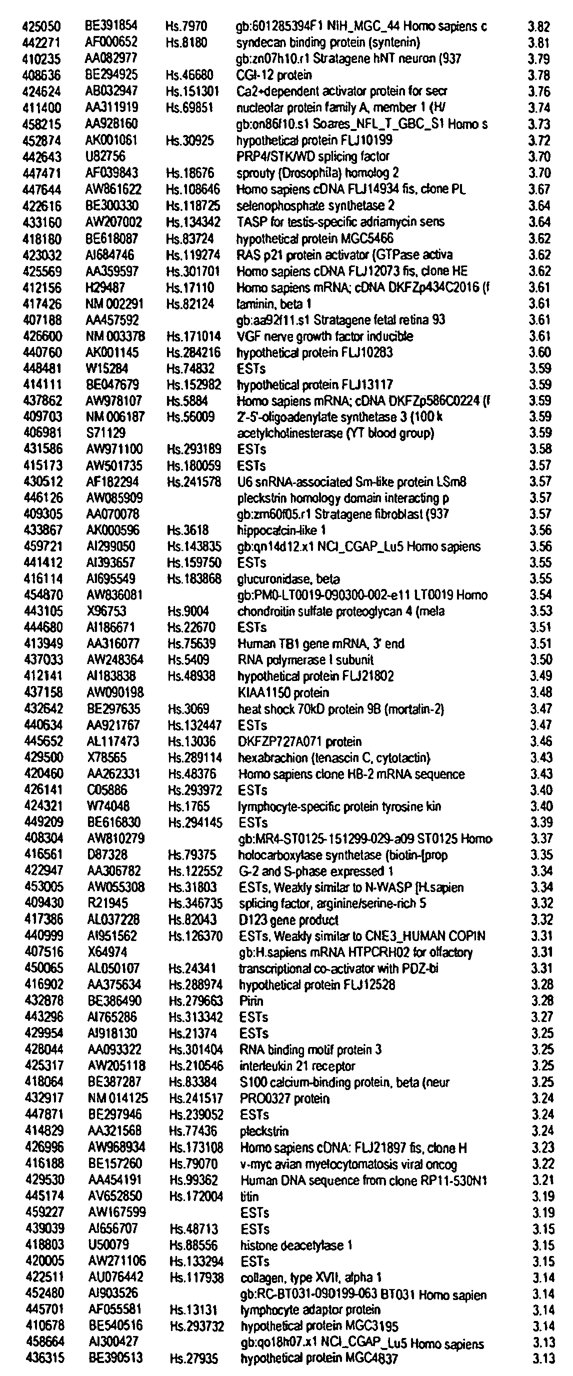

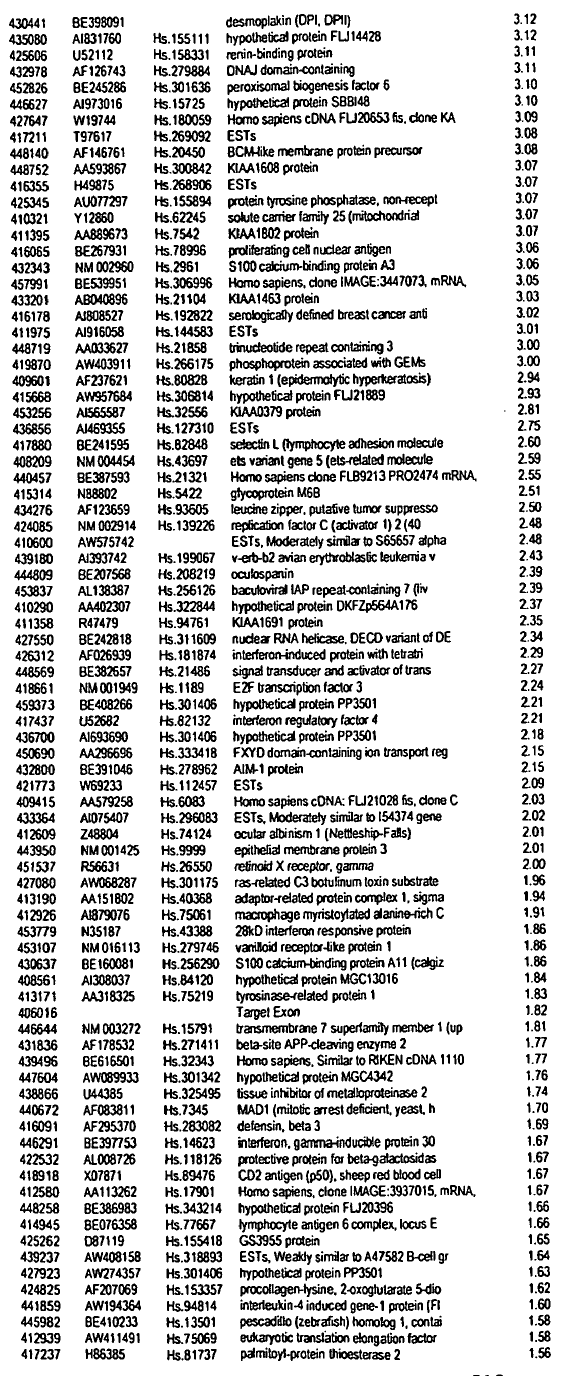

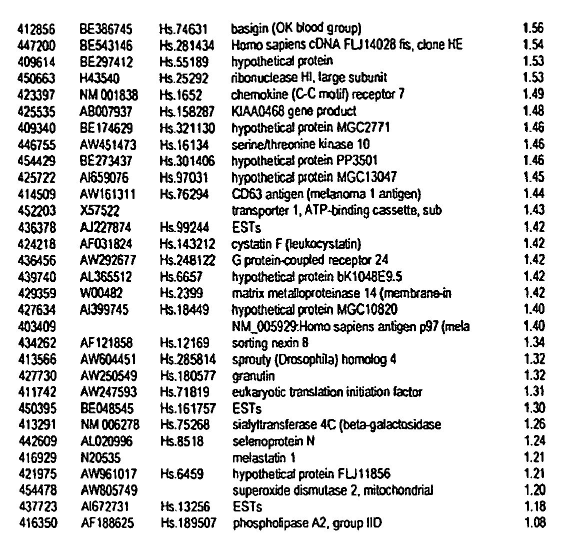

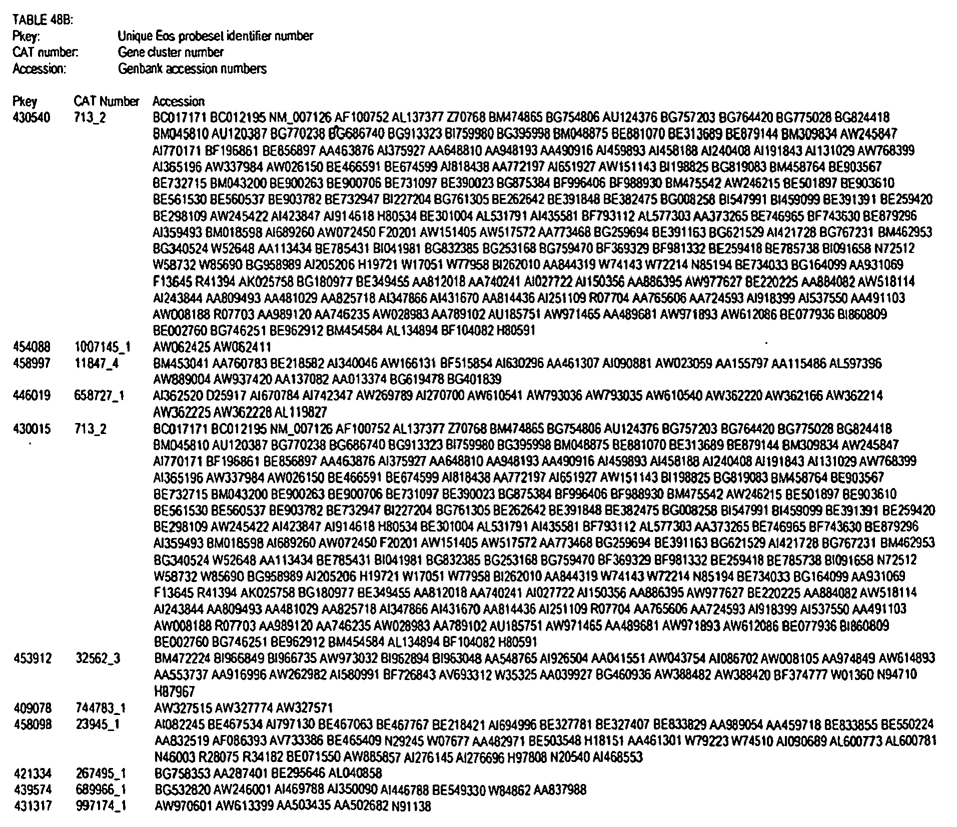

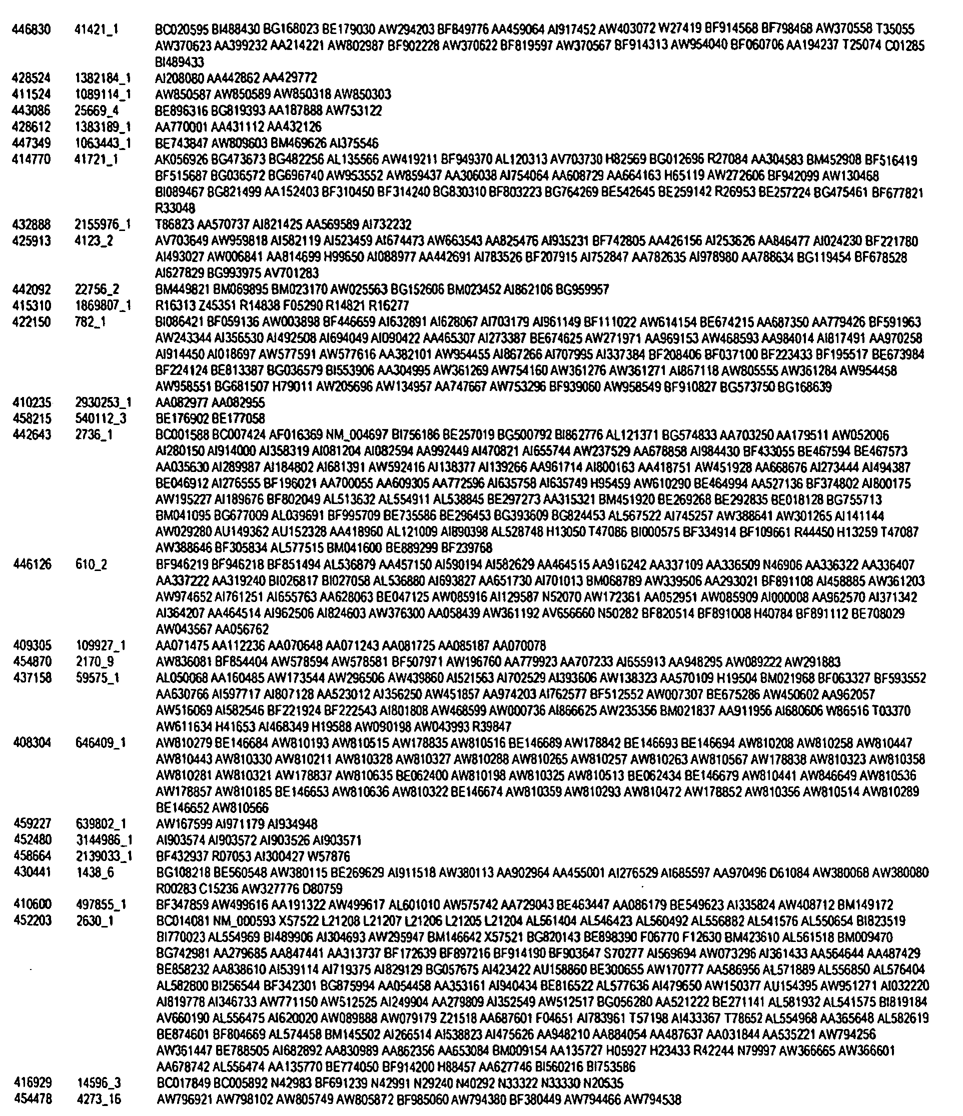

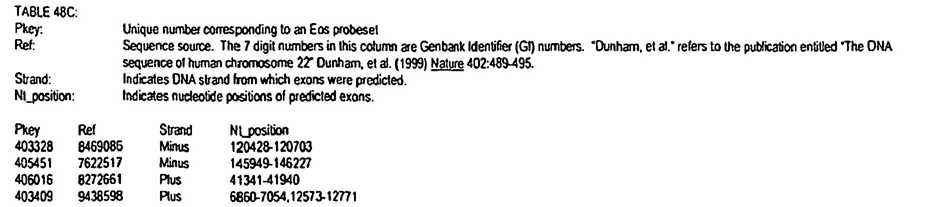

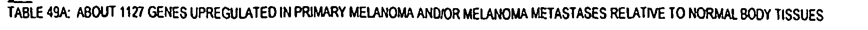

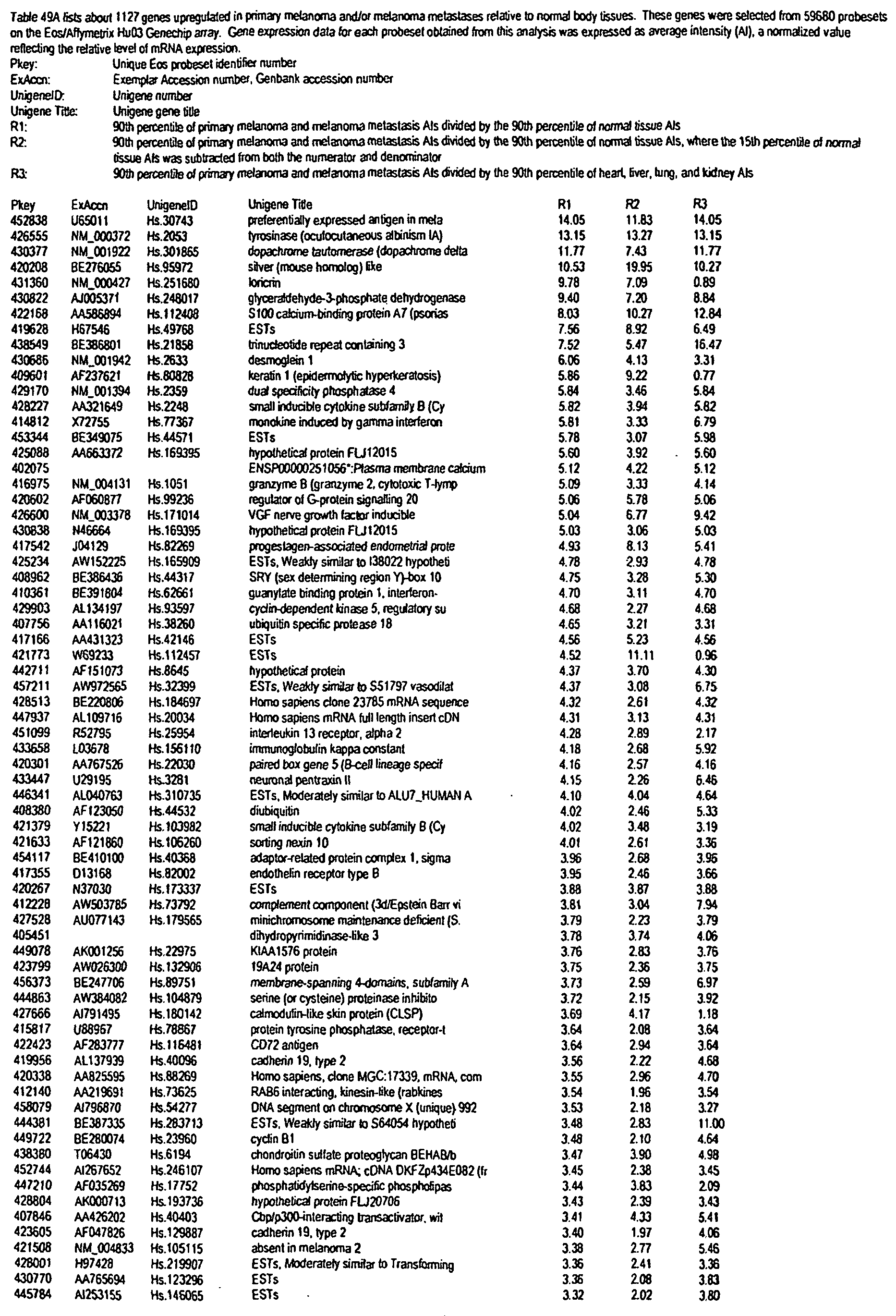

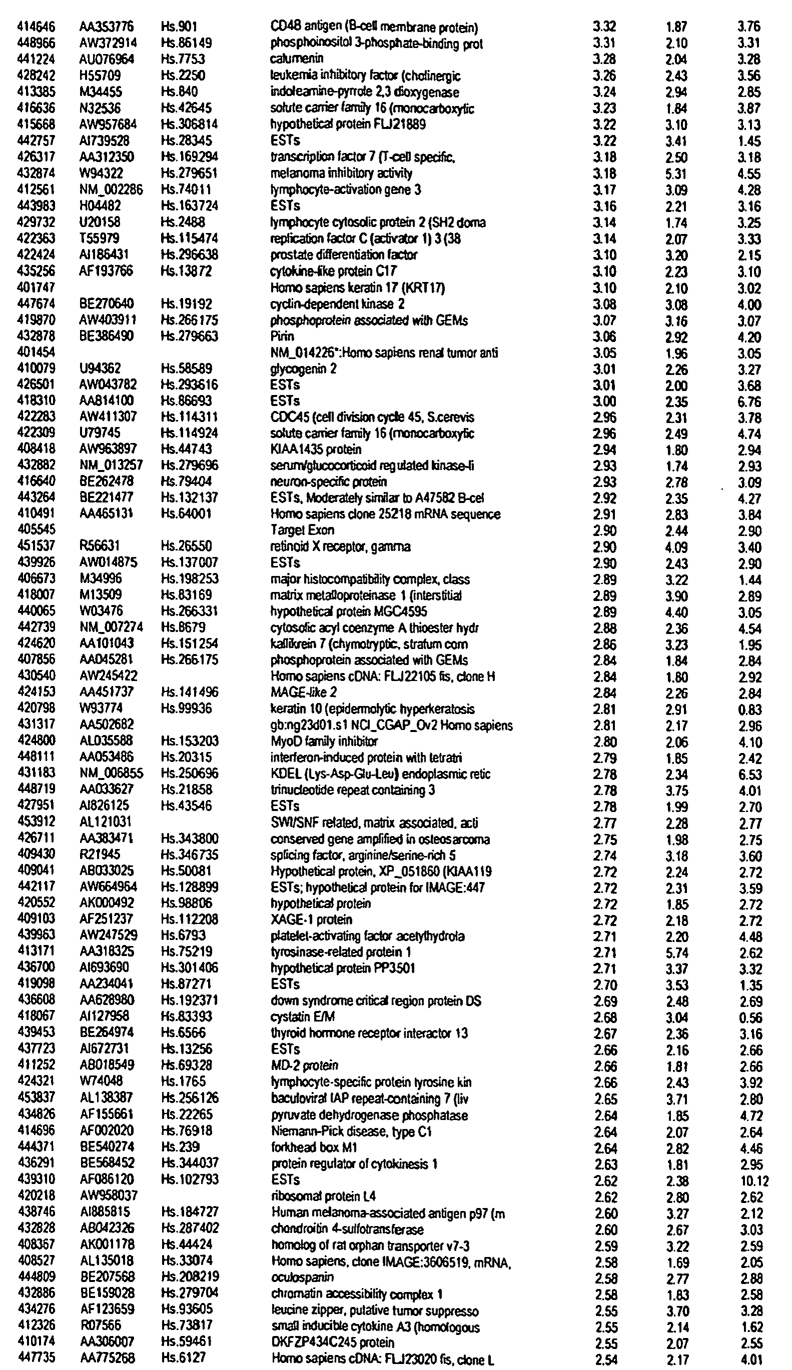

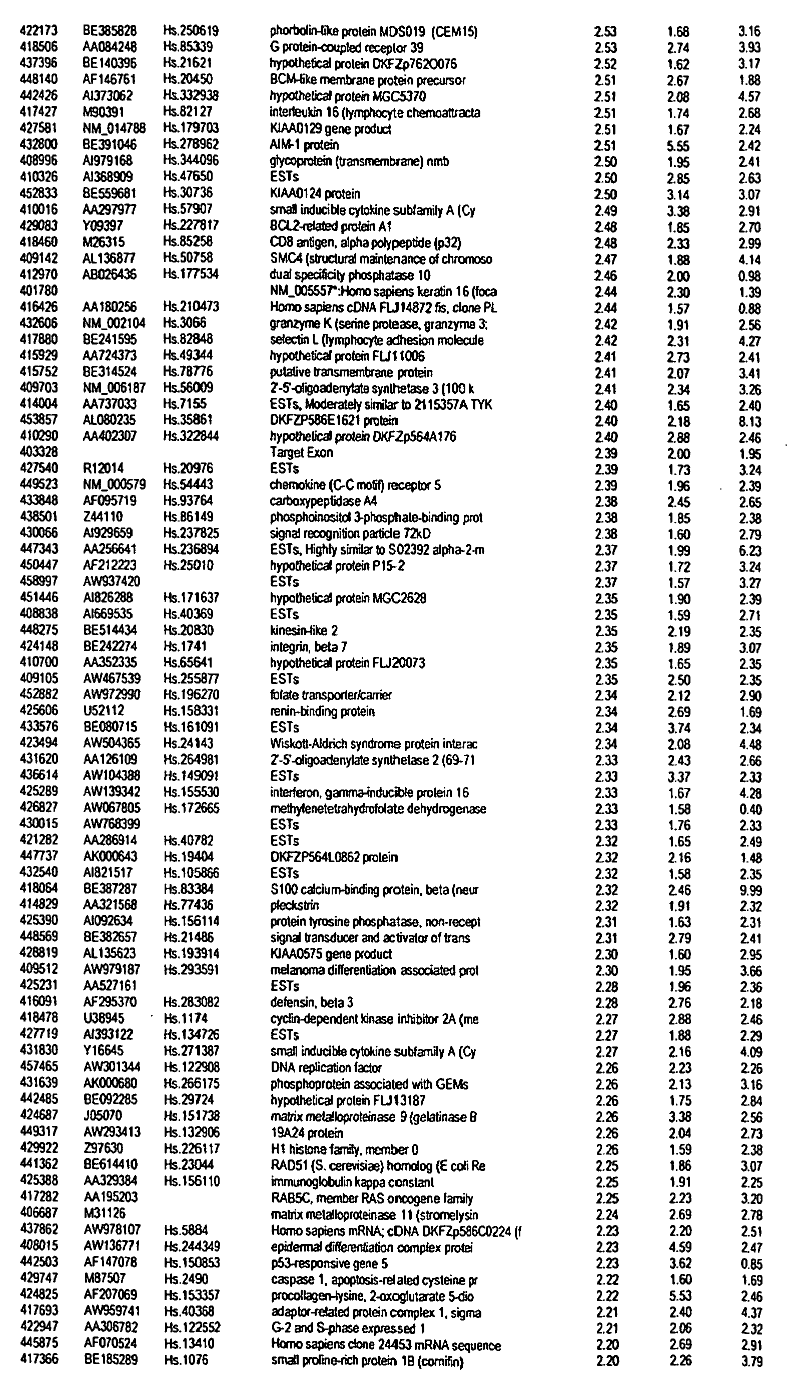

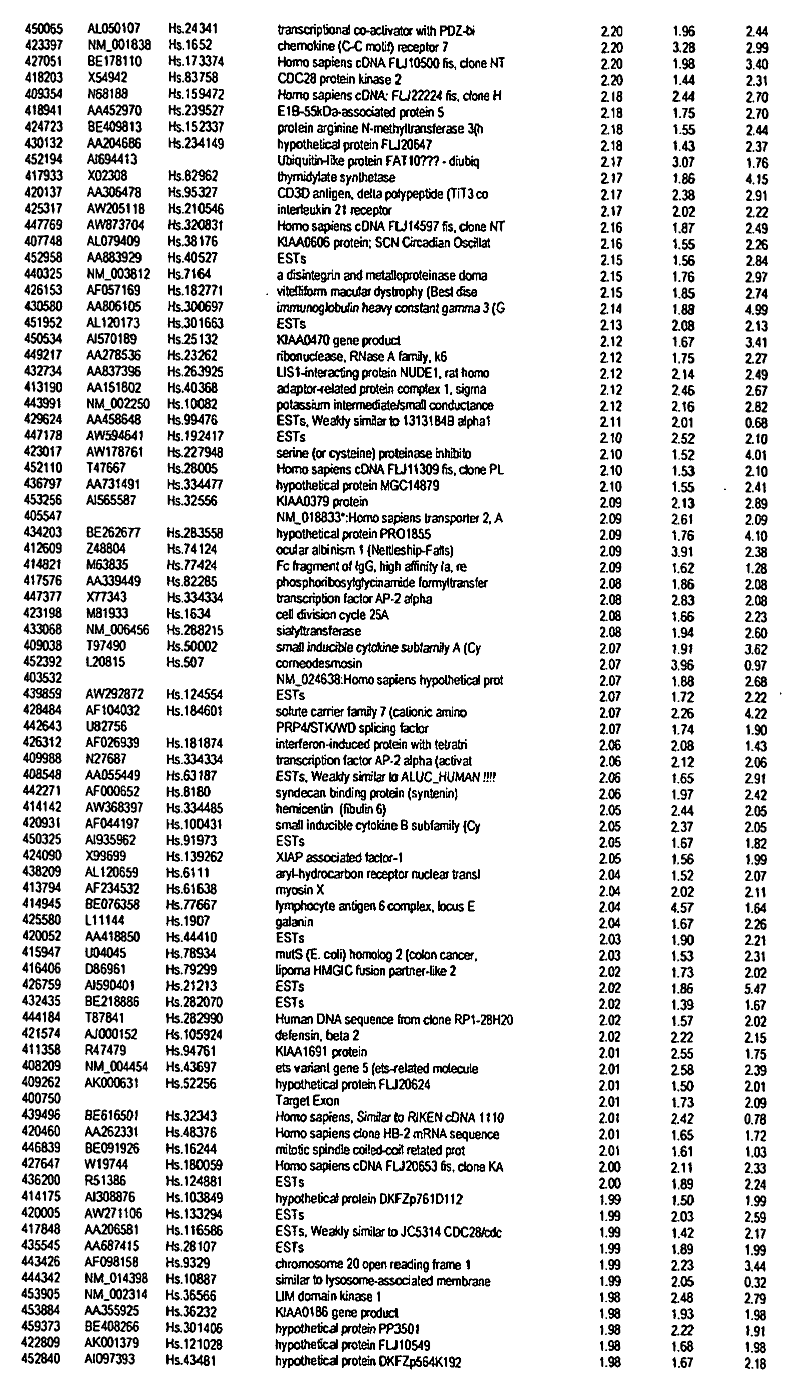

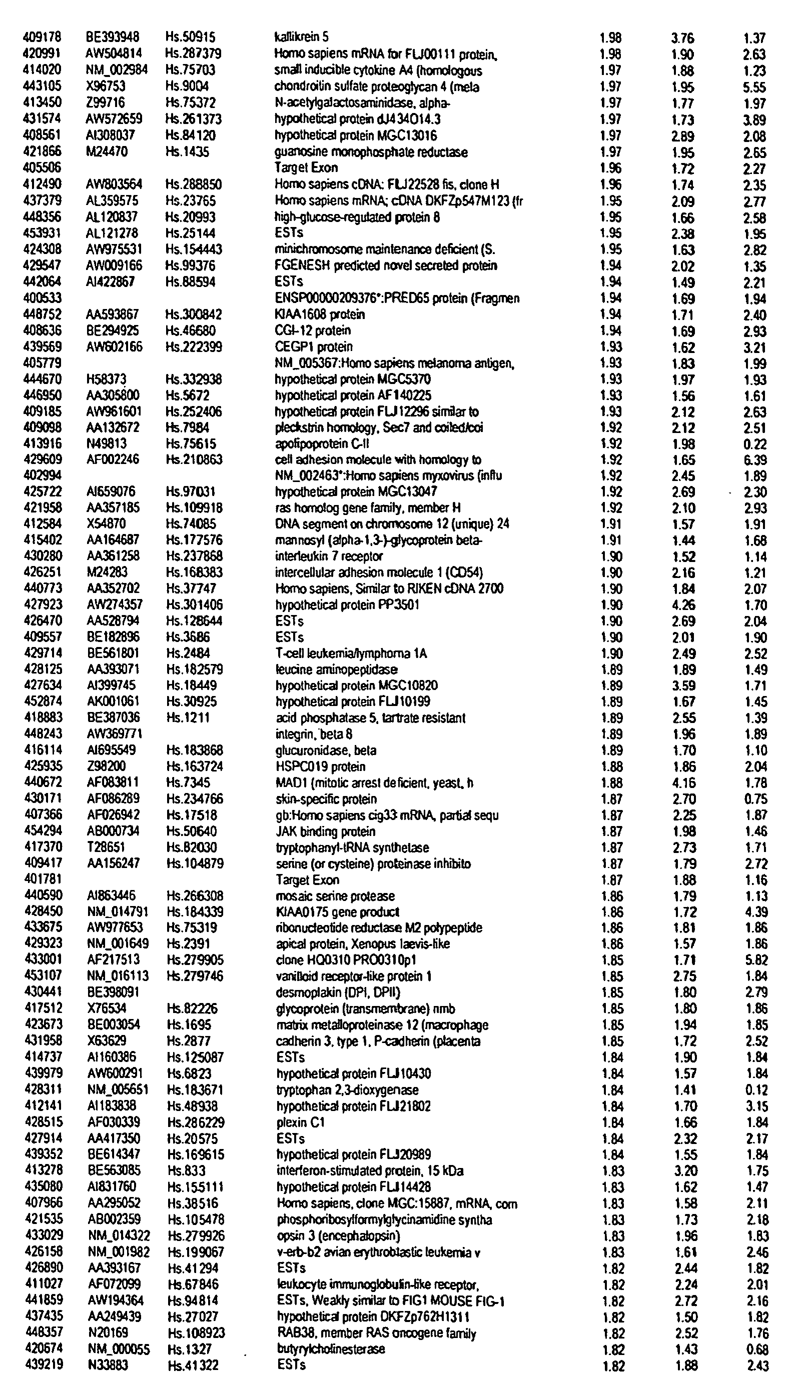

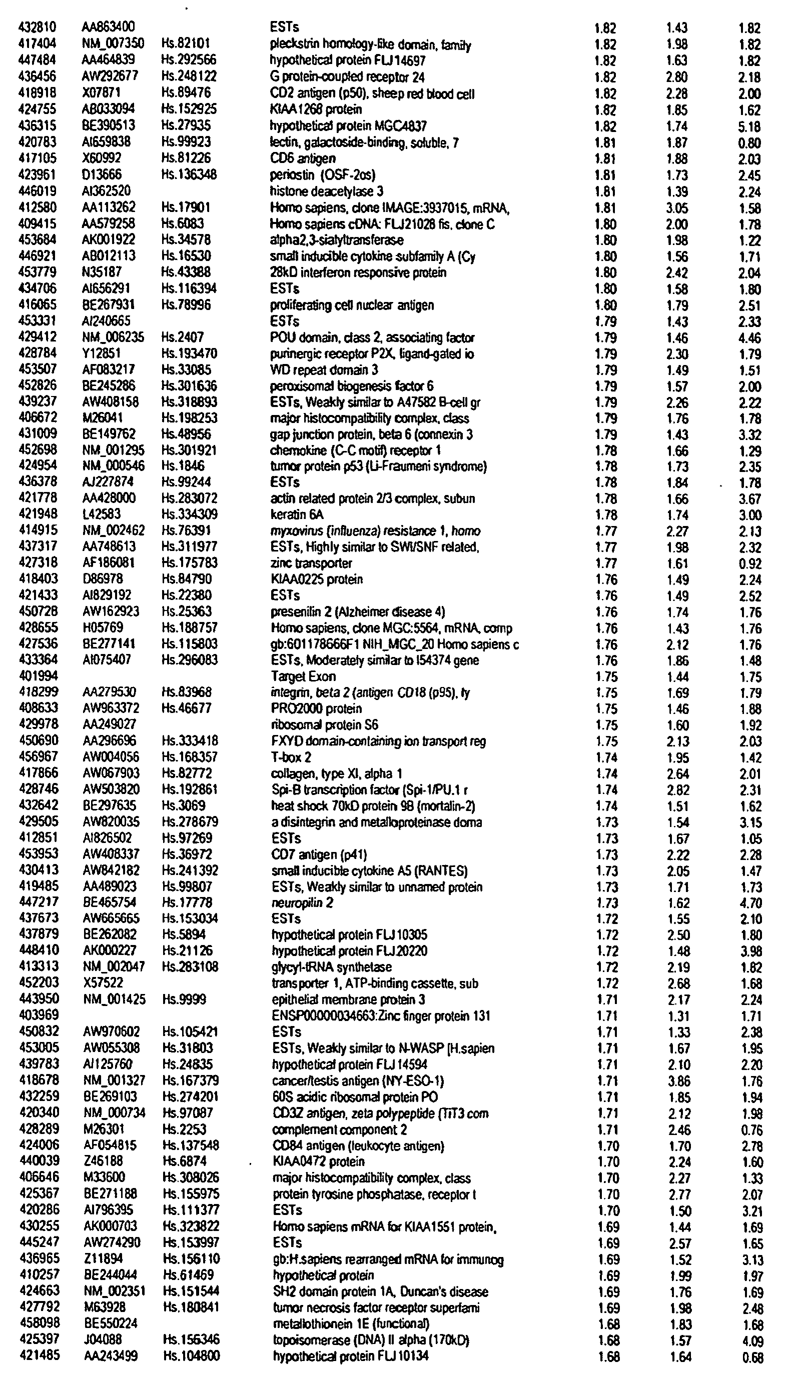

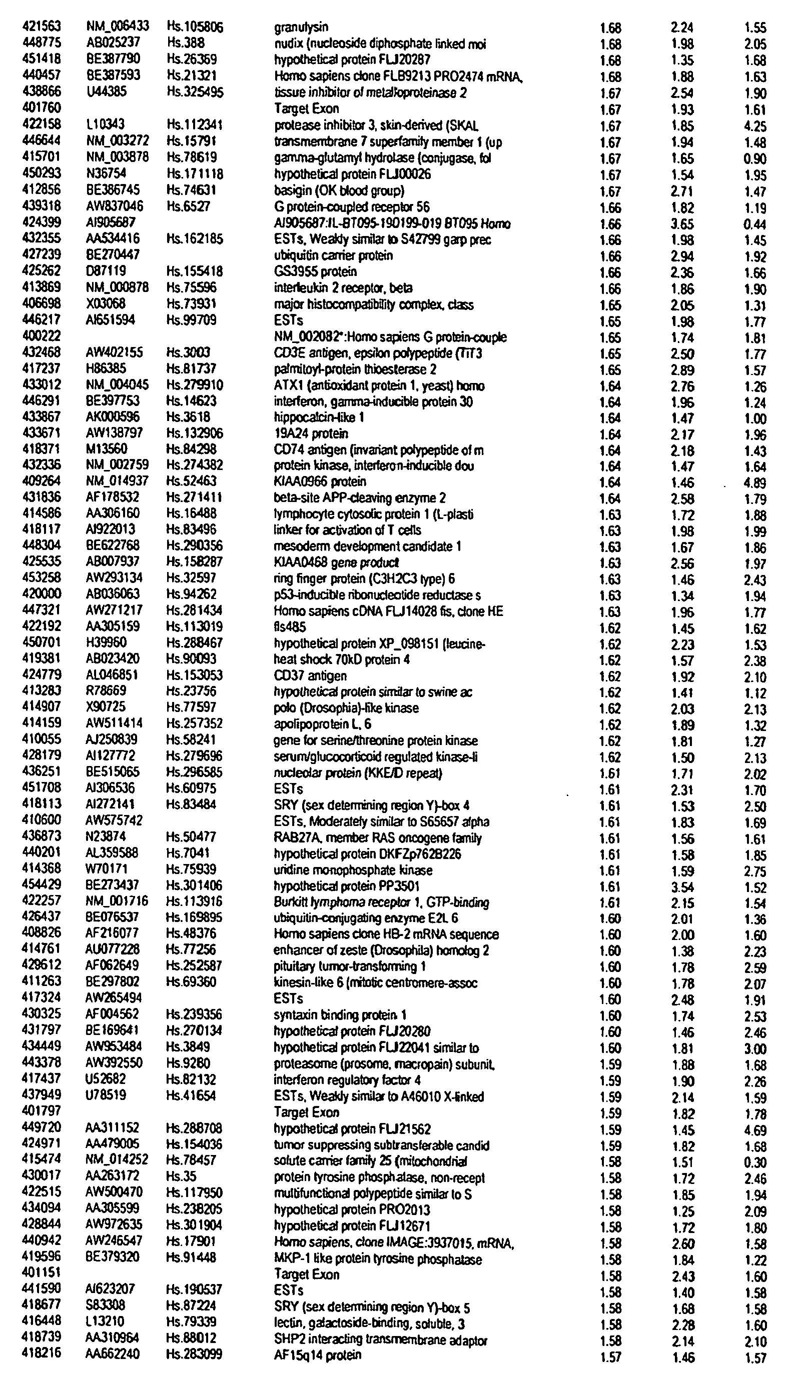

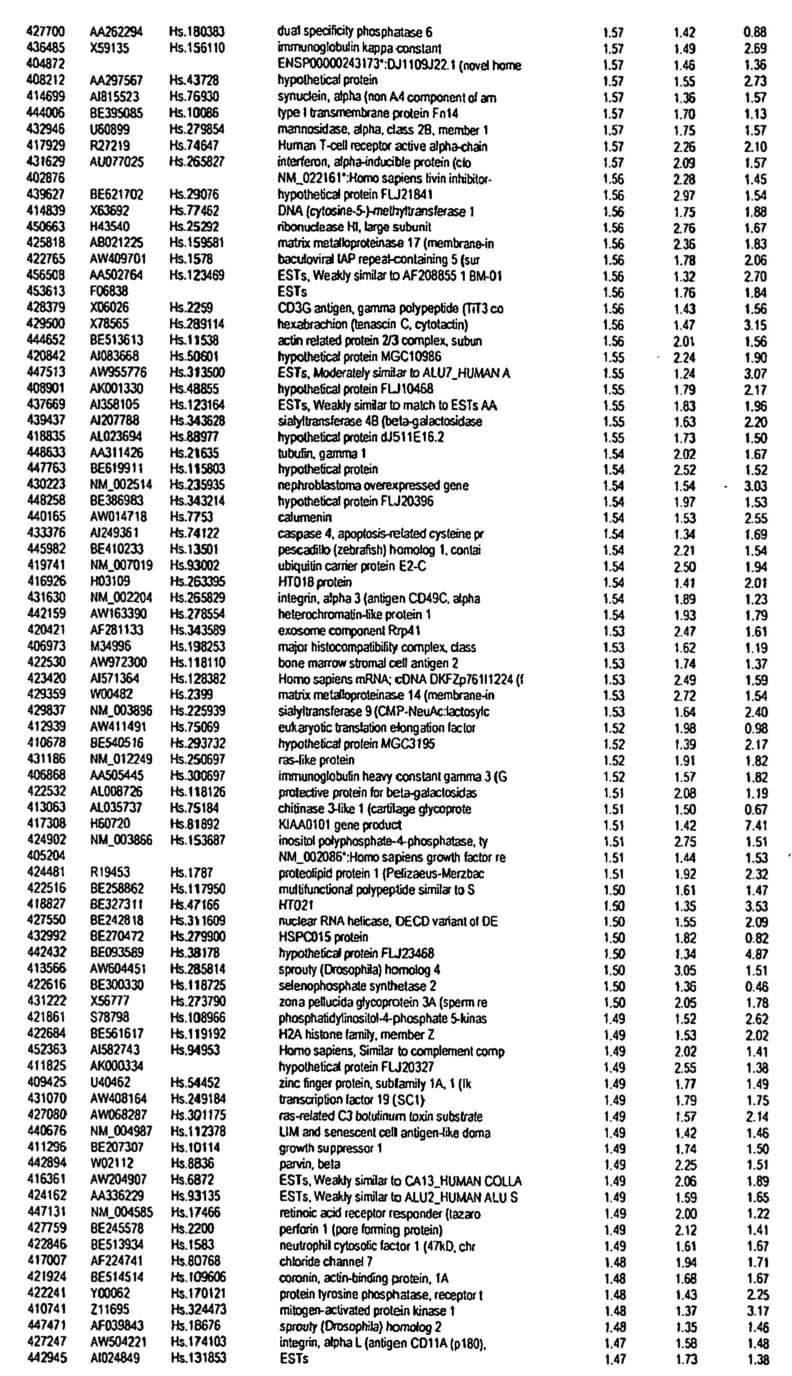

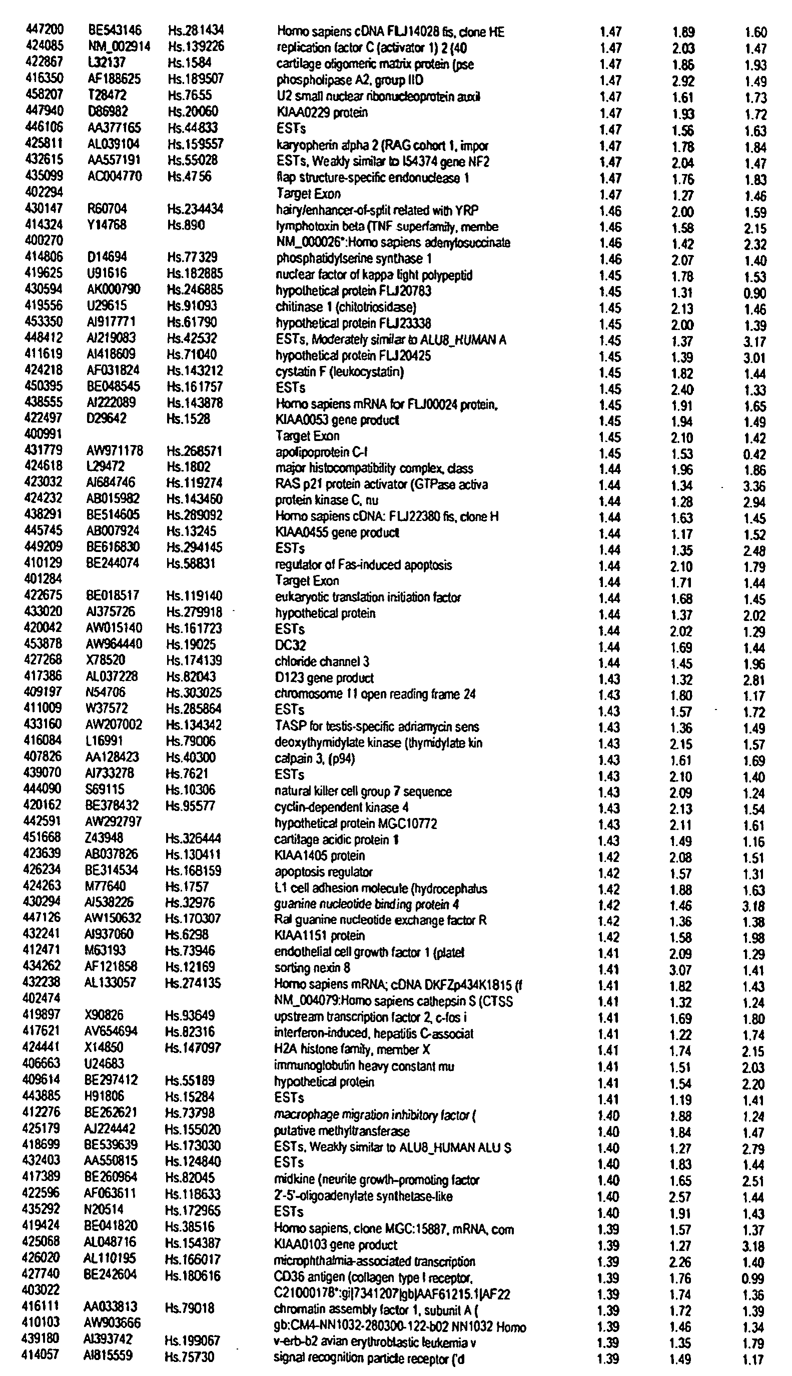

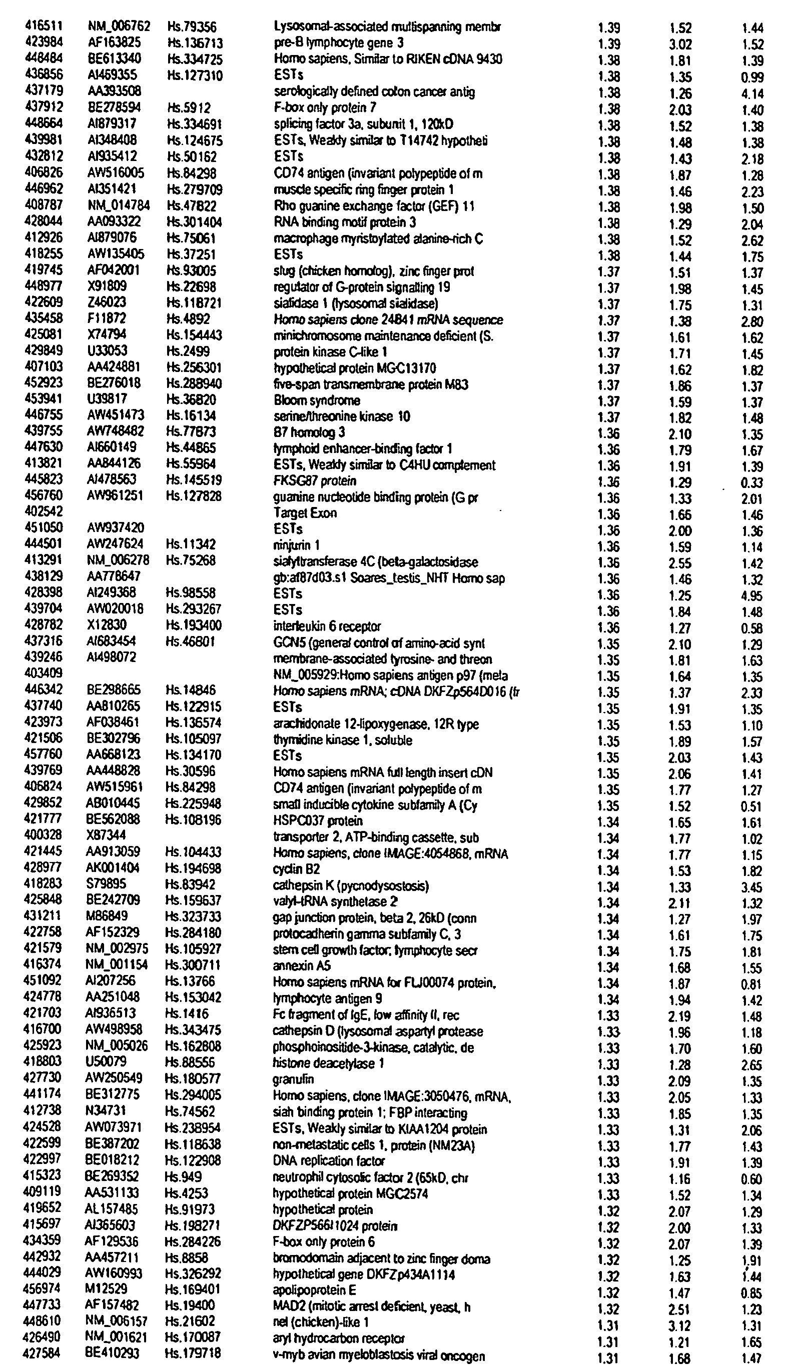

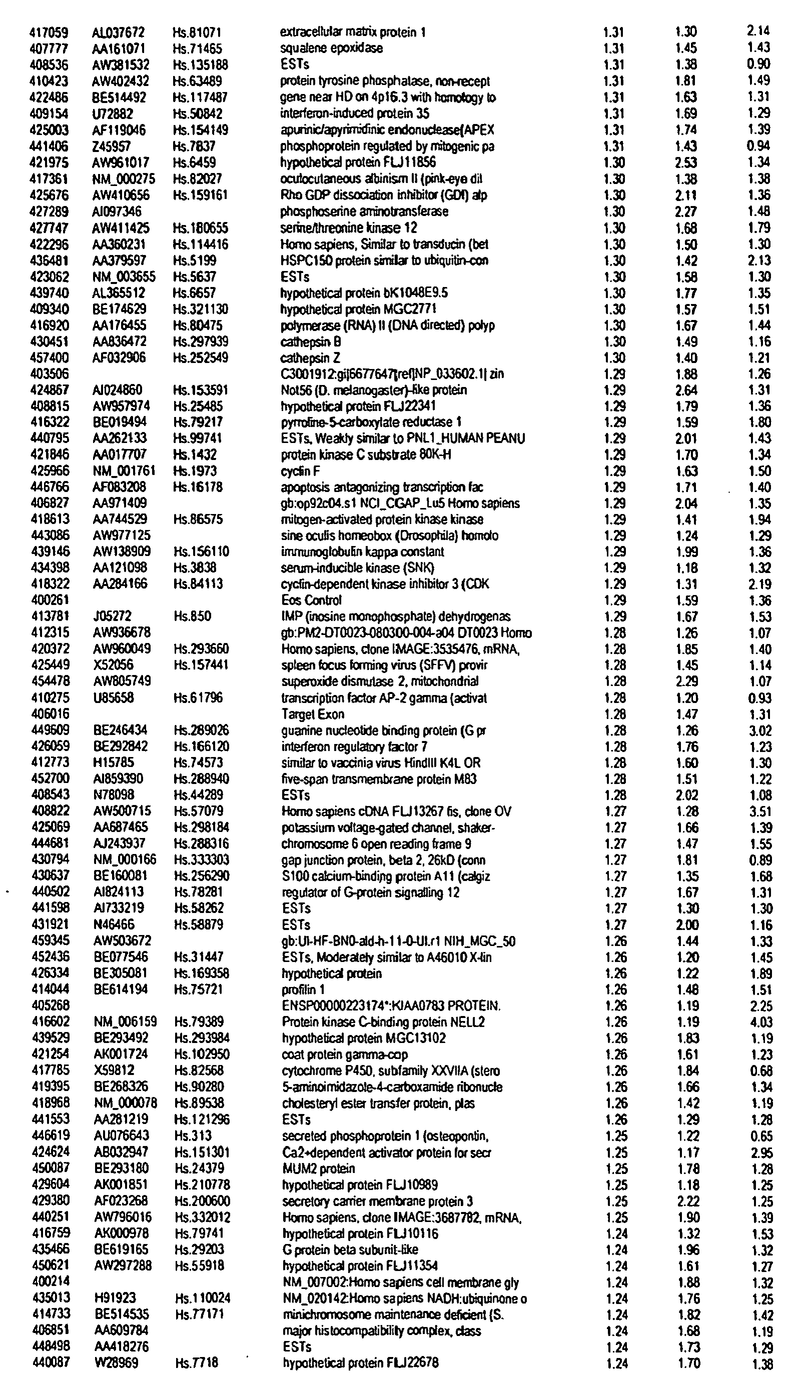

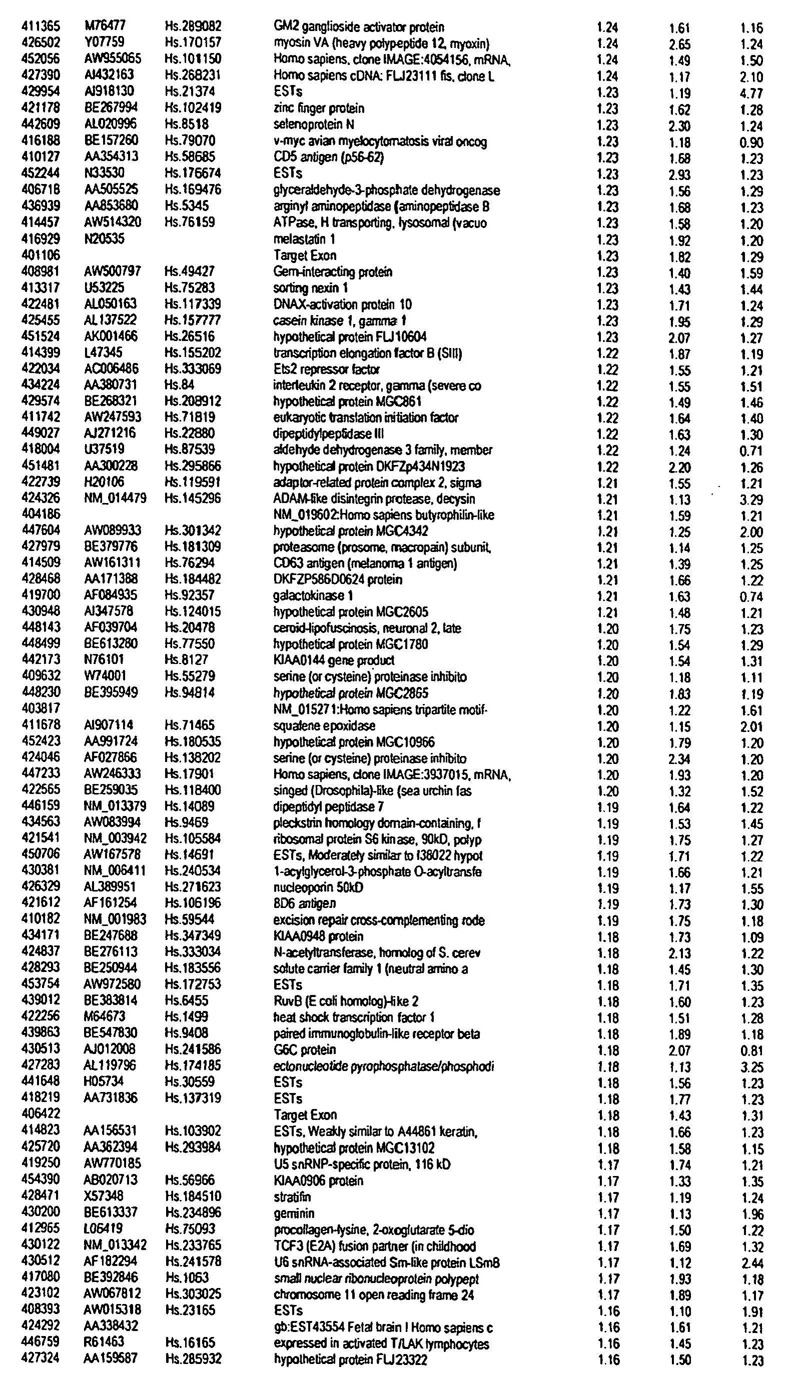

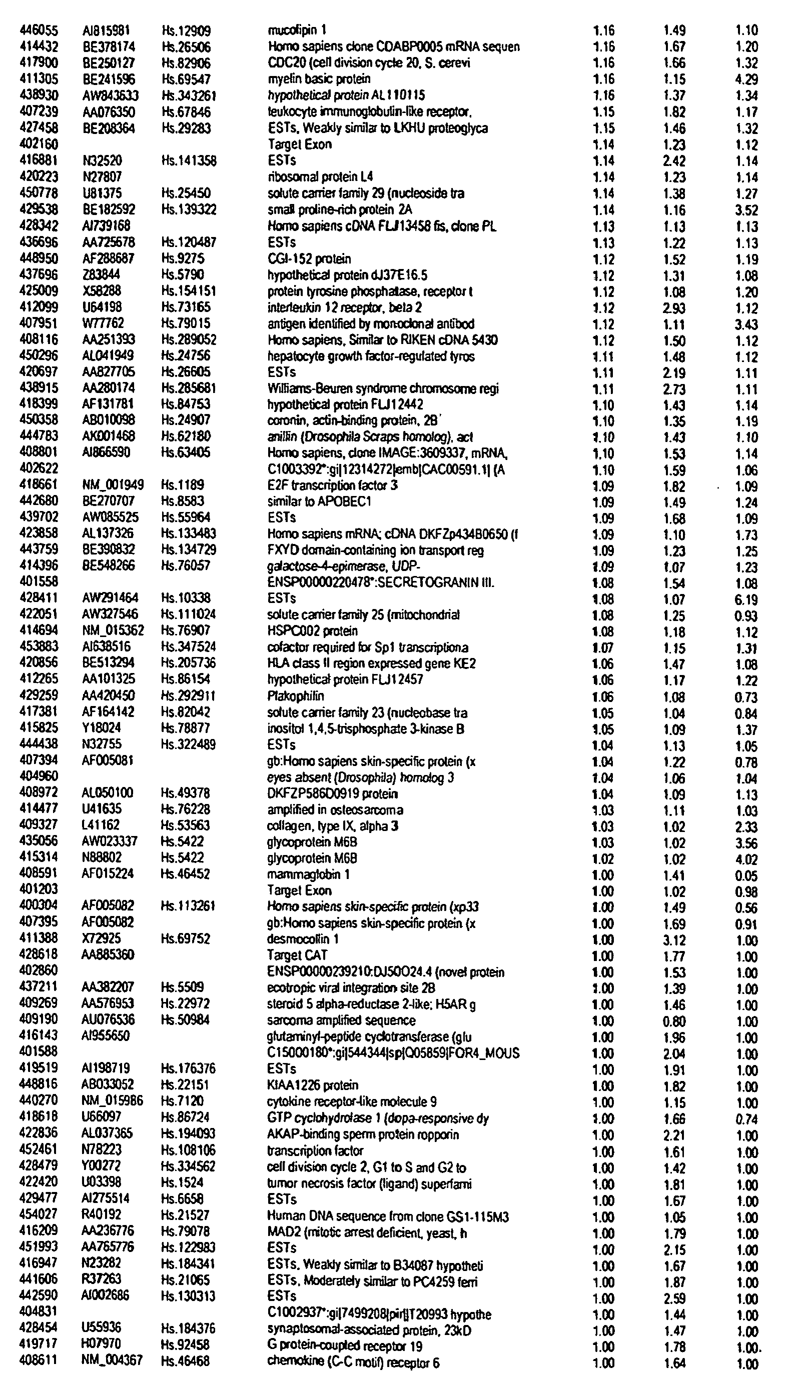

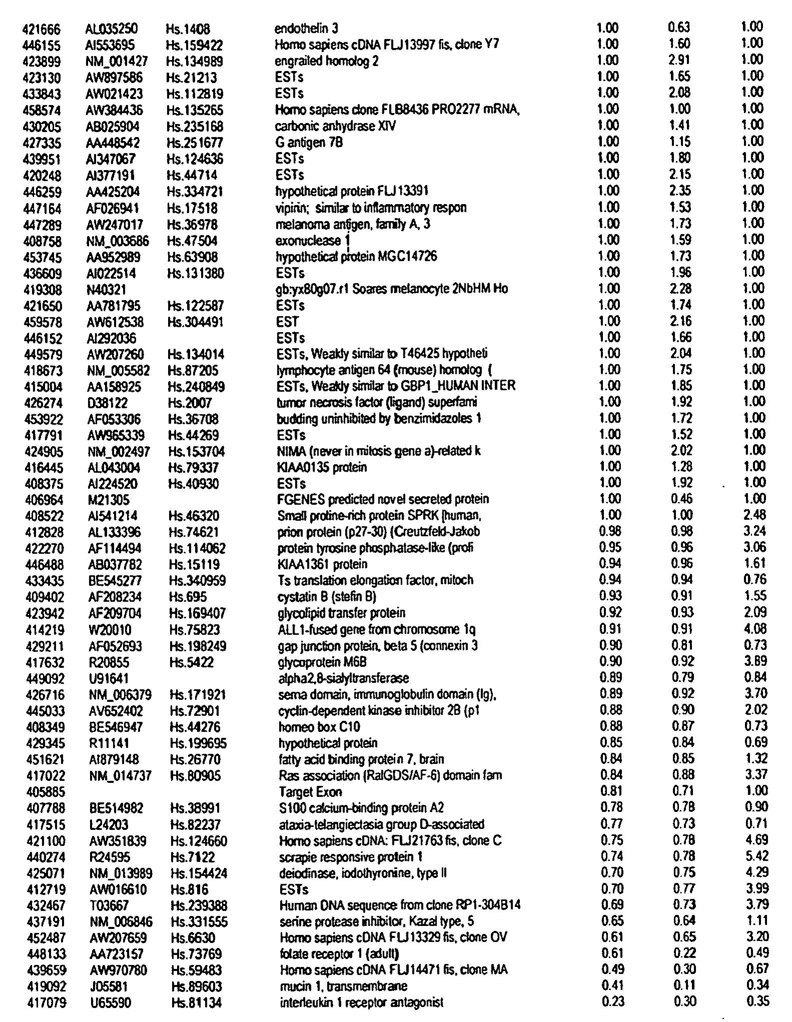

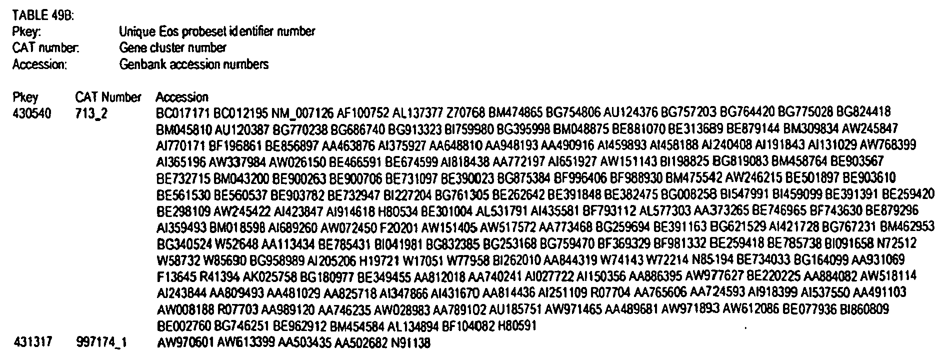

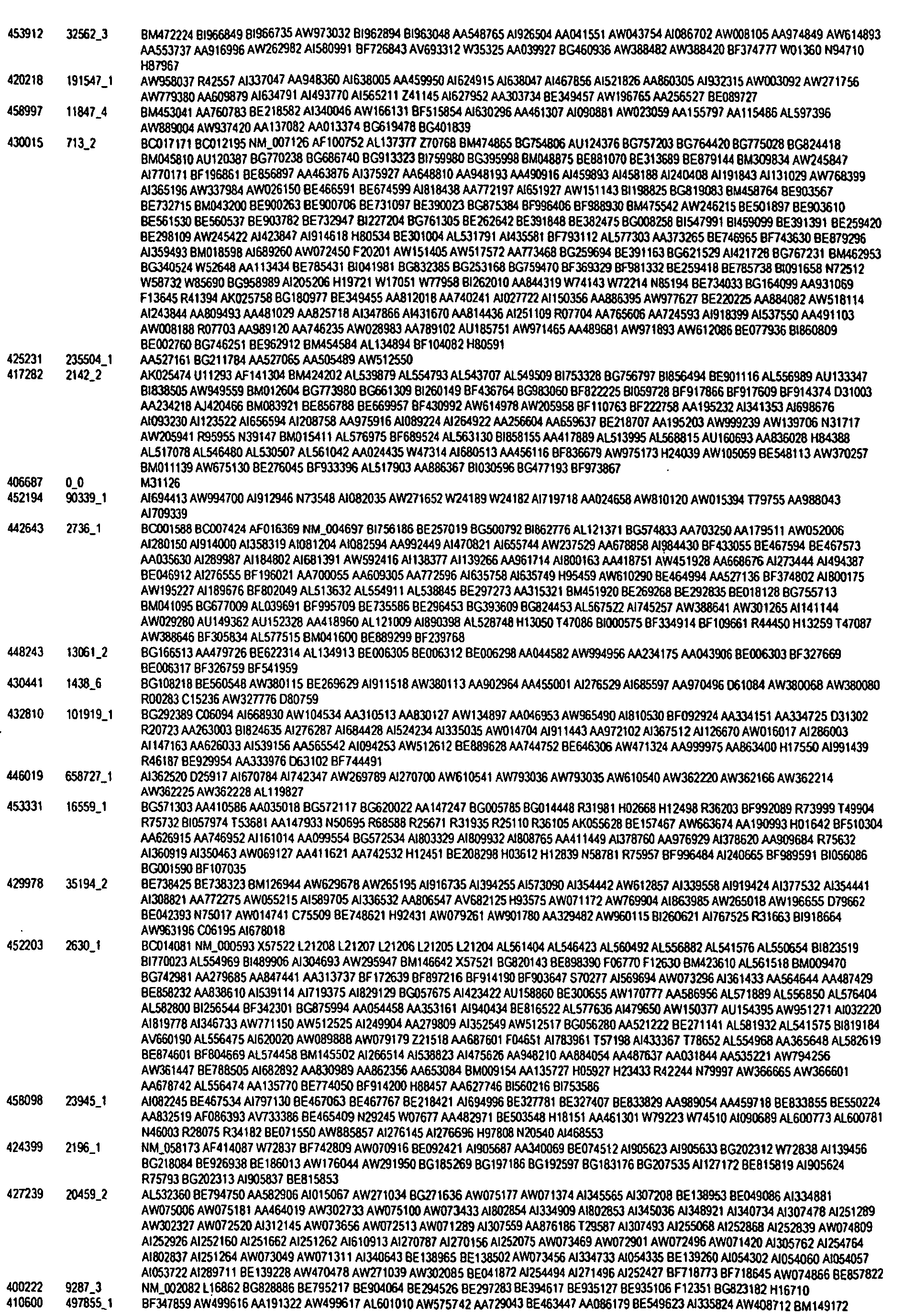

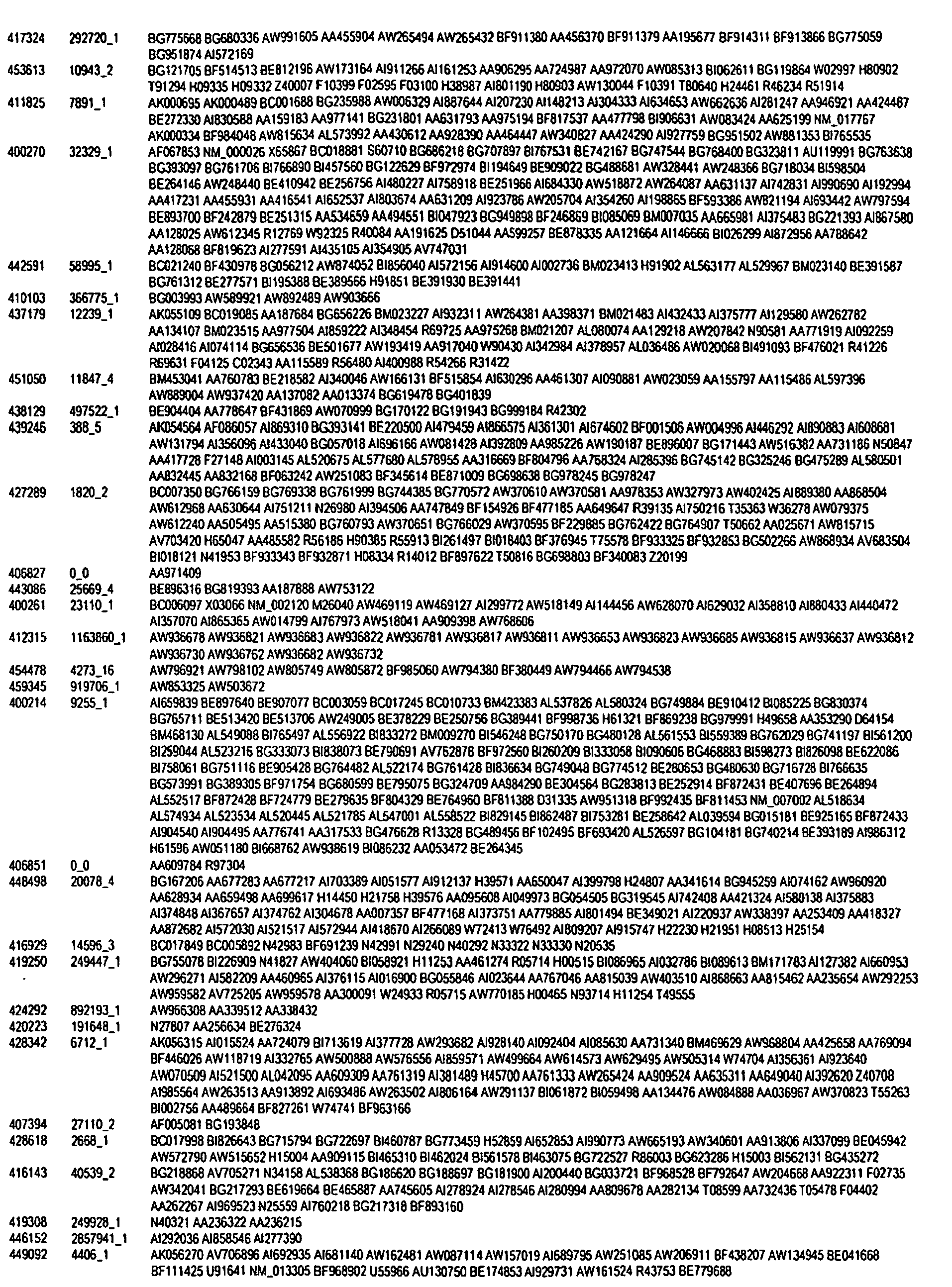

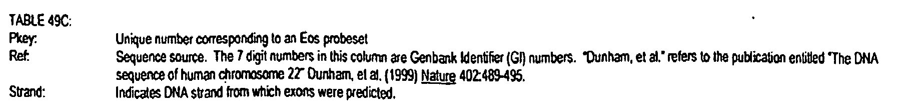

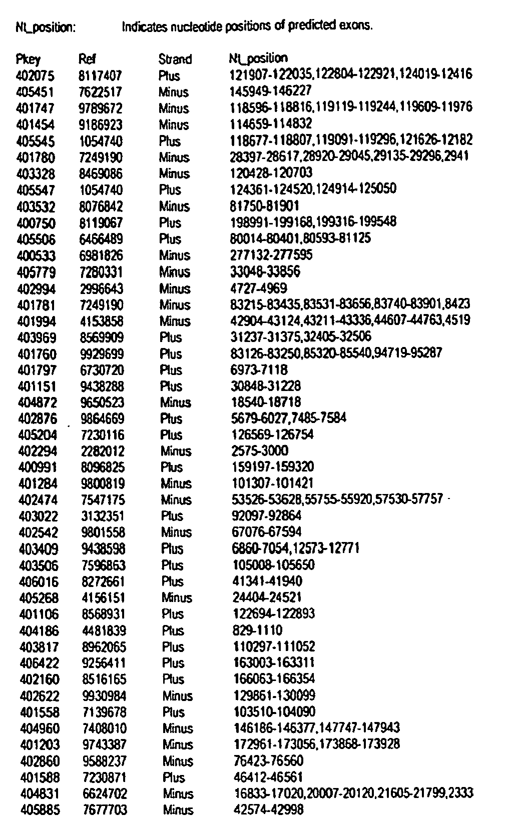

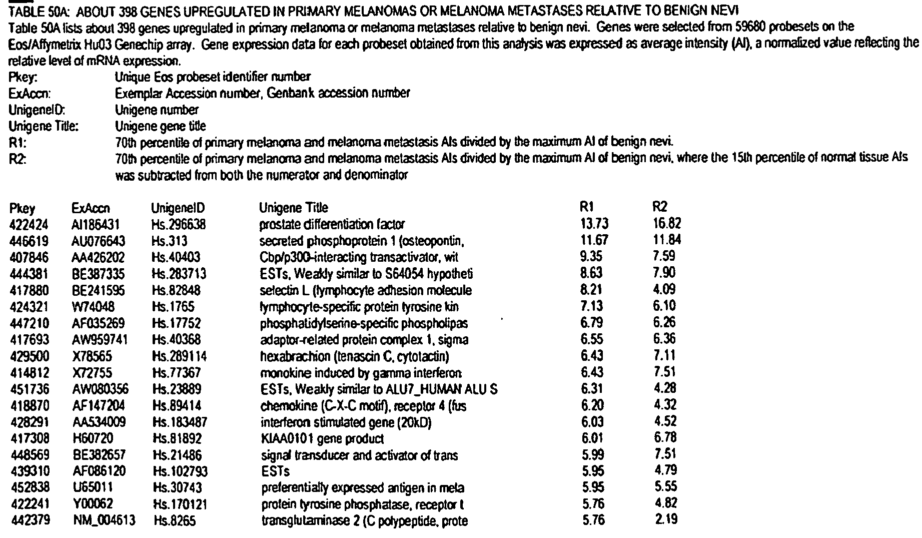

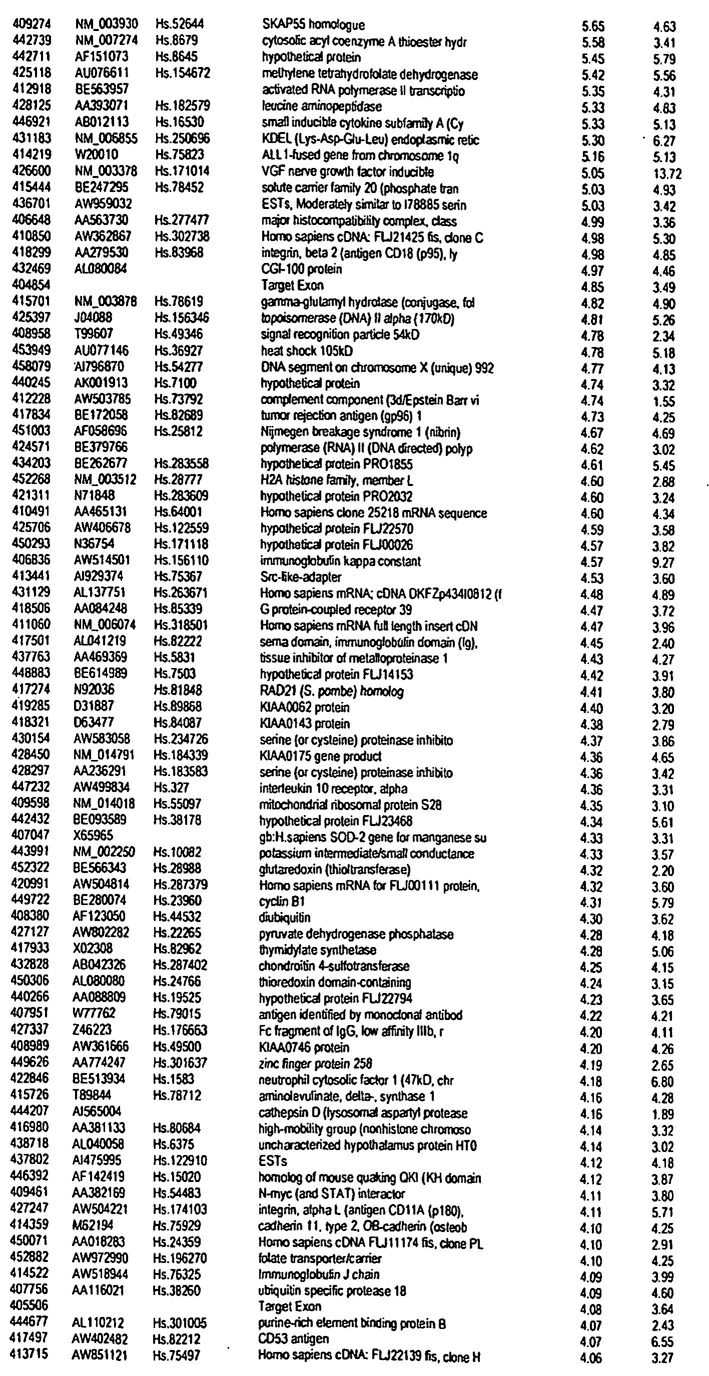

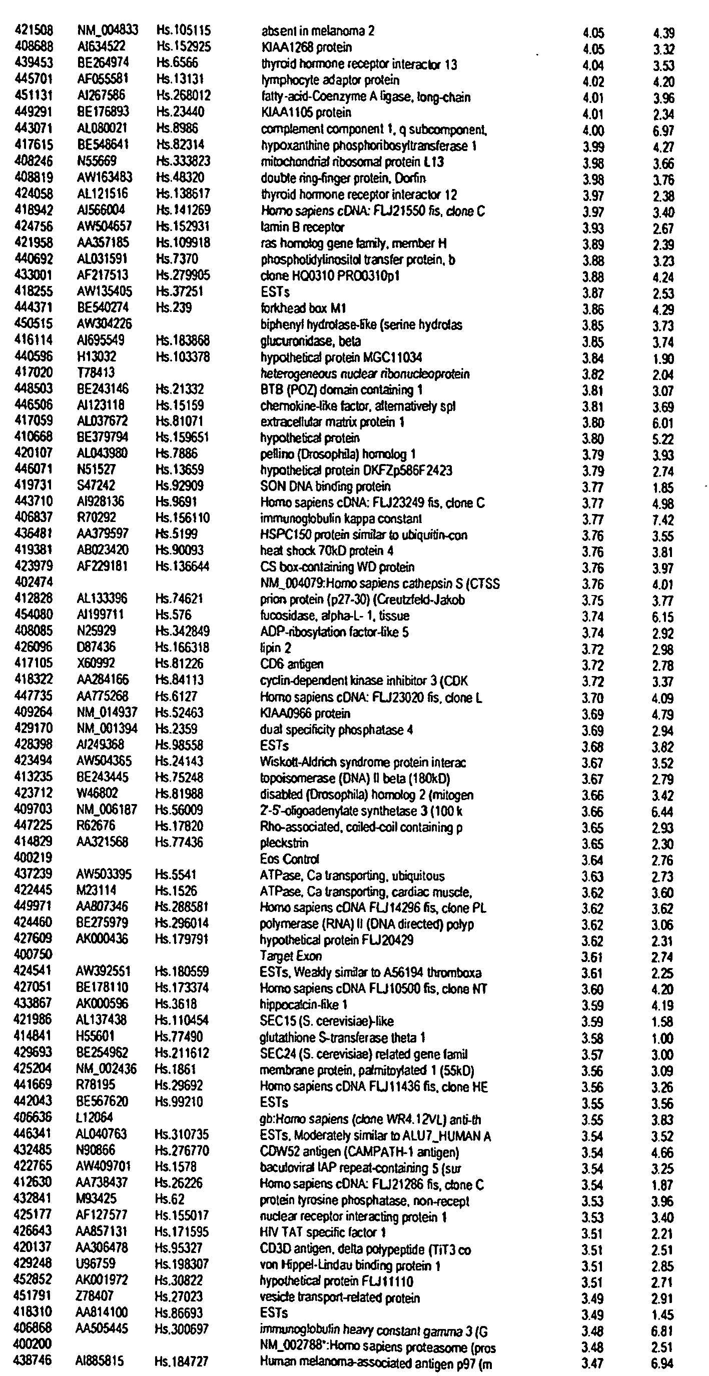

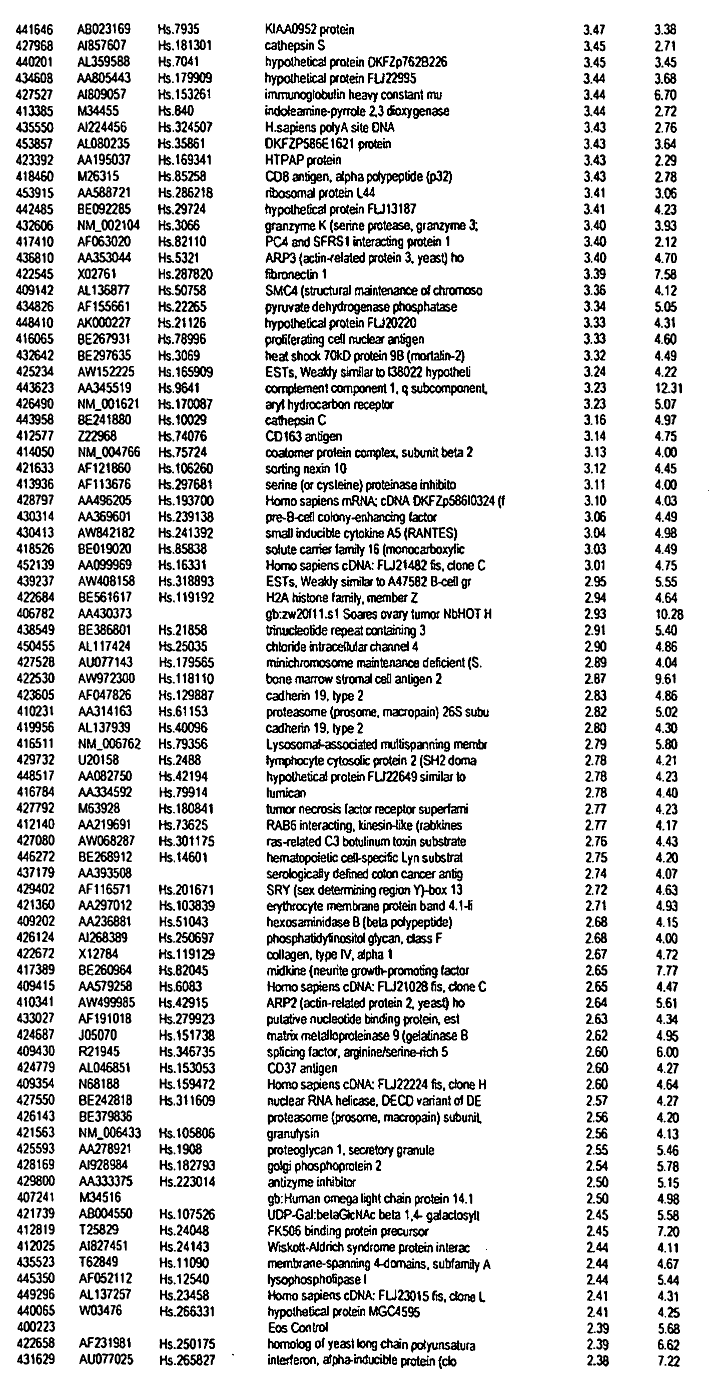

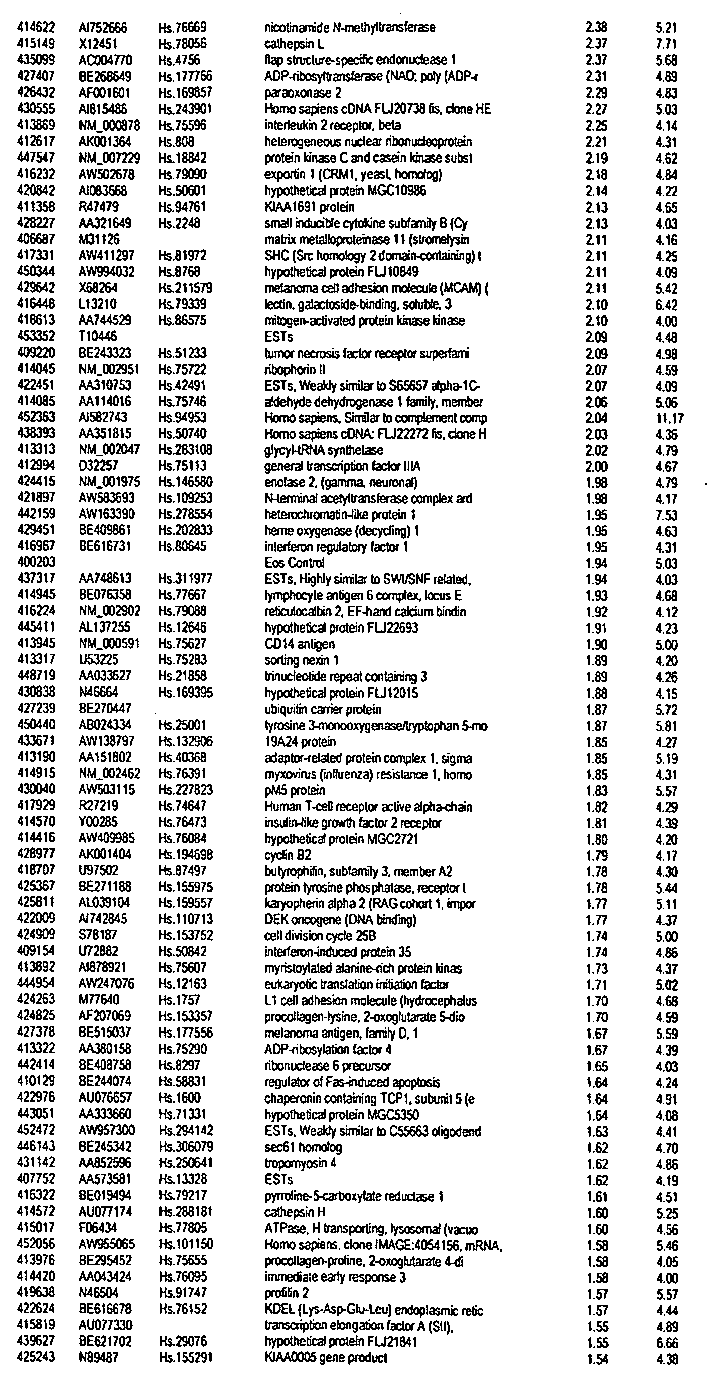

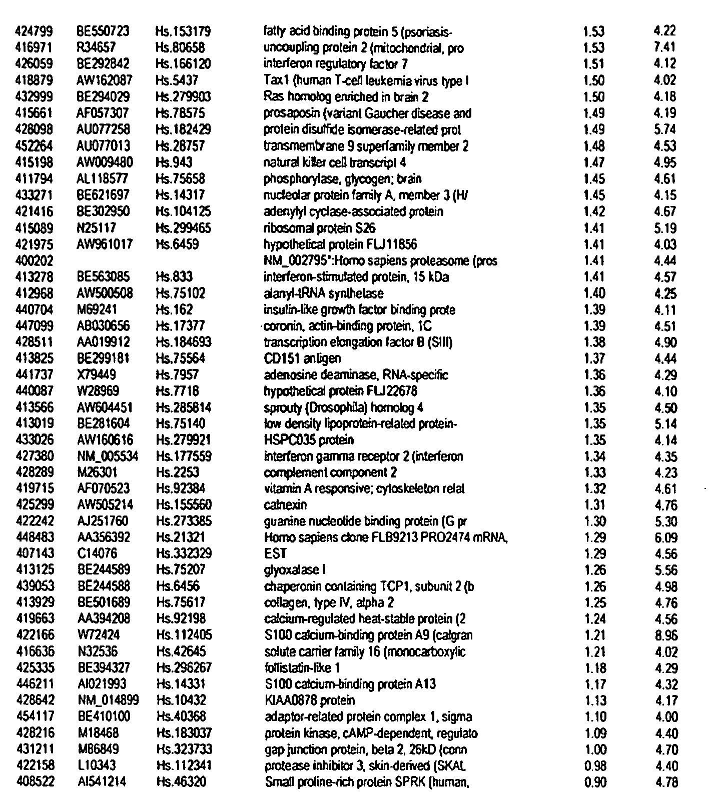

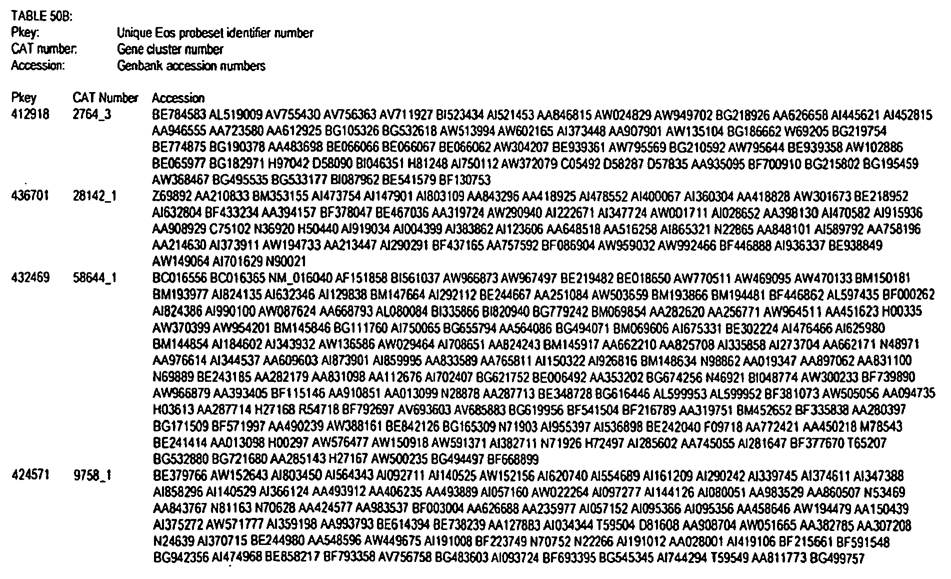

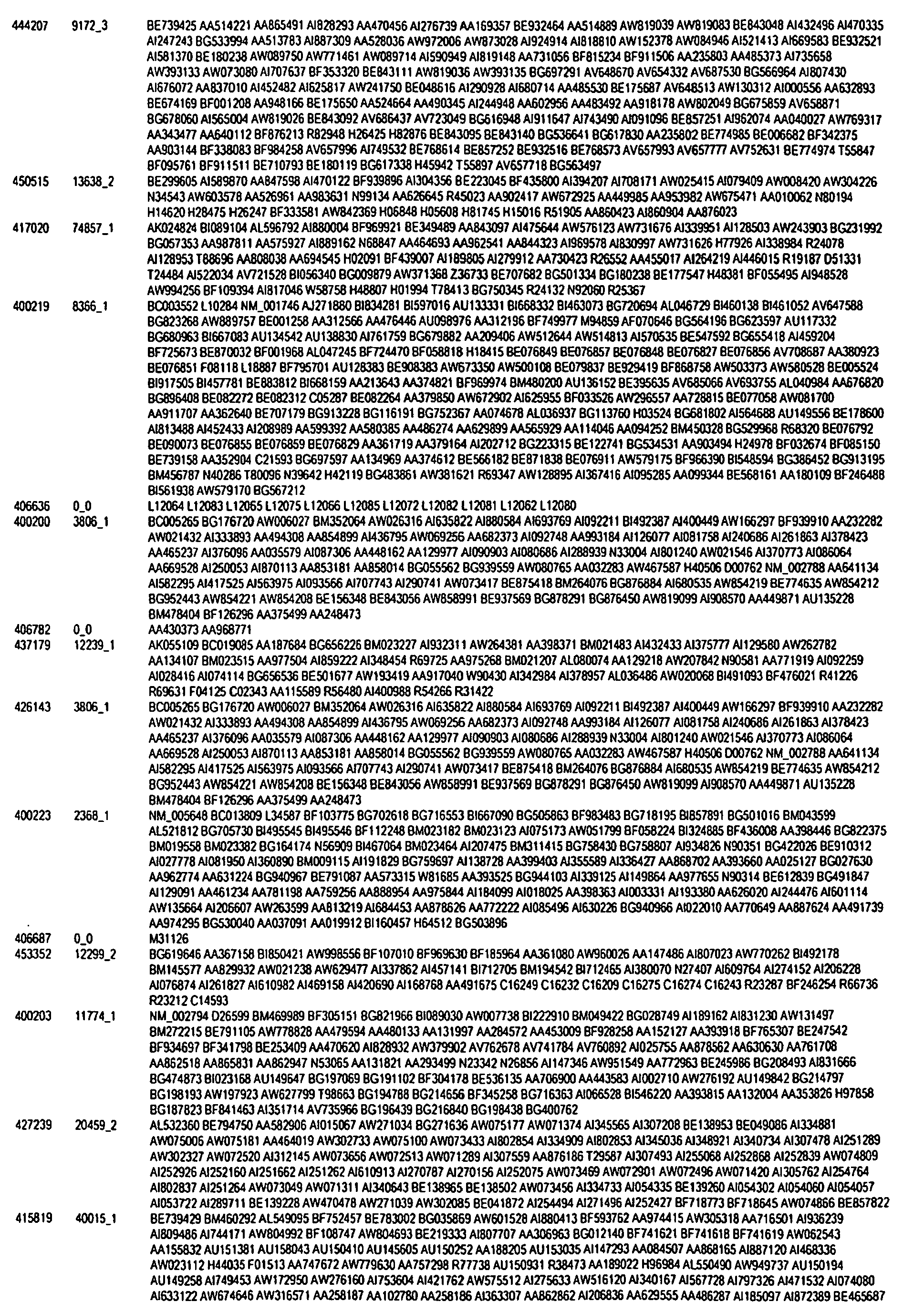

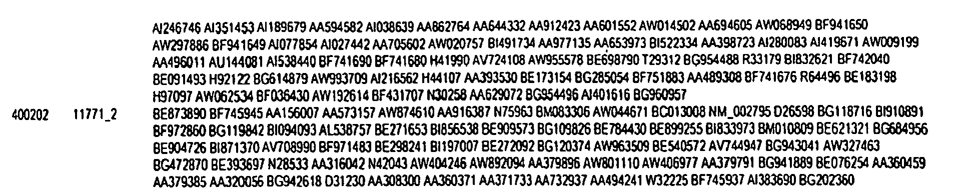

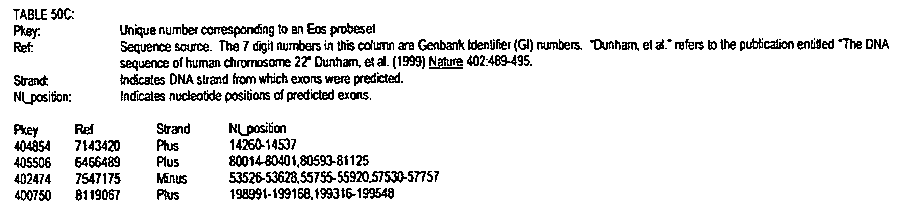

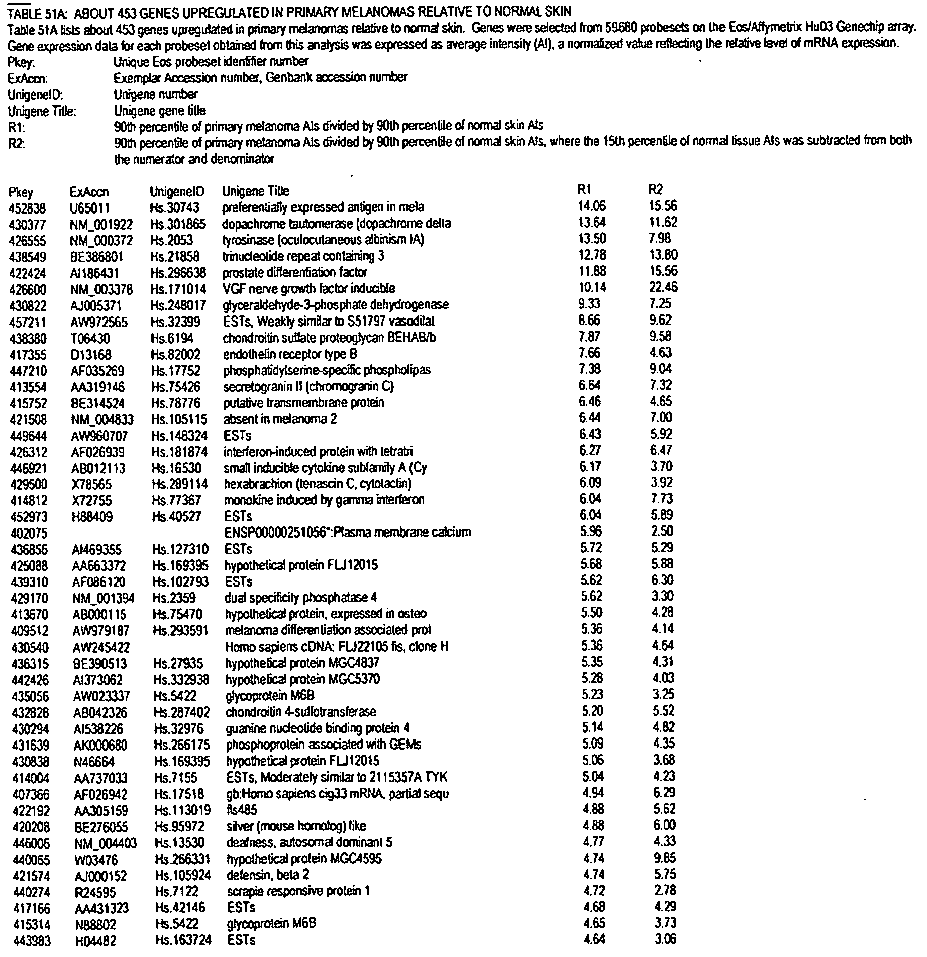

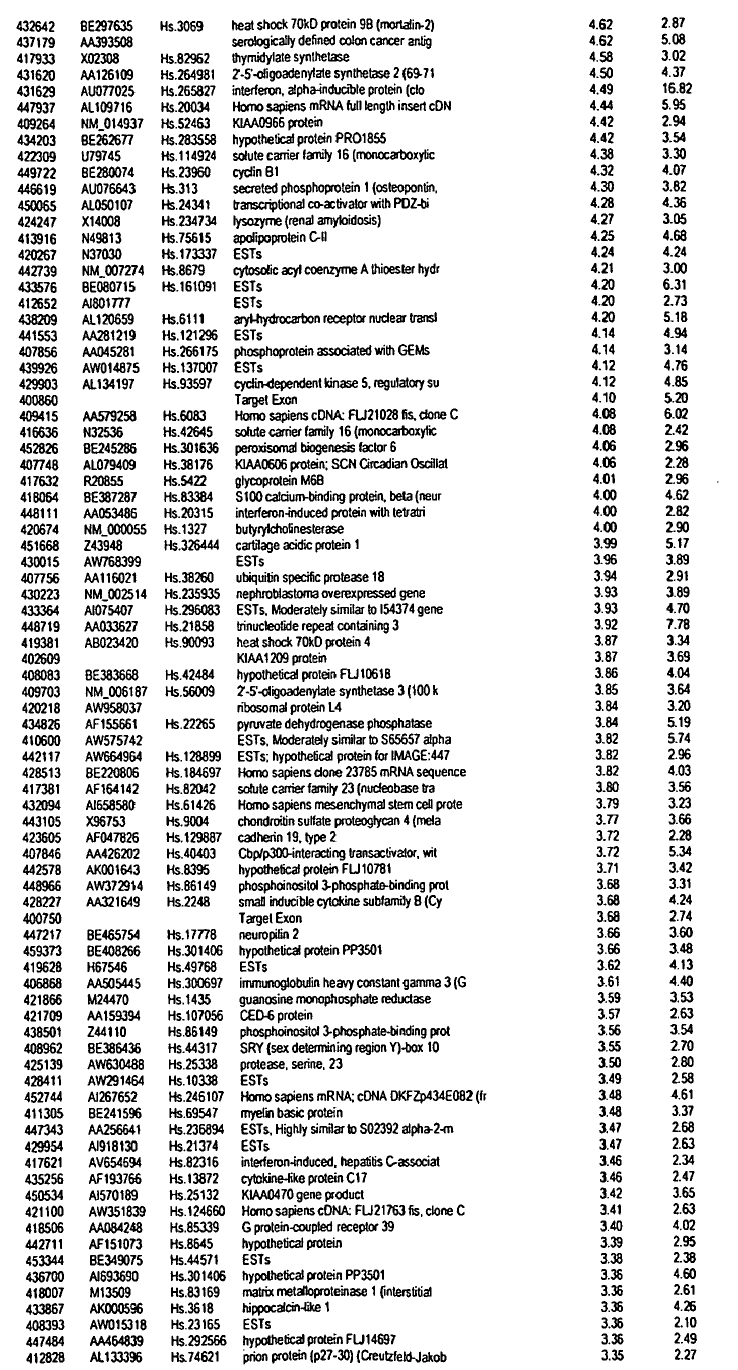

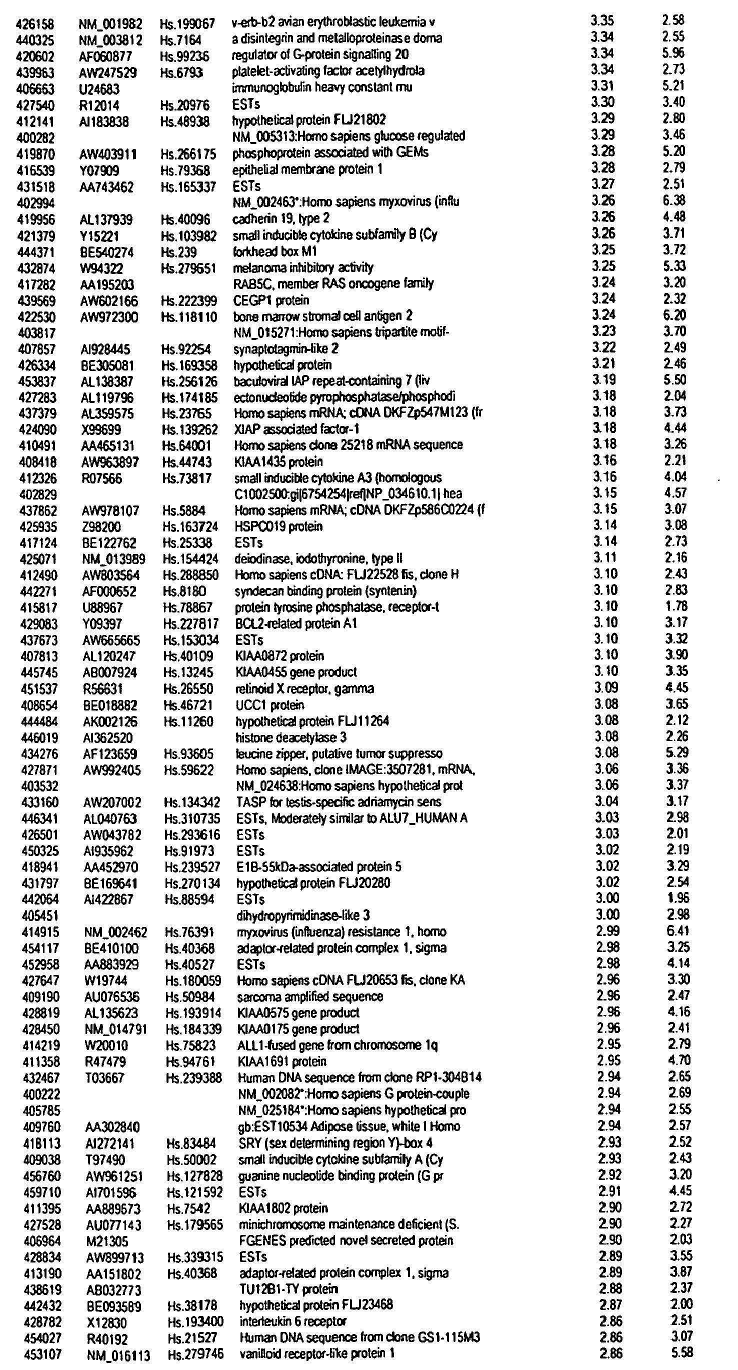

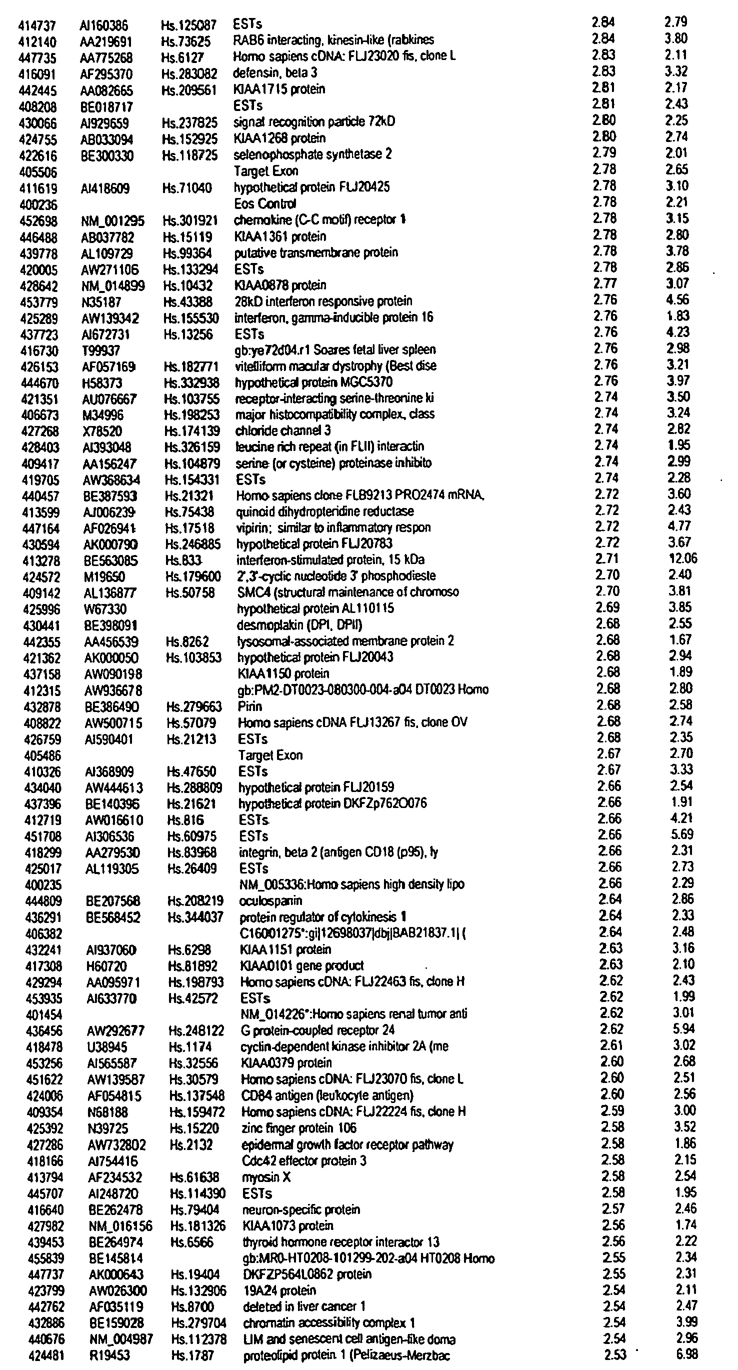

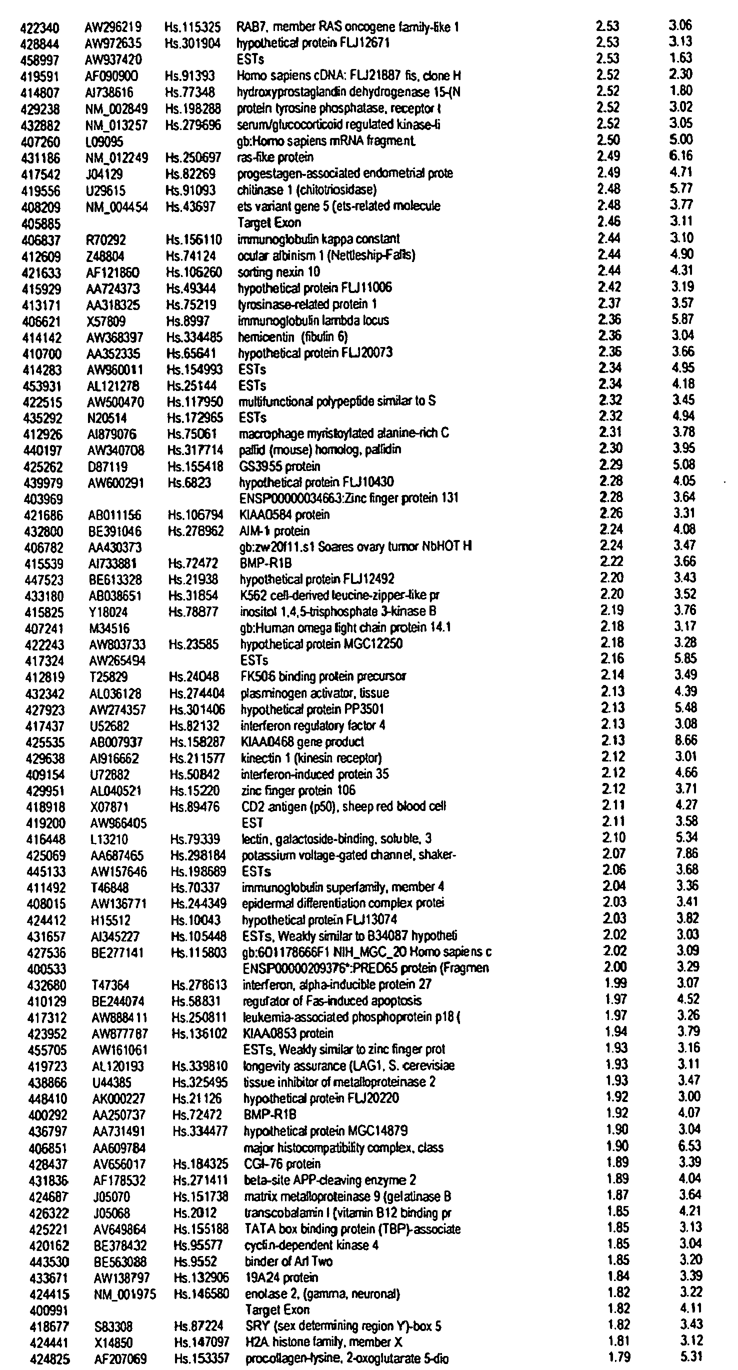

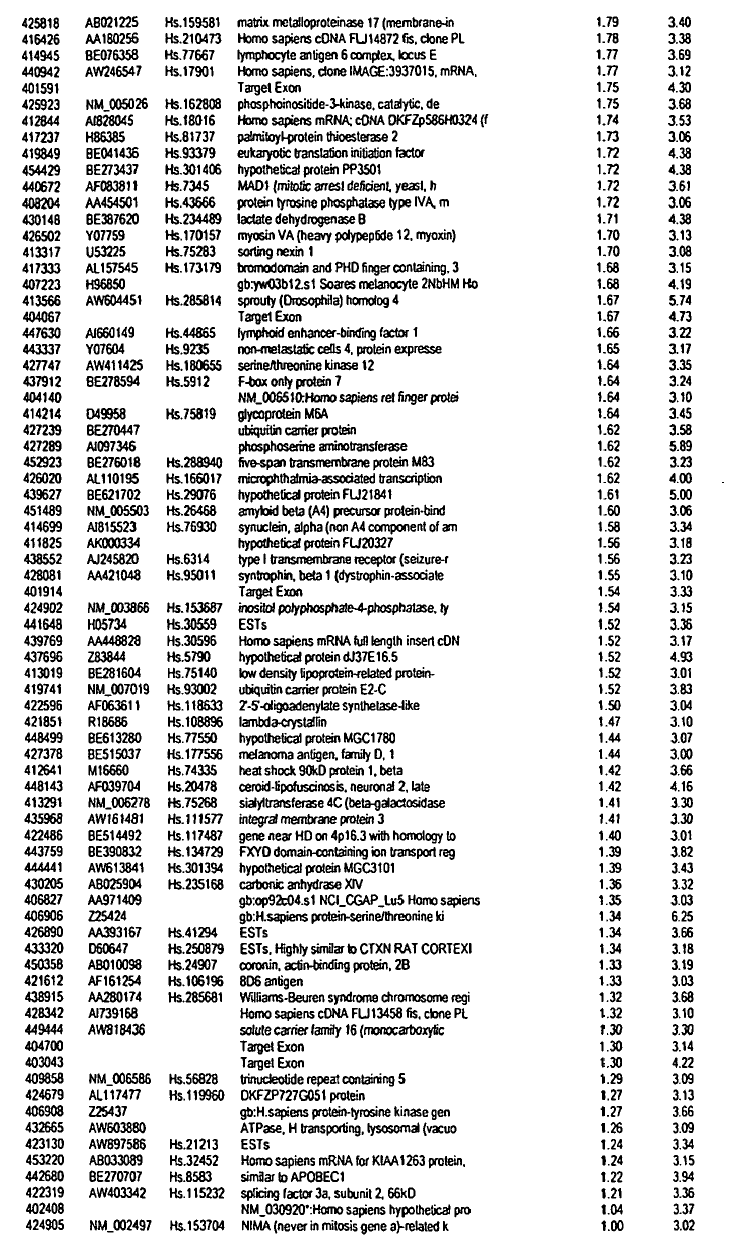

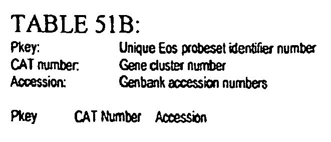

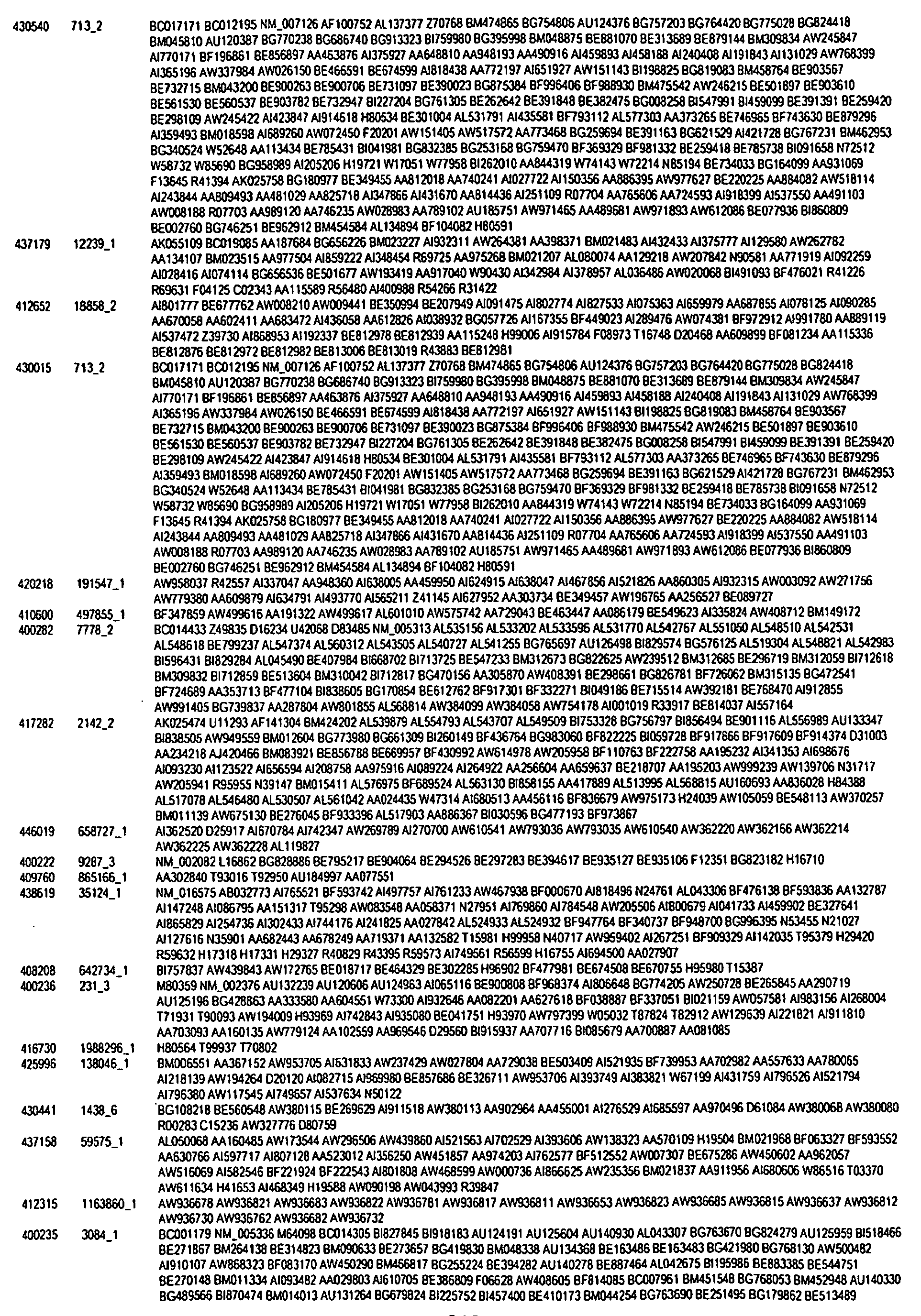

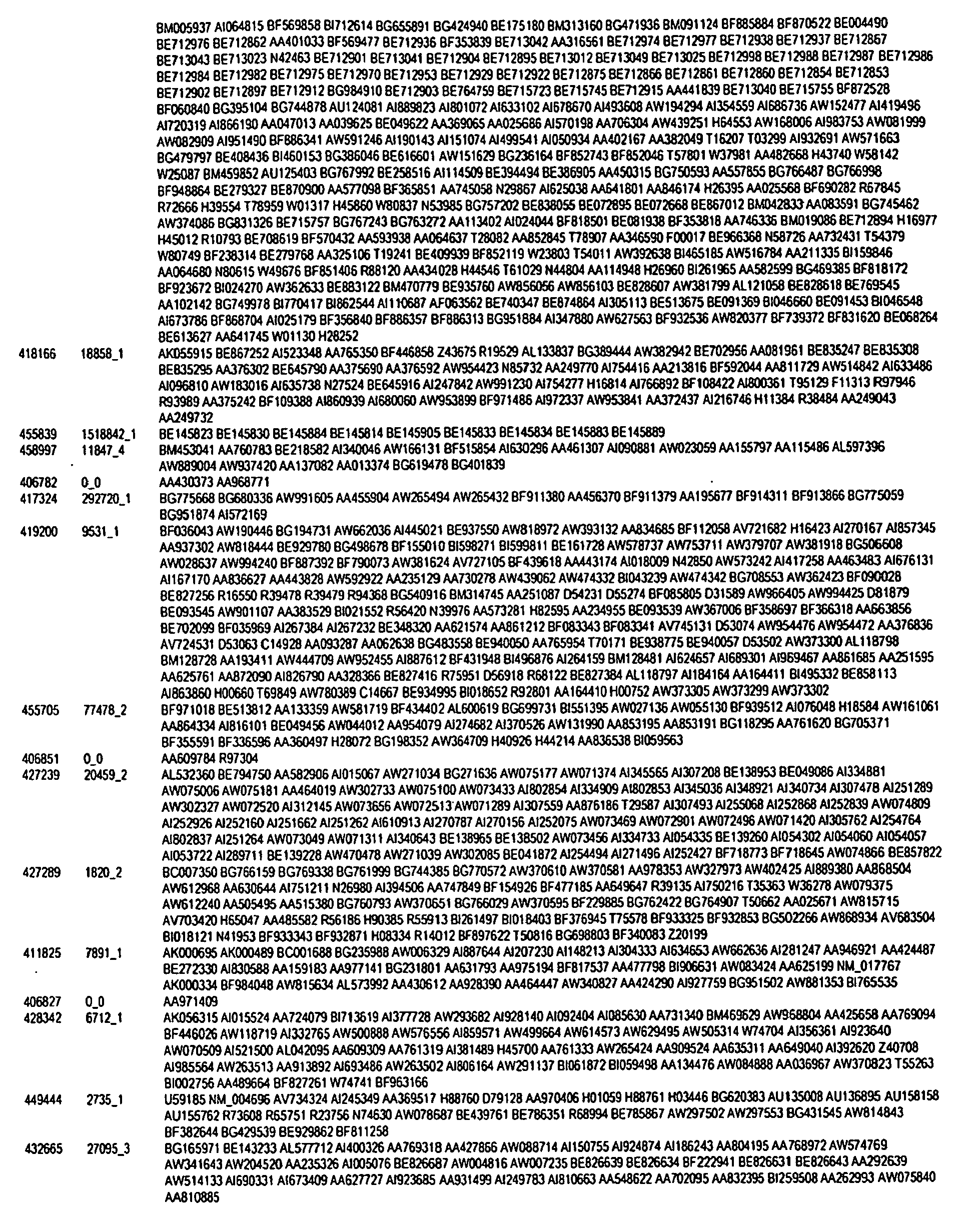

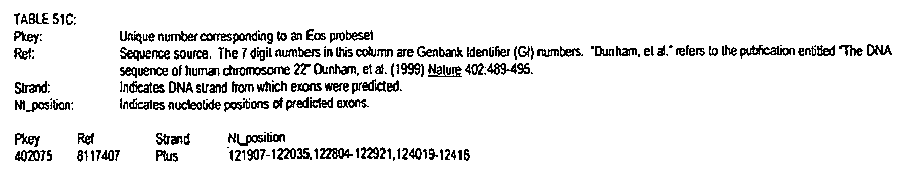

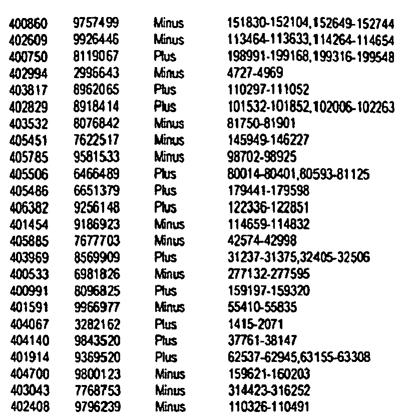

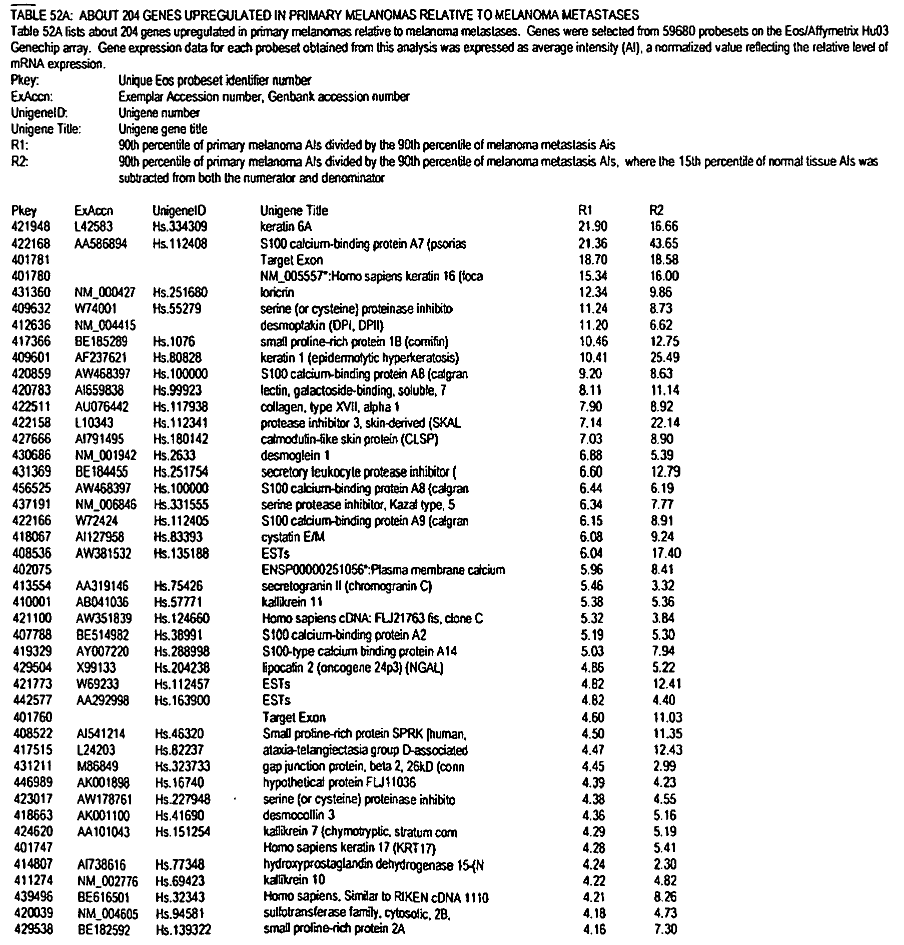

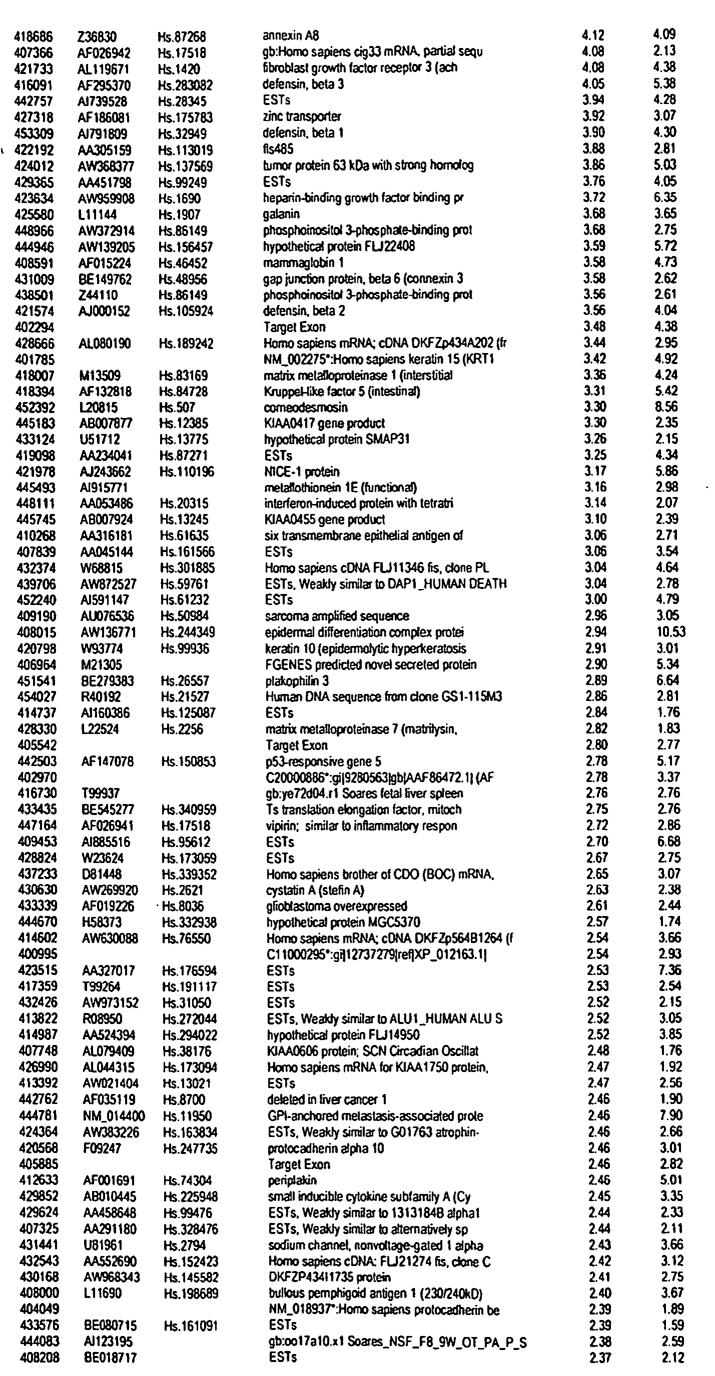

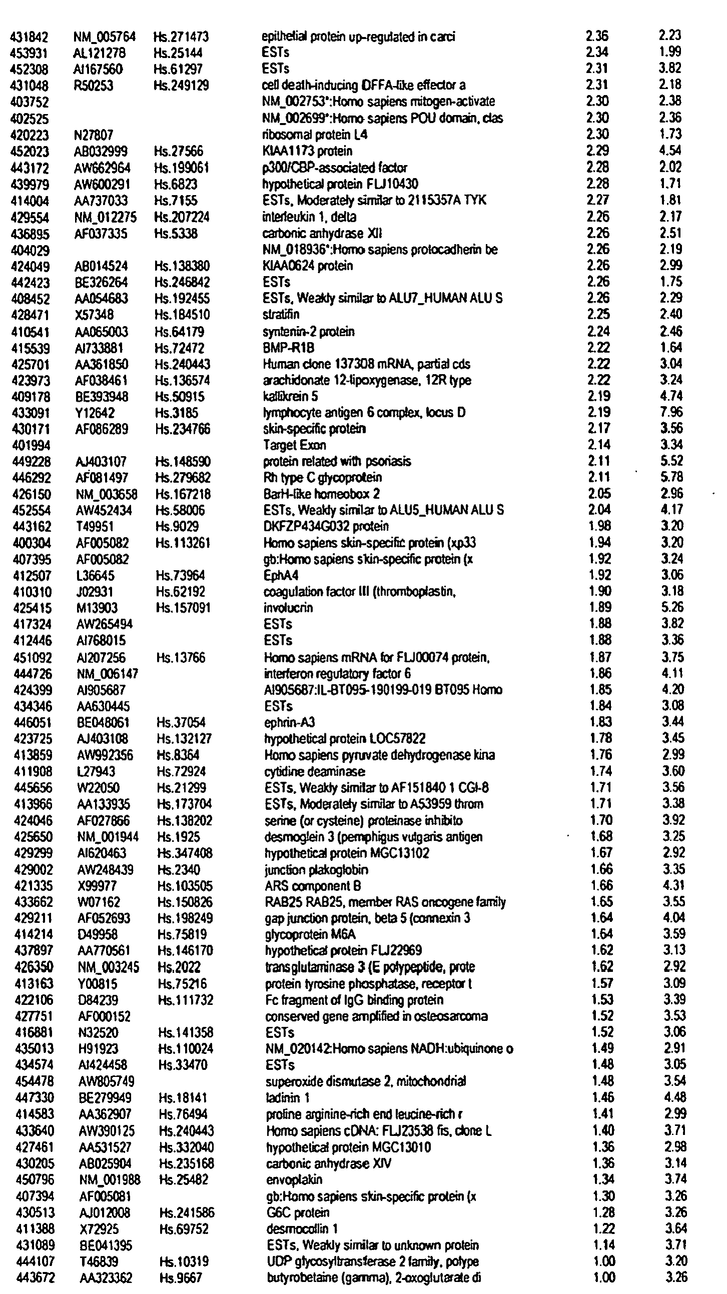

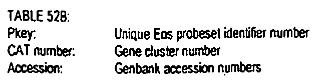

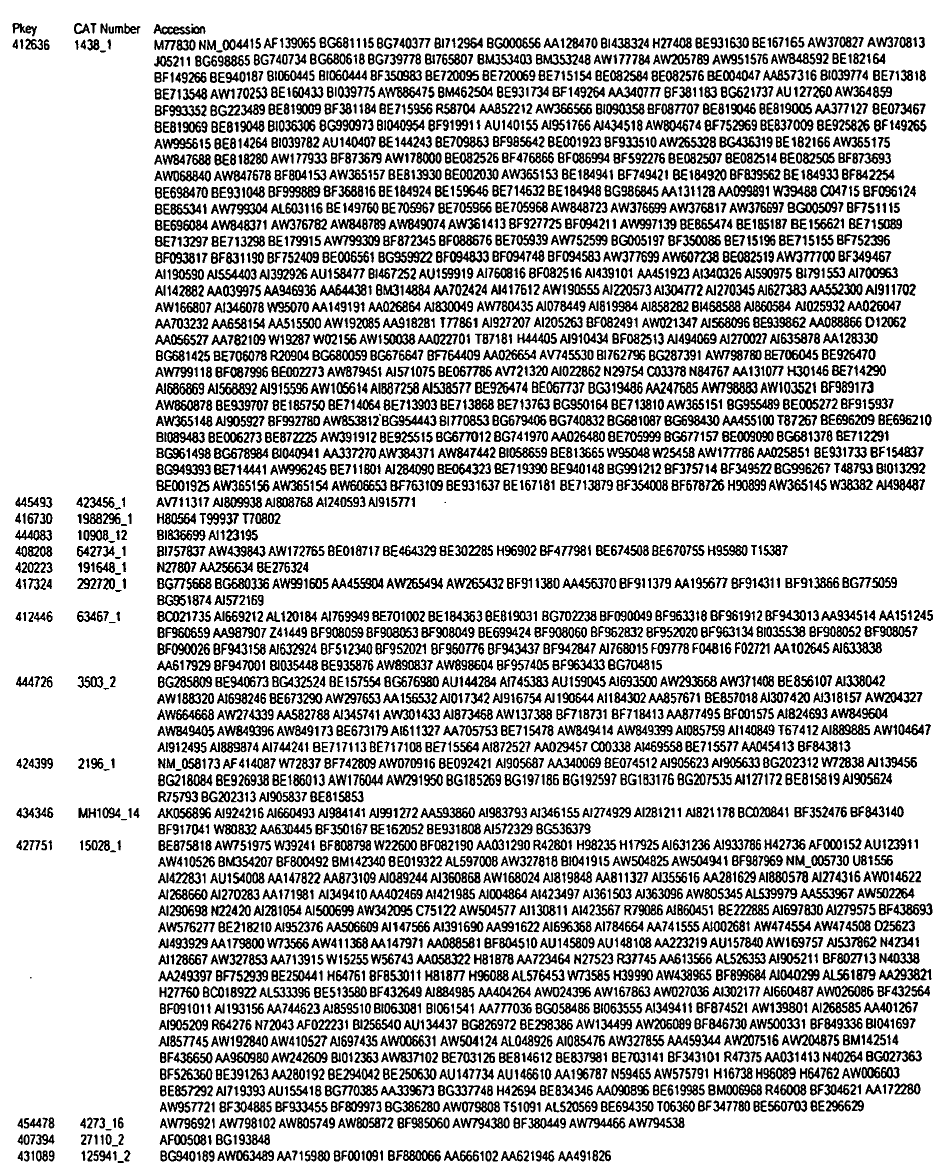

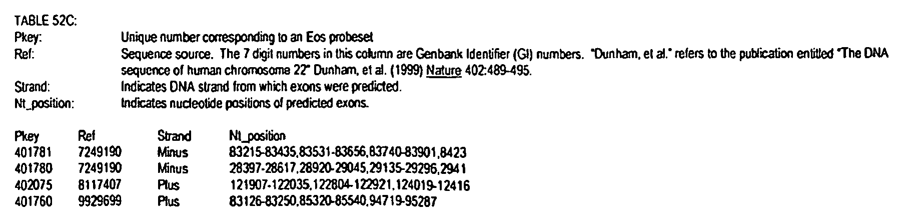

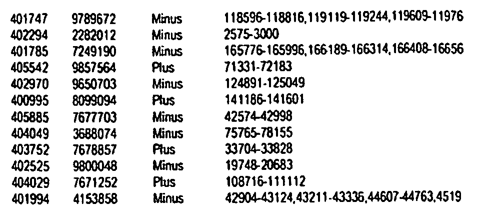

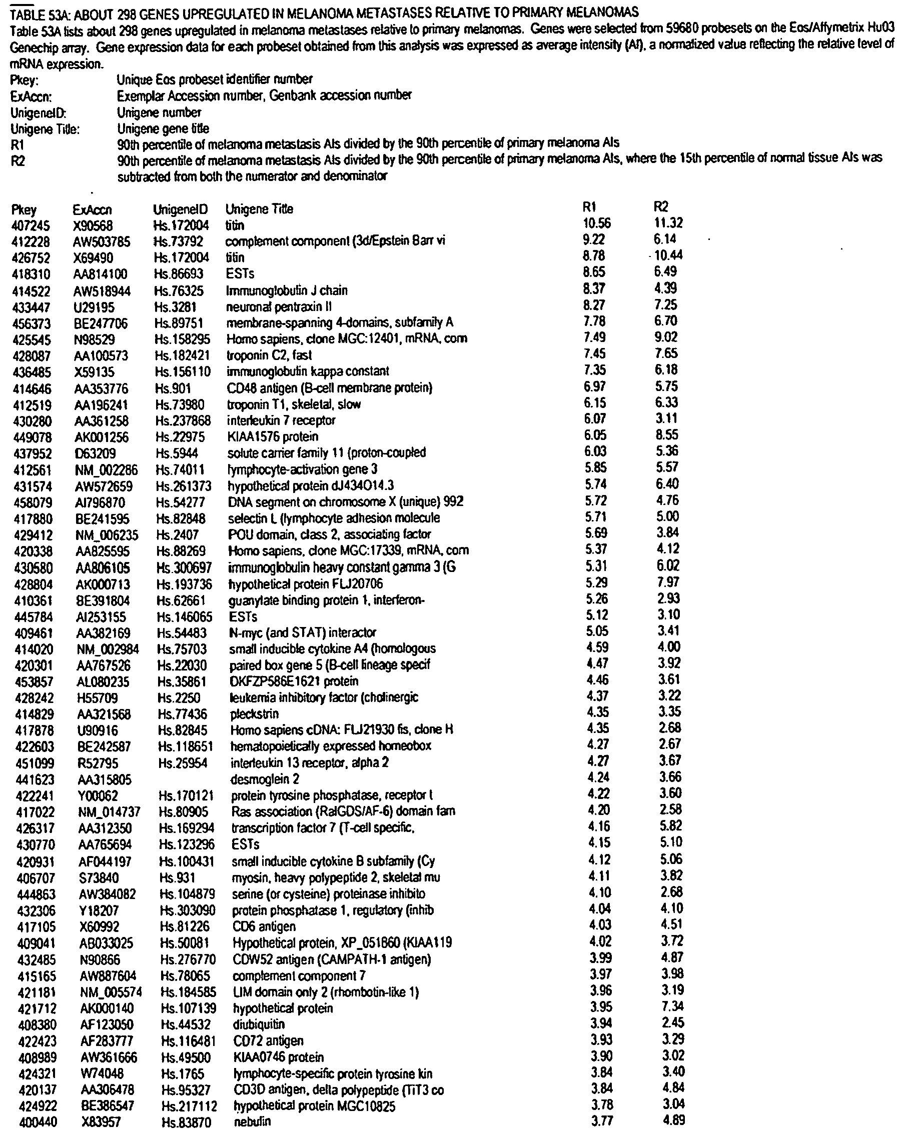

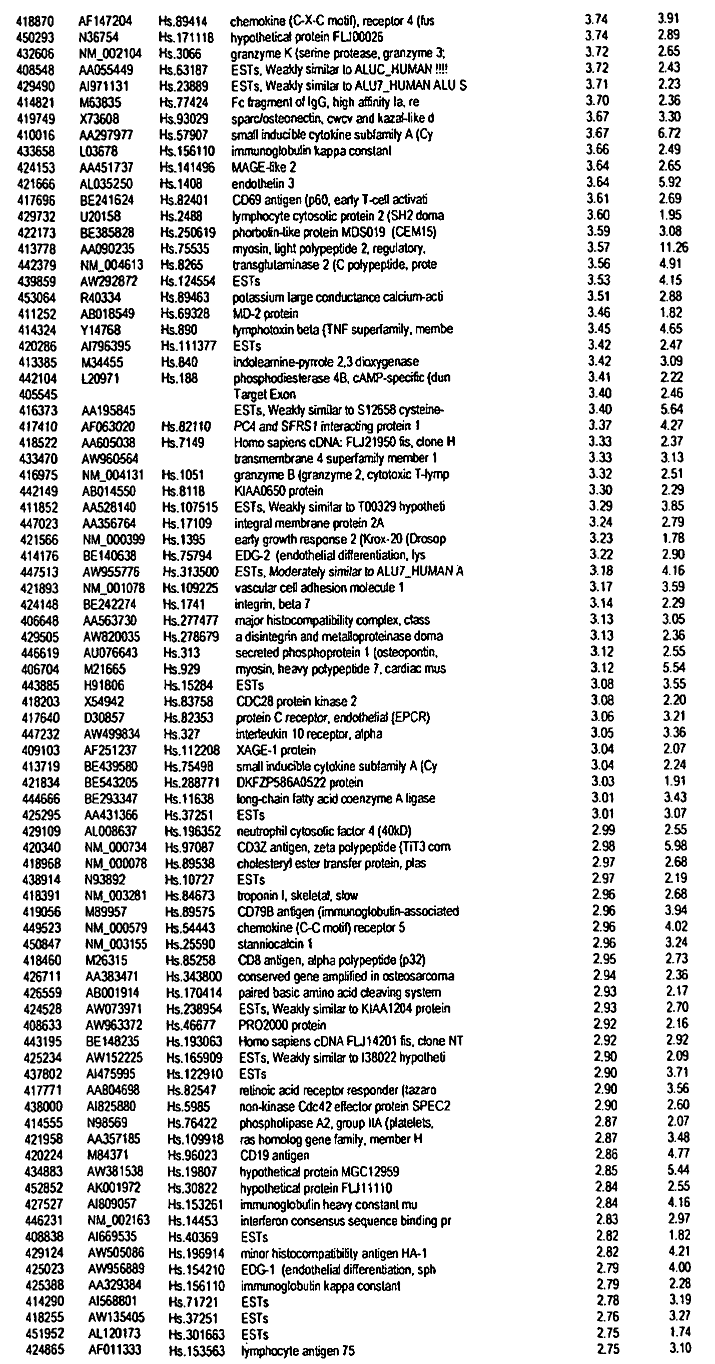

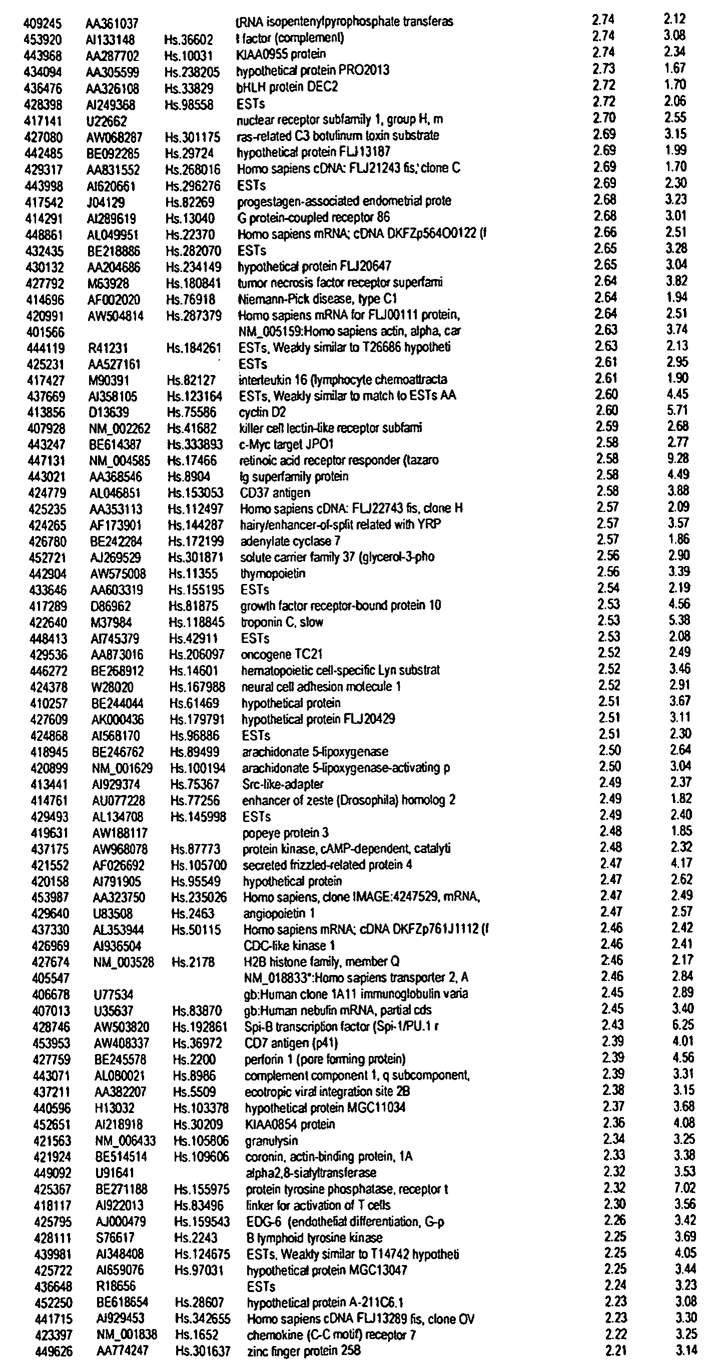

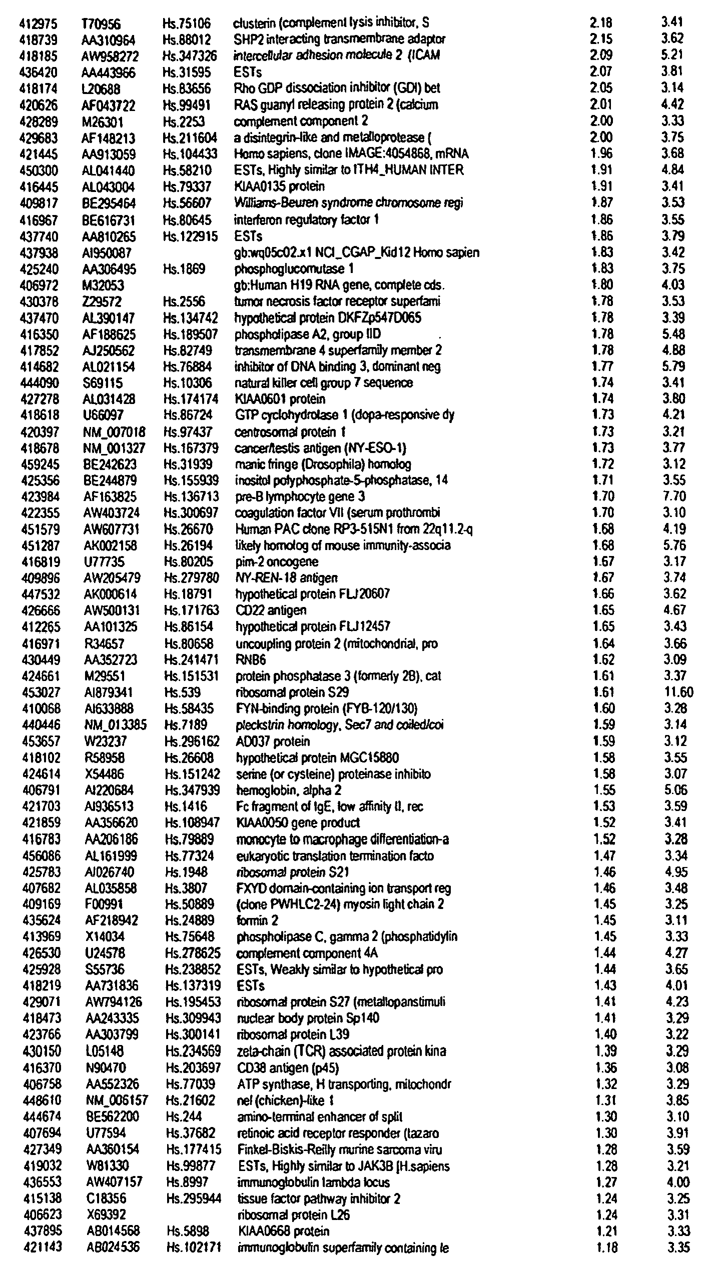

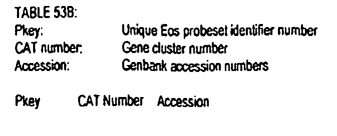

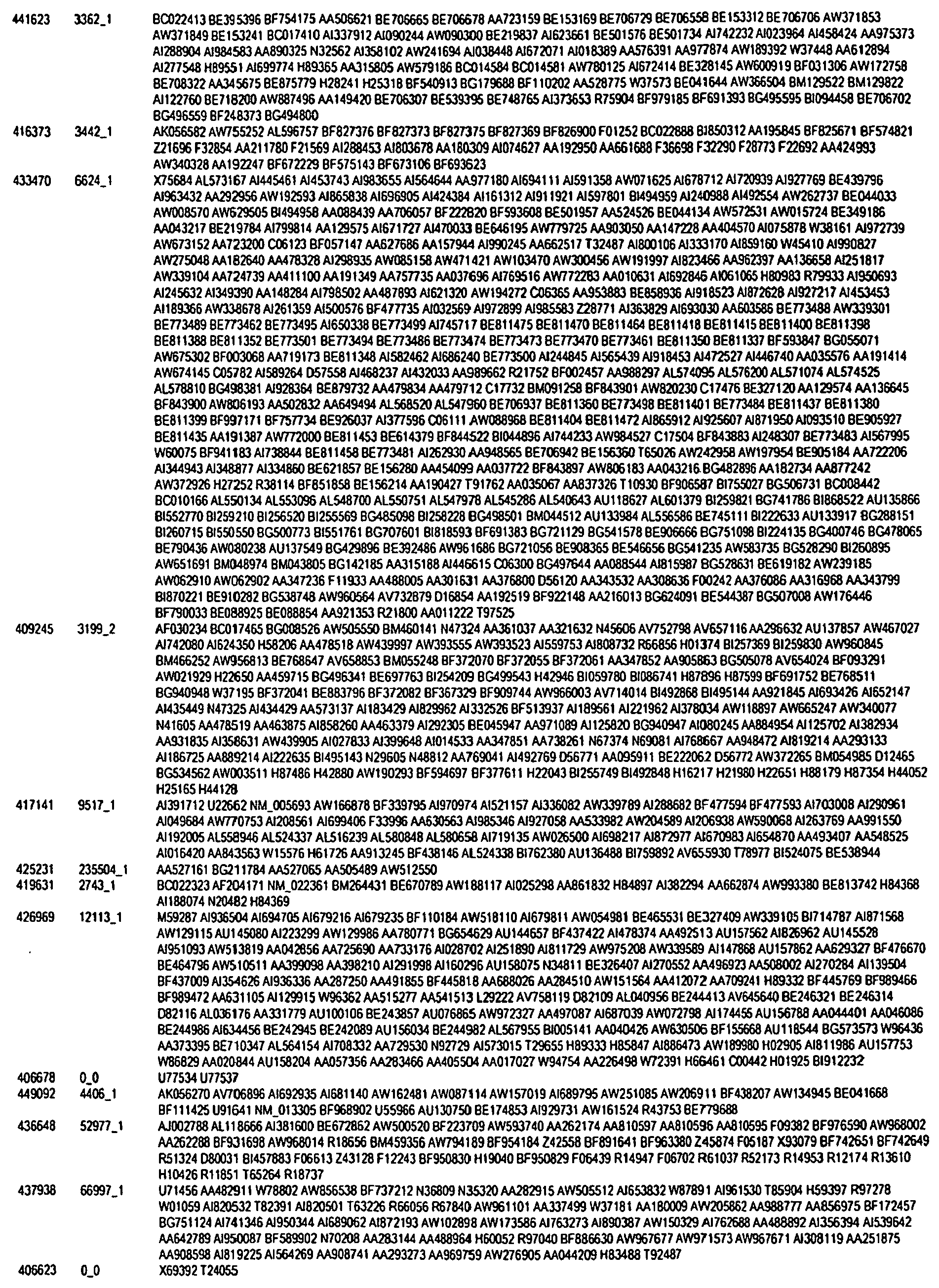

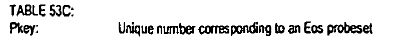

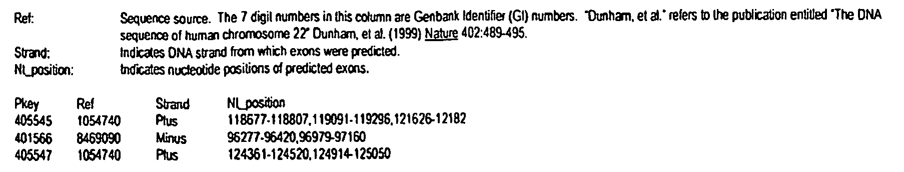

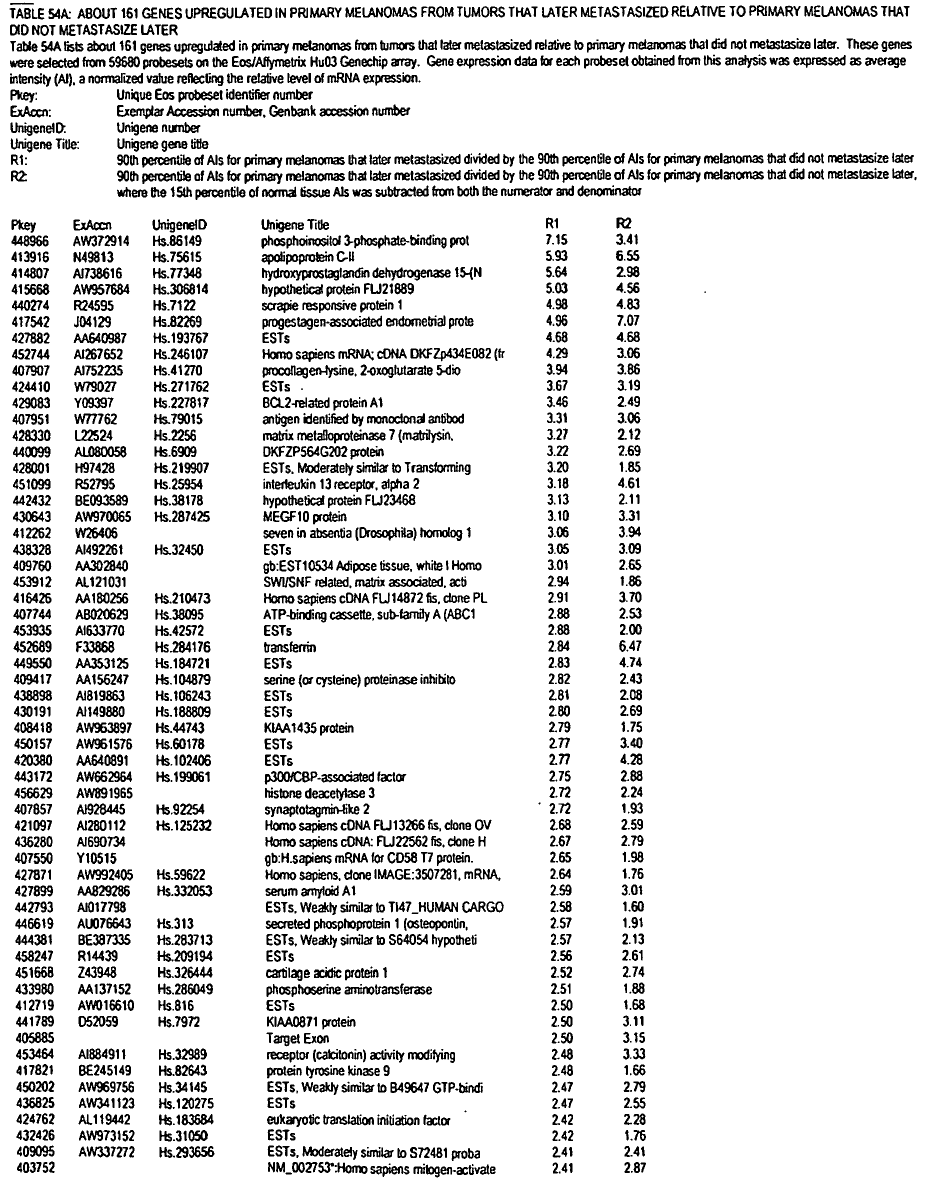

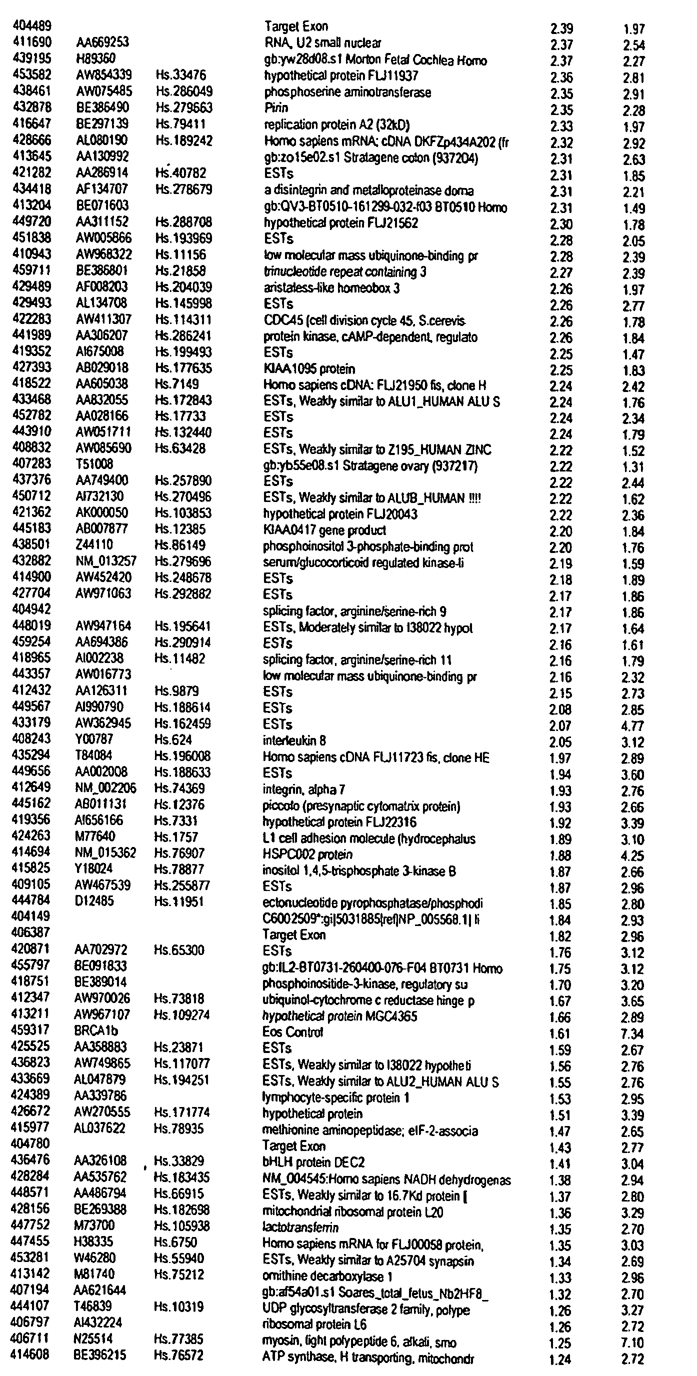

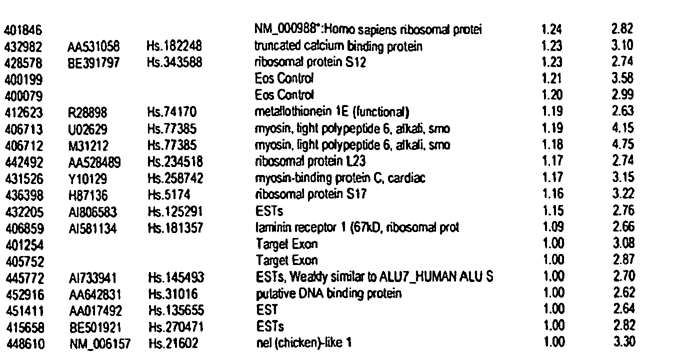

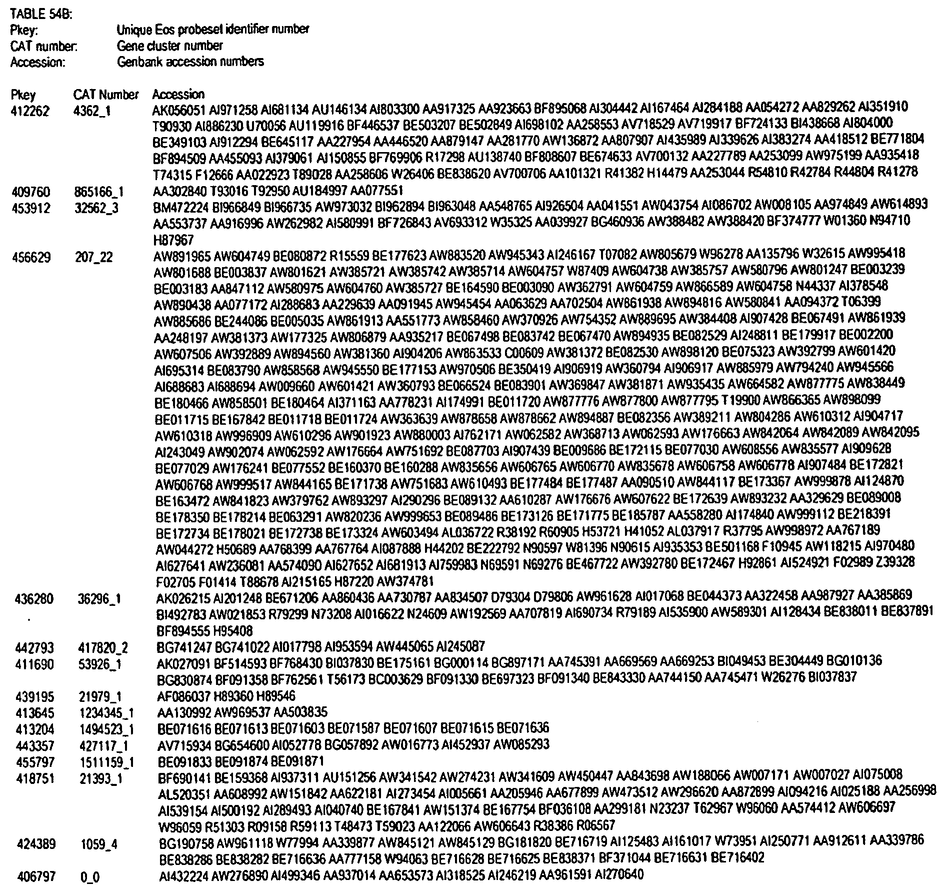

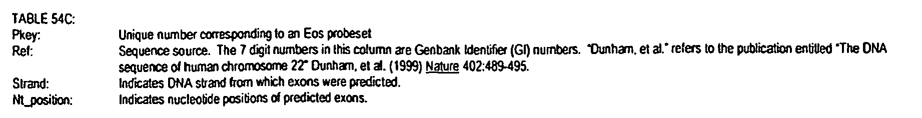

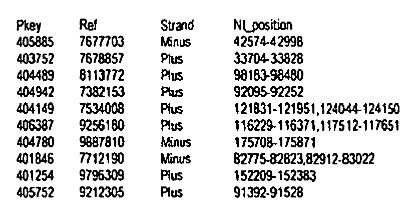

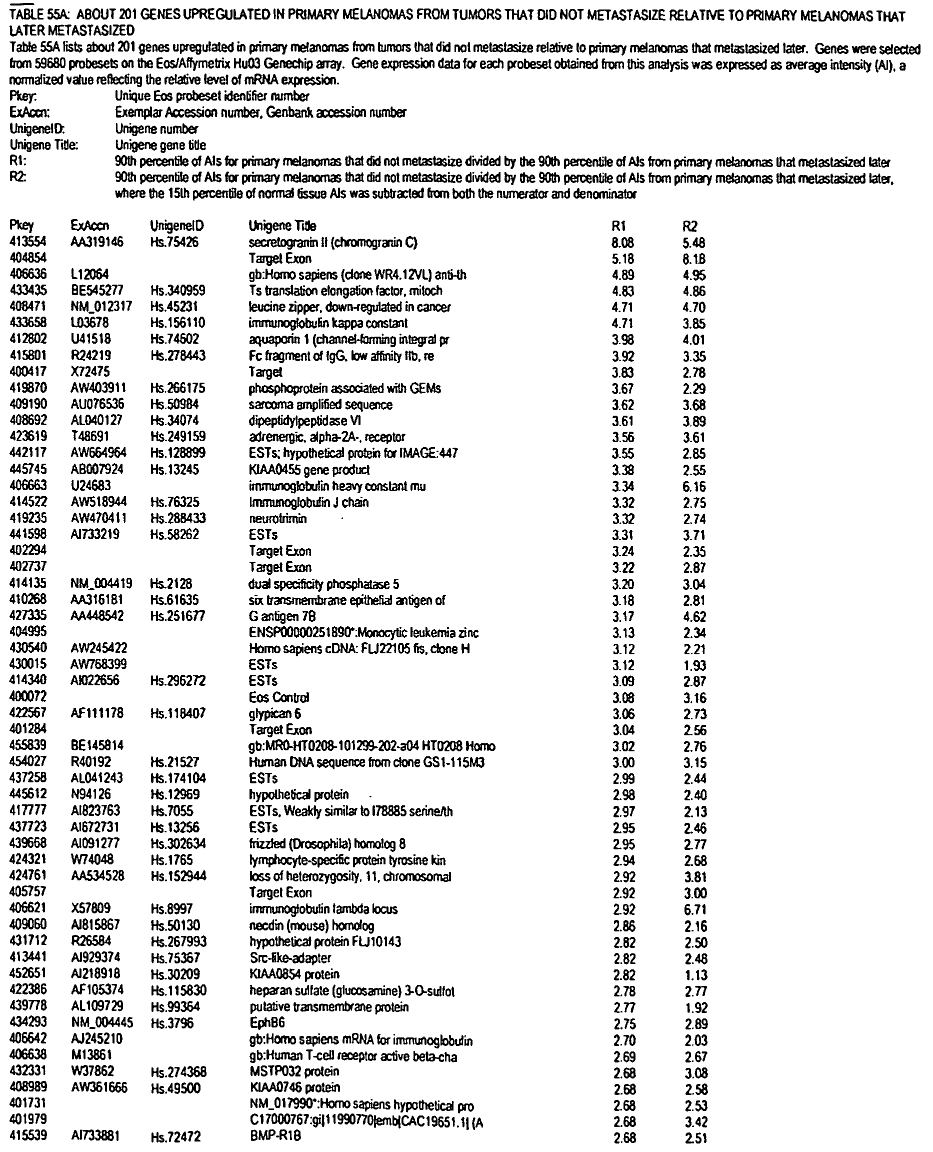

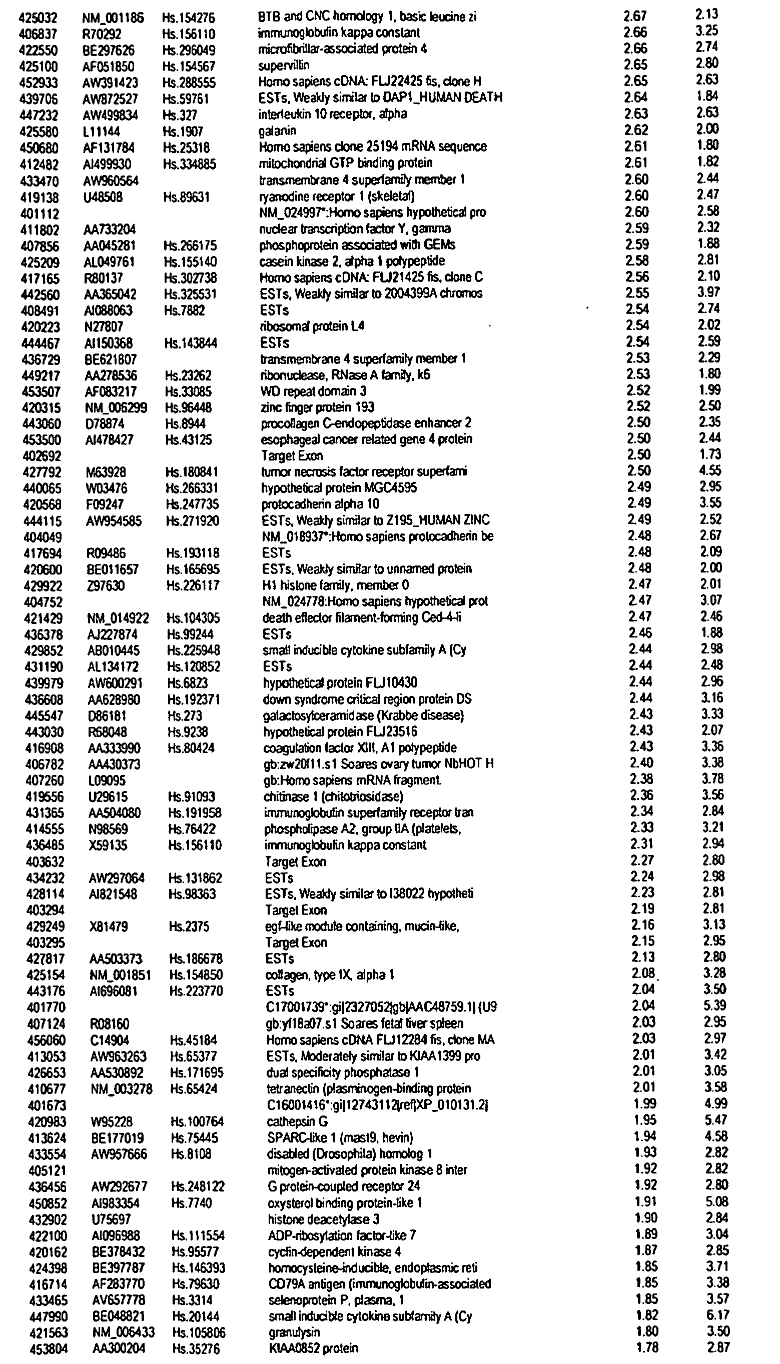

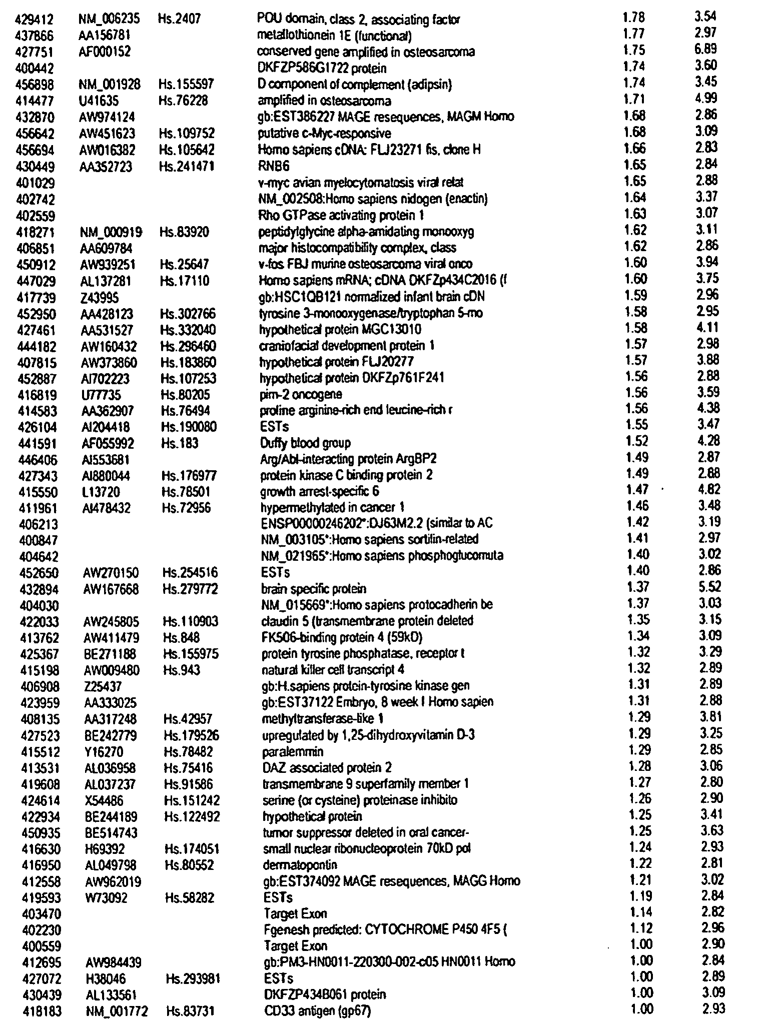

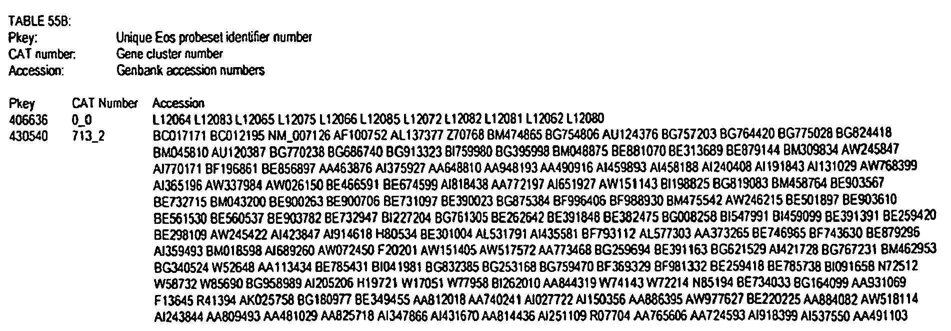

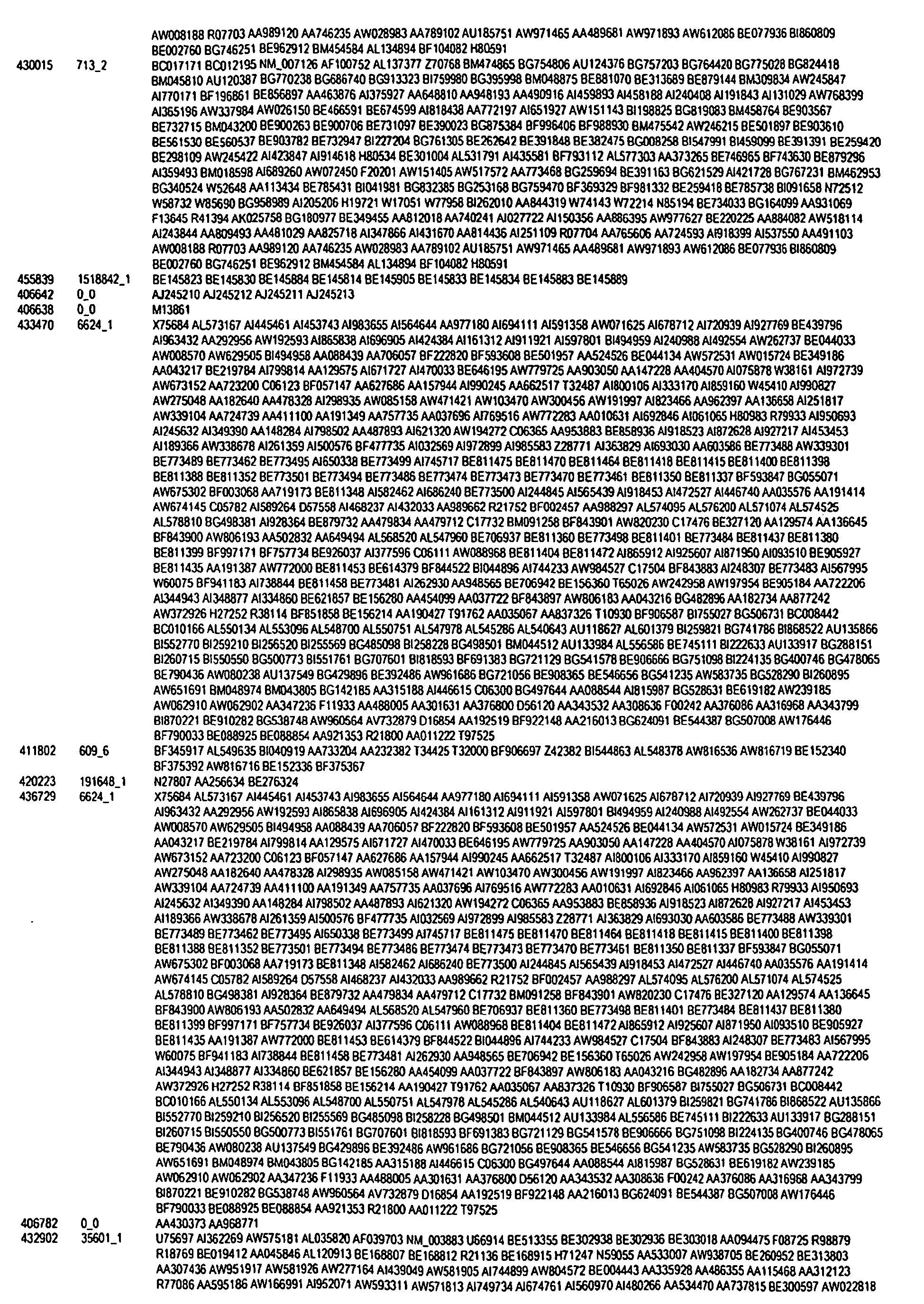

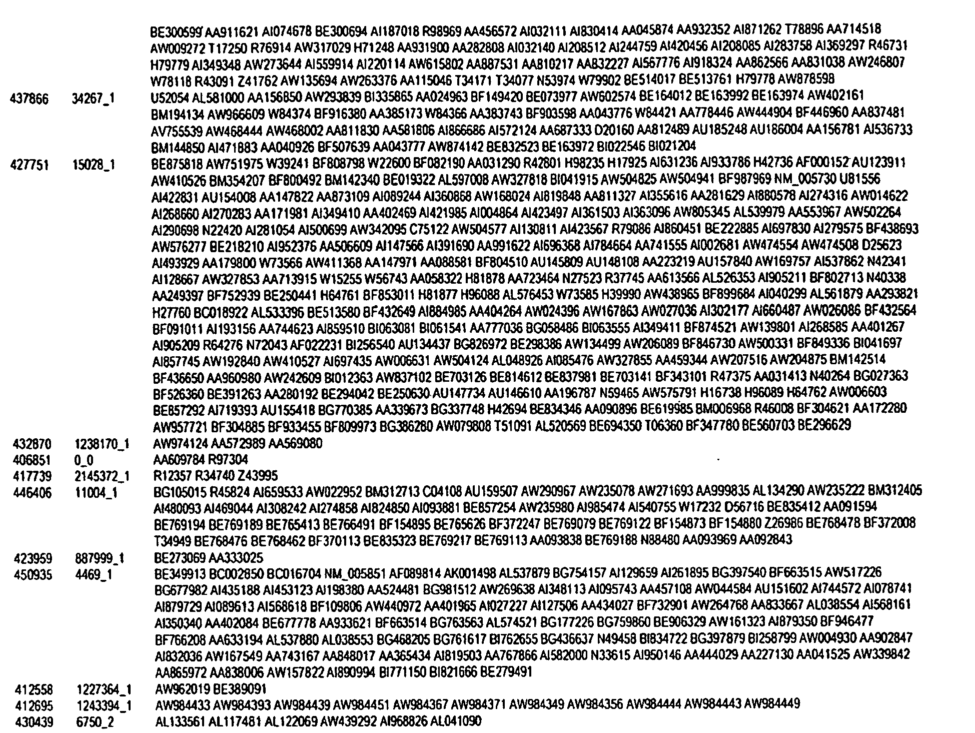

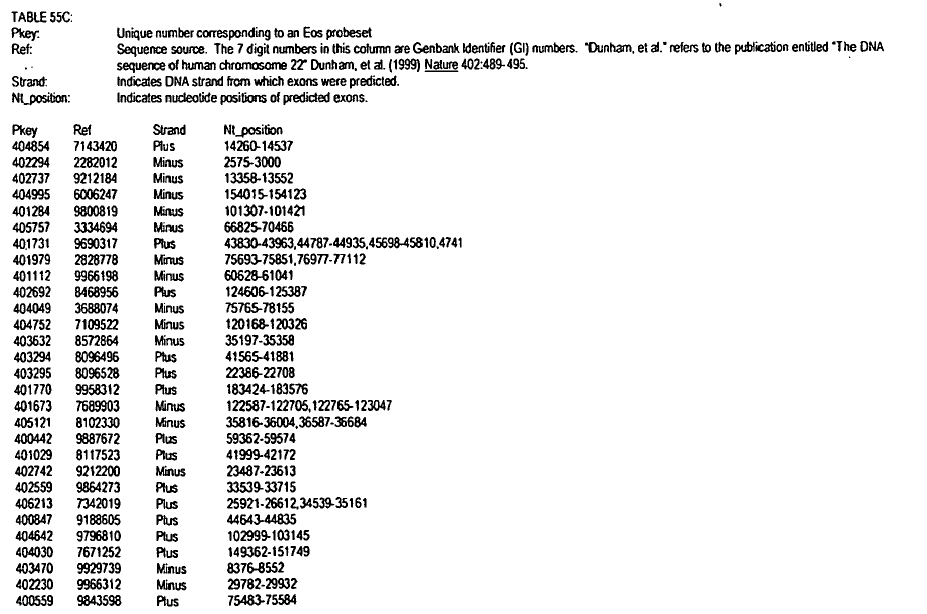

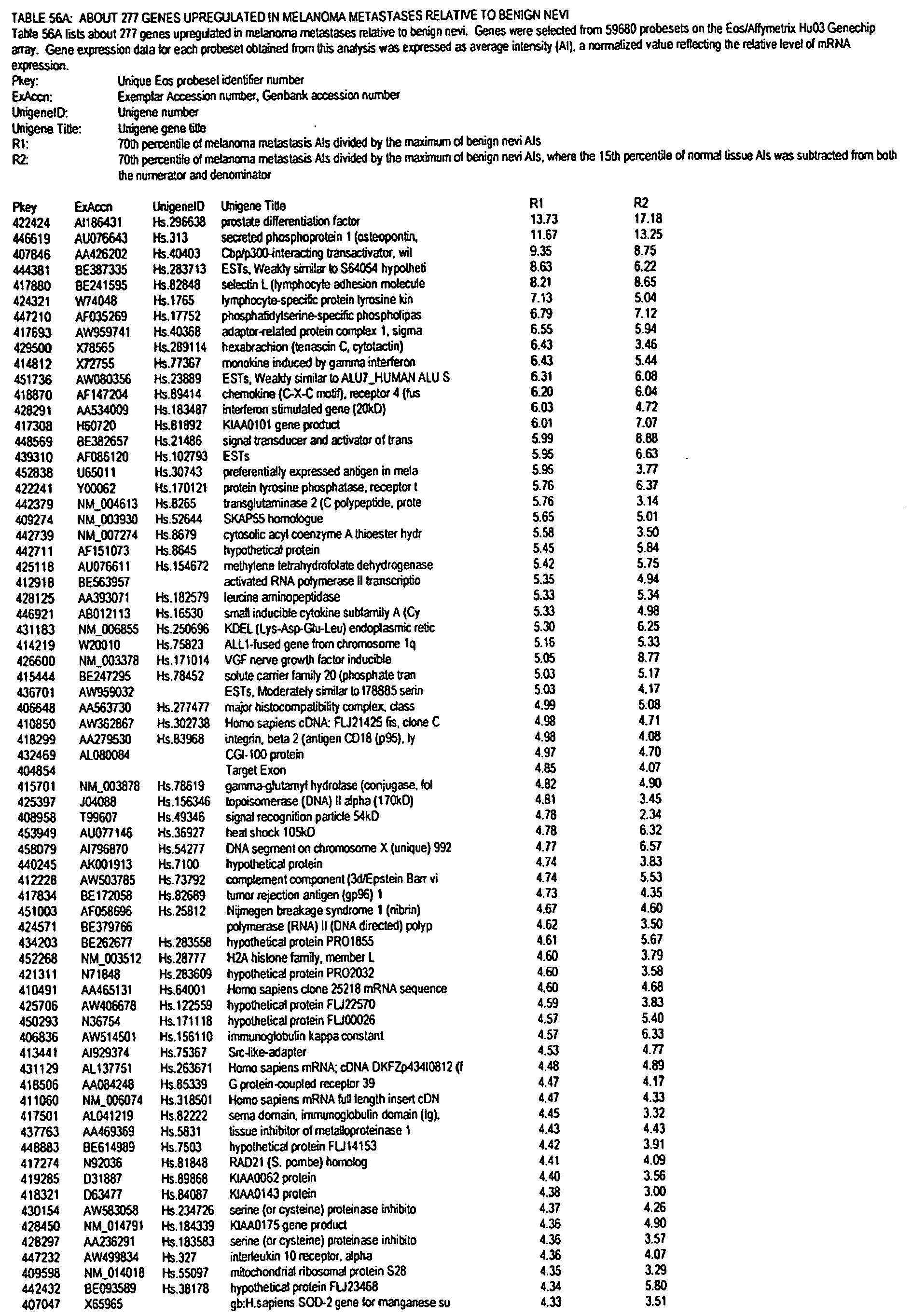

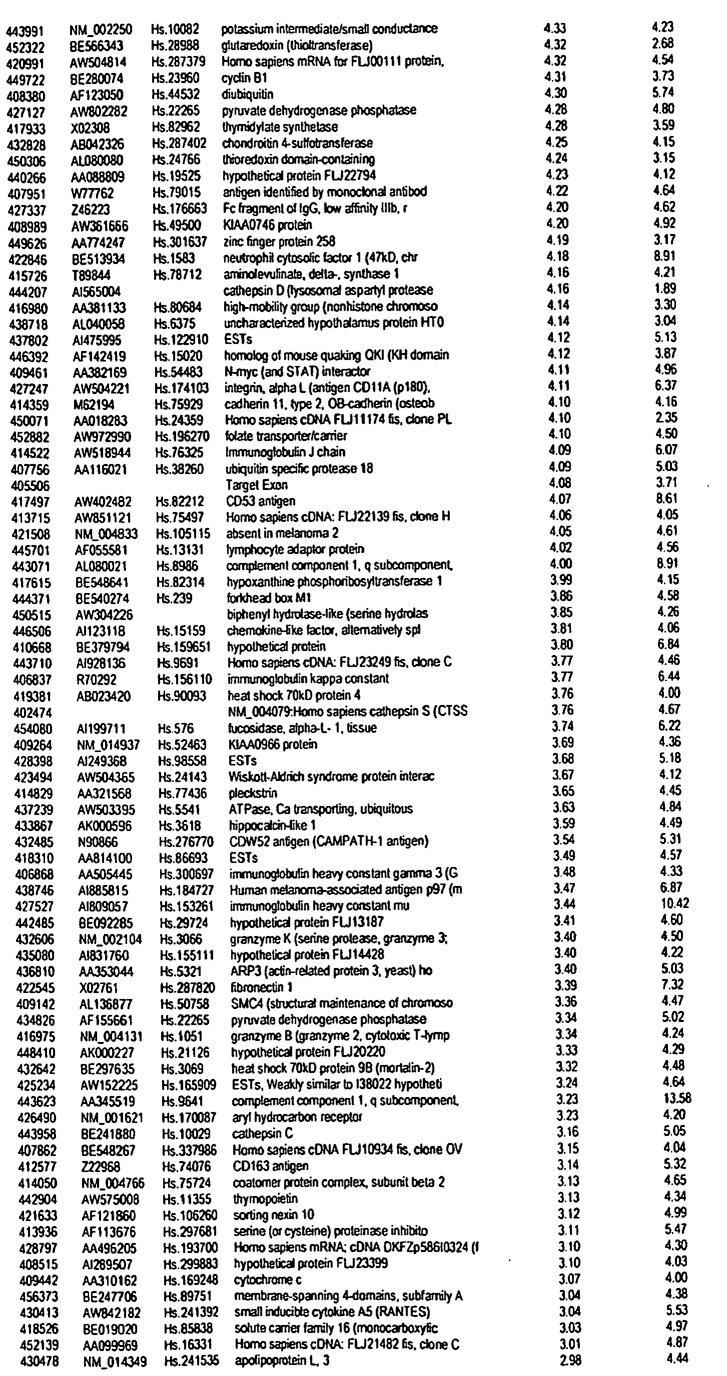

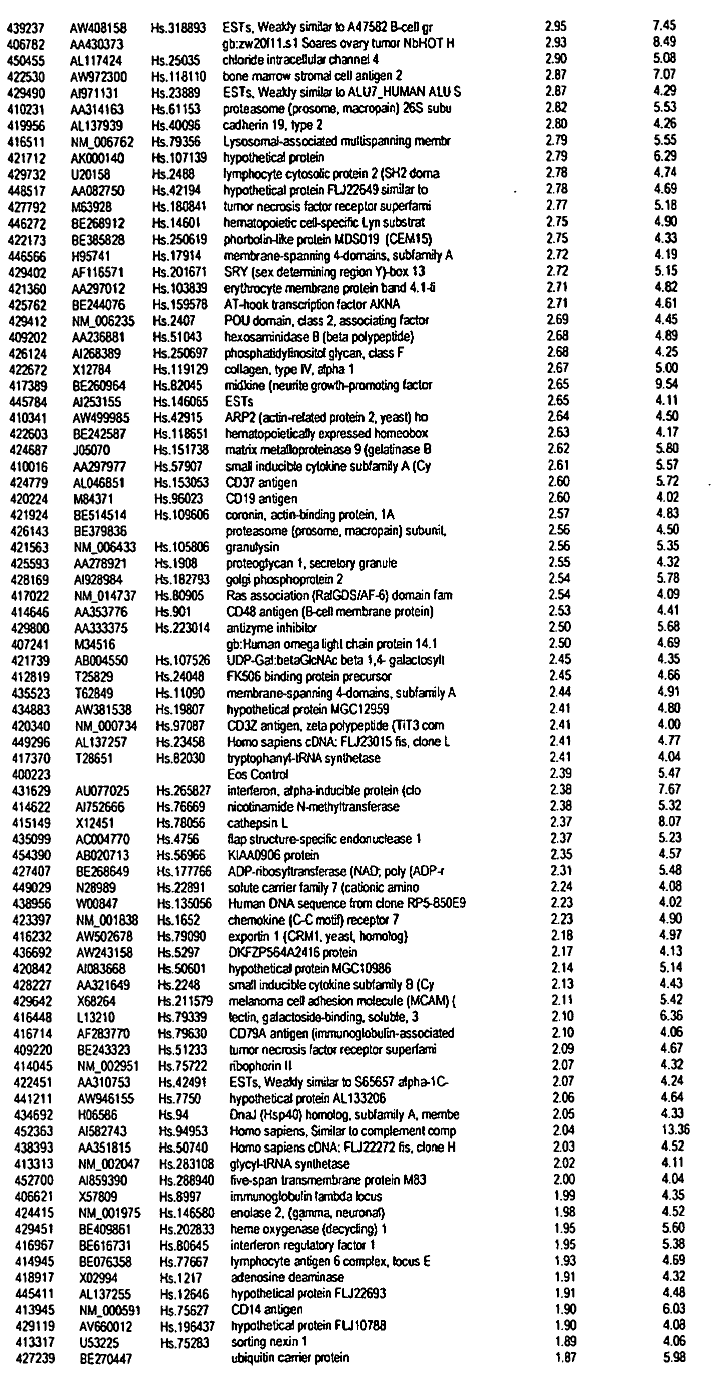

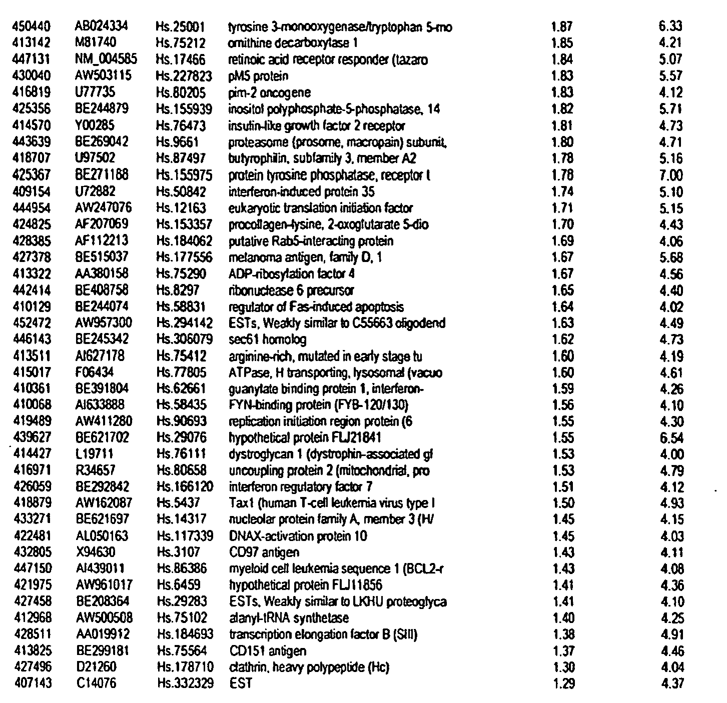

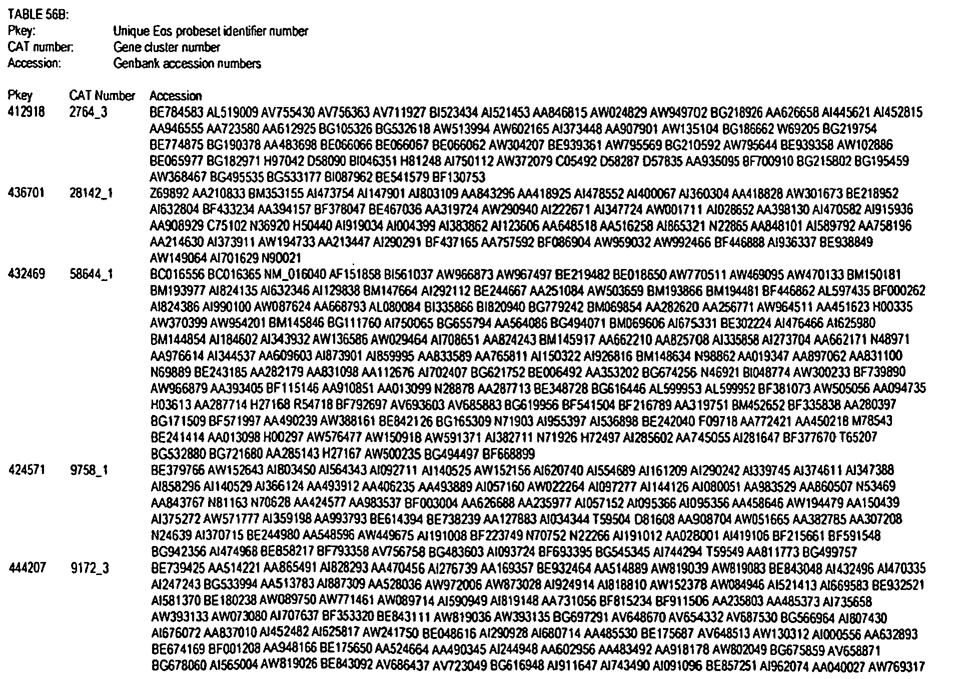

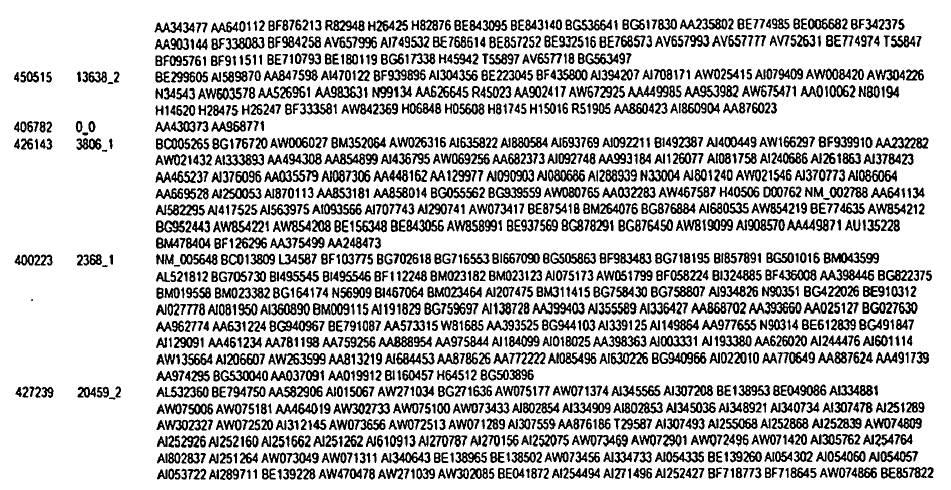

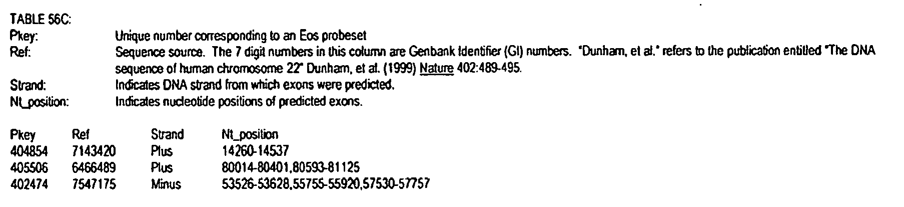

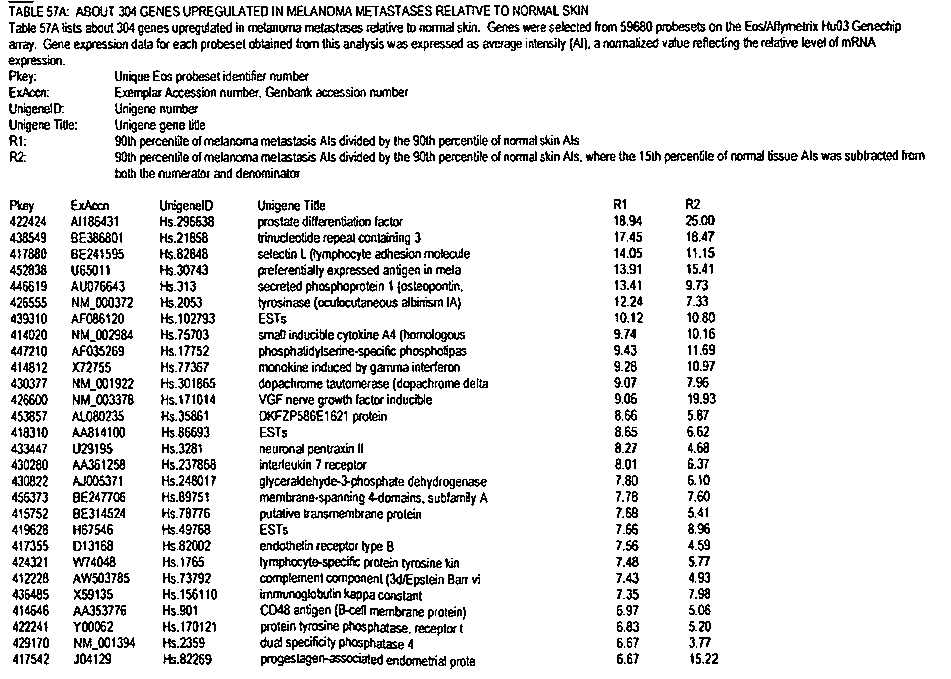

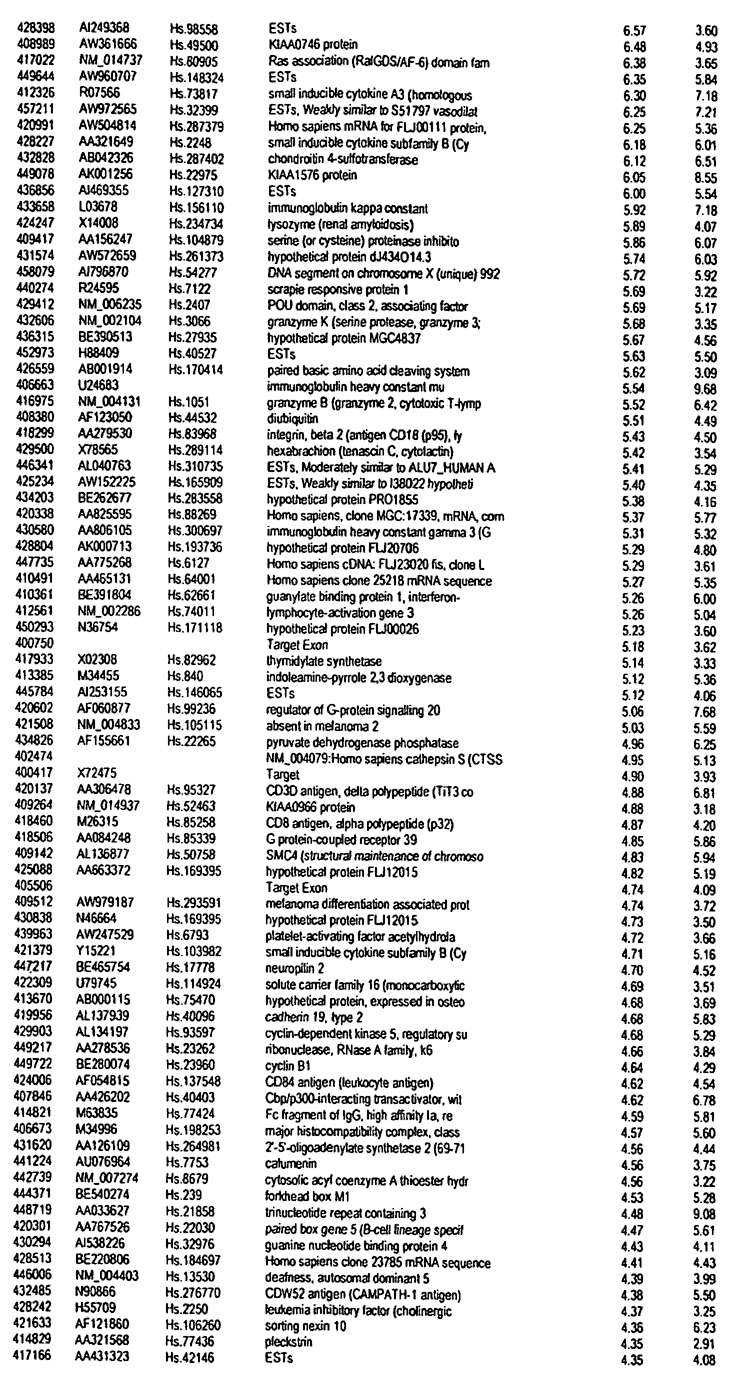

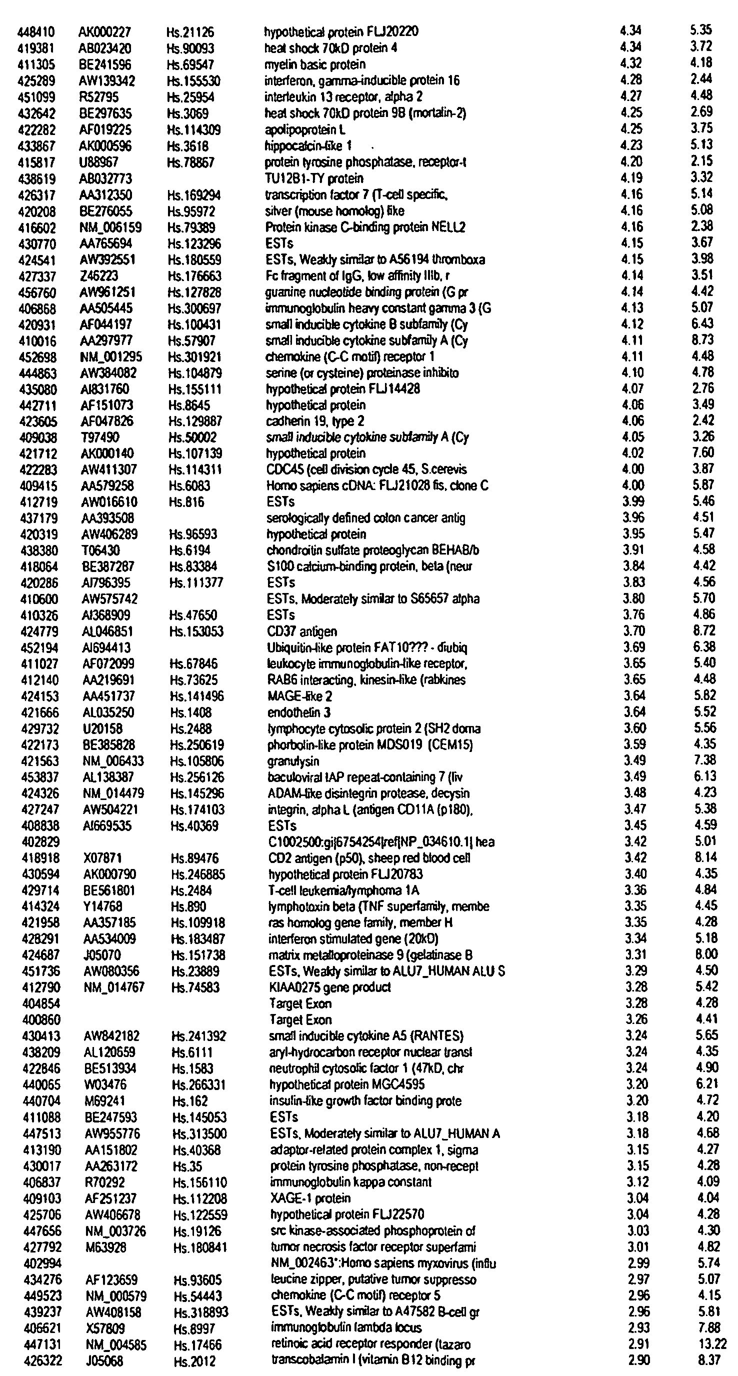

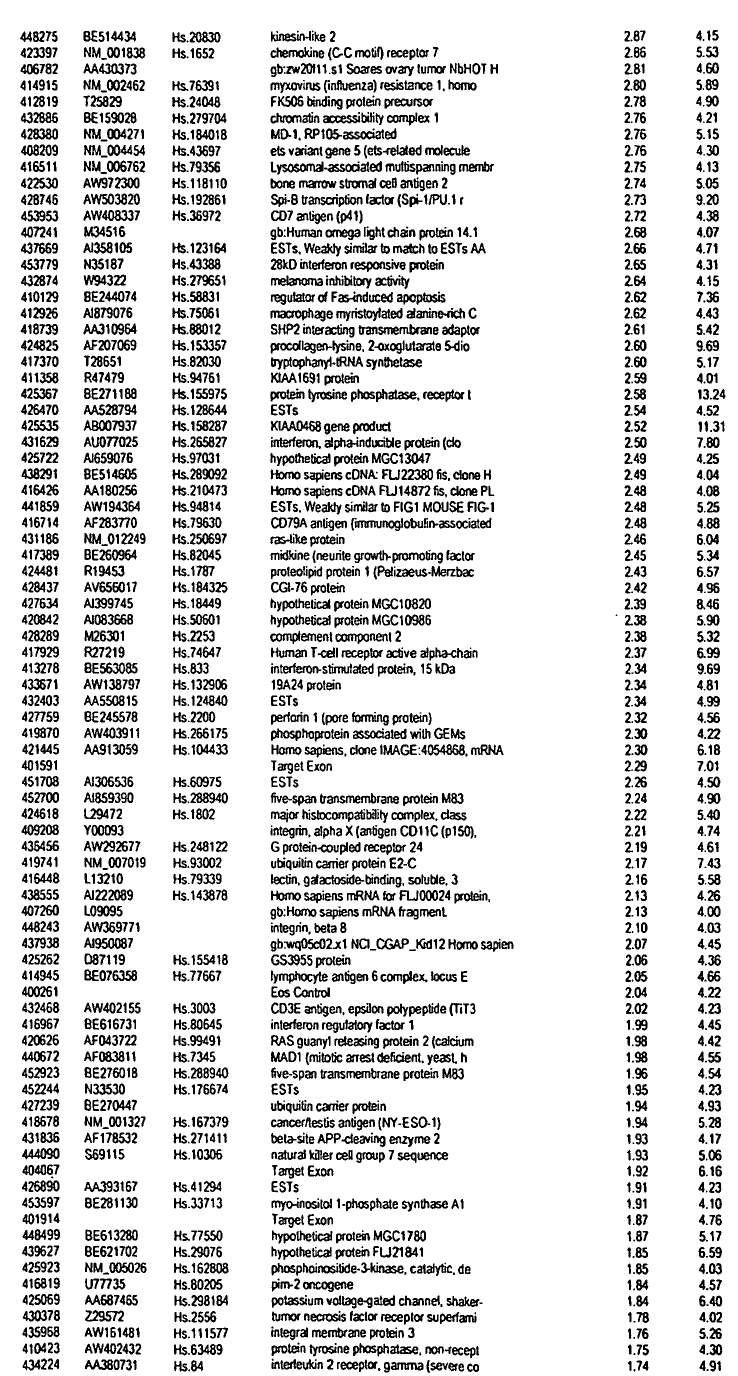

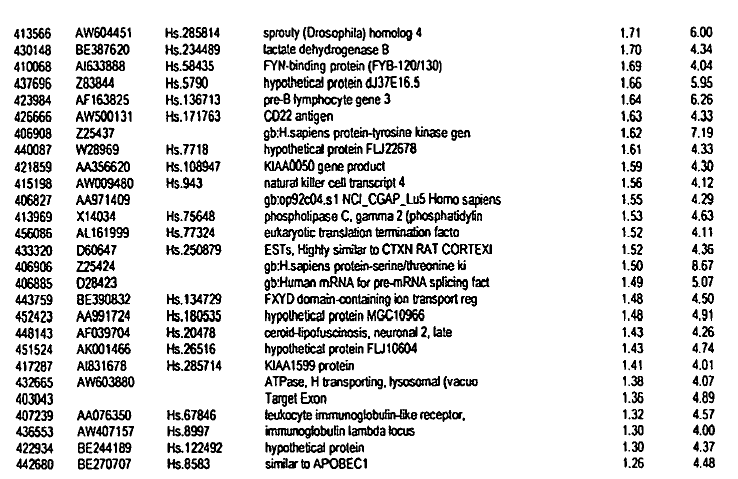

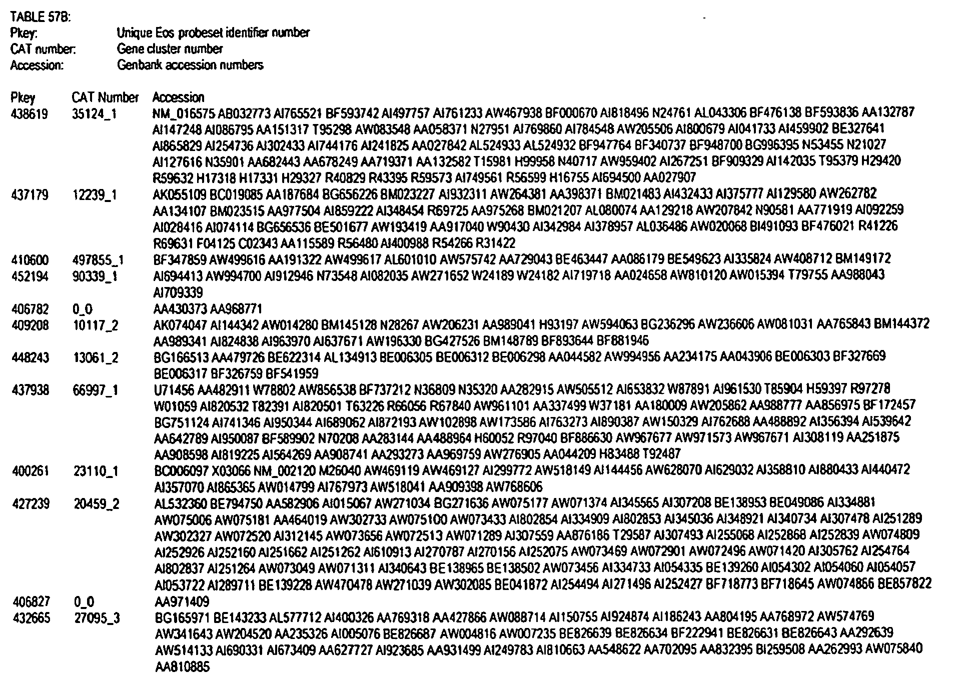

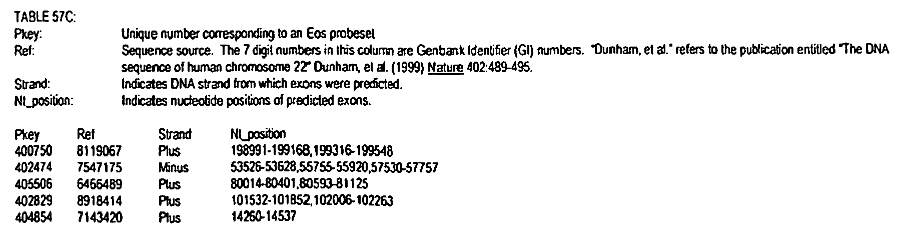

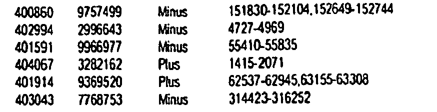

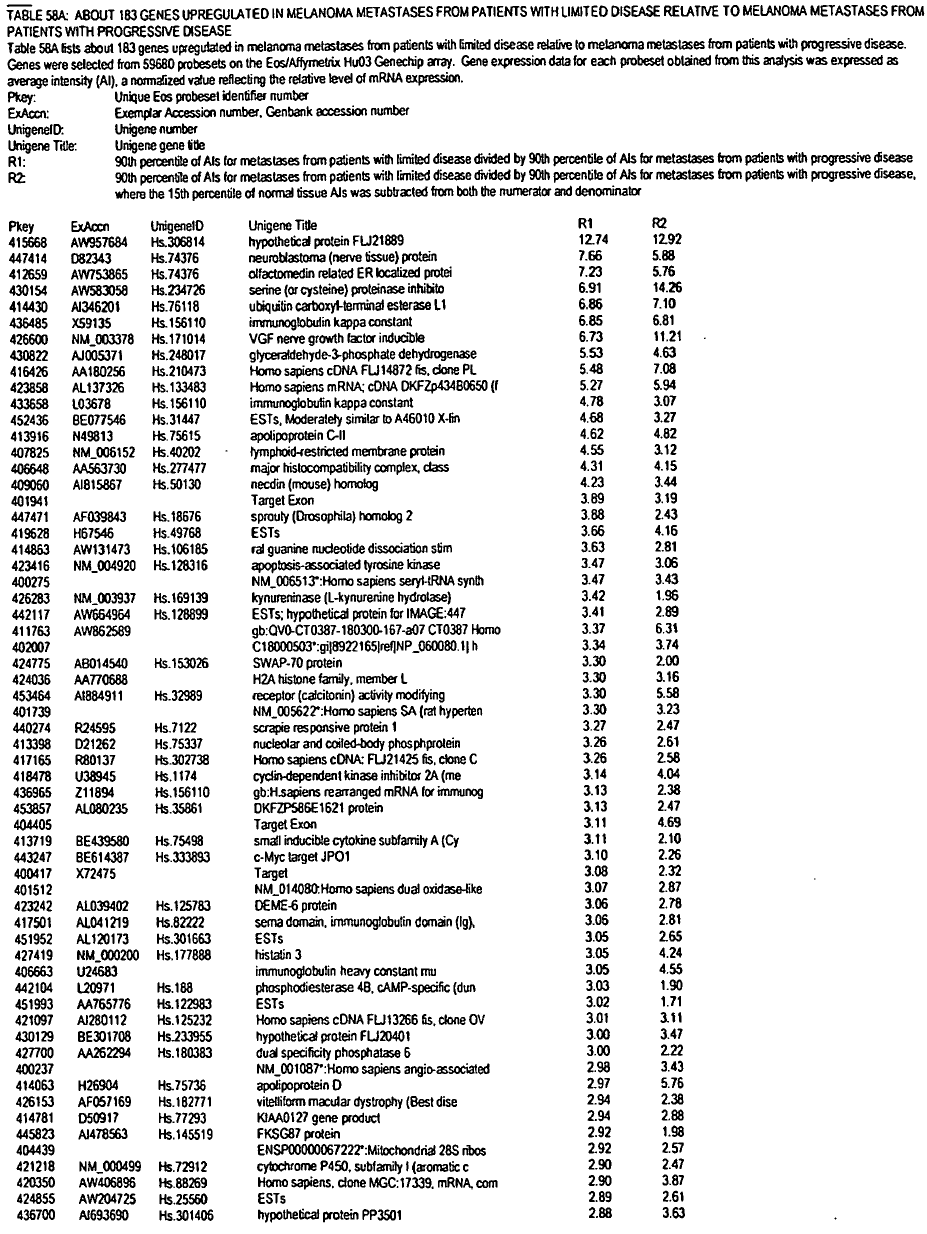

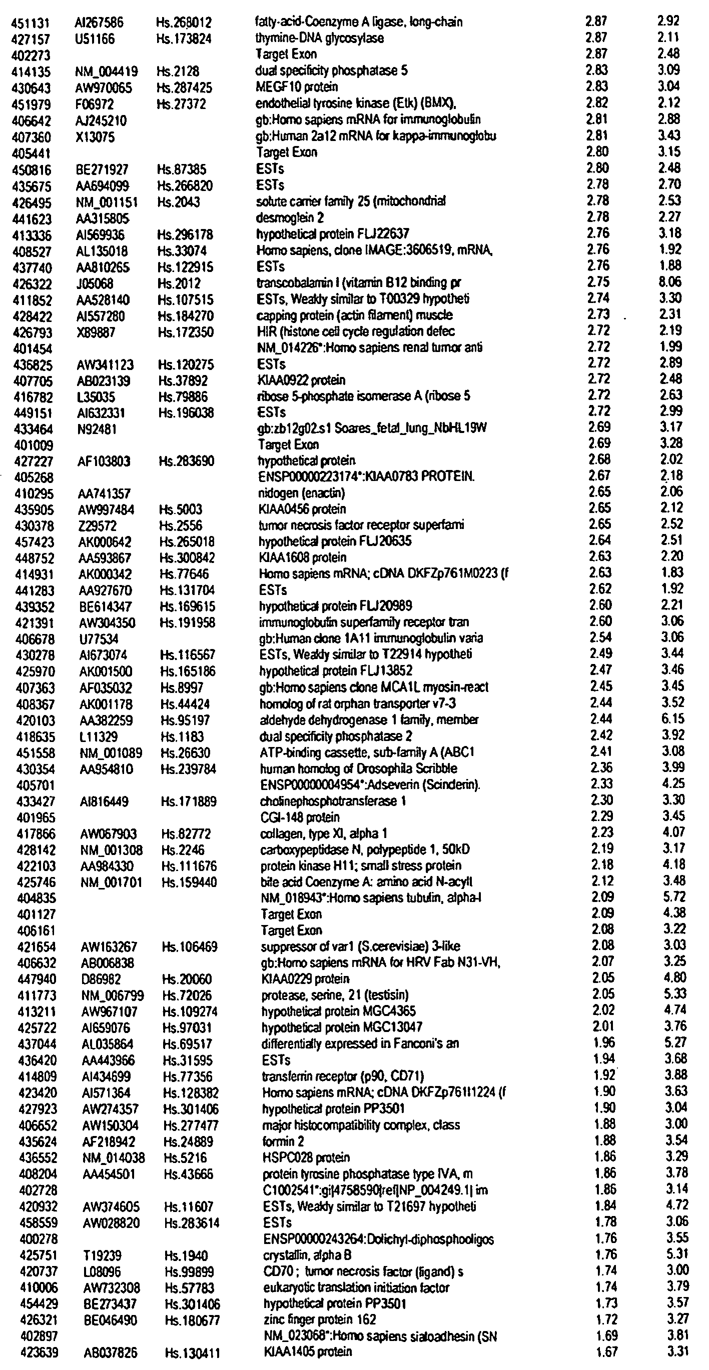

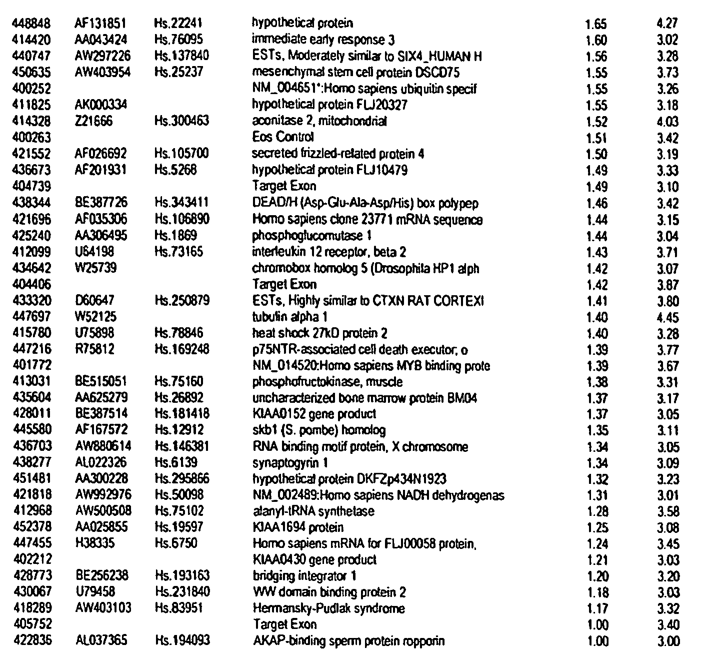

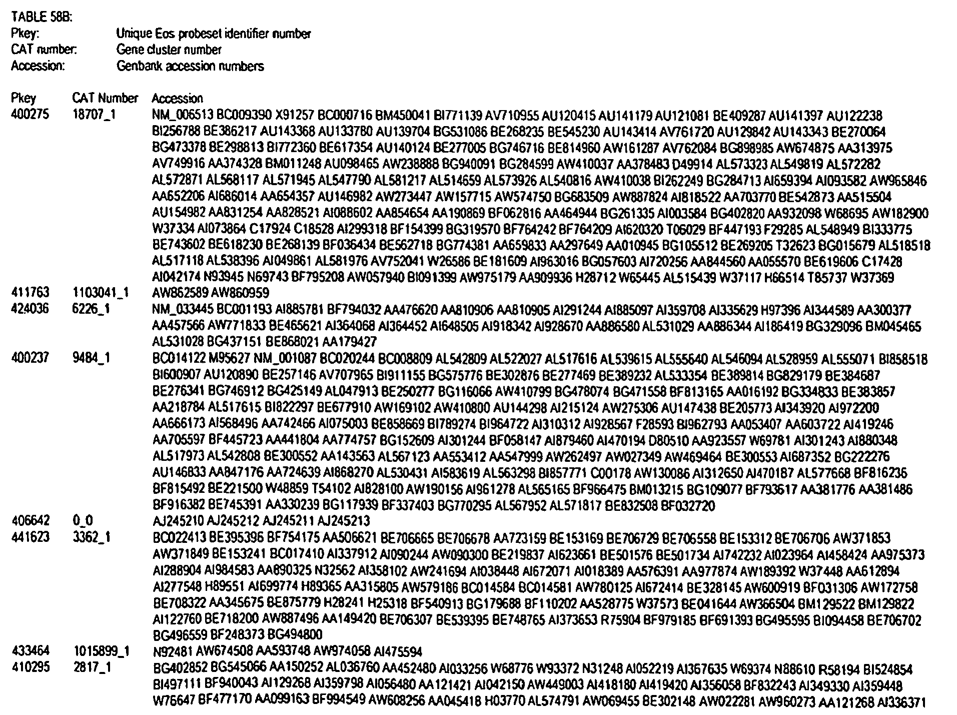

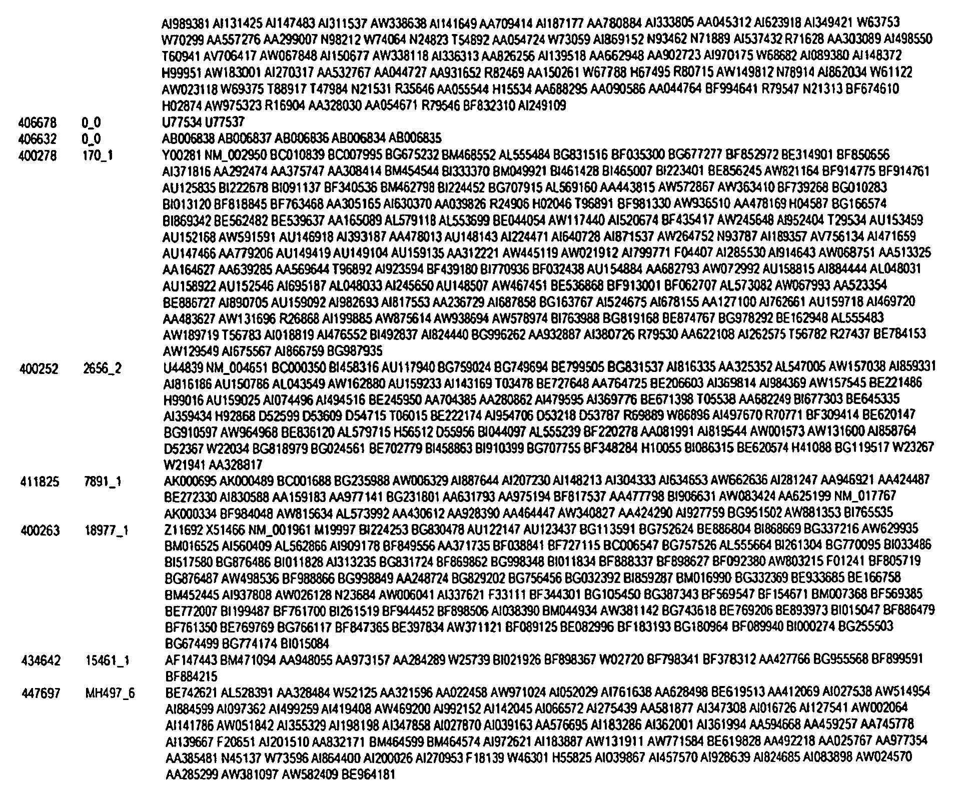

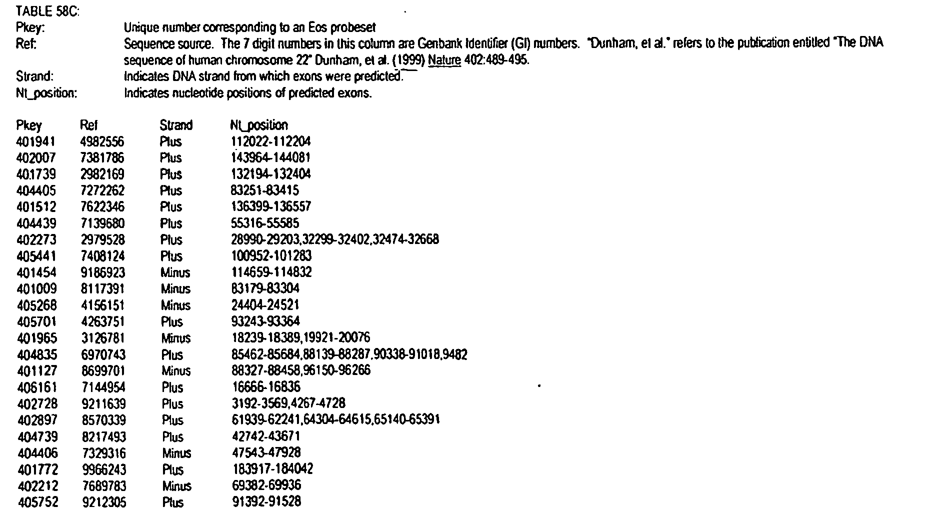

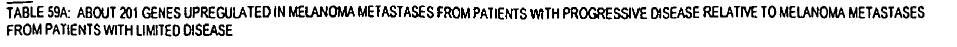

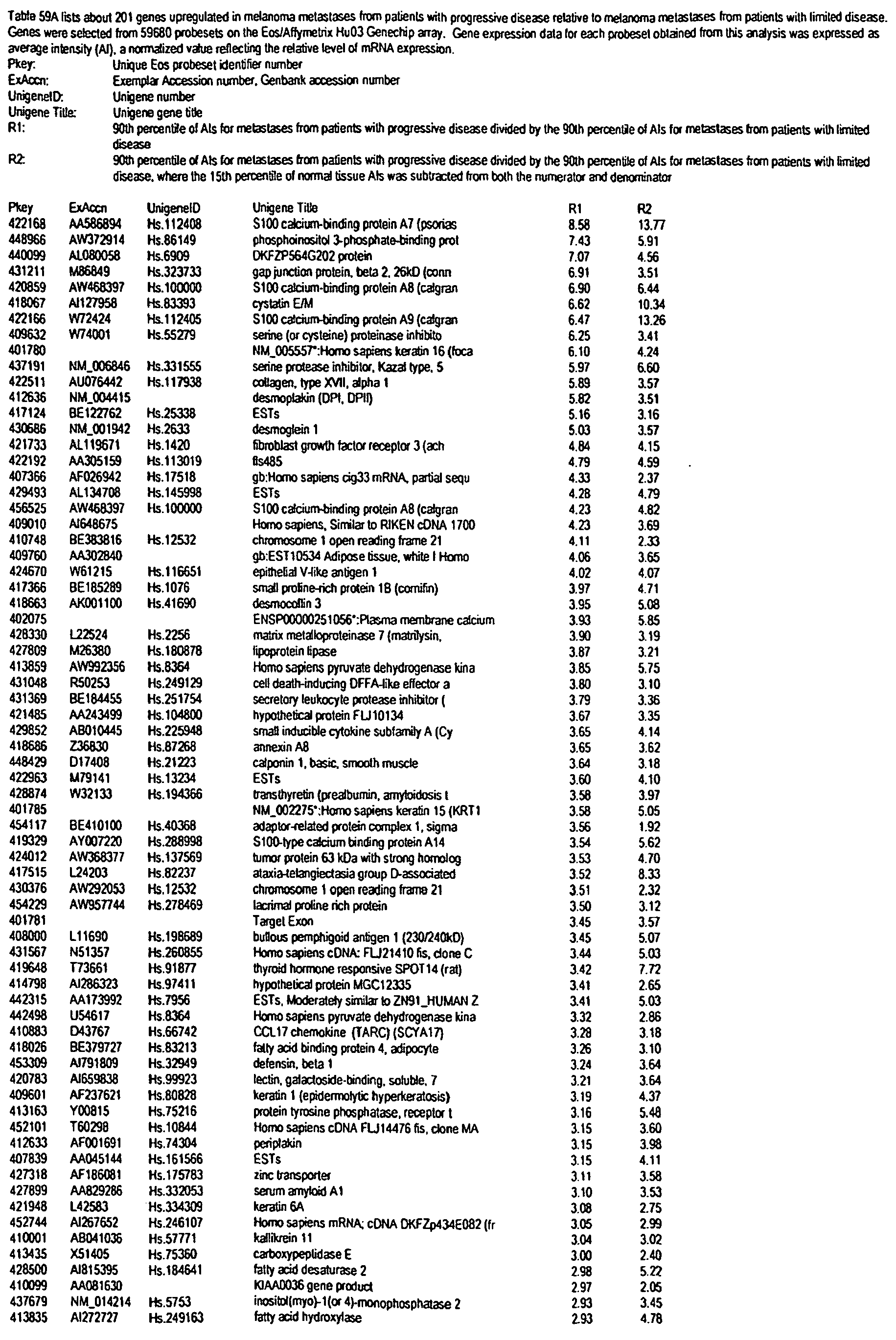

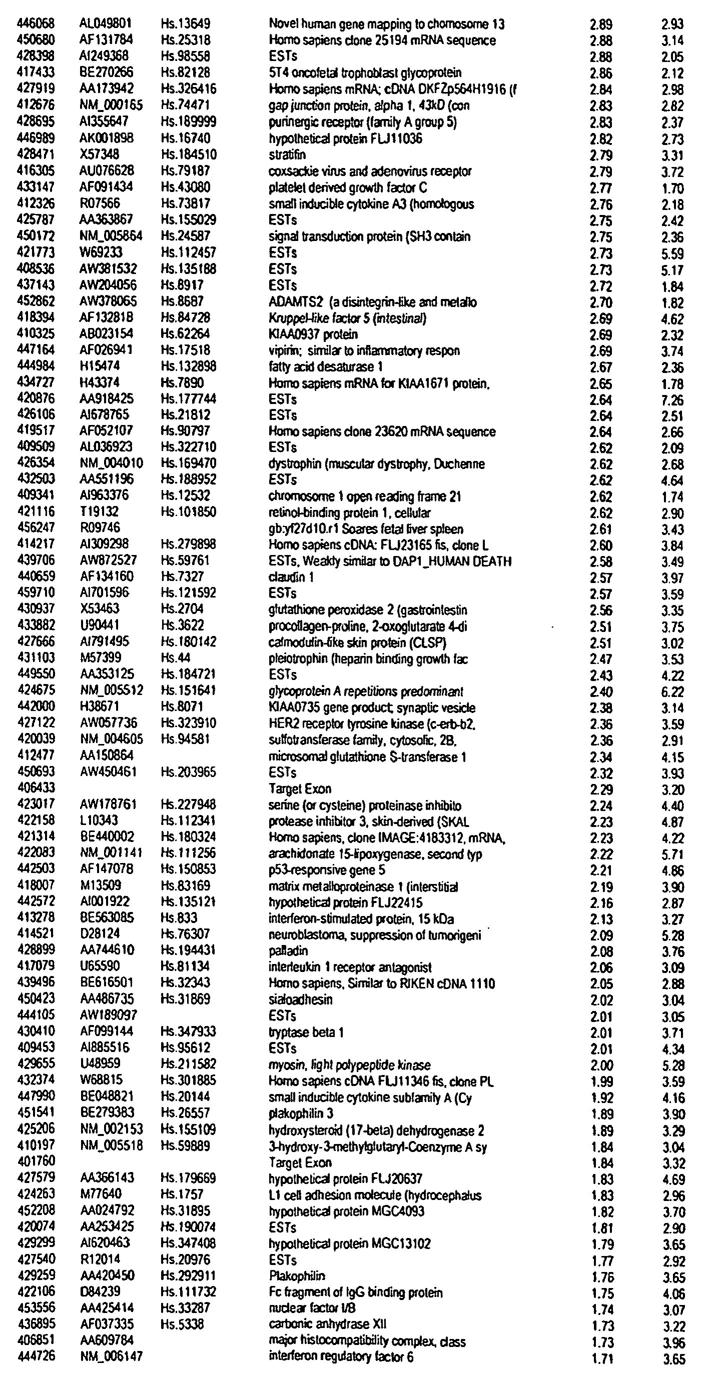

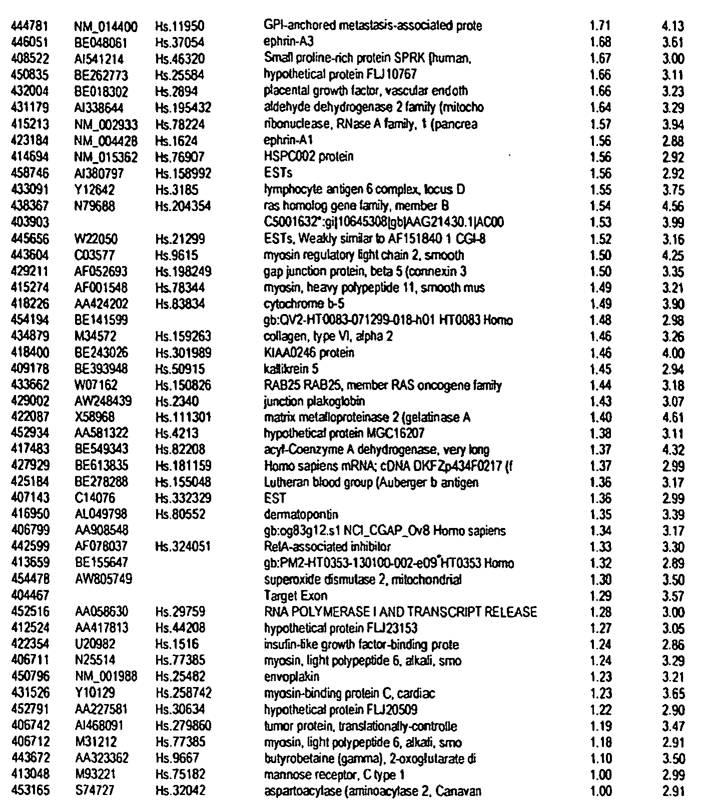

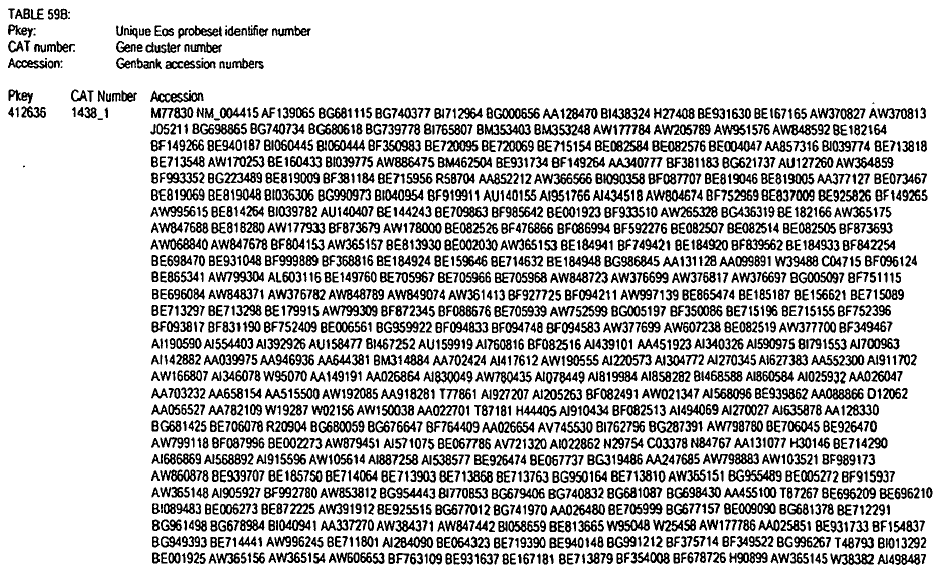

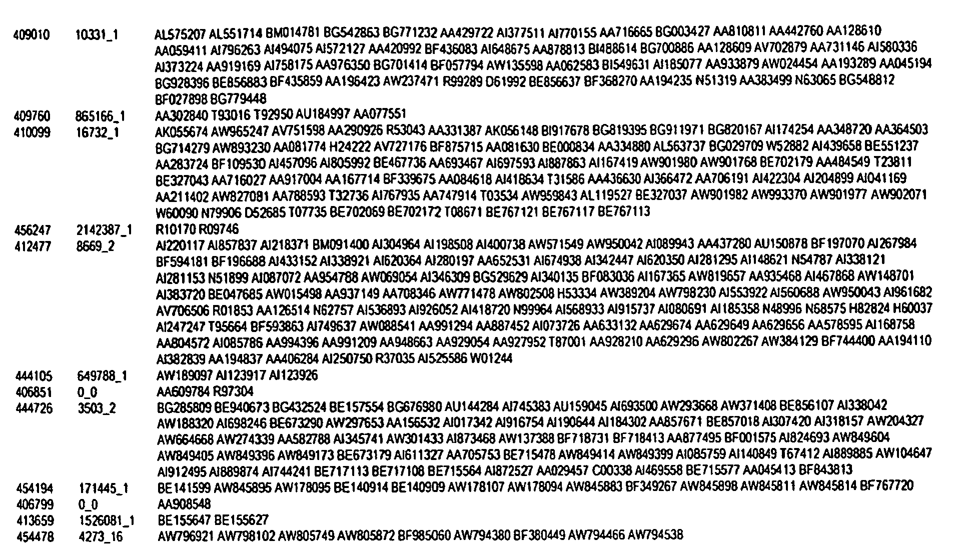

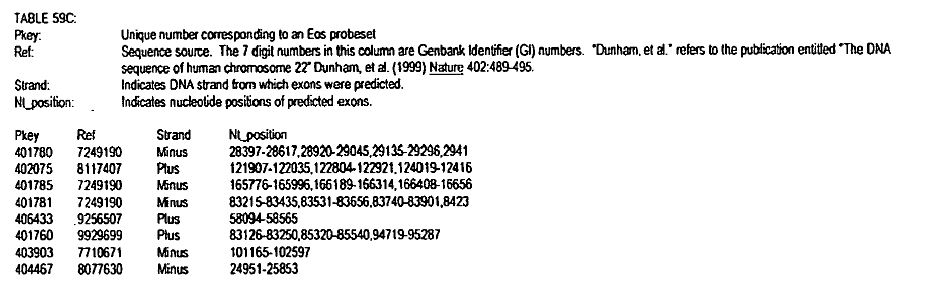

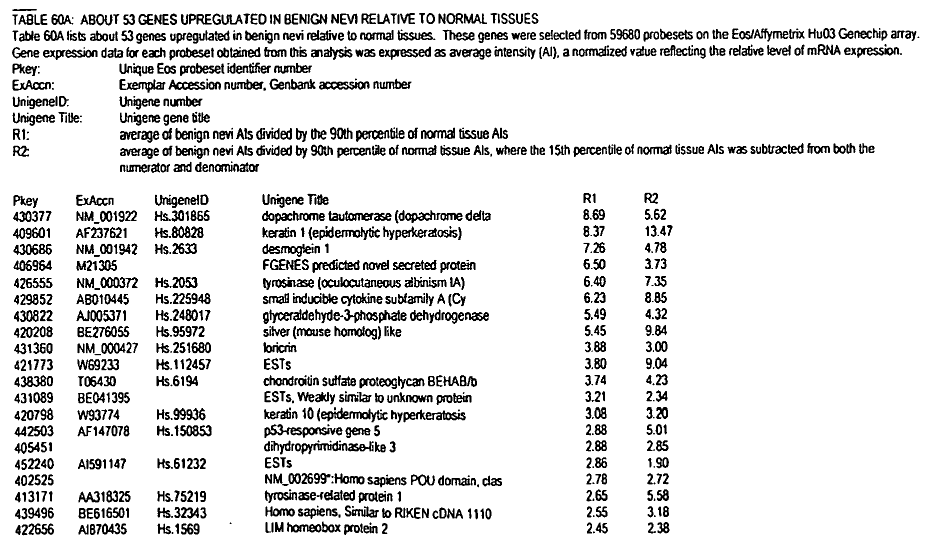

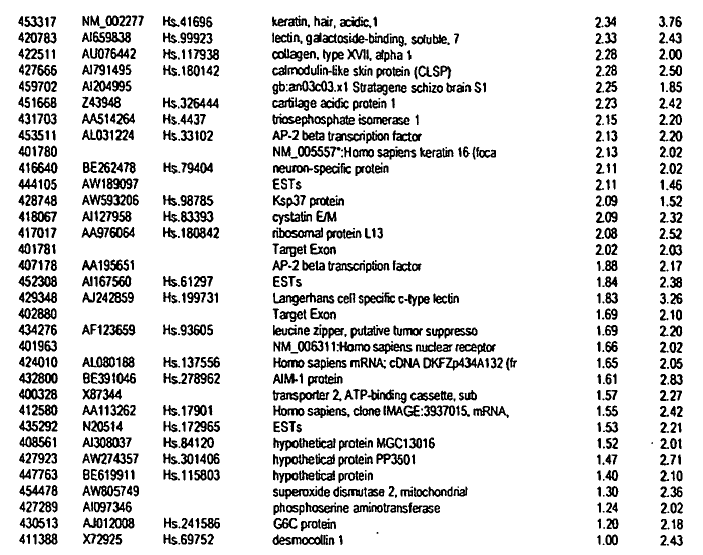

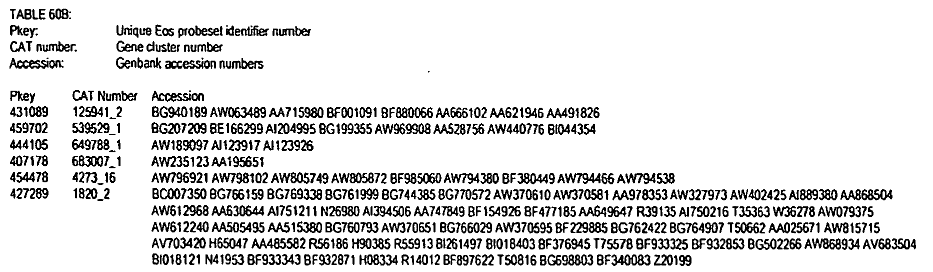

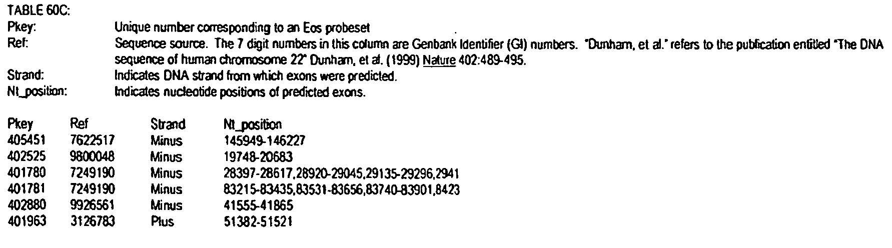

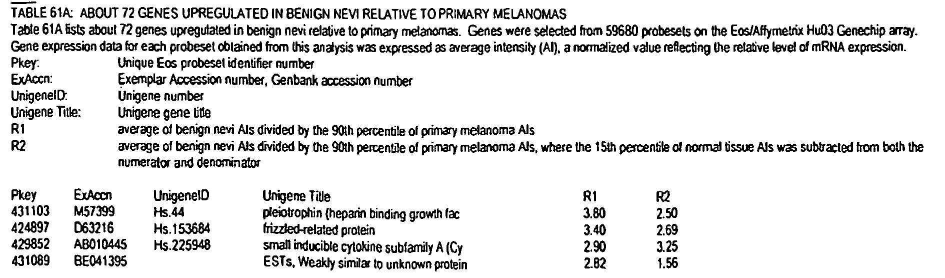

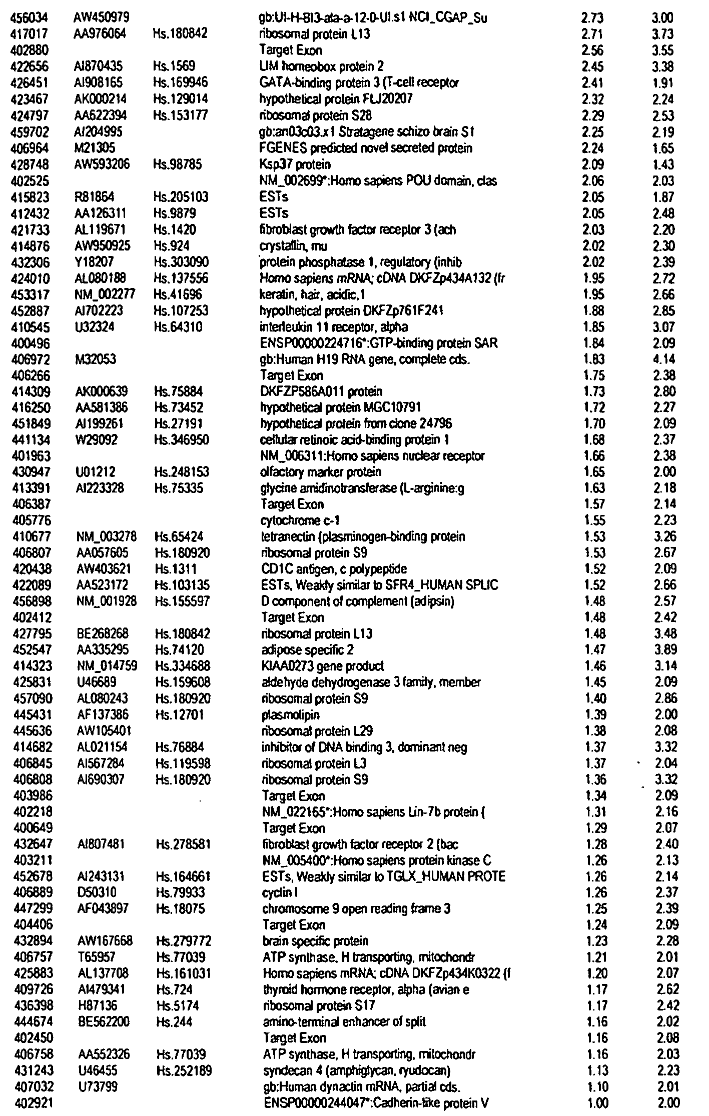

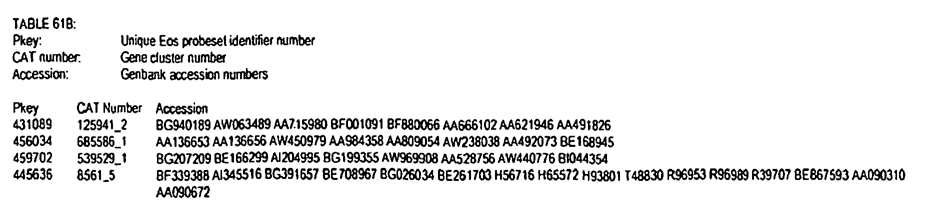

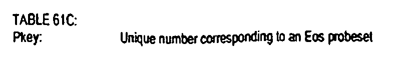

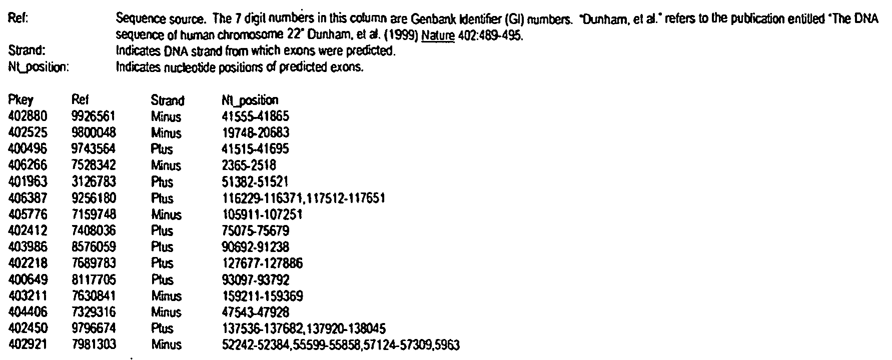

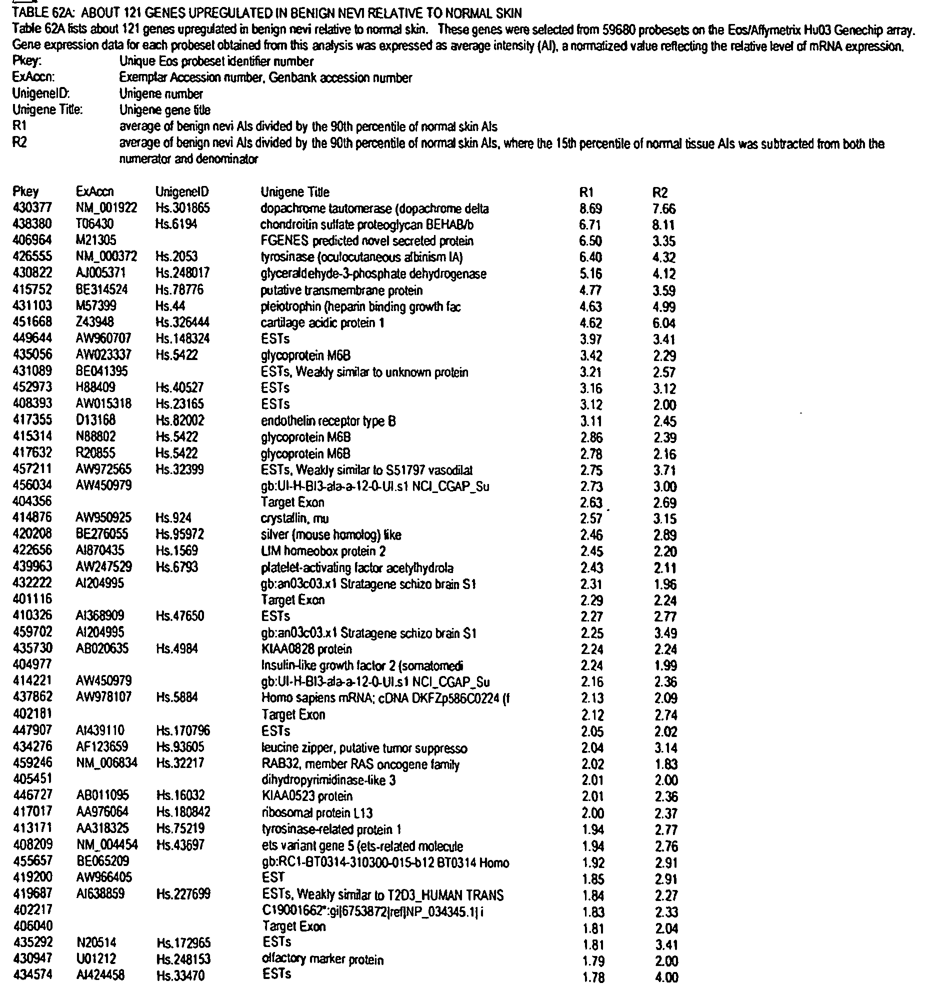

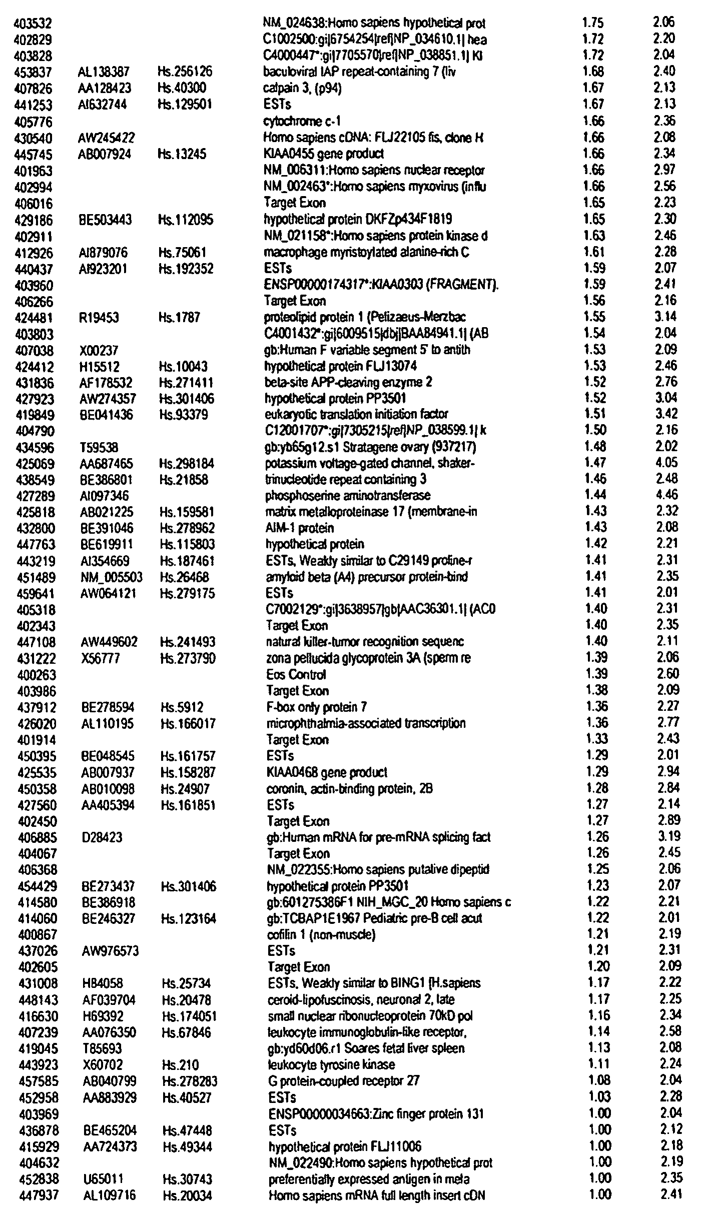

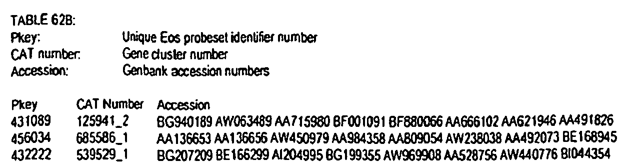

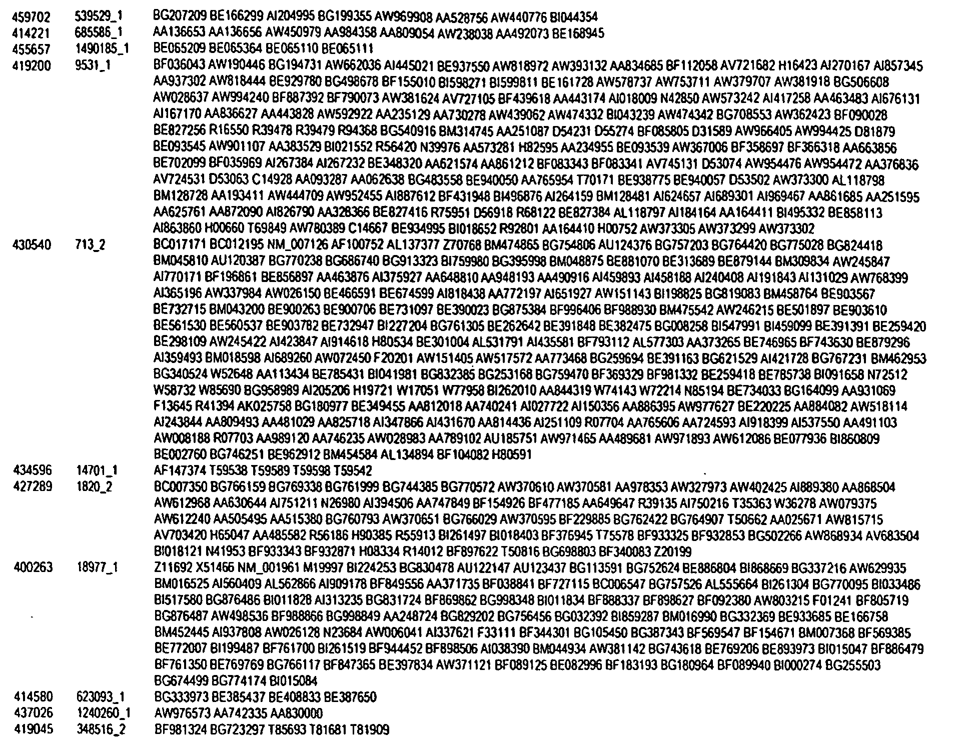

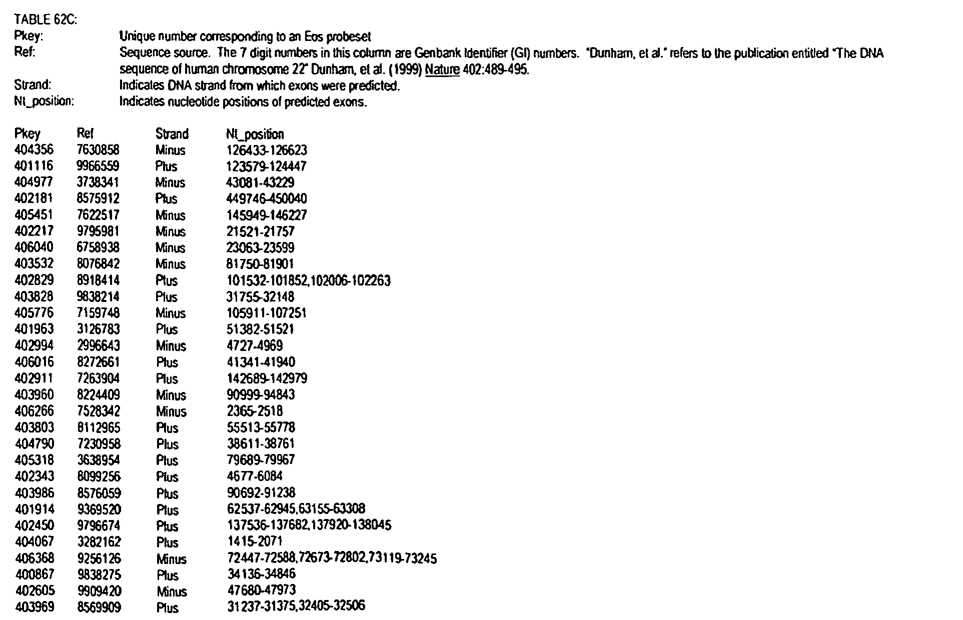

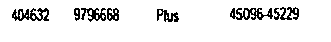

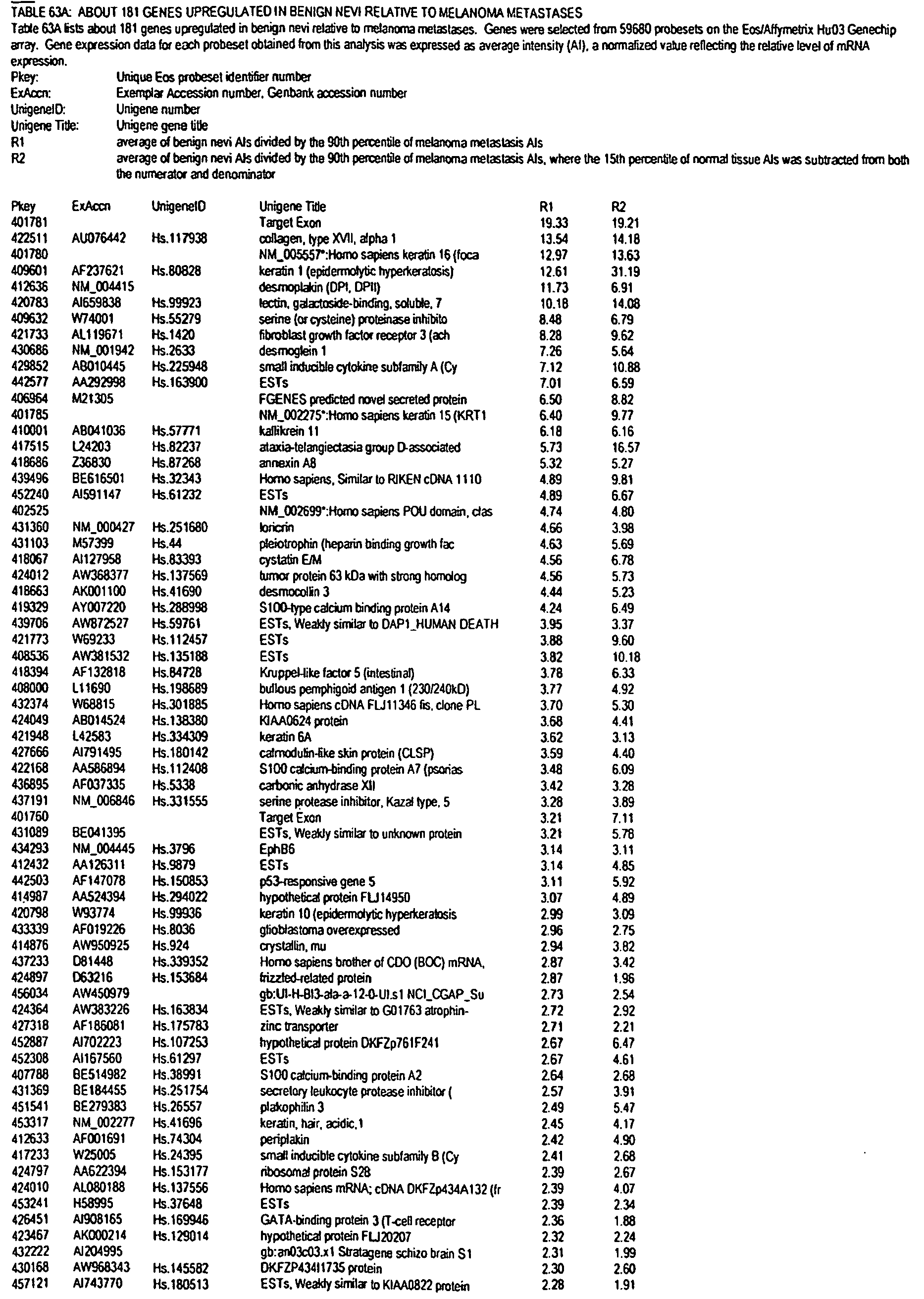

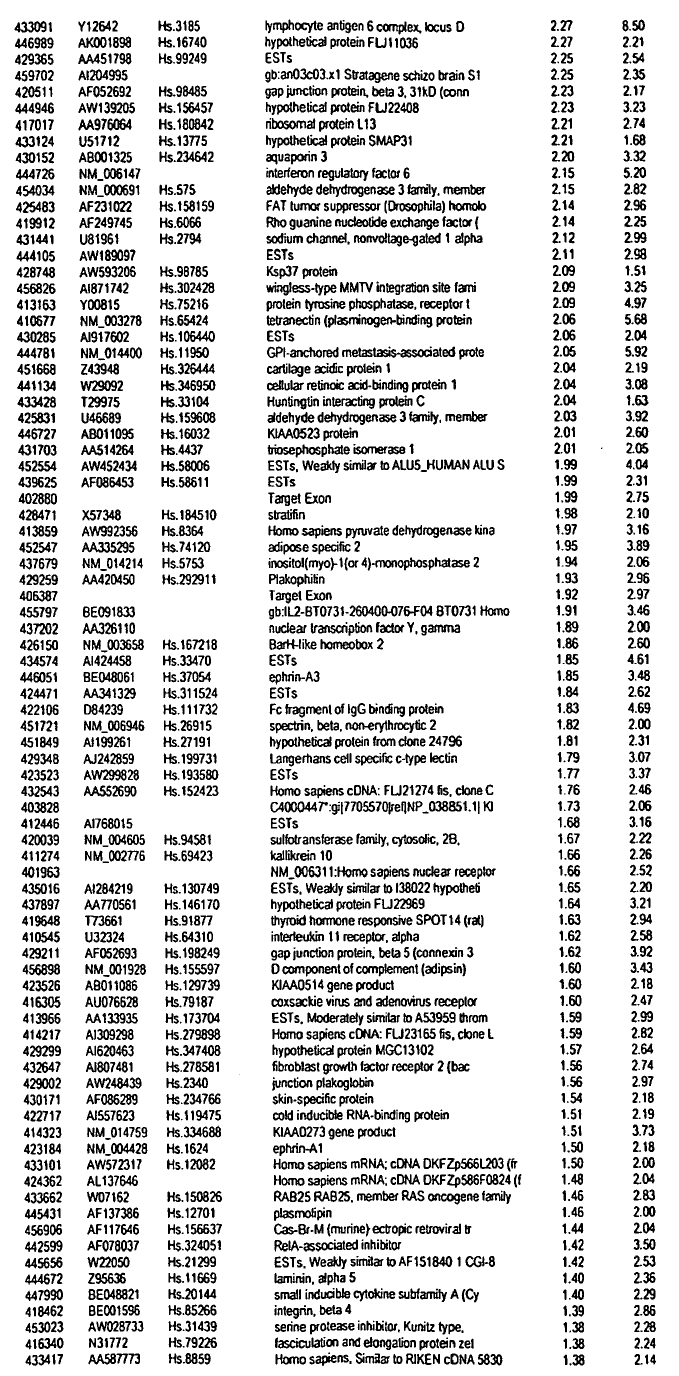

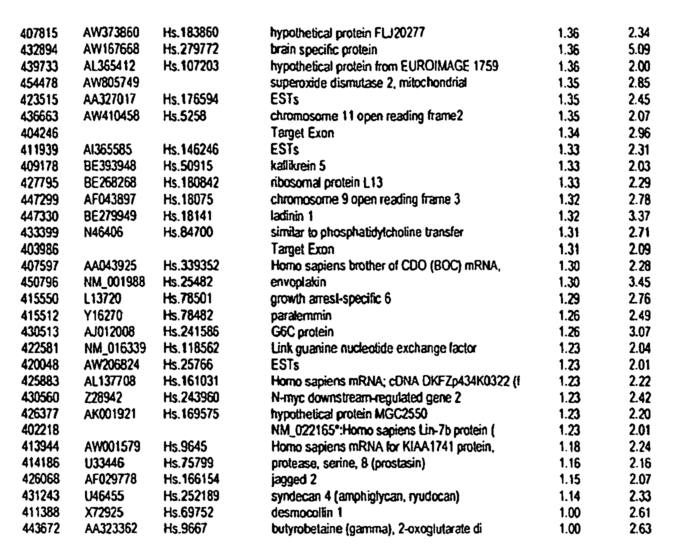

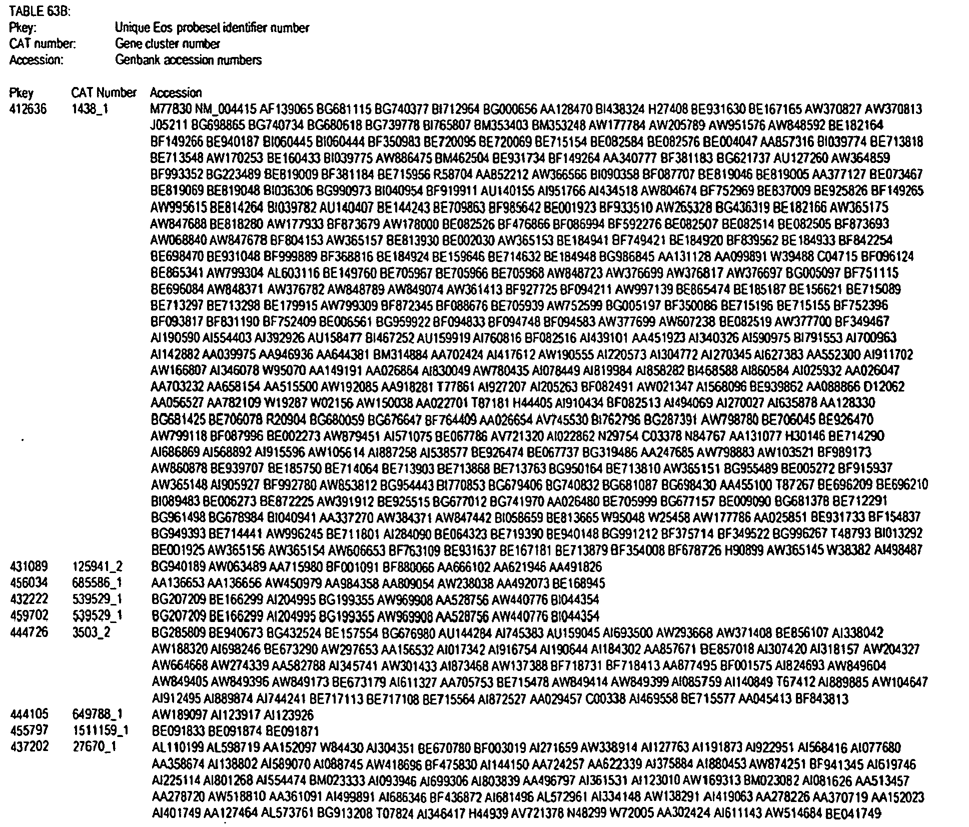

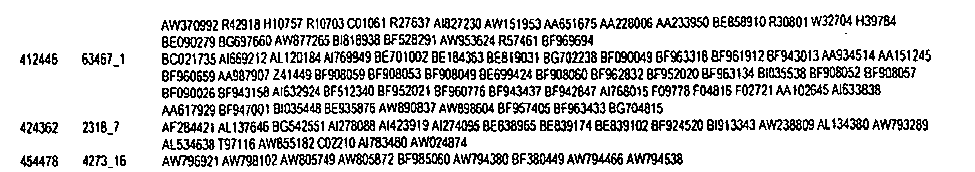

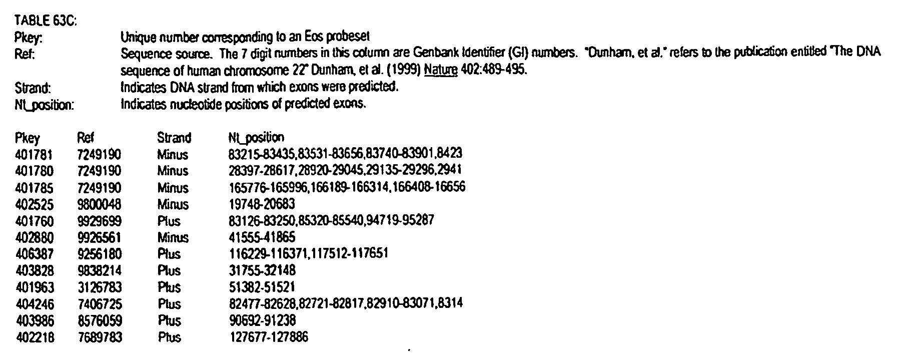

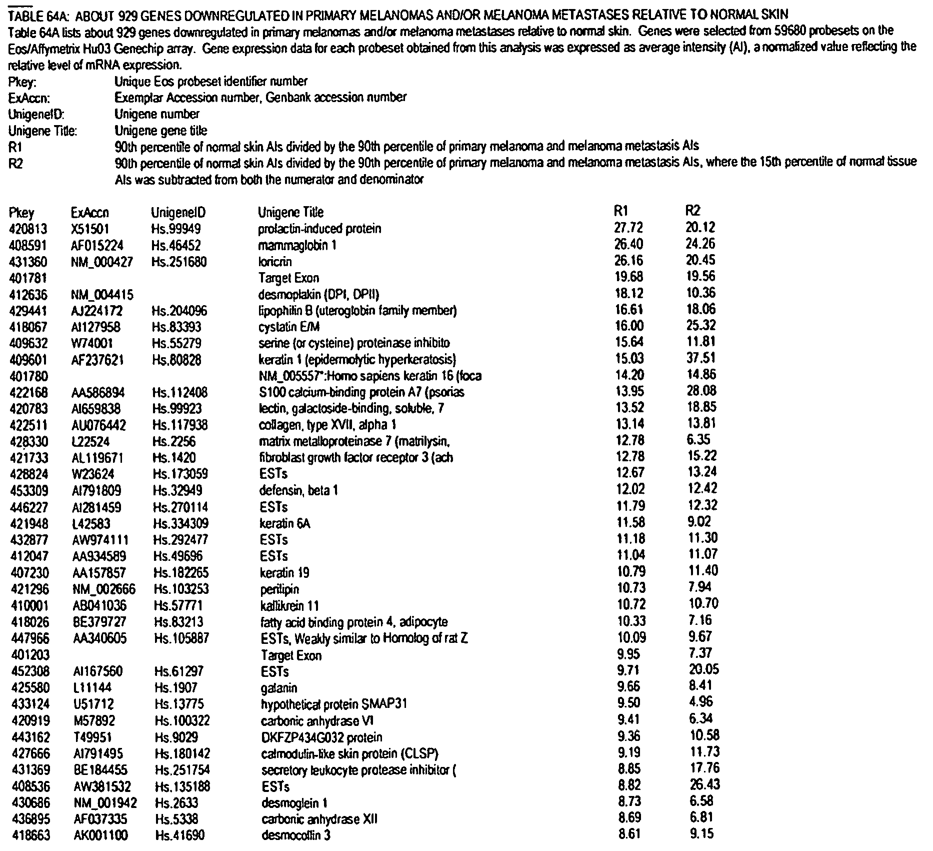

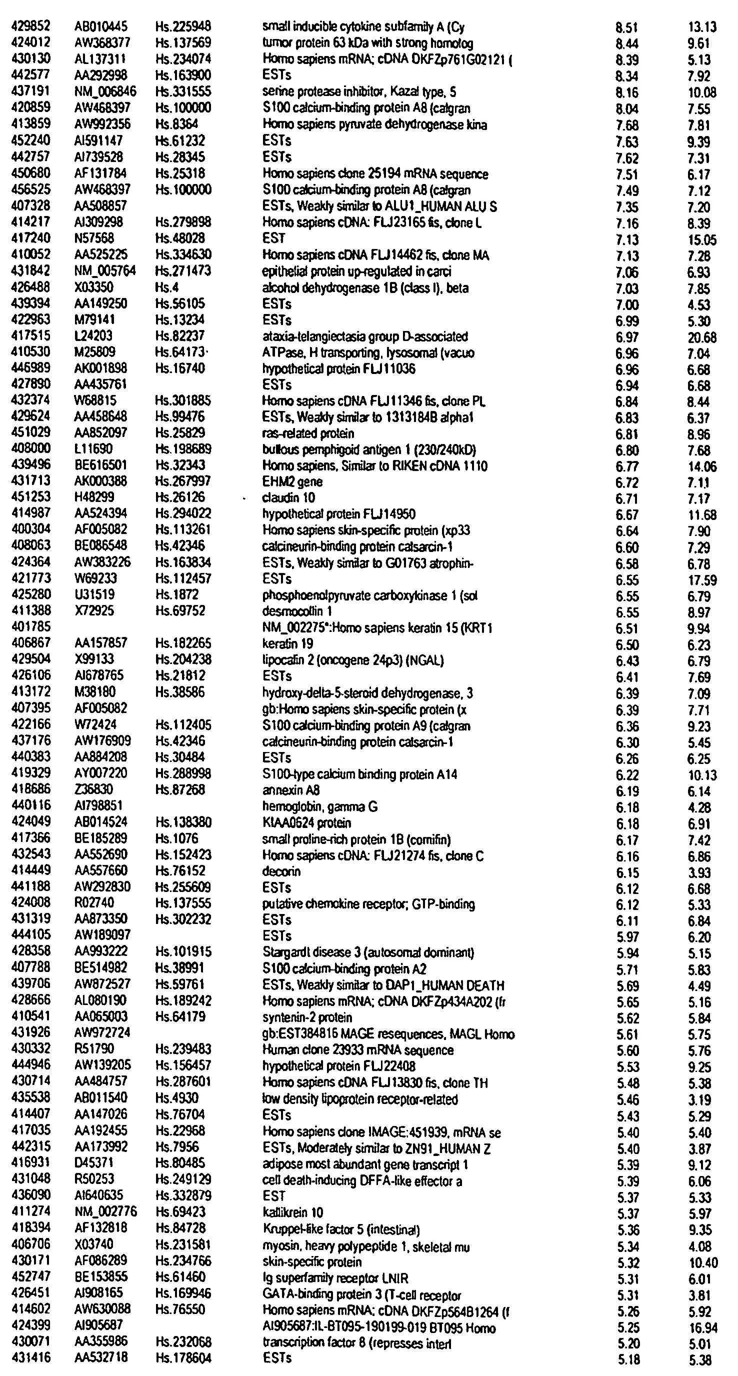

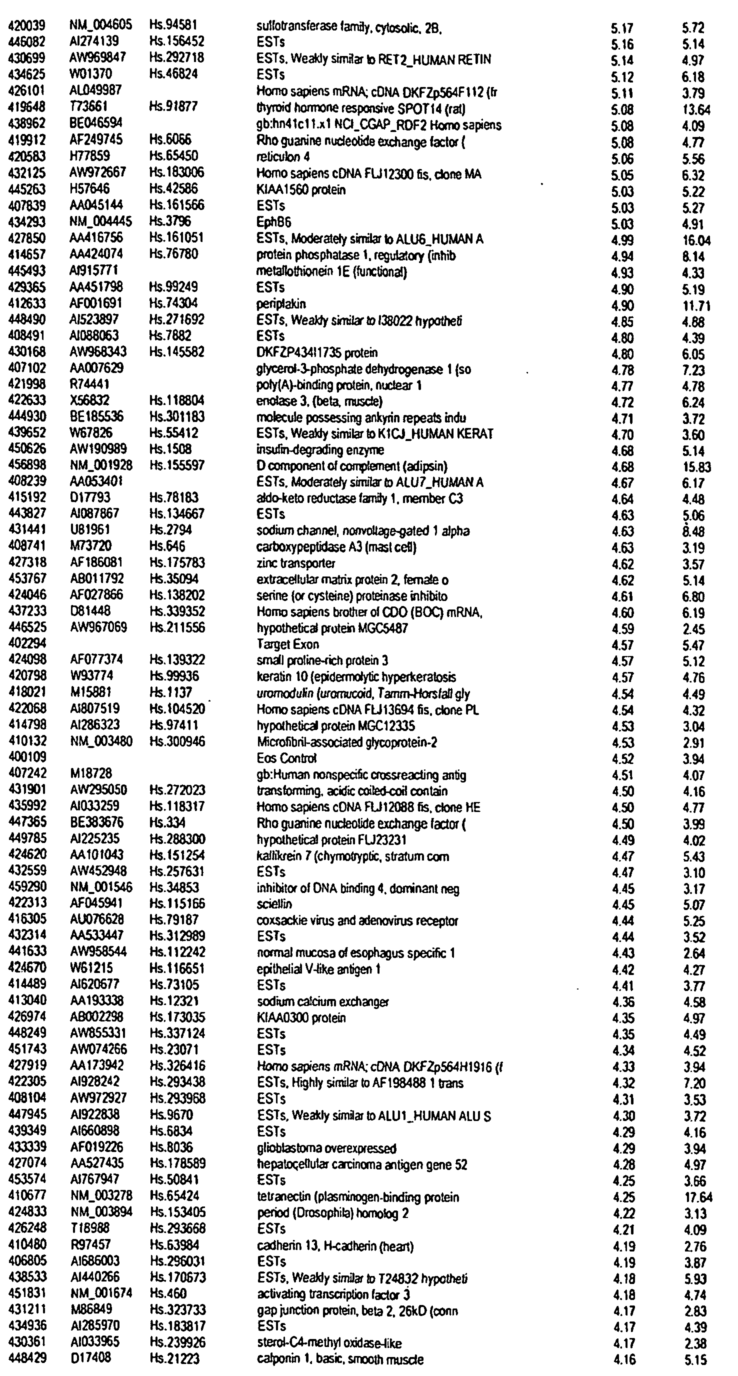

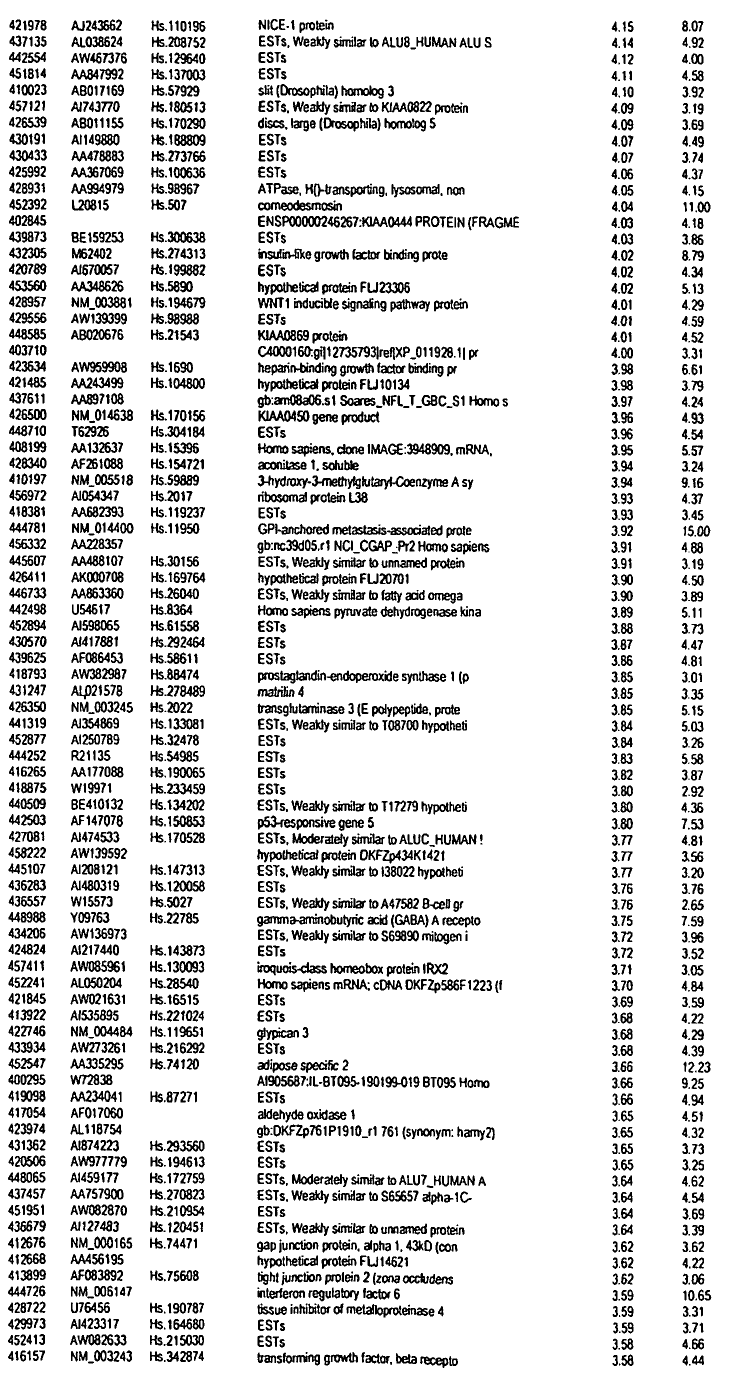

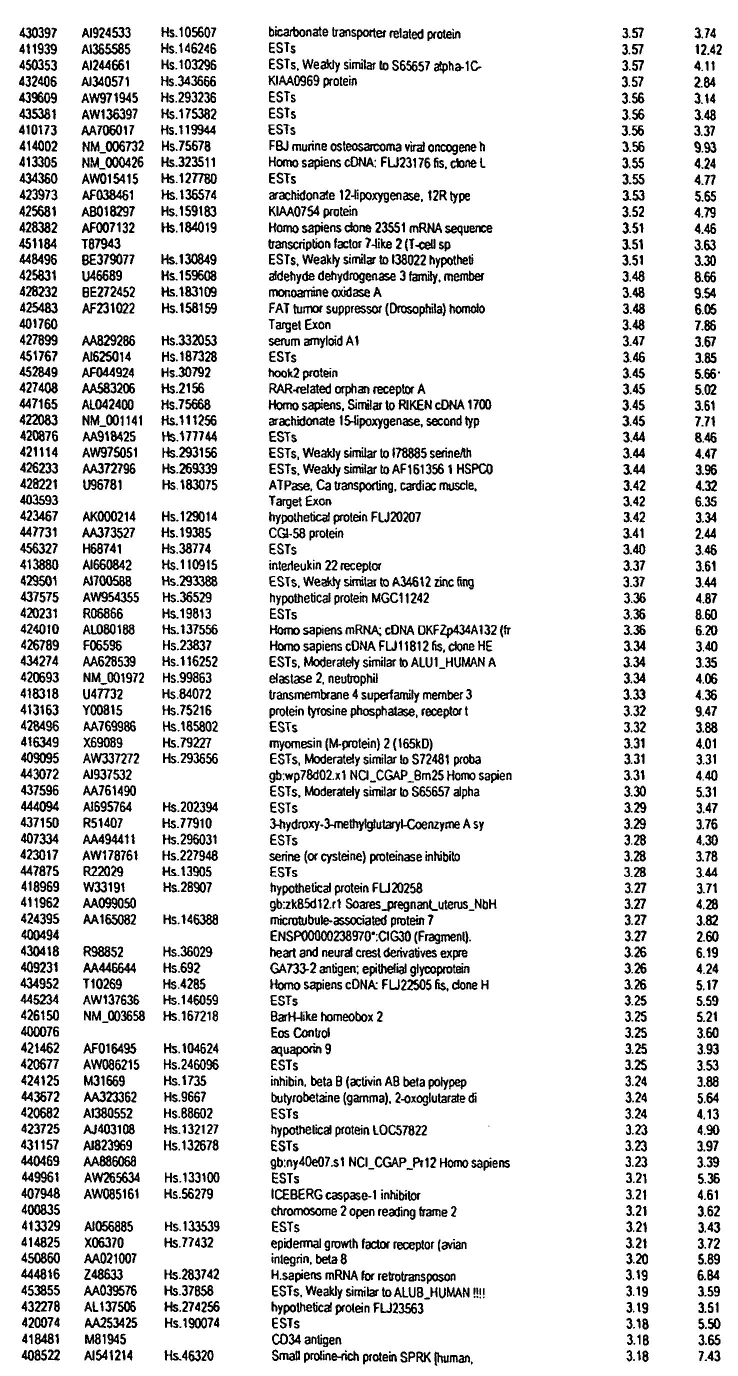

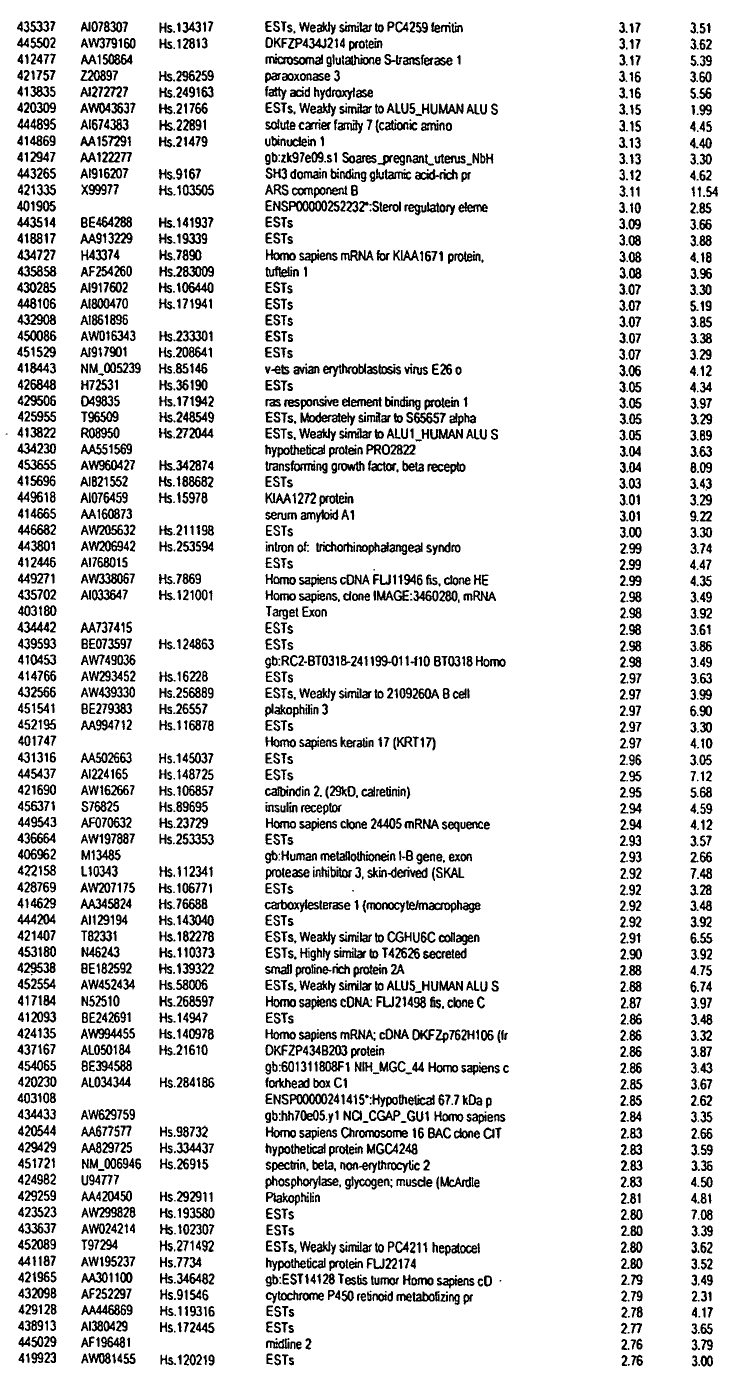

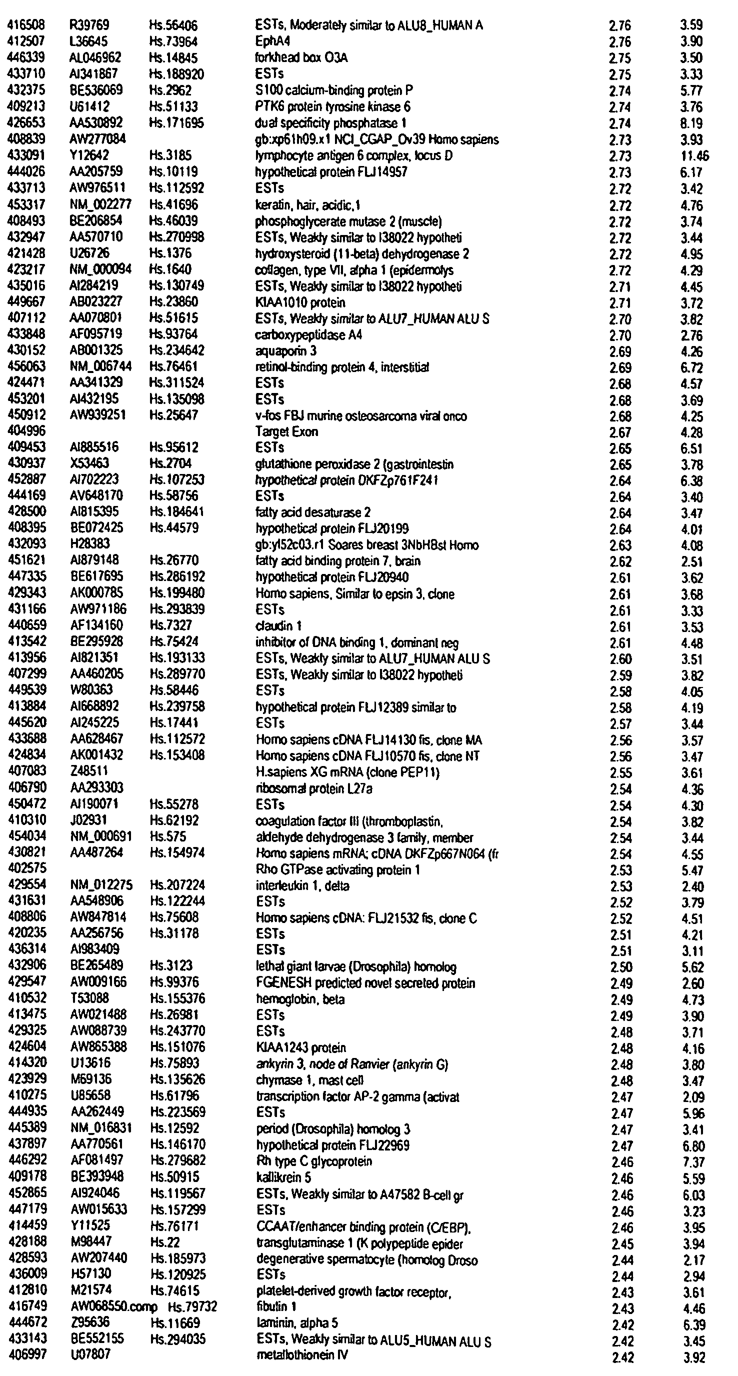

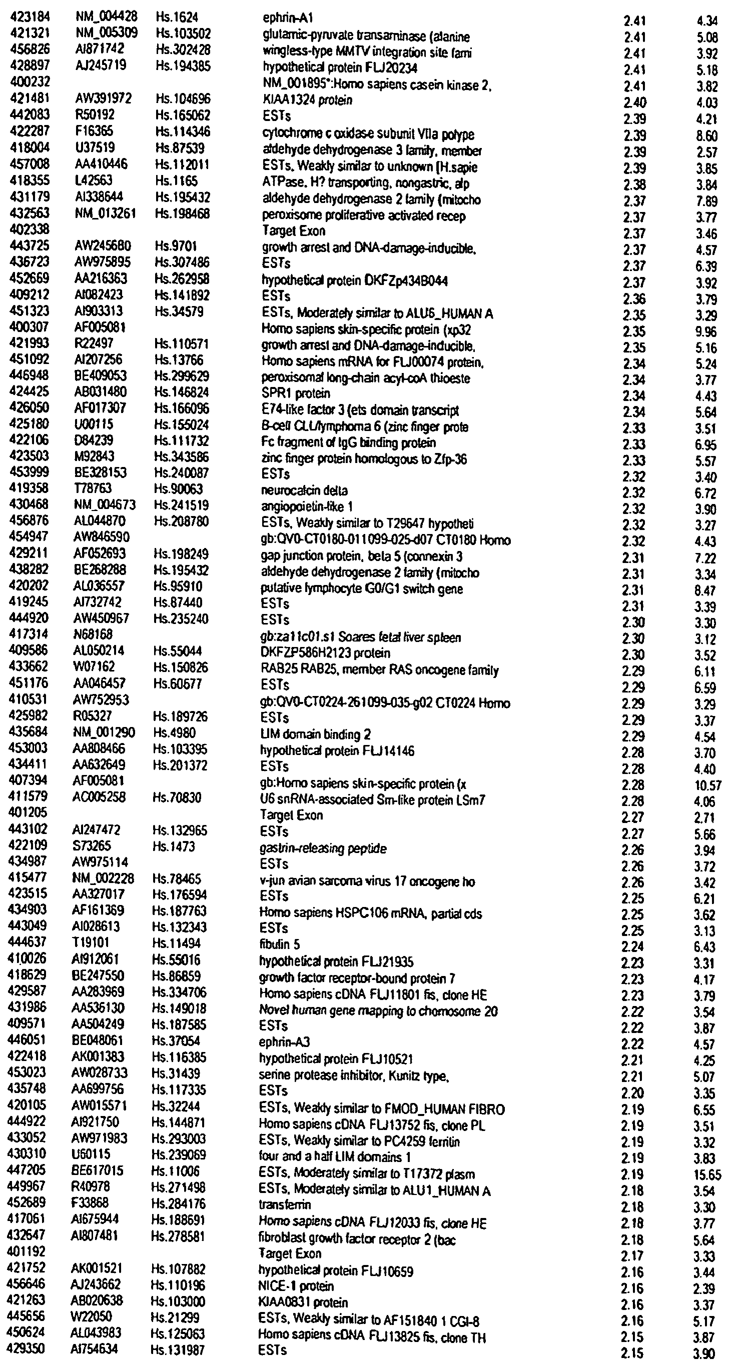

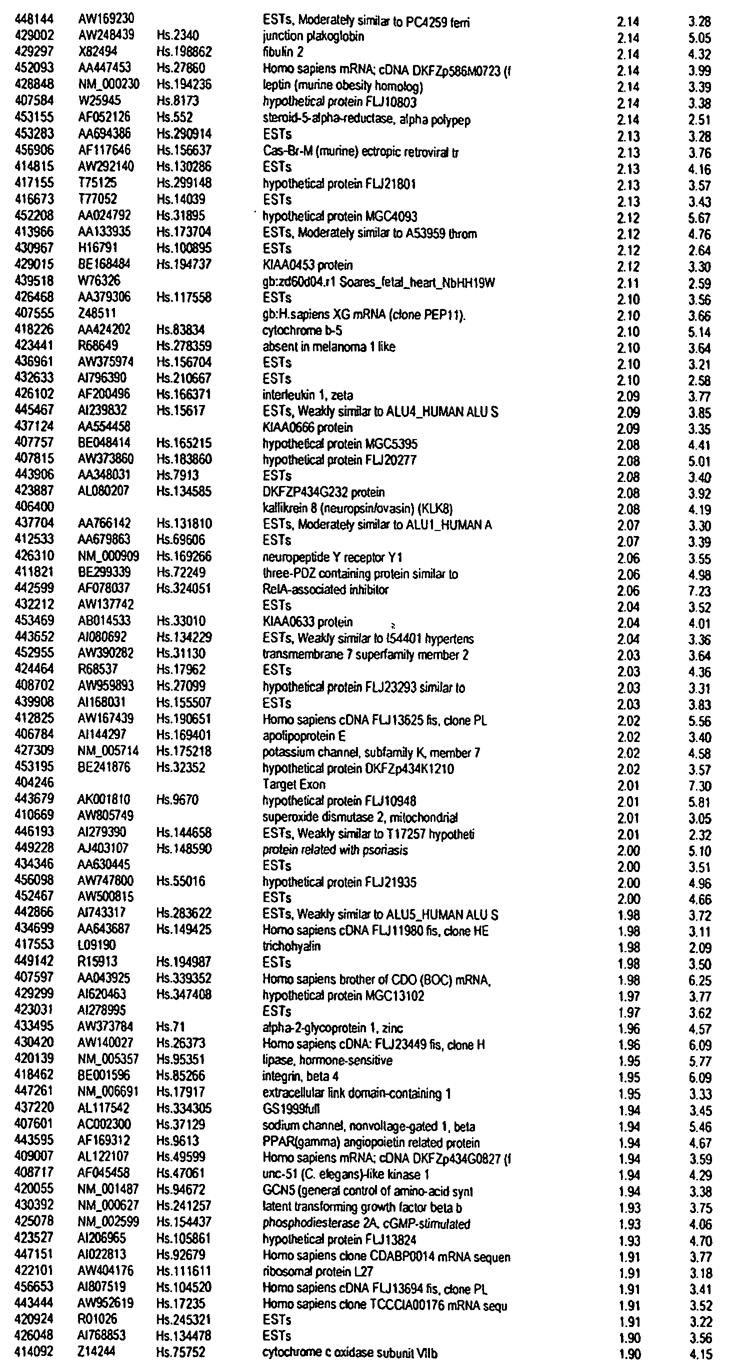

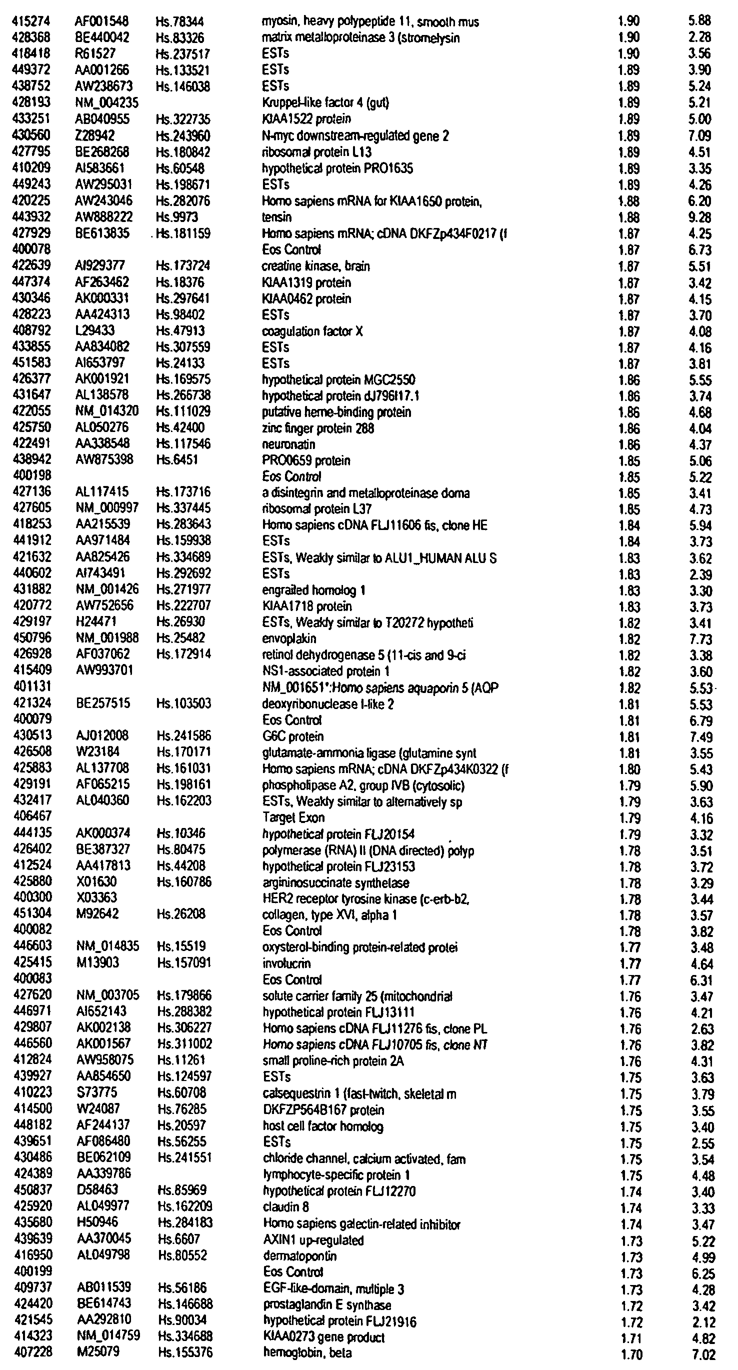

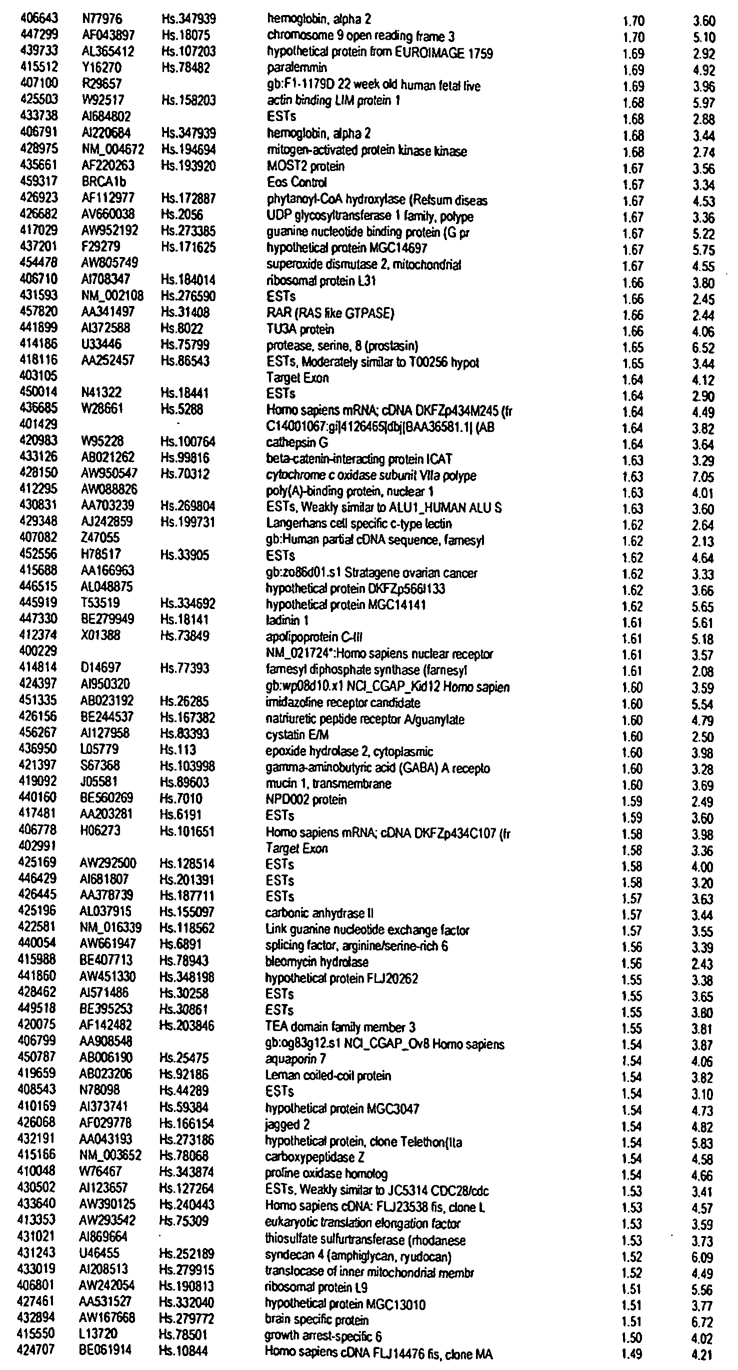

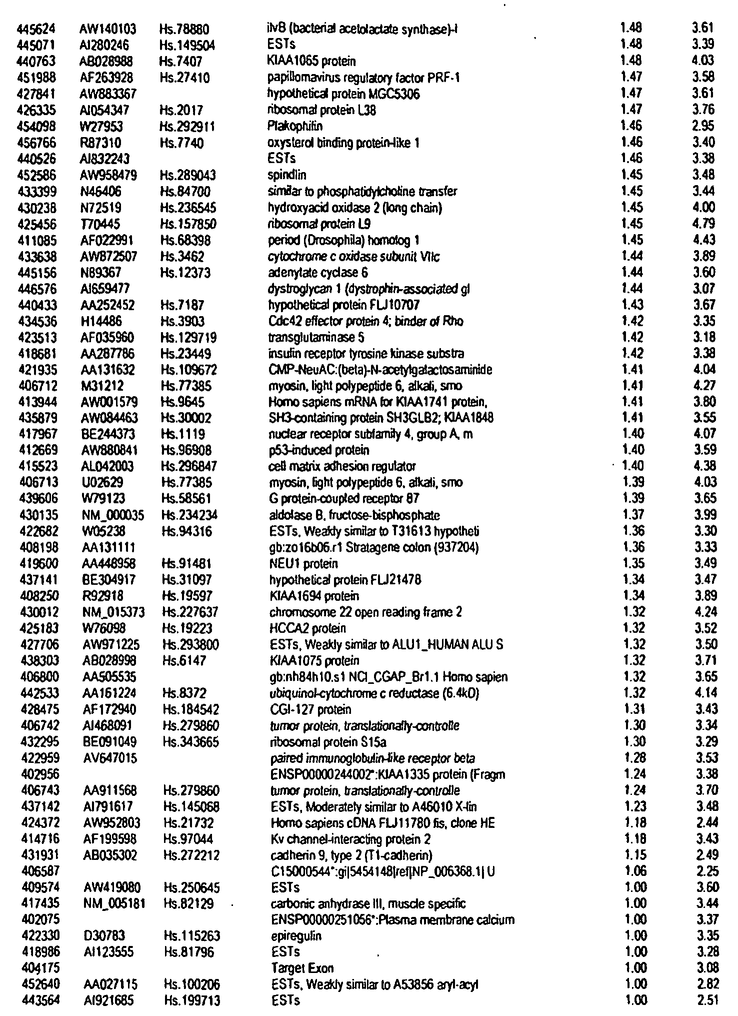

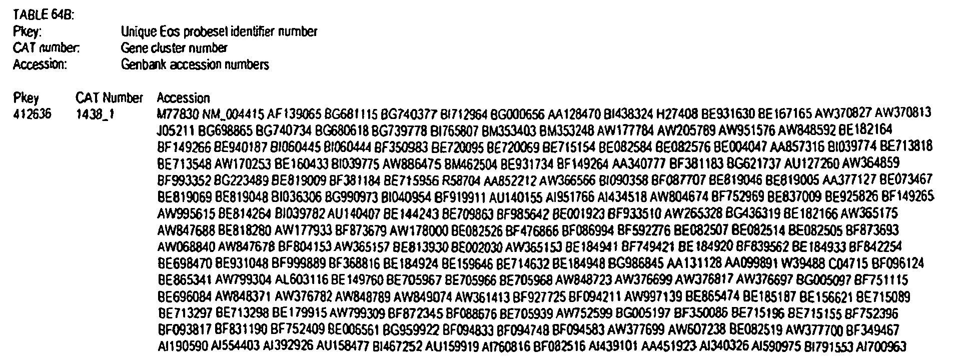

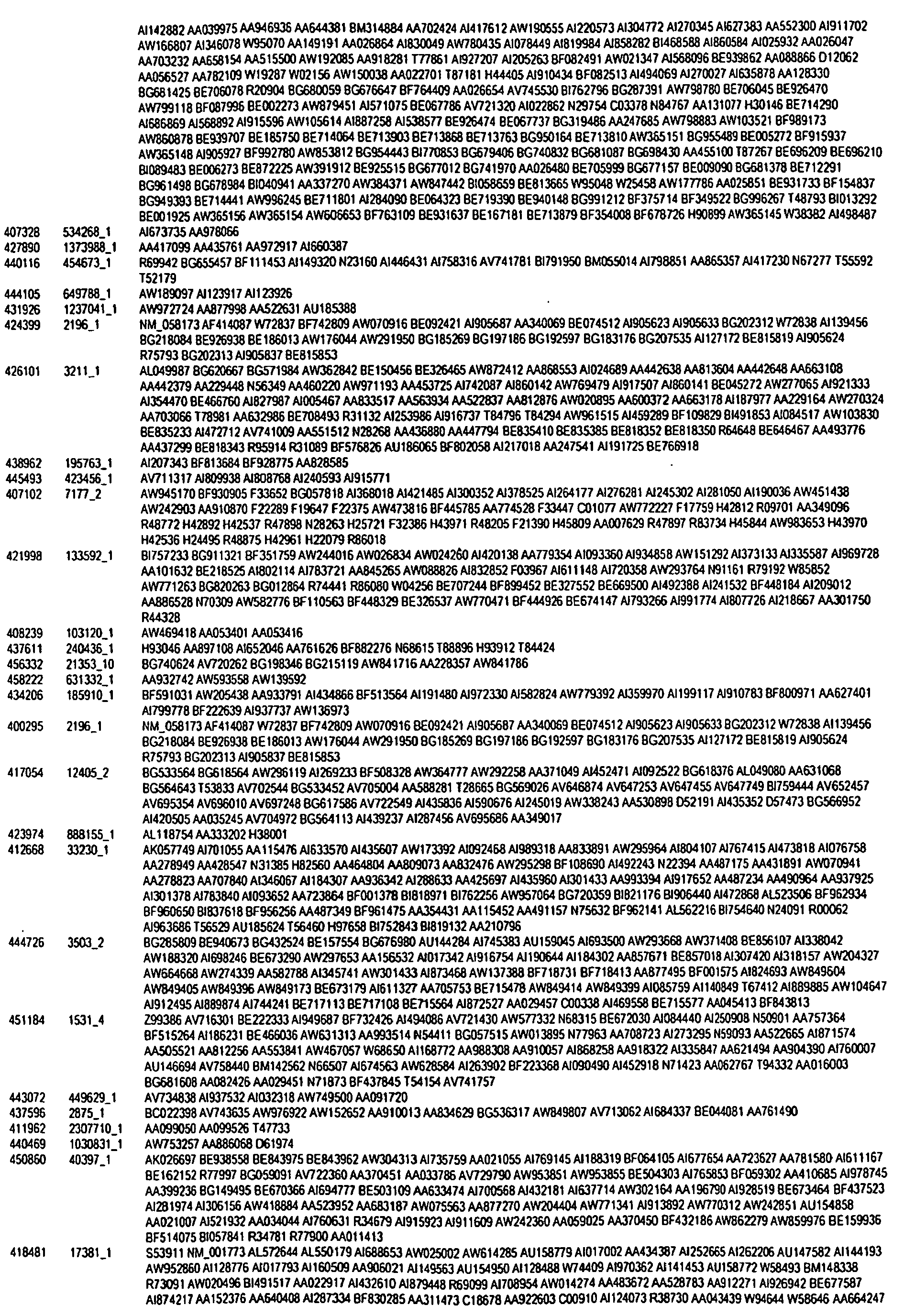

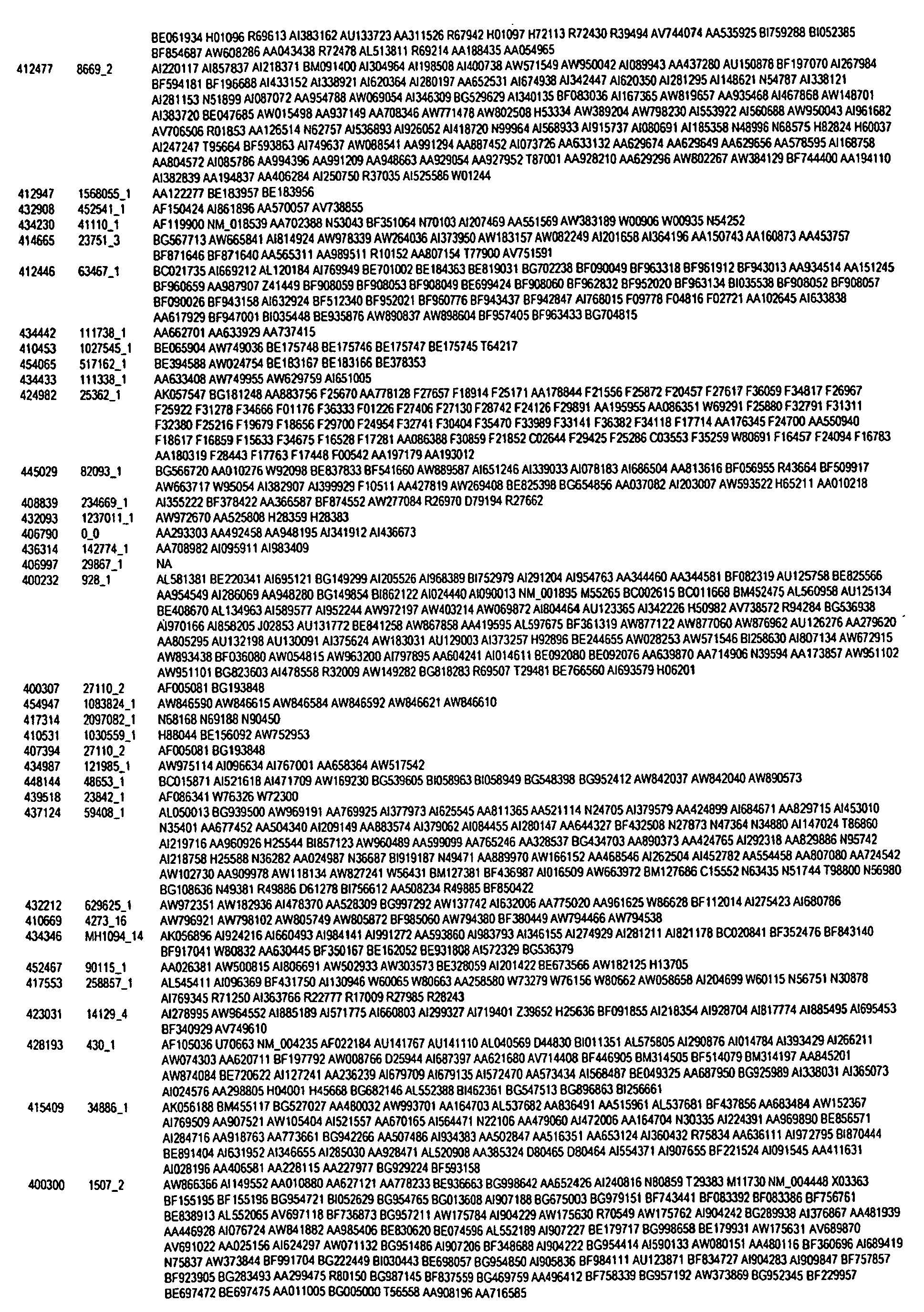

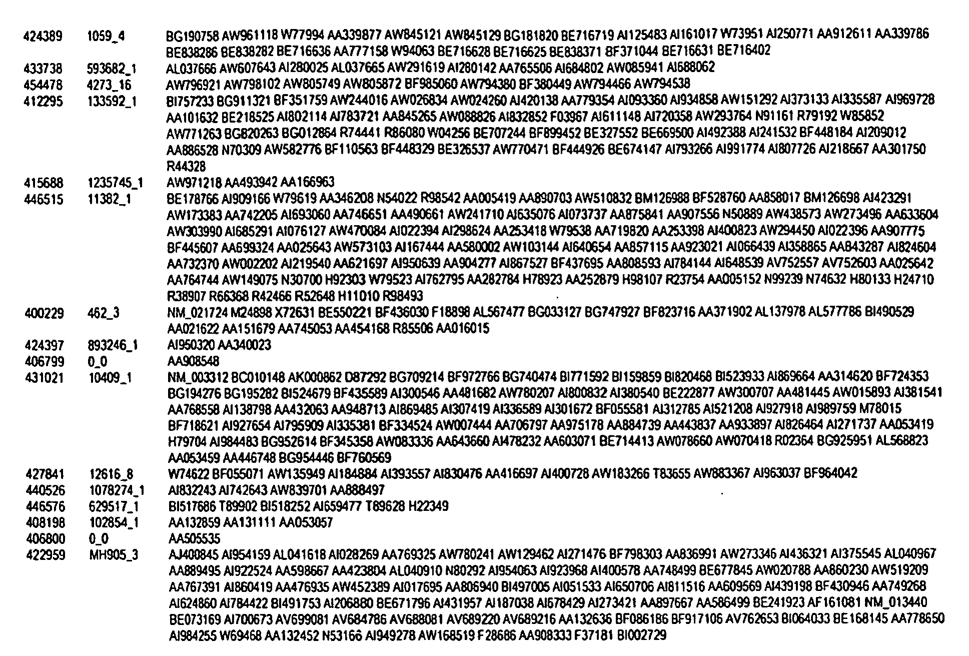

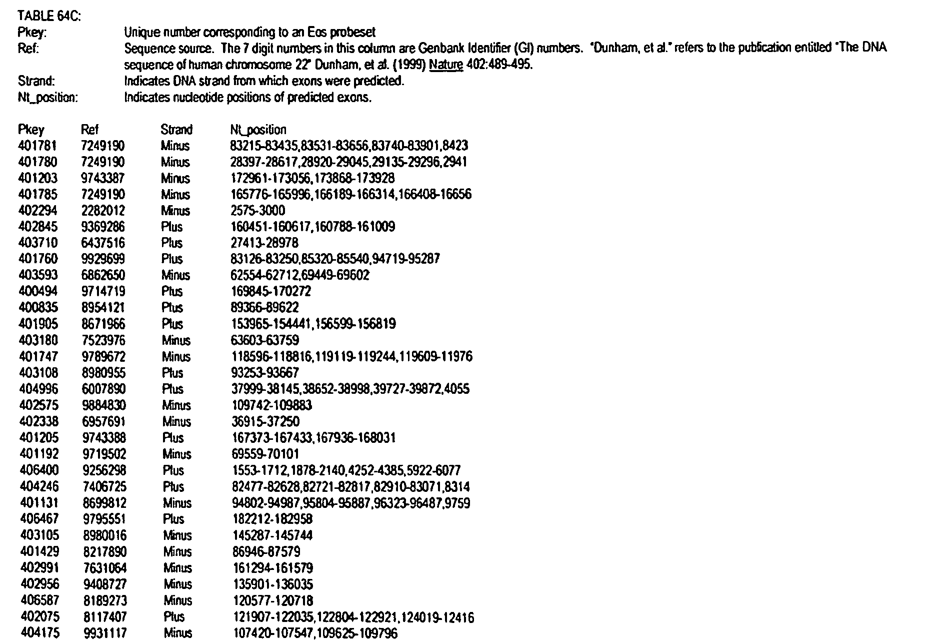

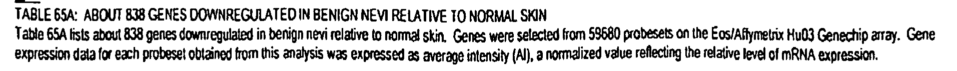

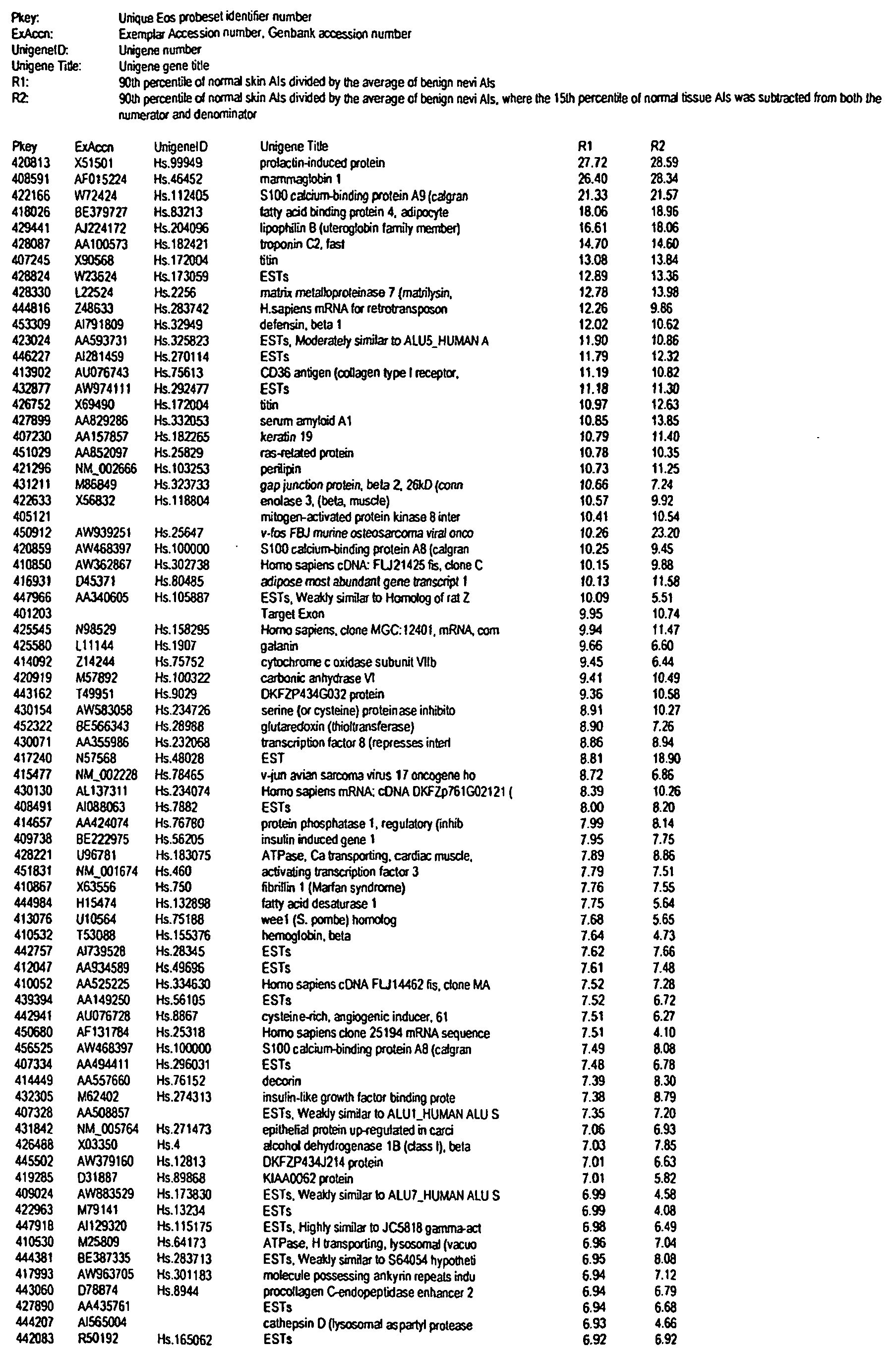

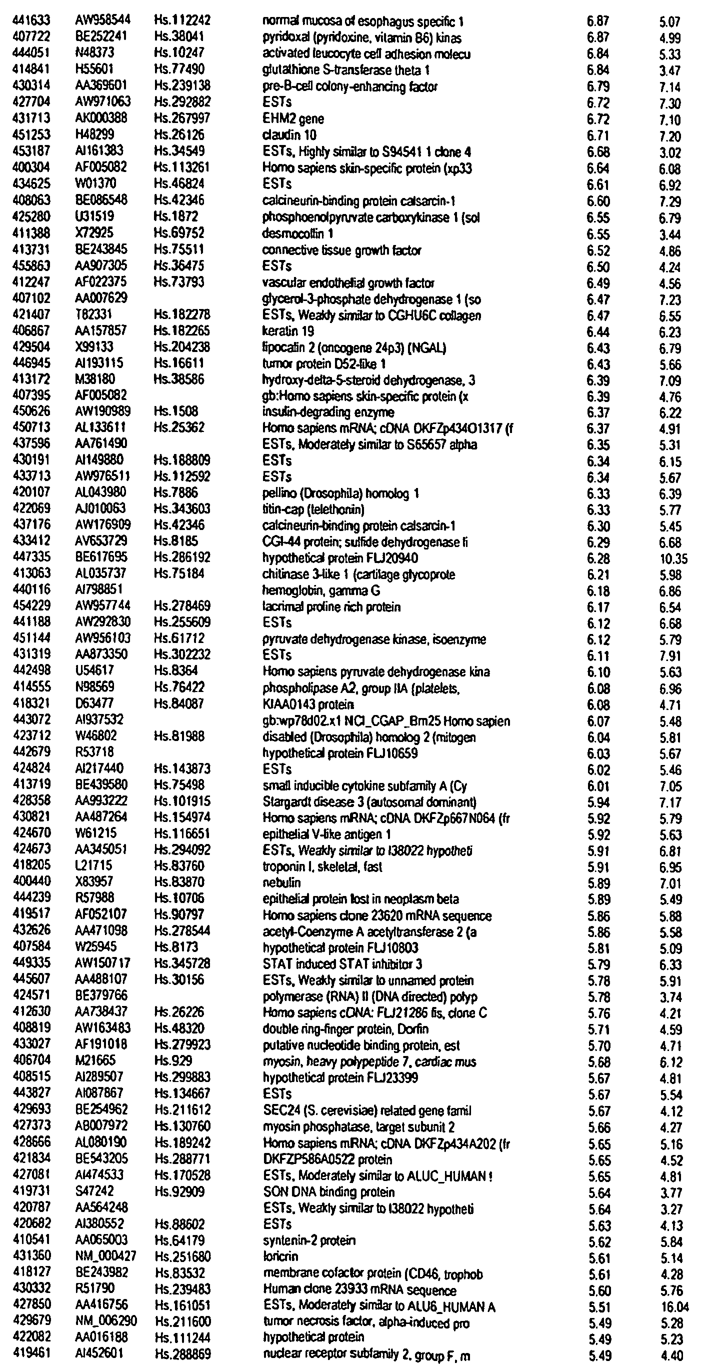

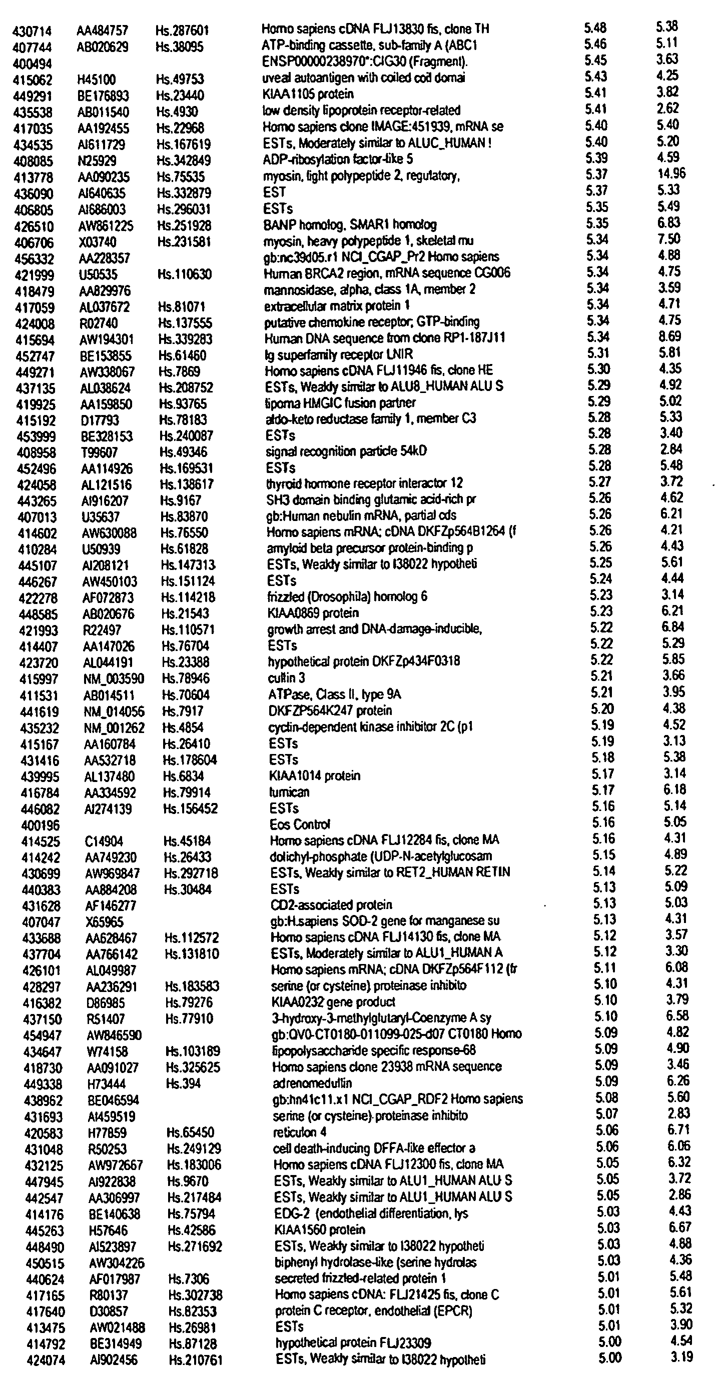

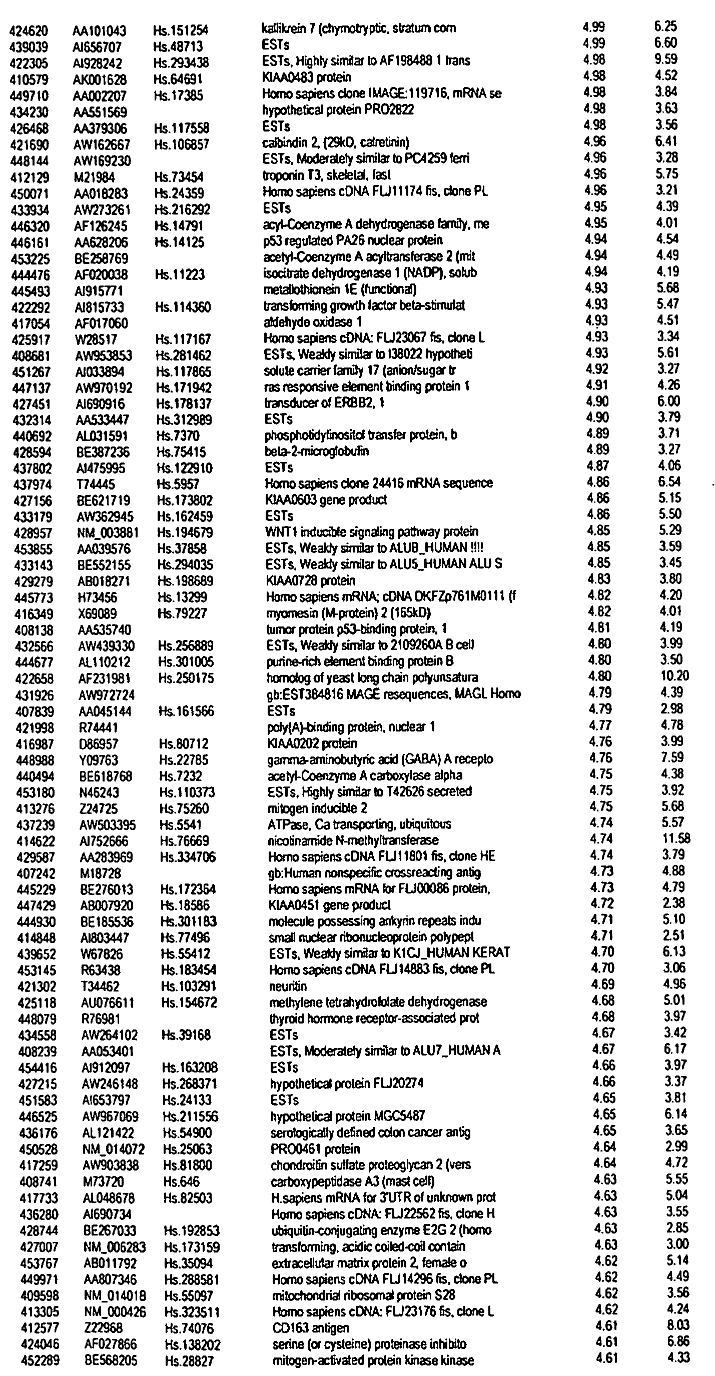

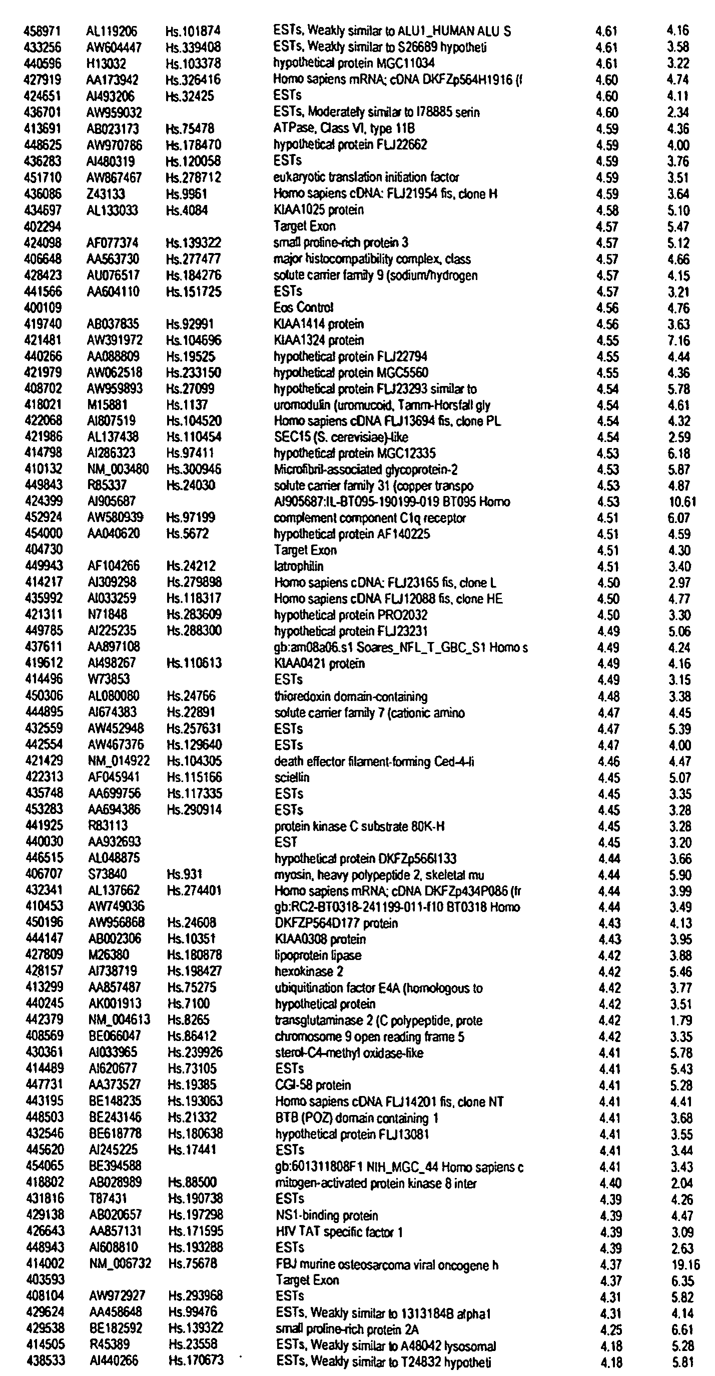

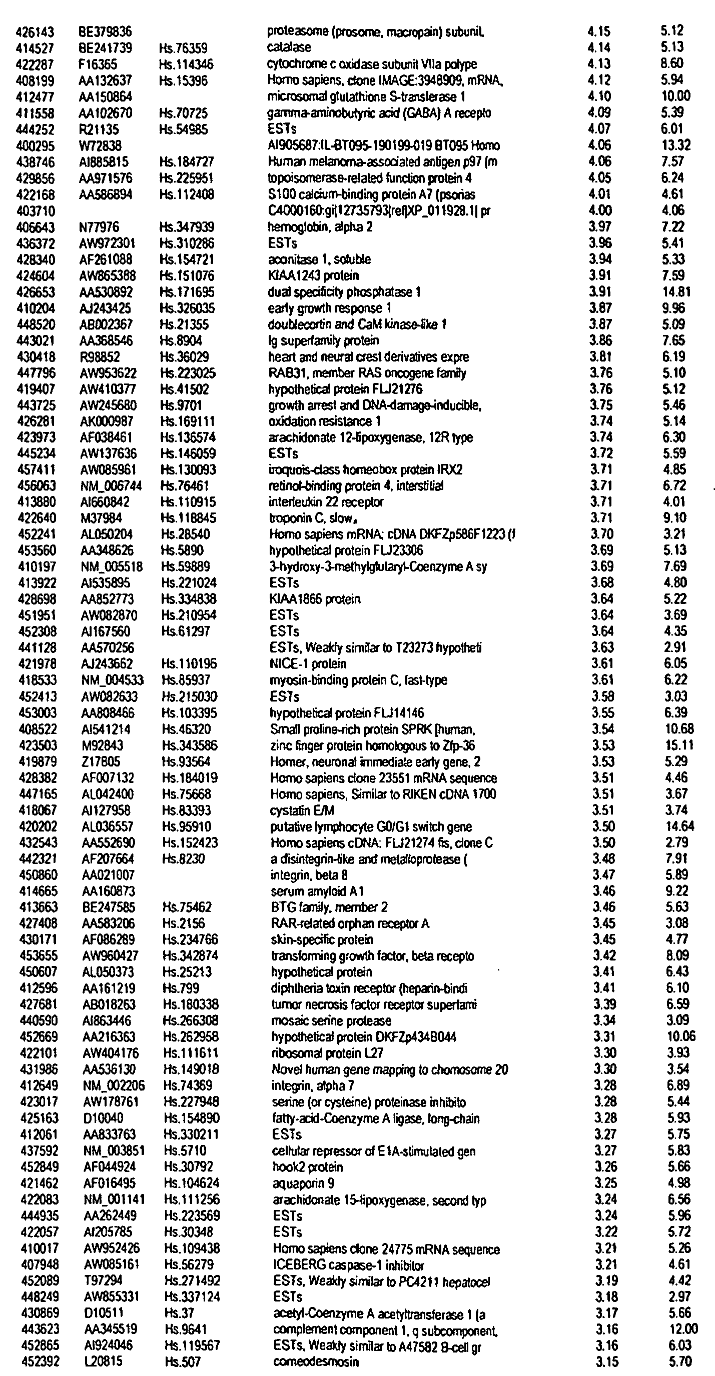

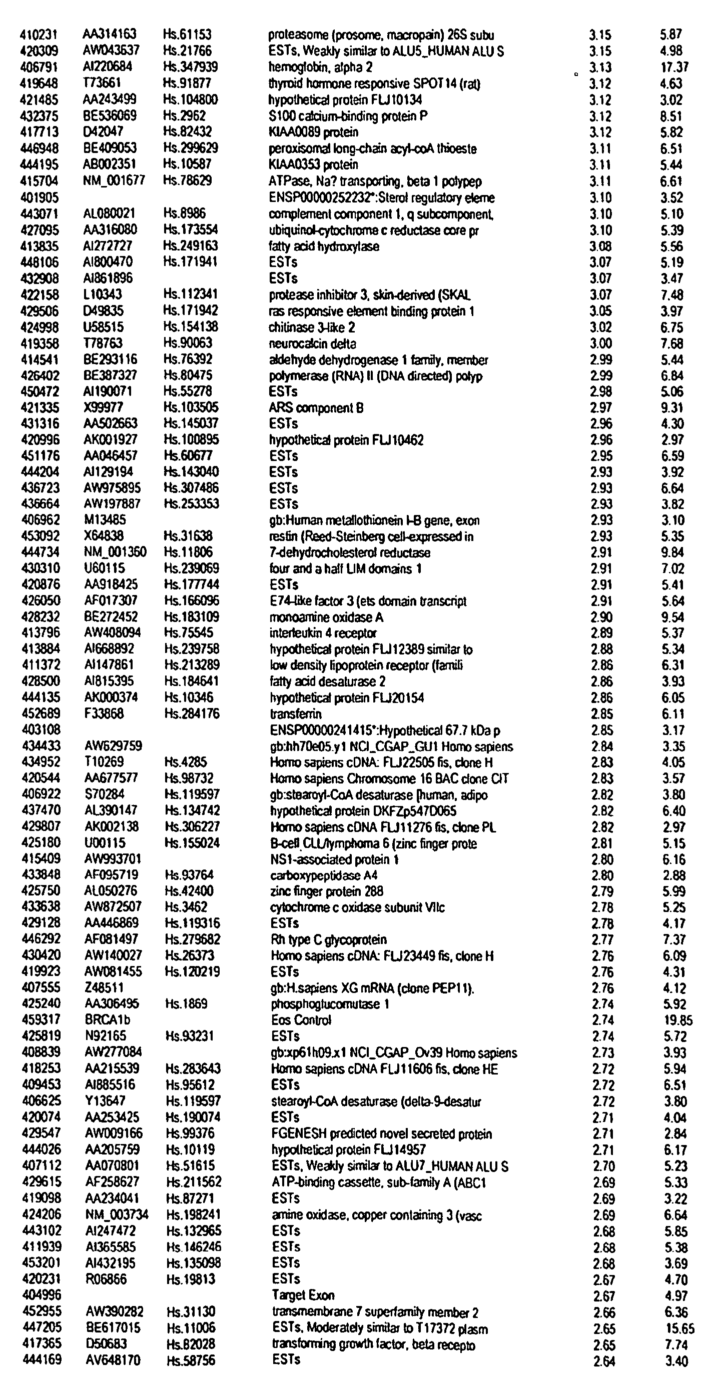

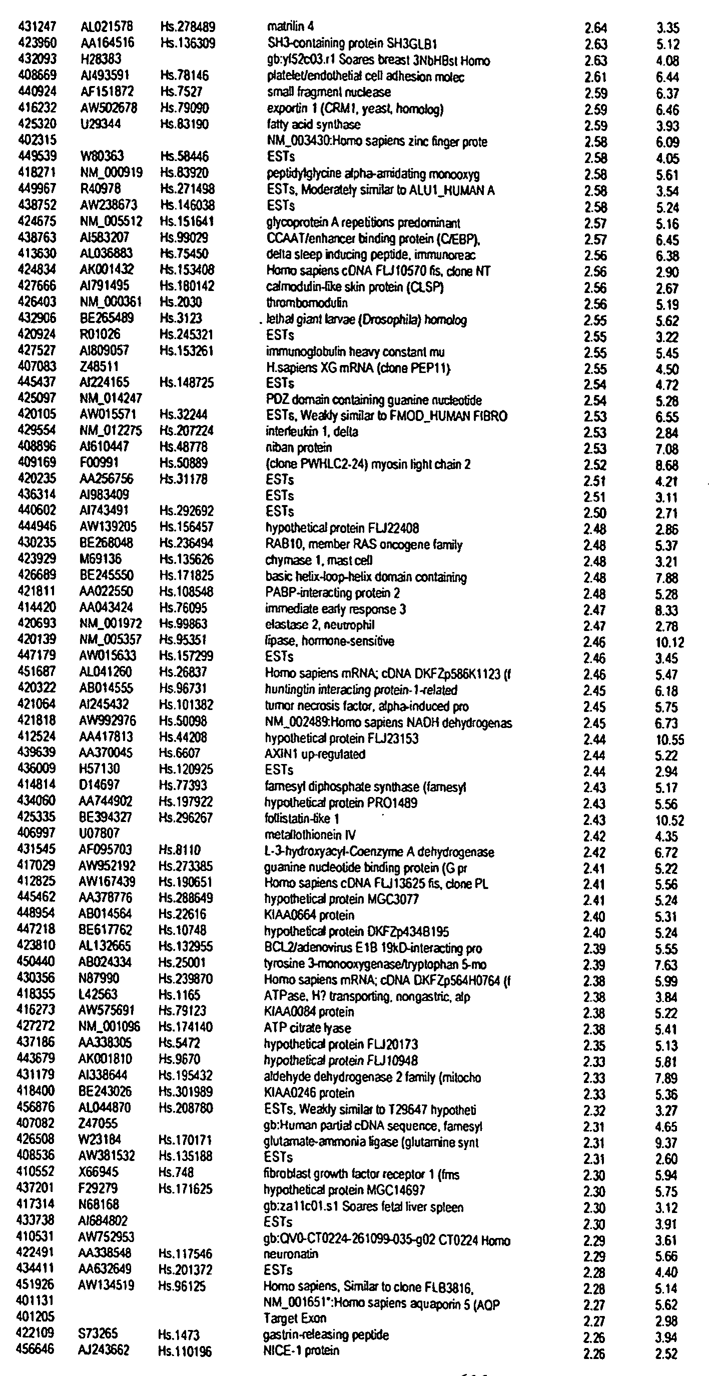

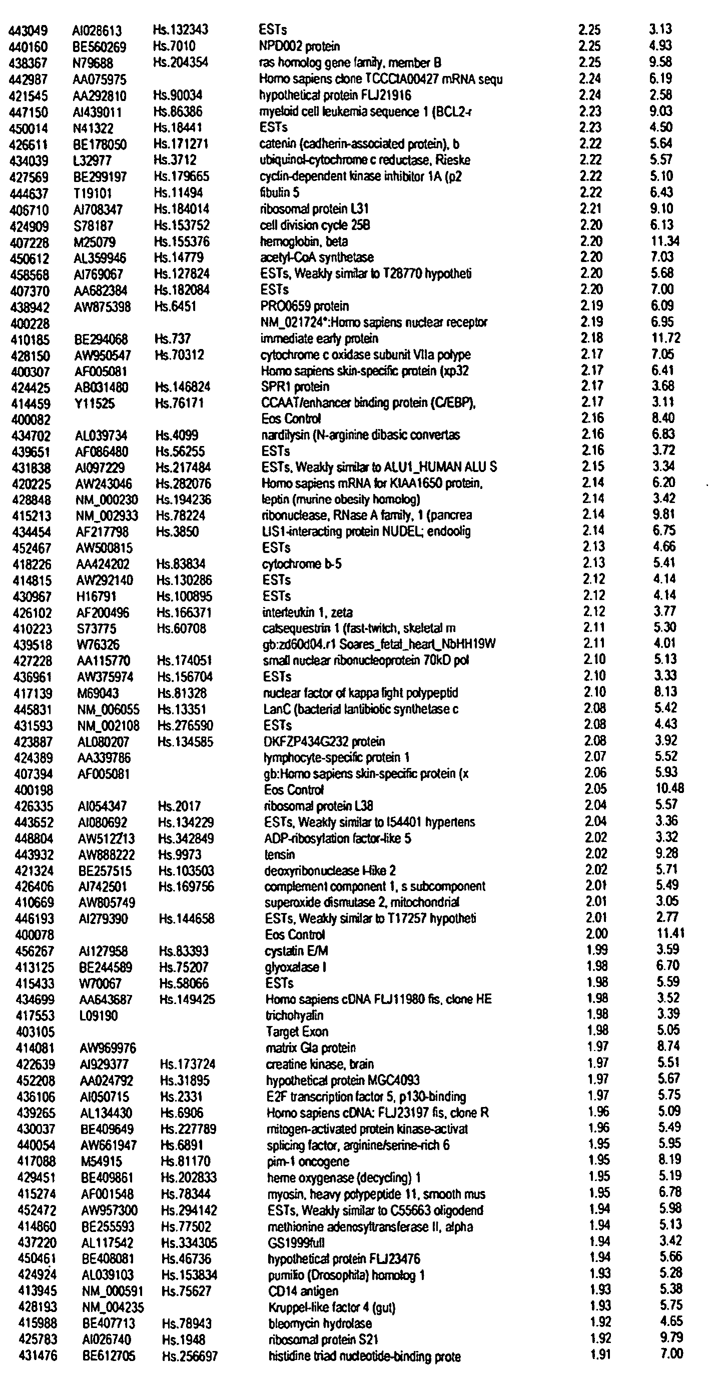

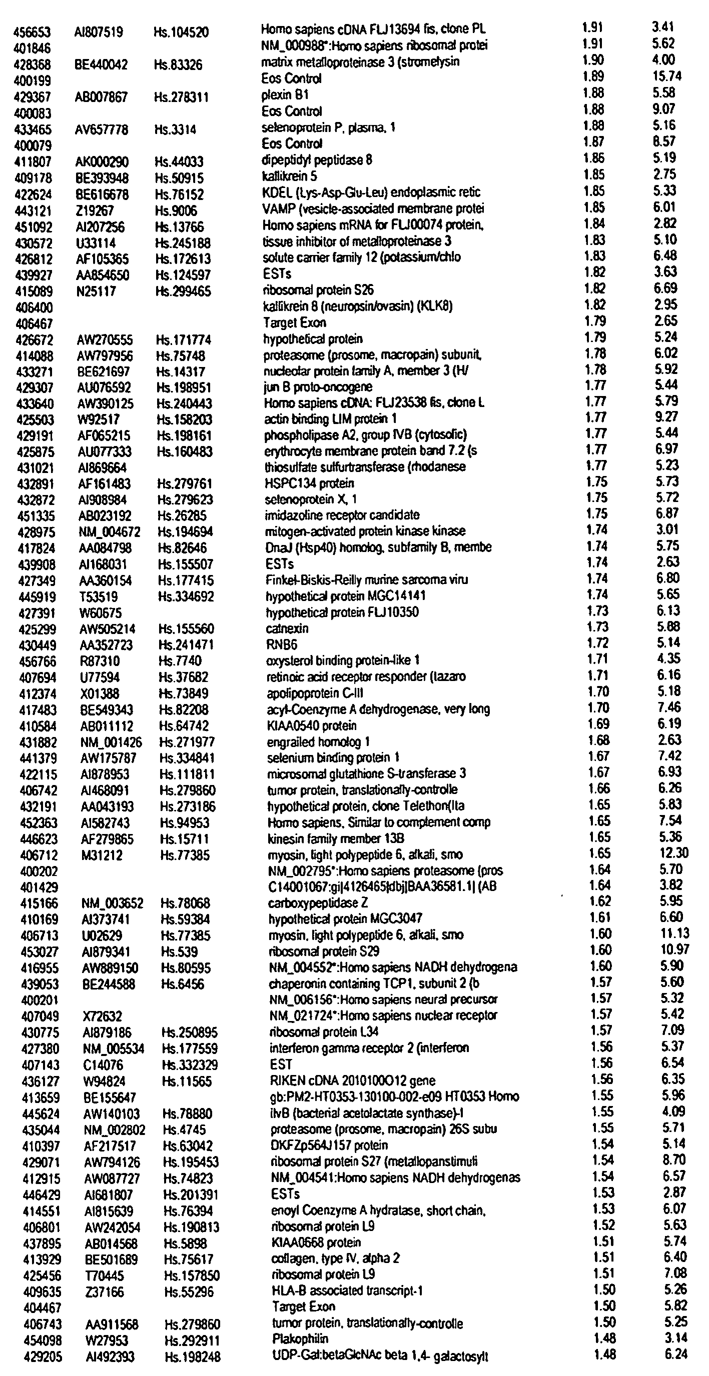

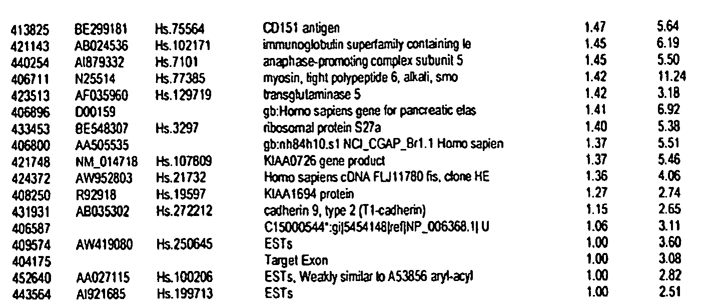

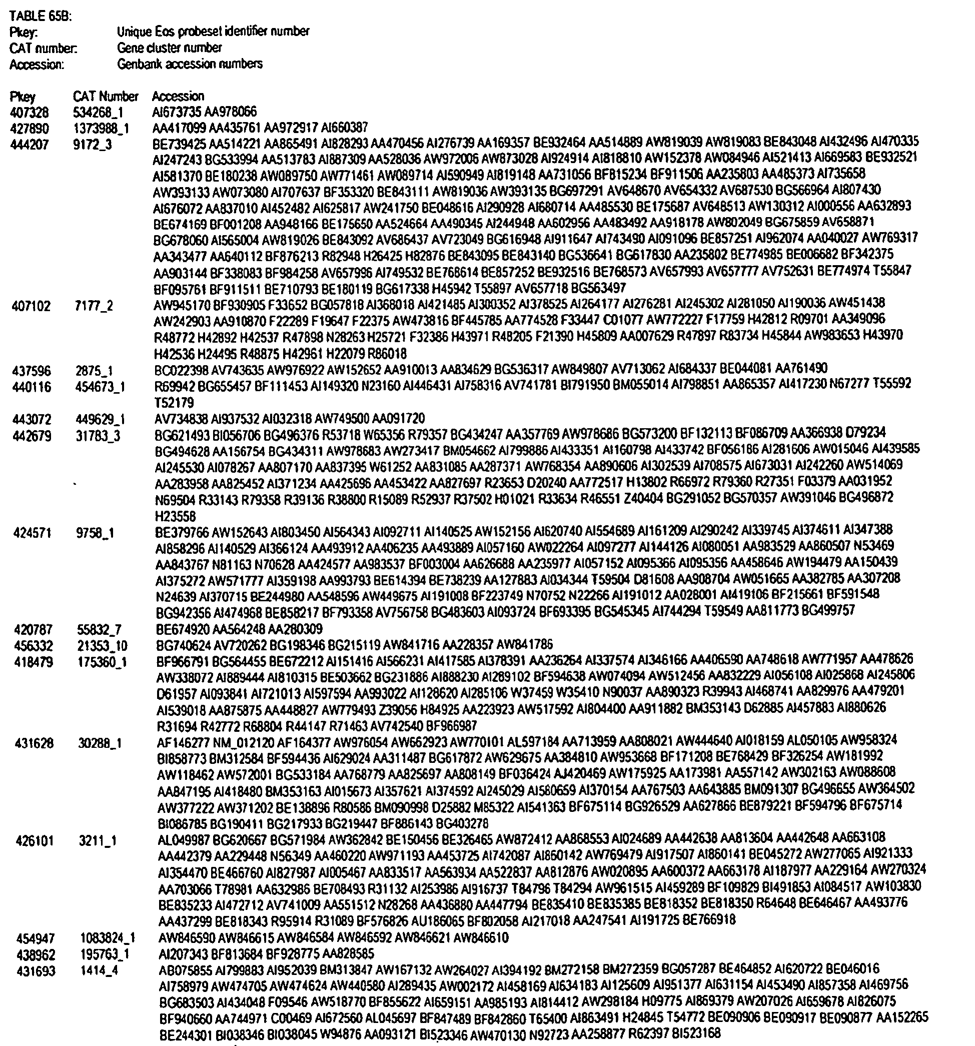

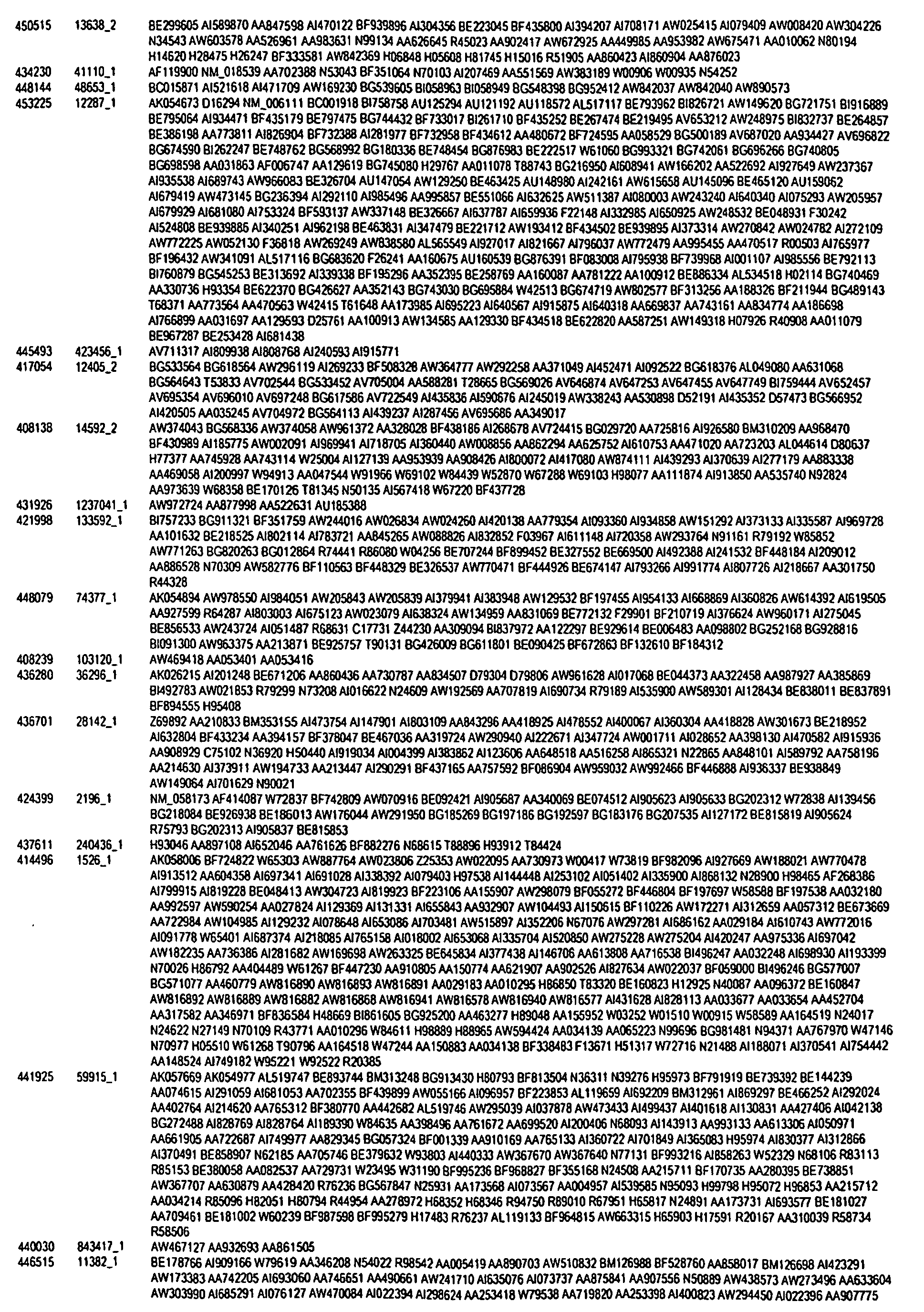

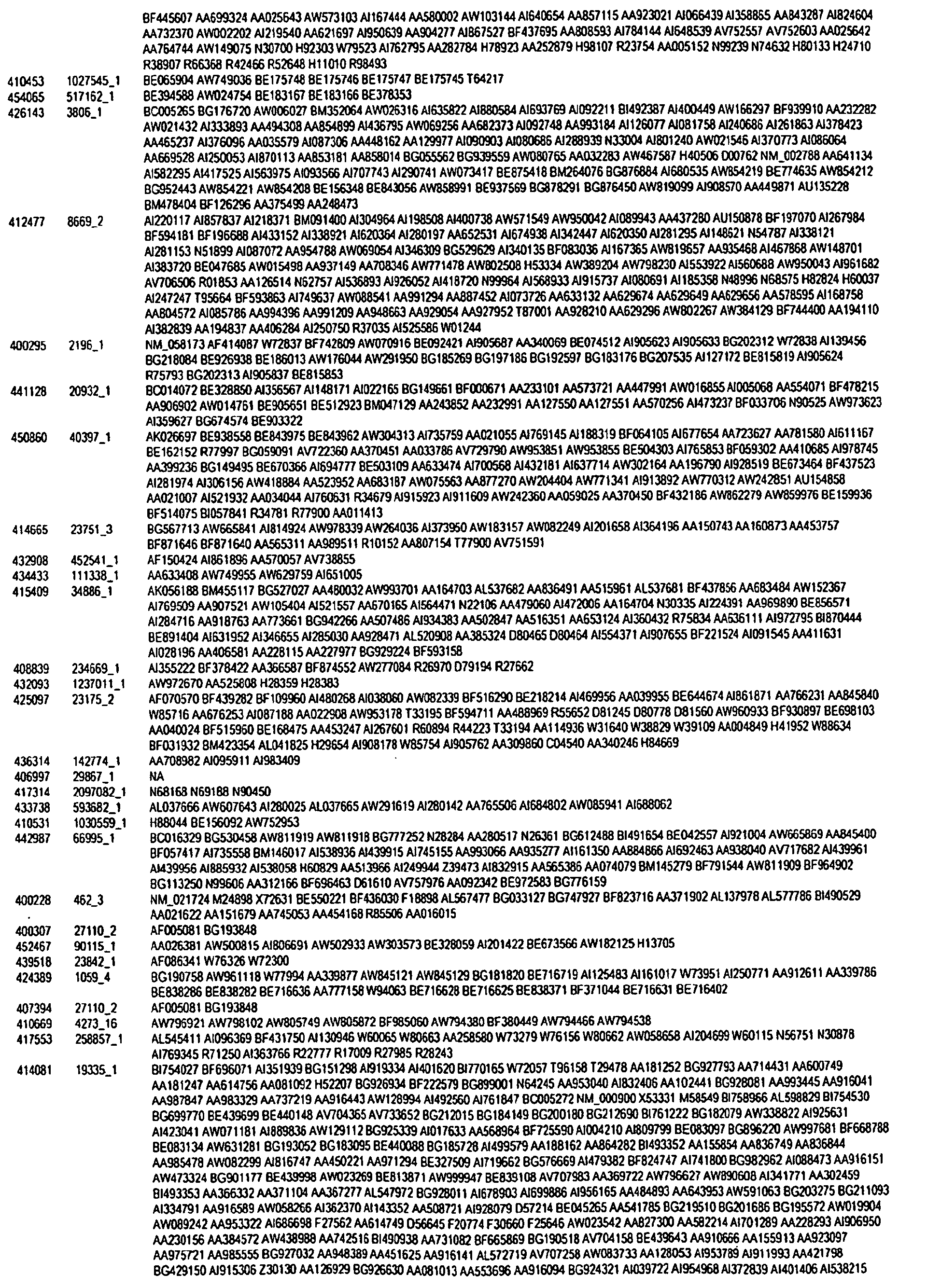

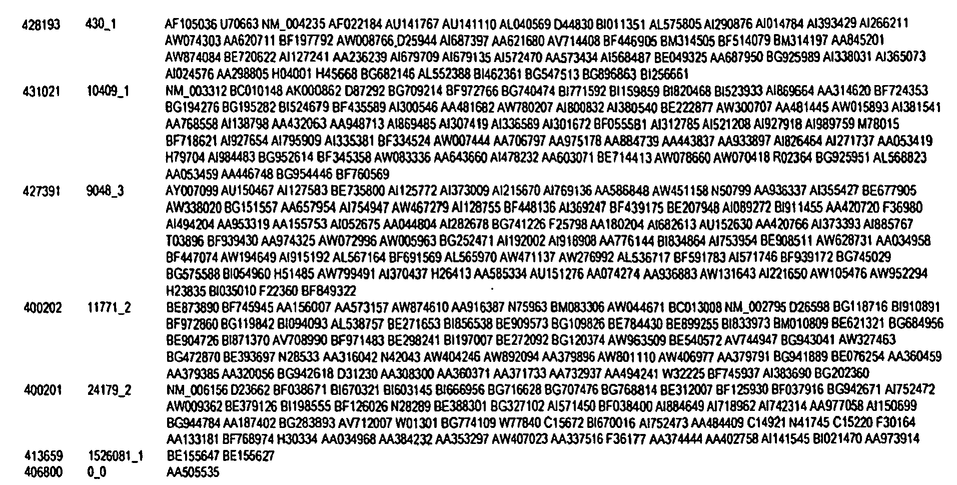

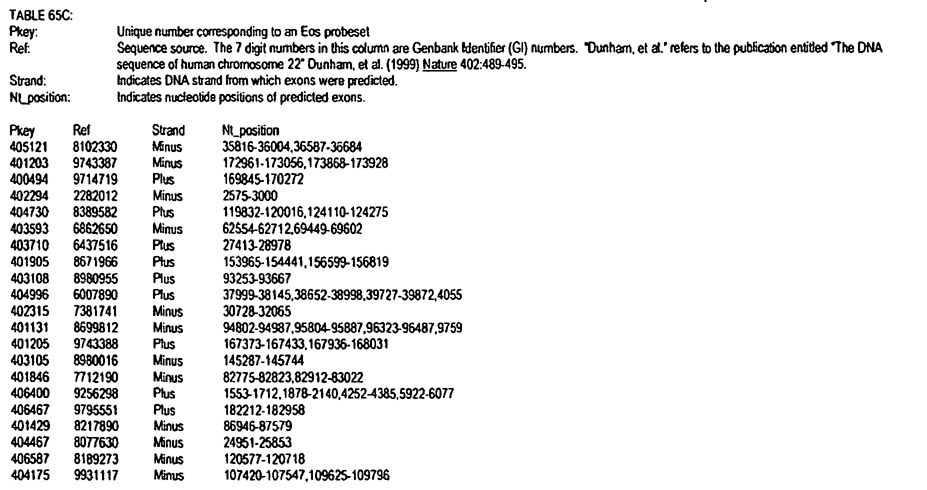

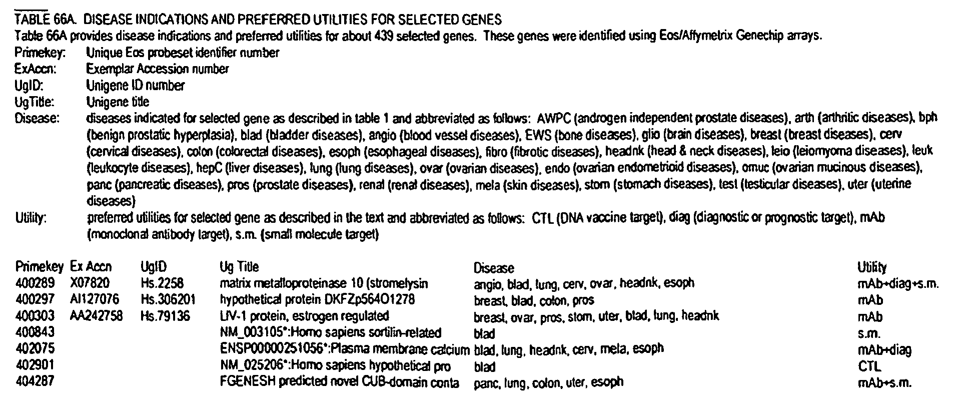

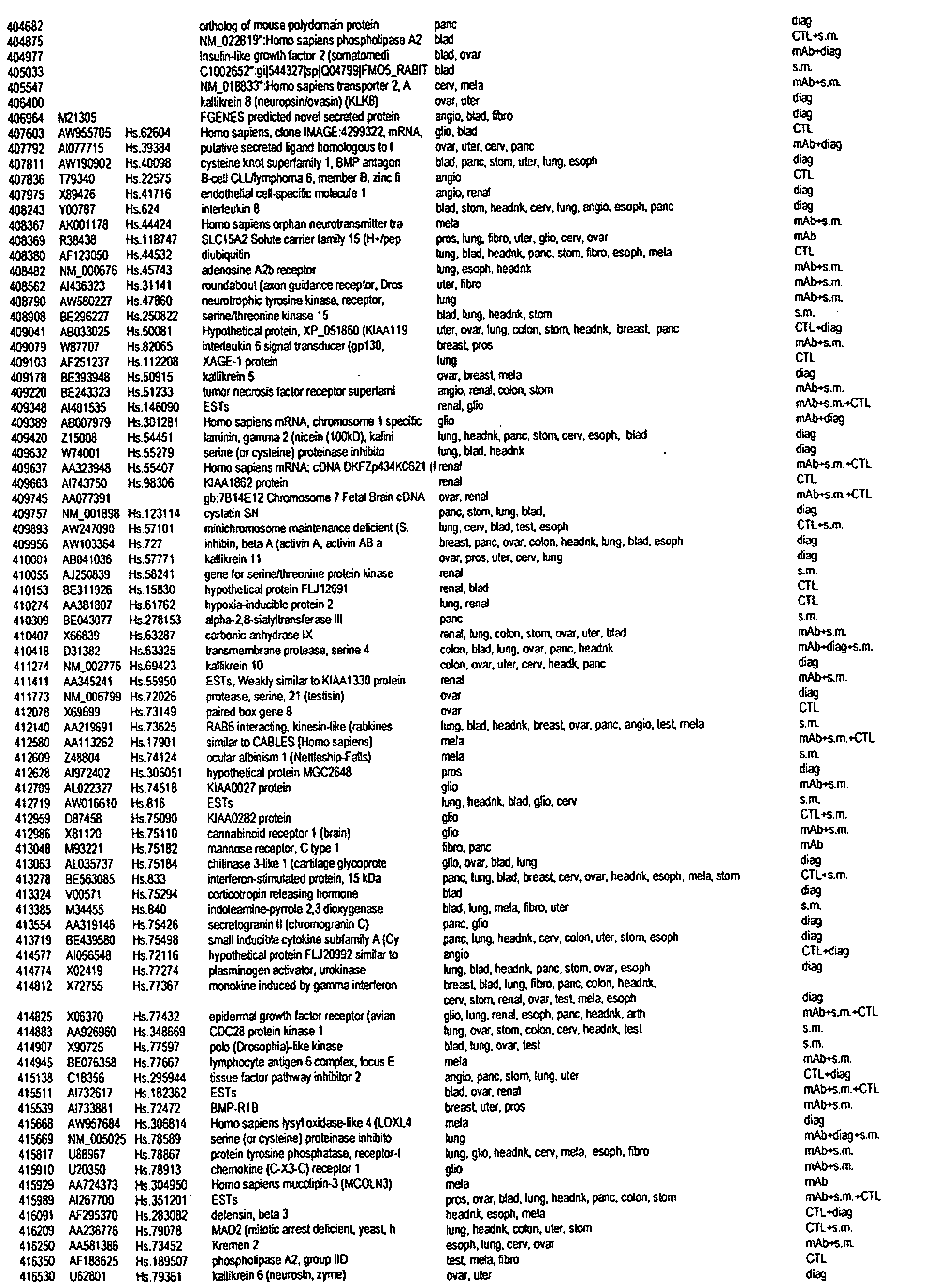

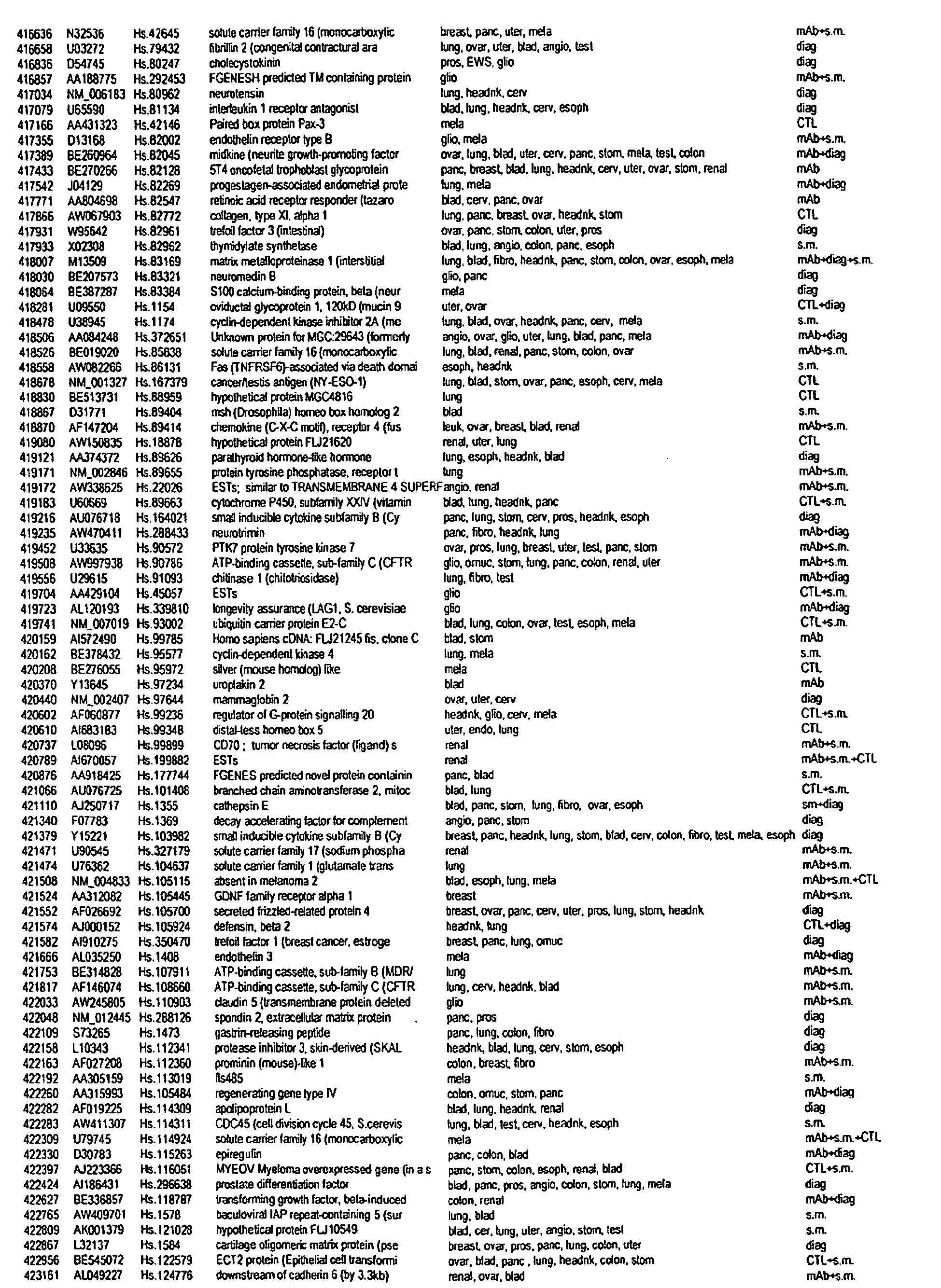

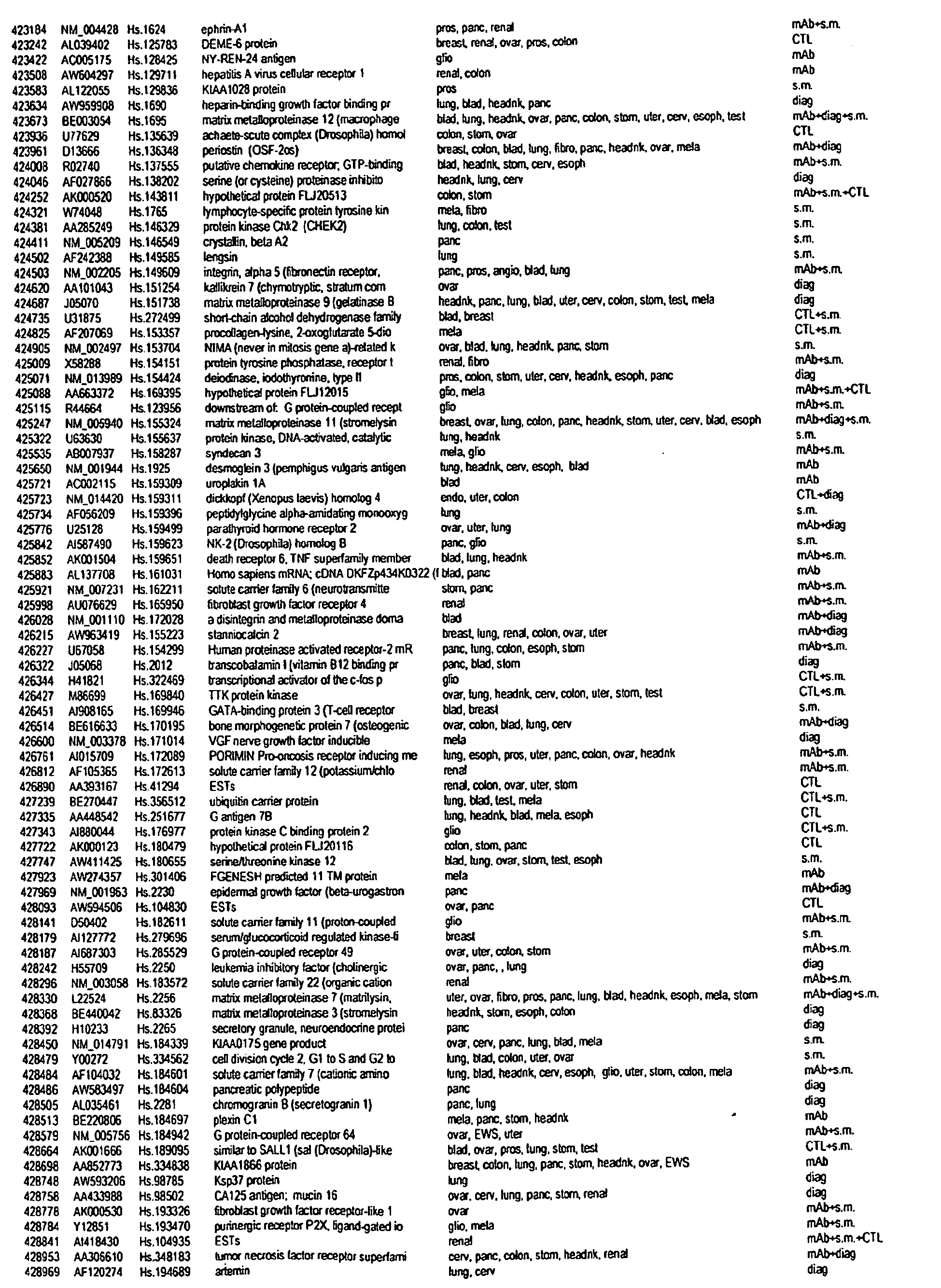

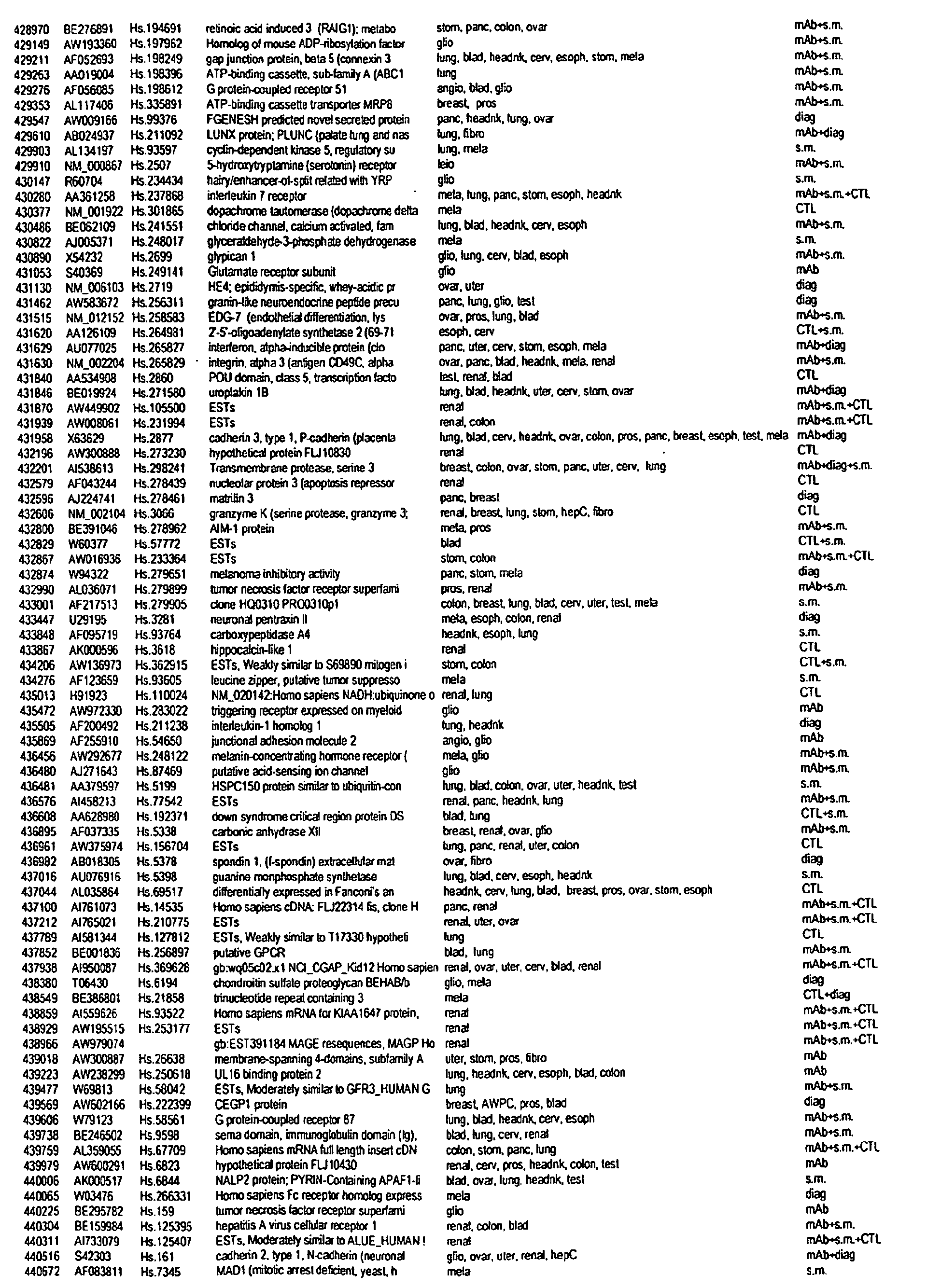

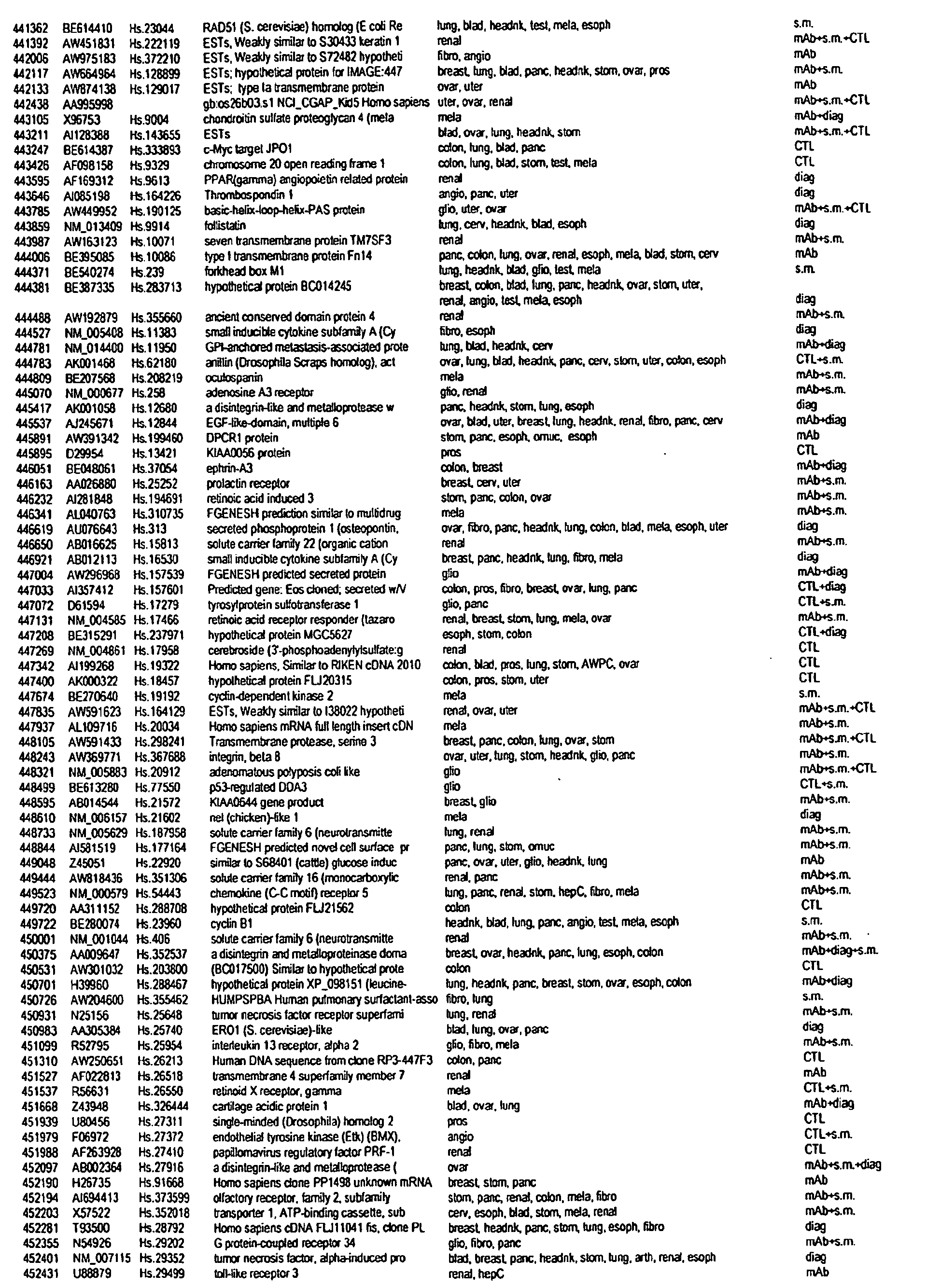

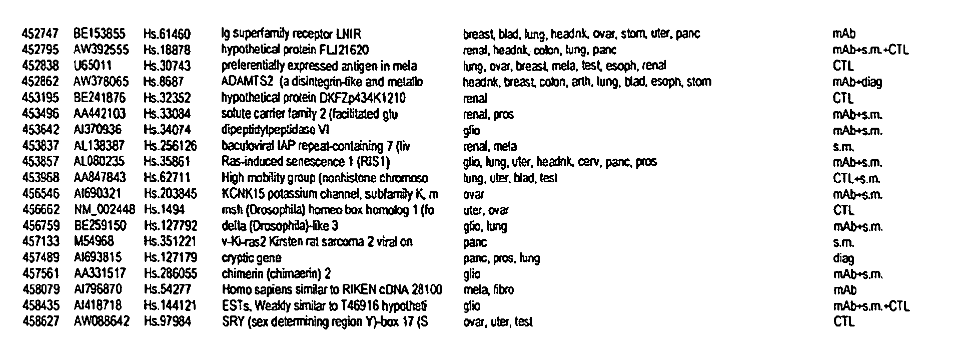

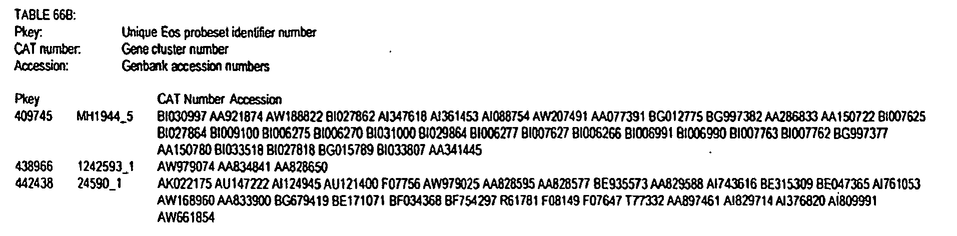

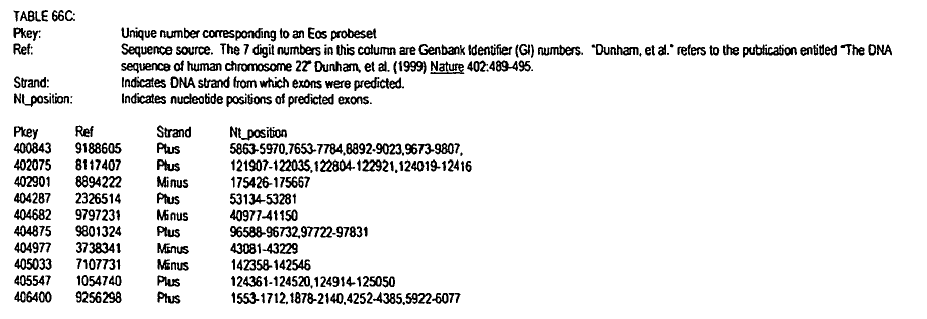

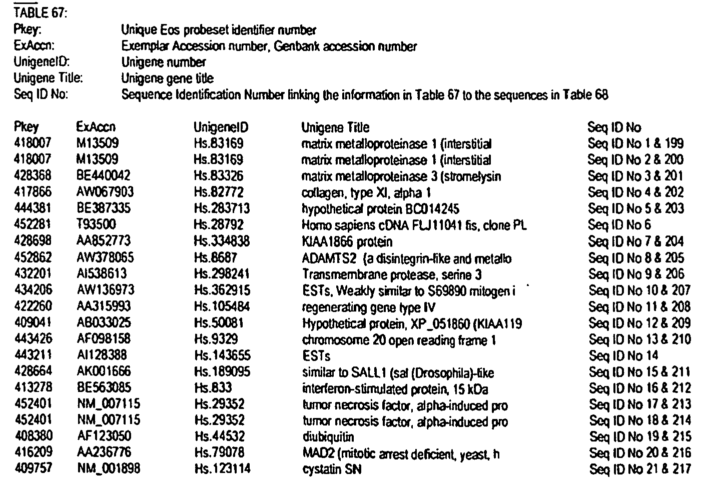

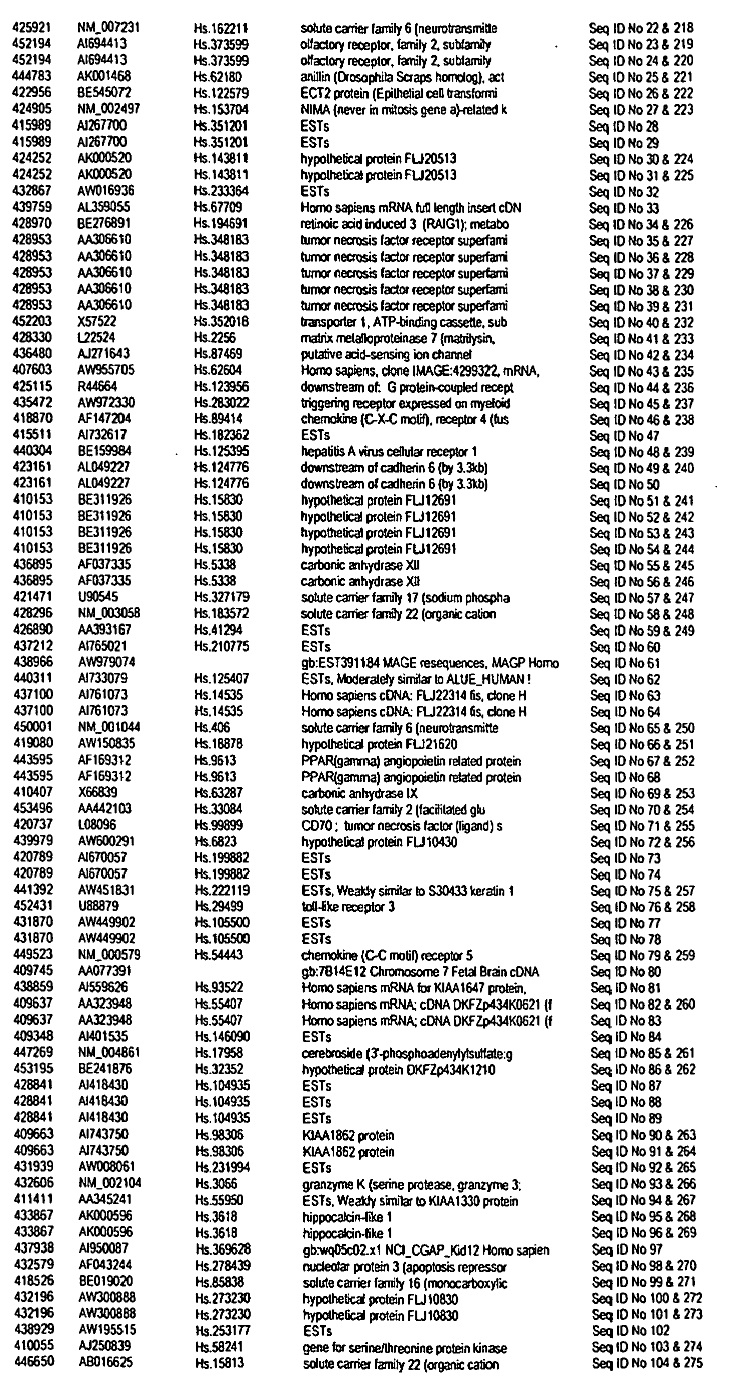

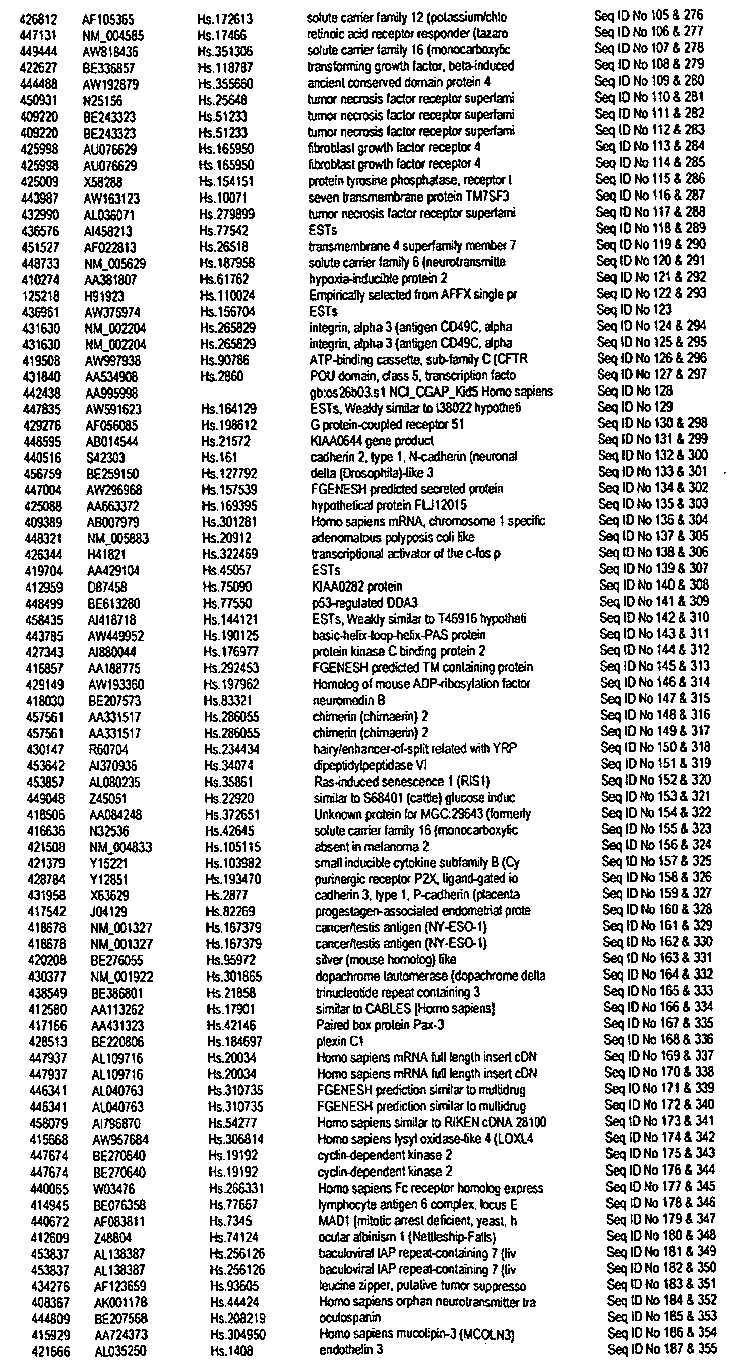

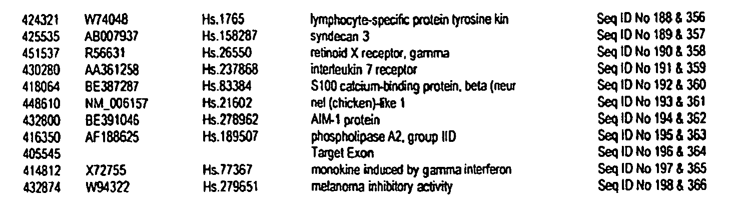

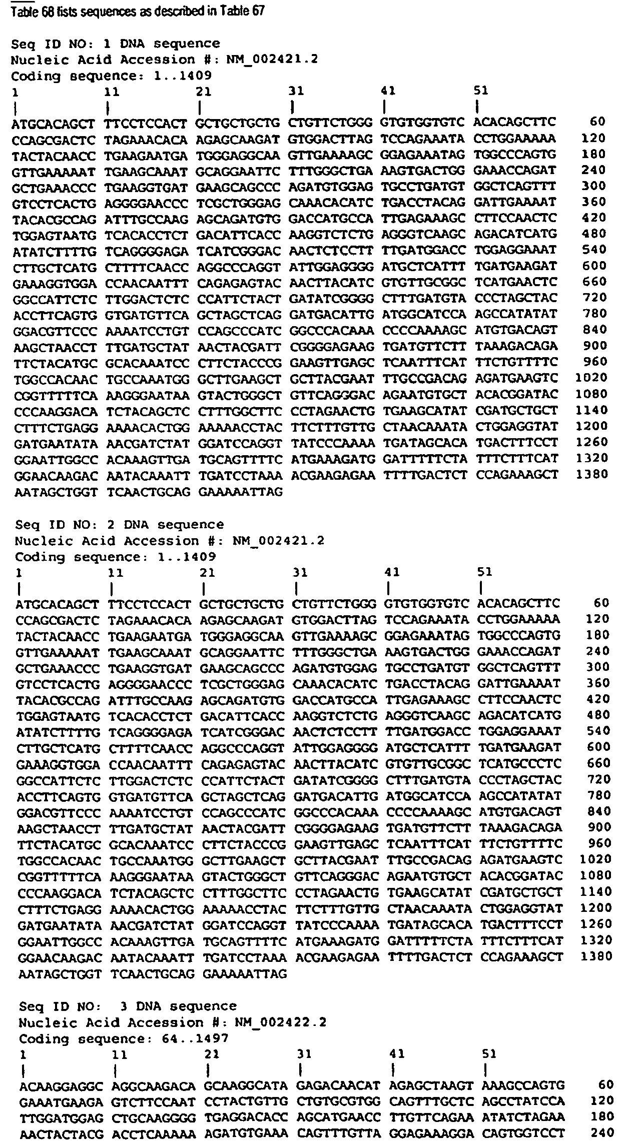

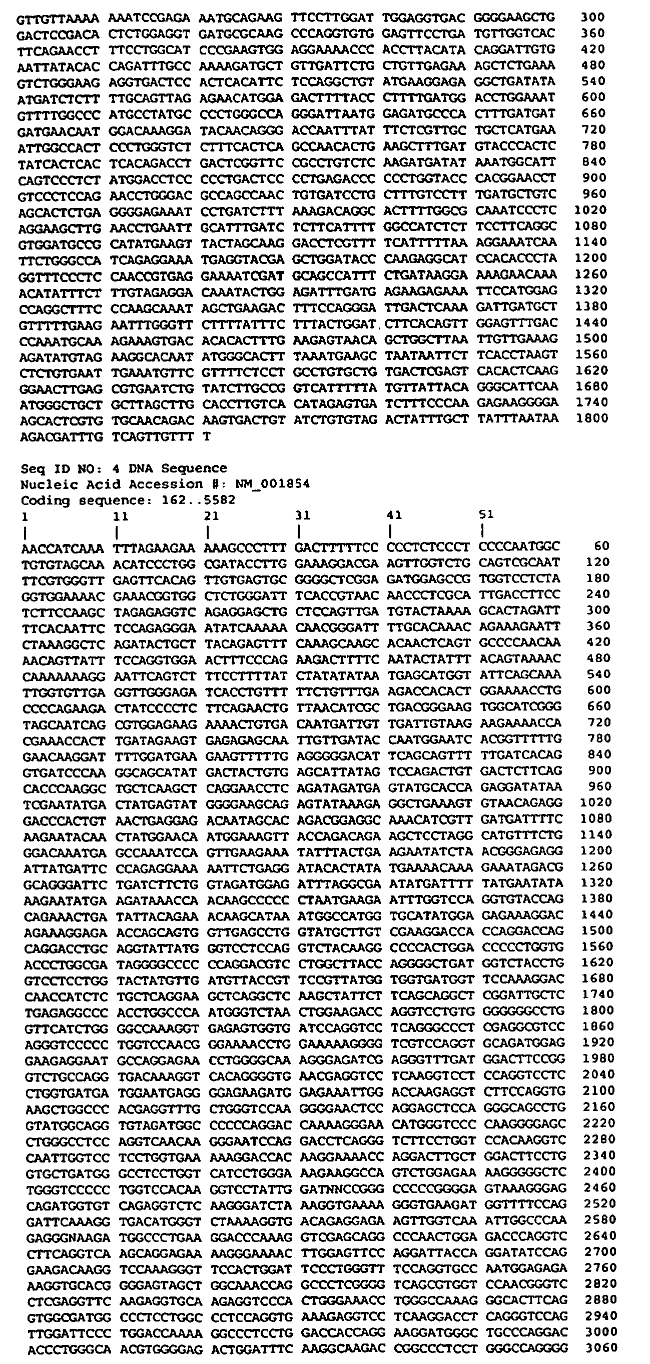

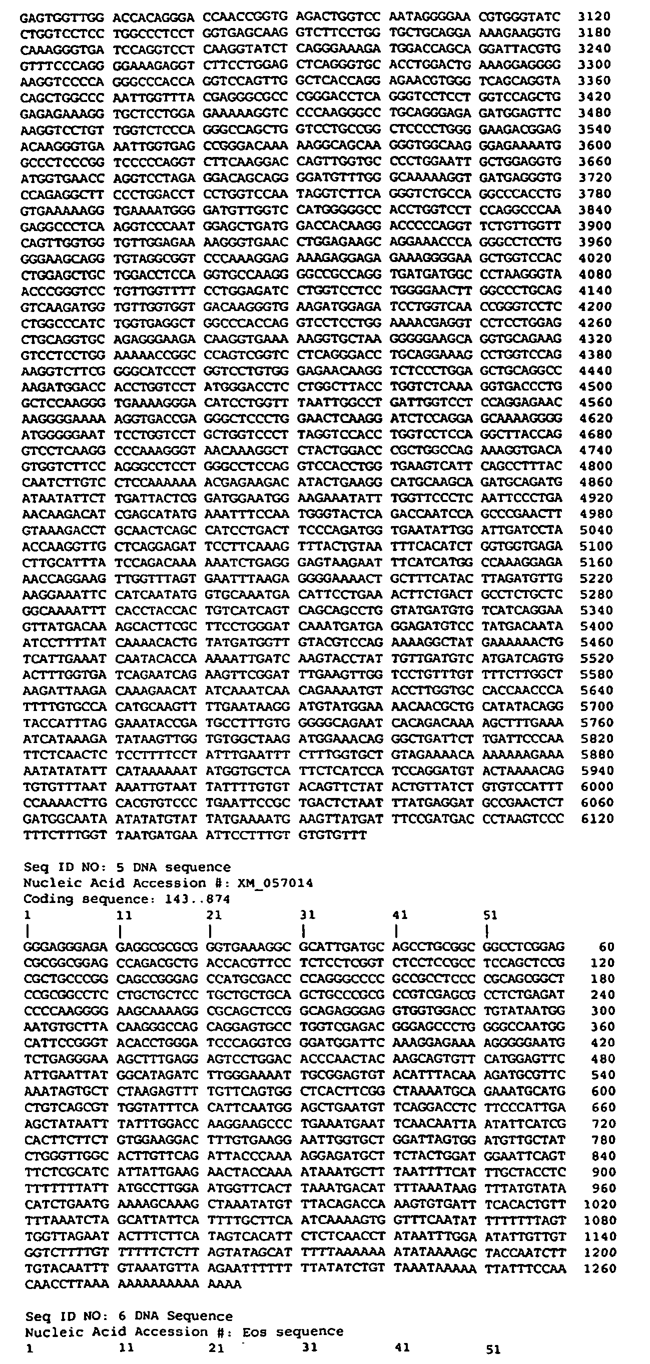

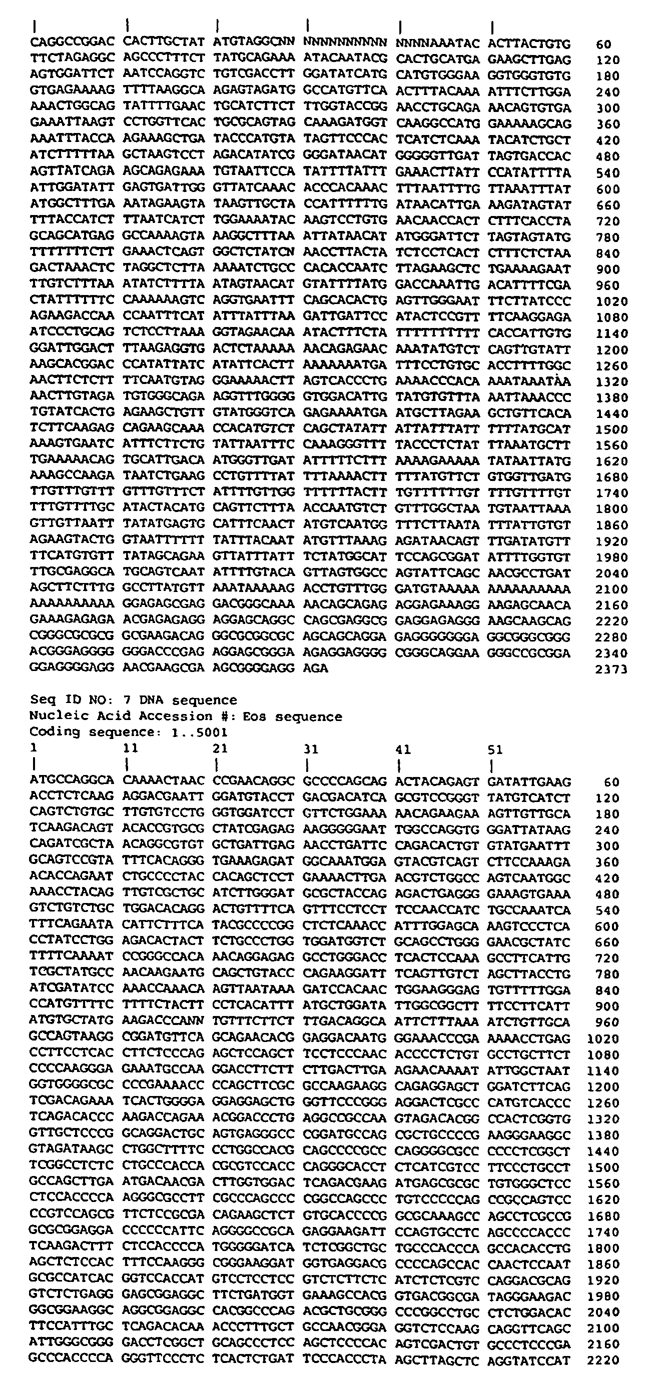

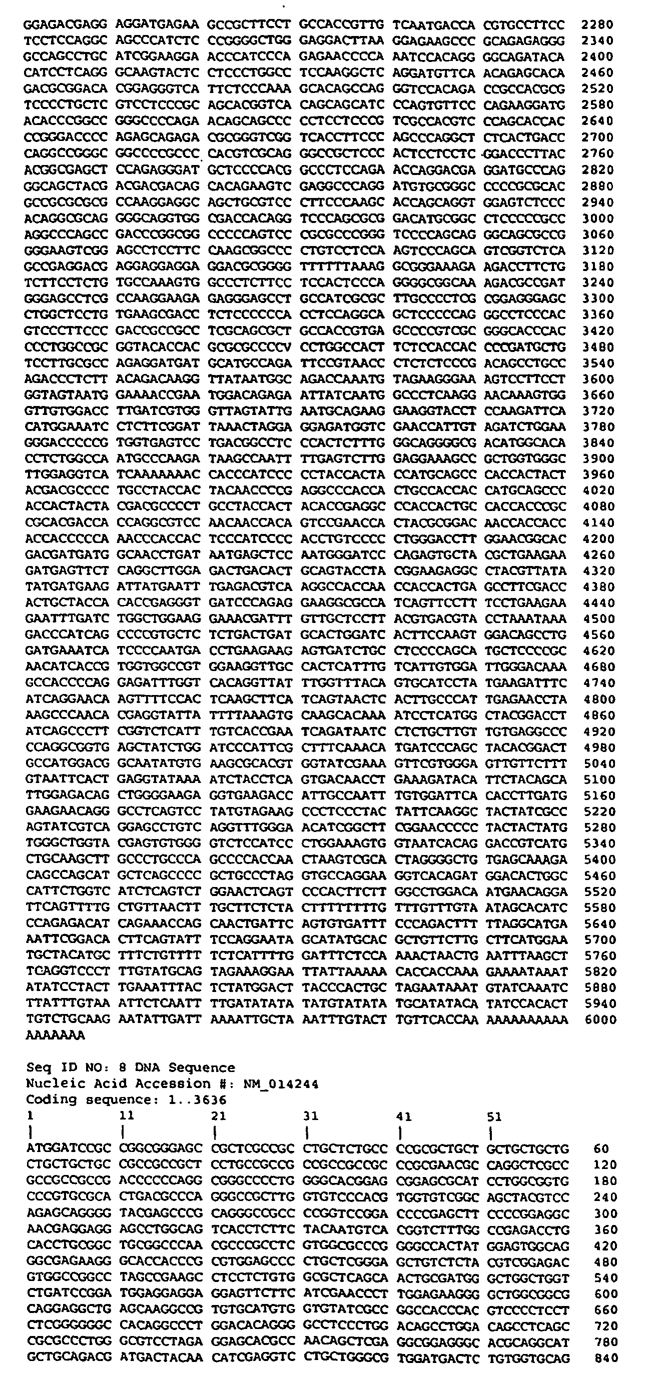

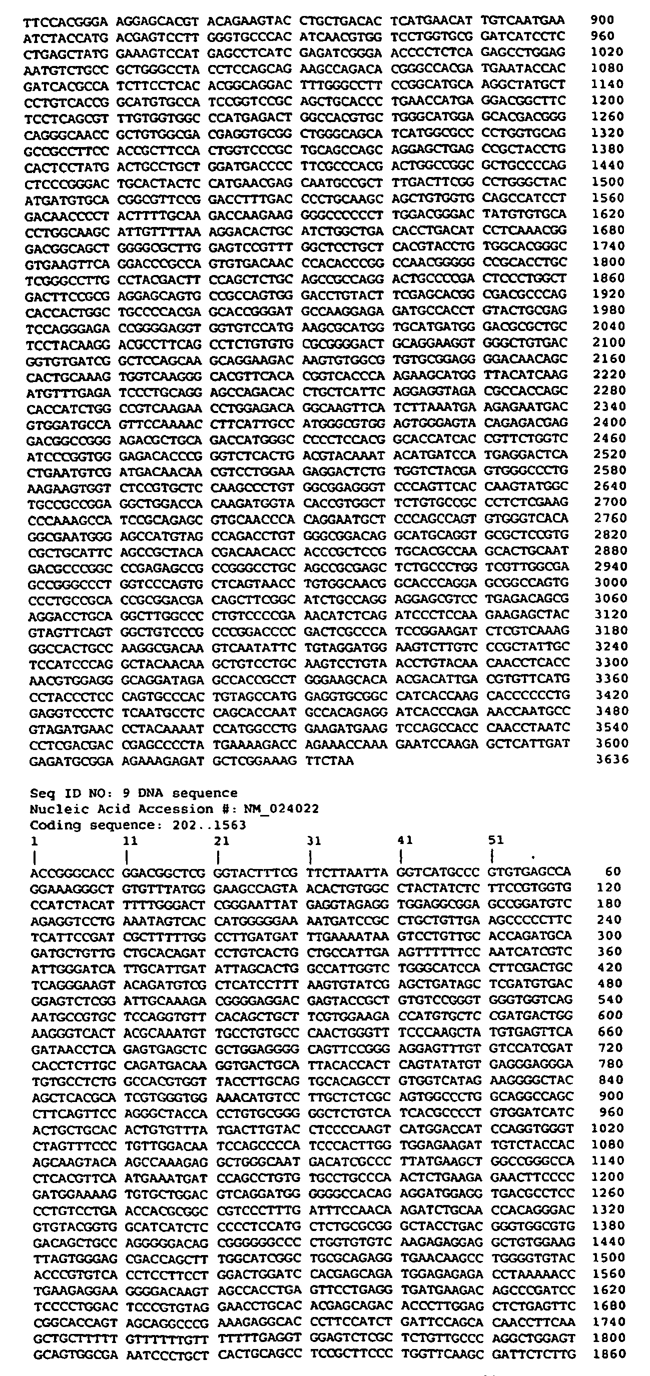

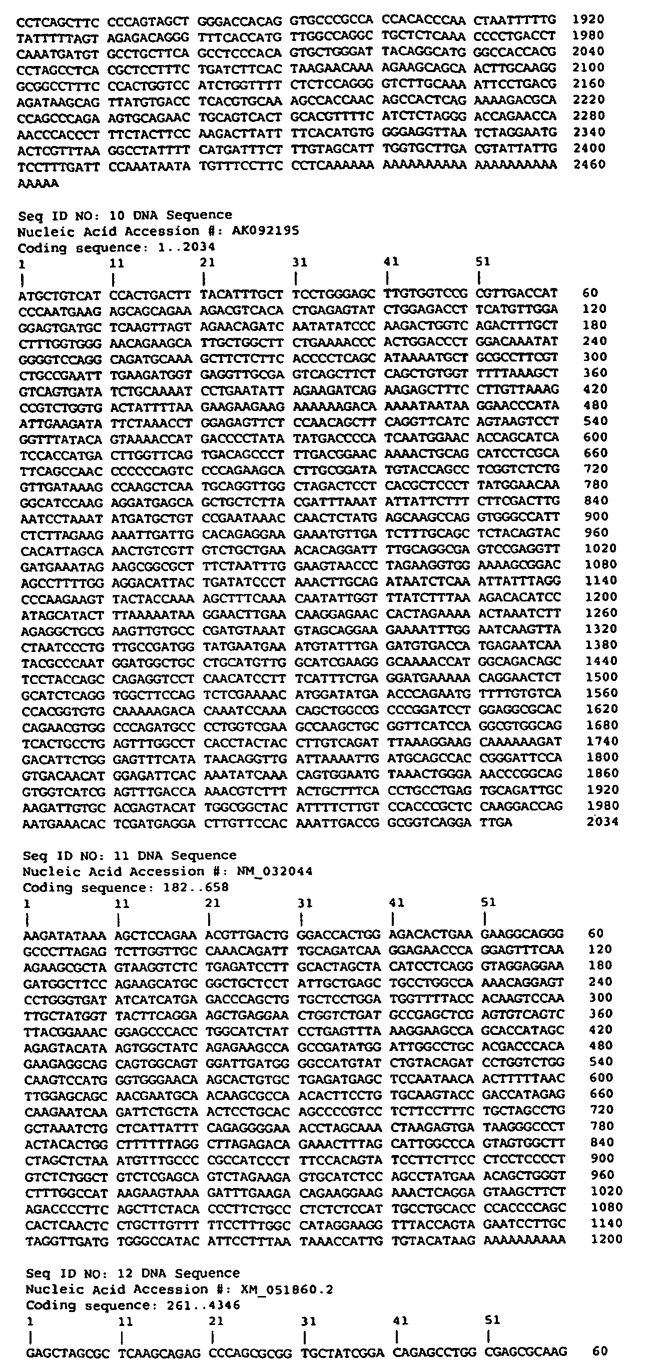

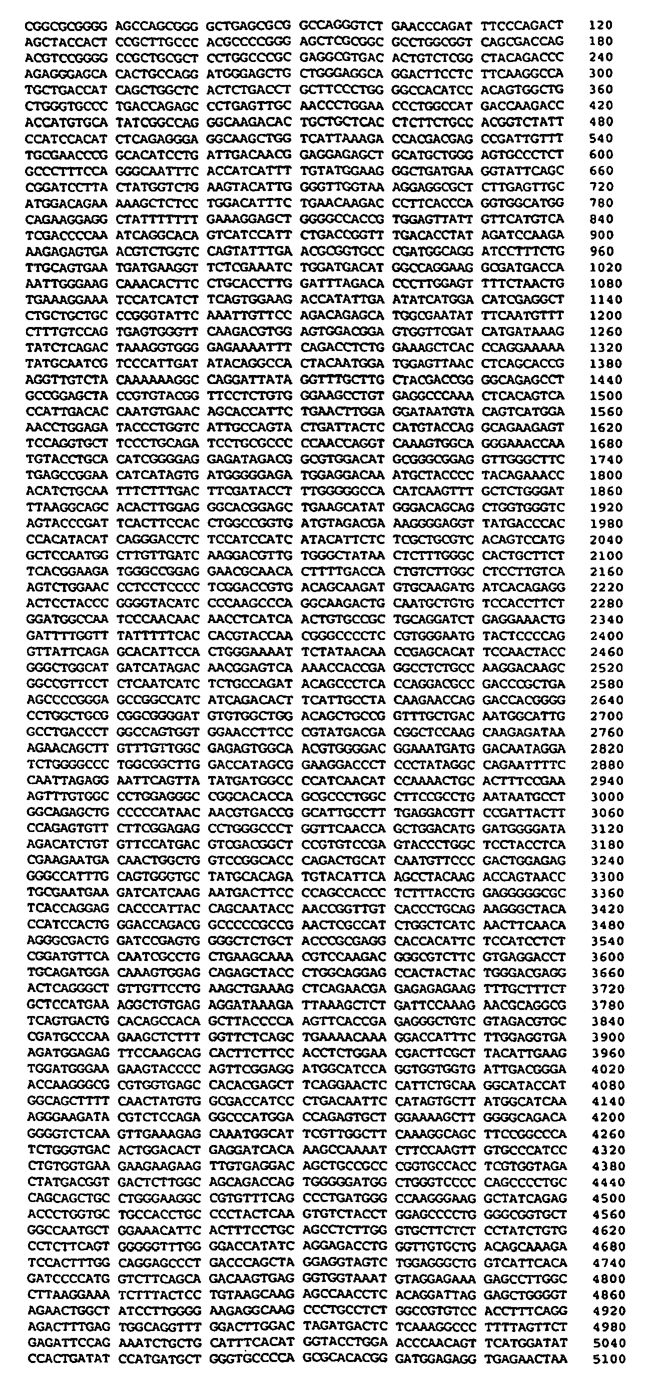

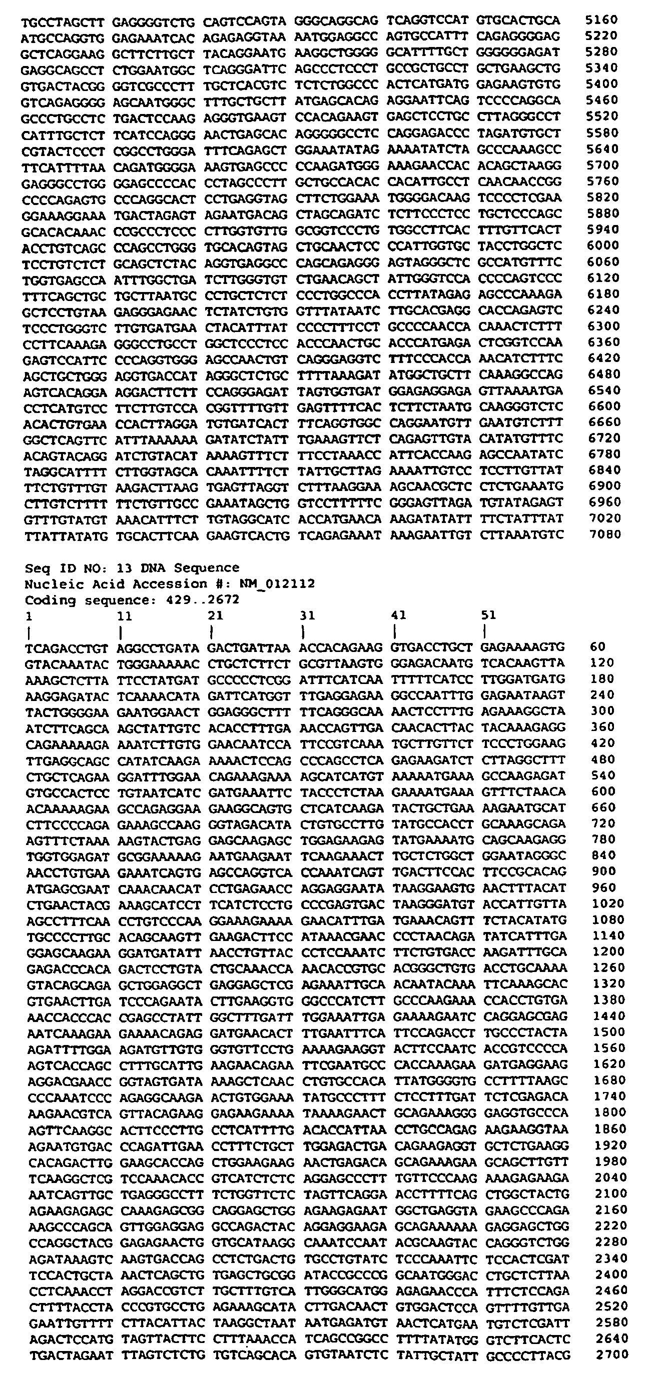

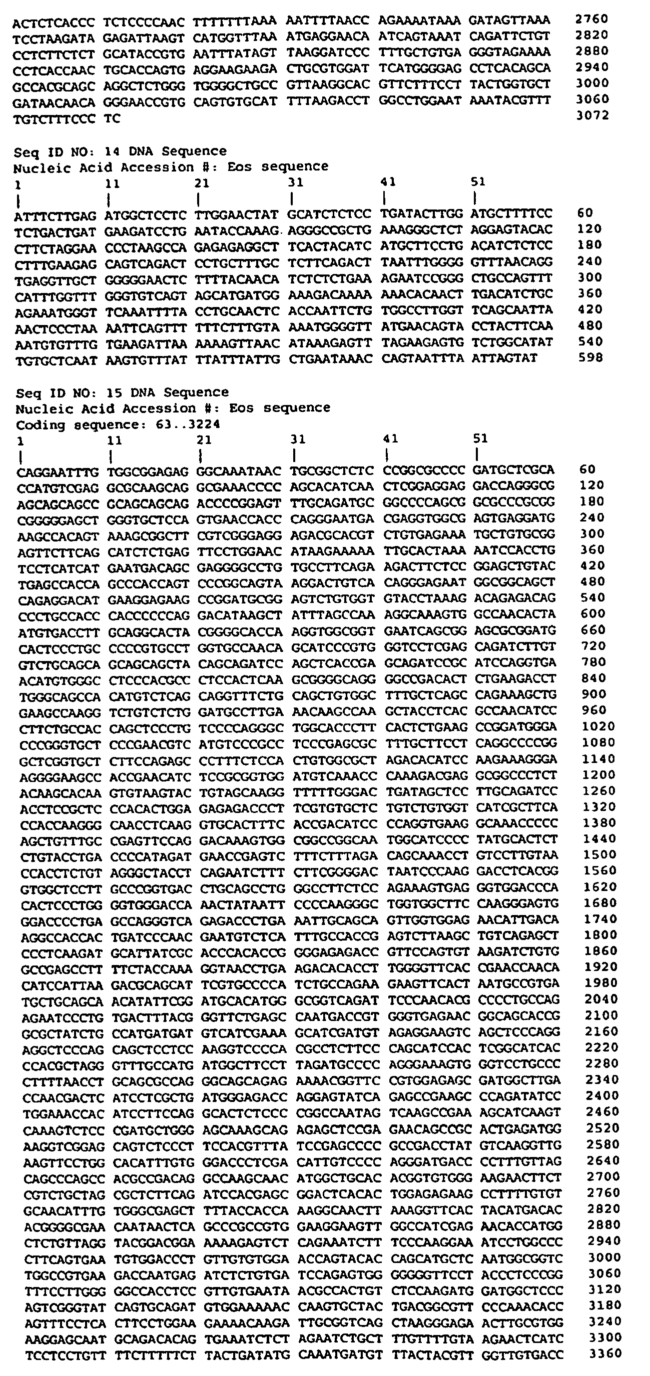

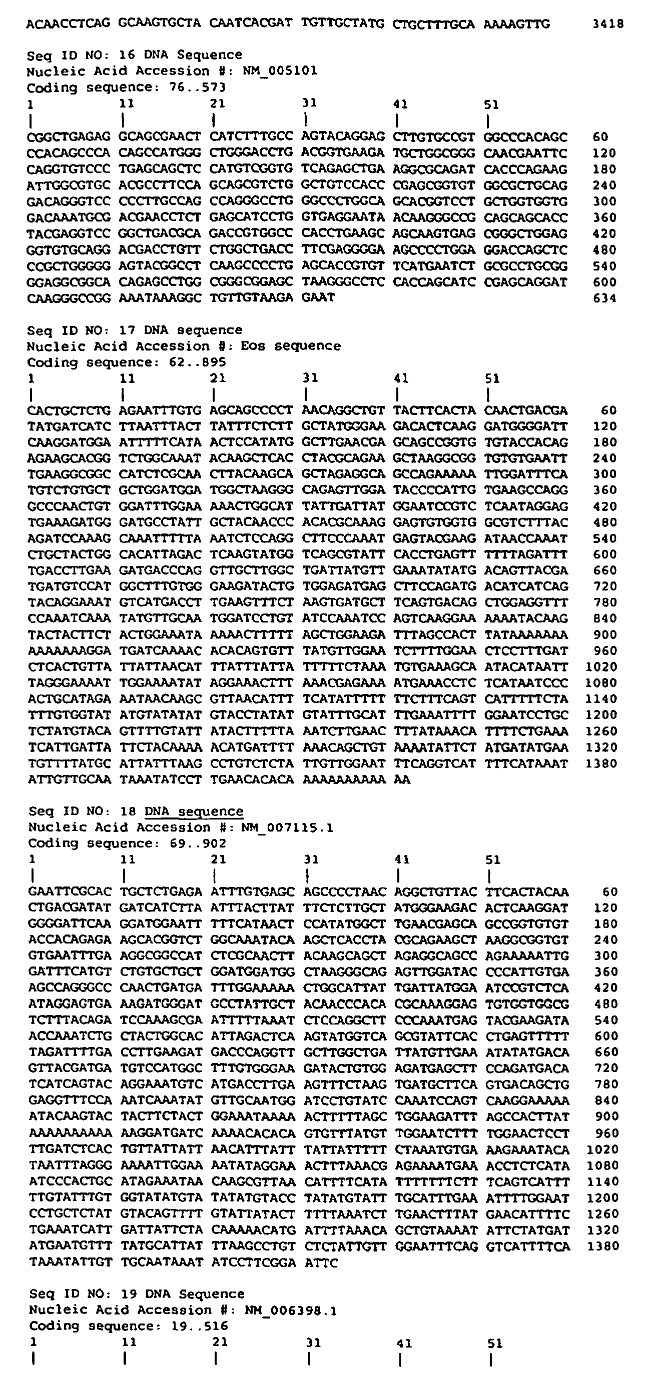

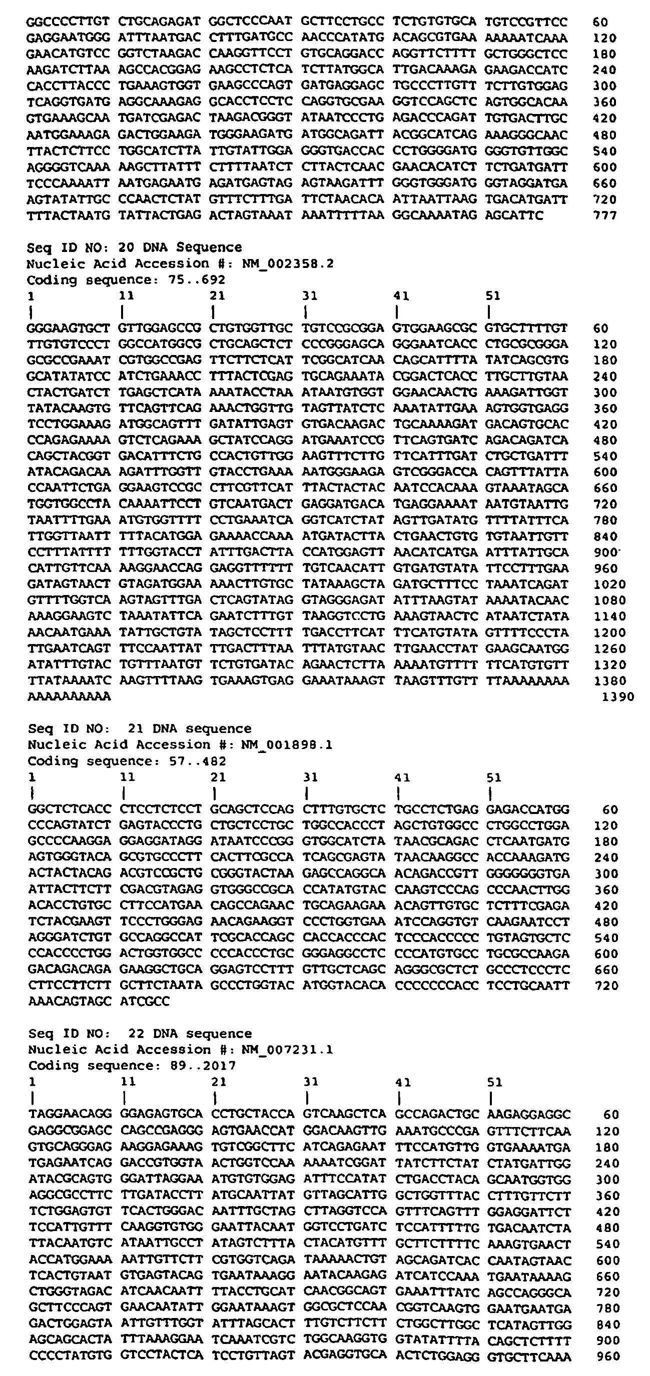

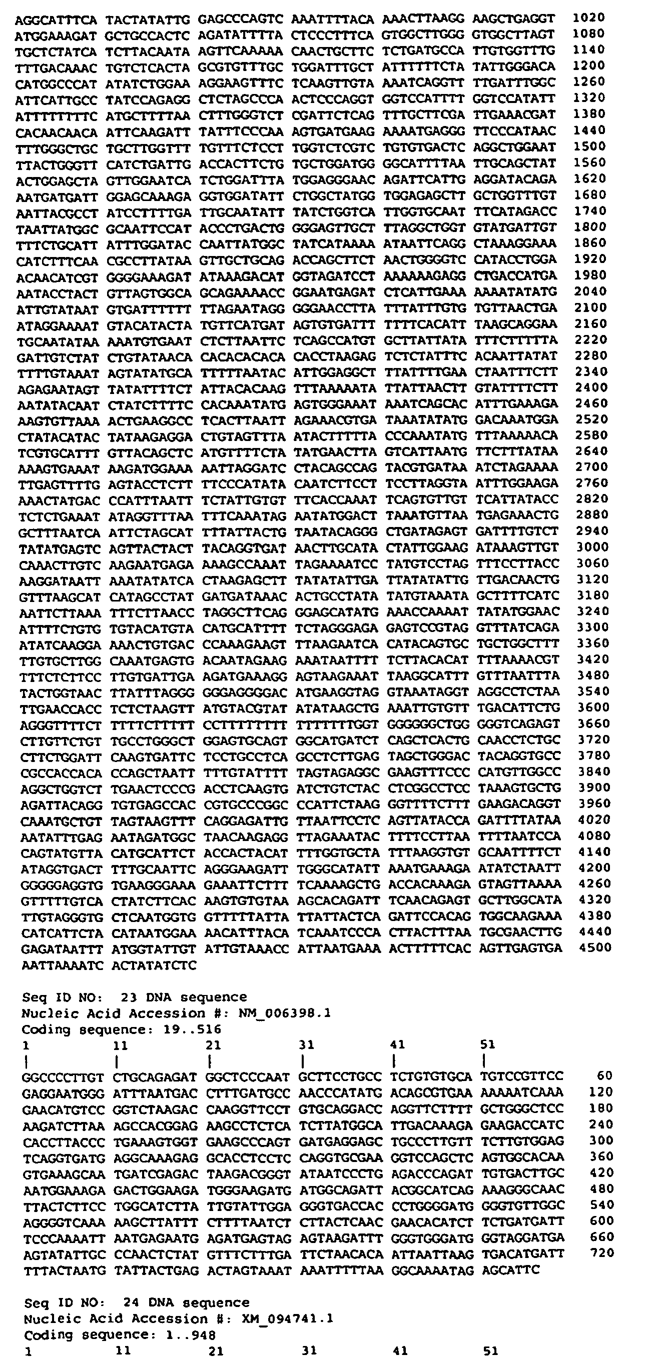

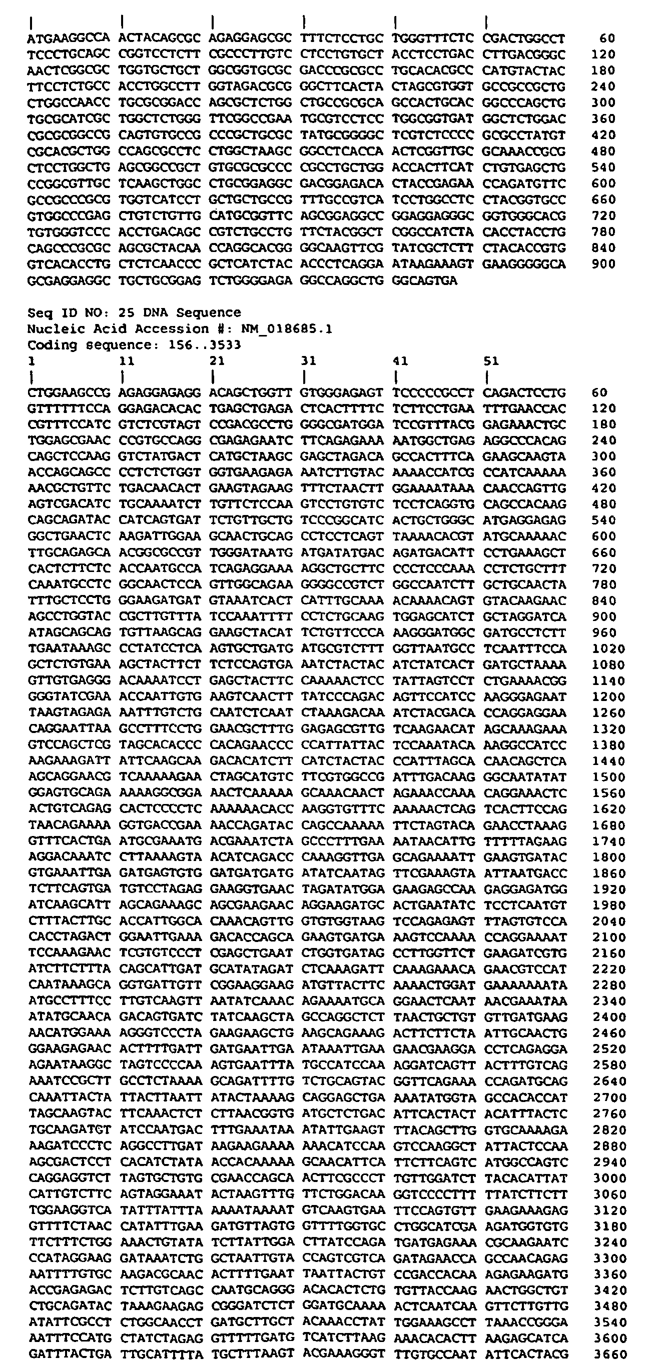

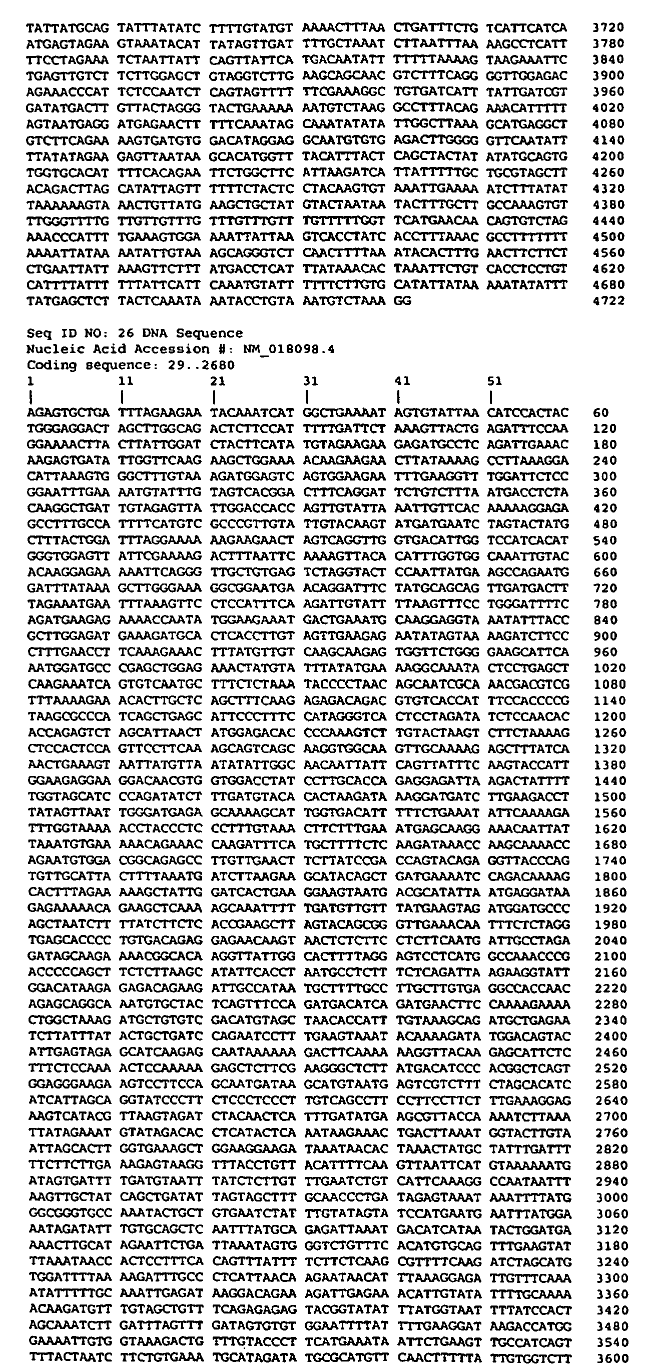

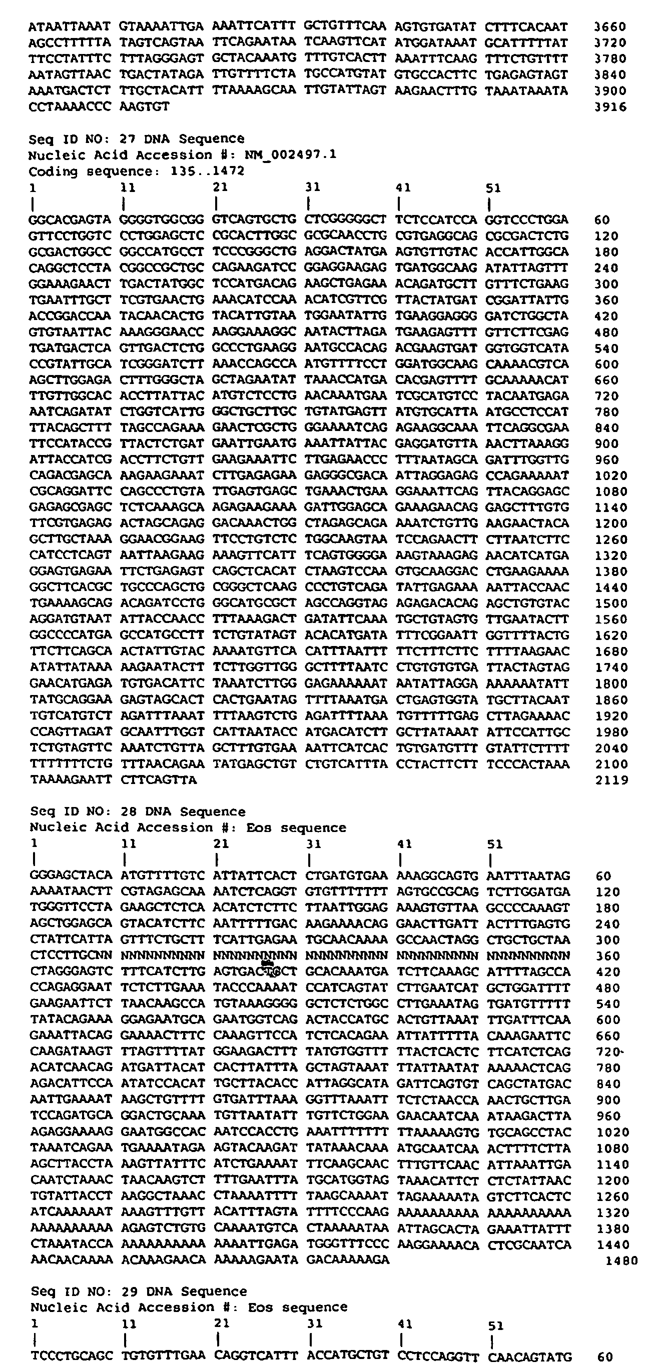

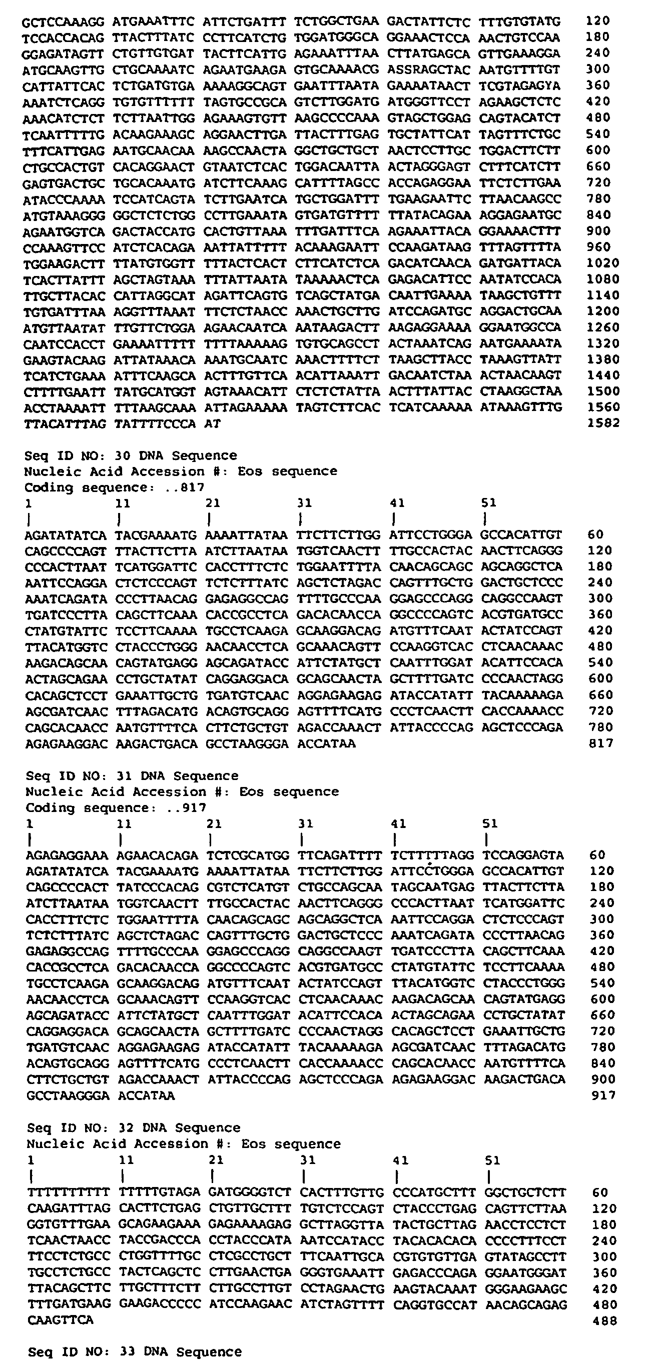

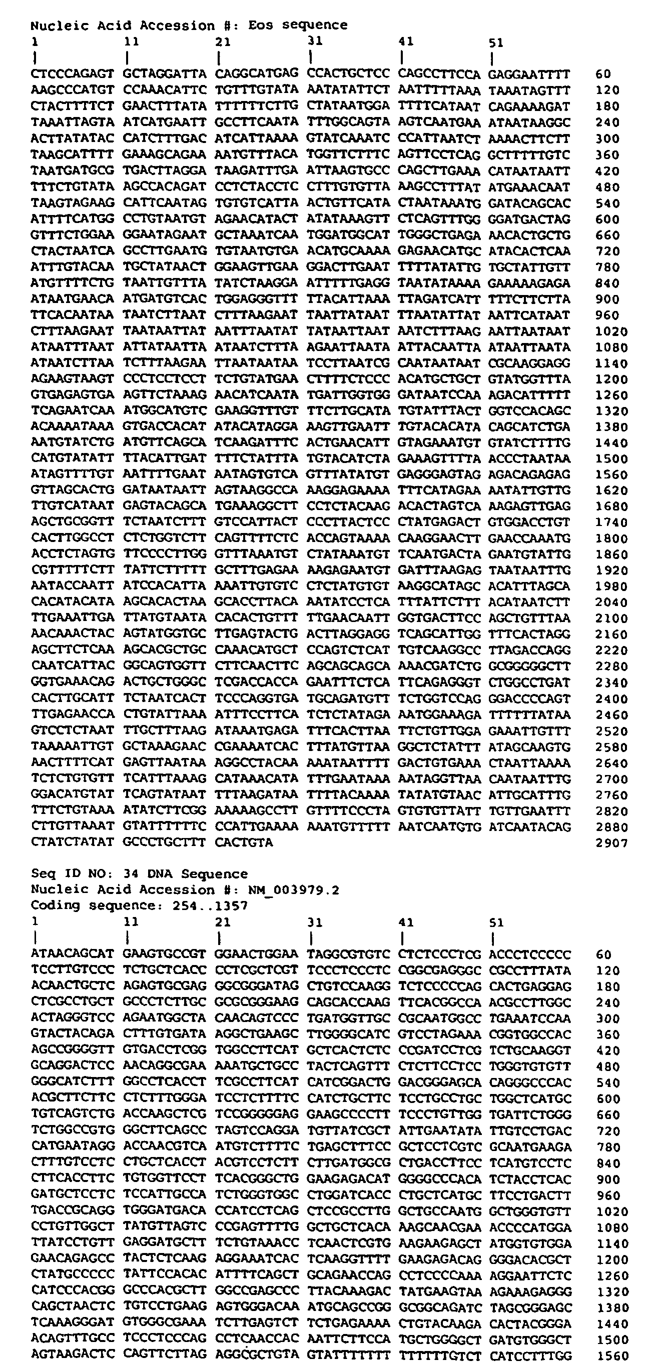

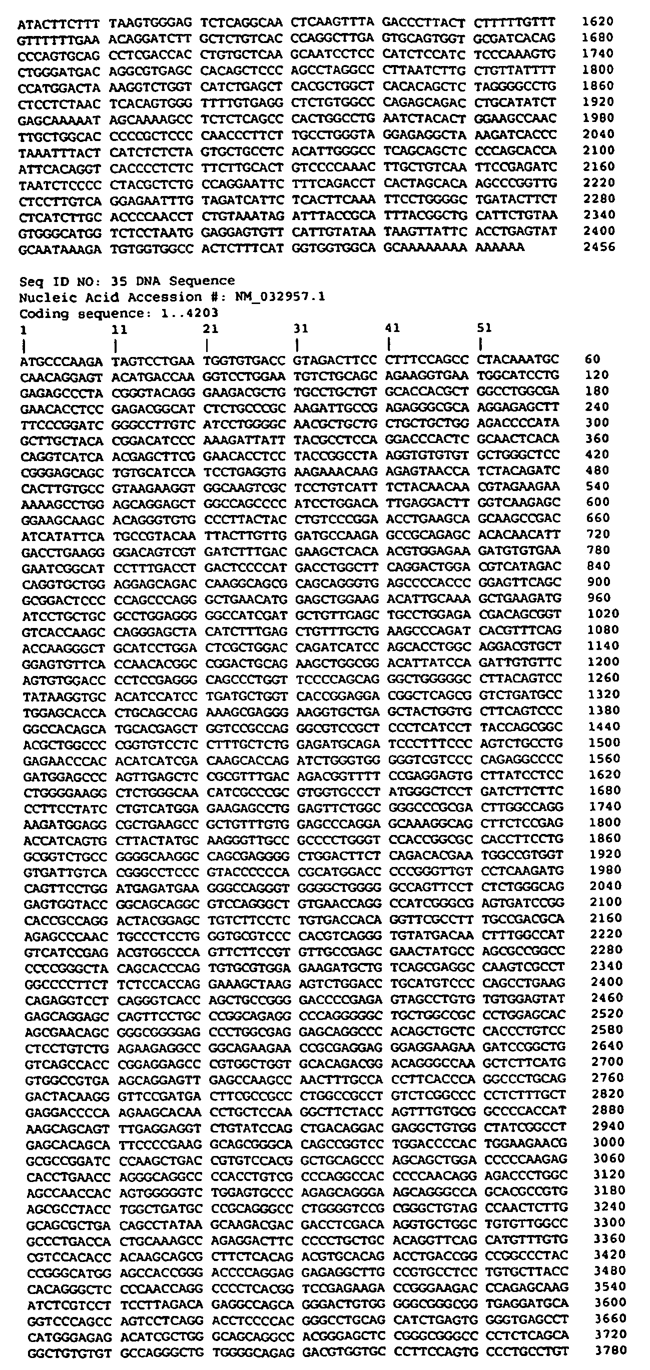

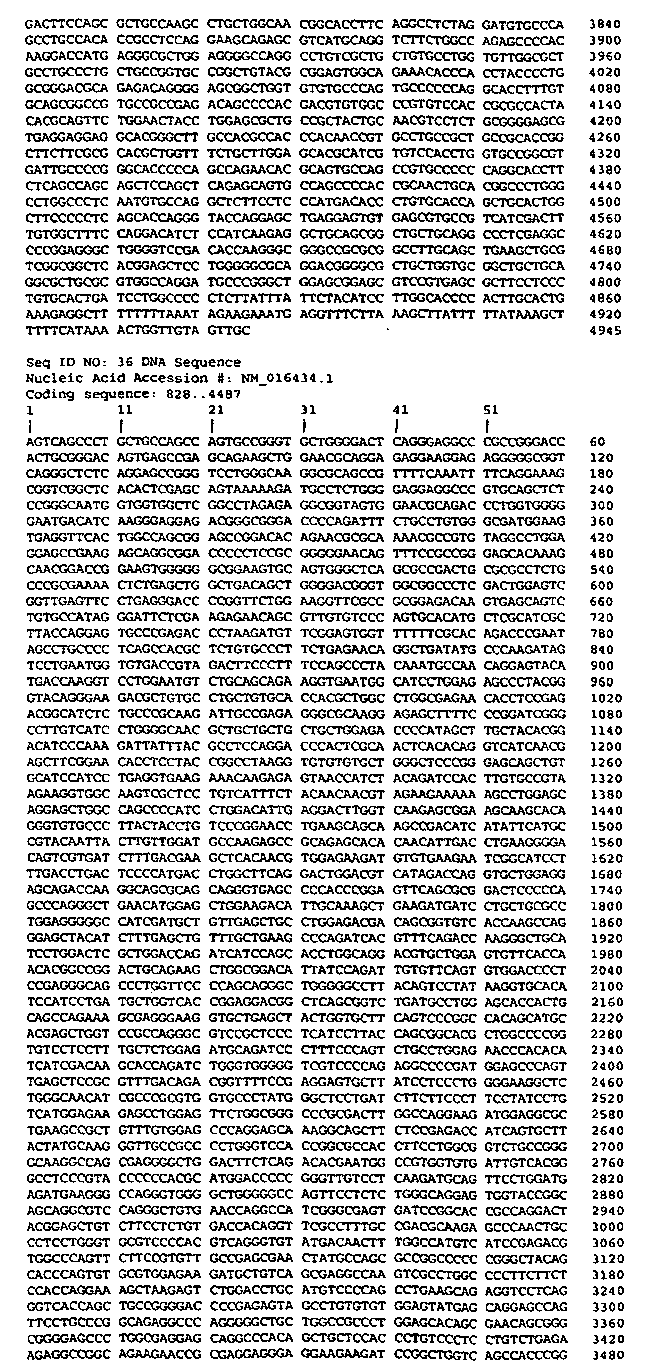

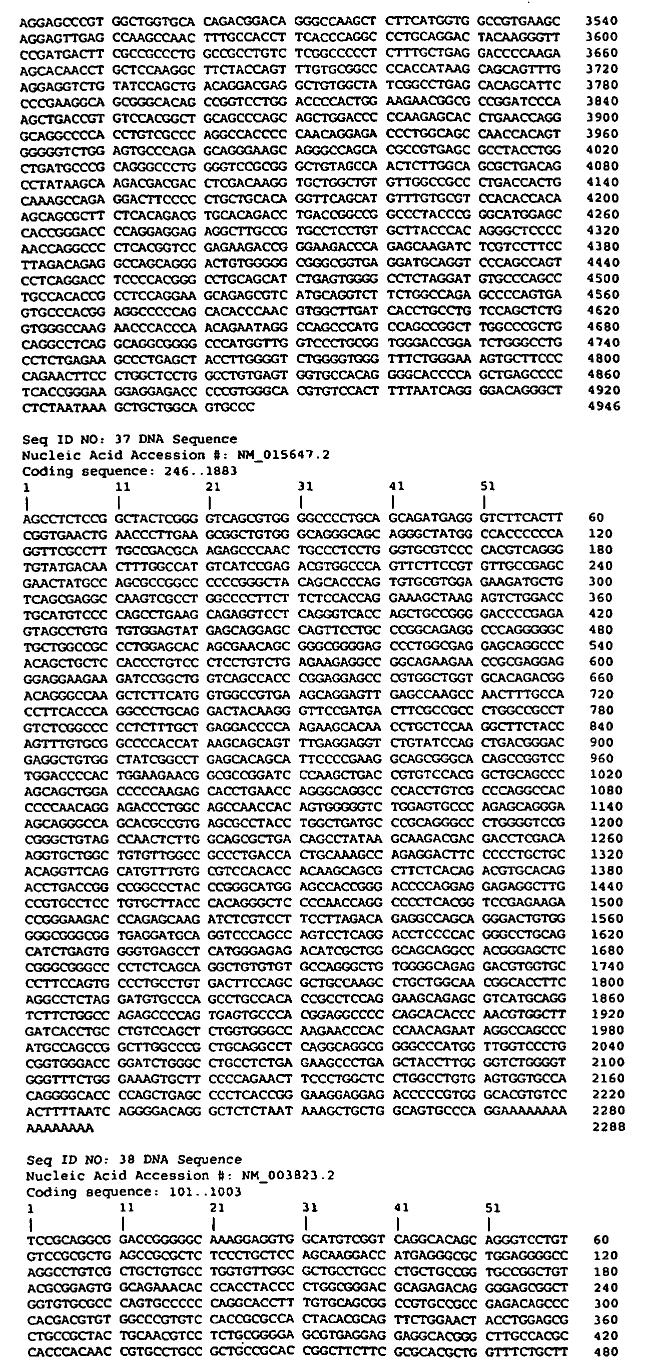

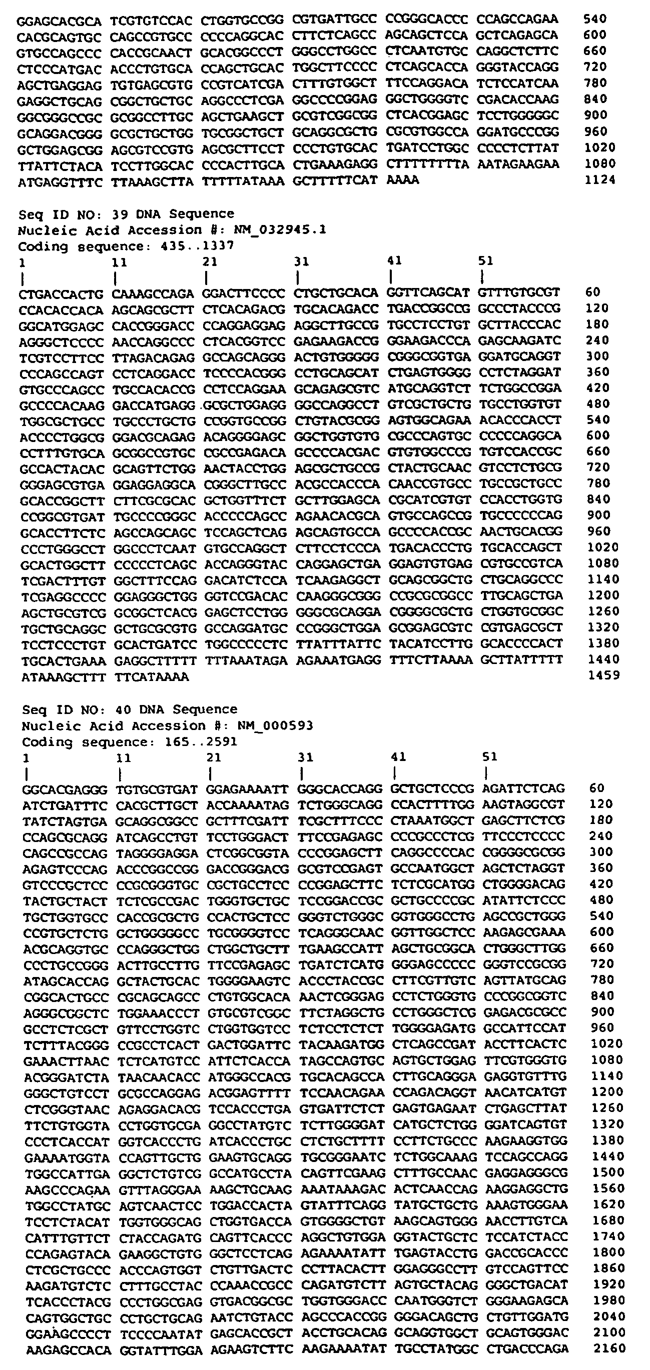

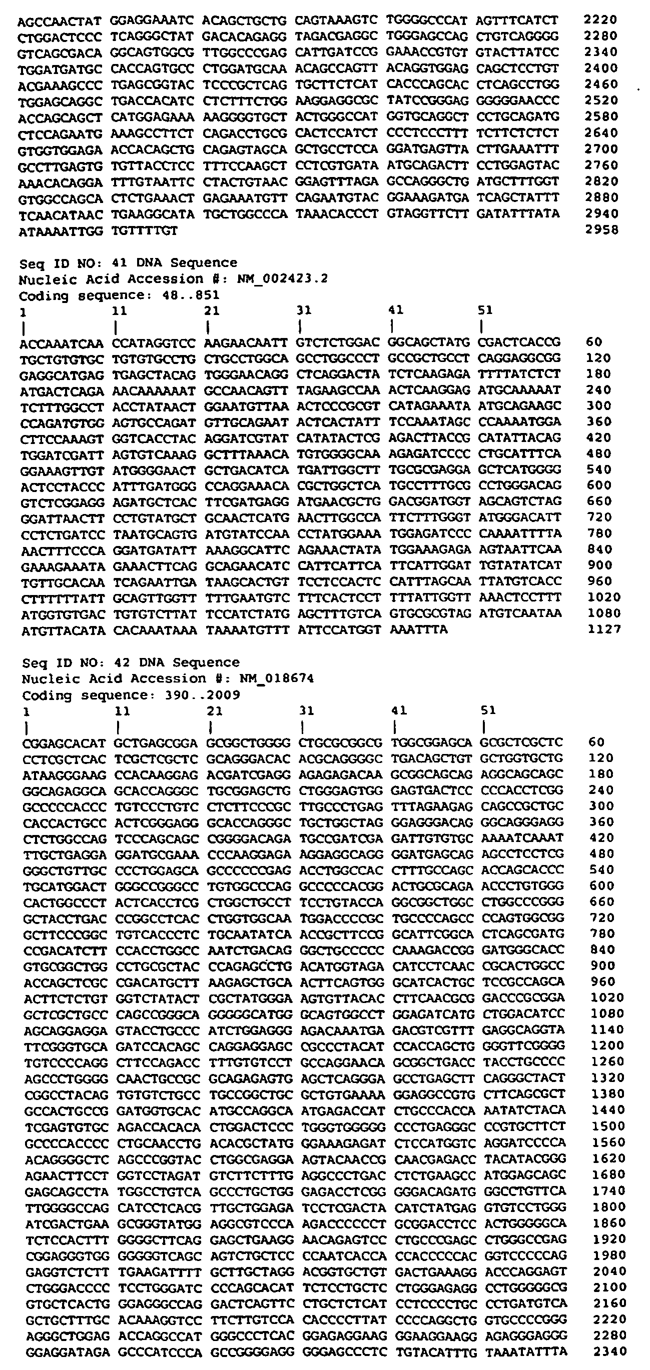

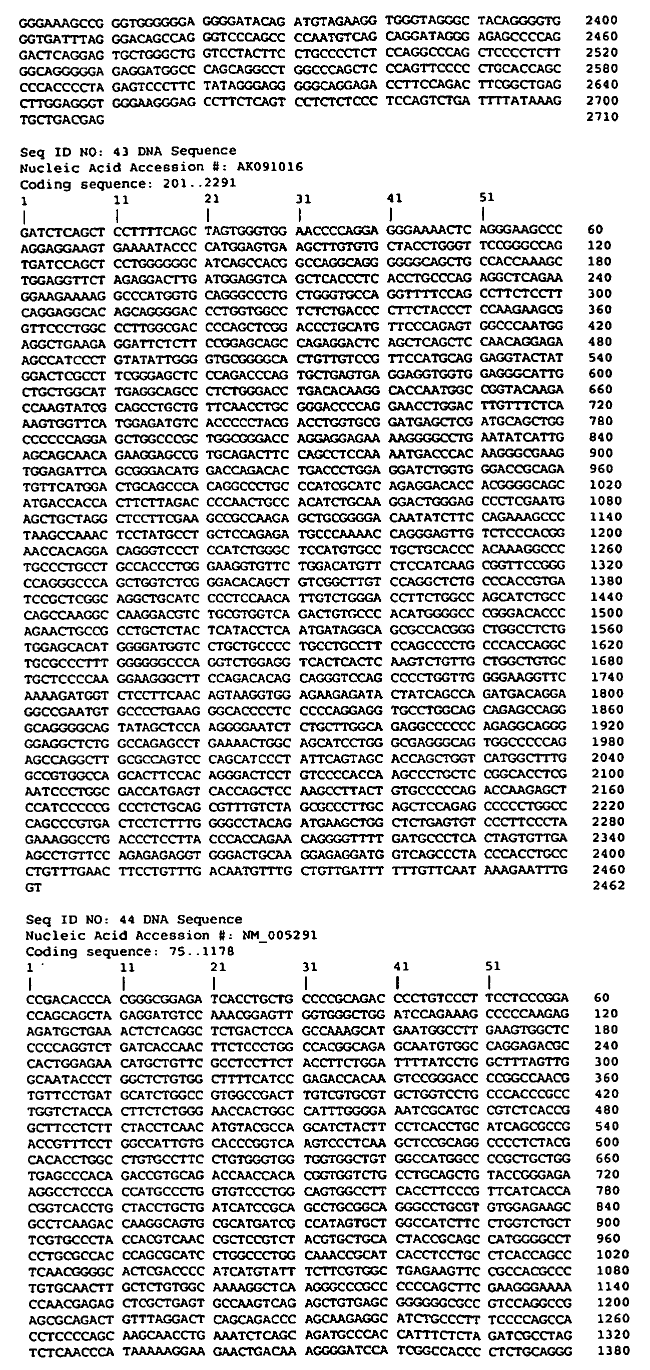

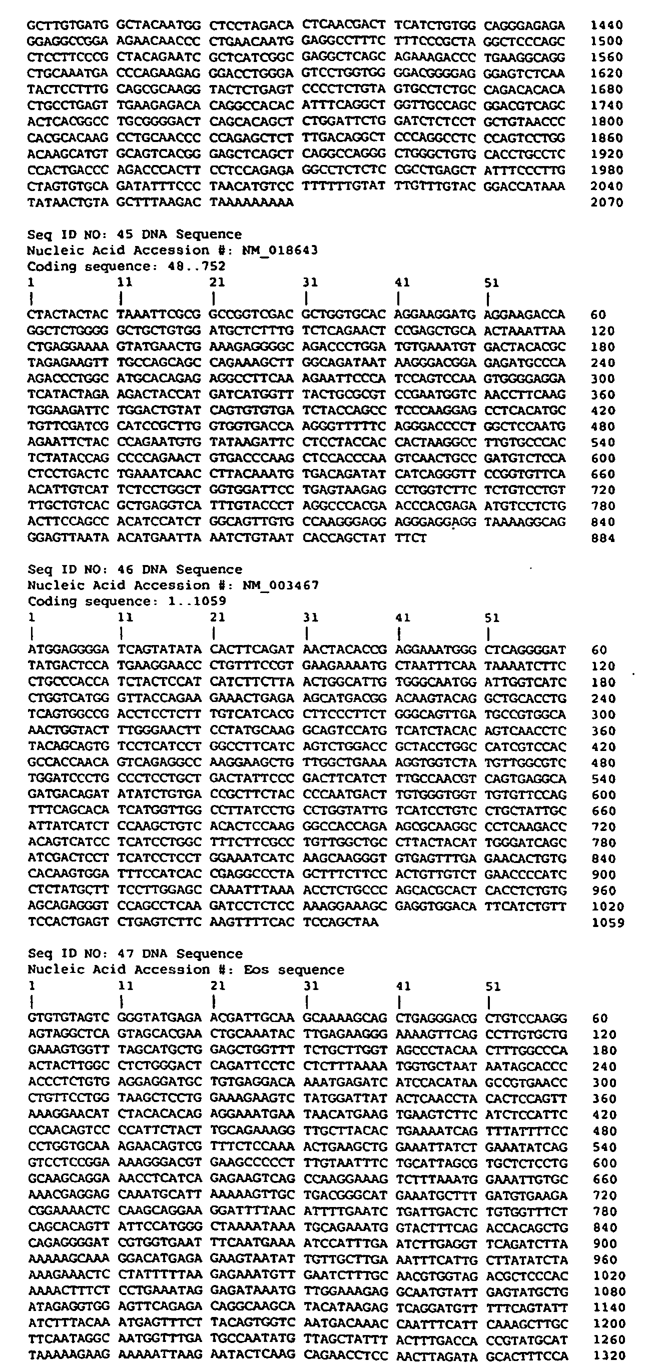

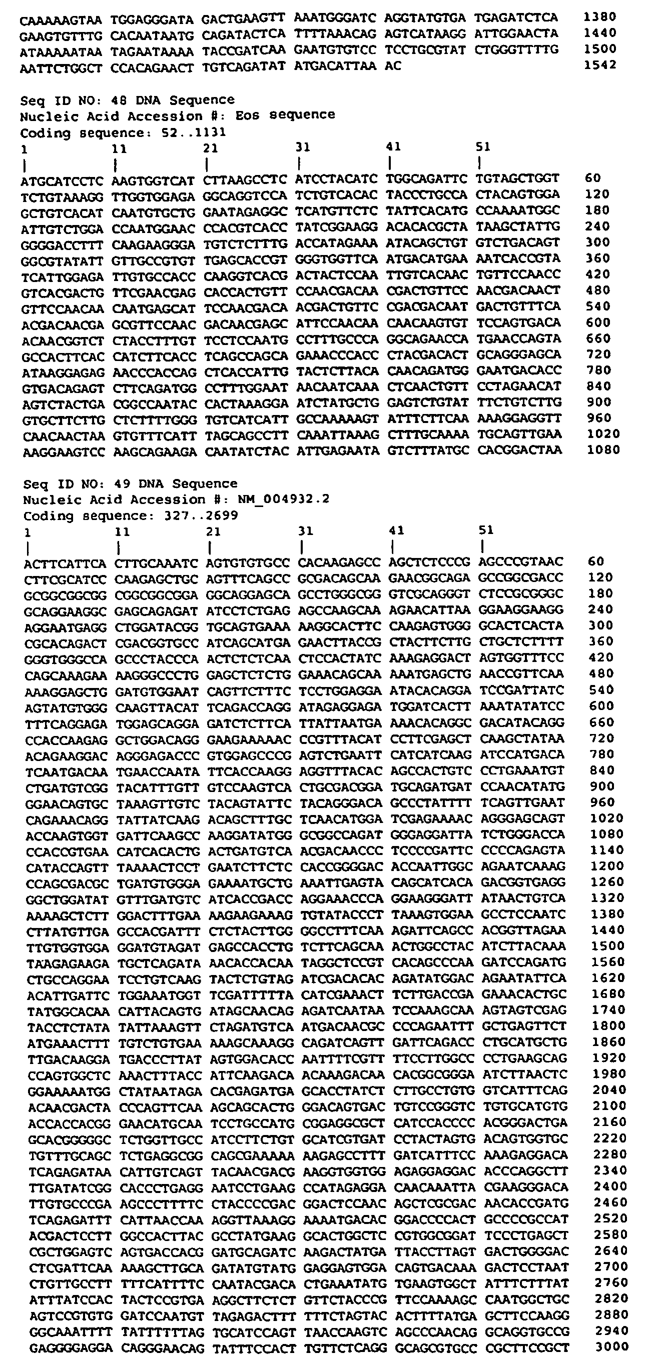

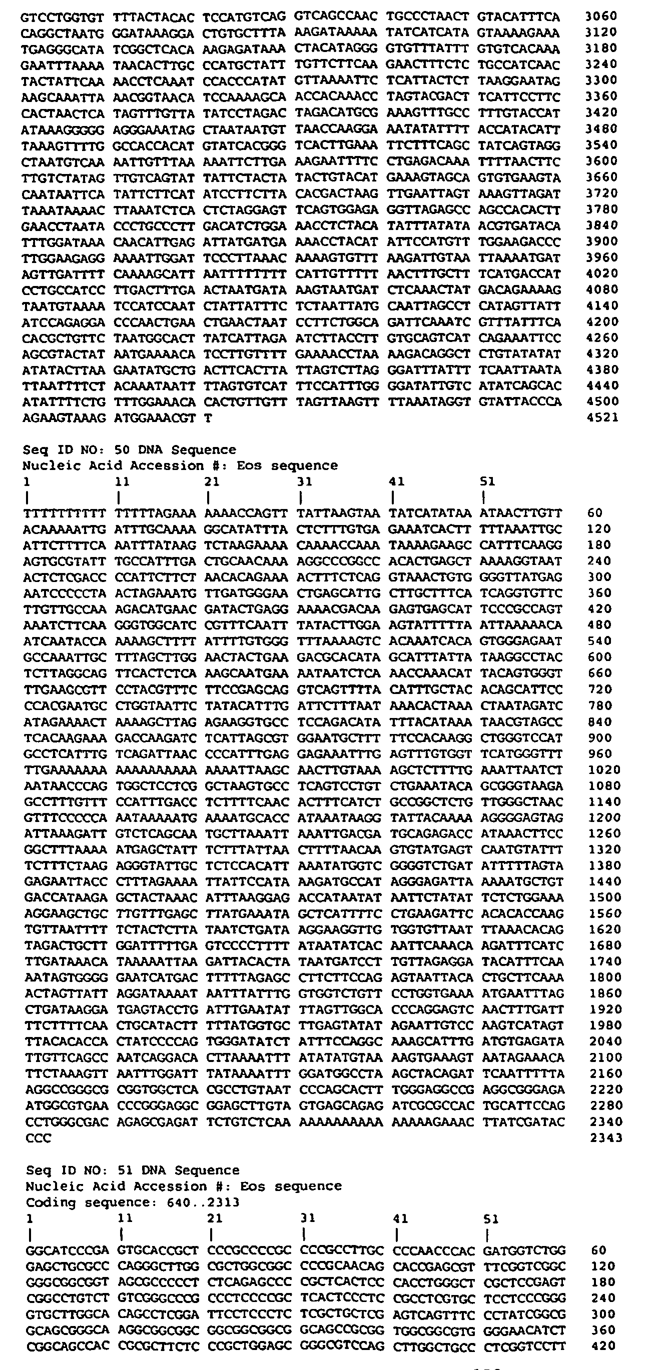

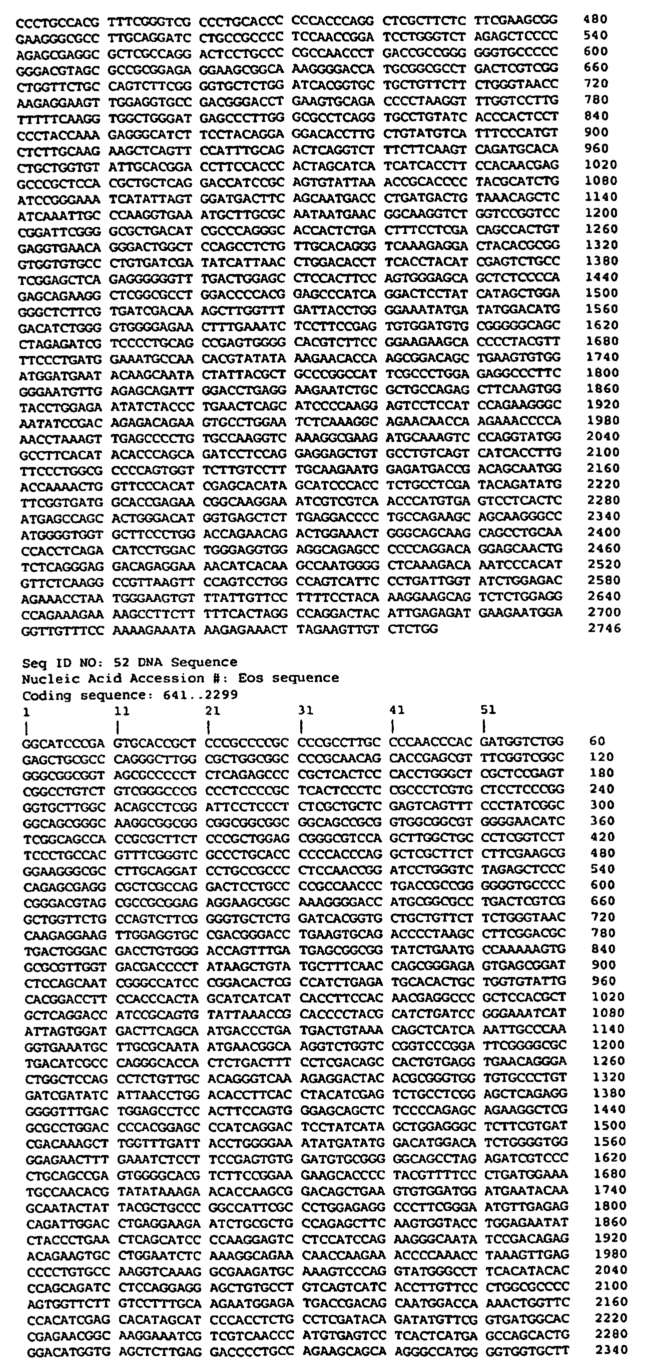

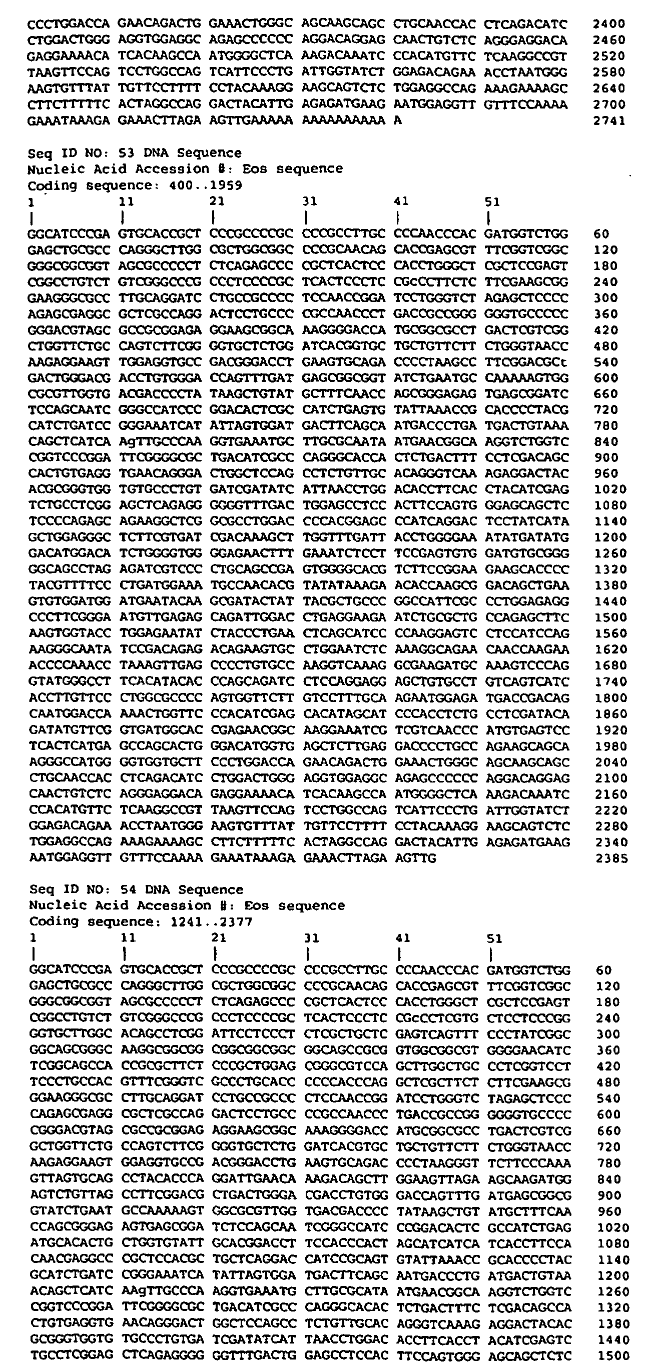

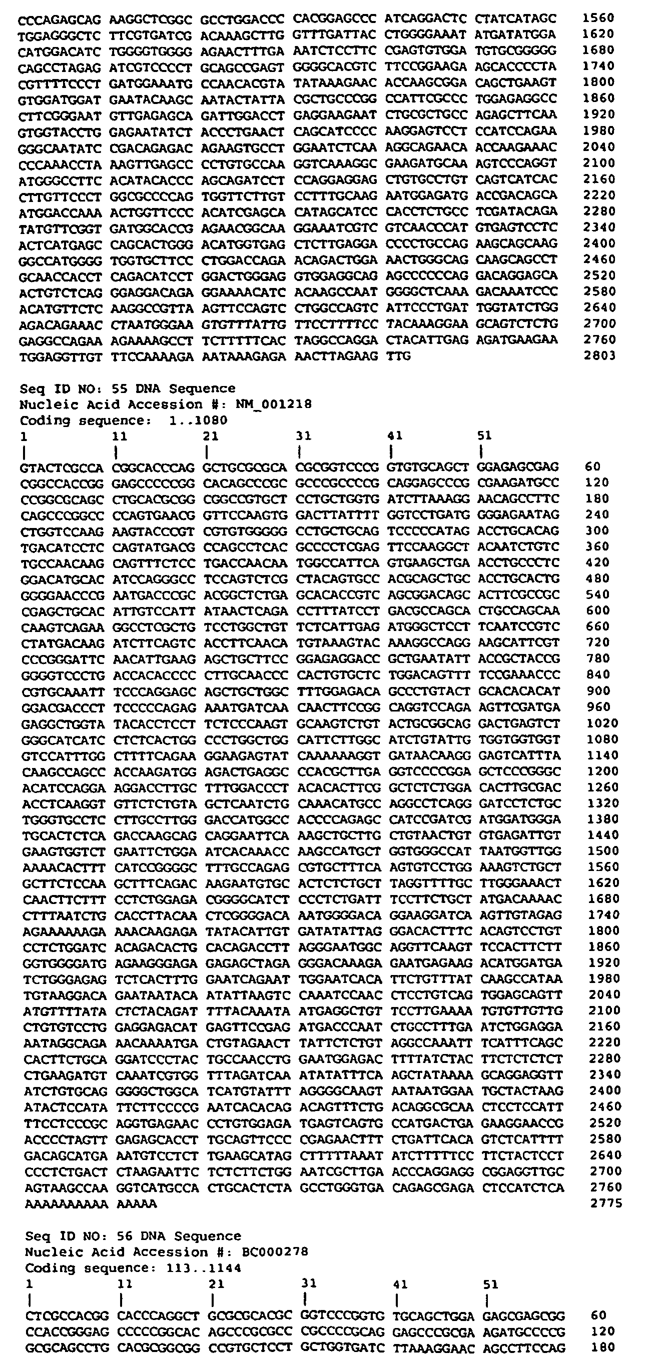

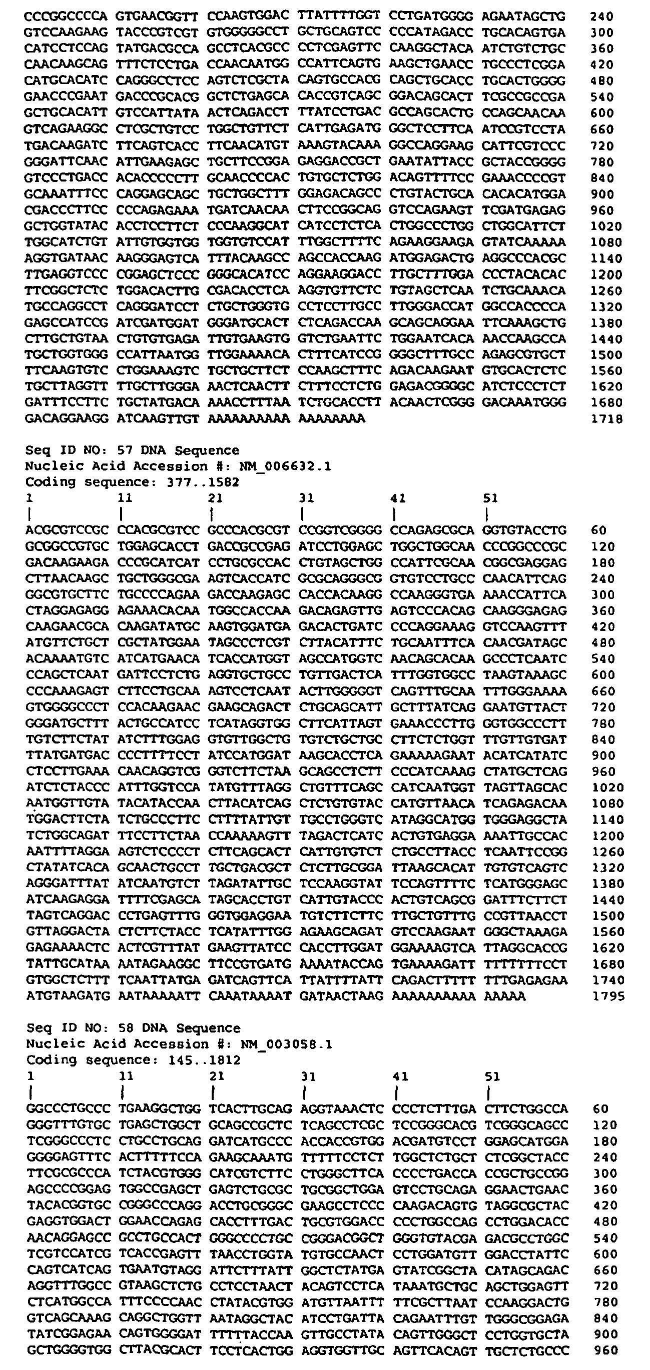

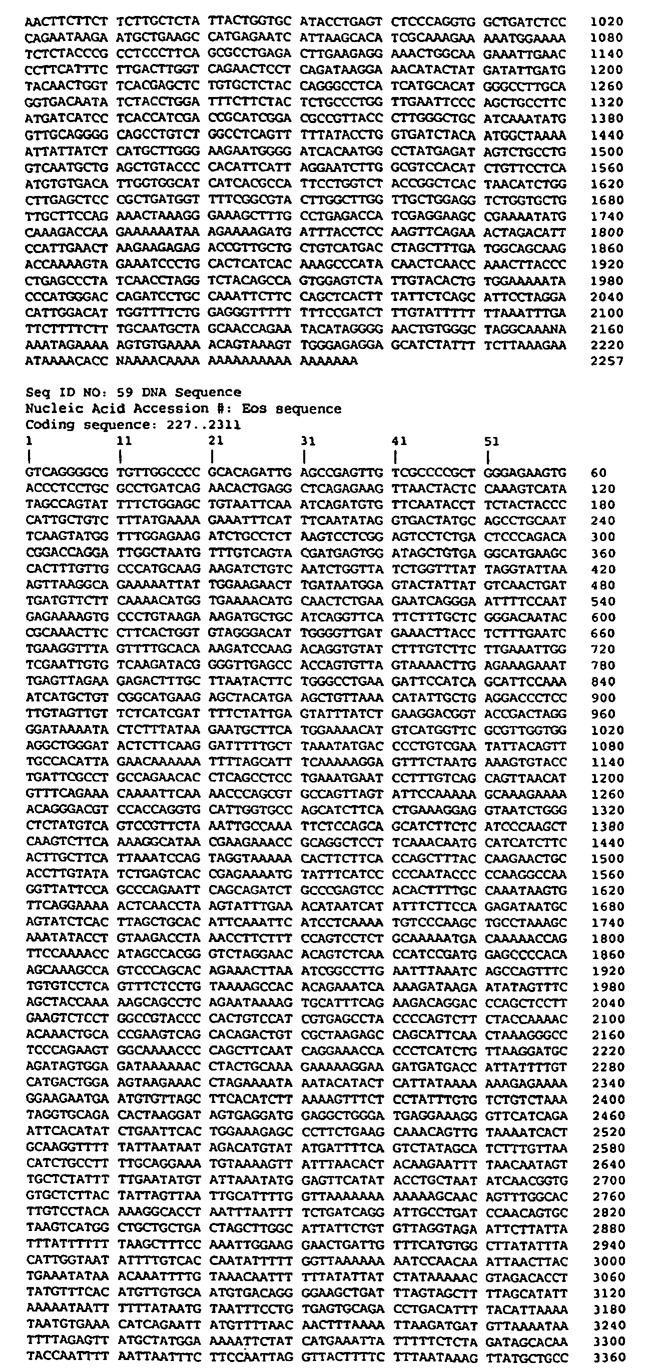

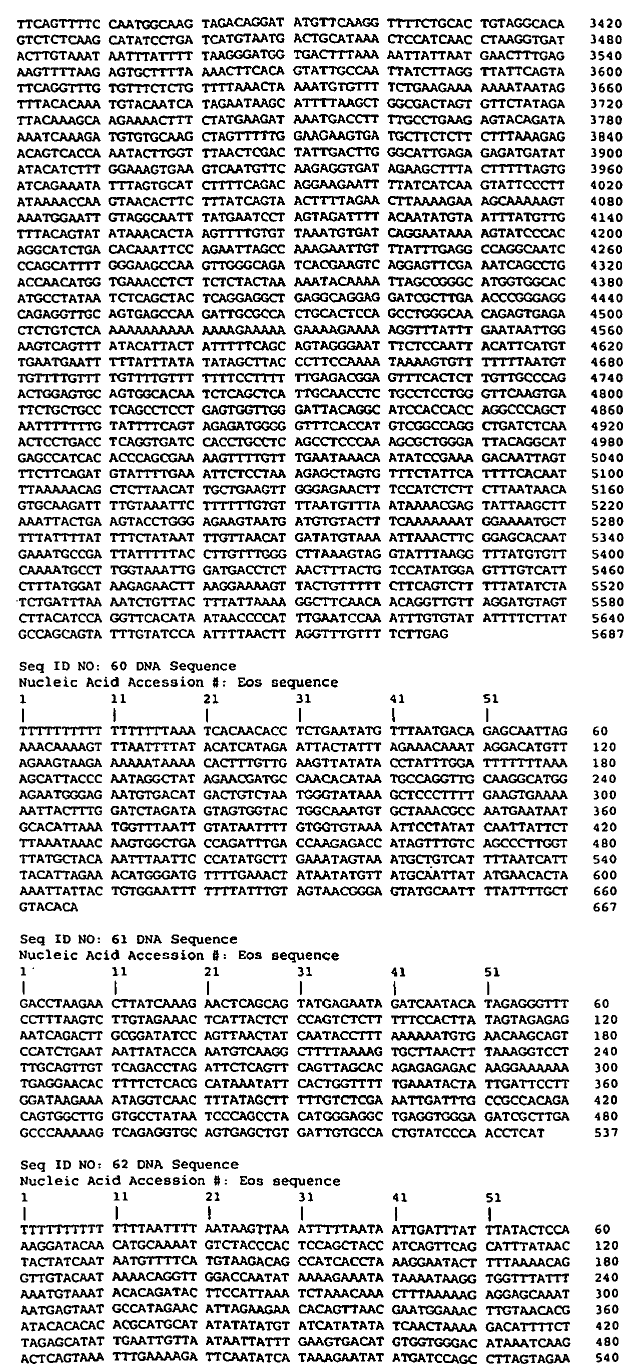

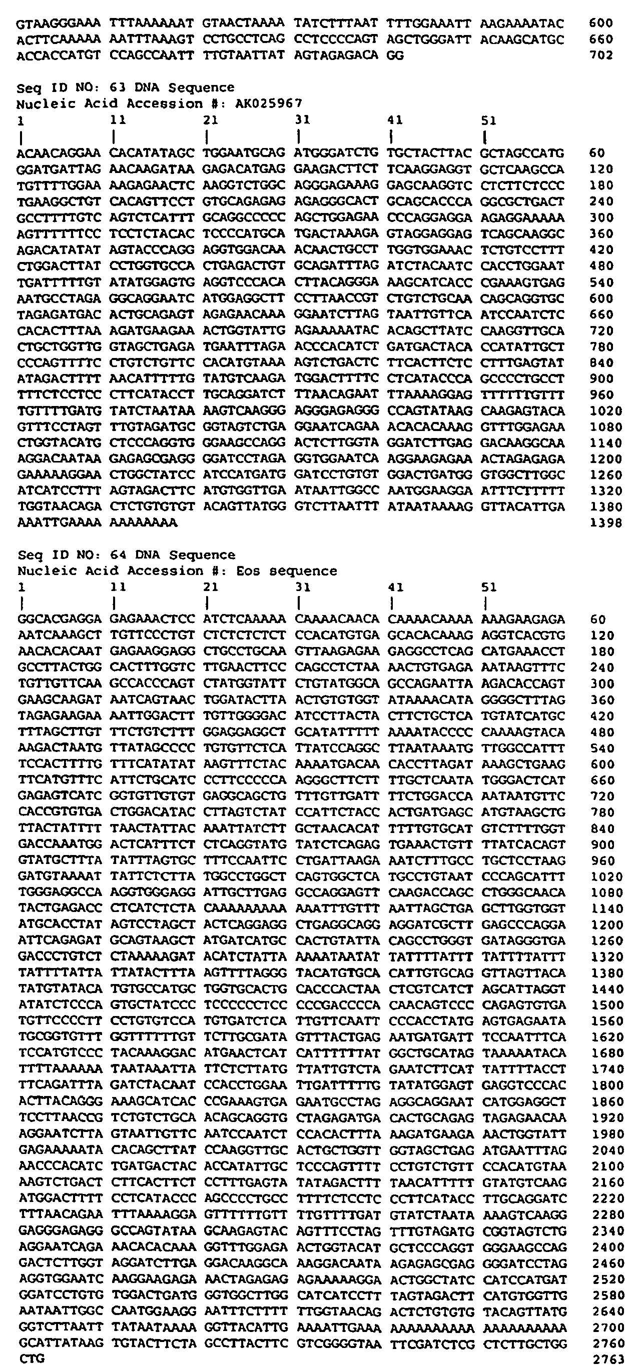

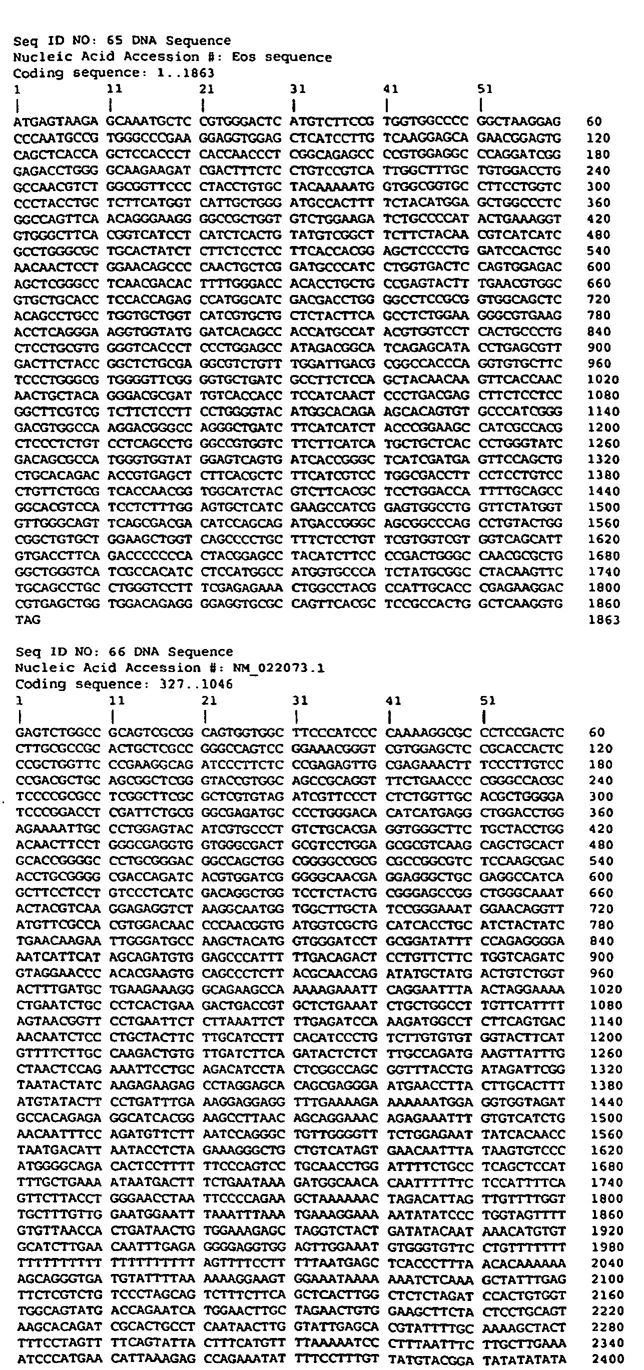

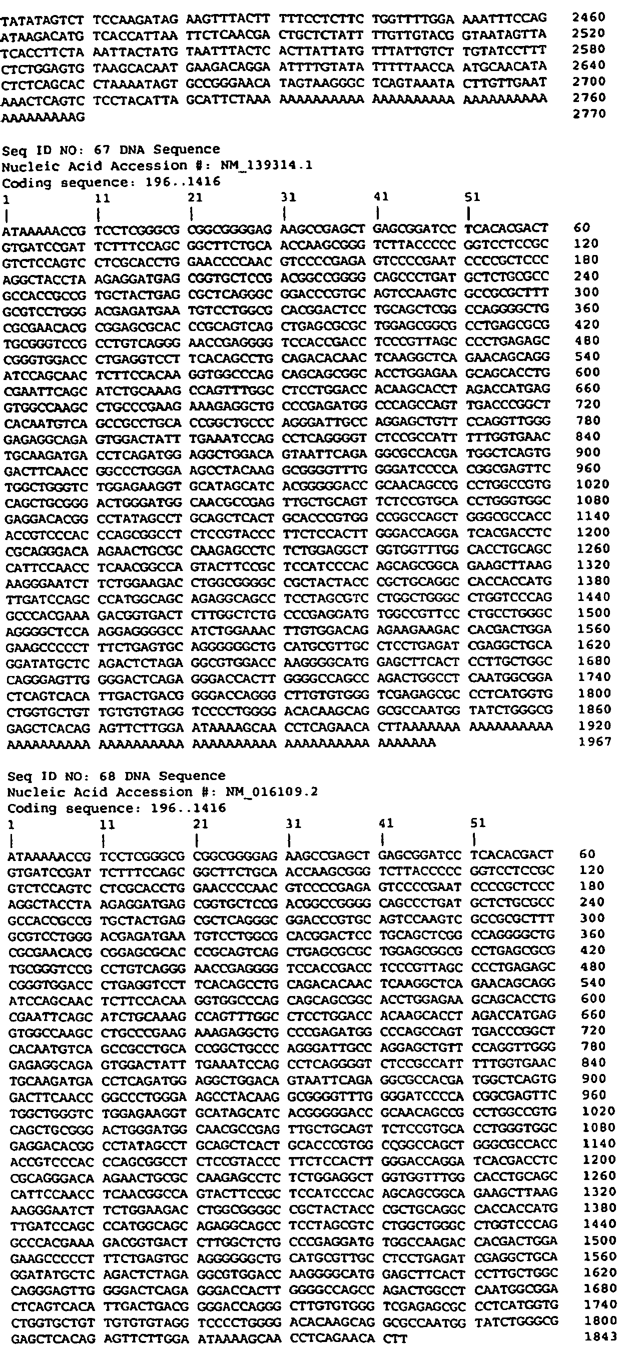

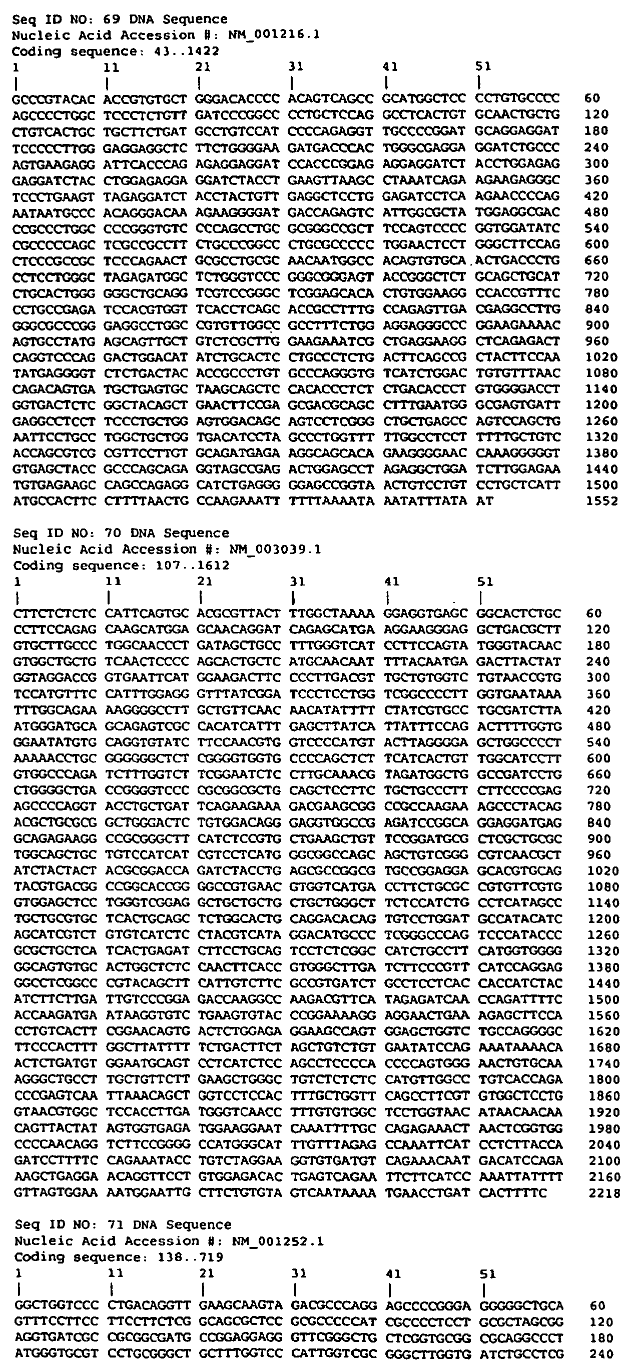

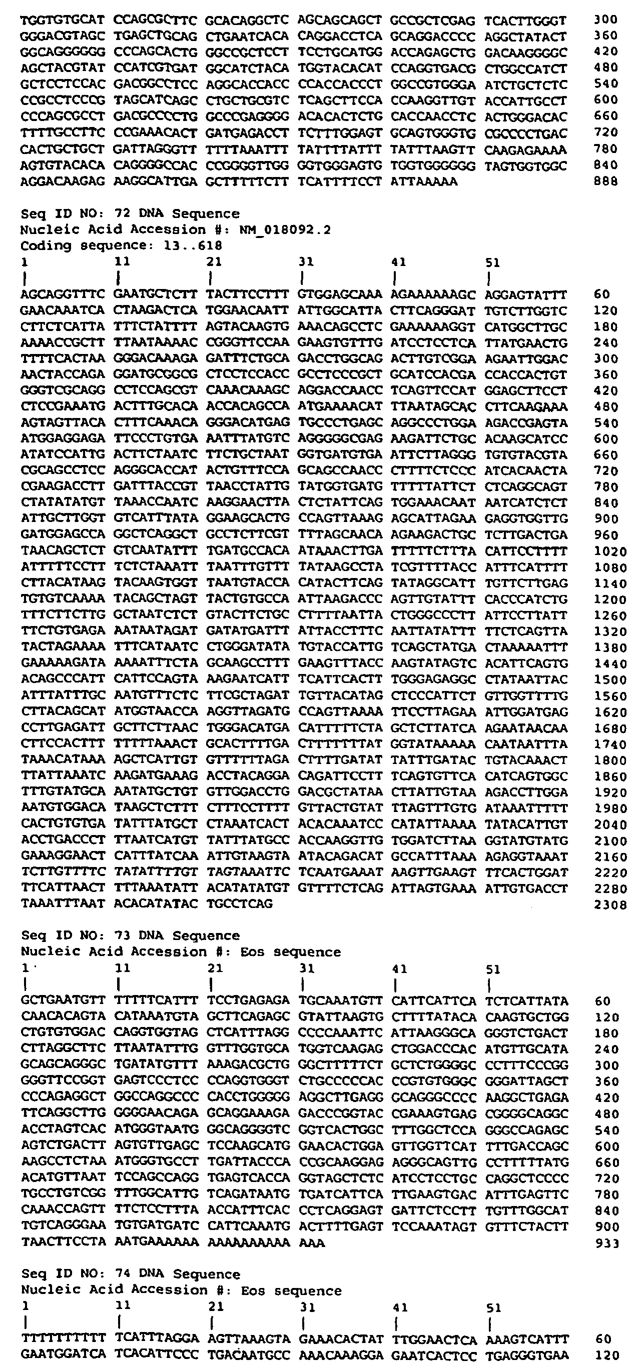

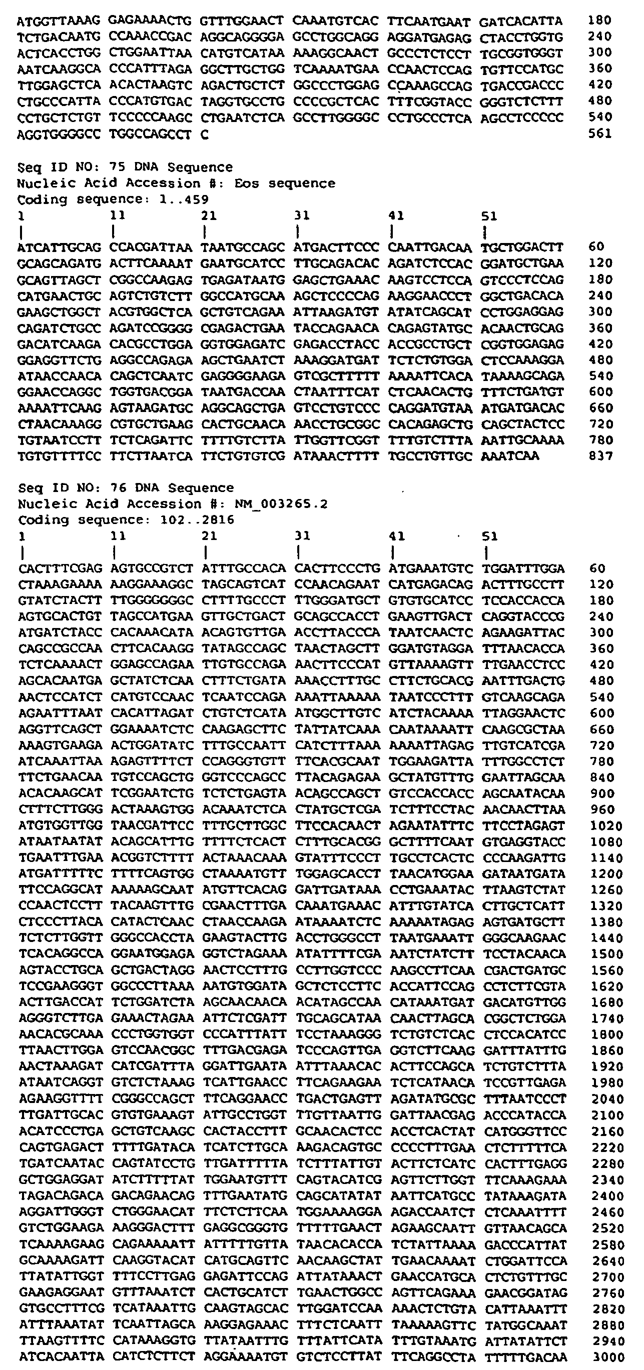

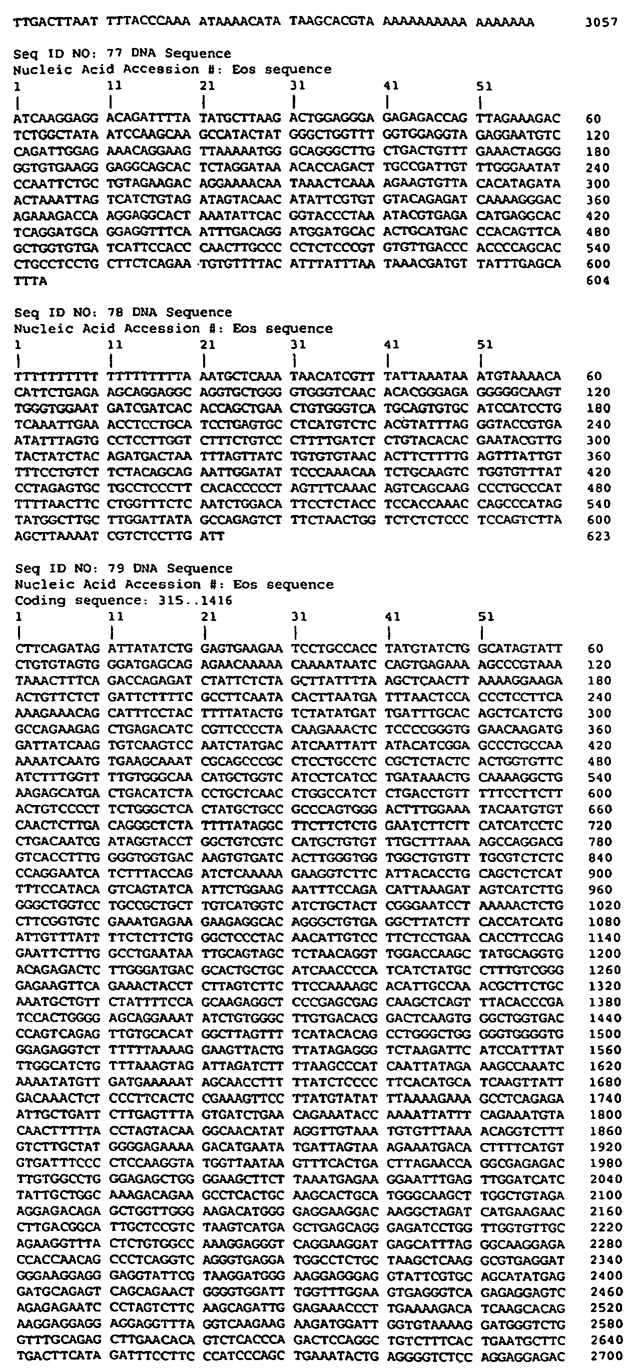

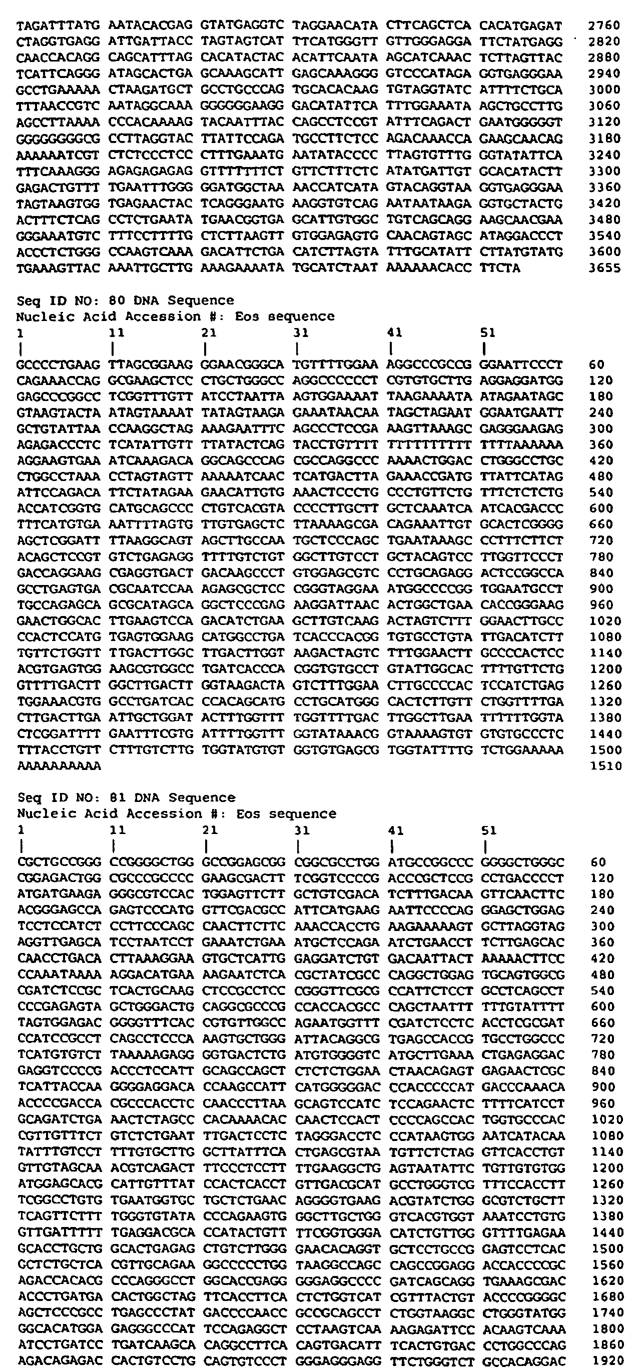

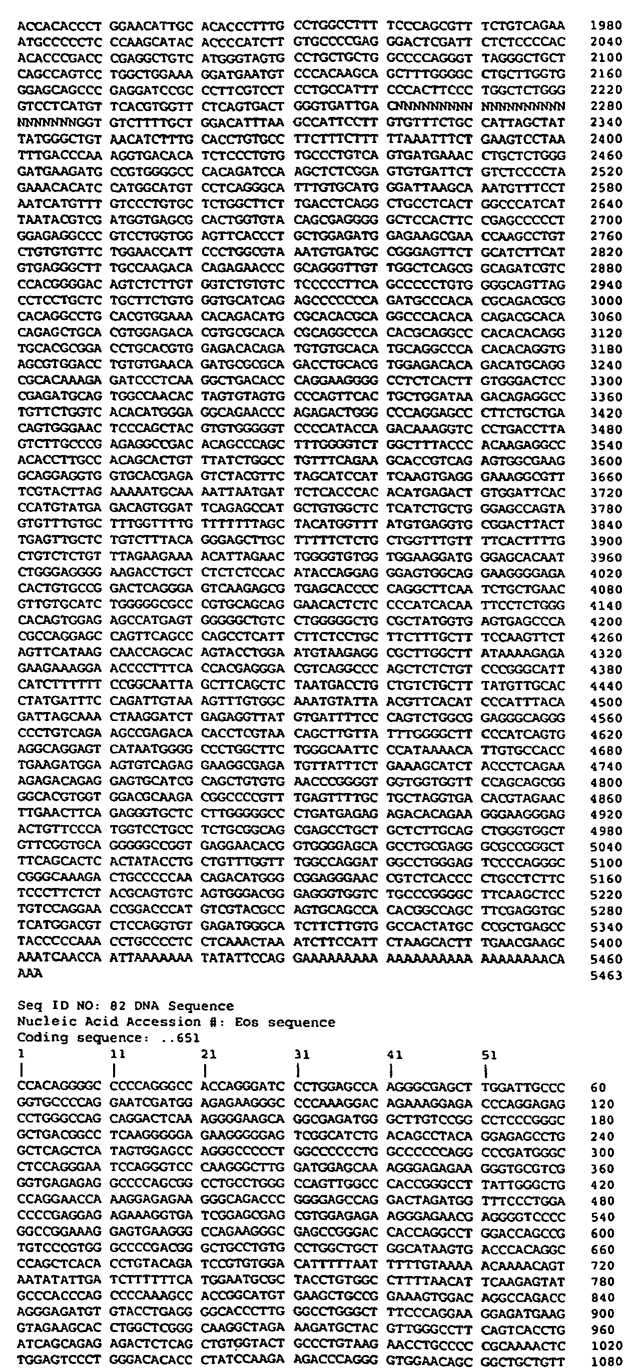

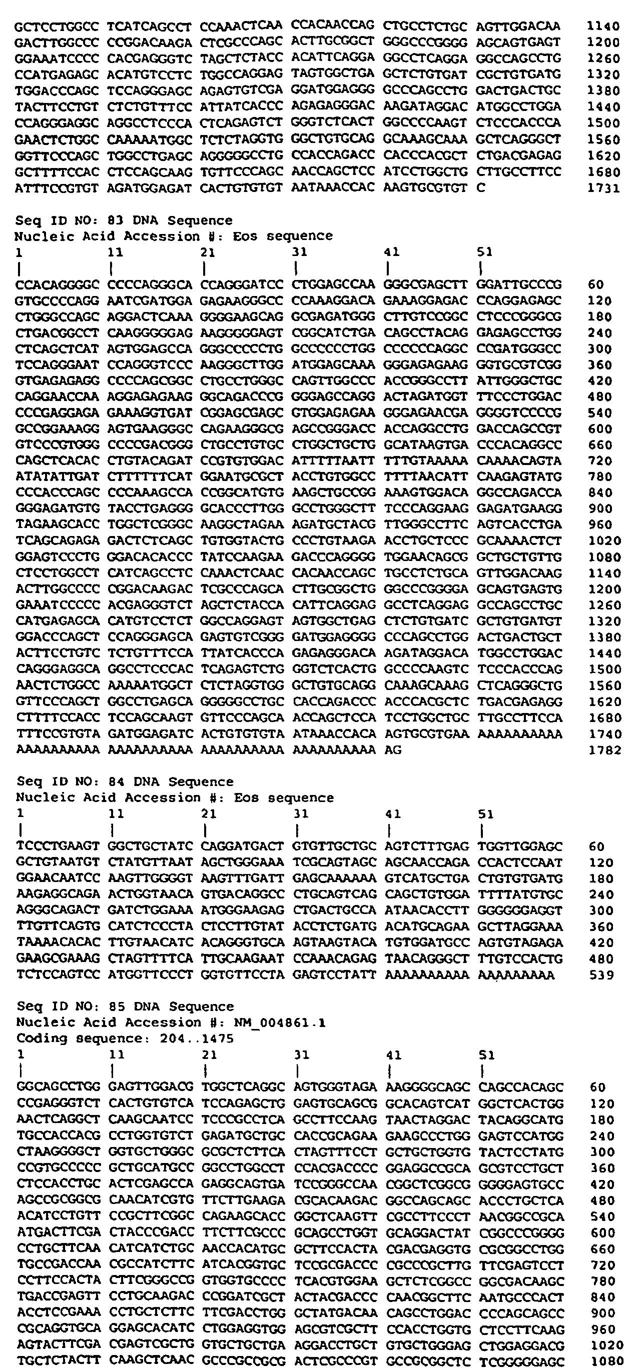

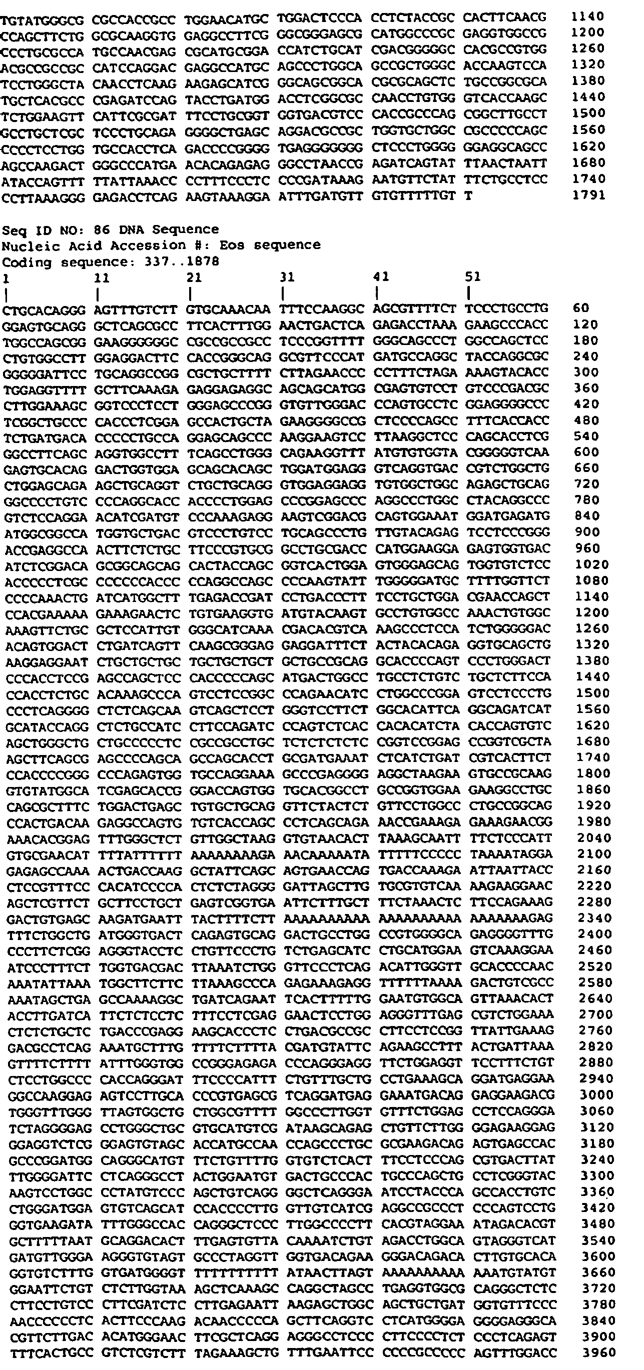

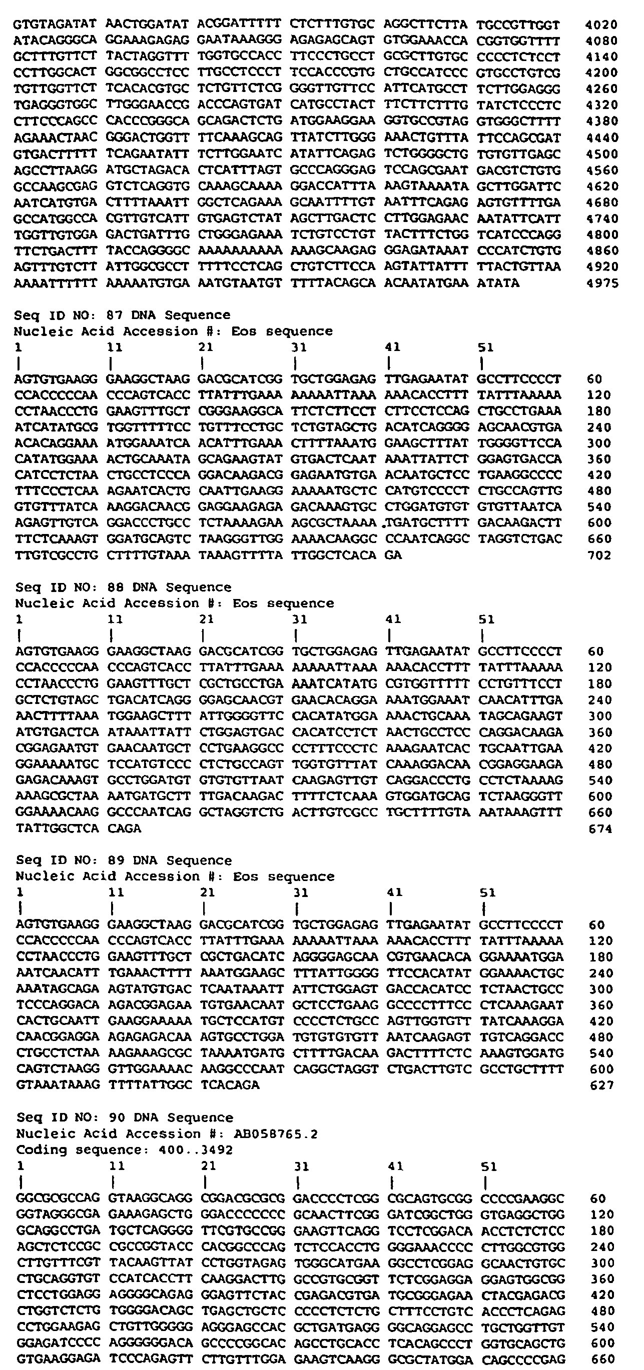

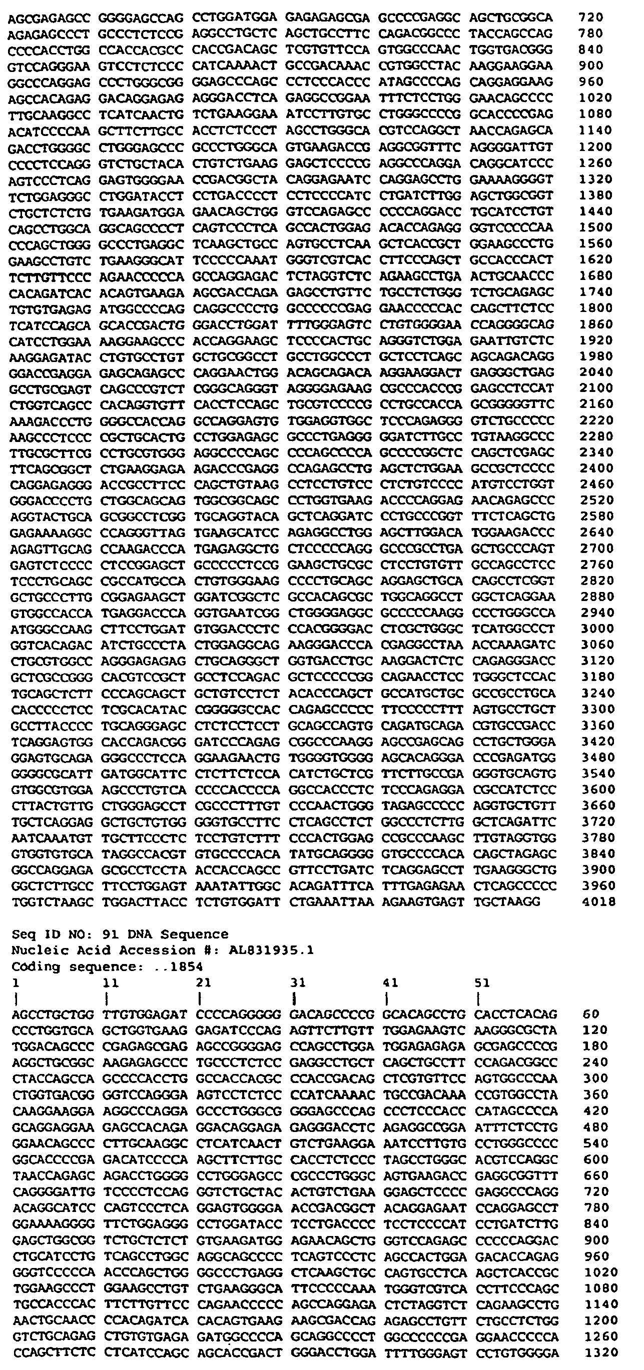

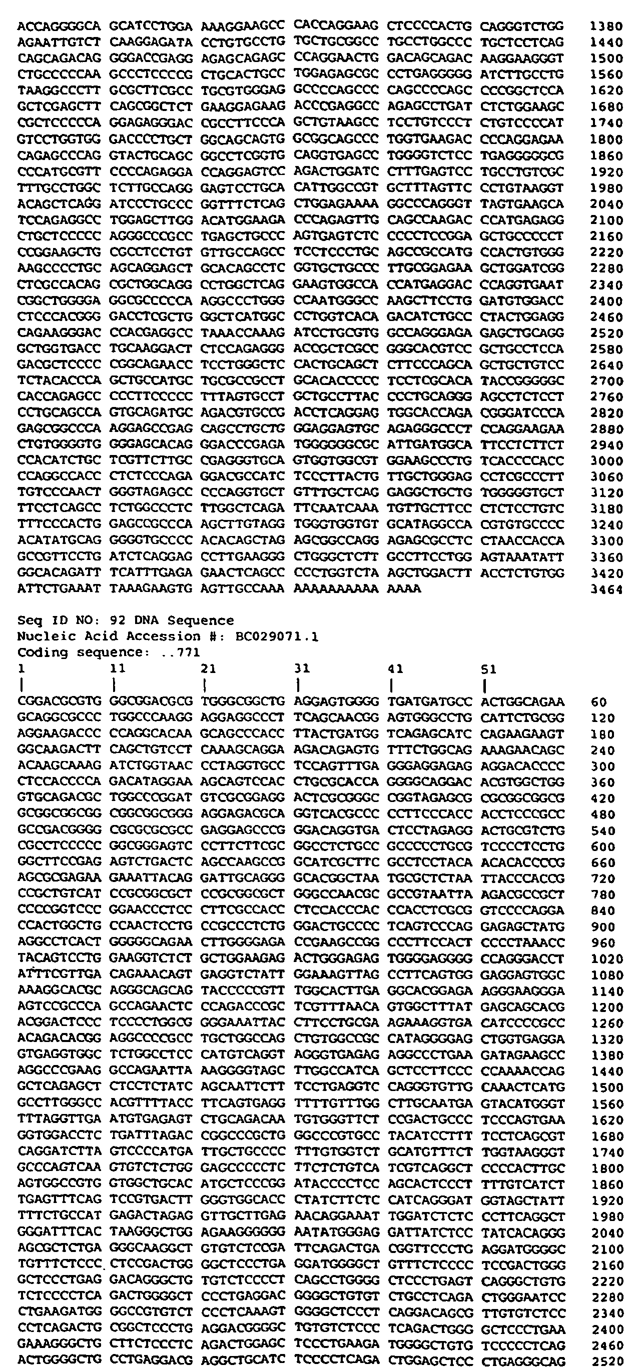

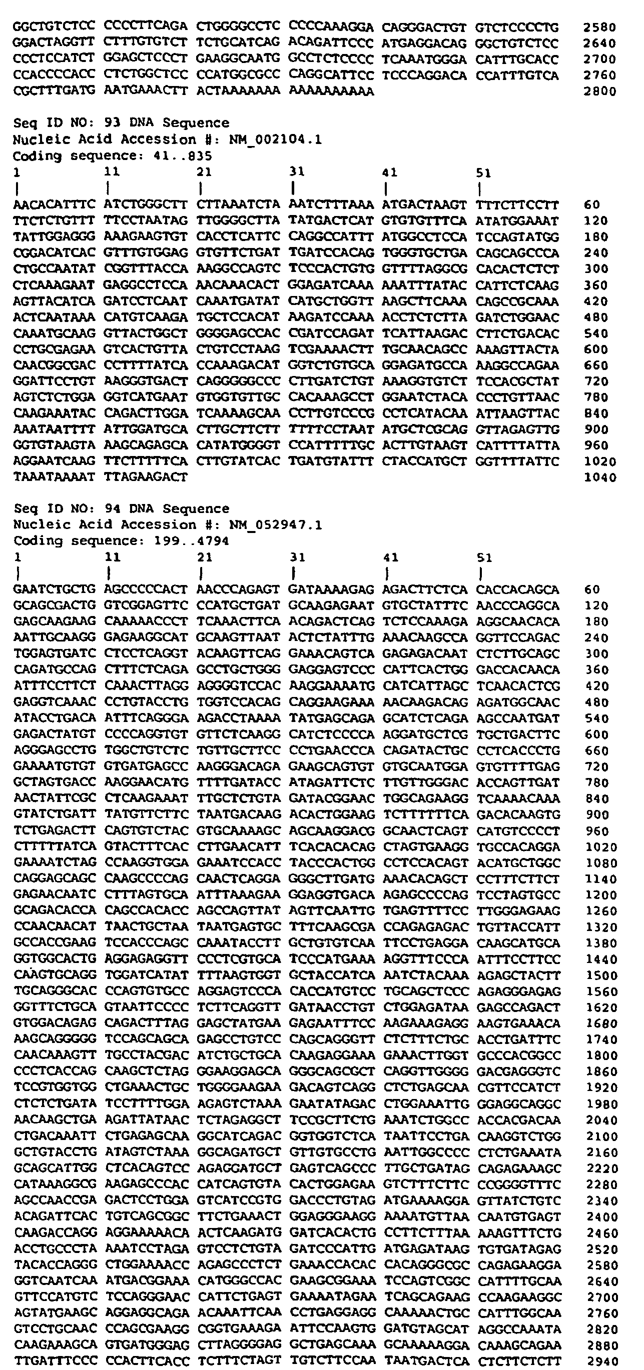

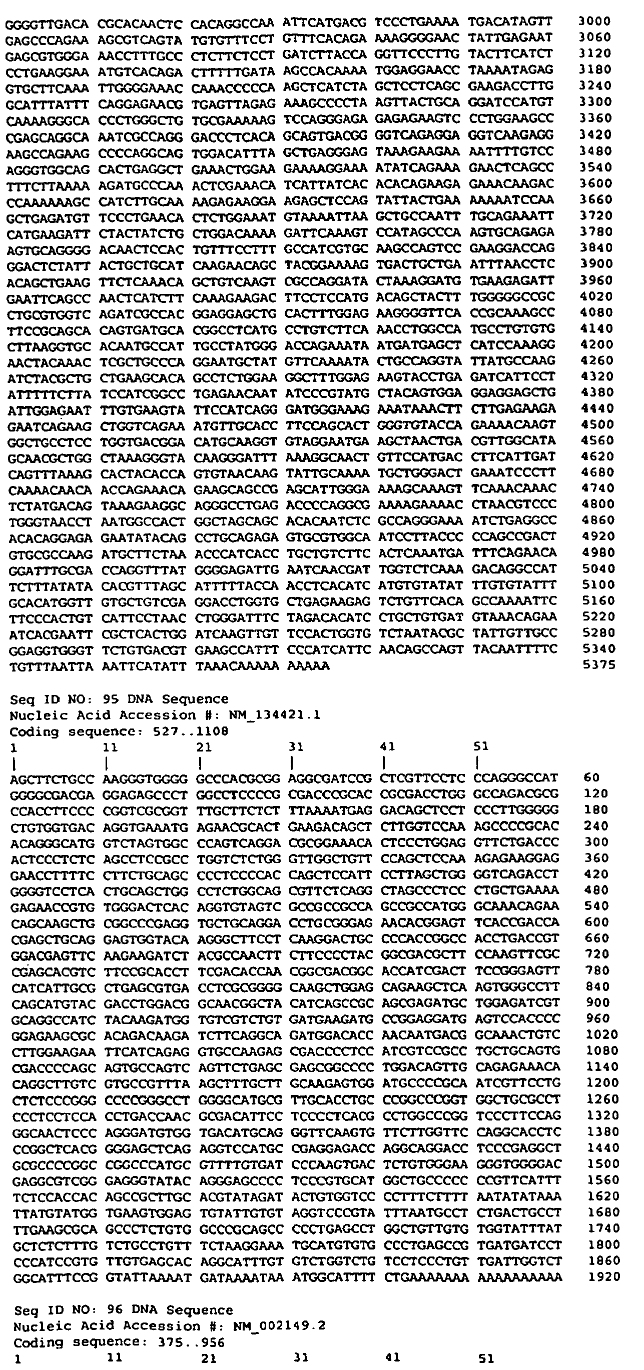

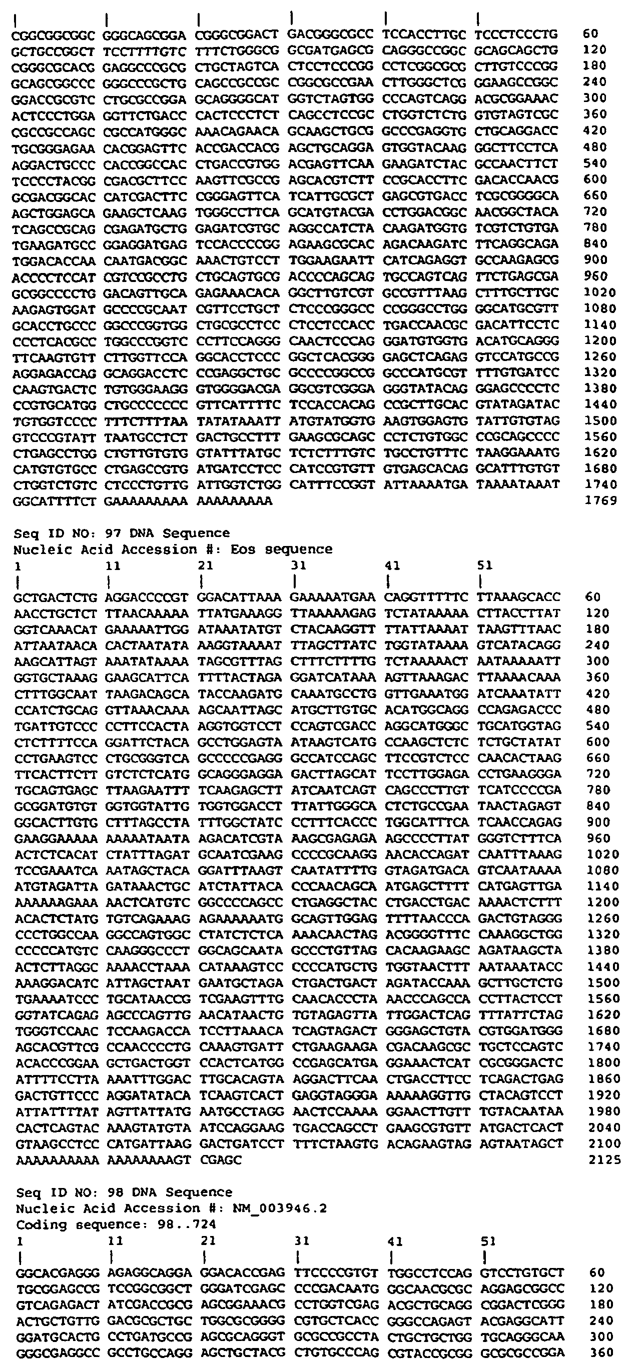

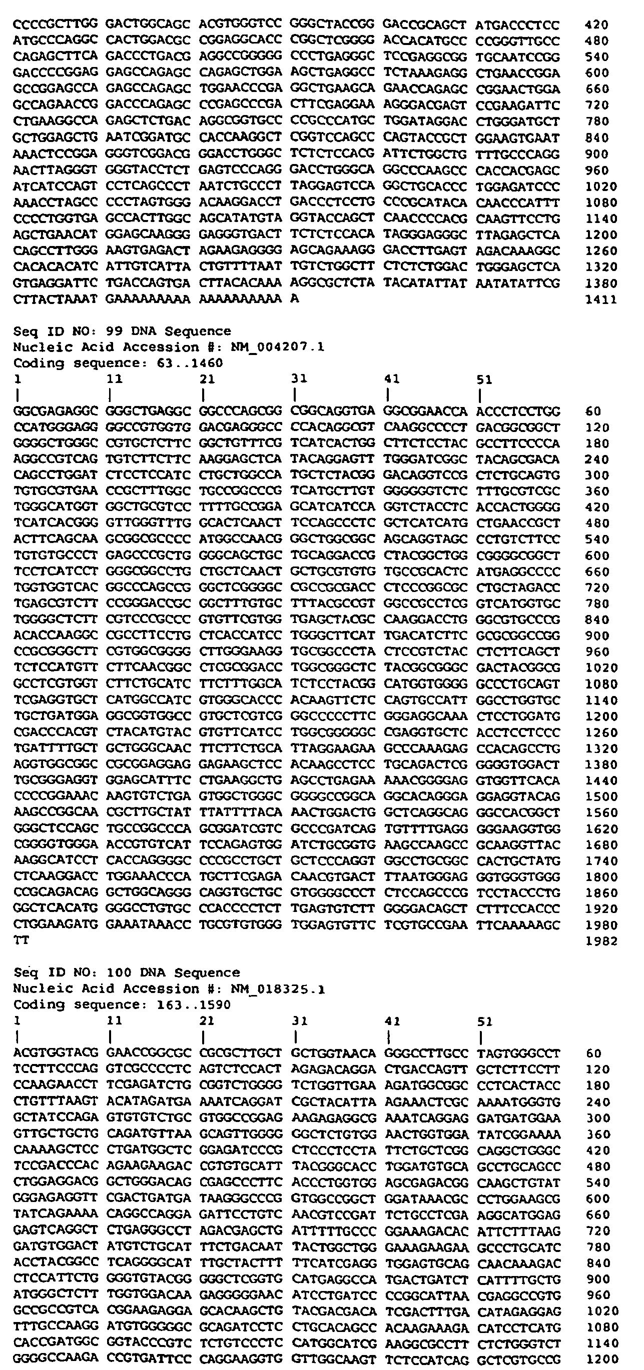

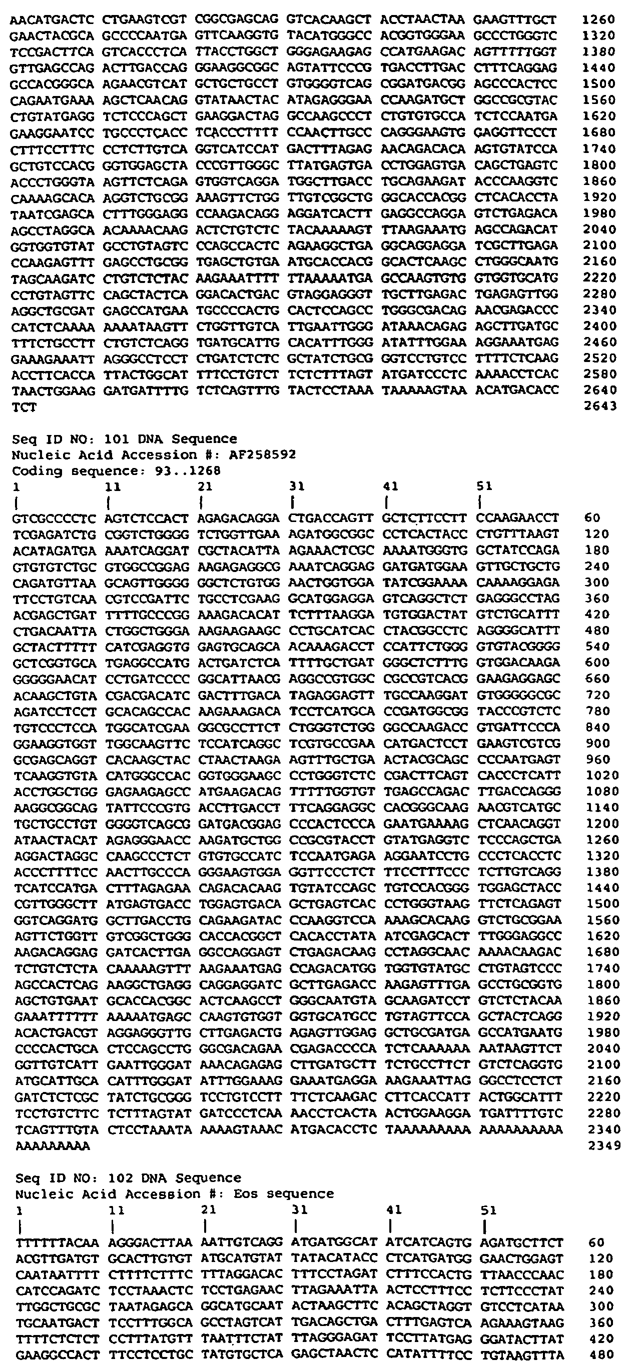

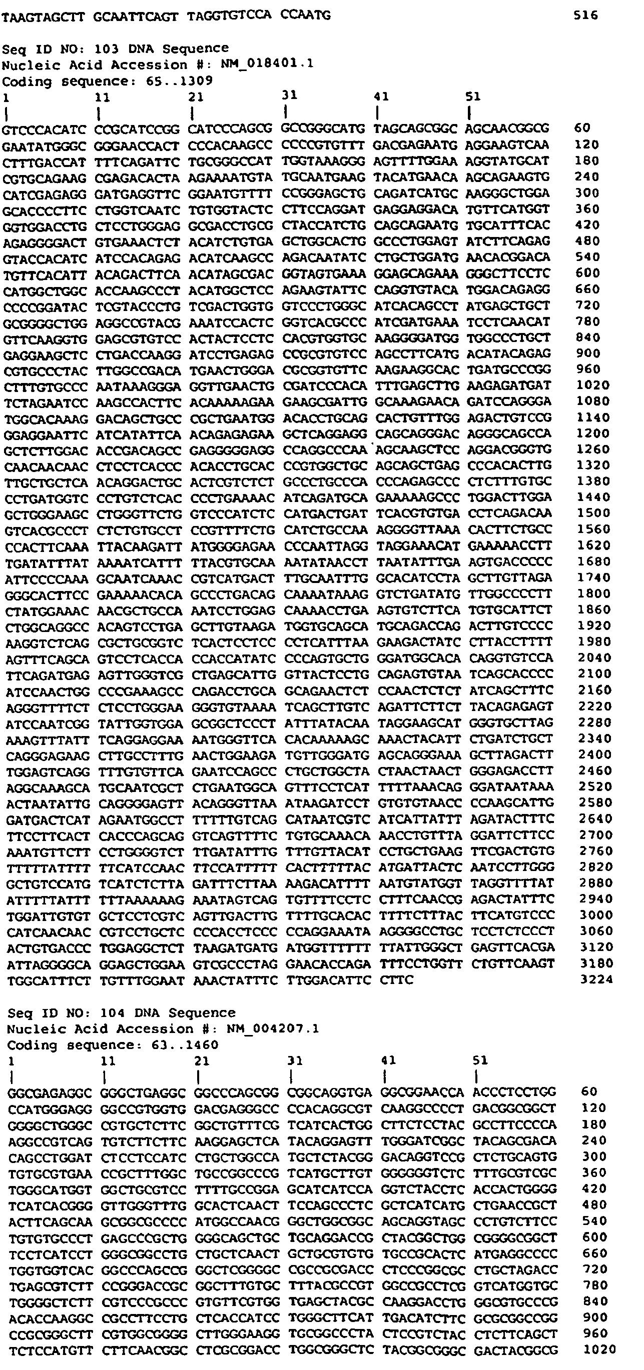

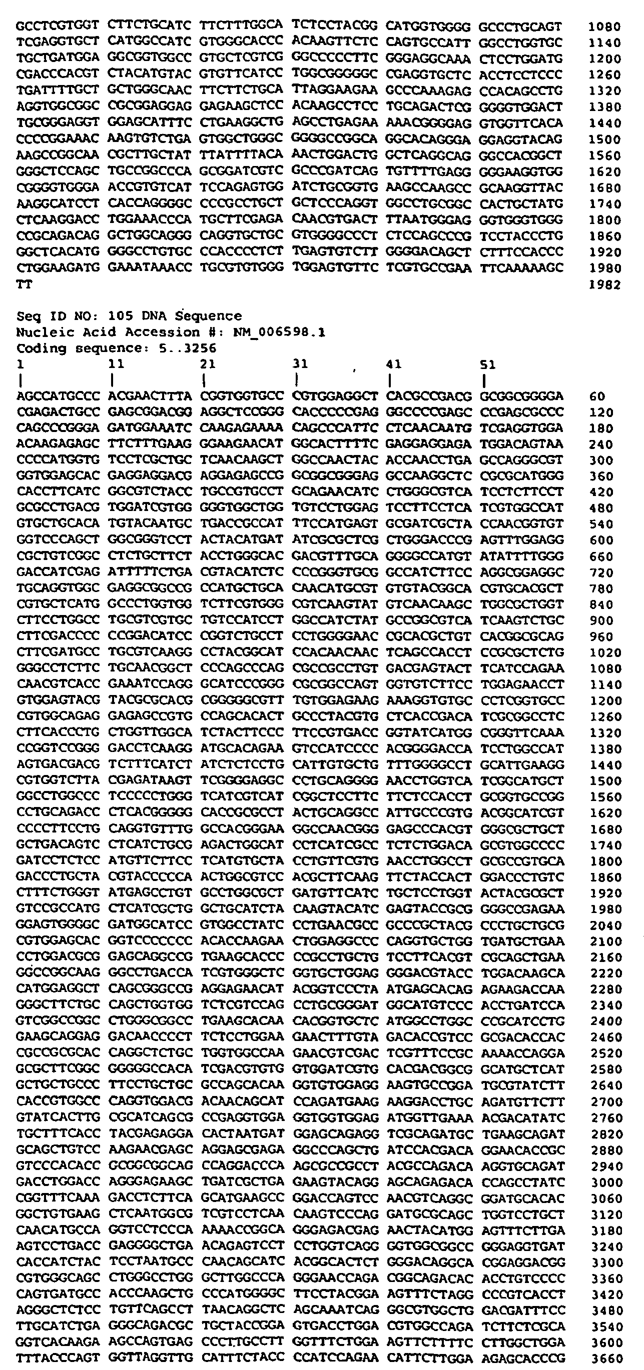

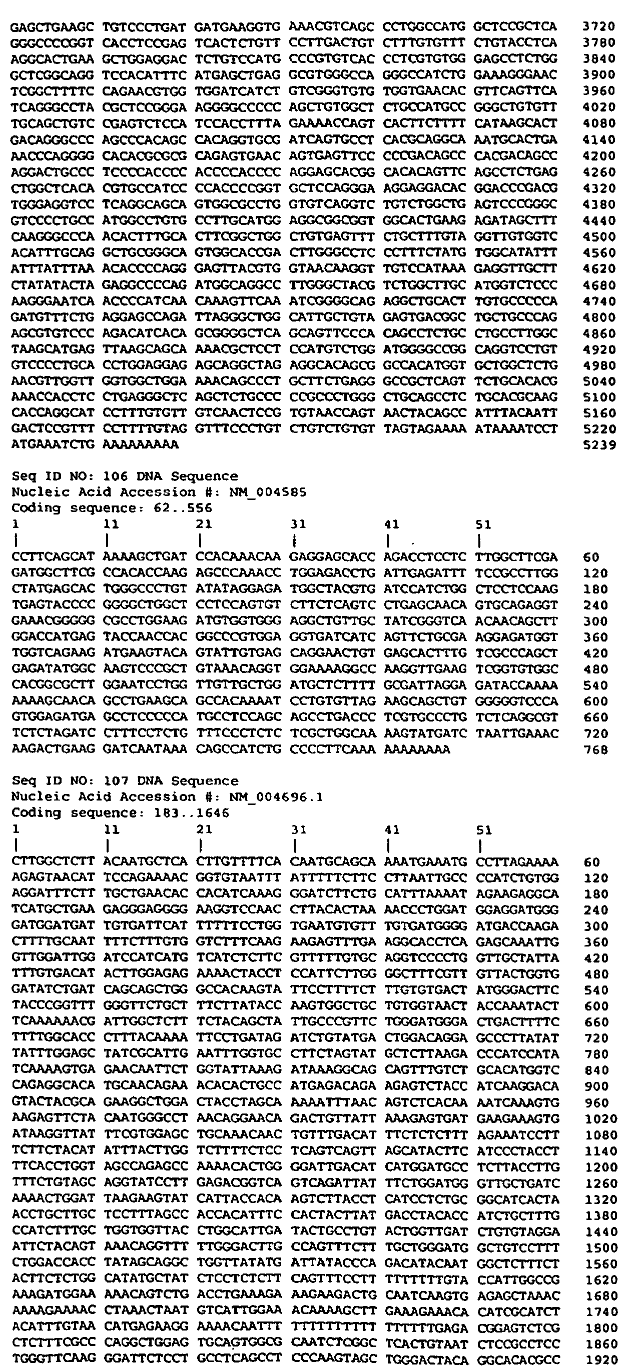

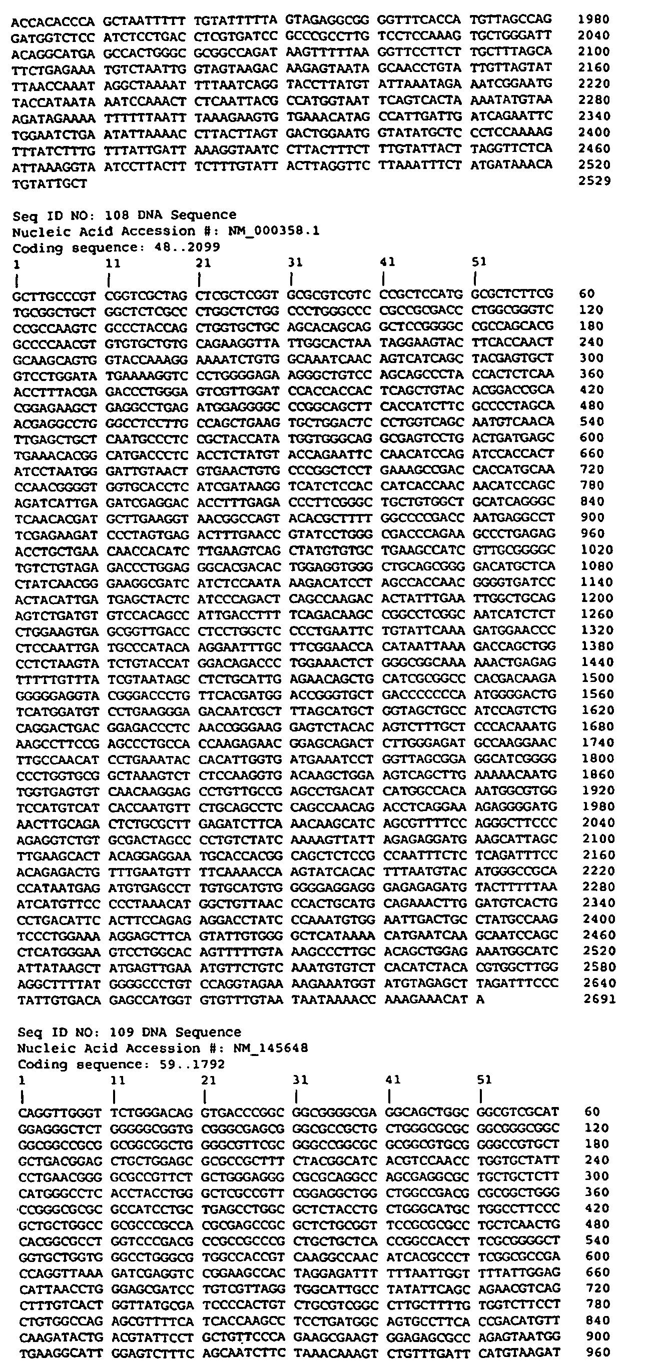

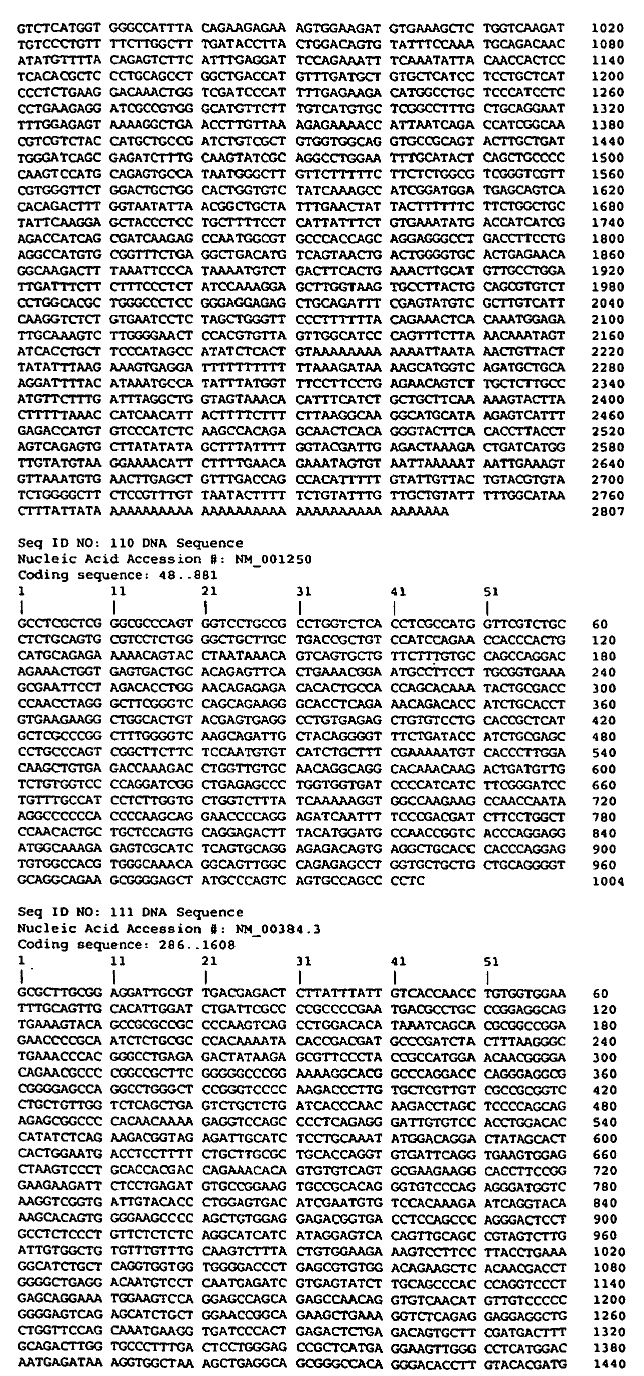

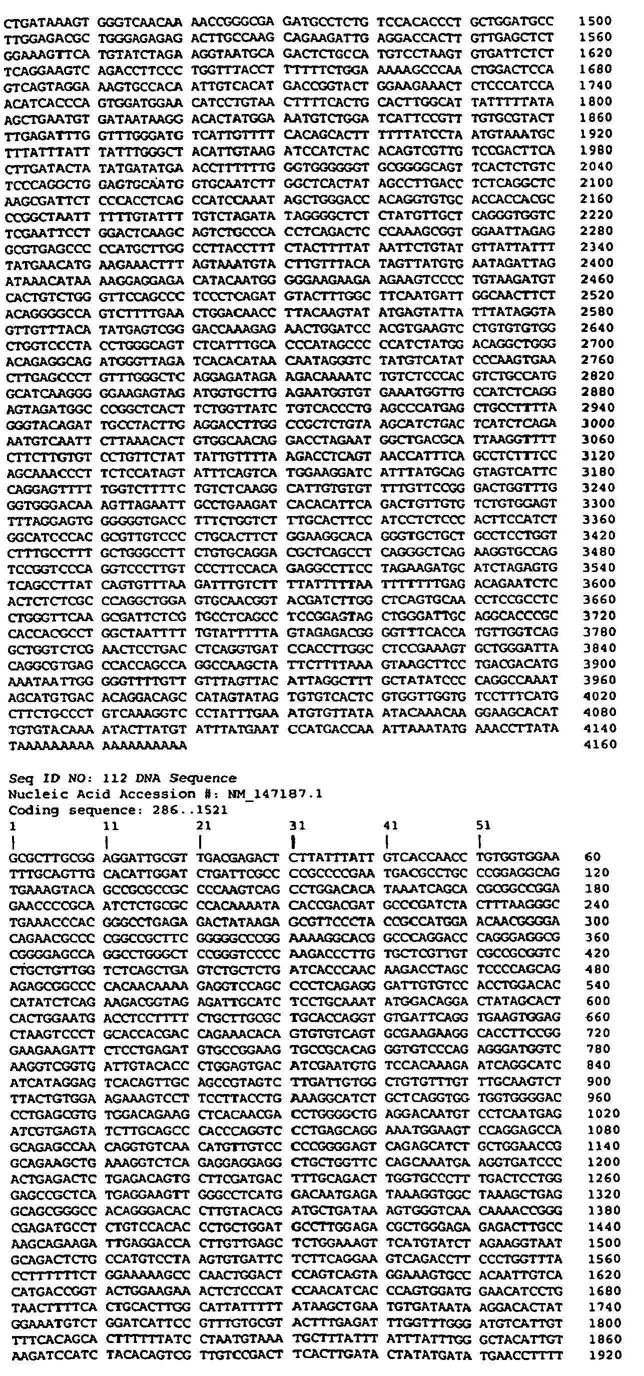

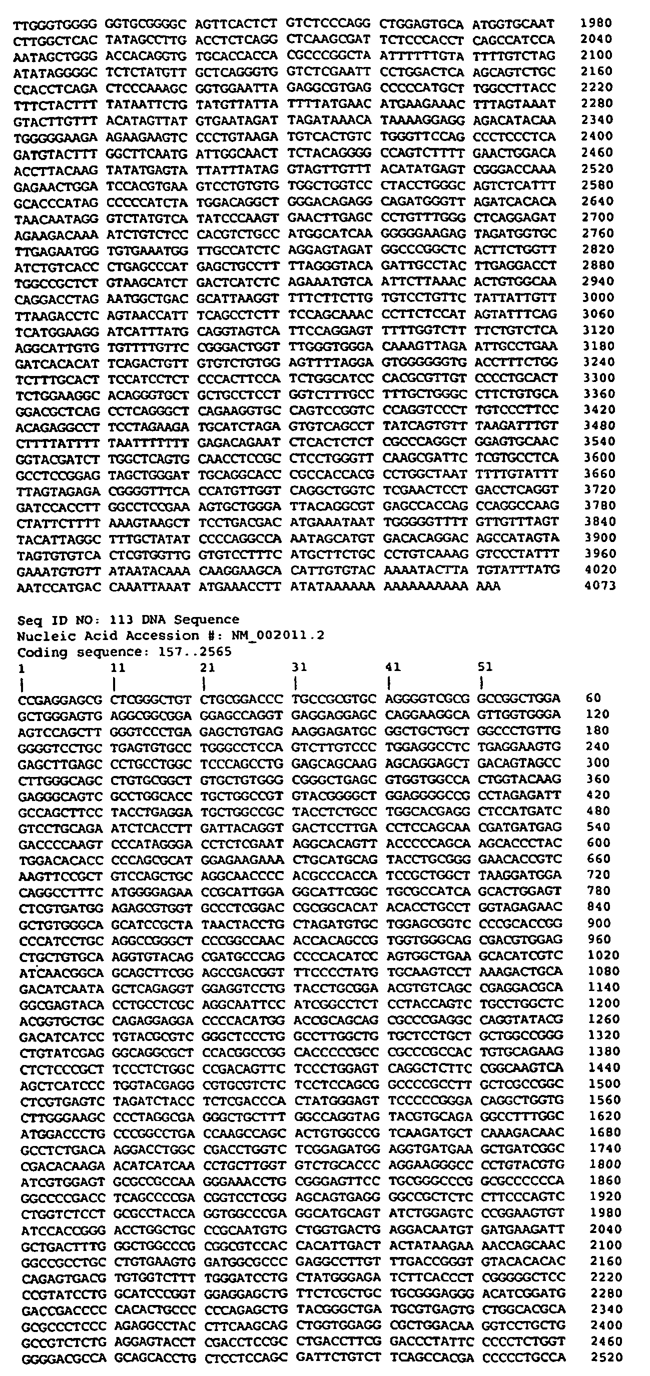

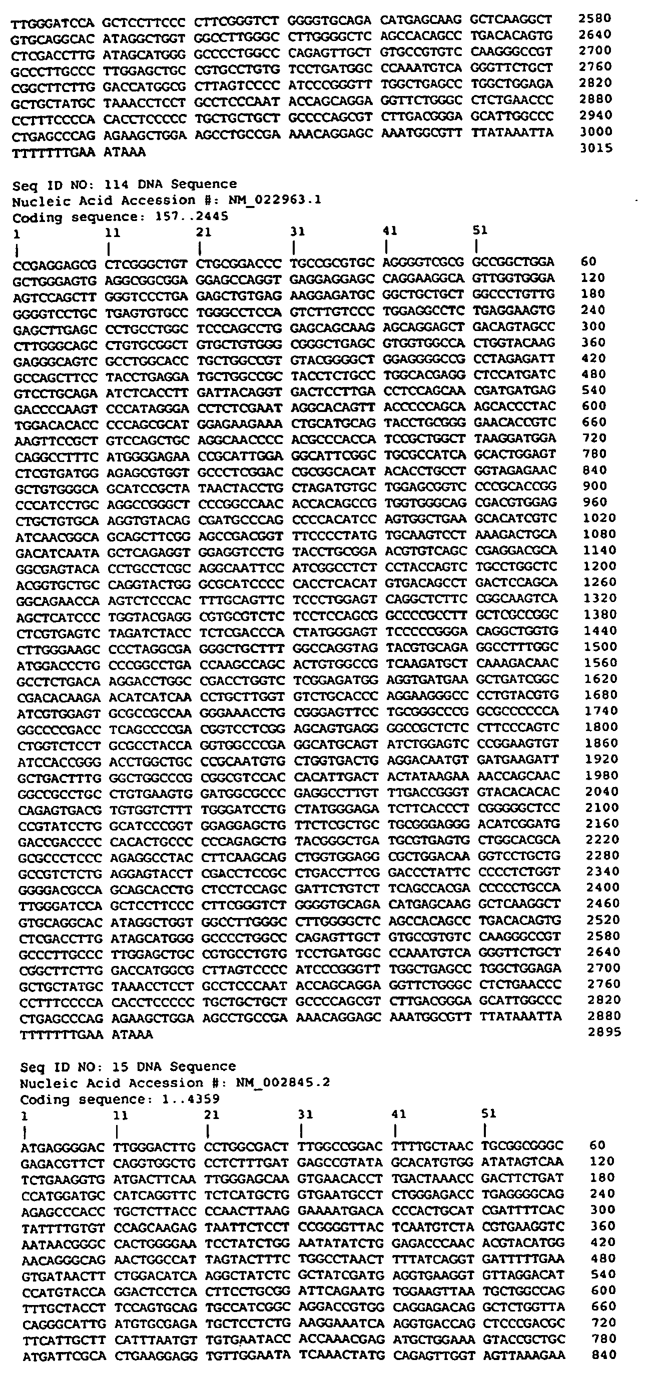

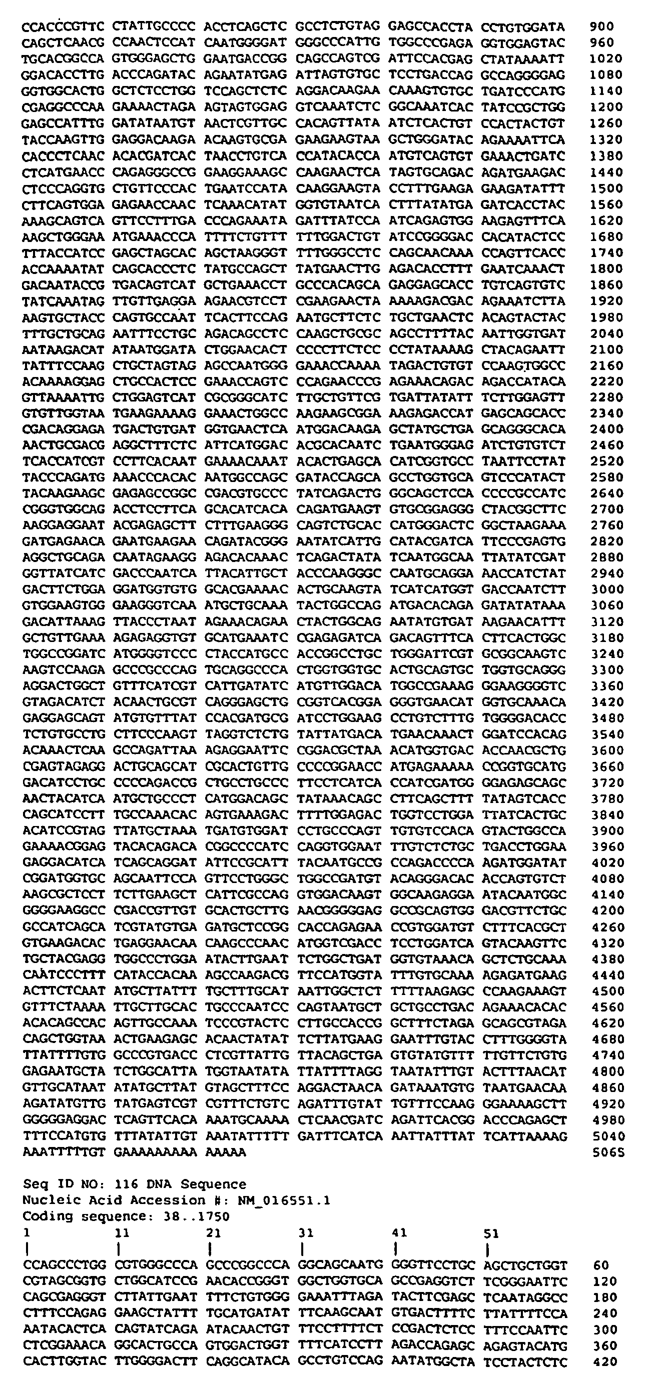

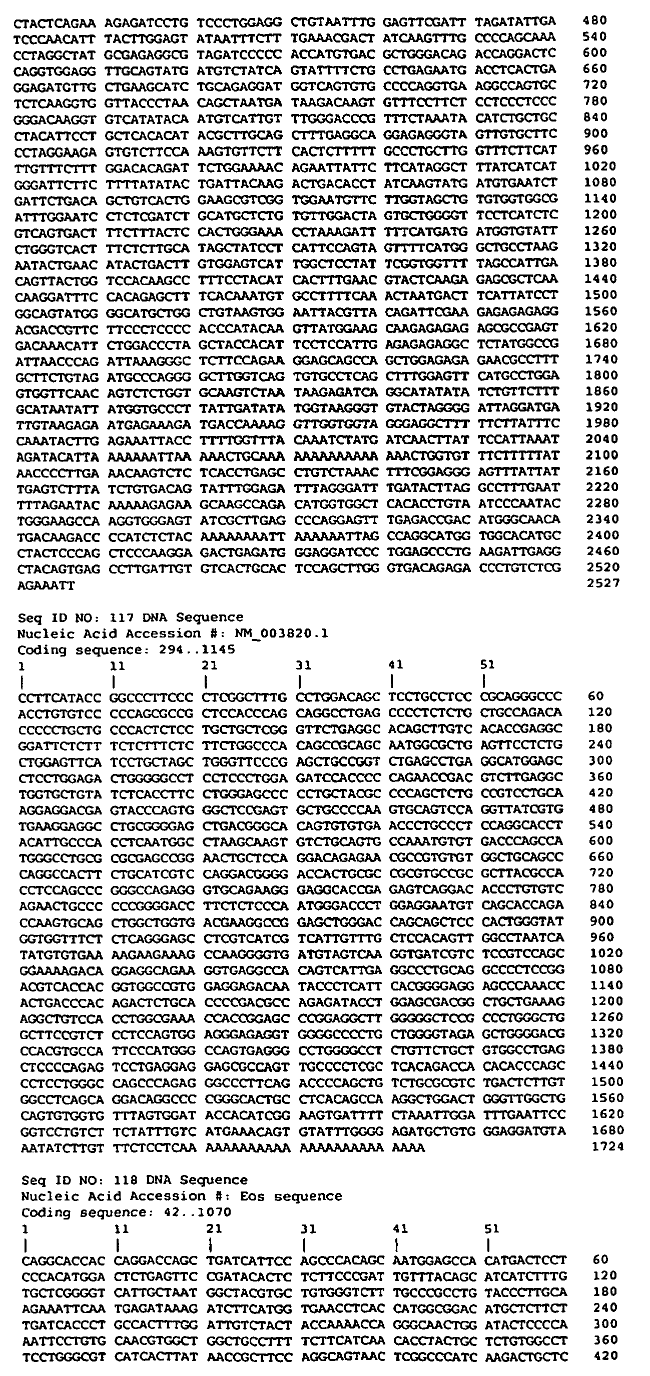

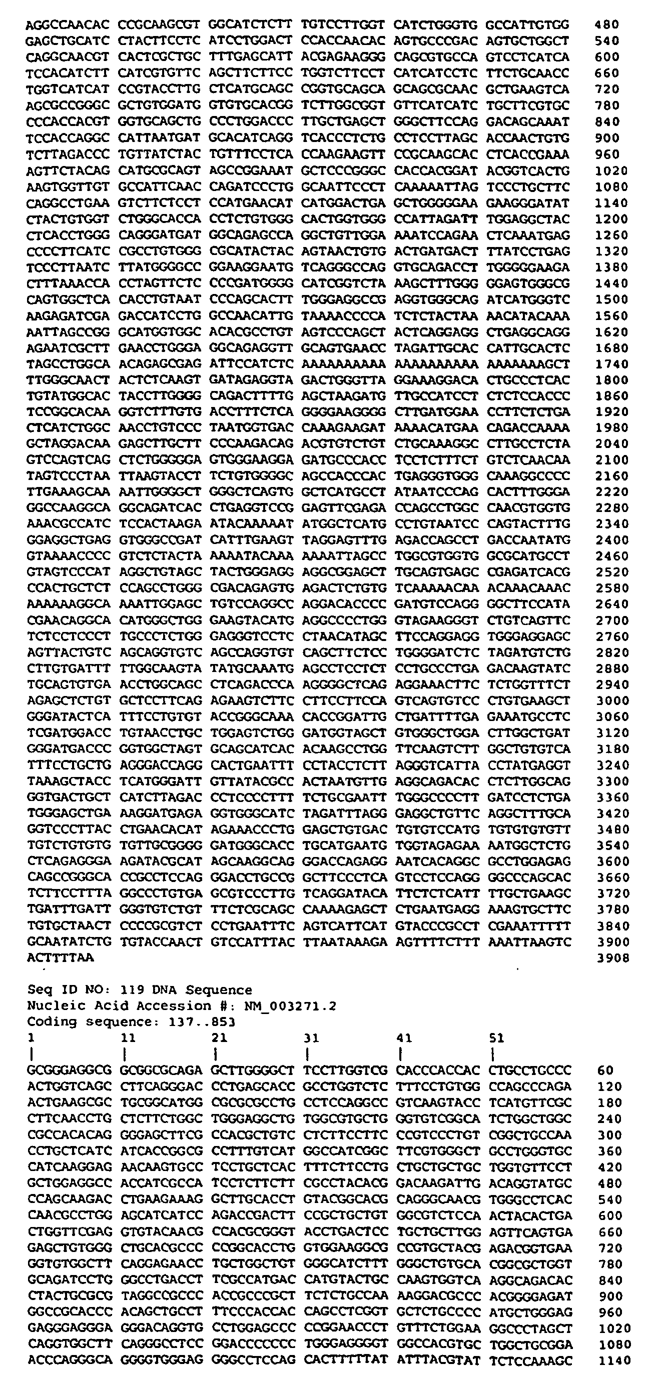

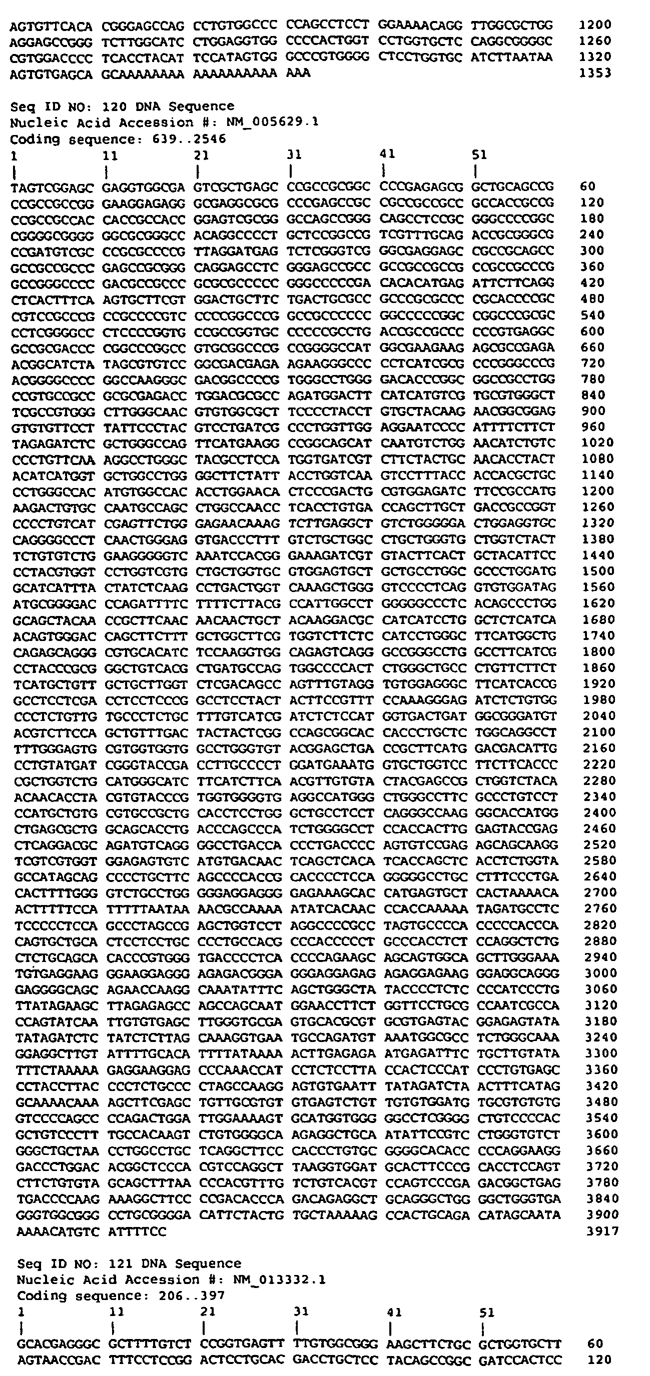

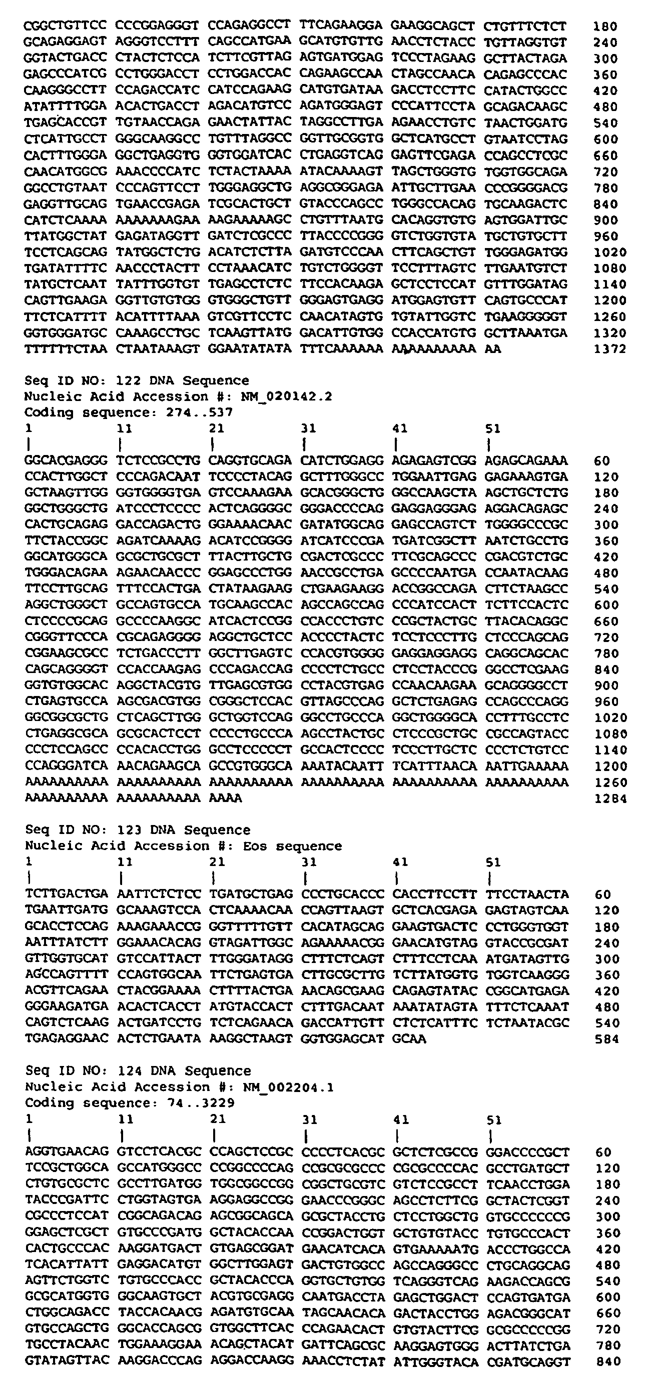

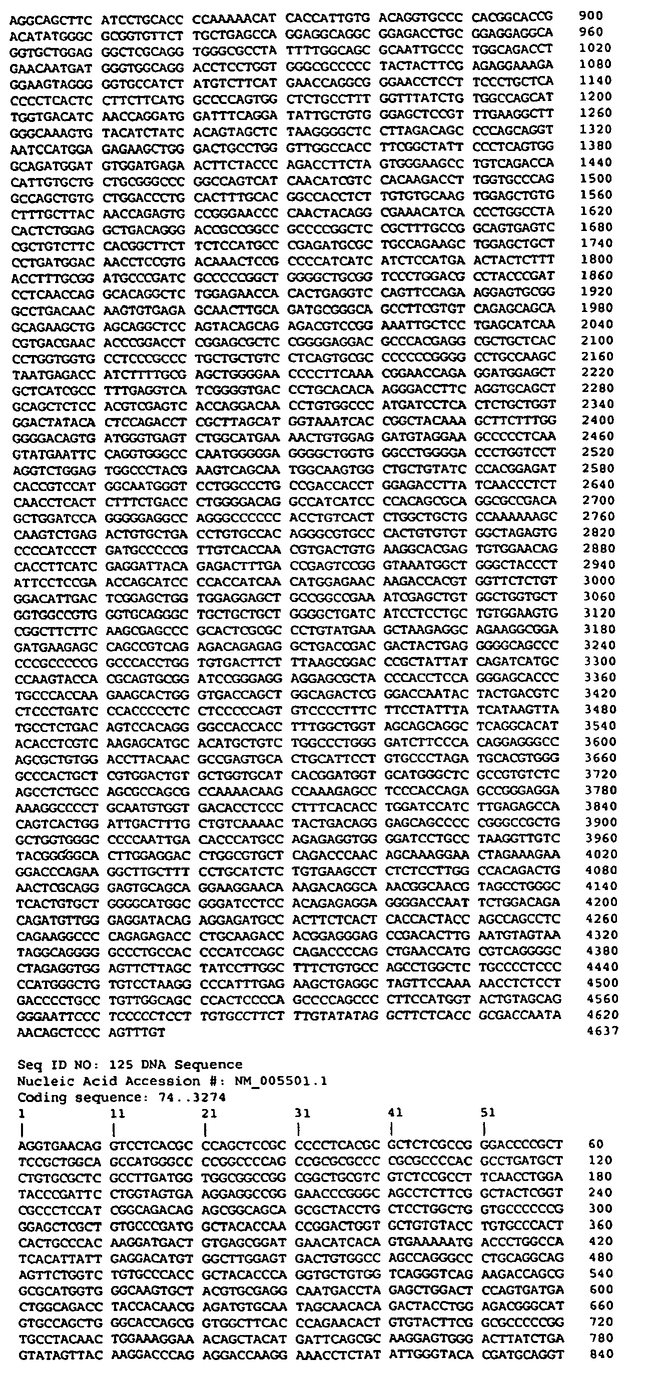

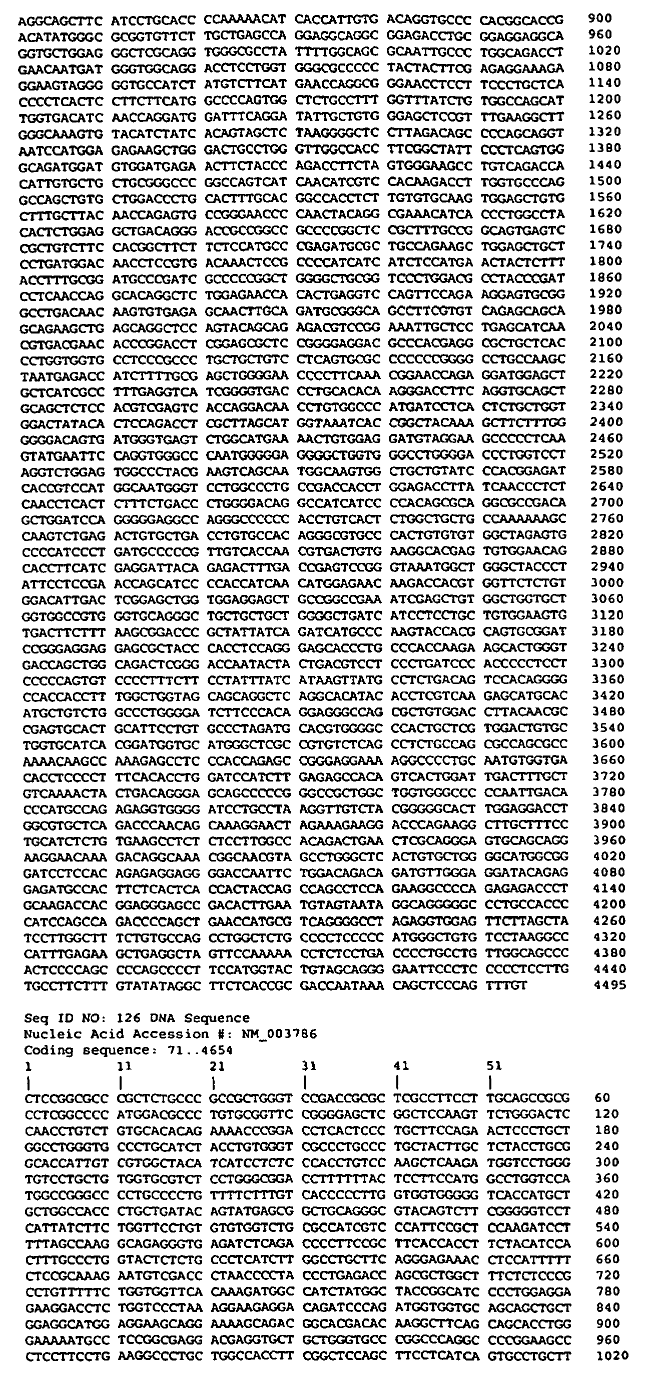

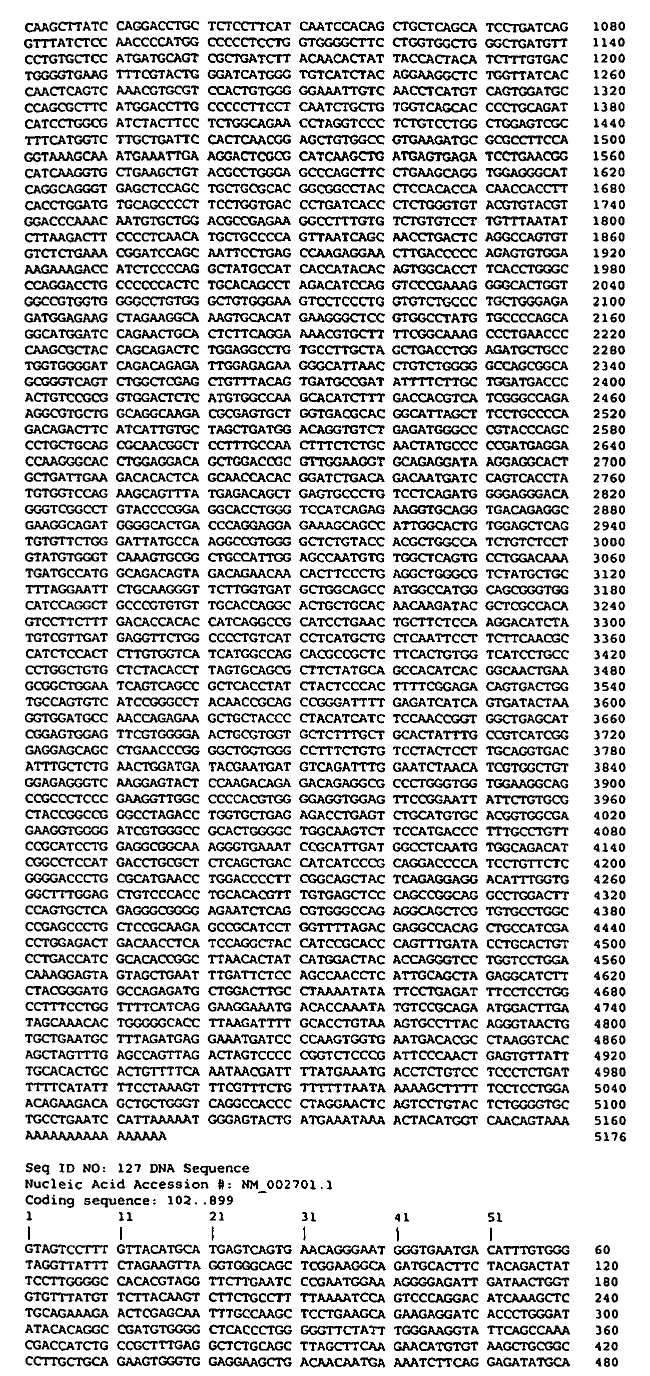

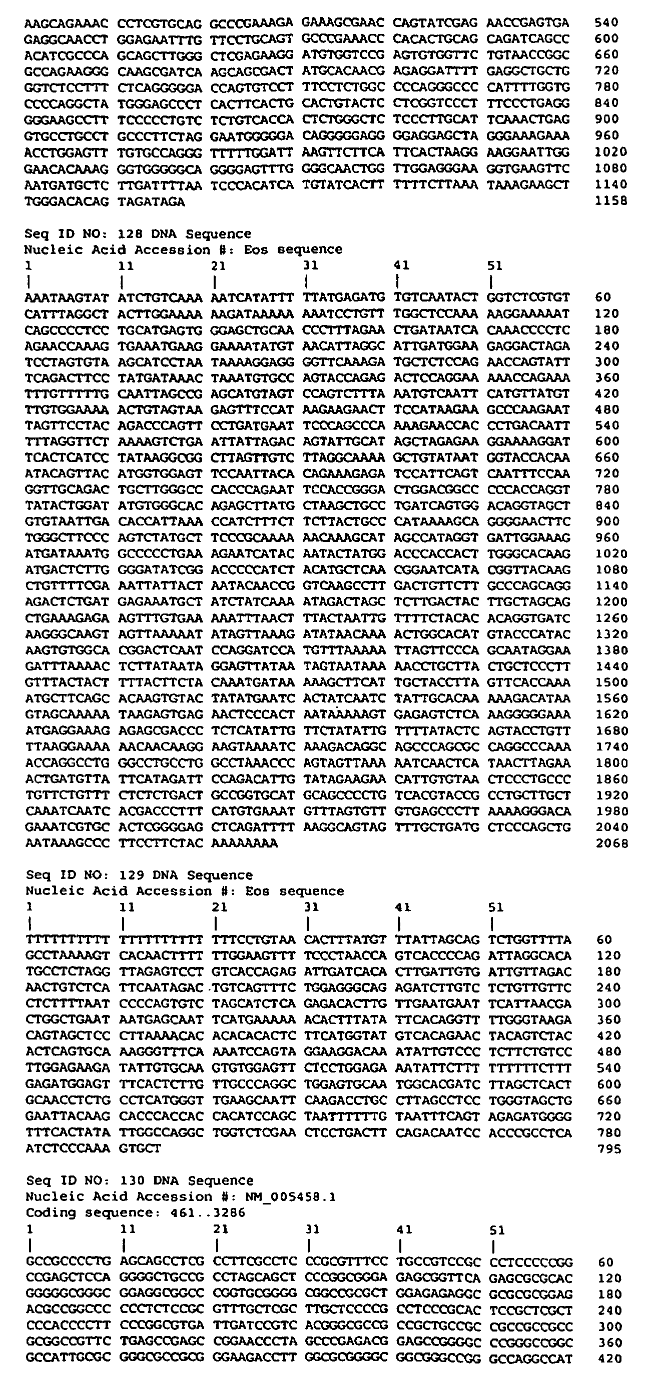

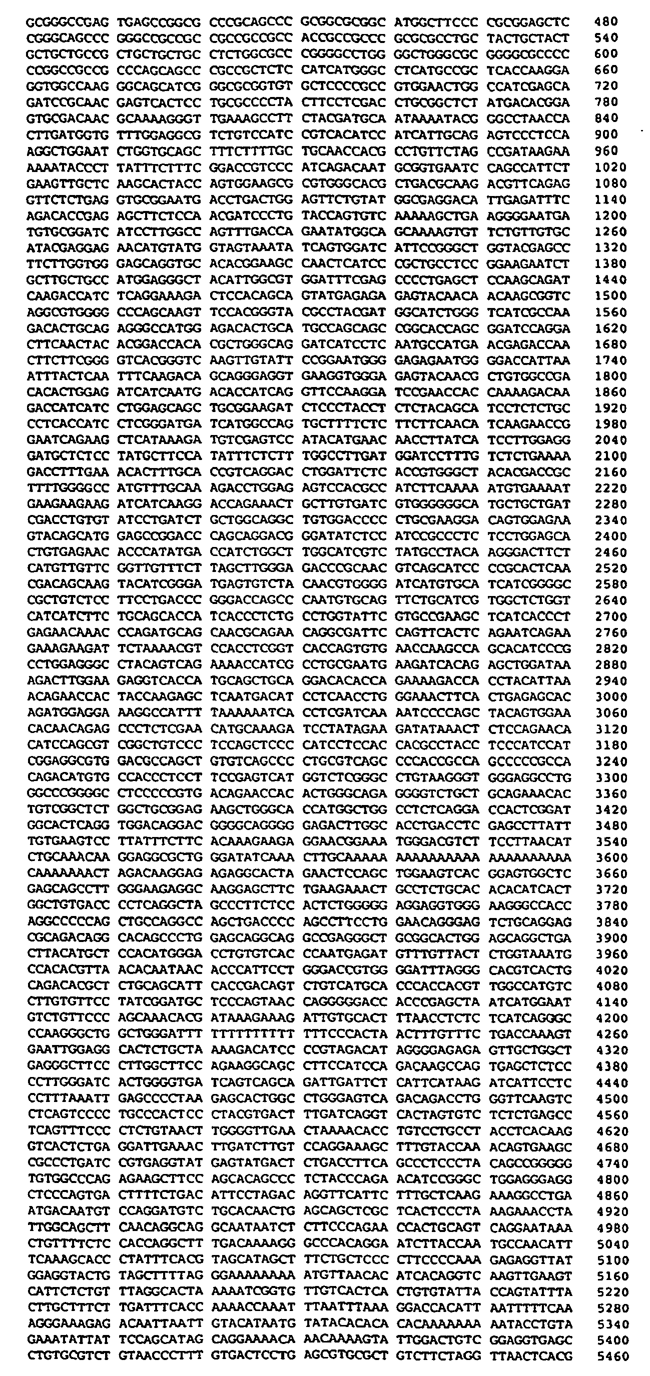

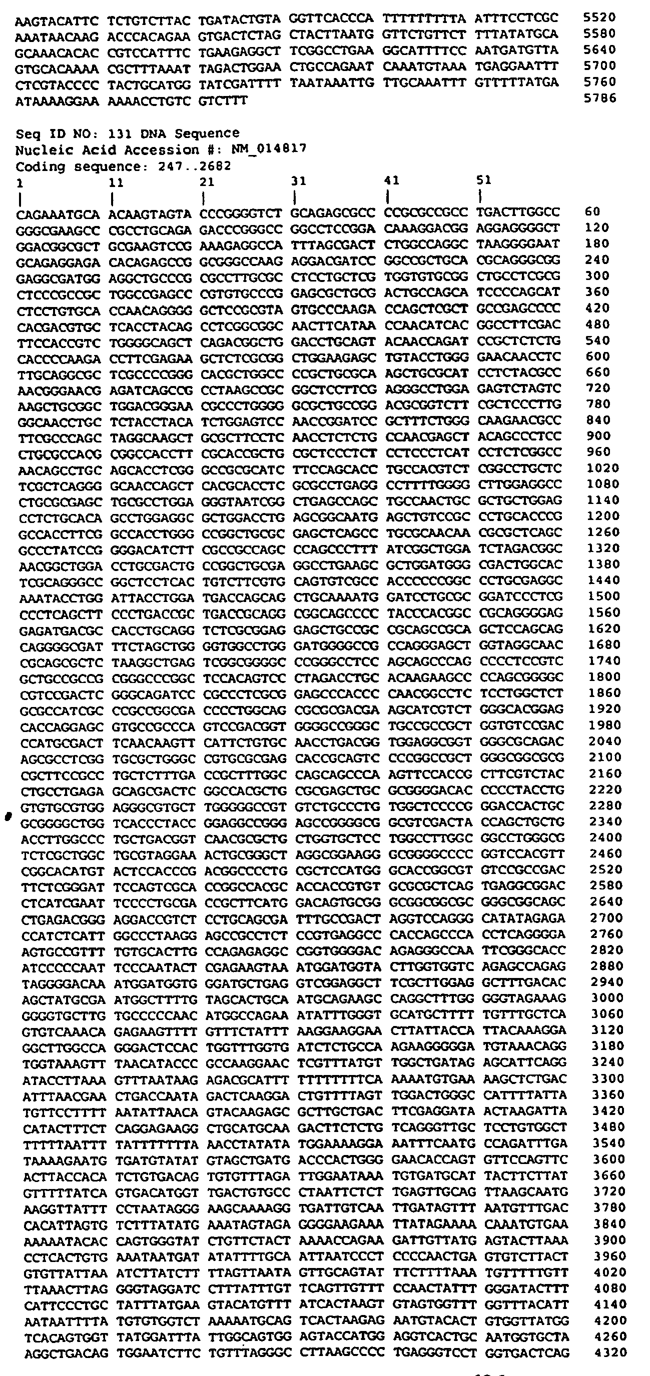

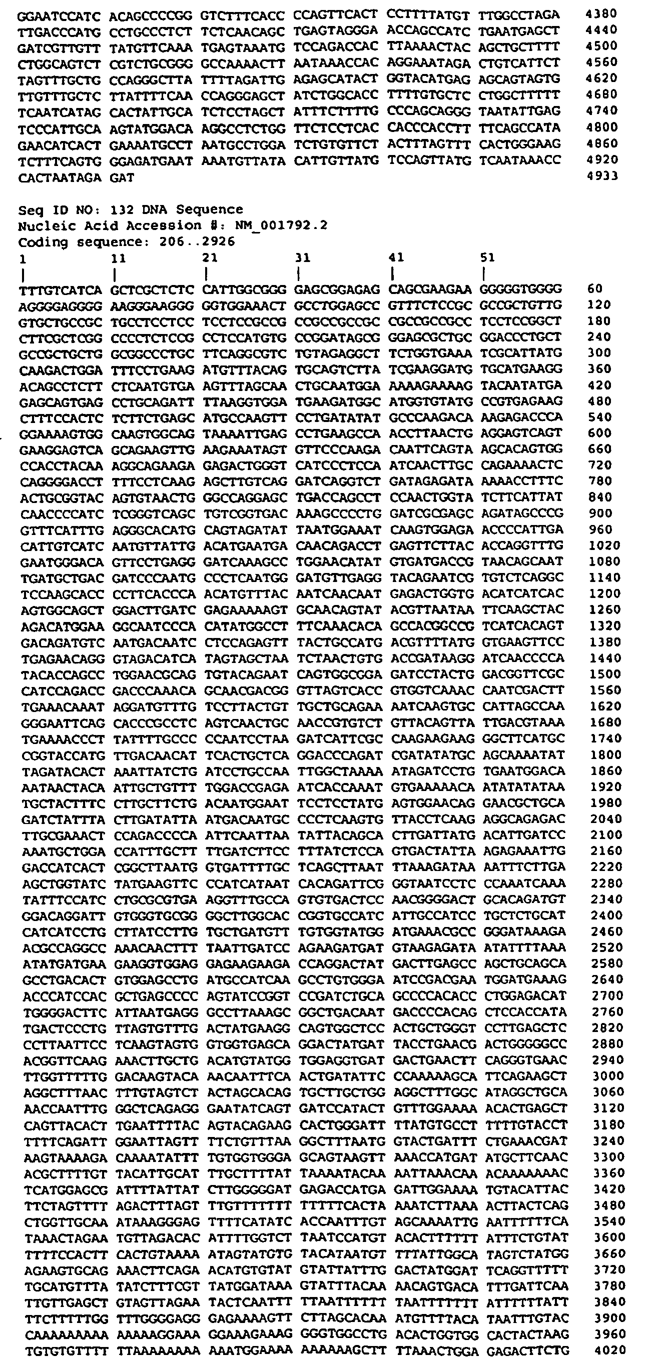

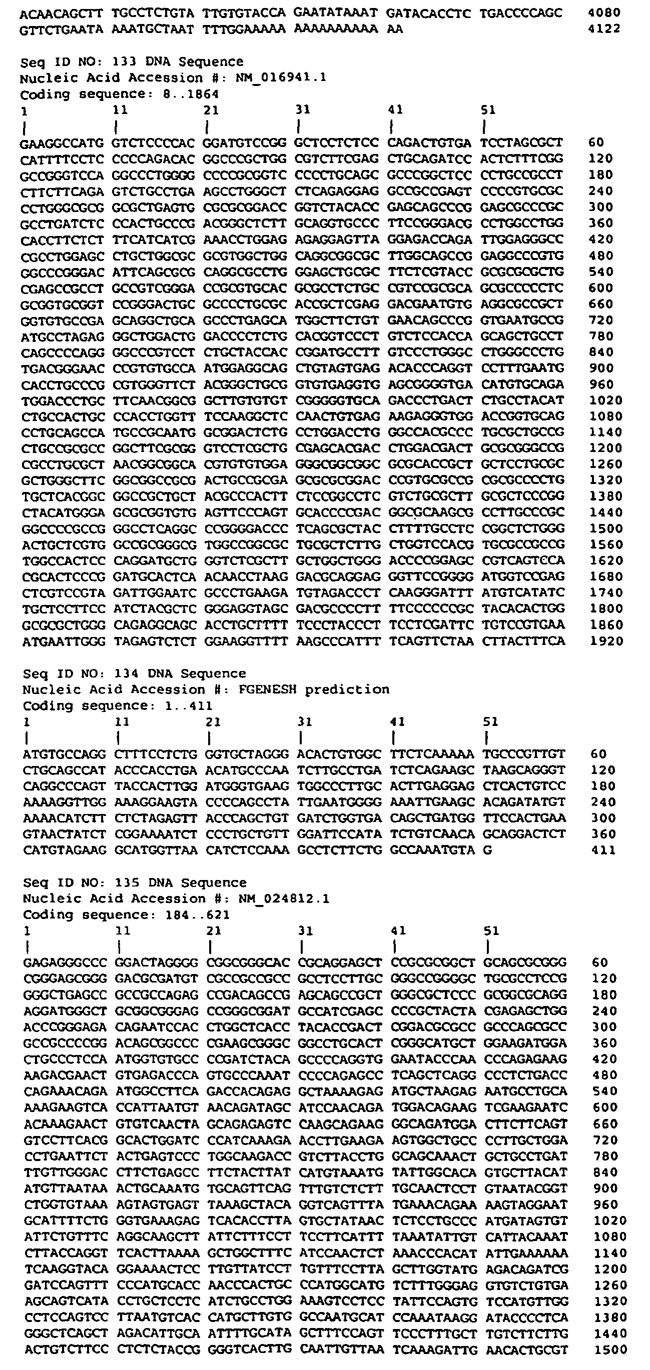

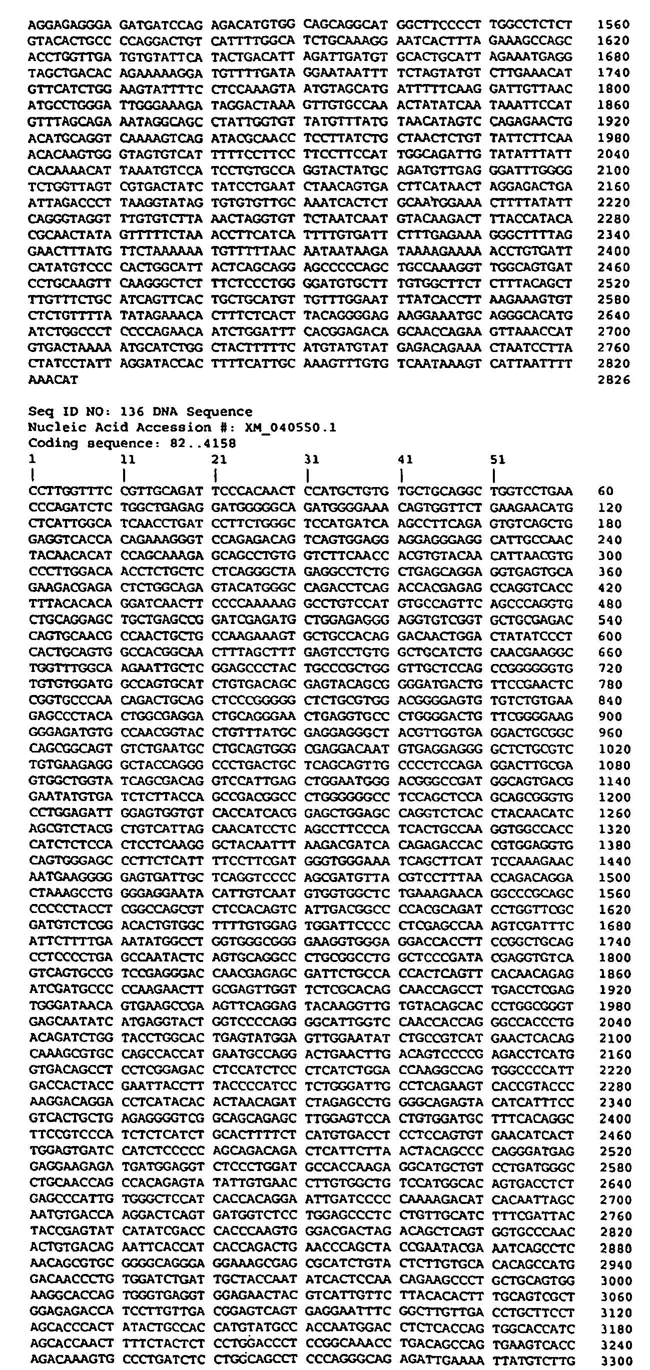

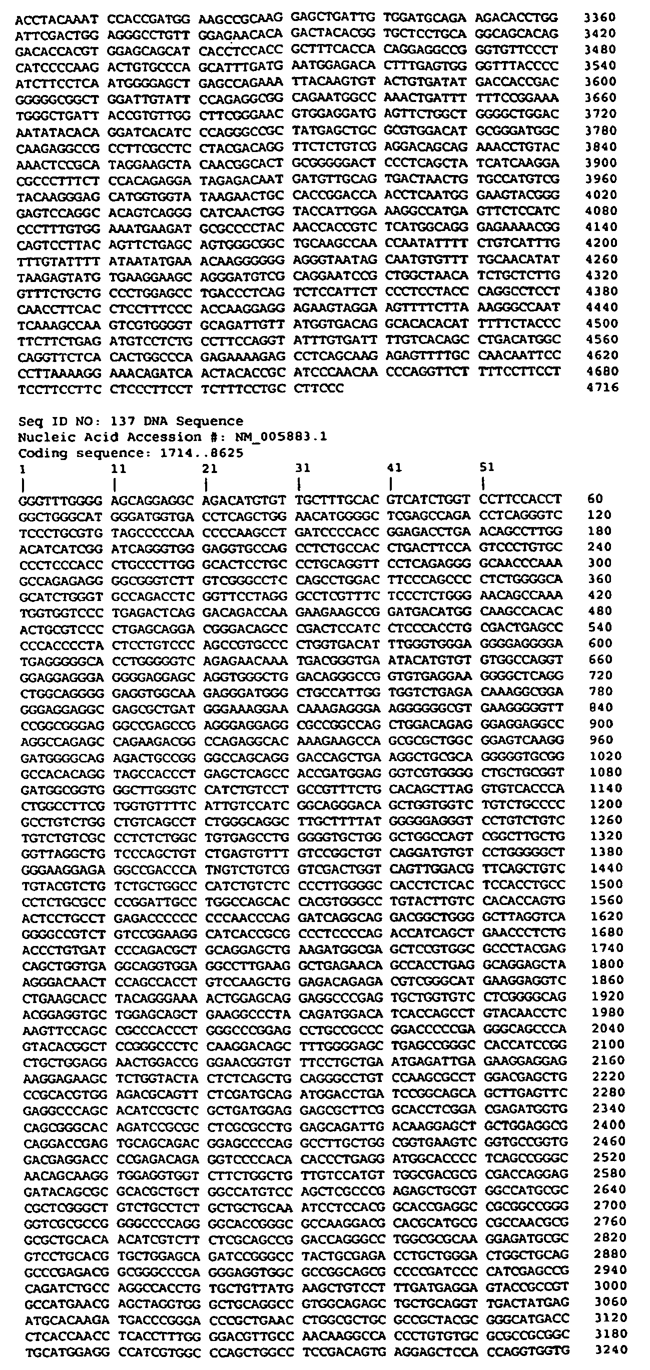

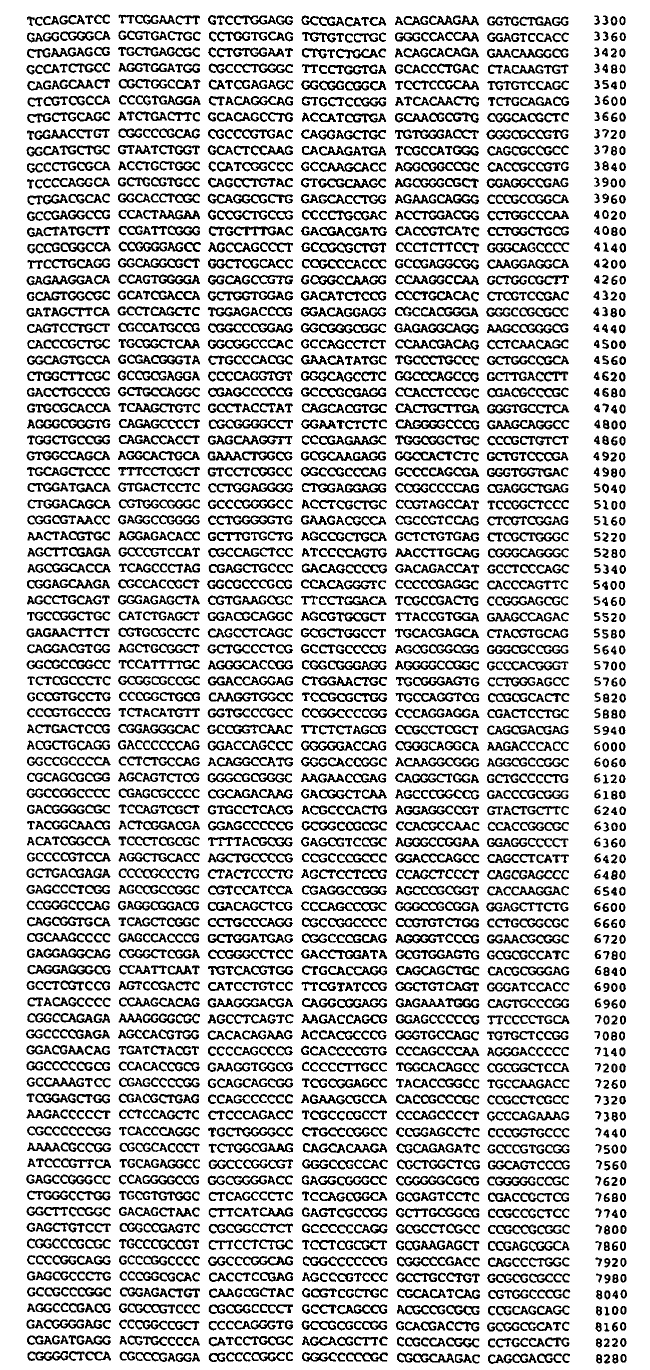

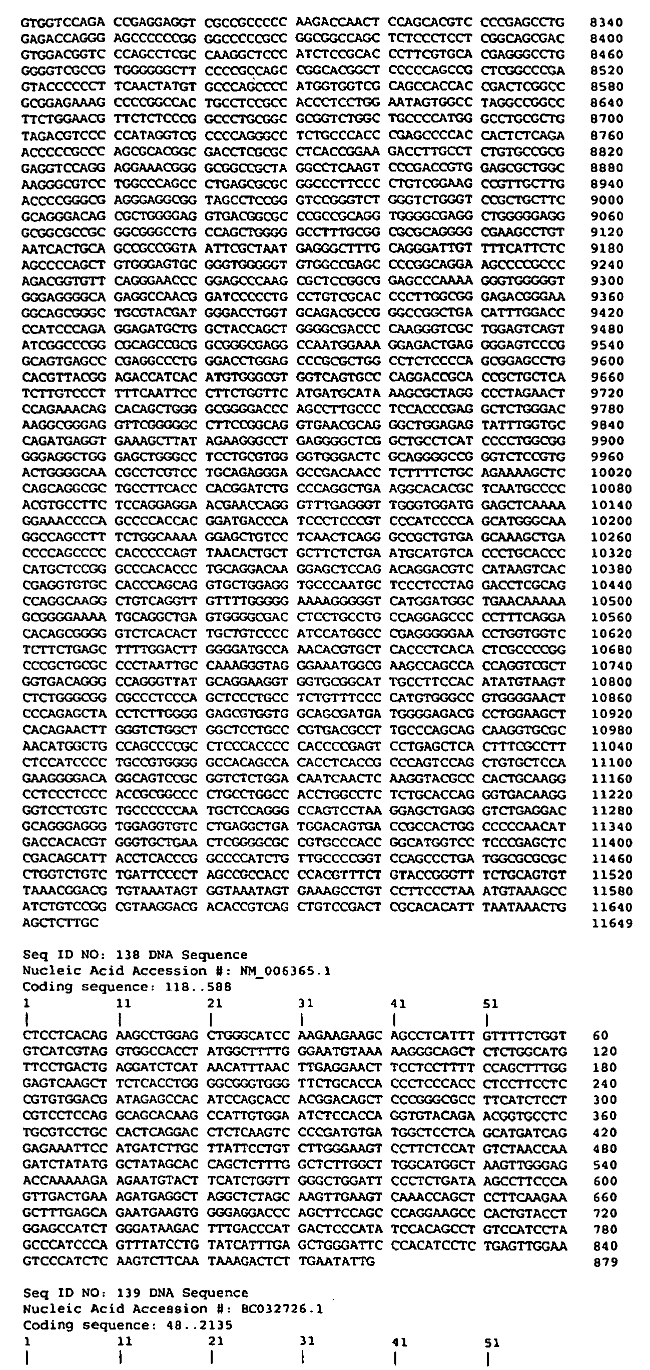

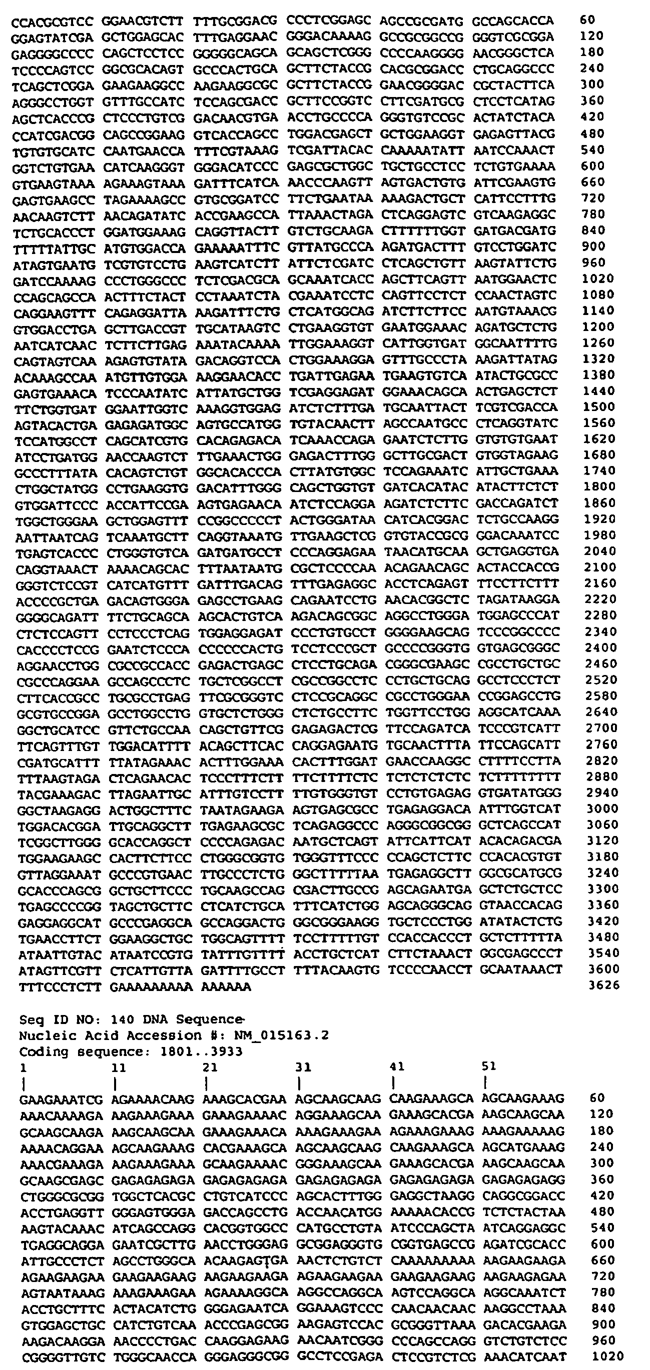

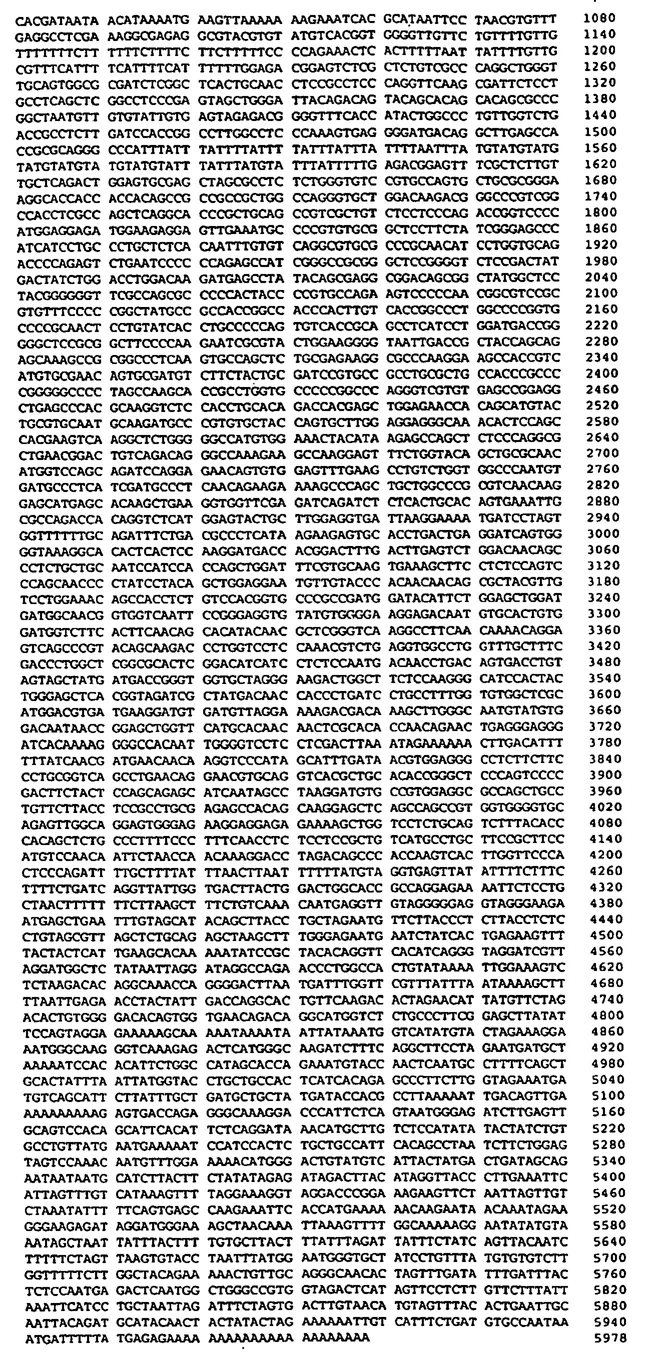

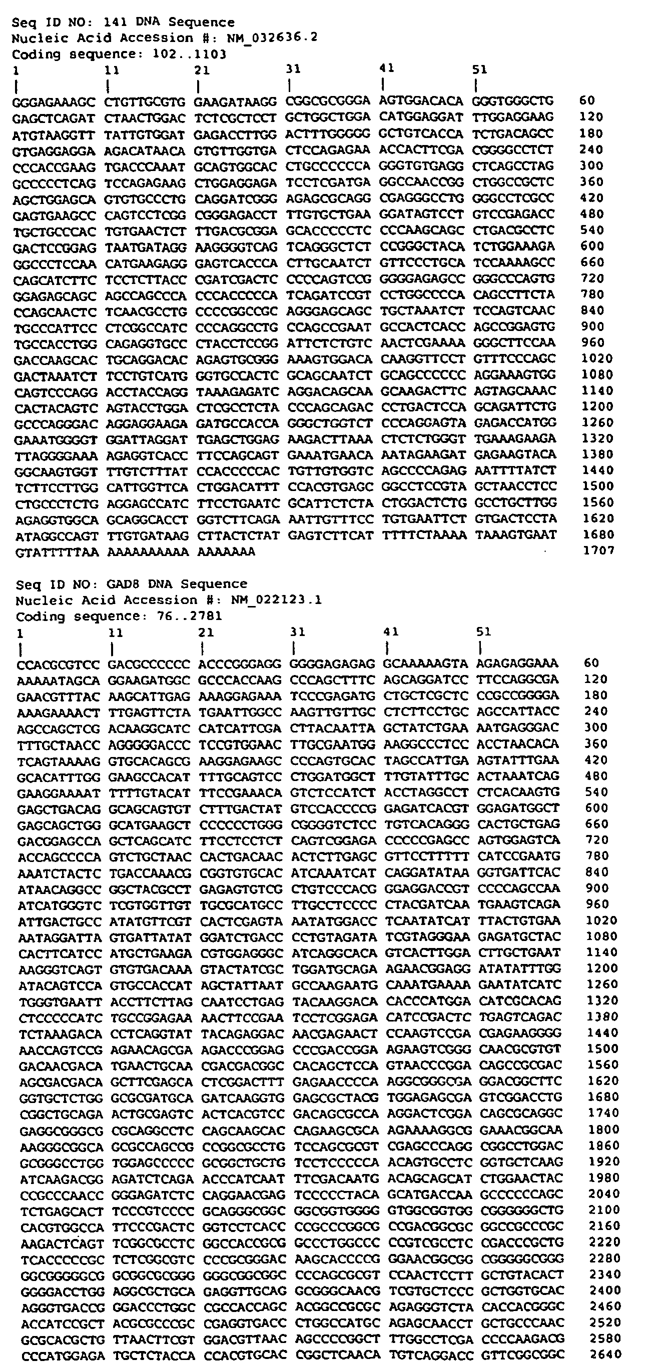

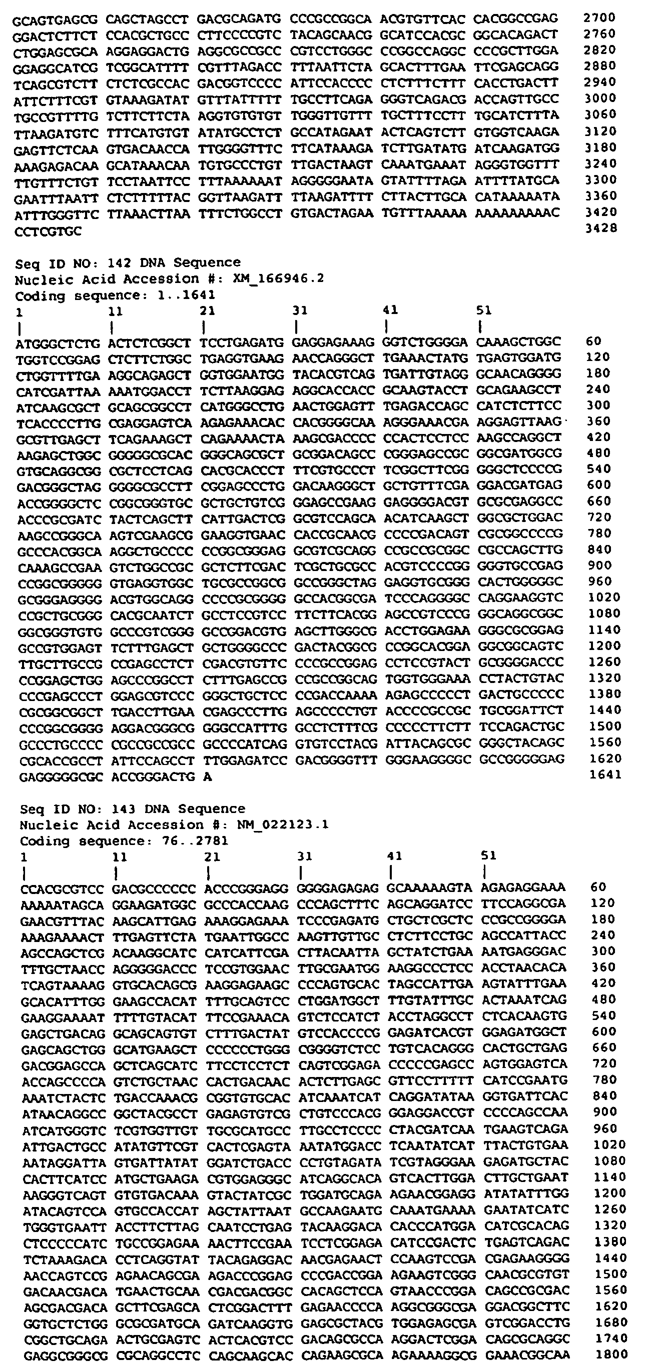

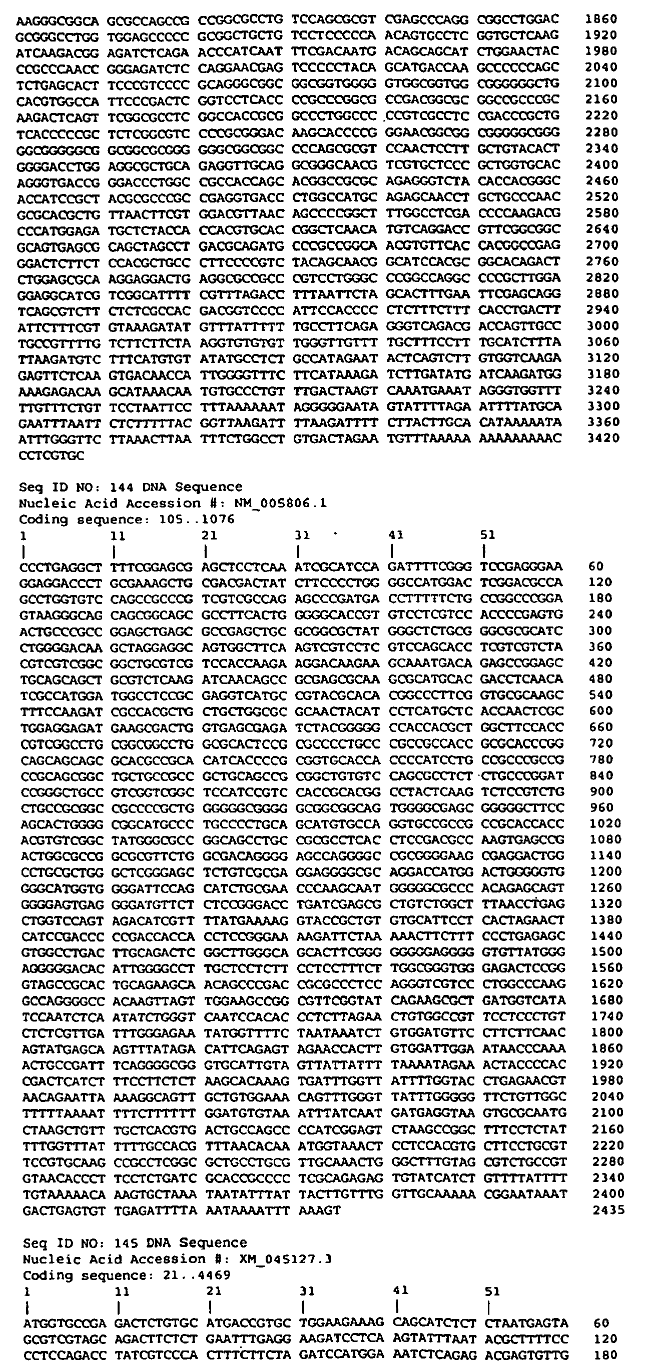

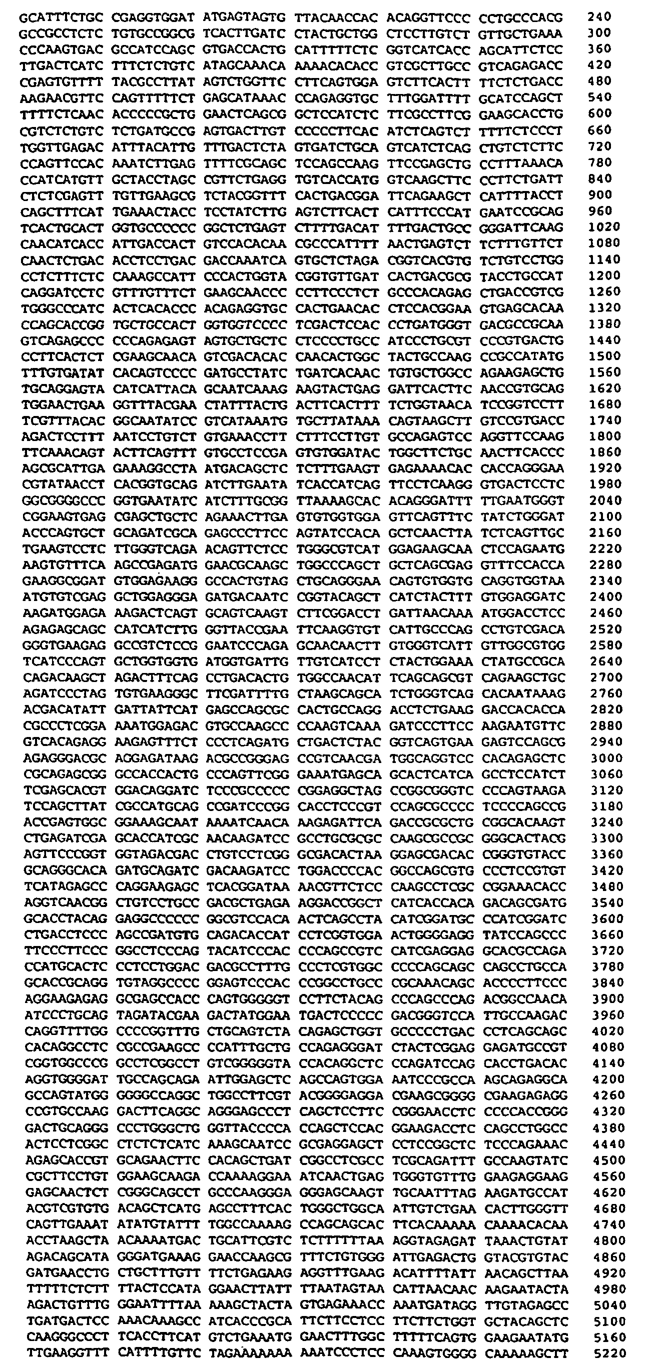

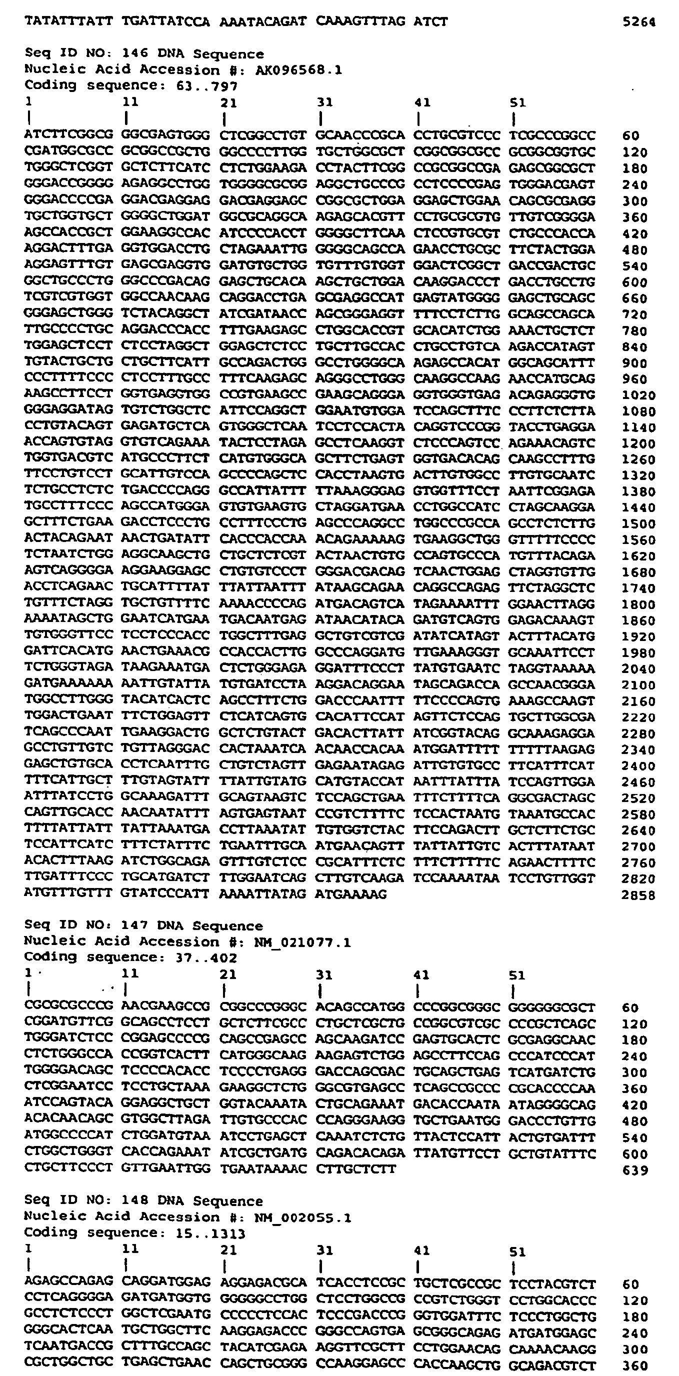

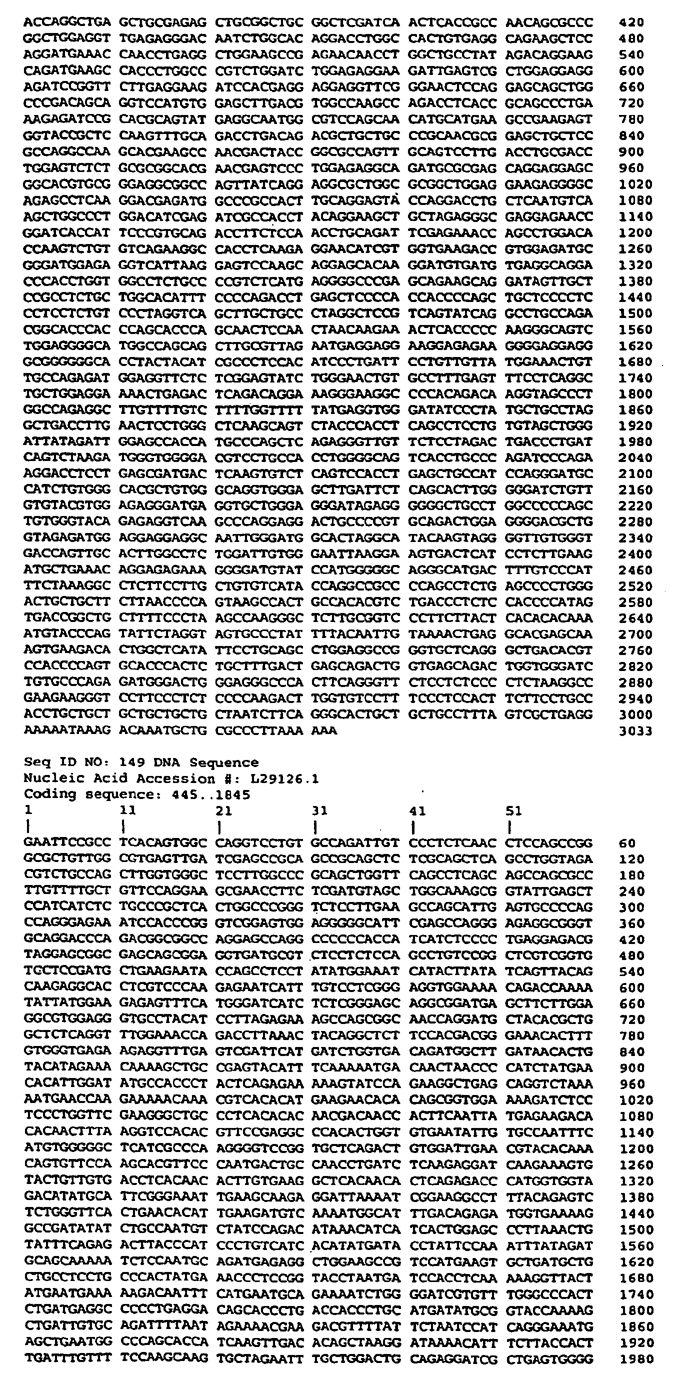

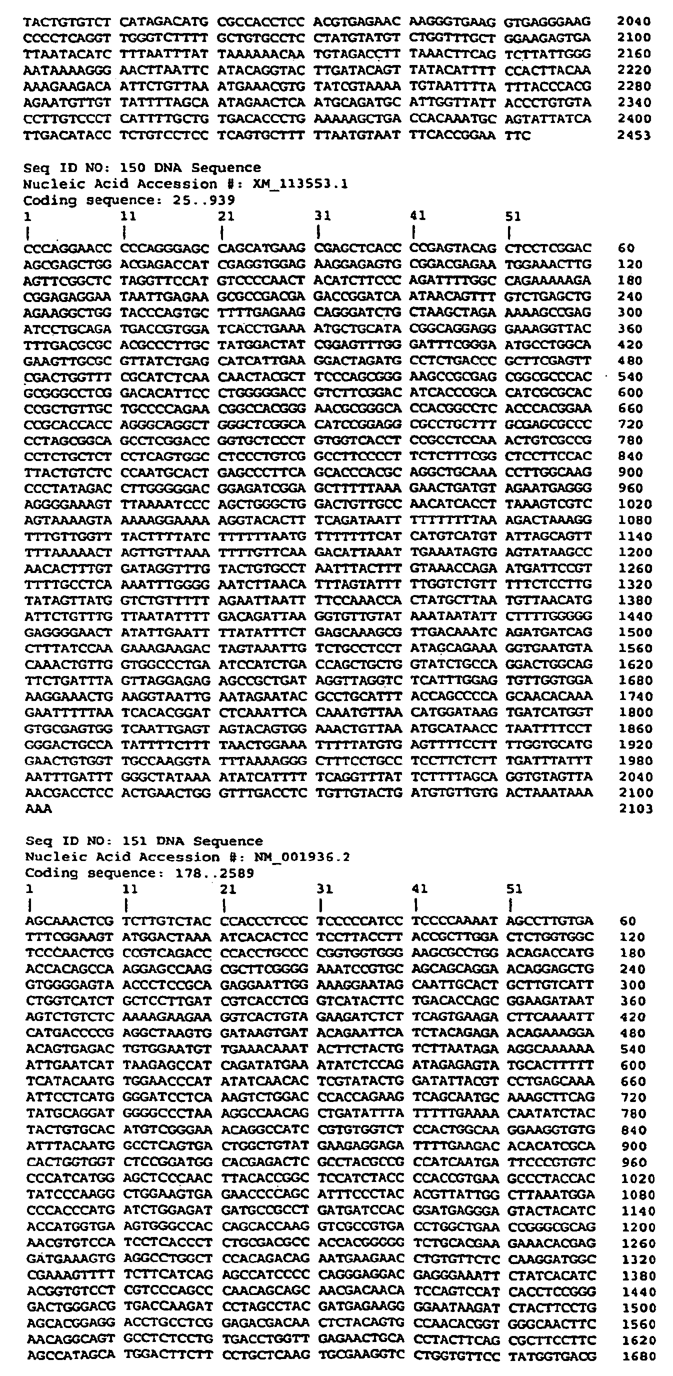

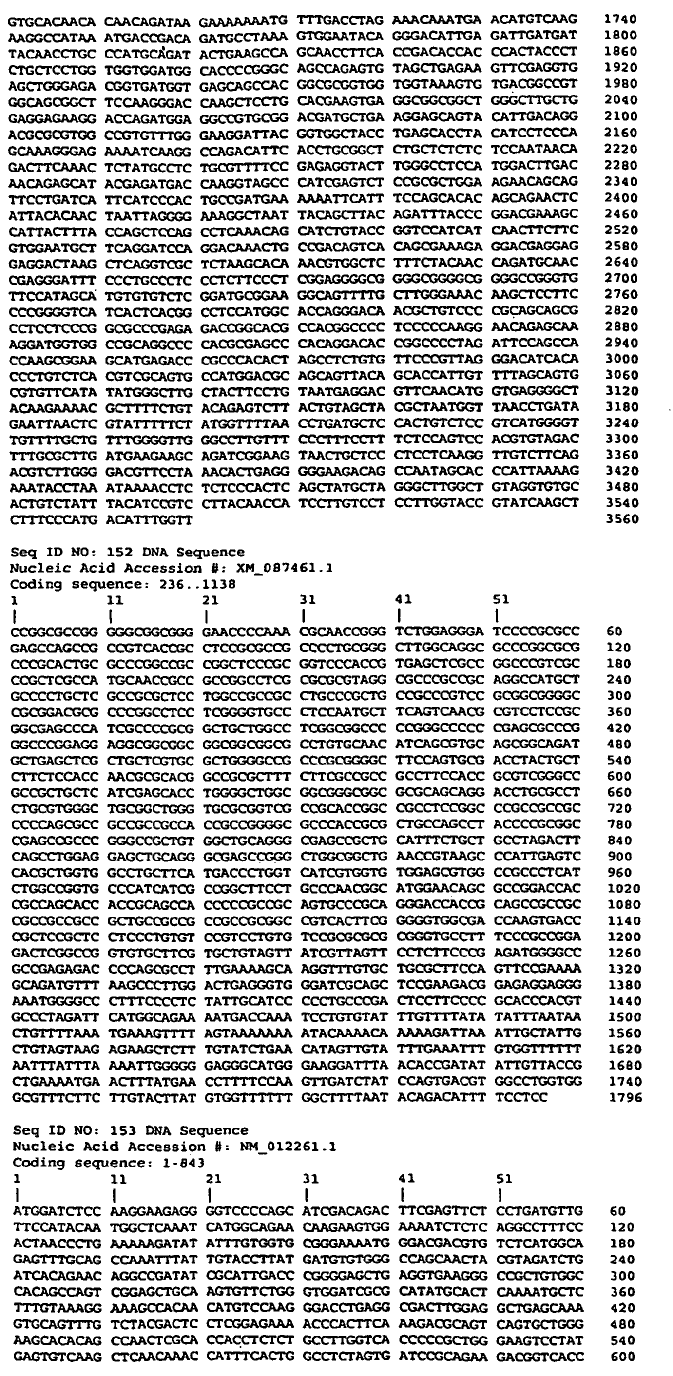

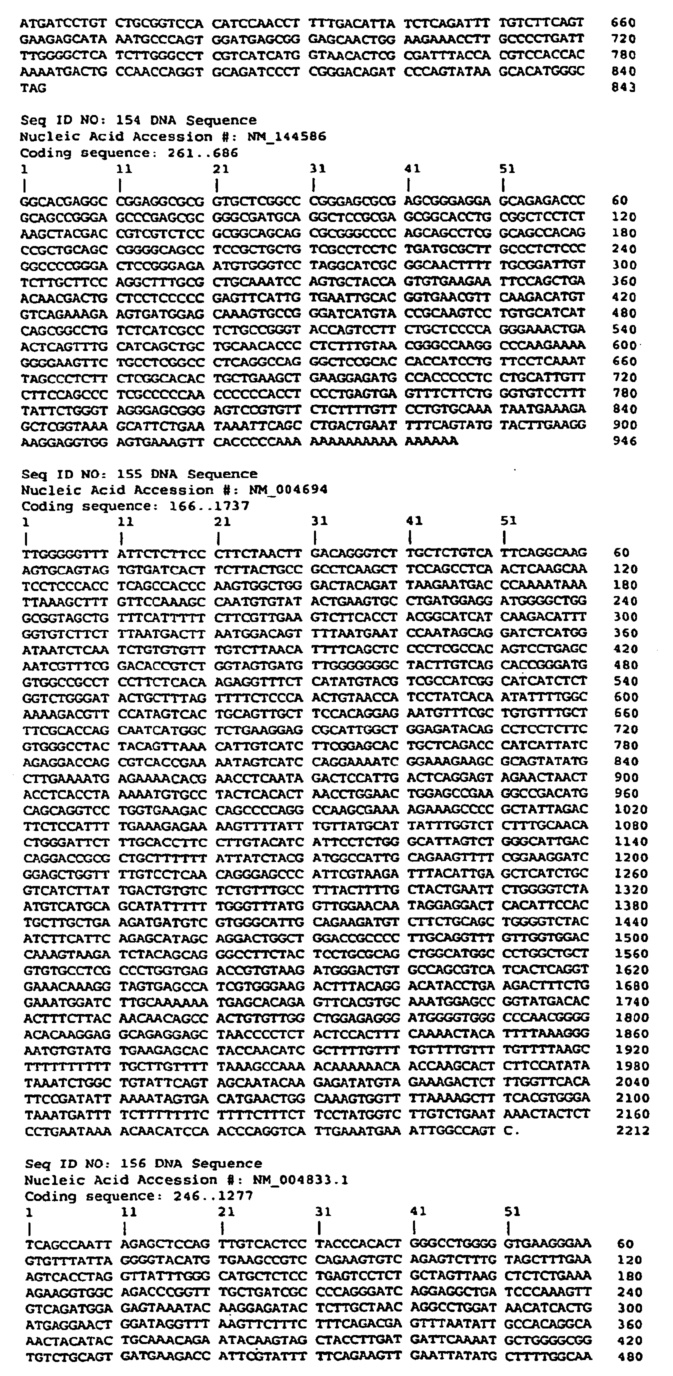

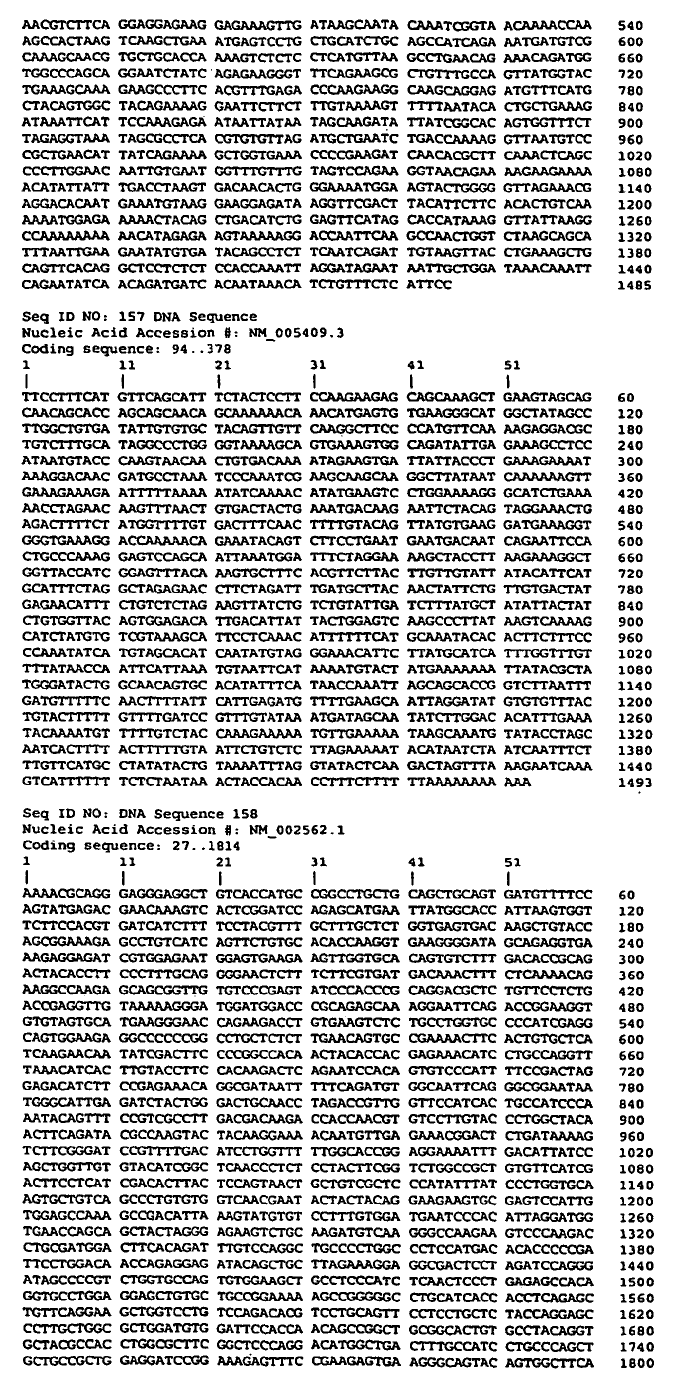

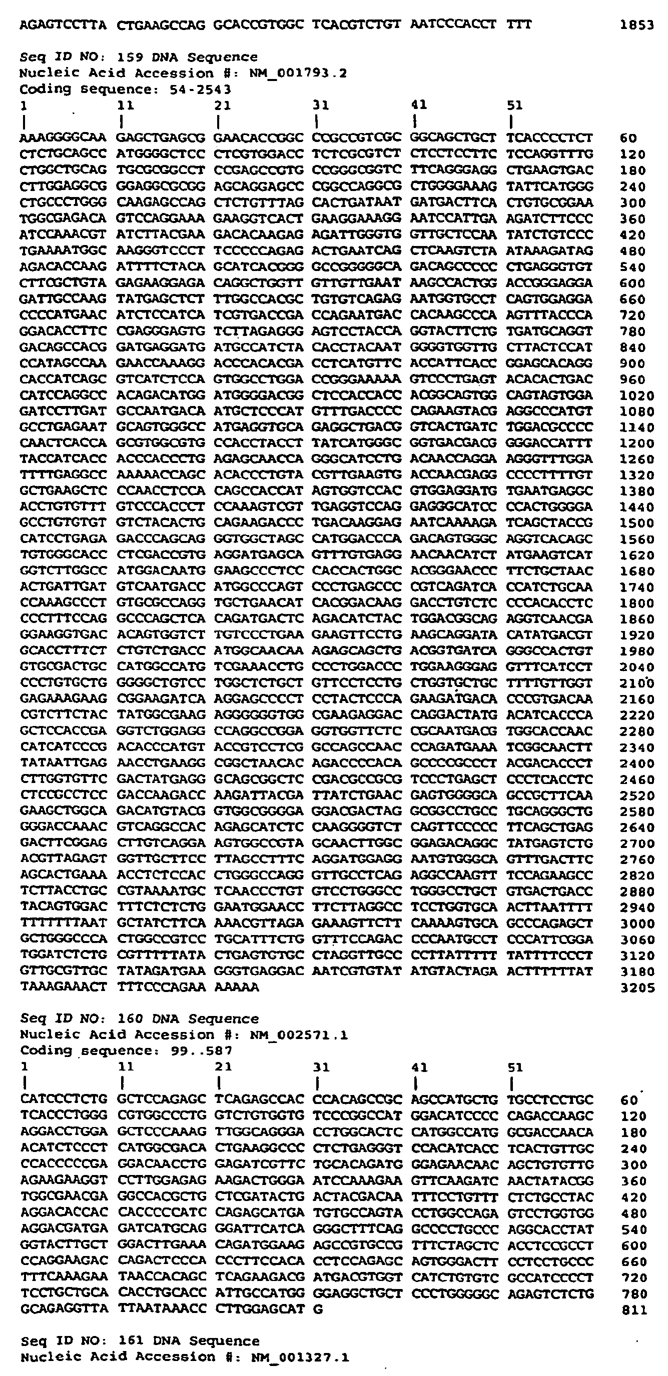

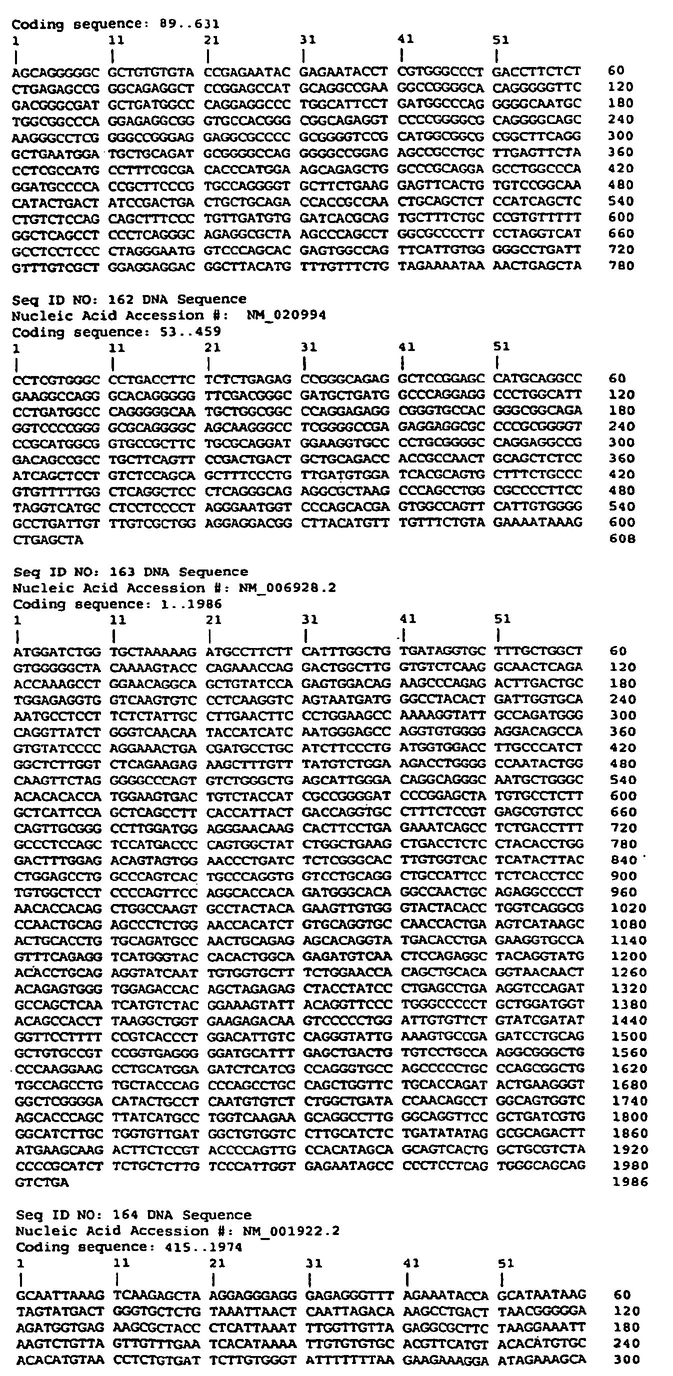

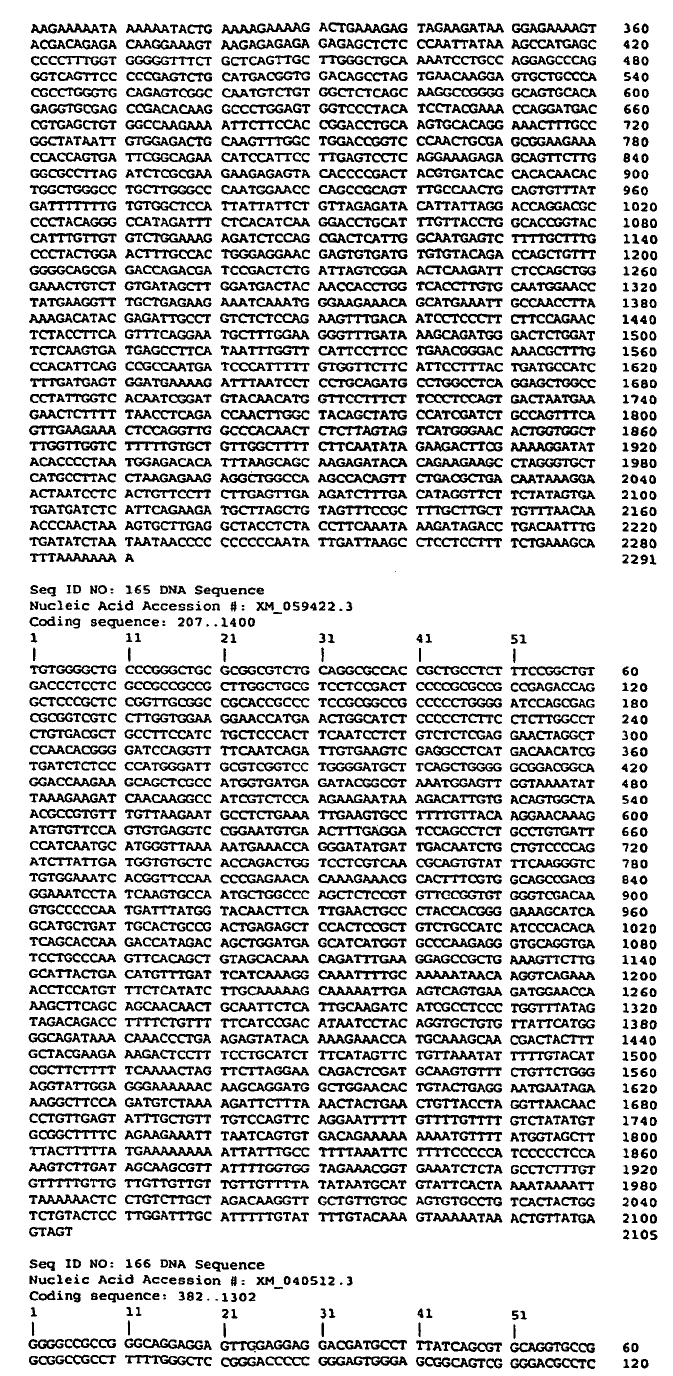

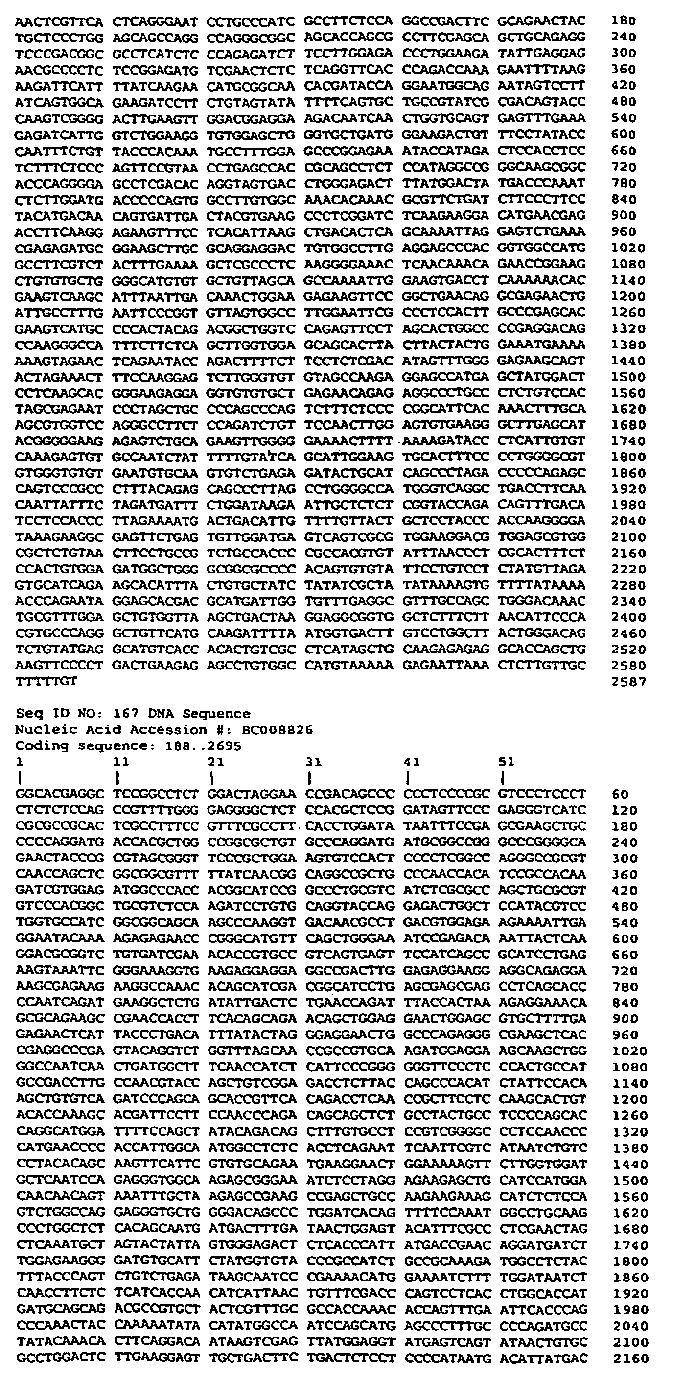

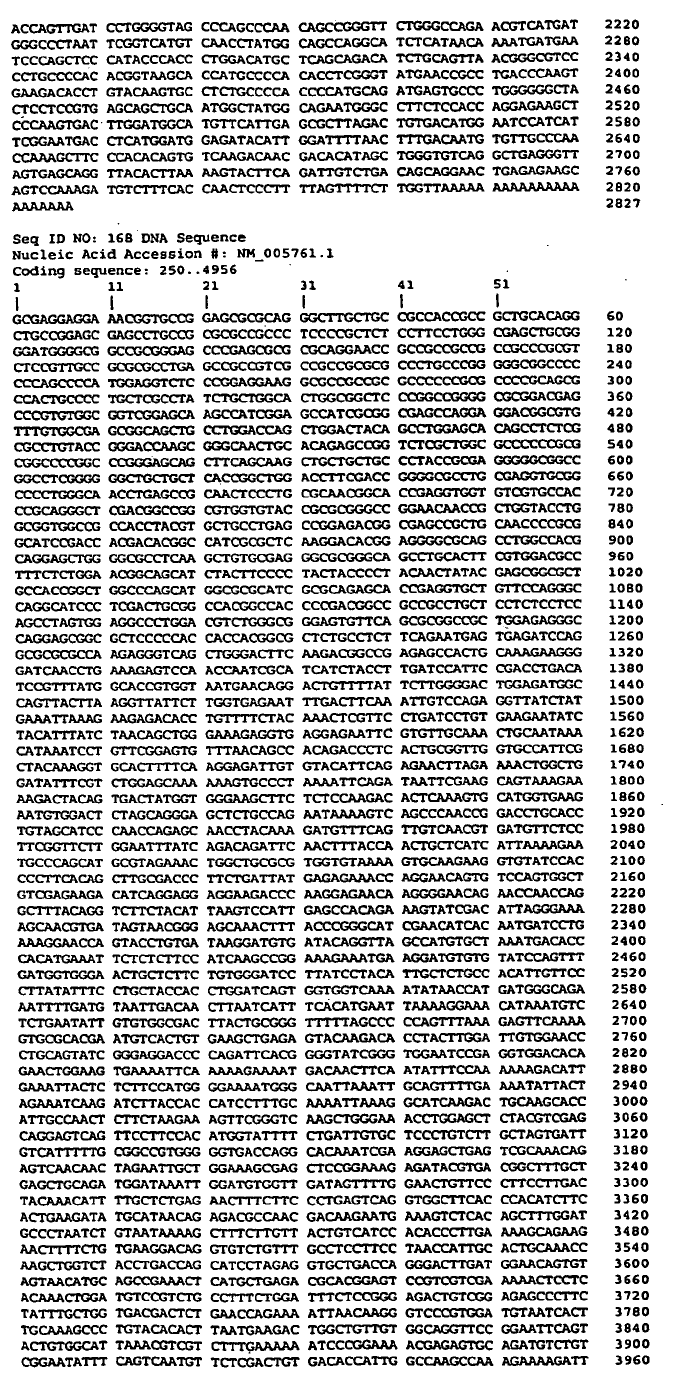

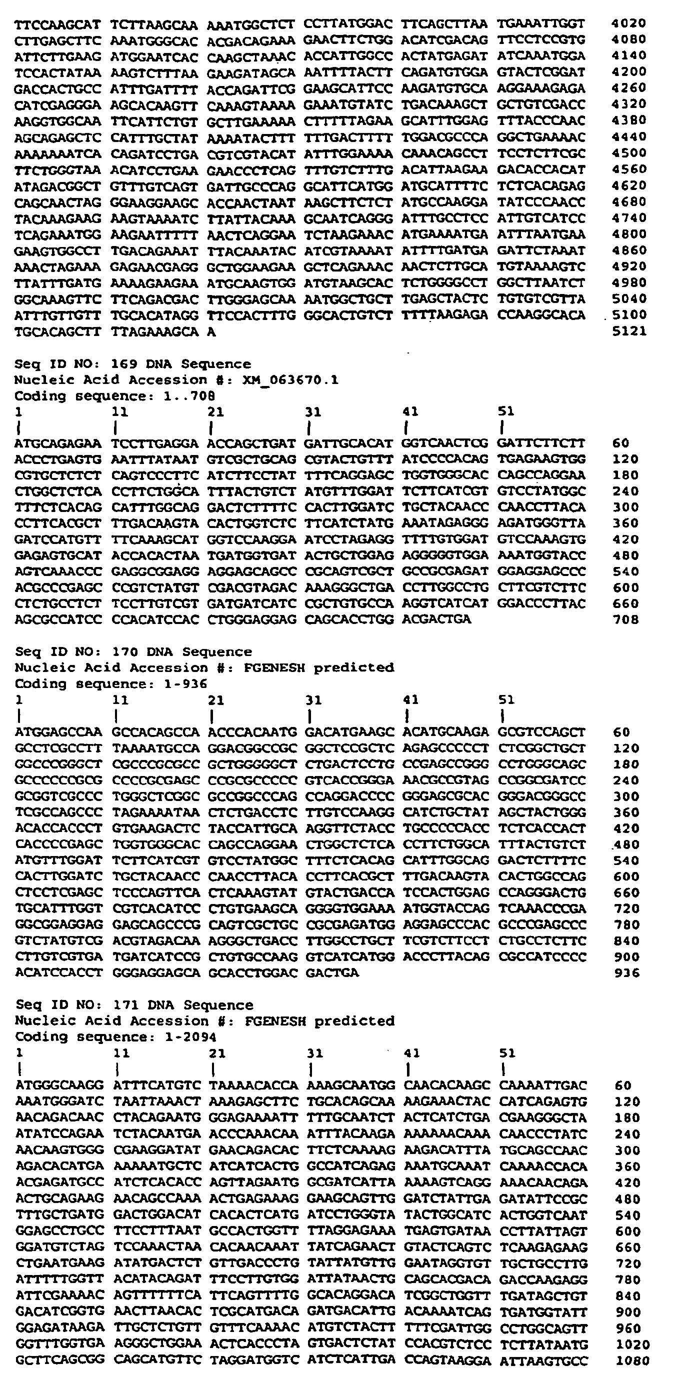

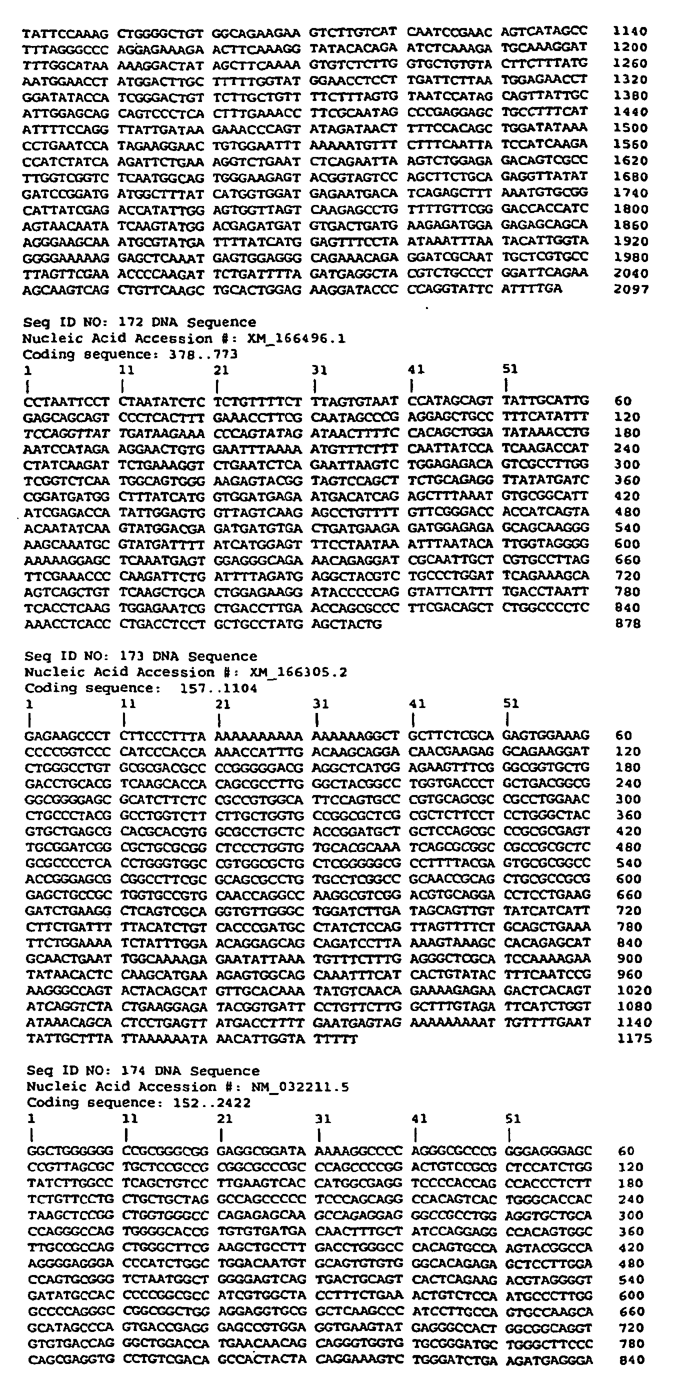

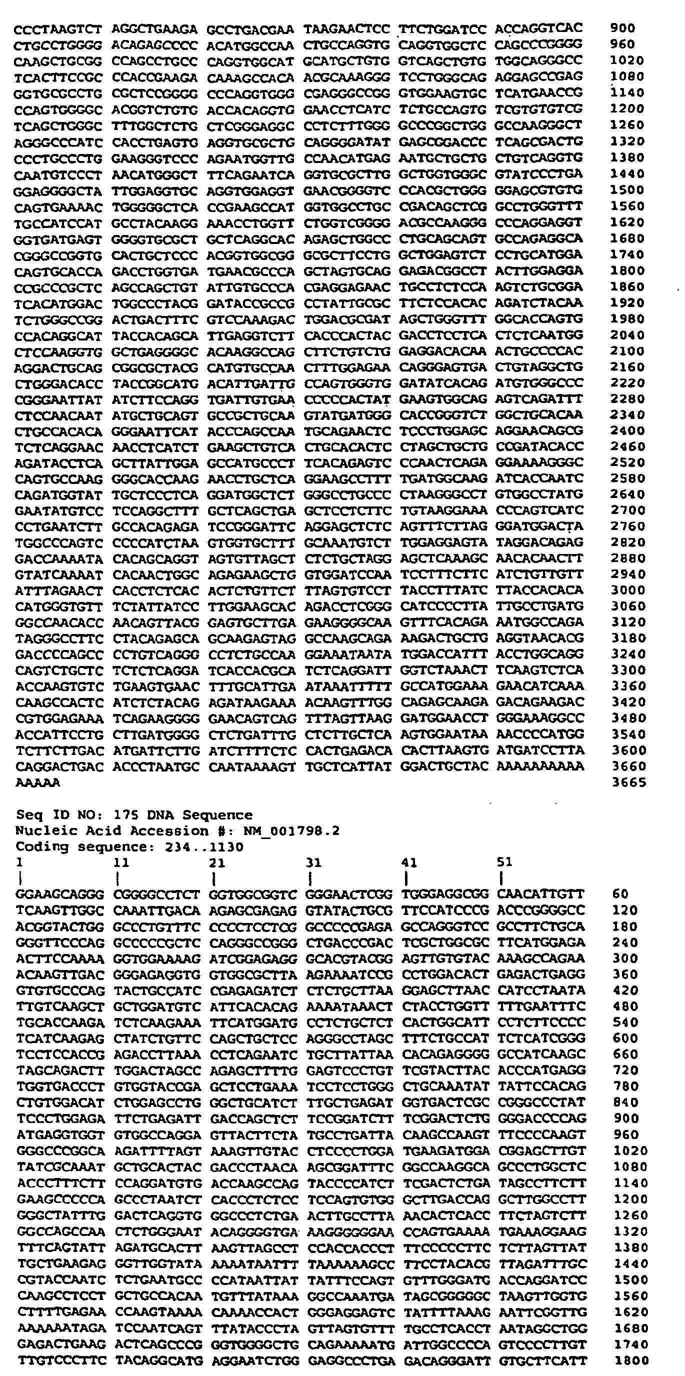

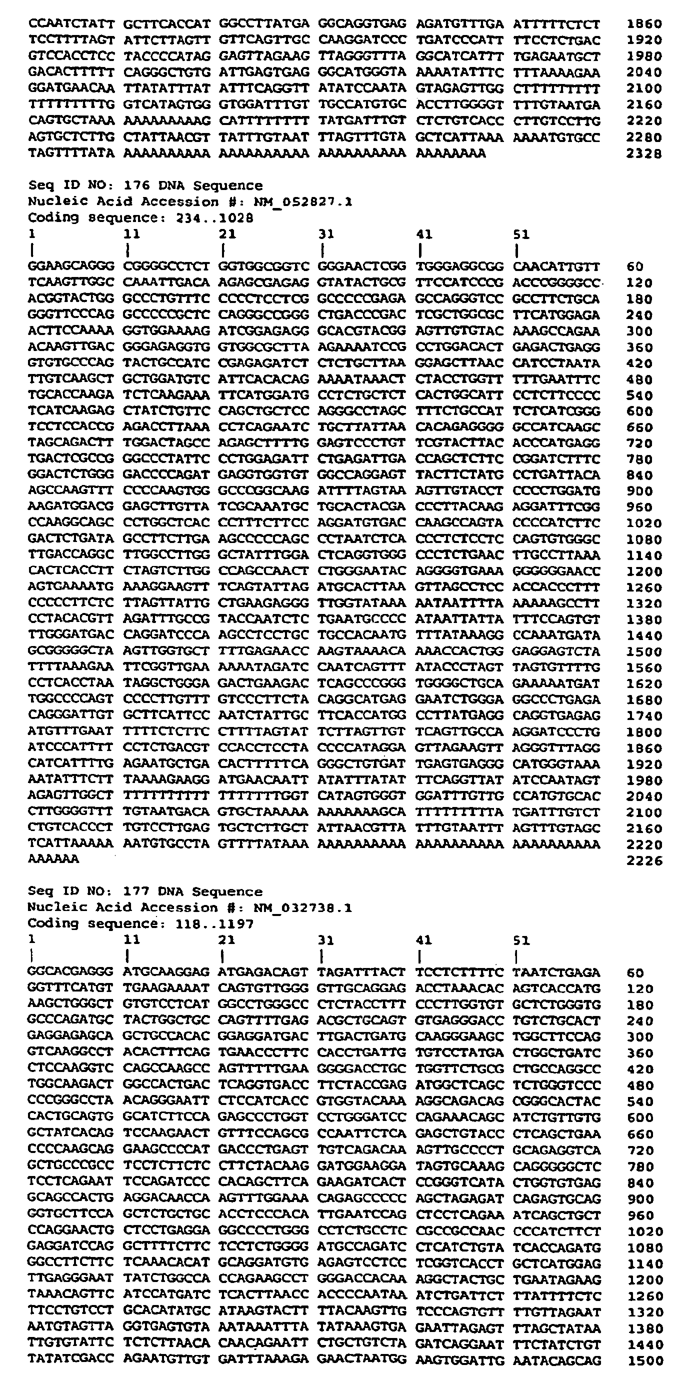

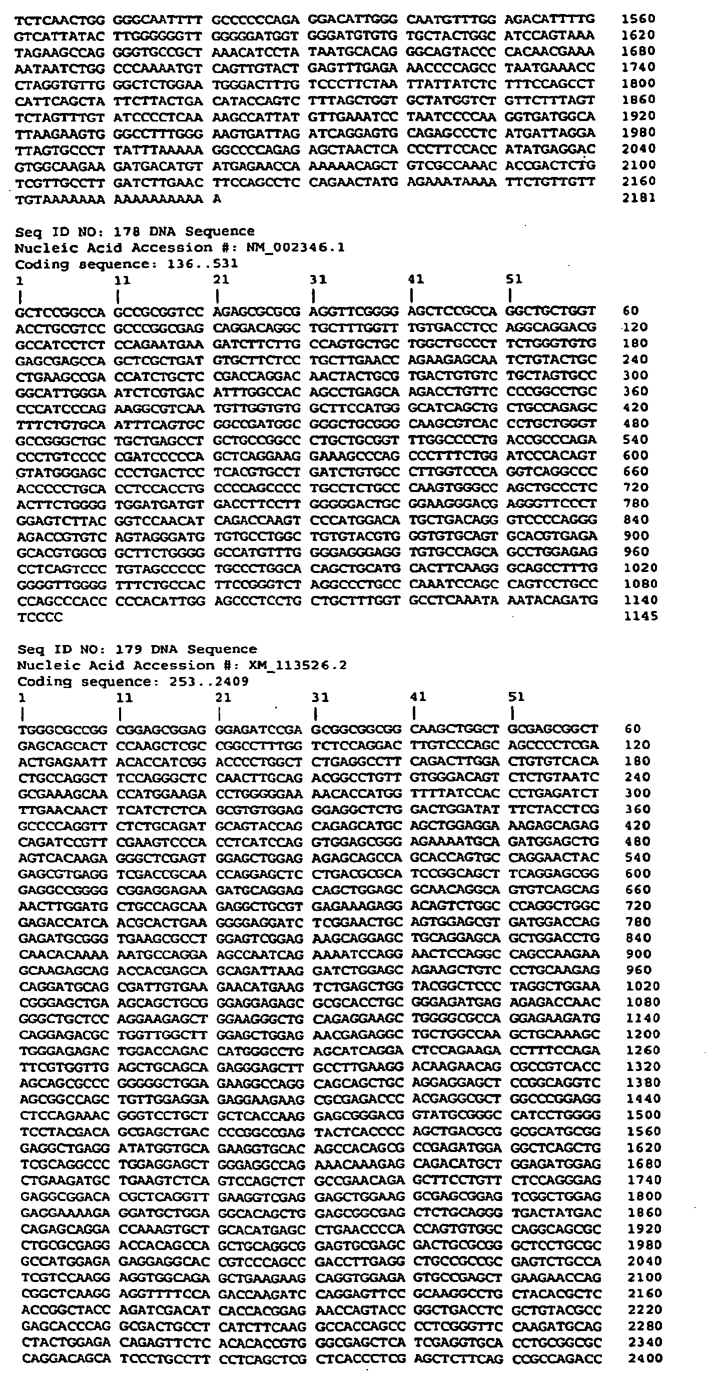

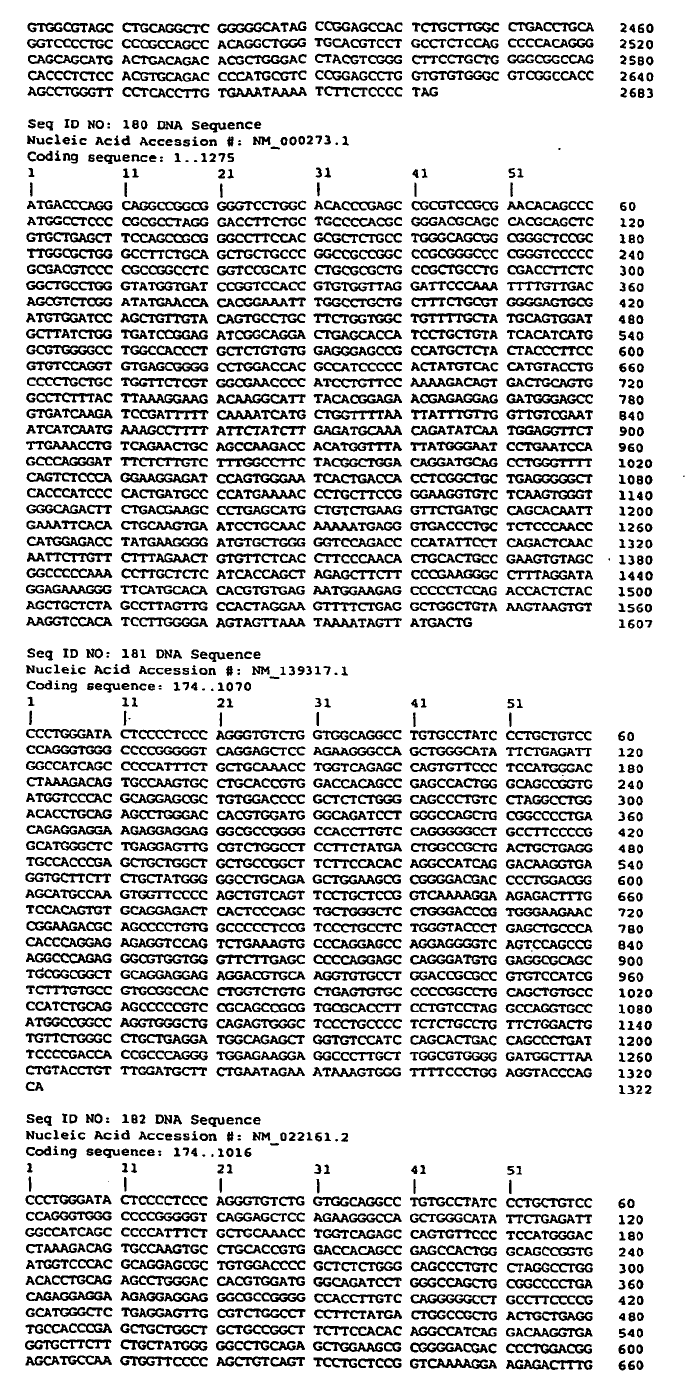

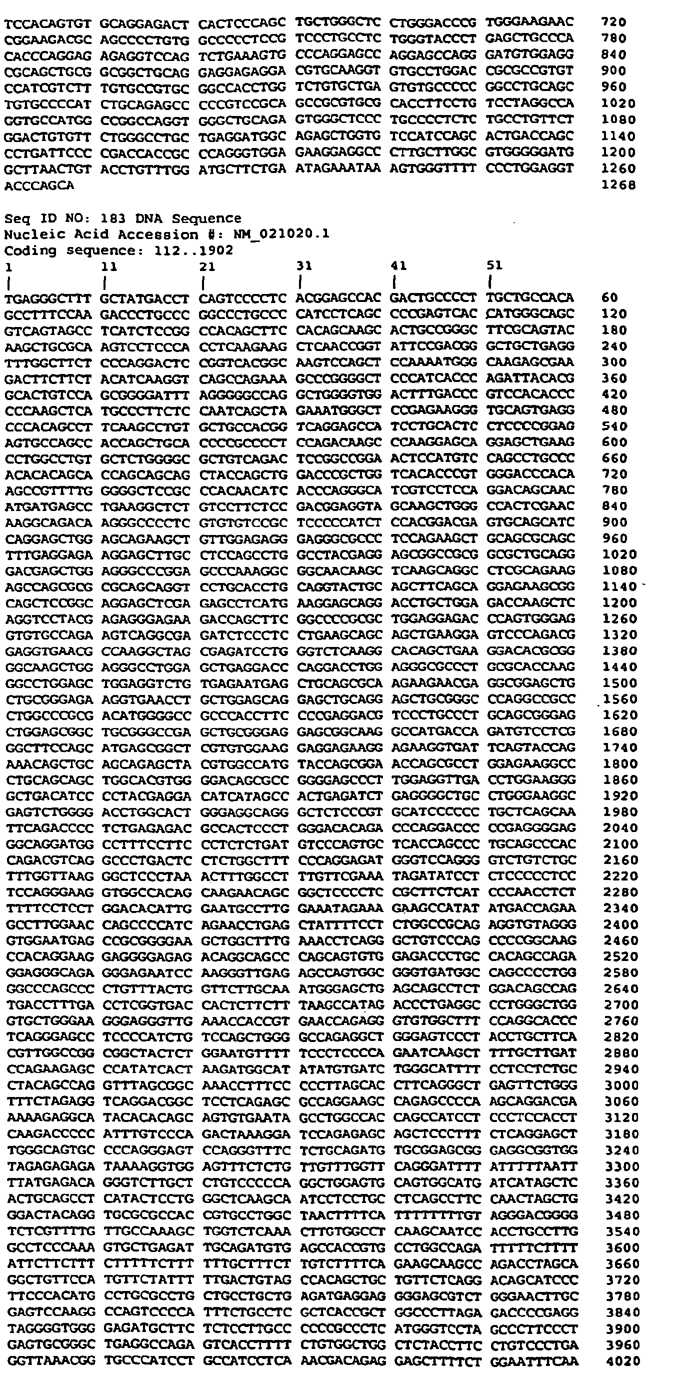

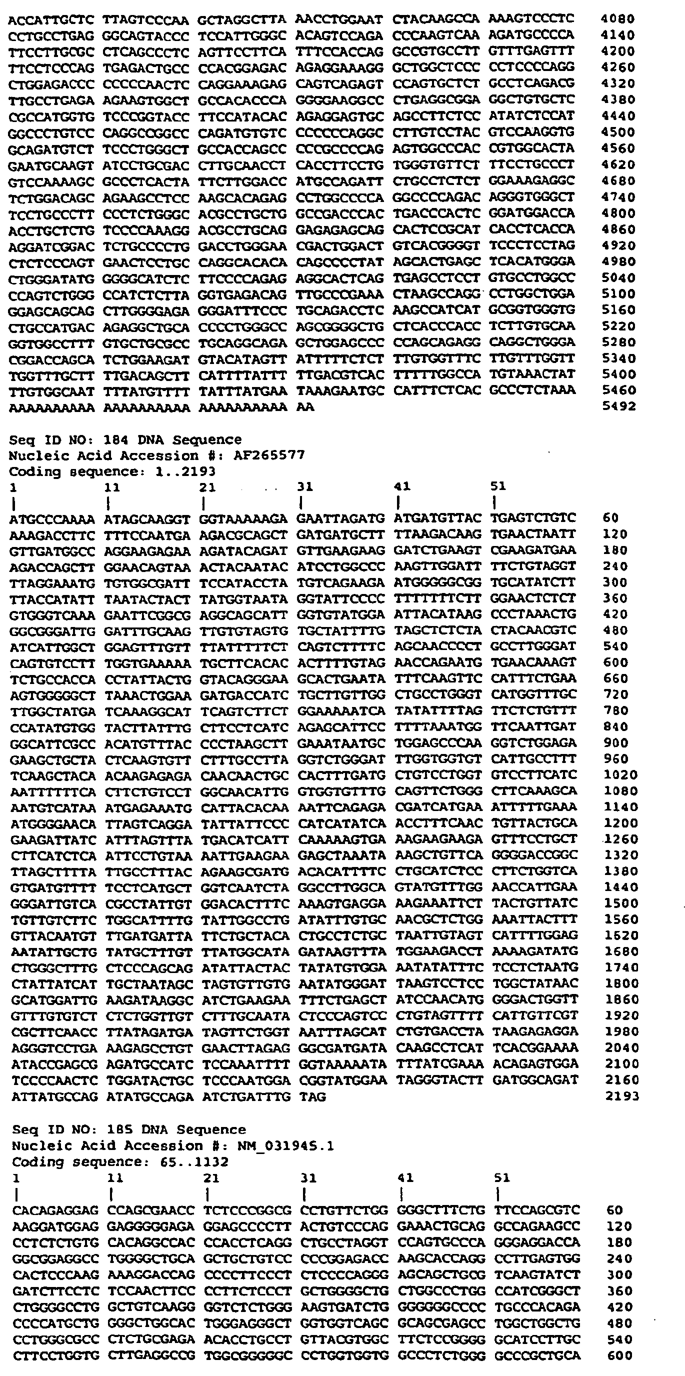

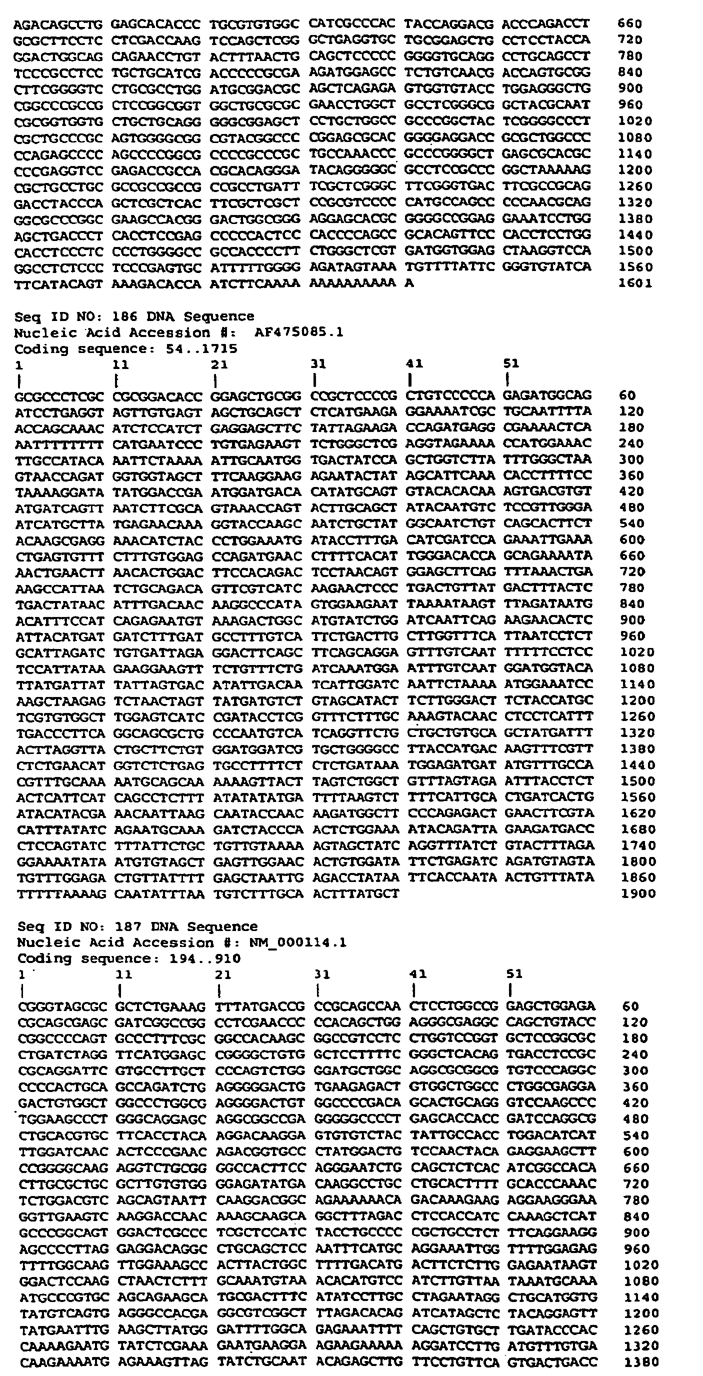

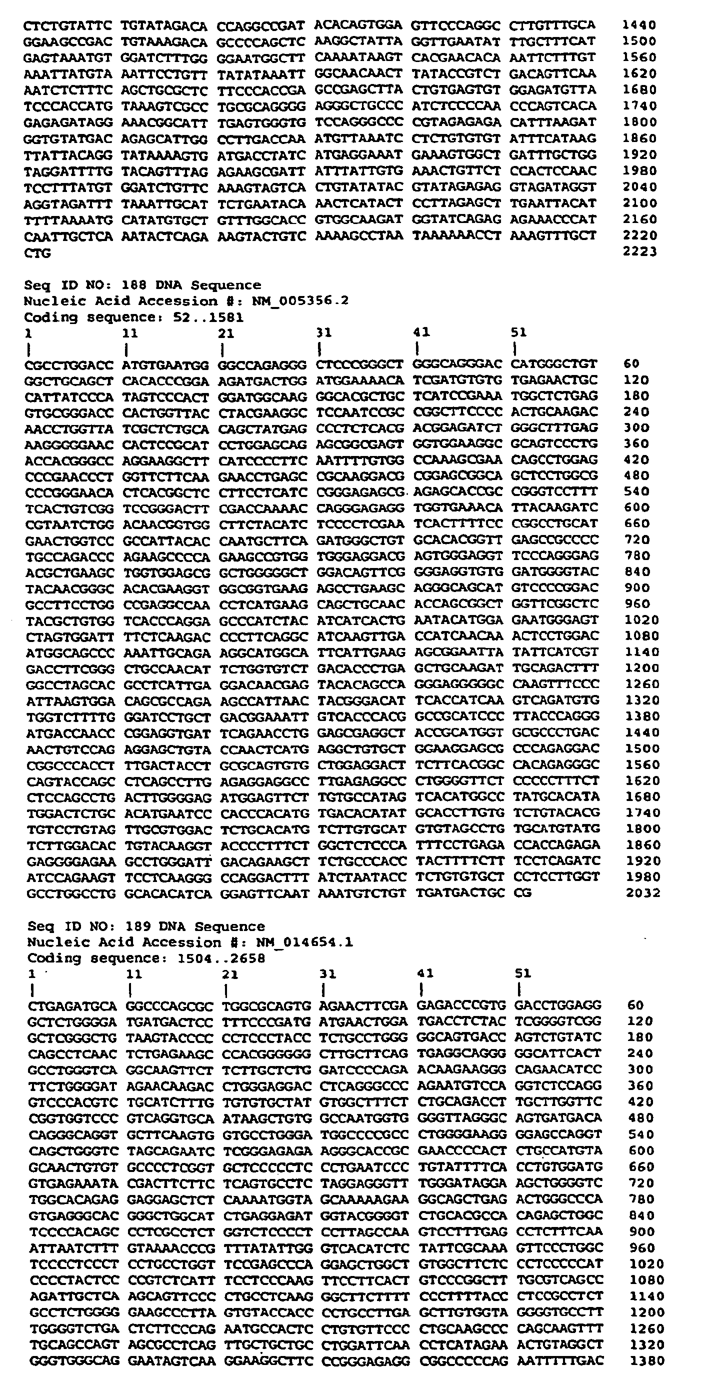

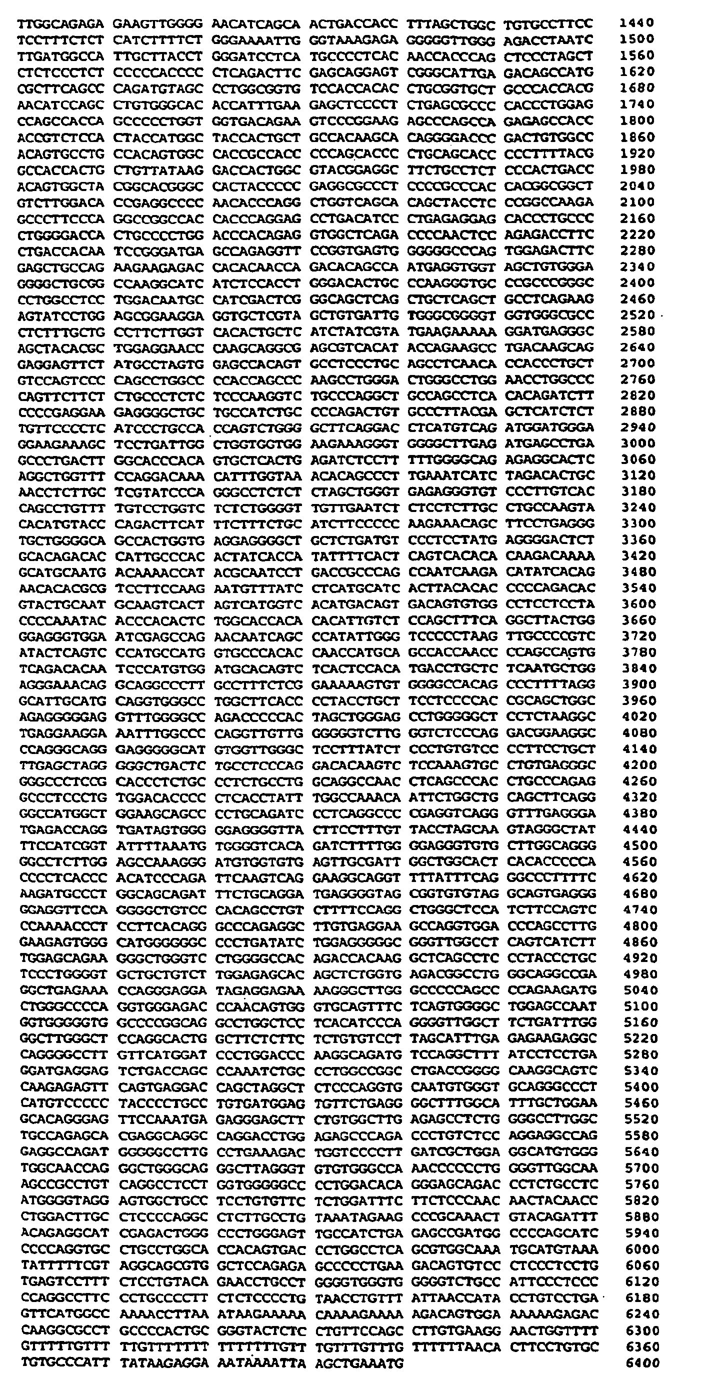

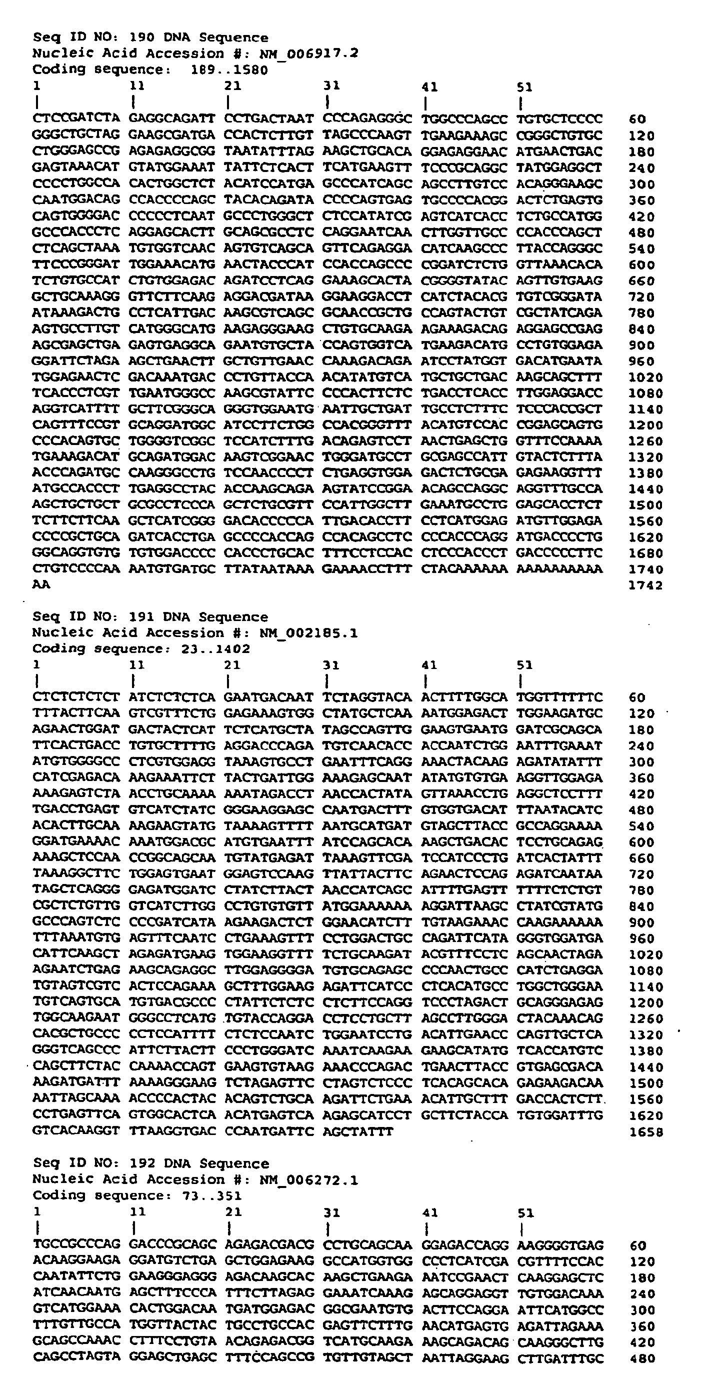

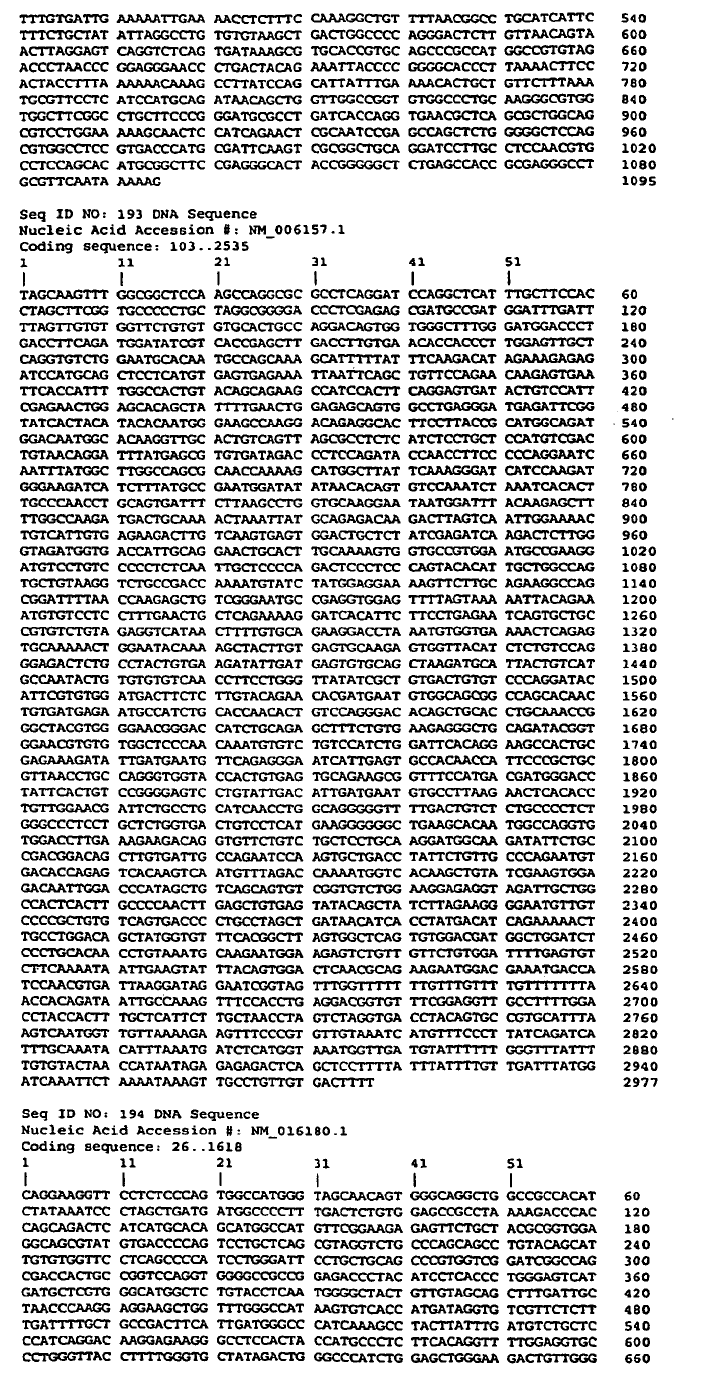

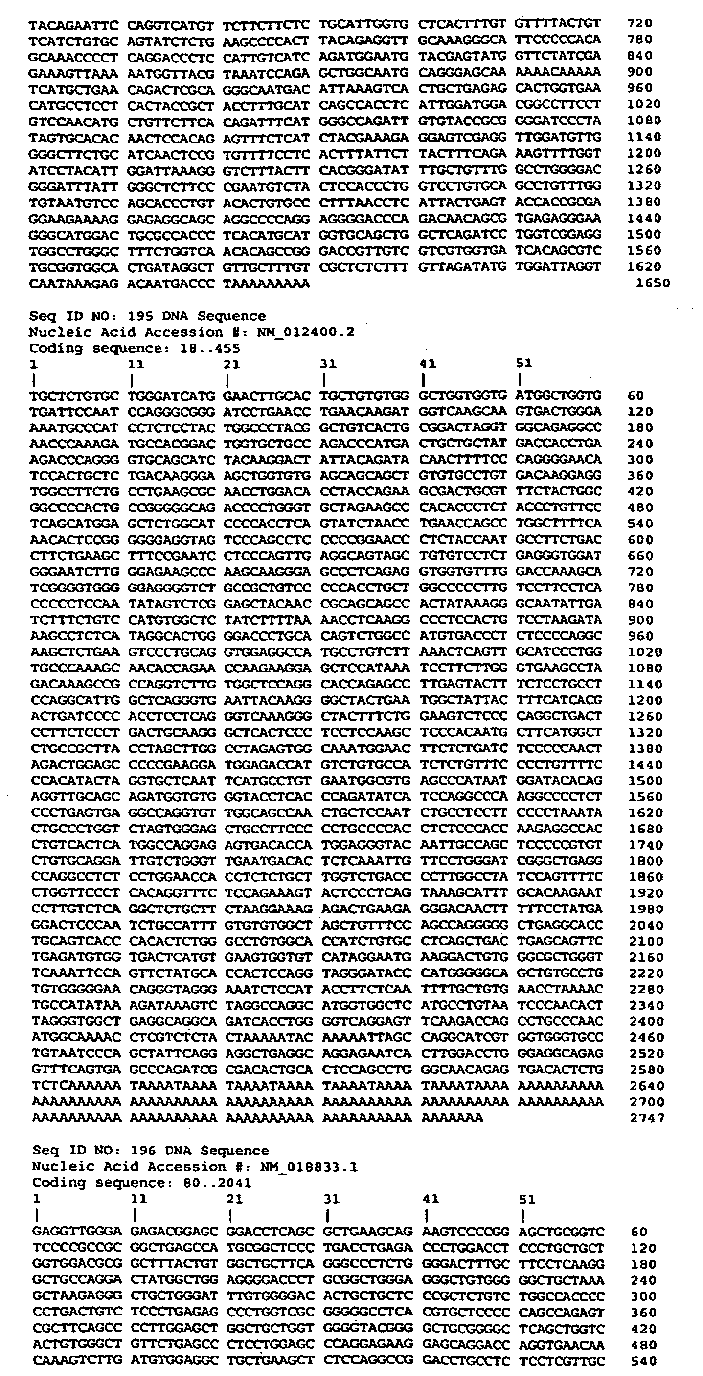

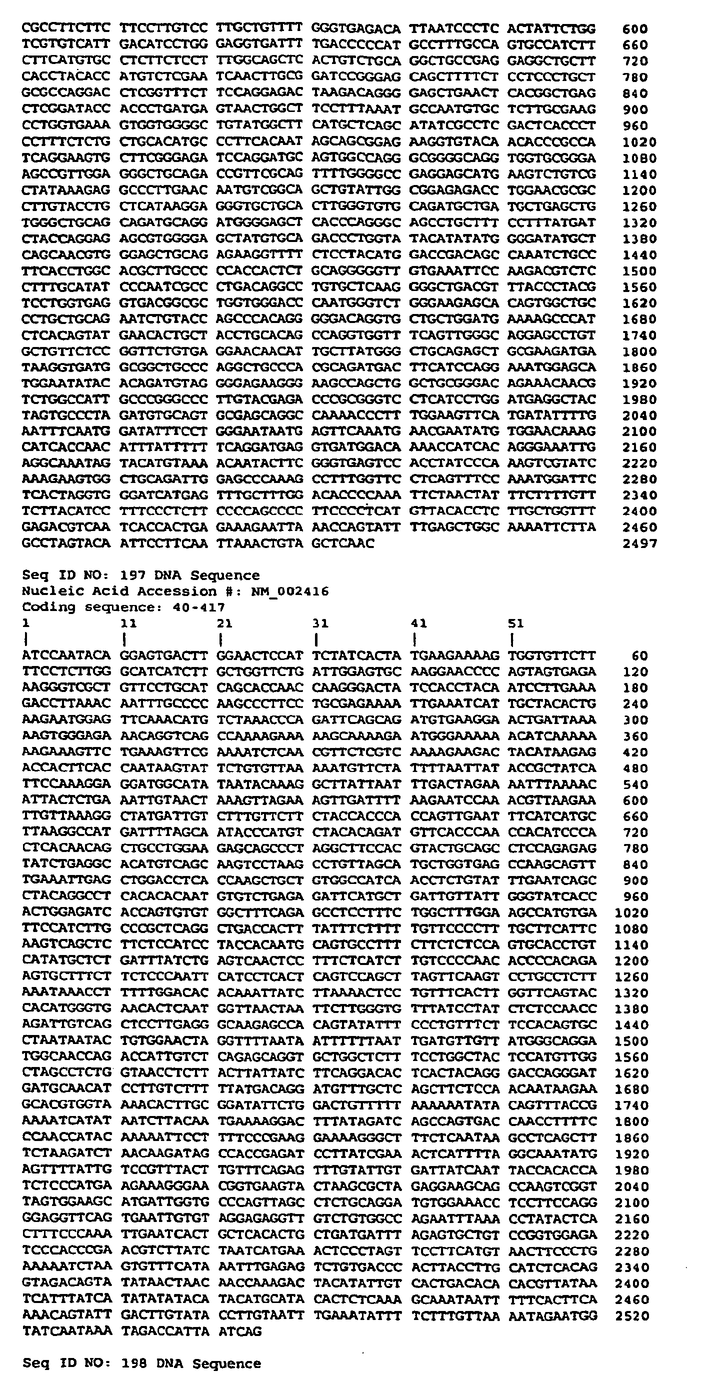

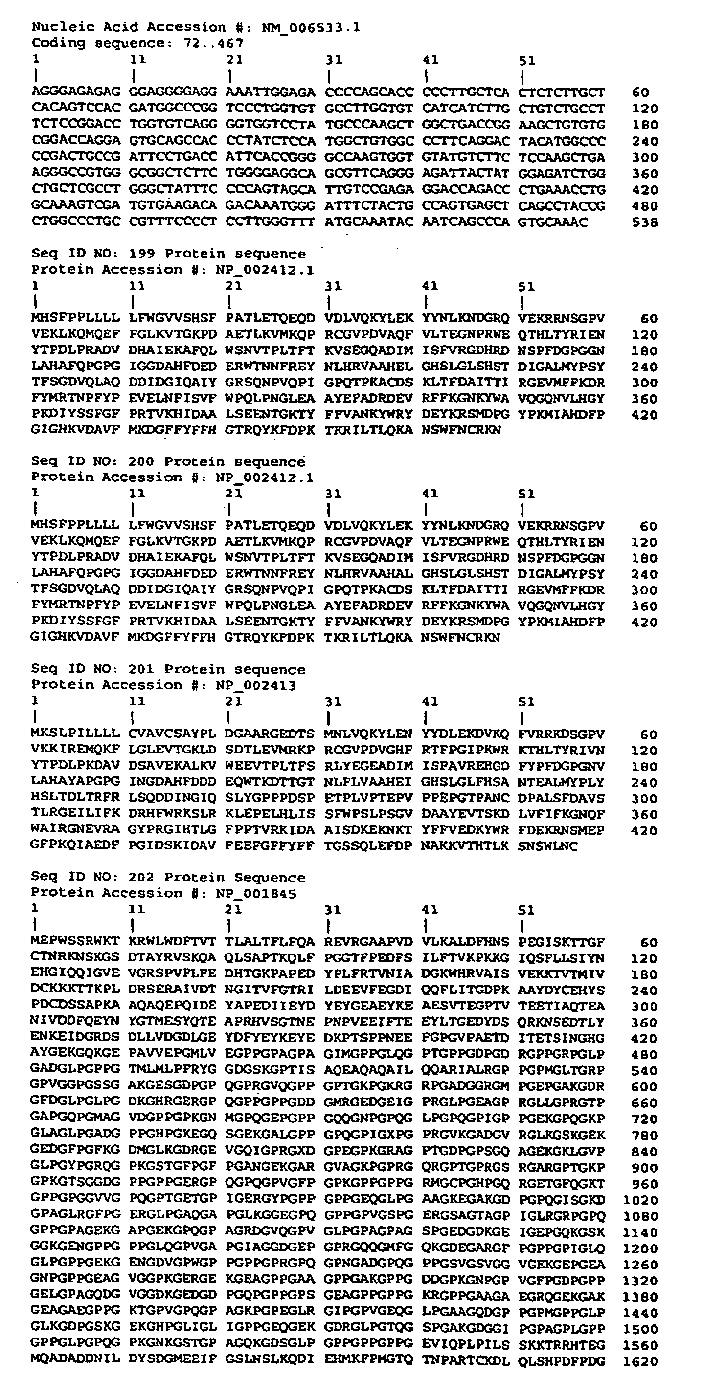

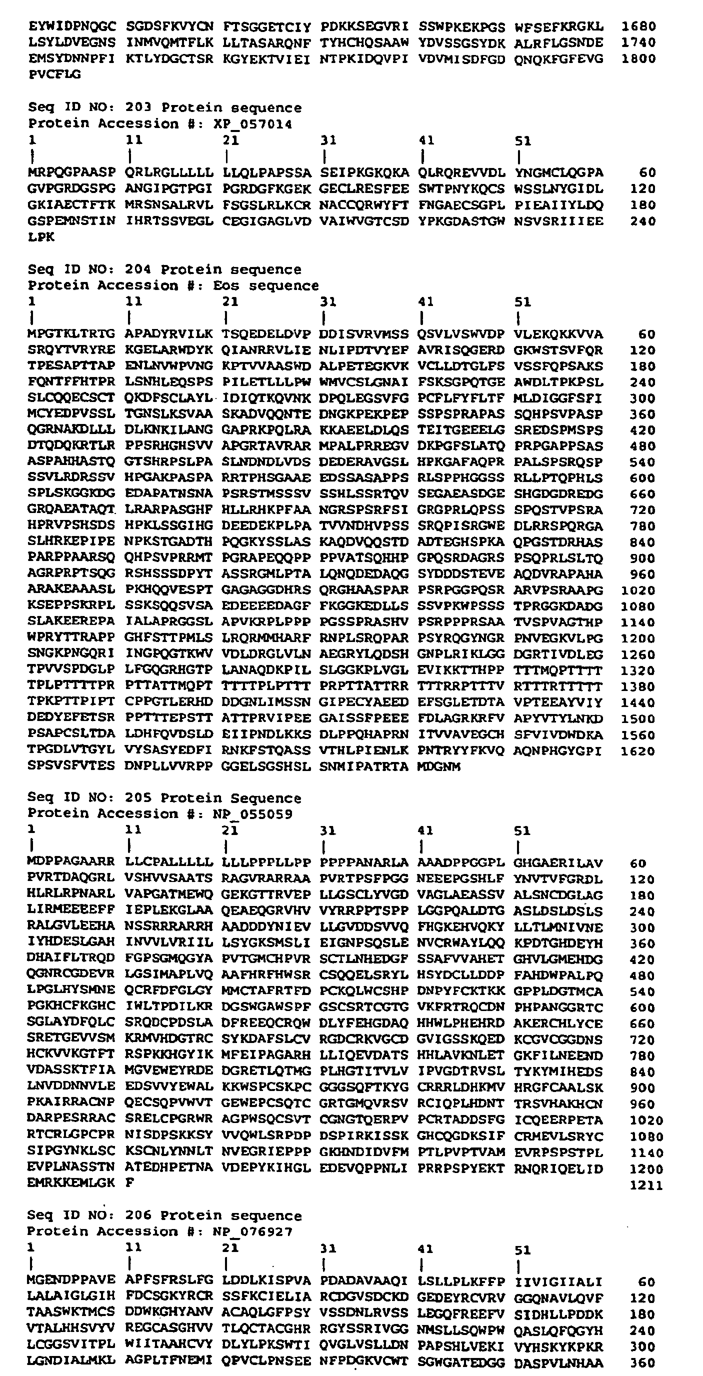

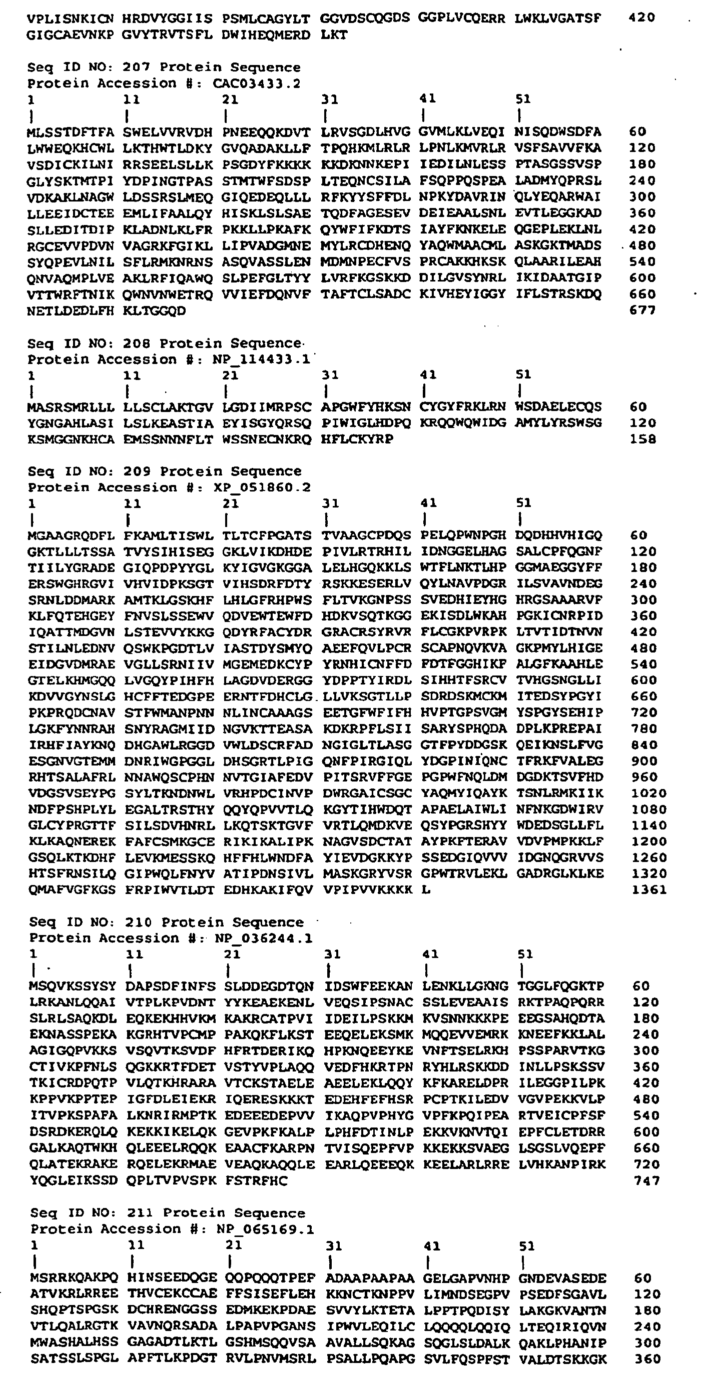

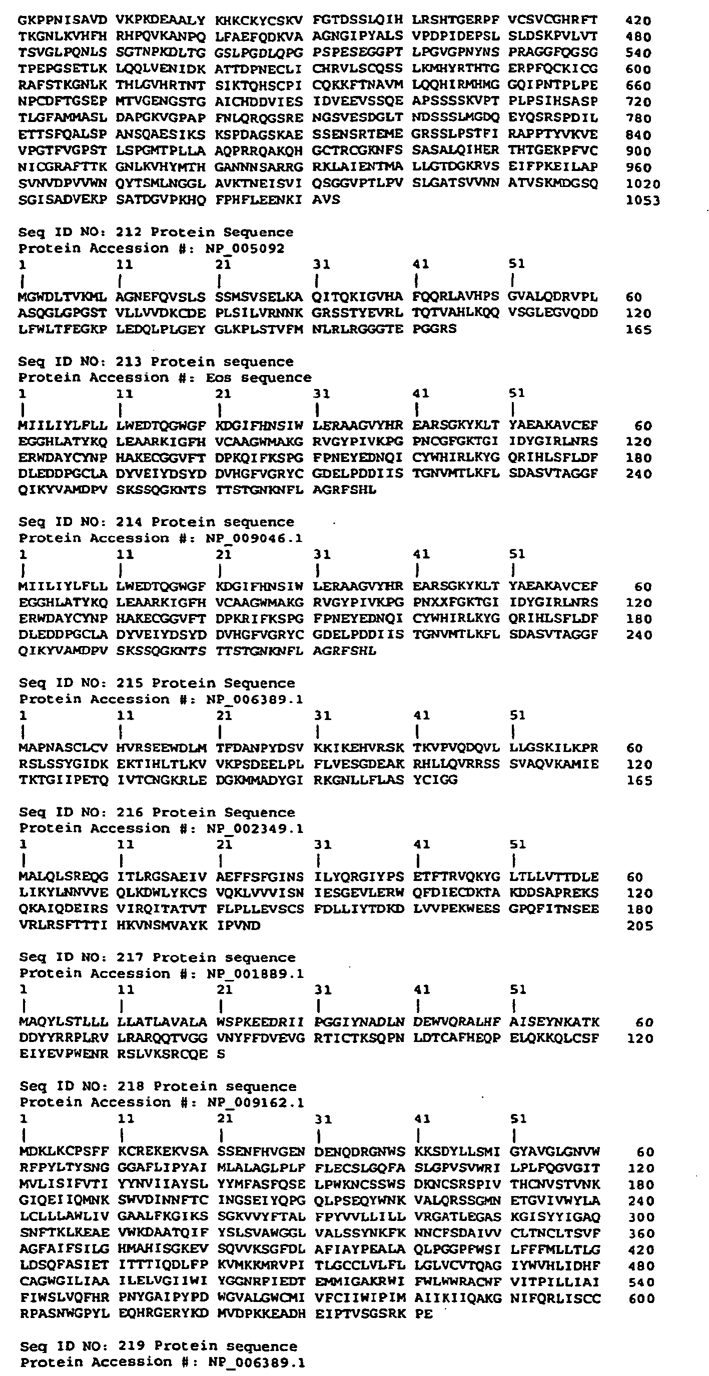

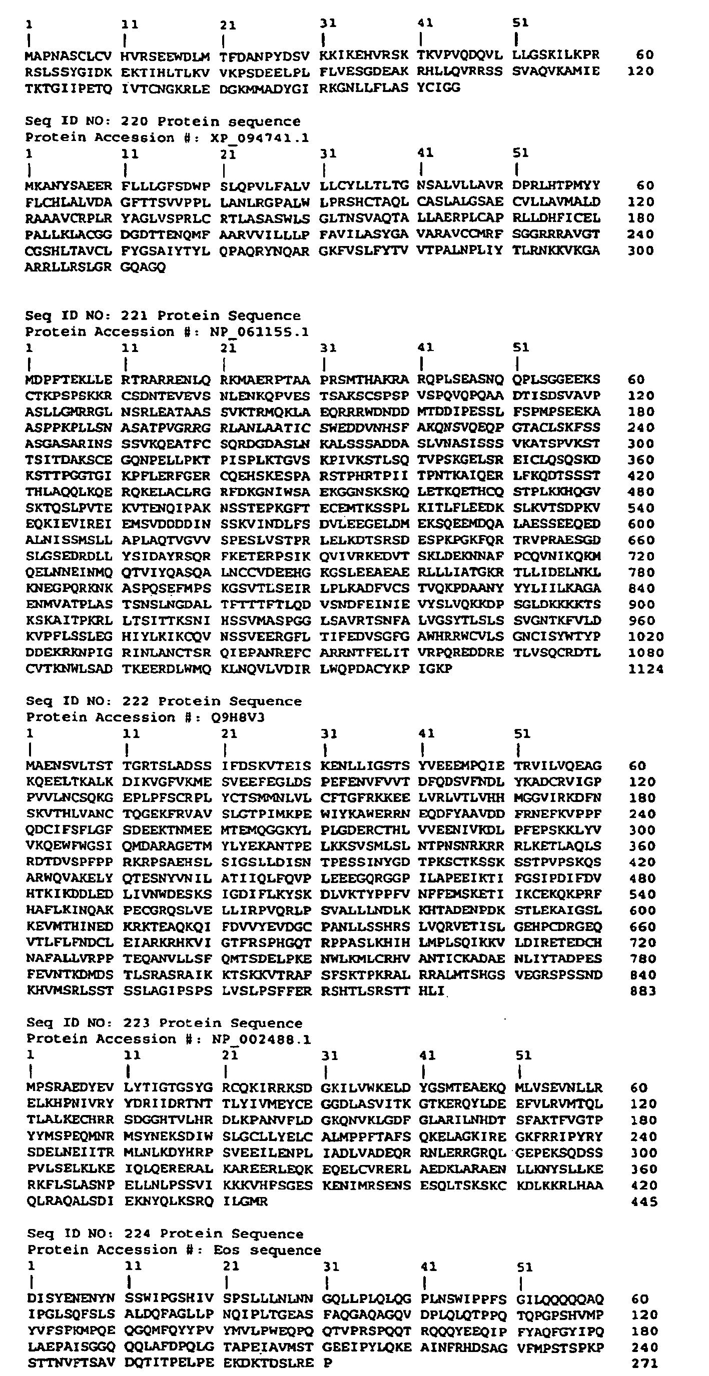

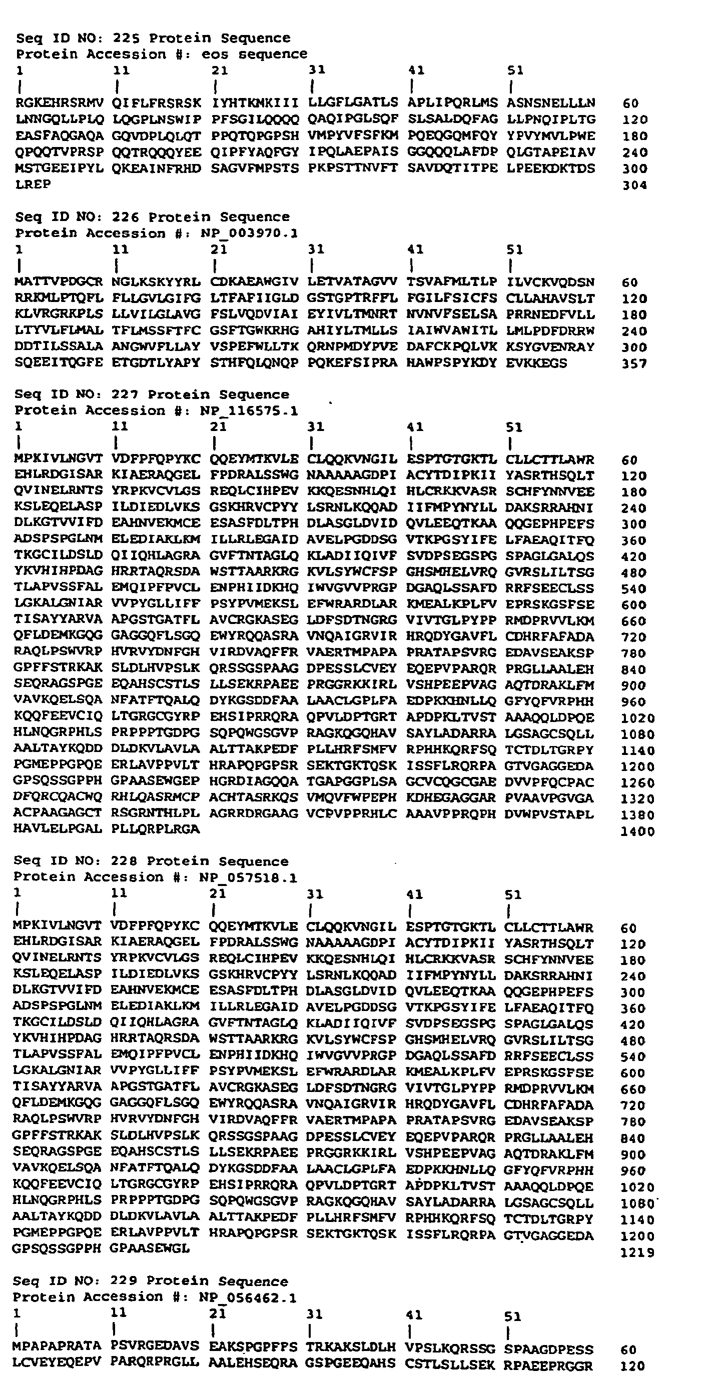

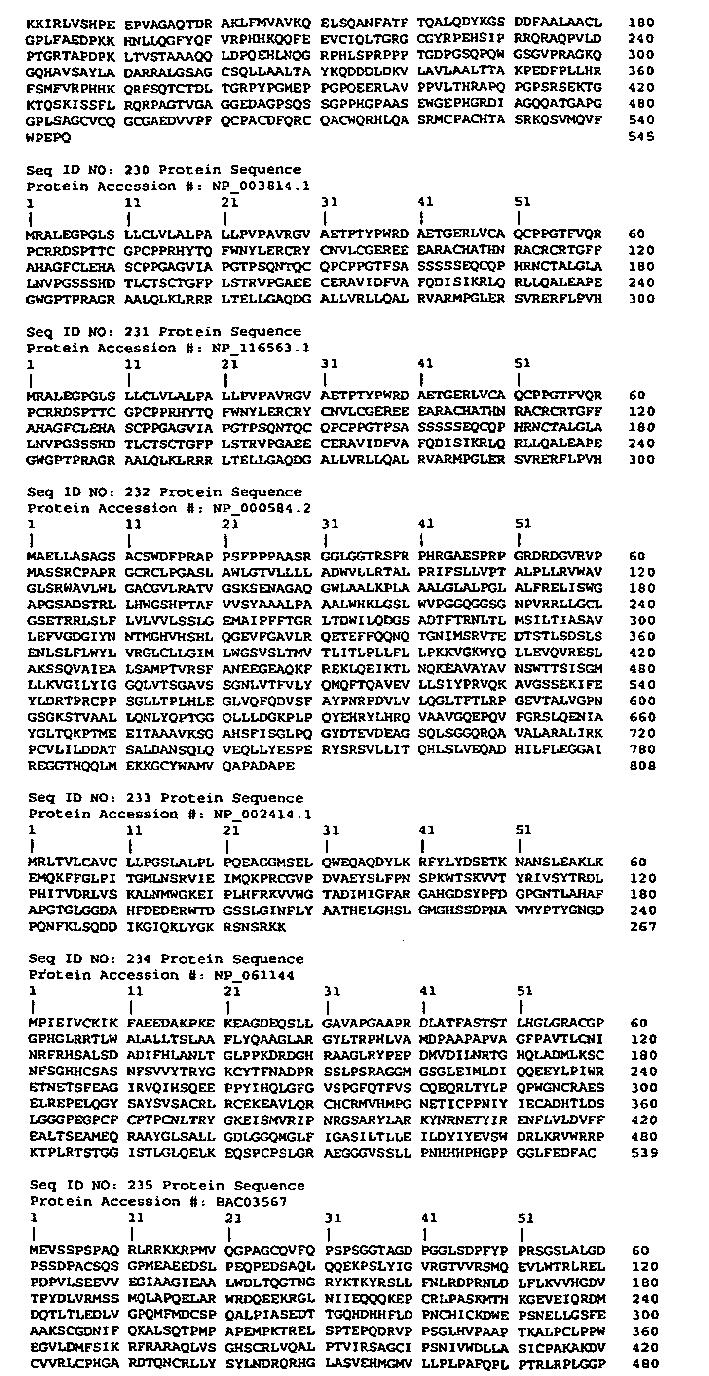

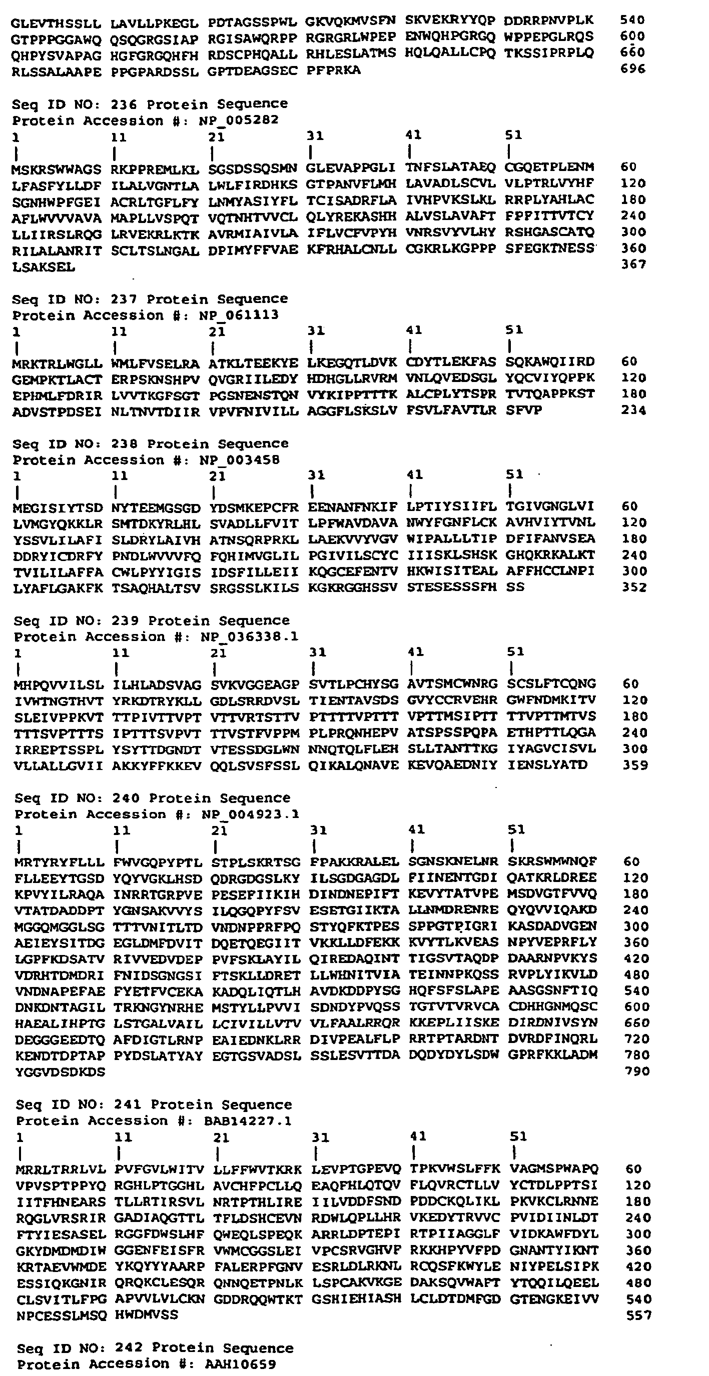

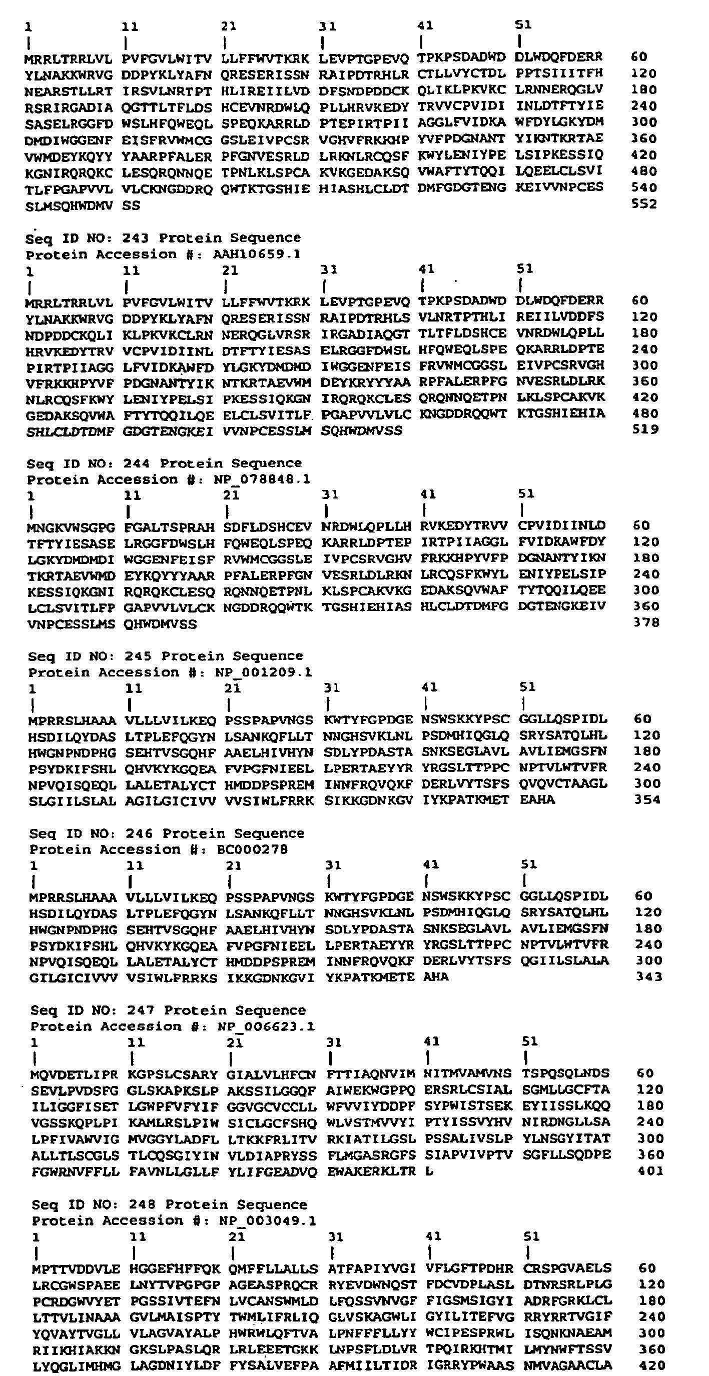

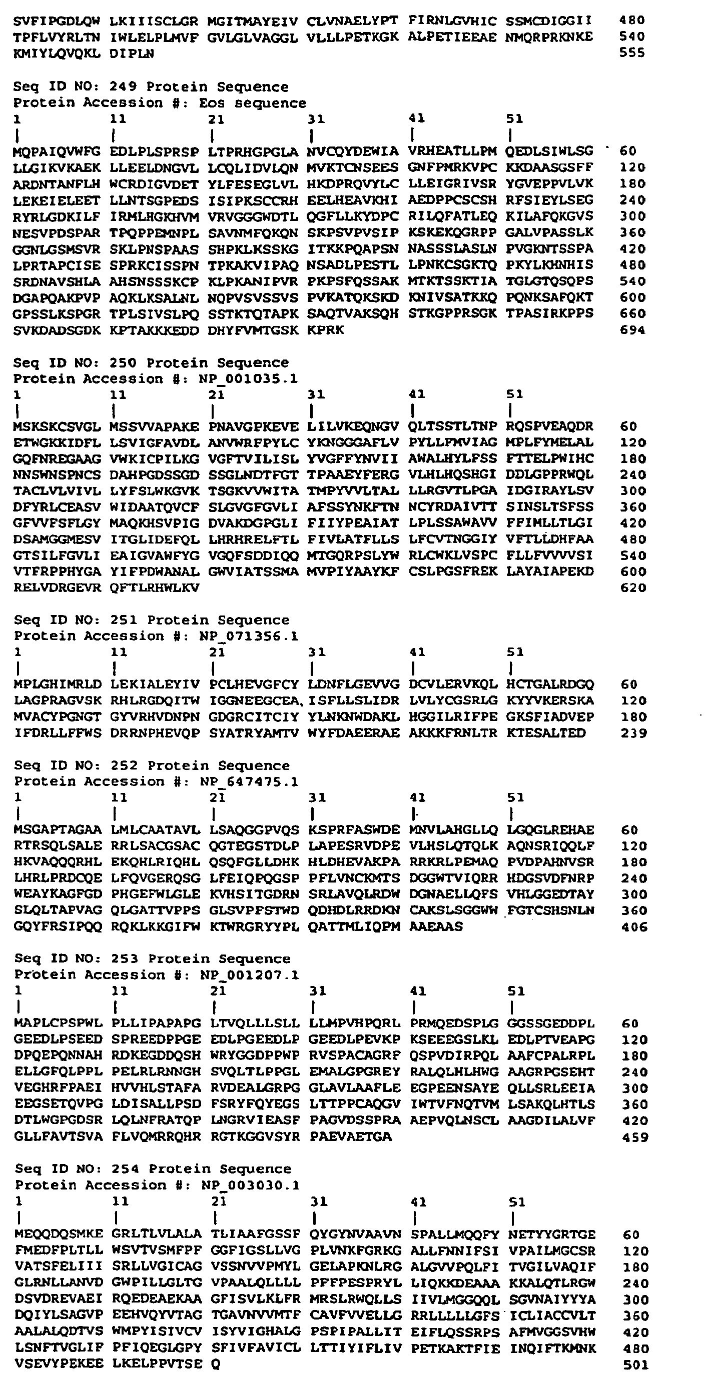

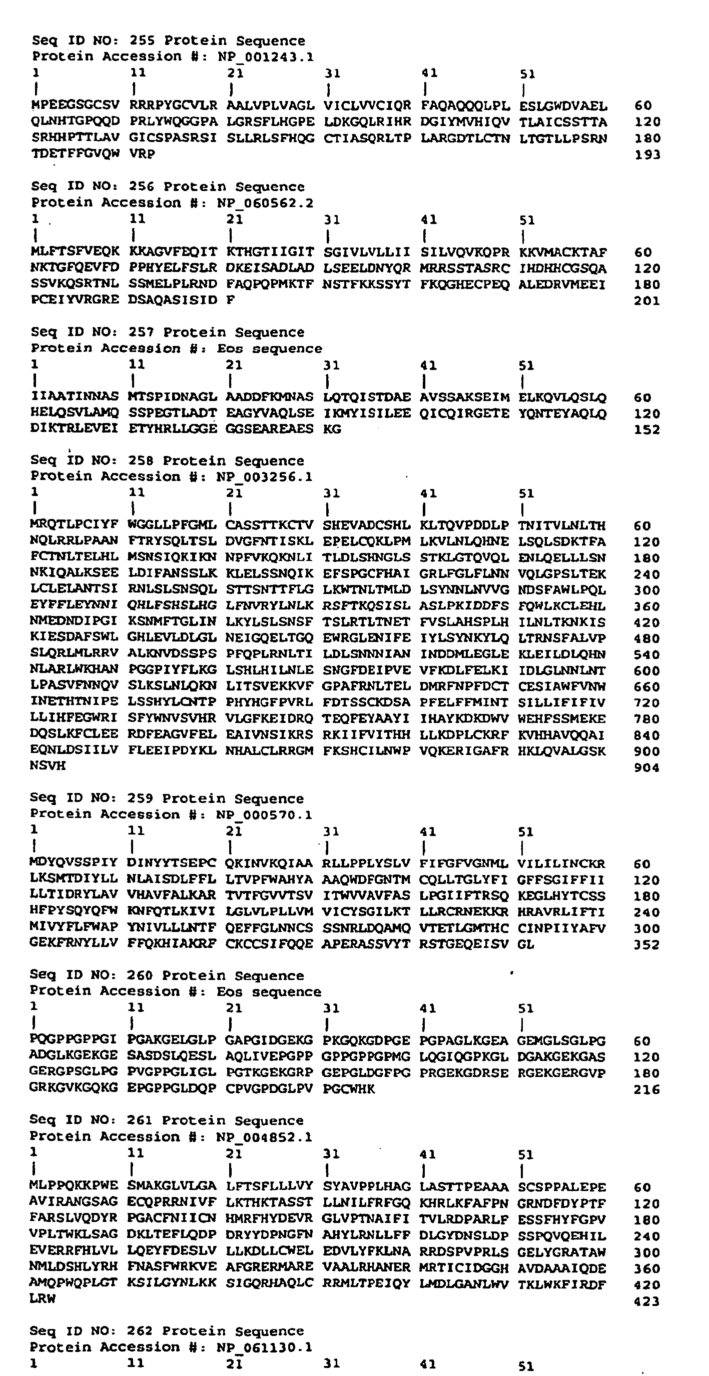

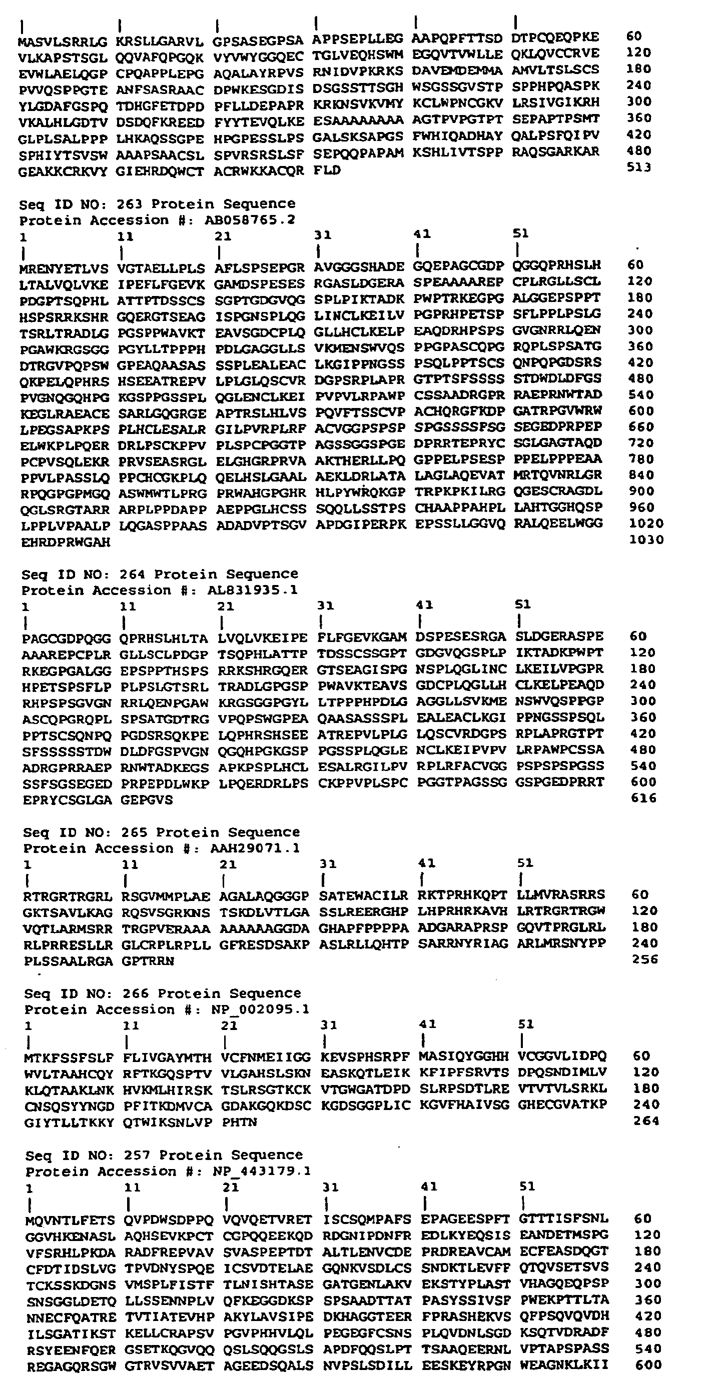

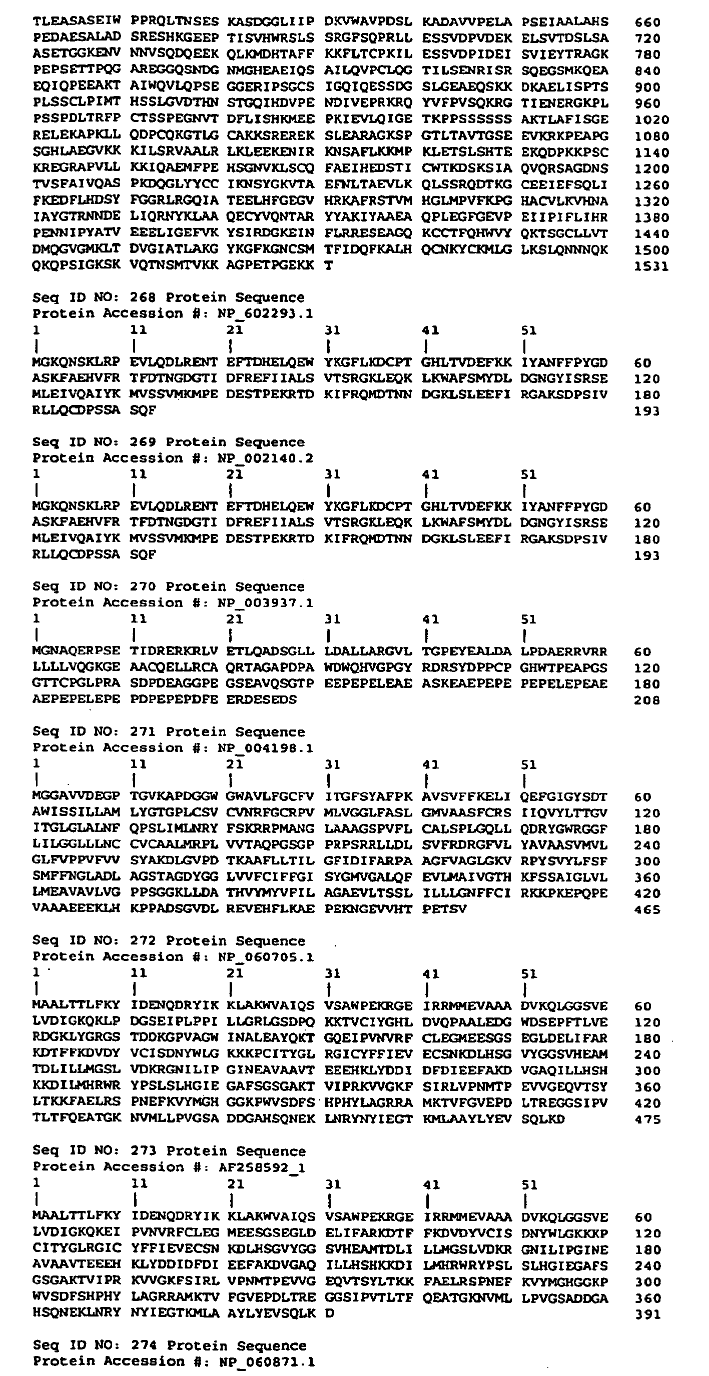

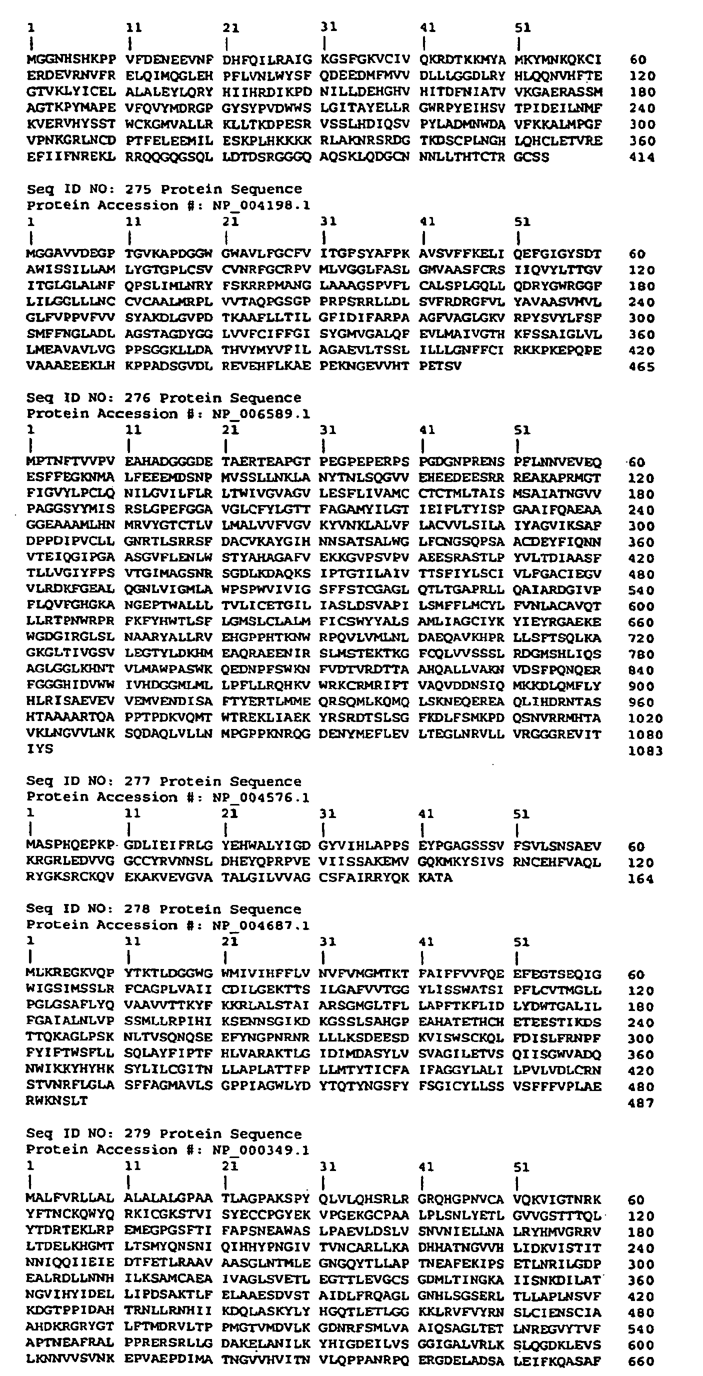

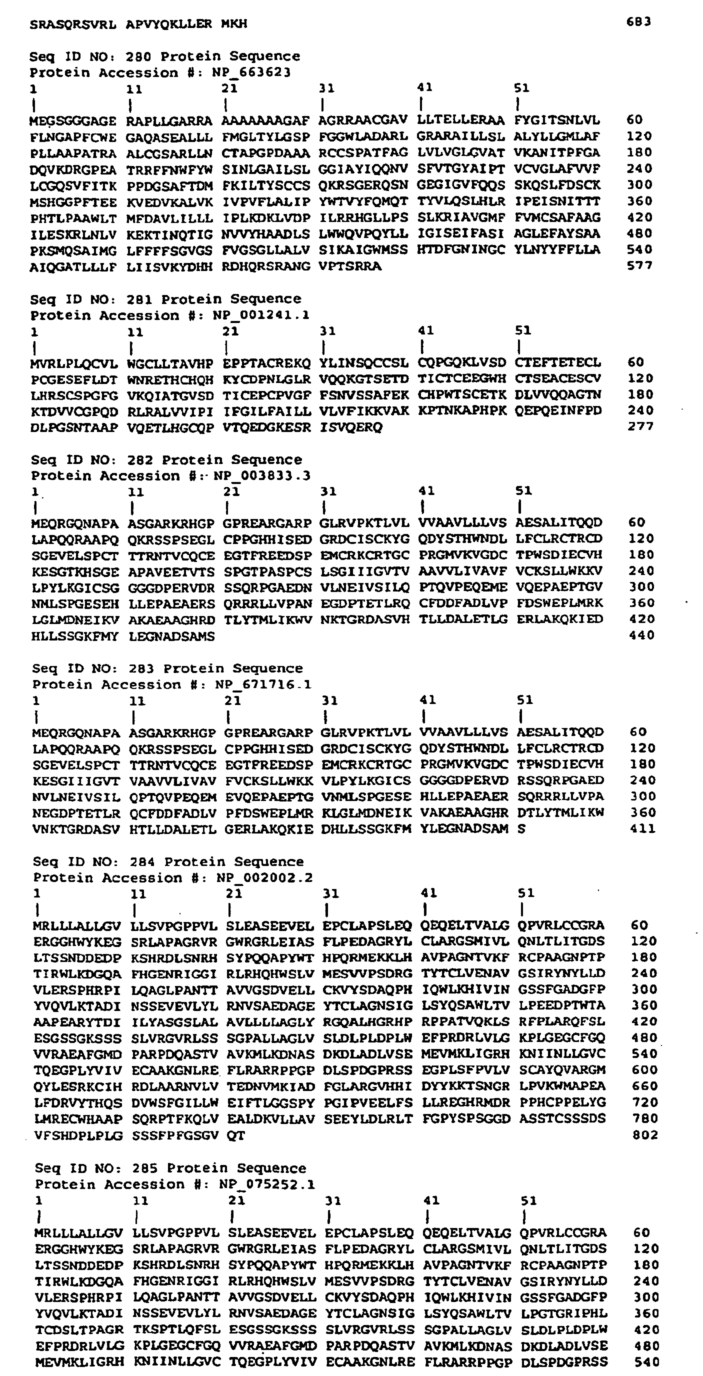

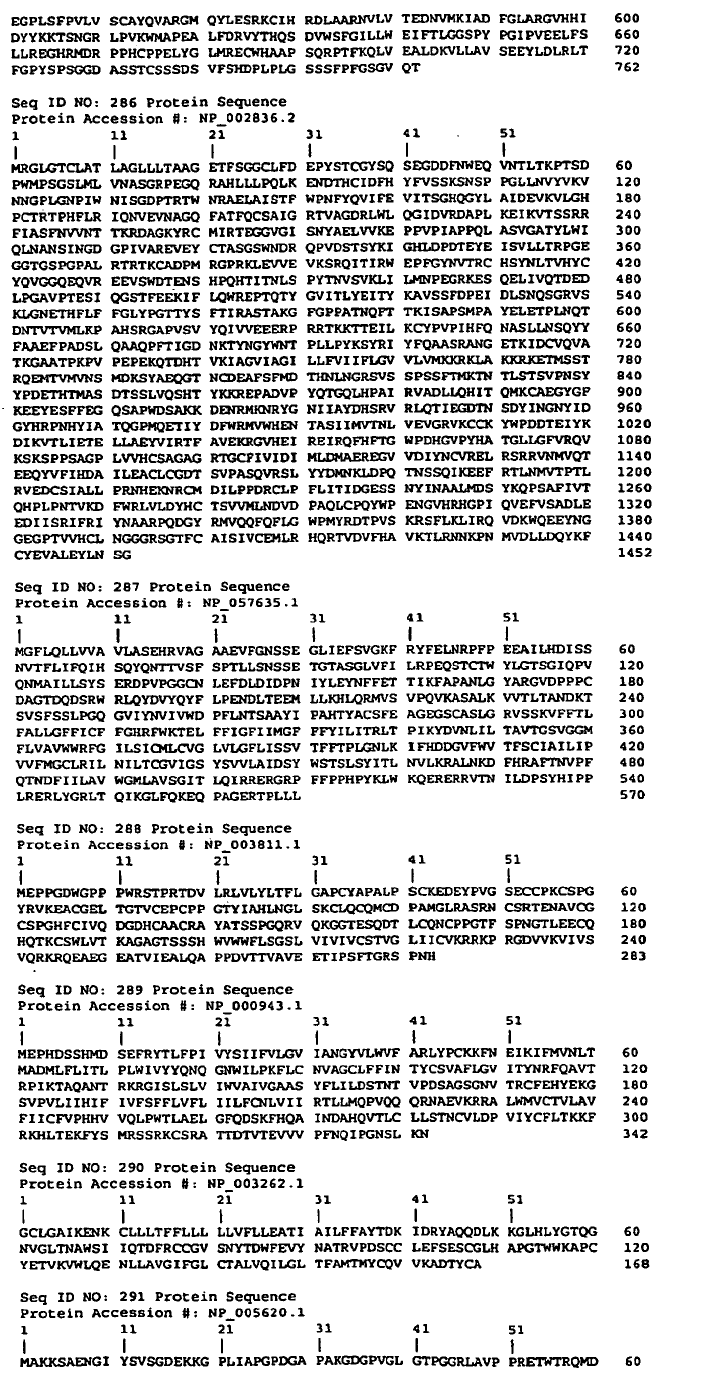

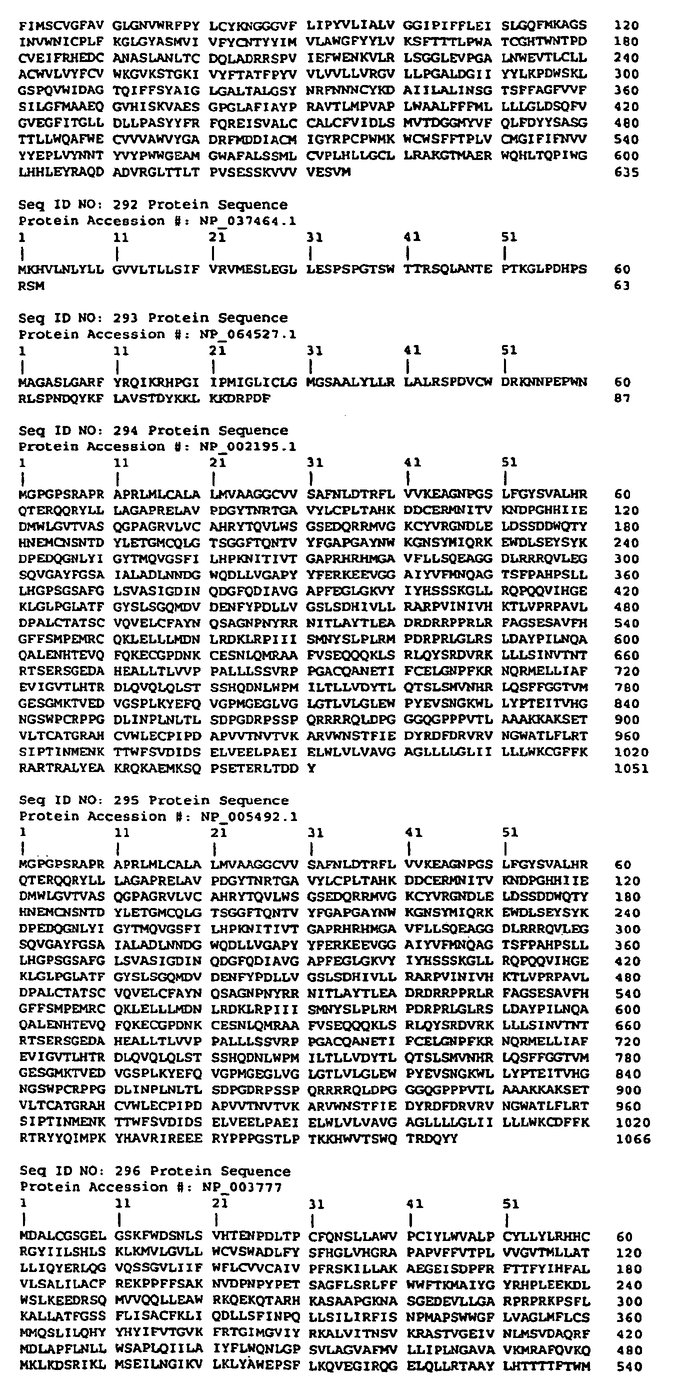

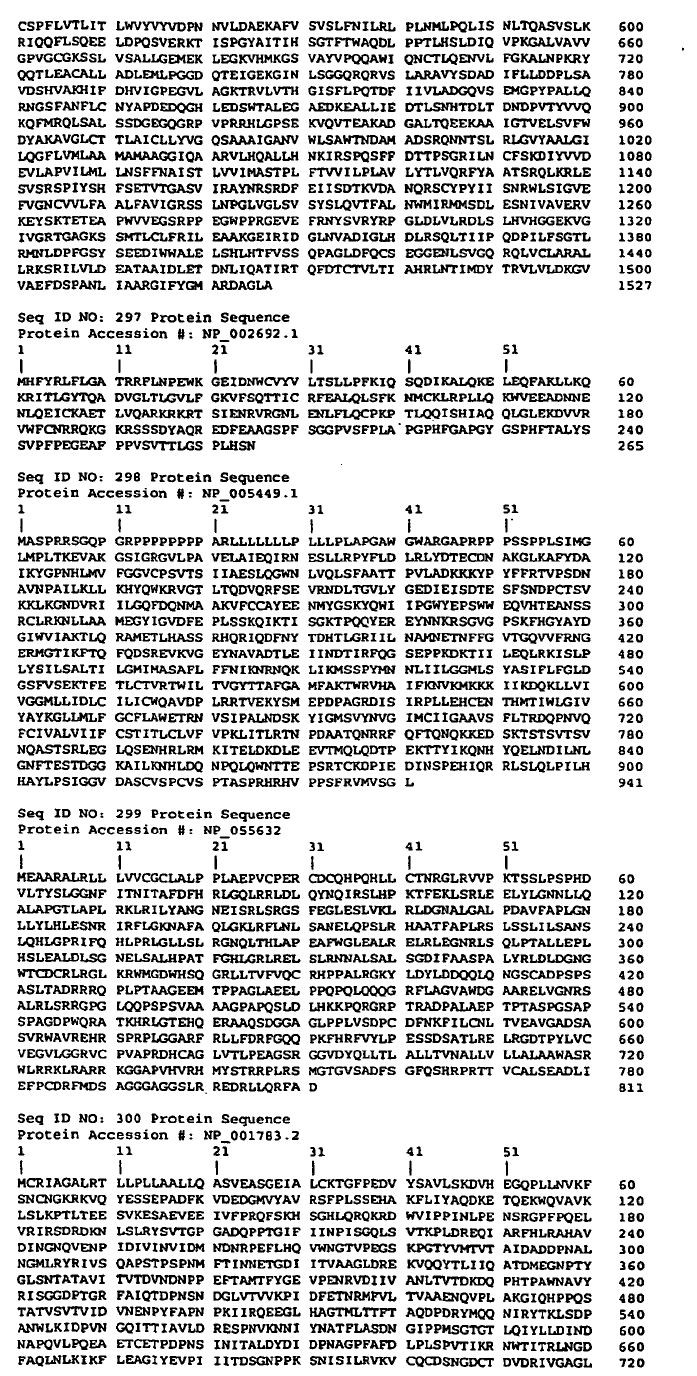

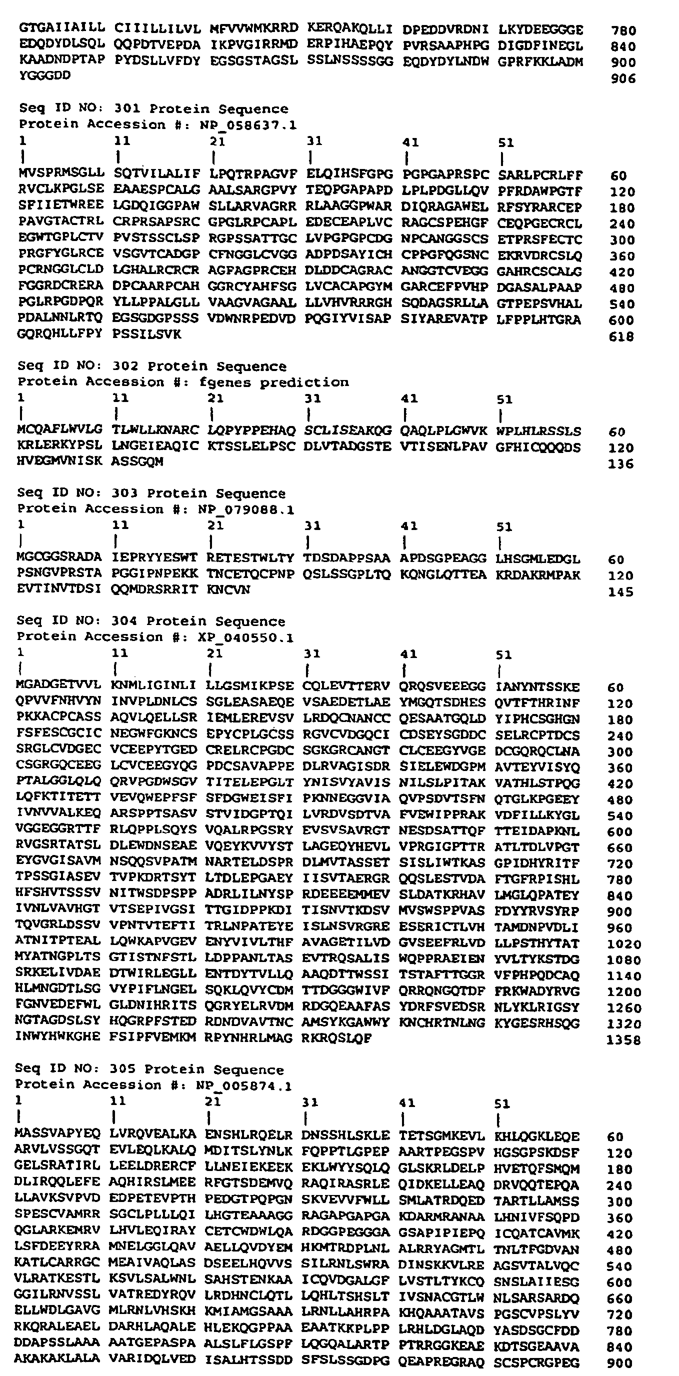

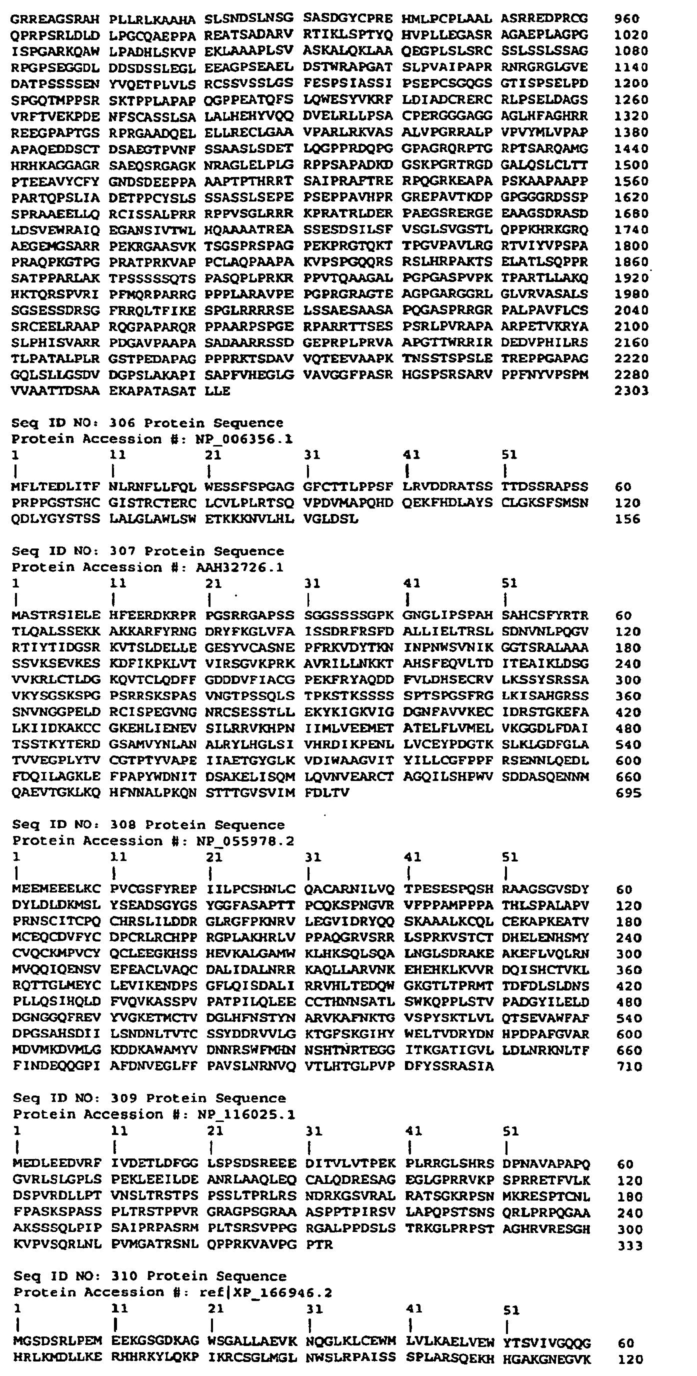

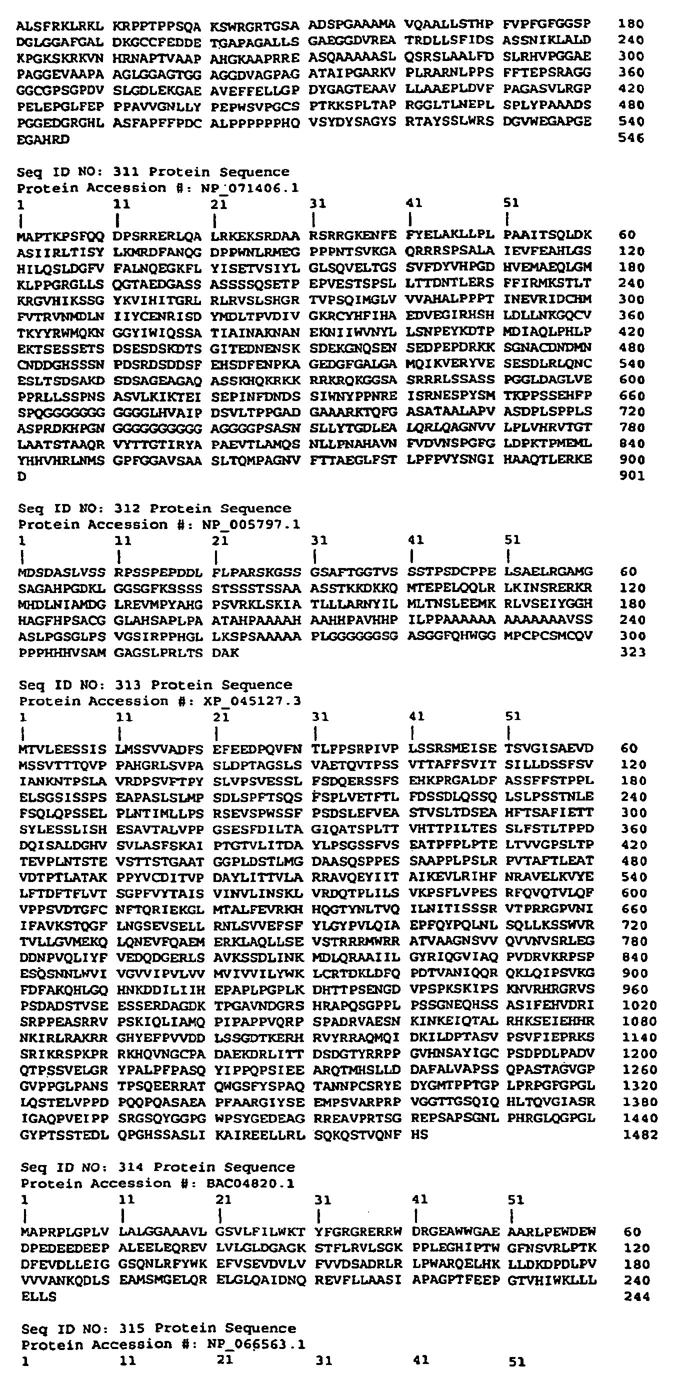

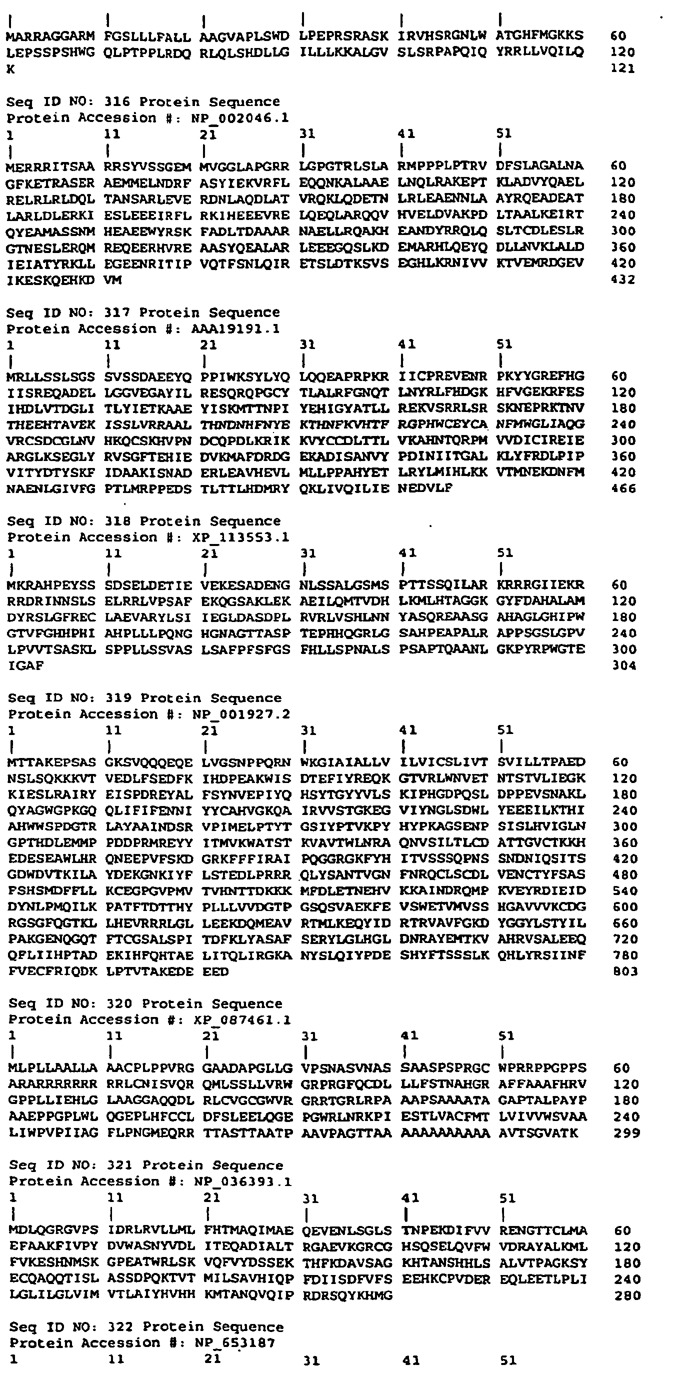

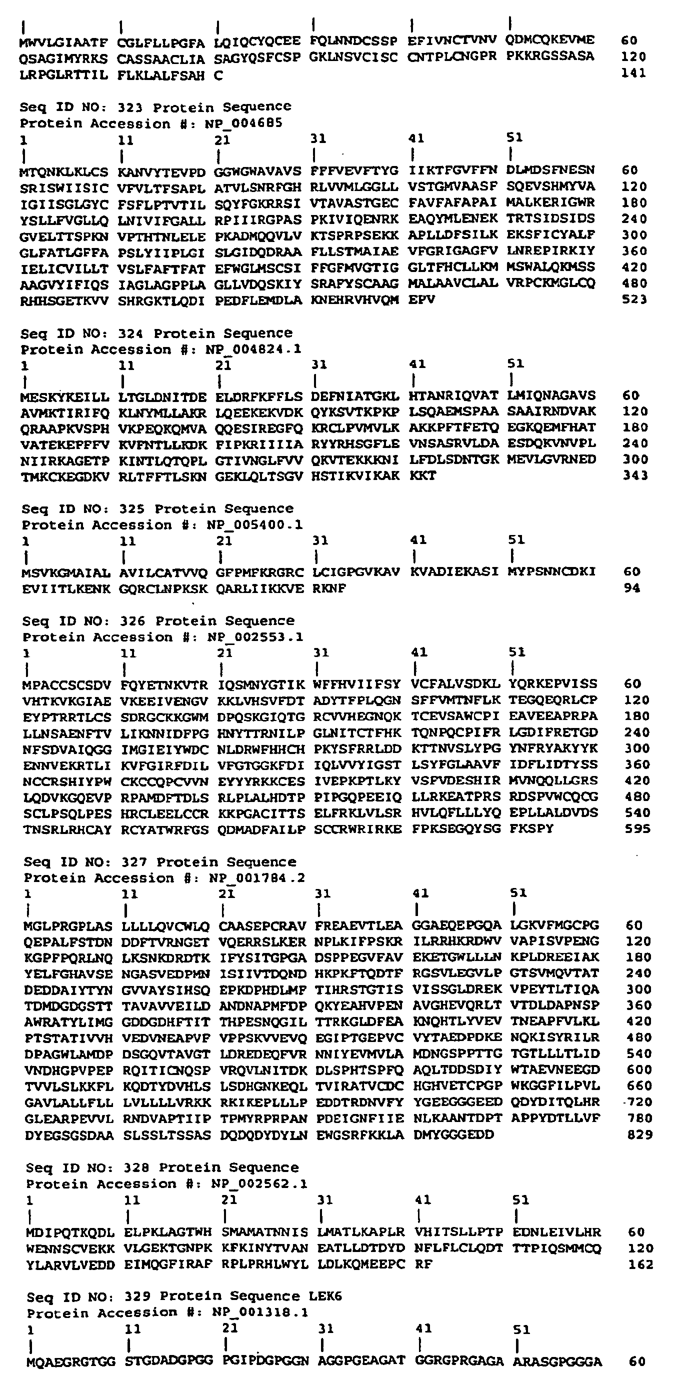

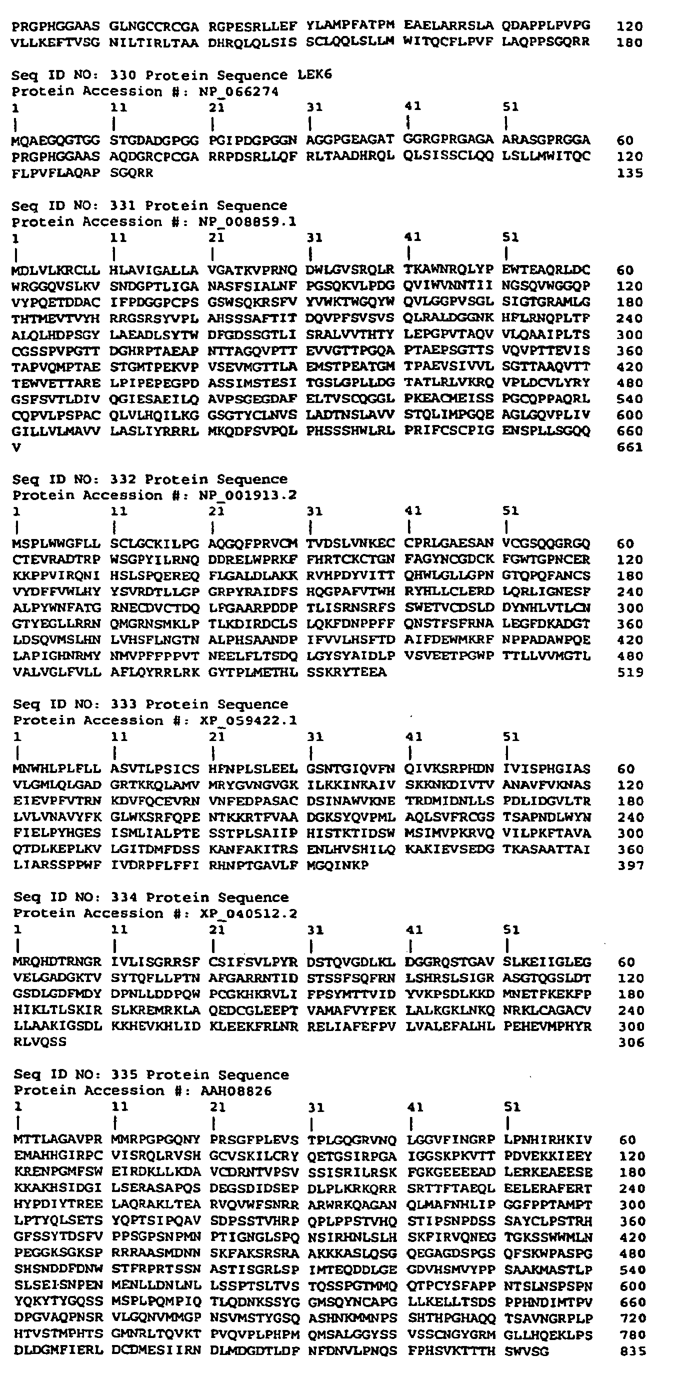

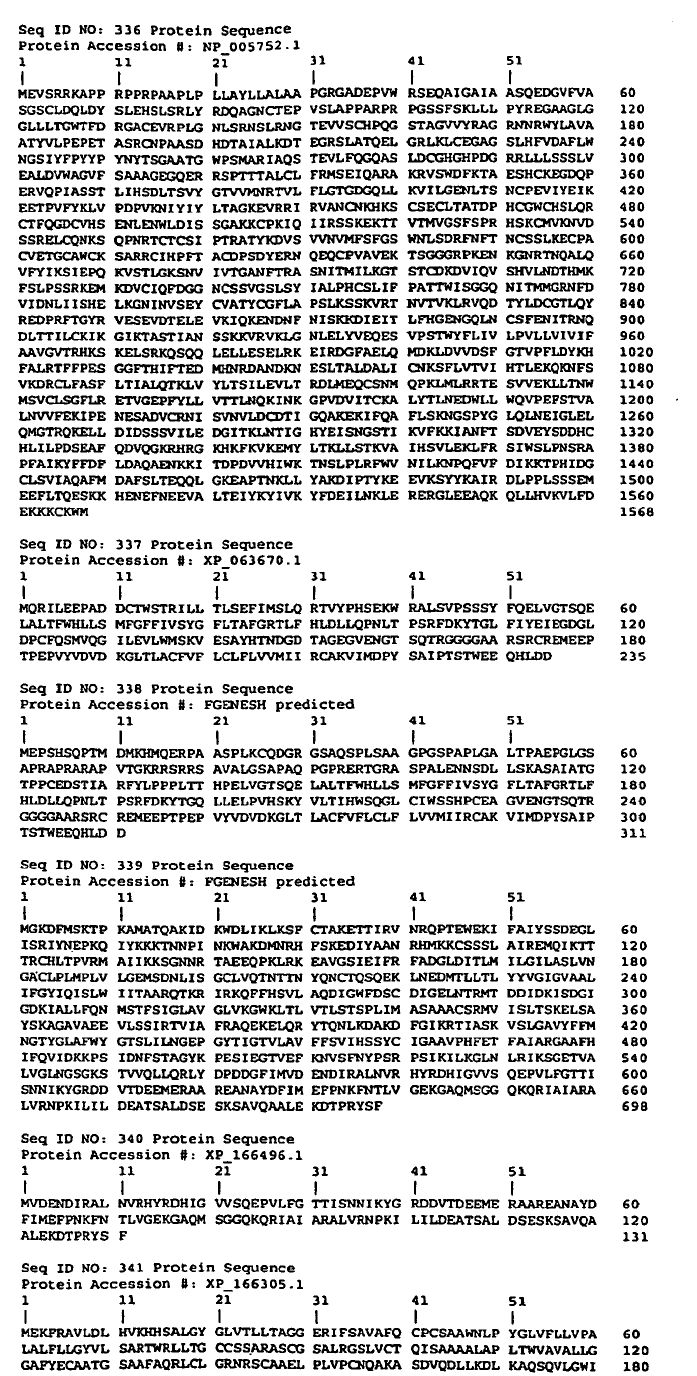

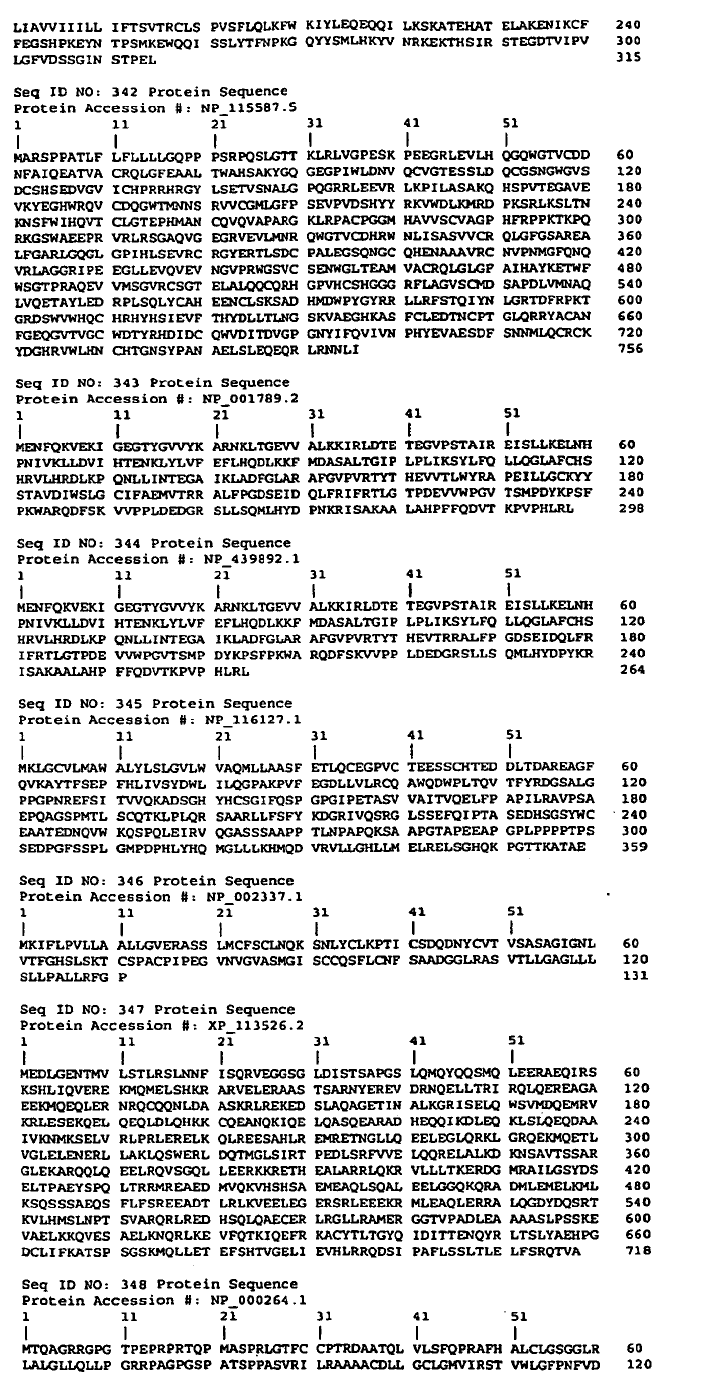

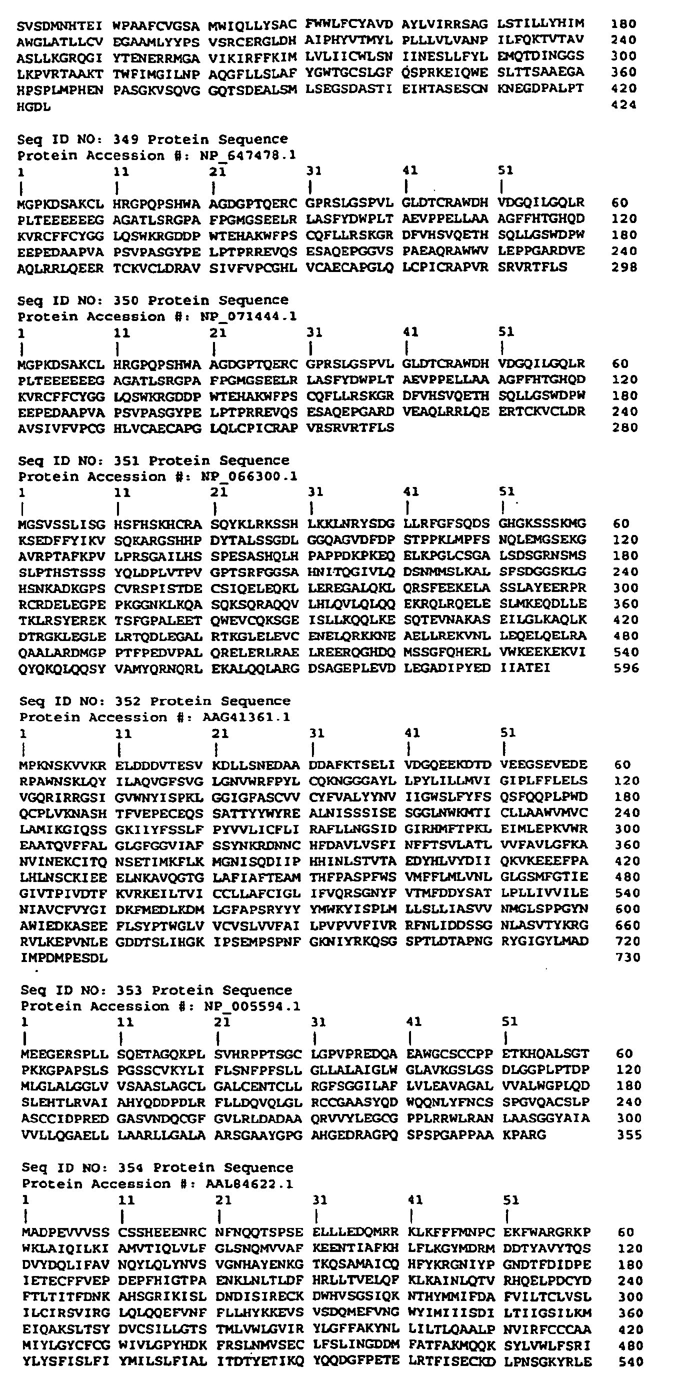

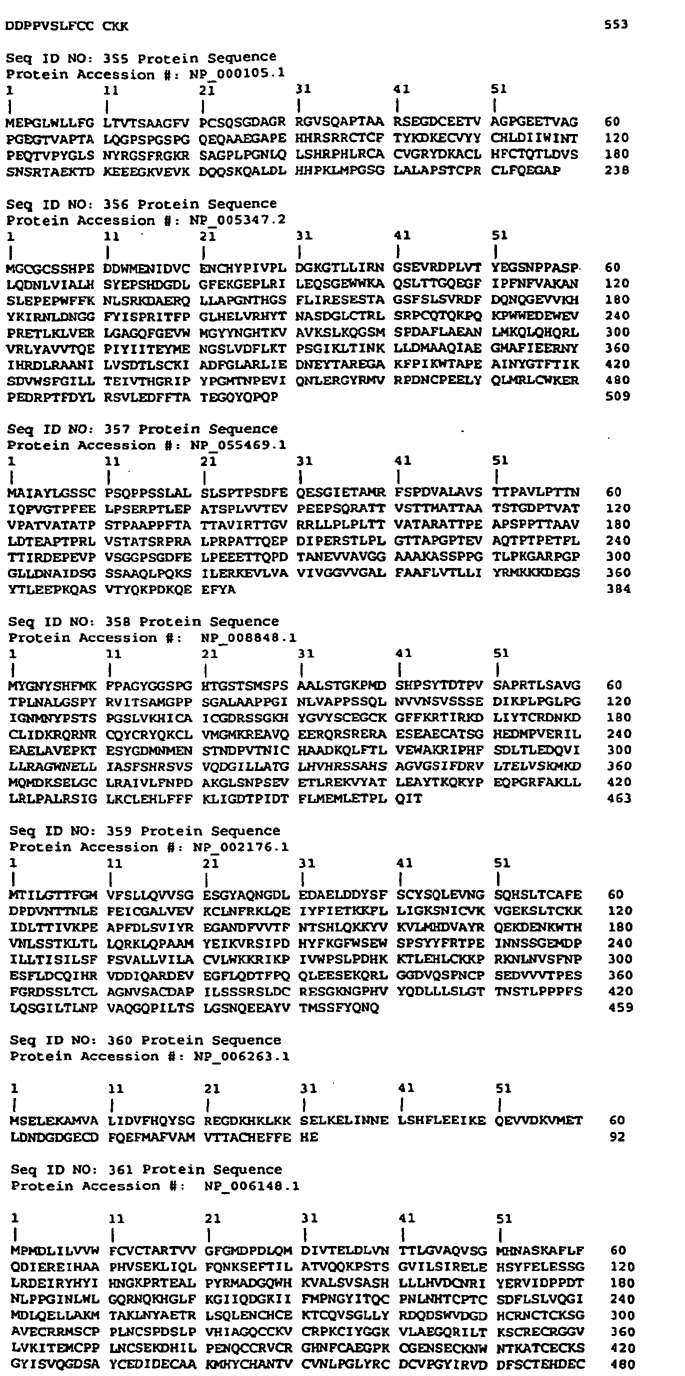

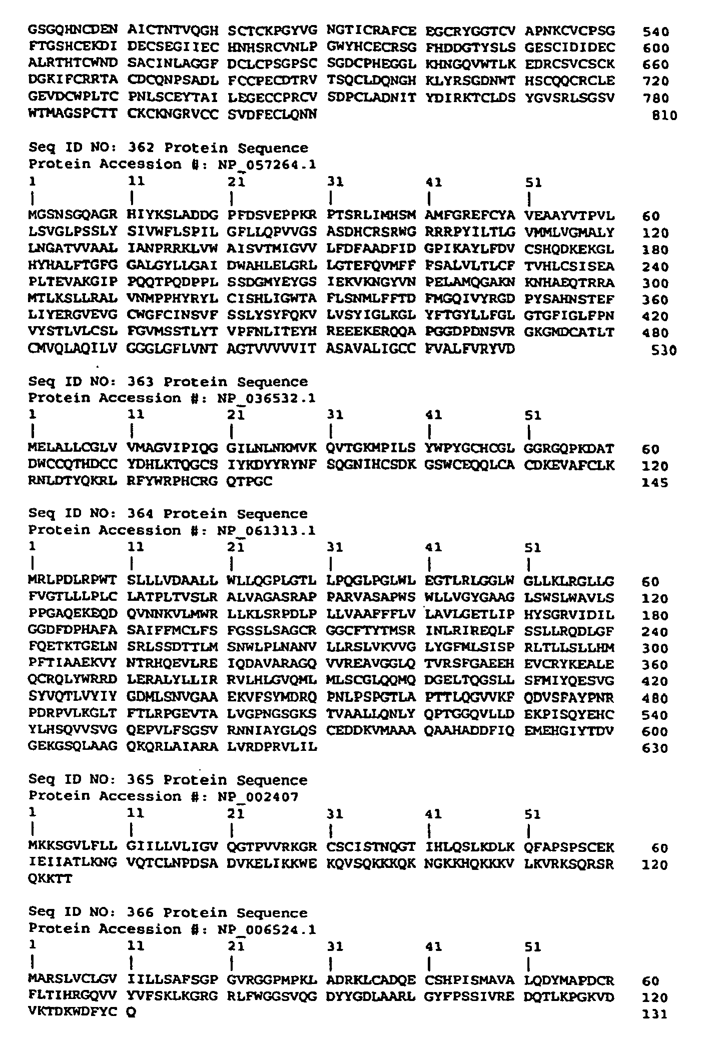

It is understood that the examples described above in no way serve to limit the true scope of this invention, but rather are presented for illustrative purposes. All publications, sequences of accession numbers, and patent applications cited in this specification are herein incorporated by reference as if each individual publication or patent application were specifically and individually indicated to be incorporated by reference.

## Claims

1. An antigenic polypeptide comprising an amino acid sequence having at least 90% sequence identity to SEQ ID NO: 294.

2. The polypeptide of claim 1 wherein the polypeptide comprises the amino acid sequence of SEQ ID NO: 294.

3. An antibody that specifically binds to the polypeptide of any one of claims 1-2.

4. A fusion protein comprising the polypeptide of any one of claims 1-2.

5. An isolated or recombinant nucleic acid encoding the polypeptide of any one of claims 1-2.

6. The nucleic acid of claim 5 wherein the nucleotide sequence comprises SEQ ID NO: 124.

7. A recombinant vector comprising the nucleic acid of any one of claims 5-6.

8. A host cell comprising the vector of claim 7.

9. A method for making a polypeptide comprising culturing the host cell of claim 8 under conditions in which the nucleic acid is expressed.

10. A method for determining the presence or absence of a pathological cell *in vitro,* the method comprising contacting a biological sample with the antibody of claim 3.

11. The method of claim 10 wherein the antibody is conjugated to a detectable label.

12. The method of claim 10 wherein the sample is blood, plasma, serum, sputum, stool, urine, tears, mucus, hair or skin.

13. The use of the antibody of claim 3 in the manufacture of a medicament for the treatment of cancer.

14. The use of claim 12, wherein the cancer is ovarian cancer, pancreatic cancer, bladder cancer, head/neck cancer, melanoma or renal cancer.

15. An isolated polypeptide which is encoded by nucleic acid molecule comprising a sequence as described in Tables 2A-68.

16. An antibody that specifically binds a polypeptide of Claim 15.

17. An isolated nucleic acid molecule comprising a sequence as described in Tables 2A-68.

18. A method for determining the presence or absence of a pathological cell in a patient, said method comprising detecting a nucleic acid comprising a sequence at least 80% identical to a sequence as described in Tables 2A-68 in a biological sample from said patient, thereby determining the presence or absence of said pathological cell.

19. A drug screening assay comprising the steps of:
a) administering a test compound to a mammal having a pathology of Table 1 or cell isolated therefrom; and
b) comparing the level of gene expression of a polynucleotide that selectively hybridizes to a sequence at least 80% identical to a sequence as described in Tables 2A-68 in a treated cell or mammal with the level of gene expression of said polynucleotide in a control cell or mammal, wherein a test compound that modulates said level of expression of the polynucleotide is a candidate for the treatment of said pathology.
